(19) **Europäisches Patentamt / European Patent Office / Office européen des brevets**

(11) **EP 2 058 408 A2**

(12) **EUROPEAN PATENT APPLICATION**

(43) Date of publication:
**13.05.2009 Bulletin 2009/20**

(51) Int Cl.:
***C12Q 1/68*** (2006.01)

(21) Application number: **08075846.9**

(22) Date of filing: **17.02.2004**

(84) Designated Contracting States:
**AT BE BG CH CY CZ DE DK EE ES FI FR GB GR HU IE IT LI LU MC NL PT RO SE SI SK TR**

(30) Priority: **14.02.2003 US 367094**
**14.03.2003 US 388838**
**23.09.2003 US 669920**
**15.12.2003 US 737318**

(62) Document number(s) of the earlier application(s) in accordance with Art. 76 EPC:
**04711946.6 / 1 592 708**

(71) Applicant: **Sagres Discovery, Inc.**
**Davis, CA 95616 (US)**

(72) Inventors:
• **Morris, David W.**
**Davis, CA 95616 (US)**
• **Malandro, Marc S.**
**Davis, CA 95616 (US)**

(74) Representative: **Goodfellow, Hugh Robin et al**
**Carpmaels & Ransford,**
**43-45 Bloomsbury Square**
**London**
**WC1A 2RA (GB)**

Remarks:
This application was filed on 28-10-2008 as a divisional application to the application mentioned under INID code 62.

(54) **Therapeutic GPCR targets in cancer**

(57) The present invention relates to novel sequences for use in detection, diagnosis and treatment of cancers, especially lymphomas. The invention provides cancer-associated (CA) polynucleotide sequences whose expression is associated with cancer. The present invention provides CA polypeptides associated with cancer that are present on the cell surface and present novel therapeutic targets against cancer. This invention relates to G-protein coupled receptor (GPCR) sequences. The present invention further provides diagnostic compositions and methods for the detection of cancer. The present invention provides monoclonal and polyclonal antibodies specific for the CA polypeptides. The present invention also provides diagnostic tools and therapeutic compositions and methods for screening, prevention and treatment of cancer.

**Description**

CROSS-REFERENCE TO RELATED APPLICATIONS

This application claims priority to the following U.S. Applications: U.S. Ser. No. 10/367,094, filed February 14,2003; U.S. Ser. No. 10/388,838, filed March 14, 2003, U.S. Ser. No. 10/669,920 filed September 23, 2003, and U.S. Ser. No. 10/737,318 filed December 15, 2003, all of which are expressly incorporated herein by reference in their entirety.

DESCRIPTION OF ACCOMPANYING CD-ROMs

Tables 1-19 are filed herewith in CD-ROM in accordance with PCT section 801(a). Three identical copies (marked "Copy 1," "Copy 2" and "Copy 3") ofthis CD-ROM are submitted.

Contents of the CD-ROM disks submitted herewith are hereby incorporated by reference into the Specification.

TECHNICAL FIELD OF THE INVENTION

This invention relates generally to the field of cancer-associated genes. Specifically, it relates to novel polynucleotide and protein sequences for use in diagnosis and treatment of cancer and tumors, as well as the use of the novel compositions in screening methods. More specifically, this invention relates to G-protein coupled receptor (GPCR) sequences. The present invention provides methods of using cancer associated polynucleotides, their corresponding gene products and antibodies specific for the gene products in the detection, diagnosis, prevention and/or treatment of associated cancers.

BACKGROUND OF THE INVENTION

Oncogenes are genes that can cause cancer. Carcinogenesis can occur by a wide variety of mechanisms, including infection of cells by viruses containing oncogenes, activation of protooncogenes in the host genome, and mutations of protooncogenes and tumor suppressor genes. Carcinogenesis is fundamentally driven by somatic cell evolution (i.e. mutation and natural selection of variants with progressive loss of growth control). The genes that serve as targets for these somatic mutations are classified as either protooncogenes or tumor suppressor genes, depending on whether their mutant phenotypes are dominant or recessive, respectively.

There are a number of viruses known to be involved in human cancer as well as in animal cancer. Of particular interest here are viruses that do not contain oncogenes themselves; these are slow-transforming retroviruses. They induce tumors by integrating into the host genome and affecting neighboring protooncogenes in a variety of ways. Provirus insertion mutation is a normal consequence of the retroviral life cycle. In infected cells, a DNA copy of the retrovirus genome (called a provirus) is integrated into the host genome. A newly integrated provirus can affect gene expression in *cis* at or near the integration site by one of two mechanisms. Type I insertion mutations up-regulate transcription of proximal genes as a consequence of regulatory sequences (enhancers and/or promoters) within the proviral long terminal repeats (LTRs). Type II insertion mutations cause truncation of coding regions due to either integration directly within an open reading frame or integration within an intron flanked on both sides by coding sequences. The analysis of sequences at or near the insertion sites has led to the identification of a number of new protooncogenes.

With respect to lymphoma and leukemia, retroviruses such as AKV murine leukemia virus (MLV) or SL3-3 MLV, are potent inducers of tumors when inoculated into susceptible newborn mice, or when carried in the germline. A number of sequences have been identified as relevant in the induction of lymphoma and leukemia by analyzing the insertion sites; see Sorensen et al., J. of Virology 74:2161 (2000); Hansen et al., Genome Res. 10(2):237-43 (2000); Sorensen et al., J. Virology 70:4063 (1996); Sorensen et al., J. Virology 67:7118 (1993); Joosten et al., Virology 268: 308 (2000); and Li et al., Nature Genetics 23:348 (1999); all of which are expressly incorporated by reference herein. With respect to cancers, especially breast cancer, prostate cancer and cancers with epithelial origin, the mammalian retrovirus, mouse mammary tumor virus (MMTV) is a potent inducer of tumors when inoculated into susceptible newborn mice, or when carried in the germ line. Mammary Tumors in the Mouse, edited by J. Hilgers and M. Sluyser; Elsevier/ North-Holland Biomedical Press; New York, N.Y.

The pattern of gene expression in a particular living cell is characteristic of its current state. Nearly all differences in the state or type of a cell are reflected in the differences in RNA levels of one or more genes. Comparing expression patterns of uncharacterized genes may provide clues to their function. High throughput analysis of expression of hundreds or thousands of genes can help in (a) identification of complex genetic diseases, (b) analysis of differential gene expression over time, between tissues and disease states, and (c) drug discovery and toxicology studies. Increase or decrease in the levels of expression of certain genes correlate with cancer biology. For example, oncogenes are positive regulators of tumorigenesis, while tumor suppressor genes are negative regulators of tumorigenesis. (Marshall, Cell, 64: 313-326 (1991); Weinberg, Science, 254: 1138-1146 (1991)).

Accordingly, it is an object of the invention to provide polynucleotide and polypeptide sequences involved in cancer and, in particular, in oncogenesis.

Immunotherapy, or the use of antibodies for therapeutic purposes has been used in recent years to treat cancer. Passive immunotherapy involves the use of monoclonal antibodies in cancer treatments. See for example, Cancer: Principles and Practice of Oncology, 6th Edition (2001) Chapt. 20 pp. 495-508. Inherent therapeutic biological activity of these antibodies include direct inhibition of tumor cell growth or survival, and the ability to recruit the natural cell killing activity of the body's immune system. These agents are administered alone or in conjunction with radiation or chemotherapeutic agents. Rituxan® and Herceptin®, approved for treatment of lymphoma and breast cancer, respectively, are two examples of such therapeutics. Alternatively, antibodies are used to make antibody conjugates where the antibody is linked to a toxic agent and directs that agent to the tumor by specifically binding to the tumor. Mylotarg® is an example of an approved antibody conjugate used for the treatment of leukemia.

Accordingly, it is another object of this invention to provide antigens (cancer-associated polypeptides) associated with a variety of cancers as targets for diagnostic and/or therapeutic antibodies. These antigens are also useful for drug discovery (e.g., small molecules) and for further characterization of cellular regulation, growth, and differentiation.

SUMMARY OF THE INVENTION

In accordance with the objects outlined above, the present invention provides methods for screening for compositions that modulate cancer, especially lymphoma and leukemia. The present invention also provides methods for screening for compositions which modulate carcinomas, especially mammary adenocarcinomas. Also provided herein are methods of inhibiting proliferation of a cell, preferably a lymphoma cell or a breast cancer cell. Methods of treatment of cancer, including diagnosis, are also provided herein.

In one aspect, a method of screening drug candidates comprises providing a cell that expresses a cancer-associated (CA) gene or fragments thereof. Preferred embodiments of CA genes are genes that are differentially expressed in cancer cells, preferably lymphatic, breast, prostate or epithelial cells, compared to other cells. Preferred embodiments of CA genes used in the methods herein include, but are not limited to the nucleic acids selected from Tables 1-19 (human genomic sequences of SEQ IDNOS: 4, 10, 16, 22, 28, 34, 40, 46, 52, 58, 66, 72, 80, 86, 98, 104, 110, 132, and 138, and sequences of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 corresponding to the human mRNAs generated therefrom). The methods further include adding a drug candidate to the cell and determining the effect of the drug candidate on the expression of the CA gene.

In one embodiment, the method of screening drug candidates includes comparing the level of expression in the absence of the drug candidate to the level of expression in the presence of the drug candidate.

Also provided herein is a method of screening for a bioactive agent capable of binding to a CA protein (CAP), the method comprising combining the CAP and a candidate bioactive agent, and determining the binding of the candidate agent to the CAP.

Further provided herein is a method for screening for a bioactive agent capable of modulating the activity of a CAP. In one embodiment, the method comprises combining the CAP and a candidate bioactive agent, and determining the effect of the candidate agent on the bioactivity of the CAP.

Also provided is a method of evaluating the effect of a candidate cancer drug comprising administering the drug to a patient and removing a cell sample from the patient. The expression profile of the cell is then determined. This method may further comprise comparing the expression profile of the patient to an expression profile of a healthy individual.

In a further aspect, a method for inhibiting the activity of a CA protein is provided. In one embodiment, the method comprises administering to a patient an inhibitor of a CA protein preferably selected from the group consisting of the sequences outlined in Tables 1-19 (SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146).

A method of neutralizing the effect of a CA protein, preferably a protein encoded by a nucleic acid selected from the group of sequences outlined in Tables 1-19 (human genomic sequences of SEQ ID NOS: 4, 10, 16, 22, 28, 34, 40, 46, 52, 58, 66, 72, 80, 86, 98, 104, 110, 132, and 138, and sequences of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 corresponding to the human mRNAs generated therefrom), is also provided. Preferably, the method comprises contacting an agent specific for said protein with said protein in an amount sufficient to effect neutralization.

Moreover, provided herein is a biochip comprising a nucleic acid segment which encodes a CA protein, preferably selected from the sequences outlined in Tables 1-19 (SEQ ID NOS: 5,11,17,23,29, 35,41,47, 53, 59, 61,67, 73, 75, 81, 87, 89, 91, 93, 99, 105, 111, 113, 115, 117, 119, 121, 123, 125, 127, 133, 139, 141, 143, and 145).

Also provided herein is a method for diagnosing or determining the propensity to cancers, especially lymphoma or leukemia or carcinoma by sequencing at least one carcinoma or lymphoma gene of an individual. In yet another aspect of the invention, a method is provided for determining cancer including lymphoma and leukemia gene copy numbers in an individual.

The invention provides an isolated nucleic acid comprising at least 10, 12, 15, 20 or 30 contiguous nucleotides of a sequence selected from the group consisting of the polynucleotide sequences SEQ ID NOS: 5,11,17,23,29, 35,41,47, 53, 59, 61,67,73, 75, 81, 87, 89, 91, 93, 99, 105, 111, 113, 115, 117, 119, 121, 123, 125, 127, 133, 139, 141, 143, and 145 shown in Tables 1-19, or its complement, or an expression vector comprising the isolated nucleic acids and host cells comprising them.

In some embodiments, the polynucleotide, or its complement or a fragment thereof, further comprises a detectable label, is attached to a solid support, is prepared at least in part by chemical synthesis, is an antisense fragment, is single stranded, is double stranded or comprises a microarray.

The invention provides an isolated polypeptide, encoded within an open reading frame of a CA sequence selected from the group consisting of the polynucleotide sequences of SEQ ID NOS: 4, 10, 16, 22, 28, 34, 40, 46, 52, 58, 66, 72, 80, 86, 98, 104, 110, 132, and 138 shown in Tables 1-19, or its complement. The invention provides an isolated polypeptide, wherein said polypeptide comprises the amino acid sequence encoded by a polynucleotide selected from the group consisting of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19. The invention provides an isolated polypeptide, wherein said polypeptide comprises the amino acid sequence encoded by a polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19.

The invention further provides an isolated polypeptide, comprising the amino acid sequence of an epitope of the amino acid sequence of a CA polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, wherein the polypeptide or fragment thereof may be attached to a solid support. In one embodiment the invention provides an isolated antibody (monoclonal or polyclonal) or antigen binding fragment thereof, that binds to such a polypeptide. The isolated antibody or antigen binding fragment thereof may be attached to a solid support, or further comprises a detectable label.

In one embodiment, the invention provides a kit for diagnosing the presence of cancer in a test sample, said kit comprising at least one polynucleotide that selectively hybridizes to a CA polynucleotide sequence shown in Tables 1-19, or its complement. In another embodiment the invention provides an electronic library comprising a CA polynucleotide, a CA polypeptide, or fragment thereof, shown in Tables 1-19.

In one embodiment, the invention provides a method of screening for anticancer activity comprising: (a) providing a cell that expresses a cancer associated (CA) gene encoded by a nucleic acid sequence selected from the group consisting of the CA sequences shown in Tables 1-19, or fragment thereof; (b) contacting a tissue sample derived from a cancer cell with an anticancer drug candidate; (c) monitoring an effect of the anticancer drug candidate on an expression of the CA polynucleotide in the tissue sample, and optionally (d) comparing the level of expression in the absence of said drug candidate to the level of expression in the presence of the drug candidate.

In one embodiment, the invention provides a method for detecting cancer associated with expression of a polypeptide in a test cell sample, comprising the steps of: (i) detecting a level of expression of at least one polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or a fragment thereof; and (ii) comparing the level of expression of the polypeptide in the test sample with a level of expression of polypeptide in a normal cell sample, wherein an altered level of expression of the polypeptide in the test cell sample relative to the level of polypeptide expression in the normal cell sample is indicative of the presence of cancer in the test cell sample.

In another embodiment, the invention provides a method for detecting cancer associated with expression of a polypeptide in a test cell sample, comprising the steps of: (i) detecting a level of G-protein coupled receptor (GPCR) activity of at least one polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or a fragment thereof, wherein said activity corresponds to at least one activity for the polypeptide listed in Table 21; and (ii) comparing the level of G-protein coupled receptor (GPCR) activity of the polypeptide in the test sample with a level of activity of polypeptide in a normal cell sample, wherein an altered level of activity of the polypeptide in the test cell sample relative to the level of polypeptide activity in the normal cell sample is indicative of the presence of cancer in the test cell sample.

In another embodiment, the invention provides a method for detecting cancer associated with the presence of an antibody in a test serum sample, comprising the steps of (i) detecting a level of an antibody against an antigenic polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or antigenic fragment thereof; and (ii) comparing said level of said antibody in the test sample with a level of said antibody in the control sample, wherein an altered level of antibody in said test sample relative to the level of antibody in the control sample is indicative of the presence of cancer in the test serum sample.

The invention provides a method for screening for a bioactive agent capable of modulating the activity of a CA protein (CAP), wherein said CAP is encoded by a nucleic acid comprising a nucleic acid sequence selected from the group consisting of the polynucleotide sequences SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19, said method comprising: a) combining said CAP and a candidate bioactive agent; and b) determining the effect of the candidate agent on the bioactivity of said CAP. According to the method the bioactive agent: affects the expression of the CA protein (CAP); affects the G-protein coupled receptor (GPCR) activity of the CA protein (CAP), wherein such activity is selected from the activities listed in Table 21.

In one embodiment, the invention provides a method for diagnosing cancer comprising: a) determining the expression of one or more genes comprising a nucleic acid sequence selected from the group consisting of the human genomic and mRNA sequences outlined in Tables 1-19, in a first tissue type of a first individual; and b) comparing said expression of said gene(s) from a second normal tissue type from said first individual or a second unaffected individual; wherein a difference in said expression indicates that the first individual has cancer.

In another embodiment the invention provides a method for treating cancers comprising administering to a patient a bioactive agent modulating the G-protein coupled receptor (GPCR) activity of a CA protein (CAP), wherein said CAP is encoded by a nucleic acid comprising a nucleic acid sequence selected from the group consisting of the human nucleic acid sequences in Tables 1-19 and further wherein the bioactive agent binds to the CA protein, wherein the CA protein is a G-protein coupled receptor (GPCR) protein as shown in Tables 1-19.

The invention provides monoclonal antibodies that preferentially binds to a CA protein (CAP) that is expressed on a cell surface, wherein the CA protein selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146; preferably to the extracellular domain of the CA protein; preferably to a CA protein differentially expressed on a cancer cell surface relative to a normal cell surface or preferably to at least one human cancer cell line; preferably linked to a therapeutic agent; or preferably humanized. Kits and pharmaceutical compositions for detecting a presence or an absence of cancer cells in an individual, and comprising such antibodies are also provided.

The invention also provides a method for detecting a presence or an absence of cancer cells in an individual, the method comprising: contacting cells from the individual with the antibody according to the invention; and detecting a complex of a CAP from the cancer cells and the antibody, wherein detection of the complex correlates with the presence of cancer cells in the individual. In one embodiment the invention provides a method for inhibiting growth of cancer cells in an individual, the method comprising: administering to the individual an effective amount of a pharmaceutical composition according to the invention. In another embodiment the invention provides a method for delivering a therapeutic agent to cancer cells in an individual, the method comprising:

administering to the individual an effective amount of a pharmaceutical composition according to according to the invention.

Novel sequences associated with cancer are also provided herein. Other aspects of the invention will become apparent to the skilled artisan by the following description of the invention.

BRIEF DESCRIPTION OF THE FIGURES

**Figure 1** depicts PCR amplification of host-provirus junction fragments.

**Figure 2** shows an example of average threshold cycle ($C_T$) values for a housekeeper gene and target gene.

**Figure 3** shows an example of the calculated difference ($\Delta\Delta C_T$) between the $C_T$ values of target and housekeeper genes ($\Delta C_T$) for various samples.

**Figure 4** shows the $\Delta\Delta C_T$ and comparative expression level for each sample from Figure 3.

DETAILED DESCRIPTION OF PREFERRED EMBODIMENTS

The present invention is directed to a number of sequences associated with cancers, especially lymphoma, breast cancer or prostate cancer. The relatively tight linkage between clonally-integrated proviruses and protooncogenes forms "provirus tagging", in which slow-transforming retroviruses that act by an insertion mutation mechanism are used to isolate protooncogenes. In some models, uninfected animals have low cancer rates, and infected animals have high cancer rates. It is known that many of the retroviruses involved do not carry transduced host protooncogenes or pathogenic *trans*-acting viral genes, and thus the cancer incidence must therefore be a direct consequence of proviral integration effects into host protooncogenes. Since proviral integration is random, rare integrants will "activate" host protooncogenes that provide a selective growth advantage, and these rare events result in new proviruses at clonal stoichiometries in tumors. In contrast to mutations caused by chemicals, radiation, or spontaneous errors, protooncogene insertion mutations can be easily located by virtue of the fact that a convenient-sized genetic marker of known sequence (the provirus) is present at the site of mutation. Host sequences that flank clonally integrated proviruses can be cloned using a variety of strategies. Once these sequences are in hand, the tagged protooncogenes can be subsequently identified. The presence of provirus at the same locus in two or more independent tumors is *prima facie* evidence that a protooncogene is present at or very near the provirus integration sites. This is because the genome is too large for random integrations to result in observable clustering. Any clustering that is detected is unequivocal evidence for biological selection (i.e. the tumor phenotype). Moreover, the pattern of proviral integrants (including orientations) provides compelling positional information that makes localization of the target gene at each cluster relatively simple. The three mammalian retroviruses that are known to cause cancer by an insertion mutation mechanism are FeLV (leukemia/lymphoma in cats), MLV (leukemia/lymphoma in mice and rats), and MMTV (mammary cancer in mice).

Thus, the use of oncogenic retroviruses, whose sequences insert into the genome of the host organism resulting in cancer, allows the identification of host sequences involved in cancer. These sequences may then be used in a number of different ways, including diagnosis, prognosis, screening for modulators (including both agonists and antagonists), antibody generation (for immunotherapy and imaging), etc. However, as will be appreciated by those in the art, oncogenes that are identified in one type of cancer such as lymphoma or leukemia have a strong likelihood of being involved in other types of cancers as well. Thus, while the sequences outlined herein are initially identified as correlated with lymphoma, they can also be found in other types of cancers as well, outlined below.

**Definitions**

Accordingly, the present invention provides nucleic acid and protein sequences that are associated with cancer, herein termed "cancer associated" or "CA" sequences. In one embodiment, the present invention provides nucleic acid and protein sequences that are associated with cancers that originate in lymphatic tissue, herein termed "lymphoma associated," "leukemia associated" or "LA" sequences. In another embodiment, the present invention provides nucleic acid and protein sequences that are associated with carcinomas which originate in breast tissue, herein termed "breast cancer associated" or "BC" sequences.

Suitable cancers that can be diagnosed or screened for using the methods of the present invention include cancers classified by site or by histological type. Cancers classified by site include cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, nos; ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemias (lymphocytic leukemia; myeloid leukemia; monocytic leukemia; and other leukemias).

Other cancers, classified by histological type, that may be associated with the sequences of the invention include, but are not limited to, Neoplasm, malignant; Carcinoma, NOS; Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchiolo-alveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcinoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus, malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astrocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia.

In addition, the CA genes may be involved in other diseases such as, but not limited to, diseases associated with aging or neurodegeneration.

"Association" in this context means that the nucleotide or protein sequences are either differentially expressed, activated, inactivated or altered in cancers as compared to normal tissue. As outlined below, CA sequences include those that are up-regulated (i.e. expressed at a higher level), as well as those that are down-regulated (i.e. expressed at a lower level), in cancers. CA sequences also include sequences that have been altered (i.e., truncated sequences or sequences with substitutions, deletions or insertions, including point mutations) and show either the same expression profile or an altered profile. In a preferred embodiment, the CA sequences are from humans; however, as will be appreciated by those in the art, CA sequences from other organisms may be useful in animal models of disease and drug evaluation; thus, other CA sequences are provided, from vertebrates, including mammals, including rodents (rats, mice, hamsters, guinea pigs, etc.), primates, and farm animals (including sheep, goats, pigs, cows, horses, etc). In some cases, prokaryotic CA sequences may be useful. CA sequences from other organisms may be obtained using the techniques outlined below.

CA sequences include both nucleic acid and amino acid sequences. In a preferred embodiment, the CA sequences are recombinant nucleic acids. By the term "recombinant nucleic acid" herein is meant nucleic acid, originally formed in vitro, in general, by the manipulation of nucleic acid by polymerases and endonucleases, in a form not normally found in nature. Thus a recombinant nucleic acid is also an isolated nucleic acid, in a linear form, or cloned in a vector formed in vitro by ligating DNA molecules that are not normally joined, are both considered recombinant for the purposes of this invention. It is understood that once a recombinant nucleic acid is made and reintroduced into a host cell or organism, it will replicate using the in vivo cellular machinery of the host cell rather than in vitro manipulations; however, such nucleic acids, once produced recombinantly, although subsequently replicated in vivo, are still considered recombinant or isolated for the purposes of the invention. As used herein a "polynucleotide" or "nucleic acid" is a polymeric form of nucleotides of any length, either ribonucleotides or deoxyribonucleotides. This term refers only to the primary structure of the molecule. Thus, this term includes double- and single-stranded DNA and RNA. It also includes known types of modifications, for example, labels which are known in the art, methylation, "caps", substitution of one or more of the naturally occurring nucleotides with an analog, internucleotide modifications such as, for example, those with uncharged linkages (e.g., phosphorothioates, phosphorodithioates, etc.), those containing pendant moieties, such as, for example proteins (including e.g., nucleases, toxins, antibodies, signal peptides, poly-L-lysine, etc.),those with intercalators (e.g., acridine, psoralen, etc.), those containing chelators (e.g., metals, radioactive metals, etc.), those containing alkylators, those with modified linkages (e.g., alpha anomeric nucleic acids, etc.), as well as unmodified forms of the polynucleotide.

As used herein, a polynucleotide "derived from" a designated sequence refers to a polynucleotide sequence which is comprised of a sequence of approximately at least about 6 nucleotides, preferably at least about 8 nucleotides, more preferably at least about 10-12 nucleotides, and even more preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence that is unique to a CA gene.

Similarly, a "recombinant protein" is a protein made using recombinant techniques, i.e. through the expression of a recombinant nucleic acid as depicted above. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises about 50-75% by weight of the total protein, with about 80% being preferred, and about 90% being particularly preferred. The definition includes the production of a CA protein from one organism in a different organism or host cell. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and deletions, as discussed below.

In a preferred embodiment, the CA sequences are nucleic acids. As will be appreciated by those in the art and is more fully outlined below, CA sequences are useful in a variety of applications, including diagnostic applications, which will detect naturally occurring nucleic acids, as well as screening applications; for example, biochips comprising nucleic acid probes to the CA sequences can be generated. In the broadest sense, use of "nucleic acid," "polynucleotide" or "oligonucleotide" or equivalents herein means at least two nucleotides covalently linked together. In some embodiments, an oligonucleotide is an oligomer of 6, 8, 10, 12, 20, 30 or up to 100 nucleotides. A "polynucleotide" or "oligonucleotide" may comprise DNA, RNA, PNA or a polymer of nucleotides linked by phosphodiester and/or any alternate bonds.

A nucleic acid of the present invention generally contains phosphodiester bonds, although in some cases, as outlined below (for example, in antisense applications or when a nucleic acid is a candidate drug agent), nucleic acid analogs may have alternate backbones, comprising, for example, phosphoramidate (Beaucage et al., Tetrahedron 49 (10):1925 (1993) and references therein; Letsinger, J. Org. Chem. 35:3800 (1970); Sprinzl et al., Eur. J. Biochem. 81: 579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Patent No. 5,644,048), phosphorodithioate (Briu et al., J. Am. Chem. Soc. 111:2321 (1989), O-methylphosphoroamidite linkages (see Eckstein, Oligonucleotides and Analogues: A Practical Approach, Oxford University Press), and peptide nucleic acid backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic acids include those with positive backbones (Denpcy et al., Proc. Natl. Acad. Sci. USA 92:6097 (1995); non-ionic backbones (U.S. Patent Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem. Lett. 4:395 (1994); Jeffs et al., J. Biomolecular NMR 34: 17 (1994); Tetrahedron Lett. 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within one definition of nucleic acids (see Jenkins et al., Chem. Soc. Rev. (1995) pp169-176). Several nucleic acid analogs are described in Rawls, C & E News June 2, 1997 page 35. All of these references are hereby expressly incorporated by reference. These modifications of the ribose-phosphate backbone may be done for a variety of reasons, for example to increase the stability and half-life of such molecules in physiological environments for use in anti-sense applications or as probes on a biochip.

As will be appreciated by those in the art, all of these nucleic acid analogs may find use in the present invention. In addition, mixtures of naturally occurring nucleic acids and analogs can be made; alternatively, mixtures of different nucleic acid analogs, and mixtures of naturally occurring nucleic acids and analogs may be made.

The nucleic acids may be single stranded or double stranded, as specified, or contain portions of both double stranded or single stranded sequence. As will be appreciated by those in the art, the depiction of a single strand "Watson" also defines the sequence of the other strand "Crick"; thus the sequences described herein also includes the complement of the sequence. The nucleic acid may be DNA, both genomic and cDNA, RNA, or a hybrid, where the nucleic acid contains any combination of deoxyribo- and ribo-nucleotides, and any combination of bases, including uracil, adenine, thymine, cytosine, guanine, inosine, xanthine, hypoxanthine, isocytosine, isoguanine, etc. As used herein, the term "nucleoside" includes nucleotides and nucleoside and nucleotide analogs, and modified nucleosides such as amino modified nucleosides. In addition, "nucleoside" includes non-naturally occurring analog structures. Thus for example the individual units of a peptide nucleic acid, each containing a base, are referred to herein as a nucleoside.

As used herein, the term "tag," "sequence tag" or "primer tag sequence" refers to an oligonucleotide with specific nucleic acid sequence that serves to identify a batch of polynucleotides bearing such tags therein. Polynucleotides from the same biological source are covalently tagged with a specific sequence tag so that in subsequent analysis the polynucleotide can be identified according to its source of origin. The sequence tags also serve as primers for nucleic acid amplification reactions.

A "microarray" is a linear or two-dimensional array of preferably discrete regions, each having a defined area, formed on the surface of a solid support. The density of the discrete regions on a microarray is determined by the total numbers of target polynucleotides to be detected on the surface of a single solid phase support, preferably at least about $50/cm^2$, more preferably at least about $100/cm^2$, even more preferably at least about $500/cm^2$, and still more preferably at least about $1,000/cm^2$. As used herein, a DNA microarray is an array of oligonucleotide primers placed on a chip or other surfaces used to amplify or clone target polynucleotides. Since the position of each particular group of primers in the array is known, the identities of the target polynucleotides can be determined based on their binding to a particular position in the microarray.

A "linker" is a synthetic oligodeoxyribonucleotide that contains a restriction site. A linker may be blunt end-ligated onto the ends of DNA fragments to create restriction sites that can be used in the subsequent cloning of the fragment into a vector molecule.

The term "label" refers to a composition capable of producing a detectable signal indicative of the presence of the target polynucleotide in an assay sample. Suitable labels include radioisotopes, nucleotide chroinophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical, chemical, or any other appropriate means. The term "label" is used to refer to any chemical group or moiety having a detectable physical property or any compound capable of causing a chemical group or moiety to exhibit a detectable physical property, such as an enzyme that catalyzes conversion of a substrate into a detectable product. The term "label" also encompasses compounds that inhibit the expression of a particular physical property. The label may also be a compound that is a member of a binding pair, the other member of which bears a detectable physical property.

The term "support" refers to conventional supports such as beads, particles, dipsticks, fibers, filters, membranes, and silane or silicate supports such as glass slides.

The term "amplify" is used in the broad sense to mean creating an amplification product which may include, for example, additional target molecules, or target-like molecules or molecules complementary to the target molecule, which molecules are created by virtue of the presence of the target molecule in the sample. In the situation where the target is a nucleic acid, an amplification product can be made enzymatically with DNA or RNA polymerases or reverse transcriptases.

As used herein, a "biological sample" refers to a sample of tissue or fluid isolated from an individual, including but not limited to, for example, blood, plasma, serum, spinal fluid, lymph fluid, skin, respiratory, intestinal and genitourinary tracts, tears, saliva, milk, cells (including but not limited to blood cells), tumors, organs, and also samples of *in vitro* cell culture constituents.

The term "biological sources" as used herein refers to the sources from which the target polynucleotides are derived. The source can be of any form of "sample" as described above, including but not limited to, cell, tissue or fluid. "Different biological sources" can refer to different cells/tissues/organs of the same individual, or cells/tissues/organs from different individuals of the same species, or cells/tissues/organs from different species.

**Cancer-associated Sequences**

The CA sequences of the invention were initially identified by infection of mice with a retrovirus such as murine leukemia virus (MLV) resulting in lymphoma. Retroviruses have a genome that is made out of RNA. After a retrovirus infects a host cell, a double stranded DNA copy of the retrovirus genome (a "provirus") is inserted into the genomic DNA of the host cell. The integrated provirus may affect the expression of host genes at or near the site of integration - a phenomenon known as retroviral insertional mutagenesis. Possible changes in the expression of host cell genes include: (i) increased expression of genes near the site of integration resulting from the proximity of elements in the provirus that act as transcriptional promoters and enhancers, (ii) functional inactivation of a gene caused by the integration of a provirus into the gene itself thus preventing the synthesis of a functional gene product, or (iii) expression of a mutated protein that has a different activity to the normal protein. Typically such a protein would be prematurely truncated and lack a regulatory domain near the C terminus. Such a protein might be constitutively active, or act as a dominant negative inhibitor of the normal protein. For example, retrovirus enhancers, including that of SL3-3, are known to act on genes up to approximately 200 kilobases from the insertion site. Moreover, many of these sequences are also involved in other cancers and disease states. Sequences of mouse genes according to this invention, that are identified in this manner are shown as mDxx-yyy in Tables 1-19.

A CA sequence can be initially identified by substantial nucleic acid and/or amino acid sequence homology to the CA sequences outlined herein. Such homology can be based upon the overall nucleic acid or amino acid sequence, and is generally determined as outlined below, using either homology programs or hybridization conditions.

In one embodiment, CA sequences are those that are up-regulated in cancers; that is, the expression of these genes is higher in cancer tissue as compared to normal tissue of the same differentiation stage. "Up-regulation" as used herein means increased expression by about 50%, preferably about 100%, more preferably about 150% to about 200%, with up-regulation from 300% to 1000% being preferred..

In another embodiment, CA sequences are those that are down-regulated in cancers; that is, the expression of these genes is lower in cancer tissue as compared to normal tissue of the same differentiation stage. "Down-regulation" as used herein means decreased expression by about 50%, preferably about 100%, more preferably about 150% to about 200%, with down-regulation from 300% to 1000% to no expression being preferred.

In yet another embodiment, CA sequences are those that have altered sequences but show either the same or an altered expression profile as compared to normal lymphoid tissue of the same differentiation stage. "Altered CA sequences" as used herein also refers to sequences that are truncated, contain insertions or contain point mutations.

CA proteins of the present invention may be classified as secreted proteins, transmembrane proteins or intracellular proteins. In a preferred embodiment the CA protein is an intracellular protein. Intracellular proteins may be found in the cytoplasm and/or in the nucleus. Intracellular proteins are involved in all aspects of cellular function and replication (including, for example, signaling pathways); aberrant expression of such proteins results in unregulated or disregulated cellular processes. For example, many intracellular proteins have enzymatic activity such as protein kinase activity, protein phosphatase activity, protease activity, nucleotide cyclase activity, polymerase activity and the like. Intracellular proteins also serve as docking proteins that are involved in organizing complexes of proteins, or targeting proteins to various subcellular localizations, and are involved in maintaining the structural integrity of organelles.

An increasingly appreciated concept in characterizing intracellular proteins is the presence in the proteins of one or more motifs for which defined functions have been attributed. In addition to the highly conserved sequences found in the enzymatic domain of proteins, highly conserved sequences have been identified in proteins that are involved in protein-protein interaction. For example, Src-homology-2 (SH2) domains bind tyrosine-phosphorylated targets in a sequence dependent manner. PTB domains, which are distinct from SH2 domains, also bind tyrosine phosphorylated targets. SH3 domains bind to proline-rich targets. In addition, PH domains, tetratricopeptide repeats and WD domains to name only a few, have been shown to mediate protein-protein interactions. Some of these may also be involved in binding to phospholipids or other second messengers. As will be appreciated by one of ordinary skill in the art, these motifs can be identified on the basis of primary sequence; thus, an analysis of the sequence of proteins may provide insight into both the enzymatic potential of the molecule and/or molecules with which the protein may associate.

In a preferred embodiment, the CA sequences are transmembrane proteins. Transmembrane proteins are molecules that span the phospholipid bilayer of a cell. They may have an intracellular domain, an extracellular domain, or both. The intracellular domains of such proteins may have a number of functions including those already described for intracellular proteins. For example, the intracellular domain may have enzymatic activity and/or may serve as a binding site for additional proteins. Frequently the intracellular domain of transmembrane proteins serves both roles. For example certain receptor tyrosine kinases have both protein kinase activity and SH2 domains. In addition, autophosphorylation of tyrosines on the receptor molecule itself creates binding sites for additional SH2 domain containing proteins.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors are classified as "seven transmembrane domain" proteins, as they contain 7 membrane spanning regions. Important transmembrane protein receptors include, but are not limited to insulin receptor, insulin-like growth factor receptor, human growth hormone receptor, glucose transporters, transferrin receptor, epidermal growth factor receptor, low density lipoprotein receptor, leptin receptor, interleukin receptors, e.g. IL-1 receptor, IL-2 receptor, etc. CA proteins may be derived from genes that regulate apoptosis (IL-3, GM-CSF and Bcl-x) or are shown to have a role in the regulation of apoptosis.

Characteristics of transmembrane domains include approximately 20 consecutive hydrophobic amino acids that may be followed by charged amino acids. Therefore, upon analysis of the amino acid sequence of a particular protein, the localization and number of transmembrane domains within the protein may be predicted.

The extracellular domains of transmembrane proteins are diverse; however, conserved motifs are found repeatedly among various extracellular domains. Conserved structure and/or functions have been ascribed to different extracellular motifs. For example, cytokine receptors are characterized by a cluster of cysteines and a WSXWS (W= tryptophan, S= serine, X=any amino acid) motif. Immunoglobulin-like domains are highly conserved. Mucin-like domains may be involved in cell adhesion and leucine-rich repeats participate in protein-protein interactions.

Many extracellular domains are involved in binding to other molecules. In one aspect, extracellular domains are receptors. Factors that bind the receptor domain include circulating ligands, which may be peptides, proteins, or small molecules such as adenosine and the like. For example, growth factors such as EGF, FGF and PDGF are circulating growth factors that bind to their cognate receptors to initiate a variety of cellular responses. Other factors include cytokines, mitogenic factors, neurotrophic factors and the like. Extracellular domains also bind to cell-associated molecules. In this respect, they mediate cell-cell interactions. Cell-associated ligands can be tethered to the cell for example via a glycosylphosphatidylinositol (GPI) anchor, or may themselves be transmembrane proteins. Extracellular domains also associate with the extracellular matrix and contribute to the maintenance of the cell structure.

CA proteins that are transmembrane are particularly preferred in the present invention as they are good targets for immunotherapeutics, as are described herein. In addition, as outlined below, transmembrane proteins can be also useful in imaging modalities.

It will also be appreciated by those in the art that a transmembrane protein can be made soluble by removing transmembrane sequences, for example through recombinant methods. Furthermore, transmembrane proteins that have been made soluble can be made to be secreted through recombinant means by adding an appropriate signal sequence.

In a preferred embodiment, the CA proteins are secreted proteins; the secretion of which can be either constitutive or regulated. These proteins have a signal peptide or signal sequence that targets the molecule to the secretory pathway. Secreted proteins are involved in numerous physiological events; by virtue of their circulating nature, they serve to transmit signals to various other cell types. The secreted protein may function in an autocrine manner (acting on the cell that secreted the factor), a paracrine manner (acting on cells in close proximity to the cell that secreted the factor) or an endocrine manner (acting on cells at a distance). Thus secreted molecules find use in modulating or altering numerous aspects of physiology. CA proteins that are secreted proteins are particularly preferred in the present invention as they serve as good targets for diagnostic markers, for example for blood tests.

## CA sequences and homologs

A CA sequence is initially identified by substantial nucleic acid and/or amino acid sequence homology to the CA sequences outlined herein. Such homology can be based upon the overall nucleic acid or amino acid sequence, and is generally determined as outlined below, using either homology programs or hybridization conditions.

As used herein, a nucleic acid is a "CA nucleic acid" if the overall homology of the nucleic acid sequence to one of the nucleic acids of Tables 1-19 is preferably greater than about 75%, more preferably greater than about 80%, even more preferably greater than about 85% and most preferably greater than 90%. In some embodiments the homology will be as high as about 93 to 95 or 98%. In a preferred embodiment, the sequences that are used to determine sequence identity or similarity are selected from those of the nucleic acids of Tables 1-19. In another embodiment, the sequences are naturally occurring allelic variants of the sequences of the nucleic acids of Tables 1-19. In another embodiment, the sequences are sequence variants as further described herein.

Homology in this context means sequence similarity or identity, with identity being preferred. A preferred comparison for homology purposes is to compare the sequence containing sequencing errors to the correct sequence. This homology will be determined using standard techniques known in the art, including, but not limited to, the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters include a default gap weight of 3.00, a default gap length weight of 0.10, and weighted end gaps.

Another example of a useful algorithm is the BLAST (Basic Local Alignment Search Tool) algorithm, described in Altschul et al., J. Mol. Biol. 215, 403-410, (1990) and Karlin et al., PNAS USA 90:5873-5787 (1993). A particularly useful BLAST program is the WU-BLAST-2 program which was obtained from Altschul et al., Methods in Enzymology, 266: 460-480 (1996); http://blast.wustl.edu/]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity. A percent amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the nucleic acids of Tables 1-19. A preferred method utilizes the BLASTN module of WU-BLAST-2 set to the default parameters, with overlap span and overlap fraction set to 1 and 0.125, respectively.

The alignment may include the introduction of gaps in the sequences to be aligned. In addition, for sequences which contain either more or fewer nucleotides than those of the nucleic acids of Tables 1-19, it is understood that the percentage of homology will be determined based on the number of homologous nucleosides in relation to the total number of nucleosides. Thus homology of sequences shorter than those of the sequences identified herein will be determined using the number of nucleosides in the shorter sequence.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5x SSC ("saline sodium citrate"; 9 mM NaCl, 0.9 mM sodium citrate), 0.5% SDS, 1.0 mM EDTA (pH 8.0); hybridizing at 50-60° C, 5x SSC, overnight; followed by washing twice at 65° C for 20 minutes with each of 2x, 0.5x and 0.2x SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, e.g., to 60-65° C, or 65-70° C. Stringent conditions may also be achieved with the addition of destabilizing agents such as formamide.

Thus nucleic acids that hybridize under high stringency to the nucleic acids identified in the figures, or their complements, are considered CA sequences. High stringency conditions are known in the art; see for example Maniatis et al., Molecular Cloning: A Laboratory Manual, 2d Edition, 1989, and Short Protocols in Molecular Biology, ed. Ausubel, et al., both of which are hereby incorporated by reference. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Acid Probes, "Overview of principles of hybridization and the strategy of nucleic acid assays" (1993). Generally, stringent conditions are selected to be about 5-10°C lower than the thermal melting point ($T_m$) for the specific sequence at a defined ionic strength pH. The $T_m$ is the temperature (under defined ionic strength, pH and nucleic acid concentration) at which 50% of the probes complementary to the target hybridize to the target sequence at equilibrium (as the target sequences are present in excess, at $T_m$, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60°C for longer probes (e.g. greater than 50 nucleotides). In another embodiment, less stringent hybridization conditions are used; for example, moderate or low stringency conditions may be used, as are known in the art; see Maniatis and Ausubel, supra, and Tijssen, supra.

In addition, the CA nucleic acid sequences of the invention are fragments of larger genes, i.e. they are nucleic acid segments. Alternatively, the CA nucleic acid sequences can serve as indicators of oncogene position, for example, the CA sequence may be an enhancer that activates a protooncogene. "Genes" in this context includes coding regions, non-coding regions, and mixtures of coding and non-coding regions. Accordingly, as will be appreciated by those in the art, using the sequences provided herein, additional sequences of the CA genes can be obtained, using techniques well known in the art for cloning either longer sequences or the full-length sequences; see Maniatis et al., and Ausubel, et al., supra, hereby expressly incorporated by reference. In general, this is done using PCR, for example, kinetic PCR.

**Detection of CA Expression**

Once the CA nucleic acid is identified, it can be cloned and, if necessary, its constituent parts recombined to form the entire CA nucleic acid. Once isolated from its natural source, e.g., contained within a plasmid or other vector or excised therefrom as a linear nucleic acid segment, the recombinant CA nucleic acid can be further used as a probe to identify and isolate other CA nucleic acids, for example additional coding regions. It can also be used as a "precursor" nucleic acid to make modified or variant CA nucleic acids and proteins. In a preferred embodiment, once a CA gene is identified its nucleotide sequence is used to design probes specific for the CA gene.

The CA nucleic acids of the present invention are used in several ways. In a first embodiment, nucleic acid probes hybridizable to CA nucleic acids are made and attached to biochips to be used in screening and diagnostic methods, or for gene therapy and/or antisense applications. Alternatively, the CA nucleic acids that include coding regions of CA proteins can be put into expression vectors for the expression of CA proteins, again either for screening purposes or for administration to a patient.

Recent developments in DNA microarray technology make it possible to conduct a large scale assay of a plurality of target CA nucleic acid molecules on a single solid phase support. U.S. Pat. No. 5,837,832 (Chee *et al.*) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells, often a cell of interest and a control cell, is compared and any differences in gene expression among the respective cells are identified. Such information is useful for the identification of the types of genes expressed in a particular cell or tissue type and diagnosis of cancer conditions based on the expression profile.

Typically, RNA from the sample of interest is subjected to reverse transcription to obtain labeled cDNA. *See* U.S. Pat. No. 6,410,229 (Lockhart et al.) The cDNA is then hybridized to oligonucleotides or cDNAs of known sequence arrayed on a chip or other surface in a known order. The location of the oligonucleotide to which the labeled cDNA hybridizes provides sequence information on the cDNA, while the amount of labeled hybridized RNA or cDNA provides an estimate of the relative representation of the RNA or cDNA of interest. *See* Schena, et al. Science 270:467-470 (1995). For example, use of a cDNA microarray to analyze gene expression patterns in human cancer is described by DeRisi, et al. (Nature Genetics 14:457-460 (1996)).

In a preferred embodiment, nucleic acid probes corresponding to CA nucleic acids (both the nucleic acid sequences outlined in the figures and/or the complements thereof) are made. Typically, these probes are synthesized based on the disclosed sequences of this invention. The nucleic acid probes attached to the biochip are designed to be substantially complementary to the CA nucleic acids, i.e. the target sequence (either the target sequence of the sample or to other probe sequences, for example in sandwich assays), such that specific hybridization of the target sequence and the probes of the present invention occurs. As outlined below, this complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions, as outlined herein. Whether or not a sequence is unique to a CA gene according to this invention can be determined by techniques known to those of skill in the art. For example, the sequence can be compared to sequences in databanks, e.g., GeneBank, to determine whether it is present in the uninfected host or other organisms. The sequence can also be compared to the known sequences of other viral agents, including those that are known to induce cancer.

A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 30 to about 50 bases being particularly preferred. That is, generally entire genes are rarely used as probes. In some embodiments, much longer nucleic acids can be used, up to hundreds of bases. The probes are sufficiently specific to hybridize to complementary template sequence under conditions known by those of skill in the art. The number of mismatches between the probes sequences and their complementary template (target) sequences to which they hybridize during hybridization generally do not exceed 15%, usually do not exceed 10% and preferably do not exceed 5%, as determined by FASTA (default settings).

Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.

In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (*e.g.*, U.S. Patent No. 5,470,967) and polyamide (*i.e.*, peptide nucleic acids). Peptide nucleic acids are described in Nielsen et al. (1991) Science 254: 1497-1500, U.S. Patent No. 5,714,331, and Nielsen (1999) Curr. Opin. Biotechnol. 10:71-75.

In certain embodiments, the probe can be a chimeric molecule; i.e., can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind, in a sequence-specific fashion, with its target sequence. A large number of structural modifications, both known and to be developed, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis incorporates the teaching of U.S. Patent No. 5,419,966.

Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target CA gene are used. That is, two, three, four or more probes, with three being preferred, are used to build in a redundancy for a particular target. The probes can be overlapping (i.e. have some sequence in common), or specific for distinct sequences of a CA gene. When multiple target polynucleotides are to be detected according to the present invention, each probe or probe group corresponding to a particular target polynucleotide is situated in a discrete area of the microarray.

Probes may be in solution, such as in wells or on the surface of a micro-array, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. A preferred embodiment comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence. Patterns can be manually or computer analyzed, but it is clear that positional sequencing by hybridization lends itself to computer analysis and automation. Algorithms and software, which have been developed for sequence reconstruction, are applicable to the methods described herein (R. Drmanac et al., J. Biomol. Struc. & Dyn. 5:1085-1102, 1991; P. A. Pevzner, J. Biomol. Struc. & Dyn. 7:63-73, 1989).

As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal as outlined below. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

Nucleic acid probes may be attached to the solid support by covalent binding such as by conjugation with a coupling agent or by, covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody $F_c$ fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative. Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon®, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signaling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.

In a preferred embodiment, the surface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or hetero-bifunctional linkers as are well known (see 1994 Pierce Chemical Company catalog, technical section on cross-linkers, pages 155-200, incorporated herein by reference). In addition, in some cases, additional linkers, such as alkyl groups (including substituted and heteroalkyl groups) may be used.

In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.

The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling); the disclosure of which are herein incorporated in their entirety by reference. Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al., "Direct Mechanical Measurements of the Elasticity of Single DNA Molecules by Using Magnetic Beads," Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established. See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, Nucleic Acids Research, 20:1679-1684 (1992) or by covalent attachment of chemically synthesized oligonucleotides (Guo et al., supra) in combination with robotic arraying technologies.

In addition to the solid-phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences. The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of double-stranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR® Greene I, is an example of an intercalating dye, that preferentially binds to dsDNA resulting in a concomitant increase in the fluorescent signal. Sequence specific probes, such as used with TaqMan® technology, consist of a fluorochrome and a quenching molecule covalently bound to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signaling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer, S., et al., Genome Res. 10:258-266 (2000); Heid, C. A., et al., Genome Res. 6, 986-994 (1996).

**Expression of CA proteins**

In a preferred embodiment, CA nucleic acids encoding CA proteins are used to make a variety of expression vectors to express CA proteins which can then be used in screening assays, as described below. The expression vectors may be either self-replicating extrachromosomal vectors or vectors which integrate into a host genome. Generally, these expression vectors include transcriptional and translational regulatory nucleic acid operably linked to the nucleic acid encoding the CA protein. The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, synthetic oligonucleotide adaptors or linkers are used in accordance with conventional practice. The transcriptional and translational regulatory nucleic acid will generally be appropriate to the host cell used to express the CA protein; for example, transcriptional and translational regulatory nucleic acid sequences from *Bacillus* are preferably used to express the CA protein in *Bacillus*. Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, the expression vector may comprise additional elements. For example, the expression vector may have two replication systems, thus allowing it to be maintained in two organisms, for example in mammalian or insect cells for expression and in a prokaryotic host for cloning and amplification. Furthermore, for integrating expression vectors, the expression vector contains at least one sequence homologous to the host cell genome, and preferably two homologous sequences that flank the expression construct. The integrating vector may be directed to a specific locus in the host cell by selecting the appropriate homologous sequence for inclusion in the vector. Constructs for integrating vectors are well known in the art.

In addition, in a preferred embodiment, the expression vector contains a selectable marker gene to allow the selection of transformed host cells. Selection genes are well known in the art and will vary with the host cell used.

The CA proteins of the present invention are produced by culturing a host cell transformed with an expression vector containing nucleic acid encoding a CA protein, under the appropriate conditions to induce or cause expression of the CA protein. The conditions appropriate for CA protein expression will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

Appropriate host cells include yeast, bacteria, archaebacteria, fungi, and insect, plant and animal cells, including mammalian cells. Of particular interest are *Drosophila melanogaster* cells, *Saccharomyces cerevisiae* and other yeasts, *E. coli, Bacillus subtilis,* Sf9 cells, C129 cells, 293 cells, *Neurospora*, BHK, CHO, COS, HeLa cells, THP1 cell line (a macrophage cell line) and human cells and cell lines.

In a preferred embodiment, the CA proteins are expressed in mammalian cells. Mammalian expression systems are also known in the art, and include retroviral systems. A preferred expression vector system is a retroviral vector system such as is generally described in PCT/US97/01019 and PCT/US97/01048, both of which are hereby expressly incorporated by reference. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter. Typically, transcription termination and polyadenylation sequences recognized by mammalian cells are regulatory regions located 3' to the translation stop codon and thus, together with the promoter elements, flank the coding sequence. Examples of transcription terminator and polyadenylation signals include those derived form SV40.

The methods of introducing exogenous nucleic acid into mammalian hosts, as well as other hosts, are well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

In a preferred embodiment, CA proteins are expressed in bacterial systems. Bacterial expression systems are well known in the art. Promoters from bacteriophage may also be used and are known in the art. In addition, synthetic promoters and hybrid promoters are also useful; for example, the tac promoter is a hybrid of the trp and lac promoter sequences. Furthermore, a bacterial promoter can include naturally occurring promoters of non-bacterial origin that have the ability to bind bacterial RNA polymerase and initiate transcription. In addition to a functioning promoter sequence, an efficient ribosome binding site is desirable. The expression vector may also include a signal peptide sequence that provides for secretion of the CA protein in bacteria. The protein is either secreted into the growth media (gram-positive bacteria) or into the periplasmic space, located between the inner and outer membrane of the cell (gram-negative bacteria). The bacterial expression vector may also include a selectable marker gene to allow for the selection of bacterial strains that have been transformed. Suitable selection genes include genes that render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways. These components are assembled into expression vectors. Expression vectors for bacteria are well known in the art, and include vectors for *Bacillus subtilis, E. coli, Streptococcus cremoris*, and *Streptococcus lividans*, among others. The bacterial expression vectors are transformed into bacterial host cells using techniques well known in the art, such as calcium chloride treatment, electroporation, and others.

In one embodiment, CA proteins are produced in insect cells. Expression vectors for the transformation of insect cells, and in particular, baculovirus-based expression vectors, are well known in the art.

In a preferred embodiment, CA protein is produced in yeast cells. Yeast expression systems are well known in the art, and include expression vectors for *Saccharomyces cerevisiae, Candida albicans* and *C. maltosa, Hansenula polymorpha, Kluyveromyces fragilis* and *K. lactis, Pichia guillerimondii* and *P. pastoris, Schizosaccharomyces pombe,* and *Yarrowia lipolytica.*

The CA protein may also be made as a fusion protein, using techniques well known in the art. Thus, for example, for the creation of monoclonal antibodies. If the desired epitope is small, the CA protein may be fused to a carrier protein to form an immunogen. Alternatively, the CA protein may be made as a fusion protein to increase expression, or for other reasons. For example, when the CA protein is a CA peptide, the nucleic acid encoding the peptide may be linked to other nucleic acid for expression purposes.

In one embodiment, the CA nucleic acids, proteins and antibodies of the invention are labeled. By "labeled" herein is meant that a compound has at least one element, isotope or chemical compound attached to enable the detection of the compound. In general, labels fall into three classes: a) isotopic labels, which may be radioactive or heavy isotopes; b) immune labels, which may be antibodies or antigens; and c) colored or fluorescent dyes. The labels may be incorporated into the CA nucleic acids, proteins and antibodies at any position. For example, the label should be capable of producing, either directly or indirectly, a detectable signal. The detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the label may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Accordingly, the present invention also provides CA protein sequences. A CA protein of the present invention may be identified in several ways. "Protein" in this sense includes proteins, polypeptides, and peptides. As will be appreciated by those in the art, the nucleic acid sequences of the invention can be used to generate protein sequences. There are a variety of ways to do this, including cloning the entire gene and verifying its frame and amino acid sequence, or by comparing it to known sequences to search for homology to provide a frame, assuming the CA protein has homology to some protein in the database being used. Generally, the nucleic acid sequences are input into a program that will search all three frames for homology. This is done in a preferred embodiment using the following NCBI Advanced BLAST parameters. The program is blastx or blastn. The database is nr. The input data is as "Sequence in FASTA format". The organism list is "none". The "expect" is 10; the filter is default. The "descriptions" is 500, the "alignments" is 500, and the "alignment view" is pairwise. The "query Genetic Codes" is standard (1). The matrix is BLOSUM 62; gap existence cost is 11, per residue gap cost is 1; and the lambda ratio is .85 default. This results in the generation of a putative protein sequence.

In general, the term "polypeptide" as used herein refers to both the full-length polypeptide encoded by the recited polynucleotide, the polypeptide encoded by the gene represented by the recited polynucleotide, as well as portions or fragments thereof. The present invention encompasses variants of the naturally occurring proteins, wherein such variants are homologous or substantially similar to the naturally occurring protein, and can be of an origin of the same or different species as the naturally occurring protein (e.g., human, murine, or some other species that naturally expresses the recited polypeptide, usually a mammalian species). In general, variant polypeptides have a sequence that has at least about 80%, at least about 81%, at least about 82%, at least about 83%, at least about 84%, at least about 85%, at least about 86%, at least about 87%, at least about 88%, at least about 89%, usually at least about 90%, at least 91%, at least 92%, at least 93%, at least 94%, at least 95%, at least 96%, at least 97%, at least 98% and more usually at least about 99% sequence identity with a differentially expressed polypeptide described herein, as determined by the Smith-Waterman homology search algorithm using an affine gap search with a gap open penalty of 12 and a gap extension penalty of 2, BLOSUM matrix of 62. The Smith-Waterman homology search algorithm is taught in Smith and Waterman, Adv. Appl. Math. (1981) 2: 482-489. The variant polypeptides can be naturally or non-naturally glycosylated, i.e., the polypeptide has a glycosylation pattern that differs from the glycosylation pattern found in the corresponding naturally occurring protein.

Also within the scope of the invention are variants. Variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/ hydrophilicity, and/or steric bulk of the amino acid substituted. Variants can be designed so as to retain or have enhanced biological activity of a particular region of the protein (e.g., a functional domain and/or, where the polypeptide is a member of a protein family, a region associated with a consensus sequence). Selection of amino acid alterations for production of variants can be based upon the accessibility (interior vs. exterior) of the amino acid (see, e.g., Go et al, Int. J. Peptide Protein Res. (1980) 15:211), the thermostability of the variant polypeptide (see, e.g., Querol et al., Prot. Eng. (1996) 9:265), desired glycosylation sites (see, e.g., Olsen and Thomsen, J. Gen. Microbiol. (1991) 137:579), desired disulfide bridges (see, e.g., Clarke et al., Biochemistry (1993) 32:4322; and Wakarchuk et al., Protein Eng. (1994) 7:1379), desired metal binding sites (see, e.g., Toma et al., Biochemistry (1991) 30:97, and Haezerbrouck et al., Protein Eng. (1993) 6:643), and desired substitutions within proline loops (see, e.g., Masul et al., Appl. Env. Microbiol. (1994) 60:3579). Cysteine-depleted muteins can be produced as disclosed in USPN 4,959,314.

Variants also include fragments of the polypeptides disclosed herein, particularly biologically active fragments and/or fragments corresponding to functional domains. Fragments of interest will typically be at least about 8 amino acids (aa) 10 aa, 15 aa, 20 aa, 25 aa, 30 aa, 35 aa, 40 aa, to at least about 45 aa in length, usually at least about 50 aa in length, at least about 75 aa, at least about 100 aa, at least about 125 aa, at least about 150 aa in length, at least about 200 aa, at least about 300 aa, at least about 400 aa and can be as long as 500 aa in length or longer, but will usually not exceed about 1000 aa in length, where the fragment will have a stretch of amino acids that is identical to a polypeptide encoded by a polynucleotide having a sequence of any one of the polynucleotide sequences provided herein, or a homolog thereof. The protein variants described herein are encoded by polynucleotides that are within the scope of the invention. The genetic code can be used to select the appropriate codons to construct the corresponding variants.

While altered expression of the polynucleotides associated with cancer is observed, altered levels of expression of the polypeptides encoded by these polynucleotides may likely play a role in cancers.

Also included within one embodiment of CA proteins are amino acid variants of the naturally occurring sequences, as determined herein. Preferably, the variants are preferably greater than about 75% homologous to the wild-type sequence, more preferably greater than about 80%, even more preferably greater than about 85% and most preferably greater than 90%. The present application is also directed to proteins containing polypeptides at least 80%, 85%, 90%, 95%, 96%, 97%, 98% or 99% identical to a CA polypeptide sequence set forth herein. As for nucleic acids, homology in this context means sequence similarity or identity, with identity being preferred. This homology will be determined using standard techniques known in the art as are outlined above for the nucleic acid homologies.

CA proteins of the present invention may be shorter or longer than the wild type amino acid sequences. Thus, in a preferred embodiment, included within the definition of CA proteins are portions or fragments of the wild type sequences herein. In addition, as outlined above, the CA nucleic acids of the invention may be used to obtain additional coding regions, and thus additional protein sequence, using techniques known in the art.

In a preferred embodiment, the CA proteins are derivative or variant CA proteins as compared to the wild-type sequence. That is, as outlined more fully below, the derivative CA peptide will contain at least one amino acid substitution, deletion or insertion, with amino acid substitutions being particularly preferred. The amino acid substitution, insertion or deletion may occur at any residue within the CA peptide.

Also included in an embodiment of CA proteins of the present invention are amino acid sequence variants. These variants fall into one or more of three classes: substitutional, insertional or deletional variants. These variants ordinarily are prepared by site-specific mutagenesis of nucleotides in the DNA encoding the CA protein, using cassette or PCR mutagenesis or other techniques well known in the art, to produce DNA encoding the variant, and thereafter expressing the DNA in recombinant cell culture as outlined above. However, variant CA protein fragments having up to about 100-150 residues may be prepared by *in vitro* synthesis using established techniques. Amino acid sequence variants are characterized by the predetermined nature of the variation, a feature that sets them apart from naturally occurring allelic or interspecies variation of the CA protein amino acid sequence. The variants typically exhibit the same qualitative biological activity as the naturally occurring analogue, although variants can also be selected which have modified characteristics as will be more fully outlined below.

While the site or region for introducing an amino acid sequence variation is predetermined, the mutation per se need not be predetermined. For example, in order to optimize the performance of a mutation at a given site, random mutagenesis may be conducted at the target codon or region and the expressed CA variants screened for the optimal combination of desired activity. Techniques for making substitution mutations at predetermined sites in DNA having a known sequence are well known, for example, M13 primer mutagenesis and LAR mutagenesis. Screening of the mutants is done using assays of CA protein activities.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the CA protein are desired, substitutions are generally made in accordance with the following chart:

**Chart 1**

| Original Residue | Exemplary Substitutions |
| --- | --- |
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function or immunological identity are made by selecting substitutions that are less conservative than those shown in Chart I. For example, substitutions may be made full length to more significantly affect one or more of the following: the structure of the polypeptide backbone in the area of the alteration (e.g., the alpha-helical or beta-sheet structure); the charge or hydrophobicity of the molecule at the target site; and the bulk of the side chain. The substitutions which in general are expected to produce the greatest changes in the polypeptide's properties are those in which (a) a hydrophilic residue, e.g. seryl or threonyl is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

The variants typically exhibit the same qualitative biological activity and will elicit the same immune response as the naturally-occurring analogue, although variants also are selected to modify the characteristics of the CA proteins as needed. Alternatively, the variant may be designed such that the biological activity of the CA protein is altered. For example, glycosylation sites may be altered or removed, dominant negative mutations created, etc.

Covalent modifications of CA polypeptides are included within the scope of this invention, for example for use in screening. One type of covalent modification includes reacting targeted amino acid residues of a CA polypeptide with an organic derivatizing agent that is capable of reacting with selected side chains or the N-or C-terminal residues of a CA polypeptide. Derivatization with bifunctional agents is useful, for instance, for crosslinking CA polypeptides to a water-insoluble support matrix or surface for use in the method for purifying anti-CA antibodies or screening assays, as is more fully described below. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl, threonyl or tyrosyl residues, methylation of the a-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the CA polypeptide included within the scope of this invention comprises altering the native glycosylation pattern of the polypeptide. "Altering the native glycosylation pattern" is intended for purposes herein to mean deleting one or more carbohydrate moieties found in native sequence CA polypeptide, and/or adding one or more glycosylation sites that are not present in the native sequence CA polypeptide.

Addition of glycosylation sites to CA polypeptides may be accomplished by altering the amino acid sequence thereof. The alteration may be made, for example, by the addition of, or substitution by, one or more serine or threonine residues to the native sequence CA polypeptide (for O-linked glycosylation sites). The CA amino acid sequence may optionally be altered through changes at the DNA level, particularly by mutating the DNA encoding the CA polypeptide at preselected bases such that codons are generated that will translate into the desired amino acids.

Another means of increasing the number of carbohydrate moieties on the CA polypeptide is by chemical or enzymatic coupling of glycosides to the polypeptide. Such methods are described in the art, e.g., in WO 87/05330 published 11 September 1987, and in Aplin and Wriston, LA Crit. Rev. Biochem., pp. 259-306 (1981).

Removal of carbohydrate moieties present on the CA polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo-and exo-glycosidases as described by Thotakura et al., Meth. Enzymol., 138:350 (1987).

Another type of covalent modification of CA comprises linking the CA polypeptide to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

CA polypeptides of the present invention may also be modified in a way to form chimeric molecules comprising a CA polypeptide fused to another, heterologous polypeptide or amino acid sequence. In one embodiment, such a chimeric molecule comprises a fusion of a CA polypeptide with a tag polypeptide that provides an epitope to which an anti-tag antibody can selectively bind. The epitope tag is generally placed at the amino-or carboxyl-terminus of the CA polypeptide, although internal fusions may also be tolerated in some instances. The presence of such epitope-tagged forms of a CA polypeptide can be detected using an antibody against the tag polypeptide. Also, provision of the epitope tag enables the CA polypeptide to be readily purified by affinity purification using an anti-tag antibody or another type of affinity matrix that binds to the epitope tag. In an alternative embodiment, the chimeric molecule may comprise a fusion of a CA polypeptide with an immunoglobulin or a particular region of an immunoglobulin. For a bivalent form of the chimeric molecule, such a fusion could be to the Fc region of an IgG molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

Also included with the definition of CA protein in one embodiment are other CA proteins of the CA family, and CA proteins from other organisms, which are cloned and expressed as outlined below. Thus, probe or degenerate polymerase chain reaction (PCR) primer sequences may be used to find other related CA proteins from humans or other organisms. As will be appreciated by those in the art, particularly useful probe and/or PCR primer sequences include the unique areas of the CA nucleic acid sequence. As is generally known in the art, preferred PCR primers are from about 15 to about 35 nucleotides in length, with from about 20 to about 30 being preferred, and may contain inosine as needed. The conditions for the PCR reaction are well known in the art.

In addition, as is outlined herein, CA proteins can be made that are longer than those encoded by the nucleic acids of the figures, for example, by the elucidation of additional sequences, the addition of epitope or purification tags, the addition of other fusion sequences, etc.

CA proteins may also be identified as being encoded by CA nucleic acids. Thus, CA proteins are encoded by nucleic acids that will hybridize to the sequences of the sequence listings, or their complements, as outlined herein.

## CA antigens and antibodies thereto

In one embodiment, the invention provides CA specific antibodies. In a preferred embodiment, when the CA protein is to be used to generate antibodies, for example for immunotherapy, the CA protein should share at least one epitope or determinant with the full-length protein. By "epitope" or "determinant" herein is meant a portion of a protein that will generate and/or bind an antibody or T-cell receptor in the context of MHC. Thus, in most instances, antibodies made to a smaller CA protein will be able to bind to the full-length protein. In a preferred embodiment, the epitope is unique; that is, antibodies generated to a unique epitope show little or no cross-reactivity.

Any polypeptide sequence encoded by the CA polynucleotide sequences may be analyzed to determine certain preferred regions of the polypeptide. Regions of high antigenicity are determined from data by DNASTAR analysis by choosing values that represent regions of the polypeptide that are likely to be exposed on the surface of the polypeptide in an environment in which antigen recognition may occur in the process of initiation of an immune response. For example, the amino acid sequence of a polypeptide encoded by a CA polynucleotide sequence may be analyzed using the default parameters of the DNASTAR computer algorithm (DNASTAR, Inc., Madison, Wis.; http://www.dnastar.com/).

Polypeptide features that may be routinely obtained using the DNASTAR computer algorithm include, but are not limited to, Garnier-Robson alpha-regions, beta-regions, turn-regions, and coil-regions (Garnier et al. J. Mol. Biol., 120: 97 (1978)); Chou-Fasman alpha-regions, beta-regions, and turn-regions (Adv. in Enzymol., 47:45-148 (1978)); Kyte-Doolittle hydrophilic regions and hydrophobic regions (J. Mol. Biol., 157:105-132 (1982)); Eisenberg alpha- and beta-amphipathic regions; Karplus-Schulz flexible regions; Emini surface-forming regions (J. Virol., 55(3):836-839 (1985)); and Jameson-Wolf regions of high antigenic index (CABIOS, 4(1):181-186 (1988)). Kyte-Doolittle hydrophilic regions and hydrophobic regions, Emini surface-forming regions, and Jameson-Wolf regions of high antigenic index (i.e., containing four or more contiguous amino acids having an antigenic index of greater than or equal to 1.5, as identified using the default parameters of the Jameson-Wolf program) can routinely be used to determine polypeptide regions that exhibit a high degree of potential for antigenicity. One approach for preparing antibodies to a protein is the selection and preparation of an amino acid sequence of all or part of the protein, chemically synthesizing the sequence and injecting it into an appropriate animal, typically a rabbit, hamster or a mouse. Oligopeptides can be selected as candidates for the production of an antibody to the CA protein based upon the oligopeptides lying in hydrophilic regions, which are thus likely to be exposed in the mature protein. Additional oligopeptides can be determined using, for example, the Antigenicity Index, Welling, G. W. et al., FEBS Lett. 188:215-218 (1985), incorporated herein by reference.

In one embodiment, the term "antibody" includes antibody fragments, as are known in the art, including Fab, $Fab_2$, single chain antibodies (Fv for example), chimeric antibodies, etc., either produced by the modification of whole antibodies or those synthesized de novo using recombinant DNA technologies.

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include a protein encoded by a nucleic acid of the figures or fragment thereof or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants that may be employed include Freund's complete adjuvant and MPL-TDM adjuvant (monophosphoryl Lipid A, synthetic trehalose dicorynomycolate).

The immunization protocol may be selected by one skilled in the art without undue experimentation.

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.* The immunizing agent will typically include a polypeptide encoded by a nucleic acid of Tables 1-19, or fragment thereof or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Monoclonal antibody technology is used in implementing research, diagnosis and therapy. Monoclonal antibodies are used in radioimmunoassays, enzyme-linked immunosorbent assays, immunocytopathology, and flow cytometry for *in vitro* diagnosis, and *in vivo* for diagnosis and immmotherapy of human disease. Waldmann, T. A. (1991) Science 252:1657-1662. In particular, monoclonal antibodies have been widely applied to the diagnosis and therapy of cancer, wherein it is desirable to target malignant lesions while avoiding normal tissue. See, e.g., U.S. Pat. Nos. 4,753,894 to Frankel, et al.; 4,938,948 to Ring et al.; and 4,956,453 to Bjorn et al.

In one embodiment, the antibodies are bispecific antibodies. Bispecific antibodies are monoclonal, preferably human or humanized, antibodies that have binding specificities for at least two different antigens. A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and.Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients. In the present case, one of the binding specificities is for a protein encoded by a nucleic acid of Tables 1-19, or a fragment thereof, the other one is for any other antigen, and preferably for a cell-surface protein or receptor or receptor subunit, preferably one that is tumor specific.

In a preferred embodiment, the antibodies to CA are capable of reducing or eliminating the biological function of CA, as is described below. That is, the addition of anti-CA antibodies (either polyclonal or preferably monoclonal) to CA (or cells containing CA) may reduce or eliminate the CA activity. Generally, at least a 25% decrease in activity is preferred, with at least about 50% being particularly preferred and about a 95-100% decrease being especially preferred.

In a preferred embodiment the antibodies to the CA proteins are humanized antibodies. "Humanized" antibodies refer to a molecule having an antigen binding site that is substantially derived from an immunoglobulin from a non-human species and the remaining immunoglobulin structure of the molecule based upon the structure and/or sequence of a human immunoglobulin. The antigen binding site may comprise either complete variable domains fused onto constant domains or only the complementarity determining regions (CDRs) grafted onto appropriate framework regions in the variable domains. Antigen binding sites may be wild type or modified by one or more amino acid substitutions, e.g., modified to resemble human immunoglobulin more closely. Alternatively, a humanized antibody may be derived from a chimeric antibody that retains or substantially retains the antigen-binding properties of the parental, non-human, antibody but which exhibits diminished immunogenicity as compared to the parental antibody when administered to humans. The phrase "chimeric antibody," as used herein, refers to an antibody containing sequence derived from two different antibodies (*see, e.g.*, U.S. Patent No. 4,816,567) that typically originate from different species. Typically, in these chimeric antibodies, the variable region of both light and heavy chains mimics the variable regions of antibodies derived from one species of mammals, while the constant portions are homologous to the sequences in antibodies derived from another. Most typically, chimeric antibodies comprise human and murine antibody fragments, generally human constant and mouse variable regions. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues.

Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework residues (FR) regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature, 332:323-329 (1988); and Presta, Curr. Op. Struct. Biol., 2:593-596 (1992)). One clear advantage to such chimeric forms is that, for example, the variable regions can conveniently be derived from presently known sources using readily available hybridomas or B cells from non human host organisms in combination with constant regions derived from, for example, human cell preparations. While the variable region has the advantage of ease of preparation, and the specificity is not affected by its source, the constant region being human, is less likely to elicit an immune response from a human subject when the antibodies are injected than would the constant region from a non-human source. However, the definition is not limited to this particular example.

Because humanized antibodies are far less immunogenic in humans than the parental mouse monoclonal antibodies, they can be used for the treatment of humans with far less risk of anaphylaxis. Thus, these antibodies may be preferred in therapeutic applications that involve *in vivo* administration to a human such as, *e.g.,* use as radiation sensitizers for the treatment of neoplastic disease or use in methods to reduce the side effects of, *e.g.*, cancer therapy. Methods for humanizing non-human antibodies are well known in the art. Generally, a humanized antibody has one or more amino acid residues introduced into it from a source that is non-human. These non-human amino acid residues are often referred to as import residues, which are typically taken from an import variable domain. Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies [Cole et al., Monoclonal Antibodies and Cancer Therapy, Alan R. Liss, p. 77 (1985) and Boerner et al., J. Immunol., 147(1):86-95 (1991)]. Humanized antibodies may be achieved by a variety of methods including, for example: (1) grafting the non-human complementarity determining regions (CDRs) onto a human framework and constant region (a process referred to in the art as "humanizing"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995); Jones et al., Nature 321:522-525 (1986); Morrison et al., Proc. Natl. Acad. Sci, US.A., 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); Padlan, Molec. Immunol. 31(3):169-217 (1994); and Kettleborough, C.A. et al., Protein Eng. 4(7):773-83 (1991) each of which is incorporated herein by reference.

The phrase "complementarity determining region" refers to amino acid sequences which together define the binding affinity and specificity of the natural Fv region of a native immunoglobulin binding site. *See, e.g.*, Chothia et al., J. Mol. Biol. 196:901-917 (1987); Kabat et al., U.S. Dept. of Health and Human Services NIH Publication No. 91-3242 (1991). The phrase "constant region" refers to the portion of the antibody molecule that confers effector functions. In the present invention, mouse constant regions are substituted by human constant regions. The constant regions of the subject humanized antibodies are derived from human immunoglobulins. The heavy chain constant region can be selected from any of the five isotypes: alpha, delta, epsilon, gamma or mu. One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region that disturb the structure of the CDRs until the predicted conformation of the humanized sequence model closely approximates the conformation of the non-human CDRs of the parent non-human antibody. Such humanized antibodies may be further derivatized to facilitate uptake and clearance, *e.g*, via Ashwell receptors. *See, e.g.*, U.S. Patent Nos. 5,530,101 and 5,585,089 which are incorporated herein by reference.

Humanized antibodies to CA polypeptides can also be produced using transgenic animals that are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions.

Using a transgenic animal described above, an immune response can be produced to a selected antigenic molecule, and antibody-producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. The monoclonal antibodies can be tested for the ability to inhibit or neutralize the biological activity or physiological effect of the corresponding protein.

In the present invention, CA polypeptides of the invention and variants thereof are used to immunize a transgenic animal as described above. Monoclonal antibodies are made using methods known in the art, and the specificity of the antibodies is tested using isolated CA polypeptides. Methods for preparation of the human or primate CA or an epitope thereof include, but are not limited to chemical synthesis, recombinant DNA techniques or isolation from biological samples. Chemical synthesis of a peptide can be performed, for example, by the classical Merrifeld method of solid phase peptide synthesis (Merrifeld, J. Am. Chem. Soc. 85:2149, 1963 which is incorporated by reference) or the FMOC strategy on a Rapid Automated Multiple Peptide Synthesis system (E. I. du Pont de Nemours Company, Wilmington, DE) (Caprino and Han, J. Org. Chem. 37:3404, 1972 which is incorporated by reference).

Polyclonal antibodies can be prepared by immunizing rabbits or other animals by injecting antigen followed by subsequent boosts at appropriate intervals. The animals are bled and sera assayed against purified CA proteins usually by ELISA or by bioassay based upon the ability to block the action of CA proteins. When using avian species, e.g., chicken, turkey and the like, the antibody can be isolated from the yolk of the egg. Monoclonal antibodies can be prepared after the method of Milstein and Kohler by fusing splenocytes from immunized mice with continuously replicating tumor cells such as myeloma or lymphoma cells. (Milstein and Kohler, Nature 256:495-497, 1975; Gulfre and Milstein, Methods in Enzymology: Immunochemical Techniques 73:1-46, Langone and Banatis eds., Academic Press, 1981 which are incorporated by reference). The hybridoma cells so formed are then cloned by limiting dilution methods and supernates assayed for antibody production by ELISA, RIA or bioassay.

The unique ability of antibodies to recognize and specifically bind to target proteins provides an approach for treating an overexpression of the protein. Thus, another aspect of the present invention provides for a method for preventing or treating diseases involving overexpression of a CA polypeptide by treatment of a patient with specific antibodies to the CA protein.

Specific antibodies, either polyclonal or monoclonal, to the CA proteins can be produced by any suitable method known in the art as discussed above. For example, murine or human monoclonal antibodies can be produced by hybridoma technology or, alternatively, the CA proteins, or an immunologically active fragment thereof, or an anti-idiotypic antibody, or fragment thereof can be administered to an animal to elicit the production of antibodies capable of recognizing and binding to the CA proteins. Such antibodies can be from any class of antibodies including, but not limited to IgG, IgA, IgM, IgD, and IgE or in the case of avian species, IgY and from any subclass of antibodies.

By immunotherapy is meant treatment of a cancer with an antibody raised against a CA protein. As used herein, immunotherapy can be passive or active. Passive immunotherapy as defined herein is the passive transfer of antibody to a recipient (patient). Active immunization is the induction of antibody and/or T-cell responses in a recipient (patient). Induction of an immune response is the result of providing the recipient with an antigen to which antibodies are raised. As appreciated by one of ordinary skill in the art, the antigen may be provided by injecting a polypeptide against which antibodies are desired to be raised into a recipient, or contacting the recipient with a nucleic acid capable of expressing the antigen and under conditions for expression of the antigen.

In a preferred embodiment, oncogenes which encode secreted growth factors may be inhibited by raising antibodies against CA proteins that are secreted proteins as described above. Without being bound by theory, antibodies used for treatment, bind and prevent the secreted protein from binding to its receptor, thereby inactivating the secreted CA protein.

In another preferred embodiment, the CA protein to which antibodies are raised is a transmembrane protein. Without being bound by theory, antibodies used for treatment, bind the extracellular domain of the CA protein and prevent it from binding to other proteins, such as circulating ligands or cell-associated molecules. The antibody may cause down-regulation of the transmembrane CA protein. As will be appreciated by one of ordinary skill in the art, the antibody may be a competitive, non-competitive or uncompetitive inhibitor of protein binding to the extracellular domain of the CA protein. The antibody is also an antagonist of the CA protein. Further, the antibody prevents activation of the transmembrane CA protein. In one aspect, when the antibody prevents the binding of other molecules to the CA protein, the antibody prevents growth of the cell. The antibody may also sensitize the cell to cytotoxic agents, including, but not limited to TNF-$\alpha$, TNF-$\beta$, IL-1, INF-$\gamma$ and IL-2, or chemotherapeutic agents including 5FU, vinblastine, actinomycin D, cisplatin, methotrexate, and the like. In some instances the antibody belongs to a sub-type that activates serum complement when complexed with the transmembrane protein thereby mediating cytotoxicity. Thus, cancers may be treated by administering to a patient antibodies directed against the transmembrane CA protein.

In another preferred embodiment, the antibody is conjugated to a therapeutic moiety. In one aspect the therapeutic moiety is a small molecule that modulates the activity of the CA protein. In another aspect the therapeutic moiety modulates the activity of molecules associated with or in close proximity to the CA protein. The therapeutic moiety may inhibit enzymatic activity such as protease or protein kinase activity associated with cancer.

In a preferred embodiment, the therapeutic moiety may also be a cytotoxic agent. In this method, radioisotopes, natural toxins, chemotherapy agents, or other substances (such as biological response modifiers) are chemically linked or conjugated to a monoclonal antibody to form "immunoconjugates" and "immunotoxins" which target the cytotoxic agent to tumor tissue or cells resulting in a reduction in the number of afflicted cells, thereby reducing symptoms associated with cancers, including lymphoma. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin and the like. Cytotoxic agents also include radiochemicals made by conjugating radioisotopes to antibodies raised against CA proteins, or binding of a radionuclide to a chelating agent that has been covalently attached to the antibody. Targeting the therapeutic moiety to transmembrane CA proteins not only serves to increase the local concentration of therapeutic moiety in the cancer of interest, i.e., lymphoma, but also serves to reduce deleterious side effects that may be associated with the therapeutic moiety. A number of investigators have used monoclonal antibodies as carriers of cytotoxic substances in attempts to selectively direct those agents to malignant tissue. More particularly, a number of monoclonal antibodies have been conjugated to toxins such as ricin, abrin, diphtheria toxin and Pseudomonas exotoxin or to enzymatically active portions (A chains) thereof via heterobifunctional agents. See, e.g., U.S. Pat. No. 4,753,894 to Frankel et al.; Nevelle, et al. (1982) Immunol Rev 62:75-91; Ross et al. (1980) Eur. J Biochem 104; Vitteta et al. (1982) Immunol Rev 62:158-183; Raso et al. (1982) Cancer Res 42:457-464, and Trowbridge et al. (1981) Nature 294:171-173.

In another preferred embodiment, the CA protein against which the antibodies are raised is an intracellular protein. In this case, the antibody may be conjugated to a protein that facilitates entry into the cell. In one case, the antibody enters the cell by endocytosis. In another embodiment, a nucleic acid encoding the antibody is administered to the individual or cell. Moreover, wherein the CA protein can be targeted within a cell, e.g., the nucleus, an antibody thereto contains a signal for that target localization, e.g., a nuclear localization signal.

The CA antibodies of the invention specifically bind to CA proteins. By "specifically bind" herein is meant that the antibodies bind to the protein with a binding constant in the range of $10^{-4}$-$10^{-6}$ M$^{-1}$, with a preferred range being $10^{-7}$-$10^{-9}$ M$^{-1}$.

In a preferred embodiment, the CA protein is purified or isolated after expression. CA proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the CA protein may be purified using a standard anti-CA antibody column. Ultrafiltration and diafiltration techniques, in conjunction with protein concentration, are also useful. For general guidance in suitable purification techniques, see Scopes, R., Protein Purification, Springer-Verlag, NY (1982). The degree of purification necessary will vary depending on the use of the CA protein. In some instances no purification will be necessary.

## Detection of cancer phenotype

Once expressed and purified if necessary, the CA proteins and nucleic acids are useful in a number of applications. In one aspect, the expression levels of genes are determined for different cellular states in the cancer phenotype; that is, the expression levels of genes in normal tissue and in cancer tissue (and in some cases, for varying severities of lymphoma that relate to prognosis, as outlined below) are evaluated to provide expression profiles. An expression profile of a particular cell state or point of development is essentially a "fingerprint" of the state; while two states may have any particular gene similarly expressed, the evaluation of a number of genes simultaneously allows the generation of a gene expression profile that is unique to the state of the cell. By comparing expression profiles of cells in different states, information regarding which genes are important (including both up- and down-regulation of genes) in each of these states is obtained. Then, diagnosis may be done or confirmed: does tissue from a particular patient have the gene expression profile of normal or cancer tissue.

"Differential expression," or equivalents used herein, refers to both qualitative as well as quantitative differences in the temporal and/or cellular expression patterns of genes, within and among the cells. Thus, a differentially expressed gene can qualitatively have its expression altered, including an activation or inactivation, in, for example, normal versus cancer tissue. That is, genes may be turned on or turned off in a particular state, relative to another state. As is apparent to the skilled artisan, any comparison of two or more states can be made. Such a qualitatively regulated gene will exhibit an expression pattern within a state or cell type which is detectable by standard techniques in one such state or cell type, but is not detectable in both. Alternatively, the determination is quantitative in that expression is increased or decreased; that is, the expression ofthe gene is either up-regulated, resulting in an increased amount of transcript, or down-regulated, resulting in a decreased amount of transcript. The degree to which expression differs need only be large enough to quantify via standard characterization techniques as outlined below, such as by use of Affymetrix GeneChip® expression arrays, Lockhart, Nature Biotechnology, 14:1675-1680 (1996), hereby expressly incorporated by reference. Other techniques include, but are not limited to, quantitative reverse transcriptase PCR, Northern analysis and RNase protection. As outlined above, preferably the change in expression (i.e. upregulation or downregulation) is at least about 50%, more preferably at least about 100%, more preferably at least about 150%, more preferably, at least about 200%, with from 300 to at least 1000% being especially preferred.

As will be appreciated by those in the art, this may be done by evaluation at either the gene transcript, or the protein level; that is, the amount of gene expression may be monitored using nucleic acid probes to the DNA or RNA equivalent of the gene transcript, and, the quantification of gene expression levels, or, alternatively, the final gene product itself (protein) can be monitored, for example through the use of antibodies to the CA protein and standard immunoassays (ELISAs, etc.) or other techniques, including mass spectroscopy assays, 2D gel electrophoresis assays, etc. Thus, the proteins corresponding to CA genes, i.e. those identified as being important in a particular cancer phenotype, i.e., lymphoma, can be evaluated in a diagnostic test specific for that cancer.

In a preferred embodiment, gene expression monitoring is done and a number of genes, i.e. an expression profile, is monitored simultaneously, although multiple protein expression monitoring can be done as well. Similarly, these assays may be done on an individual basis as well.

In this embodiment, the CA nucleic acid probes may be attached to biochips as outlined herein for the detection and quantification of CA sequences in a particular cell. The assays are done as is known in the art. As will be appreciated by those in the art, any number of different CA sequences may be used as probes, with single sequence assays being used in some cases, and a plurality of the sequences described herein being used in other embodiments. In addition, while solid-phase assays are described, any number of solution based assays may be done as well.

In a preferred embodiment, both solid and solution based assays may be used to detect CA sequences that are up-regulated or down-regulated in cancers as compared to normal tissue. In instances where the CA sequence has been altered but shows the same expression profile or an altered expression profile, the protein will be detected as outlined herein.

In a preferred embodiment nucleic acids encoding the CA protein are detected. Although DNA or RNA encoding the CA protein may be detected, of particular interest are methods wherein the mRNA encoding a CA protein is detected. The presence of mRNA in a sample is an indication that the CA gene has been transcribed to form the mRNA, and suggests that the protein is expressed. Probes to detect the mRNA can be any nucleotide/deoxynucleotide probe that is complementary to and base pairs with the mRNA and includes but is not limited to oligonucleotides, cDNA or RNA. Probes also should contain a detectable label, as defined herein. In one method the mRNA is detected after immobilizing the nucleic acid to be examined on a solid support such as nylon membranes and hybridizing the probe with the sample. Following washing to remove the non-specifically bound probe, the label is detected. In another method detection of the mRNA is performed *in situ*. In this method permeabilized cells or tissue samples are contacted with a detectably labeled nucleic acid probe for sufficient time to allow the probe to hybridize with the target mRNA. Following washing to remove the non-specifically bound probe, the label is detected. For example a digoxygenin labeled riboprobe (RNA probe) that is complementary to the mRNA encoding a CA protein is detected by binding the digoxygenin with an anti-digoxygenin secondary antibody and developed with nitro blue tetrazolium and 5-bromo-4-chloro-3-indoyl phosphate.

In a preferred embodiment, any of the three classes of proteins as described herein (secreted, transmembrane or intracellular proteins) are used in diagnostic assays. The CA proteins, antibodies, nucleic acids, modified proteins and cells containing CA sequences are used in diagnostic assays. This can be done on an individual gene or corresponding polypeptide level, or as sets of assays.

As described and defmed herein, CA proteins find use as markers of cancers, including lymphomas such as, but not limited to, Hodgkin's and non-Hodgkin's lymphoma. Detection of these proteins in putative cancer tissue or patients allows for a determination or diagnosis of the type of cancer. Numerous methods known to those of ordinary skill in the art find use in detecting cancers. In one embodiment, antibodies are used to detect CA proteins. A preferred method separates proteins from a sample or patient by electrophoresis on a gel (typically a denaturing and reducing protein gel, but may be any other type of gel including isoelectric focusing gels and the like). Following separation of proteins, the CA protein is detected by immunoblotting with antibodies raised against the CA protein. Methods of immunoblotting are well known to those of ordinary skill in the art.

In another preferred method, antibodies to the CA protein find use in *in situ* imaging techniques. In this method cells are contacted with from one to many antibodies to the CA protein(s). Following washing to remove non-specific antibody binding, the presence of the antibody or antibodies is detected. In one embodiment the antibody is detected by incubating with a secondary antibody that contains a detectable label. In another method the primary antibody to the CA protein(s) contains a detectable label. In another preferred embodiment each one of multiple primary antibodies contains a distinct and detectable label. This method finds particular use in simultaneous screening for a plurality of CA proteins. As will be appreciated by one of ordinary skill in the art, numerous other histological imaging techniques are useful in the invention.

In a preferred embodiment the label is detected in a fluorometer that has the ability to detect and distinguish emissions of different wavelengths. In addition, a fluorescence activated cell sorter (FACS) can be used in the method.

In another preferred embodiment, antibodies find use in diagnosing cancers from blood samples. As previously described, certain CA proteins are secreted/circulating molecules. Blood samples, therefore, are useful as samples to be probed or tested for the presence of secreted CA proteins. Antibodies can be used to detect the CA proteins by any of the previously described immunoassay techniques including ELISA, immunoblotting (Western blotting), immunoprecipitation, BIACORE technology and the like, as will be appreciated by one of ordinary skill in the art.

In a preferred embodiment, *in situ* hybridization of labeled CA nucleic acid probes to tissue arrays is done. For example, arrays of tissue samples, including CA tissue and/or normal tissue, are made. *In situ* hybridization as is known in the art can then be done.

It is understood that when comparing the expression fingerprints between an individual and a standard, the skilled artisan can make a diagnosis as well as a prognosis. It is further understood that the genes that indicate diagnosis may differ from those that indicate prognosis.

In a preferred embodiment, the CA proteins, antibodies, nucleic acids, modified proteins and cells containing CA sequences are used in prognosis assays. As above, gene expression profiles can be generated that correlate to cancer, especially lymphoma, severity, in terms of long term prognosis. Again, this may be done on either a protein or gene level, with the use of genes being preferred. As above, the CA probes are attached to biochips for the detection and quantification of CA sequences in a tissue or patient. The assays proceed as outlined for diagnosis.

**Screening for CA-Targeted Drugs**

In one embodiment, any of the CA sequences as described herein are used in drug screening assays. The CA proteins, antibodies, nucleic acids, modified proteins and cells containing CA sequences are used in drug screening assays or by evaluating the effect of drug candidates on a "gene expression profile" or expression profile of polypeptide. In one embodiment, the expression profiles are used, preferably in conjunction with high throughput screening techniques to allow monitoring for expression profile genes after treatment with a candidate agent, Zlokarnik, et al., Science 279, 84-8 (1998), Heid, et al., Genome Res., 6:986-994 (1996).

In another embodiment, the CA proteins, antibodies, nucleic acids, modified proteins and cells containing the native or modified CA proteins are used in screening assays. That is, the present invention provides novel methods for screening for compositions that modulate the cancer phenotype. As above, this can be done by screening for modulators of gene expression or for modulators of protein activity. Similarly, this may be done on an individual gene or protein level or by evaluating the effect of drug candidates on a "gene expression profile". In a preferred embodiment, the expression profiles are used, preferably in conjunction with high throughput screening techniques to allow monitoring for expression profile genes after treatment with a candidate agent, see Zlokarnik, supra.

Having identified the CA genes herein, a variety of assays to evaluate the effects of agents on gene expression may be executed. In a preferred embodiment, assays may be run on an individual gene or protein level. That is, having identified a particular gene as aberrantly regulated in cancer, candidate bioactive agents may be screened to modulate the gene's regulation. "Modulation" thus includes both an increase and a decrease in gene expression or activity. The preferred amount of modulation will depend on the original change of the gene expression in normal versus tumor tissue, with changes of at least 10%, preferably 50%, more preferably 100-300%, and in some embodiments 300-1000% or greater. Thus, if a gene exhibits a 4 fold increase in tumor compared to normal tissue, a decrease of about four fold is desired; a 10 fold decrease in tumor compared to normal tissue gives a 10 fold increase in expression for a candidate agent is desired, etc. Alternatively, where the CA sequence has been altered but shows the same expression profile or an altered expression profile, the protein will be detected as outlined herein.

As will be appreciated by those in the art, this may be done by evaluation at either the gene or the protein level;

that is, the amount of gene expression may be monitored using nucleic acid probes and the quantification of gene expression levels, or, alternatively, the level of the gene product itself can be monitored, for example through the use of antibodies to the CA protein and standard immunoassays. Alternatively, binding and bioactivity assays with the protein may be done as outlined below.

In a preferred embodiment, gene expression monitoring is done and a number of genes, i.e. an expression profile, is monitored simultaneously, although multiple protein expression monitoring can be done as well.

In this embodiment, the CA nucleic acid probes are attached to biochips as outlined herein for the detection and quantification of CA sequences in a particular cell. The assays are further described below.

Generally, in a preferred embodiment, a candidate bioactive agent is added to the cells prior to analysis. Moreover, screens are provided to identify a candidate bioactive agent that modulates a particular type of cancer, modulates CA proteins, binds to a CA protein, or interferes between the binding of a CA protein and an antibody.

The term "candidate bioactive agent" or "drug candidate" or grammatical equivalents as used herein describes any molecule, e.g., protein, oligopeptide, small organic or inorganic molecule, polysaccharide, polynucleotide, etc., to be tested for bioactive agents that are capable of directly or indirectly altering either the cancer phenotype, binding to and/or modulating the bioactivity of a CA protein, or the expression of a CA sequence, including both nucleic acid sequences and protein sequences. In a particularly preferred embodiment, the candidate agent suppresses a CA phenotype, for example to a normal tissue fingerprint. Similarly, the candidate agent preferably suppresses a severe CA phenotype. Generally a plurality of assay mixtures are run in parallel with different agent concentrations to obtain a differential response to the various concentrations. Typically, one of these concentrations serves as a negative control, i.e., at zero concentration or below the level of detection.

In one aspect, a candidate agent will neutralize the effect of a CA protein. By "neutralize" is meant that activity of a protein is either inhibited or counter acted against so as to have substantially no effect on a cell.

Candidate agents encompass numerous chemical classes, though typically they are organic or inorganic molecules, preferably small organic compounds having a molecular weight of more than 100 and less than about 2,500 Daltons. Preferred small molecules are less than 2000, or less than 1500 or less than 1000 or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

Candidate agents are obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification, or amidification to produce structural analogs.

In one embodiment, the candidate bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard in vivo degradations.

In a preferred embodiment, the candidate bioactive agents are naturally occurring proteins or fragments of naturally occurring proteins. Thus, for example, cellular extracts containing proteins, or random or directed digests of proteinaceous cellular extracts, may be used. In this way libraries of prokaryotic and eukaryotic proteins may be made for screening in the methods of the invention. Particularly preferred in this embodiment are libraries of bacterial, fungal, viral, and mammalian proteins, with the latter being preferred, and human proteins being especially preferred.

I n another preferred embodiment, the candidate bioactive agents are peptides of from about 5 to about 30 amino acids, with from about 5 to about 20 amino acids being preferred, and from about 7 to about 15 being particularly preferred. The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position. In a preferred embodiment, the library is biased. That is, some positions within the sequence are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc., or to purines, etc.

In one embodiment, the candidate bioactive agents are nucleic acids. As described generally for proteins, nucleic acid candidate bioactive agents may be naturally occurring nucleic acids, random nucleic acids, or "biased" random nucleic acids. In another embodiment, the candidate bioactive agents are organic chemical moieties, a wide variety of which are available in the literature.

In assays for testing alteration of the expression profile of one or more CA genes, after the candidate agent has been added and the cells allowed to incubate for some period of time, a nucleic acid sample containing the target sequences to be analyzed is prepared. The target sequence is prepared using known techniques (e.g., converted from RNA to labeled cDNA, as described above) and added to a suitable microarray. For example, an *in vitro* reverse transcription with labels covalently attached to the nucleosides is performed. Generally, the nucleic acids are labeled with a label as defined herein, especially with biotin-FITC or PE, Cy3 and Cy5.

As will be appreciated by those in the art, these assays can be direct hybridization assays or can comprise "sandwich assays", which include the use of multiple probes, as is generally outlined in U.S. Patent Nos. 5,681,702, 5,597,909, 5,545,730, 5,594,117, 5,591,584, 5,571,670, 5,580,731, 5,571,670, 5,591,584, 5,624,802, 5,635,352, 5,594,118, 5,359,100, 5,124,246 and 5,681,697, all of which are hereby incorporated by reference. In this embodiment, in general, the target nucleic acid is prepared as outlined above, and then added to the biochip comprising a plurality of nucleic acid probes, under conditions that allow the formation of a hybridization complex.

A variety of hybridization conditions may be used in the present invention, including high, moderate and low stringency conditions as outlined above. The assays are generally run under stringency conditions that allow formation of the label probe hybridization complex only in the presence of target. Stringency can be controlled by altering a step parameter that is a thermodynamic variable, including, but not limited to, temperature, formamide concentration, salt concentration, chaotropic salt concentration, pH, organic solvent concentration, etc. These parameters may also be used to control non-specific binding, as is generally outlined in U.S. Patent No. 5,681,697. Thus it may be desirable to perform certain steps at higher stringency conditions to reduce non-specific binding.

The reactions outlined herein may be accomplished in a variety of ways, as will be appreciated by those in the art. Components of the reaction may be added simultaneously, or sequentially, in any order, with preferred embodiments outlined below. In addition, the reaction may include a variety of other reagents in the assays. These include reagents like salts, buffers, neutral proteins, e.g. albumin, detergents, etc which may be used to facilitate optimal hybridization and detection, and/or reduce non-specific or background interactions. Also reagents that otherwise, improve the efficiency of the assay, such as protease inhibitors, nuclease inhibitors, anti-microbial agents, etc., may be used, depending on the sample preparation methods and purity of the target. In addition, either solid phase or solution based (i.e., kinetic PCR) assays may be used.

Once the assay is run, the data are analyzed to determine the expression levels, and changes in expression levels as between states, of individual genes, forming a gene expression profile.

In a preferred embodiment, as for the diagnosis and prognosis applications, having identified the differentially expressed gene(s) or mutated gene(s) important in any one state, screens can be run to test for alteration of the expression of the CA genes individually. That is, screening for modulation of regulation of expression of a single gene can be done. Thus, for example, in the case of target genes whose presence or absence is unique between two states, screening is done for modulators of the target gene expression.

In addition, screens can be done for novel genes that are induced in response to a candidate agent. After identifying a candidate agent based upon its ability to suppress a CA expression pattern leading to a normal expression pattern, or modulate a single CA gene expression profile so as to mimic the expression of the gene from normal tissue, a screen as described above can be performed to identify genes that are specifically modulated in response to the agent. Comparing expression profiles between normal tissue and agent treated CA tissue reveals genes that are not expressed in normal tissue or CA tissue, but are expressed in agent treated tissue. These agent specific sequences can be identified and used by any of the methods described herein for CA genes or proteins. In particular these sequences and the proteins they encode find use in marking or identifying agent-treated cells. In addition, antibodies can be raised against the agent-induced proteins and used to target novel therapeutics to the treated CA tissue sample.

Thus, in one embodiment, a candidate agent is administered to a population of CA cells, that thus has an associated CA expression profile. By "administration" or "contacting" herein is meant that the candidate agent is added to the cells in such a manner as to allow the agent to act upon the cell, whether by uptake and intracellular action, or by action at the cell surface. In some embodiments, nucleic acid encoding a proteinaceous candidate agent (i.e. a peptide) may be put into a viral construct such as a retroviral construct and added to the cell, such that expression of the peptide agent is accomplished; see PCT US97/01019, hereby expressly incorporated by reference.

Once the candidate agent has been administered to the cells, the cells can be washed if desired and are allowed to incubate under preferably physiological conditions for some period of time. The cells are then harvested and a new gene expression profile is generated, as outlined herein.

Thus, for example, CA tissue may be screened for agents that reduce or suppress the CA phenotype. A change in at least one gene of the expression profile indicates that the agent has an effect on CA activity. By defining such a signature for the CA phenotype, screens for new drugs that alter the phenotype can be devised. With this approach, the drug target need not be known and need not be represented in the original expression screening platform, nor does the level of transcript for the target protein need to change.

In a preferred embodiment, as outlined above, screens may be done on individual genes and gene products (proteins). That is, having identified a particular differentially expressed gene as important in a particular state, screening of modulators of either the expression of the gene or the gene product itself can be done. The gene products of differentially expressed genes are sometimes referred to herein as "CA proteins" or "CAP". The CAP may be a fragment, or alternatively, be the full-length protein to the fragment encoded by the nucleic acids of Tables 1-19 (human genomic sequences of SEQ ID NOS: 4, 10, 16, 22, 28, 34, 40, 46, 52, 58, 66, 72, 80, 86, 98, 104, 110, 132, and 138, and sequences of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 corresponding to the human mRNAs generated therefrom). In a preferred embodiment, the CAP is selected from the human protein sequences shown in Tables 1-19 (of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146). In another embodiment, the sequences are sequence variants as further described herein.

Preferably, the CAP is a fragment approximately 14 to 24 amino acids in length. More preferably the fragment is a soluble fragment. Preferably, the fragment includes a non-transmembrane region. In a preferred embodiment, the fragment has an N-terminal Cys to aid in solubility. In one embodiment, the C-terminus of the fragment is kept as a free acid and the N-terminus is a free amine to aid in coupling, e.g., to a cysteine.

In one embodiment the CA proteins are conjugated to an immunogenic agent as discussed herein. In one embodiment the CA protein is conjugated to BSA.

In a preferred embodiment, screening is done to alter the biological function of the expression product of the CA gene. Again, having identified the importance of a gene in a particular state, screening for agents that bind and/or modulate the biological activity of the gene product can be run as is more fully outlined below.

In a preferred embodiment, screens are designed to first find candidate agents that can bind to CA proteins, and then these agents may be used in assays that evaluate the ability of the candidate agent to modulate the CAP activity and the cancer phenotype. Thus, as will be appreciated by those in the art, there are a number of different assays that may be run; binding assays and activity assays.

In a preferred embodiment, binding assays are done. In general, purified or isolated gene product is used; that is, the gene products of one or more CA nucleic acids are made. In general, this is done as is known in the art. For example, antibodies are generated to the protein gene products, and standard immunoassays are run to determine the amount of protein present. Alternatively, cells comprising the CA proteins can be used in the assays.

Thus, in a preferred embodiment, the methods comprise combining a CA protein and a candidate bioactive agent, and determining the binding of the candidate agent to the CA protein. Preferred embodiments utilize the human or mouse CA protein, although other mammalian proteins may also be used, for example for the development of animal models of human disease. In some embodiments, as outlined herein, variant or derivative CA proteins may be used.

Generally, in a preferred embodiment of the methods herein, the CA protein or the candidate agent is non-diffusably bound to an insoluble support having isolated sample receiving areas (e.g. a microtiter plate, an array, etc.). The insoluble support may be made of any composition to which the compositions can be bound, is readily separated from soluble material, and is otherwise compatible with the overall method of screening. The surface of such supports may be solid or porous and of any convenient shape. Examples of suitable insoluble supports include microtiter plates, arrays, membranes and beads. These are typically made of glass, plastic (e.g., polystyrene), polysaccharides, nylon or nitrocellulose, Teflon®, etc. Microtiter plates and arrays are especially convenient because a large number of assays can be carried out simultaneously, using small amounts of reagents and samples.

The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of the protein or agent, excess unbound material is removed by washing. The sample receiving areas may then be blocked through incubation with bovine serum albumin (BSA), casein or other innocuous protein or other moiety.

In a preferred embodiment, the CA protein is bound to the support, and a candidate bioactive agent is added to the assay. Alternatively, the candidate agent is bound to the support and the CA protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled *in vitro* protein-protein binding assays, electrophoretic mobility shift assays, immunoassays for protein binding, functional assays (phosphorylation assays, etc.) and the like.

The determination of the binding of the candidate bioactive agent to the CA protein may be done in a number of ways. In a preferred embodiment, the candidate bioactive agent is labeled, and binding determined directly. For example, this may be done by attaching all or a portion of the CA protein to a solid support, adding a labeled candidate agent (for example a fluorescent label), washing off excess reagent, and determining whether the label is present on the solid support. Various blocking and washing steps may be utilized as is known in the art.

By "labeled" herein is meant that the compound is either directly or indirectly labeled with a label which provides a detectable signal, e.g. radioisotope, fluorescers, enzyme, antibodies, particles such as magnetic particles, chemiluminescers, or specific binding molecules, etc. Specific binding molecules include pairs, such as biotin and streptavidin, digoxin and antidigoxin etc. For the specific binding members, the complementary member would normally be labeled with a molecule which provides for detection, in accordance with known procedures, as outlined above. The label can directly or indirectly provide a detectable signal.

In some embodiments, only one of the components is labeled. For example, the proteins (or proteinaceous candidate agents) may be labeled at tyrosine positions using $^{125}I$, or with fluorophores. Alternatively, more than one component may be labeled with different labels; using $^{125}I$ for the proteins, for example, and a fluorophore for the candidate agents.

In a preferred embodiment, the binding of the candidate bioactive agent is determined through the use of competitive binding assays. In this embodiment, the competitor is a binding moiety known to bind to the target molecule (i.e. CA protein), such as an antibody, peptide, binding partner, ligand, etc. Under certain circumstances, there may be competitive binding as between the bioactive agent and the binding moiety, with the binding moiety displacing the bioactive agent.

In one embodiment, the candidate bioactive agent is labeled. Either the candidate bioactive agent, or the competitor, or both, is added first to the protein for a time sufficient to allow binding, if present. Incubations may be performed at any temperature which facilitates optimal activity, typically between 4 and 40° C. Incubation periods are selected for optimum activity, but may also be optimized to facilitate rapid high throughput screening. Typically between 0.1 and 1 hour will be sufficient. Excess reagent is generally removed or washed away. The second component is then added, and the presence or absence of the labeled component is followed, to indicate binding.

In a preferred embodiment, the competitor is added first, followed by the candidate bioactive agent. Displacement of the competitor is an indication that the candidate bioactive agent is binding to the CA protein and thus is capable of binding to, and potentially modulating, the activity of the CA protein. In this embodiment, either component can be labeled. Thus, for example, if the competitor is labeled, the presence of label in the wash solution indicates displacement by the agent. Alternatively, if the candidate bioactive agent is labeled, the presence of the label on the support indicates displacement.

In an alternative embodiment, the candidate bioactive agent is added first, with incubation and washing, followed by the competitor. The absence of binding by the competitor may indicate that the bioactive agent is bound to the CA protein with a higher affinity. Thus, if the candidate bioactive agent is labeled, the presence of the label on the support, coupled with a lack of competitor binding, may indicate that the candidate agent is capable of binding to the CA protein.

In a preferred embodiment, the methods comprise differential screening to identity bioactive agents that are capable of modulating the activity of the CA proteins. In this embodiment, the methods comprise combining a CA protein and a competitor in a first sample. A second sample comprises a candidate bioactive agent, a CA protein and a competitor. The binding of the competitor is determined for both samples, and a change, or difference in binding between the two samples indicates the presence of an agent capable of binding to the CA protein and potentially modulating its activity. That is, if the binding of the competitor is different in the second sample relative to the first sample, the agent is capable of binding to the CA protein.

Alternatively, a preferred embodiment utilizes differential screening to identify drug candidates that bind to the native CA protein, but cannot bind to modified CA proteins. The structure of the CA protein may be modeled, and used in rational drug design to synthesize agents that interact with that site. Drug candidates that affect CA bioactivity are also identified by screening drugs for the ability to either enhance or reduce the activity of the protein.

Positive controls and negative controls may be used in the assays. Preferably all control and test samples are performed in at least triplicate to obtain statistically significant results. Incubation of all samples is for a time sufficient for the binding of the agent to the protein. Following incubation, all samples are washed free of non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for the requisite binding.

Screening for agents that modulate the activity of CA proteins may also be done. In a preferred embodiment, methods for screening for a bioactive agent capable of modulating the activity of CA proteins comprise the steps of adding a candidate bioactive agent to a sample of CA proteins, as above, and determining an alteration in the biological activity of CA proteins. "Modulating the activity of a CA protein" includes an increase in activity, a decrease in activity, or a change in the type or kind of activity present. Thus, in this embodiment, the candidate agent should both bind to CA proteins (although this may not be necessary), and alter its biological or biochemical activity as defined herein. The methods include both *in vitro* screening methods, as are generally outlined above, and in vivo screening of cells for alterations in the presence, distribution, activity or amount of CA proteins.

Thus, in this embodiment, the methods comprise combining a CA sample and a candidate bioactive agent, and evaluating the effect on CA activity. By "CA activity" or grammatical equivalents herein is meant one of the CA protein's biological activities, including, but not limited to, its role in tumorigenesis, including cell division, preferably in lymphatic tissue, cell proliferation, tumor growth and transformation of cells. In one embodiment, CA activity includes activation of or by a protein encoded by a nucleic acid of Tables 1-19. An inhibitor of CA activity is the inhibition of any one or more CA activities.

In a preferred embodiment, the activity of the CA protein is increased; in another preferred embodiment, the activity of the CA protein is decreased. Thus, bioactive agents that are antagonists are preferred in some embodiments, and bioactive agents that are agonists may be preferred in other embodiments.

In a preferred embodiment, the invention provides methods for screening for bioactive agents capable of modulating the activity of a CA protein. The methods comprise adding a candidate bioactive agent, as defined above, to a cell comprising CA proteins. Preferred cell types include almost any cell. The cells contain a recombinant nucleic acid that encodes a CA protein. In a preferred embodiment, a library of candidate agents is tested on a plurality of cells.

In one aspect, the assays are evaluated in the presence or absence or previous or subsequent exposure of physiological signals, for example hormones, antibodies, peptides, antigens, cytokines, growth factors, action potentials, pharmacological agents including chemotherapeutics, radiation, carcinogenics, or other cells (i.e. cell-cell contacts). In another example, the determinations are determined at different stages of the cell cycle process.

In this way, bioactive agents are identified. Compounds with pharmacological activity are able to enhance or interfere with the activity of the CA protein.

**Applications of the invention**

In one embodiment, a method of inhibiting cancer cell division is provided. In another embodiment, a method of inhibiting tumor growth is provided. In a further embodiment, methods of treating cells or individuals with cancer are provided.

The method comprises administration of a cancer inhibitor. In particular embodiments, the cancer inhibitor is an antisense molecule, a pharmaceutical composition, a therapeutic agent or small molecule, or a monoclonal, polyclonal, chimeric or humanized antibody. In particular embodiments, a therapeutic agent is coupled with a an antibody, preferable a monoclonal antobody.

In other embodiments, methods for detection or diagnosis of cancer cells in an individual are provided. In particular embodiments, the diagnostic/detection agent is a small molecule that pereferentially binds to a CAP according to the invention. In one embodiment, the diagnostic/detection agent is an antibody, preferably a monoclonal antibody, preferably linked to a detectable agent.

In other embodiments of the invention, animal models and transgenic animals are provided, which find use in generating animal models of cancers, particularly lymphomas and carcinomas.

**(a) Antisense molecules**

In one embodiment, the cancer inhibitor is an antisense molecule. Antisense molecules as used herein include antisense or sense oligonucleotides comprising a single-stranded nucleic acid sequence (either RNA or DNA) capable of binding to target mRNA (sense) or DNA (antisense) sequences for cancer molecules. Antisense or sense oligonucleotides, according to the present invention, comprise a fragment generally at least about 14 nucleotides, preferably from about 14 to 30 nucleotides. The ability to derive an antisense or a sense oligonucleotide, based upon a cDNA sequence encoding a given protein is described in, for example, Stein and Cohen, Cancer Res. 48:2659, (1988) and van der Krol et al., BioTechniques 6:958, (1988).

Antisense molecules can be modified or unmodified RNA, DNA, or mixed polymer oligonucleotides. These molecules function by specifically binding to matching sequences resulting in inhibition of peptide synthesis (Wu-Pong, Nov 1994, BioPharm, 20-33) either by steric blocking or by activating an RNase H enzyme. Antisense molecules can also alter protein synthesis by interfering with RNA processing or transport from the nucleus into the cytoplasm (Mukhopadhyay & Roth, 1996, Crit. Rev. in Oncogenesis 7, 151-190). In addition, binding of single stranded DNA to RNA can result in nuclease-mediated degradation of the heteroduplex (Wu-Pong, supra). Backbone modified DNA chemistry which have thus far been shown to act as substrates for RNase H are phosphorothioates, phosphorodithioates, borontrifluoridates, and 2'-arabino and 2'-fluoro arabino-containing oligonucleotides.

Antisense molecules may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell. Alternatively, a sense or an antisense oligonucleotide may be introduced into a cell containing the target nucleic acid sequence by formation of an oligonucleotide-lipid complex, as described in WO 90/10448. It is understood that the use of antisense molecules or knock out and knock in models may also be used in screening assays as discussed above, in addition to methods of treatment.

### (b) RNA Interference

RNA interference refers to the process of sequence-specific post transcriptional gene silencing in animals mediated by short interfering RNAs (siRNA) (Fire et al., Nature, 391, 806 (1998)). The corresponding process in plants is referred to as post transcriptional gene silencing or RNA silencing and is also referred to as quelling in fungi. The presence of dsRNA in cells triggers the RNAi response though a mechanism that has yet to be fully characterized. This mechanism appears to be different from the interferon response that results from dsRNA mediated activation of protein kinase PKR and 2',5'-oligoadenylate synthetase resulting in non-specific cleavage of mRNA by ribonuclease L. (reviewed in Sharp, P.A., RNA interference - 2001, Genes & Development 15:485-490 (2001)).

Small interfering RNAs (siRNAs) are powerful sequence-specific reagents designed to suppress the expression of genes in cultured mammalian cells through a process known as RNA interference (RNAi). Elbashir, S.M. et al. Nature 411:494-498 (2001); Caplen, N.J. et al. Proc. Natl. Acad. Sci. USA 98:9742-9747 (2001); Harborth, J. et al. J. Cell Sci. 114:4557-4565 (2001). The term "short interfering RNA" or "siRNA" refers to a double stranded nucleic acid molecule capable of RNA interference "RNAi", (see Kreutzer et al., WO 00/44895; Zernicka-Goetz et al. WO 01/36646; Fire, WO 99/32619; Mello and Fire, WO 01/29058). As used herein, siRNA molecules are limited to RNA molecules but further encompasses chemically modified nucleotides and non-nucleotides. siRNA gene-targeting experiments have been carried out by transient siRNA transfer into cells (achieved by such classic methods as liposome-mediated transfection, electroporation, or microinjection).

Molecules of siRNA are 21- to 23-nucleotide RNAs, with characteristic 2- to 3-nucleotide 3'-overhanging ends resembling the RNase III processing products of long double-stranded RNAs (dsRNAs) that normally initiate RNAi. When introduced into a cell, they assemble with yet-to-be-identified proteins of an endonuclease complex (RNA-induced silencing complex), which then guides target mRNA cleavage. As a consequence of degradation of the targeted mRNA, cells with a specific phenotype characteristic of suppression of the corresponding protein product are obtained. The small size of siRNAs, compared with traditional antisense molecules, prevents activation of the dsRNA-inducible interferon system present in mammalian cells. This avoids the nonspecific phenotypes normally produced by dsRNA larger than 30 base pairs in somatic cells.

Intracellular transcription of small RNA molecules is achieved by cloning the siRNA templates into RNA polymerase III (Pol III) transcription units, which normally encode the small nuclear RNA (snRNA) U6 or the human RNase P RNA H1. Two approaches have been developed for expressing siRNAs: in the first, sense and antisense strands constituting the siRNA duplex are transcribed by individual promoters (Lee, N.S. et al. Nat. Biotechnol. 20, 500-505 (2002) .Miyagishi, M. & Taira, K. Nat. Biotechnol. 20, 497-500 (2002).); in the second, siRNAs are expressed as fold-back stem-loop structures that give rise to siRNAs after intracellular processing (Paul, C.P. et al. Nat. Biotechnol. 20:505-508 (2002) ). The endogenous expression of siRNAs from introduced DNA templates is thought to overcome some limitations of exogenous siRNA delivery, in particular the transient loss of phenotype. U6 and H1 RNA promoters are members of the type III class of Pol III promoters. (Paule, M.R. & White, R.J. Nucleic Acids Res. 28, 1283-1298 (2000)).

Co-expression of sense and antisense siRNAs mediate silencing of target genes, whereas expression of sense or antisense siRNA alone do not greatly affect target gene expression. Transfection of plasmid DNA, rather than synthetic siRNAs, may appear advantageous, considering the danger of RNase contamination and the costs of chemically synthesized siRNAs or siRNA transcription kits. Stable expression of siRNAs allows new gene therapy applications, such as treatment of persistent viral infections. Considering the high specificity of siRNAs, the approach also allows the targeting of disease-derived transcripts with point mutations, such as *RAS* or *TP53* oncogene transcripts, without alteration of the remaining wild-type allele. Finally, by high-throughput sequence analysis of the various genomes, the DNA-based methodology may also be a cost-effective alternative for automated genome-wide loss-of-function phenotypic analysis, especially when combined with miniaturized array-based phenotypic screens. (Ziauddin, J. & Sabatini, D.M. Nature, 411:107-110 (2001)).

The presence of long dsRNAs in cells stimulates the activity of a ribonuclease III enzyme referred to as dicer. Dicer is involved in the processing of the dsRNA into short pieces of dsRNA known as short interfering RNAs (siRNA) (Berstein et al., 2001, Nature, 409:363 (2001)). Short interfering RNAs derived from dicer activity are typically about 21-23 nucleotides in length and comprise about 19 base pair duplexes. Dicer has also been implicated in the excision of 21 and 22 nucleotide small temporal RNAs (stRNA) from precursor RNA of conserved structure that are implicated in translational control (Hutvagner et al., Science, 293, 834 (2001)). The RNAi response also features an endonuclease complex containing a siRNA, commonly referred to as an RNA-induced silencing complex (RISC), which mediates cleavage of single stranded RNA having sequence homologous to the siRNA. Cleavage of the target RNA takes place in the middle of the region complementary to the guide sequence of the siRNA duplex (Elbashir et al., Genes Dev., 15, 188 (2001)).

This invention provides an expression system comprising an isolated nucleic acid molecule comprising a sequence capable of specifically hybridizing to the CA sequences. In an embodiment, the nucleic acid molecule is capable of inhibiting the expression of the CA protein. A method of inhibiting expression of CA inside a cell by a vector-directed expression of a short RNA which short RNA can fold in itself and create a double strand RNA having CA mRNA sequence identity and able to trigger posttranscriptional gene silencing, or RNA interference (RNAi), of the CA gene inside the cell. In another method a short double strand RNA having CA mRNA sequence identity is delivered inside the cell to trigger posttranscriptional gene silencing, or RNAi, ofthe CA gene. In various embodiments, the nucleic acid molecule is at least a 7 mer, at least a 10 mer, or at least a 20 mer. In a further embodiment, the sequence is unique.

## (c) Pharmaceutical Compositions

Pharmaceutical compositions encompassed by the present invention include as active agent, the polypeptides, polynucleotides, antisense oligonucleotides, or antibodies of the invention disclosed herein in a therapeutically effective amount. An "effective amount" is an amount sufficient to effect beneficial or desired results, including clinical results. An effective amount can be administered in one or more administrations. For purposes of this invention, an effective amount of an adenoviral vector is an amount that is sufficient to palliate, ameliorate, stabilize, reverse, slow or delay the progression of the disease state.

The compositions can be used to treat cancer as well as metastases of primary cancer. In addition, the pharmaceutical compositions can be used in conjunction with conventional methods of cancer treatment, e.g., to sensitize tumors to radiation or conventional chemotherapy. The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

Where the pharmaceutical composition comprises an antibody that specifically binds to a gene product encoded by a differentially expressed polynucleotide, the antibody can be coupled to a drug for delivery to a treatment site or coupled to a detectable label to facilitate imaging of a site comprising cancer cells, such as prostate cancer cells. Methods for coupling antibodies to drugs and detectable labels are well known in the art, as are methods for imaging using detectable labels.

A "patient" for the purposes of the present invention includes both humans and other animals, particularly mammals, and organisms. Thus the methods are applicable to both human therapy and veterinary applications. In the preferred embodiment the patient is a mammal, and in the most preferred embodiment the patient is human.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. The effective amount for a given situation is determined by routine experimentation and is within the judgment of the clinician. For purposes of the present invention, an effective dose will generally be from about 0.01 mg/kg to about 5 mg/kg, or about 0.01 mg/kg to about 50 mg/kg or about 0.05 mg/kg to about 10 mg/kg of the compositions of the present invention in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles. Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins.

The pharmaceutical compositions can be prepared in various forms, such as granules, tablets, pills, suppositories, capsules, suspensions, salves, lotions and the like. Pharmaceutical grade organic or inorganic carriers and/or diluents suitable for oral and topical use can be used to make up compositions containing the therapeutically-active compounds. Diluents known to the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents.

The pharmaceutical compositions of the present invention comprise a CA protein in a form suitable for administration to a patient. In the preferred embodiment, the pharmaceutical compositions are in a water soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine.

The pharmaceutical compositions may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds having the desired pharmacological activity may be administered in a physiologically acceptable carrier to a host, as previously described. The agents may be administered in a variety of ways, orally, parenterally e.g., subcutaneously, intraperitoneally, intravascularly, etc. Depending upon the manner of introduction, the compounds may be formulated in a variety of ways. The concentration of therapeutically active compound in the formulation may vary from about 0.1-100% wgt/vol. Once formulated, the compositions contemplated by the invention can be

(1) administered directly to the subject (e.g., as polynucleotide, polypeptides, small molecule agonists or antagonists, and the like); or (2) delivered ex vivo, to cells derived from the subject (e.g., as in *ex vivo* gene therapy). Direct delivery of the compositions will generally be accomplished by parenteral injection, e.g., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the ex vivo delivery and reimplantation of transformed cells into a subject are known in the art and described in e.g., International Publication No. WO 93/14778. Examples of cells useful in ex vivo applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once differential expression of a gene corresponding to a CA polynucleotide described herein has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (e.g., antisense, ribozyme, etc.). In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonist for gene products that are decreased in expression in cancerous cells (e.g., to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic compositions agents includes local or systemic administration, including injection, oral administration, particle gun or catheterized administration, and topical administration. Preferably, the therapeutic polynucleotide composition contains an expression construct comprising a promoter operably linked to a polynucleotide of at least 12, 22, 25, 30, or 35 contiguous nt of the polynucleotide disclosed herein. Various methods can be used to administer the therapeutic composition directly to a specific site in the body. For example, a small metastatic lesion is located and the therapeutic composition injected several times in several different locations within the body of tumor. Alternatively, arteries that serve a tumor are identified, and the therapeutic composition injected into such an artery, in order to deliver the composition directly into the tumor. A tumor that has a necrotic center is aspirated and the composition injected directly into the now empty center of the tumor. An antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655; Wu et al., J. Biol. Chem. (1991) 266: 338. Therapeutic compositions containing a polynucleotide are administered in a range of about 100 ng to about 200 mg of DNA for local administration in a gene therapy protocol. Concentration ranges of about 500 ng to about 50 mg, about 1 $\mu$g to about 2 mg, about 5 $\mu$g to about 500 $\mu$g, and about 20 $\mu$g to about 100 $\mu$g of DNA can also be used during a gene therapy protocol. Factors such as method of action (e.g., for enhancing or inhibiting levels of the encoded gene product) and efficacy of transformation and expression are considerations that will affect the dosage required for ultimate efficacy of the antisense subgenomic polynucleotides. Where greater expression is desired over a larger area of tissue, larger amounts of antisense subgenomic polynucleotides or the same amounts re-administered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect.

The therapeutic polynucleotides and polypeptides of the present invention can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., WO 90/07936; WO 94/03622; WO 93/25698; WO 93/25234; USPN 5, 219,740; WO 93/11230; WO 93/10218; USPN 4,777,127; GB Patent No. 2,200,651; EP 0 345 242; and WO 91/02805), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., USPN 5,814,482; WO 95/07994; WO 96/17072; WO 95/30763; and WO 97/42338) and nucleic charge neutralization or fusion with cell membranes. Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581.

Further non-viral delivery suitable for use includes mechanical delivery systems such as the approach described in Woffendin et al., Proc. Natl. Acad. Sci. USA (1994) 91(24):11581. Moreover, the coding sequence and the product of expression of such can be delivered through deposition of photopolymerized hydrogel materials or use of ionizing radiation (see, e.g., USPN 5,206,152 and WO 92/11033). Other conventional methods for gene delivery that can be used for delivery of the coding sequence include, for example, use of hand-held gene transfer particle gun (see, e.g., USPN 5,149,655); use of ionizing radiation for activating transferred gene (see, e.g., USPN 5,206,152 and WO 92/11033).

The administration of the CA proteins and modulators of the present invention can be done in a variety of ways as discussed above, including, but not limited to, orally, subcutaneously, intravenously, intranasally, transdermally, intraperitoneally, intramuscularly, intrapulmonary, vaginally, rectally, or intraocularly. In some instances, for example, in the treatment of wounds and inflammation, the CA proteins and modulators may be directly applied as a solution or spray.

In a preferred embodiment, CA proteins and modulators are administered as therapeutic agents, and can be formulated as outlined above. Similarly, CA genes (including both the full-length sequence, partial sequences, or regulatory sequences of the CA coding regions) can be administered in gene therapy applications, as is known in the art. These CA genes can include antisense applications, either as gene therapy (i.e. for incorporation into the genome) or as antisense compositions, as will be appreciated by those in the art.

Thus, in one embodiment, methods of modulating CA gene activity in cells or organisms are provided. In one embodiment, the methods comprise administering to a cell an anti-CA antibody that reduces or eliminates the biological activity of an endogenous CA protein. Alternatively, the methods comprise administering to a cell or organism a recombinant nucleic acid encoding a CA protein. As will be appreciated by those in the art, this may be accomplished in any number of ways. In a preferred embodiment, for example when the CA sequence is down-regulated in cancer, the activity of the CA gene product is increased by increasing the amount of CA expression in the cell, for example by overexpressing the endogenous CA gene or by administering a gene encoding the CA sequence, using known gene-therapy techniques. In a preferred embodiment, the gene therapy techniques include the incorporation of the exogenous gene using enhanced homologous recombination (EHR), for example as described in PCT/US93/03868, hereby incorporated by reference in its entirety. Alternatively, for example when the CA sequence is up-regulated in cancer, the activity of the endogenous CA gene is decreased, for example by the administration of a CA antisense nucleic acid.

### (d) Vaccines

In a preferred embodiment, CA genes are administered as DNA vaccines, either single genes or combinations of CA genes. Naked DNA vaccines are generally known in the art. Brower, Nature Biotechnology, 16:1304-1305 (1998).

In one embodiment, CA genes of the present invention are used as DNA vaccines. Methods for the use of genes as DNA vaccines are well known to one of ordinary skill in the art, and include placing a CA gene or portion of a CA gene under the control of a promoter for expression in a patient with cancer. The CA gene used for DNA vaccines can encode full-length CA proteins, but more preferably encodes portions of the CA proteins including peptides derived from the CA protein. In a preferred embodiment a patient is immunized with a DNA vaccine comprising a plurality of nucleotide sequences derived from a CA gene. Similarly, it is possible to immunize a patient with a plurality of CA genes or portions thereof. Without being bound by theory, expression of the polypeptide encoded by the DNA vaccine, cytotoxic T-cells, helper T-cells and antibodies are induced that recognize and destroy or eliminate cells expressing CA proteins.

In a preferred embodiment, the DNA vaccines include a gene encoding an adjuvant molecule with the DNA vaccine. Such adjuvant molecules include cytokines that increase the immunogenic response to the CA polypeptide encoded by the DNA vaccine. Additional or alternative adjuvants are known to those of ordinary skill in the art and find use in the invention.

### (e) Antibodies

In one embodiment, a cancer inhibitor is an antibody as discussed above. In one embodiment, the CA proteins of the present invention may be used to generate polyclonal and monoclonal antibodies to CA proteins, which are useful as described herein. Similarly, the CA proteins can be coupled, using standard technology, to affinity chromatography columns. These columns may then be used to purify CA antibodies. In a preferred embodiment, the antibodies are generated to epitopes unique to a CA protein; that is, the antibodies show little or no cross-reactivity to other proteins. These antibodies find use in a number of applications. For example, the CA antibodies may be coupled to standard affinity chromatography columns and used to purify CA proteins. The antibodies may also be used therapeutically as blocking polypeptides, as outlined above, since they will specifically bind to the CA protein.

The present invention further provides methods for detecting the presence of and/or measuring a level of a polypeptide in a biological sample, which CA polypeptide is encoded by a CA polynucleotide that is differentially expressed in a cancer cell, using an antibody specific for the encoded polypeptide. The methods generally comprise: a) contacting the sample with an antibody specific for a polypeptide encoded by a CA polynucleotide that is differentially expressed in a prostate cancer cell; and b) detecting binding between the antibody and molecules of the sample.

Detection of specific binding of the antibody specific for the encoded cancer-associated polypeptide, when compared to a suitable control is an indication that encoded polypeptide is present in the sample. Suitable controls include a sample known not to contain the encoded CA polypeptide or known not to contain elevated levels of the polypeptide; such as normal tissue, and a sample contacted with an antibody not specific for the encoded polypeptide, e.g., an anti-idiotype antibody. A variety of methods to detect specific antibody-antigen interactions are known in the art and can be used in the method, including, but not limited to, standard immunohistological methods, immunoprecipitation, an enzyme immmoassay, and a radioimmunoassay. In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., $^{152}Eu$, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like. The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins. The support may then be washed with suitable buffers, followed by contacting with a detectably-labeled first specific antibody. Detection methods are known in the art and will be chosen as appropriate to the signal emitted by the detectable label. Detection is generally accomplished in comparison to suitable controls, and to appropriate standards.

In some embodiments, the methods are adapted for use *in vivo*, e.g., to locate or identify sites where cancer cells are present. In these embodiments, a detectably-labeled moiety, e.g., an antibody, which is specific for a cancer-associated polypeptide is administered to an individual (e.g., by injection), and labeled cells are located using standard imaging techniques, including, but not limited to, magnetic resonance imaging, computed tomography scanning, and the like. In this manner, cancer cells are differentially labeled.

## (f) Detection and Diagnosis of Cancers

Without being bound by theory, it appears that the various CA sequences are important in cancers. Accordingly, disorders based on mutant or variant CA genes may be determined. In one embodiment, the invention provides methods for identifying cells containing variant CA genes comprising determining all or part of the sequence of at least one endogenous CA genes in a cell. As will be appreciated by those in the art, this may be done using any number of sequencing techniques. In a preferred embodiment, the invention provides methods of identifying the CA genotype of an individual comprising determining all or part of the sequence of at least one CA gene of the individual. This is generally done in at least one tissue of the individual, and may include the evaluation of a number of tissues or different samples of the same tissue. The method may include comparing the sequence of the sequenced CA gene to a known CA gene, i.e., a wild-type gene. As will be appreciated by those in the art, alterations in the sequence of some CA genes can be an indication of either the presence of the disease, or propensity to develop the disease, or prognosis evaluations.

The sequence of all or part of the CA gene can then be compared to the sequence of a known CA gene to determine if any differences exist. This can be done using any number of known homology programs, such as Bestfit, etc. In a preferred embodiment, the presence of a difference in the sequence between the CA gene of the patient and the known CA gene is indicative of a disease state or a propensity for a disease state, as outlined herein.

In a preferred embodiment, the CA genes are used as probes to determine the number of copies of the CA gene in the genome. For example, some cancers exhibit chromosomal deletions or insertions, resulting in an alteration in the copy number of a gene.

In another preferred embodiment CA genes are used as probes to determine the chromosomal location of the CA genes. Information such as chromosomal location finds use in providing a diagnosis or prognosis in particular when chromosomal abnormalities such as translocations, and the like are identified in CA gene loci.

The present invention provides methods of using the polynucleotides described herein for detecting cancer cells, facilitating diagnosis of cancer and the severity of a cancer (e.g., tumor grade, tumor burden, and the like) in a subject, facilitating a determination of the prognosis of a subject, and assessing the responsiveness of the subject to therapy (e.g., by providing a measure of therapeutic effect through, for example, assessing tumor burden during or following a chemotherapeutic regimen). Detection can be based on detection of a polynucleotide that is differentially expressed in a cancer cell, and/or detection of a polypeptide encoded by a polynucleotide that is differentially expressed in a cancer cell. The detection methods of the invention can be conducted *in vitro* or *in vivo*, on isolated cells, or in whole tissues or a bodily fluid *e.g.*, blood, plasma, serum, urine, and the like).

In some embodiments, methods are provided for detecting a cancer cell by detecting expression in the cell of a transcript that is differentially expressed in a cancer cell. Any of a variety of known methods can be used for detection, including, but not limited to, detection of a transcript by hybridization with a polynucleotide that hybridizes to a polynucleotide that is differentially expressed in a prostate cancer cell; detection of a transcript by a polymerase chain reaction using specific oligonucleotide primers; *in situ* hybridization of a cell using as a probe a polynucleotide that hybridizes to a gene that is differentially expressed in a prostate cancer cell. The methods can be used to detect and/or measure mRNA levels of a gene that is differentially expressed in a cancer cell. In some embodiments, the methods comprise: a) contacting a sample with a polynucleotide that corresponds to a differentially expressed gene described herein under conditions that allow hybridization; and b) detecting hybridization, if any.

Detection of differential hybridization, when compared to a suitable control, is an indication of the presence in the sample of a polynucleotide that is differentially expressed in a cancer cell. Appropriate controls include, for example, a sample that is known not to contain a polynucleotide that is differentially expressed in a cancer cell, and use of a labeled polynucleotide of the same "sense" as the polynucleotide that is differentially expressed in the cancer cell. Conditions that allow hybridization are known in the art, and have been described in more detail above. Detection can also be accomplished by any known method, including, but not limited to, *in situ* hybridization, PCR (polymerase chain reaction), RT-PCR (reverse transcription-PCR), TMA, bDNA, and Nasbau and "Northern" or RNA blotting, or combinations of such techniques, using a suitably labeled polynucleotide. A variety of labels and labeling methods for polynucleotides are known in the art and can be used in the assay methods of the invention. Specificity of hybridization can be determined by comparison to appropriate controls.

Polynucleotides generally comprising at least 10 nt, at least 12nt or at least 15 contiguous nucleotides of a polynucleotide provided herein, such as, for example, those having the sequence as depicted in Tables 1-19, are used for a variety of purposes, such as probes for detection of and/or measurement of, transcription levels of a polynucleotide that is differentially expressed in a prostate cancer cell. As will be readily appreciated by the ordinarily skilled artisan, the probe can be detectably labeled and contacted with, for example, an array comprising immobilized polynucleotides obtained from a test sample (e.g., mRNA). Alternatively, the probe can be immobilized on an array and the test sample detectably labeled. These and other variations of the methods of the invention are well within the skill in the art and are within the scope of the invention.

Nucleotide probes are used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluorophores, and enzymes. Other examples of nucleotide hybridization assays are described in WO92/02526 and USPN 5,124,246.

PCR is another means for detecting small amounts of target nucleic acids (see, e.g., Mullis et al., Meth. Enzymol. (1987) 155:335; USPN 4,683,195; and USPN 4,683,202). Two primer oligonucleotides that hybridize with the target nucleic acids are used to prime the reaction. The primers can be composed of sequence within or 3' and 5' to the CA polynucleotides disclosed herein. Alternatively, if the primers are 3' and 5' to these polynucleotides, they need not hybridize to them or the complements. After amplification of the target with a thermostable polymerase, the amplified target nucleic acids can be detected by methods known in the art, e.g., Southern blot. mRNA or cDNA can also be detected by traditional blotting techniques (e.g., Southern blot, Northern blot, etc.) described in Sambrook et al., "Molecular Cloning: A Laboratory Manual" (New York, Cold Spring Harbor Laboratory, 1989) (e.g., without PCR amplification). In general, mRNA or cDNA generated from mRNA using a polymerase enzyme can be purified and separated using gel electrophoresis, and transferred to a solid support, such as nitrocellulose. The solid support is exposed to a labeled probe, washed to remove any unhybridized probe, and duplexes containing the labeled probe are detected.

Methods using PCR amplification can be performed on the DNA from a single cell, although it is convenient to use at least about $10^5$ cells. The use of the polymerase chain reaction is described in Saiki et al. (1985) Science 239: 487, and a review of current techniques may be found in Sambrook, et al. Molecular Cloning: A Laboratory Manual, CSH Press 1989, pp.14.2-14.33. A detectable label may be included in the amplification reaction. Suitable detectable labels include fluorochromes,(*e.g.* fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)), radioactive labels, (*e.g.* $^{32}$P, $^{35}$S, $^{3}$H, *etc.*), and the like. The label may be a two stage system, where the polynucleotides is conjugated to biotin, haptens, *etc.* having a high affinity binding partner, e.g. avidin, specific antibodies, etc., where the binding partner is conjugated to a detectable label. The label may be conjugated to one or both of the primers. Alternatively, the pool of nucleotides used in the amplification is labeled, so as to incorporate the label into the amplification product.

The detection methods can be provided as part of a kit. Thus, the invention further provides kits for detecting the presence and/or a level of a polynucleotide that is differentially expressed in a cancer cell (*e.g.*, by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits of the invention for detecting a polypeptide encoded by a polynucleotide that is differentially expressed in a cancer cell may comprise a moiety that specifically binds the polypeptide, which may be an antibody that binds the polypeptide or fragment thereof. The kits of the invention used for detecting a polynucleotide that is differentially expressed in a prostate cancer cell may comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information. Accordingly, the present invention provides kits for detecting prostate cancer comprising at least one of polynucleotides having the sequence as shown in Tables 1-19 or fragments thereof.

The present invention further relates to methods of detecting/diagnosing a neoplastic or preneoplastic condition in a mammal (for example, a human). "Diagnosis" as used herein generally includes determination of a subject's susceptibility to a disease or disorder, determination as to whether a subject is presently affected by a disease or disorder, prognosis of a subject affected by a disease or disorder (*e.g.*, identification of pre-metastatic or metastatic cancerous states, stages of cancer, or responsiveness of cancer to therapy), and therametrics (*e.g.*, monitoring a subject's condition to provide information as to the effect or efficacy of therapy).

The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

An "effective amount" is an amount sufficient to effect beneficial or desired results, including clinical results. An effective amount can be administered in one or more administrations.

A "cell sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides. The term "cell sample" encompasses a clinical sample, and also includes cells in culture, cell supernatants, cell lysates, serum, plasma, biological fluid, and tissue samples.

As used herein, the terms "neoplastic cells", "neoplasia", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation (i.e., de-regulated cell division). Neoplastic cells can be malignant or benign.

The terms "individual," "subject," "host," and "patient," are used interchangeably herein and refer to any mammalian subject for whom diagnosis, treatment, or therapy is desired, particularly humans. Other subjects may include cattle, dogs, cats, guinea pigs, rabbits, rats, mice, horses, and so on. Examples of conditions that can be detected/diagnosed in accordance with these methods include cancers. Polynucleotides corresponding to genes that exhibit the appropriate expression pattern can be used to detect cancer in a subject. For a review of markers of cancer, see, *e.g.*, Hanahan et al. Cell 100:57-70 (2000).

One detection/diagnostic method comprises: (a) obtaining from a mammal (e.g., a human) a biological sample, (b) detecting the presence in the sample of a CA protein and (c) comparing the amount of product present with that in a control sample. In accordance with this method, the presence in the sample of elevated levels of a CA gene product indicates that the subject has a neoplastic or preneoplastic condition.

Biological samples suitable for use in this method include biological fluids such as serum, plasma, pleural effusions, urine and cerebro-spinal fluid, CSF, tissue samples (e.g., mammary tumor or prostate tissue slices) can also be used in the method of the invention, including samples derived from biopsies. Cell cultures or cell extracts derived, for example, from tissue biopsies can also be used.

The compound is preferably a binding protein, e.g., an antibody, polyclonal or monoclonal, or antigen binding fragment thereof, which can be labeled with a detectable marker (e.g., fluorophore, chromophore or isotope, etc). Where appropriate, the compound can be attached to a solid support such as a bead, plate, filter, resin, etc. Determination of formation of the complex can be effected by contacting the complex with a further compound (e.g., an antibody) that specifically binds to the first compound (or complex). Like the first compound, the further compound can be attached to a solid support and/or can be labeled with a detectable marker.

The identification of elevated levels of CA protein in accordance with the present invention makes possible the identification of subjects (patients) that are likely to benefit from adjuvant therapy. For example, a biological sample from a post primary therapy subject (e.g., subject having undergone surgery) can be screened for the presence of circulating CA protein, the presence of elevated levels of the protein, determined by studies of normal populations, being indicative of residual tumor tissue. Similarly, tissue from the cut site of a surgically removed tumor can be examined (e.g., by immunofluorescence), the presence of elevated levels of product (relative to the surrounding tissue) being indicative of incomplete removal of the tumor. The ability to identify such subjects makes it possible to tailor therapy to the needs of the particular subject. Subjects undergoing non-surgical therapy, e.g., chemotherapy or radiation therapy, can also be monitored, the presence in samples from such subjects of elevated levels of CA protein being indicative of the need for continued treatment. Staging of the disease (for example, for purposes of optimizing treatment regimens) can also be effected, for example, by biopsy e.g.,. with antibody specific for a CA protein.

### (g) Animal Models and Transgenics

In another preferred embodiment CA genes find use in generating animal models of cancers, particularly lymphomas and carcinomas. As is appreciated by one of ordinary skill in the art, when the CA gene identified is repressed or diminished in CA tissue, gene therapy technology wherein antisense RNA directed to the CA gene will also diminish or repress expression of the gene. An animal generated as such serves as an animal model of CA that finds use in screening bioactive drug candidates. Similarly, gene knockout technology, for example as a result of homologous recombination with an appropriate gene targeting vector, will result in the absence of the CA protein. When desired, tissue-specific expression or knockout of the CA protein may be necessary.

It is also possible that the CA protein is overexpressed in cancer. As such, transgenic animals can be generated that overexpress the CA protein. Depending on the desired expression level, promoters of various strengths can be employed to express the transgene. Also, the number of copies of the integrated transgene can be determined and compared for a determination of the expression level of the transgene. Animals generated by such methods find use as animal models of CA and are additionally useful in screening for bioactive molecules to treat cancer.

### Characterization of CA sequences

The CA nucleic acid sequences of the invention are depicted in Tables 1-19. The sequences in each Table include genomic DNA sequence (mouse genomic sequences mDxx-yyy; human genomic sequences hDxx-yyy), sequence corresponding to the mRNA(s) generated therefrom (mRxx-yyy; hRxx-yyy) and amino acid sequences of the proteins (mPxx-yyy; hPxx-yyy) encoded by the mRNA for both mouse and human genes. N/A indicates a gene that has been identified, but for which there has not been a name ascribed.

The mouse and human genomic DNA sequence, sequence corresponding to the mRNA(s) generated therefrom and amino acid sequences of the proteins as shown in Tables 1-19 are described according to SEQ ID NOS as follows in Table 20.

**Table 20**

| DESIGNATION | SEQ ID NO | TYPE OF SEQUENCE |
|---|---|---|
| mD1-004 | SEQ ID NO: 1 | MOUSE GENOMIC SEQUENCE |
| mR1-004.1 | SEQ ID NO: 2 | MOUSE mRNA SEQUENCE |
| mP1-004.1 | SEQ ID NO: 3 | MOUSE PROTEIN SEQUENCE |
| hD1-004 | SEQ ID NO: 4 | HUMAN GENOMIC SEQUENCE |
| hR1-004.1 | SEQ ID NO: 5 | HUMAN mRNA SEQUENCE |
| hP1-004.1 | SEQ ID NO: 6 | HUMAN PROTEIN SEQUENCE |
| mD3-010 | SEQ ID NO: 7 | MOUSE GENOMIC SEQUENCE |
| mR3-010.1 | SEQ ID NO: 8 | MOUSE mRNA SEQUENCE |
| mP3-010.1 | SEQ ID NO: 9 | MOUSE PROTEIN SEQUENCE |
| hD3-010 | SEQ ID NO: 10 | HUMAN GENOMIC SEQUENCE |

(continued)

| DESIGNATION | SEQ ID NO | TYPE OF SEQUENCE |
|---|---|---|
| hR3-010.1 | SEQ ID NO: 11 | HUMAN mRNA SEQUENCE |
| hP3-010.1 | SEQ ID NO: 12 | HUMAN PROTEIN SEQUENCE |
| mD3-027 | SEQ ID NO: 13 | MOUSE GENOMIC SEQUENCE |
| mR3-027.1 | SEQ ID NO: 14 | MOUSE mRNA SEQUENCE |
| mP3-027.1 | SEQ ID NO: 15 | MOUSE PROTEIN SEQUENCE |
| hD3-027 | SEQ ID NO: 16 | HUMAN GENOMIC SEQUENCE |
| hR3-027.1 | SEQ ID NO: 17 | HUMAN mRNA SEQUENCE |
| hP3-027.1 | SEQ ID NO: 18 | HUMAN PROTEIN SEQUENCE |
| mD3-045 | SEQ ID NO: 19 | MOUSE GENOMIC SEQUENCE |
| mR3-045.1 | SEQ ID NO: 20 | MOUSE mRNA SEQUENCE |
| mP3-045.1 | SEQ ID NO: 21 | MOUSE PROTEIN SEQUENCE |
| hD3-045 | SEQ ID NO: 22 | HUMAN GENOMIC SEQUENCE |
| hR3-045.1 | SEQ ID NO: 23 | HUMAN mRNA SEQUENCE |
| hP3-045.1 | SEQ ID NO: 24 | HUMAN PROTEIN SEQUENCE |
| mD5-001 | SEQ ID NO: 25 | MOUSE GENOMIC SEQUENCE |
| nnR5-001.1 | SEQ ID NO: 26 | MOUSE mRNA SEQUENCE |
| mP5-001.1 | SEQ ID NO: 27 | MOUSE PROTEIN SEQUENCE |
| hD5-001 | SEQ ID NO: 28 | HUMAN GENOMIC SEQUENCE |
| hR5-001.1 | SEQ ID NO: 29 | HUMAN mRNA SEQUENCE |
| hP5-001.1 | SEQ ID NO: 30 | HUMAN PROTEIN SEQUENCE |
| mD5-002 | SEQ ID NO: 31 | MOUSE GENOMIC SEQUENCE |
| mR5-002.1 | SEQ ID NO: 32 | MOUSE mRNA SEQUENCE |
| mP5-002.1 | SEQ ID NO: 33 | MOUSE PROTEIN SEQUENCE |
| hD5-002 | SEQ ID NO: 34 | HUMAN GENOMIC SEQUENCE |
| hR5-002.1 | SEQ ID NO: 35 | HUMAN mRNA SEQUENCE |
| hP5-002.1 | SEQ ID NO: 36 | HUMAN PROTEIN SEQUENCE |
| mD5-030 | SEQ ID NO: 37 | MOUSE GENOMIC SEQUENCE |
| mR5-030.1 | SEQ ID NO: 38 | MOUSE mRNA SEQUENCE |
| mP5-030.1 | SEQ ID NO: 39 | MOUSE PROTEIN SEQUENCE |
| hD5-030 | SEQ ID NO: 40 | HUMAN GENOMIC SEQUENCE |
| hR5-030.1 | SEQ ID NO: 41 | HUMAN mRNA SEQUENCE |
| hP5-030.1 | SEQ ID NO: 42 | HUMAN PROTEIN SEQUENCE |
| mD5-033 | SEQ ID NO: 43 | MOUSE GENOMIC SEQUENCE |
| mR5-033.1 | SEQ ID NO: 44 | MOUSE mRNA SEQUENCE |
| mP5-033.1 | SEQ ID NO: 45 | MOUSE PROTEIN SEQUENCE |
| hD5-033 | SEQ ID NO: 46 | HUMAN GENOMIC SEQUENCE |
| hR5-033.1 | SEQ ID NO: 47 | HUMAN mRNA SEQUENCE |
| hP5-033.1 | SEQ ID NO: 48 | HUMAN PROTEIN SEQUENCE |

(continued)

| DESIGNATION | SEQ ID NO | TYPE OF SEQUENCE |
|---|---|---|
| mD5-034 | SEQ ID NO: 49 | MOUSE GENOMIC SEQUENCE |
| mR5-034.1 | SEQ ID NO: 50 | MOUSE mRNA SEQUENCE |
| mP5-034.1 | SEQ ID NO: 51 | MOUSE PROTEIN SEQUENCE |
| hD5-034 | SEQ ID NO: 52 | HUMAN GENOMIC SEQUENCE |
| hR5-034.1 | SEQ ID NO: 53 | HUMAN mRNA SEQUENCE |
| hP5-034.1 | SEQ ID NO: 54 | HUMAN PROTEIN SEQUENCE |
| mD6-034 | SEQ ID NO: 55 | MOUSE GENOMIC SEQUENCE |
| mR6-034.1 | SEQ ID NO: 56 | MOUSE mRNA SEQUENCE |
| mP6-034.1 | SEQ ID NO: 57 | MOUSE PROTEIN SEQUENCE |
| hD6-034 | SEQ ID NO: 58 | HUMAN GENOMIC SEQUENCE |
| hR6-034.1 | SEQ ID NO: 59 | HUMAN mRNA SEQUENCE |
| hP6-034.1 | SEQ ID NO: 60 | HUMAN PROTEIN SEQUENCE |
| hR6-034.2 | SEQ ID NO: 61 | HUMAN mRNA SEQUENCE |
| hP6-034.2 | SEQ ID NO: 62 | HUMAN PROTEIN SEQUENCE |
| mD6-131 | SEQ ID NO: 63 | MOUSE GENOMIC SEQUENCE |
| mR6-131.1 | SEQ ID NO: 64 | MOUSE mRNA SEQUENCE |
| mP6-131.1 | SEQ ID NO: 65 | MOUSE PROTEIN SEQUENCE |
| hD6-131 | SEQ ID NO: 66 | HUMAN GENOMIC SEQUENCE |
| hR6-131.1 | SEQ ID NO: 67 | HUMAN mRNA SEQUENCE |
| hP6-131.1 | SEQ ID NO: 68 | HUMAN PROTEIN SEQUENCE |
| mD7-046 | SEQ ID NO: 69 | MOUSE GENOMIC SEQUENCE |
| mR7-046.1 | SEQ ID NO: 70 | MOUSE mRNA SEQUENCE |
| mP7-046.1 | SEQ ID NO: 71 | MOUSE PROTEIN SEQUENCE |
| hD7-046 | SEQ ID NO: 72 | HUMAN GENOMIC SEQUENCE |
| hR7-046.1 | SEQ ID NO: 73 | HUMAN mRNA SEQUENCE |
| hP7-046.1 | SEQ ID NO: 74 | HUMAN PROTEIN SEQUENCE |
| hR7-046.2 | SEQ ID NO: 75 | HUMAN mRNA SEQUENCE |
| hP7-046.2 | SEQ ID NO: 76 | HUMAN PROTEIN SEQUENCE |
| mD7-104 | SEQ ID NO: 77 | MOUSE GENOMIC SEQUENCE |
| mR7-104.1 | SEQ ID NO: 78 | MOUSE mRNA SEQUENCE |
| mP7-104.1 | SEQ ID NO: 79 | MOUSE PROTEIN SEQUENCE |
| hD7-104 | SEQ ID NO: 80 | HUMAN GENOMIC SEQUENCE |
| hR7-104.1 | SEQ ID NO: 81 | HUMAN mRNA SEQUENCE |
| hP7-104.1 | SEQ ID NO: 82 | HUMAN PROTEIN SEQUENCE |
| mD7-157 | SEQ ID NO: 83 | MOUSE GENOMIC SEQUENCE |
| mR7-157.1 | SEQ ID NO: 84 | MOUSE mRNA SEQUENCE |
| mP7-157.1 | SEQ ID NO: 85 | MOUSE PROTEIN SEQUENCE |
| hD7-157 | SEQ ID NO: 86 | HUMAN GENOMIC SEQUENCE |

(continued)

| DESIGNATION | SEQ ID NO | TYPE OF SEQUENCE |
|---|---|---|
| hR7-157.1 | SEQ ID NO: 87 | HUMAN mRNA SEQUENCE |
| hP7-157.1 | SEQ ID NO: 88 | HUMAN PROTEIN SEQUENCE |
| hR7-157.2 | SEQ ID NO: 89 | HUMAN mRNA SEQUENCE |
| hP7-157.2 | SEQ ID NO: 90 | HUMAN PROTEIN SEQUENCE |
| hR7-157.3 | SEQ ID NO: 91 | HUMAN mRNA SEQUENCE |
| hP7-157.3 | SEQ ID NO: 92 | HUMAN PROTEIN SEQUENCE |
| hR7-157.4 | SEQ ID NO: 93 | HUMAN mRNA SEQUENCE |
| hP7-157.4 | SEQ ID NO: 94 | HUMAN PROTEIN SEQUENCE |
| mD7-161 | SEQ ID NO: 95 | MOUSE GENOMIC SEQUENCE |
| mR7-161.1 | SEQ ID NO: 96 | MOUSE mRNA SEQUENCE |
| mP7-161.1 | SEQ ID NO: 97 | MOUSE PROTEIN SEQUENCE |
| hD7-161 | SEQ ID NO: 98 | HUMAN GENOMIC SEQUENCE |
| hR7-161.1 | SEQ ID NO: 99 | HUMAN mRNA SEQUENCE |
| hP7-161.1 | SEQ ID NO: 100 | HUMAN PROTEIN SEQUENCE |
| mD13-023 | SEQ ID NO: 101 | MOUSE GENOMIC SEQUENCE |
| mR13-023.1 | SEQ ID NO: 102 | MOUSE mRNA SEQUENCE |
| mP13-023.1 | SEQ ID NO: 103 | MOUSE PROTEIN SEQUENCE |
| hD13-023 | SEQ ID NO: 104 | HUMAN GENOMIC SEQUENCE |
| hR13-023.1 | SEQ ID NO: 105 | HUMAN mRNA SEQUENCE |
| hP13-023.1 | SEQ ID NO: 106 | HUMAN PROTEIN SEQUENCE |
| mD14-023 | SEQ ID NO: 107 | MOUSE GENOMIC SEQUENCE |
| mR14-023.1 | SEQ ID NO: 108 | MOUSE mRNA SEQUENCE |
| mP14-023.1 | SEQ ID NO: 109 | MOUSE PROTEIN SEQUENCE |
| hD14-023 | SEQ ID NO: 110 | HUMAN GENOMIC SEQUENCE |
| hR14-023.1 | SEQ ID NO: 111 | HUMAN mRNA SEQUENCE |
| hP14-023.1 | SEQ ID NO: 112 | HUMAN PROTEIN SEQUENCE |
| hR14-0.23.2 | SEQ ID NO: 113 | HUMAN mRNA SEQUENCE |
| hP14-023.2 | SEQ ID NO: 114 | HUMAN PROTEIN SEQUENCE |
| hR14-023.3 | SEQ ID NO: 115 | HUMAN mRNA SEQUENCE |
| hP14-023.3 | SEQ ID NO: 116 | HUMAN PROTEIN SEQUENCE |
| hR14-023.4 | SEQ ID NO: 117 | HUMAN mRNA SEQUENCE |
| hP14-023.4 | SEQ ID NO: 118 | HUMAN PROTEIN SEQUENCE |
| hR14-023.5 | SEQ ID NO: 119 | HUMAN mRNA SEQUENCE |
| hP14-023.5 | SEQ ID NO: 120 | HUMAN PROTEIN SEQUENCE |
| hR14-023.6 | SEQ ID NO: 121 | HUMAN mRNA SEQUENCE |
| hP14-023.6 | SEQ ID NO: 122 | HUMAN PROTEIN SEQUENCE |
| hR14-023.7 | SEQ ID NO: 123 | HUMAN mRNA SEQUENCE |
| hP14-023.7 | SEQ ID NO: 124 | HUMAN PROTEIN SEQUENCE |

(continued)

| DESIGNATION | SEQ ID NO | TYPE OF SEQUENCE |
|---|---|---|
| hR14-023.8 | SEQ ID NO: 125 | HUMAN mRNA SEQUENCE |
| hP14-023.8 | SEQ ID NO: 126 | HUMAN PROTEIN SEQUENCE |
| hR14-023.9 | SEQ ID NO: 127 | HUMAN mRNA SEQUENCE |
| hP14-023.9 | SEQ ID NO: 128 | HUMAN PROTEIN SEQUENCE |
| mD14-032 | SEQ ID NO: 129 | MOUSE GENOMIC SEQUENCE |
| mR14-032.1 | SEQ ID NO: 130 | MOUSE mRNA SEQUENCE |
| mP14-032.1 | SEQ ID NO: 131 | MOUSE PROTEIN SEQUENCE |
| hD14-032 | SEQ ID NO: 132 | HUMAN GENOMIC SEQUENCE |
| hR14-032.1 | SEQ ID NO: 133 | HUMAN mRNA SEQUENCE |
| hP14-032.1 | SEQ ID NO: 134 | HUMAN PROTEIN SEQUENCE |
| mD22-005 | SEQ ID NO: 135 | MOUSE GENOMIC SEQUENCE |
| mR22-005.1 | SEQ ID NO: 136 | MOUSE mRNA SEQUENCE |
| mP22-005.1 | SEQ ID NO: 137 | MOUSE PROTEIN SEQUENCE |
| hD22-005 | SEQ ID NO: 138 | HUMAN GENOMIC SEQUENCE |
| hR22-005.1 | SEQ ID NO: 139 | HUMAN mRNA SEQUENCE |
| hP22-005.1 | SEQ ID NO: 140 | HUMAN PROTEIN SEQUENCE |
| hR22-005.2 | SEQ ID NO: 141 | HUMAN mRNA SEQUENCE |
| hP22-005.2 | SEQ ID NO: 142 | HUMAN PROTEIN SEQUENCE |
| hR22-005.3 | SEQ ID NO: 143 | HUMAN mRNA SEQUENCE |
| hP22-005.3 | SEQ ID NO: 144 | HUMAN PROTEIN SEQUENCE |
| hR22-005.4 | SEQ ID NO: 145 | HUMAN mRNA SEQUENCE |
| hP22-005.4 | SEQ ID NO: 146 | HUMAN PROTEIN SEQUENCE |

The CA sequences were analyzed by Panther™ (Molecular Diagnostics, Palo Alto, CA) software designed to detect homologs and enable prediction of molecular function through a system for protein functional classification. Human Gene Ontlogy annotations were prepared in accordance with the Gene Ontology Consortium (Gene Ontology: tool for the unification of biology. The Gene Ontology Consortium Nature Genet. 25: 25-29 (2000)). Similar analysis was carried out by determining IPR information regarding the CA polypeptides from InterPro, which is an integrated documentation resource for protein families, domains and functional sites (Apweiler at al. Bioinformatics 16(12):1145-1150 (2000)).

The CA sequences may be classified according to the following predicted general classifications of function by Panther™ analysis, human gene ontology and IPR domain information for polypeptides having SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 as shown in Tables 1-19. The classifications are shown in Table 21 below.

**Table 21**

| Human Protein | SEQ ID NO: | FUNCTION |
|---|---|---|
| hP1-004.1 | SEQ ID NO: 6 | HUMAN PANTHER CLASSIFICATIONS<br>    FAMILY (SUBFAMILY)<br>    C-C CHEMOKINE RECEPTOR-RELATED(C-C CHEMOKINE RECEPTOR TYPE 7)<br>    BIOLOGICAL PROCESS<br>    Signal transduction(2.11.00.00.00) > Intracellular signaling cascade (2.11.02.00.00) > Calcium mediated signaling(2.11.02.02.00) |

(continued)

| Human Protein | SEQ ID NO: | FUNCTION |
|---|---|---|
| | | Signal transduction(2.11.00.00.00) > Cell surface receptor mediated signal transduction(2.11.01.00.00) > Cytokine and chemokine mediated signaling pathway(2.11.01.02.00) > G-protein mediated signaling (2.11.01.07.00)<br><br>Immunity and defense(2.16.00.00.00) > Cytokine/chemokine mediated immunity(2.16.10.00.00)<br>Cell structure and motility(2.27.00.00.00) > Cell motility(2.27.02.00.00)<br>MOLECULAR FUNCTIONS<br>Receptor(1.01.00.00.00) > G-protein coupled receptor(1.01.01.00.00)<br><br>HUMAN GENE ONTOLOGY<br>    BIOLOGICAL PROCESS<br>    cell motility > chemotaxis<br>    cell surface receptor linked signal transduction > G protein linked receptor protein signaling pathway<br>    defence response > inflammatory response<br>    cell growth and maintenance > invasive growth<br>    defence response > cellular defense response<br>    MOLECULAR FUNCTION<br>    enzyme inhibitor > protein kinase inhibitor<br>    defense/immunity protein > antiviral response protein<br>    enzyme > 2-acetyl-1-alkylglycerophosphocholine esterase<br>    transcription factor > RNA polymerase II transcription factor<br>    defense/immunity protein > blood coagulation factor<br>    CELL COMPONENT<br>    cell > membrane fraction<br>    cell > plasma membrane<br>    plasma membrane > integral plasma membrane protein<br>    cytoplasm > endosome<br>    cell > cytoplasm<br>HUMAN PROTEIN DOMAINS (INTERPRO SIGNATURES)<br>    IPR001277 (CXCCHMKINER4)<br>    IPR001718 (CHEMOKINER7)<br>    IPR000276 (GPCRRHODOPSN)<br>    IPR000355 (CCCHEMOKINER)<br>    IPR000276 (7tm 1)<br>    IPR000276 (G PROTEIN RECEP F12)<br>    IPR000276 (G PROTEIN RECEP F 11)<br>    IPR001064 (CRYSTALLIN BETAGAMMA) |
| hP3-010.1 | SEQ ID NO: 12 | HUMAN PANTHER CLASSIFICATIONS<br>    FAMILY (SUBFAMILY)<br>    COLLAGEN/INTEGRIN-RELATED(MATRILIN-4)<br>    BIOLOGICAL PROCESS<br>    Biological process unclassified(2.99.00.00.00)<br>    MOLECULAR FUNCTIONS<br>    Extracellular matrix(1.27.00.00.00) > Extracellular matrix structural protein(1.27.01.00.00)<br><br>HUMAN GENE ONTOLOGY<br>    BIOLOGICAL PROCESS |

(continued)

| Human Protein | SEQ ID NO: | FUNCTION |
|---|---|---|
| | | cell adhesion > cell-cell matrix adhesion |
| | | skeletal development > cartilage condensation |
| | | cell communication > cell adhesion |
| | | mesoderm development > skeletal development |
| | | ectoderm development > epidermal differentiation |
| | | MOLECULAR FUNCTION |
| | | ligand binding or carrier > calcium binding |
| | | protein binding > collagen binding |
| | | transmembrane receptor > cell adhesion receptor |
| | | cell adhesion > cell adhesion receptor |
| | | molecular_function unknown > lymphocyte antigen |
| | | proteinase inhibitor > serine protease inhibitor |
| | | CELL COMPONENT |
| | | cell > membrane fraction |
| | | GO cellular component > extracellular |
| | | integral plasma membrane protein > integrin |
| | | extracellular > extracellular matrix |
| | | fibrillar collagen > collagen type III |
| | | HUMAN PROTEIN DOMAINS (INTERPRO SIGNATURES) |
| | | IPR002035 (VWFADOMAIN) |
| | | IPR000561 (EGF 2) |
| | | IPR001881 (EGF CA) |
| | | IPR002035 (VWA) |
| | | IPR000561 (EGF) |
| | | IPR000561 (EGF) |
| | | IPR002035 (vwa) |
| | | IPR002035 (VWFA 2) |
| | | IPR001881 (EGF CA 2) |
| | | IPR000152 (ASX HYDROXYL) |
| hP3-027.1 | SEQ ID NO: 18 | HUMAN PANTHER CLASSIFICATIONS |
| | | FAMILY (SUBFAMILY) |
| | | C-C CHEMOKINE RECEPTOR-RELATED(C-X-C CHEMOKINE RECEPTOR TYPE 5) |
| | | BIOLOGICAL PROCESS |
| | | Signal transduction(2.11.00.00.00) > Cell surface receptor mediated signal transduction(2.11.01.00.00) > Cytokine and chemokine mediated signaling pathway(2.11.01.02.00) > G-protein mediated signaling (2.11.01.07.00) |
| | | Immunity and defense(2.16.00.00.00) > Cytokine/chemokine mediated immunity(2.16.10.00.00) |
| | | Immunity and defense(2.16.00.00.00) > B-cell- and antibody-mediated immunity(2.16.02.00.00) |
| | | Cell structure and motility(2.27.00.00.00) > Cell motility(2.27.02.00.00) |
| | | MOLECULAR FUNCTIONS |
| | | Receptor(1.01.00.00.00) > G-protein coupled receptor(1.01.01.00.00) |
| | | |
| | | HUMAN GENE ONTOLOGY |
| | | BIOLOGICAL PROCESS |
| | | cell motility > chemotaxis |

(continued)

| Human Protein | SEQ ID NO: | FUNCTION |
|---|---|---|
| | | cell surface receptor linked signal transduction > G protein linked receptor protein signaling pathway<br>defence response > inflammatory response<br>cell growth and maintenance > invasive growth<br>defence response > cellular defense response<br>MOLECULAR FUNCTION<br>enzyme inhibitor > protein kinase inhibitor<br>enzyme > 2-acetyl-1-alkylglycerophosphocholine esterase<br>defense/immunity protein > antiviral response protein<br>defense/immunity protein > blood coagulation factor<br>1-phosphatidylinositol 3-kinase > 1-phosphatidylinositol 3-kinase regulator<br>CELL COMPONENT<br>cell > membrane fraction<br>cell > plasma membrane<br>plasma membrane > integral plasma membrane protein<br>cytoplasm > endosome<br>cell > cytoplasm<br>HUMAN PROTEIN DOMAINS (INTERPRO SIGNATURES)<br>IPR001053 (BURKITTSLYMR)<br>IPR001277 (CXCCHIVBCINER4)<br>IPR000276 (GPCRRHODOPSN)<br>IPR000248 (ANGIOTENSINR)<br>IPR000276 (7tm 1)<br>IPR000276 (G PROTEIN RECEP F1 2)<br>IPR000276 (G PROTEIN RECEP F1 1) |
| hP3-045.1 | SEQ ID NO: 24 | HUMAN PANTHER CLASSIFICATIONS<br>FAMILY (SUBFAMILY)<br>FRIZZLED-RELATED(FRIZZLED)<br>BIOLOGICAL PROCESS<br>Signal transduction(2.11.00.00.00) > Cell surface receptor mediated signal transduction(2.11.01.00.00) > G-protein mediated signaling (2.11.01.07.00)<br>Oncogenesis(2.17.00.00.00) > Other oncogenesis(2.17.99.00.00)<br>Developmental processes(2.23.00.00.00) > Other developmental process(2.23.99.00.00)<br>MOLECULAR FUNCTIONS<br>Receptor(1.01.00.00.00) > G-protein coupled receptor(1.01.01.00.00)<br><br>HUMAN GENE ONTOLOGY<br>BIOLOGICAL PROCESS<br>GO biological process > developmental processes<br>cytoskeleton organization and biogenesis > establishment of cell polarity<br>cell surface receptor linked signal transduction > fz receptor signaling pathway<br>cytoskeleton organization and biogenesis > establishment of tissue polarity<br>cell communication > signal transduction<br>MOLECULAR FUNCTION |

(continued)

| Human Protein | SEQ ID NO: | FUNCTION |
|---|---|---|
| | | mannosidase > beta-mannosidase |
| | | ligand binding or carrier > protein binding |
| | | GO molecular function > ligand binding or carrier |
| | | serine-type peptidase > serine-type endopeptidase |
| | | endopeptidase > serine-type endopeptidase |
| | | metalloexopeptidase > metallocarboxypeptidase |
| | | carboxypeptidase > metallocarboxypeptidase |
| | | CELL COMPONENT |
| | | cell > membrane fraction |
| | | cell > plasma membrane |
| | | plasma membrane > integral plasma membrane protein |
| | | GO cellular component > extracellular |
| | | extracellular > extracellular space HUMAN PROTEIN DOMAINS (INTERPRO SIGNATURES) |
| | |     IPR000539 (FRIZZLED) |
| | |     IPR000024 (FRI) |
| | |     IPR000024 (Fz) |
| | |     IPR000539 (Frizzled) |
| | |     IPR000832 (G PROTEIN RECEP F2 4) |
| | |     IPR000024 (FZ DOMAIN) |
| hP5-001.1 | SEQ ID NO: 30 | HUMAN PANTHER CLASSIFICATIONS |
| | |     FAMILY (SUBFAMILY) |
| | |     CYTOKINE RECEPTOR(Unassigned) |
| | |     BIOLOGICAL PROCESS |
| | |     Biological process unclassified(2.99.00.00.00) |
| | |     MOLECULAR FUNCTIONS |
| | |     Molecular function unclassified(1.97.00.00.00) |
| | | |
| | | HUMAN GENE ONTOLOGY |
| | |     BIOLOGICAL PROCESS |
| | |     cell communication > signal transduction |
| | |     signal transduction > cell surface receptor linked signal transduction |
| | |     protein metabolism and modification > protein complex assembly |
| | |     defence response > immune response |
| | |     cell growth and maintenance > cell proliferation |
| | |     MOLECULAR FUNCTION |
| | |     transcription factor > transcription activating factor |
| | |     molecular_function unknown > lymphocyte antigen |
| | |     CELL COMPONENT |
| | |     cell > membrane fraction |
| | |     cell > plasma membrane |
| | |     plasma membrane > integral plasma membrane protein |
| | |     extracellular > extracellular space |
| | | HUMAN PROTEIN DOMAINS (INTERPRO SIGNATURES) |
| | |     IPR001777 (FN3) |
| | |     IPR002996 (CR1A) |
| | |     IPR000694 (PRO RICH) |
| | |     IPR003531 (HEMATOPO REC S F1) |
| hP5-002.1 | SEQ ID NO: 36 | HUMAN PANTHER CLASSIFICATIONS |

(continued)

| Human Protein | SEQ ID NO: | FUNCTION |
|---|---|---|
| | | FAMILY (SUBFAMILY) |
| | | CYTOKINE RECEPTOR(Unassigned) |
| | | BIOLOGICAL PROCESS |
| | | Biological process unclassified(2.99.00.00.00) |
| | | MOLECULAR FUNCTIONS |
| | | Molecular function unclassified(1.97.00.00.00) |
| | | |
| | | HUMAN GENE ONTOLOGY |
| | | BIOLOGICAL PROCESS |
| | | humoral defense mechanism > antimicrobial response |
| | | cell communication > signal transduction |
| | | signal transduction > cell surface receptor linked signal transduction |
| | | defence response > immune response |
| | | MOLECULAR FUNCTION |
| | | molecular_function unknown > lymphocyte antigen |
| | | transcription factor > transcription activating factor |
| | | CELL COMPONENT |
| | | cell > membrane fraction |
| | | cell > plasma membrane |
| | | plasma membrane > integral plasma membrane protein |
| | | extracellular > extracellular space |
| | | integral plasma membrane protein > interleukin-13 receptor |
| | | HUMAN PROTEIN DOMAINS (INTERPRO SIGNATURES) |
| | | IPR001777 (FN3) |
| | | IPR001777 (fn3) |
| | | IPR000282 (CR2A) |
| | | IPR002996 (CR1A 2) |
| | | IPR000694 (PRO RICH) |
| | | IPR003531 (HEMATOPO REC S F1) |
| hP5-030.1 | SEQ ID NO: 42 | HUMAN PANTHER CLASSIFICATIONS |
| | | FAMILY (SUBFAMILY) |
| | | FRIZZLED-RELATED(FRIZZLED) |
| | | BIOLOGICAL PROCESS |
| | | Signal transduction(2.11.00.00.00) > Cell surface receptor mediated signal transduction(2.11.01.00.00) > G-protein mediated signaling (2.11.01.07.00) |
| | | Oncogenesis(2.17.00.00.00) > Other oncogenesis(2.17.99.00.00) |
| | | Developmental processes(2.23.00.00.00) > Other developmental process(2.23.99.00.00) |
| | | MOLECULAR FUNCTIONS |
| | | Receptor(1.01.00.00.00) > G-protein coupled receptor(1.01.01.00.00) |
| | | |
| | | HUMAN GENE ONTOLOGY |
| | | BIOLOGICAL PROCESS |
| | | cell surface receptor linked signal transduction > fz receptor signaling pathway |
| | | cytoskeleton organization and biogenesis > establishment of tissue polarity |
| | | cell communication > signal transduction |
| | | GO biological process > developmental processes |

| Human Protein | SEQ ID NO: | FUNCTION |
|---|---|---|
| | | cytoskeleton organization and biogenesis > establishment of cell polarity<br>MOLECULAR FUNCTION<br>mannosidase > beta-mannosidase<br>ligand binding or carrier > protein binding<br>GO molecular function > ligand binding or carrier<br>serine-type peptidase > serine-type endopeptidase<br>endopeptidase > serine-type endopeptidase<br>metalloexopeptidase > metallocarboxypeptidase<br>carboxypeptidase > metallocarboxypeptidase<br>CELL COMPONENT<br>cell > membrane fraction<br>cell > plasma membrane<br>plasma membrane > integral plasma membrane protein<br>GO cellular component > extracellular<br>extracellular > extracellular space<br>HUMAN PROTEIN DOMAINS (INTERPRO SIGNATURES)<br>IPR000539 (FRIZZLED)<br>IPR000024 (FRI)<br>IPR000024 (Fz) IPR000539 (Frizzled)<br>IPR000832 (G PROTEIN RECEP F2 4)<br>IPR000024 (FZ DOMAIN) |
| hP5-033.1 | SEQ ID NO: 48. | HUMAN PANTHER CLASSIFICATIONS<br>FAMILY (SUBFAMILY)<br>GALANIN RECEPTOR-RELATED(Llnassigned)<br>BIOLOGICAL PROCESS<br>Biological process unclassified(2.99.00.00.00)<br>MOLECULAR FUNCTIONS<br>Molecular function unclassified(1.97.00.00.00)<br><br>HUMAN GENE ONTOLOGY<br>BIOLOGICAL PROCESS<br>cell surface receptor linked signal transduction > G protein linked receptor protein signaling pathway<br>behavior > feeding behavior<br>G protein linked receptor protein signaling pathway > G protein signaling, linked to cyclic nucleotide second messenger<br>G protein linked receptor protein signaling pathway > tachykinin signaling pathway<br>cell-cell signaling > synaptic transmission<br>MOLECULAR FUNCTION<br>1-phosphatidylinositol 3-kinase > 1-phosphatidylinositol 3-kinase regulator<br>enzyme > 2-acetyl-1-alkylglycerophosphocholine esterase<br>amine oxidase > amine oxidase (flavin-containing)<br>defense/immunity protein > blood coagulation factor<br>CELL COMPONENT<br>cell > membrane fraction<br>cytoplasm > lysosome<br>cell > plasma membrane |

(continued)

| Human Protein | SEQ ID NO: | FUNCTION |
|---|---|---|
| | | plasma membrane > integral plasma membrane protein<br>cytoplasm > endosome<br>HUMAN PROTEIN DOMAINS (INTERPRO SIGNATURES)<br>    IPR000276 (GPCRRHODOPSN)<br>    IPR000276 (7tm 1)<br>    IPR000276 (G PROTEIN RECEP F1 2) |
| hP5-034.1 | SEQ ID NO: 54 | HUMAN PANTHER CLASSIFICATIONS<br>    FAMILY (SUBFAMILY)<br>    C-C CHEMOKINE RECEPTOR-<br>RELATED(C-X-C CHEMOKINE RECEPTOR TYPE 3)<br>    BIOLOGICAL PROCESS<br>    Signal transduction(2.11.00.00.00) > Cell surface receptor mediated signal transduction(2.11.01.00.00) > Cytokine and chemokine mediated signaling pathway(2.11.01.02.00) > G-protein mediated signaling (2.11.01.07.00)<br>    Immunity and defense(2.16.00.00.00) > Cytokine/chemokine mediated immunity(2.16.10.00.00)<br>    Cell structure and motility(2.27.00.00.00) > Cell motility(2.27.02.00.00)<br>    MOLECULAR FUNCTIONS<br>    Receptor(1.01.00.00.00) > G-protein coupled receptor(1.01.01.00.00)<br><br>HUMAN GENE ONTOLOGY<br>    BIOLOGICAL PROCESS<br>    cell motility > chemotaxis<br>    cell surface receptor linked signal transduction > G protein linked receptor protein signaling pathway<br>    defence response > inflammatory response<br>    cell growth and maintenance > invasive growth<br>    defence response > cellular defense response<br>    MOLECULAR FUNCTION<br>    enzyme inhibitor > protein kinase inhibitor<br>    enzyme > 2-acetyl-1-alkylglycerophosphocholine esterase<br>    defense/immunity protein > antiviral response protein<br>    defense/immunity protein > blood coagulation factor<br>    DNA repair enzyme > deoxyribodipyrimidine photolyase<br>    DNA photolyase > deoxyribodipyrimidine photolyase<br>    CELL COMPONENT<br>    cell > membrane fraction<br>    cell > plasma membrane<br>    plasma membrane > integral plasma membrane protein |
| | | cytoplasm > endosome<br>cell > cytoplasm<br>HUMAN PROTEIN DOMAINS (INTERPRO SIGNATURES)<br>    IPR001277 (CXCCHMKINER4)<br>    IPR000276 (GPCRRHODOPSN)<br>    IPR000355 (CCCHEMOKINER)<br>    IPR000496 (BRADYKININR)<br>    IPR000248 (ANGIOTENSINR)<br>    IPR000276 (7tm 1) |

(continued)

| Human Protein | SEQ ID NO: | FUNCTION |
|---|---|---|
| | | IPR000276 (G PROTEIN RECEP F1 2)<br>IPR000276 (G PROTEIN RECEP F1 1) |
| hP6-034.1 | SEQ ID NO: 60 | HUMAN PANTHER CLASSIFICATIONS<br>    FAMILY (SUBFAMILY)<br>    PROSTAGLANDIN RECEPTOR-RELATED(THROMBOXANE A2 RECEPTOR)<br>    BIOLOGICAL PROCESS<br>    Signal transduction(2.11.00.00.00) > Cell surface receptor mediated signal transduction(2.11.01.00.00) > G-protein mediated signaling (2.11.01.07.00)<br>    Immunity and defense(2.16.00.00.00)<br>    Blood clotting(2.20.00.00.00)<br>    MOLECULAR FUNCTIONS<br>    Receptor(1.01.00.00.00) > G-protein coupled receptor(1.01.01.00.00)<br><br>HUMAN GENE ONTOLOGY<br>    BIOLOGICAL PROCESS<br>    cell surface receptor linked signal transduction > G protein linked receptor protein signaling pathway<br>    cell growth and maintenance > cell death<br>    cell motility > muscle contraction<br>    neurogenesis > central nervous system development<br>    G protein signaling, linked to cyclic nucleotide second messenger > G protein signaling, linked to cAMP nucleotide second messenger<br>    MOLECULAR FUNCTION<br>    enzyme > nitric oxide synthase<br>    GO molecular function > cell cycle regulator<br>    nucleic acid binding > DNA binding<br>    nucleotide binding > ATP binding<br>    DNA binding > transcription factor<br>    CELL COMPONENT<br>    cell > membrane fraction<br>    cell > plasma membrane<br>    plasma membrane > integral plasma membrane protein<br>    GO cellular component > extracellular<br>    nucleus > nuclear membrane HUMAN PROTEIN DOMAINS (INTERPRO SIGNATURES)<br>    IPR001105 (THROMBOXANER)<br>    IPR000276 (GPCRRHODOPSN)<br>    IPR000276 (7tm 1)<br>    IPR000276 (G PROTEIN RECEP F1 2)<br>    IPR000276 (G PROTEIN RECEP F1 1) |
| hP6-034.2 | SEQ ID NO:62 | HUMAN PANTHER CLASSIFICATIONS<br>    FAMILY (SUBFAMILY)<br>    PROSTAGLANDIN RECEPTOR-RELATED(THROMBOXANE A2 RECEPTOR)<br>    BIOLOGICAL PROCESS<br>    Signal transduction(2.11.00.00.00) > Cell surface receptor mediated signal transduction(2.11.01.00.00) > G-protein mediated signaling (2.11.01.07.00) |

| Human Protein | SEQ ID NO: | FUNCTION |
|---|---|---|
| | | Immunity and defense(2.16.00.00.00) |
| | | Blood clotting(2.20.00.00.00) |
| | | MOLECULAR FUNCTIONS |
| | | Receptor(1.01.00.00.00) > G-protein coupled receptor(1.01.01.00.00) |
| | | |
| | | HUMAN GENE ONTOLOGY |
| | | BIOLOGICAL PROCESS |
| | | neurogenesis > central nervous system development |
| | | cell surface receptor linked signal transduction > G protein linked receptor protein signaling pathway |
| | | cell growth and maintenance > cell death |
| | | cell motility > muscle contraction |
| | | G protein signaling, linked to cyclic nucleotide second messenger > G protein signaling, linked to cAMP nucleotide second messenger |
| | | MOLECULAR FUNCTION |
| | | receptor > ligand-dependent nuclear receptor |
| | | GO molecular function > apoptosis inhibitor |
| | | molecular_function unknown > minor histocompatibility antigen |
| | | enzyme > nitric oxide synthase |
| | | GO molecular function > cell cycle regulator |
| | | ligand-regulated transcription factor > ligand-dependent nuclear receptor |
| | | CELL COMPONENT |
| | | cell > membrane fraction |
| | | cell > plasma membrane |
| | | plasma membrane > integral plasma membrane protein |
| | | GO cellular component > extracellular |
| | | nucleus > nuclear membrane |
| | | HUMAN PROTEIN DOMAINS (INTERPRO SIGNATURES) |
| | | IPR001105 (THROMBOXANER) |
| | | IPR000276 (GPCRRHODOPSN) |
| | | IPR000276 (7tm 1) |
| hP6-131.1 | SEQ ID NO: 68 | HUMAN PANTHER CLASSIFICATIONS |
| | | FAMILY (SUBFM4ILY) |
| | | CF11294(LYSOSPHINGOLIPID RECEPTOR-RELATED) |
| | | BIOLOGICAL PROCESS |
| | | Signal transduction(2.11.00.00.00) > Intracellular signaling cascade (2.11.02.00.00) > Calcium mediated signaling(2.11.02.02.00) |
| | | Signal transduction(2.11.00.00.00) > Cell communication (2.11.03.00.00) > Ligand-mediated signaling(2.11.03.03.00) |
| | | Signal transduction(2.11.00.00.00) > Cell surface receptor mediated signal transduction(2.11.01.00.00) > G-protein mediated signaling (2.11.01.07.00) |
| | | Neuronal activities(2.18.00.00.00) > Other neuronal activity (2.18.99.00.00) |
| | | Developmental processes(2.23.00.00.00) > Ectoderm development (2.23.08.00.00) > Neurogenesis(2.23.08.01.00) |
| | | MOLECULAR FUNCTIONS |
| | | Receptor(1.01.00.00.00) > G-protein coupled receptor(1.01.01.00.00) |

(continued)

| Human Protein | SEQ ID NO: | FUNCTION |
|---|---|---|
| | | HUMAN GENE ONTOLOGY<br>    BIOLOGICAL PROCESS<br>    cell surface receptor linked signal transduction > G protein linked receptor protein<br>signaling pathway<br>    G protein linked receptor protein signaling pathway > G protein signaling, linked to cyclic nucleotide second messenger<br>    sensory perception > vision<br>    vision > phototransduction<br>    intracellular signaling cascade > protein kinase cascade<br>    MOLECULAR FUNCTION<br>    ligand binding or carrier > lipid binding<br>    amine oxidase > amine oxidase (flavin-containing)<br>    protein binding > lipoprotein binding<br>    monooxygenase > monophenol monooxygenase<br>    enzyme > 2-acetyl-1-alkylglycerophosphocholine esterase<br>    CELL COMPONENT<br>    cell > membrane fraction<br>    cytoplasm > lysosome<br>    cell > plasma membrane<br>    plasma membrane > integral plasma membrane protein<br>    cell > cytoplasm<br>HUMAN PROTEIN DOMAINS (INTERPRO SIGNATURES)<br>    IPR001671 (MCRFAMILY)<br>    IPR000276 (GPCRRHODOPSN)<br>    IPR000987 (EDG1ORPHANR)<br>    IPR002230 (CANNABINOIDR)<br>    IPR000276 (7tm 1)<br>    IPR000276 (G PROTEIN RECEP F 1 2)<br>    IPR000276 (G PROTEIN RECEP F1 1) |
| hP7-046.1 | SEQ ID NO: 74 | HUMAN PANTHER CLASSIFICATIONS<br>    FAMILY (SUBFAMILY)<br>    CF10336(PHOSPHOLIPASE C-BETA-4)<br>    BIOLOGICAL PROCESS<br>    Lipid, fatty acid and steroid metabolism(2.03.00.00.00) > Phospholipid metabolism(2.03.04.00.00)<br>    Signal transduction(2.11.00.00.00) > Cell surface receptor mediated signal transduction(2.11.01.00.00) > G-protein mediated signaling (2.11.01.07.00)<br>    Sensory perception(2.22.00.00.00) > Vision(2.22.03.00.00)<br>    MOLECULAR FUNCTIONS<br>    Hydrolase(1.21.00.00.00) ><br>    Lipase(1.21.01.00.00) ><br>    Phospholipase(1.21.01.01.00)<br><br>HUMAN GENE ONTOLOGY<br>    BIOLOGICAL PROCESS<br>    lipid metabolism > membrane lipid metabolism<br>    signal transduction > intracellular signaling cascade<br>    nucleotide metabolism > lipid metabolism |

(continued)

| Human Protein | SEQ ID NO: | FUNCTION |
|---|---|---|
| | | cell communication > signal transduction |
| | | membrane lipid metabolism > phospholipid metabolism |
| | | MOLECULAR FUNCTION |
| | | phospholipase A2 > phospholipase C |
| | | inositol/phosphatidylinositol phosphodiesterase > 1-phosphatidylinositol-4,5-biphosphate phosphodiesterase |
| | | ligand binding or carriers> calcium binding |
| | | nucleotide binding > ATP binding |
| | | GO molecular function > motor |
| | | CELL COMPONENT |
| | | actin cytoskeleton > non-muscle myosin |
| | | cell > nucleus |
| | | mitochondrial membrane > mitochondrial outer membrane |
| | | GO cellular component > cellular_component unknown |
| | | cell > cytoplasm |
| | | HUMAN PROTEIN DOMAINS (INTERPRO SIGNATURES) |
| | | IPR001711 (sp Q9Z0G6 Q9Z0G6 RAT) |
| | | IPR000909 (PLCXc) |
| | | IPR001711 (PLCYc) |
| | | IPR000008 (C2) |
| | | IPR001687 (ATP GTP A) |
| | | IPR001711 (FIPLC Y DOMAIN) |
| | | NULL (LYS RICH) |
| | | IPR000008 (C2 DOMAIN 2) |
| | | IPR000909 (PIPLC X DOMAIN) |
| | | IPR001711 (PI-PLC-Y) |
| | | IPR000909 (PI-PLC-X) |
| | | IPR000008 (C2) |
| | | IPR001192 (PHPHLIPASEC) |
| hP7-046.2 | SEQ ID NO: 76 | HUMAN PANTHER CLASSIFICATIONS |
| | | FAMILY (SUBFAMILY) |
| | | CF10336(PHOSPHOLIPASE C-BETA-4) |
| | | BIOLOGICAL PROCESS |
| | | Lipid, fatty acid and steroid metabolism(2.03.00.00.00) > Phospholipid metabolism(2.03.04.00.00) |
| | | Signal transduction(2.11.00.00.00) > Cell surface receptor mediated signal transduction(2.11.01.00.00) > G-protein mediated signaling (2.11.01.07.00) |
| | | Sensory perception(2.22.00.00.00) > Vision(2.22.03.00.00) |
| | | MOLECULAR FUNCTIONS |
| | | Hydrolase(1.21.00.00.00) > Lipase(1.21.01.00.00) > Phospholipase (1.21.0 1.01.00) |
| | | |
| | | HUMAN GENE ONTOLOGY |
| | | No Gene Ontology |
| | | HUMAN PROTEIN DOMAINS (INTERPRO SIGNATURES) |
| | | IPR001711 (sp Q9Z0G6 Q9Z0G6 RAT) |
| | | IPR000909 (PLCXc) |
| | | IPR001711 (PLCYc) |
| | | IPR000008 (C2) |

(continued)

| Human Protein | SEQ ID NO: | FUNCTION |
|---|---|---|
| | | IPR001687 (ATP GTP A)<br>IPR001711 (PIPLC Y DOMAIN)<br>NULL (LYS RICH)<br>IPR000008 (C2 DOMAIN 2)<br>IPR000909 (PIPLC X DOMAIN)<br>IPR001711 (PI-PLC-Y)<br>IPR000909 (PI-PLC-X)<br>IPR000008 (C2)<br>IPR001192 (PHPHLIPASEC) |
| bP7-104.1 | SEQ ID NO: 82 | HUMAN PANTHER CLASSIFICATIONS<br>    FAMILY (SUBFAMILY)<br>    CF11923(PLATELET GLYCOPROTEIN IV)<br>    BIOLOGICAL PROCESS<br>    Lipid, fatty acid and steroid metabolism(2.03.00.00.00) > LIPID AND FATTY ACID TRANSPORT(2.03.07.00.00)<br>    Signal transduction(2.11.00.00.00)<br>    Immunity and defense(2.16.00.00.00) > Macrophage-mediated immunity(2.16.05.00.00)<br>    Apoptosis(2.26.00.00.00) > Other apoptosis(2.26.99.00.00)<br>    Cell structure and motility(2.27.00.00.00) > Cell structure (2.27.01.00.00)<br>    MOLECULAR FUNCTIONS<br>    Receptor(1.01.00.00.00) > Other receptor(1.01.99.00.00)<br><br>HUMAN GENE ONTOLOGY<br>    BIOLOGICAL PROCESS<br>    steroid metabolism > cholesterol metabolism<br>    cell communication > cell adhesion<br>    lipid metabolism > fatty acid metabolism<br>    nucleotide metabolism > lipid metabolism<br>    MOLECULAR FUNCTION<br>    GO molecular function > cell adhesion<br>    mannosidase > beta-mannosidase<br>    CELL COMPONENT<br>    cell > membrane fraction<br>    cell > plasma membrane<br>    plasma membrane > integral plasma membrane protein<br>    lysosome > lysosomal membrane<br>    endoplasmic reticulum > endoplasmic reticulum lumen<br>HUMAN PROTEIN DOMAINS (INTERPRO SIGNATURE)<br>    IPR002159 (CD36) |
| hP7-157.1 | SEQ ID NO: 88 | HUMAN PANTHER CLASSIFICATIONS<br>    FAMILY (SUBFAMILY)<br>    LATROPHILIN-RELATED(G PROTEIN-COUPLED RECEPTOR 56)<br>    BIOLOGICAL PROCESS<br>    Signal transduction(2.11.00.00.00) > Cell surface receptor mediated signal transduction(2.11.01.00.00) > G-protein mediated signaling (2.11.01.07.00)<br>    MOLECULAR FUNCTIONS |

(continued)

| Human Protein | SEQ ID NO: | FUNCTION |
|---|---|---|
| | | Receptor(1.01.00.00.00) > G-protein coupled receptor(1.01.01.00.00)<br><br>HUMAN GENE ONTOLOGY<br>    BIOLOGICAL PROCESS<br>    cell surface receptor linked signal transduction > G protein linked receptor protein signaling pathway<br>        cell communication > cell adhesion<br>        cell communication > cell-cell signaling<br>        G protein linked receptor protein signaling pathway > neuropeptide signaling pathway<br>        neurogenesis > peripheral nervous system development<br>    MOLECULAR FUNCTION<br>    ligand binding or carrier > calcium binding<br>    ligand binding or carrier > protein binding<br>    molecular_function unknown > lymphocyte antigen<br>    calcium binding > calcium sensing<br>    CELL COMPONENT<br>    cell > membrane fraction<br>    cell > plasma membrane<br>    plasma membrane > integral plasma membrane protein<br>    plasma membrane > intercellular junction<br>HUMAN PROTEIN DOMAINS (INTERPRO SIGNATURES)<br>    IPR000203 (RECEPTOR PKD)<br>    IPR000832 (G PROTEIN RECEP F2 4)<br>    IPR000832 (7tm 2)<br>    IPR000203 (GPS)<br>    IPR000832 (GPCRSECRETIN)<br>    IPR003910 (GPR56ORPHANR)<br>    IPR000203 (GPS)<br>    IPR000873 (AMP BINDING) |
| hP7-157.2 | SEQ ID NO: 90 | HUMAN PANTHER CLASSIFICATIONS<br>    FAMILY (SUBFAMILY)<br>    LATROPHILIN-RELATED(G PROTEIN-COUPLED RECEPTOR 56)<br>    BIOLOGICAL PROCESS<br>    Signal transduction(2.11.00.00.00) > Cell surface receptor mediated signal transduction(2.11.01.00.00) > G-protein mediated signaling (2.11.01.07.00)<br>    MOLECULAR FUNCTIONS<br>    Receptor(1.01.00.00.00) > G-protein coupled receptor(1.01.01.00.00)<br><br>HUMAN GENE ONTOLOGY<br>    No Gene Ontology<br>HUMAN PROTEIN DOMAINS (INTERPRO SIGNATURES)<br>    IPR000203 (RECEPTOR PKD)<br>    IPR000832 (G PROTEIN RECEP F2 4)<br>    IPR000832 (7tm 2)<br>    IPR000203 (GPS)<br>    IPR000832' (GPCRSECRETIN)<br>    IPR003910 (GPR560RPHANR)<br>    IPR000203 (GPS) |

(continued)

| Human Protein | SEQ ID NO: | FUNCTION |
|---|---|---|
| | | IPR000873 (AMP BINDING) |
| hP7-157.3 | SEQ ID NO: 92 | HUMAN PANTHER CLASSIFICATIONS<br>    FAMILY (SUBFAMILY)<br>    LATROPHILIN-RELATED(G PROTEIN-COUPLED RECEPTOR 56)<br>    BIOLOGICAL PROCESS<br>    Signal transduction(2.11.00.00.00) > Cell surface receptor mediated signal transduction(2.11.01.00.00) > G-protein mediated signaling (2.11.01.07.00)<br>    MOLECULAR FUNCTIONS<br>    Receptor(1.01.00.00.00) > G-protein coupled receptor(1.01.01.00.00)<br><br>HUMAN GENE ONTOLOGY<br>    No Gene Ontology<br>HUMAN PROTEIN DOMAINS (INTERPRO SIGNATURES)<br>    IPR000203 (RECEPTOR PKD)<br>    IPR000832 (G PROTEIN RECEP F2 4)<br>    IPR000832 (7tm 2)<br>    IPR000203 (GPS)<br>    IPR000832 (GPCRSECRETIN)<br>    IPR003910 (GPR56ORPHANR)<br>    IPR000203 (GPS)<br>    IPR000873 (AMP BINDING) |
| hP7-157.4. | SEQ ID NO: 94 | HUMAN PANTHER CLASSIFICATIONS<br>    FAMILY (SUBFAMILY)<br>    LATROPHILIN-RELATED(G PROTEIN-COUPLED RECEPTOR 56)<br>    BIOLOGICAL PROCESS<br>    Signal transduction(2.11.00.00.00) > Cell surface receptor mediated signal transduction(2.11.01.00.00) > G-protein mediated signaling (2.11.01.07.00)<br>    MOLECULAR FUNCTIONS<br>    Receptor(1.01.00.00.00) > G-protein coupled receptor(1.01.01.00.00)<br><br>HUMAN GENE ONTOLOGY<br>    No Gene Ontology<br>HUMAN PROTEIN DOMAINS (INTERPRO SIGNATURES)<br>    IPR000203 (RECEPTOR PKD)<br>    IPR000832 (G PROTEIN RECEP F2 4)<br>    IPR000832 (7tm 2)<br>    IPR000203 (GPS)<br>    IPR000832 (GPCRSECRETIN)<br>    IPR003910 (GPR56ORPHANR)<br>    IPR000203 (GPS)<br>IPR000873 (AMP BINDING) |
| hP7-161.1 | SEQ ID NO: 100 | HUMAN PANTHER CLASSIFICATIONS<br>    FAMILY (SUBFAMILY)<br>    CF11294(MELANOCORTIN-1 RECEPTOR)<br>    BIOLOGICAL PROCESS<br>    Signal transduction(2.11.00.00.00) > Intracellular signaling cascade (2.11.02.00.00) > NF-kappaB cascade(2.11.02.04.00) |

(continued)

| Human Protein | SEQ ID NO: | FUNCTION |
|---|---|---|
| | | Signal transduction(2.11.00.00.00) > Cell surface receptor mediated signal transduction(2.1 1.0 1.00.00) > G-protein mediated signaling (2.11.01.07.00)<br>Immunity and defense(2.16.00.00.00) > Macrophage-mediated immunity(2.16.05.00.00)<br>Immunity and defense(2.16.00.00.00) > Granulocyte-mediated immunity(2.16.04.00.00)<br>Developmental processes(2.23.00.00.00) > Ectoderm development (2.23.08.00.00)<br>MOLECULAR FUNCTIONS<br>Receptor(1.01.00.00.00) > G-protein coupled receptor(1.01.01.00.00)<br><br>HUMAN GENE ONTOLOGY<br>BIOLOGICAL PROCESS<br>G protein linked receptor protein signaling pathway > G protein signaling, linked to cyclic nucleotide second messenger<br>cell surface receptor linked signal transduction > G protein linked receptor protein signaling pathway<br>G protein signaling, linked to cyclic nucleotide second messenger > G protein signaling, linked to cAMP nucleotide second messenger<br>behavior > feeding behavior<br>MAPKKK cascade > activation of MAPK<br>MOLECULAR FUNCTION<br>ligand binding or carrier > lipid binding<br>amine oxidase > amine oxidase (flavin-containing)<br>enzyme > guanylate kinase<br>monooxygenase > monophenol monooxygenase<br>CELL COMPONENT<br>cell > membrane fraction<br>cell > plasma membrane<br>plasma membrane > integral plasma membrane protein<br>cytoplasm > endosome<br>HUMAN PROTEIN DOMAINS (INTERPRO SIGNATURES)<br>IPR000276 (G PROTEIN RECEP F 1 2)<br>IPR000276 (7tm 1)<br>IPR001671 (MCRFAMILY)<br>IPR001908 (MELNOCORTINR)<br>IPR000276 (GPCRRHODOPSN)<br>IPR000761 (MELNOCYTESHR)<br>IPR000276 (G PROTEIN RECEP F 1 1) |
| hP13-023.1 | SEQ ID NO: 106 | HUMAN PANTHER CLASSIFICATIONS<br>FAMILY (SUBFAMILY)<br>NEUROTENSIN RECEPTOR-RELATED(G PROTEIN-COUPLED RECEPTOR)<br>BIOLOGICAL PROCESS<br>Signal transduction(2.11.00.00.00) > Cell surface receptor mediated signal transduction(2.1 1.0 1.00.00) > G-protein mediated signaling (2.11.01.07.00)<br>MOLECULAR FUNCTIONS |

(continued)

| Human Protein | SEQ ID NO: | FUNCTION |
|---|---|---|
| | | Receptor(1.01.00.00.00) > G-protein coupled receptor(1.01.01.00.00)<br><br>HUMAN GENE ONTOLOGY<br>　BIOLOGICAL PROCESS<br>　G protein signaling, linked to cAMP nucleotide second messenger > G protein signaling, adenylate cyclase inhibiting pathway<br>　G protein linked receptor protein signaling pathway > tachykinin signaling pathway<br>　G protein linked receptor protein signaling pathway > G protein signaling, linked to cyclic nucleotide second messenger<br>　cell surface receptor linked signal transduction > G protein linked receptor protein signaling pathway<br>　behavior > feeding behavior<br>　MOLECULAR FUNCTION<br>　enzyme > 2-acetyl-1-alkylglycerophosphocholine esterase<br>　1-phosphatidylinositol 3-kinase > 1-phosphatidylinositol 3-kinase regulator<br>　transcription factor > RNA polymerase II transcription factor<br>　enzyme inhibitor > protein kinase inhibitor<br>　protein binding > lipoprotein binding<br>　CELL COMPONENT<br>　cell > membrane fraction<br>　cell > plasma membrane<br>　plasma membrane > integral plasma membrane protein<br>　cytoplasm > endoplasmic reticulum<br>　cytoplasm > Golgi apparatus<br>HUMAN PROTEIN DOMAINS (INTERPRO SIGNATURES)<br>　IPR000276 (GPCRRHODOPSN)<br>　IPR000276 (7tm 1)<br>　IPR000276 (G PROTEIN RECEP F1 2)<br>　IPR000276 (G PROTEIN RECEP F 1 1) |
| hPl4-023.1 | SEQ ID NO: 112 | HUMAN PANTHER CLASSIFICATIONS<br>　FAMILY (SUBFAMILY)<br>　NUCLEOPLASMIN(NUCLEOPHOSMI N)<br>　BIOLOGICAL PROCESS<br>　Nucleoside, nucleotide and nucleic acid metabolism(2.04.00.00.00) > rRNA metabolism(2.04.08.00.00)<br>　MOLECULAR FUNCTIONS<br>　Molecular function unclassified(1.97.00.00.00)<br><br>HUMAN GENE ONTOLOGY<br>　No Gene Ontology<br>HUMAN PROTEIN DOMAINS (INTERPRO SIGNATURES)<br>　IPR004301 (Nucleoplasmin)<br>　NULL (ASP RICH) |
| hP14-023.2 | SEQ ID NO: 114 | HUMAN PANTHER CLASSIFICATIONS<br>　FAMILY (SUBFAMILY)<br>　NUCLEOPLASM1N(NUCLEOPHOSMI N)<br>　BIOLOGICAL PROCESS<br>　Nucleoside, nucleotide and nucleic acid |

(continued)

| Human Protein | SEQ ID NO: | FUNCTION |
|---|---|---|
| | | metabolism(2.04.00.00.00) > rRNA metabolism(2.04.08.00.00)<br>    MOLECULAR FUNCTIONS<br>    Molecular function unclassified(1.97.00.00.00)<br><br>HUMAN GENE ONTOLOGY<br>    BIOLOGICAL PROCESS<br>    cell communication > signal transduction<br>    signal transduction > cell surface receptor linked signal transduction<br>    humoral defense mechanism > antimicrobial response<br>    defence response > cellular defense response<br>    intracellular protein traffic > endocytosis<br>    MOLECULAR FUNCTION<br>    sugar binding > lectin<br>    nucleic acid binding > RNA binding<br>    molecular_function unknown > lymphocyte antigen<br>    DNA binding > transcription factor<br>    lectin > mannose binding lectin<br>    mannose binding > mannose binding lectin<br>    CELL COMPONENT<br>    cell > membrane fraction<br>    cell > nucleus<br>    cell > plasma membrane<br>    plasma membrane > integral plasma membrane protein<br>    nucleus > nucleolus<br>HUMAN PROTEIN DOMAINS (INTERPRO SIGNATURES)<br>    IPR004301 (Nucleoplasmin)<br>    NULL (ASP RICH) |
| hP14-023.3 | SEQ ID NO: 116 | HUMAN PANTHER CLASSIFICATIONS<br>    FAMILY (SUBFAMILY)<br>    NKG2 TYPE II INTEGRAL<br>    MEMBRANE PROTEIN(C-TYPE LECTIN-RELATED)<br>    BIOLOGICAL PROCESS<br>    Immunity and defense(2.16.00.00.00) > Natural killer cell mediated immunity(2.16.07.00.00)<br>    Immunity and defense(2.16.00.00.00) > B-cell- and antibody-mediated immunity(2.16.02.00.00)<br>    MOLECULAR FUNCTIONS<br>    Receptor(1.01.00.00.00) > Other receptor(1.0 1.99.00.00)<br>    Signaling molecule(1.02.00.00.00) > Membrane-bound signaling molecule(1.02.07.00.00)<br>    Defense/immunity protein(1.25.00.00.00) > Other defense and immunity protein(1.25.99.00.00)<br><br>HUMAN GENE ONTOLOGY<br>    BIOLOGICAL PROCESS<br>    signal transduction > cell surface receptor linked signal transduction<br>    humoral defense mechanism > antimicrobial response<br>    cell communication > signal transduction<br>    defence response > cellular defense response<br>    intracellular protein traffic > endocytosis |

(continued)

| Human Protein | SEQ ID NO: | FUNCTION |
|---|---|---|
| | | MOLECULAR FUNCTION<br>sugar binding > lectin<br>molecular_function unknown > lymphocyte antigen<br>nucleic acid binding > RNA binding<br>DNA binding > transcription factor<br>lectin > mannose binding lectin<br>mannose binding > mannose binding lectin<br>CELL COMPONENT<br>cell > membrane fraction<br>cell > nucleus<br>cell > plasma membrane<br>plasma membrane > integral plasma membrane protein<br>nucleus > nucleolus<br>HUMAN PROTEIN DOMAINS (INTERPRO SIGNATURES)<br>IPR001304 (CLECT)<br>IPR001304 (lectin c)<br>IPR001304 (C TYPE LECTIN 2) |
| hP 14-023.4 | SEQ ID NO: 118 | HUMAN PANTHER CLASSIFICATIONS<br>FAMILY (SUBFAMILY)<br>NUCLEOPLASMIN(NUCLEOPHOSMI N)<br>BIOLOGICAL PROCESS<br>Nucleoside, nucleotide and nucleic acid metabolism(2.04.00.00.00) ><br>rRNA metabolism(2.04.08.00.00)<br>MOLECULAR FUNCTIONS<br>Molecular function unclassified(1.97.00.00.00)<br><br>HUMAN GENE ONTOLOGY<br>BIOLOGICAL PROCESS<br>cell communication > signal transduction<br>transcription, DNA-dependent > transcription regulation<br>protein modification > protein dephosphorylation<br>DNA metabolism > DNA repair<br>humoral defense mechanism > antimicrobial response<br>MOLECULAR FUNCTION<br>nucleic acid binding > RNA binding<br>DNA binding > transcription factor<br>protein kinase > protein tyrosine kinase<br>nuclease > endonuclease<br>sugar binding > lectin<br>CELL COMPONENT<br>cell > membrane fraction<br>cell > nucleus<br>plasma membrane > integral plasma membrane protein<br>GO cellular component > extracellular<br>nucleus > nucleolus HUMAN PROTEIN DOMAINS (INTERPRO<br>SIGNATURES)<br>IPROO4301 (Nucleoplasmin)<br>NULL (ASP RICH) |
| hP14-023.5 | SEQ ID NO: 120 | HUMAN PANTHER CLASSIFICATIONS |

(continued)

| Human Protein | SEQ ID NO: | FUNCTION |
|---|---|---|
|  |  | FAMILY (SUBFAMILY)<br>NKG2 TYPE II INTEGRAL MEMBRANE PROTEIN(C-TYPE LECTIN-RELATED)<br>BIOLOGICAL PROCESS<br>Immunity and defense(2.16.00.00.00) > Natural killer cell mediated immunity(2.16.07.00.00)<br>Immunity and defense(2.16.00.00.00) > B-cell- and antibody-mediated immunity(2.16.02.00.00)<br>MOLECULAR FUNCTIONS<br>Receptor(1.01.00.00.00) > Other receptor(1.01.99.00.00)<br>Signaling molecule(1.02.00.00.00) > Membrane-bound signaling molecule(1.02.07.00.00)<br>Defense/immunity protein(1.25.00.00.00) > Other defense and immunity protein(1.25.99.00.00)<br><br>HUMAN GENE ONTOLOGY<br>BIOLOGICAL PROCESS<br>signal transduction > cell surface receptor linked signal transduction<br>humoral defense mechanism > antimicrobial response<br>defence response > immune response<br>defence response > cellular defense response<br>stress response > defence response<br>MOLECULAR FUNCTION<br>sugar binding > lectin<br>molecular_function unknovvn > lymphocyte antigen<br>protein binding > lipoprotein binding<br>CELL COMPONENT<br>cell > membrane fraction<br>cell > plasma membrane<br>plasma membrane > integral plasma membrane protein HUMAN PROTEIN DOMAINS (INTERPRO SIGNATURES)<br>No Domain Hit |
| hP14-023.6 | SEQ ID NO: 122 | HUMAN PANTHER CLASSIFICATIONS<br>FAMILY (SUBFAMILY)<br>NUCLEOPLASMIN(NUCLEOPHOSMI N)<br>BIOLOGICAL PROCESS<br>Nucleoside, nucleotide and nucleic acid metabolism(2.04.00.00.00) > rRNA metabolism(2.04.08.00.00)<br>MOLECULAR FUNCTIONS<br>Molecular function unclassified(1.97.00.00.00)<br><br>HUMAN GENE ONTOLOGY<br>BIOLOGICAL PROCESS<br>cell communication > signal transduction<br>humoral defense mechanism > antimicrobial response<br>signal transduction > cell surface receptor linked signal transduction<br>defence response > cellular defense response<br>intracellular protein traffic > endocytosis<br>MOLECULAR FUNCTION<br>sugar binding > lectin |

(continued)

| Human Protein | SEQ ID NO: | FUNCTION |
|---|---|---|
| | | nucleic acid binding > RNA binding |
| | | molecular_function unknown > lymphocyte antigen |
| | | DNA binding > transcription factor |
| | | lectin > mannose binding lectin |
| | | mannose binding > mannose binding lectin |
| | | CELL COMPONENT |
| | | cell > membrane fraction |
| | | cell > nucleus |
| | | cell > plasma membrane |
| | | plasma membrane > integral plasma membrane protein |
| | | nucleus > nucleolus |
| | | HUMAN PROTEIN DOMAINS (INTERPRO SIGNATURES) |
| | | IPR004301 (Nucleoplasmin) |
| | | NULL (ASP RICH) |
| hP14-023.7 | SEQ ID NO: 124 | HUMAN PANTHER CLASSIFICATIONS |
| | | FAMILY (SUBFAMILY) |
| | | NKG2 TYPE II INTEGRAL |
| | | MEMBRANE PROTEINS-TYPE LECTIN-RELATED) |
| | | BIOLOGICAL PROCESS |
| | | Immunity and defense(2.16.00.00.00) > Natural killer cell mediated immunity(2.16.07.00.00) |
| | | Immunity and defense(2.16.00.00.00) > B-cell- and antibody-mediated immunity(2.16.02.00.00) |
| | | MOLECULAR FUNCTIONS |
| | | Receptor(1.01.00.00.00) > Other receptor(1.01.99.00.00) |
| | | Signaling molecule(1.02.00.00.00) > Membrane-bound signaling molecule(1.02.07.00.00) |
| | | Defense/immunity protein(1.25.00.00.00) > Other defense and immunity |
| | | protein(1.25.99.00.00) |
| | | |
| | | HUMAN GENE ONTOLOGY |
| | | BIOLOGICAL PROCESS |
| | | signal transduction > cell surface receptor linked signal transduction |
| | | humoral defense mechanism > antimicrobial response |
| | | cell communication > signal transduction |
| | | defence response > cellular defense response |
| | | intracellular protein traffic > endocytosis |
| | | MOLECULAR FUNCTION |
| | | sugar binding > lectin |
| | | nucleic acid binding > RNA binding |
| | | molecular_function unknown > lymphocyte antigen |
| | | DNA binding > transcription factor |
| | | lectin > mannose binding lectin |
| | | mannose binding > mannose binding lectin |
| | | CELL COMPONENT |
| | | cell > membrane fraction |
| | | cell > nucleus |
| | | cell > plasma membrane |
| | | plasma membrane > integral plasma membrane protein |

(continued)

| Human Protein | SEQ ID NO: | FUNCTION |
|---|---|---|
| | | nucleus > nucleolus<br>HUMAN PROTEIN DOMAINS (INTERPRO SIGNATURES)<br>    IPR001304 (CLECT)<br>    IPR001304 (lectin c)<br>    IPR001304 (C TYPE LECTIN 2) |
| hP14-023.8 | SEQ ID NO: 126 | HUMAN PANTHER CLASSIFICATIONS<br>    FAMILY (SUBFAMILY)<br>    NUCLEOPLASMIN(NUCLEOPHOSMI N)<br>    BIOLOGICAL PROCESS<br>    Nucleoside, nucleotide and nucleic acid metabolism(2.04.00.00.00) ><br>rRNA metabolism(2.04.08.00.00)<br>    MOLECULAR FUNCTIONS<br>    Molecular function unclassified(1.97.00.00.00)<br><br>HUMAN GENE ONTOLOGY<br>    BIOLOGICAL PROCESS<br>    humoral defense mechanism > antimicrobial response<br>    defence response > cellular defense response<br>    intracellular protein traffic > endocytosis<br>    cell communication > signal transduction<br>    signal transduction > cell surface receptor linked signal transduction<br>    MOLECULAR FUNCTION<br>    sugar binding > lectin<br>    nucleic acid binding > RNA binding<br>    molecular_function unknown > lymphocyte antigen<br>    DNA binding > transcription factor<br>    lectin > mannose binding lectin<br>    mannose binding > mannose binding lectin<br>    CELL COMPONENT<br>    cell > membrane fraction<br>    cell > nucleus<br>    cell > plasma membrane<br>    plasma membrane > integral plasma membrane protein<br>    nucleus > nucleolus<br>HUMAN PROTEIN DOMAINS (INTERPRO SIGNATURES)<br>    IPR004301 (Nucleoplasmin)<br>    NULL (ASP RICH) |
| hP14-023.9 | SEQ ID NO: 128 | HUMAN PANTHER CLASSIFICATIONS<br>    FAMILY (SUBFAMILY)<br>    NUCLEOPLASMIN(NUCLEOPHOSMI N)<br>    BIOLOGICAL PROCESS<br>    Nucleoside, nucleotide and nucleic acid metabolism(2.04.00.00.00) ><br>rRNA metabolism(2.04.08.00.00)<br>    MOLECULAR FUNCTIONS<br>    Molecular function unclassified(1.97.00.00.00)<br><br>HUMAN GENE ONTOLOGY<br>    BIOLOGICAL PROCESS<br>    cell communication > signal transduction |

(continued)

| Human Protein | SEQ ID NO: | FUNCTION |
|---|---|---|
| | | signal transduction > cell surface receptor linked signal transduction |
| | | humoral defense mechanism > antimicrobial response |
| | | defence response > cellular defense response |
| | | intracellular protein traffic > endocytosis |
| | | MOLECULAR FUNCTION |
| | | sugar binding > lectin |
| | | nucleic acid binding > RNA binding |
| | | molecular_function unknown > lymphocyte antigen |
| | | DNA binding > transcription factor |
| | | lectin > mannose binding lectin |
| | | mannose binding > mannose binding lectin |
| | | CELL COMPONENT |
| | | cell > membrane fraction |
| | | cell > nucleus |
| | | cell > plasma membrane |
| | | plasma membrane > integral plasma membrane protein |
| | | nucleus > nucleolus |
| | | HUMAN PROTEIN DOMAINS (INTERPRO SIGNATURES) |
| | | IPR004301 (Nucleoplasmin) |
| | | NULL (ASP RICH) |
| hP14-032.1 | SEQ ID NO: 134 | HUMAN PANTHER CLASSIFICATIONS |
| | | FAMILY (SUBFAMILY) |
| | | CARCINOEMBRYONIC |
| | | ANTIGEN(Unassigned) |
| | | BIOLOGICAL PROCESS |
| | | Biological process unclassified(2.99.00.00.00) |
| | | MOLECULAR FUNCTIONS |
| | | Cell adhesion molecule(1.05.00.00.00) > CAM family adhesion molecule(1.05.01.00.00) |
| | | |
| | | HUMAN GENE ONTOLOGY |
| | | BIOLOGICAL PROCESS |
| | | defence response > immune response |
| | | cell communication > cell-cell signaling |
| | | protein-membrane targeting > post-translational membrane targeting |
| | | cell communication > cell adhesion |
| | | cell communication > signal transduction |
| | | MOLECULAR FUNCTION |
| | | molecular_function unknown > tumor antigen |
| | | defense/immunity protein > immunoglobulin |
| | | B cell receptor > immunoglobulin |
| | | GO molecular function > cell adhesion |
| | | protein kinase > protein tyrosine kinase |
| | | calmodulin regulated protein kinase > myosin light chain kinase |
| | | CELL COMPONENT |
| | | cell > membrane fraction |
| | | cell > plasma membrane |
| | | plasma membrane > integral plasma membrane protein |
| | | plasma membrane > peripheral plasma membrane protein |

(continued)

| Human Protein | SEQ ID NO: | FUNCTION |
|---|---|---|
| | | extracellular > extracellular space<br>HUMAN PROTEIN DOMAINS (INTERPRO SIGNATURES)<br>    IPR003598 (IGc2)<br>    IPR003599 (IG)<br>    IPR003006 (ig) |
| hP22-005.1 | SEQ ID NO: 140 | HUMAN PANTHER CLASSIFICATIONS<br>    FAMILY (SUBFAMILY)<br>    SEMAPHORIN(SEMAPHORIN 4C)<br>    BIOLOGICAL PROCESS<br>    Signal transduction(2.11.00.00.00) > Cell communication (2.11.03.00.00)<br>    Developmental processes(2.23.00.00.00) > Ectoderm development (2.23.08.00.00) > Neurogenesis(2.23.08.01.00)<br>    MOLECULAR FUNCTIONS<br>    Signaling molecule(1.02.00.00.00) > Membrane-bound signaling molecule(1.02.07.00.00)<br><br>HUMAN GENE ONTOLOGY<br>    BIOLOGICAL PROCESS<br>    ectoderm development > neurogenesis<br>    apoptosis > anti-apoptosis<br>    defence response > immune response<br>    cell communication > cell adhesion<br>    peptidoglycan catabolism > axon guidance<br>    axonogenesis > axon guidance<br>    MOLECULAR FUNCTION<br>    defense/immunity protein > immunoglobulin<br>    B cell receptor > immunoglobulin<br>    GO molecular function > cell adhesion<br>    CELL COMPONENT<br>    cell > membrane fraction<br>    GO cellular component > extracellular<br>    extracellular > extracellular space<br>HUMAN PROTEIN DOMAINS (INTERPRO SIGNATURES)<br>    IPR003659 (PSI)<br>    IPR003599 (IG)<br>    IPR001627 (Sema)<br>    IPR002165 (PSI)<br>    IPR000694 (PRO RICH) |
| hP22-005.2 | SEQ ID NO: 142 | HUMAN PANTHER CLASSIFICATIONS<br>    FAMILY (SUBFAMILY)<br>    SEMAPHORIN(SEMAPHORIN 4C)<br>    BIOLOGICAL PROCESS<br>    Signal transduction(2.11.00.00.00) > Cell communication (2.11.03.00.00)<br>    Developmental processes(2.23.00.00.00) > Ectoderm development (2.23.08.00.00) > Neurogenesis(2.23.08.01.00)<br>    MOLECULAR FUNCTIONS<br>    Signaling molecule(1.02.00.00.00) > Membrane-bound signaling moleeule(1.02.07.00.00) |

(continued)

| Human Protein | SEQ ID NO: | FUNCTION |
|---|---|---|
| | | HUMAN GENE ONTOLOGY<br>    BIOLOGICAL PROCESS<br>    ectoderm development > neurogenesis<br>    apoptosis > anti-apoptosis<br>    defence response > immune response<br>    cell communication > cell adhesion<br>    MOLECULAR FUNCTION<br>    defense/immunity protein > immunoglobulin<br>    B cell receptor > immunoglobulin<br>    GO molecular function > cell adhesion<br>    CELL COMPONENT<br>    cell > membrane fraction<br>    extracellular > extracellular space<br>HUMAN PROTEIN DOMAINS (INTERPRO SIGNATURES)<br>    IPR003659 (PSI)<br>    IPR003599 (IG)<br>    IPR001627 (Sema)<br>    IPR002165 (PSI)<br>    IPR000694 (PRO RICH) |
| hP22-005.3 | SEQ ID NO: 144 | HUMAN PANTHER CLASSIFICATIONS<br>    FAMILY (SUBFAMILY)<br>    SEMAPHORIN(SEMAPHORIN 4C)<br>    BIOLOGICAL PROCESS<br>    Signal transduction(2.11.00.00.00) > Cell communication (2.11.03.00.00)<br>    Developmental processes(2.23.00.00.00) > Ectoderm development (2.23.08.00.00) > Neurogenesis(2.23.08.01.00)<br>    MOLECULAR FUNCTIONS<br>    Signaling molecule(1.02.00.00.00) > Membrane-bound signaling molecule(1.02.07.00.00)<br><br>HUMAN GENE ONTOLOGY<br>    BIOLOGICAL PROCESS<br>    ectoderm development > neurogenesis<br>    apoptosis > anti-apoptosis<br>    defence response > immune response<br>    cell communication > cell adhesion<br>    MOLECULAR FUNCTION<br>    defense/immunity protein > immunoglobulin<br>    B cell receptor > immunoglobulin<br>    GO molecular function > cell adhesion<br>    CELL COMPONENT<br>    cell > membrane fraction<br>    extracellular > extracellular space<br>HUMAN PROTEIN DOMAINS (INTERPRO SIGNATURES)<br>    IPR003659 (PSI)<br>    IPR003599 (IG)<br>    IPR001627 (Sema)<br>    IPR002165 (PSI) |

(continued)

| Human Protein | SEQ ID NO: | FUNCTION |
|---|---|---|
| | | IPR000694 (PRO RICH) |
| hP22-005.4 | SEQ ID NO: 146 | HUMAN PANTHER CLASSIFICATIONS<br><br>    FAMILY (SUBFAMILY)<br>    SEMAPHORIN(SEMAPHORIN 4C)<br>    BIOLOGICAL PROCESS<br>    Signal transduction(2.11.00.00.00) > Cell communication (2.11.03.00.00)<br>    Developmental processes(2.23.00.00.00) > Ectoderm development (2.23.08.00.00) > Neurogenesis(2.23.08.01.00)<br>    MOLECULAR FUNCTIONS<br>    Signaling molecule(1.02.00.00.00) > Membrane-bound signaling molecule(1.02.07.00.00)<br><br>HUMAN GENE ONTOLOGY<br>    BIOLOGICAL PROCESS<br>    ectoderm development > neurogenesis<br>    apoptosis > anti-apoptosis<br>    defence response > immune response<br>    cell communication > cell adhesion<br>    MOLECULAR FUNCTION<br>    defense/immunity protein > immunoglobulin<br>    B cell receptor > immunoglobulin<br>    GO molecular function > cell adhesion<br>    CELL COMPONENT<br>    cell > membrane fraction<br>    extracellular > extracellular space<br>    HUMAN PROTEIN DOMAINS (INTERPRO SIGNATURES)<br>    IPR003659 (PSI)<br>    IPR003599 (IG)<br>    IPR001627 (Sema)<br>    IPR002165 (PSI)<br>    IPR000694 (PRO RICH) |

A CA protein (CAP) is a G-protein coupled receptor wherein the CAP sequence is selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19.

A CA protein (CAP) is expressed on a cell surface, wherein the CA protein is selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146.

Certain aspects of the present invention are described in greater detail in the non-limiting examples that follow.

EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all and only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

**Example 1: Insertion site analysis following tumor induction in mice**

Tumors are induced in mice using either mouse mammary tumor virus (MMTV) or murine leukemia virus (MLV). MMTV causes mammary adenocarcinomas and MLV causes a variety of different hematopoetic malignancies (primarily T- or B-cell lymphomas). Three routes of infection are used: (1) injection of neonates with purified virus preparations, (2) infection by milk-borne virus during nursing, and (3) genetic transmission of pathogenic proviruses via the germ-line (*Akvr1* and/or *Mtv2*). The type of malignancy present in each affected mouse is determined by histological analysis of H&E-stained thin sections of formalin-fixed, paraffin-embedded biopsy samples. Host DNA sequences flanking all clonally-integrated proviruses in each tumor are recovered by nested anchored-PCR using two virus-specific primers and two primers specific for a 40 bp double stranded DNA anchor ligated to restriction enzyme digested tumor DNA. Amplified bands representing host/virus junction fragments are cloned and sequenced. Then the host sequences (called "tags") are used to BLAST analyze the Celera mouse genomic sequence. For each individual tag, three parameters are recorded: (1) the mouse chromosome assignment, (2) base pair coordinates at which the integration occurred, and (3) provirus orientation. Using this information, all available tags from all analyzed tumors are mapped to the mouse genome. To identify the protooncogene targets of provirus insertion mutation, the provirus integration pattern at each cluster of integrants is analyzed relative to the locations of all known genes in the transcriptome. The presence of provirus at the same locus in two or more independent tumors is *prima facie* evidence that a protooncogene is present at or very near the proviral integration sites. This is because the genome is too large for random integrations to result in observable clustering. Any clustering that is detected is unequivocal evidence for biological selection during tumorigenesis. In order to identify the human orthologs of the protooncogene targets of provirus insertion mutation, a comparative analysis of syntenic regions of the mouse and human genomes is performed.

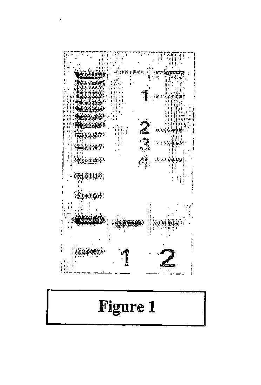

An example of PCR amplification of host/virus junction fragments is presented in Fig 1. Lane 1 contains the amplification products from normal control DNA and lane 2 contains the amplification products from tumor DNA. The bands result from 5' host/virus junction fragments present in the DNA samples. Lane 1 has bands from the *env*/3' LTR junctions from all proviruses (upper) and the host / 5'LTR from the pathogenic endogenous *Mtv2* provirus present in this particular mouse strain. This endogenous provirus is detected because its sequence is identical to the new clonally integrated proviruses in the tumor. All four new clonally integrated proviruses known to be in this tumor are readily detected.

**Example 2: Analysis of Quantitative RT-PCR: Comparative $C_T$ Method.**

The expression level of target genes is quantified using the ABI PRISM 7900HT Sequence Detection System (Applied Biosystems, California). The method is based on the quantitation of the initial copy number of target template in comparison to that of a reference (normalizer) housekeeper gene (Pre-Developed TaqMan® Assay Reagents Gene Expression Quantification Protocol, Applied Biosystems, 2001). Accumulation of DNA product with each PCR cycle is related to amplicon efficiency and the initial template concentration. Therefore the amplification efficiency of both the target and the normalizer must be approximately equal. The threshold cycle ($C_T$), which is dependent on the starting template copy number and the DNA amplification efficiency, is a PCR cycle during which PCR product growth is exponential. With a similar dynamic range for the target and normalizer, the comparative $C_T$ method is applicable.

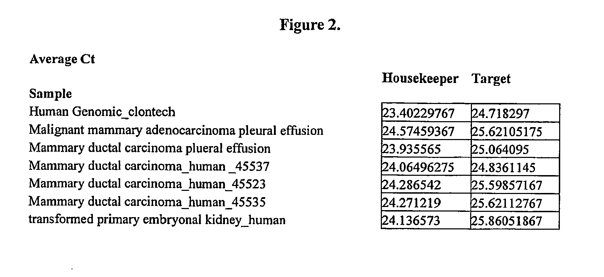

An example of the comparative $C_T$ method of gene expression for quantitative RT-PCR is shown in Figure 2. In the first step, assays are performed in quadruplicate on a normal tissue and several sample tissues. In these tissues, the means and standard deviations of $C_T$ values are determined for housekeeper genes (chosen as controls if shown to be biologically stable among various samples, irrespective of disease state) and for the target gene. Figure 2 shows an example of average $C_T$ values for a housekeeper gene and target gene. These values can fall within a range from upper teens to 40 depending on the intrinsic expression level of the gene in the particular tissue. The coefficient of variance of all replicate sets cannot exceed 1.5%.

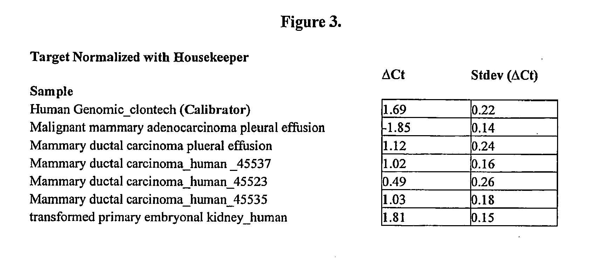

An assessment of how the $\Delta C_T$ changes with template dilution verifies that the efficiencies of the target and housekeeper amplicons are approximately equal if the log input amount of template RNA versus $\Delta C_T$ plot has a slope < 0.10. With the relative efficiencies verified for target and housekeeper, the $\Delta\Delta C_T$ comparative calculation becomes valid, as mentioned above. An example of the calculated difference between the $C_T$ values of target and housekeeper genes ($\Delta C_T$) for various samples is shown in Figure 3. The $\Delta\Delta C_T$ is calculated for each sample by subtracting its $\Delta C_T$ value from the $\Delta C_T$ value of the baseline (calibrator) sample. If the expression is increased in some samples and decreased in others, $\Delta\Delta C_T$ will be a mixture of negative and positive values. The final step in the calculation is to transform these values to absolute values. The formula for this is:

$$\text{Comparative expression level} = 2^{-\Delta\Delta CT}$$

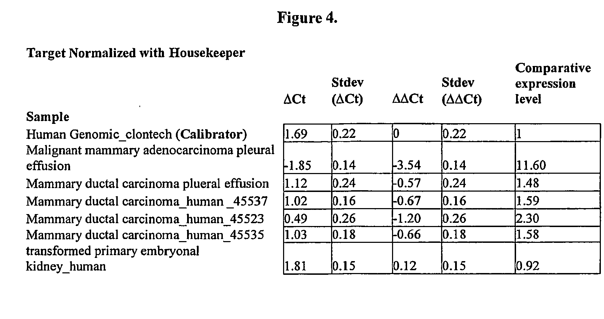

The final value for the calibrator should always be one. Figure 4 shows the $\Delta\Delta C_T$ and comparative expression level for each sample from Figure 3.

**Example 3: Detection of elevated levels of cDNA associated with cancer using arrays.**

cDNA sequences representing a variety of candidate CA genes to be screened for differential expression in cancer are assayed by hybridization on polynucleotide arrays. The cDNA sequences include cDNA clones isolated from cell lines or tissues of interest. The cDNA sequences analyzed also include polynucleotides comprising sequence overlap with sequences in the Unigene database, and which encode a variety of gene products of various origins, functionality, and levels of characterization. cDNAs are spotted onto reflective slides (Amersham) according to methods well known in the art at a density of 9,216 spots per slide representing 4,068 sequences (including controls) spotted in duplicate, with approximately 0.8 μl of an approximately 200ng/μl solution of cDNA.

PCR products of selected cDNA clones corresponding to the gene products of interest are prepared in a 50% DMSO solution. These PCR products are spotted onto Amersham aluminum microarray slides at a density of 9216 clones per array using a Molecular Dynamics Generation III spotting robot. Clones are spotted in duplicate, for a total of 4608 different sequences per chip.

cDNA probes are prepared from total RNA obtained by laser capture microdissection (LCM, Arcturus Enginering Inc., Mountain View, CA) of tumor tissue samples and normal tissue samples isolated from patients.

Total RNA is first reverse transcribed into cDNA using a primer containing a T7 RNA polymerase promoter, followed by second strand DNA synthesis. cDNA is then transcribed *in vitro* to produce antisense RNA using the T7 promoter-mediated expression (see, *e.g.,* Luo et al. (1999) Nature Med 5:117-122), and the antisense RNA is then converted into cDNA. The second set of cDNAs are again transcribed *in vitro,* using the T7 promoter, to provide antisense RNA. This antisense RNA is then fluorescently labeled, or the RNA is again converted into cDNA, allowing for a third round of T7-mediated amplification to produce more antisense RNA. Thus the procedure provides for two or three rounds of *in vitro* transcription to produce the final RNA used for fluorescent labeling. Probes are labeled by making fluorescently labeled cDNA from the RNA starting material. Fluorescently labeled cDNAs prepared from the tumor RNA sample are compared to fluorescently labeled cDNAs prepared from normal cell RNA sample. For example, the cDNA probes from the normal cells are labeled with Cy3 fluorescent dye (green) and the cDNA probes prepared from suspected cancer cells are labeled with Cy5 fluorescent dye (red).

The differential expression assay is performed by mixing equal amounts of probes from tumor cells and normal cells of the same patient. The arrays are prehybridized by incubation for about 2 hrs at 60°C in 5x SSC, 0.2% SDS, 1 mM EDTA, and then washing three times in water and twice in isopropanol. Following prehybridization of the array, the probe mixture is then hybridized to the array under conditions of high stringency (overnight at 42°C in 50% formamide, 5X SSC, and 0.2% SDS. After hybridization, the array is washed at 55°C three times as follows: 1) first wash in 1X SSC/ 0.2% SDS; 2) second wash in 0.1X SSC/0.2% SDS; and 3) third wash in 0.1XSSC.

The arrays are then scanned for green and red fluorescence using a Molecular Dynamics Generation III dual color laser-scanner/detector. The images are processed using BioDiscovery Autogene software, and the data from each scan set normalized. The experiment is repeated, this time labeling the two probes with the opposite color in order to perform the assay in both "color directions." Each experiment is sometimes repeated with two more slides (one in each color direction). The data from each scan is normalized, and the level of fluorescence for each sequence on the array expressed as a ratio of the geometric mean of 8 replicate spots/genes from the four arrays or 4 replicate spots/gene from 2 arrays or some other permutation.

Normalization: The objective of normalization is to generate a cDNA library in which all transcripts expressed in a particular cell type or tissue are equally represented (S.M. Weissman, Mol Biol. Med. 4(3):133-143 (1987); Patanjali, et al., Proc. Natl. Acad. Sci. USA 88(5):1943-1947 (1991)), and therefore isolation of as few as 30,000 recombinant clones in an optimally normalized library may represent the entire gene expression repertoire of a cell, estimated to number 10,000 per cell.

Total RNA is extracted from harvested cells using RNeasy™ Protect Kit (Qiagen, Valencia, CA), following manufacturer's recommended procedures. RNA is quantified using RiboGreen™ RNA quantification kit (Molecular Probes, Inc. Eugene, OR). One μg of total RNA is reverse transcribed and PCR amplified using SMART™ PCR cDNA synthesis kit (ClonTech, Palo Alto, CA). The cDNA products are size-selected by agarose gel electrophoresis using standard procedures (Sambrook, J.T., et al. Molecular Cloning: A Laboratory Manual, 2d ed., Cold Spring Harbor Laboratory Press, NY). The cDNA is extracted using Bio 101 Geneclean® II kit (Qbiogene, Carlsbad, CA). Normalization of the cDNA is carried out using kinetics of hybridization principles: 1.0 μg of cDNA is denatured by heat at 100° C for 10 minutes, then incubated at 42° C for 42 hours in the presence of 120 mM NaCl, 10 mM Tris.HCl (pH=8.0), 5 mM EDTA.Na+ and 50% formamide. Single-stranded cDNA ("normalized") is purified by hydroxyapatite chromatography (#130-0520, BioRad, Hercules, CA) following the manufacturer's recommended procedures, amplified and converted to double-stranded cDNA by three cycles of PCR amplification, and cloned into plasmid vectors using standard procedures (Sambrook, J.T., et al. Molecular Cloning: A Laboratory Manual, 2d ed., Cold Spring Harbor Laboratory Press, NY). All primers/adaptors used in the normalization and cloning process are provided by the manufacturer in the SMART™ PCR cDNA synthesis kit.(ClonTech, Palo Alto, CA). Supercompetent cells (XL-2 Blue Ultracompetent Cells, Stratagene, California) are transfected with the normalized cDNA libraries, plated on solid media and grown overnight at 36° C.

The sequences of 10,000 recombinants per normalized library are analyzed by capillary sequencing using the ABI PRISM 3700 DNA Analyzer (Applied Biosystems, California). To determine the representation of transcripts in a library, BLAST analysis is performed on the clone sequences to assign transcript identity to each isolated clone, i.e., the sequences of the isolated polynucleotides are first masked to eliminate low complexity sequences using the XBLAST masking program (Claverie "Effective Large-Scale Sequence Similarity Searches," Computer Methods for Macromolecular Sequence Analysis, Doolittle, ed., Meth. Enzymol. 266:212-227 Academic Press, NY, NY (1996); see particularly Claverie, in "Automated DNA Sequencing and Analysis Techniques" Adams et al., eds., Chap. 36, p. 267 Academic Press, San Diego, 1994 and Claverie et al. Comput. Chem. (1993) 17:191). Generally, masking does not influence the final search results, except to eliminate sequences of relative little interest due to their low complexity, and to eliminate multiple "hits" based on similarity to repetitive regions common to multiple sequences, e.g., Alu repeats. The remaining sequences are then used in a BLASTN vs. GenBank search. The sequences are also used as query sequence in a BLASTX vs. NRP (non-redundant proteins) database search.

Automated sequencing reactions are performed using a Perkin-Elmer PRISM Dye Terminator Cycle Sequencing Ready Reaction Kit containing AmpliTaq DNA Polymerase, FS, according to the manufacturer's directions. The reactions are cycled on a GeneAmp PCR System 9600 as per manufacturer's instructions, except that they are annealed at 20° C. or 30° C. for one minute. Sequencing reactions are ethanol precipitated, pellets are resuspended in 8 microliters of loading buffer, 1.5 microliters is loaded on a sequencing gel, and the data is collected by an ABI PRISM 3700 DNA Sequencer. (Applied Biosystems, Foster City, CA).

The number of times a sequence is represented in a library is determined by performing sequence identity analysis on the cloned cDNA sequences and assigning transcript identity to each isolated clone. First, each sequence is checked to determine if it is a bacterial, ribosomal, or mitochondrial contaminant. Such sequences are excluded from the subsequent analysis. Second, sequence artifacts, such as vector and repetitive elements, are masked and/or removed from each sequence.

The remaining sequences are compared via BLAST (Altschul et. al, J. Mol. Biol., 215:40, 1990) to GenBank and EST databases for gene identification and are compared with each other via FastA (Pearson & Lipman, PNAS, 85: 2444, 1988) to calculate the frequency of cDNA appearance in the normalized cDNA library. The sequences are also searched against the GenBank and GeneSeq nucleotide databases using the BLASTN program (BLASTN 1.3MP: Altschul et al., J. Mol. Bio. 215:403, 1990). Fourth, the sequences are analyzed against a non-redundant protein (NRP) database with the BLASTX program (BLASTX 1.3MP: Altschul et al., supra). This protein database is a combination of the Swiss-Prot, PIR, and NCBI GenPept protein databases. The BLASTX program is run using the default BLOSUM-62 substitution matrix with the filter parameter: "xnu+seg". The score cutoff utilized is 75. Assembly of overlapping clones into contigs is done using the program Sequencher (Gene Codes Corp.; Ann Arbor, Mich.). The assembled contigs are analyzed using the programs in the GCG package (Genetic Computer Group, University Research Park, 575 Science Drive, Madison, Wis. 53711) Suite Version 10.1.

**Example 4: Detection of CA -Sequences in Human Cancer Cells and Tissues.**

DNA from prostate and breast cancer tissues and other human cancer tissues, human colon, normal human tissues including non-cancerous prostate, and from other human cell lines are extracted following the procedure of Delli Bovi et al. (1986, Cancer Res. 46:6333-6338). The DNA is resuspended in a solution containing 0.05 M Tris HCl buffer, pH 7.8, and 0.1 mM EDTA, and the amount of DNA recovered is determined by microfluorometry using Hoechst 33258 dye. Cesarone, C. et al., Anal Biochem 100:188-197 (1979).

Polymerase chain reaction (PCR) is performed using Taq polymerase following the conditions recommended by the manufacturer (Perkin Elmer Cetus) with regard to buffer, $Mg^{2+}$, and nucleotide concentrations. Thermocycling is performed in a DNA cycler by denaturation at 94° C. for 3 min. followed by either 35 or 50 cycles of 94° C. for 1.5 min., 50° C. for 2 min. and 72° C. for 3 min. The ability of the PCR to amplify the selected regions of the CA gene is tested by using a cloned CA polynucleotide(s) as a positive template(s). Optimal $Mg^{2+}$, primer concentrations and requirements for the different cycling temperatures are determined with these templates. The master mix recommended by the manufacturer is used. To detect possible contamination of the master mix components, reactions without template are routinely tested.

Southern blotting and hybridization are performed as described by Southern, E. M., (J. Mol. Biol. 98:503-517, 1975), using the cloned sequences labeled by the random primer procedure (Feinberg, A. P., et al., 1983, Anal. Biochem. 132:6-13). Prehybridization and hybridization are performed in a solution containing 6xSSPE, 5% Denhardt's, 0.5% SDS, 50% formamide, 100 μg/ml denatured salmon testis DNA, incubated for 18 hrs at 42° C., followed by washings with 2xSSC and 0.5% SDS at room temperature and at 37° C. and finally in 0.1xSSC with 0.5% SDS at 68° C. for 30 min (Sambrook et al., 1989, in "Molecular Cloning: A Laboratory Manual", Cold Spring Harbor Lab. Press). For paraffin-embedded tissue sections the conditions described by Wright and Manos (1990, in "PCR Protocols", Innis et al., eds., Academic Press, pp. 153-158) are followed using primers designed to detect a 250 bp sequence.

**Example 5: Expression of cloned polynucleotides in host cells.**

To study the protein products of CA genes, restriction fragments from CA DNA are cloned into the expression vector pMT2 (Sambrook, et al., Molecular Cloning: A Laboratory Manual, Cold Spring Harbor Laboratory Press pp 16.17-16.22 (1989)) and transfected into COS cells grown in DMEM supplemented with 10% FCS. Transfections are performed employing calcium phosphate techniques (Sambrook, et al (1989) pp. 16.32-16.40, supra) and cell lysates are prepared forty-eight hours after transfection from both transfected and untransfected COS cells. Lysates are subjected to analysis by immunoblotting using anti-peptide antibody.

In immunoblotting experiments, preparation of cell lysates and electrophoresis are performed according to standard procedures. Protein concentration is determined using BioRad protein assay solutions. After semi-dry electrophoretic transfer to nitrocellulose, the membranes are blocked in 500 mM NaCl, 20 mM Tris, pH 7.5, 0.05% Tween-20 (TTBS) with 5% dry milk. After washing in TTBS and incubation with secondary antibodies (Amersham), enhanced chemiluminescence (ECL) protocols (Amersham) are performed as described by the manufacturer to facilitate detection.

**Example 6: Generation of antibodies against polypeptides.**

Polypeptides, unique to CA genes are synthesized or isolated from bacterial or other (e.g., yeast, baculovirus) expression systems and conjugated to rabbit serum albumin (RSA) with m-maleimido benzoic acid N-hydroxysuccinimide ester (MBS) (Pierce, Rockford, Ill.). Immunization protocols with these peptides are performed according to standard methods. Initially, a pre-bleed of the rabbits is performed prior to immunization. The first immunization includes Freund's complete adjuvant and 500 $\mu$g conjugated peptide or 100 $\mu$g purified peptide. All subsequent immunizations, performed four weeks after the previous injection, include Freund's incomplete adjuvant with the same amount of protein. Bleeds are conducted seven to ten days after the immunization.

For affinity purification of the antibodies, the corresponding CA polypeptide is conjugated to RSA with MBS, and coupled to CNBr-activated Sepharose (Pharmacia, Uppsala, Sweden). Antiserum is diluted 10-fold in 10 mM Tris-HCl, pH 7.5, and incubated overnight with the affinity matrix. After washing, bound antibodies are eluted from the resin with 100 mM glycine, pH 2.5.

**Example 7: Generation of monoclonal antibodies against a CA polypeptide**

A non-denaturing adjuvant (Ribi, R730, Corixa, Hamilton MT) is rehydrated to 4ml in phosphate buffered saline. 100$\mu$l of this rehydrated adjuvant is then diluted with 400$\mu$l of Hank's Balanced Salt Solution and this is then gently mixed with the cell pellet used for immunization. Approximately 500 $\mu$g conjugated peptide or 100 $\mu$g purified peptide and Freund's complete are injected into Balb/c mice via foot-pad, once a week. After 6 weeks of weekly injection, a drop of blood is drawn from the tail of each immunized animal to test the titer of antibodies against CA polypeptides using FACS analysis. When the titer reaches at least 1:2000, the mice are sacrificed in a $CO_2$ chamber followed by cervical dislocation. Lymph nodes are harvested for hybridoma preparation. Lymphocytes from mice with the highest titer are fused with the mouse myeloma line X63-Ag8.653 using 35% polyethylene glycol 4000. On day 10 following the fusion, the hybridoma supernatants are screened for the presence of CAP-specific monoclonal antibodies by fluorescence activated cell sorting (FACS). Conditioned medium from each hybridoma is incubated for 30 minutes with a combined aliquot of PC3, Colo-205, LnCap, or Panc-1 cells. After incubation, the cell samples are washed, resuspended in 0.1 ml diluent and incubated with 1 $\mu$g/ml of FITC conjugated F(ab')2 fragment of goat antimouse IgG for 30 min at 4°C. The cells are washed, resuspended in 0.5 ml FACS diluent and analyzed using a FACScan cell analyzer (Becton Dickinson; San Jose, CA). Hybridoma clones are selected for further expansion, cloning, and characterization based on their binding to the surface of one or more of cell lines which express the CA polypeptide as assessed by FACS. A hybridoma making a monoclonal antibody designated mAbCA which binds an antigen designated Ag-CA.x and an epitope on that antigen designated Ag-CA.x.1 is selected.

**Example 8: ELISA, assay for Detecting CA related antigens.**

To test blood samples for antibodies that bind specifically to recombinantly produced CA antigens, the following procedure is employed. After a recombinant CA related protein is purified, the recombinant protein is diluted in PBS to a concentration of 5 $\mu$g/ml (500 ng/100 $\mu$l). 100 microliters of the diluted antigen solution is added to each well of a 96-well Immulon 1 plate (Dynatech Laboratories, Chantilly, Va.), and the plate is then incubated for 1 hour at room temperature, or overnight at 4° C., and washed 3 times with 0.05% Tween 20 in PBS. Blocking to reduce nonspecific binding of antibodies is accomplished by adding to each well 200 $\mu$l of a 1% solution of bovine serum albumin in PBS/Tween 20 and incubation for 1 hour. After aspiration of the blocking solution, 100 $\mu$l of the primary antibody solution (anticoagulated whole blood, plasma, or serum), diluted in the range of 1/16 to 1/2048 in blocking solution, is added and incubated for 1 hour at room temperature or overnight at 4° C. The wells are then washed 3 times, and 100 $\mu$l of goat anti-human IgG antibody conjugated to horseradish peroxidase (Organon Teknika, Durham, N.C.), diluted 1/500 or 1/1000 in PBS/Tween 20, 100 $\mu$l of o-phenylenediamine dihydrochloride (OPD, Sigma) solution is added to each well and incubated for 5-15 minutes. The OPD solution is prepared by dissolving a 5 mg OPD tablet in 50 ml 1% methanol in $H_2O$ and adding 50 $\mu$l 30% $H_2O_2$ immediately before use. The reaction is stopped by adding 25 1 of 4M $H_2SO_4$. Absorbances are read at 490 nm in a microplate reader (Bio-Rad).

**Example 9: Identification and characterization of CA antigen on cancer cell surface**

A cell pellet of proximately 25 ul packed cell volume of a cancer cell preparation is lysed by first diluting the cells to 0.5 ml in water followed by freezing and thawing three times. The solution is centrifuged at 14,000 rpm. The resulting pellet, containing the cell membrane fragments, is resuspended in 50 $\mu$l of SDS sample buffer (Invitrogen, Carlsbad, CA). The sample is heated at 80°C for 5 minutes and then centrifuged for 2 minutes at 14,000 rpm to remove any insoluble materials.

The samples are analyzed by Western blot using a 4 to 20% polyacrylamide gradient gel in Tris-Glycine SDS (Invitrogen; Carlsbad CA) following the manufacturer's directions. Ten microliters of membrane sample are applied to one lane on the polyacrylamide gel. A separate 10 $\mu$L sample is reduced first by the addition of 2 $\mu$L of dithiothreitol (100 mM) with heating at 80°C for 2 minutes and then loaded into another lane. Pre-stained molecular weight markers SeeBlue Plus2 (Invitrogen; Carlsbad, CA) are used to assess molecular weight on the gel. The gel proteins are transferred to a nitrocellulose membrane using a transfer buffer of 14.4 g/l glycine, 3 g/l of Tris Base, 10% methanol, and 0.05% SDS. The membranes are blocked, probed with a CAP-specific monoclonal antibody (at a concentration of 0.5 ug/ml), and developed using the Invitrogen WesternBreeze Chromogenic Kit-AntiMouse according to the manufacturer's directions. In the reduced sample of the tumor cell membrane samples, a prominent band is observed migrating at a molecular weight within about 10% of the predicted molecular weight of the corresponding CA protein.

**Example 10: Preparation of vaccines.**

The present invention also relates to a method of stimulating an immune response against cells that express CA polypeptides in a patient using CA polypeptides of the invention that act as an antigen produced by or associated with a malignant cell. This aspect of the invention provides a method of stimulating an immune response in a human against cancer cells or cells that express CA polynucleotides and polypeptides. The method comprises the step of administering to a human an immunogenic amount of a polypeptide comprising: (a) the amino acid sequence of a huma CA protein or (b) a mutein or variant of a polypeptide comprising the amino acid sequence of a human endogenous retrovirus CA protein.

**Example 11: Generation of transgenic animals expressing polypeptides as a means for testing therapeutics.**

CA nucleic acids are used to generate genetically modified non-human animals, or site specific gene modifications thereof, in cell lines, for the study of function or regulation of prostate tumor-related genes, or to create animal models of diseases, including prostate cancer. The term "transgenic" is intended to encompass genetically modified animals having an exogenous CA gene(s) that is stably transmitted in the host cells where the gene(s) may be altered in sequence to produce a modified protein, or having an exogenous CA LTR promoter operably linked to a reporter gene. Transgenic animals may be made through a nucleic acid construct randomly integrated into the genome. Vectors for stable integration include plasmids, retroviruses and other animal viruses, YACs, and the like. Of interest are transgenic mammals, e.g. cows, pigs, goats, horses, etc., and particularly rodents, e.g. rats, mice, etc.

The modified cells or animals are useful in the study of CA gene function and regulation. For example, a series of small deletions and/or substitutions may be made in the CA genes to determine the role of different genes in tumorigenesis. Specific constructs of interest include, but are not limited to, antisense constructs to block CA gene expression, expression of dominant negative CA gene mutations, and overexpression of a CA gene. Expression of a CA gene or variants thereof in cells or tissues where it is not normally expressed or at abnormal times of development is provided. In addition, by providing expression of proteins derived from CA in cells in which it is otherwise not normally produced, changes in cellular behavior can be induced.

DNA constructs for random integration need not include regions of homology to mediate recombination. Conveniently, markers for positive and negative selection are included. For various techniques for transfecting mammalian cells, see Keown et al., Methods in Enzymology 185:527-537 (1990).

For embryonic stem (ES) cells, an ES cell line is employed, or embryonic cells are obtained freshly from a host, e.g. mouse, rat, guinea pig, etc. Such cells are grown on an appropriate fibroblast-feeder layer or grown in the presence of appropriate growth factors, such as leukemia inhibiting factor (LIF). When ES cells are transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting chimeric animals screened for cells bearing the construct. By providing for a different phenotype of the blastocyst and the ES cells, chimeric progeny can be readily detected.

The chimeric animals are screened for the presence of the modified gene and males and females having the modification are mated to produce homozygous progeny. If the gene alterations cause lethality at some point in development, tissues or organs are maintained as allogeneic or congenic grafts or transplants, or in in vitro culture. The transgenic animals may be any non-human mammal, such as laboratory animals, domestic animals, etc. The transgenic animals are used in functional studies, drug screening, etc., e.g. to determine the effect of a candidate drug on prostate cancer, to test potential therapeutics or treatment regimens, etc.

**Example 12: Diagnostic Imaging Using CA Specific Antibodies**

The present invention encompasses the use of antibodies to CA polypeptides to accurately stage cancer patients at initial presentation and for early detection of metastatic spread of cancer. Radioimmunoscintigraphy using monoclonal antibodies specific for CA polypeptides can provide an additional cancer-specific diagnostic test. The monoclonal antibodies of the instant invention are used for histopathological diagnosis of carcinomas.

Subcutaneous human xenografts of cancer cells in nude mice is used to test whether a technetium-99m ($^{99m}$Tc)-labeled monoclonal antibody of the invention can successfully image the xenografted cancer by external gamma scintography as described for seminoma cells by Marks, et al., Brit. J. Urol. 75:225 (1995). Each monoclonal antibody specific for a CA polypeptide is purified from ascitic fluid of BALB/c mice bearing hybridoma tumors by affinity chromatography on protein A-Sepharose. Purified antibodies, including control monoclonal antibodies such as an avidin-specific monoclonal antibody (Skea, et al., J. Immunol. 151:3557 (1993)) are labeled with $^{99m}$Tc following reduction, using the methods of Mather, et al., J. Nucl. Med. 31:692 (1990) and Zhang et al., Nucl. Med. Biol. 19:607 (1992). Nude mice bearing human cancer cells are injected intraperitoneally with 200-500 $\mu$Ci of $^{99m}$Tc-labeled antibody. Twenty-four hours after injection, images of the mice are obtained using a Siemens ZLC3700 gamma camera equipped with a 6 mm pinhole collimator set approximately 8 cm from the animal. To determine monoclonal antibody biodistribution following imaging, the normal organs and tumors are removed, weighed, and the radioactivity of the tissues and a sample of the injectate are measured. Additionally, CA-specific antibodies conjugated to antitumor compounds are used for cancer-specific chemotherapy.

**Example 13: Immunohistochemical methods**

Frozen tissue samples from cancer patients are embedded in an optimum cutting temperature (OCT) compound and quick-frozen in isopentane with dry ice. Cryosections are cut with a Leica 3050 CM mictrotome at thickness of 5 $\mu$m and thaw-mounted on vectabound-coated slides. The sections are fixed with ethanol at -20°C and allowed to air dry overnight at room temperature. The fixed sections are stored at -80°C until use. For immunohistochemistry, the tissue sections are retrieved and first incubated in blocking buffer (PBS, 5% normal goat serum, 0.1% Tween 20) for 30 minutes at room temperature, and then incubated with the CA protein-specific monoclonal antibody and control monoclonal antibodies diluted in blocking buffer (1 $\mu$g/ml) for 120 minutes. The sections are then washed three times with the blocking buffer. The bound monoclonal antibodies are detected with a goat anti-mouse IgG + IgM (H+L) F(ab')$_2$-peroxidase conjugates and the peroxidase substrate diaminobenzidine (1 mg/ml, Sigma Catalog No. D 5637) in 0.1 M sodium acetate buffer pH 5.05 and 0.003% hydrogen peroxide (Sigma cat. No. H1009). The stained slides are counter-stained with hematoxylin and examined under Nikon microscope.

Monoclonal antibody against a CA protein (antigen) is used to test reactivity with various cell lines from different types of tissues. Cells from different established cell lines are removed from the growth surface without using proteases, packed and embedded in OCT compound. The cells are frozen and sectioned, then stained using a standard IHC protocol. The CellArray™ technology is described in WO 01/43869. Normal tissue (human) obtained by surgical resection are frozen and mounted. Cryosections are cut with a Leica 3050 CM mictrotome at thickness of 5 $\mu$m and thaw-mounted

on vectabound-coated slides. The sections are fixed with ethanol at -20°C and allowed to air dry overnight at room temperature. PolyMICA™ Detection kit is used to determine binding of a CA-specific monoclonal antibody to normal tissue. Primary monoclonal antibody is used at a final concentration of 1 $\mu$g/ml.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

Table 1

MOUSE GENOMIC SEQUENCE : mD1-004 (Seq ID No: 1)

```
GAAATTGGGTTCCTTGTTTAATGATGGTGTCGGGGAAAGACGTGGATGGGGAGTTCCATTCTCAGCCCGCTGCTACTGCC
TGTGAGCCTCCTTTGGGAAAGTCCTTAATTTCTCTAGTTCTGTTATTTCCTCATCTCTAGAATGGGCATCACGACACTGA
TTTCACAGAGTTGTTCCGAGATGAAATGATGTGATGGAGTAAGTACTAGTTCCCAGCCTCTCAGATCCTGGAAATGGGTG
TCCTGCCTCTCCCTCTGGAAGCTCCCTGAAGGCCAGGCTTTGGGTCCTTCATCCAAGCTTGGATGAGTATTTTAAAAGAG
TCTCCGTGAGTTTTGCTCATTAGCCAAAACGGATGCTGGCCTTTGGAAGCGGGGCGGATGACTCTGCGTGGAAATATTCT
TCTCTGAGTTTCCCTCCTCTGCCTACTTCCTCGTGAGTTCTATGTGGCATCTATTTGAGGTCAAATCTATTGAAATATAC
TTCAGCTTAACTCTAGGTGCCTTTTAGTTGAGCACCAAGAATATACTAAGTGCTATGTAAGAAATAGATAAAGGACCCAG
TATGGAGGAGGAGAAATACCAAGCCAAGCCTAGTACTCAAACCGCAGACAGTGATAGGGACAGCCGGATCGCTTTCTGTA
GGCTTGAGGGTTTCTGAAATTCTTCAGGGAATTGGGAGCTCTTTGAGCTGGACTTTTTTAAGCGTATGAGTGTTTGGTCT
CTCTCTATGCATCTGTGTCATGAGCATACCTGGTACCTATGGAGAACAGGAGAGGGTATCAGACACCCTGGAACTACAGA
TAGTTGTCAGTTGCCATGTGGGTGCTGGGAAACCCACCCTAGGTCCTCTGCAGGAACAGCAAGTGCTGAAGGGACTACTG
AGCTGTCTCTCCAGCCCCTTACGTTAAATCTTGAAGAACGGGTTAAAAGATGACAGAGCGGGGGAGAGGTACCGGAGAGA
AAAATGTGTGAAAGTAGTCAGGGCCTTGACCTGGAAGAGTTAGAGAACAAGGCAGATGCAGTTGGCAGGTCCGGAGCAAC
AGCTCCTGAGTGTTCAATGGGCCAAGTTGAGAGGGGCTTTGGATGCCATGAAAGGTTGCCGAGGAGAAGGAAGTAGAGAG
TGAGTCCTTCAGCTACCACAGGGGAAGGGTGGGAAGGGGGTGAGGAGATTTCCTAAATGTGCATCATTGGCCTTCTGAGG
TCTTCAGAAGACGACCAGTGGGGTCCCCAAAGGGTGAGACCAGACAGAACAGACAGCCACTATTCCCTACTCTGACCCCC
TACCTCAAGAGGAAAGGACGAAGAGAGAGCTGAGAAGAGACCCTGAGCCTCTTCACCACAGCAGACAGGACCAAGTTTCA
GCAGGCACCAGGACACTCAGGTGTCTACGTCAGTCGCTAGCTATGCACATGGGTACCCTGGGCATGGAAAGTACAGACTG
GCTTTCTAACACATCATTTTGCCGTGAGACCAATCCCAGCATTCCCTCTATTGTCCTCAACAGAATCAAACGTCCAAGCT
AAGGGGCTTGCCACCTCTCCAAAATAAAGGTCACCTTAGAGGTCAAGAAAGCATTGACACAAACCTTGGGTAAGCCCTAA
GACTGTAACTTGGGAGTTGTCATGACAACAGGGCAGTAGAGCCATCTCTACTCTCCTAAATGGCACGAGGTTTAGATG
AGTAACTCTGAAAGAACAGACCCCACCCCTGTTTTCCTATTTACCATCTTCAGACCCCTCCTCACCTGTGCCCCAGCCCT
CTCCTCTAGGACCTTAGCAACTTGAACAACATAGACACACGCCTCCTCTCAGTGTCCAACAATGAGCCCTCCCAGCAA
AACAGGAAGCCTCTTGCTGCCAAGGCATTGTGCTTGCCCTGTCACGTGTCAGGAAACCACTACCGTGCCATGGCAACAGG
TGTTGCTGAGACCTTAGCCTTCCCGCCAACCTGTGCCCTGCCCTTCTCTCTCCTCCTTACTCCTAGAGAGTGTCCTCGTT
GTGCAGTGTCCTTGACTCACCTGGACACTGCTGTGAGTGAGCGTCCTAATTACCTTAACTTGGATGAGAAGACTAATTCA
CCCTATAAGTCAGTGGCCCAGGGCCAGGGCTTGGGCCTTAGACTGCATAAAAAGGAGAAAGCTTGCCGACTGCAGGTGTT
CATCTCCCCTTGTTTCCTGCCTATCAGTGCAATGTCACCAGCCACTCCAAGCCAATGTCACCACCAGCCACCCCAAGCTC
CTGCTGCCATGATTCCCCAACCAGGATGGACTCTACGCTGGAACTGTGAGCTGTATTAACGCTTCTCCCCTAAATTTTTC
TTGTCAAGGTGTTTTCATCACAACAGCAGAAAAACCAAAACCAACTGACACTTCCTCCTTGCAACTGGCAGCTCACATCG
CTTGCTCCTTTCCCATTTGGACACCAAAGATCTCACTGATCATCATCCAAACACAGTTCTTCATGCTAGCCAGGCAACAT
AAGAATGTACCTGTGTGGAAATTTAGCAAGCTTCTTAGCTCTTTTGGATCGCAAAAGTCTAGGTAAGGTCTGAGATGACT
CTCTCTCAAATTCCAATATCCCTAAATGTATGCCAGACCTTCCTGTAAGGTTGCTGAGTCCTGAAGCTAAACTGACTGTC
TTAGTTAAGGTTAGTAGTGCTACCACACCAAAAACAAACAAACAAACAAAAAAAAAATGAACAAGCCAGGCGTGGTGGT
GCATGCCTTTAATTCCAGCACTCGGGAGGCAGAGGCAAGCAGATTTCTGAGTTCAAGACCAGCCTGGTCTACAAAGTGAG
TTCCAGGACAGCCAGGGCTACACAGAGAAACCCTGTCTCAAAAAAACAAACAAACAAACAAATAACAGCAACAACAACAA
AAACATGAACAAAGCTATTTGGTTGGGGAAGGTTTTATTGGCTCACACTTCCATATCATGGTCCATTATCAAAGGAAGT
CAGGGCAGGCACTCAAGGCAAGGCAGGAACCTAGAGGCAGGAGCTGATGCTGAGGCCATGGAAGAGTGTTGCTTACTGGC
TTGCTGAGTCTGCTTTCTTTTATCATCCATTGTTACATGGAGATTAATCAAAGTTGCATGAAAAGAAGAGAGAAGGACCT
CCTCAGACGCAGATGGAAGCATGTGGTCCTTTGTTAGGACTCTGAAGGGCAGTGGTCTCTCAGAAAAAGGCTGTCAGCCA
AGGCACAGGGGGTTTGAGGGTTTTATAGGACAGAAAAGGAAGCGTTTGTGGTTGGAAAATTCCATCTGCAGATCTGTTTC
CTCAAGTCTCAGCACTGTAAATACCAACGGAGGTTCTTATCCATTAGTGAATTTCTTGTCAGGAGAATCCCGGCTCCTAA
CTGCCTGCAGATGTTAGCAGTGTATCCCTGGTGCCATCCGGAGTTTCTCAGCTCTCCTGGGGCTTTTAGGTCATTAACCC
TTCACCCAAGACCAGCAGCAGCAGCACCGCCCAGTGAACTAGCCTCAGAGACTCACCCTACAGGCCAATCTGATGGACGC
ATTTTTCTCAACTGTGGTTCCCTCTTCTCAAATGACTCTAGAGTGTGTTAAATTGACATAAAACTAGCCAGCACACTGAT
GATTCTGGAGTGAAATCAAAAGGAGCTTGAAGATGAGGGTGGGGACAGAGGCAAGGGTATGTCTTAGACTTTACTCATTG
AATTGCAAGCCGAGAAGGATTAGAAGACATCAGCAGTATCAGTGGACAAGTGGCATTTGAATGCCATATGGAAGAAGGAC
AATAAGAAGTTCCTGCAGACCTGGTTCCATCCAGAAGCCTCTGCTCTGACTCCTCTCCCGGGACTCTTTAGGTCAAGGAC
TTTCTCCAGGGGTCTCAAGACTTTCTGTGTTCCTTTATACTAAGTTTTCTTTCATCCTGTGAACAATCAAGTCATCAGTG
AGGTGGTTGGGTGACATGGCCCATAAAGATGACTTCCAGGGACCTGGTTCCTGCTCACACTCTGAAACCTTTGCTCCTGG
GCAAAACTTCCCCTAAAGAACAATGACACACATTCTCAGAGAACAGAGAACAATAAGCCGTGTGTAACAACATCTTCCAG
AGAGAGAGAAGCGTGGATTTAGTCCTTTTGGGCCACGCTCAGCATCAGCAGGAGCTAGCTTGACATAAGGAGAAAAGCCC
CTTAGCTTGGAGCTAAAGGTCACATCACTTTTAAGTCTCAGTTTCTACAGCCACAGAACAGAAAGTTACCAAACACAGCA
GCCTGAGGGTTTCATGTGAGAAGGTCATGGAGTCCCAATCACTCACTCCTTAAACTTGACTCTGTCTGCAGCCCCCAGCT
GGCCTTGAACTTGTGGCAGTGGAGAATGTGCTTGAAATACAGAAGCCAAATAGAAACCTGGAAATGTTACTTGTCACTGG
TTGGAAGCCCTGGACCATGGTGGATGGACACTCGATTCTGAGTACCTACCCCCAGGACTCCCCAGAGTAGATGTTTATAG
TTGCTGGATGATGGTGGATGGACAGACAGACACGGACTGGCGCCTAAGAAAAGTCTAGCATGTCATCACGTACTCCAGCA
GCCCGAGAGCTTCATGTCAAAGACACAAAGCAAAGCCATCTCATGTGCCAACCACAACCTAGCCAATGGGCAGTTATCAA
```

```
AGCCAGAAATCTGGCCTAGAGGCCATAGCACTCTAAAATGAGGCCAAGTTAAACTATGTGGCGCTCTCTGCCCTCCAAAC
TGCCAACTTCAGCAGCCCTTTTGAAGCTGAGTCAAACTCCTGACCATAGCGTTTCTTTTCACCACTGCCTTCCTCCCTCA
CCTGGCCCACTCCCTCAGCTCTCTAGGTGCCACTCTTCCCATAGGAGTCTTGGTTGGACTCCTACTGTGCCTAAGCTATG
CCAGGCTGTTCCCTAGCTAAGTCCTGCAGTGTTGTGATGCAATATTGTCCTCCATTATTTCCTAAATACCATGAAATATT
TAATATATAAAGCCATAAAAAGCCGGGCATGGTGGCTCACACCTTTAATCCCAGCACTTGGGAGGCAGAGGCAGGCAAAT
TTCTGAGTTCAAGGCCAGCCTGGTCTACAGAGTGAGTTCCAGGACAGCCAGGGCTACACAGAGAAACCCTGTCTCGAAAA
AACAACAACAACAATAACAACAACAAAAAGCCGTAAAAAATAATGCAATGAAGACTTTAGTTCATCATAAATAAAACATC
ACCTTCCGAGCCCTTCCCAAAATTAAGGTGAGCCTGTTTGTGGGTGCCATTTCTCTTCATATTGTTTGAGAGTGATTGTG
TCTTGTCATTTGTCTCACTTTGTGTTCCTTGGTTTTTTGGCCACAGAGTCTCACTCTGTAAAGGTGGCAGATCCAAGTGT
GGCAATCTTCCTACCTCAGCTGCTGCTGCTGCTGCTGCTGCTGCTGCTGCTTCTTCTTCTTCTTCTTCTTCTTC
TTCTTCTTCTTCTTCTTCTTCTTCTTCTTCTTCTTCTTCTTCTTCTTCTTTTTCTTCTTCTTCTTCATACG
GTATTCTGCCTCCATGTATACCTGCAGGCCAGAAAAGGACACCAGATCTCATTAGAGATGGTTATGAGCCACCATGTGGT
TGCTGGGAATTGAACTCAGGACCTCTTCAAGAGCAGCCAGTGCTTTGAATGTCTGTGCTGTCTCTTCAGCCTTCCTCCTC
CTCCTCCTCTTCCTCCTCTTAATAATTTACTTTTATTTTATGTACATTGGTGTTCTGACTGTATATATGAGGGTGCCAGC
TCCCTTGGATCAGGAGTCATAGACAGTTGTGAGCTGCCATGTGGGTGCTGGGAACTGAACCCAGGTCCTTTGGAAAAACA
GCCAGTGCGCTTAACTGCAGAGCCATCTGTCCAGCCCCTGCCTCAGCTTCTTAGTGCTAAGATTACAGGACTTCATCAGA
ACCCTCTGTTGATAAGACAAGATCCATCTTTGTCTATCTTCTTCACTGTGGTGGACTGGATACTCCCTACCACCATCACC
CACAAAGATCATATGCTAAAGTTATGACCCTACGATGGCTGGATTTGAGAATAAAGCTGTTGGGGGTTGGTTTTAATGCT
TTGAATTAATCCAGCCCCCAAAATCGGGAAACTGCATGTCCAAACACTAAAGATCCTTGTCCCCAATTGGTTTTTGATCA
ATCAAAAAAGAGCCAATGGCCAATGGTTGGGCAGGTAGACTGAAACAAGACCTTTAGATTTGCTCAGGCTGGGAACTGGG
AGAGAGGAAGGAGAGAGCATTGTCGTGGTTTGGAGGGAAACAGATCAGAATTAGAGACGCAAAAGGAAAGCATCCGAAA
TGTAGGAGAGAAAGAATTCGGGCCCTGGAGGAACAGCTCAGAAGTGTCTTGGGCAGCAAAGACTGGGGTAGGGGCACCCA
GAAGGAGCCAGGGCAACAAAGATAAAATATAGAATTAGAGGGCATTAAGCCAGGAGTACCAAAGGGAAATGTGTTCTAGC
CACAGGGAAGATTAGAACTGCCCAGCCTTTGAGCTAGTCAAAGCATATTTAAAATTAACTGGTGTGTGTGTGTGTGTGTG
TGTGTGTGTGTGTGTGTGTGCTTCATTCTTGAATCCAGATAGCTCTTGGGCAGGTGCATGGGTGTGACGAATCGGGAG
CACAAAATGATTTAGCTAACTACTGCTACGTAGAGAATCTAAGGAAGTTATTTAAATTAAATAGCACTATAGAGTAGGGT
CCTGGTTCCATGGAATTGGTATGTGAGTGACATTACCAGAAATTGACCCTACTGGCCTTTGCCTTGGGACTTCCAGCCTC
TAGAACCATGAGGAAACATATTTCTGTTGTGTAAGACATCCAGTCCATGGTATTTTAAGAGTCCAAACAGACTACTCACT
CCATGTAAGTTAAGCAAGGCCAAGGATCATGTGTGCTTGTCTTTGGATCAATACTGTCCATCACTATACCTAACACATAA
GTGCATGCCAAATATCTGTTTGTTGAATGAATGAATGAATGAATGAATGAATGAATGAATGAATTAGATAATTACTAAAA
CAAAACATATAGGGTTCTGGGGTATGCAAGATGGTTTAATGGGTAAAGGTGCTTGCTGACAAGCCTGACAACCTGAGTTT
GATCCCAGGAGTAGACCTGGTGGGAAGAGGAGACTTACTCCTGCAAGATGTCCTCTGACCTCAACAAGCATGCTGTGCCA
CATGCCTGCACACAGCTCTTTCTCCCTCTCCCTTCCCCCACCCCCATTCTCTCCTTCCTTCCCTCATGCCTCAACATGAA
AGCTCTCGGCTACTGCTCCCATACCTTGTTTGCCTGCCTGCCTCCTGCCATGCCATCATCCACCATGATGGATGTGAACT
CATCCTCTGAAACTGTAAGCAAGCCTACAATTAAATGCTTTCTTATATAAGTTACCTTGGTCTTGCCATCTGTTCACAGG
AATAAAAAACTAAATACAACAAATTATACATGCCCCCCCCAAAAACCCTTGTAAATGAATATTTATAGCAACAATACTCA
TGATAACTAAATAGTAGAAACAATCCAAATAGCCAACTAATAGTAGATAAAACAAATGTGGTATATCCACACAATGGAAT
ATTATTCAGCCACAGAAAGGAGTGGAGAGTTGAAACATACTCCATATAGATGAACCTAATTAAAAGAAGATAGATATGAA
GGTAACATTGTGTCTATTTATGTGCATTAATCAACAGAAGCAAATCTACAGAAAGAAAGTAGGTCTGCGGCTCAGAGCAG
TTATGTGCACAGGTGGAAAGGACATGGGAATCATCGGAAATAATGGCTGAGGAGATCTGACTAAAAATGATTCAAAGTTT
ACCGTGGCGAATAAACGCCGTGACTATATAAACACCACAGAAATGTCTACTTTAAAAGAGGAAATGTGGGGACGGGAGAG
ATGGCGCAACAGTTAAGGACACTTGATGCTCTTGCTGAGGATCCAGGCTCAGTTCCCAGCACCCACACATCAGCTCACAA
GGCCTGTAACTCCAGTTCCAGAGGATCTGACGCCGTCTTCTGACCTCCACGGGCTGTAGGCACACAGGTGGTACACAGAA
AGGCACTCAGGCAAATGCTCATACACATAAAATAAAAATCAATGAATCGTTTTCAAAGGTGAATTTAGATATGAACTATA
GCTAACTAGAGCTGTTGTCTTTTAAAAGAAAGGAAGGAAACACAGATGAATTCCTTTCTTTCAACCTTCCATTACCTGTC
AGTATAACCTCATGGCCAATTCTCTCCAGAAGCTAGAGGGAAAGTCGTCCGGGAAATGTAGTCCCTGTATGCAGAGCAGA
GCAAGGGTATCAAGTGAGTGCCTAAAGGGTTATGTTGTCATGGCAACTGTTTTAACTATTTATTTATCTTATGTATATGA
GTACACTGTCACTCTCTTCAGACACACCAGAAGAGGGCATCGGGTCCCATTACAGATGGTTGTGAGCCAACGCGTGGTTG
CTGGGGATTGAACTCAGGACCTCTGGAGGAGCAGTCAGTGCTCTTACCCACTGAGCCATCTCTCCAGCCCCCTGTTTTTA
ACTAGTTAATACAGAAGAAGGGCTAAGTCATTCTGAGCTCTGTGTCGGGTCCTGTCGCTCACTGAATTGTCTACAATCAG
TGGCTAGAGATAAAAAAATTTACAGGTGTCTCTCCTGGAAATGGAACATTTAGATATTTGCTCTAGAACTCAATGGCCAC
CCACAGTCCCCACATGATCTCTATGCTTGTTTTAAACCCACTCCTTGGACTGGGGAGGTGGCTTAGTAGCTAACAGAGAA
TTGCCCCACAAACACGAGGAACTGAGTCCAAATCCCAGAACCCTCATAAGCAGCTTGGCAGTAGCATGAGCATTCGTCAT
CCTAGCAGTCCCCCAAGGAGATGGGAAGCAGAGACAAGAGACCACGCACCCAGAAGGCCAGCTCACCTTAGTATAGAATG
GGGCACTCAATAAAGACGTGATACCTGCAACAGAATGGAAGTAGGCTGACACCTGACATTGTCTCTGATCTTCACACGTG
CAATGGCATAAAATCCCCACATTCACGCATACGATCGCACACATACACGTGTGTACACACACCATACACACCACACAGGC
ACAACAATTAAAAATGCCAATAAATATATCAATCAGTTTCCCTGTGGGGAAAATGGGTGTGGTCACAAAGAATAAAAAA
AGTAAAATAAAAATAGGCCAGGCATGGTGGCATATGTCTTTAATACCAGCACTCGGGAGGCAGATGCAGGTAGATCTCCA
TGGGTTCGAGGCTAGCTAGGTCCACAGAACAAGTTCTAGGACAATCATGGCACTGACAAAAATAAAATAAAATAAAATAA
AATAAAATAAAATAAATAAATAAATAAATAAATAAATAAATAAATAAATAAATCTCTTCTTGGGGAACCAGTTTGGATAATGAC
TTAGACTTGATGTCCTCTCGGTTAGTAGAGAAAGCCGAGGGGCTCCAGGCACCCTCATATCTAGCATCTAGGTCCTGTGA
```

```
TGTTTAGCTTAAGTACTAAAAATAAACACTCAAACTTTCACTTCGTGGTTGTGTCACTGAAGCCTTGCTGGCCGGACCGT
TCAACAGACCTCACCCTGGCTGCGCAGGTGTATGTCGGGGCTGCCTGGCGGCTGCTGGGACAATAGCCACTGATCAAGTT
CATACTGAAAGGGAGACTTTTATTCAAAACCAAGAGAAGGATGCAAGTACACCTCTGACTCCCGAGCCTCAGCCTATCTC
ACCCCACAGCAGCCACCCAGGCGTGCTTGGCACTCACAGATGTAAGCAGCTCCTCCCTGAGTCTCTACAAGGCACTACTA
CCAGGGGCTTTCTGAAGGCAAACGACTCAGTGATTCAGGATCCTATGACAGCCGAATGTGGTTATCACATACCTTGTGGC
CCAGGAAGACTCTGAGGGGTGGGACAGGAGTGGGAGGGGCTCTGGAACAACTTAAAAGGGGCTCTTACCACTTCCTCCCT
GCCAGAGAGGGCTAGCTGGAGAGAGACAAGAACCAAAAGCACAGCCTTCCTGTGTGATTTCTACAGCCCCCAGAGCACCA
TGGACCCAGGTAAGTAAGCTTTCTCTTAAGAGAAGTCAAAACTTCTGACACAAAGCCAGACTCTAGGGCAATGAACATGC
ATGGGGGATAGGGGTCATGGGGGGGGGGGGGAGTTTCCAGAGCAATCAGGGCAGTTGGTGTGAACTGGTGGCTGCTTTCA
GAGAATGCCATCCCGTTGGATGAGGCATTGGAGAGAGGATGTACCTGCTCTTTCAGACCCAGGGGAAGGAACAGCTGTGG
ATGCCAAGACAGACACAGGCATAAAGTTGGGTCCTCTCAAGCAGCAGGCAATGAACAGCCTGGTAGACTGACCTTGATGC
CTGGATAGAACACGTTCTCAGCTGGTAGGGATGGGTGATGTTCTCTGGCACCTCTGGGGACTAAGAAAATTCTCTTTAGA
GAGTTCAGGACACCCTGCTCCCACTCTGGGTCCCTCCTTTCTGTCCTGTTAAACCAGGATTTGAATCTCCTTGCTCACGA
GAAGAGTGAAACTGATGGAAACTGTGCTGTGCATAGTGCAATACAAATACTAGCTGCCACCTCTGCCTCTTGTTGTCTCT
GCCTGTCATCTGTCTGTCTGTCTCTGTCTGTCTTCCTCCCTCCCTTCCTCCCTCCTACACATCCCAGGTTTTGTGCCTCT
TGCAGGTTAAAGGGTGAATGACTAACATAAAATTGAGTGGCCCAGGGGGCACTCCACGTAGAGAAACTGTAGCCAGCACA
GCACAGCCCCTAAAACAAAGCCCACACACCACAGCCCTCTCTATAACAAGCTATTCTACCAGGCAAGCCTCAGTGTCTAC
TATAGCAAAGTGCCAAGGGGAGTCCAGCTCCAGACACCCCAGTTACACATCTTAAATCTCGGCTGCAGAGTCAGACTCGG
CTGGGGCTGGAGGGGCCAAGTTCCTAGAGTTCCGCTCACCAGCCTCAAAGCCACAAGGGGTTGACTTTAAAAAGTGAAGG
TTCCCTGGTGCTGTCCTCACACCCCCTCCAAGCCACACCACAGCCAGCAGCATCTTTATCTGTCTCTCCTGCCCAGCGTG
CATTGCACCCTAGATCTAGATCCAGCTGTGGAAGGTATATAGTGGGGTCCACCTCTGCTGGCTATGAACTCATGGGAAGC
AGGATTTTGTTCTTACACCTCTGCCTCTCAGATGTTTTGGGTGTAAGTAAAAAGTGTTGATGGGAGGGAGGGAGAGAGGG
ATGGAGGAATGGATGGATGGATGGATGGATGGATGGATGGATGGATGGATGGATGTGTGGATGGATGGATGGATAGAATC
AGCCTCTCTAAGTATATTTTAGAAAGCAAATATCTATTCCAGCTTCTCCTCCCACCTGCCTGCTTTCTAAGCCTACACCT
CAGGTATCTACCTCCACCAGCCATAGTCTCAGAGAACCCACACCATCCACCAAAGATCTTCTCCACCTTCCTAAAATGTG
GCTCCCAGAATAGGGAAAGGTTCTAAATTTACAGCATTTGCCAATTTCCATGGTGTAAATTTTACCATCCCAATTGACCT
TAGTCGGTCCCATTAAAGGTCTAAATGTTTTGACACAAAAAGGAGACAGCACCTGCCTTCCTTGGCACTTGGGACACTA
TAGAGTCTCTCCTGAACTAGGTTTGGGGATGCTAAATGCATGGCTTTGATACAGGGGGTGGGGCAAACTCTGGTCCTGGA
AGAAGAGATAGTCTTGCTTTTTTACTCAACTTAAGAAGCACCCTAATAGCTAACTCTGAAATGCTGGTGTGAGGGGAAAC
TGCTTAAGAAGTTTTGCTCAAAACAGGCTCCGGCGCCGTTGGAAGGGCGGGTGGGCCACAAACTATCTGTTTTTCACTCT
TTGTCTACCTAGTAGGGACAGGAGGTGGGTGAGAAGGCCTAGGTTTCTGTTATACAAAATGAGAAACTACAAACCAGGGC
TGAGCTGGCCTCTTTCATGATTTGAATGAGCAGCCCTGATATACCCTTCCTAACTTCAAGACATGGGACCACAGATCTCA
GCACAACATCTCAAAATCAGAGAAGGGGAGGGGGTGACAAATGAACCCATAAGAGACTTGTGTCTCACAGCTCAGCTGTA
GATGGCAGTAGAAGAGAATTCAGGGTCATTCTCTTGTGGCCCTCCAGAAACCTGGCTGTGGAGAACACTGGAGCCCAGAG
TAGCTAGGAAAAAACGCTCCTCTGTGTGGTATTGTCTTCCAGTCATGTGTCACTCATTGAGAACTTCTTGAATACAGAGT
CTGGTGGGGAACGTAACATGTATGTCAACACGCAACACCAACAGTGAGAGACACAGCACTCCTGAGGGGGCATAAGGACA
AGATTCAAAAGCAAAAAGCAAGGCTGTGAACACGTGTCCAGAAAGCATGGCAATTGTATTTTGAAGGGTGTGTAGAAGTT
GTCAAACCTAATAAAGAAGGGCCAGGTTGTAGCCAGATCTTCAGACTCTACAGCCTTAAGCCAACAAAGTAAATGAGGCA
TCCGAGTGTCAGGAACGTGACTGGCACACAAGCCTGAGGAACCTGAGCTTGTAGCCTCAGAGTTTAAAAGCTGGCTGTAG
CAAGGTACACACGTAATACCTAAGCCTGCTTAGGGAGGCAGGAATAATAAGACTTGCTGGCCAACCAGTCTTCAGAATTG
GCAAACTCCAGGTTCCATGAGAGACACTGATGTCAGCCTCTGACCTCTGTATGTGTGCACACCCATACATATATGAGCAC
ACACCTTGAGCTCATAGTCAGTCAGCTCTGGTGAAGGCAATAGAGCAACAAGGAAGGGGGAATGGTGTCAGCTGATGGGA
GTCAGGGAAATAGATGTGCCAAGAAACAAGCTAGACTGGGGTGGCCCTTACAGCGCTGTGTGACAGCACCAGGGAATCCA
TACACCACCCTAAAAAGAATGGGGGCCAGCAAAGGTCCCCCACCCTTTTCCAAATAGATGGGCAGATAGATGAAATGTGT
GTTTTGGAAGGAGTAGGGGACAACAGATCCTTGTATCAGGACAGGGGAGGGACCAACATCAAAAGATGTGGAAGATCGAG
ATGTGGAAGCAACAGACACGAGTGGCCAGAAGCAGTAGAGAGAGCAAGAGTGGCCTTGATCTGTGGAGGACCCTCCACAC
CCAAGGCTCATCAGTAATGTCACATGACTCAGCCATTAGAACCCAGGCTAGATGTTCCCCTTTCCCATCTCTGATCTATT
GTTTAATAGCTATGTGAATCCAGAGGAACGACATAACATCTCTGGGCCTGTTTCCTAGAATACAAAAGGAACTTACAAGA
TGTTATGAGGGGGGAGAGATGTAGGGAGAGGAGCTAGGAGTGAGGTGGCCATGGCCAGGGGCTGGGTGTCCTTAGGGAAG
CCTTGTGCTGACAGTTCTTCCGTTGGGGCTGATTGTCACGTGGGGTCTCACAGGGTATCCATTGAGGACGGGGATGGCAG
CGCCTGTCTCACGTGGACAGCGCACTTCACTGAAAGCTCAGTGGGTGCCAATTGCATCACTGAGTGACAGCGTTGGTGCC
CTAATAACAAGATCCCACCAGCTCTCACATCCCTGCCTCCCATCTGCGGGCTGGGTCCCTGCTTCAGCCTCTGCCCTTTC
TCATGTCCCTACAAGCGGCTGAGAAGGTCCAGAGAGAAAATCATCACCTGAGGTGTCTCTCGGGCCAGATGATAAGCAG
CCATGGTTTCCTGGGCAGCCTGGAGGGACTGGGGGAATGTACAGATGGGAGCTTTAGGACCTGAGCAGGAGGCGACTGGC
TTCCCAGGCTGGGAGCTGGGGGCGGGGGAGGGAATTGAGTGTCAGGCTGAGGCAGGGCAGGGCAGGGGAGTTGGGATGGT
GTCGGGCTAGAGTGCTAGGGAGTTTTCCTTTCACCTTAATCATTCTGCAAACCCAAGAACTCAGGAAACACCGAACACCT
CAATCAACAGCTCCCCCACACTGGCATTCCCAACTGCCCGTAGCTCAAGAGCAGTTGCTTTCTGACTATTGAGTCATCTG
ACTCACCAACACTGACCAGGTCCCTCTGTCACCACACGTTCAAATTCCCCCAAATTCTCCTGTTAACTGCCTTGAACATG
GCCTGTCCTGGATCTACAGGACCCCGACTCACCCACCCAACCCCCACCCCCAACCTCATATCCAGTATCCCTGGAATGAG
ACATCAATTTTGTTCACACACCCCAGAGCCCCTCCGGGTTTGTGGCTACAAAAGAACCGGGACAGAACCCAGAGTAAAAC
AGAAACAGTTCTTGTTCCTTAGGGGTTTCTCCACGTCCCTGACAGTCAGATTGGGCTTTCTCTACAGTAACCGAAAGCCG
```

```
TTGATGGGTTGAAAGAAACCTCGAACGCTGAACCCCGAAATGAGTCAGGGAAGGGCGGGCTGTGCATCAGACCCTGAAAA
TCCTATAGTTGAGAATGGGCATGTGACCTCAAGCTCGTGGGGTGGGGGTGGGGTGCCAGGGGTAATTCAGTTGGGGGGGG
GAATGTGTGAGAGAGATGAGCTGTGAAGCCACGTGGTGATGTTCCCCTTCCTGTCTCCAAACGTTCTAGTACAAGCTGAT
GATAAGGGGACAGTGGCTGTCTGTCCCCAGAAAAAGATGGAGCTGGTGAGGCATGGGCCCCACGCTAAGCCTAAGCGTCT
CTCTATCTCTACAGCTAGGATTTCGACTTATGGTTAGGGATTTAGGAGGGAGGCAAGATAGGAATAGAGGGCAGAGTTAG
CTTCAGTAAGACGTGCTTACATAGCATGGAGGAGAATGTAGAGGGGAAAAACGTTTGTCCGGGTCACCCTGATGCCCTTC
CTGCCTGTACTGAGTGACCAAGCGTAGCCTCCTCAGCCTGGAGTACATGTCAGGTACCCTGGGTGTCATGTAGCCTAGTC
TCCAGCAGGTGGTGCTATGGCCAAGGACAAACAACATCTGCGTTTGAACTGTTTCCCTGGCCTCAAGAGGTCCCCCAGAC
TCAGATTGGGGAGCAAAGTCCTACTCTGGACTGCAAGACCCATAGGGTCTGACTCTTCCTGTAGATCTGAAGAACTGGGA
TTCAAGGGCTGGAGAGGGGTGCCAATAGCAAAGAAGGCGGATCAAGGGTCCCCAGGGGTGAGACGGGAGTATGTGGGGTA
GAGGTCTGGGGTGGGGGGCATAAGATGTCTGCAAAAATTACTTTTCCAGTTGTCCTCTTGCTTAGCTGTCAGGAGAAAAT
ACAGCCCAGGCTGGCTCAAGGCTCAGCTCTGAGCAGAATGAGGAGTGGTCGCTGTCATATGCAAGCCTGTTCTAACCAGT
GGTCTCTGTCTGCAGAGTATTCGAAAACACGTTGAGGGAGAGAGAACAGATTCCCATTTCCTTAGCCCTTAGAACATCTG
TGAGGCTTCACATGTGGCTGTCTGTGCCCTTGACCATCAAGGAATGCTCCCAGGCCGGTGGATCTTGCCACCAAGGAAGT
GTTGAACATGTGTGCATACATCCCGACTACCACAGGGCTGTGCATCATGTGGAATGGGAAGTTGGGGGGGGGGGAGTCAG
GACCCTGTGCGGAACCAGCAGTCTGGAGGAGTTATTGGGGTACAGTGGATACTGCAAACCAGCAGCAGCCTAGACTGGCC
AGGGCAGGGGAGGGGGGAGGGGAATGGGAGGGGGTATTATTAATTTCTTCCAGCCTCAGTTTCTTCCAGCTTACAGGTTCC
TTGGCTGTAAAATAGGAATAATGTCATCAGTATTGGGANNNNNNNNNNNNNNNNNNNNNGAGGTGAAGACAGGGAATGCTG
GGCAGCAGGCCAGGTGTTCAAACACACAATCTGGTTCTGATGTTCTCTTTCAGCAAGCCCGAGGTGCCTGGAGCCTGGGA
TAAAAGTTCCCAATGGTGCCCCTGCCCCACCCCCGAATGTCTCCTTAATCACCGAGCAATTTTTACCGGGGCGGTAACAT
CATCTCCTTTGTCTCCTCTAGGGAAACCCAGGAAAAACGTGCTGGTGGTGGCTCTCCTTGTCATTTTCCAGGTGAGGCTT
CCTGCCAGGGCAAGAGCTCTCTTCCCTCCTCCACACCCCTCCCCAGCCAGGAGTGCTGGAACACAAACAGACCGCTGCA
AGGAACGAAAGGTCTAGACACAAACCCACCTGCCGTTCACGGCCCCTTGCCCCCCCCCCGCCTCCCCCCACGAAGGGGGC
AGGCATTCTCTGGACTTCCATTGTCCCCCTTTAAAATAAAGCCCAGGGAGACGTAACCTACCCCCACCCTCGTTCTTCCC
AGCTCCCACCTCCTCACTCAGGAGACAGAAACAGTGTCTGAGATCTCTTAGACTTGTGCAACACAGGGCTTAGAGTGGGC
CTGGTTTTGCACATAGGTGCACACATCATCTGTGAACTGAGAAAAGGACCCTCGGTCGGGCGCTCTGGTTACTCCCCAGT
CACCCAGAGTTGCTTCCTCTTGCCTTTCAGTCCAAGGGTCTCCTTGTAATCTCATTCTGTTTTCCTTCATAAAATAGAAA
GACCTTGGCTTGGCTTGCCTGTGGGGAGCAGGAGGGGAGAATGAGTGGAGCCCGGGACCCGGGTGGAGAGGACGGTGAAGG
GAACACATAGTGGGTCTGAATTCTGCAGAGCCAAGAGCCCACTCCGTTCTCTATCCCAGCAATGGAGGCAGAACGTGTGT
CTCCCCAACCACCAGAGTCAGGAGAAACTCTGAGAGTCAAGTTTCCTCAATTGGGGTAGGACTCTCTCAAGGTGGGGAAT
GGCCACGGCCAGCCTGGAGAAGCAATGCTTCAGGATTTGACTTTCTCCCAGGTCCGGAGGCCAGAGGGGGAGCTTTCCAG
GAGCTCAGGCCAGGCACAGGCCCCAGGCTGGGCACTGTTCCCCAAACCCCTCTCAGAGGCCCCCTGGAGCTTGGGTTGCA
GCCCCTTAGCCTGGCCCTTTCCCCATCACCACTCCCGCCCCTCCTGTACTGCTGGCTGGAAGGCCAGAGAAGGGTAAAGA
CATCTGTGGCTGGTTTTTGTCTTTCACTTGGGAGGAAGGGGGGGGGGGGGTTCAGACAAGGCCGGATGTGGTGGGGCTGT
GGGCTGCAGAGGCGGCAAGCTTGGGGGAAGGGGTGTTGAACAGGAGGGAGAAGAGAAGACTGGGAGGGAGGTGGTGTGGG
AGCACACTTCATAGAAGCTGGGAGTTCTGACCTCCAAAGGGTCTAGAAGCCCTTTAAGCCTCTGGGGCCGCAGACCTAAG
GCCACAGACCCATTCGTGAAGAGCCAAGCGCAGCTAACCTCTCCCAGGGCCCCCAGGCTGCAGTCAAACCTGGACCAGGG
AAACAAGGTTGCTATGGACTCTTCTTCTCAAGTTAGCCATGCCCCAAGAAAGGGCTCCTGAACACCACTTTGTCCTTATC
CTGTCGGTAGGCTCACAATGGTGGTGCGATCTGTGGATCACCACTGAATGACTGAAAATCTCCTACCCAGAGGAGGAACC
CTAAACTTTCCAAAGTGGGCCTTCAAACCACCCCACCCCCTCCCATCTGTGGTGGTCCATCCATCACACACAGGCAGACG
GCAGGCACCCCCTCCATTGATAAAAATCACAGCAGCTGCTGAACCGTCCCCCACACGCACCCTGCAGCCCTGCTAAAGGG
CTAAAAAAAGCCCAGAACAGCTGAGCCCTGCTGATTGGGATCTGCCGGAAGAGAGGCAAAGAGGTGTGGGAAGAGGGTGG
GAACACAGAGGGAGAGTGGGGGAAGAGAGCCGCTTCCTTCCAGGGGCACAAGCCGCCATAGGAAGGCAGTCACATTTACC
CAAGCACTACCCCACAGGCCCTACGGTACATTTGTCTACGCGCTGAGTGTAGTGGGTACCAGGGAGCTGTGAAAATCCCC
ATGTCTCTTATCAGCAGTCCCTCGACTGAAGTTTATTCCTACAAAGGAGCTTTGATCTTCTCTTCCTGCTTCGGACCTTC
CTCAACCTTGAACCTAACATAGGGGGTTGTTTTTTTGTTTTTTCCCTCGAAAGCTGAGGCATTTTGTAGCCCCCACCCCA
AAAAGAAACTCTCCTGGGACCCAAAAGAGGCTCCAGAAGAAAGACACACTTTCCCCCCACATCACAGGGTCCCAAACCC
CACAACGGTCCAGCACAGGAGTTGCGATTTGCCTCTATCTCCCAGACACAGAGAAGATGTGCGATTTCCAGCTCAGGGCT
CAGCAGACCCCAAAGCCCTAGACTATCCCCAATCTCAGCCAGTGAGGATGAGACTGGATGTCCCAGGAGTTGACAAAACA
TCAGGCCTTTGCTACTGATGAGTAGAAATGTAAACTTTCTGGTCCTGCCTGAGTCTGCAGGGCCAGACGTTTACATCTTA
ACTAGATCCTCCCTGAGTAATTCATACATGTGCTAACATCGTTACTTAGATTTATTTTTTGTTTTGGTTTGGTTTTTTGG
CTTTTTAAGATTTTTTTATTCATTGAACGTAAATGCATACACTGTAGCTATCTTTAGACACATGAGAAGAAGGTATCAGA
TCCCATTACAGATGGTTGTGAGCCACCGTGGTGGTTGCTGGGAATTGAACCCAGGACTTTTGGAAGAGCAGTCCGTATTC
TTAACCCTATTTAGGATTATTTTATTTTATGGGTATAAACATTTTGCCTGCGTGCATAGAAGTACACCACATGCACAGAG
GAGGTCAGAGTTGGGCATCAGAGCCCATGGAACTAGAGTTACAGAGAGCCACGACGCACCGTGTGGGTGCTGGGAACCGA
ACCGGCCTTCTCTACCAGAGCTACACGCACTCTGCTCTTAACCCCTGAGCCAGTTCTTCAGCCCCACATGCTGAGGCTTA
AGAGGTGTGGATTTCTAGTCAAAGACTCACTTGGGGTTTGAGGGGGAAACTATAGTTTCCTGGGCTCCTCCGTCTAAGAT
GCTGACCCAAAGGGTCTAAGGTCTGAGAGTCTGCAAGAGAACAGAAGCCCCGGGCAATGTCCTGACTGTGAGATCCGGAC
TGTGAGCTCATCAGGTGCTTCCTTCCCCCTTAGGTGTGCTTCTGCCAAGATGAGGTCACCGATGACTACATCGGCGAGAA
TACCACGGTGGACTACACCCTGTACGAGTCGGTGTGCTTCAAGAAGGATGTGCGGAACTTTAAGGCCTGGTTCCTGCCTC
TCATGTATTCTGTCATCTGCTTCGTGGGCCTGCTCGGCAACGGGCTGGTGATACTGACGTACATCTATTTCAAGAGGCTC
```

```
AAGACCATGACGGATACCTACCTGCTCAACCTGGCCGTGGCAGACATCCTTTTCCTCCTGATTCTTCCCTTCTGGGCCTA
CAGCGAAGCCAAGTCCTGGATCTTTGGCGTCTACCTGTGTAAGGGCATCTTTGGCATCTATAAGTTAAGCTTCTTCAGCG
GGATGCTGCTGCTCCTATGCATCAGCATTGACCGCTACGTAGCCATCGTCCAGGCCGTGTCGGCTCATCGCCACCGCGCC
CGCGTGCTTCTCATCAGCAAGCTGTCCTGTGTGGGCATCTGGATGCTGGCCCTCTTCCTCTCCATCCCGGAGCTGCTCTA
CAGCGGCCTCCAGAAGAACAGCGGCGAGGACACGCTGAGATGCTCACTGGTCAGTGCCCAAGTGGAGGCCTTGATCACCA
TCCAAGTGGCCCAGATGGTTTTTGGGTTCCTAGTGCCTATGCTGGCTATGAGTTTCTGCTACCTCATTATCATCCGTACC
TTGCTCCAGGCACGCAACTTTGAGCGGAACAAGGCCATCAAGGTGATCATTGCCGTGGTGGTAGTCTTCATAGTCTTCCA
GCTGCCCTACAATGGGGTGGTCCTGGCTCAGACGGTGGCCAACTTCAACATCACCAATAGCAGCTGCGAAACCAGCAAGC
AGCTCAACATTGCCTATGACGTCACCTACAGCCTGGCCTCCGTCCGCTGCTGCGTCAACCCTTTCTTGTATGCCTTCATC
GGCGTCAAGTTCCGCAGCGACCTCTTCAAGCTCTTCAAGGACTTGGGCTGCCTCAGCCAGGAACGGCTCCGGCACTGGTC
TTCCTGCCGGCATGTACGGAACGCGTCGGTGAGCATGGAGGCGGAGACCACCACAACCTTCTCCCCGTAGGGGGCTCCCC
TGCCCGGACTACAAGGACCTCTCCCAGGAGCCTTAATGTGGTGCACACATGCACAGACTCTCCATCCACCGAATTGCTGC
TGAGGGAAGAGCAATTCTGGCCAGTCAGGTTGACATGAGGACCTAAGAAACTGCTTAACCCCATCCCACTTATAACTACC
TCAACCAAAGCTGTAAAAGATATGGCTGAGAAGTTAACACTCAAGCCAAGACAGCTATCCCCAAAACGACAGCCAAAAGT
GAAAGTGAGAGGCTGCCACACTTTCCGGAGTGAGGGATGTGGGGCCAGTGAACACCCTGGTTGAGTAGTCTTCGGAGGCC
TCTGAATGAACCTGCTTCTAGCTTAGAGAGATGTCCCGGAGATTCAAGGACAGAGCTTATCTCCACACTTAGCAAGCAAG
CAAGAGATGACAGTCTCTCTAAATGCTCCCACAGAGCACCCCTGCCCCTCCCTTCTGCCTCTCCACCGCCTTTCCTGAGG
TCCAGGCCACACCATCAGCCTGAAGGCAGTCCCAGCTGGGGCTCTGGATGGCAATGACAAGTAGTTGGGTCTCTATGATG
GGAATAAAAAGGTAGGGGAAAGGTGACAGAAAGGAGAGAAGGTGACCCTGCTGGCTGACAGAGGCCAGCAAGCTACTTCT
TTGTTCTCTGTCAGCCACTGATACCTTTCCTCATGTTCTGCTTTTGATTCATATATCTTTTATGAAGAAACAAATAAAAA
AAAATTTTCCCTCGAGGAAACAACTTGGAAAGAAGGGAGGAAATTCCTTGGTTAAATGGCGAGTGAGTGAGTTGTCCCCC
CATTCCACCTGAGAGTCCTGCGCCCCACACCCCTCCGCCCCAGCCTTCCTTTCTCAGCACTCAGAACCATGAGGTCACAG
AGCCTCTCCGGAGATGCAAACCCAGGAGGCTGCAAGAGGTGGAAAAAGAGCGAAGACAGGATGTCTGTCTTGCACATACA
CCTGCAGAGCCCGTGGGATGGGACCTAGGCCCTTACAGGCAGCAGTATACATAGGAGAGCACTCTGAGTATGGAAAGACG
AGGTTGTCCCAGGAACTCCTGTGTCTGGGAACTCAGAGGAACCCCGGGTTGGGGGGAAATGGACACCTGCATCCTGTTGT
GTTTGCTGTAACAGGATAGCCCGGAAACAGCCCTAACTCTTGTCAGGCCCCTTCAGACTTGCGTACATTCCTCTAAGAAC
TCCAGTTTGGTGGGAAGCTCAGACAGAGGTCATGACATTCTAGAGTAAGGCAACTAGGGACTAGTCAGATGCCACAGGCT
GGATGGGACTCTGCAGGGTGTTTGAGAGCTAGGACCACTCGGTCCCCTCCTTTCTACCCACTAGTAACAGAAGTTACTGT
TACCAGCCTGACCCCTAAGTGCCTCTGTAGTGCGAGTCTCAGTCCATCCCCGTTTCCTGTAACAAAACACTGCGGTCTGC
GGAACTTATACACAACAGGAATCTATCTCTCACCATTCTAGAAGTGAGACAACTTCCACTCTAGTATCTCCCAAGTCCAT
TTATAAGGCCTTAAATCCATTCATCAGGGTGGAGCCTTCACGGCCAGCCAGTTATCTCTCAACCACCCCACCCTCCAATA
CCATCATCCCTAGGGTTCAAATTCAAACACAGGCCTATTGGAGGGACACACACATTCAAAAGACAGCGGTGTGGAAAGCA
GAGGGAGAGGGCCAGACTCTGTCGCCCCTCACTCCATCCCCACAGGCCAAGTGTCCTTGTCATTGTGTCCCAGTGTCTAA
CCAAAGCCCTAATTTCCCCAAGCCCGAGAGCTTGGTCCAAACTATGATACCTGGGCAAATGTCCAATGGCTTAGCACCCG
AATAGCTCTGAGCCTGGCGGTAGGATGGGGGTGGGGGGTGGGAGAGAGAGGTGAGACATTCACACAAAAACATAGTTCCA
ATCCCCCCCCCCCCAAGAAAAAAGAAAAATGGAGCCTGTTCCATAACAGTGCTCATTCTCTAAGCTTGTAACCACAAACA
CCCATTACCCTCCTCCACAGGATGCATGGGGCTAGAAAGACTTTAACACAATTTGTCAGGCGGCCTCCCGGTCTGGAATC
AGCATCGTGGGAGGAACGAGGCTGGCTCAAGTGCAGGCAGTGGCTGGTGGCGGGTGGCGGGTGGCGGGCAGCTTTCGGGA
TGCTGGTACTGAATATTAAAAAGGTTCAGTTGCGACTGTGTGACCACAAGCACGCTGCAAGAAGGCGATGCCACTGGCCA
CGGTCATCCCCAAGGGGAACCTGCCAGAGTGCACGTGGCTTTGTAGATCCTAATGAAGTTACAGATGGGAAAACAGCCCT
CGATTCCTACCAGGGAGGAAATAATGGAGGGATCACGGAAGGGGAAGCTGGGCCAACCACACAAGCATGCCACCCTGGCC
TCAAATACATTGCGCTAGTATAACTGGACATCTCCAAATATTTACAGAAACAACAGAACCCCCAGCAATGCTAATTCCCC
AAACTGCTGTGTCTCAGAAGTTCTATCCTCAGCTTCACGCTGGCCTGCTGCCTAAGACCTGGCCTGATGCACTCTTGTGC
CTACCAAGGGTGATCCCCAGGATACAAGAAAGCACCCTGCCTGAAGCCCACCCAGGTCTTCTTTCTCATTCTGCCCTTCT
CGTCTGCTCCATACAACAGCCCCATGCCTCTAAATCTGTGTCCCTTGCCACGCCATCATTCTGCAATTGCCCCAAGCTCC
TTCCATTCCACTAACAGTCCCTTGTCCACGCCCCCCACGCCCAGTCCCAACTATGAGTGTGTGTGTGTGTGTGAGCGC
TCACACACACACACACACACACACACACACACACATCAGCCCTAACTCAGATCCCCCCTGGCTGTGACCCTAGG
CCAAATGTAAGACATTCCCAGGTCATGAAACTGCCCTTTCTGCTAGAAGTGCCGAACCTTTAATAGGTTCCTGGAGACCT
CAGGAAGCAGGGGCTGACCCCTGTCGTTCTCTAGACAGAGCAAGGAGCTGGCTTGTCCAAAGAGACACAGCAATGAGGGG
AGGTTGCTTAAGCTAGCTGTGGAGCCTGAGGGTCTGGCAGAGTTGCCAAAAGGGGGATGGAGCCTGGCCTACTCTGAAT
CCCAACCCTGCCGCTCCCCAGCTGTGTGACCTTCAGCTGTTGCTCAACTGCTCTGAATCTCAGATTTCTCAATTGCAAAA
CAAGGGAGATTTGGGTAAACACTTAGCACAGAGCCAGAATGTTGAGCGAGGCAGCAGCAGGGGAGCTCCCACTCCACAGC
CAGATGGCCTGGTTCATGTCTCCGCTCCCTTACGGTGCGGTGCCTCGGTTTCCAAACCTGCATAATGGAGTTATTAGTCG
TGCCTGCCTCCCAGGATTGAGGTAAGGAAGCAATTCATCTACAAAAGCAAAATATTTAGAAATACCCGGGCAAGTGATAT
CATCTTTTGGGAGATAGTTGAGAAATTATAGATTTAAAACATTGCAGGGATGGAGAGGTGGGTCAATGGTTAGAGCACTG
GCTGCTCTTCCAGAGGACCCAGGTTCAGTTCCCAGAATCCACATGGCAGCTATTAACGGTTTGCAAATCTAGTGTCAGAT
AATGAGGTACAGTGCCATGTATGTGTGGGGTACATGCCTACATGCAGGCAAGACACTCATACATATAAAATGAAAATAAA
TCTTTTGTTTTGTTCTGTTTTCATTTTTTGGAAACAGTGTTTCTCTGTAGAGTCCTAGCAGTCCTGGAATTGACTCTCTA
GAAGAGAATGGCCTCTAACTCAGAGATCCATCTGCCTCTGTCTCCCAAACCCTGGGGTTAAAGGTGTAGGACACCACCCA
CCGGCATAAATAAATATTAAAAAATAAAATAAAAATGTTTCTTTAAAGAGACAGAGAGAGAAAAGGCACAAGAGATGGCT
CAGTGGGTAGTGTTTGTTGTACATGAGGCCCTGAGTCCAGATTCATAACCCACACTCACAGGCCAGGGTTGGTGTTGCGG
```

```
ACGATGAAGAAACAGAGATGCTAGAGCCTCTGGCCAACCAAGTCTAACAGAAAGTAGAAGCTTCAGGCTTCACAAAGAGA
CCCCCATCTCAATAGATAAGCAATAAGTAAATGATGGAGAACAGAGGCAGACACCCCAGTGTCTCTCTGCCCTCCACCTG
CACACACACGAAGGAGAGAAACTCTTGCTAAGTTCAAGGTCACCACCTTCAGTTCCTCAGCCCTATGTCTGTTCACACGC
AATCCACAACAAACTGGAGGACACCTTAGATGAGAGTCTGGTCTCCCCACAGGGTGGCCAGGAGAGACATTTGAGGCACA
AGGAGAAGGAGATCACCTGAGGTCTCTAGGGGCCTCTCTGATACTCAGAATCCTAACGCCCTTTAGGTCCACCCTCAATC
CGGTCTGCCAGCATCCATCCTTAATACGAACCTCTCTTGCCTGCAGACAGCTCTTGCCTTGATAATGAGTCTTCTTTGAG
AACGAGGTACTCACTTGCGGGTTTTTTGAGTCAAGGACAGAGTAGGTAAGGAAGAAACAAGCCTATGAGATGGTGGGAAT
AAAGGACAGAGTCGGAGAGGAGATTCTGAATTCGGAGGAAGGAGCAGGGATGGGGGAGCTGTATATTGGAGACGAGACAT
GGAGAGTATATCCAGTGGGCAGATGAATCCGTGTGTGTGAGTGAGTGAGTGTGTGTGTGTGTGTGTGTGTGTGTGTGTGT
GTGTGTGTGTGTGTCCTCCCTCAAGTCTCCTAGTCTCACAGGAAGAAATGATTCCATGGGTGTTTGTTCTTTTCAAGGGA
AAGGTTAGCCGGGGGTGTGGTGGCGCACGCCTTTAATCCCAGCACTTGGGAGGCAGAGGCAGGCGGATTTCTGAGTTCGAG
GCCAGCCTGGTCTACAGAGTGAGTTCCAGGACAGCCAGGGCTACACAGAGAAACCATGTTTCAGGGAGGGGGAAGGAGGA
AGAAGGTCAAGTTTCCCTACACTGAGTCCCTGAGGACTCTCACTCTCACCCCAACAGGAACCCAGCTTTTCCACACACCA
GGCATCCCAGCCAGGGACACAGATGAAGGCACTGTCAACAGAAGCACCAGCTAGAACTTCTGTGATCCTGAGGTCACATG
GCCCACAGGTTCCTCCCTATTCTGATTTGTATGTGACCCGATCACATTTCCTTGCAGATGTTAGGTTTACCCCTTCCCTT
GTGCACTTTGAAAATGGCTACTGCCAGCGGGGGGCGTGGTGCCGCATGCCTTTAATCCCAGCACTCAGAAGGCAGAGGCC
AGCTGATCTCTGTGATTTTGAGGCCAGCCTGGCGTACATAGTAAATTCCAAGATGCCCAGGAGAAAAAGGAAGGCAGAAG
CAAATGGCTATCGCTCTATTTTGTCTACTGCCCACTGATCCCACAAAGTTCCCTGACCAGGCTGTTGTGGCACTGTGTCA
CACCACAGTGACCCCACCGAGTTCTCATCTCCCAGTGTGACCTACGGGTAGTCTGCCCTGAAGTGGATACCCAAGTCCTC
TATCACGAGGGCAGGAGAGGGGACTTCTCAATCCATGTCACAGCTTGCCTAGGACAGTGTACGTTATGTTTGCTCTAAAC
AAATATCATTGTGAAGCTGAACAGATGTTGCAGATAAAATGCCCTAAACCCTTTCCCTACGTTTCAAAGAAGCTGGGAAC
TCAGGCGTGCTAGTCTTGGATGGTTTCTGCATCTGTGGTCCAGTAACTGGCACGCGCAATCTCTCTTGCAAAGGCAGAGG
TGAATTCTTCTGGCCCCGGGGGTGTTTTCTGCAGCCTGGCTAGCTCCAGACCTGTAATTAAGTCTAGTGGTATGCTGGCA
AGGATGTGGAGAAAGAGGAACACTCCTCCATTGCTGATGGGATTGCAAGCTTGTACAAGCACTCTGGAAGTCAGTCTGGC
GGTTCCTCAGAAAATTGGACATCGTATTACCGGAAGATCCAGCAATACCTCTGCTGGGCATATACCCAGAAGATGTTCCA
ACAGATAATAAGGACACATGCTCCACTATGTTCATAGCAGCCTTATTTATAATAGCCAGAAGCTAGAAAGAACCCAGATG
TCCCTTAACAGAGGAATGGATACAGAAAATGTGGTACATTTACACAATGGAGTACTACTCAGCTATTAAAAACAATTTAT
GAAATTCCTAGGCAAATAGATGGATCTAGAGGATATCATCCTGAGTGAGGTAACCCAATCACAAAAGAACTCACTTGATA
TGCACTCACTGGTAAGTGGATATTAGCCCAGAAGCTTAGAATACCCGAGATACAATTTGCAAAACACAAGAAAATCAAGA
AGAAGGAAGACCAACGTGTGGATACTTCATTCCTCCTTAGAATAGGGAACAAAATACCCATGGAAGGAGTTACAGAGACA
AAGTTTGGAGCTAAGACGAAAGGATGTACCATCCAGAGAGTACCCCACCCGGGGATCCATCCCATAATCAGCCACCAAAC
CCAGCACTATTGCATATTGCCAGAAAGGACCCTGATATAGCTGTCTCTTGTGAGGCTAGGTCAGGGCCTGGCAAACACA
GAAGTGGATGCTCACAGTCATCTATTGAATGGAACACAGGGCCCCCAATGGAGGAGCTAGAGAAAGTATCCAAGGAGCTG
AAGGGGTCTGCAACCCTATAGGTGGAACAACAATATGAACTAACCAGTACCCCCCAGAGCTCGTGTCTCTAGCTGCATAT
GTAGCAGAAGATGGCCTAGTCGGCCATCATTGGGAAGAGAGGCCCCTTGGTCTTTGCAAACCTTGGTACAGGGGAATGCC
AGGGCCAGGAAGCGGGAGTGGGTGGGTTGGGGAGCAGGGCGGGAGGAGGGTATAGGGAACTTTCGGGATAGCATTTGAAA
TGTAAATAAAGAAAATATCTAATAAAAAAGTCTAGTGGTGGAGAATCACCCAAGTGGGAACCATCTTTGCTCCTGCACTA
CAGCTCTCACAGGACTGGTAGACATGCCATCTTGTTCGAGCTACCACCTCTGTCTACCACCTACCCTGCCCGTCATGCTC
TGGCTCTGCTGGGTTGGCCGTTGACAATCTCTGGACTAGAAAGCCTGGTCAGGGAATGAACGCGATGAGAACTCGTGCCT
GCTCTAACCACAGTCTCTCTCTCCATCACCCCAGCAGAGCCAACCCTAAGCAGACTACGAACTAGAAGAGCTTCCGAGCT
TTAGGTTAAACACAGCAGAGCAGGGGAAACACAGGAATCACGAGGGATCGGGAGGGAGGGGAGGTTTTGAGGAAGGGAGG
AAGGAAGGATGTGCTGACATTTGCTTAGTGTCCATTCTAGGGATCCACCTGGCACTGAATCCACAAGTGAGGAAGCCAAG
GGTGAGAGCAGGCTGGTGTAACTGACTCATGGTGACACAGCCAGCAGCTGTGCTAAGATTATATTCCCTACTTCATTTAT
ACTGTGTATTGAAACCTTACCTTATTGAAGTCTGTCCTGTCTGTCTGTCTGTCTGTCTCTCTCTCTCTGTTTTT
TTTTTTTTTTTTTTTTCGAGACAGGGTTTCTCTGTGTAGCCCTGGCTGACCTGGCACTCACTTTGTAGACCAGGCTGG
CCTCGAACTCAGAAATCTGTCTGCCTCTGCCTCCCTCCCAAGTGCTGGGATTAAAGGCGTGCACCACCACACCTAGCCTT
ATTGAAGTGTTTTAGCAATCCTATTGAAGGCCCATTTTACAGATGGGGAAGTAAAGCCCAGAGAGGGGAAAGTGCCAAAG
CCATAAGGCAATAAAACTACTAAAGGATGTCAGCCAGTTCTAGCTGGCCTCCAAAGGCCAATTTGCATCTGCCCTCTTGC
TGCACCATTTAACCACATAGTTGCCTACTCTTATCAAATCCAATCAAATGCAGCTAGGAGCTAGGCACTGTGCTGGGTGT
ACATAGCATCTCTCAAAGCACGCTGGATGCAGAGTTCTGTCCTGGGAGGAGGCACGGTCGAATGAGTACCGCCTTCAGGG
AGCAGGGAAACAGCCCGGCTGTGATAGGCTGTCCACTCCTTCTTCCCACTGGGCCCTGCTTCTCTCTCAGGCCTTCTTGG
GCCTCCTGGCTCTGCCATGGAGCCACTGGGCAACATGAACAAGTCACCTGTCTGCTGCAGCTCCTCTAGAGCAAAAGAAA
GTTCCTAGATGGAGTGAGTTTTACACCCATCCCCTGCGGTTCTGAGGTGCCTTGGGCATATTGACTGGGTGGGGTCAGGG
AGCCCACATCTCAGGGCATGTTGACGCCTGGAAGGGGTGCAAAGTGCCTGGGTGAGGGGCTGAGGAAGCTACTCCACCCC
CAGCTGTGGTTTCTGGTGGGGCCACTGGTGGCAGCAGGTGAGGGGAAGTATCTGTCACTCTTCCAACAAAGGCAATCAAA
CAAAAGCCTCCTGGGGCATTGTGGGAACTGCAGTAAAGGCGCCAAAAATAAAACACAACTTTCCTTTGATAGGCACTGGT
AGCTGCCTGGGGGTTGTGACAAAAGTGTTCAGATTTCACACCTCAGTTCATACCCCAAACACACACACACACACACAC
ACACACACACACACCAGTCTCACAAACAAGTCATATAATAGGCAGTCAATGATCCCAGAAGAAACATCTCCGTGGGCT
GTGACACATGTCACAAGAACCACATATGTGTCAAATGGCTTGTCCTTTCCATTTTGAAATGTTCAATATTTTAAAAGAAC
TTTTAAATATCCTATTTTATTTCATGTATATTGGTTTTTGTCTGCATCTATGTCTGTGTACCCTACCATTCACAAAGGCC
AGATTAGATTCCCTGGAACTGGATTATAGACAGTTGTAAACTGCCAGGTGTGTGCCTGGAATCAAACTTGGGTCCTCTG
```

AAAGAGCAATCAGTGCTTTTAAAATATATATACATATGTGTGTGTGTGTGTGTGTGTGTGTGTGTGTGTGTGTGTGTGTG
TGTGTAAAATTTATTTTATGTATATGAGTACACTGTCGCTATCTTCAGGCATACCAGAAGAGGGCTTTGGATCCTATAAC
AGAGGGTTGTGAGCCACAGTATAATTGCTAGGAATTGAACTCAAGGCCTCTGGAAGAACAGCCAGTGCTCTTGACCACTG
AGCCATCTCTCCACCCCTAAAGTTTTCAGTATGTTTTAAGCAAGGAGCTGAACGGGGTATTTTGTTCTTTGTCCTTCAAG
CTACAGAACCAAACTTGCTAGCATTCATTCATAAATATTCATATGGGCAGTATGCCTAAAAATTATAAAATACTGATAGG
CAAAAGTGCATACATTGCCCACACAGCGAGAGGAACAGACACATAGACCCCCCCCCCCCACACACACACACACTGGTA
CTACAGGGGCTCCCCTGTGTACACACTGCAGTACAACTGCTTCCCCCATAAGGGACATTGACACCCACTCGAAAGGCC
CAGAGAGAGCTCAACCACAACCCAGTGACATGAATGGATTGACTTAGCCAGATCAAGGCACCTCGATGGCACTGAAAGGA
AGGGCTTAATCTCCTTTACTGTATCTTCAACTGTCACAGCTGGCCTCTCTTTCGGGCTATGGGCTCATCCACCAACCCTT
GCAGCGGAGACTAAGGAAAAGGAGACCTTGGGGAGTAGAGGAATCCTTGGGAGGAGAGGTTGGGAGGAGGAGAAGAACAT
TAGCCAAAAACAGCAGGCTGGGCGACAACGGAGGCGCCTAGTGAACTGGCCTGAGTGGTGGTTCTGAAAGAGCCTGGGGG
AGGCGGGGGCAGGTCTTGAACCCAGACAAGTCCGAGCTCCTGCTCTCACATGAACAGCCAATCCCTTGCCCCACTCCTCT
TTCTAGCTAGGTGGCCTTGGGCACATGGCTGAACTTGCTTGGCCTCAGTCTCCATCTCCACGGAATGAGAAAGTATTGTC
TGCATGGCAGGGTTGTGTGGTGCATGTGGAAGTTGCGGCCTCATCTTCTGGGCTCTCCCAACATTCAACAGTTTGCCTTC
TTTCCCGGCCTTGCAATCCGACTCTTGCTTCAGAAACTCCAGACCCCCTCATTAACGGCAGACCTTCAGAATGTGTTTAT
TTCTTGTCTCCCCACCCCCACCCCACTTCCCAGCCAGATGCAAGCATGGGAGGTGGCCGTAGTTCAAGGCCCACCCTCCC
TTCGGGGACTTTCAACGTTCCTCAGTTCCTTTTGATCCCATAGCTCCAGCCACAAGCCAAACAGCCCCCAAGGCCTCCAA
AGTTTTCTGCCTGTGCTCTCTACCCCTAACCCCAGTGCTGCCTGTAAGAAACGTTCCAGGCTCTGCCCAGAAGCCCCTC
CATCCTGTTAGGTGATACCAGATGCTCAAAGAGTGACCCAGAAAAACAATTCTTTTCAGCTCATTGTGATGGTGCACATC
TGTAACCCCACCACGCTGAGGAGGCTAAGGGAGAAAGAGAGAATTCTCGGCCAGCCTGGGCTACAGATCAAGACCCTGTC
TCAGAAAAATTCCAACAAAATTGCTGGGAAATATTGCTCAACCATCGGAATGCTTACATAGCATGCATGAGTGCAGCATA
AACTTAGCGTGGTGGTTCAAGGCCTGCAATCCCAGCACTTGGGAAGTGAAGGCCGGGGATCAGAAGTTCAAGGTCACTCT
AGGCTACAGGGAGAGTTCAAGACCGGACTGGGCTATATGAGATTCTCGTCCTAATCAACTCAAGCAAACAAGCAAACAGA
CACACACAAACTCACAGTCACCCACACG

MOUSE mRNA SEQUENCE : mR1-004.1 (Seq ID No: 2)
GCCAGAGAGGGCTAGCTGGAGAGAGACAAGAACCAAAAGCACAGCCTTCCTGTGTGATTTCTACAGCCCCCAGAGCACCA
TGGACCCAGGGAAACCCAGGAAAAACGTGCTGGTGGTGGCTCTCCTTGTCATTTTCCAGGTGTGCTTCTGCCAAGATGAG
GTCACCGATGACTACATCGGCGAGAATACCACGGTGGACTACACCCTGTACGAGTCGGTGTGCTTCAAGAAGGATGTGCG
GAACTTTAAGGCCTGGTTCCTGCCTCTCATGTATTCTGTCATCTGCTTCGTGGGCCTGCTCGGCAACGGGCTGGTGATAC
TGACGTACATCTATTTCAAGAGGCTCAAGACCATGACGGATACCTACCTGCTCAACCTGGCCGTGGCAGACATCCTTTTC
CTCCTGATTCTTCCCTTCTGGGCCTACAGCGAAGCCAAGTCCTGGATCTTTGGCGTCTACCTGTGTAAGGGCATCTTTGG
CATCTATAAGTTAAGCTTCTTCAGCGGGATGCTGCTGCTCCTATGCATCAGCATTGACCGCTACGTAGCCATCGTCCAGG
CCGTGTCGGCTCATCGCCACCGCGCCCGCGTGCTTCTCATCAGCAAGCTGTCCTGTGTGGGCATCTGGATGCTGGCCCTC
TTCCTCTCCATCCCGGAGCTGCTCTACAGCGGCCTCCAGAAGAACAGCGGCGAGGACACGCTGAGATGCTCACTGGTCAG
TGCCCAAGTGGAGGCCTTGATCACCATCCAAGTGGCCCAGATGGTTTTTGGGTTCCTAGTGCCTATGCTGGCTATGAGTT
TCTGCTACCTCATTATCATCCGTACCTTGCTCCAGGCACGCAACTTTGAGCGGAACAAGGCCATCAAGGTGATCATTGCC
GTGGTGGTAGTCTTCATAGTCTTCCAGCTGCCCTACAATGGGGTGGTCCTGGCTCAGACGGTGGCCAACTTCAACATCAC
CAATAGCAGCTGCGAAACCAGCAAGCAGCTCAACATTGCCTATGACGTCACCTACAGCCTGGCCTCCGTCCGCTGCTGCG
TCAACCCTTTCTTGTATGCCTTCATCGGCGTCAAGTTCCGCAGCGACCTCTTCAAGCTCTTCAAGGACTTGGGCTGCCTC
AGCCAGGAACGGCTCCGGCACTGGTCTTCCTGCCGGCATGTACGGAACGCGTCGGTGAGCATGGAGGCGGAGACCACCAC
AACCTTCTCCCCGTAGGGGGCTCCCCTGCCCGGACTACAAGGACCTCTCCCAGGAGCCTTAATGTGGTGCACACATGCAC
AGACTCTCCATCCACCGAATTGCTGCTGAGGGAAGAGCAATTCTGGCCAGTCAGGTTGACATGAGGACCTAAGAAACTGC
TTAACCCCATCCCACTTATAACTACCTCAACCAAAGCTGTAAAAGATATGGCTGAGAAGTTAACACTCAAGCCAAGACAG
CTATCCCCAAAACGACAGCCAAAAGTGAAAGTGAGAGGCTGCCACACTTTCCGGAGTGAGGGATGTGGGGCAGTGAACA
CCCTGGTTGAGTAGTCTTCGGAGGCCTCTGAATGAACCTGCTTCTAGCTTAGAGAGATGTCCCGGAGATTCAAGGACAGA
GCTTATCTCCACACTTAGCAAGCAAGCAAGAGATGACAGTCTCTCTAAATGCTCCCACAGAGCACCCCTGCCCCTCCCTT
CTGCCTCTCCACCGCCTTTCCTGAGGTCCAGGCCACACCATCAGCCTGAAGGCAGTCCCAGCTGGGGCTCTGGATGGCAA
TGACAAGTAGTTGGGTCTCTATGATGGGAATAAAAAGGTAGGGGAAAGGTGACAGAAGGAGAGAAGGTGACCCTGCTGG
CTGACAGAGGCCAGCAAGCTACTTCTTTGTTCTCTGTCAGCCACTGATACCTTTCCTCATGTTCTGCTTTTGATTCATAT
ATCTTTTATGAAGAAACAAATAAAAAAAAAATTTTCCCTCGAGGAAACAACTTGG

MOUSE PROTEIN SEQUENCE : mP1-004.1 (Seq ID No: 3)
PERASWRETRTKSTAFLCDFYSPQSTMDPGKPRKNVLVVALLVIFQVCFCQDEVTDDYIGENTTVDYTLYESVCFKKDVR
NFKAWFLPLMYSVICFVGLLGNGLVILTYIYFKRLKTMTDTYLLNLAVADILFLLILPFWAYSEAKSWIFGVYLCKGIFG
IYKLSFFSGMLLLLCISIDRYVAIVQAVSAHRHRARVLLISKLSCVGIWMLALFLSIPELLYSGLQKNSGEDTLRCSLVS
AQVEALITIQVAQMVFGFLVPMLAMSFCYLIIIRTLLQARNFERNKAIKVIIAVVVFIVFQLPYNGVVLAQTVANFNIT
NSSCETSKQLNIAYDVTYSLASVRCCVNPFLYAFIGVKFRSDLFKLFKDLGCLSQERLRHWSSCRHVRNASVSMEAETTT
TFSP*

MOUSE PANTHER CLASSIFICATIONS

```
FAMILY (SUBFAMILY)
        C-C CHEMOKINE RECEPTOR-RELATED(C-C CHEMOKINE RECEPTOR TYPE 7)
    BIOLOGICAL PROCESS
        Signal    transduction(2.11.00.00.00)    >    Intracellular    signaling
cascade(2.11.02.00.00) > Calcium mediated signaling(2.11.02.02.00)
        Signal transduction(2.11.00.00.00) > Cell surface receptor mediated
signal transduction(2.11.01.00.00) > Cytokine and chemokine mediated signaling
pathway(2.11.01.02.00) > G-protein mediated signaling(2.11.01.07.00)
        Immunity and  defense(2.16.00.00.00)  >  Cytokine/chemokine  mediated
immunity(2.16.10.00.00)
        Cell       structure      and      motility(2.27.00.00.00)      >      Cell
motility(2.27.02.00.00)
    MOLECULAR FUNCTIONS
        Receptor(1.01.00.00.00) > G-protein coupled receptor(1.01.01.00.00)


MOUSE GENE ONTOLOGY
    BIOLOGICAL PROCESS
        cell motility > chemotaxis
        cell surface receptor linked signal transduction > G protein linked
receptor protein signaling pathway
        defence response > inflammatory response
        cell growth and maintenance > invasive growth
        defence response > cellular defense response
    MOLECULAR FUNCTION
        enzyme inhibitor > protein kinase inhibitor
        defense/immunity protein > complement component
        plasma protein > complement component
        enzyme > 2-acetyl-1-alkylglycerophosphocholine esterase
        defense/immunity protein > antiviral response protein
        defense/immunity protein > blood coagulation factor
    CELL COMPONENT
        cell > membrane fraction
        cell > cytoplasm
        plasma membrane > integral plasma membrane protein
        cytoplasm > endosome
        cell > plasma membrane
MOUSE PROTEIN DOMAINS (INTERPRO SIGNATURES)
        IPR001277 (CXCCHMKINER4)
        IPR001718 (CHEMOKINER7)
        IPR000276 (GPCRRHODOPSN)
        IPR000355 (CCCHEMOKINER)
        IPR000276 (7tm 1)
        IPR000276 (G PROTEIN RECEP F1 2)
        IPR000276 (G PROTEIN RECEP F1 1)
        IPR001064 (CRYSTALLIN BETAGAMMA)


HUMAN GENOMIC SEQUENCE : hD1-004 (Seq ID No: 4)
AATATATGAAACAAATATTGACAGAATTGTCAAAGAAATAAGAGAAACACATAAAGCAAGAAATAAGAGAAACACATAAT
TCAACAATAATAGAAACTTAAATATCCTCCTTTCAATAATAGATACAACAACTAAGCAGTTGATCAACAAGAAACAGAA
GATTTGAACAATGCTATGAACCAACTAGACCTAACGTCTATCTATAAAACACCACCCAACAACAGCAGAATACATATTCT
TCTCAGATATACATAGAACATTCTCCAGGATAGGCCATCTGTTAGGACATAAAACAAGTCTCAAAAAATGTAAAGAATT
GAGATCAGACAAAGTCTGTTCTCTGACCACAACCAGTAACAGAAGGAAATTTGAAGAATCCATAAGTATGTGGAAATGAA
TCAAGGAACTCAAGGGAAATTAGAAAATACTTTGAAATGAATGAAAATGAAAACACAGCATACCAAAACTTATGAGATGC
AGCTAAAATAGTGCTTACAGAGAAATTAATAGCTATTAATGCCTGTATTTTTTAAAAGAAGAAAGATACCAAATTAAAAA
AAAACTTTTCACTTTAAGAAAAAGAATAGTGAACCAAGCCCAAATCAAGCAGAAGGAAGGAAATAATAAAGATTAGAATG
GAAAAAAATGAAATATGGAATTGGAAAAACTAGAGAAAAATTAACAAACCCAAAAGTTGTTATATCAAAAAGATTGATAA
GTTTGATAAACATTTAACTAGACTTACCCTAATATCAAAACCACATACAGATATCACAAGAAAGTACAGACCAATATCT
CTCATAAGACACATATAAGATAGATGCACAATTTCTTAACAAAATATTAACATGCCAAATCCAGAAACATTACAAAAAAA
ATTGTATACCATGACCAAGTGATTTATTCCAGGAATTCAAGGTTGGTTTAATATAAAAAAATCAATTCATATAATACACC
ATATTTAATAGAATAAAGGGCAAAAACACTCAATTGTCTCAGTGGACACAGGAAAGGCCTCCAACTCCCTCCATGTCCCT
CCAAAGGACATTATCTCATTCCTTTTTATGGCTGCATAATATTCCATGATGTATATGTAGCACATTTTCTTTAACCAATC
```

```
TATCATTGATGGGCATTTAGGTTGATACCATGTCTTTACTATTGTGAATGGTGCTGCAATAAACATACACGTGCATGTGT
CTTTATAATAGAACAATTTATATTCCTTTGGGTATATACCCAGGAATGGGATTGCTGGGTCAAATGGTATTTCTGTCTCT
AGGTCTTTGAGGAATTCTATACTATCTTCCACAATGGTTGAACTAATTTACACTCCCACCAACAGTGTAAAAGCATTCCT
TTTTCTCCACATCACCAGCATCTGTTATTTTTTGAATTTTTGATAATAGCCATTCTGACTGGTATAAGAGGGTCTCTCAC
TATGGTTTTGATTTGCATTTCTCTAATGATCAGTGTGTTGAGCTTTTCAAAATATGATTCTGGGTCACATGTATGTCTTC
TTTTGTGAAGTGTCTTTTCATGTCCTTGGCCCACTTTTTAATTTTTTTTTCTTGTAAATTTGTTTAAGTTTTTATAGATG
CTGGATGTTAGACTTTCGTCAGATGCATGGTTTGCAAAACTTTTTTTCCCACTGCGTAGGTTGTCTGTTCACTCTGTTGA
TAGTTTCTTTTGCTGTACAGAAGCTCTTTAGTTTAAGTAGATCCCTTAATTTACTTATTTGTCAATTTTTGTTTTTGTTA
CAACTGCTTTTGGCATCTTTGTCATGAAATCTTGGCCCGTGCCTTGGTCCTGAATGGTATTGCCTAGGTTTTCTTCTAGG
GTTTTTATAGTTTTTAGTTTTACATTTAAGTCTTTAATCCATCTTGAGTTGATTTTTGTATATGCTGTAAGGAAGGGGTC
CAGTTCCAATTTTCTACATATGGCTAGCCAGTTATCCCAGCACCATTTATTAAATAAGGAATCCTTTCGCTATTGCTTGT
TTTTGTCACGTTTGTTGAAGATCACGTAGTTGTAGGAGTGCAGTCTTATTCTGGGTTCTCTATTCTGTTCCATAAGTCT
GTCTGTTTTTGTATCAGTACCATGCTGTTTTGGTTACTGTAGCCATGCAGTATAGGTTGAAGTCAGGTAGCATGAGGCCT
CCAGCTTTGTTCTTTTTGCTTAGGGTTGGCTTGGCTATTTGGGCTCTTTTTGGTTCCATGTGAATTTTTAAATAGTTTT
TTCTAGTTCTGTGAAGAATGTCAATGGTAGTTTAATGGGAATAGCATTGAATCTATAAATTGCTTTGGGCAGTATTGTCA
TTTCCACAATACTGATTCTTCCTATCCATGAACACGGAATGTTTTTCCATTTGTTTCCATTATTTCTAATTTCTTTGAGC
AGTGGTTTGTAGTTCTCCTTGTAGATGTCCTTCACTTCCCTTGTTAGCTGTATTCCTAGGTATTTTATTCTTTTTGTGGC
AATTGTGAATTGGAGTTCATGCGTGCTTTGGCTCTCTGCTTGCCTGTTTTTGGTGTATAGGAATGCTAGCAATTTTTGCA
CACTGATTTTGTATCCTGAGAATTTGCTGAACTTGCTTATCAGCTGAAGAAACTTTTCGGCTGAGACAGTGGGGTTTTCT
AGATATAGGATCATGTCATCTGCAAACAGGAATAGTTTCACTTCCTCTCTTCCTATTTGAGTACACTTTATTCCTTTCTC
TTCCCTGAATATGACTGCCCTGGCCAGAACTTCCAATACTGTGTTGAATAGGAGTGATGAGAGAGCAACCTTGTCTTG
TCCTGATTTTCAAGGGGCTTACTTCCAGCTTTTGCCCATTCAGTATAATATTGTCTGTGAGTCTGTCATATATGGCTCTT
ATTATTTTGAGGTATATTCCTTCAACACCTACTTTATTAAGAGTTTTTAACATGAAGGGGTATTGAATTTTATCAAAGGC
CTTTTCTGCATCTATGGAGATAATCATGTGGTTTTTGTCTTTAGTTCTGTTTATGTGATGAGTCACATTTATTGATTTGC
ATATGTTGAACCAACCTTGCATCCCAGGGGATGATGAAGCCAAATTTGATCATGGTGGATAAACTTTTTGATGTGCTGCT
GGATTCGGTTTGCCAGTATTTTGTTGAGGATTTTTGCTCAATGTTCATCAAGGATAATGGCCTGAAGTTTTCTTTTTTTG
TTATATTTCTGCCAGGTTTTGGTATCAGGATGATGCTGGCCTCACAGAATGAGTTAGGAAGGAGTCTCTCCTTTTCAATT
TTTTTGAGTAGTTTCCGTAGGAATGGTACTCTTTTCTGTACATCTGGTGGAATTCAGCTGTGAATCCATCGGTCCTGGA
CTTTTTTTGGTTGGTAGGCTGTTTATTACTGCCTCAGTTTCAGAACTCGTTTTCAGTCTGTTCTGGGATTCAATTTCTTC
TTGGTTTGGTCTTGGGAGGGTGTATGTGTCCAGAAATGTATCCATTCTTCTAGATTTTCTATGCACATAGAGGTGTTTA
TGATATTCTCTGGTGGTTGTTTGTATTTCTGTGAGGTCAGTGGTAATATCTCCCTTATCATTTCTGATTGTATTTATTTG
AGTTGTCTCTTCTTCTTCTTTATTAGTCTAACTAACAGTCTATCTATTTTATTAACTTTTTTGAAAAACCAGCCTGGATT
CATTGATATTTTGAATGGTTTTTTTTTTATGTCTCTATCTCCTTTGATTCAGCTCTGATTTTGGTCATTTCTTGTCTTCT
GCTAGCTTTGGGACTTCTTTGCCCTTGGTTCTCTAGTTCTTTTAGTTGTGATGTTAGGTTGTTAACTTGAGATCTTTCTA
GCTTTTTGATGTGGGCATTTAGTGCTATAAATTTACTCCTTAATACTGCCTTAGCTGTGTCCCAGAGATTCTGGTATGTT
GTATCTTTGTTCTCATTAGTTTCAAAGAACTGCTTGATTTCTCCCTTAATTTCATGATTTACCCAAAAGTCCTTCAGGAG
CAGGTTGTTCAATTTCCATGTAGTTGTGTTGTTTTGAGTGAATTCTTAGTCTTGAGTTCTAATTGATTGTGTTGTTGC
CCAAGAGACTGTTTGTTATGATTTCAGTTCCTTTGTATTTGCTGAGGAGTATTTTACTTCCGATTATGTGATCAATTTTA
GAGTAAGTGCCGTGTGGTGATTAGAAGAATGTATATTCTGTTGTTTGGGAATGGAGAGTTCTATAGATCTATCAGGTC
CATATATTCCAGAGCTGAGTTCAGGTCCTGAATATCTTTGTTAATTTTCTCTCTCAATGATCTGTCTAATGGGGTGTTAA
AACCTCCCACTATTATTGTGTGGGAGTCTAAGTCTATTTGAAGGTCTTTTTGAGCCTATGTCTGTCTTTGCATGTGAGAT
GGATCTCTTGCAGATAGCATACCAATGGGTCTTGGTTTTTTATCCAGCTTGCCACTCTGTGTCTTTTAATTGGGGCATTT
AGCCCATTTACACTTAAGATTAGTATTGTTATGTGTGGATTCAATCCTGTCATCATGATGCTAGCTGGTTATTTTGCAGA
CTTGTTTATGCGGTTGCTTCATAGTGTCACTGGTCTGTGTACTTCAGTGTATTCTTGTAGTGGCTGATAACAGTTTTTCT
TTTCCATATTTAGTGCTTCCTTCAGGAGTTCTTGTAAGGCAGGTCTGGTGGTAATGAATTCCCTCAACATTTGCTTGTCC
TGAAAGGATCTTATTTCTCCTTCGCTTATGAAGCTTAGTTTGGCTGGATATGAAATTCTGTATTGGAATTTCTTTCCTTT
AAGAATGTTGAATATTGGCCCCAATCTCCTCTGGCTTGTAGGGTTTCCACTGAGAGGTCTGCTGTTAGTCTGATGGGCT
TCCCTTTGTAGGTGACCTAGCCTTTCTCTCTGGCTGCCCTTAACATTTTTTCTTTCATTTCTACCTTGGAGAATCTGATG
ATCATGTGTCCTGGGAATGATCTTCTCATGGAGTATCTCACTGGGGTTCTCTGCATTCCTGAATTTGAATGTTTGCTTG
TCTAGCTAGGTTGGGGAAGTTCTCCTGGAAAATATCCTGAAATATGTTTTCCAAATTGTTTCCACTCTCCCCATCTCTTT
CAGATACCCCAATCAGTCATAGATTCCGTCTCTTTACATAATCCCATATTTCTTGGAGGTTTTATTCATTCCTTATCATT
CTTTTTCTCTATTCTTATCTGCCCGTATTATTTCATAAAGACAGTCTTCAAGCTCTGAGATTCTTTCAGAGGAAGAATCT
TTCAGAGGAAGACTAAGCAGAACTTGGCCTATTCTTCTATGAATACTTCTGATTGCATTGTGAAGTTCTTGTAGTGTGTT
TTTCAGCTCGATCAGGTCAGTTATGTTCTTCTCTATAATATCTATTTTGGCTGTCAGCTCCTGCATTGTTTTATCATGAT
TTTTAGCTTCCTTGCATTGGGTTACAACTTGCTTCTTTAGCTCAGCGACGTTCATTTTTATCCACATTCTGAAGTCTACT
TCTTTCATTGCAGTGATCTTAGCCTTACCCCAGTTCCAAACCTTGCTGGAGAGATGCTGCAGTCATTTGGAAGGAAGAA
GGCACTTTGGCTTTTTGAGTTTTTAGTGTTTTTATGCTGATTCTTTCTCATCTTTGTGGGCTTATCTACCTTTGATTTTT
GAGGTTGCTGACCTTTGGATGGGGTTTTGGGGTTTCTTTTGTTTTTCTTTTAACAATCTGGCCACTTTTCCATAAAGCTG
CTGTGGCTTGCTGAGGGTCCCCTCCAGACTCTAGTCACTTTGGATTTTCCAGTACCTGGAGGTATCACCAGTGAAGGCTG
TGAAACAGCAAAAATGGCAACCTGCCCCTTCCCTGGGAGCTCCATCCCAGGGAGGTATGAACCTATTGCTGGCCTAAAC
ACACCTGTAGGAGATGGCCGGAGACCCCATTTGGGAGGTCTCACCCAGTCAGGAGGAACAGGATTGGGGAGCCACTGAAA
```

```
GAAGCAATCTAACCACACTTCCGAAGAGAAACTATGCTATGCTGGGCACCACTTGCATCCCCAGTTGGCTTGGGATCTCC
AAAGCCTGGAGACTGGTGTGGCTAAGTCGCCAAAACAGCAAAGATGGCAGCCTGCCCCTCCCCCGAGGAGCTCTGTCCCA
GGAAGTCTTCAAATCTCTGTCAGCCAGAGAACATCGACAGGAGTGGCTGGAGGCCACAGTTGGGAGGTCCTGCCCAGTGA
GAAGGAATGGATTAGGGACCCTCTTAAAGAAGCAGTCTGGCCATATTTTGGTAAAGCAGCTGTGCCACGTTGGGAGATCC
TTTCAGCCCCCAGTTGGTTTGAGCTCCCCAAAGCTGAAAGGCTGGAATGGCTGGGATGCCCAAACAGCAAAGGTGGCAGC
CCTCTCCACCATCCAGGCACCCTGTCCCAGGGAGAGATCAAAATTTTATGGGCTGTAGAAAATGGGCAGAGGTGGCTGAA
GGCCTTGTTTGGGAAGACCCACCCAGTGAGGAGGAATGGATAGGAGTCCTGCTTAAAAAAACAGTCTGGCCACGTTTTGG
CAGAGCAGCTGTGCTGTGCTGGGGGATCCTTTCCACACCTGGTGGGTTTGGACTCTCCAAAGTCTACAGGCTGGAACAGC
TGAGTCGTCCAAACAGCAAAGGTGGTGCCCCTCACCCCCACCCCAGCCACCCCCAGAACTCCACCTTGTCTCAGGTAGGT
GCTACACTGTTGCCCGTGGCTGGCTGGAATTCCAAGCCAGTTGGTCTTATCTGGTGAGGTGCTGTGGAAGTGAGGCCCAC
AGACCAATGCTGCTCAGCTCCCTGGATTCAGCCTCCTTCCTAGGGGTATGTACAGACCTCCTGCCTTGCCTGAATGGCGG
TCACCTTTGCTGGGGTTCCGGAGCCTGAGTATGTAAAGCTCCTGGGTGTCTGTGCATGCCTGAGCAGCTGCTCTGCCAAT
AATCCAATGCAGCTCTGTGTGTAAGACCCAAAGGCCTGGTGGAGTGGGTTCACAGGGGATCTCCCGATCCAAAGGTTGCA
AAGATCGGTGGGAGAAGCATCATTCCCCAGGTTTGCACATTCACTCACCACTTCCCTGGTGGGGGGAGGGGAGGGGGTTT
CCTTTGGGTCTGTGTTGCTCCTATGTAGGCCATTGCCCTGCCCTGCTTTCCTCTATCCTCTGTGGGTCGAGCTGATTCCT
TAATCAGTCCCAATGCAAGTATCTGGATGTTTCAGATAAAGGTTTTGAAGTACCCACCCCTTTGTTCCTCTCTGAGAGAG
CCATGCCCCATACCTGCTTCTAGTTGGCCATCTTTGAAAATAAAATTAAGAAAACAATTTCATTTACAAGCACATCAAAA
AGAGTAAAATACTTAAGAAAAAATTTAACAAAGAAGTGAAAGACTTGCAGCCTAAAAAGTATAAAGAAATTAAGGAAGAC
CTAAATAAATGAAAAAAATCCCATGTTCATGGACCAAAAGACTTTGTTAGAATGGCAATACTCCCTAAATTGATTTACAG
ATTCAATGAAATTCCTATTAGAATCCCAGCTGGTTTCATTGCAGAAATTGATAACCTAAAATTCATATGGAAAAGAGAGA
GATGCAGATTAGTCAAACAATGTTTAAAAAGACAGATAAAGTTGGAGCAGTCACAACTCCCAATTTCAAAATTTGCTATA
AAGCTACAGTAATCGAGACAGTGTGGTACTGTCATAGGACAGACACAGAGATTGATAAAATAGAATTGATAGTCTAGAAA
TAAACCATTACATTTATGATGAATTGATTTCCAACAGAGGTGCTAAGACAGTTCAATGGTGGAAAAAATAGTCTTTTCAA
CAAATGATGCTGGGAAAACTAGATATCCATATAAAAAATGAAGCTGGATCCCTACCTCACACCATATGCAACAATTAGCT
CAAAATGGATCAGATCTAAATGTTGCAGCTAAAACTATAAAAGTCTTAAAAGAACATATACGAATAAATCTTTGTATACT
TGAATTATGCAATGGTTTCTTAGATATGGCACCAAAGACACAATTGACAAAAGAAAAATAAATCAATTGAACCTCCACAT
TTAAAACTTTGCGCTTCTTTTGCCAGAGATTCAGGTTTTAAAAAAACAAAAAGCCTTTGTGCTTCAAAGGACGCTATCAAG
AAAGTGAAAAGATGATCCATAGTAAGGGAGAAAATATTTACAGATATGTATCAGATAAGGGATTGTAACCAGAATATATA
AAGAAGTCTTACAACTCAATAATAAAAAATAAATAACCCAATTTTTTAAATGGGCAAAGGATTTGAATAGAAATTTCTCA
AAAAATAATACAAATGGTCAATAAGAGCATGAAAAGATGCTCAGCATAGGTAGTCATTAGGAAAACGCATATCAAAACTA
CAAAGAGGCCGGGCATGGTGGCTCATGCCTGCAATCCCAGCACTTTGGGAGGCCGAGGCTGGCAGATCACCTTAGGTTAG
GAGTTTGAGACCAGCTTGGCCAACATGGTGAAACCCCGTTTCTACTAAAAATACAAAAATTAGCCAGGCATGGTGGCACG
TGCCTGTAGTCCCAGCTACTTGGGAGGCTGAGGCAGGAGAATCACTTGAACCCAGGAGGTGGAGACTGCAGTAAGCCAAG
ATCGTGCCACTGCACTCCAGCCTGTGCAACAGAGTAAGACTCCATCTCCAAAAAAAAAAAAAGAAAAAAGAAAAAAAACAC
AATGAGATACCACTTTACAGCCACTAGGATGGCTAGAATCAAAAGAATGGACAATAGTAAGTTTTGTTGACAATGTGGAA
CCCTCACGCATTGTTGATGGGAACAAAATGGTGTAGTCACTTTGGAAAACAGTTTGATAGTTCGTCAAAATGTTAAATAT
ATAATTACCATATGACTCAACAATTTCACTTCTAGGTATATACCCAAGAGAAATAAAGCATATGTCCACACAAACACTTG
CACATAAATATTTATAGCAGCATCATTCATAATAGCTAAAAGGGAAAACAGCCCAAACATCCAACTGATGACAGACAAAC
AAAATGTGGCATATCCATACAATGGAATGTTATTCAGCCATAAAAAGGAATGGGGAATGGATCCATACCACCACATAGGT
AACCTTGAAAACACTAACTGAAAGAAGCCAGATGTGAAGGTCACATATTGATACTTCTATTTATGTGAAATGTTCAGAAT
AGGAAAATCTATAGAGCCAGAAAGTAGATTTGTGGTTGCCAGGGGCTTTCGATGACAGGAGGGGAGGGGAGCAGTCATTG
CTAGTGATTACAGGGTTCTTGAGAGGGAGTGAGAAAAAATGTTCTAAAATGTATTGTGGTGATGGTTGCATAACTCTGTG
AACATACAAAACCATTGAATTCTATACTTTAAAAAGGTAAATTTTATGGGATGTGGATTATAGCTCAATAAAGCTGTCA
TTAAGAAAGGAAAGGAAGGGAGGGGAGAAGAGGGAAAGGGGAGGGAGGGGAGGGGAGGGGAAGGGGGGAGAAAAAAGAT
ACATCGTGGCATATCTCTTCCCTTTTACCTGTCATTATGTGTCAGTGCCTCCCATTGGCCTAAACTACCCAGAAGCCAGA
GGGAAAGCCATTTGGGACTATATTCCACTGTGACACTGAGCAGAGCAAGGGAAGGATGGGGACACTTCTCTCACACATCT
CTGACTCCCCAACATCTAACCCATGGCCTGGCATGTGATATTCATGCTATAAATATTTGTTGAATCAGTAAATGGCCCCT
CATCTAGATTCTGCCAGGCAGCGCAGGGGGCTTTTTGAATGTAAATGACCTAATGATTCAGGATCCTATGACCAGCGACT
GTCACCCTCTGTCACCTTTCTTCTGCCCCTTTATCTCTGAAGGCATTGAAGGGGCCCTGCATGAGTCAGGGAGGGCTGGG
GGGAGGAGCAAGGGTGGGAGGGGCAGGGAAGAGGGTGGCTTCTCCGACAACTTAAAAGGGGCTTGAACCACTTCCTCCCC
AGACAGGGGTAGTGCGAGGCCGGGCACAGCCTTCCTGTGTGGTTTTACCGCCCAGAGAGCGTCATGGACCTGGGTGAGTG
AGCCTCTTCATGTGAGAAGGAACAGTACCAGGGTCTTGGACACCCAGACTGACCCTGTGGAATGGGGGTGGAGGATGCGG
GTGGAGCGCATAGGGGTGCTTCCTGGAGAAATCAGTTCTGATTTGGTGGGCATTGGAAGCTACTTCCAGAGAATGCCCCC
TGCTGTCCCAGGAGGGGTGCACCTGCCCTTTCAGACCTGGAGAAGGGGTCAGCCTTTGGTGCCAGTGGGGACAAGGGAGG
GGCCGAGATCATGTCTTTCTAAAACAGCAGGGACATAAGAGAGTGGCGGGGCTGGGCTTGGTGGTCTGGCAGAACCTGTT
CTCATCATGAGGGTGATAGGTTAGGCCCTCTGGGACCTCTGGGGACAGGTTTCAACAGAGAAATTCTCCCTAGAGAGCTT
AGGACCTTTTACCCACTCTCTGATTCCCACCTTTCTCCCCTGCCAAATATTCATATACCTGTCCACTTGGGGTCATGAGG
AGATGCAAATGAAACGTGACTGGGTTGTGAGAATGGTGCGGTGCATTGCAAATGCAAGCTCTGGTTGCTTTCTCTCTGCT
GCACATCCCACCTGCTTTGATCTGGCTTTGCTGTTGCCTCTTGCAAGTTAAGGAACGGGCAGGCAACATGGAATGGGATG
GCAAGGGGCACTCAGCTTGGAGAAATCATAGCCAGGCCTTCAGCACAGCCCCTGCCCCTGAGCAAACCTCACGCACTCTG
CCCCGGCAGATAACTCTACTCTTTAGAAAGCCCTTTCTCAGGTCTAACCTAGAAGGGGTCAAGGGGAAGCACTGAGGGTG
```

```
TTCTGAGTGGTTCATCCAGCTCCAGACGCCCCCTTTATAAATCTCAGCTGTGGAGTCAGACCAGGCTGAGGCTAAGAAGA
CCAGGTTCCTAGATGGCTTGTCTCACCAGCAAACAATGCACCACGGCGGCCTCAAAGCCGCAAAGGGTTGGCTTTAAAAA
GTGAAGGTTCCCTGGTGCTGTCCTCACACCCCTCCTCGCCACACCACAGCCTCCTTGATTTCATCTCTTACTTTCCCGCC
TGCCCTGAACGCTGGATCTGGACCCAGCAAACAGCTGTGAAAGGGTTAAAGTGGTCACCCCACCCACTGACTGTGAACTC
CTGAAAGGCAGGTTCAGGTGCTTTGAGGATGTGAGGAGAAAATGTCTGCGGAATGAATGAATACACCGTATCAAGAACTC
CGTAGAGTTTAGTTTTTGAAAATCTCTTCCAAATGCCTCATTTCCCAGCCAATCTACCCTTAAATCCCACTCCCCTGCAC
CCCCTCCACCACCCCCCATCACAATCCCACAGTACAGACGTCACCCACAGAAGACCCTGCTCCAGCCTTTCATACTGGCC
TCCAGGAAGGAAACCAAGCAGTGCCCAGTGGTGTGCTACTGGGCATTTGAAAAGCAGCTCCCCGGGGTGGAGCACTGGTA
GCTGACTCCAGGGTGGAGGGAAAGTCCTGAATTTGTAGTGCTTGCCAATTTCCATGGTGTAAATTCTCCCACCCTGGCTG
ATTGATTTCAAGCCATCTACATGACTGAACAGGGGGTTGGGAAGATGTGCAGAGTGTCTTACCCCAGACTAGGTTTAGGG
GTGCTAGACTCCTAGGCTTTGATGCAGGGAGAGAGGGTAATCTCTGGTCCAGGAAGAAGGGATATTCTTGCCACTTCACT
CAACTTGAGAGAGCCCCAACGGTCAAACCCGAAATACTGGTAGGAGGGGAAGCCGCTGACAAGCTTTACTCAAAACGGGC
TCTCTGGGCCGTTGATGGGGTGGGTGGGCCTTGGAGGATCTGTTGTTCTGCTCTTCATCTGCCCAGCAAGGAGCAAGAGG
TGGGTGAGGAGGGAGGGTTTCTGTTACACAAAATGAAAACTCCCAAGCAGGGCTGAGCTGGCCTTTTTCACGGTTTGAAT
GAGCAGCTCTGAGGCACCCTCCCCACCTTCGAGCAATGAGACCGAAGATCTTTCATAATTCTCAAAAGCAAAGCAAGGAA
AAAATGAACCCATGAGAGATGTGTGCCTCACAGCTCGGACATAAATGGTGGCAAAGGGGGATGCTGAAGCTAATTCAGGG
CCAGTTTCTTGCCTGCCAGAAACTTGGCTGGGGAGGAGAGGAACGCTGCAGCCTGGGGTCGCTGTTGGAAAAAATCTCTC
CTCTTTGCTTCGGACATTCAGGCAACTCTCATTGTTTGAGGGATTCAGGGATGACCAGTCTGGTGGGGAGAGTGACACAT
ATGTCACAAAGCCACTGCAACAGAATAAGATACAGCATTGACCAGGGCAAGGGCAAGACAAGAAGGGAATAGTGAACCTG
GGTCAGAGAGGTATTTAAGGTGGGTTTTCAAGGGTGTGTAGGAGTTGTCAAGCCCAACAAGGAGCTGCAATGGGCCCAGG
ATGTGGCAGGAATGGCAAACTCCATTGCCATCAGAGGCCGACAAGGTAATAGAGGGAATGGGCATAAGGTGCAGGAAGTG
GAGGGGAGTAGAGTGACCCAGAGGGTGCATGCCCCACCTAAAGGCATTCAAATCTATTATTTCAAAAACTCCATGCTGAC
AAGCAGAGTGTCCTGGGGACTAAGGCTGCAGCCGTGTAGATGGTCAGCTGAGACAACAGTAACAGAGACAAGAAAGAAGC
AAGCGGAGGGGAGGGTGGAGTAGCTGGCCATGCCAAGGAGGAAGATGAGGAGGAAGGACGCAGGCACAGGAGACCCAATC
CTTAAAACGTGGTACGGCATCCCTAAGGAATTTGAACTTTATCCTGAAAACAATGGGGACCCATCAAAGATTTTTATTTT
TCAAGTAGGTGAATTAAAGGATGAGATTTGTGTTTTGGAAGGAGTTCTGTGGCAGCAAAGAGGCAGCAAAGCCCGTGTCA
GAACAGAGGTGGGGCCGGCTCTTTTGGGGGCCGGGATGTTGGGGGATGGAAGCAGGAAGCACTGGTGGCCAGTGGGGCAT
GGGGAGCAAAGATTCCCCTCATGCTCCGTGGAGCGTCCTAGATGCAGCAGGCTCATCAGAAACACAGCCTGATGCAGTAG
CCAGAGCCCAAACCTTGCTATCCTGTTCAAATCTCAGGTCCCTCTCTTAGCAGCTGTGTGGCCCCAGGCATTTGGCTTAA
TCTCTCTGGGCCTCTATTTTCTGGAATCTAAGATGAGGATCACCAGGGTAGCTCATGAGATGTTATGAGGGAGAGACACA
TACAGACCCAGTGGCCGCACAGAAGGATGGTTAGGAGTGTGGTTGCTGGAGTTGGAGGGTTTCAAAGCCTGCTCCCCTGC
TGGCCATTGTGTGACTCTGAGAAAGTTACTTGCTGTGTTTCAGTTTCTCATTGGTAAATGGGGATAATAGTAGCGCCTAC
CTCATGGGGCTTTTGAGAGGATGAAATGAGACCATACATGCAGACAGTGTGGCACGCAGTGAGAGCTCAGTGAATATGAA
TTATCTCATTGGATACAGCAGTTGATAACATAGAAAATGCAAGGTCTACCAGGCTCTCACCCATCCCTGCCTACTGTCTG
AAAGTGGATTCCCCAGATCTCTAGACCTCTCACCACCTCCCTGCTTCTCCTCTGCCTCCCTTATCCAAAAAAAAAGAGAG
AGAGAGAAAAGTTGTGTTGGCAGGTGAGGAAGTTCAAAGAGGAAAAAGCTTGGGGTCGCTCTTGGGGCGAGCTGATTGGG
CAACTGTGATTTCCTGGGCAGTCTGGAGGACCTGGAAAAATGTGGGGATGGAAGTTCATGGTTTGGAGATGGGGGCAGCC
AGCCAATAATTTTCTTTCCTGGATGGGCAGTCTCTCATGTTGGGGGCTCCGGTTGGAGTTCTAGGGAATTTTCATGATGA
TCATTACTGAAAACTCAAGAACTCAGAAAACACCCAACATCTAGGTCAACCACTCCTTAGCCCCAGAGATTCCCAACTTC
ATTGGTCTCACTGAAGCTGCTGCCCAAGACCAGTTTCTTTATGACTTTGTAACAGACAGGGATGATCTGACTCAAGGCCA
CTGACCAGGCTCCCTTTTGCCACATACAAGAGTTCCCCTAAGTTTCCCTGTGATGTTTCTTGAAAGCCACCTGTTTTGGA
CCTACAAGACCCTTGCTCATCCACCTCCTCCAAAACACCTGGAATGAGAAATCACAGGCACACACGCCAGCACTCCCCCA
ACTCCCCTCCCCTGACGGGAACAGCAGGAGTGGGACAGCGTCCGGGAGAAGGGGTTATCAACAGAGTTCAACAGAAACAC
AGTTCTTGCTCCTCAGATCTCACCCGTTGTGACTTATCAAAACTTTGAGGTTTCTCATTTCTCCCTGACAGTCAGAGCGG
GCTTTCTCTAGAGTAACCGAAAGACACTTACGTGTTTTAAAGAAACCTCGAGACTCGACCTCTAGATGAGTCAGTGGAGG
GCGGGTGGAGCGTTGAACCGTGAAGAGTGTGGTTGGGCGTAAACGTGGACTTAAACTCAGGAGCTAAGGGGTAATTCAGT
GAAAAAGGGGAATGAGCGGTGGGGAGCTCTGTTGCAACAGGGTCCAATCGCAGCAGGACTACAAATGCCCGAGCGCAGGC
TGGGAACGAGGGGACAGCGGCTGCCTGTCCCCAGAATAGAAAATGCAGCTAGGAAGCCCTCTTTGAGTGGACAGCGGAGG
ACTGGACTGCCAGGCCAAGCATCAGGGGCTTCATCCTCAGGGCCGGTTAGAGCCCCTGAGGATTTAGGAGGAAGGTGAGG
TGGGGACTGCCATCAGAGCTAAGCTTTAGGCAGATGTGTCTAGCAGTAGTGTGCAGGACTATTTGCTCAAGGCGGGAGAA
ATACCTATTTATTTATGTAGCCCGATGCAGTCTCTCCTTCCCGGAGGAACTCCCAAGCCCAGAATCCCTGGCTTTACACA
ATTTCCGGGGAACACATTAACCAACTGCAACCTGGGTGCCATGCAGGGAGGCTTCCAGCAGGTGGTGCTGTGGCCAAGGA
CACGGAAGCGCTGGGCTCTCACTGCTTCCCCAGCCTCCAGGAGAAGGTGCCTTAAACAGGTTCCCACGCATTTCCTGGCG
CTATTGAGCTTGGAGCTGCCAAGGGCCTGCCTTCACTTGTGGCATCGCAGTTACTGACTCTCCAGTGGGCCAGGCCCTAC
CTAGCTGGGACCTGAGGGTCAGGATACGGGAAGAGGGCTACTGCCGCCCTGACTTGTAGGTAAGCTAAAACATCTAGGCA
AATTACTCTGTCAACTGTCCTCCCTGCTCAGCCTCAGGAAGAAATAAGTTCCTGCTGGGCCCCAGGCTCAGCCACAAAAC
CAGACTGAGCACATCTGGGCCTTGTGCTACACCGGGGTCCCCATCAGCTGCTCAGACCCCAGGAAGCCTCCTGACTTCCA
GAACACGTCGCTTCCCAAAGCTGACCCTGTTCCCCCTTTCACCTGCCCCCCAAGCCTAAGTTAGAAACAAGTTAAAGGA
GAGAAAGTGTGTGCAGGCACTTCAGTTAAGCAAAAAAATAAAGAAGTCGTGACATCAGGAAAGAGCGTCCTTCCCCCACA
CTGCAACAGGACAGCATCCCTTCTGTGTCCTCTCGGGCCATCTTGATGCCCTCTCCCTTGGAAAAAGGACAGGCATTCGT
TTACTTGTCATTCATGTATTTATTCATCATTCATGTATTTATCATTCATATATTCATCAGCACACAATTGTGGGGCACCT
```

```
CCTCAATTCTGGGCCTGCACAAGATAAAAGGGAAGGGATCTCTGTCCCAAAGGCAGGCCAAGGGTGTGCGCCCAGTGGAC
CAACAGTCAGTGCTGAATGAATGTCATTTCAGTGAGGGGGTGTTGTGGAGCTGGTGGAGCCCTGAGGCAGTCTCCATCGC
TGTGGACCAGTAAGGAACCTCATCCCTCTACCCAGCCTGTGAGGTCATCCTCCTTCCTCAGCCCACCTCCCTCAGGAGAC
ACTCAGGCTTGGCTTCATGCACGTGTGGCTGCGCACTCACCTGGATGCCAGGAAAATGCTCCTGGGCCAGTGGCTACCAG
CACCAAAGCAGGGCTGGAAACGTGTGCACCTACCCCACGACCTCATAGCTTTGCCGCCTCATCCCACATCAGGTGGAGTG
GGAGGGGGCACTCAGACCCACCAACCAAGCTCAGTCTTGAGGGGTGGTCTGGGCTAGAGAAGACTTCCCAGCTCCAAGAA
GCAGCCCAGATGGGCCAAGACAGGGGGTGGGAGGGGCTGCCCTGCATTCTGATGCTGCTTCTTTCCAGGACCTGGGAGTG
GAGGAGGACAAGAAGGAGGTGAGGACAGTGATGCCGGGCAGCAGGCCAGGTGTTCAAAGGCACAATCTGGTTCTGATGTT
CTCTTTCAGCAAACACAGTGCCTGGAGCTTGGGAGGAAAGTTCCCAACAGCGTCTCCCCCTCCACTGCTTTCTTTAATAA
CAAAGACTTGTCCCTGCCAAGCAATAACTTTCTCGCCTTGTCTCCTACAGGGAAACCAATGAAAAGCGTGCTGGTGGTGG
CTCTCCTTGTCATTTTCCAGGTGAGGTTCTCTGCCAAGGAAAGCTCTGTTCCCTTCTCCACACGGCCTCCCAAGTCAGGG
GTTTAGTGGAGGAGCTGGGCATTTGCTAGAAGATACAAGACCCAGACATAAGCCCGCCTGCCACTCATTAAGCTAATGGC
TGCCCCCTCACCATCTGCTGAGTGACCCTGGAGAGCCACAGATGTTCTCTGGACTTCCTTTACTCCATTTAGGTTAAAAC
ATGGGGGAAAATAACTTACCACCTTCCCTATCCCACCTCCAATCTCCCTGCCCAGGAGACAGAAATATGGACTCTGAGTT
CTTAGAGAGAGAAGAGCTGCAAAAAGCAAAAAATGTTACTGGGTTTTGTGCACGTGTGCTGAGAAAATCACCCTGGATCT
GGCGATCTTGTTACTATCCTATCGCCCCAAAGTTGTCTCTTTCCAACCTTCACCCAAAGGCTTCCCTCGCAGCCCCCGTT
TTCTTTGGTGAAATAGAGACCCATCCGGCCTCCCTGGCAGGGGAGGGAAGAGAGGATGGGGCCCTAGGGATGGGGAGAGG
GGTGGATGAGCACACACTTTATAGCAGGCCTGAACTCGGCAGGAACAAAGACCCTCACTTTACAAAGTTCTGTTTCTCCA
TCCAGAAACAGACAGAAAGTGTGCCTCACCAAGTGCCAAAGTTAGAAGAAACTGTAACTCGCAGAGTCAACTTTCTCCCA
TTTGTTAGAGGTGGGAGCTCAGAGTAGGGGTAGGGGAGTCCAACTGAGGCCAGAGCTAGAGAGGCAGTGCTTCAGAATTC
GGCTCATCCCCAGGTCTGGAGGCCAGAGGGGCAGCTTTCCAGAAGCTCAGGCCAGAGGCAGGCCCCAAGCTGGGCACTGT
TCCCAAAACCCCATCCCAGAGGCCCCCCGGAGCCTGGGCTGCAGCCCATAGCCTGGCCCCCTTCCCCATCACCACTCCCT
CCTCTCTCTCCCCTGTCTCCAGGACTCTGGGGGGCTGTATTGCTGGCAGTGGAGGCTGGAAGGGTGGGAAGGGGAGGAGG
ACAGCTGAGGCTCATTCTTCCCTTTCACTTGGGGAGGGGGTGCTCAGATAGGGCAGGATGTGGTGGGGCTGTGGGCTGCA
GAGGCAGTGAGGCTAGGGGAGGGAGAATTATAAAACAGGAACAAGGTTCAAGGGAGAAGGGAAGGCAGGACTGGATGAT
GCAGAGTGCACTTCATAGAGGCCGGGAGTACTGCCCTCCAGGAAGGCTGAGACCCCCTCATGTCCAGGGACCCCGGGTCT
AAGGCCTTGGGCCGTAACCATGGCTGGGCCAAAGGCAGCAGCCTCCTTCAGGGCTCCCAGGCTGCAGCCATGCCCAGGAA
AGGGAAGAGGGGTTGCCACTGACTTTTCGTCTCAGGCTAGCAATGGCCAAAGAAAAGGCTCCTGGACATCATTTTGTCCT
CACCATGTTCCCAAGCAGGACAGTGATGAATCCAGAACTTGGGTGTGAAAAAGAGATAGAGACAAGATGCGGTGAATCCC
TCCAGCATAAATGGATAGAAACCCACCGTCGCTGAGCTGCTGAATCTGGTTCCTGGTGATAGAACCATGAGCCTCGTGAG
GCAGGTCCTCCAGCCCAGCTCCCTGCCCTGCACAAACCCGTCCATCGCTGTGCGCAGAGGCAGGGCAGGCATGGACCCC
CTCCTTCATTGATAAAATCACAGCAGGCTGCTGAACCTTCACCCGACCCTGCAGACGGCTAAAAAAAGCTCCAGCAGCTG
AGTCCTGCTGATTGGGATCTGCCGGAAGAGATGCGAGGAAGAGAGAGGCAGAGAGGTGGGGGGAGAGGGCGGGAGCACAG
AGGGAGAGTGGGAGATGAGAGCTGCGCTTCCTTCCAGGGACACAGCTGCTGAGGAGTCCTCGCCCCCACCCCCATGGCCC
ATCATCCATTCACGGAGTTTATGGCACTTGGGCTCACGTTGCCTGAGCTCCTGCTGGGTGCTAAGAGGGGACCCAGGATC
TAGAGTCTCTCCAGGTCACCCACCACCCATGATCACAGCAGTTTGCCCCAAACCCAAGGCTTCATGGCCTCAAAATGGGA
GCATTTGCCTTCCTCTCTTCTTACTCCAGGCCCTCCTCAACCTTGACCCCCAGGTGGATTTTCCCCCTAAAGCTGAGGTC
TTTTGTAAGCCCCCTGGAAAACTCCACCAAGTTCTAAAGGAGGGTCAAGAAGAAAGACACATGTTCCCTCTCTCACGGAG
CTTCCAGGGGGCAAAATTAAAGCTCCATGGAGAGGAAATGGTGCTGGAGCCTGGGCTAGGTACCTCACAGCAACCATGGG
AAGCAAATAGGATTCATCTCCATTTCACAGAAGAGAAACTGAGGCTCTGAGAGAGAGAGAGAGAGACAGAAAGAGTTTA
ACCAGCTCAAGGCTCATCTGGCCCCAGAGCCCAGCTGATTTCCAGTCCCCTGTCAGCACATGTAGAGTCTCTCAGACTTG
GCAAAATCAGCCACTTGCTACTTACTAGCAGAACTGTAGAATCTCAGGCCCCACCTTAGCCTACTGATTCAGGATAGACA
TGTTCACAGGATTAATCAGGTGATGCGTATTGGAGTTAAAATTTGAGAAGCACTGATTTAAAACCAAAGAATCACCTGGA
GACTTTAGGAAAACACAGATTCCTAGACCCATCACTTGAGATTCTGGTTCAGAATTCTCAGGTGGGGCCTGAGAATTTGC
ATTCCTAACAAGTTCCCAGGTGATGCTATGATACTGATGATGCGGACCTCACGATGAAACCACTATTCCTCCCGCCTGGG
CAACATGGCAGAATCCCATCTCTACTAAAAATACAAAAATTCGCTGGGTGTGGTGGCATAAGGCTGTGGTCCCAGCTACT
CAGGAGGCTGAAGTGGAAGGATCACCTGAGCCTGGAGAGGCCGAGGCTGCAGGGAGCCATGATTGCACCACTGCACTCCA
GCCTGGGCAACAGAGTGAGACCATGTCTCAAGAAAAAAAAAAGAAAGAAACCACTGCTCTAGGCTAAATCCCAGCCAG
AGTTGGAGCCACCCAGCTAAACTGGCCTGTTTTCCCTCATTTCCTTCCCCGAAGGTATGCCTGTGTCAAGATGAGGTCAC
GGACGATTACATCGGAGACAACACCACAGTGGACTACACTTTGTTCGAGTCTTTGTGCTCCAAGAAGGACGTGCGGAACT
TTAAAGCCTGGTTCCTCCCTATCATGTACTCCATCATTTGTTTCGTGGGCCTACTGGGCAATGGGCTGGTCGTGTTGACC
TATATCTATTTCAAGAGGCTCAAGACCATGACCGATACCTACCTGCTCAACCTGGCGGTGGCAGACATCCTCTTCCTCCT
GACCCTTCCCTTCTGGGCCTACAGCGCGGCCAAGTCCTGGGTCTTCGGTGTCCACTTTTGCAAGCTCATCTTTGCCATCT
ACAAGATGAGCTTCTTCAGTGGCATGCTCCTACTTCTTTGCATCAGCATTGACCGCTACGTGGCCATCGTCCAGGCTGTC
TCAGCTCACCGCCACCGTGCCCGCGTCCTTCTCATCAGCAAGCTGTCCTGTGTGGGCATCTGGATACTAGCCACAGTGCT
CTCCATCCCAGAGCTCCTGTACAGTGACCTCCAGAGGAGCAGCAGTGAGCAAGCGATGCGATGCTCTCATCACAGAGC
ATGTGGAGGCCTTTATCACCATCCAGGTGGCCCAGATGGTGATCGGCTTTCTGGTCCCCCTGCTGGCCATGAGCTTCTGT
TACCTTGTCATCATCCGCACCCTGCTCCAGGCACGCAACTTTGAGCGCAACAAGGCCATCAAGGTGATCATCGCTGTGGT
CGTGGTCTTCATAGTCTTCCAGCTGCCCTACAATGGGGTGGTCCTGGCCCAGACGGTGGCCAACTTCAACATCACCAGTA
GCACCTGTGAGCTCAGTAAGCAACTCAACATCGCCTACGACGTCACCTACAGCCTGGCCTGCGTCCGCTGCTGCGTCAAC
CCTTTCTTGTACGCCTTCATCGGCGTCAAGTTCCGCAACGATCTCTTCAAGCTCTTCAAGGACCTGGGCTGCCTCAGCCA
```

```
GGAGCAGCTCCGGCAGTGGTCTTCCTGTCGGCACATCCGGCGCTCCTCCATGAGTGTGGAGGCCGAGACCACCACCACCT
TCTCCCCATAGGCGACTCTTCTGCCTGGACTAGAGGGACCTCTCCCAGGGTCCCTGGGGTGGGGATAGGGAGCAGATGCA
ATGACTCAGGACATCCCCCCGCCAAAAGCTGCTCAGGGAAAAGCAGCTCTCCCCTCAGAGTGCAAGCCCCTGCTCCAGAA
GATAGCTTCACCCCAATCCCAGCTACCTCAACCAATGCCAAAAAAAGACAGGGCTGATAAGCTAACACCAGACAGACAAC
ACTGGGAAACAGAGGCTATTGTCCCCTAAACCAAAAACTGAAAGTGAAAGTCCAGAAACTGTTCCCACCTGCTGGAGTGA
AGGGGCCAAGGAGGGTGAGTGCAAGGGGCGTGGGAGTGGCCTGAAGAGTCCTCTGAATGAACCTTCTGGCCTCCCACAGA
CTCAAATGCTCAGACCAGCTCTTCCGAAAACCAGGCCTTATCTCCAAGACCAGAGATAGTGGGGAGACTTCTTGGCTTGG
TGAGGAAAAGCGGACATCAGCTGGTCAAACAAACTCTCTGAACCCCTCCCTCCATCGTTTTCTTCACTGTCCTCCAAGCC
AGCGGGAATGGCAGCTGCCACGCCGCCCTAAAAGCACACTCATCCCCTCACTTGCCGCGTCGCCCTCCCAGGCTCTCAAC
AGGGGAGAGTGTGGTGTTTCCTGCAGGCCAGGCCAGCTGCCTCCGCGTGATCAAAGCCACACTCTGGGCTCCAGAGTGGG
GATGACATGCACTCAGCTCTTGGCTCCACTGGGATGGGAGGAGAGGACAAGGGAAATGTCAGGGGCGGGGAGGGTGACAG
TGGCCGCCCAAGGCCCACGAGCTTGTTCTTTGTTCTTTGTCACAGGGACTGAAAACCTCTCCTCATGTTCTGCTTTCGAT
TCGTTAAGAGAGCAACATTTTACCCACACACAGATAAAGTTTTCCCTTGAGGAAACAACAGCTTTAAAAGAAAAAGAAAA
AAAAAGTCTTTGGTAAATGGCAAATGAGCGTGTCTTTGTTTTGCAATCCCCCACACCCCACCTGACCGTCCCCTCTCCCC
TCCTCCCTGCCCTCTCCCAGGCCTCGTTTCTTAGAGACGCTCAGAACCACAAAGTTGCTCAGGAATGCTCTGAGCCCGTC
TGGTGACACAGGACCAAGAGGCTGAAAGGTAGAAGGAAGATGAGGACAGGAGGTCCATCTTGCAAATACCCTTCCAGAGT
TCAAGGGATAGGCCCCAAGCCTGCACAGTCAGCACTGAACCGGACATGCTGATCCTACTGCAGCTTTGGGAAGGCCAACT
TTTCCGGGTAACCTCGATACCTGGGGACTCTGCATTTAGCAGCTCTTGCACAGTAGCCTGCTCACCTGTCCCAGCTCAGA
CCAGAACTAGAGCCCTTAACCTTTTGTCAGCCCCTTAGACTCTGAAAGCTGGGGTTTGGAGGGAGGGCCTTTCTGTGGGC
TTGGAGACCACAGCATACTGGACTAGGGGTCAGGGATGAGACCCTTTGCTGGCATGAGAGCTGGAACCACCCATGATCCT
CTCTGCTAGCCACTGTAGCAGCAGTGGCCTCCGTGGTGTATGTCATAGTCCCTTCCTGCTGGTACAACAAAATGCCACCG
ATGGAGTAATTTACAAACCATAGAAATGTATTTCTCACAGTTTTAGAGGATTGGAAGTCCAAGATCAAGACGCCAACAGA
TTCAGTGTCTGGTGTGGACTCTCTCTGCTTCCGAGATGGGGCCTTGCCATGCCCTCTGGAGGGGACAAACACTGTGTCCT
CATGTGGTAGAAGAGTGGAAGATCACAATGAGCCTAACTAGTTCCCTCCTGCTCTTTTATTGCTATAAGGGCATTAATCC
CGTTCACAAGGGTGGAGCCTTCATGACCTAATCAACTCCCAAAGGCCCCCCCCAACTTCTCATACCATTGCCTTGGGGTT
TATGTTCCAACCATATGAATGTCGGAAAGATACATACATTCAAACCATAGTGTGGATACTGACTGTGGAGGAGCAGGGAG
AGAGGGACAGACAAAGGCCCCCACACAGGCCAGGTGTCCGTGTCATTTCGTGCCTTGGTGCCACCCAGAGCCGTGACTTC
TGCAAGCCAGAGAGCAGGTCAGTCCAACCTACAATGCGTCAAGAGGGGTCTGAAGGACCCAGAGCTGCAATAGCTCTGGG
CCGTGGGGAGCTAGAGGGAGGGCTGAGGCACTCACGCAATAGGTGATTCCATCCATTTCCCCAAGTGGAAAGAGCCTCGT
TGAAAATGGAGCTGCCTGGTAATGACACCCATTCGCTCATCTCGCAAGCACGTGACATTACCCTCCTCCGAGAGATGCAC
TGGGCTGGAAGGTTTTTAACAAGATTTGTCAGCTGGTCTCCAAGCCGGGAATTGGCGCCCTGAGAGGAATGAGCCTGGCA
CGGATGCAGTCAGCGTGGAGCGGCGTTTGCAGCAATGGCATTAAATATTAAAAGGCACAGCTGCGACTGTGTGCGTGAGC
GTGTGGCCAGGGGGCAATGCCAGTGTGCGTGTGTGTCCCCGAGGGGAACCTGCCGGAGCGCATGTGCGTGTGGAGCCCGC
TGGTGTGGACATCCTAATGAGGTTACAGATGGGAAAACAACCCTCGGTTCCTGAAGGGGAAGAATGTAACAGAGCAGTCA
TGCAAAAGGCAGCCAGGCCAGCCGCACAAATGCGCCCCCTCAGCTGCAGATACATTTTTCTAGAATAACTGGACATCTCC
AAATATTTACAGAGAAAACAAAACCCCCAGCAATGTCAATTCCCTCGAACTGCTGTGTCTCAGAAGTTCTGACCTCGGTT
CAGTGCTGGCCTGATCCCTGCTCCCTTGCCCGAGGCTCTCATGTGCCCATCTTGAGCTTGGGGTGACCCCTGGGAAGAAG
GAAAAGGCCCCCTGGGAGGCCCACCCAGGGCTTCTCTCTTCTTTTCTTACCTGCTTCATACATCTGAACCCTCTTCCCAG
GTTCACAGCCAATAAGCCTTCTCCAGTCACCTGAAATTTCCTTTTCATTTCATCCCCAGGCCCTCATCTCCCTCATCCTT
GCTGGCTGCAACCCCAAGCCAAGCCCAAGGTATTCCCAGGCCACAGGTCTTCCCTTTCAGCTAGAAGCACCACACCTTTC
TCATCACAGACTCACAGAGCTCCAAGGGACCTCAAAGATCAGCTGGGCCAATCCCCCAGTCATTTTCTAGATGGTGAAAC
TGAGGCCCAGCGGGGAGAAGCTGACTTGCCTGAAGAGGCGTGGCAATGCAGGGATATTGGTTAAACTTGCTTTGGAAGCC
TGAGCGTCTGGCAGGGTTGCAGGAGGGTGTGGGGGAGAGCAGAGACTCTGGAGCTCTGCCGACTTTGAATCTCATCTCTA
CTGCTTCCCAGCTATGTGACCTTCAGCTGTTGCCCAACTGGTCTGAGTCTTGGGTTTCTCAACTGCAAAATGGAGAGGAT
TTGTGTAAACAGTTAGCACAGTGCCTGGAATGTTGAGTGTGGTGGTGAGGAAGAGGGACTTTGCAGCCAGACTGCCTGGT
TTGAACCCCGGCTCCACTGCCTGGGCTGGTTACTTAGCCTCCCTGTACCTCAGTTTCTTAATCTGTATAATGGGGATACT
AATGGTACCTATGTCCTAGGATTGTTGTGGAAGCAACGAATCCATATATGTAAAATGCTTAGAAAAACTTACATATGTG
TTGTTGGTAATCTTTAGGAGATGTTTAAGAAACGGTAACTTAAAAAAAAAAGTTGCTAACCACAAGGAAACCAAATTCAA
TTCCTTAGCCCATTTCCATCCCCACAGAATTTATAATTCAAGCTGGAAGGCAATTTAGAGATGAGTGAATCTGGCCTTTC
CATCCATTGCATGGTGAGTCAACTCGTGCTCTGAGAGGGAAGCAACTAGCCTGAGGTCTCTTGGGAGGTCTTTGCAATGC
TGGAACTTTAACCTAGTTCCCTGGGCTCCTAACCAGAGCTCTTCCCACCAGACCCTGCTGGGCTCACGGGAACATCCCTG
CACATCCACCGTGGCACCCGCCCTCATCCCACGCTGTCTCGTTCTCTCCGGCAGGCACCGTTCATCTTGTCCTTTGATTA
GGTCCTTAAGGAGAACCTCCCTTCTCCCCTTTAAATAGTTCCTGTCCTTATCACTGAGTCTGGAGAATGAATGAAATATA
AAAGAAATATGGCAGCGGTCCCAAAGGAAATCAGGCAACTGATCTTTTTAAGAAAGGAAGATGGGCCGTCTTCTCTGGGA
ATGGTTCTCAGTTACATGTCTTTGTTGAGGGTGAACAGTTGGAGGAAAAGAAACAGACCTAAAGGGATTGTGGGGGGAAT
TAAGGCAAAGGAGATGGGGGAAAGGCAGGGGGAAGATGGGGCAGGAATGGGTTTTAGGGAATATACACTGGGGGCGGGAC
TTGAAGCAAATCTTCAGTGGGTAGTTAGATGTCCCATCCTGCATGCACACACATGCATGTGCACACACACACACACAC
ACACACACACACACACACATAGAGCCTCCCCAGGTCTCCCAATCTCAGGTGAGGAGAAGGGGGCTCTATTAGTCTT
AGAGACAGGTGGGCCCATGGCCTTCTTTGCAGAGAAGATGGTTAGAATCGGTCATGTGTCTCTGGTCTGAGACCCTAGAG
AAACTCCAAGGAGAATAAAAAAGGTTGGGGGGGGGCCTCTCCCTCAACTCAAAAGAGCAACCCTATCAGGCTTCAGAAAA
GCAAGCCCTCCTAGCACTCCAACCCACGGGAGCAGCTGTCTGAGAAGTCCCAACTCCTCCAGCAGCCACCCCTCTGTTC
```

```
CTTCACTTCTCTTGGGCCCAGGCAGAGACACAGACAGGAGTCTGTGTGCAGAAAAAGCTCCCCAGGCCCTTTTCTGTGGA
GAGCTGCTCCAGACTAAGCGAATCTCAGCTGATCTCGTGCGAAATCAGAAACGCCAGGATGTGTATTCCCCACGGGCTCC
TCTCTTTGTGTCTGTGCATGACCCAATTCAGACTCCTCTGCCGCTGTCAGATTCTCTCCTGCCCATATTCACTTTGAGGA
TGGATATAACCTTATTTTTGTGTGTTGTTCACTAACCCCAGGGACTTCGCTAGCTGGGCTGTCCTACGTGGTACAGTAAC
CACCACATAGTGTGGCCTCCAGGTTCTCATCTCTGGGTAAATAAACATCTCCGCACAGGCTTGGCCAGCTCTGGACTGGC
TCAGTTCCTGCATGATGCAATAGGTCTCCAGGTCACATATCCGTGCCCACTAGCAAGAGGGAAGGTAAGGAGATGGTTCT
TGCCCTGAAGTCCTCTTGTTCCTGTGTCAGAGTTTATTTAGGACAGAATAGTTTATATTTGCTCCAAGTAAATAATATCT
GACATTGGGAAGCACAGCAGATGCAGCAGATGAGATGCCCCCAAACCATTTCCCAATGTTTAAAAAGAGGCCTGGAAAGG
ACAAGCTCATTGATCTCATTTGGTTTATGAAACTGCACTCAAGTGACTGAAGCAGAAACCTGACCACGTGTAAAGACTGA
ATGGCTCAATCTGCTTCCCAGGCCCAGAGGGTGTTTTCTGTGGCCTGGCCCAGGCCTATGGTGAAGCCGGTGCTAGGGGG
TCACCTATGTGGGAGCCAGCTCTGCCTCCTGCCCCGAAGCTCCAGAGACTTCTCCTACAACTGACTCTCTGGACACGCCC
TCTTGCTTGCACTAGTATCTCTTACCTGCCACCTGCCCTCCTCCACACACCCCGATTTCAGGGAAGCTCAGCTGCTGAGA
GTGCTATTGACAACCTCTGGACTTGGAAATCAGGAGGAAGGGAATGGGAAGAATGGTGGCAGCTTTGTGCCTGGGCTGTT
CAGGAAGACATCCCTCTGCCTTTGTTCTCCAGGGCAAGACACAGGGCTAGACACGCCGGAATCCTTAAATCCTTTGTCAA
AAGCAGAACTGGGATTTTCGTCCAAGGACTACACCTGTCCTGTCCTCAGCCTCCTTCTCAGTTGCCCCCCTCCCACCCAT
GCTCATCAGAGCCGAACCCTGAGGAAACCATGGACCGAAAGACCTTGTGAGCTTTTGTTGCTGGGAGTGTCAGAGGCTGG
GAGACAGAAGGTATGAGAGATGAGATGCCATGGAAGGGATGGCAGCGATCACAGAGGCTGGGGGGGCAGAGGAAAGAGACG
TTGAAGAGGTGGAGGGGTAATGAACCTAGGCGGGAGACAGGCTAACATTTACTAAGTGCCCGTGATGCACCAAGGACTAG
GCTAGCTATACAACCTTTCATTGAATTCTTATGACAACAGAGAAGGAAACAGGTTTAGAGAGCTCATGTAACTTACCTGT
GGTGACACAGCTAGCAACTTTATTAAGATTTTGTTACGTACTATACACTGTATTAAGCATCAGTATCTTATCTCATTTAA
TCCCCACAACAACTCTACTAGAGTCTCATTTAATAAATGAGGAAACTTAGGCTCAGAGAGGTTAAGTAACTTTATCAATG
TCACACAGCTACTAAGTGATTTCAACCAGTTCTAGCTGCCTCCAAATTCTTGCTTTAGTCACCCTACTTCACCGTTCAA
CTAAACAGCTGTTTAATACCAAATATTTCTGGAGCCCCTACTGAGTGCCAGGCACTGTGCTGTGTCCAGCCAACATCTTT
CAAACCCTGCCAGACACAGAGCTCTGACCTGGGAGAAGGGAGAATCAAATAACAAAGACACAGCCCCCACCTGCCAGGGG
CTGTGACCCCCACAGCTGGGCAATAACCAGGTCTTTGCTGCTCCACAGTACCTTGTCCCTGCTTCTCCCCTCTAGGCCCT
GCTTCTTACCTAGGCTTCCTCCATGGGCTAGCCCTGCAGATGACTGAGAGGGCTGAGATGGCTTTGCCACAGACTAGTCA
CTGGGCAATATGGGCAGGTCACCTCCCTTTTCTGGGCCTCCAGCTCCCCAGCTGTACAATGAGGTTGCTGGACCAAATCA
TTTCTACCCATCGCCTCCAGCTCTGACTTGCTATGGGTGCCTTGTCTGGGCAGAGTCTGGGAGGCCAGTGCTCAGCGGGG
TAGGGCTCTGGGCATGTTGATGCTTGCACGAGGTGCAGGGCAAGTGTGTGAGGGGCTGAGGAAGCTGCTCCACCCCCAGC
TGTGGTTTCTGGTGGGGCCACCATGGCAGCAGGTGGAGGGAAGTGTTTGTCACCCTTCCAACAAAGGCAGTCAAACAAAA
GCCTCCCAGGGCACCATGGGAATAGGCGGCAAGGGCGCCAAAAATAAAACACACTTCTCCTTTGATAGGCATCGGTGGCA
GGCATAGGCAGCTGCTAAGGGAGTCTGTCCTTGGGGTGGTGAGGAAAGGGTTCAGATTTCGTGCCCAAGAACACTCACAC
TCACAAGAGCTGTCCCACCTCACAAACAGCCCACACATACAGATGCTCAATATTCACACAAGAAACACCACCGCACACAT
CACACACACACACACCAACACAGCAGTCACACAATCAGCATACACATATACACATACAAATAGAAAACTTGCCCCCATAC
AAGACCAGCTTCATGGGCCGCGACCTGCACAGTCACTCTGGGCCCTGCACTAAGGCCCTGCACTAAGGCCCATGCTGGGT
ATAATGCTTTGCTGCCTGCACTTTGAAATTCTTAATTATTTTTAAACAAGGGGCGCAGCATTTACATTTTGCAATGGGCC
CTACAGGTTCTGCAGTTGCTCCTGTGGGTGTGCACTCATGAGAGCGTTCACTACCTGCTTAAGCATACCTTTTAACACTT
TTACACTGTTGATGGGAGTGCAAATTAGTTCAACCATTGTGGAAGACAGTGTGGTGATTCCTCAAGGATCTAGAACCAGA
AATACCATTTAACCCAGCAATCTCATTACTGGGTATATACCCAAAGGATTACAAGTCATTCTACTATAAAGACATATCCA
CACGTATATTTATTGCAGCACTATTTACAATAGTAAAGACTTGGAACGAACCCAAATGCCCATCAATGATAGACTGGATA
AAGAAAATGTGGCACATATACACCATGGAATACTATGCAGCCACAAAAAGGAATGAGATCGTGTCCTTTACAAGGACATG
GATGAAGCTGGAAACCATCATTCTCAGCAAAATAACACAGGAACAGAAAACCAAACACCACATGTTCTCACTCATAAGTG
GGAGTTGAACAATGAGAACACATGGACACAGGGAGGGGAACATCACACACTGGGGCCTGTCAGCGGGTAGTAGGGGGCAA
GGGAAGGGAGAGCATTAGGACAAATACCTAATGCACGAGGGACTTAAAACTTAGATGATAGGTTGATAGGTACAGCAAAC
CACCATGACACATGTATACCTATGTTACAAACCTGCACATTCCGCACATGTATCCTGGACCTTAAAGTAAAATTAAAAAA
AAAAAAATCCTACCTATATAGACAAGAGTGCATCCGCTACACACATACACACAGATGCACAAACACATGCATGCCACAAA
TGCAACACCCACACAAACAGCAATAGCCCCCATGTTCACGCGCCAAAGCACACATTGATACCCAGAAGGGGTCCAGA
CGCTCACAAGCAACGCTACAACATGCAGCCTATGCCCTGCAGCCTGCTGACACAATTCAATTGACTTAACATTGGGGGCA
GCAGAACAATGTGAAGGGAAGGGGCTTGGAGAGAAAACAGGTTCATGTCCTCCACCTGCAAACATCTCCCACCTCTCTCT
TCGGCCCTGGGCTCACCCACCCCACTCAGCGGCTGGGACCAGAAGAAAGGGAGAGCCAGGGAGGTGGCGCATGGACGGA
AAGGGGACAGGGATGGAGGTGGAGAGGGAGAGAGAGAGGGCAAGAAAGAACGGGTGGGGAGTGGGGGCAGGAACTGAGTC
CCCAGGTGAGCTGGCCCCAGCAGTGGTCTCATAAGAGCTCAGAAGGTCTTGGACCCAAAGCAGTAAGAACAATGCCCCTG
TGCTCCCCGCAAGCACAGGGCGGTCCCGCCCTCAGAGTCAGTCAGAGGATGCCCACCTCCCTTCTTACTAGCTACACAGC
CTTGGGCACGCCGTGTAACTCCGGGCCTCAGCTTCCTCCTTTGTGGAATGGGATAGCCAATTCTACATTGCAGGGTGGCA
AGGCACACACAGACACAACACCTCCTGGATTCCTGCAGCAATCAACAGCTCGTCTCGTGATCAGGCCTGCAATCTGATTC
TTGGCACAGGAACTCCAGACCCCCTCATTACAGCAGACATTTGGAACGCGTTTATTTCCCATCTTCTCCTGGTCTCACTT
CACACCTCACAGCCAGGTACAAGCACAGAGGTGGATGTAATTCATGGCCTCATGGCCACCATCACTTCAGGGACCTTCAG
GATCCTGCAGCCCCATCCTGCCCCACCCCCTACCCAAACCAAGCAGGACTCCAAGGCCTGGAAATTTTTCTTCCTCCTCT
ATTCTTTTTTTTTTTTTTTTTTTTTTTTTGAGACGGAGTTTCACTCTTGTTGCCCAGGCTGGGCAATGGCGCGATCTCG
GCTCACCACAACCTCCGCCTCCAGGGTTCATGCCATTATCCTGGCTCAGCCTCCTGAGTAGCTGGGACTACAGGCACCCA
CCACCATGCCCGGCTAATTTTTTGTATTTTAAGTAGAGACAGGGTTTCACCGCGTTAGCCAGGATGGTCTCGATCTCCTG
```

ACCTCATGATCTGCCCGCCTCGGCCTCCCAAAGTGCTGGGATTACAGGTGTGAGCCACCGCGCCTGGCCTCCTCCTCTAT
TCTTTACCCCCAACCTGTATTCTCCACCCCCAACCCTAACCTTCCTGATTTATTTATTTATTTATTTATTTATTTATTTA
TTTATTTAGAGACAGAGTCTTGCTCTGTCGCTAAGGCTGGAGTGCACTGGCTCACTGCAACCTCTGCCTCCCAGGTTCAA
GTGACTCTCCTGCCTCAGCCTCCCGAGTAGCTGGGATTACAGGCATGCGCCATCACGCCCAGCTAATCTTTGCTTTTTTT
TTTTTCTTTTTTTTGACAGAGTCTCGCTCAGTCGCCCAGGCTGGAGTGCAGTGGCACGATCTCGGCTCACTGCAAGCTCC
GCCACCCGGGTTCACGCCATTCTCCTGCCTCAGGCGCCCGAGTAGCTGGGACTACAGGCGCCCGCCAATATGCCCAGCTA
ATTTTTTGTATTTTTAGTAGAGACGGGGGTTTCACTGTGTTAGCCAGGCTGGTCTCGAACTCCTGACCTCTAGTGATCCGC
TCACCTCGGCCTCCCAAAGTGCTGGGATTACAGGCATCCCTATTTAAAATTGTCCCAGCCTCTGTCCCTTCCCTGAAAAC
CCCATCATTCTGTTAGGTGATACCAGATTCTTAAAGAGTGATTCGGGAAAAACGAATGGGCCCTAAGGGGCATGCCAGTG
TGCCCCATTCCAATTACAGTCACCCACAGGAGAGAGGAAAGGTCGTCCAAGTGGCCACCAACAAGAAACTATTTCATCAG
ATTTCTTTTTGTTTGTCTGTTTGAGACGGAGTCTCACCCCGTCACCCAGGCTGGAGTGCAGTGGCGCGATCTCGGCTCA
CTGCAACCTCTGCCTCCCGGATTCAAGTGATTCTCTTGCCTCAGCCTCCCAAGTAGCTGGGACTACAGGCACGCACCACC
ACATCCAGCTAATTTTTGTATTTTTAGTAGATACAGGGTTTCACCATGTTGCCCAGGCTGCTGTCAAACTCCTGACCTCA
TGATCCGCCCACCTCAGCCTCCCAAGGTGCTGAGATTACAGGCGTGAGCCACTGCGCCCGGCCTATTTCTTCAGATTTCT
TATAGGACATAAGTCACTCAGATCCCCTCACAGGCTTTCTGTCAACTTACTGAGAGGGAAACTGAGTCAAGGAAGAGTGA
AGTGACTTGGGTGAGCAGCAGAGTTGAGGGTTGTGGTCTTTCTGATGTGCAGTCTCCATGAGACCAGATC

HUMAN mRNA SEQUENCE : hR1-004.1 (Seq ID No: 5)
AGACAGGGGTAGTGCGAGGCCGGGCACAGCCTTCCTGTGTGGTTTTACCGCCCAGAGAGCGTCATGGACCTGGGGAAACC
AATGAAAAGCGTGCTGGTGGTGGCTCTCCTTGTCATTTTCCAGGTATGCCTGTGTCAAGATGAGGTCACGGACGATTACA
TCGGAGACAACACCACAGTGGACTACACTTTGTTCGAGTCTTTGTGCTCCAAGAAGGACGTGCGGAACTTTAAAGCCTGG
TTCCTCCCTATCATGTACTCCATCATTTGTTTCGTGGGCCTACTGGGCAATGGGCTGGTCGTGTTGACCTATATCTATTT
CAAGAGGCTCAAGACCATGACCGATACCTACCTGCTCAACCTGGCGGTGGCAGACATCCTCTTCCTCCTGACCCTTCCCT
TCTGGGCCTACAGCGCGGCCAAGTCCTGGGTCTTCGGTGTCCACTTTTGCAAGCTCATCTTTGCCATCTACAAGATGAGC
TTCTTCAGTGGCATGCTCCTACTTCTTTGCATCAGCATTGACCGCTACGTGGCCATCGTCCAGGCTGTCTCAGCTCACCG
CCACCGTGCCCGCGTCCTTCTCATCAGCAAGCTGTCCTGTGTGGGCATCTGGATACTAGCCACAGTGCTCTCCATCCCAG
AGCTCCTGTACAGTGACCTCCAGAGGAGCAGCAGTGAGCAAGCGATGCGATGCTCTCTCATCACAGAGCATGTGGAGGCC
TTTATCACCATCCAGGTGGCCCAGATGGTGATCGGCTTTCTGGTCCCCCTGCTGGCCATGAGCTTCTGTTACCTTGTCAT
CATCCGCACCCTGCTCCAGGCACGCAACTTTGAGCGCAACAAGGCCATCAAGGTGATCATCGCTGTGGTCGTGGTCTTCA
TAGTCTTCCAGCTGCCCTACAATGGGGTGGTCCTGGCCCAGACGGTGGCCAACTTCAACATCACCAGTAGCACCTGTGAG
CTCAGTAAGCAACTCAACATCGCCTACGACGTCACCTACAGCCTGGCCTGCGTCCGCTGCTGCGTCAACCCTTTCTTGTA
CGCCTTCATCGGCGTCAAGTTCCGCAACGATCTCTTCAAGCTCTTCAAGGACCTGGGCTGCCTCAGCCAGGAGCAGCTCC
GGCAGTGGTCTTCCTGTCGGCACATCCGGCGCTCCTCCATGAGTGTGGAGGCCGAGACCACCACCACCTTCTCCCCATAG
GCGACTCTTCTGCCTGGACTAGAGGGACCTCTCCCAGGGTCCCTGGGGTGGGGATAGGGAGCAGATGCAATGACTCAGGA
CATCCCCCCGCCAAAAGCTGCTCAGGGAAAAGCAGCTCTCCCCTCAGAGTGCAAGCCCCTGCTCCAGAAGATAGCTTCAC
CCCAATCCCAGCTACCTCAACCAATGCCAAAAAAAGACAGGGCTGATAAGCTAACACCAGACAGACAACACTGGGAAACA
GAGGCTATTGTCCCCTAAACCAAAAACTGAAAGTGAAAGTCCAGAAACTGTTCCCACCTGCTGGAGTGAAGGGGCCAAGG
AGGGTGAGTGCAAGGGGCGTGGGAGTGGCCTGAAGAGTCCTCTGAATGAACCTTCTGGCCTCCCACAGACTCAAATGCTC
AGACCAGCTCTTCCGAAAACCAGGCCTTATCTCCAAGACCAGAGATAGTGGGGAGACTTCTTGGCTTGGTGAGGAAAAGC
GGACATCAGCTGGTCAAACAAACTCTCTGAACCCCTCCCTCCATCGTTTTCTTCACTGTCCTCCAAGCCAGCGGGAATGG
CAGCTGCCACGCCGCCCTAAAAGCACACTCATCCCCTCACTTGCCGCGTCGCCCTCCCAGGCTCTCAACAGGGGAGAGTG
TGGTGTTTCCTGCAGGCCAGGCCAGCTGCCTCCGCGTGATCAAAGCCACACTCTGGGCTCCAGAGTGGGGATGACATGCA
CTCAGCTCTTGGCTCCACTGGGATGGGAGGAGAGGACAAGGGAAATGTCAGGGGCGGGGAGGGTGACAGTGGCCGCCCAA
GGCCCACGAGCTTGTTCTTTGTTCTTTGTCACAGGGACTGAAAACCTCTCCTCATGTTCTGCTTTCGATTCGTTAAGAGA
GCAACATTTTACCCACACACAGATAAAGTTTTCCCTTGAGGAAACAACAGCTTTAAAAG

HUMAN PROTEIN SEQUENCE : hP1-004.1 (Seq ID No: 6)
MDLGKPMKSVLVVALLVIFQVCLCQDEVTDDYIGDNTTVDYTLFESLCSKKDVRNFKAWFLPIMYSIICFVGLLGNGLVV
LTYIYFKRLKTMTDTYLLNLAVADILFLLTLPFWAYSAAKSWVFGVHFCKLIFAIYKMSFFSGMLLLLCISIDRYVAIVQ
AVSAHRHRARVLLISKLSCVGIWILATVLSIPELLYSDLQRSSSEQAMRCSLITEHVEAFITIQVAQMVIGFLVPLLAMS
FCYLVIIRTLLQARNFERNKAIKVIIAVVVVFIVFQLPYNGVVLAQTVANFNITSSTCELSKQLNIAYDVTYSLACVRCC
VNPFLYAFIGVKFRNDLFKLFKDLGCLSQEQLRQWSSCRHIRRSSMSVEAETTTTFSP*

Table 2

MOUSE GENOMIC SEQUENCE : mD3-010 (Seq ID No: 7)
TGTCTTTTTCAGTTGCTCTCTACCTTATTTTTTGAGGCAGAGTCTCTTACTGAACCAGAGGTTAGCTCATCTGGCTATAC
CGAGTGACTAAGGAAAGCCCTGGGATTCTCCTGTCGTTGCCTCCCAGGTGCTAGGATTACAAGCTCTGGCCATCATACTC
AATTTTAAAAAAAGGATGTGAGTTCTAGGGTGTTAGATTCAGATGCCACAGGCAGGCAGGCAGGCAGGCAGGCAGGCAGG
CAGGCAGGCAGGCAGGCAGGCAGGCAGGCAGGCAGGCAGACAGGCAAGCAGGCAGGCAGGCAGGCAGGCAGGCAGGCAGG
CAGGCAGGCAGGCAGGCAGGCAGGCAGGCAGGCAGGCAGGCAGGCAAGCAGACAGGCAGGCAGGCAGGCAAGCAGACAGG

```
CAAGCAGGCAGACAGGCAAGGCTGCACACTACCCTGCAGATTCACCTCCAGAGTGCTGATGAGAGGCACGGGAGTGTCGG
GCTCCCGAGTACAGGCCAGGCGCGGTAGGTTCTTTACCAACTAGCATTTCCCCGGTCCCAAGATTTTAACATTCTTTCAC
TTAGAGTAAACTAAATCTTTGAACCAGCTTACTTTCTAATAAACTAGTTTTCTGAAGTCGTTCTTTCTTGGCATTGGGAA
CTTCACCTAGAATTCTGCACACGCTCACTTTGCCAGTGAGCGGAATTTCTCCTCTCTTTCAGACAAGGAAGGTCTCACGA
AATTGCTCCGGCTGGTCTTGAACTCACTGTAGCACAACAGGCCTTGAATACTCTGCCCTTCGACCTCAGCCTCACAAATA
GGGTTACAACCTACATCATCAGGTTCCTCTACGCAAACATTATTTTTATTTACATGCCTGTGTGTGTGTGTGTGTGTGTG
TGTGTGTGTGTGAACATATGCGCCATGTGTGCAGGTGTTAGCACAGGCCACAGGGGAGTGCCAGATTTCCTGGAACTG
GAATTACAGAGAGTTGTGAGCTGCCTGACATGGGTGCTAGGGACCAAACTTTGGTCTGAAAGAGCTGCAGATGCTCTTTA
CCCAGTGAGCTCTCTTTCCAGCCACATCCTATATCCTTTAACATTGTACAATCCTGTACCTTAAGCAGGGTGNNNNNNNN
NNNNNNNNNNNNTATTTTTGAGGCAGTGTCTCTTACTGAACCAGAGGTTGGCTCATCTGGCTATACCGAGTAACTAAGG
AAAGCCCTGGGATTCTCCTGTTGTTGCCTCCCAGGTACTAGAATTACAAGCTCTGGCCATCATACTCAATTTTTAAAAAA
GGATGTGAGTTCTAGGGTGTTAGATTCAGATGCCACAGGCAGGCAGGCAGGCAGGCAGGCAGGCAGGCAGGCAGGCAGGC
AGGCAGGCAGGCAGGCAGGCAGGCAGGCAGGCAAGCAGACAGGCAGGCAGGCAGGCAGGGCTGCACACCACCCTGCAGAT
TCACCTCCAGAGTGCTGATGAGAGTCACCTGAGTGACGGGCTCCCGAGTACAGGCCAGGCGAGGTAGGTTCTTTACCAAC
TAGCATTTCCCCGGTCCCAAGATTTTAACATTCTTTCACTTAGAGTAAACTAAATCTTTGAACCAGCTTACTTTCTAATA
AACTAGTTTTCTGAAGTCGTTCTTTCTTGGCATTGGGAACTTCACCTAGAATTCTGCACACGCTCACTTTGCCAGTGAGC
TGAATTTCTCCTCTCTTTCAGACAAGGTCTCACGAAATTGCTCCGGCTGGTCTTGAACTCACTGTAGCACAACAGGCCTT
GAATACTCTGCCCTTCGACCTCAGCCTCACAAATAGGGTTACAACCTACATCATCAGGTTCCTCTATGCAAACATTATTT
TTATTTACATGCCTCTGTGTGTGTGTGTGTGTGTGTGAACATATGCGCCATGTGTGCAGGTGTTAGCACAGGCCACAG
GGGAGTGCCAGATTTCCTGGAACTGGAATTACAGAGAGTTGTGAGCTGCCTGACATGGGTGCTAGGGACCAAACTTTGGT
CTGAAAGAGCTGCAGATGCTCTTTACCCAGTGAGCTCTCTTTCCAGCCACATCCTCTATCCTTTAACATTGTACAATCCT
GTACCTTAAGCAGGGGGTAGTGGAGCACACCTTCAAACCCAGCACTTCGGAGGCAGAGGCAGGCAGATCTCTGTGAGTTT
GAGACCAGCCTGGTCTATAGAGGGAGTTCTATAACAGCTAGGGTTACACAGAGAAACCCTGACTCCAAAAAACAAAGCGA
ACTTCTCTGCCTTAACATTCTACAGTTTTAGCTGGGACTAGTGGTACATTTGATAGGTGGAGGCAGGGGGATTATGAACT
CAAGGTCAGTCTTTAAATAAAACCTGCCTCAAATAAATATACAAAACCAAAATAATTATAATACTTTCTGTGAGGAAAT
ATTGTTCAATAATCATTTTTCTTTCCTTCTCTCTCCTCTCCCCCCCCCATCTTTCTTTTTTAAGACAGGATTTCTCTATA
TAGCCTTGGCTGTCCTGGACTTTGCTCTATAGACCAGGTTGGCCTCAATCTTAGAGATCCTCCTGCCTCTGCCTCCCCAG
TGCTGGGATTATAAGCATGCGCCACCTGGCTATAATTATTTTCTATATTATTTCATTATTTTATCTACTAACATTCCAGA
ATAATATTTTAGGAGGAATATTACTTTTTTTGTTGGTGGTGGTTTTTTTGTTTTTGTTTTTTTGAGACAGGGTTTTTCTG
TGTAGCCCTGGCTGACCTGGAACTCACTTTGTACACCAGGCTGGGCTCGAACTCAGAAATCTGCCTGCCTCTGCCTCCCG
AGTGCTGGGATTAAAGGCATGTGCCACTACACCCGGCTTGGAATATTACTTAATAATTATTGTTTTCCACACTGATTTTG
TGCCTTGACATCCTGTAACTGTGTTCAACATTATCCAGTACTTTTTCCTACTATGATTTTCTTTATATCCTGCAGTTTTC
TTTTCATTTATGTAAATGTGAATTTATGCCATACGTGTGTGACGTTCAGAGGACAGAAAGAGGTGTCAGATCCTCTAACG
CTGGAGCTACAGGTGGTTGTGAAGTTGTGGGCTACCGTGTGGGTGCTAGGAAATAAACTTGGAACTTGGAAGAGCAACAA
ACGCTCTTAACTACCGAGCCACCTCCAGTCTCTATATTCTGCAATTTTGTATTCCCAATGTCTTAAAGCCCCTGAACAGG
AATATTATTTAATAATAGTAATTACATCATTTTTTCTGCATTCTATGACTTTTGATCCTCCAGGGGGCAACACTGTTCCC
TTAAGAAGCAGTCTTTGTGGAGAAGAGCTTATGTTCTATGGCTGTGTTCTGCCAGGGATGCTTGGTAGTCTCTGCTTCTC
ACCTGGAATCGCACCACCTTCTCTGTGGCAAGGCATAAGTAGGTGCCAGCCCCTCCTGGGGTGTCACTTGGAAACTGGAA
GGCACACTTGTGCAGCTGGGATATGGGTTCATCCACATCAAGGAGGGCACACTGCTTGGCCACTTTTCGGATGATCTGCG
GCAGAAGGGTGACGGGCGGGTGTGTGTGTGTGTGTGTGTGTGTGAGGTAGCAGGGTGTGGGGTCCCCAGAGGCTCCTCAC
CCAGCCAGGCAGCTCTATTCTTTGATAGCCATGAAGTCCCATACCATAGGCGGCAGTGTAATGCCTGTGACAGTGCAGAC
AAGCTGCACCAGGGAGCCGTAGCGGATGTAACCTTCCTGGGGTGGGAAGTCCCCTTGGGAACAGTGCCCATCAGCTGGAG
ATGAAAGAAAGGAAGGAAGGAAGAAACAAAGGTTCATTACTTCTCAAACCCTGTCTCTTCACCTCTGGCTTTAACGCACA
ACGCAGCTAGCCATGAGAAAGGAAGCTAAGACCAAGGTTCTAATCCTGGAGAGCTAGTCTGTGATCATCCACAGTAAGGT
GTGCTCCCTGGGGGCAGCCACTGATTGGCTGGAGGAGAAAGGTCATAGCTAGCCACTGGGATCAGAAGACATCGGGTTCC
TGACCGACTCACTAGGGCCAAGGGCAAGCCTTCACCCCCCTCCCCAAGGGACAGTTACACTGCTTCATCTTCCTGTAGGG
TTGATTAACTATGCTAACACACGGAGCACCCAGCCCGCACCTGCTCATGAGATTACCCAGGTGGAGGGTGAAGGCAGCCC
ACTGCCTTGCGCTGGCCACAAAGGCCCCATCTTCCACAGAGAGGTAACGCGTGGACACTGTCTGGGAGCGCAGGCGATTG
AAAAGGGAGACCTTTGAGCCGGAGGAGATGCATACTGCGAGGGGAACGTGGAATCAGTACCCAGGCCTCTCAGATGTGGT
CTCTCAGATGCCTCAGCTCATCTTCTGACCCATGTAACAGCCTGGCTAATATGCCCACTGGCCTAGACTCAATGGTTATC
TGCATTTCATAGCCTTTTGCCTGGGGTCTTGATTTTTGAGCTTGCCAGCTCCCTGGTACAAATGAACATCCTTCAGTGAT
GTGAGCTGGGTTCATCAAGTCACTCGGGGCTGCTTTGTGGCAGGCACGGCGTCGGGTACTGGGTATTGATGTGTGAACTG
GGTCCAGCTTTGCTCTGAAATAGTTCATGGTCTGGAGGGCATAAATACAAAATGCTAACTCCGTGTGGGAAAGGTCTAAG
CTAAGGAAGGCTTCTTGGGGGTGGGAAGTGTGAAGCAGGTGGGAGTTGCATAGATGGCTGCTAGAGTCTGAAAGAACAGG
GGGACTCTCCTAGAGCCTGGCTTGTCTTCCAGCCGATGGCATGCTCTCAGTCACAGCTTCATTTTTTTTTTTACTGGAC
TTACACCAGCTGGGGATCAGGCACTGACCAAGTGCTGTTCATCTCCTCTCCGTGCTGGACAGACTGCTGGCCTTCCATTT
ATACCTAGCCCAAGCCCTTTCCATTTAATTTACTATACAATAGATTCTTCTGCTCTTCCCTTTTTCTGTATGTGTGTGCA
GGCGTGTGGCTGGACACACAGCTGTGTGTGCGTGGGTGTGGAGGCCAGAGCTAGACTCCCGACACCTTATTCTATTAC
GCTCTACCTTATTCTCTGAAACAAAGGCTCTCACTGGATTGAGAGCTCTTTGGCTAGAGCTTTCTAGCCAGAGAGTTGGA
GGAGGATCCTATTGTCTATTTGCTCAGAGCTGGGATCACTGACATCTGCAGCCACACTCAATTTTTACGGGTATTCAAAC
CCAGGCTCATGTGTTTGCCTTGCAAGCACTTTACCCACCAAGCCATCTCCCCATCCTTTGCTTTTCTAAATACATATGTC
```

```
AGCTCAGTTCACTCTCCAGGACAGGATTCGAAGCCAGACTGCCAGACTCTGCACGCATAACCATTACTCTAGGTCCCCCA
CTTTTCCTGAGCACCAAGGTGCCAGCTAGGACACTGCTTGGAACTGGGATATTCCACAGGACTTTTCAATGACACCAAGG
AGCTGGATTCCGGGATCACACTGAGTCGTGGTTACCTCCAGACTGGGGGACACTTTCTGGGCAGACAGTCTCAAGCAAGC
CACATGGTATGCTATACTGAAATCAACCAATCAACCCACCCTCTCTCTTTCCTTCTCTCTCCCGATCCATTTTTCTTTCC
TTTCCCCACTCTCTCACTCCCTCCTAGGCTGACCTAGATAAGAGTTCAGAGTTCTTTTATCTAAGCACTGGAATTACACA
TGGGAACCACCATCTTTGGCTGATGCAACTTCCTTAAGGTCATTTTTAATTTGCCCCGGATTTGGCACCTGGCTGCAGCT
TTTGTCTGTTCTGAGTCCCCACTGTCATCTTCAAATCTGATATCTATCTCTTACCTGTGTCCTGTCTGAAATCTCTGTG
TGCCTTTTCTCTTAGTCTAGGCCAGCCACTCCACCCAGACTCCTCCCTTCTTTCTACCCAGACTCCACCTCTCACCCCCT
CCTCCACACTTGCACCCTCCGCCCATTTTTGCAGCTCTGGGTTCGAATTGCACCCTTCCACCTCGCCCCTCTCACGGTCT
GTGTTTTTCAGCGACTGCTTCTTCTGCGAGGGCTTGGAGATCACCTTGATGAGGCGACTGTGGAAAGTGCCCAGCTCCTG
GCCCCCTCGCAGCACCAGTCGCAGCACCAGCCGGAAGTGCTTCCTCTTATCGGCGTCCGAGATGTACAGGGTCTTGGCAC
AACCAAATTCCTACGGGGCTCAAGGGGATGCGGCAGGCCTTATCTCAACTTTGCCAGCGGAAGAGCCAACCTTCCGCAGT
TATGACATCCTTACCATTAGGACAAGAAACCGTACTAATCTAGGGTCTAGGTCAGAACCCACCCTCTGGCCTCCCCAGTT
GCCTTTTGGCTTCTGGAGGGGACTTGGGGAGTCTACAGTTTTGCCTCCTCTACAAAAAGAAAAAAAAAAGAGGCAACGCC
AGGAGCAGGAATCTGATGCTCTCTAGTGCAGCCTCACCCTGGAGTCCGGCTGCTCTTCAAAGTTCAACTTCTGGGTCTCG
GGAGCGCTGCCGGACGCACCGTCCAGTCCCATGTAACCGCAGACGGTGGGCCCGGTCTCCGCAGATTGGAGGGCTGAAAA
AGGACGGGAATTAAACCCTGGAAGTCTGGGGGAAGCTCAGGTGCGGGTCAAACTTAGTTCTTGCCGCCAGAGGGCGCAGC
GAGACTGCGCCTGCAACCGTCTGGGCTCCCGGGCCCTGCTGAGGCAGTTGGTCCTTCCTGCAGAACCGAGCAACAGCCCG
TCAGGGCACTCTCACCTTGGTCTTGCATTGGCTTCACCCTCCAGCCAGGACCCGCGAGGTAGACACAGGGTGGAGGACAG
AAGAACCTGGGAACACATAACATCATTTTCACTGTCTTAAAAGTGTCTCAAGGTGGCATAAATTTTTAAAGAATCATGAT
TTAGTGTCCCCTCTCCCAGAGTGAAACCGTTAATGCATTATTAGACCTCATCAGTAAGTCCTGCCCGCTCCATCTTCAAA
ACACATCTGGAATCCGTTAGATTCCTACCAACTTCACCTTGGTCCTAACCACTATCCCTTGCCTTCCCAACTCAGCCTTC
CATCTGTCTCTCCCTTGCTACAGATGCCTCTTTTAGTTTGTTTTTCACACAGCAACTAGATTACTTTAAAAAATTTATTT
ATGGATATGAGAGCTCTATTTGCATGTATGCCTGCATGGCAGAAGAGACCATCTGACAGAAGAGAACATCAGATCCCATT
ACAGATGGTTGTGAGCCAGCAAGTAGTTGCGAGGAATTGAACTCAGGTCCTCTGGAAGAGCAGCCACCAGTGCTCTTAAC
CCTGAGTAGTCTCTCCAGCCCCAGATTAGTTCTTAAGTAACAGGTGGCTCAGTGGATAAAAGGCTTGCTGAGGCAAGCAT
GATAACTTGACTTCAATGCCCAGAACCCATCAAAAGGAGGAAGAGAACCAACTGTACACCTAACTGCACACATTATCCTC
TTACCTCCACAGGAATCGTGGCATCTATACATCCACACAGCACCAGTATCACCACCAGCACCAGAACCACCACCAACATC
AACAACAAAATAACAGTCAAGGGGTAGGAGAGACGGCTCAGCAGTTAAGAGCCCTGGCTGCTCTTCCAGAGGTTCTGAG
TTCAACTCCCAGCAACCACATGGTGGCTCACAACCATCTGTAATGGGATCCAATGCCCTCTTCTAGTGTACAGGCATGCA
TGTAGACAAAATACACACACATATATAAAATACATAAATAATCTGGAAAGAAGTAACAGTTAAATTAAGTTGGGTGTGGT
GGCTCACGTTTGTAATCCTAAACCCAGTACGGAAGGGATGGGGCCTCTGAGGCAGGAGGATTGGTATAGGTTCCGCCTGA
GCTAGAGTTAGAGTCTGTCTTATCCCCCCCAAAATATAACAAAATAAACGATATTATTAATAATAATGAAAATGAATCAA
ATTGTGATGTCCTCTTATCAACATCCCTTGTGGTTTAAAATCCCAAATCCTCACATGACCTGGACACTGCTTCTCCTGTC
ATCTTCTCTAACGTCCCCCTCTTCTGACCAGTCTCCCCCTTTCATCCTCTTACAGTACTGAGTATATAGCCTTGAGGTCC
TTGCGTTTTCTATTGCTTCCAGTTAGAATGCTTCCTGGAGTTTTCCCATGCCTGGCTCTATCTCAGCACTGGTACATCAG
CTCCAATGTCACTTCTTAAAGAGCCCTTTGACCTCCTTGACCTTTCAAGCTAAGGTATCCCCTTTGGTCTACTATGTGAC
TCCACTCTGTTTTTCTCTGGCTCTCATCACAATCTGAAATACCTTGTTTGCTGGCTTGTGGGGAGGTGACTGCATGTCCC
CTACCAGAATGGAGATGCCATGGAAGTAGAGGCTGTATCTGCCTGGTCCAGCTGGAGGCTTAGTGCCCAGCAGTGACCCT
GGTGCGTGACATGTAGTACGCAGCAGAATGAATGGATACTGGTCGGGGATGTGGGGATCTGACAGCCACTGTCAAGTTCT
GCTCTAGATCCCAGTAGGCAGCTGTTGTTGGCTGGGGAACAGGACCCTGGCTGAACAATGGCTGAGGTGTCAGCCTTGAG
GCCTCCAAGATGTCAAAGAAGCTAAATGGTGGTGGGTTTGGAGGGAGGCATAGGGCTGAAGGCTGTGGAGACCTGGGTCG
GGGTAGGGAGCCACCTACCGCTTCTCATTTCCATAGGATTTCTGAGCCACCTTGGCATGCAGGATCCACACAGTCTGCTC
ACATCTTTGCTGCAGGCATGTGCGCACACCTTCTCTTAGGATGATGGCATGCTCTGGGGGTGACCTAGGAATGGGGGTTG
GGCCCACGGGGCCACAGGAAGGAGAGTCAAGGGTTGAGTTGGCACAAAGCCCACCCTGAAGCTGAGTGAGCTGAAGTTG
GAGATGGGGTTGATTTATGTCCTCCAAGGGATCTGGGGGAAGAAGGACTTGGGAAATGAGCTAAATATTTGTGTTCCAAG
AACCCTGCACAGACCTCACCAGGATCACCCAGGTGCTACACTGTGTGGGTGGCTTCCCGGCTGTTGTAGTCTGGGGCCAA
GTGGAATAGCTGTGTTTAAAACCCTACATTTGGTTCTCAGATGGAGGTGGGCTTTGTGGAAATAGTAATTAAGCTTTCAA
CTCATAAGGGGAAACAGCCTTTGCCAGCTCCAGGTCCGGAGCACAACCTCAATTCGAGCAGGCACATGCACCGGTGAAAA
TGTGACCAAGGCTCCAAACTGCATTACCAGGATTGGGGTGGGGGGGAGGCGGCGGATCTGAGTCTTTCTGATGCCCAAAC
AACACCCTTGGCTTAATGCTACGGGGGTTTTCTTTTCTTTGAGTGTAGCACGGGTCAGCTCTCTGGGGTCTGTTGCTTAT
CAGTCCGAGAGTTTGACAAATGAGGATGGGTTCTCTCAAAGCCTGCGCAATTAAAAGGAGCACATGAAAAAGAGTCTGTG
GTGAGTCTTTATTTGGGAAATGAGCAAGTTGGGCCCTGAAGAGAGCTGGGAACTCCAAGGCTGTCCTTTTGAGTCCTTTT
ACCTTTACGGCAGGACCAAGTCTCGGCCTTTCATCACTCCAGGAAGGGACAGAGGAGGGCAAGAGTCGGGCGCGTCCTTG
GAAGCCCAGGCAGGCCAGGCAGGCGGCGGTCAGGACCGCTCCGCCTCTTGGGGCTCTCCTCTGGGAAGTGTCTCGTGTAG
GATTCCAGAGCATTCCGGCTGCCCGGAGTGTCGCCGCCCGACTCCAGCTGTAGCCCAATTCCGGCCCGCAGCTCGCGCCT
GCAGCTCCCAGAGCAGCAGGAAAAAGGGGCGCCGTGCTTGCTGGGGCCCGAGTGTTGAGAGGAAGGGGGAGAACGCTCCT
GCCAGTGGGAGGGGTCGCAGCCCGCGCTCCCCCTCCCACCCTCCCGCTCCAGGTTAAATGCAGCTGCCGCTTGGGCTCG
CCCTGGCTGTCTGGCATTCCCCACTTAGGGCAACCTGCCCACGCCGCCGCCGTTACCTGGTCCAACTCCCCAGGAGGCTG
GGCCCGTCGCTCTGTAGCCGCACCTCTGAGCTGTCCGGCAGGCTCAGGTGAGTCAAAGGGCCCGGGGGCAACGAGGGGTC
TGCAGGGAAGCCAGCGTAAAACCCGTAGGGGGACAAGTCTTAACAGCCTACGTGTCCACGTCTAACCTGCAGTACCTTTT
```

```
CCGATCCCCCTCTGGCAAGGCCAACAAAATGCAGGTCCTCCTTCCCTAATCTCCCTCCACGCCTGGCACCTTCCCAAAGT
GTCCTTTCCGGATTAGCTCTTGAACTCAGACCTCTTTGGAGCGCACTTTCCTTGGGAATGCACTTTCCCTATCCTAAGGG
CGTCCGAGAAATTTCCTCTTCAATGCAAGGATCCTTATCAGGAGACCTGGGCTCACTCTAATGGGCCACTACTTCAAAGC
AAGCCTTTGAACGATGATGACTCTCTTTGCCCTCCCTAGACCGCCCCCCCCCCCCCGTGGAACAGCGGATTTGGAAAGC
TGAAATTGAGAGGGCAGCTGTTTTGACTTTCTAAGAGTCCTGAGGTGCAAGCGACATCAAGTGCGTGTTGACTTAAGCCA
AGAGGAGCTTAAGGGAGCCCTTAGTCAAGAGCCAACCCTGAGTTTCAAAGGCCCGGTGGAGCTATACTCAGATGTTTGCC
CTGGGGTGCTGGCTCCGCTCTGGCCCACTCTAGAAAAGGCTTGCCTTTCCTCCTACCTCCACCATCAAGCCACTGGCACA
TCTAGCTAGCTTGTCACCTTACCACCACAGACCCTGCCAGAAGGATTTTTGTTCTTCTACCCTCGCTGGAAAGCCAGAAG
GGAGAAGAATCTCGGATAGGAAGCTGGTCTGGAGTCAGAGCCACGCACCGGCCTTGCTCAGAATGGGCTCATTCAAGGAA
CTGGGGGGTTGGGGGAGGGGCAGCCGTGGGTTAAGGGGCGATGGCCTGCTCTGCCAAGAAACCACAGAAAAATGGATAGA
GCAGGCTCAGTGTGCCGTGTGGGGGTGGGGTGGGGAACGGAAGAAAGGCTTAGTGATTCTTAGGTGCAAGGGAACCAGGC
TTCTCTGGAGGAAGAGACAGGGGTGGGGTGTAGGGGGTGAGAGCTAGGTTTAGTTCTGTGCCTGGAGCCCAGCTGGGTA
GTCTTATTGGGCAGCACATTTTCCATCCGTACAGTGTGTGTGTTCCCTTAGTGGTTCTCAAGGGACCTAGTTAGGCTACA
CAGGAAGGAGAGGGCTCACCTGTTGTCTCCCTGGGGTCCATGGCTGCTCATCGAGGCCCAGGCCGCTCTCTTGCTGCCCT
CTGGGCACATCTACCGCCACTGACTTCGATACCGTTCACACATCTAAGCCCCGATCCGATCCTCACACTGGATTTATCCT
GTCCAAGCCTTATCTTCAAGGTCCCGGGACTCCCAGCGGCCTGTTTGCTCTGGGCAGGGACACCTGCTGGGCAGATGTAG
GCTTCCCACGGCAGTAGAAGCCAGACCCAGCAGGCGCCCCCTGGCGGCCTCTCCTAAGAGCAGGCGAGAAACAGACACCG
AGGCGTGTCTTGTGCAGGACTCCACTCTCTGGCCTACCACTAAGAACCCAGACCCCAAGAGCTGCTCTTTGGCGTGTGGC
ACGGGTTTGGGGGAATATGGGGAATGATTTCTGTCCGCAGAAGAAGAGAGTGTGGAGCACCTTAGCTGACTCACACACCA
GATAGACAGGACTCTATCTCGGCCGCAGCCACTTTCCTGCTATAAGGCGTTGGGTAGCAGCATTTAATTTCTTAAAGCCT
ATTTCTGAATAGGGAGAACCGAACCAAAACAGCCTTAAATGTCGGAACTACAAAGAGGGTCAATGCCACAACGGAAAAGC
ACATTTAGCACTATATCTGGTATTTAGGAAGGGGTTCTTTCGGGGACTGGCAAGATGGCTCAGTGGGTAAAGGTGCTTGC
TGTCGGGACTGACCACCGGAGTTCAGTCCTGTTTAATTCTGGGACACACACAGAGGAAGGAGAAAACGGACTTCAGCGAA
TTGCCCCCTGACCTCCACACGTGTGTGCCATAGTGTGTGAGCACACACCATATACATGTATATATATGTAACACACACAC
ACATATATGGTGTGTATGCTATATAATATATATGATGTGTATGATCTGTAATATATATCATATATATGGTGTGTGTGTAT
ATATATATATACACACATATGTATATAATTTTTTAATACAAGCGTTTATCTCCTTTTGAGTTTTATGTATTTTTATCTAC
TTCTTATTAACTTAGTGCCCTGTAGTTCTGTAAAATGCCATTTTTTACAAAGGCTCCTCTGTGAGTTTCAGCAATCCCAG
TGTGGTGACATCAAAAAAATTATCATGAGCCTTTTTGTTTTGTTTTGAGAAAGAATCTCACTATGTAGCCCTGGGTGGCCT
GGAACTCACTATGAAGAACAGACTGGTCCTCAAACTCCCAGAGATCTGCCTGCCTTTGCCTCCCGAGTGTGCAGCTATGC
CTGGCAAAACATTTTGAACATACGCAAAGCCAGAGTGTTCATCCCCTGCAATAAAAGCACCGTCAAAACTGTTGCTTTGC
GACCTTCTTTTCTAGCTGCAGGCTGTGACACTGTGGTTCGTCATCCTTCCAAGCACCATGAGAGGCCCCTGCTGCTGGCC
ACTCTCTCTGTTGCTGCTCTTCCTGCAGTCCTGGGAAACTCAGCTCCAGTCTGCAGGTGAGTTGGATCCCAGCCCTCACC
ATGGGAGGAAGGGAGCAGCAACGTTCTCTATCCACAGTGAGCCAGGGTTGCCAGGAGGGGTTGGCACACTGGTGGAAGGA
TGGGTGTGTGTATGGGGGGGGCGGGCTTTAGAACACAGGCTGGATTCGGGCTTCATCCCTCCCACTTCATCCCTCGGGCG
GGCGCTGTTTCCTCAATTGTAGTATGTGGCATAGTTTTGGGTTCATTCGGTGTCGACTTTTATTGAAGCTTATTGAAACC
ATGGAAAAATCAGGAGACTCCACATTAAAAAACAACCCTATTTGAGAATTTTCTTGAACAAAACCAACCAACCAAACAAA
CAGACCAGGGACCTGCTTGCTGGGACAATCCAAGTTATCAGAGATACAGGAGATGAGATGTTCTAGATATAATCTAATCA
GTTCATTCATTTATGGTGTCTGGCTGGCTCATGTAGGCATTTGAGACAGCGACATCTCTTTGTTCACTCCTCAATACATA
ACACAGACTCAGAACAGCTCCCAGAACTGCATGCGCGCGCGGGGGTGGGGTGGGGGAACCTTGTGTGATTTCCTGGCTCA
GGTCTCCTCAAATGATGGATGAGTAACTGTATCCAAGGCTAATTTGTTAGGTTAAAGACATGCTGGGCACTGTTCCATTC
AGATAACAGCAGGTCTTCCGGCTTTTTCTAAGAGACCAGCTTTAGCTGACCCGTGAGAGTCCTCCTCCATCTTCCCGCAG
GTCCTAGGTGCTATAATGGGCCCCTGGATTTGGTGTTCATGATTGATAGCTCCCGCAGCGTGCGCCCCTTCGAGTTTGAG
ACCATGAGGCAGTTCCTAGTGGGCCTCCTCCGCAGCCTGGACGTGGGTCTGAACGCCACGCGCGTTGGGGTGATCCAGTA
TTCTAGTCAAGTGCAAAGCGTCTTTCCCCTGGGCGCCTTTTCGCGCCGCGAGGACATGGAACGAGCCATCCGCGCGGTGG
TGCCGCTGGCGCAGGGCACCATGACCGGGCTGGCGATCCAGTACGCTATGAACGTGGCCTTCAGCGAGGCCGAAGGCGCG
CGCCCATCGGAGGAGCGAGTGCCGCGCGTCTTGGTCATCGTGACAGACGGGCGACCTCAAGACCGAGTGGCCGAAGTGGC
CGCTCAGGCGCGCGCCCGCGGCATTGAGATCTATGCGGTAGGGGTACAGCGAGCTGACGTGGGCTCTCTTCGCACCATGG
CTTCGCCGCCGCTGGATCAGCATGTCTTCTTAGTGGAGTCCTTCGATCTCATCCAGGAGTTTGGCCTGCAGTTCCAGGGC
CGCCTGTGCGGTGAGTTAGAGTGGCACCCAGCTCAGGCAATGCAGATGCTCATTCTAGTTCGCATCATTAAAGGCGATCC
CTCAATTTTACTCTGCCCTCTGGGTACCAGTTGCCGTGGTCCCTCCTGCAGAGTGGTATCCTCAGCAGCCAGTCATGCTG
TGTCAGAGACTGACCCTGCCACAAGCTGTCACCCCCCCTTCTCCCGCCTCCACACCAAATGTCTTTTAATCAAGAATC
GCTCAAAATTGATAGTATTGTCTGTCAGGAGTCATGCCCCGCCCACCCGAGTATGTTCCTCCAACTTTAAGTCCCTTTTA
AATCTTGTGTGTGTGTGTGTGTGTGTGTGCGCGCGCGCGTGCGCGCCAAGATTTCTTGTAACCTAGACTGTCCTTG
AAGTCGATTTGCAGCTGAGGCTGACCTTAACACCGGATCCCCCTGAATCCAAGCTGTGCAGTGTCCTCTTCCGAGACCCT
TCAAAGGAACTACTTGCATCCTGTTTTTCCTCTAATCTTGTCACCTGGACTGACCCTCTCCAAGATGCAAATCCATATCT
AACCTAAGTCCAACCTTGGGAATGGTATTTGCTTCAGACCCCTACCCCTGCCCTGCTGTCTGCCCTCCCCTTGGATGCTT
CTGCCCATCTGTTCTCTGTTGATTGCCCCTTGTGTGTCCTTAGGGAAGGACCTGTGTGCTGAGTTGGTTCATGGCTGCCA
ACACCTGTGTGTCAACGCCCCAGGAACATTCTACTGCGCCTGCAACTCTGGCTACAAGCTAGCACCAGATAACAAGAATT
GTTTGGGTGAGTGCCACCCCATCCTGCTGTCTCATTGTCTTCTTTGGCTGCCCAGCACCAGGACAACTCTTTGACAAGCT
CTTCAGAAGTAAAAGCACACCCATTTGCTGTATGATCCACACAGGGAGGGAATATCATCTGGGGCCACACAACTCTTTG
GTGGTCCGGAACTTGGAGTCTTGACCTCTCACAGTTAAATGTTCTCTCTGTGGTGCCTCCTTTCTGCAAATCTTTTTTTT
```

```
TTTTTTTTTTTTTTTTTAATCTTCTGAAGAGCTATGGTGCGTGAGGAAGCCTTGCTTAGATGATTAGTTGGGTTTTTTTGG
GGGGTGGGATGGATTATATGGTCCCATCCCTGTGACTATGTTTTCTACCACACGCAGAGTGCCCGTTGTTGGAAAGCCGT
CTCTATACTTTCACAGCAACTAGTTCCACAATTCTAGCTTCAATGATCTGGAAAAGGGGGTCATGTGATCCCAACAGCTC
TGTTGTCACATGGGTCCTCTTGTGTTTAGAAACTTTGAAAGGCTGAGGGGTGGGCTGGAGAAATGGCTCAGTGATGGAGG
GCGCTTGTTACACAGTCATGAAGACTTTGGATCTCAGCGCTCATGAACCAGACAAGCATCTGGCAAAGGCCTGTAACTCC
AGCTCTGAGGTGGGAAGAGAGGGAGATCCCTGGGGCTCGTGGTTCCCATAATAGCTAAGAAACAGGAGGTCCCGGTCCAG
AGAGAGACCCTGTCTCAAAGGAATAGGTAGAGAGTGATGGGGAGGACACGCAACACCTTTTCTGAGTGTGCATACAGTTG
TGCACACCAGCACACGCACACATTAACCAACCAACTAACTATTCAAAGGTCAGCTTAAAAGGTTTAGTATGCACCCCCGT
TAGAATATGATGGATAGTAAGTCTTTTCCTAAGGTATTTGGGAAATGCATTCAGGTAGCTGGGCTTCTGCTTACTTTCTT
TCTCCCTCTTTCCTTTCCTCTTCCCCTTCCTCCATTGTCTTACCCTCCTCCCCTCCCCTGTCTAAAACCTGTCTAAAGCC
CTTCCATGTGTTCTCTTCGCATCAACCATTGCTCTCTCCCTCTCTCTTCCCTGTTGGCTCCTCATAAACTTTAAANNNNN
NNNNNNNNNNNNNNNNCTACTAAGAGCTGGCTTCCTGAGCTCATCTAGTGACGAGTATCTCCTAGAGACCTCCGCTAATCG
ATCTTTTTTCTTCTCCCTTTTTCCTCTTTTTCTTCTAATCTCTCTCCAGGTATTCCTACCTAACCTTAACTTTTCCTCGG
GTTCAAGACCCTTGGAAAGGCCTGTATACTTATTTTGTGTACCATACTTCCTCTTTGTTCCTACTCTCTCTCCCCGCTTT
ACTTCTGATAGATTGTCCTGAATTTCCTCTAGAATTTTCAGCCCTATCTTAATCACTTGATAGCATGTGAAAAAGAACAA
AAGGGCTCCTAACACCAGAAAAAATTCAAGGCCAAACATACTCACTGGTACTCTCGTTCCCCAGCTGAAAAGTTCTGAAT
TCATACAGTTGAATCCTTCTTAACAGTCTGCTTTACGGGAACCTTTATCACCGTCGTTCCCCAGCTGATGAGTTCTGAAT
TCGGCAGTTGAATCCTTCTCAACAGTCTGTGTTACGGGAACCTTTATAAACGTGATCCGCAGTTCTGGTTCTGAAATGAA
GTATCCCTCCTGCGCCAGTCCGGAGTTTTTTCTCGTCCCGGAATTCGGCACCAATTGTTATTCGACGCGTTCTCACGACC
GGCCAGGAAGAACACAACACCCAGAATCTTCTACGGCAAAGCTTTATTGCTTACATCTTTTTGGGGCCAGAGTGTAAGAA
GCAAGAGAGAGAGAAAAACGAAACCCTTCTATTTAAAAGAGAACAACAATTGCCTAGGACGCATCACTCCCTGATTGGC
TGCAGCCCATGGCCGAGCTGACGTTCACGGGAAAAACAGAGTACAAGTAGTCGTAAATACCCTTGGCTCATGCGCAAATT
ATTTGTTTACCAACTTAGAACACAGGATGTCAGCGCCATCTTGTGACGGCGAATGTGGGGCGGCTTCCCACACTTGTCA
CCTGGACTGACCCTCTCCAAGATGCAAATCCATATCTAACCTAAGTCCAACCTTGGGAATGGTATTTGCTTCAGACCCCT
ACCCCTGCCCTGCTGTCTGCCCTCCCCTTGGATGCTTCTGCCCATCTGTTCTCTGTTGATTGCCCCTTGTGTGTCCTTAG
GGAAGGACCTGTGTGCTGAGTTGGTTCATGGCTGCCAACACCTGTGTGTCAACGCCCCAGGAACATTCTACTGCGCCTGC
AACTCTGGCTACAAGCTAGCACCAGATAACAAGAATTGTTTGGGTGAGTGCCACCCCATCCTGCTGTCTCATTGTCTTCT
TTGGCTGCCCAGCACCAGGACAACTCTTTGACAAGCTCTTCAGAAGTAAAAGCACACCCCATTTGCTGTATGATCCACAC
AGGGAGGGAATATCATCTGGGGCCACACAACTCTTTGGTGGTCCGGAACTTGGAGTCTTGACCTCTCACAGTTAAATGTT
CTCTTCTGTGGTGCCTCCTTTCTGCAAATCTTTTTTTTTTTTTTTTTAATTCTTTCTGAAGAGCTATGGTGCGTGAGGA
AGCCTTGCTTAGATGATTNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNCCTCCTTCTTCCTCCTCCTCCTTTCTCCTGCTTACTCCTCCTCCTCCTCCTCCTCCTCCTCCTCCTCC
TTCCTCCTCCTCCTCCTCCTCCACCACCACAACAAAATTCTCTCACTGCATCCAGAGCTTACCGGTTGGCTAACGGGGTT
AGCCAGTGGACTTTGGAGATCTGCGGGTCTCTGCCCATCCCAGGGCTGGGGCTACAGCTACCACTCCTGGCTTTCATAGG
GATGCTGGGGGTCCAAACTCAGGTTCTCATGCTTCTGGAGCAGACACGTTACAGACTGAGCATCTCCCTGGTTCTCACCT
AGGTTTTGTTAGAGAGGGCTTACGCGGGTTGCGTGGCTTCCGCAGGTTGCGTGGCTTCCGCAGGTTGCGTGGCTTCTGGG
GGTTGCGTGGCTTCTGGAGGTTGTGTGGCTTCCGAGGGTTGCGTGGCTTCCGCGGGTTGCGTGGCTTCCGAGGGTTGCGT
GGCTTCTGGGGGTTGCGTGGCTTCTGGAGGTTGTATGGCCTCCGAGGGTTGCGTGGCTTCCGAGGGTTGCATGGCTTCCG
AGGGTTGTGTGGCTTCCGAGGGTTGTGTGGCTTCAGGAGGTTGCGTGGCTTCCGAGGGTTGCGTGGCTTCCGAGGGTTGT
GTGGCTTCCGAGGGTGGTGTGGCTTCCGAGGGTGGTGTGGCTTCCGAGGGTGGAGTGGCTTCCGAGGGTGGTGTGGCTTC
CGAGGGTGGAGTGGCTTCCGAGGGTGGAGTGGCTTCCGAGGGTTGTGTGGCTTCCGAGGGTTGTGTGGCTTCAGGAGGTT
GCGTGGCTTCCGAGGGTTGCGTGGCTTCCGAGGGTGGTGTGGCTTCCGAGGGTGGTGTGGCTTCCGAGGGTGGAGTGGCT
TCCGAGGGTGGAGTGGCTTCCGAGGGTGGTGTGGCTTCCGAGGGTTGTGTGGCTTCCGAGGGTGGTGTGGCTTCCGAGGG
TTGCGTGGCTTCTGAGGGTTGCGTGGCTTCTGAGGGTTGCGTGGCTTCTGGAGGTTGTGTGGCTTCTGAGGGTTGTGTGG
CTTCCGAGGGTTGCGTGGCTTCCGAGGGTGGCGTGGCTTCCGAGGGTGGTGTGGCTTACAAGGGTTGTGTGGCTTCCGAG
GGTTGCTTGGCTTCTGAGGGTTGCGTGGCTTCTGGAGGTTGTGTGGCTTCTGAGGGTTGCGTGGCTTCCGCGGGTTGCGT
GGCTTCCGTGGGTTGCGTGGCTAGGCTGTGAAATGAAGCCCCTAGCAAAGACTGACTTCATCCTCTTTCCAGCCTTGGAT
```

```
CTCTGCGCTGAAGGAACCCATGGCTGTGAACACCTCTGTGTCAACTCCGTGGACTCGTATTTCTGTCGTTGCCGAGCTGG
CTTTGCGCTCCAGCAGGACCAGAGGAGCTGCAGAGGTAAGTGAGCCTCCCACAACCCCCTCTCTGTGAGCGTTCTGAATG
GCCCCTGGTGGCTACACTCAGAAGTGACATCCTGCGGTTTCTTCCTTCCCTCAGCCATTGACTACTGCAGCTTTGGAAAC
CACAGCTGCCAGCATGAGTGTGTGAGCACTTTGGCGGGGCCACAGTGTCGCTGCAGAGAGGGCACGACCTGCTACCTGA
TGGGAGAAGCTGTCGGGGTGAGGAGGTGTCTCTCATATTTGTAGGGAATAGAAAGGGATTCTAGCTGCTGGGATCTAACC
TGACCGGTCCTACTTGGTGTCTCCTTTAAAGTCAGGGACTTCTGCAATGGTGTGGACCATGGCTGTGAGTTCCAGTGTGT
GAGTGAGGGTCTTTCCTTCCACTGCCTGTGCCCTGAGGGGAGGCGACTTCAGGCTGATGGCAAGAGCTGTGACCGTGAGT
TATATATAAGGCAGAGGGTCTGCTCCGGCCCCATTGCCTGCCTCTTCTTCAATGCCCAGTTACAGCCCCACAGATGGTGG
TGTGTATGTGCGTGTGTTGGCGGATGTGCTTATAATGCCCAGCTTCTTTTCCAACCAGTCCTCTATCCTGCTGTCAAGAC
TGATTTCAAAATCCATATCTGATCACGTTCCTCTTTCACTCAAAATACTTTTGTGGTTCACCAGTGTCTACAAAAGCAAG
TGCCTTCCCTCCGAATTTTTCCAGTGCTGGGCACAACCACATTAATGGATAATGAAACCAAATTAGTGGGTTGCAACCAG
CATTTAAAGAAAAGAAGAGAAAAATAGTTTCCTGAGTTTAGGAGAGTAAGTATTGCTTCATGAGCTGGACCGTTCAGTTA
CGTGAAATGCACCGTGGTCTATCATTATTCTCTTTTTTCATCGTGGGCTGTGGTCAATTTTGAAATCATCACTTAATCTC
ATTACCATGTAGCTTTGGCCAGCTCTTTAGCCCATCTCTTGCCACACTTGAACATTTTAGTCCCACTGGCCTGTCCCTCT
CCGAGTGTGTCATACCCTTCCCGGCTCCCATGCCTTTGTGTGTGCTGTGCCAACCTTCTAGAATGCCCCCCTTGCCTTGG
CAAATTCCCGGTCATCCTTCAAGACGGCATTAGCCCATTTCGGTTGCCATGGTGCAACACCGATGTGAGTTGCTATAATG
AAAACGCTCAAAGCGGGGTGATTGGTTAGAGAGTAGAGCTTACGTGGCTCACAGTCTGGAGCATAGCGTCAACATCTCAG
TATTGGCGATGACCACGTGACAGGAGAGAGCCCCTGAGATTGAAGAGTATGTGTGTGTGAGGGTTCAAATGCAGGAGGTG
GCCGTCCTTTGTAACCTTTCACTGACAGGGTCATTCTAGTCTCTCGAGACCTACTCAATCCTGTGATGTGGGCGTTAATT
TCTTCCTAGGGTGGTGCTCCATGACCTAATCGTCTCCCAGAAGGCCTGTTATGATTCAGAGAGAAAGTGCTCTCTTGCAA
GCTCATGCCTCAAAGGCTTGGTCCTTAATGCCAACATGTTCAGAGCCTGGATCTTATTAATTGATTAACCCAAAGATGG
ATTAAACACGAGAGTAGGTTTTTAGGGTGTTGGGAGATGGAACTGTAGAGGATAGGCCCTAGATTGAGAAAGTGGGTCAC
TGGGGGGGTTAATGCCATTTTTTTTTGAGACAGGGTTTTATGTAGCCCAGGCTGGCCATATACTAGCTATGTAACTGATA
ATGACCTTGGGCTTCCTAATCCTACTGCCTCCGAATGTTATGGTAACCCCCTTTTCTGTGGTGTTAGGGCTCTAACTCAG
GGCTTGGTACAAGCTAGGCAACCGCTCTACCAAATGAGCTTCATATCCAGTTCCTGAAGCCTGGGATTTGGATGTGTATT
CCAGGCTGGTCTTGATCCTGCCTGTCTCCCAAGTGCTGAGATTACAGGCTTGTGCCTCCATGTCCTGTTTGAAGAGTGTC
TTGTCCCTGCCTGCCTGTCTGTCTGTCTGTGTTTCCTTCTGTAATGAAGGATGTTGCATTCCTCCATCATTTTCCTGCTG
TCATGATATCTGGCCTCTACCCAGGTCAAAGGCAATGGAGCCAGACAGCCGTGCACTGAAATCTTCCCAAGTCGTGAGCT
AAAGCAAATCCTTCCACCCTTAAGTTACTTCCATCACTGCAATGGAAAGACCCCTAGGTCTCAATCACTTCTCAGTCCTG
TTACCCTGGGGACCAAGTTTCTAGCACATGGTCTCTTGGGCACAAACCATATCTAAGCCACAACAATATCTTAAATGTTT
ATTTCTCAGTTCTGGAAAGGCTAAGACCAAGATTCGGGCAGATACGGTGTCCCATGATGCTCTGCTTCCTGGCTTATACA
AGATAGTTTTCTCTTTGTTCTCCCTTGATGGATGGACGGAGGAAGCAGACTCTGTAGAAGCTCTTGTCAGAACATTAATT
AATTGCATTCATGAAGGCCTGACTCTCATGTCTGTGTCTAATCCCAATTTCGTCCCAAAGATGATCACATTGAGACAGGG
TTTTATGTAGCTCCGACTGGCGGTGTATAGCTATATACTGATAATGACCTTGGGCTTCCTAATTGAACCCCACCACCTCC
ACGAAGCCTTCCTCAACCACTCTCAGGGAAGTGATTTATTCCGGTCTTGGGCTCCCTTACTGCCTCCTCATAGTGCACTT
TCCTTAACTGTGGCAGATGGCATGTCTGCCTTGCTACAGTCTGCCCTGATCTTGTGGGTCAGGACTGTGTTTTCTTTGTT
GTTGAATCCCCTTCTGGTACTCAAGAAGCCACTGGATCAGGACAAAGGAGGCAGTGTCTTAGTGAGCAGGTCCAGTGGTT
TGTGGTTTAGATGCCGCTGCCTTCCAATCTTGGCTTTGCCGCTGTTAACTTCAGAACCTAGTTTATGGGAAATGGGGAGA
GCACTCGAGGAGAAAGTGTGTCAAGTGCGAGTTTCAGCGTCTGACACGTGACAAACACCAGAAAATGTTGTTCTTGCCAT
TGTCATTAATACTGATATGGTGCTTAGAATGGTGACAGATACATACTAGGTGATCGAGGAATGCTAGCTATTGTGGTATA
TTTTTGCTCTTGGTTGTTTAATAACGAACCGGGAGGTGGTGATGTTTGCCTTTAATCTCTGCACTTGGGAAGCAGAAGCA
GGCAGATCTCTGAGTTTGCAACTAGCCTGGTCTGCATAGTGAATTCCAGGTCAGCCAGGACTACACCGAGAAATGCTATC
TTGAAAAAAAAAAAAAAAAAGCCAGGCAGTGGTGGTGCACACCTTTAATCCCAACACTCAGGAGGCAGAGGCAGGCGGAT
TTCTGAGTTTGAGACCAGTCTGGTCTACAGAGTGAGTTCCAGGACAGTCAGGGTTATACAGAGAAACCCTGTCTCGAAAA
ACCAAACAGAGAGGGGGGGGGGGGAGAAGAGAGCTTTCAATAAAGCATTTGGTTAGTGCCAAGCACTTAACATCAAAAGT
TGTCATACAAAAGAGGACGATGGTTATGTATTGATTGCTGGGCACACAGTCAGGACACATAACTGCAATGCGTTATAT
AGTAAATACTGGCGATGAATAACTAGATGAGTATGTTGTTCTCCTGGGCAAGGACCTGATCCCTCCATCCAAGCCTTCTC
CTTGGCAGGGTGCCGGGAGGGCACGTGGATCTTGTTCTCCTGGTGGATGGTTCCAAGAGCGTGCGCCCACAGAACTTCG
AGCTGGTGAAGCGCTTCGTGAACCAGATTGTGGATTTCCTTGACGTGTCTCCCGAGGGCACACGTGTTGGGCTGGTGCAG
TTCTCCAGCCGGGTGCGCACCGAGTTCCCCCTGGGCCGCTATGGCACCGCAGCTGAGGTGAAGCAGGCAGTTTTGGCCGT
GGAGTACATGGAGCGCGGCACCATGACAGGGCTGGCCCTGCGTCACATGGTGGAGCACAGCTTCTCTGAGGCGCAGGGCG
CGCGGCCTCGCGACCTCAACGTGCCTCGCGTGGGCTTGGTGTTCACTGATGGCCGCTCTCAGGATGACATTTCAGTGTGG
GCAGCTCGTGCCAAAGAGGAAGGTAGGTTTGGCAAGGGATGGACTGGTGTGGGGTTGGAGTTGGGGCTGGGAAGCAGTTT
TCTCCAGGCTTGGAGCTTTCTCTTGAGTGTCTGGGCCCTACAGGCATCGTCATGTATGCCGTGGGCGTGGGTAAGGCTGT
GGAGGAAGAGCTGCGTGAGATCGCATCGGAACCATCGGAGTTGCACGTGTCCTATTCTCCGGACTTTAGCACCATGACGC
ACCTGCTGGAGAATCTCAAAGGCAGCATTTGCCCAGGTGAGTGAATCTCTTCCAGCCGACCGGAGTGCATGCGTATGCGC
GCACGCGCGCGCGCGCGCGTGTGTGTGTGTGTGTGTGTGTGTGTGTGTGTGTGTGTGTAGGGATGGGGACGAGA
GGGCAGGGTCTGTTGTCTTTTTTACCTAGCCTCTGATAGATGCCCTGGTCCTCAGCTCTTCACTACCCTTCAGCTCTCTA
CACAGAGCTTGTTATTTAAAACCCAGAGCAGGTCTGTCCTTTACCGTAGCTTGTCAAAAACGCAGCCCCGACAGTGAATC
GGACCCTTACGTGCTTTGTGTGCACCTCAGAGTCAACGAGGCGTTGGGATTTTTTTTTCACTCAAATTAGTAGTGTCTGG
AATAACTTGAGAAAACTGTATTATTAAAAATATGTTTAAAATTGCATTTTAAAAAATTGCACACCTTTTGCCTGCATGTA
```

```
CGTACGGGCCTGGTTCATGTGGAGGCCAGAAGTGGGGGGCAGTTCCTGTGAGACTACAGATGGTTTGTGAGTCGCCGTGT
GAGTGCTGGACATTGAACCTGGGTCCTCTGGAAGAGCAGGCAGTGCTCTTAACCACTCAGCCATCTCTCCAGGTCAAGAG
AAAACTTTTAAAAAAGCAAGTCAAATTTAGCATTTACAAGCGTAACCGACTATAAGCTTCCAGCTTGTGGAGATTCAACT
TTATAGATTTGGCAAAGTGACAGGACAGGGTTGTTTTTTTATTTTTATTTTTTATTTTTATTTTTTTACAGTGGCTTGTG
CTTTCTGGGGACATAGACCATAAATGCCATCATTGTAATGTATCTGGTCTCCAGCTGGGGGTGGGGGGGAGGGGAAGGAT
CCAGACATGCCCGCGGGGAACAGCTGGGCCTTATAGGGGGCAAAGTTTCTTGGTGGGGGGCTTGATTTTGACTATGGGGT
CTTTAGATTCTAACACATGTCTAAGGCGTGACTCTTCTTCCACTGTCTACTGCTGATAGAGGTAGGTGAGCAGGGCTGTG
TCCAGGGCAGAGAATCTGTAGTTAAAAGTGCTCCCTGCCCTGCCACCAACTCTAACAATTAACTAAGGTGTTTTGGACAC
ATCAGGCTTAAGGGCCCTTCCCGTCCCGACTTATTTACAATGGACCCTGGATAACTCACTTAACCCCGGCCCTACCTTAC
CCTCTTCTGTATGAGGAAAGATGCCCAAAGTTACTGCCAGCTTTAGATTTCGGAGGCAAGTTGGAACGCTCTGCTAAAGC
TCTTCACTGGCTGACCATTCAGGCCCAGGCAGATCCTCATCTTTCTGTAACCCTCGGTTCTACAGCTCTAAAGTCAGAGG
TGAACATTTGGGAGTGTTAACGAGGCCATCAATAAAATGACACAAGGATGAGGGTTTGACTGATAGTAGAATCTCCCTAT
GGAGGCTGAGGCTAAGAATCTTGCTGCTATTTTAGTGCCTGCAATATTAAAAAACAAGAGCTGGTGAGATGGCACAGCGT
GTAAAAGCAGCTGCCATGCAAACCCGGCGACCTGAGTTCGATCCTGGAACACATTACCAAAAAGCCCGATGCGATGGCTC
ACATCTGTACCCAGCATCCCTGCAGGGAGATGGGTGGTGCAGACGGAAGCACCTGAAAGCTTGCCTGGATACAAAGAGAG
GCAAAAACTAAAGAGAGACGCTGCCTCAAAAAGGTGTGAGGAGAGAGTTGACTCACAATAGATGTTCTCTGATGTTGACA
TGAATGCTGTGGTCTGCCCTCACACATGTATCATACACATACATAATAAAACATTAAAATAGAAAATGGCGTGGACCAGC
AGTGATGCTATCTCCGTAATATTCCCCACCATTTTCTGTACTGTTAAGGAAACATAGACAACCAAGTTTATCTCTACTCT
ACTCTTAGATGTTGAAGTGTGTGTGTGGAGATAAACTTGGATCCATAATTTTTTGTTTTTGCGTTTGAGACGGGGGTTTCT
CTGTGTAGCTTTGGCTGTCCTCCTGGAACTCACTCTGTAGATCAGACTGGCCTCAAACTCACAGAGATCCTTCCTTCTGC
CTATGCCTCTTGAGCGCTGGGACCAAAGGCATGTACCACCACGCCTGACTTGGACCTATAATTTAAACATTAATTTCATC
AGAATCAGTGGAATCGCATATTGTTGCACTTCATCCTGACTCACTAGGTCTGGGATAGGGTTTGATAATTTGCCATTATG
ACATATTCCTAGGTTATGCTAATACTGCTTGTCCACGGAATGCTGGTTTACTAGAAATACACAGTACAGGGTTGACTTGT
GCCTGGCATTGCTCTAGATATTGTGCCAGTACGGTATTCAGTCGCTACGTGTGTATGATGAATTACAATTGGTCACAATA
ACACAGAATAAAAACTGAGTTCTTCAGTTGTACCCGCCACATTTCAAGTGTCTTAAGCTCCAGGTGGCTTTTGGGTATTG
AATCGGCCAGGACAGACAGATAGAGAACATGTCCATTGTTTGTCGTGGTCTCATTTGGACCAGCTCATCCATCTAGACTT
TTTCTAATCAAAGTGAAACCCATGGACTTCAGAATTAATGTGTTTCAGGAGCTTAGTGAAAGTGCAGATTTTTACTCTGA
GTCGAAATCTGGATTCTAGCAAGCTCTGTGGTAATCCCAGCAAGCATAAGGGTTGGAGAATTTCTCTTGCAAGTGGGTCC
ACATATGCAAATCGTCTAATCTCATAACCACTAGTGGGTAGCTTCTTTCATTATATCCATTTTACGGATGCGGGGGAATT
GAGACTTGAGGGGTTGAGTTTCCCCCAGGACTTCTGTGCTATTTGGCTGGGACTCAGGCTAGGTCTAAATCCAAAGCCTG
CATGTACACCATCGACTCGGTAGAGGTGTATCAGAAGGTCGAAGCCACAGCCTGGTGGGCTCCCTTTTGGTTCCTCATTC
CTTTGACTCTTGGCTCTCTCCTTGCAGAGGAGGGCATTGGCGCGGGGACAGAGCTTCGGAGTCCCTGCGAATGCGAAAGC
CTCGTGGAGTTCCAGGGCCGCACGCTGGGGGCGCTCGAGAGCCTGACGCAGAACCATATCCTTTTGGGCTGGAGGTGCGG
GTTGCAGGGCGGGTGAAGGGATCTCTGAGACCACATCTTCCTTAACCGCTCACTGGCTCGGCTGACAGAGCGCCTGGAGG
AGCTGGAGAACCAGCTGGCTAGCCGAAAGTGAGGGTCGCACGCTGTCTGAGCAGCCACGCGAGACCGAGGCCGAGCCCCG
CAGCCTGGACCGTCCAGGAGGGGGCGCTGGCGGGCCCCACGCGCTGCCTTCGAGCTGGCCTCACCTGGACCAGGAGCCAG
ATTTAGGGGATGGGGATGAGGGGATGGGGAGCGTGCTGGTGGGGAAGGACTGCTACACTCGCCGCGAGCTTTGTTGGATTT
CTTTTTTTGTTTTCCTTTTCTAAAAAAAAAAAAAAAAAATTTGGTTTTCACGGGGTCGGTTTGTGGCTATTGCTATGCTG
GCAGAGGGAGGGGAGTTCAGACGAGCGTGCTCATAGGCGCCCGGGTGGCGTCCTCTTGGAGAAAGGCGCATTGTGCAGTA
CTGGGGCTGGGGGGCAGGGGGCCGCTGCTTCCCGTCTCTTGTTTATTCAGCCCCAGAACAGATGGCCCGTTATGTAGGAC
GTCGGGAGGGAGGGGATGCCCCGAGAACCTTTGGGTCCGAGGAAGTCTGAGTCCCCATCTTCTAGCACGTCGTTTTCTT
GCTGTTCCTGTCTTCTGGAGCCAAAAAATGTGGCTCAAAGGCCGCTTGGGACCTGGCTGGGGATGTGAGAAACTCTGTTG
AGCCCCTGACCAGAAAGAGTGGGGGATGCGGGATGTGCTCCACCTGGATTGTATCATCTGCCTCTGACACTTGGTGGCAT
CCCTGAGCCTGTGGCTCTCTTCTGCCACTCCTTCAAGAAGCAAAGCTGAGCTGCATGCACACAACCCTCTCTACCAGCAA
GGAACACGAACTTCCCGGGTACACACGGCAAACTAAAGCTTAAAATACGTTGGGTTCCAGAATGGGCGTCAGGTAAATTC
CCCCGGGTTTGTTTTGCCGTTGTTAAAGCTGGGACAAGGGTTAACTGGTACAAGCAGGACTGTGGCTAGAGGAATGTATG
CTAAGGTACCTGACTTTGTGCCCGACATGTATTTTAGGTCCTTTGTTAATGATAGTTAGCAATTATTCTTATAGAAATAT
GTCTTGGACCTGGTTTGAAGCGATTTCCTAATATTGCCATTGTATCCGTTTTTATCCAGACAACCTCGTGAGATAATTA
GGATCTTAGAAGGCAAGATGGCTCAGCTGGCAAGCACATTTACACATTAAGATTTACAATCTGCTTTCAAACATCAGGGT
CCTCATGGTAAAAGAGAGAACTCCTCCTGTAGGATGCTCTCTGACTTCCACCTGCCACCTGTTCCTACTTCATCAGAATG
GTCAGAAACGGGGCGTGGTCAGAACGGGGTGAAAGGGAGGGGCCTGGACTCGATACCAGGTGACAGAAGGCTGGATACCT
TAGAGGGAGGGGAAGGGTATGACTGACCTGAGCCTCGTGTTCTCTAAAGCACATTCCAGAAGGGCCGGAGGAGAGAGGG
GAGTGGAGGGCAGTTGCTTGTGAATCAAGGAATAACCACGATGATGAAAATGGTCCTAATAATGTAAAAATAAATGCTAA
AGATAATTATTGCCTTCATCATTTCCATTTTAGACAAGAGGAAATGGAGTCATGTAGTTTCCCAAAACGGCACGTCTAGT
CAGAGGCTGGACTGACATGCTATATTTCTGATTGCTGCTGTAACAAACGAGAGCTTCGAAGGCAAAATCAGTTCCCTGTC
TCACAGTTCCCGGAGTCAGAAGTCCAAGATGGGTTGGCAGGGTTGCATTCTTTCTGGAGAAATCTGCTTTCCTTTGTAGC
TTCTAGAGGCCTCCCAAGTCCCTTGGCTCATGGTCTCACTGCCCTCTGATCTTAACACCTCTCTCTCTCTGTGTGATT
CTCTCTTTCTCTGTGATTATATTGTTATGACTTTGACAATCACAGACATGCTTCTACTTCAGAAAAACTCCCAGAAGTCT
CCTTTGTCATGTAGAGTCATAGATTTATTCATAGGTTCCCATGATTCAGGCATGGAAGTTGTTGAGCGGAACAGGGCCGA
GGCTAGGCTGGCTGATGTCTGTACTGCTAAGGCTGGATAGGGAGTGAGGTCCACCTGTGTACAACTGTATAGAAAAGGGG
AATGTCTGATGAAGGATAGTGCAGGCAAGAAAAAAAAAGGGGGTGGTGGTGGTGGGGAGCTTTGAGCAAAACCTTAAAT
```

GATATGGCAATACTTTCTGTGATTGGTCATGTGGGTCAGGAGCGGCTGGGAACAGGAGCTGAGATGATCAGGACCTTACT
TAGATATCATGATCAGATAAAGTAAGCAGGCCGGGGTAGTGGCTGGCATGAGAGCTCACCTTGCCCAATCCCTCAAGTTT
AAAGTTAAGGAAAGTCCTTCCTAGGGGCCACAGGTTCTTGTCTGAGAGCACACCGTGAGTAAGCAGAGGGGCAGATAGA
AGACTTGCGTGGATGCGGGACTGCAATAGGGCACTTCCTGTTGGGGAACTGTGAGGGCTGGTGGAAGCTAGAGCATCCAT
CTTTCCTGGATGCAACCAACACCTACCTGCTTCCTGTCCCTACCATTTCTTTACGTCTGCATTCTCCCTTGTGGCCACAG
GATGGCGCCGGAAGCTCTGCTTTAGACCTGTATCTCAGCACCCTAGCAGGTGCCCCCCTGCCCCCTGGAGCTTGGGCTAA
TGATGGTCCTTCCTTTTAACACCAAGGGTTTTGAACTGCAAAGAGGTCCGTGGGCCAGGGAGGAAGTCTCGGGTAACTAA
GATGGAAGACATCCTTCACACTTGGTCCATTTCCCCGAATCAAGCACACTTTCTCAGGGTTGGATCTTACCATTTTATTT
TGGACTGGAGGGCACCATCGATTCAACCTCTGTTAACTTGCGGGTCTGATGGTAAATGTTCCCCTTTCCTGCGAGCATTT
AAAGTTTATGAGGAGCCAACAGGGAAGAGAGAGGGAGAGAGCAATGGTTGATGCGAAGAGAACACATGGAAGGGCTTTAG
ACAGGTTTTAGACAGCCAAGGGGGTAGGAGCGTTGTGTGACCACGTGATTCTCAAGGCAACACTGTGAGGGGTTTGGAGC
ATAGATCCTTTCACTATCAGAGGAAGCCACTGGTGAGAGAGGGGAAGACAGATGTCCCAAATCACTCACCAAGCTCGTAA
TGTCCTGGCCCCTTCCTAACTCATCAGAATGGTCAGAAATGGGGCGTGGCCAGAACGGGACGAAAGGGAGGGGCCTGGAC
TCAATACCAGGTGACAGAAGGCTGTATACCTTAGAGGAGAGGAGGGGAAGGGTTTGATTGACAGGAGCCTCGTGTTCTCT
AAAGCCACATTCCAGAAGGGCAGGAAGAGAGAGGGAGTGGAGGACAGTTGGTGTGTGAATCAAAGAATAGCCACGATGAT
GAAAATGGTCCTAATAGTCACCGTGACTGGCGTTTATGGTGATTCACAAGGAAGTTTCCCATCACTTTGCAGCTCAGCAG
AGCCACGTGCCACCCAAGGGGGATTACAGGGCAGTGCGGTGGCTGCTATTTTACTGAAGGGAGACACGGGCTACAGAGGG
GAGGGTGCCTCGTTCAAGATTATACGTCCAATTACTGCCAGCGCTGGGCTGGACAGAGACCTCCTGGCTCCCAGCCACAC
ATGGGCTGCCCCACATGGGCTCCAGCTGTGGCCAGCCAGAAAGAATGGGGCCTTTGTCAGAGCTGAGGATCAGGCACAAG
CCTCCCGCAGCCTTGGCTTGAGGCCCAAGGAGATGGTTAGGAGAGGAGGCTTGGGGTGTGGGGTTGGGGGTGGGGTGGGG
TGGGGGCGTAGAAGGTTCTAGTTAGACTGAGGAGCTGATCCTCAAATGACCTGTATATGCCCTTCAGCTTCCCTTCACAC
TTCCTGCTAGCCATCCTGACTTCGAGTCGAGTCCGCGGCTTTGTCCTGGACAATCTCTCTTTCCTTTGTATTCCCTTCCT
CCCTCCCCTCTCTCAACAAAGACCTTCACTCCTGCCTTACTTAGTAACTTGACCCAAACTTTTCAGTCCTCCTGCTCAAA
GAAAAGGAGCCCCTTCTTTGTGAGTAATGTCCACTGAGTTCCTACTGTGTGCCGGGCTTCTGCCAGGCACTTTTATCTTT
TTTTTTTTTTTTTTTTAAGATTTATTTATTACATGTAAGTACACTGTAGCTGTCTTCAGACACACCAGAAGAGGGCATCA
GATCACATTACAGATGGTTGTGAGCCACCATGTGGTTGCTGGGATTTGAACTCTGGACCTTCAGAAGAGCAGTCCGGTGC
TTTTACCCACTGAGCCATCTCACCAGCCCCCCAGGAACTTTTATCTTTGTGGTCTCTACCACACCTTCACCCTTTTGTGA
TGGATTTTATTTTCTCTGTTACCTTCCTATCCCGCGTTCAAAATGGATTGTGATTCAGGGTGGATATTTCTTTCCAGATT
TCATTAGTGTGTTGTGACTTTCAAGGAAGGTCATGGCAGGATGGTGGTAGTGACTATCCCTGGAATTTTGCTGTCTTTAA
CCAGACATTCTTATATTAAAAAAAAGAAAACTAACAATAAAAATCCTACTATGCAGAATATGCTTGTGTTGCTCTTGATG
AGCCTGCGTCTGGGCCCAGGGTGTCCTTGCCAAGCAAAATAGAATCCCTACTGTGTTCCATCCTTCTGTCCTGCTTACGC
ATTATCTCATATGTGGGAAATGCACAGTGGATTAGAGAGTAGAGAGGAAAGGACAGAGAATAAACAAATACCACCTTTAA
AATATCCGAGGTGAGTTCAATGCCAATACTGATAGATAAAAACTAGAGCAAGACCCTAGTTACATTCTCTGGTAGACGCC
AGCCACAAGCCTCCTCTTCCAGGAAGTGTCCCATCTGTCCCGAAGGACCTGGTTGTCTCCTGGAGAAGAGGACTCATGGG
TCCTTGGATCTCTCTGGTGTCTGATGGTCTATGTACCCATTGAACTTTCTTATGCTCTGCAGCTTAGAGAGGGGTGGCCT
CCAGACCCCAGAAATAAAACCTATAATTAACCAGGAATACTGTCTGCATCTCTATAGTTCAAAGATAAATAGTGCTATCT
CAAGCTTCATAATTCCAATCATTGGAGGACATTTTATAAGACTTTGGTACACTTCCATATTACTCTAGGAGTACAGCT
GTATTTTTTCCCAACTTGGGTTTTAAGTAATTTCAAGGAGGACATGTTTCTCATAGCAAGAACAAAGTTTTAAGGCCAGG
TCTACACGAGCTTGAGTTACTTCTCTGCCACTTTTTTTTTTTTTTTAGCTGTATGATGTTAGGGGAAAAAAAGGTCTC
TCTTAACCCTTTGAACTTTAACTTCTCTGTGAGATAATGACAGCACATCCCCAATTGCAAATCCCAAGCCTGAGTGCAGG
GATTCAACGTGAGCATGCACAAGAGGCACGGTTCACAGGACGTCAGTAATTACAGGGATGACAGCAAACAGTCATTTGCG
TGCTTGCTGTGTGCCAGGCCCTAGACATCCATCATTGTCGTTAATCTCAGAATTGGCTCACAGAGTAGATGTTGTAACGC
TCCCCATTGCACATCCGGGAAGGGGCAAAATTAGCACCGAGCTCATCCCTTGGTGCTGTGCCTGTGAATGGCAGCAGGGT
AGAAAAGAGGCCAGGAACTTGAGGGGTCTCTCTCTGTGTCTCTGTATCTCTTTGTCTCTGTGTCTGTCTCTCTCTGTCTC
TTTGTGTCTGTGTCTCTCTGTCTCTGTCTCTGTCTCTCTCCCTCTCTCCCTGACAAGGTTTTGCTCAGGCTCCATTGGAG
CTTGTTCTCCTTTTGCCTCTGTATCCTGAGCACTGGATTACAGCCAAGTATACACCACTCTCCCAGCCTTGGAGAGCC
TTACAGAGAGATTTCCTGAGACCCGGGAAAGAGTCAAGAAAGCATGACCAGCTCTAGCTTGTAAACTGGGACAAAGGAGG
AAGCTGGACAGACCTCTGCTTCTGAGATAAAACTAGAAGGTGTGCATCTTGTGGGTGTAGGAGACCACGGAGGCAGGGA
GAGCACAGCAAGGAAGCTTTGTTCCCCCACCCCACCCCCGACTCCATCCCAATCCCAAAGACAGGGCTTCTCTGTATAA
CTCCAGCTATCCCAGAGCTCACTCTGTAGACGAGGCTGAACTTGAATTCACAGAGATCTGCCTGTCCCAGTGCTGGGACT
GAAGGCATGGACCACTATGGTGCTCTTATGAGGAAGAGTATAGGGAATAGGAGTTAGGTGGCAAAGAACTAGTTCAGTGG
GTTGGAGACTAAAGCGATGGCAGTCATGCACCTGGGAAGGACTGTAGGCCAAGCTGTGCTAGATCGTCATATTAAGAAAT
GGATGTGAAAGCAGAACCTTCTCCCCTCCATCTTGGGAATCATTTAATTGAGGAAATTACAGGTGCTTCAAATTCAGTTT
CACAGTCACATCTAAGGGTAGCACAAGCCAGGTTTCCCAGTGGATTACACAGGGCATCTGAAAGTAAATGAGTCAAAGCA
CATCAAGGTTTTTGGTCTGATTGACTCAGGATGATGTCATCGTCAGAGACAGGAAAAGGTAAGGCAGCCCAGGGATTCAG
TATTAGAGCTATTAAACGGGAGACGTGTAGGAAGCACTCATGTTGAGAAATCAGAAGTCAGTTGGATTTGTGAGCTGGAA
GTCAGGAGAGAGACTGGGGAAACAAGCCATCAGTGTGTAGCTGATACAGAGGCTGCACAGTGGATGAGATCAGATGGGAA
GCAAATGTGTGGATCCAGAAGAACCAAACCTATGCTTGCCCTCCTGGCTGCCCTGGATGCAGAGACTGGGGCGGAGAGGA
GCAATTAGCGGGAAAATCTGAGGAGCATCTGGAGACTGGAGAGGAAGGGGTCAAACCATGACGTTATGGTGTCCTAGAAA
CAACTGTGAAGGAAACTGGTGAAGGAAGACTGGGGGACTAGCGACAGTAACTGAGCATGGCGTTTAGCAACCCAAAAGTC
AGTAGTGACTTGGCAGGGACACAATGGAGGGATGTGAAGTTGGTCATCCTAGGAGGCATAAAAGAGAGAGGATGGGAGGA

```
AAAGAATTAGAGATTTTATACACACACACACATATGTATTTGTGTGTAAATATATAAGACATTGGTATATATATTATA
TACATTATAGACAGATTATATATGATATATAATATATGTATTTTATATAGGGAAGGTATATACAACATAGAGATTTTTAT
GATATATATATTATATGTAATATATGCATAGTTATATGATATATTATATGTAATATATGGAGAGATTATATATGGTATAT
ATGCTAAATACTATATATATTATATAGAGAGAATTTTTTTTTTTTTTGTTAATCAAGACATAGTCTCAATATATAGCTCT
GCTTGGCCCAGAACTCTTTTGTAGATTTTGGCTTAAAGAGATCCACCTATCTCTGCCAACTGAATGCTGGGATTAATCAC
CAGGTTCGGTGAGTATGGGTGGAGTTTTTTTTTTTTAAATAATTGTGTATTTGTTGAGCTTATAGTATAATTGCATCATC
TCCCATTATCCTTTTCTCATGCCAAAACCTTTCGTGTATCCATTTTCTGCAAATAAGCAATACCCTACCTGTCCTTGGCT
CGGAAGTTTAACTGTAGAGATAAACTTAACAAATGTGTTAGGGAGGTGAAGAGATGGCTGTTCTTCCAGAAGCCCAGAGT
TCAGTTCCCAGCGCCCACAAGGGGGCTCACAGCCAGCGGGGATTCCTGTCCCAGCAGATGTGATGTGATGCTCTCTGCTG
GCCACCTCCAGTACTGCATGACTATGGTACACAGACATACGTGTAGACAAAACACTCACAGTCAATATTTTTGTTAAAGA
ATAGTTTTAATCTATAGAACGCCTTGAGAACTGTGGAGGCAGCATGCGGTCTTCATCCTCTGAGCGTCTCTCAACGCA
GATGCACCTGTGTGATGAATTGTTAGAGCTCCAGAGTATTTTTTTTTAACATTTCCTCGTTTTCCCCCAATATTATATG
AGTCTATCCAGGACTTATTCCTGTGTCTCCTTAGACTCTTCCAGAATGTTTCCATGACCTTCCTTTTCTTCCTGATAGCC
TTGGTCACTTAAAATTTACATTTATTTATTTATTTATTTATTTATTTATTTATTTATTGTGTGTGTCTGTGGGGGTACAT
GTATTCTAGGGTGCACAAGTAGAGGTCAGAGGACAGTTTGTGGGAATCCATTCTCTCTTTCATTTCCACTGTGTGAGCTC
CAGGGCTTAAACTCAGGTTATCAAGTTTGTTTGTTTGTTTGTTTGTTTGTTGAGACAGGGTTTCTCTGTGTAGCCC
TGGCTGTCCTGGAACTCACTCTGTAGACCAGGCTGGCCTCGAACTCAGAAATCCGCCTGCCTCTGCCTCCCAAGTGCTGG
GATTAAAAGTGTGTGCTACCATGCTGGGTTATGTCATCAAGTCTTGTTGGCAAGTGTCTTCATCCACGGAGCTATCTCTC
TGGCCTGACTTTGATAATTTTGGAGAGTGTAAGTTAGGTGCTTTCTAGGTTATTTGTTAACTGGGGTCCACATAATGTTT
TTCATGGTTAAAAATGGGATATAAATTTTTGCGAGGTAGACCAGGGGCGGTGAATGCACTATTACAAGAGGTAGCTTGAA
AACTTTGTGCTAGGTGGTACTGGGGCTAAAGGACAAGTGTTAGGAGAAGTTTGAGACCCCGTACAACACTCTAGTCACAC
TAACTCTACCTGAGGGGCATCCAAGTGATGTGGAAGAGCACTGATCTCCACAAACCAAGGAAGGAAAGGGAAGAAGAGGT
AAACCTGGGCTTCGATGGGCTTCCTCTTAGAGCAGTGTCCAGCCTTTGCTGACACCGGCTCTGGGTACAGGGGTGAGTCT
TCAACTTTTTCTGTTTTCCACTTTGTTCTTATTGGCCTCTTGAGGTGTTTATTATATTCCAGGAACCAAGGTCACCAACG
GAACAGAGAGAACACAACACTTACGCAGGGGTGTGCAGTTTCAAAGCAGTGAGATCAAGCCGGGGTAGTCTGTCTTCACA
GAAGATCAGCGTGCCTCAGAGTGTCCATTGTGGTCTCCGATCTGCAGATGTATTAAAGGGAGGTGGGAAAAGGGGAAGGG
ACTTGTTTGGGGACACAACTGGCAAGTGACAAAGTAAGACCTAGACTTGAGGCAAGTGTAAATCAAGCTTAGAGTCCTTG
CCACCACTGGAAAGATGCTCAAGTCTGCCATACATATTTACTAGTGTGTGCTAGGGAGTATCTACTTGGTGAGTGTTCTC
AGCCCTCAGGTATGAGGCCTGGAGTCTGTTCTCTGTCCTTCCTGCTATTAATGCACACAGACAAACCAGTTAGTCACTGA
GAACGACACTAAGTCCCTTTCTGACATACGGATGAGAGTGAGCTTATTACTGTATTTTTCTTGGAAAATGGTTTTTCCAT
AAGCATTAAGAAACTTACATCTTGGGCTAGAGCGATGGCTCAGTGGTTAAGAACACTGACTGCTCTTCCAGAGGTTCCAA
GTTCAATTCCCAGCAACCACATGGTGGCTCACAACCATCTGCGATGGGATCCAAGTCCCTCTTCTGGTGTGTTTGAAGAC
AGCTATAGTGTACTCATGTACACAAAATAAATAGATAAATTAAGAAAAGAGAAAGAAACTTACATCTCAGCCAGGCAGTG
GTGGTGCACGCCTTTAATCCCAGCACTTGGGAGGCAGAGGCAGGTGGATCTCTGTGAGTTTGAGGTCAGCCTGGTCTACA
GAGTGAGTGCCAGGATAGCCAAGGCTACACAGAGAAATCCTGTTTTGAAATAACAAAACCAAAAGAGAAAAACAAAACAG
AAAAGTAAGAAAAAAATCTTTACATTTTTCATTTCTGAACACATCTACTCTGGGAACTATGGGCTAATGAATAAATATAA
AACAGAGGAGATGTTTGTATTTTAACAATATATGAAGAACAGCTACAAGTCAATCATGTGAAGATAAAACAGACACACAG
CTCAAGACTAACTAAGGACTATATAGGAAGGGCCTATACAGGAGGAGAAAATATCATTGATCATCAGAAAACACAAATTC
AATCCACAATAAGATATCGCTTCATGCCCACTGGAGAGTTTAAGATGTAACACTGTGAAGTGTCCATACGACTGTGGAAA
ACATTTCCTTTTTATGCAGTTATGGGGATAAAATCCAGGCCTCACACATGTGTGGCAAGCACACTGAGCCCTGCAAGTG
GGCCCTGATCCTCCTGCCTCA


MOUSE mRNA SEQUENCE : mR3-010.1 (Seq ID No: 8)
GGCCCGTCGCTCTGTAGCCGCACCTCTGAGCTGTCCGGCAGGCTCAGCTGCAGGCTGTGACACTGTGGTTCGTCATCCTT
CCAAGCACCATGAGAGGCCCCTGCTGCTGGCCACTCTCTCTGTTGCTGCTCTTCCTGCAGTCCTGGGAAACTCAGCTCCA
GTCTGCAGGTCCTAGGTGCTATAATGGGCCCCTGGATTTGGTGTTCATGATTGATAGCTCCCGCAGCGTGCGCCCCTTCG
AGTTTGAGACCATGAGGCAGTTCCTAGTGGGCCTCCTCCGCAGCCTGGACGTGGGTCTGAACGCCACGCGCGTTGGGGTG
ATCCAGTATTCTAGTCAAGTGCAAAGCGTCTTTCCCCTGGGCGCCTTTTCGCGCCGCGAGGACATGGAACGAGCCATCCG
CGCGGTGGTGCCGCTGGCGCAGGGCACCATGACCGGGCTGGCGATCCAGTACGCTATGAACGTGGCCTTCAGCGAGGCCG
AAGGCGCGCGCCCATCGGAGGAGCGAGTGCCGCGCGTCTTGGTCATCGTGACAGACGGGCGACCTCAAGACCGAGTGGCC
GAAGTGGCCGCTCAGGCGCGCGCCCGCGGCATTGAGATCTATGCGGTAGGGGTACAGCGAGCTGACGTGGGCTCTCTTCG
CACCATGGCTTCGCCGCCGCTGGATCAGCATGTCTTCTTAGTGGAGTCCTTCGATCTCATCCAGGAGTTTGGCCTGCAGT
TCCAGGGCCGCCTGTGCGGGAAGGACCTGTGTGCTGAGTTGGTTCATGGCTGCCAACACCTGTGTGTCAACGCCCCAGGA
ACATTCTACTGCGCCTGCAACTCTGGCTACAAGCTAGCACCAGATAACAAGAATTGTTTGGCCTTGGATCTCTGCGCTGA
AGGAACCCATGGCTGTGAACACCTCTGTGTCAACTCCGTGGACTCGTATTCTGTCGTTGCCGAGCTGGCTTTGCGCTCC
AGCAGGACCAGAGGAGCTGCAGAGCCATTGACTACTGCAGCTTTGGAAACCACAGCTGCCAGCATGAGTGTGTGAGCACT
TTGGCGGGGCCACAGTGTCGCTGCAGAGAGGGCCACGACCTGCTACCTGATGGGAGAAGCTGTCGGGTCAGGGACTTCTG
CAATGGTGTGGACCATGGCTGTGAGTTCCAGTGTGTGAGTGAGGGTCTTTCCTTCCACTGCCTGTGCCCTGAGGGGAGGC
GACTTCAGGCTGATGGCAAGAGCTGTGACCGGTGCCGGGAGGGCCACGTGGATCTTGTTCTCCTGGTGGATGGTTCCAAG
AGCGTGCGCCCACAGAACTTCGAGCTGGTGAAGCGCTTCGTGAACCAGATTGTGGATTTCCTTGACGTGTCTCCCGAGGG
CACACGTGTTGGGCTGGTGCAGTTCTCCAGCCGGGTGCGCACCGAGTTCCCCCTGGGCCGCTATGCACCGCAGCTGAGG
```

TGAAGCAGGCAGTTTTGGCCGTGGAGTACATGGAGCGCGGCACCATGACAGGGCTGGCCCTGCGTCACATGGTGGAGCAC
AGCTTCTCTGAGGCGCAGGGCGCGCGGCCTCGCGACCTCAACGTGCCTCGCGTGGGCTTGGTGTTCACTGATGGCCGCTC
TCAGGATGACATTTCAGTGTGGGCAGCTCGTGCCAAAGAGGAAGGCATCGTCATGTATGCCGTGGGCGTGGGTAAGGCTG
TGGAGGAAGAGCTGCGTGAGATCGCATCGGAACCATCGGAGTTGCACGTGTCCTATTCTCCGGACTTTAGCACCATGACG
CACCTGCTGGAGAATCTCAAAGGCAGCATTTGCCCAGAGGAGGGCATTGGCGCGGGGACAGAGCTTCGGAGTCCCTGCGA
ATGCGAAAGCCTCGTGGAGTTCCAGGGCCGCACGCTGGGGGCGCTCGAGAGCCTGACGCAGAACCTGGCTCGGCTGACAG
AGCGCCTGGAGGAGCTGGAGAACCAGCTGGCTAGCCGAAAGTGAGGGTCGCACGCTGTCTGAGCAGCCACGCGAGACCGA
GGCCGAGCCCCGCAGCCTGGACCGTCCAGGAGGGGGCGCTGGCGGGCCCCACGCGCTGCCTTCGAGCTGGCCTCACCTGG
ACCAGGAGCCAGATTTAGGGGATGGGGATGAGGGGATGGGGAGGTGCTGGTGGGGAAGGACTGCTACACTCGCCGCGAGC
TTTGTTGGATTTCTTTTTTTGTTTTCCTTTTCT

MOUSE PROTEIN SEQUENCE : mP3-010.1 (Seq ID No: 9)
AVRQAQLQAVTLWFVILPSTMRGPCCWPLSLLLLFLQSWETQLQSAGPRCYNGPLDLVFMIDSSRSVRPFEFETMRQFLV
GLLRSLDVGLNATRVGVIQYSSQVQSVFPLGAFSRREDMERAIRAVVPLAQGTMTGLAIQYAMNVAFSEAEGARPSEERV
PRVLVIVTDGRPQDRVAEVAAQARARGIEIYAVGVQRADVGSLRTMASPPLDQHVFLVESFDLIQEFGLQFQGRLCGKDL
CAELVHGCQHLCVNAPGTFYCACNSGYKLAPDNKNCLALDLCAEGTHGCEHLCVNSVDSYFCRCRAGFALQQDQRSCRAI
DYCSFGNHSCQHECVSTLAGPQCRCREGHDLLPDGRSCRVRDFCNGVDHGCEFQCVSEGLSFHCLCPEGRRLQADGKSCD
RCREGHVDLVLLVDGSKSVRPQNFELVKRFVNQIVDFLDVSPEGTRVGLVQFSSRVRTEFPLGRYGTAAEVKQAVLAVEY
MERGTMTGLALRHMVEHSFSEAQGARPRDLNVPRVGLVFTDGRSQDDISVWAARAKEEGIVMYAVGVGKAVEEELREIAS
EPSELHVSYSPDFSTMTHLLENLKGSICPEEGIGAGTELRSPCECESLVEFQGRTLGALESLTQNLARLTERLEELENQL
ASRK*

MOUSE PANTHER CLASSIFICATIONS
         FAMILY (SUBFAMILY)
                 COLLAGEN/INTEGRIN-RELATED(MATRILIN-4)
         BIOLOGICAL PROCESS
                 Biological process unclassified(2.99.00.00.00)
         MOLECULAR FUNCTIONS
                 Extracellular    matrix(1.27.00.00.00)    >    Extracellular    matrix
structural protein(1.27.01.00.00)

MOUSE GENE ONTOLOGY
         BIOLOGICAL PROCESS
                 cell adhesion > cell-cell matrix adhesion
                 cell communication > cell adhesion
                 skeletal development > cartilage condensation
                 mesoderm development > skeletal development
                 ectoderm development > neurogenesis
         MOLECULAR FUNCTION
                 ligand binding or carrier > calcium binding
                 protein binding > collagen binding
                 transmembrane receptor > cell adhesion receptor
                 cell adhesion > cell adhesion receptor
                 ligand binding or carrier > protein binding
                 defense/immunity protein > blood coagulation factor
         CELL COMPONENT
                 extracellular > extracellular matrix
                 GO cellular component > extracellular
                 extracellular > extracellular space
                 fibrillar collagen > collagen type III
                 integral plasma membrane protein > integrin
MOUSE PROTEIN DOMAINS (INTERPRO SIGNATURES)
                 IPR002035 (VWFADOMAIN)
                 IPR000561 (EGF 2)
                 IPR001881 (EGF CA)
                 IPR002035 (VWA)
                 IPR000561 (EGF)
                 IPR000561 (EGF)
                 IPR002035 (vwa)
                 IPR002035 (VWFA 2)
                 NULL (CYS RICH)

IPR000152 (ASX HYDROXYL)


HUMAN GENOMIC SEQUENCE : hD3-010 (Seq ID No: 10)
ATTTCCTCTCCTCTGCGTATTGTTCGTTTCTTTTTTCTCATGGGACTTTCCATACCCTGGGGGAGGAGTTTTACAAGAAA
TGAGCTCTTCCTTAGGAGGGCAAGCTCTTCTCCAGAGGGTTAGAACCTAGAACCCACATTTCAGACCAGTTTTCCCATCA
GGCACCACCAGAGGCTGGTGCAGGTAGCTGGCACCCATGATACTTATCTGTGTCCTTGATTCCAATAGCCCAAGCCAGTG
TTCTGCCTGGTTTTTTTTTCCTTCCCTATAAAGCACAGACTGGGTAGAGTGGTACCTAGGGTGGGTAGGCTGGTGGGGAA
TAGATTGTGATTCTGTCCATCAGCCCCATGCAGAGAGGCCCCCCTCTCAACTACATTACTGCCCAGTCAATCCCAGGAGT
TCTGCCCAGGGCTATCCTGGGAAAGCTTTGTTTTGAAAGGGTCCCGTGGTCACCTCTGGGCCTGGGGTGCACCCTTGCTT
GGGATGGAGAGATAGCTAGTAGGGTAACAACCCCAGGGCAGGGACAGGGACTCAGGATGCCAGCTGCCTCTGCACCCTGT
TCCTCCTCTAGAAAACGTCCAAAAGGTAGGCTCTCTGCTCCATCAGACTTGTGGTTCTTTTCTTTCTGTGCAGGTCCCCT
CTTTTGTGTGTTGGTGCGAGGGTTGGGGGGAGGCACACTTGGGGAGGTGGCTCATACAGACCAAACTCTGCCTTGGATAGG
ATTGATTTTAATGCAGCTGTAGAATCCCTGATCCTCAGGACTGCGTCCTGCTGGGCCTGTGGGAGAAGTGACTGGGGGGG
ACAGTTAAGGCGGCAAGACCTCATCACTGTCCAGGCACCTGCCCAGCCACCAAATCTGTGGACCAGGGAGCAAACACAGG
CAAGGGTACTATGCCTGGCTGCAGTCAGAGAGAGTCTCGGGAGGAGCAGGTCTTTGTGCTCTAGGGCAGGACACAGTGCT
CTAGGTGGGAACTGGTGCCCCAGGTGGGACCGAATTCCCCAGGTGAGACTTAGAGAGACCCGGTGCTCCAGGTGGATCTA
ATATTCCAGGAAGGACCTGGTGCTCCAGGGTCCTGATGCCCCAAGAGCTTGCCCCCTATGCCTGGATGTGTGAAGCTGTG
TGTTTTCTCTTCAAGAGGGGACACAGATGCCTGGAGGGTAAATGTCCCAAACTTGGCTCCTCGGGGCATGTAAAATTGTG
TGTGAGAGGCAGGGAGAGCTGAAGAGAGAAAAAGGACAGAAGGATAGGGACACGCACATAGGTCTGTAGACTAAGTACTGA
GGGGGAAGGGAGAGAGAGAGAGGTTTAGAGACAGAGTGAGAAGAAATGAAGACAGGGAGAGCCATACACGACGGACACAA
AGACAGCACCACTCAGGTCTTGTCTTCCTGTATGTGTAAGGACAAGGGAGGGAGTGAGCCGGGTAAGACCCACCAGGCCC
GGTTTCTAGGGTGAGCCTTCAGCTGCCCTTCTGCAGCAAAGGGGCAAGGAAGCAGGGCCTAGAACCCAGAAACACGTCCC
CGCGCCTGGCGCGGGCGCAGCCCTCCAGGGTGGGCAGCCGGGGCTACCGGGCGCGCCTAAGTCTGGATGAAGAGGTGGAA
GTTGGTGCGCGTGAACTCCTGATGGATGCTCTCCAGGAGCGCGTCGGCGTCGGTGGCGGGCTCGGGGACGCCGGGGTGAC
CCGGCCGCACGCTGTATTCCGGGGTGTAGGTGAAGGAGAAGGCACTAGGGTAGAAGAGCCCGTCGGCGCGCACCAGGCTC
ATGGGGATTGTGATGGGAGCGCGCAGCCAGCGCCAGTCGCTGCAGAAGGCCGCCACGTCCGGCACCACGCACACCAGGGA
CCGCGGGCTCCTGGGGGTGGAGCACCAGGGTGAGGGCTGCGGCGACTCCGACGACGCGTGCACCCCCTTCCCCTGCTCCC
CGGGGCCCAAGAGAGGCTGCCTCACCACCCCGTACCTGTACATGGTTTCTGCCTCCACGTCCCCAAACCACACCTTGAGC
CCCGCGTGGAAGTTCTCTCCGTGGAGCTCCAGCGTGGCCACGTCGCCCCCGCCGCTCAGCTGGGGGAGGGTGGGACCAGG
TGAGGTGAGAGAACCAAGTAGGGCGGGAACCCGCTGCCCCCCACTCACTAGCACCACCACTGCCCAGTGCAGGCAGGGTC
CCCCTGCCCAACGCCCCTAGCGCCCGGCTTCACCTCTAGGGTGCTGATGAGAGGCACCGGAGTGACCGGCTCCAGGGTAC
ACGCCAGGCTGGTGCTGAAGGAAAATTCCACCGACTCGGTGCCGATGATGGTCCAGCAAGAGCTGTCGTTAAGCAGAGCC
CTGTTCGCCTCCTTGGGGCAGGGAGAGGCCTGGGAGGAGACCCAAGGGGACGAGGCCGAAGCTTCTCATGGTGGCCACCA
GTGTAGGGAGCCAGCATGGCGCGTGGGCTTCTGCGTTAGACCGAACTGGATACTAATCCTAGGCTCTGGGGTTCACTTGT
CCCCTCAGCTTGGACAGTGCCCTTAAGTTCTCTCACTTAGCGTTAAGAAAGCTTGTTGAGCTAGCTGATTTCTTCTTTCT
TCTTTCTTCTTTCTTTTTCTTTATTTTCCTTCCTTCCTTCCTTTTCTTTTTCTTTCCCTTTCTCTCTCTTTTTTCCTTT
CTTTCTTTTTCTTTCTTTCCTTCCTTCCTTTTCTTTCTTTCCCTTTCTCTTTTTCTTTTCTTTTTCTTTTCTTTCTTTCT
TTCTTTTTCTTTCTCTCTCTCTTTCTCTCTTCTTCCTTCCTTCCTTTCTTCCTTTTTTTTTTTTGAGACTGAA
TCTCGCTCTGTCGCCCAGGCTGGAGTGCAATGGCCTGATCTTGGCTCACTGCAACCTCCGCCTCCCGGGTTCAAATGATT
CTCCTGCCTCAGGCGCCCGGGTAGCTGGGATTACAGGTGCCAGCCACCACACCCAAATAATTTTTTTGTATTTTTAGTAG
AGACAGGGTTTCACCATGTTGGCCAGGCTGGTCTCAAACTCCTGGCCTCGTGATCCTCCTGCCTCAGCCTCCCAAAGTAT
TGTGACTACAGGCATGAGCCATTGGCCCGGCCTGGGATAGCTGATTTCTAAGGGTCTTTTTTCTTGGGATTCCAGAATTT
TTTTTTTTTAACATTGTGCAATTCTATGCTCTAATAATCTACAATTTTAATATTCTTTGGGAAGGAATATTCTTGGGGA
AGGAATAATACTTGCTATTTTCCACATATTATAATTCTATCTCGTAACATTCTATGATAATATTCTTTTGGGAGTACTAC
TGCTTAACGATAATTATTATTTTCCACATTCTATAATTTTCCACATTCTGTGTCTTTATCTGTGCTTCGAATAGTCTTTG
CTCATTAATTCTCTGCTCTCTATTACCTATCATGATTTTTATTTACATCCTACAATTTTATATCCCAATTTATTTACTTA
CTTTTTTTGAGATGGAGTCTTGCTCTTGTCACCCAGGCTGGAGTGCAATGGTGTGATCCTGGCTCACTGCAACCTCCACC
TCTCAGGTTCAAGCAATTCTCCTGCCTCAGCCTCCTGAGTAGCTGAGATTACAGGCGCCCACTGCCATGCCCAGCTAATT
TTTGTATTTTTAGTAGAGATGGGATTTCACCATGTTGGCCAGTCTAGTCTCGAACTCCTGACCTCAGGTGTTCTGCCCAC
CTCAGCCTCGCAAAGTGCTGGGATTACAGGCATGAGCCACCGCACCTGGCTATATCCCAATATTTTATGACTCTAAAATT
CTTTGGGAAGGAATATTGCTTCATACTATTACTTATTACTATTTTTCTACATCCTATGATTCTGTCCTGGGAGTCTATGA
TTTTAATGTTCCATGGGAGGCTGCTCAGGAAGTAGCAACCATGGTGAGGGTGATGATCTTACATTCTGTGGTTCTCTGCA
TTCTCCCTTTCCAGGACACTTGGTGCTGGTATTCTCTGCTTCTTACCTGAAATTGCACCACCTTCTCTGTGGCAAGGCAT
AAGTAGGTGCCACCCCCTCCTGGGGGACTGCCTGGAAACTGGAATGCACACTTGTGCAGCTGGGAGATGGGCTCATCCAC
ATCAAGGAGCGCACACTGTTTTGCTACTTTACGGATGATCTGCAATGGAAGGATGGTAAGAACAGTGGTGGCAAGGAGCC
CGGGCGTCCAGAGGCTGCCCTCCTAGCCGGCTGTCTGCTACTCCAATAGCCACACAGGCACTAGCAGATTCCCCCTGCCC
TTTGACTTCTGTCTGGTCCTACGCGACACTGGGTGAGAACAGGTGTCTCCATGCCTTGGGGCCTCACTCTCCAGCCCTGC
CCGTGTGTGCCACATTCTCTGCATGGATCTGAGGGGACCCTCCAGGCATGCCCTCCCTTCCTATACCATGGGAGGTAGT
GTGATGCCGGTGACCGTGCAGACGAGCTGCACCAGGGAGCCATAGCGAACGTAGCCCTCTCGCGGTGGGAAGTCTCCTTG
GGCAGAGTGCCCATCAGCTGGGGGACAAAAAAGCAAGGGACAAGGTTTTCAGCGGCCCCCAAGCTTCTGCCTTAAACCC
TGCCTTGTTGCCTCTGGCTCTTCCTCACATCACACATGGGCCCTGCGGGAAAGCTTGGTACTCAGGGGAGCCAACTGAAG

```
GTTCTAATTCTGGAGAGGCCATTTGAGGCCATCCTCACAAATGTCTGCCCTGGGGGCAGGGGTTGGGGGCAGGCAGCTTA
GTTGATATATGGGGAAAGGGCATGGATAAGGGAGTCAGAATAGCTTGGAACAGTCACCAGCTCACTGGGACTGTAGTCAA
GTATTTTTGTGTTTCTGAGCCTTAATCACATGGGGCAGAGTTTACCTTCCTTAAGGAGTTGTGGTGGAGGTTAAATAAGC
TAATGCATGTGAAGTGCCCAGCTCCAGGGCCTGCAGATGTCATTACCCAGGTGGAGCGTGAAGGCAGCCCACTGTCGTGC
ACTGGCCACAAAGGCCCCATCCTCCACAGAGAGGTAGCGTGTGGAGACCGTCTGAGAGCGCAGGCGGTTGAAGAGGGAGA
CCTTTGAGCCGGAGGATATGCACACTGTGAGGGTGAGGGTCAGGGTTAGGGTGAGGGTGAGGGTCAGGGTTAGGGTGAGG
GGATAGGGTTAGGGTTAGGATTGGGGTTAGGGTTAGGGTGAGGGTTAGGGTGAGGGTGAGGGTTAGGATTAGGGTGAGGG
TTAGAGTGAGGGTGAGGGTAAGGATTAGGATTAGGGTGAGGGTTAGGGTGAGGGAGGGGGTCAGGGTGAGGGTTAAGGTT
AGTTGGTGCTATCAGTGCCCTGTCCTGGCTTGGTTCCTTGGACTTTCCAGACAGTTTCCAATCTTCTGTCCCTGTGCCGG
CCTGGCTAATGTGCCCACAGGCCTAGATACACTGGGCATTCCCAGTTCACAGCTGTTTTTCCAGGACCTGCTGCAGTCCT
GCTTTGGAACCTTTGCCTGTTCCTTTGCTGTCTGGACATCTTTCAGTTCAACAAACAAAAGTAAAACTTTTTTTTTTTTT
TTTGAGATAGCATCTCACCCTGTCACCCAGGCTGGACTGCCGTGGTGCTATCACAGCTCACTGCAGCCTCGACCTCTTGG
GTTCAAGTGATCTTCTGATCTCAGCCTCCCACGTAGCTGGGACAACAAGTGCAGGCCACCATGCCAGGCTAATTTTTCTT
TTTCTTTTTTTTTTTTTTTTTGGTAGAGACAAGATTTCGCCATGTTGCCCTAGCTAGTCTTGAACTTCTGGGATCAAC
TGATCCGCCCACCTTGGCCTCCCAAATTGTTGGGATTACAGGCATGAGCCACCGTGCCCCACCTATGTCACTTTTAGTCT
GTCCCTAGTTCAACTCCTGCCTAGTTCAACTTTTGTCTGCTCTGGGACCCCAACTCCCAACCTGGAATTTGGTATCTGTC
CCTTTCCTCTCTGCTTTTCATCCTTCAACTCTTCTCATCCCCAGGCCTGGGCCGCACCCACTCATCCAGACTCCTTCCCC
TCTCGGCTCCGACTCCACCTTTTCCACTCTGACCCCTAAGCCCAGCCTACCCCCACCTCCGCGCCCCAGTTTAGCCGTTC
TGGGCTTGGTTGCACCTCCCCGCCCGGGCCGGGGGGTCAGGCCCCGCCCTGCTCACGATCGGTGTTTTTCAGCGACTGCTT
CTTCTGCGAGGGCTTCGAGATGACCTTGATAAGGCGGCTGTGGAAGGTACCCAGCTCCCGGCCCCCGCGCAGCACCAGCC
GCAGCACCAGCCGAAAGTGCTTCCTCTTGTCTGCATCTGAGATGTACAGGGTCTTGGCGCAGCCGAATTCCTAGGGGGCT
CACAGGTACAGGGCCATCTCATCCCAGCCCTCCCCCGTCGGATGAACCAACCTCTCCCAGATATCGGACGTCCGCCCCGT
CGGAGGTGACCAGAAGCTCCACCATCCAGGCTCTCAGGCTCGGCCTTAAGGCAGGACCCCGGCCTCCCGGTCTCCCCACC
TATCTCTTGGCTTTCGGAGGGGAGTAAAGGAGCCCTCCTTTCCCAGGAGAGGCAGGGATGGGAGCAGGAATCGGACCCCT
TCCCGTGCGCTCTCACCCTGGAGTCCGGCTGCTGCTCGAAATTCAGCTTCTGCGTCTCAGTGGCGCTGCCGGACGCGCTG
TCCAGTCCCATGTAACCGCAGACCGTGGGCCCCGTTTCCCCGCCTGGTGAGCTGGAAAGGGACGCGAACTGGCTCTGCA
AGGACTCGGGAGGACGCAGGTGCCAGGCAAGCTTAGCGCTCGCCGCCAGAGGGCGCGGGGAGACTGCGCCTGCGAACCGC
CCCGTCTCGGGCCCAATCTCGTGGTCCTCCCTACAGAGCAGGCGGCCGGCAGCCCTTGATTCCGCCCTCACCTTGATCCT
GCCCTGGCTTCACCCTCCAGCCAGGCCCCGAGAGGTAGACACAGGGCGGGGGGCAGAAGAACCTGCGGAGATAAGTGAGT
GTTGGAGTCGTGCATCCATCTCCACTTGGCTCTCCTGTAAGCACGGCAAGGTAGTAGCATAAACCCAGCAGAACCTTCGT
TTCCTCCTCCCGCTCCCCCCAACGCTTTTTCACGACTGTAAACCCCATTCATAGCCAAACCTTATTGTTTTTACACCCCT
CATTCAACTCATCAGAAGGTCCTGTCCACTCCACCTTCAAAATATAATATATCCAGAATCCACTGGCTTCTTACCACCTC
GCCCTTCATCCCAGCCGCCATTACTTTTTGCCTGGACCACAGCATCAGCCTTCAAACTGGTCTATTTCCAGAAAGTTCTG
TTTCTATCTCCTTCCCATTTCGCACAGCAGCCAGAGTGCTCCTTCCTTCCTTCCTCCTCTCTCTTTCTTTCTTTTTTTC
TTTCATTCCTTCCTTCCTGTTTCTTTCTTTTTCTTTTTCTTTTTTTTTTTTTTGAGGCAGGGTCTTGCTCTGTTGCCCA
GGCTGGAATGCAGTAACTTGATCTCGGCTGTCTGGGCTCACTGCAACTTCTGCCTCCCCAACTGATCCTCCCACCTCGGC
CTCCTGAGTAGGTGAGACTACAGACACAAGCCACCACACCCATCTAATTTTTGTGTTTTTTGTACAGACATGGTTTTGTC
ATGTTGCCCATGCAGGTCTGGAACTTCTGGGCTCAAGCAATCTGCCCACCTGGGTCTGCCGAAGTGCTGGGATTACAGGG
ATGAGCCACCAGGCTGGCCAGAGTGTTTTGTTAAAAACATGAGTCACATCATGTTAACTCCTTAAAATCCTCCACTGGTT
TAAAATTCAAAGTCCTCCTGAGATCTGACCCCTGCTTACCTCTCTGACTTGTTTCCTACAACTCCTCCCTCTGATTTCAG
CCACTGTCATTTCTTTTGTACCTCAAACAGGCCTAATTGGTACAGCCTCAAGGTCTTCCCATTTTCTGTTGCCCTTTTTT
TTTTTGAGACTGAGTCTTGCTCTGTCGCCAGGCTGGAGTGCAGTGGCATGATCTCGGCTCACTGCAACCTCCGCCTCCCG
GGTTCAACCAATTATCCTGCCTCAGCCTCCTGAGCAGCTGGGATTACAGGTGCCCGCCACCATGCCCAGCTAATTTTGTA
TTTTTTGGTAGAGAGGGGGGTTTCACTGTATTGGCCAGGCTGGTCTCGAACTCCTGACCTCAGGTGATCCGCCCGCCTCGG
CCTCCCAAAGTGCTGGGATTACAGGTGTGAGCCATCGCGCCCGGCCCTTCTGTTCCTTCTAATTGTAGCACGCTTTCCCC
AGATATTCTCCTGTCTGGCTCCATTTCAGCATTCAAGTTTCAGTTCCAATGTTACCTCCCAAAAGGTCTCTCAACCTCCT
TGACCTTTCTAGCTAATGTCCCCCCTGCCTTCAGTTTCCGTCAAATGGCCTCGTTCAGTTTTCCTTCTAGTGCTCATCAT
AATCTGCAAGTATCTTGTTTCCTGACTTGTGGGGAGTTTACTGTCTGTCTCTTCCACACTTGAGTGAGAGTGTGGGCTAC
AGAGCAGGGGCTGTTTCTGTCTTGTTCAGCTGTATGCCCAGTGCCCAGCAGTACCCACAGAGCCTGGTGCATACATGACA
AACACTCAGTAAGTAGTTGTTGAATGAATGAATAAGTGAATCAATGGGAGAATGAGTGAATGAATGGAGACAGGAGATTC
ACAGGGAATCTCCTGCTGTAGAACTATAGACCAAAGTGGGGCTAATGGTGGCTGAGGGTCAAGGCCCTGGTCGACCAAT
AGCTGAGATCTCAGCCCTGAGGCCTGCTCTGCGTCTAAAAGAGGCCCAATGGGTGGATGGAGGAGGTGCATAGGGCTGAC
AGTTGCAGAGACCTGGGAGGGGCTGCGGAGGCACCTACCGCTTCTCATTTCCGTATGATTTCTGGGCCACCTTGGCATGC
AGGATCCGCACAGTCTGTTCACACTGTTGCTGCAGGCACCTGCGCACGCCTCCCCTCAGAATGGTGGTGTGCTCTGGGGA
TGACCTGGGAGTAGGGCAGGCAGGTCGACCAGCCACAGGAGGGAGAAGGGTCAAGGTGGCACAGGGTCCAAGCACTCAGG
TTGAGTAAAATGAAGTGAGCAGGGGGTTAGAGTGAGTGGGAGGGTGGGGCTGCTCTATATCCTGCCCCTGTGGGGTCAGG
AAGAGGAAGCATTAGGTAAGGAGGAGACTTGGCTGCAAATTGTAGGCTAAATATTTAGGCTGCAAGGTGCTCAACGAAGC
CTGCATGGAACCCATGGGACCACCCAGGTTCCACTCTGTGGGTCTGGCATCATGCCCATTGGAGCCCAGGGCCATAGGGA
CACGTGGCTGTGACACAAAGGCTGCACTTATACTGGCCTTGGGATGGAGAAGAGTATTTTAGGGCTGATGGAATTGGTAG
AGATGAAGCTTCCCCACCCTGGGGAAGGCAGGGTGGGGGGTGAGGAGGGAGGGGAAGGGAGAGCCTTTCCTGGGCTCCTG
GCCTGGAGCGCTCTCTAGCCTCCTGCCTGCAAACAGAAAAGGGCCCAAAACCTTGTCCTCAGGATGGGGGATGGCCTGGG
```

```
GGAATCTGAGCCTTTCTGGGGCCAAAGCCATATTCTTCCCTTTCTCCCATGAGAAGTGGCCTGTTGCTGTGTGGGCTTTC
CTACCCTTGATTACAGGAGTCAAGGCAGATGACATTGGTCCTGCAGAGTGAGCCAGGTTAGAGCCTAGAGGTCTGGTGGC
TTCTTAACCTGGGGGTCTTGAGCGGTGTGGGTGGGTAGGCCCGAGCAATTGCAGGGAGCAAGTAGAGGAGGGGAGGTGGG
AGAGATTTGGGGGGGTTAGCAGGTTGAGGCCCAAGGAGAGCCATGAAAACTGAGGCTGTCCTTCTGAGCCCTTATTCCTCT
TGGCAGGACCAGAGTCTCTCCTCACTCCTGGAGGGGACAGCGCAAGACCGCACCCTCGAAAGCCAGGCCAGACAGGCAGC
AGCCACAGCCAGGACCGCTCCACCCCTTGGGGCTCTCCTCAGGGAAGTGCCTTCTAAGCTTCCAATGAATTCCGGCTGTG
GAGTGTGGCTGCCCGGTCTCCCCAGTCTCCCGCTGTAGCTGACTTCCAGCCTGGCCCGCAGCTAGCCCTGTCTGCAGCT
CCAAGCGTGGTAGGAGCAGGGGGTGCTGGGTTTGCCCCCCCTCAGGAGGAAGGGGGGATCCCTCCCTGCCAGTGGGAGGG
GTTGCAGCCTGCACTTCCCCTCCCCACCCGCCAGCCCTGGGTTAAATGCGGCCGTTGCCTCTGTGTGCTCCAGCTGCCTG
GCAGTCCCCACCTAGGGCACGCTGCCACGCCGCCGTTACCTGGTCCAAGTGCCCGGGAGGCTCCGCCTGTCGGCTTCGCT
CTGCAGCTGCATCTCTGATCTGTCCTGCAGGCTCAGGTGAGTCAAAGGATTGGGAGGCACTGAGGGGTCTGCAGGGGAGT
AGGCATCAAGCCAGGTCAGGCGAGTGTATTTTAGCAGAGACGCTGCCTAGGGTGCCCTATCTGATTTGCAGTGCCCTTCC
CCACTTCTGCAGGGTCCTTTCCAAATCGAGCCCCCACCCTCAAAGCCATCAAAATAGGATCATCCCTTTTCTACTCTCAT
CCCTCCAGGAACCCCCTGAAATGTCCCTAAGGGACTTTTGAGAATTTATTTACAGGCCTCTCCTCCCTTGCTGCTCCACT
CCCTCAACCTCACCTCAGGGCATCTCTGAATCTTTCTTTTTTTAATGCAAGGATTAACTCCTCAGAGGAATCCCAGGTTC
TAGTATCAGTCCTGCCACGACATCGCCATGTACCCTTCAGCAAGACACTACCTCTCTGGACCTAGTTCCTTTCTTTGTA
GAATGAGGGGTTTGGAAAGGTGATGAACTTTGAGGCATTTGCTATTTTGACTTTCTAGGATTCCTGGGGTGGAGGCAGGG
TGCCAGGTGAGAAGTTGGCTCAGACTAAGAATGGGATGAGGGGAACCTTAACCAAGATTTAGGGGCCCAGAGGCATCTCC
GGGTAGGAAAGTATGGGAGGTGGCAGAGGCCCCTTGTGCCTTAGCCCTCATGCTGCCCAGGATGTTGGCTTTGCCTGTG
GCCCAAGCTTGACAAGGGTTCCCCGCCCTCCCACTTCCACCAGCGCTGCCACATCTTGCTTGGTCACCCTCCCACGACGG
GCCCTGCCAGAGGATTTATGTTCTTCTACCAGGCCTGGGAGGGGAAAAGGGAGAAGAATCCGGGATGAGAAGGTCGCCTG
GAGTGAGAAGCAGGCACTGGCCTCGCTCAGAATAGGCGCATTCACGGAATTGGGGGGCGGGGGCGGAGCTGCTGTTGGGT
TAGGGGCAGCTGGCCCTCTCTGCCAAGAAACAGCAACAGAAAAATGGCTAACACAGCTTCAGCATGGGGCGCGGCGGTGG
GGTAGGAGCTGGAAGGAAATGAGGTTTAGTGAATTCTAGGCGCCAGGGGGCCTCAAGAGAGAGAAGGGGAACCAGGAGCC
CTAGGTTCTAATCCTGCTTTTGCAGTTCGGCGGGCTGCCTCCTTGGGGCTTAGTTTTCCTATTTGTATAGTGGGGAGGGG
GTGGGGGGGTGGTCCTTCGTAGTTCTCACGGGGCGTCTCCGGGGCCTCGGGAGGGTAAGCGCTCACCTGCTGCCCCTGCG
GGGTCCATCGTGGCTCCACGGGGCCCCAGCCGCTCACCCGCCGCCCTCTGGACGAGTCCAGTGCTGCTGTCGCTGGAACC
CTGCACACCCCTGCTCCTCGGCTCTTGCGCGGGTTATATCCTGTCCAGGCCTTATCTCCAAGGCCCCGGGCCCCGGCCCC
CTGTTTGCTCGGGGTGGGCGCACCTGCTGGGCAGATGTGGGCTTCCCACGCCCCCTGCCATCTCGGGCGCCCCCTGGCGG
TCGCTCCTGAGAGCAGGCGGGAAACAGGCGCCAAGCCGCGCGTTCTCGTGCGCAGTGGGTTTTGGTCCACTCGCTGCCAC
GCCACCACGCTCAGCCCCGCAACAGGGCGCAGCCCCTCCCTGCCGCCTGCGGTTGGGCAGTGCATGTCTGTGTTTGTGTG
TGTGGAGGGGTCTGGGGGGGTTGGCGAGTTGCAAAGACAGCCACACAGAAGCCTGTGTGCGTAGGGGAGTGGGCCGAGGT
GGGGAAGGGCTTCTGTCTGCAGAGGTTCGCCTGGCAGGAGGAGAGGCACCAAACGCCCAGGCTGGCTCACAGAAACTAGC
AGGCGTGGGATTGTATCCTGCCGCAGCCACTTTTTGGCCGAATGACTTTGGGTACCAGCGCTTAACTTTTCTGAGCCTCA
ATTTCATTGTCTGTATGATGGGGGACAATCACAGCCTTAGTCTTACAATTGCAGAGAGGATTAAATGCCACAATGCGTGA
AGAGCATTTAACACTGTGCCTGACATTTAAGGAAGACCTGTTTTTTTCTGTTTAAATTTTGCATATTTGTATCTATGTAT
CTATTCTTATTTACCATTCTCTTGTGGGCCGAGCGCGGTGGCTCACGCCTGTAATCCCAGCATTCTGGGAGGCCGAGGTG
GGAGGATCACCTGAGGTCAGGAGTTTGAGACCAGCCTGACCAACATGGCAAAACCCGCTCTCTACTAAAAATACAAAAAA
TTAGCCAGGTGTGGTGGCAGGAGCCTGTAATCCCAGCTACTCGGGAGGCTGAGGCCGGAGAATCGCTTGAACCTGGGAGG
CAGAGGTTGCAGTGAGCCGAGATCATGCCACTGCACTCCAGCCGGGACGACAGAGCAAGACTCTATCTAAAAAAAAAAA
AAAAAAAAAAAAAAGAATCTCTTGTGTTTCCCAGCAATCCCACTGTGATGAGAATTTTTAATTCTGAAACATTTCAAA
CATACACAAAAATAGAGAGATTAGTAAAATAAACCTGTAACTGTCTTCCCCCTCCAAGTGCCTATCACCCAGAATAAGAG
CTCTGTAAATACTGTTGCTTTTCTATTTTCCTTCCTCCCTCCAGGCTCTGACACCTCCATTCTCTGTCCCCAAGCGCCAT
GAGAGGCCTTCTTTGCTGGCCCGTGTTGCTGCTCCTTCTTCAGCCCTGGGAAACCCAGCTCCAGTTGACAGGTGAACTGG
GGCCCAGCCCTCACCAGTGGGAGGAGGGGAGCGGAAGCAGGTCCTCCCTCTGCAGCCAGCTCCTCCTGCTGGGGAGAGGG
AAGCAAGGGCTTTGGAACATATGGGACTGGGTTTAAGTTCCCCACTTCACCTTTCTGGGCCTCAGTTTCTTCATCTGTAG
GATGTGGCTTAGTTTTGGGCGCATGCAGAACTGCCCATGGGGTGTTTAATTTGGAAAAATTATTTGAAAACATGGAAAAA
AACCAGGAAATTTTACATAAAAATCCCTGTTTAGTATTTCTCTTGAAAAACCAGGGATCTGCTGACATCCAATTCGCAGG
GGCATGGCCTCATTTGGATGCAGCTACCTGCCCAATTTAGGAGTGATAGGGGCTCTTTGGGTCTCCAGCGTCCTAACCCA
ACCAGTTCATCCATGTTCCCTGCCTGGTCCCTGCAGGCTTTGAGTTTGCGACGCTTCCTTGCCCCCTCGTCAATGGGTAA
TACCGCCTCAGAATAGCCCTCAGATCTGTAAGATGAGGGAATTTGTGAAATCTCCTAGCCCAGGTGCTAGGCACATGCCG
GGCGAGTGCATCTGTCTAATCTGCGTTTGCTGTGCTGGGCGCTGTTATGTCGGCATAGCGGCATTTCCCCTGGGTTTCCT
AGGGGGTCTCCTCGGAGGTGAGGCTTTGCTGACCTAGGAGAGTCCTCCCTGATCTCCCCGCAGGTCCCAGGTGTCACACT
GGGCCCCTGGATCTGGTGTTCGTGATTGACAGCTCCCGCAGCGTGCGCCCTTTCGAGTTCGAGACCATGCGGCAGTTCCT
CATGGGCCTCCTCCGAGGCCTGAACGTGGGTCCCAACGCCACGCGCGTTGGCGTGATCCAGTATTCGAGTCAAGTGCAGA
GCGTCTTCCCTCTCCGCGCGTTCTCTCGCCGCGAGGACATGGAGCGCGCCATCCGCGACCTGGTGCCTCTGGCGCAAGGC
ACCATGACGGGACTGGCAATCCAGTACGCCATGAACGTGGCCTTCAGTGTGGCCGAGGGCGCGCGACCGCCAGAGGAGCG
CGTGCCGCGTGTCGCTGTCATCGTGACAGACGGGCGGCCCCAGGACCGCGTGGCCGAGGTGGCGGCACAGGCGCGCGCCC
GCGGCATTGAAATTTACGCGGTGGGGGTGCAGCGCGCGGACGTGGGCTCCCTGCGCGCCATGGCATCGCCCCCGCTAGAC
GAGCACGTCTTCCTCGTAGAGTCCTTCGACCTCATCCAGGAGTTCGGCCTGCAGTTCCAGAGCGGCTGTGTGGTGAGTT
AGGAGGGCGCTGGAGTGAAGCTCGAACTCTCAATCCAAATGCTTGGATTCCTTTCCCACCACAGTAAATCCCATCCCGTG
```

GGGGTGGCCCCGCGATTTCCAGGTAGAGTTGCCCTGACCTACCGCAGGCTGTGTCAGATTTCAGCTCTGACCACTTCTGC
TCAGCCTCCATCAACAAGGAGCAACCTATAATTTCTTGCCCTACCCGCCACAGCTTACCACAGGCTTAATGTCAAATTCC
AGCACCAACCCCCTGCACACACGGAACCAGTGATTCTCAAAGCTAAGAGGGATCGGCTGCTCCAAAGAGTCGTGGCCCTC
CCACATGGGGATGACTTCTCCCATTATCATCTTAGCTCCTCCCACAAATGCTGCAAGATCTGTTCTGATCTGTTTTGAGT
TTATTCCGCCCACCTCGACTTAGCACCTCTCCACCTTCCTACCCACTCCTGCCCCTGTGTAGTCCTGTCCCAGCCCACTG
ACGTGTCTAATTGAGTGTCTCCTCTTCTGAGCCTACTCACAGGGGCTCCTTAAGCCTTATTCTTTCCCTAAGCCCTCTCA
CTTGGTACGGCCCTTCTCAAATTAGTCATTTCCTTCTGAAAGCCATTTGAGGGTGATGCCTGCCTCTGACCCCATCCCCA
TCCAGCTCTCTCTTGTTGGAGGCTTCTGCCCATCTGTTCCCTACTGATTCTAGTCCTGTGTGTCCTCAGGGAAGGACCAG
TGTGCTGAGGGGGGACATGGTTGCCAGCACCAATGTGTCAATGCCTGGGCCATGTTCCACTGCACCTGCAACCCAGGCTA
CAAGCTAGCAGCAGATAACAAGAGCTGTTTGGATGACAGCTATCCCTGTCCTGCAATCTCACTGCTCCTGAACCTTCTTT
GGCTCTCCATTGCCCCAACATCAGACCAATGAGTCTGGAAGGGCCTTTGGCAGTAAAAGCACACCTCCATCACTTTACAG
GCCTAAGAAAGAAAGGTATTGCCTGGGGTCACATAAGTCCCTGGTGGGAGCCCACAGTTCAGTGTGCTTTCTGCCCTCTT
TCTCCCTGTTTTCAGGCCAGTCTCTAATCAACCTCCCAAACTTGTTTCTGTTTTCTTCTTTTCTTTTCTCTCTCTTTTTT
TTTTTTTTTTTTTGAGACAGAGTCTGGCTCTATTGCCCAAGCTGGAGTGCAATGGTGCGATCTTAGCTCACTGCAACCT
CCGCCTCCCGGGTTCAAGTGATTCTTGTGTGTCAGCCTCCCAAGTAGCTGGGATTACAGGTGCTGGCCACCACACCCAGC
TATTTTTTGTATTTTTAGTGGAGATGGGGTTTCACCATGTTGGCTAGGCTGGTCTCGAACTCCTGACCTCAAGTGATCTG
CCCGCCTTGGCCTCCCAAAGTGCTGGGATTACAGGCGTGAGCCACTGCGCCCGGCCATTTCTGTCTTCTTACAGGCACAT
CCTTACGGTTTCTCTTCTTAGATACTGTTGGGGGGTCTTCAATTAGGTGGGTGTCCCCCCTCATGCCACCCATTCTTGAC
TCTGCACCTCAGCTCTTTGTGTGCTGCTCACCTGAGCTGCCCTTGCCACTCCCCTCTGCCTTTGCAAATCTTTCAAGACT
CTCTCTTTTTAAGAGATAGGGCCTTGCTATGTTGCCCAGGCTGGTCTCACACTCCTGGGCTCAAGAGATCCTCCCGCCTT
GGCCTCTCAAAGTGCTGGGATTACAGGTGTGAGCTGCTTCTTCATTTTTGTCTTCTGAAAAGCTATGGCATAGAATACCT
TGCTTATTCAATTCAGTTTTTTTACTGAGTACCAAGTTAGTATTAGACATTGTGCTAGGAGCATTACACATGTTATTATCT
CTCAGCCTCACAACAACCTGGAAAAAGAAATCATGTAATCCCAATAACCCAGATGTCAGCTGGGGTCTTCTTGTTTCTAG
AAACTAAAAGTTGGCTTGAACTCTGATTTGATGACTGTTTAACATTCAAATCTCTCTCCAAGACCTCTGGAAATCCCCCA
AGTGCTTATCTTCTGGTTGTCACCTCTTGCTGTGAGCCTCTGCTAAATTCATTTGATTTATGGATCCTCTCTCATTTCTC
AACCCTGCACATGCTGTTCTTGTTCCATGCATGTTAATCAACCTCTTCGTTGGTTTACACACTTGTGTATGCAGGGGCAT
ACACAAGCGTGTATGTGCTAAGTAACTCATAGCGCGTGCACACACACGAAAAACTCATTATAATTGAATATTAGACAG
CTTTGTTTCTGAAAGTCCTCAGTCAGCCTAGTACAAGGGATTTTTCACAGGCAGCCTCAACCCACAGAGTTACTCATCTT
CATACTGGCTCTCTTGTTCTTTTTTTTTTTTTCTTTCCTCTCTCCACTCTCATCTCCTTTCCAGGAACCCCAAATACTC
ACTTTTTTATACAGGGTAATTATCTTCTCTCCAGTCCAGCTCTCGGCCATCTTTGCCTATCCAGATCATCAAGATGCTCA
ACAGGGCAATAGTTCCCCATCTTCCATGGAAGGAAGCTGTGAATAGCAATCACTGTCTTCCATTATATTCCCATTTTGCA
AATAGAAAAATGGAGGCTCAAATAGGTAAAGTGGCCAAACCTGTCCTCTGTAAGTCAGAGAGCATCTTGAGGACAGGGGC
CCTGACTAATTAGGTCAGGATTTTTGGGGTTGGGGCCCAGGCGTAGGTATTCTTTAAAATTTCCTTAGGTGGTTGTGCTA
CCAGAGCTGAGAAGGGCAGTCCTGGGCATAGACTGACCCCATTTCTTCCTCAGCCATTGATCTGTGTGCTGAAGGGACCC
ATGGATGTGAGCACCACTGCGTCAATTCCCCAGGCTCCTATTTCTGTCACTGCCAAGTTGGCTTTGTACTCCAGCAGGAC
CAGAGGAGCTGCAGGGGTGAGCAACACCCCCACCCTCCCACCACACCCTGGACTGTGGCCTCCGAGGGCTCCAATCAGAG
GCAACATCTTGCTGTTTCTTCCCTCCTGCCAGCCATTGACTACTGCAGCTTTGGGAACCATAGCTGTCAGCATGAGTGTG
TTAGCACCCCTGGTGGGCCACGGTGCCACTGCAGAGAGGGCCATGACTTGCAGCCTGATGGGAGGAGCTGTCAGGGTGAG
GAGGGCTCTCCTACATTTGTGGGAGGGGTAAGAGATCTTAGCTGGTGGGGTCCATCCTGACTCATCCTGACCTGTCTCTC
CTTTCAGTCCGGGACCTTTGCAATGGCGTGGACCATGGCTGTGAGTTCCAGTGTGTGAGCGAGGGCCTCTCCTACCGCTG
CCTGTGCCCCGAGGGGCGGCAACTTCAGGCAGATGGCAAGAGCTGCAACCGTGAGTGATGGGCGGGAGGGTGTTCTGTTG
GCTTCTGCCCCATTGCCCACATTCGGAGTGTCCTTGACTTTGCCATTGCTGTGGCCCCTAGGCTGTGGTGTGAATGTCTG
TGTGTGTTGTGGGGGTGACATTGGAGGGTGTGCCATTCCCTCCTTCCATCTCTTTCCTACAATCCTCTACTCTGCTGTCA
ATGTTAATTTAAAAATACATATTTGATCACATTCCTCCTTTGTGCACAAGCCTTCTGTGGCTCACCAATGACTACAGGAG
AAAGATCAAATGACTTACCCCTAAGTATTTCAAATGGTGAGCAGAACCACATTAATGAATAATGAAATCAAATTACTGGG
TTACAACCAACATTAAAAAAAAAAAGAGTGGAAGACAATATAGAGTTTACTGCCTGTAGAAAGAGTTATATTATTTCTGAA
ATTTTAGTTCTGTGTGAAATGTCTCATGATGTATTGTCATTATTATTATTTTTACTGTGGGCTGTGATAAAAATTTTGAA
ACCACTGCCTTAGGTCATCACGATCTAGCTTCAGCCAACTCCATCTGCCCATCTCCTGCCACACTTGAACATTTCAGTCT
TACTGGCCTGCCCCTCCCGGATGTGTCAGGATCTCCCCAGATCCCAAGCCTCTGCACGTGCTGCATCTCACCTTCTAGA
CTGTCCCTTACTGCCTTGGCAAACTCCTACTGATCTTTCAAGACTGTCTTAGATCATTCAGGCTGCTATATCAAAGTACT
GTAGACTAGACATTTAGTTTTCACAATTCTGGAGGCTGAGGAGTCCAAGACCAAGGTGCCAGCAGATATGGTGTCTAATG
AAGGCCTGTTTTCTGATTCTTAGATGGCACACCTTTCAAAGCCTCCCCACGCCAACACACACACACACACACACACAC
ACACCAATTTTCTTGCTGTGTCCTCACATGGTGAAGACAGGAAGCAAGCCCTCTTCGACTCTTTTTTTTTTTTTTTTTTT
TTTGAGACTAAGTCTCCCTCTGTTTCTCGGGCTGGAGTGCAGTGGCGCGATGTCGGCTCACTGCAACCTCTGCCTCTGGG
GCTCAACTGATTCTCCTGTTACAGCCTCTGGAGTAGCTGAGACTACAGGTGTGCCCCACCATGCTTGGCTAATTTTTGTA
TTTTTAGTGGAGATGGGGTTTTGCCATCTCAGCCAGGCTGGTCTCGAACTCCTAACCTCAAGTGATCTGCCCGCCTCAGC
CTCCCAAAGTGCTGGGAGCCACCATGCCTGACCTCTTCAGACTCTTACAAGGACAGTAATTTTACTCATAAGCGCTGTAC
CCTCATGTCCTCATCTAATCCTAATTAACCTCCAAAGATCTCACCTCCGGAGACTATCACATAAAGGCGTAGGGTTTCAA
TAAATGAATTTTGGGAAACACACTCATTCAGTTCATAACAAAGATCAGATAGAAGGCCATCATCTCCATGCAGCCTTCCT
TAACAACTCCAGGAAGAATACACTATTCCTACCTTGGGATCCCTGCATGATCTTCTCTTCTAGCACTTTCCTTGACCAGA
GCACATAGCATGTCTGACTTGCTCTGCTCTGTCCTCATGGAACAGGATTATGTTTTAATTATTTCAAAACCTTTGAAAAC

```
CTCCCCACGCCAACACACACCACATTTTGGGCCTAGCAACTTTCCTGGCTCAGAATAGGGGAGGTGACATTGTAGAGTCA
TTCAGGAGCATGGGTTTTGGAGTCAGATGGACCTGGCTTCATGTCTTGGCTCAGTCAGTACTAGCTATGAAATCTGGGGC
AATCACTTTATCCATCAGAACCTCAGGTCATAGAAAATGGGGACAATATTCAAGCTTATTGGGAGGATTGAGAATATATA
TAAGCACTTCACCCAGTACCTACCACATGGTAAGCTGTCGGTAAATAATGTTTTTGTTATTATTATTGTTAATACTGGTA
GAGTGCTTAGAAGTGTGCCTGATACATAGTAGATGATCAATAAATGGTAGTTATTGTGGTCATTACTATGATTGTTGTTA
AAAAAAGATGCTTTTAGCAGAGTGGTTTGTTCTAAGCACTGAATAAATGTCAAGGGCGATGATGAGGGTGATGATGATGG
TGATGGTGATGATGATGGTGATAATTATTATTACTTAGCACACTGCCTGGCACAGGAAGGACTCAGGAAATGTTGTCTGT
TATATAATAAATGTTTGCGAATGAACGACTAGATGGATAGACAGCTGCTGCCAGTCCAGAATCTGGTCCCTCAATTCAAG
CTTTTCTGGCTGTGCAGGGTGCCGGGAAGGCCACGTGGACCTTGTTCTGCTGGTTGATGGCTCCAAGAGCGTGCGTCCAC
AAAACTTCGAGCTAGTGAAGCGCTTCGTGAACCAGATTGTGGACTTCCTAGATGTGTCCCCCGAGGGCACGCGGGTGGGG
CTGGTGCAGTTCTCGAGCCGCGTGCGCACCGAGTTCCCTCTGGGTCGCTACGGCACCGCAGCCGAGGTGAAGCAGGCGGT
CCTGGCCGTGGAGTACATGGAACGCGGCACCATGACAGGGCTGGCGTTGCGGCACATGGTGGAGCACAGCTTCTCCGAGG
CGCAGGGTGCACGGCCCCGTGCCCTTAACGTGCCTCGTGTTGGCCTGGTCTTCACGGATGGCCGCTCCCAGGATGACATC
TCGGTGTGGGCAGCGCGCGCCAAGGAGGAAGGTGGGCTTGGCATGGGACACAGTGGGCTGGGGTTGGGTCAGACGCTGGG
AGGCAGCTTTCCCGAGGCCTGGGGCACCCTCTGAGACCCCGGCCTTGCAGGCATCGTCATGTACGCCGTGGGCGTGGGCA
AGGCGGTGGAGGCGGAGCTGCGCGAGATCGCCTCGGAGCCAGCGGAACTGCACGTGTCCTATGCCCCGGACTTCGGCACC
ATGACGCACCTGCTGGAGAACCTCAGAGGCAGCATCTGTCCAGGTGAGCGCATCTCTCGGGGCTCCTCCTCTCTCTCA
TTGCCCGTCCTCTGATATCTCCCCTTCCTATTCACTCCCTGCCCACTTTGTAGTTATAGCCAGAGGTTGGGCTCACTAGA
CAGAGCTTTGCTACTCCAAATGTGGTCTGCAGGCCAGCGGCAGGGCGGTCACCCGGTAGCTTCTTAGAACTGAAACATTT
CTGGTGCTCTCCAGACCGAATGAATCAGAATTTCCACTTCGACAAGATTCTCAGGTGATTTGTAAGCATATTAAAGTCTA
AGAAATCCTAGACTAGGCTACCTGTCCCCCAAATCAGTGGAGGTTCAGAATAATTTGGGAAATTTTTTTGAAGGCACACC
AGACAGCATTTGTAAGTGGAGCCACAGCATATATCCCTGAACTTGTTAGAATTCAACCTTATGAACTTGGCAAAGTGGCA
GGACTGAATATATTTTAAGTATGCAAAGACTTTTTTTTTTTTTATTGGGGACAACATACCTCATAAGTGCAATCCCTCTA
CTTCATCATCTGGTGCTCAGCTGAGGAAAAAGAGACTGACTCTACCTGTGAAGAAGGGGACTCTTGAATCGCATGCAGTG
TACTGTGCTCTAGGTGATACAGGGAATGTCTCCAGGGTAACTTTTACCCTAGAGACTTTAGACACTAGCCCAGGCTTAAG
ATGTGACTCTTCTTCCCATTTATCAGACTGCAGCTCCAGAAACAGACTAAGTAGGGCTGGTTTGGGTCTGGAGAATTTGC
CTTTTTTTTTTTTTAGATATATGTAGGGGGGCTGGGCGCAGTGGCTCACATCTGTAATCCCAGCACTCTGGGAGGCCGAG
GCGGCTGGATCACTTGAGATCAGGAGTTCGAGACCATCCTTGGCAACATGGTGAAACCCTGTCTTTACCAAAAAATACAA
AAATTAGCCGGGCAAGGTGGTGGGCACTGAGGCAGGAGAATTGCTTGAACCTGGGAGGTGGAGGTTGCAGTGAGCCGAGA
TTGCACCACTGCACTCCAGCCTGGTGACAGAGTGAGACCCTGTCTCAAAAAAAGTATATATAATATACTATATATACTAT
ATTATATAATATGTGTTTGTGTATGCATGCACATGCACGTGCATGTATATACATATACAAATATACACATACAGTCCTTC
CCCCAACTCTCCTTCCCTCCCCGCTCTCAGCCAATTCTAATTATTGTGGCTCTTTCAGATTAGGGGCTCTTTCAGAGCTC
TTCCTATGTGACTCTTATGGGCCCTGCAGTGTGACTTTGGACGAGTCACTTACCCCACACCTACCTTAACTTTTCTCTTC
GGTAACAGGAGAGGCACTGGCTGCCCAAGGTTACCTCTAGTTCTAGAGTTCTAAGAGAAGTCTGGGTTTTGTGGTTGAAG
CTCTTTATCAATTGGTGGTATGACTGTTCCCTGTCTCCAACCCTTTGTTTAGAGAAACAAATCTGTGAAGTTAGGGGTCT
TTGCTGGATTCCAGCAGTTTGTTCATAGTACATATAAGACTAAGATGAAGAGACAACTTTTAAATCATTATGGCAGTGTT
TTCAAAGGTTAAATGGCAGGTATTGTGCTTATTATTTCACCACCTGTAATCCTTTTTCTTTTTTTCCTAAGGTACTTTT
ATTGTAGCATTTTTTTTTTCTATGTCATTCCCTGTAATCTTTTTTTTTTTTTTTTTTTTTTTTGAGATGGAGTCTCG
CTCTGTCATCCAGGCTGGAGTGCAGTGGTGCGATCTCGGCTTACTGCAACCTCTGCCTGCCAGGTTCAAGCGATTCTCCT
GCCTCAGCCTCTCGAGTAGCTGGGACAACAGGCGCATGCCACCACGCCCGGCTAATTTTTTCTAGTTTTAGTAGAGATGG
GGTTTCACCGTGTTAGCCAGGATGGTCTTGATCTCCTGACCTCGTGATCCACCCGCCTCAGCCTCCCAAAGTGCTGGGAT
TACAGGTGTGAGCCACAGCACCTGGCCTCCCTGTAATCTTAACAATGGCACATGCTTATAGAATTTCAGTGAATCTAAGA
TACCATTCATTTTAAGATTCATCACTACCTTATGTACTGCTAAGAAAAAACATGTGCCAGTTTTAATTGTAAGGCCTCCT
GCCCAACCCCCAAGATGTTGATGTGTGGGGAGATAGAGTTGGATCAGGATCAGTGGTTCCTAAAAATTAGGTCACACCAG
AATCACCTGGTGGACCTTTAAAAACACAAATTGCAGCTGGGCATGATGGCTCATGGCTGTAATCCCAGCACTTTGGGAGG
CCGAGGTGGGCAGATCACAAGGTCAGGGGATCGAGACCATCCTGGCTAACACAGTGAAACCCCGTCTCTACTAAAAATAC
AAAAAATTAGCAGGGCATGGTGGCACATGCCTGTAATCCCAGCTACTCGGGAGGCTGAGGCAGGAGAATCACTTGAACCC
AGGAGGCAGAGGTTGCAGTGAGCCAAGATTGCACCACTGCACTCCAGCCTGGGAGACAGAGCTCAAAAAACAAAACAAAA
ACAAACACAAATTGCTGGGCTTCAACTCTAGATTTTCTAATTCAGCAAGTCTGGGGTGTTGTCTGATAATTAGCATTTTT
GATAAATTCCTACACGATGCCGATGCTGCTGGTCCAAGGACCACACTTTGAGAATCACTGGTCTAGTTGAAATACACTAT
AAAGCAGTGGTTTCCAACCTTTTTGGCACCAGGCAGTGGTTTTACGGAAGACAATATTTCCATGATGGTGGTGGGGAGAA
TGGTTTTGGGATGGTTCAAGCACATTACATTTATTGTGCACTTTATTTCTATTATTACATTGTAATATACAATGAAATAA
TTATACAACTCACCATAATGTATAATCAGTGGGAGCCCTCAGCTTGTTTTCCTGCAGCTAGATGGTGCCATCCGTGGGTG
ATGGGAGACAGTGACAGATCATCAGGCATTAGATTTTCATAAGAAGCCTGCAACCTAGATCCCTCACATGCACAGTTTAC
AATAGGGTTTGTGCTCCCATGAGAATCTAACAATGCCACTGATCTGACAGGAAAGGAGCTCAGGTTGTAATAGGAGCAAT
GAGGAGCGGCTATAAATACAGATGAAGCTCCCTTGCATGCTGGCTGCTCACCTCCTGCTGTGTGGCCCAGTTCCTAACAG
GCCATGGGCTGGTACTGGTCCATGTAATGTGCTGGTACTGGTCCATGGCCTGGGGATTGGGGACCCCTGCTGTAAAGTAT
TTACTTGAACCAGGCAGTGTTCTAGATCTGTGCTTTTTCAAAAAGGTAGGCAGTACCTATTTAATTACAATTGATTAAAA
TAAAATACATTTTAGTCCTTTAGTAGCACCAGTCATATTTGAAGTGTTAAACAGCCACATGTGGCTTGTAGCTACTGCTT
TGGACAGGGCAGATGTAGAACACTTTCATCATTGCATAAAATTTTATTTGGAGAGTGCTACACAAAGTGTGGTCCACAGA
CCAGGAGCATCAGCATCATTTGGGAGCTTATTAGAAATGTACCTTCTGAGCTTCTTCTCCATTCTGAAGCAGAATCTATA
```

```
TTTTAACAAGCTCTCCTGATGATTCCAATGAGCATTAAGGTTTGAGAAACAGTCTTGTAAGTTGTGCGTGAGCACACACA
CACACACACACACGTACACAGGTGAATGAATCCTCGAAGCCACTAATGCGTACACTCTTTCATTATATCCATTTTATAGA
TGAGAAAACTGAGCTTAGAAAGGTTAGTGAGTTTTTCAGTATCTCACAGCTACTTAGCTTGGGACTCAGCCCAGGTCTCT
CTAAATCCAAAGCCCATATACTCAACCATAGGCCCAGTGCAGGGAAATCAGAGGGCCAAAGCTAGGGCAATTTCTCATCC
CCCTGACCCTCTGTGTCCGGCTTGCAGAGGAGGGCATCAGCGCAGGGACAGAGCTTCGGAGCCCATGCGAATGCGAAAGC
CTCGTGGAGTTCCAGGGCCGCACGCTGGGGGCGCTCGAGAGCCTGACGCTGAACCATATCCTTTGGGGCTGGTGGCGCGG
GCTGGAGGGAAAGTGAAGGGACCTCGGCGAACCCCAACGGCCTCCTTAACCGCTCACTGGCCCAGCTGACGGCGCGCCTG
GAGGATCTGGAGAACCAGCTGGCCAACCAGAAGTGAGGGCCACGGACGGCCCAGACCCGGGCTGGGGCGCGGCACCACGG
ACGGTGCCCCTTGCGCGCCATCGGTGCGCCGGGGCCAGGCAGAACCTGGGCCCGTCCGGCTTGGGCTGTCGGGGCGGAGG
CGCTGGCGGGCTTCCGGCATTGAGCTGAGTTGGCCTCGCCCGGACCATTAGGCGGACTGCGGCGTCAGGGGGATAGCGGG
TGGTGAGGGAAGGGGCACGTGCTAGACCGGCACGCCCTCGCCGCGTGCTGCGCTCAGTTCTTTGTTGGATTTCTTGTTTG
TGTTCTTAAAAAAATAAAAAAAACTGATTTCCACGGGGTCCGTTTTTGGGTCTTGCTGCGCCGCCTGGGGGAGGGGAAGC
CAGACGGAGCGTGCTGTGGCGGCGCCCGGGCGGCGTCTCCTTGGAGAAAGGCGCATTGTGCGGGGGTCGGGCTCGGGGGC
AGGAGACCCGGCGCTGCTTCCCCCTGCTTGTTTATTCAGCCGAGAACAGATGGCTCCTTATGCAGGCTGCGGGAAGGGAG
GGGGCTCAACAGGAATCCTTGGGGTCTGAGTAAGTCTGCGCCACTCCATTCCCCAGCGGACTGAACGCTTCGCTCCCGGC
CACGCCTATCCCATGATGCCCCCAAATCTGGCTCAAAAGCTGCTAGTACTGAGCGCTGGTAAGGGGTGCGGGGGTGGCGT
TGGTGGGAAAGTGAGAGGCAGTAATTGAACCCCTAAATGGATGGGGTGGGAGTGGATAGATGAGCTTTGACCGATTTTAA
CTCTCTCCTGCACTAGGTAGGAAGAGAAGGAGGTATCAGCTAGGACCCCTGTCTGTCCAGCCTGTGGCCTTCCTTCTGTC
ATCTTATCAACCCCTCCCAAAACACCTCTATCAGCAAAGAACACAAACCAGTACCATTTCCCGAGTGCATGTAAGAAACA
ACGAAGTTTAAAATGCTTCCAGTCCTAGAGTGATTTGGGGAAAATTATTTTGTGTTTGTTCCTCATCTGCAAAGTGGAG
ACTGGAATTGTTACCCCCAAGCGTGATGGGGAGAATCAAGGACATTCTTCTAAGGCATGTGCCTTGTACCTATCCCTCAA
TTAATGGTAGCTATTAACTGTTCTTATAGTAACAGCTAACATATTTAGTGTTGGGCATTATTGTAACTGATTTGCAGGTA
TTTTCATTTTATACTTGGAACCAGTGAAATAAGAATTATTGCCTTTATCATCCCCATTTTACAGATGAGGACACTGAGAC
ACAGAAAGGTTAAGTTTCCAACAGTAACATAACCAGTACGAAGCTGAACCAGGATGTTTCCTATGGCTGCTCTAATAAGT
TACCACAAATTGAGTGGCTAGCAACACACATTTATTACCTTACAGTTTTTAGGTCAGAAGTCTAAGATGTGTTGGCAAGG
CTTTATTCCTTCTAGAGGCTTTTGTTTCCTGGCCTTTTCCAGCTTCTAGAGGCTACCTGAATTCCTTGGGTCATGGCCCT
ACATCACTTCAGACTCAGTTTCCATCAGCACATCTCTTTCTCTGACTCTGACCTTCCCGTCTCTGTCTTATAAGGACCCT
TCTGTTTATATTGGCATGACCTTGATAATCAAAGATAATCCCATCTCAAGCTATCTTTTTTTTTTTTTTGAGATGGAGT
TTTTGCTCTTGTTGCCCAGGCTGGAGTGCAATGGCGCGATCTCGGCTCACTGCAACCTCCGCCTCCTGGGTTCAAGCGAT
TCTCCTGCCTCAGCCTCCTGAGTAGCTGGGATTACAGGTGTATGCCACCACTCCCAGCTAATTTTTGTATTTTTAGTAG
AAACAGAGTTTCTCCATGTTGGTCAGGCTGGACTTGAACTTCCGACCTCAGGTGATCCGCCTGCCTCAGCCTCCCAAAGT
GTTGGGATTACAGGCGTGAGCCACCGCGCCCAGCCTCAAGCTCTCTATCTTAACCACATCTGCAAAGTCCCTTTTGTCGT
GTGAGGTGAAATCACAGGTGCCGGGGATTAAAATGTGTACATCTTTGTGGGGAAGGCCATTCTGCCTACCACATGGGGCT
TGAAGCCAGGCTGACTGACTTCAGAGCAGCTGCTCTTAAGTACTCTGTGCCGGGCACCTTGCTAGGCTTGGTGAAGAGGG
AGAAACTGATGATTGAGAAAAACTCTTGAGCTGAGGAAGCTCCGGGCTAGAAGGGCTGAGGTCCACCTGACTGTTGCAGA
AAGAGGGAACTTCAGATGAGAGGAGGGAGAAATCCTGGAGAGGTGATCCTTAAACGGTGGCTGGAATTTCCGCAGGAGGC
CATGGAAGACTTTACAGGTGAGTGAAGCTTGAACTATGCGTGGTCAGGAGGGACTGGAGTATGGGGGATGGGAATGATC
ATCTTACCTGGCCATGAGGATCAGATGAGCTAAAGACCTATGGACTTGGCATGTGACCTCACCTTGCCTTACCATTCATT
TTACAGTTGGGGAAACTGAGGCCTAGAGGCCAGAGTTGCCTGCCCAAGGTCACACAGAGAGAAAACATCAGTTGCACCAG
GAAGAAGCCTAGTGGGGAGGTCAAACTGGAGCAGGGTAGTCAACTGATAGGGGGCACTGAGGGCAAAGGCCAGGCTCCTT
CCTTCCTCTTCCCTGGAGTTGGCAGGCACTGGCTTCTCTGGGGTTTCTTTTCTCATGCTTTTTCTCTTGCAATCTCCCT
AATGGCCACTGGATGGCGCCGGGAGCACTGATTTGGAGCTGGGTCCTAGAGCCTTCGCAGGCCGTTGGCATCTGGCACTT
GGGCTGCAGAGCCTGTCCTCCCTTAATTCCCCGACTCTGGCAGCCCCACCACCGAAGTCTACAAGCTGCTTCTGTTCCCA
TGCAGTGGCCCCATAGTCCTGGATTTGAAGACCAGCCCTGCTACTTACCAGGTGTGTGTTGGTGTTGACAGGTACTGGTT
CCCTTTCTCACTTCTTGATTCCTTTCTCTCATTTCCCTTTCCTCCTACCAGCTTAACCCTGCTGGATACAGGGAATTCCT
GTTCCCTAACCATGCCCCACATAAGGTCAGCTAGCGCTTGCTTCTTTGTTTATACCCCCATCTTACGTTATGGGATAGAA
GACTTACAAAGGACCCGTGAATTCAGGAAAACAAACATCCATTAACTCAAGAACAGAAAGTGAGAACCCAAGTAACAGTG
TGAGGAGAGTAACACTGCCAAGAAAGCTTAGATGGAGAAGGCACCGAGCCCGAACAAAGACCCCCGGGCCCGGGTCTTTT
GGTACCCAGGCAAGCACGGGTTTATGTGGCTTTCAGAGCCTGTTAAGATCAGGAATAAAGCACGTAGATATTGGGGGAGT
CCTGAGCCCAGGTGTGTGGAGAGAACTCAGCGCCCTCAGCCTTCTTGGTTCCCTGGAGAGTTTGAATCATTAAAAAACGA
CAATATCTTCCCTAGTGGTTAGCAGAGATGCCACTTCTGGAGCTGTTTCTTAGACTTAGTAACATGAACTAGGCATGGCC
TTGGGTGGGAAAGGGCAGGGCAGGAAGGGCCTGTAACTTCAGCCAAGTGCTGGACTCGCTTTCTGAATTTCACCTCTGTG
TTGTCTGGCTCTGTTTCTCCGTGGTTTTTGGCCTTTCTCTTCTTCTATTCTAGCTCACAGCTCTCTCCATTCTGCAGCC
GGCACAGCTGCTGTCTCTAAGGACTGTCTACTCTTCTGGTTCTTTGGACCTAGGATAGTGAAACAGGAGAGAAACTATC
AATTACCAAACACTGGCCTGCATGCTGGGGGCCAGGCTGTGCCTTTCATCTGTGTTGTCTCGTTGAAACCTCTCAACCTA
CAGGAGGGAAAACTGAACTTGAAAGAGGCCAGTTAGTTGCCCAAGGCCCAGAGCTTCCAAGTTAGGGTTTGAACCCAGGT
GGTCTTGATCCCAGAGTAGGCCCATTTCACATTTCTACCCTTCAGTTATTAGGGACAAATTTGGACTTTTTCAAAGAAAC
CTTTTTCAGGAAGTTTATGGAGTAGACCAAAGCAAAGCCCGAGATACAGTGCAGGTGAATGGCAGGAGCCGCTCTGCCGT
TTGTCAGCAGCACAGCCTCCATGGGACACTTTTGCCTCAGTGTCCCCTCTTGTAAAACCTGTGAGCAGGCCAGGCATGGT
GGCTCACACCTGTAATGCCAAGCACTTTGGGAGGTTGAGGCGGGCGGATCACAAGGTCAGGAGTTCAAGACCAGCCTGAC
CAACATGGTGAAGCCCCGTCTCTACTAAAAATACAAAAATTAGCCGGGTATGGTGGCATGTGCCTGTAATCCCAGCTACT
```

```
CAGGAGGCTGAAGCAGGAGAATCGCTTGAACCCAGGAGGCAGAGTTTCAGTGAGCCAAGATCGCGCCACTGCACTCCAGC
CTGGGCGACAGAGGGAGACTCCGTCTCAAAAACAAACAAAACCAAGAAACAAAACAATAACAACAACAAAAAAAACCTGT
GGGCAACGCCAACCTCATAGGTTAAAAGGAAAGAGCGAAGTCATTGATGAATGTGAACATGCACAGTGGATGATCAGGAA
GGGCCCCTTTCTCTGCAGGCCATTACTTGCCCTTTTTCCACACCCTCCCACTCATTTGAAGTGGCGTGGCTTCAGATCCC
CAGGCCCAATGAAGAAGTTCGGCCACATCACACAATGAAAAGCAAATTGCTGTTAAATACACAGAGGAGGGCATAAGCAG
CTCAGCTTTATGGAGCCCAGGAAGGCTTCCTGGAGGAGGTGGCAAGTGAGCTGCACCAGCAAGGAAGGGTAGACATTCCG
CAGATGATAAAGGAGGAGAGGGCTCCTGGGCACAGGACTCGAGTGGCTTTTCAAGGGCATGTGGGGAGGGTGAGGCTGGA
GAAGACAGTCGCCCTCAGTCCCCCAGGTGGATCATGCTGCATCCCATGACAAGGGCTTGTGGGTTAGTGGTTCTAACCCA
CAAGACTTCATGGACTTATGAATCACCTGAGGGTCTGTTAAAATGCAGCATCCTGCAGTGGGGCCCAGGAATCTTTGTTT
CTCCCAGCTCCTGGGTGACACTGCACAAGTGATCAGAGGAAGAAAGGAGACACAAAGCTTCTGGTGATGGACATGAGTGC
TGGGCAAGGGCTCAGTGTCCTCCACCAGGCAGGGGAGCAGACTAGGGTCTCAGGATCTTGGGTGTGGGCGTGTACTAGAC
CTTGCGCCCCACATGAGCGGACGTGAGGAGCTAAGCTGAGAAACAGCAAATGGGAAAGCAAATGTGAATGCCAGGGCAAG
CATTACAAGTTTGTTAGGAGTCACCAGGGGCTGGGCAGAGAAGGCAGGTTCAGTCACAAGGGTTTTAGTCCAGAGACATG
TGCTGGAGAGGAGAGGGAAGGCTGGAAGGGGAGGTATGAGGAGCAACTGCTGGGAAGCAATTTTTTTGTTGCGGGAGGTG
AGTGGCAGCCCTGGGGCTTTGGAAAAGGTGATAAAAAAGAACATATACTAAGGGCCTGAAATTCTAAGGACTTATGAAGA
ACCCATTTAATTCCCATAACCATCTTAATTAGAAAAATATATTGTTATTCTTAATTTACAGACAAAGAAACCCAGTCCCA
GAAAGTAATTTGCACAGCAAATAAGCCGTGGAGCAGAGACTTAAGTCCAGACTGTGTCAGTCCAGAGCTGGGCACTGATT
CTTCATTCTAGGGTATCCCAAGCTCATTTAAAACTCCCGGATCCATGAGCCACTGTTTCTTTCTGTTCTTCCTCCTGCCA
TGGACAATCCCATCTCCAGCCCACCTCATCCCTTGCTCTGCTCTTTCCCAGGTCCAAGATGGTTCTTTTTCCTGAAGCGG
TGAATGGAACTGCTGCTGTTTATCCCTCTCTAGGCAGCCAGGGCATGCGTGTTCTTCCCAGCAGCTGAGATGAGGATGGA
AATTAATAGAGGATATAGACAGAAAAAAGCTAACAACTAACTACACTATGCAGATAATAATTGAGGTGTATTTTCTCCCA
GATGAACAAGATAATCTGAGTGACCCCTGTTCCCTATTACAGGACTCCAATTCCTAATCTTGGCAGTTGCCCCCCAAAAT
ACCCTCCCAGTAAATGTTTCTTGAATACCTGCTTTGGGAATATGTTAGGTACTGTGCTGAACACTGGGGATTCAGTGGTG
AAAAAGAGAGTCCTACACTGTCTTTAAGAAACTTGTGTTCTAGGAGAGGGCAGACAACAAGTCAGTAAATGAATAAGGTT
TCAACTAGTGATCATTGCTGGGAAGATGATGAAATTGAGTAAGGGTAGAGAGCTGCGCTGTCCACAGGATTTCCGTGATG
GTGGAAAGGTTCTATATCCTTGCTGTCCAACATAGCAGTCAATGTGACTAATGTGACCAAGAAACTGAATTTTAAGTTCT
GTTTTAGATAATTTAAATTTAAATAGCCCCATGTGATTTGTGGCCACTGCGTCACTCATCACAGGTATAGAGGACACCTG
GAAAGGGGAGAGGTGATTAAGTGAGGTGGTCAAGGGAGACTCCCTGAGGGGGCGAAATTTGAACTGAGGTCTGAGAGGGA
GGCAGCCTTCCATTGTTCCAGGGGGAGAGAGTGCAGATGTGGGGGCCTTCTGGGATAAACAAAGCTTGAAGTTCTGGGGC
GGAGAGAAGGCTTGTGAGCTGGCATTCACTGGGTGAGGTAGTGTGGTGGGAGCTGATGGTGGAGGGGTGGTGGGTACCGG
ATAACTGGGGCATGGCAGGCTGCGGCGAGGTGGGCACCCCTCATTCCCCTTAGCAACACAGCAGGTATAGCTGCCCTGCC
CTTGCCTGCCCCCTTCTCTCTCAGGGCTGTGGGGAGGACTAGATGAGACCAGGTAACGTGCACATGCTCTGTAAGTTGTA
CAGAGCTGCCAGGCGATGGCTCATGCCTGTTAATCCCAGCACTTTGGGAGGCCGAGGCAGATGGATCACCTGAGGTCAGG
AGTTCAAGACCAGCCTGACCAATGTGGTGAAATCTCATCTCTACTAAAAATAAAAAATTAGCTGGGCATGGTGGCGGGTG
CCTGTAATCCCAGCTACTCGGGAGGCTGAGACAGGAGAATCGCTTGAACCCGGGAGGTAGAGGTTGCGGTGAGCCGAGAT
TACGCCATTGCACTCGAGCCTGGGCAACAAGAGCGAAACTTCATCTAAAAAAAAAAAAAAAAAAAAAAAAAAAGTTGTAC
AGTGCTGCCCATAAGGGTAGAGCTGTGGTTCTGAGTGTGATCTTGGACCAGCAGCATGAGCATCATCTGGGAACACGTTA
GAAATGAACATTCTTGGGCCACACCCTGGATCTACTAGTTTAAAGCTCTGGGTGTGGGGCCCAGCAACGTTTCCAAAAGC
CCTCCGGGTGGCTGTGATGCAGGCTCAAGTTTAAGAGCAACTGCAGAAGACTGGCGGCAATCACCTCCTCCTCCTCCTTC
TCATCTCAGGCCCCCAACAAATTGTCCTTCCAACTCCAACCCTGGCAGAATTCCTTGGTGGCTCATTGCGCGCCCTAGCC
CTCCTTCCCTCAGAGCTAGCTCAGCTTTTGGCTTCACATACACAGACTAAAACAAACAAACAAAAATCAGATAGATAAAA
CCTTTGTATTAAAAATATTTTCACCATACAAGCCGTCCAGGGTCGTCATTAAAATAAAACTTAGGCCAGACACAGTGGCT
CATGCCTACAATCCTAGCAGTTTGGGAGGCCAAGGTGGGCAGATGGCTTGAGCTCAGGAGTTTGAGACCAGCCTGGCCAA
CATGAAGAAACCTCATCTCTGCTAAAAATACAAATATTAGGTGGTCATGGTGGCATGCACCTGTAGTCCCAGCTACTTGG
GAGGCTGAGGTGGGAGGATAGCTTGAGCCTGGGAGGCGGAGGTTGCAGCGAGCTGAGATTGCACCACTGTACTCTCGCCT
GGGCGACAGAGTGAGACTGTCTCAAAAAATAAATAAACAAACGTCTATAGCATAGAACCGATCAAGTGAAAAAAGATCC
CCTGCTTCCGTTAATCCTATTCCCTAGAGATTACCACAGAATGATTTCTTGTGATTCCTTCTAGACGTCTCCTATGCCTG
TAGAGGCAGCTAGTGTAGTGCTCCAAAACCAGTTTGTTTGGTTTAAAATCCCAGGTTTGCTTATTTGTCAAATAAATTGG
ACAAGTTGTCTCAGTGTTGTCATTCAGAAAATGAAGTTAATAATAGCACCAACCTCATAGAATTGTTGTGAGGATTAAAT
AAATTCAAATAAAGCAATCAGGAGACAATCCACTTAACATTCTTACAACAGTGCCTGGTATATAGTATGTACAAACATGA
GTGTTTGTTAATAAATAAAATGCATGGCTGAGGTTTAATTATTCTATGCCTTTTCCTTGGGGATATAGTCTTGAGAGAAT
AAGCCAAAGGTGCAGGAACAGTTGGAAAGCCATTTACGATGGGGATGATATTGAGAGCCACTGAAAAAATCCTGACCAGA
TGATTCTTGCCGGTGACCTCGGCTGACTTCCTTGGTTTCTGATCATTATTTCTTATTTTATTTTATTTTTATTT
TTTTTGAGATGGAGTCTTGCTCTGTTGCTCGGCTGGAGTGCAGGGACGTGATCTTGGCTCACTGCAACCTCCACCTTCC
AGGTCCAAGCGATTCTCCTGCCTCAGCCTTCCGAGCAGCTGGAACTACAGGCACACGCCAACACACTCGGCTAATTTTTG
TATTTTTAGTAGAGACGGGGTTTCACCATGTTGGCCAGGATGGTCTCGATCTCTTGACCTTGTGATCCGCCTGCCTCGGC
CTCCCAAAGTGCTGGGATTACAGCCATAAGCCACCATGCCCAGCCTCTAATCATTATTTCTATAAACTCCCTGGTATCTT
TGCAAAGCTTCTAAGTTTCACAGTTTCAAAAAACATAGCAGGAGGGGGGTATTTACATAGAACCCAGTGTGGATGGTACA
CCCTGGACTTGTGAAATGGTCTGTCTGATATCAGCCAGGTGTGGCATGTCTGTTGCTTGCAACCAAGAACTCTGACCATG
CCTTCCTCCATTCTTTCTAAGGGCTGTGTAGTATTCTACTCTATGTATGTGTTGTATTTCACTTAACCATTTCTTATTGA
TGGATGTCTGGATAGTTTAGCTATTCTACACTACACAGACAAATGCAATGACAAGCTTCCCAGAGCCTATTGTCCAGCCC
```

```
CCACCTGTACCCTATTCCCCCCGCTTTTTTTTTGGAGACAGGGTCTTGCTCTGTCATCCAGGCTGGAGTGCAGTGGCACA
ATCACAGTTCACTGAAGGCTTGCTCTCGCAGGCTCAAGTGATCTTCCCACCTTAGCCTTCTGAGTAGCTAGGACTACAGG
TGCATGCCACCACAACTAGCTATTTTTTTCATTTTTTGTAGAGATGAGATCTCACTATGTTGTCCAGGCTGGTCTGAAAC
TCCTGGGCGCAAGTGATCCTCCCACCTCAGCCTCCCAAAGCTCTGGTATTATAGGCATGAGTGACTGCATCCGGCCTGAA
ATGGGCTCTTTATCTTACATGATCCTGTGTAGTGCTCATGCCAACACTGTGAAGTTTGCATCAACACTTTCCCTCTACAG
ATGAGGCAGCTGAGGTCAGAGAGAGGATGAGATATGCCCCCAAACCATCAGTGAGCCCCTTGATGAGCAGGGCTCTTGCC
ACTTCATCAGGGTGGTCAGTCGGGGGGAGAATGTTTGGGATGAAAGCGGGGGCCTTCCCTTGACTCAATACCAGCTGGCA
AAAGGCAGAGGTTCTTGGAGGAGAGAGGTAAGTCAGGGAAGGACTCGTGGGAGTGCTGGGGAGCCTTCCACTGTCAACTC
AGCTGCTTTCCAGAGGGCAAGAGGAGAGAGAGGGTGGAGGACAGTTGGCATGTGGATTGAGGAACAATAATGATGATGAT
GCTAATAATCACAGTGACTAATGTTTTGGGCGAGTCACAAAGCAATTTCCTATTATGAATAGTTCAGCAGAGCCACACAA
CCATCCCCTTGGAGGAAGACAAGGCAATGTGGCTACTCCCATTTTGCAGAAGGGAAAATTCAGGACTGGAGAGGGAGAGT
AACTTGCCCAAGTTTACATGTCAAGATAATCGCCATGCTGGGTGGGGCCAGACCTTTCAGGTCCCAGCCCAGGATCTAGC
TCCCTCCCATGGGCCACAGCTGTGGCTGGCAGAGAGGTGCGGCTTTTGTCAGGGCTGAAGCTCAGCCACACCCTCTCTCC
AGCCTCAAGTTGGGTGCCAGGCAGAGGGTTTGGACTGAAGGCTG
```

HUMAN mRNA SEQUENCE : hR3-010.1 (Seq ID No: 11)
```
GAGTGTGGCTGCCCCGGTCTCCCCAGTCTCCCGCTGTAGCTGACTTCCAGCCTGGCCCGCAGCTAGCCCTGTCTGCAGCT
CCAAGCGTGGTAGGAGCAGGGGGTGCTGGGTTTGCCCCCCCTCAGGAGGAAGGGGGGGATCCCTCCCTGCCAGTGGGAGGG
GTTGCAGCCTGCACTTCCCCTCCCCACCCGCCAGCCCTGGGTTAAATGCGGCCGTTGCCTCTGTGTGCTCCAGCTGCCTG
GCAGTCCCCACCTAGGGCACGCTGCCACGCCGCCGTTACCTGGTCCAAGTGCCCGGGAGGCTCCGCCTGTCGGCTTCGCT
CTGCAGCTGCATCTCTGATCTGTCCTGCAGGCTCAGGCTCTGACACCTCCATTCTCTGTCCCCAAGCGCCATGAGAGGCC
TTCTTTGCTGGCCCGTGTTGCTGCTCCTTCTTCAGCCCTGGGAAACCCAGCTCCAGTTGACAGGTCCCAGGTGTCACACT
GGGCCCCTGGATCTGGTGTTCGTGATTGACAGCTCCCGCAGCGTGCGCCCTTTCGAGTTCGAGACCATGCGGCAGTTCCT
CATGGGCCTCCTCCGAGGCCTGAACGTGGGTCCCAACGCCACGCGCGTTGGCGTGATCCAGTATTCGAGTCAAGTGCAGA
GCGTCTTCCCTCTCCGCGCGTTCTCTCGCCGCGAGGACATGGAGCGCGCCATCCGCGACCTGGTGCCTCTGGCGCAAGGC
ACCATGACGGGACTGGCAATCCAGTACGCCATGAACGTGGCCTTCAGTGTGGCCGAGGGCGCGCGACCGCCAGAGGAGCG
CGTGCCGCGTGTCGCTGTCATCGTGACAGACGGGCGGCCCCAGGACCGCGTGGCCGAGGTGGCGGCACAGGCGCGCGCCC
GCGGCATTGAAATTTACGCGGTGGGGGTGCAGCGCGCGGACGTGGGCTCCCTGCGCGCCATGGCATCGCCCCCGCTAGAC
GAGCACGTCTTCCTCGTAGAGTCCTTCGACCTCATCCAGGAGTTCGGCCTGCAGTTCCAGAGCCGGCTGTGTGCCATTGA
TCTGTGTGCTGAAGGGACCCATGGGATGTGAGCACCACTGCGTCAATTCCCCAGGCTCCTATTTCTGTCACTGCCAAGTTG
GCTTTGTACTCCAGCAGGACCAGAGGAGCTGCAGGGCCATTGACTACTGCAGCTTTGGGAACCATAGCTGTCAGCATGAG
TGTGTTAGCACCCCTGGTGGGCCACGGTGCCACTGCAGAGAGGGCCATGACTTGCAGCCTGATGGGAGGAGCTGTCAGGT
CCGGGACCTTTGCAATGGCGTGGACCATGGCTGTGAGTTCCAGTGTGTGAGCGAGGGCCTCTCCTACCGCTGCCTGTGCC
CCGAGGGGCGGCAACTTCAGGCAGATGGCAAGAGCTGCAACCGGTGCCGGGAAGGCCACGTGGACCTTGTTCTGCTGGTT
GATGGCTCCAAGAGCGTGCGTCCACAAAACTTCGAGCTAGTGAAGCGCTTCGTGAACCAGATTGTGGACTTCCTAGATGT
GTCCCCCGAGGGCACGCGGGTGGGGCTGGTGCAGTTCTCGAGCCGCGTGCGCACCGAGTTCCCTCTGGGTCGCTACGGCA
CCGCAGCCGAGGTGAAGCAGGCGGTCCTGGCCGTGGAGTACATGGAACGCGGCACCATGACAGGGCTGGCGTTGCGGCAC
ATGGTGGAGCACAGCTTCTCCGAGGCGCAGGGTGCACGGCCCCGTGCCCTTAACGTGCCTCGTGTTGGCCTGGTCTTCAC
GGATGGCCGCTCCCAGGATGACATCTCGGTGTGGGCAGCGCGCGCCAAGGAGGAAGGCATCGTCATGTACGCCGTGGGCG
TGGGCAAGGCGGTGGAGGCGGAGCTGCGCGAGATCGCCTCGGAGCCAGCGGAACTGCACGTGTCCTATGCCCCGGACTTC
GGCACCATGACGCACCTGCTGGAGAACCTCAGAGGCAGCATCTGTCCAGAGGAGGGCATCAGCGCAGGGACAGAGCTTCG
GAGCCCATGCGAATGCGAAAGCCTCGTGGAGTTCCAGGGCCGCACGCTGGGGGCGCTCGAGAGCCTGACGCTGAACCTGG
CCCAGCTGACGGCGCGCCTGGAGGATCTGGAGAACCAGCTGGCCAACCAGAAGTGAGGGCCACGGACGGCCCAGACCCGG
GCTGGGGCGCGGCACCACGGACGGTGCCCCTTGCGCGCCATCGGTGCGCCGGGGCCAGGCAGAACCTGGGCCCGTCCGGC
TTGGGCTGTCGGGGCGGAGGCGCTGGCGGGCTTCCGGCATTGAGCTGAGTTGGCCTCGCCCGGACCATTAGGCGGACTGC
GGCGTCAGGGGGATAGCGGGTGGTGAGGGAAGGGGCACGTGCTAGACCGGCACGCCCTCGCCGC
```

HUMAN PROTEIN SEQUENCE : hP3-010.1 (Seq ID No: 12)
```
MRGLLCWPVLLLLLQPWETQLQLTGPRCHTGPLDLVFVIDSSRSVRPFEFETMRQFLMGLLRGLNVGPNATRVGVIQYSS
QVQSVFPLRAFSRREDMERAIRDLVPLAQGTMTGLAIQYAMNVAFSVAEGARPPEERVPRVAVIVTDGRPQDRVAEVAAQ
ARARGIEIYAVGVQRADVGSLRAMASPPLDEHVFLVESFDLIQEFGLQFQSRLCAIDLCAEGTHGCEHHCVNSPGSYFCH
CQVGFVLQQDQRSCRAIDYCSFGNHSCQHECVSTPGGPRCHCREGHDLQPDGRSCQVRDLCNGVDHGCEFQCVSEGLSYR
CLCPEGRQLQADGKSCNRCREGHVDLVLLVDGSKSVRPQNFELVKRFVNQIVDFLDVSPEGTRVGLVQFSSRVRTEFPLG
RYGTAAEVKQAVLAVEYMERGTMTGLALRHMVEHSFSEAQGARPRALNVPRVGLVFTDGRSQDDISVWAARAKEEGIVMY
AVGVGKAVEAELREIASEPAELHVSYAPDFGTMTHLLENLRGSICPEEGISAGTELRSPCECESLVEFQGRTLGALESLT
LNLAQLTARLEDLENQLANQK*
```

Table 3

MOUSE GENOMIC SEQUENCE : mD3-027 (Seq ID No: 13)

```
ATACATTATAAACTAGTAAACTAAGCCTCACAGGTTGTACACACATATCTGTATTGTTAGTGTTGCTGTTATTATTATTG
AGACAGGGTCTCTTTATGTAACCCTTGTTATCCTGGATCTTTCTATGTAGACCACACTGGCCCGGAACTCACAGAGATCT
GCCTGTCTCTGCCTCCTGAGTACATCTGTATTTTAAGTGTTGTTTTTCCACAGTTTGAGACATATTCAGTCTCCTTTGAC
TCTGTTTCCCCCACGAGCCTCCTGTATCTTCTCCCTTCCCTCCAAACCCCTTCTTCCCAGTGAATCCCTTTTCTGCTTTC
ATGTGTGTCTGTGTGTGACTGCACATGCACACATGTGTGAGTGTGTTTAGTTGGGGTTTCTGGCATGAGTGTGGGTGAGG
AGTTATTTACCTAAACAAAGGCAACTTAGAGTGTCTAAGTCAGCACTGAGGACTCTAACTCCTCCTTCTCCGGCAGCCAT
CGCCTGCCTATGGCACCTCCGAGAGGGACAGGCCTCATGAGGTGACACTATCCATGCCATACATGACATATATTCACACA
TGAGAGGATTTGATGGACCCAGCCTTGTACAGATCTACAGTGAGTTCATGAGTACCATAACCATGCCATGTATGGAAGAT
ATAAAACTCATAGAAACGCCTTCCCATAGAACGCCATCTTACACTCTAGCCATCCTTCCTTCTGAGATGTTCCCTGAGTC
TCAGAGGTGGTGATGCTGCGGTCTCATGTAGGGACACTTCGACCAGCACATAGTCACCACATTCACCATCACCTACTACA
AACAAGAGCTAGAATTTTTTTAATTTCTAATTTTGAGCCCCAACGTATCTTATATATGTGAATCCATAAAAGGAATTTGC
CTGAGTGGCCATCATTTTTTGAAAGGTGCAGTTTACGTGAGCATGAGCCTTTAATCCTATCACTTAGGGAGCAGAGGCAG
GTAGATCCTGTGAGTCTGAGGCCGGCTTATCTGCAAGTGAGTTCTAGGTCGGTTAGGGTTACATAGTAAAACCCTGACTT
AAAACAGGCAGGGAGGCAGGCAGGCAAGCAGACAGACAGACAGCAAAAGAGTGAAGTAGGGGAGTAAGAAGGAAGAAAT
ATGAGTATTATATATACTAAGTATTCTTTATAATGTTTATTATATGTTGTGCTGGATAGTTTCATGTTGATGACACAATC
TAGACTCATTTGAGAAGAGGGAACCTCAATTGAGGAAATATTTCCATAAGACTGAGCTATAGAGACACTTGTAGGGCATT
TTCTTATTTAATGATGGGCGGGGGTGAGATGGGGGGCACCTCTGGCCTAGTGGTTCAGGTTCTCAGGCTAAAGAAACCAT
GAGGATTAAGCCAGTAAGCAGCATCCCTTCGTGGCTCCTGCATCAGCTCCTGCCTTCAGGTTCCTGCCTGGTTTGAGTTC
CTGCGTTGGCTCCTCTCAGTAGACTAGGACTCAGGATCTGTGAGCTTAACAGAGCCTTTCCTCCTCACACTGCTTTTGCT
GATGGCACTTTACCACAGCAATGGTGACCCTAACTAAGACAGAAGTGTATGCACACCAAGCAGGCTGCATTGAATGCATG
TCTCACTGTGGGGCACAGTCACAGTGACAGGATACATGATGTATCTAACAGGGACTAAACACTCAATTAGAGCAGAGGCT
GAAGCTGATCCCAGTGGCAGACACTTGGAGTCTCAGCATGTAAAAAACAGAAGCAGGAGGGATGCCATGAGTTTGAGGCC
AGCCCAGGTAACAGGCAACAGCGTGAGAGATCTTGTCAAACACACAAACCAGAATTACAAAACAAACTCAGAGATATGGC
TTAACAGATAAAGGTACTCACTGCCAAGCCCGAGGACCCAAGTAAGAACTGACTCCTGCAAATTGTCCTCTGTCCTCCAC
ACACATATGTAGAATGCTCATGCTCATATACACACACATACAAATACATACTTGCATGCTTACATACATACACACATATA
TGCATGCATACATAATACATACTACACACATATACATGCATGCATGCATACATACTACATGCATACATGCATACACATGC
ACACACATATATGCATACATATGGCAACAACAATATAGAGTGGAAATTAAAATAGACCAGCAAGGTATCCGAGGCAGAGG
CAGCAAAGTCTGTGTGGCATGATGGAAGTTTCAGCTGTTCCAGAGTTGGCCCTGGGGACACTCGTTCTGCGCAAAGAAAC
TGGTGGGTTTTGCTAGTTGTGGGAGGTGCTGTCCTTGTTCCAGAAAGGTCTCCATTATATAATGTGCTCTTTGTGTGGAC
AGGCCGAGAGCTCTGTGGGATGGGCTGGGAGGTCCAGTGTGGCATGCTAGCTGTGTCAGAACAGGGCACTGACTGGTGTA
TCTAAGTAAAGAGGTCCTTGTGGTGTAGGGCACTGGCTTTCTGAGTGTTCTTAGGACATATGCTAGACCCGTGACTCTAA
GGCCAGAGTCAACCACTCGGCTGGGGCCCAGACATCGTGCTTGCCAAGTCCTTCAGGCCACTGTGTCAAGTTCGAGAACC
GCTGGCTGTCTGAGGCATAACTGTTTGGTGTTTAGAGCATCTTAGAACACTGGGCAAAGCCTGCTCCTTGATGAACAGCA
GAAGTGCTGGGTCCTCCATCTGGCCTTTAGGTGCTGTATCTACAATCTTTCTCTCAGTCTCTCAGCCTTATACAGCACAT
TCACACACAGCTCTCCTGTGAGAGATGGCTGCTTGGCCCTTCATCTCAAGCTCCTTCCACCCTGTACCACTTAGCTGTGT
TACATTAACCTGGTACCACTGAAAATATTCTCAACACATGGATAGATACATATACAAGTGCATCCACAGGCTGTCCTGCC
CTCCCTGTTAGACACACTGCCCCACGTTGTCTCTTCCCTGGCCAGTGCTCACTTAGTACTCATCCGATAAACACCTGATC
CAGGCATCTGACTTGCTCTCCAGGCTGAGGTGCCCAGATACACACATAGTCCCTGTAACCTTACGGTAACCTTGGTGTGC
CAACCCAACACCTGGCCTGTTTCCTCCCTGCAGACTACTTACTACCTGGTACCTAAAGACAGCAGAGCTGTGTGTGTGGG
AGTTGGCTCCCAGCAATGACAGCCTTTATTGCCCACTTCCTGTGTTGACTGTAATGTGCTTTTGGGTCCTGTGCCTGAGT
GAGCTGCTAACCTTCATACTGCTTCCACTGCTGTCTGAAGCTCAGTTTCCACGTCTGTAATGTGGTGATAACATGATTAC
ACCTAAAGGCTGTTCAGAGGAGGAAAGGAAGGGAGAGTGTAAAGAATTTAGCTTGATGTATGGAACACAACACTAAGAAA
CAGCCTCTGGAGGGGTTAGCCATAGTTTTCCCCTGAGACAGTCAGGAAAGTACAAATCCTTTGCAAATCGTGTGGCCTTG
TGCGGAGTTAGGTTTGATGCCTATGGAGCTGCCTCCAGATGCTGGCTCTCTAAACCTTTCTCACTATAATCATGAGAAGA
AGAAAAGAAGGAGGAGGAGAAAGAAGAAGAAGAGGAGGAGGGACAGAAGGCACTGTTTCTCTACCTTCCAGTCCCCAACT
ATGGCAGGCACCATGCCACTAACTTCTAACACAGATGTTTTGACAGTTTTGTATGAAGAGTGAATGTTAAGGGTTGGGCG
GGCAGCCTGATATACTGGCCTAGCTTATGGGCAGGACTTGGTTTGAACCCCTGGTCCTAGAGGGGGAAAAGAGTGTGTTG
GGCATGGTGGCGCATATCTATCACCCCAGCATTCCAGAGCCAGAGGCAAGTGGGTCTCTGTGGGTTCAAGGCCAGTCTCC
TTTACACAGTGAGGGCCTATCTGGAAAAGAAAAGAAAAGAAATAAGACGGACAGACAGATACAGTGGAGTATAAAGAC
TGAGAAAAGCTAAAGTTGAGAGACATAGGGCTGTGTAACTGTTGAATGCTGTAAAAATCCAAGGTTGATTTTCCTTAAGG
ACTTAGAGGGGCCCTACTCTTCCTGAGGCTAATGAGCTGTGAAGTGTTACTGCTCACCACTGAAAGAGACCCAGAGGAAG
AACAACGAGGTCTCTGCCCAGGAACAGAGAAGAGCACTCCCAGTTAATAAAAGGCTGAGGCAGAGGGGACCCATTCCCTC
CAGCAACGCTCTAAGCCTCTGTGGCACAAGTTAATTTCTCTGTGGGTACAAGTGATTATAACTAGCAGCTGGAACAAATG
AAAATGAAATACAATCTATTAACAGCGAGGCATAATTACTGAGAAAATTCCACCTGAGCTGGGGCTTAAGCAGCCACCCA
CACCTAAGCCAGACACAAATCTGGCAAAGCCACCACTGGTCACTTCAGTGAGAAGCCTGGGGTAGAATAATTCAGACTTG
AGAAAGCTGTCCACAGGGTAGAAATTGAGAATCAGAAACAAACTGCAAATTGAAATGTAATCTTGGCTGTCTTACCTGGA
AGCACCCCTACCCCAGCTGACTATAACAGAAAATTGAAACCTCTGCCTTCATCTCTTTTAAGTCAAGGACCACAGCATAG
GCATTGCCTTCTCCATCCTGGAGAAGCTACTCTGGTTCTATGGGCTTGAAGGTTTCAGTTACCATAGCAACTCTCTGCAA
CCATTAGCCTACAAGAGCTAATTTTGGCTCACAGTTTTGGAGGTATCAGTCCATCACCGGTTAACACCATTGCTTGGGGC
CTATGGTAGCAGTATGTGGCAGAACCACGTGGCAGAGGAGGCCATCTGTGTGACGTACGGATGATGCTCTGAGGGGATCC
CTGGAACCCAAACGTCCCTCTGCTCTACCTCTGCTTCTGTTTGGAGCTTGGGTCTACGAGGCAGTGTCTTGCCCCAAACA
```

```
TTCAGGTCATCCAGGATGGCCCTAAACTCACAGACATCCTTTTGCCTCAGCATTCTATTATCTGTGTTAAGATAGAGGGG
CTGGAGAGATGGTTTAGTGGTTAAGAGCACTGACTGCTCTTCCAGAGGTCCTGAGTTCAATTCCCAGCAACCACACGGTG
GCTCACAACCATCTGTAGTGGGATCTGATGCCCTCTTCTGGTGTGTCTGAAGACAGCAACAGTGTACTCACATATATAAA
TTAAAAAATTATTAAAAAAATATTGCAGATAACACCGGGTGTGGTGGCGTACACCTTTAATCCCAGCACTCTGGAGGCAG
AGGCAGGCAGATTTCTGAGTTCGAGGCCAGCCTGGTCTACAAAGTGAGTTCCAGGACAGCCAGGGCTATACAGAGAAACC
CAATCTTGAAAAATCAAATATATATATATACATATATATATATGTATATATATGTGTGTATATATATATAACAGATAG
ATGTTCCTCCACACTGCTTTAAGCCCCATCTCTTTATTTATTGACCTATATATCTTATTTATTTTTATTATTGTATTAGT
TTGTTTCTCTGGTGCTGTGATAAGATGGGAGGAAAGGGTTTATTTGACTTACACATCATAACCCGTCATCCAGAGAAGCC
AGAACCTTGAGGAAGGAACTGAAATAAAGACCAGGAAAGAGCGCTGTTTGCTGACTTGCTTCCAGGCTCAGGTCTAGCTG
CCTTTTATACACCTCCCAGGACCATGTGGCCATCGCTGGCTCAGCACACAGTGGGCTGGGCTCTCCCACATCATCAGCAA
TCAAGAAAATGTCCCTTGGACCAGGCAGTGGTAACAGACACCTTTAATCCCAGCACTCAGGAGGCAGAAGAAGGTGAATC
TCTGAGTTTGAGGCCAGCCTGGTCTCTCATCTATAGAGGGAGATCCAGGATAGCCAGGGCTATAAAGAGAAACTCAGTNN
NNNNNNNNNNNNNNNNNNNNNNNNNNNNNNNNNNNNNNNNNNNNNNNNNNNNNNNNNNNNNNNNNNNNNNNNNAAAGAAAGAAAGAA
AGAAAGAAAGAAAGAAAGGGAGAGAGAAAGAAAGAAAGAAAGAAAGAAAGGGAGAAAGAAAGAAAGAGAAAGAAAGAAGA
AAGAAAGGGAGAAAGAAAGAAAGAGAGAGAGAGAAAGAAAGAAAGAAAGAAAGAAAGAAAGAAAGGAAGGGAGAAAGGGA
GAAAGAAAGAAAGAGAGAGAGAGAAAGAAAGGGAGAAAGAAAGAAAGAAAGAAAGAGAGAGAGAGAAAGAAAGAAAGAAA
GAAAGAAAGAAAGGGAGAAAGAAAGAAAGAAGAAAGAAAGGGAGAAAGAAAGAGAGAGAGAGAGAAAGAAAGAAAG
AAAGGGAGAAAGAAAGAAAGAAAGAAAGAAAGAAAGAAAGAAAGAAAGGGAGAAAGGGAGAGAGAGAGAGAGAAAGAGAG
AAAGAGAGAAAGAAAGAAAGAAAGAAAGAAAGAAAGAAAGAAAGAAAGGAAGGAAGGAAGGAAGTAAGGAAG
GAAGGAAGGAAGGAAAAGAAATGCCCCCTTGGGATTGGAAAGTTGGCTCAGCGATTAAAAGCACTTGTTCTTGCA
AATGACCCAAATTTGCTTCCCAACACCCACATGAAGGCTCACCACCTCCTTAGGAATCAGGCACATGTGTGGTGCACAGA
CTAACATTTAGGCAAAGTACTCATACACATAAAATAATAAAAATAAATCTGAGAGAGAGAGAACAAAGGAAAGGAAAGGA
AAGGAAAGGAAAGGAAAGGAAAGGAGAAAAGAAAGAAAGAAAAGAAAAGAAAAGAAAAGAAAAGAAAAGAAAAGAAAA
AAAATTGTTCCACAGACTTGCTTACAGGTCCATCTAATGGAGACAGTTCAACTAAGGTTCCTTCTTCCAAGTTCTGTCAA
GTTGACAACCAAGATTAGTCATCACAATTACATTCAGTGTAAGTGTGTGTGTGTGTGTGTGTGTGCATGCACACGCGC
GAGCAACACTTATGTGGACGTCAGAGGACAACTTATGGGATTTGGCTGTCTCCAACCATTTGTTTCCTGAGGCAGGAAAG
GGGAGGGGAGGGGNNNNNNNNNNNNNNNNNNNNNGAGGGGAGGGGAGGGGTGGGTCCTCACTCTTCCTGACATTCCCTCTG
AGTCTACAATGCAGCCTGAGAATCACTGAAGGTACAATGATTGAGAATCCTCAAGGAGACAGCCCCAGATCAATTCCAGG
CTCCACCCACCTCCTCACATTGCCATCAGCATCACCACACCCAATGCTGTGTCCACAGTGCACCACACACGGCACAATTC
TTTCTACACACTTTCTCATGCAGTCCTCCATCTCTCTCTGTCTCTCTGTTTCTCTCTGTGTGTGTCTCTGTGTGTCTCTG
TGTATCTCTGTTTCTCTGTCTCTCTGTCTCTGTCTCTCTCTCTCTGTCTCTCTCTCTGTCTCTCTGTCTCTCTC
TCTCTCTCTCTCTCTCTCTCTCTCTCACACACACACACACACACACATCTCCCTAATGTCCAGCTTAG
GTCCTACTTCCACACAGTCTTCCCCAACCTTTGAAGTGTCTAGCTATATGAAGTAAGATGCTGGTTTTGATTTTATCCTT
CCAAGAGTGGACTTGGTTAGACCTAGGGATGGCTTTTTCATGTTGTCATCACATCCACAGATGACAAGTTTCCCTTCATA
AAATCCAATGGTGGGTTAGGACCAACAACGTGAAGTCCATTAAGCACGCTGCCAAGGGTAGAAGGCACTACAGAAAAGGT
CACGCTAATTGCAACTAAATATGTTCAGAGTCCCGAACAAGGAAACTCGCCTTCCCGTGGTTTGTCCTTGTCACAGGCTT
TCTGGACCACTGACAGGAAATCACCTAAGGTCAAAGCACAGGCTGATAAGGCACCCAGAAACCAGCTATTCGAAAAATAG
TTGAAGACATTAGGATGTGGGTTGGGACACATAAGACTCACAGGCACATGATATCTGTACCCAGAGGTCACTTGGGAGAC
ATGCTACCTACACACACGGTCCTGTCTCGAGGCCCAGCTGGGGGTGTCATAGAAGATACATTTTGACTCAGCATGAAGGCT
CAAGTTTCTAATTCTAAACAGTAAACCATCCTACTCTGAAACATGATCTACCCTCCCCCTTCGCACTTTATTTTATGGAT
GGGTCTTATCACATAGACATCTTTAGCCTCAAACTCAGAATCTTCCTGCATCATCCTCCCAAGTGCTGGGGTGACAGACA
TTCACCCTCACAGCTGAGGAACCATGATTTCATGAGGCAGCTTTCTCCCCCTTGAGTGAGGATGCTTTGGTCTCCTTTCT
TGCATCCTCTGGAGAGGAGGGGTGGGCAGAGTGAGGCGTGACCGGCTTGTCTTTCAAAGTCCCTTTCCATTCCTTGGAAT
TTCTCATGCTTTGGTCTTTTCTGGACAGTGTGCTCAGAGCAGCAAAAGCTGACTTGTGTGTGTTGCTGGGCAGGAGGGGC
GTGACCGCATGGTGTAGGGTTGGAAGGATGAACAGTGCTTCGTCAGCTCCAGACTCAGGAAGCGCCGAGGCAGTGTATCC
CTGAGCAGTACAGGACCAGAAGAGCTGGAGAGGGGCTCCAACCCTGTGCCCTGGCTTTCTTCCTCCTTAAGAACAGTTTT
GGGGGGCCAGCAAGATGGCTCAACAAGGAAAGACATTTGCCACCAAACATGACTACCTGAGTCCCACATTCCCACATTGTA
GGAGAGAAACAACTTCCACAAGTTGTCCTCTGACCTCCATAGGCTCTCTCTGTGTGTCTATATCGTTGTCTGTCTCTGTC
TGTCTCTGTCTGTCTCTGTCTCTGTCTCTGTCTCTGTCTCTCTCTCTCACACACACACACAAATAGTGCCCCGTAT
GTAGTACCTACATAGTGTAGTGGGTACAATCTTACACTATCATGGACTAAGTCAGGAAATATCCTCCCACGGAAGAAAAC
CCACCAGGACAAGACCTGGAGGGGAAATGGATCAATGTCCACCACAGGTCTTCATGAACGAAGTGGCTCTCAGGATGGGA
CAGAGCAGGGGTGGAGAGCAGGGCAGAGAGAGCCTGCAGAGCAGCTATGATGGAAGCAGGGGATGACAAATGTGGAGTC
AGGACACTTGGGTGCAAGGTTACCACCATTGATGCGTTACAGGATCCTGGGTAGGTCAGTCTCCATCTCTGGGCTTCGGG
GTCCTCCTGTGTAAAATTGGAGCTGTGGGGGTGGCCTAGGTAACCATCCATGTCCTTTCAAGCTAGGTGTCTACAAGTTT
ACTTGTGGTGGAACGCCTTTTTGACCTTTTTCTCTGAGTCAGAGACAGCTCGATGGCCATATTTATACTGCACTCTTGTC
TGGTAATTTCATTTTACATTTTTTCTTCTAGTATTCTTAGGGTTCTTCCCTTTGAAAGAACTTAACTCTTTCAAACTTGA
GCTGAAAAGAGGGAGGGGCAGGGTTCCCCCTCCCTGGCCGGGCAAAAGGATGGCAGTCACAGGTTGATCAAGTGCCCTGG
CTCGCCTGGGACTGAGGGGTTTCCCAGGACAGGGGACTTTCTTTGGTTCTGGGCAAACTGGGATGGTTGGTCACCCTAGT
GATAAGGAAAGTGAGTGTGTAGAGGAGGCTGGGAGTACTCTTTTCTTAGCCCCTTCTATGAGGAGTTCCCCAAGTCTATA
TGCATCCCTCATAGAGATCCAGTGAGCAGAAGGGAGGGGCAGCCTTCACACCAGACGCCTGGCCCAGTTCAGACCATGAG
TGCCAGGAAAGGCACTGAAATGCTTGCGTAGTACTGACTCTGTGGGGGGAGTTTCCCTTTTTTCTTAAAAAAAAAGAAAG
```

```
AAAAGAAAAGAAGGGGGAAAACACACACACACACACACACACCAGAAAAGACTCAGTGGAAAAAAAAAAAAAAAAGAGA
TGAGTCGAGAGGCAAGCAGCAAGCCCTCACTGGCCCCAGGAGACCGCACATGGCTCATTTGTTTAAGTTTGCAGCTGACG
CCTGCCACCTCTCCATAGGCACCAGCACAAACCTTCTCGACATCAGACAGTGACCAGCCTGGTGACGCAGAGCTGCAGCT
ATGAACTACCCACTAACCCTGGACATGGGCTCCATCACATACAATATGGATGACCTGGTGAGTCGAGTCATGTGGCTCCC
TGCTGCTCTGGTCCTGCTTGGAACTTTAAATATACTATGTCAAGGAAGAGGGGATAGCTTGGGAATCCCGCCGCAAGGAT
TATAGAACATAGGCAAATACATCCACTTTTAGACTCTTATTGTCTCCCATATCTGTTGCCCTTAATAGATGCCAAAGGGA
GGTGGGAGACACAGGATCAGGTTTGGCATCTCCTGCTGTGTGACTGAGGACCCAGCTCTGCCTCTTGCTTCTTTAACAGA
GAAGTTTATCTTGTCTCCCTGAGTTAAAATGGCAAAGTCATTTGGAAGGGGTATTTTTGTTTGCTGCTTTTTTTTTTTTT
TTTTTTTTTTGAGACTGAGCTTCTCTGTGAAGCAAGCATTGGCTGTCCTGGAACTCTGTAGACCAGGTTCAAACTCACC
TACTACCTGCCAAGTACTGAGATTAAACGTGTGCGCTGCCACTGCTGCTGCCGCCGCTGCCACCACCACCACCTGGCTGG
AAGTTTTCTTCATGTCAGTTTGCTGGCAGTGCTAGTCAATCAGGCAGTGTGGAGTTGTTGGCTGGGAGAGGAGGGGTGGG
CAGAGTACTCAGTTGGTAGAGTGCTTGCCGAGTGTGCATGCTGCTAGGCTCATCCTCAGCTTGCACAGAGTGTGGTGGTA
CACACCTATAATCCCGGCATGAGAAAGGTGGAAGCAAAAGACTCAGGGGCGGGAGCCTAGAACACATTGAGACCTTGTCT
CCAAAAGAAGGTATATATAATTGTTCATCTTGCTGAATGACTTGATCATTAAGGCACAATACAACATCCATCTGTGTTGG
GTGGAGAGACTGAGAACATAATCTTTGCCCTCAAGTCGCTGATGGTCCAGACAAGGTGGAGCTAATCCCTGGCTGGAAGG
CTTTAGACAGTGATTTTGTATAGATGAAGAAAGCATGAGGCCAGACGTGGCAAGGAAGAGGACCCCAGGGGTCAAAGTCA
TGCAGCTGTATTGCTCCTAAAAACACCTTGGAGCATGTCGCTTTGCCCTGGGAGGGACTGTCATCTCTAGAGATACAGTA
TCTTGGAAGAGCTTCTTAGGTGGGAGCTTAAGAAAGAAACAAGCAAAGAAAGGAAGCAGTGCCTGGCACCCTCTCCGCCC
CCTGCCTGAGTGTCTTCCAGTTAAGCATAGGAGTTCACTTGACTTGGCTGCTATTGGACTACCAAAGGATGTTGTTAGGA
GCACTTCAAGTGGAAAAACCCAGGGCTGTGGGGCAGTGGGTTATGGGCATCAGTAGACTTAGAACCTTCATCATGACCTT
ATTCTCTATGAACTCCAAACCCAGAGCCCTATCAGCTCTCCTGCCCTAATCACACCTTGAGGTGTTTCCCCCCGACTCTC
CCAGCTCGCCAGGATCTGTGGTCTGCTTTATCATTCTTCCCACTATGTCGCTGGGCTTGCTGAACCTGCTCAGCTGGCAT
CACTTGGGCACTCATGGGGATTCTGCAAATAAAAGGACCTCAGGCTTGGCTCAATGCTTTCTGTTGTTGTCTTGAAAAAA
TTTGGGGGGTGGGGTTGTTTGTTGGCTTGTTTGTTTGAAACAGAGTCTCTGTGTAGCCTAAATCTGCTTCAAACTCATAG
TCCTCTTGCCTGAGTCCTTTGAAAACCTAACTGTTCTACAGGGGAGTGTGCTCCCACGCTTGCTTGCCCTCATCCCCACT
GCCAGCTGCCCCTGATATGGTTAGGTGGTATCTTCTGCCCTCAAGCTGACCATCAGCCCTGACCCACTTGGATGCCTTTG
TAACCACCTGGAGACCTGGCCGTTCCTGGCTTCTGAGAAACCTCAAGAGTTTCCCCTAACTGCACGCCTCACATCACAGT
GTGACCTACTAGAGACAGGTGGTAGTGGGGGCCAGAGGGACAGCAGGACGTGCTCATGACATGAAGCAAACCTACATTCC
AAGGAGAGAAGCAAGGTAACTTCTTTCATGACACATATCAAACCCAAGGCCCTGAGCTATGTGCCCAGACATCTCGCTAG
GAGTCCAGGGGTTCTAAGACAACCCTGTCCCTGGAGTCCTTCAATCAGACACGTTCTTCCTTCACCCACCACCCACTCTC
TTCAAGGGTGGTTGTCACTCTGAAACACAGATCCTCCTCTTTGGCCCCACTCACCTTCTAAAAGGCCATGACCGGCCTTG
GCATACTGCCCTATTCTGTCATAGCATGCATGTCCTGGAAAGACAGAACTGGCAGAAAGTTTAGCATTAGTGGCTTCTC
CATAAAACAATGGGTTTCTGTTTTGCTCTGTCTGTCTGTCATACTGGACAAGACAGTATGACAAGGCCTCCTGCATCCCC
AGCTCTCCCAAGGAGCACTTCCTGCCCTGTGACAGACACTTGGAAGGACTGCAGGCTTGGCTCAATGCTTTCTGTTGCTG
TTTTAAAAAAATTTGGGTGGGCGGGCGTGTTTGTTGGCTTGTTTATCTGAAACAAGAGTCTCTATGTAGCTAAATCTGCT
TCAAACTCATAGTCGTCTTGCCTGAGTCTTTTGGGTACTGGCATTATAAATCTCTGAGGTCTTAGCTTGACATTTTTCTT
AGGCGTCTGCTGAGCTTCTCCCATTGACTTTATTTTCTGCTCCTCACTCCCTGTCTTTGACCCAGCTGATTACTGGCCCA
CTTCTCCAGATCAGAAGGTTCAACACTCCCAACTCTGAGTTCTGAAACAACAGTCTTGATAAGTCAACCATCCCTTGTCT
TAATTTTAATTTTCTTTTTACATTTGTTTATTTATAGTCAACAGAGGATAACTTGTAGGATTCAGTTCTTTCTTTCCAGC
ATGCGTGTTCTAGGGACTTAACTCAGCTTTTTAGGCTTGATGGCAGGTGCCCATTAAGTGTCTCACCAACTACTCTTTT
TATTATTTTTAATGACAACTGATTTTGAGACAGGATTTTTTTCCCCTTTGCCTTTTTGAGACAGGGTTTCTCTGTGTAGC
CTTGCCAATCTTGGAACTTACTCTGTAGACCATGTCGATCAGGCTGGCCTAAAACTTAGAGATCCAGGTTGGAGAGATGA
CTCAGTGGTTAAGAGAACTGACAGCTCTTCCTAAGGTCCTGAGTTCGAATCCAGTTAACCACATGGTGGCTCACAACCAT
CTGTAATGAGATCTGACACCCTCTTCTGGTGTGTCTGAAGACAGCTACAGTATACTTAGATATAATAATGATACATAAAT
CTTTAAAAAAACAAGAAACAAAAAACTTAGAGATTCGCCTGCCTCTACCTCCTTATTGCTGGGGTTAAAGGTACGCACCA
CCACTGCCATCTGAGACAGGATTTTTCTAAGTAGTTCTTCCTGTCCAGAAACTTGCTATGTAGATTAAGCTGGCCCTGAA
CTCAGAGATCTGTCTGCCTCTGATTTTCAAGTGCTGAGATTAAAGGTACGCTCCACTACACCCTGTCCATGTAGACCAGG
CTGGCCTCGAAGTCAGAAATCCTCCTGCCTCTGCCTCCCAAGTGCTGGGATTAAAGGTGTGTGCCACCACTGCCCGGCTA
ACAGCGAATTTTTAATTGTTGTTGTTGTTGTTATTATTATTATTATTTATTATTATTATTTTGTGTGTGTGTGCGTG
TGTGTGTGTGTGTGTGCGTGTGTGTGTGTGTGTGTGTGTGTGTGTGTGTGCCATGTGAGTGGAGGTCAGAGAGCAACG
TTTGAGAATTCCTTCTCTCCTTCTTCCATGGGTTCCAGGGAGCAAGCTCATCATTTGTCAGTTGTCAGGCATGTATAGAG
AGCACCTTTTCCCATCGAGGCTCTTTTGTCCCTTGAACTCAAAGATCACCTTTGATCTCCCACACACTTGCTGGCCATGG
GCAGCTACTAAACTTCTTGATGGCTAAGAGCCAGGAGAAGAACCCTAGCACCTAGAGTAGGCATGGCCAGAGGTCACCCC
ACTCTGGCGAGGTGGGCCCTGGTGGCCCTGGAGGAGATCCTGTCCAGGAGAACCACCATGAGTTCTTTCCTGTCACTCCT
CAAATCAGAGGTCCGTGGTGGGGCTTGTCTGGAACCCACACAGACTAGTTCTGAAATAAAGTTCTAGGCGTCAGGAGGGG
AGGCTGCCATAGAACAGGGTGAAGCTGGTCTAAGTCTCGCTTCCCTGACCTTTCCTCATAGACGATCACCAGGAGCCATG
AACTCATTGTGACCAGAGCATCACAGGTCACAGTCTGTCCACACACAAGTGTCTCAGTTCAGCAAGGGAGTTAAAAAGAG
GAACCAAAACTCGCTCAAGCTGTTTCTGGGCTTTACCTGAGTGGAGGGGAGGGTCCCCCGTTCTAACCACGTGAAAAGT
CCCACAGAAGCCCCGTTAGAGGAAGCCTCAACCGAGGGTAACTGGAGGGATCAGAAATGAGGGGAAACCTGAAACCAGCC
TTCGAGGGGTTGCATTAGGGAGAATATAGGTTCTATTACTCAGATATCACAGACCGCATTAGGAACATATCTCTTTATAA
GAGACACGGTCTGCCTGTTCTTATTCCTTTGGGGGACACATAAAAAGCAACAGGTATTTGGGATGCGAAGTCAGAATACA
```

```
GGAATGAGTTTCTTTTGGGAATGAAAGACAAAGTATTTCAAATCTACACCATCCCAGAAGGTCCAGCAAGATGGTTCAGA
GGGTAAAGGTGCTTGCTGCCAACCCTGACGACCTGAGTTCAGTCCTGCGTCTCATATGGTAAATAGAGAGAACCAACTCT
GAAGTTGTCCACTGATCTTTTGAAATTTCCACACAGAGATAAATATAATAATTTTTTAAAATTTAAAGCAAGTGTTATCT
CAGGAGGCAGCTTTAAGAGGTTACTGGGAGATTCGTGTGCACGTTCCTCAAATACATGACCCCATGTTCACAGTGTAGAC
AGCCATTGGTTCTCAAGATGAACTCATTTTAAACCTAAAGACGTGGCAAAGCGATAGTTCTTCCACGTTTCCCCCAGAGC
CAAGCATGACGCCAATGACTAAGCATTTAAGAGATTGAAACAGGGAGGGTCATAATGTTCAGGGTATCCTGAGTTCAAGG
CTAGCCTGGGCTACACAGTGATATCCTGTTTTTAAAAACTACACACTGGGGCTAGAGAGATGGCTCAGTGACTAAGAACA
CTTGCTGTTCTTCCAAGTTTAGTTTCCAATACCCACATCTAGTCTGATGAGCTCTTCTAGCCCCCAAGAACACACACACA
TAAGTGGCAGACACACAATATATATATACACACACAGAAACATAAGTAAAATAAAAAGAAATCTCTTTAAAAAATC
TAAAAATCAAAAGCACTACATACTCCTAATGGCCAAAACCCTCAGGCAGAGATGGAAGAGGACCCAGTAGGTTTGCTCCT
TTGGGGGAAAAGAAGGACATGGGGCACATGACAAACCAGAGTCCTGAATTGTCTTTGGGGAATTCTGAAAGAAGAGTGTG
TTGAGAAGAACAGATCTACACACACACAACCCAGTCTCATTCCTATAGACTGCACCCTGCAAGTTACTGAAGGCTGAACT
TAATAGCCTCTCCCAGATCCCAACTGGTGGCTGGAAACACTTACGTAAAAGGAGACATAAGCTCAGCAGAACCTGGAAAC
ACAAGCCTGTAATCCCAGCTACTCAGGAGACTAAGGCAGGAGGGTTCAAAATTGAAGACATTGCCTACAGAGTGAGTTCA
AAGCCATCCTGGGCAACTTAGTGACATGACACTGTCTTAAAACAATGAGTAAGGGCCAGGTGGTGCCTGTGCACATCTTT
AATTCCAGCACTGGAGAGGCAGAGGCAGGCAGATCTCTGAGTTCGAGACCAGCCTAGTCTATAGAGTGAGTTCCAGGACA
GCCAGGGCTACACAGAGAAATTCTGTCTTGAAAACACAAATAAGGCCTGGAGAGATGACTCAGCAGTTAAAAGCATTGGC
TGCACTTCCAGTGATCCTGAGTTCAATTCCCAGCTACCACATGGTGGCCCGCAATCATCTGTAATGGGATCTGATGCCCT
CTTCTGGTGTGTCTGAAGACAGCTACAGTGTACTTATATACATAAAATAAATATTTTTTTAAAAAAATCTACATCTGTT
TTAAAAACACACAAAACAAAACAAAAGTAAGTAAATAGTTATGCTCTTTTGCTATGAAAGTATGAGGATTCAAGTTCACA
TACAAGAGGATCCCTGGAGGGCTGGAGGAAGGGCTCAGCAGTTAAGAGCACCGGATACTCTTCCAGAGGTCCTGAGTTCA
ATTCCTAGCACCTACATGGTACAACCTTATATAACTCCAGTTCCATAGGATCCAACACTCTCTTCTGGTATGTGCAGGCT
CATGCATATGGGCACAGACATATATGCAGGCAAAACACCAGTACACATGAAGTGAAAATAAAGTAAAGAAGTTCATTTAA
CACTTGGGAGGCAGAGGCAGGCGGATTTCTGAGTTCAAGGCCAGCCTGGTCTACAAAGTGAGTTCCAGGGCAGCCAGAGC
TACACAGAGAAACACCCTATCTCGAAAAACCAAAAACCAAAAACCAAAAAAAAAAAGTTCATTTAAAGGGAGGGGGGATA
CTGGCACTCAGTGGTCAGCAAGTCTACATAATCAGTGAGTTTAAGGTTCAGTGAGACTGTCTCAACAGATAAGATGGAGA
ATGCTGGGAGAAGACACCTAATATCTACCTCTGGCCTCCATAAACACACACACAAACAATACCCCCCCCATACACACACA
CACAGTCACAGTCACAGTAGCTCAGTGGAAGAGCACTTGTCTAGCTTGCAATATTGCTCCACAGAGAGGAGCCTGGGGGA
ACATACACCGGGCAGGAAGCCCTGTAGGCTCCTCAGGGGGGGGATTAGGGTGGGCCTGTGGGCAAGAGAAGCAGAGACTGC
TGTGGGAGGGGAGCTGAGACCATGCTAGGAAGCAACTCAAATCTGTCCACACAGGGCGTGACAGACAACCAGGACAGCAG
TGAACAGCAGACTCGAGAGGCAGGGGTGACTTTCCTGACACAGGAACTCTTCCCTTGCACATGAGTTCAAAGATGGTGGA
TCACTGTGCCTGTTAGAAAGTTGTCCCTCCATCGACTGGAGCTACTTGGGAGCTTCTGCCCAGTCCTGCTCTATACGTGA
GGCTCACATCGGAGTCTAGCTGTCTCCTCCGAACCAAAGGCCCTTCCCAGCCCTTGAAGACAGTTCCCATAGGCTTCAGG
CTTCTTTTTCCTATTTCCTACGTTCATAGTACCTGGAAATGCCTTTCCAGGGAGGGATTTTCCAAACCTTTCAACAGAGC
CAACTGACCTGAAAAAGAGTTCACCTCATCTAGAAAAAGCCTATGACGGCCTGAACGCGCAAGGAAGGTGTGTGTGTGTG
TGTGTGTGTGTGTGTGTGTGTGTCTGTCTGTCTGTCTGTCTGTGGCTCTGTGTATATGTGTGGTGTTTGAGTCAGG
GTCTAAGTATGCTAACTAGGCTGGTTTCAAACTCATGAGCTCAAATGACTCGTTCGACTCAGCTTCCCAGCTAACAGAGA
CTACCGGCTACACCCCAACCCAGCTCTAACTTGCCCCCTGCAATAATTCTACCTGGTGAATTTGGGTTCCCCATCTCTGC
TTATGAAGGACTCTGCATTGTTAGTGTTCTCCAGCCACACCCCATCCAAAGCATCCTGAATTCTAGAAGAAAATCCAGGG
TACATCCTTAAGGAATGTTCCCCAGCCTGCTCCCAGAGAAATCCAGTCAGCAAACCCTCACAGGCTGCCTGCTAGACAAC
TCGAAGAAACACACACACATATCACACACAGAGAGAGACACACAGACACAGACACACAGACACACAGACACACAGACACA
CACAGACACACACACACACACACACACACACACGGCTAGACATTTTGCCTGTCCCAGGGAGAGACCCCAGTCTTGTGT
AGAGGGAAGAGGAGCCTGTGTGGATCCCAACAGGAAGAACACTTTGCGTCGCTTACTTCTTCTTCCCACCCAACCTCCAA
CAGCAGGACCAGCTTCCCATCCTCCTGGCCCAGAGCCCACAGTGCTTTCTAAATACATCCCTAGCTTATGTCTGGCATCA
TTCCAGGCAGCATCCGTTCCCGTGGGACATGCATCCCTAGAGCTTGCGTGTACGCTGTCTGTGTTCTGTTTGAGTGCCTG
TCTATGCAGCAGAGCAGTGTGGTCCCACCTGGGCATATGTCTATGTCTATATAGGATGTGTGTTGCCCATCATGTGTACC
CACAGTGCGGGTACACATGAGGGTTTCTGCTGCCTTTTGTAGCCAAGAATGAGGGAGCCATTCATCCCATCGCTGACAAT
GCACTCTTCCCAGTGTGTTAGCTAGGCCAGCTCCCTTTGACAGCATCTTTCCACCAGGCCAGAGCCAACCCTACCTAAGG
CTTCTCTCTAGGGTTTGATGGAGAGGAGAAGTACCCAAGCTCCTGTGTATGGGAGTGGCTTTAACACTTGTCGAGCTTC
AAAAATGTTTGACACCTTCTGTTGATTTATACCACACTAGCTTCCTCCCATCTTCTCCTCCTCTGAAGACTTTACCAAAA
CAATACCTGTTGGCATGGAGGAAGAGACAGACCCTCACCCCCTTACCTGTCCCAGTGGGAATATCGCACTGATTGAACTT
CAAGTGGAAAGGAAGTTCACTAGCCTAGATAAAAACTTGAGCCGTTATCATGAAAGGCCTTTAGGCCAGACAGGCTGGCT
AGACTCACATGCTCCCCTAGATAGGTTCGGCTGTTACCAAGGTGAATCATCACATCTCCACCATCTGCCTGATGCCTGTG
CTCACCAGTTCACCCCACAGCTTCCCCCTCCACCTCCTACTGCAGAGTCAGAGTCAAATAGCTCTACTCAGTTCTACTGG
AAACAACTCCCTAAAGGGATAGAGAGCCCAAGTTAGACTCCAGAGGCAGAGTTTACAGACAGATGCAAACGTGTAAATCC
CTGCTGAGCCCCAGATGGAGTCAACTTGGGGGCCTTGAAGGAAGGCAAGAGTTTAGAGAGGTGGACTTGAAAAAGGAGCG
AAGGTGACAGGAAGCTGCCTGGGTTGCTGGGACAGGTGGCTGTGGCACACATACCTGAGGAATAGCATGTCTGGTGCAG
TGAAGTCGTAGGTAAGGTCTAATACATCAAAGGAGTGTACATTTGGGAGAAATTTATTTTTGCAGGGCTGAGGCTCAAAC
TCTGGGCCCTGTATATGCTAGGCAAGCATCTACCTGCTGAGATGCACTGCCAGGACTACTTTAGATAAAAATGTAAAGGC
CTTCTACCTGGGGCATTGAAGCCCTATCTAACAGGTCTATTGTTTTCAAGAGTTGTAGGGCCATTCTCTCTCTCACAGGC
TGTTTTTGCACACGCTAGGCAAGGGCTCTACTCCAAAAATGTTTGTTTGTTATAGAGACCCAAGCAATACTTGCCAGCCT
```

```
GGAACTCATGTAGATGAAACTGTCTTTGAGCCCTTCATCTCCTGGCTCTTCTCTCTAGTGTTAGGATAACAGCCTGAGCC
ACCATACCTAGCCATATATATAAATGGTTTTACTATTGTATACGATATCAGAAAAACAAGACAAAAGACACCTGTTTCTT
ACAGGTGATGGGGAGTCATTAAAGGACACTAGATCTATAGGGCCTAGTTAGGTGTGGTTATTTGACCTGTGTAGGTGTAG
ATTTGGTTTGTTTGTTTTTGAGACATAGTCTGGCTATACCGACAATGGAACTCAAATCGTGGGCTTAAGCCACCCTCCCC
CCTCAGCTCCTGAGAAGTGCAGGGCTGGGATGTGAGTACGAGGAAGCTCCCCCTGTCCACTGGGTAGGAAGTGGAAAAGA
ACAGAAACAGTAGTTAGAAAAGGACCCATAAGGCCGGAGCGAGGCTCCAAGGTCAAGAGCACTTGCTACTCTTGCAGAGG
ACTGGGGTTCAATTCCTAGCACCCACATAGTGATTTCCACCATCTGTAACCTCAGTTCCAAGGAAACCAGGGCCTTCTTC
TGACCTCCATGGGCACTAGGCGGTCACATGCACACATACACACAGGCGCAGGCAAAACTCTCATACACGGAAAATAAAAA
GGTTTGAAGTCTAATTTAAAAAAAAAAATGAAAGAAAGTAAGGGGAAAGACCCATAGAGACAGGATGGGGGACAGGA
TAAGGTGACGGCAGAAGAAGGGACACTGGAGAGTGGGTAGATAGTGCTGCCATTGTACCATGTGTCCCCATCCTCTCTGA
AAGAGCCACCTGCTCTAGGGACCAAAAGACCAAGATGGGGAATCAGTTTACCCATCAGGGGAAGTGAGTGTGTCACCCAC
AACAGTGTGCCTGGTGGCAAGAAAGATTTAAGGTAACCATGAACCAACCCCTACTGGCAGGAGCCCCTCACTCTCCTCAA
AGGGGGTCAGCACAGAGATGAGATCGCCCAGAGCCTAGAGGACAGAACAGCCCACTGTCTCTTACTTCCCTGGGGCCCATC
ACTTATATCACTGTGTTTTGAACCCATAGTAGTTCGGGTGTAATTGGTTTTGTATTGCCCAGGGAGGAGGTTGACCAGGG
CAGGGCAGGGCAGGAAGAACAGAGTAAGGAGCTGAGGAAACGCAGGTGCAGGCAGCTGTGAGTGAAAGGTATGAAAACA
GGCACCCTGTCTTCCTCTGTGGTTAGCAGGCAAACAACCTGAGAGCTGGGGTGATGGAGGGCAGTGGCAGATGGGGAGGA
GGACGCGGAGGTAGGGACTTCCGGAGATATAACAGGCACGCCCCCTTCTTTTCCACTCAGAAAACGCTTCCTCCCTACCC
TGCGCCTAGTCTCCCTGTGTGTGACTCATATTTGCCTCCGCTGCTGGGTTCCTGTGCCTAGGGAGTTTACTCTCTGCTTG
GGAAGTAGCTGGAAACTTAGGCTCCGCCCCTTTCCCAGAAGCCTTTGGAGGGAAGTGTGGGTAGTAGCTCTCATTGTAGC
GTTATTGATTGACTAAAGACAGGCAAACGTGCCTTTAAGCAAACCTCCTCTTTGAGCAAGACAGGTCTGTAAAATAAGAG
GAAGATTACATACCTCAAGCGTGTGTGGATTAAACCAAATAGGAGGCCATTTCCTCAGTTTCAGCAATAATCAAGACAGG
AAGAAGGGGAGAAATTTAGAGGAAGTAAGCCAGCTACATCAGCACTCAACCATTCCCCACCCCCAGTGAGAGCTTAGATT
TACTACCCACTGTCCTTACAGAATTGGAATATTGGCTTCTTGTTCCTATCAGGTTCCATTCCCAAATGTCAGTACTGACT
GCAATCCCTAAGCCTTCCATCTTTAAGAACACCCTACCACTTTGTGAGTGACTACTGGTCCAGTTTACATACAAACAAAC
GAACAAAAACGAAAGAAAGAAGAGAAAGAAAAGACCTGAAGTTTCAGGCCAGGGATGTGGCTCAGTGGTGGAACAGTT
GCTCAGCACTACCAAAAATTAGAATAAAAATTATATTTAAGAGACAGGGCGGGGCTGGTGAGATGGCTCAGTGGGTAAG
AGCACCCGACTGTTCTTCCGAAGGTCCAGAGTTCAAATCCCAGCAACCACATGGTGGCTCACAACCATCTGTAACAAGAT
CTGACTCCCTCTTCTGGAGTGTCTGAAGACAGCTACAGTGTACTTACATACAATAAATAAATAAATCTTAAAAAAAAAAA
AAAAAAAGAGACAGGGCATGGGGGCATGTGCCTTTAATCCCAGAACCCAGCAAGCAGAGACTACCCAAAGCTCAGGTCAA
CCTGGTCTACATAGTAAATTCCAAGCTAGCCAAGGCTATAGAGGGAGATCCTGTCTAAAATAAATATGCAGAAGGGAGGA
AGGAGGGTAAAGATGGCATGTTTAAGGACAGGCCAAGAGGGAAACAGAATAGTACAGACAAAAAGCATTAGCCTGGAAGGA
CAGGAAACCTCACAGGTGGCAGCTTCCTCTCAGAGGAAAAAAGAAGAAGAAGAAGAAGAAGAAGAAGATATGGCTATCGT
TTTCATTTCTCTTTCTTTTGGTCCTTACAGTACAAGGAACTGGCCTTCTACAGTAACAGCACGGAGATTCCCCTACAGGA
CAGTAACTTCTGCTCTACAGTCGAGGGACCCTTACTGACGTCCTTTAAGGCGGTATTCATGCCTGTGGCCTACAGCCTCA
TCTTCCTCCTGGGTATGATGGGAAACATCCTGGTGCTGGTAATCCTGGAGAGGCACCGGCACACTCGGAGCTCAACCGAG
ACCTTCCTGTTCCACCTCGCAGTAGCCGACCTTCTCTTAGTCTTCATCCTGCCTTTTGCAGTGGCTGACGGCTCTGTGGG
TTGGGTCCTAGGGACCTTCCTCTGCAAAACTGTGATCGCTCTGCACAAGATCAATTTCTACTGCAGCAGCCTGCTGCTGG
CCTGTATAGCTGTAGACCGGTACCTAGCCATCGTCCATGCTGTTCACGCCTACCGCCGCCGTCGACTCCTCTCCATCCAC
ATCACCTGCACGGCCATTTGGCTGGCCGGCTTCCTGTTCGCCTTACCGGAACTCCTCTTTGCCAAGGTTGGCCAACCTCA
TAACAACGACTCCTTACCACAGTGCACCTTCTCCCAGGAAAACGAAGCGGAAACTAGAGCCTGGTTCACCTCCCGTTTCC
TCTACCACATCGGGGGGCTTCCTACTACCGATGCTTGTGATGGGATGGTGTTACGTGGGCGTGGTCCACAGGCTACTGCAG
GCCCAGCGGCGCCCTCAGCGGCAGAAGGCGGTCAGGGTGGCCATTTTAGTGACAAGCATTTTCTTCCTCTGCTGGTCGCC
CTACCACATTGTCATCTTCCTAGATACACTGGAGAGGCTGAAGGCTGTGAATAGCAGCTGCGAGCTGAGTGGCTATCTCT
CTGTGGCCATCACCTTGTGTGAATTCCTGGGCCTGGCACACTGCTGTCTCAATCCCATGCTCTACACCTTCGCTGGCGTA
AAGTTCCGCAGTGACCTCTCTCGGCTTCTGACCAAGCTGGGCTGTGCTGGCCCGGCCTCCCTTTGCCAACTTTTCCCCAA
CTGGCGCAAGAGTAGTCTCTCTGAGTCAGAGAATGCTACTTCCCTCACCACCTTCTAGATCCCGGAAGTCTCGGGGCCCC
TGTCTGTTTCTGTTTTCCTTGGGAGGATAAAGTGGTGGCGGAACCCATCCAACTCGAGCTTGGGCCAGTGTCCCCAGATG
GGAAAGCTAGATAAACTCTCTCAAACTTTCCCAAAGGGGAAAGCAGCCCAAAGGCAAAGCAAGCTATATCCAGGCCACCT
GTATCACCTTAGATGAAGAGAACTCCATACACCTCCCATCCTAACCAGCTAAAGCTAAGCTCAGCTTTATTCTTCCTGG
CCATAGGGACAACCACCTCTGCTGTGGCCCACAGTCTCATCTTCCTCCTGATTATGAGCCCAGACTCTCCTCCCAGAATG
TATTCCATCATCTTAAAGACTACTGGCTGCCACAGCTACCCACCACTCCTATACCACAGAGGAATAGCCAGCTGGCGGCG
GCAGACTATGGCCTTAATGTGCCTGTCTCATAAATACAGACTTCATGCCAGACCTTCAACCGTGCCTTTCTCTTAACCAA
GCAGAAAGCTGAAACCGATCTACTTTAGGTAGCTGTCTGGTTCCAACCTAACCAGCATTGGGTCAGCCCCATGTTACTGG
ATCTTGGATTACAGACTGAGCGCAAGTTCCAGAAGGTTCTGGAAGCTAGCCAGTATCCTAAGAAGAGTAAAGGGCAAGCC
AGCAGGAAAGAGGCCCAGTGGAAAAGTGGAAAGACACCTTTTCCAGGCTCTAAGGAAGAACAAGTAAAAATCAAACCCAG
CTGTCTTCTCCACCCAATGTACCAAAGCTTACGACTGGTGGGGAAATGAGATCCAGGGCCCTCGTGGATTCTACGCACC
AATGGGGAAGGAAGCCAACTTGCCTGGGGAAAGCAAGATAGCAAAGTGGTCCTAGCCTCGAGAGAGGGGACACCTAGCTA
AGAGAATGACGACAGAGGTTCCTGTCTTCATTAGGCAGAGGCAATATAAGAAGCCAACCTGGGCAGGCAAGTCCTCAAAC
CCCAGGAAGGCAGTACCCTGCCCCTGGGAGGGTACCACTCACATGGAACCAGAGGAAGCTGCTCCATGCATACATAGGGG
AAGTTAGCAGGCAATTCTGAGCTCGGCTTCCTCCCAGCCACCGATCTGGGGGCGTGGGGGTAGGAAGCAGAGTTGCCTAG
TACCACTCAAGCCAACCGTACAAGCTCCCTGGGGGATCCCACTGGGGAAACCAATGCTATAGCTTCAGAGACTGTATCCT
```

```
CATTGCAGAACCGTGAAGACACCTGGGGACCCCCTTTTCTGCTCCCAGCATCCAACAACCAGCTGGGAAGAGGCAAACCG
GGCACAGAAATAAAAATGCAAGAGATGGCATTTTTGAATTTTCTCTTTTTAATAAAAAGGCACCTATAAAACAGGTCAAT
ACAGGCAGAGACCCCCGGAACAAGCCTAAAAAGTGTTTCAAAATAAAAACAGGAAGATGTCTTCACATATTGTATTTATA
TATTTATATTTTATATATATATTTATATAATGGTACAAAATGGCTGGGGTATGGCCATGGATGGAGGGAGAAGGCTGGCC
TGTGGAGATCACCTTGGGAAGCTAGTGGTAGAGGTGGGAGACGAGGGCACAAGAAGAGGGCATTGGGCCCTTTGTGGCCG
GAGCCCTCTCCTTGGAGCAGGGCAGGCTAGACAGTCTGGGCAGGGTAGGGATGTGGGTGGGGGGCACAAGGCCCAGATGGA
AGCAGGGAGGGCAGTGTCTGGCAGGAAGGACAGGTTTCCACCCACCTCTCAACACAAGAGACCTTGGCTATGGTCCCTGG
GAGGAGCAGGGGAAGCCAGGGGCCTGCAGCCAGCTCTGGAGGGGGCTTGGTGCCTGGCCTGGCTGATGGTGGACAGCACC
AGACTGGGAGAAGTGGGCACAAATTCCCTTTGTATTACAGCTGCCAAGGCAAAACCAGGCCCTGCAGGTCAGAAAGGCTG
GGGACAGGGCCCTGGTAAAAGACACCCTGTCTAGAATGGCCCTTGGCCCTGGAGGCAGGACAGGAAAGGCCTTAGCCAGG
GAGCTGCCCGCCACCTTACAAGGCAGGAAAGGACGTGAGAAAAACAGAAGTAGTTTACTCCTGGGGCCAAAGGGGACAAA
ACACCCATTCCTGTATGGCTTGAGTCTGACCAGGGGCGGGTGAGGGGCTCTCCTGGGAAGTCAGAGGAGAGGCAGTGGCT
GTGTCTGGTGGGCATAGACAGATCTGGTGCATGGCCCTGAGCCCTGGACAGAAGGACCTTGCAGAGAGTAGGCAGGCAGA
GGAGGCAACAGGATGTTCTCGCTCACACTGCCTTCCCTCGCTCTGCACTAGAGGTGGAGGGCCAACTCTGTACCTTCCTT
CTTTGGTGAGAAGCAAGTGGTGGTTTCTAGAAGAGTCCATGCCCTGAAGGGTTACCTGGCCCATGGCTACTGCAGCCAGG
GTCCCAGCATCCTAACCCCTCCATAGCTCCATTCTATGGGGCCACACGCTGCCCCTTCCTTCTCGCCTTTTATCCTAGAA
TGGGGATAAAGGGCTTCAAGTTCTGGCTAAGATGTAGCAGCAGAGGCACCCAGGCTGGGGGCCCTATGTCACTGGTGTGG
GTAAACACATGTACATGTGCGCACCTGCTCTCTGGAAATCACTCAGCAGCAGACAGGCGGCCGTCGTAGAGGGACGGGGG
TAGATTCTCAGTCCTCAGGGGCTCACCAAATGGAAGCCTAGATGGTCTGATCTCATTTTATAGGTGGGGCTTATGGAGGG
TGCATAGGACTGTATGTCTCTGGGTCCAGAGGAATGAAGGGTGTGATCATTTGAAAGAGGGGGGAAGGTTTTTTTTTTTC
TCAATCTTTTTTTTTCTTGTGTGTGACTTCTATCAAGACACAGAAATACGGCACACGCACAAACGGCACAAAAGCCCA
GCTCTATTTACAGCTCCGCTGGCACCTGCCCACCTGGCCAGCTGCCTGCAGGGGGCGTGGGAGAGGAGGTCAGCCACGTC
AGTAGGGAAAGGAAATGAGGCAGAAGGAAAACAGGGTAAAAGCAGAGAGACGGGAAGGCGAGAAACGGACCTGAAGCGCC
GCCGGCAAGGTGACAGTCCACGACGGGTCAGAGGATGGAAGACCCTCCTCTCTCCCCAAGGACCCCCAAAACATAGCTGA
AAGCGGGGAAGTGCAAAGACAAAGGGAGACAGAAGCTCCACGGGAAAGGGGGGGGGGGCAGAGGAGGCGCAGTCCTAGT
GACAGAAATGGAGCAGAGCCCGATTCCAACTCCTCACCCGACACTTGCCGCTTCCCCCTCAGCTGCCAGCACAGCAGGTT
TCCACAAACACCACTCCCCACACAAGCCCGTTCCCACCCGCCACCCTACCCCTGGACCCAGGCCATCCATCCCAACACTG
GAGGAAAAACAAAACTTTTTAAAGTTAACATATTTTCAGTTTCCCCAGCCCCAGACCGAGGGGCCTGCTAGAAGGGTAGA
TTGGCCATGCTGCCCGGCGTTGGCAGGTAGCCCATCTGGCTGTCCAGAGACACACTGCGCTGACGCAGAGGGTGGGACAC
CATGAGGTTCTGCTGGGGTGGGGGGCCCATGAGGGAGCCCTGTGGGGAGAGCATGTGGGGAGGCTGGGTGTAGACCTCGC
CCCCCACACCCCTCTGCTTCATCAGCATAAAATTCTGCTGGGTCATGAGGCCTTGTGGAGGGGACATGACCCCCTGGTGC
AGGCCATGGGGCACCATGCCCTGTTGTGGGGGCACACCTGTCCTGCCCAGCAAGGACATCTGTCCAGGGTGGCACATGGA
CATGCTGAGCCCCGCTGGACCCCCTGTTGGCCCGGGAGGTTGGGGGGTCGAGACGGGGTCTGCTCTGCCATCATGTTCT
GCAGGTTCATGAGATGCAGATTGGGGGGCTGGGCCTTGGGGGGCTGGTTCTCGCTCTTGGGGAAGTACTGGAGGGTGCTG
CTTGGTTTCTCAGAGGGGATGATCCTGGATAAGTCGAACTCAGGAATGCCGGTAGGGGTGGGCCGGATCACCTCACTCAG
CTCTGGGTCATTCAGTACCGATGCCACCCCAGGGAGCACCCCGGGTGGGTATGCGTCGCCCATGCGGCCAGTCATGCCTT
TGCCCATTAGGTGCTGCTGGGGGGGTATGGGACCCGGTGGCTGGGTGGGCAGGTCCTCGGGGGGCAGGGCCATGCCTGAA
GGGTAGTGCTGCTGCAGGCCTGGGCCCCCGGCCCCGGTGGGGGCCATGGCACCGTGAGGTTGCTGCAGCCGAGGAGGGAA
GGACATCATCTGGGAGGAGCTGGGCACAGGGCCACAAGGGGCATTTAGGGAGTCTGGGCCTCCTGGGGCCATCATCATTG
AGTTTTGAGAAGAGCCCCCAGTCCCCTGTGCATTGGGATGCAGTGGAATGTTGGACCCCAGAGGGGTGGGGGAGGGCAAC
ATAGTAGGGGGAGGCTCATGGGACAGGGGCTGCTGGCCTGGCAAGTTCATGCCCATGGGGCTCTGGGCAGCAGCAGGGTT
CATGTGCATCTGGTTGGGCTGGTTATTGCTGATACCTGTGGGAGGAAGGACGAACGGGACATTAGAGGCCATGCCCACCC
AGTTTGGGGAGGGGTCTCCCAGTCCTTCCCAGCCCACTAAAGAGCTCTGGGCTAGGTTTCATGAAGCGATGGTAGTCATT
CAAACGGGGCCTCAGAGCTCACACCCACCCCCATGAGCAGAGACTATAGAAGCATGCAGAAGCCAGCATGTCCACGGGGGC
AATTCCTAAGCAAACCCACTGTTGCCACTCCCAGAGAGAGCTCTGCCTCCTCGCACCTGGCCCAGAGCCCTGCGGTGGTG
GGGGTGGGGGTAGCAGGGGCCGGTCAGGCAGCAGCTCGTCATCTGAGGTGGCGATGGTCTTGATGGCGTTGTGGTATAGC
GGGGTCGAGCTGGGCATGGCGTACTTGGACATCTGAGACATCATCAGTGACAGAGGGTTCTGGGAAGGGGTGGGGTCTGG
GGAGGATGTGAACGGTAGGCTGGGGTTCATGAGGCCACTGGAAGGGTTGGCGGGAGGAGCGGAGGAGCTGTTCCGGGGTG
CGCTAGGTGGAAGAGCACCTACAGAAGCGGAGAGACAGAAAGAGAGCGGGAGGGGGGTGTCACTAGCGAGGTTAACGTC
CCCCAAATCTCCACCTCACGTCAGACACACCCATATCCCACTGACCACAGGTTCCCTGTGGTCCCTACGGCCCATGCTTC
TCCAAGCAGTTTCCCCAGTGGGGAAAGTCCTAGCTCCAAGTGGCACCCTTCAAGATGTTTAAGAGGCTCCGGCAGAGCAT
GCCCCTGCAGGAACCTACCCGCCACCAAGGCTCCCACCCAAGCCCACCTAGATCACTCACCCTGTTCCACGTTGCCCAGG
GTGGAGGAAGAGTTTATGCTGAGAGGTGGCTGCTTGTTCTGGGAGACCCCAGGACTAGGCATGGCTGTCTTGGGAGAGGC
GACCCAGCCTGGAGAAGGCACCGCCATGGAGGGAGACTTGAGCCTGCTGGGTGAGCCAGTGGGTGAACGCACACCGAGGG
AAGAGCTGAGGACCTGGGGTGACTTGAGAGGTCCCGGTGGGTTGGCAGAAGGCAAGGGTACCATCGGGAGGGAGTCTGG
GGTGACTTGAGGTTGGCAGAGGGTGAGGTGACCAGGGGGGAGTGCACCTGGCTAAGGGTGGGTGACTTCAGATGTCCCAT
GCCCGGTGAGCCCATCTGATTAATACTGATGGTGAGATCTGAAGGCCGCCTGCCTAGCCCCCTGTTGGAGGCCAAGTGCA
CAGACCCAGGAGGATTGGAGGCAGGGGGGCAGAGGCATATGGCTGAGCCGGGCAGTGCCCACAGTGCCCATGGGCACTGAA
CTCTGATCAGGGCTGAACATGTCTGGAGTGTTGCCCATGTCCTGGGGCATGGCTTGGTAAGGTCCCTGGCCCCCAGAGAA
TCCCTGCTGGCCCTGGTTGGGAAACTGTGAAGGCATGAGCATCTTCTGCGGACCCCCATCATGCCACTACTGTTCTGGG
CCCGAACCCGAGCCATCTCTTCGGGACTGAGGCCCTGAGGACCCATCATGTCTCCAGGGCCCCGCATCTTCTGTGACATC
```

```
AGCATCTGCTGCTGGGGCGTCATCTGCACATTCAGGTTCATGTTCATGTTCATGTTCACATTCATGTTCATGTTGAGGTT
GCCTGGCCCCATAGGCGGGTCTACCTCCCGCAGCCCAGACTGTCCCATTGGAGGGCTCAGGAGGCCCCGACCTCCACCAA
ACTCTATGCCCATGGGTGTGCCGGCGAGACCATCTCCTCCAGTCATCTGTCCCGGGAACATGGCAGGGTCCATCTGTCGA
TGAGCCTGTATCATCCGTTCCATTTCCATGCTCTGTCCCATGCCGCTACCTGCCATGCCCAGGGGACGCTGCATGCCCAT
GGACCGCTTCTCCAGCAACTGGTGCCTCAGCAGCTCCTCCCGGACACGAGGGGTCATGAATCGCTCTGCCTCCCCCTGCC
CACCTGGGTAGGGCACGGCATTCTGGGCAAAGTTGCTGGGTCCCCCAATAGGGGGCAAGTCTTCATTCCAGGCCATGCCT
GGCCTTACAGGTCTCTGCATGGCATTCATGGGTACTTCCATGGGCATACTCTGCATACCTCCAAATCCGGGCACCCTTTG
CATCTGGTTGCCTGGGAAACGGGGGCCAGGGAAGGGAGGTCCAGGTCGGAGCTGCATGGGATCTTGGACATCCATAGGCC
CTCGGAGTTGTGCACCCATTCCAGGTGGCCACTGATNNNNNNNNNNNNNNNNNNNNNNCCGGTGGCCACTGATCCCCAGGTT
TGCTGTGGTAAGGAGGTGGGGGCCCCCTCCCCATCATACCCCCCATTCCCACCATGTCCTGCAGAGGGCGGCCTCCGTGC
AATCCAATCTGCTCCTCTTTCCGCCGCTTCTCCTCGTAGTACTCTTCCTGCAGTTTGCGCCAGGCCACCTGCTCGGGCGT
CAGACTGTCCTGGCCCAGCCTCTGCATCATCATGTTCATTTGCTGTCCCATGTCTCCACCCTGAGGGTGCCCCGGCACTT
CATGCTCCAGAGGGGGACCTCCTAGGCTCTGTGTCTGTGAGATCATTGACTGCAGAGGCTCCTCATACTTCTTCAGCCCC
CCAGGAGGGGCGGAGGGAGGCGGTTGAGCAGCAGAGGGCGCTTGTGCCGGTGGGCCTCCCTCACCAGCTCCTCCCGGGGG
CCCCTTGAGGAAGGGCTCAGTCTCCCCACTGCGGAGCAGCAGCCTCTCGATGTCCCGCAGTGTCTGGAGGGAGCGCTCCC
GGTGCTCCAGCTGCTCTTTGGACAGGCCCTCAGAGCCCACCAGGTTCCTCTGCCCATTTCCGGTGGGCGTGGCCTCCCCC
AATAGGGCGGAGCCAGGGGCACTGCCAGGGTCTCCTCCAGGAGGCAGAGGGTTGTTAGCGGTGGCAGCCGTTGGGGTGTT
AGGGTGGGTACCCCCAGTCCCACCACCTGTACTGGCAGCTCCCACTGAGTTGGGGGCCAGGTCCTGACTGGTGTCTTCAG
GAGGCCCCTCCGGGGGCAGAGCAGGAGGGGCACTGCCAGGGGCTGGGGGTGGCGGTGGCAAAGGAGGTGGCTGGGACTGT
GGTGTACCTGCTGATGGCGTATTCAGGGGTAGTGGTTCTGGGGTGGGTGGCACTTTAGGGGCCTGCAGGAGGACAAAGGT
AAAGAATAAGACAGATGAGGGGACATCCCAAAGAATATTAGAGCCTGGCAGGCTACCACACTTACCTGATCCAGCTTGGC
CCGAGGCACATTCTGCTGGTGGTAGGCAAGGATGGACTCTGCCCGGCCCTGCAGCACTGCCTCTGCCGCTCTGCATAGGG
AAGCAGCCACAGGCAGCTCAGAGATGGGCAAAAGCAAGCTGGCCCTCACCCACCAACCTTGCACCGCACCCCCACCCCCC
CTTTGCTCAGGCTGGTCAGACAGACAAAACCCTCTCACCCCTCATCTCTCCCAAAGACTTACGTGTTGGCCAGATGGGTG
GTGAAGACATAGACGAACTGTGACGGAGGCTTTCCTGGGACGCCCCCACCCCCACCCCCAGGAACGTCTGGCCGAAGGCC
TGGCTGGGGGCCATGTTGGGGGGCCTGGTGCACTGCTCTCACTGAGGGGCAGTTGGGCAGTTTGGCCAGGACCCAACTGTG
GGGTCACCATGGCTGGGTCTGCTACATTACAATCTGTAAGAGGAGGAAGAAAGGAGAGCGGGTGAGCGGCCCAGACGTGG
GAAGAAGGCCAATCAGCCGGTGTTCCTGCCCTCAGATGCTCGCTCACTGCCTCAAGCCCATGCCCAGAGGGTCATCTGTC
CCTACAGTCTCCGTTTACCAGCCTGGCTTTCCAGTTCACTTAACCTATACTAACCTTTGCTCAAGATGGCCAGGATGCAG
AAAGATGACCATGGAGAGAGGTGAGAAAAAGTGACAGAAACCCCAAAGCTCTGCTCTAAAGCTCCCACACTCAAGCACTT
GAGATCAGTCAGTTTCTCTGCCTCTCTCTCTCTCTCTCTGTGTGTGTGTGTGTGTGTGTGTGTGTGTGTGTGTGTGTGTA
TGTGTGTGAATTTACTGTCTAAATGACCCAGCCAAGGGGAAGGTAGGGAGGACATAGTAGTAACTGATGATTCAAACCAC
AGACAGATTTTCATCTTACCCCACGATTCACTAACTGTGGTTTTACAAAACAGCATTTGTTTAAGCCTCAGTTTCTTCAT
CTACAAAATGCAAACACTACTCATCTCCCACGTGCCCTGCCCAAGGACAAGTCCTTTTAAAAACATGGGTACTACGCCAC
AAAGCAAAACATCAGTTTCAACAAGAGTCAGAAAGGCTTTCACAAATGATCAACGCAAGACTTCGGTCACCTCTGATGAC
CAAGAATCTCACGGAGGTGTTCCCATCAACAACATGCATACACACGGATGGATGCAGGTGAGATGAACACAGTTTGCAAG
GCACGTGAGAAAGGGCATTTGCTTCTGCACCCTCTCTGAACACGAGACAGTCCTTTTGGATTTTGCAGGGGAAGGGCATA
CATATTGCACACATTCAGTTTAAGGGCACAGGGCACATATGGAGCTCAACAGAGAACACTAAGTGTTGGCTCTCAGACTT
TATCATGCACACCACGTTAGCTGGACAATGACAATGAGCTTCCAGAGAACCTGGCTGCTCTGCTTTGAAAGTCCCATCAC
TGGGCTGATTATAGGCAGGTACCACTGTACCTGGCTTCCTGTGTGGGTTCTGGGAATCCGAAACCTTGCAAGGCAAGTTT
CTCCCAACTGAGCCACCTACCCAGCCCTCTTTTTTGGATTTTTTACAGATTAATCATTAGTCACATTTAATTAAACCATTA
GCTTCAGGTGATCAATTTGACTTTCAGCCCCACTCCTTTGACGAGGATGGAATGTAGGGCTGAAAGCCCCAAACCTCTAT
CCTGCTTTGATTTTCCCAGTCACCAGGTTCCATCTAGAAGCTACCTCAGGACTGCCAGACACACCAGTCGATACAGCATT
AAACAAAACAGAACAACAAAAAACCACTTATCCCTCTGGAGCTTCCCAGGAAACAAGAGAAGACCAAATATATGTTTCAC
AATATCACAGACTCACATACTCGGAACAGCCGAATATTCCTCAGGAGCAAGACAAATTTCGCTCCTCGGTCACAAGCCTG
GCATTCCGTTCTCACATAGATAAGACCCACGACTGGTCTTCAGTGGTTTTAGGTGAGGCAGAGATCCATGGTCATACCAA
GAAAAAACAAGTTAAAGCCTCCGGACTACAAACAGCAAGTGCCAGCACTTCCCAAAATCACAAGAACTTGACCAAGGTGG
CAGACACCAGAGGCCTAGAGTATAAATCAGAGCACACAACAGGCTCCTACAGGCTACGGAGAGGGAAGCAGAGTTTATCA
CAAGAAGGAATTTTCTACTACCCTAACAGCAATACAAGCTAGCACTGTTCAAAGTATGTCACATGCCTGGACTCAACTTT
CTCCTTGTGCCAAATCTATATGACAAAGATCACTTCTATAGATGAGCAATCAGAACTCCTTTCTCAAGTTTACAGTCCCT
GGGATGTAAGTAAAGGCGCCTGGGCTCTGAGCCTGTCTCATTGACAGCTACCTCAGTCTGCCTTGCTGTGTGAAGAATTC
AGTTGCATGGTTGAATGCTGCTGAATGACACAGTGACACAAGACTATAATCCCAGCACTTGGGAGCTATAGGCAAGAGGG
TTAGAAGGTCAAGGCCAGCCTCATCTATACAGTAAGCCTGAGGCTAATCTGTACTACATGAAACCTGGTCTTCACTGAAT
TGCATTCCATCTAAAAGAGGCTGGTCAAGGGATCTTAGCTATTTCTTATCGCTCTGTATCTTTCAAAGTTTCTCCAATGA
CACGGGATAATGTTACAGACACAAATTCGGGGAGACAGGAGGGTTCTGGTCCCAGGAAGCAGTGACAGATTCTACCACTC
AAGCATCCATACTGAGACTGAACATAGCTCATACAGCACTAGTTAGTCACATGGGGCCTTGGCAGTCCCAGCCAGTTGTA
GAGACTTCTTACAGGACACTGGTCAGTAGGGTCTCCTCATTCCCTCTTGCTGACTGTTTCCTCCACTGTGACAGTCATCA
TTACACTTTCTTCATGGGCAGCTGTGAGGACTAAACTCAGTAGCAAATGTATCTTTCTTCTCCTTTCCTTAACTCCTGCC
TGTAAGCAGCACACAATTTAAGCTCTGGCTAGCTTCTCACATGCTTAGTTAGGTTGGAAGCACCAAGGATGTCTCGGGCC
TCAAGCACAGAAGGATGGATTGGAATGAGAGAAATGGCTATGAATCCTAGAAATGCATCATGAGCATTTTCTGAGTGTCA
TTTAGAGACTGGAGAAAGGAACCAGGGACACAATTAAGGGTCAGTTCACACCTAGGCTTAGCAAGGCTTTGCAAAGCCAA
```

GCTAACAGATTCCCATCTTCCTGCCCATCCTCCACTGACCGGTAGGTGGCAGCATAGGCCATGAAATCTGTTAGATGTGT
AGGTGAGTTGTCCGGAAAAGGGATTACCTGAGAGACCTTCGACAGAGGGAGGGAGAGGGGAAGGGAAAGAGAGAGGGAGG
GTGGCGAGGGTGTGAGCAGCACTCACACTATAGATAAGGAAAAAGTCCAAAGTAGGGCTGAAGAAGAGCCTGGGCTCCTA
GTCCTTGCCATTTCTTGAACAATCCCCTAAAGCCCAAGGTAAGCTCCCTCAGGACGGACACACACACACACACACACACA
TACCAGGGGAAGGGCACCATGAGGCACAGCATCAATGGGCACTCACTGTGGGCGGCCGCAATGGGCTTGTCGTCCTCCTC
GCTGTCTGGCC


MOUSE mRNA SEQUENCE : mR3-027.1 (Seq ID No: 14)
ATGAACTACCCACTAACCCTGGACATGGGCTCCATCACATACAATATGGATGACCTGTACAAGGAACTGGCCTTCTACAG
TAACAGCACGGAGATTCCCCTACAGGACAGTAACTTCTGCTCTACAGTCGAGGGACCCTTACTGACGTCCTTTAAGGCGG
TATTCATGCCTGTGGCCTACAGCCTCATCTTCCTCCTGGGTATGATGGGAAACATCCTGGTGCTGGTAATCCTGGAGAGG
CACCGGCACACTCGGAGCTCAACCGAGACCTTCCTGTTCCACCTCGCAGTAGCCGACCTTCTCTTAGTCTTCATCCTGCC
TTTTGCAGTGGCTGAGGGCTCTGTGGGTTGGGTCCTAGGGACCTTCCTCTGCAAAACTGTGATCGCTCTGCACAAGATCA
ATTTCTACTGCAGCAGCCTGCTGCTGGCCTGTATAGCTGTAGACCGGTACCTAGCCATCGTCCATGCTGTTCACGCCTAC
CGCCGCCGTCGACTCCTCTCCATCCACATCACCTGCACGGCCATTTGGCTGGCCGGCTTCCTGTTCGCCTTACCGGAACT
CCTCTTTGCCAAGGTTGGCCAACCTCATAACAACGACTCCTTACCACAGTGCACCTTCTCCCAGGAAAACGAAGCGGAAA
CTAGAGCCTGGTTCACCTCCCGTTTCCTCTACCACATCGGGGGCTTCCTACTACCGATGCTTGTGATGGGATGGTGTTAC
GTGGGCGTGGTCCACAGGCTACTGCAGGCCCAGCGGCGCCCTCAGCGGCAGAAGGCGGTCAGGGTGGCCATTTTAGTGAC
AAGCATTTTCTTCCTCTGCTGGTCGCCCTACCACATTGTCATCTTCCTAGATACACTGGAGAGGCTGAAGGCTGTGAATA
GCAGCTGCGAGCTGAGTGGCTATCTCTCTGTGGCCATCACCTTGTGTGAATTCCTGGGCCTGGCACACTGCTGTCTCAAT
CCCATGCTCTACACCTTCGCTGGCGTAAAGTTCCGCAGTGACCTCTCTCGGCTTCTGACCAAGCTGGGCTGTGCTGGCCC
GGCCTCCCTTTGCCAACTTTTCCCCAACTGGCGCAAGAGTAGTCTCTCTGAGTCAGAGAATGCTACTTCCCTCACCACCT
TCTAGATCCCGGAAGTCTCGGGGCCCCTGTCTGTTTCTGTTTTCCTTGGGAGGATAAAGTGGTGGCGGAACCCATCCAAC
TCGAGCTTGGGCCAGTGTCCCCAGATGGGAAAGCTAGATAAACTCTCTCAAACTTTCCCAAAGGGGAAAGCAGCCCAAAG
GCAAAGCAAGCTATATCCAGGCCACCTGTATCACCTTAGATGAAGAGAACTCCATACACCTCCCATCCTAACCAGCTAAA
GCTAAGCTCAGCTTTATTTCTTCCTGGCCATAGGGACAACCACCTCTGCTGTGGCCCACAGTCTCATCTTCCTCCTGATT
ATGAGCCCAGACTCTCCTCCCAGAATGTATTCCATCATCTTAAAGACTACTGGCTGCCACAGCTACCACCACTCCTATA
CCACAGAGGAATAGCCAGCTGGCGGCGGCAGACTATGGCCTTAATGTGCCTGTCTCATAAATACAGACTTCATGCCAGAC
CTTCAACCGTGCCTTTCTCTTAACCAAGCAGAAAGCTGAAACCGATCTACTTTAGGTAGCTGTCTGGTTCCAACCTAACC
AGCATTGGGTCAGCCCCATGTTACTGGATCTTGGATTACAGACTGAGGGCAAGTTCCAGAAGGTTCTGGAAGCTAGCCAG
TATCCTAAGAAGAGTAAAGGGCAAGCCAGCAGGAAAGAGGCCCAGTGGAAAAGTGGAAAGACACCTTTTCCAGGCTCTAA
GGAAGAACAAGTAAAAATCAAACCCAGCTGTCTTCTCCACCCAATGTACCAAAGCTTACAGACTGGTGGGGAAATGAGAT
CCAGGGCCCTCGTGGATTCTACGCACCAATGGGGAAGGAAGCCAACTTGCCTGGGGAAAGCAAGATAGCAAAGTGGTCCT
AGCCTCGAGAGAGGGGACACCTAGCTAAGAGAATGACGACAGAGGTTCCTGTCTTCATTAGGCAGAGGCAATATAAGAAG
CCAACCTGGGCAGGCAAGTCCTCAAACCCCAGGAAGGCAGTACCCTGCCCCTGGGAGGGTACCACTCACATGGAACCAGA
GGAAGCTGCTCCATGCATACATAGGGGAAGTTAGCAGGCAATTCTGAGCTCGGCTTCCTCCCAGCCACCGATCTGGGGGC
GTGGGGGTAGGAAGCAGAGTTGCCTAGTACCACTCAAGCCAACCGTACAAGCTCCCTGGGGGATCCCACTGGGGAAACCA
ATGCTATAGCTTCAGAGACTGTATCCTCATTGCAGAACCGTGAAGACACCTGGGGACCCCCTTTTCTGCTCCCAGCATCC
AACAACCAGCTGGGAAGAGGCAAACCGGGCACAGAAATAAAAATGCAAGAGATGGCATTTTTGAATTTTCTCTTTTTAAT
AAAAAGGCACCTATAAAACAGGTCAATACAGGCAGAGA


MOUSE PROTEIN SEQUENCE : mP3-027.1 (Seq ID No: 15)
MNYPLTLDMGSITYNMDDLYKELAFYSNSTEIPLQDSNFCSTVEGPLLTSFKAVFMPVAYSLIFLLGMMGNILVLVILER
HRHTRSSTETFLFHLAVADLLLVFILPFAVAEGSVGWVLGTFLCKTVIALHKINFYCSSLLLACIAVDRYLAIVHAVHAY
RRRRLLSIHITCTAIWLAGFLFALPELLFAKVGQPHNNDSLPQCTFSQENEAETRAWFTSRFLYHIGGFLLPMLVMGWCY
VGVVHRLLQAQRRPQRQKAVRVAILVTSIFFLCWSPYHIVIFLDTLERLKAVNSSCELSGYLSVAITLCEFLGLAHCCLN
PMLYTFAGVKFRSDLSRLLTKLGCAGPASLCQLFPNWRKSSLSESENATSLTTF*


MOUSE PANTHER CLASSIFICATIONS
        FAMILY (SUBFAMILY)
                C-C CHEMOKINE RECEPTOR-RELATED(C-X-C CHEMOKINE RECEPTOR TYPE 5)
        BIOLOGICAL PROCESS
                Signal transduction(2.11.00.00.00) > Cell surface receptor mediated
signal transduction(2.11.01.00.00) > Cytokine and chemokine mediated signaling
pathway(2.11.01.02.00) > G-protein mediated signaling(2.11.01.07.00)
                Immunity and defense(2.16.00.00.00) > Cytokine/chemokine mediated
immunity(2.16.10.00.00)
                Immunity and defense(2.16.00.00.00) > B-cell- and antibody-mediated
immunity(2.16.02.00.00)
                Cell        structure        and        motility(2.27.00.00.00)        >        Cell
motility(2.27.02.00.00)

```
MOLECULAR FUNCTIONS
        Receptor(1.01.00.00.00) > G-protein coupled receptor(1.01.01.00.00)

MOUSE GENE ONTOLOGY
        BIOLOGICAL PROCESS
                cell motility > chemotaxis
                cell surface receptor linked signal transduction > G protein linked
receptor protein signaling pathway
                defence response > inflammatory response
                cell growth and maintenance > invasive growth
                MAPKKK cascade > activation of MAPK
        MOLECULAR FUNCTION
                enzyme inhibitor > protein kinase inhibitor
                enzyme > 2-acetyl-1-alkylglycerophosphocholine esterase
                defense/immunity protein > antiviral response protein
                1-phosphatidylinositol 3-kinase > 1-phosphatidylinositol 3-kinase
regulator
                defense/immunity protein > blood coagulation factor
        CELL COMPONENT
                cell > membrane fraction
                cell > cytoplasm
                plasma membrane > integral plasma membrane protein
                cytoplasm > endosome
                cell > plasma membrane
MOUSE PROTEIN DOMAINS (INTERPRO SIGNATURES)
                IPR001053 (BURKITTSLYMR)
                IPR001277 (CXCCHMKINER4)
                IPR000276 (GPCRRHODOPSN)
                IPR000248 (ANGIOTENSINR)
                IPR000276 (7tm 1)
                IPR000276 (G PROTEIN RECEP F1 2)
                IPR000276 (G PROTEIN RECEP F1 1)


HUMAN GENOMIC SEQUENCE : hD3-027 (Seq ID No: 16)
TTTAAAAAGCATGAGTGGGCCTGACGCAGAGGTGCATGCCTGACATCCCCGTGCTTTAGGAGGCTGAGGAAGGAAGTTTA
AGGCCAGGAGTTCGAGACCAGCCTGGGCAACATAGCAAGACCTTATCTCTACAAAAAAAAAATTAAAAATTAGCCTGGCAT
GGTACACACTTATAGTCCCAGCTGCTCAGGAGGCTGAGGTGGAAGGATCGCTTGAGTTCAGGAGTTTGAGACTACAGTGA
GCGATAATTATGCCACTGCACTCCAGCCTGTGTGACAGAGCAAGACCTTGTCTCTAAAAAAAAAAAAAAAAAGCATGATTG
TGTGTGTGTGTGTGTGTGTGTGTGTGTGTGTGAGTGAGTGTGTGAGAGAGAGAGAGAAANNNNNNNNNNNNNNNNN
NNNTACAGGCACATGCCACCAGGCCCCACTAATTTTTCTATTTTTAGTAGATACGGGGTTTCACCATGTTGGCCAGGTTG
GTCTCGATCCCCTGACGTTGTGATCCGCCTGCCTCAGCCTCCCAAAGTGCTGGGATTACAGGCACTAGCCAACTGATATT
TTGTTGACTGAAGATTTCAAACACATACAAAGTCGGTCTAATGAACCCTACATATCTGTCATCCAGCTTCATCTCCCCAC
AGGCAATGTTATTAACACATTCATTCCCCTTTCCTGGATTATTGTGTGTGTGTGTGTGTGTGTGTGTGTGTGTGTGTGTG
TGTGTGTGTGTGTGTGAGAGAGAGAGATAGCACTTTGGACCTATGGAGGATTTCTATTTGGAAAGAAAAAGGCTTTTA
CATACAAGATATTATTTAATAGCAAAATTGGCCATGCAGGTATTTTACAATTGTACATATTAGGAAACTAATGCTCAGAG
AAGTTATATTTGTAATTTTCAAACTGGGGATTGTGACTTATGTGTGGACCCATGAAAGCAGTGTGATTCATTGACCGCTA
TTAAAAATAGAAAGAGGGTCGGGCATGGTGGCTCACGTCTATAATCTCAACACTTTGTGAGGCCAAGGTGGGAGGATGGC
TTAAGCCCAGGAGTTCCAGACCAGCCTAAGCAACATAACAAGACCCCATCTCTACAGTAAATTTAAAAGTTAGTTTGGTG
TGGTGATGCACACCTGTAGTCCCAGCTACTCAGGAGGCTAAGGTGGGAGGATCACTTAAGCCTGGGAGGTGGAGGCTGCA
GTGAGCCATGATTGTGCCACTGCACTGCAGCCTGGCTGACATAGAGAGACCCCATCTCTACAAAAAAGGGAAGAAAGAAA
GAGTGAGAGAAAGAAAGAGGGAACGAAAGGAAGGAGGAAAGCAGGGAAGGAGGAAGAGTAGAAAATATGAGTATGCTACC
CATACTAAGCAGTGTTTACAAAACTTACTGTGTATTTGTGTGTATATGTTCATCACACATGTCCAACTAGCTTGTGATAT
AAAATATATTTCTTCCTATGGGTCATGATCAAAGTGACAGGATTACATGACATGCTGAAAGCTAACTAGTAGAGCTCATG
CATTACTTATTAGAGTAGAGCTGAGACCGACCAGAGAGATAGAGGTGCAACCTGTCAGAGCCCGGGAGGCACCACAGAAA
TCTCAACGGTGCCAGGGTGGGGCCCAGGGAGGCCGGTGTTCTGGGAAAACCAACTGTTGTGTGCTGGTTGTATGAAGTGC
TGTGTTGTTGTGGGTTGTGTGCTGGTTGTATGAGGTGCTGTGTTCTTCCTGTTGTGTGTTGTGTGCTGGTTGTATGAGGT
GCTGTGTTGTTGTTGTAGGTTGTGTGCCGGTTGTATGAGGTGCTGTGTTGTTGCTGTGGGTTGTGTGCTGGTTGTATGAG
GTGCTGTGTTCTTCCTGTTGTGGGTTGTGTGCTGGTTGTATGAGGTGCTGTGTCGTTGTTTTGGGTTGTGTGCTGGTTGT
ATGAAGTGCTGTGTTGTTGTGGGTTGTGTGCTGGTTGTATGAGGTGCTGTGTTGTTGTTGTAGGTTGTGTGCCGGTTGTATGAGGTGCTGTGTCGTTGCTGTGGGTTGTGTGCTGG
GTATGAGGTGCTGTGTTGTTGTTGTAGGTTGTGTGCCGGTTGTATGAGGTGCTGTGTCGTTGCTGTGGGTTGTGTGCTGG
TTGTATGAGGTGCTGTGTTGTTGCTGTGGGTTGTGTGCTGGTTGTATGAGGTGCTGTGTCATTGTTGTTGATTGTGTGTT
```

```
GGTTGTATGAGGTGCTGTGTTGCTCTGGAGAGACGTCAACTATGGCACCCTTTGTGTGGAGGGACTGAAGGCCCTGCTGG
GTGGTTGGGAGCTCCAGGTTGGAAAACAAGCTGTGTGGGGACGAGTTTACCAATTGGTATATGGCTGTGTACCAGTCCTA
CGTGGCCTAGAGCGGTGGTTCTAGGAGTGTAGTTCTCAGGCCAGCAGCATAGCGTCACCTGGACACTCTTAGAAATGCAC
ATTATCAGGTCCCACCCCAGACCTCCCGAATCAGACACTCTCGTGGTGGGGCCTGGCAACCTGGAGGTAACAAGCCCTCC
TGGTGACTCTGAGGCATAAAGTGTGAGGAGCACTCCCCTGGGGATGCCTGTTTGGCATTCTGTGTTTGGCGTGTCTTTGC
GCAAGCAGGCAGGGTCTGGTCTCAGAGCGGCCTTGCTGGGAAGCAGACATGCTGGGCCTCCAGTGCAGCGTTTCTTCTGT
CACCCACTCAGCCTTACAGCCTACCCAGCGCATGCGCCCACAGCACTTAGGTGCGAAGGAATGCTGCACGTTTCCCCCAT
TCCCTGTGCCTTCCACACTGCTGACTCCACCCACACTACCTTAGCAACACGGTAGAGTTTTGCATATTCTTTGAAAATGT
GCCCAGTTGCATGTGAATATGAGCATGCCAACTTGCATGATGTGTCTGTTTTGCCTTCTCTGTTAGACAAGGTGCTCCTC
CATCAGAATCTCGCCTCTGGCTGCCGCAGTACTCAGTATACAAACACCTGATCCAAGCACCAGACTCACTCTAACCCTGG
CCACACAACCTGCCCCAGGCCGACGTTCCCAAGCTCATTTGTGGCCCCCATGACCTTGTGGTAACCTTGATGTGCCTACC
CAGCACCTGCAGCCCATTTCCTCCCTGCAGGACTCTACCTGTTTCCTAACGAGAGGGGAGCTGTGCGTGGGTGCAGGCTG
CCGGCAATGACAGCCTTTACCACCCCACTTCCTGTGTTGACTTGTAATGGGGTGTTGGGTCCCTGCTGTCTGAGTGAGCT
GTGAACATTCATGCTACTTCCCATTACTGTCTGAAGCTTGGTTTCCACATCTGCAGTGTGGTAATAAAAATACCCAACAC
TTTGCCGGACGCGGTGGCTCATGCCTGTGATCCCAGCACTTTGGGAGACCAAGGCAGGTGGATCACCTGAGGTCAGGAGA
TCGAGACCATCCTGGCCAACATGGTGAAACCCCGTCTCTACTAAAAATACAAAAAATTAGCTGGGCGTGGCGGCACGTGC
CTATAATCCCAGCTACTCGGGAGGCTGAGGCAGGAGAATTGCTTGAACCAGGGAGTCGGAGGTTGCAGTGAGCCGAGATG
GTGCCACCACATTCCAGCCTGGCGACAGAGCGAGACTCCGTCTCAAAAACAAAACAAAACAAAAAACCCAACACTCAAAG
ACTGTTCAGGGAATGAAAAGAAATGATGTGTGAAAAACCTATCATAGTTCATGGCACATAGAAAGTACCTGATGAGGAGC
CTGTGCAGGATTTAGCCATAGTTTTCCCCTTAGCCAGCCAGGTAAGTGCCTGTCCTTTGCAAAATGGCGCCTCTGAGAGA
CGGGCCGTGTGGTCTTGTGGAGGGAGCCCTGGATTTGGTGCCTACAGGGCTGACTTCAGGCTCTGACTCTGCAACGAACC
CTCCCTGCAATCACGAGCAAGAAAATGAGACTCTGTTTCCTTGTCCTCACAAAATACGGCCAGGAGTGATGCCAATCCCT
CCGGCACAAGCCGCTTCAAGGACTACATATAATTAAATGTGAAGATTAAGTGAAATATAAAGATTAAGTAACATTAAATT
TAAACTTTCATGGGACTGTGTAGACCGGTCACTGCTATGCAAGTCTATGGCGAGTTTCCTTCAGTGCTGAGAGGTATACC
CTACTCTTCCTGAGGCTGATGAGGTAGGGACCTGGGAGCTCTGTTCTCAGCTGTTGGGAAAGACCCAGAGAAAGAAGAGA
ACAAAGTCTCTGGCCATGTCAGAGAGGGAGAGAGTCCTAGGTTAATAGAAGGGGAAACCCCAGCCCGTTCCCTGTAGCAG
CACCCCAAAACTGTGTGGAACAGCTTACTTTTTCTGTGGAATATAGAAAATGAACTCCAAGACAAGTGATTGTATCTAAT
CTCTGGAACAAATAGAAATTTAATGTAATCCATGGACTGTAAAGTATAATTACTGAGAAAAATACACCTGAGCTGATGTA
AGCGGCCACCCTTGCCTAAACCAGACACAAACCCAGTCTTGGCGGAAGTATCAGTAGTCTTTCTGTCTTTTTTTTTTTT
TTTTTTTTCTGAGATAGGATCTTGCTTTGTCATCCAGCCTGGAGTGCAGTGGTGCAATCTCAGCTCACTACAACCTCGAC
CTCCTGGGCCCAAGCAATCCTCTCACCTCAACCTCCTGAGTAGCTGGAAGCACTAGTGTGCACCACCAGCTAATATTTTT
ATTTTTTTGTAGAGATGGGGTATCCTATGTTGCCCAGGCTGGTCTCGAACTCCTGGGCTCAAGTGATCCTCCCCCCTTGAC
CTCCCAAAATGCTGGGATTACAGAGGCGAGCTACCATGTTCAGCCTCCAGTGGTCATTTTGACGAGAAGGCTAGAAAACA
ATTTAGCCTCAATTAGACTGCCTATGGGGTGGGGATTGGGATCCTGCATAGGTGAAATATAAACAAGTTGCACACTGAAA
TAAAACCTGAGCTGCCTGACCTGGAAGGGTCCTCTGCCATCCGATTGTCACACACAGATGGAACCTCTGACATCTCCTCT
TCAAAGGCAAAGGATCGCAGCTTAAGCGTTGCCCTCTCTATCCTAGGGAGCTAGTCTGACTTTATGTGTGTGAAGATTGT
AAGATCATGGTTTCTAGTAGAGGCAGTAACTCTCTGAGCCTTCTCAAAACAATATTAGAACTCACGGTTGCTCGGCATGG
TGGCTCATGCCTGTAATCCCAGCACTTTGGGAGTCTGAGGCAGGCGGATCGGTTGAGCCTAGGAGTTCAAGGCCAGCCTG
GGCAACATGGTGAAACTCTGTCTTGACAAAAAACATAAAATTAGCCAGGTGAGGTGGCATGTGCCTGTAGTCCCAGCAAC
TCGGGAGGCTGAGGCAGGAAGATGGCTTGAGCTCAGGAGGTCAAGGCTGCAGTGAGCCATGATCGTACCACTGCCCTCCA
GCCTGGGTGATGAAATGAGATCCTGTCTCAAAAAAAAAAAAAAAAGAAAAGAAAAGCCTCAAGGTCAGAAGGTATTTAC
CACTGGGGGCAAACTGAGTTGACTATCAAAGGTCAGAGGCTAACACAAGTAGACCCTGGGAAGCTGTAGTGTGTTCACCA
TCTGGAGAGAAGAATGGGATCTCTGAATAGGCCCAAAAAGTCAAAGACTTCAGGTGACAGTCACAGATGTGTCTAATGTG
TGACGCCTGAGTCAGCCATAGAGAAGCTGGTAGGCTACCTCTCTGCTGCCCAAACCATCATAGCTGAAACTACTGGCCAG
AGCCTGCTGCTATAGCACCTGGGAAGCAGTCTTAAGCCTCCCCCAAAACTCTTTTTCTAGGCCTACAGCACATGTGGAAA
ATATAGAGAACTGTGGAAAGAGTAGGAGAGGAGAAGGCCATAAGAGCAGCTCCTACCTTGTCCAGTACATGCTCCAACCA
CTTCCTTGCTCCTCCCCCTGCCCTATCTCATCATCATTATTGCCATAGCCACACCACCATACACAGAAGGCCCATGATGT
GCCAGGCACAAGGTAATTCTTTTTTTTTTTTTTTTTGAGACGGAGTCTTACTCTGTCACCAGGCTGGAGTGCAGTGGT
GCCATCTCGGCTCACTACAACCTCCGCCTCCCAGGTTCAAGTGATTCTTCTGCCTCAGCCTCCTGAGTAGCTGGGACTAC
AGGTGCCCACCACCACACCCAGCTAATTTTTGTATTTTTAGTAGAGACGGCGTTTCACCATGTTGGCCAGGATGGTCTCA
ATCCCTTGACCTCGTGATCTGCCCACCTCAGCCTCCCAAAGTGCCGGGATTACAGGTGTGAGCCACTGCACCCGGCCATG
CCCGGGTAGTTCTGTCGCCCGCACTGGAGTGTGGTGGCGCAATCTCTGCTCACTGCAAGCTCCGCCTCCCGGGTTCACGC
CATTCTCCTGCCTCAACCTCCCAAGTAGCTGGGACTACAGGCACCCACCACCACGCCTGGCTAATTTTTGTATATTTAG
TAGAGACGGGGTTTCACCATGTTAGCCAGGATGGTCTTGAACTCCTGACCTCGTGATCCACCCACCTCAGCCTCCCAAAG
TGCTGGGATTACAGGCGTGAGCCACTGCGCCCGGCCAGATAATTCTTTCTACACATTTTTTCCAAGCAGTTCTCATCATG
TCCCACACTGTCCACCCTGTCCCCCTCTCCAGGCACACCAAGCCTTCACTCTCTCCAAGCCTCCAGTGGCCAAGACTTCC
ATGAAGTCTTTCCTGACCTTTGGGTATAGTAAGTCTCTAATGGCATGAAGCAGGGTTCTGGTTTTGGCTTCATCCTGTCA
ATATTTGCCATCAGGACTTGGATAGACCCAAGGATGACTTATCTATGTTGTTATCACATCACCAGATGACAGAATTGGGA
TCAGAAAAATTTCCCTTTATAGGTTGCAACAATGGCCCAGAACCTTCAAAATGAAATCCTTTAGGCATGTTATTAAGAGG
AGTAGGACCCTGCAGGAAAGTCCAATTGTATTGTAGTTACATAGTTATAGAAGTATGGAATCTTTTTTTTTTTTTTTTT
GAGTGTCACCAAGGCTAGAGGGCAGTGGTGTGATCATGGCTCATTGCAGCCTCAACCTCCTGGGCTCAAGCGATCCGCCT
```

```
ATCTCAGCCTCCTGAGTAGCTGGGACTACAGGTGTGTGCCACCATGCCCAGCTAATTTTTGAATTTTTTGTAGAGACGAG
GTTTCACCATGTTGCCCAGGCTAGTCTCAAATTCCTGGGCTCAAAGGATTCTCCCATCTTGACCTCCCAAAGTGCTGGGA
TTACAGGCATGAGCAACCACGCCCTGCCAAGTATAGAGTCTTGAACAAGGAAATGCATATCGTCCTATATTTTTTCCTAG
TCAGATAATATCTAGACCATTAACCAGAAATCACCCAGAGGTCAAAAAACAGGGCGTCAAAGGACCCAGAAACCAAGTCT
GCAAATAACGACTGAAGACACTGTGGAAGTGTGTTTGGGAGACAACAAGACTCTCAGGATGTGCTGGCTGTCTTCAGAGG
ATGCTTATTGGAAGAGGAGTCAGACAGTCCAGACAGAAGGCACAGCCAGGACCTCTGGAGAGGAGTTACAGGAAGACATA
TTTTGACTCATCATAAGGAATAAGTTTCTAATCATGAAACCATCCTCCACTGAAACATGATCTATTGAAAGGAGCAAATG
TCTCACCTTCATTGATGTTCGTATTCATTGATTCTGGGTGATCATCTGATAAGGATGCAGTGACGAGAATCTTGCATTTG
CTGGGGGTGGGGGTGTGGTTGAGGATAGTCTGGTTTATATTCCAAAGTTCCTTTCAATTCCTCTATGATTCTATACGCTG
TACTCCTTCCTGATCAATGTCCCTAGCCAGGGTGGCCAAGGCTAAGTCAAGTATGCTAAGGGATTGGAGGGGCAGGGATA
TTGAGAATAGGGTGAATGGAAGGATGAGGAGTTCCCAGCAAGCTTGGGACACAGGAAACCTTGGGGCAGCTTCCTCCTGG
AGGTTTCAGGACTGTACGTGCTGGAGAAGAAGTGTGATGCCTTGTCCTGAAAGCCGTCTTCTTTGAAAGCAGCTTCTAAA
GGCAGTGAATGGAGAAGAGCGAGGAAACGACCCCAATACCACCAACAGAGGCTGGAAACTCCTCAGGCTGTTTAATCCTA
AGAATGATGCATCTGTTGGCCGGGCACGGTGGTTCACATCTGTAATCCTAGCACTTTGGGAGGCTGAGGCAGGCGGATCA
CCTTAGGTCAGGAATTCAAGACCAGACTGACCAACATGGAGAAACCCCATTTCTACTAAAAATACAAAATTAGCCGGGCG
TGGTAGCGCATGCCTGTAATCCCAGCTACTCGGGAGGCTGAAGCAGGAGAATTGCTTGAATCCGGGAGGTGGAGTTTGTG
GTGAGCTGAGATCTCGCCATTGCACTCCAGCCTGGGCAACGAGAGCAAAATTCTGTCTCAAAATAAATAAATAAAAATAC
AAAATTAGCCGGGCGTGGTGGTGCATGCCTGTAATCCCAGATGCTCGGGAGGCTGAGGCAGGAGAATCGCTTGAACCTGG
GAGGTGGAGGTTGTGGTGAGCCAAGATCATGCCATTGCACTCCAGCCTGGGCAAGAAGTGCAAAACTCTGTCTCCAAAAA
AAAAAAAAAAAAAAAAAAAAAGAATGATGCATCTGTTGGGGATGCAGTGGGGTAAGCATCTTCAGTAAGCAAGGTGTGAA
GAGGGGAAAGAGGGAAGGTGAATATGGAGGAGAGGGTGAAGGAGGGCACTGGAAAGGGTAGTAGGATCCCAGCAAAGGGC
GATTTGGCTGAAAGGGAGCGTGATAACAAGGGTGGGGGTGGGGCCAAGAAGCAGCCACCATGTGTGGGTGCCTCTGTGCG
TGCAGTCATCTTTCTCACATCATTGTGGATCAAGAGAGGAAATGCCCACTTCTGGAAGAAAAAGCCACAAAATGAGACTT
GGAAGGGAAATTGATCAACATCTACAAAACGGCTTCTTAAAGGAAGCGGCCCTCAGACAGGACAGAGTTGAGGGAAAGGA
CAGAGGTTATGAGTGCCTGCAAGAGTGGCAGCCTGGAGTAGAGAAAACACTAAAGGTGGAGTCAAAAGACCTGAGTTCAA
GTCCCAGCTCTGCCACTGGTTAGCTGTGGGATCTCGGGTAAGTTGCTCTCCCTCTCGGCCTCACGGACCTCCTGAATAAA
AATTGAGGGTTAGACTGGATGATCTCTGAAGCCATCTCTAGCTTGGTAGTTTATGAGGTTAATAATTTATGAGCAAACAA
AGTTAATTTATAGATAGAACATCATTATTTTTGAGTCAGAAAATGACTCTGTAACTATGTGCTTTTCTTGGTGATTTCAC
TTTTTTTTTTTTCTTTTAGAGACATGGTCTTGCTATGTTGCCCAGGCTGGTATCCAACTCCTGGCCTCAAGCCATCCTCCT
GCCTCGGCCTTCCAAAGTTGATTTCATTTTTGTCTTCCAGTATTGTTTTACTGTTGTTCCCTTTGAAAAATTCTAACCTA
TCTTTTCTGAACTTGAGCTGAGAAGGGAAGGGGTTTTGCCTCTGTTGGGCAAAAGGATGGTGGCAAGTAGTGTGACCAAG
TGCCCTAACTTGCCAGGACTTAGGGGTTTCCCAAGTCAAGGGACTTTTCAGTCCTTGGCAGACTGGAATGGTTGATCACC
CTAGTGGTGAGGAAAATGAAGGTTTGGAGGTGGCTTGGAGGAGAGGTTGCTGGGAGAGCTCTTCTCCTGTTCTCTGTTGA
TCAGGAGTTTCCCTCATCAACCTGCTGACTTTGCGTGGTGGTTTCATTACAAGTTGTGAGCCCCTTATTGGGGGTATAGG
GAGGAGAAGTGAGGGCAGCCTTTAAACTAGTCATAGGCCCCAGTTGAGACAATTATTGCCGGGAAAGAAGCTGAAATGCT
TGACTAGCACTGATGCTGTGGGGGATTTTCCCTCTTTCTTCAAAAAAACAGAAAAGACCCAGTGGAAAAAAAAAAAAAAG
TGATGAGTTGTGAGGCAGGTCGCGGCCCTACTGCCTCAGGAGACGATGCGCAGCTCATTTGCTTAAATTTGCAGCTGACG
GCTGCCACCTCTCTAGAGGCACCTGGCGGGGAGCCTCTCAACATAAGACAGTGACCAGTCTGGTGACTCACAGCCGGCAC
AGCCATGAACTACCCGCTAACGCTGGAAATGGACCTCGAGAACCTGGAGGACCTGGTGAGTAGACACGGGTAGCTTCCTG
TCGCCGAGGCCCTGTCTGGAATTCTAACATCCTTTGCCAGAGTCCGAGGGAGAGGGGACAGTGTGGGAATCCTCTCCCAC
TGTGGATCTGTAAAATCTAGACAGGTCAGTCAGCTCCCGCCCTTTAAGAGTTTATTTTCCATTCTGTGGAAGAAGCAGAT
AAGGAGAGCTGCTGTCCTTAGGAGACATCCTTTAGAGGAAGCTGGAAGACACGGGTTCAGGCCCTGCATCCTCCTCTGAG
TTGCTATGTGACTGGGAACAGGATACTTCACCTCTCCATTCTTTCTCTCCTTTTCTCTTAGGGTCGGAATATGGAACTAG
ACAGGAAAGTACTTTGGAGGTTTTCTTACCGTAAGGAGGCTGGCATTGCTAATCAGTCAATGAATGCATAGCTGTTCGTC
TCTGGCCATATGATTATGTCATCAGGCACTACATTCTGTGTTGGGTGGAGAAGTTGAGGACCTGGTCTTTGCCCTCAAA
TAGCTTCTGCTCTGGCCAGAGAAAAAATCACTCACTGGAAGCTATTAGGCAACAATTATGTGCTAAACTGAGGGAATGT
GAGACCAGATGGTAGCAAGGAAGAGGATCCCAGCCAGGCTGGAGTCAGGGAGCAGGGTGAGAAAACTGTTAATCTGACAAA
TGTCTTGGAACCTGTTGTAGAATCTCTAGAGATAAGGTATTCTTGGAAAGGTTTCTTGGGTGGGCTAAGAAAATGGTCTC
CAGCACTACGCTGCCACCACTCAAGTCTCTCCAGTGAAGCGTAGTGGTGCAGTTGACTTGGTTGTCACTGGGCTCCCAGA
AGATGTGTGTGTGTGTGGAGCAGCTCAACCAGAGAAACACAGGGTTCCTTGTGAGCCGACGGCAGAGGCTGGTTGTAAGG
GTTTCTTGGAAACTTCTTCACTGTCTTTTCTCCCCCTGGAGAGAGGACTTGTTTCGGGGCTGCACTCTGGGTTAGATCAT
GAAGGAGCCAAACCCTGGCTCCTACCAACTCTCCTGTCCCAAACTTACCCTAAGATATTGTCTGACTCCCTCCTGCTCCC
CCGGCTGGAGTGGACTTTGTGGAGTGGACTTTGTAGTTCCACCAACCATGTTCCCAGACTTGCTCAGAGAAGCCCGCCTC
TCGCTTGCCCAGATCCCAAGGCTGGGAAGTGTAAGTAGGACACGGGGAGAAAGTGGGCAGAAGGGCTGACTACCCAAAGC
CTGGCCCCTCGGATCCAAACCGTAGGTGGAATCAGAGCCGGACCATTCATCTAACCAGGACCTGCTACATAATTTGTGGG
ACCCAATGCAAAATGAAAACATGGGGCCCCTTGTTGAAAATTATTATAAATAAGAAGGCAACAGCAGAGCACTAAACCT
CGTGGGGGGTCCTGCTGGGTGCAGCCCCCGTGTGACTGCCCAGTTGCAAGCCCATGAAGCTGCCCTGAGTCTAATCAC
AGAGTCTGGGCAATACCCCAAATCAGACTAAAAACCCGCGGCCCCAAAGGGGCAATGTGCTCCCACCCTGGCCCCCGGTC
ACAGCCGCTGGCCTCGCCTGATTAGGTTACTGGGCGTCTTCCTCTGCCTGCAGCTTCAAACTGACCACGAGCCCTGACCC
AGTTGGACATCTTAGTAACCAACTGGAGACCCGGCGGGTCCCAGCTTCTGAGAAACCTTAAGAGTTTCCCCTAACTGCAC
GCCTCACCTCACTGAGTGACCTACTAGGGACAGGTGGGGGCAGGGGGCAGGGGCAGCAGGGCATGTCCCAAGAAAACCTA
```

CATCTGGGGGAAAGGGACCCAGTGACTTTCTTCATGTCACACCTCAAATGAGTGATCCTCAGCCAGGCATTCCAATATCT
GGATCCCTCACTCAGGGTCTAGCCAGGAGCCCAGGAGTCCTAGGACCATCTGGGCTCTGGAGCTCTTCAGCCAGCCACCA
GTGCCTCGTTCCCCCTTCACGAATCTCCTACACACCCTGTCTCCAGGGCCAATGTCCCCTCAGAACACAGACCCTCTCCT
CCGCCCCCACCCACTCACTGAAGGGCCGTGAGCAGCTTGGCAGACTGTCCCTCAGTGTCACAGTACAAAAACCTCGGGCG
TAGTGGTACAGGCAGGAGAATCTTGCATGTAGCAGCTTCTCAATAAGTATTTATTGATTTGTGATGCAGTAAAGGTCTTA
GCTCACCATTTTCCTCTGGAGTTAGTCTGGGTCCTTCTATTCCTTCTGCTTCCTATTCTCCTCCTTGTCCTTGCCCATAC
CAGGTATTGGTTCACATTCCTTGAACGCTGGGTTTTACACTCTCATGTTGAGCTAAGAACTGGGGCCTTTTGGTTACACA
AACTTGCCAGAAAAAAAAAAAAAAAAGGAACTGGGGGCTCCTGGCCATAAGCCAGGTTCGTCATTCTGTATTCCATTTGTC
CTCTTGAACTCGAAGACCAACTCGAGCCGCTTAAAATGTCCACTTCTTTAAGGCAGGGGCTGGAGGGTGGGGAGGGGCTG
CTAATTCTCATGAATGAAAGGTCCAAATAGAACAAAAATTAGATGGCTAAGAACCAGGAACAAGAAGATTCCAGGTCCTG
GGGTGGGTATGCCAGGGACCCATCCTGCTGGAAAGGCCGATCCTGGCAGACCTAGAGCAGACCCTGTCTATGCGACCCTC
GATCTGAGAGCCTCCTTCCAACAGAACCAGGGCCTGGGCTGGGGCATCCACAGAGGCTGGTCATAAACTATACCTCCAGC
CCCACCCTGCACACCAGCCGGGAGGGGAAGCTGCTATAGCGCACGGTGAAGCTGGTCCAAGCCCCAGAGCCTGGCTTTCT
ATGACCCCTCTCCATAGACAATTACCACACATCATGACTTCAGTGTGACCAGAGCATCACAGGCGACACAGCCCGTCCAC
GTGGACCATCTCATTTCATCATGTAGGTGTGAAGAAGAGGAACCAAACCAGCTCAGGCTGTTTCTGGGCTTCATCTAGG
GGAAAGGGAGGTTCTCCACCCGCCCCCCCGCAACCAGGTCAAAAGTCCCAGAGGAACCCCAGCAGAGGAGGCCCAACTGGG
GTGTAGTTGGAAGGGTCAGAGATGAGGGAGAGCCCCAAGGCCAAGCTGAGGATGGGTCAGGGTCAAAGTGAATATGGGTT
CGGGCATACAAACTGCAGAGTCTCCGTCAGGAACTCTTGCTTTCCCCAGGGACGCAGGCTGCAGCTGATCCCACTCCTCT
AGCAGAACGGCAGAAACAGTGGCCGCATATGTGGACCTAAGAATCAGGACACAAGAATGAGCATTCTTTCAAGAGTGGTG
GGGAAAAATATTTCCAACCCAGGAGATGGTTTCAAGAGGCTGCCACACACACCTGGGAGGCCTGTCTGCCTGCATCTTGG
ACTGTGTGACTCTGTATCCAAAGGGCAAAGAGACGCTGGGGTGCCAGGTGGGGCTCCCTTAAGCCTAAAGACTTGCTGCA
GAGGCTTGGAGTAGCTTAAACTCTCCCCCTAACACCACATCCAGCACAGTACCTGCTTCTGATGGCCAAAACCCGTGGGT
AGAGATGAAAGAGGAGTCTCACAGACTTGTCCCTGAAGGCTGTGGGTGCGGCAAGGGAGATGTGACAAATGGCAAGCCAG
AGTCTCAGATGGTCTTTAGGGGCTGTGGGCCTCCTGAAGAAAGACGGGGTAAGGGGTACAGACCTACACCCTCACCCACA
GCCCAGTCTCATTTCTCCAGACCCCTGCACCCCTTAAGCCACTGAAGGCTGGAATGACAGATACCCTGGCCAGCCCCACT
CACAGCTCCTGACTCTCGGCTGGGAATATCCACACAGGGACATAAAAGGATATGGAGCTTGTGTGCTCCCCCCTGGCCAG
AGCAATGACTGGCGTCTGTGTGCAGACACCTCCCGCGGCCAGCATGACTCAGGCCTTGGAGTCCTGGGGTTCGGCCTGAG
TAAGCCACGCATGCAGGCAGCCCTGCCCCCTCCTTGGGGCTGAGAAGCAAGGTCAGGCTGGGGCATTCACTGGCGGCAGA
ATGGACTAGAAATACCAAGTCTCCGGCTACTGAGCTAGACGGAGATCATGCTGAGAACCAGCTCAGCCCCAGGCAGGGCA
GGACAGACAGAGAATCTGGGAGTGGGAGCGAGGGGCATTTGAAGGCAGAGTCCAGCCTCCTTCTTCCCAGGGCAGGAATC
TCTTCCCACACACTTGGGCTCAAACGTTCCTCTCCTTCCTAAAGGTACCATCTCATTGCTGGACAGTTCTGTCTGTTAGA
AAGTTCTCCCTCCTTTGACTGAAACGCTACCCCCTCAGCTTCTGCCCAGTCCTTCTAGACTGCCCCCTGGAAGCTCATAG
CAAGAGTCCATTGCTGCCCTCTTGCCCATGGAGCTTCTTTCCAAGCCTTGACATCAGCCCTCAGGGACTCAAGTCTTCTC
TGTCCCAGACACTACACCCCTAGTACCTGAAAATTTCCTCTATGGGCAGGGTTTTCCAAGCCCCTCAACAAAGTGGTCAT
CCTTGTCCAGACCAACCAAACTGGGCTGGTGTCAGCTGCCCACTTACAAGCCTCTGTTCACCTAGATGAAGGTAACAGTG
GCCCTTCGGCTGGCCACGTCACTCCCGTGACTCATCTTAAGCTTGTAGGTGACAAACTCCCAAGTCTTTCTGACGAACTG
TTGTTACCTGGTCCTCCCTAACTTATCCCTGGACGGGTAATGTTTTAAACCTGAGTCCTGGCTTGTATATTTTTCCCCAT
GCAATTTCATCTGGTTAGGTTGGACCCATGGTTCCAGCTTGTAAAGAACTCTGTGTTGCCTGAGAGTGCCCCCAAAGCTG
GGCTGAGAGTGCTGAAGAGGAGGGGACCCAGGATAGATCCCTGAGGCATGCTAGGCACCCCTGCCCAATGCACCTGCTGA
CATAGAATCCCCATTAGCAAATGTGAGCTGAGCTCCATGCTGGCCAGTGCTGGGCAGTGGGGCAGCCTCATTATTTGGAG
AAGTCAGGTGGGGCTTGGAGACTTAATTATCCAGGGAGGAGCCTGCATCCTACAGTGGAGGGAAGGAGAAGAGTAACATT
TTGGGCCCTACCACAGGCCAAGCACTTTGCATCTCCTTACACCCCCCACCTCCCGCTAAGCCCCTCAATACTGGACACTG
TTACAGCCCCTGCTCCTCCCCAGTGCTCACAGCAATTTTCTAACTAGATTCTAGCTCCACTCTCAGCCTCATTCTGGTCA
TTGTGTTTCTTGCCTTCTTTGGGGCATGTGACCCTGGCCCATGACCAGGCTGTTGTCTGCTGGGTGCCTGTCTAGGCCAC
ATGCTGGTGTGGCCCATCTTGCCACCTGTGTCTATGCAAGGTGCATGTTGCTGTCAAGAGTGTGTGTGTGCACGAGGGCC
TCAGGTTTCAGAAGCTTTTCATGTCAAAGAATGGGCACACCGCTCACGCAAATGGTCTGCCCTGTGCTCCCTCTGGGCAT
GGGATTGGAGGGCTTCCTTCAGCAGCACATGCCCCATGGTATTCAGGATTCCTGATTCCTCAATTTCCCATGAGGCCGGA
GCCAACCTTGCTTTATTGCCTCTCTCCATAGTTTGGGGGAGATTGGGGGAGCTAGAACTCCTATGTGTGGGAAGTGGCTT
TAACATGTGCCACTCCCGAACCCCCAAAACGTCTGTTTGACACCTCCTGCTGAGATGTGCTGTCAATTCCCAGCACCCCT
CAAACCAGCTTCCTCCCATCTTACCCTCTCTGAACGCTGCCAGACAAATACCTCTTGGCAAGGAGGAAGACACAGGCCCC
TCCCAAGCCCCCTCTAAATCTGGTTCTCAGGAGATGGGAGCCCTAGGGAGATGTCCCACTGCATTGACTTTAGATGAGCA
ATAACTCAGTCAGTCTGGAATCTTTGAAGGGCAAGAACTTGTGTGGAGGACAAGCCCAGAAACCCAGGGCTTTCTGGGTC
AGACAGGCTGGCTGGACCTAAGCCCTCACCCAGGCACGGCCAAGGTAGGTGGAGTCCAGCCCTGCTGACCGCCCCAGGCC
CTGCTTCTCTCCTCCCTGTCTGAACGGCTTGGCTCTGCGTGAAGACTGCTGAATTCCAGTATTAGTCAAGTGGGTGTGCT
CAGTGACGGTATTGGAAATAGCTCCCAATAGAGGGAGAGCACTCAAGATAGGATCTAGGATCAAGGCCATCAAGGTTATT
GGGAGAAGGCAGAATGCAGAAGATTACGGAGTGAGTCCCTGCAGAGGCACTTGTCTAAGTCTCAGGTTATAAGGGCTCCT
GGCCTTCTCTGGGAAACTGCTTTCAGAGCAGGTCAGCACACGTCTGTCCTACGTAAAACACGTGGCCTTGGGAGGGGGAC
ACAGAAGGCCTGCTTCTGAGACCTTGCCATCTGGTTAGAAAGTGTCATCCCAACCCTGGGTGCTGTAATAAATGCACACG
CATCATAAGGAGAAGAGAGAATTCCAAGAGGGGGAGCCGGGAAGGATGTCTGAAGATGGGATTGAATTCGGCTTTGGAGGA
AGGGTGGAAATTAGGCAAATTTGGAAAGAAAGACCTGCTAGACAGAAGGAAAAGGCCAAGCAGAGGTTTGGAGACAGGAA
TGTGTTGGGGTGTTGGGGAAAGGCCGGGGGGTGGTCTAGAGCAATAATTTATTAATAGAAGACAAAGTTTGAAAGGTCAG

```
AGGAATATACATTTGGGAAGAACATAAAGGCTGTGTTGGGAGGTTAGACGTCTTCCCACAGGTCCATGGTTTTCAAATGA
AAGTAGAACCATTTTCTTCAATGAAAGCTTATGTGTGAAGCACTGCACAAGTCAAAAGTGGAGAGCTCTAGTTTAAAGTC
AAGGAGGGGAGCCTGGAGCCCCAACACCACTCCCCACTCCAGGGGAACCCTGTGACACACACAGTCATGGGTACTCTA
AAGAAAGTCCTTGGAGCTGGGCACTGTTGTCACATACCTGCAGTCCTAGCTACCCAGAAGGCTGGGGCAGGAGGAGGATC
ACTTGGGCCCAGGAGGTCAAGGCTGTAGTGTGCTATGGTCACACGTATTAATAGCCACTGCACTCCAGCCTGGGCAACAT
AGCAAGACACTATCTATCAAAAAACAAAAACAAAGGCGTGTGTTTGGGGGAGAAATTCCATGGAATCCTACTGCATGGGA
TGTTCTGCAATGAGTTCTAAACTTTATTTTTTAAAAAAATGTTTTGTAGAGATGGGGTCTCGCTATGTCATGCAGGCTGG
TCTTGAACTCCTGGCTTCAAGCAATCCTCCCACCTAAGCCTCCCAAAGTGCTGGGAGCCACTGTGCCCAGGAGTTCTGAA
ATTTAGCGATGTGTGAATGGCACTAAGTTTGGCTTACTCCAATCATTTTCTTTTTTTTTGAGATTGGGTCTTGGTCTGTT
ACCCAGGCTGGAGCGTAGCAGCATAATCATAGTTCACTGCAGTCCCAAATTCCCTGGCTCAAGTGATCCTCCCGCCTTGT
CTTCCCAAAGTGCTGGGATTACAGGCCTGAGCCACCATGCCCAGACTCCAATCAATCTTCTATTGATGAACATGATCACA
AATATGACTTTTTCTTATAGCTGACTTTGCTATTAAATGTGAAATTGAGGCCAGGTATGGTAGCTCATGCCTGTAATCCC
ACCACTTTGGGAGGCCGAGGCGGGGGGATCACTTGAGGTCAGGAGTTTGAGACCAGCCTGGCCAACATGGTGAAACCTTG
TCTCTACTAAAAATACAAAAAAATTACCTAGGTGTGGTGGCAGATGCCTGGAATCCCAACTACTTGGGAGGCTGAGGCAG
GAGAATTGCTTGAACCCAGGAGGCGGAGGTTGCAGTGAGCCGAGATCGTGCCACTGCACTCCAGCCTGGGCAACAAGAGC
AAAACTCCACCTCAAAAAAAAAAAAAAAAAAAAAAGTGAAGTTGAAAAAATAAGATAAAGACAAAAAGCAGAAGAAAACCA
CTGCTTACAGGTGATGGGGAACCAGTAAAGGACTTTAAACAGAACATTGATGCGATGGGATTTGTGTTTTAGAAAACTTA
CCCTGTCCTCTGTGCAGGAGGTGGACAGGAGTAGAGAAGCTGGTGGGGATGGGACCCATGGAGAGATGGTCTCCTGAGCA
TTAGAGTAAAGAGAAGGAAGACCAGACTGAGGCAATGGCAGAGGGATGCTGCGGGAAGGGTTGGCAGATGGCTCTACCCT
TGCACTGATGTATCTCCCACACCTCCTTCTCACAGACCCTCATTCCCCCAGAGCCAGAAGGAAAGACCCAGGTTGGAGAA
TGGGTCTGCTCAGCATTAGGAGTGAGTGTGTGACCCACAACTGTGGCTCCTGAGAGGCAAGAAAGCCCTGTGGCACCCAG
TGTGACCATCCCCTCCTTGTGGGGGTGCCTTGCTCTCCTGAAATGGGGGCACCATTAAGCCACCCGGAGCCTGGTGGACA
GAGTACCCCATCCCATCCTCTGCCTCCCTGGGTTCATCATTTATAGTATTGCCATTTTGAATCCACATAGGTTGGTTATA
ATTGGTTTTGAATTGCCTGGGGAGGGGCTTGGGTAGGGAGGGTGGGAAGAACAGAGCTGGGAGGTGAGGAGGAACAGGTG
CAGGGGACAGCTGTGAGTGAAAGGTGTGAAAACAGGCACATGTCTTCCTTTGTGGTTAGCTGGGTAAACAACCTGAGTGC
TGGGGTGATGGGGGGGGGGTGATGGGGAGAGGAAGAGGAGGATGGGGAGGAGGGAACTTCCGGAGACATCACGGGCACAC
CCCCTGCACTTGGAGCAGGCTCTCTCCTTCTCTCCTGCCAGTCTCCCCGTGTCGCCCTCACTTTCTCCTGCCTCTCTCTG
CCTCTGGGCTTTCTGCTTCTGAGTGCTCCTAGCTGCAGGGAGGTTGCCAGTGATGGGAAGTAGCAGGGAGCCTGCATCTG
CTGCCCCTAGAAGGAAGCCCAGGCAGGGTTGTTCAGTGGGAGGTACTGGCTTTGGAAGTAGATAAACGTCGGTTCACACA
AACCTGATCTTTAGCAAGACACATCTGTGAAATAGGAATAATATTACATACCTTGAGCGGGTTGTGCATATTGAACCAGA
TGGGAGGCTATTACTGCCATTTCAGTGATAACAGAGACAGTCAGGAACAGGAAGACCCATGAGGAGGGAGCCCACAGGCC
AAGTCAGCACCCGCCCGCCCCTAGTCCCCAGCACAATGCTAAGTTGCAGTGCCCGACTGTTCTGCTCAGAACTGGAACAG
ATGGGTCCTTGTACCAAGCAGGTCCATTCCCCAAATTGAAGTCCTGTGACTCCAGCCGCCAAGGCTGCAGGCTTCCGTAC
ATGAGGACCCAAAAACACAAGCTGACTTATGGGGTCCAGCCCTCCAGCACTTACAAAGCTCAGCCACCCGCACGCCTCCC
TTCATCAGCACCACCACTCTAAGGAATGCGGTCCCTTTGACAGGCGAAAAACTGAAGTTGGAAAAGACAAAGTGATTTGT
TCAAAATTGAAATTTGAAACTTGACATTTGGTCAGTGGGCCCTATGTAGGAAAAAACCTCCAAGAGAGCTAGGGTTCCTC
TCAGAGAGGAAAGACAGGTCCTTAGGTCCTCACCCTCCCGTCTCCTTGCCCTTGCAGTTCTGGGAACTGGACAGATTGGA
CAACTATAACGACACCTCCCTGGTGGAAAATCATCTCTGCCCTGCCACAGAGGGGCCCCTCATGGCCTCCTTCAAGGCCG
TGTTCGTGCCCGTGGCCTACAGCCTCATCTTCCTCCTGGGCGTGATCGGCAACGTCCTGGTGCTGGTGATCCTGGAGCGG
CACCGGCAGACACGCAGTTCCACGGAGACCTTCCTGTTCCACCTGGCCGTGGCCGACCTCCTGCTGGTCTTCATCTTGCC
CTTTGCCGTGGCCGAGGGCTCTGTGGGCTGGGTCCTGGGGACCTTCCTCTGCAAAACTGTGATTGCCCTGCACAAAGTCA
ACTTCTACTGCAGCAGCCTGCTCCTGGCCTGCATCGCCGTGGACCGCTACCTGGCCATTGTCCACGCCGTCCATGCCTAC
CGCCACCGCCGCCTCCTCTCCATCCACATCACCTGTGGGACCATCTGGCTGGTGGGCTTCCTCCTTGCCTTGCCAGAGAT
TCTCTTCGCCAAAGTCAGCCAAGGCCATCACAACAACTCCCTGCCACGTTGCACCTTCTCCCAAGAGAACCAAGCAGAAA
CGCATGCCTGGTTCACCTCCCGATTCCTCTACCATGTGGCGGGATTCCTGCTGCCCATGCTGGTGATGGGCTGGTGCTAC
GTGGGGGTAGTGCACAGGTTGCGCCAGGCCCAGCGGCGCCCTCAGCGGCAGAAGGCAGTCAGGGTGGCCATCCTGGTGAC
AAGCATCTTCTTCCTCTGCTGGTCACCCTACCACATCGTCATCTTCCTGGACACCCTGGCGAGGCTGAAGGCCGTGGACA
ATACCTGCAAGCTGAATGGCTCTCTCCCCGTGGCCATCACCATGTGTGAGTTCCTGGGCCTGGCCCACTGCTGCCTCAAC
CCCATGCTCTACACTTTCGCCGGCGTGAAGTTCCGCAGTGACCTGTCGCGGCTCCTGACGAAGCTGGGCTGTACCGGCCC
TGCCTCCCTGTGCCAGCTCTTCCCTAGCTGGCGCAGGAGCAGTCTCTCTGAGTCAGAGAATGCCACCTCTCTCACCACGT
TCTAGGTCCCAGTGTCCCCTTTTATTGCTGCTTTTCCTTGGGGCAGGCAGTGATGCTGGATGCTCCTTCCAACAGGAGCT
GGGATCCTAAGGGCTCACCGTGGCTAAGAGTGTCCTAGGAGTATCCTCATTTGGGGTAGCTAGAGGAACCAACCCCCATT
TCTAGAACATCCCTGCCAGCTCTTCTGCCGGCCCTGGGGCTAGGCTGGAGCCCAGGGAGCGGAAAGCAGCTCAAAGGCAC
AGTGAAGGCTGTCCTTACCCATCTGCACCCCCCTGGGCTGAGAGAACCTCACGCACCTCCCATCCTAATCATCCAATGCT
CAAGAAACAACTTCTACTTCTGCCCTTGCCAACGGAGAGCGCCTGCCCCTCCCAGAACACACTCCATCAGCTTAGGGGCT
GCTGACCTCCACAGCTTCCCCTCTCTCCTCCTGCCCACCTGTCAAACAAAGCCAGAAGCTGAGCACCAGGGGATGAGTGG
AGGTTAAGGCTGAGGAAAGGCCAGCTGGCAGCAGAGTGTGGCCTTCGGACAACTCAGTCCCTAAAAACACAGACATTCTG
CCAGGCCCCCAAGCCTGCAGTCATCTTGACCAAGCAGGAAGCTCAGACTGGTTGAGTTCAGGTAGCTGCCCCTGGCTCTG
ACCGAAACAGCGCTGGGTCCACCCCATGTCACCGGATCCTGGGTGGTCTGCAGGCAGGGCTGACTCTAGGTGCCCTTGGA
GGCCAGCCAGTGACCTGAGGAAGCGTGAAGGCCGAGAAGCAAGAAAGAAACCCGACAGAGGGAAGAAAAGAGCTTTCTTC
CCGAACCCCAAGGAGGGGAGATGGATCAATCAAACCCGGCGGTCCCCTCCGCCAGGCGAGATGGGGTGGGGTGGAGAACTC
```

```
CTAGGGTGGCTGGGTCCAGGGGATGGGAGGTTGTGGGCATTGATGGGGAAGGAGGCTGGCTTGTCCCCTCCTCACTCCCT
TCCCATAAGCTATAGACCCGAGGAAACTCAGAGTCGGAACGGAGAAAGGTGGACTGGAAGGGGCCCGTGGGAGTCATCTC
AACCATCCCCTCCGTGGCATCACCTTAGGCAGGGAAGTGTAAGAAACACACTGAGGCAGGGAAGTCCCCAGGCCCCAGGA
AGCCGTGCCCTGCCCCCGTGAGGATGTCACTCAGATGGAACCGCAGGAAGCTGCTCCGTGCTTGTTTGCTCACCTGGGGT
GTGGGAGGCCCGTCCGGCAGTTCTGGGTGCTCCCTACCACCTCCCCAGCCTTTGATCAGGTGGGGAGTCAGGGACCCCTG
CCCTTGTCCCACTCAAGCCAAGCAGCCAAGCTCCTTGGGAGGCCCCACTGGGGAAATAACAGCTGTGGCTCACGTGAGAG
TGTCTTCACGGCAGGACAACGAGGAAGCCCTAAGACGTCCCTTTTTTCTCTGAGTATCTCCTCGCAAGCTGGGTAATCGA
TGGGGGAGTCTGAAGCAGATGCAAAGAGGCAAGAGGCTGGATTTTGAATTTTCTTTTTAATAAAAAGGCACCTATAAAAC
AGGTCAATACAGTACAGGCAGCACAGAGACCCCCGGAACAAGCCTAAAAATTGTTTCAAAATAAAAACCAAGAAGATGTC
TTCACATATTGTATTTATATATTTATATTTATATATATATTTATATAATGGTACAAAATGGCTGGGGGTGTGGCCATGGA
TGGAGGGAAGAGTAGGCTGGCCTGTGGCGTGGGTGGGAGGAGAGGGGACGGAGAGGGCACTCGGCCCGCTGCAATCTGAC
CCCTCTCTCCTCAGGGCAGGAAACACAGAGTCAGACAGTTTGGGGGGGTCTTGGGCCAGGGGTGGAGGGCTCAAGGGCAC
AGGGCCCAGGCTGAGGCAGGGCGGGCAAAGCGCCTGGCAGGATGAAGGGCAAGTGGCCCCCCAAACACAGAGGCCCTGGC
CATGGACCCTGGGAGGTGACCGGGGTGAGTCAGGGGCCTGTTGTCAGCCCCAGAGGAAGCGCTGGACCTGGCCGATGGTG
GGCCGAGAGGACAGCACCAGGCTGGGAGAAGTGGGGCGAGTTCCCTTTGTATTACAGCTGCCAGTGCAAGACCAGGCCCT
CCAGGCCAGGAAGGCTAGGGACGGGTCCTGGTAGAAGACACCCTGTCTAGAATGGCCCTTGGTCCTGGAGGTGGGGCGCA
AAAGGCCTCAGCCAGGGAACTGCCCTGCCACCTCCCGAGGCAGGAAAGGAAGTGAGAAAAGGAGAAGTTTTTTTACTCCT
GGGGCCAAAGTAGGGGGACAAACACCCAGTCGTATATGGCTTCAGCTCTGACCAAAGGCGGATAGGGAGCTCTCCTGGGT
AGGAGCAGGGAGCCAAGGGGGAGGCAGTGGCTGTGCCTGGGTGGGCACAGACAGATCTGGTACATGGCCCTGAGCCCTGG
GCAGAGGGACAACCTTGCCGGTGAGTGGGCAGGCAGAGAGGAGGCGGCAGGATGCTGTTTCCCCGATTCCATCCTCAGGG
AGTGGAGACTGGAGGGGAGGTGCACTGACTCAGATGAACTGTTCTCCCCCTTCTTTGATAAGAAGTAGGTGGCAGCAGCC
TCTGGAAAAGTCAGGGCCCTGGAGGTTACCTGGCCCAGGGCTACTACAGCCACAGGCCCCAGTGGCACCATGCCACCCCT
TCCATGGCTCCACTCAAGGGGGCCACACAGCCACCGCCTCTTCCTCCTTTCCTTCATCCCAAACTGGGACAAAAGACTTC
AAGTTCTGGCTAAGATGTAGCAGCAGCGGATGCCCGGGCATCCAAAGTGGAAAGCCAGGGCCCCGTGTCACCGGTGTGGG
CAAACACACATGCACGTGCACACATGTTCTCCCTGAATCACTCAGCAGCAGACAGGCTGCCGCCCTGGGGGTCTCAGCCC
TGCTAGGGCTCACCAGGTGGAAGCCTAGGTGGTCTGACCTCAGTTTAGGAGTGGGTCATTTACGTCATCTTACCATTTGG
GGACGAGACAGGAATGGTATCCCTTAGGGACCCAGAGACACTGCAAACAGTGGGTGGCCATGTAGGGCTGCATGTCCCTG
GGTCCAGGGGAATGGAGGGAGCAATAACTTGAAGAAGGGGGGAAGGGTTTCTTTTATCCTTTTTTTTTTGTGTGACTTCT
ATCAAAACACAGAAATACAGCACACGCACAAACCAGCACAAAAGCGCAGCGCTCTATTTACAGCTCCGGCTGGCACCTGC
CCACCTGGCCAGCCGCCTGCAGGGAGGGGTGGGAGAGGGGTCAGCCACATCAGCAGGGAAAGGATACGAAGCAGGTAAAG
ACAGAAGGAAAATGGGCGGGTGCGGAGAAACCAAGAGGGAGGTGGAGCTCCAGCAATGCGGACACGAGGAGACAGGAGCA
GAAGGGGCTGGGGGAGACCTGACCTGTCCTTCCTCTCTCCCCCCAGATTCCCATCCAGGACTCCAAAAGCATAGCTGAAG
GGGGTGTGGCGGGTGCAGGGATGCACGGAAAGAGGTAAAATAGAGAGGCTCCATAGGAATTGGGAGGAGTGGGGGAGGGG
CAGTCCTGGTGGCCGAAATGGAACCAACCCCAATGCAAAATCCCCCACCCCACACTGCCACTTCCCCCTAAGCTGCCAGC
ACATCTGGTTTCCACAAATGCCACTCCCTACACAAGCCCCCTCCCACCCCCTCCACCCCACCCCACGACCCAGGCCATCC
CAACATTGAGGGGAAGAACTTTGTTAAGGTTATCGTATTTGCAACATTGCCCCGGCTCCAGCCCTGGCAGCGACTTCTAG
AAGGGCAGGTTGGCCATGCCGCCTGGTGCCGGGAGGTAGCCCATCTGGCTGTCCAGGGACACACTGCGCTGCCGAAGGGG
GTGGGACACCATGAGGTTCTGCTGGGGCGGGGGGCCCATGAGGGAGCCCTGCGGGGAGAGCATGTGGGGCGGCTGGCTGT
AGACCTCGCCCCCCCACGCCCCGCTGCTTCATCAGCATGAAATTCTGCTGGGTCATGAGGCCTTGTGGAGGGGACATGACC
CCCTGGTGCAGGCCATGGGGCACCATCCCCTGCTGTGGGGGCACGCCTGTCCTGCCCAGCAAGGACATCTGTCCAGGGTG
GCACATGGACATGTTGAGCCCCCGCTGGACGCCCTGCTGGCCTGGGAGGTTGGGAGCCCGAGAGGGAGTCTGCTCCGCCA
TCATGTTCTGCAGGTTCATGAGATGCAGATTAGGGGGCTGAGCCTTGGGGGGCTGGTTCTCGCTCTTGGGGAAGTACTGG
AGGGTGCTGCTTGGCTTCTCAGAGGGGATGATCCTCGACAAGTCGAACTCGGGGATCCCCGTTGGGGTGGGCCGGATCAC
CTCGCTCAGCTCGGGGTCGTTCAGCACTGATGCCACCCCAGGGAGCACACCCGGTGGGTATGCGTCGCCCATGCGCCCAG
CCATGGCTTTGCCCATCAGGTGCTGCTGGGGAGGCATGGGGCCTGGCGGCTGGTTGGGCAGGTCCTCGGGAGGCAGGGCC
ATGCCTGACGGGTAGTGCTGCTGCAGGCCAGGCCCCCGCCCCCACCCCAGTGGGGGCCATGGCACCATGGGGCTGCTG
CAGCCGAGGGGGGAAGGGCATCATCTGGGAGGAGCTGGGCACTGGGCCACAGGGGGCATTCAGGGAGTCGGGGCCCCCTG
GGGCCATCATCATGGAGTTTTGAGGGGGCCCCCCTGTCCCTGTGCGTTGGGATGCAGTGGAATGTTGGAGCCCAGAGGG
GTGGGGGAGGGCAGCATGGCGGGCGGGGGCTCATGGGACAGGGGCTGCTGGCCTGGCAGGTTCATGCCCATAGGGCTCTG
GGCAGCGGCTGAGTTCAGGTGCATCTGGCTGGGCTGGCTGTTGCTGATCCCTGTAGGGAATAGAAGACAGGGGGTCAGGC
ACGGTGTGCCCAGCTCGGGGAGGGTGCAGGGCTCAGGCCTTCCCCACTCCCAAGCCAGGGGGTGCCAGAGGTCAAAGTCT
TCACCACTGGCTTCCCCTGGCTGGGAGGGAAGGGACTGGAGGCATTGATGCTAGAAGCCAAGAGACCAAGTTCTGGCCCA
GTTCGCCAGACACACCACCAGGTGTTGGAAAGACCACTCGAGTGCTCTGGGCTGGGTTTCCCTAATGACTCCAGCGAGTG
GGAACAGCATCCCGGAGCCTCCACCCCCACACCCAGGGCCTTCAGAGGCACAAAAAAGCCAGCACAACCACAGCTGTGCT
CCCCGCTCCCAGTGCCCACACCAGCTGGCCTCCCTGGGGCCCTGGCCTGTCCTCGCACCTGGCCCGGAGCCCTGCGGTGG
TGGTGGGGGGGCAGCAGGGGCCGGTCGGGCAGCAGCTCGTCGTCTGAGGTGGCGATGGTCTTGATGGCATTGTGGTAGA
GCGGGGTGGAGCTGGGCATGGCGTACTTGGACATCTGGGTCATCATCAGTGACAGGGGGTTCTGGGAAGGTGTGGGGTCT
GGGGAGGAAGTGAATGGTAGGCTGGGGTTCATGAGGCCGCTGGGAGGGTTGGCGGGAGGTGCTGAGGAGCTGCTCCGGGG
GCCGCTAGGCGGGAGGGTACCTACAGAAACGGGGAGACAAAGAGAGCAGGGGTGACTGGGGAGGGGCAGATGAGTTTGGC
TGTGGATATGGGGAGGTTTAGGAAGCGGTCAGTTCCCCAAATCACCTGGTCCTTCAGACACACCCACAGGCCCCCACTAT
CTCCAGAGCCCACCAGTCTTCAAGCAATTCCTGCAGCCTCAGGGCCCCAGGCAAGGGGAAGATCCCAGGCCCCAAGTACG
```

```
ATCACTCAAAATGCCTGGGAGACTCACGTGGTACCCCCTGAGAAAGCCCACAAATGTCATCATCTGCCCCATAAGCAGAG
CCATCCACCTCTGGGCCCGTGGTACACAGGCCCTTACTCACTCACTGCCCAGCCCCAGCATGGACACACTGCTCAGCGCC
TGCCTGCCTGCCTGCCTGCCTGCGCAATTGACTCACCCTGTTCCATGTTGCTCAGGGTGGTGGAAGAGTTCATGTT
GAGAGGCGGCTGCTTGTTCTGGGAGACCCCCGGGCTGGGCATGGCCGTCTTAGGTGAGGCAACCCAGCCTGGAGAAGGCA
CCGCCATGGAAGGAGACTTGAGCCTGCTGGGCGAGCCAGTGGGTGAACGGACACTGAGGGAGGAGCCGAGGACCTGGGGC
GACTTGAGAGGTCCTGGCGGGTTGGCAGAAGGCAAGGGCACCATCTGTGAGGGAGTCTGGGGTGACTTGAGGTTGGCAGA
GGGCGAGGTGACCAGGGGTGAGTGCACCTGGCTAAGGGTGGGCGACTTCAAGTGCCCCATGCCCGGTGAGCCCATCTGAT
TAATACTGATGGTGAGGTCCGAAGGCCGCCTGCCTAGCCCCCGGTTTGGGGCTGAATGCACGGTCCCAGGAGGATTGGAC
GCAGGGGGCAGAGGCATGTGGCTGAGCCGGGTGGTGCCCACGTTGCTCATGGGCATTGAGCTCTGATCAGGGCTGAACAT
GTCTTGGGTATTGCCCATGTCCTGGGACATGGCTTGGTAGGGTCCCTGGCCTCCAGAGAATCCCTGCTGGCCCTGGTTGG
GAAACTGTGAAGGCATCAGCATCTTCTGCGGGCCGCCCATCACGCCACTGCTGTTCTGGGCCCGAACCCGGGCCATCTCC
TCAGGACTGAGGCCCTGGGGCCCCATCAAGTCCCCAGGGCCCCGCATCTTCTGCGACATCAGCATCTGCTGCTGCGGGGT
CATCTGCACGTTCAGGTTCATGTTCATGTTCATGTTGACATTCATGTTCATGTTGAGGTTGCCTGGCCCCATGGGTGGGT
CCACCTCCCTCAGCCCAGACTGCCCCATGGGAGGGCTCAGGAGGCCCCGGCCTCCACCAAACTCCATGCCCATGGGAGTG
CCCGCCAGGCCCTCACCACCAGCCATCTGCCCGGGAAACATGGCAGGATCCATCTGTCGGTGCGCCTGCATCATCCGCTC
CATCTCCATGCTCTGTCCCATGCCACTGCCTGCCATGCCCAGGGGGCGCTGCATGCCCATCGACCGCTTCTCCAGCAGCT
GGTGCCGCAGCAGCTCCTCACGGACCCGGGGAGTCATGAATCGCTCCGCCTCACCCTGCCCACCTGGGTAGGGCATGGTG
TTCTGGGCAAAATTGCTGGGTCCCCCCATAGGGGGCAAGTCTTCGGTCCAGCCCATGCCTGGTCTCACGGGCCTCTGCAT
GGCATTCATGGGCACCTCCATGGGCATACTCTGCATGCCCCCAAACCCAGGTACCCGTTGTATCTGGTTGCCTGGGAAAC
GGGGCCCAGGAAAGGGAGGTCCGCCCCGGAGCTGCATGGGATCTTGAACATCCATGGGCCCCCGCAGCTGCGCACCCATT
CCAGGTGGCCACTGATCCCCAGGCTTGCTGTGGTAAGGAGGCGGGGGCCCCCTCACCATCATGCCCCCCATGCCCATCAT
GTCCTGCAGAGGACGGCTCCCATGCAGCCCAATCTGTTCCTCTTTCCGCCGTTTCTCTTCGTAGTACTCCTCCTGCAGCT
TGCGCCAGGCCACCTGCTCAGGCGTGAGGCTGTCCTGGCCCAGCCTCTGTATCATCATGTTCATCTGCTGCCCCATGTCC
CCACCCGGGGGGTGCCCAGGCACTTCATGCTCCAGCGGGGGGCCCCCTAGGCTCTGTGTCTGTGAAATCATGGACTGCAA
GGGTTCCTCATATTTCTTCAGCCCGCTGGGAGGGGCCGTGGGTGGCTGCTGGGGGGGAGGGGGGGCTTGTGCTGGTGGGC
CCCCCTCACCCGCTCCTCCTGGGGGCCCCTTGAGGAAGGGCTCAGTCTCTCCGCTGCGGAGCAGCAGTCGCTCAATGTCT
CGCAGCGTCTGGAGGGACCGTTCCCGATGCTCCAGCTGCTCTTTGGACAAGCCCTCTGAGCCCACCAGGCTGCGCTGCCC
ATTTCCAGGGGCGGCTGCCTCCCCCAGCAGGGCAGGGCCGGGGGGCACTGCTGGGGTCTCCTCCAGGAGGCAGAGGGTTGT
TGGCGGTGGTAGCCGTCGGGGTGTTAGGGTGGGTGCCCCCAGTCCCACCACCTGTGCTGGCAGCTCCCACTGAGTTGGGG
GCCAGGTCCTGACTGCTGTCCTCAGGAGGCCCCTCTGGGGGCAGAGCAGGCGGGGCACTGCCAGGGGCCGGGGGTGGCGG
CGGCGGCAGTGGAGGTGGCTGGGACTGCGGGGTGCCTGCTGACGGCGTGCTCAGGGGTAGCGGTTCTGGGGTGGGGGGCA
CTTTAGGGGCCTGCAGAAGGACAAAGAGAGCATGAGACAGGTAAGGGGACGTTCCACCCAGTCCTGCTGCTCACAGAGGC
GCCACGCTCACCTGGTCAAGCTTGGCCCGGGGCACGTTCTGCTGGTGGTAGGCGAGGATGGAGTCGGCCCGGCCCTGCAG
CACTGCCTCTGCAGCCCTGCACAGAGTGGGGGCACCGACAGCTCAGAGAGGTGAGCAGGAGGGAGCCCCACCCTCACCCA
CCCCACCCTGCCCCTGCACGGGGCTCCCTCGGAACCCCAGGCTGAGAGGTGCTGGGCAGAGAGAGAGAGAGACTTGGCCT
GCCCACCAGGCACTTACGTGTTGGCCAGGTGGGTGGTGAAGACATATACGAACTGCGAGGGAGGCTTTCCGGGGACGCCC
CCGCCCCCGCCCCCGCCCCCAGGGCATCAGGCCGAAGGCCAGGAGGAGGGCCGTGCGGGGCGCCTGGCACGCTGCTCTC
GCTGAGGGGCAGTTGGGTGGTTTGGCCGGGACCCAGCTGTGGGGCCGCCATGGCTGGGTCTGCTACATTACAATCTGCAG
GAGAGAGGACAGGGAAAGGGGCTAAGTGGTCTAGATACAAGAAGGACCAGCTGATGCCCCCTCCCACTGATGCTCACAGC
CTTACAGCTCGTGCCCACAGGGACATTTGATGTCAGTATCTGGTGTTCTCACCAGGCTGGTTTCCACGATGGCAAGAGTG
GTGACCATGTGGAGAGCAGGGACAGAGAAAATGTGTCCTCTCCACAGGCTCCCATGGGCCTGGCATTCTGCTGGGGACTT
AACAGGTGTGATTTCATCTCATCAACACACCCTGGGAGGAACGTGTCACACACTCCACTTAACAAAGGAGGAAATAAGTT
CCTCACCCACAGTTACACAGCCAGTTAGTGCAGAGCCAGAACTGAACCCAGGGCCGGGACTCTGAAGCTCCGCTCTGAGC
TGCTGCCCCTCTCACTGGGTAAGTGTATGTGCACACAGTCTAGTGACCCAGTTAGGGAGAAGGCAGAGAGGGTACACAAG
CTGATGATCCAAATGATGGATGGTTTTTCACTCTAGCCCCACCACTAACTGACTTTGTGATTTTATGAACCAGACATCCC
TCTAAGCCTCGGTTTCTTCATCTATAAAATGTAAAATACTAACCTCCTCCTTGCACAGGGGGCAGGCCAAAGAAATATAT
TTTTTAACAGTAAGTTTAAATCAATGTCACAGACTAAAAAAATCCATTTGAAAAACAGAGAGCCGGGAGCGGTGTCTCAC
GCCTGTAATCCCAGCAGTTTGGGCGGTAGAGGCGGGCGGATCACCTGAGGTCATGAGTTCAAGACCAGCCTGGCCAACAT
GGTGAAACCCCGTCTCTACTAAAAATACAAAAATTAACTGGGCATGGTGGCGCATGGCTGTAATCCCGCTACTCAGGAGG
CTGAGACATGAGAATTGCTTGAACTTAGGAGGTGGAAGTTGCAGTGAGCCGAGATCACACCATTGCACTCTAGCCAGGGC
GACAGAGCGAGACTGTCTCAAAAAAATAAATAAATAAATAAAAATAAGAAAGAAAAACAGTTAGAAAAAGCTGACATAC
TGGAAACACTCTCAAGACAGACCAGCTGCCCTCAGCACCAAGGGAAGTTTTGTTCCATGGTTAGACACTTGACTGTAAGT
CTTAGCGTAGAAAAAATCTACAACGGGTCTAGGTGAGTGGGTAGACCCATTCGCATGCCAAGAAAAGCAAGTCACAGTT
CCTGGCCCGCAGACAGTAAGTACCAGCATTCTCAGTTAATGGGAGACAGATTGCCTGAAGTTGTCTCCATCCAGCCAGGG
CAGTGACCACCTTCCCATGGGCCCAGTGTCAGGCTGCCCTCTTTGGAAAAAGTTTCTTCTGCACCTTCTTCTCTGATGGA
CCCACCCCAAAACGGCCCTGCTCTGGACCCTATCTATCCAATTTTACATAGAAGAGCAGAGCCAGAAATTTGCGGGGGAG
CTTGAGAGCTGACCTCAATGTGTGAAGGCTGCTGAGTAGCTCTTGGATACGAGCTATGGGGAGACAGGCAGGGCTTAGTC
CAACAAGGAACTTTCTAGTACAATAACAATGATAAAAGCTAACATGTTACATGTATAACCTCATTTACTCCTCATACCAA
CCCTATGAGATAAATAATACTTTTACAGATGAGGAAACTGAGGCCCTTTCCCAAATTTACAGAAATAACCTGGGTTGCAA
ACGAAGGCAGCCTGATGGCAGAACTAGTCCTGTCAATGTCTACGTTCTATCGCCCCTCCAGTAAGAACTCTTCAAGAATC
TAAGGGCAGGCCGGGCACGGTGGCTTATGCCTGTAATCCTAACACTTTGGGAGGCCGAGGCGGGCGGATCACTTGAGGGC
```

AGGAGTTCAAGACCAGCCTGGCCATAATGGTGAAACTCCATCTCTACTAAAAATACAAAAATTAGCCAGGCGTGGTGACG
CATGCCTGTAATCCCAGCTACTTGGGAGGCTGAAGCAGGAGAATTACTTGAACCCAGGAGGTGGAGGTTGCAGTGAGCCG
AGATTGTGCCACTGCACTCCAGCCTGGGCAACAGAGTGAGTGAGACTCTGTCTCAAAAAAAAAAAAAAAAAAAAAGAATC
AGGCCGGGTGGCTCACGCCTGTAATCCCAGCACTTTGGGGGGCTGAGGCAGGTGTATCACCTGAGGTCAGGAGTTCAGGA
CCGGTCTGGCCAACATGCTAAAACTCCACCTCTGCTAAAAATACAAAAATTAGCCAGGCGTGGTGGCAGGTGCCTGTAAT
CCCAGCTACTCGGGAGGCTGAGGCAGGAAAATCGCTTGAACTCAGGAGGCAGAGGTTGCAGTAAGCCGAGTTCGTGCCAT
TGCACTCCAGCCTGGGCAACAAGAGTGAAACTCCGATTCAAAAATAAATAAATAAATAAATAAATAAAAGAATCTAAGGG
TAGTACGACTAAAATAGTCGCCTTGAGTATGGGATGTTAGCAATTTCTACTTTGTATTTCCCTATGTTTGCCAGGCACAG
TGGCTCACTCCTGTAATTCCAGCACTTTGGGAGGCCAAGGCATGAGGATCGCTTGAGACCAGGAGTTCAAGATCAGCCTG
GGCAACATGGCAAGACTGTCTCTACAAAAATTAGCCAGGTGTGATACATGCCTGAGCCCCAGCTACTTGGGAGTCTGAGA
TGGGAGGATCACTTGAGCTCAGGAGTTTGAGGTTGCAGTGAGCTGTGATCACACCACTGCACTCCAGCCTGGGTGTCAGA
GAGAGACCCTGTCTCTAAACAATAAATAAATAAAACTCACAAAGTTTCTACAATGGTGCAGTCGATGTGACATAAGTGA
ACTG

HUMAN mRNA SEQUENCE : hR3-027.1 (Seq ID No: 17)
GCTGCCACCTCTCTAGAGGCACCTGGCGGGGAGCCTCTCAACATAAGACAGTGACCAGTCTGGTGACTCACAGCCGGCAC
AGCCATGAACTACCCGCTAACGCTGGAAATGGACCTCGAGAACCTGGAGGACCTGTTCTGGGAACTGGACAGATTGGACA
ACTATAACGACACCTCCCTGGTGGAAAATCATCTCTGCCCTGCCACAGAGGGGCCCCTCATGGCCTCCTTCAAGGCCGTG
TTCGTGCCCGTGGCCTACAGCCTCATCTTCCTCCTGGGCGTGATCGGCAACGTCCTGGTGCTGGTGATCCTGGAGCGGCA
CCGGCAGACACGCAGTTCCACGGAGACCTTCCTGTTCCACCTGGCCGTGGCCGACCTCCTGCTGGTCTTCATCTTGCCCT
TTGCCGTGGCCGAGGGCTCTGTGGGCTGGGTCCTGGGGACCTTCCTCTGCAAAACTGTGATTGCCCTGCACAAAGTCAAC
TTCTACTGCAGCAGCCTGCTCCTGGCCTGCATCGCCGTGGACCGCTACCTGGCCATTGTCCACGCCGTCCATGCCTACCG
CCACCGCCGCCTCCTCTCCATCCACATCACCTGTGGGACCATCTGGCTGGTGGGCTTCCTCCTTGCCTTGCCAGAGATTC
TCTTCGCCAAAGTCAGCCAAGGCCATCACAACAACTCCCTGCCACGTTGCACCTTCTCCCAAGAGAACCAAGCAGAAACG
CATGCCTGGTTCACCTCCCGATTCCTCTACCATGTGGCGGGATTCCTGCTGCCCATGCTGGTGATGGGCTGGTGCTACGT
GGGGGGTAGTGCACAGGTTGCGCCAGGCCCAGCGGCGCCCTCAGCGGCAGAAGGCAGTCAGGGTGGCCATCCTGGTGACAA
GCATCTTCTTCCTCTGCTGGTCACCCTACCACATCGTCATCTTCCTGGACACCCTGGCGAGGCTGAAGGCCGTGGACAAT
ACCTGCAAGCTGAATGGCTCTCTCCCCGTGGCCATCACCATGTGTGAGTTCCTGGGCCTGGCCCACTGCTGCCTCAACCC
CATGCTCTACACTTTCGCCGGCGTGAAGTTCCGCAGTGACCTGTCGCGGCTCCTGACGAAGCTGGGCTGTACCGGCCCTG
CCTCCCTGTGCCAGCTCTTCCCTAGCTGGCGCAGGAGCAGTCTCTCTGAGTCAGAGAATGCCACCTCTCTCACCACGTTC
TAGGTCCCAGTGTCCCCTTTTATTGCTGCTTTTCCTTGGGGCAGGCAGTGATGCTGGATGCTCCTTCCAACAGGAGCTGG
GATCCTAAGGGCTCACCGTGGCTAAGAGTGTCCTAGGAGTATCCTCATTTGGGGTAGCTAGAGGAACCAACCCCCATTTC
TAGAACATCCCTGCCAGCTCTTCTGCCGGCCCTGGGGCTAGGCTGGAGCCCAGGGAGCGGAAAGCAGCTCAAAGGCACAG
TGAAGGCTGTCCTTACCCATCTGCACCCCCCTGGGCTGAGAGAACCTCACGCACCTCCCATCCTAATCATCCAATGCTCA
AGAAACAACTTCTACTTCTGCCCTTGCCAACGGAGAGCGCCTGCCCCTCCCAGAACACACTCCATCAGCTTAGGGGCTGC
TGACCTCCACAGCTTCCCCTCTCTCCTCCTGCCCACCTGTCAAACAAAGCCAGAAGCTGAGCACCAGGGGATGAGTGGAG
GTTAAGGCTGAGGAAAGCCCAGCTGGCAGCAGAGTGTGGCCTTCGGACAACTCAGTCCCTAAAAACACAGACATTCTGCC
AGGCCCCCAAGCCTGCAGTCATCTTGACCAAGCAGGAAGCTCAGACTGGTTGAGTTCAGGTAGCTGCCCCTGGCTCTGAC
CGAAACAGCGCTGGGTCCACCCCATGTCACCGGATCCTGGGTGGTCTGCAGGCAGGGCTGACTCTAGGTGCCCTTGGAGG
CCAGCCAGTGACCTGAGGAAGCGTGAAGGCCGAGAAGCAAGAAAGAAACCCGACAGAGGGAAGAAAAGAGCTTTCTTCCC
GAACCCCAAGGAGGGAGATGGATCAATCAAACCCGGCGGTCCCCTCCGCCAGGCGAGATGGGGTGGGGTGGAGAACTCCT
AGGGTGGCTGGGTCCAGGGGATGGGAGGTTGTGGGCATTGATGGGGAAGGAGGCTGGCTTGTCCCCTCCTCACTCCCTTC
CCATAAGCTATAGACCCGAGGAAACTCAGAGTCGGAACGGAGAAAGGTGGACTGGAAGGGGCCCGTGGGAGTCATCTCAA
CCATCCCCTCCGTGGCATCACCTTAGGCAGGGAAGTGTAAGAAACACACTGAGGCAGGGAAGTCCCCAGGCCCCAGGAAG
CCGTGCCCTGCCCCCGTGAGGATGTCACTCAGATGGAACCGCAGGAAGCTGCTCCGTGCTTGTTTGCTCACCTGGGGTGT
GGGAGGCCCGTCCGGCAGTTCTGGGTGCTCCCTACCACCTCCCCAGCCTTTGATCAGGTGGGGAGTCAGGGACCCCTGCC
CTTGTCCCACTCAAGCCAAGCAGCCAAGCTCCTTGGGAGGCCCCACTGGGGAAATAACAGCTGTGGCTCACGTGAGAGTG
TCTTCACGGCAGGACAACGAGGAAGCCCTAAGACGTCCCTTTTTTCTCTGAGTATCTCCTCGCAAGCTGGGTAATCGATG
GGGGAGTCTGAAGCAGATGCAAAGAGGCAAGAGGCTGGATTTTGAATTTTCTTTTTAATAAAAAGGCACCTATAAAACAG
GTCAATACAGTACAGGCAGCACAGAGACCCCCGGAACAAGCCTAAAAATTGTTTCAAAATAAAAACCAAGAAGATGTCTT
CA

HUMAN PROTEIN SEQUENCE : hP3-027.1 (Seq ID No: 18)
MNYPLTLEMDLENLEDLFWELDRLDNYNDTSLVENHLCPATEGPLMASFKAVFVPVAYSLIFLLGVIGNVLVLVILERHR
QTRSSTETFLFHLAVADLLLVFILPFAVAEGSVGWVLGTFLCKTVIALHKVNFYCSSLLLACIAVDRYLAIVHAVHAYRH
RRLLSIHITCGTIWLVGFLLALPEILFAKVSQGHHNNSLPRCTFSQENQAETHAWFTSRFLYHVAGFLLPMLVMGWCYVG
VVHRLRQAQRRPQRQKAVRVAILVTSIFFLCWSPYHIVIFLDTLARLKAVDNTCKLNGSLPVAITMCEFLGLAHCCLNPM
LYTFAGVKFRSDLSRLLTKLGCTGPASLCQLFPSWRRSSLSESENATSLTTF*

S DISCOVERY 3-027

Table 4

MOUSE GENOMIC SEQUENCE : mD3-045 (Seq ID No: 19)
CTCAACCCCCACCCCATGTATCCCTTAAGGCCATGCCCATGAGACTCAGTTCCTCTAATGACGTTTCACCTCCTGAAGAC
TCTATACCCTCTCAGAACAGTGTCCCCAGGAGGAGGTTGGCGGACACCAGCTGGATCAGCACCCATCCCTCTCTGTCTCC
TGTCTGAGTACCCTGTGAGCAACAGCCTCAACTTCCAGACGCTGTGCCTTTCCCTCCACAGTGGACGGTGTCCCCGAGCT
GTGAGGCAGAACAAACCCTTTCTCCTTGAGTCGGGTGTTCTGTCCCAGCATCAGGAAGAGCAAGTGGTACAACTAGAACA
TCAGCATGACCACGGTCCTGTTTCCAGCACCAAAAATACAAATATGGCTGCCATGCTCCCCGTGGGTGGGTGGGTGGGGC
GAGAAGCCATATCTCAGGTCTCTGGGTCCTTGGGTGAGGTTGAACCCCCCATAGGAGGATAAAGTCCCCGAGTAACTTCC
CTCTTTCGCCATGAAACCCAGATCCACTCATGGGGCCTTCTTAGTACTTATTGCTTGAGGCCCCATGCCTATGACAGTGG
CCTCTACCTAAGGTTGATGAGCAGGCCGGTGCCCATTAGCCTAGCCCCAGGTGCTTACTTGACTGGCTGACTGGGGATGA
CAGCTAGCCAACTGATACCTAGTGCTGCTGGCTGTCACACCTGAGGTTCTTCTCCCCGGACAAGTAGGCATCTTGGCCCC
CATCTCCAGTGTTAGGTGACAGCTGACTGCCCAGACTTGCTAGGCAAGTCCTGGCTCACTTGTCTCAGTCCCAGCAAACT
CAGCTGCCTCTCTGGGCTGGAGAGGAATCTGGCATGACTCCCTCACTCTGAAGACAGCAGCTGCAGACTTCACAGAGCTT
CCCTTCCCCATCCTCTGCCCTTTGGCTCTGGTGCCCATTGGGCTTCTGTGCTGGGCCCTGAACAACTACAATGGAGAGGC
AGCTTCAAAGGTCTGCCCAATGTGTTGGCCATCTGTATGGCAGGGCTACCTTTAACTCCAGAATTTCAGCTTTCCGATCT
GATCTGGGGGACAGGGTAACAAAAGCATTGGGGGCAGAGAGGTAGAGGCATCTGGGAGATGGGTCCATGGGTAAAACAGC
TTGCTATGCAAGCACGAGGACCTGGGTTTAAATCCCCAGGACTTAAGCTGAGCATGGTAGAGTATCTACCATCCAGCTGT
CCCACAGTGAGATAGGAGGCAGAAGTAGAATTCCCAGAAGCTCGAGGGGCAGTGAGCTTAGCATTCACAGAGGCAAAACA
AGAGACCACCCCATTCTACCCCAAGCCGTCTCATATTAAGTGGAAGACAAAGACCTCTGATCTCCATATACGCAAGGCTG
ACTTCCTGGCTATCTGAATAGCCCAACCTACAACAGTTGATGTTTTGACCTCAGGTCCAGAGCTCACCCAGCCTTATTCT
CAGCTGAGCTCTTCCTCTGTCACCCCTACTCCGACCCAGGCATGATTTAGCAGCCATAGATTTGGAAAGACTGGAGCCAG
ATAGAAGTATACATCACTCCAGAGCCTGGAGAGCTCTGCCTTTCACTCAGAAAGTCATCACAGGCCCACCAAACCTGCCA
ATGAGGCCTAAGAGTCTCTCTCCTGTGCTGTTTGGATTTTTGTCAATTAGATACAAGCTAGGGTCATCTAGGAAGAGTGA
CACTTCCTTGAAAAGATGCCCCCATCACATTGGACCATCGTCTAGGGAGCGTTTTCTTCATGATTGATGTGGGAGGGCCC
AGCCCTTTGTAGGCAGTGTTATCCCTAGCCAGATCTGGGCGAGGTGGCACATACCTTTGGTCTCAGCACTCGGGGAGGTA
GAGGCAGGCAGATCTCCATGAGTTCGAGGCCAGCCTGATCTGCAAAGGCTTCCTGGACAGCCAGGGCTACATAGAGAAAC
CCTGTCTGGGTGGGAGATGCAGGGAGCAAATTGAGCAGGGCGTGGGGTTCAAGCTAGCAAGCATTATTTCTTCACGGTCT
CTGTTTCAATCCCTGCCTTCAGGTTCCTGCCTTCCCTTCATGACAGGCTGTAAGATGAAAGAAACCTGTTTTCTTCCCCA
GGTTGCTTTTGGTCGCCGTGCTTATCCCAGCCACGGATGACAAAGTAAGCCAGAATCCGCAAACGGCCTCTGCTCTGCGG
CCTGTTGGGATTCACTCTTTCCTTGATTCGTTTCCAGTCTGATTTCTTTGACCACCCAGACACTGCTGGGCTCTAGACCA
GTCGTTCTCAACCTGTGGGTCGTGACACCTCCAGGGGGTCCTGTATCAGCTATCCTGCCCATCAGATGTTTCCATTATCA
TTCAAAACAGTAGCCAAGTCACAACTGTTTTAAAGGGCCACAGCATTCGGAAGGTCGAGAACCGCTGCTCTAGACGGAGC
CCTGACCAGCAGGCTGACTTTTTTTCCCAAGTCCGTGTGGTTCTCTCTCACATGATGCTCTCTTTGGCTCTGATGCTGTT
CCTAACTGTCCTGCCTAGGATGCAGCTACCTCCATCCATCTCCTTTAGCCCCTTGGATCTTTCCACTTTCCCCACTCTCC
CAGCATGGAAGATCCACTCGGCTGTTCTCCATGCTGACATGCCTAGGCCCACCCTCTACCTCCTTCTGGAGGCCGTAGTG
TAAGACTACAGAGCTTTCTTTCCTCAAGCATCTTCCCGGGATCCTTCCTGCCCGCCTGCACCCCCCCCCCCAATCCCTGA
GAAGAACAGAGAATGACCCATCAGCAAGCCAATGGGTTACCCCGAGAATGTGACAAAGGGTGAGGCTGTGGGTCCCAAGG
GTCTGCAGATAGAGGGACGAGCCAGCAGCCTTGGGTGCAAGACCAGGCCTTGGAGGGTATCTCTACCTTACCTTATACCA
CAGAGAGAAGTCTGGCTCCCATGTTGCCCTGTGGATGAAGGTGGGCAGGCTGAGCACAAGCTTTCAGGAACCATTGAATT
CATTGTGAGGCTGAGATGGATCTGCGTCCATAAGGCCAGGAGGGGTAACAGTGTCAGACCCCAGCCCCAGAGGGCGCTCT
GCGGTTGATTCAGTAGCCAGGAGCATCCAGAGAGCTCTTCTGGGGCCCTTGCTTTGTCTCCCACTCTGCAATCCAGGGGA
GATCTGCAGCCCCTTTTCCTTGGCAACACTCAAGACAGAAAGAGGAAGCTACATGCCCAGCCACCCACGGGGAAGGAGAT
TGGTTCCACTCCTGCATGAGGTTGTCGACGAAGTCATTCTGCCTTCTAGCAGGACAATGGAGAATTGAAGAAAGAGGGCC
GAAGAAATGGCTCAGCCGTTAAGAACACTTGCTGCTCTTGCAGAGGACACAGGTTCAGTATCCCAGGACCCAGATGGTGG
CCCACAACCGCCTATAACTCCAGTTCCAGGAGATCTGACCACCTTCTTCTGGCCTCCTCGGCCACCAAGTAGGCATGTGT
ACAGGTACAGTTACAGGCAAACACTCACACACTCAACTAGCCAGGGAGTCTGCTGGCTAGTAAATCCCAAGTGGCCAAGT
GGCCTGCTTCTAAATCCTTCCTTGTAGCTGTCTGATATTCACTCCATCCCAAGTCTTAAAAAATAAATAAATAAAATTGA
CGTTATTACCTTCATTTTAATGCAGAGGGAACAGAAATCAGATCAGAGAGGTTAAGTAACCAGCCTGAGATGCCACAGCT
GAGGTCATAAATAAACATCTCTCCCCTTCAATCTCTGACTTTGAACAGCCCTCTTAAGAGACTGTAGAACACAGATGCTT
CTCCGTCCAGGAGGAACGATGACAGCTCCACAGGGTCTGTTCCTGGAAACCCCAGCTGGCTGCTGACATTTCCTGGGTGC
ATGCTGTTCCGAGGCAGATGTCACCCAGGACCCCTGAACCAGATGGGGCTTCTCAGATCACAGCTATAACGTGACTCTCG
GGACTCAGTTTCCCATCTGTGAAATGAAGAGGCTCCTTCCTCATCTTGCTTCAGCAGTTCCTGTACAATCAAGTATCTTA
GTTTGTCCCAGGAGCTCAGTAAGTAGCCGGATGTACCAAAGGCTCGCTGTCCAAGTGACTCACGTTGGCTTTTAAATGAT
GGATTTCTGGGATTCTTGTCTTACTATTAGTTTGCTGTGACAGACTAACCAACAAGAAGCAACTATGTAAGGGCTTATTT
CGGCTCACGGTTGAAGGGCACAGCCCTTCATGGTGGGGAGCCACAGTAACCCGTTTGTGAGGTAGCTGCTCACGTTGCAT
CTTGCTTCAGGGCAGGGAGCAGAGAGAGAGATGAATGCTGGTGTTTGGCTCACTTTCTCCTTTTTTCTTCAGTCCTATGG
ATGGTGCTGCTCACAGCTAGGGTGAGTCTTCCAACTTCAATTAACCTAATGTGAAAACTCCCGCACAACAGCAGCGGCCT
GAGGAGCGTCTCCTACATGGTCCTGGGGCCTAGAGAGTTAACAATAACCATCAACCAGGATGATGCCCGGGTTATATTTA
CTGTGCTTCACAAACTGTCATCTTGGAAACAGCCTTGTGCCTGGCCTTACTAGATTGGATCTTATTTTTCAGGTAAGAAA

```
ACCAAAGCCCAGAATCCGGAATAGCTCAGCCCTAGTCAGGTGACTCTAGACCAGCGGTTCTCAACCTTGCTAATGCTGCA
ACCCTGTAAATACATGCTGTGGTGACCTTCGCCCCATAAAATTATTTTGTTGCTACTTCATAACTGTAATTTTGTTACTG
TTATGAATTGTAACATCAATATCTTCTATGTGGGATAGCTGATACTCAACCTCTAGGAAAGGACCCTTGGGCCTTAAGGG
GGGGGGGGGGTCTCAATCCACAAGTTGAGAACCAATGCTCTAGAAAGTGGTAGAGGGATTTTCAGACCCAGTGATGGTAGG
TAGAGCTTAAGGTTGCCCCAGCTTTGTGTCCAAGTTCCCCCCCCCCCTCCCCGTTTACTGTGTCAGGGAGGATGACGGA
CCAGTTTCCAGACTAGGAAAACCCAGGTACTGAAGAAATGAATAAAGCTGAAAGAAGCAGCGGTAGCTGGGGTGGTGGAC
AAAGAAAGGCACTGGCCTAGCAGCTGAGGGGCAGAGGCCTCAGTCGCATGAGCCCATGGAATAACTGTAGAAGTGGCTGT
TAATTTCAGCAAACTGTCTTCCGGTGTCCTCTGGGACACTAATGCAAAACAGAATCACGTTTGCCTGCTTCATTTTGTGA
GGTAATTCTGTAAATTTTATTTACTTGTTTGTTTATTTATTTATTTATTCGTTTATTTTATTTTTTAAATTAAACTCTA
TATACTAGGGTTTAAATGCTGCTCACATGGGGGGTTCCTGAAAGCCCTGGAGGGGGCACACGTGGGTCTGCCCGTGTTTT
AAACAGAGGTATCTCCTGCAATCCGAAGTCACTTAGCCTGGTGCACTTTACACCTTTTTAAAGATTATTCCCCACTGCCC
TCCCCATCCCCACCCCGACACATTTGTGTGGAGGTAGCAGAGACGGAAAAAGCTGTGCTGTGTCTTTAAACAGGGAGGAG
CGATCAGCAGAAGCCTGGGCTGTAAAAAGGCTAATGCGCTGTGGAAGAGTAGGAGTTTAATCTAATTTAGTTGGAGGGTG
TCTGTCTGCGGTGTTGTTTTTGGAGTGTGGGAACAGAGGAGGGGAGCTGCCCCCTTGAATGGTCCCTTCTGCGCCAGAA
GCAGTGGGGAGCCGCGCGACGCCAGCGATCCAAGGCCCGGCCTTGAAAGAGGATTAAAGCGATGCTTGATTTGCAATTCA
CACGAAAGCGCCCCGCGAACCTCTGGTAATATTTCAATCCACATTTCAAGGGAAGTGGCCTTAAAATAGCGGCCTAAATT
GTTCTGCCGCGGCCCGGACACTCCTTCATGCATTTTATAACGCAGAGGGCCAAGGGCCTTTTCTCTCTGCGCCTCACCAC
CCCCAACCACCCCGTATCTTACATGTGCTGAAGTGTAATTAAAACTTGTTCTTAAGTTTGGGGCAACCTACACTGCTTTG
TGGTATGAGGTGTGTGTGTTTTATGCTTGGAGCATATGTTTGTGTGTGTTAACTAAACATCACTTTGGATGACTTTAATA
TATCTCCTCCGAAAGGGGGGGGGGGGATTGTGAGGCAGTCGTGGAACCAAGCCTAGTGGGTTGGAAAACTGAATTCTCAAT
GTGTGCCTTTTAAAGAGACCATTACCAGTAAGCAGACCACTTTCCTTACTGTAAGGAACAGTTTGGAAAATGCAAACAGA
GATCTTAAGAGCTTTCCAACTGGATCCGCAAATAGGAAAGTTCTAGAAATGGCCTTGAAGCTACCAAGACAAACGTTAAG
GAGCCAGCTCCCTGGTTCATCTTCAGGCTGGGAGACAGGATGTTCATGTCTTTCTATAATCTCTGCCCATACCCCACATG
TTTACTCTTGCAATTAGCTTGTAACAGGCTAAGCTTCTGGCTTAATTTTTCTTTATTGTAGGAGACGAAGTACTTTTTTT
TTTTAATCTTAAAGAGATTATTTGGGTTCTCTCTAATCCTATTTCAGAAACCCCTCTCAAGACTTTATGGTTCCTTCACC
CATACTGAGGAAGGCTGCAGCTTTGATTACCACTGAGACCCATGGCAGAGAATCTTTTCTTTTTTTTTTTTAACTTCTGA
CTTTACCAATTAAACAGGGCCTTCCTTTCAACTCCCCACCACCTCCAAAATTATTTAACTCAAGACACCATCTGACTCTT
AAAGAGTATTCCTTCCAGAATACCTTGCACTCTCCTTGGTAGACATTTGCAAGAACTCTACGCTAATATCTACAGACAGT
ATTTTGAAATCTCACGTAGATAGATATATTAGGAGATTTGGGGTCCAAAGTACCCTTTGCTCTAGGATGCTATTTAAAAT
CATGTTTAATTATCAAGTTTCATTAAAAACCCTTGCTCAAATTTTGAGAAACCTAATAAGATTTCCATTAATTTAAACAT
AGCTCTATTCCCAGGCCAGTCCCCCCCCACCCCACCCCAAAGCTTTCTCTTCCCTCTTTTATCCCTGGTTAAATATGTAAA
TAAAATGTCTAAAGTCGTTCTTCAAAGAAGACAAAAGAAAAAAAAGAGCCGAGGGGAGAGAGACAGCCATAAAATTCCTC
CCAGCTCTCCATAACGCTTGCCAGAGGGGGGGCCGGTTTCCGAGCCAGGGCTGAGCAGCAGGGGACCCGGCTGCTCTGGAG
TCACTCGTCTCAAATCCCACACACAGGAGAGAGAAAAATAAAATATCTTTTCAGCTGGAGCCATCAAAGGGGTCCTTTCA
TTTATTAGCGTTGCCTTCCACTCTCGCTATCTCGGGGCCCTTATTTTTAGATACTAATGTCTATTTGTTGCTTTCCTTTC
TTTTTCTATACTAGGTTGGGCAGATGGAAGAAATTCTGTTAGCTTTTTCTTCTCTTAATGTATTTTAAGGGAGTCCCGGG
GGGTTGGGGGAGAGAAGAAAGGAACGTGAAGTGATCATAGCTAAAATAAAAGGTCGGAACACTGTAGAAGTAACCAGAGC
ATAAAAGACCAATTACGTGGGGCAGTCTCAAATAGCTCACACCCTGCTCCGGAGCTGAGCTCACCCAGGCTACTTCCACG
CCCCCCACGCCCCAAGGGGCGGGGGGGGGGGGAGTGTGGAATTAGGAGAGAGAGAGAGAGAGAGAAAACAGAAGGCAAAAG
TGTCAGGAAGGACCCCTTGCAAACTAGTCTGTCTTAAAGCTAAGCGATAGCCCAAGAATCTTACCTGACTTAAAAGGTTT
TGGAAACAGCGTACTAGACTATAAATATCTAAGTAAACCATCCTTATCCAAGCCGCAAGCGTTCACCACACCGCAGGTTT
TTCTTGGGTCACTAACACGGTGGTGGGTGGTGATTCTGACTGTGTTAACCTCAGTCTTCCGGACCGCTGTTTCCACGGAT
CCAACTTCCTCCCGTGAAAGGCAGAGGGGACCAGGGGATCTAAAGGTGAGGCATGAGCCTGTGCACACCCTCCGCCCCC
CACCCCCAGGACTCAGCAGGTCCTGGGAGTGTCCCAGCCTCAGGTCTGTCCGCTATGTCTGACAAATGAAAGGGGGGTAA
AGAGGAAGAACTCCCTGCAGATGGGACGCTGAAGCCTTGTTTTAATTAGAAGTCGATCCTCTGCAGGAAGCGCAAGGCAG
GGCGGGGAGGAGGGGTCCTGGGTAGCCTCGTTCCTGGCTTGGCTGGAGCCACTGAAGGCTTCCAGGCATCTGTGCACCCA
ACTCGGCCCTGGGGTGCGCAAGACTGACTGGCCGAAAGATGAGCAGGGACTTAGCTGCACCTATGCAGTTGGGGGAGTC
CGCACTGGAGGTCCTGGTAGTTTCCCAGACACTTAAAACGCAGACCTGAATGGGGGTGGGGTGGGGTCTAAGGAGAGAGT
GGGCCACCGCCCTGAGGGCTGTTTATCCAAAGCCAAGAGTCCGCGACAGGTGCCGCACCCCAGATCTCTCGGACATCTAA
GGACTGCGGTGGGCTCCCATGGTGCACTCACTCCCTGTGCGGGGGAAGGAGGTTGAAGCTCTGTGTCCTTATCACAGATT
AGCCCAAAACCAACCAAGGGCAGGAGCCAGCATAGCAAGGCACGGAGAGGCCCCACCACTCTGCGTGGAACCAGCCGT
GCGCGTGTAGGCCGGGGCTCAGCCTCCGCCTCCCAGGAGGACGCAGGCCGAGCACATCTTTCGCCAAATAATATAAATAC
ACAACTTCCTATAATAAATAAACTCAGCCGCTTGGAGAAACCACCAGCCGGCTTGGCGCGATGGTCCCCAACACGGTGGC
CTTGGCGGGGCAAGGGTCACCACGATCCAAGCCAAGCCCCTCTTCTCGGGGTGCGGGGTGTCAGCGGCCCGGAAAGTCTC
CTCCCCGCCCCCTCCCTTCTTTCTTTGAGTAGACCTTGCAGAAGAGGCCCTAAACTCCGGCTTCCCAAGTCCCCTCCCCC
TCCCCTGCACTCGGCCCTTTTGAAATTTCAAAGGCAGCCCCTCCCCGGAGTCCGAGCTCCGCGCCCCGGGCAGCAAACCC
CCGGCTTCGGGAAGATGCGGGCAGCTTTACAAGCCGAGGGGCCACTTTTTTATGAGCACGGTCATAAAACGCAAATCCCG
TGTCTCTTTGAAAGTGCCCTCCGCCAGGGCCCGGTCGTAAAAGTAGAAGATGCGTCCCCAGCCCCCAACCCCGGGTGAGG
ACCACCCTTACCCGAGTCCCCAAACTTCCCGTAACCTCGGCGTCCTTGGGGTACCGGGGACAATCTCAAGGCCTCCCACC
CCCCCTCTGGGGCGGGGAGGGCAAGCAGAGAGGCCGGCAGGAGGTGAGGCTGGAGGCTGGGGTCCAGCCCCAAGCTGGGG
CTGAGGGCGGAGGCTGCCCGGGGCGCAAGNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNGCGGAGGCTGCCCGGGGCGCAAGGCTTTCTGATGCAAAGCAGCA
GCGGGGCGGGGCGGCGGGCGGAGGCGGGGCCTGTCACCAATCGGCTTTGCTTTTTTTTTTTTTTTTCCCTCTTTGCTAC
CCTTTGCCTCTTTGCAAGTCTTCTCATCCCGGGACGCCGTGCTGGAAGCAGCTGCGGGCGCCGGGAGGGAGGAGCTCCAG
GGCCTAGGGCCACGGTGTGCCCGCGCTGCCCCACCGCGGTCCGCGATCGCCCGCGCCCGGAAGAAGAGGCCAAAGTTTGC
GCGCTGAGCGGCGCTCGCTCCGCCGTATTGTTTGCAAACTTCGCTCCCCTCCCCCGCCGCCCCCGACTCCGCTGAGGCC
GCGCGCCCACCGCGGTGGCCCCGGGCGCCGCGAGCGCTCCGGCCAGACTGGATGGGCATGCGCGGGGGCCCGAGCCGGGG
CGCGGAGCCGCGGGCAGCAGCAACAGCCCGTGCCCGGGAGCGGCGGCCCTGAGCGGCGCTGGGACATTGAGGGCGCCACC
ATGCAACACCCGGGCCCGCGCCTGTGGCTGGTGCTGCAGGTGATGATAGGCTCGTGCACGGCCATCAGCTCCATGGACTT
AGAGCGCCCTGGAGACGGCAAGTGCCAGCCGGTGGAGATTCCCATGTGCAAGGACATCGGCTACAACACCACCCGCATGC
CCAACCTGATGGGTCACGAGAACCAGCGCGAGGCGGCCATCCAACTGCACGAGTTCGCGCCGCTCGTGGAGTACGGCTGC
CACAGCCACCTTCGCTTCTTCCTGTGTTCGCTGTACGCGCCCATGTGCACCGAGCAGGTCTCCACACCCATCCCTGCTTG
CCGGGTCATGTGCGAGCAGGCCCGGCTCAAGTGCTCGCCGATCATGGAGCAGTTCAAATTCAGGTGGCCGGACTCCCTGG
ATTGCAGCAAGCTCCCCAACAAGAACGACCCCAACTACCTGTGCATGGAGGCACCCAACAACGGCTCGGATGAGCCCAGC
CGGGGGCTCTGGCATGTTTCCTCCGCTCTTCAGGCCCCAGAGGCCCCACAGCGCGCAGGAGCACCCACTAAAGGACGGGGG
TCCGGGGCGCGCAGGTTGTGACAACCCAGGCAAGTTCCACCATGTGGAGAAGAGCGAATCTTGCGCACCGCTTTGCACTC
CGGGGGTGGATGTGTATTGGAGCCGCGACGACAAGCGCTTCGCTGTGGTCTGGCTGGCCATCTGGTCCGTGCTGTGCTTC
TTCTCCAGCGCCTTCACCGTGCTCACCTTCCTCATCGACCCATCGCGCTTCAGGTACCCCGAACGTCCTATCATCTTCCT
CTCCATGTGCTACTGCGTTTATTCGGTGGGCTATATCATCCGCCTCTTCGCGGGCGCGGAGAGCATCGCTTGTGACCGGG
ACAGTGGGCAGCTGTATGTTATCCAGGAAGGACTGGAGAGCACGGGCTGTACCTTAGTCTTCTTGGTACTTTACTACTTC
GGCATGGCCAGCTCTTTATGGTGGGTGGTCCTCACCCTCACTTGGTTCCTGGCTGCTGGAAAGAAGTGGGGCCATGAGGC
CATTGAAGCCAACAGCAGCTACTTTCACCTGGCAGCTTGGGCCATCCCGGCTGTGAAGACTATCTTGATCTTGGTGATGC
GCAGGGTGGCAGGGGATGAGCTCACTGGTGTGTGTTATGTGGGCAGCATGGATGTCAATGCTCTGACCGGCTTCGTGCTG
GTCCCGCTGGCTTGCTACCTAGTCATCGGCACTTCCTTCATCCTGTCCGGCTTTGTGGCTTTATTCCACATCCGGAGGGT
GATGAAAACGGGTGGGGAGAACACGGACAAGCTGGAGAAGCTCATGGTACGCATAGGGGTCTTCTCCCTCCTCTACACTG
TGCCGGCCACCTGTGTGATTGCCTGCTATTTTTATGAACGCCTCAACATGGACTACTGGAAGATGCTGGCCACCCAGCAC
AAGTGTAAGATGAACAATCAGACCAAGACACCTGACTGCCTGATGACCACCTCCATCCCTGCCGTGGAGGTCTTCATGGT
CAAAGTGTCCATGCTGCTGGTGGTGGGCATCACCAGTGGGGTGTGGGTCTGGACTTCCAAGACCCTGCAGTCCTGGCAAC
ACGTATGCAGCCGGGGGCTAAAGAGAAAAAGCCGGAGGAAACCAGCCAGTGTGGTCACCAGTGCAGGGATCTACAAAAAA
GCCCAGCACCCCCAAAAAACCTCACCTTGGGAAGTATGAACTGCCCGCCCAGCCTTCAGCCTGCGTGTGAAGGCAGAGCTG
GCTGCAAGAACAGGACATTCGGAGCCCAAATGGGCAGCTTTTCTTGGTTGGCTGGTTGTTGGTAAATCAAAGTAAAAAGA
ATATGTATATATATACATGTATGTGTATGCATGTGCATATATATGCATATATTTACACATACATGAAGTCTACCTTGA
GGTTCAGAACAGCTGAATGTAAAGGGTTCTGTTCAGTTTGTTGCCTCCCTGAAGGGTCGCTCATTAAGGAGGGAGCCTCT
TGTGTAACTAATTTGTGGTAAAGTAGTTGATTCAACCGCCCTCCAGAAAACTTTTGTTTAGGCCTCAAGCACACAGCTGT
GTATTCAGAAGGCTTTGCTGCCTGTGCATAAACTTGAGGCTGGAGAGCTCCTTTGTAAGCTAAAGAGCTTTCCTGGGGGG
GGGGGGCAGCCAGTGAGCCACCCAAGGGCCTGCTCTCCCTCCCTCACTCCCCCAGCTAGGCTGCTGTATGGGTGGGAGGG
AAGGTGAGGNNNNNNNNNNNNNNNNNNNNNNNNNNNNNNNNNNNNNNNNCTGCTGTATGGGTGGGAGGGAAGGTGAGGAGGAGAGG
GGATAAAAGAGGTGACACCCCCCCAGCCCAAGGTCTGCCTGCAGATCCCTTAAAAGAGAGTCACTTCCCCAGCCTTCAT
AGCCATGTTTCTCCCAACACAGAGCCCACGCTACCCTCTCACCCCACTGCAGATCTAACACTGGAGAATTCAAATGGTGT
GCAATACTTTTTTTTTTTTTTTTTTTACATTTTCCCCCTCTCCCTAGCAAAACAAATGAGAGAGAGAAAAAGTATTTTGTT
GTAAATAAAAGGCAAGAAAAGAAATCATCTAACAAAAGAATTTAGAGGCTCAAGCCTCAGGGTTGGGGGTTGGGGGGGCG
CCTTCAGCCTGATACATTTTGTGGCTTTTTAATGGAAACCAAGCCAGTGTGTTCTACACACTGGGACTGATTTGTGGAGA
GGAAGGGGGAGGGGGGGAAGCCAGTGGAGAGCACCAGAGGGCTTATTGACTCTCTGAATTGTTAAACAAATGATTTCCAT
GAGTGGTCTTGAAGCACTTGGGAAGACAAACTTTGTCTCCAGCCAGGGAGGGCCATGCCTGCCTCTGTATATCTGTAATA
TAGGATATTTTTCATGCTCCACTATTTTATTAAAAATAAAAGACATTCTTTAGTTTGCTGCTAGTCACTGTGCATTGGTG
AGGGGAGCGTTCTCCATTTCTTAGCAGATTCTTGGGTGTGCGAGGCTGGCTAGACATGGACCATGAACCTGGATACAAGC
TGGCAAAATAAAACATCCTGGGGCTCAGAGGCCTGTGTGCTGGCTGTGGGAAAAGGTGGCGGTGACTCTGCCCCCAAGGC
TCCATGGATACACAGAATGCCGAGACCACACTCACCCAGGATGCAGCAGTCTTTACCTGACCTACTGTGTGCACCAGTCT
GAGTGGACCACTCTACTGCAGCTCTGGGGCCATGGGATACATTTCAGAGCTGGATAGGTGAAAGTGGGACTAAGTGTGTG
GTGGGGGGAGGGTCATCAGCAACAAATTCAGAAGCTTGGCCACCTTGAAGTTTCTGGTACCCTTTCTTCTCATCACTAAG
ACAAGCTATAGGCCCTGGAGCAGACCCAGGATCCACTAGGCTTGACTAAGCTAAAGTTCTAGCTAGAAGGACCCAAGGAC
ACAGTTCAGCTCTGAGAAATTGGGGGGGAGGGGTCAGTGTAGACCTACCCAACTCTGACCTTCATAGCTCAGAAGCACTT
TAAACTATTTGAGCCCTTCTGAAGCAGAGATACCAGCTTGATCGTGGCTTTCTGAAGCAAGTATTGGGGAAGTTTCACAG
GGCCATGGGTCCTTGAGCAGGAAGGACAGGTGAATTGGGTAGTCTGGGGAACAAGTTCTAGAAATCAATGCTGGGCAGCC
GGGCCATCGGTGAGCATAAAACCCAGAACCCAGAATCATGGAAATAAACCTCTATATTGACTATTGTTGTCCTTACCACC
AGAAGAGACACTAAGAGTGAGTGTGTGTGTGTGTGTGTGAGAGAGAGAGAGAGAGAGAGAGAGAGAGAGAGAGAGAGAGA
GAGAGAGAGAGGATCACTGGAGTGGCAACTGGCCAGACCTAGGCTCATCTGCCCCCCTGAGCTGTAGTGGTTAGTTGTAT
AGGTTTTGAAAGTTCATACAGAGCAATGCCTCAGCCAAGAACATGATAAGGCCTTGTTCAGCTGTGAGCCTGCTTGACGG
TGGCCCCATCCCCAACAGTGACAGGGATCCTGTTTACAAATATCAAGAGACTAACAAGTGGCCAGAAGTGTGCTTTTAAC
ATGTGCTCCTTGGGCCATAGTGAGAACAGCCTGAGCTTCTAAGGTATTTGTTTCCTGCACCTTCTCTTGTTTCATCCGTC
TCTCTACCTGCATCAGTCTGTCTCTTGCAGTTGAACTCTAGTTCTAAACCAGATGGTAGATTTAAAGAAAAACATCAAAG
CTTAAAAGTTAGAAAGCTTACACCCAGACACACAGACACACAAACACACCACTTATCTTTGGACACCCCCCCACCCCCCC
```

ACCCAATCCCCTCCCCCTCAGCTCTGTCCAGACCTCGAAAGGGAACTGCTGTTTCCTTAATTTGGAGACTGTAGGCTTCA
CTCCAGGACCACAGCCATGCTGATGGAGAGACAGGAGGATTTCTGAGTTCGAGGCCAGCCTGGTCTACAAAGTGACTTCC
AGGACAGCCAGGGCTATACAGAGAAACCCTGTCTCAAAAAACAAAACAAAACGAAACAAAACAAACAAACAAAAAACCCC
AAGAAAATCAAAACAAAACAAAAAAAAAAAACCCCAACCAACCAAACAAACAAAAAAAGGCCCGGAAAAGGTGAATGTCA
AGGTCTCAAAGCACTCAGTTCTTGATCTCCTACCACCAGCTGTTGCTCACATGTTAAGAGAGCCCGACTTAGGGAAACCA
TAGCCTCATAGAGGAGACAGCGGCTCACGTCTCCTGAGTTCCAAATATTGGGGAGATATCCTGAGCCAGGGGTCCCCCCT
GTGGTTGGGAGACCCAGCACAAGTGCCTGCAAGAAAAGTGAAGCTGCTGGGGTCATGCGAAGGGCAAAGGTAGGAAAGAG
AACACCCCTTTGAGAAAGTGATAGGCAGGGTCTAGCTGGTGCCCCTTATATACCCCACACCAAGCACAGTGTCTTGTCCA
GTCAGTACACAACACAGGGAAAGAATGGGGAGAACAGCTGTGGCCACAGCCCAGGCAGATATGAGAGCCTCTCTGGACCA
GTGGTTCAGGAAGTGATCTTTTCAGTAGATGGCGTCGGGGGTGGGGAGATCCAAGAAGCACCAGGTGTCTGTATGTCCAG
CCCTCTGACGAGGAAATTCAAGGTTCAAAGGGCTCCGGAGTCCAGCCTAAGGTCTCAGCTTATTTTTTGAACTCAGGGTC
TCTCACTAAACTAGTCACCCTGGCTAGACTGGCCAGTAAACTCTGGCAATCCTGTTTCTGCCTCCTCTCTAGCACTGGGA
TCATGAATACACTGTTACACTCCAGTGTTGACAGGGAACTAGGGGTTCAAATTCAAGGTTCCTTTGATAATTCCTAGACT
AAGCCAGGATATGAGATGAGCACTAAGCCCAGAACAGTCCAGTGCCACAATGGAGAAACCAGGCCTACTTATACATCCAT
TGCCCCTCCCCTCGGAGAGTCAAACATGCCTCGCTAATGGTCTCCAAAGAGAATGAGCTTTTGAGCTTTCCTTACGGGAA
CACTTTCCGACGCCAGGCACAGGATTCCAGAGAATACCCTCTGATCTTGCAAGTGTAAAAGGCGGCGCAGTGGCTTCGCC
CCGGTGAGGGAGACCCTAGCTCTGTGTCTTTGAACACCCTCACATCACAGTCACCTCCCAGGGACAAGGGCTCTGTGCTG
ACTCCTCCCCCAAGCAAACGGATGGCCCAGCAGCAACAGCTGGAAGATGAGAAACGCAGACAGGCTGCAGGGGAGCCCCG
GGATGACACGGGGCTGGGGGTTTTCATGTTTTATTAAGAGTGCGGAAGGCCAAGCTGGGGGGAGGAGGGACTGAGGCGTT
TGAAATCCAAAAGAATGAGTTGCTATCAGGTAGGTGAGCTGTTGTAATTGGGGACACCCCTAGGAAAATGACACGGTTG
GAGCGCTGGCAGGTCCTCGGCAGCCTGTGGGAACCTTCGGAAGGCATCGTATCTTGCCTTTTATTAGAAGACATTTTTAA
TTAAAAAATGGGGATTCCCAGCCTCTCCTCCAGGGGCTTTAGTGTCAATGAAAGGATTCAAAGCCATTCTTTGCCTGGCA
CCTGCCTCCGTCTAGAAGGTCGTGGCTTCTGGGGGCCTTGGAGGGAGAGTTCTCAACCTGAGCACTAGACTATATCTTAA
AGGCTATTCTTACCTGTTTTAAGGACCTGTAAAAGCAGGGTGATGCATGCCGGAAATTCTAGCGTTTGTGAAGTTGAGGT
AGGTCAGTTGCCAGCTCAAAGTCAGCCTGGGCTACATAGGGAGAGCCTTACTCAAAACAAGGAAAACACCCTCTCATGGT
ATCCCGAGAACCTCTGCATTTGCCCCATCCCTACCCTCAGAGTCCCAAACACACACTGTTAGCTATGTGCACCATCCT
CCACTGGCAACCTGCCTGCCCAGCCACCCTTAGATCCCCATACTCCACACCCCCCACTGCCCCCGCTATGATTCTTCTGA
GGCTATTATCAATCTGCCAAGGTCCCGTCTGTCACCAGGGTCCCATGAAGTCTGGCCTGGACTAAACTCAGAGGCAAAAT
TTCGGTTACTTGCTTTTCTTTGTTTGGCCTGGAGCAATGAGTCTATCATTAGATTTTTCTCTCTTGGAGAAATATGTCTA
CATGGATAAAACGGTTTGTTGAAAGGGTGGAGAAGGGTGGACTGTAAAACTTGCCGTTCTCCCACAGTGGCTGAAAGCTG
AGTGTTTTCCATCAGTGAAGTCCAGAGCTCTTCCATGTTTAGCCTTAGCCCATCTCCCAGCTAAGCAGAGGACACTAAAG
AAGGCATTCCCCACAGGGACCAAGGAGATGTGTTGTTTATTCTGACTTACCCTTCAGAGGGCTTTTTTTCCCCCCAGTGG
AAAAAAATGTCCCAGGAGCTGGGGAAATGGCTCGGTGGATGAAGTGTTTGCTCAGTAGGTATGAGGAGAGGACTTGAGTTC
AGATCCCAGGTACAGCCCCAAGTGTGGCTGTGCCTGTCTGTAATCCTGGTTCTGGGAGGTGGAGACAGGAAGATCCTGGG
GTTCGCTGAGCAGCCAGTCTAGCCAAAATGGAGATCTCTGGGTTCAATGAGAGACTCTATCTCAAAAAGAAAGCCAGATG
AAGATGGAATTGAGGAATATGCTCTGTCAACCTCTGGCTTCCATGTGCACTCATACCAGCATGAGCATCACATACATACA
CAAGTGTACACACACACACACACACACACACACACACATAAGAAATGAAAGATACTGCCTATATCTTAGGT
GCTAAGCCAACGCCCAAGTAAGATACCCATATAGCTTAATGAAATCACATCTTTCCATCTGGGGTATGAAGTGTGTCCTT
TTGGAACACAAGAGAGAGGCTGTGCACCTGGGAGAAAACAGGAGAAATCTTAGTTTTGTTTCCTGTTGCTGTGTTAAAAT
GTCCTGACATAAGCAATATAAAAGAGATGGCTTTATTTGGCTCACAATGCCAGGTTACTGTCCATCATTGCAGAGAAGTC
AAGGCAGGCACAAAAAGCAGCTAGTCATGTGACACCCAGAGTCAAGAGCAGAGAGACATGAATACACGAATGCTTGGAGA
CAGAAGGCATTCTCAAGGGAGTAGGGACAATAGACATTTGGGCAACTTTGGGTAACTTTAGAACCTGCTGGGATCCGATC
TATCTATCTATCTATCGATCACTTCCATTTGATGGTAGATTGCTGGTGTGCATATGCTACTTTATGGCTTGATGGCTCAG
ATGACACAGATGACACAAGCTCAGGATAGGGAGTTCAGGTTGCATGGTAACCCTGGTGGCCTATTGTCAAGTGTTCAGTT
CCCACCACAGCCCAGTAGCAGGTCGAGAGCTGTCTCTCAAAGGGAGAATAGTTGTCTGCAGATGATGGCAAATTCTTCCT
CGAGGGATTCTCTCGTGGTTCACCTATAGAGGCTTGCCACAGGCTCCAAGCAGCACCTCTGACTGCCACTGCCACCTCAA
GAACCATTGAGTTTGCTGGATCGTGTGACCAAGTGGCAAAGCAGCCTAGACCTGTTGAAGAGCCTTTGCCTGTCCCAGGT
CCCACTCGAAGCTAGCAGCTTGCCAAGTCACTTCGTAGATATCGGCCCAAGCGACACACCCAGGTGAGGAATGTGCTGTC
TCCAGAATCCAAATAGACCCACTAGGCATTGTGCTTCTTTCTTGATGGTGGGAGGGACCAGGTACAACAACTTATCCCTC
ACTTTAGAAGGAATATCTCTGCACGCCTCATGTCACTGAACTCCTATACATTTCCCTGAGGTAGAAGGCCCTTGCATTTT
GGTTGGACTTAATTCCCATCCTTTGATGCACATACGTGTTACCAAGTTCAGAGTGGTTGCAACCTCCAGCTTACTTGGTC
CAATCAGCATAATGCTATCCAATATAGTGGATCAGTGTGATATTGGTAGAAGAGGCAGGTGGTCAGACCTCTTCAAACTA
AGTTATGACACAGGGCGGGAGTGTTACCATACTTTTAAGGCAAAACTACGAACACATATAGCTGGTCTGGCCAGCTGAAA
GCAAACTGCTTTTGGTGGTCCTTGGGCTGAGGGAAAAAAAAAAGGCATTTGCTAGATCAATAGCTACATACCATGTGCCA
GGAGTTGTGTTAATCTGCTCAACTAAGGACACCACATCTGGTACAGCAGCTGCAATCAGAGTCACTATTTGATTAGTTCT
GTGATAGCCAACTGTCATTCTCCACGAGCCATCGTCTTCTTCACAGGCCATACAGGAGAGTTCAAGGGAGATGCGGTAG
AAAGGACCACCTCTGTATCTTTCAAGTCCTTGATGGTGACATTGATTTCTGTGATCCCTATAGGAGAGAGCCGAGACCTG
CTGAGGTGCTTGCAGTGGGTGGAGCAAGTATGGAATGGGTGGTAGAGAAAAGTAGTCATAGCTACCAGCCAAGGTCACGG
TGATCTGCTACAGAAGTGAGGGCTGTAATTGGCCTAAGCGCTTCTGTCTTATTTTGTTACAAACATGTTTAGACATAGAT
CCATTTTGTTAAGAACACGCTTACACATATATTAAGCCAAACTCCATGTTTTCTTTGCGTTTACTTATTTATTGTGTGAT
GGAATATTGATAGAGACGGTATCCACGGTTAAAGTGGTGTAAATCTACATTATCATATTTAAGTGATAAGACACTAAAAG

ATGAAGCATTCAAGACACTTTGCCTCCTATGCTGAGGGGTACACCTGCAGTTAGAGGTGGAGCAGCTATATCATGCTATA
CAGAATTGTGATCCTGTTATCACTTTCATTTGGAAAGTTAGCAAGGTCAAAGGAGATGCGATTGGATGGCAAGAGGGGTC
AACTTATCTTAGTTGTCAACTTTACTAGATCTTGGATCAGGCCAATAGGCAAGCCATTGGACATTCCTGGGAGGAATTTT
CTTGATTAGTTGAAATGAGAAGACTCACTTGGAATACGGGTGGCACTTTCTGAAAACTTCACTGAGTGCGGACAACACGG
TTGGCATTTCGATGTTGTGGACAGACTGTGCTGACATCTGGCCTCTTGGGCTTTCTTAACACAGACTGGTGAGCCAGTGG
CTCTCCAGGAATTCTACATGCCTTCAGTGCCTGATTGACAGCTCGCCCCTCCGGTGTGAAGGTAGCCATCATTGCATGGC
CCAGATGATATCGTGTAAGTTAACCTAATAAACTCCTCTTTAACACACACACACACACACACACACACACACACACACAC
ACTCATTCTATTAGTTTCATTCCTCTAGGAAAAGACAGATGAGAGGAAGCCTGTCTTCCTGCACAGAACGTGAAAGTCA
TCTGCGTGGCTGCTGCAATCATTTTGTGGCCATGAGGCAATAGGCCCAATATTAAAGCCAAAAACTCTGACAGGTACTAA
TCAAGAAGCAGCCAGCCTTGTTATGACTTCATTAAACGTTTGTACCTCCAGGCTTCTTGTTAGGCAAGGTAATTAAGTGC
CTGGTTTTTTTTTTTTTTTTTTGAATGTCAGCACGTTGGGTGTCCTGCTCTTAGAAAAGTAGATCTGGTAGAGTCAACCA
GTTTAGGGCTCCTCGAAGTTTATCCTGGATACAAATCAACCAGACCAGAACTCTTGCTGGAGTTCAGGTGCTGATTCTTA
GTGGACCTGAGGCATAGTTGCAATGAGGTTTAGAATTCTTGAAGCCTGCAGGTGCTTCATTCCCTGTACAGCGAATGGTC
ACTGATCATCCAAGCAGGAGCCTGATGAGTGTAAAGAAAATATAAGAACAAACTTCTTTTTAAAAATTAATTGATTCATT
AATTAGGTTACTCAAGACAGGGTTTCTCCCTGTAGTGTTAGCTGTCCTGGAACTCTATCTGTAAACCAGGCTGGCTTTGA
ACTCACAGCGCTCCACCTGCCTCTGCCTCTCCAGGGCTGAGAGCCACCCAGTTCAAGATCAAATTTCTTTTTTAGATTTA
TTTATTTATTTTATGTATGGGAGTACCCTGAAGCTGTCTTCAGACACACCAGAAGAAGGCATCAGATCCCATTACAGATG
GTTGTGAGCCACCATGTGGTTGCTGGGAATTGAACTCAGGTCCTCTGGAAGAGCAGTCAGTGCTCTTAACCGCCGAGCCA
TCTCTCCAGCCACAGCAGCAAAGAATCCTGGAAGATGTGACTTAGTTGCTGGAGTGCTAGCAGGCATGACGCTCTGGGTT
CCATCCCCAGGACCACACAGTGGTGTGGTGATGCACACCTGTAATCCCAGATAGTAAACGGAGGCAGGAGGATCACAGGT
TCTGTGTCAATCACAGGTACATAGAGAGTACAAGGCCAGCCTAGACGAAATAAGACCCTGTCTCAAAAAAGAGGGGGAGG
AAGAAAGATAGGGATGCTGAGTTACAGCTAGTCTGGCCCCTCTGGAGCAAAGAGAATATAGTTCAAATTCTTCCTGAAAT
TCCAACCCTCTGTGTTGTTCTGTTACTGGACAGTTTCTTAATTGTTACTGGCTGTCAACTCGAACCCAAACCCTGTATCA
CTTTCTTTCTTTTTTTTTTTTTTTTAAGCAACCTCGAGTGCCATTTAATATCACTGCTGCATTGTTACCACAGAAAGCTG
GGTAGATCACGTTAGGGGTGAAAACTTCCCAGAGAGTGTCAACTTGTTGACTCTATTGCTCCCTAATGAGGGAGCAGAGA
TGTCCATTCAAGGATGAGCAGATGGGGAAAGAAGGATGAGAAGCTGGAGGTGGGGAAGGGCTGATTAGAAGGAGCTGAGG
CGTGAACGCAAGCCTTCTTTCTTCATGGGAGTCATGTGGGCGTGGATCTGCCTTGTGGGTGCTTTGGCATATGAAGGACA
CAGCCACCACAGTTAGAAGCCAGTCGGACAGAGTGGGGGAGGGTTTCATACAGTGGTCGCTGCCCAGACGCTCCCGAGAA
TCTGATCTTGGTTGCTCCCATAATTCAGCCTCTTCTGACCTCTGACCTCTGCCTCCTGCCGCTTCCTGAATTCTAAAGTC
ATCAGGACTGGGGTCCTACGCTTTGCCTGCTTCTTCATCTTCAACGAGAAGTGTCTAGTAGGACCTTTGCCTGATGCATG
AAAGTATTATCAGTCACACAAATCCAGAACTCAAGAAAGACTTCCTTGATATTAATAGATTTAATATTCTCTTCTTCG
TCCCAGTTTTAGACTGTCTTGAGCTATGCAAGGTTCTCTGGGAGTTCTCCCTGTATCACTTTCAACCTG

MOUSE mRNA SEQUENCE : mR3-045.1 (Seq ID No: 20)
ATGCAACACCCGGGCCCGCGCCTGTGGCTGGTGCTGCAGGTGATGATAGGCTCGTGCACGGCCATCAGCTCCATGGACTT
AGAGCGCCCTGGAGACGGCAAGTGCCAGCCGGTGGAGATTCCCATGTGCAAGGACATCGGCTACAACACCACCCGCATGC
CCAACCTGATGGGTCACGAGAACCAGCGCGAGGCGGCCATCCAACTGCACGAGTTCGCGCCGCTCGTGGAGTACGGCTGC
CACAGCCACCTTCGCTTCTTCCTGTGTTCGCTGTACGCGCCCATGTGCACCGAGCAGGTCTCCACACCCATCCCTGCTTG
CCGGGTCATGTGCGAGCAGGCCCGGCTCAAGTGCTCGCCGATCATGGAGCAGTTCAAATTCAGGTGGCCGGACTCCCTGG
ATTGCAGCAAGCTCCCCAACAAGAACGACCCCAACTACCTGTGCATGGAGGCACCCAACAACGGCTCGGATGAGCCCAGC
CGGGGGCTCTGGCATGTTTCCTCCGCTCTTCAGGCCCCAGAGGCCCCACAGCGCGCAGGAGCACCCACTAAAGGACGGGGG
TCCGGGGCGCGCAGGTTGTGACAACCCAGGCAAGTTCCACCATGTGGAGAAGAGCGAATCTTGCGCACCGCTTTGCACTC
CGCGGGTGGATGTGTATTGGAGCCGCGACGACAAGCGCTTCGCTGTGGTCTGGCTGGCCATCTGGTCCGTGCTGTGCTTC
TTCTCCAGCGCCTTCACCGTGCTCACCTTCCTCATCGACCCATCGCGCTTCAGGTACCCCGAACGTCCTATCATCTTCCT
CTCCATGTGCTACTGCGTTTATTCGGTGGGCTATATCATCCGCCTCTTCGCGGGCGCGGAGAGCATCGCTTGTGACCGGG
ACAGTGGGCAGCTGTATGTTATCCAGGAAGGACTGGAGAGCACGGGCTGTACCTTAGTCTTCTTGGTACTTTACTACTTC
GGCATGGCCAGCTCTTTATGGTGGGTGGTCCTCACCCTCACTTGGTTCCTGGCTGCTGGAAAGAAGTGGGGCCATGAGGC
CATTGAAGCCAACAGCAGCTACTTTCACCTGGCAGCTTGGGCCATCCCGGCTGTGAAGACTATCTTGATCTTGGTGATGC
GCAGGGTGGCAGGGGATGAGCTCACTGGTGTGTGTTATGTGGGCAGCATGGATGTCAATGCTCTGACCGGCTTCGTGCTG
GTCCCGCTGGCTTGCTACCTAGTCATCGGCACTTCCTTCATCCTGTCCGGCTTTGTGGCTTTATTCCACATCCGGAGGGT
GATGAAAACGGGTGGGGAGAACACGGACAAGCTGGAGAAGCTCATGGTACGCATAGGGTCTTCTCCCTCCTCTACACTG
TGCCGGCCACCTGTGTGATTGCCTGCTATTTTTATGAACGCCTCAACATGGACTACTGGAAGATGCTGGCCACCCAGCAC
AAGTGTAAGATGAACAATCAGACCAAGACACCTGACTGCCTGATGACCACCTCCATCCCTGCCGTGGAGGTCTTCATGGT
CAAAGTGTCCATGCTGCTGGTGGTGGGCATCACCAGTGGGGTGTGGGTCTGGACTTCCAAGACCCTGCAGTCCTGGCAAC
ACGTATGCAGCCGGGGGCTAAAGAGAAAAGCCGGAGGAAACCAGCCAGTGTGGTCACCAGTGCAGGGATCTACAAAAAA
GCCCAGCACCCCCAAAAACCTCACCTTGGGAAGTATGAACTGCCCGCCCAGCCTTCAGCCTGCGTGTGA

MOUSE PROTEIN SEQUENCE : mP3-045.1 (Seq ID No: 21)
MQHPGPRLWLVLQVMIGSCTAISSMDLERPGDGKCQPVEIPMCKDIGYNTTRMPNLMGHENQREAAIQLHEFAPLVEYGC
HSHLRFFLCSLYAPMCTEQVSTPIPACRVMCEQARLKCSPIMEQFKFRWPDSLDCSKLPNKNDPNYLCMEAPNNGSDEPS
RGSGMFPPLFRPQRPHSAQEHPLKDGGPGRAGCDNPGKFHHVEKSESCAPLCTPGVDVYWSRDDKRFAVVWLAIWSVLCF

FSSAFTVLTFLIDPSRFRYPERPIIFLSMCYCVYSVGYIIRLFAGAESIACDRDSGQLYVIQEGLESTGCTLVFLVLYYF
GMASSLWWVVLTLTWFLAAGKKWGHEAIEANSSYFHLAAWAIPAVKTILILVMRRVAGDELTGVCYVGSMDVNALTGFVL
VPLACYLVIGTSFILSGFVALFHIRRVMKTGGENTDKLEKLMVRIGVFSLLYTVPATCVIACYFYERLNMDYWKMLATQH
KCKMNNQTKTPDCLMTTSIPAVEVFMVKVSMLLVVGITSGVWVWTSKTLQSWQHVCSRGLKRKSRRKPASVVTSAGIYKK
AQHPQKPHLGKYELPAQPSACV*


MOUSE PANTHER CLASSIFICATIONS
     FAMILY (SUBFAMILY)
          FRIZZLED-RELATED(FRIZZLED)
     BIOLOGICAL PROCESS
          Signal transduction(2.11.00.00.00) > Cell surface receptor mediated
signal transduction(2.11.01.00.00) > G-protein mediated signaling(2.11.01.07.00)
          Oncogenesis(2.17.00.00.00) > Other oncogenesis(2.17.99.00.00)
          Developmental processes(2.23.00.00.00) > Other developmental
process(2.23.99.00.00)
     MOLECULAR FUNCTIONS
          Receptor(1.01.00.00.00) > G-protein coupled receptor(1.01.01.00.00)


MOUSE GENE ONTOLOGY
     BIOLOGICAL PROCESS
          cell surface receptor linked signal transduction > fz receptor
signaling pathway
          cytoskeleton organization and biogenesis > establishment of tissue
polarity
          cell communication > signal transduction
          GO biological process > developmental processes
          cytoskeleton organization and biogenesis > establishment of cell
polarity
     MOLECULAR FUNCTION
          mannosidase > beta-mannosidase
          ligand binding or carrier > protein binding
          GO molecular function > ligand binding or carrier
          serine-type peptidase > serine-type endopeptidase
          endopeptidase > serine-type endopeptidase
          metalloexopeptidase > metallocarboxypeptidase
          carboxypeptidase > metallocarboxypeptidase
     CELL COMPONENT
          plasma membrane > integral plasma membrane protein
          cell > membrane fraction
          extracellular > extracellular space
          cell > plasma membrane
          GO cellular component > extracellular
MOUSE PROTEIN DOMAINS (INTERPRO SIGNATURES)
          IPR000539 (FRIZZLED)
          IPR000024 (FRI)
          IPR000024 (Fz)
          IPR000539 (Frizzled)
          IPR000832 (G PROTEIN RECEP F2 4)
          IPR000024 (FZ DOMAIN)


HUMAN GENOMIC SEQUENCE : hD3-045 (Seq ID No: 22)
ACCACTCCCGGGCCCAGGAGAGATGAACAAGCATCCTTAGCATAGGCCGCAGGCCCCACGGGGAAAGTGGGTTCTCTGAC
CCCATGCAGGCTGGGTTGAAAGTCAGGCCCCGCCACTGACTGGCTTTGTGCCCCAGAGCCTCCGTTTCCTCCTCTCTAAA
GTGGAAAGACTATGGCACAAGCCCGGGAGAAGGATTCGATGACGTGACACTTGGAGACCACTTGGCAAAAGGTCTCGCCC
TATTTGGGCTTCCCAGGGACACAGATTGCGTGGCTTAAAACTGCAGAAATGTATTCTCTCACAGCCCTGGAGGCCAGAAG
TCCAAAATGAATATATCTGCCAGGCTGCGTACCCACCACAGGGTCCAGAGGAGGCTCCTTCTGCCTTTGCCAGCTTCTGG
TGGCTCCAGGCATTCCTCGGCTCACGGCCACATCACTCCAGTCTCGGCGTGGCCTTCTCTCATATCTTACATCTCCCTCC
GTCTTTCTCTTACAACATCACCAGTCATTGGATGTCAAGCACAGCCGGATAATTCAGGGTCACGTCCTCTCAAGATCCTT
AACATGATTACTTCTGCAGAGACGTAATGTAGAGCTTTTTCCAAATAAGATCACAGCCACAGGTTCCCAGGATCAGGACG
TGAACAGGTCTTTTGGGCGAACACCATTTAACTCGCTAGAAGCACTCAGCGAGCAGCTCTCAGAAACAGTGGCTCTGAGG
ACAGCAGCTGTTAGGATTGTCAGTTCTCTGCCTGTCTCTACCCTCCCTAATTCTAGGGAGAAACTTCATTTACTCCACAA

```
ACCCTAGGACTCCCTAAACCTAGCTGGGGTCTCTCCTGCATAGAATTTCAGAATGCTTAAAAATAAAGCTGTACAAACGT
GTAGATATGAAAAGTCGTGGAGTGGGTGCCAGCTTTTTGCCATTGGTCGTGGTGGCGTTTTATTGTTTCTCTCTTAGGGA
AAACTGTAGGATGGAGTAGAAGAATGAGTTTTCTGGCAGGGTGCAGTGGCTCACGCCTGTAATCCCAGCACTTTGGGAGG
CTGAGGGAGGGGCGGATCACAAGGTCAGGAGATCGAGACCATCCTAGCCAACATGGTGAAATCCTGTCTCTACTGAAATA
CAAAAAAAAAAAAAAAAAAAAATTAGCTGGGCTTGATGGCACACATCTGTAGTCCAGGCTACTCGGGAGGCAGGGAAATCAC
TTGAACCTGGGAGGCAGAGGTTGCAGTGAGCTGAGACCGCACCCACTGCACTCCAGCCTGGTGACAGGGCAAGACTCTGTC
TCAAAAAAAAAAAAAAAAAAAAAAAAAGAAGAAGAAGAAGAATGAGTTTTCCATCAAAATGGACAGCCTTGGTCCATG
GTCAAAATGGACAGCCGGCCCAGGCTTGCCTGGTGGCCCTCTAAGCAGAATCCAATCCCTGCTGACCGTGGCCTCTTCTG
CCTCCGGGGTCCAAGCCACCAGCCTATCTCCTCTGAACCACTGTGGCTGTCTCCTAACTGTTTCTCTGCTTCACTCTTTC
CCTGCCACTATGTGTCCTTGATGCACTAGCCAGAAGGGTCCTGGGAAGACTGAAGTCAGAGCATGGCTCTCGTCTCTCCA
GAACCCTCCGTGGCTCCCCATTTCACTCAGAGTCAAAATCAAAAGGCCCATAGACGCCATCATGGCCCGATTTCTTTCCC
TCCCACTTCCTCTCCACACACCTCTTCTGTCACCTACCTTCTGACGCTGGCCCCTATGTCCTGACTTCCAGCCATTGGCC
CCATTGTTCTTCCTCAAAAACCCCAGGTATAGTGGCACTTCAGGGCCGTGGCACTGGCAGACACCCCATTATCCCAGCAG
GCACTTCTAGAAGTGCCCTCAGCTCTCTGCACCCACTCCTGGTGTCTTTGCTCAGTGGTCTCCTTCTCATCCAGGCCTTC
CCTGCCCATCTTGCTAAAACTGCAGCCCCCCTTTCCTAGCTCTCTGGATGGCATGAGCCGCTCACCTGCCCCGCACGCCA
CGCACCGTGGAACAGGCTGTAGCACGCACATGTGCAGACCCCGGCCTATCGCCTCCTCCTACTGGAAGGTGAACTCCATG
AGGGTGAAGACATCTGTCCTTCTATCCCCTGCTCTGTTTTAACCATCTGGCAAAGCAAGCACACTCAGCGCAGCACTTAT
TAGAAGGGAGGGAGCAGAACAACTATTCTGGGACTGACTTCCTGCTGGAGGAGGAACAGAAAATAAATACATAAGCAGAT
GAGTAAGATCATCGACGGGCAGAAAAAGTACCACAAACAAAATAAGAGTAAGACTGCTCCGAGACAAGGGGCTGGGGAGG
GGGAACCTGAGTAGGAGGCAGCCTAGTGGACGCGTCACCCAGGAGCAGACATCGTTCAGCCATGAGGAGGCGGGGGATGC
AGCCTCCTGATGTGTCAGCTCTGAAAGCTAATGACTGTGCCTGCGTGTGTGCGCATGACATGCGTGAGCTGTAAGGGCAG
CTCCATAATTAGATTATATATGTGTGTGCGTGTATATATATGTCTGTGTGTATATATATGTGTGTATGTATATGTCTGTA
TAATAATATGCATACACACGTGTACATATATATAACCTATATATCTCTCTATATAGATTATATATGTGTATAGTATATAC
ACACATATATATTATATATGTGAATGTGTGTGTATATATGTATATATGTGTATTATATATACACACACATATATATGT
ATACATATATAATCTATATCTCAGAGAGAGAGACTGACCTGCCAGCAGATGGGCAGACATTTGTCAGACATGGAGAGAGT
GTCTTTATTCCTAAAGTTGGTTTCTCAGCTGCAAGGGCAATGGCCAAGCCCTTCCCAATCTTACCTTACGAAACAAGGGT
GACAGGCATTCAGGGATAGCAAAACGATCTTTCCTATCCTATTTTGGGCCTTAGAGAAAAGCGTAATTGTGCATTTAAAC
TGTTGTTTGGGATGAGGGCAGCAGAGCACACAGCTGTTGGAACCAGTTTCTCTTCATGCTATCACATGAGGCTGGAGCCT
GAACTATCTCTGGGAGGCTGCAGAAAGTGGGAAGGGAGTTTCAGATCCATGCTGGAAGAACAGGGAAAGGCTCCCGCAGG
GCTCCGGGTGGTGTCTGCCGCTGATTCAGCAAAGGACTTGTCCTGGACACGGGGCATGTGGGAGCTGAGCACGGGATTTC
AGGTCCTGCTTCACCTCCTTCTCATAGGGAAGAAGCTGTCCACTGCTCACTCCTGGTTAAATTCAAGGAAGAAGGGATAC
ACAAGTGTGGCAGCCCTATAGAAAAGAGAAAGACTCGTAAGTGATGCTATTAGTAAGCCATCTTGGCTTCCGGCCAGTGA
AGACAAATGAATTTAACAAAGAAAGTCAACTAACCAGAAAGTTTACAGACCAGTGAATCCCACAGGGCTAGCATTATGT
GCCAACTCGGTTCTCAGAGCTTACTGAGTTATCTCACTTCATTCCTACCCCGAACCTGATCAAGTAAGTGTCATTGTTCC
CATTTTACAGCAGAGGAAACAGAAAGAATCTCAGAGAGGTTAAGTAACTTGCCTGGGGTCACACAGCTGAGGACATGGGT
AAACCAACACACATTAATACCTCCCACATTGCTTCTTCCGCCATTTCCTCCCTGTTTGTGGCTCCAAAGAATCCCTTCAG
ACACCATCACGGGGAATATCAACATGACAGGAAAGTGGCAGCCTCGCCCCAGGCCAGGAGCCCCTGAACAAAGCCATAAA
CCAAGACTTGATCTTTGCCTGGCTCCAGACAGGACAGCAGAACGGGGTCCCATTCTGAAAGTCAGGCTCCATCACAAGTA
CCCACTGTGCTTGCATCTCCGTGGAGAGGCAGGAGGCCAGGCGTAGGCAGCTGAGCCCCTGGGGGAGTGGAATGGTGTCG
GCTCTACCCCCAGTAGCCCCGTGGCCTGGGTCTAGTGACTCGGACTCCCCAGCCCCAGTTGCTCCTCTCTGAAATGGAAA
TCTTCTCTGGACCAGCTTCCTGGGGTACCTATGAGGAGGCAGTGAGCTGCTTCCTATCAAGGCTTAAGGCAGCACCTGTC
AAATGCTTAGTCAGCCTCGGCTCTTGTTAGCCTTTTGATTATTTGCTTGCCTTGATTATCAGCTTGTATTACTGTGTGCC
TGACACTGACATGTCCCCAAACTCCTCCCCACAAATGTCACATTCCAGTGCCTCTCCTTATTGTGCTCTCTCCCTCCATT
TATATTGCATTTCAACAATAATAACCGCTACTCATCACTGAGCACTTATTTGGTGCTTGGTATTGCCATCAACGCTTCCA
AGTGTGTTATGACATCATCCTCATCGTGCCTTGGGAAGCGGGAACTCTGCTGGTCCCATTTTGCAGATGAGAAACCGAG
GCAGAGCAATTAGAAGTGGCATATCTGAGGTCATGGGGCTAGAAAGTGGAAGAGGGGGATTCAGACCCAGGCATCCGGGT
CGTTTCCTCCCCAGATCATCACTGTGGTGCGGGGCTGCTCAGGCGTTTGGTGAGTGGCTGCAGATTCAGAGCTCACCTAA
GGTGAAAAGCAGGAACGGGGTTCCTTCTCATTTCTTTGTAAGGCAGAGCCAAGCTCGTGGGTGTCCCACTCTGTCCCTG
AAGGGATAGTTTCTCTGTGTCATCTTTGCCTCCTCTGTCACCCATGCAGGGGCCGCAGGCCTGTGATGAGCTGACCCACT
CAGCTCCCAGAACAGGCAAAAGGGTCCCAGGGTGCAGGAGGGAAGCCGAAAGAAAACAGATGGGAAGCGGGAGTAATTAAA
TCAGCACATCCCAAACAGCTAAGGGAGGGGACAAGGAGCAAAGCAATGAGACCAGGTATGAAGTCTGAACTCACAGAAT
GGGCACGCGCTTCCGATAAGCTGTCCGCTCAAGCTGTTTGGAATACAAACACCAACCAAAACAACACACGCACACACTCT
TTTGTGTCGTGTTTCTGGAATTCTGTGATGTTTCTTTTCTCATATATTCCTCCAAAAACAGTCCAGTACAGAATTGCAGG
TTGAAGCACTGTGAATAGGCTCTAGGTTTGCCCCAGGTTAAGGGTTAATTTGTTGAGTTTCAGAACAGGTAGGCGACGCC
CTCCTGGATTTTAAACAGATAACGTCCTTGCCATGTAAGAGCACTTAGAAGGCTGGCTTTATCTCTTTAAAAAAAAATA
TATATATATATATACACACACACACACATATATACACACACATATATATACACACACATATATACACACACA
TACACACACATATATACACACATATACACACACATATATACACACATATATACACACATATATACACACATATA
TACACACACATATATATACACACACACACATATATACACACTATATATATATATGATTGTGTTTGTTGTTGTTG
CAGTTGCAGAGATGAAAAAGAAATCTGTGTCTTTAAATGAGGAGGGGCTGCGTAAGGAGGCTTGGGCAGCAAAAAGGCTA
ATACGCTGTGGAAGAGAAGGAGTTTAATCTAATTTAGTTGGAGGGTGTCTGTCTGCGGTGTTGTTTTTTGGAGTGTGGGA
```

```
ATAGAGGCGGGAAGCTGCCCCCCTTGAATGGTCCCTCCTGAGCCAGGAGCAGTGGGGAGCCAGTCGACGCCAGCGATCCAA
AGCCCGGCCTTGAAAGAGGATTAAATCAATGCTCTGATTTGCAATTCACACTAGTGCACCCCGTGGAACCTTTGGTAATA
TTTCAAACCACATTTCAAGGGAAGTGGCCTTAAAATAGCTGCCTAAATTGTGCTGGAGCCACACTGGACACCCCACTCAT
CCACTTTAGGATGGGGGTGCTTCGGGGTTCATTTCTCACCTTCCCCCAAACCTCCACATCTCACAGCCAGTGTTTTTAGG
CAGAATTAAAACTTTTGCGCCTGTTGAATTCGCTGCGACTTGCATGGCCTTTCTGGTGGTACAAACTGTGTTTTCTTTGC
TAAGTATCACTTCAGATCACTAAAGGTATTTTCCCTGGAGAAAAAGATGGGGGCACGCCACGGGGAGCATGCCTACTTGA
TCAGAAAACATAATTTTTTGTGCATGTTTTATATCTGATAAGTTTCTGCAGAAACTTGATACTAGATGAGGTCAGAGAAA
ACAAGGTCTATTCACATCAGACTTCCCTGCATGTGAAGCAATTATTGGTAAATAGATCAGTTTTCATTCTCATACTTGAA
GGTAGAATTGGAAATGTCCCGGACTAAATACAAACATTGACGACACGAGAAGACAAGAGAAGAAACACAAGAATCACTTA
CGTGGATCCTCACACTTGAATGTACCAAGAGAGGTGATTCAAGCAGCAGCTACCGAAAGGCGTTGCGACAGCCTGAACCA
CTTTTTATCTCAACATTCACCAGCACGATTTTACTTCTGTTTATGAGCTTTCTTCCTATGCCACCAGTTTACTCTTGCAA
TTAGCTTGTAACATGCTAAGCTTATGGTTTAATTTCTTTCTGTTGCTGAAAGACTCAGTACTTTTTATCTTAAAGAGAT
TATTTGGGTTTTCTCTAATCCCATTTCAGAAAACCTCGCACAGTGCTTATAATTTCCATTGACTATTACATAATAAGAAA
AGATACAACTTTGATTTTTAGCAATATCCGTGTCAAAGTACTTTTATACTTTTGACTTTGTGAGTTTAAATGGAATGAAA
CTGGGCCTCTCTTTCAATTCCCCTCCACCTCCAAAAAATATTTAGTTCTTGTTCAAAACACTGCCTGACTCCTGAAATAT
TCTCTCTCAAAAACCTCATACTTTCCCTGGTGAACGTTTGCAAGAACCTGAAGGCGGATAATGTTTACTGATAGCTTTCC
GATAGACAGACATTTTTAGGTGGTTGGGGGGTAAAAACATATTCCTTGTCTCTGCACGTTATGTGAAATCATGTTTAATTA
TAAAGTTTCATTAAAAATCTTTGCTCAAAATTTTAGAAGCCTAATAAGATTTCCATTAATTTAAACATATCACTATTCCC
ACGACGGCTCCAAAGCTTTCTTTTCCGTCTTTCATCCATGGTTAAATATGTAAATAAAATGTCTAAAGTCATTCTTCAAA
GAAGACAAAAGAAAAAGAGCTGAGGGAAAGACAGCCATAAAATTCCTCCCAATTCTCCATAACATTTGCCAGAGGGGGG
TCGGTTGCCAGCCAGGGCTGAGCAGCGGGGGGACCCCGGCTGCTCCTTAGACTCACACATCTCAAATCCCACACACAGGAA
AAAAAATAAAATAAAATATCTTTTCAGCTGGGGCCATCAAAAGAGCCTTTTCATTTATTAGTTACAGACTTATCTTCCAG
TTGCTGATTTCTCAGAACCCTCATTTCTCTATAATAAAGTCTATTTGTTGGTTTCCATCTCAGCTGGACAGGGCGAAGCA
CTTCTGCAGACATTTTTATAAAAAACGAGAGAAAAAGGCAATCTGAAGTGGTTATAGCTAAAATAATGGGCCTGAACGTG
GCTGAATTAAAACAGCACAGAAAAGATCAATTAGGAGGCACTTTCTCAAATAGCTCACACCCAGCTCCCGGCGTTTGGCT
CTCACTCTTGCACTAAATATTTCTAATAGTTGCTGAGGGAGAGACACAAAAAGGAAAAGAAGGAAAATAGGGCCCAGGAA
GGACCTTTGCCAGGAAAATTCTAGAAAAGCCAAGTGATTTCCGCTGAATTAATTTTAATTTAAAGGTCCTGAAAAATTAC
TCACTAGACAAAAACAACAACAACAAAAAAAAAAAACACTATAATAAAAATGGCAGTCTTTCAAAAATAAAATCATGCAA
CTTTTCACTTGGGAAATAACACAGTTGAAGGTGGTGTTCTAACTGCCCGGAGTTTCTACCTTCTTTTTCACTTTCTCCCT
CACCCCGACTTCCTCCAGCACCTCCTAGTGAGACCCAGGACACCCAGGGACAGCGACCCAGGGGTGGGTGCCTGAACCTG
CCTATTAATTCTGGGATTCTGGGCGCTCAAATGTCTGTTGAACGGATGAATGAATGAGTGGAAAGGTGAGATTTCCCCAA
GTTTCTTACCGATGTAAAAATTGTAGGGCTTGTTTTAATTAAAAGCCAATACTCTCCGAGGAAGTGGCCCCCTCCTGCAC
GGCGTGCAGAAAGCTGGTCAGTGAGGGGGGTTCCTGGCGGGCCCCATCTCCTTCCCGGGCGCTTCCTAGGGAGACCTTGC
CCCGTGCGCAGCGTCCCCACCGCGGGGGCCCGTGCAGCCGCCCCCCACGCCGGGTGGAGCGCCGCCAACGCGGGAAGCCC
CAGCACTGCCTCCAAAGATGTCCGCACACTCCACCCTGCTCCCTGGAACTATCCTCAAACGGGCAGGGGGCAAGCGAGGC
CGGTGGACGCGGGACTCGCTCCTCTGACCTCTCCCCGGCCCCCCGGGAGGTAGCGGGAGATTGCACACGCGATCCTGCGA
GTTTCCGAACTTTGGAAGATCGTGACCCGGAGAGACCCTGGCAGGAGAGGGCCGGCCACCTCCTAGGGGTGCTGTTTTTT
TAAGGGTCAACCCAGGACGCTACGGGAAGCACCTGGCGCATCCTTGGGAACAGTGGGGCTTGGCGGTGGGCGCCCACCGC
GAACGCCCTGGGCGGGGGAAGGGAATGGCGGGGGGGACGTCGTGTCCTAAGTGACCCCGTCACAGACCCGCCCCAATCCGA
GGGGGGGATGAGCTCAGAGGACCTGCCCAGGACGCTCCTTCTCCACTTTCCAGGAAAACCGAGCGGCGTGCGCGCCTCCGT
GTCCTCGCGGGAGCTGGGGGTCCCCGGGCAGGCGCTGACGCGCTCCTCCCACGACCCACTCCATCCCACCCTACTCCGGG
TCCTCCTGCGGGGCTGCAGCCAAGGAGGGCGCTGGCCGGGCACATCACTCGCCAAATCACATAAATACCCCACGAGCGAA
TGACTTCCTATAATAAATAAACTCAGCCGCCAAGAAGGCATCTCCAGCCCTGCTGGCGCGAGGCCCCGGCTCTGCGGCTT
CGGAGGGGGCGGGGGTCACCGCGGTACAAACCAAGCCCGCACTTCCCGCGGGGTGGGAGGCGGTGTTCAAAGTCCCGGGA
CGCTTCCCGCCCCTCCCCCTTCCCGCCTTCTTAGAGGAGACCTCGGATGAGCGGCCCTAAAATCCGGATTCTCGAGCCCC
TCCCCCTCCCCTTAGCTCGTCTCTTTGAAATTTCAAAGAAAATCCCTCCTCGGAGCCCGGAGCCCGGGGCTGGTAGCACC
TCCCCAGCCTCAGGAAGATGCGGGCAGGTTTACAAGCAAGGGCCTACTTTATGGCCCAGGCTATAAAACGCAAATCCCAC
CTCCCTTTGAAAGCGCCCTCCGCCCGGGACTCACCATAAAAGGAAGAGAAGATGCACCCCGCGCCCCTCGCCGCGGACCA
CCCCCAAACCCCTTCTCCCGCCAGTCCCCGAACTTCCCGTAACCTCGGCGTCCTCGGGGTGCCGGGGACCATCCCGGGGA
CCGCCGGGAAAGGGGGCGCCGGCGGAGAGGATAGCGAGAGGCAAAGTTGGAGGCCAGGCAGAGAGAGGGCGAGGAGAGGC
GGGCGGGACTGCCGGGGAGGGTCGCAGCGCGCAGAGCCCCGCGCGCCAGGCGGGCTGTCCCGGGGCTGGCGGGCAGACGC
GGGCGGGACTAGGCGCTGCGCCCCCCGCAGGCTGGGCTGCAGGCCGGGGGGGGATCGGCCCCCGCGGGGCCCGGGAGCCCG
CGGCGGGGCGGCGGGGAGGAGGCTGCCCCGGGCCGCTGCGCTTTCTCATGCAAAGCAGCGGCGGTGCGGGGCGCGCGGCC
CGGGGGCGGGGCCTGTCAGCGCTCGGCTTTTCTGCCCCCCTTTGCCTTTTTGCGAGTCTTCTCATCCCGGGACGCAAACC
TCGAAACAGCTGCCGCTGGTCCCGGCCGAGGCCGGCGCAGGGAGGGAGGAGCCGCCCGGGCTGTGGGGGCGCCGCGAGC
TGGGCCGGCCTCGGTGTGCCCGCGCCGCCAGCCCGCTCCAGACGCGCCACCTGGGCGCTCCAAGAAGAGGCCGAAGTTTG
CCGCGGCCGTGAGTTGGAGCTCGCGCCGGGCCGCTGCGCCGGGAGCTCCGGGGGCTTCCCTCGCTTCCCGGTATTGTTTG
CAAACTTTGCTGCTCTCCGCCGCGGCCCCCAACTCGGCGGACGCCGGGCGCGGAGAGCCGAGCCGGGGGCGCTGTGCGCA
GCGCTCGGGCCAGGCCGGGCGGGGCATGGGCGGGGGCCCGAGCAGGGGTGGAGAGCCGGGGCCAGCAGCAGCCCGTGCCCG
GGAGCGGCGGCGCTGAGGGGCGCGGAGCTCCCCGCGAGGACACGTCCAACGCCAGCATGCAGCGCCCGGGCCCCCGCCTG
TGGCTGGTCCTGCAGGTGATGGGCTCGTGCGCCGCCATCAGCTCCATGGACATGGAGCGCCCGGGCGACGGCAAATGCCA
```

```
GCCCATCGAGATCCCGATGTGCAAGGACATCGGCTACAACATGACTCGTATGCCCAACCTGATGGGCCACGAGAACCAGC
GCGAGGCAGCCATCCAGTTGCACGAGTTCGCGCCGCTGGTGGAGTACGGCTGCCACGGCCACCTCCGCTTCTTCCTGTGC
TCGCTGTACGCGCCGATGTGCACCGAGCAGGTCTCTACCCCCATCCCCGCCTGCCGGGTCATGTGCGAGCAGGCCCGGCT
CAAGTGCTCCCCGATTATGGAGCAGTTCAACTTCAAGTGGCCCGACTCCCTGGACTGCCGGAAACTCCCCAACAAGAACG
ACCCCAACTACCTGTGCATGGAGGCGCCCAACAACGGCTCGGACGAGCCCACCCGGGGCTCGGGCCTGTTCCCGCCGCTG
TTCCGGCCGCAGCGGCCCCACAGCGCGCAGGAGCACCGCTGAAGGACGGGGGCCCCGGGCGCGGCGGCTGCGACAACCC
GGGCAAGTTCCACCACGTGGAGAAGAGCGCGTCGTGCGCGCCGCTCTGCACGCCCGGCGTGGACGTGTACTGGAGCCGCG
AGGACAAGCGCTTCGCAGTGGTCTGGCTGGCCATCTGGGCGGTGCTGTGCTTCTTCTCCAGCGCCTTCACCGTGCTCACC
TTCCTCATCGACCCGGCCCGCTTCCGCTACCCCGAGCGCCCCATCATCTTCCTCTCCATGTGCTACTGCGTCTACTCCGT
GGGCTACCTCATCCGCCTCTTCGCCGGCGCCGAGAGCATCGCCTGCGACCGGGACAGCGGCCAGCTCTATGTCATCCAGG
AGGGACTGGAGAGCACCGGCTGCACGCTGGTCTTCCTGGTCCTCTACTACTTCGGCATGGCCAGCTCGCTGTGGTGGGTG
GTCCTCACGCTCACCTGGTTCCTGGCCGCCGGCAAGAAGTGGGGCCACGAGGCCATCGAAGCCAACAGCAGCTACTTCCA
CCTGGCAGCCTGGGCCATCCCGGCGGTGAAGACCATCCTGATCCTGGTCATGCGCAGGGTGGCGGGGGGACGAGCTCACCG
GGGTCTGCTACGTGGGCAGCATGGACGTCAACGCGCTCACCGGCTTCGTGCTCATTCCCCTGGCCTGCTACCTGGTCATC
GGCACGTCCTTCATCCTCTCGGGCTTCGTGGCCCTGTTCCACATCCGGAGGGTGATGAAGACGGGCGGCGAGAACACGGA
CAAGCTGGAGAAGCTCATGGTGCGTATCGGGCTCTTCTCTGTGCTGTACACCGTGCCGGCCACCTGTGTGATCGCCTGCT
ACTTTTACGAACGCCTCAACATGGATTACTGGAAGATCCTGGCGGCGCAGCACAAGTGCAAAATGAACAACCAGACTAAA
ACGCTGGACTGCCTGATGGCCGCCTCCATCCCCGCCGTGGAGATCTTCATGGTGAAGATCTTTATGCTGCTGGTGGTGGG
GATCACCAGCGGGATGTGGATTTGGACCTCCAAGACTCTGCAGTCCTGGCAGCAGGTGTGCAGCCGTAGGTTAAAGAAGA
AGAGCCGGAGAAAACCGGCCAGCGTGATCACCAGCGGTGGGATTTACAAAAAAGCCCAGCATCCCCAGAAAACTCACCAC
GGGAAATATGAGATCCCTGCCCAGTCGCCCACCTGCGTGTGAACAGGGCTGGAGGGAAGGGCACAGGGGCGCCCGGAGCT
AAGATGTGGTGCTTTTCTTGGTTGTGTTTTTCTTTCTTCTTCTTTTTTTTTTTTTATAAAAGCAAAAGAGAAATACA
TAAAAAAGTGTTTACCCTGAAATTCAGGATGCTGTGATACACTGAAAGGAAAAATGTACTTAAAGGGTTTTGTTTTGTTT
TGGTTTTCCAGCGAAGGGAAGCTCCTCCAGTGAAGTAGCCTCTTGTGTAACTAATTTGTGGTAAAGTAGTTGATTCAGCC
CTCAGAAGAAAACTTTTGTTTAGAGCCCTCCGTAAATATACATCTGTGTATTTGAGTTGGCTTTGCTACCCATTTACAAA
TAAGAGGACAGATAACTGCTTTGCAAATTCAAGAGCCTCCCCTGGGTTAACAAATGAGCCATCCCCAGGGCCCACCCCCA
GGAAGGCCACAGTGCTGGGCGGCATCCCTGCAGAGGAAAGACAGGACCCGGGGCCCGCCTCACACCCCAGTGGATTTGGA
GTTGCTTAAAATAGACTCCGGCCTTCACCAATAGTCTCTCTGCAAGACAGAAACCTCCATCAAACCTCACATTTGTGAAC
TCAAACGATGTGCAATACATTTTTTTTCTCTTTCCTTGAAAATAAAAAGAGAAACAAGTATTTTGCTATATATAAAGACAA
CAAAAGAAATCTCCTAACAAAAGAACTAAGAGGCCCAGCCCTCAGAAACCCTTCAGTGCTACATTTTGTGGCTTTTTAAT
GGAAACCAAGCCAATGTTATAGACGTTTGGACTGATTTGTGGAAAGGAGGGGGGAAGAGGGAGAAGGATCATTCAAAAGT
TACCCAAAGGGCTTATTGACTCTTTCTATTGTTAAACAAATGATTTCCACAAACAGATCAGGAAGCACTAGGTTGGCAGA
GACACTTTGTCTAGTGTATTCTCTTCACAGTGCCAGGAAAGAGTGGTTTCTGCGTGTGTATATTTGTAATATATGATATT
TTTCATGCTCCACTATTTTATTAAAAATAAAATATGTTCTTTAGTTTGCTGCTAGTCCCTGGTTCATGGTGAGGTTTCCC
TTCTCTGTGTGTGGAGAGACTACAGGACACTTTCTCCCTTTGCTTTTTAAGCAAATTCTGGGGTTGTGCTTCTAGCCAGA
GAGTACTGGTTATCTGTATAAAATTGCAGATCAGAGGATTCCAAGATTGGAAGAATCATTCACTTGTGAGTTTCTGAGGA
ACTGCAGTCAGGCAATGTGTGAAGTTGGAAGTCGTTTCTGTGACTGTTGGTAGCTGGACATGAGGGCTTAGTTGTAGACA
CACATCACCAAGGAGAGCCTCACCACTAAGCGTGGACCCACTCATGAGCCTGGATTAGCCAGTTTTAATATATTCAGAGA
GTAGAGCCAACCGATTAGGAGAATTAAATCATCTGTATTCAATTGACCTGCTAGGCTCAAATGGTACCTAGACAGAGCTG
TATACATCAGCCCAAAGTGTGCTGGCTTTGCAGTGACTCTGAACTATGAAGACATTTACAAAACAGATTATAGACCTGGA
CGGGATAAAATACCTGCTAAGGGTGGCGAATCCTTAAGAGGAAGTTAAGCAGCTTTGGAACTTCATGGTACTTGGTTCTT
CTCCAGGGTTTCCTGTTAGAACTGGGTAGCTGTAAAGAACTGCCCCAGGTCTGCTGGTCAAAGGCACAGCTACACTCAAG
TTCCAGCCAGAAGGAAGCAAGGGCACAGTGCAGTCCTACTGTGGTGAGGACTCTATTAATATAAAGCAAGCCAAGCTGCC
CCTGGGGCCATGCTTTTGTCCAGCTCCAAGTTAAACAAGGAGCTCTGGAAAACTTGGAGCATGGATGACACGCTTACCAA
CGAGGAGGAGAGCCTGGAGCTAGTGCAGTCTGTTTTCTGAAGAGGGTGTTCAGAACTTCAGCCTGGTAAACATAAGATCA
CCCATCAACACTAGGGCTGGAAACAGGCTTACATTGGCTTTGAGGGGCTACTGCACTTGGCCGTGAATATTTGCAGGGCA
CAGGAGCAGGCAGATCGGTGGTTTGGGATCAAGTTTGACAAACGGAGGAAACACCAGCTAGGTAGGCAAAGTAAAAGAAT
GCAAGATCCCAAAGAGCTTTCAGTGCATTAAAAAAGAAGGCAGAAAAGCTTCAGAAATGCCCCCAAATAAAATTTCTTTG
TTGATTGTTTTCGCCTAGTCCCCCAAAAGACTACATAGAAATATTGATGTCTCTGTGCATGTATGGGTATAGCGTTTCCT
TTTTGTCACTCTTAGGGAAAATGAAAATAACATATTTGGAATAGAACTTTCAAAAAGTCCAGAAGGGTAACATTGTCCGG
ATGCTGCTGTGAGCCACAAGGCTAGATATAACACAAAGACATTTGGTTCTAAAATTCCTGGACTACAGCAGCTTCAGACC
TGCCCATGCTCAGGTGTGTAGGCTCGAAAATGTGGTCCCAAGGAAAGTCATATTCAACAACATACCTAAGAGCCTTTGTT
GTTAGTTATCCCTGAATGTGTCTCACTGTGGATCCATCCCAGAAATGACTGATCTTGTTTACGAATGTGAAGGGATTGTG
ACATGTGGCTGGGTTGGGCGCAGCTGGGTGAGTTTAACAGTGAGCAGTGCAAGGAGCAGCTGTAGAGCTTCCAAGCAAAA
TGCTCCAAGGTCTTCCTCTCCCTCTCTCTCTCTTCCTCTCTCTCTCTCTCTCTCTACTAAGCTGAAATCTGA
CCCTCCCTCACATACAGACAGTGAGTTTTAGGAGAAGCATCAAGGCCTTAAGTCAGAAAGCTGGGGAAAAAAGAAAGGA
AAGAAGGGAGGGAGAGAGGGAGGGAGGGAGGGAAAGAAGCAAGGAGAAAGAAAGACAGGCAAACATCACTAGTGAAGGAAG
GAAGGAAAGAAGGAAAGAAAGAAAGACAGGCAAACACATCACTAGTGAAGGAAGGAAAGAAGGAAGGAAGGGAGGAAAGA
AAGAAGGAAGGAAGGAAGGAAAGAAAGAAGGAGAAAGAAAGAAAGACAGCCAAACACATCACTAGTGAAGGAAGGAAGGA
AGGAGAAAGGCAAACACATCACTAGTGAAGGAAGGAACGAAGGAAGGAAGGAAAGAAAGAAAAGAAAGAAAAAAGAAA
GAAAGAAAGGCAAACACATATCACTAGTCCTTAGATTCTCTTTAGCTCAGTACAGGGTTTCCAAAGGAATTGTTGACAGC
```

```
ATTAATTTGGAGATTTCTCAGCATCTGTACCCCACGGCCTTAGTTTTTAGAAAAATGGCAACCTGTTTTGCATGGCAGTGG
TGGAGTCATAGAAGAAGCCCAAAGAAGTAGATTGTCCAAGATCTACATCCATTCATCCCTCCCTTCCTTCCTCCAGCCTC
CAGCCAATCACCACCCACCACATGCTAAGGCCGAGAAACAGAACTGGTACCTGGTCTGTGTGTGGTTTTGCAGTGGACAG
GAGAGACCTGCATGGCACATCTTATATGCGCCTCTGTGAGGCATCAGGGCAGGAGGCCCAAAAGTAGATGTATTTCCACA
GGGCCCCTAAATGCATACAAGGCAGAAGATTCAGCCTATGTGAGGTCATAGCAAGGGGAAGCTGCAGGGTCATATAGAAG
GCAAGAGAAGTTATCAACAGAGCATCAAAACATTCCATCGGGACCCAGACTGGGTCTGGCTCATGATCCCCTATGTAAGC
CCACACCTTGCAGAGGGCTTTGGACGTAGTAAGAATAACTACAATAATAGCAATGGCTGTGATAATAGTAATCGTAGCAG
CACAGCCTTGAGACCACTTGCTGTGTGCTGAACAGGCTGCTGGGACTATCTCAGTGAATCCTCAACGCATTCAGGGAGGT
ACTGTCATCAGCCCCACTTTACATGTGAAGACCCTGACACAGAGAGGTTAAGTAAATTCCCAGGGTCACATGACAACTAA
AAGACCCAGAACAGGAACTGGGTGGTCTGATAACAGATCTGGAACCCCCAATTCAGTAAGCAATTGTTGAATAAATGAAT
GAATGAACAATAGCTGACCACTTTACATGCATTAATTCAATCCTCTGACAAACTTTGGTGGTACTGCTCTCATGCTCATA
ATATTACAGACGAGGAATTTGGAGCACAGAGAAGTTGTGTCACTTACCCAAGGTCACACAGGCAGCAAGCAGAGAGTCTG
GCATGCAAACCTGGGCTGTGGGACTCCAGACCTGCACCTCTGACCACGAGGCTTTCCCACGTCTCTGGTTTCTCAGGACT
TGACTAAATCATTTCATCTTCACAGCAGCACTGCTGCACCTTTATTCCAAGCAAAAGGGGAACAAGGTTGCATGGCAAAG
AGCAAAAGCCACTTGGCCAGTGTGGGGGAGCCCAAGGGAGGAGGCCGTGGCAACCTGCATTTTTGCAGCAGTCGCGAGGG
TGCTAGCAGTGGGGCTAGGGGGAAGCAGGTGGATGGAGAGGTCCTTAGAAGGTAGAACCAGGATTAGGTTTTATGTAGAG
AGGGAGCAAGAAGGAATGGTCAGGGAGGACCACACCATTTCTGACTTTGGTGGCAAAGTGACATTCATCAAGACAGGAGA
AAACCAACAGAGGACAGGCAGTGTGATGGGAAGCAAAGATGAGTGCAGTGTGGATTATTTGCCATCGAGGTGTCTGTAGA
GAATTTGGGATGAGGTCATGGTCACAGGCGGGTGTTTGAAATGGTGAGAGTTGGAGCTGTGGTTAACCTCAGGAAGCAAG
TGGGTCTCTCCAGGGACAGAGGAGGGAGGAGGGCAGACCTCAGGGCCAGGTGAACAAAGAGGAGGTTGCAAGGGGAGCTG
TGACTGGGAGCAGCCCCTTAACCACTTGGGCGCTGGGTGCCCATTTTTACTATAAGAGGATTGGCAGAGGAGATTATGCA
AATGGCCTTATCCAACGTCAAAATACGTGATCCGGATAGCGCTAAGGGGGAATGCAGCAAGGGTCTTACAGGAGTGACTT
AGGAGGATCCACTCCTGCCCGTGATAACTCAGCCTCTGTATCTTTAACTTAACCCTTGGGCTACCCGAGCAAAATTAAAA
TCAGCTCCAAGAGCTCATAGTTTTAAATTCTCCACTAGCATCTTTGGGGATGCTGTTTCAAGACAGAGAAACAGAAGAGA
AAAATCAAGAAGTATTCCTGAGGCCATAGATATTATCCTACATTCTAGATTATTTTCTCAGAAGGAGGAGAAAGAAATAC
AGAAAACGATAACAGATTGGAGTGCAAACTGGCATCTTACACTTAAAGGATGTGCAGATATAAACATCGTTTAGGACTGA
ACTATATCTTGCCTTCTCTATGAGCTGGTTTTTAGTTTGACTCAACCACTTACACATTGCTTCACCTTGAGCAAGATACC
AGAACTTTCCTGCCTCTGTTTCTTCATCTGTAAAATGGGCATGATAATGGTACCTATCTCACTGGACTGTCACTCACAAA
TAGCTCATGCTTATTGAACAAGTATTACATGCTCGATTGCTTTAAGTTCTCTGTGTTTATTAATTCATCTACTCCTCACA
ACAGCCCTAGGAGAGATGGTACGAGATTAAATAAAACCATTTGTGTGAAGTACTTGGAAGGCTGCTTGGCGCAGAGCAAA
TGCTCAATAAAGTGATCTATTTTTATGTTTTTTGTCCCTCCCCCCAAAGAAAGTTAAATCCTGTAAGCTAATGAGTAGGG
CTCTTTAAAGAAAATGAGTTTTTTTCTTACAGAAACACTTTTTCCAATACTGTGTTCAGGTTCCACAGAAAACCCACTGA
TCTTTCAAGTTTAAAAGGCAGGCACAGAGCCTCTACAGCAGTGAAAAATATTCTCTGTCTTTCCCTTTGAACACAATAAC
ATCACAGCTGACTTCCCAGGAAATCAGACCTCATGCACGGACACCTCTATACGCAAATGGATAAAGATGGCTGTAATATG
GAAATGCAGAGAAACGGTCAGACTTCAGGGAAGCTCGCGAAACACGTGCAGGCAGCATAGTGTGGGGTGTTTTTTAATGT
TTTATTAAGAGTGTGAAGGCCAAGCTGGAGGGAGGAGGGAAGGAGACGTTTGAAATCTGAAAGAATAAGTTTCAGCTGCC
GAGGTGAGCTGTCGTAATTGGGGACACTCCTAGGAAAATGACACTGTTGGATCCCACACAATGTCCGCAGCCCATCCGGA
CTTCCCCAAAGTCAGTGCCTGTCGCTTTGTTATTCTAAGACTTTCTGAATGTTTCTGTTTTTATATACATTGTTTGGTAC
CAGGCTGCCCTCCAGGGGCCTTTGGTTTATAGGAAGGGATTCATAACAGCAACCCTTGCCACACGCCCATCTCCTTCCCA
GATGCTGGGGGCTTCAGGGGTTCTGTAGATGGGATGGGATGTTCCCAGGTGGTCTCTTGCAACAGATGTCCTCAAAGGAT
GCAATTTGTGGCATTTTAGGGAATACTTGCACTTAAAAAAACATCTAAAACCTATCCTAACAATGTTTTAAGTATTTTAG
GCAGAACTACAAAGTGATCGACCTTGACATGATGAATCAACACCCATTTTCCAAGGGGGCGATGGTGATCGGGAGCTGCA
GGTGCTGACTTTGATCAGAACTGACAAGCTCATGGGGTGGTGCAGCTCTCTCCCCACCCCAGCCCTCCCCATTCCACCCC
TGCCCCCAGGGCAGCAGCACCTGTTTACCTCCCATGCTCTCCCTGCACTATTTTCGCCTCTCATAAAACACTTGGATGTT
GTTGGCTCTGAATAAGATCATTCATCTGCGAACTGCCTCTCTTCCTGCAGCTCATCTGAAACAGTCTTATTGAACTTGGG
TCAATATCACCTCGGTTTCTTTGGATTCCAGGATCACAGCAGAAGGCTCAAATTTGAACTAAACTCAGAAGCATTTTAGG
TCAGCAAAACTTTTTTTTTTCCCCAAGAGACTTTAAGTATTAGGAATCTAAGTGCCACCTTACTTTTCCACACATGGCACA
GGGTAAAGAGAACATGAGATAGCCATGAAGAAGTTGTGATTCTGATTTGCTATCATGATGACCCAGGTCTAAGAAGACAG
AACAAAAGCAATGAGAACAAGAGAACACGGCCAATTATAACATTTGACATTCTTCCATGGCAGTCATGATTGTAACCATT
TTCAATTAAAAGAAAAAACACAATACTACAGCAATGTTCTGAGCTGAATCTTATCTCTTGAGTAAACAGAGGGCCTCTGA
GCAAAAAACCATGCAATAGAAAGGAGGCATCAAAAGAAGTTGGAAATGTAGTATTTTTATTTTTAACTCACCTCTACCTT
CTAAAAGCTTTATTCTTTCCAAGACAAAAAGCTTCACGCATGTCCCAGGGGATGAGCCAAGAAAAAAAAAAATCAGGTAA
AATAATCCCTCTGGGTCAGGGGCGTGCATGTCACTCGGTCTGGTCAGTGAGATGTGAGGGGAGCTTTCCTAGTGAGACAA
AAAAAAAATGAGGGTGTGCGTGTGGGAGTGAAGGAAGGTTCTAATAAAAGATTTTGCTTCATGATAAAAAAAAAAAATGA
TGAGGAAACCTGTCCTTTTCTTCTGCACACAATGTGGTGTGTGAGAATCACATATGGAGCTATTGCAACCATTTTGTGAC
CACGAGGCAATAAGCCCAACACCAAAGCCAAAATTCTGAGATGGCAGGTAAGCAAGACAGAGCCAGCCTCGTGAGCACTC
ATTAAGCGACTGCACCCCCAGGATTCTTGTTATGCCAAGTAGCCAACACTGCCTGGATCCCCTGAGAGTTGCAGGTTGGG
TGTTCTGTGCCTCTTAGGAAAATACACCTGATGTGGCAAATGAGTCCCAGGTGTCTCAAACCCTACTGTGCACACAAATC
ACTGGGGCTCTTGCTAAAAGGCAGCTCCTGATTCCTTAGGTCTGGGTGGGACCCCAAATTCTGCATTTGTAATAAGTTCC
CAGTTGATGTCGATGCTTCTGGTGCTCCTGACCCCTTTGAGTGAAGAATCTATTTGGCCACCCACCAGAAGTTCAGAACG
ACCCCACTTACTCCTGGCTGTAATTATATGAACACATCTTGGGCATCACTTCCTGGGTGTCCTGTAGTGGACTAGCTGTC
```

```
AATTAAAACAATTTCTTATGTTGAGTTAAAATTTGCTTCCTTGTAACTATTTTCTATTATTATCTCTACTTTTGCCACAT
AGAGCTTCACAGAGCAAGTCTACTTGTTTTTCCATGGGAAAGCCACTTATATATGGGAGCTTATCACAAATGCAGAATTT
GGGGTACCACCCAAACCTACTGACTCAGGAGCTGCCTTTTAGCAAGAGCCCCAGTGATTTGTGTGCAGAGTAGGGGTTCAA
GATGCCCAGGACTAATTTGCTATATCAGGTATATTTTCCTAAGAGGCACAGAACACCCAACCTGCCAACTCTCAGGGGAT
CCAGAGAGTAATATGTGCTCCCTGGATCTTTCTTTTTATTCTTAAAACTGTTGCCCAGATAACTTGGTTTCATCTTGCTG
GCTGTCCCCTGAATGTGTTTACCATGGTCTCAATTCTTGTCAAGGAAGAATGTGCCCAAGTAAAGACAAATCTCCAGGAA
GCATCTAACAAGGAGTGGGATCATTAGACACCCAGCAAATGTTATACAAGATAATGGGACACTAAAAGCATTTTTACTAA
AACCAGAGACAAAACAAGGCTATCTATGATTACTGGTCACTACCCGGAACATGATTAACCCTGAAGTCAGAGAAGAGGAA
AGAAAAAGGAAGGTAAAACCATTGGAAACTGTCATTATTTGCAGCTGTTGTCATTGAAAAGTGACTTAGCTGGAAATAGA
ATTGATGAGATTTTCATAAGGAGACCAGGACAAAACATAGCTACAAAAATCAATAGCATCCTCATGCACCAGCAGGAACC
AGTTGAAAATATGAAGAAAATTTAAGATCACTTATGCAATGCCTACAACAAGCACAAACTATCTAGAAATTAACATAGGG
GGTGTGCAAGCCTTTTGGAAGACAATGTGAAGATGTTACTGAAGAGCATAAATAATTGGCTCAAATAATTGGAAGCTCAT
GTCATGTGCCTGTAAATATGTCAATTATCCCCAAGTTAATTCCTAAACATAATGCAATTGCAACTTTAAAATATGTATTT
CTGTGGAACTCCACAAGATGATATTAAATCCATAAAATTTGAGCAGTAGTCAAGGAAAAACCCTGGAACAGGGATAAGA
AAGGACATAATCTATCAGTGAAGTACACTGTGAGTCTGCAGAAACAGACAGCATGGGCCAAGTGTTAAAACAGACAAAT
AATGGAACAAATAGGAAATCCAGAAAGAGTCTCAAGTGCATTTCAAATAAATAAGAAAGAATGGATTACGCAGTCAATA
CTGTTGAGGCTTAGTTTAGATGTGGGGAAAGTAATATTCCATCCCCACCTCATGCCTTTCACCAAAATACATGTTGGATT
GATAAAACATTTAAACATAAAAATAATACAGTAATTAGGGGAAAATGTTATCTAAAGTTTTCATTCAATAGAAAGAAATT
ACAGGTAAACATTTTTTATAAACTTATCAGGAAAATGTCTTCTTAGCATGACATCAAAGCTAAAGCCATTTTTTAAAAAT
CATAATTTCACTACATAAAAATGTAAAATTCTGTAATGTAAGAAATCAGTTAATTTAAAAAAAATTGCAAATCTCCATA
AACAAAATTAAAAGACAAAAACTTAAGTTTCTTGTCTGGGTCTATGAGTAGGCAATTCAAATTAGAAGAAATGCTAGTGA
CCCATAAACCTGTGGAAAGTTATTTAAGCTCACTTCTAATAATCCAAGGGTGCATGCATAAAAAAATGATTATTTGTGCC
TAACAGGTTTCCCCGTTTTTTTTTTCTTTGAGACAGAGTCTCTCTCTGTTGCCCAGGCTGGAGTGCAGTGGCATGATCTC
GGCTCACTGCAACCTCTGCCTCCCCAGTTTAAGCAATTCCCCCGCCTCAGCCTCCCAAGTAGCTGGGATTACAGGCGTCC
ACCATCATGCCCGTCTAATTTGTTTGTATTTTCAGTAGAGACAGGGTTTCACCATGTTGGCCAGGCTGCTTTTGAACTCC
TGAGCTCAAGTGATCCGCCCGCTTCGGCCTCCCAAAGTGCGAGGATTACAGGTGTGAGCCACCGCGCCTGGCCAGCTTGG
TACTTTTTTTTTTTTTTAAAGCGGATAATACCTGGATTTTGCTCAGGCATGGAGAAATGGACTCCCTGTATCACAGAGT
GGAACCATAAAGTAGCTCACCCTTTCTGGAGAACAAGTGTGATGTGTACCTAGTGCCGTTTGCAGTATACATGTCCTTTG
ACCCAGCAATTCCACTTCCAAATAAATGTTGTATTGAAAATTGGGAACAGTCTAAATATATACCGGTAAGCTGACTATTC
AAGCCAATTATGGAAAATCCTTAAAATAGAGTAACATGTAGTCATTAAAGTAATTACGTAGAACCGTGTCATTAACATGG
GAAAATGGCCACACGGCACTGCTAAGCAGGCTGATCTTCAGACAGGACGGACCAGGGCAGCCCTTCCAGTTTGTTTTGTC
TGGTGTGTTTTTGAATTTGTCCACACCCCTATGCCAAATCAGTTGTTGAACATTTCAAATATTGCCCTGTTGTTACTACA
CAAAGCAAGTTATATAATTCCTGAATAAGACAAGCCTGGGCAACACAGGGAGACCCTGTCTCAAAAAATAAAAGAAAGA
AAAGGAAGAGAGGAGGGAAAGGAAGGGGAGGGGTGGGGAGGGGAGGGAAGGGAAGGGGATGGGGAAGCAAAGGAAGGCCG
GGTGCAGTGGCTTTCGCCTGTAATCCCAGCACTCTGGGAGGCTGAGTGGGCAGATAACTTGGGGTCAGGAGTTTGAGACC
AGCCTGACCAACATGGTGAAACCCCGTATCCACTAAAAATACAAAAATTAGCTGGGCATGGTGGTGGGCACCTGTAATCC
CAGTTATTCAGGAGGCTGAGGCAAGAGAATTGCTTGAACCCAGAAGGCAGAGGT

HUMAN mRNA SEQUENCE : hR3-045.1 (Seq ID No: 23)
TCGAAACAGCTGCCGGCTGGTCCCGGCCGAGGCCGGCGCAGGGAGGGAGGGAGCCGCCCGGGCTGTGGGGGCGCCGCGAGC
TGGGCCGGCCTCGGTGTGCCCGCGCCGCCAGCCCGCTCCAGACGCGCCACCTGGGCGCTCCAAGAAGAGGCCGAAGTTTG
CCGCGGCCGTGAGTTGGAGCTCGCGCCGGGCCGCTGCGCCGGGAGCTCCGGGGGGCTTCCCTCGCTTCCCGGTATTGTTTG
CAAACTTTGCTGCTCTCCGCCGCGGCCCCCAACTCGGCGGACGCCGGGCGCGGAGAGCCGAGCCGGGGGCGCTGTGCGCA
GCGCTCGGGCCAGGCCGGGCGGGCATGGGCGGGGGCCCGAGCAGGGGTGGAGAGCCGGGGCCAGCAGCAGCCCGTGCCCG
GGAGCGGCGGCGCTGAGGGGCGCGGAGCTCCCCGCGAGGACACGTCCAACGCCAGCATGCAGCGCCCGGGCCCCCGCCTG
TGGCTGGTCCTGCAGGTGATGGGCTCGTGCGCCGCCATCAGCTCCATGGACATGGAGCGCCCGGGCGACGGCAAATGCCA
GCCCATCGAGATCCCGATGTGCAAGGACATCGGCTACAACATGACTCGTATGCCCAACCTGATGGGCCACGAGAACCAGC
GCGAGGCAGCCATCCAGTTGCACGAGTTCGCGCCGCTGGTGGAGTACGGCTGCCACGGCCACCTCCGCTTCTTCCTGTGC
TCGCTGTACGCGCCGATGTGCACCGAGCAGGTCTCTACCCCCATCCCCGCCTGCCGGGTCATGTGCGAGCAGGCCCGGCT
CAAGTGCTCCCCGATTATGGAGCAGTTCAACTTCAAGTGGCCCGACTCCCTGGACTGCCGGAAACTCCCCAACAAGAACG
ACCCCAACTACCTGTGCATGGAGGCGCCCAACAACGGCTCGGACGAGCCCACCCGGGGCTCGGGCCTGTTCCCGCCGCTG
TTCCGGCCGCAGCGGCCCCACAGCGCGCAGGAGCACCCGCTGAAGGACGGGGGCCCCGGGCGCGGCGGCTGCGACAACCC
GGGCAAGTTCCACCACGTGGAGAAGAGCGCGTCGTGCGCGCCGCTCTGCACGCCCGGCGTGGACGTGTACTGGAGCCGCG
AGGACAAGCGCTTCGCAGTGGTCTGGCTGGCCATCTGGGCGGTGCTGTGCTTCTTCTCCAGCGCCTTCACCGTGCTCACC
TTCCTCATCGACCCGGCCCGCTTCCGCTACCCCGAGCGCCCCATCATCTTCCTCTCCATGTGCTACTGCGTCTACTCCGT
GGGCTACCTCATCCGCCTCTTCGCCGGCGCCGAGAGCATCGCCTGCGACCGGGACAGCGGCCAGCTCTATGTCATCCAGG
AGGGACTGGAGAGCACCGGCTGCACGCTGGTCTTCCTGGTCCTCTACTACTTCGGCATGGCCAGCTCGCTGTGGTGGGTG
GTCCTCACGCTCACCTGGTTCCTGGCCGCCGGCAAGAAGTGGGGCCACGAGGCCATCGAAGCCAACAGCAGCTACTTCCA
CCTGGCAGCCTGGGCCATCCCGGCGGTGAAGACCATCCTGATCCTGGTCATGCGCAGGGTGGCGGGGGACGAGCTCACCG
GGGTCTGCTACGTGGGCAGCATGGACGTCAACGCGCTCACCGGCTTCGTGCTCATTCCCCTGGCCTGCTACCTGGTCATC
GGCACGTCCTTCATCCTCTCGGGCTTCGTGGCCCTGTTCCACATCCGGAGGGTGATGAAGACGGGCGGCGAGAACACGGA
```

```
CAAGCTGGAGAAGCTCATGGTGCGTATCGGGCTCTTCTCTGTGCTGTACACCGTGCCGGCCACCTGTGTGATCGCCTGCT
ACTTTTACGAACGCCTCAACATGGATTACTGGAAGATCCTGGCGGCGCAGCACAAGTGCAAAATGAACAACCAGACTAAA
ACGCTGGACTGCCTGATGGCCGCCTCCATCCCCGCCGTGGAGATCTTCATGGTGAAGATCTTTATGCTGCTGGTGGTGGG
GATCACCAGCGGGATGTGGATTTGGACCTCCAAGACTCTGCAGTCCTGGCAGCAGGTGTGCAGCCGTAGGTTAAAGAAGA
AGAGCCGGAGAAAACCGGCCAGCGTGATCACCAGCGGTGGGATTTACAAAAAAGCCCAGCATCCCCAGAAAACTCACCAC
GGGAAATATGAGATCCCTGCCCAGTCGCCCACCTGCGTGTGAACAGGGCTGGAGGGAAGGGCACAGGGGCGCCCGGAGCT
AAGATGTGGTGCTTTTCTTGGTTGTGTTTTTCTTTCTTCTTCTTTTTTTTTTTTTATAAAAGCAAAAGAGAAATACA
TAAAAAAGTGTTTACCCTGAAATTCAGGATGCTGTGATACACTGAAAGGAAAATGTACTTAAAGGGTTTTGTTTTGTTT
TGGTTTTCCAGCGAAGGGAAGCTCCTCCAGTGAAGTAGCCTCTTGTGTAACTAATTTGTGGTAAAGTAGTTGATTCAGCC
CTCAGAAGAAACTTTTGTTTAGAGCCCTCCGTAAATATACATCGTGTATTTGAGTTGGCTTTGCTACCCATTTACAAA
TAAGAGGACAGATAACTGCTTTGCAAATTCAAGAGCCTCCCCTGGGTTAACAAATGAGCCATCCCCAGGGCCCACCCCCA
GGAAGGCCACAGTGCTGGGCGGCATCCCTGCAGAGGAAAGACAGGACCCGGGGCCCGCCTCACACCCCAGTGGATTTGGA
GTTGCTTAAAATAGACTCCGGCCTTCACCAATAGTCTCTCTGCAAGACAGAAACCTCCATCAAACCTCACATTTGTGAAC
TCAAACGATGTGCAATACATTTTTTTCTCTTTCCTTGAAAATAAAAAGAGAAACAAGTATTTTGCTATATATAAAGACAA
CAAAAGAAATCTCCTAACAAAAGAACTAAGAGGCCCAGCCCTCAGAAACCCTTCAGTGCTACATTTTGTGGCTTTTTAAT
GGAAACCAAGCCAATGTTATAGACGTTTGGACTGATTTGTGGAAAGGAGGGGGGAAGAGGGAGAAGGATCATTCAAAAGT
TACCCAAAGGGCTTATTGACTCTTTCTATTGTTAAACAAATGATTTCCACAAACAGATCAGGAAGCACTAGGTTGGCAGA
GACACTTTGTCTAGTGTATTCTCTTCACAGTGCCAGGAAAGAGTGGTTTCTGCGTGTGTATATTTGTAATATATGATATT
TTTCATGCTCCACTATTTTATTAAAAATAAAATATGTTCTTTAGTTTGCTGCTA
```

HUMAN PROTEIN SEQUENCE : hP3-045.1 (Seq ID No: 24)
```
MQRPGPRLWLVLQVMGSCAAISSMDMERPGDGKCQPIEIPMCKDIGYNMTRMPNLMGHENQREAAIQLHEFAPLVEYGCH
GHLRFFLCSLYAPMCTEQVSTPIPACRVMCEQARLKCSPIMEQFNFKWPDSLDCRKLPNKNDPNYLCMEAPNNGSDEPTR
GSGLFPPLFRPQRPHSAQEHPLKDGGPGRGGCDNPGKFHHVEKSASCAPLCTPGVDVYWSREDKRFAVVWLAIWAVLCFF
SSAFTVLTFLIDPARFRYPERPIIFLSMCYCVYSVGYLIRLFAGAESIACDRDSGQLYVIQEGLESTGCTLVFLVLYYFG
MASSLWWVVLTLTWFLAAGKKWGHEAIEANSSYFHLAAWAIPAVKTILILVMRRVAGDELTGVCYVGSMDVNALTGFVLI
PLACYLVIGTSFILSGFVALFHIRRVMKTGGENTDKLEKLMVRIGLFSVLYTVPATCVIACYFYERLNMDYWKILAAQHK
CKMNNQTKTLDCLMAASIPAVEIFMVKIFMLLVVGITSGMWIWTSKTLQSWQQVCSRRLKKKSRRKPASVITSGGIYKKA
QHPQKTHHGKYEIPAQSPTCV*
GRES DISCOVERY 3-045
```

Table 5

MOUSE GENOMIC SEQUENCE : mD5-001 (Seq ID No: 25)
```
AGTTCAATCCTGAAGAAACCGCAAGGCTCACCAACTGGCCTGAGGCCTCAGGAGTGGCGAGCTGCCGCAGGAACCTCACA
AGCAGGTCTTGGGTGAGTTTCTCTCAATGGTGGTATTTCCACAAGTTCAGCTCAACAGTGCAATGTAAGGCGAACCAATA
CAAGCATGTTGTTAGTGAAGAGTAATGAGGTGAAACAACCCAAACCAACGCTAAACGCTTCTCTCTCACTGTCTGTGGGG
TCATATTTATACTCCTTCATCCCTGGCCCTTTCACATGTTTGTTATATCAAAACGTCCTTTCACCTGTGTCTGCTTCAGA
TAAACGTTCTTTCACGTGTCTGCTTTAGCAAGACATCCTTTCACCTGTGTGCCCCAGCAAAACATCATTTGACATAACTG
ACTTCCCAAAGAAACTAGAAGTTGCCACTTCATCTCCCCCTTCCCTCTGAATGTGCTTCCCTCTTGTACAGCAGTGACAC
CCAGCAGTGTGTTCTCTGTGTGGGGTGAGCTGATGGTGGTCGATTGCACATTTTTTTTAGAATCAGTGGCTAATCTTTTA
TCAGGGGTGGGGGAGGATTTCGAACTCCTTTAAATCCTGATCTTCAACTGGAGTGGTGGACAGACCTGCAATCCCAACAC
ATGGGAGTCTGAGACAGTAGGATTGCTCCAAGTTCAAGATAAGCCTAAACTACAGAGTGAGACATTACCTCAGCCACCCC
ACCCCCAGTTAAATACTTATAAATAGCTCATGTTCAGCTGTTGTTGCAAATTCTGGGTTTGCTGGCAGTATTGAGCCAGC
ATTTCTCACCAGTATGCCATGCACTCCACCTGCCCCCCATCCATCTGAAATAAAGTCAGTCCTACCCCTCCAACTGCAGT
TGGAAACCCTGTCTATGCCTACTAAGAGGGATACCAGGCTGCCTCTCTCTCACGGTGCCTGAAGCTTTGCTTGTCCCTTC
ATGTGCCCATGTGCCCCCCTGCACCTGTTCAGAACAAGAATCTTACATCTTGGATGGGATCCCTCAGTCCTGAGCTGTCC
CTCCTTCAGGGAAACCTGCCATTGCTACCATCCAGGAACAAGGGCACCTGGATGCGTAGTTCAACGGTAAGAGAGCCACA
TTTTTTTCTGGCTGTGCACCTGAAGAGTCCTTATATTGGGAAGCCAAGAAGCTTGGGTCACAATGTAGCTAATTTCTGTT
TGTTCCCCCAGATGTCACTGGGTTTATACTATGTTATAGTGGAGCTGGAGTGACCCTTGCTGTGTGCCTTCACACATTT
AGTAATGGCAAGAGCCTCTTGTCTGATTGCTATGAGGGCCCAGTGTGAAAACACTGCATGGAAACTTCAGGTGGCCATAG
CTCAGCCAGGGCAGGAGGTCCCAACACATTTTCTGTGAATCCTCATTAGTGGCCCAGCGCCTTCTGGAGACCTTGTACCA
CCCCAGAGCTCCTGTCCCCATAGGCCCAGCTTAGTCCTGCAATTCTGGGGAGTATATCTAGATACCCCATTGATATCTGG
ATCCTCCATTGATAGATGGTACGCAGTTGTACATGTGGCCAGAACTGGGAAGACACTGCCTTTCTCCTCTCAATCCAAGC
TGATTTAGATTCTTGTAGTGCCGAGAGAGTCATGACTGGGTATCACAGGCAGGCCAGGAAGGATTAACCTACTGTCTGT
ATACATTATAGCTTCAGACCCTGCTTTGTTGCTGCCCACTGTCCCAACATAGCATCTTTGCTGTTCAGCGCACAGAAGGT
CCCCTGTTCTTTGGAGTCTCCAGTCACCAGGTCCCGGAGCCACCCCTCACAAGGTTGGGGAAGGGTATCATTCCAAGATG
GAGTGGGTTTCTAAGCCGGGTTTCTGCTTCTTCCAAGCAGACATGTTGGCCTCGAAGACAGCTAGGCAAAGCCTTCTCAG
ATGGACAATTGAATTTTCAGTTCTTTAATAAAAACACTAACGCAGAACAGAAGGGAAGCAGGGCTTCTAGAAAGAGCTTC
TCACAGGTGTTCTGCAAACATCACACGTCCTTTCCTCGAGGTGACACCTAGCCTGTCTTCTGAGAAAGATCAGAGATCTT
```

```
GGCGAAACCCATTTGAGAAGCGAGCAAACAGAACATTTGAGAAGAGAACAAACAGAATCTTGCCCTACACGTTTCCCACG
GGGGCGGGGTGGGGGTGGGGAGTGGGCTTTGCACAGCTTTCCTGCCTATCCCCGAGGGCACAGTAAGGAGACCTGCATTA
GGTCACTTTGCCATTGCATGCCGTAATTATGGACAAAATGCGCTTTGCTCCCTGGGCTTTGCAGCCCGGAGCTCCTTTGG
GCTACAGGACTTGAGGTACGCTTAAGGTTGTACAAGGTCACAGAGATCGTGGTTGATAGGAGATCGCACGAAGGGAACCT
CCCTTTATATTGGAGTTTTAGTTTTAGTTTATTTTCAGAAGGAGAGGCTGTGTTCCTATGAAACAGGGCTCTTAGCCACC
TTCCCAGGAGACCTCAGTGGATGTGTAGTTCAAGCCTATCCTAGGCAGCGACTAGCTTTCCCACTTGGAGTCACTCAGTG
GAAGCATCCTCTAGCTCACAGCATGTGCATGCATGCATACATGTGCATGTATGTGCCGTGTGCACACACACGCACACACA
CACACACACACACACACCCCTACCTTTTGATTGCCCCAGGTCAGAGAACAGGGCTACCTAACTTCCTATTCCTTCCGGCC
ACCCTTGTTCTTTCCCATTTACTCAATGCCAGACTCCTGTGGATGCCCGTGTCACTGCCTCTGGTCTCGTGGCATCATGA
CCACACTATCAGTCTCCACACATTCCCCATGTGTGAAGACTGACTTCTGGGCCGCATCTAGAGATGCCAATAGATGTCAG
ATTGTCTACCAGAAAAGTGGGGCCATTTTTGGCATGTGTGAGGGATGTGGCTTACATTTAGAACCTCCCTGGGTAGCAAA
CTTCTTGAGACTTTAAGGATGGGTTGTTCCCAGCATCATTCGTGCTTTGCCTCCCTCTCAGGTGCTGCTTAAGGAGGGGA
TTCCTTCACTCTGAGAAATTCATCTTGAGTTAGGAAGTCTCCTCACTACCCGATCACCATAAGAGGTGCTTACACAAGCC
TAGGTGGCTTCAAGGTCAGCCACCTGTGGCATCTTATGCAATTGCTGTGGCACTGGCAATCCATCCTCAATGGAAGTGTC
ATCAGGCCTACTGTGCGCAACTGTATCTGAAATGGCCACTTCTCCTTCTCTTTCCCTTCCTCCTCTCTCCCCTAGAAGCT
CTCTTGTGAAGGTCAGGTCATTTTCACGCTCTGCCAGTCTGCATGAGTCCCACAGCCCAAACAGCAAAGCTATTTGGTCT
GGAACACTGGCCCTAGCACAGATCTCAAATCTGGACTGGCTTCAGCCCAGAAACTGAGTTGCTCTGGGGAAGAGCAGGGC
AGGGATATATATGGTGACCAGCTCTGTCCTTGTCTAAATGCTAGGTGCTGGTGACCCTGCCAGCAGAGATGGTCACAGAA
GGGATCTGATGGGACCCGGGTGGGGCTAAATGGGCTGCTGTTGATGCTGTCAATGGTGCTGGAATAGCCAGGGGAGCAGG
TGACTTCCAATGCATCATGGGAGAATGCATATCTGTAGATCAGGAGCCCAGGGAGGAGTTGAGGACTATGAGGAGGCATA
CTCTGGAAGTGGGGCTGATGAAGGAGCCATAGAAGATGGCACCTTCTGGCTTTGGCATTGGTGGATAGAGTGTCCCTAGG
AAGAATAGGTGTTTGTTGAGCCCCCTCGTAACTAGCCGCAAGACTGTTTGTGGTGGTTTGAATAGGCTTGGCCCAGGGAG
TGGCACTATTAGAAGGTGTGATATTGTTGGAAGAAGTGTGTCACTGTGGAGGTGGGCAATGAGACCCTTCTCCTAACCAT
GTGAGAGCCAGTCTTCTCCCTAGCGGTCTTTAGATGAAGACATAGAACTCTCAGCGCCTCCTGTGCCATGCCTGTCTAGA
CACTGCCATGCTACTGCTTCTCTCAGCTCCTTCTGCACCATGCCTGGATGCTGCTATGTTCCTGCCTTGATGATAATGGG
CTGAACCTCTGAACCTGTAAGCCAGCCCCAATTAAATTTCGTCTGTGTAAGAGTTGCCTTGGTCATGGTATCTGTTCACA
GAAGTAAAACCCTAAGATATTTTTCTTTGTCCTTATCTCAGCTGGGTCCCTCTGAGGTTCCTTCCTATCCTGTGCCCTTT
ATCAGTTGGGAGGGAGAAGCTTTCAGTGCTATAGGGATGAGACTTGGAGTCAGATACATGTTAGGCTCTTGGCCAATGAG
AAGACTAGAATCTGGACCATCAGTGAGGCTGATACCGTGGGGGTGGGGTGAGGTCTTTTCTTCAGCTCCCAGGTTGAATG
GCAGAAGGCAGGTTAGCATTAGTGAGGGAGTGGTCTCTAGGTGGGTCACACACAGTGTCACTTGGGTGAGTGATGCTATC
CAGGCTGAACGGGGGAGGGGGGTGTTGGGGGGGTGAGGCCATGGGAGGGTGGCTCAGCAGATGCTGGGATCTTTTCAGTG
GTTGGTGGAGGATGGACTGTGGCAGAGGTAGGAATGGAATGGTGCCCTCTGTCTATAGCTAGGAACTTTCTCAGTTGCTT
CTGGACTGTTATGTGATTCAGACAGAGATGGCCAATGCAAGGCTAAAGGTAGCAAGGGCAGGCTGGGTAGCTGAGTTCCT
GAGTTCGACTCCAGATCTGGCAAAGACAGGAGGATCCCAGGGGCTCACTGTTAGCCGGTCTAGTAAACTCGAGAGCTCTA
GGTTCAGTGTGACTCTTCTGTAGAAGTAAGGAGGTGGGGAGGTGGCTAAACAAGCAAGATCACAGTAGCACACACAGGAA
GGGCTGAGTTCTAATTCCCCCAAACCACATAAAGGTTGGAAATACTCCCACACACTATCGTAATCCCCAGTATGGATATC
ACTTGCTGTGTGCTGTCTTGGGTCCAGGTCCACTGAGAGACCCTGTCTCAAGGGAACAAATCAGAGTGATGGAGTGGGGA
ACCTGGCATCTTTCTCGGGCTTCTACATGCCTCCACAGTATACCTTCATCACATGTGTGTACACACGCATATACCACACA
TGTGCACATGCCAAAAGAAAAAAAAATAGTCCAGCGACTTTAGGGAGGGGTCCTAAATAGCTAGAGGCACGCCCTTTGCT
TCTTTCTCTGCTTTCTAGTTGGAGGAATGAGGATGTGATGGGTGGTACCCAAACAGCTCGTTTGGTCTGTGAGGAGGCAG
CAGAGTGGTTGCTGAGGATGTCTAGGGAGGAAGACAGTTGGCTTTGGATTCTGACCTGAAGCAGGGGGATAGGAGCTTGT
GGGAGGAGCTACTACTGAAATCTGGGGACTGCAGCATGGATCCTGGTGGCTCTCAGAAGCTTGAGTGAGGAGGAATGTTG
CAGCCTGTCTCAGTCAGGGTTTCTATTCCTGTGACAAAATACCATGACCGAAAAAGCAAGTGGAGGTGGAAAGGGTTTTT
ATTCAGCTTACTCTTCTCCATTGCTGGTCATCACTGAAGGAAATCAGGGCAGGGGCCTGGCTACGGGAGCTGATGCAGAG
GCTGTGGAGGGATGCTATTTACTGGCTTGCTCCCTGGCTTGCTTTCAGCTTGCTTTCTTATAGAACCCAGGACCACCAGC
TCAGGGATGGCACCACCCACAATGGTCTGGACCCTTCCCTGTCAATCACTAATTATGAAATGTTCTACTGGCTTACCTAC
AGTCTGATCTTATAGAAGCATTTTCTCAATGGAGATTCCCTCCTCTCAGTCGACTTTAGCGCCTGTCAAGTTGACCTAAA
ACTATCCAGTATGGAGCCATAAACCTAGATTCCAGGGTGCAGAGGGGACAGGAAGATGAGAGGGTACAGTATGGCTTAGT
CTCCAACAGGGGCAGTGATGTGATAGACAAGGGGGAAAGTCATAGCTAAGAAGGCACATTGGGGCCAGGAAACCCCAATG
CAGGCATCTCACAGCCCCTGTGAAAATGGAGTCCTCGTGAGGAACTGGCTGTGGTAGTGGATACTCTGGGAACTGTCTCT
GTGAAAGAGGAGGGTGCAGGGAACTTTGTTCACAGTGGAACCCCATGAGGATGCAGGAGAGAGGTGTATAAAGGGGCCAG
GAGCAGCAACTGTAGGGACCACGATGCAGATGCTGCAAGGAAAGATAGCCTTGTAAGTGGGGGTTGGGGGTTATTCAAAC
AGCAATCCCCAGTAAGGGATCTGGGGTGACTGGGTGGGTACTGTCAGGCCTCTGACATCTGGGAAGCTTCTATGAGATTC
TTCCTCACAACACCCTGGGAGAAATGCTGGTTTCTCAGGACGGCAGGCCAGGGCTTTAAGGGTGACTCTACCGGTCTTCT
GCTAGCATGCTCTGTGAATGCTAGATTGAGTCCAGCCTGTGACACAGTAAGCCTGGCGGATCCTGCACATGAAACGATGT
CCAGTTGATAGCCAGTGATAGTCCCCAGGCTGGAGGAAGCTGGGGTGAGAGGTTCCTTGAGCTCTGAAAGCCTGGTGGCT
TCTGTGGGCTTTCAGAATAAGCGTCCAAGTTCGGGCAGTGGGATGAGTATTCTGGAAACCCCTGGGGACAAGGGGGTGA
AATGCCCTGGTTGGGGCTGAAGCAAGATCATCATGGCTGCCTATTCTGGCTGCTGGGCGGGAAATTCTTGTGGTCCCAGG
GATGGGAAGAAAGTTGGCACGTTCCCTGGCACTTGGATGGTACCTAGGCCTATGGATCCTGCTATGTCCAGTGGCCTGTG
CTGTTGTACAGATAAGGGTGAAGGTGAAGTTTGTGCCTATTTACCTTACAGACCAGAAATCCCAACAAGGAAGAGGGGGG
CAGGAGATAGATAGGTGGATAGATAGATAGATAGATAGATAGATAGATAGATAGATAGATAGACAGACAGACAGAGAAAG
```

```
GGATAGAATCAGAAAGGTAGAGAGATAGAGAGAGGATGGCTGGAGAAACAGAGACAGATGAACTAAAACTGGGGTTTTTA
GGACCTGGATGTAGACAAAGCCATTCATCTCCCACTCCACTGGCTGTTCTGGGTCCTAGGTTCCACCTCCCCACAAGGAT
GGCTGGGAATGGAGCCTAGGGAGGAGGCCAGGCAGGGTGTAGGGAGCACGAGCCCACCTTTTCCCTCATGCCTCTGTGGG
CCCCTACTATGTCTCACAGGTCAGAGACTCTGAGTAAAGGGTAAAGACTATGCTTCTCTCCGCCCCTGGCCTGGCTGCTT
AATAGGTTCTTGCTGGCTGAAGGAAGGGGCGAGGGGAAGATGGAGGAGTGGGGAGGGGCCTGAACTGCATCAGCGAGACT
GGTCAGGGTCGTCCCCACAGACTAAGCTATGATGGCACTGGACCTGTGGCTCCTAACTCTGCCCAGGGACAATTATTCCT
TTTGACTTTACATGTGCTTTAATAAAATGGTTCCATTTATCTGGAGGGAAAGGCTAAGTCCCAGGTCTGCATGCAAAGCC
AAGAGCCGCTCATCACGGCTTCCCGAGAAGCTTCCAGAAGTATTCTATGCTTGGGAAACCTGGCCTGTCAATCTCCCCGT
CACCATGGATATGTCTCTTGCCTTCTACTGTTATTAAAGAATCTTCCAGGGGAGGAGGGGGGTGGGACAAGAGAGAGTCG
TTAGCCATTTTCTGTGGCTGGGCCTCAGTATGTCAGAGAGTTGGAGCTTGCTGGGCCCACAGCTGTGGAGCTTCCTGAGT
GTTGGCAGAGGTAGTGGGTAAGGCCTTCCTTCTGCCTCAGCACAGGTCAAAGGCGTCACATGGCTGGAGGGAGCGCCATG
TTGGCTCCTGTTTCTCTTCCACATCAGTGCCATTGTAGGAAGCCACCTTCATGCCTTCACCCCCACCCTAATGGCCTCTG
AGATTTCTGCTCTCACAGACAATCAATATAAGAATTTAGGGGGCTTGGATTTCTAATGCATGAACATTTAGGGGCACATT
CACGCCCTGTCAAGGAGAACTGGCTTTTATCCCAAGTCTTCATGGATACCTTCCTGTGTGTCAGTCCTCACCCCAGGCTC
AGGCAAGAACTGATAAGACCCTATTCCAGTCTGTCTATGAGGTTCTCCCACCCTACCCCAGACAGCTCCTCTGCAGGAAG
TCTGGAGCCCTGATATAGCTATAAGGATGCGGCCATGCGGATGCCATGGGTCCAGGGATGAGTCTGTAGCTTGGTGGGCT
GATAGGAGGTGGGAAGTAGTTGGAGGAGGTAGGTCTCTGGGAACATGCCTTGGACACATATCCTGTCCTCAGCCTTCTCC
CCTTTCCCCACTTCCTGCTTGACAAGTCAGGGAGCAGCTTTGTTCTCTTATCATGGTGTTTCACTGTGGCAGATCTAAGA
CACCAGGGGCCGGGCCACCCAAAAGCCACAAGAAATCTTTCCTCCTTTAAGCTGATTTTGCCTGGGAATTTTGTCACCTC
AATGAAACTAAAATAGTAATCCCATTTTACAGATTTGTGAACTGAAGTTCAGCAAGTCCAGGTCACACAGCCGGGGTGAC
TCTCCCTAGCAATGGCAGCCCACATACCTCCTTTCCTGGCTTGTCTCAATTCTCTTTTTAGGGTTCTTGATTTCACTCTC
CAGCATCCCCAGAGTCCTGGGGATTTTGTGTGTGTGCATACGTCCCTCCATATCCCAGACAGGGCACCAACGACAGAGCA
AGAAACAGCTCCACTGAAGTCTAGCTTGGTGAGCTAGTGAGTTTCTTGTGCACATTTATGGAGTAAGGGGTTGCTTACAT
CAGTTTGGGTGACCACCCCCCCAAGCAACTATGTTACTGGGAAGTCACAGCTGAGCATGGATGATGGCATCCCCATAGTT
GTATAGATGGAGTCACTTCTTCTTTGGCTAACCTATCCTCTCTAGTGTGTAAGTACCCCTGAGTTCAGTTACACATCGCT
AAAGCTGGACCTCAGGGGAGGGGTTCCTGTTACTTCTCTTCCCTTCTTGTGAGGGGATGAACATCAGCAGGTCCAATCGT
GTGGCTCTCTTGTGGCAGTTACAGCTGCCCCGATGAAGATGGTAATGGCTGTTATGTCCAGAGAACAGAATTCTTACAAC
TCCCTTCTTTTCACAAGAGGGCAGAAGCTTGTCATATTTCCCCAAGCCTGGCATTAAAACCAAGATGAAGGGGAGATAGA
AACGGGGAGTCCCTGGTCCTGTGATACAATATGTAAATAAACACCCATTCATTTACGGAGAAATTCGTGACTTCATTGTT
AAAAATAAAGCCGTGAACTGAACCCTGCCACCCTATCTCACCGAGAGAGGAAGGATTAGAACTTACAACTCGGAAAGGA
AGAAAGTGATAGGAGGGCCACTCATTGGCCATCAAACTGCTTATCAAGCCTTAAAACCAGTCAGTCACCTTTGAATGCTA
GCCTAAATAAAGGGCATCTCAGAGACTGAAGTAAAGGGCAGCATGTAGCGGGGGCTGGGGGATGGCTAAAATGGAGATCA
GCAAAGAGGACACCTAGACTCTAGGTCCTAGAGGGATGATGTGTCTGTGGGTGGCTTTGGTGTTAGGGTGTGAGCATATG
TATGTGTGTGTGTGTGTGTGTGTGTGTGTGTGTGCATGCGCGTGTGTGCTCATGCGTGAGCAGAAGTCGA
TGGTGTTAGGGTGGCATGTGTGAGATGTAGCGGGGAAGAGTAGGCAAAGAAGGGCCTGCCCCCTCAGGTTCTGTGCATAG
CTAGGTCCCTGTACCACAGTAACTGTGTGAGCGTGTGTGCCACCACATTCAGTGGCTGCCCCTGGGCGTAGGAGGAGATC
TTATCAGTGCTAGGTGTGTGTGAGTTTCAGGCTACCTCATCACCCCCCATCTCTCCACCTCCCATTGGGCCTTGCCCCT
CATCCTCTATACAGGAAGTGGGCTCGCCTTGGGTTTCAGTCTGTTGCTGGCCTAGCTGTTGTCTGCTACCTAAGCTGCGG
AGCAGGAGATGGCCGTGGACCCAACCTCCAGCAGGACTGCAGGGAACATCTCGACACAACTCCATGTTGCAGCCAGGTGA
TGCCCCTGTGTGGTCCCCTGGGAGCCCTGGGCACCCCACTACCTCTTGCTGGTCCTCCCAAGGGACAGGGAGCTGGGAGG
GGGCGCTCATCTCTAACTTGGTTCCCTTTGTAGTTTGTATCTGGGCTTTGATGATTAACCTTGTCTTAGGATTCCCTGGG
GAAGGGGGTAGCCTGGCTGTCATGGTTTCTCCTTTAAGAATAAACCCCTTCCAATGTCAGGGCACAGTGGCTTCTTCCCA
GTCTACTCTGTGGGAAGCTGTCTGCCATTTTCGCTAGGAGGGACTTCCCTGGATCCTGTGTGGGGATGTCTTAGCCCAGA
AGGGTGGAGGAAAAGATTTGTAGCTCGTCAGTATTGGGAGGTGGCATGGCGTGTATGGGACAGGTGGGATTCAACGCTGA
CGCCTGTGGAGTTCATCTCAGCCTGGAGAATTCTCCTTAGTGAGAAAGAGAGAAGGAATCTCCCCCTGGGACCTGGCTGT
CCAGTCAGCTATGTTGATTGACAGGTTTCTGAGGGCCCTAGGCTGGAGGTCAGAGGTAGAGTTCTAGCTTAGATTTGTCT
CTGACATTACCGAGTGGCTAGAGAGGTTAAGGACATCGCCAGGCATGGGGCTCCGTCTCCGAGGCTGCCTGAGGTAGGAG
CTTCCCACCGTTAGCTGTGTTTACAGTACTTCAGGGCCAAGGTGGGTGGCTGAGGAAGCAGAACTACCTTTGAAGGTCC
CCTTTCTTTCACACACTCCTCAGCTGAACTGAACAGCAAGAGGTGAACAGTGGTGTGTGTGTGTGTGTGTGTGTGTGT
GTGTCTTTGTGAATGTGTGTGTCTCTGGAGAGGTGGCCAGAGAAAGGATCTCTGAGGTCAAAGGCCACCAGATCTGACAG
ACAAGCTCTTGCTTGCCGTCCGTCCTCCCTCAGTCGACCCTGTACCTCACAGACCTGTGAGGCCTTGGACTGCCCCAGCA
CTGGAACCTTTATCCTGGGAAACAGGCTGCTTTAGGCCCTGATCTTCCCATAGGGAAGGAGCAGTCAGTCACAGAACCTG
CTCTTGTGCTGTGAGACCTCTCTGTGACATGTGTGGGTCAGTCTGACGCAGGGAGTGTGACACGACTGTCACTCATGGCT
GACCCTCCTCCTCCGTCAGCATCACCACTGTACTGGAAATCAGGTGACCCAGTTAGAGCAGCCTGGGTCTGTGGGATGAC
AGACCTTTTCAGGGCCTCAGCGGAGACATATATGAGTCAGGGAGATGAACGTCTGGTGGTTGCGAAGGGCATACGTGGGT
GAGCCTCAGGGGGACAGTGCCTGGGACTCTGAGATTCTTGGGCTGTACTCACTCTGCCCGAGGCTGGCCTTGTGCTGTGT
GGTTTTGGAGTGGTTTTCTTAGTCTCCCAGAGATTCAGTTTCCCTCTTGCCGAAGGAAGCTGCAACCACCATGACTGCTT
GAAGCTACCCTGCCCCCCAGCCTGACCCTTCCAGAACCTTCCATATGCTCTTCCGTCTAACCCTGTCCATGATGGGTGGA
TGCTTTTGTGCCTCTTCAGCCAGGGGTGGGGAGCAGGTTTGAGTGCCTTTTCTTAGGCATTCTCTGTTTTTGGCTGTGGC
TTGTGGCCCGGCTATAATCTTGCCTCTTTCATTCCTCTTCCTCTCCCCCAGGGTTATGCCCTCCCTCACCCCAACCCCAC
GCCATCATGAGGCTGCTCAGATGTCTGGGTCCGACCTGGCTTGCTGCCTTATTGAATGAACACGTAGAGTGCTTCAGATC
```

```
CTGTTGGGCTCACAAGGGGCTGTGGAAGGCCCCTTGCTTGGGCTGAACCTGTTAGGGCTCCCTTTCTACCCAAGCACCTG
CGGGGAGTTGATCTTCCAGGGAAGGGAGGTCTGGGGTGGAACCCCAGTTCCGTAGACTTGGTCCTGGGATCCTTGGTCTC
CTTGTCTGCCTAGCAAAGACACCAAATAGCTGCTGAGCTGCCAGGCAGCATAGAGTAGCTGGAAGTATGAGGGCCTGGGC
ACAAGGCCTGGGCAGTGTTTGATGAACATACCCGGCAAACACTGTCACCGGGCCGCCACCCATCACTTGCTGTCCCACAA
ACCCTTCAGTGCATGACATTTATTGAGGGCCTCCTGTGTTCCGAGGTCAGTGGTTTGGAGTGGGCACACTGCCTTGGCAG
TGTGGTAGGTGACAAGCCAGAGGGATGGAAGGCAACCCAAGGTAGATTGTCCTTCCTTCATGGGGGAGACCAGGGCGAGT
GCCACAGAACCAGCAGAAAAGAGAATGAGAAATGGGATACCAGGAGCCTTGGGTGCAATGTGGGAAGGACAAGTCCAGGG
AGGCTTTTAGAGGTCCAGACCAAAGCCACCAGCTTATGTGGACTGCAAGAACCACCTAAGGCAGGCCACGGTCTTGCCTT
CCATGCTCCTCAGGGCTGAAGTGGGTGCTCTTGGTAGCCCTGTGATAGGTATTCATTTGGAGATCAGAGACCCAGGAATG
AATGGAATCCAAATGTCCTTGTCCTGATAGACTGTCTATGACTCATATACATAGGTACGTGAATGCATGCGTGTATGTGT
GTTTTCAAACTGAACACTGTTTTAAGTCATGTGCAAAAGTCTTGAGCTGGTCTCTATCCTGCTTGGGGCCTGAGCGATTC
CTTTTCCCAGAACAGCTATGATGTGGATGCTTCTCGCCTATTAGTCACCCAGGGACTTGGCTGTCCAATCACAGAGCTGA
GTCCCTGTCACCTCACCTCACCTGGGTACATGGGGTGGGCATCTGCCAAAGATGCTTTGGGTGGGAGACACCTCATTCAC
ACCGTGCTCCGGAGAGCCGGTGGCTGCCCCTAGGAACTCCTTGCATGACCACCTACTGCCACCAGTTTCTTGTGATGGGT
TGGTCATTTATAGTGAAAACAGAGTCCGTGTAAGGACTGGTTTGGTCTGACATTTTAGGCACCTCTGGAGGAAGGAAGGG
AGCTCAGAGGGAGAGAGAGCCTGGGCCTGCTTTGCTCTGGCTGGGCGGAGAGAGGGTAGAGTGGGGTGGGGTTTCTGACT
TGTTCTTTCCTTCTCCACTTAGGGTTTGCATCCTCAGCTCCTCTCAGCTGTGATGGCTACCATAGCTCTTCCCTGGAGCC
TGTCCCTCTACGTCTTCCTCCTGCTCCTGGCTACACCTTGGGCATCTGCAGCAGTGAAAAGTGAGCATCTGGCATTTTGA
TGTCCATACTTTCCCCAGTGGGAGGTCACATCCCAGATTCCTGCTTCAGGAGGAGATCCATCTAGCCTGGGAGGCAAGG
TGAAGAGGGACAAGGCAGTGGGACCCAAGGGCAGGTATCCATCTTCCTGAGCTTGCACAGGCTTCTCCCAGACACATGCA
GAGGCTGCTGATGGGGCCTGCTTGGCCATGGATAGGCGCTCTGTGTCTTCCCTTTGAAATGGGTCTGGATTGGGGTTGTG
ACAGGTGGCTCAGTGGGCAGAGTGCCTGGCACAGGCGTACAAGCATGAGAACTGGCGTCCAGAATTCCAACACTCACATA
AAAACAAGTCAGGAATGGGCTGGCAAAGTGGCTCACTGGGCAAAGTGTTTGCCCCACAGACTTGACAACCTGAGTTCAAT
TTCCCAGATGCTGTGGTGGAAGGAGAGAGTTGATCTCTAAAAGTTGTCCTCTGGCCTCTATACACATACCATGACCACCT
ACTGCCACCAGTTTCTTGTGATGGGTTGGTCATTTATAGTGAAAACAGAGTCTGTGGCGTGTGTATCTACATACATGCAC
ATGAACACAAACACACACACACACACACACACACACACACATGGCAATGAAATGAACTCGAATTCCTTTTCT
TCCCAGCTGAGTCCCCTACCTCTTCCTGGGGCAGGCACAGGGTGCCAAGCGCAGGGTAGTGGACATATCCAGAGGCCATG
TATGTCTCGTAGCCAGCCACTCACACCCCACTCTGCCCACAGACTGTTCCCATCTTGAATGCTTCTACAACTCAAGAGCC
AATGTCTCTTGCATGTGGAGCCATGAAGAGGCTCTGAATGTCACAACCTGCCACGTCCATGCCAAGTCGAACCTGCGGTG
AGTGTAGCCTGGTCTCGCCAGGACCTCAGGGCTGTGGGACCTCCAAGCTGTGGAATCCTGCCTGGGCTTGAGATCCACAG
TGGTTAGCACCAGGCTCAGACACTCAGTCATGTGGGCCCCCAAAAGTGGCTGTGATCCAGGCTTTAGTTGAGAGCTAGCA
AGGTCCTGGACCAGGTAGTTTGGGGACAGTGACTGTGAGCCTTTCATGCAAGGTTAGACTTCATAAAAAGGTAATCAGGG
TACTTAGTGCCTGATATATTATATATATTATACATATATAATATATATAATTAATATATATATTAATCTGGTATTTGGGA
GACCCCACAGGCAGCTGTGGGGGTTACATTGCCTGGGGAAGTATCTGGGGAGCCATAGGCTTTGTCAGGCTGACTTTAGCT
GCCTGGAGGTAAGGGCTCTCGGGCAGGAGTGGGGCTGAATTTCTTGGTCACTCTGGCTGCTGGGAGGCCAGGCTGGGGTG
GGGAGCTGGCTGCTTGCCTACTGTGCTGCTGTCTCTGGCACAATGGAGCCTGAGAAGGGTCCTTGGATGGCTCTGTGGTC
CTGACTTGGTTTCTGATGTGACTTTTCTGTTCTGCAAGACACTGGAACAAAACCTGTGAGCTAACTCTTGTGAGGCAGGC
ATCCTGGGCCTGCAACCTGATCCTCGGGTCGTTCCCAGAGGTAAGTAGCCTGAGTAGCAGTGTGTGTGCATGTGTGTGTG
TGTGTGCGTGTGCGTGTGCGTGTGCGTGTGTGTGTGTGGTCAAGTCCTTCCAGCTGTGGGCTGAGGCCTCGAGGTGGGCT
TGGACCTCTGCAAGCATCAACCTGGTAATGCAGACCAGGCCATCTGGGCTGAGAGGGAGGAACAGGAAGTTGGATTTTGA
GGTGTAGGGTCTGTATCCGTGCTGCCCCTTCATGAGCTGTCATCCCCACTCTCCTTGTGAGTCTTGCCACCCTTTTGCAT
CCCAGACAACGGCAATCACGCCAGACTCCGGCTGAGGTCTCAGCATCTAGGGGGTAGTGGCATGCTATCGCTGAGTGTCC
CGGCCCTGGTGCTACCTCACAACAGCTCAGTGCCTCTGGGCAGCTTCAGGGCCTCCGTTTCCCCTCCATAAAATAGGAA
AGAAAATGGAAGCTCCCTCAAAGGGTTGTTCTTGGACAGAGGAGATGCTGAGAGTCCTACGTAGACCCTGATGATTTTGT
CTCCTTGCCAAGGAAACATCTAAGTCTCAGAGAGATGTGGTAACTGCCAAGGTCACCCAGCCATCAACGGCTGAGCTGGG
GTTGACCCCAGACCTATGGGCTTCTGGTGGCTTTTCCATATTGTTCAGCTGCCGCTTGCATATTCTCAGGTTCAGACCAT
GTGGATGTAACTTCATTTTTTTCCTCAAACCCTCTGGGCAGGGCAAGGGCAGGGCAGTGCCAGGGTTTGCCTGGGGAGTC
CAGGCTGTGTTGGCAGGTTCACATTTTTTCTCAGAGAAAGAGAATGGGAGATACACAACACAGCCGGTAAGTCTCCAGCT
ATACCAATCTGGGAGCTTTGCAGGAAACTGGCCTCCATCCTGGTCTGATTAGTTCTTAGCTGTGTGGCCTTAGGCATGCC
CCTAACCCTCTCTGTGTTTCAGTCCCCTGGGATAACTGATGGGAATGGCAATAGTATATGTCTGTCTCAGGGGGTGGTGA
GAGCTCAGTGAGGTAATTTATACAGAGAAGAGGGGACAGAAGTTCCCAGCAGCCTCATACTGGCGATACCTTCAGACCAA
CCATAACAATAATAATCAAGTTGTGCTACGATTAAGTGGGTGAACCTGAGCTGGTAGCAGCTGTGAGAGATGAAGGCCGT
GCCCTCCATGAAGTGAGAGGATGCAGTACCCAGCTGATTGGTTGGTCAGGGAACTCTAATGGTCAGGCTCAAGGCTGGTG
CAGGTAGGAAGGGCCAGGCCTCAAGGCTCCGACTGCAAACCACCACCTAGTCCCAGAGCATAGGTCCTACCATGGCCTGC
CACTGATTCTCCTCCTGCCGCCTACTTGGAGGTAGGGCGGGAGGGGGGACAGCCATTCATCTCTAAGCTTTGGTCATTCT
GAGGAGCCATGGCTGCAATGGCCTTGTGTGTCATGGGTCTCAGAGGGGAGGAACTACTGCGCTCATCAGCAAGCTGTGAG
CAGCGGCTGCCTGGGGCACACTCAGCAGCTAGCCATCACCTTCCAGAAGAGTCAGGGTTATTGAACAGGTCTAACACTGC
CTTCACTATGGCTGTCCTTCTTGGGGGCTTGGCTACTTGTATTTTTCTTGTAATGGGGAGCTTCCTTCTAATCCTGGTGA
CCTACTTCAGTTTATATCAGGGATTATTGAATGACTGCTCTCTGTGCCAAACTTAGCTGCTGCCTGTGTTTGTAAATAAA
GTTTTATTGGAACATGGCCCTTGAGTTGTGACAGAGACCGTGTGTGTGTGTGTTTGTGTGTGTGTGTGTGTGTGTGTGTG
TGTGTAGCTGTGGAGCTAAAAACACAACCTGGCTGGTTAGAGACATTGGCTACCCCTTGCCTTAGATAGCTTGGTAGAGG
```

CAGAACATGTCCTTACCTTGACATTTTAACGGTTCCCCTTTCTCAATGACCTCTGCTCTCCTCCCTTGCAGTCCCAGTCA
CTGACCTCCGTGGACCTCCTTGACATAAATGTGGTGTGCTGGGAAGAGAAGGGTTGGCGTAGGGTAAAGACCTGCGACTT
CCATCCCTTTGACAACCGTGAGTATGGAACCTGGTGTGTGGGTCTGAATCTCTGAGTCTGTCCCTGCGCCTACCTGAAAA
GCTCCCCCCAGCTCTGACCTTCCCACCCAACCCCACCCCTGCCTGGAAGATTCTTACTTCAGCTCTCGCCTATTTGTGGG
TCTGTCTCTCTGCTGGGCTGAGAGCTTCCTGAGGGTCTCTGCTGGCCCTGAGGCTCCTCTCAGGAACACTCAACTCTAGG
TCTGAACAGGCAGGAAGCTCTTGTTGAATTGCGGAGTGTTTTTCAACCTCTAGAGAAGAGAAGGTGCAGAGGGGGAGATCC
AGGCAAAATTCAAATTGCAGCCCTGCCTTGATGCATCAGGCTGGCAGATCACATCTGGGACCTTAGTGTTTTCTTTGTAG
AATGGGGATAATTGAGGTCCTTGTGACAGGAAGGAATGTTGAGGTGCATAAACTGCTCAGCATTCAAGACATAGGGAAAA
AAAAACTAGTGCTCTGTGGGCACACAGAATCTCAAGGTTACATGTAGAGCACTTGCCAAGCTGTGCCAACTCACTCTCAC
AAATATTAATAAACAAGTATATTGAATGAGTGTGTAAATAGTATAATTGAGAAATGAATCATAGCTCTTTATAATTTATA
TTTACATTTAATATGTATACTGATGGGATACACGGATATAAATTTATATGAGAGGCTATGTAATTATAGCTACATGTTCT
AATTATTTAAATTTATGTCTTATTTAAATGTATTCATATAAATACAAATACATCAATAATACAGATACATGGATAAAATA
AGGTACATGAGCTGGTGCCTGCACAACTCTCAGATCAGTTTTCGAGCAGAACAAGGCTGCCTGCCAATCACTCAGCAGTT
GATTAGTCATTCCTGAAGAGTGCCTTGGGTACCAAAAGCTCTATTGGCACGGCACAGCCCATACTCAGGCATCCTGGGAG
GAGCATGGGCACAGAAGGATCCCCTATAGATTTACTTTTTCGGGTGTAGGCAGAGCAACAAGGGCAAGGGGTGTAAGGGG
TGACACATGGCCGGGGAAGGCTGGGCCATTTGAGCACAGGAATGAGGAGGAGTGGGTGCCGAGGAGGAGGAACAGGGGCC
AGGATGGAGAGAAGAAGGTTCAAACTGTGGTAGAGTGGGCTCAGAGGAAGGGGAAGTAGGTAGGTATAGAGGAGGAAATA
GAACAGGTTCACAGTGGGATGTGAGGAAGGGAAGAAAGGGGGCACATGTGGAAGAGGGGGACAGCTGTTGCAGAGGAGGG
GGAAGAGCAGGTGCATAGGAGAAGGGGCAGCAGGTGCAGAGGAGGAGGGACAGCAGGTGCAGAGGAGGAGGGTCAGCAGG
TGCAGAGAAGGAGGGACAGCAGGTGCAGAGAACGAGGGACAGCAGGTGCAGAAGAATGATAGCAGGTGCAGAGGAATGAT
AGCAGGTGTAGAGGAGGAACAGGTGCAGAAAAGGAGGAACAGCAGGTGCAGAGGAGAAGAAGCAGGTGCAAAGAAGGAGT
TGGTGCTGGGGGTGTTGTGTGGTGGAAACTGGTTTGCAAGTTCTTGGTATCATACCGATTGGTCCATGATATCTGCTCCC
TCCTGTCTTCTGTCTGCAGTTCGCCTGGTGGCCCCTCATTCCCTCCAAGTTCTGCACATTGATACCCAGAGATGTAACAT
AAGCTGGAAGGTCTCCCAGGTCTCTCACTACATTGAACCATACTTGGAATTTGAGGCCCGTAGACGTCTTCTGGGCCACA
GCTGGGAGGTGAGGAATGGCCAAGCCTCTGCCTCTACACCTTCCTGTTCTGGTGTCTACCCTTTTCTGTTTCTACATGCT
GGTGTGACACTGATTCAGCCTGGTCCTCCGACATGGCTTCTCAGTGCCTGCTTGGCTCATGCTTTTACTGCTTGGGGTCA
CATCTGGCTCCATATCTATGTAGTCCCTCGAGTTAAGGCGGCCTCTGAGACTTGGCAGCCACTTTTCCACATTCTAATTG
CAGCGTGTTCCCTGGCTGCAAGGGATGGGGTGGTGGTGGTGGTGAGGAATGACGGGGACTGAGGCTTGCCATCTACACAC
TTGTCCCTTACTTGCCTGGTGACCCTGGGACTCAGGGCCCCAGCAGCCAGTGAGTCAGGGAGCCTTTGGGAAAGCTGGGG
AAGAGGCTGTTTGCTTAGCCTCTTGACCTCGTTGCGGGAGGCGAGGTGGAAAGTGGAGCTGAGCTGGTACCCATCTCTGC
CCAGGATGCATCCGTATTAAGCCTCAAGCAGAGACAGCAGTGGCTCTTCTTGGAGATGCTGATCCCTAGTACCTCATATG
AGGTCCAGGTGAGGGTCAAAGCTCAACGAAACAATACCGGGACCTGGAGTCCCTGGAGCCAGCCCCTGACCTTTCGGACA
AGGCCAGCAGGTACAGTGGGTGGCACAGGTTGGGGGTGGGAGTGGGAGAAGCTGGGTAAGGGGAAGGTGCTCTGGGAGCT
GCAAATGTGGGTCAGCCTGCTTCCCCCTCATCTGCCAGCACCTCCCGTGGGCTCACTTACCAGAGACAGGGATGCTCAAG
ACATCAGTCACGGCCATAGATGGTAGGAAGATTCTCCTTGTATTAGCTCTAAATTCTTAGAATTCAGGGTCGGGGGCTCC
TTTTTCCCAGCCTTCCCTGCAGGACAGGTGCTGTCCTAGGCAAGGTAGCTCTACCAGAAACAAGGTAGGCATAGCCTCTC
AGTGTGGATACTGTGGAGCTGTCATGGTGGGGACTCTTGAGGCTCCATCCCTCATCATGTCACTTGCCAAGTTCAAGGGA
CATGGACGCTCTAGGTGTGGTGTGATGCTCAAAGGCTGCAGATGGAGAAAAGGTCTCAGTGAGTGAGGTAGGGAGTCAGA
TCAGCCATGGGCTCTGCTGGGAGTGCCACAGGGACATAGCCTTTAAGAACAGCCTTGCCTTTCTGGAAAGCTGGGCTGGA
GCAGGACTGAGGATGGAGCCCCCAGCAAAAGCAAGCCTGGCTAGACACTGGTACCTGGCAGAGTGCGATTGTTGTCCAAG
ATGTGTGTGGCCTTCTGAGGGGCCATGCCTCAACTTCCACTTCTCATACCCCGGACAGATCCCATGAAGGAGATCCTCCC
CATGTCATGGCTCAGATACCTTCTGCTGGTCCTTGGTTGTTTTTCTGGCTTCTTCTCCTGCGTCTACATTTTGGTCAAGT
GCCGGTACCTTGGGCCATGGTAAGGAGACGCCTACCCAGTCTTCCTTTCCCACCCAGTCTGTCTTCAGCATGCACATGTT
TCCAGGTTGGCCTGGGCTGCATCCTGTTCCCTCGTATCTGCCCTCTCTGTCTCCCTTCTCAGCAGCTTCCTTGTCCCT
GTGCCTCATCCCACAGGTAGAAGCTGGTGTGGGAAAGGGAGGCAAAGCTCACCTGGAAGAGTCCCCCGGGCACCCCTGAA
GCCCACTGGAGTCCCTGTCTTTCCTGTTGCTCTTGGTCCCCTCCTGTCCATTCTGACTGTGTCACACTCTTGTCTAACCC
TCCTTAGAGATTCCCCACCACACCAGTCATACATCCTGACCCTTGAGGGCTTCCACTGGAAACTGTGTGCCAATCAGAAA
GTGCCTCCCCTTTCTCCGGTTAGATATGTCCACGTTCTGGGTAGCTTAGTGGTAGACCAAGTAGATCAGTAGATCGAGTA
GATTGTGATGTAGGGATTCAACTTCAAACTGCCATTGTGAAATGCACAGCGTCGCCATTCCCCAGGGGAGTGTTCACCAG
GAGGTCTCTCCTGCCTCCCTCTCTAGCCTGTGTAGTCCCTCCCACTCTACAGCCATCTTTTGACTAGCACACAGGTCT
GTGTCCACAGACTGGGCAATCCTATTACATGTTTGGTTCAGGTCTGTCTTCATGTCAGCATGTCAGCTCCGGGGGAGCAG
AGGCCTTCTTTGCTGTCCACTCATGTGTCCCCAGCACCCTGTGCAGTGTTCTCTGTTGGTGACTGCTCAGTCAACATCAT
TGAAGGAGCGAATGCAGGATGGGCCCATCCCCTACTCTAGGGGCTGTGGGCTGCTGGTGGGACCTCATGACACCCACTA
CCTGGTCTCACTCTTCACCCCTCCCCTCTTCCTTGGCCAGGCTGAAGACAGTTCTCAAGTGCCACATCCCAGATCCTTC
TGAGTTCTTCTCCCAGCTGAGCTCCCAGCATGGGGGAGACCTTCAGGTAGGAACCAGGGGTAGAAAACAGAGGGATGGTT
GATGGACAGCCCAAGAGCAGGCTGCAGGGGAGAGATGTGATAGGGTGACAATGGCTTTGCCTTCTGTGAGGAGGCTACAG
GATGTGAGGCAAGCAGTTTCCCCATGGACAACAGCTCTGTCTCCGAACTGCCTGGGAGCCTTCTGTCTAAGGCGTGTGGC
CTTGGCTCTAATCCTGGCTCTGCCACTGCCTGCCTCTGTAAACTTGGAGGAGTTACAGATCTTCCCAGTGCCTCAGTTTC
CACCCTTATACGGCCCTGGGACTGTTGCTGGGTGCAGTACGGGATTCAGGGAGCTGCAGAGATCCCTGCCTCTGTGAACA
GAGCAATTCCAGGCAGATCCAGTGAGCAGAGTGATTCCTGGCAGGCCTAGTGATTCATCACAGGAATGCAAGACCTCAGG
CCATCTGGACTCACAAAACCTGGGCTTGTTTTCAAACTGTGGCCCACACAGTCCTATCTTCTGTCCCCCTAGTCTACCTG

```
CCTCAGGCTAGATGCTTCTTGAGGCAAGAAGTGGATAGAGGGTAGAGGAAATCTACTGTTTCTACCCCCGACCCCACTGG
GTTTCCATGATGGGAAGCGTGTGGGGGGCAGAGTGACCTGTGGCACTATGCTGAGAATGGGAGGCCCACAGGCCTGCTGG
ACTGGCCTGTGACCATTAGGGCTGAGCAGGGCCCTTCTAGAATATCTGAGAGGCTCCCAGAATTGAGGATGTCTTATTAT
GATACTTGATGTGTGTGTGTGTGTGTGTGTGTGTGTTGTGTGCACACATTCCTGCATGCTTGAGTGTGTGCATGTAAT
GGGGTGAGCAGCTCCATAAAAAGTTTGTCCAGACTGTGTTATGGGGGTGCACACCTTTGATCCCAGCACTCGGGAGGCAG
AGGCAAGTGGATCTCTGTGAGTTCGAGGACAGCCTGGTCTACAGAACTAGTTCTAGGATAGTCAAGGCTATACAGAGAAA
CCCTGTCGCAGAAAACCAAACCAAACCAAACAAAAAAGCTCAAACCAAATCAAAAGAAACAAACAATCAAAAGACCAAAA
TAACTCTGTTTTTCCTGAAATCCCTTATGCCTTTGATCGTGGTAAGATCCTGACCAACAGACAAATGCAAACAAACCTTG
ACCTTGACTGGCTATGGGTCAGCAAGGATTCATGTCTTAGCCTCTTTGGGTTGTGGCCAGGGCTCTGGGTCATGAATCTC
ACTCACACACGAAGGTTAGCCTGAGGGGTATATGCAGGCCATGACAGGGAGGGGCGAGCTGGGGATCCACCAGTTCTT
GACCACTGTGTCCCTTGTCCTTGCTACACCAGCTGCCTGTGACTTGAGCAGGCTAGACCATCAAGGACATTGGTCAGCTC
ATTGACTCTGGTCAAGACAGTTGCGATTGTCCTTCTGTGTTCGCACACCTAGTTCAGTCCAGGTACACAGCAGGTACTCA
GTAGCCAGCCACATTCACCCCTAGCCAACATCCCCCAGACACAGGTGAGCCAGCACTGAGCCTGTGTTCATCTAGGATCT
GACCTAGGGTGACAAGTTGAGAGAGTCCACCAAGACAGCAAGGCTACAAGATGATATCAGGTTGGGGGCAGGGAGGAGGC
GGTCCCGGGTGGTCCTGAAGGATGGAGAACAGGTGGGAGGGAAGGGAGAGGAGGAGTTGAGTTCAGTGGTGGGGAGGACG
TTGAACGACCCACTTACTCTCCTCACTTTCTCTGGCAGAAATGGCTCTCCTCGCCTGTCCCCTTGTCCTTCTTCAGCCCC
AGTGGCCCTGCCCCTGAGATCTCTCCGCTGGAAGTGCTCGACGGAGATTCCAAGGCCGTGCAGCTGCTCCTGTTACAGAA
GGACTCTGCCCCTTTACCCTCGCCCAGCGGCCACTCACAGGCCAGCTGCTTCACCAACCAGGGCTACTTCTTCTTCCATC
TGCCCAATGCCTTGGAGATCGAATCCTGCCAGGTGTACTTCACCTATGACCCCTGTGTGGAAGAGGAGGTGGAGGAGGAT
GGGTCAAGGCTGCCCGAGGGATCTCCCCACCCACCTCTGCTGCCTCTGGCTGGAGAACAGGATGACTACTGTGCCTTCCC
GCCCAGGGATGACCTGCTGCTCTTCTCCCCGAGCCTCAGCACCCCCAACACTGCCTATGGGGGCAGCAGAGCCCCTGAAG
AAAGATCTCCACTCTCCCTGCATGAGGGACTTCCCTCCCTAGCATCCCGTGACCTGATGGGCTTACAGCGCCCTCTGGAG
CGGATGCCGGAAGGTGATGGAGAGGGGCTGTCTGCCAATAGCTCTGGGGAGCAGGCCAGTGTCCCAGAAGGCAACCTTCA
TGGGCAAGATCAGGACAGAGGCCAGGGCCCCATCCTGACCCTGAACACCGATGCCTATCTGTCTCTTCAAGAACTACAGG
CCCAAGATTCAGTCCACCTAATATAGCAGGTGGCCAGGACTGGGATCCAGCTGCCTGGATCAGGTCAGGCTTGAGGAAGA
CTGCTTAAGAGGTCTCTTGAGGACAGTCCACTGCTGAGGACTGTCCTGGCTATCCCTGCCCCCCCCTCCAAACTTAATC
ATCCACTTCTGAACTCCATTTGCTACTTCCTGGTCTAACCAGGGTTTGGTGGAGGGTGGGGAGTGGGGGAGCGGTGGTCA
GCTCCACTGCCCTATTTAGTCATGAGGTCACTAGCTTCCTGTACCTGCCTCCTTCCCTCCTGCTTGCCTTCACAGGGCAG
CTAGAACTTGCTTCCCCCAATACAAACATAACCTTGCTCCTCATTTATCCCTGAGCTTGCTGGGGCTCCTTACTTCCCTC
TGCCCCATTTCCTGTCTCAACTCTTGCCCTGCCAGCACGGCTGTGGAACAGCTGTCCTCAGGTTGTGATGTGGCTGTACA
CTGTCTTAAAATCAGAGCAGCTTTGACAACCCAAACGAAGAGGTCCTGATAAGACTGAATGCAGCCTGTTGACTATGGAG
GGTCCCTCGTAGAAGGAGGGGTTGGGAAGGGTCATCAGTCATCACCTGAGATCGTAGCCACTCTCCTGCACCCCATCCTC
AGCTATTCTGCTCCTGGAGCATGAGTTATTTTATGTGTTCTTGGGCGATGCTGGTCTATGTAAGGGGTGGGGGCGCGGGG
TTGATTGAAGTGAGGGCTCCTTCTATGTTGCTTTGGGGACCTTGTCTATTCTGGGGTGGGTTTTTCTGTGAAGCAGCTTC
TTTGGGGTAACCTAGCCCCCTTGTCAGTAAGCCTAAGAGAGTCAAAGGACCACCGAGCTGGACTTGAGTGTACGCCTACT
TTTGATGCAACATCTGGGAAAAGTACTTTTTTTGTTAATGTATCCTTGAAGCTAAGAAAAGCTTACACCACAAACATTGT
GTCAGCACTGTCCTTTGTGGAGATCTCACGAGGATCCTCTGGGGTCTCCTGGAGTCATGATGTGTCATTCCCTATCCCCT
ACCCCACCCCACCCCACTCCCAACCCCCTCCTTTGCAGGCACTGTTCATTGTGCTCCGCCTAGGTATCAAGCTTCTCCAT
CATCTGTAAGTCCCGCCTACCTAGAGGGAGGGGCTTCTGTAAATCCCGCCTACCTAGAGGGAGGGGCTTATCCTATAAAT
CCCGCCTACCTAGAGGGAGGGGCTTCTCCTGTAAGTCCCGCCTACCTAGAGGGAGGGGCTTCTATAAGTCCCGCCTACCT
AGAGGGAAGGGCTTTTTTTCTTAAGGCACTCAGAAGGAGATGAAGGGGATAGCACTTGACCCATCTTCCTTATCCCGGGC
TGTCTCCTGACCTCCCTGTGATAGACTCAGTTTGGACACCAATACCTACCCAGCAGTGGCTGTTGGATCTTTCCACGGCT
GCCTCACCAACTCATGAAGGAGTCCACTGGAACCTGAGCCCCGAGTCCCAACAGCCCAGGCATGCTAAGTGTACCACCTC
ACCCTCAGAGGAGAGCACTGCTCTCTACAGACCACTCCATTAGCACAGAGCCTTAGGTGTGGGTCACTGTTCTAGGGTGA
TCAGATCGTATCTCCTCAGGGAAGCAGAGATCCAGACCTAACTAATGGCTGCCCTACCCACAGTGCCTGCCTTCGTGGTC
CTGCACACTGCTCACTGGCTGTCCTGCCAGACTACAGGACCACTGGCTGACTAGCAACTTAGGAAGTGCCTTGGCGCAGA
AGGGCTGTCACAGAGGCCAGGCCTGATCTAGCCAAGTAGGGTGTCTCTCAACCAGATGTCCTGGGAACAGGTGCTTGGGG
CAAGGCCCATTGTTAGCAAGAGGAGATGAGCCAGTGCAGACGGGATTCAGAACCCATGCAAGGGTTCTCCTGCCCATCCC
CTCCTGCCACCGGATGCTGCCTGCCACCAGCCTCCTCTAACTCAGCAATGAGTCAGACCTGAGCTCTGCATTCACGTTCT
TCACTGTGTATCCTGGGCAAATGGACAAACCTTCAGGATTTTCTTGCCTCGGTTCCCAAATAGGAATACAACTTATTTCC
ACAGCTGAAAAGGTCTGAGAAGGATTCAGTTCTCTTTCAATGATCTGTGCATGGCATTTTTGCAATTATTTCCTGTAAAG
TTTTAAAACACATTCACCTTTTTGTTTGTTTGTTTGTTTGTTTGCTTGTTTAAATGCACTTAATTAAAGATTTTCCTCAT
ATCGTATCTGTGGCTTCAAAACCCAATCTTCCTATAAATAAAAGGTAATTATCAAAAGCAGGTGCAATAAAACTCTGTAA
CGTGTGAATCTGATCTGGGGGTGGAGCCTGATAGCCGAGCACTGCCTTGCTCATTCTGTCCCAGTTCTACAAGAAAGAGA
GGTAATGTCAGGGTAGTGTCTATGTCATTGCTGTTGGAACTCTGTCATCTCAGGCTAGAGACTGAGGGACACTTCCTCTG
TGACAAAAGCAGGGCTGTTGAGAACATCTTGGTCATTTATAGGAACCAGGCTCCTGTGCACAGATAGGCTCCAACCTCTC
TCAAATCCACATGCAGGCTTGGGCAAGCATCCCAACAGACTATGGCTCAACCTATCATTCGTCTATCCGCCTCTCTGTTT
CTCTCTGTGTGTCTCTGTCTTTCTCTCTCTCTAGCCATCTAATGATCACCTATCAATTAGCAGAATTTTTGCAGAGTATT
GAGAAACAATCTCTAGCATTCACATATGGCAGACATTGCTAATTGATTTTATTATGTATGGTAGACGCTAATAATCAAAT
GCACCTATGCATGGCAGACATTGCTAATCAATTACACCTATGCATGGCAGACATTAATAATCATAGCCTTCCTTCCATTA
GAAGCTGGATGCTGCCCTAGATTCTGTGGTACTGTGTCTGGGGCAGCCCCAACTGATGAGAACCTAGCCACATGTCTGGG
```

TCACGCTGCCTCAGTTTCCCTCTACAATAGGAGCTCTTCCCCTTTCTCTAGGCAGGGGCAGCAGATGCTGAGATGGGGCA
GCCTGGGAAGCTGAGAAAGCAATGAACAAGGGTCCCCAAAGCCCAGGAACCCAGAAACCCAGGTGTTCCTGTGTCTGGTG
AGAGACCTGAGCTGGCATTGTCTGGAGAAAGGGGTCACCTTATGCCAGGTGGAGATGGCCAAAGCCACTGCATGTCCAAT
GGAGATTGTGGGAGTGGAGCCAGGAGAGAGCAGCTGGCAGGGCAGTGCAGGAAAGGGGGTGCCAGGACCCCCACGATCAG
ATTTCGGGGGGAAAGCAGCGGCTGGAGAGAAGGCCCAGCCCGGGCTCTGGAGAGTCTTGGGTGGGGATCAGGAAACAGTT
AGGGACATCTTGGGCTGCAAGGAAGAGACAGGGCTGGGGGTTGGGGACAGAAGAAGGCTTCAGGCCTCTCCCAGGTCCTT
TTTGGCTAAGGTTGACCTGTTCCTCTTATCTCCAAGTCTACATCTCTGCTCTCTCGTCCTTGCTCTCAGCAGCTTCCGGA
GTTTTCAGGTAAATTGTGGTTGAATAAGTTTGTTCAAGTTTGAGACAAAAATCCAGCCATACCTTACTCATTGTGATGCC
CCGGCCAACAGAGGAAGCCCCAGGGAAACAAAAGTAAAACCAAACAGCAACATTAGAGGCAGAGATGGTAGTTCATTCCT
GAACCCCAACAGCAGAGAGCTAAAATGCGGGATGGGGGAGGTGTCTCAGGTTTGAAGGTGACTGGTCTGTGTAGTGAGCT
CCAGGCCAGTCTGGACTACTTGACAAGTTCATGTTCCAAAAACAACCAAACCAAACCAACCAACCAAACAAACAAATAGC
CCATACCACACCACCCTGCCCCCCAAAAAAACCCCACAAAACCCACCACCCAACTCTCTCTGTCTCTACCCAAAACAACA
ACAACAACAACAACAACATGTCACCTCCATGTCTGTGAGAGACAAGAGCTTTTGTCCCACTTTTCAGATGAGGAAAC
TGAGGCTGAGGGTAGTTAAGGAGTGTAGTTGCTAGTGGATAGAGATTGTGTGCTCCAAGCTCCTCAGTTCCTGGACCTTT
CTAAGTGCAAACTTGCCTGGAGACGATTCCTCCTCCTCCTCTTCCTCCTTCTCCTCCCCCCACCCACCTTTTCCTCCCCC
CCTTCCTCTTGCCCTTCTTACCCCTCCTCCTCTTCTTCTTCCATCAGTTTATTTACTCATCCACTTTACATCTGGGGTAC
ATGGGGTGCTTCTTGGAGACACTTCTACCAAAGGCAGAAAACCAGAAAGCTCTGTCTTGGCCTCTGGGTAGCAGCCTTGA
AGAAAGAAAAAGATGAAAGGCCCACCTCACTGCAGGAGGAAGAGAAACCCTTCTTCTCACTGCTCCACTGGCCAGGGCAT
GGCTGCCAGTCAGGTGAGGTAGTCTTGAACTCCACCCTCCCCTTCATGACTGGAGCCTCCTTCTCTCCAAAGCCTCCTAC
CTGAAACCCAGCATCCCGCAGTCCAGGCACCAGCAGCAGCAGCAGCAGCAGCAACTTCATCCTGCTCAACCTGTTCC
CTAGCCCACCTGTCTCCATAACCCATCTGTCTACCAGGAATGTCCATTTTGATATTGCAACATGATGCCATCTACAAAAT
CCACCAAGAACCAAGTAGAGAGAGAGAGAGAGAGAGAGAGAGAGAGAGAGAGAGAGAGAGAGAGAGAGAACTCACATAATGTGGTT
TTACTTAAAACATTTCTGTGTTGGTGATAGAGCTGTCCTGCATGTAGCCATGGGTTGCCAGTTGGGAGACTCAGGACAGC
TTCACTGAGAGCCACATGTGGCCGCCCCGCATCCTGGTTTGACTCCAGAAACACCCTGTTGTCATCAGGGAAGACTGGAT
TCTTCAATGTCACTGTCCCTGAATGTGGGGACCCTGGCGAGCTGTCATCAGCTCCATGCCCCTGTGCCCACGGGGTGTAC
TAGGTACTCAAACTAGGCTGCTTCCAAGTAGTCCCTCAGTGCTCACAGTCTGCAGCTCTTAGTACCACTTGGAAGGTGAC
AATGCTGAGGCCATGTGTGTTACTTTCAGGGCTGGAGGCTGAGGTTGAGGGTGGCTACAAATAGAAACTAGAAAGAACTG
ATTTCCATTGTCACCTGCTGATCCCCAGATTGAAACAGCCCTCGACTCAAGCAATCCACCCACAGACTCCTACAGATCCT
TCAGAACCCCATTTAAGAACTCCCACATCCAGGAGGCCCTCTGAGTTTCCCCTCCAGAATTAAGACAGTTCCTCTGAAGA
ATCTCTGCTCCAAGTGTTGCCGTGTGCGTGTGCTCTAGCCTTACATCCGTGCCCTTTGGTTTGCTGGTTTGCTTCTTTCA
TCCCATTTTACACAGGAGACATGAACTCACAGTGTTTTGATGGCTCACTTGAGATCACACAGCTAGAACACTGAAGAGAA
AGCCTGTGACCCTGGGTGATCTGTGACACTGTCACTGGCCCAGATTGTCAGTGTTCTTTAAAGCCTGTGCCAACAAGTAT
CTCAGACTATGTGCCAAGAACCTATGGTGACAGAGTGGCAGCTTCTGAGACCTAAGAGGCTGCTGCCACATCCCATGCCA
CAGGGAGTTTAGTGTAGAGACAGAGCAATGCTGAAATGTAAGCTCACTAGAAGCTCATAGCCTGATGCCTGTGTCAGCCC
TGGGACAGCCCCTCTGCTGACATTTTCCGATACCAGGGATGTGACACACCCACTAGGGGCCTTGAGAAAGCAAAGCAGGA
AGCCGCCTGAGACCTGGGGTGCAGGATGCCAGGAGGTACCCTTGATTCCAGGAAACCAGGGCACTGAGGGGAGGGAGCAG
CTGGTGAAGAGGCCAGCTTCTCCGCCCCTCTGTGCACCTGGCCTCTCATGGGCATGCACCCAGGCACACAGCTGTAAATA
TACCATAGGTGCTGGCCCTGGGACTGGAATTGTCCACAGAGACCTCAGCCTTGGGGTGAGAGAGTAACCATGGAGGGCAC
AGACAGGTAGTCATCACTCGCCAGCCTGTCCCCATACCTTGCACAAGACCTCATTAGGAAGCACAAGCAGACTGCTTGGC
TAGGAGAGAAGATGCTGGAAGGGCCTTCGCGGGTGCTCAGCTCAGATTTAGCGAGATGAGGAAGCACTTGACAGTGAGAA
ATAACAAAGCTATTCATGTATTCATTCCATGCTTGTTTGTGAAAGATGATGACATGCAGGCAGCTACAGGGTGAGCAAGG
GAAGGAAAGTAAATGCCATCTACCTGTGTAGCCCTTGATGGCCGGGACAGAGAGAGAGAGACAGTGGCCCTAGCCTTGTG
GAGTTGGGCAGCCTATGCAAGGTTCTGGCTTCACTGCCAGCCAGCTGTGACCTTCCTGGATGCCAAAGCTTCTTTGTGCC
TCTGTTTCCTTATTCTGGTGATACCAGGGCTGTCATGAGGATAAAATAATTAAGTGTATATGGTGTACTTAGAAGAGTGC
TGGTAGTTGGAGCTGTGGCTGGGGACAGGACTGTAACCGGGTGGCCCAGTGATGGTGGGTGTGGGAGCTCCAGCTTGCT
GCTTGCTGTTGGTGCTCTTGTCTGCCTTGCAGCTCTTGGAGACAGGCACATGGCCTCTGCTGCCCCTCTGCATGCGGTCT
CTGTTGCCCCTCTTTTCCCTTCCTAGGCTCCTCCTCCTTTGTACATCTGCCCCAAGCATCCTGAAGAAGAATCCTAAGTC
TTCTTATGATTCTAGTGGCCTCCTCCTCCTCTTCCTCTTCCTCCTCTTCCTCCTCCTCCTCTTCTCCTCCTCCTCCTNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNCTTCTTCTTCTTCTTCTTCTTCTTCTTCTTCTTCTTCTTCTTCTTCTTCTTCTTCTTCTTTTCTTCTTCT
CCTTCTTCTCAGGTCGGCCATCTTAGCTGCCACCAGCCTTACTTGGGTCATTGTGCCCCTGCTAGGTCTGTCCCATTGA
GACTCTTGGAGCCTTAGTTTTCTTGTCTATGATGTGAAATGAGGGAAGCAAAAGAAAAAAGTCTTGTTTCTATGCTTCT
GGTGGACCAGAAGGCTAATCTAGAATCTCAGAAGGAGGTGGAGGTCCCCCATCATCAGAACATTCCCCCCTCTTTTACCT
CCCCTGCTCCTGTTCTCAACATTCAATGTCCAGGGGACTTAGAGACAATGCTGTGTTTTTGTGAGCCCACCTGAGGCCAG
CCTGACATGCAATTATTGATCAGTAACTCTTGCTGCAAGCAGTTGACCTTCATACTAGATGATGAGATTCGAAGGAGCTG
GGACATCGTTAGATGCTCTTGGTGGTGGTCAAGTCCGGCCATTGAGTAGGTGACCTGCTGATGGATCCTGTCACCTGCTG
CAGTATGGGTCTGAATGAGGCTCCAGGAGCAGAGCTAGCTGGCTGGCAAGCAGATTCTCTCACTGTGATAATGATGCTTG
TGCAACTGGGATTCCACACGGAGACTGGGGAGAGGAGACAACATCACTAGGGCTCAGAAGGATGGCAGTGATGAGCAGTG
GCCACATAGAAGGCACTGGCTCCGTTCCCAGGTCCTCCCACCATCCCCTACCCCTGCTAGGCTTTCAGCTATTGGGTCTA
CTCTATGTGAGAAACAGTTGCGGGAACAGAAAAGAGGGCATGTGGCTAATAGAAGGCGAAGCCTCCTTGGAGACTGAGTG
CGGAGGATGCTGGCCGGGTCACCTAGGGAAAAGAGCCAAGCCTGGAGATGAACAGTCTCCAGTGGGGCTCTGGCTGCAGA

```
TTGTAAAGATCAACAACAAGAAAATGCTGGCTATTGAACCCTGATCCCTTGGACATTTGGAACCATCTGCCTGAGGCCCG
TGTGCCCAGCTCTGCCAGCAGCCACCAGAGGCAGGAGGAAGCTGGAGGGGTGCACACCCCATCCTGGACTTGCTATGGC
TCGATTGGAGACGTCTGGACTTTAGAACTGAGATGGAACATATACTACTGTTCTAAACCATTCTTCCGCAACCCTCTGTC
TCAGCTCCATCTTCCCAGACATATATCTAGATAGTGTCTGGCAAGGATCCTGGATCCCTCTCCTGCCTTCACAACAGAAG
GTTTGAGAGAGGCTCGAATAATTAGGAGTCTTCTCCCTGCTGTAAGAGCACGGGAGTGTCTCCCAGTCCAATGTATGGAG
ATGCCTGCCAACAGAGCGTGCCTATGTAGAACACGTGAATAAGACCCAATTAGGAAACCTAAGTGCACATAAGTGAAAAT
GTTATCTGTCAGCTGGCTGTGCTCCCTCTCCTATCCAGATAGGACTCATGGGTAGGGGAAAAGAAGTTCTTTGGTGCTGG
CCATTCTGGGCCCTGTAGTCCTCACTAGACTAGTGAACCTAAAGGCTTACTTTAATTTAATTTAATTCCCAGCTCTGAGC
ACGAGGGACCTATGGCTCTAACCTTTGGAAACTAGACACTTGGACTTGAGGTTTGTTGCTCTGGGTTTTAACCACCCTGG
TACTTGGCAGCTATTGTAATAACGCTTCACCTTCAGCAACAGTACATAGCAAGAAAAAGCACCGTTGGCACTGCCTTCT
CTTACAGCTCTTACGCACGCCTTGGATGCTGGGACCCTCGTCCTATAATGATGACTGTTCTAGGCCCAGAGTGTGAGGCT
CCTCAGCCATGACAATTGAACTACGGTGAATAAGATACTCGGTCTCCATTCTGGGCAGCTCTCTTGAACGTGCTCAGTGA
TCAGGCCTCGTAGCTTCTCCTCTTCAGGTCTTTACATCTTACATGGGCTCTGTGACTCTTACTGGCCATCAGTTCTGCTC
CAGCCACTTTCTGGAACATCTCAGCTGTCCCCTGGCTGCTCTTGCCATGGTTGTTCCTTACGGCTGCAGTCACCTGGAGG
CTGCCTTTGCTGCTGTCCCACGATCCTCTCCTGGTGTCATCAGCCAATTCCATTAATGATAAGGATCAAATATCGGCAAA
AAGAAACCACTCAAGAAAATTTTATTGGATTTAATGTTAAAAAAAAAACCCGGGGCCAATCAGGGAAGTATCTCCAGGCT
GGCGGAAGGGAGCTGAATAGCCAACTTATATAGACGAGAAGTGAGTGTGGTAGTGATATTTGCAACAATTGTGGCAAACT
TTTATGCAAACAAAGGACTGTCTTTGGACTGATAGCAGGCCTTCTGCTGCACGGTTTGGGGAGGTGCTATCCCTCTGGCC
CGGGTGGAAATGGCTGCAGGGTCATCTAGAGTTCACCAAGTCCAGCCCAAACTGTCTGAGACAGACCGGTGGAAACAAT
GCCTTCTAGTTCCTTCTGCAGCTGACCTTACTCTTCGTTAGCAAGCAGCTGTACTGGCCAGGTAACTTCCTCCAGGTGTC
CAGGGAGCATTTAAATTGCTGGTATGAGGACAGTCCAGCTGGCTGTTTAACTGGGAATCCTTTGCTCAAGATGGTCAGGA
CATCATAGAAGCATTTGACATCTGTCACTTTGGGAATTAAATTATCCTATGCTCCAAATCTTAGCTTACTAAGAGGAAAA
AATAAGCATTGAAAAGGGTGTTTATCATAGAATCTTACCATCAGTGACTTAACAGGAACCTGCTACATATGTACCCTGCC
ATGAGTGGCCCCTGGAGATGCTGTGCTGCACACAGGCCTGGAAGGGGCACCCGCCGCGCGTGCACTGGGAAGACGCTGA
CTACTCATCTGGTGAAGTGCACAACATATGCCCAGCCCTGGTTTGTGAGGTCTGGGTAAGTTTACTGAGGCTGTTGAGAC
TAAGCACAGGTTCTCAGCATTGGAAACATGTGCCTCCCAAATGATTCAGGCTGTGAGTTCAAGATCAAGGTGTCCGTGGG
TCGCTGCCTCCTGGAGCTGGAGGAGAATCTATTCTAGGCATCACCCTGGAATCCAGTGGTTGCTAACCTGTGTGCTGTCC
TTGCCTTTACAGATAGCTAATCTTGTCTCTGCCTTCACTTGCATTTTGTTTGTTTGTGAGTGTGTGTGTGTGTGTGTGTG
TGTGTGTGTGGTATGTGCTTGTTTGTGTGGTGTGTGTGTGTCTGCCTGCCTGCCTGCCTGTCTGTCTCCAAGTCCCTCTT
TTTATAAGGACTCCAGTCATATATCATTATTGTCCATCTTACGACTTTCTGCCCTCAACAACTCAACTCTGTCAAGCCTG
AGATGGCAGTCTGGGGTTGTGACACATCTTGGCAGCAGGCAGGAAGACTTGGATTTTAACCAGCCTGACTCTCCAACCTC
TGGATTGACTTTTGTCACCTGATTAAGTTGAGTGCCAGGGCCTGTATCTGCCATTCTTTGTGCCCGCCCCTACCTATAGC
AGCCAGGCCTGTGAGTCTAGGGGCCCAGGAGCCTGACAACTAGTAGCACTGTACCATACAGAGTCCCAGGAGCACTGAGA
CCCTTGTGGCATCTATGTGGAAGTATGCCCCTATTGTCTCTGCTGTGGGGACAAGCAAAGAGAAGCAGAGGACGAGGGAG
GGCCAGACACCCCAGCCATGGCTCCAGCCTCTGAGATCTTGGGGATCTTATATCCTTGCCGTCCTGGGGTCAGCCCCTCC
CCCAGGCTGAGTGGATCAATGGCTGTGGTCACCAGGTGCAGAGGCCACCAATTGTGCAAGATCTTTCTGAGATTTTTCAG
CTATCATCCTGCCTGGGCCAGGGGCCAGGCCACTTGGTAGTTCAGAGGACAGGACAGGGCACAGAAGAGGGGCTCAGGGT
GCCTGTGTCCCAGTGGTATATGGACTTCGTTCCCATCCTGAGCTCCTGTTCCTAGTTTGCAGTGTGGAAGCTGGAGATTG
GGAGAGAGTGAGGAGAAGGTACTGGGCTGGGAATTCGTGTTGAGGGAAGAATGGACCCTCACAGGTCAAGG
```

MOUSE mRNA SEQUENCE : mR5-001.1 (Seq ID No: 26)

```
AGCAGGAGATGGCCGTGGACCCAACCTCCAGCAGGACTGCAGGGAACATCTCGACACAACTCCATGTTGCAGCCAGGGTT
TGCATCCTCAGCTCCTCTCAGCTGTGATGGCTACCATAGCTCTTCCCTGGAGCCTGTCCCTCTACGTCTTCCTCCTGCTC
CTGGCTACACCTTGGGCATCTGCAGCAGTGAAAAACTGTTCCCATCTTGAATGCTTCTACAACTCAAGAGCCAATGTCTC
TTGCATGTGGAGCCATGAAGAGGCTCTGAATGTCACAACCTGCCACGTCCATGCCAAGTCGAACCTGCGACACTGGAACA
AAACCTGTGAGCTAACTCTTGTGAGGCAGGCATCCTGGGCCTGCAACCTGATCCTCGGGTCGTTCCCAGAGTCCCAGTCA
CTGACCTCCGTGGACCTCCTTGACATAAATGTGGTGTGCTGGGAAGAGAAGGGTTGGCGTAGGGTAAAGACCTGCGACTT
CCATCCCTTTGACAACCTTCGCCTGGTGGCCCCTCATTCCCTCCAAGTTCTGCACATTGATACCCAGAGATGTAACATAA
GCTGGAAGGTCTCCCAGGTCTCTCACTACATTGAACCATACTTGGAATTTGAGGCCCGTAGACGTCTTCTGGGCCACAGC
TGGGAGGATGCATCCGTATTAAGCCTCAAGCAGAGACAGCAGTGGCTCTTCTTGGAGATGCTGATCCCTAGTACCTCATA
TGAGGTCCAGGTGAGGGTCAAAGCTCAACGAAACAATACCGGGACCTGGAGTCCCTGGAGCCAGCCCCTGACCTTTCGGA
CAAGGCCAGCAGATCCCATGAAGGAGATCCTCCCCATGTCATGGCTCAGATACCTTCTGCTGGTCCTTGGTTGTTTTTCT
GGCTTCTTCCTGCGTCTACATTTTGGTCAAGTGCCGGTACCTTGGGCCATGGCTGAAGACAGTTCTCAAGTGCCACAT
CCCAGATCCTTCTGAGTTCTTCTCCCAGCTGAGCTCCAGCATGGGGGAGACCTTCAGAAATGGCTCTCCTCGCCTGTCC
CCTTGTCCTTCTTCAGCCCCAGTGGCCCTGCCCCTGAGATCTCTCCGCTGGAAGTGCTCGACGGAGATTCCAAGGCCGTG
CAGCTGCTCCTGTTACAGAAGGACTCTGCCCCTTTACCCTCGCCCAGCGGCCACTCACAGGCCAGCTGCTTCACCAACCA
GGGCTACTTCTTCTTCCATCTGCCCAATGCCTTGGAGATCGAATCCTGCCAGGTGTACTTCACCTATGACCCCTGTGTGG
AAGAGGAGGTGGAGGAGGATGGGTCAAGGCTGCCCGAGGGATCTCCCCACCCACCTCTGCTGCCTCTGGCTGGAGAACAG
GATGACTACTGTGCCTTCCCGCCCAGGGATGACCTGCTGCTCTTCTCCCCGAGCCTCAGCACCCCCAACACTGCCTATGG
GGGCAGCAGAGCCCCTGAAGAAAGATCTCCACTCTCCCTGCATGAGGGACTTCCCTCCCTAGCATCCCGTGACCTGATGG
GCTTACAGCGCCCTCTGGAGCGGATGCCGGAAGGTGATGGAGAGGGGCTGTCTGCCAATAGCTCTGGGGAGCAGGCCAGT
```

GTCCCAGAAGGCAACCTTCATGGGCAAGATCAGGACAGAGGCCAGGGCCCCATCCTGACCCTGAACACCGATGCCTATCT
GTCTCTTCAAGAACTACAGGCCCAAGATTCAGTCCACCTAATATAGCAGGTGGCCAGGACTGGGATCCAGCTGCCTGGAT
CAGGTCAGGCTTGAGGAAGACTGCTTAAGAGGTCTCTTGAGGACAGTCCACTGCTGAGGACTGTCCTGGCTATCCCTGCC
CCCCCCCTCCAAACTTAATCATCCACTTCTGAACTCCATTTGCTACTTCCTGGTCTAACCAGGGTTTGGTGGAGGGTGGG
GAGTGGGGGAGCGGTGGTCAGCTCCACTGCCCTATTTAGTCATGAGGTCACTAGCTTCCTGTACCTGCCTCCTTCCCTCC
TGCTTGCCTTCACAGGGCAGCTAGAACTTGCTTCCCCCAATACAAACATAACCTTGCTCCTCATTTATCCCTGAGCTTGC
TGGGGCTCCTTACTTCCCTCTGCCCCATTTCCTGTCTCAACTCTTGCCCTGCCAGCACGGCTGTGGAACAGCTGTCCTCA
GGTTGTGATGTGGCTGTACACTGTCTTAAAATCAGAGCAGCTTTGACAACCCAAACGAAGAGGTCCTGATAAGACTGAAT
GCAGCCTGTTGACTATGGAGGGTCCCTCGTAGAAGGAGGGGTTGGGAAGGGTCATCAGTCATCACCTGAGATCGTAGCCA
CTCTCCTGCACCCCATCCTCAGCTATTCTGCTCCTGGAGCATGAGTTATTTTATGTGTTCTTGGGCGATGCTGGTCTATG
TAAGGGGTGGGGGCGCGGGGTTGATTGAAGTGAGGGCTCCTTCTATGTTGCTTTGGGGACCTTGTCTATTCTGGGGTGGG
TTTTTCTGTGAAGCAGCTTCTTTGGGGTAACCTAGCCCCCTTGTCAGTAAGCCTAAGAGAGTCAAAGGACCACCGAGCTG
GACTTGAGTGTACGCCTACTTTTGATGCAACATCTGGGAAAAGTACTTTTTTTGTTAATGTATCCTTGAAGCTAAGAAAA
GCTTACACCA

MOUSE PROTEIN SEQUENCE : mP5-001.1 (Seq ID No: 27)
AGDGRGPNLQQDCREHLDTTPCCSQGLHPQLLSAVMATIALPWSLSLYVFLLLLATPWASAAVKNCSHLECFYNSRANVS
CMWSHEEALNVTTCHVHAKSNLRHWNKTCELTLVRQASWACNLILGSFPESQSLTSVDLLDINVVCWEEKGWRRVKTCDF
HPFDNLRLVAPHSLQVLHIDTQRCNISWKVSQVSHYIEPYLEFEARRRLLGHSWEDASVLSLKQRQQWLFLEMLIPSTSY
EVQVRVKAQRNNTGTWSPWSQPLTFRTRPADPMKEILPMSWLRYLLLVLGCFSGFFSCVYILVKCRYLGPWLKTVLKCHI
PDPSEFFSQLSSQHGGDLQKWLSSPVPLSFFSPSGPAPEISPLEVLDGDSKAVQLLLLQKDSAPLPSPSGHSQASCFTNQ
GYFFFHLPNALEIESCQVYFTYDPCVEEEVEEDGSRLPEGSPHPPLLPLAGEQDDYCAFPPRDDLLLFSPSLSTPNTAYG
GSRAPEERSPLSLHEGLPSLASRDLMGLQRPLERMPEGDGEGLSANSSGEQASVPEGNLHGQDQDRGQGPILTLNTDAYL
SLQELQAQDSVHLI*

MOUSE PANTHER CLASSIFICATIONS
        FAMILY (SUBFAMILY)
            CYTOKINE RECEPTOR(Unassigned)
        BIOLOGICAL PROCESS
            Biological process unclassified(2.99.00.00.00)
        MOLECULAR FUNCTIONS
            Molecular function unclassified(1.97.00.00.00)


MOUSE GENE ONTOLOGY
        BIOLOGICAL PROCESS
            signal    transduction    >    cell    surface    receptor    linked    signal
transduction
            cell communication > signal transduction
            protein metabolism and modification > protein complex assembly
            defence response > immune response
            humoral defense mechanism > antimicrobial response
        MOLECULAR FUNCTION
            transcription factor > transcription activating factor
            molecular_function unknown > lymphocyte antigen
        CELL COMPONENT
            cell > membrane fraction
            plasma membrane > integral plasma membrane protein
            cell > plasma membrane
            extracellular > extracellular space
MOUSE PROTEIN DOMAINS (INTERPRO SIGNATURES)
            IPR001777 (fn3)
            IPR002996 (CR1A)
            IPR003531 (HEMATOPO REC S F1)


HUMAN GENOMIC SEQUENCE : hD5-001 (Seq ID No: 28)
GAGCACACAGGTGGAGAAGGCTTTGGGCTGCAGATGCAGGGACCATGGGAATAACAACGGGTGGCCCCCACGCCAGCACC
CCTGAGTGCCAACCAAAGTGGGGGGTCCTTCTCCATGAATCCTTGTCACTGGTCAGGCACTTTTGGGGGCTTTGCCCTAG
TCCAGAACTTCCCTTCTGCAGGCTCAGTTCAGCCCCTTCGTTCTGGGGAGGGCTTCCTCTGCATCCTCACTGTGGCGTGT
TTCAGGATCTGCCAAAATGCACCTGCCTCTGTCCCCTCGGCCTCACCTTGTTTAGATTCTTGAAGTGGTAATGGAACACA
AATGCTAGGACTCCAAGAGGCAGAGACCAAGAGGCAGAGGTTTTACAGTTTCTCTCTATATATGTTGTAGGGCCAGGTGA
TACCCAGATCTAGCCTGCCTTGCTCCTTCCCCCTTCCTGAAATGGGCATCTTTCCCGCTCGGAGGAGTTCAGGGTCCCCT

```
GTTCTCAGCGAGCCCAGGGTGGCCGCTTCTGGTGCCAGGCCTGACGGAGGGCATCGTCACTCTTGAGTGAGAGCATGTTT
CTGACCCCATTTTTTGCTTTTCCCCAAGCAGACTTTGCAGGCAACGGTGACAGCCAGGCATAACCTTCTTAGAGGGACAT
TTTAATTTTTTCCGATTCTTTAATAAAAACACTAAGAAAAAAAAAAGCAAGCAAGTGTTACTTCTTGATAGAGAGGAACT
TCTCACAGACACTTTTCAAACATCACACGTCCTTTTTCCTCACAGCAACACCTAGATTGTCTTCTGAGAAAGATCGCAGA
TTTTGGTGAAACCTGCCCAAGAATAAGAAAAAGCAAAGAGAATGTTTTCACTCTACCTGTTTCCCACAGGAAAAGCGGAA
ACCGTTGTGGGTACTGGCTTTCTTGACTGTCCCCATCACAGGACACTGGCCTGGCTGTGAGGAGACCTGGGTTAGGAGTC
CGCCTTTTTGCCAACCAGCGTTGTGGCTGTGGACAAAATGCTTTCCTCCCTGGGTGCTCTCAGCATAGTGAAGACGGCGG
AGGATGGGTGGTTCCACGGGCCTGGTGACTCAGGGACAGTTTCAGGTAGGAAACTGGCTGGAGGACTTGGCTGCTAGCAG
TGTCCAGGGATTCCTGCCTGAGTGGCTCAGTGCAGGTAGATGCTAGTTCACAGACATGCTCCCACGCACCTTCACAGACG
TATGCCACACTCATGCCTTCGAACACAGGGCTTACCGCCTTTATGTTTACCTGAGCAGTGGTGCACCCACGTGTATTCTG
AACTTCGTTTTTTGTCTCTTCTCCGTACATCCTGGAGATTGCTCCATATCAGGACCCAGAGAGCCTCCCCTTCCTATTTT
TTTTAGACGGAGTCTTGCTCAGTCACCCAGGCTCGAGTGCGGTTGCGCAATCTTGGCTCACTGCAACCTCTGCCTCCCGG
GTTCAAGTGATTCTTCTGCCTCAGCCTCCTGAATAGCTGGGACTACGGGGTGCCCACCACCACGCCCGGCTAATTTTTTT
TTAATTTTTATTTTTGGTAGAGACGTGGTTTTGCCATGTTGGCCAGGCTGGTCTCGATCTCCTGACCTCGTGATCCGCCC
GCCTCAGCCTGCCAAAGTGCTGGGATTACAGACATAAGCCACCGCGCCCAGCCCTCCCCTTCCTATCTTGGCATAGCTAC
GTGGCATTCCACTGGATGGAAGTTCCCTAATTTGCTCATCACCTGTCCCCTATGGATGCGCATTTTGTTGGCCTCGGGTC
CCGTGACTGCAGGAGTTACTTTGTACACACGTGTTCAACATGGGTCACTCTGTTGATGAGTTTTAGGTGTGGAATTGCTG
GGTCATGTGTCAGAGATGTTGGTAGATGTCAGATTGCCTTCCAGGGAGATGCTTGAAGCCCACGCCAGCAGGGTGACTCG
CCTTTTCAACCTGCCTTTGATGGCAAAGCTTTTGAAACTTCAAATATGCTATCCTGGTGTTTTTAATGTGTATTCTCAGG
ACTCTTCAGGGTATTAGGGACTTAGCACTTTTGATTCTTTCTCTTTTCCTTTTTCCTTCCTTCCCCTCATATTGACACT
GTCCGTATGTCAGGTAATTTTCTGGGTTCTGGAGTGGTGTGGATGAATCCCACACTGTTCCTGTTGTCCAAGAGCTCCTG
GTGTAGACTCATAAGCAAGGCTCTGACATGAGATATGGTAACTGGCTTGTGCCCAGAATCTGCCTTGGCCAGAATTGGAG
ATCAGGGAAGGACTGGTTTTAAATCGGGGAGCTGATTAGTCTGAGTAAGTAGGGTGGGAATATGGTGACCAACTTTGTCC
TAGGAATATCACTGTAAATCTTATGTTCTGGAAACCCCCCTCAGTCCCAGGCACAGTGGGACAGCTGGTCACCTTGGCGC
GATCCTGGTAAGGAATGGCATGGTTTGCACAAGATAGGAGCTGCTCAGGGCGCTGGTGCTGGTGTGGCTATGAAGCAGGC
AGCTCCCGGATGAACAGGGAAGAACGTGGGGCTTCAGGTCTGGAGCCTGGGGGGCTGGCGTGGAGGTGGCTGCTGTGGCA
AAGCAGAAGTAAGGGTGATGGCTTGGAAGGAGGGGGTGAGGGAGGGAAGGAGTCAGGGAGAATGGCCACAGTCTGTCTTG
GGGACTGGTGGGTGGGGCGCCTCCAGTGAAGAGCAGGTGTCAGCAGAGGGGCATGCTCGGAGGACAGCCCTCAGGAAGTC
GGCCGCTAGCCTGCTCTTTCTCCTTGTTCTGCTCTGGCCCCTCTGTGGGCCCCTGCAGCTTTGTGATCCTCATTTGTCAT
GAGGGGTACCCCACAGTCTGATGGGGACTGGGCGGAGGGTCAGACACCCAAATTATGTATACCATAGTCCTCCTGACCAGG
GACCAGGACTCAAAACCAGGTCACTGGTGAGGCCTGGCACTGTGGGGGCAGGACTTTTTTTCCAGCTGGGGACTCGGCTA
CCTGCTGACTTTCCAGAAGACAGGTCACAGCACCGAGCAGGGGTGGGCGGAACACAAGGACTTGAGGGTGGGTGACAGTG
TCAGCTGGCTGAGCCAGGGAGGGCAGGAAGGGAGGTGGCCCTGGAGATACTGGAGGTCTTCACAATGGCAGAGGACAGGC
GGTGGCAGAGACAGTGAATGGCACCCCCAAACCTGCTCTCCCCTGCCCGTGTGCCCTCTACTTATAGCTGGCTACATCTC
CCAGCCTCCTCTGCAGCTTGGTTGGCTATGTGACTCAGGCGTAGCCAATGGGATGTACATTAGAAGTGGCTACGGCAGCT
TTGGGGAAGGGTCCTTAACAGGCAGAGGCAAGCCCTCCCTCTCCTTCCTTTCCCTTCCATCTGCAGGAACGCAGAGGT
GATGGGTGGAGCTTGAATAGCTCTCTTGGTCTATGAGGAGATGGGAGAAGGATATTGAAGAGACCCTAGTAGCAAGCCAG
ATTTAGCTCAGGATCCTGACATCATAAAGCCACCCCACCTAGCCTGGGTGGCCTCCCTTTGGACTTCTTTTATGTAAGAG
AGAAATAAAGCCCTCTCTTATTTTAGGTCACTGTTTTCTTCTTCTGTTCTCTTTGGACACACGCAGCTGAACTTCATCCT
AACTGGGACACTGTTGTACAGTGGAGGCGGTGATGGCAGCACGCGGGTGAGCTGAGGTGGAAGTTCAAGGGGTCTTGGTT
AACACAGGTCAGAATGATGGCAGGGTGCAAGTGCAGGCCTGGGTGTGGGACTGGTATGGAGCAGTTCAGAGGGAACAGGA
GGCTGGAGGCTACAGTGTGGACCCTGACATCTCCTGGGAGGCGGAGTGGAGAGGGAGGCTGAGCGCAAGGGCCAAGCACT
CCAGTGAGCAGAGGGAGGACGGGTAGGGCGTTTGGCAGATACCAGGACTGAGGAGGATGCAGTCTGGCTTGGCCCCAAAA
AAGACAGCCCAAGGACATCAGGGCTGGAAGACCCCAATATGCCCTTGTCCCATGGCCCCTGTGAAAGAGGGGTCTGGGTC
CAGTGGTGGGAGGGCTGTGGGACTGGGTGCATGGAAACAGAGGGTATGAGGCGGTTTGTTCCCAGTGGGACCCATGAAGA
TGCAGCGCAGAGGGTCAGGTGGGGATGTGGAAGACGTAAGAGAAGTAGTTATAGGAGCAAGGCGTGGAGCCTCGTGGAGG
AGGCTGGGTCCAAACCCCAGCACTCCTGAGACCCAGGTCTGGGTAGGCCCAAGTGGGCTCTGTCAGGGCTCTGCCATCAG
AGAAGCTTCTGGATTACATCTCAGGTTGCCTCAGAGAGAATCGGATGTCCTCTGGGACAGACCTAGGTTCTAATCCTGGC
TCTGCCACATTCTGGTAGGATGACTCATGAGGCCTGGTTTCCTGGCCTGTAAAATGGCAAGGGAAGAGCAGGCTCAGCAT
GCACAAAGCCCTGACAGAGTGAGAAAGCACAGGAGGCAGGGTCAGAGATGAAGATGGGGCAGTCCCTGTAGGACATGTGG
GTCAAGACTGAGAGTTTGGATTTTACTGTGAATGCACTGGGAAGGTTTGAGTGTGTATGATGGGGAGTGGCTGGGGGGTG
AAATGACGTGATTTGAGTTTGGGAAAGGTTGCTCTGGCCGCTGAGTGGAGAACAGACCAGAGAGTCAGGAGTATTAATAG
AAACAGGAGAACAGGGCGGAGGCCACCATGGGCCTGCTGAGGTGGGAGATGACACGGCTCTGAGCAGGGCAGGGGCTGA
AGAAAGCAGGTGCACCTCTCTGCACACCCCTGCTGGCCACCCATGCCTCTGGGCTTCTGCTGGCGTACTGTCTCACCCCT
CCTCACCTGGTTTTATGTTCAGCTGTAGCACCTGGGGGACAGACTGTATGAGTCAGAGAAAACAGAAACAGCAGTCCCCA
GGTCTCAGAGGCCCAACAATAGAGGTTTATTTTTCATTCAAGTTATAGCCCAGTGTGAATTGTCTGAGGAGGGGTGAGGG
CCTGTCCTTCAAGGGACTCCAGCCCCTCTACCCAAAACTTCACTGGATTCTGGCAGGGGAGGGGAGAGAGAGAAAGAGAT
AGAGGGATGGCCGGCCGTGGTGGCTAACGTCTGTAATCCCAGCACTTAGGGAGGCCAAGGCAGGCAGATCACGAGGTCAG
GAGATCGAGACCGTCCTGGCCAACATGGTGAAACCCCATCTCTACTGAAAATACAAAAATTAGCTGGGTGTGGTGGTGTG
TGCCTGTAATCCCAGCTACTCGGGAAGCTGAGGCAGGAGAATTGTTTGAACCGGGGAGGCGGAGCTTGCAATGAGCCGAG
ATCGCGCCACTGCACTCCAGCCTGGCTACAGAGCAAGATTCCGTCTAAAAAACAAAAAAAAAAAAAAGAAAAAAAAAGAGA
```

```
GATAGAGGGATTAAGAGACACAGGAGATCATAGGTGAGATTTTTTTTAGGCCTAGAAGTAGAATAAATCACTTCCTCCCA
CATCCCATTAGCCAGACTTGGTTCTGGAGCCCCAGCTCACTGCAAGGGAGGCTGGGAAGCAGACCCGAGCTGATGCCGGG
AGGAGGGTGCACGCCATGGCCTGGGGACCCATTCCACCAGGTTTCCCTCCTACGTGCTCCCACAGGCCATGAGGTGTTAC
ATAGAGGATGTGAAGGAGAATCATTCTCTGCGTAGCCCCCTCCCTGCCTCTCCATCTGAGGTGTTCTGGGCTAGGACCCT
TGACAAAAGAACAAACCGGCTCCATCCCCTGTCTCCTGGGGTGGGAGACTCTGTATGTGGGGGCTGCACCATCCCCCATT
CTGCTCCCCACTTTCCTGGCTGCTGCACATCTCAGGGGCACGAGGGTGGAGAGTGGCCTACCTCCCTCCCTTGGTCCTAC
CCCGCTGGCCTGGGCTGCGCGCCTCAGGCTTCCTGTGTCTGACCTGGTCTTTGGGGCATCTGGGGTGCTCAGTAAATGTT
TGTTGAATAAATAAAAGAAGGAAAGCAGGATGAGGAGGAAGAAGAGTCTTGGGCAGTATGTGCCAGGCAGACTGATGCTG
TCTCATGGATGAAATAGCTGTACGCAGCTGCTCCTTGCAAGCTGGGTCTGTGACTTCTGGCTTTGGCTGCGGATGCATTT
TTCTTTTCTTTGTATGTGTGTGCTTTTAAAGTTTTAACTGAATTCATTTATTTGGGGGCAGGAAATATGTTCATGCCATA
CAAAAGGGTCTATAGAGAAAAGCGTCCTTCCTAACCCTTCCTCACCTGGCTCTCCTCCCTGAGACAACCGGCGTTCCTAA
TTTCTTGCAAAGCTTCTGGGAATATTTGAGGCTTAATAAATGTTGGCTGTTATTCTCATGGTTGTGGCTGTTGTGTTTAG
TATCCTCTCCTGCCGCCAGACAGCCTTACTCTAGAGGAGAGGGAAGGGCAGTGTGGAAGGAACTGGCCCTTACAGCAAGC
CTGCAGTGCGTGGTCACATTAAATGCTTTATACACACCTTCTTGTCCAGTTCTCCCTTCATTTGGTAGGTCTGCACACTG
AAGCACAGGGAGGCCTAAGTCACAGCCTCAATCTCCAGTCCTGCCCCTCCTGGGCAGCAGTGCTCCACGCTTCTTCTTTT
CCAGCAGCCGGCTTTGCACATGGGAGTTGGGCCTGATTCTCTTTTTGGAGTCTTGAATTCCACTTTTCTCACATTTGCAG
AGCTGCCGTCTCTGCTCTTCTCTCTTCTTCTCAGAAGAGGTGGCAGGCTGGGCGCAGTGGCTCACACCTGTAATCCCA
GCACTTTGGGAGGCCGAGGGAGGTGGATCACCTGAGGTCAGGAGTTCAAGATCAGCCTGGCCAACATGGGGAAACCCCGT
CTCTACTGAAAATACAAAAATTAGCCGGGCATGGTAGCACGTGCCTGTAATCCCATCTACTCGGGAAGCCGAGGCAGGAG
AATCGCTTGAACTTTGTGGGCAGAGGTTGCAGTGAGCCGAGATTGTGCCACTGCACTCCAGCCTGGGTGACAGAGCTAGA
CTATGTCTCAAAAAAAAAAAAAAAAAAAAAAAAAAGAGGCCACAGCTTGCAATAAAACCCCTCTGAAACAGGGAATCAGGAAA
GGGAAGAAGCAGAGCTGCTGCTTCTGGGCTCTGAGGATGGAACTTCACAGGGTCTATGGGGCTGTGATGTAGCACGTAAA
TAGATGTGCATTTTTCTAAAGACCAATCCATAAGTTTCCATCATTTAAAAAAAAGAGCCCTGAGCTGAGCTCCCACGCCA
CCCTTCTCCCCCGCCAAAAAAGGGGGAGAAGGGTTAGGAACCATGACTTGAGAGGAAGAGGAAGGTTCTAGAATGTTCAG
TAAGTGACCATCAAATTGTTCACAAAGACCTGATAGTCATCTCTGGGAAGTGCTAGCTAGAACAAACAGACAAGAGAAAG
AAGTAAAGGGCATCCAAATTGGAAAGGAAGAAGTCAAATTATCCTTGTTTGTAGACGATATTATCTTATATTTCAAAAAG
CCTAAAAACGCCACCACAAAACTATGAGAACTGATAAACAAATTCAGTTAAGCTGCAGAATACAAAATCAACATACAAAA
GTTAGTAGTATTTCTATGTACCAACAGCAAACAATCTGAAAAAGAAATCAAGAAAATAACTCCATTTGGAATAGCTACAA
ATAAAATAAAATACCTAGGAATAAACGTAACCAAAAAAGTGAAAGATCTCTATAATGAAAACTATAAAACGTTGGTGCAA
GAAACTGAAGAGGACACAAAAAATAAAAAGATATTCTATGTTTATGGATTGGAAGAATCAATATTGTTAAAATGTCCATA
CCACCCAAAGAAATATACAGACTCAATGTAATCCCTATCAAAATACTAATGACATTCTTCACAGAAGTAGAAAAAATAAT
CGTCAAATTTATATGGAACCACAAAAGATGCAGAGTAGCCAAAGCCATCCTAAGCAAAAGAGTGAAACTGGACAAATCA
CATTATCTGACTTCAAATTATACCACAGAGCTACAGTAACCAAAACAGCGTGGCGCTAGCGTAAAAACAGATGCATTGAC
CAATGAACCATTGGTCAGAATAGGGAACCCAGAAACAAATCCACACACCTACAGTGAACTTATTTTCAACAAAATTGCCA
AAAACATACCTTTGGGGAAAGGCCAGTCTTTTCAATAAATGTTGCTGGGAAAACTGGATATCCATATGCAGAAGAATGAA
ACTAGACCCTCATCTCTCTTCATATACAAAAATCAAATCCAAATGGATTAAAGACTTAAATCTGAGACCTCAAACTATGA
AACTACTAAAAGGAACATTGGGGAAACTCTTTAGGACATGGGACTAGGCAAAGATTTCTTGAGCAATACCCCACCAGCAC
ATGCAACCAAAGCAGAAATGATAGATGAGATCACATCAAGTTAAAAGGCTTCTACACAGCAAAGGATGCAATCAGCAAAG
TGAAGAGACAACCCACAGAATGGGAGAAAATATTTGCAAACTATCTGACAAGGGATTAATAACTGGACTAGATAAGGAGC
TCAAACATCTTTATGGAAAAAAATTATAGAAATCCCATCAAAAATTGGGCAAATGATCTGAATAGACAGTTCTCAAAAGA
AAACATATAAGTTTATGAAAAGGTGCTCAACATCATTGATCATCAGAGAAATGCATGTCAAAACTACAATGAGATATTAT
CTCACCTCAGTTAAAATGACTTTTATCCAAAAGGCAGGCAATAATGAATGCTGGTGAGGGTGGGGAGAAAAGGGAACTCT
CGTACACTGTTGGTGGGAATGTAAATTAGTACAACCACTATAAAGAACAGTTTGAAGAGTCCTCAAAACACTAAAAATAG
AACTACTATATGATCCAGCAATCCCACTGCTGAGTATATACCCAGAAGAAAGGAAATCAGTATATGGAAGAGACATCTGC
ATTCCCACATTTGTTGCAGCACTGTTCACAATACCCAGGATTTGGAAGCAACCTAAGTGCCCATCAACAGATGAATGAAT
AAAGAAAATGTGGTACATATATGCAATGGAGTACTATTCAGCCATGAAAAAGAATGAGATCCTGTCTTTGCAACCACATG
TTTGAAACTGGAGGTCATTATGTTAAGTGAAACAGGCCAGGCACAAAAAGACAAAGCTCACATGTTCTCACTTATTTGTG
GGAGCTAAAAATTAAAACAATTGAATTCATGGAAATGGGAAGGGCAGTGATGGAGATGGGAAGGGCAGTGGGGGTTGGAG
GGGTGGGGATTGTTGTTGGGTACGAAAACAGAATTAGATCGAATGAATAAGATCTAGTATTTGATAGCATAACAGGGTGA
CTTTAGTCAACAATAATTTATTGTACATTTAAAAATAACTAAAAGAGTATACTTGGATTTTAACACAAAGAAAGGATAAA
TACTTGAGGTGATGGATACCCCATTTACCCTGATGTGATTATTATACATTGTATGCCTGTATCAAAATAGCTCATGTGCC
TCATGAATATAGACACCTACCACATGCCCACAAAATTAAAAACTAAAAAAAACAGTCATCTCTGAATGCTAAACGGAGTA
AGGGGCTTCCTGGAAGGCTGGGTGAAATGGGAGTCTCGGAAAGATGGTGTGTTGCAGGCTGGGAGGAGGGTGAGACGCTG
GGGTCACCTAGAGGGACCTGCTTGTGTGAAGCCTACGTATTAGTGGGTATGTGTGTGACCGGATGGAGGCGTCAGAGGTG
TTGGGTAGCCTGTGTGAGTTGGCGTGGGGGTGATGTAGGAGGGGAGAGAGGGAGGGCCTGCGTTCCCTTGGCTCCTGTGT
GCAGCTAGGCCCCTATTTGACAATGTGTGTCTGTGTGTGTGTGTGTGTGTGTGTGTGTGTGTGTGTGTGTGTGTCCGC
CCCCAGCGTAGGAGGCAGATCTTTATCTGGCCCTGGGTGCTTGAGGAGTTTCAGGCTTTCTCATAAGCCTCGTCTCCCCG
CCTCTCCACCCCAGGCCTTGCCCCTCTATCCTCTGCACAGGAAGTGGGCTGGCTCTGGGCTTTTAGTCTTTGCGGCCCCA
GCAGCCAGAGCTCAGCAGGGCCCTGGAGAGATGGCCACGGTCCCAGCACCGGGGAGGACTGGAGAGCGCGCGCTGCCACC
GCCCCATGTCTCAGCCAGGTGATGTCCCCCTGCCTCCCTCCCGGCCCCTGTGGACCAGCCAGAGGGCTGGGAGTGAAAGT
CACAGAGAAGACTTTCAGCTCTGACTCAGTTCCCCCAGCAGTTTCTGCCTGAACTCCCATCCCCCAACTTTGTCTTAGAA
```

```
TTCCCCTAGGGAGGGGTTGGGCCCAGCTCTCATGGCTCCTGGCTGGAGGAAAAAGCGGTCCCCAGTCCCAGGTCAGCTCT
CCTTCCCAACCCATTCAGGGGAGGCTAGGGACTGCTGTGGGATGAGGGGCTTCCCTGGGCTCTGTGTGCTGGGCCCCCAA
CCCGGAGAGGTGGAGAAGGGATGGGTGGATAGCCTGGGTGCCCGGAGAGGGTACGGGATATGGGGTGGGACAGGCGGGAG
CCCCCTCCTGACCTCTGTGGAGTGGGGCCCATATACCGGGGGAGGCAGCCCTGTGTTGCGTGCTCATCAGGAGGCTTGTG
GAGTAAGTGGGGACAGAAGGGTCCCCTTGGAGAGCCATCTATGAAGTGAAGTTGCTGCGAGGCCTCTGGGAGCCCCAGGC
TGGAGGCAGAGCCCTGGGTTTGAGTCTGGGTTTGTCCCTCCCTTGGCTGCATGACTGGACAGGTCAGAAGCATCTCCGGA
CATTGGGGATGGGACCCTATCTTTGGGGCTGCCTGGAGCTCATGCTCTCTCCTCCTGGGGGCCGAAGCCTCCCACTCT
TAACTGTGTTTGTAGCACCTCGGGGGAGGGAGGTGGAGTGAGGAGCTGAGCTATCCCCAAGGGTCCCCTTCCTTCCCCCA
CAGCCAGGGTCCCGCACCCAGCTGGAGAAGCCCCTGAAGTTAAATAGCAAGAGACAAGCACTGTGTATGGGAGCAGGGGG
GTGGCAGGTGGAGGTGGCCAGAGAGGACGAGGGGACATAGGCCCCATTGAGTTCAGAGGTCAAGTTGGATGCACCATGGG
CTCCCGAGCTCCTGCTTTACCACCCTGCCCCAGCCACCATATACCCCTGATGGCAGCAGGGGCCCCGAACTGCACCTGAC
CACGGTTCAAATCCCAGCTCTATGCCCTTCTCGCCTGGGTGAGAGGTTGACCCGTTTCTTAGTCTCCTCATTTGGCAAAAA
GGAGGCATACTAGAGCCTCCTCATGGGGCTGTAGGGAGGGCTAAAAGCTGAACTACCCCCGAGCGCTCAGGGCAGGCAGT
GTAGGCAGAGAGAGAGCAGGCACCAGTCCCTGTCCCCAGCAGCATCTCTCTGAAAATCAGATGGCTGGGGTCAGCCGCTC
CGGGCTTCAGTTCTGGCATGGATCAAATGGGGCAGCAAGACCCACCTTGCAGGGCTGTGGTGGGGACGTAGGCATCCGTG
GGTGTGACCAGTTTCAGAGTCATGAACGACAGGGCACGTGTGGGTGTGCCTGAGGACCCAGGAGGGGGTGCTCAAACTCG
AGGTCCTGGGCTCCACTTGCTGTCACCCGAGGCTGGGCTTCTCCTCACCGTGCAACCTTAAGTGGGTTCCTTGGCCTCCC
TGAGGCACAGCTTCCCCATTGCAGAATGAGGGTGCAGCCTCAGTGACTGCCAGGCGCTCCCTCAGCTCTGTGCCCCTCTG
GCACCCCCACACCATCCTCCCGTGGACTCTGCCCTCTCCGAGCATCGCTGCACCTCTGAGCTCCAGGCGGCGGGAAGCAG
ACTCCAGATGGCGCTTTCTCCTCCTGCCCGGCTGCCTCTAGGCTGGGCCTGTGGTTTACCCCTAACCTTCCTTTGACCTT
GCACCCCTTTGTCCCTCAGCACCTTCCTCACACACTGGGGCTCTCTGCAGTTCCTGTCCTGGGCATGGGGTCATCCTGAG
GCTGGGCCCGACCTGGGGTGACACCCACACAAAACATTGATGGTCCCCTTGCCCCAGGGCCAGGTTGGCATGATTGAGCG
TGAAGGGGCATAGGGGCTATGGGAGTCCCCTGGTTGGGCTATGCACTCTTTGGGGTCCCAACCCTGTGCCCTCTGCTATT
GGGGGATCCCAAGAAAGAGGTGAGCAGCCCCCACCCTGGGGAGCTCACCCTCCTGTGGAGACGGTAGGCCAGGCAAAGGC
GAGCCCATGGTACCCCGAATAAGCACCTGCCAGTCTGTCTGTGAGCTGCTGGGTCCTGACTCCCAGGACAGAGAGGGTGG
CAGCCTTACCTCTGGGACCAGTGTCTGGCAGGGTGGGACAGCATGAGAGAGTGAGCCTGGAGCCCTAGCTCCACCTCTTA
CTGTGTTTATGACCCTGGGCAAGTTAGGGGCTTGGAGCCTTAGTTTCCTCATCTGTAGAATGGGATAATAAAGCCATTGG
GCAGCTTAGACGAGCTGGCATATGTGAGGCACTTGGCCCCAGGCCTAGCATAGAGTATGTGCTCAGTACACAGAACGAGT
ATTATTAGTTCACCAGTGCATATTTACTGTCGTGTGAGCTGTCGTGGTGGGAAGATGCGTCTGGGGAGTTCCAGGAGGGG
AGGATCCCTGCCTGGGCACTGGGCAGTCTGCCCTGACTCAGATTCCAGAAAGGCCTCTTATCCACTGCATGACCTTAGGC
AACGCACTTCACCTCTGTGTGCCTCCGTTTCCTCATTGGAAAATGGGGATAATAAGAGTATCCCCGCCAGAGGCCCTGAT
TGCTGCATTTAAAGAGTTTTAAATGGAGCCTGGCTTACAGTAACCTGTCAGCCGATGGTTTTGGTGTGATTAAGGGGGTG
GCAGAGCGGCGTGGAGTTGGGTTCTGATCCCAGCTCTGTGCTGGGCTCCTGATGTAACCTTCAGCAAGTCTTTCCCACTT
CGGGACCTCAGTGTTCTCAAATGTAGAACAAGGGACTGTGCTTGCTCAGAGAGCCTTTGCAGCTTTGACAGTGCGTGGGC
CTCTGACTCTCACTCCTGTGACATCTGCCTTAGGAGATGGAACCCATGGCTCTTGGGGCCAGGGTGTGTGTCCCTGCTCC
ATGCTGGCTCTGATGTAAGCCACGAGACAATCCAGGGCCACCTAAAGAGCTCTGCTTTTTTTTTTTGCCGTACTGAGGCC
TCTGACCACACTCATTCATTTCACTACATTTATTGAGCACCGACTGTGTGCCCGGCCCTGTGCTCAGCAGTCCCTGGAGC
GAGTGTGCTGGTGGGCCAGCCCCTGGTCAACGCCGGCCCTGCTGCTGCCAGCCTAGAGGGAGGAGCAAGGCGCTCAGAAG
AGGAAGCCCTCGCCCTCCATCACGGAAATCAGGGAAGGCTTCATGGAAAAAGGCATGAGGGCCCGTTTGAAATGGGCTTG
AAGGATGGCAGGATCTGTAGGGGACCCAGGGAAAGGATAGACTTGGGAAGGACCTTTGGAGGGTGAGTGTCGGTTCCTG
GTCACCTGGAGCCACTGGCTTGTGCAGGCAGGTAGGTGCGGTGTGTGTGCTTTGGCCTGGGTACATGCACCTGCTCCT
GTGAGGGCAGGGTCTGGAAGGGTGGGCTGACCTCATTCCGGTCCCAGAAAGCCTCGAAAATGGGAATTTAGGCTTTGATT
GCAACAGGGCCACTGCAGGTTCCTGGGCAGGGATGTGGGTGGTTGGAGGCGGGCTTCAGGGAAGGCAGGTGAGAGCCTGC
AGATGGGGTGGTGCCGTTGTCTGGGCTGGAACGGCACGAGGCTGGCCTGGTGAGGGTGGGGAAGAGACAACAAGTAGTCA
CTGGAGTGTCCTGCTGTCTGAGGAGAGTCCTGACTGGCTAGGTATGGGGGCAAAGGTCACCTGGAATAGGAAGGGTACAG
TTGTCCCAGAACTGAAAGTCAGACAGGAGAAGAGCTGGGGATGAGCTGGGTTTTTGCCGGGTCCAGGAGCATCCCCTGTG
AGGGGGTCCTGCAGACCATCGGGAGGGACGTGGAGGCTTGGGGCTGAGCTGGAGAAAGACCCTGGGAAGCTGAGGGCTGG
AGCCAGTGAGAGGCTCAAGAGAGAGAAGGGAAGGGGAGTGAGAGCTCGAGTTTGGAGCCGCTGGAGAAGGGGGAACAGCG
GGGGAAATGCCCAACGCTCAGGAGCCTTGGAAGCTCTCGAGATGGAAGAGCAGGAGGGAGGGAAGGATGTTCCACCTCCT
CCACATCTCTTGACTCACCTGCTCCTCTCCCCTCTCCCCCTGCCCCTCTGACTGAGGCTACCCTCATCTTTCCCTGGAAG
CTCCCTCCTCATCTCCCTGCCTCCCCCTTTGCCTGCTCCAAACACCTCTCCTGGAAGCAGTCCCCTTATCTTCCCGAAGT
GACCATGTGCTCACTTTCTCATTCCCCTCCGCACACCAGCCCCTGCAGAGGGTGGGGGCAGCTACTTTTCTTTCCCATT
TCCGCTGCGACAGTTGTGGTCTTGCCTCACGGGAGCTTCCACGGTTGGCTAGAGGTTCCCTGAGAAGCAAGTCTTTGTCT
CAAAAAAAAAAAGTAAATAAATAAAGCACTGGCCAGTGGCCAGTGCATAATAGGCACTCCGGGAAAACTTTTGAGCCTCC
CTCTGACAGGGCTGGCTTAGAGATTTCATGTCTCTTTCCTCCCTGGGGTATAATCTGGGTGACCAACCTGGCCCTGCCTC
CAGGGAGCTTCCAGGCAGGTAGGGGAGTACGAGTGTGGACAAGGTCCTCCGTAAAGGCTACTGCAGCCCTCTGTGCCTCA
GTTTCCCCTGTCCTCTAGGGCTCTTCAGTGTTTGGGTCAGAGCTCCTATCTGCTTTTCTGCTTTTCCGAGTTCGCGTCCT
GATGCCTTCACACCATTGCCTCAGCTCCCTCGAGGGACAGTGAGGGGCAGAGGCAGGGGCTAGCCTCAGAGCCCTCTGGA
AGCCCCTCTACCTGCTGACTTTAGCTCTGTCTATATTTATTCCATCACTTGTTTGTTCAAGAATTTTTTTTGGCCAGATG
CCTACTATGTGCCAGGCACTGTTCTAGGAATTGTGGACCTAGCAATGAACAAAACACAAAATACCTGTCCTTAGTGAGC
TTACGTGACTCTGTGTGTGTTTGTGAAGAGAGATAGAGGAAAACATGAATCAGTCAATAAACGATGAATAAATAATGCTA
```

```
TGATGAAAATGTAGGGGGGTGGTGAGTAACAAAGGGTGCTTCTTTTATTTTTTTCTTTGAGACAGAGTCTTACTCTGTCG
TCCAGGCTGGAGTGCAGTGGTGCCATCTCGGTTCACTGCAAACTCCGCCTCCAGGTTCAAGCAATTCTCCTGTCTTAGAC
TCCCGAGTAGCTGGGACTACAGGCACCCACCACCTTGCCCGGCTAATTTTTGTATTTTTAGTAGAGACAGGGTTTCACCA
TGTTGGTCAGGCTGGTCTCAAACTCTTGACCTCAGGTGATTCGTCTGCCTCGACCTCCCAAAGTGCTGGGATTACAGGCA
TGAGCCACACGCCTGGCCACGACGGGTGCTTCTTTATGTAGTCTAGTCAAAGAAGACCTCTCAAAGGAGGCTGCAGTTGA
GCAGAAACTTGAAGGAAGGGTGTGAGCATGTGTAGGGTGGTGGGATTAGCAGGTGCAAAGGCCCTGGGGCTGGACTGAGC
TGGCACCGAGGAGTGCAGACCATGCACACTGAGGGGGCGGGCCCAGCCTGCCTCTAGGGGCAGCGAGGCCTGGGATGTGT
ATTTACGTGTGCTCTCTCTTTCTCCTCTCCCACCAGGGCTTCCTTCCTCGGCTCCACCCTGTGGATGTAATGGCGGCCCC
TGCTCTGTCCTGGCGTCTGCCCCTCCTCATCCTCCTCCTGCCCCTGGCTACCTCTTGGGCATCTGCAGCGGTGAATGGTG
AGGACCCTGACATCTGAACAGCTCTGCTTTCCCTATGGAGAGATGGCTAAGGCCCTGCTAAAGCCTCTTCCCAGAAGGGG
ATGGGGCCAGAGACCAGGTGTCCACCTCCTGCCTCACCCTCTGTCCTGGACCCCTGCCCTGGCATGCATGCATGAATGCG
CACACACACACACACACACACACACACACACGCCTCTCCAAGCCACAGGCTTGTGCTACTTTCTGTCCCTGCCTTG
CCTTAGTTTGCCTGTAGGAACTTGCAGGCTCTGGAGCTCCATTTCCTCCTCTCTGAAGTGGGTCCATGAATCCCTTCTCC
GCTTGCCAGAGTTACCCACCTCCTCCTCTGGCAGGTGTGGGTGCCAGGTGCAGGGGCAGTGGGCGTGTCTTGGGGGGGCT
GTGGGCACCTACAGTCAGCACTCACACCTCATCTTGCCCGGCAGGCACTTCCCAGTTCACATGCTTCTACAACTCGAGAG
CCAACATCTCCTGTGTCTGGAGCCAAGATGGGGCTCTGCAGGACACTTCCTGCCAAGTCCATGCCTGGCCGGACAGACGG
TGAGTCCACTGGCCACACTGCAGAATCTTAGGATGGTGGGATCTCATATTCTGGATCCTATGCACTGGACTCCAAGGAGT
CAACGTTTACAGAATCTTGGACTAATGGGACCCACAGACTTTTTGAATCACAGGAGTTGGGGCCCAGGCCTGGAATCAAG
GGATCGTTTAAGAGAATTTGGGAGTCAGCTATTTAGACTTCTAGAACCTGGGTGGAGTCCAGGCTGCAGTGGAGGGGCTG
GGGATGAACTGGACAGGGTAGTTTGGGGACACTGAGCTGCAAGCCTGCTATGCCAAGGCTGGACTCCACTCTAGAGGCAA
GCAGAGAGGAAGTGGGGAGAAAGGAGGGTCCCCAAGCTTTTTGGATAAGCTCGGATTTTGAGAGACCCTGTAGGCAGCTG
TGGGGACAGGGAACCTGGGAGTGGTTTGGGTGCCTGGTGGATGGGAGAGAGATGTCACTGTGATGTAAACATCCAGGCGG
CCAGTGAGTGAGGGACTGACCCCAGGTAGGCTTGATGGGGTCAGGGAGAAGAGGAAGACCACAGAGAGGTGGCTGGGCCC
AGGTGGATTCCATAGGGTGTGGGGTGGGGGAAAATGGGGAGGGAGATGGAGGGGTAAGGCAGGTTCGGGCCCTGGCTTCT
GGGTGGGTGGTGATTCCACACGGCTCTGAAGGCCTAATTAAGGATCTATCTTCATCCTAAGCCTCATGGAGCTTTGGAGG
GCTCTAGGTGGGGAGGACTGCCTGTCTTGAAGTGGTTTGTGTTTCTAGATCACTCCAGCTGCCGTGGGACCAGGCTGGGA
TGAGGGAGCAGGACAAGGTGCCCATGAGGGAGGAATGCCATGGAGCCAGTGGGGAGGCAGTGCCCCTCACAGTGGTTGG
CACAGGGGCCCCAGCCTGGCCTTGCTGCCTGGTGCCATCCTGGCAGCAGAGAGATCTGAGAAGAGTCCCTTGGGCCAGCT
GTGTGGGTCCTTCCTGTTCTTCTAAAGAGGGTCTTTTGCCATGAAAGGCGGTGGAACCAAACCTGTGAGCTGCTCCCCGT
GAGTCAAGCATCCTGGGCCTGCAACCTGATCCTCGGAGCCCCAGATGTGAGTAGCCCACCCAGGGAGAGAGTGCAGGGAG
AGGGTGGTCTCCCAGCTCCGGGCTGCGATCTCAGGGTGGGCCGGATGCCACGGGAAGAGCTGAGGCCCAATGGCTGGGGC
TGTGGCTAAGCAGGATGCCCAGCGCTGAGCAAGAGGCAGAGAAAAACAGGTGACACCCCAGGGAGTGGACTCAGGCCTGC
CCCAGGGCCGACGGACCCTCTGTATGTGAGGCATCTGCCATTCTGCCTCTGCTCCTGCTGTCTCCCCTGCGCTTTGTGGG
TCCTGTGACACTTGTGTTCCTTATCAAGTAAGACAGCACTCCAGTAACTGTAATGACCACACCCAGCTCTTCCCGGGGGC
TTGGCAGTGCAGTGCAGTGTGGGGGGTGAAGGGTGTAGACTCTGGAGCTGGATGCTCCAGCCCTGGCTCACCAGCTGGGT
GACATTGGGCAACCCCTGTGCCCCAGTCTCCCCTTCTGTAAAATGGGCATAATAATGGCACCTACCTTGTTGGGTCAAGG
CCCAACAAGGGCCTTGTTTTTCAGGCCCTCCCCAGCCTGAAGGTGGGACCTCACCAGGGACCCGCCTCCTTCTGCCCAGG
CTGCTCGGGCCAAGGGGCACCTGCAGGCCAGTGCCAAGCTGTCCCCTGCACCCCGTGGCTTCCCATCCCCCTGTGCTCA
TTGGTGCCTAAAGTCCAGAGGGGGCCGAGGCAGCTGGGGCCAGTGAGTCAGTGCTGCCTCGAGCGTGCCAGGCTGTGGTA
GTGCCTGGGCTTGGTCCCGCTCTGAGATTGGAGTGGGCACTGGAGCAGGAGGAGGCCAGACAGTGGGAGCAGACACCCCT
GAGCTTGTGGGGGCAGTAGAGATGCCTGGGTCCTGGCTTGGGCAGCTGCAGCTGCCCCAGGAGAGCTCCTGGCTACCTCG
GAAGGGGCAGGGGTCCCGCTTGTCCCCGACTCACACTGGATCCACAGAGTGCGTAGCCCCGACTGTGCCTCCAGGATCAG
ATCGGGTGCCAGAAGCAGGGGAGAGGCCAAGCAGTGGGAACAGACAGCTCTGAGCCTGCGGAGGCAAGGGGGGATCTTCC
CAGACCCTGAGAGTGCAGGGATGCCTGGGTCCACAGCCACAACTTGGGTAGCTGCAGCTGTGCCCGGGAGGGTGGGTCTC
CTGCGTGCTCCCTGCTTGCAAGATCACCTGGAGGCTTGGGTTTGGACCAGGGCCCCTGAGAGTGCAGGGACACCTGGCTC
TGTGGCCACGACTTGGGTGGCTGCAGCTGCGCCCAGGAGAGCGGGGCTCTTGCCTGATCCCAGCCCCCAACAGCACCAGG
AGGCCTGGGTCCACAGCCAAGACTTAGGCAGCTGCAGCCCTGCCTGGGAGGGCAGGGCTCCTACCTGCTCCGTGGAGCAG
GAGACCCCAGCTACGCCTCCCGGCTGCAGCCAGAGTTTTAGCAGCGGCCACTCCGGATGGGCAGCTGCGGCCTTCACCTA
CAGCCCCATGGGTCAACATCAGCACTGTCCCCTTCTTACCAATGAAGACACTGAGGCTCAGAGAGGTATGGTCTCTGCCC
AAGGTCACACAGCCAGGAAGTACACAGCTGGGATTGAACCCAGGCCTGCGGGCTCCCAGTTCAGGCTTCTCACCGTTGCA
TTAGGCTGCCTCTTGCAGGGCATATGGCCATGTCCATATGAGTGTACATCAAATTATTGTCTGTCAAAGCCTCCTGGGCG
GCAGCGGGCTCCAAGGACACCCGGACCACATCTTCATCAGGTTATATGTCTTCTTGAGGGGCAGGAGAGTGTGAAGATG
GAGTCACACAGTGTAGCTGATAAAATCACAGCCTCTGGAACCGGATGCTGTTCCCCTTGCAGGGAGCTCTGTAGGTGGCC
TGGTTTCTGATCCTGGCTCCACCATTTCTTAGCTGTGTGACCTCAGGCAAGTCCCTAAGGCTCTCTGTGCTTCAGTCTCC
TCAGCTGTAGAATGGAAATAGTGACAGTGCCTGTGCCCAAGTTGAGAGAATTAAATGAGACAATATATGTAAAGTGCTCT
CAAGAGTGCCCGGCACAGTGTGAGTGATCACGCGAATGTATGATTGGCAATATTTTTATAACAATAATAGTAATAATAAT
AATAATAATAATAATGAGACTACTATCATCGGGGAAAATCTAGCCCCCATAGCAGCTATAAGAAGGCTAGGACAGGGTCT
ATAGGGTGGAGAGGAACTGGCCAGTTTGGGGAATTCCAACGATCAGGTTTAAGGTTTGCCCATAGTGGGTCTTATGCAGA
CCAGAGGGACGGTGCCCAAGGTCCCGGCCCTGAAATCAGCATCCTGGACCCCAGATCCTAAGAGGCCTGCACTAGAGCTT
CCTCCCGCAGGGCTGCCGGGCTGTAGGGTTGGCAGCTGCACAGTGGCAGGATGCCTTAAGCTGCTCACTCGGCAGCCCTC
TGTCAGGCATGACAAGCCAGGACTCGGAGATGCAGTCACCCTGTAGGGGGACACAGAGGGCCTGGGTTTAAACTTGGGCT
```

```
CCCCTGCTGTTCATCTGCATGACCTGGCGCAGGTCCCTTGACCAGCCCAAGCCTCAGGCTTCCCACCCATACCATGGGAT
GGATGCTGTGTGCATTGAGGGGCTAAGGCATCTGAGCAGTGACTGCTCATAGGAAAATGCTCAATACCTGGCCATTTTCT
TCCAGAAAATTCAAGGCGTAGGGATGGGGAGAGATAGGGGAGGGGAGGGCCTTTCACACTAAAAAATGTTAAACAGGTAT
AGTTCCAGAACTGTACCTTTGTGGGGGCTTACCAGCTTGCATGCCTCCTGTAACAGGGAGCTCACTCCCTCTCAAGGTAG
CTCACTTCATTTTAGATCAAGTGTTGGTAAACTACCTTGACCCATAGGCCATATCCAGCCCACTGCCTGTGTTTCTAAAT
AAAGTTTTACTGGAACATGGCCCTAATATTCATTTACATGTTGCCTATGTCTGCTTCATACTACAGTGGCGGAGTTGAGT
CATCGTGACAGAGACCATGTGGCCCCATAAAGCCAAAAATAGTTACTATGTGGCCTTTTACAGAAAACATTTGCTGCCCC
TGCCTTGGGTGGCGGGGCATGTGGAGAGGCTGCCATCCTTCCCAGGTCATCCCGGTGGCCTCCCCCTTCCCTGCCTGAA
GCTGCTAGAAGTTTCACCCCTGCCCTCCTTCCCTTGCAGTCTCAGAAACTGACCACAGTTGACATCGTCACCCTGAGGGT
GCTGTGCCGTGAGGGGGTGCGATGGAGGGTGATGGCCATCCAGGACTTCAAGCCCTTTGAGAACCGTGAGTGAGGAAGCC
AGGGTGGGGCTGGGGCAGGGCTGGGCACCTCCTTCCTTCCACCTCCTCTGGGATCTTCCCTGCTCCAGCCTTTCTCCCCG
GCAGCTTCCACACCCACATCTGATCACCTCACCGCCCGCTGTTCTCGGGATGACGTCCTGGCTCCTTCCTCTGGCATTCA
AGGCCGCTCAGGATCCAGCCCTGGGCCTCCTCCCCGGCACTCCTCCTCTTGAGGCCTATATTTCTGCTACAGCCAAAGGC
CAACTCTCCAAACCCACCTTGCCTATCCCTGCCTCTGAGCCCTTGCACATGCCGATCCCACTGCCTGGAAGGCCCTTTAC
ACCTGGAAAATTCCCACTCACGTTTCGAGGCTTAGTTGAAAGTCACTTCCTCCAGGAGGCCTTCCTAGACTCCCCTAGCC
CTTTGTACCTCCTCTTTCTGGGAATGTATGTCCTGGGTTGTTACTGCCTGATTGTAGGTCTGTCTCTTTCACTGGGCTGT
GAGCACCCTGAAGGCAGAGTCTCTGCCACCCCTGTGTCTCCTTGCCACGCACCCAGCACTAGGTCTCATCAGGACAGTGG
TCTGGGAAGGTTTGTTGAATGCCTGAGCGACTGCCTGCTGACCTTGGGGGGCCATCTCCAGGGAAGCAGTGGGGTGGAAAG
TGAGAGTCAGGCCTGAACTGGATGTGATCTGACTGGCAGACCATCGAAACTTTCTGAGCCTGAGTTTTGGGGAGAATCGG
AGTCACCTACCTACCAGGCTTGTAAGGCATCTTTGAACTGAGACATAGAAACTGCTTACCATGTAGTCAGCACTCAGCAT
CTGCTTATCACTTATGTGGTTCTATAGAGTCTTAGAAGTATAGAAACTGAGATTCATTTAACTTCAGAATCACAGTGTTA
TAATCATCACACTTAAAATAAATGAGATGATCCATGGAAAGCTCTCAGGTCAATTGTAAGTGCAGAGAAAAGCTGCCGTG
AGTCATTCATCCAGCAGCTACTGAATCAAGTGCCAGGTAATCTTCCAGGTGCCAAGGACACAGCAGACAACCAAGTCCCT
GCCCTCAAGGAGCTCACAGTCTGCTGGGGGAGACAGAAATGAGTAAAGAAACCAAACCATATATTTCGTAGGTCAGGTG
ATGATAAGCACTGGGAATAAAAGTGAAACAGGCAAGGGGGATGCAGAGTGGTGAGTGGTCAGGGAAGGCTTCTCTCAAGA
GGTGATCATTGAGAGAGGCCTGGAGGCAGTGAGGGAGCGGGTCCTGGGGCTTTGGGTCGGGGGGTAGGGGAGGTGGTAGC
ATGTGCCAGGAGGGAGGAGTGGCAGGTACAAAGTGGGAGGTGGAGGTCATGGCTGACTGCTCTCCCGTACCCCCTCTTGT
CTCCAGTTCGCCTGATGGCCCCCATCTCCCTCCAAGTTGTCCACGTGGAGACCCACAGATGCAACATAAGCTGGGAAATC
TCCCAAGCCTCCCACTACTTTGAAAGACACCTGGAGTTCGAGGCCCGGACGCTGTCCCCAGGCCACACCTGGGAGGTGAG
AATGTGGCCCAGTCCGTACCTGCCTTGGTTCCTCCTTCCTGTCCTGGTCCATGTCCTGTCCCTTTTTCATTGTGTCGAGG
TCCCCTCAGCCCCAGGTGGATGGTGGGTGGCTGAGTCCAGTGCTCTTTCTGCACTGCCTGGGGCTGCCTCCCCGACTTCC
ACGTCCTTATCTAGCCCCCTTAATCCTGGGGATCCAGCTGGCAGCAGGTTCCAGGCTGACACCAGGGAAAGATTCAGAAA
GCCAGGAGATAGGCAGCTGCCCAGCCCCATGGGCTTTCAGAGGCCAGGGAAGCCAACCCTCCACGTGTCAAAGCCCATCT
GCTCCACATCCGTGCGGGCCTGTTGCTTATTCATTCACTTACACTCATTTCTTCATTCACTCAACTGCTCACTCAATAAA
TATTTGCTGAGCACCTACTATCGCGCTGAATGATGGGGAGAGAGCGGAGGGTGAGCTCAGCAAACTTCCACTCTCAGGAAT
CTCAGACTGGGGCAGGGCAGGCAGGCAGTCAGTCAGCAGATGTAGCGACAGGGTCTGGGATAGTGAGGCACCCGGATAGT
GATAAGTTCCATCAAGAAAACCCAACGGGGCAAGGAATTTGACAGTGACTGGCACAGGCTACTTCAGACTCAGGTGACCA
GGGAAGGCCCCTTGGACACCTGGGTGTCCAGAAGGAGCTGACCTGCAAAGATCTGGGGAGAAGTTTTGCAAAATTAGAAA
GACCTGCCACGGCAAAGGCAGGATGCTGCTATGTGTCTGGGGAGCAGGAGGAGGCAAAAAGAGTGACCCAGGGTGGGAGA
GGTGTGCGGAGACGTCCCACTTTCTGGTCCTGTGGAGACTAGACTGCAGCGGGGCACGGGTGGGAACCAGAGACCAGCTG
CAGGCTTGGGAGAGGGCGGCAGCTCAGGCCTGGGGTGAGTGCAGCGGGGAGAGAGGGGGATGGTTCTGGAATGGCTGTGG
AGGTGGAACCAGGTTCTGGAGGTGGAACCAGCTGATTAGCTGATGACTTGGGCTTTAGGGGATGACCCTGGGGTTTGAGG
CCCCAGCAACTGGGTAGTGAGGGAGCTTTGGGTGGCAAGGAAGTGGGGGTGCAGCTGGCAGGGGTTGGGGGGGGGTTCTG
TGATGCTCAGCCTCCTGGCCCTGCCATGGGAGGTGAGGTGGGCGCTGACAGTCCTGTCTCCGACCCAGGAGGCCCCCCTG
CTGACTCTCAAGCAGAAGCAGGAATGGATCTGCCTGGAGACGCTCACCCCAGACACCCAGTATGAGTTTCAGGTGCGGGT
CAAGCCTCTGCAAGGCGAGTTCACGACCTGGAGCCCCTGGAGCCAGCCCCTGGCCTTCAGGACAAAGCCTGCAGGTACTG
GTGGGCGGCAAGGGTGAGAGGGGGCTCAGGCAGGAGGGGGCGGCTGGAGGAGGAGCTGCTCCATTCTCTCCCTGCCTGGG
GTGAGGGTATAGCAGTCAGCTCCATCCCTCACTTGCTGTAACCCTGCAGGGGGCTCGCTTTTCAGACAGGAACAGGGGTT
CAGTGAGGGGACGGGGCTTGCCATGGGCCTTGTGAGTTGGGGAGGGGTCGAAAGGCTGTTTTCACTCTTCTCCACCCTGA
AATCTCCAACTCCTGGTTTCTTCTGGGGCTGGGGGTGGCTGCTTTCACCTCTCGGCCTGATCTGTGAAATGGGTACAGTG
ACCGTGAGGCAGAGCTGCTGTGAGGAAGGCGTGTGATGTCCCGGCAGCCTGGCCCTCAGCAGGTCTGCTGGGCGCCTGTC
CCTCATCGCACCACATTTCGAGTTCAAGCCCAAGCAAGACCTGGGTCCTCTAAGTGGTGCTTGGAGGGAGGTGGAGAGA
GGGGTGCAGCCAGCTGGAGCCCAGGCTGCTGTGGGGGTGGTGGGGGGCTGTAGGCTCACCAGGGGGGCCCATGGGATCTG
TCTGGAGCTGGAGGAGCTCTTAGAATGCCACCGTGTCATTTCTTTTTAGCATGAAATCTGGCTTCCCAGAGGATGGGCTT
AGCAGAACCCTGTCCCCAGCCCCCCAGACGCAGGCCTGCGTGGCAGGGCCCTGGATGGGGGTGTTGGGGTGACCGACCCA
GGGTGAGTGAGGGCACTTCTCTAACCCCTCTCCTTGCTGTTTCTCCCACCCCTGGGCAGCCCTTGGGAAGGACACCATTC
CGTGGCTCGGCCACCTCCTCGTGGGCCTCAGCGGGGCTTTTGGCTTCATCATCTTAGTGTACTTGCTGATCAACTGCAGG
AACACCGGGCCATGGTAAGAAGGTTCTCCTCCCCTTCCTTCATGCTCCCACCCCTCCAACGTGCACACACATGCACACAC
ATGCACACACACGCACACAAGCACACATACACACACACACTTACATGAACACATGCCACCTTGCCAGGTCAGATTC
TGGACCGAACCCCCTGCTCTGATACACCCGTGCTTCGTCTACCCTCCAAAGAGTCCTCCCTGTCCAAAACCTGGCCTTTC
CCTGTGTCGCATCCCCACAGGGGGACCCTGTGGAACCAACCCGAGGGGAAGGTGGGAGGTGCAGCCCTCCAGGGAGAGCC
```

```
CCGGGGCCCTGTCCAGAACACTGCTCTCAGGACTGGGGATGCCCGTGTGCACATGTCAGCTGTCTCTGTGTCTGGGTCCC
CGCCGGTCCATCTAGGTCTGTCATTCGCTTATTCCCATATTCACTGAGTACGAGCTGCATGCCAGGTACAGCTCAAAGAG
CTGGGAACACAGTGGCAGGCCGCATGCATGCGCCCCAGCTCCAGGCAGCTCACGGCCCAGTGGGGGAAGGCAGACAATCA
TGCAGGTGTGTAATGACACACCGGGATCAGGATGGCAGAGGAGGATGTCGACGTATTGCCATTCCTTAGGATCCATGGAA
ACCAGTTCCAGGACCCTCCCTACCCCCTACAGATACCAAAGCTGGTGGATACTCAAGTCTCTGATTTTTGTTTTATTTTA
TTTATTTATTTTTTGTGAGATAGAGTCTCACTCTGTCACCTAGGCTGGAGTACAGTGGCGCAATCTCGACTCACTGCAAC
CTCCACCTCCAGGGTTCAAGCGATTCTCCTGCGTCATCCTCCCGAGTAGCTGGGACTACAGGCACACGCCACCGTGCTCT
GCTAAGTTTTTGTATTTTTAGTACAGACGGGGTTTCACCATGTTGGCCAAGCTGGTTTCAAACTCCTGACCTCAGGTGAT
CCACTCGCCTTGGCCTCCCAAAATGCTAAGATTACAGGTGTGAGCCCCACGCCCGGCCTCAAGTCCCTGATTTAACACA
GCAGAATATCTGCGTGTAACCTACACACACCCTCCCAGAGACTTTAGGTCATCTCCTAGATTACTTATAATACCTAATAC
CATGTAAATGCTATGTAAATAGTTGTTATGCTGTATTTTAAAAATTTGTATTATTTTTTATTATTGTATTGCTATGAGGG
TTTTTTTTTCCTGAATATTTTTGATCAGAGATTGGCTGAATCCGTGAGTGCAGAACCCACGGATCCTGAGTGCTGACTGT
ATCAGGAGCAGAAAACAGGGAGGGGAAACTTGGCTGGGGCGTGGCCAGGGCTGGTTTCCCTGAGGAAGGGATGATTCTGT
TGAAATCTGTAGTGGGGAGCCCATGGGGGGTGTGGAGGGGATGGGGAGAGTGTCCCAGGCAGAGGAAACAGCATGTGCAAA
GGCCCTGGGCAGGCAGGAGGGGAAGAAACTTCCAGGAACAGAGTGCAGGGGCGCTGGTGTGGCTGGAGACAGAAGTGGGTG
GGGCAGGAGGCTGAACTTGAGCCTGGGCCTGTGGCGGCAGTGATGCTTCCTGGGGCCTCAGGCTGTGGTGAGGACCTGGG
CCGTGTGCCGAGCAGTCATGGAGGGTTTCCCCAGAAGTGGGACAGGATCCACTTCGCCCTCTGTAGCCCTGGCTGCTGTG
TGGAGCGTGGCCTGGTGGAGGGCAAAGCGGTGAGGAGGTGACACTTCTGACCGGGGGTTGTGGCCCCCTGATGAGAGCCC
TGCTTCTCCCAAGAGGTTTGCAAAGCGTGCCTAGCTGTGTCACCTCACTCGACTTTCCCCAGCACTGGGAAGGCAGTGAG
TCAGGGGCTACCATTGCCTTTTAACTGATGCCAGGACTGGCCAGAGAAGTCAGCTGACTTGCCCAAGGTCACATAGCCAG
TTAGGAGTGACTGCCGCTCTCCTGACCCTCGTCCTGGAGCAGTGGTTCTCGGCAGTGTTCGGAGCCCCGTTTCCTCTTTC
CTCCCAAGAAGACAAGCATTCCTCCACCCACCCGTCCCCTCCGCCCACAGCTCCCTGCAGGCCTTCAGGGAAACCATGTC
CCTGTAGAGGGAGAGGTCCTCTCCCAGGCATGGGTTCAGTGGCCTCCAGACTGCCCTCCTCCTTGTCGTTCTTGCGCCCC
TTCAGTGCTTTCTCCACCATGTCATTCTCCTGTTTAACACTCTTCAGAGGCCGCTCACTGCGCTGCAGTACACCCTGGCC
CTTGAGGGCCTCCACCAGCTGTGTGGAACCTTTGGGCAAATCAAAAATGGTGCCTCTTTCTTCACGGTGATGTACACAGC
TTCCAGGCACTCAGCTTAGCAAGCAGAACATGGGCTGGAATTCAGCCTCACTCACCACCACCTTTCCCACTCCCTGCCCA
AGCCTAATGCCCTTGGGCAGTGTACAACCTGAACCACCATCCATGGCAGCCCTGACAAGAAAGCCCATCTCCTCTCTCCC
GCCTCCTGGTTCATTCCACTTCTTCTAGCCATCTCCGTCCCTGACCACTCTGGCTTGTGTGTTCACCCCATCGACCCCC
ACCTCCCCGTCTTGTGTAGCACTGTGCTCTCTGTCTCCACAGAATGAATGGTGATGTTGCAACATGCTTTGTGTCCATCT
CCCCATGAGAATGTCAGCTCCTTGAGAGCAGGGACCTCCTTTGTTGGCCATTCTTGTCTCCCCAGTGCCCAACACAGCGT
CCTCCCACAGTGAACACTTAGTATCGGCGGAAGAAATACATAAGTAAATGTGGTGTGGCTCCGTGCCTGGCATCCAGAGC
TGTGGCGCCGATGGCATAACACCGCGGTCCTTCCCTTTCTCACCCTCCTCCTTCCCTCCTCTCCTCTGTCCAGGCTGAAG
AAGGTCCTGAAGTGTAACACCCCAGACCCCTCGAAGTTCTTTTCCCAGCTGAGCTCAGAGCATGGAGGAGACGTCCAGGT
AGGAGGCCGGGGCGGGGAGACAGAGGTGGGCAGGGGTGTGAGTGGGATGAGGGGGTGGCCGGTTAGTAGCTAATTTTGTG
TTTTGTGAGAGGAGACAAAACGCCGAAGTGAGCAGCCTCCGGGGTGTGCATGGTCTCTTCTCCAGCTCTGGCACTTTCC
ATCTGAGCCAGACTGCCTGGGTTCGATGAGGAGGAAAAACAGTGTTACTATGTGTCCTGCCCTGAGTACATTGCCTGGCAC
GCAATTAGTGTTCAAAAGAACCCTATCAGGCTGGGTGCAGTGGTTCATGCCTGTAATCCCAGCACTTTGGGAGGCCGTGG
CAGGTGGATCACGAGGTCAGGAGTTCGAGACCAGCCTGACCAACATGGTGAAGCCCTGTCTCTACTAAAAATACAAAAAT
TAGCCGGGCTGGTGGCGCATGCCTGTAATCCCAGCTACTCAGGAGGCTGAGACAGGGGAATCGCTTGAACCCAGGAGGCG
GAGGTTACAGTGAGCTGAGATGGCACCACTGCACTCCAGCCTGGGCGACAGAGTGAGACTCCATCTCAAAAAAAAAAAAA
AAGTACCCTATCTGGTATTACCGTTGCCGGTGTGCTGAACGAACAGGACCTGCCAGCACAAGCTCTCTCCACCCCACTGT
CTGTGCAGACATGCTCTGTGCTTCTCCTGTCCCTGCTCAAAATTCCTGAGCAGGCTCCATAACTTAGCGTGGATACTAAC
AAGTCTTCCCGTCACTTTGAGTCACAAAACACCGATTCATTTTTAAAGTGCCGGCCCGCAGATCAGTAACGCTCTAAACT
CCATCTCAGATTCCGTCGTCTCCTCTTCCCCAGCCCATGCCCCCTTCAGGCAGGAAGCTTCAAGAATGAAGGCGGGGACAT
GGGGGGTACTTTTTACCACCCTAGCCCCACTCTAGGCTCCCAAGATTGGGGGGTGGGGATGAGGTAGGGGCAGAGGGAGG
TCAAGAAGAGTGGCGGAGGAGGAAAGCCATGCCGGACCAGAGCTGTCATGACCTGGCTTTGGAGTTCTCTATCAGGGCTG
ATCAAGGGCCCCTTCTGGAACTCCTTGGAAAACTCCCAAGATGAGAATATCTTAGTTGCAGTGCTTTTATTTATTTATTT
ATTTATTTTTTGAGATGGAGTCTTGCTCTGTCGTCCAGGCTAGAGTGCAATGGCACGACCTCAGCTCACTGCAAACTCTG
CCTCCTGGGTTCAACCGATGCTCCTGCCTCAGTCTCCCGAGTAGCTGGGACTACAAGCACACACCACCACACTTGGCTAA
TTCTTGTACTTTTAATAGAGACGGGGTTTTGCCATGTTGGCCAGGCTGGTCTTGAACTCCTGATCTCAGGTGATCTGCCC
GCCTCGGCCTCCCAAAGTGCTGGGATTACAGGCGTGCGCCACCGTGCCTGGCCATTAGTTGCAGTACTCTTAATGTGCGG
GAATGTATTTCCTTAAAATTCTTCCCTCAGCCTTGAGGGTAGCAAGATTCCCATCTATAAAGGTATTCAAGCAAGCCATG
GTTGCCTATGTGTCAGAAGCATAGAGATTCTCCCATTGAACTGGTTGGAGGCTGCGGTAGATTAGCTGCATGCTCAGATA
CCTTCCAGCTTCAAGTCCTGATTCTATGAACCTACAACATCGTGAGGTGGGAGCTGCAAACCATTCCAGCTTCCCATGAG
GGTATGGTATCCTTGGGTCTAGTGTGTGGAGCGGGGTCCTGAGGAAGAAGGCATGATGTGGCTGGGGGTTGGGGATGGCA
AAGCAGGCCCCACCTTTTCTGCATCTGGGTGAGAGACCTACCCCTCCACCCTAGGACCCACAGGGGCGTCACAAGGCCCACC
CCCACTTCCCAGGGGATCGGTGGAACGTGTGTGCGAGTGTGGGAGTATGTGTGAGGGGCGTGAGTGTCTCCTGAAGCCCCT
TTCAGGGCATTGTGGGCAGAGGCTCAGGCCCAGGAAGTAGCTGGCCACAGGCAGTGGGCCCCTGGCTCACCGGCACTGGC
CACAGGCCTGGCCGCTCCCTCTCTGGCTGCACACACAGGGCTGCTGTCTGGCCCCGCCCTTGCCCTGGGTATCATGAATC
TCACCCGACCAGTGCTTCTTCCTCTCTGCCCAGCCAGCGACCCTCTTTGCCATCCTCAGTTTATCCTTTCTGCCTCTCTC
```

```
AGCCTTTTCTTTTGTGACATAACTCCTGGTTGCAGAAAGGGGGCGGATTTAATTGAGTGGTGTGTGCACGTGTGTGCATG
TGTGCATGCATGTAAGCGTGTGTGTGTGTGTCTGCGGAGCAGCTGGCTCAGCCTCAGGGACTCTCCCTGGAGAGGAGAAC
TGGGCATTTGTTGCTGGTTCATTAATTCATCTGAAATAGATCCATGAGACCCGCCATGCTGGGCACAGAGGCCTGGGGAG
CCCATTGGGCCTGGCCCCTGTGCTTAGGGGGCCATAGTCCAGCTGCTGAGGGTTCCCGGGACAGAGGGTTGTCAGGGCTC
CCAAACTCACCCACCCGAGGCCCCTGCCACACCACCAGGAGGGCCTGCTGTCCCCCACTCTGCTGTGCAGTCTGTGTCTT
ACCCGTTTTGGGTGTTTACATCTCCATTTGCTTACCTGATGTCTCCCTGGTGACCTGATGGGTGACAGCAAGGAAGTCAG
GAGAGACGTGGCTGTTGTGGAAACTGAGGCCCAGAGAGGTGCAGGGGCCCTCCCAGGGTCACACAGTTAGTGAGTGCCAG
GGCCAGGGCTAGAACCCGGGTCTTCCCACTCCTTGCCAGGGCTCTCTGGAATCCCGAGCAACTGAGAAGTGCTCTGATGG
GCACTTTCCTCTCTGGGGATGGGAGGCGGAGGGGGTGGGGGTGGGGACTGCAGGGAACTGGGGGAAAGGTGAATCAGG
AAACCATCAGCTCCCCAAGAGACGTTCCCACCACAGTGTAAGCGGAGACCCTGGGTCCCAGCTCTGCCGCCTGCTGGTC
TGTGATCGGGGCCAGGGCATGTCCCGTCTCTGGGCATCTGTAGGGTGAACAGGCTGTGCTCTCAGTGCCCTTCTGCTTTG
GCATGGGTGGATCGATTCCCACACTTGTGCTAGTGCCCACTAGACAGTCTGAGGATTCTCTCCTTTCCTGGGTCCCCAGC
ATCTAGAACGGTGCCAGGCTCATAGCAGGTGTCCAATAAACAGTCACTCTTGAGCAATCAGACATTCCTGGGCACTACTG
GCAGCCAGGCAGGCCTGTGCTGGGCTGTGGGTCAGGCAGTGTCCCTGGGCTGGTGGCCCAGAGGGCCCCCAAGCCAGTGG
GTGGGTTGGGGGAGTGCGGGGCTATAATCAGGGAGAGCTTCCTGGATGGGGCAGCCCTGGGCCAACTGCTTCAGGAGGAG
GAGGAGTTAGGAGCTGTTGAGAGTCCAGAGGAAGGGGTGGAGGGTGAAGGAAGCCCCCACTCACCCACCCCTGGCCTTTC
TCCCACCAGAAGTGGCTCTCTTCGCCCTTCCCCTCATCGTCCTTCAGCCCTGGCGGCCTGGCACCTGAGATCTCGCCACT
AGAAGTGCTGGAGAGGGACAAGGTGACGCAGCTGCTCCTGCAGCAGGACAAGGTGCCTGAGCCCGCATCCTTAAGCAGCA
ACCACTCGCTGACCAGCTGCTTCACCAACCAGGGTTACTTCTTCTTCCACCTCCCGGATGCCTTGGAGATAGAGGCCTGC
CAGGTGTACTTTACTTACGACCCCTACTCAGAGGAAGACCCTGATGAGGGTGTGGCCGGGGCACCCACAGGGTCTTCCCC
CCAACCCCTGCAGCCTCTGTCAGGGGAGGACGACGCCTACTGCACCTTCCCCTCCAGGGATGACCTGCTGCTCTTCTCCC
CCAGTCTCCTCGGTGGCCCCAGCCCCCCAAGCACTGCCCCTGGGGGCAGTGGGGCCGGTGAAGAGAGGATGCCCCCTTCT
TTGCAAGAAAGAGTCCCCAGAGACTGGGACCCCCAGCCCCTGGGGCCTCCCACCCCAGGAGTCCCAGACCTGGTGGATTT
TCAGCCACCCCCTGAGCTGGTGCTGCGAGAGGCTGGGGAGGAGGTCCCTGACGCTGGCCCCAGGGAGGGAGTCAGTTTCC
CCTGGTCCAGGCCTCCTGGGCAGGGGGAGTTCAGGGCCCTTAATGCTCGCCTGCCCCTGAACACTGATGCCTACTTGTCC
CTCCAAGAACTCCAGGGTCAGGACCCAACTCACTTGGTGTAGACAGATGGCCAGGGTGGGAGGCAGGCAGCTGCCTGCTC
TGCGCCGAGCCTCAGAAGGACCCTGTTGAGGGTCCTCAGTCCACTGCTGAGGACACTCAGTGTCCAGTTGCAGCTGGACT
TCTCCACCCGGATGGCCCCCACCCAGTCCTGCACACTTGGTCCATCATTTCCAAACCTCCACTGCTGCTCCCGGGTCCT
GCTGCCCGAGCCAGGAACTGTGTGTGTTGCAGGGGGGCAGTAACTCCCCAACTCCCTCGTTAATCACAGGATCCCACGAA
TTTAGGCTCAGAAGCATCGCTCCTCTCCAGCCCTGCAGCTATTCACCAATATCAGTCCTCGCGGCTCTCCAGGGCTCCCT
GCCCTGACCTCTTCCCTGGGTTTTCTGCCCCAGCCTCCTCCTTCCCTCCCCTCCCCCTCCACAGGGCAGCCTGAGCGTGC
TTTCCAAAACCCAAATATGGCCACGCTCCCCCTCGGTTCAAAACCTTGCACAGGTCCCACTGCCCTCAGCCCCCACTTCTC
AGCCTGGTACTTGTACCTCCGGTGTCGTGTGGGGCATCCCCTTCTGCAATCCTCCCTACCGTCCTCCTGAGCCACTCAG
AGCTCCCTCACACCCCCTCTGTTGCACATGCTATTCCCTGGGGCTGCTGTGCGCTCCCCCTCATCTAGGTGACAAACTTC
CCTGACTCTTCAAGTGCCGGTTTTGCTTCTCCTGGAGGGAAGCACTGCCTCCCTTAATCTGCCAGAAACTTCTAGCGTCA
GTGCTGGAGGGAGAAGCTGTCAGGGACCCAGGGCGCCTGGAGAAAGAGGCCCTGTTACTATTCCTTTGGGATCTCTGAGG
CCTCAGAGTGCTTGGCTGCTGTATCTTTAATGCTGGGGCCCAAGTAAGGGCACAGATCCCCCCACAAAGTGGATGCCTGC
TGCATCTTCCCACAGTGGCTTCACAGACCCACAAGAGAAGCTGATGGGGAGTAAACCCTGGAGTCCGAGGCCCAGGCAGC
AGCCCCGCCTAGTGGTGGGCCCTGATGCTGCCAGGCCTGGGACCTCCCACTGCCCCCTCCACTGGAGGGGTCTCCTCTGC
AGCTCAGGGACTGGCACACTGGCCTCCAGAAGGGCAGCTCCACAGGGCAGGGCCTCATTATTTTTCACTGCCCCAGACAC
AGTGCCCAACACCCCGTCGTATACCCTGGATGAACGAATTAATTACCTGGCACCACCTCGTCTGGGCTCCCTGCGCCTGA
CATTCACACAGAGAGGCAGAGTCCCGTGCCCATTAGGTCTGGCATGCCCCCTCCTGCAAGGGGCTCAACCCCCTACCCCG
ACCCCTCCACGTATCTTTCCTAGGCAGATCACGTTGCAATGGCTCAAACAACATTCCACCCCAGCAGGACAGTGACCCCA
GTCCCAGCTAACTCTGACCTGGGAGCCCTCAGGCACCTGCACTTACAGGCCTTGCTCACAGCTGATTGGGCACCTGACCA
CACGCCCCCACAGGCTCTGACCAGCAGCCTATGAGGGGGTTTGGCACCAAGCTCTGTCCAATCAGGTAGGCTGGGCCTGA
ACTAGCCAATCAGATCAACTCTGTCTTGGGCGTTTGAACTCAGGGAGGGAGGCCCTTGGGAGCAGGTGCTTGTGGACAAG
GCTCCACAAGCGTTGAGCCTTGGAAAGGTAGACAAGCGTTGAGCCACTAAGCAGAGGACCTTGGGTTCCCAATACAAAAA
TACCTACTGCTGAGAGGGCTGCTGACCATTTGGTCAGGATTCCTGTTGCCTTTATATCCAAAATAAACTCCCCTTTCTTG
AGGTTGTCTGAGTCTTGGGTCTATGCCTTGAAAAAAGCTGAATTATTGGACAGTCTCACCTCCTGCCATAGGGTCCTGAA
TGTTTCAGACCACAAGGGGCTCCACACCTTTGCTGTGTGTTCTGGGGCAACCTACTAATCCTCTCTGCAAGTCGGTCTCC
TTATCCCCCCAAATGGAAATTGTATTTGCCTTCTCCACTTTGGGAGGCTCCCACTTCTTGGGAGGGTTACATTTTTTAAG
TCTTAATCATTTGTGACATATGTATCTATACATCCGTATCTTTTAATGATCCGTGTGTACCATCTTTGTGATTATTTCCT
TAATATTTTTTCTTTAAGTCAGTTCATTTTCGTTGAAATACATTTATTTAAAGAAAAATCTTTGTTACTCTGTAAATGAA
AAAACCCATTTTCGCTATAAATAAAAGGTAACTGTACAAAATAAGTACAATGCAACAAAATAGTGTTGATATTCATTGCC
TGCGGTAAACCTGTTGTCTCTTAAAGGGGGAAATGAGAAGCTGCTGGGGAGCTGTCGAAGACATCCCAGGGCCAAATGCAA
ACTTCCTCCTTGAGAGAAAGGAAAAGGATTGTAAAGGGAATATCTTTTTCACTAATAGGAGTCAGGACTGTCTGTCTTAT
TCTGTGTCCAGGTATCAGCTCATGGCCATATCCCACAAGTCTATGCCTGGGGAAATATTGCACTTGATGACGAATCTCTC
TCTCTCTCCTCTCTCTCTCTCTCTCTCTCTCTCTCTCTCTCTCTCTCTCTCTCTCTCTCTCTCCCCCCCCTCTCTATCCCCC
TCCCTCCAGATAGGTAGATAGATGATAGATAGATAGTGTCCTGATAAAAGTTGGACCAGACCCAGGGATGGCAATTCCTGG
CATTCATAAAGCCACACTCATGGCAGACATTACTAATCAATTGCACCCATGCACGGCAGACATTTCTAATTGAACACAGC
TCTCCTCCTGCAGAGCCTGGGTGCAGCTTCAGATTCTGCAATGCTGCGCTATAGGCAGCCCCCAGTTGGTCACAGCTGAT
```

```
AGCTGATGACATCTCTGGACCGCATAATTCCTCAGGTTCCTAACACAAAAGGAGCTCTTAGGTTTCCCAACTCCGATTTC
CCAGGCAGGGAAAAACTGAAGCTGAGAGGATGGAGACTGGAAAGCTGAGAGGGACCTGTGAGAGGCAAGCCCAGGGAGCCC
AGCTGTCCCCACCATGTCTGGTGAGAGACTTGAGCTGGCAGGGAGCAGAGTCAGGGCCACCTTATACCGGGTGCAGATGA
AGGGATGGGCCAGCGGTCACTGTGTGTCATGCCCAGTGTGGATCCGAGTGTGGATCTTGGGAGAGGAGACAAAGAGGAAG
CAGCTGGAGGCAGGGGTGGTGGTCAGATTGCAGGAGGGGCCTGGGTCAGATTCAGGAGACAGGAGCGGCACCAGCTGGGG
ACCGGGCAGCCGCTGGGGCCCTGGAGAACCGTCAGGTACAGAGCGGGAAGCAGGAGGCAGCCAGGGCAGATCTTGGGGGT
AAGAGAAAAAGGAGGAGAGGTGGGGGGAGGGGGGGGCCTTCCAGCCTCTCATGTTTCCTCTTTGTTGAACTAGGTTTGTT
ATTTTGCTTCCTCCGTTCCCCCACCTCCCATCTCTCATTCGAAGCAACTTCAGGTGCCTGCATTTCCCAAGTAAATTATG
GTGGAAACTGTAGGCCCAGGTCCAGGATGAAGCACCCACCACTGTCCTCCTCATTACTGACAGCTGAAAAAACAGACCAA
GGAGGACAGTAAGAAGGGGCAAGAGAACCAATACTTCCACTCTACACATTGTGCCAGCATGGCCTGGGCGTCATCTCTCA
TCATCTGGAAACAACCTTGAGAAGCAGGTGCTTTATTCCCCATTTTGCTGATTAGGAAACAGGCCCAGGGAGGTGGAGCA
ACGCAGCTGATGGTTGGTAGCATCAGGATTCAAACTCAGGTACATCTGACTCCGGAGCTTTTGGCCCTTAGCATACAGAA
GGCTACATAGGAGATAATTTATACTAATGGGAAAATACCTGGTAAAATTTCATTCTAAGCTTCCTGGCAGTCAACTTCGGG
GGGAAAATAAGATGCCATGATCCATTAGTGTGATTGTGGGAGAAAAAGCAAAGCCCTTTGATAGTTTGGCAGGTAGATGG
GGCTGAACGGTGGCCAGGAGAGAAGGCTGAGGGCGATGCTGGTTGGTCAGGCCGAGGTGGTCTTACCTCCTCCTCCTCCG
TAACTGCTGAGCCCCTCCTACTGCCTCCACCCTCCAAGCTGGTCCAGTGTCCCTCTGGGCCAGCATTTCACTGGCTTCTC
CCACCCCCACCCTGGTGACCTCCGTGCCACAGACCTGACGCCATCACCTCTCACTCGTATGCCTGCTCAGCCTCCTCCTC
GGTCTGCCTGTCCCCACAGCCGCCTGCCCCTCTGATCCTTCCACCTCTGAGCCCTGCCAGGCTGATCTGGCTAAAGGTCG
TATCTGGCCACACCACATCCTTGCTAAACCTCCAGTGACCCACTGTCATTTTCAGGATAATGGCTGAGGTCCTTAATGTG
ATCGTCCATGGCTTGGGGGACTTTGGGGAGGTCCCATCTGCTCTTATGCCTCAGTTGCCCCATCTGTGAATTGGGCAGGC
AGATTGGATGATTTCTAAGTGCTCTCCTAGCCCTGACATTCTGTGGTTCCAGGTGCCACATGGAATATGGCTTAAGGCCT
GGGCTACATGTATTTTACATCAAGAGTTCACGCTGGGGTTGGGGGGTGGGGATGAGAGTAGGGATTGCAACTGACTCATT
TGCTTGGCACCTGCTGATCCCTGGGTTTGAAGTAGCCCTGCCCTGCCACCCAAGCTATCTACCCTCAGGTTCCTATGCAT
CCCTCAGAACCCCACTCAAGCATCTCTGCTTCTGGCAGGCCCTCTGATTTGCCCCCAAGTAGAGTAGATTACTTCCTCCT
ACTGTCCCCCTCCTGAGCTACCTCTGTTATAACCAACTGTTGTGCCGTGGGTCAGAGCCTGGGCTAGATTCTGGGGATCT
AAGGGTGATACTAATAGCTTTGGAAGAACATCAGGTAGAGCCCCGTTAGCTCAAGACTTTAGTCAGAAAAGCCAGGCACA
AGAAGACTCTGAAAGGACCTTCTAATTGGGGAAGGAGGGTGGAATGTTGGGTGGAGCTTTAGCAAGGGGGCTCAACTGGA
ACTCAGCTAGTTTTGGTTATAGTTGATTGAGGATGGTGCTTGGAAGAGAGAGGAATTGTTCTTGCAGATTAGCAGCCACT
ATTTGACAATTTGGGCTTTATCAACAGGCAGTGGGGGAGGGACAGCTTTGGGGTCAAGGATCTGTGAGAGCCACTGTGGA
ATGTTCTCGGTCCTTCAAAGTCTTCGAGAACTGCATGTATTGAGCACTTAGATGCGTCAGACTCTGTTATAAGCACATTG
ATCCTTCCACTCTCTGGAAGCATCCTTAACTCACTGGATCCTTAAGCAAATGCCATGAGGGAGGTACTATTCTTAGCCCA
TTTTATAGCTGAGCCTATGGCTCAGAGTGGCCAAGTGACTTACCCATGATCACACAGCTAGAATGTAGTGGAGCAAGTTG
AACCCAGGCAATATTTAGCACCCACATGGCACAGCTTCTTACAGCTTCTAAAGGGTGTGCACCCCTGGTATCCCACACAA
ACACACAGTGACCTACCAAGATAGGAATGACAGAGGTTCTAAGTCTTGTTCCTCAGTAGAGGCCCAGAGGCCCTGAGGGG
ATGGCGGTGCCGTGTCCCGGGCCACACAGTGCGTTTGGTAGTGGGGGAGGGAGTGTTGATTAAATTTGAGCTCACCACCA
GCACCTGTGACAGGCCTGGGACATCCCCTAGGCTGACTTGCTTAGAGACATTTTCCACCACCAGGGCTATGACACACTCG
AGGCTCCCATCACAGAGCCTGGCAGAAAGTGAGGCAGGAAGCGCTGAGTGGTGAGAGACGCAGGTCGGGTAGTGGAGGGG
GGAGTGCCCAGTCTACGATTCCAGGAAGCCAGGATGCAGACGGAGGGAGGAGCAGAGCTGGCAGACAATGTCTCATCTCC
CATTCCTCTATTTACCTGGCCTCATGCTGGGCACTGGCCACAGAGAGGAGGCCGGCTACTGCTCTGTCCACAAAGAGTCT
CCAACCTTGTCTGGGAGGGAGTGACTCTGGAGGACACATACAAGTAGACCAGCTGATCACAGCCCTCCTTCGGTGTGGAA
GAAAATCAACCCGGTCCTGTGAGCACAAGCCTTGATCAGGAAATGGTGCCAGTGAACACTCTGGTGGAGTCTTCTAACCT
GGGGAGGAATTGTGGAATGCCGTGCGGGGCTTTAGCAAGAGAGGCTGAGCCAGAGTTCAGGTAGTTTTGGATAGCTGCTT
GAGGATGGTTCTTAGAAGAGGAAGGAATTGTTCATTCATTCATTTATTCATTCAACAGACATTTAGGAGGGCCTAGTACC
TGCAAGTCACGGTGTTGGGGATCCAGCAGCGAGCAAAGCCCCCTTCATTCTACACTTTCAGCCTTTGTGGACACCTTGGG
GCAGTGTGGCATAGTGGTTATAGCCTTAACTCTGGAGCCCCGCAGCCGGGTTAAGTACTGGCCCCCTCACTCTGGAGCCC
CGCAGCCGGGTTAAGTACTGGCCCCCTCACTCTGGAGCCCCGCAGCCTGGGTTGGTTAAGTACTGGCCCCTGCTGCCTATCAGCT
GTGACTTTGGACAAATACCTAAGCTTCTCCGTGCCTCAGTTTTCTCATCTGTAAAAGGGGACAATACCAGGACTGTTGTG
AGGATGAAAGAAATAATTGAATGTGGCAGGGTGCTGTGGCTTGCACCTGTAATCCAGCACTTAGGGAGGTCCAGGTGGGA
GGATCCCTTGAGCCCAGGAGTCCAAGACCAGCCTGGCTAACATGGTGAGACCCTGTGTCTACAAAAAATATATACAGAAA
ATTAGCCGGGCATGGTGGTACATACGTGTAGTCCTAGCTACTAGGGAGGCCGAGGTGGGAAGATCCCTTGAGCATAGGAG
ATTGAGGCTGCAGTGAGCATTGATCGTGCCACTGCACTCCAGCCTGGGCAACAGAGTGAGGCCCTGTTACAAAAAAAGAA
GTAAGTTAATGTAAAGTACTTAGGAGAGAAGAGCTAGCCGTGGGAGAGACAGACAGAGACAGGGCTGTGACCAGGGCAGC
CAGGGGCCAGAGATGTGGAAGCTGCTGACTTGCTGGCTGGCCTTTTGGATACTCTGTTGCTGTGTTGGTGATGGCTGTTT
GGGGCAGGGGATGAGCTGGACACCTGCAGGCTCGGTGTCTCCTGCCACCTCCCTGCCCTTTCCAACCTCCTATCCCTGAC
AGCTCATCTCCCTCAAGCACCTGGGTAGGTCTTGGGGGATGATGGTTTGAGATAAGGCCCAAATCTATGATGACAAGATT
CTAGGAGGTGTGACAAATGCTGAAGGAAACCTATGAGGTCTTCTTCCTACCTCTCAAATCCAGCCGTGCTGGCTGCCACC
CGCCTAGCTCAGGGCCGAGGACATTTTGTCCCTCCCAATTCTGTACCACTTGGCCCCTTTGAGCCTCAGTCTCTTCATCT
AAAATGGGGTGAAGAAGTGAAATTTCAAAGGCCGGGTGCGGTAGCTCACTCCTGTAATCCCAGCACTTTGGGAGGCCAAG
GTGGGTGGATCACCTGAGGTCAGGAGTTTGAGACCGGCCTGACCAACATGGTGAAACCCCATCTCTACTAAAAATACAAA
AAAATTAGCTGGGCATGGTGGCCTGCGCCTGTAGCCCCATCTACTCAGGAAGCTGAGACAGGAGAATCACTTGAACCCAG
GAGGCGGAGGCTGCAGTGAGCTGAGATCGCGCCACTGCACTCCAGCCTGGACAACAGAGTGAGACTCTGTCTCAAAAAAA
```

```
AAAAAAGAAAAAAAAAAAGATAGCCCACCCTGTGCCCAGTAACTACTACTCATTCATTCTAATCTATTTATTGAGCTTCT
ACTATGTGCACTGGACTGTTCAAATTGGACATTTCGGTTTGAGAGAGCTCGGGTTGCTTGGAACGGGCTTGATGGTGGGT
GGCTGGCCGTCCCCGGCCAACGGTCCAGTGATTCTCGTAACAGTGGGTGTAGGAGAGCACCCGTTACACTGTCAGTCCGA
GCAATCAGCCAGGAACGGTACATGTTGGCGAGAGAATTCTATTGCTTGTAATTTATGCAAATATGAAACTAACAATGAAA
CCTTTGCTGCTGGGATCCCAAACTCAGATGCTGACAGGGACTGGGGTTTGGGTAGATATTAGCTGCAGTGATGAAATGCT
GACGGTGTACACCCAGTAGATGCGGGCCCCGTTCTAAGTTCCTCACACAGGTTCTGTCAATCTTCACAACACTTAGGCTT
ACTGTCTCATTTTCTATATGAGGAAATGAAGGCCCAGTAAAATCAAATACCTTGTAGCTCAAGGGCATGTGGCCAAATAA
GTGGTGGAGCCAGGACTCAAACTCAGGCTCCTGACTCTAGTAACTCTTTTTAACCCATGCAGTGTGATACAATAGGGAGT
GGTGAGGTCTGTGTCAAACCGAAATTGCTCCAGGGCAGCCATCGCTGATTGTTGCCATGTTGGGGATCAGCTAGGTCATC
TACTTTTTTTTTTTTTTTTAAGACAGAGTTTCACTCTTGTTGCCTAGGCTGGAGTGCAATGGCGTGATCTCAGCTCACCG
CAACCTCAGCCTCCCGGGTTCAAGCGATTCTCCTGCCTCAGCCTCCCGAGTAGCTGAAACTACAGGCACGCACCACCACG
CCTGGCGAATTTTGTATTTTCAGTAGAGACGGGGTTTCTCCACGTTGGTCAGGCTGGTCTTGAATTCCCGACCTCAGGTG
ATCCGCCCGCCTCCACCTCCCAAAGTGCTGGGATTACAGGCCTGAGCCACTGCGCAGGCCTGATCTTCTACATTTTTAAG
AGAAGCTGAAAATTCAGTTTTTCCTGCAAAGTGTCCTTATTTTTAAATATTGGCCACTCATCTTTTAAAAACAGAAAACA
AGCAAGCCAAGCAAAATGTGTCTGCACGGTAGAGAGGGAGGGAGAAAGGAAGAGCATCCTGCGGGATTTATTGTAAAGAT
CACTTCTTCCTGACCCCAGGTGTGCTGGCTGCTCCGTGCCCCCACCCCCTGCTTGCTGAGCTCAGCTGCAACTCTCTCCA
GTTAACGATCCTCGATCCTCCGTGATCCATCCATTATTGAATCCCAGCACCTGCCACAGGTTGTTCGTTGAATGGATGTG
TAGGTTGGTAAATGGGCAAAGGGGAGGAGGGAAAAAGGAAAAGGAGAAAGGAAGGAAGGAAAGGTGGGAGGGGGAAGTTAC
TTAGAGGCTTTGAAAACCTGGAATGACCAAGGTTCTGAGAAACCAACGTGGAGGGAAGAGGCAACAGAGCCGACTGGTAT
CAGGAGCTCTCTCGGGCCCACCAAGTCAACAGCTGTCTGACACAGATGACCAAGGAGAATAGAGGTACTCGGGGAACCTG
AGTGAGTGCTGGGGTCTTCCCATGAAAGGGCAGCAGGAAAGAGCCTCCTTGGGGAAATTTAGGTGTCTGTGACCTTGGAC
AGGGCCCCCTTCAAGGGCAAGAGCTAGTCCCACAAACCGGACTGTCTTTAGAGGAGGCTCTGCCTGCAATGTTTTGAGGC
AAAAAAAAAAAAAAAAAAAAAAAAGTTGAAAATTGCAATTTTGTGTCCTTTGGAAATTTTTGTTGACTCTCTGCCTAAAG
CCTTATGTATATGGAAATAAGTAATATTGTTCTTTTGTTTTAGTTTTTAGAATTCATTTTTGGACAGTATACTGGGAAGA
GTACAGGCTGGGAATCAGGTCACCTGGGTTCCAGTCCCAGCCCTGCCTCAGCATTGCTGTGCAACCTTGGGTAGTCTCCA
CCCACTCTCTGGGTCTGGCTCCCTGATGTGGTTTGGCTGTGTCCCCACCCAAATCTCATTTCAAATTGTAGCTTCCATTA
TCCCCACGTGTAGTGGGAAGGACCAGGTGGGAGGTAATTGAATCATGGGGGCAGGTTTTTCCCATGCCGTTCTTGTGATA
GTGAATAAGTCTCATGAGCTCTAATGGTTTTGTAAAGGACAGTTCCCCTGCACACACTCTTGCCCGCCACCATGTAAGAC
GTGCCCTTACTCCTCCTTTGCCTTCTGCCATGATTTTGAGGCCTCCGCAGCCATGTGGAACTGTGAGGCCATTAAACCTC
TTTTTCTTTATAAATTACCCAATCTTGGGTATTTTTTCATAACAGCATGAAAATGGACTAATATAGTCCCCCTATCTGTT
CTGGGAGCCCCACCTGCTACAATCCCACAGTGGAGGAGAAGGTCAGGCTGAGTGATGCATGCAGAGTTTCCATTCTGTGT
CCCCAAGGTGGCTTGAAGCAGCCCCTCCCAAACCCCAAACCCCTGATCAGCATCTGTACCCCGACTGCCCATCTGGGAAA
GAAGCGACTGGGCATGCCAGGGCCTGGTGGTGAGTTTGTCCTAGCAGCATCTTCAGACAGAGGCCAGAGGCCAGGACCCA
GGGCTGAGACAGAGGCCGAGGGCTCAGGAGTGGTGGTGTGGGCAGTGGGAAGGAACACAGAGACAGGAGAAGGTGTCACT
GGGAAGGACAACTGGCTCTGAAGCCAGAGGGTCCTGGGTTTGAATCCAGCTCCTAGCACTTCCTGACTGTGTGACATCAA
GCAAATCACTGCTCTGAAACTCCACTTTCTCACCTCAGATGGGTGAAGTGACCATTTTTCTTATTGGAGATCAGGCCTAT
CCTGGGTCTGGTTAGCTGTATTGTAAGCCATAGATTATTCTAAGCCTTAGGATCCTTAAATCCCTGTACAGATGGAGGAA
GACAAGCCACAGAGATGGGGACTCCTGAGAGGTCCCACACCTGTTGGTGACAGGCCATTCTGGTGGATTCTTATTCCTCC
AAGCACATAAGAAGCATCTCCATGTACTGCTGAGGGTCCTGTCCTCCACCGGGTGCTGTCACGTGGTGGGCGGGGCCAAG
TGCCTGCCAAGCTTCTGTCCCACTGTCTGTTTCCCTAGGCTTGGCAGCTTTCTTCAAGGGCAGCAACTGAGACTTGTTCT
ATTTCTTTTACATCTTTGTTCTTGTTCTTTCTTTTTTCTTTTTTTGAGATGGAGTCTTGCTCTGTCGTCTAGGCTGGAGT
GCAGTGGTGGATTTCGGCTCACTGCAACCTCTGCCTCCTAGGTTCAAGAGATTCTCCTGCCTCAGCCTCCCGAGTAGCTG
GGACTATAGGCGCCTGCCACCACTCCCAGCTAATTTTTTTGTATGTTTAGTAGAGATGGAGTTTCACTGTGTTAGCCAGG
ATGGTCTTGATCTCCTGACCTCGTAATCCACCTGCCTCGGCCTCCCAAAGTGCTGGGATTACAGGCGTGAGCCACTGCGC
CCGGCCCCCCTCTCTTTTTATTCTTGTCTAAAATCAAAGTGTTGGCAGGCTCATTCTCTCCCTGAAGGCTCTAGAAAAAA
AATCTTCCTTGCTGCTTCCTAACCTCCTTACATTTTTATTTTTTAGAGACAGGGTCTTGCCCAGGTTGGAGTGCAGTGG
CAGGAGCATGGCTCACTGCAGCCTCAACTTCTTGGGCTCAAGTGATCCTCTCCCCTCAGCCTCCCGAGTAGTTGGGACTA
CAGGTACATGCTGCCACACTTGGCTAATGTTTGTATTTTTGTAGAGGCTGGGTCTTGCTATGTTGCCCAGGCTGATCTTG
AACTCCTGGGCTCAAATATTCCTCCCACCTTGGCCTCCCAAAGTGCTGGGATTACAGGCATGAGCCACCTTGCCCAGACT
CCTTATCCTTTTTATGTTATTTTTCTTGTTCTCTTTTGTGGCTGTGTTGAGGCTCAACACCAATCAAATGAACGAACAAG
TGAATTGCAGTAGGGACACCGTGCATTGGAAAGAGATGGCAGAGGTGGCCTCTGGGACCCTGCCTAGGTGGCCTCAGAA
AAGTTGCATCTATTGAGCAGTTCAGGCTTTAGAGGCCTGTGGGAGGTGGAAGGAAGCAGGTCATAACTGTGATTTTGGGG
GAGGAGAGTAAGATGGTGGTAGCTCAGGTGTAGGGAGAGGACACACTTCAGAAGTGACTTGAGGACTCATGGTCATGTGG
AACCTTTGGGACACTTGTCCACAGCATTGTCAACAGCCAGCCTGCCCCTGCCTGCCTCACCCTGCTTAATATGGGCTGGA
TTGTGTCACCCCAAAAGATACATTGAAGGCCTGACCCTTTAACCTATGAATGTGACCTCATTTGGAAATAGGGTCTTTGC
AGATGTGAAGAAGATGAAGTCCTTAGGGTGGACCCTAATCCAGCATGGCTGGTGTCCTTTAAAGCAGAGGAGAAGAGGCA
TGGAGCCACGCACAGGGAGAAAGCCATGAGATGGCAGAGGCAGAGTTTGGA
```

HUMAN mRNA SEQUENCE : hR5-001.1 (Seq ID No: 29)
```
GCAGCCAGAGCTCAGCAGGGCCCTGGAGAGATGGCCACGGTCCCAGCACCGGGGAGGACTGGAGAGCGCGCGCTGCCACC
GCCCCATGTCTCAGCCAGGGCTTCCTTCCTCGGCTCCACCCTGTGGATGTAATGGCGGCCCCTGCTCTGTCCTGGCGTCT
```

```
GCCCCTCCTCATCCTCCTCCTGCCCCTGGCTACCTCTTGGGCATCTGCAGCGGTGAATGGCACTTCCCAGTTCACATGCT
TCTACAACTCGAGAGCCAACATCTCCTGTGTCTGGAGCCAAGATGGGGCTCTGCAGGACACTTCCTGCCAAGTCCATGCC
TGGCCGGACAGACGGCGGTGGAACCAAACCTGTGAGCTGCTCCCCGTGAGTCAAGCATCCTGGGCCTGCAACCTGATCCT
CGGAGCCCCAGATTCTCAGAAACTGACCACAGTTGACATCGTCACCCTGAGGGTGCTGTGCCGTGAGGGGGTGCGATGGA
GGGTGATGGCCATCCAGGACTTCAAGCCCTTTGAGAACCTTCGCCTGATGGCCCCCATCTCCCTCCAAGTTGTCCACGTG
GAGACCCACAGATGCAACATAAGCTGGGAAATCTCCCAAGCCTCCCACTACTTTGAAAGACACCTGGAGTTCGAGGCCCG
GACGCTGTCCCCAGGCCACACCTGGGAGGAGGCCCCCCTGCTGACTCTCAAGCAGAAGCAGGAATGGATCTGCCTGGAGA
CGCTCACCCCAGACACCCAGTATGAGTTTCAGGTGCGGGTCAAGCCTCTGCAAGGCGAGTTCACGACCTGGAGCCCCTGG
AGCCAGCCCCTGGCCTTCAGGACAAAGCCTGCAGCCCTTGGGAAGGACACCATTCCGTGGCTCGGCCACCTCCTCGTGGG
CCTCAGCGGGGCTTTTGGCTTCATCATCTTAGTGTACTTGCTGATCAACTGCAGGAACACCGGGCCATGGCTGAAGAAGG
TCCTGAAGTGTAACACCCCAGACCCCTCGAAGTTCTTTTCCCAGCTGAGCTCAGAGCATGGAGGAGACGTCCAGAAGTGG
CTCTCTTCGCCCTTCCCCTCATCGTCCTTCAGCCCTGGCGGCCTGGCACCTGAGATCTCGCCACTAGAAGTGCTGGAGAG
GGACAAGGTGACGCAGCTGCTCCTGCAGCAGGACAAGGTGCCTGAGCCCGCATCCTTAAGCAGCAACCACTCGCTGACCA
GCTGCTTCACCAACCAGGGTTACTTCTTCTTCCACCTCCCGGATGCCTTGGAGATAGAGGCCTGCCAGGTGTACTTTACT
TACGACCCCTACTCAGAGGAAGACCCTGATGAGGGTGTGGCCGGGGCACCCACAGGGTCTTCCCCCCAACCCCTGCAGCC
TCTGTCAGGGGAGGACGACGCCTACTGCACCTTCCCCTCCAGGGATGACCTGCTGCTCTTCTCCCCCAGTCTCCTCGGTG
GCCCCAGCCCCCCAAGCACTGCCCCTGGGGGCAGTGGGGCCGGTGAAGAGAGGATGCCCCCTTCTTTGCAAGAAAGAGTC
CCCAGAGACTGGGACCCCAGCCCCTGGGGCCTCCCACCCCAGGAGTCCCAGACCTGGTGGATTTTCAGCCACCCCCTGA
GCTGGTGCTGCGAGAGGCTGGGGAGGAGGTCCCTGACGCTGGCCCCAGGGAGGGAGTCAGTTTCCCCTGGTCCAGGCCTC
CTGGGCAGGGGGAGTTCAGGGCCCTTAATGCTCGCCTGCCCCTGAACACTGATGCCTACTTGTCCCTCCAAGAACTCCAG
GGTCAGGACCCAACTCACTTGGTGTAGACAGATGGCCAGGGTGGGAGGCAGGCAGCTGCCTGCTCTGCGCCGAGCCTCAG
AAGGACCCTGTTGAGGGTCCTCAGTCCACTGCTGAGGACACTCAGTGTCCAGTTGCAGCTGGACTTCTCCACCCGGATGG
CCCCCACCCAGTCCTGCACACTTGGTCCATCCATTTCCAAACCTCCACTGCTGCTCCCGGGTCCTGCTGCCCGAGCCAGG
AACTGTGTGTGTTGCAGGGGGGCAGTAACTCCCCAACTCCCTCGTTAATCACAGGATCCCACGAATTTAGGCTCAGAAGC
ATCGCTCCTCTCCAGCCCTGCAGCTATTCACCAATATCAGTCCTCGCGGCTCTCCAGGGCTCCCTGCCCTGACCTCTTCC
CTGGGTTTTCTGCCCCAGCCTCCTCCTTCCCTCCCCTCCCCCTCCACAGGGCAGCCTGAGCGTGCTTTCAAAACCCAAA
TATGGCCACGCTCCCCCTCGGTTCAAAACCTTGCACAGGTCCCACTGCCCTCAGCCCCACTTCTCAGCCTGGTACTTGTA
CCTCCGGTGTCGTGTGGGGACATCCCCTTCTGCAATCCTCCCTACCGTCCTCCTGAGCCACTCAGAGCTCCCTCACACCC
CCTCTGTTGCACATGCTATTCCCTGGGGCTGCTGTGCGCTCCCCCTCATCTAGGTGACAAACTTCCCTGACTCTTCAAGT
GCCGGTTTTGCTTCTCCTGGAGGGAAGCACTGCCTCCCTTAATCTGCCAGAAACTTCTAGCGTCAGTGCTGGAGGGAGAA
GCTGTCAGGGACCCAGGGCGCCTGGAGAAAGAGGCCCTGTTACTATTCCTTTGGGATCTCTGAGGCCTCAGAGTGCTTGG
CTGCTGTATCTTTAATGCTGGGGCCCAAGTAAGGGCACAGATCCCCCCACAAAGTGGATGCCTGCTGCATCTTCCCACAG
TGGCTTCACAGACCCACAAGAGAAGCTGATGGGGAGTAAACCCTGGAGTCCGAGGCCCAGGCAGCAGCCCCGCCTAGTGG
TGGGCCCTGATGCTGCCAGGCCTGGGACCTCCCACTGCCCCCTCCACTGGAGGGGTCTCCTCTGCAGCTCAGGGACTGGC
ACACTGGCCTCCAGAAGGGCAGCTCCACAGGGCAGGGCCTCATTATTTTTCACTGCCCCAGACACAGTGCCCAACACCCC
GTCGTATACCCTGGATGAACGAATTAATTACCTGGCACCACCTCGTCTGGGCTCCCTGCGCCTGACATTCACACAGAGAG
GCAGAGTCCCGTGCCCATTAGGTCTGGCATGCCCCTCCTGCAAGGGGCTCAACCCCCTACCCCGACCCCTCCACGTATC
TTTCCTAGGCAGATCACGTTGCAATGGCTCAAACAACATTCCACCCCAGCAGGACAGTGACCCCAGTCCCAGCTAACTCT
GACCTGGGAGCCCTCAGGCACCTGCACTTACAGGCCTTGCTCACAGCTGATTGGGCACCTGACCACACGCCCCCACAGGC
TCTGACCAGCAGCCTATGAGGGGGTTTGGCACCAAGCTCTGTCCAATCAGGTAGGCTGGGCCTGAACTAGCCAATCAGAT
CAACTCTGTCTTGGGCGTTTGAACTCAGGGAGGGAGGCCCTTGGGAGCAGGTGCTTGTGGACAAGGCTCCACAAGCGTTG
AGCCTTGGAAAGGTAGACAAGCGTTGAGCCACTAAGCAGAGGACCTTGGGTTCCCAATACAAAAATACCTACTGCTGAGA
GGGCTGCTGACCATTTGGTCAGGATTCCTGTTGCCTTTATATCCAAAATAAATCCCCTTTCTTGAGGTTGTCTGAGTCT
TGGGTCTATGCCTTGAAAAAAGCTGAATTATTGGACAGTCTCACCTCCTGCCATAGGGTCCTGAATGTTTCAGACCACAA
GGGGCTCCACACCTTTGCTGTGTGTTCTGGGGCAACCTACTAATCCTCTCTGCAAGTCGGTCTCCTTATCCCCCCAAATG
GAAATTGTATTTGCCTTCTCCACTTTGGGAGGCTCCCACTTCTTGGGAGGGTTACATTTTTTAAGTCTTAATCATTTGTG
ACATATGTATCTATACATCCGTATCTTTTAATGATCCGTGTGTACCATCTTTGTGATTATTTCCTTAATATTTTTTCTTT
AAGTCAGTTCATTTTCGTTGAAATACATTTATTTAAAGAAAAATCTTTGTTACTCTGTAAATGAAAAAACCCATTTTCGC
TATAAATAAAAGGTAACTGTACAAAATAAGTACAAT
```

HUMAN PROTEIN SEQUENCE : hP5-001.1 (Seq ID No: 30)
MAAPALSWRLPLLILLLPLATSWASAAVNGTSQFTCFYNSRANISCVWSQDGALQDTSCQVHAWPDRRRWNQTCELLPVS
QASWACNLILGAPDSQKLTTVDIVTLRVLCREGVRWRVMAIQDFKPFENLRLMAPISLQVVHVETHRCNISWEISQASHY
FERHLEFEARTLSPGHTWEEAPLLTLKQKQEWICLETLTPDTQYEFQVRVKPLQGEFTTWSPWSQPLAFRTKPAALGKDT
IPWLGHLLVGLSGAFGFIILVYLLINCRNTGPWLKKVLKCNTPDPSKFFSQLSSEHGGDVQKWLSSPFPSSSFSPGGLAP
EISPLEVLERDKVTQLLLQQDKVPEPASLSSNHSLTSCFTNQGYFFFHLPDALEIEACQVYFTYDPYSEEDPDEGVAGAP
TGSSPQPLQPLSGEDDAYCTFPSRDDLLLFSPSLLGGPSPPSTAPGGSGAGEERMPPSLQERVPRDWDPQPLGPPTPGVP
DLVDFQPPPELVLREAGEEVPDAGPREGVSFPWSRPPGQGEFRALNARLPLNTDAYLSLQELQGQDPTHLV*

AGRES DISCOVERY 5-001

Table 6

MOUSE GENOMIC SEQUENCE : mD5-002 (Seq ID No: 31)
AATGATTTGCAGAATTCCTAATTTGATTTAAATCATTTTAGAAAGTTAGAGTAATTGGTAGAAAGTTTAAAGAGACCATT
TGTTATAATAATGTTAGAATTAAGAGCAAAATGTGCTCCCCACACCCAGAAGTGAGGGCTGCATAAGTTAGAACAGAAGC
ATGGTGGGCCTTCCACGCCCGTAGTTGTACTTGGAAGAGAAAACAGGCTTGGGGAGATGTTAATGATCAAAGAAAACATTC
ATTCTAGGTTGAGACTGATTTCCTGAATCTTGTGATCTAGGGATGTGTCTGCAGGGTTCAGTGTGCACCATAGAAGGGCA
AGGCTATGAATAAAGAGTATATAATTCTATTGTGAGTTATTATAATTATATACCATTTCTATATAGAATGTATTAATTCT
ATAATTACAGTTCTATAATTCTGCAAGAATAGAGAATAGCAGATTAGCCAGTTTAGCTGAACAAGGCTTCAGACATTAGC
AGTAAGATGGATGAGAACCTGGATCTTTGTGAATACAAAGTGTCACCAGCTAAGCATAGGAAAAAGTCGCTACTACAGAC
TGGCTCGCTTGAATTTGTTGATCACTGACAAAAGAATTATAGTTTGAGGTTACAGTGAACTCATGGAGAGAAAGACTGAT
GAGAAATGTTCATTCATTCATTCATTCATTCAGTGCCTCTGTCTACCAGGATGCCTGAGAACAGGGATTTCCCAGCAGAG
AGTGCAAGGACTCCTTGACTCTACACTATGAGGACTCTGAGCACTTTGGAGTGAGTCCAAGGCTGGAGCCAGAAATCTAC
AAGATGAGATGAAGGTCCCTCACAGTACCTGAGAGGATTTCAAGGATGTGTAGAAAGAGATGCAGACAGAGCTCACTCTG
ATCATGTGAGATCATATTGGCCTTGTATTGATCAAACCTGAGATGACATAAGCTTCTACATAAGGAATTTTGAAAATCTC
CCTTTAGAAATCATCACTACAATCATCATGTGCATCTTCATCACAAATGTTATCATCATCGATGCAATCACCAGGATCAC
AACAGTCATCATCTGCATCCTCATCACCGCCATTATCATCGTCAATGACATCACTGTCATCAGCATCATTATCACCACCA
TCCTCATCACCATTATCACTATCGTCATCATCACCATCCACACTGTATAAATGATACATTATATTGTCACTCCATGGTGA
TTTAAGGGATGGTTTGGATTGTAAATGAGAAAGAGAGTATGACAGACAGAAACACACAAAAAAACAGACAGACAGACAGA
CAGGAGAGAGAGAGAGAGAGAGAGAGAGAGAGAGAGAGAGAGAGAGAGAGAGAGAACAGCCAGAAGAATCTAGAAGAG
TCTACAGCAGAAAGAGGGGAAAAAACTGAACAAGGCAAGCATACTTGACCTGGCCAGGGTTACCGTGAGGCAGGAGAGA
ATAGCAGTGGATAGAAACTGGAGAAAACATGGTGAGAGAAGCAGAAACGGGGGAGGGGGGTTCAGAAAAGCAAGAGAGTG
AAGGGGGTGAGGCACATTGAGGATAGCATGGTCCTTAGGAGTGGAGTAGCTTGGGAGAGGGAAGATCATGAGTTTAAGGG
AGAGGAAGAAGGAAGAAGGCCTGGGGTGCAGCATAGACTTTGAGATGTGTAACAGGCACTGTGGTACTGAAGGAGCCTGG
AGTCCAGGGAGCCATATGTCCTTTCTGTCAGAGGGAAGGGAATTGCCTCCTTTCCTGTAGGAAGAGGGGCGTTGGCTTTA
CAATTCCTGAGAAGTGCTGCTTTTTGTCTCCTTACTGGAAATCCTCCTACAGTCCAGTGTGAGCTCATTTCTGGACTTCT
GGACTGCCTTTTGGAGTTCAGGGAATTGGAGTTTCTGTTGGACCTGACAGCCACCATCATACTGTCCCCATCATCTCTGT
ATCAATCAGACTTACAGAGGGAGAATGCTGTGGCCTGGCCTACAGTCAGATAAATCCTGAGGAAAATAGACTGAGCTAAG
CCAGCCACAGAAGAACAGACAGGGCAATGAGACATAGACGGGGTCCCTACTTGGTCAAATTTACAGAGGCAGGAAGTGGA
ATGCAGGTGTCTGGGCACACAGAGGAGAATGACATGTACTGGCCTGTTCAGGTGAGGGGGCGGGAATGCTCAGGAGATG
TGCAGTAGTGACAGTCACCTAAGTGTACATAGGCAGTGACTGTGCTCATGTAAGGACCAAGAGCATTAATTTTTGTATGT
TTTATCATAGTCTTTTCAGAAGCAGGCATAGTTGGTATACAGTAAGGCACCTGAGCAATGGCCACCAACAGTCACCCATT
ACTGAGAGAGCAACTAGGCAGGGACCCAAATGCCACCTTGGATTAGGATGTTGTACATGACCTGATGGTAGATATCTAGG
GGTGTAGGTTTATAAGTATATGCCATGCACGTGAGATATAAAAACTGATAGAGTGTAATAAATTTAAAGTTCTTTAATGT
AAATACAGCATAGAATTCTCTTCTGTTCTGTGGAAAAAACTGAAGTCCCCATCAGTGAGGAGTCAACTTCCATGACATTC
AAAGCAAGATCCCAAGACTAAGATGGATCCATGTCTTTCCCATCTGTTTGTCAGACCACTCCTCATGTGACATCTGCCAG
TGGCCAAAGTGGGACCTCACAGCCCTATCTGAGGTGAGTCAGAGATGCTTAACACTCTAACGGTGCATGGTTGTGACTAA
GCAATGTTGGTTGTCTTAGAAATGTAACTTACTATGCCTTCTGTGCCTGTCCTTAAATGAGAGTTGACAGACAACTCTCC
TTGTGCCCCTTAGGTTCAAGGCAGCACCCAAAATGACAGTGCTGGTGACAGTGTCCCAAACAGAGGGGCTTTGGCTGGGT
AATCCGAAAGTATGCTACTGGATAAACAAGTCTAAAGACAGATTTCTATTTACTGGAAAAGCAGCCATCTCAAGGAGGTT
TCTTTTTATGAGAAACTTCCCTTAATCAGCCCCAACAGTCAACCACTTTAGGCAAAGATATCTATTTCTGGGTTAATCTG
TCCAGCCAGCTGTCTCAGCTCAAGGACCCTACCCTGTTGACACATAAAATCTGTTTGCTCCACACCACCCCAAATTGCTT
CTCCCTCTGCCTTCTTAGAGATAGTCTTGGCAATTCAATCCTGGATAAACCTATCCTTCTGCCCATGAGTCTAGTCTAGT
CCAGTGGTCACACTGTGCTTGGCTGCTTTGATCAGGAGCTGGATGCCAAGTGGCCCCTCCTTCAACAGTCCCTGCAGGCA
GTCATGACAGCTGTGTGCTCATGGCTCATTTTCCTGTCACCTTTACTGGATTTAGAATCATCATGGAGAGCATGTGAAGG
CATTTCTAGATTAGACTGACTGACATGGGGAGACCCGTGTCAATGTGCATGGCACTATACCTTTGGCTAGGGTATCTGAC
TACATAAGAAGGAGAAAGCTGGCTGAGCCCCAGAGGTTGAGGCTCTCTGCTCCCTGACTGCAGACTGCCTCACATTCCAG
TTGACATGGCTTCCCATCATGATGTAAGTCAAGTAATCCATTTTCTGCCTTTTGGTGTGTTTAAAAGCACAGGGGGTCAG
AGAGTGTTTAAAAACACAAAAGGGGTCATTAATCCTTGCTGGATTAAACAGTCTGTTACTACCAGAAGGAAACACTTCAAT
CAGAAAGCAAGCAGGGCTGCTGGGGAAGCCATGACTGCTGCCCCGGGAGGCCCTGCTCCAGGCCTGGCAGCTTCAGGATA
CTCTCATTAACTTTTGCATATTCCATGTGGAGACAGGGATGGATTCTGAGTTCAAGCCTGCTGGTTTTCAAGTCTCAAAG
CAATAAGCAGCCCACCTGAAAACACACAAGACTCTGTGAAGTTCCTCGTTCTCAGGGCTCAGAGATGTGAGTCTCTAGTT
TAGGAAGGCTTAGAGCTTGTTCAAACTGCAGCCTAGAGCTGTAGCCATTCTGCCTTTAAATGCCTGGAGCCACAGTCTCC
TTTTTGTCAAAACCTAGAGCTACAAGCCCAGCCTGTAAGCCACTTGTATTCAGAGCTCAGGACTGCAAAGGCTAAGGAGG
CTGAGCCTTGCCCACTTGTAGTGTTTACCTGAATAGAAAAAATCTTCATTGGTACACATAGAAGGTCCCTGTGAGACCAG
AAGGAAGGTTTTCCAGGTTCATCTGCTTATCTGTTCTGGCTTCATGGTTTTATATCATCGACAATTCTATATCTTCAGGG
GGATGATTTTCCAGTTCCAATATTTCACTTTCATGTCACTTCCTAGGTGTGGACTGGATGTCCCCACAGGAAGGCCTGAG
TTTGAGACACATCTTTCCTTGCCCAAGAAAAGGGGGTCCCAGTTGTAAGGAGCTCAGCTGAGCTCCCAACAGTTGTAGTG
TCTAAGGGAAATGGAAAGGACAAAGGCATTCTTCTGCAACACTTGCTGGGTTCAAAAAAGCAGGAGCCCGGCCCAGAGGA
CGCAGCCATTGCTCTACTGTTTCCCCTGAGGAAGGTAGACAGAGGGTCAGATGGGTCAGCACATACATGACCCTCTCTAT

```
TGTTGAGAGAACCCAGAACAAAAGTTCTGGCAGTCTGGGGAGGCTCTGGAAAGGACTGGGAAGGCATGGGGTGAAGGTTA
CAACTGGAAAGAGGATATGTGTTGTCAAGGACTTTCTCTTTCTCCTTGGGGACCTCACCTGAGGTGCACCATTAAAGACC
GCAGCCCAAGTTGTCAGAGAGGGATCCAGGCATAGGACAGTTGGCCAGGAACACAAAGATGTAGAGAACCAAGACAGTCT
GGTGTACCCAGTCCTTGATGTCAGTGCCCCTCAGAAACTTAAGGTTAGACCTTGAGTGGAAGTCAGTCTTCCTGCATACA
CCTACCAGGCAAACTTTGGAAAGAATCCTAGGCTTAGGAACTGCATAGATTTTGACCATAACCCAGCTCCTTACTAAGGT
GAGTCCCTTGGTGGGCAGTGTGGTTAGGCTTTTCCTGGGGCAGAAATTGAAGCTTCAAACTCTCTGGTCACAGAAATCCT
TGCCAGGCAGAAAGATCTGTACTGTGCAGGTCTCTTAAGCAGGAAACCCACAGAGGAGCCAAGCTGACAGTGCACACTCC
TCGCTGAAGCTCTTCTCCACCGGGCCACCTAGTGGCCAGCCCTTGATTGCAGCCCCTTCCTGAAGTCACAGCCCCTCCCA
GGACAAGAGGGGAAGTTCAGGGAAGCAGGCCCAGAAAGACATTCTTCAAGCCTGCCCTGTCCAGCCCAAGGCAATCCCCT
CCTGACCTTGCCACAGCAGAAAGAGACATCACCTTGGGATTTAGCACCCGAGAGAAATCGGGGTCAGAAGCTTCTCTCCA
GCAAATCCAGCCAGGGACTTCCCTGTCAGGGTGGAGCATTGCAAGCTCTGCCTGGACAGCTGTGGTCTTCCTTGCAGGGG
GAGAAGAAGGGGGTGAAAAGAGGGGGGAGAGAGAGAGAGAAAGAGAGAGGGGAGGGGAGGGGAGGGGAAGGGAACGGAGGA
GAGGGACAGAGACACACAGAGAGACAGAGACACAAGATAAGAACAACAAAAAGACCCTACCAGGCTAGGCTTGGCTTCCC
CAGCTGTCATGGATCCAATGTCTCCTGACTGCTGTGCTCTCCCTCTGCCTTACTAAACAATCAGAAAATAAAAACTTTGG
CCTTCCTTTGGAGAGCTCTTTTCCCAGGGATGTTCCAGAGAGCAGATTCCCATCTCTCCCTGTTAACTGCTCAGCATGCT
GCTGCAGAGGCTCTGCTGTGGTGTGTGAGAAGTTCCCAACCTGGTGGATGTGACAGAGACTCGGAGGTCTCAAGTATAAG
ATCTCCCATGGGTGCTCCCTCGGAGGGCTTTAAAGTGAGCATGTTTGACAATTAGATTATTCCAGGATGGAGAGCAGTGG
CCCTCTGTCAACTGCATGAAAGGCAATGTGGAACATCCCAGGAGCAAACATTGGGTCAACAGCTGTTCACTGACAAATGT
CTCTACAAAGAAGCACGCCATCGTGCCTGTCTCTGCACAGCGTTAAGAGACACCCTCCACATCATTGTGACAGAATTCTT
AACTGTCAGAGGATGAGTTGGTATCTTGTTTAAGGGACTTCCTTCTTGATTTTTTGGTTGCTCATTGGCCAAGTCCATAG
GGCCAGGTCTGAATGACAGCCCAGGCTGCCTCCAGCTGTCACAGAACAGTTTGGGCATAGCTATCTTTTGTCACCCACCA
CACCACCAAATTCACTGAAAGTGAGGAGCAGAGCAGGTTTGGAAAAGCCTGGGATGGAGAGATCTGGGATGCTCTCCTCC
TCCAAAACAAGCTGTGTGAGTTGCGGCTTTGTTTTGTTTTGTTTTGTTTTTCCATTTACAATGTTCATAAATGTGGCGAC
TTAATTGGCAAAGATGAAAACGTCATCGGTGTCTATACCGTTGTTGAGTACAGGGAATTGATTCTACACTAGTCCATAAC
TAGCTGGTGCTTGAGTTGTATCAAGGGACAGTGAAGTTAGAGGTCCAAACTCGGAGCTTGGGTCTTAGGAAAGCCAGACT
TGCCAACCAACTGTGGACTATATCACTCTATAACAGTGTGTTGGCCAGCCCAGGAATCCTGTTAGTGAGACACGTTTGTC
ACATACCTAGGACTGCCAGGAGCATCAAAGCCACTGGGAGCTTTGATTGATCCACAGGCATCTGTCCTGCCCCATGTGAG
CACAGTGCTGTGTGTGGTACCTGTGTCCTGGGTGTGTTTAGACCTGTGACAGCTGGAATAAGCTTGATCCTGGCAGCAAG
CGCTGAGCTGCAGCAAACATGCTTTCCTCAGAATAGAAGGAGCATCATGCCTGCCCTTTGCCCTCAATGGTAAGCTTTGT
TCCTTCCTGCCATTAAGACTTGAAAAGCTGTGTCCTAGACACTGAGGGGTAAGAGCTAAGGAAGGGCTGTATGCCTGATT
CTGAAGAGTCATGAATGAGCATATCGGTGTATGTGACTTATTGTGAATTTATTCATGGTGGGACAAGTACCGTGGTCAAA
GCAACATAGGTGAGGAAAAGACCTATTTCAGGTCACAGCCCACCATGGAGGGGAAGTCGGGGCAGGAACTCAAGCAGAAG
TGTGAGCCATGAACCGTGGCAGGACACTGTGTGCTGGCTGGTTCGTAGGTTCATGCTTATGTAACTATCTTATACAGCTC
AAGATCACATACCTAGGGAATGGTACCGCCCACAGTAGGCGGGGCCCTCCTACATCAATAAGCAATCAAGACAACTTCTG
TGGTTGCTCATGCCTTTTGGAGGCACAGGCAAGCTGATCTCTGTGAGTTTGAGGCCATCCAGGGCTGCATAGGGAGATGC
TATCTCAAGCCAAGAAACAGTCAAGACAACCCCATCTGATCTGGACAAGTCCTCACTTGAGACTGCTTTCTTAGGAGACT
CTAGGCTGGTCGAGTTGCTAGTTAAGCTGACTAAGACGTGAGGGAATATATGGAAACATATTTAAAACTTCACTTGTATA
AATTATGGTAGAGTTAATATGTATATCTATTGCTTTGAGACAGGTACTGTTTCCAAGGCTCTAATTTCCAAAGCATAGGA
TACACTGCATAAATATGCCAGGGACTTCCAGTTTAAAAGGTGACCTGTAAACATTTGGATAGCTTAGTCATATCTTACCA
ATGTTGTTCTGTGGGAAGTCCTTCTTAATGGCCTTTTGGGCTGCTAACTAGCTGTGTTCATTTTCACTTTGAGTTGAGAT
CTGAAATCGAGTGAGAATGGCTCCCTTGGGGCAAGGGCACATTTATCTGGGGACAGTCTGTGTTAAGAAAGCAATGGGTA
TTATATAAAGTAAGTAGCAGGAGGTTGGTGACAAGGACCCTATTGAGCCCTAGTGTGACACTGATCCCAAAGAGTAGTAG
GTTGTCCAACAAGACATGTTGTTCCTGCTGTATGATCTGAGGATCAAGGATTTCTTGTCAGTTTTACATGTCCGGGGTGT
CCCGTGTAGGTGGGGATGGTGGACATGACCATTTATGCATTGATTTCAAGGGCTGTCTGGGTGATGTGTCAATTTCCTCA
AACAGTAAGATATTTGGAGAAATGGTTATGCAATGTGTGAGGAGATACTAATCTCTCTGAATTTCAAGGCCACATAGGAG
GGTGGATCCAGTTATCAGACAGAGACCACCTGCCTTGTGTTAGTATCCCCTGTTCCATGAGAGCCTCTCATAGGTACCAC
ACAATTCATACTCTGTGTCGTTTGCTGCCATGACTATGGCATAGCTACTGCACGGGGAGAAAGGGTTGACTAAGTACCTA
ATGAACACCATTGTGAACCAAAACACCTGCCGTGTGCCCCATGGATTGCACCCCTCGCGACAAGTCAGCCACCGAACTTC
TTGACTTTCGTTTTCTGTTGAATAAAAGAAAATATGAAGTAAAACAAACCCATTGGTGTCTGGACATGAGGATCTGCAGT
GTTGTGGATTAGAGGTATACGATTCGGGCACACTGATCTCTGAGAATAGAATCAGTCCAGAGGTTGGTGGAAGGTTTCTG
TTCATCTGGAACTAACTGCTGTCCCCCTGCCCAGGGGCCCTCTCCTACTGTCTGGTCTCCTATCTCTGTAGCTCTGTATT
GGGAGATAAGAAATTACCAGTGTATCACTGAAGCTACTTTTAAAAAAAATCTGACATTTCAATGCTTGTATTTTTATGCT
TATTTGAATTTATTTACTATATTAAAATATCACTCATTTCTATGAAAGGGTTTTGGTGTCCCTTTAAATTTTGCCCTGAG
AGAATGGCTCACTCCCCTCCTCTTTTTTTTTTTTTTCCATTTTTTATTAGGTATTTCGCTCATTTACATTTGCAATGCTAT
ACCAAAAGTCCCCCATAGCCACCCACCCCCTCTCCCCTACCCACCCACTCCCCTTTTATGGCCCTGGCGTTCCCCTGTAC
TGGGGCATATAAAGTTTGCGTGTCCAATGGGCCTCTCTTTCAAGTGATGGCCGACTAGGCCATCTTTTGATGCATATGCA
GCTAGAGTCAAGAGCTCCGGGGTACTGGTTAGTTCATAATGTTGTTCCACCTATAGGGTTGCAGATCCCTTTAGCTTCTT
TGGTACTTTCTCTAGCTCCTTCATTGGGGGCCATGTGATCCATCCAATAGCCGACTGTGAGCATCCACTTCTATGTTTGC
TAGGCCCAGGCATACTCTGACAAGAGACAGCTATATCAGGGTCCTTTCAGCATAATCTTGCTAGTGTATGCAATGGTGTC
AGCATTTGGAAGCTGATTATGGGATGGATCCCCGGATATGCTAGTGTCTACATGGTCCATCCTTTTATCTCAGCTCCAAA
CTTTGTCTCTGTAACTCCTTCCAAGGGTGTTTTATTCCCACTTCTAAGGAGGGGCATAGTGGTCACACTTCAGTCTTCAT
```

```
TTTTCTTGTCACTCCCCTCCTCTTAACCCCAGCAGCCTCAAGTTATTCATTTGTTCATTCATTCATTCATTCATTC
ACTTTATACCCAGTCATGTGTTCCTCCTCTGGGCCATGGCTGGGCACTGGGGGAGGGGAACAGACACAAAACATCACTTG
CTCTGAAGACAAGATGGGAAGAAAACAGGGACTCTAGGGCAGGACAGAAAAGGGAAAGCCAAAGCTGACAGCCAGGGATC
AAGACTGCTCCCACTGCCTACTGACTGTTGTCAACTTAGGTTTTCCATGTCAATGGCATACCCTCAAAACCTTTCTGGTT
TGTAGGTCCTTCTAGAAAAATACCCCAAGGTGTCTCAAGACTTGAAGAGGCCACCTGTAGCTGATTTGCATAAGGCCACA
TGCTAAGAAGTGGTGCCCTCAAGGTCCTCCCCAGCTCTGCCTGGCTCTGGGCACATGCTTGCAGGGAGGTGGTTATTGCC
CTCTGTGTCTGCTTCCTCCAGAGGAGTCAGGGAGGGGTGTGGAGGAGACCATTTCAGGCTGAGACCTGAAGGGCACATGA
GAGCTGACTGGGAGATGGTGGGGAGCGAGCTACTCTGGCAGAACTAAATGTCGTGAGTATTATCAGAGGCTAATGTCCAC
AATTCTGAGAATCAGGCGAAGCTGCTTGCGAAAGGGCACTGGCAACTTCCTCAATCTGCTCTCTGTTTCCTCCAGATGGG
GCACAGATAAATAGGAAGAGCCTGCAACTCACTGGCACTTGGAGGCTCCCGAAGGAGGCTGCCTGTCGCCCAAGCACAGA
GGTAAGTGGCTGGCTTCCTGATGCAGAGCTGAGGGTGGGCGCAAAGCAGGGGCTGTGGGGGTCTCCAGGGTGGAAAAGGA
CAGGATAAGACAGGGCTGGGGTAGCGTATTCCTCAGACTTGATCTCTCTAGAAAGACTAGGTCTCAGACTCCAAGTCAGT
GAGTGGAGGAGGGGCACCCCGGAGCATTGGAGCTGGGAACAAAGTTAGAACTCTGGAAGAGGTACGGTTAGGATACGCTC
TGGGGCTGTGGGACTCCGGGAGGACACAGCTTTATCACTGGAGGTCTGGTGGCAGGGAGGACTCCCCTAAAGCTCTATAT
CGGGCTAAGCACTGTGTTCCTTCTAACTTTGCCATCCATATGTTACTTAATTCCCTGTCCTGAGCTTCAGGTCACACATC
TGTGGGACAGGGTGAGTGGGAGACATGGCTTCCTCTGTTTCAACTAGAAATGGAGGACAGAGAGAGGAGAAAGAGAGGGG
GTAGAGAAAATTTACCTACAAGGATGACGAAGTGTTGGGGACAGAACCAGAGATGTAGAACCTCCCTTGGTACTGGGTGT
AATTTGACAATCTTACTTGGGAAGTGCCCAGAGATATACCAGATCATCCAGCTCTGTTTTTCTCTTTCTCTGTCTCTGTC
TCTGTCTCTGTCTCTGTCTCTGTCTCTGTCTCTGACTATCTCTTTCTCTATGTGTGTGTGTCTGGTTTCT
GTCTCTCCAACTCTGTCTCTCTCTCTTTCTTTGTCTCTCTCTGTCACAAACACACACACGCACACGCACACGCACACG
CGCACACGCACACACACACACACACCATACTGTGCTCTGAAATCACCACAGATGATTTGGGTCCAGATGTGTCGCT
TCTGTACCTAGTGTGCCGGAAGGTAGGTGTCTCATCACATAGCTAGGGACCAGTCGCTCTCAGACAGGGACACAGACAGC
CCCTCTGCTGACAGAGACCACTGTCATGTCTCACAGATCATAAACTAACCTGTCCATATATCCCACATGCTGTATTCCAG
AGGTCATGCATATGTGCGACAGAAATCTCTGTGATCTGTTAAGCAAAGAATTTGCCTGAGGTTCAGGCCCCTGCCTCTGC
TCACGCCCAGCACATAGGGCCTTTGAACCTGATCTGGCAATGGAAGGAAAAGGCACCTCCACTCACTTCTTTGTAGTGCA
GCGTCTCCCCTTTCTCTACCTCTTTTTTCAAAAGCCCTTATTTTTTTTAATTTATTTATTTTATTATATGTAAGTACACTG
TAGCTGTTTTCAGACACTCCAGAAGAGTGAGTCAGATCTCATTACAAATGGTTGTTAGCCACCATATGGTTGTGAGATTT
GAACTCAAGACCTTCACAAAAGCAGTCAGTGTTCTTAACTGCTGAGCCATCTTGCCAGCCCTCTTGAAAAGCTTTAAAAG
AAAACTCCTTCTCTGTCAGCATGTGTTGAAATGCCACTTCAGACTGTATCCCGCCATTGGCTCTAACAAACCTTTAGCTG
GAACCTAATGTCTGTACTGGAACCAGAAGGCTGTCAATGAGGTTGTCCTATAAGGCTCCCCTTCACTTGCTCTGCTGTCT
CTGACCCCTAGACCTCTCTCTTCCCCTGGTGAGAAGTCTTCTAGGATCTCCTGTCTGTCTATACCAGAAATCATAGTAAG
CCACTTGTTGCATGGGCCTGGGACTGTCCCTTCACCCAGACCTGCTGAGTACACAGATACCTGTTCGCCATCTTGCTTCA
CTACTTCTGGTCTGGCCACACTTCAGGAAGGGACCTGAGTGCCACAGGGTGCTTGTCTCCCTTGCTGCCAGTCCTCTAGA
CAATGGTTCTCAACCTGTGGGTCGGGACCCCTTGGCAGTCTCCTGTCAGATGTCTAGCAGATCAGATATTTACATCACGA
CTCAGAACCACATGAAAATATTGAAGTAGCAATGAAATAATGTTAAGGTTGTGGGTCACCACAACATAAGGATCTGTTTT
AAGGGGTCACAGCATTAGGAAGGTTGGGAGCCACTGGTCTAGACCCTCTTGACCAGGGTTCATGATGGCATTGCTCAGTT
CCACGTGTCTAAGGCCTCTCCTCAGCTGCTTTCTGTGTGACCTCTAATGACCTCTATATGACCTCTACACACTTTGTCAT
GAACTTTTCTCTAGCAATTCCTCAAATCCTGAGCCTCTTTAGCAGCACTGACTACAAAGCAAGTAGCCCGGAAAAACAGC
TTCTTCGATGTAAACATTCCTAGATTATGTCACACCATTCAAAGCAGGTGACCTGAGACCCCTTACACTTGGGCATCCTT
CATCTTCCATAGCTGTCCCTTCTGTCAACATAGGACCCCATTCCAAATTATAAAAGGATCCATCCATTCTCAGGGCACCC
TTCCTTTTCCTGGAAGAAGTTGCCCACTGTGCCTCTGTCCATAGCACCTGCTCTTACTGAAATTCTCACAAATTTGATCT
GGTCAATTCTTTCTCTACCAGGCCTTGAGCTATTCTCACCATAAGAGAGAATTACAGGGTGAGTGCCTGGGTGTAGTCAG
GTACATGCTGCTGGAGTGTATGCATGGATGTGTGTCTCACATATGCATGTGTGTGCATGTGCTTGTGTGTGTGCGTGC
ACATGTACCTGCATGTGCCTGTGGCTCTTTCTGCACCCGGGATTGGCTTGTACCCTCACCATACAAGCTTCTAGGCCTGG
CTATGGGTTTTCCCTCCTCAGAGGCCCTGCCAGAACATACTTGCATATTCACATTCTAGCTTAATTAATTCCTATCTGGT
TTCTTTGTAGTTTCAGAGTGAATAAACCTTTGTGCTCACTCCTGGTAGCTCCCCCCCCCCCACAACCCCACTCAACAGTTT
TGCTTTTATTGTGTGAAGTTATTCATAACCCAGGACTGTAGGAAAGGTCATGAGTTCATTAGCGTTTAATGTCAAAGAAA
GACACAAAAGACACAGACACCTGACTTTTTAGTGGTTAGAAAACCAGAATCATGGATGTGGCAAACTCATTTCTGTGAC
TAGGAATATGTTCAGAAATCGTGGCTTTTTAACTTACCCAGTCCAACCTGGGCAAGAAACAGAGCATCATCCTCTGCTGA
ATTTGAAAGGTTCTGGATCGAGGAAATTCTACCTCATCTGAGCTTATTTTTGAGATCTTTCAGAATAGGAGGAGCCCAGA
AACCTGGCCTTATCACTCACTGCTTGGAGTTGAGGAAGGTTTATAAGCAACTACTGCTTACACTGTGCTGTGTGTACACA
CCACAAAGAAAAAAAACTAAGAACCCAGAGTAGCCCTCCGGCAACTAAAGTCACCGAGTCAGTTCTGAAGAGTGATTGTT
AGAGATGTCCCCAGAAGAATATAGTTATTTTTTCTACACTTGTGAATATATGCAAGTGGGTGTTGGTCTGTAGCTCCCAG
ACAAGGTGCCAAGTACACTTCATTTCATTGACAATGCTTGCCTCTTAGCCAGCACCTCACCCACTGCAGCAGTCCTTCCA
TGTGTGAGACACGTTGTTGCTCCCCGCCGAGTGGGCCCATGGCTCTGGCAGAGCCCCTGGCTCATCACTGATGTCTCCCC
TCCTTTCCTTTGCTCTCCTCCAGCTGAGGAAAGTGTTCCTCTTGGTGTCACCTTTCCCTTCCAGGGCTTCTGAGACCCAG
ACAGGCTGCCTTCCTTTCTTCCTTTCTTTCTTTCTTTCTCTCTCTCTCTCTTTCTCTCTCTCTCTTTCTCTCTCTC
TCTCTCTCTCTCTCTCTTTCTTTTTTTTGAGGGGGGGTTGTTTGTTTGGTTGTTTGGTTGTTTTGTTTTGTTTTGCTTT
GTTTTTTCAAGACAGGGTTTCTCTGCCCTGGCTGTCCTGGAACTCACTCTGTATACCAGGCTGGCCTCGAACTCAGATAT
CCACCTGCCTCTGCCTCCCAAGTGCTGGGATTACCATCTGGCCAGGCTGCCTTTCTCCAGGTCCCTGAGCATCTGTCAGT
CTCACTGACTCCACAAACCCCTCCCCGCTTTGGCCATGCACATCTCAGAAGTTTCCCGCCACGCCTTTCTCATGGCTATC
```

```
TAATCTCTTTCTGTCCTGGCTTGGTCACATGCTCCCCCTGAGCCCTTTTCACTTCCCCTGCACACCAGAGCAGCTTCCAT
CTCTGGTCACCAATACCGCAGCATCTCTGGCTCCAGTCAGATCCCAGAGGTGTCTTTAAAATATCAGTTGGGATTGTCCA
GTGGTCCCAGTGCAAAAGCACCTGAAAGTGACAGCCGCCTAAAGTCCTAGTGAGCAGGATGAGGTTTCCCTCCTGCTACT
TCATCATCTTTACCCACATGATTGTTAGCCAAGCTAGTATTCCATGCATTCAGACTTAGTTACATGTGATGGAATATATA
TCCCTGGGTTACGTTTTTCTCTGTCAAGTTTACGATGGCTTCTGTCATAGAAAGTATTTCCATCATCTGCTGTTTCCATC
ATCTGTTCTGTTTGCTTACGTGTGTGATCATGGATGTGTGGGTACATTTGTGTGTAAGTGCATGTGGAGGCCAGAGGACA
CTCTCAGGTGTCCTTCCTTAGGTACAGAATATCTTCTTCTTGAGACAGGGTTTCTCATGGGCCTGAAACTCAGTAAGTAG
ACTAGGCTGGCCTGGAACTCAGTAAGTAGCCTAGGCTGGCCCACCAGGAAGCCCCTGGGATCTGCCTTTCTCTCTGCCTC
CTAGTGCTTGGATCAGAAGTGTAGGCCACCATACTTGACCTTTTTCCAGTGGCCTCTGGGCCTCTGACTCAGGTCCACAT
GCTTGCTAACAAGGTTGTTACGCACTAAGCGACCTCGTCAGCCCTGTCTGTTGGTTTGACTTTTGTTTTGCTTTGTGGTT
TCTGGCTTCAATGTTTTACTCAACCCCTAAAATTAAAGACATAGTTTAATGGTCTTAATGGCTTAGTTTTGAGTTTCAGA
TCTTTAATTCACCTTGGATGTGGATGTAAAGAAACAAGTTCACTTGAAAGCACGGTTCCCTCATTGATTAGAAGCCACCA
GTCACGAGTGTGCAGCCCCTCTGTGCCTCAGTGGCTGGAGACAGTAGTCTAGCTTTCTAAGCTCTGGCACACTTGGTTAG
ATTCACCCCTGCTTTGATCTTTGCTCTGACTATGGGTGTTTAGTAGCAGATGCTCATGCCTTTCTGCTCTGTATTTTGGA
CTCAGAGTCAGCTCAGTCAGGCACTCACATCCTTCTCTATTCTTTGTTACTATCTCTCTAAGCCTCAGGAGTCCTCTCAG
CACACTGATCCCTGGAGACAGAGCTGGGTCTCTCTCCTTTGCTAGCATTGGCCGGGGGCTGGGGCAGCCCAAGTCCTCTC
TGGCTCTGACATTGGCATGGGGATCAGGGGGACAAACTCAGGTCATCAGAACAGTTTGCACACTGAACATTCCCATGGCC
CTGGCAAGTAAGTTGGCCCTGTAGCCTCTTGCTATGGATGCACTGTGGGTGGACGTTTGAGCTTCTCTCCTTCCCTGGTG
AAGTGCTCCAAACATGGCCACCATGCACACTTCTTTAACAAATCTGGATCTGACTGGTTTGTGAAATGGTTCTGTCTGCC
CTCCCCTCAATCATATTTCTTCATACACTCAGGGATCAGGGTGTGCAAACACTTCTGGTTTCCTGCACTTCTCCCTCTCT
GCCACCTCCACCCTGCCCCTTTTTTTCCTAGGTCCTCATCTCTCTATACTTATCCTTCGTCTTCAAAGGCTATCAAGCCT
AACTTGTTTTTTTTTTTCAGACTGGTGCTCACTGTGTTGCCCAGGCTAGCCCCACATTCCTAGTCTGGAATAATCCTTCC
TTACTAGCAGCTGGAATTGTATGCCCCCCAAGGACCTGACTCAGTCCTTAAGGTCAATGCTCCCTCCTACTTATATTCTA
CACCAGCACTTGGGTTTAATTAGCATTTTTGAACCCTCATAAAGCCAGGCATGTCTCAGCCCATTTTCAGTTCTCCTCTT
ACCCCGCCGCCCCCCCCCCATCCTCCTCCACCACCTCAGAGCCACCTCCCACCTTAGTCATCTGAGGTTTTGCCTGGTTC
TTGCCCCTGCTGTGTGCTGCCTCACTGTTGCTTCCTGCAGTGCAAATCTCCCTACATCTCTGCAAGGCATGCTGCTTTC
TCTGTTCCCATACTGCACGCTGTCTTGCTTTAAACCTGTCTTTCCTGAAAAACATGATAGAATAACTTCTTTTCAGGAAC
CATCTGGACATTTCTTCTCTGCCTTCGTGCAACCAAACCAGACATTGGGCTGGGGTGGTTACCTCAGAGGGACATGAGGC
TTTGCATGTGGCTTCCGTCCTTTCCTTGTGATCCAAAATGGTGACTGGAGTGCCAACCGTCCTTTCTGAGATATAGGAAA
GAAAAGAAAAAAGTGAAGACAAACGGACCTTTTACCATGCCTTTTAAGTAGTCTTTCGGAAAGATACGTACAGGGCTTCC
CTCATGGCTCCTTGGACAGCATTTAATCAAGTGACCCTGCCTAGTGGCAGGAGAGAGTAAAAGGTATTATTACTTTTATT
CTTGTGAAAATAATCCCCAAAAGGCAAGGGACAGTGAGGGAAGCTGCCACTGCTGGCCATGCATATCCCTGATCCTCAG
CTTGTTCCCCTTTCCAGTGCTTCCACGGTGTTCTGGAGAGTGTCCCCTGCCTTGTCCTTATCCCCATTTTTGCACATCCC
CCTCAGTTGGGTTCTTCTCTGTGGTGTCTATTCTGTGGTGTCACTCATCATTGCACTGATCTTTCTAAAATCCCGAGTCT
CCTATCTTAGGACTTCTTGTTTTTTGTTTTTGCTTTTGTTTTTACAACAGCAGTCTGTGTTTAAATAGCTTTAGGCTCTG
CAGTAACTAAGGGATACAAGCTCAGAGATTCAACTTTTCTCGCCTTGGGCTCTGTCTGGTCAATATAAGTATGGCAGAGC
AGTCTGAGTTGGGGCAGACATAGGACACCAACGATCAGGAAACACAACCTCTCAGGGAAGCATGGTGAGAGAGGGCGGGT
CCTCAGCCAACCCACCTGGGCTCTGTCCCAGCCTGCCTCACACAGCTGTGGCACTGTGCCCACTCACTCCACATCCCTAT
ACCGTTTCCTGTCTGCAAGGATTCTCACAGCTGCCTTGATCAAGATGCATTGATTTCCCCAAAGCGCTTTGCACACTGGT
TGATGGTGGGCATTAAACCTCAGGCGCCTGCTCATGGATTCTCTGTGTGTATTTAAATACTCTGCTGTAGGTGTTTGATG
ACACAGCTCTGTTGTCTTTTACAGTAGATAATAACTTATTCTCCATGATTTCAATTGTTAGGGACTGAATACTCGTCGAC
CCCAAAACCCTTATGTGGAAACTACTTTCCCAGGGGGAGGATACAGGAGACTGGTGTCCTTCTATTAGACATCCCAGGAC
GCTCCCTTGATGCCTCCACTGTGGCAGTAGCAGCCGAAAAGCCTATGAGGAAGAGGTCTCATCTGACCTCAAACCTGCA
TGTGCCTTGACCTTGGCCTTGCCCACTTTCCAGAAACTCAAGAAATAAATGTCTGTGGTTTCTATGAACTATGGGTGGTCT
TTTGTGACAGCCTCCTCCCAATGAAGACATTGGTCATTGATACATCTGTTTTGTTCCCCCCTTACTTGGTGACTCTGTTG
GCTTTATGAAAGGCAGGCAGTTGGCTGCAAACCCTTTCATCCCTCTTTGTCACAGAATCTTTTGGTTACTTGTAAGGTTG
ACAGCTCAAGGGCACCAGCAGCAAAGGTTTGATGTGATTTAGCATGGGACTTTTTAATTAACCTCACATATAGGGAGGCT
TGTGAGACAGAGGTTTGGGATTTGTGTGTGTTGTTATTCAATCATCTTAGCATTTTCCAATGGAACATTCCATCATTTTA
TGCTCTTTCCTGGTTCCTCCTTCATTCATATTGAGCATTATGGAGGTAAAATGACACTCGTTGCAGTCAAGGGGTCCAAA
GAGTAGAGATGTATATGGCTGATAGGTAAGAGATAGATAAAGATAAAAACATAGATAGATGATAGATAGATAGATAGATA
GATAGATAGATGATAGATAGATGATAGATAGATAGTTGATACATGATAGAGAGACAATAGATAAAAGATAGATGATAGG
TAGATAAATTATAGACATAATGATAGATGATGAAAGATAGATGATAGATGGATAATAGATAAATAGATGAATAGATAGA
CAGACAGACAGACAGATAGTTGATACACGAATAAATAGGCACGTGATGCAGGCACATGGATGGTTGCAAGAATAGATGTG
CACATACAGAGATGAATAATACATAGGTACATGGATGAACTGATAAATGGATGATAGAAGGACAGATGGGTGTAGGCTGT
GTGCATCTTGACTCAAGTTTCAAATGCCGGTAGGAGAAGGGGTCACAGCAATGGCTCTGCAACTCTGGTACTTCCCAAGA
GAACTCAATGAAAACAAGGAAATCCCCAGACTTCAGAGAAAGGGGAACTGGGAAGCAGAGGGGCCTGTGGGAGACCTGAT
GGTAAACTAAGCAGAGGCAGGGAGAGCACCAGGCTCTGCCTGTGCTGGAGAACATAGCTGGGTAGGTGTGTGCAGACAGA
AACAAGAGAGCAATCACACCTAGGCTTGCCCCGACCCCACGGCCACATCCATCAAAGGTGTGGGTCTCAGCTCCTCTATG
TGGCCTGGTGCAAGCTTAACAGCAGCTTCACTCTCACCTCTCCACACTGCAGTGTGGTCTGAGGAAGGATCTGCTGCCTC
AGAGGCCATCAATCCATCTCTTCTATGTTCCTGCTTTAGCACAATGAGGCTTAAGAGAAGGGAACAGCTCTAGGGCAGCA
TGCATCAGGGCCTCTTCTCTGGGTTCAGGTCACTTGGCATCGCAGGGTACTGGACAGCTCTCAGCCTTACGCATCTTTTT
```

```
ACAGCCACAAAGGATGCAGTCTAGGAGGGAAGAATCACAAGCCCTGTAAGATGAGTGGAGCCAAACCCCCAGCCAAGCAC
CAATACAGAACCCCGGGACAATGAGGACACCCCCCTGCCCATAGCTTCCAGTGCAGCCACCAAAAGTGCCAAAATGGACC
AGCAAATGGCACTCACATGGGGGCTGTGCTACATGGCACTGGTGGCTCTCTGTTGGGGACACGGGGTGACAGAGGCAGAA
GGTAAGTCTGAAGACACCTGCCCTTTATCTCCCTTTTTAGTGTTGAGCCTGGTGGGATCTGAACCCACCATAAGCCCAGC
AGCCCCCAGAAACCCTGCAGCTCCACCCACTGTATCCAGCTCTGGTTTTTCCTGTCTCTCCTACATTCTTTCTCTCCTTG
AGGGTGCCCCTGGCCCCTCTCTTCCCTGTTCTTCATATTCCTGGTCCTCCTTGTCTGGCTCTGGAGCATCCAGCTCAATG
GTGTCCCTCTCCAGCCATATTACTCAATGCATCCCCTAAAAACCTCAACCTCCAAGGAGCCAGCATCTTCTCTGCCTCTCC
CCCACACCCCCATTCCTTTAAGGACTGCCAGGACGCCTAATTCTTGGTCTTGGGAGCTTCAGCCTAAAGACCTGTCCCCA
AAATATCTCTAGCCTTCTCCTCTATCCAGAGGCCCTATGGGATGCCCTGTTCCCAGTGCCCCATGGATTTCCTCCATGAC
AAGGTAGGAGCCACTTTCTCTTTCGAATCCCAGCTTTAGAGAGATGCAAAGGCTTAGCAGATCAGGACCCTAGAAAGGGA
TATACTAGCTACTTCTCAGGCACTGCTGTGTCCGTAATGGCCAAGTGACACCTCAGAGAACAGGGTCAGGCCTGAGCAAT
GCCCTACATGAGGGCAGGTGCAGCACAGAACATATATCATGGCTGAGAATCCACTGGGATCTCATGTTCCTGACAGAAAA
GGAACGTCCTCAACAGGATCCACCACACAGCCACACCAGGCTCAGGCCCTCTACCTGGTCACCTGGGCCTGAGCAGGAAG
CCAGAGACCAGGGCTTTGTGTGCATGTCTTTAATGGGAGCCCTCAGGAAACAGAGGCAGGAGGATCTCTGTGAGTTTGTC
GCTATATGGTTTTCAAAGGAAGTTTCAGACAGCCTGGGTTACATAGAAAGACCCTGTCTCAAAACAAACAAATGAAAAAA
ACACTCCTGTGGGACAGGAGTGAGGGGGATGGGGACACCTGGACACCTGGAGGGGAGAGGCTGGCTCTCCTGATCCTCTG
CTTCTCCTTGCCAGAAACGGTCCCTCTGAAGACTCTGCAGTGCTACAATGACTACACCAACCACATCATCTGCAGCTGGG
CGGACACAGAGGATGCCCAGGGGCTAATCAACATGACCCTCTATCACCAGCTAGAGAAGTGAGTGCCACTGAGCCCATTG
GTATTGCTAGCCACCACCACCTCTCTCCCCACCACTGTGTTGTCAGTGATTGTGGCCATCATCCCCGGTCACCTGACCTG
TGGGCTACCCGGCCATCCCAACACCTGCAGCTTCTGCAGCTCTAGACCTGTCCAGCCCTGAGCATCCTGTCTCTCCTC
CACTCCTCATCAACCTGGCCCGAGGAGCTGCTGATCTCATAGTCCCCCTGTCTCCTGTATTCCTGGCCTGAACACACT
CCCCCAGGATTTGACTATCTCCAAGGAAACCAAACTGCCCCTGCCACTGAGAAATACCCATAGCCAAATGGCCAGGGCCG
AACCATGTTCTCCCAACCCACCCAAGATGACCTCCTGTAGCTTACCTTTACTACTGTCATGGTCCCCTGATGTCTCTGAC
ACCTCTTTCTGCTCTATTTGCTTCCCTTTTCTCTCCTGTCTACTGTAGAGTGTGAGATCTGTAAATGAAGCCCTGGCTTC
CTATCTACATGCAGTCATTTGGGGGGGGGGGGGCTCATGGGCAGAGACTCTCTGGGGAGAATGGCCAACTTTAGCACAGAC
ATCCCTGCCTAGCACACAAGCATCTCTCTATGCTTTTTATTTGAGTAATCCATTTCTCTGACTCCAAATGCCACAAACTGG
TTTCCACTCTTGCTCCAGGGATTGTCCTGCTTCTGCTGTCTCCACATCTCACTTTATTAGCTGGGAAGAGATGACAGCTT
GACTAAATGACTGTGGAGGCTGGTTTTAAAAGTCTCCTACCTCCTTTTCTTCTCCTTATTTAACACTACTAAGTTTATCC
CCCAATGAAATACACGTCTGACCAACTTGGAAACCACCACCAGCATCCAATCTACTTAGTATGACCAGGATGGAACTATC
TTGCTAGTTCATCATCCTAACCAATCAATCCCTGCACCAAAACACGCCCCTCACTTTATTACCCTCAATCCCTGCCTGCT
TGCTACTAACAAACACATGAAACCTACTTTCCTTGCCAGCAGACCCATGACTGTTTTGCTTACAGCAGGAAAGCCAGTAC
TGAGGGACAAGTCATTAGGGCCACGGATAGGACAGACTGACAAGCATACAAACTAAAGAAGATGCCAGCGAGGGAGTTGC
CCAAGAGCACCCTGCACTGACGTCACAAGTGTAGGAATTCAGTGGGGACTTGGAGAGAGGGTCCTTTTGGTCTCAGGGCT
TTGAGGCAGTTACCAAGTGATCCTAAGGTTCAGAGTGATCTGGGACACAGTGCTACGGTTATATGAAGGTCACTGGCCGT
CCTAGGTGCTGGACATCTACAGAGAAATTACTAGTCTGCAAGCTTTGTGAGCTTGTCTCTTCAGTTTCTTTTCTAAGGAA
TAGTAACCTTGATACAGGCGAAGATACAGAAGAGGGGAGGAGAGCAGACAAACTTCCCCTAGAGAATGACCTTGACCTGG
TTATCTGACCTTGATCAGAGGTGGTGTGCACATGAAAGCTCACCCAGAAGACCAAGAATAACACACTGCTCAACAGCATG
GCTGCCAAAATTAGTGATGTTTAAAGTCAACTAGAAGTCTCTGAAGCTAAGGACGTCTTAGGCTCCACCTGGATGCAGTC
AGATTTCTTGTGATGTGCTGTAAGCAAGCTCTTTAACTAGATAGGTGCTAGACTGTACATGCGCCAGTCTATGCTTCCAG
TTACAGGCCTGGTGGCAGCTTCAGGGACTTTGTACTTGCTGCAGTCAGCCTGCTGCAGAAATTGCCTGCTTTCCCTGCAA
GTCCTCGGAGATTCTGGTCAAACTGTGCATGCAGTGAGTCTATCCGTACTCCCCACAGACCCTTCCAGATTCCTCAGCCT
TGTCTGTTGTATTCAACAATTTCCAATGACAACATACTACTTTGTGATCCAGTGTGTGCCTGTTCCTTCCATCAGGATGC
TCACTGCATGTGTGTGTGTGTGTGTGTGTGTGTGTGTGTGTGTGTGTGTCTGTGTTGTGTGTATAAGGTGTATATCTTTTTTA
TATGTATATGTGTTTGTATATGTGTGTATAGGTGAGTGTCTCTGTCTGTGTGCTTGTATATATGCTGTGTGTGTCTCTGTGC
TGTATGTCTGTGTGCATGTGTGTCTGTTTCTGTGACTCTCGTGTGCTTGTATATATGCTTGTATATATGTCTGTGTGTCTGTGTGC
TCATGTGAGTCTCAGTCTCTGTATTTCTGCATGTTTCTTTGTATGTATATGCATGAGTGTTTGTGTGTGTGTGTGTTG
CTCATTGCCAGACACTGCCCTTGGATAAAAAGCAGGGACATAGTAGAAACTAATGAATGCTCCCTAACAGAAAACAGCCAG
TGTCCTGTGAGCTCAGTGAGGAACTCATGTGGTCAGAGTGCCCGTCATCCCACCGCTGTGTGCCCAGAAGATGTGTCATC
CCCTATACACGATTTTCCATCACAAACGAAGACTACTACTCCTTCCGGCCAGATAGTGATCTGGGCATCCAGCTCATGGT
GCCACTTGCCCAGAATGGTGAGGAGCGGAGGTCCTGCCTAGGCAGTGGGTGCAAGAGTGGGCAGGATACTGGGATGGGCT
AGAGTGAGGTTCTCCCAGAATTAGGGACCATGTCTTGGGGTTGGAAGAGATGTGATGTGGGGACTCTGCCCAAGGATGAT
CCTGTTCCAGCTGCTTCTGCCACTATCAAGCCACAAAGCTTCCTCCACAGGAAGAGCTGCCTGTGCCTCCTATAGGA
CTTTCCAGGTGACAGAACTCTGCTCTCTCTTGCTTTCGCCCATGGCTGCAACACCTTGCCCTATTGCAGAGGTCCTCTGA
TTTTTGTGGAGCAGAGATGACCACAATAGGTCACATTGTCAGGCTCTTTCCATGCTTCTGACCCAGAGCCAGGGTCTGCG
CCACCCTCTCTGGACCACCGAGTGTGCAGGCAGCAGGCCCACAAGGCCCATTGTCACAGCATGACCTTAAGTGGGGCCAA
AAATCATCTGTGTGGTTCTCTTGGGGACTGTAGCCGACATGTCACCCACACTGCAGAATAATGGGAAAACCATGGCTTGT
ATTGTTCTTCTAAGCCCCAGCTTAGATGTCAGACTCTCAGCGGGAACACTGTGAGTCCTCTTTGCCCGGCCAACAATGTTAG
ATTGAATGCTCTCATCACATCATTGCTGCTGGTGTCTGAAAGAGTCCCTGCCCCTGGGGCTCAGGGTGCAGACTGAAGG
GTTAGGCATCAGCTGCCACATCCAGGGATGGAAAGCAAAGGGCTCTGGCAATCAGGAAGGGCTTCCCAGAAGGAAGGAAG
GAGCTCTGCAGCCAAGTCCTTGCTCTGGGAGGATCACAGAGCCAGAAGCAATTCTTAGCCAGGAAACCACGTGTGCAAAG
GGGGCTTGCAGGGGGAGATCTGAGGGAGTGGGGCATGCTGGGTACACCAGGCATGTGCACTGTGTTCCAGGAAGCATCGA
```

```
GTTCCAAAGGCTAGACAATGGCAGGGAAATCATGGGATACAGACAGCTGCATCTCTAGCCTTGAACTAGGGAAGGGGGGG
ACAGGGAAAAGAAACCCTTAGCCTGATGTGACAAGACAGACATGGGGTTACAATGAGCTGCAGTGGGACAAGAAGAAGGA
TAAAGGCTTCTGTCTGGGAACTGGAGTCCAAGAAAGTCATCAGGGCGGTCCTACACTTATGCAGGGCAAGGACAGTCAGT
GATAGGCACTTGAACTGTGAGGAGGCTGTGTGGCAGGAGAAGAGGCTCCCTGGCTCACAGCACCGTCTGCTGGTCAGAAA
GCATCAGTGCACAGCAGGGCAGGAGGAGGCCCCACTGAGGAGAATGTAGGGCAGAATTACTGTCAAATCTGCAGTTTGCA
GGTTTCAGGCCTGAAGCTGAATGGTGTGAGCCAAAGTGGCACCCAGATCACCAGGCCACCCGCAGCTCCCTCGGTGTGCA
ACCCGATTAACCCTCTGCTTCCCTCTCTATAGTGCAGCCACCACTTCCCAAGAACGTCAGCATCAGCTCCTCTGAGGATC
GTTTCCTGCTGGAGTGGAGTGTGTCCCTTGGGGATGCCCAGGTCTCCTGGCTTTCATCAAAGGACATAGAGTTTGAGGTG
GCTTATAAGCGGCTTCAGGACTCCTGGGAGGTAGGACCCTGAGCCGTCTGTGTCCACCCTTGGGTGATGGCATGTCAGGT
TCCTCCTTCAGCTCCCATATCTCCACAGGATGCCTACAGTCTCCACACTAGCAAATTTCAGGTGAATTTCGAGCCAAAGC
TATTCCTACCCAACAGCATCTATGCTGCCCGTGTGCGCACTCGGCTGTCCCCGGGTTCAAGCTTGTCTGGGAGACCCAGC
AGATGGAGCCCAGAGGTTCACTGGGACTCCCAGCCAGGTAATGGAGCTGGGAGGACATGCCCCTTTTAGGAGACTAGAAG
TGGGGACAGTCTAGGTCCGAGTAAGGCTCCAGGATCTGCCCTACCTCGACATTGGAGCCTCCCACCAGCCCTGTGTCCCC
CGTAGTCTCTCTGGGCCACTCTGTGTCACACACTCTTGGCTCTCTGGCAGCAAACATGTTCTGTTAATGACCTCAATAGC
AGCAGCAGAGGCCCATGTGTTTACAAGCGCTGGGCTGTGTGTAGGGTCACCTGAGTCCCTGAGTTCATTCAAACACTCAA
TATTTAGAGCTGGTTCCTCCCTCACGATGGCGCAGACGTGCTGGACTCTTCCCTAGCTGGGGACACACAGCAGGCAGGCT
CAGAGCACAGTGGGCTGATGGTCACAGCCAGGTCTGCTGCCCTCAAGGATCTCCTCTGTGTGATTAACCTCGTGCCCCAA
CCATTGAGGGAGGGTGGACTGACTGAATGAGGGTATGTTGCCACAGGGGACAAGGCCCAGCCACAGAACCTTCAATGCTT
CTTTGATGGGATCCAGTCCCTCCACTGCTCCTGGGAGGTGTGGACCCAGACGACTGGCTCTGTTTCCTTTGGGCTCTTCT
ATCGCCCCAGCCCTGTAGCTCCGTGAGTGTCCCCTGTCCATCTTGCCTTGTTTCTTCTTATCCCCAGGACCATACCTCTT
TGAGGTCTTAGATGGATCTGACATTCCCCCAACCCTGCTCTCAGCTCAACTTATTTCCCTAGGGAGGAGAAATGCTCTCC
GGTGGTGAAGGAGCCGCCGGGGGCCAGTGTCTACACCCGCTACCATTGCAGTCTACCTGTGCCTGAGCCCAGTGCACACA
GCCAGTACACAGTCTCTGTTAAGCACCTGGAACAAGGGAAGTTCATCATGAGCTATAACCACAGTGAGTGTGCCTGCCTC
TGTCTGAGTTCCCAGTACTATAGGGGTCCATTGAGTTATGCCAATAAATACAAGAGCCTCTTAGGCATCTTTGCTTTTGC
CACTTTCAAAGAATTGCAGATTTTTCACTCAAAGTGATAAAAATAACTATTTTTGGATTAGGTGTATGTCCCAATGGGTG
AAAGCCATTGATGAACTGGCTTGAGGACGTGAGTTCAAATTCCAGCACCCACATAAAAAAAAATTGAGCGTGATCATATC
CAACTGTCAAGAGACAGGGGGATAGTTGGGGCTTTCTCGATACTCACCAGATCTGGGTTCAGTGCAAGATCCTGTCTTAA
GAACACAGAAAGAAGACAAAAGCCATCCTTCTCTGGCTTCTGCATGCCTGTGTTTAGACCCTGTCTTAGGGTTTTACTGC
TGTGAACAGACATCATGACCAAGGCAAATCTTATAAAAACAACATTTAATTGGGGATGGCTTATAGATTCAGAGCTTCAG
TCCATTATCATCAAGGTGGGAGCATGGCAGCATCCAGGCTGGCATGGTTCAGGCAAAACCTAGAGTTCCACAGATTCATC
TAAAGGCTGCTAGCAGAACACTGAGTTCCAGGCGGCTAGGATGAGGTTCTTAAAGCCCACACCTAGAGTGACACACCTAC
CCCAGCAAGGCCACACCTAATAGTGCTTCTCCCTAGTGCAAGCATATACAAACCATCACAGTCTCCCATACCCCCATGGA
GGCATATGCAACAGAGTGAGAGATAGAGACAGAGAGATGGAAAGAGACATTGAAAGAAAGTTTATTCTAATATGTTTCTA
CTGTCTTCAAGGACAGGGAAGCTACAGGCAAGCCCTCCTGCAGACTATACTGTCTGCCCTCAAGAGTCTCACCTGGGACC
TGCTCTCCTTCCCCTCTGCTTCCACCCAGGTTCATGCCTGCCCCTTCCCTGGACCACTGTCCTTCTATACCTGTCTTTTG
CCTCTGAGATTTCCGGCTCCTCCTCCAGCTAATGTTCACATTTCTCCCATTTGGCTCTCTGTTTTACTTTCCTGCCTGGA
AGGAGTAGATGCCCACAGATGTCGTCCTTACAGACCTATGTCCCACCTTCCTGTCACAGGGCAAGGACTCTGACACAAGG
GAGACTTTCCAGCCAACCAGTAATGTTTGTCCTCTTTGGTAGCCAATCTTTTTGTCACCTGAATTCATATCCAGCATTTT
CTTCCTGGGAGCCTTGCTCCAGGAAAAGGAGGTCCTCTGATTCAAGGGACTCCTCCATGTTTTAGAGCCATCTGAATTAT
TTTTGTGAAAGCCCTCTAATGTCACAAGCTATGACTCTTTTGGGTTTTTTGTTTTGTTTTGTTTTTTTTATTTTGTTTTT
TTTTTATTAGATATTTATTTCATTTGACTCTTTTGTTTTGATGGAGAGATAGAAGGTGATTGAGTGCCTTACTTTAAAGT
GCCCACCTCTCTGAGAAGGCACAATAAGGCGACATGAGGAAGCCTGAATTTGTGCCCAAGTCTCATTTTGTTGTCTACTC
TTTCAGTGGACGGGGCTGTCACCAAAGATCCCAGCTTTCTGTTTGTCCACAGTGAGATGGTGAGATGGTGATTCGGGTGT
GTTTATACAGCCATGTAGGGGACACTTTTAGAACAGCAAGGACTTGCCAGCCCGTGCTGATGTCAGGCAACTCCCAGAGT
CACTGAAGCAGCAGAGGGGAGCAGCTAACACAGAAAAATAGAGACAGGGCTCTCCACTGCCCAGTTTTATCTTCTCCAGA
CTCAGTTTTCCCTGTGTAAAATGGAAATGAAGGGACCTCACAGGGGGAAGGGGAGGATGGAGTGCGGGAGTTCCCACAAA
CTGAGTGTGTTGCCCAGGGCTGGAATAGTCAATGGACCAGGGTAGAAAGCCATGGGCTCTTTCTGTTTTTATTTTTTATT
TTTGTATTTAAATCAAATACACTAGCAGGGAATCACCAACAAGCTTGGTCCACTGTGGCTTACTTCAGGTGCTCCCCTCC
CTCATGGCCCAACATGGGGTAACACTGTGATGCAGTAATGATGGGCATTTGCCTTGGTTACTTTTTTACCATCATTACCG
AGGTAACTCATAGAAGAGTGTTTACTTTGGGATTAGGGTTCCAGTGGGTGAGAGTTCATGGTGCAGAGCAGTCACAGCAG
GGAACAGGGGCAGGAAGCTGGAGAAAGGGCTGAGAGTTCATGTCTTTTGTTACTTGCTTGCTTGATTGATTGCTTGGTTG
TTGATTGGTTGGTTGGTTGGTTGGTTGGCTAGTTGATTTTTCAAGACAGTTTCTCTCTGTAGGTCTGGAATTCACCTAGC
AGACCAGGCTGACCTGACACCCGTGTACATAGTCCTGACTGTCTCCTAAGTGCTAGAACTAAAGTCATGAGCCATAATGC
TCACATCTTGAACCACAACCCAAAAACAAAGTACAAATAAAAGCCCCAGCCCCATGACCCACTGGCACATTTCCTCCAAT
AAGACCACACCTCCGCAACCTTCCCAAACAGTCCCAACTGGGGACCCAGACCCAAGGACTCAAACACATAAGCCTATGGGAC
CATTCTCATTCAAACAACCACAGAGTTCAAAACAGTCAGAATGGCTGGGGTTAGGGATATTGGACCAGCTTGGAGAATTC
ATTAGACAAGCTTGGATAATGGGGAACATCCTGCTGGGGGAAGGGCATGATGGCATTCCCTGAAATGATCCTCCCTCTAG
TCCAGATGGAGCCTCCAACCCTCAACCTGACCAAGAACAGAGACAGCTACAGCCTGCATTGGGAAACTCAGAAGATGGCT
TACTCATTCATTGAGCACACATTCCAGGTCCAGTACAAGAAGAAATCGGACAGCTGGGAGGTGAGTTCCAGGACCCAGGG
GATGTTACAGACAGGGAGTAGAGGAAAGAAAGGACATTCTAGGGAGCCCTACTTGCAAAGGGGATGACCCCTGTACCTTG
GCACCCACATGGTCAACAGGAGACAGTCCAATTTCTTGATGAGCGATTCAGGCTCAGAGAGGTGACATGGCATGGTCACA
```

```
TGACCAGTGCTGCCCTTGAGCTGAGGGGCCAGACTGGCAGAGGAATGGAATGAAGGGTTCAAAGATCACCAAGGCCTGGG
TGTATTTCCTCCATGGAAGAAGATCTGACCAGTGGGACAGCTGCTGTGCGTTAAGCTGAGGGCAAGTCCTGGCCAGCATG
CATCACACATGCAAAGGCCTGCCAGCTAGATGCAGACCCTGGGGCGCCACAGAGACTAAAGCAGGACCAGTCAGCCAGGA
TTCTAGCTTCAGACAGGAAGTAGGGAAACTGAGGCAACCAGAGAGTCAGTCCTAACCCCAACTCCTAAGCTAGGACCCAG
CAAACTAGCATGGTATTCCTGGACCAGACAGAAATTAGGCCAAGGCACAGCAACAAATTGGGAGGTGGAGATAGCCTGGT
CTTGGGCCTGGATGCAGCTGTCAGGGATGGAAGGGCTGATATCTGGGGCCACCACAGTGGCTGGTTATAGTAGGCTCTGG
AATGGCATCCTGGGCTGGACTAGAGAGACAACCAATTTGGGGGTAGTTGCCAAGAAGGCAGCTGAGCCCCTGAACCTGGC
TGGTGGCTCAGCTTCAGCACATCTGCCTCTACAGGACAGCAAGACAGAGAACCTAGATCGAGCCCATAGCATGGACCTCT
CCCAGCTGGAGCCAGACACCTCATACTGCGCCAGGGTGAGGGTCAAGCCCATCTCTAACTACGATGGGATCTGGAGCAAG
TGGAGCGAAGAGTACACTTGGAAGACTGACTGGGGTATGTCCCTCGGCCACCACCACACTCCTGGGACTACATAGGGCAA
GGGAGGCAGAGAGCCACTCTACCCTGTGTCCATCCCTGTGGGATACACTTGGTACATTACACAACTGTAAGTGGGCCTTA
GGGAGAGAGAGGAGCTAGGGCCCAGGCCTCTATTTATATAAGGCTCTTGGCTTTCTTAGCCTTTGTGGAAGCCATGATAG
GCCAAGCTGGGTTTCTTGTTACAATGGCTGCCAGCTAGTCTATCTATGTACACAGATAAATTGGAAGGTTGGTGGGACCC
ATTCGTCCTGCTGATTTCCCTTCCCTTGAACCCTGCAGTCAGTGCTGGTGTGTCCCATAGTGACTGCCTGACACACACTG
TGCAAGGTGGATGTGGGGTGGGGGATGGTAATGAAGCATTCCGCATAGTCAGCCCTGTCAGAAGGGGGGATCTCAGAGTG
AGAGTCATTCTGGAAGAAAGAGGGCTAGAGCCATGAACTCCAGAGATGGGGAGGGGACTTACAAGGCCCCATTCAAGAAG
GCTAGGGGACCTTCTGCTGGAAACCTTCCCATCTCCTCTGACCCTCCCTGCATCAGTAAGCCATAGCCCATGCTCCTCAT
TTCAAGAAGTCCCCCAGTTGACAAGCTCAGTCACCCCCCACCCAGGGTACTTGCTGCTGCTTAGGGCAGAACTTCCCCAT
ATTGAAGACAGGGAGCGGACCCAGGGTTTCTTCCCAGGTTCCTGGCCTTTCTCACAGAGGGCATGGGAGACACTAGCTTC
CTCCCTTAGCCTGCAGCTTGTGCCTCCCATCTAATGCTGCTTCTCCTTGTTGACCAAGTGATGCCCACGCTGTGGATAGT
CCTCATCCTGGTCTTTCTCATCCTCACCTTGCTCCTGATCCTTCGCTTTGGCTGTGTCTCTGTATACAGGTAAGTGCACT
CTGTGCGCAGAGGAAAGTGGAAACTGGGCTCCGGGGTCACAGAGATGACACAGCTGGATACACTCTCGTTCCCAGCCTAC
CATTCACTCCCCTGGTTTCAGTTTTCTCTCTATACGTGGGCACAGAAGTAGCTGGCACAGAGGTCTATGGAATCTCACAT
ACACGTGTCCCCACCCTCCTGCTGTCTTCCAGGACGTACAGGAAGTGGAAGGAAAAGATCCCCAACCCCAGCAAGAGCCT
CCTGTTCCAGGTAGGAAGGCAGGCCCTGCTGCTGGCTCCCTGTCCCATTCCTGTGTCCGTGGGAAAGCGAGGGGAAGCTG
GAAGGCTAGGCAGCAGGAGGTGGGTATCTGGTATTGCCTGGGTGCCTGAAGGAGGAGTCTCACAAATTGGCCTGCGTTCT
GTCTGGAGCCACAGACAGAACTCAGAGCCTTCTAGTGCACAAAATGGCTCAGAAGAGTCGGCTCCTGGAGGTAGAGACAG
GGCTGAGTGTCTCCAGGACAGGACTTGAGCACTGCACAGCAAGATGCCTTAGAGCTGGTGGGAGAGCTGATTGCAACCAG
GGTGTCTCCAGGGGGTCAGCTCCTGAGACAGGAATGGGAATGACAGAAGGACACGGCTGTCCATGTCTCACAGAACAATG
GTCATGGGGAGACACCAGTGCCATGAGAGAAGAGGCTATAGGGAACAGCATGGCTGCTTACCTCAGGAGCTTTAGGGCTA
AGGGAACACAACCCCTAGGATGCCTCTTCTTTCTTGGAGTCAGAGGCCTGAGCTGGGCCCTACCAGGGGGACATTCTTCC
CATGTCCAGAGCTTATGGAAGACACCATATGCACATGTGTCCTTCCTGAGGTGCCACATTCACAGACACAAATGCCCTTT
CCTTTGTAGGATGGAGGTAAAGGTCTCTGGCCTCCTGGCAGCATGGCAGCCTTCGCCACTAAGAACCCCGCTCTCCAGGG
GCCACAGAGCAGGCTTCTTGCTGAGCAACAGGGGTGAGTGTTTGGGCTCTGACCCTTGGGGAGCCTGTGGGAGGGAACCT
GTCCATGGTGCACATCATTGTAGCCTGTGGGTCTCTGAATTTGAGAGGAGAGGCTGCCCACTGTAGTAAACAGCACTCCA
GGTAGACATGAGGAAGACTTTTTCTGACTCCCCGGTTGGCGTGAAACAGACAAGGGACCAGAGGCAGCATCCCAGAGCAG
GAAGCCTCTCCTTGCCAAGTGACCTATGTAAGCCCTGCACTGCAATGGCCTCATGGGTGACTGAGTGCCACACTGTGGG
ACTTCAGCCTGCTAAGCCCACATGTCAGGGAGTCAGCAGCCCCATATGGGCAGGTGTGAACCCGGCCCTGAAGGTTCACA
CTTGCTGCAAGGAGCAGAAGAGAGCAAACATTAGGATGGGGGTGTGAACTGAGCCCTGAGCACGTGACACTTTGGGACAC
TGGACACACACCATGTCATTCACAGGACCTCATGCCTCGGTGCTTACTGTGAGGCCCAGAGGGGATTCAGGCCTTCCTAG
AGCTCACCTGCTCTGGCTATACACTAAGTGCCTAGGTTCAACATCTCTGCCCCTATTGTCCCGACAGGGGAGTCATATGCA
CATTTGGAAGACAACAACGTGTCACCTCTCACTATAGAGGACCCTAATATAATTCGAGTTCCACCATCCGGGCCTGATAC
AACCCCAGCTGCCTCATCCGAATCCACAGAGCAACTTCCCAATGTTCAAGTAGAGGGACCAACTCCTAACAGACCTAGGA
AGCAATTACCCAGCTTTGACTTCAATGGGCCCTACCTGGGGCCTCCCCAATCCCACTCTCTGCCTGATCTCCCAGACCAG
CTGGGTTCCCCCCAGGTGGGTGGGAGCCTGAAGCCAGCACTGCCAGGCTCCTTGGAGTACATGTGTCTGCCCCCTGGAGG
TCAAGCGCAACTGGTTCCATTGTCCCAGGTGATGGGGCAGGGCCAGGCTATGGATGTGCAGTGTGGGTCCAGCCTGGAGA
CCTCAGGGAGCCCTTCTGTGGAGCCAAAGGAGAACCCTCCAGTTGAGCTGAGCATGGAGGAACAGGAGGCACGGGACAAC
CCAGTGACTCTGCCCATAAGCTCTGGGGGCCCTGAGGGCAGTATGATGGCCTCTGATTATGTCACTCCTGGAGATCCGGT
GCTCACTCTGCCCACAGGGCCCCTGTCTACCTCTCTGGGCCCCTCTCTAGGGTTGCCCTCAGCCCAAAGCCCCGTCTCT
GTCTTAAGCTGCCCAGGGTCCCCTCTGGAAGCCCAGCTCTAGGGCCACCAGGGTTTGAGGGACTATGTGGAGCTGCCTCCA
AGTGTGAGCCAGGCTGCCAAGTCCCTCCAGGCCATCCTGCTCCTCCTGTGGCAAGCAGCCCCACAGTGATCCCAGGAGA
GCCCAGGGAGGAAGTGGGCCCAGCATCCCACATCCCGAAGGCCTCCTTGTTCTTCAGCAGGTTGGGGACTACTGCTTCC
TCCCTGGCCTGGGACCTGGCTCCCTCTCACCACACAGTAAGCCACCCTCTCCAAGTCTGTGTTCTGAGACTGAGGACCTA
GTCCAGGACTTGTCTGTCAAAAAGTTTCCCTATCAGCCCATGCCCCAGGCGCCAGCCATTCAGTTTTTCAAGTCCCTAAA
GCATCAGGACTACCTGTCCCTGCCCCCTTGGGACAATAGCCAGTCTGGGAAGGTGTGCTGAGTCTGTCTCCTCCCAATCT
CACCAGCAGCTGGCACCGCAGCCTGTGGTCCTCAGCCTGAGCATCACCACAGAAGCCTCTCTGAGTTCACACTCCTCCT
TGCTCCCAGCCCTCGACATGGCAATACCCCACCTGTGCTTTTCTCTTCCCCCTCTGTCCCTCTTCCCCTCCCTCCATGCAC
CCCCATCTCTCTCTGCAGTTTTCTGCCTTCTATGGGAACTTCCTTCCTATTCCTCTGACCTGTTGGAGTGGGGGATGAG
GCCTGTGAGGGTATGGGCTGTTTAGGGTTTACAAACCTGTGAGGCCGGACGGTGGTGGCGCATTCCCTTAATCCCAGCAC
TCTGGAGGCAGAGGCAGGCAGATTTCTGAGTTTGAGTCCAGCCTGGTCTACAAAGTGAGTTCCAGGACAGCCAGGACTAT
ACAGAGAACCCCTGTCTCGAGAAACCAAAAAGAAAAAAAAAAAAGAACTGCTGAGGTCGGCTTGTCCACCACAGGCATTG
```

GTAATACAGCACATTTCTGGCTCTGTGTGAAAGGTGGGAGTCTCAGGTGAGCCCCTTGGGAGGTCAGACTGTACAATTGG
GCAGGCTCTTGTCGCTACAAGAATAGACATGGTCCTCTATGGTTGTCTGAAACTCCTGGGGAGACCTGGGGAACCTTGTA
GAGGGTACTTGGTACCCTCCTTATATACAGAAATCACTAATGTGGACAAGCTGGACACAGCTATGGCCAGGCTGGAGGGA
TGAGAAGCAATGGGAAGGCTGTGTGTCTAGAACTCATGGTCTCAACTGCAGACAAGCTTAGTTGTGGATCCTGGACAGCA
CACCCAGATACTAAGTCTAGACAGCTGAGCTGTTCGTTAGTTCTCCCTCCAGAGCAAAGCCTAGATACCTGTGCTCCCAC
CGGCCTCAGATGAGGGTCTACTGTGAGACTGTGGCCCTTGGCCATCACCACCCTCTTGACAATCCATGCACAATGCCATT
ACCTCATTCTGTTCTCACTGGAGTTGTTTGTTCATGGTCACGTTTGCACATGGCCTTTGTGTGTGTCTCGAGCATTGGCT
TTTTGGGTGGTAGCGAGGAAGTAGACTTGGAAGTTGAGCTCATGAACAGTTTTGTGCTTCAGACTGTCTTTTTTGGATGA
GACTGAATAGCTCTTCCCACATGGCCTCTTGTGGGCCACACGTGTCTCCCTGAGTGTCCTAATTTTGTGCAGGAGGCAC
ACCAGCTCTGTCAGCCATTGCGTGTCTGCATTGCTATGAGATGGGTTGCCGAGCCACACCTGTCTTTGGGTCCCTGTTTG
CAGGGGTCTTTGCTGTAAGTGCACACGTGCCTTTGTTTATGTTCTTTCTGGTTCCTTCTGTGTCTAAAGCCTTGAGTGAG
AGACTTGGAGTTTTCTAGAACACATGATCTCTCTACAGAGTTCAGTCTTGATGTGTTCTATGGACCTGGAGTCAATGGAC
ATTGTATCATTTCTTCACCTATTTGTGCTCTGTCAGGCACTCCATGATCCACCAAGTCATGAACTGGATCTGGTCTGTCC
TATCCCCTGGCCTCACTCACATGATGGCAGACATAGAATGTGGCAGATCAAATGTCCCTTGGTCTCTGTTCATTGCAGCA
AAGAAAACCACTAACATCTTGAGTGTGTTGCAGTGGCAATGTTCAACATCAGCCTGTCAATACTTTCCAAAGTGACCACT
CTAGAGAAACAAACAGGAGTGGCCTCCATGTTAAGATGTGAATGCCAAAGTGCCTTGCATAAGAGGAAATATTGTAATAA
ACATGTTTACATACAATAAAACAGCAAAACAAGGCAGTGTGTGTGTGTGTTTGTGTGTGTATGTGTGTGTGTGTGTGTGT
GTGTGTGTGTGTGTTTATGTGTGTGTGTTGGAAGGTTGGGGATGGGGACAGTTCCAACCCATGGGTGCCTTGTCAAGTAACT
GGGTTCCATAGACAGGGCACTTGATACGCAAGACCCTTGCACCCTCTCAGAGGCGAATAGGATGGGACATGGGGTGGGAG
TGGGGACTTGTGGAGGAGGAACCAGAGGGAGAACAGCAATCAGAAGGTAAAGTGCACAAATAAATTAACTAATGGAAAAA
TAGAAATATAAATATTATTGAAGGCTTCAAAAGGAAATCATAATCACTTAGTTTCCTACAGAAAAGGCAAAGATCTACAT
TGGAAGAAAGACTAATGGTTCTAACACAACACCACTCTTCATGCAGAAGGATGAAATTCAATTCAGACCTTCCACTACAT
ACAAAATAAATTCTAATTATATCAATTCTGAGGGCTGATGAACGCCACCCTCTCTGTGTCTTATTTGGGATCATGATGAC
TTGGGGAGACACAAATATTACACCATCCTTCGAGGTTAAGAACACCTAGCATGGTGTGTGTGTCATTGTGGAATGCACCA
CTGGGGAAGAGCTGGTGAGAACGTTATGAAATTTGAGTAACTGAAGCAGAAGAGGCTCAGACAGTCTTGAAGAATTTAAT
TCTTACCAAAACCATTTGCACACCACTGGCATTGCTTACGGAAACTTCAGACTTGGGAACAGGATAACAGATGTGCAAGG
AAAAGTGAAACTGACTTTGATTTGGCCAAGCTATTGAACAATGGGGCAAAGGGGAAGGGCTTCTGTAGCAATACAAGACA
TTCTGGCTCCAGAACTCCTTGGGCAGTTTCAGTGTGGATCTGCTGCTCAGTGCTAGCATTGATGAACACTGTTTTCTGGC
TGAAAGCCTTGGTGTCCTGGCTCTAAAAGGATCTCACCTAATTTGGGGTTGGGGGGAGGTTCATAGTTGAGATTCACCTT
CTCTGTTGCAAGAATGTCAAACTCCCAGGCTCTAGGAGTGGTGACCTCACATGGTCCCAGAAGACACACTGATCTCCGGC
AGAGTTGCCCTCCCAGTGTGGCAGGACAATGACTTCAGGAGCACAAAGGAACCTGATAAGGCCTAGGAATCAGCCAAGGG
AGTGCTAGTGCTCAGACTGATAAAAAGCCTAGGCTCATTTGGGCAAGCATATTTTTAAGAGGCAGAAGGACTGGCCCATC
ACCTCCCAGAGTTTCCCGATTAGATTTAGAGGAAATAACATCACAACTCTAACACTCAACTCGAGAGTTCTAAATCAAGC
AAGGGCTAAAGCCAGGATGCAGTTCCTTTAGAGGGTTGCTGGGATTCTCACAGAGCTCACGCTGGGTCACATGGGAGGGA
TCTGATGTTGGGTCATGTGGTCCCACCAATGCCAAAGACTCTGAAATGTCTCTGTGAGGAGAACCCTGGAGATGGGCAGG
ATCATTTCTCATGCCTTCCCCCTTCCTCTTCACATGGGTGATTGTTGAATTTCAGGGAAAGGAAAAAGGAGACAAACAGAA
AAAACTTAGGCACAGACTCATGCCTGATTAAAGTGCTGGAACAAGCAACCATTGTGTGCCCAGCCCTAAGCAGAGCAGGG
ATATCTACCCTCCAAGGCTAAGGAAACTCCATGGAAGAGGGAAGGAGAGTGTGTGCAAGGGCCGGGTGATGGGAGTAACT
GCTGTGAAGCACTGACTTCTTGATATAACATGGCCATTACACTCATATTACACTCCGGTTATAAGGATAAGGCGTCCCTT
GTCACGTTTCTATAAATCTTTAACTCTCAGCAATTGCCAAGAGGAACAATGCTATGTTCTTCTGCAGTGATGTTTTCATG
TCCCCACAAAGAAATTCCACTCACACTCACATAAGCAGCCCTATTTAAACACGGTTCCTCAAACCCACAAAGCAAAGATT
TGAAAACAGGAGGGCTACTCACCAGGAAGAACTGGAGGTTTAGCAGGTATTGGAAAGGTAATGAGAGACAATGGGGGGGG
GGGCGGGGGACACAACCAAACTGTATCTTGTACATGTATGAAAGAATAATGAAGACATATAGAATTGATTGTTGTTACAG
TAGACACGAGAGTGCAGAGATGCCTTCAGGGAACCTTGACTGGAATTGGCACAGAGCAGGACACGCAGCCATGCTACATCT
GTGGTTCCTAAGACAATAGTGTCCATGGTTGGAGATTTAGTCTTTTATCGTGTGGACTCCTACCTTCCTGTTATACACAC
TTACAGATAGAGTCAGGCCTTCCCATTCCAGGCTTACTATTGATTTCCTCCATTTCCAATGCTGCATCCACAGTTGTTTT
GTAACAGAAGAAATAACCTATGTAAAAGTACAAAGCAAAGAAAATATGTCTATAGTTTACATTTTATTTTGGGTACTGTA
ATCTCCAAGGATAAACACATCCTTGCCATGGTGTCTCAGCGGTGAGCAGGGCTCCTGCTTTGCTAAAAGCTGACTTTCAT
CCTTTGCTCATTGTCAGGACCAAGAGGAGAAGATCATGGACCGTGGCCTTTGAACCTCTTCCCTAAGCCATGGGTGGAGA
CATGATCTGGGACTCTACCCTTTCTGCCCATGGATGCTTGGGCACTAAGAACAGCTTCTCAGGGACTGGGAGGGCTGGAG
AGATGGCTCAGAGGTTAGGAGCACTTACTGCTCTTCCAAAGGTTCTGAGTTCAACTCCCAGCAACTACATGGTGGTTCAC
AACCATCTGTAGTGGGATCTGACGCCCTCTTCTGGTGTGTTTGGAGACAGCAACAGTGTACTCATAAATAAAATAATTCT
TTAAAAAAAAAAAGAAAAGAACAGCTTCTCTCAAAGTCTGACTGTTGCACTGTGTCTGCAACCTGAAAGACAGACAGAA
AGCTGGCTGTCGGAGTGTTCCTCCTATTCACCGAGAAGAGTACCAAATATCTAAAAGGTACACAATCCATGTGGTTTAT
GCTTAATCCCTAACCTCTCTGGGAGTCCGGCAAGTTGGTGTATCTAAGTAGAACGCACTCCTGGGACAAGCCAACAGTAA
ACTCTGGCATCCTGTTTTGTTATCTATGTTTTGCCCAAGCGAACATTTCACACATTCATGACAATGCATTACCAGAGAAA
GACAGTCTATCCTGTGTGACTCTGTAAGAGAAGTCCCATGTGGGCTCATACCTCCTTGCCTCAGAAGCCCCCTCCCCAC
TCATTCCTCTGTATTAGTCAATACTACTGCAGGTGTCAACTGTGCTGAGACCTGTATGTGGGGCTATAAATCGCAAGATG
AAGCCCCCCAGCCTGGGAACCCCCTGGTGTCTCAGCACCTTCAGGAGCTTGGTCTTCTTCCAGAACATTCCATCTCTTGC
TGTTCACCTCCTTGGCCTTATTTCATGCCACAGGACTTGGCAGCACGCCACACAGGGACTCCTCCTGTGGGAGAAATCAC
AGGGCCATCAGTTCAATTTGATGGAATTTGATACCAGGTTCTCTGTGTACAAGGCCTTGCTAGATTAATTGGTCCCTTTC

```
AATAAGGCCATACTGAACATTTGTCAGTTTGTAATGAGGGGCTTGCCTGGCCTTATTTGTTTACAGATTTCGTTTTCAGA
TTATAAATGATATTTCTATTGTTCTGTGTGACTACATCATTGAGTGGTTCAGAAAGAGTTCAGTAGCATTGAAGTAAGCA
AGAGAAGTGGTTATCCTAATCAGATTTTCCTATTCATTCCTTCCTTATTTAAATGTGTATATATGTTTGTTTATGGGTGT
ACACCAGCCACGGTTCACATGTGGAGGTCAGAAGACAACTTGGAGGAGCCACTTCCATCCTCCCAACAAGTGTGATCCAG
AGACTGAACTCACAGCATCGGGCTTCGTGGCAGGTGTCTACACCTGCTCAGCCTTTTGAGTACCCACCTGATTAAGTCTC
TGTACAAATCTCCTTATTCTGATACTTTAGTAATCAGTTCAGAATATATTGAGCAACTTCTGGATTCACTAATTTCTTT
TCTCTTCTCTTCTCTTCTCTTCTCTTCTCTTCTCTTCTCTTCTTTTCTTTTCTTTTCAGATTTCTTTATTTTATG
TATGTGAGGACACTGTTGCTGTCTCCAGAAACGCCAGAAGAGGGCACCAGATGGTTGTGAGCTACCATGTGGTTGCTAGG
GATTGAACTCAGGACCTCTGGAGTGCAGTTGATGCTCTTAACCTCTGAGCCATCTCTCCAGCCCTGGATTCACTAATTTC
ATAAACAAAAACAATAAAAATTCTAACAAACAATTTAAATTTCAGCTAATCTACAAATAGTAAAAAGAGATTCGCCAATT
ATATGTCAAAGCTCCCCTTTCCTTCATTCCCACCTCCATGTAGTTGTCATAGCCGTCCTTCATAGCCGTCGGTCACAAGC
CAACTAGGAAATGAAAGTTCAGGGAGGCATTTTAACACAGATCTACCTGAGTGCGAACGGTTTCTGCTCCGTGCTACTTG
ACTTTCAGGTGTTGCTGTGCTTATCAATAGGATAGATAGCATTCTTGTATAAGTGTATTTGTAAAGAAAGTATATTTGTG
ATTGAAATGACAGAACAGTAATCAATTATTGTGGAGCTCATGACTATGATGTCAATTCTGAAGGAATTTCAACACAGGAG
AAAGAAGTTGCCCTGGCGCAATGGCACGCTCTAAGGGGCACAGAGGAAGTTCCTGCTCAGGGCAGCTCGGCATGACCAGA
GGACTCAGCAAGCAGGGGTGAGGGGGAAGGGAAGATCAACTGTATGGTTTAGGCTCAACAGGTCAGATGAAGGTGGAAGG
AGATGTGGGTGAGAATGGAGGTCCCGCTGGGATAGCAGGCTGCTCAGGACAGCTCGGCATGCCCAGAGGACTCAGCAAGC
AGGGAAGATGGGTGGGGGGAGGGATGCTTACCTGTATGGTTCAGGCTTGGTATCTCAAGGATTCTTTTGATAGGAATCAA
GGATGCACAAATCAAGGCTCAGCTAGGCTCATGCTTTGATGGTGTTGCTAGCAATATCAGGGCTTTTTTCCCCTTGTGGT
TTTAATCTCATGGATGAATAATTCAGTAAGACTCTTGAAAGAAGCTCATGAACAATTTCATTCAAGTGTTGTAAGAGAAA
CAAGACAAGCTAGCTGGAAGCCTCGGGAGCTGCAAACAAGATTGAATGACAAAGATGGAAGAAAGCCATTCCCGTTGTAA
TGGACATCGCCGAGCAGCCATGTTTAGGAAAAATGTGCAAATGATCAAGATAGAACGTGAGAGGGTCCAGAGTGTACAGC
AATGCAGAAATGCAGTCATCATCTTTGGATGGCACTGGAAAAATAGAAGGAAGAAGAGGAAGAAGAGGAGGAGGTAGAAG
AAGACAAAGACCGATGACTACAAAGACAATGAAGGAGGAGGAGGAGAAGGAGGAGAAGGAGAAGGAGGACAGGGAGGAGG
AGAACAAGGAGGAGGAGGAGAGGAAGGAGGAGGAGGAGGGCAGAGAGGAGAAGGAGGAAGAGGAGAATCATCACTCTTTT
CTGAAATAGGACTGTGGTGGATTAAATGGAAATGGGCCCCATAGGTTCATAAAGAGTGGCATTATTAAGTGCTGTGGCTT
TGCTAGAGGAGATATGGACCTATTGAAGGACCTGTTTCACTGGGGGTGGGCATTTAGGCTTCAATGCTCAAGCCAGGCCC
AGTGTCTGTGATTATGGTTTCTCTTCACAGCAATAAGACTCTAAGATGGAAGTTGGTAGCGGGGCCAGGGGAACTGCCAT
GGCAGGCCAGACAATGCTTTCGTTTGGAGGAATATGGAACCCTTTGGGGCTTTGAACTAGAAGAGCAATTGGGCAATTCA
AGCAAGGTTTACTGGGTCATACTAGCTGGAGCATGGAACACAATGGTGCTAAGGGTTATTTAAACTGTGTGGACACAATT
CGGGAGGATTCAGAGGGGGATATTAGTATGGGGCCCAGAAACAATTCTTGTGATGTTTTGGCTAAGAACATGGCTGCTTT
CTGCCATTATGTAAAAAGCCAACCTGAGGCTAAATTGAAGAGTTATGCATTAAAAGCTTTGGCGGAGGACAGTGAGAAGC
TGCCTAGCATGGGCTGTGTCATATGGCTCTTGCTGGTCTGACTTACACAGTTCTATGATGAAAATGAACAAGCTGAGCTA
GGAAAAATACAAAATGTAGTTTGCGAAAGGGAGCACCAGGAAGTTCAATGGAGTTCAATCCTGTGTTCAAGGACATAAAA
AGATTAAAGAAATACTTGATGTTAGATGGAATACAGGGAATGGTTGATGTCAAGGTAAGATGCCGCCCTGCCAAGCTTCC
AACACATGGAAAGGTATTTAAGCAAAGCGTAGGGCCTGGCACATGCCTTAATACCTGAAGTGGGGAGGCAGAGACAAGCA
GATCTCAAAGTTCAAGACCAACCTGGTTTTCAGAGCAATTTCCAGGACATCCAAGCTTAGGCAGTGAAGAAACCATTGA
AACCAGAAAGCTGGTGAAAATGTATTTGAAAGAGAAGGTAATGTATTTTAATAAAGAGGCCATGTTCCAGCCTCAGCGAG
TAATGGAACTTGGCAGCTTCAGCTATGTGGTTATGAGTTCAGAGACAAGGATATAAGAAAGGAGTTGTAGAATCTCCCTC
CATGTCAAAAGAAAGTCCCTGAAGCCAGACTTACTGAATGGAGGCCTAAAGAAGCCATTGCATGAATCTGTGAAGATGAA
GCCTGGATCGCCCTAGAGACCCTGAGATGCTGAAGATGCCCAAGGCTCTTTGGCATCCCTGCTGAAGAAGGCTGCTAACA
CTGGGTTGGATCAGCCCTAGAAAAGGAAGTGTGATACAGTCAGCAAGGCTGAAAGGGAGCTGGAGATCTGAAGAGTGTTTT
GACATGAAGATGCAGAGTTCGGGAGTTTGTCCATCTGGCTGTCAGTCTTGTTTTGGTCCAGCATTTTCTCACTATGCTCTA
TGTATACCCTTTGGGAATGGTAATGTATATCCTGCGCCATTAATTGTTGGAAGTATGTGACCTGCTTCTTCGTTTTGACT
TTACAGGGGACTACAGTTAAGAGACTGGGTGAGTCTCAGAAGAGACTTTGAACTTTAGGCTTTAAAACAGTGCTGGTGC
TGTGATAGACTACAGGAACTTTCAAAGGAAGACTCAATGGATTTTGCATTGTGATATGACTGCAAGACTATGGGCAGAGT
GGGGTGGAATGTAATTGTTTGAGTGAAAATGACCCTGATAGCTTCACAGGGAGTGGCACTATTAGGAGGTGTGGCTTTGT
TGGAATAGGTGTGGCTTTTTTGGAGAAAGTGTGCCACCGAGGATGAACCTTGAGTTCCAAATGCGCAGGTCAGGTCCAAT
GTCCCTTTCTCTTCCTACTGACCATCAATTCAGATGTAAAACTCTTAGCTATCTCCAATACTATGTCTGCCTGCATTT
CATGCTTCCCAACATGATAATGAACTGTAAGCTGACACCAATTAAATGTTTTTCTTCAGAAGACCTGCTGAGTTCCTGAC
TGCTTTCTCGGAGGCCATGGAAGCACCAGGGTCCAGAGGTAACACCCTATCCCCTGTCCACAACCTCCCTTAAAAGTGGA
CCCTATATCCCCAACTCTCTATATTCTTGCCCACCACTCTAGCAGCTTTCCCAGAACCCATGTGGGCATTAGGGACCCCA
GGTTAGACATGGTCCTCTGCACACTAACTCCCTTAAAACATACCCAACAGCCTTAAACTATACCCCATTTCCTGGGCACC
AGATTAGACATCATATTAGACATCACAACTTTGGTAGATCTCTGCTTTCTCAGAGGCCATGCTGGTTCTAGGAACCTCAG
AGGCAATATTGGTTCCCAAAATCCCAGAGGTCACCCAGGTCACAAAAACCCCAGTAGAGATGCCCTACTGGACCTGAACT
CTGGCTGGTCACCAGAACTGCAGGCAACCTGGGCCCAGAAGACCAGAGAAGAAACAGAATCAAGGAAATATCAGCAAAAAA
CACTCAGCGATCTAACAAAGATGAACTCAGAAATCAGCACCTAAACCCATAATAACCCTTAACCCGGATAACTAGGAACC
AGGACAAAATCAACAGCAGCCAGGGAAATATAGCACAATATTGCCCAAGGCCTGAACAATCAAATGCCTTAAAGAAATAC
TGGAAAATACAGACAAACAGGTAAAGAAAATCAATATAAACTGTTCAAAACCTGAATATGGACATAGAAGCAATAAAGAA
AACAGAAAGTAAGGGAAAACAAAAGATAGAAAATC
```

MOUSE mRNA SEQUENCE : mR5-002.1 (Seq ID No: 32)
GAGCTGACTGGGAGATGGTGGGGAGCGAGCTACTCTGGCAGAACTAAATGTCATGGGGCACAGATAAATAGGAAGAGCCT
GCAACTCACTGGCACTTGGAGGCTCCCGAAGGAGGCTGCCTGTCGCCCAAGCACAGAGCCACAAAGGATGCAGTCTAGGA
GGGAAGAATCACAAGCCCTGTAAGATGAGTGGAGCCAAACCCCCAGCCAAGCACCAATACAGAACCCCGGGACAATGAGG
ACACCCCCTGCCCATAGCTTCCAGTGCAGCCACCAAAAGTGCCAAAATGGACCAGCAAATGGCACTCACATGGGGGCTG
TGCTACATGGCACTGGTGGCTCTCTGTTGGGGACACGGGGTGACAGAGGCAGAAGAAACGGTCCCTCTGAAGACTCTGCA
GTGCTACAATGACTACACCAACCACATCATCTGCAGCTGGGCGGACACAGAGGATGCCCAGGGGCTAATCAACATGACCC
TCTATCACCAGCTAGAGAAAAAACAGCCAGTGTCCTGTGAGCTCAGTGAGGAACTCATGTGGTCAGAGTGCCCGTCATCC
CACCGCTGTGTGCCCAGAAGATGTGTCATCCCCTATACACGATTTTCCATCACAAACGAAGACTACTACTCCTTCCGGCC
AGATAGTGATCTGGGCATCCAGCTCATGGTGCCACTTGCCCAGAATGTGCAGCCACCACTTCCCAAGAACGTCAGCATCA
GCTCCTCTGAGGATCGTTTCCTGCTGGAGTGGAGTGTGTCCCTTGGGGATGCCCAGGTCTCCTGGCTTTCATCAAAGGAC
ATAGAGTTTGAGGTGGCTTATAAGCGGCTTCAGGACTCCTGGGAGGATGCCTACAGTCTCCACACTAGCAAATTTCAGGT
GAATTTCGAGCCAAAGCTATTCCTACCCAACAGCATCTATGCTGCCCGTGTGCGCACTCGGCTGTCCCCGGGGTTCAAGCT
TGTCTGGGAGACCCAGCAGATGGAGCCCAGAGGTTCACTGGGACTCCCAGCCAGGGGACAAGGCCCAGCCACAGAACCTT
CAATGCTTCTTTGATGGGATCCAGTCCCTCCACTGCTCCTGGGAGGTGTGGACCCAGACGACTGGCTCTGTTTCCTTTGG
GCTCTTCTATCGCCCCAGCCCTGTAGCTCCGGAGGAGAAATGCTCTCCGGTGGTGAAGGAGCCGCCGGGGGGCCAGTGTCT
ACACCCGCTACCATTGCAGTCTACCTGTGCCTGAGCCCAGTGCACACAGCCAGTACACAGTCTCTGTTAAGCACCTGGAA
CAAGGGAAGTTCATCATGAGCTATAACCACATCCAGATGGAGCCTCCAACCCTCAACCTGACCAAGAACAGAGACAGCTA
CAGCCTGCATTGGGAAACTCAGAAGATGGCTTACTCATTCATTGAGCACACATTCCAGGTCCAGTACAAGAAGAAATCGG
ACAGCTGGGAGGACAGCAAGACAGAGAACCTAGATCGAGCCCATAGCATGGACCTCTCCCAGCTGGAGCCAGACACCTCA
TACTGCGCCAGGGTGAGGGTCAAGCCCATCTCTAACTACGATGGGATCTGGAGCAAGTGGAGCGAAGAGTACACTTGGAA
GACTGACTGGGTGATGCCCACGCTGTGGATAGTCCTCATCCTGGTCTTTCTCATCCTCACCTTGCTCCTGATCCTTCGCT
TTGGCTGTGTGTCTCTGTATACAGGACGTACAGGAAGTGGAAGGAAAAGATCCCCAACCCCAGCAAGAGCCTCCTGTTCCAG
GATGGAGGTAAAGGTCTCTGGCCTCCTGGCAGCATGGCAGCCTTCGCCACTAAGAACCCCGCTCTCCAGGGGCCACAGAG
CAGGCTTCTTGCTGAGCAACAGGGGGAGTCATATGCACATTTGGAAGACAACAACGTGTCACCTCTCACTATAGAGGACC
CTAATATAATTCGAGTTCCACCATCCGGGCCTGATACAACCCCAGCTGCCTCATCCGAATCCACAGAGCAACTTCCCAAT
GTTCAAGTAGAGGGACCAACTCCTAACAGACCTAGGAAGCAATTACCCAGCTTTGACTTCAATGGGCCCTACCTGGGGCC
TCCCCAATCCCACTCTCTGCCTGATCTCCCAGACCAGCTGGGTTCCCCCCAGGTGGGTGGGAGCCTGAAGCCAGCACTGC
CAGGCTCCTTGGAGTACATGTGTCTGCCCCCTGGAGGTCAAGCGCAACTGGTTCCATTGTCCCAGGTGATGGGGCAGGGC
CAGGCTATGGATGTGCAGTGTGGGTCCAGCCTGGAGACCTCAGGGAGCCCTTCTGTGGAGCAAAGGAGAACCCTCCAGT
TGAGCTGAGCATGGAGGAACAGGAGGCACGGGACAACCCAGTGACTCTGCCCATAAGCTCTGGGGGCCCTGAGGGCAGTA
TGATGGCCTCTGATTATGTCACTCCTGGAGATCCGGTGCTCACTCTGCCCACAGGGCCCCTGTCTACCTCTCTGGGCCCC
TCTCTAGGGTTGCCCTCAGCCCAAAGCCCCCGTCTCTGTCTTAAGCTGCCCAGGGTCCCCTCTGGAAGCCCAGCTCTAGG
GCCACCAGGGTTTGAGGACTATGTGGAGCTGCCTCCAAGTGTGAGCCAGGCTGCCAAGTCCCCTCCAGGCCATCCTGCTC
CTCCTGTGGCAAGCAGCCCCACAGTGATCCCAGGAGAGCCCAGGGAGGAAGTGGGCCCAGCATCCCCACATCCCGAAGGC
CTCCTTGTTCTTCAGCAGGTTGGGGACTACTGCTTCCTCCCTGGCCTGGGACCTGGCTCCCTCTCACCCACACAGTAAGCC
ACCCTCTCCAAGTCTGTGTTCTGAGACTGAGGACCTAGTCCAGGACTTGTCTGTCAAAAAGTTTCCCTATCAGCCCATGC
CCCAGGCGCCAGCCATTCAGTTTTTCAAGTCCCTAAAGCATCAGGACTACCTGTCCCTGCCCCCTTGGGACAATAGCCAG
TCTGGGAAGGTGTGCTGAGTCTGTCTCCTCCCAATCTCACCAGCAGCCTGGCACCGCAGCCTGTGGTCCTCAGCCTGAGC
ATCACCACAGAAGCCTCTCTGAGTTCACACTCCTCCTTGCTCCCAGCCCTGACATGGCAATACCCCACCTGT

MOUSE PROTEIN SEQUENCE : mP5-002.1 (Seq ID No: 33)
VEPNPQPSTNTEPRDNEDTPLPIASSAATKSAKMDQQMALTWGLCYMALVALCWGHGVTEAEEETVPLKTLQCYNDYTNHI
ICSWADTEDAQGLINMTLYHQLEKKQPVSCELSEELMWSECPSSHRCVPRRCVIPYTRFSITNEDYYSFRPDSDLGIQLM
VPLAQNVQPPLPKNVSISSSEDRFLLEWSVSLGDAQVSWLSSKDIEFEVAYKRLQDSWEDAYSLHTSKFQVNFEPKLFLP
NSIYAARVRTRLSPGSSLSGRPSRWSPEVHWDSQPGDKAQPQNLQCFFDGIQSLHCSWEVWTQTTGSVSFGLFYRPSPVA
PEEKCSPVVKEPPGASVYTRYHCSLPVPEPSAHSQYTVSVKHLEQGKFIMSYNHIQMEPPTLNLTKNRDSYSLHWETQKM
AYSFIEHTFQVQYKKKSDSWEDSKTENLDRAHSMDLSQLEPDTSYCARVRVKPISNYDGIWSKWSEEYTWKTDWVMPTLW
IVLILVFLILTLLLILRFGCVSVYRTYRKWKEKIPNPSKSLLFQDGGKGLWPPGSMAAFATKNPALQGPQSRLLAEQQGE
SYAHLEDNNVSPLTIEDPNIIRVPPSGPDTTPAASSESTEQLPNVQVEGPTPNRPRKQLPSFDFNGPYLGPPQSHSLPDL
PDQLGSPQVGGSLKPALPGSLEYMCLPPGGQAQLVPLSQVMGQGQAMDVQCGSSLETSGSPSVEPKENPPVELSMEEQEA
RDNPVTLPISSGGPEGSMMASDYVTPGDPVLTLPTGPLSTSLGPSLGLPSAQSPRLCLKLPRVPSGSPALGPPGFEDYVE
LPPSVSQAAKSPPGHPAPPVASSPTVIPGEPREEVGPASPHPEGLLVLQQVGDYCFLPGLGPGSLSPHSKPPSPSLCSET
EDLVQDLSVKKFPYQPMPQAPAIQFFKSLKHQDYLSLPPWDNSQSGKVC*

MOUSE PANTHER CLASSIFICATIONS
        FAMILY (SUBFAMILY)
                CYTOKINE RECEPTOR(Unassigned)
        BIOLOGICAL PROCESS
                Biological process unclassified(2.99.00.00.00)
        MOLECULAR FUNCTIONS

Molecular function unclassified(1.97.00.00.00)

```
MOUSE GENE ONTOLOGY
        BIOLOGICAL PROCESS
                humoral defense mechanism > antimicrobial response
                cell communication > signal transduction
                signal   transduction  >  cell  surface  receptor  linked  signal
transduction
        MOLECULAR FUNCTION
                molecular_function unknown > lymphocyte antigen
        CELL COMPONENT
                cell > membrane fraction
                plasma membrane > integral plasma membrane protein
                integral plasma membrane protein > interleukin-13 receptor
                cell > plasma membrane
MOUSE PROTEIN DOMAINS (INTERPRO SIGNATURES)
                IPR001777 (FN3)
                IPR001777 (fn3)
                IPR000282 (CR2A 2)
                IPR002996 (CR1A 2)
                IPR000694 (PRO RICH)
                IPR003531 (HEMATOPO REC S F1)
```

HUMAN GENOMIC SEQUENCE : hD5-002 (Seq ID No: 34)
```
TTTTTATCCCCTGCTGCTCTATGATGACTTTTAAAAGGAAAGGGTCAGTCAAGTGAATTGGCTGAGGTGTTCCTAAAGCT
CCTTCACATTTTGGGTCTGGTGTCCGGAGTGATTTTGACTCAGAGTCTCGGGTTCTTCGTTTCTCCACCAGACAGCATCC
TCTGTGCTCCTGAGGGCATCCAGGCCCGCAGCCTCCCAGTCCCACAGAGGACCTCAGCAAAGACAGGCAGAGTGGGGACA
CCTGGGAGAAAAGAGAGCCCGGGACGTCACTTTAATTCCATCCTCTCCAGCCCTGGAGAAGCCCCTGCAGGGTTTTTCCA
TGCCAGGCAGGGACCTCACACTGACCGGAGTGCCTGAGAGAACCACACATGCCTTCCTCTACCCTGGGCTTTCTCTGCTA
GGGTGAGGCCCTGCCAGCTCTTCCTGGCCAACCCTGCCCTTCCTGGTAGGAGGAAAGCAAAGAACAACAATCAGGCCAGA
ATTCCAGTGGCTTCGAGGCAGGTTCCTGCCCCTCCATTTCCTCCATTCCCCTCATTCTTGCCCTCCATTCCCCTCATTCC
TGCCTGCTGCCCTTTCTCCTGGGAGGCAGCCCCATGTAGGAGAAATATGACTGCGTTTTGTTGTGTCTTGGCACTGTGAT
TCATCTTGTCTTTATTCAAACTTTTCATATTTGGTTCACCGTGAATAGTTTCTGCATCAGTTGGGATTTTTTTAAACCAT
ATTGCATTAAAATATTACTTACAACAATTACTGAATTTTTAAATGCCTGTTTAAATTTTGAGCACGAGAGACTGCCTCAC
TCACCTGACCTTAATTCTGCTGGCCCTGCTGTGCTCATGGAGCCACTCATTCATTCATTCATTCATTCATTCATTCATTC
ATTCATCTATTCATTTGCACACAGTCAAAGGTGCAATTTCTCCTCTGTACCAGGCCATGTGCTAGGTATTTGGGAGGGAA
GTGCAAGCCGGACACAAGCCAGCACTGAAGATAGTGTCAGAAGAAAGACAGCCATGAACACACATGACCATATGACACAT
GGTGGGGTGGGGCGGCAGGGCAAAGAGGACATCGGGGAAATCAACATGCCACCAGCTACCACTTGTAGGGGGTTCTCCTG
TGGCCAGCTCTGAGCTAAGGTCTTCCTGCACAGAATCTCACTGGATTCCCTCAACAGTCCTTCCAGGTTTGTAGATGCTT
CTGCAAAAGTAACAGACAGATGGATGTCCCGAGGCTTGGGGAAGTAACACGCAGATGATTTGCACAAGGACACGCTTTAA
GAAGCAGTGGACTTGGAAGTCCAACACAGATCTGTCTGGCTCTGGGCTCAAACCAGCTATTGTTATTGCAGCCACTTCCA
CCTGCATCCCCATCAAGCAGTCAGGGAAGGCTGCATGGAGGAGGCCCTGCGTCAGCTGAGGCTTGAAGGAGGCCTAGGAG
TTTATAGGAAGGTAAATTGGAGTAGGAGGCTTTCTGGAAGTAAGAAAAGTCACGTGCACTATTAGCAAAGTAGGCCTGGG
CCGGCACTGCTTCCTCTTTCTGCTTCTCTGTTTCCTGATGACATCAACATAAATAGGAAACGTGGGAGGCTCACTCTGCC
TAGAGGCTCCAGAAGAAGACTGGTCTCTCCCACCACACAGAGGTATGGCCCGGCGTCCAGGAGCAGGGCTGAGGGGTGGG
GTCAGAGCAGGGGGGAATGTGGGGAGTCCAGGATGAAAAAGGACAGGGGGAGCCTGGGGGGCGTCCTGTGGGTAGCTGTG
CCCTGGGGACTTGGAGGAGTTAGGGCTTGGCCTCTTAGGATGACCTGAGCCCAGACTCCTAGGGCGGCCAGTGGATGGGG
AGGCAGCCAGGGGTGCCAAGCTGCTTGGGTGTTGTAGGAGAGCTCTGGGGAGGACTCAGCCTCGCTAGCGGGATCTGGTT
GGTCCTGGTGGGAAGGACGCCTAGTGGGGTGAAGAGAGCTCAGGCCTGGGAGTCAGGCACCTATAGTCTGTCTTCGCTTT
GTCACTAAGATGCTGGTGGTAGTGAGAGCACCCCCTGTCCACCCTGGGCTTCTGTCTCCTCATCTGTGGAATGGGGAGCA
TGGATAGTCCCTGACATCTTGGTTTAGAAAGAGAGAGAACGCATGCTCGTGTGTTTGCCCGTGTGTGCATTTGTGTGTGT
GCCTGTGTGTGTATGTGCATGTGCGGATGTCTGGGGATGCAGGAGTGCAGAGAGACTAAGAGGCAGAGGGTGAAACCTGG
AAGCTTGCTGCGCTGAGGCCCAGAGCTGGGTGCGGTGTGGCTTGAGAAGCTCACTTTGAACTTGAGAAAGAGCCCAGCAC
TTTGCCCAGAGACATACCAGGCACTATCCAGCAATCTCTCTCTCTCTCTCTCTCTCAGTCTCTCTCTCTGTCTGTC
TGTCTTTTTAGTTATGACACTCTGCCCTGCTGATTTGGGTTGCTGATTTGGGTTGGGGCCCAGGTGTGTGTGTTCTGTAC
CCAAGTATGCTGGCTGGGTGGGTGGGTATCTGGGGTGCCTGCTTCAGGGTGTGTGCAGGTGTGTGTACTGGCCCTGAGAA
TGAAAGGGCAGGTGTTTCACTGGGACCTTGTCAGACAGGTGCTAGCGACAAGCTGCATATGTGCAATTTTTAGTCTTCAA
AAACCCTGCTGTTATATTTTTGTACAGCCCAATTCTAATTTTTTCTATCATCTTTCCTGCTTTGTAATCATTTTATAAT
GAGTAAACATTTCCTATTAGCTTTTGGTCACTCTTTTTTTCATCCAGCAGTTTTATTTATATTGTTCCAGTATACAGATA
TACCAACACCACAGGATAATTCATGAGTTGTATAAGGAATGTATTTAATGCTAAACAAGCAACTACACAGAAACCTACCT
TCATGAAAATTAGAATAATCTAGAATTATGTAGCACAAGAGACAAAGTCCCCAGCAATTTCTTTTCCCTAGTTCCATAGC
```

```
TTATTCTTTTCACCACATGTATTCTTCCAGAACATTTTCTAAGCATGATACAAGCATATAGTTTTTCTTTTCCTGTTTGT
TTTTTGTTTTGTTTTGTTTTGTTTTTGTGACAGAGTTTTACTCTTGTCGCCCAGGCTGGAATGCAGTGGTGCAATCTTGG
CTCACTGCAACCTCTGCCTCCCGGGTTCAGGTGATTCTCCTGCCTCAGCCTCCTGAGTAGCCGGGATTACAGGCATGAGC
CACCACACGTGGCTAATTTTTGTATTTTTAGTAGAGACAGGGTTTCACCATATTGGCCAGGCTGGTCTCGAACTCCTGAC
CTCAGATGATCCGCCTGTCTCAGCCTCCCAAAATGCTGGGATTACAGGCTTGAGCCACTGTGCCAGGATGGTAGTTTTTC
TTTTTACAGAAATGGGATTATTTTATTTATATATATATATATATATTCCTAATTTTTTCACTTAATATTGTATTTTGGAA
TATTTCCAAATTTCTACAATAATACATACAGATTTAACTCGTTAAGTAACCCTAAGAAATATGTGGCATTTATTAGGATG
GGTACACCACAATTCATCTAATCTCACGTTGATCGACACCTAGATTGTTTCTAATTTTTCCACATCTGTGAAAGTGTTTC
TGTAGCCTAAATTACTAAATGCAGAATTGCTGTGCAGAGGTTGTGCGTTAGAAATTATAATAGGTATTGTCAAATCAGCC
TCAAAGTAGCTGAGCTAAGCTGACGTCCCCAAGGTAGTGCATAAAGGGTCCAATTTACCTTTAGAGAAATTATCAATCTT
CTAATTTTGTCCAATATTATGTATGGAAATTCTCAATGTCGCGTGATTTTTTTCTTTCCCTGATTATGGGTGAAGTTGTA
CATTTCTATATGTGGATTGGCCTTTTGGATTTCATTTATGATGAATGTGGAGAAATTTTTGTTCATCTCCTGTTGAATT
CTCTTTTACATCTTAACTTTTGGAGTGACTCATTTATTACGTGTATTAATAATCCATTTATATATGTTGAAAATATATTC
TCCCAGGTTATGACTTATCTTTTTCTTTACCATGTTTAAGATGTCTTATGTCATATAAAAATGTTTTCATTGTTAAGTCA
AACCTGTCATTACTTTTTACTTATGGTTTCTGATTTTGGTGTCTTGTAAACCTCTAAGATCACAGGTGTCTTGTAAACC
TCTTTTTACTTTATGGTTTCTGGTTTGGTGTCTTGAAACCTCTAAGATTACAGAAAGAGTTTTAATAATTTTACTATTTT
GGTTTTGACTTTGAGATTTTTAGCCCACCTAGAATTTAGAGAAGTGAGTAATGTGTAAAGTAACTTTTAAAAATAATTAC
CATCTAATTGTCTTAACATTGCTAATTATGAGTGTGTGATTTCTCCTCTCTTCAATGATTTGAAGTGCCCCTATTACATC
CTAGGCTTCTATACATACTTCGTTATTTTCTTTATTGTTTTCTCTTCCATTTGGCTGTCACTACATTCCTTATTATTTTT
CTAGCGAATGGAGCAAGTCTCTCATCTCTGGTTTGTTATAATTTAGAATCAGTTTGCCAAGGCCAGGCACAGTGGCTCAT
GCCTATAATCCCAGCACTTTGGGAGGCTGAGGCAGGCAGATCCCTTGAAGTTGGACGTTCAAGACCAGCCTGGCCAACAC
GGTGAAACCCCATGTCTACTAAAAATACGAAAAATTAGCCGGGTTTGGTGGTGTGCACCTGTAATCCCAGCTACTTGGGA
TGCTGAAGTGGGAGAACTGCTTGAACCCAGGAGGCAAAGTTTGCAGTGAGCCGAGATCACGCCACTGCACTCCAGCCTGG
GCAACAGAGTGAGACTCCAACTCAAAAATAAAAATAAAAATCAGTTTGCCAAGCCCATTAAATTCCTTTTTGGAATTTTC
ATGTTGATTGCATTGAATTTAGAGATGATCTGAGGGACATTGACCACTGGAAGTAAAGACAATAGTGAGAGCCTCCTGTG
TGAGGCTTTGACAGGAGGACACTAACGTGCCCCCTTGACTCTGTCATCGCCTTTGGTTTCTGGCAGACTTTCACTGATGC
CAACGTTTCCGCCACTGCTTCATTTGCCAGGGTTTGTTTGTTCATTTAACCAGAAATGTCTTGTTTATTATTAAATATTT
AGGAGTGTCTGTCAAGATCAGCTTATCCTTTTTTCCCTTTTAATCCGTTCATCTGTTTGTTTGAATCATAAATAGGTTTC
TTAAGGGTTCTCCTTCCATGTATGGAGTACCCTCTACTTGGCCATGATGTCTTCTTCTTTTAAAAGTCTTTGTCAGGTTG
ACCTATGAAATTGTTTTTGTCTGGAGTGAATTTGTATTTTGCTTTTCCCCCCTAGTTTTTATCCCTGCCATTCAATTTCT
TCATAAGCTTGGGAATGTTTATCCCTGCCATTCAATTTCTTCATGAGCTTGGGAATGTAATATGTGCATGTTTATTTTGA
GTAAGAAGGTGTTTGGTTTTTCTCTTCTCTTTTCTCTCTCTTCCTTTCTCTGTCCCTCCCATCCCTTCTCTGTCATCCTC
CTGTCCCCATGCTTACTCTTCATTCTTAGTGATTTTTAATCTTTATGTTTTTAGGGATTTAAAATGTATTGAACTTATAT
TTTCTGGGTCGGGCACAGTGGCTCACGCCTGTAATCCCAGCACTTTGGGAGGCCAAGGTGGGCAGATCACTTGAGGCCAG
GAGTTGAGACCAACCTGGCCAACATGGTGAAACCCAGCCTCTACTAAAGATACAAAAACTAGCCAGGCGTGGTGGTGCAT
GCCTGTAGTCCCAGCTACTTGGGAGGCTAAGTCACAAGAGTCGCTTGAACCCCGGAGGCAGAGATTGCAGTGAGCCGAGA
TCATGCCATTGCACTCCAGCCTGGGTGATGGGGTGAGATTCTGTCTCAAAAAAAAAAAAATAGTATTTTTTTTAAAGAAAC
CATATTTGCTGCCAGCATTTGGGTTTATTTAGCACATATATGATCTTCCAAAACAAGAGGAGATCTTTAGCAAGTTTTCA
GTTCTCTTCCATTCTCCACCCCAGCCTCACAGCAAACTCCCACATTAAAATCATCTGAGTTTTAGTTCAATATTCTTCTT
TAACTTATCTGTAAGTGTTACAGGTTTATTTACCTCAGTATTTCAGCTTTCTTGGATTTTTTTTTCCTGAAATATAACCT
AGAGTAATTTCTTTCAGAAAAGATCTGAAAAACTTTCTTTGTCCTTACATACTCAAAATTTTTTGTGTCCTTTAAATGTC
AAAGAAAACTTGGCATTTTGTGATTCTAGGTTTAAGCATAATTTTCCCTTGGTATTTTCAAGACATCTATTTAAAGTTCA
GAGTCTCTTATGTGAAATCTTTAGCCAATCTGACCCTTGTTGCATTCAAAGTATCCTATTTGTTTTCTTCTGGAAGCATT
TCTGGGATCATCCTCATATTCTTGCAAGACGAAAAGTTTACCAGTGAGAACTAGGCATGGCTTTATTAAAAGGAATCTTG
GGAGTTAACCCTGATCTCTGTCCCCTACTACAAAACCTCATCGTAGTAGCCTCCCCCCCTTGAATACAGATTCCTTACTGT
CTTTGAAAAATATTTTAAAAAGGAGTCTTGTTCTTTATTGAGTGGCCTCTTGTCTCCTTGAAAGCCCTGTGAAGCTTTCC
CCATTTGCTGTTGTATCTGTTTGGCTGCACATGGAAAATCCCTAAGCAACAGAGGCTTAACAACATAGAAGTTTCTTTTT
CCCAGCATATAAAAGATGCCCAGAGGTAGCAGTTTCAGGCTAGTATGGTTGCTCCAGAGTGAGAGTGGGAATCCAGCTTC
CTCTCTTTCTGTTGCATCATCTTCACATATGGCTTCCATCCTCAAGGTAACCTCATGGTCCAAAATAGCTGCTGAAGCTC
CAGCTATCACCTCCAAGTTGCAGGCTGGAAGAAGGAAGAAAGACAGAGGGACAAAAATGAACCCCTCCCACCTAAGTCAA
CTGCCATTGAGCAGCCTTTTTTGGAAGACATACACAATGTTTCTATTCTCATCTCCTTGGATCACGTTTAGTCACTTGAC
TGCACCTAGTGGCAACGGAGTATAGGAAGGAAATAGACCTTTTTATTCTGTGTAGCAATAAACTCAGCTAAAACCTTGGC
TTCTGTTGCCAGGAGGAAAGGGACACGGTCTTCCATTGTCTTTCAGACTAGGTAGCTGACATTGTCTGCCATACCTGCTC
TTAATTCTCTCCACCCCCTGCTTCTGAATGCTGGAATTTTTGAATTTCTTTCCCAAACCTAAAGTTTTCTCTAAGACTTT
TCATCTCCTTGACTATTTGCTCTATTTTATATATCTATCTTTTTCATTTGTGGTATCCATTCTGTGGTTTGGCCCACTAT
TGATTGTTGTTGTTTTTTTGTTTTGTTTGTTTTTTTGAGACAGAGTCTCAGGCTGGAGTGCCATGGCAACGGTCTCAGCTC
ACTGCAACCTCCACCTCCCAGGTTCAAGCAATTCTCCTGCCTCAGCCTCCAGAATAGTTGGGATTACAGACGTGCGCCAC
CATGGCCAGCTATTTTTTTTTTTTTTGCGTTTTTAGTAAAGACGGGGTTCTACCACGTTGGCCAGGCTGGTCTCAAACT
CCTGACCTCAAGTGATCTGCCCACCTTGGCCTCCCAAAGTGCTGGGATTACAGGCATGAGCCACCGCGCCCAGGCTTTGA
TTGATTTTTTTTTAAATTATGGATCTCATATCTTCAAATTCCAAGAACTCTGTTCTTTGCATAGAAGTGTCTTCTTTTAA
ATGGCTATGATTCTCAAATCTCCCTGAGGATGCTGATTTAGATGTCTTAATGTACTCTTTTATTTTTGCTGTATCTGTAC
```

```
ATGTCTGTAGAAGTGTCTGCTGGGTTAGCATATGTCAGAATAGATGTGCGCTGTTTTGACATTTGGGAAACTGAATCACT
ATTGGCATAGAGGTCAGGGACAGAGTCTAGAGGCAGCCCACTGCGGGCTCGAATCTCAACCTGTTCCTTACCAACCGTGT
GACTGTCGACCAATTACATAACATCCCACCCCTGTTTTCTCATCTACAAAGTGGAGCTTCTAGTGGTAACCTATACATAG
ATGTATTCATATCCGTAAAACCTTTTGAATAATGCCTGGCTCATGGAAAACACTGCAATATTGTCAGCCACTATCATGAA
TTCTCTCTGTGTATGTGAGTACTGTTTGTGCTCTTTGATGATATTGATTTGTTTTAACCTTTTAAAGTAAAAGACACTAA
CCTGTCCTCCATAATTCTGGTTGGTATGGTCTATATGTTGAAGTGCACCCAAGTTTATGTTGCAACCTAAGCCCCAATGT
GAGAATATCTGGAGGTAGTGCCTGTGGCACCCTATAAAATACCCCAGCGAGCTCCCTTGCTTATTCCATGTGAGATTATA
GGGAGAAGACTGCAAGGAACAGGCCCTCACCAGATAGTGAATCTGCCCGCACCTTGATCTTGAACTTCCCAGCCTCCAGA
ACTATGAGAAATAAACTTTGTTACTAGTAAGCCACTCCATCTATGGTATGTTGTTACAGTAGCCTAACGGATGAGTACAT
TGGTGCAAAGATTTTCTGTTTGCACTCCGTCCTTTCCTTTTATGGTTCAGTCAGTTTGACAAATTACAGAAGATTTCACT
TTTATGTACTTCTGCCAGCCTTTGTCTCTGTGATGTCTCCTATGCTTTTAAGCTTAAAGAGTTCAAAGGCATTCACTCCT
CTGGTTTGCAGCTTTCATAGGATGTAACATTTTACATTTAAATTTATAATTAACTTGTTATTTGGGGAATGGTGTAAAAT
GGGGTTTTAGATAGGCATTTTTATGTTGTTAATCAATGATTTTAGCATTTTTTTAAATTTAATATTTCATCGCTTCACAT
TGATTCCTGATTCCTTCTCAGTCTGGGGTCAAGTAGAGTTTGTTTCCAGCCAGCATGTCCATTGATAGATTCTGTGTGTG
TGTATGTGTGTCTGTGCATGTATCTGTATGTGTGTGTGTATGTCTGTGTGTAGGTGGTATATGCGAGTGTGTATGTGTGT
GTGTGTTGTGCATGTATGTGTATGTGTGTCTGTGTATGTGTGCCTCTGTGTGTGAATTTCTGTGTATGTGTGAATGTGTG
TGCATGTTTGTGTGTATATGTGTATATATGTGTATACATGTATGTATCTGTGTTGTTCATGTGTGTATGTGTGTCTGT
GTCTCTGTGTGTGTGTTTGTATGTGTGTGTGCATGTTTGTGTGTGTATATGTGTGTATATTGTATGTATCTGTGTGTGTC
TGTGTCTGCCTCTGTGTGTGTGTCTGTGTGTGCATTTGTGTGTATACATGTGGGTATGTGTGTGCATGTTTTGTGTGTAC
GTGTATATGTATGTATCTGTGTGTGTCTGTGTGTTGTGCGTGTGTGTGCCTGCGTGTGTCCGTGTGCTTGTGTGTGTG
TGTGTGTTTGCATGGGCATCTTGAGTGAAGCTTCCAACAATCTAACAGAAGAAAAGGAGCCACACTTGTCTGTTCTGCTC
TCTTGGGTACTTCCCAGACCAGTGAAATGAAAGGGAGGAAACCCCCGGCCTCCGAGGAGAAAAGGGAACTGGCAAGCAGA
GGGTGGGGGGATGACGGTAAAAGGAGCAGGGGTGGGGAGAGCACAGGCCCTGTGGAAGTGAGGACATGTGTGTGTACATG
TGTTCATGTCCAGGGGATGACACTGTGGCATCCAACAGCCGTGGACCAGCAGCCCACGGGGGAGCTTAGTAGGAGTCAAAT
CCTAGGCCCCCGTCTCAGCTGCTCTGCTGTCTAGCGCATTGTGCAGTGGTAGGTGTCAGTGATCCAAGTGGGGACCCAGT
CCCTCAGGCCACACAGCGCATGTCCATTGCCTTCATGCCGCAGGAGACTGAGGGGTCATGCATGAGGGCCACTTCTCTGG
GTTGTCCCCACAACACGGGCGGTGTCCCTGGGACACGTGGAAGGGGAGGGGCAGCTCACTGCTGACATCTCCTTCTGCAG
GCCTGGAGGAGGCAGAGGCCAGGAGGGAGAGGTCCCAAGAGCCTGTGAAATGGGTCTGGCCTGGCTCCCAGCTGGGCAGG
AACACAGGACTTCAGGACACTAAGGACCCTGTCATGCCCATGGCCAGCACCCACCAGTGCTGGTGCCTGCCTGTCCAGAG
CTGACCAGGGAGATGGTGCTGGCCCAGGGGCTGCTCTCCATGGCCCTGCTGGCCCTGTGCTGGGAGCGCAGCCTGGCAGG
GGCAGAAGGTGAGTCCCGTGGCTCCCACCCACTTCCCTGTCCCTGTCCTCACTGCTGCACCCTGGGGGAGGGCCGCAGCG
TATCCTCAGGATCCTGCCCGCCAGCCCTCCTCCTGCTCCCCTCCCTCTGTCTCTCCCCCTGGCCTTCCCTGGGCCTCCCC
CGCTTCCCTCCTCCTGCACATTCCTGCTCATCCTGTCTTGGAAAGTCCAGCTGAGCGTGTCTGGCTTCCTTGCCCACATT
TCTCAGGGCGGCACTCCCGGCCCCTAGGCTCCAGGATGGCTGCTCTGGCCGTTTCCCTGCCCCTCCTTCCCAGCAGACA
CTCTCTGTGCCTCAGTGGTTCCCACCTCCGGGACTTTGCTCCTGCAGGGCCTTGGCTGGGGTTCTCTCCCTGCTTCAGCC
GCTAGCACCCTCCTTGTGCCTGAAGCCCGCACTGGATGCTCCTGGGCTCTTGAGGTGAAATGGCCCCTCCCAAGGGCTC
CCAGAGACCTGGCTTCTGTGATAATGCTGGGACCACAGTCCCCTTAACAAATACCAGGCTCCTGAGGACGGGGACTAGAG
AGGAGGTGGGAGGTTGCAGGGTAGAACTCTGCCACGCTGCATCCCAGGGCTGAGTGTGTGCCCCCTCCCAGGCTGCACAG
TCGGTCCAGGGGCCAGGCCTGTGCTTGATGCATGTCCCTGTCCTGGGGTGGGGGGAGGGGACCGTGCCCAGGAACAGCAC
ACTGCGGAGGCCCAGAAACCATGCTGAGAACCAACAGAATGTCTTGCTTCTGCCAGGAGGAGGGGTCCGCAACCAGGAG
CCCACCCCGGCAGACATGAACACATGTACATGTGCCTGGCCACCTGGTGCCTCTGCAGGGACCTGGGAGACCCCTCCCCA
GAGCGGGACATCCCAAAGCAGCTGGGGGTGATGGTGACAAGGGTCCCTGCAGGAAAGAGAGGTGACCCCTTCTACCCCT
CTTGTCAGAAACCATCCCGCTGCAGACCCTGCGCTGCTACAACGACTACACCAGCCACATCACCTGCAGGTGGGCAGACA
CCCAGGATGCCCAGCGGCTCGTCAACGTGACCCTCATTCGCCGGGTGAATGAGTGAGTGATGCTGGGGGCAGGGGCCACG
GGCAGGGGCTACGACGTCCCCTGTGCCTGGCATGGGGCGACAGTGTAGAGAGGGACCTGTCAGGTCAGCCTCGGGGTGGG
AGTGGACAGAGGACAGAGGAGGAGGGAGGCCCTGCAAGAGCTCCTGCTCCGCCAGGCACCTGCGAGGCCGGGTGCTGCCG
TCTGACCTGGGGTTTCTGCGGAGTGCCTCCTACACTGATGTTGGTGACAATGGTGGTGGTGGCAATGGTGAAAATCTTCT
CTTGTTTCATCACAATTTAACACAATTGCCCCACATGACCTATCTTAGTTCCTCCTCACTGTGAGGTGGGCAGGGCCAGG
CCACGAAGCCCAGCTGTGCCACTGCCTGCCTGTCCAGAGTAGACCAAGGAGAGATGGCGCTAGCCTGGGAGTTCACAGGA
TGGGAAAACTGAGGCCCTGGAGGTGAGGTGCCAGCCCTGGCCACGGGGTAGACAGTGGCAGAGCAAGCTGTGTGGACGTG
TCTGGGAGGGGGCTCCGCGCTCTCCCAGGCCCCCCCTCCGTATGTGGGCTGCCACCTCTCCCACCCAAGCTCTCCTGGGC
ACGTGCCACAAGGGAAGGGGACCAGAATCGCTCTGGGGTGTTGGCCAGGGTCTAAATCAGCCATAAATTAAGTAATTAAA
TAGCTGGAACCAGAGAGGCAGATGACAACCCATTGGAGGTCACCCCAGCGTTGGGGGCGGGGGCCGGCTCTGCCACCCCC
ACCCCTCCACCGCCTTCAGTTCTCCAGGGCGTCTCTGTCGGACCTTCTCCATTCTCCTCAAAGACCATCCTGCCCCTCCC
TGTGACCTGCTTCCTGCTGCTCTGACACAACAGGGGACCAGGGAGAAGCTCCCCACCCACCCCACACTCACACTCCCCAC
CCCCTTTGCCCACCGCCGGGGCCTGGGCCTGGACCAGGGCTCCTGTCTGAGGCCTGAGACACGATGCTGTCTGGTCCTGT
TGCTCAGGAACATCACGGCCCCCAAGTCCCCACTCTGCCCTGTGCCACCCACCATGCCCTCTCCCAGGATCTTTCCCAAT
TGCCTTGCAAACCTGCCCCAACCCCCTCTCAGATGCCCACAGGAGTGCAGCGCCTGGGCATCTCCTGACCTGGCAACCCT
GCGGCCCCTCTTTCTCTGCTTCCTTTGACAGCAGCATTCCCTAAAGAGTTGTCCACATTCACCACGTCCAGTGTCTTTCC
AGCCATTTCCTCCTGGGCCCACTCCCCTAGGATGCTGCCCCCACCAGGCCACCCAAACTGCTCTGGTCCAAGTCAGCAAA
TGTCCCAGGGAGCAGGATCTCATGGTTGGGCCTCCGCCTTCTTCCTACTTGACCAGGGGCAGCCCCTGACAAGGAGGACC
```

```
ACCGCGGCCTCCCTGCAGTTTCTCTTGCACCTGACTCTGCCCACAGCTCACCCAGTGCCCCCGGGGCCCTGCCCATCCTC
CTCTTTCTCCTTGTCTGCTGCCTCCTGATGGCAAGACACCCAGGCCCAGTCCATGGACCTCCTCTCTGCTGTATCCACTC
TGCAGAGACCCCCTCAGGCCCCTGGGGCATCTCCCAAAGGTGACCTCAAGCCCACAATTCCCCTAAGCTCCAGCCCTCCA
GGCGTACTGGCCTCTCCACCTGTCACTTAGACAACAGGTGTCTCCGACTCCAAACGTCCTCAACCAAAGTCCCGCTCTCA
CCCGAGCTGCGCTGCTGCCTCTGTCTTCCCCATCTCCCTTTCTAAATGGGAGCTCCTAGTGGCTCAAACTAAACAACAGG
GAGGATTTTGTTTTGTTTTTAAGTCTCTTTTGTCACCATTCCTTCCTGACTGCTACATCCCTCCACCAGCAAATGCTGCC
AACCTTATCTCAAGCAGAACATGAATCTCACCCACGCCAGCCTGACCACCCTGTTGCACGCCACTTCCTGTCACCTGAAT
ATTCCAGAAGCCTCGTAACTGGGCTCCCTGCTTCCAACATTGCCACATTCCCACCTCAGTCTATTCGCAACCGAGGACCC
TCAGGAATCCTTTCAAAGCATTTATCAAAATCTACAACACCTCTGCTCATGACTGTTTCCCACTCACTCGCAGTGAGATC
CAATCCCATCATGTAGATGGATGGGTCTCCTAGACCTCCCTATCACACGCTCTACTCCCTGCGCACTGGTTTTAGGCCAA
GCCACGCAGCACACCAAACTCTGCCTGTCCCAGGGCCTTTGCACGTGCTGTTCCCATTTCCTGAAATCCTCACAATTTGC
ACAGTCCCTCCCTCAATGCCTTTAACATTCTGCCCCCAGGTCACCTCCTTAGAGAAGCCTGCTTGTCCCATATTTCAAAT
TGCCTCCCTGGCCCAGCCCTTCCTCCCTCCTCACCTGCTTCTTGCTTTATTTTTCCACATGAAGTTCTCTCCACTGCACA
CGCTACACACTTTGTTCCATGCGGGTCCCCTTCTCCCAGGATGGCAGCTCCGTGGGCAGGATTTTTGTGTGTTTTATCAC
TGCTGGCCCCTGATTCTGAACAGAGCCAGGCATGTGGTGAGCACTCGAGGAACCTTTCGTGAGTCAGTGATACCCATACA
CCCTGGGCTAAGCCGTGTCCTCTCCCAACAGGGACCTCCTGGAGCCAGTGTCCTGTGACCTCAGTGATGACATGCCCTGG
TCAGCCTGCCCCCATCCCCGCTGCGTGCCCAGGAGATGTGTCATTCCCTGCCAGAGTTTTGTCGTCACTGACGTTGACTA
CTTCTCATTCCAACCAGACAGGCCTCTGGGCACCCGGCTCACCGTCACTCTGACCCAGCATGGTGAGGGGCTGGGGGCCC
TGCCCGGGGCTTGGTTTCCTGTGTGGACAGCGGGGGCACCAGGGGTGGTCCAGGGAGTCTTCAAGGCAGAAGGCTGTGGC
TTGGGGTTGGGTGAGGGTTTCTTGAGGGATGAGGGTATGGTCTGGTTACATGGAGACTTCAGAGCAGAGGGGCCCCTGAC
AAAGGCTTCTCCTGTGCCCCTGGCTGCTACCACCTCCCACTAAGCCATGGGCTTCCAGCACTGCAGGCTTGTCTTGAAGA
AAACCGTTCTCACCACTCACTTAGAAACCTACCAAGTTAGAAAACTCTGTCCGCTTAGTTGTTTCATCTCATCTCTGAAG
CAACCCATTTCCTGGATGTGGCCCTTGAGGCCCAGGAGGTCAGATGCCTCCTCTGAAGTCCCACAGCTGAGCAGTAATGA
CAGAGATGGGGTCAGCCTTCCCGGGGGCTTCCTGCATGGTAGACAGGGAGCTTGTCTCCTCGCTGCCCTGGAGGAGGGGC
CCCGGTGTGGGCCGAAATCCCACAGTGGTCCATGAAGCCATCGTGGTCTAGGGTGGGGTAGATGATTATTTTATGTAGT
TAGGTTTTATTTGCCTCTATATTAGAAAGAAATATAACCTGCACTTCGAAGTCCTGGATTCAAGGAAATGGTTGCTTAGA
ATGAAGCTAAACCTGAAAAGGGACACGTTTTATGAAAGTTTTCGTTGACGTGGCAAAAACCCATGCTGGTGGCAGGCTGG
TAGGAGGTACTGCAGGCTGTCATTGGGAGTCTCAGAACCACGGCAGGCTTGGTGCTGTTATTGGTGCCTTTGGCTGCTGC
TGAAACGGAGACAGAGGGAGGTGACTAGTCCAAGGCCTCTCTACTGCTGCTGGCAGAGCTGAGATCCAGTGCAGGATGGT
CTGAGTCCCCGGTCCTAACCACCGAACCACCTGGTGCTCTTTGCAAGGGTCACACGGAAGGGGCTCTGACCATGGCTTCC
TGCCCTTTTTTGCCACAACATCATAAAGCCACTGCCAGAGGAGGGGATCAGTTAGGCCACCAGGAGTCCATCAGAAGCAA
CATTTCCACACATGGGTTTGATGGACACGAGAGTCCCTTCCTTCCCGAATGGAGCTCAGGGGGCCCAGGCTGGAGGGAGG
GGAAACACTGTATGCCGTCCACCGTGAGAACTTACTGACAGTGGCGGGTGGGTGCCTCATGCAGGGGGTAAAGGGCAGGG
CCCCTGGGAGATCAGAACAGGCTTCCCAGAGGGAAGAGCTTTGCAACTCAGATCTGAAGGGTAGACAGGAGGGGAGGGCA
GTAAGAGCCTCTCTGGCAGGGTAACAGCTGTGCAGAGGCTGGGGGATCAGAAGGGCATGGGCAGCTGGGGAACCTGCTCA
CCATTGTGTCCAGGGAGCATCAGCTTCAAGGGCTGGACAGCGGTGGGAAGAAGCAGGAGACACAGGTAAGGACCTGTCCC
TGGAGAGGCTTCTGTCTCCTGCAGTCTCAGGTAGAGGGGACCCTCTAGGCATGGAGAGCACTGAGGGGAAATGACATGAT
CAGATGTGGATGTGAGAATGCTGGGGACAGGGGACTTACAGGGTAGATTGGAGGAGCATTAGTTGGGAAAGAAGTTGAAA
TTCAGGCTGTGGCCTAAAGCACTCATTCCTAGAAATGATGAGGACACAACTGGCTAGGAGATGGGGACATGAGGGCATGC
AAATCATAGTTCAGATATACACACAATTCATTCATTCAACCTTCTTTCCTTGAGCGCCTACATAAGCCAACGAAGCAGTC
AACAAACCAGCCAGCAACCCTGCCCTTGGAGCTTACAGCCTGGAAACAAGGAAGGTTTCTAAGGCGATGGAGACAACTTA
GATATAGGGAGAGAAGTGTGGCCCTTGGCCTCCTGGAGCCTGTGTTGAGTAGCGAGGTAGGGTCCAGCCTAATGCAGATT
AGCAATGAATCAAGGATGCTGGGAGGTACTGGGCAGGCGACAGGCATGCATCTGATCTGCTCTGAGTCTCCATGTTTGGA
GACCTTGGACATTTTACTTCCTCTATGAGTTTCTCCGTCTGTGAAATGAGCTGGTGGACTCAAGAGGTTTCTCTTGAGTT
GGCTGAGCAGGTTTCTGATGGGGCTCCCAGTCTGCGCAGTTTGTGGCAGCTTCCGAGAGGGCTCTGCCGGGAAGAGCTCC
CCCTCCATGACAGCCTCGGGGGCTGGGAGTGCAGTGACCCATGAGGGACGCCTGTCCTGGCTGTGGGTGAGGAGGGGCGG
TTCCCCTGCTGTGTGTCTGCCGTTCTGGGTTGATGGTTCCTGACATGCTCTAGCATGCCATAACCCATGTCCAGCAGAGA
GCACTTACATCCTATGTGTGAGGATGTTTTCGTTTGAAAGCCATCCCTCAGCAAGCAGACACCAGAAACCAGAAATCAGG
TGCCGTGTCTTCATTCTGCATTTTCTTGAACAACCCAGAGTTCCCAGGAGATAGATGCTTGCCTTGTGGCTGCAAGGATT
TCATGAGAAGCCCCAAAGTTGCTTACGCGTATTTGTTCATTCATTCACTCATTCACCTTGCCCCATAATTCACTGAGAAG
CCGCATCTCAGCCTGGAGGTAGGGAAGGGGGTCAGGACCAATCCCACCCACCCCCATCTCCTCACACCTTAGGGGAGGCA
GACACAGAAGCATAGGAATCCCTCAGCTGTGGTAAGGCCCTGGTGGAGGGAATTCCACTGAGCTATGGTGAAATGAGAGA
AGGAATGAGGGATTCCGCCTGGAGATGCAGATCCGAGGATGTTCCTAGAGCCGGAGGCATTTGCCCGGGGCACTGACAAC
AGGAAGGACCCTGGGCAGGAGGAAGGGAGCTTGGACAGCAGGAGGGGGGAGGCCGCTGAACCGCAGGCCCCTCTGCTAGC
AGGAGCCACCCAGGCCGCAGCGTGGGCAGTGGGGAGCCTCAGGACAGAGGAGGCTCCAATGAGTTTCCTCGCCAGCGCTT
TTTATGGAGTTCGGGTCACGTGCGCATTGCAATCTGCACGGCTTTCCATTGCTTTCATGTTGAAACCCTACAGTTTTGCA
GATGAAGGGCTGAGGCTCACAGAGGAGACGGGTCTTGCTCAAGGTCCCTCAGCTGCTGGGGGCAGGGGTGGCCTGGAACC
CCCTGTGTCCACACAAAAGGCCATGCAGGCCCTGACTGCCCCCCAGCGGTCCAGCCCTTAGGTGCCCTTCACTTCCTCCC
CTCCAGTCCAGCCTCCTGAGCCCAGGGACCTGCAGATCAGCACCGACCAGGACCACTTCCTGCTGACCTGGAGTGTGGCC
CTTGGGAGTCCCCAGAGCCACTGGTTGTCCCCAGGGGATCTGGAGTTTGAGGTGGTCTACAAGCGGCTTCAGGACTCTTG
GGAGGTAGGAACCACGGCCAGCTCTGCCCCAGCCCGAAGGGATGGGCAGCACCCCTCCTCCAGCACCCACTGTCTCCTGA
```

```
CAGGACGCAGCCATCCTCCTCTCCAACACCTCCCAGGCCACCCTGGGGCCAGAGCACCTCATGCCCAGCAGCACCTACGT
GGCCCGAGTACGGACCCGCCTGGCCCCAGGTTCTCGGCTCTCAGGACGTCCCAGCAAGTGGAGCCCAGAGGTTTGCTGGG
ACTCCCAGCCAGGTAATGTTGCCAGAGCCCAGGAAATGCCCCGTGGTGGGAGGGCAGGCTCATCAGGAGCTCCTGGCACA
GCAGGGTTCCTGGGCTCCACCTGGGGGCTTCCCAGATCTCCTGCTGCCATCTTTCCAGTAGCGTCCCTGGGCCGTCCCAC
CTCTACTGTGACCACTGACCAGTAGGACTCTGCATCTGTTCACTTTGGGTTTCCAGTTTTCTGCACGTTCTCTGCCAATG
GCAATTACAATAATAACAACAACAGTGCTATTAGCAGCTGTGTGTTAATGGAGGCTACAGGATGCTCAGGGCTTACCCAC
ATTTTTCAGTTCAATCCCCAAACACTGAAACTTAGATACTATTTCCATTCTCCCGGGAGGGCGTGCAGGTGCACAGAACT
CTCTCTCTCTCTCGGAGCTGTTGGACACACAGCTGGCAGGTTCAGGCTGAAGTTTCAGCCCTGGTCTTTTGGCCCCAG
AGCTCATGACCTCTGTGTGATGAATCACACGGTGGGCACCCACTGAGAGCTATGGGAGGGATGAATGACGGAGTACATGA
GGACCTGTCTCCAACCCAGGGGATGAGGCCCAGCCCCAGAACCTGGAGTGCTTCTTTGACGGGGCCGCCGTGCTCAGCTG
CTCCTGGGAGGTGAGGAAGGAGGTGGCCAGCTCGGTCTCCTTTGGCCTATTCTACAAGCCCAGCCCAGATGCAGGGTGAG
CATCTTTTTTCTCCATCCCCTCCCCTCCTCTTGGCCTTGCTCTCTCCAAGCTTCCTCCTGTCCCTGGGGCCCCAGCAGAA
GCCACAGCCCACCCTAAGCTCTCCTCCCTCCCGTGTGCCCTCCCTCTCCCTGCCCTCAGCTCTGCTGTGCTCCTCAGGGA
GGAAGAGTGCTCCCCAGTGCTGAGGGAGGGGCTCGGCAGCCTCCACACCAGGCACCACTGCCAGATTCCCGTGCCCGACC
CCGCGACCCACGGCCAATACATCGTCTCTGTTCAGCCAAGGAGGGCAGAGAAACACATAAAGAGCTCAGTGAACAGTGAG
TTTGCTCCTAGCCCGCTGTGGGGATGGTCTGGGACCAGCACACCCTCATTGTGTAACCCGAATCAGTTCAGGGTTCCTCC
TGGCCCCGTCTTCATGTTTGTCACTTTCAAAGAGATGCAGTCCAGTGACCAAAAGTGAACAGAGAAGCAATGAAACCACG
ACGGCAGTGGCCAAAAACAGGAGCAGATCTTTAAAACCTCTGATCTCTTGTCCTTTTCTTCTGCTTCCCTCTCCCATCCT
GCAGCTCTCTAAATCTCCACTGCTAGCCACACCCTCCTGGTCCTGTCACCAAAACCTTCCCTTTCATTCCTCATTGGATT
TTCCTCTTTCTGATATCCGAAATTCCCCACCGACTGATATTCTATCTTTAATGTAATTGATCTATGATGTACTTTTCAAC
TGGAGCCTGTGGTGTGTACAAATAGTGTTGATCCTTGGAGGTTAACATCCCTCGTTTCTGTGATGTAACAAGGACCCCAG
TGCAATGGAACCTCTCACGTTGTTTCATGATGACCAAGTTCTTCCATCCAGTCTTTAGCATATGTGAAGGGCAGAGGCCA
ATTTGTCTTAATTACCTGGGTTTGAATTTACCATTAACTCTCTTGGGATCCTCACTGGAAAAAGGAATTCTGATTGTTCA
AAAGCACAGGATATATTAAGGGCCTCATATAATGCCTGGCACATAAGAGACCTCAGCAAATTACGGGCATTCATATTATG
TTTATACAGTGAAGGCATCAAGGTTATAAGCATTCTCTTTTTTCTTTTGGGTAGACTGAAGCTCAGAGAGGTTGAGTGGC
TTACTTAAAGCTGCACAGCTATTAGTAGGCAGATCAAGATTAGAGTTCAGAACTTCTCACTCCCTGCCCAGTTTCTGCTT
TCTTTACCCTTTGCCTCTTTCAAGTTGTGGGTCTGCCGGCCAGGTGGGAGGTGCTGTCTGCAAAGGGCTTCCCTTTCTCT
TTGGCCACTATCTGGCTGGGGAGAGGCCTCACCTAGATGTTGTTGAAGGCCTGTTACAGCCGCTTTATTGGGGATTTTCT
GGCGATGAGAACGTGTGAATGTCCTGGCCTTTAGTCAACTCCCTACACCTCTGAGAGTGTCAGACAAGAGAGCCCATCAC
ACTGGTGGGATTGCAATCTTTGCCTCTACCACTGCTTAGCTGCCTTTCCTTAGGGCAAGTTACTTAATGGTTCTGTGACT
CAGTTTCCCTGTTTGTGAAAAGTGAAGGTTAATAGTACCCACCATATAGGGCTGTTAGAATGGAGTGGAATAATTCATGT
AGAATAAGTATGTATAACAGTGGCTAAAACATAGTCAGGCTGGGCGCGGTGGCTCACGCCTGTAATCCCAGCACTTTGGG
AGGCCAAGGCATGTGGATCACGTGAGGTCAGGAGCTCAAGACCAGCCTGGCCAACATGGTGAAACCCCGTCTCCACTGAA
AATACAAAAATTAGCTGGGCTTGGTGGCGGGTGCCTGTAATCCCAGCTACTCGGGAGGCTGAGGCAGGAGAATCACTTGA
ACCCAGGAGGCAGAGGTTGCAGTTAGCCAAGATCACACCACTGCACTCCAGCCTGGGCAACAGAGTGAGACTCCGTCTCA
AAAAAAAAAAAAAAAAATAGCCAGTTGCCTAGAATAGAACCAACTAACAGTGGTTTTATTTTTACTGCAAAAAATAAAAA
TAAAAATAGGAGTAGTGCAAGCACTGGGCCACATCACTACAAAACAAGTGTATCTCAGCATCTCCCACGAGAATACCACT
CAGGTCAAAACATGATATAGTGAAGTGGGGATGAAAAGGATCCAACCATGGGCAGAACCTGGGGTCTGGTGCCAGTGGAG
ACAGCCCCAGTGTCTAGCATGAGACACGGGGAATGTTCCGTTGGAGGGTGGGTATGATGACTCTCCTGAAAGCTTCCCTC
CCTCCAGTCCAGATGGCCCCTCCATCCCTCAACGTGACCAAGGATGGAGACAGCTACAGCCTGCGCTGGGAAACAATGAA
AATGCGATACGAACACATAGACCACACATTTGAGATCCAGTACAGGAAAGACACGGCCACGTGGAAGGTGAGGGCCTTTG
CCCAGGGACGGGAGAAACACTGGGGAGGGCGGGAGAAGGGAAAGCAACCAGAGGCATTCCACCTGCAAGGCGTCGGGCCC
TTGGCAGGTGACCAGTGAGAGGTAGCCACTGGGACGTGGTGATCACTAGGCTGTGTGGTCAGCAGGTCACTGTCCTGTCT
CTTGGTGAAGTAACTGAGGTTTGGAAAAGTGGCGTGGCTTGGCCAACGTGAACAGCTGACCCTGAGTCCCCAGGCAACAG
AAGACCCTCTGGGCAGGGAGGGGTTGAAAGGCCACTGGGAAGAAGGTTTTCAAAAGTCATGAAAGTTTGGGGTTATTTCC
TCAGAGGAATCTCATCTGGACACACATGGAGGCTCAGACAGAGCTGCTTCTAATGAGTCGGGGGTGCGCCCAGGCCAGGG
CTCGGTCCCCTGCCTCCACAGAGCCCAGAACAGAAACCACAGAACCAACCCCACACCTTCAGTCTAGAAATGGGGCAACT
GAGGCTAGGAGGGAGGTGGGCCAGTGGTGGAGCCAGGAGCGGGCCCTGGGTCCTGAACCCCCATTCTCAGGGTCCAGAG
TCCAGTCGGCCTGCACTGCGTTCCTGAAAAGGCCACAATATGGGTGCAAGCTGCCCCAGAAGGGCTGGGAGCTGAGAAGG
CTCAAAATAGGGTGGGACAGGTGGCTTCAGGGTTCTGGGCCTCAGTGTTGTCAATGTCAGGGGCTGCACTGACAGGCGGA
GTCCCCGGTGCCATCCGAAGTGCTGTCCGTGGGTGGGCCCTCAGGGAGGATCCACGGTGGTGAGAGAGAAGCCGCAGCAG
GCCTGGGGTATGGCAGGAGCTAGGAGCCAGCGAAGCCGAGGGTCCAGGTGGGAGGGATTTGCAGCTGCTCCCACGGGCAC
CGGGCCAGGCCTCACCCTCAGTGCCAACCCACAGGACAGCAAGACCGAGACCCTCCAGAACGCCCACAGCATGGCCCTGC
CAGCCCTGGAGCCCTCCACCAGGTACTGGGCCAGGGTGAGGGTCAGGACCTCCCGCACCGGCTACAACGGGATCTGGAGT
GAGTGGAGTGAGGCGCGCTCCTGGGACACCGAGTCGGGTAGGTGAAGGCTGGAGTCCAGAGCTTCTGGCCAGGACCAGCT
CATAGTTTCTCACTGCCAGAAAATCCCCAATGCAGCAGCCGTAGCAGGCCTGCAACAACTTGTAGGTGAGCCGTCTCCCC
GATTAGATGGTGGCCAAAGAGAAAGGGAAGGCCTTTGCAGAGAGCACCTCCCACCTGGTCATCAGACCCGCAAGTTGAAA
GAGGCAAAGGGTAAAGCAGGCAGAAACTGGGCGTGGAGTCAGAAGTTCTCCACCCACTCTTCATGAGACAGCACAGCTGG
GCAGAGGATGCCACATCTCTGGGTGGGCACAGAGATGTGGGGCTCCAGACCCCACTCCACTCAGGCCTCCTTCCCTCCCT
TGGACCACTAGTCGTTGGAAAGTGACACCTGGTGTTCAGTACAGACTGCAAGTGAGGACTTGAGGGAGCAGAGGAGAGCA
GGTCTCATGATCGCTCCTCCTTTAAACTGGGACTTAGGCTTCTGTTAATATGGCCCTTGGTTTCTTAGCTTTTGGGACTA
```

```
CCAAGTTTTTGTGGTTGCTCAGCCCGAGTTTCCTGTTACCATGGAAGCAAGAAAATCTAGAGCCACAGACTGGGTCCTTG
GATTGGAAGGTTTTGCTGTGATTCCTTCATCTTCCCACCTCCCTGCACTTGCTGCGTGCACTGACCCCTCACCTTTCACC
ATAGTGACCACCAGGCGACTCCCGCAGAACAAGACAAGTCCAAGGGGTGGTTTCAGAGAGAGTAAGGATACAGGAGATAT
TGGGAGCAACCCAAGTCCCCAGCCAGGCTTGGTAGCGGCAGGGGCCCAGGGAGAGGGCCACAACCAGGTAGAAAGGAGGC
CGGACCAATGGGCCCAAGAGAGGAGGAAGAGGGGAGTCCTGGAGGAACCTGGCCTGGGAGCTCAGGGCTGGGCAGCAGGT
GGGATCAGGGGCCTCTGGAGGGGCCTTTGCTCCTGCTGGTGCCTCTGCCACTATGCCTTTCCCCATCTCCACCTCTAATC
CTTCCTATTACTGGAGGCCCAGCTAATATGCCACCTCCTTCAGGAAGTCCCCTGTGATTGCCATGAACAGTGGTTCTTGC
CCTCCCTGTAGCACCTCCTGTGCTATAGATCCTGGAAAGGATCTGACCCTCCTGTGTCCTCCCAGCACTTTGAACACTGT
GGGGGTTCAGACCAATGCCCTTCCCAGCATGGATCTGGCCTCTCTCCCTAATCCCCCAAGGCAGTAAGTTCCAGAGCCC
TGGGCCTGCACAGAGCGGGGTCTTAAGGAATACTGCACCAACCCCACTCCCTGCCCTGAGGTCGATTTCCCGCCCAGATG
CTGACATTCCTCTTTCTCCCCGGCTGCTGGAAGTGCTGCCTATGTGGGTGCTGGCCCTCATCGTGATCTTCCTCACCATC
GCTGTGCTCCTGGCCCTCCGCTTCTGTGGCATCTACGGGTACAGGTGAGGGGACTCTGTGGGGCTGGAGGTGGCAGCCGA
GACCCCAGAGGGCACTGGGGAATCCCACCCAGCTCCCTCCAGGCCCTGCTCTGCCGCTCCCTTCCTGGGCCTCAGTTTCC
CCTCTGGGCATGAGCATGGGAGCAGCTGGCCATGAGGTCTCTGATGGCTGTCACCTCCGTGGTGTCTTCCAGGCTGCGCA
GAAAGTGGGAGGAGAAGATCCCCAACCCCAGCAAGAGCCACCTGTTCCAGGTAGGAACTGGCTGCGAGGGGCGGAGTGGG
GGCTTCTCTGTTCCTGCCTCTCTTGTCTCTGTCCCCACCTCCTCCTTCCCTTCCTGTGGGCTTTGGGTGGTAGGGATGTA
GGTGGACTGGGCTTTGGGAGCTTTGGGAGTGGGGCCAGCACTTGGAAATTCATTCCCGGGATTCCCTGGAGTGCCCACAC
CTGGCCCTGCCTGCCAGAGCTCTGCATGGAGCCCAGTGGAGAGAACTGAGTGTGGGGATGCCTGGGACAGGGTGGGCTCA
CAGAGGGGGTTCCTGCTACCAGGATGGATGGGGTGTCAGGAGGCAGAAGGGCTCCCCAAGGACAAGATACCTGGGCTGAG
CTTTGAGGGTCCCAGTGGTGCCAGGTGGCAAAAGTTGCTCTGGCTCAGGGCATCTTGAGGGTGGAGACAGAAACCCTTCA
GGAAGGGAGCTGAAGGGGTGGGACAGGAGGCTGGGGGCCATGTTGGGAGGCTGAGATGACCATGGGGAAGACACCAGCGT
GTGGGTGATGGGAGCCCTATAGTCAGGCAGGCAGGGCCAGCTTTGCCTGTGGAGGGGTCTCTTGGCTGGTGTGGAGGACA
AATGGGGGTGAGCATGGAGACAGGCACTCCAGGTTAGAAAAATCACAGGAAAAGGGCCCTGTCCTCAGGTAGCAGGGACA
TGGGGGAAGTCCCCGCCCCTCTCTGGGCCTCACTTTGCTGGTGTCAAAAGGCCGTGGCCAGTCCTGAAGAAGTGGAGATT
CTTCTCTTGTCTGGGGGCTCCTTGAATCCTCCTGCACCCCCGTCACCCTCTGCCTTGCCCCCACCTCGGACCTCCTGATG
CTCACCGGCCCAAATGTCTCTGCTCTTGCAGAACGGGAGCGCAGAGCTTTGGCCCCCAGGCAGCATGTCGGCCTTCACTA
GCGGGAGTCCCCCACACCAGGGGCCGTGGGGCAGCCGCTTCCCTGAGCTGGAGGGGTGAGTGGGCTCGTGGATCACTCCT
GACCTTTGGGGTTCATACGGGGGGCTGACTCCATTGTGGAAATCAGGGACTCAAGTGAGGCTGCCTGTGCTCACTGTGA
AGGGATCCAAGGGAGCTGGTTTGCACTAAAGCAGGAGGCACCTGGGGGGGGATGTGAGGAAGAACTTCCTCTTTCCAGGTA
AGGAAGTGCCAGACAAGGGGAGTGACCGGAAGTGAAAGAGGGAAACAGTTGGCCTTCCCCTCCCTGGCTGCCTCAGAGGC
GTCACACTGCAGAGGCCTCATGGGGTAGCTGGGGCTGCAGACTCCCGGGAACTCGGCGCCTGAGCCGGCAGCTGACCACC
AACTCGGCAGGGAGAAGGGGGTGGCATGGTTGATAGAATGATGGAAGGGTTGCCCAGTGCACAGGCTGGGCATGAGCCTG
GTGCTGAAGGAGGATGGACTTTAAGGTCAAAAGGGAGAGGGTACCAGCCTTGCAGAGGAAGACCTTGAGCTCAGCATGGA
GGATGGAGCTCCTGAGCCACGGAAAGACTGAATCCATGTCCCATGTCCTTCTCTGGGGCCCCTGCTCCCTCAACCCTGTC
CCGTTCAGGTTCTCTCTGTGAGATCTGGGGGGACATCAGGGCTTCCAGAGAACCATCTCCACCCCACCAAGACCCTTGTGC
CTGACCCGGATCATCTGCCCAGGGTGGTCCCAACTCTTCTGCCCATTTCTTCCCACAGGGTGTTCCCTGTAGGATTCGG
GGACAGCGAGGTGTCACCTCTCACCATAGAGGACCCCAAGCATGTCTGTGATCCACCATCTGGGCCTGACACGACTCCAG
CTGCCTCAGATCTACCCACAGAGCAGCCCCCCAGCCCCCAGCCAGGCCCGCCTGCCGCCTCCCACACACCTGAGAAACAG
GCTTCCAGCTTTGACTTCAATGGGCCCTACCTGGGGCCGCCCCACAGCCGCTCCCTACCTGACATCCTGGGCCAGCCGGA
GCCCCACAGGAGGGTGGGAGCCAGAAGTCCCCACCTCCAGGGTCCCTGGAGTACCTGTGTCTGCCTGCTGGGGGGCAGG
TGCAACTGGTCCCTCTGGCCCAGGCGATGGGACCGGGACAGGCCGTGGAAGTGGAGAAGGACCGAGCCAGGGGGCTGCA
GGGAGTCCCTCCCTGGAGTCCGGGGGAGGCCCTGCCCCTCCTGCTCTTGGGCCAAGGGTGGGAGGACAGGACCAAAAGGA
CAGCCCTGTGGCTATACCCATGAGCTCTGGGGACACTGAGGACCCTGGAGTGGCCTCTGGTTATGTCTCCTCTGCAGACC
TGGTATTCACCCCAAACTCAGGGGCCTCGTCTGTCTCCCTAGTTCCCTCTCTGGGCCTCCCCTCAGACCAGACCCCCAGC
TTATGTCCTGGGCTGGCCAGTGGACCCCCTGGAGCCCCAGGCCCTGTGAAGTCAGGGTTTGAGGGCTATGTGGAGCTCCC
TCCAATTGAGGGCCGGTCCCCCAGGTCACCAAGGAACAATCCTGTCCCCCCTGAGGCCAAAAGCCCTGTCCTGAACCCAG
GGGAACGCCCGGCAGATGTGTCCCCAACATCCCCACAGCCCGAGGGCCTCCTTGTCCTGCAGCAAGTGGGCGACTATTGC
TTCCTCCCCGGCCTGGGGCCCGGCCCTCTCTCGCTCCGGAGTAAACCTTCTTCCCCGGGACCCGGTCCTGAGATCAAGAA
CCTAGACCAGGCTTTTCAAGTCAAGAAGCCCCCAGGCCAGGCTGTGCCCCAGGTGCCCGTCATTCAGCTCTTCAAAGCCC
TGAAGCAGCAGGACTACCTGTCTCTGCCCCCTTGGGAGGTCAACAAGCCTGGGGAGGTGTGTTGAGACCCCCAGGCCTAG
ACAGGCAAGGGGATGGAGAGGGCTTGCCTTCCCTCCCGCCTGACCTTCCTCAGTCATTTCTGCAAAGCCAAGGGGCAGCC
TCCTGTCAAGGTAGCTAGAGGCCTGGGAAAGGAGATAGCCTTGCTCCGGCCCCCTTGACCTTCAGCAAATCACTTCTCTC
CCTGCGCTCACACAGACACACACACACACACGTACATGCACACATTTTTTCCTGTCAGGTTAACTTATTTGTAGGTTCTGC
ATTATTAGAACTTTCTAGATATACTCATTCCATCTCCCCCTCATTTTTTTAATCAGGTTTCCTTGCTTTTGCCATTTTTC
TTCCTTCTTTTTTCACTGATTTATTATGAGAGTGGGGCTGAGGTCTGAGCTGAGCCTTATCAGACTGAGATGCAGCTGGT
TGTGTTGAGGACTTGTGTGGGCTGCCTGTCCCCGGCAGTCGCTGATGCACATGACATGATTCTCATCTGGGTGCAGAGGT
GGGAGGCACCAGGTGGGCACCCGTGGGGGTTAGGGCTTGGAAGAGTGGCACAGGACTGGGCACGCTCAGTGAGGCTCAGG
GAATTCAGACTAGCCTCGATTGTCACTCCGAGAAATGGGCATGGTATTGGGGGTCGGGGGGGCGGTGCAAGGGACGCACA
TGAGAGACTGTTTGGGAGCTTCTGGGGAGCCCTGCTAGTTGTCTCAGTGATGTCTGTGGGACCTCCAGTCCCTTGAGACC
CCACGTCATGTAGAGAAGTTAACGGCCCAAGTGGTGGGCAGGCTGGCGGGACCTGGGGAACATCAGGAGAGGAGTCCAGA
GCCCACGTCTACTGCGGAAAAGTCAGGGGAAACTGCCAAACAAAGGAAAATGCCCCAAAGGCATATATGCTTTAGGGCCT
```

```
TTGGTCCAAATGGCCCGGGTGGCCACTCTTCCAGATAGACCAGGCAACTCTCCCTCCCACCGGCCACAGATGAGGGGCTG
CTGATCTATGCCTGGGCCTGCACCAGGGATTATGGTTCTTTTAAATCTTTGCCTTTCAGATACAGGAAAAATAATGGCAT
TAAATTGCTTTAATTTGCATTATTTTAGTTATCCAGTTTGCACATATTTTTATAGGTATCTTAGGCATCGATTGGTATTT
TTTAACTGGGCCAAGCCCATTAAGGTCTTTCTTCTGTTGGGTGCTATCATTTTCTGATTAAGTCTTTTTGACTATTGACA
TACAGTCTTTCACAGATGGTGGAGTGTTTTTCCCCCAAATCTGTTGTTTGTCTTATAATGTTGTATATGAGGTTTTATGG
TGTATGAATATGAATGCTTCTGTAATGTCAAACAGATCCCTAGTAAACTCCTTCTTCACTTTTACTGTCAGATTTACAAA
GGTCCTCCCATTGCAAAGCAGTGTTTGTCCTAATTTATATATTGTTTTTCTAGTTCATTTTGTGTTTCCAACTTTTCATG
TAAAATTTTAATTATTTTTGAATGTGTGGATGTGAGACTGAGGTGCCTTTTGGTACTGAAATTCTTTTTCCATGTACCTG
AAGTGTTACTTTTGTGATATAGGAAATCCTTGTATATATACTTTATTGGTCCCTAGGCTTCCTATTTTGTTACCTTGCTT
TCTCTATGGCATCCACCATTTTGATTGTTCTACTTTTATGATATGTTTTCATAAGTGGTTAAGCAAGTATTCTCGTTACT
TTTGCTCTTAAATCCCTATTCATTACAGCAATGTTGGTGGTCAAAGAAAATGATAAACAACTTGAATGTTCAATGGTCCT
GAAATACATAACAACATTTTAGTACATTGTAAAGTAGAATCCTCTGTTCATAATGAACAAGATGAACCAATGTGGATTAG
AAAGAAGTCCGAGATATTAATTCCAAAATATCCAGACATTGTTAAAGGGAAAAAATTGCAATAAAATATTTGTAACATAA
AACAAAGTGAAACCCTGAATTTGTGTGTGCATGTTGGTGTAGTTGGAGGAAGGGGTTGCTCTTTGAAACCTCAATTGCTA
TTGTAAGTGATACAGCTCCAGTGACTGGAGGAACACCAGGGTCCTTAGTCTTGCGCCGATTTAAATAAAACGACACGGAA
ACACATGGAGTGGTTTTAAGGAGTGGAGAGTTTAATAGGCAAGAAAGAAGGAAGAAGCTCCCCTGTACAGAGACAGAGGG
AGGGGGGATCCAAAGCTGAGAGAGGAAACCCTGAGTGCCACAGAAATAAGCCAGTTATATGAGGAGGCTAGAGAAGGCAG
TATCTGATTTGCATAGGGCTCAGGGGATTGGTTTGACCAGGCATGTCATTCATGTAACCCCTGAAAAACCTGGCCCTTTC
ACCCTAGCATTTTAATATGCAAATGCAGGGCGCCATGATGTTCTACACAGGTGGGACTATGTGGGGGTGGCCATATTGCC
AGGCAAACATGGGGACAAGGAAAAGATGGCGGGAATCCCCATGTTTGGGTGGACCCAGTTTCTAACGGTCTGCATTTGCA
TATCAAAGGTTGCCAGCCTGATTCTAAGAGCCGGGGCTTTCCTGCTAGACAAGAAACGTTTTTTGGAGCTGCTTTTAAAA
CAGAAACGAAAACTTCCCAAGGACCACTTTTCCTCTTTATCTGCCTCAAATAATTTTTTAATAATTCCTATAACACAAGG
AAATGAATTCTGCCAAACAGAAGGGCCTTTGGTCTTTGGGGCACTACAGTGGGTACATGATGGCGTGTGGAACACCACGC
AATGGAGGGAGACGCAGGGCACTCCTGGGAAAATCCAGGAGGGATGAGGGAAGAGGAAAGACAGTGAGGGAAAGGAGGAT
AAAGAACACACTTTTAAAAAATCGTTCATTATTATCTGAAATTCCAACACAACTGAACGTCCTGTATTTTTCCCTAGTCA
TGAGAGTGAAAAACTAAATAGAGACTGAGTTCCCCATACAAATAACTGAGAGTGATCGAGAGCTTATTCCTGGCAGGCCC
CATTCGAGTGACCTCTAAGATCAATTCATTTAGATACTATGGAAGTGGAAGATACTATCTTTATCATTTTTGTCATTGAG
GAAACTAAGGCACAGAGATGTCATACAACTTGCCCCAGGTCTGCCAGCAGGTAAGGGGCAGAGCCAAGATTTGAACTGTA
GCCCTGTGTCTCAGAGCCTGCCCTCAAAAGGTAAGTCTCTCACCTAAAGATCCATACGGAGATGTAAGATGATCCATGTT
GGTTGGCAAGACAGAAAAATCCATAAACCAAATAAATTGATTTGGTGGCAGGGGAGATTGAATCAGAGGTGACCCTGAA
AAGTGTCTGTTTATCTGGGAGCTGAACACTGAAGGTGCAATTCTCACCCATTGGAGTCCAGAGAGACCTTCCCTGGAGGA
CTGCAGCTCCCTCATCTGTCTTCCCCTGAGAACCCAGCCCAGAGCCATGCATGTAGTAGGTGATCATTGAGGTGAGCAAG
GCCTGCAGGAGGCTGTCCCTAAGATGGAGGAGAACAAAAAACTTATTAGGGGGATGAAAGGGAGGGCAGGAAGCAGAAGT
GGGCTAGGGAAGCCATTGGCCATCCAGAGTTGACATTTGTCCCCCCATGGGGAACTTGGGGGATGAATTGCCTGTTGGAA
GAGCCCATGTTGGGTGGAAATGGCCAGTCCCGGAGCCCTTCATGGTGGGGAACCCTGAGAACACACTACATAAGGAAGGA
GCAGGGCTGGAGACCATCAGCAGCTTGGGCCTCACAAGCATTTCACATGTTTAATTGATCACACAGAACCCTTGCACCAA
GACAATCGTTTGCTCCTAGAGGGAGCCTTTCCTTCGTTTGGAAACAATCCTTTCCCAACCCCAACAAGGCAACCTGCATC
CGGTGGTGCTCAATCAATAGAAGCTGGGCAGCCCGGGTGCAGGTCAGTGTGCCCAAAGCATTGAAATGCGGTGATTTGG
GCCATGGGTCATGCTGGGTGTCCTGAGCAGGGGTAACTTCATCTACCCAGTGACGCAGGTAGCAACTCTCTGGGTCTGGT
GGCACAGGAGGTAAAGGAATTTACCAAGCCAGTTGTAGGTAAAGAAAGGCAGATTTATTAGAGAAGGTATGAAAATACGT
TGCAAGGTCGCAATTACAATGAGCAGAGAAGGGGCTGTCTGCAAAGAGGCAGTGGCTGAAGGGAAGTTTCATAGAGTCAC
GTTGAAGAGGGCTACATGCAGAACAAGCAGAACGAGGTTGTTGTGCCTGGTTGTTTGTGATTGAGTCTGTCTCTCAGAACAA
TTGATGACTTTTCTCCCCACTTGGGGGCCCTCCCCAACCTGGGGCCTCTTCCTCATTGTTGCTTATTTATCAGGACTCCAC
CAGGGACAGAGTCACCCTACAAGTCCTAACTCAAGTGTGGGTTCTTCTTAGAACACAGCCCCTCCAGCTCCCTTGGAAAT
CCCCAAGTCTTCCCAAACGATGTAGGACTTTTCTTCTTGGTCACTTTGCAAGCCGGGGCCCCCAGTCAGTGATGCCTTGC
CCAGGTCTCACTCGGCCACGCTATCGCGTATAACTTGTATAACTTGTACCCATGTTCAGTGGTTCCTGAGCCCTTGTACC
TCGCCCAAGAAGAATGAAGATATACTGGACATTGGAGGGTGAGGAGGGCTGAGAATAATTTTATTGAGTGATGAAGATGG
CTTTCCGTGGAAAGGGGATTCAGGGGTGGGAGGTGGGGTGTAGGGGGAGGTTTTGCCACGTGGCTCGGTCTGGGGCCCTT
TATGGATTCAAAATGAGGAGTGTATGCTGATTGGTTTGTGAGTATGCAAAAAAGGTTAAAGTGACAACACCACTCAAAGC
TGTGCACAATGGTGTAAGAAACCAATTAGGAAAGGGTAGGGAGGTATATGTAAAATAGGTGAAGGGTGAGGACTAATCA
GAGGTAAGCATGCCAAACAGAAAGACAAGTTCTCAATCCAGTCCAGTCGGATGATTTAACTTGTAGCTTGGCTTTCAGGC
TTTAAACTGTCTTTGGCTTGGAGGTGGGGTTTCACGGAGGACCTGCCCCATCTGCATAGGCATTTGTCCACCTCCTGCTT
CTATCACAGAGCATGTCTCAGCCTCTCTGTCCTGCCACTCAACTTTGTCCCCCTGCCTTTATTACCCATCTTGTAATTCC
TGGCTCACAGCTTTGTGTTCCAACTAGACTGAGAGCTCCCTGAGCCCAGCTACTGCCAGGCTCACTTCAGGCTTCAATGC
CCAGCTCAGACGCAGTGCATCTGCTAGTGTGTGTGTGTGTGTGTGTGTGTGTGTGTGTGTGTGTGTTAAAAGTGTTTC
TTGCTGGCTGGTTCTGGAAATTTGTCATTCTCTGTCCTCTATCTTCCTGTTAAAAACTTCAGCTGAATTAAATTTAAAGG
AGTTTAATTGAGCAATAAACAATTCGTGAATTGGGCAGCCCCCAGAATCACAGCAGATTCAGAAAGACTCCAGGGGTGCC
TCATGGGCAGAACAAATTTATAGACAAAAAAAAAAGTGACATACAGAAATCAGAACTGAGGTACAGAAACAGCTGGATTG
GTTACAGATTGGCGATTGCCTTATTTGAACACAGTTTGAACGCTCAGCAGCGTATGAGTTGTTGAAGTTTATCTGCTGGG
ATTGGCCAAAACTCAGTGATTGTTACAGATGCATACTCCTAAGTTAGGTTTTCAATCCTGTCTACCTATTAAGTTAGGTT
GTAGTTCATCCACAAGGACTCAAATATGGAAGTACAGAGTCCTTCTCAGGCCATATTTAGTTTGCTTTGACATCCCCATT
```

```
GAGATGACTCAACTATTCGGCTAAGGGCTGTAGACAGTGAAGAAATGTGACTAATTGCTGGATACTGTAGTATATTGAGT
GTGCAAGAGCATTATATCTAAAAAACAATGTACATACTGCAATTTAAAATGCTTTGCTGCTAAAAAAAAATAATGCTAAC
AGTCATGTGAGCCTTCAAGTTGTCATCTTTTTGCTGCTGGGGGGTTCTTGCCATGATGTTGATAGCTGCTGACTGAGCGGA
GTGGTGGTTGTTGAAGGCTGGGGTTGTGTTGAAGGCTGTGGCAATTTTGTGATTGGCCACCAATGGAGTTTGCTGCATCA
ATGGACTCATCCTTTCATGAAATTTTTATTGGTAGCATGCAATGTTATTTGATAGCATTTCTTTCCAAATTGAAGTTAAT
CTTCTTAAACCCTGCTGCTGCTTTGTCAACTAAGTTGATTTAATATTCTTCATCCTTTGTTGTTATTTCAACATGTTCAC
AGCATCTTTACCAGGAGTAGATTCTATCTCAAGAAACCACTTTCTTGCTCCTCTGTAAGAAGCAGCTCCTCACCTGTTC
AAGTTTTATCATGAGATTGCAGCAATTCAGTCACATCTTCAGGCCGTACTTCTAATTCTAGTTTTCTTTCTATTTCCACT
ACATCTGCAGTTCCTTCCTCCCCTGAAGTCTTGAATCCCTCAAAGTCATCCATAAGAGTTGTAATTGACATCTTCCAAAC
CCTGTTAGTGTTGATGTTTTGACCTCCTTCCATGAATCGCAAATGTTCTGAATGATATCTAGAGTAGTGAACCCTTTCTA
GAAGGTTTTCAATTTACTTGGCTCAGATCCATCAGAAGAATCACTGTCTATGACAGCTATAGCCTTAGGAAATGTGTTTC
TTAAATAACAAGTCTTGAAAGCAGGAATTTCTCCTTGATCCAAGGGCTGCATAATAACTGTTATGTTCACAGACATGAAA
ACGACATTAATCTTGTGTATCTTCATCAGAGCTCTTGGGTGACCAGGTACATTGGCAATAAGCAGTAATATATTGAAAGA
AATATATTTTCTGAGCATTATATCTCAACAGTGAACTTAAAATATTCCTAAACCATGCTGTAAACAGATGTGCTGTCAGC
CTTTACTGATCCATTTCCAAAACACAGGCAGAGTAGATTTAGCATAATTTTTGTATTAGTCTGTTTTCATGCTGCTGATA
AAGACATACCCAAGTCTGGGCAATTTACAAAAGAAAGAGGTTTAATTGGACTTGCAGTTCCACGTGGCTGGGGAAGCCTC
ACAATCATGGTGGAGGGCAAGGAGGAGCAAGTCCCATCTTACATGGATGGCAGCAGGCAAAGAGAGAATGAGGAAGGCGC
AAAAGCAGAAACCCCTGATAAAACCATCAGATCTTGTGAGACTTATTCACTACCAAGAGAATAGTATGGAGGAAACTGTC
CCCATGATTCGATTATCTCCCACTAGGTCCCTCCCACAACACATGGGAATTATGGGAGTATAATTCAAGATGAGATTTGG
GTGGGGACACAGAGCCAAACTGTATCTTTTTTTTTTTTTTTTTTTTGAGATGGAGTCTCACTCTGTCACCCAGTGCCACG
ATCTCGGCTCACTGCAAGCTCCACCTCCTGGGTTCACCCCATTCTCCTGCCTCAGCCTCCCGAGTAGCTGGGACTACAGG
TGCCCACCACCACGCCCAGCTAATGTTTTGTATTTTTAATAGAGACGGGGTTTCACGGTGTTAGCCAGGATGGTCTCGAT
CTCCTGAACTCGTGATCCACCTGCCTCGGCCTCCCAAAGTGCTGGGATGACAGGCGTGAGCCAACGCACCTGGCCAACTG
TATCAATACTTAAGGGGCTTAGGGTTCTTTGGAATTGTAAATGAGCATTGGTTTCAATTTAAAGTTACCAGTGGCATTAG
CCCCTAACGAGAGATTCTGCCTGTGCTTTGAAGCATTGCAGCTTCAAATAAAAGGCATCTTCTTCCGATAAAAAGGCTAT
TTTGTCTACATTGAGTATCTGTGTTTAGTGCAGCCACCTTCACTAATCATCCTAGCTGGATATTCTGGATAACTTTGTGT
AGCTTCTCCATCAGCACTTGCTGCTTCACTTTGTTCTTTTATGTTACAGAGATGGTTTCTTTCCTTAAACCTCACGAACT
AATCTCTGCTAGCTTCCAACTTTTCTTTTGCAGCTTCCTCACCTCTCTCAGCCTTCAGGGAATTGAGGAGCATTAAGGCC
TTGCTCTGGATGAAGCTTTGGCTTAAGGGAATGTTGTGGCTGGCTTGGTCTTCTATCCAAACCACTAAAATTTCTCCATA
TCAGCAATAAGACTGTTTTCTTTTCTCATTCATGAGTTCACTGAAGTAGTATTTTTAAGTTCCTGCAAGAATTTTTCTTT
TGTATTCATAACTTGACTATTTTCCCAAGAGGCCAAGTTTTCAGGCTATCTAGGCTTTCAGTGAAGCTTAATCATTTCTA
GCTTTTGATTTAAAGTAAGAGATGTGTGCAACTCTTCCTTTTACTTGAAAGCTTAGAGGCCATTGTAGGGTTATTAACTG
GCCTAATTTATTTATTGTTGGGTCTCTGGGATGAGGGAGGCTAGATTAGGTGTCTAGTTAGGGCCTGCTTCCTCATAGAT
GAAGGCTTCTCACTGTAGGGTGAGAGATAAGAGAACGGCCCGTAGGTGGAGCAGTCAGAGCACACAGAACATTTGTTGAT
TAAGTTTACTGTCTTCTATGACAGATCATGGCATCCCAAAACAATTGCAGTAATAACATCACAGATCTCAGACCACCATG
ACAGAGACAATAATCATGAAAAAGTTTGAAATATTGCAAGAATTACCAAAAGGTGACACAGAGATGAAACTGCCCCTATA
AACTTTATGAAATGAATCAGGGAAGAAGGAAGGGGGAGAAATAAAAGTCACCCACGCTTGCAGCACATTTGGCCTCCATC
ATGAGGTCGGCCTGCTCTCCAGCCTGCGTCCTCACAGCTGTCTGGTGCCTGTCGTACTAAAATCACATAGACCCTGTTAC
AAGATCACGGTTCCCCTTAACTGCTCCGTAGATAACAACGTGAACATTATAAAATGTTTAGTTTTCCCTGTGAGATAGTC
CTTCAGGTCCCATACACTGATGAAACTACTGACCCAGCTGGTCTGAAGGACCCCACAATGGGCTGACTCACCAAAGAATG
CAGTTTCCATATCGTGATGATTTCATCCCCCCCTTCCCCCGACCAATCAACAACCCCAATTTTCCAATTATGGAGGGAGT
CTCTCACCCTCTATAATCCCCTTAAAAACCCCAGACCAAAGCTCCTTGGTGAGATAAACGGGAGGGCCTCCTCCCATCTC
GTCACTCAGCACTCTGCAATCATTAAACTTTCTCTGCTGCAAACCCTGCTGTCACTATGTAATGGGTCTGTTACTGCACA
GTGGGCATATGAACCCATTGGTCCTATAACAGAGGCATGAAGTGAGCACATGCTGTTGGAAAAATGGTGATGGTAGACTT
GCCAAAAGCATGGCTGCCACAAACATTCAATTTGTATAAAACACGGTATCTGCAAAGCACATAAAACAAAGTGCAATAA
GATGAGGTGTGGCTGTATATTCAAATTAAAATGAGATGTAGTTCTTTTACTCACCACATTGGTAAATATTACTGGGAAAA
TAACTGATAATATCCAGAGTGGGAAGTGTGTGGGGGTACAGGTACTCTTGTACTATAAAATACAAGTCTATTTTTTTAAT
AGACTTTACTTTTTAGAACAGTTTTAGGTTCTCAGCAAAATTGAGTGGGCGGTTCAGAATGCCCATATAATCCCTGCCCC
ACACATGCACAGCCTCCCCAACGATCAACAGCCCACACTACAATCGTCTTAGCCCACTGGGCAGCTATAACCAAAACAAC
ATAGACCAGTGGCTTATAAACAAAAGAATTTATTTCTCACAGTTCTGGAGGCTGGGAAGTTCAAGATCAACATGCTGGCA
GATTTGGTGTCTGGTTAGGGCCTGCTTCCTCATAGGTGAGGCTTTCTCACTGTAACCTCATATGGCCGAAGGGGCGAGGG
AGTCTCTCAGGTCTCTTTTATAAAAGCACTGATCTCATTCCTAAGGCCTCTGCCCTCATGAACTAATCACCTCCAAAAGG
CCCATCTCCTAATCCATCACCTTGTGGGGTAGGATTTCAACACAGATTATTTTGTAATTCATAGACGTGATGATTGGGTG
TTCCTATGCATGTGTGAGATGTGCCACCTTCAAACCTTGTTACTGTAAGAGTTAAAGAAAGAGGAAAGAAGCCAGAAAAG
GGGCTCAACAGTCAAAAACAGGTTTATTTTGGAGAATAAACCTGAAAGGGGCTTCTGGCCAATTTCAGTCAGGAGGATTC
TCTATTGCAACTATCAACAGCCTGCACTACAATCGTCTTAGCCCATTGGGCTGCTATAACCAAAATAACATAGACCGGTG
GCTCATAAACAACAAGAGT
```

HUMAN mRNA SEQUENCE : hR5-002.1 (Seq ID No: 35)

```
GCCTGGAGGAGGCAGAGGCCAGGAGGGAGAGGTCCCAAGAGCCTGTGAAATGGGTCTGGCCTGGCTCCCAGCTGGGCAGG
AACACAGGACTTCAGGACACTAAGGACCCTGTCATGCCCATGGCCAGCACCCACCAGTGCTGGTGCCTGCCTGTCCAGAG
```

```
CTGACCAGGGAGATGGTGCTGGCCCAGGGGCTGCTCTCCATGGCCCTGCTGGCCCTGTGCTGGGAGCGCAGCCTGGCAGG
GGCAGAAGAAACCATCCCGCTGCAGACCCTGCGCTGCTACAACGACTACACCAGCCACATCACCTGCAGGTGGGCAGACA
CCCAGGATGCCCAGCGGCTCGTCAACGTGACCCTCATTCGCCGGGTGAATGAGGACCTCCTGGAGCCAGTGTCCTGTGAC
CTCAGTGATGACATGCCCTGGTCAGCCTGCCCCCATCCCCGCTGCGTGCCCAGGAGATGTGTCATTCCCTGCCAGAGTTT
TGTCGTCACTGACGTTGACTACTTCTCATTCCAACCAGACAGGCCTCTGGGCACCCGGCTCACCGTCACTCTGACCCAGC
ATGTCCAGCCTCCTGAGCCCAGGGACCTGCAGATCAGCACCGACCAGGACCACTTCCTGCTGACCTGGAGTGTGGCCCTT
GGGAGTCCCCAGAGCCACTGGTTGTCCCCAGGGGATCTGGAGTTTGAGGTGGTCTACAAGCGGCTTCAGGACTCTTGGGA
GGACGCAGCCATCCTCCTCTCCAACACCTCCCAGGCCACCCTGGGGCCAGAGCACCTCATGCCCAGCAGCACCTACGTGG
CCCGAGTACGGACCCGCCTGGCCCCAGGTTCTCGGCTCTCAGGACGTCCCAGCAAGTGGAGCCCAGAGGTTTGCTGGGAC
TCCCAGCCAGGGGATGAGGCCCAGCCCCAGAACCTGGAGTGCTTCTTTGACGGGGCCGCCGTGCTCAGCTGCTCCTGGGA
GGTGAGGAAGGAGGTGGCCAGCTCGGTCTCCTTTGGCCTATTCTACAAGCCCAGCCCAGATGCAGGGGAGGAAGAGTGCT
CCCCAGTGCTGAGGGAGGGGCTCGGCAGCCTCCACACCAGGCACCACTGCCAGATTCCCGTGCCCGACCCCGCGACCCAC
GGCCAATACATCGTCTCTGTTCAGCCAAGGAGGGCAGAGAAACACATAAAGAGCTCAGTGAACATCCAGATGGCCCCTCC
ATCCCTCAACGTGACCAAGGATGGAGACAGCTACAGCCTGCGCTGGGAAACAATGAAAATGCGATACGAACACATAGACC
ACACATTTGAGATCCAGTACAGGAAAGACACGGCCACGTGGAAGGACAGCAAGACCGAGACCCTCCAGAACGCCCACAGC
ATGGCCCTGCCAGCCCTGGAGCCCTCCACCAGGTACTGGGCCAGGGTGAGGGTCAGGACCTCCCGCACCGGCTACAACGG
GATCTGGAGTGAGTGGAGTGAGGCGCGCTCCTGGGACACCGAGTCGGTGCTGCCTATGTGGGTGCTGGCCCTCATCGTGA
TCTTCCTCACCATCGCTGTGCTCCTGGCCCTCCGCTTCTGTGGCATCTACGGGTACAGGCTGCGCAGAAAGTGGGAGGAG
AAGATCCCCAACCCCAGCAAGAGCCACCTGTTCCAGAACGGGAGCGCAGAGCTTTGGCCCCCAGGCAGCATGTCGGCCTT
CACTAGCGGGAGTCCCCCACACCAGGGGCCGTGGGCAGCCGCTTCCCTGAGCTGGAGGGGGTGTTCCCTGTAGGATTCG
GGGACAGCGAGGTGTCACCTCTCACCATAGAGGACCCCAAGCATGTCTGTGATCCACCATCTGGGCCTGACACGACTCCA
GCTGCCCTCAGATCTACCCACAGAGCAGCCCCCCAGCCCCCAGCCAGGCCCGCCTGCCGCCTCCCACACACCTGAGAAACA
GGCTTCCAGCTTTGACTTCAATGGGCCCTACCTGGGGCCGCCCCACAGCCGCTCCCTACCTGACATCCTGGGCCAGCCGG
AGCCCCCACAGGAGGGTGGGAGCCAGAAGTCCCCACCTCCAGGGTCCCTGGAGTACCTGTGTCTGCCTGCTGGGGGGCAG
GTGCAACTGGTCCCTCTGGCCCAGGCGATGGGACCGGGACAGGCCGTGGAAGTGGAGAGAAGGCCGAGCCAGGGGCTGC
AGGGAGTCCCTCCCTGGAGTCCGGGGGAGGCCCTGCCCCTCCTGCTCTTGGGCCAAGGGTGGGAGGACAGGACCAAAAGG
ACAGCCCTGTGGCTATACCCATGAGCTCTGGGGACACTGAGGACCCTGGAGTGGCCTCTGGTTATGTCTCCTCTGCAGAC
CTGGTATTCACCCCCAAACTCAGGGGCCTCGTCTGTCTCCCTAGTTCCCTCTCTGGGCCTCCCCTCAGACCAGACCCCCAG
CTTATGTCCTGGGCTGGCCAGTGGACCCCCTGGAGCCCCAGGCCCTGTGAAGTCAGGGTTTGAGGGCTATGTGGAGCTCC
CTCCAATTGAGGGCCGGTCCCCCAGGTCACCAAGGAACAATCCTGTCCCCCCTGAGGCCAAAAGCCCTGTCCTGAACCCA
GGGGAACGCCCGGCAGATGTGTCCCCAACATCCCCACAGCCCGAGGGCCTCCTTGTCCTGCAGCAAGTGGGCGACTATTG
CTTCCTCCCCGGCCTGGGGCCCGGCCCTCTCTCGCTCCGGAGTAAACCTTCTTCCCCGGGACCCGGTCCTGAGATCAAGA
ACCTAGACCAGGCTTTTCAAGTCAAGAAGCCCCCAGGCCAGGCTGTGCCCCAGGTGCCCGTCATTCAGCTCTTCAAAGCC
CTGAAGCAGCAGGACTACCTGTCTCTGCCCCCTTGGGAGGTCAACAAGCCTGGGGAGGTGTGTTGAGACCCCCAGGCCTA
GACAGGCAAGGGGATGGAGAGGGCTTGCCTTCCCTCCCGCCTGACCTTCCTCAGTCATTTCTGCAAAGCCAAGGGGCAGC
CTCCTGTCAAGGTAGCTAGAGGCCTGGGAAAGGAGATAGCCTTGCTCCGGCCCCCTTGACCTTCAGCAAATCACTTCTCT
CCCTGCGCTCACACAGACACACACACACACGTACATGCACACATTTTTCCTGTCAGGTTAACTTATTTGTAGGTTCTG
CATTATTAGAACTTTCTAGA
```

HUMAN PROTEIN SEQUENCE : hP5-002.1 (Seq ID No: 36)
```
MVLAQGLLSMALLALCWERSLAGAEETIPLQTLRCYNDYTSHITCRWADTQDAQRLVNVTLIRRVNEDLLEPVSCDLSDD
MPWSACPHPRCVPRRCVIPCQSFVVTDVDYFSFQPDRPLGTRLTVTLTQHVQPPEPRDLQISTDQDHFLLTWSVALGSPQ
SHWLSPGDLEFEVVYKRLQDSWEDAAILLSNTSQATLGPEHLMPSSTYVARVRTRLAPGSRLSGRPSKWSPEVCWDSQPG
DEAQPQNLECFFDGAAVLSCSWEVRKEVASSVSFGLFYKPSPDAGEEECSPVLREGLGSLHTRHHCQIPVPDPATHGQYI
VSVQPRRAEKHIKSSVNIQMAPPSLNVTKDGDSYSLRWETMKMRYEHIDHTFEIQYRKDTATWKDSKTETLQNAHSMALP
ALEPSTRYWARVRVRTSRTGYNGIWSEWSEARSWDTESVLPMWVLALIVIFLTIAVLLALRFCGIYGYRLRRKWEEKIPN
PSKSHLFQNGSAELWPPGSMSAFTSGSPPHQGPWGSRFPELEGVFPVGFGDSEVSPLTIEDPKHVCDPPSGPDTTPAASD
LPTEQPPSPQPGPPAASHTPEKQASSFDFNGPYLGPPHSRSLPDILGQPEPPQEGGSQKSPPPGSLEYLCLPAGGQVQLV
PLAQAMGPGQAVEVERRPSQGAAGSPSLESGGGPAPPALGPRVGGQDQKDSPVAIPMSSGDTEDPGVASGYVSSADLVFT
PNSGASSVSLVPSLGLPSDQTPSLCPGLASGPPGAPGPVKSGFEGYVELPPIEGRSPRSPRNNPVPPEAKSPVLNPGERP
ADVSPTSPQPEGLLVLQQVGDYCFLPGLGPGPLSLRSKPSSPGPGPEIKNLDQAFQVKKPPGQAVPQVPVIQLFKALKQQ
DYLSLPPWEVNKPGEVC*
```

GRES DISCOVERY 5-002

Table 7

MOUSE GENOMIC SEQUENCE : mD5-030 (Seq ID No: 37)
```
GCCAGGTCTGTCTGCCAGTCCAGCTGGCTTAACTTTTAAGGAGAGATAGCAGCAAGCCACCAGTCAGGAGTAGAGTCTGA
CTCTTCTAGGATCTTCCCCGTAATGTGCCTCCAAGTCAATTGTACTACCAGAGCCTCCCTGAACTGTCTCATCTGGCCTT
```

```
GAAATCCCTCTGTATCCCGGACAGGCTTTGAAGTCTCTGTTCTCCTTTGCCTCTGCCTCTCCCCCTTCTTTCTAAGTACC
TGGAACCACAGGCCTGCCGCACCCTGACTCTTGAGAACCCCACATTCACCATTTGTACATTGCTTTATATATAACCAACT
TCCTCCCAGTTCCTTACCTAATTTGTCTCTGAATTCTTTCTGTGACAATGCTGGGACCCTGGGGACAGGGGACTGAGCCC
TCACACCGCTGGGCAGAGCTGTCACACTGCTCCCGAGACTGCTCCACTCTGCATTGTCACTGAGCAAGTGTCCTGCCAGC
ACCAGGCCCTTCTCCTGTCCAACAATTAATGTCCTGTTGGGGCCCACATAAACGGCTGCAACTCTGTGTGTGTGTGCGTG
TGCATGTGCATGTGTGTGTGCGTGTGTGTTTGAGTATGTAACCCTGCCCTGGAACTCCTTATGTAGAGCAGGCTGGCCT
CCAACTCACAGACATTCTCCTGCCTCTACCTCCTGAATGCTGGGACTAAAGGCCTGCGCCACCACACCTAGCTCTTTCTT
TTTTCACATTTATTTCTTTGGGGCGACACATACACACACACACACACACACACACACACACGCACGCACGCACGCA
CGCACGCACGCGCGCACTCGCGCACCTCAGCCTGTGAGTGGAGCTCAGAGGACAACTCTCCAGTGGGTGCTCTCTCCTTC
CACTGTATGGGAAACCAGAATTGAACTTAGGACCTCAGGCTTAGTGGCTCCTTTACCCAATGAGGCACACACACACAC
AGATACATGCAGATACGCACACAGGCACACACAGACATACACAGTCACACACAGACACACTTGCAGATATACACACAG
ATACATGCAGATACATACCCAGACACACAGATACACACAAAGACACAGACATATACATACAGACATAGACAGACACACAC
ACAATCAGATTCACACACAGGCACCCACAGACATACACACAGAGACACACAGAGACACAGACACACATACAGACATATGC
ACACAGACACACACTACCCACCCTCTTTTTTTTCTTCTTTGAGACAGGATCTCGGCAGGCAACTGTAGTTAGCCTTGAA
TTCATGCTAGTCCTCCTGCCTCAGCTTCAGGAGTGCTGAGGTCACAGGGGACACATGTCCTGCCATGTCCCAGTGATATG
GTCATCTGTTACTTTCCACCTTCTGTCATTGCTAGCCCCATGGGCCAGTACTGGATGAATTCAGTCTCTGTACTGGCTAG
TTTTGTGTCAACTTGACACAGCTGGAGTTATCACAGAGAAAGGAATTTCAGTTGAAGAAATGCCTCCATGAGATCCAACT
GTAAGGCATTTTCTCAATTAGTGATCAAGGGGGAAAGACCCCTTGTGGGTGGGACCATCTCTGGGCTGGTAGTCTTGGTT
CTATAAGAGAGCAGGCTGAGCAAGCCAGGTGAAGCAAGCCAGTAAGTAGCACCTCTTCATGGCCTCTGCATCAGCTCCTG
CCTCCAAGTTCCTGTCCTGTGTGAGTTCCTGTCCTGACTTCCTTTGGTGATGAACAGCAATGTAGTCAAGAAAGTGAGCT
CAGTAGATCATGAACTTCTAAAATTCTGACCACCGGGACTTAGGAGCAAAAATGACAGCATTTTGCTGTGGTGTGTATTT
TGATGTTGGACTACACAATTTCTTAATCAGGAATAGAATCCCTGACTAAAACAGTCTCTGAATTTACAACTTAGATCAAC
CCCTAGCACCATAACTGCCCAGACTAGATTGGAAAGATGGGGCTCGGGACAGTGGAGTTCGTCCATCTGAGGGGTGACTT
CCTGCAGCTGGCCAGGCAAGCTGGAGAGAGCTTTATAAAGCCAAGAACCCCAGCCAGACCCTGGCAAATGTCCCCGGGAC
AAGATGAGGCCTGGCAAAGGCTGGGTGCCCCTCCCACCTGGTTGACCCCCCCCGGGGGGGGGGGCTGCTATTTGCCACC
CCAGAGGAGAGTTGGTGTCTTGCAGGGAGAGACTGCTAATGGACTATAGGCCTAGAGGACTCCCCTCAGCCCTCAGGGCC
TGTGGGCAATCCCTGGGGCCTCCGGGCAGCTTGAGGCTCCTCTGCCAGGCTTTTTGAGGCCCCTCCGGCCTGGAAGGGCT
GTGGAAGTTGGCAGGGTTTCTTTCCTCCTCGCTAACTCAGCAGCTGAGGCTTTTTCTTTTTCTTTCTTTCTTTTTTTTT
TCTTTTTTCTTTTTTTCACCCGCCTGGGCCGCTGCTGAACTGGATAAATAAGCAGGAGCGTCCAACGTCAACGAAACAGG
AGCTCGCTCCGGGCTCAGCCGCACAAAAGCCGCTTTGAGCAGCAGCGGGCGCCTCCCGCCCGGGCCGTCGGGGCTCCCCC
CAGCCCAGCCAGGCTGCACTTGGCCTTCATGCTCGCCCCCCCAAACCCCACCTTTTCCCAGAAGCGCCCCCAGCCCTGCT
CGGGATCCCGCCTGCCCCCAGCAGGGTCTGGTTCTTTGTCCGGGTGTTAGAAAACGCTCGCCCCTTTAAAAGCTTTGCCT
GGCCCTCTGTTGCCCTAGGGGGATCCCGGAGTCCTGGCTGGGGGCCTCCGCTTTACCAGACTAATGCGTCCTTGTCCTCG
AGGGCCGATGCTTCTCTCTCTCTCTCTCTCTCTCTCTCTCTCTCTCTCTCTCTATCTCTCTCTCTCTCTTCCTCCT
CTCTTCCTGTCCCCTTGCTTCCTCCCCCTCCTATCCCCCCCGCCTTTCTCTTCCTCTCCCCCCCCCCCTTCCTAGTTTT
TCTTCTTTTTGGAGCTTTCAGACAGAGTCTCATGAAGCCTCGGCTGATCTCAAATTCCCTGTGCAACACAGGATGACTTA
AACTCGTGATCCTCCTGCCTCAGCCTCCCAAGTGCTGGAATTCGTTGTGCACCCACACTGTGCATCTTGAACACCCTTCC
CTTTCCTTCTCCGTGGCTTCCTCTTCGGACTGAGGTCCTGCGGGGCAGGGAGTATCACCTCTCTGCGTTCTCCTCACGAT
CTGGGATCTGGACCGTGATGGTGCCTTACCCCTTACCTCTTCCGCTTCAGTCGCCACAAGTGACGTGAAGACTGTGAACT
GCAGGGGGCGCAAAGGAGTCGTTGCCTGGCACTGAGCAAGTGCTCAGTTAAGTCTGTATCAAGCTCTTGGTTATAGGCTT
TTAATTCCAGCACCTGGGAGGCAGAAAAAAGCAAGGAGATCTCTGAGTTCAGAGCCAGCCTGGTCTATGGAACTAAGAAC
TAGGTGCAGGGCAACCAGGGCTACTCAGAGAAACACTGGGGGGGGGGGGGGAGAGAGAGAAAGAGAAAAGGGGGCAGAGC
GGGAGAGAGAGCAGGGAGAAAGAAAGAAAGAAAGAAAGAAAGAAAGAAAGAAAGAAAGAAAGAAAGAAAGAAAGAAAGAA
AGAAAGAAAGAAAGGAAGGAAGGAAGGAAGGAAAAGAAAGAAAGAAAGAAAGAAAGAAAGAAAGAAAGAAAGAAAGAAAG
AAAGAAAGAAAGAAAGAGAGAAAGGTCGGGTGGTGGTGACTCAGGCCTTTAATCCCAGCAGCACTTGGGA
GGCAGAAGCAGGCAGATTTCTGAGTTTGAGGCCAGCCTGGTCTACAAAGTGAGTTCCAGGACAGCCAGGACTACAGAGAA
ACCCTGTCTCGAAAAAAAAAAAAAAAAAAAAAAAGAAGAAGAAGAGAAAAGAGAGAAGAAAATCAAGCCACTTAGTAGACT
TAGTAGCACAAGTCTTTAATTTTAACAGGTGGATCTCTGTGAGTTTGATGCTAGCCAAGGCTACACAAGGAGACCCTATC
TCCAAACAAACGAATAAGTAAAATAAGTCTTTTTAAAAAATCATATCGTTGGGTCAGCAAGATGGCTCAGTAGGTAAAGT
CACTTACTACCGACCTGACCATCTGGTGGAAGGAGAGAACTGATTCACATACAATAGGTGACTCACATGGGTCCCAATAC
ACAGACAGACAGACAGACAGACTGACTGACTGACTGACTAATTAAATATCAAGGGCTGTAGAGGTAGAAAAG
TCTCATAACCTAAATCCCTATGGTAGACAGAAAGAACTGACCCCTGGAAGCTGGCCTCTGGCCTTCACACATACTCTATA
GTGCCTGTTTGCGCACGCGCAAAAGCACATTTCGTACATGTATGGAAATGTTATATGGACCATCATGTAATTATATATC
ATTGATCCATGCTAATAAGACATAAACAGACAGAGCCAAGTGTGGTGCCATACACCTTTAATGCCAGTACTAAGGATACAG
AGGCAGGCAGATCTCTATGAATTCAAGACCAGCTTGGTCTACATAGTGAGTTCCAGGTCAGCCAGGTTAATAAGTGAGA
CCCTGTGTGGTGGTTTGAATACGTTTGGCCCATAGACAGTGGCACTATTAGGGGGTGTGGCTTTGTTGGAGTAGGCGTGA
CCTTGTTGGAGGAAGTATGTCACTGTGTAGGCGGGCTTTGAAGTCCCAATGCACAAGCTCCACCCAGTGTGAAATTCCAG
AGCCTCCTCTTTGAGTCCCCTTCTGCTACCTGCTGGATCAAGCTGTAGAACTCTGGGCCCCTCCAGCATGATGGCGCCTG
CACACTACCATGCTTCCCACCATGATGATACTGGACTGAAACTGTAAGCCAGCCCCAATTAAATGTTGTCCTTTATAAGA
GTTGAATTGGTCATGGTGTCTCTTCACAGCAATAAAGTCCAAACTAAGACACCCTGTTTCAAAAACAACCAAAACCCAAA
ACCTTAAAAAAAAATTGCCATTTAAGGTATAATACCAATAATGTCCCTCCATATGGTTTACGAGGAGAGGGAGGCCAGCA
```

```
TGGGGGGAGCGGAGAAGGAAAGAAAAGAAAACGAAAAAGAAAAAGTAAGCTGATGGCACCTCTGCATCTCCCAATCCTCT
GGCGTCCAGTCCAGAAGATGTTAATGACAGGCTTCCGCCACCATACCTGGCTTTGGATTTGGCCTCTCATTAACTGTGTA
ACCTAGGCTGACCTTGAACTCATGACAATTCCTCTGCTCCCCCAAATGCTGGGATTACAAGTATGTGCCACCATGACTGG
CTTCTATTTTATTTTGAGATGGGGTCTTACTGTGTAGCCCTGGCTCTTCCGGAATTCACTATGTAGACCAGGCTGGTTTC
AACATACATTGATTTGCCTTCTTCTGCCTCAGAGTGCTGGCGTGTGCCTGCGTGCTCAGAGTGCCTAGATTTTAAGAAAA
TGCTTCTTTCAGAGCATGGTGGTGCACGCCTTTAATCCCAGCACTCAGGGGGCAGAGGCAGGTGGATCTCTGAGAGTTTG
AAGCCAGACTAGTCTACACAGCCAGTTCCAGAAACAGCCAGGACTACACAAGGAAACCCCCATCTTGATAAACTAAAGAA
GAAAGAGAAGGAGGAGAAGGAGGAGGAGGAAGAGGAGAAGGAAAAAGAAAAGAAGAGAAGAGAAGAGAAGAGAAGAGAAG
AGAAGAGAAGAGAAGAGAAGAGAAAAGAAAAGAAAAGAAAAGAAAAAAGCGAAAGAAAAGCTTTTTGGGGCTGAAGATG
GCTCAGCAGCTAAGAGTATGCACTGCTCTCTCAGAGCACCCGAGTTTGCTTCCCAGCACCCATGTCAAACACCTCACAGC
CACCTATAACTCCAGCTCCTGGGTGTCTGACATCTTTTTCTGGCCTCTGAGGTCCCCAGATCTCATGTGCACATACCCCT
CCCTATACAAATACAAACATATAAAAATTTAAAAATTTTAATAATTAAAAAAAAGATGCTTTTTAGGGCTGGCAAGATG
GCTCACTGGGTAAATGTGTGTGCCGCCCAGTTTGATGACCTGAGTTCTAGTCCCAAAGGTGACATAGTGGAGGAGAGAGC
CAACCCCGAGGTTGTCCTTTAACCTTTACATGTGCATTGTAGCCTACACACTGTAGCAGGTATATGGCCCCTCCCCCGAA
TAAATACATATAACAGATTTTAAAATACTTTTAGCATTTCTTTTAGACAAGTTTATTGTGTAGACTGGGCTTACCTCTGC
TGGAATCAAAGGTGCATGCTACACCTTTTTTGCGTATGGTGTGTGTGTGTGTGTGTGTGTGTGTGTGTGTGTGTGTGTAT
GTTGCATTGCATGTAGAGGTCAGTGGACAACCTTGGGTTTGAGTCAGGGCTCTGTTGTTGTCCGTTATCTGTAAAGCAGG
GTAGCTGGCCTGCCGGCTTACAGGGGCTCTCTCCTGTCTCTACGTCCCATTTCCCTGTAGAAGCCTTGGGATTACAGCTA
CCATGCCTGGCTTCAAATGGGTCTGGAGACTTGAACTCGGCTCCTCAAACTCCTCACACTTCCCGGGCAAGTGTATCGCT
CCCTGCGCTGTCTCCCCCAGCCCTCTTTAAGCATTGGTAATGGTCCCTCTCTTTCACACGTTCTTTTAACACAACGGACA
TTTATAGGCGCCTCCTCCAGGCCAGGAGCCAGAACCTTGTAGGGCATGGCACGGTGCCTTCCTCCTGAAGCCCCCAGGCT
TTGTAGGGAAATCAGGCTTTGGAGCAGTTAGCTGTACTCTGCAGTGGGGTGCCCGGTAGGGCATCCTGACAGGCTAATCA
GAGCCGAGGTGGGGCCTGGGGGAAGGATTCCGGTTTATCCAAGAATGAGGGGAGCTTACAATGCAAGAGAGGCCTAGGGG
GAAGGTTGGCTTGGTGAGGGTGGGAACTCAGGACAGAAGGCTGTCCCTTCCCACAATTGGCCTCTAGCCAAGGCCCCTAG
AAAGGCTGGCATCTTCCAAGGAGGTGGAGCTTCGAGGGGCTCCCAGTGGGGGTGGGGCAGTGATGGTGACCTCCTTCGGT
CTCTGCCCCTTCGCATCGTCTCCCTTTCAGTGTGTGCCTGAGGTATTGTTCCTCTTTTGTAAAGAGGGGGATCGGGTAAC
AAAGCCGGGTGGGTGGGCAGGCAGCCCAAGGGGCCTCTTCTCTTCCCTCCCCCTGAAGCATCTGCACTGAGGGGTAGGCA
CACATACACCTCTGGCTTCCCAGGAGCTGGCTGTTGCCATAGTGACAGGCAGGCCTTGCATCCCACTGGCCCCTGGAGTT
GCCCTTCCCACTATGGCTGCCAAAGTTAGGGGCCCCCAACTCTTTTGCATCCTGGAGTGGGAGGCAGGGACATACAAAAT
AGAACTCCTGGCTAGTCTCTGCCTCCTAGCTGGCTTTGAGGAAAGGGACAACTTTCCTTAAAGAGAGCTCCAGCCACTGG
GACAGATGCTCAGGAAGCAAGGCTAAGCCTTGAGATCAGAAATAAGTGCTCCCTCACCCGGGTGTCTGGTGTGGCTGTTA
GTGTTCTGCAAAAAGGAAGGGAAAGATAGCAGAAGGCTTATAGGATAGGTAAGGACACTGGGGGACCCAAAGCAGAGCCT
GCCCCTGAGTCTACTATGATCGATGGAATAAGAGAAAGGTCTTGGGGTATTTGAAAAAAAAAAAAAAAAAAAAAAACCTTA
AAGAATGGAGAAGTGAGGACCTAGGTTCATAAGGTGGGGGTTGGGGGGGGGTTGAGGTAGAACCCTCAGAGATCTTCTTG
TCTCCTCTCCAGGAGTGTGATCAACAGGTGTGTATCACGCCACCTGGCTTCGTTTGTGGGTGCTGGGGATCCGAACTCAC
GACCTCATGTTTGCCCAAAAAGCACCTATCTGCCTAAGCCGTCTCCCCAGCCCCGAGTTTAGGATCGCGAAGCAAGACCC
AACCTGGGGACTCGCAGGGGCTCAGTTTGGCGTGCAGGGCTGGCTGCATCTTCACAAAGCCTCTTCTCTCCAGTCTTTTC
GGGGGGGGGGGGGCTGGGTGCATGCCAGTGACACATATGTTTTTCTTTGTCCCTGCCGAGCTGCAGCTTTCTGGGAGCCAG
AGAGACTAGGAAATGACCCACCCCATTTTCTCACTGCGGCCATCAGCGCTGCCCTGGCTTCTTCCTGAATCCCGGCTCTAA
ATATAGCAGGAGGGTATTGGCCAAGCCGTGTATGTGTGTGTGTGTGTGTGTGTGTGTGTGTGTGTGTGTGTGTGTGTGTG
TGTGTGTGTGTGTGTGTGTGTGTGTGTGTGTGTGTGTGTACGCGTGTGCGCGCGCATGTAACAGGCAACTTTTAGCAGGGAAACAATGTG
CCTGCCAGTCCTGACACCGAGGAGGTGTGTGAGCCTGAGTGTGAAATACCTGGCTCAGGCACTTGGGAGGTGGAAGTAGGAG
TTTCCTGTAGGGCCACAGTAGAGGCTGAAATCATCACTGTGAAATACCTGGCTCAGGCACTTGGGAGGTGGAAGTAGGAG
GGTGAGAAGTCCTTTGATACATAAGGAATGTGAGGCCAGCTTGGACAACATGAAATAGTCTGGAAAACAAACAAATGGGG
AGTTTGAGAAATGGATCAGGAATTGAGATCACTGGTTGCTCCTCCAGAGGACCTAGGTTCGATTCCCAGCACCCACATGG
AGGTTCACAACTCCAGTTCCAAAGCCTTCTTAGGGCCTCCAAGGGTACCAGGCACACGTGGTGCACATACAGATAGAC
AAGCAAAGCACATTAATAAAGAAAATTTAAAAATTTAAAGCAAAGCAAGGCAACAACAAAACAGACAAGTGGAGGAGCCGG
AGAGATGGCTCAGTATAAAGGTAGTCACCCCATCCCAGTCATGATGACCCGAGTCAGGGGGCAGGTATCCCGTGATAGAA
GAAGAGTACTGACTCCCCAAGTTGTCCTTGCTTGCATGCAGTTTCGTGCGTGTCACACACACACACACACGCACACACAC
ACACATGCACACACACGCACACACAGACAGACAACGGCAGGATGTGTGTGTGTGTGATAAATATAATGAAAACAAAACA
AAATCAGCTTGGGCTGGGCACGTGGCTTGTCTAGCCTGGGTTCCATCCCTAGAATGAACAGCAGAAAAAACTCATGTGGG
GGGCTGGCAAGATGGCTCAGCGGGTAAGAGCACTGACTGCTCTTCCAAAGGTCCTGAGTTCAAATCCCAGCAACCACATG
GTGGCTCACAACCATCCATAATAAGATCTGATGCCTTCTTCAGGAATGTCTGAAGACAGCTACAGTGAGTGTACTTACAT
ATAATAAATTAATAAAAAAAAAACCAAAAAAACAAAAAAACAAAAAACAACCTCATGTGGTGGCAGCCCTTATGAGGCAG
GAGGATCAGGAATTCAAGGTGAGCTTGAGGCCAGCCTGGGTTATATGAGACCCTGTCTCAAAAACAACAGGATCAACAAA
AACACCCTTAAGGGAAGAAGAGAGGAGAGGGGGAACAAGCAACTCCCTAGCCTGGAGCTTGGAGCTTGGGATGGTCCC
CTCCCACCTGGGAAGAAAAGGGCGCACTTGATGCGAGGGAGTACATTGGGAGGTGCGTTCCCGCGGACTGTCGAGTCTGC
AAAGGAAGGGAAAGAAATGAGCAGAAGAGAAGAAAAAGCTGACCCACAACGTTGCCCACACGCCTCCAGCGTGGCAGAGAG
GCGCGGCACCTTTAAAGCGCGACGGGGGCGTGCCCTGAGGGACTGCCCCGCCCCTGGGCGTGTCCTCCTACTGACCACGC
CCTCGGGGTGCCGAGCCGCGGCGGCTGGCGGCGGAGACCACGCCGGCTCTGGCTGGAGTGGCGGCGGCATCGGCGGCGGC
GGCGACGGCGGTGGTAGCGGTTGTTCCTCCGAAGGCGCCACGCGCTCCGGGACACACGTGGGCGGCCGAGAGATGGCGCG
```

```
GGAGCCGCGACGTGGCGCGCAGATCCCGGGACGCGGGATGGAGCGGTGCGAGTGAGCCCGAGCGGCCGCGCCGCGGGGCG
ATGGCCGTGCCACCGCTGCTCCGCGGGGCGCTGCTGCTGTGGCAGCTGCTGGCGACGGGCGGCGCGGCGCTGGAGATCGG
CCGCTTCGATCCGGAGCGTGGCCGTGGCCCCGCACCGTGCCAAGCGATGGAGATCCCCATGTGCCGGGGCATTGGCTACA
ACCTGACCCGCATGCCCAACCTACTGGGCCACACGTCGCAGGGCGAGGCGGCGGCGCAGCTGGCCGAGTTCTCGCCCCTC
GTGCAGTACGGCTGCCACAGCCACCTGCGCTTCTTCCTCTGCTCGCTCTACGCCCCAATGTGCACCGACCAGGTCTCCAC
TCCCATCCCAGCCTGCCGGCCCATGTGCGAGCAGGCTCGCCTGCGCTGCGCCCCCATCATGGAGCAATTCAATTTCGGCT
GGCCCGACTCACTCGACTGTGCCCGGCTCCCCACGCGCAACGACCCGCACGCACTCTGTATGGAGGCACCCGAGAACGCT
ACAGCAGGCCCCACAGAACCCCACAAGGGCCTGGGCATGCTCCCTGTGGCACCTCGGCCCGCGAGGCCACCGGGAGATTC
AGCCCCAGGTCCCGGCAGCGGTGGCACCTGCGACAACCCCGAGAAGTTCCAGTACGTGGAGAAGAGTCGCTCGTGCGCTC
CGCGCTGCGGGCCAGGCGTCGAGGTGTTCTGGTCTCGGCGCGATAAGGACTTCGCGCTGGTTTGGATGGCTGTGTGGTCC
GCGTTGTGTTTCTTCTCCACGGCCTTCACCGTGTTCACCTTCCTGCTGGAGCCTCACCGGTTCCAGTACCCAGAGCGCCC
GATTATCTTCCTTTCTATGTGCTACAATGTCTACTCCTTGGCCTTCCTGATCAGAGCGGTGGCAGGTGCACAGAGTGTGG
CATGCGACCAGGAGGCAGGGGCTCTGTATGTGATCCAGGAGGGTCTGGAAAACACAGGCTGCACCCTGGTCTTCCTGTTG
CTCTATTATTTCGGGATGGCCAGCTCACTTTGGTGGGTGGTTTTGACTCTAACCTGGTTCCTGGCTGCAGGCAAAAAATG
GGGCCACGAGGCCATCGAGGCTCACGGCAGCTACTTCCACATGGCAGCGTGGGGCCTGCCAGCACTCAAAACTATCGTGG
TCCTGACTCTGCGCAAGGTGGCTGGCGATGAACTGACTGGGCTCTGCTATGTAGCCAGCATGGACCCGGCAGCCCTCACT
GGCTTTGTGTTGGTACCCCTATCTTGCTACCTGGTACTGGGTACCAGTTTCCTCCTGACCGGTTTTGTGGCTCTCTTCCA
TATCCGCAAATCATGAAGACGGGAGGCACCAATACGGAGAAGCTGGAGAAGCTGATGGTCAAAATCGGAGTCTTTTCCA
TCCTTTACACAGTGCCGGCCACCTGCGTCATTGTCTGCTACGTTTATGAACGCCTCAACATGGACTTCTGGCGGCTTCGG
GCCACAGAGCAACCATGTACTGCTGCCACCGTGCCTGGAGGCCGGAGAGACTGCTCGCTGCCAGGGGCTCGGTGCCCAC
TGTGGCTGTCTTCATGCTCAAAATCTTCATGTCCTTGGTGGTGGGCATCACCAGTGGAGTCTGGGTATGGAGTTCCAAGA
CTTTTCAGACTTGGCAGAGCCTGTGCTACCGAAAAATGGCAGCTGGCCGAGCCCGGGCCAAGGCCTGCCGAACCCCAGGG
GGCTATGGCCGGGGTACCCACTGCCACTACAAAGCCCCCACGGTGGTCTTGCACATGACTAAGACAGACCCCTCTCTGGA
GAACCCCACACACCTCTAGAACATAGGCTAGGCTGTGAGTTATGGTTGCTCCCTCCTTGCCCTCCCCTCCCCCTTCAGA
GACAGCTGACTAACAGCTGCCCAGCTGTCAAGGTCAGACAAGTGAGACACAGGGGGCTGAGGACTAGGGTGGGGACCCAG
TAAAGCTCAGGGCCTTGACCTTCTGTCTCATGCAGGGAGTGGTCCTAGTCCACAGAGGGTCCCAGGATAAGAAGGGGCAG
AAGGGGGCAGGGTCCAGTGCAGAGTTATTTAATGATGTAATTTATTGTGGCATTTCTCTGGAAGCTGTGACTGGAATAAA
CCCTCGTGTGGCACTGCTGTGTTCTATCTGGGCTGGAAAGAGGGAAGACAAGACAGGAGGGTGAGAAACTTCATAAGCAG
AGCTGGGGGTGGGGGGCGGAGGCCATGAGATTCATCCTCTGACCCCTTGTCCAGGAGGAAGTTCCTCTGTGGGGGCATCG
GTGGATTATAGGAGTCCTAGACTGCTGTGGGTGTGTGCGTGTGCGTGGTGTGTGTTTCTCTTCTCACCTGTCTCAAGTCT
GAGATCTGTGCTTCTGAAGTGCAACAAGAACCTCCTCATGAATACCCAGGAACCCCTGGCTGTGACATGGCTCCTTCTAG
TCCTAACAGTGTCATCTAGCCTGTTTAGAGAAACAGGTAAGCTCCCCGCCCCTACCCACCCATACTTTGCTTTAGCAAA
TAAGGTCCGGGCTGGCAAGAGAGCTCAGTGGAGACAGGTGCTTGCCGCTAAAACTGAGGACTTGCTTTCAATCGCAGGAA
CTCACATAGGGCTGGGGACCAACTCACAGACTGTTTTTACCCCTACCTGTGCCCTGTGGCACAACTCCTACTATACAAAT
ACATACATGTAGTAAGATGCTTTTTATAAAGAAAGAGAGGGGGAGGGGAGGGGGAGGAGGGGAGACTGTTTCTAAGGCATCCTTT
CACCCAAGTCTGGAGGATGGTCCTCATTATCTATGAAGGTTGCTGAGAGCTGGGATCCAAGCTCAGTTGGCAGTGTCCTT
GCCTAACGTGCATGAAGCCCTGGGTTCTGTCTCCAGCACGGCTTAAAAGAGGCATAGTGGAGCATGTTTGTAATTCAAAT
ATTTGGGAGATAGAAGTAGGAAGATCAGGAATTTAACATAGGTATCCCCTGGCTACCTGAAACTCTTTGGATAAAAATAA
AAATAAAAAAAGAGAGTGGGCAGGCCAAATGGCTCAGCAGGTAAAGTTGTTTACTATGGAGTCTAATCTGAGTTCAGTCC
TTTGAACCCACTAGGTACGAGAACCAACACCCACAAATCCCTGACCCCCACTAAATTGATGTAAAAATATTTTTTAAAGA
CTTGCTCAGCAAGTTAGAAGCCTTGTCTTCAAAAAACAAAAACAGCATTAACATCAACAATAAAACCCTTCACTAACAAC
AAAACAGAATATTCGGTAGTTGGAGCCTCAAGCTCTCTCTTTGTATTTTGGGATGGAGGGGGAAAGATGGGCAGGTCTAT
CTATGTCTTGGTACCAGTCTGGGAGGTAGGGAGCTGTTGAATTCATTGACTATTGTGTCCCTTCTCACCCACCCCAAACA
TCAGAACTTGGGGCACTGACAGCAGCTTCGAGAATAAGGCAGGACCACAGGGATGTGCACAGCTGTGCTGGGACAGTTGA
CAGTGTGTGGGAAGCTGGGGAAACACTATGTGTACCTGTGTGCTAGGGAAGCACAGGTTACTGGGACATGTCCCATCCCT
GTCCCCGTGAGGGACGTGTGGTCATCCCGGAGGTCTGTATCAGCTGAAATTGTATCTAGACTGTTGATGCCAATCAGGCC
GAAGTTGCCTGTGTGTGCGTGTGTGTATGATTGCGTGAATATATGCATACATGGGCCAGGGAGAACGGCAGGTGTCCTCA
TCGGCTCACTCATCCACACCCAGCCTTTTCTGGGGATGCTGGGGTTTCATCTCAGGTCCTTCCGTTTTCACAGCATGTGC
TCCTGCCCACTGTCCTAGCCACAGGCAAAGATTGTGGTCAGTGGCCCTGGATGTATTCTACACATAACCAGCATTTATGA
GTGAAGTCCCTCAAAGTTATTTTTTTTTATATTTTGAGACAGGGTCTCTCTATATAGCCCTGGCTGTCCTGGAACTCAAT
AGCCTCAGACTCAGAGAATATTTGCATCTGAGTACTGTGATTAAAGGCACTCACCACGTTTGGCTAAGAAAGTTTTTCAA
AAACTGTTTTATGAAACCTCAGCCTTCATTTCTTGTTTTTTGTTATCCTGTTGAAATCAGAGGTAGGAAATGCAGGTAGT
TATCTCTGGGAATAACGTGTGTATGTAACTGAACTATGATATGGACTAGATAGATACTTGGTGCACAGAACACTGCTTTT
GCTTTGCTGGGGCTGGAACCCAGGGCCAGAAGTGTGATAGCGACAGGCGGTCTACCACTGAGCCACACCTTGGACTTTCC
TAAGAAGTTCTTTATAAAGACAACACCCTCATTCTCTGCTTTGTATGGAGCATTCATTCATCCTATATCTGACCCACAAA
GCCAGCAACCTTGAGTCTCATACAAGGATTCTCTTGCATGAGAAAGCTTTTATGGCCCACACTGAGTGAGAAGGTAGTGG
GTACACACGACTTGCTCAAGGGCCTGTAGTACGACTTTGGCAGCCAGATAAAGGGAAAGGCAGGGAGCGGGCTGTGCTGA
GATGTGCATTTATTTAATGGCAACCAGTACACCTTCTCTTTTCCCTTGCCAGTCAGGAGGTAAGGAAGGGATCAGTACTT
GGCTGTCTGTGCAATAGAAGCAAGACTGGTGTTAGCTTCTAGAGAAACTAGGAGGAAGCAGAGATGAGCAGCGAAGGACA
GACGTCAGGACTCATCAGGGCATACCTTAGGAGGGCAGACAGGCCATGCTGGCAAAGGCTCCAGGTGCCTAGGTCCTGCT
CTGTTTTATAGCAGGGATGCATTTGGAACAAGCTATGGCCTCTCATGGGGTAGGTAGGGTGTATGCTCTAGGCAGGTGTT
```

```
ATAAAATTCACCGTATTATTCATGCTTGAAATACCCGGCTCTCAGGGCTTTACTGCCCAGCACTGCCCCAAGAGAAAAAA
GGAAACATCAAAGCTGTCTCTTCTTTATAAACACAAGTACATAATGTTTATTTGAAATTCCAATTTATTACAAGTCTACC
CTTTACCGTGGCCCTTCCTCCTTTAAGACAACTTAAAAATCCACCATGCAGACAGATGTCACTGCAGTGGGAACACCAGC
TTTCTGCTACAACACCAACATCTAGGGTAGGAAGCAGGGAGGCAGCAGTGCCAGGGATCACAGCGTGAGTGTTAGCGCTA
ACCTCAACCAAGCCTGCAGGAGGGAACGGAAAGGAAGCCTTCCTCAGCCACTGGACACCCAGAGCCACAGCAAGGCTATC
CCTCAGCCCTGGGAATGGTGAGCTTCCGGACCTGGTGGACTTCTTTTCCTTTAAACAAGAAGAGCTGAGGGACTCACATT
TTTCAAATTCAAATCGATGGAAAAGAAAATTAAACCCTGTCTTGTACTTTTATCAAAATCTGTCATAAAAGGGAACGCA
GACTACAAACTTTTGCCTAAATACAACACGAGGCTAGACTGGCTACAGAGGGCAGAGCTGGCCCTCCCCACCCCCTCCAG
ATGCTGACACTCCGCTAGAAGCCCTCGGAAGGAGCTCTAGGTCTGCAGAGTGGGCCCTGGAGGAACAGGCACCCACAGGT
AGGTAGAGTGCCGCCAGGGCAGAGATGGTGCCAACCCGAGATCCTGCTGCCCGCTGGCCTCTTTGAGGGTAGTAAAGCGA
ACACATATCATAGAAACTGTACTGTACATGTGCCAAAGCAAGATGACAAGTATTTTACAAGATGTTCTTTATACAATATC
ACTGCGGAAACAAGCAACTTTAATAACTACAAAAGTCAATTTAAAAAATTAAACATTTTAACTTCTTCCTGCTCTTTTTC
TGGCTCCCTAAGGGCTTGTCTGGTTTGTGGGTGGGCTGGGCTCCAACACCCCTCTGGCCAGAGCCATTAAAGCCTAGGTC
ATAGCATGATTGGAAGCAGGACCCCAGTCTATGGGAAGTGCCTTAGTTTGCTTTGTCTTTTATGAGGCAGTGAGCTGGGT
GAGGGCGGGGAAGGGCTGCACAATGGAGAGTCCTCAGGAACAGCTCCAGAAAATGTCCCTCTACTCTGCCACGATCAACT
CAGAGCCCACACTGTCCCAAGATGCTGGTGAACACGGAATTCTGTATGGCACTCGCTGATACTGTCACCTGAGAGGGAGG
AGGAGGAAGAGACAGTGCGGACGTAAAGAAATAGGACTGTGTATAAATATAAATTCAAAAGATCCACACCTCACCCAGCG
TGGCCAGATGACGCTCCTTCCTACTGTGGGGTACAGCACCCTCTTTGAGGCCAGGGGGCTTGGCACTGCTGTAATGAGGC
AGGAGGAGGAGGGAGAGGAGGAGAGCAGGGCAGAGCTCACAGAATCCATGCCTAGGCTTCTCCAACTCTCAGGGGGAGAC
CTACTTCTGCTGCGAGCTTTTCTGGGCAGGGCTCATCAGGCTAGCATGATGCTCCCCAAGAGCTTGCCTTTGCTGGAAAA
AGGCCCCATGGGAACTGCAGGAGAAGCCCAAGCTTGGCATGACAGGCATTTCTGGATGCTCCAGTTACTTCTCCTTTAA
ATCCTCCCAGCTTTCCTGCCTCCCCCACACCCCTTGAGGGCTTCTCCTGTCCCCTCCTCAGCTCCCTCACTAGGGCCCT
GCCTCTTTACTTCTTCTGTCTCCGTCCCCTGGACTGTCCAACAGACTCTGAGTCACTGTCCCCTTCATCATCAGCGAGCC
TATCAGGAAACTTCCTGCGTTTCCTGCGCACGTAGGGGTGGCCGGGGAGGTGTTTTTGCAGCAGAGCCAGCAGACACTGC
TCTGTCTTCTCCATGCAACTCAGCACGTGGCTGCCGCGGCAGTTATAGAGCTCGGCATTGGCAAACACTTGCTTCATGTC
AGTGAGGAACTCCTGCACAGATCGGTAGTTCCCGCATGAGCATTTGTTCTGTATGGTCTGGAAGTCCATGGGGTGCTCGA
TCACATCATAGTAGTCCTCAGCCTCGTCTCTGGTCACAGGCTCCCTGTAGGAAAACACCAACTGAGAAACACACCCAGGC
CAGAGCAGCACTAGCCCAGGATGCATGCAGCCATGGGTCAACATGAGCCAGGAGACAAGATGCTCCCTCCCTTTCTAGGG
GCTGTGACTCCCCATCCTCTTGCCAGGACCTGTCTCCAAAAGAAGGGGCCAGAGTAGCCAAGAGGCAGGGCACTCACCGG
AAGGGCCAGCTGAAGCGGTACTTCACCAGTTTGTGAAGGATATCCTCACACTTTTGTAGCTCCAGACTCTGCCTTCTCGA
GATACGCTTGGTCTGAAGTACCTAGCACCAGAGGAAGCAGAGCGCCAGTCATTATAGGACTTAAAAAGTAGAAGACTAGG
TAGACGGTGGTGGTGCACGCCTTTAATCCCAGCACTTGGGAGGCAGAGGCAGGCAGATTTCTGAGTTCCAGGCCAGCCTG
GTCTACAGAGTGAGTTCCAGGACAGCCAGGGCTACACAGAGAAACCCTGTCACAAAACAACAACAAAAAAAAAAAAAAG
TAGAAGACTAGACAGAACACACACAGATGCACACATACACAATAAAAAAGTAATAATAATAAAAACTAAAAAAAATTCC
CTATCTATTAAAACACAAAGGAAGACAGTTCATCACTTCCTTGGGCATCATGGTAGGGTGATGCTCAAGGCAGGTGGTTC
TCTCTAGTCAGAATGGTTAAAGCAGCAGAGGGGACGCAGCAGGTGCTCATCAACAGAGAGTGGTGCCAGGAGATGGCTAG
CCCTGTGAGATGGATCCAGGACTATGGACATAAACAAGCAATGGCAGAAAGCAGCTGCAGACCCCAGCAACCAGCCTTGT
GGAACACATATGCATGTTTAGGAAGAAAAGTAAGAGTGTAGCACCAGTCCTTGCTGGCTGCAGGAGGAGGTGGTTACAGA
GATGGGGGTTGGGCACCCTTACAGGCAGCCACAATCGGGTGGAACCCCAGCAGTAGGAACCCCACACAAGGCACTGGCAA
CTTTAAAGACTGGTCTCTCCCCAGTGGCTTCTTTCATGTCTCCAAGCCCAAACTAGACTGTAAAGATAAGCCAGCTCCTG
AGAGCTTCCTTCTGTTCAAGCTCAAGCCAGACCCAAACTACTGAAGTGATGCCGCGATCAACAGAGGAGCAAGGCAGTGT
CCTAACAACTGTGGCTAAAGCAGCAGTTAGTGGCTCTGGCATTGCTGGCATTCCCACAGGGAGGGCCCACCCCTCTGGGCC
CACCAGAGACACATGGATACTATTTTAAAGTCTCTACTGAGCTGCAGGCTGCTGCTAACTAGGGGGACTGTCTGCAGGC
TAGGCAGGATGCCGGGCATAGCTGCTCCTAGAGGGCCTGCTGCCTAGTCTGCCCAGAGCCTATCCATCTGCTGCCTCAGC
CCCCAACCCAAGGGGAGGGGGAAGGGAGGGGGAGTCTCCCACCTGTTGCCTTGTGAATTCCCCATTGTGGACTAGAACATA
AAGAATACAAAGACTCTAAATACCTAATAAAAAAATGGAAAAAAAAAAAAAAGAATACAAAGACTAGAGGAGGCTAAGTAA
CTCTTACCAGATCATCGACCTCTGGGTCATCCTTTGGCCGTGACCTTCTGGCAGGATGTGACTTCTTACCCGGCGGTCGG
CCTGGTCTTGCTGCAGGGATGACACTCTGCTTGCCTCGAATGGTCTTCCGAGGTCTCACTAAATGAAACGTGGGCTCAG
AGCACAGCAAAGGGGTGGGTGAGGCATGGTGTGTGTATGACAGGGGAGGGGCGAGAACACCAGAGCAAGCGGGCAGTGAC
TCTCTGAGGCTGCAGGAGGCCAGGTTCCAGCACTTCTAGCGTGTCCCCATTTGGTTCAGCTATATTCAGGACAAGGTCTT
TGCAGGAGTCAACTGTCAAGTAACTGAGGAGGAAACAAGTTTGCCAACAGGGCAGGATGGCTCTCCTACAGCCGAGACTT
AGCCCAAGAGAATGCACACACTTTCATGTACAAGGGAATCTGACATTGCCTCGTCATTTCCAGTTACCCTATTCCTTTTA
CCCAGTGTTTCCAGGGGAAAGCTCACTCACACAATGGGCCACAACATTCTGCCAATCCAAGTCAGGACCCACTTTCTCAT
GTTTCTGAAAACCAAGATGGTAGGTGAGGACTCTTCCCTAAGAGCCCTGCCTGGAATCTCTGAAGAGAATAAACTTGAAG
GCACGTAGACTACTCATGTCACTGGTTGGCGACAGGTGCTTAGGACAGGCAAACCAACATTCCTTGGGATGCCCTTGGGA
ACATTCTCTGCAAGGATTAGCTCAAAGCTGCTTCTCCACTACTCCATTTTCCGAGGCCTTTCCTCACTAATACCCTGAGT
GGGGGGAGATAACATTTGGGCAGAGAATGTGTTCAGAGAAGCAGCCTTGTGGAGAACCAATGAACCATGCTTCCCATTCT
ATTTCTGGACCATGCTAACCCTTCAGTGGCTATTTTAAGGCTTCCAGCTCCAGACAAGTTTCATGAGGAAGAGCTGAGGC
TCAGGTCCTAGGCCTGAAATTGCACAAGGGAACCAACATTGATGGCCTGAAAAGCTGAAGGCACCAAGGGCTCTGGAGTA
CCAAATATTTCCTAGATTCTTGGGTGATAACCCTAGGAGAACAGCTGCCTCTGTGGAGAAGAGGTGGGAAGCAGTCACTA
GCAAGAAAATAAACACAATCAGCACACTGCTGCATCCAGCTCTCACAGATAGTGTGCGTCCACACTACAGATCATTATGA
```

GGAGGCTCTTCATGGCAATGGCTGACCCTGAACTGCTGAAGTTGTAAGTGTGAACTAACAGTCTAGTGTGGTGGCATGTG
TCTGCAATCCCACCAATGGAGGGAGGTGTTCCAGGACAGCTTGGAAATCTACCTTTAATCAGAGCATTTAGAAGGCCTAG
ACAGGCAGCTATCTATAAAATTTGAGGCCAGCCCAAACTACACTGCAAGTTCCAGGAAAGCCATGGATACATGGTGAGTT
CCATTCTCAAAACAGAGCCTGAGATGGGAAAGATGATCATTCAATTGCTGTGCAATATGAGGGAGGAAAGTAAATTAGGA
CAGGCACGGAAGCCAGAACACATGGGATACACCACCATAGTAGGAAATGGCCACACTAAACACCCGCTTTCTTATCACAG
CATGGATAATGCTTCAGAGCTGCGAGGCCTGTGTTCTGTTTTCTACTGGCCTATGAAGAGGATACCACTGAAAGGATGGC
TGGAGCCACTGGCTTCATCAAGGACAACATGGGCATTCTCAGCACCCCTAGGACTGAACCACAGGAAACCAGCCTTCCT
TTTCCAAACATCGAAAGCTGGAGATGTGCATACTTTGGGAGCATTAATCAGCATGTGATGAGACAATCAGAAATAACTGA
AACCACCCTGCACCATAGGCCAAAGACCTCTCTGGCCACTGTCACTATGCATAGATGGGGCCAGACCAGAAATCTTGTCA
TAAACATGCTTGAATCCCAACTAAATCTTAGAGCAAGTGCTCTGCTGTGGGCTGATGCTCTGGGAACCACTCTACCTTCT
CCCTGACAGCTTAAAAATAGATTTTATTTTTAACCATGTTTCTGTGAGGACCAGTGTATACGTGTACATATGTGCACATG
AATGAAGGAAGGTTCCAGATAAGAGTCTAGGTAAGGGTCCTAAATCCCTTAATTTGGAGTTGCAGGCAGTTGTGAGCTGC
CTAACATGGACACTGGAAAGTGAACATTGATGATATGGTAGAGCAGTATAGATTCTTAAGTGGCGATCCACCTCTCCACC
CTGACCATCTTGAAAGCCAATAGAAACACACGGCAGCTCATTTACCCACAGCCCCAAGAGAGAAGAGTAAGACAGCAAAA
TGGCAAGGCTGAATTTCTCTTGCCAGCCAGGCCTCTCTGGTCAGTTTGAGAGACCGAGGCAACTATGCCATACTAGACAC
TCACGCCGCAAGCCAGCCACTTCATAATCCTCTTCTTCTTCCTCCTCCTCCTCTTCTTCCTCTTCCTCTTCACCGCTCTC
ATCACCCTCGCTGCCCTCAGAGGTAGACTCTTCAGTGTAATTCCTGGAAGGAAAAGCCAAGCAGAGACATGGTTTCAGTT
CAGTCCAGGACGGCTTACGGAGAGCTAAAGCCCCTTACATCTCTGCAGCATGGCTATTCATGGACCAGACTGAGCTGACA
GTAAGGGGAGAGACCTGGAGGTCAGTCCTACTATGCTTCAGCAGCATGTGCCCTGCCCTAGCCTACAAGGGCTTAAGGGA
CATCAATGGAGGAAGGGGACCAAGAACAAAATACAACCTCTACTTCCTCTCACAAACAGTATAAACCACCATTACTTTTG
TCTCACAAAGCCAGATAAGGAAACATTTTAGACACCACAGCTGCCCGACTCTGGGGTGTATTGAGAGGGCTGAGAGGCT
GGCAGGTGTTACTGCACACCTTGATGGCTCTCTTCCAGGAGCTGAGACAGCTTGCAGATGACCACACAACAAAGCAACTA
ATCCCGGCTCAGAACAGCTGGTCTAAAAAAACACTACTTCAGAGCAGCACACCGCATGTCCCTACAAGAGGACATGGTAA
TGGAGACAGAGGGAGAACTAACCCAGCACGGCTGCTAAGGTGTGGTTGGAATCAAACAGCTCTGCCCTTTACAAGAGACA
GCCTCGTGCCCGTAAGGATGCCCTTTCACATCTCAAATGTCAATGTAAGAAGTAACGACAGTTAACTGGGGAAATATTAG
GTGCCTTCAAGGTGCTGGTTTGGCTGTACTGACATAGGAGAAACCATGTCCAAATAGACAGCAGGGAAGGAAACTAAGCA
TGGGTCGAGAAGGCAATGA

MOUSE mRNA SEQUENCE : mR5-030.1 (Seq ID No: 38)
ATGGCCGTGCCACCGCTGCTCCGCGGGGCGCTGCTGCTGTGGCAGCTGCTGGCGACGGGCGGCGCGGCGCTGGAGATCGG
CCGCTTCGATCCGGAGCGTGGCCGTGGCCCCGCACCGTGCCAAGCGATGGAGATCCCCATGTGCCGGGGCATTGGCTACA
ACCTGACCCGCATGCCCAACCTACTGGGCCACACGTCGCAGGGCGAGGCGGCGGCGCAGCTGGCCGAGTTCTCGCCCCTC
GTGCAGTACGGCTGCCACAGCCACCTGCGCTTCTTCCTCTGCTCGCTCTACGCCCCAATGTGCACCGACCAGGTCTCCAC
TCCCATCCCAGCCTGCCGGCCCATGTGCGAGCAGGCTCGCCTGCGCTGCGCCCCCATCATGGAGCAATTCAATTTCGGCT
GGCCCGACTCACTCGACTGTGCCCGGCTCCCCACGCGCAACGACCCGCACGCACTCTGTATGGAGGCACCCGAGAACGCT
ACAGCAGGCCCCACAGAACCCCACAAGGGCCTGGGCATGCTCCCTGTGGCACCTCGGCCCGCGAGGCCACCGGGAGATTC
AGCCCCAGGTCCCGGCAGCGGTGGCACCTGCGACAACCCCGAGAAGTTCCAGTACGTGGAGAAGAGTCGCTCGTGCGCTC
CGCGCTGCGGGCCCAGGCGTCGAGGTGTTCTGGTCTCGGCGCGATAAGGACTTCGCGCTGGTTTGGATGGCTGTGTGGTCC
GCGTTGTGTTTCTTCTCCACGGCCTTCACCGTGTTCACCTTCCTGCTGGAGCCTCACCGGTTCCAGTACCCAGAGCGCCC
GATTATCTTCCTTTCTATGTGCTACAATGTCTACTCCTTGGCCTTCCTGATCAGAGCGGTGGCAGGTGCACAGAGTGTGG
CATGCGACCAGGAGGCAGGGGCTCTGTATGTGATCCAGGAGGGTCTGGAAAACACAGGCTGCACCCTGGTCTTCCTGTTG
CTCTATTATTTCGGGATGGCCAGCTCACTTTGGTGGGTGGTTTTGACTCTAACCTGGTTCCTGGCTGCAGGCAAAAAATG
GGGCCACGAGGCCATCGAGGCTCACGGCAGCTACTTCCACATGGCAGCGTGGGGCCTGCCAGCACTCAAAACTATCGTGG
TCCTGACTCTGCGCAAGGTGGCTGGCGATGAACTGACTGGGCTCTGCTATGTAGCCAGCATGGACCCGGCAGCCCTCACT
GGCTTTGTGTTGGTACCCCTATCTTGCTACCTGGTACTGGGTACCAGTTTCCTCCTGACCGGTTTTGTGGCTCTCTTCCA
TATCCGCAAAATCATGAAGACGGGAGGCACCAATACGGAGAAGCTGGAGAAGCTGATGGTCAAAATCGGAGTCTTTTCCA
TCCTTTACACAGTGCCGGCCACCTGCGTCATTGTCTGCTACGTTTATGAACGCCTCAACATGGACTTCTGGCGGCTTCGG
GCCACAGAGCAACCATGTACTGCTGCCACCGTGCCTGGAGGCCGGAGAGACTGCTCGCTGCCAGGGGCTCGGTGCCCAC
TGTGGCTGTCTTCATGCTCAAAATCTTCATGTCCTTGGTGGTGGGCATCACCAGTGGAGTCTGGGTATGGAGTTCCAAGA
CTTTTCAGACTTGGCAGAGCCTGTGCTACCGAAAAATGGCAGCTGGCCGAGCCCGGGCCAAGGCCTGCCGAACCCCAGGG
GGCTATGGCCGGGGTACCCACTGCCACTACAAAGCCCCCACGGTGGTCTTGCACATGACTAAGACAGACCCCTCTCTGGA
GAACCCCACACACCTCTAG

MOUSE PROTEIN SEQUENCE : mP5-030.1 (Seq ID No: 39)
MAVPPLLRGALLLWQLLATGGAALEIGRFDPERGRGPAPCQAMEIPMCRGIGYNLTRMPNLLGHTSQGEAAAQLAEFSPL
VQYGCHSHLRFFLCSLYAPMCTDQVSTPIPACRPMCEQARLRCAPIMEQFNFGWPDSLDCARLPTRNDPHALCMEAPENA
TAGPTEPHKGLGMLPVAPRPARPPGDSAPGPGSSGGTCDNPEKFQYVEKSRSCAPRCGPGVEVFWSRRDKDFALVWMAVWS
ALCFFSTAFTVFTFLLEPHRFQYPERPIIFLSMCYNVYSLAFLIRAVAGAQSVACDQEAGALYVIQEGLENTGCTLVFLL
LYYFGMASSLWWVVLTLTWFLAAGKKWGHEAIEAHGSYFHMAAWGLPALKTIVVLTLRKVAGDELTGLCYVASMDPAALT
GFVLVPLSCYLVLGTSFLLTGFVALFHIRKIMKTGGTNTEKLEKLMVKIGVFSILYTVPATCVIVCYVYERLNMDFWRLR

ATEQPCTAATVPGGRRDCSLPGGSVPTVAVFMLKIFMSLVVGITSGVWVWSSKTFQTWQSLCYRKMAAGRARAKACRTPG
GYGRGTHCHYKAPTVVLHMTKTDPSLENPTHL*

MOUSE PANTHER CLASSIFICATIONS
    FAMILY (SUBFAMILY)
        FRIZZLED-RELATED(FRIZZLED)
    BIOLOGICAL PROCESS
        Signal transduction(2.11.00.00.00) > Cell surface receptor mediated signal transduction(2.11.01.00.00) > G-protein mediated signaling(2.11.01.07.00)
        Oncogenesis(2.17.00.00.00) > Other oncogenesis(2.17.99.00.00)
        Developmental processes(2.23.00.00.00) > Other developmental process(2.23.99.00.00)
    MOLECULAR FUNCTIONS
        Receptor(1.01.00.00.00) > G-protein coupled receptor(1.01.01.00.00)

MOUSE GENE ONTOLOGY
    BIOLOGICAL PROCESS
        cell surface receptor linked signal transduction > fz receptor signaling pathway
        cytoskeleton organization and biogenesis > establishment of tissue polarity
        cell communication > signal transduction
        cytoskeleton organization and biogenesis > establishment of cell polarity
        GO biological process > developmental processes
    MOLECULAR FUNCTION
        mannosidase > beta-mannosidase
        ligand binding or carrier > protein binding
        GO molecular function > ligand binding or carrier
        serine-type peptidase > serine-type endopeptidase
        endopeptidase > serine-type endopeptidase
        metalloexopeptidase > metallocarboxypeptidase
        carboxypeptidase > metallocarboxypeptidase
    CELL COMPONENT
        plasma membrane > integral plasma membrane protein
        cell > membrane fraction
        cell > plasma membrane
        extracellular > extracellular space
        GO cellular component > extracellular
MOUSE PROTEIN DOMAINS (INTERPRO SIGNATURES)
        IPR000539 (FRIZZLED)
        IPR000024 (FRI)
        IPR000024 (Fz)
        IPR000539 (Frizzled)
        IPR000832 (G PROTEIN RECEP F2 4)
        IPR000024 (FZ DOMAIN)

HUMAN GENOMIC SEQUENCE : hD5-030 (Seq ID No: 40)
TCTGCGGAGGCTGAAGGTCACAGCCCCACCTGCCCCGCCGTGCTGGGCTTTGCCCCTCATACCTGGAGAGGCGGGTGGGC
CGGGAGCCAGGGTCCTGCGTTGGCTGCAGTGGCCCGCCTGCGGCTGGGCCAGCCAAGCAGGGTCAAGAAAGTCCCCAGAA
CACCTTGGGGACCATCTCTCCCAGAGCCTCCCCCAGGCAGGGGCCGGGCACAGTGCAGCTTGCTGGCCTCAGGGGCTGGC
CCAGGAGTCAGGTGACATTCCCTGGGGACCCCCCTGGGACCAGCACAGCCTCCGAGAAGGTGGGCTCCCGGCCCGGCCCG
GGGCTGGGGCAGGGCTCTGCGCCTGCGCCGTCTGAGGCCCAGGCTGCAGGGAGCTGGGACCCCGGGTCCTTCCAGGTTCC
CTGGCCCCTCTGTGGGCCGGACAGGCCGGCGGGAAGGAGGGTGGGGGTGGGGGACGCTGGGGCCTCTTTCCTGGTCGCCC
GGCGGCAGCTGAGGTTATTTTTTCCCCCTCGCCGCTGCAGAATTTCCCTCCGAGTTGGATAAATAAGCAGCAGAGTCCAA
CGTCAACGAAACAGGAGCTCGCTGGGCCGCACAAAAGCGGCTTTCAGCAGCGGCGGCGCCTCCGGGCCTGTCCCGTGGCC
GGGGCTCCCCGAGCAGCGCAGAGAGCGGGACTGCCCTTGGCCGACGCTGCGATCACGGCCTCAGAGGCCTCCAGGGCTCC
GGCGCTTGTCCACTTCTCTTTTTCATTTTTATTTTTAATTTAAATTTTTTTTTAAGAGATGAGGTCTCGCTCTGTCTCCC
AGGCTGGAGTGCAGTGGTACAATCACAGCTCACTGCAGCCTCTACCTCCCGGTTCAAGCGATCCTCCCGCCTCAGCCTCC
CGAGTAGCTGGGACTACAGCTGTGAGCCACTGGGCTTGTGTCCTCTTCTGTCCTCTCTTCTGGTCCCCACCTGCTCCCTG
CAGAACCCATGCTTGCCATTTGCCCCCACAGCACTCTGTGCAGCCCCTATTAAGGACTCCCTCCCTTAAAACCTGTGGCT

CCGCCAGGCGTGGTGGCTCATGCCTGAAATCCCAGCACTTTGGGAGACAGAGGCCGGCGGATCACGAGGTCAGGAGTTCG
AGACCAGCCTGACCAACATGGTGAAACTCCATCTCTACTAAAAATACAAAAATTAGCCGGGCATGGTGGCGCAATCCCAG
CTACTCCGGAGTCTGAGGCAGGAGAATCACTTGAACCCAGGAGGCAGAGGTTGCAGTGAGCTAAGATCGTGCCATTGCAG
TGAGCCGAGATCGCGCTATTGCACTCCAGCCTGGGCAACAGAGCGAGACTCCGTCTAAAAAAACCAAAAACAAAACAAAAT
AAAACAAACAAGAAAAACCTTCTAAGGCTCCTCATTGCCCGAGGGAAGATCCTGGCTTTCCAGACCCGCAGCATGAAGTG
GGCTACTTCCTGTTTCTCGCCAGTGCTTTTGCAGAGGCTGGTCCCTCTGTCAACAACACCCTTCCCTCTCTTCTACCTGG
CTCCCACTGGGTTTTGAGATTCAGCTCCTGCATTGTTTTGCTGACTCCAGCCCCCACAGCTTCTCTCTCTAATCATGGG
CATCAGCTGACACCAGAGTGGTCACTGCTCTGTCTGTCACTCCAGCTGATCTTGAGCCACCCTAGGGCAGGAGCTGAATC
TTTTTTTGTTTTTGAGAGAGATGGGGTCTTGCTCCATGCCCAGGCTGGAGTGCAGTGGCGCGATCATAGTTCACAGTAGC
CTCAACCTCCTGGGCCCAAGCCATCCTACCGCCTCAGCCTCCCGAGTAGCTGGGACTCCAGGCATGTGCCACCATGCCCT
GCTAATTTAAAAAATATATATAGAGAGATAGGGTCTCACTATGTTGCCCAGGCTAGTCTCAAACTCCTGGCCTCAAGTGA
TCCTCCCACCTTGGCCTCCCAAAGCACTGGGATTCAGGCATGAGCCACTATGCCCGGTCCCAAATTTGGCTCATCTTGTG
TCCCCCGAACCCAGTCTAGTCTGTGCTGTGTGCTGCAGAGGCACAAAAGGGCCATTGCCTGGCATGGAGTAAGTGCTCAC
TTAATGTCCCCTATTAAAAAGTGACATAAAATCAGGCAGAGGTTATAGTGAGCCGAGATCGCGCTACTGCACTCCATCCT
GGGTGACAGAGCAAGACTCTGTCTCAAAAAAAAAAACAGTGACATAAAATCATAACATTATATTCTATATAACAAATGCA
AACTAATCTGTCATGACAAAATGTAGTGCAGTGATTGGCTGCCTGACTATAGATGCAAGGAAGGGAGGGAGGGCACAAGA
AAACTTTTGGGGTGATAAATATACTCGCTGTCTTTTTTTTTTTTTTTGAGGCAGAGTCTCACTCCGTCGCCCAGACTG
GAGTGTAGTGGTGCGACCTTGGCTCACTGCAACCTCCGCCTCCTGGCTTCAAGCAATTCTCCCGCCTCAGCCTCTGGAGT
AGCTGGGACTACAGGCACGTGCCACCATGCCCAGTCATTGTCTTAAAAAAAAAAATCCTGGCATTATCACTTGCCCAAGT
TGCTTCAATTCTCTCTAAATATCAATGTCCTCATCTAAAATGAGAATAAAAAATAACCACCAGCCAGGTGCAGTGACTT
ATGTCCGTAATCCCAACACTTTGGGAGGCCGAGGTGGGTGGATCACTTGAGGTCAGGAGAGGCCAACATGGAGAGACCAG
CCTGGCCAACATGGTGAAACTCCCTCTCTACTAAAAATACAAAAAATTTAGCTGGGCGTAGTGGCGCATTCCTGTAATCC
CAGCTACTCAGGAAGCTGAGGCAGGAGAATCACTTGAACCTGGGAGACAGAGGTTGCAGTGAACTAAGATCACGCCACTG
CACTCCAGCCTGGGTGACAAAGCGAGACTCTGTCTCAACAAAAATAAATAAATAATCACCTCATAAGGTATTGTCAGGAT
TAAATGACATACAAATTAATATTTAATAAATGGTAGTTATGATTAAGCTCCTTTCACACTATACTGTAGTATGTACTAGT
TATTAATTTTTTTTTGGACAGGGTCTCTGTGCCCAGGCTGGAGTGCAGTGGTGATTGCAACCATAGTTCACTGCAGCCTC
GACCTCCTGGGCCCAAGTGATCCTCCTGCCTCAGCCTCCCAAGTAGCTGGGACTACAGGTGTGTGCTACCATACTCGGTT
AATTTTTTTCCTTTTTTTGTAGACATGGGGTCTCACTTTGCTGCCCAGGCTGGAGTGCAGTGGCAAGATCACAGCTTACTG
CAGCCTCGACCTCCCAGCTCAACCAATCCTCCTGCCTCAGACTCCCGAGTAGTTAGGGCCACAGTTGCACACCATTACAC
CTGCCTAGTTTTTAAATTTTTGGTAGAGATGGGGTCTCGCTATGTTGCCCAGTCTACTCTCAAACCCCCAGGCTCAAACG
ATCTTCCCACATCGGCCTCCCAAAGTGCTGGGATTACAGGCGTGAACCACCAAGACTGGCCAGTTATTAACATTTTAATG
TGCTACTTTCCAGTAAGATATTTGCATTTTAACTGGAATATGTTCATTATGTGGACCAATCCTGATGAAGACTGCATACA
TATAATCCATAGGTCAAAAATCATCAAATTGGGACAGGTGCAATGGCTCACACCTGTAATCCCAGCACTCTGGGAGGCCG
TGGAGGGTGGATCACCTGAAGTCAGGAGTTCGAGAACAGTCCGATCAACATGGTGAAACCCCGTCTCTATTAAAAATACA
AAAACTAGCCAGGTGTGGTGGCGCACGCCTGTCATCCCAGCTACTCTGGAGGCTGAGGCAGGAGAATCGCTTGAACCCAG
GAGGCGGAGGTTGCAGTGAGCCGAGATCATGCCACTGCACTCCAGCCTAGGCAACGAGAGTGAGACTTGGTCTCGAAAAAA
AAAAAAAGAAAAAATGGAACAGACAGTCTAGTATAGCCCTTGATAGCTCATAAAGGGAAACTCAGTTAGAACCAGGGCT
CAAGCTACAGAAGGGAAAATATTCCCTGAGCCACACCCGGGGTCTTTCCAGCCCCGCCCCTGTGCACAGCCTGTGTACCT
GGGCACAGGTGCACACACAGGAGTAAACAGGCACCTGCATGGACCCTGTGTGTACACCTAACCTACACACACAGAGACAC
ACACACACACACGACGCACACACACAGACACACACAGACAGATGCGCACACACAGACACACACAGACACAGAC
ACATAGAGACACACACAGAGACACACATACAGAGCCACACAGACACACACAGAGACACACACAGACACACACAGAG
ACACACAGACAACCACACACACAAAGACACGCACACACACAGAGACACACACATACACACAGAGACACACACACAGAC
ACACAGATGCACACACAGACACAGACACACACAGATACACACACATACACACATAGAGACACACAAAGACATACACACAC
ACACAGAGATACACACAGACACACACAGACACACACACAGAACACCCCCACACACAGACACACACACACAGAGACACA
CATACAGAGACACACAGACACACACACACAGAGACAGACACACACACTCAGAGACACACATACACACACAGAGACACACA
CACACACAGAGACACACACAGAGACACAGACACACACAGAGACACACACACAGAGACACACACAGAGACACACACACACA
GACAGACACACAGCCCCCCACCCAACACACACACTAACTGGCACCTGCCTCCGCAGGAACACACTGGTGCTCCTGCCC
ATCCTGCCCCAGGGAGCAGGTGTGTCGGGTGGACCTGCTGGGTACCCCGAGACCACAGACAGCCTCTCCTGTAGGGCAGC
CCAGGCACCTGAAGGAGGCGCAGGATATTCCCCTTTCTGGTCCCTGCTTACTCACACCGCCACCTCTCTCCCCCAGAATC
CAATCAATACCCCTTCCCCAGCCTTAGGCCCCACAAGAGTTCAGCCATTCCAGAAGGATCTCTGCTCCTCTTCTCATTCT
GACATTCTTTAGACACAACAGATATTTATGGGGGCCCACTCCAGGGCAGGCTCCCAGACGCTGCAGAGCACAGCCAGGGC
CCTGACCTCCCCAGGCTCATGGGGATGAAGGCACAGGTGCAGCATGGTGGCTGCAAGGCACAGAGGTGGGCACGAGGGTC
CGCAGGGCGCTGAGTGGGGGTCATCCTGGAGGAGGCAGGAAAGGAAGGGGAGGGTGTACCCTCTAGGGGTTGACATGTGT
TCGGGGAGGGGAGGGTTGGGGATGCAGGGACACAGGTCAGGAGGGCGTCCCTTCCCACATATGCAGGACAACTACTGGCC
CCAGCCAGGGGTCCCAGGAGGGACGGCTTCTCCCCAGGGGGAGGAGGAGTTAGGGGGGCCCCTCGCCCCAGGGAGGGGTG
CCGGAGATGGGGCATTGATAGTGACCTCCCTCCTCCCGGCCCCTCCGGTCCTCCGGTCCTCCGCATAGTCTCCCTTTCAGT
GAGCTCCAGGAGCATTGTTCCACCTTTGTAAAGAGGGAGATCGGGTGACAAAGCCGGTGGGTGGGCAGGAGGGCCAGAGG
CCTCTTCTCTCCCAGGCCGCATCTGCGTGGGGGGCGGAGCTCATCCACTCCCCTCCTCTGGCTTCCCAGGCGCGGCCGTTG
CCATGGTGACAGGCCTGGCATCCCACGGCCCCTGGGGCGCCCTGCCCACCGAAGCTGCCAGAGGAAGGGCCCCCCGACGC
TTCTCCACCCTGGCAAGATGGGGGCGGGGGCCCACAAAACCTGGCTCTTAGGCTAGCCCCAGCACTGGGAGGTTTCTAGC
AGCCTGAACCTCCCAAGTCTGTGGGAGGGGAGCCCCACCTCCTGGCACCCTGACAGCTGGGGGCTCAGGAGTTGGGGCCA

```
CACATCACTATCATCTGTGCACCTAGGCCTCCTTGGGGCCAGATACAGAGAAATAATATGGTTGTTTTTTTGTGGGTTTT
TTTGTGGAGAGAGGGGGTCTTGCTGTGTTGCCCAGGCTGGTCTTGAACTCTGGGCTCAAGCAATCCTCCCACCTTGGCCT
CCCAAAGTACTAGGATTGCAGGTGTGAGTCATTGCACCCGGCCAATATGTTATTTCTTGATGTTATTCCATGGCCCTTGA
ACAGTGGCTGGCATGGGGCCGAGGGAGGGCACGGATGCAGAGGCCACAGTGTGCTGGACAGATGGGCTGGCCTGGACCCT
GGATCCACAGAGCAGAGCCTCCAGCCCCAGCCCAGCTCTGCTGGGCTGGCCCTGGGAGGTGATGCTTCAATCTGGGGTGC
CAGATGGAACAGAAGAGGAGGAAGACCTTCAGAGCTGGGGAAACAAGCCTGGCAAAGGCCTGGAAGCATGGCTTTGCAGG
GTGTTTCTGGGGAGGAGTAAGAGTGGGGTGTCGGGTGTGACAGGAAATGAAGAGGTGAGGCGGGGTCTCTGCTTGGAGGA
ACTTCCACTGCCATCCCTGAGAGCATGCACTTTATTCTGGGCAGCTGCTGCTGAAGTTTTGGGACCCTCCATTATCAATG
CCCAGGTGATCAAAGCTGGGTCCCTTCCCTTCCCTGCAGTGCCCTGCCCTGCCCTGCCCTCCCTTCCCCCCCGCTCCCCT
CCCCCTCCCCCTCCCTCCCTCTAACCCCTCCCCCTCCCTCCCCTTCCCTCCTCCCCCCTCCATCCCCTTCCCCTCCTCCC
CCTCCCTCCCCTTCCCTCCTCCCCCCTCCCCCTCCCCTGCCTCCCCTCCCCCTCTCCCTGTCTTTCTTTTTATTTTTT
TTCTAGAGATAGGGTCTCACTCTGTTGCCCAGGCTGGAGTGCAGTGGTGTGATCATGGCTCACTGCAGCCTCCCAGGCTC
AAGGGATTCTCCAGCCTCAGCCTCTTGAGTAGCTGAGACTACAGGCGCATACCACCATCCCTGACTTTTTTGTTTATTTT
TGTTTTTAGAGATGGTGGGGTCTTGCTATTTTGCCCAGGCTGGTCTCGAACTCCTGGGCTCAAGTGATTCTCCCACCTTGG
CCTCCCAAAGTGCTAGGATTACAGGCTTGAGCCACGGTGCCTCACCTGGGTCCCCCAGGGTCACAGGTACCATACTCCTG
CCTTACCTCTTTGGGAACTTCAGGACGTCTCATGGCCCACATGTGGCTGAGTACTATCCTGTGCTGGACACAGGGCTGGC
TGCGTCTCCTTGGTGCCCCATCTCCCGATCCTCTTGAGGAGCGAGGGGCTGAGTGTATGCACGAGATGGCATGTCCCCTG
CCTGGTCCCCTCCCTGCTCCAGCACAGCTCTAAGGGTAGCAGAGTACTAATTACAGGGATGTGTGGGAAATGATGGCCCC
ATTTTCTCACCATTGCCACCACCACTGCTGACTCCTTCCCAAGTCCCTACCACAGGCCAGGCTCTAGTGCAGGCAGGTGT
GGCTGAGCTGGGCCGGCTGGGCTGGTGTCCTGCCTCTGGTGGTGTTAACAGGCAGCCTCTTGCAATGGAGGAGCCTGGAT
GCCAATCTTGACCCTTACGAGCTGCGTAATCCTGGGCAAATATTTGAGTTTGCTAAGCCTCAATTTCTCATCTGTATATG
GGTACGGGATTCCTTGTAGCACTGTGATGAGGGTGAACAAATGATCACTGTAAAATCCCTGGCCCAGAGCCCAGGAGGCT
CACAACAGGTCCTCGGTCAGTTTCTGTGCCTCCCTGAAGTTCTAGGTTACTCACTCATTCATTCACTCACCATCCCTGGG
CTCAGCCAGGGTGCCTAGCCCTGTACTAAGTGCTCGTATGAAACAGTGCTAAATGCTCACGTGCAACAGTACTAAGTGCT
CACGTCAAACAGTACTAAGTGCTCATGTGAAACAGTGCTAAGTGCTCACGTGAAACAGTACTAAGTGCTCACATGAAACG
ATGCTAAGTGCTCACGTGAAATGATGCTAAGTGCTCACGTGAAACAGTACTAAGTGCTCACATGAAACGATGCTAAGTGC
TCACATGAAACAGTACTAAGTGCTCATGTGAAACATTCTGAGTCCTCCAGGTGTTTCCAGGCCAAGAGAATAGGAACAGT
TGGGTGAAAGGGGGATGGCCTTCTCTAAAGAGTCCCAGATCAGGGCCCCTTGCCCCTTGTGTGGCCTGCCGGTGGCACTT
GGATTTCACCCACAGAGCTACCCAGGAACCATGGCAGTTTCTAAGCGGGTCCCAGGAGTCTCAAGGGGGCAGCCTAGAGA
GAGGTGAGAGGGTGATGGCGACACTCCCCAGAGGAGCACGGTGGCCTTGTGAAATGGGTATGGGTTCCGGAACAGACAAT
CACAAATGGAACACTTAGTAGCTCTATGCCCTTGGGTATGTCATTCCTCTCTGAGACTCAGTTTCCCCACCTGGGAAATA
AGGATAACGTAGCATTCTTGGATTGCTGAGAAGATACAATAAGGGCAAGTATGAAGTGTGATCGATCAGATTCAGGTGCC
CAACACGTGTTGTTGATTTTCTTTTTTTTTTTTTCTTTTTTTTTTTTTCAGGCCAGGCACCGTGGCTCATGCCTGTAAT
CCCAACACTTTGGGAGGCCGAGGTGGATGGATCACCTGAGGTCAGGAGTTCGAGACCAGCCTGGCCAACATGGTGAAACC
CCTGTCTCTACTAAAAGTACAAAAATTACCCGGGAGTGATGGTGGGCGCCTGTAATCCCAGCTACTTGGAAGGTTGAGGC
AGGAGAATTGCTTGAACCCGGGAGGCGGAGGTTGCAGTGAGCCAAGATCGCACCATTGCACTCCTGGGCGATGAGATGAG
CCTGGGCGACAAGAGCGAAACTCCATCTCAAAAAAAAAAAAGCTATTTTTTTTCTTTATTAGAGACGAGGTCTCACTAT
GTTGCCCAGGCTGATGGGGCAAGATGGGGGTGGGGGTGAGCCACTGCGCCTGGCAGAACTGTTGATTTTAACAGTAGGAT
TATCTGCCTGCATTACAGAAAAGCAAGCTGAGGCTCAGAGAGGAGCCCCAAGTCACATACATGAGGGGGAGAGGCAGACG
CTGTATTTGAGCTGCCACTGGGTACAAGGCAGACTTCCACACGTGGGAAGCCTTAGAAATGGTCTCCCCTGGCCGGGCAC
GGTGGAGCACGCCTGTAATCCCGGCACTTTGGGAGGCAGAGGCGGGCAGCATCCCTTGAGCCCAGGAGTTCAAGACCAGCC
TGGGTAACATAGTGAGACCCTGTCTCTACAAAAACATTTTAAAATTAGCCGGGAGTGGTGGTGCCCACCTGTAATCCCAG
CTACTCTGGTGGCTGAGGCGGGAAGGTCGCTTGAACCCCGGGAGATGGAGGCTGCAGTGAACTATGGTCGTTCCACTGGAC
TCCAGCCTGGGCGACACAGAGAGACCCTGTCTCTAAAAAGAAATAACAATTAAAAATAAAAATAAAAAGTCTTCCAGCTG
GGCGCGGTGGCTCAAGCCTCTAATCCCAGCACTTTGGGAGGCCGAGGCAGGTGAATCACCTGAGGTCAGGAGTTCGAGAC
CAGCCTGGCCAAAATGGTGAAACCCCGTCTTTACTAAAAATACAAAAAATTAGAGGGGCGTGGTGGCAGGAGCCTGTAAT
CCCAGCTACTCGGGAGGCTGAGGCAGGATAATCGCTGGAACCCGGGAGGCGGAGGTTGCAGTGAGCCGATATCGCGCCAC
TGCACTCCAGCCTGGGCGACAAGAGCAAACTCCGTCTCAAAAAAAAAAAAAAAAAAAAAAAAAGTCTCCCTCCTAGAGTA
AAATGAGGCGACCCAGTCTGAGAGTGTGGGTACGGGGACTTCCCGCAGCCTCTCCCAGGCAGCAAGGGAAGGGAAGAAAC
CGAGTGTAAGCGGAGGGCAAGCGGGCGGACACCCACCAGGCCCGGCCCCACCCGTGCTCCGCGTGGCCGGGAGACGCGG
CGCCTTTAAAGCCCAGTGGGAGGCGTGTCCCTGGGCGTGTCCTCCCGGAGCCCCGCCCCGGGGGCTGGTAGCCTCGGCCC
CGGGAGGTTGCGCCGGCTCTGGCTCAGCGGCGGCTCCGGTGGCAGCGGCGGTGGCGGCTAGGCGGGCTCGGCGGCTCCTG
TCCCTCGGGCGGCTGCCCGCTCGCTGCCCAGGGCGCCGGACACACGTGGGCGGCTCAGCGATGGCCGCGCCCCGCGAC
GTGGCGGGCAGGCACCGGGGCGCGGGACCGCTGAGCCCGAGTGAGCCGGGGCCGCGCTCCCGCCTTCGGCCCGCGGGCCTCC
CGGGATGGCCGTGGCGCCTCTGCGGGGGGCGCTGCTGCTGTGGCAGCTGCTGGCGGCGGGCGGCGCGGCACTGGAGATCG
GCCGCTTCGACCCGGAGCGCGGGCGCGGGGCTGCGCCGTGCCAGGCGGTGGAGATCCCCATGTGCCGCGGCATCGGCTAC
AACCTGACCCGCATGCCCAACCTGCTGGGCCACACGTCGCAGGGCGAGGCGGCTGCCGAGCTAGCGGAGTTCGCGCCGCT
GGTGCAGTACGGCTGCCACAGCCACCTGCGCTTCTTCCTGTGCTCGCTCTACGCGCCCATGTGCACCGACCAGGTCTCGA
CGCCCATTCCCGCCTGCCGGCCCATGTGCGAGCAGGCGCGCCTGCGCTGCGCGCCCATCATGGAGCAGTTCAACTTCGGC
TGGCCGGACTCGCTCGACTGCGCCCGGCTGCCCACGCGCAACGACCCGCACGCGCTGTGCATGGAGGCGCCCGAGAACGC
CACGGCCGGCCCCGCGGAGCCCCACAAGGGCCTGGGCATGCTGCCCGTGGCGCCGCGGCCCGCGCGCCCTCCCGGAGACC
```

```
TGGGCCCGGGCGCGGGCGGCAGTGGCACCTGCGAGAACCCCGAGAAGTTCCAGTACGTGGAGAAGAGCCGCTCGTGCGCA
CCGCGCTGCGGGCCCGGCGTCGAGGTGTTCTGGTCCCGGCGCGACAAGGACTTCGCGCTGGTCTGGATGGCCGTGTGGTC
GGCGCTGTGCTTCTTCTCCACCGCCTTCACTGTGCTCACCTTCTTGCTGGAGCCCCACCGCTTCCAGTACCCCGAGCGCC
CCATCATCTTCCTCTCCATGTGCTACAACGTCTACTCGCTGGCCTTCCTGATCCGTGCGGTGGCCGGAGCGCAGAGCGTG
GCCTGTGACCAGGAGGCGGGCGCGCTCTACGTGATCCAGGAGGGCCTGGAGAACACGGGCTGCACGCTGGTCTTCCTACT
GCTCTACTACTTCGGCATGGCCAGCTCGCTCTGGTGGGTGGTCCTGACGCTCACCTGGTTCCTGGCTGCCGGGAAGAAAT
GGGGCCACGAGGCCATCGAGGCCCACGGCAGCTATTTCCACATGGCTGCCTGGGGCCTGCCCGCGCTCAAGACCATCGTC
ATCCTGACCCTGCGCAAGGTGGCGGGTGATGAGCTGACTGGGCTTTGCTACGTGGCCAGCACGGATGCAGCAGCGCTCAC
GGGCTTCGTGCTGGTGCCCCTCTCTGGCTACCTGGTGCTGGGCAGTAGTTTCCTCCTGACCGGCTTCGTGGCCCTCTTCC
ACATCCGCAAGATCATGAAGACGGGCGGCACCAACACAGAGAAGCTGGAGAAGCTCATGGTCAAGATCGGGGTCTTCTCC
ATCCTCTACACGGTGCCCGCCACCTGCGTCATCGTTTGCTATGTCTACGAACGCCTCAACATGGACTTCTGGCGCCTTCG
GGCCACAGAGCAGCCATGCGCAGCGGCCGCGGGGCCCGGAGGCCGGAGGGACTGCTCGCTGCCAGGGGGCTCGGTGCCCA
CCGTGGCGGTCTTCATGCTCAAAATTTTCATGTCACTGGTGGTGGGGATCACCAGCGGCGTCTGGGTGTGGAGCTCCAAG
ACTTTCCAGACCTGGCAGAGCCTGTGCTACCGCAAGATAGCAGCTGGCCGGGCCCGGGCCAAGGCCTGCCGCGCCCCCGG
GAGCTACGGACGTGGCACGCACTGCCACTATAAGGCTCCCACCGTGGTCTTGCACATGACTAAGACGGACCCCTCTTTGG
AGAACCCCACACACCTCTAGCCACACAGGCCTGGCGCGGGGTGGCTGCTGCCCCCTCCTTGCCCTCCACGCCCTGCCCCC
TGCACCCCCTAGAGACAGCTGACTAGCAGCTGCCCAGCTGTCAAGGTCAGGCAAGTGAGCACCGGGGACTGAGGATCAGG
GCGGGACCCCGTGAGGCTCATTAGGGGAGATGGGGGTCTCCCCTAATGCGGGGGCTGGACCAGGCTGAGTCCCCACAGGG
TCCTAGTGGAGGATGTGGAGGGGCGGGGCAGAGGGGTCCAGCCGGAGTTTATTTAATGATGTAATTTATTGTTGCGTTCC
TCTGGAAGCTGTGACTGGAATAAACCCCCGCGTGGCACTGCTGAGTCCTCTCTGGCTGGGAAGGGGGGAAGGTAGGAGGG
TGAGGGCCCTGCAGGAGTCTTTCTTGTAAGCAGAGCTGGCAGTGGGTGCTGGCACCCCCCAATCAGGAGGAAGTCCCCCC
GGAACAGCTGTGCATTGCAGGAGTCCCAGACACCATGTGTGCGTGTTTTCCCTCCCGCCCGTCTCTGTGCTTCCAAATG
GAGACAGGGACGCCTGCGGATGTTCAGGGAATTCCCCAGGGTGACACTGCCACCTCTGGCCCCAGGGAGTCCAGTTTGAG
GCTATGGATGAGTTTTCCTTCTCTGGGAACTTGCTCTCCTTATTGGCGTGGAAAGTAAAGGTGTCAGCCTGTGTCAATGT
TGTCACGAGGACCGCCTCCCTGGGGCCTTGGAAAATTGCTGGTGCGTCTGCCTGGCTCCTCGGGGCCCCCACCAGGGCAG
CCCCCACCAGGGCAGCCCCAGCCTGCTCCCTGGCGTCTCCAGGTGGGGTGAAGAGGTTGTTCTGGTCTGTGGGCCTCTGG
GTAGCACAGACATGCACGTGGAGCTGGGAAGCCCTTGAGTAGAGTGGGAATTGGGCCCCTAGCTCCTAAGCTTGTCAGAT
TTGAGTTGCTAATGGCAGCGGTGGGAATGAGGCAGCTCAGGTGGCTGTGCAGAGCTCCACACTGGGGCAGGTATGGGGAA
AGGTCCCATGTCCATCTGTGTGCTGGGGTAGCTTAGGGCTGCTAGGGCCTGTCCTGTTTCTGCGGACTGGGTAGCTGGGC
ACGTGTGGCCATCTGGAGACCCCTATTGGCTTGGGGTCAGACCCCAGGGCCCCATCTTGGCTGCTGTGACTCCAGCCAGG
TGAGAGTATTAGTTACTAGTGTCCCTGGGTGCCTACTATACACGGCCGGCATTTGGTGTCAGTGCCAGGAGATCCCAGCG
GTCCAGCCCTGCTGCCAGCCTCACTTTAGAGCCACGGGGAGGCTCCAGGAGGGGAGACGCTCAGGTGCTGAGGATGAATG
GTGAGGCGGAGCCCCAAACCTAGGGCCTCTCGGGCTGGAGCCCTGTGCCCTACCCCAACCACCAATCCTACAGGGACAAG
AAAAGTCCAGTGAGAATCAAACAGGTTGGGTCACGCTCCCAGGAGACAAGACCATGGCAGGCTGAGGGGGTTAAGGGGTA
TTCCTCACCACAAGGCGCAGTGGTGCCCCAGGGAAGCTTACAGCCCTTGCACCTGTTCTTAGGCCGTTTATTGCACGGGG
AACGCCCTCTCCATCTAAATCCTTCCCTTTGCGCAGAGCACACATTAAGTAAGTCCCCATGTGCCTCTGACCAACAAAGC
CAAGTGACCCTACCTCCTTGTAATGCTGCACGAGGGTTTTTTGTTTGTTTTTTGTTTGTTTTTGTTTTTAATTTAAGGCA
GAGTCTCACTCTGTTGCCCAGACTGGAGTGCAGTGGCACGATCTCAGCTCACTGCAATCTCTGCCTCCCAGGTTCAAGCA
ATTCTCCTGCCTCAGCCGCCTGGGTGGCATCCGCCACCATGCCTGGCTCATTTTTGTATTTTTTTGTAGAGACGGGGTTT
CACCATGTTACCCAGGCTGGTCTTGAACTCCTGGCCTCAAGTGATCCACCCACCTTGGCTTCCCAAAGTGCTGGGATTAT
AGGCTTGAGCCACTGCACCTGGCCATGCGAGGGTCTTTGAGTGGGAAACAGAATGAGCTCGTTAGAGGCAGTAAAGTGCC
CACACTGGGCGTGGGAGGGGCAGAGGCTTCAGGGAGCTGGGCTCACAGGCAGTGGAAGGGAAGATGGGAAGGGCCTATAC
CAGGGCGGGGGCAGCTGGAGCGAATGGAGAGGATTCCACAGACTTGGGAAGAGCAAGGCAGGCGGGGTGTGGGAAAGGCA
GGGGGAGGATGAGCATCCAAGGGCACCCACCTGCCCCTTCTTTCTTCCTTGCTGTCAGTGACCATGTCCTGCTCAGCAGA
CACCAGCCTGCTGCTGGTTTTCAGGGCAGCCCAGATGGGAGCGCAGAGAGGGGCAGTGCAGGGTCAGGACAGGCCCCGGG
GGCTGTGCTTTGAGGCCAGGACCTGGGAGGGAGGGCAGGGCCCTGCCAGCTTTCTCAGACCTGGAAGATGCTATGGAAAT
AAGTCCTAGGCCCTGGAGGAAAGGGAAAAAGGAAACACAGCCCTCTCATGTGATCTGCTCGCCTCACGTTTCGGACGGGA
AGTGTTTAGATTGGTTGGCCGCTACAGCACATAGCCACGCAGTTTGAGGCCAAGCTGGGAGTTGAACCCAAGTCTGCCTC
CAGAACTTTTGTGCGTTCCACACCAGGGATCCGATTAGGTCCTGGTGGGGAAAGGCAGGGTGTATGCCTTGGCAAGTTTA
TGTCCCCAGAAGCTGGGCAAAGCGGGTGGGGAGGAGACAAACACCCCTACCACTGCCAGACCTCTCCCCACGCCCATTAA
AGAACCCTCCAGGGCCAAACAAAAATACCTCGGAAACCCACACGTGCTGGCATGTGCATCGCGTGCCCAGGCCCGGGCCT
TCCCCTTAGTGGTCCTGGTGCCCAGCATTTGGGTCTTCAGAGTGCGGCACCACACGCCCGCAAGCCTCTTGGGAGGCTGA
GCCTGTCCCTGTGGGTTTGCGCTTGTGTGTGGTGAGCCCAAGATCCTCCTGCTCCCAGGAAGTTGCCCGCTCAAGGAAGC
AGCTCCTGAACACAGCAGTCCCAGGTGTTGAAAAGCCAGGAGGCCCAGGGCTGGGGTTAGGAGAGCGCAGTTAATGGGAG
GTGAGTTCCTCAGTGTCCTCCAGAGACACCCTAGCCCCTGGCCAGCAGGAAGAGATGCCCCCTCTGAGTGGCTCTGTTGG
AATCCAGATGCCACCTGTTAACCAAGTCTACTGACCATGTCACCTCCCCAGAGACAAAACTTGAGAAGAGTGTGCAGGAC
AAGGACAGGCCTCAGCTGACTCCCAGGTCATGACTCAGGCCCCTGTCCTCAGAGATGGAGTTCCCATGCCCCCATTTGCA
GTCATCAAAGTGGCTGCCCCACAAGGTCACAGCCTGGTAAGGCAGTGGCTAAGAGTGTGCTGAGACCAGGACACGACCAG
AAGTGACCACCCCAAGGCAGTATCCTAGTGTGTGCCTGGGGAAATTACTGGAGGAGCAGGACTTCATAGAGGAAGGCTGG
GAGCCCCAAGTGTCACTGTCGCCATGCAGAAAATGTCCAGAAGGAGCATGGTTCTCTTCTCCAGAGCTGGAGGCTGGAGC
GGATGAGCAGGAAAATTCAATCTGCTGTTGGGGCAAGCCCGCTTCCTCCAGGCAGGACAAAAATTCCCCAAGTGTTCTGT
```

```
GTAGGACCTGGCTCGCCCGTGCTTACCAAGGCCGCAAAGCTGCTCAGCAGCAGAACCTGCGCCCAAGGCTCAGGCCTCAA
AGGCTCTTCCCATTACACCAGCTTCACCTCGTTCAGCTGGTCAGGAATTGGCAGTTTAATTTCTCAGTAATTGGGCCCAC
AGCAGTGGCTCAGCAAAATCTCTGTATTCCATTGGCGGGTGGCTGCCTAGCAGCACCCTACGCATATCAGCCAGTCAGAC
TTTAAAAGATAAATGAAGGCATAAATAAAACAAGATTAGACATGTGGTAGAACCGTCAAAGCCGAGTGCATGGGAATTCA
TTATACTTTTCCACTGTTTGTGTGTTTGAAATTTTCCTTGAGGCTGGGCGCGGTGGCTCACACCTGTAATCCCAGCACTT
TGGGAGGCCAAGGCGGGTGGATCATCTGAGGTCAGGAGTTCGAGACCAGCCCGGACAACATGGTGAAAACCTGGCTCTAC
TACAAATACAAAAACTTAGCCAGGCGTGGTGGCATGTCCCTGTAACCCCAATTACTCGGGAGGCTGAGGCAGGAGAATTG
CTTGAACCCAGGAGATGGAGGTTGCAGCGAGCTGAGACCACGCCACTGCATTCCAGCCTGGGTGATGGAGTGAGACTGTC
CCCACCGCCCCCCTCCCCAAAATTTTTTTTCCCTGAGAAAGGTTTTAAAATGGCAGAATTTCCCCATAGTGAAGGCTTAA
AAACCTATTTTCACCGTAATTCCTAGTTTCAAATGAAGTTTTTCAGGGTTTTAGACGGAGACAGGGCCAGGAGAGGCTTG
GAGGGAGTACTTCTTTCTGTGGTTAAGAACACTGAGAGGCCCTGACTCTGGAGGCCAAGCCTTCAGAGGACCCGGCTCCA
AGGGTCAGTGGGAAAGCAGCACTCGGGACAGCACTACCCACCCCCCACCCACCAAGAAAGAGCACAAACAGCCACCAGAGT
GGTTTCTTCTTTATAAACACAAGTACATGAGGTTTATTTGAAATTCCAATTTATTACAAGTTCACCCTTTAATGGGGCCC
TTCCTCTTTGAAGACAATAAAAAAAAAAAAACAACAACAAACAATTCAGGTGTCACTGAAGTGGGAACACAGGATTCTCAC
TATGAACAAGAACAGACTGGATGTAGGAGGCAGGGGAAGCTGGCGGTGGTGGGGTTATGAAGCTGTGATGTCAGTGCCAA
CTCCATGCCTATAGAAGGGACGGTAAACTACCCAGCAGCCCTGGAGCAATCCTCGTCGTCCTTGGGCAGGAGGACGGAGG
AAGCAGAGGCCACATTCCGCAGTTTAAAATTAAGAGGCTTGATTCTCATGTTCTAGGAGCTGCAGTCCCCTTGGTGGACT
TTAAATAGGTACAGAAGAGCTTGGGTGCTCAAAATTTCCAAATTCAAAGGTGATAAAAAAGAAAAACCCTGTTTTGTACT
TTTATCAAAATCCATCATAAAAGGGAAAGAAGACTACAAAGTTTTGCCTAAATATAACAACTAGAGCTAGATTTGTTGTG
GGGGAAGGGGCTCTCAGAGCTTCTCTGCCGCTCTCTCCTCCCACCCCCCACCGACACTGACCACTGGAATCTTCAGGTTC
TTGAGGAGTTCCAAGGCTGAGGTCAGAACGGGCATGGGAGGCAGCAGACACAGCACTGCCCTGCTCCCCCTCCAATGTTG
CTGCTTGCCTCAATGAGCCTCCAACCCAACTGCTCTAGAGGAGAGTGGATGGGCTGAAGGAGAAGCCAGGGAAGTTAGAG
CAAACACATTATCATAGAAACTGTACTGTACATGTGCTAAAGCAAGATGTCAGGAGTATCTTACAAGATGTTCTTTATAC
AATATCACTGCTGAAACAAGCAACTTTTAATAACTAGAAAAGTCAATTTAAAAAAAAAATTAAACATTTAAATTCTTCCT
GCTTTTCTTCTGCTCCCCTAAGAGCTTGACTGGTATGTGGGTGGGCTGGGCTCCAACACCCCTCTGGCCAGATCCATCAA
AGTAAAGGTTATATGCATGATTATAAGCAGGACTCCAGTCTCTAAGTGAAGTGCCTTAGTTTTGTTTTTTTGTTTTTTGT
GGTTTTTTTATTTTTTGGGGGGGTGGGAGGAGCTGTCACAAAGAAGGGGAGATTCAGGAATGGCTCCAGGACACGTCCCT
TTACTCTGCCAAGATCAACTCAAAAGACCACAACTGTTTCATGATGCTGGTGAATACACAATTCTGTATGGCACTCACTG
ATACTGTCACCTGAGAGGAAGACGGGGGAAGAGACAGAGTATGGGCTTAAAGAAACAAGACTGTATAATAAATACAGATT
AAAAAAGAAAAATCGCCACCATCTCCCCTGTTGGCCTGATTACCCCGATCCTGCTATGTAACACAGCAATCCCTCCCCTG
GAGACCAGAGGGGCTTGGCACTGTGGTGGAAGCCAGAGCGAGCAGGCCCTTAGGAAAGAAGGCAGGAACAGGAACTGGCT
TCACCAGAAAAGCTAGACCCTCGGACTCCTCCTGGAAACTCTCAGAAGGGAGGGTTATGGCCCTCTTTGTCCCTTCATAT
TTCTGGACAAAGACCACCAACCCAATATCAAGCCCCATAAAGAGCTTTTAGAAAAACAGCATAAGCTTGGGATGACAGGC
GTTTCTGGACTCCCTGTGATCTCTTCCAGGTTCTTGGTCTTCCTCGCTCGCCTCCCTCCCACCCTCCCTAGCTGTCCCCC
CACCTCAGCTCCCTTACCACGGCCCTGCCTCTCTACTTCTTCTGTCTTCGTCCCCTGGACTGTCCAACGGCCTCTGGCTC
ACTGTCCCCTTCATCTTCAGCAAGCCTATCAGGAAACTTCTTGCGCTTCCTGCGGACATATGGGTGGCCAGGAAGGTGTT
TATGCAACAGAGCCACTAGACACTGTTCTGTCTTCACCATGCCAGCTTAGCACATGGCTGCCACGGCAGTTGTAAACCTCA
GCATTGGTAAACACTTGCTTCATGTCAGTAAGAAACTCCTGCACAGAGCGGTAGCTCCCACAGGAACATTTGTTCTGCAC
TGTCTGAAAGTCCATGGGGTGCGTGATCACATCATAGTAGTCCTCGGCCTCATCTCTGGTCACAGGCTCCCTGTGGAGAA
GAACCAAATGGAGAATCTGCACAGCCTTGACGGCAGTGCCCCTGCCACTGAGCACGTCCTGAAAAGCAAGGGCTTGGCT
GAGAGCCCCTCAGGGGCTCTGGAAGCTTTTAGAACCAGCCAGGCCACTCCTCTCCCCTGCACCTGAGAACACAGCACTCA
CCTGAAGGGCCAGCTGAAGCGGTACTTCACGATCTTGTGGAGGATCTCTTCACACTTCTGCAGCTCCAGGCTTTGCCTCC
GGGAGCTCCGCTTGGTCTGAAGCACCTGGCAGGAAAGAAAGAACAATGTTATTACAGATTCAAGACAGGAGGAAGAATGG
ACAGGGCAGAAAATAAGTGTATCTGCTCAAAAAAGGAAGAGAGTTCAACTATTTCCTTGGCGCAGAGGTGCTGAAACCTT
CTGATATTCAAGTCAGGGAGCACCCTCGGCCCAGGTGGCAGGCATCTTCCCAGTAGTCCACTCACTCGCCTCCCCCGGGG
CCACGGCAATCAAAGCAGCAAAGAGCAGGCAGCGGGGGCCGCCTTTACCAGCAGTCCTGCTGGAGGTCGGCTGAAGGGGG
CTACTGTCAACAGGACACTAGCTCTCAGAGAACAGGTGGACCTGGGGTTACAGTGACACCATGGAAGGCATCAACAAGC
CACTGCAGAAAGCAAAACATGCTATCTGGGCCAACAGCAGCCAATTCTGTGGGGGTGGGGTGCCTCTGGTAAGAAAAGCA
AGAGGTCCAATTCCCTTCCTGGGCTCTTCCACCGTAGACATGTGAGGGTTAAGTGGCTAAAGAGAAAAGGGCGGGGCCT
CCATTCCAGGTAGCCATAACAAAAGCTGTGATTCACAGCAGAGGCCACAGAGCCCAAGGCTGTGGCAAACCGAGCATCTT
CTAATGCCTTATGATCTCCCAAATGGCCTGCCTTAAGCCCCTAACTCCAATTTGATCTGTCAAAACAAGCACCTTTTCAA
GAGTTTCTTTTTTGTTCTTTAAAGCTCAAGTGAGATCTAAAAACTACTAAAATTACATTGGTTAAGGAGCAGAGGGGAGG
GTAATGTCCCTCCCATAACAACTGTCGACTCAACAGTAAGTGAGGCTCCAGCATTGCTGACATTCCCACAATGAAGACCC
AGCCCCTCTGAGCCCACTGGAGACACATGGATACTATTTTTAAATCCTTAATAAGTTGCAGGCTGCCCGCTAACTAGTTT
GCAGGCTACAGCAGGGGAATGCCGGACATAGCTGTTCCTGGAGGCCTTGCTGCTTCTGTCTGCGTACAGGCTCACCCATC
TGCCTCCCCAGCCTCCCAAGTTTGGTAAGAAGGAGGAGGGGGGAGGCTCCCCTCCCACCTGTTGCCTGATGAATTCCTAA
TTGCTGGGGTAGGGAATAACGAAACAGAAAGGTTAGAGAAGGGATGATAAATAATTCTCACCAGCTCATCCACCTCAGCAT
CATCCACAGGTGGTGCCTTGGGCTGAGACCTCCTGGTAGAGTGTGGCTTCTTACCCGGGCGCCGGCCTGACCTTGCTGCA
GGGGGGATGACGCTGTGCTTGCCCCGGATGGTCTTTCGAGGTCTCACTGAAAGAAAAACTATGGATTCAGCAGAGAACAA
CGGAAGGTGAAGGGAGGAGCATCTTCGAAATGGGGGAAAGGGATGCAGGGAGAATCCTTTTCCTGGACAAGGAAAGCAGT
GGCCTCCTTTGGTTTTAAGGTGCTCTTGCTATTTTATCTTGCCTTACCTAACAGGACACTAAAAGGCAGTGGCAGCCGAT
```

GAAGAATGGGTGCCTTTCATCCACTGCCAAAGCCCTTCAGAGGGCTAGGTCCTTCAGTACTTCTGTCCTGCCCCGTCCTG
CAGTTACTCCCAGCCATAAGGCCCCTGCAGGAATCAACTGTAAACTCTCACTCATGAGAAGCCATTTGCTAATAGGTCAG
CCTGGATTTCCCACAGCCCAAGCTCAGCCTGAGCGAGTGGATGTATTTACATGTAAGATGGAACTGGATTGTAAAACAAA
CCCTATGTTGTAATTCCCAGAGTTATTCTACACTTACCATCAGAATCTTTCCAGAGAAAAGCTGGTACTTCCATGAAGGG
CCACAAGGCTCCTCATTAAATACAATTTAGGGCTAGTCCCCTCCATAAGAGCCCTGCCCAGGATCCCCAATGAGAGAATA
AAGAATATAGGCCGGGCGTTGTGGCTCACACATGTAATCCCACCACTTTAGGAGGCTGAGGCAGGTGGATCACCTGAGGT
CGGGAGTTCAAGACCAGCCTGACCAACATGGTGAAACCCCGTCTCTACTAAAAATACAAAATTAGCAGGGCGTGGTGGCA
CATGCTGGTAATCCCAGCTACTCAGGAGGCTGAGGCAGGAAAATCGCTTGAACCTGGGAGGCGGAGGTTGCAGTGAGCCG
AGATCACGCCACTGCACTCCAGCCTGGGAAACAAGGGTGTAACTCCATCTCACAAAGAAAAAAAACACACACACACA
CACACAAAGGCACTGAGACTACTTGTGTCTCAGGTTTACAACAGGTGCTCACCATCAAGCCAACTGGCATTCCTCAGTGA
GCCCTGGGAAACAATGCCATGAGGATTTAGCTCCAGGCTCTCCACCACCTTCCCTTGGGGAGTGGGGAGATGACACTGGA
CACAGAGGTCTAGGAAGGCAGTGACACGAATAATCCAGAAACACTACTTTCCATTCTATTTCTGGATCACGATAACCCTT
TCTTGGCTACTTTAGCGTCTCTAGCTCAAGCCCAGGCTAGTAAGAAAGGGTAAGAATCTGATGCTTAGGTGCTAGAACCT
TGAGCTGCACTGGGGAACCACATCTTTGGTGGTCTGAAAGATAGAGTTGACAAAAAGCTCTGAAGTACCAAATACTTCCC
AGATCCTTTTGGCAAGTCGAGGCCGTAAAAACTTAGAAAAGCAATTCCTTGGAAAGAGAGCTACAACGTAGAAGTGGAAA
GCAATCACTAGCTTACAAATAAACAGAATCAGTATACTACCATATTCAGGATCTGCCCTCAGAGAGATCAGGCGCAGTGG
CTCACACTTGTAATCCCAGCACTTCGGGAGGCCAAGGCAGGAGGATCACTTGAACCCAAGAGTTCAAGACCAGCCTGGGA
AACATGGCAAGACCCTATCTCTACAAAAAAGCCGGGCATGGTGGCACGTGCCTGTAGTCCCAGCTGCTCAGGAGGCTGAG
GTAAGGAGGATTGCTTAAGCCTGAGAGATCGAGGATACAGTGAGCAGTGATCATACCAGTGCACTCTAGCCTGGGCAACA
GAGAAAGATACCCATCCCCCGCTCCGCCAAAAAAAATATGTTCCCAAACAAAGGTGTGCCCAAGTCACTGACTCTGACCA
ACAGAAGAGGATATTCCTGAATGCAGGACTGATGGGCCATTTGTTCACTTACCCTAGGATACCTGAAAAATCAGGCGTAG
TGAACAAACAAAAAGAGCCCATACGTATGTGAGAGCTTAAGGTGTCCCACATCACACCTCTGAAGTATGGGGGGGAAAGT
GGGAGGACAGGAGAAGACGGGCAAGCCCAAATCATTAGGTTTTGGCAGTCTCACTACAGTAAGAGACTAGTCATGCCAGG
CATCC

HUMAN mRNA SEQUENCE : hR5-030.1 (Seq ID No: 41)
CGGGAGGTTGCGCCGGCTCTGGCTCAGCGGCGGCTCCGGTGGCAGCGGCGGTGGCGGCTAGGCGGGCTCGGCGGCTCCTG
TCCCTCGGGCGGCTGCCCGCTCGCTGCCCAGGGCGCCCGGACACACGTGGGCGGCTCAGCGATGGCCCGCGCCCCGCGAC
GTGGCGGGCAGGCACCGGGGCGCGGGACCGCTGAGCCCGAGTGAGCCGGGGCCGCGCTCCCGCCTTCGGCCCGGGCCTCC
CGGGATGGCCGTGGCGCCTCTGCGGGGGGCGCTGCTGCTGTGGCAGCTGCTGGCGGCGGGCGGCGCGGCACTGGAGATCG
GCCGCTTCGACCCGGAGCGCGGGCGCGGGGCTGCGCCGTGCCAGGCGGTGGAGATCCCCATGTGCCGCGGCATCGGCTAC
AACCTGACCCGCATGCCCAACCTGCTGGGCCACACGTCGCAGGGCGAGGCGGCTGCCGAGCTAGCGGAGTTCGCGCCGCT
GGTGCAGTACGGCTGCCACAGCCACCTGCGCTTCTTCCTGTGCTCGCTCTACGCGCCCATGTGCACCGACCAGGTCTCGA
CGCCCATTCCCGCCTGCCGGCCCATGTGCGAGCAGGCGCGCCTGCGCTGCGCGCCCATCATGGAGCAGTTCAACTTCGGC
TGGCCCGGACTCGCTCGACTGCGCCCGGCTGCCCACGCGCAACGACCCGCACGCGCTGTGCATGGAGGCCCCGAGAACGC
CACGGCCGGCCCCGCGGAGCCCCACAAGGGCCTGGGCATGCTGCCCGGTGCCCGGCGCCCCGCGCCCTCCCGGAGACC
TGGGCCCGGGCGCGGGCGGCAGTGGCACCTGCGAGAACCCCGAGAAGTTCCAGTACGTGGAGAAGAGCCGCTCGTCGCGCA
CCGCGCTGCGGGCCCGGCGTCGAGGTGTTCTGGTCCCGGCGCGACAAGGACTTCGCGCTGGTCTGGATGGCCGTGTGGTC
GGCGCTGTGCTGCTTCTTCTCCACCGGCCTTCACTGTGCTCACCTTCTTGCTGGAGCCCCACCGCTTCCAGTACCCGAGCGCC
CCATCATCTTCCTCTCCATGTGCTACAACGTCTACTCGCTGGCCTTCCTGATCCGTGCGGTGGCCGGAGCGCAGAGCGTG
GCCTGTGACCAGGAGGCGGGCGCGCTCTACGTGATCCAGGAGGGCCTGGAGAACACGGGCTGCACGCTGGTCTTCCTACT
GCTCTACTACTTCGGCATGGCCAGCTCGCTCTGGTGGGTGGTCCTGACGCTCACCTGGTTCCTGGCTGCCGGGAAGAAAT
GGGGCCACGAGGCCATCGAGGCCCACGGCAGCTATTTCCACATGGCTGCCTGGGGCCTGCCCGCGCTCAAGACCATCGTC
ATCCTGACCCTGCGCAAGGTGGCGGGTGATGAGCTGACTGGGCTTTGCTACGTGGCCAGCACGGATGCAGCAGCGCTCAC
GGGCTTCGTGCTGGTGCCCCTCTCTGGCTACCTGGTGCTGGGCAGTAGTTTCCTCCTGACCGGCTTCGTGGCCCTCTTCC
ACATCCGCAAGATCATGAAGACGGGCGGCACCAACACAGAGAAGCTGGAGAAGCTCATGGTCAAGATCGGGGTCTTCTCC
ATCCTCTACACGGTGCCCGCCACCTGCGTCATCGTTTGCTATGTCTACGAACGCCTCAACATGGACTTCTGGCGCCTTCG
GGCCACAGAGCAGCCATGCGCAGCGGCCGCGGGCCCGGAGGCCGGAGGGACTGCTCGCTGCCAGGGGGCTCGGTGCCCA
CCGTGGCGGTCTTCATGCTCAAAATTTTCATGTCACTGGTCGGTGGGGATCACCAGCGGCGTCTGGGTGTGGAGCTCCAAG
ACTTTCCAGACCTGGCAGAGCCTGTGCTACCGCAAGATAGCAGCTGGCCGGGCCCGGGCCAAGGCCTGCCGCGCCCCCGG
GAGCTACGACGTGGCACGCACTGCCACTATAAGGCTCCCACCGTGGTCTTGCACATGACTAAGACGGACCCCTCTTTGG
AGAACCCCACACACCTCTAGCCACACAGGCCTGGCGCGGGGTGGCTGCTGCCCCCTCCTTGCCCTCCACGCCCTGCCCCC
TGCACCCCCTAGAGACAGCTGACTAGCAGCTGCCCAGCTGTCAAGGTCAGGCAAGTGAGCACCGGGGACTGAGGATCAGG
GCGGGACCCCGTGAGGCTCATTAGGGGAGATGGGGGTCTCCCCTAATGCGGGGGGCTGGACCAGGCTGAGTCCCCACAGGG
TCCTAGTGGAGGATGTGGAGGGGCGGGGCAGAGGGGTCCAGCCGGAGTTTATTTAATGATGTAATTTATTGTTGCGTTCC
TCTGGAAGCTGTGACTGGAATAAACCCCGCGTGGCACTGCTGAGTCCTCTCTGGCTGGGAAGGGGGGAAGGTAGGAGGG
TGAGG

HUMAN PROTEIN SEQUENCE : hP5-030.1 (Seq ID No: 42)
MAVAPLRGALLLWQLLAAGGAALEIGRFDPERGRGAAPCQAVEIPMCRGIGYNLTRMPNLLGHTSQGEAAAELAEFAPLV
QYGCHSHLRFFLCSLYAPMCTDQVSTPIPACRPMCEQARLRCAPIMEQFNFGWPDSLDCARLPTRNDPHALCMEAPENAT

AGPAEPHKGLGMLPVAPRPARPPGDLGPGAGGSGTCENPEKFQYVEKSRSCAPRCGPGVEVFWSRRDKDFALVWMAVWSA
LCFFSTAFTVLTFLLEPHRFQYPERPIIFLSMCYNVYSLAFLIRAVAGAQSVACDQEAGALYVIQEGLENTGCTLVFLLL
YYFGMASSLWWVVLTLTWFLAAGKKWGHEAIEAHGSYFHMAAWGLPALKTIVILTLRKVAGDELTGLCYVASTDAAALTG
FVLVPLSGYLVLGSSFLLTGFVALFHIRKIMKTGGTNTEKLEKLMVKIGVFSILYTVPATCVIVCYVYERLNMDFWRLRA
TEQPCAAAAGPGGRRDCSLPGGSVPTVAVFMLKIFMSLVVGITSGVWVWSSKTFQTWQSLCYRKIAAGRARAKACRAPGS
YGRGTHCHYKAPTVVLHMTKTDPSLENPTHL*

Table 8

MOUSE GENOMIC SEQUENCE : mD5-033 (Seq ID No: 43)
GTGGCTACTAAATAAGCCAACCTTCCACTCTGAAGAACTAGAACCAAGGGAGACAGAGAGCACACTTTGCAGTTAAGAGC
ACTTATTCTTCTTGCAGAGGACCTGGGTTCAATTCCCAGCAACTGCATGGCAGCTTACAAGCATCAGTGACTCCAGTCCA
GGGGCTCTGATGTCCTCTTCTGGTCTCCATGGGCACATGACACAAACATGGGGCACAGATATATGTGCAGGCAAAACACA
CACACACTAATGAAAAATAAATATATACAAAATATCTAGAACTGGAAGTTCTTCTGTAGAATCAGAGATAATAGAGGTAA
GAGATCAGGCAGAGTCAAGATGTATAGGGACCAGCAGTTATCTATGTGATAGAATGTTTAATCATGGATTTCTATGACAA
AGGTGATTGATTCTCTATACTGGCTGGTTTTGTGTGTCAACTTGACACAGCTGGAATTATGACAGAGAAAGGAGCTTCAG
TTGGGGAAATGCCTCCATGAGGTCCAGCTGTGGGGCATTTTCTCCATTAGTGATCAAGTGGGGAGGGCCCCTTGTGGGTG
GGACCATCTCTGGGCTGGTAGTCTTGGGTTCTATAAGAGAGCAGGCTGAGCAAGCCAGGGGAAGCAAGCCAGTAAGTAAC
ATCCCTCCATGGCCTCTGCATCAGCTCCTGCTTTTCTGACCTGCTTGAGTTCCAGTCCTGACTTCCTTTGGTGATGAACA
GCAACATGGAAGTGTAAGCTGAATAAACCCTTTCCTCCCCAACTTATTTCTTGGTCATGTTGTTTGTGCAGGAATAGAAA
CCCTGACTAAGACATTCCCTAGATTTCTAATTACTATTCCTATGTTTGTATGATCCTTATAACTAGGTGGTTGTGGCACA
TGCCTTTGATTCTAGCCCCTGGGAAGCACAGTTGGGCAGGTTTCTGAGTATGAGAACAGTTTGGTCTACACTGTGAGTTT
TAGGGCAGCCATGGCTACACAGAAAAGCCCTGTCTTGAAAAGAACAAAACAACCTCAAAATTAAAAAACAAAAAACAAAA
AACAAAAAAAAACCACAACAAAACCCAGACCATGTGACCCTCGTAACATTCCATAGAACAGTTTTTCATGTTCTAAGAAC
AAGGCAGTATGACTAACATGCTTGTGACACACATGAACACGGTATGAGCACATTATCTGTGGTGACAAGTGAGAGAGTTC
CTTTCCTAACTCTCACAGACCTCGTCAGAGGCAGATCCAAATTATGCTACAAGCACTCTCCATCCTCACCGTCAGAGGCA
GGTCAGATTTAACAGTTAACTCTCCATCTCTGAGACTCGAATGCAATCAACCCAACCCATCTGACACAGAACATGGACAG
CTGATCAAAGGCAAATTCACTCTCTCCTTGTGTATGTTAGTTATCTATAAGGTCTCAGGCGTTACGTTGGGTGTTGCAAT
CATAGACACAAGTTCCATGTACAGCTCAGATGCACCATATGAAACCCTGAACAATGGGGCCAGGGAAATGGCTTAGCTGC
TAAAGGCTCTTACTATCAGGTTTGATGAGCTGAGTTCAGTGTCTGAAATCCACATGGTAGAAGAGAACCAACTGCTGAAA
GCTGTCCTCTGAATGCTTGTGGGTGTAGTAGTTTGATCAAACATAGCAGGGGGGGGGGGCTGGAGAGATGAGATGGCTCA
GTGGTCCTGAGTTCAAATTTGAGCAAACTGCGTGGTGGCTCACAACCATCTGTAATAAGATCTGATGCCCTCTTCTGGAG
TATCTGAAGACAGCCACAGTGTACTTACATATAATAAATAAATAAATCTTAAAAAAACAAGGGGCTGGAGAGATGGCTCA
GTGGTTAAGAGCGCCGACTGCTCTTCCAGAGGTCCTGAGTTCAAATCCCAGCACCCACATGGTGGCTCACAACCATCTGT
AATGAGATCTGATGCCCTTCTGGTGTGTCTGAAGACAGCTACAGTGTACATATAGTAATAAATGTTTAAAAAACAAAACA
AAACAAAAACAAAAAACAAGAGAGAGAGAAGTCCAAAATTTGTCTTGCCTACAAGATGTGGAGGGATAAAGATGGAGCAA
GACAGAGGGAATAGCTGACCAGTGACTCATCCCATGTGAGAGAGCCAACCACTGACCCTATTAATGAGCCTGTGCTATTG
CAGACAGGAACCTAGCTTAACTGTCTCCTTAGAGGCTTCATCCATCAAGGCATGAAGGCAGATGCAGAGACCCACGGATA
AACATTAGGTGGAGCTCCAGGAGTCTTGTGGAAGGGGGGGATAGAAGTGAGCAGTCCTAGGGCTCAAGGACACCATAAGG
CCTATGGAGTCACAGAGCCTGGTCCACCAACCAGGGAGCACCAGGACGTGGACCTAGACCCCTACACATTTGTAGCAAGT
GTTCAGATTGGCCTTCATGTGGGTCTCCTAACAAGTGTGTGTGGGGGGGGGGGCCTGTCTCCGTCCCTGTTCCCTGCCTGG
ACCTCCCCGTTGCCATTGGGTCACCTTCCCCTTACCTCCACTGCCTAGTTGGGACTCAGTGGGAGAGGATATGCCTAGGA
CTAGATATCCCAGGATGGGGTGGTACCCAATGGGGACTCCCCTTCTCTGAGGACAAGGGGAGGGGCAATGGGGGAAGGGA
TTTGTAAGTGTGGGACTGGGAGGAGAGGAGGGAGGGGGGCTGTGATCCCAATGTAAAATGACTAACAAAAAAATTAATTG
TTAGGGAAAAAAAAAGTTAGAGAAAATTTAGAGTTCCCAATAAACACAGTTCTCATGACCTAAGTGGTAGATTCTCTTTG
GCATTCAAATTTTTCTGACAGAACTTTTCTACTCACATACAGTTTATCAGTCATGGTGAGGTTTGTGTCAATATACTGTT
TAACCAAGCGAAAACACAGGGCAGGATTAACTCATAGCTCCTCAGTGCCTTGATCACTGGTATGTGGAATGGCCTAACAA
GATGGCCAGGATTCATAACGACCAAGTTCAGTGTAATTATTGTTTGAATGCACATGTGATAGGATTTTAAGGTGATAACG
ATGTTTTGGAGTTGTTATATATCTCTTAATGACTTCTTTTCTTCATTTTTCTTTTATTTTCTGTGAGCGCCTGACTCGCC
AGCAAAGCAGATGCCACAACAGGATCCTTCTGCAACACATTTACTGGGAGAGCATTTTACTGTGTAGGCAAGAAGATCCC
GAGCCCAGAACTGGTGCTGCTTATATAGGCCTAGGAGAGGCGTGTCTCACACTCGGATTGGTTATGCATTCTACCTCATT
TGCATGTTCTTCATCTGATTGGTTATTCTTTCTCAGTACCTTGCCAAGCCTCATTATCATGCCTGGGCCAGGCAGTGACT
TTGTGAAAAACTTTACTGTTTATATACACATTGGTTGTTTACCCAGACTTCATGGTGGCCAGTGGTATCCAGCACCACCT
TGTAATGGCATATGTGGCTTCCCACATTTTCCTTGCAGACAGTATCTTAATATATAACACAGTTTGGCCTGCAACTCTCC
GTAAATATCCAAAGTTGGCCTCCAACTTGAGATCCTCCTGTTTTAGCTTTCCAGATACTGGGCTTGCTGGTGTATCTTAA
GAGAGAGGAGGCACTGAGGAAAGGGAAAGGGAGATAAAGAAAACACGTCTCAAAATATGAGTGAGTCATCACACAGCCTG
ATGGGACAGCTCAGTGGCTAAGAGCCCTTGCTACTCTCCTTCAGGACCCAGGTTCATTTTCTAGCACCCACATGGCAGCT
TACAACTGTCTATAACTTCAGTTCTGGGGGAATCTCGGCTGGCCTCCAAGGACATCAGGTACACATGTATCATGCATGCA
TAAACATACACACACAGTCCATGTGGGTAAAACCCAGACACATAAAATAAAAACAAATAAGCCTTTAAAAACATGCATGA
GTCATCACTGCTTGGACCCAGAATTACCACAGGTCCTTATTTACCGTGGATGGAGTCATAACTGCTTACAATAAAACGTG
AGTCTAACATTCAGCTTGACTTTTTAACACTACTCATTCTGAGAGCTCTGTCGGACTCTTTGATAGGATGTGGAGAAGTG

AGCTCTTAGGGCAGGAGTCTGTCTTAATGGAACAGCACTGTACAACATCCTTGGTTCAGCTGGTCCAGTAATCCCAGGAC
TGGAGAAGCTGGGGCAGGAGGATCACCACAAGTTCTAGGCTGGTTAGGTTGCACAGTGTGTTCCAGGATAGCCGGGGGTG
TGGGGGCATGGGGGGTGGGGTGGGGCACAGAGTGTAACCCACAGGGGGTGGGGGTGGGGACAAAAAGAGACAAGACACCA
TTGGATTCCTCAGCACCAAAACAGTAAAAAAAAAAAAAAAAAACCTTTATTCATTTCACAACACTGAGTTCTGTGGTGAGC
CTACAAGGTGGAAATGAAGGTGTGAAACCACTTTCTCCTGTCTCCACCGGAGTGCAGACTGATCAAGAAAGCACTATCTA
GCCAGACACTCTACACACCCACGTGACCCCAGCTACTGTTCAGGCTGAGGAAAGGCTGCAAGTTCAAGGCAGGGCTGGTT
AACTTTGGGAGAGTTTATCTTTTTTTTTTTTCCCCCGAGACAGGGTTTTTCTGTGTAGCCCTGGCTGTCCTGGAACTCAC
TTTGTAGACCAGGCTGGCCTCGAACTCAGAAATCCGCCTGCCTCTGCCTCCCAAGTGCTGGGATTAAAGGCGTGCGCCAC
CATGCCGGGCGGGAGAGTTTATCTTATGAAAGAGAAGGAGGAGGAGGAGGAAGAGGAGGAGGNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNAAACTCTGGATCTGGTTTTAAACCTTGAAGGAAGCTGCATTGGTTT
GCGTTAGTGTTTTTGTCTGTATAGAGATTGAGCCTAGAACCCTGTACAGTCTAGGCACTCTACCTCAGGGACATTTTCCC
AGTCCTGGTTTTGCATTTTATGTTGACTCAGTGTTTCACAAAGTTGCTCAGACTGGTCTTGAACTCCTTCTGTGGCCCAG
GCAGGCTTTGAACAGCAATCCTCCAGTCTCCTGCTCTTATCTGTTTTGGTCTGCCAGCCTGTGATACTAGGTCTGGATGA
AGCTACAGTGTTTTTGTTTTCTTGAGAAATAGGCATACATCGCTATTCTTGGCCTAGAACTGCTGTCCATTGCAACAAAT
GCCCTTAAGGGTAAGTTCAAAGAGCAGCACCTGTTTCAGGACTAAAGGCTCCAAGGTGGCTATGTCTAATAGAAATAAGA
CACACAAACAATTTGCTTCTTAATAGCCACTTTTTTTTAAAACAAAGTAAAAGTAAAAAGAAACACATGAAATGTCATTG
TAGCACGTAGTAAATATAAAATGTATCAGTTGTTGCTGTTGCTGTTGCTGAGACAAGGTCTCAGTATGTAACCCTGGCTG
GCCTGGAACTAGCTATCTAGATAGACCTGTCTGGCCTCAATTACAGCCCCCCTGGCTTCTGCCTCAAGGGTCCCAGGAT
TACAGATGACTCACCATACCCAGTTTCAAGACTTTCACATTTCCTTTTTCATTCTGATTTCTTGAAATCTAGTATACTCT
ATGTTTACAGTCCATCTTAATTTGGATCAGCACAGTTCACTGCTCCACAGCCTGCATGCTGTTCAACAGGAACAGGTATT
TTTCTAACGCAGCGGGCAGGTAGCCTTGAAAGGCTATGAGAGTAAGACCCAATGCTACTTGAGATGTCGTTGTGTGACTTC
TCACCAGCACACGATTTTAAAGGTTCCTAGGGAAAGTTTAAATGGCCTACACCTAACGCTAAACTTGTTATTTAGAGAGG
CCCTTATTTGTGACGGGGCTTCACACAATCCAACCTGGCCTTGGGCTGATCTTCCTGCCTCCACCTCCGAACTGCTGGGA
CAACAGGTGTGCGCCACTATGATCAGCTTCCTAGGTGACTTTACAGAATGCTATTTTCTGTGTACCTACTTGTAGTTCAA
TCTATGAGTTCAATATATGATGGAGCTCTGGGCAATAAAATACAAAGGGGTGTCTGCGAAGTCATAGTGGGAAGCAACTG
TCCTTACGAGAGTGACTTCAAGAGGAATGCTGTAAGCTACTCACACTCCTTACCTTTACAGATGTTAAAGATTAAGTAGC
ACAGGCTGGCTCCTGTCTCAGCCTCTTGAGTGCTAGGATTATAGGCATATGTTACCCTGCCCGGATGTCTCTTTCCTTCT
GTTTGGAACATTTCATATGGAGAACACGTTTAGGGGGGCGTGGCAATTGCCTTGGGATAATGACAAAGATTTTTAACAC
AGTAAGATAACAGAAGGGTTCTTTTCTTATCAGAGGTGTACATTTATGAAGCCAACCATGCAAACAAAGGGCAAAGGGAG
GCAGGGACAGAGTTCAGTGGACTCTTCCTAAATAGTAACAACCCAAACCTACCCTTCTTCCTGCTGCAGCTTCTTTTGCG
GAAGGCTCTGTCCATCATTACTACTTCTTCTTCATTTATTTATTTATTTATTTATTTATTTATTTATTTATTTATAGACA
AGGGTCTATGTAGTCTTAGCTATGCATATGTAGACCAGGCTGGTTTCATACTCACTGAGCTAGCTCTTCCTGCCTCTGCC
TCTGGAGTGCTGAGATTAAAGGTGCATGTGTCACCACACCCAGCTCACGTCATTCCTTCTTAAAGCATAACAAGGGCCTT
GTATGTCCGAAAGCATAGAACTCAAAGTGACAGCAAGGATTTTAAAAAGAAAAAAAAGTTGCCCACCCTGCCCCCACCAG
AAATACCAAGAACTTGTGTGTATTTTGTCAATACATAGACACAAATGACAGCAGACTTTTTAGTACTTTATGAGGTCATG
GCAATCACAGGCAAATCTGCCTAAAAAGTTGCTGCAGAAATGACACTGTCAGTTTTTTCAGTTACTGACTGTAAAATCT
TTGCTAGGTATATTTTCTGAGTTACACGGGAGACTAAGTGGGTGACCATTACTCTGGTTTGATTCTTCACCAAAGGATTC
CTTTTCAATGAAGAAAGACATTTTGTTTGATCACTTACCAGGCCTACACTAATTTTTATCACATGTAGGCAAGTTAGAAA
CTCAAGGCTTCACTTTCTTTTAAATCCAAATGTCTTACATTTAGTGTTTGAGTGTAAAGACACTACAGCCCACATGTAGA
GGCCAGAGGACAATCTTTAAGAGTAGGTTATCCCCTTCCACTGTGTTCATCCAGGGGATCAAATTCAGGTCATCTGCACT
TGGTGGCAAATGCATATACTCACTGAGTAGTAGTGACAGCCCAGCAACCTTTTAATTGTCTTTTTGTGTTTTCTGTTGTT
ATCCATTGCCAGGTTGTTTGAGACAATGTTTTGCTGCACTCCTTAGGCTGGCTTTGAACACCTGAAACTCCTAAGCCTCC
CAGAAACTAGCTTTGAAGATGAAGGTCAGACTTTTATCTCTTGAAGAAAAAAAAAAAAAGACAAAAAAATTGTTTGCTGTG
TGCATTTACATGCATCACGTGTGGTTCCCACGCTGGCCAGGAAAGGGTATTGGATCCCCTGGAGTTGGAGTTCTCAGAGT
TTATGAGCTGCCATGTGGGCAGTAGGAATTGGACTCCAGTTGTCTTCAAGAGCAGCGACTGCTTTTCATCGCTGAGCCAT
CTCTGCAGCCCTGATGGACTCTTCATCTAACTCCCATACAGACTTTCCATTAGAGCCAGATTATACTGTTCAATGCAATC
CTTTAGGGTGGCTAAGGTGGCGTACCTCTGTAGTCCCCGCACTTGGGAGAATGAGACAAGAGGATTCCAAATTTGAAGCC
ATTCTCCATTACATAGCAAGACCCTCTCTCAAAAAACAACCAACCACTACAATCTGCAGTGATAGCTTCTTTGAGGACAG
ATGTCAGTCTTAACAAAGGAAGCCCTTTTTGAGTTAGTCTAGTCATAAATATGAACAAGAAATTCATTCACCCTCTCTGT
ATTTTCTCTCTTATGAAGGAAATGTCAGGTCAATATACGTGTGGAAAACTGGTTATCATTCTACCTCAAGTAAGCTATTG
TTCTTATCTATAATAGAGAACCTGTTTCTTTGTTTAAAACAGCGTTTCATGTAGCCCAGGCTGGTTTATGACTACGTAGG
TGGGGATTGCCCTGAACTTCTGATCCTGCCTCTATAGGTCTACCACCAAGTCCCATTTTACTCAGTTCTGCATAAATCCG
TGGCTTTTAGGCAAGGAGCCTACCAACTGATCTACAAACCCCATTTTTTTTCTTCTTCAATGTACCACCATACCCAGATT
GTATAGGAAAAAAATGTTCAAAGTATGCTAAATTTTACTATTATATTGAACATAAAAAAAAAAAATCCGAGGTGAGATG
ATAGCTTCTCTTGAGACAAATGTTGGAAGCATGACAAGTCTTCAAGTTCTGACCTCTTCTGTTCACATACATACATTTCC
CCCTTCTTACTGGTTAAAAATCTCACTTTTTCTGGTAGAAAAATGTTACAAATCTAAAGACTTCAATCTCACCGGTCAAA
AAATAGGGTCTAATAAAATTGCCAAATAAATTCCATTGTTTTTGAAAACTAGAAAAATGCTCAGGCTTAGCTAAGTTGCCA
GTTATTAATTTTTTTTTGAACGTACTGGAATGTATCAATAGAAATTCCTTCACAAATCAGTTTGCCTCTACTTGCAAATGG

```
ATAACCATATCTACAATAATCTATGCTTATCAGATAATATTTTATGTGCACTGTCTGAGTCTATTTAGAGCGGACTTTGA
AGGGACATTCAGACTCTTATGAACTAACTGGCTTCTGCTTTAAAGGGCTACTGCTTCTAGTGTCAGCTCTTTCTAGTTTC
AAAATTTGCATGGCCGCCTTTCTGAGTCGCCTTCAAGAACAGTCTTAGCCCAGTCAGGAATTGCTCGAACTCCGGCGGCC
TTAGGGTCAGGCTTATTTGCATCTTTGCTAAACAAAGTCCATCCTTTGAGGCGTCAGCGCCGCCGGTCTTCGCTCTCACG
CTCCGCTGCTCACTTCAGCGACGGTGGCGTGCACAGCACCTGGGTCATCTGTGCTACTCGCTCCCTAGTCCCGGGACTAGG
AGGCGCGGTTTTCCCGGAGAAATCCACGTCAGCCGCGCAGGCGCAGGCGCAGGATCTGGGCGGAGGGAAGGGGCCTGCGC
TCGGCCCGCGGTAAACAAGCACTGCGCAGGCGCCGTGCGCCAAGGTGATTTTTTTTTTTTTTTTTTTTTTTGCTTTT
TGGTTTGCATAGCATCATAAAAAAGGGGGAAGGCCAAAGCAAACCCTAGCATCTTAAGACGATCGACGGTGTGGCTTCTG
CTACTCTCACTCTGAGGCACCGGCTGCGCGACCATGGCAGCCATAACTCTTCCACAGAGCTGCCGGCCTTGAGCTCTTTC
GCTCACTGTCTACACTGAGGCTGAGAGGACAGAGAAAAAAGAAGAAAGAAGAAGATTTAAAGGAGAGGTGGTGGGCGG
TTCCCACAACGCCACCGTAAAGACCTCAGCGCCGCTCTTTCCCCAGACAACGCGAGATCCTGTGCCAGGCCGGGACTCCG
CCCCCGCCCCGCCCGTGGCTGTGTGCGCATGCGCCGGGCTTTTTGGCGCGCAAACCGTTCAGAACTTCCTGCTTGGTCAC
AGGTGGCCCCGTCGGCCCCCGAGGGATGTCAGTCAGGTCGGAGCCGGTGATTCACGCCCACTGCGGCACGTCCTTAGGGA
TTTTTCGGTCAAAATTCCACTTGACTGTAACTCGCCCCCCCATTCTTCTGTTCTCGTATGGGTCATGAGGTCTCCGCGAA
TCCAAGAGTCAGAGTGGGAAGAGAGCCCCTCGCGGTTTTTAAAGATGTTGCGATTTCGTTGTGTGGCACGCGAGCTAAC
TGGCATAAAAGGATAAATCTGGGGAACGAACGGGACTTCCCCCCCCACCCCCGAAGATTCTGAGAGGCTGTGAATGTGGA
CCGGGCTAACACAGTGATATGAAGATGAGAAGTTTTAAAAATGGAAGGAGCCAATGAAGGCTTGAGAGATGAGGCTAGGA
GGAATTAGAGCAGTAGAGGAAGTTTAGCTTAAAATATTTACGCATATTTATAAGCAAGTAGGCAGCAGTGCCGCATATTT
AGTCTACGATAGCGAAGTGAAATGGGGATGTCTTTGAGTGCAACAGAAACATTTTTAAAGTTGTCGCAGGCAAGAGCTG
AGGTTGGGGCGGGGTAAAACAGATAATCTGGAGTCTTAGGATGTTGCAAGTGTGGACCGCTCTGACTGATTTGGCCTCTT
TTCTCTCTCAGGGGACGTGGAACACCTGCACAAACTGGATTTTTGCCCTATCACGACTCTGAGGTACCATCCGGCAACTG
AAGTGTCCTCTTTCCAATAGAATGCCTGGCCTCTCCCACCAACTTCACGGTTCAGCCGGAATGAAATTTGGCTAACAAGT
CACTCAGATGAGTCACTGGCTCATTACAAGATTATAGATTTCTAATCAATAGCCTTCCAGTTGTGTAGATCTTAGGATTC
ACTCTAATATTGGGAGCAGTGCTATATAGTACAGACAGTATTGTCATTTTTACTGTCCCTGGAGATGGTTTGCAGGACCT
TGCACAAGTCCCGCAATGCATGATGCTCAAGTCCCCTGCGTAATGAGGTATACTATTTGCATATGACCCTTGTACATCTT
TCTGTAAAGTTTAAACAATCTCTAGATTATTTGTGATATGTACATCGATGTAAATGCTATGTAAATAGCTCCTGTACTAT
ATTGTATAGGGAGTAATGGCAAGAAAAGCATTCAGTACAGATGCAAATTTTTCTTCTCTATTTTTATATCTGCTTTTGGC
TGAATGTGAGGATGTGAAACCCATGGATGTGGGGGGGTTGACTCTTCTCTCTCTCTCTCTCTCTCTCTCTCTCTCTCTCT
CTCTGCCTGTGTGAAAGAGTTACCTCACTGTATTGATCAGATACGCCTAAATGGACTAAAAAAAATCTAGTCTCTTATCT
CAACTTTCTGAATAGCTGACACTCCCAAGTGTGTGTTGGGTACAGTTCTGGATATATTTTGATCCTTTGTGTGCTTTATA
AAGAAAATGCAGGAAGATTGACGGGATATATAATTTGACTAGTCCTAACAACAGAGTAGCAATATAGCTTCCTTTGTAAG
AAAACAAAGAATGGAAGGTCTTAGGATTAAAGCTGGCAGTGATGGAGAAGGCTCTAATTATTAATGTTTAGAGGAAAGCC
CTTGGAAACCAGGTATAGGATCATACACCTATAGCCCTAGCTACCCAGGGATGAGCCAGGAGGACTGCTTGAACCTATCT
GGGACATAGCCTGGGGAGGGGATGGAGAGAGGAAGGAACGAGGGAAGACAGAACCAGACCCTCTCAGACAGTTCCCTGTA
AAGCACTGTATTTTGGCTGATTGTATTCTGTTGTGTGGGTTGTTTGTGCTGAATCCACAGGAAAATGAAGGGGAAACATG
GGTACAATATAATAGGACTTCTGGGTTGACATCTAAATATTTGGTTTGGGGGATTGGGGTCATCCGTCATCTAAGTGTAA
TGTCATAGGTACTGTTTAGAACTGGGGATCAGAGCCCTGAGGTGTGACCTTTGGATCTGCCGGTTACACCTTATGACATC
TCATTTCTTCATTTATAAGTAAGGATTACAGTAAAAGGAGAATGAGGATGAGGGTATCTTTTTAACAGTGACTTCATTTT
ACCTTGGTGGTATTCTTGCCTGCATCATATGTTTGTGCACCACTTGTGTTCTTGGTGCTCTGAGAGGCCAGAAGAGGACG
TGGAATCTCCTGAGTTACAGATGTCGGTGAACCTTCCTGTGGGTGCTAGGACAGAATTCAGCCTCTTGGGCTTGCTTTCT
GGAGCAGAAAGAGCCTTTATCTGTTGAGCCATCTCTTTGGCCCATCAGGTTTTTTTGTTTCCTTGTTTTTTTGAGACAGG
GTCTCTCTTAAGTAGACCAGGCTGACCAAGAACTCACAGAGTCACAGAGATTTTCTTGTCAGCACACAGACCTGCATCTG
TGTCATCTCTGCAGCTCTAGACACTATCTTTCAGTGGGTCCCATTTTGTAGGGAACCAAGCATACATGTGAACGAGTCCT
CGTGGGACAGGCAGGGATGCATGAGACATTGGTCTCTGTCCTAGTTGTTCTGTCTTTACACTTTATTATTGATGTGTCTG
TGCTGAAATTAAAGGTGTGCACTACCATGCCCACCCTCCATCAGTCTTTTTAAGCTACTATTACTACTATTGCTACAGTT
GAGAACTCCTGCTTAAAGAGTTTCTGAAGAAAATGAGAAGAAGCTAGAGCTAAGGCTTAAGCCCCTCTGCTCTGCACAGT
TATCTATTTGAAGGGATTCTGAGGACTAAAGCAATGGCTTGAGATGAGCAGGCGAGTCGGGCACAGTGACATAGATTCTG
GAGGGGAAGCGCTGAAATAAGCATACTAACTTGGGAGCAGTTAGTGTCGTCATTACTGTGGATGCCAGGAACACACTGAA
TTGGCTGTATAGCAGACTTTGAACCTCCCAAACTGGCCCTTTGATCTAGCTCAGGTAACAAAAAGATGTCTTGCTTTCTT
TGCCACCTGGAGCTCTTCTGCTCCTCTGCCTGGGGAAGGCATGATTAAAGTGTTTGCACATGGGTGGTCTCTCCTGGGAG
GGCAGGTGGGAATGCTTTTTTTTTTCTTTTTTAAAGCAGGCATTGATTATGAAGGCTTCCCGAAGCCTGTCAAAGGTATG
AGATTCATCTGACCACACTGATGGTTTTGTTGTTACTGACATATGGGAAAAGTAGGGTTTAAGCCTACTTTCATAGCCTT
TCAATGAAAACAACATCACTTGGGAGTAGGGAATGTCTGGGAGAGAAATTTTTCTTTTTTTCTTCTCTTGTGTGATTTTG
GACACAAGATCCCACGTGTCCTAGGCTAGCCTCTGAGCAACTGTCTCACCTGCCTTCACGCCCTAAGTGTTAGAATCATA
GATATGTGCTGTCACACAAAGTTTGTCATGTTCTGGAGATCAAACCTAGTGTTTTGTACATACGTCCTACCTAGGTAAGT
AGGTAGGTACTGTTCAAAGCAAGCTACATGCCTCCGCCCCAGGGACTTTACCTTCTTACACACAAACTGTTTGATAGGAC
CACAACTCTGACTTGTAGTCACAGTTGAAAACACCATTGTTGGACACTATGACTAAAATGTAGTAGTTATGGCCAAGAAT
ATCTTTTGCCACAGATTGCATTCTTAAAAACTTTTTAATTATGTGTTTGTGTGTGTGTGTGTGTGTGTCTGTCTCTGTGC
ATGTGAGTGCAGGTACCCTCAAAGATCCCAAGTGTTGGGTTCCCTGGATTTGGAATTACAGGCACTTGTGAGCTGCCTG
GTTTGGGTGTTAAGAACTAAACTCCGTTCCTCTGGAAGAGTAGCAAGTGATCTCGACCTGTGTCATCTCTGCAGCTCTAG
```

```
ACACTATCTTTCAGTGGGTCCCGTTTTTGTAGGGAACCAAGCATACATGTGAAAGATTGTCCTTGTGGGACAGGCAGGAAT
GCATGAGACATTGGTCTCTGTCTTAGGTTGTTCTGTCTTTAAACTTGAATTATTATTAATGTGTCTGTGTGTGTGTGTGT
GGGGGGGGGATCAGGACAACTGTCTGGAGTCACTTTTCTCCTTCCCCTTTGTGTAGGTTTTAGAGATCCAACTCAGGTTGTC
AGGCTTGCGAGGCAAGCATTCTTCTTAGGCAGCACTTACTGCCCTTCACAGGTGACTTTTATGAGTTGGAATGAGCCAAA
GACAAATAACACTTGAGGTTCATGACTGATTTTTCATCCGCTGGAGACCACCAGTGCTGTCGAATTGGGGACAAGAGAAG
GGGGGGGGGGATGTAGGAGGAATGGGGTTATAGCTTAGTAGTAAACACATGCTTAGTATTTGGAAAGCCCTGCGTTCCAC
CACCTGCACTCCCAATTAACAAAAATTGGTAGGTTTGTGTTTTCTTTGTAGATGTGAAACAATGTCTTTTGGACTTTTTT
CAGTTTCATACTATACCAATATGAAGTTCTGGTCATTCATATAAGGGTGTCCAGTCCATCTGTCTGCTTCTTTAGGGCGT
GTAGAGGAATTTTAATCCAGCAACATGGCTACTCTGGCTAGGGATCATGTCCAAAGACCTTCTAGGTCTATATGATCTAG
CTGGAATGCAGACATGCCCTGGGTTACAGTATGATCTGGATGGAATACAGACATGCTCTTAGTATACACCTTAATACACT
TTAATCCCTAACAATGAAGGAAAAGGTAGTTTGTATAAGGAAGCAGCCATGTTTGAAAGTGACATTTAATAGAAGGGCAG
AGTGATGAATCAAAGAAAGATTTGACAGAATGAGTCAGAGATCATATACATCCAGCTCACATGATAACAGCACAAGAAAG
AGAGGCTACTTAAGAGCAGAGAGAGAAGGAGAGGTGAGGAGATGGCAGTTTTATGTCTGGACAGTTTTGCAGAGGCAAGC
TGTGAAAGAACAAGCTAGACACAGGTGAAGACAGACGAGCCAGAGAATGAGAAGGAGCCAGAAGATTAGAACAGATTGCC
AAGTTAGAATGTGGCCAAGCAGACAATTCAGCCAGAAGCCAGAGAAACTGGATTGAATTGGTCATCTTGGAAGGTTAGAC
TGTACAGAGGCTAGAAGCTCCTAGGCTTAGGGTTAGAACAGAAAAGAGACACTCTGGGCTCAGCCTCAACTGTGTATTT
GCATAGCGTGTGTACAGCTTGCATCTCATCCCATCATCTGAGGAAATAAAGAGACATTTATGAGGAGGGGGATGTTAGAA
TTAGGATTCTAGGGCTGGGTGATGGTAGCACATGCCAGCACTTGGGAGGTAGAGGCAAGCAGATTTCTATGATTCTATGA
GTTTGAGGGCAGCCCGGTCCACAGAGCAAGTTCTAGGAGACCCAAGGGTACACAGAGAAACCCTGTTTAAATTCCACTCC
CCCCTCACCCCCTCAAAAAAAAAAAGGAGAAAGAAAGACAAGAAAAAGAGCTCTAATGAGTACTTTCCTTGGTGCAGTG
GACTTTTTTTTTTTTGTAATGTGTTATGTTTTTTTCTACAGAAAGCATTTGGGTGTTGGCACTATAAAGATTGAGGTTA
TTTTGTATTTGCTGTTATTGGAGTTGCAGCTTCCTATACCAAATGCAGTGTCATCCTGGGGGTCTGGCAGCTAATGACCT
ATGGACAAAAGACACCCTCTAACCACAGTTGGCTCAGCTGAAAGTAGAAGATATCAGTCTGTGATAATCCAGTAAGTGTA
GCATCAGGGACCCCCAGCACTCCAGACTAAGAACAAAAGGGAACGGTCTTCAGAACAAAAGGAGGTTGCTAAAGTGGGTA
TAGGGGAAAGACAGAGGCTTTACTGTGTCTCACTGGGATAAGAGGCAGAGTCCACAGCCGAGTAGGACTAGAACTGTGAA
GTTCTGAAGAACATAGTCTGCAGAGTTGGGTAGAAAGATAGGAGAACTGGGGTAAAGAAAGAAGCAGGTTGAAAGTCACA
GGAACCTTAGAGGGGGGCTGGAATGATGTAGTCAACTGGCAGAGTGTGAGATTAGCATGCATGAAGCCATGGGGTTAGTCC
CTGTCATTGATGATGCATATGTCATTCCAGGCTTACATAGAGAGCCCTCAAGTCTAGCCTGGGCTACATGAGACCCTGTG
TCAAAAACCAAATCAAAACCTAGACAAAATACTCTAACTAGTAATATGTTATAAGTAAAAATATCCAGGAAGATTAATGG
GACTACTCACATTTTAGTTCAGGAATAAAAGAATCAACATATACAAGATGTTTTTAAAGGAACAATGAAGAATAAAAATT
TGCTTCATGGGAATACCCAAATTCACATTTTTGATAAGGTTATTGATAAGGTCATTGAGCACCTAGTTAGCTTTGAATTT
TTTTTTAATTTTGTGGCAGTTGGAGAGATGACTCAACAATTAAGAGCACCCAGATTAGATCCCCACTGCCTACACCGTGG
TCCACAACCATCTGTAATTCCAGTGCCAAAGGACCTGACACCCTCTTTTGGCCTCCTTCAGTCTGCACATTGTGTGCAGA
CAAAAATGCTCATAAAAGAAATTTCAAGAACATCTTAATTAATTATTTTTTTTTAATCTTGAAGATGTGAAGATTGGTTG
GGTGAGGATCCTCCCTACAGATCTGTGAACAAGACGTAATACATGACAACCAGGGCGGCTCATGCCTCGTTCTAGCCCGT
GGGCAGCAGAGCAGGAGGATCAGCAGTTCCAGGTCACCTGAAGTGACTCAAACAAGCTCTAAGCCATTCTGGGCCATATG
AGACACTGTCTCAAAAAATAGCAGTAATAGCAACAACCCTTCTAACCAAAAGATTGAAATGCAATAAACTCAGCAGTAGA
AGTACTATGTGTGCAAATTAAAGTAGTACAGAGTGAAAAGGGAGATGTGGCTGGGGGTGTAACTTACTATAAATGAATGC
TTAGCGCGCCCTTCAGCTGGGTCAGCCCTCAGCCTCAAAGATAAAGAGCAAGGACATGAGTAACCTGATTTGGAGGGCG
CTGTATGACTTACCTTTCTGTTGCTGTGACCGAATACCTGACAAGAAGTACTTTGAAGAAGGAAGGGTTTACTCTGGCCC
GGATTTGAAGGGATGTAGTTTAGCCTGGAGAGAACTGCATGAGCGTGAGCTTGAGGGAGTCACATTTCATCTACACTCAG
AAAGCAGAGAGGCGAGTGTTGGTGCTCAGCCTGTGTATGTGTGTGTGTGGGGGGGTGGCCTATGCACTTATTGGAAGCT
CACCTTGTTCTTTGAGATAGTCTCTCACTGAATCTGTAGCTTACCCAGCTAGCTGGGCCGGCCGGCCAGTGAGGTCCAGG
GATCTGGGATCCTGCCTTTCCAGCACTAGAATTACAGATTCTTGCCCAGTGTCTTACGGGAGTGATGGGGATCTGAACTC
TGGCCCTCGTGATGGTAGGGCTGCTTCTTCATCCAAGGAGACACCGCCCCAGTTCTGAGAGGTCCCCTGGTCCAGTCAAA
CTGCTACTTAACCAAGATAGGGGGAAGTGAATGAGACACAGCAGCGGGAAGATTATCTGAAAAAACAACAGATTGAGAGA
CCAGTCTGGTTTTAGAATGGACAAGACAGAAACTTTTAAGCCTAAAGCATGAAGGCTTGAGAGAGAGAAGTTAAAAAAGA
AAGAAGAGGATTTTAATGTATTTCCCAGGGACACTTGGATAGTGGTTTTCTCTCAGGTAATGTTTGAAACAATGGGGAAG
GTCCTTTTTATTTTTAAATCTAAAATTGATTGTGTTGACTGTGTGTATCTTTGAGTACACATATTACATACAAGGGTGTG
TGTGTGTGGTGTGTGTGTGTGTGTGGTGGTCAGAGGACTACTTGTAGTCAGTTCTGTCCTGCCACATGGGTCCTAGGGCT
CAAACCCAGGCAGTCTGGCTTGGTAACAAGCACCTTTACAAGCAAGCCATCCTGTTCCTGTCACTAGAAGGATTTAGTTA
CCCAGTTATTTAAACCATTCACTTCTTTTGTTTGGTCATCAATTGGGCTTATAAGTGATAATGAGTTTGAGGACAGTGTA
ACCCAAACAAAACTAAACAAGTAGATAATATTTATTCAGCTTATGGTAATTTCCTAAACGTGGCAAATGTTAAAATTAAG
AGCTGAATTTCTAACGGGGTGTGGGCATATACCTGTAAACCCCAAACTGGGAGACAAGAGCAGAAGGATCACTAACTGGA
AGCTAGCCTGGGCTACATAGCAAGACTGAGTCTTATATTAAAAGTACTTCACCTGACCCCTTTCATAAACTAAGAGTAGA
TACTGCAGGGTTGGTGAGGTGACTTGGTCAGCACAGGTGCGTGAGCGCGAGTTTGGATCTGTAGCACTCAAGGCGAAAGC
TGGAGCTGGNNNNNNNNNNNNNNNNNNNNNTGGCCTTGAACTCAGAAATCCGCCTGCCTCTGCCTCCCGAGTGCTGGGATT
AAAGGCGTGCGCCACCACGCCCGGCCCTTAGTTACCCAGTTATTTAAACCATTCACTTCTTTTGTTTGGTCCTCAATGGG
CTTATAAGTGATAATGAGTTTGAGGACAGTGAAACCCAAACAAAACTAAACAAGTAGATAATACTTATTCAGCTTATGCT
AATTTCCTAAATGTGGCAAATGTTAAAATTAAGAGCTGAATTTCTAACTGGGTGTGGGCATATACCTGTAAACCCCAAAC
TGGGAGACAAGAGTAGAAGGATCACTAACTGGAAGCTAGCCTGGGCTACATAGCAAGACTGAGTCTTATATTAAAAGTAC
```

```
TTCACCTGACCCCTTTCATAAACTAAGAGTAGATACTGCAGGGTTGGTGAGGTGAATTGGTCAGCACAGGTGCGTGAGCG
CGAGTTTGGATCTGTAGCACTCAAGGCGAAAGCTGGAGCTGGCTAGCTGGCCGGCTAGCAAATCAGTGAGTTCCGGGTTT
GGTGAGGAAGTGACCATCTCAGAAAACAAAGATTGCAGAGAAGGTGCATCATCAGCCTCTGGCAACTTTCTGGAGTTGCT
TCTGCATGTCCATCCCAGGAGTGAACTTGAATTGTCAGGCCTTCCCTGACAATTCAACACACTGAAATAAGCCACTTTAA
ATTCCTGATTTGGGGTAATCCAAATGCCCTCCAATTATTATCTGAATTTATGTTTAAAAAATAAATTTAGCGGTCTGTAG
ACAAATGTATTTTTATTTTTCTTATAAAATGGCAAAACATTTTCAACTGGTTCTTGTGGTCAACATTTAGTTGTGTTTCT
TCGATATCTCTGCAATATGCTATTAGGAGTTGCATGAATGGCTTCCAGACTGCCGACATCTAGTCTCAAAGAGAAATGCC
TTTCCTGTGTTCTTTCCACTGCCCTGTGGAAGCAGTCCGCACATGCTCTATCTTCAGTTCAGTCCAAATAGATTTTTAAG
GAGAGAAGCAAGGGGGGATGGGAGGACTTCCTGTCTTAGTGTAATTGTGGGTTTCTAGAATCCTGGCAGTGTGCAGGGCC
ACAGCAGTCCAGTCAGGAAGAAGTCTGCAAGGTAGGTCTGGTCTGCAAATGCTAGCTGAAAGCCTCTGCAACAATTCGG
TTCTTTTCCCTTTTCTTTAGTCAGCAGGGTCCCATGGTGACCGGTTTTGAAAGCAATAGTCCCCTTCAGCAGTTACCTAG
CACCATGGCAACCAGGACGTCAGCAAAGCAGGAGAGACCCACACAAAACAGCTGCAACTCGGCTTCTATCCTGCTTCCAA
CTCAGCTCTGCCTGAGAGGCCCACTGATGGAGCTAAAACCATCTAGGCTTGTTCACCCCAGCATTCCTGTGTGCTGAGGG
GGTTTTCCCATAAGCACAGGTGTAGGAGCCTATAGTAAACTATGTCTTGAACCCCCACCCCTGTAAAAAGAAAAACATAA
GAGTATATTTAAGGGGTAACAGTAAGTTGTAGGATAGGCTAGAAAGCAGGTATGAATTTAGTAATATAAAAACCTGATTT
GTCCTCCTAAGTGAAGTAAGATAACAGTAGAGACAGTTAGAGCCCTAGCTATATGGGGTGGTTTATATTTAGATACAAGA
TCAGGGGGAAACTTTAACAAATATCTTTATTTACCTGAAATAGACAAATGGCTTTACGGACTGCAATGCTGTCCGTGGTG
CTGAACAGTTCATCGACCAGATGTGCTATAAGGGTACCCCCCAAAATAGAGTAAGGAACACAATGGCATATCCAGATGCT
GATAATAGAAAGAGGTTGAGGAATTGGGAAGGAGGCTTGATTTTCTTCCCGTATGAAGCAACTCTGAAAGCGCAGGGTAA
AATGGCCAGATTGAAAGGAGAAAGTGAGGAAAGCCAGAGGCCTGTGTGCCCGAGTCTTTCCTTTTCATATGGAGATTTTT
CAGATCAGAGCCAATCAGGTCAGAGATTTCTGCTGCTTTGCTCCTGAAGAGGAGTGGGGGAAGGGAGAGAGAAGATGG
CCACATTTCCCCCCCAAGCCACATGATCCGTTTCCCATGTGACACATTCCAGAGCCTTGGAAAGGAGAAGGGAGGGGCAC
TGAGAGAGTGGGGCTGGGGGTGGGAAGATGGTTTTAGCAGTGAACCTTCACAGCGTCATCTTCTCTCTCTACCAGGAAGCA
GGCTGAGGGCTGAGGGAAGGCAAAGGGGGAGATATGAATGTGTTCCGAAGGTTTCCCGGTGGCTCATGGCATCCTCCAAAG
TCCTGCCTGCGAGCTGTTCCTGACCAAGAAAACCCGCGGAGCCTGACTCTGAAATCCAGTCCCGATGAAGGTATGTGTTC
AAGCTCCTCTATCACCGCTGAAATGTGGCTCAGAGGGACAATACCGTCTCTTCCTATTTTTTTTTTCCAGGAAATTTGGA
GATTTTTGTCCCGGCTTAGATGTTTGTCAGGGGTGTTAAATTTGTTACAGGTTCAGAAAAGATGTCTAGCTAGTTCTGTA
AAATAAATACTTCTCCAGAGTAAGCTCCTATAGGGGAAAACAAAATCGGTTGAAAATCGTGTATAGTAATTGGTAATAAA
ACCAGAAACTTCCCTGGTGGGGGGATTTCTCCCTGTCTAATCCTAGAAGAGGAGGAAGCGTTACTCAGAAGAGCTAGATTT
CATTGTCACTAAGAAAACCCTGCCTCATCCCTTTGAGCTGCTGCTCTGTGTCTGTGTTCCCAGAGGTTACTGCCTGTAGC
TTAGGAGCCTGAGTGGGCAGTCCTGCCATTCCTTTGTGCTGTTAGCTCACCCTTCTCCTGATGTCCCCAATACCAAGAGC
ACCAACGATTGTTCCCAAGCTCTGCTGTAGACACTAGACTGCCACAGAACCCCACACGGTATGGGAAGGAAGCCTTAGGG
AGCTCTCACAGGAATCTGCCAAATGTAATACAGTCTGTCGTACACTCCTCTGGGTTTCTTTGAGTATTTCTCCTCAGGTC
AAAGAGAAGAGCTAGCAAATTCACGTTTAATTTCATTTAATAGGTACTTGGAAAAGTTCTTGGTCTGTGCAAGCAATCAT
CCCAAGCATGTAAGTAATCATACAGATTGTAATCTAGACATAAACTCGGGCACAGAGGAGTTCAGCCCTATGCTAGGGTA
ACTTAGGGAGCACGTAATAGAACAAGAATTGAATCCCAAGACTCTGAACCGTAAAGTCCTTGTTCTTAGTCATGAACTGT
ATTGCTTGGCTGCTGTGTTATCTCAAATGATGTCAGTAGTCTGCTGGGCTACAAATGTTGTTCAGCTGACGCTACTAGAA
GATTGAGTCAGCTTGCCTGGCTAAAAACAGATCCCAAGCGAAATCTATATTTAATTTTGAATTATCCCAATAGTCATGTTC
TGTACCCCAAATAACCCAGTAAGTAATTGCCTGTTCTTTCCCTCTCGTGCTACATAGATATCTAGTAGTAAAACACGGCG
TTACTATGGTGGTCACTGCTGTCTAAAGGAAAAAGATAAACCTGGAAGTATGTTATGGTGGACTTTTTAAATCAGACCTA
GAAATATTCTCTCATTCTTGGAACCTAGAAACAAGCCTACCTTAGGCTTCTGATTCCCAAAAGAAGTTAATATATCAGAA
CAGGTGTCATGGCTCTTGCCTGTAATCCTGGCCCCTGGGAAGGAAGAGTGCCACATGATTAAAACCTGCCTGGGCTTCAG
AGTCAGTCTCAAAAAGAGACTTAAAAAGTATGCATCTTAACTACGATGCCTGACTGAGTTTGTTGTTTTAAAGTAATATG
CTTTTAAAAACAAAGATCTCTTTGCAAAATATTTTAGGGTTTTTTTTTTTTTTTTTTTTTTTGGGCAGTTTCTCTGTA
TAGCCCTGACTCTTCTGGAACTCTCTATAGACCAGGCTGCCCTTAAACTCAGAGACCCACTTGCCTTTGTCTCCTGAGTG
CTGGGATTAAAGGTCTCTACAGACAGGCCTTATTTTTGGTTTTTAAGGGTAAAAAAAATGTACTAATACTTTTAAATAAA
GTTAGGGATTTCTGGGGCTTGGGAGATGGCTCAGTGTATAAAGTGTTTGGCAGGTGAGAGGATCTGAGTTTGGATAGTCA
CCACTGAAATTTAAAACAATTGAGTGTGGCTATCTCAGCGCTGGTAGGCAGAGGTGGGAGGGTCTCTTGGGCTCACCCTA
AGCTTTACCTCTGGCCTGCACAGGCATGAATGCACACATGTACACATGCCACACACATAGGAGACTGAGATAAAAGGATT
TCCTTGCCATGAATGGTAGAACAGTGCCTGCAATCCCAGGTGAGGACTGAGTTGTTGAAGTAGAAGGACCACCAAGAGTT
CAAGGGTAGCCTGGGTGAGTTTCCGTCTGGCCTTTGTTGGTAGAAGATGCCCAATTTCATCCCTACAGGCTGTGGAGAAT
TGAAAGAGGTGCTAGGTCTAAAATATAAAGTGGAAAGATTTGCAAACTTTTTCTTCCAAAACATTCCATTTCCTCTGGTA
GTTCATCTCAGAGCACTTTGAGCAGTGGTTGTGCCCTCGGTGACATACACGTTTCTCTGGAGCAGCTAACCTGAAGTGTG
GTAAGCTCTCAGGACTCATGTGGGGAGTGTACCATGGGAAGGTTCAAGACTCTTAGCAGAATTCTACTGGAGCCGGTTTT
GGGGCCTATGTCTGTTTTGTTTTTCTGGTTAGTTTGTTTTTGAGACAGGGTTTCATGTAGCCTCGGTTGACTTTAAACTT
GCCGTGTAGTTGGTGCTGGCCTTGAACTGATGCTCTTGGTCTCCCCTCCCCAATCCTGAGTTACAGGCATCTATCAGCAT
GCCCAGCTCTGAGTCTATCTTAGTTACATATGTTTTAGTGAAACAGCAAACCTGCAGTAAGAACTTGGCAGCCGTCAAAT
CTATGCTTGGAGTTATGTATGTTGGCTAGGCCCATGGTGAACCAGAGCTCAGTGTTATCTATAATCTCTTCCCTGGGCCT
CGCTGCTTCTCCTCATAGGCTCATTCGTACCTAAGATGAAGTCCTCCTGGTATTTTGTGGAGAGGTAAACTGGGACAACC
CAGCAGTCTTTCCCTTCTTCCCCCAGATGTTTGTCTAACCCTTCCTGCCATGAAACAGCTCACATCAGCTTGAGATTGAG
CATCCCCAGTGCTCTGCTCCACGCAGTCTTTTCTCTAGTCTGCCACCCACTTCCCACCACACTGATCAAAGAGTCTCGGA
```

```
ACTGAATAGACCTTTCCCTGGGGACCCTTGAGATCCTAAGTGTCAACTCAAGCTTGAGCCCAAAATATGGCAGTTAGGAG
CCAAACGAGAAACTGTCTCAAAGCATTTCCCCTCTGCAACCTTGTGCTCAAACCCAGCCTCATGTGTGCTAGCAAGCTGC
ATTCCCAGACCTGCCAGGACACTTTTGAAAGTTTGCTTAGTACTTGAATTCAAGTTTGCAAGCTAGCTGTGATTACACTA
GTCACCCCCACAATCAGATACATAAGACAGCCTTTATTTTTCTACCTAATTAAATCATTAAAGACAAAAAGACAAAGTTT
AAGAGTAAAGTGACCACAGAGCCTCCTCCCCTCCGGTGTCACCTGCTAGGTTTGGCACATAGCAGAAACTGCAGCCTGTT
GGAAGGGCAGTTGCACAGCTTGTAGATTGCACATATACAGGGCAGCCCAGCTCTGGGACCCAGCGGGGCTTTCTATCTGC
TCTCTTGAATCTGAGATGGGTAACCCACTGAACTTTCTGAACAGAAGACAGAAGAGAAAGGAGAGTCGTTGCCTTGAGGA
GAACCCCTGACAGTGCGGTTTTGACGTGAAGGGCAGGTATGGAAGATCTGTGAGGAAGTGGACAGCAAACCTGGGTGCAG
AATCTGAAACGCGAATAGCTAGGTCTCCTCTGTAGTGTGGAGTTGCATTTCTCCCTAGGGTGTTTATATCAAAAGGGCAG
GGAATAGAGCAGAGGGCCAAACGTGGGCGGTGTATCCTTCATCCACTATGGCAAAAGGTGGGTTGAAATGGAGGTGTGTT
TTCTGAGGGGACAGGTTCAGAGTAGATGGAGAGTTACTGTCATGATGTTATTTCCTGTTTGTAGTCACTTTCCGCTGACA
GAAACTCTAAAGAGGCATCTCCTGTAGCCTTCATGCCAAAGAAGACACCTGGCAACTGTCATAACAACTCTGACAGACAT
TCTCAGCCCTGGTCTCCAATGAGGAAACTACAGCTCAGAGACATCTGTCTAAAATCACAACTAGTAGCCAGCATTAAACT
AGTTCCCTTGGATACTAGTATATTGCCAGTAATGTTCTTTCTTCCCTGCTTTAAAAAGAATGTACTTATGTATTAGACTA
GTAAAAACATAGTTCTCAAAAATTGGAAAACTAGGGACTGGAGACATGGCTTGGTGGTTGAGAGCACTACCTGCTCTTCT
ACAGGACCCAGGTTCAATTCCCAGCATTCTTGTGGTTCACAACCATCTGTAACTCCAGTTTCACGAGATATGATGCCCTT
TTCTGGACTTTGAGAGCAACTAGGCATGAAAGTAATGCACAGGCATACATGCTGGCAAAATTCCATCATTTTTTTTTTAA
AGTAAACTGAGAAAAAGTAGAGTACTGTTAAAATCCTGATGTATTTCTTGCTCTTTTTCAATGTGTATCACTAAAAGCAG
ATATTTGTTGAGATCTTGCTTTAAGTGTTTGTGTGTGCATGCGTGTGCATGCTTGCCATTGGAAGCCAGAAGAGGGTGAT
GAATAATCCGGAGCTAGAGATGTACTCCGTGTCCTCCTGTCTCTACCTCCCAAGTGCTGGAATAACAGGCATGCAGCACA
CACCTGGCTTAGCCCTTTTTCATATACTTCATTCTGACGACAGAACAGGTAGTTACTGTCATTTAGTGATGAGAACAAT
AAGCACAAAGATAATTAACTTGCCAGAAGCCACATGGATTGTGAGTTGAAACCCAGATAGGCTACCTCTGGAGCAGTTTC
TCACCACAGCTAGCGTTTGGGGCAGACAATTCTTTGATGCTTATTGTAGAATGTTTAGCAACATCCTTGGATATTGCTTT
TCAGATACCAGTAACATTTGAACCCAGTGTCTGTAGACACTGCCATATGTCTTCTGGGACTAAAGTTTCCACCTAAGAAC
CACTGGTCTTGAGCCCCAGCACTCAACCCCTGGCCTCTATAGCCTCTTCCTATCTAAAATGCACATAGTGTCCCCATGAG
GTGGCTCTCTGGGCCCCTCACAGTGGAAGGAAAAAACAGAGTCTTGTAAAAGCCAGCCTTGGGTGCACACTTGTCATCTC
AGCATTTGAGAGGTAGAGGCAGAAGGATTAGGAGGTTAGGGTCATTCATGACTACATTTCAAATTTGAGGCCAGCATGGG
CTATTAACTCTTCTCCTGATAAACAAGATACAAAAACTTAAAAAAAAAATAAAAGCAAGGAGCTTGGGGGTCTCAGTCAA
ATAAAGCACTTGCTTTGCGAGCAGGCAGGCCTGAGTTACTCCCTAGCACCCGTGTAATGCTGGCCATGGTGAGAACGCTG
GGTGTCGTGGATGGAATGCAAGCATGGGGAGGTGGAGGCTCAAGGGGCTTGCTGGCCAGTCATTTTAGCTTCATTAATGA
GTGATGAGCACTGTCACAAAGGAGCTAGACAGCATTCCTGAGGAGGACACTTGAGATTGTCCTGCCTCCATATATACATG
CAACCGTGTACATTTACACGTATACACATGCCTCCGCATCCACATGCAAACATGTACACACTGTCCACATACATACACAT
ATACACATGCCTCCACATACACATGCAAACATGTACACATGTGTTCATAAACATATACACACATGCTTTCATATACACAT
GCAAACATGTACACTGTCCACATACATATACACATATACACATGCCTCCACATACACATGCAAATGTGTCCACACACATA
TACAAATATACACATGCTTCCATATACACATACAAACGTGTACACATGTGTCCATATACACATATACACATGCCTCCATA
TATACATGTAAACGTGTCCACGTATCCACATACATATATACATGCCTCCATATGCACATGTAAACATGTACACATGTGTT
CATATACATATACACATATGCCTCCATATACACATGCTTCCATATACATATGCAAACATGTACACACTCTCCACATACAT
ATACACATATATACATGCCTCCATATACACATGCAAACATGTACACATATGTGTCCACATAAGAGTTAAAAAAAGGATGTA
CAACAACAAAGGAAAACGGGAAACAGGAGAGGAAAGCCAGTTTTGGAGCCCCTTCTTTCTGGGTGTGTCAGATGGATGGG
GGCAGGGGCAGGGCAGGTGAAGTGTGCTAGTGTTGCCTGTGGTGGACAAGGATCAGAGTCCTCCAGAGCTGTCTCCACTT
TAGTGCTTTCCTTTGGTACACCTCAGGGGTCTTTGTTGCCATAGTTAGCTTTGTATGGGCAGGTGTTGTCACTCTCTCAA
GAGTCTCCTGTGGTGGCTCTAGCTGGCAAGTGTAGGGGAGGCAACCACAGCCCTGAGATCTGTGCAGGCAGACCTTGTCT
CCATACTCGAAAGGCATCAAACATCCATCACCCTCCGGTAGGGGTATAGCTGTGCACATGATGATACCGGAGCTGCAGGG
CTCCCAGACTCTGCAGCAGTGATCCTCATGGCAGATAGTCAGTCCCTAAATGGACCCACAAACTGAGCTTGGACTCAGGT
CCTCCCAACTACAAAGTGATTCTTGACTGATGAGGTCAGCACCTAGCCATTCAGGGGCGCTACCTCCCCTTGTAGGGCCC
CCTGCCCAAGCATGTCTTTGTGTGTCCCAGCTGCCCACTTCTTCACATTTGCACCCCTTGGATGTCTCCTGTGGGGGAAG
TGGCAGGTTGATTCGTGCTTCCTACCTTGATTGCCAGGCCTCAGGTCCTTGGGCAGCTCTGTTCTTTTTGCTGAGTTTCT
CTTATTCTCTGAACTGTTCGGCTGCCCTTCACACCCTCAGTGAACTCCATCCTTTGCAGTCAGTTTTTACCCTGACTCT
TCTGCACACCACAGTGCAGCTTCTAGTCCTCTGCCTGCCTTCCCTACAAGTCTAGCAGAGCCTTGTAATTTCAAGGGC
TCTCATGTTTTTCTTTTCTTTTTTCTTTAAAGATTATTTTATTTTTATTTCTGTCTATATTTGTATGTGTTTGCACAAAC
GGGTAGACAGAAGAGGGCGTCTGATTTCCTGTAGCTAGAGTTACAGGTAATTATGAACTGCCAGATGTGGGTACTGAGAA
GAGAACTCTGGTGTGCAAGAACAGCAGGTGTGTAGCAGCGTTCTTTAACTGTGGGGCCATCTATTTCTCCAGTCTAATTT
TGCCTTTTTTTTTGTATTGTTAGCACCCCGTCTTTCCTGGTTAACTTTTTGTTGTTGTGATAAAACCTGCTCAAAAGTAG
TTTAGGGGAGGACAGGGTTTAATTCAAATTACAGGTTAGAGTCTGTCACAGAGGGAAATCCAGGGCAGGAGCCCAAGCAA
GAACTTGAATGAGAAGGACTGGTTACTGCTAGCTTGCAGACGCATGCTTATTAGCCAGCATTCTCATACAGCCCAGGACG
TCCTGACCAAGGAATAATGCTTCCCACAGTGGGCTGGGCCCCCCACCTCAATAATCAAGACAGTCCCACAGTCATGCCCA
CAGGCCAATCTGATCTGGCCATTCTCCTCAATTGAAACTCCCTTCTGAGGTTGTGTCAACTTTCCAGAGTTAACTTGGAT
GCCATCCAGAAGTTCTTGCCAGTCCCAGAATCCTCAGGCTTCCCTGGTTCCTTCAAGAAGCTTAGTTTCAGCTCCAGCAT
TCAAGCTTCCATCCATTTTGAGTTGCTTTGTATACCTGGCAAGAAATGGGAATTTCATTTCATCCTTTTTCTTGCAGATA
ATTCTGTCTTTCCAGTACCACTTATTGAAAAGACAGTTCCTTCTCTGATATGTGTCCTGAGCACCTTTGTGAAAACTCAG
CTGGGTTTAGTTGTATAGTTGACTTGCAGGCATGTGTGTTTAATGCTTTATTTAAGTTTTGTATTACACTCCTTTGGGGG
```

```
TGTTGCTTTGCATGCCATGGCAAGAATGTGGAAGGACTCTGTCAGTTCTCTCCTTCTACTCTGTGGGTTCCGGGGATGGA
ACTCGGGTTATCAGGCGTGGCCCTGCTCCCCGTGTGTGTGTGTGTGTGTGTGTGTGATAATGCCAATGCCATGCTGAC
TTGATTAATACAACTTGGCTGTGGCTGCGTGGGGACTTTTGTGATTTGGCATATATACATTCTGTCTCTTCCTGACTATG
AAGCGTATCACTGGTATTGTGGTGGAGGCTGTGCTGACTCTGTAGCTCTCTTTGAGCGTTCTGGACTTCTAACAATACTG
ATTCTTCTATCTGTGAACACAAAGTATACTTCTTTTCCTTCTCCCCTTCCCCTTTCTTTACCTCCTCCGATCCCTCCCTC
CTTCTGTCCTTCCCTTTGTGCTTCCTAGTCTCCTTCTATTGCTTTGACCAGTATTTTACAGTTTTCAGTGTAGCAACTTT
CACATCTTCGATTACATTTATTCGTAGCTATTTGTTTTGCAGCCTTATAACTGAGTTTGCTTTCTTGAGTTCTAGTTTA
GCTGATTTGCTAGTGGTGTATATAAAGTGACTGGGCTTTTCTGTGTGAGCCCTCTAGCTTTGCGGAATTGGCTGCATTGG
CTCACTGGTTCCAATAGTTTTCAGGGAAACTTAGTTTTTCTTCAAGTGAGAGCATAGTATCAGCCAGCCGACAGGTGACT
TGATCTTCGCTGCCTTGGTGTGTCCTTGTTCCTTTCTTCTGCTTGGGACTTTCAGTATCTCACTAAATAAAAGTGATGGA
AATGGGCACCTTACCTTGCTGTAGGTTTTAGAGAACAGGTTTCAGCCTTTCTCACACACTTCAGTGGTTATTGCTGGCTC
ATGGTGCTTGGCTTTCATTGTGTTGTGAAGTATTTTCTCAGTTCCTAATTTGTTGAGAGTTTTTAACTAATGAAGTAAGA
TTCAACTTTGTTACTATAGTCAATTATTCAATTATTCTTACTATAGTCTTTATATTCTGTTTATTTTATTTAGTGTCTTC
TTACACTGTAGCCTAGGCTATCCTATAACTCACTATGTAGCCTAGGCTGGCCTGTAACTCACTATTGTTACTCACAACTC
ACAATGTAGCTCAGGCTAACCTGTAACTCACCTTGTTGACTAGAGTGGCCTGCAACTCACCATGTTGTCTAGACTGGCCT
GTAATTCACTATATAGCTGTTGCTGCTCTCAGATCTATAGTCCTCCTAACTCATTTTTTTTCCCAGTACTAAGAGGCAACA
CCTAGCATCCTAGCACTTGGGGGGTGGAGGCAGGAGGATTAGAAGCTCAGGAATATATGAGATCCTGTCTAAAACAAACA
GACAGACAAACAGAAGAAGGAAAGAAAATTAAACAATTCTTCCTTGATCCATTAGTAGTTCAAGAATAAGTTAATATTTA
TATATTTGTATAGTTTACAAAGTTGTTTGTTATTGATTTCTAGTTTTACTACAGTATATTTTTAAAATATACTCAATATG
ATTTCAGGTTTGTCTGTCCGTCACTTGTTGAGACTGTTTCGTGGCTTATCGCATGTCTGTACAGGAGAGTCTCAGTCTTT
CTCTCTCCCTGCTTTCTGCCCCCTTGTGTTCCCTTGCTCTATCTTACTCTCTCCATATGCATTTATTTATTTATTTATTT
ATTTATTTATTTATTTAGGCAGAGTCTCACTCTGCAGCCCAGGAGAGTCTCAGTCTTTCTCTCTCCCTGCTTTCTGCCCC
CTTGTGTTCCCTTGCTCTATCTTACTCTCTCCATATGCATTTATTTATTTATTTATTTATTTATTTATTTAGGCAGAGTC
TCACTCTGCAGCCCTGCCTGGCCTGGCCTAGAACTCACTGTGTAGACCTGGATGGCCTCCAGTTTATGGAGCTCCATCTG
CTTCTGACTCCCAAGTGCTGGGACTGAAGGCATGCCCATTTTATATGCTAAACTGGAAAAAATGTGCCTAAGATCCCTAG
TTCTACAAAAGAGAATTAGTCAATTAACTCTCCATCTGAGAATGCTCACAAACTACTCTATGGTTAGTTTCCGGGGCCCT
CTTCTGACCATGAGCGAATCATGGTTCCTTGAGTATTTTCTCCTCTTCCCTAGACTCTTGAGTATGCTAAGAAAACCCTG
CATAGCTGCTATATCATCAGCCAAGTTTGTTTTTTTTTTTATTTTTTAAAGCTGTATGTATGTATGTATGTATGTATGTT
TGTATGTATGTATGTGAGTACACTGTAGCTGTACAGATGGTTGTAAGCCTTCAAGTGGTTGTTGGGAATTGAATTTCAGG
ACCTCTGTTTGCTCCAGTCGGCCCTGCTCACTCAGTCCCTCCTAGCTCCGGCCCAAAGATTTATTTATTATTATACATAA
GTACAGTGTAGCTAACTTCAGACACACCAGAAGTTCAGATCTCATTACGGGTGGTTGTGAGCCACCATGTGGTTGCTGGG
ATTTGAACTCAGGACCTTCAGAAAAGCAGTCAGTACTCTTACCCACTGAGCCATCTCACCAGCCCCAATCAGCCAAGTTC
TTTGTTGTTGTTGTTTCCTAAAAGTTCTGCTTAACCATTTTTATTTATTATTGTTCTAGAGGAGGTGATATTCATGTGTC
CTGGCTCACTCGTGAAGCAGAGGGCTTCTTGGAGTTGGTTCTTCCCTTTCCCCTTTATTCAGTTCTGGCAATGGAACTCA
AGTGTCCAGGCTTTCAGGTAAAACACTTGAACCTGCTAAGCCAAATTGCTGCACCCCCTGCCCCTGTTCCTGTGAGTGGG
CTGCACATGGAAAGGTTAGCTATCTGTCCTCGTCTTCATGCTCTGGCATGGTTTGTACTGTCATTATAGCTCGACAGGCC
TGTGTGCCAGTGGATACTGCCATTTCAGCACTAGATGGCCGGCCTGAGTCTGGGTCTACTCAAGTCCATGGTCTTGTACA
TTGCCACGGTTTTAGCACTAGAGGGCGATCCAAGGCCAGATCTTCCTGAAATACACAGCTGGTGTATCCTTCATCATGAG
ACACCTATAAAGGATGGTCCTTCCTTCCCTCAGTGTTTGGGGCTGAGGGGATGTCTATACGCCTCATGCTCAGCCCTGGC
TTGTGGTCCAACACTGTGGAGTTTGCCTGAGCTCTGGGCAAAACCACTGTCACGGTGCTATCCTCAGGCTCCTGTGATCA
CTAACGCTGCAGCACTGCCCAGCCTTCCAAGACCAGTGTGTCTCAGAATCCACCCACACTCCTTTTCACTAAAACATGGA
CTTTGGTGCTGAGACCCTAGAGATCAGAACTGTTAGAAAAACAAAAAACAAACAAACAAACAAAAAAAACCCTGTTCTTT
AAAAAGGAGTTAGATTTTAGCCAGGTGGTGATGGTGGCAGTGGCTCACACCTTTAATTCCAGCACTCGGGAGACAGGTAG
ATGGATACCTAAGTGTGAGGCCAGCCTGGTCTACAGGATAGCCAGAGTTCCAGGATAACCAGAGCTGCACAGAGAAACCC
CATCTCAAAAAAACCTAAACAGGGCTGGTGAGATGGCTCAGTTGGTAAGAGTACCCGACTGCTCTTCTGAAGGTCTGGAG
TTCAAATCCCAGCAACCACATGGTGGCTCACAACCGTCCGTAACGAGATCTGACGCCCTCTTCTGGAGTGTCTGAAGACA
GCTACAGTGTACTTTACATATAATAAATAAATAAATCTTTAAAAAAAAAACCCTAAACAAACAAAAACATAAATTTTAACA
AAGTATTTATATCTTTAAACTTTAAAGATGTTTCTTATTGTATGATGTATGGGAGTGTGTATTCCCTAATGTGCATGTGG
AAGTCAGAGGACACCTCTCAGGAGCTGTTTCTCCCTTCACATGAGCTCTGAGACCCTAACGCAGCTTCTCAGGTTTACAT
TGCTATGTAGACCAGGCTGGCCTTCAACTCACAGCGGTCCTCCTAAGTGCTAGGATTACAAATTTGTGGCACCACACCTA
TGGGAGACCTTGACTAAAGAAGAATGATGTAGTTATGAGAGAGCCTGGAGGGATGGAAGCTTTCCTGGCCTCCTAAAATC
ATTTCAAACATGCAGACCCTGTCTGATATTTTAGTGTAGGTGGTGGTTAGTTTTGATTATCACAAGTGATTAAAAAAACA
AATATTGTCCAATCATACGTCCTTAAAACCATATCATGTATCATGTACTATAGTGGCCACATTAACATTAGTCCTATAGT
TTGGACTTGTTAAGGATTAATGGCTTTAAAAAAAGATTTATTTATTTATTTATTATAAATACACTGTAGCTGTCT
TCAGACACTCCAGAAAAAGGACATCAGATCTCATTACAGATGGTTGTGAACCACCATGTGGTTGCTGGGATTTGAACTCA
GGACCTCTGGAAGAGCAGTTAGTGCTCTTAACGGCTGAGCCATCTCTCCAGCCCAGATAAATGGCTTTTGTGAAGGGTAT
TGAGTACCACTTTTTTTTTTAATTAAATAACTCACAGAGTCCAGTTTGTGTTGCCCGTATATGCATGGTTGTTGGGCCC
TCCATTAGAGCATGGTTATCCTACCAGGGATCACACCCCAGAGAAAACTGGCTGCCTCCCTTGTCCACCATCAACTGTCA
GTAGCTCCTCACCTAGGGATGAATAGATCACACATTTCAATTAAACATATGCCTCTGTGGATAAACAAGCAGCTATGGGG
AATGACTTTATATTCCTAGTTCTGGATGAGGAGTCCAGAAAGACCCCCAAGTACAGCCTGTATGGCCCTCTGGATTCAGA
TCAGGCCCCCTGTGGTCTGTCTCCCTGGGCACAAAGCTTACCTAGTTTGGAGTGAAACTCTGAGGTGATCACCATTTCCA
```

```
CTGTAAGACCTAGGGTATATAACTCCAGGATCTTTTCACTTGGTAGGCAGGCTCGTGATCCCATCTGGCTGATAGGATTG
CACATGGGCCGTCCTTGATTACAGGAGCCATGAACTGTAGGGATGTATGCTGCCACAGCCCTACTGTAATTGAATTACTG
CTGTCTTCTCAAATAACTGTAGGTGCCGCAGTCTATGTTACTATTAGACAGAAAACATTTTAAACTAAAGTACATAAAAA
CTAGCATTTTGAAAACTCTAAGCCAAGATTGGAGTTTCTTTTTAAAAGAAACTTAAATGGTTTTGGAGGCAGAGTCTCAG
TGTGTCATTCAGGCTGGCGTCAAGATCTTTGCTTCAGCCTCCCAATGTTGGGCTATAGGCGTGCACTACCACTAGATTAA
ATTTTTAAATAAATCTCAGATATAAGAATGGGTATTCTGCCGGGCGTGGTGGCGCACGCCTTTAATCCCAGCACTTGGGA
GGCAGAGGCAGGCGGATATTTGAGTTCGAGGCCAGCCTGGCTACAAACTAAGTTCCAGGACAGCCAGGGCTATACAGAG
AAACCCTGTCTCAAAAAACAAAACAAAACAAAAAGAATGGGTACCCTATGGTGAGAATATTTTGTAATAAAATTGTTTA
TGTTTGTGAATATTTGTAATAAAATTGGTGGGTATTTCAGTAGGAAAATTCAGAATTATTGGGAATTTTTTATATGTGTG
TGAATGTATGGCATGTGTATGTGTGTGTTGCGTGTGTGCACGTGTACCGTGTGTGTGCAGAGACCAGAGGATGACA
TTAGGTGTCTCCCTTTGCTCACTCTTTACCTTACTTACTGAGACAGGGTTTCTCACTGAAGCAGAGTGTGCTGGTTTGGT
TAATCCAGCTGACCAGTTTGTCTGGGGATTAGGACAAAGGCAAGGGCTGGGATGATAGGCAGGCAGGCCCCTGTGCCCAC
CGAGCTCTATGTGCGCTCTGGAGATCCACAACTCCTGTGTGGTACCAACGTGGCCAGCACTTCATCCACGGAGCATTTTT
CTGTAGCACCCGTACTAAAGTTGTCAAACCTGTAGGTGTCATGTTGCATGCAAATAGAAACTAACTAGCCATTGCCCAGC
TTGGCTGGGGTCATCCCTCCATAGGACACAACGTTTATCAAGTGTGCCCCTCTGGTATTTTGTTTTTCTTTTGACAAGGT
CTAACTATGCAGCTCGGGTTGGCCTTGATCTCATAGAGATCCACTTGCCTCTGCCTACCCAGTGCTGCTGAGGTTAAAGACTT
GCACTATCATGTCCAACAACCCCTCTATTCTTTGTGGTTCTTTTCTTTCATCTCATTAGCGTCCAGTCTTTTCTCTCCAC
TACAGATGAGCTTCAAAAAGTTAGATGTGTAATTTACACAGACATGATGAACATGAAAGAAAGAATTATTCACTTACGAT
TGAAGGAGACATCGAGATAAGGGTGCTTCCAAGAGCATTTGAAGGAACACAGATCTAGAGACATTAAAAAAAAAGATAG
GGGGCTGGAGTGATGGATCAGAATTTTTATTAGTTTAAGAGGAATTCTAAGTGTCCTTTCTCTTAAATTTTATTTTTATG
CGTGTATGTACAAGAGCACATGTGCATGCGTGTCTGCAGGCCAGGGGACAACCCTAGTTGTCATCTCCAGGGACACTGTC
CACCTCCTTTAGGTCAGGCTTGCTCAGGCTGGAGAGAGGGCTCAGCAGTTAAGAGCGCTGCCTACTCTTCCAGAGGTCCT
GAGTTCAATTCCTAGCAATGCCATATGGTGGCTCATAACCATCTATAATGAGATCTGGTGCCCTCTTCTGGCCCACAGGT
ATACATGCAGGCAGAATGCTGTATACATAATAAATAAATCTTAAAAAAAAAAAAAGTTAGCACTTAAACAGTGTCAACTG
GATAGACACAGAACCGGTTAATCTCCTACAAGGCAGCAGGTGCCTCCCTAACCTTTGGGGTTATTTGCTGAGCTAGACAG
GAATATTCAAGTTAACATATAACTTCACAGTGTTCTATTGAGTAGTCTGGCACTGCTCAATGGTAAACAGCAAAATCAAG
TATGGGTTTGCTTTGTTTTCCAGAAACTTTATCCTTTGGAAAGCCCAGATAAGGATGCCTCAGGGTAGGTAGCTCAGTGT
AGGGCATTTATTTGCCCAGTGTATACTAGGGCCTAGGTTCAATCCCCAGCATGGGAATGGAGAACGTCCTAGCAGAACAA
CAAAGAGACGGTGTACCTTTTTGTATCTGTCACTTCTAAAATTACTGTGTATAGGTGACTCTAGACGTACTTATTAATTT
AACAGCCATTTTTATTTCAGAAGCCTTCCCTGAAAGACATTTATTTCCAGGCTAACAGAAGCTGTTGAGAACTTCTACTC
TGGGACTCCAGATCGAAAAGTTAGCAGGCTTTCCCCTGGTGCTGGGTAGCCGTGTCTGAGGACTCTTAAGGTTCCTTTCT
GCCTTAGGGTAGCAGTGGAACTGAGTGATTTCAGTGTCCCCTTGTCCTCTCACTTGTCTTCCCCTCTATTCCTACATCTC
CAGTGTCCCAGCCTCTTGAGGACACACAGCGATCTTCTGCCCACCCCCACTGAAGTCTAGCAGGGCCACGGGCAGGAGGA
ATTTAAGTTCTCTCTCTTTATAGTCTGACTTAGTTAATATTTAATAAGCAGTTATTAATTCAGAGTACCTCAGCTACATG
AGGCATGGAGAAAAGACTAGCACCTCCTCCAGAACCCATAATTCAGTGGGGAAGACAGAAGCATGCAAAGCCACCTGACG
TTCTCTTTCTTAATTGTGGCTGAGCATTAGGGTCACCTGGGGAACTCTTTTTTTTTTTTTTCCCCTTCCCTTCTCCCCCTT
GCTCCAGCCCCGCTCGCTCCAGCCCATAGGTTTGTTTGTTTCATTTGTTTCTCATTATAGGTGGTTGTGAGCCACCATGT
GGTTGTTGGGCTTTGAACTCATGACCTCCGTAAGAGCAGTCAGTGCTCTTAACCACTGAGCCATCTCACCAGCCCCCACC
TGGGGAAACTCATGAGACACCCTGATGCCCTCTGTGTTCCAGACTCTTTAAACTAGAATCCCAGAGCTGGGGGATAGGAC
CCAGGCATCTGTTTATTTAGAGGTCTCTGTGAAATTCTAGTGTATTGCTAAACTAGAGCAAAACAAACTTGGAAAACTTA
CACGAGGATTACTTCAGGCACCACCGCCATCCTGGACAGGCTAATGCACTATCGAGTCACATTTCAGCCCACGAGTGCG
GAAACTATGACCTTTCATTGTAAGTATGAGAAAGTCTTAGGTCAGTTTGACTGCAGAACTCTTAGAAAACGTACACTTTG
GGAGCCTGGGAGGTGGCTCAGTCAGTAAAGTACTAACATGCAAGCATGACAACCTAACTTGGTCTCCTAGAAGCCATTTA
AAAGTGGACACTGCTGAATGGTCCAGTTTTCTCCATGGTGCAGAGTAGAGACAGGAGGGCCCTTAGGAGATGCTAGCTAG
CCAGTCTGAGACCCTGTGTCAAAAATACAGTGGAAGGGCTGAAGAGGTGGCTCAGTGGTTAGAAGCATTTGTTGCTCATG
AAGCTGCCCCAGCTTTGGTTCCCAGCACCCACATCATGGAAGGGAAAATAGTCATGTACATAAAATATATGTTTTTAAAA
AAAAAAGGAAGTGCAGCTTATGTAGCTTCCTTCAACCCATACCTCCGAAAGTATTCTCCGTGTGTGTCTGTGTGTGACGT
GCATCTGTCTGTGTGCAGACATCAGAGCTGTCTTCCTCACCCTCCTCTGTTTTACTTTTTAAATAAATGTGGGTATGGTA
AGAAAGCATAACTACTGAGCTGCGTGGAGAATCTGCAAGTTCCAGGCCAGCCAAGGTGACAGTCAAAAAAGTTATGTTTT
CTTACCAGTGCACAAGTCCTAACATTGACAAGGGCCACCAAGCTCAGTGTCAAATAAGGTGGGTAGTGTTTTTACCGCTC
CTGGTGATACACCCAAAGGACTCTGTCTGTCACAGAGATGCCTGCACACTCATGCTTACTGCAGAACAACTCACCACAGC
CAAGATAGGAAATCAGCCTAGATGTCTGTCAACAGAAGAATAAGGACAGTGCAATACGCACGCACAGGAGAGTCTCACAC
AGCCATAGGAATGAAATGATAGCCACTTACAAGAAAGTGAATGGAAGCAGAGATCTAGTAACAAAACAACTCAGGCAGAC
AATTATCACTTGATTTCTCTCTCATCTTTGGATCCTAGATTTCGAATAGCTACATAAAATCATTGATATATGTGTGTAGG
TGTGTATACATATGTGTAGGTGTGTGAGAGACACATGAAGCAAAAGCAAGACTTGCTGGGAGGAGGCAGATTCGCAGGAG
AGGAGAGAGAGGGATGCTCTGGGGGTGAGTGTGGTAGAGCATATGGTAGAGAATGGCTCTGTGGAGCCCAGCCCTATACA
TAGTGCTGTGTGCCCGGTCTGTGCCATAGTGGGGATTCTAACGAGGGCTGTCCTGTTAGGAGGCAGCTCACACTGACACC
ACCTTAACAGGAGGCCTCGTTCTTCTTGTCTTATCTAGAACTAAGTGGAAAAGAGTGACTATGGTTTTTGCTCACAGAAT
GGATAACGACCAGCCGCCTGTGGTGACTGCCACCCTGCTGGTGCCCCTTCAGAACGGCAGCTGCGCGGAAGCAGCTGAGG
CCCTGCTGCCCCATGGCCTGATGGGATTGCATGAGGAGCACAGCTGGATGAGCAACAGGACAGAGCTTCAGTACGAGCTG
AACCCTGGAGAGGTGGCCACCGCCAGCATCTTCTTTGGCGCTTTGTGGTTGTTCTCCATCTTTGGCAATTCCCTGGTATG
```

```
TCTGGTCATCCACCGGAGCCGGAGGACTCAGTCCACCACCAACTACTTCGTGGTGTGTCCATGGCGTGTGCTGACCTTCTCA
TCAGCGTGGCCAGCACGCCGTTTGTCGTGCTGCAGTTCACCACCGGGAGGTGGACCCTGGGTAGCGCCATGTGCAAGGTG
GTCCGCTACTTCCAGTACCTCACCCCCGGCGTGCAGATCTACGTGCTGCTCTCCATCTGCATCGACCGCTTCTATACCAT
CGTCTACCCGCTGAGCTTCAAGGTGTCCCGAGAGAAGGCCAAGAAAATGATTGCAGCCTCCTGGATCTTGGACGCGGCCT
TCGTGACGCCTGTCTTCTTTTTCTACGGCTCTAACTGGGATAGTCACTGTAACTACTTCCTCCCTCCCTCCTGGGGAGGGA
ACTGCCTATACCGTCATCCACTTCTTGGTGGGCTTCGTGATTCCCTCCATCCTCATAATCCTGTTTTACCAGAAGGTCAT
AAAGTATATCTGGAGAATAGGCACGGACGGGCGGACGCTGAGAAGGACGATGAACATTGTCCCCAGGACAAAGGTGAAAA
CGGTCAAGATGTTCCTGCTCTTGAACCTAGTGTTCCTGTTCTCCTGGCTACCTTTCCATGTGGCTCAGCTCTGGCATCCC
CATGAGCAAGACTACAAGAAGAGCTCCCTTGTCTTCACGGCAGTCACGTGGGTATCTTTCAGCTCTTCAGCCTCGAAACC
CACTCTGTACTCTATTTATAATGCCAATTTTCGGAGAGGGATGAAAGAGACATTCTGCATGTCCTCGATGAAATGCTACC
GCAGCAATGCCTACACCATCACGACCAGCTCGCGGATGGCCAAAAGAAACTATGTGGGCATTTCGGAAATCCCTCCCGTG
AGCAGGACCATAACCAAAGACTCCATCTATGACTCATTTGACCGGGAGGCCAGGGAGAAGAAGCTCGCCTGGCCCATCAA
CTCAAACCCTCCAAACACTTTTGTCTGATTTCTAAGAAGTCTTTCACTGTTATGTGCCAGAGATTAAAAAGCTTTCACTA
TAAAGTGAGAATTGATTTACTTAGTTTTCACTAAACTTCCGTTTTGTAAAACATGTTCACTATTGATTATGGTTCCTGTG
GATTTGTTCTGGTTGCTTTTTGCTGGTCAGTAGTGCTCCCACTATTAGAGCCTATAAAAATTCTTTCCTGGGCCGGAGA
GGTGGCTAATTGGCTAAGAGCACAGGTTGGTCTTCCAAGGACTCCAGCACCCACAGGGCAGCTCACAGCCATCTGTATTT
CCAGTTTTAGGGAATCCAGTGACCTCTTCCGGCCTCTCGGAACACTACACACTTGTGGTGCACAGACATTCACACAAATG
AAACATTCACACACATAAAATTTTTAAAGATTTTAAATATTTTTGTTTGTTTCAAAGTTTTGTTTGTTTTTTCGTCTGA
GCAGGTAAGTTGGCCTAAGAACTTGAGTCCAATCCCTGTAAGTCACACGTGAAGGAGAGAACCACTTGTAAGTTGTCTCT
GATTTCCACACAAGTGTTGTGGCATGCAAGCACACGAGTGTGCACACACTGACACTAAATGTACAAGAAAAATTGCAA
ACACTTTGCTATTCTCGTTCTGTCACTGGGTCATTACACACAAGTCTTCTATTTTTTGTTCTGTCTTGCTATACTATAT
TTTGTTTTAATTTTCTTTTCCCTATTGAATTTTTCTTCTTTTTCTTTTTTTAAAAAATTTTCTTCCTTTTTTTTTTTTT
TTTTTTTTTTTTTTTTTTTGAGACAGGGTTCTCTGTGTAACCCTAGATGTCTTGGAACTCACTCTGTAGATCAGGCTGGC
CTTGAACTCACAGAAATCTGCTTGTCTCTACCTCCCGAGCACTGAGATTAAAGACGTGTGCCACCACTGCCTGGCAGCCT
GCTGAACTTTTCTATGGCCTACTAATTCACTGACGTCCCATACGCATTACATTTCAAGAACTTAACTTTTCCCAGCCTGG
CGACACAGTATGCATTTGCATATTACCTTCTGGAAAGGAGTGGAGACCATACACCAGAGCCCAGAAGAACTGGTTGGGAA
GCTGGCTGGGACGAGTTTAATCATTCTTTGGGAGCGAGTGCCCAGTCATCTACAGTCTTTGCCTTTTCTGGTTTCCATCC
TAATCCAAGCAGCTGGGGAAGCCTTCAACATTCTTTTATTTAAATTCATGTTATTTATTGGTAGTGGTGGCGGTAGGCTG
CACTTGAAGAGATCAGAGGGCAGCTGCAGGCATCAGGTCTCACTGTCCACCATGGTGTAGGTTCCAGGGCTTGACTACAG
GCACCTTCTCCCACTACCCTAGGACACGTGTCACCTTCTCTGAGTCATACACAAGTAAAGCAGCAAGTGGCAGTGCTAGGCTTG
CCAAAGAGAGGAATGACATTTTTCTGGGCTCTGCGATCACAGACACCCTCAACTATGGCTCTACTGTGACCCACAAGGAC
ACCAAGGGCAAGATTTGACATTTTCTATAAATTCAGACAGGAGGCCTTTTATTGTAATCACATTTTTTTCCTTACTTATG
TGAATGTGTGTTGGGGTGCCCCCAGAGGCCAGAAAAAGGCACTGCATCCTGAGGTTGGAATTAAGGCTATTGTGAGCTAC
TGAACACAGGTGCTGGGTGACAAACTTGGGTCCTCTATAAGCACAGTATGTGTACCTGAAAGGTGAGCTATCCATTCCGG
CCCATGAAGTTCATCTCTGCCAGACAACCCCTTCTCTTTCTAGATTGAAGCTCAACTTGTCTTCCAAACTCACACTCTCC
ATTGTCTGTGTGACTCAGAGCTTGGTTCCTTGGTTCCCAGTTGTTTAAGTTTAGAAATGAACCAGTATATTAGAGAATAT
TTTTCATTACTGGGTATGGTAACACACACTCCTAATCCCAGCCCTCTGGAGATGGAGGCAGAAGAATCAGGAGTTCAAGG
TCATTGTTGGCTACACAACAAGTTGGAGTCCAGCTTGGGCTGAGACTTTGTCTCAAAAGCAAAACAACACAAGACAAAAG
GAATTTAGTTTAATGAAATAAGGGCTTACAGACAAGCCTGGTTTGCATTGCCATGGGCTAGTTAACACGAGCTGCAGATA
ATAAGTCATTGTTTAGCAGTCTACGTGTATTGGCTCATCCTTTCCACAATTCCACGATGTATTTATGGGTTTTGCTCTTC
TGTTTTTGATTGGGTTTATAGCCCAGGCTGGCTACATACTCAAAGTGATTTTTTTTTTTTTTAAGATTTATCTATTTTTA
TTATATATAAGTACACTGTAGCTGTCTTCAGACACTCCAGAAGAGGGAGTCAGATCTCATTTTGGATGGTTGTGAGCCAC
CATGTGGTTGCTGGGACTTGAACTTCGGACCTTTGGAAGAGCAGTCGGGTGCTCTTACCCACTGAGCCATCTCACCAGCC
CCTCAAGGTGATCTTGCTTCAGCCTCACCTGCGATGAGTTTATGATCATCTCTATTCACCAGATCATGAGACTGGAACTT
CTCCATGAGGCAGCTTGTCAGCTTGGAGTCTGACAGCTGGTATTCTGACTCGGGCGCTCCTACAACCCTGTGGCTTGTTC
CTCACCATATCAGCTGTCTCCTTAGAGAACATGAGTGGTCCCACCCAGACATGCCCTGTAGGCCTGATCACAACCTCAGT
AGAACAGTACAGTCGGAAATAAGCCGTCGTTCTGATGGCATCAAGATGGAGTGTAATGAGCTGGCTCACACTTTTCTCTT
GGCCATAGATTGGCTCCTCGACCTCTGGTACCGAATGGTCTGAGATCCAGAAGGTAGTCTGTTCTTGGTGACAGAGCATC
AGTGCAGGCCAGGTGTCGGGGCACACACTTCAAATCCCAGCACTCAGGAGGCAAAACAGGCAGATCTGTGTTTAAGCCAT
TCTGGTCTACATAGCAAGGTTCAGGACAGCCAGGGCTATGTAGTTAGAGCCAATCTCAAAGGCCAAGGAAACCCCCAAAC
AGAGCCGTTAGATTTTGATCTACAATCATCCACAGAAGCTGACAGGCTGGCACACCCTCATTATCAGCACTCAGAAGGCA
GAGGCAGGAGGATCTGGAATTTCAAGGGTTAGCCTGGTCTACACAACGTAAGCTCCCGGCCAGCAAGAGAAAGGACAACA
AAATATACCTTCAGGCAAGCTTTTAGGTTGAGATGCTTCCTTCAAAGGACCAATACAAAGGCTAATAAAAACTCATTTTT
TCCTTTCTTCCTTCTCCTCACACGCTTGGCTAATGTGTCTGGAATGAAATGAGTCAGACACCTGTCTCACTGAGCCCTGC
ATTTTATGGCTCAGATTTCAGTGGAAAAGACCCTTCTGGTTGTTTGGCTGCTTGACTGATTGATTGGTTTTCTGTTTTAT
TTTGTTTTTCTTCTGCTTTTCAAGCAATGTTTCTCTGTAGTCCTGGCTGTCCTCAGGACCAGGCTGGCCTTGAACTAAA
AAATCTGCCTGCCTCTGCCCACCTGCAGGCACTTATTTATTTTAAAGATTTATTGTATTTTTATTTGTATGTGTGTGTGT
GTGTGTGTGTGTGTGTGTGTATGTATACACAGTCAGTGTGTGTATTGTAGATGTGTGCAGGTGCCTGTGGAGGCCAGA
AGAGGACATCAGATCCTCTGGAGCTGCAGTTAGTTGTGAGCAGCTGAGTGGGTGCTGGGAATTGAACCTGGGTCTTCTGG
GAGACCAGCCAATGCTCTTAATTGTTGAGCTATTTCTTCAGACCCTATTTAACTTAATGTGTGTGTGTGTGTGTGTGTGT
```

```
GTGTGTGTGTGTGTGTGTGTGTGTGTGTATACATATTCATGCCAAGCTCACTCAGCAAGCACAATGGCTATCTTAGCTTG
CTTCTGTTCCTGTGGTAAACACCATGACCAAAGGCTAGTTAGGGAGGAAAGGGTTTATTTTGCCTCACATGTCCCTATCA
CAGTCTATCACGAAGGGACGTCAAGGCAGAAGCCTGGAGTCAGAAACTGAAGCACAGACCAGGGAATAATGCTACTCACT
GGCTTGCTCAACTTGCTTATTTACACAGCACAGGACAACCAACCCAGTGGTCTAGGCTTTCCCATATCCATCATCTGTCA
GGAGAATACTCCAACAGGTGTGCTTCCAGGCCAGCCTGATGGAGATGTCTTCTCCATTCGGGTTCCTCTTTCCAGATGAC
TCTAAATAATCGGCACAGCTGCCCAGCTTTCCTTATTTGTGGATTTTTGTTTCTGATACAGTCTCAAGTAGCCTAAACTG
GTTTAGAACTTTATATAAAGACAAAGTTAGCCTTGAACTCCTGACTCTCCTGTTTTTACCTTCCAAATACTGGGGTGACA
CCAGTTTGCAGCACCACATCTAGCTTTGTTATTTTATGTTTTTTTTAGAAACTTAATCTTTTTTTTTTTTTTGGAGGGGG
TTATTTGTCACTATGGAGACCAGGCAAGACTCAGACTCATATAGATCTGCCTACCTTTGCCTCCAGAGTGCTGGGATTAA
AGTCATATGCCAACATGCCCCGAGTCTTAAAATTAAAAGGAGGAGGAGGAAGAGGAGGAGGAGGAAGGTATAGCCA
AATGGCAAAGTGGTGGTCTAATATGTAAGAATTAGGGTTAGTCCCCAACATTCCCCCAACCCACACAGACTTAAAAATCA
GAAAGATGTAGGCAGGTTTGGTGGTTCGTGCTTTAGCTCTTAGGAGGTTGAAGACTGTCCTAAGTTGGAGGTCGGCCTGG
ACTATCATAGTTGAGTTCCAGGCCAGCCTAGGCTACAAAGTAAGACTATGTCTCATAAAAAGTAAATAAATAAAAGTATT
AATTTAGAAGGCTTGGAGAAATGGCTCAGTGGTTAAGAGCACTGACTGCTCTTTTAGAGGTCCTGAGTTCAATTCCCAGC
ACCCACCGGGCAGCTCACAACTGCCTAATTCCAGTTCCAGGGGATCTGACGCCCTCACACAGACATATATACAGGCAGAA
CACCAATGCACATAAAATAAAAATAAATTTTAAAAATATTAATCTAGGGGCTGGTGAGATGGCTCAGTGGGTAAGAGCAC
CCGACTGCTCTTCCGAAGGTCTGGAGTTCAAATCCCAGCAACCACATGGTGGCTCACAACCATCTGTAACAAGATCTGAC
TCCCTCTTCTGGAGTGTCTGAAGACAGCCACAGTGCACTTACATATAATCAATAAAAAAATCTTTAGAAAATATTAATC
TAAATTACATTTTTTTCCCAGAGCAGAGGAAGCCTCTTACCTTTTACAAGACTGAAATCAAGCCAATAAGTAAAGTTAAG
GGTGATCTACCATTCCTGGCAAACTAAGTGGGTGAGATGGTTCAGCTGGTAAAGGTGCTTACCACCAATTTGACTGAGTT
CTGTCCCTGAACGGAAAGAGCCAGCTCTACAAAGTTTTTCTTTCACCACACACCTGCTATATGGTTCTTCTGCTATATGG
TTCCCCCCGCCCCGACCCCAATATATTTTATTTAATATTTACTTTTAATTTGGTACCCAGTTCTGTGTCACTCCTGCTTA
CTGCGACACAATTCGTGTGTTGGAGAAAAAAAAAACGTCCTCCCTGCGCGCAGACCTTGAAAGGATGCACACACAGCCCG
CCTTTGTTCAAATTCACCAGGAAGGGAAGACTGAAGAATCCGGACGCAGTTCTGAGAGACGCCGCTTGGCGGCGCTGTGG
CTAGCGGGCAGAGTTTTCACCCAGCTACTCATTGGAATTCAGAGAAATCTTGGTCTCTTAGCAACCACACAGCCAAGCTT
GCAGAGCACCCAGACTCAGCTGTGCGGTTCATCAATCAGTGTGCTCCAAACGCAAAGCTTCTAAACATCAGAGGAAATTCC
CCAGCAAATAATACCCGTTACGGGCGCGAAATCGCTACTCAAAATATTGTTTAAACTATTTTACAAATGTGATCATGCT
CAAAAGGCCCCAACAGACCGCCAAATCCACAGCACTTACCGTGGCCTCTCCGTTTTCTGTTCCTTTTTTTTTTTTCTTTG
TAAGTCAGCTTCTCATGTATCCTGGGCTGGCTCTGAACTCCCCGATCCTCCTGCCTGTACGTCCCCAGTCCTGGGATTAC
AGGCACGCGTGACCACACTAGATTTTTTTCATTGCTGGCAGCAACCCCATCTGTTGCTTGGACTTGGTGCACGCTCGGTG
AGCTCTGTACAGACCGAGCTCCTGCCTGCCCCTCGGCTGCTTTCATAATTAGGTTCAGAGGACATGCAGATGTCTCCATG
GCAATACACGCTTTCCCCTGAGTGGGTGCGCATGTGCAGGGTTAAACAGACAATTTGACTTGGTGTGGAAGGAATTCACT
GCAGGCCAGTCTCACTGACCTCAGGCCTCTCAGACCCTCTTTAATTTTTTTGAGCTGCTACAATCGGTAGGTTCTGGAC
TTTTCCCAAGTGTGGACATGTCAAGAAGTGCTATCTATTCTTTCGCAGAAGCCTCTTTTAAGAGGGTTTTTGGAGTCCAG
TGAGATGATTCAGTGTTTAAGGGTGCTTGACACCGAGCCTGGCTCCTGAGTTTGGTCCCTTAAACCCACAAGGTGGAAGG
AGAAAACAGATTCCCATCGTGTGTGTGTGTGTGTGTATGTGTGTGTCTATTATAACCCTTATCTAAACCCATGTCTATAACC
CCAGCATGTGGAGGTACAGACAAGAGAGTTAGGAATTCAAGGCCAGGATTGGCTACAGGAGTGTGTGGGAAAACAAAAAC
CAAACCAAACCAAGTCAAAACAAAGGTATTGCTTTAGTGACTGACTCCATCAGCTCTGAGTTATACAGTAGACGTTACAG
AGTTTGGATTGCCTTCATACTTGAAGGACGTGGGATTTTAGCTCCCCAGGGTGACCAAATGCAGGCTGAGGAGGCAGACC
TGGAGGTCCGAGGACATCATGTGGTTTGGAGCATGCAACAGTCGACATGGCCTTCCAGCACTTTACTAACCAAAGCGTAG
ATGCAGAAGACTGATGCTCCTTTAGGGAAAGGTTCCAGAGACGCTGCAGTAAGGTGGATACTCTTCTTTTATGTAGTCAT
GGCAACCTGGCTCTGGTTCCCATCTCTAAACCCAGCACCGGATAGGTGGCCACAGGAGATCCCTGGAACCTGATGGCGGG
AAACCAGTGATTTCCAGGCTCATTGAGAAAAGAAAGTGCAGAAGATTGACAAGGGTCTGATGACAGCCTTCCCTGCTCTG
TGCTTCAAGTTGGCCTGTACCCACCCCCTGTTTAATGAATTCCTGTCTCATTTGTTAGGTTCGCTCTCTCCCTCCTCTTG
AGAAGAGTCTATCACAGTCCCTCGAACCCTGCTCAGGGTGACTTTGAACTTCTGATCTGCCACCTCCTGCGTGCTGGGAT
TGCAGGTGTTCACACCCTGTCTGTGTGGTGCCGGGGACTTTTGAGCTGGTGTCTGCCAGACAAGCACTCTACCCGTCGAG
TTCAGCGCCTGGCCCGTTCCCGCATTTGTCCTCAAGACGCTCCCATGATGTCCCGCTCCGGGAGGCTTTAGAGTTTTCAA
AGACTTTTTTTTTTTCCATACTAGGCAGAGTTCCTGATCTCTCTGGTTGAAAAGCTCATGTTCTCATGGTTTTGTCCTT
TTTGGCCGGGAATACTAAATGACGAGGGTCACTCTAACAAGGCCCGGGGCATTCTCCACTTGCATCTCCTCCCAGGCCTC
ACAGAAGAGCAATGATGTGAAGGGATGTTTGCTGTGTCTTTCTGATATTCCTTTCTCTTCCCGTGGCTCTTGGGTTTGGC
TGCTCCAGCTTGAACAGCACGTTTGTATCAGTATTTTTTCTCTTCTTTGTTTTGTTTGGTTATTTTAAGCAAGATCTCT
CTATAGCCCTGTCTGGTCCAGAACTATGTATTGATCTTCTTGTGTTTTTTGAGTTGGCATCTCATATAGACCTGGTTGGC
CCCAAACTTGTGATGTTGCTAAGAATGAACCTTCTACCTTCAGCTAAAAGTGGGTGGGACAAGTTTAATGAGGATCATTT
CCTGTTTGTTGCTGTAGTCCCAGTAGGGGTGCTCTGGCTGTGGACCTGGGTGGTCTGTCCTGGCCTTCAGACTCAGTTCA
GAGTCATTGGTCCCAGAGTCTTGAAAGTGCTTGCCAGGAAGAGGTTGCTGAGGGACTGGACTGGGCTTTTCCCGTTCAGA
TCTTTATCCAACAGAATAGTCTGCTAGGCACAGCTCACTGCCTAACTCCAGCCCAGCCCAGCCCAGTCCAGCCCAGCCCA
GCCCAGCCCAGCCATGCTTGGGACCCTGTAACAAGCAAGTAGTAGATTAGTTGAGTTTGTGGTGACCCCAGGATTAAGAA
TTCAACTGAAGGCTGGGTATAGTAGCATAGCCCTTAAATCCCAGCACTCAAAGAGACAAGGCATGTCGATCTCAGTTCCA
GGACAGCCTGGTTTACAAATCGAGCTCCAGGCCAGCCAGAGCTGCATAGTGAAACCCTGTTTAAATAAAGATAGAAAAGA
AAAATAAACACAGCTGATCTTCTGCCTCATGGTTCCTAGAGTGGGACAGGGCTGCAACAGTTTCAGTCACAGCTACAATG
TGTCCTGTGCCATAATCTGACTTTCTGGACAGCTCCATGCTGTTTACAGTCTGAGTTCCTCCAGCCAGTTCTTCACCCTC
```

195

```
TATACTCTGTGTCTCAGATGTACTGTTCTGAGCTGTATTGACCCCCATTTTGGCCATTTCCAGGGGTGCATCGGAGCCTG
ACTGTCCATGGTCTGTAGCATTCACCTATATCCACAGAGTACACACACCTATACCCCTGCTGAATAACTACCAAACTGAT
GTCACTGAACAGGGAGAAGAGGCACACTTACTTGGGTTAAAGCAAGCTGTAAAATGCCATTATTTTGCATACTTTTCACA
CAGGCACCCCTAGTGAGTTGAGGAATATAAAAAGGTGTGCCGATTGAGTAAGTTCTAGGCAGGCCTAGGCAACGGGAATG
GTTCAATGAGCAGATGGTAGGAGGTTTTCTTCCATGAGGGCTTATTAAGGGGGAACTGGATCTGGAGATCAGGCCCATCA
GACACTTTAACTGAGTGACTTTGTGCAGTGCACACTCAGCAAAACTTTACATGACAGTCCAGACAGATTAATGACCACTT
GTAATCAAGTAAGACAGAGAGTAAATAAGTGGTTCTTAAAATGAACCTAACTAATTGAAGGCTATTATTGCCACAAAGAG
CCTCCAATTATACCAACTCAAAGGATAAGTATAACAATATATAGAAACCACCTGGGAATATCCTAACACTGCATTTTGAT
ATGAGCTCATTAAATCTTTTTTTAAGATTTATTTATTATATGTAAGTACACTGTAGCTGTCTTCAGACACTTCAGAAGAG
GGTGTTAGATCTCATTACAGATGGTTGCAAGCCACCATGTGGTTCCTAGGACTTGAACTCAGGACCTTCGGAAGAGCCGT
CTGCGCTCTTAACCACTGAGCCATCTCACCAGCCCCAAACACTTCTATTTTTGATAACTTAAATCCTTACATACTGTGAA
TATTTTTATTTTCAGATTTATTTAGTTTGTCTATGGGTGTGGTCAAAAGACAGTTTGGGGGAGGGGGCTTTCCCTTCTGC
TGTATGTGTGGGTTCTAGGGATCAACTCAGGTTGTCAGTGTGGTGGCAAGCGCCTTTACCTGCTGACCATCTTGCCAAGC
CAATCCCGTGTTATTTAACAGGCAGGTTTTCATACCCCTGTGACTCAGAAAATGGACTAAGGACCCGCCCCATACATTTA
AATAAGAAAGGATCATGTGTCTGTGTGCTTGCCTTAGTGAACGCCTATGCAACACCTATGTGCAGGGGCTCATGGGATCT
CCTGCAACTGTAGTTAGTTACCTGGGTGCTGGGAACTGAGCCTGGGTCTTCTACAAGAGCAGTAAGCAGTCTTCAGCACA
CTTGACCGTGGAAGCAGAGGTTAGCCCACCACTCCAGTTCTCCCCGTTCTACCATGTGGGGCCTGAAGT
```

MOUSE mRNA SEQUENCE : mR5-033.1 (Seq ID No: 44)

```
AGGTGGCCCCGTCGGCCCCCGAGGGATGTCAGTCAGGGGACGTGGAACACCTGCACAAACTGGATTTTTGCCCTATCACG
ACTCTGAGGAAGCAGGCTGAGGGCTGAGGGAAGGCAAAGGGGAGATATGAATGTGTTCCGAAGGTTTCCCGGTGGCTCAT
GGCATCCTCCAAAGTCCTGCCTGCGAGCTGTTCCTGACCAAGAAAACCCGCGGAGCCTGACTCTGAAATCCAGTCCCGAT
GAAGAACTAAGTGGAAAAGAGTGACTATGGTTTTTGCTCACAGAATGGATAACGACCAGCCGCCTGTGGTGACTGCCACC
CTGCTGGTGCCCCTTCAGAACGGCAGCTGCGCGGAAGCAGCTGAGGCCCTGCTGCCCCATGGCCTGATGGGATTGCATGA
GGAGCACAGCTGGATGAGCAACAGGACAGAGCTTCAGTACGAGCTGAACCCTGGAGAGGTGGCCACCGCCAGCATCTTCT
TTGGCGCTTTGTGGTTGTTCTCCATCTTTGGCAATTCCCTGGTATGTCTGGTCATCCACCGGAGCCGGAGGACTCAGTCC
ACCACCAACTACTTCGTGGTGTCCATGGCGTGTGCTGACCTTCTCATCAGCGTGGCCAGCACGCCGTTTGTCGTGCTGCA
GTTCACCACCGGGAGGTGGACCCTGGGTAGCGCCATGTGCAAGGTGGTCCGCTACTTCCAGTACCTCACCCCCGGCGTGC
AGATCTACGTGCTGCTCTCCATCTGCATCGACCGCTTCTATACCATCGTCTACCCGCTGAGCTTCAAGGTGTCCCGAGAG
AAGGCCAAGAAAATGATTGCAGCCTCCTGGATCTTGGACGCGGCCTTCGTGACGCCTGTCTTCTTTTTCTACGGCTCTAA
CTGGGATAGTCACTGTAACTACTTCCTCCCTCCTCCCTGGGAGGGAACTGCCTATACCGTCATCCACTTCTTGGTGGGCT
TCGTGATTCCCTCCATCCTCATAATCCTGTTTTACCAGAAGGTCATAAAGTATATCTGGAGAATAGGCACGGACGGGCGG
ACGCTGAGAAGGACGATGAACATTGTCCCCAGGACAAAGGTGAAAACGGTCAAGATGTTCCTGCTCTTGAACCTAGTGTT
CCTGTTCTCCTGGCTACCTTTCCATGTGGCTCAGCTCTGGCATCCCCATGAGCAAGACTACAAGAAGAGCTCCCTTGTCT
TCACGGCAGTCACGTGGGTATCTTTCAGCTCTTCAGCCTCGAAACCCACTCTGTACTCTATTTATAATGCCAATTTTCGG
AGAGGGATGAAAGAGACATTCTGCATGTCCTCGATGAAATGCTACCGCAGCAATGCCTACACCATCACGACCAGCTCGCG
GATGGCCAAAAGAAACTATGTGGGCATTTCGGAAATCCCTCCCGTGAGCAGGACCATAACCAAAGACTCCATCTATGACT
CATTTGACCGGGAGGCCAGGGAGAAGAAGCTCGCCTGGCCCATCAACTCAAACCCTCCAAACACTTTTGTCTGATTTCTA
AGAAGTCTTTCACTGTTATGTGCCAGAGATTAAAAAGCTTTCACTATAAAGTGAGAATTGATTTACTTAGTTTTCACTAA
ACTTCCGTTTTGTAAAACATGTTCACTATTGATTA
```

MOUSE PROTEIN SEQUENCE : mP5-033.1 (Seq ID No: 45)

```
RTKWKRVTMVFAHRMDNDQPPVVTATLLVPLQNGSCAEAAEALLPHGLMGLHEEHSWMSNRTELQYELNPGEVATASIFF
GALWLFSIFGNSLVCLVIHRSRRTQSTTNYFVVSMACADLLISVASTPFVVLQFTTGRWTLGSAMCKVVRYFQYLTPGVQ
IYVLLSICIDRFYTIVYPLSFKVSREKAKKMIAASWILDAAFVTPVFFFYGSNWDSHCNYFLPPSWEGTAYTVIHFLVGF
VIPSILIILFYQKVIKYIWRIGTDGRTLRRTMNIVPRTKVKTVKMFLLLNLVFLFSWLPFHVAQLWHPHEQDYKKSSLVF
TAVTWVSFSSSASKPTLYSIYNANFRRGMKETFCMSSMKCYRSNAYTITTSSRMAKRNYVGISEIPPVSRTITKDSIYDS
FDREAREKKLAWPINSNPPNTFV*
```

MOUSE PANTHER CLASSIFICATIONS
         FAMILY (SUBFAMILY)
               GALANIN RECEPTOR-RELATED(Unassigned)
         BIOLOGICAL PROCESS
               Biological process unclassified(2.99.00.00.00)
         MOLECULAR FUNCTIONS
               Molecular function unclassified(1.97.00.00.00)

MOUSE GENE ONTOLOGY
         BIOLOGICAL PROCESS
               cell surface receptor linked signal transduction > G protein linked
receptor protein signaling pathway

behavior > feeding behavior
                G protein linked receptor protein signaling pathway > G protein
signaling, linked to cyclic nucleotide second messenger
                G protein signaling, linked to cAMP nucleotide second messenger > G
protein signaling, adenylate cyclase inhibiting pathway
                cell-cell signaling > synaptic transmission
        MOLECULAR FUNCTION
                1-phosphatidylinositol 3-kinase > 1-phosphatidylinositol 3-kinase
regulator
                enzyme > 2-acetyl-1-alkylglycerophosphocholine esterase
                amine oxidase > amine oxidase (flavin-containing)
                monooxygenase > monophenol monooxygenase
                defense/immunity protein > blood coagulation factor
        CELL COMPONENT
                cell > membrane fraction
                plasma membrane > integral plasma membrane protein
                cell > plasma membrane
                cytoplasm > endosome
                cytoplasm > lysosome
MOUSE PROTEIN DOMAINS (INTERPRO SIGNATURES)
                IPR000276 (GPCRRHODOPSN)
                IPR000276 (7tm 1)
                IPR000276 (G PROTEIN RECEP F1 2)


HUMAN GENOMIC SEQUENCE : hD5-033 (Seq ID No: 46)
ACCCAGCCAGGGTGAAATTTTTTTGCACTTGTTTTTTTTTACATTGTATCTTACTTTTCAGATTTTGTGTGAATGTAGGGA
GGTAGGGAAGTTCAGTATTAAACCTGCAGCTGTGTCCTGTATACACGGAACTCTACTAAGCTACTAAGCAGCAGTTGGGA
AGAGAAATATTAGATAGTATTCTTTATACATCCGTGGGGGTGGTCACCCCCAAAGAGATGCCAACACGACCTAACATTTT
TGTCATGCAACATTGTTTACAAGGAATTAGGGGAGGACTGCAGCCTGTGGTCAACTTAAACCTTCATGTGGGGTAAAGCT
CAGGCTCCTTTCTGTTTTATTCCTTTGGGTAGGGTAGCTACCAGTGGTTATCCATTCCTTCACAAAACTGTCAAGGAGGT
GAAGTTTATCTTCTTCAACTTCACCTTTCACCCTTCACCCGGCCCTTCTCATAATGACACCCCATCTTAATTCTCTCAGT
TACTGATGGTCCTCACGGCTTTTGCTTTTGGCATCTCTTCTGTTTTCATTTTCACTAATCGTAGATTTCAGAGATAGAAT
GAATAGGTCTCAGAAATCACTACTGATAAAATATAAAACTGAAAAAAATGTACAACTGGTCTTTAAGAAACATTTATATT
TTAATAGGGATCTTCACTTTAAAGGATGATGCTGTAATTGTGAGAGAGGTTGATTTTTTGAATTCATGTGAAGGGTAAAG
AAAGAGGCAAATGTGATAGGAGAGTAATAGTCCCTTAAATCTACATAGGCAGAATTTCTGAGCCAGGAATGTTCATGGTT
GTTTTAAGCTATTTTGTGAAGTTAGAAGAAGCTGTTTTGAAAACATCTCTGTTTAAAAAAAAATTATGTTCCCATGAAAG
CATATCGAGTTTCCAAACAAAAAGAAAATATACCCAGTCTGAGCTTGAGAAACTGCTTGATGCCAGTTAGGAAACAGTTT
GAAAGGTACAGAGCTGGATTATTATGGAGGAATACTTATATATATTATATAAAAGGAGGAAAGGAAAAAGACCAGTAGAG
AAAATTCCAGGCCTATAAGCTTGTGGCACCAGGAGTTTCAGGTGGTGGAAAGAAGCTCTGTTTTCATTGGTGTGAGGTTC
TTTTGCAATTATTTAAAATATCTAATGGAAACAATTTTTTTTTTTTTGAGAGGGAGTCTCACTGTGTTGTCTGGGTTGGA
GTGCAGTGGTGTAATCTCAGCTCACTGCAACCTCTGCCTCCCTGGTTCAAGCAATTCTCCTGCCTCAGCCTCCCTAGTAG
CTGGGATTACAGGCACCAGCCGCCACACCCGGCTAATTTTTTTTTTTTTTATTTTTAGTAGAGATGGGGTTTCACCATGT
TGGCCTGTCTGGTCTTAAACACCTGACCTCAGATGATCTGCCCACCTCGGCTTCCCAAAGTGCTGAGATTACATGCCCAG
CCTCTAATGGAAATAATTTAAGCAGAATAGATTCTATGGGCTTTTGAAAAAGGCGTATTCAATCCAGTTGTATTATGTAT
TTTGATGTAGGATACCTTAAAAAGTTTGATCTGGACAACATGTCACATAAAATCATCCACAACTTGGAGAAAATGTGTTT
ATTTATTTGGCCTCAATGATTTCCCCCTTAAAAACATTGTTGTAAATGATGATTAGGTACCCAGGCCCCTTCACAAAAGC
CATTTACCCTTGACAAAGCTAAATTATGTGACCAATGTTAGTATTATTACTATAGTATGTGATGTGTAATATGGTTACTA
AAATTTGTTTGTCATATGGTTTAAAAGACTAATTTTTGAAGCAGTTGCTTTGTTCCAGACACAGAACTAGGTGTTTTGTA
GAACTGTATATAAAAATAACACCTGTTGTTTTCCTCATACCAGGCAGATGCACATGTATTAACTCATCTAAGTCCTGTGT
ATATTCTCTCATCTAGTTATAATAATCAAATGAGATAGGAACTGACTATCTTCATTTTACAGAAGAAACTGGAGCTCAA
GAGGGTTAGGTAACTTGCCCAAGGTCACCCAGCTAGTGAGTGAGTGACAGGAATAGGATGCAACTGACTTCAAAGCCTAC
ATACATATCCACTTTGCAGTATGCCTACACTGTGAGATTTAAAACATGAGATTTTTTTAGCAGAAGATTACATTCTGGGT
GTAGGTTTGCCAGATTTAACAAATGAAAATACAGGACACCAGTAAAATGTTGCATGGGATATTGCATGGGACATACTTTT
ACAAAAAAATTATTTGTTATTTATCTGAAATTCACATTTACCTGGGCATCCTGTATTTTATCTAGCAACCTCATCTGAGT
CGACATTGTAAGAACATGTCTCACCCTCTCTGATTTTCCTGTTACCCCACACAGTCATGCTGGTGGCTACCTAGCCTTAT
GTAGTTTTATAAATCACCTAATGTTCATATCAGTCTTTTCTCAGCTGGGCATGGTGGTTCACACCTATAATCCCAGCACT
TTGGGAGGCCAAGGCAGGAGGATTGCTTGAGCCCAGGAGTTCAAGACCAGCCTGGACAACATAGCAAGACCTCATCTCTA
TAAAAACACACACAAGCACACATACACCAGAGTCTTTATCTCAAGGCCACACAGCAAGAGAGTCAGTTAGGAAGCTTCAGA
AGAGCTTGTGTGGCCTCCTGAGCCATTTTTAAGTTCAGCAATGTGTGTAAGTTCAGGACTTAGGATAGGCTGCCCTGAAA
GCCCTGCCCTACTGGAAGTGGTGTGCGGGTAAATGTTTAACAACTGGCTCTGCAGAGGGGGGAAAAAAACAAAACTTCAA
TTTTTGGTGTTTGCCATACTCCTACCATGGCTGCTTCATGATGCCAACATGATGTCTTTGAGCCTGGAGTTGGGAAGAAA

```
TGTATATACAGTTGGCTCAGGGTTCCCTATGTAAGCCAGTCCCAGCCCACTAGTGTTACAGGCATTTCTCATTTTCACAT
GTAAACAAATCAAGCCTACACAACAATGAAGGGTTTACCTTAAAATCTCCGTACCGTGGTATTTCTTGAATTTAAACAAC
TAAAATTTTAAAAGATAATTTCCTACTTGGGATAATACAATTGATCAAATTTTTTCCTCATTTCAATTCAAATTGAAGTC
TAGAATTCTGCTTTCATTCTGTTTAAAAGGCAACAGTTAATTATGCAGCATGTTGAAATGCCAATACTCTCACACTCTTT
ATTCTGAAGAAAGGCTATTTCTCCAGGCCAGCATGTCTGGCGCACTTTTGATGCTAAGCTTCTGTGCAGTGCGTTGTGGG
AGTGATCATTTTTTAAAACTGTTCACTATGTCACATATTTCTGAGCTAGCATATTGAACAGATGTATCTGTGGGTAGGCA
AGAGGCTATAGGGAATAACTTTGTATTCCTCTCTTTGAATGAATAGTCAAGAAAGACTTACCCATGTACAGCCAATTTGA
CTCTCTGAATTCAGATCCAGCCCTCTGTGGTCTCACTGGTCAGGAAACCCTGATGGTAGTTAAGAGCAAGGGCTTTGGAG
TGACCACTTTCCAACGGTATGACTTTGGGGAAGAACTTCAGTTTTTTTCATTTGTAAAACAAGGCCAGTAATTTTACCTA
CTTTGAGGGGTTACACATATAAAGTGCCTGGCACATAGTAAGCACAAAAATAAGTATTAACTCTTGGTAGTATTAATGCT
ATAACTACTGTCATGATTGTAATCTTACTGCTCATTAGTAAATACCCATATGTATCCATGTAGGTCCAGCAGATACTTCC
CATTCTACAGAAAATGTTTCTGTTTTTTTTTTTTTTCAACCAAATCATGTAAAAAATCAATATTATAAAAATTCTTGTTTG
ACATTAAATTTTTAAACAAATCTCAAGAAATAAAAATGGTCTTTATACCTTGTGAGAATGTTTGTGAATCTTTATAATCT
CAAGAGGACAAAAAGTTAGTTGTTAGGGTATTTTTAGACTTAAAAAATAGACTAACTAAAAAACCTATCCAACAGTTGAT
GCAGAATTGTGAAGCAAAGTTTGTCATCCATGCCCCATTCCTCTCTGTGGTCCTCCGCTCCCTCTTATTAGCTTCCAGTC
ATTCATCCATCCATTGCATCCAACAAAGTATGATTTTCAAAAAGTTAGAATCATGACTTTTACAAATATTGAATGAACAT
ATGTTAAAGATTTATTAACATAATACCGAAGGAACCAATGATATAATAAAGCTGGTTCCAAGAGCATTTGAAGAATGCAT
CTTTATAGGCATTTTTTAAAAAGAATTTCAGCATAAGATTGCTAGGTAAACACTAAACTGGCTAATGTCCTAGAGGGCAG
TAAGTCCATAACCTTTGGGGTTATTATTTTCTGCTAGAAAGAAATCTACAAGTTAACATATAAATATCTTCACAATGTCT
CAGCTCTAGTCAAATATTAAATAGCAAAGTCAAGCACAGGTACATTCTCCCAGAGAGAGAGGGCCCATTTAAGAATGTCCC
AGCAAGATCTTAAGAGTATATGTCCTTTTTTGCCTTATTTTCAAGTATTGGATAATTTTTACTAACATATCCACCCATTC
ATATATTCATTAAACCTTTTTTAATAGCTATGTGCCCCAGGGATATGAAGTAAAGACATAGTCCTTATCCTCAAGGAAC
TCATAACCTAATGGGGGAGACAAACATGCAAAATAAAAATTACAAAGCAAAGTGATAGGGTACATTCTCCTCCCAGGTGC
CCCAGGATCCCATCATTACAGGGGTAACCAGTCCCCAGTTCTTTTTTTTTTTTTTTTTCTAATTGAGATGCAGAGTCTCA
CTCTCACCCAGGCTGGAGTGCAGTGGCGTGATCTCAGCTCATTGCAACCTCTGCCTCCCGGGTTCAAGCGATTCTCCTGC
CTCAGCCTCCCATGTAGCTGGGACTACAGGCACATGCCACCACGCCTGGCTAATTTATGTATTTTTAGTAGAGACGGGGT
TTTGCCGTATTGGCCAGGCTGGTCTTGAACTCCTGACCTCAGGTGATCCGCCCGCCTCAGCCTCCCAAAGTGCTAGGGTT
ACAGGTATGAGCCACCATGCCTGGCCCCCAGTTCTGTGCTATGACAACCCATCTACCATTTTCCCCACTCATGTATTAAT
CTTAAAGCTTGGATGGGTGAGAAGTAGATAATAGGATAAATGAATGATTCCTCTTTTAGATCATATATAGGTAGAGATAA
ATAAGGACCTTCAATACCTCAGCCAAGGGAGTATGTCAAATGGTAGTCTCAGAACTGGCTGGGAAGAGATAGGTCGAACA
GGAAAGAGGTGAAGAAGCCTTTCTTGTCACTGCTAAAATTACTGCATATGGATGGCTCTGAACAGAATTACTAATTAACC
AGATCTTATATTTCAGAAACACCCCCTTGAAGATATATTTTTATCTAAATTAATGGAGCAGGGGAGGCCTTTCTGAAACT
TTACTTTGGGACCCTAGAATTTAGTTATAATATGGCAGACTTTTTTCTGGGAGGAAAGTGCAAGAACCTGTCTGAGGACA
CCTAAATTACCTTGGCTTTCCCTTAGATGAGATATCTGTTTCATATAGCAGTTGACCTGGTAGCTTCAGTTTCACCTGCC
CTGCTTTTATCAGGAGTCATGTTTTTCCCCTATCACCATAATCCCCAATGTTTCACCTCTCTGAGAATATATAGTGATTTT
CTATGTGCATTTAATTCTCCTAGCAGAGGCACATAGAGTAAGAGTCATTTATTTCAGTTCTCTGTCCTTACAGATCCAAT
TTAGTAAACATCTAATAAGCAATTATTAATGTAAAGAACTTCAGTGGAAGCTGTAGAGCATGGAGAAAAAGAATAACATC
CCCATCTCCCTACTGGAGTCCACAGTTTATTGGAGAAGACAGAAACATGGAGGTATTTCTAAAGCACAGATCTTAATCTT
GGCTACACTCAGGATCACCTGGGAACTTTGAAACATCCGACACCCAGGCTGCACTCCAGAGCAATTAAATTAAAATCCCT
GAAGCTGGGACTCAGGCATCAATATTTCTTAAGGCTGCCTGGAAGACTCCAATCTACTGTATTGCTAAGCTAGAGAAAAA
CAAACTTGGAAAATGCTGCAGTAAAGGACTAACAAGATGCTGTATGAGCAAGGGTACCACAACCATTCTGGATATGCTAA
TATTCTTATCCACTGTCTGACCGCATTTCTGCCCATGAATGCAGCAAATATAACCTCTCATTGTAACCAGGGGAAAAATT
TAGATCAGTTTGATTGTAGGGCATTTAGAAACTATATACTCTGGGAGGCATGGTCTGAAGGAAACTATGTTTCTGAGGGG
AAAAGGGCCCCAAAAAAGATCAACCCAGAAAATGACCCCAAAATTCAATCCACATGCTATCTCTCTCCCCTGGAATGTT
TTATTCACTTGTCTTCCTGAAGAAGTCCTATTCATCCTCTAAGCTCCCAGCTCAAACTTTACCTCTTCTGTGAAGCCTTT
CCTTTTCTTCCCCTTACCCGGGCAAAACTGGGTCCCTATTTTTGTCTTACTTAGCTCCTTGTACAAAGTTCTGTTCTTAG
CAGTGAATTATAATTATTATCAAATTGAGTTATAATATGGCACACTGCTTACGTATCTTTGTTTTCTTTTTTTTTTTTTTT
TTTTTTTTTTTTGAGACGGAGTCTGGCTTTGTGCCCCAGGCTGCTCGGCTCACTGCAAGCTCCGCCTCCGGGTTCA
CGCCGTTCTCCTGCCTCAGCCTCCCGGATAGCTGGGACTACAGGCGCCCACCACCATGCCCGGCTAATTTTTTGTATTTT
TAGTTGGACGGAGTTTCACTGTGTTAGCCAGGATGGTCTCGATCTCCTGACCTTGTGATCCGCCCGCCTCGGCCTCCCAA
AGTGCTGGGATTACAGGCGTGAGCCACCGCGCTCGGCCGATCTTTGTTTTCTTAATACCTAGCCCAGTTTCTCGGCTTAT
TGAATGAATATTCAGAACTGCAGCGATTTTAGGTGGAGTGAGAACTTATGCCACCAGCAGGGGAAATCTAGACTCAGGAA
CTCCTCGGGCTCCCTCGTCCTGTTTCTGAGCATTGGGATCCTCTGCTTCCAAGTGGTTGGGCTGGGGAAATGGGCAGACA
GACTCATGGGTGTAAAGCAGCTGGAGGAAATGATGGAAGGGCCCCCCGCCTCCGGGCTTTAGTTAAAGCGGTGAGCTTGA
GTTTCATTTGCAGAGTTTTGTTGTTAGAGGAAGAAAGAGACAGAAGGAAGTTCACAGGTTTTCAAGTATGCGCATTAAGG
AAAAGTGCCTGTGTGGACTCCTGTTTTATTCAGGCCACAGGCATCCCAGAAAAGTTAGAACAGTTTTCTGTCTGGGGAAA
TTGTCTGTTTTAGTTTATTTTTAAACTTTAAATCTTAACAGGTTTTTTAAAAAGTTTAATAGGTTTTACACACATGAAGG
CGAAAAGATGATTTCAATCTTATATGTATGAGGGTGCAGTCCAGATTTTCTAGGACAATGCTAATTTTAGATCTTATGTC
CTGATTTCTTTTTTTTTTTTTTTTTTTTTTGAGATGGCGTCTCACCCCGTCGCCCAGCCTGGAGTGCAGTGGCGCGATC
TCAGCTCACTGCAACCTTCGCCTCCCGGGTTCAAGCGATTCTCCTGCCTCAGCCTCCCGAGTAGCTGGGACCACAGGCGC
CTGCCACCACGCCCGGCTAATCTTTTGTATTTTTAGTAGAGACGGGGTTTCACTGTGTTAGCCAGGATAGTCTCGATCTC
```

```
CTGACCTCGTGATTTGCACGCCTTGACCTCCCAAAGTGCTCGGATTACAGGCATGAGCCACCGCGCCTGGCCCTGATTTC
TTATATGGAAAGTGTATGGTTGCTATTCTTACCATTGGCTATCATTTTCCCAGCATGCTTAAAGATCTTTTAGTAGTCAA
AATGTTTAATTATTTTTGCTTTCTGTGTGGTGAGCAAAAACTTTTTTTTTTAATTTTTAAAATTTTAATTATCTTACTTAA
GCTTTCTTAAGTAAGGTTGCCTCATGGACAAAAAAGCCTAAAGATAATATAGAACAGGCATTGAGTATATTGCCTTGAGC
ACTAGACTTGTATTGAATATAGCTTTTTGGTGAAGATTATATATATTAACACTTCTTTCTCTACCACAAATTAGTACAAA
AATCCCTCAAAGAAAAAATGAGGGCTGGGCGCAATTGCTACACCCGTAATCCCAGCACTTTGGGAGGCCAAGGCGGGCGA
ATCACTTGAGGTCAGGAGTTCAAGACCAGCCTGGCCAACGTGGGGAAACCCGGTCTCTACTAAAAATACAAAAATTAGCC
AGGCATGGTGGCACGCGCCTCTAGTCCCAACTACTCGAGAGGCTGAGACACAAGAATTGAACCTGGGAGGGGGAGGTTGC
ATTGAGCAGAGATCGCGCCACTGCACACCAGCCCGGGGGACGGAGCGAGACTCTGTCTCAAAAAAAAGAAAATATTAGTC
ATTAAGAACCGTTCTTTCATTATGAACTATTATTTAATCCTGACTGTCACTTGAGAAATGTTTCTTTGTGAGGTGCAGAC
ACAGGAGCCCACCAGGAAAGAAACTAGTGACTACTAGAAAAGCCTGTTGCTGGAGCCACATCCCCTTGTGCCACAGGCCA
GATGGAATCTCTGCAAATGGTGGCTGTAACCCCACATGGGCTTTTACTTTTAGTGGGCACATTTTTGAAAATATTAATG
TTTATCCTTATCTTACCTTAATAACCATAATTATTATGGTTATTATGGATTAAATTGCTACTGTTTCTCTTTTTGTTTCC
TGGATTCAGACCAAATTAAGCCTTTATAATCTGTGAGCAAAATCACCTCAATTTTAATTGAATGACCCACAACTATTTAA
TCTTTTTGCCTATTTAAACAGTGTGACTTTTTTTCTCACTGAATAATACAAAAAGTACAAAATAAAGGCTAAACTTTAAA
ATATTCTTTTGTATCTAAGATTTGTAAGCTTTATAAGCTTTTAAAAAGTGCTTAGTAATTTTAAGTTACTTTTTTTTTTT
TTGAGATAGGGTCTTGCTATGTTGCCCAGGCTGGATTCAAACTTCTGGACCAACTGATCCTCCTGCTTCAGCCTTCTGAG
TAGCTGGGACTATAGGTGTTCCCCACTGTGCTTAGCTAACAGTTACATTTTTTGTTGTTTTTAATCACCTGACTGCCAT
AAGGTCTCCCGTAACCTGGTTACATTTCTTAGAGGACATCTTGTATGTTCTTCAGTCAGTATTTTATGAAACAATAACAA
CAGAATAGCATCAAAATATATGTCACTGGTAGGTGGCAAGCTATTAGTGCATGAACTGTATCGTCTTTTAGAAAAAGCAA
GCTATTTTCAGATGTCATTAAATTAGGAGCTATTTCTGGCTCATGTAGCAGTGTTGACTGCTCCATGTGTTACCATAATC
CTTCTGTGATGGAAAATCTACACTAAGTAAGGACAGCACCCTTCCCAGTAGCTAGCCTGTCTACATTCACAGACTGCTGT
TAGCTACTTCCATAGTCAAAATCTGTGTGTTTTCATCTGCCCAAGCTTTCCTCTTTTGCAACCTTAGGACAATTAGGAAC
TTCTCACATACTTGCCCAGAATTCCAGCAGATCTTCAGTTGGTGGTAACACCGTTACCATGAGCCAGATTTGAGATCCCT
AATATTCTGTGATCCCTGATGAGTGAAGGGAACAAGGATATGTGTAGGAGGGGAGCTCGGTATGACTAAGGGTCAAGAGA
AGGTGAGGCCAGAGAGAGCCTGAGCTGAGATCTGCTGAAACAGCTCCTAAAATGAAAACAAAGTTGGGGCCAGAATTTTT
TCTGGATAGTAGTTATGTTTTCCTGCCAACGCTCAAGTCCTACACAAAGACAAATGACAATCAATGTAAATGTCAAATAA
GATCGTTAGCCTGAGTAATCATAACCAATCTGTATGACACCTTTTTAACAGGAGGCCTCATTCTTCTTTTCCCCAACCAG
AATTAAGAGAAAAAAAGTGAATATGGTTTTTGCTCACAGAATGGATAACAGCAAGCCACATTTGATTATTCCTACACTTC
TGGTGCCCCTCCAAAACCGCAGCTGCACTGAAACAGCCACACCTCTGCCAAGCCAATACCTGATGGAATTAAGTGAGGAG
CACAGTTGGATGAGCAACCAAACAGACCTTCACTATGTGCTGAAACCCGGGGAAGTGGCCACAGCCAGCATCTTCTTTGG
GATTCTGTGGTTGTTTTCTATCTTCGGCAATTCCCTGGTTTGTTTGGTCATCCATAGGAGTAGGAGGACTCAGTCTACCA
CCAACTACTTTGTGGTCTCCATGGCATGTGCTGACCTTCTCATCAGCGTTGCCAGCACGCCTTTCGTCCTGCTCCAGTTC
ACCACTGGAAGGTGGACGCTGGGTAGTGCAACGTGCAAGGTTGTGCGATATTTTCAATATCTCACTCCAGGTGTCCAGAT
CTACGTTCTCCTCTCCATCTGCATAGACCGGTTCTACACCATCGTCTATCCTCTGAGCTTCAAGGTGTCCAGAGAAAAAG
CCAAGAAAATGATTGCGGCATCGTGGATCTTTGATGCAGGCTTTGTGACCCCTGTGCTCTTTTTCTATGGCTCCAACTGG
GACACTCATTGTAACTATTTCCTCCCCTCCTCTTGGGAAGGCACTGCCTACACTGTCATCCACTTCTTGGTGGGCTTTGT
GATTCCATCTGTCCTCATAATTTTATTTTACCAAAAGGTCATAAAATATATTTGGAGAATAGGCACAGATGGCCGAACGG
TGAGGAGGACAATGAACATTGTCCCTCGGACAAAAGTGAAAACTATCAAGATGTTCCTCATTTTAAATCTGTTGTTTTTG
CTCTCCTGGCTGCCTTTTCATGTAGCTCAGCTATGGCACCCCCATGAACAAGACTATAAGAAAAGTTCCCTTGTTTTCAC
AGCTATCACATGGATATCCTTTAGTTCTTCAGCCTCTAAACCTACTCTGTATTCAATTTATAATGCCAATTTTCGGAGAG
GGATGAAAGAGACTTTTTGCATGTCCTCTATGAAATGTTACCGAAGCAATGCCTATACTATCACAACAAGTTCAAGGATG
GCCAAAAAAAACTACGTTGGCATTTCAGAAATCCCTTCCATGGCCAAAACTATTACCAAAGACTCGATCTATGACTCATT
TGACAGAGAAGCCAAGGAAAAAAAGCTTGCTTGGCCCATTAACTCAAATCCACCAAATACTTTTGTCTAAGTTCTCATTC
TTTCAATTGTTATGCACCAGAGATTAAAAAGCTTTAACTATAAAAACAGAAGCTATTTACATATTTGTTTTCACTCAACT
TTCCAAGGGAAATGTTTTATTTTGTAAAATGCATTCATTTGTTTACTGTAGTTTTTGTGGGTTTTATTTTACTTGCTTTT
TATGTTTTAGGAAAAGCGTTCACTTTGAACTTTAGCCAACAGTCCTTTTACTATTAATATATTAGTTACATGCATAAAAA
AGAAAATGCTTTCTTACTATTTACTGTTGGTTCAAAAACATAAACCTAAGCCATACACACAGTCTTTGATTAATGATTCA
ATCTAGTTTTATTTTGTTTTCTTTTTCTCCTCCTTACTGAATTTTTATAACTATGGGCTTCAGTTCCAAATGAAATCCCC
GTGTCACCTATCTCAGAGATTTATATTTCTTTCACTAAGCAACATTTAGGATGAGTTAATATTGTATAGGTTGTCACGTT
ACCTTCCTCCAAGTAACCACTAGCCAGATCTGCTATGGAAAGCACGCCATATATTGGAACTTGCTGGCAAGTCTTCTGTG
GTTGGATTTTAATCAGGATTCTTAGGGGAAGAAAGCATACGAGTTGCTTAATTTCTCTTGCTCTTTCTCTTGTTTTTATC
CTTCCTTAATCTAAGCAGCTGATGAAGCCTTGAAAAATGCAAAGTTAATGCAATGTTTTCCTCACACCTGTGTCCCATCT
AGGCTGTGTCTAGGACATTCACCTCCCATTTTTGAGTTATATACAAGTAAATGAGCAGGTGCTGTTGCTAGCATATCTAA
AAAATGCCAAAGACAGAAATGACAGATGGCATTTCTCTGTGCTCTGCAATTCAAGCCGAGACTTTCAGCTGCCCAGCACT
ACCCTATACACCAGGACAGCAAGGGCAAGGACTGTCTTCCTCATTCTTGTAGAAACTGGAGGCCATTTCTCTAGCCAGAG
CCTGTTCTCCCTCATCTCTCTCCAGAGCACAGCACCATACCCTGGCCAACCTCAAACTCTTCTTTGCCTGTGATAACTAG
GGAATTTTTTTCCTCTGGTTCCCAGATCTCCATTATTTCAAATCGAGGACCAGCCAGCACATTTAAAAAATGTTTTTATT
TAAACAATAAAGGGTTTACAGACAAGACTGGTATGCATTTTCACGCCCTAGTTAACAAGAGCTGCAAATAATAACTCCAG
TTGTTCAGCAATTCTACATGCATTATCTCCTTTATCCTCTCAACAATTCTGTGATATATTTATTATCATCCCTTTTATAG
ATGAGCAAACTGGAACTCAATAGTTATGTGACCTGCCCAAGTTGGAATGTGACAGAGCTGGTATTCAAACTCGAGCCTCT
```

```
AACCGCATGTCTGTCTGTTGCTCTTTCCTTGACATTTCAGATGTCTCCCTAGACGACACGAGGCGCCAAACCCATCCAGG
CCCTGAAGGGGCCTCCTGACCACTGCCTTGGTGGAAGTCAAAGGTCTAAGAGAATGTGGCCATTCTTTTTTTCTTTTTTTA
ATTTTTTTTTTTTTTTTTTTTGAGACAGAGTCTCACTCTGTTGTCCAGGCTGGAGTGCAATGGCGTGATCTCGGCTCACT
GCAACCTCTGCCTCCCGAGTTCAAGTGATTCTCCCGCCTCAGCCTCCCAAGTAGCTGGGATTACAGGTGCCCGCCACCAT
GCCAAGCTAATTTTTGTATTTTTAATAGAGACGGGGGTTTCACCATGTTGGCCAGGCTGGTCTCGAACTCCTGACCTTAG
GAGATCCGCCTGCCTTGGCCTCCCAAAGTGCTGGGATTACAGGCACGAACCACCGCACCTGGCCTCTTTTTTATTTTCAA
TTTTAAAAAATCAGCTGAATTTAAGGTTCACTGGTGATTCTTAAAAAGGGTCTAGTTGAAATGTAATGAGCTGGCCTTCA
CCTGTTCCTTGGCTGTAGCTAGGTTCTTGGACTTGTGCTGCGGAATGTTTGCAATAAGGAGGGAAACCTGTGCCTTCCTA
CCTCCCAGACACAGGGGAATGAGACAAGATTACGTTTTGATATTTGGAACAAAGAATAACAAAATATTTAATCTACAAAA
CATTTACAGAGCTGATGTTTTTAAGTTGAGATGCTTTGTTCAATTGCAGATTCCAAAGAGAGAAATAATATATCATATTT
TTCTTTCTCCCTTTTCCCATCCTCCTCACATTCTTATCCCATCAGCAAATGGCTCTAAAACAGCAAAATGTGCCAGATAT
TTACCTCACTTAGCCCTTGAATTTCATGGTTCAGACTTGTTAAAATGTAGAAGAGATCCCTGCCGTAGTTTTATTGTCTT
TTTAAGTGAAATATTATTTTTTCGAGTTACAGTGTGAAAAGTTTAATTTGTTTCCATTTTGTTTTTATATATTTATAGGT
CTCGGTTTAAACTGAAAGAATTACACTAGGTGATTTCTAAGCTCTATTGTGTCTAACGTTCTGGGAGTTTACTACAGTA
GGTGGATATTCAGTGAGACAATGTGATACAAGCTCTTGTGTAAACAGTTTGTTTTTTTTTTCTTTTTGAGACAGAGTCTC
GCTCTATCACCCAGGCTGCGGTGCAGTGGCACGATCTCAGCTCACTGCAACCTCCATCTCCCAGGTTCAAGCAATTCTCA
TGCCTTAGCCTCCCAAGCAGCTGGGCCTACAGCCATCACACCCAACTAATTTTTGTATTTTTAGTAGAGACAGTGTTTTC
CCATGTTGGCCAGGCTGGTCTCAAACTCCTGGCTTCAAGTGATCCACCCGCCTCAGCATCCCAAAGTGCTGGGATTACAA
CACGAGCCACCATCCCGGCCAGAAGGTATTTTTTAATCCCCCAAATAATGACAAATTAAGAGTATTAAGTCACAGTCAAG
AGAGCCCCAAAGTCGTGTGACCTGTATTAATTCAGCTATGTTCTCTCAGGGTGTTGGTAATTCACTGTATTTTTGTTTAG
CTTAATATTCAGCATTCCTGGTGTTTCTCTTATCCTGAATTTGGAAGAGGCTTTACATAATGCACAGATGGATGAAAATT
TGCTTAAGTCCCTCAACACAAGAACAGCATTCACTACATCTCACATTTACTCCACAAGAACCAGAATTAAGCCACAGGGA
GACAATGAGGTGCACCACACACCCAGAATCATGTGGCCCTTCAAACAGGAGTACAAGAAAGGAAACAGTTGGAAGCAGGC
TCCACAGTAGAGGCAGTTCTCCTACCTGGCTCCAGGTTCTCGTAAACCGTCGGTGAGGAGGGAAGGGACAGGGATGCATC
CTTTGAGTGGAATCTGAATTCTCCCTTTCTTCTCATCTTCATCGAATACGTTAATGTCCAGCCTGCTGCTGAGTCAATAA
GCACATCCTGCACAGATGCGCTTGCCTGTGAGCCTTTCATCACAGGCTTAGCCCCCATGCCCGGAGCTGATACCCCGGGC
CAGACTTCTCCCTCAGGAATGACAATTTTGTCCTTGGTGGAGGCCCATCTCCACCAAGCATGTGGGGAGTGGAGTCGTTT
GCCAGAGAGTGCTGGCAATTTCACCTCCCCCTGTGGGCACAGACCCTCTTCCGCCCTTCCATCTTTGCTGCCATTTGCAC
AAGCTCACTCTGAAGTCACATAACCAGGCTCAGCTGGGGAGACCTGACCAGGATCCGCTTCCAGGTTATGCTCCTCTAAA
AACCATATGTAAAAGAAGCAAGGAATTAGACACTTTCAGAGCAGAAGGGATGTTGGGAGACCCAGGCCAAGCCTTCATTA
GTTAGAAGCGGANNNNNNNNNNNNNNNNNNNNNATGGGGTTTCCCCATGTTGGCCAGGCTGGTCTCAAACTCCTGACCTCA
GGTGATCCGCCCACATCGGCCTCCCAACGTGCTGGGATTACAGGCGTGAGCCACCATGCCCAACCCTTATTGTTTTAATG
TACATTTCTTTATGAGTGAGGTTGAATAGCTTTTTATATTTAAAAGCAACTCTTTTTTTTCATGTCTTTTGCCTGTTTTCA
TAGTGAATTGTATATCAGTTAGGTCGCTTTTGATTTTAAGTGACAGACCCAAATTGACAAAAGAATGAACTGCTCCCCTA
ACTAAAAATTGTGGGAGTAGAACTTCAGGCATAACTGTATTTGAGACTCAAACAATGTCATGATGAAGTGGCCTCACTGT
CTGGGGTGATTTCCTAGGTTTGTTGTCTCATGCCAAGGAAATCAAGGACATGGACACACTAGGAGTGAGGTTAAGAGCTG
AGGCTTAACAGGCAAAAGAAAGAGAAGAGCTCTCTCCTGCAGAGAGAGGGGTCCCGAACAGGCACATGCAGCAGGTTTTA
TAGGTGAGCTTGAGGAGGTGGTGTCTGATTAACATAGGGCACAAAAGATTGCATGGACCAGGTGTGCCATTTGCATAAGG
TGTGAAAAACTGGTTAGAGCTAGGGGTGCCATATGCATAGGGCACAAAAAGCTGGCTGCACCCACCCTAATCTTTTATTA
CGCAGATGGGTTATTTACCTGGCCGGTGCCATGTTGCCTGTTTCTTTACTGTACACATGGTAACAAAGAAAAGGGAAGAT
GGAGCCTCCATGCCAAACATACCTGGTTCCCAGGTAGCCCTCTTCTATTGGCACAGATGCTGGCATTCACCCATGTAAAC
TTCCAGTTGCTTATCTATTTTTGCAGCTCAATTTTTCAAGCTGTCCTTAGTTAGAAAAAAATAATTTGGGGGCTGCTTTT
TGTTAAAAGGGAAATTCTGCCAAAGACTCGTTTACCCTATCTGCCTAAATAATTTCTTTCTGGTTCCTGTATCAGTGAGA
GAGTTGGTACTCTCTCTGCCCTCTGCCTTGAAGTAGCTTGGCTTTACTGGGAGCAAATTAGAGTAGCAGCTCTATCCCCT
ACAGCTTTTCATATGCCAAGTTCAGCAGGAAAGAGCTAATTCACTTCTCACAGATATCATAAAAGCTGCAGAATTGAGTC
TCTTTGATTCCTGATTGGCTGGACTTGGGTCAGGCCCAACCCAGTCACTAAACCAATCATTTTCACCTCGAAAACATGAT
GGTCTAATTATTTCAGGAAAATACTCTGTCATTTTGAAGTAGATTGTATTAGTCTGTTCTCACATTGCTATAAAGTACTA
CCTGAGACTGGATAATTTATAAAGAAAGAGGTTGAATTGACTCACAGTTCTGCATGGCTGTACAGGAAGCATGGCTGGG
GAGGCTTCAGGAAACTTACAATCATGGAGGAAGGCAAGAGGGAAGCAGGCACGTCTTACATGGCTGGAGAAGGAGGAAGA
GGGCAGGGGAGGTGCCACACATTTTTAAACAACCAGATCTTGTGAGAACTCACTCACTATTATGAGAACAGCAAGGGGGA
GATCTGCCCCCATGATCCAATCACCTCCCACCAGGCCCCTCCTCCAACAGTAGGGTTACAACTCAACATGAGATTTAGGC
GGGGACACAAATCCAAACCATATCATTCCACCTCTGGCCCCTCCCAAATCTCATGTCCTTCTCAAATTGCAAACTACAAT
CATCCCTTCTCAATAGTCCCCAAGTCTTAACTCATATCAGCATTAACTCAGAAGTCTGCAGCCCAAAGTCTCACCTGAG
ACGAGGCAAGTCCCTTCTACCTATAAGTCTGTGAAATAAAAAACAGTGAGTTACTTCTAAGATACAATGGGGGTACAGGC
ATTGGGTAAATACACCCTTTCCAAAAGGGAGAAATCAGCCAAAACAAAGGGGCTACAAGCCCCATGCAAGTCTGAAACCC
AGCAGGGAAGTCATTAAATCTTAAATCTCCAAAATAATCTCCTCTGACTCCATGTCTCATATCCAGGGCACACTTGTACA
AGGGGTGGGCTCCCAAGGCCTTGGGCATCTCTGTCCCTATGGCTCTGGAGGGTACAGCCCCCTCGGCTGCTTGGCTGCTT
TCATGAGCTGGGGTTGAGTGCCTGTGCTTATCCAGGCATGGTGCAAGCAGGTCTCCAGAAATGTCTTTGAAGCATTTTCC
CCATTGTCTTGGCTATAAACATTGGGCTCCTCTTTACTTACGCAAATTTCTGAAGCCAGCTTGAATTCCTCCCCAGAAAA
TGGGTTTTTCTTCTCTACCACATGGTCAGGCTGCAAATTTTCCAAACTTTTATGCTGTGCTTCCTTTTTAAATATAAGTT
CCACTTTCAGATCATCTCTTTGCTCATGAATATAAGCATAGGCTGCTAGAAGCAGACAAGCCAAGTCTTGAACATTTTGC
```

```
TGCTTAGAATTTCTTCTGCCAGATACCCAAAATCATCACTCTCAAGTTCAAAGTTTCACAGATCTCTAGGGCAGGGGCAC
AATCCTCTTTGCTAATGCATAACTAAAGTGACCTTTACTCCAGTTCCCAATAAGTTTTTCATCTCCATCTGAGACCACCT
CACCCTGGACTTCATTGTTCATATCACTATCAGCATTTTGGTCACAACAATTTAACAAGTCTCTAGGAAGTTCCAAACTT
TCCCTCATCTGCCTGTCTTCTTCTGAGCCCTCCAAACTGTTCCAACCTCTGCCTGGTACGCAGTTCCAAAGTCACTTCCA
CATTTTCAGGTATCTGTATGGCAATGCCCCCACTTCTTGGTACCAATTTTCTATATAAGTCCATTCAAGCATTGCTATAA
AGAACTACCTGAGACTGGGTAAAGAAAAGCGGCTTAATTGACTCACCGTTCCACAGGCTGTACAGGAAGCATGGCTGGGC
AGGCCTCAGGAAACTTACAATCATGGCAGAAGGTAAAGGGGAAGCAGGCACATCTTACAAGGCCAAGAAGGAGGAAGAGA
AGGTGGGAGATGCCACACACTTTTAAACAACCAGCTCTCATGAGAACTCACTCATTATCACAAGAACAGCAAGGGGGAAA
TCTGCCCCCATGATCCAATCACCTCCCAATGAAACGAGAGAAATTCCCTTGTCCCCCTCGTAGGCCATGCAATGGGGGTG
TGCCTCACTTCTTCAGTGCCCCGCTGCTCAAACCTCTAAGGGAGCATACAGACAGGCGGGCTATGGGGCTCCAACCCCAT
GGCAGTGTCTAGGAGTGAATGTTTACAGCTGAAGCCCCAGTGGGTGTGTGTTACAGGGTGCTCTTTTAGTTTAGCTATCC
ATAGGTGGCTTGTGTTAGTCAGCTCAATTAGACCCCTGCCTCATCACAAGGACAGAGGGCTTTCTGTATCCCGGGATTCT
TGCCTTGGTGTACTGGAAGACTTGGATCGCATGTGGGCTTGGACCGTGAGTGCAAGGTTTATTGAGTGGAAGTAGCTCT
CAGCAGATGGTGGAGCCAGAAGGGAGATGGTTTTCTCCTGGAGTCGGGCCGCTCAGTGGCCCGGGCTCTCCTCCAACCAC
CCCGGCCAAACTCCACGTCGTTCTGCCGGTCGGTGGCCTGCTGGCATGCCAGCGTGCCTGTCAATGTGTTCTTGATATCC
TCTCAACATCCAGCCATTTGTGTGTTCCTCTGCCAATGTGTTTCTCTGGACATCCAACTGCTGGAGTGCCTGCCTGCTAG
GGTCTTGGGGGTTTTTATAGGCACAGGATGGGGGTGTGGTGGGCCAGGGTGGTCCTGGGAAATGCAACATTTGGGCACGA
AGGCAGGAGTCCCTGTTCTCACCTAGGTCCGTGGGCACAGGCTGGGGTGGAGCCCTCACCAGGGACCTGCCTTTCTCTA
CCCAGCACTTCCCTGCCTCCACTTCCATATCACCAACACTGGAGGTTGGAGGTTGCAATTCAACATGAGATTTGGGCAGG
GACACAAATCCAAACCATATCAAAGATATTTTCTATTTTATCATTTGTCTTTTAAAGCTTTGTTATGGCATTTTTTCAAA
CAGACACTTTTCTGTAGTCCATTTATCAGTCTTGTGGGTTTTGCCTAAAAAGCCCCCCGACTCTGATTTTTTTCTTTTAT
TTTCCATGNNNNNNNNNNNNNNNNNNNNCTCCCTCAGGAATGACAATTTTGTCCTTGGTGGAGGCCCATCTCCACCAAGC
ATGTGGGGAGTGGAGTCGTTTGCCAGAGAGTGCTGGCAATTTCACCTCCCCTGTGGGCACAGACCCTCTTCCGCCCTTC
CATCTTTGCTGCCATTTGCACAAGCTCACTCTGAAGTCACATAACCAGGCTCAGCTGGGGAGACCTGACCAGGATCCGCT
TCCAGGTTATGCTCCTCTAAAAACCATATGTAAAAGAAGCAAGGAATTAGACACTTTCAGAGCAGAAGGGATGTTGGGAG
ACCCAGGCCAAGCCCTTCATTAGTTAGAAGCGGAAATTGAGACCAAGAGATGTGAAGTTAGGGCTAACCTGCAATGTCCC
CTACCCACCGGTTAAACTTTCTTTCTTTCTTTCTTTCTTTCTTTTTTTTTTTTTTTTTTTTTGAGACGGAGTCTCCCTCT
GTCCCCCGGGCTGGAGTGCAGTGGCGCGATCTCGGCTCACTGCAAGCTCCGCCTCCCGGATTCACGCCATTCTCCTGCCT
CAGCCTCCTGAGTAGCTGGGACTACAGGCGCCCACCATCATGCCCGGCTAATTTTTTGTATTTTTTTTTTTTATTTTTAG
TAGAGACGGGGTTTCACCGTGTTAGCCAGGATGGTCTCGATCTCCTGACCTCGTGATCCACCCGCCTCGGCCTCCCAAAG
TGCTGGGATTACAGGCGTGAGCCACCGCGCCTGGCCTCAACTTTCTTTCAACTTTACCATTTGGCTTGCTGTGGCTTTAT
TTATATATTTATTTATTTATTATTATTCAAGATGGAGTCTCACTCTGTCGCCCAGGCTGGAGTGCAGTGGTGCAATCTCA
GCTCACTGCAACCACCGCCTCCTGGGTTCAAGCAATCCTCCTGCCTCAGCCTCCCAAGCAGCTGGGACTACAGGCATGCG
TCACCACACCCAGCTATTTTTGTATTTTTAATAGAGACGGGGCTTCACCATACTGGCCAGGCTGGTCTTGAATTCCTGA
CCTCGTGATCCACCCACCTTGGCCTCCCAAAGTACTGGGATTACAGGCGTGAGCCACAGCGCCCGGCCTGATTCATTTAT
TTTTTAATACATTAGTTTTATTTATTCCTCAGCACTCTGGGAGGCCAGGCAGGCAGATCGCTTGAGCCCAGGAGCTTGAG
ACCAGTCTGGGCAACATAGTGAGACCCCTCATCTCTACAATTAGCCGGGTGTGGTGCGTGTGCCTGTAGTCCCAGCTACC
CAGGAGGCGGAGGTTACAGCTGAGATCTGCCACTGCACTCATCTTGGCTCACTGCAACCTTCGCCTCCCAGGTTCAAGTG
ATTCTCCTGCCTCAGCCTCCTGAGTAGCTGGGATTACAGGCCCCGTGCCAACACGCCCTGCTAATTTTTTTGTATTTTTA
GTAGAGACGGGGTTTCACCATGTTGGCCGGGCTGGATGTATTCGATTTTTAAAAATCCAAACTGTGTGCAGGAGAATATG
AAAGGAATACATAAGCCTTCACTGTTCATTTAACCTGCTAATTCATCTGTGAAAATGATGACCTTAGAGTGCCATAATTA
GAGAACAGTGGAAACAAACCGACTTCCTCTAACAGTTTTCCAGAATGACCGCTTGATGGCACTGTGACTTGTGGGCAGAG
CCCACCACCTCTTCACGATGCCTGTCACACTGGCGAATTCAGGGAAGTCCTCGTCTCATAGCAACAAGACAGGGAAAATT
GCTGAGCACCCAGAAGCAGCTGTAGATTAAGTAATCAATCAGTGTGTTCAAAACACAAAGCATTTAAACACTGGAGGAAA
TTCCAAAATAATACCCTTTTCAGACATGAAATCACTAGTCAAAATATTGTTTTAAACCCATTCTACAAATGTGATCATTT
TCAAAAAACTCCATCAGACCACCAAGCTGGCATTAGTGCCACCCACACTTGCCAGGGCCTGTCTTTTCCTTTTCCGCTCT
TTTCATCATTGACTCAGAGAATTCATGGACTTTTTCATCGCAGTGTATCTAGAGTTTCCCTACAAGTGAATGTTCATATG
CTGCTGGTAAGCAGGCAGTTAAATGTATTTTAAAGTAAGCCCCTACAATCCGGGCCATTCTGAACTTTTTTGGACAATTG
AACACTTCAAGAGCTCTATATTTTCTTTCTCAGAAGAAGCCTCTTTTAT
```

HUMAN mRNA SEQUENCE : hR5-033.1 (Seq ID No: 47)
```
AATTAAGAGAAAAAAAGTGAATATGGTTTTTGCTCACAGAATGGATAACAGCAAGCCACATTTGATTATTCCTACACTTC
TGGTGCCCCTCCAAAACCGCAGCTGCACTGAAACAGCCACACCTCTGCCAAGCCAATACCTGATGGAATTAAGTGAGGAG
CACAGTTGGATGAGCAACCAAACAGACCTTCACTATGTGCTGAAACCCGGGGAAGTGGCCACAGCCAGCATCTTCTTTGG
GATTCTGTGGTTGTTTTCTATCTTCGGCAATTCCCTGGTTTGTTTGGTCATCCATAGGAGTAGGAGGACTCAGTCTACCA
CCAACTACTTTGTGGTCTCCATGGCATGTGCTGACCTTCTCATCAGCGTTGCCAGCACGCCTTTCGTCCTGCTCCAGTTC
ACCACTGGAAGGTGGACGCTGGGTAGTGCAACGTGCAAGGTTGTGCGATATTTTCAATATCTCACTCCAGGTGTCCAGAT
CTACGTTCTCCTCTCCATCTGCATAGACCGGTTCTACACCATCGTCTATCCTCTGAGCTTCAAGGTGTCCAGAGAAAAAG
CCAAGAAAATGATTGCGGCATCGTGGATCTTTGATGCAGGCTTTGTGACCCCTGTGCTCTTTTTCTATGGCTCCAACTGG
GACAGTCATTGTAACTATTTCCTCCCCTCCTCTTGGGAAGGCACTGCCTACACTGTCATCCACTTCTTGGTGGGCTTTGT
GATTCCATCTGTCCTCATAATTTTATTTTACCAAAAGGTCATAAAATATATTTGGAGAATAGGCACAGATGGCCGAACGG
```

```
TGAGGAGGACAATGAACATTGTCCCTCGGACAAAAGTGAAAACTATCAAGATGTTCCTCATTTTAAATCTGTTGTTTTTG
CTCTCCTGGCTGCCTTTTCATGTAGCTCAGCTATGGCACCCCCATGAACAAGACTATAAGAAAAGTTCCCTTGTTTTCAC
AGCTATCACATGGATATCCTTTAGTTCTTCAGCCTCTAAACCTACTCTGTATTCAATTTATAATGCCAATTTTCGGAGAG
GGATGAAAGAGACTTTTTGCATGTCCTCTATGAAATGTTACCGAAGCAATGCCTATACTATCACAACAAGTTCAAGGATG
GCCAAAAAAACTACGTTGGCATTTCAGAAATCCCTTCCATGGCCAAAACTATTACCAAAGACTCGATCTATGACTCATT
TGACAGAGAAGCCAAGGAAAAAAGCTTGCTTGGCCCATTAACTCAAATCCACCAAATACTTTTGTCTAAGTTCTCATTC
TTTCAATTGTTATGCACCAGAGATTAAAAAGCTTTAACTATAAAAACAGAAGCTATTTACATATTTGTTTTCACTCAACT
TTCCAAGGGAAATGTTTTATTTTGTAAAATGCATTCATTTGTTTACTGT
```

HUMAN PROTEIN SEQUENCE : hP5-033.1 (Seq ID No: 48)

```
MVFAHRMDNSKPHLIIPTLLVPLQNRSCTETATPLPSQYLMELSEEHSWMSNQTDLHYVLKPGEVATASIFFGILWLFSI
FGNSLVCLVIHRSRRTQSTTNYFVVSMACADLLISVASTPFVLLQFTTGRWTLGSATCKVVRYFQYLTPGVQIYVLLSIC
IDRFYTIVYPLSFKVSREKAKKMIAASWIFDAGFVTPVLFFYGSNWDSHCNYFLPSSWEGTAYTVIHFLVGFVIPSVLII
LFYQKVIKYIWRIGTDGRTVRRTMNIVPRTKVKTIKMFLILNLLFLLSWLPFHVAQLWHPHEQDYKKSSLVFTAITWISF
SSSASKPTLYSIYNANFRRGMKETFCMSSMKCYRSNAYTITTSSRMAKKNYVGISEIPSMAKTITKDSIYDSFDREAKEK
KLAWPINSNPPNTFV*
```

Table 9

MOUSE GENOMIC SEQUENCE : mD5-034 (Seq ID No: 49)

```
CAATACATGGCTGTCATTAGCAAAGGATCTTTCATCACGTGTCCTTTCAGGTGCTTGCTTTAGCAGAACATCCTTTCACT
TGTGTCTGCTTCAGAAAAACACTCCTTCGTGTGTTTACACCAGCAAAACACTATCCAACACAACTTACTTTCCAAAGAAC
TCTTAAGTTTCCACTTCACATGGGTTATATGAGACTGTTGGGGAGATGGAGGGAGATAAACCAAAATAAAGGGAAAATCC
TCATCCTTGGGATATAGTTTGTATTTTAAATTTTTTATTATTATTAATTTATTTATTTATTTATACATATGCTTGTCTAT
GTATGAGGAAGTCATTAGGGCATCAGATCCCCTGGAGCTGGAGTTACAGGCAGTTGTGGGCTGCCTGATGTAGGTGCTGG
GAACCAAACTCATGTCCTGTGCATGAACAATATGTGCTCTTAACCAGTGAGCCATCTTTGCAGCCCACCTAGTTTGCCCA
TCATAAAATCACCTTACAATTTCATTCGCAAAGAAAAAAATGTTATTGTCGGTGTCTTTGTTGTTGTTCAGACAGGTCT
CATACGTCAGACTGGCCTAGAACTTGCTATGTATTAGAAGGTGAGCTTGACCGTTTGATCCTCTAGCTACCTCCTATGCT
AAGAATACAAGCACAAGCCTCCCAATGCAGTTTATGCTGCCCTGATTGAACCCAGGGCCTCGTGCACTTTATACAAGAGC
TCTGTTGACTGAGCTACATTCCCAGTCTGCAGAGTTCAGCAAGACTCCAGAAACAGACCTGAATGAGATGGCAAGTGACT
GAAGAAAGATGGACACACTGATAGAAAGCTAGGACTTGATTTTATATTTAATTCAAAAATTAATGATTAAGAGCTTGCC
TTTTTGCAATTTTTTATCCTGTTATAAACTATAAACTATCTAAGGAAATGTAGATGTTAAAAAATATATTTAATGTATAT
GCATATTTATATGTATATATACACACTGTAGCTGTCTTCAGACACTCCAGAAGAGGACATTAGATCTAATTTATGGATGG
TTATGAGCCACCGTGTGGTTGTTGGGATTTGAACTCAGGACCTTCAGAAAAGCAGTCAGTGCTCTTAACCGCTGATCCAT
CTCTCCAGCCTGGAAACATGTAGATTTTAAATATTAAATAATCATAATAAAGATATTTTATTTATTGCCAGCAAAAAATA
ATAATAATAAAGAAAGCTAGGATCTTGTGATCTGGGCTCTCGGATGGAGAAACCGCAGCAAGTGCAGGGTGTTTATTATA
TAGAATCAACAAAGAATGAGGGTATTGTATACCGCTGAACAAAGAGGCAAAGTTATTGCTTAAGATAAACAAGGAGGCAG
GGTTTGTAGTATATAACTGGACCAAGGAGACAGGATGAGCTAATATCAGTATGAGCAGTCTCTCTCTGTAGGCAAACTGT
CTTCAAGCCATAACTGTCTAGGGGTAGCGAATTACTGTGAACATTTTCTGCACACACTATTAATATCCACACATGGCCTA
GAAGAAGGAATTGCCATTTCCCTCTGTATCTGAGGCTAGGGTCCTTGACATGGCGATGCCCACGACAAGCACACACACTC
AGTACCTCACATACTCAGGACTTCCTGAAGCAGCCTCCAGGAAATGTTCTAAAATAATAAGTAAACCATATACATTTTAA
AGGATTTATTTTATTTTAAATTATATATATGTGTGTCTGTGTGTGAGTATATGCATATGCATGCAGGTGCCCTAGGAAGC
CAGGGGACAATTTCAGATCCCCTCGAACTGGAGTAACAGGCAGTTGTGAGGTACCAAACATGGATGCTGGGACTCAAACT
CAGGTCCTTTGCAAGAGTGGCAAGCATTCTTAATTTGAGCTATCTCTCCAGGACCCCATTAAACTGTGTACTTTAATTTA
ATCTTTAAATTTTGCTTCGCTTGTAACTCTTGTTGTAAATCACATCCTTTGAGTAATGTAGAAAGCCACAGAATCATAGG
GCCGTTGTGAAGACACAGAAGCCTTCCTCACTCTTGAGAAGTTGGCAGCCACGTTTGGATGTGTGCCTGTCTTTCTCCTG
CTTGCCTCCGTCTCCAAGTACTCATGCTTAAATCTGAAAAGGATGCTCTCTCGCCTTTACTTCGCTTCCTTTGTTTCACT
CTGAGAGCAAGATAAGGGATCAGAGTGGAGCCATCCTCATGGCAAGCATGCAGGAGGACCTGCTAAAGGCAGAGCAGAGT
AGGGTGTCAAACGCAGTCCTATAAAGTCATCTGCAAATCATTGTCTCCTCATTACCCTTTTTGTTCTTCCATAAAAATTC
TTCTCTCAGCCAGGCGGTGGTGGCGCACGCCTTTAATCCCAGCACTCGGGAGGCAGAGGCAGGTGAATTTCTGAGTTCGA
GGCCAGCCTGGTCTACAGAGTGAGTTCCAGGACAGCCAGGGCTACACAGAGAAACCCTGTCTCGGAAAAAAAAATTCTTC
TCTCATTTATTACATCCCGACCACATTGTCCCTTCTTCCCCCACCCTCCCTATTCTCTGTTACTCTTGATTCAGCTAACA
CCCTTCCCCAAAACTCACCTCCCTATCCCTGCCTCCCTCCACACTGGGCCAGGTGACCCAGGCTGGCCTAAAACTTGAAA
TTCTCCTGCCTCTGTCCTGCTAGGAGTACAAGTATACATCACCACACCTGACGCCCTCCCCACCCCCGCCTCCTCTTCCC
TCACTCCCTGCTTTTCAGTCTTTCAGTCTTTCATTCTCGCTCTCTCTTTTTTTTTTTTTTTTAATTTCTGAGGTAGGGT
CTCATGGATCCCAGAGCAGCCTCAAACTCACTATGTAGCTGAGGACGACCTTAAATTTCTGACCCTCTTGCCACCTCCCA
AGAGCTAGGATTACAGGGTACAGCACCATGCCCTGCCTTCCAACTCATCTCCCCATGCAAGCCCTTTCTTAGCTAGATCA
GAAGTGATCCTGATTTATAAATTCTTTGGGCAATTATGTGAGAACATGATTCTATTCACTAGCAGGGCAGTTATGTGAGA
TTCTATTCACTAACAAGTAAGGTAAAAATTCACCTGAGTTCCATCCGATGAATGGGGATAGGGGCAGGCTGTCCTAAGCC
ATCATCCAAAACAACTTGAGGTTTTAATGATATAATTTCCTTTTTCTCTTCTGTCCCATCCCCCAGCCAGCCAGGCTCAG
CTAAGAGCTTTAACACACTGCTGGTTTCCTTGGTAACCACCTCGGGTTCCCACTCCAGTGTCTCTTGTTCTTCCTATTCC
```

```
ACCCTCTCTTTGTGTGTTAGTATTCTGTCTAAACTCCACTCCACAGTTACCTGGCAACAGCCAGGTAGGCCTGACCCACT
ATAAAAGGGGCTGCTTGCCCCCTCTTGCCTCTCTCTCTCTTGCCTTCTTGCCTTCTTGCCTCTTGCTCTCTTGCTCCCTC
TTGCTTCCCCTCTCTCTCCATTCCCTTCCCCACTTTCTTCCCATTCCCTTCCCCCCTCTCTCCACGTGGTCATGGCCAGC
CTCTACTTCTCTATTCTCTCCCTCTCTCTGCCTTTCTCTGCCTCTATTACCCTCTCAACTCCCCTCCCCATGCCCTGAAT
AAACTCTATTCTATACTATACCATCGTGTGGCTGGTTCCTCAGGGGGAAGGGATGCCTTACATGGGCCCACGGAGGTACC
CCCTTCCCCCACACCTCACCACACCCCATAACATATCTTATACCTCTTTATCTTTTTATAAACACATCACTCTGGAGGGG
CTCTCCTGCTTCCAGAAGGGGACAGCTTTGGACACGGCCCAGTCCCCATTGGGGATTTGCTGGAGAGCAGTGTCCCAGC
ACCCCATGCTAGGCACACATATCCATGTTCCATGCTCTTCCTAAATTCACATAGAGGCCTTTCAAATCCTGTTGAATTTC
.AAATGAAATATTCACAAGAAAAAATGTGTTCCCTGGTCAGAGCTGAACAGACTGGGCTTAAGTATGGACTCTGAAGCCAG
CCCGGCTTGAAATCCTGCTTCTGATATTTATTTGCTATGGATCTTGCAGAGTAATCTCTTGGAGCTTGATTCCTGTCCTT
AAAGTAAGAATAGAATAATTGAGCTTTTCTGAAAGTGTGTGTGTGTGTGTGTGTGTGTGTGTGTGTAAGTGCTTCAAACAGT
AGCTGGCATATGGTAAATATCACATAAATGTTACTAATGAATATTATTGTTACCAGCCATCTGTCCACTCACCTCTGGGC
ATTTTGGTTAAGCCAGCAATTGACCTGGAATTCTGTGACTAACAAACTCTTAGGAGCCACAAAGGAGTGTATGCTTTTGA
TTTGATATCTCTTTGAGGGATTTTTGATAGTTTTATTTTTGAGATTATAATATAATAGCATCATTTCCCTCTTCCCCTTT
GTGTCTCAAAATCTTCCCTGAACACCCACTCTTCCTTTCTTTCAAATTCATGGCCTTTTTTTATTGTTGTCATATATATG
CATGTATGTGTACATATGTTCCTAAATAAATAAGTACCACCTTCTCTGCCTGTATAATCTCACTTGTATGTATGTTTTCA
GGGCTGACCAATTGGTATTGGATACCCAATTGGTTTGGTATTCTCTGATTTCTCCCATACTTAGTATTCCCTTAATTGCC
AGTTCTTTGTCTAGATTTGAGGTCCTGGGCTTTCTGTCTGGACTCTGCCTTTACTGTGCTCTGGATCTAGTTGACCATCT
GTCAGGTACCCTAGATGGAGAGGCCACCCACCCCTATACCTATAAATTTAGATATGGAAATTCAAGATGGCACTCAGGCC
CCAAGGCCAAAGGCCATTACAATTCCAAACCTCATACAAGTTCAAGACTTTGATTTTCCAGTATGTGGTGACTCAAGGCA
AATGAAGCTGTTTCCCTGGGTGTGTTCAGCAGTGGTAGGAAGCTGAGCTGCCACCACCACTGAGAAGCCTGGCAAATGGT
CTGAGTGCCTTCTAATAGACAGACAGTATAGTTTTCTCTTCTGACCCAGAGGGGAAAAATAAGACACAGGGAGATGAAAT
AAACTACACGTGGTGGGGCCACTAGTAAATGGCAGCGCCAATAACACAATTCAGCCTGACTCACTATTCAGGGACTTACT
GTGTATCAATCCCCCTGCCTCACATGGACGGCAGGGCTCTGACAGGCAGAGCCCTGCTGCCTGTGCCTTATGCAGATGGA
TTCCTGCCTAGATGGAGAAATCGGAGCTCAGATGTAATAAGAGGCAGAGCTAGTGCCACAGCACCATGCACAGGGTCCCC
AAAGCTAATACCATGCTCTGCTGGGATTGTAGAAAATTAGTTTTGACTAACTGAGTTATTTTCCAGCAGTTCAGAGAGTT
CAAGTGAGGCAGGATAGAAAGAAAGACAGGGAAGTTTGAGAAGACATGAAAATGAGTCCCTAACCACTGGTGTGAAGACG
GGAATCTCACATGCAGGAGTACAAACTATGGTTTGGGGAAAGCATATGCCTTGGGCCAGCTCAGTGGTTGTAAACAAGGG
CACAGTGAAACCACCGAACTCAACCACTCCATCATGGGTGGGAAAAAGAAAAGTTTATTTAGGAAAAGTTCAAAATTGCC
ATGGCTCCCTCTTGTGGGAGGGTGCAGAGAATAGCCAAAGGCTTTTAGGAGAATCCAAAGCTGCTATGGCTACCTCTCGG
GGACAGCAAGAGGGACAGCAAAAGGGTAGAGAAAGCCTTGCCAGTCGTAAAGGGGGAGTAGCTACAGGGAGGGAGGTGTG
CAAGCCAGAACCACCTGGAGGGTTAGGGAAGTGGGCGGGGGAGGTGAGGATCAACGAACTTTCATGTGTGTTACACGTAT
GTGCTACAGGGAAGCAGATGCCCCATTTGCAGAGGTTGCCAGAGGTAGGGAATTAGTGGCTCCTCCTGACAACAGACACA
GGTTTGGGAGGAATGCTGACTGTGAGCCTTTGTCTGTCTGTCAGCAAGCCTTCCATTTACTCTGTCTGAGTCCAGACAGA
CAGACATTAGCAGTGCCATTTTAGAGCGTTTGGAACCTAACAGTGGTATGAAGTCAGCCTGGGCAGCATGGACTTTATGT
CAAACCTAACACAATAGCAACATCGAGCCACTTAGAACAGCACCTGAGGTTAAAAAAATAAATAACAATAAAAAGAAGGT
GTTCTTATCACCCTGCTGGGGCTCACCCAGCTCCCAAGATAGAAGCAGCCTTGGCACAACTGCCAAAAATATGCCTGACC
CACTCAGTCCCTCGGCCCTAGCACACTTTGAAAGGGTTGACTCCCCGAAGGCTGAGAAGCCATATTATGGGCAGAGGGAA
GGACCACACTCCACCCAATATCCTGTGCCTCGGTCTCCCCGTCCCTGGGTCTTCTTGTAACTTGAGTGCTCCTTCCCTTG
GGAAATCTGGGGCCAAGGGGAGAAAGCCTCAAGACTTAGGAGTGTGGAAGGAAGATGCCCAGAGATGAGACACCTCATTT
CATGACTCAGTCTTGTCTAGGCAGACTTTTAAGGCAGCCTGGGGCTGACAACTGAGTTAGGGAGCTTTTGGAAACATGAC
TAGGGAGGCTATTGTGTGGCTGTGAAGCAGAGGCTGGGAGAGAGAAGGAAATGACCCCTGTTCCTGATGAGGGGGCCCTC
CTTCCTGTGAGGGTCTGGGCACAAAGGTGTCTATCTCCCAGCCTCCTGTCTCCCCAAACCACAGCTCAGAGGTGTGAAAG
AATGAAAAACAACAGGGGGAGGGGAGGCAGAGAAGAGCCAGGTGCACATTTCACACCTTGACCACTGATGAGGCCCCAGG
CAGCTTCACTCTGACTAACTTCCTCCTCAGCCCTCTCTGCTGCAGAGCAGGGAGGGCCCAGAGACCAGAACACAGGCTGT
GAGCTTTTGGCTAGGTAGTTGCCTCTGACCTTGTCTTGGGATGAAAAAGAACCAAATTAGAAGGCCAGGTGAGGGGTTGG
CTCAGCTCTGTGACCCAGGAACCTTCCAAGGTATCTCAACTGCAAACAGCAGCTGAAGCCCAGCACTGCAATTTTGAGAC
TTGGGAGTTGGGTTGGTCCCAGAAGAGGAGGAGCAAGCCACTAACTGGGCACCCATCTTTGCCACAGACTTGCCACGCAT
ATGGTGCACAGTCCATAAAGCAGTCCCATCAAATGCATGCTATCCTTTAGAGCCACTCTTCAGATAAGGAAACTGAAGCA
GAGAGATGAGATGCTTGCCCAGGGTCAATAGCTGGACAGCCAAGGAATTTAGCTAGAACTGGCAGAGCCTGCTCTATCAA
TTGAGGCCAGAGACCAACCACTTTCATGGAGGACTAGCCCACTGGCCCTGGCCACAGCCACACTTACAGGTCCCATAAGC
CACTACTCTCTATCATGGGCTCTCTTGGCATGGAAAGTTCTAGGTAGTCCTGGTTCTGGGGTCTGCATTTACTTACTTCC
GGCCCTCGGCTGCACCTGTGCGAAGCCCTTGGCCCCCGTGCTAGATGCTTAAGCCTATGGTTATCTAGGTTAATCTTAGA
GCAACTCTGCTGAGGGAACTGCCTGCTTATGGTTCTGATCGTCCAAGATCTCAGAGGGCTCGTAGGGTGGAACTAGGCC
TGGAGTCCAGACAAGGTCGGTGCTCTGCCACCCCCAAGACAGATATACAAGTCACTCAAGCAAACCAAGAGAGCCACCTG
GTCTATCGGGGCCTAATGCTAACATCTGGCTGCCAGGCCTCAGAAAGGAAGGCTTCTCTACACATGACCTTCAGTCCAGC
ATCCCTCCCTGCAACCTGGGAAGGTAAAGATCAATGAGGACAAATGGGGAGACTAAGGGCCAGGAAGGAGATTAGGGGAC
CCTAGACCTTTACACTTCATATAGATCAGTAATGTTTCAGCAGCCACTGGGAAGAGGCACACTGAGGGGCTGGCTTCTGC
CACCATGAGGCATTTCTAAAATGACCATCTGTTTTCACTACCTTTTCTTTCATAAAGCAAAAGTTCCCTTTAAAGCCAAA
AAATGGGATGCGGGACACAATGGCAGACTGCATCCTTGAACATGAACAAGTGTGGGCATAGCCTTTACAGTTATTTTTCT
CATTTGCATAAGTCACAGACAGGCATGGGGAAACTGTGGTCCTTGTGGGCACTAAAGCAATTTTGTCGTGTTTTCCAGGC
```

```
TTCTGTCATCTCTGTCTCTATAGTCAGTCAAGTGCCAAGTCATCTACATCCTGACTGGCGGGCCCTCAGGAATCCTTAGT
CCCCTCCATACACACAGCTCCTGACCTCGTCTAAGACCTCGGGTCCTTTTCACCTGAGCCCTGCAGGTGGTGGTTGTTTC
AGAGAAGTGTCCCACCTGTCCATAGGAAGAGACGAGCGCTAAGTTTGGGCAGGAGCATGCCATCTCAATAAAGTTGGACT
GAATTAGTGGAGGAGCAGTTTGTTTGAAAACTGAGCATCAGCACGATGTGGTGGCTCATAACTGTAATCTCAGTTCTCCA
GAGGCTATGGCAAGAGGAGCATGAACTCAAAGCTAGCCTGGGCTATATAGCAAGTTCTTGTTTCAAAAAGAAAATTGCAT
TTAGGATAAACACGATGGCCCACAACTTTAATCCCAGCAGCCAGGAGGCAGGCCCCTGAGCTTGAGGCCAGTCTGTGGAG
TTCTAGTCCAGCCAGTGTCACATAGTAAGAGCCCTGAAAAAAAAAAGTTATCCAGTCCCGACTTCCACTACCCAGCACTG
ATGCATGCATACTGTAGCCTAGTATGGCACTTTCTACATGTGCATGGGTAGTACCAAAGTACATACAATAGTCTGTTACT
TGCCTCTTCTACCACATGGGCATAAGCTCCTCTGCATTCAGCCTCATGAAAAATATGTGGCTCTCCACTTGTGTTTAGCT
GGATGAATACATCAGTTTGTCTATTTACAAGAGAGTGGACTGCCCCACCCTCCTGCCACATGCTGTTCACAATGGTGCAC
TCCAATCATCCCGGCTCTCGGCTCTCGTGAGGCTGAGCCAGGAAGGTCACTGAGAGTTCAAAGCCAGGTTGGGCTACAGA
ATGAGGCTATCTCAGAAAGAACCCAAACCCAATTTTAAGAGTGCTGGCGTCCTTTTCAGACCACCGACTTTGAGATGGGT
CTGTAAAAGCAGGCACCACCCTGGGACAGACCAGCACACAGTCTAGGTGTGATTCAGAAGCAGTCCGTGCACCTGGTAAC
AGGGCTGCCCTCTACTTCCCCACTGTGTGGCTTCAGACCTTTCCTCAAAGAACCTTCCCCCAGACAGCCACCCCAGACCC
TTCCCTGGGTCCCAGGTTTTTTGCTTTCTCACCAGATGGAACTCCAACATTCCTGATAGCCTGTTGCCCTATCAGCTGCA
CTGCTTGTCTGCGCCACACATGCCCACCTGCCGATCACTGAGGTATCAAGCATTTCCCAGCCCCTGAGCGTGCTCCTGAA
TAGTTCCATCTGATCTGGACATTAAATGGACATTCCAGATGACTTTGGGCTCATTATAATCCCATCGTGATTTCATTCAT
ATATATATATATATATATATATATATACATCAATGCCAGGTCTGATTCAACCATGCCCAAATGAGAAAAGACAAGA
GGTGGCAGGCATCTCTTGGAAATGTCCTCCCTACTCATGCAGTTATGATAGCAGTAGCCACACCACAGCCCACTCTAACA
CAGTCCCAGTGAGACTCCAATCCCAGTACTTGCTAAGGAGAAGGTCCCTTCAGTTGAGCTTCCCTCCTGCAGTCACTGCC
ATCAGATCACACTTCCTGCCTGCTCCTAGACTCACTTAGTTGTTTATGAATGATATTGAGTAAGACAAGAACTGCAGGCT
TTGTAACAGTCCCAAGTTCCTGGCTAGCCCCTTCTAAGTGTATTCACCTGACCACAGGCGTGGCAGAAAAGACAGAAAAG
CCCTCTGCGGAGGGCTGGTATAGATTACCCTTTGGAAGAGGCTGTGCTGAGCCGTGAAATCTTCACTTCCTCTGTTCACT
GCAAGTTCCCAACCACAAGTGCCAAAGGCAGAGAAGCAGGCAGCACGAGACCTGACCCCAGCAGCCACAGCCGGAGCACC
AGCCAAGCCATGTACCTTGAGGTAAGTTCTGCTGCCCAGCTCAGCTACAATACAGCGGCCAAGTGGCCGAGCTCGGATAC
CAGGGACAAGCGTGGGCTGGCCCACAGCCAAGAGGCACCAGCGGGCTGAGTGAGCAGCATCGAATGCAGAGGTTCTGATA
ATAAGAGCATTGGTGGATTGGGAAACCCAGCCTGGCCACTAACCACAGGCCTGTCTCCTGCCTCTGTGGCTTGGATGCCT
CATCTGAAAATGTCAAGCTGTAGAAACTCAACTTCGGTGATCTCTAGTTCTCAGATAGTTTGGGATAAGGTGTAGAGCTG
AAGTGGAAGTGGTATGCCCCCATTGTCTGAGCTACTTGGGAGGCAGAGGCAGAAAGCTCATCTGAGCCCAAGAGTTTGAA
ATCAGCCTGGCCATCTTGGGAAGACCAATTAGGGCTTGGGTGGGAAGCTCAGTGGGTACCCACTGCCTCTGGGTAGGGGA
AGAGAGGCATGTACGGTGTGTGTAGGGGTGCACAGTGGTTTGTAGAAAATCCTTATGTGAGACCAGGAAGTCAGGGTCAC
CAGATAAGGGCTGAGGAGGGAAAAGGAGGGCCAGGGACTAGAAGATAGAGTAAGGAAGCACCTGGGGAGGCCAGTGGTTG
ATCCAGGCAGGGGTGGCCATAGCAGCAAGGCTGACAAATCACCAAGCGTAAAGAGGAAGGGAAAGAATGGAAGGCTCCAG
ACCCTGGAAGGAAGTTCCTCTTCCGGCAGAGAATCCCTGTCCTGGATGATAATCCAGAAAGGGTCTGGGGGGGGGGGGGC
CGGGGGGGTTACCAGCCTTTGAAAAGGGGGGGGTCACCTTCCCAGTCTGAGTAGATGGAGTTAAGGAGATGTGGTCCCCGGA
GAGAAAGAGTCAGCTCAGGGTCTGGCTATTGCTAAGGGAAACAAACACTCACCAGAATAAATGACAGGGCACAAAGGGGC
AAAGCCAGTTGCTGGGTCTTTACAGAGAATGGCCTCCCTTCCCTCTCTCCCCCTTCCTCGGGCAGGTTAGTGAACGTCAA
GTGCTAGATGCCTCGGACTTTGCCTTTCTTCTGGAAAACAGCACCTCTCCCTACGATTATGGGGAAAACGAGAGCGACTT
CTCTGACTCCCCGCCCTGCCCACAGGATTTCAGCCTGAACTTTGACAGAACCTTCCTGCCAGCCCTCTACAGCCTCCTCT
TCTTGCTGGGGCTGCTAGGCAATGGGGCGGTGGCTGCTGTGCTACTGAGTCAGCGCACTGCCCTGAGCAGCACGGACACC
TTCCTGCTCCACCTGGCTGTAGCCGATGTTCTGCTGGTGTTAACTCTTCCATTGTGGGCAGTGGATGCTGCTGTCCAGTG
GGTTTTCGGCCCTGGCCTCTGCAAAGTGGCAGGCGCCTTGTTCAACATCAACTTCTATGCAGGGGCCTTCCTGCTGGCTT
GTATAAGCTTCGACAGATATCTGAGCATAGTGCACGCCACCCAGATCTACCGCAGGGACCCCCGGGTACGTGTAGCCCTC
ACCTGCATAGTTGTATGGGGTCTCTGTCTGCTCTTTGCCCTCCCAGATTTCATCTACCTATCAGCCAACTACGATCAGCG
CCTCAATGCCACCCATTGCCAGTACAACTTCCCACAGGTGGGTCGCACTGCTCTGCGTGTACTGCAGCTAGTGGCTGGTT
TCCTGCTGCCCCTTCTGGTCATGGCCTACTGCTATGCCCATATCCTAGCTGTTCTGCTGGTCTCCAGAGGCCAGAGGCGT
TTTCGAGCTATGAGGCTAGTGGTAGTGGTGGTGGCAGCCTTTGCTGTCTGCTGGACCCCCTATCACCTGGTGGTGCTAGT
GGATATCCTCATGGATGTGGGAGTTTTGGCCCGCAACTGTGGTCGAGAAAGCCACGTGGATGTGGCCAAGTCAGTCACCT
CGGGCATGGGGTACATGCACTGCTGCCTCAATCCGCTGCTCTATGCCTTTGTGGGAGTGAAGTTCAGAGAGCAAATGTGG
ATGTTGTTCACGCGCCTGGGCCGCTCTGACCAGAGAGGGCCCCAGCGGCAGCCGTCATCTTCACGGAGAGAATCATCCTG
GTCTGAGACAACTGAGGCCTCCTACCTGGGCTTGTAATTCTGGACTGGAACTGTAGCCTGCGCAGCCCAAGTCCTAACAC
ACTCCAAGTGCTTGTCCTCCTTGTAGTTGGGCTAGCTCGAACTTACCCGTAACTTTGCTGCCAGGATGCACTGACAGCTC
AGCATATATCCAGGTCTCCTGAGAATCAATCTCAGCAACAAGGACAACACCATTACTGTGCCTTAGCTGCCATGCCCTAT
CTTGCTGTTTTAGAACTAGCTGCCTGGAGCCCCACCGCCCTACTAAATTAGCAAGTAGAACTCAGCCATCCCTGTGTGAG
AAGAGGGAGAGGCAAATAGCACAGAGGGCCAGGCGTTGTCAGCACTGAATGTGCCCATCTCAGTATCTCAATATTTGCCC
AATTTTATTTCTAGAAACCTCACTTAAACTTTCAATAAACAAGGTAATGAGGACCAGGGCGTATTCACAGTCATTCTTTG
AACCAGGTCTGGCTGGACTCTGGCGTGTATTTCCTGCCACATTCTGTCTGGCTTAGAGTTTGTCTCTCTGCCCTCCTGGG
TTCTACTGTGCCCTCTACTGCACCCACTGTGGCCTAGTGGAACTGCTAGATACAGTCTCAGGTTTGGGTGGTGGCAGTGA
ACAGCTGGTTGTCTAGGTATGCTCTGGGGCCACTAGACATTAATAAGCCATAATTTCCTGATCCCAGCAGCTCAGGCCAA
CAGGGAAGGTGTGGTGAAAATGAACAAATACCAAGCAATCTATACTACAGAGCAAGAGGGTAGCACTCGCTAAGTCCTGG
GCTCAGCAAGATGGCAAGATCTATCTGAAATGGCTTTCTGGAGACTTAAAGGGCAAGTGGTATCTCGAAGGCTAAGAGAA
```

```
GGAAGAGTTCTTTATTTCTAAGCAGAAGAGAGAGCGGTAGCAGAGGCAGAGAGGCTGAAGACAGTAATGCTTTGGGGACA
CTATAAGTGGTCTGGGCTAGAGGTGGTGGTACATACCGGTATTTGGGAGGCAGAGGCAGGCTGATCTCTGTTCAAAGCCA
GCCTGTTCTACAGATTGAGTTCCAGGCCCACCAGGGCTACACAGTGAGAAACTCTCTCTCTCTCTCTCTCTTTCTCTCTC
TCTCTCTCTCTCTCTCTCTCTCTCTCTCTCTCTCTCTCTCTCTCTCTCAAAAAAAAATGGTCTCAGCTGGCTAGAGTTAAGG
GTTTGGGTAGGGGTAAGGGCTGATGATGAAGCTGTGGTCTGGGTAAAGGCAATGGGCTCCCAGGCCACTCAGAGAAAGTT
GTATTGGTTATTTGGGTAGTATTCAGGGAAAATAGGCTTGAAAAAAAAAAGGTGGGGCTGGGGAGCTGGTTCAGTTGCTG
AGCAAACATGAAGACCCAAATCCAAGTTCAGACCCCCAGCACCTATGTAAAAGCTGGGTGTGTCCATGCCTGCCTATAGG
GTTGAGTTGGGTTGGCTGGCTGCCAGCCTGAGGGTGGGGGTGAGTTCCAGGTTCCTGTCTCAAAGAAATAAGGTAGAAAG
TAATAGAGAACACTCAATATGCTCCTATGGCTTTCACATACATGCACACACACACACACACACACACACACACACACACA
CCCCACACCAGCAACTGGGTGGGCCTGATGATTTCTTTGCTAGCAGACATTAGCATGAAGCACCAAAGCAAGAAGTGGG
CAACAAGGAGCAGTTTCAATGTCTAAAGCTGAGTGGAAAAAATAACCAGTGCTTTCCAGAAGGGTAGGTGACAGGGCTAC
CCCCTGCACTGTCACTTTCTCCCAATGAAGTCACCAACATGCCATGACTCACCCTGACAGGCTGGGTCTTATCTGAGGCT
GCAGATCTGCTGGAGCTTGGGAAAGAAGGAACAGAAGCAGTTAGCTGGGTAAGTTTGCCCTGCCCTATAGATACATACCT
GCAACCTTATCTAGTCTCAGTATTCCAAAGACTAGACTACCCTTCTACAACATGAGAAGCAAGAAGAGAGGAAAATGTCA
AAAGAGAATATCCTGGTAATAAAAAGTATCAGTAATAAAGATAAAGATAAGCATTATTTGTAGCAGGCCAAAAACTGGGT
CCATCAAGACACCTATACTCTGACTCCTAGAGCTTGTGATCAGGTAAAGGGCACTCGCCAAGCACAGTGGTACATCCCTA
TAATCTGAGTGCTTGGGAGGATACGGCAGGAAGATCAACGATTTGAGGCTAACCTAGGTGACACCATGGATTTTAGACTA
GCCTGGGGTTAAATAAGGAAAGTCCTGTCTTAAGCAAGCAGAAGGGTGAGGTGGTACACAAACTTGAATTGCAGCTTTTG
GGAGGCAGAAGCTAGAAGATTTCTGTAAGTCTGAAGCCAATGTCATCTATCTAGCAGTTTCCAGACCAGCCATGGATACC
CAGTGAAACCCTGTCTCAAAAACAAACACACAAAGAACAACAACAAACAAGCCAACAAAGATGGCAAAGAGGAACTGA
GGTTGCTAATCTGTGAAGTAACTGGTTTAAAAATAAGATTGTGGGGGTTGGAGAGGTGGCTCAGTGGTTAAGAACACTGA
CTACTCTTCCGAAGGTCCTGAGTTCAATTCCCAGCATCCACATGATGCCTCACAACCATCTATAATGAGATCTGACGCCC
TCTTCTGGGGTGTCTGAAGACAGCTACAGTGTACTCACATATAATAATAAATAAATCTTTTAAAAAATAAGATTGTGGCA
GGTCATAGTGGTGCATGGCTATAGCCCCCAGTATTTGGAAGGCAGAGACAGAAGGACCAGAAGTTTAAGGCCTGCCTTGC
CTACCTAAAGAGTTCAAGGTCAGGACCAAGCCAGTGACCTCAGCAGATAAGAGTATTTGTCACCAAGCCTGACAACCTGG
GGCCCACAGAGTAGAAGAGGCCTGACTCCCAAAAGTTGCCCTCTGATCTCTACATATGTGCTATGACATAATTATACCCC
CTCCCTCAAAATAAATGTAATAATTTTTTAAAATTAAGGTCAACTTGGGATATATGAGACCTATCAAAATGGTAATAAAT
AATAAAAATTAAAATGATACTATTGGGCTGAAGAGAGGCTCAATTTGTAAAGTACTTAACCATGTAAGCACAGTGACCTG
AATTTAGTCCTAGCAAAAAAACAAAAACAAAAACAAAAACAAAACAACAACAACAACAAAAAGAAAAAAAAAAAAACAAG
CATGGTAGTGTGTACTTGTAACCCCAGTCCTGGGGAGGTGGTAACAGGAAGAGCACAGTAATGTGCTGGTTAGCCAACCT
AGCCTAGTCGGAACGACCTACAGCCCACATGAGAAACTCTGTCTCAAAAAACAAAAAACAAAAAAACAAAACAAAACAAAA
CAAAACAAAAAAAACCAACAACCAAGGTGGACAGCTCCTGGGAATTAAACCCAAGGTTAGCCACTGACTTCCACGTGTA
TATGCCAAATACACACACACACACACACACACACACACACACACACACACACAGGTGTGCTGTGGTGGCAGCAGCAGAAG
AGAGGGGGAGCAGGAGAAGGAGAAGGAAGCATCTGCCACCGCCACCACTGCCACCAAGTGAGCCTAGTGTCACAGGAATC
TATAAAAGGAAAGGAAAGAGACAAAAGATGTTTTGTCTTTGAAGAAGCCATGCAGGCAGGGGCTGTGCGAGGAACTAGGA
GCTGGGAAAGACAGAGAACTTCATTCTCCAGAGAGGAAGGTCAAGATTGAGATTGTTATCTCGACGACTCATCCTAGGCT
CAGGACTGCCAGAACTCTAAGATAAGACATTCATGCAGATCTAAGCCATTCAATTGGTGGCCATTTTCTCTGGTTTCCAC
AGAAATATACAAATACAATCTTGGATCCAATCCACAACCCTCAATAGCTCCCAAAGATAGGGAGTCTGCTGGAACTCTTG
AATAAGGAGAACAGTAAACTAAGATGAAAATACTTATTTTCTAAGTAATTACATGACTTAGCTTTGCTCAAAGACATGAC
AAGCTGGAATTCATGAAGCACGTTGGCTCTTCAAATGTGGCTAGTGTAATAGGCTGACAGAAGCCTAGCTGTGGGGTAGT
GCCTTTACAACTTACTCTTTTATGACAATATTTTCTCAACACTCCCCATCCAAGAAAATATCATTGTCTTCTGTTTCATT
TTGCATTTTCAACTACATAATCAAAGTTAAGACCCAAGGTGGTTCCAAATGGCTTCAAGTGTTGTCAAGGGATATATGGT
TAGCAAAAGATCTCTTTGTTAAAAACAGTAACCATTAATAACAAATCAGAAAACACATTTAAACTCCAATATTTAAACCT
TTACAGAAATAGGGATTTCTCTTTTTTTTTTTTTTAAGATTTTTGTTTATTTATTATTGTACACTGTAGCTGTCTTCAGAC
ACACCAGAAGAGGGCATCAGATTCCATTATGGGTGGTTGTGAGCCACCATGTGGTTGCTGGGATTTGAACTCAGGACATC
TGGAAGAGAAGTCAGTGCTCTTACCCGCTGAGCCATCTCACCTGCCCCATGAAATAGGGATTTCTTAAACTTCTTGCCAG
TAAAGGAGTCAGCTCATATAAATATACTCCGGGACAACCTTAAGGATTTTCCTTAAAGATTTACTGGCAAGATAATCCTT
GCTGAGTCTTTTCTCCACATCCCCATGGCTGATTCCTGTTGTCCCATTAAACACTGCTCTATAATTCTCCTGAACATATT
TAGCAAATGGCCTTAAATGGGGCACAATGGGGGTTCCATTCTTCCAAAATAATGACAGCAAGACCAGAGGCCCTTTGCAT
CTGGCACAGATGAAGCACTCAGTGTTCAAATATCTGGTATAGTGGCCAACCCTGCTGAGGAAGGAGATTCAATACCTAAA
TAAATAAATAATGATACCTCAACATCAGCTCCAACTCCTTAACCTTGTGCCCAAAACTGTTTTCTGTGGGTTCTGAGGAG
CCCCAGGCATCAGCAGGGTCAGGCCTGCTAAATTTAGGCCTGAGGTGGTTAGGTGGGGGTAATAGAGAAAGAGGACACAG
AAATAGTGAGATTCACTCAAGTCAGACAGTCCACAGTCTTAACTAAAGATCTACTTTGTCTTGCCACGTGGGAGGCTGAG
GCAAGTTCAAAGCCTGTCTGAGCTACAAAGCTAGCTCAAGACTAACATGGAAAACTTGCTAAGACCCTCTTAAAAACAAAA
CAAACAAAACAAAAACCACAGGAAAAAGGCTGGTGACATTGCTCAAGCAGAGATTCAGTGTAGTGTTTGCCTGGTACACA
TGAAACCCTCAGTTCAATTCCCAGCACCAGAAAAACAAACCAAAACAAACCAAGAGCTACTGGAGCCTGAAATGACAAAG
TAATTGGAAAAAAGTATGAGAAAGACCTTTCCTGTATTTGCACAGAATACATTTGTTGCAGGAAATATTTTTTTTTTAAAT
GCCAGCAGGCTTCCTGCACTTCTGCTGACAACTCTCTGCCCCAGTGCCGTCCCTCCAACTCTGTCTCTAGCTAGGCCCTG
CAGTGACCAATTGCTCTCTGCTCAGCTCCGGTTCACTTGTCTCTGGGGCCACTAGTTGCTTCTCAGCCATAAACCCCTGC
AGTTCAACAGTCCCCACCTTCCAGTCACCCAGTAAAACAAAACTCTAGAAGCTTATAATTAGGAAGTCAGATTTATATATC
AATAAATTCTCAATTCACAAGATGCCCACACAATAATTTTAGAGCCAATTGATAATGATACAAGCTGCCCACCTAGATCA
```

```
AACAAGTTATCCCAATTATTCTATCCCTATATGATATTCATAACTGTCTGTGGCTAATTTAAAACCACGCGGGATCGGGA
TCTTCTTCCTGTCCATCTGCCTCCATCTTTGCTTCCCTTCTCCCTCCTCTAAGTCGCTCTCTGTCCCTGCAACTCTTCGC
TCCACCTCCCTTTTCCCTGTCCAATCACAGGCCTCCTGCTGCACTAATATTTTAATGTAATTGGACAGGGAAAATCCTGT
CACATACATTCATAATAGATCTTGTAGTTAATTGTATAGTTATGGCAATGGATGACCTTAGGCAGCTCTGGGTGTACTGA
ATTGCATTTTTGAGCATAATATTGCCATGAGTCACCATGAGAATCCTGGATGCCATCAAGCAGCCAGGACGATGCATGGC
ATAGATCATGGATGGTGTCCTGGATTCCATCTTGAAAGAATGAAAGGAAACCGTGAGCACTGAAGTAGATTTTCTTCTGA
TCTGCTGCCCAGAAACCCCTTGATTTTCACTTTCACCAAACACAAGGCCATCTCACAGAAGTGTATACAGGCAGGCAGCC
ATAGGGGTTGCCTTCCCAAGATTCTGAGAGAGAAAGTTCCACCCATGACAAGCTGGTTTGGGTTTCAGCTGAACCTTAAG
AAGTCAGGTTGGGAGCAGCTCTAACATGTTCATCTTCTTGTGGGGCATTGCTACAAGTGCACCTGGACAAGGATTGGTTT
CTTCCTTCTATAAACAAAGTGGGCTCAGGGACTATCTGGACCATGAGATTTGGTCAGCAGAAGCTTGGAGTGAGATAGA
GATCTGAACTGGGCTCTATTATACATTTATTGATTTATTATGTGTGTATGTGCATACATGTGGAGGCCAGAAGACAACTT
CATGGGAGTCGGTTCTTTCCTTCTAATATTCAGGCCCCAGGGATCAAACTCAGATCAGCAGGCTTAGTAGCACTGAGCCA
TCTCATCCAGTCTGAGCTGGACGCTTAACAACTCACTGACTTTAGAATTAGAAAATCCATCCAAGCTGATACTCAACCTA
TGATCTCAGCCCTTGACGGACTAAAGCTAGAGGTCAGAGTTACATAGTGAGCCCATCTCAAAACAACAAGAACACTATTT
TGTCTGCTTTAGTATTTTTTTTGTCTTACTGATGTTTTAAAAGGTTTTTATCTGTTTTGATATTTTACTTTCCTGTTTTT
TGTTTGTTTGTTTTTAAGAGACAGTGTGAGCACAAGCACACAGAAAGTTGGGTGGATAGGGCAGATCTGGGAAAGGGGAA
AGAATATAATCAAAATATATTGTATGAAAAAAGATTTTTAGATTAAAATAAACAACAACAAAAATCCAGCAGCTTACTTA
TGTAATCCTAGCACTGGAAGGCTGAGGAAGGAGGACTACACAAATTCTAGGTCAGCCTGAGCTATATAGTGAGTGTGAGA
CCAGCCCTGGCTACACAATGAGACCCTGTGTCAACCTGTACTCTCAACTCTCTCCACCAACTAAGGTCATTATAAGCTGG
GTTGCTGGTAGACCCTTGTATGGAGTTCCAGCTACTCAGGAAGCTGAGATGAGAGCTCTGCTTGAGCCAGGGGTTCAAGA
CCAGCCTGGGCAATATAGCAAGCCACCATGTCAGAAGTAAAACAAAAATCCTTTGATTTCTGAGAATTAATGAAGGTTGT
AGCTGCTATTTAGTGGCATACCTCTTACAGAACATGTGTGAGGCTCTGGTTCCCAATATGGTAGGGGTAGATGGAAGAAA
CCTGCTAAATTACACTTACCTGGCAGACTATGTCACTGCTTTGAGCTGTCATTTCTAGGTGGACAGGCCTTCTGTGTGTT
GCATAGAGGATATCATTATTGGATGCTTCACCAATCCCTAGGAACTCAACATAGGAGATGATTTTCCAGGCCCACCTGCT
CATTTCCCTTGCCAAAACCAGCATCTCCATTCTCTCAGTATCCAAGGGTCAAGATGCCCTCTCTAGCTAGTAGTCAACCC
CTCCTCCGGCTTTGCATTTAGCCAGACTTCAGCTCTCAGGTTTCTTATCAGAAAGACAGGGTGCTAAGGTTAAGTCGCCA
TTTTCCCGGAATGACCAGCAGAGTCACAGACTTTCAGAGAAATGCCAGTTTTGGCATAGCACTCCCTCTTTGGTTGTTGT
ATCTCATTGGTAGTGCAGCAGTCTGCAACATCTTTTTATTCCAAGTGATTTCCATTTTCTCTGGCAACTCAAAATGAAAA
GCAAGCACAAGTATCAAAAAGTAGTTCTAAAGCTTTACTTATTTTTCTATTTAAATGTATGGGTGTAAGCCTGAGTTTAT
GTGTACCGTGTGTGTAACAGAAGAGGCCAGAAGATAGTGTTGGGTTCCCTGGAGCTTGGAACTATAAAGCAGTTGTGAGT
TGCCATATGCATGCTGGGAACCACACCTGGGTCCACTGCAAAAGCAAAAAAAAAAAAAAAAAGTTATGAGCTCATAACTG
CTGAGGCATCTCCCTAGAAAACAACTCTTAGCAGATCGGAAAATAAGAGTGATCATTTTTCTCCATTTAATGAAAAGTAA
GAGGACATGCCAACATGGATGAAAGAAATTTCAAAAGACTCTACCCGTAGATGAAAGCTACAGGCAATAGCTGCCCTGG
CAAAACAGGTTTTTTTCCTCCAGGGATGAACCCCTGATAGGTTATACAATTCCCAGTGATCTGCCCTAAACACATGGACA
TAGGAGCAACACTAAATGGACTCAGTAGATTTTACACACATACACATAAACACAAAAACAATTAAAGAGGTTATGAGTT
TGAGAGAAGGGGTTGAAGCAGGGAGTGGTGGAAATGATATAAATATAGTACTCACATATGAAATTCTCAAAACATAAGAG
AACACTTTTTTAAAAAAAGGCAAGAGGAACTATCTTCCCTAGGCGTTTTACTCTGAGTACATGAATATACTGAAGAGAAA
TATATGTGAATAAAATGTAAATCTAAACACTCTGGGAAGAATGGGCGCTTAGTTGAAGTCCTGGGAAGGACTTAATTACG
TTAACTAATCAGGACCCAAGGGAGGAAATTGTTGAGGCCACACAAATGTAGGATTTGGTGCTTAGATGCATGGATCCAAG
TCTGGGGCCGTCAGGAGAGAAGTGCAAGGCTGCTGAGGTTGGCAAACATCTGCACAACTCGGAATGCTTTGGGGTTCCAC
TCACCTGATATAGCATCAGTGTCATGAGGAAGGGTACCAAAGACTGAAGAACCTTCAGGATATAAGAACTGCCCTCTGCA
AAATCTCATCAGGAAACTACAAAATTGGATCAAAAAGCAACAGCAAATGACCTCTGCCTGATAGTAGAGCGCAGGGACAG
AGTGCTTTCCTCGCATGCAGGAAGCATGGCAAGACAAACAAACAAACAAACAAACAAGAAAACCAAAGTGAACAATCAAG
GGGACTGGCCAGCAGGCCTCTGCCTCTGGATTTCTAATCCAGTACATACCACCAAGCTACTAGCAGGTGGACTTTATTCA
GCAGTAGCTCCCTGTCACATTTTCCTGTGCACTTGTGCCTACTGATTTCAACTTAACTTCCCCTAACCTAATCCCCAAGG
CCATCTATAGTCAGAAAAACAAGGGCTTTCTCTGTTTAGGACCTGCTCTAAGCATTTTATGAACATGAAATCCTGCAGCC
CTAACAGTAATTCTACGGTAGTAAGTAACACTGGCCTTGTATCCACGAGTTCTGCATTATGGAGTCAACAAACTGCAGAT
CAAAAGTTACCGAATTTATTATATAAACATATGAACTTTCAAATAGTAAATAAAATAAAAGAACACAAAACTGCATCTG
CTCTGAATATATACAGATTTTCCCTTGCCACTCTTTCCTGCATAGTGTTTCCACTCTGTCGGGCATTGGAAGTAGCATAC
AGGTGGGATGAAGTGTAGCGGAGGATGAGTAGAGGACATTTTATATAAGGTATTTGAGCATTTTAGGTTTTGTCATTTGT
TGAGGGGTACCAGAACCAGTTCCATGCAGGCACAGAAGGATGACTGGACTTTTATTGGCCCCATTTTACAGAAAACACGG
GTAAAATGAGGTTAAATAAATTGTCCAGCTCCTTCCTATCATCTTCTTTTCCTCAATACATGTGACTCCCTACTGCTGTT
CTCAGTACTAACTTTCCTTAAGAAACAGGAGCCTCATGTCTTCCAGAAAATCCTTTCTTCCTTCTCTGATTTTTCAGCCA
TTCTCACTCTGCTAATCCCCTACTATAATAACCACCGTTCCATATAGCACAAAACAAAACTTCTCCTTCATGTAAGCA
TATCTCTCATGTCTGTAAAGTCTAAATGCATCCCCCCCCCCCAGATATGGCAGTGTTGGTGACAATGTGCTAAACAGAA
GGGCTCTAAATGGGTAAACAGAATTGTTATTGGGCAAAGGCATCTAGTTCCTGATTGATCCAAACAGCCTGTATCAGCAT
AAAGATCTCCACCCTGCTGACTATCATATAAAATCTGCCTCCTTTTCTACAAATTTGCTTCTCTCTGCCCCTCTGAAAGA
TAGCCTGGCAGTTTGATCCCCAATAAACCACTACTGATGGAGTGGTCCAATTCTGCTTAAATTAACACATGTCCCGGTAC
ACATGCGCAGCAGCAGAGAGTGCTGGCATACAAGGGGTATTCAATAAATGCT
```

MOUSE mRNA SEQUENCE : mR5-034.1 (Seq ID No: 50)

```
GCAAGTTCCCAACCACAAGTGCCAAAGGCAGAGAAGCAGGCAGCACGAGACCTGACCCCAGCAGCCACAGCCGGAGCACC
AGCCAAGCCATGTACCTTGAGGTTAGTGAACGTCAAGTGCTAGATGCCTCGGACTTTGCCTTTCTTCTGGAAAACAGCAC
CTCTCCCTACGATTATGGGGAAAACGAGAGCGACTTCTCTGACTCCCCGCCCTGCCCACAGGATTTCAGCCTGAACTTTG
ACAGAACCTTCCTGCCAGCCCTCTACAGCCTCCTCTTCTTGCTGGGGCTGCTAGGCAATGGGGCGGTGGCTGCTGTGCTA
CTGAGTCAGCGCACTGCCCTGAGCAGCACGGACACCTTCCTGCTCCACCTGGCTGTAGCCGATGTTCTGCTGGTGTTAAC
TCTTCCATTGTGGGCAGTGGATGCTGCTGTCCAGTGGGTTTTCGGCCCTGGCCTCTGCAAAGTGGCAGGCGCCTTGTTCA
ACATCAACTTCTATGCAGGGGCCTTCCTGCTGGCTTGTATAAGCTTCGACAGATATCTGAGCATAGTGCACGCCACCCAG
ATCTACCGCAGGGACCCCCGGGTACGTGTAGCCCTCACCTGCATAGTTGTATGGGGTCTCTGTCTGCTCTTTGCCCTCCC
AGATTTCATCTACCTATCAGCCAACTACGATCAGCGCCTCAATGCCACCCATTGCCAGTACAACTTCCCACAGGTGGGTC
GCACTGCTCTGCGTGTACTGCAGCTAGTGGCTGGTTTCCTGCTGCCCCTTCTGGTCATGGCCTACTGCTATGCCCATATC
CTAGCTGTTCTGCTGGTCTCCAGAGGCCAGAGGCGTTTTCGAGCTATGAGGCTAGTGGTAGTGGTGGTGGCAGCCTTTGC
TGTCTGCTGGACCCCCTATCACCTGGTGGTGCTAGTGGATATCCTCATGGATGTGGGAGTTTTGGCCCGCAACTGTGGTC
GAGAAAGCCACGTGGATGTGGCCAAGTCAGTCACCTCGGGCATGGGGTACATGCACTGCTGCCTCAATCCGCTGCTCTAT
GCCTTTGTGGGAGTGAAGTTCAGAGAGCAAATGTGGATGTTGTTCACGCGCCTGGGCCGCTCTGACCAGAGAGGGCCCCA
GCGGCAGCCGTCATCTTCACGGAGAGAATCATCCTGGTCTGAGACAACTGAGGCCTCCTACCTGGGCTTGTAATTCTGGA
CTGGAACTGTAGCCTGCGCAGCCCAAGTCCTAACACACTCCAAGTGCTTGTCCTCCTTGTAGTTGGGCTAGCTCGAACTT
ACCCGTAACTTTGCTGCCAGGATGCACTGACAGCTCAGCATATATCCAGGTCTCCTGAGAATCAATCTCAGCAACAAGGA
CAACACCATTACTGTGCCTTAGCTGCCATGCCCTATCTTGCTGTTTTAGAACTAGCTGCCTGGAGCCCCACCGCCCTACT
AAATTAGCAAGTAGAACTCAGCCATCCCTGTGTGAGAAGAGGGAGAGGCAAATAGCACAGAGGGCCAGGCGTTGTCAGCA
CTGAATGTGCCCATCTCAGTATCTCAATATTTGCCCAATTTTATTTCTAGAAACCTCACTTAAACTTTCAATAAACAAGG
TAATGAGG
```

MOUSE PROTEIN SEQUENCE : mP5-034.1 (Seq ID No: 51)
KFPTTSAKGREAGSTRPDPSSHSRSTSQAMYLEVSERQVLDASDFAFLLENSTSPYDYGENESDFSDSPPCPQDFSLNFD
RTFLPALYSLLFLLGLLGNGAVAAVLLSQRTALSSTDTFLLHLAVADVLLVLTLPLWAVDAAVQWVFGPGLCKVAGALFN
INFYAGAFLLACISFDRYLSIVHATQIYRRDPRVRVALTCIVVWGLCLLFALPDFIYLSANYDQRLNATHCQYNFPQVGR
TALRVLQLVAGFLLPLLVMAYCYAHILAVLLVSRGQRRFRAMRLVVVVVAAFAVCWTPYHLVVLVDILMDVGVLARNCGR
ESHVDVAKSVTSGMGYMHCCLNPLLYAFVGVKFREQMWMLFTRLGRSDQRGPQRQPSSSRRESSWSETTEASYLGL*

MOUSE PANTHER CLASSIFICATIONS
        FAMILY (SUBFAMILY)
                C-C CHEMOKINE RECEPTOR-RELATED(C-X-C CHEMOKINE RECEPTOR TYPE 3)
        BIOLOGICAL PROCESS
                Signal transduction(2.11.00.00.00) > Cell surface receptor mediated
signal transduction(2.11.01.00.00) > Cytokine and chemokine mediated signaling
pathway(2.11.01.02.00) > G-protein mediated signaling(2.11.01.07.00)
                Immunity and defense(2.16.00.00.00) > Cytokine/chemokine mediated
immunity(2.16.10.00.00)
                Cell        structure        and        motility(2.27.00.00.00)        >        Cell
motility(2.27.02.00.00)
        MOLECULAR FUNCTIONS
                Receptor(1.01.00.00.00) > G-protein coupled receptor(1.01.01.00.00)

MOUSE GENE ONTOLOGY
        BIOLOGICAL PROCESS
                cell motility > chemotaxis
                cell surface receptor linked signal transduction > G protein linked
receptor protein signaling pathway
                defence response > inflammatory response
                cell growth and maintenance > invasive growth
                defence response > cellular defense response
        MOLECULAR FUNCTION
                enzyme inhibitor > protein kinase inhibitor
                defense/immunity protein > antiviral response protein
                enzyme > 2-acetyl-1-alkylglycerophosphocholine esterase
                DNA repair enzyme > deoxyribodipyrimidine photolyase
                DNA photolyase > deoxyribodipyrimidine photolyase
                1-phosphatidylinositol 3-kinase > 1-phosphatidylinositol 3-kinase
regulator
        CELL COMPONENT
                cell > membrane fraction

```
cell > cytoplasm
plasma membrane > integral plasma membrane protein
cytoplasm > endosome
cell > plasma membrane
MOUSE PROTEIN DOMAINS (INTERPRO SIGNATURES)
        IPR001277 (CXCCHMKINER4)
        IPR000276 (GPCRRHODOPSN)
        IPR000355 (CCCHEMOKINER)
        IPR000174 (INTRLEUKIN8R)
        IPR000248 (ANGIOTENSINR)
        IPR000276 (7tm 1)
        IPR000276 (G PROTEIN RECEP F1 2)
        IPR000276 (G PROTEIN RECEP F1 1)


HUMAN GENOMIC SEQUENCE : hD5-034 (Seq ID No: 52)
TAGTACAGATGGGTTTTCTCCATGTTGGTCAGGCTAGTCTCGAACTCCCGAACTCTCGTGATCCGCCCGCCTTGCCCGCC
CAAAGTGCTGGGATTACAGGCGTGAGACACCACACTCAGCCTTAACTTTTTAAAAATATCGTTTGCTTTTTTTTTTTAGAA
TTAGACAGAGCCTTACCGCACTGTTAGTTTCCTTGGTAACCACCTCTAGTTCCCAACCCCTAGACCTCCTGTCCTCAGTC
CCTAACTGCAGACCGTCTGAGGCACTCCCACACTCCCTGGCTCCAGGAAGATGCCTTGCCGTGGTTCTCACCCCAGCCCC
TGCGCTATCTCAGTGCTCCACTCCAGAGCCCATCGCCAGGTCCCAGACTGGGTGTCCTCAGCCCCTAAAGTCACATCATG
GTCTTTTCAAATCCAGTTGGGGGCAGGAAGGGTATTCACAAGATAAAACGTATTCCCAGAGACAGAAGGGCGGGCTGGTT
AGGAGTGTGGACTTTGAAGCCAGAAGGCTTGGTTTGAAAGCCAGGCTTTGTCACTTACTAGCCGTTTGACCTTGGGCAAG
TGACTTAGCCTCTTTGAGTCTGTTTTATCATCCTTAAAAGAGAAATAAAAATAGAACCTTTCTGAAAGAGTTTTCACGAG
AATAGATATGTGTAAAAGTGCTTTAAGCAGCACCTGGCACTTGGGGAGCGTTCCTGGTCAATATTATTGTTGTGGCCATC
CATCTGTCCGCTCATCTTCAGATGTTTTGGCTTAGTCAGCAGTTAGTCTGGGATTTTGTGAGTGAGGAATTCATGACTGC
CACAAGCAAGAGCATGTTTTTAATTTGGTGTCTCTTCAAGTTTTTTCTTTCTTTTTTTTTTTTTTGAGACAGTCTGGCTC
TGTCGTCCAGGCTGGAGTGCAGTGGTGCGATCTCAGTACACTGCAACCTCCACCTCCCGCTTCAAGCGATTTTCCTGCCT
CAGCCTCCCCAGTAGCTGGGATTACAGGCGCCCATCATCACGCCTGGCTAATTTTTTGTATTTTTAGTAGAGACAGGTTT
TCACCATGTTGGTCAAGCTCGTCTCAAACCCCTGATCTCAAATGATCCACCCACCTCGGCCTCCCAAAGTACTGGGAGCA
CCCAGGCATGAGCCACTACACCCAGCCCTAGATTGACTCTTAATCCAGAAGACAGAGAGGTTAAATAACCTGTCCGTGGT
CACAAAGCTAGTAAATGGCAAAACCAGGACCAGGAACCCAATCCAGACAGCCTGCTCCAGGGTCCGTGTGCTTACCCACT
CTGCTGCCCTGCCTTGCATGGGACTAGCCAGGCTCTGGCCAGACTGCCACTGATGCCTATGCCTGTACAGATGGCTAGCT
GCCTAGTTGGAGAAATACGGGCTCCACTCTAAGGAGGAGGCAGGGCCTGGGCCATGGCACCTCGGCACGGGCAATGTGGA
CAAAGTCCCCAAGGCTGACACCACACATTGGGATTGTAGAAAGCGATTTTTGGCTGGTTGAGGAATTTTCCAGAAGGCCT
GAGAGTCCAAGCAGGTAAAGTCCAGCCCAGAGTCAGGCCCACCATTAAGAGGCCTCGTGCGGGTCCTGGGAGGGCAGCTT
CAAGCAGTATTGGGTGTCTCAGAAGGTATGGACCAAGGGAGGAACGTTACTTGGCAGCTTTGCTCAGTGGAGTGTCTTTG
GGTTGGGCACAACGGTTTCCAGGGTGTGCCTTTCCCTGTTCCTGGAAGCCCAGCAGCAGCCAAGAGCCCCAGGCCACGCC
CAGTCCAGACTACCTGCTCCTCAGGAAGGGGCCTTAGCCAGCTGTGCAGGCCCTCTTCAGTTTGGGAAAAAGAACTTCAG
GCTCTCAGCTGGTGACACCTTCTGAATAACACAACCCCAAATCCCTTCTATTCAGCCAGTCTGAGCCAAGGATGGGGTCA
ACCCGGGAGCCTCTGGTGTGGCCTGTATTCCTTGAGGGGCCCTGAGTGGCTTGGAGAAGAAGCCCTCGGGCTGCTGATGC
CTTGCAGCTGGAGGGCAAGCAGGCTTCCCTCCCTGGTTCAAAAGGCTGCAGGGGAAGTCAGGAGGAACTTGGCTGAGAGG
TTGGAGGTAGCCAGACAGTGAGAAAAGGGAAGAAGCCCAGGATTCTAGAAGCACAAAGTGAGGCCACGAGCAGGAAAGGT
GGGGACCAACTGAGGGAGGGAGAGGTGCTTTCCTGGGACCGGGATGAGGCCAGAGGCCACTCCCAGAAAAGATGCGTTCC
CAGGCAGGCTTGACCCCACCTTCTTCTCACACGGCCTCTAAGCTGATTCAGGGAATAGCATCCGATATCAAACCCATCCT
GCCTGAGATTCTTCTGATACTTTTTTTTTTTTTGAGGCAGAGTCTTACTCTGTCACCCAGGCTGGAATGTAATGGCGTAGT
CTCAGCTCACTGCAAGCTCCGCCTCCCGGGTTCAAACGATTTTCCTGCCTCGGCCTCCCGAGTAGCTGGGACTACAGGCA
CGTGCCACCATGCCCGGCTAATTTTTGTATTTTTAGTAGAGACGGGCTTTCACTATGTTGGCCAAGCTGGTCTCAAACTC
CTGACCTCGTGATCCGCCCACCTCGGCCTCCCAAAATGCTGGATTACAGGCGTGAGCCACCGTGCCCGACCTAACTTTTA
TTTTGATATAACATAAAACTTATTAGGTTGGTGCAAAAGTAATTGCGGTTTTTGCAATTAAATGCGGTTTCTGCAATACT
TTCAATTGCAAAAACTGCAATTACTTTTGCACCAACCTAATACAAACAAGTTGCAAGAATAGTACAAAGTTCTCCTTTAT
GCACTTCCTCCATATCACCAGTTATTAGCATGCCGCCCCATTTGCTTTATCCTTTGTGCATTCTCTCTTGCTCTGCATAT
TTGGTTTCCAAGTCACACGAAAGTAAATTGCAGACCTCATGACCCTTAACTTCTAAATATCTCGATGTGTATTTCCTACA
AACAAGGACATTCTCTTACATTATCCAAATTAGCTAAATAGCAGGAATACAAGGTTTTTGTCTAATCTCCAGGCCATATT
CAAATTTTACCAATTGTCCCAATCTTTTAGCTTAATTTTATTCTTTCATTATTATTATTATTTTGAGACAGGGTCTT
GCTCTGTCACCCAGGCTGGAGTGCAGTTGTGCGATCTTGGCTCACTGCAGCCCCAAATTCCCAGGCTCAGGTGATCCTCC
CACCTCAGTCTCCCGAGTAGCTGGAACCAAAGGTGCGCACCACCACGCCCAGCTAATCTTTGGTTGTTTTTTTTTTTTTG
TAGAGACGGGGTTTCACTGTGTTGCCCAGGCTGGTCTCAAACTCCTGGGCTGAAGCTATCTGCCCACCTTGGCATCCCAA
AGTGTTGAGATTACAGGCGTGAGCCACAATGCCTGGCTTATTCTCCTTTTTTTTTTTTTTTTTTTTTTTTTTTTTTTTTG
AGACAGAGTCTTGCTCTGTCACCCAGACTGGAGTGCAATGGTGCAATCTCGGCTCACTGCAACCTCCACCTCCCAGGTCA
AAGTGATTCTCTTGCTTCAGCCTCCTGAGTAGCTGGGATTACAGGCACCTGCCACCACGCCTGGCTAATTTTTGTATTTT
TAGTAGAGATGGGGTTTCACCATATTGACCGGGCTGGTCTTGAACTTCTGACCTTGTGATCCACCCACCTCAGCCTCCCA
```

```
AAGTGCTGGGATTACAGGTGTGAGCCACCGTGCCCGGCCTTATTCTCCTTTTTGATGACTGTTTAATCACACAGCTAATA
CATGAATGCAGTCTCTGAATAAAACAATACAGACAAGGCCAAAGCAGTCCTCTTTGAACAGCACTCTCGCTCCCAGCCCC
TCTCACCTTACACAGTGTCCCTGTAGAGTGTAATGGGGGAAGATGAGGAGCCTGTGCCACTAACTAGCTGTGTGACTTTG
AGTAAGTTCCTTGAACTTTCTGTGTTTCTGTCATCTGTAAATGAGGATGATGATGATAGTAATGGCCACATAGTTACTA
CATAGTTGTAGGTATTAAATGAATTCATCCCAATAAAACCCTGAGAACAGTGCCTAGCACGTAGGTAATCAATAAATGTC
CATCAGGCGAGGTGACTCACGCCTATAATCCCAGCACTTTGGGAGGCTGAGGCGGGCAGATGGCTTGAGTACAGGAGTTG
GAGACCAACCTGGGCAACATAGCGGGACCCCTGTCTCTACAAAGAGTTTAAAAACAATAATAATAATAAGTTGGGCGTGG
TGGCACATGCCGATAGTCCCAGCTACTCAGGAGGCTGAGGCAGGAGGATCGCTTGAGCCAAGGGAGTCGAAGCTCCAGTG
AACTATGATCGCACTCCCCACTGGGCAACAGAGTGCGACCTTGTCTCTAAAAGACAACAACAAAAAAAAAACAAGCAAAA
AAACAAAAAAGAACCAAAAAAAGCCCCAAAACAAATACATGTCCTCAGTTCTTATGACCCTGTTTGACCTCACCCTGTGG
GGAAACCCAAGGAAATGCAAGAAGCAAACTATCAACACTCCCCTCTATGGCTCCAGCCCAGCCAAGTGGGTGTCCACCCT
CACCCACACAAGATGCCTGATTAGGGCTGTGTGATGAGAAAGGTGTGGCTCCTGATGAGTCCTTGAGCAGCAGGATGTTTG
GAGGGCTGGCAGTGTGACACAGCCCTCCTGGCTGACTAGGCTTGGACAGATCACTTCACCTCTCTGAACCTCCATTCTGC
AAAATGGGCGCAGTAGCCTCTCCCTCACTGGGTGGCTGAACGGTTTAAAGGAGATCGCATGTATGAAAGTACAGCCTTGC
AGAATGGAAGTCCTTTTATTTAATAGTTCCCAGGGAACTGCCAGAGACAGGATGACAGGAAGGTCATTGTGCTTGGTGGT
GGGGCTTGGGTCCTGAACTGCTTTCCAGCCCCCTCTACCCTCCTGCCGTCACAAGAACGTTCCGTTCTAACCAGTCTGAC
CGCCCCTGGTGTGAGGAGCTCTTCCTTACTTCCCTGTCTTGGCACCTGGTGTTCCCCAGCCTGAGAACAGCCTCGTCTC
CCTTTGCCATGTGACAAACAGCAGCAACGTCCTCCAAGACCCAGCTCAAACTTCATCTCCCCTGTGAGGCCTTCTCCATT
CCCCAAAAAAGCAGTGTCCCCACAGGTCTGGGAACTCACCCTCATGGGCTGGGGCTGTCTGTCCCACCTCCTGGGCTCCC
AAGGCCCCAGCCCACGGCCCCCAGCACTTAAAATGGACTTGCCTCCCAGGAGGGCTCAGAGCTGCAGGACATGGGCAGAG
GAAGAGGCAGTCCCCAGGCCCCAGGTCTCCCTGTGACTTCAGCTCTCTTTCAAGGGAAATTCAGGGCCAGGAGAAGGAAG
CCCAGGGTCCCGGCACTGTCTGGAGCAGATACAGACCCCTGCCCAGGGCTTGGACTCTTCTTGATTTCACGTAAGATAGT
CTCAGCCCCAGCCTGGGCCACACGGGAACCTCAGGCCTGGCTGAGCCTGCTCCCCTAACAGCCTGGGGCTGGCGGCCAAG
TCCAGTAGCTTTTGGAGTAGTGCTCCAAGAGGCATTTGGAGGAGCTGCAAGAGAGGTTGTGAAGCAGCCGACTCTGACAG
AGAGGAGGAGGAAATGGCCCTTTTCCCGGGCTGGCCGGCCTGGCAGACAGCAGTAGGGGCCCTCCTTCCTGTGAGGCACT
GGGCCCAGGCCTCAACCTTTGAGCCTCCCATCTCCTCAAACCACAGCCCAGAGGTGTGAAAAAAAGAAAAAAACAGGGGG
AGGGGTGGCAGAGAAGAGCCAGGTGCACATTTCGCACCTTGACCACAGGTGGGGCCCGGAGCAGCTTCACTCTGACTAAC
TTCCTCCTCAGGCTTCCCCGCCAGCTGCTCTGGAGGGCGGAGGCAGGAGGCTCACTGGCCAGAACTCTGATTGCAGGCCC
TTGGCCAGGCAGCTGTCCCCTAAGCTGGGCCTGATTCTGTCCTGGTCTGGGAGCTGTTTGGGACCAGAAAGAACCAAATT
AGACAGCTGGTCAAGGGTTTGGCTCAGCCCCGTAACACAGCAACCTTCTAAGCACATCTCAGCTGTGCACGACGGCAGAAG
CCCACCACAGACTTGCAATTGCGAGGTTAGGAGGGGTCAGTTTGGAAAGTTCCTAGAGCGGGAGGAGACTGCTGCTCACT
AGGCACCTACCCTGCCCCCAGACATTTCTACACATATCATTTGCAGTCTCCAGAGCAACCCTATCAAATGTATACTATCC
TTAGACCCATTTTTCAGATGAGGAAACCAAGGTACAGAGATTAAATAATTTGCCTGAGATTACACAGCTGGGCAGCCAGA
GGATTCGCCTAGGACTGGCAAACTCACCGTCCCCCTCCACCCCCAGTTAAGGCAGAGGGTGGCCTCTCTGGAAAACCAGC
CCTCCGGCCAAGCCCACACCTGTACGCCCCACAAGTGCCACCCTCTATCACTGGCTCCCTTGATATGAAAGGCTCCTGGT
TGCTCCGGGTTTGTAGCCAACATCTCTCTCACTCAACATTCTCGATTGTACTCCTACTAAGCTATGGGCCCTGTGCTAGA
AGCCTACAGCCTTGGTTATTCCAGTTCATCTTTTTAACAACCCTGCTGAGGAAACTGGCTCGACTCAGCTATGTAATTTG
TCCAAAGTCACACAGCTCGAACAGGGTGGAGTCAGGGCTGGAACTCAGACAAGTTTAGCATTCTTCCCACAACATCATGG
CCTCACTGGGAACAGCTCAGACCCATAGCCCAGCTTGGGCTATCAGGACACGGCCCTGATATCAGGGTACGGCCCTGATT
ATCAGGGTGTCCGAGCTCAGAAAGGAAAGCTCTCTGCTCCTTTCCAAAGCTCTTGCCCTGCCTTTCTCTCATGTGTCCTT
CCCAGCAACCTGGGAAGGACATGATACAAACAAGTGAGGAGACTGGAAGGACACCAGGGAGGACACCAGGGTTACCTTTT
TTTCTTTTTTTTTCTTTCTTGTCTTTTTTTTTTTTTTTTTTTGAGATGGAGTCTCACTCTGTCACCCAGGCTGGAGTACA
GTAGCACGATCTCAGCTCACTGCAACCTCCACCTCCTGAGTTCAAGCAATTCTCCTGCCTCAGCCTCCTGAGTAGCTGGG
ACTACAGGCATGTGCCAGCCTGGCTAATTTTTTGTCTTGTTTTGTTTTGTTTTTTGAGACGGAGTTTCACTCTTGTTGCC
CAGGCTGGAGTGCAGTGGCATGATCTCGGCTTACCACAACCTCCGCCTCCTGGGTTCAAGCAATTCTCCTGTCTCAGCCT
CCCGAGTAGCTAGGATTATGGGCATGCACCACCACACCCGGCTAATTTTTGTATTTTTAGTAGAGACAGGGTTTCTCCAT
GTTGGTCAGGCTGGTCTCAAACTCCCGACCTCGGGTGATCCGCCCGCCTTGGCCTCCCAAAGTGCTGGGATTACAGGCAT
GAGCCACCGTGCCCGGCCTGGACTTTTATCTTTCACATGGACCAGTAACATTTCAACATCTATTTAGAGCAGGGCTCCCC
AGCCCATGGGTGGTGGACCAGTATACCGGTCTGTGGCCTGTTAGGAACCAGGCTGGACAGCAGGAGGTAAGCTGCAGGGG
GAGGGAGCATTACTGCCTGAGCTCTGCCTCCTGTCAGATCAGCAGAGCACAAATCCTGTTGTGAACTGCACGTGGGAGGG
ATCTAGGTTGTGCATTGTTTATGAGAATCTAATGCCTGATGATCTGAGGTGGAACAGTTTCATCCCGAAACCATCCCCAC
TCCAGTCCGTGGAAAAATTGTCTTCCACGAAACCGGTCCCTGGTGCCAAAAAGGCTGGGGACTGCTGATTTAGAGGACCC
ACATTGGGTTGTCAACTTCTGCCATCACAAGATGTTTCCAAAACTACCATCTATTTTCCCCATCATTTCATAAAGAAAAG
GCCCTTTTGATACCAAAAAAAGGGGACAAATAACATGATTGCTGAATATAAGACAGACCTTTAAATGGAACAAATGTGGC
TTTCCATGGTCAATTTTCTCATTTGCAGAAGCCCATGGAACTCAAACCTCTTCACAGGTCAGCACTGGGAGACTGTGGTC
CCAGCTCCCAACCCTTTTCCACCCCCATAGGTAGCTACTGGCCAAGTCCTGCTCATCCTAACTGGCCTGGTTTCTGGAAT
CCTTCCTGCTCCCTCCACTGGCACTGCTCCTGACCTCTGCCAGGCCCTCAGTTCCCTCTGGCCTGAACCCTGCAGGTGAC
AAGCTGTTTAGAGCAAGGAGAGTCACATCTGTCCACAGGAGGGGAAGGGCCTTGATGCCTTGCAAGTATGCCTCATGTGG
GTGTAAATTTAATTCCAGGCCCTAGCCTGGAGGCTTTGGTGGGGCAGTCTGGCTGAAAACTGGCCCCAAGACCATGGGCT
AGCACTTGCTTTTCTCTCTCTCTCTTCTCTCTTCTCTCTTCTTTCTCTCTCTCCCTCCACCCCTGCTTTCCAACTAGG
GGAGAGAGAGAAAGAGGAAACTGCTTGGGAGAAGCAAATTACGGGCAGCCTTGCCCACTGGTAATGAGAAGAGGGCGACA
```

```
GGGAGGGGAGAAGAGGGCAAAGGGGGAGAGCTGAGGGCAGGTGGGGAGAGGAATTGTTCACCATTCTCTGCCATAAAGTGA
CTGATGTGCTGTACCAAGGCTGCCTTACAGCTTTTCCTTAAGATCAAGAAGTTCCTATGGCCTCCTCCCCAACCCCATCC
TCCTTCTTGCCCGAGAAGCAACTGCTTTTATGAATTTGTGGTATCTCCTGTCCCTGTTTTAAACCTCGAGAACCAACAGT
GTTATCCAAAACAACATGGTATTGATTTTGTGGTGGTTTTTAGAGTTTATGTAAGTGGCATCAAACTATACATGGCATTC
TGTAACTTGCTTCTTCTACCATGTGATCGTTGGCTTTTCTAGATGCATCCATGTTCAAATGTGTAGCTTTAGTTCATTTG
TTTTTAACTGCATGAATATATCATAGCTTATTCACTTAAAAGAAACTGAAGACAGTGTTCATGAGTAGGAAAAGGAGAAT
CAGACAGCCGTGGGATCAATCCTCACGCCCACCTTTAATTGCCGTGTCTGGCGAAGGCCTGTCCCCTTTCTGAATCATCC
ATGCAGATAACCCCGCACGTCACAAGTGAGTTGTGTGGTTGAAATAAGGCAGTGTCGGAGAAAGCACCTAAAGCAATGTC
TATCACATAGCAAGTTCATTACTGGTAGTTCCCTTTCTTTTTCCCCACTGATTAGCAGGGAGAGGTGGTGGTAAAAAGCT
AGATTTAGTGTAACGGCTTTTTAAAAAAATATTTTAATTCCCTTTTTAAGAGACAGGGCCTCACTCTGTCACCAGGCTGG
AGTGCAGTGGTACAATTATATCTCAAGGCAGACTTGAACTCCCGGGCTCAAGCAATCCTCCTGTCTCAGGCTTCCAAGTA
GTTAGGACTACAGGCACATGCCACCATGCCCGGCTGGCTTTTTTTATTTTTGGTAGAGATGGAGCCTCGCTATGCTGCCC
AGGCTGGTCTCAAACTCTTGGCCTTAAGTGATCCTCTTGCCTCAGCCTCCCCAGTAGCTGGGATTACAGGAATGAGCCAC
TGCACCTAGCTGAAATTTTTAAGAAGGTGGATCTCCTCTGCAGACTTATTTCTCTACCCAGCCTTTACAATCATGCAAAG
ATCAACTCAAGGCCAGGACAGATGAGTTCCAATCTATGGAGCACTGACCCTACCAGTGCAGGCAGTAGGGTTGCCCTTCA
CTTCTCAGCTGCATTGCTTCCTCTTCAGACCTCTCTTCTCAAAGGACCTGCCCCCAGCCAGTCATCCTCTGCCCAGCTTT
TCTGGTCCCAAGCTTGTAGCCAGCTCCTCCAGAGAGGTTTCACCCAGTCACGGAAACTCTGTGGCCTGAGGTTTAGGGAG
GTCTGGTAGAGGTAACTCCCTGGAAGAGGCTGCTGCTGAGAACTGCCTGAAACTCCCACTTCCTCTGTGACTGCAGGTTT
CCAACCACAAGCACCAAAGCAGAGGGGCAGGCAGCACACCACCCAGCAGCCAGAGCACCAGCCCAGCCATGGTCCTTGAG
GTAAGTGCTGCTGCCCAGCCAGGCCAGGCCAGAATTCGAAGTGGAAAACCCGGGGCATCAGGGGAATGCCAGGGCTGGCC
CACAGCCCAGGGTCACCACAGGGTTGGGTGAGTAGCTGGAGGCCGCATGGGTTGTGGCAGAAACAGCACTGGGGAATTGG
GAACCCAGCTTGGCCACTGACCCTGGGTGCCTCTCCTCCTCTCTGGCTTCGGTGTCTCGTCTGCAAAATGGGGGGGGC
GGGGGGCAAGTAGACTCGAAGGCTCCCTTCAGGAGACTTGGAACTTTGACTCTTGGCGTGCTGGACTAAATTGGTACTGG
GGAGACCTGCAGGTCCATAGTGCTGGGGGTGAGGGGATAAAGAAGATTCCTTCTCAAGCTAAGTGGGGGATCCCCTTGTG
GGCAGCTAAATGGCCGTGCAAGCCTCAGTGTAGGGAGGGGGAAGGACTGGGCAGACTGCATGCATTTGGGGGGCGTGGTG
ACTTGTAGAGAGGCCACACGGTGGGGGGGGGCGCAGGAAGTTGGGGTCCCCGGACAAGGGCCAAGGAGGGAAGAGGAGGG
TTAGTGACTGGAAAAGGGGATTGTGGAGAGGCGCTGGGGAGGAAGGTGGTTGAACTATGTGTTGGGGGTGGGGCACTGAG
GACGCAAGGCAAGCCTGAAGGGAGAGCAGGGAGAGAGAGGACAGTGGGCAGAGAGGGCTCTGGGCACTGGAGGGACGCTC
TTCTTCCTGCCCAGGGGTCCCTGGGCCGATGGGATCACGCAGAAGAATGCGAGAGAAGCAGCCTTTGAGAAGGGGAGTCA
CTATCCCAGAGCCCAGGCTGAGCGGATGGAGTTGAGGAAGTACGGCCCTGGAAGACTGGCGGGGACAGTTATAGGAGGAG
CTGCTCAGAGTAAATCACAGACTAAATCAGACTCAATCACAAAAGAGTTCCTGCCAGGCCTTTACACAGCCCCTTCCTCC
CCGTTCCCGCCCTCACAGGTGAGTGACCACCAAGTGCTAAATGACGCCGAGGTTGCCGCCCTCCTGGAGAACTTCAGCTC
TTCCTATGACTATGGAGAAAACGAGAGTGACTCGTGCTGTACCTCCCCGCCCTGCCCACAGGACTTCAGCCTGAACTTCG
ACCGGGCCTTCCTGCCAGCCCTCTACAGCCTCCTCTTTCTGCTGGGGCTGCTGGGCAACGGCGCGGTGGCAGCCGTGCTG
CTGAGCCGGCGGACAGCCCTGAGCAGCACCGACACCTTCCTGCTCCACCTAGCTGTAGCAGACACGCTGCTGGTGCTGAC
ACTGCCGCTCTGGGCAGTGGACGCTGCCGTCCAGTGGGTCTTTGGCTCTGGCCTCTGCAAAGTGGCAGGTGCCCTCTTCA
ACATCAACTTCTACGCAGGAGCCCTCCTGCTGGCCTGCATCAGCTTTGACCGCTACCTGAACATAGTTCATGCCACCCAG
CTCTACCGCCGGGGGCCCCCGGCCCGCGTGACCCTCACCTGCCTGGCTGTCTGGGGGCTCTGCCTGCTTTTCGCCCTCCC
AGACTTCATCTTCCTGTCGGCCCACCACGACGAGCGCCTCAACGCCACCCACTGCCAATACAACTTCCCACAGGTGGGCC
GCACGGCTCTGCGGGTGCTGCAGCTGGTGGCTGGCTTTCTGCTGCCCCTGCTGGTCATGGCCTACTGCTATGCCCACATC
CTGGCCGTGCTGCTGGTTTCCAGGGGCCAGCGGCGCCTGCGGGCCATGCGGCTGGTGGTGGTGGTCGTGGTGGCCTTTGC
CCTCTGCTGGACCCCCTATCACCTGGTGGTGCTGGTGGACATCCTCATGGACCTGGGCGCTTTGGCCCGCAACTGTGGCC
GAGAAAGCAGGGTAGACGTGGCCAAGTCGGTCACCTCAGGCCTGGGCTACATGCACTGCTGCCTCAACCCGCTGCTCTAT
GCCTTTGTAGGGGTCAAGTTCCGGGAGCGGATGTGGATGCTGCTCTTGCGCCTGGGCTGCCCCAACCAGAGAGGGCTCCA
GAGGCAGCCATCGTCTTCCCGCCGGGATTCATCCTGGTCTGAGACCTCAGAGGCCTCCTACTCGGGCTTGTGAGGCCGGA
ATCCGGGCTCCCCTTTCGCCCACAGTCTGACTTCCCCGCATTCCAGGCTCCTCCCTCCCTCTGCCGGCTCTGGCTCTCCC
CAATATCCTCGCTCCCGGGACTCACTGGCAGCCCCAGCACCACCAGGTCTCCCGGGAAGCCACCCTCCCAGCTCTGAGGA
CTGCACCATTGCTGCTCCTTAGCTGCCAAGCCCCATCCTGCCGCCCGAGGTGGCTGCCTGGAGCCCCACTGCCCTTCTCA
TTTGGAAACTAAAACTTCATCTTCCCCAAGTGCGGGGAGTACAAGGCATGGCGTAGAGGGTGCTGCCCCATGAAGCCACA
GCCCAGGCCTCCAGCTCAGCAGTGACTGTGGCCATGGTCCCCAAGACCTCTATATTTGCTCTTTTATTTTTATGTCTAAA
ATCCTGCTTAAAACTTTTCAATAAACAAGATCGTCAGGACCAGGGCATGTTCGTGCATCATTACAGTCAGCCCAGCCCAC
CTGGGCTCCAGGGGCACTTCCTGGTTCTCCCTGCCTGGCCCTGGGTCTGTGCCAGCCCATCCTGCCTTCATCAATGTCTG
TCTGCAGCTTTGTCTCTGCCCTCGTGGGCACTCCTGTTCTACTCACAGGGAAACACCCAAGTACATGGGTGCAGAGTGAG
CAGTTAGGCAGCTGGTTGGCATAATTGGGGAAGGGGCCATCAGACATGAACACGAGAGCCCTGCACTCCAGCAGCTCAGG
ACAGGAGGCAAGATGTGTGTGAAACTAGCGAAACCCAGCAGCCTGGGCCATGGGGCACATAAGTGGCACAGTGAACCCAG
AGCTCAGGGCACTGAAGGTTGGAGCCATTTGGGAAGGGCTTCTTGAAGACTTAAAGGGCAAGTAGTATTTCCAGGCATAAG
GGAGGGCAGTAGCAACAGTGGGAAGGCTGAAAGTAAGACTGAAAGGTAATTTGGGATCAGGTGATGGTATTGGAGACCAG
TCAGAAGGCCCTACTGGTTTTTTCAGGTGAGTGATACAAGCGCTTACTAGGGAAATGGCTGTGGGCTTGAAGAAAAGCAAG
GCCAGATGACTTGCCAACAGTCATTAGCACACAGCCACCTGGAGGCGGGCTGTGGGGAGCATTTTTCCAGGGCTATCAAT
GAGGCCGAAGTGAGGGAAAGAAATAACTGGGGTTTCCCAGAAAGGCAAGTGACAGGGCTGAGTTAAAACAGAAGGTGACA
AGGTGGGTCTACTGCACTGTCAGTGCCCCCTAATTAAGTCACCAATGTGCTAGGATCCAGTTTCACAGGCCAGCTGCACC
```

```
CCTGCTGAAACTGCAGAAACGGCCGGAGATGGGAATGGGGAGGAAGAATCTGAAGAGTTACCTGGGGAAGCTCGTCCCAC
CACATAAACACACCATCCCGCTTTGTGGCCTCTAGACACTCCAAGCTTTTACAAAATGAAGAAAGGAAGGGAAATGACAG
AATGCACCCTGCCTAGAAAAAGTATCAGGGTAGTCAAGATGCTTGTGGCTGGCTGAAAACATGGCGCCCAAGATGTCCCC
GTGCTAACCCCCAGAGCTTGTGACTATGTTACCTTAGATGGCAAAAGGGAATTGAGGTTGCCAATCAGCTGACTTGAAGA
AGAGGCTCCTGGATTATCCAGGTGGGCCCAAAGTAACCACAAGAGTACTTTAAAAGTGAAAGAGGCGCCGGGAGCGGTGG
CTCACACCTTAAATCCCAGCACTTTGGGAGACTGAGGCAGGCGGACCGCTTGAGGTCGGGAGTTCGAGACCAGCCTAGCC
AGCATGGCTAAACCCCGTCTCTAATAAAAACACAAAAATTAGCTGAGCACAGTGGTGCATGCCTGTAATCCCAGCTACTC
AGGAGGCTGAGGCAGGAGAATCACTTGAACCCAGGAAGCGGAGGTTAAAGCAAACCAAGATAGTACCACTGCACTCCAGC
CTGGGCGACAGTGAGACTCCATCTCATAAAAATAAATAAATAAAAATAAAAGTGAAAGAGAGAGGCAGCAGGGGAGTCAG
AGAGGGAGCTGTTACTGCCTCTGGAGGACGGGACCATGAACCCAGGAAATGTGAGCAGCCTCTAAAAGGCAGAAAAGGCC
GGGCGAGGTGGCTCGCGCCTGTAATCCTAGCACTTTGGGAAGCCAAGGCGTGTGGATCACTCGATGTCAGGAATTCCAGA
CCAGCCTGGCCAACACGGTGAAACCCTGTCTCTACTAAAACTACAAAAATTAGCCGGGCATGGTGGCAGGTGCCTGTAAT
CGCAGCTACTCGGGAGGCTGAGGCAGGAGAATCACTTGAACAGGAGGCAGAGGTTGCAGTGAGCCAAGATCGTGCCAC
TGCACTCCAGCCTGGGTGACAGAGCAAGACTCTGTCTCAAAAAAAAACAGAAAGGAAAATACAAGGCAACAGATTCTCCCC
CAGAGCCTCCAGGAAGGTACACAGCCCTGTCAACACCCTGATTTTAGCCCAGTGACACCGGTGATGGACTATCTGACCTC
CAGAACTGTAAATGTGTGTTGTTTTAAGCCACTATGCTTGTGGTAATTGTTGTGTGGCAACCATAGGAAACGAATACAATG
TGGATCCCAATCCACATCCTGAATCACTCCAAGAAGTAGGGAAGCTGCTAAGTCCTTATGGGCAGTCACAGAAATCCAAG
GAGAAACACGGAGTGCGGCACGCTCCTCTAGACGAGTCCTGGAGGGCTTCAGATGTTCGGAGTCTTGGACAAAAGACCAG
AGGGTCTGTTCTGCTCTAGGTGCAGGGATTCAATGAACAGACTTTGCTAACAAGCAAGATGCAGAAAACTTTTATTTTTT
GATTACATGGCTCAGATAGTTTCAATCAATACCATCAACAACGAATTTTATGAAACACGTTTGTCTTTCCTGTCTACATA
GATGCCACAGTAGCCCTAGTGTTTAAGTGTTGCCTCTCAAACTTGTCCTCTTTCCAGCATTCTCCTCTCTCAACCTGCCA
CTCTTGAAAACTCACTTTTCTTTTGTTCTTACATGTGAAAAATATGGAACTCAAGTAAGACCCAAGGTAGATCC
CAAGGGCTTCAGAAATACCCTGTCAAGGGCACAGAATGGTTAGCAATAGAGTCCCTATTTAAGGTCAATCATCGTTAATA
ACATGAATCTTAAATTCTAACATTACTTACTTAACAGTACCTAAATTCTAATATTCCTAACTTCACAAAACAAACTTTTC
TAAAACTTCTTACCAGTAAAGGATATAGCTCACATTAATATACTCTAAGATAACCTTAAGAATTCTTCATTGCCTTGTAA
TTCCTGCCAAGTGTTTTCATCACATCCCCAAGGCTAATCCTAGCCATCTCCTGCATAATTTTTCTATAATACTTCTGCAC
ATACACAGCAAATGGTCACACGTGGGGCACAATACGGGTCCCATCTTTCTGAGTTAATGGCACCATGACCAGAGACCCCT
TGCATTTGGCACAGATGAAGCGGCTGGTGTCCAACGATTTGGTGTAGCAGCCAATCCTGGGAAGGAATGGAACAACACAC
AACAAAAGAATCAGTCACTGCACCTCAGCATCAGCTCTGAGATGTTAGCCTTGGGCCCAAAGCAATACTTTTCTCTGGGT
TGTCAGAGACCCGGGCACCAAGCGGCAGCAGTGGTCAGGCCGGCTCAAATTACGGAAGAGAAAGGAGAAAGAGAACGTGG
AAAGGAGGAGAGAGGTTCTCCTCTTCAGTTCAGACAGCCATCTACTTATAAAATCACTGAAGAGCCACTTGGAGGCCCCA
GCAAAACGTAACTGGGAAAAGTCTGAGAAGAGTCTTACCTCGTTTTGCATCCAGTACATTCATAATGGACCTTGTAGTTA
ATCTTATAGTTATGGCAACGGGTGACCCTGGGCAGCTCCGGGTGTATCCTGTTGGATTTCCTGGCATAATACTTCCATGC
GTCACCATGAGAATCATGGATACCATCAATCAGCCAGGAGGCAGCATGGCACATTTCATGGATCAAGGTATCCCGGATTC
GGTCTTGAAAGAAGAATGAAAAGAAATGTGAGCACTAAAACCAATCCTCTTTTTTTTTTTTTTTTTTTTTTTTTTCCTTTT
TTGAGATAGTCTTGCTTTGTCGCCCAGGCTGGAGTGTAATGGCATGATCTCAGCTCACTGCAACCTCCACCTCCCGGTTT
CAAGTGATTCTCCTGCCTCAGCCTCCCAAGTAACTGGGATTACAGGCATGCACCACCATTCCTGGCTGATTTTTTTTTTT
TTTTTTTAGTAGAGACGGGGTTTTGCCATGTTGGCCAGGCTGGTCCCAAACTCCTGACCTCAGGTGATCCACCCACCTCG
GCCTCCCTAAGTGCTGGGATTACAGGCGTGAGCCACCACACCCGGCCCCAATCCTCTTCTTATCTGCTACCCAACAACCC
CTCGACTTTCAGCGCCACCAGACACAAGGCTATCTTAAAGAAGCACATGCAGGCAAGCACGAGTGTTACCTTCCCAGGAT
TCTCAAAGTGAATAGTGAACCTACTTCAACCCGATATTGTTTATGGCAAAGAGCCTTCGGTGAGCAGGTCTTTTCAGAGC
AGCTCCAACATGCTCATTTTTTTATGAGGCACTTCTAGAAAAACATCAGGACAAGATCTAGTTTCTGCCCTCCAGGAACA
GAGATGGACAGGGTGGGGAGCAATCGCTATGGCCGTGGGATTTGGTTAGGAGAAGCTCCATGGTGAAATTGAGATCTCAG
GGGTTCTTACTAATTGATTGAATTTGCAAAGTTAGAAAGGCCATTCTGGGTGTGTGGCAAAACATGAGTATGTGGCACATTT
TTAAAAACATTTTAGAGACTAAGTCTCGCTATGTTGCCCAGGCTGGAGTGAAACTGCTATTCACAGGTGCGATCATAGTG
TACTACGGCCTCGAACTCTTGGGCTCAAGCAAGCCTCTTACCTCAGCCTCCCAAATAGCTGGGACTACAGATGGGTGCCA
CCACACCCAGCCTGTGGTATGTTTTGAAGGCCAGTGTGAAGAGAGCCTGTCTGGGGCATGGGCTAGTGGTAGAATACTGA
GAGACAAGATTGGCCAAGCCAAACAGGAGCAGATTACAATGGCCTCAAAAGCCAGGCTCTAGGCCTTGAAAAGCCCTGGT
TTCTCAGCAGGAAAGCAATGTAGTGGCATATGGGAGACCAAACAGAGCAGGGATGAGGGCCTGTCCTTGGATGGAGGCAC
AGATAGAGAAGGAATGGTGAGCTACTCTGAAGGAAGATTTGGTGATTGGCTAGAAAGAGGGGACCTTGAACCTCAGCTCC
TGAGAGAATGAAGATGCTACTGCTGGCAAAGGGGAAATGCAGAGAGAAAGGCAGGCCTGGTATAAAGATCGATAGGTTTG
ATTCACAGGGATGGTGAGACATCCAATAACTATGTCCTCTAAGCAACACACAGCATGCCTGCCCACCCAAGAATGACAGT
TCGAGCTGTGACACAGGCTACCAGCTAGGAGTAATTTCACAGGCACGGTGTCAGAAGGAAAGAGCACAGTGGTGAAGCTA
CCACACTCCTGCCATCACCTGCAGAGTCGCGAGACTTTCAAGCCAATCTGGATCTTGGCAAAGCGCCGCCACTTTGGGTAC
CACATCTCACCAGTGCTGCATAAGCCAGCAGTTTTCACCATCTTGTTATTCCAGCCTATGCGTAGTTTCTCTGGCAGCTG
AGAATGAAACGCAAATGAAAGGAATCAGAAAGCTGTTTTGGGTTTTTTTGTTTTTTGTTTTTGAGACGGAGTTTTAGCT
CTTGTTGCCCAGGCTGGAGTGCAATGGTGCAAACTCAGCCCACTGCAACCTCTGCTTCCCAGGTTCAAGTGATTCTCCTG
CCTCAGCCTCCCAAGTAGCTGGGATTACAGGCATGCGCCACCACACCCAGCTAATTTTTCGTATTTAGTAGAGACAGGGT
TTCACCATGTTGGTCAGGCTGGTCTCGAACTCCTGACCTCAGGTGATCCGCCTGCCTTGGTCTCCCAAAGTGCTGGGATT
ACAGGAGTGAGCCACCACGCCGGGCCCAGAAAGCAGTTTTAAGCAGACCAGAAAATATTAACTCCAGAGTTTTTTACCCT
CTAATAAAAAGGAAAAAGAACCTTCTCCCCCAGGTTCTTGGCTCAGAGCAAGTTTGAGAAGAAATTTATTCAAAGAGCAC
```

```
ACAAATCAAAACATTCTGAGGAGAATAAAAGCCACATTAACGTCTGGGTAAAGACAACAAACAGGCAATCAGGACACAAG
GGAGATTGATAGAACACGTGAGTGCAGGACTTGGGTCTCTTGTCCTTAGGGCAGCCAAGGAGAGAAGGACAACCCTGCTG
AGGTGGGCGAGGCTGGACTGAAAAGGCAACTCAGAACACTTCAGTTTCCACCCATCTGATGTGACACCACTGTCATCCAG
AAGGGCATCAAAGGCTGGGGAACCTTTAGGAATATGTAAGAACTTCCCTCCATAAAGTCTTGTCACCAGAGATGATGAAG
TCTGAAAAATCTAATGACCAATAGGTCTCTCTGCCTCCAGATTTTCTCCAATCCAGTCCATACCCACAGGTTAGTGTCAG
GTTAATTTCTTTCAGCAGGTTAATTTCATTCACTCCCTGATGCACCCCATCCCTGTGCAGTGGTGCCTACTGATGTAAAT
TTCACCTCCTCCAACCTGATTCCCAAGGCCCTCTGGTATCCAGTCCTACACTATCAAAAAATAAGAGCTTACTATGTCCT
AGGCCCTGTTCTAGGCACTGTATGTGTATTAACTTATGTAAGGGTAACAATAATCCTATGAGGTAATACGTACTGTGTTG
TCCCCATTTTACAGATGAAGAAACTGAGGCAAACTGAAGTCAAGTAACTTGTTCAAGGTCACACAGCTCTTTTCCATTAT
CCTTCCACAAGAATCCTCTCTTCCTGATTGTCGAGCCTCAACTCTCCCTCTCCCCCATCCTGTTTGCCACTTACCATCCT
TTAAGAAGCAGAGTCTCACTTCCTCCAGGTAATCTTACTTCCTGGCTGTTGAGGCTCTTAGTGATTTCTCCCTCTGCTGA
TCTCCTGTGGTAACAGCCATCTGTCCTATACATTATAACTCATACACATTCTCTATTGCTTTCACCTGTACCAGGGCTCA
TCTCCCAACCTTAACTGCCTCTGCTCAAGGGCAGGAAGTATACATGGTTAGCACACCTCACAGCACCAAGAGTACTGGTG
TATAGCAGGTGCTCAATCAGTGCTCATTCATTGATCCTACCTTTTTATCACAGACGGATCTGTTAAACAGGTCGTAGATT
CTCTGAACCAATTCATCCTTATTTCGCTTTAAATTTTTTCCAGAGTATTTCTTTGACTTTTCAAGGTCTTGCAAGAAACA
TCCAGGGACTTTGCATTTAGGAGTCCTGCAAAGATAAATTCAACTGAGGTTTATGTAGCTAAAGAGAGAGAAGGAATGCA
CACCTAAGCGTCTCGTCAAGAGACCCTGAAAACATGTTGCTCCCTCTTCCATCCAGCTCCTTAGATCAGGGCATTTCCCA
AGCCTTCATTTGTAGCCTTTTCTCTTCTCTCTTTGCAACCCTCAGAGGTGTAAAGCCCGTGGTTTCAAGCTCAACGAGAC
TTATCCCCACATCTGTATCTGCACTCTGGAGCTCTCTCCTGAGACCTAGTTCTGAATTTACAATTGCCCAGTGCAAACTT
CCAACTGATTAACATGAATGTATCTCCAGCTAAACGTGTGTTCAATTATCAAACTTGTGTTACAATTGGCTGAAAAACCT
CAAAGACATTTAAAAATTCCTCTTCTTGGCTGGGTGCGGTGGCTCGCGCCTTAATCTCAACACTTTGGGAGGCCGAGGCA
GGCGGATCACGAGGTCAGGAGATCGAGACCATCCTGGCTAACACGGTGAAACCCTGTCTCTACTAAAAATACAAAAAATT
AGCTGGGCGTGATGGTGGGCGCCTGTAGTCCCAGCTACTCGGGAGGCTGAGGCAGGAGAATGGTGTGAACCCAGGAGGCG
GAGCTTGCAGTGAGCCGAGATCGCGCCACTGCACTCCAGCCTCGGCGACAGTGCGAGACTCTGTCTCAAAAAATAAAAACA
AAATAAAATTCCTTTTCTCTCTTTATTGGGCACAGAGACAGACTATCTTCTTCACACTTTCTCTTCCAGCCAGCACCTCC
TTTCCATCCCCCTGGTTCCTACTCTAGTTCAAGTTCTTGTCCTACCCTTTAATGCCTAGATATGAGAACACTCCAGTGTT
CCTTACCTCCAGGCTCTGTCTCTTCTCATCCAATCACACACACCCCTCCAGATTCATTTTTGCAGGCCATGCTCCAATTA
ATGTCCCCCTCTGGTTTTTCACTGACTGCCTGGCCTGACATTCATGGCCAGGGTCAACCCTGTCCAAAGCTCTATTCCCA
GCTACCACTGACACTCTAGCCAAAGTGGGCAACTTAAATGTTCCCTGGACACAAATTGCAATTTCCCGCATTTGTGCCTT
GGTACAATCTATTTTCCCATGGCTTACAAGGACTACATCTCCACCTCTACTGAGAATAACCTTCTGTGTAGCACTTTATC
ATTTGCAAAGAACTTCTTTTTGCATTGTCATATTTAATCCTCACAACTGCCCTTCAAAGAAGCTGTTACTATTTTTACGA
TCTCCACTGTGCTCAGGGGTCAAGTGACTTGCCCAGAGGTCACAGAGACAGTGAATCTCCTGATCCAAATCTCATGGTCT
TTTGATTAAACCACAATCGCTGCCCCATCTTTCAAGGCCTAATTCAAATGGCACAAAGAATAATGCTGAACACATAAAGG
ACTCATACTGTAATAACTACCATTTAAACAGAAGCATTTTTATCTTAGCTGGAACAGTCCACTAAGTCCAGAGATGAGCC
CAAAGAGCTGGAAAGACAAAATGGGAGCAAATGCAGCAAGTTTCTGACTTAATAGAACTCCTAAGACTAAGAATTTGCCA
GAACACTTATTAACCCTTAAGTCACAAACTAAACCCTACGTGAGGAAGTTGGGCTGCAGAGGGGGGAAGTACCAGACAAG
ACAGAGGTTACAGAAGGTTCTTCATCCCAAGCCTGAAATGGGGGACTCAAACATTTCTGCCTCCAGTTACTTAGGTCTGA
AAGCAAATTTACATCAGTATTTAAGGTGGCAGATGCAAACCTGAACCCTCTGCAGACTTTGGCAGAGCCCGGACCCCTGT
GTGTTTGTGCTCACAGAACGTTGGCCTTGTGGCTTAGCATACTTTCACCTCAAGGCCTGTGGCTCTTTACTGCAGGCTGA
GCACCTTCACATCAGAGGAACCGACAAGACCCCAAACTGGGTACATCAGACACCATGACCAGCAAGCAGCCTACACAGGA
ACAATGCTAAGCATAACAGGAAGCCTCCAGACTGACACAGGAAGTGCTAAACAACAGTTAAGAGTCCAAACGGGCTAGCC
TCCTTTGGATAATGCAGACTGACAATGACACCAAGGGACAGCTTTTCCCTTCTTTTGTCACGCTCTAGCAAAGTAACCTG
GGAGTTACCCAAGACACACGTCCCTGGAGGCAAAGCAGCTTAAATATGTAGTTAGGAGTCTGGAGCAGAAACCTTGCTGA
AAGGGGATCCAGCTAAGAGAGTACTTGGCAAGCTGCTACCGGCTGCCTAAGCCAGTGCAGAAACAGACAGCCCTTCTGCT
CAGGTCCGGGGTAACCTGGTGCCCCTATGCCTGACACATCAGTCTTTTTCCCCTCCCTTAATATACACTTATTGTCCCCA
AATACTCGTTAAAAAATAACATATAGTCCTTCAAATACATTCTTTGTCTTGTAAGACAATTCAGATGTGAGCATACATTA
CTCTTCAATGAAAGTAAAAACAAAGAAAATTATGCTTACTATATCAAATTTTTAAGGGAAAAACTAAGAAATTCCTGGAA
TCTAAATAAGAAAATATATTAATTTTTTGCCTTAAGTATTAACATGTCTTTGGTCTACAGGATCAGTGGTTGTTTTCAGT
ATCAACTATATATAGCTTAATAAAAATAAAGGAATGATAAGCAATCCATTTCATCCTAATATTTAAGCTTAAAAATTCAT
ACAGATATACATAGTAGTAAGAGTAACTGGGTTCAGCCAAAGCACTAGAAAAACCTGGAAGACAATCTTGACCACTCTCC
CTCCAATTCCAACAGATTAATGTGTTGCTTTCGCCTTCTCACCATTTCCAGTGCCTTTGCCTTGGTTTAGCACAAACTGG
TGGCCATGAGGCTGTATCCAGCCTGAAGAAAGTGTCTTGTTTGGCTACACATTGTTCTTTGATCTGAGTTGAAGGACAT
AGGCAATCTCAGAGGCCAGAGCTGCTGTGGGTGCAGAAAGGCCCCTCCACCCAGGTAACTGCAGGCACCTTGGCGGAGA
GCCCATGCCCAGCCTCAGAGAGTGGGGAAAGAAGGGAGGAAGGAGCCA
```

HUMAN mRNA SEQUENCE : hR5-034.1 (Seq ID No: 53)

```
CCAACCACAAGCACCAAAGCAGAGGGGCAGGCAGCACACCACCCAGCAGCCAGAGCACCAGCCCAGCCATGGTCCTTGAG
GTGAGTGACCACCAAGTGCTAAATGACGCCGAGGTTGCCGCCCTCCTGGAGAACTTCAGCTCTTCCTATGACTATGGAGA
AAACGAGAGTGACTCGTGCTGTACCTCCCCGCCCTGCCCACAGGACTTCAGCCTGAACTTCGACCGGGCCTTCCTGCCAG
CCCTCTACAGCCTCCTCTTTCTGCTGGGGCTGCTGGGCAACGGCGCGGTGGCAGCCGTGCTGCTGAGCCGGCGGACAGCC
CTGAGCAGCACCGACACCTTCCTGCTCCACCTAGCTGTAGCAGACACGCTGCTGGTGCTGACACTGCCGCTCTGGGCAGT
```

GGACGCTGCCGTCCAGTGGGTCTTTGGCTCTGGCCTCTGCAAAGTGGCAGGTGCCCTCTTCAACATCAACTTCTACGCAG
GAGCCCTCCTGCTGGCCTGCATCAGCTTTGACCGCTACCTGAACATAGTTCATGCCACCCAGCTCTACCGCCGGGGGCCC
CCGGCCCGCGTGACCCTCACCTGCCTGGCTGTCTGGGGGCTCTGCCTGCTTTTCGCCCTCCCAGACTTCATCTTCCTGTC
GGCCCACCACGACGAGCGCCTCAACGCCACCCACTGCCAATACAACTTCCCACAGGTGGGCCGCACGGCTCTGCGGGTGC
TGCAGCTGGTGGCTGGCTTTCTGCTGCCCCTGCTGGTCATGGCCTACTGCTATGCCCACATCCTGGCCGTGCTGCTGGTT
TCCAGGGGCCAGCGGCGCCTGCGGGCCATGCGGCTGGTGGTGGTGGTCGTGGTGGCCTTTGCCCTCTGCTGGACCCCCTA
TCACCTGGTGGTGCTGGTGGACATCCTCATGGACCTGGGCGCTTTGGCCCGCAACTGTGGCCGAGAAAGCAGGGTAGACG
TGGCCAAGTCGGTCACCTCAGGCCTGGGCTACATGCACTGCTGCCTCAACCCGCTGCTCTATGCCTTTGTAGGGGTCAAG
TTCCGGGAGCGGATGTGGATGCTGCTCTTGCGCCTGGGCTGCCCCAACCAGAGAGGGCTCCAGAGGCAGCCATCGTCTTC
CCGCCGGGATTCATCCTGGTCTGAGACCTCAGAGGCCTCCTACTCGGGCTTGTGAGGCCGGAATCCGGGCTCCCCTTTCG
CCCACAGTCTGACTTCCCCGCATTCCAGGCTCCTCCCTCCCTCTGCCGGCTCTGGCTCTCCCCAATATCCTCGCTCCCGG
GACTCACTGGCAGCCCCAGCACCACCAGGTCTCCCGGGAAGCCACCCTCCCAGCTCTGAGGACTGCACCATTGCTGCTCC
TTAGCTGCCAAGCCCCATCCTGCCGCCCGAGGTGGCTGCCTGGAGCCCCACTGCCCTTCTCATTTGGAAACTAAAACTTC
ATCTTCCCCAAGTGCGGGGAGTACAAGGCATGGCGTAGAGGGTGCTGCCCCATGAAGCCACAGCCCAGGCCTCCAGCTCA
GCAGTGACTGTGGCCATGGTCCCCAAGACCTCTATATTTGCTCTTTTATTTTTATGTCTAAAATCCTGCTTAAAACTTTT
CAATAAACAAGATCGTCAGGACCAGGGCAT

HUMAN PROTEIN SEQUENCE : hP5-034.1 (Seq ID No: 54)
MVLEVSDHQVLNDAEVAALLENFSSSYDYGENESDSCCTSPPCPQDFSLNFDRAFLPALYSLLFLLGLLGNGAVAAVLLS
RRTALSSTDTFLLHLAVADTLLVLTLPLWAVDAAVQWVFGSGLCKVAGALFNINFYAGALLLACISFDRYLNIVHATQLY
RRGPPARVTLTCLAVWGLCLLFALPDFIFLSAHHDERLNATHCQYNFPQVGRTALRVLQLVAGFLLPLLVMAYCYAHILA
VLLVSRGQRRLRAMRLVVVVVVAFALCWTPYHLVVLVDILMDLGALARNCGRESRVDVAKSVTSGLGYMHCCLNPLLYAF
VGVKFRERMWMLLLRLGCPNQRGLQRQPSSSRRDSSWSETSEASYSGL*

Table 10

MOUSE GENOMIC SEQUENCE : mD6-034 (Seq ID No: 55)
CTGGCTGGCTTCCAAGGACTGCAGCAGCCATGGAGGCAGTGAGGAGCATGACACAATCATCATGGCCAGAAAAGGCATGG
CCAGGGACAGGACCCGTGCGGGGGTGGGGCAACCGAAGGAGAGATGGACAGATGTCTGTCCCTGGACCTCAGTCCCCTTC
CTGTTTTGGGATGCACAGTGAACACACTGGTAAGCTGCTGCAGGAGTTAATTCAGGGGCTGCAAAGGCAACTTGGGTTGG
GCCTTGCACAGACCAGGCAGGTATAATATCACACCTGTCATCCCAGCACTTGGAAAGTTAAAGCAAGAGGGTTGTGGCAA
ATTCCAGGCTAGCCTGGGCTACAGAGCGAAATGAGACCAAAAGATAAAACTCCCGGGCTGGCCCTACCACTCAGTGGGC
AGAGCTCACCCAGCTGCGTAAGCCCTGAGTTCCATCCCAGCACCGCAGAAACCAGATGGGGTGGCACACAGCTGCCGTCC
CTGACTTGGGAAGTAGAGGCAGAAAGCAGGCATGCAAGGTCATCCTTGGCTGTAACCAGTTGGAGGCCAGCCTGGGCTAC
ATGAGACCCTCTCAAAAGAGAATCTGTAAATATCTTTGTGACTCCCCATCGTGTCCCCATCCTGTTCTCTCCAACGGCCA
CTATTGCCCCACCGTGGACGGTGTGTGAACTGTACTGTAAGGCCTGGGGGCTCCTCCAAGGCTGAAGAGGTGCCCTCTCC
GGCCCACTTCTGTTCTCCTCAAGCCATCCCAGCAAGTCCCCTGTCCAGCCCCACATCAACCTCAGTGCAATATGCCCTCC
CCGTGGGTCCCTTGGCACGGTACTGTCTGCAGCCCCTTGTCTCTCACCCCTAGCTCCACTCTCCGGTGGTTAAAAATCG
CATCGCACATCCTCCCCCCAATCCTGTTTTTATTGTTGAATCCATTCTGTAAACTACAAACCTCGTGTGTCTTACTCATC
AGGACTTGGGATCCCTCGTCCTCAGCCTATGTGGTCTGGTGTTTGACAATCCAAGCACAGTGTCCTCTTGGCACCAACCA
GAGGGGCCTGTCCCTCCAGCGCAGCCGGCAAATGTATGCAGTCCAGGTCCCCGCCCCACCTGTCACAGCCCCTCTTTTTG
AGATCTGTAATAAATCTCTAATAAGAAACAGCTCCTCTCCGGTGACTTATGCGTGCGTGCGATGGGAGAGCAGGCGAAGG
GTGGGCACCACGTGTTTTGTCTCCCCCGCTCTAAACCCTCCCTGGATCCCACATGATGAATCCCAATTCGCAGATGACTT
CCTTTCTCCAAGCCTAAACTTCCCTATCCATAGATTAGCCACAAGTTGAGCGCAGTACGCAAGCTGGCGGGCGTAGTAGT
ACTCAGACTCGTCCTGGAGTCAGCCAGAGACTGTCTGTTCATGTCCTGTTGTTTTATTAGCTCGGAACCCGGTTGCTACAA
GGCTACCCTAGAAACCGAGGACTCCCGCGAGCGCTTGGGTCTCCGGGTTTCAATTTTTAGAAGTACACCGGGCCCTGCAG
GTTTAAGTTCCGCCCACTCTAGCCACGCCCCTTTGACGTCCTTTTCCAAGCCCAGCAAGACCCCTAAGCGCAGTTTCTGG
GTTCATTGGCCCCGCCCTCAGATCCAATGTGTCCATCTCGCCCCCAAGGTCCCGCCCACCGCCGCTCTACTTCCTCCCAC
TCCTCAGTCGGCCACTCTCGACTACATATCCTGCCCGTACCCATTCACAGTTCGTAGTTACAGATGTCAATCTTGCCTCA
ATCAGCCCCTCTTCCACCCTCTCAAATCCTGATATGCACTCCTCCGCCCATTGGTCTACGCCTGTCAATCTAGTTTACCA
GACCCCGCCCACCCCGGCTCCCGTAACCGCCATTCTTGCGTTCCTTCTCAGGCCACGCCCAGCGCAGGTCCCCGGCCGGG
TCTCCCGATCTACAGTTGTCGATCTCCGTAAGCCCCGCCTAACCCAGCCCTATTCTTTCCCCATAAGCTCCTCTACTAGG
CTCCGCCCCTAGGGCACTGCCCATGCAAGTGCAGGCTCCGCCCACTAGGCGCCTCTTCCGCCCTGGAACAACCGCCTCGT
AGCTCCGCCTCCCTAAGCACGCTACTCGCCGGTCACCTAGGCGCGAGGGAGGGCCCACCTAGGTCAGTCTCCTTCACTG
CATGAATGGAGCGTCAATCCGCTAGTTCTAACGGCGTCCCGTTAGGAATCTACTATGTGTTCCCAGCTTGAGAACTCCGG
AGAAATCTCGGGTTATGTTGTCCCTGGAGTCCCGGTTAGAGGGAGTAAGTCATTCCCTGTGATCAGGCGCACTTTGGACA
GTCCTTAGAGCGCCGCGCCAAAGGAAAGCCCCCGGCGCCAAGCTTCCTGGTTGTTTACGCGCGCGCATGCGCGGCGCGGG
CCGCCAAATGGGTCGGGACTCTCGCTCGCGCTCACGGTCTGTGGGTCGCAGGGGTCGGAAGCAGCGGAGTCGAAGCCGGG
GCCGGAGCCGCAGCCGGAGTCGGAGTCGGAGTCGGAGTCGGAGTCGGAGCCGGAGCCGGAGCCGGAGCCATGGGCGGAGC
AGCCGAAGGCGACGGGAACACGAGCGGCGGCGCGAACGCAAGCGGCGCAGCCGAGGGCGCAGGTGAGCGTCCTGGGGGCT
GCTCGGGCCTGGGAGCCGAGGGACAGGCGGGGCAGTATCCGAGATGCCGGGCTGCGCCGGGGCAAAGGAGACTTCCGGGG

```
ACCGCGGGCCATGAGCATAGGGGACCGAATGACTCCACTCGCCACCTCTGCATTCATAGACATTGTCTTGTCCCGGGCTG
GCCTCCTGCGTCAGCCTCTGGAATGCTCGGGATTACAAGCCATTGCCTCACAGTTAAGGTTTTTTGCATTTCCTCATAGT
TAATTCTGCTCAGTTGACAGCCCTTCTTCTCAACTGGCAGTACTTCTAACAAGTTGACCAAAGCCTAATCAGGACACCGT
TCATACAGCACTGGAGATACAAGCCCGAACAGCCACATCTATCTTTTACTGTGGGTACTGGGGATTCGAACTCATGTCCT
CAAAATACTACCTGGTGGTATTCCTCTTTCGTGGTTCCTGGGCTGTCAGAGAGTCCATCTATTGGGCCTCTCGTCTGTCA
TTTCTCATCCCTTCTACCTGTCATCTCAGCAGGACTTGACGGCACTGTACACACCAGTAACCCCAACACTTAGGAGACAG
ATAAGAACCAGCCTGGCTGTAGAGGGAGTTCCCACCTGATAGCATTGTAGATGTCATATTTAAAATAAAACAAGCACTCA
GAAAGCAGAGACAGGAGGGTCTCTGAGTTCCAGGACAGCCAGGGCTACACAGAGAAGCCAGCCCTGTCTCGAAAAAAATC
AAAAATTAAAAGAAAATAAAAACTACATAAAAAAGAACTACTCCCTCACTTTTAACCTTCATTTCTCTTGCTGTATTGTC
TGTTATGGCCCTGGGACTTGTGTAGCTCTACTATGAAGGACAGCCTAGCTCATGTGGGGAGCAATGTGATTTACAGGCCG
GTGAGGCTAGAATTCTAGGCAAGTACCTCAGCACCTAGGATGGTGCCCAGCAGCAGCCAGCATTGAACAGGTCTTGATGT
GTCCTTCCCACAGGTCAGATTCGGAAGGAGAGCAGCGGCAGAAGTCTAGGAGGCGAAGCCAGAGCCTTCGACCACCCCGA
TGGCACTCACAGAACCAGTCCTCATGCTCTGACTCGGGTGAGGAGCGGGCTCAGGGCTCCTGGGCCCGTAAGGGTCACAG
GCGCTCGTGGTCCCCGGGGTCATCAGCGTCTAGCTTGGACTCCCCAAGACGCTCCCGGAGTCCCGGGACAGCTACCCTGG
CCCTGAGCCAGCAGCAGAGCCTGCAGGAACGGCTGCGGCTTCGTGAGGAGCGGAAGCAGCAAGAAGAGCTGCTCAAGGCC
TTCGAGACGCCTGAGGAGAAGCGTGCGCGGCGACTGGCCAAGAAGGAAGCTAAGGAACGCAAGAAGCGGGAGAAGATGGG
CTGGGGTGAGGAGTACATGGGCTACACCAACACCGACAACCCCTTTGGAGACAACAACCTGCTCGGCACCTTCATCTGGA
ACAAGGCCTTGGAGAAGAAGGGCATCAGCCACCTGGAGGAGAAGGAGCTGAAGGAGCGCAACAAGAGGATCCAGGAAGAC
AACCGTCTGGAGCTGCAAAAGGTAGGCCGGCCTCGCACGCACGCACACTTGCATGGGAGCTCACATCCTCTTCTGGAGGC
TGGATGTGCCTGGACACACGCCTCTCACCATGGTGGCTCCCTGTGTTGCTGTCCAGGTCAAACAGCTGCGGCTAGAGCGG
GAACGGGAGAAAGCCATGAGGGAGCAGGAGCTGGAGTTGCTGCAGCGGGAGAAGGAGGCAGAACACTTTAAGACGTGGGA
GGAGCAAGAGGACAGCTTCCACTTGCGGCAGGCCAAGCTTCGGTGCGCTCCTCCCTGGCCTTCTATTGGAAACTACAATC
TCATGCCAATCTCTGCCAGGCGTGGCGTGTGAACTCATGAGACAAATCCCCGGGGAGTCCCGTTAAGACAGTGATGGGGA
TATGGGATCCATTGTCCAGAGTATCTCCGCCTCACTGAAGGAACCTGAGCACAGACCTGGTCAGATGTATTGAGGACCCC
AGCTCAACCCTATTGTCCCACCTTGGGGAGGGCCAGGCTCACAGGGCCATCTGACACTCCTATCAGTCTGAACCTGTTTG
GCTGGTTGGGGCTGAGGGTCAGCTGTGCCAGCGGAACTCTGGCCCTGACCCTGCCACCCTATTCCTCCTGCAGCTCCAA
GATCCGAATCCGAGATGGGCGAGCTAAGCCCATCGACCTCCTGGCCAAGTACATCAGCGCAGAGGATGACGACCTGGCGG
TGGAGATGCACGAGCCCTACACCTTCCTCAACGGCCTCACTGTGGCGGACATGGAGGACCTACTAGAAGACATCCAGGTG
CTGCACATACTTCGGTCTGGGTAGCTGTACCTGGCTCTGCCCTGCCCTACCCTGCCCTATATTGCCTCAGCCCTCCTCCC
CACACCCCTCCTTCAGGCCCCACCGCTCACAGCCCTGCCCCACAGGTGTACATGGAACTAGAGCAGGGCAAGAATGTGGA
CTTCTGGCGGGACATGACGACCATCACAGAGGATGAGATTGCCAAGCTCCGCAAGCTGGAGGCCTCTGGCAAGGGCCCAG
GTAAGCAGTGTGGGACATCTTCCCAGCTCCTCTGAGGCCTCTGCAGGGCATGGCAGCGCCTGAATGCTGGACAATAGAAT
GTGTCTCCTAAGGAGCCTCAGGGAATGGCCGCAGCCCGAGACAGCTGCTATCCCTGTCTGCAGAGCCTCAGCCTTCACCA
GGGCAAGCAGAGTCTAAATTGGTGTGCTGCCCAGCTACTCCCTACCAGGCAGGCCTGTTCCTGAGCCCCGGGTGGGGAGC
TGGGAACCTCAGGCCATGCTCAACAACCTGACCTACAGGTGAGCGCCGTGAGGGAGTCAATGCCTCAGTCAGCTCCGATG
TGCAGTCGGTGTTCAAGGGAAAGACGTACAACCAGCTGCAGGTCATCTTCCAGGGCATCGAGGGCAAGATCCGGGCCGGG
GGCCCCAACCTGGACATGGGCTATTGGGAGAGCCTGCTGCAGCAGTTGCGTGCACACATGGCCAGGGCCAGGTGAGGCTT
TCACAGGGCCTTGGCACCTGTGTCCTCCCTGACCAGTGCTTCTTTAGTGATTGTCACCTCAGCACCGGGCTGCCTATCCC
GCTGCTTCACCCAGGCCCCTCCTCCTCCCTACCCTTATACACATTTCTTTTTCCTCCCTCTGTCCCCTCCCAATCTCGGCA
AGAGAATTGTATATAGATTCTGTGCATGTTTGGCAGTGGTGGCTGCTGGATGTCCCCATGCCCCATGGATGGCAACTGTT
GGCTTGCCTTGGTTCCTCTGGATGTCCCCCATCCAAGTAGACCAAGCACCTCAGCCAGTTGGGGCCACACTGTGTGAATG
TGCCCTACCAAGGGACTGAGATAGTGACACCCTCCTTTTTCCAGGCTCCGTGAGCGCCACCAAGATGTTCTTAGACAGAA
GCTGTTCAAGCTGAAGCAGGAGCAGGGTGTGGAGAGCGAGCCGCTGTTCCCCATCCTCAAGTCGGAGCCCTCGGCCGCCC
ACAGGTGAGCAGCGTGACCCGGCCAGGTGGGGCACCCTTAGGTGGGTGAAGAACTGGTACAAGCCTAGCAGGATGGGCCTC
ACCTCTTCCTTCACCACCCGTACAGCCCCGAACCTGAGGAGCGACCCCCCAGCCCCGGGACTTCAGTGGACCCAGTGGAG
CCTGTGGAACCTGAGGAGGCCACAGCACCAGGAGAAGCAGAGGGGGAGGCAGAAGGGGAGGCAGTGCTGATGGAGGAAGA
CCTGATCCAGCAGAGCCTGGCGGACTATGACGCCGGCCGCTACAGCCCACGGCTGCTCACGGCACACGAGCTGCCTCTGG
ACGCACACGTGCTGGAGCCTCACGAGGACCTGCAGCGCCTGCAGCTGTCTCGCCAGCAGCTTCAGGCTACAGGTAGGGGC
TGGCCCAGACTGGGGACCTTGTCATGGCGGGGGCAGGGATGGGTGTGGGCAGCCCCAGGTGACCCCTCTGCCCTCACAGG
TGATGCGAGTGAGAGCGCTGAGGACATCTTCTTCCGGCGTGCGAGGGAGGGCATGGGGCAGGATGAGGCACAGTTCAGCG
TGGAGATGCCACTGGGCGGCCCGGGCCTATCTGTGGGCCGACAAGTACCGGCCGCGAAAACCTCGCTTCTTCAACCGCGTG
CACACGGGCTTCGAGTGGAACAAATATAACCAGACGCATTATGACTTCGACAACCCTCCACCCAAGATTGTGCAGGGCTA
CAAATTCAACATCTTCTACCCAGACCTCATCCGTAAGCGCGCCACACCTGAGTACTTCCTGGAGGCTTGTGCAGACAACC
GGGACTTTGCCATTCTGCGCTTCCATGCGGGACCGCCCTACGAGGACATCGCCTTCAAGATCGTCAGTCGTGAGTGGGAG
TATTCGCACCGCCATGGCTTCCGCTGCCAGTTCGCCAATGGAATCTTCCAGCTCTGGTTCCACTTCAAGCGCTACCGCTA
CCGTCGGTGACACCTGTGGGCTGCCTGCCCCTGTCAGGCTGGCCCCTGGACAGCTCCCAGGTGGGAAGGCCAAGAGGACC
AGGAGGCCTCCTTCAGAAGCCGTAGGAATCTGTGGACTTCAGTCACCAGCACTGTGCTGGCCGCAGCCCCTCTGGCAGAT
CCCTGCCTGTCCTGAAAGGATTTTTAACTGTTCTTGGGAAGACTGCTTGGGGTGGCAGAAAGGTCTTGTTTCCATGGACT
TGTTCAACCGGGGAACCCCGTGTGGTTTTGATACATTTAATAGAAAGCTTATTCTAAACTGGCTTCTGATCTGAGTCACA
GGGCAGATACCACACCCAGATGGCTCAGGGAATTGGGGTCAGGACTCAGGGTCTTCTCTAACAAGACCACTGCCGTCTGG
GGAGACATGGCTGTCACCACTGGGGTGGGTCATTGTTTGCACCCAGTGGAGGCCAAGGATACCAAGTGTCACCCATTGGC
```

```
GAGGCTGAGAGGCCACCCTGTCATCCCAGCCCCTAACCCCATGGAATGTAGCAAGACACACACCTGTCCAGATAGGACTA
TACTGCAGCCCAAAAGTAGCCCTGTCCTGTTAGCCCAGGGAGGTGTGACTAATGGCCTCTGCCGTGGACACTCACTCATC
CCCAAAAGTCCCACCAGGACAGAGCTGGGACATCTGAGCTTAGGAGATACTTGTATGCCTTACACAGCACATAGCATGTT
GGGCCTCCGTTTCTTTACCTGGGCTACAAGGGGTCCATCCCCTCCCCCACTACACTATGAAGTCTCCGGCCTGTTGGCTT
CTCCTGTGTGCAGGGTCCCAGCTTGCAAAGAGGCAGAACTGGGGAAGGGGATGGGGGCATGCAGCTAAGGGCATCCTGAG
GTGCCCAGCGTTGGAGAGCAGGCCCCGCCCTCCGGAAGGGGGTCCAGCCATCCCAGGGCACTGAGGAGCCATAGGTGGTG
AGGCGGGCATGGATGCAGACCCTTGCTCAGACCTCTCCCAATGCACACAGGACTCTAAAGTCCCGGAAACTGCCTCCAGC
TCCCACGCCAGTTGTTTCCTTCCTTTGGCTGTGTCCATGGCTAAGAATAGGCCCCATAGCCTGGCCCCCAGCCTCGGCGG
AAGCCACAGCCACAGGGGGTACATCCTGAGCCTCAGCTCCCACACCAACCCAGCATCCTTGAGCCCAACTCCTGTCCTTG
ACTGTGGTTGGGAGCCTTAGCAGGAAACTGCAGCTCCGGGCCTCACCTGATGGTCTTGTGTGGGCTCTACATCACACTAT
GTTTCTGCCTAAGTTTCACCATGCATTCTGAGCTCTGTCACCTCCACACAACAAAGATGAGTGTCCTTCAGCCATTACCT
GATTCTCCAGACCAAACATATCCTTATCTCAGGTCAAGGGACAGAGGAAGAACATACAACCACCTCTCTGTTTCATCCAC
CCCAACCCATCCACATCAAGAGCAGCCTCAGATCCTCTTCCCACCACAGCTCCTCACCCTCACCACCAAACATTCCTGCC
AGGACACCTAGGCAGGGTTTCTCTGTGTAGCCCTGGCTGTCCTGGAACTCACTCTGTAGACCAGGCTGGCCTCGAATTCA
GAAATTCACCAGCCTCTGCCTCCCAAGTGCTGGGGTTAAAGGTGTGTGCCACCACGCCCGGCTTTTTTTTTTTTCTTTTA
AGATTTATTTATTATTATAAATAAGTACACTGTAGCTGTCTTCAGACGCACCAGAAGAGGCCATCAGATCTCATTATGGG
TGGTTATGAGCCACCATGTGGTTGCTGGGAATTGAACTCAGGACCTTCAGAAGAGCAGTCAGTGCTTTTACCGGCTGAGC
CATCTCACCAGCCCCCGAAAAAACCCAATCTTCATACCCCTTACACCCACAGTATTGATACCTAGCCAAATGTTGAGAAG
GTAAAGACATTTGTTTTGCCTCAGCAGCCTGAGATCAGAAACGTGGGATGATCTGTGTCCATTTCTGTCCCTAGCTTATG
GGATGGATAGACAGGGCAGGCCCAGCTAGGAAATCAGAGGAACTACAAACTTCACGGGGGTAGAGGCCAGTCAGGGTCAG
GACCTAGTGTTGGGATGGGTGGGAAGGCAGGAAGGGTGGGGGTGGTGCAGCAGGCAAGGGGCTTCCTGCTGCTGACAAGA
GTCTGGCTGACCCTGGGCAGACTATGGAACTGGGAGTTGAGTCACAGCCAGGCCAGGGCATCTCAAATCCCCACCAGGAC
CTGTCAGGGCCAAAGAGACCCAAGGTCACCTCCTCCACGCAGGCCCCCCTCCTCCAGCTACTAGGCACAGGACTAACACC
CCTGCCTGGCTCAGTCATCCTGGGCAACTGTCAGGGCTTCAGGCAGTGAGTCTGCAGGGGAGGGTTCCCCAAGACTGCTC
CATCTCTAGAGGACAGCCTGGTCTCCGACCTTACACCTCCCCTACCCCAGCCCTCTCCTTCCCCTCCAGCTCTTGGTCCC
TGCAGTCAGGCTGGAGCTGGCCTTGCTGACCCAATATCCCTGGAAGGCTCCTGGACAGCCTTGGCGAAGGTTAGTTGCTG
CTGTGTCAGGACCAGCCCCATTTGGCCTCCCAGCTCTTCCCACATGGCCTCCCTCTGGGTCCCTGACCTTTCAACAACTG
ATGTCTCTCTCTACTCTGTCACCCTTGTGCCCTGCTTGGTTCCCACCCTAGCGGCTGAGAGGCGAGGCCAGCGCCAGCGC
AGGGGGTGGGGCTGACGAGAGTCGGCAGAAAGACAGATGGAGACAGGGCGGGACGGGAAACAGCCTCACAGTCCCATCCC
TCCAGCCCTGGACGCCTGAGGGTCTCCAGTGCTCCCCTGTACCCACGGCCCCAGGGCTTCTGTTGGGGCCTCCCCTCAAC
CAACGTGACTTGGTTTCCCCAGGGAACTCCCCTACCTGCTCTCTCTGCCTGGCCTGGAACACCACAGAGGACACCTAGGT
ACAAACAGCACAGGTGAGTCCCTTGCCTCCCTACTGTGACTATACCACTGTACCCAGGTAAGCCTCTGCATAGATCCTGG
GGAACCCAGATCCACTAACAACCCTGCTGTGGCCTGCCTACAGGGGGGAATGATAGCAACCAGGTGCCAAAGTTCCCTGA
AGGGGATCCTGTGGTGTGACATTTACTCCGGTGGTCTAGCCTGGGCAAGTGCAGTTTCCCTGGTGGTACGTGGGTACACT
GGTTTCCTGTGTCTGGGTGAGGCCAGTCCAAGATGCTGGCAGTGATGAAGTTGGGGGTGGGGATGGAATGGAGCTGGGA
GCTACAGTGTGTGGCTTGGAGCTGGGCATGGTGATTGTACTCACATAGGCTCAGCTCAGAACCTGGGTCACATCTCGATG
GCACATGTCCTACCCAGAGAGGGGCATCCATCCAACATGCCAGAAAACAGGCCTCGGGGCCCCTCCCCCAGCCCACTGCC
ACAGGATGACACCTGCACCCTGGAACTGTGGCACAAACAGGAAGCAGGCAGTGGGGGGAACAGCATGCGGTGAAGGTGAA
CCTGAGTCTGTGGGGTTGTGGGCATGCACACTGGGTATTTGGGGTGCTGGCAGAACAGCCCACTGGGCACAGCTGCCGCCC
AAGGCTCAGCCTCAGGAGCTCATAAGCAGAAAGTCACCAGGGAAGGCAAATTCGTTACACGGGGAAAACTGAGGCTGTGT
AGCGTGGCTATGGGTGATGGAGGTGTGGGGAGCCCAGGCTGCTCTGAGGCCTGGTGGGGGAGGCTTGTTGCAGATGGAGG
AGTTGGGAGAAGGAGGAGGGGCCTAGCCTGCAGAGCCCAGCCACACACCACATTCCACCAAGGCTGGAACTCAGCCACCC
AGCCTGGGCCTCAAGGAATGAGCTCTAGCGTCCCAGGGCACACATATACAAGGATGAGGCCCCATCCTAGACAGAATACC
ACTGCCTTCCACCCCCAACGGTGGGAACATGGCTCAGGAACGCAGGACCATCCCACATCCCAGGGACTCAGGGTGGGAGG
GTTCCAGAATCCTTCTGCTTCCTGGTGTCCAAAGCTGGGGTGTGGTGGCCTGTGCCTGTCACCCCAACAGAAGCTGGAGG
CAGATGGAGCTCAGTGAGTTCCAGGCCAGCCTGGTCTACACAGAGTCTGCATGAGGACAAACAGAATTCCTGAGCCTTGA
AATTCCCAGGCCCAAAGCTCCCTGCTCCAAAGCTGTGGAACCTGATGGGGCGCAGAGACGCCAAGTAAGTCTCCTGAGAC
CACACAGCATGGGCTGCTGGCCAGCGTGGGAGGCTGCCAGGTCTGCCTCAAGTCTCGCCTAGGCAACTTGAGCTCACCAT
TATCACTTGAACCCCAGGGGAGGAAGGAAGGGGCTGGAAAAGCCATCCCTCCTTGATGCTTGTCTCCAAAGAGCTGGCTT
GTGAGGGACCTGGTGGCCTTGGTGAGGCTGGGTCAAGGACTGGACAGAGCAGGGGGCTGACCACCAATGACGCCCACCTT
GAGAAAGGGAGCATATGAGTCCTGGCACTGAAACTCAGAGATAGGCAGAGCACCTCAGAGGCAGGGCTATGCCATCCACC
AGGCCGCCCACCCTGACCTCATCCCTGAGCCTCAGGTTCATCCCTGGATCTTCCTCTGGATGGTGCAGGCCCAGCCAGGC
AGTGGTCTGTAGTAAGGCCTCCTAAGGGGATCGTGGAAGCAGCACCTACTAGGCTGCTTATACCTGCATGGAGCATTCTG
GGTTGCATAAATTAAACACCTACTGTGTGCCAGATGTAGGTGCCAGAAATAATTGTTCTCAGCATCAGCTGTCTTTACCA
GAAGAAAAACTGAGGCTCTCTGTGGGGATCTGGGGGCAAGAACAGATGATGCATCCCAAACCTTGAGTGGCCAACTGTTA
AAACCCTGGAGCTCAATGGGAGGGAAGAAGGGTCTGGAGGTGGGGAGGGAGAAAGAGAAAGCCAAGGGCTAGCTATGTGG
CTCAGTGGGTAAAGGTGATTGCAGCCAAGCCTGACAGTCTGAGTTCAGTCTCCCAAGACCCACGTGGTGAAAGGTGAGAA
CTAATACCCACTAAGTGTCCTCTGACACACACACACACACACACACACACACACACACACTGGACTGTGCATGTGC
CCTTGCACACCCCCACACGTAAATGAATGATGTGAATTAAAAACAAAACAACAACAACAAAAAAAAAAAAAAAAAAAAAC
AAAAAATTAAGAAAAAAGGGCAAACATGGTTCACACCAAGAATCTGAGGCAGGGAGGATCAGCATGAATTGCCAGCCTGG
GCTGCACAGGAACACTGGGTGACTGTCAGACTTAGGCTACTGCCTGTCTCTGGTCTCTGGCCTTGGACTTGTCTGGTTTT
```

```
CTTTCCTTTTCTTTCCTTTTCTTTTCTTTTCTTTTCTTTTCTCTTTTCTTTCCTTTCCTTTTCTTTCCTTTCCTTTTTTT
TCTTTTCCTTTCTTTTCTCTCTCNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNGGGTCCTTCCGTCCTTCCTGTCCTG
TCCTTCCTTCCTTTTTTAAAATCTCCCTGTGTACCTTAAATTACTGCCTCAGCCTTCTGGGTGTTGCAAAGGATTTTGTT
GTTGAACAGTCTCACTTTTTACGCAACCTCGGTTCTGTGATGTTTGTCTCTGCATCTCCCCGAAAACCCCCGCCTGTCTC
ACACCAATCTCTGCTGGAATCCCCTCTCAGCCGCCCTGCTCCCCCAACCCCCCCAGCCCCACACCGGTCTCTGCTGGAGT
CCTTCCCGGGGACATGCCATCAGGCTGTTTTTTCATTCCCAAAATGGAGCAGGAACTTCTGGGTCCGTTTCTTATTATAA
AAATGACGTCAGAGGAGAGGAGGAACAGCCCACACCCACACCCATCCAGTGGGAGTGTCCGGGCAGGTACCAAACCCTGG
CCATAACGAGTGTCCCCACAACCTCACAGGCTAGATGGATGACAGATAGGGAAACTGAGGCCACACAGTGAGTACAAGGA
CTGGAACTCCAACCTGGAGTGCAGGGTGTCAGAACACATCTTTGTCATTTCAGCTCCTCAGAAGGTTGAGGCAGGACTAC
TTAAGAGTTCCAGGCTGAGCTGGGCGTGGTGTCGCACGCCTTTAATCCCAGCAACTCGGGAGGCAGAGGCAGGTGGATTT
CTGAGTTCGAGGCCAGCTTGGTCTACAAAGTGAGTTCCAGGACAGCTAGGGCTATAGAGAAACCCTGTCTCAAAAAAAAA
AAAAAAAAAAAAGAGTTCCAGGCTGGCTGGGCCACTTCATGGGGTCAGAAGCAGTCGCAGGACCAACAGTGATCCGGCA
GTTGAAGGCTGCCTCAAAGAAAGGGTATTTTGTTCCTGAGGCATTGAAGTCTCTGTAGGAGTTCTCCATAGGCGCTCGAG
TCCCACTGAGCAGCCCACCTTGTCTTCCAGGTGCGCTGGAATCAGCGCCAGCCCAACGTCCCTGCCATTGGTGTCTGATG
GGGTGCTGACCCCCTCGCCGCGTCAGGCTTAGGAGCCATGTGGCCCAATGGCACCTCGCTGGGGGCCTGCTTTCGCCCGG
TGAACATCACGCTGCAGGAGCGACGTGCCATCGCGTCGCCATGGTTCGCTGCGTCCTTTTGCGCGCTGGGCCTGGGGTCC
AACCTGCTGGCACTGAGTGTGCTGGCTGGGGCGCGGCCCGGCGCGGGACCCCGCTCCTCCTTCCTCGCTCTGCTGTGCGG
CCTTGTTCTCACCGACTTCCTGGGGCTGCTAGTCACCGGCGCCATCGTGGCGTCGCAACACGCTGCCTTGTTGGACTGGC
GAGCCACTGACCCCAGCTGCCGCCTGTGCTACTTCATGGGGGTAGCTATGGTGTTCTTCGGGCTGTGTCCCTGCTCCTG
GGGGCCGCCATGGCCTCTGAGCGCTTCGTGGGCATCACACGGCCCTTCTCACGACCCACGGCCACATCGCGCCGCGCCTG
GGCTACTGTCGGGCTGGTGTGGGTGGCGGCCGGGGCACTCGGGCTGCTGCCACTACTGGGGCTTGGCCGCTACAGTGTGC
AGTACCCCGGCTCTTGGTGCTTCCTGACACTCGGGACACAGCGCGGTGACGTGGTCTTCGGGCTCATATTCGCACTCCTG
GGCAGCGCGTCCGTGGGGCTGTCCCTCCTGCTCAACACCGTTAGTGTGGCCACACTCTGCCGGGTCTACCACACGCGCGA
GGCCACACAGCGCCCACGGGACTGCGAGGTGGAGATGATGGTTCAGCTCGTGGGCATCATGGTGGTGGCCACGGTGTGTT
GGATGCCCTTGCTGGTGAGTCGTCCCCGCCCTGCCCCGCCATCCCTCCCCCACGCCCCCCAGCAACATGAGGACCTTC
TTTAGTTGTATCTCAGGAGAAGGGCAATCCTGGGGTTCAGGTCTTTGGGGCTTTAGGAATCTGCAGGATGTAACAGGAAA
GACTTTCAGGTGTTGTGTGTTCTAAAGTAGGGCTTTGAAGGATGAATAGGAGTTCACCTGCTAGACAATGTTGGGGAAAG
CATTCTGGCCGGAATGGCTGGATAGTCCACTCAATATTGCTGTGTTCCCTGGCACCACATAGACTCCAGGCTTGTATAGA
TGTCCTCCTGGGAGGTTAGAGCCCAGCTGATAGGACTTTAACGTGGGCACAGGTTTTTGTTTTGTCTTTGCCTTGTTCCG
CCAGCAAACATTTATTGTGTACCATCTGTATCCAGGCTCCCCTAGCACAAAGGCGACTTTCCTCTGGGAGGAGGAGGTG
TGTGGTTCCCAGGCTCCAAGGTCTGTCCCAAGCTGCTGGGGCAATTTGACTAGACCCTATCTAAAATAAAATGTAGCCAG
GCGTGGTGGCGCATGCCTTTAATCCCAGCACTTGGGAGGCAGAGGCAGGCAGATTTCTGAGTTCGAGGCCATCCTGGTCT
ACAAAGTGAGTTCCAGGACAGCCAGGGCTATACAGAGAAACCCTGTCTCGAAAAAACAAATAATAATAATAATAAATGTA
AACCGGCATGGGGGGCACACACCTTTAATCCCAGCACTCGGGAGGCAGGGGCAGGCAGATCTCTGTGAGTTTAAGGCCA
GGCTGGTCTACACAGTAATATCCTGTCTCAAGAAACAAAAGAATCAATAAGAAGGAGCAGTTGTTGGAGACTGGCTTAGG
GTAGATTCCTGCCTCAGTAAGTCCAAGTTTGCTTTTTTTGTTTTGTTTTGTTTTTTTGAGACAAGGTTTCTCTGTGTAGT
CCTGGCTATCCTGGAACTTACTCTATAGACCAGGCTGGCCTCAAACTCAGAAATTGGCCTGCCTCTGCCTCCCAAGTGCT
GGGATTAAAGGCGTGCGCCACTATGTCCGGCTCGTCCAAGTTTGCTTTCTAAACCTCAGTCCAAGTAGGACGTGATGAGC
TCAAAGCCTGTGCTCATTCCTGGACCCAGCTGTAGCAGCAGCTCAGAGGTTGCCCCACTGCTCCAGGGTCACACAGTACA
ACCCTCTGCTCCTGCAGGTCTTCATCATGCAGACTTTGTTGCAGACACCACCTGTCATGAGCTTCTCTGGGCAGCTGTTG
CGGGCCACAGAGCACCAGCTGCTCATCTACCTGCGTGTGGCCACCTGGAACCAGATCTTGGACCCCTGGGTCTATATCCT
CTTTCGCCGGTCTGTGCTCCGGCGCCTGCATCCGCGGTTCAGTTCACAGCTCCAGGCTGTGTCCTTGCGCCGGCCTCCAG
CCCAGGCCATGCTCAGTGGACCCTGAAGGACAGTGCCACTTAAACCTGGGGTCCTTGGTGACTGCCATTGACCTGTGCCC
TGCCCCAGGAGCCTGAGTGCTTGGTGGACTAAGGACGGAGCTACGACATGGGGCTGACTCCTGTGAACTGCAAAGATACC
TCCTCTATGCTAGAGAGGTCCTCCCTGACCCTCTCCCCGGACTACTCCCTGTCAGCCAGGAGGCTGGGGGCACATGTGGG
AGGCCCCCTGGCAGGAATCTAGGATTCCACAGGGAAAACCTGGGATGCTGGAGGTGTCTTCCCCCTTGCCAGCATCTGAG
TCTGAGACACAGGGCTTCACATTCCTCCAGGCTCACAGGTCACCAGCCTTGGGTGTTGGAAGCACCCACTCTGTTCCCTG
TAGCTGGGTTCACCAACCTCTCCAGTGTTTCCTGAGCCCCCTTGCTCCATGGCTGCCTAACACACATGCTCAATAAATGG
TTGAATGTCCAGATTCTGGAGCACTCCCCTAAGCATCCCTGTGTCCATTCGCCTCCCTCCCCCCGCAGCTCCAAGAAAACC
ACAGTCAAGAGGAGACTGGCGGCTGTGGAGGTGCGGGCTCCTTTATTGCCCACCAAGATCTCCTAGGAGCCTCCCACCCC
TATGCCTTCTATGGCTGGGTCCTCTTACAGGGGCTTTGGGGTTCGTAGCCAGGTCCCTGAAACCCAAGCTTCAGTTTCTA
CCATCCTGTAATCCTGCTGGGATGAGTCAAGTCACAGGCTGGGGTGGGGGAGGGACCCAGACCCAGGGGAGGGGCTAGGA
GTGATGCATATGGTGGGGGGGTTGGGCTTCTGGCATCCCAGCTGGGGCTTGGGCAAAAGGGCTGGCTTCTGGGGAGTGTC
TGGGCCTGGTGACCCAGGGCCCAGGAGAGAGAGTGAGGGGCCTGGCCTGGGGCCGCTGTGGCCTGGGTTCCATCTTGAG
GCCAAGGCCCAGCGACCTTGCTCGGGGCAGGGGTGGGGGCTCTTGGGGTCAGTGGGCTTGGAGACTGAGGTCGGGTCAGG
GGCTTAGGCTCCTTTATAGGCTGAAGGCCAGGGGTCTGTGGCGGCGACTGTCTCTAAAGCTGAGGGGGCGGGCTACTGT
GCTGGGCTGACTTCCAGAAGTGTCGGGGCTGGGGCTGGGGCTGGGGCTGGGGCTGGGGCTGGGGCTGGGGCTGGGGCTGG
GGCTGGGGCTGGGGCTGGGGCTGGGGCTGGGGCTGGGGCTGGAGTCTAGCCGGGTTCTGGAACTGGAATCCTTCCCAGCG
CTGGGCTGGGCTCCAGTGCGTGATGGAACCTGAAGACTGACTTGGGTTCTCGAGGCTGAGTGGGCTGCAGAGCCAAGCCA
CTGCCCTGTGTTCTGCTGGGGGACACAGCTGGCTCCAGTTGTCACCGAGTGCTGTTTTCTGCCTCCAGAACTAGAGAGGG
```

```
ACCTGGAGCTGGGCTCCTTCTCTGTCAATGACTGTAGCTGGGACCTTCGATGTCTGGCCCAACCTACGTAGGCTCCGGAG
CTAGGCCAGGCCTGGAAGTCGGTCTGGGTCCCAGAGTCGGACTGTGTCCTGGAGCCTGCATGCCATCTTCTGGGTGGAGT
GGGGTCAGCACTCTGAGACAACCCTAAGCTGGGCCTCGATTTGGGGAGTGCATGGCATTTGGGGGTGGGGCTCTGCTGTCA
GAGCCTTCCTGCTCCTGCCCCTGGGCTGAGCCTCAGAGTCAGACTCATCACTGGGGCTAGCTTGGGGTTCAGGGCTTTGG
GAGGCACTGGTAGGTAGCTGTGCTGGGGAACAAGGCTGAGGTTTGAGTTTGGACTGGGTAGCAGTATGGCTCTGAGTGCC
TGCACTAGGCAGGGGTGTGGAAGATGGCTGGCTACTGGAGCCTACTTGGGTTTCATCAGTCAGCGGCTCGCTGGACCTTG
CTTGGGGTCCCAAGCAAGCCCTTCTTCCTAAACCACTCCAAGTCTCAGGGAAGGGCCAGGCCATGGTGGTGGCTGTCTCA
TACCTGGGCTGGCTCTCAGAACCTGGCTTCTGAGACTGGCTGCTGGGGTGGTGCTCTGAGCCCAGCTGTCTCCTAACATT
TGTCTGGATTCCGGAGTGTGGCTGTGTGGGACGGGGCTTGGCTGCATTCTGGAAGGCTGAGGGTCAGCCATGACCTGGGCTG
CAGCATCAGGGTTGGTCCCAATGTCTGGCTGGGCTCTAGAGCATGGTTTCCTATCTGGGTGGATCTGTGTTCTAGAACCA
AGCCCACTCTCAGAGCCTGACTGTGTTCTGGAAGTGGACTTGGTCTCCAAGCAGAAGTTGACCTGAGGGCTGGCTTGGGT
TCTAAGTCGGGCATGAGTGTCAGAATTTCCCTGGGCTCCAGAATGGGGTGTGGCCTCAAAGCCTGGCTGGACCTCAGAGC
CAGGCTGGGTTCTAGGGATGGGTTGGCAGTCAGGTTCAAGATGAGTTTTAGAACAAGGCTCAGGGCTGGGGCTGCTCTGG
GTTCCAAAGCCAGCCTGAGCCTCGGAGCTGACCTGTGTTCTGGAGTCAGACTGTACCCCAGGACTAGGCTGGGATCTAAA
GGCTGGCTGGACTTCAGAAACAAGGGTTACAGAACCAAGCTGGGCCTTGGGGTGGGCCTGGGTCACAGAACTGGGTTGGG
TTCTAGAGTTATCCTGGGCTTCAATTTTGGTTTTAGAGCAGGACTGGAATTCAGGGCCGACCAGGGATTCCAGCTGGGTC
TCAGAGCTGAGCTGGGTGCTAGAATCAGGCCAGGGCCTTGGGATGGGCCTGGGTCACAGAACTGGCTGGCTCGGGTTCTAGAGCT
GGGCAAGACATCAACTTTGGTTCTAGAGCAGGAGCTGGCCTTGGGGCTAACCTGGGTTCCAGAACCAGCCTGGAATTCTG
ACTGGGTGCTAGAACCAAGCTGAGTCTCAGGGTGGGCCTGGGTCAAAGAACTGGGTCGGGTTCTAGAGCTGGGCTGGACT
TCAGTATTGGTTCTAGAGCTGGTCTGGCCCTCGGAGCTAACCTGGGTTCCAGAACAGGGATGGGGTTCTGGCTGGCTCTT
AGAGCTGAACTGGGTGCTAGAACCAGGCTGGGCCTCAGGGTGGACCTGGGTCTCAGAACTGGGTTGGGTTCTGGTGGCGG
GCCTTGTCTCAGGGCTGTCCTGGGATCTGGAGTGGCGTTGGGGCAAGGGGTCTGGGCCCAGGACTGGGAGACTCCCACACAC
GCTAGCCACAAGCGTCGTGGGCCTCGCCGATAGCTGCCCACACCTCCACCACGAACTCGTCTGGAAAGGCGAACTCGCCC
AGAACGGCCGTCCAGGCCGCGTGCGAAAGCCTGGGCTCCCGCCGAGCTCCGAGCAGTCTCCACCACCGCGGCCGCTGCGGG
ACCAGACGACAGAGGGACAGGATGCACAAGCAAAAGAGATGGAAGGGCAGACGGAAGGACAGGGAGGACAGACAGACTG
ACTGTGAGCTCCGGCGCATGCTTCCACCTACCACCCTCCCGCGCCGGGGACCCCGCGCTTACCCGCGCCGCTTACCCAGC
TCGGGGTCTCGGATGCCCATGTAGCTCTCCAGCAGGTCGTCCACCCTGCGCGCCGCTGCCGCTTCCACATCGCTGGGCTG
CGGATAGGTGAATGGCCCAGTGGATGAGTGACAGGCTGGAGGGCCCGCCCCAAGATTGATAGACTATTAGTGGTTCCTAG
GGGTAGGGGGTGCGGAACCCCGCTTCTGAGAATTGACAGAGTTGTGGTTGCTGGAGGTGACGCCTTCCCGATTGATAGTG
GGGAGGGCCTGGGTGACTCGCCCCCAGGAGTGATAACTAAAGGGATAGGAGACACTTTCTTCAGTGGTGTAGTGGATGGT
CTGTGCCCTGACACAAACCATGTGTCATTATGAAGAGGTGAGAGGAGGAGACAGAGGTCTGGGTGGAAGAGGTAAGGAGA
TGAGAGATTGGGTGATGGATGTACCTGAACACACTTGAAGAATCAGACAAGACACACATTACCTAGTCCAGTTAATACAC
AGAATTCCACCTCTCAAGGCGCTCCGAGCGCTTCGTGAGACTCTCGTCTCACAGGCATGAGGAACTGGGTTTGAGCTCTA
GAAGGCTCTAAAGGTGGAAGTGAGAACTGCTCCCCACATGTCCTCTGACCTCCACCCTCCTGTCCACGCACTTTCCAAAC
ACGTGCACACACAAAGTACACAAGCAAAATATAATAAAACAATAAAGAAATGCCATTTAAAGATTTAAAAGCTGGGCATG
GTGGCGCACGCCTTTAATCCCAGCACTCGGGAGGCAGAGGCAGGCGGATTTCTGAGTTCCAGGCCAGCTTGGTCTACAGA
GAGAGTTCCAGGACAGCCAGCGCTATAACAGAAAAATCCTGTCTCAAAAAACAAAACAAAGATTTAAAGTTGGAGCTGG
ACATAGTTGCACAGCACACCTTTAATCCCATCACTTGGGAGGCAGAGGCAGGTGGATTTCTGAGTTCAAGGCCAGCCTAG
TCTACAAAGTGAGTTCCAGGACAGCCAGGGCTACACAGAGAAACACTCTCTCGAAAAAACAAAAATTAAAAAAAATGATAA
TAAATTAAAATAATAAAATAAAAGAAATAAAAAAAGATTTAGGGCTGGTGAGATGGCTCAGTGGGTAAGAGTACCCGACT
GCTCTTCCGAAGGTCAGGAGTTCAAATCCCAGCAACCACATGGTGGCTCACAACCATCCGTAACGAGATCTGACTCCCTC
TTCTGGAGTGTCTGAGGACAGCTACAGTGTACTTACACATAATAAATAAATAAATAAATAAATAAATAAATAATTCTTAT
TAAAAAAAAAAAGATTTAAAGTTGGGCTGGAGAGATGGCTCAGTGGTTAAGAGCACTGGCTGCTCTTCTGAAGGTCCTGA
GTTCAAATCCCAGCAACCACATGGTGGTGGCTCACAGCCATTCGTAATGAGATCTGACGCCCTCTTCTGGTGCGTGTGAA
GACAGCTACAGTGTACTTACATATAATAATAAATAAATCTTTAAAAAAAATTAAAGTTGAAGCCGGCAGTGGTGGTACAC
ACCTTTAGTCCCAGCATTTGGGAGGCAGAGGCAGGCGGGTTTCTGAGTTCGAGGCCAGCCTGGTCTACAGAGTGAGTTCC
AGGACAGCCAGGGCTACACAGAGAAACCCTGTCTTGAAAAACAAAACAAAACAAAACAAAACAAAACAAAACAAACAAA
AATTAAAGTTGGGGAGTAAGAAAGACACAGATATTGACCTTTGACCTCTGTATGCATACACACAGTGCAAAAAAATAACC
AATAATAATAAGTTAGAGGTCACGATGGTCCCTCCCGCTCCTTCGCCAGGAACAGGTGGCACCCATTGGACTCTGTGATC
GGAGCTGTTCCTGTTGTCCTATTCTCCAACCGGTACCCACTGCACAAGCTGAAAAACTGCGGCTCTGAGAACAGGGTCTA
TTCATAGGTTCAGGCTAGCCTTGAACTTGTGACCATCCTCCTGCCTCATCCTCCTGAATGCTGGGATGACCAGCCTTATC
ACCATTAGCACTGTGGGCTTGACAAGAGGGTGTCAATTGCTGCCATCCCAACAGTCACTCACCGCCTCCTCCACAGTGGC
AGCCCCCCCTGAGCGCAGGCGTAATGTTTCCCTCCCGCTGCTCACTTTAGCTTCCACAGGACACTTGCTGCTCCTGCTCC
TCTGGCCAATCATATCTGAGGGGGGCAAAGCGGTGACAGATGTGTCCTGTTTTGTGTGGACTGTTTTTCATGTTTATTTTC
TTTTGTCAATAGGGTCTTCTTCTGTGACCCAGGCTGACCTTGAACTCAAGGTTGGCATGCCTTAATCTCCTGAGGGCTGT
TGAAGGACTATGGGGCTACGGGTGTGGTCAGGGGGTAAAGTCCCATCTAAAACCCCCCAGTGAGGGACTTGGGGCGTGGT
CAGGGTGGAGCCCCTCCTAGAATCCTCCAATGAGGAGATAGGGGCGTGGTCAGGCATAGAGCCCCCACCTAGAGTCCCCT
ATTGATGGGTTGTGGGTGTGGTCAGGGTGGAGCTCCGCCTAGAACTTCTGTGAAGGAGCTGCGGGCGGGCGTGGTGGTGG
AGGTTCAGGTCCTCCCCTCCCTTAGGATCCTCCAGTGAAGGGCTTGGGGTGTGGTCAGGGTTGGAGTCCTGGAGGTTTAT
ATTATTCTTTCCCTAAGAGACCCTGCTGAGTGATGTCTGGGGACATGTCTGGCTATCCTCCCCTGAGGAGCTCCTGACAA
AGAGTAAGGAACACTGCTCAGCACCCTGAAGAGCCCGGGACACTACACAGAAAGCACCCCAAAACGGTGCGGTGCTCACA
```

```
TCGCCAAGTTTGAATAACTTTTCTAGAAGCAACCCTCCTTTCTCGAAGTCATTGCAAAGCAGGCCCAAGGTCATCTTTGG
GTACATATTGAGTTTGATGGCAGCTTGGGCTATGAAAATATGAGTCAGAATACCATTTCCTGCTTCTCATTGCCCTGGGG
CAAGCCTGGGGCCCATCAATCCTTACCAAAAGCTCTCCGGGGCTGCACTAGACGCAGGGTGAAGGGCTGAGACTTGGGCA
GCTCCCGGAGCATTTTAGCCACCTCGTAGTGCCGGCAGCCCACAATAGAGTGGTCATTGATGGCCTCAATGCTATCCCCC
ACGCACACAGCCTCAATCCGGTTGATAATGCTGCCTTCCTTGATTCTCTATGGGAAATGACAATCACTCGTGACTAGTCT
GCCCGGGGCCACCCTCCCTGGGGTCCCCAGAGTCTTCTTAGGACTCGACAAGGCCATTGATCACCCAGTTCACACACACC
CACAGGCACCTTGATGAAGGCGTATCCGGCTCCGTTGTCTGTGATGGTCAGTCCTAGAGCATCCTCCGTTTTGGTGACCT
CCACTTCTTTGGTCTCTCCACGCACATGTGCAAAGATGAAGTCTTCCAGCCCAATCTGTCCCCCCAGCAACTTTTGCATG
TCCACTTTGTGACTGTTGAGGGTGCAGAATAGGATCTGTTCGGATAAGAGAGGGACCCAGATTGAGCCATGGACAAAAGT
CGATTCTGTGCACATCCTTCTGCGGGCGCCCCCTGGAGGCCAGATGCACCCGTGGCCCGCAGGAAACAGCAGCTGCCCCC
AGCCTCTAACTGCTCACCGGTGACCAAGTGGGCTGGCAAGACTGTGGGTGTGCTTTAGGTCTGTCTGTTCCTAAGCAGGA
ACTTAGGAAGCTGAGGCAGAAGGATGACTGAGAGTCTCAGGCTTCCCAAAAATAAAAAATAAAAAAGCCAAGCATCCTGA
ATCGGGAGTGTAAGCCCAGAAGTCGGGAAGCCAAGGCAAGGGAAATGCTGGAAGTTAGAAGCCAGCTTGGGTTACAGAAT
GAATTTCAGGCCAGCCTGAGCTACAAAGCGAAATATTATCTTAATTAGACGAACAAGCAATGGAATGAAAACGGACAAGA
GGGCTAGAGCTCAGCGGTTAAGAGCACCATCTGCTTTTCTGAAGGTCCTGAGTTCAAATCCCAGCAACCACATGGTGGCT
CACAACCATCTGTAACAAGATCTGATGCCCTCTTCTAAAGTGTCTGAGACAGCTACACAGTGTCCTTACGTATAATAAAT
AACTAAATCTTTAAAAAAAGAAAAGAAAAGAAAATGGACAAGAAGGCTGGAGTCAGGAGGGAGGGAGGGAGTGGGAGGGG
CTTGTGGCCAGTCTGCCAAGTCCTGAACCTGTTTGATTCTCAAGGGCTGCATGTGGAAGGAGAGTACTAACCCCTTGCAA
ACTGTTGCCCAACCTCCACCGTGGTGCCCACTCTGGTGCCCCTACATATAATAAATCAAACCCACACTAAGTGGGTGGCC
CACACCTTTAATCCCAGCTCTCTAGAGGCTGCAGCAGGTAGATTTCTGTGAATTTGAAATCAGACTGGTCTACAGAGCTA
GTTCCAGACCGGCCAGGGCTACATGATGAGACTGCCTGTCTTAGAAAAACAAATTAAAAGAAAGAAACAAACAACTGA
AAGGGAATAGTAGAAATAATTGAGTTGAAAAAAATTAAAAGTGATTTTATTTTATATAAGGCCTCATGTAGCTCAGGCTG
ACCCTCTAGTTTCCTATGTATCTGAGGATGACCAGCTTAACTCTCAATCCTCCTGCCTCCACCTTCCCAGAATCGGAATA
ACAGACTTTACATCACTACACCTGCTTTATCCTGTGTCGGGGATGGAAACTGGCTTTCCAAACCCTAGGTGACTGCTCTA
CCCACTGAGCTGATTGCCACCCATTAGCCATTCATGGGCTCCTGACACCTGGGTCGGGTCCTCGGCGGCTATGGGAACCC
CACTCCCAGGGTCTGATCCTGTCTTTCGCGTGGAGTGCAGACAGGATCCTGGGTGGTGTCCGCGGTCCACCCTCACCTCG
GTAGGAGCGATGCCGAAGGCCTCGGCGATCTTGGCGTAAAGCTCGCGGACATTGGTGAAGCCCTCGATCCTGCCGGTCGG
ACTGCCGTGCGCCAGCTGCGTGCGGAACACTAGGCGCGGGCGCGCGGGCGGGGGGCTCGGTGGCCCCGGGCTCGCGGG
GCGCAGCACTGTCCATGGTGCGTGGGGTCCGCCGCGTCCTGAGCCTCCCGCCGGAGGTCCCGCGACAGCGGAGCCGGGCG
TAAGCGGATCCGGGAACCCGAGCCGCCCGGGGGAAGGTGAAACCGCCCGCCGGGCCAGCCGCGCCCTCCCCCGGGACTC
GCGAACAACAGGGGGAGGCCCCGGGCTGTGGGTGCTGCGTCCAGTCTTGGCCTCCCGGGGAGCCCCGCCCGTCCTCCCCT
TCTCCCTCCTCCGCTCCGCACACAGCCCACCGTCCATGTCAGCGGTCTTCCCGCAGCCCCCGACCCTGTCTGTGAACTCC
GTGCCTTGTCCCTCTGTCTATCTGGCCTCGCGTGCCTCCTTCCTTGCCCTACCTCAGGGTCCACCCTGCCTCAGGGCTCT
TGCACAAGCTGGGCTCTCCTCTGGGGCTCAGTCCCCAGAGCTCCCAGTGAGTTCCTGCTCACCTGTTTATTTTAAAGACC
ACCTGCGGCCCCTACCTCCAAATCTCTCCCTTAATAATTTTTGTTTTGTTTTTTGAGACAGAATCTCATGTAGCCCAGGTT
GGCCTGGAACTCACTATGTACCCAAGGGTGACCCTAACCTCCTCCTGATCCTCTGGCGTCAGACTCCCCAATGATGGAAT
TAAAGTTGTGTGCACTACCCCTGGAAAAATAATTGTCGATAGCCAGGCAGTGAGAGTGCACACCTTTAGTCCAAGCACTG
GGAAGGTAGAGGCAGGTGGCTCTCTGAGTTCAAGGCTAGCCTGGTCTACAGAGCGAGTTCCAGGACAGCTAGGGCTATAC
AGAGAATCCTCTCTCAACTCAGCCCACTCTCCTGGCAAAAAGAATTTTTGTTAAGAAATGAAGACCAGGGCTGGAGAGAT
GGCCCAGCGGTTAAGAGCACTGACTGCTCTTCCAGAGGTCCTGAGTTCAATTCCCAGCAATCGCATGGTGGCTCACAACC
ATCTGTAATGAGATCTGACGCCCTCTTCTGGTGTGTTTCTCTGAAGAGAGCAACAGTGTACTCACATATGTAAAATAAAT
AAATAAATAAATAAATAAATAAATCTAGAAAGAAAGAAAGAAAGAAAGAAAGAAAGAAAGAAAGAAAGAAAGAAAG
AAAGAAAGAAAGAAAGGAAGGAAGGATGACCAAACCACTATAAAATGCTACTTTTTCAGGGATCCCCAGGTATGTGGTGG
GTGAAGTGGGGTGTGCAGGGGTCAATGAACATCTGAGGCTGTTTGTGTCTTCTAGATTCCCACGACAGAGTGTCACAACG
TAGGTGACTTAAAAGAGGACCAACAACGGAAACTGGAGGGATGGTTCAGTGGTTAAAAGCACTGGCCAGTAACTCACCTC
CAACTATAATTGGATCTGATGCCCTCTTCTGGCATGCAGGTGTACATACAGAGAGAGTATTCAGAGAGAGAGAGAGATCT
GGGCATAAGGACACCCCTACCTGGGGACGTGGAGGATCAAGGTCATCCTTGACACACAGTGAATTCCAGGCTAGCCTTGG
CTACATGAGACATTGTCACAAAACAAAACTCAGATGAACTTAATCCTTCCTCAGTTGATAAGTGGAGGTGCTCCCCTCTC
TCCCGGGGCTGTGGGAGGCTGTCTTCGACTCTGGCAGTCCTTGACTGGCCTTGGCCCACAGGCCTGTTGTGCAGTTTCTG
CCTCTGTTGTCACTAAGCCCGCTCCTAGGCCTGTGTTAACTTTCCTGTTCTCAAAATGATACTCGCTGCTGGCATAGTGC
CCACTCTACCCCAACATGACCTCACTCAAGTTAATCTCACTGTTTTCCTCCTGATTCCCAATAAGGCCATGCCTGGGGGT
TCTGGGGTGAGGACAGGAGCACATTTGGGGGGATACAATTCCTTGGCACAGCATATGTGTGCTGGATTGGGAAGTTTTAC
AAGATATTCT
```

MOUSE mRNA SEQUENCE : mR6-034.1 (Seq ID No: 56)

```
CAACGTGACTTGGTTTCCCCAGGGAACTCCCCTACCTGCTCTCTCTGCCTGGCCTGGAACACCACAGAGGACACCTAGGT
ACAAACAGCACAGGTGCGCTGGAATCAGCGCCCAGCCCAACGTCCCTGCCATTGGTGTCTGATGGGGTGCTGACCCCCTCG
CCGCGTCAGGCTTAGGAGCCATGTGGCCCAATGGCACCTCGCTGGGGGCCTGCTTTCGCCCGGTGAACATCACGCTGCAG
GAGCGACGTGCCATCGCGTCGCCATGGTTCGCTGCGTCCTTTTGCGCGCTGGGCCTGGGGTCCAACCTGCTGGCACTGAG
TGTGCTGGCTGGGGCGCGGCCCGGCGCGGGACCCCGCTCCTCCTTCCTCGCTCTGCTGTGCGGCCTTGTTCTCACCGACT
TCCTGGGGCTGCTAGTCACCGGCGCCATCGTGGCGTCGCAACACGCTGCCTTGTTGGACTGGCGAGCCACTGACCCCAGC
```

```
TGCCGCCTGTGCTACTTCATGGGGGTAGCTATGGTGTTCTTCGGGCTGTGTCCCCTGCTCCTGGGGGCCGCCATGGCCTC
TGAGCGCTTCGTGGGCATCACACGGCCCTTCTCACGACCCACGGCCACATCGCGCCGCGCCTGGGCTACTGTCGGGCTGG
TGTGGGTGGCGGCCGGGGCACTCGGGCTGCTGCCACTACTGGGGCTTGGCCGCTACAGTGTGCAGTACCCCGGCTCTTGG
TGCTTCCTGACACTCGGGACACAGCGCGGTGACGTGGTCTTCGGGCTCATATTCGCACTCCTGGGCAGCGCGTCCGTGGG
GCTGTCCCTCCTGCTCAACACCGTTAGTGTGGCCACACTCTGCCGGGTCTACCACACGCGCGAGGCCACACAGCGCCCAC
GGGACTGCGAGGTGGAGATGATGGTTCAGCTCGTGGGCATCATGGTGGTGGCCACGGTGTGTTGGATGCCCTTGCTGGTC
TTCATCATGCAGACTTTGTTGCAGACACCACCTGTCATGAGCTTCTCTGGGCAGCTGTTGCGGGCCACAGAGCACCAGCT
GCTCATCTACCTGCGTGTGGCCACCTGGAACCAGATCTTGGACCCCTGGGTCTATATCCTCTTTCGCCGGTCTGTGCTCC
GGCGCCTGCATCCGCGGTTCAGTTCACAGCTCCAGGCTGTGTCCTTGCGCCGGCCTCCAGCCCAGGCCATGCTCAGTGGA
CCCTGAAGGACAGTGCCACTTAAACCTGGGGTCCTTGGTGACTGCCATTGACCTGTGCCCTGCCCCAGGAGCCTGAGTGC
TTGGTGGACTAAGGACGGAGCTACGACATGGGGCTGACTCCTGTGAACTGCAAAGATACCTCCTCTATGCTAGAGAGGTC
CTCCCTGACCCTCTCCCCGGACTACTCCCTGTCAGCCAGGAGGCTGGGGGCACATGTGGGAGGCCCCCTGGCAGGAATCT
AGGATTCCACAGGGAAAACCTGGGATGCTGGAGGTGTCTTCCCCCTTGCCAGCATCTGAGTCTGAGACACAGGGCTTCAC
ATTCCTCCAGGCTCACAGGTCACCAGCCTTGGGTGTTGGAAGCACCCACTCTGTTCCCTGTAGCTGGGTTCACCAACCTC
TCCAGTGTTTCCTGAGCCCCCTTGCTCCATGGCTGCCTAACACACATGCTCAATAAATGGTTGAATGTCC
```

MOUSE PROTEIN SEQUENCE : mP6-034.1 (Seq ID No: 57)

```
WGADPLAASGLGAMWPNGTSLGACFRPVNITLQERRAIASPWFAASFCALGLGSNLLALSVLAGARPGAGPRSSFLALLC
GLVLTDFLGLLVTGAIVASQHAALLDWRATDPSCRLCYFMGVAMVFFGLCPLLLGAAMASERFVGITRPFSRPTATSRRA
WATVGLVWVAAGALGLLPLLGLGRYSVQYPGSWCFLTLGTQRGDVVFGLIFALLGSASVGLSLLLNTVSVATLCRVYHTR
EATQRPRDCEVEMMVQLVGIMVVATVCWMPLLVFIMQTLLQTPPVMSFSGQLLRATEHQLLIYLRVATWNQILDPWVYIL
FRRSVLRRLHPRFSSQLQAVSLRRPPAQAMLSGP*
```

MOUSE PANTHER CLASSIFICATIONS
      FAMILY (SUBFAMILY)
            PROSTAGLANDIN RECEPTOR-RELATED(THROMBOXANE A2 RECEPTOR)
      BIOLOGICAL PROCESS
            Signal transduction(2.11.00.00.00) > Cell surface receptor mediated signal transduction(2.11.01.00.00) > G-protein mediated signaling(2.11.01.07.00)
            Immunity and defense(2.16.00.00.00)
            Blood clotting(2.20.00.00.00)
      MOLECULAR FUNCTIONS
            Receptor(1.01.00.00.00) > G-protein coupled receptor(1.01.01.00.00)

MOUSE GENE ONTOLOGY
      BIOLOGICAL PROCESS
            cell surface receptor linked signal transduction > G protein linked receptor protein signaling pathway
            cell growth and maintenance > cell death
            cell motility > muscle contraction
            G protein signaling, linked to cyclic nucleotide second messenger > G protein signaling, linked to cAMP nucleotide second messenger
            G protein linked receptor protein signaling pathway > G protein signaling, linked to cyclic nucleotide second messenger
      MOLECULAR FUNCTION
            enzyme > nitric oxide synthase
            ligand-regulated transcription factor > ligand-dependent nuclear receptor
            receptor > ligand-dependent nuclear receptor
      CELL COMPONENT
            cell > membrane fraction
            plasma membrane > integral plasma membrane protein
            nucleus > nuclear membrane
            cell > plasma membrane
MOUSE PROTEIN DOMAINS (INTERPRO SIGNATURES)
            IPR001105 (THROMBOXANER)
            IPR000276 (7tm 1)
            IPR000276 (G PROTEIN RECEP F1 2)
            IPR000276 (G PROTEIN RECEP F1 1)

HUMAN GENOMIC SEQUENCE : hD6-034 (Seq ID No: 58)

```
AAGGCATGAGTGACTGCATGCTTTTTCTTTTTAATTTTTACAAAATTAAAGTTTTTTTATTATTTCTGTAGGTTATTGGG
GAACAGGTGGTGTTTGGTTACATGAATAAGTTTTTTTGTTTTTTGAGACGGTGTCTTGCTCTCTTGCCAGGCTGGAGTGC
AGTGGTGTGTTCTCAGGTCACTGCAACCTCTGCCTCCTGGGTTCAAGCAATTCCCCTGCCTCAGCCTCCCAAGTAGCTGG
GATTACAGGCACCTACCACCACACCTGGCTAATTTTTTGTATTTTAGTAGAGACAGGGTTTCACGATGTTGGCCAAGATG
GTCTTGATCTCCTGACCTCGTGATCCACCCACCTCAGCCTCCAAAAGTGCTGGGATTACAGGCGTGAGCCATCGTGCCCG
GCCGTTTACATGAATAAGTTCTTTAGTGGTGACTTGTGAGATTTTGGTGCACCTAGCTTTTTCTAGGAAGTTCTAACCCT
ACAGGAGGTTGTGCAGTAGAAAACGGGTGTCATCCCAGCTCAGCTTCATTAGCCTCCCACCCTGTGTAGGGCCTGGCCTT
TCCCCCACGCAGCCCCAGGGCCCTTGGCCTGCACCCCCAGCCTCTAGCAGCTGCTGGGACCCCCTAAACCCCCGGGCCCC
CTTGTGGGCATTCGGGAGGCATCCAGACATGCAGCAGCATGTCCAGACCCGGCTTTCTATAGACGCTGACCTGGCGGCAC
CTGGCCTGGCTTCCTGGGAGCCTCGCGGCTGGGCTCTTCTGCTTGGGTTTCAGTGTTCGTGGTAGCCCCGCCTGTCTGGG
CACGGCCGCGCTGTGCTTGGCTGGGGCTGCCACGGATTCCAGTGCCACTTAGCGCCCAGCAAAGGGGCTGCAGCTCTGGG
TGCCGATTCCAGGGTCCCAGGTGATTGGCTGGAGGGGACTCGGGTCCGTCTAGACTCTATCCAGTCCCCTGTGGTGTAGG
TGTGGGCCTCTTGACCCTCTGTCCGCCAGCGGGGACTGGAGAAGGCAGGTCTCGAGCAGGGTGCCTGCACTGGTCACCCC
CGCCCAGTGTGTCACCAGCAGCTATGCACCAAGCTCCCTGTGGGCCCAGCATGGCACAGGGAACAGCAGCAGCCAGCCCC
ATTGCCCGCCTCGGGAAGCTGCCATCGCTGGATGGTCAGGGAGGGCTTCCTGGCGGAAGGGATGTTCTCACTGAGCATGG
AGGATGCGAGGAATTCAGGAAGCGAATGGGACAGAGAAGGATGGGTAGCTGGCAGGAAGCACAGTGCGTGCAAAGGCCCA
GAGGTGGCAGGGGTTCGGCATGGGCAGCAAGGGGCAGGGTTGCGAGGTGAGGCCAGACCCAGCGGGGTGGGTGGAAGTGG
CCTGAGAGCAGCACTGAGGCCGCCGGGATGCCACGTGGTGTGGGGCAGAGCACGCGGAGTCAGGCTAGGCTCCAGCCTCC
CAGCACCCCACTCCCGCCCTCAGCAGCCTCCCCGTCTATCGAGGAGGGACTTGAGCCTCGGGCCCCTGGGCCAGTGGTAG
ATGCCAGACAGACTCCATGTAGGATGGGGGGCGGCGGTGGGGAGCTGTTTGTCCTGGGGCGGGCCCAGGACTTCCCCAGG
GGCCAGCAGGGTGGCCTTCTTTGCTAGGCTTCACAGAGGGAAACTAAGGCCCAGAAAGGGCAGGCCACTTGCCCACAGTC
ACACAGCCCAGGCTGGCAGAGAAGATCTGAATTCCAGAGAGCCTGGCTACCGAGCACACCCCCCAACCCCCTCATGGACT
GCCCAAGGCCAGGGCAGAGTTGGGCCAGGTTGGTGGGCCGGGCCCACAACTAGGGTTCCCAGACCCACATATCAGACTCA
CCTGGGGAGGGGCATTCTCAGGGCCAGTCCTGGCCTCCCCCACGCTGGGGGCCTGGGCCTCCAGGTCAAGGCCTCAGCAA
CTGCATCTTAACGTGGCTCCCAACCTCCGCCCACCCCCTCCCCTCCGCTGTGGCCAGGGTGGGGACAACCTGAGACCAGG
GAGGTGGCGGGACTGGATGGGACCCCTGTGGGGGAAGAGGAGGGGAGGGGGACCCCCCCATGGCAGGGGAGGAGGCCCTG
ATGGGGTGGAGGAGCACGTAGGAAATGTGGAACCGCCCCCCCATGCCCCCACCCCGCTGCAGGTGAGCGCCGCGAGGGGG
TCAACGCCTCCGTCAGCTCTGATGTGCAGTCGGTGTTCAAGGGGAAGACATACAACCAGCTGCAGGTCATCTTCCAGGGC
ATCGAGGGCAAAATCCGCGCTGGTGGCCCCAACCTGGACATGGGCTACTGGGAGAGCCTCCTGCAGCAGCTTCGTGCCCA
CATGGCGCGGGCCCGGTGAGCACTAGGTGCGGGTACCAGGGTTGGTGCCGTCCGGGTGGGATGGGTTTCGCCCCCTCCTG
AGTCCTGGGTGGGGTGGGTCTCGCCTCCTCCTGGGACCATGTGGGGTGGAGAGGGGCAGGAAAGTGCCGACTGGCCGGGG
CCAGCCTGTGAGTGACGAAGGGGGGCCGGGGGGCGCGTAGTCCCGTGCCCTAGCGGGAAGGCCCCAGGATGCTCCAGTGC
CCTCACCTGGAGTCAGCCCTGTTGGTCTCACAGGGCTCCCCAGGAGCTTCCGCCCTTTAACGAGTACCTGGCGCGCTCCA
GCAGCCATGGCATCACAGGAAGCCCCCAGGCGGCCTGCCTGTCCCCACTGCCTTGCGCCCAGGCCTGCGCCCTCCCAGCG
CTGCTGCCTCCCGGCCTCTGGCTGTGTACAGATTTCTGGAGTGTTTTTGGCACCGCTGTGTAAATACATGTCTCCTCTGC
TAATTTGTAAATGTCTCTAAATTTATCATGCTTGTTGGCTGGTGTCCTGCTGGGCCCCTTCTCCTGGCCAGCCCCAGCCA
CAAGCACTGAGACTGCAGCGTGAGACAGAGACCCACCTCTCCCTGCCCATGGGATGGTTCACCAGGGTACCTGCGTCCTC
TCGTCCTCTCTCCCATAAGCCTTGACCCTGAACTGACCAAGCCTGGGCAAAGGTCCCAGCCAGAGCCGTGGCTGCTCGCC
ACCTTCTCAGGGTCCTTGCAGCAATAAGGGGCGTGAGAATCTCGGTGGGGGGGTGGGGCGCGGAGCCCCTTCGCCTCCATG
CGGGTGACCCCGGCTCTGCGTCCCAGGCTGCGTGAGCGCCACCAGGACGTGCTGCGGCAGAAGCTGTACAAACTGAAGCA
GGAGCAGGGCGTGGAGAGCGAGCCGCTGTTCCCCATCCTCAAGCAGGAGCCCCAGTCCCCCAGCCGCAGGTAGGCCCGGC
CGGCACCCCGGGGACGCCGATGCAGACGCGGAGCAGGATGGAGGGGCCGTGGGCAGCCTCCGCGCCTCCAACGCTCTCTC
CTCCCAGCCTGGAGCCTGAGGACGCGGCGCCCACCCCGCCCGGGCCCTCCTCGGAGGGCGGCCCCGCGGAGGCCGAGGTG
GACGGCGCGACCCCGACAGAGGGCGACGGCGACGGGGACGGTGAGGGCGAGGGCGAGGGCGAGGCGGTGCTCATGGAGGA
GGACCTGATCCAGCAGAGCCTGGACGACTACGACGCCGGCAGGTACAGCCCGCGGCTGCTCACGGCGCACGAGCTGCCAC
TGGACGCGCACGTGCTGGAACCGGATGAGGACCTGCAGCGCCTGCAGCTCTCGCGCCAGCAGCTCCAGGTCACGGGTAAG
GGCGGGGCTGGAGGCCAGGGGGGTGGGGCCAGTGGGGCGAGCTGGGGCGGGGCTTCTCCAGCCTCATTTCCCGGCCGAAG
CCGCTGGCGTCAGTGGGGAGCTGCTTGCTGCTTCTCCCCCGGGTCTCTGAGCGTGCATTCCGTCACCTGCACGCGCACTG
GGGCGTTCCTTCATTCATCCTGGGCTTGCTGGCCCACTGGCCTTGGGGCCACAGGTGGTGGAAGCTTTTACCGGCCGCTG
TCCGAGCACTCCCTGGGCAGCCTCCGTCCTAAGACAGAGGAGAAAATACTGGCTTCAGAAAACCCCTGGGCCAGGACCCC
CTCGGGGGTCATGCGTGGGGCTGGATCCCGCAGCCAGGCCCTAACCACCAGGTTCTCCTGTCGCTCGGCGGGGAAGCGCG
CTGCGCTTGGTCCCCACCCAGGAGGACGATAGACATGAAGACCAAGGTCCGGGGCTCGGGGCGGATGGTGTGGGTCTCCG
GGTCGCCCAGGCCCTGTCCCCTCCCCATGTGCCTTAGCTCGCCCCAGCCTGTTTTTCCTTTTGCGGGAGGCACCAGCGGCC
GCCCCTGCCAGAGAGGCTAGGAGGGAGGCCAGGACCCAGGGGGAGGCCGAGGCCCGGGTGAACGCCGTCCCGCCCGCAGG
AGACGCCAGCGAGAGCGCCGAGGACATCTTCTTCCGGCGGGGCCAAGGAGGGCATGGGCAGGACGAGGCGCAGTTCAGCG
TGGAGATGCCACTCACCGGCAAGGCCTACCTGTGGGCCGACAAGTACCGGCCACGCAAGCCGCGCTTCTTCAACCGCGTG
CACACGGGCTTCGAGTGGAACAAGTACAACCAGACGCACTACGACTTTGACAACCCACCGCCCAAGATCGTGCAGGGATA
CAAGTTCAACATCTTCTACCCCGACCTCATCGACAAGCGCTCCACGCCCGAGTACTTCCTGGAGGCCTGCGCCGACAACA
AGGATTTCGCCATCCTGCGCTTCCACGCGGGGCCGCCCTACGAGGACATCGCTTTCAAGATCGTCAACCGCGAGTGGGAA
TACTCGCACCGCCACGGCTTCCGCTGCCAGTTTGCCAACGGCATCTTCCAGCTGTGGTTTCACTTCAAGCGCTACCGCTA
TCGGCGGTGACGGCCCTGGGGAACGGCAGGCAGGTGTCCTCGGGGGCACAGTAAAGGGGCTTCGGTGAAGCTGGTGGTCA
```

```
CTCCGGGGCCGCTGTCTGGGCTGCAGGGCCGCGGGTTCACGTCCCACCTCAGGCCGCATCTTTCTTTCGCCAGGCCTCAG
TTTCCTCCTATATAAAAGGGGGGTCACCATTGAGCCTGCCATGGTGAGCTGAGGAGACACTGGGACAGGCTGGCCTAGGG
TCCTGATGCAACGGAGGGGGCGCTAGGGGCATCTTTCGAGCACGAGGGTGGCCAGCAGCCGCCAGCTGACCTCACCGTCC
AGGCGGACACTCTCCACCGCCCCTCAGCCAGGCTGGGTGGGGCCCACAGGGCCTGCCCCAAGTGGCGGCCACACAGGGTG
TTGTGGAGATGGGCCAGAGGGGCCTGGGCTTGGCGGTGCCAGGCTCGTGAGAAGCAGGAAACTCAAGGCCAGCCCTGCCA
CATCACGGGGCACCCACTGGGCGTGAGGCAGAGCCCGCGGCTGTGGGGCAGCTCCAGCCCTGCCCACAGGCCACCCTCGC
CCGCACAGGCAGCACTGAGGCCGGGACCCTGCTGGCGCCCGATCCGCCACTGCAGAAGAGACCCTCTGTGGACACCCACA
CTCTGCAGGGACTGGCCCCCAACTCACCCCGTGCTGGCTGGTGGGGTCCATTTCCCCGCAGCGGGTATGGAGGATGCGTC
GAGGTCCCTGTCTCTGTCAAGGTCGTGGGAACCAAAAGTCACTGAGCCGTCCTCCCCCCACCTCTGAGCACAGGGCCTGG
AGGGCAGGGGCTCCCCGAAGGCTGGGGGTTGGAGAAGGGTGAGGGGCTTCCCCCAGGGATCCCCCACCTGGCCTGCTCA
CGGGGCCTTGGAGCATGAGACCAGTGACCCCGAGTTTTGCCCATATCAGGACAGTGGCTCCTTCTCACTCCCCTTTCTCA
GGCCAGGAGGGCCGAGGGCCACACGGGTGCCACAGCCCAGGTCGGAGTGGCCCAGCCGGCAGGCTTGTTCTTCAGCATCC
GACGGGAACATCTCCAACAGAAGCAAAACGGAAAGTGCCTCCCGGACCCCCAGAGGGCCACCCAACCTCACCAGTCACCA
GCCCCAGACCACCCACAGCCCCTCCCAGACACCCCGCCTCATCTGGAAATAGTTCCGTTTGTTTCTCTAAAAAGACTTGT
AGGTGGGAAAAAAATCTTTTGTTCTCATGGAATTGGCCTATTGGCAAGATCGCATGTTTTTTTAATAAACGTTGTATTT
TAGAATAATCTTAGGTTTGCAGAAATGCAGAGAGTGTAACGTGTCCGACGCCCCACACCCGCTGCCCTAGTTGCTGCTGG
CATTTGTGACACAGCGCATTTGTCCCAAGGGAAGAGCCTGGGAAACAACACGGCACCCAACTGGGGCCCGGTGGTCCCTT
GGGAGCTGTGTTAGTGCCCGGATCTGTCTTTGCCCCCCACCCCTCTGCCTTGCCTCCCACAGTGGGGGTGACAGTCCATT
TCCCAGACGACAGGAGGGAGGGTACAAGGGTGGACACAGCTGCTGGCCTGGGCCCAGGCTCTCATGGGGGCAGCGCTTGG
CCATGCCTGGGGGCATTCGTGGTGGTCCCACCTTGGGGCTGCTCCTGGCGTGGAGTGAGTGGAGGCCAGGGATGCTGCCC
AATGTGCTTCAGTGCCCAGGATGAGCCTCCCCAAATGTCCTCACTGCAGTAGGCAGAGGTGCAAGGCAGATCCTGCACTC
CCCTGGGGCACTGGTCACCTCCCAGCCCCCACCTCTAACAGGATCCCGGAAGTCTGTGGCACAGGGCTTGGGAACTGGCC
AGGGAAGTGCATCTGATCAGCGTCCGCTCTCCACACCGCAGACCCTGGCCCACCCTGACCAGCCTACTCCAGAATCCCAA
ATCGCATTGCAGCACACACAACACTGCCATCCCCAGCCCAGCCTTCAGCAGACATCACAAGTTGATGGCAGATGGCCAGA
GCCATCTTAGAATCCTCTGGGCAGCCTCTTGCCTGGGTTCCTGGCCAGCTCCACCGCCGATCAGGACAGGCTGCTGGGGC
AGGGCTGAGTCCGGGCTGGACTCGGGGGGCACTTTTTATTTTTTTGAGACAGGCTGGAGTGCAGTGGGCGTGATCACAGC
TTTCTACATCCTCAACACCAGAAGGCACTTTTGAGCCCAGAGTGGTGTTTGGGACAGGCCGGGTTCTGTAGTCACAGCCC
AGGTTGTGTGTGCGTGGCTGGCGCAGCCCAGCCTGTACTGGGCCTAACCCAGACCTCCCACCAGCTCTATGTGACAGTAT
TTACTTCCATTTGGCTGCTCGGGAAACTGAGGCCTCACAAAAGCCCACGTTGTTGAGCCCAGAAAGTGGCAGGGCCAGTAT
TAGAAGCTGGGTTTCTCTACCCTGGAGAATCCCAATCCCAGACCACCACCCCGTCCCTGCCTCAGTTTCCCCACCTGCCC
CTGCAGAGATTCCCCCAGCCCCAGCATCCTGCTTCCTCCTCCTCACGTTCCTTTTGCACCCCAAGGGAGATGGCTGTCCT
CCAACTGGCAAGACACATGGTTTGCCAGGGGCCCCAGTCTGGAGGGAGCAGAGCTGGGGCCCTGGGACCCCTGAAGGGTC
ACGGGCACTGAGCCTGGGAAGACGGGCTGCTGGCCGAGAAGGGTGACGGCTGCCTTGCAGAGGACAGGGCAGCAGGTGTG
CCCAGGGCAGGGGGCACACAGGGCTCAGGCCAAGCTAAGGAGCCAGATCCTAGGGGAGCTGGGCACCAGGGAGCTACGGA
GGGCGCGTGAGCTGGGGAGGGCCTCCGAGCTGGGTGTCGAGACCCTGCAGGCAGACTGGAGGTGGGGGCCAGGGGTAGGC
TCTGCACACCACCCCCCCATCTACCCCATGACTCTCCACCATGGCCCGAGGTCCACCTGGTGTCCTGGAAGCTGCCCCCG
CCCCCACCCAGCTTCCTGACTTTGGCTGTGTCCAGAGCTAAGAATAGACGCTCCCTGCCCGGCCCCCGCCGGAAACCGCA
GCCACGGGGGTACATCCTGAGCCTGGCCTCTGTTCCCACCATCTGCCCCCCAGCCCCTCACAACTCCTCCCACCAGAGCC
TCCTGTGCTCCCATTGAACCCGAAGTGCTGGCGGGGGGTGGGGGGCCGGGGGCCGGGCCAAGACCGGGAGACCAGCAACCA
GCCCCAGATGACAGGGAAGCCCAGTGCTCATGTTCCAATCCCACACATGGGCGTGGCATTGTTCCCCCTTTGTGCCTCAG
TTTCCCCATCTCTAAAATTTAAGAGTATCTTTACCCCCTGCCTTGCAGAGATGTGGTGAGGCTTAAGCTAAATGCAAGTG
TCCTTCTCTGAACTCTGCCCAGGCTGTTTCCCAGAAGAGTCATTGTCCTTGTTATCTCAGGGCCGAGGACAGAGGAAGTG
GGGACAGGACAGCCTTCCTCCGTCTCTCACTGCCCCCTCATCTGGAGCCCCAGCCCCACATCCTCCTCCCCCAACATCTG
CCCTCCTTGCAGTTAGTGTAGCAAGGGCCTCATGCTCTCTCTTGCCTCCAAAGCTTTATACATGCAATTCCTGTCACTGC
CACGTAGCCCAGACCTGGGGAGCTCCTATTCATCCTACAAAACCCCACTTCCCATGCCCTCTCCTCACAAGATGTCTTCC
TAGCTCACCTCTGGGCTTCCCTAGGCCCTGTCTCTTCCTTTGGCCCAGCCCTGACCCCAGGGGGCTGGAACAGCCTGTGT
CCAGTTCATTCTCCAGCCTAGGGCATCCTGAAGGGCAGGCCTGGTTTCAGGTCTCACTTGGAGCTCATAATTGTCCCACT
GTATAAGCTGAACACAGAGAGGGGTTGGGGAGAGTAAGACTTGGAGATTAGGTCGGCTTCACGGGAAAGCAGCCTTGGAG
CCAGGGTTTTGCCACATCCATAGGAGTTGGATAGCCTCCTTGCCAGGAAAGAGAGAAGTTGTGTTAAATGGCTGGTGACT
TAATGGGATAGAGCTTTGGGAGGGAGGTTGGGTGTGGCCGAAGGCCAGGAGGAGGAAGGATAGTGGGCGGGGTAGCCGGG
GAAGGGGCTTCCGGACTGGGCTGACGAGAGGGCCGGGCCAGGCTGGAATACCCCTCACTCACAGCCTGGACTAGCAGCC
CTCCCGGGCCCAGGGGACGGGGTGGGGTGCAGTCAGGGCAAGGTCAGCTTGTCCGGTCCCACTCCAGGTCCCTAACTCAA
GGTTCTGTCCGGCTCGGGTGTACAAACAAGGCCAAGGAAGCCCAAGGTCCCCCCTCTGCAAAGAGACGGTCCCAGTCCCT
GCGTCCGGCACCTTCTCAGCCATTCCTGTTGGGCTCCATCCCTCCTTCCCCAGCCCGAGCTGGGGGGTGGCCAGGAAAGA
GTGCCCATCCCCCACTGCAGCCTCCCGGCTCCAGACTGTTCCTCCTCTGGCTCCAAAGCCACCTCCGAGCCTCCTCAAGA
GACCCTCCCATGCGATGCCATCACCCCAGCCCTCCCGGCTCACCTGCTCTGGGACCACCTTTCTCAGTCTGGCTGTGTCG
TCTGGTGCACCCCCCATCCCTCTGTCCTCCCACCCCACCCCTGGAAGGGGCCTCAAATATGTCCTGTATGTATTAAGCAG
GGCATACTACTACTGTGTGCCGCGTTCCAGCGCACCCAGTGAATTTGGAGCCCTGGAGTCTGGGCGACCCCCGGGAGCCG
ATCCGCCTGGACCTGCCCCGCCCCCTCCAGGCCTTGGGTGCTGCCCAGATGGTGAATAATGCGGGCCTGCCGCGGGAGC
GCCGGGAAGAGGCCTGGCAGGGCGCTGGGCGGCTGGAGGGGCTGAGGCTCCCGAGGAAGCGCCCCTGCCACCCCTACGCG
GGCCATTGCATCCCTGCCACCGGTGGCCTGGGGGTCCCAAGGGCGCCGCCTTCCCCAGAAGGCTGTAGGGTGTCCCGGCG
```

```
TGCGGGGGGCACCCACTGACTCCAAGTCAGCCAGGCCCGGGCCCGAGGGGCGTGGCCAGCGCAGGGTGGGCGGGGCTGAT
GAGAGACAGCGGGAGACAGAGACTGGACGGAGAGAGCGAGCCGCGGGCTGCCAGCGGCCCCCACCCGTCCCAGCTCGGCT
TAGCCCCCGCGGACCCCTCCAGGCCGCGACCCCAGGGCGTCCTGTGCCGCCACGCCCTCCATCTGTGTGGGTCCTCTGCT
GGGCCCGCCCCTGGTCACAGCCAGACTGACTCAGTTTCCCTGGGAGGTCCCGCTCGAGCCCGTCCTTCCCCTCCCTCTGC
CCGCCCCCAGCCCTCGCCCCACCCTCGGCGCCCGCACATCTGCCTGCTCAGCTCCAGACGGCGCCCGGACCCCCCGGGCGC
GGGATCCAGCCAGGTGGGAGCCCCGCAGATGAGGTCTCTGAAGGTGAGGTCGCCCCGCTGGCTTAGGGCGGGGATGCACTG
CTCTGGTGTGAGCCCGGGCTGCTGGGCGCATCCGGCGCTGGCCCTGCCCTGCTGTGGGTGTGTCGCAAAGCCCGCGGTGA
CAGCGGCCAGGTGCCAGGTGTCCCCTGTGTGGCCCGGCGGGTGGCATGGTGCCCTGAGGCCTGCCCTGGGCGCCCAGGCG
GCTGGGTCTGGGTGCGGCCCAGGCTGATTCTGTCCGGGGGGCTCTCTGGTCTCTGTGGGGCCACATGGCTGTGAATGGGG
GTCCCAGACTCAGGGGGCTGCAGCCGGGGAGGGGGCCCAGGCCCTGGGTGTGGCAGGGATCGAGTGTGGTTGTGCGGCTG
TGTGTGTGATCTTGTCTCTGGGAGTGAACCATGCATGGCCCTGGTTGTGTGTCTGTGTCTGTGCGTGTGTGGTGGTTTTG
TGTGTGTGACCGTGTGTTTGTTTCTGCCGAGAGTCTGTGACTGTTAAATACTGTGTGTTCTTCTGTGTGTCTTTCTGTGT
CTGTCTGTATCTATGTACCTGTTGTGTTTCTGCCTGTGTGTCTGTGTTTTTCTGTCTGTGTCTGTCACATTTCTGCCTGT
GTGCCTATGTGTGTATCTGTGTGTGTCTATGTCTGTCGTGTTTCTGCCTGTATGTGTCTGTGTGTGGCTGTGTGTCTGTT
GTGTTTCTGCTTGTGTGTGTCTATCTGCGTGTGTCTGTGTGTCTGTTGTGTTTCTGCTTGTGTGTGTCTGTGTCTGTCAT
GTTTCCATCTGTGTGTGTCTATCTGTATGTGTCTATGTATTTGCCATGTTTCTGTATGTGTGTCTGTGCATGCCTGTGCG
TCTGTCTACTGTGTTTCTGTCTGTCACATTTCTGCCTGTGTGTCTGTCATGTTTCTGTCTGGGTGTGTCTGTGGTGTTTC
TGTGTGTGTCTATCTGTGTGTGTCTGTGTATCTGCCATGTTTCTGTATGTGTGTCTGTGCATGTCTGTGCATCTGTCTGC
TGTGTTTCTGTCACATTTCTGCCTGTGTGTCTGTCATGTTTCTATCTGTGTGTGTGTCTGTGTGTCTGCCATGTTTCTGT
GTGTGTGTCTGTGCACGTCTGTGCGTCTGCCTGCTGTGTTTCTGTCTGTCACATTTCTGCCTGTGTGTCTGCTGTGTTTC
TGCCTGTGTGTGTCTGGCTGTGTCTGTGTGTCTCGAGTGTGCACACACGGTGGTGGCCTGAGGTGCATGTTGGTGGAATC
ACTGTAACTTGTGGTCCACCATTAACCACCAGCTGCACCTCCCGGGTCCCGCCCCCTGGTCCCTGCTGGCCCCTGTGTGA
CGGAGCTCCGTACATGCTCCCTGGGGAGCCCCCACTAGAGAGATGGGGAAAGCGAGGCTCACAGGGGGCCATGTGACTTA
GGGCCCCAACAAGGCTGGCCGGAGTCTGGCTGGCTGGGGGAGTCCCCGCCCTGCACTGTGTGGGCTGGGGACCCTGTTGG
TGTCACAAACAGGAAATGTTCTCGGAAATGGAGCTTTGCGATGAGGGGAGGTGGGCCCATCAGGGTGTGGAGTGTGTCTG
CCTCCGCTACCATGTGGGGGTCCACGCCCATGAGCGTTCAGATCTACAGCAGGCATGGGAGGGTCTGGCCGGTCCCTGAA
GTTTCAGTCCCAAAAGGGCCCTGTGGGTCCAAGGTCCAAGGCCGACAGGGGTGACACGGAGCCTCAGGGCTCCCTGCTGG
CTGCCCGGGCACCTTGTAGGGAGTTGAGCTGTGAGTACAGCCAGGAGCCCTGCAGAGGTGAGAGCCAGGGAGGCCTGAGC
TTAGCACCCGCAGGCATGGAGATCAGAAGCTCACCCCCATTGCACAGAGCGGGGAAACTGAGGCTCGGCAAGGTGGAGCC
CCAGCCTGGCCACGGCAGATCAGGGACTAGAGCCTCCAAGCCTGTCTGGGGGAGGTGGGGTGGGCTCAGGCCGTGACAGG
CAGGCGGGGCGGGCAGGGTGGGGTGCTGAGGAGAGGGGACTTGTTGAGAAATGGATGGATGATGAGGTGACGTGCTCCAG
GGAGTGGGGGAGGGGGCACGAGGATGGAACATGGGGCGGAGGAGAGGCCTGCTGTTTGGGAACCAAGGATCACATTCCAC
TGGCAACCAGGCTTGGGGCAGCCAGGCCTGGTGCCCACGCCCAGCCCAGCGCCCGGGCCTCTGGAAACAGCGCCGGACAG
AGCCCTCCGAGTCACACGACCTGCAAGCCGGATACCCTGTCAGGGGTGGCATCGGATGAGGGAGAACTCACTGTCCGCCT
TCCAGAAAGAAGCCAGGAATTAAGACAGAGATTTCTGGAACCCGGGACCCAGGCAGATTCCCATCAAAGTTGCAGGACCT
CAGCCTCTTGGGAGTTGCAGGACCTCAGCCTCTTGGGATTCTAGAATTGTGAGGCCTTCTGCGTCCCAGGACTGGAAGCG
TGTTAGAATTCCACAACCCTGAACCCGCTGAGGGCAAACGTCTGCTCTCAGACTGCAAAATTCTGGAGTTATTCAACATC
GAAAAGCGATCTTGGGTCCACACCTCTCCCCAGACACAGGGAGGGACAGTAATTTTCCCAAGAGCCGCAGTGCTGCTCTA
CTGCCCCACCGCGTGGGGGCCCCTGCTACCCCTCCTGGCCAATGCAGGAGGGAAGGGAGGCCCTGCCCGGTCCCCCCATT
GGCATCCTTGCCTGGAGAACTTGAGATGGCTGCCATCGCCTGAAGCCAGGGTGGGGAGTGGAGGTGGGGGGATTGGAAAC
AGCCAGCTGTGGCCAGAAGGACTCAGTGCCAGTTTCCAGGCTCCCCTGGGCCAAGGGCTCTGGGCCATGCATTTGCTCCC
AGCCACCGGAGGTGTGAGGGAGTTCCGGGTGTGTGACCCCCGGTGAGTGGAGTGGAGTGTGGTCGAGGAGACCCTGAGAC
CCAGGAGGGACTGGAAGTTGCTCCAGGCAGAGGTCGGGTCAGTGGCCGCAGGGACAGCCGAGTCCCAAGGAATGTCCAGA
AACAGCACATACCAGCTCTCGAGGGAGGAACGCCTTGTCCCAGGGGAAATCCCAGTGCAGGAATCCAAACCCCAGGCGCC
AAAGCAGCCCGGAGTGGAGGGCAACGATCGCCAGAGGCCGCGAGGAACCCCCCACCGCATCTGCTCTCCAGGCCTCAGTT
TCTCCTCTGGAAGCCCTGCCTCCCACCACGGTGGCAGTGGCCTTAGCTGTCTTCCCAGGAAGGGAGTTCAGTGCCGCTGT
CAGGAGCTGGATAATTTATGAGGCTGGATACAGCGTAGCCAAGGCCAGAAGCCAGGCCAAAGCGTCACAAGTGAGATGGG
GAGTGAGGGCCACAGTCACCCATTTGACCGCCCTGGAATGCGTTAGCTGCTGCGACCCCACAGCCCAGGCCCTGCCTCAG
GGCCAGCATGCATTGAGTGCCCGCTGTGCTGTGCTGAGTGCTGCGCGTGGATCCCTTCACTCCCGTCATCAGCAACCCCG
ACTTTTTTTTTTTTTTTGAGATGGAGTCTCGCTCTGTCGCCCAGGCTGGAGGGCAGTGGCGCGATCTCAGCTCACTGCAA
GCTCCACCTCCCGGGTTCACACCATTCTCCTGCCTCAGCCTCCCAAGTAGCTGGGACTACGGGCGCCCGCCACCACGCCT
GGCTAATTTTTTGTATTTTTAGTAGAGACGGGGTTTCACCGTGTTAGCCAGGATGGTCTCGATCTCCTGACCTCGTGATC
CGCCCACCTGGGCCTCCCTCTTTTCTTTTCTTTTTCTTTTTTTTTTTTTTGAGACGGAGTTTTGCTCTTGTTGCTCAG
GTTGGTTCAGTGGCACGATCTTGGCTCTGGGGCCCTGTTTCCGGCCCACAAACATCACTTCCCCTGTCGGGTTCAAGCGA
TTCTCCTGCCTCAGCCTCCCGAGTAGCTGGGATTACAGGTATGCACCACCACACCCGGCTAATTTTTGTATTTTTATTAG
TTGAGATGCGGTTTCACCATGTTGGTCCGGCTGGTCTCAAACTCCTGACTTCAGGTGATCCGCCCTCCTTGGCTTCCCAA
AGTGCTGGGATTACAGGCGTGAGCCACCACGCCTGACCGTAATAGGACCTTTTGAGCCTTAACTGCCTGGCACCTCCATC
TCGCACACAGTCCCATTCGCCCACCTTTGCCCACCCAGTCCCTCCAGGTGACTTGGGGGTGTGTCAGGGCAAGAGGCAGG
AGACCCGGGTTCCATCCTAGCCTCCTGGGTGACTTTACAACCAGCCACAACCCTCTGGACTTTGACATGACCCATGGGGT
ATGGGGAGCTGGTTTTGTTTTGTTTTGTTTTTTAGACGGAGTCTCGCTCTGTCACCCAGGCTGCAGTGCAGTGGCACGAT
CTCAGCTCACTGCAAGCTCCTCCTCCCAGGTTCACGCTATTCTCCTGCCTCAGCCTCCCGAGTAGCTGGGACTACAGGCT
```

```
CCCGCCACCACGCCCGGCTAATTTTTTTGTATTTTTTAGTAGAGACAGGGGTTTCACTGTGTTAGCCAGGATGGTCTCGATCT
CCTGACCTCGTGATCCGCCCGCCTCGGCCTCCCAAAGTGCTGGGATTACAGGCGTGAGCCACCGCACCTGGCCGTTTTGT
TTTCTTGAGATGGAGTCTCGCTCTGTTGTCCAGGCTGGAGTGCAGTAGTGCGATCTCGGCTCACTGCAACCTCTGCCTCC
CGGGTTCAAGCATTTCTTCTGCCTCAGTCTCTTGAGTAGCTGGGATTACAGGCGCCCGCCACCATGCCTGGCTACTTTTT
GTATTTTTAGTGAAGATGGGGTTTCACCATGTTGGCTAGGCTGGTTTCGAACTCCTGACCTCAGATGATCCACCCACCTC
AACCTCCCAAAGTGCTGGGATTACAGGCATTAGCCACTGCACCCAGCCAGAAGCTGGTTTTTGAACCATCTCATTTTATT
TTTACTTCACTCATCACACCTGGCCCCATTTTTTTGTTTTTTGGGGTTTTTAGCGTTTTTGTTTGTTGGTTGGTTGGTTT
TTTTTTTTTTGAGACAGGGTCTCACTGTTGCCCAGGCCGGAGTGCAGTGGCACAATTGTAGCTCACTGCAGCCTCAACAT
CTGGGGCTCAAGCAATCCTCCAACCTCAGCCTCCCAAGTAGCAGGGATTACAGGTGCATACCACTACACCCAGATATATA
TATATTTTAAGAGATAGGGTCTCGCTATGTTGGCCAGGATGGTCTTGAACTCCTGACCTCAAGTGATCCGCCCACTTTGG
CCTCCCAAAGTGCTGGGATAAGAGGCGTGAGCCACTGCGCCTGGCTTGGCTCCGATTTTGTTGTGCATTGTGGCTGAGCG
TTAGCCCCACACAGAGTAACAGGTCCCCGCCTGCTGCTCTCTACCAACCATCCCTGGGGAACCTGCCATTCCGAAGAAGT
GTTTTTTGTTCCCAAAATAGAACTGGATTTCAGGGTTTATTTCTTATTATAAAACCGACACCATGAAAATGGAGGAAAAG
GCCGGGCACGGTGGCTCACGCCTGTAATCCCAGCACTTTGGGAGGCCGAGGCGGGAGGACTACTTACTACAGGGTATGTG
CCTGTAGTCCCAGCTATTTGGGAGGCTGAGATGGGGGGATCACTTGGGTCCAGAAGGTCGAGGCTGCAGTGAGCCTTGAT
TGCATCACTGCACTCCAGCCTGGGTGACAGAGCAAGACTCTGTCTCAAAAAAAAAAAAAAAAAAAAACCGGAGAGGATATT
TGAGCTGGGGCATTGAAGGTTGTGTAGGAGTTCACCAGAGCTACTTACACTGAAATGCAGAATTAATCACAAGCAAATCT
TCTCTCGCCTCCCAGGTGTGCCTGAACCAGTGCCAGCCTGCCCTGTCTGCAGCATCGGCCTGATGGGGTGGTGACTGATC
CCTCAGGGCTCCGGAGCCATGTGGCCCAACGGCAGTTCCCTGGGGCCCTGTTTCCGGCCCACAAACATTACCCTGGAGGA
GAGACGGCTGATCGCCTCGCCCTGGTTCGCCGCCTCCTTCTGCGTGGTGGGCCTGGCCTCCAACCTGCTGGCCCTGAGCG
TGCTGGCGGGCGCGCGGCAGGGGGGTTCGCACACGCGCTCCTCCTTCCTCACCTTCCTCTGCGGCCTCGTCCTCACCGAC
TTCCTGGGGCTGCTGGTGACCGGTACCATCGTGGTGTCCCAGCACGCCGCGCTCTTCGAGTGGCACGCCGTGGACCCTGG
CTGCCGTCTCTGTCGCTTCATGGGCGTCGTCATGATCTTCTTCGGCCTGTCCCCGCTGCTGCTGGGGGGCCGCCATGGCCT
CAGAGCGCTACCTGGGTATCACCCGGCCCTTCTCGCGCCCGGCGGTCGCCTCGCAGCGCCGCGCCTGGGCCCACCGTGGGG
CTGGTGTGGGCGGCCGCGCTGGCGCTGGGCCTGCTGCCCCTGCTGGGCGTGGGTCGCTACACCGTGCAATACCCGGGGTC
CTGGTGCTTCCTGACGCTGGGCGCCCGAGTCCGGGGACGTGGCCTTCGGGCTGCTCTTCTCCATGCTGGGCGGCCTCTCGG
TCGGGCTGTCCTTCCTGCTGAACACGGTCAGCGTGGCCCACCCTGTGCCACGTCTACCACGGGCAGGAGGCGGCCCAGCAG
CGTCCCCGGGACTCCGAGGTGGAGATGATGGCTCAGCTCCTGGGGATCATGGTGGTGGCCAGCGTGTGTTGGCTGCCCCT
TCTGGTGAGTAGGGGCTCTGCGGGGTCCTGCTACCCTCCTGACTGGACCTCTGGTCACCCAGGGTGGATTTAGATCCTGA
TGGTGGGACCGGGCGCGGTGGCTCACGCCGGTAATCCCAGAATCTGGGAGGCTGAGGCGGGAGGATCGCTTGAGGCCAG
GAGTCCGAGACCAGCCTGGCCAACATGGTGAAACCCTGTCTCTACTAAAAATACAAAAATTAAGGCCAGGCGTGGTGGCT
CACGCCTGTAATCCCAGCACTTTGGGAGGCCGAGGCGGGCGGATCATGAGGTCAGGAGATCGAGACCATTCTGGCCAACA
CGGTGAAACACTGTCTCTACTAAAAATGCAAAAAAATTAGCCAGGCGTGGTGGCAGGCACCTGTAGTCCCAGCTACTCTG
GAGGCTGAGGCAGGAGAATGGCATGAACCCAGGAGGCGGAGCTTGCAGTGAGCAGAGATTGCGCCACTGCACTCCAGCCT
GGGCGACAGAGCGAGACTCCGTCTCAAAAAAAAAAAATAAAAAATAAAAAAATTAGCCAGACATGGTGGCATGTGCCTGCA
ATCCCAGCACTTTGAGAGGTCAAGGCAGGAGGATTGCTGGAGCCTGGGAGGTTGAGGCTGCAGTGAGCTATGATTGCACC
ACTGCACTCCAGCCTAGGTGACAGAGTGAGACCCTGCCTCCAAATTAAAAAAAAAGAAAAAAAATAGAAAGCAGCCGTAG
AAAACACATAAATGAGTAGGCGTGGCTGTGGCCAATAAAACTACTGATGGATAGATGATGAAATTTGAATTTCATGTCAT
TTTTATGTCACTCAATTTTTGCCTCTATTTTGTTTTCCTCTAACCATTTAAACATGGAAAGAAATTCTTAGTAAAAAACA
AAACAGGCCGGGCGTAGTGGCTCATGCCTGAAATCCCAGCACTTTGTGAGGCAGAGGCGGGCAGATCATGAGGTCAAGAG
ATGGAGACCATGCTGGCCAACATGGTGAAACCCCGTCTCTACTAAAAATACAAAAATTAGCCGGGCGTGGTGGCGCACG
CCTGTAGTCCCAGCTACTTGGGAGGCTGAGGCAAGAGAATCGCTTGAACCCAGGAGGCGGAAGTTGCAGTGAGCCGAGAT
CACGTCACTGCACTCCAGCCTGGGTGACAGAGCGAGACTCCGCCTCAAACAAAAACAAAAACAAACAAACAAACAAAAAC
AGGCCAGCCACGGTGGCTCATGTCTGTAATCCCAGCGCTTTGAGAGGCCGAGACAGGCGGATCACCTGAGGTCAGGAGTT
CAAGACCAGCCTGGCCTACATGTTGAAACCCCATCTCTACTAAAACTAAAAAAAATTAACTAACCATGATGGCACACTCC
TGTAGTCCCAGCTACTCAGGAGGCTGAGGCATGAGAATTGCTTGAGCCCAGGAGGTGGAGGTTGCAGTGAGCCAAGATTG
TGCCACTGCACTCCAGCCTGGGTGACAGAGCAAGACTCTGTCTCAAAAAAAAAAAAAAAAAAAGAAAGAAAAGAAAGAAA
GAAAGAAAAGAAAGAAAAAAAACCCAAAGCAAGTGGCAGGCCAGATTTGGCCCATGGGTCAACCCTTGCTCTAAACCCT
CCCATGGCTCCCTGTTGCCCTCAGAAGAAAGTTCTCACAGGCACTTCCTGCAGACGCACCTTGCCCACTCCCATTTGCCC
TTCAGTGACTTCCCTAGGCCAGGTCAGGCACCTCTTCTGGGCTCTGACATTGCTTCCTCCTCACAGCTTTGCCCACCCCA
TGCTGTAACTCCCCGGACCCAGTTTCCGTCTCCCGACTGGACTGTGAGCCTATGAGGGTGCGCCCTGGGAGTTCCTCAGC
TGCTGTTGCTGCTTCATTCAGCCTACAGGACCTACTGCTCAGGAAATGATTTGATTGATTGATTTAATTTATTTAG
AGATGGAGTCTCGCACTGTTGCCCGGGCTGGAGTGCAGTGGTGCGATCTCAGCTCACTGCAAGCTCCGCCTCCCAGGTTC
AAGCGATTCACCTGCCTCAGCCTCCCGAGTAGCTGGGATTACAGGCGCCCGCCACCATGCCCGGCTAATTTTTTTTGTAT
TTTTAGTAGAGACGGGGTTTCATCGTGTTAGCCAGGATGGTCTCAATCTCCTGACCTCGTGATCTGCCCGCTTCAGCCTC
CCAAAGTGCTGGGAGTATAGGTGTGAGCCACCGCACCCGGCCAAATTATTTTATTTTATTTTAAGATGGAGTTTCGATGT
GTCACCCAGGCTGGAGTGCAGTGGCGCAATTTTAGCTCACTGCAACCTCTGCCTCCTGGGTTCAAGGGATCCTCCCACTT
CAGCCTCCGGAGTAGCTGGGACTACAGGTGTGCACCACCACACCCGGCTAATTTTGTATGTTTTAGTAGAGATGGTGTTT
GACCATGTTGGTCAGGCTGATCTCAAACAAAGTATTTTAATATAATGTAACATTTAATTATAATATAATTGTATGATATA
TGATATATATCAATTATAGGCCACATTATAATTATGAATAATTATCATATAATTGATTATTATATATTATATACTAATTT
GTAGATAACATTCTTATTTTAAATATTTTTAAATATAGTTTAAAAATTTCCTGCCAGGCGCGGTGGCTCATGCCTGTAAT
```

```
CCCAGCACTTTGGGAAGCTAAGGCGGGTGGATCACCTCAGGTCGGGAGTTCAAGGCTAGCCTGACCAACATGGAGAAACC
CCGTCTCTACTAAAAATACAAAAAAATCAGCCAGATGTGGTGGTGCATGCCTGTAATCCCAGCTACTCGGGAGGCTGAGG
CAGGAGAATCGCTTGAACCCAGGAGGCGGAGGTTGTGGTAAGCCGAGAACCTGCCATTGCACTCCAGCCTGGGCAACAAG
AGTGAAACTCCGTCTCAAAAAAAAAAAAAAATTTCCCATCAATCCTGACCTATAGATAAATATAATTTTTATTATTTAAA
TATTTTATTTATGGCCAGGTGTGGTGGCTCACACCTGTAATCCCAGCACTTTGGGAGGCCGAGGAGGGCAGATCACAAGG
TCAGGAGATCGAGACCATCCTGGCTGACACAGTGAAACCCTGTCTCTACTAAAAATACAAAAAATTAGCCGGATGTGGTG
GTGGGCGCCTGTAGTCCCAGCTACTCGGGAGGCTGAGGCAGGAGAATGGCGTAAACCCAGGAGGCGGAGACTCCGTCTCA
AAAAAAAAAAAAAGGTATTTATTTATTTATTTTGAGACAGGATCTTGCTCTGTCACCCAGGCTGGAGTGCAACCATGCCT
GGGTAACTTTTCTATTTATTTTAACTTTTTGTAGACATGAGGTCTCACTATGTTCCCCAGGCTGGTCTTGAACTCCTGGG
CTCAAGTGATTCTCTCCTATTTACCTCCCAAAGTGCTGAGATCACAGGCGTGAGCCACTGCCTGGCTAAGAGTGCGCTAA
TTTATGAACCCATCAAAAGGAGCTCAGAGCGGTTAGTCAACTCTCCTGAGGTCACCCAGCAATGCAGTAACAGAGTGGAC
ACTGGCCGGGCGCAGTGGCTCACTCCTGTAATTCCAGCACTCTGGGAGGCTGAGGCTGGTGGATCACCTGAGGTCAGGAG
TTCGAGACCAGCCTGGCCAACATGGTGAAACCCCCATCTCTACGAAAAATACAAAAATTAGCCAGGTGTGGTGGCAGCCA
CCTGTAATCCCAGCTACTTGGAAGGCTGAGGGAGGAGAATTGCTTGAACCCGGGAGGCAGAGGTTGCAATGAGCTGAGAT
CGCACCACTGCACTCCAGCCTGGACGACAGAGTGAGACCCTGTCTCAAAAATAAAATTAAAAAACGGAGTGGACCCTGGA
TCTCAAGGGACCCCGGGCAGGGACCGGGGCGGGCGGGGCTGGAGGCGGGGGCCCAGGCTGACAGCTCTCCCCTTTGCAGG
TCTTCATCGCCCAGACAGTGCTGCGAAACCCGCCTGCCATGAGCCCCGCCGGGCAGCTGTCCCGCACCACGGAGAAGGAG
CTGCTCATCTACTTGCGCGTGGCCACCTGGAACCAGATCCTGGACCCCTGGGTGTATATCCTGTTCCGCCGCGCCGTGCT
CCGGCGTCTCCAGCCTCGCCTCAGCACCCGGCCCAGGTCGCTGTCCCTCCAGCCCCAGCTCACGCAGCGCTCCGGGCTGC
AGTAGGAAGTGGACAGAGCGCCCCTCCCGCGCCTTTCCGCGGAGCCCTTGGCCCCTCGGACAGCCCATCTGCCTGTTCTG
AGGATTCAGGGGCTGGGGGTGCTGGATGGACAGTGGGCATCAGCAGCAGGGTTTTGGGTTGACCCCAATCCAACCCGGGG
ACCCCCAACTCCTCCCTGATCCTTTTACCAAGCACTCTCCCTTCCTCGGCCCCTTTTTCCCATCCAGAGCTCCCACCCCT
TCTCTGCGTCCCTCCCAACCCCAGGAAGGGCATGCAGACATTGGAAGAGGGTCTTGCATTGCTATTTTTTTTTTTAGACG
GAGTCTTGCTCTGTCCCCCAGGCTGGAGTGCAGTGGCGCAATCTCAGCTCACTGCAACCTCCACCTCCCGGGTTCAAGCG
ATTCTCCTGCCTCAGCCTCCTGAGTAGCTGGGACTATAGGCGCGCGCCACCACGCCCGGCTAATTTTTGTATTTTTAGTA
GAGACGGGGTTTCACCGTGTTGGCCAGGCTGGTCTTGAACTCCTGACCTCAGGTGATTCACCAGCCTCAGCCTCCCAAAG
TGCTGGGATCACAGGCATGAACCACCACACCTGGCCATTTTTTTTTTTTTTTTTAGACGGAGTCTCACTCTGTGGCCCAG
CCTGGAGTACAGTGGCACGATCTCGGCTCACTGCAACCTCCGCCTCCCGGGTTCAAGCGATTCTCGTGCCTCAGCCTCCC
GAGCAGCTGGGATTACAGGCGTAAGCCACTGCGCCCGGCCTTGCATGCTCTTTGACCCTGAATTTGACCTACTTGCTGGG
GTACAGTTGCTTCCTTTTGAACCTCCAACAGGGAAGGCTCTGTCCAGAAAGGATTGAATGTGAACGGGGCACCCCCTTT
TCTTGCCAAAATATATCTGCCTTTGGTTTTATTTTCCTTTGGGTCCAGAAGTTTTCAATCTCTGGAGTTTGTGCATTG
GGCCTCACCCGGAGCAGACGAGGGCAGGGCTGGGAAGGATGGGGAGGGAGCATAGAGGATTCGGTTCCTCCCCCCACCTTGT
TTCTTGAGTGTTTCCTAATCACTTGATGGGTGGTACCACCTAGAATTCTTCCAGCAGCCTGTAATGGGAGGGCTACAGGT
TAGCCCTGGAACCTGCAATCAAACCACCTTTGAATCTGTGTGTCATTAAAAGTAGATATAAATGGGCCGGGCGCGATTGT
TCATGCCTGTAATCCCAGCATTTTGGGAGGCCGAAGCGGGAGGGTCACTTGAGGCCAGAAGTTCGAGACCAGCCTGGCCA
ACATGGTGAACCCCTGTCTCTACTAAAAATACAAAAATTGCAGGGCGCGGTGGCTCACGCCTGTAGTCTCAGCACTTTGG
GAGGTCGAGACGGGCAGATCACCTGAGGTCGGGAGTTAGAGACCAGCCTGACCAACATGGAGAAACCCCGTCTCTACTAA
AAATACACAAAAAATTAGCCGGGCGTGGTGGCGCATGTCTGTAATCCCAGCTACGCGGGAGGCTGAAGCAAGAGAATAG
CTTGAACCCGGGAGGTGGAGGTTGCAGTGAGCCGAGATCACGTCATTGCACTCCAGCCTGGGCAACAAGAGCAAAACTCT
GTCTCAAAAAACAAACAAACAAACAAACAAACAAACAAAAAATTAGCCAGGCATGGTGGCGGGCGCCTGTAATGCCAGCT
ACTCAGAAGGCTGAGGCAGGAGAATTGGTTGAACCCAGGAGGCAGAGGTTGCAGTGAGCCGAGATCATGCCACTGCACTC
CAGCCTGGGCGACAGAGTAAGACTCCATCTGTAAATAAGTAAATAAATAAAACAGAGAAAGAGAGAGAGAGATGCAAAAG
CGTGGAGTGATGCCCGTTTCCTCCACTGTTCACTTGTTCACCCAGCAACCTTTCAAACACCACAGTGTTCTGTACAGCAC
CTTGCACATAGTACGTGCTCAATAAATATTGGTAGAAAGGACCAGTGGCTGGGTCTTCGTGGAGCACCTGTCGCCTGCAG
GCACGTAAGCTCATGCCTGTGCGGTGCTTAATGAAGTGCCTGGTACACACTAGGTGCTCAAGAGGTATTCAGTGTTGCCA
CAGTTTCAGCCAGCCCCCCAGCCCCACATCTCTGCATTCCCCCAAACTTCCCCCAAAATGACACACCAGGCTGCAGAGAT
GGAAGGTGCCTTTATTCACCATGAGCCCTCAGGGTACATGCGGTGCAACCCGTCTCCTGCCCTCCTTTTCCCTCCAAGGA
CCTGGATCTGAGCCAGGACCGGTGAAGCCCCTGTTTTCACCACCGCACACTCCCTACCACCGCCTGCTGGAGAAATAAGG
TCACTTGCTGCTTCTGGGGGCCCCAGGCTGGAAGGGACAGGAGAGGACGAGAACCACATGAACCAGGGCTAAGTCACAGC
CAAGCTACGGCCCCCCGCGGTAAGGGCGGGCTCCTGGCTCCCCCAGAGTGGGGCAAAGGGCTGGCGCCAAAGCTGGGGAC
TCAGACAGCTGGGAACTCAGGAGGGCTGCCCTAGGGGCAGGACTAGGAGTGACAGATATGGTGGGTGGGGTGGACTTCTG
GGGTCCCACCCGGGGCTTGGGAGAGACGGCTGGCTTCTGGGGGGTTGAGGAGACCTGTGACTGTGGGGCAGGTAGGAGG
CTTGGGGTAAGCCTGGGATGGGGCCAAGGCTGGCCTGGATGCCACATACCTGATCATGGGGCTGAGGCAGAGGCTGGGGG
TCTTTGCTAGGGGCTGGGGTGGGGTCTCTGGGGGATGGGCCTGGGGGCTGAGGTTGGGCTGGGGCAGGGGCCTCAAGCTG
GATTCTAGGGTCAAGGCCAGGGGTCTGTGGGCAGATTCCATTTCCAGGGCTCAGCCAGATTCTTGAACTGGTGTGAGTTC
TGGAACTGGATTGGGTTCCAGAAACAGGCTGGGTTCCAGAACTAAGGTGGGCTCCAGGGCTGGGCTGAATTCTTGAGCCG
GTCTGGGTTTCAGAGCTGAGCCCCGTCTCAGAGCTGGACTGGGTTCTGGAACTAAGTTGGGCTCCAGAGTCGGGCTGAAT
TCTTGAGCCGGTCTGGGTTTCAGAGCCAAACTGAATCCCAGAACTGAACTGGGTTCCAGAGCTGGACTGAGCCTCAGAGC
TGAGCCTGGTCTCAGGACTGGACTGGGTTCTGGAGCTAAGATGAGCTCCAGGGCTGAGCTGAATTCTTGCAGTGGTCTGG
GTCCCGGAGCTGAGCTGGTTTCCAGAACTAGTCTGAGTTCTGGAGCTGGGTTGGGCTCCGGAAATCAGCTGGGTTCCCGA
ACTAAGCTGACTTCCAGAGCTGGCTTGAATTCTTGAGCTTATCTGGGTCCCAGAGCTGGGCTGAGTTCCAAAGCTAAGCT
```

```
GACTTTCAGAACTGCTCTGCTTCCCAAAGTCCAGTTGAATACCAGAGCTGTCTGGGGCTGTGGTCTGGAGCTGGTATCTA
GAACCTGGCAGGGTTTTAGAGGCGAATTGAGTCCCAGAGGCTGGTCGAGCTCCTGGGCAGCGCTGGGTCCTGGAGCTGGG
CCTGCTGTCGGAACTAGACAGGGCTGTGGAGCTTAATTCTATTGCTGCGATGGCCTGGGTCCCAGGACTAGATTGGACTC
CAGAGCTTAGCTGGATTCTGGAACTTGGCTGGGTCTCAGCTGCTGTCTGTGTTTTGGAGCTGGCTGGGATTTCAGAACCA
GCCTGTGCTCCAGAACTGAGCTGAGTTCCAGAGCTGGGTGCGGTGCTTGGGCTGGATTGAGCTCTGGAGCTTGGCCAGGC
CTTGAAATTGGTCTGGGTCCTGGAAGTAAACTGCATTCTAGAGCTGAGCTGCATTGTGCAAGGAGAGTGGGGCTTGGCAC
CAAGCTGTGTGCTGGAGCTGGGTCTTGCTTCCAAGATGGATGGGGTTCCAGAGCTGGTCTGAATCCTGGAGTTGGGCCTG
GTTGTAGCACTAGGCGAGGTTCTAGAGCTGGGCTCAGTCTCAGAGCTGGAATCATCACTGGAGCTGGACTGGGCTGCAGG
ATGGGGATGGGCACTTGGATGCAACTGGGGTTGGGATCTGGACTGGGCAGCCGTGTGGACCTGGGTTTCTGAGCTGGTCT
GAGTTCTTGAATAGGATTTGATTTGGGAGCTGTCTCTGAATCCTGAGCTGACTGGGGTTCCAGAGCTGGGCTGCTTCATG
AATCTCACATGGGTTCCAAAATTAACTGGGGTTCCGGAGCTAGACCGAGTGTCCAGGTGTGGCTGCATCGTGGAGCTGTC
CAGAGTTCCAGAGTCAGCTTGAGTTTTCCAGCAGGGCTTGGTCTCCGAGCTAGCCTGGATTCTGGAACTGGGATTGGTCT
CCAAGCTGGTCTGGGTCTTAAAATGTGTCTGGGTTCCAGAGTTTGGCTTGTTGTCAGGGCTCAGCTGGATTCTAGAGATG
TGCTTCGTCTCAGAGCTGGCCTGGGCTTCAGAGCAGGGCTTGGTCTCAGATCTTATCTGGGTTCTAGAGCTGGGTTTGGC
CTCAGAGGTGGCCTGAGTTCTAGAACAGGGCTTGGTCTCAATGCTGGGATTGGTTTCAGAGCTTATCTGGGTTCTAGAGC
TGGGCTTGGTCTCAGGGCTGACCTGAGTTCTAGAGCTGGGCTTGGCTTCAGATGTGGCCTGAATTCTAGAACACGGCTTG
GTCTCTATGCTGGGCTTGGTTTCAGAGCTTATCTGGGTTCTAGAGCTGGGCTTGGTCTCGGAGCTGGCCTGGACTCTAGA
ACAGGGCTTGGTCTCAATACTGGGCTCTGTCTCAGAGCTCATCTGAGTTCTAGAGCTGGGCATGGTCTCAGAGCTCATCT
GGGTTCTAGAGCTGGGCTTGGTCTCGGAGCCCATCTGAGTTCTAGAGCTGGGCATGGTCTCAGAGCCCATCTGAGTTCTA
GAGCTGGGCTCGGTCTCAGAGCCCATCTGAGTTCTAGAGCTGGGCTCGGTCTCAGAGCCCATCTGAGTTCTAGAGCTGGG
CATGGTCTCAGAGCCCATCTGAGTTTTAGAGCTGGGCTCGGTCTCAGAGCCCATCTGAGTTCTAGAGCTGGGCTCGGTCT
CAGAGCCCATCTGAGTTCTAGAGCTGGGCATGGTCTCAGAGCCCATCTGAGTTCTAGAGCTGGGCATGGTCTCAGAGCTC
ATCTGGGTTCTAGAGCTGGGCTTGGCCTCAGAGCAGGCCTGAGTTCTAGAACAGGGCTTGGTCTCAGTGCTGGGCATGGT
CTCAAAGCTCATCTGAGTTCTAGAGCTGGGCTTGGTCTCAGGGCTGGCCTGAGTTTTAGAACAGAGCTTATTCTCAATGC
TGGGCTTGGTCTCAGAGCTGGCCTGGGCTCTAGCACATAGCTTGGCCTCAGATCTGGGCTGGATTCTAGAGAAGGGTTGG
GTTCTGGAGTTGGGCTGGGGCTCCAAATTGGATTGGGTTCTAGAGAGGAACTTGTCCTCCGATCTGGGCTGGGTTCTGGA
GCAGGGCCGGGGCAGGGGGCTGGGCTCCGGGCTGGGGCTGTGCTGGGCCCCCCAGGGCAAACTAGCCACAGGCCTCTCT
GGCCTCGCCGATGGCGGCCCACACTTCCACCACAAACTCGTCGGGGAAGGCGAACTCGCCCAAGACGGAGTCTAAACAGC
GTGCAAACTCCTGGGCGCTCGCTGTCTTCTTGGACGTGTCTCCACCATGGTGGACGCTGCGGGCCGTGCCAGAGGGGATGTC
AGTGAGGGCACTCCCCGCGCCCAACTTCCCGCTGGGACCCCTGCCTCCTCCCGTAGGCTTCCCTCCCCCCAGGGCCCCCT
GGCTTACCCTGGCCCCTTACCCAGCTCGGGGTCCCGAATGCCCATGTAGCTTTCCAGCAGGTCATCAACCTTCCGAGATG
CCTCCTCCTCAAACTCACTGGGCTGGGGGCACACGGGAGGGAAGTCAGGGGTGAAAAGCTTTGGAGCCCTCCAGCCCAGC
CCCCTCCCGACCCCCACCCCTAAGGGAAGAGTAGACTGGACCCTCACACGAGTCAGTAGTGCTGGTACCAACATCATTGC
CCCACTTTTCAATGAGGACCCATTTTGCAGGCAGAGAAACTGAGGCTCCGAGGAGAAGTTCTTACCCAAGACCGCACTGA
CTCAGTGGAGGAGCTGAAGGTGCAGAAGCCTGGGGAGAGCCCCTCCCCTTCACTCACCGCTTCCTCCACTGTGGCAGCCC
CCCCAGAACGAAGCCGCAGGGTCTCCCTCCCGCTGGTCACTTTCGCCTCTACTGGACATTTGCTGGACCGACTTCTCTGG
CCAATCATATCTGGAGGGAACAGAGTGAGGGATGGCCATGGGTTCTGGGTGGAGCCACATCTTTGTAAACCCTGGGAGGC
CAACAGCCGAGCCTTTCTAGAATCTTCCTCCCCTTCAAAGTCCTCCTTACATCTGTACGGCAAGTCTCCAGAGACCTATT
CGAGATTCCACACTTCGCATCCCCAGCAAACTAAATCACAAGTCCAGAATGAGGTGTTGTAATGGAGCAGGTTTAGAACT
AACATCCCAGTTCCATGCCCTCACTCTCCCTCTTCTAGCGACTTTCTCTAGAAGGGACTGCTTGGGACCCTCCTTGTGGT
AGGATCCTCAATCTAGATATGTTCTTGTCTGGGTTCCAGAACCCGGCTTAGTGTCAGGACTGAGGTGCTAGAAACACACT
TAGTCTCAGAGCTGGCCTGGGCTTAAGAGCAGGGCTTGGTCTGAGAGCTGGCCTGGGCTCTAGAGCTGGGTTTAGTCTCA
GACTAAGCTTGATTTTTTTTTTTTTTTTTGAGATGTTGTCTTGCTCTGTCACCCAGGCTAGAGTGCAATGGCACGATCTC
GGCTTACTGCAGCCTCCACCTCCCAGGTTCAAGCGATTCTCCTGCCTCAGCCTCTCGAGTAGCTGGGATTACAGGCACCT
GCCACCATGCCCAGCTAATTTTTTGTATTTTTAGTAGAGACAGGGTTTCACCATATTGGCCAGGCTGGTCTCGAACTCCT
GACCTCGTGATGCACCGCCTTGGCCTTCCAAAGTACTGGGATTACAGGCTTGAGCCACTGCACCCAGCCAGGTTTTTTT
TTTTTTTTTTTTGATACGATGGATCACCTGACCTCAGGTGATCCACCTGCCTTGGCCTCTCAAAGTGCTGGGATTACAGG
TGTGAGCCACCACGCCCGGCCTAAACTCTATTTTTAGGCATGGCTTTTGGAGTTCACAGCCACGGCAGGGCAGATTCTAG
AACCCCATACAGCTCACTCTCCCTGGGACGTCTCTCCATGTGTGCTGGGGCACTTCTAGAATCTCTGTCTCTGTTCTATA
GAACAACGCTATCCAAAGGGCCAACCCATGGCTACATGAGGAGAATGTGCTAGTGTGTAAATCAATACACTGTTTTCTTC
ATTGAGAAAGTCTAGTTATGAAAAAAAATGGGCAGGGTGCAGTGGCTCATGCCTGTAATTCCAGCACTTTCGGAGGCCGA
GGCGGGCAGATCACCTGAGGTCGGGAGTTCGAGACCAGCCTGCCAACATGGAGAAACCCCATCTCTACTAAAAATACAAA
ATTAGCCAGGCATGGTGGCACATGCCTGTAATCCCAGCTAGTTGGGAGGCTGAGGCAGGAGAATCACTTGAACCTGGGAG
GCAGAGGTTGTGGTGAGCCGAGATCACACCATTGCACTCCAGCCTGGGCAACAAGAGCAAAACTCCGTCTCAAAAAAAGA
AAAAAATTAAAAATTAGCTGGCACGGCCAGGCGCGGTGGCTCACACCTGTAATCGCAGCACTTTGGGAGGCCGAGGTGGG
CGGATCACGAGGTCAGGAGATCGAGACCGTCCTGGCTAACACGGTAAAACCTCATCTCTACTAAAATACAAAAAATTAGC
CGGGTGTGGTGGCGGGCGCCTGTAGTCCCAGCTACTCGGGAGGCTGAGGCAGGAGAATGGTGTGAACCCGGGAGGCGGAG
CTTGCAGTGAGCTGAAATCTAGCCACTGCACTGCAGTCTGGGCGACAGAGCTAGACTCCGTCTCAAAAAAAAAAAAAGAA
AAGAAAAGAAAAAGAAAAGAAAAGAAAAAAGAAAGGAAAAAAAAAGAAAAGAAAGAAACTTTTTTCTCATATTCTCA
TTCTCTACAATCCACCATGACCTCAAAGCACTGCTTTAAAAGTTCCCAGGCTGCGCGCGGTGGCTCACGCCTATAATCCT
AGCATTTTGGGAGACTGAGGCGGGTGGAGCACTTGAGGTCAGAAGTTCGAGACCAGACTGGTCAAGATGGTGAAACCCCG
```

```
TCTCTACTAAAAATACAAAAATTAGCCAGGCGCAATGGTGGGTGCCTGTAGTCCCAGCTACTCGGGAGGCTGAGGCAGGA
GAATCGCTTGAACCCGGGAGGTGGAGATTGCAGTGAGCTGAGATCGCGCCATTGCACTCCAGCCCAGGCAACAGAGTGAG
ACTCCGTCTCAAAAACAAACAAACAAACAAACAAACAAAAACAAGTCTCCCAGCCTGGCTCCAAAGTGGTTCTGGACGCG
TCCTCCTCCAACTCCACTTTACCCAGTCTCTCCCCCACCCCTCCCCCACAGGCTGACTCCATAACAATCTATTAATAGAA
ACTACCTACGAGTTTCTGATACCCTGCCAGGAGCTCCTTGGCACGCAGCGGGTGCACCTAGAACCTTCCAGCCCGTCCCA
TCCCCGACCCTCCTCCACCCCAGGCATCCGTAGAACGCACTTCGGGAAGAGCTCCCGCCAGCCCACCCGCCTCACCGAAG
GCCCTCTTGGGCTGCACCAGGCGCAGGGTGAAGGGCTGGGACTTGGGCAGCTCCCGGAGCATCTTGGCCACCTCGTAGTG
GCGGCAGCCCACAATGGAGTGGTCGTTGATGGCTTCGATGCTGTCACCCACGCACACTGCCTCGATCCGGTTGATGATAC
TGCCTTCCTTGATTCTCTGCAGGGATCCAGCGTCGCTGGCAGGGCAACCCCAGCCACCCGCCTCCAATCCGCAGAACCCA
CGTCGACCCAGAGTGGGGGGGGCAGTTGCTGGACCCCAGGAAGCCACCCGCCCACCCCCTCCCGGGCACCTTGATGAAGG
CGTAGCCAGCCCCGTTGTCCGTGATGGTCAGCCCCAGAGCATCCTCTGTCTTAGTGACCTCCACCTCCTTGGTCTCGCCT
CGCACGTGGGCAAAGATGAAGTCCTCCAGGCCTATCTGACCCCCCAGGAGCTTCTGCATGTCCACTTTGTGGCTGTTGAG
GGTGCAGAATAAAATCTTCCCGGGGGAGAAGGAGCCTAGAGTTAGCCAGGGCATGCATCCCCCCACGCAGCCACCCTGTC
TGCGCAGGCCCCTGCCCCCAGCGACCAGGATCCCAGCCCATGGGAAAGGGCAGGGCGTGGGACCCCAGAACAGAGAGAG
GAGGGAGAAGGAGACTTCCAGCAGGTGGAGACCCCTCTCCATACCCCGGGACCTCCAGCCCCCAGACCTCCAGGTTTAAT
CTGCTGGCGCCCCTGGGGGCTGCAAACCAAACGAACCCGGATTGGAAAAACGCCCAGGAACCCCCAACCCCACGGCGG
CCTCAAACTTCCCATCCCTAAGGGGGCCATCTGGAACCCCAGACCCCAGCTACGCCCAGGAAGCACAGAGGCGCTTCCCC
GAGGGCTGGGGTCCGCTCCAAGCTAGGTCTGCGCAGAGGATTTTCTGGAATATCTAGGGGTCCGCTAGTCCTAAGACCTG
CCCATCTTTTGCGCAGAGCGTGCAAAGGGGGCCCCTGGGAGTGTGGCACCCCGGGATCTGAGGGCTGGGTCTCTGAGATC
CGGCGATTAGGCGGCCACCCCGACGTCTGGGATCCGGGGATCTGGGTCTCCCGTCCAGGGTCCAAGCTCCCTACCCCTC
ACCCCATCAGGCGGGTGGTCTTGGGCGCCGGTGTCCGGGCTCCTTACCTCGGTGGGCGCGATCCCGAAGGCTTCGGCGAT
CTTGGCGTACAGCTCGCGGACGTTGGTGAAGCCCTCGATCTTGCCCGTGGGGCTCCCGTGCGCCAGCTGCGTGCGGAAGA
CGAGGCGCGGGCGGGCGCGGGGCGCGGCCGGGGCTCCGAGGGCGCGGGCGGGGCGCAGACGCGCGCGGGGTCTCGGTC
CCCCGGGCCTCCCGGGCCGCTGCTCCCTCCATGGCGGGAGAAGTTCGGCCACCCACCCGGGCCCTCGCCGCCGCCGCCGG
ATCCGCCGCTTCCTGCAGCCTCCCCTCCCCCGGCCCAGGGCGCGGACAGCCGAGACGCCCGGGTAAGGGGATCTGGGAACC
CGAGCCTCCCGGGAAGGCGACACCGCGGGGTGCGCAGGGGTGGGTCGGGCCCCAGGTGTCCGGGCCCTGCCAGGGCCAGC
TGTGCCCTCCCCGGGGATACCCGCAGTCCGGGGGGGCCTGAGCTCAGGACCCTGGAAGAACAGGGACCACAAATAAGGAC
AGGGACAGGCCTGGACGCGGTGGCTCACGTCTGTAATTCCAGCACTTTGGGAGGCCGAAGCGGGCAGATGACCTGAGGTCA
GGAGTTCAAGACTAGCCTGGCCAACATGGTGAAACCCGTCTCTACCAAAAATACAAAAATCAGCCGGACGTGGTGGCGG
GCGCCTGTAATCCCAGCTACTCGGGAGGCTGAGATCTCTTGAACCCGGGAGGCGGAGGTGGCAGTGAGCTGAGATGGCGC
CATTACACTCCAGCCTGGGCAACAAAGCGAGACTCCGTCGCAAAAAAAAAAAAAAAAAAAAAAAAAAAAGGACAGGGACAGC
TGAGGGTGCTGGAGCACTCACCCTCTGCTAGGCGAGGTCATTGTTTTATTTTATTTTTATTATTTTTATTTATTTATTTAT
TTTGAGCTGGAGTCTCGCTCTGTCACCCAGTTTGGAGTTCAGTGGCACGATCTCAGCTCACTGCAACCTCTGCCCACCAG
GTTCAAGCAATTCTCATGCCTCGGCCTCCCCAGTAGCTGGGATTACAGGCATATACCACCATGCCCGGCTAATTTTTTTG
TATTTTTAATAGAGATGAGGTTTCACCATGTTGGCCAGGATGGTCTTGAACTCCTGACCTCAGGAATCCACCCACCTTAG
CCTCCAAAAAAGCTGGGATTACAGGCATGAGCCACCACGCCCAGCCTTTGGTTTGTTTGTTTGATTTTGGAGACGGAGTC
TAGCTCTGTCGCCCAGGCTGGAGTGCAATGGCGTGATCTCGGCTCACTGCAACCTCCACCTCCTGGCTT
```

HUMAN mRNA SEQUENCE : hR6-034.1 (Seq ID No: 59)

```
GGGCCCGCCCCTGGTCACAGCCAGACTGACTCAGTTTCCCTGGGAGGTCCCGCTCGAGCCCGTCCTTCCCCTCCCTCTGC
CCGCCCCCAGCCCTCGCCCCACCCTCGGCGCCCGCACATCTGCCTGCTCAGCTCCAGACGGCGCCCGGACCCCCGGGCGC
GGGATCCAGCCAGGTGGGAGCCCCGCAGATGAGGTCTCTGAAGGTGTGCCTGAACCAGTGCCAGCCTGCCCTGTCTGCAG
CATCGGCCTGATGGGGTGGTGACTGATCCCTCAGGGCTCCGGAGCCATGTGGCCCAACGGCAGTTCCCTGGGGCCCTGTT
TCCGGCCCACAAACATTACCCTGGAGGAGAGACGGCTGATCGCCTCGCCCTGGTTCGCCGCCTCCTTCTGCGTGGTGGGC
CTGGCCTCCAACCTGCTGGCCCTGAGCGTGCTGGCGGGCGCGCGGCAGGGGGGTTCGCACACGCGCTCCTCCTTCCTCAC
CTTCCTCTGCGGCCTCGTCCTCACCGACTTCCTGGGGCTGCTGGTGACCGGTACCATCGTGGTGTCCCAGCACGCCGCGC
TCTTCGAGTGGCACGCCGTGGACCCTGGCTGCCGTCTCTGTCGCTTCATGGGCGTCGTCATGATCTTCTTCGGCCTGTCC
CCGCTGCTGCTGGGGGCCGCCATGGCCTCAGAGCGCTACCTGGGTATCACCCGGCCCTTCTCGCGCCCGGCGGTCGCCTC
GCAGCGCCGCGCCTGGGCCACCGTGGGGCTGGTGTGGGCGGCCGCGCTGGCGCTGGGCCTGCTGCCCCTGCTGGGCGTGG
GTCGCTACACCGTGCAATACCCGGGGTCCTGGTGCTTCCTGACGCTGGGCGCCGAGTCCGGGGACGTGGCCTTCGGGCTG
CTCTTCTCCATGCTGGGCGGCCTCTCGGTCGGGCTGTCCTTCCTGCTGAACACGGTCAGCGTGGCCACCCTGTGTCCACGT
CTACCACGGGCAGGAGGCGGCCCAGCAGCGTCCCCGGGACTCCGAGGTGGAGATGATGGCTCAGCTCCTGGGGATCATGG
TGGTGGCCAGCGTGTGTTGGCTGCCCCTTCTGGTCTTCATCGCCCAGACAGTGCTGCGAAACCCGCCTGCCATGAGCCCC
GCCGGGCAGCTGTCCCGCACCACGGAGAAGGAGCTGCTCATCTACTTGCGCGTGGCCACCTGGAACCAGATCCTGGACCC
CTGGGTGTATATCCTGTTCCGCCGCGCCGTGCTCCGGCGTCTCCAGCCTCGCCTCAGCACCCGGCCCAGGTCGCTGTCCC
TCCAGCCCCAGCTCACGCAGCGCTCCGGGCTGCAGTAGGAAGTGGACAGAGCGCCCCTCCCGCGCCTTTCCGCGGAGCCC
TTGGCCCCTCGGACAGCCCATCTGCCTGTTCTGAGGATTCAGGGGCTGGGGGTGCTGGATGGACAGTGGGCATCAGCAGC
AGGGTTTTGGGTTGACCCCAATCCAACCCGGGGACCCCCAACTCCTCCCTGATCCTTTTACCAAGCACTCTCCCTTCCTC
GGCCCCTTTTTCCCATCCAGAGCTCCCACCCCTTCTCTGCGTCCCTCCCAACCCCAGGAAGGGCATGCAGACATTGGAAG
AGGGTCTTGCATTGCTATTTTTTTTTTTAGACGGAGTCTTGCTCTGTCCCCCAGGCTGGAGTGCAGTGGCGCAATCTCAG
CTCACTGCAACCTCCACCTCCCGGGTTCAAGCGATTCTCCTGCCTCAGCCTCCTGAGTAGCTGGGACTATAGGCGCGCGC
```

CACCACGCCCGGCTAATTTTTGTATTTTTAGTAGAGACGGGGTTTCACCGTGTTGGCCAGGCTGGTCTTGAACTCCTGAC
CTCAGGTGATTCACCAGCCTCAGCCTCCCAAAGTGCTGGGATCACAGGCATGAACCACCACACCTGGCCATTTTTTTTTT
TTTTTTTAGACGGAGTCTCACTCTGTGGCCCAGCCTGGAGTACAGTGGCACGATCTCGGCTCACTGCAACCTCCGCCTCC
CGGGTTCAAGCGATTCTCGTGCCTCAGCCTCCCGAGCAGCTGGGATTACAGGCGTAAGCCACTGCGCCCGGCCTTGCATG
CTCTTTGACCCTGAATTTGACCTACTTGCTGGGGTACAGTTGCTTCCTTTTGAACCTCCAACAGGGAAGGCTCTGTCCAG
AAAGGATTGAATGTGAACGGGGGCACCCCCTTTTCTTGCCAAAATATATCTCTGCCTTTGGTTTTATTTTCCTTTGGGTC
CAGAAGTTTTCAATCTCTGGAGTTTGTGCATTGGGCCTCACCCGGAGCAGACGAGGGCAGGGCTGGGAAGGATGGGGAGG
GACATAGAGGATTCGGTTCCTCCCCCACCTTGTTTCTTGAGTGTTTCCTAATCACTTGATGGGTGGTACCACCTAGAATT
CTTCCAGCAGCCTGTAATGGGAGGGCTACAGGTTAGCCCTGGAACCTGCAATCAAACCACCTTTGAATCTGTGTGTCATT
AAAAGTAGATATAAATGGGCCG

HUMAN PROTEIN SEQUENCE : hP6-034.1 (Seq ID No: 60)
MWPNGSSLGPCFRPTNITLEERRLIASPWFAASFCVVGLASNLLALSVLAGARQGGSHTRSSFLTFLCGLVLTDFLGLLV
TGTIVVSQHAALFEWHAVDPGCRLCRFMGVVMIFFGLSPLLLGAAMASERYLGITRPFSRPAVASQRRAWATVGLVWAAA
LALGLLPLLGVGRYTVQYPGSWCFLTLGAESGDVAFGLLFSMLGGLSVGLSFLLNTVSVATLCHVYHGQEAAQQRPRDSE
VEMMAQLLGIMVVASVCWLPLLVFIAQTVLRNPPAMSPAGQLSRTTEKELLIYLRVATWNQILDPWVYILFRRAVLRRLQ
PRLSTRPRSLSLQPQLTQRSGLQ*

HUMAN mRNA SEQUENCE : hR6-034.2 (Seq ID No: 61)
GGGCCCGCCCCTGGTCACAGCCAGACTGACTCAGTTTCCCTGGGAGGTCCCGCTCGAGCCCGTCCTTCCCCTCCCTCTGC
CCGCCCCCAGCCCTCGCCCCACCCTCGGCGCCCGCACATCGCCTGCTCAGCTCCAGACGGCGCCGGACCCCCGGGCGC
GGGATCCAGCCAGGTGGGGAGCCCCGCAGATGAGGTCTCTGAAGGTGTGCCTGAACCAGTGCCAGCCTGCCCTGTCTGCAG
CATCGGCCTGATGGGGTGGTGACTGATCCCTCAGGGCTCCGGAGCCATGTGGCCCAACGGCAGTTCCCTGGGGCCCTGTT
TCCGGCCCACAAACATTACCCTGGAGGAGAGACGGCTGATCGCCTCGCCCTGGTTCGCCGCCTCCTTCTGCGTGGTGGGC
CTGGCCTCCAACCTGCTGGCCCTCAGCGTGCTGGCGGGCGCGCGGCAGGGGGGTTCGCACACGCGCCTCCTCCTTCCTCAC
CTTCCTCTGCGGCGTCGTCTCACCGACTTCCTGGGGCTGCTGGTGACCGGTACCATCGTGGTGTCCCAGCACGCCGCGC
TCTTCGAGTGGCACGCCGTGGACCCTGGCTGCCGTCTCTGTCGCTTCATGGGCGTCGTCATGATCTTCTTCGGCCTGTCC
CCGCTGCTGCTGGGGGGCGCCATGGCCTCAGAGCGCTACCTGGGTATCACCCGGCCCTTCTCGCGCCCGGCGGTCGCCTC
GCAGCGCCGCGCCTGGGCCACCGTGGGGCTGGTGTGGGCGGCCGCGCTGGCGCTGGGCCTGCTGCCCCTGCTGGGCGTGG
GTCGCTACACCGTGCAATACCCGGGGTCCTGGTGCTTCCTGACGCTGGGCGCCGAGTCCGGGGACGTGGCCTTCGGGCTG
CTCTTCTCCATGCTGGGCGGCCTCTCGGTCGGGCTGTCCTTCCTGCTGAACACGGTCAGCGTGGCCACCCTGTGCCACGT
CTACCACGGGCAGGAGGCGGCCCAGCAGCGTCCCCGGGACTCCGAGGTGGAGATGATGGCTCAGCTCCTGGGGATCATGG
TGGTGGCCAGCGTGTGTTGGCTGCCCCTTCTGGTCTTCATCGCCCAGACAGTGCTGCGAAACCCGCCTGCCATGAGCCCC
GCCGGGCAGCTGTCCCGCACCACGGAGAAGGAGCTGCTCATCTACTTGCGCGTGGCCACCTGGAACCAGATCCTGGACCC
CTGGGTGTATATCCTGTTCCGCCGCGCCGTGCTCCGGCGTCTCCAGCCTCGCCTCAGCACCCGGCCCAGACGGAGTCTCA
CTCTGTGGCCCAGCCTGGAGTACAGTGGCACGATCTCGGCTCACTGCAACCTCCGCCTCCCGGGTTCAAGCGATTCTCGT
GCCTCAGCCTCCCGAGCAGCTGGGATTACAGGCGTAAGCCACTGCGCCCGGCCTTGCATGCTCTTTGACCCTGAATTTGA
CCTACTTGCTGGGGTACAGTTGCTTCCTTTTGAACCTCCAACAGGGAAGGCTCTGTCCAGAAAGGATTGAATGTGAACGG
GGGCACCCCCTTTTCTTGCCAAAATATATCTCTGCCTTTGGTTTTATTTTCCTTTGGGTCCAGAAGTTTTCAATCTCTGG
AGTTTGTGCATTGGGCCTCACCCGGAGCAGACGAGGGCAGGGCTGGGAAGGATGGGGAGGGACATAGAGGATTCGGTTCC
TCCCCCACCTTGTTTCTTGAGTGTTTCCTAATCACTTGATGGGTGGTACCACCTAGAATTCTTCCAGCAGCCTGTAATGG
GAGGGCTACAGGTTAGCCCTGGAACCTGCAATCAAACCACCTTTGAATCTGTGTGTCATTAAAAGTAGATATAAATGGGC
CG

HUMAN PROTEIN SEQUENCE : hP6-034.2 (Seq ID No: 62)
MWPNGSSLGPCFRPTNITLEERRLIASPWFAASFCVVGLASNLLALSVLAGARQGGSHTRSSFLTFLCGLVLTDFLGLLV
TGTIVVSQHAALFEWHAVDPGCRLCRFMGVVMIFFGLSPLLLGAAMASERYLGITRPFSRPAVASQRRAWATVGLVWAAA
LALGLLPLLGVGRYTVQYPGSWCFLTLGAESGDVAFGLLFSMLGGLSVGLSFLLNTVSVATLCHVYHGQEAAQQRPRDSE
VEMMAQLLGIMVVASVCWLPLLVFIAQTVLRNPPAMSPAGQLSRTTEKELLIYLRVATWNQILDPWVYILFRRAVLRRLQ
PRLSTRPRRSLTLWPSLEYSGTISAHCNLRLPGSSDSRASASRAAGITGVSHCARPCMLFDPEFDLLAGVQLLPFEPPTG
KALSRKD*

Table 11

MOUSE GENOMIC SEQUENCE : mD6-131 (Seq ID No: 63)
TTAACATTTAGAAAAGGTGGAGCAAGAGGACTAGGAGTTCAAGGTCATCCTTGGCTACCCAGGTGATTAATAGCTAGCAC
AGGCTACAAAAGACCCTGTCTCAAGACAAACACAAAGAAGCAGCTTGACTCTTTCATATTCAGCCATGTGAACACTTAGC
ATTTGCCCCTTTAACTATCTGAAGACTTATCTGAAGAGGTACCAACTTGGAAACAAAGATCCAACCTCACCCCCATGTCC
TTCATACTCCATTTGCTTGCTTGGGTCCATCCATCCATCCATCCATCCATCCATCCATCCATCCATCCATCCATCCATCC
ATCTTCTATCTATTTAGAAATATAATTTCATTATTTATCTTTATCTGGGATGGAACTAGCAATGGAGACTAGGCTAGCCT
CAAACTCACAGAGATCCTTCTGCCTTTTCCTCCTGAGTGCTGGGGTTAAAGGCATACACATGTCTAGCTAAATTTGGCAG

```
TCTCTTGATATTGGACATCACAGTGTATAGAACTGGTATATATTTGTGTTATTTGAAAACTACTCACTCTAATGTACTTT
GTTACATTACCAAGAATAGTCTAACACAATCCTAATCTCAATTTTGATGAAGTGAGAGAGAACAGGTGGTATCATAGGGT
TTTACTGCTGTGAACAGACTCCATGACCAAGGCAAGTTTTATAAAGGACATTTAATTGAGGTTGGTTTACAGGTTCAGAG
GCTCAGTCCAGTATCATCAAGGTAGGAACATGGCAGCATCCAGGCAGGCATGGTGCAGGAGGAACCAAGAGTTCTACATC
TTCATCTGAAGGAAGCTAGCAAAATACTGACTTCCAGGCAGCTAGGACTAGGGTATTAAAGCTCACACCCACAGTGACAC
TCCTATTCCAACAAGGTCACACCTCTAAATAGTTCCACTCCCTGGGCTGAGCATATACAAACCATCATAGGTAGGAAGAG
AGAGAGAGAGAGAGAGAGAGAGATTATTTTTCATGACTTACCCTGAAAATCATTAGTATAATTTCTTCCTTATGCTGGTT
AGAAAAGGTCCAGGTTCAAGTAGAGGCTCCACAGAGTCCAGCTCTGGTGGTCAAGTATAACATTTATATGTAAGAAGGGG
GCATGGGGAGAAAGGGAGTCAGATACAGTGTTCACAGACGGGAAGAACTTCCCCCTAGAGAGCTACATTCTGCTGAATG
CTTAAAGTGTGAACACTGGCTGAAGCCGCTTTCATACAAGTAACTTCTCAAGGAATTAATTTTTCCATATGCCCTTAGCT
AGAAAACTTCTAGACAGTTCCACCAGCAGTGTAGCACATATACGATGATAAAGAGCCAGGACATGAAGCTCCAATGAGGA
GAGAGATAAGGGGAACCTCAGGATGACAGGCAGGTAGCAGCCAGGACTGCAGAAGTCAAGGCCTCCAGAAAGGTGTCTGC
ATGGATTTGGTGTGTTTTCTCATGTATTTGAAGGAATACTCTATGACTGTGTATTAACTGATTGTAAGTATACAGGAAAT
TGAGCAAATACAAGAAGATGCAGGGTCAGCAAAATGTCTCAGTGGGTGAAGGCACTTGCTGACATGCCTAACCACCTGGG
ACCCACATGGTGGAGGGATAGAACCAACTTTCCCATTTACTATCTGACTTCTACTTGTTCTCCCCATTACAGACAGACAG
ACAGACAGACAGACAGATACACACACAGAACTAACTAACTAAATATATCTTTTAAAGGGAAAGGAATATACAAAAAGT
ATATTTTGTACATACTCATGGTAGCACATATCCATGTTTACAGCAATTGGGAGTCTGAGGCAGGAAGACTGCTGTAAGCT
TGGATGCTGGCCTGGGCTATAAAGTAAGTATCAGGCCTGACAGAGCTGTATAACAATTTATTTTTCACATTAAAACAAAA
AGGTATATGTCTTCCACACGCCTGTGAGAGCAGTGTTTGTGAGGGGGAGGGGGGATAGAAAGATCAGCAAAGTTGAAACA
GACATTCTTCTACCCGAATAATAGGAGTTCCGGAGATGGGAAGATGAAAATAACAAAGACATGAGTTCAGACAGTGTTCA
GAACTGAAAAGGTGAAAGGGGCTGGGGAAAAGTTACATTATTCTGGAACACTGAGAAAAGAAGAATCCACCTATAGACAA
GAAGTAAGGTAGATATGTCACATTCTGCATTCGGGAAGCTTTAAAACTAGACATTAAAGATAAAATGAAACCTTCAATTC
TCAGGGATATAGATATAGTTATAGATATGGATATGGATATAGATATCAGACATGTAGGCTTTGGAACATTTACAGCACAA
GTATTGTCTTTAAGAGTAAATTAGTCAACATTTCATCATGAAACTTTTTTTAGACATTACAGCATGGCAAAATAAAATC
TATAAATAACATTAAATACTACTAATACAACTATAGACATATAAACACAGGACTAAGGGATTTTTCCCAATAGCCATCAG
AAGCATCAACAGGTCTCTGCCTACGGGAGGTGGGGAGCTGAGGACCAGCGAGCACCCAAGAAGAGACACTCGTTTTTACG
AATCAAGTACTCTTTCATGTTCTCTAAATGATGTGCACATATTGGCTTGTTGAAGAATGGTTTTGTCAAGGCCGGAGACA
AGACTCCATGGCTACAAGTACTTGCTGCTAAGTCTGACTAATGAGTTTGATCCCCAGGAATGACTTGGTTGCAAGGAGGC
AATTGCCTTCCTCAAGCTGCCGGCTGACATCCCTGTGCATGCTGTGGCATCCTTGTCCTTCCCAACAACATGCGCATCTA
AGAAAGGTAATGAAACGACTGTTCTAAAATCAGAAAGAAGTGAATGAGAAGTTGGAGATGCGATCATGACAGCAACAGGA
GATGCGAAGATGCTTTCTCTGTTGTTGAGGTTGCCATAGGAAGCCCTAAGTCTTAAAATGACTTAAAGAGAAGGAATATC
ACCCTCCGGGAAGTTGATGGAGGAGCTGTGGTGTGACTCACTGGCAGAGAGCTTAGTTAACATCAGAGAGAGCCCTGAGT
TTGAGCACAGCACAGCTTCGCTTACCACCAACTGTAGATACAGTAAGTAAAGGAAACCGTGCAGAGACATAGGCATGTAG
CACAAAACTGGCAACAGTAAGGCTGAGCCCCAAGTCTGTTTTCAGGGCTCTACCATACTTATCTATGCAGTTTGGAGTGTT
GGCAGCAAGAAGGTTTATGGTGAACCCTTGGATAAGCAGAAGACAATGAGGTTTTGGTATAAGAAACACATAGATGTTG
ATACAACTTGAAGGCTAAAAGTCATCAGAAACCAATCTAGACATGCTGGCACATTCCTGTAACCCCAGCACTCAGGACCG
AGGCAGGAGGCACTGGAGGCTGGTGTAAGAATCTGTGTCAGAAGAAAAGAACACACACACACTCTCTCTCCACCCCCA
ACACACACACACACACACACACACACACACACACACACACACACACACACACACACTCACACTGAGAGCGAG
TAAGCGAGCGAGCAGGAGAGAGACAGAGACAGAGACAGAGACAGAAACAGGGAGAGAGACACAGAGACACAGACGGGGAG
ATAGATAGAGACAGATACAGAGAAACTAAAAATATTAGAAAGCAAGCTGTGCCAGAGGTCTGGGGAGCAGGCTCAGTGGT
TAAGAGCACTCACTGCTCTTCCAGAGGACACAAGTTCTGTTCCCAGCACCCACGTGGGACAGCTCAGGACATCCTGTAAC
TCCAGCTCCAGGATATCTGATGCTTTTCTCTGGCCTCCGTGTGAACCCCTACATACGTGTAAATAAAACCAAAAATAAAT
CTTTGGGAAAAGAAATAACCAAAAACTGTTTACAAGAGGGATTGCGCAATGGAGCCGGGTCTTAGGGTCTTAGAAAGCTT
TGAGGGAGCCAGTGAGGTGGTTGATAGGGAAAGACTTTGCTTTTAAAGAGTGAAATGGGTTTCGCTTGTCAAGCAGGTAG
AACAGACCTGCTGGGGGCAGCTCTGTTAATTGAGAGTCAGAGAGTCAGGAAAGGGAGGTTGTCATACAGTTTTCTCTGTG
AGAAATGGGAGACTGGTTGTTTGCCTATGAATAAATGAGAAAGAACTCGGTTGCATTTCCCTGGGTCTTGAGAGGCGGGA
TGAGTTGAAAATCAACCAGAATAAATAGAAAAGAGAGTAGTCAGAAATTAACAAAGAGAAGGCATAATCAACTCCTTTAC
CTTTTATTCCCCAGATGGACGAGCATTTACTTATTGATCTATACTACATAGCTTAAGGATCCTGTGTCATTTCATCTGAC
AGCTCTTAAGGAAAAACACCGAAGGGCAGGGAAGCCACCTTGAAATTAGTACCCTGAAGTGAAGCCACCTTAACGGGAAC
AGGACATACAGCGGAAGTGTGTCCTTAAGTTTGTCCTTAGTAAGGTAGGAATGCAGGGAGTAGCTTCCAGCCAGAAAAAG
GGCTAGACTTCTGGCCAGGAATTAGCTGGAGGGCATAGTTCTTAGAGCTGTCTTCTACCTGTGGGTCTTAAAGGGCGTGG
TGCTAGAAACCTGGAATTTACCAGAACTCGTCGTCTAGGAACAAGCCTTGCTGGATTTTACCATTTGCCCAACGGTCTTA
CACATTTTACATCCCAGAGATGTATAATAATGGTGACCATTACCAAATCACCGTCTGCAAGACATCTGTGCTCTTTACAA
TACTTGGCCAGGCCTGCATGTTGTCCTCAGTGATGGTCAGAAAAGATTTCCCACAAGTAATTCTTTAGTACACTTGAGGT
TTATCTAAGTGGTTTGGTAGTGGTGGTTATGATTCTACAGTCTGTCTATGGTCATGTCTCACATTAACCAACACGTTTAA
TATATCTGGAGCCCATGTTTTTCTTGTTTATAAAATTAGAATAATAATAGTATGTAACTAATATATTTACTGAAAGTATA
AGTTTTCTCTAGGTCTGTAATGTGGGCACTGAGAAGCGTCAAGGAGGTAGCTAAGTTGGTAGAGTACTTGCCTAGCTTGC
ATAAAGCCCTGGGTTTGGTCCCCTGTTCTGTTTAAACATAAAGCCAGGTGTGATGGTGCATACCTGCAATACCAACACTA
GAAGTAGAAGAAGGAGTATCAGAAACTGAAATTGTGTATGGTGAATATGAGTGCATTTTGAGGCCATCCTGGGCTACATA
AGATTCTAATACCTATAGTTATGTAGACCTATATCTACACATGGGTCATATGGACTCTTGGTTGTCAATCTGGAATCAGC
TAAAACCCAAGCAGCTAGGCATGCCTGAGAGGGGTTTTCTTGACAGGATCATTTGAAGTGAGAAGACCCACCCCAAATTT
```

```
AGGGCATACCGTTTGGTGGCAGCCCCCCTAAACCGACATGGAAGAAGGAAGCTTTTGCTTTTTGCCTGCTCACCTTCACT
CTAGCTGACAAGTTCATCTGCTCTGCTGCGGAGGCATCCCTTCCCCACTCCTAGGACCTACCTCTTCAAGACTTCGATGG
AGGCTGATGAGCAGCAGTTCTCTAGGACTCCAGAACCAGACTGGGGTTGATGAACCGATTGGCTACTGGATGCTTGGCTT
TTCTGTCAGGACATAGCCATTGTTGGACTAGCCAGCCAGACCACCATTAGTTCTGTTCCTTCAAAGAAACCTGACTATAC
AATGTCAGTATTTTTCAGTATTTTTTTTCCCTTCTTTCTTTTTGTTTGAGACAAGGTCTCACTATATAGCCCTGGCTAGCC
TGGATCTAACTATATAGACAAGTCTGGCTTCAGACTTACAGAAATCTACCCCACACTTGCCTCTGGAATGTTGGAATTAA
AGGCATTTGTCACTATGCGTAGCCTATCTTTTTCTTTTCACAGTATTGGGAACTGAATCCAAGGTATTGCACTTACTAAG
AAAACAGTCTACCTCTGCAATATACCCAAGCCTTTGCTTGTGTGTGTGTGTGTGTGTGATATACAGTCTCAAGTATCT
CAAGGTGGTCTCCAATTCACTATATAGCTGAGGATAACCTTGAACTTCTGATCTTCCTGCCTCTACAAATTCCTAGATTA
CAGACATGCACCATTGTGCCTGGCTATTTGTGTATATCAATGATATGGATGCATAGCTAAGTGGAATAACCCAAGCTTGA
GAGCTTCACTCTCCTCCCACAAAAGAACACACTGTATACATTAGTAATAGTTTTATGCACATGTAAAGACAAGTACAATC
AGAAGTACAAATGTAAGGAGTCCAAGTGCAGAAGTCTCCTCTGTTCAGGAAAGGAATGTGAAGTTCTAAGCTGATTGACA
GCATTTGAGCAGACAGGCAAGACAACTTAGATTTGGTGACCCATCTCTGCCGCATACTACCTGGACAATCTACAGGTCTC
TCAGAGCTGAGGGATTGCTCATGGGTAGAACGGCCCATGCTACTCCATTGCTTCATGGGACAGTGGCCATCACATCTTGG
GACAACAAAGTGCACAGTTCCCCTTTAAAGACCTCATAAATTGTTAGTGGGAGAAGGGGAAGCTAACTCCTTGCCTCTGC
AGCACTGCGATAGACAGAAGTTATCTCCTCTTTCAAATGAAACCAGTCTCAAACTCTGTCTTTTCTGGAGGAGACTGGAC
GGCTGTTATCTTGGTTTCCATATTGTTTTGCAAGATCTGTAGTTTGTGGGTAGGTAAGGTAGGAAACTGTCAATGAGTGC
TTCTAGGCAAATATGAATTAACAGCTACTTTTTTTTTTTTTTTTTGCAAGCTCATAAAATCCCAGAAGGTAGGGGTCTGTT
GTTTGTTTCTTCTATCTCTCTTGAGTCATTCAGGTACAGGGTGTGTCTGTTAATTGAAACTGCACAGCAGAGGAAGTTTT
GTGATCACATCTGCTCAGGGTGGGAAGGCAGTGAGAACAGGGGACTTGGGTGGAACCACTTTTTAACAGAAGGACAGACA
GACAAGGCATAAAAGGCCTCAATCTGCTACTCAGACAAATCTGACCAAGGCCTCTTCAGCCTCAATCTAGCATTATCTAA
CAGTAGAAAAATGTGGACAAGGTGCAATTCCTTAGATTTGGGGATCTCTTAGTTATCTACGTAGACAGGGAGAGAAGA
GGGGTCCAGCTTTAGGCAGGGGGCTGTAGGCACTGGCCAGTGAAAGGACAGGCAACCTCTTTCCCCTGTGGACATTCCTG
AACTGCTGCTGGATTGTTTAAATCTCTGAGGCCAGGGCAGCAGCCCACCTCCTCAAAGGCCTCTGAGCTTCCCAGAATCT
GGGGCACGCCTGGCTTTTCGGTCAGCTGAGAGAGGGGAACCCCCGGTCCAACATCAAGCACAGCACTGGCCCCACTTCACC
GTGCACCCGGGGAAAGCCACTTTCTGCCCACCAGACCGCAAGTTTCAAGGCTTCACCAGGATCGGAGTGGAGGGGGTGGA
GGAAGAGGAGGTGGAGGACAGAAGCTTTTCAGTACTTCGGGGCTGCTCTCCTCCGACCTTCTCAGGATCCCCTCTCTGCG
GCGTGGCTGCGGCTCTCCGGGCAGTGGCTCGGGTTTCCCCTGACCGCGTGGAAGTGGCCCCCAGGGCACCGGACCCACTCG
GGCTACACGGATCCTCAGGGCTGTGGGGTCTGGGACCAGAAAGCTCCTCTACCAAGTGCAGGCTTGTCCTCTGTCCTCTA
AGATTTTTAACGAAGAAAAGTTTTCCCAGGGATCCCCATGCCTAGTTTCAGAGTCCGTCCCGCGCTGGGGACTGCGGGAA
GCTGTGTGAATTAGAATTTAGGAGCTGCCAGGCTCCGCCCCTTCTCTTCCCTTCTCTCTGCTGGTTGGGGCGGAGGGGCT
CCTATTCATTTGGTTTTATTTTAGTCACTCTGCCCCAGATCTTTCTGGGAGGCTGCTTCTTAGCAGCCCAGACCCTGAGG
GGCCCCCTGCCGCTGGCAGAGGGCGGAGGAGTTAAAAGCATTAACCCCTCCCAGTCCCTTCCTAGAGGAGCCACCCAGCC
TCGGCGGGGCGCTCAGAGACTTCGTCTTGCAAAAGTGGTGCGGGCAGCGCAGGCAGGGCTGGCCATTGGAGTGCACCGCT
GAGGAGGGAGGGGACCCCAGCTCAGGGAACTTTGCGAGTGAGCTGGTCGCCGTCAGTCGCCGACAGCAGCAAGATGCGGA
TCGCGCGGTGTAGACCCAGAGTCCTGCGGACGCAGCTTCGTCCGGCTTGAGCGAGGTAGGGGGTTGTGAGCCTCGCAGGC
TCCGGCTATGGGGCGTCCAGGTGTCAAGAACGAACCTTTAAACACAAACGAAACAAACAAAAACTTTGAGAAAAAACAAA
GCAAAACGAACCCGGGCATCACCAAGATGCTATGGAGTCCTTCTTGACTGACTTCTTCTTGGGGCACGGAGTTTATGAAG
TTAGAAAAAAAAAGATTAATTTCCAAATTATAGAAACGACTGGGGGAGGGCATGCTAGAGAAGCTGTGATTCATTAGAAC
GGAGAAAAGGGAAGACGCTTATTATTTTTTTTTTTTTTTGAAATCCTGTAGACAAAATTATCAGGCACCACGCCAATAATT
TCCATTGCCTGAGAATGGACTGTAGCGATAAGAGTGGAGAAATGTTGAGTTTCGTAAGCTTTTGTCACAGAAATGACTAC
AATGTCTCTAGGGTCCCCTCCCCCCTCATTTGTTATAGAGTTGATCAATTACTGTGCAGACTTTAGAGTACTTATTTAGT
GCTGTGTGTCCAACTTAAGTAGTGCTGTGTGCCCTTTAGGGATTGCTAGTTCTGTTGAGAGGAGGCAATGTTGGGGGTGT
GGGGAGCGGAGGAAGTTAAAAGTGCCCATGCAAACTTAGCAGTATGTGTTTCTGGAAATTAATGTACCTGAAGTTAAAAC
AAAGTGGCGTGCTTCGTTATCGCTAGCAGGGTGACTTTCGTGTGTGTGTGTGTGTGTGTGTGTGCAAGTAAGCGTTGG
CTAAAGTTAAAACTTTCTTAAGCAATCAGTTCATGGAGAAACGTCTAGGATTTGCTGCAATCTCTTAGGAGGGGTTCTCC
GTGAATACCTTTAAACGTAATAAATGAACTTGTGGGGCGGTTGGGTGAGGCGGAGGAGTAAAGACAGAAGTTTTAAAAAG
AAGAATAGGCAGTTGCTTCACGGAGGCGCCAAGCTTTCCTGTTTACTTCAAGGCCTGGCGGCCCACACCACACCTCGCTT
TACCCTCTGACCATCGGACCCTCAGCACACCTGCTCCTAGGGAGTTATTAATAGAGCCCAGAAAACGGCTTACTTGTCAA
ATGACCTCCTACAAAGCTACTTTTTTTTTTTTTGGCATGGTAATGAGAAGGTGGGCTGGTCTCTTCGAGGTGTGTGTGTG
TGTGTGTGTGTGTGTGTGTGTGTGTGTGTTGGGGTATGCATGTCTGCCCTGCACCCAGAATGGGCAGTTGGTGCCTTC
TGTGAGCAAGCAGTCGATTTTCGCTTGAAGGAATCTCAGGGCTGGACACTGAACGTGGCAATGCCAGTGGAAATGGCGTG
GGCTCGAAAACTAGAATGGGTGGCTCTCATTTCCATCTTGGTGGATTTAGTGACCCAAGGGAAGGTTTTGTCTCCTCAGA
GTCTGGGTAAATTGTTTTTTCTACCTCTCTTTCTAACTTCATTTCTCTTTTGTTTGTTTATTTTTGTTTTGTTAATAGAA
ATGACCATTTGAGTTAAAAAAAAATCCTCCCTGTGTCCTGTATCCTTAAAACAAATCATCAACAAAGTTGGAGCAATGCC
TTGGCTAGAATGAATTATGCTAGTGTGCTGGGCATTTTCCCTGATTCTTTTCTTTGTAACTTGTTTAAGGCTGCTGTTTC
TCGGAGGCCTCTCCAGCCAAGGAAAAGCTCCATAAAAGAAAAAAGCATTGGATTGCTCGCTGACCTGGCTTTGCTGTAAC
TGAAGGCTCACTCAACCTCGCCCTCTAGCGTTTGCCTGGAGAAATACCACCCCAGGCTCCTGGGGACACAATTAGCAGCT
ATGGTGTCCACTAGCATCCCGGAGGTTAAAGCTCTCCGCAGCTCAGTCTCTGACTATGGAACTATGATATCATAGTCCG
GCATTACAACTACACAGGCAAGTTGAACATCGGGGCGGAGAAGGACCATGGCATTAAACTGACTTCAGTGGTGTTCATTC
TCATCTGCTGCTTCATCATCCTAGAGAATATATTTGTCTTGCTAACTATTTGGAAAACCAAGAAGTTCCACCGGCCCATG
```

```
TACTATTTCATAGGCAACCTAGCCCTCTCGGACCTATTAGCAGGCGTGGCTTACACAGCTAACCTGCTGTTGTCTGGGGC
CACCCACTTACAAGCTCACACCTGCCCAGTGGTTTCTGCGGGAAGGGAGTATGTTTGTGGCTCTCTCTGCATCAGTCTTCA
GCCTCCTTGCCATCGCCATTGAGCGCTACATCACCATGCTGAAGATGAAACTACACAACGGGAGCAACAGCTCGCGCTCC
TTTCTGCTGATCAGCGCCTGCTGGGTCATCTCCCTCATCCTGGGGGGCCTGCCCATCATGGGCTGGAACTGCATCAGCTC
GCTGTCTAGCTGCTCCACCGTGCTCCCGCTCTACCACAAGCACTATATTCTCTTCTGCACCACCGTCTTCACTCTGCTCC
TGCTTTCCATCGTCATCCTCTACTGCAGGATCTACTCCTTGGTCAGGACTCGAAGCCGCCGCCTGACCTTCCGCAAGAAC
ATCTCCAAGGCCAGTCGCAGTTCTGAGAAGTCTCTGGCCTTGCTGAAGACGGTGATCATTGTCTTGAGTGTCTTCATTGC
CTGCTGGGCCCCTCTCTTCATCCTACTACTGTTAGATGTGGGCTGCAAGGCGAAGACCTGTGACATCCTGTACAAAGCAG
AGTACTTCCTGGTTCTGGCTGTGCTGAACTCAGGTACCAACCCCATCATCTACACTCTGACCAACAAGGAGATGCGCCGG
GCCTTCATCCGGATCGTATCTTGTTGCAAATGCCCCAACGGAGACTCTGCTGGCAAATTCAAGAGGCCCATCATCCCAGG
CATGGAATTTAGCCGCAGCAAATCAGACAACTCCTCTCACCCCCAGAAGGACGATGGGGACAACCCAGAGACCATTATGT
CGTCTGGAAACGTCAATTCTTCTTCCTAAAGCCTGAAGAAGCTGTTGATACTGAGGGAAGCATTCTGACTTGATCACCCA
CTACCCCAGCATTTCAAAAATGTCTCTCGCTCTCCACTGCTGCAAGGAAGGAGCAACTGGGAGCCTGAGAGAGGGAGGGA
AGGGAGAATGTGCGGCCTGGTGATACCATGATGTAGGTAGGTAGTAATTGTGAACAATGCACTGGGAAGGGTGGAGATCA
GGTCTGGCCTGCAGTGGGTTTCCTGCCCCCCCCTCCCCCCGTACCCTGGAGCTTTGATTTTGCACTGAGCCAAAGGTCTA
GCATTGTCAAGCTCCCAGAGGGCTCATCTGGTCCCTCCTCAAAGACTAAGGTCCCAAGAGAAAGTGTTGTGATCTCTCAC
TGTAATGTCAAAGTGAGTATGTGCCCTTTAGCTTGGTTGGGTGGTGTACATGTCCCCACCCTCCGGTTTCTTCTCACCCT
GTCTCCCCTTACCACCCCTTCTTTATATCGTAACTGCCTGACTTTTGTTCATCAGAAGGAGGTTGTGGAGGGGTCTATTT
CTTGATTATAGTGAGCAAGCTATTTGCTGGGAAGGCTATTTGCTGAGGCCAAAGTTTCCATGTAAACTGGGTCAAGTTGT
TGGGAATTTGGTAGGCCATCTTTAAGATGAAAATGTGTTCCTTTTTTGTCACATGTTCCTTATTCCCATTACAACCACAA
TGGTTGTGAATGAAGAGTAACAAAGAATAACGAAGTCTACTTTATTTGAATTATACTGGGCAGAATCTTAGATGATACGG
GAAAAGCAAAAAACAAACAAACAAACAAACAAACAAACAAACAAAATCTACAGCATGAGGTGAAATGTGAGAGTTAAGT
CATGCAGACCAAGAGCTGTTTCTTGCTGTCTTTCTCTAACCCCTACAGCACCTTTGGCACTTTTATTGATGATTTCAGAA
TGTTGTGTGATCCATTTCAACCAACTGGGGGAGAGTTGTATTTTGTTTAAAGAACATTTTCTGATTTTCGAATGTATTTG
TTTCTTTGAGGTCATCTTATTGAACTTCTTCCTAACCCGTGTTAACACCACTGAATGTGTAGTTCTTAAGAAAGTGTCAT
CCTTTTGTACCCCCCACCCCCCAAAAGCATTACTTTAACTGACAGGGAACATCAGGAATTCTTTATTTCGGCTGTCACTA
GGTATTGATGATGGTTATAAATGTTACAGAGAATAAAAATATATTAGTCTCTTTAGTCTGGTGTTCAGTGAGTGCAATTA
AGCCAAGAAATGTCTTTTTTAAAAAATAGTATTTAATAGGTTCCTGACTTTTGTGGATCATTTTGCACATAGCTTTATCA
ACTTTTAAACATTAATAAACTGATTTTTTTAAAAGATTCTTTTGTGAAGAAAACTTTTTAAAAAATCATTCACTGAGATT
TGGAGTTAAAGGTAAGGCCCAATTTAAAAAAGTTAAGAGAAGTTAAAAGCTTTTACCTTGATGAAGTAAGGTATGAAAAA
AAATGTGGTTTGTTTAGAAACCACTCTAGCTTTTTCAGGAAGAGAAACTTCAATAAATTAGCATTAGAGACACGTGGAGA
ATATGAGAAAAACCATCAACCTTCTAAGACCACAGCAAATAGATACAACTATTTCAATTTTAAAGTGCAGTGAGGCCAAA
CAAAACCAAGTGCGATAATTCCATCCTAAAATACAGCCTTCTGAAGTTCAGCAATGGAGTCTTAAATCTATTTTAAATTA
TGACTAAAATGATCAGTTCAAACACCACAGCAAAAGCATACATACTTGGCCTTTTATCAACATACTGGGAAGAAAAAAAC
ATGGCTAATGGGTGATAATGTTAAATTGGTCAGTGCTGTGTAAGTTTTTGGTTTGTGTATTCTTAAGACAAGGTCTCATG
TGTTCTAGACTTGCCTCATGCTTGCTCTGAAGGAATGTGGGCTGTTGATCCTCCCACTTCCTCNNNNNNNNNNNNNNNNNN
NNNNTTGGGCCCAGGTTTATGAAAGGCTGGGGATTGAACCCAGGTCCTGACACATGCTAGGCTAGCACTTTACCCACTG
AGCTCCATCTCCAGCCTCTAAGTTGTCCCATTCTAGGACATTGCCTTAGGATCTTATGAGAACACTATGGAACTATGCGA
TATCAATTATAACATGTAACAAGTGGAATAGCGGGTGGATGTTCACCCCTCCCACAGCTTTTAAACTTCCTCGGTTCTTG
CCCATTTAACTGGTCCTACTAAAATGAAAGCAACTGTGAAACAAAGGCATGTAGCATAAAGTTCTAACAGTTACCTCAGA
GTTACCGCCATCTTTGCTGCAGATTAGGTCAGCCTATTGCTACTTTTCAGAGTTTGTGTTTTCAAGTGTCATTGAGTTTA
CACAGGTCAGGACACAGTTGGGACATGAAGATAAACCTCACCTACTAGAGTAGATGTCATTTCTGGGTAAGGCTAACTGT
GGGAAACAGAGTGTGTTCGCTAAGGAAACCTTAGGTCATAACCTCCAGCTTCCTGAAGGAAATGCCAAAAGTGCTCAGAA
GACAAAGGGCCGGGAACAAATGCGAATGTCTCTATCCATCGTCTGCTGGGGCGGAAGGGGTGACTTCATGTTCCACTCA
CTTCCTCAGGCTTTCGTTAGAGAGCCAGGCATAATGGAAAAGTGTGTCTATTTTGCTTCAGTGGATGATAAAGCTCTGCT
TTTGGACTTATCTCCAAGCACAAGGTGACTCATGACATCGGAGCATAGGGTGGCTCATTGGTGACGGGAGGTCAATCTTC
TTGCCCACCTCTGAGTACATCATCCTGTGGACGTGCATTCTTCATAGCTCTGCCAGACAGTGGATCTTGTTTGGAGAAAG
CGGGGCAGTTGTAGATGTGTGGAAGCCCCAGTTCTAGTCAAGACACAAAGACAAAGATGAAGGGGTCTTTGGTTGCATCC
ATTGTGTATATCCGATTTTAATTGAGTACACTCTAAGTAGTGAGGATGAGAAATCACTCAGTGACTGAACACACACACA
CACAATCTCTACATATGTGCATGGAGACATAGCTCAGTTGGCAGAGAGCTTTTCTTGCATGCAGAAAGCTGTGGGTTCCA
TCCCCAGTATCATAAACCTGGTGTAGGTATGGCAGCAATCCCAGGAAGGTGAAGCAGGAAGCTTTTCAGTTAAATATTGA
GTTTGAAGCCATCCTAGGATATATGAGACCATGTCTGTAAAACAAAACAAAACAAAACAAAACAAACTAACAAAAACCAA
GAAGAACCCCTAAAGTAGTCTTAGTCAGGAGAAGTGAGGCATATAAAACTACTTACATATTTGTATAAAAATCGAATTAG
TTCCAAATATCAGGAAAGCTACAAAAGGACATTACATACATAGTATTTGATCCCCAACATTAGTAGCATCACAGAACACT
CTCCAGGGCTGTCGAGATGTCTTAGGAGGTAAAGGGGCTTGTCACATAAACCCAATTCTGTCCCCAGAGCCCGTTAATAA
TAGAAATTGGACAAGGGAGTGGTGATTCAAGCTTTTAATACCAGCAAATGAGAGGCTGAGGCAAGAGGATAAAGAACTCA
AGACCAGCTTTAGCAAGTGACAAATTGGAGGCCAAACTAGGTTATGAGACTATCTTAAAAAACTAGAGCTGTTACTATGA
CCAGTTCTTGTAATATTATTGTTAGGAATTTAGAAGCTTTAGTGCAAGTTATGACTTCAATCACCCATGCTTATTAGATA
GTATATTCTATTGAGTAATATTGGCACTTCATCTGTAATTTCTAGAATCCCTATTTTTTCTCCCCAATGTAAAAAGTCTGC
CCTATAAAAGTAATCATTTTAATCAATTTGGAATTATGCCGACTTCTTCTCTGGACATTGCCTGTGTTAATAAACTGTCC
AAAGCTGAGTGGTGGTGGCACACACCTGCAATCTCAGCACTCTCAGAGGGCATAGAAAAGTGGATTTCTTTTAAGTTTCA
```

```
GGCTAGCCTGGTTTCCAGAGTGAGTTCCAGGACATCTGCAGCTACAAAGAAAAACCCTGTCTCAAAAACAAGTAAACAGT
CCAAAGCCAACCAAACAAGGAACCTGCCCAACACATATTGTAATACGTCTAGGACTGCACTTTCTTAGGCTTTGGACCCT
TTGCACCTATGTTCTTTATATCACCAAGATTGTTCACGTCTAAGCATTTGTTTGGAGATGCTACTCAGAAAGGAAGACTT
CCAACAAATGATGGGTATTTCTAGTGAATGCTATAAAATGAGGGCTTGCCCAACTCTCAGTGGTAGCAATCCCATGTTTA
ATGGGAAAGCAGGCTATAGGACCAGAGTGGCTCATGTTAAGGCATTCTATGTACAGAATTTCTAAAGACCTTCTTAATAG
ACACTGGCTTTGCTGCTGGGCACATCTATAATCCTAGTATTCAGGAAGCCTAAACAGGAAGATTGCTGGGAGTTCTGGTT
CTCATTTAAAACAAAACAAACAACTAAACAAACAAACAAACAAACACTCCCCCAAAGAAAACAAACCAAAACTCATTGGT
TCAAAAATTAATTTCTAGCTAGAAACTGTTATAATAAGCATGGCAAGTATTTGTAATGAGCTTTTGTAGTTTGTTTGTAA
TATTATTGTTTTTATATAACTAATTAGTTACTTTTATTTCTTTGAGACAGGATCTTGTATAGCCAAAGCTGGCATCAAAT
TTATTTTGAATTTGAGGCATACACCATCACACCTGGCTCTAGAATTCTGTTTTGGGGGCAGGGTGTCGTATAGTCCAGGC
TGGCTTCAAACTGCTGGAAGATGAGCATGAACATAGTAGGACTTTGCTTTGACTGATCAAGTTCCAAGATTACAGGTGAG
TGCTACCACACCCAGTTGACGCAGTGCTGGATGTAGAACCCAGGACTTCCTGTGTGCTAGACAGACACAGACATGAGCAG
AGACACTCTATTAACTGAGCTGTATTCTTTTTTTCTTTTTCTTTTTTTAGATGACTCATTCCATTCTTCTGGTGGTGTTT
·TCCAAGGCCCCCTAACTAATTTTTATCAAACTAAATTTGATTGCGTTTATTTTTAGAGTTGAGATGTTTGCATTATTTTT
CCCTGAATAGTTTCTAAAGACACTGAGGGAGGAATTATAAATGAATCAAAACCAAAGAAAGCTTCACAGATTTGAAATA
TCCTTTTCCTGTGAACGTGGTAAGTAGTGTTGGTGGGGCTCGTGTGTGTGTGTATGTGTGTGTGTGTGTGTGTGTGTGTG
TGTGTGCGCGCGCGCGCGCGCACTTGAGTGCGCATGAGTGTTAAGATAGGGTCTCAATCTGTAGTTCAGGCTGTCCTT
GAATTTACAGTTGCCTCAGCTTCCTGAACCCTGGGTGGCATGAGTGAATTACCCTGCCCAGCACAGTAAGTTCTTGAAGA
TGGGAAAGACATAACAGGTTGCACCATCTCCCTCATGTGTTCGTTCTTCCTACCTACTTTGATGTGCTTCCCATCACTCA
AGGAAAAGGACTTAGTTGAGCCATCTGCACACGTCTTTGTTTCTGATGACAGCCATTGCACATTGACTACTACTCAGTTA
CTACTAATTCTTTTTTTTTTTAAAGATTTATTTATTAATATATGTAAGTACACTGTAGCTGTCTTCAGACACACCAGAAG
AGGGAGTCAGATCTTGTTACGGATGGTTGTGAGCCACCATGTGGTTGCTGGGATTTGAACTCTGGACCTTCAGAAGAGCA
GTCGGGTGCTCTTACCCACTGAGCCATCTCACCAGCCCCACTACTAATTCTTTAAACAATTTGTATGAATCTCAAAGCCA
TTGTGTCGAGCAAAGATAAAACACAAACAACAGGCCAATCTTCTACTTCATAAGCTCCTGAAGTGGAATTTGGAAGAAAG
CAAAACTGGCACTGGTGAGATGGATCACTGGTTAACAGGGCTAACTGCTCTTGCAGAGGGTCTGAGCTGGGTTCCCAGCA
CCCACGTAAGGTGTTTTACAATCCACTGCAATTCCAGTTTCAGTGGATCCAACTCTTCTGACCTCTGCTGAAGTTACACC
CATATGAACATATATGCACTCATATTATTAAAGATCATAAAATGTATCTTTTTAAAAAGGAAAAGAAAACCAGCTTATA
GAGATCAAAAGAGTACAAACAGAGACCCAAGCACAGAAAGAGGAAAAACTTTCAGATTGTTAAGAATGCTCTATTTATAC
CTTTTTTTTTAAAGAACATTGATTATTCAGATGGATATATGTGCTTAAATTTATCCAACTATATTCTAAAGACAAATGAA
AGCTTTAAAAATCCAAGGCAGCAGGCTTCTGCTTGTAAGCACAATGGCAGTAAAGGGTCTTCTCTTGAAAATTCTGCAGA
TTCCACACAGTCCAAGAGAATGTGGCAGAAGTCCCAGAGTATTCCATAGACGAGCATGTGATCTCTTTGTATTTATCTCA
CGCTCCAGAAACAAACCATTAAAATACTAAATAATCGTGACCCAATGGTACTCACTTGCTTCTGTAGGATGAGAAAATGC
CTTAGCTTCTCTGTGTGTTATCCCTGGAGAATTCTGTGTGTGGAAAGAGTCTAAGGGTACAGTTAGGCTTTCACAATAAA
AAATTCATTGGCATTCAGAACCCACTCTTATGAACTCATGAAGGGCTGTAAGCATTCTTAAGAAGAATACAATTTAAACA
ATTTCTATGAATGTAGAAGCCAGTAAGGCTTTGACTGGTAAATTAGTACTGGAAACACACAGCCATGGCCACATGAGTAA
ACCTATAAAAAGCCTTCAGTCAAGAAAAGGCAACTTCCTGGGTATAAACTCTGCATAGGTAGAAGTAATAGGGGGAAGATT
TAAGGGGTAGGCGAAGGCAACACTGAGACCAAGTACATTGTCTGCCTGTGCACAGCCCTGTCTGAGACCTTCAGCCAGCT
CTAGGTGTAGTGGAACAGAGGTGACATGCTTCAGGTGTTCCACAGGTCCTGTGACATGCTGATTAGATATTAATCAGAAC
TAGAAACAGGAAAGCAGGAGAAATGCCGCCTGTTCTACAAGGGGATGGGGAAGGTAGTAAGTGTCTTGCTTATCTGTTTC
CTGTAGAGACATCAAAGAGATGATGTGGCTGACCTTCCCGCCCCCACCTCCTCACAACTCCTCACTGTCCGTTCTCTCGC
CTGCCCGGTTAGCTGGAACGCCTGCCCTGGGTTGTGTTGTTGTGCTCTGGGAAAATGGCGAGCAGGGTTCTTCTTGTCTT
TCTAGAACCTACCAGGTCTATTTTTTCTGTTCGGGTGCATTTGTGGCCTCTTCCCTTTGAGGTTAAGGGATGATGAGATG
GCAAGAAATACTTCTTTTTTTTTTCCTTTCAAGAATGTATTCAGTGTCTCATACTTCCTCCATGATTTTAGCTTCTCTGC
AAGACTTACTTAGCACTTAAATTGGACAGGCAAATTAGGTCCAATTCATGGCGACTCATGCTGTAATCCCAGCACTTGG
GAAAACAGGACAGAAAGAAGCTTGTGCTATACACTGAATCCTCCAGACCAGCCTGGGCTACTGAGTAAGAACCTGTCTTT
AAAATACAAATGAAAGGCTGACTGTATATATAGCTCAGCAGGTAAAGGTTCTTGCTAAGTGAACTTGGGGGTCTGGCTTG
GATTCTTGGCTCCTAAGTCAAAGTAGTAAGTTTTTTTTTTTTAGTTACACATTTTGTCTCCAATATTTTTTTTTTAATAC
AAACTGCATATGGTTATTCAAATGACTTAAGTGGACCTGATTTACGGGTCCACTGACTCCACAGCACCTGACCTCCACAC
ATGCGTCATGGCACACACACACACACACACACACACACTGATAAACAACAACAACAACAGAATGTAAATCAGACAA
AGTGTGCTTCATGCTGACAGCTTTCCTCCTTGCCTTGCCTTGCCTTGCCTTCTCTAAACTACTCACGTGAAATGCCGTGG
AATGATTTCATCTGGAAGAGAAGGTTACTAGATGTGCTGCAAAAATCAGGGTGGAGCTTTTCCAAGTTGTTCATGGTCTT
CCTGATGAAAAGCATCCAGTTTCGGGTAGATGGAAACATTTTACATTGTTGCCAGTTTCTGCCTCAGAAACTGAGGCATT
GCTGTCTGAGTGTGTGCTTTTCTTTCTGTAGCGCTGCTGTGAACTGTAGCCATGTCGGGTATAACCCTTAAATCAGGTCC
ACTTAAGTCATTTGAATAACCATGGTGGGATCCTTATGCAATTTGTATTAAAAAATATTGGAGACAAAATGTGTAACTAA
AAAAAAAAAATACTTATTTTATTCTCGTGTGTGTGTGTGTCTGTGTGTGCTCGAGTGCATGTGTGAGCCAGAAGAGGACGTTA
AATCCCCTGAAGCTAGAGTTACAAGTGTTGTGAGCTAAGATTTGAACTCAATCTCTGGAAGAGCAACAAGCCCGTAACCA
CTGAGCCATCTATCGCTCCAGCCCCTTAAATGCTTAATATTTTAGGAGAGAAAGCATGACATGTGCTTTCAATGATCAGA
ATCACGATGGCACAAACTCGTGGAATGGAAAGCCCTCCAATCATCTAAGAATCTCAGAGAGGTGCCTAGGTAGCTCTCGC
GCACGCTTCCTGCTCAAATCCACCAGCAGCCCAGGGAGCTGTGAACCCCAGACCCACACGCTAACCCTAATGAATGTGAGC
TGACCTAGTTACGTTTGCTAGGCATTGTGTTTGGTTGGTTTTGTAGGTAGGGTCCTACTGCGTGTTGATGGCAGAAAGGA
CACTTGTGGCTCACCCTCCCGAGTGCTGACATTACAGGATGCATCGCCATGACTGATGTCGCTTGCTGCTTTTATCTATT
```

ACTTATTTTTTACTTTGAAACAGTCTTATGTGTCTCAGACTGGCTTCCAACTTACTATAATATCTGAGGATGACTTGGAA
TGTCTGATTGCCCACTAGCAACTCCCAAGTGCTGGTGTCATAGGCAAGGATGACCAATCCTGCTTTATGTGGTACAAGGC
ATTGAACCCCAGGCTTCTTGTGTGCTACATGAGCACTCTGGATGGAGCTGCACCCCAGCCCCATTGCTATTTTTATGAT
GAGGATTTTTAAGAATTCAGTCACATGTTTGCTCACAACCCCCTCCCCCCCATCCCCCCCGCCCCATTGACGGGCTCTTC
CTCTTAAAGTTCACCATCTTCTTGAAGGTAAAGTGTCTTCACTACTTTGTNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNAACATGAGCTATCAGAGGCCT
GGAGACTCTGCTCACACAGCCTCTGCCCTACCAGAAATAGAGGTTGGTTAGCTCACATGCTGTTCTGCCTCAGCGAGCTC
TGCATGTGGCCACCTGCAGAGAGAACCCTTGGTTTCTCTTCCTTGTCTAGATGCCAGTCAAGGGTGCTTTGAGAAAAACA
CAGATTTTCATACTCCTTTCTCATGTGCAAGTGAGCTGGGGTATCAAAGAATTTCCTAGGTTGTTTAATCTAGAAAATAC
CTTGTTTTTTTGGTTTTGGCTTTTTAAAAGATTTATTTATTTATTCATTAATTTTTTTATATGAGTACACTGTAGTTGTC
TTCAGACACACACCAGAAGAGTGCATTGGACCCCATTACTGATGATGGTTGTGAGCCACCATGTGGTTTCTGGGATTTGA
ACTCAGGACCTCTGGAAGAGCAGTCAGTGCTCTTAACCGCTGAGCCATCTCTCCAGCCCCTAGAAAATCCTTGGTTACAA
GAGCAATAAGATGATCAGGAGGGCAAAAGTCCTGGCCATGCAAACCCAAGTACAATGTCTGAAACTTGTATGCAAACACA
CACACACACACACACACACACACAC


MOUSE mRNA SEQUENCE : mR6-131.1 (Seq ID No: 64)
ATGGTGTCCACTAGCATCCCGGAGGTTAAAGCTCTCCGCAGCTCAGTCTCTGACTATGGGAACTATGATATCATAGTCCG
GCATTACAACTACACAGGCAAGTTGAACATCGGGGCGGAGAAGGACCATGGCATTAAACTGACTTCAGTGGTGTTCATTC
TCATCTGCTGCTTCATCATCCTAGAGAATATATTTGTCTTGCTAACTATTTGGAAAACCAAGAAGTTCCACCGGCCCATG
TACTATTTCATAGGCAACCTAGCCCTCTCGGACCTATTAGCAGGCGTGGCTTACACAGCTAACCTGCTGTTGTCTGGGGC
CACCACTTACAAGCTCACACCTGCCCAGTGGTTTCTGCGGGAAGGGAGTATGTTTGTGGCTCTCTCTGCATCAGTCTTCA
GCCTCCTTGCCATCGCCATTGAGCGCTACATCACCATGCTGAAGATGAAACTACACAACGGGAGCAACAGCTCGCGCTCC
TTTCTGCTGATCAGCGCCTGCTGGGTCATCTCCCTCATCCTGGGGGGCCTGCCCATCATGGGCTGGAACTGCATCAGCTC
GCTGTCTAGCTGCTCCACCGTGCTCCCGCTCTACCACAAGCACTATATTCTCTTCTGCACCACCGTCTTCACTCTGCTCC
TGCTTTCCATCGTCATCCTCTACTGCAGGATCTACTCCTTGGTCAGGACTCGAAGCCGCCGCCTGACCTTCCGCAAGAAC
ATCTCCAAGGCCAGTCGCAGTTCTGAGAAGTCTCTGGCCTTGCTGAAGACGGTGATCATTGTCTTGAGTGTCTTCATTGC
CTGCTGGGCCCCTCTCTTCATCCTACTACTGTTAGATGTGGGCTGCAAGGCGAAGACCTGTGACATCCTGTACAAAGCAG
AGTACTTCCTGGTTCTGGCTGTGCTGAACTCAGGTACCAACCCCATCATCTACACTCTGACCAACAAGGAGATGCGCCGG
GCCTTCATCCGGATCGTATCTTGTTGCAAATGCCCCAACGGAGACTCTGCTGGCAAATTCAAGAGGCCCATCATCCCAGG
CATGGAATTTAGCCGCAGCAAATCAGACAACTCCTCTCACCCCCAGAAGGACGATGGGGACAACCCAGAGACCATTATGT
CGTCTGGAAACGTCAATTCTTCTTCCTAA


MOUSE PROTEIN SEQUENCE : mP6-131.1 (Seq ID No: 65)
MVSTSIPEVKALRSSVSDYGNYDIIVRHYNYTGKLNIGAEKDHGIKLTSVVFILICCFIILENIFVLLTIWKTKKFHRPM
YYFIGNLALSDLLAGVAYTANLLLSGATTYKLTPAQWFLREGSMFVALSASVFSLLAIAIERYITMLKMKLHNGSNSSRS
FLLISACWVISLILGGLPIMGWNCISSLSSCSTVLPLYHKHYILFCTTVFTLLLLSIVILYCRIYSLVRTRSRRLTFRKN
ISKASRSSEKSLALLKTVIIVLSVFIACWAPLFILLLLDVGCKAKTCDILYKAEYFLVLAVLNSGTNPIIYTLTNKEMRR
AFIRIVSCCKCPNGDSAGKFKRPIIPGMEFSRSKSDNSSHPQKDDGDNPETIMSSGNVNSSS*


MOUSE PANTHER CLASSIFICATIONS
        FAMILY (SUBFAMILY)
                CF11294(LYSOSPHINGOLIPID RECEPTOR-RELATED)
        BIOLOGICAL PROCESS
                Signal    transduction(2.11.00.00.00)    >    Intracellular    signaling
cascade(2.11.02.00.00) > Calcium mediated signaling(2.11.02.02.00)
                Signal              transduction(2.11.00.00.00)              >              Cell
communication(2.11.03.00.00) > Ligand-mediated signaling(2.11.03.03.00)
                Signal transduction(2.11.00.00.00) > Cell surface receptor mediated
signal transduction(2.11.01.00.00) > G-protein mediated signaling(2.11.01.07.00)
                Neuronal        activities(2.18.00.00.00)        >        Other        neuronal
activity(2.18.99.00.00)
                Developmental            processes(2.23.00.00.00)            >            Ectoderm
development(2.23.08.00.00) > Neurogenesis(2.23.08.01.00)
        MOLECULAR FUNCTIONS
                Receptor(1.01.00.00.00) > G-protein coupled receptor(1.01.01.00.00)


MOUSE GENE ONTOLOGY
        BIOLOGICAL PROCESS
                cell surface receptor linked signal transduction > G protein linked
receptor protein signaling pathway

G protein linked receptor protein signaling pathway > G protein signaling, linked to cyclic nucleotide second messenger

sensory perception > vision

vision > phototransduction

G protein signaling, linked to cAMP nucleotide second messenger > G protein signaling, adenylate cyclase inhibiting pathway

MOLECULAR FUNCTION

ligand binding or carrier > lipid binding

protein binding > lipoprotein binding

amine oxidase > amine oxidase (flavin-containing)

enzyme > 2-acetyl-1-alkylglycerophosphocholine esterase

monooxygenase > monophenol monooxygenase

CELL COMPONENT

cell > membrane fraction

plasma membrane > integral plasma membrane protein

cell > plasma membrane

cytoplasm > lysosome

cytoplasm > endosome

MOUSE PROTEIN DOMAINS (INTERPRO SIGNATURES)

IPR001671 (MCRFAMILY)

IPR000276 (GPCRRHODOPSN)

IPR000987 (EDG1ORPHANR)

IPR002230 (CANNABINOIDR)

IPR000276 (7tm 1)

IPR000276 (G PROTEIN RECEP F1 2)

IPR000276 (G PROTEIN RECEP F1 1)

HUMAN GENOMIC SEQUENCE : hD6-131 (Seq ID No: 66)
TAGATTGAGATAATTATTAACCTATCTGTTATTGATGTCTTTATGTCATACTGACATATTTTGACCTTTACAGTGTCTTT
GTTCATGTCAGGGATTTTTTTTGTGTGTCAAATCAGCAGGAAATTTACCTTTTTCTAGTTCTCATGATTAATTGTTCTTC
ATTATATAGGTTATGAACAATCAAATAACATATGTTTGCTGTTTCTGTTCAAAACATGCATCTTCCTTTTAATAATGTAA
CATGCTCTCAATATGATCTAAAGTCAGAATGATTTTTATTTATTCAATTGCTCATTTTTATTGTTTTTGTTTCATATTTT
ATGCATTTTTAATATAAATTTTCTCTCAATCCTTTAATAAATTTATAGATAACAAAATATTCTTTATATAAGGCCAGTCT
GTAATTTCTCATATATTTCATTGAAATAATCCTAAATTTATAACCAAATTATAATAGCTATAGCATACGAATATATGAAA
ATTTTGATTAATCTTTTTTTATGTGATTCTTATTAAAAATGGAAAAAAAGGCCAAGTGTGGTGGCTCACGCCTGTAATTC
CAACACTTTGGGAGGCTGAGGTGGGCAAATCACTTGACGTCAGGAGTTTGAGACCAGCCTGACCAACATAGTAAAACCTT
GTCTCTACTGAAAATACAAAAATTCCAGCTGGTAGTGAGTGCCTGTAATCCCAGCACTTTGGGAGGCTGAGGCGGGTGAA
TCACTTGAGGTCAGGATTTTGAGACCAGCCTGGGCAACATGATGAAACCCCGTCTCTACTAAAAATATAAAAATTAACCA
GGTGTGATGATGTGCACCTGTAGTCCCAGCTACTTGGGAGGCTGAGGCAGGAGAATTGCTTAAACCTGGGAGGCGGAGGT
TGCAGTGAGCCGAGATCATGCCACTGCACTCCAGCCTGGGCAACAGAGTGAGACTCTGTGTCAAAAAAAAAAAAAAAGAAG
AAGATGGAAAAACAAGTGTGATGTGTTTATAATTATATATACTGAGTTAGTAAATATATTCTGAGAAGAATAGAACTTTT
GTTTTAATTAGGTGTCAATGAGAATCAAATCCTCAAATTATAATTTGCATATGGTATTTTGGATGAATTTCCCCCAGACT
AGCTCCTGGCTCAGTCACGTGACACCAGCCCTGTACTTTTGTATCATGGCAGCCATAGAGGGTAGTTGAAATAGTCCTAT
AAACCAAAAATACCTTCAACAACTCCAAAATCACTCCTACACAAGAGAAAATGAGACAGAGGGAACGTCCTAATGGAAGA
AGACAGTGATCTGTGTCAGTCATAGCCAATTTTGGTTAAAATACCATGCTTTTAATATTTTACAAAAACATATAATCTTG
GGGACATATTGCTGGGTTCCTTCCAGGATTTGGAAGTGTAGGACTCAAGTTTAAGCTACATTGGTTTCAAAGTTAATTTT
GCCTCTAACAATAGCAATAAAGTTTTAGAAAACAAACTGTGTGTAAGTGGCTTGACAAACCAGAGAAAGTTGAGACCTAA
GCCCATAGCAGGAGGATTGTAGAAGCAAACCAAATTCCCACTACAGATCCTCAAATAAGAGGACTTAGAAGCTCCAGGTA
TTTATGAAAATGGCAATAAATGCTTATCTGAAAACAAGAAGCCTGATTATAGGGTATCCACTATAGCAGTGAGAAAATAT
TTCACACTCACCCAACCACTTCTCTCTAGCCCTGTTGTAGTCATTGCCTTCATTATTTTTACTTTCTGTTTATTTTCCTGT
TTGATTTTTTTTTCTTTATGTCAGATGGGTAATGTGCTGACATCATAACAAGGTTCAAGGATGGCATATCTCACACGTGCG
TAAACACCCAGTGATCACGCTTATGAACTACAAAAGGATTCCTTTTTGATTTTTAAATAAAATTGTTTTAATTAAAATAA
GCTGCCAAATATCCAGCCCTTCATCTCCAACCTGTACAATAGGATAAGTGTCGGTACGCTCATTCCAGGAGAAGACTGGA
GGTTTCTTCTCTGAATGGGTTAAATCAGAGGGTCACTGGAGTCAGAGACACCTGATGGAGCTGAGTGTGTAGAGGGGGTT
CCCATCATGGTGATTCAGTTCAAATCTAAATATCAAATGGTGAGGTGTTCCCTCCTTCACTCTCCCACTTTTCTTTCTTG
GTTCACTCCGGAAAATGCTAGAAGTCAGACAAGAGATTTTAGAATTTCTGTCTGGCAAAACTGACCTGCCCAAAACTTGA
GAACTCCAGATATTGACATTTGGCATACTCAACGAAAGTGTTTATCCTGATCATTTACCACCCCTCCATTGACAAGCCCC
CACACAAAGTTTTAAATTAGCTTTCTGATTTGCCTCTATTTTAGCCTCTGTTTGAAATATAAATGGACGGCCAGAGATCA
TCTGACAATTGGGAAAGCCAATGGCACAAAGAACTGAGAGCAAAACAAATAGGAAAGTGTGACAACAAAGAAAATAGGAG
AAAATGTCAACAACAAAAACAACAACAACACTCTGATATCCTCAGAGATAAGTAAAGATGTGGTATCCCTAACATAAGAA
CAGAGTTCTGTAAAAATGTAACATTCAGAAAAGAAAAAGAGAGCCCTTGGAAATTAAAGTATGAGAGTGAAAAGAGAGCT

```
ATTAACTGAAGGATTGAAAGTACAAAAAGGAAAATAAAATAGGAGGAAATTTAAAAATCAGGCTAGATAGATGGTCCACA
ATCTGAATAGTGGGATTTGCAAAAAGGGAAAATAGAAAAACTGGAGAGAAGATGAAGTCATTAAAGAAATAATTTCAGAT
AATTTTCAGAACTGCAAAGGACTTAGAAAATCAAGGTATATCATCATGTCACTCTGGAATACTGAAGAGACAGAGAAGTT
TCTAAAAACATCCAGAGACAAAAAATAGTCAGCTGTGAAGTTTTGAAATCAGAATAGCTTTATGCTCTTTATCAGTAGCC
CTAGAAATTAAAAGACAAAAGAGTAATGCTTTCAAAATTCTCAGGGGAAATGACTTACAACTTTTAACTTGATACCTAGT
CAAAATATTATTCAAGTAAATAATAGAATATTTAAAATGTCAGACATGCAAGAATTCAGAAAGTTTATAATATAAGCATC
CTTCTTCAAGTTAATTAATACTCCTTCAACATGGCAGAAACCTATAGATATTGTTATAAGATTGATAATGAAGAAATGG
AGGTATAAAACATGGGATTTCAAGATTTGCAAATGGCTACCAGAAGAAAATCAACAAGACTTCACAGTGACTGGAAAAAT
TGGTGAACCAGGGAACCTCGGAGGACTAATGCTCTTCTTTTCAAGTTTTGTACCATTTGATCTCTTTAAAATGATATGCA
CATATTATTTTGACTTTAAAATTTTATTGGAAAATTAAAAAGCAAAGTGAATAAAAAAATGGAAAGTGGGATGGTGAAAG
CAATGATTGCACTTAAAGACAGTTAAAGACAAATGTGGTGCGGTCAAGTCAGCTTCATACCTGGGATGCAAGGCTGGTTC
AGCATATGCAAATAGATAAACGTAATCCATCACATAGACAGAACCAATGACAAAAACCACATGATTATCTCAATTGATGC
AGAGAAGGCCTTCAACAAAATTCAACACCTCTTCATGCTGAAAACTCTCAATAAACTAGGTTTTGATGGAATGTATCTCA
AACTAATAAGAGATATTTGTGACAAACCCACAGCCAATATCATACTAAATGGGCAAAAGCTGGAAGCATTCCCTTTGAAA
ACCAGCACAAGACAAGGATGCCCTCTCTCAACACTCCTATTCAACATAGTATTAGAAGTTCTGGCCAGGGCAGTCAGGCA
AGAGAAAGAAATAAAGGGTATTCAAACAGGAAAAGAGGAAATCAAATTGTCTCTGTTTGCAGATAACATGATTGTATATT
TAGAAAATCCCACCATCTCAGCCCAAAATCTCCTTAAGCTGATAAGCAACTTCAGCAAAGTCTCAGGATACAAAATCAAT
GTGCAAAAATCACAAGCATTCCTATACACCAATAACAGACAGAGAGACAAATCATGAGTGAACTCCCATTCACAATTGCT
ACAAAGAGAATAAAATATATAGGAATACAGCTTACAAGGGATGTGAAGGACCTCTTCAAGGAGAACTACAAACCACTGCT
CAAGGAAATAAAAGAAGACACAAACAAATGGAAAAGCATTCCATGCTCATGGATAGGAAGAATCAATATCATGAAAATAG
CCATACTGCCCAAAGTAATTTGTAGATTCAATGCTATCCCCATCAAGCTATCATTGACTTTCTTCACAGAATTGCGAAAA
ACTACTTTAAATTTCATATGGAATCAAAAAAGAGCCCACATAGCCAAGACAATCCTAAGCAAAAAGAACAAAGCTGGAGG
CATCATGCTACCTGACTTCAAACTATACTACAAGGCTACAGTAACCAAAACAGTATAGTACTGGTACCAAAACAGATATA
TAGACCAACGCAACAGAACAGAGGCCTCAGAAATAACACCACACATCTACAGCCATCTGATCTTTGACAAACCTGATAAA
AACAAGAAAGGGAGAAAGGATTCCCTATTTAATAAATGGTGCTGGGAAAACTGGCTAGCCATATGCAGAACACTGAAACT
GGACCCCTTCCTTACACCTTATACAAAAATTAACTCAAGATGGATTAAAGACCTAAACGTAAGACCTAAAACAAGAAAAA
TCCTAGAAGAAAACCTAGGCAATACCATTCAGGACATAGTCATGGGCAAATGCTTCATGTCTAAAACACCAAAAGCAATG
GCAACAAAAGCCAAAATTGACAAATGGGATCTAATTAAACTAAAGAGCTTCTGCATAGCAAAAGAAACTATCATCAGAGT
GAACAGGCAACCTATATAAGGGGAGAAAAATTTTGCAATCTATGCATCTGACAAAGGGTTAATATCCAGAATCTATAAAG
AACTTAAACAAATTTACAAGAAAAAACCAAACAACCCTATCAAAAGTGGGCAAAGGATATGAACAGACACTTCTCAAAA
GACAACATTCATGCAGCCAACAAACATATGAAAAAACCTCATAATCACTGGTCATTAGAGAAATGCAAATCAAAACCGCA
ATGAGATACCATCTCATGCCAGTTAGAATGGTGATCATTAAAAAGTCAGGAAACAACAGATGCTGAAGAGGATGTGGAGA
AATAGGAACGCTTTTACACTGTTGGTGGGAGTATAAATTAGTTCAACATTGTGGAAGACAGTGTGGCGATTCCTCAAGGA
TCTAGAACTAGAAATACCATTTGACCCAGCAATTCCCTTACTGGGTATATACCCAAAGGATTATAAATCATTCTACTATA
AAGACACATGCACACATATGTTTATTGTGGCACTATTCACAATAGCAAAGACTTGGAACCAACCCAAATGTCCACCAATG
ATGGACTGGAAAAGGAAACTGTGGCACATATACACCATGGAATACTATGCAGGCATAATAAAGGATGAGTTCATGTCCTT
TGCAGGGACATGGATGAAGCTGGAAATCATCATTCTCAGCAAACTAACACAACAACAGAAAACCAAACACTGCATGTTCT
CACTCATAAGTGGGTATTGAACAATGAGAACACATGGACACAGGGAGGGGAACATCACACACTGGAGCCTGTTGGGGGTG
GGGGGCTAGGGGAGAGATAGCATTAGGAGAAATACCTAATGTAGGTGACGGATTGACGGGTGCAGCAAACCACCATGGCA
CGTGTATACCTATGTAACAAACCTGCACATTCTGCACATGTGCCCCAGAACTTAAAGTAAAATAATAATAATAATAATAA
TAATAATAATAAAAGATGGAGGTGGTGGATGGGTGAAGCATTTTCTCTGTAGTGTAGGGAATTCTCACAGGTATGTAGCT
CCTTAATCCTCACAATGAAAGAGGCAAATATCATTATTTCATTTTACAGATGAGGACACTGAGGTTCAAGGAAGTTAAAA
GAACTTGCCTAAAGTCACATAGCTTAGAACTGGCAATATCAAGGCTGAACCTAAGTGTCTGTTCCAAGGCTCAGTCATGT
TCCACTAATTTTGAATGTTGGCAAACGAGGAGAGAGAGATATTGGGACCTAGGATGAAGGGAAGAAAGAGTTTTTGGTAG
AGAAAACACATAAACATTTATGTAAGTTGAAAGTAAAGAGTCCTCAGAAGCTAAGAACTGAAAGTACAAGGGAGAATAAG
AATAATTGAGCCAAGTCTCAGAAAGTTTGGAGGAAGCAGCATGCTGACCTGGGCAAGAGTTTGCCTTGGAAAGGAGTAAA
GTTGCTTTTATTTCTGAAACAGGTGGGAAGGAGGTAAGGGTGGGTAAAGTTGCTATTAAGCTGGAGCTTGGAGAGGAGGG
AAATTGAGATGGTCATACTATTTCCTCCTTGAAATAAGAGGCTAGTTATTCTCCTGTGAATAGACAAGTAAGGGAAAGCC
TGGATTTTTACCAGTGACTGGGTCAGGGGAGGGAAGAGGGGAGAAAAGGGAGTTGGAAAGTGACTGGAGCAATTAGGAAA
GAGAGTAAGGCTCAGGCTTGTAATCCCAGCACTTTGAGAGTCCAAGGTGGGAGGACTGCTTGAGCGCAGGAGTTCAAGAT
CACCCTGGGCAACAAAGTGAGACTCCTGTCTATACAAAAAATAAAAATAATAATTATCAGGACATGGTGACTGGTGCCTG
TAGTCCCAGCTGCTTGGGAGGCTGAAGTAGGCGGATCCTTTGAGGGCGGGAGATCAAGGCTGCAGTGAGCTATGATTTCG
CCACTGCACTCCAGCCTGGATGACAGAGCAGAGCCAGACCCTGTCTCAGTTGAAAAAAAGAAAAAAAAAAAGGAAAGGGA
AAAGAGTAGAGTTGCAATATTTAGCAGATAAAAAATATAGCTCATGGCTGGGCTCGGTGGCTCATGCTTGTAATCCCAGC
ACCTTGGGAGGCCAAGGCAGGTGGATCGCCTGAAGTCAGGAGTTCGAGACCAGCCTGGACAACATGGTGAAACCCCATCT
CTACTAAAAATACAAAACTAGCTGGGTGTGGTGGTGCATGCCTGTAATCCCAGCTATTCAGGAGGCTGAGGTAGGAGAAT
TGCTTGAACCTGGGAGGCGGAGGTTGCGGTGAGCTGAGATCACGCCACTGGACTCCAGCCTGGGCAATAGAGTGAGATTC
TGTCTCAAAAAAAAAAAAAAAAAAAAAGATAGCTCACACAGTTACATTTGAATGGCACATTTATGACAAATTTTTGTTT
AGTGTATGCTCCAAAGATTGTGTGGTATTTTATCTGTAAACTTTTAAAGAGAAACACTAAAGCATGTGTAAAGAACAACA
AAGCCACTTTGAAATCAAAAATTGGAAATGGAGCAAACTTAAGGTAGGAATGTTGGGCTTGACTTCTAGGCAGCAGCAGA
CAGGGAGTTGTACTGGCAGTCAGGAGATAGCTTGAGAATGCAATTTTTAGGGCCTCCTTGGAGAGCAATGGGAAGAGACT
```

```
TATATGGGAACTAGAGACTTGGAATTGGCCAGGACGCCATTGCTGGAAACATTTGCCTACTATCTAATTTTACCTAAGTT
GATCATCTACAATTACATCTCATAGGTGTGTACTAAAGATGAATATTATTAAATAACAATCTGAAAGGTTTTTGGACTCT
GTAAAACACTTTATCAATATTGAATGTTAAAAACCAGTGGTATTCACAAGAAAACAACTGATATCCCCAAGGAAATTCTT
GAACCTGTTTGAGGTTTATCATGGTGGTCTGGAGTAGTGGTTAAGAGTCTCGCAGCCTGTCCCACATTAAGCAAGCTGCTT
AATATGTCTGGGAGTCAATTTCCTTGTTTGTAAAATTAGAATAATTATAGTGCCTAACTTGTCAGATTAAATGGATTAAT
ATGTGTAACACTATGTGCAAATACTAAAGTACAATGTTACATGAGATATCATAGGCAGACAATGAGAAGTTTCAGAAAA
CATTCATTAATATGCATATGTATACACTTTGTATGCTTACGTAGAAAATGAGTGGTTTTAAAAGGACATTCATTAGCATA
CTTTTATATGCATTTTGTAGACTTGTACAGTCAGAAATGCAGCAAATGTAAAACTTTAAATCTAGAAGTCTGTTTCCCTC
TAGGAACATATATTTCAATTCCTTAACTATTTATTGACTGATAGCGTTTGAGCCACAAGGCAAGGCATTTGCAATATTTA
GGTTTTGGAACCCAGCTCTGCCACTCACTAGCTGGGAAATCTACAAGTCACCTTAGTTGTCTGAATGACACTTCTCTCAA
CAATAAAATGGCCAGCAATGCTTACTTACTTCACAGAGCAGTAAGGTTAAATTAACCTAGCACATGCTTGATACAGACTA
GGTGTGAAATTTCACTTTGATTCCTTTGGCTTTAAAGAATGCATAAATTAGTGTGGGTGGAGGCGGGAGAAGGGGGGAA
TGCCAAGTCCCCCGTTTGTTGTGCTGGGTAGACACAATATACCTCTTCCCCTTTAAGTGAAATAAGTCTTCAAATCCAA
TTAGGATCTACTCTGGAGTCTTAGAGGACCGTTATCTTGGTTTCCACATTATATGCACTGTACTGTTGTTGAAGCTGTGG
GTATGTATTGGGTGCGTCGCTGTCGATGAATGCTTTAAAACAGCTGTGAATTAACTGTCACTTCTTTTTTTTTTTTTTTT
GGCAAACTCACAAAATCCCAGAAGTCAGGGGTCTGTTGTTTGTTCATTCTGCCTCCTTCTGAGTCATTCAGGTACAGGGT
GTGTCTGCCAATTGAAACTGCACAGCGGAGGAAGTTTTTGAGTTCACATCTGCCGGGATGGGAACGCAGTGCGACCAGGG
ACTGGGTGGAAACTTTTACAACAGTAATGCAAGGGCAGAAGCGTCCTGTCCTGAGCTTGTCGCAGGCAGATCTGACCA
AGTCCGCCTCTCAGCCTCACGTTCTTAAGTAGCATCTAAGCAAAAGAAAAGGGTGGGCAAAGTGCTAGTAATGAGATTTG
AGGCCTCTGAGTCGACTCCCAAGCAGAATGTGAGAGAAGGGTGCCGGCTCCTGCTGGGCAGCTGCAGTTGCTCCGCAGCT
CCTGGCCGAGAATCTCCCCCTTTGGAAATTCCTGAGCTGCCCAGGCGTTTTGAATCTCTCAGGACTCAGCAGCAGCCCAC
CCCCTCAACGACCTCTCCATTGGCCAGAATCTGGGACACACAGCCTGACTTTGTGTCAGCTGAGGGAAACCCCTCGCCC
AACACCAAAACCGGCGCTGGTTCATTTCTCTGTGCACTCTGGGAAAGCCACTGCCCCCTCAGACCGCAGTTGCGAGTAGCA
CGAGGATCTGGGGTGGCGGGGGGAGTACAGGAACCTCCCGGTTCATTCTCCCTCCCGGGCTGCTTGTCTCCTCCGTACCT
CTCGCAGCCCCCTTCCCAGCCTCTCCTCCCCAGAGCGCCGCCGACACTCGCCGGGCCGCGGCTCAGGTTTTCTGCTGCTCG
CGCGAAAGTGGCTCGGGGAGCTCCTGGCCACAAGCTCAGCACACCGATCCTCCTAGGGCTGCGGGGGGGTGTGGGACCAGG
GCGGTGCGCTACGAGACTCCGGGCCGTCCTCTGCCTCCTCAGACTTTTCGAGAGAAAAGTTTTCAAAGTCTCTCCACCTT
TTTTTTTTTTTCAGGGGCTCGGGAGAATCCTCTCCACAGCCAGGGATCAGCGTCTATCTGGGCGAGGGCAGTGATTTATAC
AGTAGAATCCAGGGGTTACCGGGCTCCGCCCCTTCTCTCTCTCTCTTTTTTTTCTTTCATTTGTTGTTTGGGGAGGAGGGG
TTCTTTTTTTTTTTTTTTTTTTTTTTTTTGCTTCTGCCCCAGATCTTTCCTGGACAGTGCGTCTCAGCAGTTCAGATCCG
GGGGCCCCCAGCTGACAGAGGGCGTGGGGGGTTAAGGCATTAACCCCTCCCAGCCTCTTCCTGAAGAAACCACCCAGCCT
TGGCGCGGCGCTGGGTGACTTCGCGTAGCAGGCAGGGAACTGGCCGCGGCGAGCGGGACTGGCCATTGGAGTGCTCCGCT
GCGGAGGGAGGGGACCCCGACTCGAGTAAGTTTGCGAGAGCACTACGCAGTCAGTCGGGGGCAGCAGCAAGATGCGAAGC
GAGCCGTACAGATCCCGGGCTCTCCGAACGCAACTTCGCCCTGCTTGAGCGAGGTAGGGGGCGGCGAATGGCGGGGGTCG
CGGGCGCGGGGAGCCCGGTTGTGGTAGGGCTTAGGTGTCAGAAACGAATTTTTAAGTCGAACGAGGCAAACAAAAACTTA
GAAAACATGCATCTCCGGGCGCTGAAAATTTGCATCTCTGGGCTACTAAGGAGCCCATCTCGATCAGATTGTTCTTGGGC
ACTTGTGATTTATGGAGTTCAGAAAAAAGGTTAATTCCCAACTGTGGAACCTACGGGTGTAGGGGCAGTGAAAATTGCAG
TTGGTCGGAGGAATAGGAGGGAAGGCGTTAAATTTTGGAAATCCTATAGACAAAGAATATGGTGGGTGGTGTGGAGCGTG
GGTAATGGCTTAAATTAGTACTGCCCAGGAGCAGGGTATAACATTAGTTGGGGGAAAATGCGGAGCTTCTTGGCTTGAGC
AGCCTCTGCTGTTCGCGGAGTTGCTGGGATTGTTCTCATCTCTATTACCTCATTTTATTGCATTGCTGCATTACCTTGAA
ATATTTAAGCAACTTAGAAGTAGACTTCTGCTGACCAGTGCCGTCTTAGAAAGTTTCTTTGTAACTTTCTAGTCCTAGGA
GAGGAGTCAACGTGTTGGGGGACTGCAGTAAATAAAACTGCCCACGCAAATTTAGCAATGCACCAGGGAGCAGTTTAATG
TGAGTTTCTGAAGAGTAGTTTTGCAATCGAAATTAGAAATGTGGTAGCATGTATTATGGCTGTCATGAGTAGTTTGATTT
CTCTCTTTTGTGTATGTTTTGGCTAAACTAAACAAAGCAAAAAATATTTATGCAATCAACTAATGTACGAAACTTCTGGG
ATTCGCTGCAATCTGTTATGACTGGTTTTAACTGAATACCTTTAAATTTACTTGCCCTGCTGGGGTGGGAGGNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNGGGG
GGGCGCGGAGGGAGGCGGGTTGAATATGAAGAAGAAAGAAAAGGCAGTGGCTGGACGGAGTCTTCTAAGCTTCCCTGTTC
ACAGCAAGGCGGGACGGGCCTGACTGCCCACACCTCACTGCGCCCTCGGACCCTAAGCACACCTGCTCCTAGAGAGTTAT
TAATAGAGCCCCCAAATGGCTTACGCTTTCAAATGATTTTCCTGCAAATCTGATTTCTTGCATGGCACATGGCAATTAGG
AGGTGGATGCTAGTATCTTTGACCTGGGGGGCTGGGGGTGGGCATGGGGGACAGAGGTGTGTGTCTTCGCCCTCAGACTG
GCAGTTGAGTCTTTTCCGAGGAGAGACGTGGGCTGGTATTGAGCGTGCAGCTAAGCTGGTGACTGCTTCAGAGAATCTCA
TGGCTGGAAATCCGGGGGTGAGGGGGTGTGGGGTGGTGAGAGGGCAGGAGTGGAGTTAAAATGGGGTGTAGTTCTCTAGG
TTTCCCATTCCTCCCACTCCTTGTTTTCCTTTTCATCATCTTTTTTTTTTTTGTCAACCCACAGCCAACATCGTAATTCCC
```

```
ATTTCCCTTTGAGTGAATGTGGCCATAGAGTTTCTCCCTCTCTTTTTCTTCCCCCTCTTCCTTTTTTGAAAAAAGAAAAA
AAAGGAAGAAAAAAGATAATTTGATTTTTAAAAAAAATCTCTCTCCCTGAGCTCTCCCCAAGTTTCTTTAAAACAAATCA
ACAACAACACAATTGGAGCAGTGCCTTAGCTAGAATGAATTATGCCAGTATGCTGTGGCTCTTTCCCTGACTCTCTCCTC
TGACTTGTTTAAGGCTGCGGTTTCCGAGGCCCTCTCCAGCCAAGGAAAAGCTACACAAAAAGCCTGGATCACTCATCGAA
CCACCCCTGAAGCCAGTGAAGGCTCTCTCGCCTCGCCCTCTAGCGTTCGTCTGGAGTAGCGCCACCCCGGCTTCCTGGGG
ACACAGGGTTGGCACCATGGGGCCCACCAGCGTCCCGCTGGTCAAGGCCCACCGCAGCTCGGTCTCTGACTACGTCAACT
ATGATATCATCGTCCGGCATTACAACTACACGGGAAAGCTGAATATCAGCGCGGACAAGGAGAACAGCATTAAACTGACC
TCGGTGGTGTTCATTCTCATCTGCTGCTTTATCATCCTGGAGAACATCTTTGTCTTGCTGACCATTTGGAAAACCAAGAA
ATTCCACCGACCCATGTACTATTTTATTGGCAATCTGGCCCTCTCAGACCTGTTGGCAGGAGTAGCCTACACAGCTAACC
TGCTCTTGTCTGGGGCCACCACCTACAAGCTCACTCCCGCCCAGTGGTTTCTGCGGGAAGGGAGTATGTTTGTGGCCCTG
TCAGCCTCCGTGTTCAGTCTCCTCGCCATCGCCATTGAGCGCTATATCACAATGCTGAAAATGAAACTCCACAACGGGAG
CAATAACTTCCGCCTCTTCCTGCTAATCAGCGCCTGCTGGGTCATCTCCCTCATCCTGGGTGGCCTGCCTATCATGGGCT
GGAACTGCATCAGTGCGCTGTCCAGCTGCTCCACCGTGCTGCCGCTCTACCACACAGCTATATCCTCTTCTGCACCCACG
GTCTTCACTCTGCTTCTGCTCTCCATCGTCATTCTGTACTGCAGAATCTACTCCTTGGTCAGGACTCGGAGCCGCCGCCT
GACGTTCCGCAAGAACATTTCCAAGGCCAGCCGCAGCTCTGAGAAGTCGCTGGCGCTGCTCAAGACCGTAATTATCGTCC
TGAGCGTCTTCATCGCCTGCTGGGCACCGCTCTTCATCCTGCTCCTGCTGGATGTGGGCTGCAAGGTGAAGACCTGTGAC
ATCCTCTTCAGAGCGGAGTACTTCCTGGTTAGCTGTGCTCAACTCCGGCACCAACCCCATCATTTACACTCTGACCAA
CAAGGAGATGCGTCGGGCCTTCATCCGGATCATGTCCTGCTGCAAGCTGCCCAGCGGAGACTCTGCTGGCAAATTCAAGC
GACCCATCATCGCCGGCATGGAATTCAGCCGCAGCAAATCGGACAATTCCTCCCACCCCCAGAAAGACGAAGGGGACAAC
CCAGAGACCATTATGTCTTCTGGAAACGTCAACTCTTCTTCCTAGAACTGGAAGCTGTCCACCCACCGGAAGCGCTCTTT
ACTTGGTCGCTGGCCACCCCAGTGTTTGGAAAAAAATCTCTGGGCTTCGACTGCTGCCAGGGAGGGAGCTGCTGCAAGCA
GAGGGAGGAAGGGGGAGAATACGAACAGCCTGGTGGTGTCGGGTGTTGGTGGGTAGAGTTAGTTCCTGTGAACAATGCAC
TGGGAAGGGTGGAGATCAGGTCCCGGCCTGGAATATATTTTCTACCCCCCTGGAGCTTTGATTTTGCACTGAGCCAAAGG
TCTAGCATTGTCAAGCTCCTAAAGGGTTCATTTGGCCCCTCCTCAAAGACTAATGTCCCCATGTGAAAGCGTCTCTTTGT
CTGGAGCTTTGAGGAGATGTTTTCCTTCACTTTAGTTTCAAACCCAAGTGAGTGTGTGCACTTCTGCTTCTTTAGGGATG
CCCTGTACATCCCACACCCCACCCTCCCTTCCCTTCATACCCCTCCTCAACGTTCTTTTACTTTATACTTTAACTACCTG
AGAGTTATCAGAGCTGGGGTTGTGGAATGATCGATCATCTATAGCAAATAGGCTATGTTGAGTACGTAGGCTGTGGGAAG
ATGAAGATGGTTTGGAGGTGTAAAACAATGTCCTTCGCTGAGGCCAAAGTTTCCATGTAAGCGGGATCCGTTTTTTGGAA
TTTGGTTGAAGTCACTTTGATTTCTTTAAAAAAACATCTTTTCAATGAAATGTGTTACCATTTCATATCCATTGAAGCCGA
AATCTGCATAAGGAAGCCCACTTTATCTAAATGATATTAGCCAGGATCCTTGGTGTCCTAGGAGAAACAGACAAGCAAAA
CAAAGTGAAAACCGAATGGATTAACTTTTGCAAACCAAGGGAGATTTCTTAGCAAATGAGTCTAACAAATATGACATCTG
TCTTTGGCACTTTTGTTGATGTTTATTTCAGAATGTTGTGTGATTCATTTCAAGCAACAACATGGTTGTATTTTGTTGTG
TTAAAAGTACTTTTCTTGATTTTTGAATGTATTTGTTTCAGCAGAAGTCATTTTATTGGATTTTTCTAACCCGTGTTAAC
ACCATTGAATGTGTATTTCTTAAGAAAATACCACCCTCTTGTGCCCTTAAAAGCATTACTTTAACTGGTAGGGAACGCCA
GAAACTTTTCAGTCCAGCTATTCATTAGATAGTAATTGAAGATATGTATAAATATTACAAAGAATAAAAATATATTACTG
TCTCTTAGTATGGTTTTCAGTGCAATTAAACCGAGAGATGTCTTGTTTTTTTAAAAAGAATAGTATTTAATAGGTTTCT
GACTTTTGTGGATCATTTTGCACATAGCTTTATCAACTTTTAAACATTAATAAACTGATTTTTTTAAAGATCCCTTTGTG
AAGAGCATTCTTAAAAATAATTATTGAGTCATCTACTGGATTGTGTAGCTCTTTGGAATCAAAGTGAAGGACAAATTTCT
ATTATTCTTTAAAGGATTTTTACCTTAACTAGGGTGTGAAAAAAGTGTGGTTTGTTTAGAAACCACTCTATCTTTTTCAG
GAAGAGAAACTTCAATAAGTTAACACTGAAGAGTAGTAAAGAATGAGATAAAAACCACCAACCTTCTGAATACCACATTG
GCTAGTAAGTAGAATTGTTTGAGTTTTAAACTGCATTTAAGCCAAATAAATTAAATGCAATAATTCTGTCATCAAATATG
GGCTTTTAAAGAAATTATACACAAAATGTGGTTGTAAATCTAGTTTAAATGATGAGACTCAATCATCATTCAAAACCCA
TCAAAAAAGCATACATTCTTGGCCTTTCACCAACATGTTGGGGAAGTGGGGATATGGATATGGGTATTAAATCATGTGTT
TTTTTTTTTTTTTTTTCAGGACACTGTCTTGGCTTCTCCCAGCCTATCTTCTGAACCACATAGAAGTATACAGTATCAGT
GAAGCAGCTAGCTGTAAAATGTGGGCTGAGTAGCATAACACTTTTATCTGAAAACCTTCAATTCATAGTATCTGAAAGCA
TTAATATTCTCAGTCATTAATTTTTCCAACTACAAAATGAAGGCACTTTGCAAGCATCACAGACTTATTGTAATAGTTAA
TTCTTGTTTTTGATTATTGTCATCTTTTGGGGGATTAAAGTGTTTTGGCATGTTGGGCTCTCTAATCTTTTCGCTACTTT
CAGAATTTGTGCTTTCAAGTGTCATTGTATATGCACAGGACACAGTTGGGACATGAAGATAAACCTCACCTAATAGAGTA
GATGTCATTTCTGGGTAAGGTTAACTGTGGGAAACAGAAATGTGTTCACTAAGGAAACCTTAGGTCACAACCTCCAGTTT
CCTGAAGGAAATGCCAAAAATGCTCCAAAGACAAAAGGCTGGAAACAAACTGGAATGTCTCTCTCCATCTGTCTGCTAAG
GCGGAAGGAATGATTTCATGTTACATTCACTTCCTAGACTTTCATTACAGAGTCAAGCATAATGGAAAAGTATGTCCATT
TTGTGTTTCTGTGGAAGATAAAATGTTCATATTCTCCGACTTATCTTCATAAGCACAGTAGTGATTCATGATATGGGGAA
AGATGGGATAAGGTGGCTGGCTTTGGGAGATGGGGAGCTCTTTGGTGACACTTTTCCCCCTGTGACCACATTATCCAGTG
ATTAGATGTACTTTCTTCCTGGAAACTCCAGACTGTGCATTTCATTCCAAGAAAGCTGGGTAGTCATGGACTGTGGTTTT
CCTGGGCACATGTGTGGTAGAGCCCCAGATCAGACCAAGAAGCAAAGACATAAGAAGAAGGAATCCTTGGTTGTCTCCCA
ACATGAATATCTGGTTTTAATTCAGTGTATTCTCAGAAGTAAGAATGAGAAAAAAATCTTAGTAATTATATGTTTGTACT
AAAAAATACACATATAAGGCTTTGATTTTAAAATTGTAGTGTTTTTCAAGAGGAGTGAAACAGATCCTGGACAATACTTG
ACATATTCTTGTAAAAAGTTCAATTGACTCAAAATAGTCAGGAAAGTTATGAAGTAACATCATATAATACTTTATCCCCA
ACAATATTAAATGTGATTACAATTTTACAGAATATTCAAATCAAAATGTTATATAAGACAAATTATTATTCTTGCTTTTA
CATCTCCTTAGAAGGAGAAACATTCAATTGTTTTAAAAATGTTCTATTTATTTTTCAGAAATGAGGTGTTGCCTTCTTGC
CCAGGCTAGTATGCAGTGGCACAATCATAGCTCACTGCAGCCTTGAATTCTTGGGCTCAAGTGATCTTCCTGCCCCAAGC
```

CTCCCAAGTAGCTGGGACCACAGGCACGTGCCACCATGCCTGGCTGATTTTTTTTTTTTTTTTAATGTTTTGTAGAGACAG
GATCTCAGTATGTTGCGCAGGCTGGTCTTTAATTCCTGGGCTCAAGTGATCCTCCCATAGATTTGTCTGTGTTTTCTCTA
AATAGGTTATAAATACCTTGAGGGAGGAATCACAAATAACTAAAAGAAACTTTTGTAGAGACTATCAGAGTATTATTTCC
TTGTGGACACAGTAAATGTATGATGATGAGAAATCATAATAAACTACAACATTTTCTCCAACCTTCCTTCCTGCTCTTTT
GATGTGTACTTCACGTGGTCCAAGAAAAGGGACTCAAATACTTTTTAACATACACCATTGCCTTCACTGCAATACTAAG
TGGAACTAGAGTTGTCTCTCAGAACATTCAAGCAAGCAAGGTTTACTTGAATAACTAAAGGCCTTATATGGATTATCTAT
AAAGAGGATTTTAAACCGGTGGTTTAAAAAGCTGGGAGAGATAGGAGGAAGAGCATAGTGAGGGGTTAGACCTGCATTTA
TTTCAGTCTTTCACAATCTTTGACAAGAACCAGGACCCAGAGTAGTTTGGGTTCATGTTGAATATATCTCTCCTAATACA
TCCGATCTCTTGTTCCCTACACTTTCTTCTTGGATTCAGACTCTCAAGTAATTTCTGTTTTCCCGTGCTCTCTTTTTA
TCCCTTTGAGGACTAAAGGATTAAAAAATTAGACTTAGCTCTGGTTATAGTCATTAGCAAGAGCCAAGTGTGGACTGTGG
ACTTGAGTGAACAGGAATTGCTACATTTTATGTAATCCAACCTTGATATATGAAGTATCTTCATATATCCAACCAGAAGC
TTCCATATCAGTAATGGATAAATACACCATGGTAAATTAATACAATGGAATATTATTCAGCAACAAAAAGGACCTACTA
CTAATCTTGCAACCGTTTTCATGAATTTTAAACCTCTTATGCAACCACAAAAGAGAACATACGTCATGATTTCCTTTATG
TAAAAGTTCCAGAATAGGCAAAACTAATATATGGAGGTATAAATCTGCACAGTGGTTGCCTGGGGTAAGAGGATAAACTG
GAAAGGAACACGAGGGAACTTTCTAGGTTGTTAGCAAAGCTCTTTATCTTAATTTTTAGGACCTTGTTCACTCAGATGTG
TATATTTGTCTAAACTTATGTAATCATATTCTTAAGACCAGTGAAATTTAAAAATATCAATATGCTAGACTTTCACTTCT
GAGTATAAGGACTATATGAGACTTTCTCTGTGAAATTGTGGGAAGGTTCCATGAAGTTCAGTAGAAAGTGGCAGCCGTGC
ACAGTGGCTCACGCCTGTAGTCCCAGCACTTTGGGAGGCCGAAGCAGGAGGATAGCTTTGAGCTCAGGAGTTCAAGATCA
GCCTGGGCAACATGGCAACACCCGTTTCTACAAAAAATACAAAAAAATTAGCTGAGCATGATGGCTCACACCTGTAGCC
CCAGCTATTTGGATGGCTAAGTCTGGAGAATCACTTGAGTCCAGGAAGTGGAGGTTGCAGTGAGCCGAGATCCGGCCACT
GCATTCCAACTCCAGCCTGGGTGACAGAGTGAGATCCTGTCTCAAAAAAAAAAAAAAAAAAAAAAAAAAAAAGAATGTGGAA
AAGGTTCCAAAGTGTTTCATAAAAGGGAAATACGCTTTTTAAGTATTATCCCATGACCCAGAAATAAAACTTTAGAATGC
TAAATAATTGTGACATGATGATACTCTTTGCTTCCATAGGATGAGAATTTGGCTTAGTATTGGTATTTGTAATCCCTGAA
GAATTTCTGTGTCTTGAAAGAGTCTAGAGATGTAATGAAACTGTCACAATAAAAGTTTATTGACATTTGGATTTCAGTTT
TAGGAATATATGATGGGTTGTAAATAGCCTTATGAAGAATGGACCTTGTATACATTGCAAGGATATGTAAACATATTAGT
ACTGGAGACATACAAATTGGAGTGTGCCAATTTCTTATCATGGCCACCTGACCAAACCTATAAAAGGCATTCATTCAGAT
AATAACACTCGCCCTGGGTGTAAGCACTGCATTGTTAGAAGAGATAGCAGAGCACAAAGGATATGGAAGACAAGCTTATG
TTCCTGTGGATCTATTTTTTAATAATTTCAGCTTTTATATTAGATTCAAGGGGAACATGTGCAGGTTTGTTACATAGGCA
TATTGCTGTGATGTTGAGGTTTGGGGTGTGAATGATCCCATCACTGTGGTAGTGAGCATAGTACCCAACAACTAGTTTAT
TAGCCATTGTCCCCCTCCCTCTCTCTCCCTTCTAGTAGACACCGGTGTCTATTGTTCCCTTATTTATTTATTTTGGAGGG
TCTCACTCTGTTGCCCATGCTGGAGGGGAGTGGTGTGACCATAGCTCACTGCAACCTGGAACTTCTAGGCTCAAGCAATC
CTCCCGTCTCAGCTTCCAGAGTAGCTGGGACTATAGGTGTGTGCCACCACACCTGGCTAATTATTTATTTTTGTAGAGAC
AGGGTCTCACTTTATTGCCTAGGCTGTTGCCACACTCCTTACCTCAAGCAATCTTTCCACCTTGGTTTTCCAAAGTGCTA
GAATTACAGGCGTGAGCCATCATGACCAGCCTGTTTCCATCTCTATGTCCACGTGTACCCATCCTGTGAGTTTTATAAGC
TTGCAGCCAGACACATTGTTACCTGCATTCCCCTGTCTGAGGGGTCTTTCTTTCAGCCATCTCTGGGAGTAACTGGACAG
AGAAGAAATGCCTCAGGTGCTGCACTGCTCCTTGGACAGACTGATTAGATACAAAGCAGAAATAGAAACAGGGAAGAGGG
GGCATGGTTACCTCAGCTAACAAGGAGCAGGGAGACGTAGGAAATTGTTTATCCATTTCCCCAGAGCTCTACTACTATTA
TTACTCCACTTTTCTGCTGCTTTGTTGATACCAGGGCAGAAAGAAAAACCTTGGAAAAAGTTACTGGTGTGGGTCATGCT
TCCTACTCCCATTCCTCTTGATAGGTCGGCAAACTTACCCACTCATCCACTCAACAAGAATGCTTGCCCTGTGCTCTGTG
CTGTGGATCTAGGGGCAAATATAAACAGAGGTTCTGCCTTCCTAGAGCTCACAGGATTCATTCCTTCAGCTCTGGCACTC
CTGTGGCTCCCTCTCTTTGGGGTCCAGGGACCTTGATATGGCAAGACAAAATTCTTTCTTTTTTTTTCCTTTTTTTGAGACG
GAGTCTCACTCTGTCGCTCAGGCTGGAGTGCAGTGATGCGATCTCGGCTCACTGCAACTTCTGCCTCTCAGGTTCAAGCG
ATTTTCCTGCCTCAGCCTCATGAGTAGCTGAGATTACAGGCGTGTGCCACTACGCCAGGCTAATTTTTCTGTTTTTTAGTA
GAGACGGGGTTTCACCACGTTGGCCAGGCTGGTCTCGAACTCCTGGGCTCAAGCGACCCACCTGCCTCGGCCTCTCAAAG
TGCTGGGATTATAGACGTGCATCACGGCACCCAGCCAAGAAACAGTTCTTTTTTTCCCATGAGTTTGTTCATTGTTGTAC
ACTTCCTCTAATATATTGAGTTCTTTAGCAGTCTAAAATAATTTCATTTAACAAATACAATTAATATTGGCAGCTTTTCA
TCATTGGCTTTTATTTAATTTATATCTCTAAATTACTTAAAATTAAATAATGAAGAATAAACTCATATTTGGAATGGAGA
GAAGTTCCTTGTGTTATTGGGTGGTGTTGTAAATATCAGTATTTGTATTCTATTTTTCAGCATCTAGTCCCCATGTGATC
AAGAAGCAACTCCGTAAAATAAAAGCATTCAACATTGTCTTCAGTTTCTATCTCTCCTTAGTTTGTGTTTTTCTTCCTAG
ATCCCTATTGTGAACTGTGCTAGGTGGAGCTGCTTGGTTTATAATGTGCAGATGAAGTGTACGTTTGAATAAGTCATAGG
TGGTGTCCTTATTCAACTTGGATGAAAAGGAAAGGAGAGAAAAATTCACAACTCAAGAGGATAAGCAGACTTATCTACTA
GTGAGCCATTGAAAGTCATGTAGAGGACTTGAGAGAGCAGCCTAGGTAGGTCGTTTGTGTGCTGTATGTTCAGTGATTGC
TTGAGGCCAGGGAGCTGGGGATAACTCCTTGTGCAGCCCAAGAACTCTGCACTCATCTTATACACCAGCTCTACTGAATC
CAAGTTGACCTGCTCACATTTGTGTGCCATTTCTATCAGGAAGTCTCTTGAGAATGCCAATAGCTCAGATACCTGCTCGC
CATTACCAGATCTACCCCTCAAAGCATAATGTCTGCTGGTGGGAGGATGAGTAGCTTCAGTGTTTTGTACAAAGGAGAGT
ATATTTATTTCTAATTTCAGAGTTTCTCTTGCTCCTGTCTAGACACTTCAGAGAAAAACAAATTCAGGTCACCAGTAAAG
TTATGTTCTATAATAAGAGAAGACTTGTGCTAAATCACTTGTAAAGACATTAACTATCATCTGCCTGAAAAGTCTTCTCT
CACCAAGCTTCTGCAGATCTTTTGAGGTACCAGAAATAGTGGTTAGTTCTCACATGCTGTTCTGCTTCAGCGAGCTTCAT
GTGGCCACCTGCAGAAATAGTTCTTGGTTTCTCTTCCTTGTCTAGATGCTGGTCAAGGCAGCGTTTAGAAAAACACAGAT
TTTCATATTCCCATTCACACGTGCAAAGTGAATTAGGGTACCAAAGAATTACCTAGATTATTCAATCTGTAAAATGCTTT
GCCTCAATTGACTGAATATTTTATATCAACACTTTTTCCAGCTACATATTTATACTATAATAGGAACCTATTTTTACTAT

```
ATATACTATTTATACTATAAAAGTAGGAGTTGCAGAATCATAAGAAATTTAGGGCCGGGTGCAGTGGCTCACACCTGTAA
TCCCAGCACTTTGGGAGGCCGAGATGAGCAGATCACGAGGTCAGCGGTTCGAGACTAGCCTGACCAACTTGGTGAAAACT
CATCTTTACTAAAAATACAAAAATTAGCCAGGGGTGGTGGTATGTGCCTGAAATCCCAGCTACTTGGGAGGCTGAGGCAA
GAGAATCACTTGAACCCGGGAGGTGGAGGTTGCAGTGAGCCGAGATTGTGCCACTGCACGCCAGCCTGGGCGACAGAGCA
AGACTCCATCTCAAAAAAAAAAAAAAAAAAAAAAGAAATTTAGAAGACACAACCACAAGAGCTTTCTTTCATGTATTTATTA
AGGAGACAGGGTCTTGCTCTGTCACCCAGTCTGGAGTACAGTGTCGTGATCCTAGCTCACTGCAGCCTCAAACTAAGCGA
TCCTCTGGCCTCAGTCTCCTGTGTAGCTGGGACTATAAGGTGCATGCCATGTTGGCTACTTTTTTTTTTTTTTCCAGTA
GAGATGGTTTTGGGGACAGGGGTCTCACCTTGTTGTCCAGACTGGTCTTGAACTGCTGGCTTCAAGCGATCCTCCTGCCT
CGCCGCCTAAAATGCTGGGATTACAGGCATGAGCCACCGTGCCCAGCCTTGGACCTTCTAATTGTAAAGAAGCAAAAAGG
CCCATATAATATACATAATATACACCATAATATATACATAATATACATCATCTCACTTTATATATCCACTGTATTAATTT
TTCTGTTAAAAAAACTATGCTCGTTAGTGTTTTATGTTGTTTTTTCAAAAAATAGTGATTTTGAATTGTGGGCCTAATT
AGCGAAAATTGAAATGTATTAAAATAATATAAACATCTGTAAGGGATTTTGTATCAAATGGAGTCTGCTCTCAGTTCTTT
TTTTTTAACTTTTATTATTTTATTTTTAATACAGACAGGGTCTCACTATGTTTTTCAGGCTGGTCTCGAACTCCTGAGCT
CAAGCAATACTCCTGCCTAGGCATCCCAAGTGCTAGGATTACAGGCATGAGCCACCATGACCAGCCTCTACTCTCAGTTC
ATTTTCTAAGAAATTTGATGCAAGCCAGTATTGTTGCTGAACTTCATACCAGCGTGAAGAATATCTGGATTGCAGTGTTC
TTTCCTTAAAGGGGTACTGTAGGCACAAGAGTTGTGCCGAGATTTATGTGGTCTTCAGAGATGGAGAAGCGTTTGATTCC
TAGAATTATTTGGCATCTTTCTACACTCATCTGGGTTTTCTCTTAGATTTATAAAAAGGCCAGTGTTGGGCATTCTGACA
AAGTCTAGGTTAAACGCTAATCAAGGCACTAGAGTATATATTTGAAGTTTGTGTGTTAACACCTATGTGTTCGGGGCAAC
AAACTACTAGTGTCACTAGTTAGCTGCCAGTAGTTAGCTGCTCTGAACACTTAGGATACCTAAATATACATCGGATTTTT
CATCAATCTTTTGTTTGTCTTCTCCAAAAAGGAATCAATTAATGAACTAGTTCTGCCCCCAAAGTAACAGTTTAGAATAA
ATGTATTAATTCAATGCAATGTTCAACATTGTTTGAAAAATCTAAAGTATTTTTATAATTTTATTTTTTTCTTTTCCTTT
TTTACCTTTCACACACTTGAATCTAAACATTTTTACTTGAAGAATGGACAGTTATTTTTCTTTGTGTTTTAAAATACAAC
ATGTACCAGGAAAGGGAGATGGAAGTAAAAATAAATAAATAAAATACAACACGCTCTTCAGATAGGTGGCTTTTGGAAAG
CAATCTATGATTTTCTAATTCTCTAGGGCTCAGAGTCTCTTGTAAAACACAGTTCTTTCTATATAATTTTTGCTAATGGA
TAGAAAAACTTGTGGGGTATTTGGAGGAATTAAATAAACATTGAAGATATAAAAGCACACAAGGGATGACTTTTATTGTA
AAGTCATGCTGTCATCAAATGGTGTGATTTCTTCCCTGGTGAGGGCAGTTTTCAGTCTACCACACCTGCCTCTGGTCATG
TCACGGTTGAGTTCTGGCACTGCCAGCTGATGTTACCGGTGGTTTGCTGAATTTTGACTTATATGGAATTAAATGTCTGT
GTTACCATAGTAAGTTCACATTAGTCTTTGCATTTCATTATCAAGAATGCACTGGTAACTGGCAGGAAACTTCTATTTGA
AGCATAGTAATCGGAACATTTTCTTATTGTTAAAGTACTTTTGCTTCTTCCCTACTTTCATTTAAATCTATCTAAAGGAA
AAAAGAGGATTTTGTCTTCCATACAAAAGAAGTTTTAGACATTGGGGCTTACTATAGTTTCTCTGTGTTATAAACGATCT
CCATTCTTTATGTCACTCCATGAACTTTTATTTAATTTGGAGGAAATCCATAGTATTCACTCTATATCAGAGGCTGTGAC
ATCCTGGCTAAAGCTATTGAAGTTGGCAGCATCATTTCCTCTTCCCGAATTCCTTTTTTGGTTAAGTTTAAGAAGGAGCA
CAAATTGAGGGTGCAACTTTGTTTCCCGTAGTTTTGAACTGGCAGGGTGTCCCTGGGATTTGTGGCTGCCTACTTGAACA
ATCCCTTAGGACAGACACCATTCACATTTCATGGCTCTCACTAGGAATCATTAACAGCTCAGTGCAGAGTTTTGCTTTTC
ACTTAACTTGTAAGATAATGAAGAGGCCCTGCCAAGGTTCAGAAAAGGATAACAAAGCATAAGCCCCTTAATGGGATGAA
CTAACGTAAAAGTCTAAGTATCTGGAGCTCTTTCCCACAAGTAGGACCTGACTCAGGGACTGGCATTCAGGCTGACAACC
ACCATATACAAGAAACTGTAATAGAAACGAAAGTAATTTCCCTAAAATGAGCCAAGTTATGGGCTTTGGGAAAATAAGGA
TCTGTTAGCTTTGCTAAGATCAGGATCTTTGATTTACAGTGCTTTCCATGAAACGGTATATTTTATTGAAGAGAATCAT
AGGATCTAGAAAGCATTGACTTTAAGTGAAGCACTATTTGAATTCAGCTTTAAGTAGACTGAAATAGCTCTTTATATGAT
TGAAATGTCTCCATATAATTGTTTGTTAATTAACATATCTCATAAAATGGCATTTTATATACCATGCA
```

HUMAN mRNA SEQUENCE : hR6-131.1 (Seq ID No: 67)

```
TGGCGCGGCGCTGGGTGACTTCGCGTAGCAGGCAGGGAACTGGCCGCGCGGCGAGCGGGACTGGCCATTGGAGTGCTCCGCT
GCGGAGGGAGGGGACCCCGACTCGAGTAAGTTTGCGAGAGCACTACGCAGTCAGTCGGGGGCAGCAGCAAGATGCGAAGC
GAGCCGTACAGATCCCCGGGCTCTCCGAACGCAACTTCGCCCTGCTTGAGCGAGGCTGCGGTTTCCGAGGCCCTCTCCAGC
CAAGGAAAAGCTACACAAAAAGCCTGGATCACTCATCGAACCACCCCTGAAGCCAGTGAAGGCTCTCTCGCCTCGCCCTC
TAGCGTTCGTCTGGAGTAGCGCCACCCCGGCTTCCTGGGGACACAGGGTTGGCACCATGGGGCCCACCAGCTCCCGCTG
GTCAAGGCCCACCGCAGCTCGGTCTCTGACTACGTCAACTATGATATCATCGTCCGGCATTACAACTACACGGGAAAGCT
GAATATCAGCGCGGACAAGGAGAACAGCATTAAACTGACCTCGGTGGTGTTCATTCTCATCTGCTGCTTTATCATCCTGG
AGAACATCTTTGTCTTGCTGACCATTTGGAAAACCAAGAAATTCCACCGACCCATGTACTATTTTATTGGCAATCTGGCC
CTCTCAGACCTGTTGGCAGGAGTAGCCTACACAGCTAACCTGCTCTTGTCTGGGGCACCACCTACAAGCTCACTCCCGC
CCAGTGGTTTCTGCGGGAAGGGAGTATGTTTGTGGCCCTGTCAGCCTCCGTGTTCAGTCTCCTCGCCATCGCCATTGAGC
GCTATATCACAATGCTGAAAATGAAACTCCACAACGGGAGCAATAACTTCCGCCTCTTCCTGCTAATCAGCGCCTGCTGG
GTCATCTCCCTCATCCTGGGTGGCCTGCCTATCATGGGCTGGAACTGCATCAGTGCGCTGTCCAGCTGCTCCACCGTGCT
GCCGCTCTACCACAAGCACTATATCCTCTTCTGCACCACGGTCTTCACTCTGCTTCTGCTCTCCATCGTCATTCTGTACT
GCAGAATCTACTCCTTGGTCAGGACTCGGAGCCGCCGCCTGACGTTCCGCAAGAACATTTCCAAGGCCAGCCGCAGCTCT
GAGAAGTCGCTGGCGCTGCTCAAGACCGTAATTATCGTCCTGAGCGTCTTCATCGCCTGCTGGGCACCGCTCTTCATCCT
GCTCCTGCTGGATGTGGGCTGCAAGGTGAAGACCTGTGACATCCTCTTCAGAGCGGAGTACTTCCTGGTGTTAGCTGTGC
TCAACTCCGGCACCAACCCCATCATTTACACTCTGACCAACAAGGAGATGCGTCGGGCCTTCATCCGGATCATGTCCTGC
TGCAAGTGCCCGAGCGGAGACTCTGCTGGCAAATTCAAGCGACCCATCATCGCCGGCATGGAATTCAGCCGCAGCAAATC
GGACAATTCCTCCCACCCCCAGAAAGACGAAGGGGACAACCCAGAGACCATTATGTCTTCTGGAAACGTCAACTCTTCTT
```

```
CCTAGAACTGGAAGCTGTCCACCCACCGGAAGCGCTCTTTACTTGGTCGCTGGCCACCCCAGTGTTTGGAAAAAAATCTC
TGGGCTTCGACTGCTGCCAGGGAGGAGCTGCTGCAAGCCAGAGGGAGGAAGGGGGAGAATACGAACAGCCTGGTGGTGTC
GGGTGTTGGTGGGTAGAGTTAGTTCCTGTGAACAATGCACTGGGAAGGGTGGAGATCAGGTCCCGGCCTGGAATATATTT
TCTACCCCCCTGGAGCTTTGATTTTGCACTGAGCCAAAGGTCTAGCATTGTCAAGCTCCTAAAGGGTTCATTTGGCCCCT
CCTCAAAGACTAATGTCCCCATGTGAAAGCGTCTCTTTGTCTGGAGCTTTGAGGAGATGTTTTCCTTCACTTTAGTTTCA
AACCCAAGTGAGTGTGTGCACTTCTGCTTCTTTAGGGATGCCCTGTACATCCCACACCCCACCCTCCCTTCCCTTCATAC
CCCTCCTCAACGTTCTTTTACTTTATACTTTAACTACCTGAGAGTTATCAGAGCTGGGGTTGTGGAATGATCGATCATCT
ATAGCAAATAGGCTATGTTGAGTACGTAGGCTGTGGGAAGATGAAGATGGTTTGGAGGTGTAAAACAATGTCCTTCGCTG
AGGCCAAAGTTTCCATGTAAGCGGGATCCGTTTTTTGGAATTTGGTTGAAGTCACTTTGATTTCTTTAAAAAAACATCTTT
TCAATGAAATGTGTTACCATTTCATATCCATTGAAGCCGAAATCTGCATAAGGAAGCCCACTTTATCTAAATGATATTAG
CCAGGATCCTTGGTGTCCTAGGAGAAACAGACAAGCAAAACAAAGTGAAAACCGAATGGATTAACTTTTGCAAACCAAGG
GAGATTTCTTAGCAAATGAGTCTAACAAATATGACATCTGTCTTTGGCACTTTTGTTGATGTTTATTTCAGAATGTTGTG
TGATTCATTTCAAGCAACAACATGGTTGTATTTTGTTGTGTTAAAAGTACTTTTCTTGATTTTTGAATGTATTTGTTTCA
GCAGAAGTCATTTTATTGGATTTTTCTAACCCGTGTTAACACCATTGAATGTGTATTTCTTAAGAAAATACCACCCTCTT
GTGCCCTTAAAAGCATTACTTTAACTGGTAGGGAACGCCAGAAACTTTTCAGTCCAGCTATTCATTAGATAGTAATTGAA
GATATGTATAAATATTACAAAGAATAAAAATATATTACTGTCTCTTTAGTATGGTTTTCAGTGCAATTAAACCGAGAGAT
GTCTTGTTTTTTTAAAAAGAATAGTATTTAATAGGTTTCTGACTTTTGTGGATCATTTTGCACATAGCTTTATCAACTTT
TAAACATTAATAAACTGATTTTTTTAAA
```

HUMAN PROTEIN SEQUENCE : hP6-131.1 (Seq ID No: 68)

```
MGPTSVPLVKAHRSSVSDYVNYDIIVRHYNYTGKLNISADKENSIKLTSVVFILICCFIILENIFVLLTIWKTKKFHRPM
YYFIGNLALSDLLAGVAYTANLLLSGATTYKLTPAQWFLREGSMFVALSASVFSLLAIAIERYITMLKMKLHNGSNNFRL
FLLISACWVISLILGGLPIMGWNCISALSSCSTVLPLYHKHYILFCTTVFTLLLLSIVILYCRIYSLVRTRSRRLTFRKN
ISKASRSSEKSLALLKTVIIVLSVFIACWAPLFILLLLDVGCKVKTCDILFRAEYFLVLAVLNSGTNPIIYTLTNKEMRR
AFIRIMSCCKCPSGDSAGKFKRPIIAGMEFSRSKSDNSSHPQKDEGDNPETIMSSGNVNSSS*
```

Table 12

MOUSE GENOMIC SEQUENCE : mD7-046 (Seq ID No: 69)

```
ATGACATGAAAAATTAATTTTCAGTGAGGCTAATTCAACAATAATTTATAAATTTGGGGTTTACATTATGCACAGATTTG
TATACCAGGTCAGAGGAACTATGTACATACCAAGAATGTTGTTCTTTGTAACTCAAAAGGTTAAAACACAGATATAACTC
TGCCTCTCCTTCTAGTATCTGTTTCTTCTTCAGCAGCTAGCCAGACTTCAATTAGGATCAATGTTCTCATCTGTAAAATA
GGGTTGATTGTTTTTATATATTTTTTAAATTGGTAATACATAGTCTTTATTAGGAATGCTAAGAATTGTTAATGTCTTAT
AGTAACAATTGATAAGTATTTACTGTGTTCACATTGCATTGTGATTTTGTTTTATTTTAGTAAGATGGAGTCTCATTAGA
TAGCCCAGACTTTCCTCCAAGTCTCAATCCCCCTGCCTTAGCCTTCCAACCACTGTTATTACTGCTATGTGTCAGCATGC
CTGGCTTTGGTTTAGCTTATGTGTCAAATTCTCTAGTACCTAGAGGTACACTGTAGTTGATATTATTCTCTATTTTCAGT
AATGAAGAAATGCATTTCTTTCTTCTTTTTTTTTTTTTTTTTTGGTTTTTTGGTTTTTTGAGACAGGGTTTCTCTGTGTAT
CTCTGGCTGTCCTGGAACTCACTTTGTAGACCATCCTGGCCTCGAACTCAGAAATCCGCCTGTCTCTGCCTCCCGAGTGC
CGGGACTAAAGGGGTATACCACCATACCCGGCTAAGAAATGCATTTCTAATCACCATGTTTGGATTATACTCATCTGATC
ATAAAGCTGATTCTAGTGATCATAAGGTATTTTGTGCTTTTATATTTCTGTTGCTATCTAAAGGAAGCTATAATGTAAAA
ATACATACTTTACATACTGCTAGCTAAAATAGAGGCATGACTTTGAATTATTGGGTAAATATCAACATAGGAAAATATGC
TATATTAAGGACATCTGGTGTGAGCAACAAATGTCCTAAGTTGGAAGAGATGAGTTGCAGGTGTCCCTCAGGTGTCCTGT
TGCCAGAACACAGGGAAAAGGAATGGTCATAGGTCACCGTGTCCATTGCATTGAAGGAAAGCAGCAGGTCACTGGGGCCT
TCTCTCCAGTTCTAGTGTCTGAATGGATGTTCCTTCCTTAAAAGCCAGTCCTACAAAGATATCAAAAATACACATTAGAA
AAAAGGCAGCACCTTCAATAAATGGTGCTAGGACAATCTGTAGAAAAATGAAACTAGGTTCTTATCTCTCACCATGCACA
AAGCTCAACTCCAAGTGGATCAAGAATCTCCATGTGAGACCTCACACTTAGAAACTGCCAGAGAAAAATGTAAGCAGTCT
ACTTTTATAGGGAGACACATGTAAGGACATTCTGAACAGCACTCCAGTGGCACATCAGTCCACAAATGAGATAACATGAA
ACCAAAAGGCTCTCTACAACAAAGGGAACTGTCAATTGAGTAGGAGGCAGCCCACAGAAAGGGGGATTTGCCATTCATAC
ATTTGACAAGAGGATTCATGTCTTAGGATTTCTATCGCTGTGAAGAGACTCCATGACCGCAGCAACTCTTATAAAGGAAA
ACATTTAAGTTGGGACTGCCTTACAGTTTCAGAGAGTTAGTCCATTTTTGTCATTGTGGGGATCGGGTGGGGAGCATGGG
ACTCAAGCAGATGTGGTGAAGGTGGAGTTGAGAGTTCTACGTCTGGATCTGCAGGCAGCAGGTCTTCCTGTGACTCTGAG
CCACTGAGCCCGAGCTTGAGCTTCTGAGAGCAAAAAGCCCAGCCCCTTATGACGCATTGATATAGGGTACTGGCTAGTTT
GTGTCAACTTGACACAGCTGGAGTTATCACAGAGAAAGGAGCTTCAGTAGAGGAAATGCCTCCATGAGATACAACTGTAA
GGCATTTTCTCAATTAGTGATCAAGGGTTTCAGGCCCCTTGTGGGTGGGGCCATCTCTGGGCTGGTAGTCTTAGTTCTAT
AAGAGAGTAGGCTGAGCAAGCCAGGGGAAGCAAGCCAGTAAGGAACATCCCTCCATGGCCTCTGCATCGGCTCCTGCTTT
CTGACCTGCTTGAGTTCCAGTCCTGACTTCCTTGGTGATGAACAGCAGTATGGAAGTGTAAGCCGAATAAACCCTTTCCT
CCCCAACTTGCTTCTTGGTCATGATGTTTGTACAGGAATAGAAACCCTGACTAAGACACATAGTTTTTTGTTTGTTTGT
TTGTTTGTTTGTTTTGTTTTGTTTTGTTTTGTTTTACAAAGCCACACCTTCTCCCACAAGGCCACCTTACTCCATCAAGA
GCACACCTCCTCCAATAAGATTGATCACACCTATTCCAACAAGGCCATACCTCCTAATAGTGTCACCCCCTATGGACCTA
TGGGGACAATTTTAATTCGAACCACCACAATAAGTATCTACACTATACAAAGAACTAATAAAAACATAAACATCAAGAAA
ATTAAGAAAAATTAGGCTATGAAACTGAACAGGGAGGTCTCAAAAAAAGAAAAAAAAAGAAATGCAGATGTTTAGCAAAT
```

```
ACGCTTAAACACATTCAGTGGCCTTAGCCGTCAGGGAACTGTAGATTCAGACTATCTTAAGATTTTATGTCACCCCAGTC
AGATTGGCCCTCATCACAAAATCAAATGGTAACAAATGCTGGTGAGGATGTGGGAAGAGGAGACCCTGCACACTGTTGGT
GGAAGAATAAACCAAAGCCACCACTTTAGAAAGTCAATCTGAAGGTTTGTCAAAAAAGTAGAACTCCCATGTGACCCAGC
TGCCCACTCCTAGGCATATAACCAATGGAACCTATATCCCGTACAGAGGTAATTACACACCCACGTTCATTACAATATG
CAGGAAAGGGAAGTGCCCTCGGTAGCTACCATCTGATAAACAGATAAAGAGAATGTAGTACATATACACAATGCATTTCT
ATTCTGTGCTGAGCGAATAAAAGCAGCACAGATTTAGTATCAGAGATATTGGCTCAAATCTCAGGTTTGTAACTCATTGG
CTCATTGAAGAAATTCCTCTACTGTGAACATTGTCCCCCCTTGGAGAACATGTACTGCCTGTGCTGGTAGCCACTAATCT
CATGTTGGTATTGTCATTACAATCATGATACACATTGTGATCCTCTACTGCCCACCACATTTCAAGAGCTTCACTGTTCT
GGGTGGCTCACAGTCATCATATGTCACATTTGCGTCGTCGTCGTGGTAAATCTACTAAAACCCAGCAAAGCTCTTGTTCATTT
TAACTGGAGTAGTGCTTCTGATTTGTGTGACGCAAAACCATAAATTCATTTGTATCTGTTCTCTCCTGTTCATGGCTATT
TGTTGATTCGGCATTCACCTTGTGCCAACTCCTATTCCCTTTCATTCTTTGAATGTCCCTTGAGGTTAGTGTCCGTTTAC
TTTCGGATAGAGAGGAAAGCTTGAGCTTGCGAAGATTAATCAATATGGCTACCAAGATGCTTGAGCAGCTGTCTGCAGAG
AGGAGCTTGGAGTCTTCAAGCCACTGTAGAGTGCCACCTCATTTGAATGTCTTTGTTAGTTGCTTTTCTATTGCTCTG
CTAAAGCACCATGGCAAAGGCAACTTAGTTTATCTGGGCCTGTGGTTCCAGTGGAATTTGAGTCTTTCATGACAGAAGCT
GTAGGCATGGTGACTGGATTAGCAGGTTGAGAGCTCACATCTGGAACTGGAAACAAGAGGAGAGAGAGAGAGAGAGACAGAG
AGAGACAGAGAGAGAGACAGAGAGAGACAGACAGACAGAGAGACAGACAGAGAGAGACAGACAGACAGACTTGAGAATAG
CTCATGGCTTTGAAACCTCAGGGAAATCCTTCCTCCAGCAAGGCCACTTCCTAAACCTCTCCAAACACTGCTACCAGCTG
GAGACCCAATAGTCAAACATCTAAACCTATAGGGGACACTCTCATTCAAATCCCCACAGCCCTCATCCTCAAAAGGTGTG
AAAGCATCATGTAAGGCAAAAGCCCCCACACCGTTCTCTTCCATATCGGAATCTTCCCAAACAGTCACAAACAGAAA
GGGGAGTCCTACACCATTCCTGTGTCAATAAAGTGGAATCAAATTCCACATGAGCCAATCCAGAAAAGCTGGCCATAGAT
GGAGGCAAGGCTTTACTCGTCCCTAGTTTGTGTTGCAAAGCCCACAGTATAAACATAGATAGACACCAGCGGTTCACCTG
GATAGCTGATGTAAAGGAGGGCAGGAAAGAAAGACATACTATAGTCTACTTTGAAATCAAAGTTATAGGACTAGGGAGAT
GTCTCAGTGGGGAAAGGGTTTGCTACCCAAGGGTTTTGATTCATCAGCACCCACTCTTGAAAAGCAGAAATGGGGCATCT
CCAAGGTAAGCTGGCTAGCTAGACTAGAGAAATTAGCAGACTCTGGGTTCAGCAGAAACCCCTGCCTCACTATATATAAA
CAGTCAATGTAAACTCCAGGCATCAACTGTTGAATCTCACATGCATGCAAACATACATACATGTGTAACCATACACACAA
GGCACATGCACACCCACCCATCCCCCCACACACTCCTGTTATCTAGGTACCCATGCACCCACTCTCCTTTTTATTCAGCT
GGCTGAGTTTTCTGTAATTCCTTTCAAGAAAAAAGGAGATTAAATTTTATTTGACAGAGTACCTTTTGAAATAGGAAGAA
TGGAGAAATATTACTAAGCTAAATCAATTTGAATATTTTCTCAGGCTACACTATTGAATTCTGTGAAGATAGCATACACT
AAAAAAATGGCATCCAGCTTAATCAGGCAGTCCAGGGTTGATGAGATGGTAGGTGTCTCCAGAAGCCCTGAGTGGCACGCT
GCTTTCTGAGCAGGGATCTGAGGGACAGAAATAGACTCTGCCAGCAGGGCAGCCAGCGAGAGTGATGCTTCTGCCCCTCT
CAAGAAAAGTGGCCTCTGTCTGCCACTTGTTCAGAATTTCCACAAGAATTCCGGACCCTTTTTCGTCTGCACTCTCTGTA
GCCTAAAGTAAGAGTGGGGCCTGACAGCCAACCAGGCCTTTGAACGTCAAGGTTAGCTTCTCTCCGAGAGCTGAAAACAC
TGCTTCCAGGAAGAAGTTCTCTTATGGTTTGTTTTCTTACTAGCAGATTCAACTACTGTTAACTGGAAAAACAGATTTTC
AAAGGACACAAAGTCTGGCTTCCTTTTACAATCTCCTCCACAGTTCCCCGGATGGTAGACAGCGTACCTGACTATGGGTC
TCGGATATCATTATGTCGGTCTGGCTAGGCTGCCACAGCCTGAGTTCTAGTTTGAAAGTGCACATGTGAAATTGAAAACT
AATGTTGGAGAAAAAGAATAGATTGTGTGTATTTACTTCTAGAGGGGCTCAGTGTTTATTTTGTTGGCAGAAACCTAAGT
AGGGTTATTATACGTCCTTTCAGCTGGCTGTCACTTTGTTCTCAGACTTGAGTTCTGAACTGTCCAGAGTAGATTGGATG
GGTCTGGTGAGGAAACTTCTCGGCTATGTCAAGGAGCTCATTGTGGGCTTTTATGTACTGTGCCTTTAGAATCAGTCAGG
GCTTCTCCATAAGGTCCCTGGAAAATATCTACTCTGGCCATTACTTCTCACGATAACAAATAAGGAATTATCTCATGAAC
CAGATAGGCCATTTGTAAATATCTCTTTATCTTTTTTTAGTTGCTGAGGACCCCAGAGGCAAATAGGTTTTGTTGTCTAA
TTGTTGGGGATCATCCAGCACCTACCTGTCTCTGTGGGTTTCTGCCTTTCACGCTCTCTGTTTTACCATTCTACTGTACC
TTGTTTCTTCTTGATCAGTTTTATTATCTTGGTTTGGAATAAGTTTGGAAGAAAGTAGCTGCTGCTGCTTCTCCTTCTTC
TCCCTCCCTCCCCTTCTCCTTCTCCCTCTCCTCTCTCCTTTGCTTTCCTGTCTTTCTTCTTTGTTTGTTTGTTTTTC
ACTTTTAAGGTCACAGGAATTGAACCCAGTGCTAGTTAAGTGCTTGCTATTCTACAGATCCTGATCCGTAGTTAACTGTT
GTTTTGTTTAGTCTTGTCTTGCTAAGTTGTACAGGCAGGAATTGAATTTGTGATCTTTTTGTCTTAGCTCCTGAGTAGCT
TCGATTATGCCATCCTGCTGTGCGAGAAGTAACTTCTTTACAGGGGCATTCAAATGGTGGCCCTATGCAAACTGGGCTTC
CCAGTCAGTCCTTCTGAGCCATCCCAAGTTTTGAATTGAGTACGTATGAATTAGGAAAAGATGTCCCTTCTTCCACACAG
CTCACTTTAACATGTTGCCTTATGTGTCACCTTCTCTACCTGAAGTTCCAGAAGAACTGATGGATCTGGCCTGGTGCCAT
CCCTTCAGTATGAAAAAGTTTGTTTTTTGTTGTTGTTGGTATTTTTGTTTTGTTTTTTCTGTTGGAAAATATTTTATTT
GAAAAATTTATTTGTCCACTTATCCTTTCATGAATGAATTAGTTTTAAGTTGATTTTGAAATAAATACTTCAAGTGCAAG
AAAGCCAGAGATGCGCAAGGCCATTTTAAGGTCTCTCATGAATGTGACATTCTAGAGCGTGAGTCAGTCAATCCGAAGAT
GACAAAACACAGACCGTGTTTGGTGCCAGAAAGGAAGTAAGCAGGTTGTTGTGACTGGTACAGAAAAGCATAGTATGGAG
ATTTGAGAGCCTCTGAAGAGAAGAGAGGATGGATCAGTCATTAAGGTGAGAACTGGAAAGTGAAGAGCCACAGACACCAA
GGGGATGAGAGAGACACTGAAAAGGTGGAGCTAGAAGTGCAAAGGCCTTGAGACTTTAAGAAAAGCAGGTCTGTTGCTAA
TGAGCAAAAAGAGTCGAGATCAGGAAGCCGGTAAGTTAAGTACAACAGAAGGAAGGACCCGGATCCTTTAGACCACTGTA
GGTGAAATTTTACCCTAAATATAATGGGAACTTATTAGTTTAGGATTTATTTATAGAACTGGCTAAAACTTAAAGACTCA
GTCTGGCTGCTGTGGGGTAAACCATGGAATGGAGAACAGTCCACAGCAGTGGTGACAAGGTCGTGATGGTAGAGGTGGAG
TTAGTGGGCATAACACATCAGTGCGATGAAAGCAAACTTAGTGAACAAGAGGAGAAACCACAAGGAAGGAAAAGGCAAGG
ATGACTCTCAGTCTCAGTGAGTGAGTTGGCAGAGTGAAAAGTTAGATGCACCAGTCACGTTTCTTGAAGTAAGGACAGTC
TGTGATGGTAGAGAAGCTGAGAGAGGAGAGAGGGAACTTAAGAAGTCCCACCCGGACACGTTCAGTTCATTTTGAAACAT
CCCGGGATGACAACTGGGGAGTTAAATGTCTGATCTGGATCCCACAGGTCATTAGGCTGCTATTTTAAATGTGGGAGGCA
```

```
GCACTAATTTGTCAGTATTGATGCCATGGCATTTTACAGAAACCTAGGTGCATGATGGAAATGAGACATCCAAGAACCAA
GTCATGAAGGGCATACGGAGTTTGGGTGTGTAAGGAAAAGGCAGGAAATGGCATTAGAGGACTGGGGCTTGGTCAGCGTG
TGATGAGAGAAGGTATTTCAAGAAGAAAGGAACTGTCAACCACACAGAGGCCAAATCACACACATACTACCTTCATACAA
TTTGTTTATAGTTGGTCACCATGGTGACAATACTGCGTGCTGCAACTCAGTGAGTTGTGTCTCTGAGCTGTGTACCTTGC
ATACATACTTTGATTAGTTAGGATTCCGAGACAAATTTAGGAGGAAGTAAATACTTAATGGTGCAGCTGTATTTTGTCCC
CAAAGGCAGAGGTCTTCCCTCACCTTCGCCTCTGTTATATTCACTGCTCTGTCTCCAGTTCTCAGTACTGTTATGAATTT
CCCAGTACCTGGTATGGAGTTGAATGTTTGTGGTGGTTCTTCGCCAGGAATCTAACCAAACTGAAGTCCATCTTTGTTCA
GGGATACCATGCCCCTGGATTCTGCCACCTGTGGTGGCCTTCTCTTGACATCAGATTTATTGTGATGAATATTGCTTTTT
TTCCTAGAATTTAGAAAGATAATTTTGGATGGATGAAACTAAGAATCTGAAATACCTTTCTTAACATGTTAATCTTGTAA
TTGCTATTTACTTACTGGTGCTTTTATCTATGGGGGAGGACAGGTTCCTAATTGTTAGCTTTGTGATGAGCTAGGAAGAC
ATCTTTAGATCATGTGAAGAATGCCTAAATTTGTTGAAGCACAGAGCCATGTCTTGGTATTATACAAACATAGGGCACAT
TGCATCTGAGTTCATTTCCTAAGCATCCCTTTTCTCTCACTGACAGAATTTTCATGGTACTCCTAAATGTAGTGGACCAG
GAATCCCTTGGGCATAGGTGGCTCTAATGCGGTGCTTGAGTTTGTAGAACACTTCTTCTCGAGGGAAGCTTTCATTCTGT
GGTTTTCATTGAGTTTCTGGAGGCAGAGTCCACAGTAGTGGGACACAATGCAGTTGCAGCAGAAGCCCCGGGGGGCTCAT
GAGAACATGTCACTGATGCTAGTGTAGGTATGGACCAATTCCAGGCAGCAGTGTGTCCTGTATGGTGGGCAAGCAGGTGA
CACTTAGTTACCTTCCAGGAGCTCCTATCACAAGCCCTCCACAGAACTCTTCTATATAAATCCAAGACAGCGGTGTCATT
AATTTCAAACCTGTTCCAGAGGAATGTTCTCTGTTAAAGAGGTAAACCTTTCAGTATACAGAAAGAACACACTGTGTTCT
TAATAAAAATGTTCAAGGGGCCAGGCCGTGGTGTCCTTTAATCTCAGCACTTGGGCAGAGGCAGGTGGATCTCTGTGGGT
TTGAGGCTGTCTTGGACTACAGAGTGAGTTTCAGGACAGCCAGGGCTACACAGTGAAACCTGTCTCAAAACAAACAAACA
AACAAACAAACAAACAAAACCCAAAAAAACCAACCAAACCAAACCAAGCCAATCAACCAAACAAAAAGAAATGTTCAAC
AGTATTATCAGAAATGGAGTTGTTAGGATGCAGATACAAAAGGTGCCTGCTTGTGGTCCTGTGATTGGATACCATGTTGT
AAGTTATGAATACCCTCCCACTTCTTTTTAACAGGAATCTTTTGTGTTTGAGCCCAACTGCCTCTTCAAAGTAGATGAAT
TCGGCTTCTTCCTGACGTGGAAGAGTGAAGGCAAGGTATGGCATAAGACAGCAGCTTGTCCTGCCTGCTGTCCAGGTCAA
GCCACTTCCCTTCCCAATAGGTGGACCAAGCTCCTCTTGAGGGCATGCAGACCACGCTTCCTCCTTCCCTTGAGATTCTT
CACTGCATGATTTCCCGTCCCACTCCTTGGGGATACCCTTGGAACTGAAGACATTATACAGTTTAGCTCTCAGAGATTCA
ACTGGAGGAACAAAGCTTGCATTTTTTAAAAGAGCTACAAGTCATTGGGAAAAGGAAGTTACATTGTTATAAAAAGCTTGG
ACAAAACAGGTCTGGTTTAATCTTGAGAGTTAGCAATATTTTCTAGAACGTAATGTTCAAGCTGAGACAAACATGATCTG
ATGTGCAGCGCAGCGCACACTTAGTGAACACTGGAGATGAACCAAGACAGGTGGAGTTGGCAGTGTATAAAAACTGGCGT
TATTCCTACTTAATGTTTATATATGCAGATATTTAATGATGCAAGGCATTATTATGCATATAAATGCAGTAATACTTTA
TAAACATTTTACTAATCATTTAGAATTACACTCATATATATGTATTCATATTTATGTGTATACAAATATGAAATAGTTTT
GAGTCTTTTCTTTCTGATCATTAGACATCATTACTGTTATTTGCTTTGCATTTTGCTATTTGTATGAACAGCGTTGATTT
TATCTTTAGAAATGTTTGTTAATACTCTGGGCCACCAGGCTGGCAGAATTGAAGTTCATTGCCGGGGTGTATGCTCTCTC
TGCAGGAAGGACAAGTGCTAGAATGTTCCCTCATCAACAGTATTCGCCAAGCAGCCATACCAAAGGTAAATGCAATTGGT
GGTGTTCTTCAATTTTATTTTATTTATAATGTGTAAGTGCATGCGTGCATGAGTGTCTGTGTGTGTTGGGATGACGTGAG
TGTGCATGTGCTATCCACGTTCCTGTGGAATTGGTTCTGTCTGTCCAACTTGACATGGGTTCTGGGAATACAACTCAAGT
CATCATGCTTGTGTAGCAAACACTTTTACCCTCAGAGCCATATTGCTGGCCCTGCTGTTCTTTTAAACATAGATTTACAA
GAGTATATTTTTCATCTATAACTCTTAGGTGACTTGGGTTACATTATTTGGATTTCAGTTATATTAGCAATGTTTTAACC
CTTTGCTCTTCATGTTTAACTGATGACTGTGTGGGTTTACATGGCTGAGGCACCCATACTGCTTCATCTGTGCACTCAAA
ACACTAGTGCACACAAAAATCCTGGCTGAACTCTGAGAGGCAAGGCACCTGGAGCACAGAAATCAGGGATCGTCAGAGAT
TGGTGTGCTTGAAGAAGAAGCGGTCTACAGAGTGCTGTGCGGAATTTAAAAGGTTTCTCTTCATAGAATGTTCAACCACA
GAATTTATTTCCCTCACTGTAACTGCCTTGTGTGGTTTCAGATTTCCCAAACAAAGTTCTATTCCTGATTGTTTGTTTGT
TAATTTTTACTTGTCAGTTGGCCTCTTTCATGTTAGCTATGTTTCCTGTGTAACTACTTATTGGAATTTCCTCTGGAAAA
CAAGCGTGGTATTTTATGTGGGAATCAGGGTAAACTTTCACAGCTTTCCTTCCATGTGAGGATGCAAACATTATAGAAAT
GCAAGTGCTTGTTCTTTGTATCCAAGCTGCATGTGAGTCTTAACTTCATTTCCTCAGAGTAGCCTATGCTAGTGACTGTG
GTGGTGGTGGTCTCATTCCTGGTAAGGAAGCCCAAGCCTGTGTTCTGTTGTAACAGGCTCCGTGGGATATTCATGGACAC
AAGCAAACACCAGTACAGTCTTTCCTGAACATCACCAAAGCTGAACTTTTCTGAGAGTGACAATCAAAAAATTCAGACAA
GGAGGCGGGACATGTGGTTCAGCAATGAAGAGCACTGGCTGTTCTTCTAGTGGACCCCAGCCTGATAAAGTCCCAGAGCC
CTCATGATGACTTTCACCTATCTGAGCACTTGGCACAAACAATCATAGACATACGTGCCATCAAACAAATAATTGAGATT
TATTTTTAAAAGATGTATACAAAAGACTATTGAGCCAAGTTTTCTTGAAGAAGAAGAAGAAGGAGAAGGAAGAGGAGAA
GGGGGAGGAGGGAAGAGGAAGAAGAAGCAGGAGCAGCGGAAGCGGAGGAAGAAGGAGAAGAAGGAGGAGGANNNNNNNNN
NNNNNNNNNNGAGGAGGAAGAAGAAGAAGAAGAAGAAGAAGAAGAAGAAGAAGAAGAAGAAGAAGAAGAAGAAGAAGAA
GAAGAAGAAAGTATGATGCTCTTCTGATGCAGTTTTTGTGCACTGTAATGCTGGGGACTTCAGCCCTCCAGGTTTTATA
ATAGTGCTAGTTCCCCTGGGGGGTTTTAATGCAGCAGGGACCATTTCCCTTTAGGTATATTTTTTGCATGAAGTGTGGTT
TCTTTTCACCGCATTTTTTCTCCACACTCTGCAACCTACCATATTTTTTCCTATTTTTATACATCTACAGGATCCCAAAAT
CCTGGCTGCTCTCGAAGCTGTTGGAAAATCTGAAAATGATCTGGAAGGGCGGATATTGTGTGTCTGCAGCGGCACGGATC
TGGTGAATATCGGCTTCACTTACATGGTGGCTGAAAATCCAGAAGTAACTAAGGTAACATCTCAATGGCCTCCCTCCCCA
ATAAGTGTATGGTGTGTGTGTGTGCGCCGCGCGCGCACAGTTGTTTAATGTTTAATATAATTTTAAATATGAAGTTGAGA
ATTGTACACGCATTTTAATCGTTTAAAAGTTCACTGCTCTGCTGGAATATTTCTGTAGAAAATTCAGCATCCCAAATGCT
TTCATTACCCATCACACACAGAAGTCAGCCCTGCCTGGCATGATGTGTGTTTTCAGAAAACTAGCCCAGGCCTCCCATAACT
TGAATGTGCCCAGGAGTTCCTGTGATCAGTAGGTCTGAGTTAAGAGACAAGAGATTCCTTTTCAGTTAAGCTCAGAGAAG
CACACTCTGAGTATCAAATACCTTGGTCAGTCCTGCCCACCAGAAACAACGTAAACCATGAATATGGCTTAGGTGTAGCT
```

```
GTGTGCGAGAGAGCAAGCATGGTATGCATGAAGTTCTAGTATAAAAAAAAATGAAATAAAAACATAAAACGGTAATGAGT
GGCTCAGTAGTATATACTGTGACAAGCGTGTTCAATATCCTGGGTTCTACCCTCAGTGCCAGAGACCAACATGGCTATAA
CTATTTCTGTAAAATATGGTTATTTAACCCAATATATTCTAACCATTGTTGTTTTAGTATGCTATAAGCATAAAATTTAC
TAATGACATAGTTTACATTCACTTGTTCATGGTGTTTGACATCTAGTGTGTATTTTTACATCAAACATGTCAAATTAAAA
GTGCAAAGTAGCCGCTGTCTACCCTATTGAACAGTGTAAACTCGGTTGCTTAAAAATCTCAATCTCTTTTCCTCAGTAAG
CTGTTTGAAGCATTGAGTTAAGACCCATCTTATATCATGATGCCCATTACGTAGTAAGAAACCATTGCCCTAGCAAGGTA
GTCCCTAAAGAGATTTTGATGCAAAAATCTTCCCAACTTGACTTCCAAACATATGTAATCTATATGAAAGAGACCAAGTC
TTTTCTATCATAGAGACCTGGGTTTGGGAAAGGAGAGGTCTTTCATTTTCCATTAATCTCATAATTAGAAACACAGCATA
GATTTAATGAGAAAGGTAGAGATGCTGAGTTGTATAGGAAGGATAGTCACCAGCTAAGATCAGGTGGGGAAGCAAAGGAC
CATCACAGTCATGGACTCTGGGTCTAACAAGAGGCAGAATTGAGTGTTGCTCATTCTGGGACCACGGGGCTTACTCACCA
TCACTTGCATGGCATGGCCCTCATTCTAGAAGCACTGGCCTTACCAGGGATGTCCTTGCGCACGTGTCCCAGACCCACAG
AGGCACTGATGCAGAGCCTGCATTGGTAAAAAACAGCTTCCAAGTGACTAGTGTGTGTGTGTGTGTGTGTGTGTGTGTGT
GTGTGTGTGTGTGTGTCTCAGAGGCTCTTGCTTTGTGCCTTTGGAAGAAGGCATGGGCCCTCCAAGGACAGCCTTTGC
TCTGTCCTTGTGGAAGTGGCTTTGTAGGTTTTGTGCTTTTGTTCTGTGTCATCTCAGTTTGGGAGAACAGCTTTTACAGG
CCTCTCAAGTTTTTATTTCTCTGCTCAGACAGGAGCCTAATTTGAATGGATTATTGACCTTGATCTAGACAAATAGCTTT
GCATATTTGCTGCAAAGGTCTTTCTGCTTGCCGTTTGCCATAGCAACCTTGACTCTTGGGGATCCGTTCTGGCTAACTGT
GAAGAGAATGACTTGCTTGTCACATACACATGTTTGGCTACTTGAAATTTTGCAAAAAATATTGTAAACTTTGTTGTTTT
GTGACATCAATTCCTGACAGAAAAACAAACAAACAAACAAAAACAACAACAACAACAAAAACCACTGTGATTCCTTCTAA
CTCCAGAATCTGCACTCGGGCTCCAATTAGCTGAGCAGAAATCCGTACTTTTCCTTCTCAGCCAACTGGGAACATAGACA
CCATATTTACTCTTCTGTGTCTCACTTATTTTTTTCTTCTGTGAAATGAGACTTGCATAAAAGTACCTGCTGTACGATT
GTGAGGGTTGCTGGACTTGCTGCTTTGAAAACGCACGATGCATTTCATCCCCCTGTTAGCTATGATGATCCCAAAGTCAG
AATGTAAGCTGGAGGGGTGGGGTGGGAGAGATCAGGATGAGCCTCCTACCATCTTATCTCTCGGACATGACAAATAGACT
GTGGAATACCCAAAGACCACATGGGAACATAGACCAAAGCCCAGGCTTAACCTCACGCTGTCTGCCTTTGCTCCTCAATAC
TGGCTACATCGCTCTAAGACCTAAGCTAGTAAGGTCCTGGGACAATGTCTCAAATCAGCAAATCTCAGGTGCTTGTTTCC
CATAATTTGATTGCTGTCGATCTTGGTCCATTGTGTTTAAGAATCTCCTTTTGGGACATACAAAAATGAGGAAGTTCTAT
GCAGAGGAGGGATAAGGTGGGCAGTACATCCTCAAAGACAAAACTTAACTGTTTTTCTTTAAGTAGCAGACACGTTTTAA
AAAAAGTTTTATTTCCTAGAAAATAAAGACTCTAATTTTGTTCTGATTTTCAGTTCAAACTCACAGATATTTAATCCTAC
TGTTTTTAGGGGGAAAATATTTTAGCTTCTTGGAACTGTTACTTAGAAGAAACTGTTATTTTTTAATATTTCTCTACTG
GTATAAAATACAAATAATAAATCTCATTATCCAGACTGAAGAGTGTTAGTTAGTTAATGTCCTTCTGGTTAATGAGTGCT
TGCTGACAGCATTAAATACCATATTTTCCCCAACTATGAGTATTAATTTTTCCCTGAATTTTTCAGTGAATATGCATGTG
AATGGGTAATGATGCTCATATCTTGTTATCAATACTTAAAAGCTTATATTAGTCAACACCAGAACCTTTATCGACTTCAG
TATTGACAGTATCTCCTACATTCCCATTTTTATTCCTAATCTTGGATAGATGCATCCCTAACAGATTATACAAACCGAGA
AATGAGTGATTGGGTTAGGTAAAAAAGATGGAAGAGTCCATCAATTAACTCAGGTCAATGAGAAAAGCAGAGTAGCTTAA
GGCTATGCAGGAGAGAAGGCAGATAGCCCAAGTTTAAGCCTGGGCTTTGGTCTGTCTTCCTGACTGGTCTTTGGAGTTTC
ACAGTTTATCTGTCATGTCTGAGGTGGAGAGGTGGATGTGACTCAGTGATACCAGTCACATATGAGTGGAACCAAGGACC
CCTGAATTATGGGATCACAAGATATGCTAATGGCCCAATTAGCCCATATGGAAGATGTTCATGGTGAGTTCATTACAAAA
GCCAGTAACAGCAAAGAAGTGGTTTTTATATTAATCTGTAGAGATCTTGAGTGCTTTGGCTGAAAGAAACTACTTGTGCT
TTGCTTTCTGGCCGTTCTCTCAATGGCTGAGCTGCAGACATTACATGTCATCTGTTGGTGGGATGTCATGGAGCTTGTTA
TATATACCCCAGCCATTAAGGAACGGGCTGCTATTAAGCCCACATTTCATAGCTCTGTGACTTCCCTTAACCTATCGCTT
GTCTAGTGCTGTGCAGACTAGCACATGGACAGCATCTGGAGGAAACAAATAGCAGCCTACCTTTAAAAGGATTGCTCTAG
AATATCCAACTCTACTAGCAGCAGGCCTTTTTCTTTTCACTAGGCTTTCATTTGTTTATGTAATGTTTTATTATAACCGA
CATGCACAGGTTTGAAAATTAATAGAAGTCTTTTGTTGTAAGCCTCCAAATTTGGGGCAAAAGAATTTACTATCTATATC
AACATGTGGCCTGGAGATTGAGTTTAGTGTCTGGGTATCCAAGGGCTTTGAATGTCTTAGGAGCATTCCACATTTCTTTC
TCTGACCAAGGAGGGAAACAGGAAGAGAGTTTCCAGCACTGCATTTGATTATTGAAGTGCTTGAATCTCCTCCGAACTCC
TAGACTATTATGCTACAGAACAGTCTGATTGTTCTGAAAAACTCACTTCTCCAAACATTTAGGCGAAGCCAACTTTCTAT
TCATCCAGACTCCAGAAGGACCGAATTTAACTCAAGAGAAATTTTCTGACTCTGAGCGAGTCCTTCAAATTGTTTTCAAA
GAGTGTCTGCAATATGTGTGTGTGTCTGTGTGTGTGTGTGTGTACATGCCTCATTTTACATGGCTGTTTGGTGAGTCA
GTAAAGCCCAGATGATTCAACTTTCTCCAAGTTCAGGTTGCAAATCCTTATAAAGTGTTTTCTAGTCATTTGACTGTGAA
AATGTGGCTGTGTTTTTTCCAAAAATGCAAGTCTTATATAAGACATCCTGGAAGCTCAGCTTGTGCGGTCTCCAGCTGCT
CTCTGCCATGTAAACAGCCTAGCCTTGCCACTCGCCACTGATTTAGGCTTGTGCTNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNCCACCTCCTCCCCTCTCATGTCTTCATCTTGACCTTGCCC
CTTATAGTCCATTCTCAGAATAGCAGCTGAAGATAAGCCTCTTCAGATCAGGGGCTTTCATCACGTGGGATGCTCTAAGG
CCTCCAAGTCTCTATGAAATCCAGCCTCATCTGCTTCCCTGCCTGCCTTCACCTCCGTTTTCCTCAGCCATGTTTCTGTC
AGATCTCTGTGGATATGCTGGCCGTTTGCTCTTTCCCCTGTTATAAAGGAAGGTTTTACGTTTGTTGTTTGTTTGTTTGT
TTCCCTGTTTTTTTTTCCCTGGTTCTCTTTCCATCCCTACTGGACTATTCACTACCTACTGCAGCTCCTGTCCTCTCAACA
CACTTCAGCCCTTCCCCATTCGTTCTTCTTTCCAGAACAGTTTCCAGCCTTTTGAAAGTGCTCAATAAACGCATGCTTAC
TGAAGACAGCTATGCTGGCCAGGCTAATATATCCTAACCCAGCTGGTTAAGATGACAGTAGTCTTGCCCTAATTTTTGGG
AAATGGGTTCCAGTATCCCACAGTGAATTCCTAAAACCACAAATGGTACCAAGCTCCGCATGCACTTCTCCATTGTTTCT
TCTTCCTGTAAATACATCTGATTAGGTTTAATTTATGTTAAAGGAAGTCGCAATACATAGTGAATTGTGAACAATAGATT
```

```
GTGATAGAATTGCCAACATCAGTACAGTTATGCATGAGGACATTATTAAGTAAAATAGGGACTTATTTGAGCACAGCCCA
TCTGATAATGAGGTAGCGCTGAGTGCCCAACAAGAGGGCGGTACATCCCAGTTGTTTAGAGCAGGACAATATGGGATTTG
ATTCTGCTACCAGAGGGTGTGCAAGTTATAACTTATGAGTAGCTTGCTTCATCCATTTTCATTTAATATTTTCAGACATG
AGCCACTAATATCATTGACAGTGAAATAAGGGTAAGGGGGTGCCACTGTATCAGCTCGGTGTTGTACACTTCGATTTTAT
ACAGACTCGCTGAGGAGGAGCTAGGGAAAACGGTACTTGACAAGCAGGCTGAGAAAATGTGATTTGATGGCAGTCCAAGG
GGAACGGAATGATCTATAATCCCGGGGTGTTATTTTTAGAGTCATTTTTCAGGATGTAATGAGATTGCTGCCACTGAGAG
TCCATGACAGGACGGGAGGTTTCGATGCTGTTCTCTTGGCTTCCTCCCAATCAGGATCACCGTAGCCTGCTTACCTCAAT
ACTTCCTGTCCCTCCACTTAGAAAAATGTCGCTTTCCTTTCCTCTCCTGAAAAGCCCTCTGTTAAAAAAGAAATGCTAAC
AAGTTGCTGCCACCTTTATCCCAACCCCAGGGTGCTGCTGGGTAAAAGGTGTGTCTTCTCTGTCCTGTAGCTAGAAACAC
TTGAGAAGAAGCAACCTCATGCTGAAAGCTTTGGCCCTTTTTCCAAGAAACTGTAATTAAGCTTTTTATTCTTCCTCCCT
GATTCCTCCTCCCTGAACCATCTTCATCCCTCTGCTTATTATTTTATCATAGGCTATGCAAATTACTTAGTTAGCACGCT
TACATCTTATTTACTTAGAAAACCAACTCTGGTTTGTTTTTGTTTCTTTGTTTGATTCTCAGAAGGCAGCTAGCTCAGGA
GGGTTTCCATCTCCAGCAAATAGGAGTTCAGATGTCAGTTACAGCTGGTGCTGCACAGTGCAACCCCAGTCACTGTGCTG
TGCGCCAGGCTCAAGCACATCCCACATTCAATGCTTCCACACATAAATCTGCTATTTTATAGCAACTCGAATTTTCCTTT
GATTATAGAAATAAGCTGTTATTGTGAGGTTCAAAATGCAGAGATTGGAACTGAGTTCAGGCAATTTCCATCCCGCTCAT
TATACTTTCTTCAGGTTGTCAATATTGGGAGAACTTCGTAAATTATACTTTGTGACTAAGTTACCAAATAGGCACTAGAC
TAATTACTGCAGAAATTAAGTTATGAAAAGTAATGTGTTCTTTTTGGGGTTCAAAAATGATAATAGGGTGTGTGATTTA
TTGCAGGCAATACAGTATCATTTTAAGAAACTGAGTTGAATGTTAAAGGCTCCTTGCTTGCACTTGGATTATGTTGACTA
ACTACAGAGGTTCCTGGAGAAGGAAGGGATTTACTCTTTGTTTCACTGGGAAAACAAAAGCAGATCTCGCTTCCTTCTGT
TGGCTCTGCTAACATCCTCCATGACCTCAAAGACCTCAGTGGAGTTCCTGCGTGGATGGCCAAGCACTGAGGAACTTCAA
GGGATCAGACTGAGCTTCAGCCAGGGTCTTCTAGAGGTGCCAGTTCTCGGGCCCCTTCTTGCCTGATAGTGAGAAATCTA
GATAAAGAACCTAGGAAAGTTTCAACAGGTAGACCTAAACCCAAGTCACCAATTTTCAAGCCCACAAGTGTTAAGGTAAC
TAGAGCATACAAGTAGACAAATGCAGCTCCCTGCCCTCCCAGCCTGAACATGAGTTCACCAAGGCAGAGCAAAGAAAGGC
ACCTGGGGTAAACCAGGGTCTGTGACTCTAGAAAGCTCACACTGGCTTCACAGGACCCCATTTTAAAATCCTTTCATACA
GTAGTTTGTGTTAGTTTGGGTATGATATGCCCAGGGTCTTGTGTATTCTAGGCAAATGTTCTACCACTGAGCTAGACCCC
CAAACATTACAATGGTCTCAATTTTCAGAGTATAGGCTAGCGGCTCCCAGAGCCCTCGCACCTCCCACAGAGCTTCAAAG
TCATGTCCAGGCCCAACCTTGGGCCTTCTCAATCAGCAACCCATGTTCTGGTGATCCGATTTGCCCTCCACATGATTGTG
CTGCACCCTGGAAAGTGTGGCTATGGGAAAACGGTCCACCAGTAGCCTTAATATCCCACCGTCCTATGGAGATCTAGGAG
AAGGGGCATTCGCCTGCTGGGGTGAGCTGTGCTTGCCGCCTTAGGCAGCTGGCTGGTGCATCTGAACCTCTCTCCCCACC
CTCAATATTCATTAAGTCTCTTCGTTACCTTGCCTCACAGTGAGGCTCAAACATAACGAGCATAGAGGATGTGTGTGGAT
CTGTGTGCATGTGTGTGTAGGGTGTATGCATGTATCTGTGTGTGGGAGAAGGTGTGTGTTTATGCTATGCATCTGTGTGT
GTGTGTCTGTGTGTGTCTGTGTGTCTGTGTTTCAGGGGGAAGGATGTGAGAGGAGTCTCTCTCTCTCTCTCTCTCTCT
GTGTGTGTGTGTGTGTGTGTGTGTGTGTGTGTGTGTGTGTGTGTGTGTGCTCGAGAGTTTCATGCTTCCAAAACTAGCAG
TTGGCATTTTCGTGCCAAAGTGTTAGATCCCATGGGAAGACTGGAGTGAGCGTTTACGTTTCTCTGTTTGGCAAGGATCG
TAGCTAGCTTTGAGTGCCAAGCCTCATTGCATACTGAAGAGTCTATGGAGGTGCTGAAAGGTCATGACTTGTGCTCAGCA
AAGAATGCCTTGGATGCTTTACCTTCTCAGGAGATTATTGTATGGTAAGACCATGGGGGTCAAAGTATGGTCAGGGTACA
GCAGGAGCCAGTGTCAGAGTCACCAAGGCAAAAAAAAAAAAAAATCATTGTGCCAAGATGCTCAGGTGCTTCCTGTGGACA
CTAAGGTATGAGTGGTCCTACGGGGAGCATGAAAGTCTCAGTGGTTCTCTACTCATGTGAGCTGTACACAGGGAGAAAG
TTTTGTGAGGCATTGGAATGTTGAGTCCAGATTGGTCCCCGGTTGCCACTAGAGAAAAGAGGTTGCCCTGAATCAATAAA
TGTAATGCAATTTTCCCTGTACCCTCAGTGAGTATAGTTTTTACAAGGGAAGCAATCTTTTCATCACCTAATCAAATGAC
AGGCAAAATGAAATAAAATGGTCCCAGGATTAGCTGGCTTACTATAATACAGAGCTAGAGAAACAGAATAAGAAAACCAG
GCAAAAAGCATATATGAAAATATAGACAAATGTAAAGGAAAATTGTTTAGTGCAATGCACCACAAACAAACCAAACACTG
TCCACCGGAAGGAGCTGTCCAAATGGTGGGAGACCAGGCCCTCTGGGTAACATTTGGATGGATCCAGAGAGCTAGCTGCC
AGGTTGTCAGAGTAAAGGAAAGGACATTTTATTCATATGGAGTATCTGCATGAGAAGTTCAAGAGTGGCTTCACAATGAG
CTGTGTCTCATGACGTGGCAGTCTGAAGGCACCCAGGGTCCCTAAATTGCATTATCTCAGCTTGCACTCATCAGCTGTGA
GGCATGAGTCCCATACACCAACTCACTTAAATATCAGCATAGAGTTGCTACTGTTGGTTGTCAATCAGTCTGTGATAATA
TTGAGCTAGTCGCCTGGCTTAGGTATATCTTCTGTGATTAGAAATCTCAAACTGGAGTTACCACCGCATTCTGCATAGGA
AGTACAGAAGAATACAGTCAAACAAAACCAGCAGTCCCTTAAAATGAGTTTTCCAGTTCCTGTGTCATCTCGTAAAAATT
GCTATGAAATTTGCTTTCTATAAAACTGCTTCAAAATGAACAAATTGAGTGTTCTTTATGACTCTGTAGCGTTGTATAAC
CACCCCTACCGTATAGTTCTATAACACATCGCCATCCGGAAAGGAAACCCATCCTTCCACCACCATCCCTGCCTGCAACC
TGAGCTCATCCTGTTATCAGTTGGCCTCCTGCTGTTTATCAGCATAGTGTATCACATTCTATCACTAGGAGTTTCACCAC
ATTCATTTGCTGGTCTTTAAAACTCGGTCCTGGACTCGAATGTCATCAAGTTTCTGTGATCACTGAGGTAGGCTTCCTTG
GAGTTTTAGCTTCCTTTTCTTTGGAAAATGTCCAGCATCCGAAGCACTTCCCTTTGAGGATCTGTGGTGACAACGAGGGC
CATACTGTTCCTGGCTGGTGCCCTCCCACCATTGTGTTTCTGGAATGTACTGGTTGAGGAGCAGAATCTAAACCTGCCCA
GAGCACGTATTTTGCCCTGATTTTCATTCCCTCCAGCAGGAGAGTTAAAATAATCTCCACGGCTCCTGGAAGGAAAAATG
TCACTAGCCTTAAATTACTTCTTCATTTTCCATAGAGCTCCAGTTTTTACTGCTAGCAAAACCAGCTATTGGGAACATTG
TGTGTGTGTGTGTGTGTGTGTGTGTGTGTGTGTGTGTGGCGCGCGCGCGCCTGCCATGTATGTATGGGTGTTC
ATGCATGCATTTGCGCGCTACCAAGAACACATATGGTCCTGGAAGCTGAAACTATTATACCTTTCAGAAGTTGTGCTGAC
TCTTAGTAATAATACAAACTGAACATCCTTACTTTCAGAATTCCAAATCTGAAGTGCTCCCCAGTGTCTCGTAGTACAAGC
ATGAAAGAAAGATGGGACCTGGTGTCACAGGTAATAGTCACAGTGTAGGTGCAGGAAAAATATTGTGAAAAATTACACAA
CTTACAAAATTACAGGATATGGGAATAAAGTATATATAAAACACAAATGAACTTTATGTTTAATATACGGACTCATCCCT
```

```
AAGATACCTCATTCCATATGTACTGGCTAATTTTGTGTTAACTTGACAGAAGCTAGAGTCTTTTTAGTAGAAGGAAAGTC
AACTGAGAAAAGTCCCCCATCAGATTGGCCTGTGAGCAAGACTCTTGTACGTTTTCTTGATTGATGATTGCTGTAGGAAT
GCTGAACCCACTGGGTGGTACCTCCCCTGAGCAAGCTAAGGGAGCAAGCCAGGAAGCTGTGTTCCTCCGTGATTGACTTC
TGCATCAGTTCCTGCTTCCAGGTTCCTGGAACACTTGAATCCCTAATCTGACTTTCCTCAGTGTGATGGAGTGTTTGTCA
CCTGAATGATGTAAGATGAAATAAACCAAGCATTTTCGACGTGTATTAGACTCTGTTCAAAGTGTTGTGCTGTTGTGCGT
ATTATTTAATGTCACAACAGCTCAACTGGAAGACTATTAATGATGGTCTTCCTTATCTAGAGGAGATATGGGCTCGAGAG
ATGAACTTGCCAAGGTCATACCCTCAGTGACATTATACACCCGTCGTGCCAGGCTGGGCTCCAGATCTCGAGTTCTAACA
CTGCTGTGCTGCAGCTCACAGCTATGAATTTTTCTTACCAATATAAAGACAAAGTGTGACCAGCTCCTTTACCTGTAAC
ATAATACGGTTTTCACATAGCGTTTAAATGAATAACTTTAATGAGGTTGCTATTCTGTGGGATGTATGTGCTTCAAGAAA
GCTACCATCTCAACACTGTTGATCTGCGCCCAAGTCAAGCAAGAGACCGCACATTCGGAGCTACTCCATTCTCATTGAAG
CTCAGCAAGGAGAAGGGTGGGGATTTACTTATTTTCCACAGTGCTTCTTCCTTTTGGACGAATACCAAGTTAGTATTTCA
GAAATGCTGCTCTGCAGACAGAACAGTGACGTCATGGAGGCATCATTCGGCCCGATGTTACTGTAGCACTACCAAAACAT
TGATGAAGTCTTGGCATTCATCTATTTATTGACACTGTGAATATCCTATTGACTGTCAGGGTGCCCTCTAGTCTCTTCCA
CTGGATGAATTTATGGTAGGAAGCTTTCCTTAAAAGATAGCACCTCAGATAACAAGACTTTTCTCAGAAGTTTATGTCAC
TTCTAATCCCTCTTATGTTGATAGTGTTTATAGGCTTCTCTATAGATCTGACTTGTTTGAACTAAACAGGTCGATGCTGC
CTGGACAGAGTCAGTCAGCCAGAGGACCCCTGACTGAGTTTGGTAACATAGCATGCTTTGCAGGTCAAAATAATACTTAG
TTTGGTCTGAGATAATTTTCCAAACTGTGTTATTTGGTATATTATGCGGAAGAATGGGAGCTTTGGTACTATGGTTGCAG
GCTTCAATACATAGATGCGTACATACATACATACATGCATGCATACATACATACCTACATACATGTTGATGTTTGGCTTT
GAATTCACTAGATACTTCCTTATTCTTCATCATCAGTATGCTGTAGAATAAACAATTAAGAAAAATCTGACTCTAACCAA
ATTGGTGGTTTCTGACATCCAAAGATTGATATTCTTGGTTTTATTAAAGTTTTAAAATTCTTCGAATATTTTAGAGCATC
CCATATGAATGCCAGTTTGCTGAAATAAATGGGATTACTTGAATTTTTTCAAATGCCTTTTGGGCTTTGTAAGGATATAG
GTGTGCCATGGACTTTGAAGTGTATTAATAGATGTTATTTATTTATGTCAGGAAGTAGAGCGAGTATAAGGCCCAGGTTT
TCTTTCTTCTGTTCAATATGTAACAGTCTGTGGACAGAAATGGGGAAAGTGGAGTAAGATTCTATAGATTCAAAAGGACC
TGTTACAAGATATCTTTGAAATCAGGCTTAGGCAAAGCAAAAACAAAACAAAACAAAACACCCTGAGTAATACCACTGG
ACAGATGGCATCTGAAGTGACACTTGCCTGAGTTATAACCCAGAACAGCACTTTGTCCTCTCCTCTCTAGGGACTGTTTA
GAGGTCCTTTTTGACCTAACCATCTAGCGTTGTCAACATAACCAGCAAGACTGACAGCCTGTTTTGACTGTCTGGATGGC
GCTAACTGTGGCCAACAGTATCTAAACCCAACCCAAGCCTTACCAGCTCTGTCAAACACAGCCACCGTGTGCCTAGCCGG
CAGCCTGCATCCTACCAGGTACCATTCTCCATGCTCCGAACTAGTTCTAGGAGTCCATTCCGTCTCTAACTCTCTCCAGC
TTCTCCCTGGAAAGAGAACAGAATGAAGAACATGTGTGGCTTGGAGACTTTCAGGCCATGTCAAGGAAAAGGGCCTGGTC
ACTTTTCCATTCCCCCATCTCTGCTTCCAAACTTGGACACATTGTGAGAATGTCAGCCGTTTCCCAAAGGCCTCTGTGAT
TCTCACTGGGTTGTGTTTCTTCTCTTGAGTTGCAGGCAGATCCCTTATAGCTTCCTGGTCACTGGTCTCTGTAGTTTACT
ATTTGTGAATTCCACTGTCTCAGGCTTTTTTGCTGTCCTTTCTCAACCCCATCATAGCCCGACCCCGTTTTCTTACTCTC
CTTTACACATGCATTGTTGGTGGTGACTTTCACATTTCCTTGCCTGGTTTGTTCTCACCTTTGGCACTATCGTCATCTGC
CTGTTTGCATGCGGGGGTGCCAACCCCTGGGAGATATGCTGAGTGCCAGAATTTCTTCATGTAGGATCAAGAGAAAGTA
GTTGACATGACTTATTTTAATTTTATCATTATTTATTTATTATTTAAATTGTTATTATTATTATATTGCTTGATAATTTC
ATACATGAATATAAAGTATTTAGATCATAATACAGTCTGTTATTTGGTATATAAACTTTGGTAGCCAGCTCCTATTTTCT
CTTAACCTCCACTCTCTACAGTTTCTTCGTTCTTGCACCTTACTTAGTCCTGTGCTGAGGGCTATGGTGACAGGAGAATC
ATTGGTCTGGAGTCAGTGACCCAAGTTATAGTCTTGTTATTACTACTGTCCTGAGAAGGGTACTTCTGTCTGCTTTCTGT
ATCCAGTATTCTAGTAGGCATTCCATAGTAAGCCTTGATTTTGTTGTCTTGATTTTGTGCAAAAGCCTCCTTCGAGCAGG
AGATGCTGTTGTTGTGACAGGCTTCTCTAGGCCTCCTGCTGCTGCCTAGATCCTGGAAACCCACATCTCCATGGGTTCCC
TGTTTGTGTTGTGTCTTCTCTGTCAAGCTGCCAGTGACTGCTTGCACAGCCTTAGTAATAACTGAAACTCCATAACCTGT
TTCAGCAATGTAGTGGGACATATGGGTACTTGAGTGGTACTTAAAGAACCAAGGAATGAAGATTTAAAATATAGAAATGT
CCCAATGTGCAGAGCTCACTATGAGAGTGGTTATTCCTCATTTGGAAGACTTGATTTAAAAACCTGTTATTTATTATATA
TAAATTATGCATAAGGAGAATAAATTACAATGAAAATAAAACAGGGCCCTAGTTATCAATGGAGAAGACACCGGTTTCCT
GATGCAGGTTCATTCTGCAGTGATCACATCTCCTTCAATTGTCTTGTTCTCTCACTGGTGAGTGATTCTTGCATTAAAGT
TACCTTCAGGGTTAAAATGCTGAATAATGGGGACTGACTCATAGAATTACTGATTCTTTTGGTTCAGGATAGGGCATTTC
TGAAAAGGACCTAGTAAATTCTTAAATTACTAGCTGGGGAAATCTGCTTCAAAATTACTTGTATATTCACTAGTCTTCCC
ATTCATGACTGTGAAACACATGAGGAAACTTAATTGTCTTGAGATTTTAGAAGGTTCAGTCCATTGTTTTTATGGGCCCT
ATGATGTCATAGAATCTAGAGTGTCATAGAATGTAGAATTTGCAAGCTGAGCTTCTGAGCATGCCCATGGATAGTTATCT
TGATAACATTAATTAAAATCAGGGTTCCATCTTAATTGTGGGTAGGAGCATTCCATAGGCAGGGGATCCATGGCTGAATA
AAATAGAGAAATGAACTCACCCTTAGCAACATTCATTCCCCTCAGCCTCTTTTTTTGGGGGGTGGGGTGGGTGGGGTTG
GTTTGTTTTGTTGTTGTTGTTGTTGTTGTTGTTGTTGTTGTGTTTTGTTTGTTTGTTTTTTGTTTTTCAAGATAGGGTT
TCTCTGTATAGCCCTGGCTGTCCTGGAACTCACTTTGTAGACCAGGCTGGCCTCGAACTCAGAAATCCGCCTGCCTCTGC
CTCCCGAGTGCTGGGATTAAAGGCGTGCACCACCATGCCCGGCTTCCCCTCAGCCTCTTATCGGGATGACCGGGCCAGCT
TCTTCAATCTCATCCTACCTTGACCTCCGCACCAGGATGGAAGACAGTTTTGAGTCAGAATACACTCTTTCTCTCTTACG
TTGCTCTTGTCAAGGCATTTTACGGCAGCAATAGAGAAGTAACTAAGATGCTTGGGTAGAACACCATGGCAGGAGCACAT
GACAGAGGAGGTTCTTCATCTCATGGTTGCAAGGAGCAGGAGAAAGATGAACAGAGAGAAGCCCAAACTAAGATGTAGCT
CCCTGACTGCTTGGCTCCTACTCTTACAAGTTTCAGGATCTCTCAAATAGCATCACGGGATCAGAGAGTCAGCATCTCTG
AGCCAGAATGTGGGTACTCACCAAACACCTCACTTGCTTGTATTTTGGGAGATGCACTCTTTTAGGTGCATCACACCTTT
CAAATGAATCTGCACAGGCTCACACGGCAGATGTAAAGTCTAATCCAGGGGAAGACACCTAAGACTCTATTTCTAACAAG
TTTCCCTGGAAATATTGCTGCCCCTTGGCCAGGAATCATATGTCTGTGTGTGTGTGTGTGTGTGTGTGTGTGTGTGTGTG
```

```
TGTGTGAGAAAGAGAGAGAGAGAGAGAGAGAGAGAGAGAGAGAGAGAGAGAGAGAGAGAGGGGAGGGAGAAGAGAGAGAGAGA
GAGAGATTGCTGAGGTTATAGTTCAGTGGTAAAGCCCTCACCTCCCATTTATGAAACCATAGGCTACACACCCTCTCCCA
AAAAATTAAAAAGAAACAGATTTCTGTTCTCCATCCTCTGAGTCTCCATACCTGCAAACTGAGTCAGGAGGTTGGACATG
ATCACTTAGATTTAAATAATTTCCAGGTTATCTTGCTGATATTCCAGAAAGAAGGGATGGAAGGAGAGGGAAGGAGCAAG
AAGGAGGAAAGGAGAGAGGGAGGAAAAGGAGGGGTGGGGGCTTTTAAGGTCTAACCCAGGCTAGCACTCCTGGCATACTT
TACCTAGCAGGTCTGTGCTACTGTTGGGTGTATTTCCTTGGGAAGACTTCCACAGTGCTTGAAGCAGGGACAGAAGAATG
AAGAGAAATTCTGTGAGTGGTCTCCTCAGATGAATAAGGCGATTGGGCTAATGTCGTAGAAAGAGCTGGACTGGGATCT
AGGACATGGAAAAGGAAAGAGGAAGCTAAACCACAGCCCTGGCTGAGCTGGAGCTCTGTGGATCCCTCTGACCTCGAGCT
TGTAGTGATAAAGCAGTGGTTCTCAGCCTATGTGTCATGACCCTGGGGGGGGGGGTCAAACAACCCTTTCACAGGGTTGC
CTACGTAAGACCATCAGAGAACACAGACATTTACATTACAATTGGTAAGAGTATGAAGTAGCAACGGAATAGTTTTATGG
TTGGGGGTCATCACAGCATGAGGACCTACAGCATCAGGAGGGTTGAGAAGCATTGTTCTAAAGGAAAATTGTTGCTATAG
CAACTGAGTTTCAGTTCATGTGGGTTTAGTCTAGTTTTGTTATAATTGTGTGTTTCTGCATTATCTTAATGAGGAGGGGT
TCCTTTTGTTTGTGACAGTGTTTTCTAGTGTTCTACAGCAAGGAGACACATAATGAGGAAGGCCCAGACATTTGCCAGGA
GCAGGATGCTTGATAACTCGGTGTCTGCATGAAGAAAATCAGGTCAAGAAGTGCTAGAAGCCAGACCCAGTGTCCCCAGG
CACCTCTTAGCTTTCCACATGCCCAGACAATCCTACACAAGACTCCTCAAGGATTTTATTTTCCTGAGAAGGGATACTGG
AAAAGGTCAAATCAATCTATAAAGTATAGAAAGGATTATAGAGTAAGAGAACAAAGTGGTTATCAGTGCTAATATGTGTA
GTTAGTATGGTCAAGGCTTTGTCTTCAATATTCAGCACAAATAAAGTAAATAAAACTAAAATATAAAAGAAGGGTGAGAC
TTTACGGAAGCCACACCTCTCTGTATTCAGGTGCTGTTTTTCCTGTCATGGTGAAGTTACCTGGTACCCCTGCTGCGTGT
GCCCACCGCAAGCAACTCTGAGACCCCACCAAGGCATGTCTGTTATCCAATAGCTCAAAGATACACCTCTAAGGGTGACA
TGCTATTAAAGTATTCAAGCTTACCTACAGCAATTCAAACAGGTGTATTTTTAAGTCACCACTTTAAAACCTGTTAATCT
GGAAATAAGCAAAGGCCTGTAGCTCATACCTGGAGAAACTTTCCAGAGAAATTCTGGGACTCTACTGACACCTGCTGGCA
TCTCATCTCCTTAATGTTTGAAAAGAGCAACAAAACACGTCAGGCATTAATATTTCCTGTAGTTACTGGCACGCATACTA
TGTGTTTGGCCTGAGTCATGGCATGGACATGGAGGAGGTCTAAAGGACCTTGCCTTCCCTGACAGAGAGACAGGGGAGCAA
GCGATTGGGTGAAAACATCCCAACATGGGGACCTGACCCAAGACGGGCTCTGTGACTCTGGGCAAGGCCCTCAGACCTGC
TTTCATCTTCATAAAAAGAACACGTCACAGCCTCCCACTCAGGGCTAAGCTATCCCTGAGGATGGTGGTGACATTTGCGA
TGCGTGAGTTCTGTCATGAGTGGTGATATCAAAGGTCATCACCTTTCCATTCTGATAAATAAGAGTCTTCCCCAATTCTA
TTAAAAAAGAAGAAGAGGAGTAGGAGGGGGAGGAGAAGAAGAAGAAGAAGGAGGAGGAGGAGGAGGAGGAGGAGGAGGCG
AAGAAGAAGAAGAAGAAGAAGAAGAAGAAGAAGAAGAAGAAGAAGAAGAAGAAGAAGAAGAAGAAGAAGAAGAAGAAGAT
GATGATGATGAAGGCAGTTTAAGCCTCCTCAATACCTCCCCCTCCCCCAGTATCTGGTGGTTATTTCTAGAAACAACTAA
ATTAGTGTGATCAAAAAACGTTGTTTCTCTGTACCGGCCACTTCTGTGCCCTAGGAAACTGTGGACTTTGTTCTATGTTT
TGGAACAGATCTCAAGCCTTCAGGTTGAGAAAAATACTCCCAGTTAATGCCATCCAAATTATTTGTGCTTTTTCTGTCTG
CTCCTGGGAAACTGTGTGGGCCTGTGGTTTGTTAGGCCAGTTACAGGATATTCAATAATCTCCGGGGAGCCAAGTCCATA
TTTAGCCCGACAGAGGAAGAGAGGTCTGGTAATGAGAAAAGAAGCAATTCTTTAAAGCTCCTGTTTCGTTTCTTGATTCT
CTCTCTGTGTCCAGACCTTACCTGTTTGTTGAAGCTTTTGATGCTATTTGCAGTTCTCAGAAAACGGCCTTTGCTAGCTG
CAAAAGGAAAGGTGCCTCCTGAGAGTTCTTCCGTCTTCCATCAGAAAGCTGTCAGCAACGTCTGGGACTCTCCAGGGCAA
TAAAAGCCATTAGCTTCACACCCAACAGAACGGCACTAGTGAGGATGTAGAAGTTAGCACCCTCATGCACTGTTGGGAGA
AATGTAAGATGATGTAGCTATCACAGAAAGCAGTATGGCATTTTTCTTTAAAAAGTTGAAAGGCTCCTCGTGTCGTCCTGT
GACCCTTGCCTGTGGGCATTTATGCCAAGTAATTCATATCAGTTATTTACAGTATTCAAGACAAAGAAGCTCAGGTATCC
ATGGAGAGAAGAACGGATGGATGACCGTGGCATGCACATACAATTGAATTCTGCCTTGTAAAAGCAGAATTTGGCACATT
AAATGAAAGCATAATGCCGAGAGAAATAGCCAGTCAGAACATGGAAAATCCTGTGTGATTATTCTTGTGCGAGATACTTA
ACATCGTTTAAAACATAGACCCAGGAAACTGACTAGCGAGGGTCAGCGGGAGAGGCACAGATGGGGATGATGAGTGCAAG
GTATTGAGGATTAGCTAAAGCTCCTGAGACAGAACCATTTATGTCCTGTGTGTCTTTACCGAAATTAACAGGAGAAGACA
TCAAATAACAAGACCCAAACCACACTGCTATAATGCAGTTATCAGCTCAGGGCTTTCAGACATGAAATCCCTCATCTGGT
AACGTGAAGCCTGGTGTTTTGGGGCAGTGCTGTGCACCAGGAGCTTCTGTGATGACAACAGTGCTTCTCCTGGGCACTCT
CAGGACAGGGCCTCGAGTCCTCGTCATGGCTTTTTGTGGGGCACTTGAATGTCAACTGAGACATCCAATTTTAGACTTAT
TCAATTTGAATTTAAGGAATCCTATGATCAACGACAGTGGACATTCACATTTATGTGACTACCCATTCCAAAATGTCTTCC
TCCCCTTGGCAGTAGCTGCAAACTTAAGGAGGTAGAGTGGGCCGGGGAGGGAGCCTTAGCTCTGTTTATTCCCAAGCTCCA
GTTTGCTGCACAGCATGTCGCCAATGTGTCATTCCTTTGTGGCATTCATTAGCATCAGACATTGGATCCAAGAGCAGTGT
TGGCTTTGGTTCATGGGCTAGCCTTTTCTAGAAAAATGCCCTTTTTACAGACCCAAAGTTCTTTCCTGTCATGCGTAATCT
GTCCTCACTTTTCCTTGTAAACCCGTGGAGTGGTGGTATGAACTGCAGCTGAGGTTCAGGTCCTTATACAGAAGTTCTGT
GAACACATCCCTGCTTGCTCTGGGTACCGTGGCAGCAGGCAACGAATCTTTGACTTTTTAAGGCTTTGCTGGAAAAGACA
AACAGGTAAACTATGTAAGCAGGCCAGATGGAGCAGCATCCTGACACTGCAGTGAAGGGCCTTGTCACATTTGTCCTCTA
AACTGGAATAGGTAAACGTATCGGCATGTACTTGTACAGCATGACTTAGAGAAGGCTGAACAACTAAAATAATAAGGCTT
ATATTGAAACAGGCTGCAAGTGGGAAGCTTAGGAAGCCAGCCAGTCTTTCATGGCATTGTCATGCCACATTTAACTTAAT
GGGAAAAATGAAACCTGCAGAATAAAATACAACCGTGTAAAATATAGATGCCTGTGCAGCAAGGGGGGATAAAAGAAGAG
TCTGTAGTTTTATTCCACAGAGGAGACAGGCTGTATTATAGTAATAATAATAATAATAATAATAATAATAATAATAATAA
TGAAAAAGCCTGTACCTGGCTACAGAGGACTGCTCACTGTGGACTTTTTCTTTCTTTTTCTTCCTTTTCCAGCAATGGGT
AGAAGGCCTGAGATCGATCATTCACAACTTCAGGGCAAACAACGTCAGTCCGATGACATGCCTCAAGAAACAGTGAGTTC
TGTTTGTCTTGCCTTTTCTTCAAAGGAGTTATTTAGTAAGCTTTCTGGCTAGGAGGGAAGAACTGTATCACATCCATCAG
GCCAGTGTCCACCTTTCCTTCTGTACCCCTGGCTTTGGAAGTTGACCAACTCCCACCTGTATTTAAACCACTTAAATACA
TAGCAAGGCATATGTGATATTCTTGTCATGAGATCTGTAGCAGACAAAAGGTTGGTAGCACAGGTATCAAGCATTCTTTT
```

```
TGTTGTTGTTGTTGTTTTCTTTTCTTTTATTGTCTCCTAAACTCCATTTTCTGATGGATAGCTTGCATGCCTCACCTGTA
GGATAAGTTCTAGGGCACCTCAGGTCTCCACTGCATGAAACTGACAATTTAGGGATATAGCCAAGAGAAGCATTTGGAAA
AGCATCCATTGCATAAGTAAAACAGAAAGTCCTGCCCCCCCCCCCCCAAGAATACTGCTTCCTTGAATGTGCGGTCAGGG
TTCTTTGACATTTCTTTCGAAATTTCAGTCAATGGTTCAGTCAACTCCTGAAAATCTTACTGCAGGTCACTGTTCCTGGA
GTTCCAAGTGGCTTACTACACTGATAGATTGTCATCATGCTCAGATACATTACTGGGTCAAATAATTAGTTTTCATGGAG
AAATCACAGAAAAGCAGTGGTCTGTAATTGTGCTTGGCTGCAAAATTGTCTGTGGCTTCGTAGTGACAACAAATGGCACA
TAGCTATGAGGTACCAAGATAAGAATATGATGAAGATGCAAATAACCTTGTGGAGATAAACTTCCACGTCTGAAAATGAG
GCCCTAGCACCAAGAGAGCGATCCAGTGATTCTTCACCCTGCTCAGTGGGCCAACAGGCCAGTCCTTAGGCACTTCCTGA
GTAGTCAGCAGGTACTTAACTATATGAACTCTAGGTTGTAAACCCCAGCTTTGCCACATTGGGCAAATCTTTAGCCACTT
AGTCCCCATTTGTGAACCAGATTACCATATGGATGAAATCAGAAAGCACCTCACCCAGTGTCTGGCATCCTCGGTGGAAG
GAAGTTATGAAGGCTTGTAGATTCCTATTTGCTGTGCAGCAACACTTCCTTCCTCAGAGTCATTGCGTTCTCTTTCTCAG
AGATAACCTTGTGAATGTTGCACACTGTGACTTTGGGTGCTGGTAGCTTCAAGGGATCCTACTTATACTTGAATGGCCAA
TATGCAGACACCAGATCTTTGGGCAATGGAGCCACTGTTTACCCACCCACAGGCTGCTTAGCAGGGGTGCAGACCTGCTT
ACTTGTCCAGAGGATGCGTCTCTACTAAATCGAAGCCTCCATGCCTGGGAGATTATACTCCTGTTGCCATGACAAATTCC
AATAGGGTAGCAGAGCCAGCCCATGTGCTACCAAGGGTGGAATATATTGTATGAGGGCATGTAAAGACCACTTGAAACAC .
AAGTTTGCTTAAGCCAGCTCCCTGCCCATTCTGCCTCCTTCGCTCCTTTTGTCCCATTGTGCTTCTGGAACTTAAGATAG
AAACTTTCCCAAAGCCATGATAATTTGTAAGTTGTTAGACTGTTAGTGCCTAGAAGTAGTAGGAATAAGACTCATTAGCT
GAGAACAATCTTGATTCCAGTGTGTTCTGGAGTGTTCTGAGGCATTGAAGCTCCTCACGTCCTGAGACCCTGGGGAAGGC
AGAGTTACCCAACATTCTGAGACCCTCACTGGGGATTGAAAATGAAGGCGGGCTTCCACTCATTCTCCATCCAAACCTTG
GAAGCCGGGAGTAGTGAATCCCCTTTGGCCTTAGCCTAAGCTGCATTTCTTGCCACCTGAGTGTTTTCAGAAGAGCAAAA
CCAACACAACCATGGACAGGTGCCATGTTTTGCTCAGTGCAAATATCCAAGTTCTAATTCCCCTAATGGTATCAGATTTT
AAAACATAGGCCTCTCGGTTAAATTTGAATTTCAGCTGTAGCTATTTTACTGTAAATATGTAGTAAATGTTACCAGGACA
CATTAAAATAGTAGGTTACTCGTCTTAAAATCAAATGTTACTGGGCATATTAAAATCTGAGGGCCCTAAATTCATCCCGT
AATGATGGGGCTGTTAGAATAGGTGTTCTGTCTGAAGAAGCTTTTCTCTGGGGTTCTCGGTGATCTGGAGCAGGTGCTGG
TGGATCTGCTGGTGGGTTCTGAGGCTGTGTTGCTCTTGTTTTCCAGCTGGATGAAACTGGCCTTTCTGACCAACACAACT
GGTAAAATCCCAGTGAGGAGGTAAGTGAGACACGTGATTCTCTGTGTCACCTTCACCGCATCAAGCCAAACAGAGCCTTC
CAGACCAAACCAAATGCCCAGGCTGCCACACTGAACCAAAGATCCTCCCTGCCACACCAAGTCAAAGGCCCACCCGGCTA
TGACATAATCACATATGATCCTTGCCTTCAAGATATTTCCAGAAGTCTAGACTCTGCTATTTCGTAAAAATCACCCATGG
AAGTAAAGTTGTACCTCCCTCAGAATAGGATGCATTGCTGTGGTAAAGAATGTCTGAAAAAAATGTCTGAAAATTGTAGC
ACTAGATTTTCATCAGTGAATCACCTGGCTTTCATGATTTATATTGTGTCTTCCCCCCTCCAGCCCCTTTACCTGAGCAA
CAACTACCTCCCTCCTTTCGAGCTGGACCCCAGTACACTGATCTTCTGGCAGGTCTATGCCTCTGGCCAGCACTTCCCCA
TTCAAGGTTGCTGCTTATGCAGTCCCCCGAGAAGGTGGAGCTTTCCCTAAATTCAGCTGCTGCTGCAATATGCTGCATAG
TGTTTGGCTCCTTCTCACACTCACCAAGAGAAGCTGGTTGTGGGTGCTTACATTGACTGAACATGAGAGAAGTTCATTTC
TCACAGTTGGGGAAGCTGAGAATGCATGAACAGAGTGCCGTCACGGGCCTTACAGACTGCAGACTGCTTTCTTCCAGTTG
TGCCCTCATGAGAGGTTAGAGGGCTAGAGAACCGTCTCAAGGACATTTTTATAATCCTATTCCCAAGAGCTCCCCACCCC
TCACCATAAAACCACCTCCCAGAGGCCCCTAATACCAGCACGGTGAAGTTCAGTTAGATTCCATATCAGAGGGAATTTCAT
CCCTCACCTCCTCAGCGTTAACAATGACAGAGAACAAAAGGCATATGGAACCCAAAGGGAAGAAGGCATAAGCCAGACGGA
TTCTTCCCATGTGCTCTTTAATAGTCCTGACATGGTTCACCCTGGGAGGAGTCCTCACACATCTTCTCTGCTTCTCACTT
CCTTTCACAGTTTAGGGTTTGCTACTGGTCTGTGTGTAGCTGGAAGGACTTGTGAGGGTAGCTATGGGAATTCTTGACGA
AACTCTCCAGAATCATATTGTTTGATGTTTGTGTATGCATTCACAGACATCAAAGAAGCTTGGCTCAGACTCAGGACATC
ATCTGTCCACATGAAGCCAGACGTTATGTGCCCTTAACCTGAAGCAAATGCAGACTCCCTGTATACCAAGCCCCTTTGCC
CAGGTTTCTCAACTCAATTCCATGTTGATGTTGTCTCTGGAGATGAACTTCAGTAAAGACACAGACTTGCAAAACACCAA
AATATAGAAGAGAGCTGTCCTAGACTGACCTACCTTTAGCGCACAAGACAAACAAGTCACAAGTGCAGATCATTTGAATG
CATGGATGCCATTTAGCCGAGGCATTCAGGTTACTCCTGGCCCTTTCCAGGTTTCTTATCCTACTTCTCTGCTCACATAC
TCCATTCTACACCAAGCTACTAATCTGTGCCCCTGTGCAAATGTCCCCATACCCCCACTCACATTGTCACACAGGACAGA
ATCATCCTAGACACCATCATTTTGGTCCATTCCATAGATCATGGGGATCCTGAACCTAAGTACCCAGGTGACATGTCTAG
GATTCTTATTAATGAGAAACTGGACTACACTGATGGCATGAGGATGACCCTGTGCAGTGTCCCTTTTTATTCAACCTTTC
ATGATTATCGACCAGGTTAAAGATCGCAGGCAGTTGTTGTGACTTTTAAAAACTCCGCATGTAGGGTTCTTTGCTGCCTC
GTTACTGCTCAAGTGAGCGGAGGTTTTCAATAATATTTTTTTCTAACAAGTCAGGTTTAAGTATAGTATAGAAGTTTATA
AAAAAACAAAAACACCCAAAACCCTGTGTGTGTGTCCTGACTGGTAGTTTTTATGAAAGACATTTCAGAAGGATCCTTAACT
AATTGAAAACCTCCCTTCACTTGAGCAGGAGCAAGGCAATTATTTCAATGTATCCAAACCATCCTTGTCATAGAGACAGT
GTGTGCCCAGACTCAGAGTACATTTGTTGGTGTGGGGAACAGAGGGTACATACGGCAGTGCAGAGGGGGAACTCTGTGCA
TTATGCTTCATTTTTTTTTCCATAAGATTTGGAAAGTGGACCGAGTGGGATACAAGATTATGATCCAAGTGCTTCTAGCC
AGTAGTTCTGGTTTCAGTAGCCTGCCTTCCACCCACCGTGCTATCATCCCTCAGGGTTGGAGGCCTGTTGAGGTTCTGTG
GGTCCCCATATAGCATGTTCACATACAAAATCATTACAACTGTGCTTGAAATGCTCTAGATATTTTGCCATTGACATGGG
TTTTGTGTGTGCGTGCATGCCATTATTAATAGGTCCTTAACACAAGACATCTGTGACATGGGGCAGCTTGTTAGAATTGC
TGTAGTATTGATGCCAAGCCAACAAAAGGGGAATAACTCTCCCGGTAGATAGTCTGCCAAGAGTTAGGTAGAAATTGTTC
TGAATGTTTTAATGGATGTGAAGGGTAAACACTTTTAAAAATGTCATTTTTTCACCAACATTTTAGGGGTTTAAAATGGA
AACTATTAATATAGTGCACATTGTGTTAAAAGGGGATCCAGCTTCATAGTCATTGCGTAACTTGTGAAAGGCTTCTGCTC
TTAAACGTTAAACCTAAATATAATACCCAGGAAATTGCAGGAATGATTGATGAAGGCAGCCTCACTCCTTCCCTCCCTCT
TCTCTCTTTCTTTATCTATATGATCTTACACATTCCGCCTTTCCCTCAAATTCACCAGCTTGCTGCAGTTCATCCTCCCA
```

```
CTTAGACCATTCTCTGAAAGTAACTTGTCTGTCTCACATTCCCTGTAACATGGAGTACTACCTAGAGCTAGAGAGTTGGG
TGATGGAGATCTGAATGGCTTTGAGCAGTAACTGGAGAGGCTACTAAAGTGCAGACCAAGAGCCTCTGTATAATAATACT
GGCAACCTGGTCTGCATGACCCATCTATCTATTCAGGTCCTGAAGCTGCAGGACCCTACAAAGGGTGGTACCCTGGTCCT
TCACTTTAGGAGTCTCCTGTGACTGTGTCAGCAGTCGGTGTTCAAAACTCTCAGAAACATTGAAGAACAGTTATGGGCAA
AAAGATGAACTCCCTTGCATTCTAGACTCATATTCTCTAGTCGTTTGCAGGGTAGCGTGGGATACAGTATTTAGCCAGCT
CTGTGATAAGACTGAGTACAACTTCAGTGTGCTTGCTTTTGTACATTTCTTTAAGGGAAGGAGTGACAGTTTGTTTTGAA
TGAATAAAATCTCATTGAGAAACTTGGAGTGTAGCTTAGTTGGTAGAGAGATTGCCTAGTGTATCCAAGTCCTTGGTTTC
CATAAATCCAGGTTTGATTGTACACACTCAAAACCCCAGAACAGCAAGGTTGTAGGGTTCTAGGCTGTAGACCCTGTCTC
AAAAAAACAAAAACAAAAGTGAAACCTCACTTAGCACCTTGTACCTTAAAGCATTAAGCAGATGCCTTTGGCCACAGAATT
TTATATATATATATATATATATATATATATATATATATATATATATATAATTATATTATTATATTATTATATAGGGCTTG
TTATATATATTATTATATAGGGCTTATTATATATGGTTCTTAACCTTTGTGTTGCAACCCTTTAGCAAACCTCCATCTCT
AAAAAAACTTTATATTATGATTTATAACTGTAGCAAAATTCCAGTTATGAAGTAGCATCAAAAATGACTTTATGGTGGGGG
TTCACCACAGCATGAGGAACTGTATTAAAGGGTCACATCATTAGGAAAGTTGAAAACCACTTATATAGAGGTTATAATAA
TGTCACAGTCCCTTTATCAGCACACTGCTCTCGGAATGCTGGTTAAAGTGCACCGTTGTGGAAACCAAAGAATGCATATT
CCCTCTCTAGCCTCCATATCACAGTAGATGAATTTTTCAGACTCAGTAATGATTTGGAAAGGTGTCTGGAGCAAAGGAAA
CAGACATTACTCCAGGGAAATAGTCTGATAAATGCCCTTGTGGCTATTTTCCATTCTGAGGTTCTTCTCTTTTTACGTAC
AATGAGCAAATGTGCCTTGAGGCTGTGCTGCCGAACCCTACTCATGTCTGAGAAGTCAGCACCTCTCTCCCCCATCTCCT
TGCTCCCTGATCTTCTTGTTCTTCATCCTGTTTCTTGGCACACAGCCCAGCGCTCACCACACGCCTTCCTTCCTTGCCTC
ACTTCCCATGGTGCCTTGGTTCTGATATAGGAGGATGGCTCAGGCTCCATACCTGCTGCCTCGTTCGTGGCCATGGTGAT
TAGTTTTCATGTTAGAACCAGGCCTGTCTAACCCATCAGCCTTGAGTCATTGCTGTGGAGAGTTTTTCCTCCTGATGACA
GGTTCTTAACTTTGCTGTGGCACCACATGTATTTCCTCATTGCATCATTTTGTCAAGAAAGACACACTGCCATTTGTCCT
TACATTGAAATTGCTAAATGGCATCTACCCCAAGTCAGTGTTTAAAAGTTGAACTGGACTCTAAAGACTTGAGAAAATAT
AGTCATAACACCTTCTATTCTAGAATCCCATTATCCATGTACTTAGTCTGACTACACACCTAGGCGCCGCTGTCGGGCTC
ACTTATCCATAAGTAGAACCGTCTCAAGAACACTTGATTATGCTTCCTGGCATCTTCATGGACTGACTATAAAACAATAA
TGGTTTAAATGGACGCATCATAATGTGTTTATGTAAATCTCCTTCCTCCGGGTCTATTTTGATAAGCTGGATCCTGCAGC
TCAAGTCTGTTTTGCTAAGCTGTATCCTGCAGCTCCTCACACTCACCAAGAGAAACTGGCTGTTGGTGCTTACATTGACT
GAACAGAAATGCTGAGGCATATGTGAAGGTAGCGTGCGAGATGCTAAAGCTCCATTGAAAAGCCACACTTCACAGGTGAA
AGTCACCCTAGTCTTCAAGTCAAGTCTACCCACCCCCATCGGAGTTCAGCCTTTCAATAATCATAGGAACTCACAACCAG
GGCTTCTAACCAGGGAGTGCATAAAGCCACCTGCGTAGACAAAGCAAGGCTGAGAGCCATGTGCATGCTGTCTGGATACC
AAATATCCTCTGATCTCTCCCACTCAGTAATTTTCATTCTAAGTATCTTCCACACATTTATCATGGGTGGGGGATGTCT
CTCTTTCTTTTACATAAGGAAGTTTGACCATTAAGGCATTACAAACTTGACTAGCATCCATGCTCCCTATACTACCTAGT
TAAAAAGCTATTGAGAAATACCATGTCCTAGGAAGTACCAAGTTTCTGATGAAGTAACTAACTGGGTCCTTCCCCCATCA
CGTTTACAGTAAAGTCCCAGACTTTTAGCTAGCTAGCATGGCAGGGTGTCTCAAGCGTGCTTAGATGAAAACAGTTAAAA
TATGTCTTGTACTGAGTCACTGTGGAACCATGCAAGCCAGAAGACGAGTCACAGGCCTAGCCAATCTGGCATCTACAGGG
CTACTACTGTTTTCCTTAAGTGTGGGCCTCACTCACAGACACCTGTAGAACCACTGCACCACCAGCCACGTAACTTTTTC
TTAAGAACACCGTCTCTTAGGATTCGAATGTATCATTACATTTGTCCAACACCCACTTGGTCACAAGCACATAATGGGAC
CTCTTATGTTAGAATTGAGTCTCATCAGTGACAGAGTTGACCCTGGTAAGCAGAGGGAAGCTTGGGAATTGAAAAGCCCC
ATCAAACACAAGGTAGCTTTATTCTTTTCCTTCCCCTGAACCCATTGTATCCCTAAGGTATTTTGTATTGCTGCGTCAGA
GAAAGCAAGATGAGCACTAACTCGGTTTGTTTTTTCTTTCTTTCTCTTCTGCGCCAGTATCACTAGAACCTTCGCATCAG
GGAAAACAGAAAAGGTGATCTTTCAAGCCCTCAAGGAACTAGGTCTTCCCAGTGGAAAGGTATTGACTAACTTAAACACA
TTTCTGAATCTGTTTTTCATGGTTGGTTGTCTGTGCAGGCATATGTGCTAAGCCAGGTGACGGTTAAGCTGACCTCAGTTT
GTCAGTCACACGGAGGGCAGATCACTTTCTTTCTTTCCGTTCGTCAATCACTTGAGAAGTGTTTTAAAATCCAGCTAGTT
CACTTCAAACTGATCTTTGTCAACTTTATTAAGCTAAGAAAAAGAAAGGACGGGCAGCTGTCCTCTGTACAGTTTATGGA
ACCTGTTTAGAACACCCAGCCGTGTCTGCCTTACCAAGCAGAGCAGCAGCATGAATTATTGACTAAGAAATTCAGTTTCTC
CAGAAAGCCTTGCACAATTTCGTGAAGGATTCCTCTCCCTCCCACACATTTATACTCATGACACTTCGTATTCAAGTCAT
TAAAATTAGTTTTTACACTGCAAGCAAGACTTACGCCTCTGACTCTGTTTAAAAGTCTTGTTTGTAAATTGCATTGCAGT
GTGATGGGTAAATTATTGGTTGTTCCAGTACCTTTAGAGGATCCCTTGTGATCCTCTAAATATTCACAAAATAAATGTAC
TTAAATATTCGGCCTGATTATCCCATATCCTCTATAGGAGCACTTCCTTTCCTTCCTCAAGGAGAGATAGAAAAATGTCT
GTGTACTTGGTCAATGAGGGGTTTCCCAGAGAAAGCTGTGCTGCAGGCAGGTATCACCAAGTGTCCCATGTATCACCCAT
TCCATGGTGACAACCAGGGAAGCCTAACTTCACATGCTGTGGTTGTCCTAAATGTCCATCTCAAGTCTCCCAGGAGAAAG
ATGTTTGTTTGTTTTTTAAATGCCTGTCGTTCTTTTATTCTCCACAGAATGATGAAATTGAACCTGCTGCATTTACTTAT
GAAAAGTTCTATGAACTGACACAAAAGATTTGTCCTCGGACAGATATAGAAGATCTTTTTAAAAAAATGTAAGTTCTAGT
TATGAGAAAGTGTCATATAGTACTTGTTATTTTGTCCCTGTAAAAATTTGAAACATTTTTTTTGCTGTAATGTTTAAATAT
ATGTTGCTAGCGTTAGGCCTAGTCAGGGTTACATTTTTCACCCCTTAATTTATTGGCGTAGAAGCCCATTGTGAGTTTTT
TAGAAAACTTGACATAGATTAATTACAGCACTTGGAACATCCCCCTCCCCCACCCCTGCATTGGCTTTACTAACGACCTA
CAATTTTAACCTGCTCCTTTAGGTATTAACCCAGTATGTGAACTAACTAGATTTGGTTCGGAATGCTGTTGAGAAGTTCA
TTCATTCCAGTTATTTCAAATGGATGACTTACATGTTGTTTTTGGAGGAATGTCTCTTGATAATGCTAAAGAATTGCATA
GAGCAGTCACTTTCTGTGACCTCGTGGGAATGTTATCTTTCCGCTATCATTGTCCTTGAGTTCCTCGCACCCACCCCTCC
CCCCCCTCCACTACAGTCTCTGACTACTCTAAGAATTCAGGGTTAGAAAAGGAGTTTAGAATTATCAACAAATGTCAACA
TGTAAATCGTACCTACTGTACATCATATTCAAAAACTTACTTTTTCTAATACATTTCTTTTCAGCAATGGAGACAAACT
GATTATTTAACGGTAGACCAATTAGTGAGCTTTCTAAACGAAGTAAGCTTTTTCATACATTAACTCCATCAGTCTGTGTG
```

247

```
CAGTGGCTTGCCTCCCCCGTGTCCTCACTGCATGCTGGCCTCACGCACCCTTTCCTGGCTCGGCTTCTGACCTGCTGATC
TTTCCATGGTGGCTTGACCAGGTGCAAAAATCAGCTCCTGTCTGTGCGTCCATGTGCTTCCTCCAGGCTGGCTTTGCACA
CCTCCTGTTGGTCTCACGCTGTGGTTCTGTGTGTACGTGTGAAATTTGTGATGTATTTATTCAGTTGAATGTGTCTGATA
TTGCAAGATGGTTTTTAAGATAGTTTGATTCTGTTGATCTCGTGAACAGAGAATGGATTGAGTTTGTGACACTAAATTTG
GATTCTCCATTGCTAACTTTTCTCATACCTTTGCATGACCTGGAAACCTGTATTTTTTTTTTTTTGCCTTTTAATAGAT
TGCATGTAAGAAAAATACATAGGTTTAAATTGAATATTTGACACTTGAGCCCCTAGATAAAACTATAGTGTTAGGCCAAC
TAGATTGCAGGTTAAAATTTTATGTATTATTTTCAAAACAAGCTCAGAGCTGAATTTAGACCACGATTCATTTAGCAACT
AAAACTGGCTAGCAGCGTTTGCTTAAGAACAAGGAAGATATTTCAGAACAGGTAAAGGTCGAAACTTTCATTATTATAAC
TAAAACGAGACTTTTCTTCTCATGTGCAAAAAATACATTTTCGCAAAACCCACAGAGGCTCTTTTTTTTTTGTCTGAAAA
AAGAAATTCACCTTTCCCCTGAAACACATAAGTAGTTAGAAAAGTTTAACTATGGAGTAGACAGTACAGTCTTGTCTGGA
AAGCATTTAAGTTCTAACTAGGGGATCTAAAGAGAAAGGTGGTAGGAGAGGTTGAGAGGCCTCTCGGTGTGGTAGGACAT
ACATAGCATACAAGAGTTCAATCCCTAGCCAAGAGACAGGGAAGTTAGCATCACGTTAGACACCACCTAGTGCCTTAGAG
GAAAGAATGGGCAGTCCCTGACTTGCCGCCTTACAGGAACAAATGACATCTTTTTCAAAGGACTCCTCTTCATGATTTCT
GCAGGCTCACCTGCTTCCAGTGTATGAGGCTACACACCAGGAACTGTTACCCCGAACATAAATGCACTGAGACAAGAAAC
AGAGGGGGTCCATATATGCACACAGATGCCTCCACCCCCCAGGGGATCCGTTCAGGGCAGTACTGTTTTGATTTTACTCA
AAGCTGTTTTAAGTCTTTCCGGTTCACTGCACCTTGGGCATCGAACGCTGTGTTTGTACACAATCTTTACCCAGATCTTA
TCCACATACTTGGCACATTTCTCACTCTCTTTCCTTAACCCTCTGCCTCCTCCCCTTCTTTCTCTGTTGCCTGTTTTTCC
TTTCAAGTGGGGGGGACCAGCAGGAACCCAGCTTCCGAGGAGGTGGAAGATGCTTTGGGACAGAGGAGAAAACTTTACCAT
GCCATCAGTTCATCATCCCTTCCTAAGCCAGCCATCTTCCTAGATAGAAGCTCGTGCTTTCTGACTGTATTTTGCGTTGT
AGTCCAGGACCAGTTCTGAAGTCCCAAGGACAGGTGCTGCTCTTGCAGGCTCCTGCAGGCTTCATTTCCTCTCACAGATG
GTTTTCCTTTGTCTGCCTAATAATAAATTTTATAATATTGAAAGAATTTAGTTTTTTTATTTGTAAACCCTATAAGCTTT
TATTTTTAATTGGACAATTTAAAGGAAACATACCATTCTGGCTCTGCCATCCATATTCTTCATTATGAGGCAGGTCTTTC
CCCATCACTGGTCCAGTGGACAGCCATACCTGTGTTCTTCATATTTCCTCCCATGTAGCTTCAGCAAGCAAGGAGATACT
TTGACTGTGTGGATGAGACTGATGGAATGGACATGTTTCTAGATTTCTGCAGATTGTAGTATATGCCTTTTAAGTTTATG
CCGTGGGATTTCAATCATAGCATCGTCTCCAAAGACCTCTTTCTTCTCCCAACAGCAGTCCCTCGTCCCCACCCCTTGCC
AGCTTCTTGCACATTCTATGTGCAGTTCTACTTTTATCTTGTTAACGTCTCCAGAGTCAAGACAGCGTTTGGCAGGGCAG
ATTCACGTCTTGTAACGCAAGGACACAGGGCTCATCAGGAAATCAAGAGGGAGTGGAACATGAAGGTCTGTGACCCTAAA
AAATAATTAGACCTCGACATTTTCTAAGTATCTCAGCCTTGTAGCCTTAGTAGGCAGTTTCACTGCTGCCTGGGACTCAG
CCTCATGCAGAAAGACAAGAGTAAGTGAGGCCCACCTGAAAGGAGGCCCTGGCCTCCATATACGCAGAATTTAGAGCAGA
TTGTTATACCTCGATGACAGAAACAGTCAGCTTGGAAACCCATTCAGTCAACTAGAGAACCAGTTATAGCAACAGCAAAA
ATCAATAGGTGCAGATACAACAAGGTCAATAGGCTAGAATTGACTGGGGAAAAGAAATCTCCTTCCCATTAACCAGTGA
CACAATGGCTCCTACCATCTTGCAGCCTGAAGTCTTGTTCCTTCTTTGTAAATTTCTGTTTAGTATACTCATCTCCTACC
TTCAAGAGACAGGAGGGGAGGGAAAGATTCAGTCCTTTACTTGGTTGCCGAGTACCTTATGTGCCCCCTCCTCTGTCAGTG
ATAGCTTTGGATTCATTTCTCATCTGCTTTCCAGGGCCTCTTGCTCATAGAATGCCGCAGTCTGATTCCCCAGACAGGTA
TCATTAGCATCTCCTAGTAGTCCACTAACAATGCACACACACTGAGTTGGTAGTTGTCCCTTAACAAGGTGGTCCAGGTA
ATGTGTGCACATGGAGGTTTATGCAGCACTGTCTAAGCTAACAGGATTCTTCTTTGAGGTGCTTATTTGAGTGGGAGAGA
AGCTGAGCCATCATGCATGCCTACATACATGCATACATACATATACATACATACATACATACATACATACATACATTACGG
TATTCCCCATAAAGAATTTAGTCAGCCAAGTTCACTCACTACTCACATCTAAACTTCTCCTATCACTCTGCCTCTGGGTTCTGT
ACAGTGGCCAAAGGGAATCTTTCTTGCCCACATCATGAACTAACAGGCTGGAGTCAAGAAGAGCCAGTGGAAGCACATGC
TGGGGAGCTGCCAGGGTTGGGGTTCTCCTCGAAGCTCAGTTGTAATTCCCAGGACTAGTCGCCATCTCTGGGATCAGTTGGT
TTCATATTCTTTTTCCCGGTTGCTAATGAGATACATTGATTTTAGTATCACCTCCATTTCTGTTTCACTTGTCTCACAGAA
CAGCTAAGGGCTTGTAGAACGGGAGGGCTGTGTAGAGAGCGTGAAACAAGCAAAATGAGGAAGAGGGAATGGGCCTCCTAT
GGTGAGAGCAATAAAAACAAAGCTTTGTTTTCTTACCTCTGGTTACCTACGCCCTCACACACATCACCCACATCATCTGAT
CACCATAGCACTAAAGCTGATTTTTCCATTTTCCATTTTTATAGTTGGAAATAGGTAAGAAAGCACCCCAAATCTCTTTC
TCTTTATGATATCTTTATTTTTTATGAAAAGGGAGAAGAGAGAGAAAGAGGGGAGGGTATTTCATTCTATGAGTGAACC
AATCCTTGCTGATTTTTATTTTCACCTTTTATATAATTTTTGAAATGATATCATTTATATTGTATATCGTTCTATTATAT
CATCATCTATCCTTCCCAGTGCACTTGTAATAAGGTCAGTTGACACCTTTTCTCAGTGAGGCTAAGGACAGAGGTGTGGA
GACTGTCCCTAATAGATCTCATTTACCTGGGCACAGTTTTCTGTGACCTACTCTTAACAGCTATTTAGAACCTCATTTTG
CAAAGCACTGGGTACCAGGAAGTGAAAATCCTGGGCATGTTGTTTATCTGACTGGAAGCTGATGTTATCTCAGAGCTGTG
AAGAACAGGGCCTCACTGTGCTTGTTTCTAACTAGAACTCCTCTGCATATAATGTTACTCTGAATATGATATGATATGAT
ATGATACGATAGATATGATATGATATATGATTTGATGTAATATATGCAGCATTGGTCCTTACTCTGATCGCAGTGATGAAA
ACCTTACCATTTGTTTCCCTGCCTCTCAGTTATTCAGAGGGACCATGCTATGTTGCCTTTTAAAATGTGAAAATTTGGGG
GAAAGAAAAGATTCTATTATTTGGCTGAGCAGGTCGAAATGGAGTTTTATTTTTGTCTGCACTTAAAGCTCTTTTGCTAA
GACTGTATCCATTAGGTAGCAAATACTGATATGTGCTGTGTTCCCTGGCTATCCAGTCTGCTATATGGATGGTCTAATGA
GTATGATGTCCTCTAATGATTTTTAATGGATAAAGCTTTATTAGGTACACAGGTCAAATGATTAGTCCTACCTTGCAGA
ACTTAAGTATTTGGGATATTTTGTTTTAGTTTGGTTTGTTTTTTGCTTTTGTTGTCATGGATATACAAGTGTATTAAAAT
GCATGTGGTTACAGGTTAAAGCAATGCTTTTTGGTTCCGTTCTTTCTTTCCTTTTATTTTTTGAGACAGGGTCTCATATT
GGCTGTTCTCCAACTCACTACATAGCATAGGATGCCCTTCAACTCCCGATCCTCCTGCCTCTGCTTTCCAAGTGCTGCAA
TTACAGACAAGTATCACCACAGCTGATCAGTGCAGTACTGGAGATTAAGCCCACAGGGCTGGGCAAACACTCTATCCATT
GAGACACACCTCCTATCTTAGAGCAGTGCTGCTTAAAATGAACAAGATAGGATCACCTGGAAAGCATACTAGCCCTCCTC
TACCCCAAATAAGGCCTGGTTTCACCCACGAATCAGTGTCAGTAGATCTGAGGAGAAGCCCCCAGAGGCTACATTCTTTA
```

```
AAATCACCCACATGATCAGAATGCTGCTGGTGCCAGGCACAGAGAATATTTTAAGTAGCGCCATGTTTGTATGTTCCCCT
GTGTATAAAACACCCAGATATGTCTGAATTCCCTTCCTTTTTCCCCGTTGCCATATATTTTCTCTCTCGTGGCTATATTC
CCATTTTACCTCCCACATGGATTTGCTCCATCATTTCTCTTAAGGTCCAAACGCTCAACAGTGATTATGACCTAATTTTC
CTAGGAAAGATAGAGAGGATAGCTCTGTGTGCAACCATGCCGGGGTGTGAAAGAACCCTAGCCATCCCTTATCGGTCTTC
TTTAGTGCTATTGTTTATGAGGAAAGAATGATTTCTAGCGAGTGGTTGAGGTTCTATTTCTTTGAATGTGTTTAAATGAT
TCTTCTCACCCCAGGGTGGGTTCTTATCCCCCTGAATACTGGAGCGTGCCGCATGGTGCAGCTCCTTCAAGGGTGCAGCA
TGTCTTCACTTGCCTGGCAGCCTCCTGATCACATCAGTCTCTCTTTCTCTGCAGAACTCAGCAGCATTCTCTCCAGAACC
AAGGCCTGCTGTTGTTAGTATAATAGTTTCCAACATATACATAGCTTAATCAATAATTTTTGTTATTACAAAAGAAGCA
TGCTTCTTCTTGATAGTCTGTCAAAAGATTTCATCCCTTTAACACCCCTAACTAAACAGGACAAGGAGACGTCTAGACAC
ATCTTTGTTTTAGCAGTTAAGACCAAGTGTTCCATGGGCTGAAGGTGTTGTCCTGTACATAGTCCTCCTTTCCGAACCTG
TAACAGCTGCTTCTAAGTGTCTTCCTAATAATATTGCAAAGTCTGTGCATGCATCTGGGTAAAGAGTTGTTGCCAAAATA
CTATTTGTTGAGCAATTCTTGCTCATATCTGCTAATTGTCATTCTTCCACAAAAGTTTGTTTTTACTCCTTTGAGGGATA
ATCATGGACACAATGCAATCCTGTCATTCTCTGAAGTATAAGGAAGTATAGTGGACCCTCTATTTCCAGACATGTGCCAT
GACATGTGAGCATATGAACATACACACACACACATACACACACAAACACAAGCACACACACCTGTGCAGGCACATACA
TGCAAGCACACACATACATGCACACACACATGCAGGTACACACACCTGTGCAGGCACATACATGTAGGTGCATACCATGC
ACATGTGCTCATACACACACACACACACATACACACACACACACACACTTAAATAGTGATTGGGTTTCTCACAATT
CTAGGAAGTTGGGGTGAAAGAAATGTGTTGCTCCTGTAACACAAGAAACATCTTGTGTGCTGGTTAGGAAAGGTTGCTGT
CAGCAGCTTCTGCCAGAGCCTTCTTCCATACACTGCACTTGGAGTCCTAGAGTAAAGTGTGTTCAGCTTTAGAGTGAAGA
TGTTCTGCACCATTGCTTTTTAATTTCAAGGTTTTTAAAAAATATAATATACACTTATAAGTGTTATGTGCTTATTAATT
TTCATTATTACAACCATAATTTTTTGGAAAACACTGGACTCAAGAAAATTAAGTTTGCAGGGTATTCAAGAACCTCTCAT
TGTTAGCAAAAGACAGATTAAAAACTATTTACTTATTCTGTCATTAAGAGATTTTGCTAATACTGTTTTGGAGTTTTGGG
CCTCTCAGTGCATGATCTTGTTGCTTATTTTAATTTTTTTCACTTTTTAAAATTAGATTTAATACTAAACTTTTACATAT
TGTAAAGATTCGTTGCTCTCATTTTTACACATATTTGTAGCAAGTCACATCCCTATACCACAGCCATGTGAATCGTAGAA
GTAGAAGCCAGTGCAGCTGACATTCTCTGTCATGTGTCCCTCAGCATTTAAACAGGAGAAAAGCACACATCTCTTTCTTC
TCTGCCAGGCTCCATCAGAGTAAGTTCAGATATGGACTTTCTGAAGACTAGGTTAGGGAGAGAGTCAGGAAGGCAGCTAA
GCCTGATCTTGGTGATTATAAATACAATAGTCTAGATGTACCTTGTTTTACAAATTATATGCTTAGAAATATGTTTCAAG
TATGAGGGTTATAGATCTAAATGGACTTATTTTGTCATCACAGCATCAGCGAGATCCTCGGCTGAATGAAATTTTATTCC
CATTTTATGATGCTAAAAGAGCAATGCAGATCATTGAAATGTATGAGCCTGATGAAGAGCTGAAGAAAAAGGTAACATC
AATGGAAAACTTTAATTCAATTTTGTTTTTGTTAAGACAGAAAAAGTGCACAGGCTTTAAAACCCTTGAATTTTAATTAA
AATATCTTAAATGCTTTCGAGCACTGAAGATTCAAAGATTTGTAATTTATTTATTCTTTTCCTGCTCTTAAATTAATTCA
ATCTGTATATTTTATCAAGCGAGTCCACACTGTCTGAAGTACACTACTACTGAATATATCATGTTGTTAAGCACAGTCCC
TGTACATGCTTAAAAATCAAGTTTTAATTTTGCATAGTGTGCAAAGACAGTCTCTCTGCAGTTTTCGAAAGAATTTCTGT
CACATAAGAATCTGCACCTTTCTTTTCTCCCTTTTGTTTGCTAAGCTTAATAAATTTAGTACGTAATGTGAGGTGCTCT
AGAATTCCTAGAATTGTGAGCTTGGGTTGCAAGATTAAAAACACTGATAGAAAATTAACTAGTCAGGAAACATGTCCATA
AGTTGTTCATATCCCTGGACCTTTGTCCCTTTCATTTTATTTTGGAAACAATATGTTGTTCAAATATTAGTCAAAGGAAA
AATGTGGAGAAGACTTCGGGTCATCCCTGCAGCAGGGGCATGCATGCCTGCGACCCTGGCATGTGGAAGTAGGAAGCAAG
CACCATTGCTCTTGCTTTGAGCAGCTTTGTCTTATCATTGTGACCAGGATACCTGACAGGAGCAACTCAGTGGGGGAAAA
GGATTTCATTTGGATCATATTTTGAGGAAATTTTCATCTGCCATGGCAGGAAGGCATGGTGTCATGGTGTGAGACACCTG
GTCACATTATAGCAGGACTAACAAGCAGAAGCATCCAGATCCCAGAGATGCCCCCAGTGACCTACCTCAGCTAGTCTGTT
CAGTAACCTTTGAAACAGTGCCACCTACTGGCGATGAGGTGTCCCAACAGATCCACCTGTGAGGGATATTTCACATTCAA
ACTGTAACACTGGGAATTCAAACATGAGTAAGCACCCCCCTCCCAAGGGCAGTTGTAGTTTGTTCAGAGCAAAGACAAGTG
GAACAGTTCCTTGGCAGTGTGATAATGTTATGATTTACGTATGTCTTTGTTACTGCTCAAGATCTATCAAGACCTAAAGT
CTAATTATAACTGGCACCACCTAAAAACATAGCTTCTCCCTTTCACAAGTGACAGAAAATTAAAGCCTGTCTTAACTTGG
TCAGTGTAAGAACACAGCACAAAATTCTTTCCTCATAGGAGGTTAAATACATACATTGGGATTGGGACAGTAGCTCATAA
AATAGAAAACGTGCCTCTCTGCTAGCTCAGCATTTCCACATGCAGGTATGTACCCTAGAGAATCTCTCATACTCAATTCT
AGACCTCAGATTCTTCACTGCCTCTGCTTGAAGAAGGGGTGGTCTGCAGGCCAGTTTTGTTATCTTCTGTATACAT
GTGACTTTGAGAGTAAACCCTCCAGTCCTAACCTCTACCGTATACCAAGGCCTTTCCATCTGCACGTTGTAAGGGGATGT
TTGCAGCACGCTTTGATTTCCCTGTACCCAGCCTTCTTCCTTTCCCAGCCTATAAAATGCCACCCTGCTCACTTAGCTTT
CCAAGCCAGAACCCCGTAGTTTTGAATTCTTTCACATCACGTATGATCTTTCAGGAAAACTCATGAGATCCACTTCTTTG
TTCTCAGCTTCTCAGATGCTCAGGCCTACAGTGCCTCTCATGGGCAATGCTAATTGCCTTCTGATTTATCCCCTGACCCC
TTATCCAGTACTATACGTCACACAGCAGCCAGAGTAAGCCTTTTAGAACACTATTTGCTGGTATCATTGAACTCTTCCAT
CCCACCAAAGCTTCCCAGCACACTTGGAATAAAATCTCAAGTGCTTAGGACCAAAAACTGGTGAATGTGGCCTTTGGCTT
CCTCTCCAGCATCATTGCCCACCACTTTCCTAAGATGTCGCTCTCTCTACCCTACATTCATTGAATAGATCGAGAACACA
CACTTCTGCATCAAGATCTCCGCATTTGCTCATTCTTCTGCCTGAAATCCCTGCTCCTGCTTGTGTGGTTTGTCCCCTTA
CTCATGTGTGTCCTTATGATCTCACTGGACACATATCCAAACCAGGATCCTAGTATTCTTTTGTAGCTTTCATCTTGCAG
GCTTTGTCCCTTCGATACTTTTAAGGCATTATTTGCTCATATATTTATTGCTCTTACCCATAATATGTTAGTTTTGTAAA
ACTGGAATATGTATCTGTTTTCTGCATCTATACCAGTGTGTCAAAAGTATTTGGTGTTAGAATGAACGAACACTCTCTTA
TGCGATCAGTGGAGCTCTTCTGTAGGTGTGAAACTGTTTTCATAACTTGCAAATGGTGACATGAAGATACAAAGGGCTGA
GGCTGCCAGCGCTTGTGAAACATTGGTTATTCGTTCTGTGCTTGCCATGTAGCCTTGCCCCATTTTAGAAAAGAGTGACT
GAAGTGAGAGGTGCCCTGTATCTCAGGAATGTTTCTCACCCAGCTCTACAGGTTACCTCCAGACAGTTGTGAAAATTACGT
TGTTGAATTTTGGAGAGGCAGCCATTCCCAGGAAAGGTCCCTCCTTTGAATGAATGAAAATTCCAGTGACTTAGAATTAA
```

```
TGCCACACATCCTACAGGTATTCTCAAGTTACCAGTCCTGGAGGTAGGTGAGCTAGCATTCTCTCTATTTTTCAGGAGAA
ACCAGGAGACAGGCAGCGAAAGGCAGGTGGTCATAGCAGAGCCAGAGGCTCCCAGGTCCCTCCTCACCATGGTTCCTAGC
ACAGTTTCTTCATTTGCAGGACTGGAAACATCTGCTGCTACTGATATGAAAGGAGAATATTTATAGACTGCCTTGGTTTG
CTTGTAATATGTAATCGTGTATTGTTTAAGTTCTGAAAGGTACGGGTTCTTGGGAAGGACCATGAAGTTGCTTCTGCTCT
TATTTTTAAGCTGTCACAACTAGGCTGAGCAAAATGTAAGATGTGCATCTTCAGACACCCAGTGTCATTAAGATCTCCTT
TCTTCCTAGGCCTCATATCCAGTGATGGATTCTGCAGATATCTGATGTCAGATGAAAATGCCCCTGTCTTCTTAGATCGC
TTAGAACTTTACCAGGAGATGGACCACCCGCTGGCTCATTACTTCATCAGTTCCTCCCACAACACCTATCTCACTGGCCG
GCAATTTGGAGGAAAGTCTTCAGTGGAAATGTACAGACAAGTTCTCCTGGCTGGTTGCAGGTCTGGAAATCAAGATGGCA
ATTTGCTTTGTTTGTATATGGCACCTGTAGATTTATAGTTGTCCTCCCCTTATCCCTTGGATCCCCAGTGTTCCAAGATG
GCCAGAGGATCTGCAAAGCAATGGCTCATACCTAATTCTATATATCTACACCCCATCCCTATATATCAATAAATATGATG
AAGTTCTATTTACAAATTAGGCACAGAGATAGTTTAGCAACACCGATATTAAAATTTGCCCAAGGATATACTGTGATAAT
ATCGGCAGCTTCACTGCCTTTCACCTTGGGGCTGCTATTATGAAAGGAAGGGTTCCTTGAACAAGCACTATAGTTCTACA
ATATTGTGAAACAGGGAAGGCCACTACTGGCTGACAGGTGGTAGTGAATACAGCGTGATGTGCTGGGCCCTGCACGGATG
CTGTTCATAGGCAAGATGCTCAGCGGCCTGTGCCACCCATGACTCACTTGGTGTTTACGTTAAATGTAGAAGTTGCTTA
TTTCTAGAACTGTGCATCCAATATTCTCCAACCAATGTTGACCGTAGTTAACTGAACACAGGTGAGGGAAGGCTCTTTTA
ATTTACTTTTCTCAAAAGTCAGAAATAGTAATCATCATGAAAAGGCTCTCTACATTTTTCTTTAACAGATTATCTTCATT
TAACTTTAAGTGGGAACAAACAATATCACACGAAGGACCCAGTTATCCTAGGAGTCAAAAGACACTAATTTACTATGATT
TTAAAATATGACCTCAAAAATCTTAAAACCAGTAAAGATAGATATTTTAAGAATCCATATTAAGTTGTATATACCCATAT
TTAATNNNNNNNNNNNNNNNNNNNNNNNNNNNNNNNNNNNNNNNNNNNNNNNNNNNNNNNNNNTGGTGGGGTATTTGCTTAGCCTGTGC
AGGTCTTTAATAAAATCCCTACCACTGCCCAAAAATACAAAAATCACATTTATCAAAAAAAAAATGTGTCTTCTGATTAT
AAAGCTCGGAAGTTTAGTTCTCTGAGGGGAGGACACTGAAAGTGATTTTTATAGCAATAAACAAATAATTAAGGTTGAAG
TGGTGAAAAAAAATAAGAAATATAATTGTATTTTTTTGGAAATTTTTTTTAAGGCTTGCTATGTTTTGTGTCTGGAAGTG
ATGCAAGAGAGAATAACTCCATCCTTACTTCATTGATGAGCTTTGTCGGAATACCTGTGTGTGAACCCTAAAACAGCATG
ATCATTTGTTTCTTGCTTGGCTTTAGGCACGGAGATGGGAGCCCCAAGCCTGCTAAGCAAGGGCTTGACTGAGTTATGGC
CCAGGATTTCGCTTCACTTTTTATTTTGAGAAAGAATCTCACTTACTTGCCCTCGTTGGCCTTACAATCTCAAGACACTG
GAATTGTCCATTTTCTCCCCCAAGGTCTTTTCAGTGATGCTAACCCGGGCATTAAAATGTAGTTTTGACCTGCATACCCA
TGAGATACCCATGTTACACACTCAGCTTTCTGACCTGTAAGATGCCCCTTATTTTGATGCCTCTTGGACTTAGTGTGTGG
TTTCCTGTTACGGGCTCAGTGTTGTAATCTGACTGACTGTGTTCCTAGGTGTGTTGAACTTGACTGTTGGGATGGAAAAG
GTGAAGATCAGGAACCGATAATAACTCACGGAAAAGCAATGTGTACAGACATCCTTTTTAAGGTGGGTTTTAAGTGTTCA
CAATCATAGCTTTCCCAGACTTTTGAACAAATGCAGGAAACAAGAAAACAATTTTTTTCATTTTCCCAAACAATGTACCT
TCACATTTCTTTTTCTCTCTTTTTAAGGAGAAAGTCAAGACAGGTATTATTCGTTGTATAATATTCCCTTCATGGTCTTG
TTTGCAAATGATAACTATAATGCACAGAGATCCTTTAGCAGAGTCTACCTAGTTCTAGCCCAATACAATTAATTTTTGGC
CCAGTTGGGTACATACAGCCTAGCTACTCGAGATGCTGAAGTAGGGAGATTGCTTGAGTTTAGGATTTCAAGGCTAGCAT
GGAAGACACAACGAGAAATTTCCTCAAATTAAAATCAACAAAACATTTTTTATCTTACCTATCTATTTGCTTGTCCACTT
TCTGTTATTCCTGAGAGACTCCAAGGTAGCATAAAAAGATAAACAAAGCTTATACATAGCCTTATAATAAGGAGACAGTG
AGGTTGGCATTGTGAATGATTGTTAGGAAATATTAGAAATATGGAAACTTTACCACAAACCAAGCTCTACACTTTCAAAG
GATTTAGAGCCAAGAAGAAAACATGATTCCTAGGACCAGCAGTAAGCATGGTCATTTCAGTACCAAGGCCAGTGAGAAGT
TCCCCACTGAAGCTTCCCACCTAAAGTTAAATAGTTAATAAACAAGGCATCTCATGGGATCTTTGGGTGGTGTCTACATG
TGATTTGAAGGCTAGTTTCTTAGTGTGTTCTTCCACATATCCATAGTATTCCACCAAAGCTAAGAAAATGTGTGGTAGGC
TGATGTCAGAAACATCCATGCCTGCTGGTTTGCACAGTCTAGGATAGATGGTGACGATGTCTGAAACAATGGCCCTGAGC
TCTGGTGACTACTCAAATCACCAAGGTGTCTTTCAAACCAGAGATGCCTCAATTCTGTACATGGAATGTTCCATCCATAT
CATGTTTCTTAGACAAGGTATTTGTGACTTTCAGTCAGCAACAATTCAAGATCATGATTTCTAAGGAGAGTTAGGGTGCA
TTCAAGTGAGCAGGTATGAGTTCATACAGTTATCAAATGACAAAACCAGAAATGTCCTCCTACAAGAACTAAGGAGATCA
GTCAGGACACAGCATGGGAAAGAATCACCCAGAAGGAAAGCAGACTGCAAGGGCAAAGCCAGAGTTTTTCTCAAGTGAAA
ATTCTGGAACATTTACTAGGCAATTAAGTGTGTGGTCATTTTTACCTGTGAATAAACTTTAAGAAATAACAATTAAATGA
CTGATTCAATAGGATATTTTCAAGTTTGTCATTTTATCCGCCCTGCCATGGATAATTATTGTAGAAACATTTTCTGACTT
GCTGAGGAGTGACGATGCTTGCCCTGTGGTTAGCTACAGACCAGTAAGAACTTGTAGATTTATAGTGTGAGAGTGTGATT
ATAGAAGCTTTGGTTCGAAGTCCCTTTCTCTAAAATAATCATGAGGGCTAACCCTCAGATTCATCTTGTCTATGTCATAA
GATACAAGTTATGGGGAAGTGTCAGCCCATTTGGAAGTAGGCAGGGATATAAAAGTGAAGTCAGAGCCCATGCCTGTTTT
ACCTCACATGTGACCTGAATACAGAGGAGACCCACTTTTCACTCGGCTCACCGGAAACAATGGATATTGCAGCTGTCATA
GCAGTACAGTAGGTCAACAAAGAACGGCGGGCACAGACATAGGCCAAGATGATTCCAAACTCGGAGGAAGGACAGTTAGA
AGCTTCCAGAAATCCTTGTGTTACTACCAGCACATTTCTCCAGAAGAGTGAGTTGAAGAATAGACTGTCATGAAATGCC
TGTACAAAGCTTACGCTCGCCTCGAGGAGAGTGATGCGGGCCAATGGCGTGGCTTTGTGGTTGAGCACATCCTTCAAGGC
TCTGCATTCAAAGTGCTACACAAAAGGGGGCAAGAGGAGCCAGAGTCCCTGGCTCTGCGCATTGTCAAGTGAGAACCAGC
AGGCCACTCAAGTAATGTCACTTAATAAATAGCACTGGAGAAATAGAAGAGAGCTTTGACTGAAAAGCAATGTCCTAAAC
CTCTTGAGTCCCCCAGGAGGGGTGTCTAGGCAGACGTGTTCTCCAGGTCAGAACTGTTAAAGCTGACTTTCTGAAACGC
TCAGTTAGTGGGCTGTGAACGGTGTCAGGTGCCTTAAGTCTGTCTGCTCTTTTCTTTCATAAACACTCTTTGTCGTTAAA
GGATGTAATCCAGGCCATCAAGGAAACGGCGTTTGTCACATCAGAATACCCTGTCATTCTCTCCTTCGAAAACCACTGCA
GGTATAAAGGCTCCTTCTTTTCCACGTCCCTCCTTGGTGGTGTTTCAGATCCCTCACTCTTCGTCCTGCTGTTCATTATT
TCTTTTAACTAACTGAGTAGTTTGCTGTCCGATGTGCTAAGTCGCAAGTCACCCTCCAGTGAGGACACAGTCATTCCCAC
CCTGTCTACCAAGAAGTCATCTTCATGTGACTCAGTAATGCTTTCCCTGAACAAAAGGCCAGAAATTAGGGGACACCTAG
```

```
TCTAAAGTTCCTTTGAGATGGAAAATTTGGCAATAACTTGGAATGCATCCTTAGGCTTTTTTTTTTTTTAAATTTAAAGT
TGTACCAATTAAAGTCTGTACACTGTATTACAAGTATAGCTCCAGTTAATGCTATAAAGACAGCCCTGCACTTTGTAAAG
CAGAGGAGTCTAGTTGTCAGAATGGATGTCTCTCTTTCTTGGAGAGCTAATGGACACTTGAGTTTACAAATGCCACTTCT
TTCTCCCCAGCAAATATCAACAGTACAAGATGTCCAAGTATTGTGAAGATCTATTTGGGGATCTCCTGTTGAAACAAGCA
CTTGAGTCGCATCCAGTACGTATTTTTAGCAAACCAGAGTTCCAGCATGAATGGATTTGTTTTGAAATGTGTTTTTCAGG
GGTCAAGTAGCACTGGAGGCTGAATACTAAGGATTTTCATATTTATTTAGATTCTCTGGTACAAAAAGCTTTTATCGTGC
TTCAGAGGGATGTAGAGAGGAACACAAAGTAAACATCTGACTTGGTTTAATCCGGCTGCTCTCAGGACCTCGTGTTTTAT
GTTTCATAAAGCTTCCAAGCCAAGAGTCCAGGCCCCTCCGTGCGTCCCAGTGTCCATTGCATTCTGAGTCACTTCAGATA
CCCGTAAAAGCACCTGCACTAGGGCCCCACCTACACCGGTTCGGTTCTGATTGACCCAGGGTCATAAGCAGGTGCATGAT
TTAAGTCCTCCAGGTGAACAAATGTGTGCCCAGGGTTCAACACTCATGGGAGCCCGTCGCTGTGGGAAAAGCTCCAGACT
GATGTCCTAAGACACAGAGGTCAAGTTCCCAACTCCAAAGTAAATACTCAGGCAAAGTCACATGACCGTTTTTGAACTTC
ATTATATACCTGCCAGGTTGTTCATTAAGCACTTGTTATTAAAGTCCGTGGTCCATAGGAGGGCTTTATGGAGTCTGAAT
GCATTTGTAAGCGTTCCAGAGAATTCCTTGGATTTCTGTCAGGATCCCTAACATCCAAGAGGATGAGATGCACTGTGGCA
AGGTGTTACGAGAAGCAAGGCTTGCTAAAATTAAAGTGCTCTATTAGGCTGTTCAGCCTTCCTGTGACTATGGTAGCATT
CGACAAAAGGAGAGGGGCAGGAAAGTCCATGCATGACAAGAGGGTGTTTGCTACGTGAATGACTTGGCTGCTCTAACCGT
GGATTTTTCCCAAAGGTGTGTGTGGCAGCAGAAAGCGCATGGAGGCCAGGCTGCATCCTTTGCTCTCTGCTGCCTGCTGA
AGCACTGGGGCAGCTGCACTCCCCGCACTCCCTCTTTCCTCCACACTTGGCCTGACTGTTGGCTGCCAGTGCCTATGGCT
CTCTGTGGAATATGACCTCTAGTCAGTGGTCAGGCTCCGATCACATGAGAGACAGGCCGGACGGGGTCTGAGAATGAACT
CCCCCACAAATCCCCTCACTCGGTGTTCCTAAGGGCTATTTCTGAGCACCTGTTCTCTTCTGACCCTTGGGATCCCCAGC
TAGATTCCCTGTTTGCCACCCTGGGGTGCCCCAGTGATAACATCTGAATTACATCCTTCACTGGCCGTCTCCTCCTTTCT
CTTGCTTCTCCACCTCTCTGTTGACCTGTGCAGGAACCGGCTTAAACTTGTGATGTGTTAGTTCTTGTCTAGGAAGATGA
CTCTGAGGGATTCTGTCCAAAGAAGCATCCTTGATTTATTTTATTGCTGTGGTTTCCTTAGCCTTCTGTTCTCTTGGGTA
AGAGAGGAGACTTGATATTTGCAAAGGCCATTGTGTTTGTCTTAATTAACTAAATAATTAATGTGCATCCCAAATGCTGC
TCATCCCCCAGGTCCCTCCCTCACAGAGTCCCTTCCCTTCTCCTCTGAGAAGGTGGGACCCGCTGGGTACCCCCCAACCC
TGGAAGTCTCTGCCAGATTAGACACATCATCTGCCACTGAGGCCAGACCAGGCAGCCATGAAGACTGAATGTTCTTTGGT
TGGTGGCTCAGACTCTGAGAGCTCACAGGGGGCCAGGTTTGCTGACTCTGTAGGGTTTGCATCCCCTTCAGGGCCTTCAG
TCCTTAACCCCAACTCTCCCATAAGTGTCCCTGACCTTTGTGTAATGCTTGGCTGTTGGTATCTGCATCTCTTTCAGCTG
GGTAAAGCCTCCCAGAGGACAATTATGCTAGACTCCTGTCTACAAGGATAACAGAGTGTCATTAATAGTGTCAGGAGCTG
GTTCTTGCCCATAGGATGGGTTTTAAGTTGAGCCAGTTATTGGTTGGCCTTTCCTTTGGTTTCTGCTCCATCCTTGCATT
TCTTTTAGACAGGACAAATTTTGGGTCAAAAGTTTTCTGGATTAGTTGGTGTCCTTATCCCTCCACTGGGGGGTCCTCCA
GGCCTCTTCAGGTTCCATATCCCCACTGTTAGGCATCTCAGCTAAGGTCACCAGCATTGACTACTATCCCAGGTCTCTAG
GACTTCCTAGACATCCCATTTCCATTTATTCTCTGGCCTCTCCTATCTCTCACTACACCTGATCCTAACCACCCATCC
CTCTCTCCCACCCAGTTCCCTCCGTCCCTCTGCCTCCTATGACTATTTGTTTCCCCTTGTAAGTGTGATTTTTTTTTT
TTGTGAAGGGACGTACACATTCCCAGGGAAGGGACCAGTGCCACCAGGCAGCCTGCAGCAGGCAGCCGTCAGTTAAAAGT
CCTAAGAAGTCAATGCATAGACTTGCTTCTGAAACATAGGGTAAAGTAATGAGTTGTGAGATAAATGGTTGTGTGGAAAG
ATGCATAGGTAAGAGCGTGTGGCTAATAGCTGGGTGGATGGGGAGTGAATAGGTAGACAGAGGCCAGTCCCACTGATCAC
AGAAAATGACCTAAACAGACTTAACTTCCTGTAAAGTACAAACATGACACCACAGCGAAGTTCAGATTCTAGCCATTCAA
AACTCTCCCATGTTTAGAGAACCCATAAAAATCCCACGTCAGTCTGGTGAGAAGAGAGGCTTAGTCTGTGACCTGCTGCA
GACGCAGGGGCGATCTGAGGCCATTCAAGTGCTTTATGATGGACCTGAGATGTGAAAAGCCCACCATACAGCAGGACTGC
CACCCCCAGCCCCCTCTCAGGTCAGACCCTACTCTACTAGGGAGCTTGGATCCATCTGTATACCCCAGAAGACTGGGGGTG
CTCCAGCTTTACAGCATTGTTTAAATTAAAGTTGAATTCAGTGAGCTAATGAGCATTGGGCAGAAGCAGAGCTGTGCACA
CTGCCATCTAGTGGATGCTGGCTGGTATGACTCCCGGCCCCTGGGGCTCTTCCGCTCCGGAATCGAGCATGAAGTCCCTA
CCTAACTGAGCTCTTTCTATATATCTCTAGCTTGAACCAGGAAGGCCCTTGCCGTCTCCTAATGACCTCAAAAGAAAAAT
ACTCATCAAGAATAAGAGGCTGAAGCCTGAAGTTGAAAAGAGTAAGTCAGTTGCACATGTGGCTTTTGAGCAGTTCGGCT
GTCTAGATGAAATCTGATTTAGAACAGATTTGGGAAAGGAATGGTGGAATGCAGATAGTACATTTATCTTCCAGCGTTAT
TCCACTACATTCCCATTAATCCTTAGTTCTTACAACTGGTCCATTCAGTGGAAACCCTCCATGTGTTTACGTGCAAAAAA
TACAGAAGGAAAAAGGGTTTGTACTGCTACATACATTGCCACGGTTTTGTTCATAAAAGATAAATTAATTTACGCTCACA
TTGGAAACATCTCAGTGTTGTAAGGCCGGGGAAGCGGCTCCAGGGTTAAACGCTCACCGTGCATGCGTGAGGATCTGAGT
CTGATCAACGAGAATCCACACGCTTAACCTGAACACCCGTGATGCCCCTAAAGACGGAAGGGGAAGACAGAGAGTTTCTC
ACACTTCCTAAGCGATCTAGCATCTGTTCTCATCTCCGTGTGTGATGATACTTACAGAACAGCTTGAAGCTTTGAAAAGC
ATGATGGAAGCTGGAGAGTCAGCCGCCCCAGCTAGCATCTTGGAAGACGACAATGAAGAGGAAATAGAAAGTGGTGAGTT
GTGGGGCCGGGGAAGAAGGCTGCATGGGTAAAATGCTCGCCGTGCAGGCGTGCAGATTATAATCTGAATACCCAGAATCC
ACATGCTTAACACGAACACCTGCAATGCCCGTGAAGATGGGAGGAGGAGATAGAAGAATTTCTTTAAGTCCATGAGCCAG
CTAGTTTGGCAAAAGCACTAGAAACCACCATGAGAGCCAGCCTCAAAACAAGATGGGAGGTGATGCCTGACAACCAAGGT
TGTCCTCTGATCTCCATGTGTGCACTGTGGACATATACTCACTGTCTTTCACATACTCTCATTGGCACACATAAACGTAT
GCCACAGACATCACCACACACACACACACACACACACACACACACTCACTCACTCCCCGCACACACACTCATA
CACACACACACACACACACTCCTGCACAGACACGTACACACACTCACACTCCCCGCACACACACTCATACACACACAC
ATTCCTGAACACATACACACTCCTACACACACACACACACACACACACACACATACACACTTCTGCACACACAAAGAGTT
ATGAGATGTCATTTTAAGGTACAAGTTTTTTAAAAAGGGTTATATTGGAAATAAGAAAAACATTAATCTCTCTTAACAAACT
CCTTTGACTAGCAATGACTTTATCTTGCTAAATATTTAGGAGTACTTCATCTTTTGAGGGAATGGCATTTTTATAGATAC
TGTTATTTCCATTTTATCTGAGCACTGGATGTTAGTGGCTTAGCTGTCTCCCTTCCCATTGTGAACTGAGCCCTTCAGAA
```

```
AAGATTTCCAGTCAGAGACTCGGCCTTTAATCACTTGCTGTATTAGGCAGGAGTTGAAGAGAATTTTTGAAAGGTCCACT
CTATAATCCTTTATGTTTTGTAAAGAAAAACATGAATGTCAGTTTTGTTACTGATAGTAATAAGACATGTGATAAGAGAA
GCGAGTGAAGTGATTGACTGATGCTCATAAAGTGAACTTGAGAGTCAAAGCCTATATTGCAATTTTGTTAAGCCTGGGTG
TAGCTCAGTGATATAGCACAGGTTTAACCTGCATGAGGCCTTCTCAACCAGGGTCCCTGGAAGCCTTACTGAAAATATGA
ATATTGACCATCACCTATTTAGCCTCTCAATATAGCCAGCCATGATGTGCTGTTTAAAATTGGTGCTGGGGTTGTAACGC
GATAGCATATTTGCCTAGTCTGAGCAAAGCCCTCGGTTCGGTTCTCTGCACTGAAAATAATAATATACTACTTAGTAAT
AATAGTCACAAGTGGGCCATTTGAAAGTTTGGGGATGTTTTCAGATCGTGATCTCCTGGCCATGTATCCTGAGAATTACC
TGGCCAGGTGATTGGAACTTGCATTGCTTTATGGAACCATGGTGCACAGACTGACTTAAGACTAATTACTTGTAGAAATT
TTCTTATTACTCACACATATGGACTATGTCTGATTTTAGACTAGGTACATAGGTGAAACCCAAAAGCTATGAGAGTTACC
TAATTTTACTCACTGTCTCTTATTGAAGACCCTCACTGTGGGAGAGATTCCATTAAGAAACAAGCCAAGTTTCCATAGGG
TTTAACTTCAATCTCAGAGAAAAAACCATAGCGTTTGGCTTCCTTGTGTTTCTCTGTGCCTAGTGGGATCTAAATGACCA
AAGATCTCTGGTATTATTCAGTTAGTTCCTATAATGTTTGTGCCTTATTACATAATGCACCATCCACATTTGGAGGCAGA
AATCTGTGGTGATTTATAATTACCTCTCAGGATTTTGTGAGCAACTGGGGTTTATTTGGCTAGTTCTTGAGTTTTTTTTT
AATACAGTTGCTAGCAGGTGGACATCTGGAGTCGGCATCTTTTTAAGATTTGTAGACAATTTAGACAAGATTGCTCTCTA
GTTTAACCATCTGTGGAGCTCAGCTGGGTCATTGTAGCAGAGCATCCCCTGTGGCCTCTGTGTAATTTCAGCATCTCACA
ACATTTGCTAAGTTATACCATCCAAGAACCCGGGCCCTAGATGCAGGAAGTAGGAGCTGTTACGTTATTTAAGAACTATG
AACACTTCATCTCTATTTTGTTCATCGACGCAGTGAGTGGCAGATCAGATATCAGTTCAGCTATCTTTGGATGTATAATA
TGTAACCATTAGATTTTTCAGATTTAAAAAAAATACAAATATTTAAGAATAATATATATATATATATATATATATATATAT
ATATATTAAGAAAATAAGCGCTCTCATTCTCCAGTGCCCCTGAAGCTTAAAGATATCCGGTAGTTTGTACACAATCCCTT
AGTTATAAGACAGTGTGAGGCATCCGGCATTTAGTAGCAAAGTTAAATATAACTGTGAGTGCTCTATGGACTTGTGTGTG
CTCAGGAAATGCTAACATATATTCAGATACTCTGTATAAGTCCTAGTTACTAAGTATCTACCAGGAATATACGGTTTACG
CCTAAATAATCAGGTTAGACTACTTATGCAGGTACCCATTATCCAAAATACTTGGCCGGAGAAGTAGTTCTGATCTCAAG
ATTTTTCTACATTTAATTTTTTTAATTTCATAATCTGTGGCCCACCCTGCTGAGCCCTGGAAAACCAGGCTTCTGGGTGA
CTGCTCTTACCCCCAAGCTCACATTTTCTGATTCCTAGCTATTTCATAGATTACCAATTGAGTATCCCTAATACAAAAAT
GTGATATCCAAAATACCGTGAAATCTGAAACTCCTTGTTTGTTACCACAATGATCACACAGCTTAGGATTTTGGAACACT
CTGGTTTTTTGACTTTCAAATTCAAGATAATCAACTGTGTATGAAACAGACACTGTAGAGGTGGTGCGTGGTCTCCCGGT
CCTGGTTATGTGCGTAGGTCATATGAACCTTCTCTCGAGTTCTCTTAAGGTTATGGGCAGGGTCTTATTTAGCCTCATGA
ATTTTCTTTCTATCTGTTAAGCAGCTGATCAAGAGGAAGAAGCCCACCCTGAATACAAATTTGGAAATGAACTTTCTGCC
GATGACTACAGTCACAAGGAAGCGGTTGCAAACAGCGTCAAGAAGGTCTGAGGCATTGTATTCCCATTCTTAAATGGTTT
TTAAGGGGTGGCAGTTATTGTGGGAAATTTGGGGCTGTGACAACAAGAGAACCAGGTATTGTGTTTTTCTCTTAATTATA
TAGGACAAAATTCTCTGCTGTGGGACCAGAATTTGACACTCTCACAATATTAAGTTTCAGAGGACCTATAAATTACTCAT
TAATTGTACCCTCTAAGGGTCACTACCCAATTGGGCAGGTACTTTTAAATATCACCGTTTATAGTAGTAGAGCTGACAAG
GAGAGACGCAGTATCGCAGGGCCAAAGGTTACCCTGCTGGCATCATTAGGAAACTGCCTTCTGGATGTGTCAGGTGAAGC
CTCGGTACAGAGCTGGTCCAGCTGGCAAGAAAAGCATCCGCCTAGCAAAAGGATGAAAAAGTCCAGCCATTCAGCCAAGG
AAAGTTGTCATTGGGTTCCTAGAACTTGTCAGGCCCAGCTTAGGATGCACTTTCATTTTGCCTTTTGTTAGCTTCTACGG
GTCTCCTACAGTTCAGGAGAACAGATGGGAAACACGGAGTAAAAGAGTCAAGGAAGCCTATAATTCAATTGTGGCTCGAA
CTATTATGACAATAAAAATGAGTGCTAAAGTGGAGACCAGGAGAGAACAGGGCGACTGGAATAAGGAAGAGAGGGACTCA
CTATGCAGAGAAACAGAGAATAACATTTCATACCACAGGACCGCAGTAAGAGCAGAGATGGCGTGAGTGGAGCAGATGGG
ATGATTGAAGGGTGAAGCAAGGTGATATGTGTCAGGTGGAAATGAGGTAATTGAGAAGTTTCTATGAGAAATTAAAAATG
AGGGTGTAACATGACCTGTTTCACATAAAACTAAGTGGGGAGAGGGTATCATAGAAGACTGTGGCGGGCCCTTTGTGGCC
TCTCACCTAGAGGAAAGTAAAGGCAAAAGTGTGGAAAACAGTGAGAGTGGGCTTTTAACTGGTCAGAAGCAGCCCTGGTG
GTTAGAGAGTAAAGAGGGAGGAGATAGAAGAGTCCAAGGCATAGCAAGGGCACAGGCAATGGTACAGTTTCCCGATGATG
GACTGAACTTCGTGAAGGTAAGGAGAAGAAACAAGGTAACCTCCTGGAATCCTTGTTGGGAGGAGCAGGGTAGGCAGTGGTACT
GTTGACTGGGGTGGGAAACAATGAGAAAGGACTCTGAAGTTTCCAGTGAGACAGGCAACTGGAGAGGTCTGTAAGCTGTT
CCAGTGGACGTTCCGAGGAGGGACATTGATCCCTGACCTCTTGTTCTATCTAACCTTGGGTAAGTTATTTAACCTCTCCC
CGATTTTGCTTCCCCATCTTGAAAACGAAGGTCATAATGTCTGTTTCATCAGGTTCTCATCACAGAGCAAAAGAGCTTAC
ATCGGCGCCGTCCACACGGCAGCCGCTGTATAGTAGCAGGTCTGCTATGCTCTCATTACTTTCATCCTCATTATCACGGT
CGTCAGCACTCATACAACAGCACTGGTGTGTAGAAAGGAGGTTGAGCAAGAGCAGTGAGTTCGAGAGATAATGTTTGAAG
TCGTGGGAATTGCTTAGATGACTCACGAGAGGTAGAAAAGAAGGCCCGTGTGGAGCCCTAATCCATAGAGGGCTCACGGG
AGAGGAGCCAGGAGGAAAGAGCAAAGGCTTTGCCAGCAGCCACACCGAAGAGAACAGGACAGTTAGGGGCTTTGGATAAG
AAGACCTGTGTCCACTAGGTTAGTAGCATGGAGGTCAGTGGGATAGGGAAGTTTTGGGGAGAGCACTTGGAGGAAAGGAAG
TGGCGGGACAGACAGGCAGAGAGGATGAGGGAATTGTTTCCGTCTTGAACTGGAAGAATCAAGTGCGCTGATATCCGAGAT
GTGAGATGTGGAACGGATGCTCTTTTCAACCTGGCTGATGTCTCCTGCTCTGGAAAGAAAGCCCACTGGTGTGGTGAGACT
AGACGAGATCATAGCAATGTCAGGGTGTGGTCGTTAGTGAAGTAGGGACGCGCCTGCTTTGTGAGGATGATGGACAGAGATG
CAGGTACAAAGGTTTGATGGCAGAAGGATAGGAGATTATTTGTCTGGGAGAAAAGTCAAAGAAATGTTATCATTTCTGAT
GACAGGGAATCCTGAGTGCCATGAGAGAGCAGATGCCCACTAAGACTCAGGATGCTGCTGTGCTCCACAAAGTACTCAGC
AGTACTGTCTCAGGCGGGGCTGGCATGACAGAAATAGCAGACTTGCTGGTTCAGATACCAAAAATGTATTTCCTGATGAT
CTCTGGGATGGATGTCAAGGTCAAGGTGCCAGTCAATATGTTCCAGAAATCTCTTTTAGCAGGTACATTGTCCCATAGTG
GAAAGAAAATGGGAAAGTAAGATTTTTCTTATTTCTTCTTCTAAGAGGACTAACCTCACCATGAGGATTCACCTATGACC
TGATTACTTCCTGCAAGCCCCTTCTCCAAGTTTGGATAAGCCTTGCCTTACAATGTGATTATGATAAATCCACTCTAAAC
ATTGAAAATGTAGGTTAAAAATGCACCTAACACACTTAAATTGAACAGTATAGCTTAGCAACATAGCATCCTCTAGAACT
```

```
TTGGTCATTTACCCTCAGGCTTAGGTGGCTGACAAGGAGCCACTGTCCTGCATCACAAGAGGACTCCAAAGTGTATCTGT
AGCCATAAAAAGAGCAACCTGACAGTATCGAGTGAGTTTCCACTGAATGAATACCACTCATACCCTCATAAAGAAAGCAA
TCATAAACAAGCCATCATTATATTGGGGACCATCAGTCCATCACACTGAAGTCAGGGTTCACCACATGCTTTTGGTTGGG
ATGCAAACACCGAGTCACAAACATGAATGTTGGCTTACCGACGACCGTTTGCACTCCATTTTACATTTAAGGGAAGCTGG
CCACATTGACAACCATTTTCAACCTTCATGCTTTAATCCTTAGTGAAATGTTTACAGTGAAACAACTAAGACTGTCAACA
AGGGAGGGAAATAGCATCAAGATGAGGATTGATAAAAGTATAAAACCATGAATTTTTAGAATACCTGCGACATATGCTTT
CGATGACGGCTTCATGTCATCAATAATTTACCTACCATTATAAGAAATAAATAGAGTAAGAAGTTTACATATGCTTATTT
ACCCAAGGACAGACGAATGAGTTTGGAAATTTGCCCTTTAGAGAGGTAAACAAATAATTCTCTTTCTCTAATTTAGACC
TCAGATGACCTTGAACATGAAAACAACAAAAAGGTAACAAAACAATCCTCAAGCATTTTCTCCATCAGGAATCACCCGCA
TGTGGACTTCTCACATTAACCTTGATGGTCTTCCTGGCCCTTGCAGTGCCAGGGTCACTGTTCATAGTATTCACATGCCT
GATTCACCTAGTTGCTGTACAACCATTAAAGCCGATTTAAGACATAACCAAGGCAGGCGAGACAGTTTCCCTAGAAGTAA
GTATTCAATGTACTACACTATCTCCTTAAATGGAATGAGAGTTCCGTCAAAGGAGAAGGTTACATAATTATTAGCATATT
GAATGTGAAGATAAAAATAACCAGTAGGTAATCGGAGCCATATGATAGTGTGGTGGAATCGATCGTGGTTCTGAAGGTGC
CAATCCTCTGAAGGTTACCATTCATCATAGGGTTCTCAGAATTCAGTCGCATTGGGCACTGGCCTTACTGTGAAGAAAAT
ATATTACATTTTGTTTTCTTTTATGCTCCACAAGGTTCTCGTTGTTTGTACACGTATTTCATTGTGCAATGCCGAGACCA
ATGGCAGTCACGCACAGAATTTGCCAATTCCCCGGATTGTCACTCACAATTCAGAACAGAGAACATCCTGCCTTGCTGTC
TGTAGGGTCTTGTTGCATATAGAATGAAGTGTCATCTTATTTCCTGACTATTCATCCTGAAGGGATACCCTCCCGTGTTC
ACAAGGACCCTCTATGCCCGGATTGATTTTCTTAAGATGGTAGGGTCAGAAGTACACAGACTGGCTAATTCGATGTTTTC
CATGTTAGCTGGATGATTTTAGTCCCCAAGGCCTCAGTTTTGCCCTGCGGCTACTGGGAGTTCTCATGATACACGGGGGT
ACATTCCAAAATCCTTCAAGTTCAGTAAGATGAGGAGATTTCAGTCTAAGTGCAACAGGACCCCGCAGACAGCACGGCGG
GCATACCCTCATGGCTCCAACATACAGCACTGGTGTTCTGAGCATGAGACACATGGGACAAAACAGATTTGGCTCTTGCC
CATATTTGGCATGGTGAGTGGACTCTGGGCTTATATGGAAAAAGATGAAAATGGGCATAAGTGTCATGTACTCTATCACA
GACTTTCACAGGAAGAAGCAGGCTGATGTCAAAGATGCAGAGGTTAGGTCCTCAGCCAAAGCGAGCAGATAAGCTAACTG
ACCTTACTCACTGTGCACCCGAGCTGTTTCTGTTCTTAGATTGAGTCTCTTGGAGGAAATAACTCCAAAGTTCCCAACA
AAATACCGACCACTTTTCGGGACACCCATTTTGTTGATGAATGAGTTATTTTTGCTTTAATTTCTCAAGAAATGCAAACA
GTCGTTTGTAGTTTTTCTCTGTACAATGTAATATGTCCTATGTAGCAAGTTACCAAGTTCTTTTGATAGTAAGTGCATTA
ATAGAGATAACTAAATCATATGTGTGTGTGTGCTGCAAGTTCCTGAAGAGATTACTTTAAAACGTGGTCTCCAGTTCCCC
ATTGGCAGTGCCTGAAAACTCGGCTGTGGAATGACAACCCTTTAGGCCTCCTCCAGCAGATCTCTTCCAGCGGGGCCTGC
AGTTTCTGCCTACAAAGCCTGTGGGTGATTTTGATGTATGCCATTGCTGGATGGCCACCCAGTGGAGCTTTGTGTATTGG
CCTGAATGGTAATACTGCAGTCTATCACTGTCGGTGTTGGGTGAGACCTGGTAAAGTTTCTTGAACGTTTGATGGTCCTT
TGAAAACCTAACAGATAAAATGAATGCTTTCAGGTTATCTCCTTTTGTATTAAAACCTATGACTTTTAAAGTAAAACTTT
AAGAGAGCAACAAGGTCTGTTACATATTAATTTACGCAATGCAGATACTCTTTTAATTAGGATGCCTAAACTGAATCCGT
GAGTCCCTTTGAAGCCATCCACTTAAACGGGTTTGTCCTTGTGCTTGCAGGGCCTGGTCACCGTAGAGGATGAGCAAGCA
TGGATGGCATCTTATAAATACGTAGGTGCTACCACGAACATCCATCCGTACTTGTCCACGATGATCAACTATGCCCAGCC
CGTGAAGTTTCAAGGTTTCCACGTGGCTGAAGGTAACATTGCAGGTTAAATGCAGTAGCTTTCCTGTCTGCTTCCCTGTC
GCACTGGCTGCCCCGCTGATTTGTTGCCATCTTTTTTTCTTTGTGGATGTCCATCACTTCACCTGACTTGATTCACTTGTG
GTGGTATTATGCTGTCATTCTCCAAATCATCCACGAGGTGGCGGTGCAAGACTGTGACTTAGCTTGTAGCTTTCCGTATG
CTTTCGTAAGGCTTCTTTATTTACTTTGTTATGACAAACTATATTAGTAATATTTATGAGGCACAGTACCTGTCTTTGTT
ACCTGTGTGTGTATATTGTGTAATCTATGGCTTCAGGAGAGTGAGGAAAACTTAATCAGTGGGTACAAATGCCTTGAGTT
AGGGAAGTAAGATTTGATGTTCTGTGGTACATAACTCCTATTCAAAAGTTGGTTCCCCACGAAAGCCCGTTCAAAAGTAG
TTAAAGGTATTTCCTAACCATTTCTGGCCCGAAGAGTACGTTATTATCAAAAACTGCATATCGCAGCAGTGACTATGACA
CTTGGGTTGCATATCACTTGTTTAGTGAGTCCACATTTAATTCATCATATGCACAGAAAGTTAGTGAAACTCAGAGCAAA
TGACACACACAGTCATGTAGCATGGGCCACGAATTCTTCCTGAGCTGGGATGTAAGATGCTGGTTGTGATTCTGTCTGCT
GCTTGCTTTTTGAGGGGTTTGATGGCTGAGTTTCTGAGTACATCTTTGCTTGTCTTTTACAGAGCGCAATATTCACTATA
ACATGTCTTCTTTTAACGAGTCGGTTGGCCTTGGCTACTTGAAGACGCACGCGATTGAGTTTGTAAAGTATCCTAACGGA
CTGTATGGGATGAATGGTGCTCTCAAGGACCGGCAGGGATCTCTGCAGTAGATTGGCAGTGGTCTATAATTTTAATATTT
AGCTTTTTGTATAATTTTTATCATAGCGCTGTAAAATGGTGTCCCCAGACGTACATGCGTTGTTTCCAGAATGCACACCG
CAGCTGTGATTTCCTCTGAGCAGTGTTTATAAGGGGATTGGCCCCGTGCTATCTACCTGCTTGCCATTGTCCCGCACCCC
ACTGTTGTTGTTCTCTGTTGTGCGTTCGCCTTGGCTGCTCTTGGATTTCCTTAATCAGTTACCCAGTTACAATAAGCGAC
AAATGAGCCGCATTTACCCCAAGGGAGGCCGAGTTGATTCCAGTAATTACATGCCTCAGATTTTCTGGAACGCTGGTTGC
CAGATGGTTTCACTGAACTATCAAACCCCAGGTAGGCGCTAATGTCCAGTGACCCCGAATTCCACAAGAACATTGGGACA
GGGCAGCCATTGCAGCTGCTCCCGTTGGCCTGAGGCATTTTCTTTTTAAAGGAAGCTGCCATGTGAAAAAAATTTGTTTT
TCTTGCATTTATATTAATGGCAAAAAATTAAATCCCCAAAACGAAGCCATAACAGAAGATGCCATTTCAGTTTTAATCCA
TTCCATCAATGCTATGAGTAGCCCCTATTCTATAATATTTTTAATTTCTCTCTTCTGTATCAAGCTTACATCTTTGTT
CCCAGAGATGGGATTTGGTTTCAGTATCTTTGTGTGATAACTTAGTCTCTTTGCTCCTTTGGAAAATACCACAACCAAGG
TGGCCATCCAGCAAACACGGATGGCAGCAGCACCTACCATAATATGACAGACTTTCATCGTAAACTCTGTAAGATGCCAT
GCAAAGAGATGTCATCAGAGTACTAATCTGTAGTTCAATCTTCCAGAATATATTCACGGGCAGGGTTTCAACATACAGTG
GTGCTTTTTTTTCCCCTACAAATTCTAACTTTTGATAAATGTACAAATGCTTGAAGTTTTCCAAGTTTGCATCAGAGCCT
CTGACCATAATCCTGTGACTACAGATTGGGAAATAGGAAAATAAGCATTAAAAAAAAGAGAGAGACAGATGGCACAAACC
ATATTTATTTTACATGACCGTGTTCTTCTGCATGGCTTTAAAATTTGGTAGAAACAGGAATAAAAGTAAAATAGCTAACT
AAATAGCAACGCATGTCTGGTCAGTCCCTCACTTTGTGAGCAGCACCCTTGCCAGGACTTTGCAGGCATGGAGCCGTTTT
```

```
CTTTTCTGGTCACTCTAACCTGAGGACCACTGTCCTTGCTCCGGACTCACCACCTCCTTTTGGTGGGAATATGAGCTGGC
TGTATTTTTCACGCCTGGGGGAGCCTTCCTGAGGGCCAGAGCTCCTTACCAATGGATCACTGCGTGGAATCCTAGCCATC
CTTTCTAGAAGAGTCTTTTCCAGGTCTTGAAAGAATCTCGAGTGTTTTTAGTAGGGACAGGACAACACGATGGCAGTGCT
TTACTTACCAAGGACTCAAGCGGCCCTCCAGGAGGCCAGCGAGTCCGTGATAAAGGACAGCGTGATCCTAAAGCAGTTCA
TTGTCTGGGCAAACGTGTGCTTCATTCTGAAGAATGGAAAGAAAAATATCCACTTAATTTAGTTACTGCTCCTCTTCCTG
CTCCTCCTCCTCCTCCCCCTCCTCCTCCTCCTCTTCCTCCTCCTCTCCTCCTCTTCCTCCTCCTCTCCCTCTTCCTCTTC
CTCCTCCTCCTCCTCTCCCTCCTTGTCCTTCTCTTATTTATAAGAAGAAAGAGAAACCTTTCTCTGCTTACAAAGAAAT
TTATAAATCAGGTCTTCCCATTCCCAGGGTAGCCCTATGAGAGTTCCAACACTCACACAGGGGCTGAGGGAGGGTAAGCA
GCAACATTTTATGATTCTTATCCATTTTATAATGTTCCTTTTAATCAAGTTCTGTGTTCTTAATAGCCCTGTTGTGTGTC
TCCAGCATCCCTGTCCCATGTGGCATTGAGACAACGCATACCCCTAACTATAGCTGGTGTCTATATCCCCAACTACTACG
TCTCTACTCTGAATATCGAGTTAAGCATCGTATGACTTGTATACCAAATGCCGCTTAGTAAGCCAGTTCTAGGGTAATCT
CTATGAAAATGTACCTGGTATTTAATTTAAACTAAATATATGAGGTCATTTCGGCATGGAAAAGAGTCATGGCCCCTAAA
CTAGGCCAATAGAATGCGATGGTTTAATGGAGAGAGCTTTGGACCTTGGAATAATATGGCTTTCATTGTTTCCAGTCACC
CTGTCAGGATCTTCTCTGTCCAGGGAGATGAGATGTTAAATTACTTTGTCTTTTGACAGATTTAGCGATGCAATTGAATC
AACGAAAATTTGAGTATAATGGATCATGCGGGTGAGTGATATTTAAATTATTGTCTCCTCATCTCAGACTCTATACCTCA
GCTTTAATGAAACCATGTCATTTTCAATTACGGACAGCTGAATGTATGCTGGTTCTGAAGTGTGGGTAAAAATAAATGAA
GTCCATAGTTAGATTACATTTACAAATATTCGACCTTTTCTCTGTCCCACTTTGTTAAACTGGTTCTTTCCTTTTAACCA
CAAACTCTGCAGTTCCTCACATATATTCTAGAATTTGATCACTTCATTCGAAATTCAATAGTGGAGGTTCTTGTGTCAGA
GACAGGCAATGGGCCGTGCTCATCTGGGCTTCAGCCAGAGATTTAGGCATCAAATATGTATAGCCTTATTGCTGATGAAT
ACAGAAATGAAGTAAATTTCATTTCAAATGAAGCTGTGGGTGTAAAGTTGAATTAATTCAGCCAGGAATAAACTTTCAT
CCTCTCTTGAATCTTTTGCATGTTTATAAATAGGAGTTATATAATGCTGTGAATGTTGTTCTATTTTACTTAAATAACAC
TTAAAAAATCTGTGAACCAAGTAGCAAAATAGGAAATGTTAATTCTTCTGAAATACCGAGAAGTTGAATAAAAGTCCGGA
ACTTGAATTCCGTAAGCACCAAAAACTCAGCTGAGACCACAGCCCAGTGGCTCCCACATGGATGCGTAGGAATTCTCTGT
TGGCCAGTGGTAATGCTGTCTAGGAGTTCTGTGTGGTGGCTTGCCTAGTGAGCTACCTTAATCTCCATCATGGAAAATAA
GGTGAGGTAACAGGTTTTCAAACAACTCTAATATCTGAACACTCTCTACCTGTGCACATCTGTTAGTTATCTGCCAGAGT
GCCTGTACATAGCAAAACTCCTTCCGTCTGAGTCTCTCTCACCCATACCCGTGGAGTAAGCACTACATTGCCCTGTGCTG
TGGACTGTGAAGGGCACTCAAGTTATATAGAAAGGAGGAAAAGACCCACAGCTAAGTTAACAAGTAGTCTTTCTACAAGG
AAAAGAGGCAGACATTTGGCCAAATGTCAAAGGACTCAGGAACTAAACCTTTGGTAAAAGGAGACAGCTTGTCAGCATAC
CACTCTCTTCCCCTTGTTCAAGCAAAGAGGTGGTCTGTTGTTTAATAGCACAGCACACCCTGAGCAGCCTAGGTTGGTTG
ATGATGCTCTTCAGGACCGTTAGAACCTTCTACCTGTAAGTGTAGCTCCTGAGTTATGGAAGAGAGAAAGGAAGTAGCTT
GTATTTAATAGTCATTGCACAAGCCGTCAGTAGAATAAACCCCCTGTGTTTCTGTCCTGCCAGGTACCTTCTCAAGCCAG
ATTTCATGAGGCGGCCTGATCGGACATTTGACCCCTTCTCTGAAACCCCTGTGGACGGGGTTATTGCAGCCACGTGCTCA
GTGCAGGTATGCCCCTGCTCACCACAGACTATTTTATCATACAGACGTATACAAATGCACCTAGAAGAAGACATGTTTAA
CATCACGGTGTAAGAATTGTAAGTTGCTCTCAACTTGGACCCAATGTATGCTGTCTTCTGAGTTTTTGTTACTAAAACTA
TGAGGTGCTTTAAGTAAAGTACACAGTCTAGAAAGTATCTCCCATCTGATGACATTCACATGAAGTTCTGAGAAATGTAA
GCTATGCTAACAGGGAGCAAATTAGCACCTGTGCCTGGACACAGATGGAAGGGTCACAGAGAAGCTTCGGGGGCACTAGG
AAGGCTTGCTGTCTTCACTGTGGTGATAGTTCTAAAGACACACTCACATGTCCATAAAATTGATCTTACTGTATAACTAA
CTGCATATAGTTTAGTATATATCACTTATACTTCAAAAAAATTGCTGTTTAAATGAGATAATATGAGAACCAAATAAAT
GAACAGCATAGTTGTTTGGAAGGTAAATGTGAAATAAGTTACTCCCTAGCTTTATAAAAGATAAAACTCAACACTGACAT
TTACAATTAAACAACTCCCTCACCGCTAACCAGGGTTAATGTCATAAAGAAAGTAGGCCAAGTCTAGCACTGGTTGTCTT
TTGCTGTGTTTCTTTGCAAGAATTCAGGGGTTAATATGCAGCCTGATAGAATCCAAGAACTAGAAGAGATCTCAGGACAT
AGCCCATAGCCGCAGCCCTGGCTTCATTCCACAGGTCCGGACAGCTCCGTGACGTGTGGAGGGCGTGATGTCTTCATAAT
ATACTTCACCTGTATATTTCCTTGCTGTTTTTTTCCAGCCGTATTCAGAGGAGAGTACATTTATAACCTCTGCCTAGGAA
TTTTGCTTTCAAAGAGTAGTTACTCAAAAACTGTTTTGTTCTAATATAGAAAATTCCAGGAAAAAGATCAAATAATTGTA
TTGATTGGAAATTCCCCAACGAAAGTTTTCATCACACGCCTTGCCACCAATACAATGAGGCCACTGTCCCCCCTACCCTG
TGTCTGTGTAACTGCAGGTTATATCAGGGCAGTTCCTCTCAGATAAGAAGATCGGGACATACGTGGAAGTCGATATGTAC
GGGCTGCCCACCGACACCATACGGAAAGAGTTCCGAACCCGCATGGTTATGAACAATGGACTCAACCCAGTGTATAATGA
AGAATCGTTTGTGTTTCGAAAGGTAAGACATTAGCAGGGTGTCACTGTTACCAGTAGGAAGACTCATTTTCTACATGAA
GCCCACAGGATAGAGGAATGCCACTCACCACACTGACTCGGCTCCTTCCCTTCGTCTACTAAGTGACCTCGGTCACTGCC
GAATGATTGTGTGCAAAGAGTCGGCTGTTCTGGCTCTGCCCCTTCCTTGGCCACCTCTAGTCACATAGAACACACTTCCA
GGTTCTAGTCTATTTCAGACGTGTTCTTACTTGTGGCTGCTATCATGCACTGAAGGGTCTCATTCTGTGCTCACAGCTCA
AAGGTTAGATGAAAGGCTGCACCAACGCTAAGGTCAGTGCTGTGACCCTGGTGGGCCCTGTGATCAGAAGTGAGGCACCG
CTGCTCACTTCACCGCTGCTCAGAGTTTTGAGTTTCTTCCAGTTCTGTATGCCAGGTCCAGGGAGCAAGCAGACGTCCTC
TCTCGTGTGCAGTCTTTAATCTCTTTGGTTGAAGATGCTTTGAAAACTCAGCGAGCAGGCAGAGGCCTGTCATTTCCTTT
GTCTTCTGTACTGTCAGACAAAAACTGTTTTCTTGCTTTTTTGGACGCAGATTTTTCCAGGTGGTTCTGCTCCATCCATT
TGTCATGGAACAAAGAGAAGGGGCTCCTGATCTTTCCTAACAAAATAGAGACTATTTATTTCAGAACAGAACAACTGGCT
GAGAGAGAAAGCAAAGCAGGCTGATGGGCTACAGATCAGAAACGACATATGCCATGTGTCTGTCTGTCAGGTGCTGAGCT
GTCTCATTTGATACGATTATCACCAGGTCCATCATTTCTCACAGGCCATGTAGTTTTTTCTTTTCTCTGAGAAAAAGTCT
CTGGTATACATATACCACATTTTCTTTCTCTGCTTCTCTGCATTTGGACACATAGGTTGGTTTGTAACTTAGCTCTGATG
TTTAGTGCCTGCAGTGAACACAGATGTGCAAGTACCTCCCTGGTATATGCACTTAGAAACGTTTGGGTAAATGCCATGCA
GCATGTCCAGGTCACGTGACACATCTAGTCTTAGTTTCTTGAGGAACCTTCATACCAATTTCAGTAGTGGCTGAACTATA
```

```
CATTCCCACCAGCACTTTATATGATTTCTCTTTTGTCCACATCTTTGTCAGTATTTTTTGCCATTTGTTTTCTTAAGGAT
AGCCATTCTGAGTGGGGTGAGATCGAACCTCAATATTGATTCATTTGCATCTTTCTGATGACTTCTGAAGTTCAATATAT
TTTTCATATGCTTATTTGCCATTTGCATCACTCTTTTTAAAATTAATTTTTACCTTATGCATATGGTGTTTTGCTTGCATG
AATATCAATGTACCATTTACAAGACTGATGTCCTTGGAGGCCGGGATAGGGTGTGGGATTCCCTGGACCTGGCGTTACAG
ATGGTTGTAAGCTGCCATGTGGGTGCAAAGACTTTAACCTGGGTCCTCTGGAAGAGCAATGAGTGCTCTTCACTGCTCCG
CCAGCTCCCATTTCTCTGTCACACTTTGAGAACTGCTCATGTCACTGGGCACTGTATTGACCAGAGTGTTTGATGCCTTG
CTGATTAGGTTTTCCTTCTTTGTATATTCTAGATGTCCTTCCCCCAACTCATTTATAGTAGCTAACAATGATTTTCTCCC
ACTCTGAATGTTATTTACTGGTTCCCTTGGAGTTGAAAAGCATTTTAATTTCATGAGTCTCTGTTGTCACATTGTTGGCA
TTATTTCCTGAGCAAGTGGAACTCTATTTGAAAAGTCCTTGCCTGAGTCAGTATCCGGAAGTTTTCCTCCAATATTTTCA
GAGTTGCAGATCTTATATGAAGTGTTTTGGTTCATTTGGAATAGATTTTTGTTCAAAGATAGGGCTATAGTTTTATTCTT
TCCCAGAGTTATTTGCTAATGGAACTATCTTAGCTCCAATAATACCTGTGACATCTGTGTTAGAAACCTGTGCTATAGCT
ATATGGAATTTTATATGAATCCTCAGCTCTGTCTGCTGATCTACATGTGTAATTTTGGGCCAGTGCCATGCTGCTTTTGT
TTCTATGGCTCTGTGGTATAGCTTGAAATCATGTATACTGATACCACCACATTATTCTTTCACTAAAAATTGGTTTGGT
TCTATGGTCTTTTGCTTTGGTTCTCTGGTCTTTTGTACTTTCATATGCATGCTAAGATTTTCCTTGCTATTTCTTTTACA
TTTATTTCAAAATGTTTTGTTTTGCTTTTGAAACAGTTGTGCTGTGATTATTTTGTGGGGGTGTATATGTGATGTGTGTG
TATGTGTGTTAATTTTATACCCTTTCATTTTTCTGAAACCGTTCATCAGATCTAACTTTGGGGGGTTAGTCTTTAGGGTC
TCTCATATATAGAATCATTTCATCTACAAATAAGGATACTTTGACTTCTTCCTTTCTGCTACCTAACATCCCAAAACTCA
TCAAAATTTGGGATGTGATAAACACAGAGAATGATAATCCATCCAGAAATGGTCTTTATCCATGAAGTGTTTCCTTGAAA
ACACTCTTTATGTTTTAAAAAAACAAAACAAACAAACAAACAAACAAACAACAACAAATCCAAGCAGAGTTCAATGTTGT
CAGTAGCCCAGGTAGCAGACTGACATGGGGTTGAGTACAGGAGTCCCAATCCCAGGCTCCACTGATAATCTCTAAACTAA
AAAGTCCTTCAGTTTTCCAGGGTCCCCATCTTTGTGTTGGATGTAGATGGGGGAAATGACAAGAATTGGATCTGCAGTCT
TTAACACGCATCCACTTAATATATTTGATGAGATGACTTCCTGGAAACCTGGACCCAGGGCCTTATTGATGTCTTGGGCA
CCAATCTTCCACTGCATACTTTAGTCATGCAAGTGGCTCATTGGTTTGTTTTCACCATGAAGGAGATTGAGGGAACGGTA
TTGGTTTGGTCCAGATTCCCTGGAGCCATGGTTCCGAGAGGCCTCATTACAAATCAATGAAATGCCCAACGGTTTGATT
TCAGGTGATCCTGCCTGACCTAGCTGTCCTGAGAATCGCAGTCTACGATGACAACAACAAGCTAATTGGCCAGAGGATCC
TTCCTTTGGATGGTCTCCAAGCAGGCTACCGACACATCTCCCTGAGAAACGAGGGAAACAAACCATTATCACTGCCAACA
ATTTTCTGCAATATTGTTCTTAAAACATACGTGCCTGATGGATTTGGAGGTAAGACAAGCATTTAGACATGGAAGACAGT
GTACAAGAGGTCCTGCATTATGAGACTGTGAATCGTTTTGCCCATTCCTGAAACTAACAAAAATGGCCGTGTGTTTTTCT
CCCTGTGCTGAGAACCTTGGTGGTTTTGAGTAAAGAAAGCAGTATATCCGTAGGTATTATTTCAAAATACTGTAATTTTT
GCTTTTGGGCAACTGACCTTCAATTTTAATAATGTGATTTTAGGTTCAAACATAAGCCAGGAGAGTTTCAATCAGGATGG
ATAGGAATTATTCCTTTCTTTGTACCCTTTGCTTGCCATTTTAAAAGGAAGCAATTAGAGGCTGTAACATACAGTAACAG
ACATAGGAGCCCATCAGAGCAGCTTGGGCAGTGCCATGGGGCTGGACAGGTAGACGTCTGTCAGGTGTGTTTGAGGCCG
CAGTCCATGTCACCAGCAGTGTGGGCACATCGATAGACTGAAGCCAGGAAGAAATTATTCTGTTTGAGTTTTAAAGCAGG
GGACATAAAGGCTGCGGTGATGGCTCAGTGGGTAAAAGTGCTTGCTGCGCATAAGAACCAGAGGTCAGATCCCAGCATCC
TTGTAATGAGCTGGGGGAGGCCCAGGTGCTCCTGGAACACTCTGGAGGGTGGAGAGCAGAATCCTTAGGGCTTGCAGGCC
ACCTTCTGAGCTCCAGACTCAGTGAGAGAACTTGCCCCACAGGTAAGAAGGTAGAGAGTGCTGGAGCAATGCACTGCACT
TCCTATGGCCTCAAGACAAATATAGAGATACCTACACACATAAACATACACCCCCCCCACACACACACAAAGAGAGTGTG
ATAGATACTTATTACATTATGGAAAGTGTCACACAGACCCATTTGGTAAAATTTGTTCAAGTTTTGCCATTGCAATGGCA
ACATTTCAAATAAAACTATAATTTCTTCCTGGGAAAAGTGCCATTTGATTTGAATTGGGAAAAACGATCATTTGCTGAGC
TCCTGCTTGGTGCCAAGCACTTACATAAAGAGACCCTCAAAATGTGGAATTGATCTGCAGGGATGTGAGCGTGTGCAGGT
TTACACCGAGAAATGTTTGTAATGTGACACGTGCCAGGGCCAAATCTTTACATCCTGCAAACCCATCTCTCATGACAGTC
CTGCAAGTAGTCTCCCCTGTGGTCGTGCCTACTGCCTTTCTGTCAGATTGTTGCGAATCTCTGTACGAGGTTCTGCAGTT
GGCATCCTAACCTCAGGTTTGATATTCAACAACAGCACGCTCGCTCAGCCCTCTGATCATCTCAGGCTCACGTGGTACCT
GGGGAAGACACTGAAGATTTAGCAGTTTTTGGAAAAGATAACACAGGGCAAATATGAGGATTGCCTTTTTTTTCTGAATTT
CTGACCTTGAGTCTCCTGACTTACAGTATGTTCATATTTGGTGTCTAGTTTACTTCCTACATCCACACCTCAGAATTTAC
CTCACAGTCCCCAAGTATGCTAGTATTCTTGATTTTGTAAGGACACGTTCAAGTGAAACTATACCATCACAGGGAGAAAG
AAAGAGAGAGAGGGGGAGGGAGGGAGGGACGAAGGAAAGAAAGAAAGAAAGAAAGAAAGAAAGAAAGAAAGAAAGAAAGA
AGAAAGAAAGAAAGATAGAAAGAAAGAAAGAGAGAGAAAGAAAGAAAGGAAGAAAGAAAGAAAGGGAGAGAGAGAAA
GTAAGTTACTGTAAAGATCATTCGAAGTTAACTTGTAAAGAAACTTCATGAGACTTCACTGGGTCATTCTGTAAGTACAT
TCAATGTCAATTAGTCCACAATCGACTGGGTGGCTAGAATGATTATCAGAGAAGGGTGCCTGCCTTGCAGCAAGTGAGGGT
CACTTGGAGGTCCTGAGTTCAGAGTGAATTGCTCTACATCTTTCCCAGCCCTCCCTCACTCCCTTCCTTCTCCTCTCTTT
CTCTCGTTCTCAAGGTCCTCCCTCACACTCTTTCTCCACCCCCCCCCCACGCCCCTCCTGGTAGTACTAAAGACTAAACCA
GGCGCCCATGCCTACTAGACAAACATTGCACCACTGAGATTTTGGGTCAAGGCCGTACTAAGTTGCTTTAGCTGGCCGTA
AACGTGTGCTCTCCCATTGTATGTGCAATCACACCCTGCTTAGAGAGATTTTATTTAGTTTTAACAACACACTGAAGAAA
GATCTGTTGGTTGTAAAATCCTCACCTTAATTTACCCTGTAGTTTATGTAAAATGGCACAGGAAACAATTATATCATAAG
CAATTCATCACCCAAAGGAAAGAAGTCTTTTTAACTCCCCACTGAGAGGTTTCAGAACTCCTGTGCCTTGGCAGCACCGC
AGCTCTGTGTTCTGCATGACTTTTAAATGCATTTTCATTGTCAGTCATTGTACGTGGGTGGAAACCTTTTTATAAAGATA
AGAAAACTCTTCTTGAGCCTTTTCCAAGGTAGGGCCCTTGCTTGGACCTAGACTTAGGCTCTTACAGGTCACCTGTTCAT
TAGGTTCTGCTCTACTAAGTGACCGTCAGGTGATGAATACCCACATATGAGAAACTATATGCAATTGTTCATAGTTATCC
CTGGCCCCCAAATCACGCCCACATACATCTAGTGCTTCTTAGACATTCTGAACTCCAGCTGACCCACCATGGGAATGGTC
AACAGCCCAGATCTTACCTCTGACCCCTTCCATTCACGGGAATTCCTGTGCCCCGACCTCTGTCCTGCCGTTCCAAGCCC
```

```
TAGGCAACAGAAATAGTGGTCTTGTTGAAGCTGCTTCTCTTGCTACTAAAATCAGTTACAATGCACAGAGAAATAACAGC
AATGATGGTTTGTTTCTCAGTTCATTTTCTTGTGCTGAAACCAAACACCCAAATTGGCTAACTTATAAAGTAAAGATGT
ATTTTTAGTTTTGTAAAGATTTATTTTATTTTATGTGTAAGGGTTTTGCCTGCATGTGCACACGTGTGCCTGGTCAGAAG
AAGTCATCAGACCACCTGGAACGGATAGATGGCCATGATCCACTATGCGGGCTCTGGGAGCTACACGTGGGTCCTCAGTA
AGAGCAACAAGTGCTCTTAACCCTTGAGCCCAAGAAAAGGAATTTTGTTTCTTTGACTCAGTTCTGAAAGCTTAGCTGGT
CCGGTTCCTGCTGAGTGTTAGTGCTGCTTTGTGCATTTGTTCAGCGGAATACTGGTATAAGGGGCATGATCTGGTGGGGT
TTTTGTTTGGGTTTGTTTTAACTTTCATACAGTGTGTTTTGATGAGATTCACTGACCTTGCCCCAACTCTTCCCAGACCC
ACACCACTTCCCTACCCATTCAACTCTCTGTCATCTTTTTATTAAAATGCTTCAAAACCAGTTTATAATGTCCAAATATT
CTTAGATGTGTGAGCTTTTCCTGGGATATGTCGGTTTACCAGAGGCTACCCTTTTAGAGAGAACTATTTCACTCTCTCCC
AATAGCTAACAACTGTACATTGCTCCATGGCTAGGGGTGGGATTGTATGCCCAACTCTCATCTCCTTGCTGGGATTTAGT
CTGTCACAAGCTTGCACAGGGTCTGTACAAGTAAGTTCATGTGTGCAGCTCCCTGCTGTGTCCATATGTTTCCTTTAGC
CATCCACCATCTCTGACTCTTAAACTCTTTCTGTACTCCCATCTGTAATGATCCTGAGCTGTGGGTGGGTGATGTGGTAT
ATATGTTCCCTTTAGAGATGAGCATTTTCTGAGTGGATTTAAGTCTTGCTCCATGAGATGAAACCCATAACTGGCACCAC
CTATGGCTATATATATCCTAGGCCTCAGGAGGGAACCTACTATTAATTATTGCAGATGAACGGACATAATATTAAATCAA
CTTTTAGTGACTTACCATTATATGCATAGATGAGTGCATTTTTCAACCCTAACCTGAAAAGCTTCAATTTGCAACAAATG
ATTAACTCAGTGACCCCCCAAACTGGCCAGTGTGCAGAAACTAAAAGAGAACTGTTTATACAACCCAGATGGAAAATATT
TCTGGGCTAAAGAGTAATAGTAGACCATCTTAAGTATTTAAAACTAAATATTTACTGAAAAAATAGTAACATTCCTTATT
TCTTTTTTAAAAAGATATGTCTTGCAGTGAATCAAGAAGCCTTGGTTTACATCTTCCAGTGTCCCAGTCCTCAATATAAT
GATAAAACGGTCCTCTCTTTTCTCTCCACAAAGCAGAACTCCTGGGTTGAATGCTAAGCACCTCCCTCATTTATCAAACC
TGAGAGACTCCATCTGTGGGCACATCTGACCTGCTAGTGGTGTTTACTCACCACACAGAATTATCACATCAGCCATCAAT
GTTGGGGACAGGACAGCAAATAGTTCTGGTGCCAAAAACAATGTCTCAATGTTATATTTCCCACCTCCATTGCTGAGGGT
TTTTCCTCTCTGTGTATGTTGTCTTTATTACCAAGAGGTGAAAGAAAGGAGATTTGTCTAATATATATATTAGACACATA
TATGTGTGTGTGTGTGTCTGTGTCTGTGTGTATGTGTATGTGTGTGTTTCTGTGTCTGTGTATATGTGTATGTGTGTG
TGTGTGTGTGTGTGTGTGTGTATGTGTATGTGTATGTGTGTGTGTGTGTCTGTATGTGTATGTATATATAAAAC
ATTAGCATTAAGTTAAATGTCACCCTAAGAAGTTTGCTTTTTAGGTCCTTGTTTCTTGGGCAGGGCCCTTCTTCATCCTG
TGGCTCCTCTGTTGCTTTATGTATATTAAGATAAAGTGACTTTATCTTAATTTTTGTTTGGTTGCCTTTCTGCCTTATGA
TTAAAGATGCATCCAGGAAGGTACAGGGGTGTTTCTCATTCTCTCCACCCAGATCTTCAACTACCTCTAGCATAGGCAGA
TGTTTCATACAGCTGATACTTCCAGAGGGAGAATATCCTCAATTAAGATGCCATTTCTGGAGGGTCATGCCTGGATTTCC
TGATGCAGATTCTTTTTCTTTCAAATATTTGTTGAAACACACATAATACATGCCATCCAGGGGCAGTGGGGAGGGTCGGA
TTGCTGGTGTTTGAACCAGGGCAGTGTGCATATGATGCCCACTCATAAGGCATCTTCTGCACTCTGCAAAAGAAGGGAGC
AGTGTGCCTTATGGAAAGTACACCAGCAGGGCTTGCGAAGTAGCTTAGCAGGGCAGTGAAGGTCCTGTGTTCAGCACTCG
GCACCACAAGCAACGCAGACACTGAAAGTGGCTGGCTTCAGCACACTGGTCAGGCCCTTTCTTACACATATCACTAAGAT
ACCCTGCCAACGCAGCGAGCCGTGTGGGAAGAGATTGGCTTTTCTTGGTTTTGTTTAGCCCTGATTCCCCTGTACGGGGC
ATAGAGCCTTCACAGGACCAAGGGCCTCTCCATAGGAAAGGGAATAACATTTGAAATGTAAATAAAGAAAATATCTCATA
AAAAAAAAGAAAAGAAAAAATGTCACAAAAAATGGTTATTTAAGCTAAATATTTTCCTAAATGAAAGCTAAGTCTTTGTA
CACTTTATCCAAACTTTATTCCAAAAAAAGAAAAAAAAATTGAGTTGCATTTCGTGATCTGTTCATTTAAACATAACCT
TGATAGTTTGTTGATTGTTTTTCAGATATTGTGGATGCTTTATCCGATCCAAAGAAATTTCTTTCAATCACAGAGAAGAG
AGCAGACCAAATGAGAGCAATGGGCATTGAAACTGTAAGTGAAGCCGTGGGCTAACGGCGGTCCAGTGGGGGGAGTTGGC
TTCCAGAGAGTGACTGCCAAAGAAAGAGAGAGGTGATGTGGGAGTTTGCAGAGGGTTTTCAAAACCAAGCTTATCCATT
CAATAGAAGTTGGAAGGAGCTACAAAAATATAGCACATTTTATACAATGTGCCACTATCAGAGAGAGCTTCTTAGTCAC
CAGTGAGATATCTGCAGTTGTCTCGGTCCATTATGAATGAAGTTGGAACTATCCATTAGAAATTACAACAAGGATTTCTTT
TTATAATGCTGAAATGCCAAATGACTGAGTTCAGTGAGTTAATGAAAATGACCGTTATTACCAGGTACTCATTTCTTTGT
CCTTGGATCCCAACACTCCCTCTCTTCATCTGTTTCTCTATTGTCAATGTCCCTTTGCTGGACTCTTTGAGTTGGAACAC
AATCAGCCATCCAGTCAGCCATCCCAGTAGCTAGAAAATGCCGTGCTTATAAAGGAGTTGGGTTGTCTTTCCTTGAATTTGAG
ATCATTTTTCGTCCAGCACAAGCAAGCTATATAGTCTCACTCCCCTTAGGTAAGAGCTTGCTTGTTTTGTTTAATTTGAG
ACACAGGCACAGTTTGTAGCTTCGCTGACCTGGAACTCACTATGTGCACAAATCTGGGCTTGAATTCATAAAGATCCGCC
TGCCTCTGACTACTGAGTGCTGGGATTAAAGGAGTGCCCCATCAAGCCCAACTTCAGGCAAAGATCTTTAAAGGCAGGAA
AGTCTGGGAGGCTGAGGGATTTCTCATTAAAGTAAAAATGCTTCTAGGGCCAGGTATTTTAATCATCGGGAATATTTTTT
TGTTCCAGAGTGACATAGCAGATGTGCCCAGTGACACTTCCAAAAATGACAAGAAAGGCAAGGCCAACCCAGCCAAAGCG
AACGTGACCCCTCAGAGCAGCTCTGAGCTCAGACCAACCACCACAGCCGCCCTGGGCTCTGGCCAGGAAGCCAAGAAAGG
TGAGGTGTGCAGTTCAACTCCTTGTCAGCTTTGTCATTTAGCCTCGTTGTGACTGCCTTGACGAAGGCATTTAGAATC
GTCATTTCTGCCCACTTTCATTGTGATGCTAGATGAAAAAAAAAAAAAAAAAAAAAAAAAAAAAAACCGAGAGACTTACTGGAC
AAGTTGCTTGATTTCCTCCTCTGAGAGTTGTTTAATGGGAAATCAACGATTGTGTTTTATGGGCTGGAGGCCCATCACCT
AACCAGCGTGTTATGCCTAGTGCAACAGTGTTTACTTTGACATTCTTCATGGCAGGGCTGAAGGAGCCCTTGCTGCTACA
GTGCCAATAGCTAAGTCACCCACCTCTGTCTTCCAGACTCCAGCAGGCAAGGGGCACATGAAGCATCAGAGTCACAAATG
CCGTGGGTAATTTCTCCATACATAGAGAAAAGGCTTTACTGTTTATTTTTAAAACCCGTAGATGGGCATTTGATGGCCAG
TTTCAAGTGGTTTTCTGTGAGTGAAACAACAGCTCATAAGAAATGCCATAATACAGAAACACGTGACCCTGTGCTGCTAT
TGGACAGCCTCTACATAAGGAGACATCCATTTTGACTATTTAAAGTAGTCTTCAATTCAAGAGACCAGGCATCCATTTTG
ACTGATCGGTACCCATTTCCATATTGACAGTTAAATATCTAAAATACCCCTTGACCATAAATAATTTACTACACACAGTC
TGTTAATTTTCATAGTAATTTCTTTTTCTCCTAAGGGTTTCTTAGCATGTATATTTCCTATTTACAAGTTAATTTAATGC
```

```
CAGTGGTACAAGGAGATTAAGCCACTCTTACATATACATGTATCCAGTGCTAACAATTCAGGGGAAGAAGAGACAGTCTT
TCCCAACCAAAGCAGCCTGGTAATTTAGCAGGAAACTAAGTGTGCACTGAACTTCATGGAGCAAATTAGAAAATGAAGCT
TTACAAAAGTAGCCCAATCTGTACTGGCCCTGCAGCTGTTTGTTCTTCTCAGGAAGGAACGTGGGGTGAGGTAATGCCCC
AAGCACAGAAGGAACATGGGGTGAGGTAATGCCCCAAGCACAGACACTTCCTTTCCAGCGCTGGTCAGTCCTAGCACTGC
TACTCCTACAGAGATAGACATTGGAAAATGCAGGAGGTAAAGTTCTTGTCTGACCCGCACGTCCATAGTGAATGTAGTAA
TTGCATTGTAAAGTAGCTCCTAAAACAGCACACACAACAGGCCTCCAAAGGCTCAGAGAGTCCGTAGCTCGAAATGGGTC
TGTACTTGGGTTTGTTGGTGAGTTAGTTTCCTTTGTTCCCCAACAAGACTACATCTGAACCCACCAAGCAGCTGTCAGTT
GAACACCAGTGATTTCCCCCAATCCACCCCAAAGGTAGGGCGTACATGCTATTTCTTCAGGAGGCTTAGAAGCCTACAAG
AAGCCATTACCATACTAGGTTCTACCAATAATTAATAATAATTAATAATAATGTAACAACAATAATAATAAACCTGCATT
CCTTTGGGGATGTACATACACAGGGGGGAATAAGGACAGGCTCCAGTGTAATAGGCAGCCCAGGTAGGTGCAATGAGGAC
TGACCAGTTAGAACAGAAGAGGACCCTCAGCAAGGTCCAGAGAGGACTCAGAGATAAGATCCTAACTGAGCTGGACCTGG
AAACATAGGTCTGGCTCAACCAGAAGGGACCAGTACGGAGACCAAGGGAAGGAGGACACCTGAAGAGAAGTGGGTAGTAG
AAAGTGGAAGCAGTTCACTTGCCCAGGTCTGCAAAGAACTTGGGCCCACCCTGAGGAGGCAGTGAAGGACAACACCGATA
GTATTGACATCAGGAGTCGTTACAAGAAGAGATGCTACAAGCCGCCAGTGGCCCTGGTACACAGGACTGTATTACAGCAC
ACACCGTATTACGATCAACCAGAGGAAGAGGTCAATATAGACAGACACATCCTTCATCATTACCTTGTACCTGGAGCCAT
GCTGGGAAAGCTCTGTTGGCCTCCTTTCTTAGGAAATACGTGGCTAATAAATGCCTGAGCAGAGAGGGCAGTCTGGGGTA
AGCTGTGGTCACCATAAGAAAAGAACGGAGGAAGAAGAATGTAGTTATTTGGATATGGAGCTTAGAAAAAGACAAAAAGT
GCAGACCGACTTCAAGAGACTTCGGGGTCACACTCTACAAGTGGACAGCAAGAGAAAGAGAGGAGCTGAGAGGGGATGAT
CGTGATGAGAAAATGGCTGGCTCCCACAGCACTGTGACCAGCGAGGAGGGGGAAGGGCAGAGGGCCGAGAGGCTAGGAGC
TCACAGGCAGAATGAATGACAGGAAAGAATGAAAATGAAGACTTTGTTTTGAGAAGGAGTCGCAGATTTAGAGAAGTCAT
GGCTATAGTGTTTCTGTGCAACCAGGGATACCCACTTGTTGATGCCAGAAGCCTACCCTTCAGACATGATGGCATAAGGC
GTAAAACACATGTTCTAATAGCGTGTCCATAGCACATAATGAGGAGAGACGGATGGAAAGTGTGAGGGTGCAAAGTGACA
TGGCCCTCGGGAAGCTCAGATGTGGAGACAGGAAGTGAGCGATTCGAACAGCGCCCCCTAGCTGTGCAGCCCTCGAAATG
ACTCCAACCACCGCTGCATTGCTGATTTAAGAGAGGATTAAGCATCCTCGGGTCCTAGCTAGTTTCTAGGTTAGAGTTTG
AGAAAGGAAAGTTAGTTGTTCTATGGAGCTGTACAGATAATAGCAAAAGCTTTATATAGTAAAACTGAAATTAGCCAGAA
ACTTTGGAACTTAAGATGTAACCTCTGACCTGGTTTCCTACCAGGCAAGGCAGCCGAGAAGCACAGTCCAACATGGTAGC
CACATGCAGGACTGTAAAGGAGGTAACATTAAGCTCTGTTCCTTGCACACCCACGTTTCAGATGCTCGGGAGCTACGTGT
CTCTGGATACCTCACACATAAGCTGTCTCTATTCCTGTCAGATGTTCTATGGACCACACTGCTCTAAGCACATAGAGCGG
AACCAATTCTTGTTCTACAAAATGGGACATTTTCCTTCCTACTTAGCAAATTAAATATTTTGCACTTAATTTGTATATTT
TTCCAATAGTGCAAAATGTAATTGAGGGAAGGTACCGTTGTCTTCAGGATCCTGCTTATAAGAATTTGGTTAAGGNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNTACAGCATGAGGTCAGGAGGGGGCTGTGACACCAGATACAAGAGATCAGACAATAGC
CACACACACCCTGCCCATCCTACCCTGAAATACCATGGATCCCATCACTGATTCCCCCCCACCCCCCATCAAGATTTCC
TTCTACATCCACTGGTCATGCCAATCTTAAAAGCAATCAAACCTACAAGCAAGAGCATGGGTCTCCATACCTATCCTCCT
TAAATACATCATACCTGCTTTGGGGATTCCAGTAGAAGTCAGTTATCTTTGAATACACAATCGTTCTGTTTGCCTACAAT
GAAAATCTGTAATCAAACAGAACCATCTGAGGATTCTTCTTCGGCAATTGTGTTTACCGTGACTACTCTGAGTCCAGCCT
TTACTCAGCTAGCAAACAGGCTGAGTGTCTTACTCAAGGTTTTCATGACAGAACCCCTTAAATAGGTAGTCTGATTTCAT
TTTTCCTGTGTTTAGGACATTGCCACACTCAATAGCACATTGCTTAGATGCTAAGGTTAAATCTACCCCAAAGAAGCTTA
CCATGTTTTGCTTCTTGGTGATGGATGACTAAGATAGGAGTCGAGCTGGAAGTTAGTTGACTGATACCACGTACCCAAAT
GCAAAGATGAAAATAACTGCGGCAGGTCCTAGGTGCCTGGCCGCTCGATGGGTCCACTTAGCCCATGCCCCAGTGCTAAG
AAGGCTGGCTGATTGAGAGTTATAGCTACTCCTCCTCAATTGCCCTGAGACCGTGGGTAAAAAGCCCATGCAAGCTGTTT
TGGAAGTATCAGGTATACCTTAGAAAGTTTATATTGTCTGTTATTTACTCTAATTTTAATATACATAATGATATTTTTGT
GACTTTTTTTAAAGGTATTGAACTTATCCCTCAAGTGAGGATAGAAGATTTAAAGCAAATGAAGGTAAAATGTCTTGACT
ACTTTTTAGATGTCCGAGTACTATGAACATTGATGAAACAAGGGTTTTTTTTTTTTTTCATCACAAAGTACAATCATTA
TGATTACCTCTGGCAAAAGGATAGATGGAAAGAAGGGAAGAGAAAGACAGAGAGAGGGAGGGGGCGGAGAGAGGGAGAGA
GAGGAAGGGAAGGAGGGAGCAAGGGTGGGAGACAGGGAGGGAGGGAGAGAGACAGACAGACAGACAGACAGACAGACAGA
CAGACAGAATGAATCTCATTTCGGATCAGATCCTCTTTTTCTCCCCTGCAGGCTTACTTGAAGCATTTAAAGAAACAACA
GAAGGAGTTAAACTCTTTAAAGAAGAAACATGCAAAGGTAGTGTGGTTTTGCATACGTGGCTGTGCATTTTGTTGGTTCT
TTTGTATTTTACTTTTAGCTCTATGATGAAGACCTACCACTTCATTTTGTGTTGTGTACAATGAACCATTCATCACCCAG
AAGACTAGTTAGAAGCTAATGCTAGCAGCAAGTGTGACCCCCGTTATCTCACTGGTAACTGCTGGTGCCTTGTTCTCTG
GTTGCTGTGCAGTAGTTTATAAAAGCAGTGTATGTGGGGAGATGCTAGCAGGGGCCATCTCTCACAGTCCTTGTCAATAT
AACTAGAAACCTATTTCTGAACTTTCTGTACTTTTATTATTTATCGCTAAGAAGCCTGTCAGACAGGGATCAGAAGAAAT
CTTAAGAGGAAGATGCAAATTTAAGAGAACAACAAGAAGTGGCCCTGAGGTTTCCCAGATAAGTTGAGAAGGTTCTCAGT
TTTCAGTTTCTTTCGATTTTCAGGCTTTTTTCTGATCTTCTCTTGCAGAGGAGGGATGGAGGCTTATAGAACTGAGTTAG
GGGGATACAGAACTGGGGTGGCAGAATGCTGAACTGGGGCAGTGAGGGGGTACACAACTGAGGTGTGTGTGGGGGGTACA
CAACTGTGGTGGGATTGGGGATGCAGAACTGAACTATGTGTGTGGGATACAGGACTGAGGTGTGTGTAGGATACAGGACT
```

```
GAGGTGTGTGTGTGGGGGATACAGGACTGAGGTGTGTGTGTGTGGGACACAGGACTGAGGTATGTGTGTGGGATACAGGACTGA
GGTGTGTGTGGGGGACACAGGACTGAGGTGTGTGTGTGGGATACAGGACTGAGGTGTGTGTGGGATATAGGACTGAGGTG
TGTGTGTGGGACACAGGACTGAGGTGTGTGTGTGGGACACAGGACTGAGGTGTGTGTGTGGGACACAGGACTGAGGTGTG
TGTGTGTGATACAGGACTGAGGTGTGTGTGTGGGATACAGGACTGAGGTGTGTGTGTGGGATACAGGACTGAGGTGTGTG
GGGGGGATACAGGACTGAGGTGTGTGTGGGGGGACACAGGACTGAGGTGTGTGTGTGGGACACAGGACTGAGGTGTGTGTG
GGGGACACAGGACTGAGGTGTGTGTGTGGGACACAGGACTGAGGTGTGTGTGTGGGACACAGGACTGAGGTGTGTGTGTG
GGATACAGGACTGAGGTGTGTGTGTGGGACACAGGACTGAGGTGTGTGTGTGGGACACAGGACTGAGGTGTGTGTGGGGG
ATACAGGACTGAGGTGTGTGTGTGGGATACATGACTGAGGTGTGTGTGTGGGACACAGGACTGAGGTGTGTGTGGGGATAC
AGGACTGAGGTGTGTGTGTGGGACACAGGACTGAGGTGTGTGTGTGGGACACAGGACTGAGGTGTGTGGGGGGGGATACAG
GACTGAGGTGTGTGTGGGACACAGGACTGAGGTGTGTGTGTGGGATACAGGACTGAGGTGTGTGTGTGGGACACAGGACT
GAGGTGTGTGTGTGGGGGACACAGGACTGAGGTGTGTGTGTGTGTGATACAGGACTGAGGTGTGTGTGTGGGATACAGGACT
GAGGTGTGTGTGTGGGATACAGGACTGAGGTGTGTGTGTGGGATACAGGACTGAGGTGTGTGTGTGGGACACAGGACTGA
GGTGTGTGTGTGGGATACAGGACTGAGGTGTGTGTGTGGGATACAGGACTGAGGTGTGTGTGTGGGATACAGGACTGAGG
TGTGTGTGTGGGACACAGGACTGAGGTGTGTGTGTGGGATACAGGACTGAGGTGTGTGTGTGGGATACATGACTGAGGTG
TGTGTGGGGAACACAGGACTGAGGTGTGTGTGGGGAACACAGGACTGATGTGGGGTTGGGTATGCAGAACTGAGGTGTGT
GTGTGATGCTGAACTGCTTTTCCCTCTTGAGAAGCCTAACTACTGCACCCACTGTCAGGATCCTGTGAGTCACAGAGAGA
CAGAGACCCAATTTGTTTGTCTGAAAGGACTTTGAAACTCTTTCATGTAAACATTGCTTGCCCACTTCTTTGTACTCTGC
CCAGGCACCATGCAGGTCTGTGCTTCCTGCTGGGAGTGAGGAATAAGCATACCAATGTGCCAATGGCATGGCAGACAGGC
TTGTCCTTTCTCTAATGGTTTGTTCTCAGTCTGTCTTAGTCAGGGTTTCTATTCCTGCACAAACATCATGACCAAGAAGC
AAATTGGGGAGGAAAGGGTTTATTCGGCTTACACTTCCATACTGCTGTTCATCACCAAGGAAGTCAGGACTGGAACTCAA
GCAGGTCAGGAAGCAGGAGCTGATGCAGAGGCCATGGAGGGATGTTCTTTCCTGACTTGCTTCCTCTGGCTTGCTCAACC
TGCTCTCTTATAGAACCAAGACTACCAGCCCAGAGATGGTCCCACCCACAAGGGGCCTTTCCTTCTTGATCACTAATTGA
GAAAATGCCTTACAGTTGGATCTCATGGAGGCATTTCCTCAACTGAAACTCCTTTCTCTGTGATAACTCCAGCTGTGTCA
AGTTGACACACAAAACCAGCCAGTACACAGTCCAAACCTTACCTGGACCCATGATGTTCCCTGTGCTGTGGAGTTGATAG
TTGAGCATGTTTACTGACAGGTCACTTCCACAACTCTTCCATACTCTCCTGAGCACATGTTGTACTTATTCTATATTGC
CTGGCAAAATACCATGACCTACACAACTTATGAAAGAAATCATTGCATGGGGTCTCACAGGTTCACAGGGTAAAAGTCCA
TGACCATTGTGGTAGGGAAGAAGCTGAGAGCTTACAAGTTTAGACTACGAGAGAGACAGAGACACAGAGAGAAACAGAGA
GACAGACAGACAGACAGGGAAAGACAGAGAGAAAGACACACAGAGAGACAGACAGAGAGAGACTCTCTCACTGGGAATGG
CCTATGCTTTTGAAACCTCACAGCCCACACCCAGGAACACACATCCAGGAAGGGCACACCACCCAATCCTTCCCTCTCTT
CTTGGCAAGGGGTAAAGAATTGTTAAAGATGTTCCATTTAAAGATGAGTGCGTAGTCTCTCTTCAATACTTTGACCAATT
ATGAGTCCCTGTACTGAGGGCTGCCCACTAATAAAAGGAGCTTTTCCCACCAAGATTGGGAGCAGACCAGGTCTGTGGGT
GTAAACATAAATATATAGAAGGCAACATGACAAAGCGACCATTTAATAAAATAACAATAGCAGGTTCTGTGCTGGGGCTT
ATGATCTCCTAAGCCCTGGTCTTGTGACTAGAATTACCATACCAGCATGAAATTCCTTCTTGTGAAGCAGGTCTCAGATC
CCATCAGAAAGTAGTTACCAACCCTGTAACAGCCATGCCGCTATTGCACGGGTGGGTACACAGCGTCTGTCTTTCTTCAG
GATTTAATTTTTAGCATTGAATTGTTGGGCTAGAGCCACAGGGCTTCACAGAGATGCATCTTGTGCCAGCCCAGACTGAG
TTGCTATCTCCAAGCAAGGTGTGAAAATAGCTTTTTATTTTTTTTTCCCCTCACATACTATATCCTGTGTAGTAGTAGCCGGCCCGC
GGCCTACATGAACCAGGTATTCCTGGAAGGTAGGCTGGGGCTGAAAGAGAGACTAGGTGGCAAGAAAAGAATGATGAGGC
CAAGACAAATTTTTCTCTGATCAAGGCCCACGGAGTTTACTAAGAGANNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNCCCCCCACCCCCGCGCCGCTGCCCCCGCCCCCTGCCAGTCCATCCCTGGTGCTTTGTCGCCA
TGCTGTCAGCACTTCGTAGCCAGGCTGTTGCTTATTCAGGAATGTCTCAGGAAGACCGGTTCAGCAGGTAGCAGAATCTC
AGGGCAGTTGTCACTTCAAAAGAAGCCAGCCAAGTTGGAAAGCTGCACCGCAGGTGGCCCCACCTCAGTGGCAACAAGGT
CTGCACCAGCCCGCTTCAGGCTGGGGGGAGGCTACAATTCTGATTTTTTATTTCCTTCCCTCCTCTCCTTCCAGTTCCTAC
CTCTCCTCCAATTCAGATTCACTCCCTTTCTGTCTCTCATTAGGAAACACACAGGCTTCTAAGGGATAATAACAAAAATA
ATATAATAACATAAAACAAAACTAACACATTGGAATGAGACATAACAAACAAACAGAAGGGAAAGAGCCTAAGAGAAGGA
CAGGAAATAGACATAGATGCAGAGACCCACTTGTCACACACTCAGGGATACCACCACAACACTAAACTGACTGCTATACG
TACCCAAAGGAACAATGCTGGCCTGTGACTACTGCCCAGTCTCTGTGAGTCATGTGCGTCAATCATGTTGCTTTAGAGGG
CCTTGTTTCCTTGGTGACCTCCATCCCTGCTGGTTCTTATGCTCTTCCCACCTCTGCTCCTGCAGGGTTCCTTGAGCCTC
AAGAGGATGGAGAAAACCTATTGAGGGCAGAGTTTTCCAAGAACTCACTCTACATATTACCTGGATCTGGGTCTCTGTAT
TTGTTCCCATCTGCTGCAGGAGAAAATGTCTCTCATGCTGGCTGAGCAAGCCACTGGTCTATGACTATAGCAGAATGTCA
TTTGGAGTCATTTTATCACTGTGTTCTTTTATAGAACAGTAATGATTGGTTTTACTCTCGATCCCTGGGCTATCTGGTCT
CAGCTCACTCAAGCAGTGTTGGGTATGGGTTCTGTCTTGTGGAGTGGTCTTAACTCAAATCAGACATTGGCTGGTTGGTT
GCTCCCCCAAGCTTTGTGTGTCTCCATCGCTCTAGCATATCTTGCAGGCAGGACACCATTGTAGATCAAAGAATTTGTAGCT
GCTTTTGTATTTGCATCCCTCCTTTGGTAGTGTTTAAGAGTTCCTTCCTCTCCCAAAGCCCATAGCACATAGGAGTGAGG
CTCTCTGTGGTCATAATGGAATATTACTCTGCAGTTAAAACCAAAACAACAAAATCATGAAGTTCACAGATAAATGAGTG
GAGCTAGAAAAAAAAAAAAAAACATCCTGGGGACATAACTGGACCCGAAGAAGACAAATATGGTATTTATCCACTTATATG
TGAATGTTAGCTGTAAAGTCCTCAATAAGTAGGCTGTAATCCATAGCCACAGGGGTTAGGTGTGGAGTACAGGACTAGTA
GGGGACAGATAGATCTCCCTAGGAAAGGGAAATAGACTAATTATGGACGGATGGTGTGGGGACTGGAATGGGAGGGTCAA
GTTGGAAGGGAAAGGGAGGAGGAAGAGAAGGGAGGGGATGTGAGGGGAGAGACAGCTAAAATTAAGGGCCATGTATGGAA
TAGTATGGAAACCTAAGACAGTAGAACCTTAATAAAATATATACATATATGAAGACAAACTAAAGAAAATCGACAAATGT
```

```
TAGGGAGACAGAGCCCCAACTTGTATATCTGTTGCCACAAGAACCAGGAATAGATTGAACTCCGATCAAGTCATTGTCCA
AAGGGGTCCCATGAAAATCCCCAAACAAACACGGCTGTTTTCAGAACTGTAGCTTACTCTCCACAAACTTACAGAAAGGC
CCTATTACCGCAGACAGCACCTACGTCACCCATTGAACACGGCAACGCCTTTTAAACAAGCATCTCTCCTAAGCCCGTCT
CCAAACATGAAAGTTAGTTCCAAGGTGAAGCAGTGAATGACAGCAGGAATGTGAGTGTGAGAATCTGCTATTCATAAAAG
TAAGCCCTGAAAAAAGGTCTCTGATACGGTGGTAGGACTGAGACAAAAGACAAGGGTAATGACCGCTGTGGCCCTTGGCT
GCTTATGGTCAATTGCTTTTGGCTCTCGGAATGCAGCTGGTAATTAGGATTCTAAATGTCTCTCCGCTTCCAGAGTCACT
TTTTCAATCTGAAGTGGAAAGTAGGGTAATGGTTCTTGGGAGCCCATTAGACCTGACACACAAGAAAGTAAAGAAACCAG
CAATCCTTTCCAACAGTGAGCTCAAGGTCCAAATCTAGAGTCACTCTCATTAAGAGACAAGCATGATTTTCAGTTGTCTG
TGCTGGTCACGTCCTTAAAAGAAAATGTTTTAAATCCCAGGTTAGAGGAGTACTACCAGTAAGCAATTAGAAGAATTAGA
ATCCAGATGCCGTCAGCCAGTGAGAAGGAAGTAGCTAAGGGGAGTTGGTCTTGCCTAGACTTGACAATAGTCGGGAAAGT
AAGAGCACTTATTCCTGGCCAAGATCAAGAGACTCAGTGTCTACACCTTGAAAGTATGAGACCCTCGCCTGTTCTCCCGA
GGAAAACCATAATCCAAGTTCCTTATGGGAATTCCCTGCTTTCCACACAACCCTCCAGCTCTGTGTCTTCTGAAAACAAC
CTGAGGAGGGTGGGAAGGTAGATGTAGGTCTAGGCTTGTGGCACCAACATCAAATGAGTCAAATAAAGCACCGCAAAAAC
CTGCACATCCATGGCATGCCACGGGGTGGCGCCCACGGGCGGGGAGTGTCTTGGATCATTCGCTTGTACTACATGCAGC
TTGGCTTTTAATAATGTTAAAAATCCCACCGCACCACAATATAATAATCAGGCAAAATGGTCTACAATTTCGGTAAAGCT
ACTAGTGAAGAATGTGTGCTCGGCTCTGCAAGCTGTGTAACTGATAACAAGAAGGCTGCTTCTGAAATTGGCAGTTGTAA
TTCACACGGAGGCATCAAGAACATTACCTCTGTTAGGAGACCGGACGGGTGATGGGACTGCCGGCTCCATATTCTACTTC
TCCACATCCTCCCAGTTTAGAAAAAGCCCAAATGGCTAATCACGATTTCGGCAGTTCATGTGGGATGCCACTTGAAAGGG
GGCCTCCCTTTTGAAGTGGTGTGTTCATTAGGCTGAGGCGGGGACTCCCTTTTGGGGGGAAGGGGGCTCAAGGCAGGGTT
TCTCTGTGTAGCCCTGGCTGTCCTGGAACTCACTCTGTAGACCAGGCTGGCTCGATCTCAGAAATCTGCCTGCTTCCCAA
GTGCTGGGATTAAAGGTGAGTGCCACCACTGCCCAGCTGGGGGCTCCCCTTTTAGGATGCTCTTGAACGCTAATGTTTG
GTGAGGACCAGAAGCCTGCTTGGTTCTCAGACTGCATTATTTAGTGGCTTTCCAAACAAACACTAGGGGAAGCTGACAAG
AACCAGGCTGGCCTGGCAGGGTAAAGGTTCTTAGCGTCCATTACAGTCCAGCTCATAACCGTAAACTCTCCTTGCTCAGG
AGCACAGTACCATGCAGAAGTTACACTGCACACAAGTTGACAAAATCGTGGCCCAGTATGACAAAGAGAAGTCGACTCAT
GAGAAAATCCTAGAGAAGGCGATGAAGAAGAAGGGGTAAGACTGATGATTGACTTGCGATGCATCCCCCCTGTAGGAGTG
TGACTCAGCTGCCTTCATGGACCACAAGGGACTCAGTGTTACTGCGACTTCATCATTAAAGACTACCCCTAGGAAAAAAA
TGTGATCCCTTCATTTCAGTCCCTGTGTCATCACAGCGGGGTGTGGCATCAGTCAGCTTAGTTGTGGTTTAAGGATGGTC
TCAATAGAGTTCTCAGAACCTTTAGTTCATGGCAATCTGTACTGTGTTTTGGGGTTTTTGTTTGTTTGTTTGGTTAGTTT
GGTTTGGTTTTTGTTGTTGTTGTTTGTGATTAAGAAGTTAGCAAAAAAAAAGAATTTAATTACTTTCACCCTAAGAACT
GGTTGTAACGCCTTTGTTTTATAAATAATTTTTTAAAGTTGCAATAATGTAGTAGTTCTCCTCAGTTCCACTCTCTAGTA
TTCTAATTAGCTGCAACCCACCACACTCCAAGACGAATTTCTATCATGGTATATGACTTGTTAATTGGTCTGTGTTATCA
TTGGCTACCATTACACTTCGCTGTGTATAATGGACACAGTAATCACTGCCCTAGGCCTGTGAGTGGGACAAATATACTAC
AGCTCCAAATACCTGGGGTTTTAGATATCCACAGGGGGAAGAGGGTGGCTTGGAACTTATGCTGTTGTGCATGTAACTTG
GCCTTTCATAATGTTAAACCCCATCTTCCCTCAGTAAAATAATCAGGCAAAAGAGTTGACATTGCTGAGATACCGCTGGT
GGAACAGCAGTACATTGGCTGGTTTACCCGACCCCCTTTCTTTCTGGGGTTCCCTCTGAAGTTGCACCCTGCACTTACTG
TCTGCACAGTCTTCTCACTCTGTCATAGAGTCTGACTCTCCCTGTCTTTCATGGGCTTCAGTTTTGCGGCCACTCAGCCA
CCTGTACAATGCCTCTAATGTGACTTCTAGAGTAAGTAGAGAGAGGTTACAGATCTGTGAGTGCCACACCCTAAGGGGTA
GCCTCCCCCCAGTGCACAGGGTGCCACTGCAGGTGATGATGTGTTGGAAATGGCAGTGCTGACCTGGCCAGCGTCCATC
TGCATGTCCCGTTCAGAGCTTTCCCCCCTTTAGAATAAGTAGCTTAGGGAAATACCAGACATATTCTATTTCTTTCAGAC
ATCATTTGATTTGCTCTCCATCAGTTGATTGTATGGTTTTGTCTTTGTTTTCGTTTTTCAATGTTTTGAGCGCCCCCC
CCCCCACTAGGGTCTTCTAGCCTTCCATGGTGTGAGCAAAAGGAGGAGATAGACATCTTTCTAGTTTTCTTCTTTCTTT
CTCTTTAGTGTTCAATCCAGATACCACCTAGAAAACTAAGGATCTGGATTCAGATGGATTGTTTTTGTTACAATTTCTGG
TCCCTAAATTCTAATTTCATGACATGGACCAATGTCCTTGGCTTGAAGCATCAGTGGCAGATAATGATGTGCCCACACT
CAACCGGGGGCTCCTTTTGGAAAGCACCTTCCTTTTTATTACAGCTACATTTTCTAGAAAAACACAAACAAGGCAGTATA
TGGAAGCGATCCACCTTCTGTTTCTCCCATGGCTGTTCCATGTTTCACTGAAGCAGAGCCATGTCCCCATGCTTTAAAAG
CATTTGGATTTTTTTTTTTAAGTGAGTCAGGACAAGCTCAGCAGCCCATCTCCTGTGCGCAGGGACATACTGGGAGGGA
ATGTAGTGAGTGTGAGAGTGTCTATGTTGTTAGTTCTGTGACACTGGCCACACTACTCCTGGAATCACTCCGTCGTGGTG
GAGGAGGCAGGCAGAGCAGCAGGTCACTCTCCTTGGCAGAAGCATGCGGAGGATGCAGACACTCATCACTGAGGACCAGG
AAAGATAGGCACCAGCAGCAGTTCACACCGTCACAGGCCTGCTCTAATGGCCTGCTTCCTCCAGCAAGATCCTGCTCCTA
AAGCCCCGCAGCCCCCAGAACAAGAGCCTCTAAAACCACATCACACGCAAACCGTAAGAGCTGATGTTTAGAGGCTCTGA
CCACATCAGCAGCGTTACCTTTTTTTTTTTTTTAAAGAAACTTTCCCATTTTTAATGTAATTTTTAAAACAAAATGTTGA
AAGGGAGGTATAGACCATAATACCATGTTTTTAAGGTACAAGGTTTTGATGGATTTTTCAGCTCTTTCCAAGAGTAAACT
TATTTTGTTTACCTTTCACTTACCAACAAATGTCCTGTGTTTTTCCTGGATTGCTATATTGACATTTTCTTGGTTTTTT
CTATTTTCAAAAAAATCATTTTTTGTTATTTTGTAGAATTCTGTACATGCATCCAATGAAATACAATAATATCTACCTTC
TATTTCCTCCTTCTGAATTCCCCCATACACATCTTCCTCCCAACTTTATATCTTACTTTTCATTTAAAAAAGCAAACAAA
CAAACAAACAAAAAACACTAAGTCCAGTTAGTGCAGTTTCTATGTGCATGGGTGTGGAGCCTCTTTCTATATTTTTAAAT
CAGTGTCTTGTAAGTTTTCAGACTCTGTACTACATAACAACTCTTCAGAGAGGGGATACTAGGGATATATTGTCCCTGGGA
CATTAGAAAATACCCATTTAATCTTATTTTTATTCTCAAGCATAAGTGGTATGAAGGTGATACTTAGGACTGGTTGTTAA
AGATGAGACAATGACCCATGGAGCCGTCCTCATGAGCAGGAGTTGAAGTCCTACAATGTGTAAAGCCCTTTGTCACGCGAG
GAACAGGGGACCCAGGAAGCCTGCCTGGGGGTTTCTTTCTAGGTCCATGCTGCCGAATGCAGATCTGCCTCCCGACTCTG
CAGAGAGAATTTTCCCAGTACTTGAAAACAAGACCTAAAAAAAAACACATTGAAAGTTTCTTCGAAGCCAGTTACCAGAT
```

```
GTTCTCATCTGCACAGCCAGCTCCAAGGAGAACGTCCTCTTATTGCCTAGACTTTGTTACCTGGTTGCTGAAATTCATGT
AAAGGGGGAGTTTGGTTATATGCCTTTGATCCTCTGCAGTGGACTGGCTAGGCTGGCTGGGGTGAATGCACCTTGCATTT
CTGATACTTTCAACTTACAGTGTGGGCTCATCAGACTCCATCCCATCCTCAGCGAAGGAGCGCCTGCAGCCTGCAGCCGT
GTCCCCTGTTCTTCATACCCGACTGAAGGGAGCGATGGAAGCGAGACAGGGAGTTTGCACTGAAGTGCTCTTGCCTAACT
GTCTCTCATGAGTTTTCCATTCAGTGTGTTTCAGGTGACAGGAGAAGGTGAATGGGGAAGGCAGAAAAGAAAAGCCAGG
GTTGGAGAAATCAGAATATATGTTTCTGCAAAGGCAGACAATAAATAAGGCATATGCAAAAGGGAATAATTATGCAGCCG
TGACTTCTTAATACTACAGGGATCCTTAGCTATGCCGGCTGCTTAAGGGCGCTGAATAAAAACCATCGTAGCATTTATTG
TGAGCGTTCCTGGAAGGAAGCCAGAGGAACATTTCGGCATGCTGGAGAAGAAATGCACTGAGTGCTGGCCATTGAGGAGC
AGAGGGTGTGATGGGAGTTTGCAGCAGAGAGCGTTTCTCTCAGGGGGTGCTTCTTTAATAGAACAAAGATCGTGAGCCGG
GCTAGCGTAAGCCAGAGCATACGTGCTGAGGACAGAGCCTAGGTCCTGTCCTGGTTTCAGCAATAATGTGGAGAAGAAAA
TACCAACACAGAAATAAGCAGTAATGGATGCTGGGCAGTTGCCTGATATATATTTTCTACTCCTAGGAAGCTAAGAAGCC
AGCCTCCCTCCCTTGTCAAAGTAGTGCCAATAAATGCTATTTTCTTCCTCATTGGCATGTTTAAAAGACAAAAATCTGGC
TTTATGACCAACATGAAAGAATTAGACATTCATTTCATGTTGGCTGTTTGCATCCCTAATAATATTCTATGAAATAAATA
AAAAACGGCTTGAAGAAAAGAGGAACCAAAATGGAAACTCTGACAACAGACCCTACACAAGGCAATAATTGCTAGGTCT
GAAGTATCTCTTTGTGCTGTTGTTGAATTGAGCAATCACGCTACAGATAATATTTTCTAAGGTAAAAATCTATTGCTGGA
AATGGATGCTGCCCAATGAGCCATGGCATTCCGGATATATTTGATGAATATATTGTCTTCACCAGAAAGTGTTGTTACAG
AGGCCCAAGGGAACTGAAAGATATCTAATTTCTGGCATGCATTTTCAATTCTATATTTTCAGGGGAAGTAATTGTCTTGA
AATAAAAAAAGAAACAGAAATTAAAATTCAGACCCTGACAACGGATCACAAATCTAAGGTAAGAAAACCCCGGTTTTTGC
ATGGTGACTTGTAAAGGATACTTTATAGTTTATTTTAGAAGGGGCAACTCTGGATTGATCTGGATTGATCTGATCAGAAA
CAATAGGAGAGAGTTGGACTGCTAGGACAGGGTCGGAGGTTTGTGTTTGCCTCAGGTTTACTGTCTTTGAGAAGAGCCAC
TTCACGGAAGTCTTTTTGTCTTGTGTGTAGAAAAAAAACTAATGACATTTATGTTGTTTGATGACCCTGATGCATGAAGT
AAAACGGTGAGCCGGTGAGCCAAGAGGCAAGGAAACAGTGAATGTACAGAACATACCTTCCTAGGGAGGTAACATCAAAC
ATGACACAGGAACAGCCAGACTGCGGGCGGAGTACACACTTACATCTTACTGCATCTTTGTGAGGCCTGGATGTCATGCT
GTTTAAAATGGATTTCTCCTTTGTGTTTTATCTTTGGTGTGGCAGGAACCGTACACCCAGGGATATGAGAATTAGTGCTT
CATGCACTATTGCCTTGTGTTTTATATACTTTCCTCCTATAACACGGAAACCTTGGCGATGGTCTCAGTTTGCTTTGGCT
GTGTGCTACGCTGCCAAAACTTAATGCCTTTAAACAACTCTCATTGATTAGCTCATTATTCTATGGGCCACACATCTGAG
CCATATTCAGATGGGTGATGACCCTGACTTTGTGGGGGCACACTCAAGCATCTACAGTTAGCTGGGGTTGCTATCCTCAC
TCTGCAAAATGCTTGTGTTTCTTGAATGACCTCCACCTCCAGCAGGACAGTCCGGTCTCATTTAAAGGGCAACCCCCATA
GAATAGGGGAAAGACCTCTGCTTTGGACATATTTGCCACTATCTCATTGGCTAGAGCAAGTCAGAGAGTCAGATTTCAGG
AGCTGGAGAAGCAGCCTCTAGCTGTTGACTAGAGAACTGCAAAGCCACAACTTTGCACAAAGTGTGGTCATGAGAAGGAG
AGGTCTTCCTACCCTAGTGTCTATTGAGGGTGATGGAGTAAGCCAGAGCCTAGGCATAAGAGAGCGCTCTGCTCTCTGTT
CAGACCTGTGGACTGACAGAAATGGAGCTGGAAAAGCATGAGATGATAAAGAGAAATATGCTGAAACTGACCAAGATCAA
GACACACCAGAGTATAAGACAGGCAGTGAAATACCGGGCCGACGTAAGCTACTTAACAGCCAGAGTGGTGATTTAGCTCG
GTACAGTAGTGACTAGTGTGTATTATTTTTGTGTGGCTTGTGAATGTCCATTGATTCACAATATAAATTGTGATCTGGCC
TGCATACATAGTCATGCTTTCAGCGCATACCTGACTCAAGAGATTCCAGATGACCCCATCCCATTTTTTTCTGAAACAGG
TCAAAGAGATTGTGGCCCAGCACACAAAGGAGTGGTCAGAAATGATCAACACTCACAGTGCGGAGGAGCAGGAAATCCGG
GATCTGCACCTGAGCCAGCAGTGTGAGCTGCTGAGAAAGCTGCTCATCAATGCTCATGAGCAGCAGACCCAGCAGCTGAA
ACTCTCCCATGACAGGTGGGAGGGCCAGATGCCATACTGCTATAGATCTTTGTGTCTTCAAATGACTGGTGCGTGTGTGT
GTGTGTGTGTGTGTGTGTGTGTGTGTGTGTGTGTACACAAAACAAGCAAAAATCAACTTGACGTATTTCGCATACTTA
GTGTGAGCAAGCTGATTTGTAAGAAGTAGCCTCTGCCAACAGTGACTTATAGGTGTTAAGAAGGTACTACAATCAAGTAA
AGTTAGCTAAGATTGCAAGTTGCTATAATCCATATTAAAATATGGTCTTCCTCTTCTAAGTGTTTTGAGGATCAACAGTA
AGTTATGGCTGGAAGTAGAAAGGACCTGAATCCTCTAGGAATATGAGGCATGGGAGAAAGCCTGGCCGGTGGAGCTCCT
AGGATGACTCTCATCTCACAGTGGCAATGCATTAGAGAGCCTCCTCCTTTCTGTATGTTGTTAGGCATATTGTGTTAGAA
ATACTGCCGGGACTGAGCGGACTCCTCCCTGCGTAGAAGAGCATGTCTGCTGACTGTATCTTTCCATCCCAGTTTGCTAG
CAGCGTGCTTTTTCCCTATTAAGGATCTGCTGTATGCCACAGTTATGGTATGATGCTGGTTTACACAGGACAGGGCAAAA
CAAACACTTTTTTACCAATAGAGAAAATGATCATTTTAAAATGGCAACTGCATAAGTTATATGGTAGTATATGAAAGT
TCCCAGCCTTGCTTTAGGTGAGACATCCTGCTGTGAGAAAATAGAGTGGTGCCTTAGGTCCCAGTGCTTGTGTGAGGCAA
GTGAACAGTGTCTGGCATGATCTGGTTCCGTCAAGAAATGTTCTGTACTCTGTGAAGTCCTAAGTACTTCGTTTACATAT
TCAGTGTAAGAGCTCGTATTCCTTTTTCGTCAAGACTGCTTTTGATTGGATATGTGCTATACAACGAGTCTAGGCAGCAT
CCCATCCTGCCTTTGGGCAATGCTGCAATGTCAGCTTGGAGGAACCCCCTGCCATTATTCCAAAATGTGTTAGAGTCTGG
GGCACTTCCTGGCCATCACTTTAGTAACTGCATGAAGAAAAATCATGAGTCTCAAACTTAGATATTTTTTGAAAAAAAAA
TTTTAAATGTAATTTTTCATTGCTTAGGAAGTCCTTTTTGAAAACCACAAACCCAGCTGCTAATTTGTCTTTCTAAGTAA
GTCTGTGCTAAGAGCAACTCTCGGAGTGAGAGTGGGACTCGGGGTTTTTTTCCCCTGCCTGTTAGATGGTGTCTGTTTTG
CTTGCTAATTCGTATTGCTTTTACCAAATGCTTGTCATTCATACAGTGGGCATTCGTAAGTGTTCTTGCTTGAAGGAAAC
AAAGGAAGGGTAGGGGGCAGATCGATCACTTTACAAAGAAATCATGGGAAACTCTGAGAGTGGTTGCTTTGGGTCCAGCG
TTTTTCTCAGTGTCCTGCTGATGAGATGTAAGGTGGCTGGCTAAAGCTTCATTTTAGCATTGCCCATCTCTCTTGATGTA
GAGTAAACCACTGGACTTAGGTAACCAGCCTCCTCGCCTGTACATTGCAGGCAAAGACATAAGGGGATGACTATAAGTTA
AAATTATTTGTAATTGCATCATTTAAATATTCCAAACTATGTGCCTTTATTGTCTACAAGAGTGAATCAAAGATAGACAT
GTCCATTATACATACCTGTGCACTTACACAATCATTAGTCACCTGAAATACATACAGGTCTACTTCCTGTACTTACTTGA
AACTACATAGCTCCACTCGTGTCTATGGGTTGTTTGTCAGCAGGAAGGTCACTTGTGAAAGTTATAGCATCCTGTCGCTC
CCATCCTGTCACTGCTGGGTCCCAGACAGTTCATGGTGAAGCTGGACACCTGTGCTTTTTTTGTTGGTTGGTTTTGTTTT
```

```
TGAGCAAATACAGCATCTGATCACCGACCATGATGCATGCTTCCACTAGATGGCACTACCTTCCTCAGCCATCTCCATTC
ACCTGAGCCTGTCAGTTGTGTAAAGAATGTGAACCTAGTCCTACGGTTATGTATTGTATTGAGGTTTTTATCTTTCGTGT
TATAAATCATAAAGGCACCCTTTAGGAGTGTGGCACAAAGAGCCATACATTCGCATTGCTATACCCTCTTAGCATATATC
AGAACTCCATCTGACCGCTCACTGACCTTCAACGGTAGTCTCTCTGGATTCTAACTCTCCTCTGTATGCCTCTACATTTA
TTTACTTTTATTTCTATTTCACAGGGAAAGCAAGGAGATGAGAGCCCATCAGGCTAAGATTTCTATGGAAAATAGCAAGG
CCATCAGTCAAGATAAATCTATCAAGAACAAGGCAGAACGGGAAAGGTAAGTGTGAGGGTGCTTGCTGGGACTGTCTAGT
TTACTGGGAACCAGTGTCAGCAACATTTGGTTATGTGAACTCGGCATAGATCTGAGATATTAATACTGTGGTGTTCGAAG
AGTCCTGTTAGTACAGTTTCTATGGAAGTCAATTATTACTGCAATAATAGAGTAGTTTCTGATGTTTTCCTGATAACTGT
CACTGGTAAAATGTATTTGCATTCACACAACATATGTACAAACCTGACAATAATTGATATAATTTCATATAATTCTTTAG
CATTTCTCCTCCCATCGGTCTGCAAACTTCTCTCCTGCTCCAGTGTCAGCGCGTCCTGGGTGAGATGGGTCTGAATATCA
GGAAGGCTTCCTCCCACCTTGATTTGCTGCCTTTGTCAATAGGAACTATCTGTAAAGTTTCCACATAGATTTTTTTCTCG
TTTGTTCTCAGTTAGGTGGGCTTTCTAGAAGAAACTGAGGCAGGAATCCAGGGAAGACAAGATGGGGTGGAACCAGACCT
GCTGTGGTAAGGCTATGGTTGGGCTTTACCACAGCTGGTGAAGGGAACCTGAGACAGAGCAGCAGCCATGCTGCTGACAA
GGGGGCATTGCCAGTGACCACAGATAATAGTCACCCATCATGATCCATCTCTAAAGACCCAAAGCCATCTATTCTACAAA
TAGAAGACCAGGCAGAGCTGAGGAGAACAATGGCTATCTGGGCTCCTGCTCACAAAGAAATGGTATCTAATAACGTCCAG
GTCCACTGAAAGACTGGTTTCTGCTCTACAAAACACAGAATTCAGATTAGCTCTTGGGGACCAAAGAGTAAAGTGAGTTT
AGAATGTCAGACTGATGCATCACTCCATGAAACATGGGCTGCTAGAAGACTCTGCAGCCCAAAGATGGGATCTTCTGGGC
TTCATCTCCAGATTCCTGGCCTTCTGAGATGCTCAGTGGATACTGACGTTTTAGGCAGGACTGCCTCGGTTGTATTTATT
CTTTTCAGTTCTTAAATGGTGCTAGGTAATAATAAATCAAGATATGGCAAAATATAAAGCATCCAACTAGTTAATACATC
TTTTATGGTAAAGTTTTATAAAATGCCTTTTAAACATAATAGCTCCACCCCTCAATGGGAAGTAGCATTCCACAGTAATC
TATTTATTATTATATGTATTTATTTACTTTATGTGAGTGTTTGTATTTGTGATGTGGGGGAGGGGAGAGCATACAGGAGC
TGCCACAATCACACAGGGTGCTCATATGTAGGTCAGATGACAGCTTGCAGGAGTTGGTGCTCTCCTGTGGGACCCAGGGA
TCAAACTCAGGTTGTCAGGTTGGTTTTAAGTTCCTTTATCCCTTGAGCCATCTCACTAGTTCGCCACAGTGATTTTGACC
TGGAGACAAATTGCTTTTTTATTTTAACTGTTTGGCCAAGATATTTCTCAATCATTGTGGAGTGCCATCTGTGCCTCCCA
GTAAGAAGGTTCAGGGGAGGACATTAGTCATCTGTTCAAGCAATTATTTGTAGGCTGGTATTGTAGCATCTCTTTGGTGC
TTGTTCACTTTTCAAAGGTGCCAATTTCTGGACATTTTCCCAGATAAGGATCTTCGTTTGAGAGTCTTGTATACATGGAA
ATACATCTTCTACCCGGAAATCTCAAATATCCAAAACATAATAAATACAAATTATTTTTGTTTGATTTTCTTAGACTCTG
GTGTTAACTTTGAACTCCTTTAGCCTGTACAAAGTGAGATATAACAGTCAGCATGAAGACCAGACTGCTAAGTAGTCATT
GATTGTGTTTTATCAAATGTGTTACAGAGTGGACCAGGTTCAGCTGAGTACAAGCTTTCCTTTTCCTTTTCCTTATTTTT
TTTTTTTTACATGCAATGCTTCCCTACATTTTTAAGGCTACCCTGGAACTCATTAAGCTCCTCCTGTCTCAGAATATTGA
GCTCTGGGATGGGGGGACCGGCCACTCATCTCAGCTTTCAAATCACCCTAAACTTTAAAGTACCACTAATACCATCAGCT
AGGCTTTTGAAGTAGCCAGGTGTTGGGGGTGCACGCCTTTAATGCCAGAATTCGGGAGGCAGAGGCAGGAGGATCTCTGG
GTTTGAGACCAGCCTGGTCTACAGTGTGAGTTCCAACACATCCAGGGCTACACACAAAAAATATTATCTTTATTTTGATT
TGTATGTATTGACTGAAATTTTGTATCCATCATTACATGGTTCTAAAGCATCAACAAATTGCTGTTTCTATAATAATAAT
AATAATAATAATAATAATAATAATAGTAATAGTAATAATAATAATAACAAATTGCTGTTTCTATAATAATAATAATAATA
ACAACAACAACAACAGCCTCTCTGCCAAAAAGCCACTTACCTATACAGTAGCATATTATATATAGTGTGGCTTTGGCTTT
ATCACACTGCTGAGGACTAAGGCTAAAGACTTATTATAGGACTTAGCAAGACCAATAGAAACTAAAAAACTATTCTTTTA
AACAGAAATTATACGGTATTTATATATAAAATTAAAATGCAGACACTGTTTATCATGGACAGAAATTATAGCGACTTTTA
CATGAAAATCATGGGTATTTCTTAAAAGATGTGAAAATTAATGATCTGAACGCTCAAAGAATGGAGAAACCAAAGTGAAC
ATCAAGATCCCAGGTCAAAATAAATAACAGAATGCACAAAGTGCTAAAGTAGGAGAGAAAAAAAAAAAGCAAGAGAAAGC
ATGCAGAAAAGTAATAGCTTATCTAAAAATGAAAAACAAACAAACAAAAAAAACCCCTAGTGACTCACTCAGTAGATTGT
CAGCAAGAATGATCAAGAAGAGAAAAGAAACATGAATTAGTGATATTTGGGATGAAAATGAAAATAAACAGAGGCAAAT
CACTTTCAAAGCAGCTGTAATGCTAATGGTAAATCTGAAACTATGCATAGAATGGAGAATTGCTTCGGAAAATACACACA
TCCCTGTGGACAGCCCTGCATCCATGTGAATGCAGTCAGCACTAATTGGATTTGGGGGCTATTAATGGTGATAATAAGAA
CAAAAGAAGACATGAACATGGAGGGCAGGCCTTGGGGGAAGCTGGAGGAAGGCAGTAGGAAATTTAGAATGTTACGTGTA
TACATTTTCAAAGAAGAAAAAGAAAATACTAGTCTAAAAGAGAAAACGAAAACTGCACATGCCGTGAGCACAAACGGAGA
AGCCTAAGCGGACACCATTACTGCAGGAGTTCACCCAGTTCTGTTGTGTAGTCATGAAAAACCTCCGAACTAACATAGGG
GAGGAAGCCGTGGGGTGAAGATAAAGCCATAGGACGAAGATGAAGTTGTCTATATCCTACCAGCAAGCAAGTCTTGGAGC
TTGAGTTTCTGAAGGAAATATACCCAAAATATCCTAAGTCCTGAAAACACACAAAATGATTTTAGCGTGCCGACTGATTT
TAACAGTGCTTTGTGGGATCCCATTAGAAATGCTGATGCCCAGGCCCTCTCAGCCGCACTGAGTCAGGCTCTTAGGTTTG
GAGCACAGCAGTCTGTCTGTGACACAGGAAAATTTGAGAACACTTAGTTGTATTTCCTGTGGATACATCCATTTTGGTTA
AAAATATTAAGATATGGCTAAGTAAGCCTCTGTCAGCCTCGGGACTTCCTCCAAGGAGAGGAAGAAGGGAAAATAGAATG
TGAAGGAGTACAGAGGAAGTTTAAATTTTATGTATAAAATTCTGTCTTTAAAAATAGGAGAGCAGGGACTGGACAGATGG
CTCTGTGGTTAAGGGTGCCTACTGTTCTTGCAGAGAACCTCAGTTTACTTCCCAGCGCCCACTTGGCAGGTCACAACCGC
CTGTCACTGTTGGAGAAAGTATTTAATCCCAGCCCCCGCCGTTGCTATTTAAGTTCCTGGATGAAAGACACACACACAGC
TTTATATTTACAATAAGCCTGCAACAACACAAGAGCTGGGCAGACATCTACCCTCCATACTGTTAGGATCTACTTTCCTA
TCAATAACCCCGAGTACTACTTGCTATGTTTCATCTGGGCTGCTCTTAACTCCAATTGGCCAGCCCACAAGCTCAGGTTC
TCTTGACTCCTAAGCCATCCTGGCTTCTCCTTCCTCCTCCCGAACTCCTCTTCTTCCCTTTCTGGTTCTCCTCAGACCCC
AAGCCCTGGGAAACCTAAACCCCACCTATGTCTCTTCTACCCAGCTATTGGCTGTTGGCATCTTTACTTATCAATCAGAA
ATGACTTGGTCACAGGGACTACATACAGACTTCAGGACTTGGGGGCCCGCACTTAGCAGTACAAAATCTCAATCTATAAC
TCCAGTACCAGAGGCTTCAACACCTTCTGTCTTCTTGGGGCTCCTGCACATGCGTGGTGGAAATAAACTCACACAGGTAA
```

```
ACACACATTCACAAGAACGGGAAATTATAAAGAAACATAAGTATTGGTTAATCTGAGTGATGTGACAAGGTGGTTTTATA
TTTTCCTATCTATTTTGTGTGTTTGAAAAAAATGATACAATAAAAATGAAAGGGAAAGGATGCGACAGTCTTCTCCGGGA
AGCGAGAGAAGACAGTTTGTGGTGGGTGAAAGTGACAGTCCCACAGGCTGGTGGACTGACAGTTCTTTGTCTCCATTGCT
TTTAGGCGAGTCAGGGAGTTGAACAGCAGCAACACTAAGAAGTTCCTAGAAGAAAGAAAGAGAGTGAGTATCCCATGTTT
TTCTCGACACTCCTCTTTGGAAATGTAACTTGTGCTTGCATCTCAGCCTTGCAAGCTCATTTACCTAATTTCATTTTTT
ACCTGTACATTAAGTATTCCAGCTTTGCCCCTCCCTGAAAGCCAAAGCCATCTCTTACTAAAATCATTATGGAAATTAAC
TGACATGGCTTTGTGGAGGTGACAGTGCCACCTTCCAGTCATGCATAAGTGCTCAGCCATTTTCTAAGACTTGGGAAGGA
AAGATCTGCCCAATATACACTACGAGTTTAAACCCTTAACAGTTTTCAAGCTCTTTTTTCATTCCTTTTTGAAAGTGTG
ATCACCCCCGGCTAAATTATAAACCCAGTAAGCTATCCACCATTTATAATTTTAAGACAACATGACATTGATCATATTTT
CTTTCCAGCTGGCGATGAAGCAGTCAAAAGAAATGGATCAGTTGAAAAAAGTCCAGCTGGAGCACCTAGAATTCCTAGAG
AAACAGAACGAGCAGGTATTTCATCCCGTGCAAATAACAGACATAGGCGGCCCATCAGCTTCAGGGGTGCAGTGCCATGG
CCCACGGGAACCCTGTGGTCAGGAAATACAGTGGAAGGTAGAGAGACTTTCTGGGTCTTACTGGACTTCCTCCTCTTCGT
CCAAAAAGAACAGAAGTGCCTTTTGTGGAGGGGGAGAGGCAAGAGGCTCTGGGGGATTAAGAGTGTAAAGTTGTCCTTTG
AGTCTATGACAGGATGGGTTCTAAGTGTGATGGCCCTAAGCAAGCAAGTTATGAACCACTTCTTCATTTCTGTTTTTAGC
TTTATAGCTTACAGGCTGCACGTGTTAGTGTATTAAAAGTAAAAGAGAAGAACCGTCTATTTTCAGTGCCAATCTAAAAG
GTGAACTTGTAAATTCTTCCCAGGGCTTTATGGTGGGATCTAAATCCATGCCCCCCACAACTCAACAACCCAGTGAAGAG
TGTTCAGCAGCTTTATGGTGGGATCTAAATTCATGTCCCCCACAACTCAACAACCCAGTGAAGAGCATCTTCTCGGGGTT
TGTTTGTTTCAAAAAAAAAAACAAAAAAAAAAAAAACCAAACCACTCAATAGAGAAGAACAGTTGTACACAGCAGTAGTTA
TCCAAGCTCCACTTAGTCAGTTTCGACACCTGGACAAGTCTGTTTCCACGAAGCATGCCAAGTGTAGTATAGCAGGCTCC
ATCCCATGTGACCCACTGCCCAGCGGCCTTCCTTTGAACCCTAGCTCCCCAGGCGGGAACTCCTAGCAAGGCGCACATCT
TTTCTGTCTCACTTGGTCCCGTTTCAATCTTGGTTCAAGGCATTGGACTTGGATGTTTATCTTCACATTTTAGAAACTGT
ATAGAGCTTTTGAAGAAATAATCATACTGCATTTTTCTTTTACATGTCTTCCCTTCTAAAAAGCAATCCTACCTAAGATT
TTTTTTCAGAGTTAATAACTCATCCTGAAGAGTACTTCAGAAGGAATTATACTTTGAAAGGTCCTTAAGAGAGGAACACA
AAACCACAAAGTCATTTTTTTGTGTTGTAGCTTAGGTAACAAACCTAGCTTCTTGTTTCAAGTCCTCGCTCTTGCTCTCA
CTCTCTGTTGGACAGAACCCAGTCCTCTTGTGGGCATGAATTAAGTTCAGCTACTCAGTTCTAGTCATAAATACCGCCCG
GGTCCTCTAAGAGAGGCAGTTCACATGTTTGGTCCTAGCTGTCCAGTTCATTTGACCAACTCATAAATACATCAGGTACA
CACCATACAACTTATGCTCTTTCCTGAAAGCACTTCTCAGTATGTGTCCCCAAGGTGACATGAGACTATAAGAAAGGGTT
GAGCCACTATGATCTCCAAAGCTTCCGAACTCACTGGGGGCTGGGGGAAGATGTTCAAAGAAAGCAGAGGATAAGCATCT
CTGAAAGGCTTTCGAATCTTCTTTCAAAGAAGGGCCTGATGAAAGGCAAGGCACGCTTAGGGTTTAACTATCTAAATTAA
GAAACTATTTAGAAATTACTGTGTTGCTTAGCAACAAGTTTGACTAAAGAATGTAAATGTGCTAAAAATTACTCATCACT
ACATAGCTAGAGAATACAGTTTTCAGGTGTTGGTACTTTTAAAGCATAATAAAGAAATCACTATGCGCATTATACTAATA
CATGGGTACCTTGAACTATGGAAGCCTACTTGCATTAAAATCCTGGATTACTGGTAGTTGTATTGATTGGACCATGTTGG
AAAGATCCAGGCTTTTTTGTTCTTAAAACTCACATATTACCTATTAATATGTCATAAGTACATCTACCACCCACAAAAGT
GAAATTTTGAGTCACAGAAACTTAAGAAAGCACCGAACGTGAAAAATATCCAGATGTAAAAATGTAATGATTTTCAAGGA
AAAGTCATTCCAATAGAAACGTTGTGCAAATGTAGAATTGGGGATTGAATTTCTACATTCAGCTTTAATTTGAAAATATA
ATATCTTGTTGCATAACCAATTAAACGCCCGGTTTTGGTAGTATGTATTTTTTTAACACGCAGGTCAGGAAGCACTGAGT
CCTCAGTCCCTCTGTCTGCTATAATCCTCTCCTGTGCACAGGGCAGAGACGCAGGGAATCCCCTGGCTTCCTCGTAAACA
CAAAGGCTGAAGCCATGTAACGGAGCTCCATTAAAAGCCAAGTACACATTCCTTTAGCAAAACAGGTCACCATATTCACA
CAATCCCAGACTCGTAAAGCAATCTTCCAACCCTTTGTTAAGCCCCAGGTTTCTTCCTTGTGTGTAAACCAAAGATGCTT
TAATCTACACATCTATATTATATCGCCTTTTATGGTTTCTTACTTTTTCCTATTATAGTCTTCCTAGCCATGCCCTGTCA
ACCCCAAAATAAAGCCCGGTGGATCTATGGAACTAACGAGACCACTGTCAAAGGCATTGGGCGAAACAGCTAAAGGGCTC
ACACTGTTTGGTCTTGATTCTGGATCCTGTCCTTTGTAAAGCACTCTCTAGCCTGGCTTCTTTATCAGCCAGTTGTTCG
CTTAGCTTCCCAAGGGCCAGGACATATATGTACACATATTGTAATCTTGTGACTTAGTTTTTCTCATAGTCCTCCAACCA
CCCAGTGTTCCATCTGTCTTGACATGTACCATTTCAGACATTTAATGATTCCATCCCTGTCACATTTAAGTCTTAAAAAT
TAGTATCTGTCATCAACCCTATCATTCAATAGAGTGAGGGAAATGGCTCAGCAAAGCTAAGTCTCCCTATGCACTCACTG
GAGAGTGACACACGATGTGCTAATAGGCATTGGTTGTTTGTTTTGTTATTCCTTTTTTCCTTTGTTGAGCTCAGCTTGGG
GAAAGTTGTTGCTGATGGGTGTGTACATGTTTTACTCTTAGCTTTTTTGCTTTTCTGTATATAGTTTTGCATTCATTTCT
CAAAATCACTTTGGTATTCATTTATTTTCTTTGTTTTCTATATATGACTCAATTTTGACATTACTATTTTGTACAAACA
GCTTTTGAAATCCTGTCATGCAGTGTCCCAAACACAAGGTAGGACTGAAATTTTGATGATAAGCAAGACCTTGCTTTCCT
TCCTGGCTATGTCAGTATTCTTGGTTTGATTTCCATTTATGACTGCCATCTGGTCATAGCTAACTGATATCTTAAGGGTT
TAGACTTAAAACCCTGGCTATTTCATGCTTGAATTCCTCATACTACTTAATGGGAAATGCTGTCACATTTGCTTGTGGGA
GTGTTTTATCCAGCATTTCCAATTACAAATCCAAGAACTGGGTTGTTACCATGAAGACAACCCAGACTGAGAACATTATT
TTTATATAATGTCTGTTGGTCCTTTTCGATGATGTAATGTTGTGGTCTGCGTGCAGAAATGTTATAATACAATGGCTTTT
GCCATGTTCTCTGTTAAGTCTGGTCTGCCTATGGCTAGTCACCCCTTATAAGGTGACTATATAATTGACTCTTGGAAACA
GAATGTTTTTGAGAAAGATGTCTGTTGTAATAGCCAAACATCTGCTAAGTGATCCCAGGCAATAAAAATGGATGGCCACG
CTACTTGTGTTACAACTTAGCTCTCAGGGCAATCTTTGAATGGCATTTCATTATGCATTTTATACTATGTTCAATAGAAT
CCTTAAATGCAAAGGGGCCAAAATGTAGGGAATCTTTTATTACTGTTTACTGAACACCAAGTCATTTAATTTCCAGTGCT
GAAAATGTGTCACTATCATCTCTCACGGATACTGTTTGTGTCATTATTTCATGTTTGTATATTTTCTCCATTTCTTCATT
TTCTTGTGAGTATTACTGTATGTCAGACATTGAAAATAATATGGCTAGAGGGACTCACTAGCCTCAAATACATGGATC
TAAAACTCTTTAGTGATGCTATATCTACCCGCACTAAAATGAAAACGAAAGCCATAATGTTGAAATAATAATAATAACAG
CACTGCATTATCTGTGACATAAATTGATTTTTGTTCTGGGACTTCATTTGGGAGATTTAGGTATTTCTAGGATGATCTAG
```

```
AATTGGCCAGCTCCTTCCTCTGACCTTCCATTTAGCTGGAACTTCAAAGCAAACATGAACCGTAATATCATGGCACTTCA
GAATTTTCCTTTCAAAATATATCTCTATTGATGTGTCATTCTCCATTCTTACCTGTCCTTTGTTTCAGTGGTACATCACT
GAAAAAAGGAGGAGGGTTTCTTACCCTTTCAAACGTTGCAAGCATGTGGGTTGGGCATCTAGGCTTTGGTTGGAAAGTAA
GTAAAAGTTGAAATGAATCACTTGATTGATTTTCCCATGTCCACCCCTAGAACCTGCTTCTATAGCCCCTCTCAAATAAG
CTTGTTGCTTTAGTGACTGTCTTAATTCATGATTTTAAATTCACCCCAGCTTATATCCTGCCAGTCCGTAAAAAGTAAAT
TACTTCAGAGAAGCAAGAGGCCAGAGTTTGAATAGTAACATTTCTAGCACCCTGCCTGCTCCTGTAATGTTCTGTTTGTA
GGACTCTAGCCAAAGCTGTGTAACACACATGCCCTTACCTTGCCTAGCCCCATCGGTAAACTCTGAAGTAATTACTTTTA
ATTAAACTTCAGGCTGCTTGGAAAATTAGTTGTACGAAAGGAAAGCAATGCTATCCTGAAAGTGATAGAATTATCCAAAA
AAAAGTCACGTGACACTAAATTAAACAGTTTGATTTGACATCATCTAAGTATCTGGCTCAAGTTCTTTTCATGTGGTTTT
TCCCAGAAATTTGTAGGCCAGATTTTCCCAGATTTTTGTGTACCATAAAAGTTTCTTGCAAGATCCTGTTGAAAGAACGA
TTCAGATCCTGTGGCTTTTAAGCAACCTCCAAGACTCTGCATTTCTGAAAATAACCCCTAGAACTTCTGCGGCTGCCGGT
CCGCTTGCCATTTATGTAATAAGGATGGGTTTAGCAGGAAGAGAGGCTAGGGCCAGTGGGAATCTTCGGAAGGGAATTTC
CATGCATAAGGCTGTGTTTCTGGTGTTGTAGGCGAAGGAGATGCAGCAGATGGTGAAATTGGAAGCCGAGATGGACCGCA
GACCAGCAACAGTAGTATGAAACTCCAGAGTGCAAACTGAAGCTGGAAGACCATGCAACACCAGAAGATCATGCCCAGCT
TCCAAACACAGACTCCACAGACAAAAGCTGTTTTCTTTGTGTTTCTTTATGTATAGACACGGTGTTACCTGAAACCACA
TGGACTGGGGATGACCAAACGCTCTTATATTTAACAATTTGAGTATTGAATTTCTTTGGCCAACCAAAAGTAGCTATGTA
CACATACACACACACATACACACACACACACAGAGAGAGAGAGAGAGAGAGAGAGAGAGAGAGAGAGAGAGAGAGAGAAA
TCCCCTATTCCTGACAGGCAGAGTTGAATCATGATATATGACTTAAACATGTTTGCTATGAGACAGCATCACAAGCCAGT
GGGCTTGGTGATAACAACTCTGCTTTGTGGTGCATTAGGACATGTTTGAGCTGCTGCTGCTGCAAAACAAAACAAAACAA
AAAAATTAGTGCATTAGTAACTTAATAGTAAGCGCATGCACTAGAAGAAACACCTCTGTCTCCAGTTCATTAGCAGAAGT
GGCCGTCTGCTATACAAATCACTTCGCAGAATTTTCTACACCTAAGTTACCTCATCAGTAAGTGCCATGTCTTTACCATG
CCATAAGATGCTAACCTTCCTGTAAATGTTGCAGAAATCGTTAGGACAATAGAGACACAAAACGGTGTATCTGTGCCAAG
CTCATCAATGCTCCCAGGGGGACTCGGCAGGCTCGTTTCAGAAAGTTTACAGCTGACCCTGCTTTGTAGGAATTGCTTCT
GCCGATTCCACATATTGCAATTCACTCTACACTATAAGGATCGTCAAGTGGTTATTGGCAAATGTTTGTAAATGTGACCA
TGTACAAAGTATTTATACTCCTTAATTCTCACAGTTATAGCTCAAAGTCATCTAAATATTACCACACGACAAGACTAGTG
AGCAGGAATCATAGACGTAATGTTTTGCATGATGCACAAGCACCAATTATGACTAACCCATTCAAACACAGTTAACCTAA
CACCAAATAAATACTGGTTAAGTAAATGTACGGCACAGAATATTATTTGACTATCAAGACTTTTAGCATAATGAAAAAGT
CTATATATATGTGTATATGAATTATGTGGGCATTCTTGATACTTCAAGTTCTAGTTTGAAAAATACATAACTAATTTAAT
TTTACACAAAAATATTTATGCAGATTTTCAGACTTTCATATCAGGAAATAACCTTTTTATGTCTGTTAAATATCCAAACA
ATTTGCTACCGTGTTAATCTGCATGGTCTTCTCGCCTGCTGTAGTTGCCTTGCAGGTGTCTCATAAAGTGGACCGATGGG
ATGGAGTCATGCTGCCCAAACTCAGAGGAGCATGCAGGGATCCGGTAGCCTAGCAAGAGTCAGATTCCTGATTTTAGAAT
ACAAAATGCCAACTTAATTGTTGTATATGGTTGTACAAATAGATTCTGTACCTTCCTCTGAAGAGGGCAGAGAAGATTAT
GATACCATGAAGGCTAAAAATTCAATGTACAACTTCTCTAATAAAAAATGAAAACACATGTAACATCTACCAGAGTATTTG
CTTCCACGATGCTTTCTAGAAAACCTTTGCCTCTTCATGTATGTTTTCTTCATTATTTTCAAAAAGGGGCTTTATTTCCC
ATCCCCATTGTGGTTTCCCCCAACTGACACAGAACACACAGATTCTACATACAGAAGTCATGAGCTTTTTGTCCCTCATA
GTTATGAGTTGAATAGCTCATAATAGCTCTGTACCCTGTCAAATTCCTGGCTTTCTTCTTTGTAAAACAGTAGAATCAAG
ACTGCATATATTCAGCACCAACATCCCATTTGATAAAAGGCCCTCACATTGGGATTGCCGGTTTTCAAAATAAAATTGTT
AGACTCTCTTCCTCAAAGCCAGTGCTCCTTCCAAGAGCCTCAGGCTGCTGTCATTCTGTCATCTGCCCTGAAGCTACCAC
TCACCACTTGTCTTTGCTTTTCAGAAGGCTCCTAGTTTCCCATTTTTCTGGTCATGTGATCTCTGGGTCTCCCTCCTTCT
ATACCATGGTGAATAGTTATAGTTTGCAGTTTTAGATTCTGAAACAGTTTTAATTTATCCCAGATTACTTTTTTCTTTTT
TATTGGATATTTTCTTTATTTACATTTCAAATGTTATCCCCTTTCCTGGTTTCCCTGCCTCCCAGAAACCCCCTACCCCA
TCCTCCCTCCCCCTGCTTCTATGAGGGTGTTCCTCCACCCACCCACCCACTCCCACCTCCCCGGGCTCAATTCCCCTACA
CTGGGGCATCTATTGAGCCTTCATAGGACCAAGGACCTCTCCTCTGACTGATGCCTGGCAAGGCTGTCCTCTGCTACATA
TGCGGTTGGAGCCATGTGTACTCCTTGGTTGATGGCTTAGTCCCTGGGAGTTCTGGGGGCTCTGGTTGGTTGACATTGTT
GTTCTTCCTATGGGGTTGCAGACCCCTTCAACTCCTTCAGTCCTTTCTCTAACTCCTCTATAGGGGACCTCATGCTCAGT
TCAATGGTTGACTGCGAGCATCCTCCTCTGTATTTGTCAGGCTCTGGCAGGGCCTCTCAGGAGACAGCCATATCAGGCTC
CTTTCAGCATACACTTCTTGGCATCCACAATAGTGTCTGGGTTTGGTAACTGTATATGGGATGAATCCCAGGTGGGGCA
GTCTCTGGATGGCCTTTCCTTCCGTCTCTGCTCTACACTTTATCTCCATATTTGCTCCTGCGAATATTTGTTCTCCAAG
AAGGACGGAAGCACCCACACTTTGGCCTTCCTTCTTCTTGAGCCTCATGTGGTCTGTGGAGTATTTGAGACATATTTACC
CATAAAGGATCTACTGGAACAACATTAAAGTCAAAACATATAAAATAACAGCAAAATTATGACCTTTGCTGTGTGCTAGA
GAGGTGGATCAGCATCCAAAAGTGCTTGCTGTGTTTATAGGTGACCAGGGCTTGAATTCCCAGCATCTACGTCCAGGTGG
CTTATGATCAGCTGTCATTCCAGCCCCAGGCGATCTGGTCCCCTCTTCCCTCTGTACACACCCACCCACATGTGGCAGAC
AGTCAGACAGACACATTGTTTACAAGTTTATGCGCCAAATTCTATAATTATTAAAGAAAAAAAAACCTTGATAATTAAAA
TGACTCATTTTCTTATAGCATTAGAACACTGCTGTGTTTAGGGCATGTTGCTCTAGTGCCCAGTCCTATTCAGAGCACT
GTCTCCTCTAGGAGTTCCTATGGCCTCCTTCTCTAGTTGATATCTGGCTAAGAAGGAAGCTCTTTGTTTTCTGCTAAGTG
GGAGGAGCTTCTGGTCTTCCAGACAATCTGAAGCTCTGTAGAGTGAGCCTTTTTACCTAGGTTTTTCAAGATCACTGCCA
CTGTTCTAATCGTGGCTGTTCATTTTAGCATTTTTCTGGTGCATACAGATGTACTGGATCTTGGCTGTGCCACCTTGGAT
TCCACCATGACACCACCTTTGTCTGGGTGTCTCGTATAGTTGTTCTAATACAGTGGAGCCTCACAAATCCAGGATCCTTG
CTGTCACCTGGAGCTAAGTCCCAAGAAATGCAAAGTGAGCCTGGTTATACTAAGCCACAGACTTACCAGAGTTTCCGCTG
TTATCTGGCTTCTGTATATAGGAATTTTGTTTTCAGTTTAGGACTCCGAAAACTTCACTTGATTTCTTTTTAGATATTTA
TAGATCTCACAGAATAAAGATATCCTGCCGGTCAAATTACATGCCACCCAATGATCACTACCACATCTCTTGACTTAAGG
```

GATGGTAAAATATGAAGAATGTGTACATGGGAGAATTTGTGAAACATAGTATTTCAAATGTTTTATGCACAATCTTCAAG
CCCCAAGGCTTAAAGGGGGCTCAAATTATAGGGTACAAGAGCATCCTTCAGAAAACCTACAGAACTGTCTGCACCTGGGT
CTAACATTCAGCAATGAGCTTCCTGGAAAGTAATTGAAAACAGGAAAGAAATCTATAATTGATCACTTGTTGAGAGATCT
GTTTTTCCCTCCATTCTTTCATCCAATCGCATGCCCTCAGTTATGCTTGTGAAGTGTCTTACACCCTTGTGAGGAGACCC
TCTATCTTTCCTCCTTAGAGATATCAAGCTTTACACAGACAGTGTTCACATTGTGTGTAATTAATAAAAAATGGAGCCCG
GTTCTCCATCTGTTCTCCCTATGTGTTTGAGCGATCCTAGCCACTGCTTAACCTGGGGCAATCTTGTTCTGCTTGGCCTC
ATCAGCCCCCATTCCCAGCTGCTGACACAACTACAAATTATATCTCCCTGTTCTGAGAAGCATTTTTCTCTTTCCATTAA
ACTTCTCTGCTTCTTTGACACCCAGGAGTTTGAGCATTCTTACTTGTAAGCATACATTTTTTTGCAACATACTTTTAATA
ATGGTTCCACCTCCCCTCTAGCCCACCACCCAGCAGAGATAGTGGAAAAGTTATTAGGCTATTGGGGCAGTGTACCTGTT
CATCAGTAGTTCTTTGGACGTGAGCTTGATCTTAGGCAGCAGTTCAGTCCTGTAGCAAACACCAAACACAATTCAGAAGC
TACAGACCAGTCCTCTAGGCAGCCAAGCATCAGGCAGCTTCAGGCCAGCCCTTTCAGCAAGTAGACATCAGGCATGGACC
AGCAACTCTAGTTCATTTCTGAAGAAGTCACCAGGATTGCCAATCCGCCTGAGGCGAGGCTGCAAAAGAAGCAAGCTGGT
GCAGGAACCTAAAGATCAGTTCTTGGGTGAGTTTCAATGGCGGTGTTATCACAAGTTGAACTCAACATCGCTGTGTACGG
TGAACCAATACTTGTGTGTCCTTAGTAAAGAATAATTCGGCAGAGCAAACCAAACCAATGCTGAGTTCACTTCCCACTGT
CTGTGAGGTCATATTTATACTCCTTCATCAAGTGTCCTTTCACGTGTTTGCTATATCCAAACATCCTTTCACCTGTGTCT
GCTTCGGGAAAATGTCTTTCACGTGTTTGCTTTAGCAAGACATCCACATCCTTTGACGTGTGCTCCAACTAAACATCATT
TGACGTAACTAACTTTCCAAAGAACTAGAAGTTTCCACTTTCACCTATTCAACTTCTGTTCCCTTCAAATCCCTTCCATA .
TACACAGAATAGTTTTGCCAAATGATGGTCCTAATCATATTTCCATGCCATCTGCACATAAGGAATTCATATCTACCAGC
TAAAGAATAATATCCACTTTATTGAACACAGTATTACATTTCTAGTCTAAACTTGTCTACTCACGTAACCCATCTCAGCT
TATCATTCATTGTTAACGGAAGGATAACACTCATCTTTTCTAGCCCACTACACAGTATTCTATCCTGACACAATCTCTCC
AGCCAGGCAGTGGTAGCACACACCTATGATCCCCGTACTTGGGAAGCAGAGGGAGATGGGTCTCTGAATTTGGGGTCAAC
CAGGAGTATCAAGAAGGAAGCCTCCTCAAAGTTGCCTTTGATTTTCAAAGAACAATGGGCTGTTCACCATTATTGGATGA
TTTGAAGTATTTCCAGTAAGGTGAAATTTAGGACTTATAATTGTGTAACTTCATAAGCAGGAAATATCTTCAAATACCTG
ACTGAAAAATACTAAAATAAGCTTTTTGCAGAATATGTTTTCAATTCCAAGAAAAAACAAATACTTCAAATCCAATAGT
GCCTTTTCAATGAAGTCCAGAACTGGGTAAGCCATTCAGTGTTGGGGTACAAACCTGGGACGTGCATCGATCATCAGCAA
TGCAAAAAAATAAAATAAAATAGTCACCATCATAAAGGCCACCAGGAACTTAACAGGTGAGTCTGTCAACATTAGGAGCA
TGTGCAGAGTGAAGGATAGATGTTCTTATTGTTGGTGTTGTTCAAGGATACTTCATTTTAAACCTGAAGCAGCTTTTCCT
TTGTACTCTCTTCTGCAACTCTGTATGAGGACCACATTTCTTTGAACTTTTCCGTGATACTCTTGTTTTGAAGAATCCCA
AGCACACTTTAATAGTTGCACCTTTAGTATAAATCTGAGGTTGAAGCAGCTGATAAGAATATTTATCTTTAATATTTCAT
GAAAAGCATTCTGTTCCATCAGAGTAGTGAAAACTATTGATAGGAAGGAACAGAATGTAACTTTAAACAGCAGCACCATT
ATTTCTGCACAAAAAGAAACCCGTATGCCTGAGCCAGTAAAAGAGGCAAGGTCCCTTTCACAGTCCTATCTTTTTCTGAC
TTGCATGGGAATAAGTGCTGGAGCTCAGTTCCTGAGATGCTTTATCTTGGACATATTAGACTCCGTCACAAGGAAATAAC
AATAAAAGTCAGCAAACAAGAATATATCCAAGGCTCAAACGTCTGTTTGCTAAACACTAGCTTACCTTATGTTCCAAGAA
ATTTCTTTCCCAAAGAAAGGAATCTGAATGTTCGTCAAAACACATATTATTTATTCTTAATATTAAAATAGTGGAAACCA
AGTTACATCAAAAGGGGACCACATCAACATATGTGGTTTCTTTATAATTCCAATGGCAGAAAATCTTCAGGTAGCTTAGA
ACGGGTTCCCTCGTGTGACCTGGCCTGATGGGAATGGAGTGGTAGAAGTGGTTCTCAGAATGTATGCCAGGCACCTTGGGT
CCATCCTGTGCTTCCAAGAACTTGCATAATGACATGGGAACATCTCTAGTTTCCAGGTCACCTCTGTAGATGATCAGTGT
GTGTGTGTGTACACATGTGTGCATGCATTATGTGCATGTGTATGTATGTGTGTGTATAAATGACATCACAACACATTAAA
AGGAAATCTTTCAGAATGTAGATGACTCTATGAAGCTAGACATTAAAAGACGTAGAATGATATAAACAGTGCCACTCTAC
CAATATTTTGATGTCATTTTTGCATTTAAAATTACTATTAACCTATATTATTATATTATTATGTATTAACCTATATTACT
ATATTAACCTATATTACTATGTAACCTATACTAGTTAAATATTAGTATGTGATAGGTTGAGTGCTAGTTGATTGGACTGG
TAAATATTTGAAAATTACTGTTTTAATTTCTAAGAGTAGTATTTACAGACAAATTCACACAAACAAAAGCTCATATTCCA
GGCTAGACAGATGGCTCAGTGGTTAAGAGCACTGGCTGCTCTTCCAGAGGTACTGAGTTCAATTCCTGGTAACCACATGG
TGGCTCACAAACATCTGTCAAGAGTTCTGATGCCCTCTTCTGATGTGTCTGAAGGCAGCAATTATGTTCTCATATACATA
AAATAAATAAATAATTCTCTTTTTTAAATTTTTAATATTTTTTATTACATATTTTCCTCAATTACATTTCCAATGCTATC
ACAAAAGTCCCCCATACCCTCACCCCCCCCCACTTCCCTACCCACCCATTCCTAATTTTTTGGCCCTGGCGTTCCCCTGT
ACTGGGGCATATAAAGTTTGTGTGTCCAATGGGCCTCTCTTTCCAGTGATGGCCGATTAGGCCATCTTTTGATACATGTG
CAGGTAGAGTCAAGAGCTCCAGGGCACTGGTTAGTTCATAATGTTGTTCCACCTATAGGGTTGCTGATCCCTTTAGCTCC
TTGGGTACTTTCTCTAGCTCCTCCATTGAGAGCCCTGTGATCCATCCAATAGCTGACTATGAGCATCCACTTCTGTGTTT
GCTAGGCCCCGGCATAGCCTCACAAGAGATAGCTATATCAGGGTCCTTTCAGCAAAATCTTGCTAGTGTATGCAATGGTG
TCAGCATTTGGAAGCTGATTATGGGGTGGATCCCTGGATATGGCAGTCTCTAGATGGTCCATCCTTTCGTCACAGCTCCA
AACTTTGTCTCTATAACTCCTTCCATGGGTGTGTTTGTTCCCAATTCTAAGAAGGGGCACAGTGTCCACACTTTGGTCTTC
ATTCTTCTTGAGTTTCATGCGTTTAGCAAATTGTATCTTCTATCTTGGGTATCCTAAGTTTTGAGCTAATATCCACTTAT
CAGTGAGTACATATTGTGTGAGTTCCTTTGTGATTGTGTTACCTCACTCAGGATGATGCCCTCCAGGTCCATCCATTTGA
CTAGGAATTTCATAAATTCATTCTTTTTAATAGCTGAGTAGTACTCCATTGTGTAAATGTACCACATTTTCTGTATCCAT
TTCTCTGTTGAGGGGCATCTGGGTTCTTTCCAGTTTGTGGCTATTATAAATAAGGCTGCTATGAACATAGTGGAACATGT
GTCCTTCTTACCGGTTGGGACATCTTCTGGATATATGCCCAGGAGAGGTATTGCTGGATCCTCTGGTAGTACTATGTCCA
ATTTTCTGAGGAACCGCCAGACTGATTTCCAGAGTGATTGTACAAGCTTGCAATCCCACCAACAATGGAGGAGTGTTCCT
CTTTCTCCACATCCTCGTCAGCATCTGCTGTAACCTGAATTTTTCATCTTAGCCATTCTGACTGGTGTGAGGTGGAATCT
CAGGGTTGTTTTGATTTGCATTTCCCTGATGATTAAGGATGTTGAACATTTTTTCAGGTGCTTCTCTGCCATTCGGTATT
CCTCAGGTGAGAATTCTTTGTTCAGTTCTGAGCCCCATTTTTTAATGGGGTTATTTGATTTTCTGGAGGCCACCTTCTTG

```
AGTTCTTTATATTTATTGGATATTAGTCCCCTATCTGATTTAGGATAGGTAAAGATCCTTTCCCAATCTGTTGGTGGTCT
TTTTGTCTTATTGACGGTGTCTTTTGCCTAGCAGAAGCTTTGCAGTTTCATGAGGTCCCATTTGTCAATTCTCGATCTTA
CAGCGCAAGCCATTGCTGTTCTATTCAGGAATTTTTCCCCTGTGCCCATATCTTCAAGGCTTTTCCCCACTTTCTCCTCT
ATAAGTTTCAGTGTCTCTGGTTTTATGTGAAGTTCCTTGATCCACTTAGATTTGACCTTAGTACAAGGAGATAGGAATGG
ATCAATTCACATTCTTCTACATAACAACCAGTTGTGCCAGCACCATTTGTTGAAAATGCTGTCTTTTTTCCACTGGATGG
TTTTAGCTCCCTTGTCGAAGATCAAGTAACCATAGGTGTGTGGGTTCATTTATGGGTCTTCAATTCTATTCCATTGGTCT
ACTTCTCTGTCAGTATACCAGTACCATGCAGTTTTTATCACAATTGCTCTGTAGTAAAGCTTTAGGTCAGGCATGGTGAT
TCCACCAGAGGTTCTTTTATCCTTGAGAAGAGTTTTTGCTATCCTAGGTTTTTTGTTATTCTAGATGAATTTGCAGATTG
CTCTTTCTAATTCGTTGTAGAATTGAGTTGGAATTTTGATGGGGATTGCACTGAATCTGTAGATTGCTTTTGGCAAGATA
GCCATTTTTACAATGTTGATCCTGCCAATCCATGAGCATGGGAGATCTTTCCATCTTCTGAGATCTTCTTTAATTTCTTT
CTTCAGAGACTTGAAGTTTTTATCATACAGATCTTTCACTTCCTTAGTTAGAGTCACGCCAAGATATTTGTTATTATTTG
TGACTATTGAGAAGGGTGCTGTTTCCCTAATTTCTTTCTCAGCCTGTTTATTCTTTGTGTAGAGAAAGGCCATTGACTTG
TTTGAATTAATTTTATATCCAGCTACATCACTGAAGCTGTTTATCAGGTTTAGGAGTTCTCTGGTGGAATTTCTAGGGTC
ACTTATATATACTATCATATCATCTACAAAAAGTGATATTTTGACTTCCTCTTTTCCAATTTGTATCCCCTTGATCTCCT
TTTGTTGTCTAATTGCTCTGGCTAGGACTTCAAGTACAATGTTGAATAGGTATGGAGAGAGTGGACAGCCTTGTCTAGTC
CCTGATTTTAGTGTGATTGCTTCAAGCTTCTCACCATTTACTTTGATGTTGGCTACTGGTTTGCTGTAGATTGCTTTTAT
CATGTTTAGGTATGGGCCTTGAATTCCTGATCTTTCCAAGACTTTTATCATGAATGGGTGTTGCATCTTGTCGAATGCTT
TTTCCGCATCTAACGAGATGATCATGTGGTTTTTGTTTTTGAGTTTGTTTATATAATGGATTACGTTGATGGATTTCTGT
ATATTAAACCATCCCTGCATCCCTGGAATAAAACCTACTTGGTCAGGATGGATGACTGCTTTAATGTGTTCTTGGATTCG
GTTAGCGAGAATTTTATTGAGTATTTTTGCATCTATATTCATAAGGGAAATTGGTCTGAAGTTCTCTATCTTTGTTGGAT
CTTTCTGTGGTTTAGGTATCAGAGTAATTGTGGCTTCATAGAATGAGTTGGGTAGAGTTCCTTCTACTTCTTAAAAAACA
AATTTCTGTAAGCACATGAAGGAGTCCCAAAACCAAAGATTTTTAAGAATTGTTGAGCTATAATTTGGCTTGATCAAAGG
TTATGTGAACATAAAGCTTATCCAGGTGTGCAATTAAGATTCATGTTCCTCCACAGAGCCCCCAATGGAGGAGCTAGAGA
ATGTACCCAAGGAGCTAAAGGAGTCTGCAACCCTATAGGAGGAACAACAATATGAACTAACCAGTACTCCCCAGTGCTTG
TGTCTCTAGTTGCATATGTAGCAGAGGATGGCCTAGTCAGCCGTCAATGGGAGGAGAGGCCCTTGGTCTTGCAAAGATCA
TATGCCCCAGTACAGGGGAATGCCAGGGCTAGGAAGCAGCAGTGGGTGGGTTGGGGAGCAGGGCAGGGGAGGGTATAGG
GGACTTTCAGAATAGCATTTGAAATGTAAATGAAGAAAATATCTAATTAAAAAATTCATGTTCCTCATTATCTGAACATA
GTTCAAAACTGTTTGAAATATACAATGGAAAAATAGGTATATATTTATGACACAACATAAATTAGGGGGAATAGCACATC
GTTTATTATACATAAATAAATACACATTACCTCAAGATCATTAGGAATTATGTCAAAATAACAAAACTTGAAGCCAAATG
GGAACCAGCAAAGAGGCATAATTATCTAAGAGTTCGGATTAAAAAATTAAATCTTTGTTCCTTTATGGAGAGGTTAGTCC
ACATCCCTCTTAGAAGAAGTGGAAAATTCTGTGTTAAGGGATACTGTGAGTTTAATATTTTCTACTCTAAGAGTTGCTTT
TGCTAATAGGATGTGTTACAAAACAGAAAGAAGAAAAAAAAAAAAACAGCCTAGAACACTTTCATTGTTTCCCATGCCA
TGTCTCCAAGTTAGCAATGGAAAATGGTTTGAACCAATCCAAGAAAAACTAAGAGATTGGCAGGGAACAAAAAATTACAC
ACAGAAAAGAACAGTTACTTGATAAAGATGACAGCTGTCATCTAAAGGGAAACTGAGCAGTAAGCAGACTGGACGTAAGC
AGTCCTTAAGAGACTGTGCAGTACCAGGAATAGCAGAGAAGAAGCAGGAAAGCCTGTTGGGTGGACGGAACAGATGGAAT
TTCTTTCAGAGATCACTGTTGCATTATTACCATTTTGTAAAAACCATCCCCGCCTCTGTGTGTCGGGGGGAGGGGGAGGAG
GGTGGCATTACATTATCAAGATTGCTACTGGAGTCAAGCATGCTTAGTGTACATATCGATTGCCTGAATAGCCTTGTGTC
TTTCATTCTCTTGTCTTCTTTTATTAATTCAGAAATATCTGTTCAGAGCCTTGCCTTTTTAAAGTTGATTGCCATTTTTA
GGTGAATTTCAGATGTTCTTTATACATTTCAGGTTGCGTCCTATTTTAGATGTGTGATTTAATTTTTTTTCTCATTTTATA
GCATGCCTACTTCCTACTAATTTTTTTTTTAATGTTGTGTCTTTTAAAGGACAAAAGTTTTGCTAAACATGGTGGTTCAT
GCCTTTC
```

MOUSE mRNA SEQUENCE : mR7-046.1 (Seq ID No: 70)

```
TGCAGGAAGGACAAGTGCTAGAATGTTCCCTCATCAACAGTATTCGCCAAGCAGCCATACCAAAGGATCCCAAAATCCTG
GCTGCTCTCGAAGCTGTTGGAAAATCTGAAAATGATCTGGAAGGGCGGATATTGTGTGTCTGCAGCGGCACGGATCTGGT
GAATATCGGCTTCACTTACATGGTGGCTGAAAATCCAGAAGTAACTAAGCAATGGGTAGAAGGCCTGAGATCGATCATTC
ACAACTTCAGGGCAAACAACGTCAGTCCGATGACATGCCTCAAGAAACACTGGATGAAAACTGGCCTTTCTGACCAACACA
ACTGGTAAAATCCCAGTGAGGAGTATCACTAGAACCTTCGCATCAGGGAAAACAGAAAAGGTGATCTTTCAAGCCCTCAA
GGAACTAGGTCTTCCCAGTGGAAAGAATGATGAAATTGAACCTGCTGCATTTACTTATGAAAGTTCTATGAACTGACAC
AAAAGATTTGTCCTCGGACAGATATAGAAGATCTTTTTAAAAAAATTATGAGGGTTATAGATCTAAATGGACTTATTTTG
TCATCACAGCATCAGCGAGATCCTCGGCTGAATGAAATTTTATTCCCATTTTATGATGCTAAAAGAGCAATGCAGATCAT
TGAAATGTATGAGCCTGATGAAGAGCTGAAGAAAAAGGCCTCATATCCAGTGATGGATTCTGCAGATATCTGATGTCAG
ATGAAAATGCCCCTGTCTTCTTAGATCGCTTAGAACTTTACCAGGAGATGGACCACCCGCTGGCTCATTACTTCATCAGT
TCCTCCCACAACACCTATCTCACTGGCCGGCAATTTGGAGGAAAGTCTTCAGTGGAAATGTACAGACAAGTTCTCCTGGC
TGGTTGCAGGTGTGTTGAACTTGACTGTTGGGATGGAAAAGGTGAAGATCAGGAACCGATAATAACTCACGGAAAAGCAA
TGTGTACAGACATCCTTTTTAAGGATGTAATCCAGGCCATCAAGGAAACGGCGTTTGTCACATCAGAATACCCTGTCATT
CTCTCCTTCGAAAACCACTGCAGCAAATATCAACAGTACAAGATGTCCAAGTATTGTGAAGATCTATTTGGGGATCTCCT
GTTGAAACAAGCACTTGAGTCGCATCCACTTGAACCAGGAAGGCCCTTGCCGTCTCCTAATGACCTCAAAAGAAAAATAC
TCATCAAGAATAAGAGGCTGAAGCCTGAAGTTGAAAAGAAACAGCTTGAAGCTTTGAAAAGCATGATGGAAGCTGGAGAG
TCAGCCGCCCCAGCTAGCATCTTGGAAGACGACAATGAAGAGGAAATAGAAAGTGCTGATCAAGAGGAAGAAGCCCACCC
TGAATACAAATTTGGAAATGAACTTTCTGCCGATGACTACAGTCACAAGGAAGCGGTTGCAAACAGCGTCAAGAAGGGCC
```

```
TGGTCACCGTAGAGGATGAGCAAGCATGGATGGCATCTTATAAATACGTAGGTGCTACCACGAACATCCATCCGTACTTG
TCCACGATGATCAACTATGCCCAGCCCGTGAAGTTTCAAGGTTTCCACGTGGCTGAAGATTTAGCGATGCAATTGAATCA
AGGAAAATTTGAGTATAATGGATCATGCGGGTACCTTCTCAAGCCAGATTTCATGAGGCGGCCTGATCGGACATTTGACC
CCTTCTCTGAAACCCCTGTGGACGGGGTTATTGCAGCCACGTGCTCAGTGCAGGTTATATCAGGGCAGTTCCTCTCAGAT
AAGAAGATCGGGACATACGTGGAAGTCGATATGTACGGGCTGCCCACCGACACCATACGGAAAGAGTTCCGAACCCGCAT
GGTTATGAACAATGGACTCAACCCAGTGTATAATGAAGAATCGTTTGTGTTTCGAAAGGTGATCCTGCCTGACCTAGCTG
TCCTGAGAATCGCAGTCTACGATGACAACAACAAGCTAATTGGCCAGAGGATCCTTCCTTTGGATGGTCTCCAAGCAGGC
TACCGACACATCTCCCTGAGAAACGAGGGAAACAAACCATTATCACTGCCAACAATTTTCTGCAATATTGTTCTTAAAAC
ATACGTGCCTGATGGATTTGGAGATATTGTGGATGCTTTATCCGATCCAAAGAAATTTCTTTCAATCACAGAGAAGAGAG
CAGACCAAATGAGAGCAATGGGCATTGAAACTAGTGACATAGCAGATGTGCCCAGTGACACTTCCAAAAATGACAAGAAA
GGCAAGGCCAACCCAGCCAAAGCGAACGTGACCCCTCAGAGCAGCTCTGAGCTCAGACCAACCACCACAGCCGCCCTGGG
CTCTGGCCAGGAAGCCAAGAAAGGTATTGAACTTATCCCTCAAGTGAGGATAGAAGATTTAAAGCAAATGAAGGCTTACT
TGAAGCATTTAAAGAAACAACAGAAGGAGTTAAACTCTTTAAAGAAGAAACATGCAAAGGAGCACAGTACCATGCAGAAG
TTACACTGCACACAAGTTGACAAAATCGTGGCCCAGTATGACAAAGAGAAGTCGACTCATGAGAAAATCCTAGAGAAGGC
GATGAAGAAGAAGGGGGGAAGTAATTGTCTTGAAATAAAAAAAGAAACAGAAATTAAAATTCAGACCCTGACAACGGATC
ACAAATCTAAGGTCAAAGAGATTGTGGCCCAGCACACAAAGGAGTGGTCAGAAATGATCAACACTCACAGTGCGGAGGAG
CAGGAAATCCGGGATCTGCACCTGAGCCAGCAGTGTGAGCTGCTGAGAAAGCTGCTCATCAATGCTCATGAGCAGCAGAC
CCAGCAGCTGAAACTCTCCCATGACAGGGAAAGCAAGGAGATGAGAGCCCATCAGGCTAAGATTTCTATGGAAAATAGCA
AGGCCATCAGTCAAGATAAATCTATCAAGAACAAGGCAGAACGGGAAAGGCGAGTCAGGGAGTTGAACAGCAGCAACACT
AAGAAGTTCCTAGAAGAAAGAAAGAGACTGGCGATGAAGCAGTCAAAAGAAATGGATCAGTTGAAAAAAGTCCAGCTGGA
GCACCTAGAATTCCTAGAGAAACAGAACGAGCAGCTTTTGAAATCCTGTCATGCAGTGTCCCAAACACAAGGCGAAGGAG
ATGCAGCAGATGGTGAAATTGGAAGCCGAGATGGACCGCAGACCAGCAACAGTAGTATGAAACTCCAGAGTGCAAACTGA
AGCTGGAAGACCATGCAACACCAGAAGATCATGCCCAGCTTCCAAACACAGACTCCACAGACAAAAGCTGTTTTCTTTGT
GTTTCTTTTATGTATAGACACGGTGTTACCTGAAACCACATGGACTGGGGATGACCAAACGCTCTTATATTTAACAATTT
GAGTATTGAATTTCTTTGGCCAACCAAAAGTAGCTATGTACACATACACACACACATACACACACACACACAGAGA
GAGAGAGAGAGAGAGAGAGAGAGAGAGAGAGAGAGAGAGAAATCCCCTATTCCTGACAGGCAGAGTTGAATCATGATATATG
ACTTAAACATGTTTGCTATGAGACAGCATCACAAGCCAGTGGGCTTGGTGATAACAACTCTGCTTTGTGGTGCATTAGGA
CATGTTTGAGCTGCTGCTGCTGCAAAACAAAACAAAACAAAAAATTAGTGCATTAGTAACTTAATAGTAAGCGCATGCA
CTAGAAGAAACACCTCTGTCTCCAGTTCATTAGCAGAAGTGGCCGTCTGCTATACAAATCACTTCGCAGAATTTTCTACA
CCTAAGTTACCTCATCAGTAAGTGCCATGTCTTTACCATGCCATAAGATGCTAACCTTCCTGTAAATGTTGCAGAAATCG
TTAGGACAATAGAGACACAAAACGGTGTATCTGTGCCAAGCTCATCAATGCTCCCAGGGGGACTCGGCAGGCTCGTTTCA
GAAAGTTTACAGCTGACCCTGCTTTGTAGGAATTGCTTCTGCCGATTCCACATATTGCAATTCACTCTACACTATAAGGA
TCGTCAAGTGGTTATTGGCAAATGTTTGTAAATGTGACCATGTACAAAGTATTTATACTCCTTAATTCTCACAGTTATAG
CTCAAAGTCATCTAAATATTACCCACGACAAGACTAGTGAGCAGGAATCATAGACGTAATGTTTTGCATGATGCACAAG
CACCAATTATGACTAACCCATTCAAACACAGTTAACCTAACACCAAATAAATACTGGTTAAGTAAATGTACGGCACAGAA
TATTATTTGACTATCAAGACTTTTAGCATAATGAAAAAGTCTATATATATGTGTATATGAATTATGTGGGCATTCTTGAT
ACTTCAAGTTCTAGTTTGAAAAATACATAACTAATTTAATTTTACACAAAAATATTTATGCAGATTTTCAGACTTTCATA
TCAGGAAATAACCTTTTTATGTCTGTTAAATATCCAAACAATTTGCTACCGTGTTAATCTGCATGGTCTTCTCGCCTGCT
GTAGTTGCCTTGCAGGTGTCTCATAAAGTGGACCGATGGGATGGAGTCATGCTGCCCAAACTCAGAGGAGCATGCAGGGA
TCCGGTAGCCTAGCAAGAGTCAGATTCCTGATTTTAGAATACAAAATGCCAACTTAATTGTTGTATATGGTTGTACAAAT
AGATTCTGTACCTTCCTCTGAAGAGGGCAGAGAAGATTATGATACCATGAAGGCTAAAATTCAATGTACAACTTCTCTAA
TAAAAAATGAAAACACATGTAACATCTACCAGAGTATTTGCTTCCACGATGCTTTCTAGAAAACCTTTGCCTCTTCATGT
ATGTTTTCTTCATTATTTTCAAAAAGGGGCTTTATTTCCCATCCCCATTGTGGTTTCCCCCAACTGACACAGAACACACA
GATTCTACATACAGAAGTCATGAGCTTTTTGTCCCTCATAGTTATGAGTTGAATAGCTCATAATAGCTCTGTACCCTGTC
AAATTCCTGGCTTTCTTCTTTGTAAAACAGTAGAATCAAGACTGCATATATTCAGCACCAACATCCCATTTGATAAAAGG
CCCTCACATTGGGATTGCCGGTTTTCAAAATAAAATTGTTAGACTCT
```

MOUSE PROTEIN SEQUENCE : mP7-046.1 (Seq ID No: 71)

```
QEGQVLECSLINSIRQAAIPKDPKILAALEAVGKSENDLEGRILCVCSGTDLVNIGFTYMVAENPEVTKQWVEGLRSIIH
NFRANNVSPMTCLKKHWMKLAFLTNTTGKIPVRSITRTFASGKTEKVIFQALKELGLPSGKNDEIEPAAFTYEKFYELTQ
KICPRTDIEDLFKKIMRVIDLNGLILSSQHQRDPRLNEILFPFYDAKRAMQIIEMYEPDEELKKKGLISSDGFCRYLMSD
ENAPVFLDRLELYQEMDHPLAHYFISSSHNTYLTGRQFGGKSSVEMYRQVLLAGCRCVELDCWDGKGEDQEPIITHGKAM
CTDILFKDVIQAIKETAFVTSEYPVILSFENHCSKYQQYKMSKYCEDLFGDLLLKQALESHPLEPGRPLPSPNDLKRKIL
IKNKRLKPEVEKKQLEALKSMMEAGESAAPASILEDDNEEEIESADQEEEAHPEYKFGNELSADDYSHKEAVANSVKKGL
VTVEDEQAWMASYKYVGATTNIHPYLSTMINYAQPVKFQGFHVAEDLAMQLNQGKFEYNGSCGYLLKPDFMRRPDRTFDP
FSETPVDGVIAATCSVQVISGQFLSDKKIGTYVEVDMYGLPTDTIRKEFRTRMVMNNGLNPVYNEESFVRKVILPDLAV
LRIAVYDDNNKLIGQRILPLDGLQAGYRHISLRNEGNKPLSLPTIFCNIVLKTYVPDGFGDIVDALSDPKKFLSITEKRA
DQMRAMGIETSDIADVPSDTSKNDKKGKANPAKANVTPQSSSELRPTTTAALGSGQEAKKGIELIPQVRIEDLKQMKAYL
KHLKKQQKELNSLKKKHAKEHSTMQKLHCTQVDKIVAQYDKEKSTHEKILEKAMKKKGGSNCLEIKKETEIKIQTLTTDH
KSKVKEIVAQHTKEWSEMINTHSAEEQEIRDLHLSQQCELLRKLLINAHEQQTQQLKLSHDRESKEMRAHQAKISMENSK
```

AISQDKSIKNKAERERRVRELNSSNTKKFLEERKRLAMKQSKEMDQLKKVQLEHLEFLEKQNEQLLKSCHAVSQTQGEGD
AADGEIGSRDGPQTSNSSMKLQSAN*

MOUSE PANTHER CLASSIFICATIONS
        FAMILY (SUBFAMILY)
            CF10336(PHOSPHOLIPASE C-BETA-4)
        BIOLOGICAL PROCESS
            Lipid, fatty acid and steroid metabolism(2.03.00.00.00) >
Phospholipid metabolism(2.03.04.00.00)
            Signal transduction(2.11.00.00.00) > Cell surface receptor mediated
signal transduction(2.11.01.00.00) > G-protein mediated signaling(2.11.01.07.00)
            Sensory perception(2.22.00.00.00) > Vision(2.22.03.00.00)
        MOLECULAR FUNCTIONS
            Hydrolase(1.21.00.00.00)          >          Lipase(1.21.01.00.00)          >
Phospholipase(1.21.01.01.00)

MOUSE GENE ONTOLOGY
        BIOLOGICAL PROCESS
            lipid metabolism > membrane lipid metabolism
            signal transduction > intracellular signaling cascade
            nucleotide metabolism > lipid metabolism
            cell communication > signal transduction
            membrane lipid metabolism > phospholipid metabolism
        MOLECULAR FUNCTION
            phospholipase A2 > phospholipase C
            inositol/phosphatidylinositol          phosphodiesterase          >          1-
. phosphatidylinositol-4,5-biphosphate phosphodiesterase                                    .
            ligand binding or carrier > calcium binding
            nucleotide binding > ATP binding
            GO molecular function > motor
        CELL COMPONENT
            cell > nucleus
            cell > cytoplasm
            mitochondrial membrane > mitochondrial outer membrane
            GO cellular component > cellular_component unknown
            actin cytoskeleton > non-muscle myosin
MOUSE PROTEIN DOMAINS (INTERPRO SIGNATURES)
            IPR001711 (sp Q9Z0G6 Q9Z0G6 RAT)
            IPR000909 (PLCXc)
            IPR001711 (PLCYc)
            IPR000008 (C2)
            IPR001687 (ATP GTP A)
            IPR001711 (PIPLC Y DOMAIN)
            NULL (LYS RICH)
            IPR000008 (C2 DOMAIN 2)
            IPR000909 (PIPLC X DOMAIN)
            IPR001711 (PI-PLC-Y)
            IPR000909 (PI-PLC-X)
            IPR000008 (C2)
            IPR001192 (PHPHLIPASEC)

HUMAN GENOMIC SEQUENCE : hD7-046 (Seq ID No: 72)
AGGAACTCAGATGTCATTTCCAAGGGAATTGCTACAGGCAAACAACCAACATCCAAACAATCTTTTGAAACAGCTCAGTT
ATTCTCTTAGGAAGTTGTTATACTTCGTTGTATTCTAATTTCTTTGAAACTGATTATTGCAGATTGAATTAAATTGCCAG
GGCTCAGAACACAATACCTGAAAACATGGCACCTTGACATACTGAGTATTTTGAGATAAAGGAAATTGAGAAAACTGCAG
AAGCTGGAAGGTCAATCTCTGACCTTCTTTCCTGAGACACCTTCATGTGACAGGTGTCCCACTTTATGCCTGGAGGGAAG
GAATGATAACACAAAGATACCAAGAAGAATGTGAAGAGACCTTTCTCAGTTCCCCCCAGTTCAAGACCATTATATCGTAC
CCACTTTTGTCTAATCATGCTTCTATATGACTATCCATTCTTTATCAAAACTAAACATAGAAATATACGATTATCTCAAT
TTCTGTCTTTGTTTCTGAAGGCTCCTGTGTCACATAAAACTTACATTAAATAAATTTGTATGTCTCTCTTGTTAACCTGT
CTTTTGTTATAGAGGGTCTCAGACATGAACTTTGTGATGGATGAGGAAAGATATTACTTTTTCTTCCCTAGAATATATTG
ACAGTTATTCATTATATTTAAATTAAGCTGGATCTCTAAATACTCCAGGCAACTAAGCATATTTTAGACATGTTTTGTAA

```
AATCCTGATAGATTGAAACCTATAACACGATCACAAATGTCATCTAATACATTTATAAACCTGGATCTTCACCCCAGGTA
ACTAAACATATTTTAGACATGTTTTTTAAAATCTTGATGTAATGGAACCAATAGCATGATCACAAATATCACCTAATAAA
TTTATAAAGGCAGTACACTTTAATATTTTTATTCATCTATTTGTAATTATTAAATTTTAAGGAAGTTACACTTGCCCCTC
AATTATGATTAATATTGAGGTACGAGAGGCAGGGACAAATAGCGAATGCTTGCTTTCTGTTTTTATTTTATTCCTGTGCA
ATTTGTGTTCTTTTAATACACTCCAGGAAATTGTTTTTAATCATGTGATGCTTTATAATAGATAAATCATAGCAAAGGCC
ATAGATTTCTTGTAGCCCAGAATTCATTAAAGTCTAATTCCAGTACCATCACAATCAGTTTTTTCCCAAATATAAGTATT
GCAGAGGTTTCAGTTAACGTTCATTTTAGTCAGTATATTAAGTAACTCCTTACTAAGTAATGAATAAGGATCAGTCATAA
CTGGGATGTGTGAAGGCTCCCATTGTACAGGGAAGGTTTTAAATTCTGAAAGGGGAGAACAACGTTTTACAGCTTTATGT
ATTTATCTCTTCCTGTTATTGTTCTTGCCAGGATTTCTGCTAGAAAACATAGACATTATTTTAAGACTAATTTATATGA
AAAGAAACAAGTTTTAAAATTGTATCCTCCATTTGCTTTAGAAAATATTTTTTCTGGAATATAAAAATTACAGTCACTAT
GAATATTTTAAAATACAGGAAAGTATTAAAGAAAAGAAAATTCTAAAATTACATCAGCTAGCTGTAACTACTATTTGTTT
TGGTATATGTCTTGCTAGTTTTTCCCGTTGTAAAACAGTGTACATATGTAAGCAATATGTGTATAATATATAACATAAAA
ATTTGGATCAGACTCGTGTAATTTTATAGCCTGTGGTTATTTTTTTTCTTAACTGTATGTTAAGAGCATTTGTCTTTGCT
TCACAAGAGTTGTGAATATTTTTAGAAACTCTTCTAAATTTGCTATTGTTAAGCAAATAACTATTCCTTTAAAGTACTAT
TTTTAATGGGTACAGTATTTAAGTACTAGAGTGTGTTTAATCAATTGCTTAGTGATGATTATATAAGGCCAATATTTTTC
TACCATAAACAGCACAACAGTATTCTTGTGCATAGATTTTGTACACATTTACATAGGATAAATCCTACTTGTACAATTAC
TAGATAAAAGAGTATGGATATTTCAAGGCTATAGAAATGTATTACAAAATTCTTTTTTACCAGATATGCCCTCACCCCAT
CCATCAACAGCATTGCATATTATCATATTTTTCTTCCTGTACTCTTTGTATGTTTTTTGAAAAGGTTATTTAATGGCTTA
ATAAAAATGGAGGTATTTTTGCAAATGGGGAAGTTATCCCTAATGATACAATATATTAATGTCAAATTAGATTCTTACTG
GAGGTAAAGCCATTTATTTAAAAGCCCTGGATGATCTGATGACAGCTTGTTGATATGTTTTTAGAAATATAAGGATATCT
TATTTAAAGATTGAAGAGCAAGGCACTGAAATATGTGTGATAGATTCAACTGATTTACTTGTGGTCAGTGGAAATTTTAT
CCCAACTCTCTATGTTCTGGAAAACAACTTTGGTTGTGCCATCATGAGGTCACTTAAGGTCATGGGTTATTTGGTTTAAT
GGGATTTGTATGGTTGGATGGGGAAGGGATTAATTTGTTATGGAAATTTTTTTGTCATAAGGTTGAAGTTAAAAGTTGCA
CTTAATAAAATTTATAACTGAGTCATGGTGCATTTATTCACTTGAAAGCTATATGTTATCAAGTAGAAGCAAATGCTTTT
TCAGATGAATTCATTTTTTCACAAATAGGAATAAAAAATGAAGAACACCTTGTTTGGCTTCGGGGGAGCTGCAGGATTTG
AAGTTTGTATTTATTGCTGAGATGCAAAATTGTTACAAGACATTTGGAACCTGGGGAAGAGTATGCAGAAATAATGTCAT
TAGTCCTCAAAGGGGCTTTGGAACAGAAGGACAGGAATGATCTGAATTCATTTCTAGGTGAAGTTTATTCTGTACTTTTC
TCTCTGTCTTCCTCTGATGATTCAGTTGAACACACATGTTGAATGACCATTCCCTTCAAATCAGAGCAGTCTGTACTGCA
TTCCAAACTTATTTGGCAACTTTTTAGTTCTTTTAAGAAAGGTGTTCAGCTTCAGGGGGCCCAATATATGCATTTTCTGG
GCTACTATAAAAAATTAGCACAAACTAGTGGCTGAAAGTAACCAAAATTTATTCTGTCACAGTTCTCGAGGCCAGACGTC
TGACATCAAGGTATTTTCAGGGCCACATTTCCTCCAGGCTGTGGGGAAGAACCCATTCCTGGCCTATTTCACCTTCTGGT
GGCTGCCAACTCTTCTTGAACTGTGGCTACATCACTCTAATCTCTGCCTCCGTAGTCGCATTTCCTCCTCCTCTTCTGCT
TGTGTCAAAACTTCCCCTGCTCCCCTTTTGTAAGGACATTTAGGGCCCACCTGGATGATCCAGAATAATCTCCCCATCTC
AAGATCTTTCGCTTAATCTCATCTGCTAAGTCCCTTTTTGCCCTGTAAGGTAACATTCGCGAGTTGCAGCAGTTAGGAGG
TGGGTATCTTTTGGGGGCCATTTATTCAGCCTGCCATACCCAGGTACCTTAGATATGCCTGGTTTCACATACTGCCCTCT
GTTCACTCTCTACCAGAACGTGAGGCACATGTCCAGAGACAAAAAATTCCTTGTGAAAGTATTGACCTAAACTGGTCTAG
GTTGGGGGTGGTGGTGAGGGAGGGTGAGCAGCACTGACTCTAAAGTTGATCCAGCACATCTGAGATAGGTGGTCTGGTAA
TTCCTGAAGACCACCTTATAGGTCTAGCAAGGAAGACAAGACAGCAGGTTTAAAGACTCAGGACAGAGTAGGCCAAGAAA
ACCAAATCTCTTCCTTGGTTCACCCCAACCGAATGGATAGAAGGCCATATTTATGGGTAATGCACATGTCCTACTTGAAT
ATTTGGCCCCTCGACAGCCCACCTCAGCCCCCTTGTGTTCCTCCTAACTCTGCCAGTTTCTTATTATCATCTTTAGGAGG
AGCTTTTGCCACTTGCTCTGGAACAAGACCTTCTAGGACTCCCCTTCTTTCTTGTCCCCCGCTTCCAAAGTTTCTTGCAG
TCCAGGGAGTTTTTCCTCTGCCCTAGTGCAATAGCTAATCCGATCCAGTTGCACAAGGATTTAGAACAATGCGACTATTT
AAAAGCCTTCTGGCCTCCTTCCAGTAACTGCTCAATGTGTTTACACTGCAAGAGGTTTCAGGCTGTCTATTCTTTGACAA
TGTAAAGTCTCTTCCACTTCCCGTGTTCCTCAAAGAGAAGGTGGATCCTTTCCTGTGGGCTCTGCATGGTGAGATGATGC
CATTCTAGGAGGTGGAGGAGACAATTAAACCTTGAAATATCTTGTAAGTGAGGCCTTGGAAGGCAAGCTAGAGAATTTGA
GAAGAGGAACTTTTGGCATGGTACAATCAAAGCCTTTTTTTTTTTCAAGCCAGTTAGTTGGCTGTGGGTTGGGATTTGAG
ATAATTGCAATAAGAATGTAGTTATAGGGTGGCCAGTTCCTCCCCTCTTTTAAAAAGAAGTAACTGTCCCTTTAACATTC
TCTGCAGTCACTGGGACTATAATGTGTACCTTCGTTTGTTCCCATTTTAAATCAATTATCTGGATCAAACTGTAGTGAAA
CAGGATTGGCTGGGTCAACATTTTCTAGTCTGTAGGTCATCATTGTACCATGTGTGTAAAATAACCCAGCTAGTGCCCCT
GATGCCAGGCCCTAGGAAGTTTTCCACAGATAGCACAAAGAACAGCCTCGACCACAGCCACCAGTTTCAAATTGGGAACA
CTCAGGATACTCCAACATCATTTTGCCAGAATACTTCTTACCTTGTTCTGAACCCCAATGCTATGAAGGAATAAGCAATG
AAATGAACTTTCACCTCCACTGCAGAAGAGATTAAGGTCAAAATTACCCTCCTCCTTCACCTCCAAAGTTCTTCATATAG
TGAGACTATTGTGATGAAATTAGTACCCAAGGTAGAGATGAATATTTTAGTAGTTAAGAATAGTAGTGAGGTCTGAGAAA
CATCATATACTGGGTACTCATGTTGATTCTAGGAGAACCAGAGTTAAACCAATGGAAGATATGCAAACTGTAAAAGCTT
TTTTTTTTTATTTTTATTTTTGATATAAACTTTTTGTATCCAAAGCCTCTTACTGATTTAAAATGAAACCAAAGGGATGA
ATTAAAAGGGATGGTTGTCAGGAGTGAGACAATCATAGAACACATAAAATTCTGCAGAAAGAGATGTTTTGCCCACCAT
TCTTCACAGCCTGGCAAACTGGGATGGCCACCATGTTGTTTTTTAATCTATTTTTCAAGCTAACACTTAGCTCCCTGTAG
TTGGTTATTAAGGGGGGGTCTGTGGGTCTATTTTTCTCTGCAGATCTGTTTATAGCATAGAAGGCATGAATGCACATCAT
CTACCTCAGAAGCTTGTTTCTAAGCTTCTTGGGTAGAAGCTATGTGGTTATGTGTGTACATACATGCTATATTCACCAAG
CTATGCAAAAGGTTTCAAAGTCAAACTTATTTCTTCTGTGTAACTGATAGAAGGCTTTGTATACTTCGATTATCAACTCC
ACATTCCCCCTACCCTCAGCCTTTGGTAACCACTGTTCTGCTCTCTGCTTTTATGAGTTCAACTGTTATAGATTCCACAT
```

```
GTAAGTGAGCACATGGATATTTGTCTTTCTGTATTTGGCTTATTTCACTTAGCATAATGTTCTCCAGTTCCATCCATGTT
GTCACAAATGACAGAATTTCTTCCCTTTTTTAAGGCTGAATAATATTTCACTGTGTATAATACACCACATTTTCTCTATG
CATTCATTTGTTGTGGGATACTTGGGTTGATTCCATAACTTGGATATTGTGAATAGTGCTGCATTGAACATGGGAGTGCA
GATATCTCTTTGACCTACTGATTTCAGATCTTTCCAGTAAATACCCAGAAGTGGGATTGCTGGATCATTTAGTTCCTCAA
AAAACTAAAATTCCATTTTTAGTTTTTTGAGGCACCTCCATACTGTTTTTGACAATGGCTGTAGTAATTTAAGCCAAATG
TATTTTAACTTTGAAAGATATATACTTTTTATTTAGCTTTTAAAATATTCTGATTAAAGTTATATACAATATTCTCTTAG
AATATTCACATTCTCATTCTTTAATTATATCCCACTTTTCATTGTAAATGTTGTGAGTTTGTGCTGCAGCCACCTCCTCC
TCCTCATCCTCCCTTCTGCCCCCACCTAAATCTCCCTCTCTCTGTGTCTCTCACTCTTGCTCTAAGTACTTGCCAAATAT
ATATCCAATTCAGAAACAATTAACTTTTGCTTTTACTGATCATTGTTTTACTATTTTTTTTTTTTTTGGCTACATATTA
CATATCTAGGTTTTGGTTTGTAATGCTCTCATCTTTTCTTTCTAAAAAGTTTATAATTTCTGCTTAACTCAAGAATGATT
AAGGGGTGTATGTATGTGTGTGTGGGGGGGTGTGAATGTTTAATTTCCATGTATATAGATGTTGTACACTCTCTGTCATT
AATTTATATGTTTCCTTGTTGTTGTCAATGAGCATGACCACAATATTTTCTGCCTTTTGAAAAATGCTGAAATTTCCTCT
AGGGCCTAATCGTGATCGGATTTTGAGATTGTCACATGTGTGTGGGTAGCCAGGGTGCAAAGAGGGAAAGAAAAAAAAAT
GTATTAGAGAGAGAATAAATATATTAAGAGAGAGAATAAATATCATTAAGTACTGTTATTTAAATTGTGTTACTTTGAG
TTATTTAAGTTGTTGTACTAAATTATTAAGTATGATATTCAGATCTTCGAGATTCTTACTTGTCTTCTTATTCTGTTATT
CTGAGAATCATTGTCTCCTGTTTGTGACTTTGCTAATTTCTCCGTGTACTTTGTCAATTTTCAAATGTTGTGATGCTGCG
TGCTCACATGCTTAGATATTAATGGCAGTTAATGTGATGTATTATTACTCTGCCTCTGCTTTTTCTCTTGAAGTCTGTTT
TGTCTCATGTTGTTGTTACACCTGTTTTCTTTTTTTCTTAGCACGTGCCTATGGTATCTTTTTTCATTCCTGCGTTTTCT
TTTTTATTTTCTTTTTTTTTTAATTTTAGTTCTATCTTTTTTTTAATTATACTTTAAGTTTTAGGGTACATGTGCACAAC
GAGCAGGTTAGTTACATATGTATACATGTGCCATGTTGGTGTGCTGCACCCAGTAACTCGTCATTTAACATTAGGTATCT
CTCCAAATGCTATCCCGCCCCCCTCCCCCCAACATCCCTGCATTTTCAAACTTCTTAGATAGTTTTCAAAATATTTCTGT
AAACAACCATAATTTTACCTAATCCGAAAACATCTTTTATAAGTGAATTTAACCAATTCGTATTTACTATGCTTAGTGAT
GTGTCATTTTATTTTGTGTTTCCTACTTATAATATGTTCCTGTCTTCTCTCCATTCTTTATCTGGTTACTTTTTAAAAGA
GGCTCGAGTTTCTTTTGCTATTTCTCCCCTGCTAATCATTTGGGAGTTATGTTTTTTTAATTTTAATTTTCTATAGGAGA
ACTCTTAAATTCATAGCACCCATATTTAAACTTCTATTTTCCTACTGATGTCTAGAGTTTTTAGTATCTCTGTTCCCTTC
CCAAAATGGTTTTACTTCCCTTTGTCTCATACTTTGTTCCTCCAACCACTTCACTGGCCACAACTCATATATTGATATAA
TCTAGGATTTGGGTTGGCTAATTTTTAAATATATTTTTTCTTTTCTACTTTTTTGGCATAGTACAATAAAACTTAAGATT
AAGCCTATTGTTTCTTTTTACTTTATTTTTCTTAACATTATTTCCCATCTCTCTCTTATTTCTCTATTTATTTTCCCCTG
ACAGAGTATATAATACATTAAATTTTTCAGAGTAGATTACTAAATCTTTCTGATTACTTACAAGTCTGTAAATAACTACA
TTTTTCTCCTAAAAGTTGAAACAGTATTGAACACAGAATTTTAAATCCAAATTTATTTTCCACAGAACTTCAAAATTGTC
TTACTGCATTTAGTGTTGTGGATGATGTACCTGATATCAGCCTCATCGTCATTTACCTCTAGCTTATCTTTTCTCTTCCT
GCATCTTTGAGAGATTTCATCATTTCTGCATATTTTGTGTCTTCAGTGTCACCATGAGGTATATTTGGGGGTGTATATCT
TTTTCCCCCATTTATACTACGTGGAATCAGTGGATTTTTTCACTTTGAGGACTCAAATATTCAGGTTTCAGAAACGTTCT
TCTACTGTTACTTTATTTCCATCCCTCCATTGTCTCCATATAAGGTTGGTAAACCTTTCCTTTCTATTTTCTATATCTCT
AACTTTTTCACCCAGGCTGGAGTGCAGTGATGCAATCTTTGTTCACTGCAATCCCCGTCTCCTGGGTTCAAGCAATTCTC
CCTGCCTTAGCCTCCCGAGTAGCTGGGATTACAGGTGCCTGCCATCACATCCAGCTAATTTTTGTATTTTTTAGTAGAGA
TGGGGTTTCGCCATGTTGGCCAGGCTGGTCTTGAACTCCTGACCTCAGGTGATCCACCCCCTCCCCCCACCCCGGCCTCT
CAAAATGCTGGGATTACAGGCGTGAGCCACTGAGCCCGGCGTGGTGTTGCATTTTAAGAATCCCTTGGCTCAACACTTC
ATCTGAGTTTTCTTTACAATTCAGCTCATCTATTAAGTTTTATTTCAACAATCTTGTTTTAAATTTCTAGTATCTGTGGT
TAATTATATTTTAGGAACAGAATATACTCTCTCTTCCCTCTGAAAAAATCAGTATGCTGAAGTCAGGTCCTGTTTTTCCT
GCTCACCCTCTTTCCTTGGGTGTAAGTTTTACTTGATGTGTTTGGTGTTCTTCCATGTTCTTTGCATTGCTTAACTATTT
GGTGACTCATTGTTGTATGCTCCCCTGTCATGCAAGATGTTTGGGTTGTAGTTATTGCATACATACCATGGTGCATGTTG
CCAACAAGGATGGGTAAAAGGAACGCTGAGTTCTTCCTAGTTGCAGGGAGCCACTCTGAACAGTGCCTAGCCTCTCTTTA
AATGTCCTACTCACTGTCTATAATAAAACTGTTGCAGGACTTTTCCTTAGTTCAGCTAAAAACAGGGTTCTTTGTCCCAC
AGCCATGAAAATTCAAGCTCACAGGCAATTTAAATGGCGAGTAAGACAGGGTTTCATTGGGTAAAAAGGAAGAAAATGGG
GCAACAGGGACTCTTGCAAGCCCAGAATCCCTGCTAGAGGGCTTCATTCTAGGCACTGGAATCCCAAGTTCCACACAGA
AGGAGGAGGGGCCAGACTCCTCTCTGCTACAAACATTGTGAACACCCCGAGGCCCCACCTCAGTGGGCAGGCTGGTTGGA
GTTTCTCCAGGGACCCCCTATGAGCTGGCTGTCTCAAAATCAGAAGTTCTCTTGTTCCCCACGTGGCCCAAGGGGTGAGG
AACAAAGGGGTAGGGGAAAGGGAGCCTAAGAGAGGGCTAAAGAGAGGACCAAGAGCTATAAAAAATTCTGGCTATTCCTA
CGTGATTCTGCAACTTATACCCTGTGGGCTCCTCCTGCCCATATGTTCACCTTCAAGCCCATAGTGCCCTTAGCTATTTA
CAGCAGGCCTCTCTTATGTATTGATTTTGGTCTACGGTTTCCTTATTTTATCGGTGCCTTCAGATTTTAAAAAAAGAAT
ATACACACACACACACACACACACACACACACACACACACACATCTTGTTATTTTGTAGGATTTGGAGAG
GGAATGGAAACTTCCCAAATGATCACCTCTGGTATTTCTTGATTATGATATCTCCAGGTTTAGAGAAAAGAAAGCAAGTT
GGGGGTCAGACAAAGGGAAGAAGAAAAAACGTATTCCACATATTTAAAGTAAATCTTCTGCAAACACAGAAATGCGAAG
TGATTAACCATCCTCAATTGTTTTTTCATTTTATATACTAACGAGCTATTCTTTTTCATTTTTAGGTCTTGAATATAATC
ATGGCCAAACCTTATGAATTTAACTGGCAGAAGGAAGTTCCCTCCTTTTTGCAAGAAGGAACAGTTTTTGACAGATACGA
GGAGGTAAAGAAGTGTTATTAATCTTTTCTCTCAAAACATTCTAATAATTATTAAATTTTAATTACAGTATTGAGTAAAT
ATTCTTTGACATAAGTAAGAACATCTTTAGGATTTATAGGTTGGAGTTGTTGAAAAGAGGCAAGTTATGAGCTAGAGTAA
TAATGATAATGATACTATAAATTTTTGAGCGCTCACTATGTGCCAGATGACCTTCTAAACATTTTATGTGTATTATTTGT
TTAATTTTTTACAACAACCTTACAAGGTTGTTGCTATTAATATTCTCAGTTTACAGATGGGTTTTATATAGTAACAGTTT
TTGCTTAATGCAGTGCTTTTGGCAGTCATTCTACATTTAACTTTGTATGTGTTATAAATGTAATAGTGATTGAACTTTGT
```

```
GCCAGTCATTGCTTTGCTCATTTCATTGATATAAACCATTTTGACCAAAGCTTTGCATGCCTCTGATTCTATCATTTATT
GTCTGTGCCCTTGCAAGGTGGTGATGACTCCAGACTTTCTATAGAGCAGGGGCTTTGGGCCACCAATCTGTTCAGAATGG
CTGGGTAGGTGAATTAAAGCCTCATTTGCATTGTAAATGCATGATTTTTCTTCTGTTGTACTTGAATTTGAGGTGACTTG
GGAAGGAGTGTATAATGAAATTGATGTTTCCTTTACCAAGAAGTTGAAAGTAAAACATATTAAAACTTCATTTAGAGTCA
GAGTTGGTTCCTTTCAAAATCAATGAAATGTATGAAGTGGCTAATTCGCTGATCTTCCCAGAGGTCCAGATTGTCAGATG
CAGAAACTAGGTCTTCTGTGAGCTGACATAGCTTAATACTTGTTGACTTGATATTCTGCAGGGTGCATCTCTAGATTTA
CAGGATAGTCACTTTTTAAGTAAACAAAATTGCAAATGCTAAGGATGGACCTAAACATGGTTACTATTCCTTGTTTGAAT
TAAAATATTGAAGTAATAGCTGTTCATATGTCTGAAATTAAAATAACAGTGAACTGCATTCTGCTATAAGATGAAACAGA
ATAATATTCTGCCATCCAGAGACAAGCACTGTTAATTCTTATGCAGTTTTCTTCTAGTCTTTTTTCATATTTGTTTTTCT
TTTTTTTCTCTCTCCCATGGTTGAGATCATAGAGTAATATTTTTAAAAATATAAAGCAGATCCCATTTCTCCTGTGTTTAA
AATGCTTCCATCACTTCCCATTGTAGCGTAAAGGGAAGTTCACAGAATGAAATACAAGGCCTTCTATAATTTGGCTCCTG
CTTAATTACCTCACCTTCATTTCTCTCCAGTTTCCAACATACAGCGTATGTTCAACTATAGCAAACCACTTACAGTTTCC
CCCATAAACTATTCTACTACATGGTTTGTTTATGCTACTCCCTCTTCCTGAAATGTATTTATTCCAACTGGTTTGATTGG
TGAACTCCTCTCATCTCAGAACCCAGATGAGAAATGTCCACTTCTGTGATATCTTCCTTGACCCATTGAATGAATAAGAG
GCGATAACTTATTTTTTGCCACCACTAACCTATCCTTGCATATTTTACACTGCCTTCACATTTTATTTAATGTTTCTTTC
TCTCCAACTATGTTCTGAGTTTCTTGAAGTCAGGGATCATATTTTACTTATCTTTTTCTCTCCAGGGACTAGCAGAATAC
CTGTAACCACCAAATTCTTTGTGGATGTCTAGCATCTAAAATGATTTTAGATCCAGTTGATTCCAATTCAGACTAATAAT
TCAGAGAAGGTAATTTTGAAGAGATTACATGCAATCATTGGTTCAGTATATCTCCTACTGTTATGTAAGAAAAAGCTATT
GGAGAAAAAAGTACTTGACAACAGTGAAATGTGATTTGATGAACCAGAGATTCATTTGAATCCTTCTTATTGCTTGTGTT
TTTTTTGATTATTAATCATCACAGCTCATATTAGGAAATACTGATCACCTGTGCTTCTAAAGGCATTTGCTTTTCCTCTG
GATTCCTCTACAAACTAGGCAAAGTTAGAAGAAAAGAACCCTTGAACAAGTATTAAATAAAATGTAACTAAGCTTTCAAC
TCTTTCAAGTGCTATTTTACTAGGATTTTGAGCAAAAACATTTTAACTTTATAAAGCAAACATGCGTCTTCATGATAACA
TACCAAACCACAAAGCTGAAGAATATTTCAAGCTGGATGACAGCCAGTTTAAAAAATAATTATTTCCCACTCCTATAAGA
CTCAGTTCTTTAGAGCCAGAGCCATGAAAACATTTAAGGCAACAACATGTAAGCAGAAAAGGTTCGTTTCTATGGTTGGT
ACATGAACAGCCATGAGGGATTACAGCTGAAAGCCAGTGCGAGGAATAAGATTAGAGAAACAAAACCCAAGCATCCCTAA
AGTGTCATAGCAAAGGTACCTTTTGTGAAGGGGCATAAATGCTGTCAGCACCATATGATAACTAACGTTATCATCATGAA
CTCAAATAGCAGGTAAACATAAAATTGTATTCCATGTATTTGTTTAAAGAAAGCAGCTGGGCACAGTGTCTTATGCCTGT
AATCCCAGCACTTTGGGAGGCCAGGGTGGGCAGATCAGTAGGTCAGGAGATTGAGACCATTCTGGCTAACACAGTGAAAC
CCCATTTCTACTAAAAATATAAAAAAAAATAGCCAGGTGTGGTGGCACATGCCTATAGTCCCAGCTACTTGGGAGGCTGA
GGCAGGAGAATCGCTTGAACCCAAGAGGCAGAGGGTGCAGTGAGCCAAGATCACGCCCCCACACACCAACCTGGGTGACA
AAGTGATGCTGTGTCTCAAAAAATAAAAAAAAATAAATAAAAAAAGGGAGCAGGGAGTAAGCAGTCAGTAGGTGGGGGAA
AAATGTTTAATAGGCACTGTTTTAAAAATAGTCAATAAATGTAGTAAGTGCAAATGCATTTAAAGTTTATTTTATTATTT
CTGCATTACTGAAGAATTATTTTTTGAGGCAGCCAGATTTTTACAAATGACCTAAGTGGTAGGCTGTGATTGTATTGCCAT
GACACTTCTAATTCTGTACTTTTCTAACTTTACTTGACTTACACGTTACTTAAATGCAAAATATATTTTGTTTCCTAAGT
CATGCTTAACATTAAATCTTTAAAAGACAGATTCGTCTCTTGTGGAGGAGAGAAATAGCAGTGTTTTTAAAAATACATGC
AACCCAGATATCTCTAACTCAACCAGCCCCCTGACTTTCCTATAATATCCACATCAAAGATCTGAAGAACCCATTCCCTG
GCAGGTCAGGGCCTTCCCAGTCTTTCCTAGAATTCCTAGGTGCTTACCCACTTTTGATGATGACTTATGTCCTCAAATGC
TTGGCTACCATTCAGTTTCCAGGACTGTTGACTACTCCAATTTGTTTCCTTCCCTAAACCCACTTTAACATCAAATTCCA
GAAATACAAGGATGTTGTATAAAGTAGACTTTTTACTGAAGATTTAGTGGCAGCAGAGGAGAATTGCATCATACACCACA
CCAAGACAACTTTAAGATTAAAAATCGGAGATCATGCTGAATAAGTTTCATTAGCCAGCCCAACCAGGAGTGTCTCTCTT
TTTTCTTCTTTGTCCCTCTCCTCCATCAAGATTCCTGTTCCTGTTTCCTTTATTTTATTTTAATCACACTCAGAGTCCTT
TTTCACTTTTCCAGATACTTTTTTCTTAACTGTCAAACATTTTCTGTCTCTCAATCTAAAGAAGAAAACTTCAAAATTCA
AGTCCCTCCTCATCCTGGAGCTGTTTGGCTCCCCTTTCACCTCAAACTCTCCTACTCAGACTCTCCTGAGATCCTCCCTT
TTGCATAAGCCTGAAAGTCAAAGTATGTCCTTAAAACACACAAAATCTAAAAAGAGCTTTATTCTTAAGAAGGAGACTGC
CATCATAGATAAGTGTTCACCAACAGAACTTTCCACAATGATAGAAATGGTCTACATTTGTGTTGCCGTTATGGCAGCCA
CATGTGGCTGTTCAGCATTTGAAATGTGGCTACTGGGACTGAGGGACTTAAGTTTTTAATCTTACATAATTTAAATTCAA
ATTCATATAGGTACATGGGCCTAGTGGCTTTCTTATTGGATGGCATAGATAATCAACTCAAACTACAACCAGATTTTAAA
ATTCCATTCTCCCTTCTTGACACTAAGAAGCATTCTCAGTGCATTCCGTTGGAATCTCCAGAATTCATCAGCCAGTTCTT
CTTAGGGGCAGCCAGAGCAGGGGTTCAAATGTTTATGGATGACTCACCTCAGCCACTGACCTCGTCTTTCCCACATCAT
AAATCCCAGCTCGGGCTACCCCTTCTTCTTGCCCATGGGACTTTCTTGTATCTGAGTCCAGCTGCCTAGATGGAGTCCCT
TTTCCTAGCAAAGTCTTGGTCTTCTTGGTCTTTATACTAGCCTCCCCACCTTCCACCCTACACACACACACACACACACA
CACACACACACACACGCACGCACGCACTCACACACACATGGTGGAGGAAAACTGAGACAATTATTGCAGGTGTCAG
GATCAAAGGAGTATCAAAGTGCAGGGTCAATACAAACCTAAAGTCAGGCACAGTGGGACAGGCTGACTTTGGCCTCTTTC
CAAGGTGGTTCTGAAATCAAATTGTGAGCAGCCACACCATGAAGCTCAGAAAAGCTCAAGCAGCCTACACCCCACAGGCT
GTTCATCAGCAAAGCAAAGCGCTTTCCAAATACAATTTTCAAAAGACTTAAAAACACAAGCCTCATATAAAGTATTTTGA
TTAAAAGATATATTTAACTAATCAGGGAGGACATTAGCAGGATGATAAAGCTAGTTCCAAGAGCACTTGAGGAAGAGAAT
TTTACGTAGGAAAGGAAAGGAAAGTGAAGTAAGTTTTGTCAATTAGATACAAAACTGGCTAATGTCCTATAAGGCAATA
AAACTCTAACCTATTATAGATTTGCCTTTTTCTACTGGACACAAGTCATCTACATCATTATAGAATAACAGCAGAAAAAT
GCTATCTATTAAACTTCGAAGAATCTATGTCCCAAACAACAGTAAATAGTAGGTCAAACCAAAAATACATTTTTCTATAG
AAGTAAATCATTCTTAGGTATGCCTCTTCAATCATGCTTTGATGTAACAATGAAAGAGGGATGGCCATCTTTGCATATT
ATTTTTAAGCTTGAGATAATTTAAATTCAATTATAAAACAATGTCCAGATATACCTGTATTTACACTAAAAGATACTTAC
```

```
AGTCATCAAAATGATAATAATCATAATAAATTAGTTTTTCAACTGTTATAATCAACCTATTAGTTTTTCAACTTTTATAA
TCAACCTAAGGGTAGAGAAAACATTTAATTATTTTTGACCAAGCATAACAAAAAAGCTGTTACCTTTATCTATTTAAAGT
AAGGGTGCCAGACAGATGCTGGAAATTAGAAGGGACTTGGAGAAACAGAGGAAAGTCCAGAGGCTTTATACTCTCAGCAA
GAGGGAAGGTTGGTAGTTGATGTACAATAGAGGCTTGCAGATATGATTTAAAGTATAAAAGTAGCCAATCACAAACCAAA
AATAAAATAATTTCAAATTTGGAGTTGGGGGAGAAAAAGTAGTGAAAATGCGTTAAGTGGAGTTAAATTGCTGTGTTTTT
ATTCCAAGAAACCGATTTCTGCATATTCCTGGTGGATAATTAGTTTAGTTACTGGCTGTCTTGCCATGAGTCCTGTTTTC
TTAAGCTCCTGTCTCTATTAGAGTTGGACATATGGAACAGAAACCTGTTGGTGCCACTTTATTATGCACTGCAGTAAGTA
CTGGCTTCATAGTTTGGACGGCAGCTGTACTCACCGCTATACCACCACCACCACTGGCTGGGCTTCTCAGTTTTAAACTC
TAGGCTTTCAGCATGTGGCCCTGAAGCCCTTTCACTCTCACCCCTCCACTTTCTTCCACTTGCATATTGTGATGAGAAAG
GTGGTAGAATGATCTCCTTGTGGTGTGAGAGGAGAGGAAGGGGCAGCAGGGCTGGGGGACTGGCCCCAGAAGCCTCAATG
AGGAGGCCCGTTTCAGGCTGAGTCTTGATAGGTGAGTCAGTCAACCACTCAAAGCACAGCTAAGAGGAAGGGAGAGGCCA
GGGCTTCTGCAAGGGCATGAGTATGACAGCACATTCTATTGAGGGGGTAAGGGGATTATTCAGCTAGGGCAGAGGGTAGG
GCAGTGGAGTACAGGGGTGAAGGCACAGAGATGGCCAGTGTCCACACCATCAATGGGTTTACTTTCCAGCCCAGGGCATT
GGCATTTGATTCAGAAAGCTCTAGGGAACCTTTGATGGGGTTTCAGCAGGGCAGTGATCAGTTCTGGATGACCAGTGACA
AGTGGATGTGGCTGGATTCCTTGTGGGAGTGGTCTGGAGGGTAGTGTTGGGCTTGAAGGACTTAATGGACAGTCAGTCAC
ACATATGGAGAAATACGGGAGGATTTTGAGTGTAGGAATAAATGAGAAGATTTTTCTAACCTGGTGGATGTAGAGTATGT
GAGAGAATAAGTGTAGAATGAGGTTGGAATTTCTAGCACGGGAACTGGACAGCTCTGATGCAAGGTGGAGAGAATGCCAG
AGAAGGGGGTACATGTATGGATGCATTTATATTGTTTCTTTCTGAAATGACGAAGAACCAAAAGCAAAAGTGATATCTTC
TGTAGTACCTAAGAAGCATTTTGTATATAATCACATTAATACTAGTTAAAAATACATTGAATAGAAAGGGAAGAACGTAA
TAAATGATCCAAGAGGATGAACACATGGGCGCATCTTCCTCTCTCAGTCCTCTTCTCTCAGGGTGATTGTTGCTGCCTGA
TGGGATGAAGGAATGAGGACCCCAAGACCCTCACAGCCACACAGGCTTCCTTCACTCACCCTCTTATGTGGGTGATACTGCA
TTCCTCAGGGTAGAGGAATTTTTTTTTTTTTTGAGACGGAGTTTCGTGCTTGTTACCCAAGCTGGAGTGCAATGGCAC
AATCTCGGCTCACTGCAACCTCCGCCTCCTGGGTTCAAGCAATTCTCCTGCCTCAGCCTCCCAAGTATCTGGGATTACAA
GCATGTGCCACCATGCCCGGCTAACTTTTGTATTTTTAGTAGAGATGTGGTGAAACCCCGTCTACATAAACAGAAAGTCT
ATTTACACAGAAAGGTTTGAGGAAAAAGAACCTCTGACAGAATATTCTGAATTATAATTAGGGGTAAGATCGTAAATGTG
TATACTTATATGAGTCTTTCACACACTCTGTATATTGTATGTGAATTATCATATTGGAATCATTAGGCTACAACAAGCTA
TATAGCAACTTAATGATGATAAACAATCTTGAGTACAGTTGGCCCTTTAACAATTCGGAAATATTTTAAAGACATTACAA
CTTAATCCATGGTGCGAACCACTGCTCTAGAAGGCTGAGATAAAGCAATGAACAAGACAAACAAAGTCTCAGTCCTTTGG
GAGTGTATGGTCCAGCGGCAGGAGACCAACAATAAACCAATAAGCATAGAAATGAATGGCTTCCAGCAGTGTTCAATACT
CTTATGTTACTAAAAGGAGTGATAGAGGGATCAGAGAGACAGGCAGACATTGAGGAGTAGGGGAGGGGACATTGCTGTGG
GTTGGGTGGTCAGGGCAGGCCCTATTCAGGAGGTGGCATTTGAAGTTGTAGCTGAATGATAAGAAGTAGCTAGATTGGCC
AGGTGCGGTGGCTCACACCTATAATCCCAGCACTTTGGTAGGCTGAGGCGGGCACATCACCTGAGATCAGGAGTTCGAGA
CCAGCCTGGTCAACATGGCAAAATCCCATCTCTACTAAAAATATAAAAATTAGCCAGGCATGGTGGTGCGCGCCTGTAGT
CCCAGCAACTCAGGAGGCTAAGGCACAAAAATCGCTTGAACCTGGAAGGCAGAGGTTGCGGTGAGCTGAAATTGCACCAC
TACACTCCAGCACTCCAGCCTGGGTGACAGAGCGAGATTCTGTCTAAAAAAAAATTAAAAAAAAAGAGCCAGTTTGGCCA
AGAACTAAGGGAAAAGCATTCCAAGACATGGAAACAGCAAATGCTAGGCTCTGAGGCAAACATAGTCTTGGCATGTCGAA
AGAAGAGCACCAAGGGTGGAGTGAGCATCATGGTCAGGGCATGGTAAGAGGAGAATTCTGAGAAGCAGGCAGAAGCCCCT
CATGTGGAGCAGTAGTTCTCTCAAAGTGTGGTCCCCAAGCCAGCATCATCAGCACCACCCAGAACCTTATTAGAGATGCG
CATTCTCAGGCCCATCCCACGGACCTATGGAATCAGAAACTCCAGGAACTGGCCCAGGCGTATGTGCATTAACAAGCCCT
TCAAGGAAGTCCGGTGCACACTTAAGTACGAGAATAAATGGTGTAGAACCAGGCCGAGGTGTTTGGATTTCGTTCTGATT
GCTATGGGAAGCCATTTTCAACAGGGACTGGCATGATCTGATTTATATTTTGGAGACAGTTACTCTGGAGGCCGTACAAA
GGATGATTCGTAGGCGGGGGCCAAAAGGTTGGAGAAGACCAGGACTTTAAGGAAGGTGAGCTAGAGGCTTAATCCTTCCT
TTTCCTGACTATGTCTCCATGGGCGATTTTCTTAATATCTCTGTGCCAGAAAAATAGGATATGTTAAGGTGGATGGGTCG
AAGAATATCAGTTGTTCTCCTTAGGATTCTACGAGTCTGCACACACTTTTCTTGTCAGCACAGCTGTGTGACTTCTTCCT
GATGTGAGAAGAAAGCCAAGGCCAGGCATCTGAGCACCACCTCAGTTGGCCCGAACACTGAAACAACCCAAAGCAGCAAA
AACTAAGATCAGCCAGATTTTTGCAAAGAAAAACCCAAAACAACATTCAGGCCTGTTCCTTCCTCTAGACCAGCCTTTGA
AATAACAGTGCTGATAATATTATCTGGTCTCTCATGCTCCCAAAGCTCAGAGCTCATTGTCATCCAAGCATGAGACCTTT
AAGGAAAGGTCTGTATTGTAGGTCATCTCATGCTGAAAAGTCATAGAACTGGGTCAGCATACTTTTTCTGTAAGGGACCA
CATATTAAATAGTTTAGATTTTATGAGTCCTGCAGCCTCTGTTACAGCTATTCAACTTTGTCATTGTAGCCCAATAGCAG
CCATAGAACATAATATGTAAAAAAATGAGCAGGCACTTGGATTAGAAGACAAACAATGCCACATAGAAATGAGTAACAAA
TTGGTTTTGATGGTAATGGTAGTAATGAAATAATTCTAGTTTCAAGTAATTATTTTTACTCCTCGTGTTCTTTTACTACT
GTGTTGTATTTTTAATTTAGTCACCCTCTATTTCTCTTCCTTATCTTGATAGATCATTGTATCAGTTTATCACCTTTTGC
TGCATACATAACAGACTGCTCACCTTTCTTTTCTAAGATGCTCTCATTTTTGTGGGGTAGGGCAGGGGCTTGCTCTTCTC
ATTTTCATGTCTCTTCCCTCTCTGAAGTTTCTTTGGGCTTGCTTTTATTTCTTCACAAAAGTAGAAGATAACTCTATTTC
CTGGAGGAATGGAAGCATCTCCCCCATGAAATAGTGTCTCAGTCAGTTTCATGGAAAATGGTAATCCTTGAGATCCATGC
TTCAAGGATGCAAGAAGGACGTCTTATTCATGGAAAAATGTGAGATGCATCCATTAGGAAACTAGACAGGGAAATCTGGG
GTCCCACTAGATTTACAAGGAGCTTGTGGAAACAACACAAGACAGGAAGAAAGTATGGAAAAAATATGGGCAATTAAATT
AAAATATAAGTGTAAGCACCTAGGGGTTCTTGCTGCCAGCTGCACAAAGAAAGAACATGGCATTGTAGTAAAGAAAGAGT
TTGGGCAGGCGTGGTGGCTCACGCCTGTAATCCCAACACTTTGGGAGGCAAAGATGGGCAGATCACTTGAGGTCAGGAGT
TGGAGACCGGCCTGGCCAACATGGTGAAACCCTGCCTTTACTAAAAATACAAAAATTAGCTGGGCTTGGTTGCAGGCACC
TGTAATCCCAGCTGAAAATACAAAAATTAGCTGGGCTTGGTTGCAGGCACCTGTAATCCCAGCTGCTCGGGAGGCTGAAG
```

```
CAGAAGAATCACTTGAACCCTGGGACACAGATTGCAGTGAGCCCAGATTGCGCTATTGCACTCCAGCCTGGGTGACAGTG
ACAGAGTAAGATTCTGTCTCAAAAAAAAAATAAATAAAAATAAAAGAGTTTAATAGAGACAAGGCCAGCCACACCACTTG
AGAGATGAGTTAGTATTCAAGTCATCTTGTCCAAAGCTGCTAGTTTAGGGGTTTTTTAAAGGCAGTTTGAGGGAAGAGGA
GGTGCGGGGTCTAGGCTTCTGCTGATTGGATGGGGCAGGGATGAAATCATGCTGAATCGCTTCTGGGTGGGGCCATAGGA
GTGGGGTTGGCAAGTCCAGGTGGGTCCAGGTGGAGACATGGGTGTGAGGCATGCAAAAAAAACCTGAAAAGATATCTCAA
AAGGCCAGTCTTAGTTTCTACAATGGCAATGTTATTTGCAGAAGTAACTGGGGACATTGCATGTCTTCTAACATCCAGAA
TAATGGCTGACAGTCATATATGTCTGCACCTTAGCAGGACTCAGGCCCTCCTCTCAAACAGAAGTGGTTGGTTTTTGGGC
AATGCCTGTTATCATTTAAACTGTAGCCTAAACATCTCCCAAAGTTACCTTCATCCAAAAGCCCAGGAATAATTAAGGAA
AAGGCAAGGTGGGAGGTGGGTTAGCTTAGCTAACTGTTATAATTTTTCTCACTGATACAATTTTTGCAAAGGCAGTTTCA
TAAGGAACTAGCTGAGGATCTACTTAGGTCTCTCAAGTACTACTGTACTATGCTAAAGAATTAAACACAATGGCTAAAAT
TACAGTGCTGATTCTCTATTGCTTCTTCTCAACCTGCAGTATAAAGAGAAAAATGCTCAATATTAGCTACCTCAAGCCGT
GTCTGTGGGCCGCAGGTGAACAGGAGGGTGTTGGCCAGGGCATCCTTTTAATTAGGGTTGATGAGTGCAGTCTTGCAGAT
CATCAGGCTAGTGGTGAAGAGACCCTAAGGACATCCTGGTTTCATTCTCTTTGCACACCTCTTTGGAATTGCACTCTCTT
AATGCATGTGGTGTAGCCATTGAATTAACAGAGTTCATTCAAGTGTGGACAAATGAACAGTCAGATGAAATATACAGCAC
ATGTTAAAATGAATTCTTTTTTCTGTTGAGCAGTCTAGGGAGAAGAAATTTTTTTAAAAAATCAAGTTAATAATTTTGAA
GTCCCTTCTCGATTGATAAAATTGCACTTCTTTAAACCTGTGCAATTTCATGCCACTGAATTCCCTATTCCTGAAAATTT
TCAGAAATTATTTTTGTCATTACTTGTTTTGGTTGTTTTTGAGAGGGTGCTGTATTAGTGAGGGTTCTCCAGAGAAACAG
AGCCAATGGGATATATAGAGATATATAGGTAGGAAGAGATTTATTATGAAGGATTGGCTTACACAATTATGAAGGCTGAG
AAATCTTATATCGTCTGTCTATAAGCTAGAGGCCTAGGAAAACGAGTAGGGCAGTTCCAGTTCAAACCTGAAGGGCCAGA
GAACCAGGGGAGCCAATGGTGTGTAAGTGTCAGTTGAGTCCAAAGGCCCCAGAACCAGGGGCATCAGTATCCAAGTTGAA
TGTCCCAGATCAAGACAGAGTGAATTCATTCTTCCTCCACCATTTGTTCAGTTCAAGCCCTCCGTGGATTGGATGGTGC
CCTCCTGTATTCGGCAGATCTTCTTTATTCATTCCATCAATTCAAACACAAATCTCTTCCAGAAACAGCCTCACAGACAC
ACCCGGAAGTAATATTTCATCAGCCATCAGCCTTAGCCCAGTCAAGTTGACATATAAAATTAATCATGACAGGTGTTTTT
TAGCACAGACAGTAGTAAATCACTACTACTTTGAGCACTCACTAGGTGCTGGACCTTGCACTTGACAAATGGTATAAATG
ATCAGGAAAGTATCCTGATCTAGACCCTAAGAGAGAGGATTCTTGGATCTCGCCCAAGAAAGAATTAGAGGTGAGTGCAC
AGAGTAAAGTGAAAGCAAGTTTATTAAGAAAGTAAAGAAACAGAAGAATCGCTATTCCATAAACAGAGCAGCCCTGACAG
ATGCTGGTTAGCTATTTTTATGGTTATTTCTTGATCATATGCTAAACAAGTGGTGGATTATTCATGAGTTTTCCAGGAAA
GGGGAAGTAATTCCTGGAACTGAGGGTTCCTCCCCTTTTTAGACTATATAGGGTAACTTCCGGACATTGCCGTGGCATTT
GTAAACTGTCAAGGCAGTGGTAAGAATGTCTTTTAGCATGCTAATGCATTCATATGAACATATAATGAGCAGTGAAGACA
ACCAGAGGTCACTTTCTTCTAGCCATCCTAGTTTTGGCAGCTTTTGGCCGGCTTCCTTAGTGCATCCTGTTTTATCAGTG
GGGTCTTTGTAACCTGTGTCTTGTGCTGACCTCCTCTCTCATCCTGTAACTAAGAATGCCTAACCTCCTGGGAATGTAGC
CTAGTAGGTCTCAGCCTCGTTTTACCCAGCCCTGTTCAAGATGGAGTGGTTCTGGCTCCAGTGCCTCTGATTTAAGCATT
ATTTCACTTAAGCCTCGAAACAACCCTGTTTTAAGTAGGTCTTTTTAGCTTCCTTTTGCAGTTTACAAAACTGAGGATTA
AAATGGTTAAGCATGTTGCTTGAGGTTTCACAGGCTGGTTACAGACTTAGCAGAAAAATGGCCTGCCAGCTAGCCTACAG
TGGATCTCCAAGTATGGTTTCAGACAGCATCAGCAACATTTGTGAGCTTGCTAGAAATGCAGATTATTAGGCCCCACCCC
AGACCTACAGAATCAGAAACTCTGGGGGAGGGTCCAGCCAATCTGTGTTTTAAGAGCCCTCTCAGTGATTCTGATGATGC
AGCTCAAGTTTGAGAACCTAGGCCTAGGGAAAGGTAAGGAATTGTAGACTTACTAGAAGGAAACAAAATTAGATCCATTT
AAAGTTCTTTACAGCTAATCTTCCTCAAACAGGCAACACAGAGAAAAGGGTGAAGACTGTTGAATAAAAGAGACTTCTTT
ATCTAGGCTGAACATATTAGATTTATTGAACCCTTGACACAGTGCAAAGTACTTGACATCAGTCATCTCTTTAAACCCTC
AGGTGGTGGTGAGGGCTAAATGAGATTTTCCACAGTTTACAGATGAGGAAAGAGAAGCTTAGGTCACTTAAGAATTGCTT
GTTAATCAACATGTAGTACGTGATGGAGCTAGCGTCCAGAACTAATGTATTGCTAAAGAGAACATACACAGGTCACACAG
CTTTATGACCCAGAATGGCTGTCAGCAGAGAAACATGTTTTTGAAGATATATATGATGTATAAAATACATAATTAAAAAG
TCTTTCTAAAGTTCATTAATAGGCTGGAACCAAAGACCTGTTTAACTTTTTACTCACCGTTTGCATTACTTATTCTAGAC
CATAAGTATTATACTACTAAGTCTAGGTGTCTTTAGTCTAGTTCTCTTAGGGAAATGAGAATGGGAAGACTGTATTTTTC
ATATAATTTCCCCCACATTCCCCAAGGGAAAGTGTTTACCAGGGCTTTTTTAGGTTTTTTGTTTTTTGTTTTTTTGAGACA
GACGGAGTCTCACTGTGTTACCAGGCTAGAGTGCCGTGGCATGCTCTGGGCTCACTGAAACCTCTGCCCCCCAAGTTCAA
GCGATTCTTCTGCCTCAGCCTCCCAAGTACCTGGGACTACAGGTGCGCACCACCATGCCCAGCTAATTTTTGTATTTTTA
GTAGAGACGGGGTTTCATCATGTTGGCCAGGCTGGTCTCGAACTTTTGACCTCATGATCTGCCTGCCTCAGCCTCCCAAA
GTGCTGGTATTACAGGCGTGAGAGCCACTATGCCTGGCCTTTCTTTTTTTTTCTTTTTCTTTTTTTTTTTTGATACAGGA
CCTCACTCTGTCACCTAGGCTGGAGTGCAATGGCACAATCATAGCTTACTGCAACCTCTAACTCCTGGGCTTAAGCAATT
CTCCTGCCTCAGCCTCCCTCCCGAGTAGCTGAGATTACAGGCAGAGCCACCACACCCAGGTAATTTTTTTTTTTTTTTTT
TTTTTGTAGAGACAGGGTCTCACTATGTTGACCAGGCAGGTCTTGAACTACTGGGCTCAAGTGAGCCTCCTGCTTTGGTC
TCCCAGAGTACTGGGATTATAGGCATGAGCCACTGTGCCTGGCCCCTAGTCTTTAATTTTTGAGGGGGGGAGAAAAACTAG
GTAAACATACTATATGGAAAGATTATAATATTTTCTTTCAATTGATGGACATAGACATAAAATATTGTCAGCGAGAAAG
CCTATCTTTTTAATATTTATTTTGATAAACTAACAAGGAGCATATAATTTATAAAATACTTTCTCTTAAAGCCATAAATA
GTAAGCACTTGCTCCGAAATGCTGCTTTGTCAAGAACGTTAATTGAAGCAGATGAGCAGAGTCAGTATAGCTTCCCAGGT
CCAGCCTGCATTCCCTTTCTACTGCAGAGTTACGCTGAGGCAGCATGCTGGCACCTCCACGGCTCTTCTGCTGTCAACT
TTTCTTCTCCCAAGTACCTGAAAGGGCAGGTGATCCTCTTAACTTTGTAGACATGTGAAAAGGAGACTAGTTTTTGAATG
CTCACCAAAAGCCAAGTTGCCAGGATATTGTTTGCAAATACAGAAAGGGTTTTTGTACTAAGTGGTATTTAAGGCAAAAT
CTTTGCCAAAGAACTCTATCCAAAAGCTTGAAATACACATAGTTGGTTAAGGGAATGCATTTGGAGTTAAACAGAAATAG
GTTCAGTTTTGGCTTTGCTACTCAAGAGATGTGACCATGAGTCAGTCTTAACACCCACCAAGCACTAAGCCAGAGCTTCC
```

```
TTGTTTGAAAAAATGGGAGTGATAATACCTACCTCGCATTGCCATCATAAGGAATAAGTAAGATGGATTTTTTAAAAATGG
AAGTCAGATTATGCCCTTCTTTTGGTTTAAACCTTTAATGGGTTTCTACCTCATTCCAAGTAATTTCCAAAGTTCTTTCT
GTGTCCTGCAAGTCCCCTACATGACCTACCATTTCGCTGTCCCATTGATATTCCCTGAGCATTCTAGGCACACTCTGACC
TCAGGGCCTTTACACTTGCTGTTCTCCCTCTGGACTACTCTCCTCTGTACAGTCCTCATGGTTCACTTCCTCACTTCATT
CTGTTTCTGCTAAAATATCACATTCTCCAAGAGGCCTTCTTACTCACCTACACACACAGGCTCCTGACCCTTCTGGATGT
TTTATTTGTTACATTTATCACTACCTGATAACTTACTCTTTTTTGTTCATTGCTGAAGTCCTTCTAGAAGATAAGCCCTG
AGAGTAGACTTTGTTTCCTGCTGTATCCCCACCAGGGAATTAATTCATCACTCAACATATTTCATAAGTACCTACTATGA
TCCAGCAATTCCTTAGTTACTTATCTGAGACTGAAATTCATTGATATTTCCATGGTTCCTAATTTTTAAGGAATTTTAAA
GCCCCAAAGAACTGGAACTGAAAATGTCACTTTGGAATGTGGAGAAAGGTTAAGCCAGAGACACTATCTGTGGCAGCTAT
GGAGGTGTGTCGCTTAAATCTCCCTTCAAGAGACAGCTGCCCCAGGAGTCCCCTCATTGGACAGTTTTGCTGCAGGGGCT
TCAGCACCCTCAAAATGTCCCTGCCCAGGCTGTACTGAACATGGCAAGCTGCTACAACCGGGCCTTTCCTGCCCAAGTGA
GGCTCCTCTGGTGGGCGTTCTGTGCTCTGCAGCTGCAATCCAGCAATGTTCTCAGCTGTACTGCAAGCTGAGACAATTCC
TCCCCAGTTCTCCCTGCTCCCTCCTCTCCTCTCAGGTGTTTGTTCTGAGGTCTGGTTTGGAGGCTCTCCCTGTCACTC
GGTTTCTCTCCCCTTTCTCTTTCACAGGCATTTGCCCTAATGAATTGCTCACATTTCCAATTCTGTCTTGCCTGTAATCT
CAGCTACTCAGGAGGGAGGCTGAGGCAGGAGAATTGCTTAAGCCCAGGAGTTAGAGGTTGCAGTAAGCTATGATTGTGCC
ATTGCACTCCAGCCTAGGTGATAGAGTGAGGTCCTGTCTCAAAAAAAAAAAAAGAAAGAAATTTAAAAAAGAAAGGCCAG
GCATAGTGGCTCTCACGCCTGGCCAACATGGTGAAACCCTGTCTCTACTAAAAATACAAAAATTAGCTGGGCATGGTGGC
ATGCACCTGTAGTCCCAGGTACTTGGGAGGCTGAGGGAGGAGAATCGCTTGAACTCGGGAGGCAGAGGTTGCAGTGAGCC
CAGATCATGCCACAGCACTCCAGCCTGGTGACACAGTGAGACTCCGTCTGTCTCAAAAACAACAACAACAACAACAACAA
ATCCTAAAAAAGCCCTGGTAAATACTTTCCTTGGGAATGTGGGGGAAATAATATGAAAAATACAATCTTCCCATTCTCA
TTTCCCTAAGAAATGTCTTGGGGGTTTGTTTCTAGATGGAGAAGCCCTTCCCTCAGGGTAAGAGGTCCTCAGGATAAGCC
TCCAGGACTCTGCTGCCATTTCCTGAGGCCGACTCTGAGCTCACTGCAGAATTCTCTAGGTGGTAGCAGTGGTTCTTGTG
TGATGTTCCAGTAATTGCTGCACTTATTTTAGACACAATAAAAAGTGACAGAGCCAGCATTGACCCTAAGAGCCAGTGGG
GACTATGTATCTTCAAGGCCTAAATATAAGCTATTTCAGAAGACAAAAGTCCAACTGGAGTGACGTATCATGCAGCTCTT
GTGGAAAAAAAAAAGCATTTTCAAAAGCTGCATTATTTAAATGAGAAATGCATATATTTAAAGGGAAGTTCCATAAAAGT
CATTTGAGATTCCTTTTCTTTCAATGACTGTATTCAGCATTGTCTATTATCTCCATATATCAATAGTTTAAAATATTTTT
TCATAGACCTGCCTTTTCAGAAACATCTTCTATGTCACTGTATTTTTAATTACTTGCTCAGTGTAATTTATTATGCTGGA
ACTAATTGTGTTTTAATTAGAAATGCTTTGCTAAAGAGAAGAACTTATCAGTTTGCGGCGGTGTTTCCGGTGCAACTACA
AAGCACGTCTCCTGTTTTTCTTAGGATTTTTTTATAAGGGGACAGCCACATAATGTGTTTTTAATATTTGTTGTAAATAT
TTATTAAGAATCCTGTTTACTTGCCTCCATAACTCTAAGTTGTTCCTCTTTAATTTTTCCTTATTTTAGTTGCTAAGGCA
ACTGTTTTGACTACTGTGACAGTTTTCCTGCCCAAGAAAGTTACTTTGAGTGACCACCCACTCCTCTTGAAAACACATTT
TGCTTTCTCCTTCCTTCTGCACTCTGAGAGGTAGTTGTTGAATATTTTAGGAATAATTTCTTCACCTCTTTTCTGGCAAG
AGTCAGGGAACCCTCCCAGAATCTGAGCATCCAGATTTTGCCTCACAATCCTCACAGCAGAGACACTTCAGGGAGGGAAA
ATTACAAACCTAGACAGATCCGGAGTGTTCAGCCATTCAAACTGGCCTGCCTCACAAATCAAGTCCCAAAGATTTAACAT
CTATATCGTCAAGTAAAAGATAAAGTGGGAATCCCTAGCTATTAGTGACCATAAAGTCAATCGTTAGAAACAGTTTGGCC
TCTCCCAGGTGGCTAATCTGGGCATTTCATTTCTCCCCCAGATTCAATCATAGTTAAGCTGATTAAATTCTCAAAGAACC
AAGGATATTTCTGAGACCTTGTATCTAAGTTCTTTCAGGCTGCTATAACAAAATACCATACACTCGGCAGCTATAAACAA
CACATTTATCCCTCACACTTCTGGAGGCTGCGATGCTTAACATCAAGGTGCTGGTAGATTCGGTGTCTGATAAGAGCCCA
TTTCCCGATTCCAAGATGACACGTTCTTGCTGTGTCTTCACACGGTGGGTGAAAGGGGCAGCCGTCTCTCTGGATTCTTT
TATAAGGGCACTAATCCCATTTATCTCCCAAAGGCCCCCACTTCCTAATTCCATCACATTTGGGATTAGGATTTCAGCAT
AGGAATTTTTGGTGGGGTAGAGCACAAACATTCAGACCATAGGACCTTGGTAAGCACCATCAGGCCAAATTTTATTTTAT
TTTTTAAATATATAATATGAGAGATCAGAAAAGATGGTGTACCTTCCTTTGTTTCTTTTCAATAGCCTTCATCCACTCTT
CCAAGCCAGGTCACTTAGAAAGTTGATGATGTTCTTTATTCCTGAGGTAAGAATAAAATGAAAGGGGTGGATTAAATCTT
TCTACTTGACACCAGAATAAGCGTTCTCTGTATCTCACTGTCATTGCTGGAATGAGCACTTTAAATGCTCATGGTTGATT
ACTGAGGGAGAGACGTTTTCATTTTTGTTCCTATTGGTTTTGAAATGAAAGATAGACAGCAGGTTTACCACCTTTTGAAG
TTGCTGAATAACTCACTTCATTGCCCTCTTGTCATCTGAGAACGTGCTCTGTCGTGTCAGGGAGGTGCAGGGCAGAGTCT
CCCCAACCAGGAATGACCTCTTCTGTTCACTATACCCCAACATGCACTTGAAAATAAAAACTGTGTTGTGATGGCTCGTC
TGTGGCAGCAGCTTCTTGGCCTGATTGTAGACTGAGGCCAGCTTAGGCAGAGTGGGATGGAGGAGTTGGAGCAGGAACAA
ACAGAAGATGACCTTCTGTGCAGAAACCACACATTTTCAACAAGTCTAAGTCAACCCAGAGTTACGAACTCTGGGTTTCCCT
TATGCTTAGCTTTGTATTCTAGCTCACTGGAAATAGACATGCACTAATACCATTGCTCTTTTTAAAGATCTGTGTAACAG
AAATGGATTCCCTGTCAAATGTATTTGTATTTCTGCCTCATATTATATATTATCTGCATCCTCTTGGGTGACTTGCCTC
ACCTCAGTTAGGAAAAGTTTTCTCATCTGTAAGGTAGGGATGGTGGTGTTTACCTCATTTGTCTTTGATGGTTAATAATA
ATCAGTACTAGCAACATTATAATGATTATTATTACTATGTTTTATATTAATAATCAATACTGGGCCAGGTGCAGTGGCTC
ATGCTTATGATCCCAGTGCTTTGGGAGGCTGAGGCAGGAGGACTGCTTGAAGCCAGGAGTTCGAGTTCAGCCTGGACAAC
ATAGCGAAACTCTGTCTCTACAAAAAAAAATTTTTTTTTTTAATTAGCCAGCCATGATAGTGAATGCCTGTAGTCCTAGC
TACTCATGAGGCTGAGGCAGGAGGATTGCTTGAGCGCAGGAGTTTGAGGCTGCGGTGCTATGATTGTACCAGGAGTTTGA
AGCTGGAGTACTATGATTGTACCACTGCACTCCAGCCCGGATGACAGCAAAATTCTGTCTCTTAAAAAAAAATCAATACT
GGCCGGGTGTGGTGGCTCACGCCTGTAGTCTCAGCACTTTGGGTGGCCGAGGTGGGTGGATCACGAGGTCAGGAGATTGA
GACCATCCTGGCTAACACAGACAAACCCCATCTCTACTAAAAATACAAAAAATTAGCCAGACGTGGTGGTGGGCGCCTGT
AGTCCCAGCTACTCGGGAGGCTGAGGCAGGAGTATGGCGTGAACCCAGAAGGCAGAGGTTGCAGTGAGCTGGGATCATGC
CCCTGCACTCCAGCCTGGGTGACAGAGCGAGACTCCATCTCGAAAAAAAAATAATAATAAAACAAAATTAAAAAAATCAAT
```

```
ACTAATTTACTTAGTTCAGACTATGTGTGTCAAGTGCTCTAATGCCCAGAAATAACCTGTGAGATAGGTATTGTGGTTGATG
TTGTTGTCTCTGTTTTTAGAGATGAAGAAATGAATATCTAACCACATTTGGATCAGGTTCATCTAAAACCCTAAAGCCTA
TGTAAAATACCCAATTGTTTCTGCTGCTGGTGAGTTGGTTTTTATGCTTACATGTTTCTCTCACCTTCCACAGGAAGCTG
TAATGTGAAATATGTACCACCAGATAAAATAACGGAAGAGGGATTTAGATTGAAGGGAAAAAAGTAAACAAGGGAGAGTA
CATCAAATGAGGGGTAAATTTAGCAGGCAGCAACTCAACAAGCAGCGAAATGATCTGTAAAGTTGAAAGAGGTGAGCCT
CAGATTTGCTTCTGGTGTTCCTGGTGGCTAAAGCAATGTAGAAAACACAAGTTGTTGTAGGGTTCACAGTGTCCATTAGA
TTGTAAGGAAAGCAACAGGTCAGTAGGAACAGAGTTTTTCCTGGTCCTAGGATCTGAAAAAAAATTCTTACAGAATTCTT
ACAAAATACACTCCCCCATAAAAAACTGTAATAAGTACAGAATGTTTCAGTAGTACAGTTGTTCAGTTATCTATGTGGTA
GGATTCACGGGGAATCCATAAGAAATGCAGTAATTAAAACCAAAACACTTCAGAAACCCATCTGATCCTTTGTGATGATT
GCTGGCATCAGTGGCAAGGAGAATAGCAGAGGCTTCTTCAGGTATGACTGCTGAGCAGTTACCTGAGCCGACTGCTGAGC
AGTTACCTGAGCCCACTCCTGAACACTGTCAGTCTTTCATGTGTCAGCCTCAAAGCAGGTGTAGCATATTGTTCAAAACA
TTTGATCTGCATGCCTCAATGTCCTCACTTGTAAAATAGGTATAAATAGTGAAGCAGCGTTGTTTGTCTGGGGTAATACC
CGAGGTTCGTTGTCCCACAGCCATGGAAAGCTAGGACGTGGACACATCAGAGTGAGGTTAAGAGTGGAAGTTTAATAGAC
AAAAGAAAGAGAAGAGCTCTCTGCACAGAAAGGGCTCCTGGAGAAAATGGGTTGCCACTTCCGCAGTGAAATGCAGAAAG
TTTAATAAATGAACTTAAGGAGGGGGTATGTGATTTACACAGGGGATGAAAGATCGGTTGGAGGAGGCATGCCATTTGCA
AGTGTGCAAAGAAGCTGGCCACTCCACCCTAATCTTTTATTATGCAGATGGTTATTCTAGCTGGCTGGTGCCATGTTGCC
TCGTCCTTATTGTACACGTGGTGACAAAGAAAAGGGAAGATGGAGCCTCCGTGTTGAACATACCTGGCTTCCAGGTAGCC
TTCTCTTGGCGCAGCTGCCAGCATTCACCTGTACAAGCTTCCAGCTTGTTTATCTATGTCTGTAGCTCGGTTTTTCAGGC
TGCTCTTTGTTAGAAAAGAAATGATTTGGGGGCTACTTTTATTAAAAGGGAAGCCTTGCCGAAGACTCCTTGTACTGACA
CTATCTGCCTAATAATTTCTTTCTAGCTCCTATATCAATAATAAAAATGGTACCTCCCTCATAGGAATGTTGGGAGGATA
ATAAGTTAATGTAGAGAAAGTACTTAGACGTTGCCTGGCTCTTAGGAAGCACTGTGTATGTTACCTAGCAATAGTAGGC
ATATTACTAGGTGCCACAGGCCTTTTGAATTGCTCATCAGGAGACTAGAACGGCATCTCCCTGTTGGATTCCAGCTGAGC
ATCAGTTACTGGTGGAAGGGGGAAAGGTCTATATTTAACCAACTTAACCCAATAGGAACATCTGTCCAAAGAATCAAAGGC
CATCAAAAGGAATGCAGGAGCTACACATGCTGTTATTTTGTCTGACAGATTTTTTCACAAGGTAATACTTGTAGCACATA
GCAAGGGTCTGTGATGGAGCAGCTACAGCCGTGCCCTCTGGCTCCTCATGTGGAATGTGAGCTGAGAGGCCCAGTGTCCT
TTATCTCCTGGGATCTTAAAGCAGATAGGAGAGGCACAAATGGAATGAACATTCTATATCCTTCCAAGCAAGAGACCAAG
CTCATGAGGGTAAAAGCAACTTAATAAGGACCCATGCCCATCTATTACATCAGTCCATAAATCATTCTGATGGCAGGATG
TTATCACTGAGGGCTTTAGTTTAACTTGAAAAAAATAAAAATTTGTGGTTCTGAGAGAGGCGGTGTTTGTGCAGAATCAC
AAAGGATCTGCTAGGTATGAAGCTTAAAGCATTTCCCTGGAATAAGGAGAGGAAATAAATAAGCCATAAAGCATCTCTTG
GGCTCTGAGCTTTCAGGATCTGATAGAAAGCTAGACCTCTTGGTTAATTCCAAACCAGACTCCACCTGCTTTCCCAGTCT
GAAGGCCACACCCATCATTCTCCCTGGTTTATGAGGTCTCTTTGGTGGTAGTCACGCATAATGTGATTTAAATTGAATGA
GATCCTTTATGGCCTCTTCCTCAGACTCCTCTTTGTTTCTCTCCAACCTTGACAAGGTCTTTTTAACTTCCTCTCCATTC
TTATCAAACCAGGAAGCAAGGAGGGTGAGTCTCATATGTACCTAGTGGAGTCTACCTAGGCAGTGTTCGTTTATTGACCG
TCCAACAATCTTCTGGCGATAGGCAGAGGGGCCCTGCTTGGTCAAGTTTGCCCAAGTCAGACAGGCATGGGCTTTGGAAT
CAGAGAAGCTGGCTTGAATTTCAGGTTTGTTACTATTTGACTGTGGCTTTGAATAATTTAGTCTCCTCTGAACCTTTTTC
CCCCCTTCTCTCTGAAACCGCATTGGCCAATATGGTAACCACTAACCACATTGTGGCTGTTTAAATGAAAAATTAATTAA
AATAAAAAATTCAGCACTTGGTTGCATTACCCACATTTCAAGTGCTCCCTGTGGCTCATGGTGAACGTATTGGGCAGAAC
CCATCTGGCACATTTCCATCATCACGGTAAATCCGTTGGACAATGTTGATCTAGTCTAAAACCTTGCAGAGTTATCATGA
GATTTATGTGAGATAGTATTTGTGAAATACCTCACAACAAGAGCCCTAAAATTGTAGCTATTTTCTCATAAGTACGACCA
TTTAGTCAGCATTTACTTTGTCCCAGGTACTTAGACATTCTCAATATTAATTCTTTGAAAGCCCTGTGAGTCGGTATCAG
TTTTCCCAGTTTACAGCTAGGAAAAGTGAAATATGGCAAGGTTAAGCAGAATAACTGCAAGATTCTCCAGCAGTTGCCTG
GTGAGGAGGCTGACATGCAAACCCAGGAACCAACCACTACAAGGCCTGGATCTCAAAACCACTGTGGACTGCCACCTCTT
CTGAAGGTTTTCTTTCCCAGAAAACGCAGTTTTGTGATGGAATGTGAATGCCTACTCCATTTCACATTTCTCTACGTCCT
AATGTATCTGTCCAGTGACAAACACAGAAAAGCCCCTGGCCAACTCATGTTTAAATAAGGTAGAGTTAAAGCTTTTATGA
TCTGGTCCAAAATAGCTGTCCCTAGCTGGTAATAAGACTTGACTATGCCCTATTTGAAGATGCCAACTCCACAGTATAAG
ATACAGACATTTGCAGTTAACTTAGGAAGTGATGGCGAGAGAGAGGGCAAGAGAGAAAGGGTAAGAAATAACTACAACTC
ACAATTAAAATAAAATCAGCTACCACTTAGCTGTATCCCCATCCAAGGAATTTCTCCACTTCAATCAGAGTATTTGGCCT
GCTGAACTCTCTGACATTCCTTATGAGGGGATTAAACTTTATTGAACAGAATCACTTTAAAAATTGAAATAAAGGAAAAA
TGTTTAATGTTAGTTGATTTGAAAGTAACTTGAATCTTTGAAAAATAGGTTGTGATATTGATTAATGTTGAAACAGCATG
TGTGAGGGAAAATATACTAAAAATAACACACAAGTGAAAAACATACTCTATCTTTGATAAGAGAGGGAAGTGTCTCTGTA
AGCCTGTAGTGGCACAAACCCCTCAGAGTGGGGATCTCTGAGCCAGAAATAGCTTCTAGCAATGGTGATCTCCAGCAAAA
GTGATTCTAGACTCTCAAACAGAATGCCCTCTGGCCTCTGCTTAAGTAGTTTCACAAAAGGTGTTCCTGTCCCTTTCTAG
CCTTGCTCTCTAGTGGCCTAAGAGTTGGGCTGGATGAGCTAACGTGTCCTTGAGCTCTAAGGCGTGACTGATGAATTTTA
ACTAATATGAGAATTAAAATTCTGCCCACCCCCCAAGGAAGTTCTCTTATGGTTTGTTTCTTTACCAGAAAATTCTGTTA
CCATTTAACTGCAAAACAAATAGATTCCCAAAGGAGAAACATTCCAGCTTTCTTTTATGATCTCCCCTGCAGGGCCCAGA
TCTGGAGGAGGATTTTTCTGACAATGGGACTTCACAATAGCCTGGCCTCAATCTGGTCAGGGAGCCATAGCCCACTTTCA
GGTCAGGAGGGATTCATGTGAAATTTAAAACTGCTGTTAGGGAAAAAGAATAGACATGATGTGTCTTTACTCACCAGAG
GGTAGGGTTATTATGTTTGTTAGTAGGAGGTGGTGAGAAACCAAATTAGATTGTGTTTTAGTTCCTTTCAGCTGATTACC
TCTCATCCCTCAGATTTGCTCTGTGAATTTTCATGGGTGGTTTGAAGCATTTGGTCCAAGAACCTTTCAGCCACAGTTGA
TGAGCTGATTGTGGGTTTTGTCGTGAGCCTGGTCTTTAGTGTCAGCTGGGTTTCATTCTAAGATCCCCCTGGAGAATTTC
TATTCTGATAATTGCTTCTCTTAACAACTGATACGTTTTTATTTTATGAGCTGGATGGGCACCTGGTAAATATTTTTTTT
```

```
CCCTTTTTTTGCTTTTGTTGCTGGGGGAGCCCAGAGCCAAGTTGCTATCCTGCTATAACTGTGTAGGACCTTATGACAGGCA
TTTCAGAGAGCTGCCTTTTACTCTGGCCTTAGTTTACTATTCCACTTTATTTTTCTTCTCTTTGCCCAGATTTCTCATCC
TATTTTGAAAATGATTTCTTCACAGTGTCAAACAGTGCTCCTGGTTCAATAGGAATTCCCAGGACGTGCCTCAAAGTCAC
CCCCAACCTCGAAGATGAGTTTGTATGCATTACAAAAAGGGACTCCTGCCTTTAGGCAGACGGCCTCAACCAGTGCCCTT
GTAATTGCACTTCTATCCTTGGCAGTCTTCCAAGGACTTCCTTGTTGGGTTCTGGGCCTGATGCAGCCCCCTCAATTTGA
GCAACTTCTTGTTTATTTGTTTTACATATTGGCCTGCTGCAAAATCTTTTATTTAAAAAAATTCATTTTTTCATTTATTT
TTAAGTTAATTTGTTAGTTTTATGGTGCTTTTGATGTGCTAGGTGCTAGAACACAAAGGTAAACAAGACTGCTTCAAGGA
CTCTGCCCTCTTGGAGGTTGTTGCATTCTGGTGTGGGAGTCAGTCAGTAAATCAAGGATCAGGGAGAGCCTCTCTGAGGA
GGGGATAATGAAGACAAGGTCTGTAGAATGAAGAGGAGCCTGATGCACCAAGGGCAGTAGAAAGAGACTTCTGAGCAAGA
GGAACTGCAAGTGCAAAGGCCCTGATGCTAGAGAGAGCTTGAGGGGTTGCAGGGAAAAGAGTTTTTATGGTTGTGAACAAG
AAATAGAAGGAAGAACAGAGAGTGGCATGAGATAAAATTTGAAGGATCCCAGATCGTTCAGGACCTTTTCGGCCAGGTAA
GGAGTTGGGATTTTATCCTAAATATGATAGGAATCCATTGAATGGTTTTAATAAATTCATTCAAAGTTTAAAGAATCAAT
CTGGCTCCTATGGGCAGCAGAAAAAGCAGAAGTGAGAAGATCAATCCATGGGAGAGATAATATTGGTTTGGGCCAAGGTG
GGTGGTGATGGAGAGGGAGTAAAGTGGGTGTGTATTCGAAATATTATAGAAGAAGACTTGCTACTGAAAGTGAGAGGAAA
AGATGAGGGAAAATTAAGGATGACTCTTAGTTTCAATAAGTGACCAGATGCTAGTGACATTTATGGAACTAAGGAAGTAC
TGTGATGGAGAAACTTGGAGGGGAGAGAATAAAGAGTTCAGTTATGGACATACTTATTTCAAGACATCCTAGTGGAGATG
TCAAATAGCAAATATTTGAACTCACAGAGGAGAGGATGGGGCTGCTCTTACAAATTTGAGGGTCATCAGGAAATAGATGA
TATTGTAGACACAAGATTGAATGAAATCTAGGAACACAGTGGAAATGAGAATTCCCAGAACCAAGTCCTGAGGACTATGT
AAAAGTTGAGTACAGAAGGAAAAGGCGGGAAATTAGACTAAGGTAATGGAGCACAACCAGGATTGGAGAGTGTTTCAAGA
AGGAAGGAATAATCAAACTCTCAGAGATCAACTCAGGTGAGGAAAGGAAGGTGTGCACTCCTATTTTGCAGCATGCTGGA
CTGGCCAGAGAAGTTTTAGAGGGTGATAGAAATGGAAGCCAGATAGAGTTGCTCAGATGGTATGAAGAGAGGTAATGGAA
ACAGTGAATATTTCTAAGATATTATGTCCTGAAGAGGATCAGGGACATGGGGTAGTAAGTGGGGTAGTAAGTAACCATTG
AGAAGGAAATATAAGATTAAACGTCACTGTTGACTATCCATTTGAGGTTTATAATCTTGAATTTAAACTAAAGCCAGACA
GTTCCATTTTGTGATTTTCACCACCAAGTTTGGTGATAAAGTATGCATAAAATAGACTTCGTTTTTTGGCCAAGAAAACA
CTAAATGAGACATGGAAGTTGCGAACCACCTGTGAGGGAGTGATTGCAATATTGGGCTGCTTTGGAAATAAGCTAGATAA
GGATGGAAGTAAAAGGAGGAGGGAACTGGTGCGTAGTGAGAGATTGGCAGGGATAATCAAAAGGGATATTTGACTTTGCA
AAGAAGTCAAATATAGGAGGGGGAGAACTGTAGAGCTAGGGGCTAGAAGGACCAGATGGAATTCCAAGAGTGAGATATTAG
GAATTGAAATGTCCAGGTGGTAATGGCAAGCTCTAGGGGTACTCAGGGGGGAAGGAAGAAAAGAATTTTGTATTTAAGGA
GGCCAAGGAAGTGAAAGTTCACTCTCAAAATAAGTTGGAAACCCATTCTTATTTATTATTTGTATTTCATGAATCATCTC
AAATCCTTACTTGGAATTAGGTGATAGGGTGCACATAAGAATGATAGTGGCTGATTCTTTGTGGATCTACTCTGTAAACA
CCATTTTACGATGTACTTTTGTACACTGTGTACCAAAATTCTTCATTGCCGTCAGGATATTCATTGGGATATATTTGGAA
ATGGGCTAATTCTTATGTCTTGATTAGTTGATAATTCTCATTTTTTTAGTCAGTAACAACATAAGACCCGCCATGGCCTT
TTGGGTAATAGCTAATGCTTTTCAGGTGGCACGGAAGACCCTTGCAATGAAACAACTCTTCTATACATGTCCCATCACAC
CCCCTGTCATTCTCTTGAAGTGCTCTAGCCACACAAACAAATTTCTAATGTGAGGTCAATAGCTGCTCACTCTGCCTGGC
ATGAAGATTCATCTAAAGTATCAACTTCTGGGTTAAGCCTTTTCTAAAAGGTTGAACTGTCCAAAAATGCCATTTTGAAG
GGTCAAAAAATGTCATATACTGGCACTTTCATATTGTTAATAATTTGCTACCACTATTACAAGTCTGAACCATGCATTGT
TGGTAGAGCTATTACTTGCTGAATTGGAATTCTGTGTTATGTGCTTTATGTTCACCATCTCATTTAATTCCAGCAATAAA
CCCAAAAGGTAGGTGGAACTACTTCCACTTGTTAGACAAGGATTCTGAAATTTAGAAAGGGTCTTTCTGGGAGAGAGTAC
ATAACAAATGGTGGAACTGGGTTTTAACTCCTAAAGCAAAAATTATACCATGCCCTTTCCTTTGGTCTTATTCATGGCAA
TGACAGCAGTACAGAGCACACGCCTGATACAGAGTTGGAATACATAAATACATACAGTAATACTTCATCTGCCAGAAAAC
TACCCAAATTGAAGGCTGCTTTGTTCAGGGACCCCTAGGACTACTTTATGATGGCCTAGTATCTTGGCATTGGATTTACC
TTGATAAATGTTGTTGTTCCCCTGTAATTTAGAAAGATAAATTTTAACAGGTGAAACTAAGAGTCTGAACGGCCCTTAAA
ACCTTAATCCTGTAATTACTATTTATTTGTTTTAAGGCACTGCCTTGATCCATGTTTGCCAGACAGGTTCCTAATTGCTG
GCATTGAAATGATCTAGGAAGCCAGTTTCTTCAGCTCATGAAAAGAACATGTAGATTTGTTGAAGTGTGAATGCTAAGTA
TTGGTGTAATGCAGTCAGAGGGCACAGGACATCTATGTTCATTTCCCAAATGCCCTTTTTCTTTCATTCACATGGATTTT
TCCTCTCATTCCTAAGCACAGTGGTGCTCTAATAAGACAAGAAGTCAGTTGGCAAGTGTAACACAGATGCTCTGATGCCT
TGAATTTGGCTAATATTGCTTCTTCATTCAAACGTCATTCTGTAGCTTTCCCTGAGTTTCTGGTGTCAAAATTCAAAGCA
ATGGTTGTCAAACTTTCGTGTGTGTTCGAATCTTCTAGAGGGCTTGTTGGAACACAGATTGCTGGGTCACATCCCCCCAG
ACTCTGACTCAGTCCATCTGGACTGGGGCCTCATATTCTGCAATTCAGTAAAGCTCCCAGCTGATGTTGATGCTGCTGGT
CTAGTGACTACACTTTAGCTCTAGGGGCTGTTCAAGATGCGCTTTACAGGACACCAAATGAGGTTAGATAAAAAAGCAAA
AATCTCAGCAAATATCTGAATGCTAGATTTTACAGACAAAAAAAAAAAGAAAAAAAACACAAAACAAAAACCAGTGACT
TAAAAAATAATGTATCTCCTGATTTTTGACATCAGTGGGCTAGAACTTGGGAATCCTTCTTCCAAGATATTAAGGAAACT
TTCTTCTCTATGACTTTCATGCTTTCAGTCAGTATGCTTTGTTCTTAAACCTATCCTAGAGCAAGATGTTTTATTAAATT
GATCAGTTTTACAATACAAAATTGAAAGCAAAGAGTTTTCAAAATGAAACATTCCATAATACAATAAGAATTGTGGATA
TTACATACAAAAGAGTGATTTCTAATTTGTTTAGAAGCTCAGCAATATTTTCATTTTTATTAGATTCAAAATGGTGTGAG
TCATGAAGATCAACACATTTCTTTTTGACAGGAATCCTTTGTGTTTGAACCCAACTGCCTCTTCAAAGTGGATGAGTTTG
GCTTCTTTCTGACATGGAGAAGTGAAGGCAAGGTATGGCCCAAGAAGAGCAAGTTGTTCTTTCTGCTTTGTGGTTTAAGC
CATTTTGTTTCTCAACAAGTAGACTGAGTGCTGTTGATGAACCCTACCCTTATTCATTCATTCAAGATTTTTAAAAATGT
GCCTACATGTGCCAGGCATTGAAGTATACAGTGATGATTAAGGACATTATCCTCATAGATTCCAGTCTAGGAGCAATGTC
AGGCATTAAAAAATAAGTTACAAGTCATTGAATGTTGTGAAAGAGAACACTGGATTGTCAAAGAGCTTAATTTAATCTTG
GGAGTTAGAAAGAAATCTGCCAGAAGAAGAGATGCTCAAGCTGAAATCTAAAAGGTACATTAAAAGTGTGTTCATCTCAT
```

275

```
TATACCCTGGGACTTAATTCGATGTATGGTACAGTGTCCACATTTAATGAACAATTGAGCTGAATTTAAGAAAAGAATTA
AAGTTATATAAAAATTAGCATTTTTCTCACATTTCATGTACACACATGTATTTAATGATTATTAACAATGTTTTATATAT
AAAAGTAACAGATTTTAGAAACATTTTTATATTCAGCATTGCACATATATATGTCTATATGTATGTATATATATATATGT
GAAATGGAATACTTGGGGTCTTGATTATTTCTGATTATTTACAATAATTATTGTTACATGGTTTTAATTCCCTCTTCAAG
CTGACTGTAATCAGCACTGCCTTCATCATCAGAAATTTTTGCCAAGCAGTTTGCCTGTATCATAGGTATGGCATAATTTA
AGCTCATTGCTGTGTTGTATTCTCTCTCTTCAGGAAGGACAGGTGCTAGAATGCTCCCTCATCAACAGTATTCGGTCGGG
AGCCATACCAAAGGTACCTGTGATTGGTATTTGCTTTTCTAAACACGGATTTACTTATATTGGAAATGGCCATGGCTTCT
AGAGATAAAAAACTCACTCTTTGTATCCAGGCTACTTAAAACATTTGATCTCAGTTTTGAGAATAGCATGTGGTTGCTGG
TTGTGGTAATGTCACATTTATTCACATGAAACCTCTGTGACTCTCCTCTGGTGAGAGAGAGGCTTTGTGGACAAGCAGAC
CCTAATAAAAGGCTCTTCTATCCAATTGCCATATTCCAGGTCCTCCAATAACTACAATCAAAACTCTAAACAAAGACCCT
CATATCTAGTTTCTAAATCAAAAATTTAGGCCAGACACAGTGGCTCACACCTCCCAGCACTGTGGGAGGCAGAGGGAGGC
AGATCCCTTGAGCCCAGGAGTTTGAGACCAGCCTGGGCAACATAGTGCAACCCTGTCTCTACAAAAATTACAAAAATTAG
GGTGTGTTAGCATGTGCCTGTAGTCTCAACTACTCAGGAGGCTGCGGTGGGAGGATCCATTGAGCCTGGGAGGCAGATGT
TGCAGTGAGCTGAGAATGCACCACTGTTCACCAGCCTGGGTGACAGAGCAGAGACTCTGTACCTCCCCCAACCAAAAAAT
TTGTAATACCTTTCTTATCAGTTGATTTCAAGGAATATATAGTGTTACAAATTTCACCTCTACAGGTTTTAGAATAATGC
TAGGTGTCTGGGCCCTTATGTTTATTTTTACATTAAATGTGGTTTCTTTTTACCACGTTTCTTCCTCCATGCTCTGTGAT
GTAACTTATTTTTTTTTTCTATCTGTGTACCTCTATACAGGATCCCAAAATCTTGGCTGCTCTTGAAGCTGTTGGAAAATC
AGAAAATGATCTGGAAGGGCGGATAGTTTGTGTCTGCAGTGGCACAGATCTAGTGAACATTAGTTTTACCTACATGGTGG
CTGAAAATCCAGAAGTAACTAAGGTAGCTTCAGTTAAATGGCCTCCTTCTCTTCCCCACCATGTATATATGTTGTGTGTT
TAATTTTATGCGTGCTATATTTTTATATACTTAAATTGTATGGTAATTTAAAAGTTAGCATTGCTTTGCTGACATGTTTC
TGTAGAAAATCAGCAATTAAAATACCCTTTTAGCATGTCACTCCTGTGGGCCAACAACCCAAATACCTACTACGATATGT
TGTAGGGATACTAGTCCAGCACTTCTCCAACTTGAGTGTATACCAGAATCCCCTGAGCTCTTGTTAAAATGCAGATTCTG
ATTCAATAGGTCTGAGGTGGGGACAAGATTCTCCTTTTCTAACAAGCTCCCAGGTGGCATGGATACTACTCATCTGACAA
CTACAGTTTGAGTGACAAAGGTCTAGATCAGTACCACCCAGTAGAAATGCAATGTGAGCCACATATATAATTTAAATTGT
TCTTGTAGCTGGGTTTTAAAAGTAAAAAGATACAGGTAAAATTAATTTTAATAAAATATTTTATTTAACCCAGTAGACCT
AAAATATTGTCATTTTAATAGGAGATCAATATAAAATTTATTAGTGACATATTTTACATTCTTTTGCTCATACTAAGTCT
TAGAAATCTGATGTGTATTTTCAAATCAGACTAACCACATTTTAGGTGTTCAGTAGCTGCATATGGCTAATGGCCACCAT
ACTGGGTGGGCAGTACGGGTCTGGATGGTTAAGAAATCCAAACCCCCCCTCCCAATAAGTTATTAAAATATTGGGTTAAG
ATCAGTCTTTTAATACCATATCCAGCATACCATTGCCCTGGCAAGTGAGTCTCAAAGAGACTTCTCGAAGAAGTAGAAAG
CCTCGTAAATATTTTGTCTGCTAATCATGTGTTTACAGGGTGATTTTACCTCTGTACAGAGCTCCCATTATCTTTCTGTA
ATGTGTGTGAAAAGGACTAAGCCTTCACTTTCCATGGCAGAAACATGGGTTTGGGGGAATGGGGAGGCATTTTGTCTTCT
CATTAACCCTATAATTGGAAACTCAGTGTAGGTTGAATGAGAGAGGGTTCCAACAAGTGGAAACTCAGTGTAGGTTGAAT
TTCTAATATCATTAGAAATATTGAATTTTAGAGCTAACAGGAAATGTACAGGGAAGATCATTTGAGGACACTGAGATTAC
AAAACTTAACCATGAATCCAGGATAACTTATGGCAGAATTGAGTTCTGTTGACCCCTAAACCATCCAAGCTCCATTACCC
TCACTCACAAAGTATGGTCCTCATTCCAGAAGTACAGACCCCTCCTGGGAGCTTGTTACACATTTGAGTCTCAGGCCCAC
CCAGATGTTCTGATCCTGAATCTTCATTTTTGACAAGATGCTCCAGGTGACTCGGATGTGTGGTGAAGTTTGAGAACTCC
TGTTTTGTAGCTCTGGAGGCAGAAATGGATGCTCGATGTCACATAAGAGTGTTTGGTTCTGTCCTTATTGTGGTGGTTTT
ATGCTATCGTTCAGGTCACCTCACTTTGGGAGAACAGATTTTGCAGTGCCTTCAAGTTCTTTTATTACTCTTCTGAGATC
AACTCCTTCTCTAATTACCAATTCATATTACAAGAAACAGGATGATGCAGTATGAGCTCAAGTATTGGGGCTTTGGGGCC
ACATTGCCTGGGTTCAAATCCTTGCTCGTCCCCTTCCCAGCTATGTGAAGCAGATATACGCTGGACCTCTCTGTGCCTCA
TTTTCTTCATCTGTAAAATGGGAACAGTACTAGAATATCTGCTCTTAGGATGATTGTGAGGCTTACATGAGTTATGACGT
GGGAAGCACTTTGTGTACTGCCTTTCTTATTGAAAGCCCTTAATATGTGTTAGCTATTAGGATGACAGAGGCCAAGAATT
TTAGAGTTCGAAATGGACCTTAGGAAGGGCTTCTCACCTCATTTTTCAGATGAGAAAATGAAGCTAGAGAGGTAAAGGA
ACTCTGAGACCACACAGCAAGCTGGGCCAGAGTCCAACCTAAAACCAGGTCTTCTCATGCCCTATATGCTGCACCCACTT
TGCTCTGTGCCCTGAGCAAATACAGTCCTGGGGAGATGGCTCAAATCAGTAAATCCCATAATGTTTTTGCTTTCCATAAT
TTGATTGCTGTTAGTCTTGGCCCATTCTAAGAACCACGTTCTGAAACATACCCTTGAGTAGATTATGGATGTGTACTGTT
GGCCCTTGAACAATGTGGGGGTTAGAGCTACCATACCCCTGTGCAGTCGAAAATCCATGTATAACTTTTGACTCCCCCAA
AACTTAATAGCCTACTGTTAACTGAAAGACTTATGATAATATTAACAGTCAATTCACATACTTTTTGTATGGTATATGTA
TTACCTATTGTATTCTTACAATAAAGTAAGCTAGAGAAAAAGCAAATGTTAATAAGAAATCATAAGGAAGAAAAAATAT
ATTATTCATGAATTGAAAGTGGATCATCATAAAAGTCTTTATCCTCATCATCCTCACGTTGAGTAAGCAGAGGAGGAAGA
AGAGGAAGAGGTTGGTCTTGGTGTCTCAGGGGTGGCAGAGGTGGAGGAGGTGGAAGTGGAGTCAGGAGAGGCAGGCACAT
TGGTGTAACTTTTACTGAAAAAAAATCCATGTATAAGTGGGCCCACATAGTTCAAACCCATGTTGTTCCATGACCAGTGA
TATATGTTTGAGGGTTGGGGGTGGGGGGATGTATACTTCAAGGGAAGATTTGAAGGTTTTCTCCTAGTATTATTGCCATT
GAAATTTAAGAGCAGCTAGCCAGTTAAGAACTAAAAAATAATTCTGTATTGGATTTTTTATTCAAACTCAATACTATTTG
AGCCTGCTCTTACTGAAGAAACTAAGTGTTTCAGCTTCTGGGAACTATTACATCGAATTTCATTAATAGCATAATATATA
AATAAATATAAATCTCATTGTCCAAACTGGACAGTGCATTGTCCTTTCGGTTGATTGACACTGGAGAAAATATTGAGTAC
ATTTTTCCACCAAATATACCTGCATGTGGATACTCTTGCCCCCTTCCTTATCTCAAAACTTAAAATAACCATATTTACCA
CCTTCAGCATTGACAACATTTTCTCCATCACCACCGTTTTATTTCTATTCCTCTATATGTGTTTCTATTCCTAATAGAAC
ACACAAGCCATCAAAGTGAAAGTCTTTATATGAAATACACATAGATATAATTACCTCAGTTCGAAATGATGGCAGGATAC
ATAAATCAAATTGGGCCAATGAGAAATGTAGAGATTTCCCAGGGTACATGAAATAGTGTTTGTGACTCACTGAGTGGTTC
TACTCAAACGTGAGTATAGCCTAGGAGCCTAGAATCAGGGGGCCATGAAATATACTAACAGCCCTCTTACTTAGCCCATG
```

```
CAATGAGAAGGTGCATTTTTGGTCAGTTTATCACAAAAGTCAATTCGAGCAAGTAATCATTTTTTAAAATTAATTCATAT
ATACCTTAAACACTGTAACTTAAGGCATATAAATGTTGTTCTATTGTTGACTAATCTTTTAATGTTAGGGACAAGCCTTT
TCATTAATTGTGGATCTGTTGCGTGGCTTTTATGTTTTGGGTTTTTTTTTTTTTTTCCCCCTGTACTTTACTCTGAATGC
CTCAGATAGCCAGTTTGGTTTCTTTAAGGTGGATCTGAGACTAAGGACACTCATTGGGCAGAGTGCAATGGAATGCAAAA
TCCTACAGTTCTGTGACTTTTTTCCCTAATCTTTTAGTTATCTTGTCTACATGGAATTTGAGAAAAATATTTTTGGCAAC
TTGCCTTTAAAGAAATCTTCCCAAAATATTCAGCTAGAGAAACAACAATGCCCTTTCTTTCCACAAGATCGTGTCATTGG
TTTACGCAATTTTTATGACAACTGACAATAAATAGGTTTGAAAACAAAAGTAACCCATGGTAGACATGTAAGGTGAGAGT
AAAAGAGTACAAAATATTGGAAAGTGGCTTGCTGGCTGGGAGAGTCCAGCACTCCCTGGGCAATCTGTGTATTTTGTTTG
TCTTATAAACACCTGATGAGTCTTGGTCTGACAAGTGAAATGTAAGTTAAGTGAGTTTCTAGTACAGAACTTGCTTATGA
GTATCCTATGAGATTCATGAGTCTTTTTTTTTTTAATTCCTAGGCTCTTATTACACTTAAGATCTGACCATTTGGAATAA
TTCACTTAACCCAACATTTAATCTAGGCAAAAGGGTAAACTTTGAACTTAGTGTCTTTTATACCAAGGACCATGTTCCAT
GTGGAGGAAGCTTTCCTGACTTTCAGCAAGCCATTTTCCCTTCTGTATTCTTCTACTTCTCAAAGAGGGTCTCTGATAGT
TTTTTTTTCATATTCAAGTGGGTGCTTTAAGAGTCAATAAAATATTATCTGAAACCTGAGTTTCTCAGGGTTGAATTTTA
AATAAATACAAAATACTTTCCAGTGCATATGACTCTGAAAATATGATTGTGCTTTTTTCAGTAAAAGATAACAGCATTGT
TAAGAGACACCTTGGTAGCTCACCTTGTCTTTTCTTCAGCTTCTCTCTCTGCTTTGAGAGTAAATACGTTTGTGTTGCCA
CTCTTACCTATTTAGGAGATTTGTGCTCAGTTCTATATACCTTTCTCCTCTACCGGTAAGTCCCAAATCAAAGCTTTCAA
CTCTTCTCACCTGCTTCTCCCACAGTCATTTTCATCTCAACAAATGTGCTCTCAGTTGCAGAGACCATTCTCAGTTGCAG
AGACCATGAACACCTTCCTCTTGTGTCCTATGTTAGTCTGTTCACAAATACTGTTTGCTCTACCCTCAGCAGGTATCCAG
AATCCAGCTGCTCCTCACCAACGTCATCACGACACAGTGGCCCAGGCCAATGGTCCAGGCCAATATCATTTCTTCCCTAT
TGTGCCCCAAGAGGTATCTAACTGGTCTCTTTTCCTTAGCCTTGCCCATTTTGGTCTAGTCTAAAAACAGCAGCTGGAGG
GATGTTTTTAAAATGTAATTCAGATCACATCACTCCACTGCTCAGAACCCTCCAAGGCCCTGCTAGATATCATCCCCACC
TCCCTTCCCCTTTCTACCTTAATCTCTCTCTTTCCTCCTACGGCATTTTCTTCCAGCCTCTGGGACTTGGCATTTGCTGTT
CCTTCTGTCTGTATCGTGCTCTTTCCCAGTTACACACAGAGCTTTCTCCCCCACAGCCCTCACGTGTCTGCTGGAAAATT
GTCTTAGCAGGAGGCTCCCCATTTCACCAGCCTTCGGCACTTCCTCACTCCTTTTCCTGCTCTGTGTTTCTTCTTAGCACG
ACTTACTTACCATAATATCCATTTATTCATTTATTTTCTTCCTCTGTCTGCAGGACGGCACTGTTTCTGTTCACTGCTGTA
TCTGCAACATCTAGAACTGTCAGTGCCCGACACTTAGTGAGTGCTCAATAAAAACCTATTCTATTGAGGTATTTATGTTG
ACTAAATGAATTCCAACCCAAGTGATAAAGGTGTATAAACCTTAATGTTGTGCAACTCAAGTTTGTACAGTTCAGTTGTT
AGAACAGACAGGAAGATGATCCTTTGACAACAAGACTGAGAAAATGTGATTCAATGATAGTCTAAAGGAAAGACAGAACT
CTGGGATCCCGTGTGTTATTTTTAGAGCCACATTTCAGGATGTAATAGCGCTGCTGCTAAGAATCCATGACGAGACTGGA
GGTTTTGACCCTGGTGTCCTGGCCTCTTTCCAGTTTGGATCATTATGTCCTGCTTACCTAAATCCTTCCTGTCGTTACAG
TTAGAGAATTTAGCTTTCATTTCCTCTCCTAAACAACTGCCTGTTAAAAAGAATTACTAACAAATTGCTGTCATATTTAC
CCCAAACCTAGTACACCTTTCGCTAATAGGCGAGTCTTATACATCATAAAGCTTAAAACAGTTAAGAATAAATGCAGCCT
ACTAAAAAAAAACAAAAACAAAAAATTTACCCAGTGTGATGGCAGATGCCTGTAATCCCAGCTACACCAGAGGCTGAGTC
AGGAGAACTGCTTGAACCCAGGAGGTGGAGGTTGCAGTGAGCCGAGATTGCACCACTGCACTCCAGCCTGGGCGACAAGA
GTGAAACTCCATCTCAAAAAAAGAATAAATACAGCCAAATGAAGGTATTTTCCCATTTTTTTCTAAACTGAAATAAAGCT
CTTTTTTTTTTCCTGGAATCATTCTCATCTTTTTGCTCACTGTTTTACAAGGCACTATAGAAATAATTGAATATGAGCAT
TCTAATAAGTTAATTTCATTAGAAAAGCAACTTTAGTTCCTCTCATGTTTTCTTTTGTTTTTTCCTCGCTCAGAATACAA
CTAGCTTGGAAGGATTTTGGTGTCTAGCAAAGTTGAAGGCAGTTCAGATGTCTTTCATTAATAAGTTACAGCTGGCACTG
CACAGCAAACTGAAATCACAGTCACTAGACGTAAGATGGAAATGCAGGCCTCATTCAACATCTCCATAAATAAATCTGTT
ATTCTATACCAAGTTGAATTTTCCTTTGATTACAAAAAATAAGCTGTTATTATGACGTTCAAAATGCAAATATTGGGATT
GAGTTTAGACCAGACTGCTCATTATACTTTATGCAGGTTTGTAAATATTTGGCAAACTTCATAAATTATGCCTTGTAATC
AAGTCATCAAATAAGCATGTAAATAACTACTATAGAAATTAAATTACAAAAATATTATGTATTCTTTTGTGTACAAAAAG
ATAAAGTCAGGTTATTTGTTACCAGTAATAAAATATTATTTTAACAAATTGGGCTGAAAGTCAAAATTGCTTTGCTAAAC
TTGAATTGTGTTGAGTGAAAGACTTACTGCAATGGTTCCAGAAGAAGGAATTTATTCTTTGTTTCACTAAAAAAAAAATT
GATTCTCTATTTAAATTGAGTCCTCCTGGCTTTGCTAACATCCTGCAAAGCCTCCAAGCCCTGCAACCAGTTCCTCTACT
GATATTTAACCACAGCATTACCCCAAGGGATCAGAGGCTTCAGCCTCGTCTGGGGACTTCTTAGAAATGCTATTGCATTT
CTTAAAATTCAGTTTTAATTATACTGTATAGGCCAGTGGCTCTCAATACCTGAGAACTTATTAGAGATGCAGATTCCCAG
GCCCTGCCCCACCCTACTGAATCAGAAAGCTAGGATTTCAGCGGCCTTCCAAGTGATTCTTATGCACACTCAAGTTAGAG
AATCACTGCTAAGCAGAAAAGCTCAATCACTAACATTTATATCCTGCTTTACTTGCAGAGGCCTTAAAGAACTTACATTA
ACTTAGTGGTCTGATGCCCAGGCTCAACTGAGCTGATGCCTGCTGAATACTAAAGCAGCTAAATTGTAAATCTGAACTGC
CTATTGATCACCCAAACCCCAGTATACTAATTAATAAAAGCTTCTTCACCACATTGCTTCAACAGTTTTTGAGGCTCAAA
TGTGGGATGAGTGTGTAGGTCTCTGTGTTAATTTTTGTTTTGTTTTGGGTTTCTTTAACGTTTCCAAGACTGAAAGTTGA
CATTTTCTTGTTAATTGTTTTTCTTGAAGCTCTTCTGAGTTCATTGGAAACAAATCAATGAGGGGGGGCATTAAGCTGTC
CGGAGTGCAGAGCTGTGAATCAGAACAAATAATTGAGTGCATATTCTTAAGTTTAAATGTCCCATCCCACCTAAGAGTTT
TCTTTTTGCCAAATAAGGATAATGGGTTTTATTGTTATTTCTATTGCTGGGTTGAGTTTACGGACCACCAGTCTCTGCCT
TACATGGGCTGTGGTCATCAGAGAATCTGTGGAGCTGGTGAAATCAGTCTCCTGGGGCCTCCCATGACCACCTTTTTCCT
CTGTGGCTCCTTCTTCCTTGCCTGCCAGTTCTCATTTGCTCTTGCAAGACCATCTCTCCTAGCAGCTGCTACCTCAGTGT
CTCATCCGTGATGGCGCTCTCTGAACTCTGAATTATTTATTTGTGCATCACCAAAAGTGATAGTTCCTTTAGACCAGATT
TTGTGAACTCAGATGCCTGTGGGAGCCAGGGAAATGATGTGAATAGCTGAAAACATGTCAGGCAAAGACAAATACGGGAG
GGTGTGGACTTTAGCAAGTTGGAGTGCACATACGCTGTTGAGGGGGGGCACTTTCACTTATTTCCAGCTAGTTTTCCAACT
ACCAAAACATGGGCCTGAGGTTTCTAGATTGTTTTCATGTCCATTTAATACTAACGTGGATTTTTTACATGAAATCTCTT
```

```
GATATTAAAATGTTGGCAACTCATTTTTTAAACATTGTGCAAAATGACAAACATGTCTGTGGATCATTTAGAACCATAGC
TATAGACATCAGGCACCATGTAGCATTAACACACTTGCACTAAAGTGAATGCTAATATTGACTTATTGTGTTCACCTTAT
TGGCATTTTAAATGAAAGATACCTGTTTGTTCCTTTATTTCTTCATTCAGCTATTCATCCAGGAATATTTTTTTGATGAA
ACCTGAACTTGGACAAATCATTTCAATTATATTAATCTTTTTAATCCTCATTTGTTAAAGAAAGGAATTGCACCATGTGT
CCTTTGAAGTCACTTTCTCTTATAAGACTTAAGGAAGGACTGGCTGGGCATGGTGGCTCACACCTGTAATCCCAGCAGTT
TGGGAGGCTGAGGCAGGTGGATCATGAGGTCAAGAGATCGAGACCATCCTGGCTAACATGGTGAAACCCTGTCTCTACTA
AATACAAAAATTAGCCAGGCGTGGTGGCCCATGCCTGTAGTTCCAGCTACTCAGGAGACTGAGGCAGGAGAATCGCTTGA
ACCCAAGAGGTGGAGGTTGTAGTGAGCCAAGATCACGCCACTGCACTCCAGCGTGGCAACAGAGTGAGACTCCATCTCAA
AAACAAAACAAACAAACAAAAAAACTTAAGGAAGCACTCTCTTTGCAAATAAAGTGTACCAGGAGCCAAGGATGGTCCA
GGTTAGTTGCTTTGACAGGACACTTGTGTACACATGGAAATCCCTAGATAAGCTTAACCTGGACTACATCATCATGACAC
CAGGAGTCAGCTACTAGGATAGGAGGCTTTGTGGTTGGAGAGGTGACAGAAGTCATTCTGGGTACTTCCTGTGGACATTA
AAGGTTGAGAGATCCTGAGATAGAATGCAAGAAAATCAGTTGTTCTCGGCTTCAATCAGTTACACAAGAAGGAAAGATCT
GAGAAACATTCAGTGTGGAATCCAGAGGTTTTGTTCCTGGGTAAAGTTAGAGAAGCTAAAATAGAGACCAGATTCTGATT
CTGATTAACTGGGAGTACGATGATACAGCTAATCAGAAATTGAGGGTCTGGTTACTTTGGATCAGGGAGAAAAAGGAACA
ATGAATTTGTTTAAATTTTTTTCTCTGAGGAACCTGTTAGTTGCATGACCTGCTATGGGTGATGCAATAGGACAGAGATC
ATATACTACTTAGGATCCTTAGGGGCTTGTCCATGGGGCGAGCTCTGTTCTGTCTCTGTGGCACATCTCTAGGCCTCTGA
AATCCTGAAAAGGTCTACGTTTGTTTGTTTGTTTGTTTTGAAACTCCCTACTTTCATAAGGAATTAAGTGTGGAT
TTTTAGCTGGCATTTTTCTTAGTGCTGGACTTACTTAAAAGCTCCTGGTCAAAATGATTCTTCAGGAACATGCAGCTGAA
GCTAGAGGATATTTGCTGTTGTTTTCGGGGAATTTTGTCACCTGTATCAGTGAAGTTATTTCTTTGGATGAGAGTATCAA
AGATGAAAGAGGCCTCAAGCCTCCGCTTAGCTCCCAGGTTCTCTGCAGCTGCACTGACCATGTTGACAAGGTCATTTAAA
CATTTGCATGGACAGTGTGTTTTTATACTGTTGACTGGTATTTCCTGAATGCTAAAGGAATGTTGCTCATGATAGAGGAT
GTAGTTGCATGACATTCTATGCAGAAAGTACAGGATAGTAAAAAAAAAAAAAAGAAAGAAAAGAAAAGAAACTAAAAAT
CCCCTAGAGTGTATTTCCCCTTCTGTGTCTTTTCTTAATTCAAGTGAAATTTATGTAACATAAAATTAACCATTTTAAAG
TGAACAATTCAGTGCCATTTAGTACATTCACCATGTTGAGCAAGCACCACTTCTACCTACTTTCAAAACATTTTCATCAC
CCTCAAAGGAAACTGCATGCCCAATAAGCGGGTTCCTCCCCATCCCCTCTCCCCACAGCGCTGATAACCACTCTAATGAG
TCTTTTTATTCCCCACACACTTTCCTTTTTATATGCAGTTGGACTCATGCTGTTTAATATACTGCATTTTATTATATGAA
TTTTCCTGTTTAAATTAAAACTTTTTCTTAGGTTTCAATGTAACCGCAAATCCCCATGATCTGTGAGGTGGATTTTCATG
GGCGCTCAGCTCTTGCTGTTTTAGAAATCATCCAGCCTCCTTCACAGTTGTGTTTGAAATGCTGTGCTCTTCATGGGGCA
CATGGTTTTCAGTGCTGGAGCCTTTCTCACCACTGCATTTCATGAACTTGACAGGTTTCATAAGAAGAGACTGTAGACCC
AGCCCAGAGAAGCTCATTTTGTCCTAGTTGCAAGTGTTATCAAATGACTCCACCAAGGGAATGCAAATGAATTACTCTA
ATTGCACTAGAAGCATAATGTGAAGTTTGTATTTCACCTTTTGGCCCCTGGGAGAGAAAAATGTCACTGACTTTAAATTG
CTTTTCCATTTTCCACATAGCCCCAGTTTCCAGTGCCAGCAAAAAATTGCCATTGGAAACATAATGTATGCTGACATGGG
CCTACATGTATTTGTATGTTTTCTCATCTGTGTGGCAAAATTAAAAACTGGATGGCATGAAGAAGTGGGCAGAATCCCTT
GTATTACATGAGACTTTGTTGCCTCCAAGCTCTATGATCTGGAGCCAGTAATTTAATGATCATAATCTGGGGTCGGCAGG
TGCAGGCCATGGGCTGAACACAACCCTTAGCCTAATTTTGTATGTCCTGTGAACTAAGAATGGCTTTTACATTTTTGAAG
CGATGTTTAAAAAATAAATTTAAAAAAAAAACAGGAAAACTAGACAACATGGAACTATGTGTGCCCCTGAAGCCTAAAAT
ATTTATTATCTGGCTCTTAATGGAAAAGATTTGCTGGTCTCTAATAATAATACTAATAATAGTAGCAGCAGCAGCAGGAA
AATAGTTTATATTGTATGTCAGACACTGTTCAAAGTGCTTGATATGTATTATCTCATTTAACTCTCACAAGTCTGTGGGA
TAGGTATTATGATTATCCTCATTTTACAAATAAAGATGCCCAGAGAGGTTGAGTAACTTGACTTTTCAAGGCCATACGTC
CAGTAAGTGGTGTTTTGAGCCCAGGCAGTGGCTCTCTCTAGTGCTCTAGATCCTGAATTCTACCACCACCCTGTGCTGCA
ATTCACAGTTGGCTTCTTTTTCTTTTTCTCTTTTTTTTTTCTGAGGCAGAGTCTCACTCTGTCCCCCAAGGCTGTAATACA
GTGGTGTGTTCTCAGCTCACCACAACCTCTGCCTCCCGGGTTCAAGTTATTCTTGTGCCTTGGCCTCCCAAGTACCTGGG
ATTACAGGTGCATGCCACCACGCCCAGCTAATTATTGTATTTTTAGTAGAGACCGGGTTTCACCATGTTGACCAGGCTGG
TCTCAAACTCCTGGCCTCAAGTGATCCACCCACCTCGGCCTCCCAAAGTGTTGGGTTACAGGCGTGAGCCACCGTGCACA
GCCAGTTGGCTGATTTAGCTTATCTGTATAAAAACTGGGGCAGGGAGTCGGGGGAACAGCCTCTTTCACCTGTAACACTG
TGTGATGTTAATGTACTTTTTAGGCAAATGTTTTAAGATCATTATCTGTGGGAGCTATAGTCATACAGTAGTACTTCCCG
AATTAGTGGCCTGAAAAAAGGATTCCTTAAAAAAAGTCCACATATAAAGGGGGTTCTTTGGTCAGCTAAGCATGAGAAAGC
CTTTAGACAGGTTTCTTTATGGTAGTGCTTTCAGAAGAAGTATTTCTTTTTTTTTTTTTTGGAGGAGCTGGGTCTCAGAT
ATATTTGAGTCTTAGTGCTTTACTCTTTTTTTTTTAAGTTTTAGGGTACATATGCACAACGTGCAGGTTAGTTACATATGT
ATACATGTGCCATGTTGGTGTGCTGCACCCATCAACAGAAGAAGTATTATTCTTATGTGGATCTCAGCAAGGAAGAAAGC
TATTCAGCTGTTTCCTAAACTTTTTTTTTAATTTTTTGATGAGACACCATTAACATCTTTCAGAACCAGTGTGGCACTGA
ATAAAAGTCAGTAAATTATATCATAGAGGCATTATTCAGCTCAATATTTTTCTAGAAGTATCAAAACAATGATGTAATCT
AAGGATTCATTTATTCATATTTACCATAAATATTCTATTTATTTTCCCTGTTTTTCCACTTCCACTTGTCTTCACTCCTC
ACTGTTTTCTGCTGTGTGTGGGGGAGACTTTACCTTAAAAGACAGAGAACCAGGTAATGAAACTCAAGCCCACCGATTTAG
ATTCCTTTTAGTAGCTCTTATATTGAAAGTCCTATTTAGTTTATAGAATTACCTATAGATTTGATTTGCTTGAACTAAAT
TGGTGGATGCTGCCTAGTTAAGAGTTAGACAAAGGCTTCTTGATTGATTATATTGATCTCAGACTAAGCAGGTATGATTG
TCATCAAGTTGAAATACTATTTACTTTGGCATGACTTAATTTGCCAACATTGTCAACAGGTACCTGTCAGGAAGAATGTG
ACCGATAAATACAGGCCATCAACTAAAGAAACAACTCTATGAACACCTTTTAATTCACCAGCTGTTTACTCATCCCATAC
CAACAGAATTTCACAAATTTGACTGAGTTGAGTCCAAAAGTAATGCTGTCAAAAAACTGATGGTTGCTGACTGGCTTAAG
ATGATGCTTTTGGTTTTCTCAACATTTTAAAATCCCTCCATTCTTCTAGAGCTTTCCATTTGGATGCCAAATAATGTTAC
ATAAAATAAACCAAAAATCTTATTTTATTTTTGAAAGATTTCTGGGGATGTAGGAATGTAGTTGGTCTGTGGACTTTCAG
```

```
GCACTAAAGAACATCATGTGTTGTATATCTAAATCAGAAGCACAAGTGAATATAGGCATTAATGTAAGTATACTCTATGT
ATACATTTTTCCCTCCTGATCTTTTGTGTCCATAACAGTATATTTATTGTAGTAGATATTTGGAAATAGATGCAGTTATT
TGTGACAAGATTCTATAGATCCAATAGGACATTGCTACAGGGTATCTATTAATATATAGAATCAGGCTTAGGCAATGCAA
AAATTGTGTCTAAGGAATGCTAACAAAGCCATTTGGTATGGAAGTTTGGGACTGCAGCTTCTGAACAGCACAGGTGTCCT
CTTTTTTCTTGTGACTATTTAGAGGCTCTTTTTGACCTAACTGGCCAGTGTTGTCAAGATAACCAGCAAGGCTGATAGAC
TGTTTTGACTCTCTGGGAGTGACTAAATTAATTTTGTCTAGGAATGTATAAATATTATCCTCACCCATTCAATTATCTCT
TTCAAACATAGCCACCCATACTCATAGATGGCAGCTTATTTCTTGCACAAATACTCATTCACCATGCTCTGAGGGTAATT
TAAGAATCATATGATTTAATTTTGTCTAGGAATGTATAAATATTATCCTCACCCATTCAGTTCTATCTTTCAAATACAGC
CTCCCATGCTAATAGATGGCAGCTTCTTTTTTGCTCACATACTCATTCAGCATGCTCTGAGGGTGATTTAAAAATCATAT
GACACTTAATATATTTCTTCTTTCACTTTCATCAATTTTACTCTGTAGCTTCAGACTGTCAAGAAAAAGAGGCCCCAGTT
GCTCTCTTTTTCCCCCTTTCAATTCTTCTCTTTTAGAATTTGAAAACTTTATGAGGATTATTATTGACTTCCCAAGGGCT
TTTGAAATTAATGTTGGGTTGTCTTTTTAATTTTTGTGGGGGCAAAAGTCCTGTTGCTTTTTAGCCACCAGTTTCAGAAG
TAGGTTGCTGTCAGTCATTATCACAGGCTCACTGATGTAGCTTTCTATCCTGGGTTTATCAACTGGACTTGTGTAGGATG
GCAGCTTAGCTCCTTTATTATGCAGGAAAGTCTTCACTGTCTTTTTTCTTGCACTCATCTTTCTTGCAGCAACAGTTCAT
GGTGAATTGCCCTCTTGTCCCATGGGCATGTGGGCGTTTCCAACACTGACATTGATATCTTGTGACACTAACACTTAGTA
CCTGCACTTGTCTGTCCTTGGTGGAGCCAGGAGAAATCAGCTGACATGAAAGAGAGAACTTGTTTAATTTTTACTTCTTA
ACATGATTTAGAAGTTGGTCTGTTATACAAACAAACATTAGTGGTTTTGTTCCTTTTTAATTTTAGGCTGGATTTAGTGT
CCTAGATGAATACCTTTGAAAGATAAATGTTCATTTGTCAAGTCCTGCCTGTACCATCAGCTTCTCCACTCTCCACCTC
TGCCACTGGGGTTTTATAATTCCCAGGCCTCAGACCTGGACCAAATGCTGTGGGAATGGGGACTCAGGCTTTGGGAACGC
TGGCCCTTAGCCTCTGGCTCCTCCTGCTGCTCTGCTGGCTCTGGCCTTGGTCCTTAGGTCTGGACTGGACAGTTGGTGGC
CCAAGTCATGTTCTGATATACCCCAATTTTAAGAAGTAGACCTCTGTCTGGTTTCTGTACTGGCCCAAGTCATGTTCTGA
TCATGTACCCCAATTCTAAAAAGTAGACCTGTGTCTGGTTTCTGTACTGAATCATCTGGTAAGAGTTATCCCAAGAAGCC
CAGATCTTCCAGACCAGAACTATCTCAAGCAGGAGGAGCTGTAACTTCCAACAGTCTTCCTCTGGGCCAGCTCCCTGCTG
CCTGATGAGATCCTGGAAATCACCAGCTCCTGAATTCCTGTGGCTGTGAAATATCCTCTTACCCAACCTCCCTGCAGCT
GCCTGCATTGCTTTTATGTTGCCTGTAACTTTCTTTCAAATACTTCAACACATGCAGTGGGGTGTGTGTGAATTTTCATC
CAAAACCAGGGAATGAGAATTTAAAATTTTAAATGCTCACCCTAAAGGTAGTTAGTTAGTCGTTTGAAAAATTTGAATCAA
AGCTTAAAGATCAATCATTATATATAAATTATAGCTCAATAAAATTAATTCAAAATAAAATAAAACAAGCCTACCCTCCA
GTGGCCACCTACACACCCCCTTCAGTTAATTTTCTCTCTCATTAGCACCAGGTGCTCTGATACTAGCCTCCTGGAGTGGT
TTCTACATGATAGACACCTGGAGGCTTGTTAAAATGCAGATTACTGGGCCCTGCCCCCCAAGTTGCTGATTCTGTAGATTT
GGGATAGGGCCTGAGAATGGGCACTTCCGGTAAATTCCTATGAGATGCTCAACCCGCTGATCAAGGGAACCACACTTGAG
AACCTCTGGCATAATGCAGACTGGAACTACCTTTTCTTTGGTGGAGTTCTTCATGGTATTCAAGATATGCAATTTGTGTA
GAAATAAGGTACAAGCCCTGGTATCCTTGTGAGCCTAGGGCACAGGAATCTGGCAAGCCCAAATGCCTGCCTGGTATCTG
TTGGAAGCTTGTTAGAAATGCAGAATCTCAGTGCAGATGCCAGGCTTACTAAATCCAACTCTGTGGTTCACAAATGCCCC
CAGGTGATCCAAATGCACATTAAAGTTTGAGAAACACCTGCCCAAACCAAATTTAGTTCACATCATCTAGTTCAATTGTC
CTCAACTATAGGTTCACGTTAGAGTTACCTGCAGTGTTTAAAAAAGCAGATTGAAAGACAGCCCAAGTACATTCTAATTA
TTAGTTAGAATCTCTAGGGTGAGACCCAGGCTTCTGAAATGTCTTATAATTCCCCCAGGTGATTCTAATGTACCACAGGT
GCTGGGAACTACCAGTCTAGTCTCTCTAAGTGCTATCCGCATAGTTCTTTTAACAGTGTACTAGCTGGAAATGTCTTCCC
TTTAGCCTGCCCTCAAAGTTAAATACTGACAATTTAATGCCAACTAAACCTTCTCCTAATTCAAATGAAATGATTTCATT
TGCCATGCTTTTTAAAATTTTTTTATTTTTTATATTTTGTATTATTATTATTATTATACTTCACGTTTTTAGGGTACATGT
GCACAATGTGCAGGTTAGTTACATATGTATACATGTACCATGCTGGTGTGCTGCACCCATTAACTCGTCATTTAGCATTA
GGTATATCTCCTAATGCTGTCCCTCCCCCATCCCCTGACCCCACAACAGTCCCCAGAGTGTGATGTCCCCCTTCCTGTGT
CCATGTGTTCTCATTGTTCAATTCCCATCTATGAGTGAGAACATTCGGTGTTTGGTTTTTTTTGTCCTTGTGATAGTTTA
CTGAGAATGATGATTTCCAATTTCATCCATGTCCCTACAAAGGACATGAACTCATCATTTTTTATGGCTGCATAGTATTC
CATGGTGTATATGTGCCACATTTTCTTAATCCAGTCTATCATTGTTGGACATTTGGGTTGGTTCCAAGTCTTTGCTATTG
TGAATAGTGCCGCAATAAACATATGTGTGCATGTGTCTTTATAGCAGCATGATTTATAGTCCTTTGGGTATATACCCAGT
AATGGGATGGCTGGGTCAAATGGTATTTCTAGTTCTAGATCCCTGAGGAATCACCACACTGACTTCCACAATGGTTGAAC
TAGTCCACAGTCCCACCAACAGTGCAAAAGTGTTCCCACCCCTCCACATCCTCTCCAGCACCTGTTGTTTCCTGACTGTT
TAATGATTGCCATTCTAACTGGTGTGAGATGGTATCTCATTGTGGTTTTGATTTGCATTTCTCTGATGGCCAGTGATGAT
GAACATTTTTTCATGTGTCTTTTGGCTGCATAAATGTCTTCTTTTGAGAAGTGTTTGTTCATATCCTTTGCCCACTTTTT
GATGGGGTTGTTTTTTTCTTGTAAATTTGTTTGAGTTCATTGTAGATTCTGGATATTAGCCCTTTGTCAGATGAGTAGGT
TGCGAAAATTTTCTCCCATTTTGTAGGTTGCCTGTTCACTCTGATGGTAGTTTCTTTTGCTGTGCAGAAGCTCTTTAGTT
TAATTAGATCCCATTTGTCAATTTTGCCATGTTTTTATAGACTCATAAGAGGAAACATGTTGGTGGTGATTTATCTACAG
TCCCTATTTATTTTAGAATCTGAGGCACTGGCTATATTTCATGGCAAGCTAAAACTCAAATGCCTTCATGAAACTAAATT
TCTAGCTTAAGATGAAGGTGTAGTTCCTGCACTGAATAGCCTTCATCTGTAGATTTGTGAAATGTTGCAGTTTTACATCT
AAGCACCTTATAAGGAATTCTATCTTAGACAAGATTAGATCCTGTGGCAATCTTTATTTGAAAAGCTTCATAGTTTGCTC
CAAGTGCATGGAAATGTGTCTTATTTCTCTTGAATGCCCTTTAGGTCTTAACAGAATTCACAGTGCCAAGGAGTCAAAAA
CTATATTTCAAATTATATACATTCCTGACATATTATTTGAGCAGATTAAAACTGATGGATAGAGCAATTTAGGCACCTTG
AAAGTAGCTACTTGAAAAATGCCAACTTTTTCTTATCTCCATTCACTTAAACAAAAATGGGCCAGGTGCAGTGGCTCATG
CCTGTAATCCCAGCACTTTAGGAGGCCGAAGCAGATACATAGCTTGAGCCCAGGAGTTTGAGACCAACCTGGGCAACATG
GCCAGACCCCATCTCTGCAAAAAATACCAAAATTGGCTGGGTGTGGTGGCTCACACTTGTGGTCTCAGCTTCTGGGAGGC
TGAGGTGGGAAAATCATCTGGACCCAGGGAGGTCAAGGCAGCAGTGAGCCTTGATTGTGGCCACTGCACTCCAGCCTGGG
```

```
CAACAGAACAAAACCCTGTCTCAAAACAAATAGAAAAGTAAAACAAGAAAACAACAAAATTAATTGATCTAGATTTGTGA
GTTGTCGATACCAAGAAGTTCCCATGTTTTAAATATTTCAGATGGCCCCGTCATTGATTATTCTCACAGCTCAGATATAG
GGAAGATGAAAGCTGCTGCCTATAGCATCTTGCATCCTCTTGCTTCCACATACAAGAAATGTGAACTTCAATCCCCTAAA
AAGGTCAGAGTTTCCATTCTAGTGAACACATTTGTTCAGCCCAGGTGGGGTGGGACATGACAAAAACCAAGAGTGGGTCT
AGAACCATGGGGCTCTTCCCAGGGCTATCACTATGAGATAGAATATCCTGTGTGTAGTCACTCTCAGAAACAGCAGCTCC
CTTAGGAAACTGCTGTGTCCTGAATGCTTGTGTCCCACCAAAATTAATAAACTGAAATCCTACCCTCTAAGGTGATGGCA
TTAGAGGATGGGGCCTTTGGGAGGTGATTAGGTCATGAGTGTGGAGCCCTCAGGAAGAGGATTAGTGTCCTTATTTTAAA
AAGGTACCAGATAGCCCCCTTGCCCTTTCCACTGCATGAGGACATAGCTAGAAGGCACTACCTATGAACTAGAAAGCTGC
CACTGAATCTGTTGGCGCCTTGATCTTGGATTCTCCAGCCCCCAGAACTGTGAGAAATAAATTCTGTTGCTTATAAGCT
ACCCATATATTGTATTTTGTTGTGTGTCAAACAAAGACAAAACCAAACTCACATCTTATATGGGCAGTGGAAGGAGAAC
TCCAAGTTGAGGGGGGCTGCTCTTTTAGACCTATGATTCTCAAATTCTAATGTGCACACCAGTCATCTGGGGATCATGCT
AAAATGCAGATTCTGATTCCATGGTTCTGGGATGAGACCTGGGACTCTGCATTCCTAACAAGCTCCCAGATGATGTTGAT
GCTGTTGGTCTTGGGGCCCTTTGAGTAGTAAGGGTTTGCAACAGTGCTTCTTAAATGTGTGTGTGCAAAAGAATCACAGG
GAAGGCTGATTAAAACGTGGATTCCTATTTCCCATCCTGAAGATTGATTCAGCCGGAGGCCTGATAATTTGCATTTTAAC
AAGTTCCCAGGTGATCCTGATGCTGCTGGTCCCCAGACTACATTTTAATAACCAAGCCTCTAGTTAACTTTTGAACAACTT
AATTAAGATACCTTCACTAGTAGCCTCTTCTGTGAAAACAGAGAGCCAAACATCAAAAGATGAAGGAGGATGCTACTGAG
TCCCTTTGGAGAAAATTACATCACAAACTTGGAGAGTGCCACAGGGTCTCTGTTCATTTTTCCATTGACAATATGCTTCT
TTGACTAAAGACGAAAAATAATTCTTCAGGATCAAAACCTAGAGATAAGATTGGAACCCTAAGATTGGAAGTAGTGCTGC
CTGATGAATAATAGGACTAGTTATTTTAGGATGACGGGCCAGCCTGTGCCTAGTGAAAGCTTAGAAATGCCAATAGCAGA
CTGAGCACGGTGGCTCACGCCTGTAGTTCCAGCACTTTGGGAGGCCAAGGCGGACAGATCATCTGAGGTCAGGAGTTCAA
CACACCCTGGCCGACATGGTCAAACCCCCTCTCTACTAAGAGTACAAAAAAAAATTAGCCGAGAGTGGTGGTGGGCACC
CACAATCCCATCTACTCATGAGGCTGAGGAAGGAGAATCGCTTGAACCTGGGAGGTAGAGGTTGCAGTGAACCGAGATCC
CACCACTGCACTCCAACCTGGGCGACAGAGCAAGACTCCATCTCAAAAAAACAACAAAAAAGGAAATACCGAAAGCAAA
ATGTTATTGGTGGCCAATTCTTAATATACGTCATGAGATTTGGGGTGATGTTTACTTCTTACTGTGTATTTCTTTGCTTT
ACACTTTCCTATGAAGCATATTTAAATATTGTTTTCATAAACTGGGGGAAAAACCCTAACCAATATTTTTAAAAGAAAAA
AGTAAAATATATGCTCAGTGTAAGTTCATAGTATTTTTGGTTTGTGTTTGTTGTATCTGTGTCTCTCTACGTTTGTATTT
TCATGGGAAAGGGATTATTTTTGTCTATGTCAGTCCTAATGTTCCCGGGGCAAAGGTGATAGATGGGTAAGAGCAAGGTC
TAAACATATGCCTGGAAGTTGGACAGGGCCTGGGGTGGTGAAAATAAATTTAAGGAGTCCCAGAGGAGAGGTCAAAAGCT
CCAGGAGAGAATTAGAGGCAGCTTCTCTTTCTCTCCTTGCCCTCCAAACAATTCAGCACAAGCCCTCAAGTCCCTTGAAG
AGTCTTTCTTTCCCCCTTCTTCTTAGAAGGGCTGAAACACACTTTAAGTTAACTTAGGAATGGTTAGAAAAGGTTAAAAA
AATGGGAGAACAGTAGCAATTAACAAGCTGCTAGGGAAACAGGGAAACCGTGCTGATCCGAACATCAGAGCCGACCTCCT
GTGGTCTGGTTCCCTAGCACCCAGGCTCAGGCTTCGGCTTGGGGTGGCCGGACCAGAAGGAAAGTGCCCGTGTGGCAGA
AGTCATCTCAGGAGCGTGTCGCCATCCAGCCAATGACTTGTGTTCCAAGGACTGGAAAATGCGCTTCCTGGACACTATGT
AGATGATTACCACACGAGCTTATTTTCAGCAATTCAAGAAAGAATCCAATTCGTGGTTTATTGCTTCTGTTAGTCTGGAA
ATGGTCAAACAAGTTGATAGGCATCTAGTTCACGCCTGGCGTAACTTTCCAACAAAACTCCAGGGCTCTACTGACACCTG
CTGGCATTTCATCTACTTTATGTTAAAAAAACCCCAACAAGACAAAGAATCATTAATATTTTTCCTGCAGTCCTTTGCCC
TTGTTATGTTACTTGTACTTTACCTGAACGATAATAGAAGGGGCCTTTACCATGTCGTGAGGAGAGAAGTGTGGGCAAGC
CATTGGGTGAAAATAAAAATCCCCAACACGAAGAATGTACCACAGAGCTCTTGTTGCCTGTCTGCCCTGCACCAGCTGCT
CTGTGACCTTAGGCAAGGCCATTGACCTCACTTTCATCATTGCAAGAAGAAAGAGGCAGCAACCTTCCCTCCCAGTTCCC
TTTAGGGCTTCTGAGGATGCTGGTGGCATTTCCTACTAACTAAAGCATGGGCCCTGTCACTGGAGTGATGGCCTTAAAGG
TCACTCTCACTGACCTTTCCATCACAATAAACAAGATTCCTTGCCAGCGTTGTATGTTCAAAAGATGAATGAAAGTTATT
TAAGCCTCTTCAAAGTGCACCATCTCTGTAATTTATTTCGACAAACAACTAAATTAGAATGATCAGAAAAAGCCCAGTGA
ACAGTCTCAGAAATTGTCCACTTTTTTTTTTTTTGTCTAGGAAACTATGGACTTTGTTTTGTGTTTTAGAACATGTAGTA
AGCCTGCAGGTTGAGAAAATGCATCCCAGTTAATGCCAACCCAATTATTTGTGCTTTTTCTGTCTATTCTTGGGAAACTC
TGTGTGTGGGCCTGTGGTTTGTTAGGTCAATAACAGGATATTTGATAGTCTGCAGGGAGTTATAGTCCATATTTAGCCTGAC
AGAGGAAGTGAGTTCTGCTGATGAAAAATAATTGAGGCATTTCTTTAATTCTACTATTTCATTTCTTCATCTTCCTTCAG
AATCCTGACCCCACTATGATGCTTGCTGAGGCTTTCGATGCTATTTACAGCTCTGCTTTTGCTAGTCACAAAAGAAAATT
TACCTCTTATAACCCCTAAGTCTTCCCTCAGAAAACTGTTAGGTGTGACCAGGATCAGCAAAAACTGTTAGATTCACATC
CATTAGTTTGGCTACTACTTAAAAAAAGAAACAAACAAACAAAAAAACCCAGAAAATATCAAGTGTTGTTGAGGATGTAG
ACAAATTAGAACCCTGAGCAAAAGGGTTCCCTTGTTGCATTTGCAGTCCTTTGTTGGTGGGGGAAAAATGGTATGGCCAC
GATGAAAACAGTATGGTATTTCCTTTAAAAATTAAAAAATAGAATTACTATATGATCCAGAAGTTCCACATCTGGTTAT
TTATCCATAAATAAGAATTGAAAGCAGTAACTTATTTGTGTATCAATATTCATAGCAGACATGCAATTCACAATGGACAG
GGAGTCCATCAGAGGTGAATGGATGAGTGTATTATTCAGCCTTTAAAAGGGAAGAAATTCCGACACATGGTAGAGCATGG
ATGAACTTTGAGGACTTTATGCTAAGTGAAGAAAGCCAGTCACAAAATGACAAATTCTGTATGGTTCCATTTATATGAGG
CACCTAGCATAGTCAGAATCACAGAGACAGAAAGGAGAATGGTATTAATATTTATCAATGGCTGGGAGGAGGGGGAGATG
GGGAGTTATTTAATGGATATGGAGTTTCAGTTTTGCAAGATGAAAGAGTTCTGGAGATGGCTGGTTATGTTGGTTGCAT
AACAACATGAATGTACTTACTGCCACTGAATTTTTCATTTAAAAATGGTGAAGGTGATAAATGTTATGTTATGTCCATTT
TTAACACAATTAAAATTAAAAGAAAGGTTAAACAGCAAAAAACAAAACAAAACCATACTCTGTTAGAACGCAGTCATCAC
CTTTGCCCTTACAGAGATTAAATTGCTTGACTTTTTAAAACCTGGTTCTACTGGGCAGTGCTGTCCAACAGTCCCATCTG
TGATGATGGAAATTATCTGCACTGTCTGAGCCATCAACCCCATGTTGATGTTTAAGCATGTAAAATGTGATTAGTGCAAA
TGAGACACTGAACTTTATATTTATTAAATTTAAATAGCCATATGTGTCTAGTGGATACCATACTAGACAGTGCATATAGA
```

```
GTATACGTCTATATTACTATCTATTCCAAGATATTTTTCCTTCCTCAGCACTAAGCCGCAAATCTAAGCAGTCAGGCTGT
AAACCTAAGAAGGTAAGGAATCAGCTTATGTGTGGGCCACCGTTCTATTTAACCCAAATGTCAATTTGCTACGAAGTGTT
TTGCCGAGGGTATGATTCTTCTATGGCATTCATTGGCATCAGTTTTTGGACCCAAGGGCAGAGTTGGCCTTAATTCATGG
GCTGAGCTTTTCTAGTAAATGCCCTTTTAGAGACCCAAATTCCTCTACTGCCATGCACAATTTCCCTTCACTTGTCCTTG
TAAAACAGTGGAGTGATGGTATGAACTACAAGTGAGGTCCAAGTCCTTATACAGAAATTATGTGAGCACATCGCTGTTTG
CTCTGATCACCCTGGCAATGAAGAATGTTTTTCTTTTTAAGGCTTTGCTGGAAAACATAGGTAAGATAAGTAACAAACT
CTTAGATTGAAAGGATCACACAATAAAATTGATGAACCCCTATTCATTTATCCTCTAAAGTGAAAATAGTTAAATATGTC
ATGTATTTTATAATGCTGGTTGGAAATAATTAAGATTGCCAGCAACTGAAAAAGGGCTTATATTTAAACAAGCTGCAAAT
AGAAAGCTTCACAAGTCAGTCAGTGGGCAACATTCCTTTGTGGCAGCCCAGAGTTTAATGGCACAATAGATGGCACATTT
GTCTTCATGGAAAGAATGAAAGCTGTTTATTAAAGAATAAAATATGAGCCTGCAAAATATAGATGGCTGTGTGACAGGAA
AGGGACAAAAGAAGAGTTTGTGTTTTTATTACACAAAATGGATACAATCTATCTAATAAAAACTGCTCTATTTGACTCCA
GCAGATTTGCTCACCAAGAATATTTTTTCTTTCTCTTTCAGCAATGGGTAGAAGGCCTGAGATCAATCATACACAACTTC
AGGGCCAACAACGTCAGTCCAATGACATGCCTCAAGAACAGTGAGTGTTGTTTGCATTACTCGTTTTTCTTGGCAGTTA
TCAAACAAATGGTCAGATGAAGAGATGAAGGCTAGGTGGTTCCTTCCTCCAAATTCCTGCTTGCCTTTCCCTCCATGCTC
CTGACCTTGGAGTTGACCAACACACACCTGTACTCTTCATACCACGTAGAAAGGCATGCATGATAAACCCATAGCCCAAA
ACCTATATGAAAAGAATAGTTGATGGTCAAAGGACACTGGAACCTCAGTCACTCTGTGATGCTGTCAGTTGTGGGGTACTGG
CTTTTGTGTCCCCAGATTGAGTTTTCTGATAGACAGCTCTGCATGTCTGGAGCCATCGCTGAGTGCTGGGGTAATTCTAT
CCTCTGCTGCATAAAACTGAGAAATGAGGAGTGTGACCAAGAGACGCACTTGCCAATGCACCTGAAAAGAAGCCTCAGC
CCCAAGTGCAGTGCTCCTTGTTTAGCACACCGTCAGGGATCCAAAGTTACCTCTGAATTTTTTTCTGTAGGATTCAGTTA
TTTAAAGATTTGATCAAGGATCCAATGGTGATTTCCAAATTTATTTTTTCCATGAAAGAAGGAATTACTCCAGCTTACAG
TATCTGAAATCCAGAGGAGTACTACAGTGGTAGATTTAAACCCTACCCAAGTTAACCTTTCAAGGTGACATGAAAATCAC
CCACAAAGGAATCCTAGCCAAAGTCAAATGTCAGCCAGTTTGAAAAGCAGTGATCCGTGAAGGTGGGGGCCATCTGCCTT
CACAGGGCCATTGGCAGGCAGTTGTGAGGTGCCAAGGGGAACCATGTTGAGAAATTGCAGGGATAAGCCTGTATCTCTCA
AAATTATGCCCCAGCACCAAGAGTGTGATTCATCATACTCACTTGGCAATGGGCCAAGAGGTTAGTCCTAAAGGTACTGT
CATGTCTTATCTTCTGGTCATTGTGGCAGGTGCTTACAGGTGTGGTCTCTAGGCTATAAATCCCGGCTTCACCATGGCAA
ATTCTTACACTTGTGCCTCACTGTCCTCATCTGTGAATCAAAATTACCATGCAGATGAAATTGGAAAGTATTCTTTATTC
AATACTACTTATCTTGTTATGGTATCCCTCCAATTTTCTTTCACCCTTTTAGTGGAGGAAAGTTATTTAGTCTTATCTAT
TTCTATGTGTTGTGCAGCTGCTTCTTCTTTTCCTCTGCAGTTGCCCTGGCATGTGTTTTTAAAGAGAAACCCAGTGGATA
TTCTGATGTTATGACTTCAGGCACTGATAGTCCTAAAGGGTCAAAAACTTTTAGAAGGCTTGCTTATACCTGAGTGACAC
AAATATCTCTATCTGACATGAGATCTAACAACATCCTGCCCAGATCCCTTTATGAAGCTGGTGTTCACCCAGCCATCATC
TGCCATGAGAATGGGCTCACCAAGGGCTCAGACCTGTCCACTTCTTCAGAGCATTGCCCTCTGCTGAAGGGGAGCTTCCA
TTCCTGGAAGATTATACTCTTCCCTCCTCCAGGAGGAAGTCCACAGCCAATGATGAGCTGCAACAGGGTACAGAAGGCCA
TCCCTCTTGCCGCCAAGTCTGGCACAAATCACATGAGTTCAAGCTAAATCAAGCTCCATCTGAAACCACAACTTTGTTTA
ACTCTTTTTTCTGCTCAGTCTTCTCTCACTCTCTTCATCCCTAAAACATCCCGTTATTAAAGTAGTTAGTGGTGCTTCTA
CGAAACTTAAGACAGAAACTTTACTAAATCATCATGTTCTGCAAATTGTTAGCACATTATTGATTGGAAACAGGAAGAAT
GGGAGTCATTAGCTAAGAAAAGTCCTAGCTCCAAAGTACAGATAAATGTTCTGAGTCTCCTAAAAGCCTGGGGAAAGCAA
GGAATTTTTCACCATCAATTTGAGTTGATGAACTAGTCTTACCACTGGAAATTAAGGAGGCGCCCATGTGATCAGTACTT
GGCAGCACTAGGAGCTGGGAGTGATGAGTCCTCATGAATTCTCAAGACAAGGCTGGCTGCTGTCACCGTGCAGTTGCATT
TCCCACCACCTTAATATATTATTACAGAGCAATAATCGAGCAGACCAATCACAAATAAGTACCAACAATGTGTTTTGCTC
AATGGAGGCACCCAGTCATCTCATTTAGCTAGGGTTGCCAGATAAAATAGAGGACACTTAGTTAAATATGAATTTTAGAT
AAGAAACAAAATATTTTAGTATAAGTATATCCTATGGAATGTTTGGAGCATACTTACAGTAAAAGAAAATTACCTGTTG
TTTTTCTGAAATTCAAGTTTAACTGAGAGCTCTATATATTTATGTGCTAAATCTGACAACCCTAAATTCATCTTATAGCA
CATAATTGTCTGATTTTCAGAACCAATGTTCTTGATTTAAATGAACTTTCTTTTAAAGTTCTTAACAGAATTGAAGCTGC
TCCCTCTAAGAAACATTAAGGCAATGTTATTGCTATTGTTTTGCAGCTGGATGAAATTGGCATTTATGACCAACACAAAT
GGTAAAATTCCAGTTAGGAGGTAAGTAATCATTCCTATCTGCTGTCCGTGTCCCGGGTCAACGCTTGTCATCCCAAACCA
AAGAGAGAACCCTTCACAAGTATTTGTGTGTTAAATGCTTTAAAGATATTTCTGGAATCTTAGTTTCAATTGCTTTGACA
AATGCTATATTCAGTTTGTAAAAATCATTTATGGAAGTAAAACTGCATTTTTTTGGTCAAAATAGGTGGCAAAAGAGGTA
CTTTGACAATTTAAAGAACATCAGAAAATGTAGAACTATTTTTTAAAATCGGCAAATAAGTGGTTTTTAGGATTTATGTT
TCTTTTGCTTATTTGCCATGAGAAACTGTTAGCTAAACTTAAAGAAGATTTTATTGTGCACCTACTGTGGAAGGGTGTGG
ACCGTGTCTTGAGAAGTGTGATGCCTCTGAACAGAGCATTTTCATTCTGGGCTTTGCTGCTCCTCTGGTGCTCAGGGCAG
ATGGGGATACTTCTCTGGCTTCGTGCTGCCTACTCTCTACATGTACTGTAACTGACACCACACACTCTGTGGAGCTATCC
TTTCCACCTTTGCCCTGATGCAGGTAACAATGACAAAGAATAAAAAGGCTTGAGGCAGGGCATGGTGGCTCATACCTGTA
ATCCCAGCACTTTGGGAGGCCGCAGCGGGTGGATCACAGGTCAGGAGATCAACACCATCCCTGCTAACACAGTGAAACTC
CGTCTCTACTAAAAATACAAAAAGAAAAAAAAATTAGCCGGGTGTGGTGGCAGGCACCTGTAGTCCCAGCTACTGGGAG
GCTGAGGCAGGAGAATGGCATGGAACCTGGAGGCAGAGCTTGCAGTGAGTCGAGATGGCACCACTGCACTCCAGCCTGGG
TGACAGAGTGAGACTCCATCTCAAAAAAAAAAAAAAAAAAAAAAAGACTTGAGTGGAATCAAGAATAAAGAAAACATGGAC
AGGATACATCCTCCACATGTGTTCTTTAATACTTAAGTGGACAGATTTCACCCTCTGAGGATTCCCCATGCATCCCTCTT
TTCTGCTTCTCACTTCCTTCCAGGGGTTAGGGTTTACACATGGTCTCTGGGTACAGTTGGAAACTCTTTCCACAGTAGTG
ATGGGAATTCTTTACCAAACTCTTCAGTACCTTTTTGGTTGGTGTTTGTTTTAAGCATTCATGGAAATTCCAGAAGCTGG
GCTGGGCCCCAGGGCATCTGCTTTTTCACATGAAGACAGGCATGTATCTGGATTCGTGAACCTAAGCCCACAACCAGAATC
AAACATGGGTTCCCCAGATGCCAACTCTCTCTTCCAGGGTTCTCCAGACTCAGGTTCATGTTCACTTCTCTGGACACCAC
```

```
TTTAGCAAATACGCAAATCAACAAATAATAACAAACAAAACCCAGAACTACTGGGTGGAATCCTGTCTTAGAATTGATCT
GTCTTTCAGAAATAACCAGGTCACCGAGTGCGTAGCATCTGTGTGGATGCATTGAGCAGTGGCATGTGCTGTGGCTCCTG
GCATTTCTGACTCTCCTTTGCCTCCTTCCCTGCCCAGCTGCTTCATTCTGCTCTGCAGCTTCCATTCTGCATCCCTGTGA
GACTATCTCCATACTCCCTTGACATTACTCTTTTCATATTTAACAGAATTGTCCCAGAAGACCTCATTTTTCTCAATCTT
GTATATTAAACAATCACGACCTAAAGTATCCATGTGACCACTTAAATATCGAGAAAATGTAAAGCACAGAAGTCATGAAG
ACCTTAGACAATTTTTTTTAAATTTAACTCTTTATTGAGGGCTTGTTGAGTCATGGACCTCATGCATTTGTTGTGGTCTT
TCCAAATCTATCTGTAGGATTCTCTGCTTTTATTTAATAACTTGCTAGCAAGTCAACTTTTAGTCTGCTTTAGAAGATTG
AAAAAAGCCCTGTTAGCAGCCTTGACTGATGGTTGTTGGCAGATACTTTATGCTTATCCTTATTTAAAATCCTTTGCTCA
GTTGAGTAATAGCAGAACAATTATTTTAATGTATCCCAGTACCCTACAGCTATTGCATTTTTATTTAAAATCAGAGAGCA
CAAAGCAGTGTGAAGAATAGAAATGTAGGTTGGATGTGTTTTTACATGCCTTACTATCCTGCTTTTTCTTCCAAGATGAG
GTTCAGGAAATGGGCAAAGTAGAGACACATAGATTGTGATCAAAGCACTTCTAACCAGCAGTCTTGGTTTCATTTGTATG
TATCCTATGAATCAAGTCATTGTCATACAAGCTTTGAAGTCGTTTCTGATTCCAGTGATTTTTATCTAACATGTTCACAT
ACAAAATTATTACCACCTTTTTGTAGAAATAGCTTAGATATTTTGGCATCTTCTTTAGCTCTCTTTGACATGGACTCCAT
GGTGCTTGCCAGTATTAACAGGTGTTTGACACAAGACGGCTGTGATTTGGGGGCAGTTTGTTGAGTTGATGGAGAATTG
ATACCATGCCAGAAAAAAAATCAGATAACTCTATTGGTGAATGAGCTGCCAAGAGTTAGTTGGAAATTGTTGTGTATGTT
TTAATGGATATGAAGAGTAAACACTTTTAAAATGTTATTCACTATCATTCTGAAGTTTCTAAAATGGAAACTATTAGTGT
ACTGTCCATAATATTAAAAGAGATCTGGCTTCATAGTGATTACATAGCTTGGGAATGGTTCCTGCTCTTTAGCATTAAAC
CAAAATTTAATATCCAGGAAGTTGCAGGAATAACACATGAAGAAAGTCTTACCTTTTGTCTCCCTCTTCTCTTGTCTTGC
TGTTTACATAACCCATGCAGTCTGCCTTTCTCTCAAATTCACAAATTTACACTCAGTTTATCCTCCCACTCAGACCAAAG
TTTTGGAAATAACAATTCTCTTTTGCCACCTTCCCTCCTCTCGTAACCTGGAGTGAACACCAATGACTAGAAAATTGG
GAAGTGAGTAGCCAGACAGTAGTTGTGGGCAGGGGCTGGAAGAGATCCCAAACAAAACGGCTGTGTTAAAGAAGGCTGAG
AGCCGCAGGTGCCGTCTTGGAAGCATCCCTATATAATCAGCCCAACCATGTCCTGAAGGCCCAGCAGCCCACGAAGAAGG
TATGATTCTGCTGGGGCAGTTGCCCCATCCTTCACTCAAGAGTTCTCTGTAACTTTCAGAACACGGTGTTAAAACCATCG
TGTAGATTGCAGACCCTGTTTCAGCTAGCAAAGAGTGGGACTCCCTTAGTTTCTAGGATCGTTGCTGTTTTAGCCACTTG
CGAAGTCCCCCGGAATAGAATTTAACCAGGACTCTAATAGGACTGGACACAACTCAGGGGTGTTTGCTTTTGTAGCATTT
CTTTTGGGGAATGGGGGTGGTTTAGTTTGATTCAACAAAAACTCATTGAGTACCAGTTAGCTATGAGGCACTAAGCTTAT
GTGAACATGGGGCTATATAGAGATTTGAGTAAATCACATTCCCTTTGTAAATGCTCTATGTTCCCATGAAACACAGCCAC
GATATTATCTCCCTTCTTGGCCCCCTCCATCTCCCTTCCCCTTGAGACATTCTTCTACTTTCTATTTGTTGGCACCTGGC
CAAACCCTAGCCAAAAGATTCAATTTCTGTGTAGGAAACAGAAGCTATTGGTACCCAGGTTCTGTTTCACCAGAAAGGCT
CAGTCTCCACACCCGCTGATTTCATGCCCACGTTGATCAGTTTTCATCTCGTTATGCCAGGCATGTCCAAACCTAACAGA
GGCTTCAGTTAGCTGGGATACCCCTGAGATTGCTATGCAAAGATTGTTCTCCTGAGAGCAAATGCCAGATCCTGCCAGAG
TCTTACTAGCTAGGTTAACACAAACTTTTTATGAAATAATAGTCACCCTGAACAGCTACTTTCCCCTAAATTAAAAAGGC
TACAAAATTTTTGTCTTACCCCAAATTTAATGTATAAAAATTACCTGAACTCTAGAGATGAAAAATGTAAACATAGCACC
CTCTATACTAGGAATCTTAACCATTTATTGATGTCACACACATATTCTCTCCCATATTTGTAGATTTGTTAAAGAGAATT
CACAGGCCTCCTTATATTTGAATACAACTGTCTCAAGAACTCTTAAAAATGTACTTCTTGAAGTATTCTTGGACCTGGCA
TGAAACAATAAAGCTGTGAATGAACACATAATTCATTTACCTACAGCTACTTCTTAGTCTATTTTGGGAAGTCCTACTCT
GCCACTGAAGTGGTGCTTTCTCCCACTCAGGAAGAGGAACTGTCAGCACTCTCATTCAGAACTTGCCTTCTACTTAGCAC
GGATGGCGGAAAGTACAAAAACGCTGGGCATGAGATGCTGTTAAAAATCCTTCTGTGCTCACAGGGGAGGCATTCCAAAG
CCAGCCCTCATGGATAGAAGTAAAACTCCATCCTCAAGTCAAGCTGGACTTCTGAATGTCGAGTTAAAATGAAATTATCA
TGGTAACACATTACCTGGGCTTCCAATCAGGGAGCGATTGAAGGCTCCTTGGTGGCTAAGGTATGGCTCAGAGCCAAGTG
TGGTGTTCCAGGGTACCAAGTCCTCTGCTCAATAGCTTTCATTCTGAGTGGCACCAACACATTTATAATGGAGAGTCTCT
CCTAACTTTTATCATCACCAGGAAGTGTGTTCATCAAGGTGTTATCTATTCTACTAGCCTTGATGCTCACTGTTCTAGTT
AAAAGGCTATCCAAAAATACTATGTCTTGGGGTACTACTGAGATTTGTTGAAACATTGGATAATTGTTCCCCTTCCCCCT
CACCCAACCCATCATGTTGACAGTAAAGTCCCTTATCCTTGAGGCAGCTGACATTTCAGAGGGGCTCAAGGGTGCTTAGC
TTGAAAACAAGAACAAATATGTCTGATCACGCCCGTGTCAACTGTGGTTAAGTGTGGGAACCAGGATAAGGACAACTGA
AGGCATGGATTGAGTTGTAAGGGGCCGCCTCCTTTTCCCTAGGCCCAGATATTGTCAACATGAGGGTTTGTTCTAGAGCT
ACTGTCTTATCTGCCACATTCCTTTTTTCATAAGAAGGCAATTTGGTAGCATCAGAATTTATCATTGTTTTTGTCCAAAAA
TGTGTGTCCCAGGCCAGGCGCTGTGGCTCATGCCTGTAATCCTAGCACTTTGGGAGGCCGAGGTGGGTGGATCACCTGAG
GTCAGGAGTTTGAGACCAGCCTCACCAATATGATGAAACCCGTCTCTACTAAAAATACAAAAAATTAGCCAGGTGTGGT
GGCGTGCACCTGTAGTCCCAGCTACTTGGGAGGCTGAGGCGGGAGAATTGCTTGAACCTGGGATGCAGAGGTTGCAGTGA
GCTGAGATAGCGCCACTGCACTCCAGCCTAGGTGACAGAGTGAGACTCTGTCTCAAAAAATAAAAAATAAAAAATAAATG
TGTGTCCCTCCAGTGCCGCTTGCCCCTTGTTCTCCCAAGCACCAGGGGGACCCCTGGTCTTAGATTCAAGTTGCATCACT
AGACTAACCAGCTCTGGGAACCAGAGGGACACTGGAGGAACTAGTTTGGGAAATGAAAAACATCTGCAATGAGAGGTAAT
TTTATTCTCCTCTTCCTTTTACCCTAGTCTCACTCTCCATAGTAGGACCTGGATCTTTGCATCAGAGAAAACATAATGAA
CAATAACTGGAATGTGTGTTTCTTTCTTTTCTGCCCTAGTATTACTAGAACATTTGCATCGGGAAAAACAGAAAAGGTGA
TCTTTCAAGCACTCAAGGAGTTAGGTCTTCCCAGTGGAAAGGTATGCATTAACTTAATAATGTATTTCTAAAACCATTTT
GTTTATTTATCTTCTCATATGTAATGAGCTAGATTATTTATATTAAACTGATCTAAATTAGATTTGGAATTATATGTCAG
TGACATATAGTACAGTTAATTTTCTTTTCTTTCTTCTTCCTCCCTCTCCCTTCCTCTCTCCCTCCCTCCTTTCCTTCCTC
CCTTCCTTCCTTTTGTTGCCTGGGCATTTAACCTAAATCTCTAAAAATGTAAATAGTTTGCTTCAAATGATTTTTGTCA
CCTTTCTTAAGAAAAGGAAATGAAGGCCAGCTTGCTGTGTTACACAGTTTTTATTTTAGAGAAGCTCACCAACACTGCCT
CAACCAGGCACAACATTAGCATGAATAATTAACTAATAAATTCAATTCTACAAGATGGCTATGCAATATTTTGTGAAGCA
```

```
TCTCTCCCCGCACCATTTGTATTCATGGCCCTTCAAATTTAAAATAATTGAAATTAATTTATTGACTTGAAGAAAGATTG
AAGCCTGGTACTATTTTAATAAGTCCTATTTGAAATATAAATTTCAGTTTGAAAAGTAAAATCTTACATGGTTACCCTAA
GCCCGTTCACATGGTCTTGCAAAAATTCACAAAATAAGCATAATTAAACCTTGGGCTTGATTTATTCAGCATCCCTCAGG
CAGGCACTTTTCTCCCTCAAGGTGAGTTGGAGGAAATATTTATATTCGTTCTTTGGGGTATTTCCCAGAGCAAGGCCAGC
TGTAGCCAGGTGTTCGCACACACAGAATCTGGCAGCATGATAACTAGAAACACTGATGTACACGCACTGTTTTTTAGTGA
GATGGCCTGGCAGTGCCTGGAAGTTGGCCTCCTGGCTTCTGTCAGCAGGAATCAGAGATGAAGACCCTGAGTCCAAGTGG
TCTTCACTGTGCTTCATGACCCTGTAGCTTCTCACCCTCCAAGGGAAAAACGGTTCTGTGATTCATAACATGATTTTATT
CCTTACAGAATGATGAAATTGAGCCCACAGCATTTTCTTATGAAAGTTCTATGAACTGACACAAAAGATTTGTCCTCGG
ACAGATATAGAAGATCTTTTCAAAAAAATGTAAGTTCCACTTATGAGGAAGTGCCATATAAATATTATCACTGTCCTTTT
CGATTTTTTAAAACGTTTTTGTCCTATTTAATAGAGTATGTTGGTAGCATCTAGGAATATCTAGTCAGGGTTACATTTT
TAACCCCATACTTTATCAGTGTAAAAGTTCACTCTGAGTTTTTGAGAAACTTTTACGTATATTAATTGCAGCACCTGGAA
CTTTTTTTTGCATTGGCTTTACTAACTACCTACAATTTTAACCTGCTCCTTTAGGTATTAACCCATTATGTGAACTAACT
AGATTCAATTTGGAATGTTAATATGTTTTTGAAAAGTTGATTCATTCCAATTATTTCAAATGGATGACTTACATGTTGTT
TTTGAAATAATATCTTTTGAAAACGGTAAAAAAAAAAAAAAATTGCAGACTTTTTCTGTGACCTAGTGGGAGTGTTTTCC
TTCTGTTGTTGTTGTTGTTGTTTTATTTTTCTCCCTTTATGAACAGTCTCTGGCCATTTTAAGAATTCACCACTACT
TCAGGGTTAGAAAATAAGTTTAGAATTATCATCAACAAATGTCAACATGTAAATCGTACATACTGTAGCATCATATACAA
AAACTTACTTTTTCTAATAAATTTCTTTTCAGCAATGGAGACAAAACTGATTATTTAACGGTAGACCAATTAGTGAGCTT
TCTAAATGAAGTAAGCTTTTTCATACATTAACTCCATAAAGTCTGTGTGCAGTGGCTTGCCTTCTCTGGTGTCCTCATTG
CATGCCTGCATCATATGCCCTTATTCGCTTTGCTTCTGACCTGCTGATCTTTCCATTATGGCTTTACCAGGTGCAAAAAT
CAGCTGCCGCCCTGTGTGTTCATGTGCTTTCTTCACATTGGCTTTGCACACCACTTTTTGTTGGTTTCTGGCTGTTACAT
GTAGCTCTGGTTCTGTGTGTATGTGTGAATTGTATAATATATTCATTTAAGTAAATGTGTCTGTGTTTTTGCAAGATGAT
ATTTAAGGTACTTTTTTTCTATTGATCTCATTAAATAGAAAGAGAATGGATTGAGTTTACTACACTGTAGTTGGATTCTC
CATTGCTAACTTTATCTCATACCTTTGCATGGACTGGAAAGCTTTATTTTTGTCTTTTAATGGATTGCATGTAAGAAAAA
TATGTTGGCTCAAATAGAATATTTTGACAACAAGCAAAACACTTGAGTTCCTGGGTGAAAATACATTGAGTTTTAGGCTA
ATAGGATTGCAGGTTAAATTTGTACTTTTTCTTTTTATAACAAGCTCAGAGCTAATTTTAGAATGTGATTAACTTAACAA
CCAAAACTGATAGATAGAATTGCTTAAGCACAAGAGAAAATATTTTCGAACAAAAAAAGTTTGCAACTTTTATTACAAC
TAAAATGAACTACTTTTTTCTTCACACATACAAAAAAGTTTTCCCTTCAGTGAAACCCATCAAGACTCTTGTTAATCCA
AAATGGAAAATTCACTTTTTTTTCTAACACATTAATAAGAGTTTGAGTGAAGGATGGACGTATGCTTTTGTTTCAAAAT
AATTTAAGTTAATCATTATACCCATATAAAGAGAAAATGCAAGAAAGATGGTATCATGCCAGATAAAACGTATTATCTTA
AGAAAAGATAAAAAGGCAGACCTTAATTTGCCACTTCATAGAGACAAATTACATCTTCTTTTCTTCCGTTGTGAAGGTTC
AGTGGCTTCAAATTTATAAAGGTCGCACTTTAGAAACTATGTTAACTAAAGTGTAAATGCACTGATGCAAGAAAGAGAAA
ATCCTACCCCACATATCAGACAGATGCCTCTAAAATAATCCTTCGTGTACAATACAATGTTTATTTTACTCGAAAACATT
TCAAATTTTTCCACTCCACTGGTTTTTAGTCATATAAATACAATGTTCATAATCTTTACCCAGATACTATCCACGTTCTT
ATGCCACAGCTGCTCAGAAGTTAAATTGCTTTGTTTTGTTTCTTTAACCCCTTCCCCTTCTTCTGCCTTTTGTTTTTTGG
TTTCTTGTTTTGTTTTGTTTTATATCCCTTGGCTTCTTCATGCACTTTGGTTCAGTGATCATCTTAGGTTTGGTGAGGTG
AAGAGGGAAGACGGTTTAGTGTAAAGGAGAAAATCTACCACATCTTCAGTTTGCCACTTCCAAGACAAGGCCTCTTGTGT
TCTTTGGGATGAAGCCCTCTCTTAAACATTACAGAGACCTTAATACTGCATTTCACTGTTAAAGAATGAAGCAACTCTTA
GGTCTTGAGGATAAGTGCAGTATTTATAGGCTCTTATCTAATGCATTTCCTTTTATGGGTTGTTTTTGTTGGTGCTCCCA
ACAGTCACTCCTGTAATTCTTAGACATAACATTTAATTTTTTAAAAATATCATCATCCATACTTTCTTGTTCTGCCATTC
AGATTCTTTCTTTAAGTATTTATTATTTGTCAACCTTTTATCCCTCACAATTACAGTAGACAATAGTATTCTTTTTTTTA
ATAGTGTTTCCTTCTATTTGATGTCTTCTTAACATTAAGGAAGTACCTTAAATTTTGGGGTGTGACTGATGGAATCGGTG
TGATTCTAGATTTCTTTAGGTCATAGGAACTGTAAGACTACTCAGGAAAGTCTATAGCCTTTATTTTTAATCCAAGTGTC
TGCCCTGAATTTATTCCTTTTCTATCCTAGTGAACTCAACTGGAAGAACTAGACAAAACATGAATGTCGGCTTGAAGAA
GCCTTGTCAAACATAGAGAACATTCTGCCTGTTCCTTGCACTCCCTCTTTTGAGTCCTACCCTGGCCCCTCTTATCTCTC
CAAAATCAAAAACACCCCTTGGGCAGAACAACTTCAATACTCATAACAAAAGGAGACAGACAGAGCTCACTCAAGAAAGA
ATGGGCATTGGAATGTAAGGGTCAATTACTCCAAGAGTTTAAACCTAAACATTTTCAAAATGCCTTAGCCACATAGTCTC
AACAAGCCAGTATTCAACACTGCATTGGAAGCCCCTGTGAAGGTTCCTTGTGAAGGTTCAATGGCTTCAAATTTATAAAG
CTTGCACTTTAGAAACTCTGTTAACTGAAGTGTAAATGCACTGATGCAAGAAAGAGCAAGTACTACCCCACATATCAGAC
AGATGCCTCTACTTTTAAATAATCCTTTGAGTATGATACGATGTTTATTTTACTTGAAAATATTTCAAGTTTTTCCACTC
CACTGGTTCATATAATGAGACAAAAAAATAAACAAGTCCCATGAACACTGGAAAGGACAAGGTCCTAGTCTCATTTGCAG
ATGTTGATTGTCTACTCTGATATAAACAATTCCACGGAAAAACTGTTTGAAGTAATTAGAGAGTTCAGCAAGGTAACCAG
AGAAACAATGAGCAAAGCAAAAACCTGGAGACACACATACACTGCCATTAACCAGGTAGAATAGATAGTAGGAAAACATA
AATCCCATTTATATTAGCAAAAGACATGCTGGTTCCTACCACCTTCCAGCTGGTTAATCCTTTCACTCTTTCTATGAAAG
TTTTTATTCATATACTAATGTGTATCTTCAGGGGGCTGCAAGGAGAGGAGAGACTATATATATAGTCCATTTACTGCTTT
GGTCACCCTGTAGGGTGACCTCCTCCCTATCCATGCAATCAACACTGACTAGATTTTCAGTAGTAGCTTTAGACCTGACT
CCTCATTGGCTTCTCAGTATTTCCTTGTTCGTTGCTCCCCAAAGTGTGATCCATGGACCAGCAGCATCGGCACCCCTTGG
GAGCTTATTAGAAATGCTCAGTCTTGGGTCCCACCCAGACCTGCTGAGTCAGAGTCTGAATTTAACAAGATCTCCAGGTG
ATTTATCTGCACATTCAAGTTTGTAAAGCACCATCCTAGTGGATTACTGGATTCCTCCTTGGAATGTTTAGTGTGAGTGG
GAAGAATATGAGAGCGACATATACCTTCTGTTATTCTCCATTAAGAATTTAGCCATAGGAATCCACCACTCAGAGCTAAA
ATTTTGCCACATCTATGTCTCTTAGTCTCATCCAATGGACTGAGGGAAATCTTCTTTGCCTATAATATGAAAATAGCAAA
CTAGAGTCCAGGAGGGATAGCAGAGGCACTTAATGTGAGCTGCCACGTGGTGAGACACTACTACTGGCTCAATTGCTTGT
```

```
ATGTGGCAAGACTAGATAAAACCTCTTGGCTTTGGTTAGTTTTACCTCCCTTTCCCTATTCCTAATGAGAAGCAAAATTG
ATTTTTAGTTTTACCTTCATTCTTTGTTTGACTTCTTACAAAGATGATAACTAGGGTCCTGCAGAATTGGGAGATGGGTG
GAGGGCAGGAGAGAGGGATAAAAACAAGGAAGAGAGGGAAAAGGCCTTAGGTGGCCACAGGAATCAAGGAGTAGCTTTAC
TTTCTTGTTGCTGGTAATCTTCCCCCTTCCTCACACATATCATAGGATCACATCAGGGCTCAACCTGACTTTTTTCTTTT
TGCAGATGAAAGAATATTTTAAAAGGACCCATGATCTCTTTCCTTTTACAAGATCTTTATGCTTTAAATGCACAGGCAAA
GAGAGAGAAGGAGGGTATTTAGTCTTATGAGTAAGCTTATCCTTGCCAATTATTCCTTCTATATTTTTAAAATATTATTT
ATGTCAACATTCATCTCTCCCAGTGCTACTTGGATTGAAGCCAGACGATGCCTCTTCTCAGCACCAGTAAGGACGTAATA
CTGGAGTTCTCTTCATCTCCTCCTTACTTACTTGGACACAGTTCTGTGACCTATTTTTGGAAGTCATTTAGAATCTCATT
TTACAAAGCACTGGATACCATGGGGTGAATAACCTGAGTAACTTGTTTCCCCAAATCCTGGCTGAAAACTGATGGTTCCT
CAGAGATGTGAAACATTCACCTCACTGTGCTTGTGTCTCTGTGGAACTCTACTGCAGCCTTATTACTGTGAGATCAGCTG
GAAGTGATCCTTCTCCTCTTGTTTTTCCATATGAAATGATAGTTTTCTGACTCTAATGCAAATCTTATATTTGTTTCCT
TGCCTTTGGATTACCCAGATTGATTTGTGTTACCTTTTTGAAGCATGAAGATTTTAGGGAGAGCTGAAGATTGTTATCTA
GTGGGAGCAGAAGCAAAAACAAATGCAATTTCGCATTGTCTGCACTTCATGCTTATTTGCTGCGAATGTAATTTTCAGAA
TAGGGGATTATGATACATCCATTTTAGGGCTTGGTTTTCCTGGCTTTGTTTTGCCATAGGCAGGATGGTCTAATGAATGT
CATACCCTCTGACTAAATTTTAATAGTGTTTAATTGGGAGGACATCTGAAACACTGAACATGCTTCTATCCTTGTGAGAC
TTAGGTACTTTTCTTTTTACTGATGTGCAAATATGTTAAAATGCATACCTTTATATGTTAGGTAAAGTGATTCTTATACT
TGAGCACATCAGAATTACTCAGAAGTCTTATGAAAAACACAGATTAATAGGTTCCACCCCAGAGATCAGCAGATTCAGTA
GATCTAGGGAGGGACCTGAGAAGCTGCATTTCTAGCAAGTTGACAGGTGTTGGTGAGGCTGATGCTGCCATCCACAGATC
ACACTTCAGGTAGTACCTTGTCAGAATAAACACATGTATGTATATTTTAAAATGCACAAAAATGTCTGAGTTCTGTCTCC
TTTCCTAATTTTATCTCTTCTCTCTCCTGTAGCTGAATCCCTTTTTTCTCTCATTTTTTAATTCATATGGATTTGCCCCA
TCATCTCCATGAGTTCAAAATATTTACCAACAAACATATTTTTAAATTTAAAAAACTTAATTTTAATAAGGAAGATAAAT
AAAGTGGCCACCTATGAACATAACAGTGCTGGGGTCTAAAAAGGACCTTTTTCATCCTTTATCCATCTTCTACCATGATA
TTATTTATGGAGATGTGAGAAGGGATGTGCCTAGAAAGATGGGTTGTTGTTGTTGTCAGTTTGTTTGTTTTTAAAACAA
TGCATTCTCACCAGAAAGACGGAGCTCCTTGTTTCCTTGAGTAGTGGGCTCCATTGCACGATGGCATCAATTTCCTAAAC
AAGGTGTAGCATGCCTTCACTTGCCTGGCATTCCATGGTCCACCTCACAATCCTCTTCCCTGCATATCCCATAACTTTTG
TTCTTGGTGGCCTCGAGGCCTGCTGTTAACATAACAGTTTCTGTTGTAGCATATGCATGAATGTTAGCTGTTGTGAGCCA
TAATTTCTTAGATTACAACAGAAGCATGCTTCATTGGGATAAATTGATGAAATTTGATATTGATTCCTTATGAGTTAATG
CTCTTAACTCGGTGGGGGAGGAGGGGACCCAGAAAAGGCTGGGCCCCATATGCATGAGGAAATGACATCCAAGTGCTCTG
TTGGCCAAAAGCATTCATTCTGTGTATAGCTCACTTTTCCAATGCCCATTCCAAAGCAAATAGGGAAGGGAAATCCATAT
GAACTTTTAACAGTTGCTTTTTAAATACCTTTCCAACAATGTTTTTCAGTCCGTGCATATATTTGGGTAAAGAGTTGTCA
CCAAGGTCCTATTTATTGACCAGGTTTTGCTCACACCTGCTTATCTGTCATTATTCCAAACTTCCATTGAGGTTTGCTTT
ATTTATTCACTTATTCATTATTTTTGTAAGGGCTGACCAGGGACACAGTGCTGTCCTCTAATCCTCAGTGAACAGAAAGG
AAGTTTAGTGGACCCACTGCCAACACACATACACACACAAACACACACTATTGGAGCTGAGTAAGTTAGTAAACTGGGTT
TTTCATCATTGCCATGAAGTTAGGATGATACCATTGCAGGGACAGCATTGCTCCTGGTGCCTCATGTCATAGCAGGAGCT
CCTTAGGTGTCAATTTGAAAACAATAATGTCACCAACCTCTTAAGTCCCGTAATCATTCATGGACTCTCTGCCCAGAGAA
TTCTATATTGTTTATAGCTTGTAAATATGCTTGTACTGATTAATTTTCATCATTACAATTGGTTCGTTTTGAAAAAAATA
GTTACTCAAGAAAATGAAATTTGTTGAGTGCCCAAGAAATCATGGATTGATGTAGAAAGGTGTTATGAAAAAGAGATGAT
GTTTGCCTGTGTAACATTAGGCTTTTTGTTGTTATTGCTTTTGGGGTTTTTGAGGGGTGCACTTAAATTAGTATATTACC
TATTTGAACTATTTTGGGGGGGTTGGTGTTTGCTTTTTAAAAATATTTTGACCTCACGTTTTATAGATATTCATGCCAAAT
GTCTTCCCTGTTGTGTGGGAACTGGAAGACAGTACAATTGACATGCACACTTTATTATGTATCCTTCAACTTTTGAAGGG
GAAAAATGTATTCTCTCTCTCTCTAGTTCAGGGAGAGTAACCCAAAAGTATTCCCAAATGTGGACTTTTTGAAGTAT
ACTTTAAGGAGAAAGGCAGGAAGGCAGTGAGGTCCTATCTTTGTAATTATAAACATGACAGTCTAGATATATTTTGTTTT
ATAAATTATAAACTTAGTAATGTCTAATGCCTCAAGGCCAAGAAATATCAACCTAAATGGAGTTATTTTCTTATCATAGC
ATCAACGAGATCCTCGATTGAATGAAATTTTATTTCCATTTTATGATGCCAAAAGGGCAATGCAGATCATTGAGATGTAT
GAACCTGATGAAGATTTGAAGAAAAAAGGTAATAAACATGAAAAAAGGACTTAAAAAAAAAAACAAGAAAAGATGCAACT
TTTAAAGCCTTGGTTTATTTAAAGGTATCTGTAAATGCTTTGCAACTCCCTGAGCCATGGATTGATGACTATTTTTTTTT
CCTGCTCTTAAGTTCGTATAAATTTTACCTTTCATCAGAATTAGTCAATCCTGACTGAAGTATACTGTTACATTTATTGT
ATTATTAAATACATTATAGTGATGTTTCCCCATGTACTTTCTTGTATTAGATTTTAGTATTGCATATTGCTTGAAATATT
AAAACAACCAATACAGTCTCTACTTGCAATCCTTGAAAATAATTTTTGTCATATAAAAACTTATACCTTTTCTTTTTCTC
CCTTTTGTTTTGCCAAGCCAAATAAATTTAATACGTGATTTGATGTGCAGTAACCCTCATTTGACCTCTGGTATGAAAGA
TAAATACAATTGATATGAAATTAAATAGTCAGGAATCGCCTTAGCTAATTGCTGGACCTTTGTCCCTTTCAAAGAAGCTT
TGGAAACAAAGGGTTGTTCGCACAAATATTTGTCAAAGGAAAGGCATGGGGAGAAATTTTAAAATCAGTCCCACAGCAGT
GAGAAATGCCTGCGGAACATGGGAGGGAGAGCTGGGGGTACCTGAGCTAGAGCCTTTGGCGTTATGTCTCAGTGGAGAAT
GTAAAGAGATGAAAGTTCAAGTTCCATAGGTCCCGTATGTCCGTGAATCTCAAGGTCTCAGTTTCCTCCTTTTTAAAATC
TATTTTTTTCTTTGTATCTAATGCCAGTGAGTGTGGTTGAATGGTCTGTATGTTATCTGGACCCTCTTTTTAGGTATACC
TGGCCCTCTACACTCCATGATTTCACCCCTGATATTTTTCCAAGCCCAATAGTTTTCCCTTTAGTTGTAGTTGTACAGTT
TACACTTTCTGCTATGGCTGAAGATACCTATTTTAAGCAAGAATGCTTCATTCCTTCCCTCTCTGTCAATATTCTGAATA
TCATAATTTATTTCACATTGCTTATGATTACAGCAAAGGGAGGTACAGCTTGGTAGAAAATCTAACACTTCGCATTGGCA
GGAGGTGATATGATTCTTGTCACTTCCCTGTGACCTTGAGCTAGGAACTGGCCTCCTGGGCCTCAGTTTCCCTGCTTGTA
AGGTGGTGGGGCTCATGCAGTAATTTGGCTAGTATTTCCCTTCCCTACCTTCATTCTTTGAAGTTTGTAGAGGGCTGACC
GGGTGCCAAGCACTGTGCCAGATGCTAGGAATTCAAAACAATAAGAAATGTCCCCAGCCTTGAAGGCAGTTGCGATCTGT
```

```
TCAAGGAGAAAGACAAGTGATTCAACAATTCCTTGGCAGTGTGATAAATGTCATGATTGAGGTGTGTCCTTGTTTATTTC
CAAATTCCTTCTAAGTTTTAAAGGTCTATGAAGTGACCATTGACACATCTAAAAAGGGTAGCTTGACCATTTCACAAGTG
ATGGAAATTTTAACGTCTGTCATTTTCAAGCGTGGGTGAGAATGGAGTACAAAGCCATTCTTATAAACTTCTGGTAAGAC
CCTACATTGGTACAACCATCTTGGGGAATATTTTGGCAATAGTTTATAAAGTGAAAAATATGCCTCTTATACAGCCCAGC
ATTTTCACTTCTAGGTATGTACCCTAGAGAATCTCTCATCTATTTCCCAAACTCTGGATACCAGAGCGGCACAGTTCTCA
GATCTCTGCTCCACCTATATTCACTCTGTACCTGAGCTCATCCAAGCCAATGGCTTTACATTTCTTCTATTAAACTCTCC
AGTCCTAACCTCCAGCTTACACTACAGACTTGTAGTAGAACTCTCCATCTGGATGTTGAATGGGCATTTCCAATGTATAT
ATACAACATATTTCGATCCTTCCTACCCCTAGATATGTTCCTTTCAAAGCCTATAAAATAGTACCGTTATTTACCCAGTC
AAAACCACTGGGCTCATGTTGGACTTGTTTCATTCTTTCACATCATGTCTGGTTCATTTGCAAAACCTGTAAGATCCAGC
CTCAAAAGAAATCCTAAATAAATCACTTCTCACCATCTCCAACATTACTGACTATCCAAACCTTCATCATCCCTCTCCAG
GCAATGCCTTCTCCTTCCTACTCAGTGCCCTACAATTTATGGTCCACACAGCAGCCAGAGTAAACCTTTTAAAACCAAAT
CTAATCATGTTATTCCACCCTTTGAAACCCTCCAAAGGCTTCCCAACACATTTAGAATAAAATCTAAATACTTAGTGAG
GACTAAAGCCCAAGTGATGTAGCCTTTGGCTCCATCTCCAATCTCACTTCAAGCTACCTTTTTCCTTGGTCACTTGCTCT
GTCTTGCTGTCATTGAATGATGTTGGCACGCATACATCTGCATCAAGGTCTTTGCACTTGATGATCCTTCTGCCTGGACT
GGTGTTTCCTGGGTGTGCACGTGGCTTGCTCCTCACACATGTACGTGACCTCAGAAGGGCTTTCACTGGCCCATATTTAA
CACAGCATCCTGCCATGCTTCATCACCTTCCTCCCTGCTTTCTCTGTCCTTCTAGTACCTCTTTGACATTATATTTATTT
GTTTACTTATTTGTTTCTGTCACTCATAATATGAGAGTTTCATGAAGGCAGGGCTTACCTGTTTCTTCTATCACTGCATC
TATCCTGGTGCCTCTAAAAGTACTTGCTCTTTAGAATGAATGAAGGTGCCCAAGGAAAGAGGTATACAAATTTCTACTTT
CATTTATAATTAGAAATGACCTAACTATCCATCAACCAAATAATGGATAATAAATTATGATATATTCATATATGGACTTC
TGTACCTTCAGTGAAAAATAAATGAACAAGATCTACCTACATAGCTGGTATATAAAGATAAAAATAGCAAGTTGCAAATG
CACACAGTGGGATAGTTTTTACATAAAATTAAAAACAGCCCAAACTACCCGTTGTTTGGATAAATGCATATGTAGTAAGG
TTGCAGAACCATTTTGGAGGTAAACACCAAAACTAGGATACTAGTTATCTATCTGGTATGGAATGGAATTGAGGTGGGGT
ATACAGGATGCTGCAGTGGCATCAGAAATTATGTATTCTTTTACATGTAAGTCAAATAATCAAAAATGTAAATCAAATAA
GGCAAAATGTTGAGATCTGACCACACTGGTGTAGGCCCGTGGCTATTCATTATGCTATCATCTGAACACTTCTGTATTAG
CTTGAAATCAGTTCATAACTTGCAAGTTCTTAAATAAAACAAAAACTTGAAGCTGTCGATGCATCAACCATTTATAAGTT
GCATAAATACTGATTGTTGCTCCTGTAGTTACCACGTGGGCTCTACCCCATGTCAGAAAGGTGTGATCGAAATGAAGGGT
GGCTTTTGGGGCCACGTTTCAATGACCACTGTATACTCTTCTCACCAGTCCCATAACCTGACTTTTAGATAAAATTGAAA
ACTGAATTGTTTAAACTCAGAAGCACCATCATCTCCAAGAAAAATCCCTTTTCCAATTCAGTGAGAACAATGTCTTATAA
TTAATACATTTTCCATTTTTAAAGAGCACCTCTCGTATCTATTCTCATTTCCCATTATGAAGATCTTGGAAGTCAGATAG
GTCCAGTCTTTTTCCCCAATTGACAGAAGAAGAAGCTGAGATCTAGGAGCCAAAGGAGTGAGTAGCAAGAGCAGAACTTG
ATACAGGCCCCCTGGACCCCTGGTCACCATAACTCTCAGTGCTGTGTTTCTGAACTTGGGGGACCATGTACATCTGCCAT
CATGGATGTAAAAGCAGAATACCACCTGTTTGTGGAATTCATTATGTTACTTATAATGTGCAACTCTCTTATTTCATTCA
AGTTCTGACAGATGAAAGTTCTTGGGAAAGAGACTGTGAAGACGTTTTTACTCTTATAATTTAAGCTGCCTCAGCTTCCA
TGAATAAAACATGAGATATGCATCTTCAGAGCTACAAGTGCTACTAAGATGTTCTTTTTCCCCTAGGCCTTATATCAAGT
GATGGGTTTTGCAGATATCTGATGTCAGATGAAAAACGCCCCAGTCTTCCTAGATCGTTTAGAACTTTACCAAGAAATGA
CCATCCTCTGGCTCACTACTTCATCAGTTCTTCCCATAACACTTATCTCACTGGCAGACAGTTCGGCGGGAAGTCTTCGG
TAGAAATGTACAGACAGGTTCTCCTGGCTGGTTGCAGGTGAGGAACTCAAGATGGCAATTTGTTTTTTGTGTGTGATGCC
CTTCAGTTTAATTTACTTTTCTCTGAAAAACAGGAAATAGTAATCAGGATGAAAAGGAAATTTGTTTATTCCCTTTAATG
GATTATCATGGTCATTTAACGTTGAGAAAAGAAGGACAGTATCTCTTTCGGAAAAATCTTCAGTCATCCAAAAGACTTTA
CTGTGAAAAGACTTTTCTTTACTGTAAATTTAAAAATATGCCTCCAAAACCACAAAAAAGAAAACATGCATATTGTAAGAA
GGCGTGTTGAGTTTTTTGTTGATTATAGGTACTTGTTACTAGCACCATGCTCTAACCAATTGAGCTAACTGGCCATTGAT
TATAGGCACTTGTAAATTAGCTTTTTTTTTTTTTTTTTAGTATTTATTGATCATTCTTGGGTGTTTCTCAGTTTCTCAGA
GAGGGGGATGTGGCAGGGTCATAGGATAATAGTGGAGAGAAGGTCAGCAGATAAACACGTGAACAAAGGTCTCTGGTTTT
CCTAGGCAGAGGTCCCTGCCGCCTTCGGCAGTGTTTGTGTCCCTGGGTACTTGAGATTAGGGAGTGGTGATGACTCTTAA
CTAGCATGCTGCCTTCTAGCATCTGTTTAACAAAGCACATCTTGTACCGCCCTTAATCCATTTAACCCTGAGTTGACACA
GCACATGTTTCAGAGAGCACGGGGTTGGGGGTAAGATTATAGATTAACAGCATCCCAAGGCAGAAGAATTTTTCTTAGTA
CAGAACAAAATGGTGTCTCCTATGTCAACTTCTTACTACACAGACACAGTAACAATCTGATCTCTCTTTCTTTTCCCCAC
ATTTCCCCCTTTTCTTTTCGACAAAACCGCCATCGTCATCATGGCCCGTTCTCGATGGTCACTGTCTCTTTGGAGCTGTT
GGGTACACTTCCCAGATGGGGCGGCCTGGCAGAGGTGCTCCTCACCTCCCAGATGATGGGCGGCCAGGCAGAGGTGCTCC
CCACCTCCCAGACGGGGCGGCCGGGCAGAGGCGCTCCTCACCTCCCAGATGGGGTGGCGGCTGGGCAGAGGCGCTCCTCA
CTTCCCAGTCGTGGCGGCTGGGCAAAGGCGCTTCTCACATCCCAGACGGGGTGGCGGCCGGGCAAAGGCTCTCCTCACTT
CTCAGACGGGGCGGCTGGGCAGAGGCGCTCCTCACATCCCAGATGGGGTGGCGGCCGGGCAGAGGCGCTCCTCACCTCCCA
GATGGGGTGGTGGCCGGGCAGAGGCGCTCCTCACCTCCCAGACGGGGCAGCCGGGCAGAGGGGCTCCCCACATCCCAGACGATGGG
CAGCCAGGCAGAGATGCTCCCCACTTCCTAGACGGGGTGGCGGCCGGGCAGAGGCTGTAATCTTAGCACTTTGGGAGGCC
AAGGCAGGCGGCTGGAAGGTGGAGGTTGTGGCGAGCCGAGATCACGCCACTACACTCCAGCCTGGGCAACACTGAGCATT
GAGTGAGCGAGACTCCGTCTGCAATCCCAGCACCCCGGGAGGCCGAGGCTGGCAGACCACTCGAGGTCAGGAGCCGGAGA
CCAGCCCCGTCAACAGGGCGATACCCCATCTCCTCCAAAAATAGAAAAACCAGTCAGGCGTGGTGGCGCACACCCGCAAT
CCCAGGCACTTGGCAGGCCGAGGCAGGAGAACCACGGAAGTCCGGGGCAGAGAGGCTGCAGCGAGCGAGACCACGGCAG
TATAGTCCAGCCTCGGCAACGGAGGGAGACCGAAGAAAGGAGGGAGGGGGAGGGGGAGAGGGAGAGGGAGAGGGTAAATT
AGCTTTATTTGATGTAAAAGTTCAAAAATTTAGTTCTCTGAGAGTAGAAATTATTAAAATCGTTTTTGTTTTATAGAAAC
```

```
TGGCAAATATTTGAGATTTAAAATATGGCAAAAATGATGAAAGTGAAAAATAAGAAATGTAGTTACATTATGTTTTTTTC
TTGGTTATAAATTAAACAATACTTTATTTTTTTAATTCATTTTTTTTTGGATATGGAAATATGTTAAAAGAAATTAACTG
TCTTACTACTTCCATTTGCAAACTTCCTCTGAATACCTGAGTATGAATCACTAAACATTTTCTGAAATGGCCAAAGTAAC
ATTATCCAGCCTATCAAAATTAAAAGTAATTTTTTTAATTTATTTTATTTTATTATTATTATACTTTAAGTTTTAGGGTA
CATATGCACAACGTGCAGGTTTGTTATATATGTATACATGCGCCATGTTGGTGTCCTGCACCCATTAACTCGTCATTTAG
CATTAGATATATCTCCTAATGCTATCCCTCCCCCCTCCCCCCACCCCACAACAGTCCCATGTCATCTAAAACCCAGCCCA
CATTCATATTTCCCCAGTTGTCCCCAACCAAATTGTCCTTTTTTTTTTTTTTAATTGGGACAGAGTCTTACTTTGTTGCC
CAGGCTGGAGTGCAGTGGCATGATGTCAGCTCACTGGAACCTCCCCTCCTGGGTTCAAGCAATTCTCATGCCTTAGCCTC
CCAGGTAGCTGGGATTACAGCCTCCCAAGTAGCCATCACACCTGGCTAATTTTTGTATTTTTAGTAGAGATGGGGTTTCA
CCATGTTGGCCAGGCTGGTCTCCAACTCCTGACCTCAAGTGATCCGCCTTCCTAGGACACACCACGCCCGGCCCCCAAAT
TGTCCTTTATTGCGTTTTGTCACAAGTAGGATTTCATCCAGAACCATGATGCTTGTTATATTTCTTAAGTCTCTTAATCT
CACTCAGACAATCCCCTTTTGATAATACCAACCAGATGAGGAATATAATGTGGTTTTAACCTGTATATGCCATGAAATAA
TCAGGTTACTTAATTTATTCAATTTTCTGTTCTGGAAGAAAACAACTTATTTTGATGTCTTTCTTTTAATTTGTATGAAC
ACTATTTCATTGTAAAGTTTCAATATTGTAACTTCACTATATCCCTAGATGTGTTGAACTTGACTGCTGGGATGGAAAAG
GTGAAGACCAAGAACCAATAATAACTCATGGAAAAGCAATGTGTACAGATATCCTTTTTAAGGTAACTTTAAAAATAATT
ACACAGACTTTTCCAAACTCTGTGAAAATGCAGGAAATAGGGAGATGGAGAAGAGAAAACAAATGATTCATTTTCTCAAG
CATTATGCCTTCATATTCCCTTCTTCCAGTCTTGAGGAAAAAGTCAAGACAAAGATCTTTTCCACTGAACGTCCCCTTCA
TGATACTGTTCTTGCTTTCAAATGGTGACTGTGTTACATAACAAGGTTTCTCTGGCAGAGTTTACCTAGTTTTACTTACC
TTAGAAACAGTGTCTCCTTTTGTTATCTACTTATTTGCTTATTTACTTTACATTATTCCTAAAGGACTTCAAGGTGGCAT
AAAAAGATAAATAGAATACAGGAATAAAATAGATTGTAAATAGGGCCTACAAGAATAAGAAAGGCCGGGCGTGGTGGCTC
ACGCCTGTAATCCCAGCACTTTGGGAAGCCGAGGCAGGGAGATCACCTGAGATCAGGAGTTGGAGACCAGCCTGACCAAC
ATGGGGAAACCCTGTCTCTACCAAAAATGCAAAATTAGCTGGGCATGGTGGCACATGCCTGTAATCTCAGCTACTCAGGA
GGCTGAGGCAGGAGAATGGCTTGAACCCGAGAGGTGGAGGTTGCAGTGAGCCGAGATTACACTGTTGCATTCTAGCCTGG
ACAACAAGAGTGAAACTCCATCTCAATAAATAAATAAGTAAATAATAAGATAATGAGGTTAGACAAATCAAAGAATGAA
ATTCTGTGGTTTGCAAATGTCAGCTGCAGCTGTCTGTCGGTTTGGTATAACTCAGGATAGATATTACAACACCAGGAGCA
GCGGTTCTCAACTCCACCTACATGTGAAACATCACCATGGGTATTTTCAAAAACATAGGTGCTGCTGGGCGTGGTGGCTC
ATGCCTATAATCCCAACACTTTGGGAGGTGGAGGCAGGTGGATCACTTGACGTCAGGAGTTCAAGATCAGCCTGGCCAAC
ATGGTGAAACCCTGTCTGTACTAAAAATACAAAAATTAGCTGGTGTGGTGGCATGCACCTGTAATCCCAGCTGAGGCAGA
GTCAGGAGAATTGCTTGAACACAGGAGGCAGAGGTTGCAGTGAAACCAGATCATGCCATTGCTCTCCAGCCTCAGCAACA
GAGCGAGACTCTGTCTCAAAAAAAACACACATACACACACGCAGATGTCTAGATGCTATGCATAGTATTTCTCTTAGC
CAAGATATTGGAGAGTTTTGACCAGCAATGAATTCAAGATAATACTTCTTGATATGTACCAAGAGTATATTCAAGTGGGA
AGCCATGAATTTGCATAGCCAGGAAGAATAACTAAATGACTACCTCAGCCACAAGGAATGCCTCAATAAAATATTTCAAG
ATTATCTAACCTTGGGCTTTATCATGTCAAATGGATTTGCCTAGATAACTATTGTGAAATATTTTTCTGATTAACTGAAT
GATGAGATTTGCCCTGTGATTAGCTACAAACCAGTAAGAATTTGGGGATTTTTACCAAATATGATGCTCCGGTTGTAGAA
GTGCTGGTTTGAAGCCTTTCTTTCTAAAGCTAAGCAGAGACGGGGCCAACCATCTCTTAAAGCCTCTCCTATTTCATGGA
GAATAGTGAAAGGGAAGATTATGTTTAGCTCCTTCAAATGTTGTTTTCTGTAATTAAGTTCTGTGTTACAGATGGGGGGT
CAAGAACACTTCTATGTTTCACTTACATGTTGCTTGACCAACGAGAACCCCTTCCCCCGGCCTTGTAACCAGGCTAATAG
GAAACAAGGGATACTGCAAATGTCACTTTAGTACACAGAGGTCTACATCAAGTTAATGAAGTATAGCCCAAGACTGATTT
CCAAACACAGAGGAAAGTCAGTGAAAGCCTCCAGCAGTCCTGAGGTTACTACCCACACTTTTCTGCATGAGGTGGGGAGG
AGTCATACAGTGGTCGGGAGTGAAAACCTCTGTGCCAGAGATCACCTGCTCCTCAAGAAGAAAGGTGGACTCTGAACTCC
TGGCCTGGCAGATTAACAAGCCACAGACTGGCATGCCATTTAGGTGAAATCTCTTGTAAGTTAATAGCACCATAATTCTG
GAGAAGTAAAAGATAGCTTAAAAAATTGTCATTAACCATTAACTGCACCCTGGCTATAACCACAGGGAACTCAACCTTTA
GAGGTGTCAAGGTAGATGGGGGCTCCAGGTCTGAATTGCTGGAATTGACTTTCTGAAAGACTCAATTAATAGTCTCTGAA
TAGTTTTGGGTGCCTTAATTTTTTTGCTCTTTTCTCTCATTAACGTTTTTTTTTGTTTTTAAAGGATGTAATTCAAGCCA
TCAAGGAAACTGCATTTGTCACATCAGAATATCCTGTAATTCTCTCCTTTGAAAATCACTGCAGGTATAATGATCCATTC
TGCCACAAGTCTCTATGTGGTATTGTTTCCTAACCCCTAATGCCCACTCCTGCTCTACAACTTTTTAAATAGCTGAATAG
TTTGTTTTGTGAAGTACTAAGTTCAAATAATTCTCCAGCAAGGGCACCTACTCTTATGCAAAGCAAAAAACAACAAAAAA
AGGAAAAAACGACAAAAAACCACCCTTGTCCCCCCAAACCAAATCATTTTAATGTGTTTCAGAAATACTTGTTCCAAATA
AAAGTTCAGAGATTGGGACACCTTACTTAAAGTTCCATTGAGCTGGAAGATTTGACAGTATATTTGAAATCAGATTTGAT
GAACTTGAAAGACATTTTTTATTCTTCTTTCCAGCTCTTAGTTTTGGAACAGAATCTCCTTCTCCTTCTTTTAAAATTAT
ACCAGTTAAAGTTTTTGATACATCTCACATTAGCATTAAGAGGACAATAATGCATTTTGCCAAGTGAGAATCTTTAATTG
CCTAGTAATGGGTGTTTCTTATTCTTGGACGGTTAATGGGTGTTTGAATTTACAAATGCCACTTTTTTCTCTCCAGCAAA
TATCAACAGTACAAGATGTCCAAATATTGCGAAGATCTATTTGGGGATCTCCTGTTGAAACAAGCACTTGAATCACATCC
AGTATGTATTTTTAACAAACCATATTTCTAGCATGAATGGATTTGTTTTGAAAATTTCTGAAGGGTCAAGTAGTACTAGA
GGACTAATGCTAAAGATCTTCATATTTATTTAAATTCTTTGGTACGGAAAGCTTACATCATGCTTCAGAGGGATGTAAAG
AGACACAAAATAAACATCTGGTTCAATTTCATTTGGAGGATCTTTGGGTCTTATGGTTTTCAGATCTCAGTCTTAAAGTT
TCATAAAGCACCCAGGCCAAGAGTCCAGATTCAGGTCAGTTGTTCTCAAATTCAGTTACATGTTAGAATCACCTACAAAA
CTGTTAAAAACACCTGTGCCAGGGCCCCACTTGGAGACTGATTTAATTGATGCAGGGTCAGCCCTGAGCATGGTGTTTTA
AAAGCCCTCCAGGTGAACTGTATATGCACCCGGTATTCAGAACCCATGGGAGGCTCATGCTGTGGAAAGTGCCCTGGATT
GATGTCACGACATCCTGAAGTTCATGTTCCTAACTCCAAAATAAATCACTGAGTAATGTTGCACATACATTTTTGAACTT
CATGTTAAACCTGCTAATTTCTTCATAAAGCAAAGAACATTAACCTGTGCTCCATAGATAGGCTTTATGAGATCTGTGAA
```

```
TTCATTCTAATTGCATGCAATTTTTTTTGTATATACTCATTCTAGAGAGTTAATCACATTTTTATAAGGATCCATGACCTC
CAAGAGATTAAGATCCACAATCACAAGATGTTATGAGCTCCAAGGCAGCTAATGAAATCAAAGTGGAAAGACCTGCTATT
GACAGCAGCACCTGATGGGCTCAAACCTGGTAGCATGAGGTAGTGGCAGAAGAACATCACAGCAAATGACAGCAGGAAGG
TGCTTTGTGCTACATGTAAACACTTTATGAGGTTCTTTTCAGAAGTGTGTCTATGGGAACTGAAAAAAAATATGAGAGAC
AAGGATATCTGTATCCTTTGTCCCCCTTCCTTCTTCCCTCGAACTTGCAGAAGCAGTTGCTGCAGGTGACCTGCCCATAT
CCTTGCAGTGCCTCCTGTTTCCCTGAACACTGGCCTGACTTTCCACTGCCAACATCCACAGCTTTTTGCCTGAGAGTGAG
CTCTGGTCACTGGAGCCTGCTCTGCCACACATGAAAGACCAGCAGTGCCAGGGAATTATCACCCCTATCCCATGCTTCAT
CCCTCCCGAGGTGGGATAACCCTGAGCACCTGCTCTTCTCTGCCTTCCAGGGTTTCCCAGCAGGATCGTGCTCCAGCTGG
CTGCAGTGATAACACACCCTTGATGAGCTGCCTGTCTTCTTTTTCTCACTTTCCCACACCTCTGTTGGAGTTTCCTTCCA
AGTAGACGACCTGGTGCAATACTACTTTTCTCTGAGGCTGATTTTAAGGGAACCCAAACTAAGAAACAGCTCTGATTTAT
TTTATTTGTTTTTACCTAGGCCCTCCATTTATCTTTCTGATTAGGTTAAGGTGATTGCTTTTGTGGAAAGTTCACTGTAT
TCTTAGTGAAGTGATGTGCACATACTTTTACCAGCTGAAGGGTGTCCAGGTTCTTGGCATCTTGAACAAAGAATCGGACA
AAATGCACAAACAAAGCAAGGGAAGAATGAAGCAACAAAGCAGAGATTTATTGAAAATGAAAGTACACTCCACAGGGTG
GGAGCAGCCTGAGCAGAGGGGCTCAAGAGCCCTGTTACAGAATTTTCTGGGGTTTAAATACCCTCTAGAGGTTTCCATTG
GTTACTTGGTGTCCGGTCTATGTAAATGAATAGGATGAAGTAAAGTTATAAAGTCATTTACTTGGTGTACATCCTTTGTA
AATGGAGAGGATATTTCCTGTCATAGCTGAAGTGTTTCCATTTGATTTAGTTCTAGGAAGTCAGCATGAATAGGCCTTAT
ATTCCCTGCCTCAAGACCCTATTCTCCTTTGAATACAGGACAGGTGTCTGGGGAAGCATGGGTAATATAAATGGGAATAA
GACCTGCTCCAAAAGCTAAAGAGCTTTCAACCTTGGGAGTGGGAGCAGGACACAAATGTGAACACATAACTAGAAAAGAA
GATGGCGTATGGTGCTTTATTTGTTGGAGGTGTCCTGGTACAAGAGAGATCACTGGGGAATGGGCAGTTATACCACGCT
GCTGGAAGGAAACAGCATTTCAACAAAGGGTTTTATTTGAGGCAAGACAGACATATGAGTGCACAGTGCCTTACATATAA
TAAGTAGTATAAAAGCAAGTTTTGAAAATGAATGGATGGGTGGAGGGGTGGGTGGGTGGGCATATAGAGAGATGGGTGGA
GGAATGGGGAATGGAAGACTAGATACAGGACGGTTCTGTAGACCAGAGGTGATAGCATACAGGGGCACCTCCTTTGCAGA
TATAAATCTAAGCAAATGATGCTGTAGTCAAGTTCAGATTCCAGGAATTGGAACAACTCCCTTCTTGATTCCCTTATCAA
ACCTACAAAACCACAAAACCACAATCTCAAGTGTTGAGGGTTGGGTCAGCCTACTCAGCAGACAGGCTTACTATACAGCC
TGACACTTGGGGGGATCTGGGGGGCCTCTCACTTGGGAAAGCTAATTCTGAACCCAGCCATCCACAATTGTCCCTTGCACT
TTACCAGATACTTAAAAGGTGCAAGGCCCTCTGGGGAGCAGGAACCCCTCCCCGGCACATCCCAGGCCAGACCCCTGCTC
CACTGGGGAGGTTGGAGCAGCTCTGCACACCTCTGGAAACTATGTGGATTTCAAGTTTGCAGCACTAGTCTAAGGAAAAG
CTCAATGGGTGTGCTAATTTGGGTTGGGGGAGTGAAAACAGAAGGCTGTGCCGCCTGCCATCTAGTGGGTGTTGGGTTGCA
TCACTCCCAGGCCTCCCAGGCTCCTCCTGGACTGGGAAGGAGAATGGAGAAGCACAGCCCTTCCTTAATTCAGCTCTTTC
TGTTTCTCTCTAGCTTGAACCAGGCAGGGCTTTGCCATCCCCCAATGACCTCAAAAGAAAAATACTCATAAAAAACAAGC
GGCTGAAACCTGAAGTTGAAAAAAGTAAGTGAAACACACACATTTATAATGGAAGCATACATAACATGTGTGGACATTTC
AGCTGTTGAGAATTCAATTTATTTTAAAACCACTGATTTTTGGGGGAAGGGTGTAGGGGTGATGTTACAGGTACTTTTCA
GAGCAAGATTGCCACATTTCCATTTAAATTTAATTTCCATATCAATTAATTCATGCAGCAGGAAAAGCATATCTATATAT
TCAAATGCCAGAATTTGGAGAGGGCCCAAGGAAAATTGAGAGGAAAGTAGAAGCATTTTAGAATTTCTCAAGATATAAGG
AACCAGCATTTTATAATTTTATACACTCTAAAGTCCTCATAAGTTTCCCTTTTCAGTTTGCCCCGTGCTCTATACCTATC
TAGGGATACTAGGTACATATGAGTGTGTGACTGTGTATATGTGAATAGAGAGATCTAGAGACAAATGATTTTATATAAAC
ATGTTTCCTTTTATTCTCCCAGGATGATTTCCTTGAAAATATTTTCTTTCAGAGTATATCCTAGTTCAGTGAATTAATGG
TATCAAATATTGGCATGATTACACTTCATTTTCCCCCCAAGTATTCTAAATGAAAAATTTTAAATTATGTTAATGACAGT
TTATTTTATTACAAAAGCAATAATGCCATAGAAAAATCAAGACAATGAAAGTGTAAGAAAGTAACAGCACTAACATATA
TTATACACGTATTCATATACTATAGACACATGTACGTAATGTACATGTGTAGATATATACCCACATTTTTAACATGACTG
AGATCATATTAATGATACTGAGTTTAAATTTTTAAAATTTAACCAAGTAAGTATACTTACATCAGCAGGATATACATCTA
CATACTAACTTTAATCCTAATGTATGAAACACTATATAGAATTTAATATATATGACATAGAATTGTATGCATTTATTATA
GCATGATGTAACGAGTCCCAAGTTGATGGATTTTCACTACTATAAACATGCTTGAATGTTATTTTGTATGTTTGTGCAAC
CCTCTCCTTGAAATCAATTGGTGCAATTAGAGTTATTCAGCCAAAACGTATATATATTTTATATTGTGATATAGTTTGTT
ATGCTTTCTTTTAGGAAAGTTCTACCACCTTAGACTCTTCACCAGTCATATATGAGAGTGCCTGTTTCCCACCAATATCC
TTAATGCTGGCTGTTGGCCATTTTTAAATGTGTGTCCAACTCATAGAAAAAAATTGGTACCTTAGGGGTTTTTTTATTTG
TGTGTCTTCAATAACTCCTTTTTTTGCATATTACTATTTGTGGTTTGTCCCTATGTGTTTTCCCATTTTTATTATTTTGTT
TACCTTTATCTTTCTGACTTATAGAAAAATTATTTAGTCTTATTTTGTATAGTAGGATATAATCCCCATTAAATCAGTTG
CAAATATTTTTTCCTGAAGTTTTGTTGCCAACCTGCCTATTTTCTATCAGTTCCCCATAACTGTTCATAAACCTTGGTCT
GTGCCAGATTCTCCAAATTATGGGCTTTTGTTATCTAGCCTTAAATCCTTCACGAGGACTGAACCTCCTCTCAAACTTGG
TGACCAGAATTTTGGGCATTTCACCCATGTCCCCGCAATTCTTCATCATGTGTATATAAGGTGGTTGTTGCAGGTTTCTT
ACAAGGTCGAGACCACCAGGAAATGGCGAGAGAGAGAACACTGCTGAACCTTAGAAACATGGTGCCTTGGTGCCTGAACA
TTCTATTTCTTCTCTCTTTTCACGTCCTCCTCAGTGTCGATCTCAAGGCCTAGGGTTTGTATGTTACCTAGCATCTGTCA
CCATCTCTTTGCGTGTTTTTGCTAACAGAACAGCTGGAAGCTTTGAGAAGCATGATGGAAGCTGGAGAATCTGCCTCCCC
AGCAAACATCTTAGAGGACGATAATGAAGAGGAGATCGAAGTGGTGAGCTGTTTGCTTTTATTTTAAGTTACATGCTTT
GAAAATACTTTTTAAATAAGAAACCAGGCTGGGCACAGTGGCTCAAGCCCATAATCCCAGCACTTGGGGAGGCCAAGGT
GGGAGGATTGCTTGAGCCCAGGAGTTCAAGACCAGCCTGGGCAACATTTTCGTAGAGATGAGACCCCATGTCTACAAAAA
ATAAAATATTAGCCAGACGTGGTGGCCTGTGTCTGTAGTCCCAGCTACTGGGGAGGCTGAGGCAGTAGGATCCCTTGAGC
CCAGAGGTTCAAGGCTGCAGTGAGCTATATGTAATCCTACCACTGCACTCCAGGCTGGACAACAGACTGAGGCCTTGTTT
CAAAAATAAATGAGTAAGAAAACCAGGAATTTCTTTCAGAAGACCCTTTTATATAATAATGCCGTTTTAATTCCTCATAG
TAATATTTTGGTATTCTTGGTATTTTGCCAAACACTTCCCATTTTTTCTGGCTACTAAATTTTAATGGCCTTGTCATCTC
```

```
CCTTCCTATTGGCCATTCAGCTATTCAGCAGAAATTCTTAGCCAGAGAATCCCTGAAGCACTTGCTGTAGTAGGCAGAGG
AAGCAATATAAGGCACATTTTGTAATCCCTTTGTATAGACAAAAACATGAACTTGAGTCTGTTAATGATGAGAGTAGAGA
GGCAAATAATTTAAGAGACCCTCAGGCAAATGATTGGTTTGTCTCCACAAAGTGAACTAGAAACTAAAAGCCAGTATTAA
AATTTCAAGTTCAAATTCCTTCAAATAACCAGCTCACCAGAATTATCTCTGTGTAGCTAGATAATTTTAAGAAGGCAGTC
TTGCTTAAAATATTTGAGTATGTGCTATTACCTTCTCTTTTACTTTGATCACATAGCAACAATCATAATGTTCAGTTGTA
AATGGGTCATTTGAGAGTTTGGGGATGATTTTAGAGAATTATTTGTTTGTTATGTTTCATGAGAATTGATGAACGAAATG
ACAAGGACTTGTGTTGGTTATACAATGCCTTGGATTTATATACAGGCATACCTTGGAAATATTGCAGATTCAGTTCTAGA
CCACTACAATGAAGTAAATATTGCAAAAAAATTATTCACAAGAATTTTTGTTTACCAATGCATACAGAAATTATTTACAC
TGTACTGTAGGCTAGTAAGTGTTCAATAACAGCATTATGTCCAAAAATGTAACTTAATTAAAAAATACCTATTGCTTAAA
AATGCTGACAATTATCTAAACCTGCATCATATTGAAATCTTTTTGTGGAGGGTCTTGCCTTGAGTGATCAGGGAGGCAGT
TGCCAAAGATTGGGTTGGCTGTGGTAATTTTTTAAAATAAGACAACAATGAAGTTTGCTGCATCAATTGACCCTTCTTTT
CACAAAGATTTCTCTGTAGCATGCAGTGCCATTTGATGACACTTTACCCAGAGCAGAGCTTCTTTCAAAATTGAAGCCA
ATCCTCTCAAACCCTGCTGTGATGCCTTCTCAACTAGGTTTATGTAATATTTTAAATCCTGTGTTGTCATTTCAACAATG
CTCACAGTATCTTTACCTGGAGTAGATTCCATCTCAAGAAACCACCTTCTTTGCTCATTCACAAGAAACAACTCCTCATC
GGTTTAAGTTTTATCATGAAATTGCAGCAATTCAGCCCCATGTTCAGGCTCCTTTTCAAATTCTAGTTCTCTTACTCTTT
CTACCACCTCTGCAGTGACTTCCTCCACTGAAGTCTTGAACCTCTCAGCGTCATCCATGGGAGTCTCCCAAACTCCTATG
AACGTTGATATTTTTACCTCTTCCCATAAATCATGAATGTTCTTTCTTAATAGCATCTAGAATGGTGAACATCAGAAGCT
TTTCAGTTTACTTTGCCCAGATCCATCAGAGGACATTCTCTTTTAGGAAAGCTAGACATAGAGCTAAAAATCCAAAAGTT
GGAAGAATTAACTAACCTTATTCAAGACTCCTTAATAAAGACTCCTGACTGTGGAACAGACTTTATAAGTGACTCAGTTG
CATGTCCAGTCCTTGGGTTTGACATTACTCCCAGAGAAAGTCATAATGACTGTACTTTCATTTGTTTTATGTGGCAGGG
AGGGTCTAAGAGGCTAAAGATATCTGATACTACTCAGTTGATACTCGTTATTATGGTCATCTGTTACTATGCAATAAACC
ATCCCCAAATTTAGGGGCTTATAACAACACCTTATCATTTTCTGTCATAATTCTGTGGGTCATGTGGGCTTAGGTTGGTG
GTTCTCCCTTGGGTTTCTCCTGCTTTTGTCAGCAGATGACATCTGGGGCTGACTGGTTCCCTTGGGTGGGTTATCCCGGA
CGGCTTACTCACATGCTGGCAGTGGATGCTGGCTGAACGCTGGGAGCTCAGCTGGGCCTCACTGACCATAGTATCCCTGT
GGCCTCTCTCTGTGACTTGGGCTTCTCATAGCACGATGGCTAAGTCCCCAGATGGGAACATCTAAGAGCCAGTGTCTCAA
GAGGCAGGAAGCAGAAGCTGCTAAATTAGTTAAGAGCTAGGCCTGGAACTGGTGCAGTATCACTTCCTCTATGTTTTATT
CATCGAAGCAGTCTCAGGGCCCATCCAAGTTCAAGAGGGTGAGTGAGTGACAAGGTCACGTTGCATAAGAGCACAGAGGA
GGGCAGATGATACGGCAGCTGTCTTTGGAAAATATAGTCAGCCGTGATGCCTACTATATTTTTTCACATGTTTTTGCAAA
TAATAAATCTAAACAAGATTTAAGAAAAATATATTTATAGAACTGTGTTTCAAATGGTCAAGAAAATACTCCTAAGATAC
AAAAAAGATGACTATTCAGTAAAACCAGTTCCTTTAAGTTAAATTTCATAGATGGTAATTAAAATATGTAAGGCATTACT
GATACGATAATCCAGTAAAACAAACTGCAGAAATAATGGAATAAATTAACAAGAAAACAATGTTAATAATAATTACTACC
ATCGTTGTACCAACAGTGGACCAGGCAGCTTACATTATGGCCTTATCTAACCTGTATACAGCCTGATGAAATGGGTATTA
TTATTATTATTATTTTTGAGATGGAGTCTTGCTCTGTCACCCAGGCTGGAGTGCAGTGGCGCGATCTCGGCTCACTG
CAAGCTCTGCCTGTGAAATGGGTATTATTAAACCAATTTTTCAGATGAGAGGACTGAAGCTTAGAGAGGTAACATATTTT
GCACATGATCCCACAGCGGGCAACCGATTGCACTAAGATTCAATCCCAGAGCTACCTGATTCAAGTCAAGACTTTTGTAC
GTTACAGTACTGCCCTTTCAATTCACAGGAATATGGTGAAAACATAGATATGCAAAAATACTTTTTTAAAAAGGAGAAAG
AATAGAATGAATGAAAGTCAAAGATGTGATGTTTCCTAAACACACACAGTCATCTGAAATTAGCATCTGTATGGCTTCAT
GTATTATTCTTAAAGGTGCTTTCCCACCATCTACATCCATATGGTAATTATTCCTCCAGGCAGAAAAATATTTGCCTCTA
GTGACTCCCATGTTATTCCAAAATATACTTGAGATTTGGGTTAAGTCTTAACAGAAATAACCACATGATTTAGTATCCAC
GTAGGAAAGTACCTAAGGACAGAATCATCCACCTAGATGGCACAAGGCTCCTGTTTCACCTGTTATGTGTGTGATTCTTA
GGTTGCCTCAGTAATACTTGGCTTTGGTTCTGGTCAACTAGTCGGCTATAATTACCTTAGTGCCACTGTATGAATCTACT
ATCTATGTGTGGCTAGAGTCTCTAATTTAAATACATCAACACAGCAGGCAAGGGGAAACCAAGCATAGTTCAATAGGGGA
GGCCTGCCTGGAGGCTGTAGAAAATAGAAAAGGCTTTCAGTCAGTGAAAGCAGGTGCCCAGGCATTAGGCTGGATTTAGA
TTCAGAAGGACATGTGGCTCCCAGGATAGGAGACAAGTAGTGGAGAATATCAAAATTGTATGTGGTTATAGGTGTGTCTG
TGCATATCTGCACACTCAGAAAGTGATCATGTATTCATACAATTCTCTTAAGGTGGTTAGTAAGTCTCCACCACTTGTTT
GGGGTATGCCCCTTGCCTTCAAGTGGTTTACATCTGTATTGGCAGTTTAGACATAAATTAAATTAAATGAAATGAAATTA
ATAAATTAAATGAAAATTTAATAGTGACATGAGAAGTTTTGAAATCTTGGTTACTGACTGTAGGTCATGGAAACCTAGTC
CTGGAAGAACTGTGTTAAGAATTCAAGAGACAGGTAGATCCATTATCTTGCTGTGTGATTTCAGACAAGTCAAATCATAT
CTCTGCATCTAGTTCCTCGTTTATAAGTAAAGACAGATGCTGGATTAGATTATTTTAAGATGTATTCCATTTCCTCAAAT
CCTTTTCTTCCTGCCTAAGCCTTACAATTACCCACTTGTTGTTGTTGTTGTTTGGATTTTGTTTTTTTAATCGTCTG
CCTCTAGAATATCCCTTCATCAGAATTGTTAGCTCCAGAAGAGGAAGGTGGCTGTTTCTTTCCCTCACCCTCTTCCTGTG
CCTGGAGAGATACTTCACAAAATAAAACAGTCTATCTAGAAATTCTGAATCCAGATACTACCAGTTGAAAGATACCACTA
TTTTATTATGACTAAGAAAGAAAAAGTAGTTTCCATTCACTGGAAAAAACCGTCAGTTGAAGAACACACTACAATATCAG
AAATATTAAATCAAAAAAAGTACATCTTAGAATCAGTGAAATCATTTTTCATTTACTCCTGTTCTTTCATTCTCCTTTGT
ACTTTTAAACATGCTTTAAAAAAATCTATCTCATTCTTATGGGTCTACTCATTTCCTTTCTGAATTTCTCAGAAATTATT
TTAGTGGGTTTTTTTGTTTTGTTTTTACAGTAACGTTCCCTCATGCCCTGTGGTGTGACCATGTGGATTTATATTGTCCT
CATCCTGATGTCTGTGGGTTTCTCCTGATCCTGGCTAGATTTTAGGCAAATTCTTTATCTGGGAATTACTGCAAATAGTG
ATTGTGTGAATCCAAATCTCACCTCAGCATGTTGCACATTTTCCAGCAGGTACTTCTTTTTTTTCCCCACGAACATCCTG
GGTGGATTCTGGCATTCTTGTCACTTTCCTGGACCAATGAACACAGTGTGTGTACTTCCTTTCACAGACTAGACCACGCT
CCAGGGACACCAGCTTTATTTGGCACCCACTTCCACTTCTCCTCCTACCTGAGCCTGACAAATGAGCCCTGAGCCTTTGA
AACCTATGCTAGGTTTGACAAATAGTTTGCAGGAGTAGCACAATCCTTCTGAGGCCTTTCTTTTTTCTACCACTCCAAGC
```

```
TATAAGTCATTATGCCCATAAAATTCAAGTGGACAGTTTAGTCTCTTTCAAAATTATCCGATGTATTTGTTTTCTTGCAT
CTTCTTATTTGCTCTTGATGTCATGAAAGATCAAAACACCTTTTTATGAGTTATAGATATTCCTGCAGTGTTTGAGGCTT
TCATTTTTAGAATTAGTATACTAGAATAGAAGTAGCCATCCACTTAAAAAAAATTTATTAAGCATCAAGCATTTCAAACC
ACATTTTAGGAGCAGGAGAAATAATACATAACAAGATGTGCTCCTTGACGTTGAATAGGTAAGAGTTTAGTGAGAGTTTT
ACTTGAAAAAATACTGCATATATCAATGTCCCCATTTTCTTTTAGCATCCTTTTTCTCTAAAAGTGCTTATGTTCTACCC
AAGATTGTGAACAAGGTCTGACTACTTGGCAGTGGTTTGGTGTCATGGATTTTCCATTTGTCTTTCACACAGTCGACCAA
GAGGAGGAAGCTCACCCCGAATTCAAATTTGGAAATGAACTTTCTGCTGATGACTTGGGTCACAAGGAAGCTGTTGCAAA
TAGCGTCAAGAAGGTCAGAGTCCCCTTTCTTTCATCACTCTCAAGAGGTAGCAGCTGTTATTTATGAAATTTTGGAAATG
TGAGAACCACAAGAGAATAAAGTAACCTATAATTCCCCTTCTAAATTGCTTAGGATACGAGTCTGTGCTGGGTGACCAGA
ACTTGACACATACACAATATTAAATTTAAAAGGACATTTAAATTACTCATTAGTCAGGGCCAGTGTTAACCACTACCCAT
TTGGCCAGTGTCCTCTAAATATTATCATTTATTGTGTTATTGCAGCTGGGGAGGGAGAAAATGACAGCATCCCAGGGGTA
AGATTTAATCTTGAATTCATCAGGAAATGACCCCTGAACATCCCGAGTCTAGCCCTCATTTGAGAACTAGTCCTGCTAA
TTATATACCTTCCCCGTAAAGTACAGTAAGCATAAAAATGTTTCCAGATATTCAGCCAAGGAAAATAGTTATTCATATAC
TTACCTTTTATCAGGCATAGTCTAGGCCATAATGACCTAAAAGTGTAAAACACACTTGCCTTCTGCCCTTAGGCAGCTCA
TGGCCCCCTGTAGTTTAGGGAAAGGGATCGGAAACAGCAAACAAGCAAGTATTCAAATAACTATATAATTACAACTTACG
GTGAGTGCAATTATGAACATAAACACGAATGCTGAAGAATAATGAAGTAGAAAGAATAAGGAAACCAGAACGCAAAGGGT
CAAGAGGAATTGGCCATGCACAGAGGTTGATGAATCACTTTCCATGCGGAGGGACCACAGTAACAACAGCAAGGGCTGTG
AGTAGAGTGGCTTGAGTGAGGGTGGAGTGAGACGAGATGAGGCAGGTAGGTGGGAACTGGGTGATGTAGGATTTTGGAGG
CCATTATGAGAAATTCAAATATTCAGTGAAATGTGACACTACTGAAAAAGTTTAGAGAAGGGGGCAGTGTAGCCTGATTC
AAGTTTTAAAAGATTGCTGTGGTTGCCATATGGAGACTAGTTTAGGAGGAAACAGGAGTGGAAGCAGGAAAAGCAGTGAG
GACAGGCTGCTGCTCTAGGCCAGACGAGAGTCCCCGATGGTCAAACCAGACAATGCATCAAAGAGGACTGAGTGGCTTCA
AGACATATTGAGAAATGGGACAGCCTACTGATGGATGGAATGTGGGAATGAGAGAGAAAGAAGAAGTGAGATGACCCTCT
AGTGTCCTTGTTTGAGGAACTGGGTAGGTGATGGTACCATTTACTGGCATGGGGAAGACCGAAAGAGAAAGAATTCAGGA
GTTTCAAATGGGATGACTATTTGAGATGCCTGTGAAGTATTCAAGGGGAGGTGTTCATATAGCATTGCCATCTGACTCTT
TCTCTGGGTTCATTTCCTAACTGTCTAACTTTGGGTAAGTTACTTAACCTCTGTGTACCTTAGTTTCCTCATCTGCAAAG
TGAAGACGATAACACCTATTTCATGGGGTCACTGTGAAGAGTGGATGGTTTGTGACATGTCAAGAGCTTAGCGTGGTGCC
TGGTACATGGTAACTCCTGTAAAGGTATTAGTTGCTGGTATATTACTCATCCTTTTCATCTTTATCATTGTCATTATCCTC
ATGATCACTGGCGTCATTATCACGCGTATATAGAAGACAGGTTGAGCTAGAGATACAAGTTGGGAGTTCAAAAGATCGTA
TTTGAAACAATGGAAATACTGAGATGATTCACAAGGAAAGGTGGAAAAGAAGGCCCAAGTGGAGCCCTAGTTAAAAGTGG
AGCCAGAAGAAGAAGCAAGGTATGGCTAGAGAAGTAGGAGGGAAATTAGGCAGTGACACCAAGAAAACCAGGAAAGAGA
ACAGTGTTAACAGAAGGACTGAGTGGTTGACTGTAGAATATGTTAAAAATCTTAGACAAATTAAATTTAACAGAGTTTAG
TTGAGCAAAAAAACAATTCCCAAATTGGGCAGGACCCAAACCAGAAGAGGTTCAGAGAAACTCCAGTGCAGCCATGTGG
TGGAAGATTTTTTGGCAGAAAAAGGAAAGTGACCTACAGAAAACAGAAGTGAGGTACAGAAGCAGCTGTATTGGTTACAG
GCTTATTTGCCTTATTTGAACAGAGGTTGAATAGTTGGCCACCTTTGATTGGCTGAAACTCAGTGATTGGCACATGAGTA
GGTTCCAGTCTGTTTACACACCCATTCAGTCTACAGTTTACTATGTGAAAGTTTATGAAAAAACCTTTAGAATAGGTAAA
GAGACACCTTTAGGCTAAACTTAATTTAACAAATGCTACTGACTGATTAAGTAAGAAGACTCAAGAGTGTCCTTTGGGTT
GTTAGCATGGAGGTCGTTGGCAACCTTAAAGAGACATTTCAGTGGAGTGCCAGGCAGGGTGGTGACACTGGATCACACAG
ATATGAATGACTAGAGACTATGCTTTTGAGAAGTTTTGATGTGAAGGGGAGCAAGAAATGGACTGGTGGCCAAGGGCACC
TGAGGAGTCAAGGGAGTGTTTTCTTTTTCGGATGAATGAATCTGGAGTGTGTTTGAAAACTTGGAAGTGGTGCAGTAGAG
AGGAAGAGATTAGTTTTGCAGGAAAGAGAGGGGATAACTGCCTGGAATTCCTTGAGGAGGTGGAAGAGGTGGGATTGCT
GTGGTGCAAAGGTGTGGCCCTTCACAGGAGCAGGAACACCTGATTAGTGAAGACAGTGGATGAAGATGCAGGTAGGCAGA
TACATTTGGTGATAGGAAGAGGGGAGAGTATTTGTCTGATGAGCAAAGCCAAAGGAATATTGCCTTTACACACTATCTTT
TCTGATATAGGGAATTGTGACTACTCTGAGAATATTGAACGTCTATTAAGACTCAAGAGTATTGTTTTAATACAAAAATT
AGCCAGGCGCCATGGTACGCGCCTGTAATCCCAGCTACTCAGGAAGCTGAGGCAGGAGAACTGCTTGAACCCAAAAGGCG
GAGGTTGCAGTGAGCCAAGATTGTGCCACTGCACTCCAGCCTGGGCAACAGAGCAAGACTGTGTCTCAAAAAAAAAAAAA
AAAAAAAAAGTTGCTAAAGCCTTCATCAATATTGTCTTAGTGTCTTAGTCAGTTGGTGCTGCTATCACAAAAATACCATT
GGCTTAAACAACTGAAAACTATTTGTCACCATTCTAGAGTCTAAGAAGTCTAAGATCAAGGTTCCACGCAATCAGTTCCT
AATGTGGGGGGCCTTCTTCGTGTTTATGTCCTCATGTGGTGGACAGAGAATAAGGAGGCAAGCTCTCTCTTGGCTTGGCC
CTCATGACCAAATTACCTCCCAAAGGCCCCATCTTCAAATGCCATCACATGAGAATTAAGGTTTCAGTATATGAATTGGG
GACAGTATGGGGGGCTGCACCAGCAAACAGTCCATAACAAATACCCAGACATGAATGCTGAGTTACTCAAGAGTGACCAT
TCATGCTGCTTTTCCTAAGCCCCATGACTAAAGCACTAATTAAATAATTATAATAAAACATCTAAGTAGGTAAAGACTGT
CCACGAAGGAGGGAAAGAGCATTAAGATTAGCCCTTCTGGTGCATAAAACAGTGAGGCCATCCATGGCTTTTTGTCATCA
GTAATGTCCCACCTTAGCAAATAAATAGAATAACAAGTTTACCTACACTTATTTAAATATCTATGTATGTATGGCTAATA
AATTTGGAATTTTGCCCTTTAGAGAAGTAAACAAATTATTTCCTTTCTCTAATTAGGCTTCAGATGACCTTGAACATGAA
AACAACAAAAAGGTAACAAAATAATTCCCTTGCACATTTTAAATCAGATGCATCTAATTTAAAAAATCTTCAAAGGAAGG
AATCAGTTGTGGTGAAAAAATGATTAAATCCTGATAAAAATCACAAAACTAGGGCAGATGAGTAAGATGATTTTCTAAAA
GTAGGTATTTAATGCAGTAAGGCATTTTCTTCACTGGAATTAGAGATTTCATTCCTGCAGTGGAACTACATGATCATCAG
CATATTGAATTTGAAAATAAAAATGAGTGAATAGGTCATCAAATCCATATTTATAGCATAATAGAAAGGATGATCTTAGTT
CCCACTACATTGACATTCTGGAAGATCACTGATTAATTTTACACTGCATAACTTCTAAGAGTATTATTTGATGTTATTAG
CTATTGGCCTTATTGTAGGAAAGGTAAAGTTACATTTTCACTTTCTTTCATCCTCTTCAAAGTTCTTCAAGTGATCACAG
TGGTTTTATTTAGTAAGTATTTATGGTTTATCCTACAACAGTGAGGCTGACAGCCACAGGCAGAAGTTTGCCTAATAACT
```

```
TCATTCTCATACTTCAGAGGTTCGGAAAATACTGCCTTGCTGCCTGTGGGGTCCTATCCTATTTGAAGTTGAGATCGTAT
CTTCTCACCCTCTAAATATTAAACTTGAAAGAATTATGAATAAATATGGTCGCAGGCCTGTCCCAAAATATTTGTGAGTA
CATTACTCTCTTGTTCTAGAATTTCATAATTCATAATTTGGGTCAACAGTATACAGATTGACATTTGGTCATTGTCATTT
ATTAGCTAAGTTATTTTAATTCTCAAGGCATCAATCTTCTCATGTGGTCAGTGGGTAGGACTCTAATCCTCACTACACAT
CACAGACAGTAATGAGACTGTGTGTGCAAGAACTCATCCTGGTGCTCAGCTCATAGCACTGGTGGTATTTATTATTATTA
AACACACATGGAAGGAACGGACATGGCCGTTGTTTGGTGGGGGAGCAGGAGGACACATTAAGAAAATGATAAAAACAAAA
ACAAAACCAAATTACATTACTTGATTATTGTTTCAGGCTTTTATTGGTGAAATCAGTTTGATAATTTAGAATGCTCAGAT
TGTTAAGTGATCAGTTAAGACAAACAAATTGACCATTTTTTTTTTTTTTTGAGATGGAGTCTCACTCTGTTGCCCAGGCT
GGAGTGCAGTGGCGTGATCTCCGCTCACTGAAAGCTCTGCCCCCCGGGTTCACTTCATTCGCCATTCTCCTGCCTCAGCC
TCCCAGTAGCTGGGACTACAGGTGCCTGCCACCATGCCCAGTTAATTTTTTGTATTTTTAATAGAGATGGGGTTTCACA
GTGTTCACCAGGATGGTCTCGATCTCCTGACCTCATGATCCACCCGCCTCAGCCTCCCAAAGTGCTGGGATTACAGGCGT
GAGCCACTGCGCCCAGCATCTGACCATTTTTTAACTCATGGATTACATCATCCAACTAAGGAATTTATCCAACTAGATTC
ACTTTATGCAGGAAATTTAGCTTTGCAAAGTTCTCAGCAAAAATATTGATCACTTTTGAAGGTATTTTTGTTGTTGGTTT
TATTGTTGTTTTTTCTATCAATAAATAAACTAATTTGTTTTCTTTTTTCTGTATGCAAAGTAATGCAGTTTCAATATGTC
CTATGCAGCAAGTTATTAGGTTACTTTTAAGATTAAGTGCTTTTTAAATACTATAAACCACGTATGTATGTTGCTTACAT
TCTAGAGTGCTACAGTGAAACATAGTCCCCAAAGAGGCAGCATCATTAGCACCTGGGAACTAGTTACAGCAATGACTGGC
CTCACTGCAGACTCACTTCAACAGAAACTCTGAGTGTGGGGCCCAGGAATCTGTTCTAGTTTAAAAAGTCTTCCAGGTGA
TTCTGATGCACGCTAATGTTCGAGAGTCACTATTTTTGATGTTGTGCCTCAATTTGTAAAAATATGATGCTATATCTATT
TCATAGCATTGCTGTGAGGTCCAATATAGTAATTTCTTTTTTTTTTTTTTGAGATGGAGTCTTGCTCTGTTGCCTGGTC
TGGAGTGCAGTGGCACAATCTCAGCTCACCTCAACCTCTGCCTCCTGGGTTCAAGCGATTCTCCTGCCTCAGCCTCCCAA
GTAGCTGGAATTACAGGCTCACGCCACCACACCCAGCTAATTTTTGGCATTTTTAGTAGAGATGGGGTTTGCCATGTTC
GCCAAGTTTGTCTTGAACTCCTGGCCTCCCAAAGTGCTGGATTAAAGGCATGAGCCACCGTTCCCGGCCAGTAATTTCTT
GAATAAAGTCCCTTAAAAATCTAAAAATGTCCTACAAATGTAAGATAAGCTTAATATTTTATGATTACCTCCTTTTGCAT
TATTCAGAGCTACAACAGACACTAACTTCTTAAAGTATCCTTTAGCATAATTGAGGAAGAAAAAGCCTTTACCTTTCAAT
TTGTGTGTGTGCAATTATCTTTATTAAGGAAATCTGCAGCACGTATCCGTGGGTCCTACTGAAGTCATCAACTTATATGT
TTTCTTCCTTGTGCTTGTAGGGCCTGGTCACTGTAGAGGATGAGCAGGCGTGGATGGCATCTTATAAATATGTAGGTGCT
ACCACTAATATCCATCCATATTTGTCCACAATGATCAACTACGCCCAGCCTGTAAAGTTTCAAGGTTTCCATGTGGCAGA
AGGTAACACCAAAGGTTAAATGCAGTCGCCTTTTTTGCTTGATTTTCTTTTATGGTGCTGATGAGCTTTTATCTAATTTG
TTGCCATTTTTCTTTGTGGGCATGGGGGCTGTTCCACCTCACCTTATTTTTGTTTCACAGACCATAGAAGCCACCTTGGT
ATGTGGTGACATTACATTGTCATTCACGACTTCAACCACAAGGTGGCGATGAGAGACTATGGATTTGTTAGTGCTGGGCA
TGTAGCTTCTTCTGTTCCCAAGTGCATTCACAAGACTCCTTTTAATCAAAAGCTGGTTTTCAAAAAAAACATAAGAAGTAT
TTAAAAACTTCATTTCTGGGCTAGAGAAGCCATCGTAATCATCATTATCATCAAAAATTCATATTTCGGTGGTGGCTGAA
AAGACAGATGTCTCCATCCACAGGATGTCAATGAAAGTAGGAGCTACTGACATGATAATGTAGCATTAGCCTCCAAGGCC
TCATGATTCAGGGTGTAATTGCATTTATTTATTGCTGTTTTTCGGTGAGGGTTTGATGGCAGAGTTCCTGAATCCATCTT
TGCTTTTCTTTTACAGAACGCAATATTCATTATAACATGTCTTCTTTTAATGAATCAGTCGGTCTTGGCTACTTGAAGAC
ACATGCAATTGAATTTGTCAAGTATCCTTATGTATCAAGTGGAATGAGTGGTTGACTCACGTTGGTTTGGCAAGAGCCTC
TGAAATAGATAAATGGCTAATATCTCATTCTGGTGGTGGAGTTCATAAAATGATATGTGGGAAAGACCATGGCAGCTGTG
ACAATCTGCCCAAATGAAAATTGTTTTTATAATCCACACAGTGAACTGTGATCTTAAGCATCATTTGTAAATGTGAAATC
TGCTCTGTGCTGTCTACTTGTTTGTCATTGTTGGCCTAGACCTTTGTTGTTTTAGCTCTTTGTTGTTTATTTTTCTCTGC
TGTAGGACTCTTTTTTTTCTGTTTTCCTTAATAAGTTACAGTTATAACAAACGGCAAATGAGTCGCATTTACCCCAAGGGA
GGCCGAGTCGATTCCAGTAATTACATGCCTCAGATTTTCTGGAACGCTGGCTGCCAGATGGTTTCACTGAACTATCAAAC
CCCAGGTAGGAGCTGATGTCCAGTGACCCCAAATTCCATGAGAACACTTTGACAGGATGGTGCCAGCACTTCCCATCAGT
TGGCTGTGGGCATTTTTTAAAGGAAGCAGACATATGGCAAAGCAATGTGTTATTGGATTTATATAATGGTGAGAAATAAA
AAAAAAAAACCAAAATAGAGCAAACAATGGTAGAAGATAATGTGTTGTGATTGGAATTTAATTCCTTTCATAAATGCCA
TGACTATTCCTGCTAACCATTATCCCATATTCCAAAATATTTCTCATTTTTCTTCCTTTTGGATCAAAGTTGTTATCTTT
GTTTCCAGAGGCAGAATTAAGTGAAAAGAATTTGAGTATCTTTGTACAGTAACAGCGACTCATTTCTCCTTTGGCAAACA
CCCTGACCACTGTGAACATTCAGCAAATATGACTGATGCAGGGGCACTTCACTAAACAAATACAGAATATTTTGAAGTAA
ATGCCACTAAAATGGTATATAAGGAGTTACATATTTGAAGAGGTACTAATCTATAACTTGGTCCTCCAAAGTGTACTCAA
AGTCAGGTTTCCAATATATAATGACTTTTTTTTTCTGGAAGGAAAAGAGTAAATTTTTATAATTGATAAACACATTTTTA
AAATTTTTGAGGTTTGCTTAAGAGCCTAGGATCACAGTATCAAGACTAGAGGTAGCAAAATAGAAATAGAATAGATAGAA
ATATGCCACAAACCCTCTTCACTGTAGATGGTCAATTCTACAGCATGGTTCTTACAACTGGTAAAATAAAACAAAACCAG
AAAGCTCTTTTTTCCTTCAACTCTTTGCTATTAAGAGAACTGCCCATGCTTCCCAATTTGAGGATTTTTTAAAATCTATT
TTGTATTTTAAGAATCCCTGTCACTTCGTTCCCTGCTATGTGGATTGAGTGGAAGATCCTTTGACTTTCATTCCTTTGGC
ATGAGTGTGAGCTGACTGTGCTTCTCATGCCTTGAAGGAGCCTCCCCAGTGACCAGAAGCTCCTCCTACTGGAGTCACCA
TTCTGCATGGGGTCCCCACAGTGCTTGCTAGGTTAGACTGTGACGTGATAGCTACCAATACAATCCTGAGTGTTTCTAGC
TGGGGCCCAGTAACAAGGCTCTGATGACTTCACTTACTTAAAAAAGCCCAACTTGTGTTCTCAGTTGTCAGTGAGTCCAT
AATATGGATTAAATGGCTAACCCAGCTTATGTTTTATTCTATAATATTAGGTTTAAACACAACATTTTGAAGAGGAAAAA
ATATCCATCAGTTTACCCATTTCTTTTGCGGATGGGAAGAAAGAAAAAATGTTATTCACTTGCAAGACAGAAGTCAAAAG
CCATGTGCTGCCAGTGTAGACCCCACTAGAGTAATTCCCCCTGCCTCCCATCCTGGAAGCATGCCACCAAGTTTTATGCG
ATACTTACCTAGTTCATTTTCCAGTCTTTTAAATCAAGTTTCATATTCACCCTCCCGGGTATCTCCAGGGTTCCTCTGCC
CAGGGATCCTGAGACCCATCCTCTCCCACTGTAGCAGGTTGGATATCTCTACTCTCAATACTCATCAAACAATGGTGTGA
```

```
CTCCTATATTGAATCCAGTTTTAAAAGTGACCTTTAGCATGATCTTTATGAAAACACATTTTATGTTTAATTTAAATGAA
ACATGAGGTCAATTCAACATGGAAGAGAGTATGCCTTAAATAGACCAAGCAGAACCTTGTAGTATTAAAGAGGGACCTGT
GGGCCTAGGAGCAATCTGATACCTATTGTTTCAAATCACCCCGTCAGGGTCTTTTTTGTCTAGGAAGACAAGATGCTAAA
TTATTTTTGTCTCTTGACAGATTTAGCGATGCAATTGAATCAGGGAAAATTTGAGTATAATGGATCGTGCGGGTGAGTAA
TATGATTTAAACTGTTTTCACCTAGATTGAGAACTCCATACTACAGCTCTAATGAAGCCATGTCATTTTCAATTAGGGGC
TACAGAATTTAAGCTGGGTTTGAGGGATGGAATAGAAAGAAATGAAATACCTAGAGAATATTTGCACATATACATTCTTC
ATCTACCCCATTTTACTAAGCAGGTTTTGGGTTTTTTAAATCACAAGTGAAGCAGTTCATACATATGCTACAGTTTAATC
CTCTCACTTTAAAATCAATAGTGGAGGATTTTATGTCTGGGACAGGTAATAATCCCTGCACACTTGGACATTAACTGGAG
ATGTAGGCATTAAATATGTGCATTTTACCTAGGACAAATCAAATAATGAAGTTAATTTCATATGTAATGAAGTTGTGGGT
GTCGAGCTGAATTTCATTCAGTTGGGAATAAACCTTTGTCCTCTACTGATTCTTTTTGTGTCTTGTAAGTAGCAGTTAT
CAAAGAATAATGCTGTGAATATTGTCCTAGTTTCCTTAAATAGCACTCTTGAAACTGTGAACCAAGAAGTGAAATAGGA
AATGTTAATTCTACAGAAACACTGAAAGGTTAAATGAAAATATAAAATTAGAATTCTCTTGAGATAAAAAAAAAAAAACA
CAACTATGTGGTCATCCCCTCACCCCACCTCAGCCTCATGCATCTGTCCCCAAACAAGTAAATATTAATAACTCCAGACC
ACTGCCCAAAGGCCTCTAGAGGAATCCAATGGAAACTATCCCGTCATAATGATGCTATTAGGAATTTCTTCATGGTGGC
TCACCTGATTGGATATCTTTTTATCACTGAACACTGCCAGTCAAGATAACATTTTTCAGTGGTTGTGAGTTAGGAATGAC
TTTTCAGCATGCACTCTGCCTCCCTCTGCCAGCCTGGTGAATGGTTCTCTGCCAATGTGGCTGCAAACACAGTGATTATG
CTATTCCCCAAATCACCCACCACTTACATACCTGGAGCATGCCCTACATTTTCCTCTGCTGTGTGCTTTGGAAGAAAATA
AATTGTGTAGCTGGGAAGAGAAAGATCTGAATTCATATTTCAGAATTTTGTGAAAGCAATTTTGTTATTTAATGCCAAGT
GTTCCAACCCAGGAGATTTTGCGCATAAATTGCTTTGTAGACCTCCTCCTCTCCCAAGTAGTGGGCCAAGGCATCAAGCC
CCAGGGGTTCAGCCCTGGACTGTCAGTAAAAGCCCTAGGCTGGGAGTCTAAAGATCAGGGTTTGAGCTCAGCTCCCAGCT
GCTCCTAGTTCTTGCTGTATTGCCTAGAGCAGGTGTGTTAGACCCAGAGCCTCTCTTCTGCTTGATAAAATGAAGACG
GTGACACCTGTCGCACAGGGTTTGTTGTACTCAAAGGAAAAAAATGTCTTTTTCTAGAACGAAACAGGGAACTGAAAA
AGACTTTGTCCTTTGACAAAAACCGAGTCCTTTCTGATGAGACCCACCTTTGGGCTTCCCTCTCAGTCACTGTAGAATCC
AGTCTGAGCAACAATCGTGCTAGTTCAGTTTAGTGAAATCCCCAGCCCTTGGTATCTGACCACCCTGGATATCCTGTCA
CTTTGGCCTGCCTTCAGCAATAATCCTATCAAGTCAGTTTGGCCAGAAGTCCTTATCCTTGATGTTTCCTCATAGTAATT
TTCATCTGCTGGCGCTCACCTTACTCCATGGTTCTAAATCTCCATGTACGCTTGTTGGGGTAAGGGTTGAGCCCTTATA
CCTATCACCATGGCTCCCTACCCCTTTGAATAAAGTCCACTTTACCATGTTTAACAAGTGTTTGTGTACATTTTTTTCTT
TAACAGTTTACGACAGCTCAGTCAGGTTGAATGAGTGTACGAAAGTACTTCAGGAACTGCACATTTCTGTGCAAAAGTGA
GATTTAAGAAAAGCTTAAGGCCTTGTGATCAATCACACACACAGACATTGCAAAATTCAATGAGTGCTCTGTTCAGTCGT
CCTTTAAGGACCCCAAGGAGTCAGCCTCAGCACTCCTGGCTCTTACAGGACCCGTCGGGCATGGCTGTGCTCACTTCTGA
AAACTTGCAGTGGCCCGGTGATGCTTGAAGCACTTTCCTCGCCCACCTCAGCGATCACACCTAAGATGCCATCAGCTGTC
TAACATGCCATGAGGAATCCTGGCAGCCTGGCCGGATGCAGGGAGTATTGTTTCTGTATAAAAAAAATCCTGACGGCTCG
GCCCAGTGGCTCACACCTGGAATCCCAGCACTTTGGGAGGCCGAGGCGGGCAGTGGCTCACACCTGGAATCCCAGCACTT
TGGGAGGCCGAGGCGGGCAGATCACAAGGTTAGGAGATCGAGACCATCCTGGCTAACACGGCGAAATCCCGTCTCTACTA
AAAATACAAAAAATTAGCCGGGCGTGGTGGCGGGCACCTGTAGTCCCAGCTACTTGGGAGGCTGAGGCAGGAGAATGTCG
TGAACCTGGGAGGCAGAGCTTACAGTGAGCCAAGATCGTGCCACTGCACTCCAGCCTGGGTGACAGAGCAAGACTCCATC
TCAAAAAAAAAAAAAAAAAAAGTCCTGACAATCCTATGTTGTTTGTTTTTCCTAGAATTTCTGCTTAGCCATTATTAGCA
CGTGCAGTGTCTCACTCTCTCCACCGTTATTTATTTTTTCTTAAGACAGGGTTTCGCTCTGTCACCCAGGCTCAAGTGCA
GTGGTGCAAACGTGGCTCATTGAGGCCTTGACATCCTGGGCTCAATCCATCCTCCCTCCTCCACCCCTCAAATAGCTAGG
GGTGCCCACCACCACACATGGCTAGTTTTTTTTATTTGTACAGATGGGGTTTCACCATGTTGCCCAGACTGGTCTCGAACT
CTTGAGCTCAGGCAATCTGCCTGCCTTGGCCTCCCAAAGTGCTGGGATTACAGGCATGAGCTACTATGCCTGACCTCACT
GGTCATTTGTAAATGCGTTCATTTATTTTAAAATACCTTCACAGAAAAGGATACTGTTGCTACTTTAGCCTGAATTCTGT
GCTGATGTGTTGGACGCTATGTGACTGAAAGCATACACCTCACCAGAAAAGCTGAAAAATAGCCTGTGCAGTGGGGCAGC
CCAGCTTGATAAAGTATCAGCGATGCTGTTGACTCTGTAATCTCCTAATTCCCCTTCCCATACACCCAGCTGGTGGCAAA
GGCTGTCAGTGACAGGGAAAAGAGAAGAAATTGCCGTCACCTGCATGCCTCTGTGTTCCATGGCGATGGTAGATGTGGGG
CCCGGTGCTGCACAGGAGCTCCTGTCACTGGTGCTCGGGCACTGCATGGTTCTCACCGTGTCACCCTTGTGGTTCTCCCA
CCCACCTGCAAAGACTTTTAAAAACTGCCCCTTTGGGCTGAGTCTGAGGACTGCATTTCTTAAAGAGGCATTCTGTGCCC
TTTAGGCCTGTGGAGGCAAAGAATCCCTGTTTTATATAGAACATCAGTGTGCAAAGTACACAAACACCCCCAGGATCTAG
CAACTGTAGCAAAACGTCAGATACCCTCCTGGGAAAACCCTCTGTGCTCCCTGGCTGATCTTGTGGGAGGACGCACCACT
GGGCCCAATAAGTGTGAATGTTACCAGGAAAGTGAGCAGTGCTTTTAACAGTCTCGTTTTTGAAGATGGAAAAATGGAAG
ACATTTCCCTAATTGCAAATGAAACAAAATAGTGTAAATTAATACCTAAAATCTTTTTAGACTTTCATAGGAAAGTGAGT
GATTGATATCAAAATGGTATGACATTTTATGATTCAGAGGCTGTTTTTTATACAGCATTTCTCCCTTGCCCACAGGCCGT
ACGTTCTAAGAACCCCAGTGGATGCCTGAAACCACCAAACCCAACATGTATTATATATATTGTGGTTCTACACATTGCAT
ACCTGTGATAAAGTTTAATTTATAAATTAGGCATAGTAAGAGATTAACATCAATGACTAATAATGGAACAGTTATAACAA
TATAGTGTAATAAAAGTTAGGTGAATGTGGTCTCAAAATATCTTAATTTTTTCTCTTTTATGTTTATTGGCTATTTGGAT
ATTCTGTTTTGTGAAGAACTTCTACTCATTTTCCTATTGGGTTTTCTATCTTTTAAAATAGATTTTTTATTTCAAATTTT
TATTTTCCAATTTTTATATTAGGTTCTATTTTAATATTTTTGGAACAGAGTTGACCAGGGGTAGCTGAAGCCTCAGAAAG
CAAAATTGCAAATAAAGGGGGATACTGTATAGCATAGCAACTTGCAGTTATCATCAGCAGCCATTTTGCTTTCTTGCAGC
TAAATAAAATAATGGCATGTTGGACAACTGATGGCATCTTAGGTATGATGAAATAGGATGCATTTCCTTATGACCCCATT
TTCTCATTCTTGGTGCTCCTATCCACCCACCTGGTTAGCAACCCTTCTTCTCTCCCTCGCTTGCCTTCCAAGGCCTTACA
CAGTCCACAGAAAATCCTTGGATCCCTGTCTTGGCATTGGCTTTGGCCTTGTGCCTGTGCCTACCGCACAGACCTAGATG
```

```
TCTGGAATGAGTTCATTTTCTGTACCAGAAAGCACCAAACAGACCATCTATGTCATTTGCAGGGCCCAATGCAGAATGAA
AATGAAGGGCTCCTGAGAATTTAGGATGGTGGATGCAGAGCATTAAACCAAGCATGGGGCCCTTCTGAGTTCAGGGCCCT
GTAGAACTGCACAATTAGCACATCCACATAGCCAGCTCTGGTGCCAGATTCTGGCTTCAGCTTCCCTATCAATGCCCCAG
CCCCCTGCACCCTCACAGCCACCAGGGATATGAGAGGCAACCTCTGCTGCTCTCTCAGACCGCCTTGGACCCAGAACAGA
TGCTCCTTTGACCTTCAAAGAAAGCTTGCTAAGTGTCCTATCCCAGGCCAGGGCTTATCTCATGGTCCTGACAAGCTTCT
GCATACTCTGTTTTACTCTCTTCAAGTATACAAAATGAACCCATTTCACCGGAGCATGCCTGCTTTGGAGAATTGGATAT
GGGTTTCTTGCCCCTTTCCCTGCCATTTCTCATTTTGGATCAATTCACTCCTTCAGAGATGAGTGTTCTATGCAGATTT
GAAAGGGGGGATGTGGAAGAATGCTTTTGACCCTGAGATGTCATTGTGATCCCGTTTCTTATTTTGGTGGGTAGGAAGGG
CTCTTGATCTTTAACAAAGGACTGTGGAAAGTGAGGAATCTCTGGCACTCACTTCCCAAGGAGATTCAGAACTGGAAAG
GTGACTCCAGTCCTGTATTCATCCAAGTCCCGACCTTCCTCGGGAGGCAGAATCCCTCATTTGCCACAGCTGGGGTCCAA
ATGCTGCCTGTGTCATGAAAGCCAGACCCACCATTGTTACCTCCCCTCACAGTCTGTGTCGGTTTTCCTTCCAGAGAAAG
CTGGTGGGCTTCCCTCGGAGATGCAGGCGTAGTATTGTAGCTACTCACCTACTTTCCATCCTGATTCTTTACCTTTTGCT
GTATCATCATATTTTCTAGTTGCCAACTCTAGCCACAAAAAAAGCTGCAAATCTAAAATTCCATGAATACAGAAGATAAT
CTCTTCTATATAGAGAGCTTTTATATTAATATTGTGGGTTGCATTCTCTTGGAGACAAGAGTGTTCAAAATTACCTTCAA
CATTTCAACAATTGTGTAAATGTCTATATTCTTGATAAAAAATCAGTGTTGTCTCTTAAAGATTCCTTTTTGGACTATCA
AATGTAAGGGAATATTTTTAACACTTTGTTTTTGTTCAGTCTTAGAAGATAACAATGAATGGTGACTTTTTCAAAACTA
AGAAAAGTGACATTAAACTTTAAAGAAAAAATATCATGACTCAGGATCCCTTTTGGGTTGTATTTAACAGATAATAAAG
TTGACATCATCTAGACTTGAAATTAATAAAAGTTATGCTTCTAAGAAAAATTAGAGATTTGAGTTCCCATAATTTTGATT
CTTTTACATTTAAAAGCAAACCTTTACTTTTGGAAAAAAGTACTTTTATTTTCATTGATGTACTGCTCATAGTATGGATT
CTTTAAATCTATGGTTATACTTATTTCTAATAATTTTTTAATTTTTTTAAGTTTTTTGACACAAGTTTCAGGGAAATTA
TGGAATCTGTGATTATATGACCACTACCCCGTGGACATGAGGGGCAGACATTGTGTAGTGAGGGTAATTGCAGAACATTT
TCATCGCCTCAAAATCTGTATCGTTTAGCTTTCATCTCCCAATAGGAATTTTAATCTACCCTCTGTCTCTATATTTGCCT
ATTCTGAACATTTGATATAAATGAATTTATATAACATATGGCCTTTCGTGCCTGGCTTCTTTCACTTAACATAATGTTTT
CAAGGTTCATCCAGGTTGTAATGTGTATCAATACTTTATTATTTTTATTGCTGAATAATTTTCCATTGTATGGATATACC
ATGTTTTGTGCCATACATTCATTCATCGATGGACATTTGGGTAATTTCCATCTCTGGATTATTACAAATAATGCTGCTAT
GAATGTTCATATGCAAGTTCTTCTATGAATATGTCTTTCCATTTATCTTGGTTACATGCATAAGAATGGAATTGCTGGGT
CATATGGTAACTCTATGTTTAACTCTTTGAGGAACTGCCAGACTGTCTTTCTAAGTGAATGCATCATTTTACACTCCTAC
TAGCAACATATGAGAGTTCCAATTTCTCCACATCCTTGTCAACACTTATTGTTATCTGACTCATTAATTACAGTCATCCT
AGTGTATACATAGTGATATCTCATTGTGGTTTTGATATTCATTTCTCTGATGACTAATGATGTTAGCATCTTTTTATGT
GCTTCTTGGTTTTTTGTATATCTTATTTGGAGAAATGTCTGTTCAGATCATTTGACCATTATTTAGATGAGTTATTCTTT
ATGTATTCTAGATACAGGCCTCTAATCAGATATATGAATTGCAAATTTTTTCCTTTTTGTGACTAGTCTTTTTACTTTCT
TCATAGTGTCTTTTGAAGCATAAAAGTTTTCAACAGAAATCCAGTGTATCATTGCATGTGCTTTTGGTATCATGTTTAG
AATCCATTGCCAAATTCAGGTCATAAAGATTTGTCCTTATGGCTTCTTTTAGGAATTTTGTAGTTTGGCTCTTGCATTTA
GTTATTTTATCCTCTTTGAGCTAATTTTTGTATATGGTATAAGGTAAAGATCCAGTTTTATTTATTTATCTATTTATTTG
CATGGAACTATCCTATTATCCTGTCACTATTTATTTAAAAGACTATTCTTTCACCCATTGATTGGTCTTGGCATCCTTGT
CAAAAATCAATTGGCCATAGATAACGTGGATTTATTTATAGACTTTGATTCTGTCTCAATGAGTTATGTGTTTTTCTTTA
TGTCAGTACCAGACTGTCTTGATTAAATCTTGTAGCTTAGTAATAAGTTTTGAAATCAAGAAGTGTGAGACACCCAATTT
TGTTCTCTCTTTTAACATTGTTTTGGCTATTCTTGATCCCTTGAGTTTCTATATGAATTTTAGGATTAGCTTGTCAGTTT
CTACCAAAAAGTCAGCTGGGATTTTGATAGAGATTACACTGAATTCACACATCAATTTGAGAAATACTTCCAGCTTAACA
ATATGGTCTTCCAACCCATGAAGGCAAGATGTCTTCCCCATTTATTTAGCTCTTCTTTAATTTTTTTAAACAGTGTTTTGT
AGTTTTCAAATTGTAGACACTTATTTTTTTTTAAAAAAGTTTATTCCTAAGTGGTTTATATTTTGCTGTTGTTATAGAATT
GTTTTCTTAATTTCATTTTTGCATGTTCATTGTCATTGTATAGAAATACAATTGACTTTTTATATGTTTATCTGGTATCC
TGCAATAGTGGTGAATTTATTTATTCCAATAGTTTTTTTAGTGGATTCCTTAGAGTTTTTTTTTATTTATGGGATCATGT
CATCTGTGACTAGAAATAGGCTTACTTCCTTCTTTCCAATCTGGATGCCTTTTAATTTGTTTTCTTGCCTGATTGTTTCT
ACTAGAAATGGAGAAATAATTTATTTTTATTTTACCTACCTCTAGCTGAAATGTCAATTACCTTTGATTGTGAATGTAG
ACAACAATCCACAGTCACATTAGTGGTACCCGTGACTCTCTCACCAATGTAAATCACAGACATTGTCATATTTCACTACT
ATTGTTACAGCTGTCTCAAAATATTTTTGTACTTTAGATTATTGTCATCATAATTAGATCTTACCATTAGATCTTATAAT
TTAATGCATTATTACAAGTACAATTACAGAGGGTAGAATATCACATATTTTTATATTTTGATAACTATAGCTAATATA
ATTGGTTTTTCATTGTTATTCTTTGCATTTTATTTTATACATATAAAGAAAAGTATTTAAAGGTGACCAGGGGATAAAAA
AGTTCCAGAGAAGATGATAGGATCAGGAAGTCCTGGATACCACTGAGGCTGCAGCTCTTTAAGGTTGTGCATAAGGGCGG
GCACATTCTCTTCCATCCTTAGGACCCTCTGCCTAAAATCCTAGCTCCTGTGTTACAGATGGAGAAAAGGAAGCATCCAT
TGTTTCAACTAGCGAGTGTTTTAGTCTTTGTAAAACCTACTAATAAACTTCTATGCTATTGTCCTACCAGGTACCTTCTC
AAACCAGATTTCATGAGGCGGCCTGATCGAACATTTGACCCCTTCTCTGAAACTCCTGTTGATGGTGTTATTGCAGCCAC
TTGCTCAGTGCAGGTAAGGCCCCTGCTCATCACAAGGTAGTATAAATGTATACACAAGTGGTCCTTGAAAGAAATTATAA
AATATTGAATGTTAAATTGCAAGATCCATGTGCTGCTCACAACTTTGACTTAAGATTCCACCCCCTTTTTAAAAAATGAG
ACCCATTTTTTCACTAGTAAAATTATTAGCTACTTAAAGTGAAAGATACCATCAAAAAAGTACCTGCTACTTGATTCCAT
TTATACAAAATTCTAGAAAATGCAAATTATAGTGACAGACATAAATCAGTGATGGCCTTTGAAAAAAGGGCAGGGAGGA
ATGGGTTACAAAAGGGCACAAAGAAACTTCAGGTGATGATACAAATGTTTATCATCTTAATTATGGAGGTGGTTTCGCAG
ATGTGCTCATATGTCCCAACTGATCTAATTTTACATTTTTTTTTTGTTTTTTATTTTTTGACACTGAGTATCACTCTGTC
GCCCAGGCAGGAGTGCAGTGGCACAATCTCGGCTCACTGCAACCTCCTCCTCCCGGTTTCAAGTGATTCTCCTGCACCTG
TAGCTGGGATTACAGGTGCCCACCGCCACACCTGGCTAATTTTTATATTTTTAGTAGAGATGGGTTTTTACCATGTTGGC
```

```
CAGGCTGTTCTCAAACTCCTAACCTCAGGTGATCTACCTGCCTCAGCCTCCCAAAGTACTGGCATTACAGGGATGAGCCA
CCGTGCCTAGCCTAATTGTACATTTTAAATGCATATAATTTATTGTATGTCAAATATACCTCAAAAAAGCTGTTTCAGAT
TGTTAGCTAGAAAATAATGTAAACAAAAAACCCTAATAAGTGGCTATAGTCATTTAAACACTAAATGAAAAATCATCTCT
CCATAGCTTTATCGTAGATAGACTAACATTATAAAACTGTCCTAGCTGACCAGTGTCAATATCAGTAATGACAGACAAAT
TCTAGCTTCACTTTTCTGTGTGTCTCTTTTCAAGACTTCTAAAAGAATTCATTAATAAGTATGGCATGATGGAATTTCAG
AGCTATGAAGGGTCTTAGGACATACCTGAATTCATCACTTCATTTCATAGGTGTAGACACTTAGGTGAGATGAAGAGGCC
GTGGCACTATTTGTATTATGATTCATATATATTTTTTCTTTGTTTTTTTTCTAGACAGTTCTAGAAGGTAGAACATCTG
CTTGAAACAGCTGGGGTTTTTTTGCCTATAGTAGTTGTTCGATAATGTTTTGGTTTGATAGAAATGAATACATTATTGCC
ATAGATAATTGAAATTCCTGCTCCCACCCAAAAGACAGAATTTCTTACTTCCTGATTATTTTTGCTACTGATGCTTACA
CAGAGCTTACTTCTCTTTGCTCTCGTTCGTGCTGCTACAGGTTATATCAGGTCAATTCTTATCAGATAAGAAAATTGGCA
CCTACGTAGAGGTGGATATGTATGGGTTGCCCACTGACACCATACGTAAGGAATTCCGAACTCGCATGGTTATGAATAAT
GGACTCAATCCAGTTTACAATGAAGAGTCATTTGTATTTCGGAAGGTAGGACATTTTCAGCACGTCAAACTTACTCTAAT
AACTTGGGAGCCATGTTTTAGTTCACAGACGCTACACGATACAGGGGCACTTATTCAACCGACAACATCTTCTTTTTCTC
TTCTCCTAATGGCTAACTTCTGTAGCTGTCATAGGATCATTGGTTCTATTTGGCCCTGCCCTCCTTTTGGTTCTCCTCTC
CTCCTTGGAAGAGCCCATCTCCTGCACAGCAGTGCCTCAGCTCTGGGTTTACCATCTTTTAGAATGAGTCCTTGCTGGTG
GACACCATCGTACACTAGAGAGTTCTTCCCTACCCTGGAAGTTACTGGTAACTTACACTATTCCTTTAGCCAAGTAGTCA
GGTAAGATGCTGCACTCCAAAATTTTGGTCAATAGATGTCACTACATGGGTCAGTTGGCATGGTCCTGTCTCTCCTGAAA
GAGTGACACCTGTTGCCTACTTGCAGGTCTTATCCTTCTTGTACTGTTGTTAGTGTTTTATTTTTTTCTCCTAATACCGT
ATGTTGGATCCTAAAAACAATCACAGCCATTCTCTCTAACGTTCGGTTGTTAATCTCTTATTCACCTTTTCCCTTGGTTT
ATTTTTTGTCTATTGAAGATGCAGTGGAAAATCCGCACACCGGCAGTGCTGTCTCCAAGAATGTTCACTTTCCTTTCTTA
CTTACTCTGTTGTCCTGACCACAATGATTTCTTGCTGCTTCTTTAGGCACAAGTTTTCCAGCTGGTTCTGCTCCATTTGT
ACATCATGCAACACAGATAATCAGCTTCTTTCCTTAACAAAATAGGGATTATCCATTTCAGAATAGAGTAACTGGCTTAC
AGAAACCAAATGAAGGGAGATCAATGTAGGTTTGATGAAACAGGCCATTTTGACATTTAAGTTTACGCTGGGATCCACAC
AATCTTCGCATCCCATTGTCTTCTTCTCTGCCCCTGTCAGGATATGGTGTAAATTACATGCATAGCTGTGATTTGGTTTT
TTCCTTCCCTGGTATTTCCAATTTGTTATAGCTGGATCCGTTACCAGTATGGCTAGTGTTCTTTGGCATAGTGAATAATC
CCTACTCACATTTCTAGAAGCTGAGAAAGTTGACTCAGGCATTCTTTGTTTTGGATGAATGAGAAAATACCCAGCTTAGT
GTTTATATATAATAGTTCTGGCACTAGAAACGAGATGCCAATTCTGAGTCCTTCTCTGTTCTTCAGAAGTTAAATCGTTT
TTTTCATTGCTAGCAAGTGCTTTAAAAACTTTGTTTTCTTTCTTGTCATTTTTGTATTTTTCACTAATCTCACATGGCAA
GAATGGCAAATCCCAAGCCTCTATTCACTGCATAAGGAGCAAGAGAGAGGGAAATTGGCTAAGTTACCATGGTTTCTCCT
CTTCATCTTCATTAAGCTGGAATTAAATTATAGCCTTCAGATGTAAATAGGCAACCAGTCCATTGTCTAGCTTTAAACAT
AGCACTCAGATCAAGCAAGATGTCCTGGTTGCTTTTAGCCAATAAAAAGGCATTCCTGATTAAACACATTGGATACCCTT
TTTGTAATGTTGCTATTTTGTAACTGAATCAGGTTTCTGCTATCTGAACTCAGATTTTATGTTCATTTATTCCGTTTGGG
GATAGCAGGAGTAGGTGTCTTAGAATCAAAGTATGTTAATGTTTCTAGCTACCGCATCTTACTCTATTTAACATCCCAAC
ATTTGCTGGAAACTGAGGAGTACAAGAAACAAGGAGGACCCAAATTTATGTACAAAAGTTATCTCTTATGCATGAAGCAT
TTATTCAGAGATACTTAATTTATTTATTCAGAGGATACTTTATTTATTCAGAGATACTTTCGTATTCTTAAAAACAAACAA
ATATAGATGTCAGCAATATGCCAGATATTAGATCACAGGATTAGATCCCCAATTCCCAGGCCCCACTCTGTCACTGATAT
GTCTAAGATCAAGGAAAGTGGCTTTATTTCTCATCCATTATATGGGTGGGGACAAGGATCAATCACAAGGGTTGAAGTGG
TAATCTTTAACATTCATTTCATCTAACATTTTTCACGAGATAATTTCCAGGAAACATGTTCCAATTGCTTTTCTCAGTGT
CTTGGGCACTGTAGCTCATGCAGTACATTTTGGGGAATGCAGATGGATCATGGACTTACTTTCAACATCACGGGAACATTT
AAGAACAGCATGGATTTGGTCCACACTCCTTAGAGTCATGGTTCTTGGGCGCTGCATTGGAAAAATCAGTGAAAGTCCTG
TTCTGTTTGCAGGTGATCCTGCCGGACCTGGCTGTCTTGAGAATAGCTGTGTATGATGATAACAACAACCTGATTGGCCA
GAGGATCCTCCCGCTTGATGGCCTCCAAGCCGGATATCGACACATTTCCCTTCGAAATGAGGGAAATAAACCATTATCAC
TACCAACAATTTTCTGCAATATTGTTCTTAAAACATATGTGCCTGATGGATTTGGAGGTAAGATAAACATTTGGGTACGG
AATATGATATAGATGAGGTCCTAGATTATAAGATTATAAAACATCTTGTCCATGCTTTAAAAAACAATAAAAATACTGTC
TATTCCTGCTAATGCTGCCAACCCTGATTACCTTGAATACATCAAGAATACATCATAACCTCAGGTTTCATAGTAAGAAA
TGAAAGTACAAAACGAGATGATCTCTAAAGTCCTTTCTTATTTTAAAATACTATAATTTTATGTTGTTTATCAATTTTTA
CAAACACATTTTGTGAAGGGTATATTTATATTGACAATCAGACTAGGGAAAAAAACATAAATAAGGTGAAACCATAGATA
TCTTATCAATAGTATCGCTAAAAGATTTAAATTGAACTCAGCTATCAACATGCCTGAATTTTCTTCCAAATCCACAAGAA
AGTTCAATTTCATAAAAAATCCTAGTCATCAATCCATATGCTTTAAGGTAATCAATGGGAGGAATCCAAAGCTAGATTAA
GTCATAGAATTTTGCTTACATGCAAACATGTTAACTTTTTATGGCCTCCATCTAAGCCAATACATTAAAAGAAAGCAAAG
ATCCTCTATATTGTCAGCCTGTTGAACCATAAGGAAATAGTACAGCAGGATGATGGCATCCGATTAGCCAAGGGTTCAAA
TCCTTACTCTCCACTGCTGGCAGGGTGATCTTGGGAAGAGGATGTAACCTCTCAAACCCCTCATTTCCTCCCCTGCAAAG
TGAAGATAATAATAACTACCTCCCTCATAGCATTATTGTGAGGTGCTTATCATTAGAGTACATGATATAGCAAGGCTCAG
AAATGTATGAGCGCATGGGGGTGGGCGGGGTGTCTGTAGACACAGATATAATTTCTATACTTCACTGGAACACATCACTA
TTTTATCTGGGGAATATGGGATAATTTTGCTGTTTGTTTTTAGTAGTGCATGCTTCTGTGGAGGGCAGATTTTCTGCAAC
TAAACCACAACTTTACCAGTCTAATATCAGAGTCAAAAATTAAAATAAGCCATGAGGATTTTTCCATCCCAAAATTCATA
GTCCAGGCTCCTTCCTCCTTCTCCTTTGTCTTTCATAAAAAAAAAAAAGGAAGCACATTAGAAGCTACAACATACAGTAA
TAGAGGTAACAACCCATAAAAAGCCTGGAAAGGGATGCTGTGAGGCTGGGTAAGAGGATCAGAAAGGGGCACTGGAGACA
AGTGGTAGGTTTAGGACAGCAGTCCGTGGCAAACCTCAGAAACCTCCCTGCAACACAGCATGGTGGTGGCCATGGGGCA
TCAGCTGATGAGTTAAATCATACTCCACATACGTGAGCTTAGAAAGCAGGAACTGTGGAATACCTCATACATTTTAGAAA
TAGTTGTATAAACCCATATGATGGAAGATTTAGAAGTTTTGCCTCTGCATGGGTTAACTTCCCACTGAAACTCTAATTCA
```

293

```
TTCTTGGGAACTAAGCCACTCGAATAGAAAAGGGAAAGAAATCATTTGTTTAGCTTCTACTTTATGCCAAGAGCTTTATG
TAAAGAGATCCAAAAGCATGAAGTTGTTCAGAATGGATGTAAGCATAGGGAGATCTGTGCTGCAGAAACATCTGTATTTA
CAATGTAGCTTATTACCCATGTTGTATTTAAGTATTTAGCCAAATTAATCTCTTTAATATTAGTCCTGCCAGTATTCTTT
CCTAACATGTCATTGTGGCTGCTTACTTTTTATCAGAAGCGTTTGAAATTTCTATGTACCATTGATGAGATTCTGCAGTC
GATGCCTTAACCAGAGTTGACATTCAAATACAATTTATTCAAAATTCTCTGGTCATTTATTTTATATGTGAAGAAACCAG
GGACAGACATTATGCGGTGTCAGGGACTTCCCTGTAGATAACAGAAGAAGAATATGAGAACTGTCTTTTTCCAAATCTCT
GATCTTCTTTATAGCTTCTTGACTTATTCAAAATATACTTATATTTTCCCTGTTTAGCCAACCTCCCACATTCATATCTC
AGTGTGTTTATGTGCTGAACCAGTGAGATAGGAAATCTATAGAGTCTGTGTTCCCTGGAGTATTGATATTCCCTCTTCAT
TCCACAGGAACAAGTTCAAGATGAAGCTCAGCACCATCACACTCAGCATTTTCTAGAAAAAAAAATGTTTTTTTTCCCCT
AGGAAAAAAAGAAACTCATCAATATACATCATTAGATTTATATTTAGCTAAAGAAAGATAGTGTGGATTTTCATTACTT
TTTTCCACAAATATGCATAGCAGGTTTATTCTATAAAAAGTTAAGTAGCTAAAATATGTATCAGAGAGCAATTCTGAGAA
CACAGAGGATCAAATGAGGGTCACTCACAAATCTGCTGCCCAGAGACAGCTATTGTTTACTTCCAGTCTCTTTTTTTCCT
GTTTGTTTATTTATTTATTTGAGACGGAGTTTTGCTCTTGTCACCCAGGCTGGAGTGCAGTGACACAATCTCGGCTCACT
GCAACCTCCATCTCCCATGTTCAGGCGATTCTCCTGTCTCAGCCTCCCGATAGCTAGAACTACAGGCGTGCACCACTATG
CCCAGCTAATTTTGTGTTTTTAGTAGAGACGGGGTTTCACCATGTTGGCCAGGCTGATCTTGAACTTCTGACCTCAGGTG
ATCCACTTGCCTTGGCCTCCCAAAGTGCTGGGATTACAGACGTGAGCCACTGTGCCTGGCCTTTTCTGTTTTATTTTTAA
TAAAATTAAATCTTACTATACTGCTAATTTGGAATTCTAATTTTTTTTTAGAATATGTTTCCATGTCATGAAATATTTTT
ATATTTTTATTAGCTGTATAGGATTACATTTTATGTCGGCCATAATTTAGTTATGGATAAACACTGAAGTTTTCTTCCTT
GGCCGCTGAGGAAACAGAGTTAGGAATTTGAGTGCAACTCCTTCTGCTTCTCCTTCTCATTTCTATAGCATAAAAACTCT
CAAAACTGGCCAGGCGCGGTGGCTCATGCCTGTAATCCCAGCACTTTGGGAGGCCGAGGCGCGTGGATCACATGAGGCCA
AGAATTCAAGACCAGCCTGGCCAACATGGCAAAACACTGTCTCTACTAAAAATGCAAAATTAGCCTGCCATGGTAGCAC
ACGCCTGTAATCCCAGCTACTCTGGAGGCTGAGGCACAAGAATTGATTGTACCTGGGAGGCAGAGGTTGAAGTGAGCCGA
GTTCATGCCACCGCACTCCAGCCTGGTTGACAGAGCAAGACTCTGTCTCCAAAAACAACAACAACAACAAAATAACTCCC
AAAAGACACAAAGAAATAACAGAGCAGTGATGGCTACCATGAGTTCTGAACCCTTCCTGGCTGTTTCTAAGCAGCCCAGA
TGCAAAATGTCCAACAATACAGTCTCCTATCAGAAAAAAAAAAAAAGTAACATTTAGGACAGATGATTAGGAATGAATCA
TGAAAACTTGGCTTAATTTTTTCAATATTATTTTGCTACAAACGTAAAGAAAAACAACCCTGTCTTTTACTCCCTCTGT
CATACACAAATAGAAGTACTAGATTGGGTGCTAGGAGCATTCTTCCTTTGTCAAATCCAATGAGACTCCGAGTGCTGGGA
TTATCTTTGCTGCTTTATCTGTGGGTGCCAACGCCACCGTAGATGGCATTTGCCCATTGCAAGGCTTTATCAGTGTTAGA
CACTGCTGTAGGAGACAGGAAGCAGTTCCAGGGCTAATGGCAATGAAACGAGGGTCAGTATTTCTTTTTCACCCACTGTT
GCTTTGGGTGGGTTTTATCACTTGTGTTTATTTTATTATTACTGAGTGATAGGGAGGGGAGCAAATAAGAGAGTTGATGC
TTTGCTTCCGAAATCTATGGAGCCTCAGCATTGTATTGAAATGTCACTCCCCGCTGTGTTTCATGAGCCTCCCAGCGCTT
TGTTCCTCGGGCAGTGCTGATCATTACTTCTGCAACCATAAATTTTGCCTCCTCTTCTACAACTATTTTATGTCAGGGAA
GATGAAGTGACCCTCTATTTCTTTGTACATTTCCCCTTTCCACCTTCCCTATGATTAAAAATACATCTTGGGTCTCTGCC
CAGGGTTTTCATAGGGTTGCTTTCCAACCATTTTTACAAAGATAGTTGACTGTTTCTTATATCAGATATTTCCAGAGAAA
GAATGTTCTTAATTAAGATGCCATTCCTGGGAAATAATCTCAGGACTTCCTGATGCAGTTTAAACTTGTTCCATCTGTCC
TCTTCCTTAAGGGTGCTAAAATGTCATATTTTATTAAATATTTGTTGAAAAACCCAGTTGCATATTCACAGTGATATCTT
TCTTGTGGCCTTTTCTGTACTACAGAAAATATTAGTCAGACTTCAAGAAGAAAAGCTCTGGAGGGGAAGGGGGCAGGATG
GATTATGGTAAAATACTTTGGAAATTGCTAGACTAAGCAAATTTAAACAGGCTTATCTATTGCATGGCTCCTGGGATGTA
TGGTGAATGCCCAAGGGGAGGGGGAGAACATGAGCATTTCCCAATCTTATTTGACACAGGATCTTGTTGCCCAAGCACCT
ACTGTATTTTCTACCATTCTAGGAGTAAGCAAATTTAAACATGAGCATTTGAGAAATGCTTAATCTTTTGGTAGGAACCCAAAGGACTAAGACAAA
ATCATAGACTTGGGATTTGCCTTTCTTTATCAGCACCACATTGCAGCTGATAGGGGAAAAGGATTTTTCTTCTGCCTGTA
CCTCTTGGTAAGTTAGGAATAGTAGAGGGGAACGGAGGGAACCTGGGCCAGGAGTGAGCACATAGGGGTGGGGAGGGGG
AGTGTAGGTGCCATGGGCAGTGCTCACCACTGAGGCACTCAATGATTTGAAAAGCATTGGTTAAACAAACGAAAATCAAC
TGAGTTCTCCTGAAAAGCCAATAAGCATCTGGAAGTCTGTACTAAGGAATTGCTATCTTCATTTCCATTCTTAGAGACAC
CTACATTGATGCTTTCAACTCTATATGAAAGTCAGGAAGCATGGCAGGGAGGAAGGGAGGGGAAAAGCAGGGCCTACATG
GAGAGTCTTTACTAGGAGAGACCAGCTTCATGGAAATGACATTATGCCCAGAAATAGATGGTGTTTTTAAAAATACACAT
AAGTAGAGCTTTAAGTTAAATCTTAATGAAAGAACCAAATCAGAAAAAGCAGACTGGTTTCTTTTAAAAAGAGAGGTTCC
GGAGCCCCTGCTGTGGCCTCCAAGAATCTAGGGTGTTTTAGGCCACCTACATGGGTTCTGCAGAGTCAGGAGTTTGCATG
CATCCATCTCTACCCTGTTAGATGTACAATGTGGCTCCCCCCAGCAAGGCCTACTCACCTGCCAGTGGATGCCGATCAGC
ACTGGAAATGAATTCTTGCCACAGTGATTTATCCTGTGTGGAACCCTAAACTAAATGGGAAATAGGCAGCCACAGTATTCCC
CAATAAAAGGAAGGAAGCCCAGTGAAATGTCATATTGGCACCAAAGAAAGATACTGGGTTTACTTGAATATGCAAAACTT
TGGCCAAGAATATACCAGATTTATTTAACCACTTCCTCAAGTTATTTTTCTGGGCTGAGGCAAGGAAAAAATAAATACCA
TTTTTCAAGTACAGTTACACAAGTAGGTAAGTATGAGATATTTGAGGTAGAGCTTTTCATTCTCTGATTACAGATTATTT
TAAATGTGGCTATTAAAAATCAATTTCCTTTTTTGATATGCAGCACTCTTCGTATCTACCTATCCATATGTCTCACATAT
ATATGCCTCTGGCCAGTGGAGAAACTGAATTACAATGAAAAATTATCTTAAAGATGCCAAGGCAGGCCTTGGCTCCCTAA
TGCTGAGCCCTTTGCACTTTGGCAGAGAATTTTTGTCAGAACTGTCCCAGGTATTCAAATGCCATTCTTCTGCATTGTTC
TATAACTCCAGCTACAGAATGAACACCTGCCACCACATGACCCAATTAGACATCCTAGAAAGGGGTATCCAATCTTTTGG
CTTCCTTGGGCCACATTGGAAGAATAAGAATTGTCTTGAAAATAAAAATAAAAATAAAAAAAGTTTGTGCATAATTCTCATGTTTTAGG
CTATAAAAATAAATAAATAAATAAATAAATTTAAAAAATAAAAATAAAAAAAGTTTGTGCATAATTCTCATGTTTTAGG
AAAGTTTACAAATTCGTGTTGGGCCACATTCAAAGCCATCCTGGGCCACATGTGGCCTGTGGGCCATGGGTTGGGCATGC
TTATCCTAGAAGTATCTAGTTTGTTATATGAAATTGCTAGGAATGAAGCATCCATTTCAAATAGCCTGCTAACTTTAGCC
```

```
TTGCTCAAGGCATTTAGTCCTTTTGTAATCTCTTTTTTTGTCTTATCTGATTTAATTTGAACAAACCTTACTTTACACTTT
TCATATTTTTGTTGCATTTTGTTCAAGAACATTATATTGCATTATTATTGTATATTGTAATTTCTTAAAGGTAAAGGCAG
CCTCTAACTGCAAATAAAACATCTCAAATAAGACGTTTCAAAAAATAAGACCAAATAAGGCAGCATGTTAGGATCTGGCA
TCATATGTCCCTAATAGTGTCTAGATTTGTTAATATTGGTAATTCAAGATAGAGTTAAGTAATGATATTTATCCAGCTAA
TGGATAAATCTTCAGCAGGCTCCGATTTTGTTGTATCATTTAGGTGACATCTACATAATCCGATAGCAGATGGCTGATGA
GAGCATCTCAGATAGCTTGCCTGTGACAAACAAGACAATATGTGGATTCAGCTAGGTCAGGATTGTGTAGAATCAGCCCT
AGAAGCTGAGACCAAGACATTGCTGCATAAACAGGAGGAAAACCACTGGTATTCTAGCAGGAGGTCTCCAGGGGCTGCTG
GACCCTCCATAGTCTTAATGATAAAGCCTGCCTTGTACACTTAAGCACATGACTTTTTATGGAAGATGCATACCCAAGAA
TTTAAGTAGTGAATTTTTTTCCCCTCAATTACTGTTGGCAATCGAATGTACAGTTGTCTTGTTCTCCCTCTGCCCTAGTT
TCCTAGAACTACTGTAACAAATAACCACAGACTGGGTGGTTTAAAACAGCAGAGATTTATGGAGGCTAGAAGCCTGAGAC
TAAGTGGTGGGCAGGGCCATGCCCTCTCTGAAGGCTCTCGGGAAGAATCCTTCCTTGCCTCTTTCTGGCTTCTGGTGGCT
TCAGACAGTCCTTGGCATTCCTTGGCTTGAAGATGCACCACTCCAGTCTCTGCCTCTGTTGTCACATGGCCTTCTTCCCT
GTGACTCCATGTTCTCTCCCTTCTTATAGCGACACTAGTGATTGGATTTAGGGCCCACCCTGATCTAGTAGGATCTCATT
TCAGTCCTCTTCTAAAACCTAAGCAAAGACCCTGTTTATAAATAACATCACGTTCTGAAGTTCTAGGTAGACAGGAGTTT
TGGTGGACACTATTCAACCTACTCCACCCTCTTAATTCTGCTTTTAATTTTTATTTTTTATTCTTTATGAGACAGAGTCT
TGCTCTGTCGCCCAGGCTGCAGTGCAGTGGCATGATCTCAGCTTACTGCAACCTCCACTTGCCAGGTTCAAGCAATTCTC
CTGCCTCAGCCTCCCGAGTAGCTGGGATTACAGGTGTGCACCACCACACGGGGCCAATTTGTGTGTGTGTGTGTTTGTGT
GTGTGTGTGTGTGTGTGTGTGTGTGTATTTTTAGTAAAGATAGGATTTCACCATGTTGACCAGGCTGATCTTAAATTC
TTGACCTCAAATGATCCACCCACCTCAGCCTCCCAAAGTACTGGGATTACAGGCATGAGCCACCGTGACCGGCCTCCGCT
TTATTTTCTTAGTACTCCTTCCATTCATCATTCTTCAATAACTATTTTAAAGCTTATTAAGAATAAATTTTGTTAGGAAT
GCCTATCAGGCATATGAGTTATATAAAGTGCTATATTATTTAACTAAACCATTAACTTTCTGATAAAATGACAAAGAAAT
CATTTTTTGCACTAAGATATTCCTAAATTCATCTTTTTATAGAAAAGTTGAAAGCCCATCTAAGTATCTACCAGTGGGGA
ATAGTTTGATATAGAAAATCAATGTGACATTTCCCATTTACTAAAATAATTTTCAGAACTATGCCGATATAATATATGC
ATCAATGAAAATGCAGGTTATATAACTTCTAATAAACCATGGAAAATATGTATTCATGTGCAAAAATGACACAAAAATAT
ACAAAAATGCTTGGTTAGGATAGATGGATCATTTAATATCTTGCTTTATTGATTCTTTAATGTCTTGCTTTATTGATTCT
GTAACTTGTTTTAAGTAATTAAAGTATTATCTGTTATTCCTATGGAAAAGACTACCAGTTACTCCTCAAAAATGCCATAT
ACTCTCTTCAGGCCCCCAGACTGATTATATGTTATTAAAAATTAGCGAGTATTTGTTTCCTAACCACCCAGTCCTGTGTG
TTATCACTGAAATGTGCCATTGGGATTGGATGCTTGGCAAAACATATCTGAAGTGAGAATGTACAGAGAAGCATAGACCA
AGTATATCTCCTCCAAAGAAGGGCAGAAAAGTTTAATGGATACAAGTTAAACCTCACTCTGCCTCATAATGGTGTGGACA
AGTGGCAAGAAGAGGCATTTGGGAGTCAGGCACAAAGAGTTGTGCTAAAAGTCTAGGTTGGTCAAATTGTGTCTTCATGT
GCTGAAGGACCTTAATCATGCCCACTTCATGGCATCCTGATGACGATGAGAAAGCCATGTGGTTCTCCTAACACAGCATC
ACCTCATGGTTGACCAGTGACCACAGGGCAGAAGGATGCTGAGTAGAAAGACATGAATTGGCATCAGAGGAGTCTTCTGA
ACATTGCATTTCCTCTCTAAGCAATGGAGGTAGACCCTGTGTCTCCTCCAGACACTACTGACTGAAGAGAGATGTCAGCA .
CCATCAGCACCACTAAAAGTAATGTTGGGAGCCAAAGTATTGCCATGAGAATCACCGAATTGTTGTGAAGAGAGATAGCA
GCAGTATGAATGCAGAGATCGAGTTGCCACCTCCTGCAAGCACCTCTAAAAGTAATGTTGGGGAGCCAAAGTATTGCCATG
AGAATCACCGAATTGCTGTGAAGAGAGATAGCAGCAGTATGCAGAGATGGGAGTTGCCACCTCCTGCAAGCTGCCAC
CTCCTGAGCCTACTTGAGGAACTTAGCGTCACAAATGGGACAACCAGCCTTTATGTCCCTCAATGTGATGCATATAAAGT
CACAGCACCTCCTAGAAGTATCCTTAAAGAAAGATGTCAAGAGGGATTGCTTTAGACTAAAATTGACTTAAGAGACATAA
TTATCAGAGACATAGTGTGAAATCTGGTACCTTGATTCAAGCAATGTAACTATAAATAGGGGAAATATTAAGATGGGCTG
TGTATTATATCATAGTAAAGAATTACGCCAATTTTGTTGGATATAGCATTGGGATGGTTGGCTCCAAATTAAATTTAGAG
CTGCAGAGACAAGTTATTAGGAATTAAATGTTCTGATATATGGAATTCACCTTAAAATATTTCAATAGAGAGGAAAGAGA
AAAAATGAAAGGAATATAAAGGAAATGTGGCAAACAAATGTTTATAATTGTTGGGTTATCATCATAAATATATGAGGGGT
TATTGTACTGTTCTCTCCACTTTTGTGTATACTTGAAATGTTTTGTAACAAAGAGTTTAAAATATATAAATAAATTCCTA
GAATATTAACTTGCCAAAAATAATTGTCTGTGATTTTTTTTTCCTGAGCAAGATTTAAGAACTAATTTTTCCACAGCTGT
TGTTGAGAGGCAAAGAGTTCTTAGGGATCATGGGTGATAAAAACAGACCTTCTCACATGCCACTGGTGGGAGAATAAATT
GATGCAATTTCGGTGGAGTGCAATTTGGCAATAACTACCATAGGACAAATACTCTTACCCATCATTCTAACTTCTAAGAA
TCTATTCTGTAAATATATGTAGAGGTATGCTACGAAATGTTCAAAGACATTCACTACAATACTTTAATAGCAAAGAACTG
GAAGGACAAGCTGTCTACCAATAGGAACTGGTTAAAGAAATTATCACCATTCAGAGAATGGAAAGTTTAAGCAACCCTTA
TAATTAATGTATTAGAGCTATATTGCCAATATGAGAATGTTTCCCAAGATATTTGACATTAAAAAATGGTGCAGAAAACT
ATGAACCCATTTGGAGTTTTAAAAAATATCAGAAAGGATACAAGAAATGGATAATAATAGTTACCTTCTGGGCGTAGGAT
TGGCAGGAGAGAAAGTTTTCACTTTTCATTTTATATATTTGAATGTTTACCATGTGCAGATGTTATTTTATTTTATTTGT
TTATTATTATTTTTTTCTGAGACGGAGTCTCACTCTGTCACCCAGCCTGGAGTACATTGGCACGATCTCTGCTCACTGCA
ACCTCCGCCTCCTGGGTTCAAGTGACTCTCCTGCCTCAGCTTCTCAAGTAGCTGAGATTACAGGCATGCGCCACCAAGCC
CAGCTAATTTTTGTATTTTTAGTAAAGACGGGGTTTCACCATGTTGCCTAGGCTGGTCTTGAACTCGTGACCTCAGGTGA
TCCACCCTTCTCGTGTTCCCAAAGTACTGGGATTACCGGCGTGAGCTACTGCGTCCGGCCAACCATGTGCAAATGTTATT
TTCTGTTATAAAAAGGAAAGAAAAAAAAATTGAAACCTAGAGAATTATGTGCCCCGTGTACTGAGAATGTTATCTTAAG
AATTATCCTAAGATTGTAATTAGGAAGGCGATTCTGTTAGGGAAAACTTTATAAACTTACACAGTATTTAAAATGTATAA
ATTATGGATGGTTTCAAGTTAACAAGAATAATTTTGCCTTCTATAGTATGAGTAAATTGTGCACGATTATATGAAACTCT
GAAAGTACTTGGAAATCTTCAAATGGCATTAATATAAAATATTAAATTCTACTATCTTTTAAGTTCAAGTTATACCTGAG
AAAACACTAGAGAAAGATTTCATTTTGTCTACCATGGAACCAAAATTGGCTTTGAATATGTATTATCCTTCTGTATCGAA
AAATAAGACCTACCTGCCTCAGGTACTTTTTGTGTTCATCATCTCATTGAATTATCATGATTATTCTGCAGGGCAGGTGG
```

```
TATCCACCCTAATTTCCAGATAAAGAAACTGAGGCTGGGGACATTAATCAACTTGCTGAAAGTCACACACAAGTGAAGAA
ACAGTCTAGAACCCACACACTTCACTCAGTTTGTCCTGACACTTTGCCTCCTTCATATCAGGAAAAACTTTGTATCAGGA
AAAACTAAATAAAAAAATCATCTGGTCTTTGGCCCTTGCTTTTAACTCCTTTCTTGTTTCTGCAACTAGCTGTGCCTTC
CTCAGTGTGTCTTATGTCCTAGGAAAAGATTATTAACAGAAAGCACAACAGGTATCCAGGATTGTAGTTTATTAAGCAGA
GTTTTAATGAATATTTTCAGTAAAACAGAGAATTAACGGCTCATTGGGTTTTTTTTTCCCCTAGATATCGTGGATGCTTT
ATCAGATCCAAAGAAATTTCTCTCAATTACAGAAAAGAGAGCAGACCAAATGAGAGCTATGGGCATTGAAACTGTAAGTA
GAAATGGTTGATTAAAGGTGGCCAAGTTGTGGGAGTTTGATTATCAAACAGTGTTTACAAAAACAAGACAAAGTAATGAA
TTAGCAAATTTAAGGAGGGTTTTTAAAACTGAGCTCATCAATTCAACAATAGTTAACTTTCTACAAAAGTGTGGCATGTT
TAATAAAATGTGACTGCCATCAGAAAAATCCACTTCATCATCAGTGGCTTAACTCACATCTGGGGACCCCTTCCCCATTA
GAAAGAAAATGCAAACATTTGTTATACTTTTGGGAAAGTTTAAAATGTAGGTGGAGCATTTCTAATCAGAAAAATGCAA
CATCCGAAAAGTTCCAAAATTCAAAACCTTTTGAGTGCTGACATTGGATAGTCATACCTTTGCTTTTTGAAGTTTCACTG
TACATAAACTTTCTTTCATGAACAAAATTACTAAAAATATTGTATAAAATTACCTTCAGGCTGTGTATAAGTTGTATATA
AAACATAAATGAATTTTCTGTTTAGACTTGGGTCCCATCCCCAGGATACCTCATTATGTATATGTAAATGTTCCAAAATC
TGATATCCAAAACATTTCTGGTCTCAAGCATTTCAGATAAGGGATATTCAAACTGTACTGTACAGGATGTTTCTATGATA
CACTGAAATACCAGTTAACCTAGTTCAGTGAATTAATGATAATTGCTTTTTATTTTTAATTATTTATTTTTTAACAGATA
GGATCTCGCTCTGTTTCCCAGGCTGGAGTGCAGTAGTGTGATCATAGCTGACTATAAACTTAAACTTCTGGGCTCAAGCC
ATCCTTCCACCTCAGCCTCCTCAGCAGTTGGGACTACAGATGCATGCCACTGTGCCTGGCTAATTTTTTACTTTTTTTG
TCAAGACAGGGTCTCACTCTGTTGCCTAGGTTGGTGTCAAACTCTTGGCCTCAAGCAATCCTCCCACCTTGACCTGCCAA
AGCATTGAAATTACTGGCGTGAGCCACCATGCCCAGCCAAAATGGCGTTTATTATTGATTTTCACTGTTTGCCAGCCTT
ACATCCCAACCTTCTCCCATTCCAGTGCACACGTGGATTGTTTCTTTGTTTTTAAATGTTCTCTTTACCAGACTCCTCAG
TTTGGAATGGATGTAGCATCTCTCCAATAGCTAGAAAAAGAGAAAGTATTCTCTGCTTGTTAAGAAGTTGGATCTTCTTT
CCTTGGCTGGATCCTTTGCCTTTAAGAGAGTAGAAGTCTAGGAAGAGTATGGTAGAAGTTTACTGGATTCAGTAAAATAA
AGAATGTACAAGATATTTGACCTTGAGTGACATTCATTTGGGTCAATGTAAATGTTTCTGTTCCAGAGTGACATAGCCGA
CGTGCCCAGTGACACTTCCAAAAATGACAAGAAAGGAAAGGCCAACACCGCCAAAGCAAATGTGACCCCTCAGAGTAGCT
CTGAGCTCAGACCAACCACCACGGCTGCCCTGGCCTCTGGTGTGGAAGCCAAGAAAGGTGAGAGAGAGCCGTTGGGTTCC
TTATCTTCTGCACCCACCTTAATTACACAGTGTACAATATCTATAGCTTTAGGAAATATATAAATTCGAAGTTCTTTGTG
GGAAAGAACCTTTTACATAGTGATGTTAACTAATTCATCCCCACTCTCTTCCAGACCCTCCCATAGGTAGTTACCTGGAC
AGGCCGTGGGCTCATGAACCAACATAGTTATTCCTATTGGACATTTCTAAATTCTCATACTGATAATTTTCCCTCATAAG
TAGAAACATGATTATTTGTTTGTAAAATTATCAAATGAGCACTTGAAGGCCAATTCCATGTGCTTACCTGTAGATGAAAC
AAAAGATCATTAAGAGGTGGCATCACAAAGAATCACGTGTCTCATATTCAGCCAGTATGTAGGGAAACAACTGTGCTGGT
TATTTTAATTGGTTTCCATATGAATTGGCCAGACATCCATTGATTGGATGGTGCCCATTCCATACTGGAGGGAAAGTGGG
CAGGGAGAATGCTGATAGGGAGTGGCAAGGAGTTCATTTTTCTTGTTCTGGAAGAATTCTGGGGTCATCCTGAGAAGGT
CATGGGAAATGAGTCAAAGGAGTATTTGAAGAAAATTATAGTCTTGGCAGAAGAATTCATTGAAAGTGGGCATGCTAAGG
GCAAGGTAAGAATTATGATACTCATGCTTCTGTTATAACACACCTATCACATTGTGTTAATAAAGTATTAACAACCAGAG
AAAGAGGTTATTGCAGATGAGACCACACCTCATCATCAGTGTGTCCCTGGAACCATGCCTAGTTAAAATCTAGTAGTCTA
TTTTCTCAGGAAGTAAAGCGCTGTTTAATGAATGATAGGGGCAAGGAAATCTAGATTAAGTTGGAAATTGTCAAAAGGAA
GGAAGAATAGAAATAAGTTGGCAGCTTGAGGCCAGGTGCAGTGGCTCATGCCTGTAATCCCAGCACTTTGAGAGGCCGAA
GCGGGCAGATCATGAGGTCAGGAGATCGAGACCATCCTGGCTAACATGTTGAAACCCCGTCTCTACTAAAAATACAAAAA
ATTAGCCAGGCATGGTGGCGAGCCCCTGTAGTCCCAGCTGCTCAGGAGGCTGAGGCAGGAGAATGGCGTGAGCCCGGGAG
GCGGAGCTTGCAGTGAGCTGAGATAGCGCCACTGCGCCACTGCCGGCCTGGGTGAAAGAGGGGAGACTATATCTTAAAAAAAAAA
AAAAAAAAGAAAGAAAGAAATAAGTTGGCAGCTTTGATATGGGCCTTAGGCAGAAAGGGGCTTTTTGGTAACTCAGACTT
CAAGGACAGATCCCAGAGATAGATTGTACCATTGAGAGAAGTAGAGGAGTTAGGAGGAGGAGCTGCTTTTGGTAAGGAAG
ATGATTTTGGTTCTGTATATCACTGAGGACAAGCTGTTGCTGATACATCTGGGCAGAAATATGCAATTACCAAATCTATA
TCAGGTGGAGAAGAAGAAGGAGGGACAGAGAAACAGAATAAGAATACTTAGAAGGCAGAATGAATGGGGAAAAAAGAGAA
AAGAGAGATCTATGTTCTAAGAAGAATTAGTTTTGAGAAGATGCTGTAGACAAGTCCAGAGAAGTGAAGACTACAAAATT
ATAGTTGAACCAGGGGTGATTGGATGATGAAGGTAGAAACATGGTTAAAAAAATGTGAGACATAATTTAAGACATAAAAT
GGTTGTTCTGGAGGACAGTAGCATGTTGGTAGGAACTAATAAAGAAAAACAAGGAAAATGCAAAACAGAGTACATGGTAA
AACAAGGCTATAAGGAAGATCTGAATCAACAGAAAGGGTGAAATTCAGCTGGATAAGGAAGGAAGAGATATGTTCTGTCT
GAGCTAAGAAGAATGATGAGTGAATAGAAGTTTCAGGATAGACAGGAAGAATTCTTGACAGACGGTGTATCAGTCAGCTT
ATGCTGCATAACAAATGACCTCAAATTTTAGTAGCTTAAGACAACCACCATTTATTGAGATCATGTTTCATTGGGTTGGT
TGGTCTAAGCTGGCTTGGCTAATCTCTGTGTCTTGCTTATGCACCTGTGATCAGCTGATAGGTTGACTGGAAGGTAGATG
TGGGATGACCTTATGCACACATCTGTCATTTGGCAAGTTGTTTCACATTATCCTGAAGCCTGAGCAGGCTTGTTCATATG
GTACTCCCTGAGTTTAAAGAGCAGCCCTTTTCCAGCCTCTGCTAGTCTCCCATTGGCTACAGCAGGTCACAAAGTGAACC
AATATTCAAGGGGTAGAGAAATAGACTCTACCTCTTGATTAGAGGAGAAAGAAATAGTGGTCATTTTTGTAGTATAACAC
AGATAGATTTTATCTGATCTAAGAAGTTGGTTAGGTGGCTGCTCTCTGCTGAGAAACGTAGAGGTAGAACTGAAAAACAG
CAGTATAGAAAAGCTTTGGAGTTGCCCCTGGCTGGAATAGAATGATCCACAGGAAATAAGGAGAAGGTCTGCCACATAGC
ACACAGGACCCTGGTGAGGGTTGGTGGTGTGTACTAAGTTGATGTAGCTTGATCTCATTACTTCTTCAGCAGTGCCCAGA
AACTCAGCAACAACTGTGGACAAAGTACAGCTTAGGGAGTTGCAAGCCTGAAATTATCACAATTTATATGGTCTGGGACC
AAAAGTTTGAAGACCATTAAGTTATGACACTGTAACAGTTGGCTATTGATGTGTAACAAGCAATCACAAAAGTCAGTAAT
TTAAATAAGTAAGCATCTATTATAGCTCATGAGGTTGTCTGGGTGGCTTTTCTGGTTATGGGAGGATTAGTATCAGTGAC
TCAGCTGATCTTAGCTATGCTTATTCAAATGTTTGGGGTTCAGCTAAAGTGTCATTGGCTGGGACAAGTGGGCTGTCTTC
```

```
CTTGTGGACTCTCATCTTTCATCAGCTTAGCCTGGGCTTGTTCACATGGTAGTTGCAGTGTTCTAAAATGGAGGTGAAAG
TACAGATGCTCCTTGACTTATGACAGAGTTACATCCTCATAAGCTGAACATATCATTAAGTCAAAAATGTGTTTAATATC
TAAGCTACCGAACATCATAGCTTAGCTTAGTCTAGCCTATTTTAAATGTGCTCAGAACACCTACATTAGTCTACAGTTGG
GCAAAATCATCTAACACAAAGCCTATTTTATAATAAAGTGTTAAATATTTCGTGTAACTTATTAAATGCAGTACAGTGTA
GAGTATACAGCTGATTGGGAGCTGAGGCTTGCTGCAGCCCCAGCGCATATCCCTAGCTTAGGAAAAGATCAAAATTCACA
ATTCAACATATGCTCTCTACTGAATGCATATCACTTTTGCTTCATCACAAAGTTGAACCACTGTAAGTTGAACCATCTGT
ATGTCAGGTTCCTTGATGAGTCCTAGACTTAGAACTGGCATGGCATCCTTTCCACTACATTCTGTCATCCCAAGCAAGTC
ACATGGCCCAGCTCAGATTCAAGAGTTGGGGAAACAGATTCCACATATTGATGGGAGAAATTCCAAAGTCACATGTAAGT
AGGTGTGGAAACAGGGAGGGGTGGAATTTTTTGGCCATTTTTGCACTCAGTCTGCCACCCTGATCATAATATATAGGTTA
ATGTTCCAGTGGGGTAGATACTAGAGGAGAGAGACTTTTCTAGGAAAGGAAAAGGTTAAAGGGAACCAGGAACAAGATGA
AAGTAAAAAGCTTGCTTGGTAGGTGTGAGGGAGTGAGTGAAGTTGGCAGGATGGAGATTGCAGTCAGAAAGAGGTTACTC
TAAGCTACTTGTCTTGGAGGAAGGGCAGTTTCAAGCAGTGTTAACATCCCGTTTACAACTGTCAGTGGGAGGTGAGGTAA
AGTAGAGATGAAGATTTCTAGAATGGAAGGCATCATGAAAACAGAAAGAAAAGTCACTCAGCACATTCTTCTCATGCATA
TTGAATCACTGTGTTGGACGTCTGGGAATGCCTGCATAGGAAAACCACAATTCAGATATGAAGTTCATGGGGCCGGGCAG
TGTGTGACCTAAAAGGACACATGTGGAGGTAGTTAGGCTCAGCAGAGCCTGGTGTAAGTGAGAGGTCTGTTCTTTTGTAT
GATAGTTTTTAGGAGAATGGCATGGAAGTACAAGAGAGAAGTCTATAGGTGTTCTTCCCACGCCTCTCCATGGATTGGGG
TGGGGTGGGGAATAAACTCTACTCCAGGAGCCTTTGGGGGAAGTGATGTCATTTGAGAAAGCAAGCCTCCATTTCTTTCC
AGAAAACTGTCTTAATTTGAGTCACCCCAAAGACCCTGAGAGAAGGCTTCCAGTGCAAGTAGTTTATTCTGGGAGGCAGT
GGACATGGATAGAGATGTGAAGAAGTGACACCAGGGAGGGAAGGCAGCCAGTAATTAGAATGTTTAAAGCCAGTTGCCA
GTAGGCACCTGGAACTTAATGCCCTGGAGAAACTCTGGGGGCTGGTATTGGACATACTTCTCAGAGGTCTCCTCTTAGAG
GGATGAGGGAGCTGGGGAATGTATACCCTGACTCCTGTCAGCCTTTTCTTGAGGGTTCCCGTGGAAGGGATAAATTCTCG
GCATTCAGACGCTGGCAATTGGAAGTGAACTAGAAGAGCTGATGTAGAGAGGGCATAGTGTGTGGCTGGAGCAGACGGCA
TTTGCTGCAGACCTCTATGAAGGTGGGGAGGTGTGAAATTCCAAACTGTTTTTCTTAGACATTCAGCCAAACAGCAACAA
CTGCACCAACACGACTTTTTTTTTTTTCTGATTTTAAGAACAAAGGATTAAGAACACCCAGCTCCTAACTTCCAGATTA
GCAATTAAGAGAAAACAAGTTATTGTTAAGGAACTATGCTGATATGAACAAAAACTTTATGTAGTGAAACTTCAATTAGC
TAAAACCCAAAATGTGCTGCCTAACCTACTTTCATACCAGGGAAGCCTGTAGACCTGCACAGTCCTATATAGTAGCCACT
AACCACATGGGGCTACCACTAACCACATAGGGCTATTTGAATTTATTTAACAATTAACTTAAAAGTTAAACCTTGAGTTC
CTCATTCACACTAGCCACATTTCAGGTGCTCAAGAGCCACAGATGGCTAGAGGCCGTCAGTTGGACAGACCCACGCATGG
AACATTCCCATTATTGTGCAAAGCTCTATGAGGAAGGCTGTTCTTGGCACGCAGATGGAACTGTCCTCTTCTGTAGAGTG
TGGTTTTTTTTTTTCTAACTAAATCACTTATGGGAATCAACATTTTTACAAATTGTGTCTTTCCCCCAAAACACAAAACC
AGGTAGTTGAGGGAAGTTTATGTTTTTGTAGGTTCCTTTTAATCAAGACTTAGTTGAACCAAGACAAAAAGAAAATAAAA
ATTGAGAGAGAGAGTGTAAGTTTCATCAGACTGGGTCAAACTCAGAATTTGCTAGATCTCTGGAGGACCTTGCATACTCC
CCAAATCCTGTATGTTCATTTTACAGATGAGAGAGCTGAGGCCTAAAGACAGGGAGCTCTTTGTGTGCCAGGAGTCACTC
CCTTGCCTTACCCTGCCTCAGTCACTAGAGAGACAGCTTGTGAAAGCCAACTTAGGAGATCAGTAGAGAGCCCCATGGAC
CCTTTATACCCCGATCATAAAATGTTACAGATCACAAGGCTGATTTCCCTCAGCCAAAACTTTCCTTCTGCGGCCTGACC
ACCCTATCATTGAATGTGCACATCTTAAAATGAATAAAAGGGAAAAACAAGAGCCTGGGACTTGATGCCTATCTTTCTTG
AATATATCATACCTAGCCTTTGGGATTCTAATGGAAATCAATTACAGCACAGAGTACACATTCATTTTGTTTGCCTAAGA
TGAAAATCTGCAGTCAAACAGAAACTGTTTGAGGATCTGGCTACAACTGTTGTTGCCATGACAACACCTGGCCTAGCATC
ACTCAGATAGCAGGATGACTTAGTGTATTACCCTAGCTCCTCCTGGTATGCTCCTGTTATACACTGGTTTGATTTTATTA
TTTCCTGTATGTTTAAGGCATTGCTGTGCTCAATAGCACATTGCTTAAATGCTAAGATTGAATTTGGTAAAAGGGAGAAG
CCAGAAAAAAATGTTTTATTCTTTCACAATGACTGAATTGAGAATAGAGTTGGGAATTAGTTGACTGGCATGATATGCCT
TGAAATACAAAGGTAAGAATAAGGATAATAGATACTTTTGTGCCTGATCAGCTCCCCTTTATGGGTTCACTATCCCATCT
CACAGCTCCTATGAATTTTGGCTGATTGGGCATTTCAGCTGCCCTTCTCCATAGAATTGCCCTGGGACCATGAGAGTTCC
CTTGCCTGAAAGGTTTTGTGAAACCTCTGTAGCACTCAGCTGATTGATCCAGGGTTACAAATGCCCAGCCTCTTTTTGCC
AAGGTGGAATGACTCTTGGGTGCCATTTGTACTGAAGAGCTCCCTGCCAGGATCAGACTGAGGCTGGATTTCTACTGGAAA
CACACCCTACCTTGTTCCTCTGCCCTGCTCCACTTCTTTCATTCCCTTAGGGGTTTCTCCTGAGAGTCTGCCCTCAATCA
ATCACTTGCACATGAATCCCCTTCTCAGGCTGCTTGTAGGAAACCTGACCTAAGTTAAGACATTCAACATTTCAATTG
TCCAGTTAATTGCTTTGTAATAGGAACTTAAGATGTTTATATTCATCATATGTAAAGCTGCTTCAAAGTCTTTCGATGAG
ATTTCGATATGTCAGTTCTATATTACCAGTTATTCTCTCTGATTTTTAGTATACATAGTGACTTAATTTTTTTTGTTTGT
TTAGGTATTGAACTTATCCCTCAAGTAAGGATAGAAGACTTAAAGCAGATGAAGGTAAAATTGCTTGACTATTTTGCAAG
CACTCTTGTATTACACATATTGGTGACACCAATATATTTTTGTTTCTCACCAAGTGTAATCATTGTGATTACAAAAAGAA
AAAACAAAAACCTATTTTTGATTCTATTTTTCTCCCAAAGGCTTACTTGAAGCATTTAAAGAAACAGCAGAAGGAGCTA
AATTCTTTAAAGAAGAAACATGCAAAGGTACAGTGCTCTACAGCTACTATTTTGTGTTATGTATGGATGAATCATTTGTA
GCCAAAAACACTACTTTGAAGCTGATACCAGACCACTTAACAGTAATAACTCATTCTAACTGGTTAACAATGAAATTAAA
AAATAGAATCGGTTTGGGAGTGCTTGTCACTTGTTTGTAGTCTGCATTTCTAACAATTCTTTCAAATGCATAACAAAGCT
TCATCCATTTAAAAATTGTTTCACTAGGCCGGGCACAGTGGCTCACGCCTGTAATCCCAGCACTTTGGGAGGCCAAGGTG
GGTAGAATAGGATGTCAGGAGTTTGAGACCAGCCTGACCAACATGGTAAAACCTGTCTCTACTAAAAATACAAAAATTAG
CTGGGTGTGGTGACATACCCCTGTAATCCCAGCTACTCAGGAGGCTGAGGCAGGAGAATCGCTTTAACCCGGGAGGCAGA
GGTTGCAGTGAGCTGAGATCGTGCCATTGCATTCCAGTGTAGGTGATAGAGTAAGACTCCATCTCAAAAAAAAGAAAAA
TTATTTCACTAATAACATGACTATATAAAATATTGAAGACCAGCTCACAATTCTAGCCACTAGTTACCTAAATTGCCAGG
TCACTGTCACATAGTTTGCCATACACACTGGTGCATGGAGGGAGATGCTAGCAATCTGTCAACCTCCCACTCACAGCACC
```

```
TAGAGAGAGAACACTTGTCACTTTTTCTCCCTTATGTAAATCTTGCCCAGTAGCCTACTTCTGAAATAGATTTATTTTTG
CTTTTTACCCATAAGAAGTGGTATTTTGACAGTGATTGTGAAAAATTACAAGAGCTAGTTGCAAATTGTAAGAGAACAGC
AGGAGGTGGCCCCAGGAATTCTCCTGTGGGTTGAAAAACGTCATGGCCCTCAAACATCGTCACTTCCAGACTCCCTCACA
GGTAAAGAAGAAAAAGGTAGTACCTCTTTCCTTCCTAAGAACATATAATAACCAGCTGCTGCTGGTAGGATAATGTCTGT
CCTCTTTTATTAAGGGCAATGAGAGCAAGAGAAACTTAATTTATAATTTATTAATTTGAAAGAACTTCTGAGTTCTTTTA
AACTAATCTTGCATTCCTGTTTCTTTATTCTCTGACCCAAGCCTCAAACCAAGGCATGTCCTTCAGAACTTGGCTTGAGT
GATGTACCTTTTAATCATTCCACGTATTGAGATGCCGAGAACATTGTCTGGACCCTGTCCCTGTCATTTCTTTAAGAG
TTTGTGCTAACACTGATTTTTTGAGAAGTCATTTCTTTAAGAGTTTATCCTAACACTGATTTTTTTAGAAGTTAGGCCT
AATCTTATCCTAGTAATGTTCCCTGTATTACAGAACTGTTGGGCTCAATGGATTTGCTGATACAGTCACTTCACTTCAAA
GACTGTTTCTAAGTTTCATGAATAATTCCATTCAGTGAAATTCCTTACATTGCTCAATGGGGTTATTGGAGACAGAACAA
TTTAAAGTAGGGGCTTCAATGTTGTTGGTTATTGACAACAGGCTGAACTATGGAAAGCCAACAGAAAAGAGTAAGAATG
AGAAATAAGAGAGAAAGCACCTAAATGACTTTAATTATTTGGAATAGAGTGGATGCCCGTATGCTGCAGCACTATCTAA
CAGATTTTTCTTGCATTTAAATTGGATCTCTTTTATTGAGCAGACACCTCAGTGGCCTTTGGGCTAGTTGGAAACATAAA
GATTTTGGCTTCATTTAATATTTTCTTGAGGCATGGCCAGAAAATATGCTGTGGCGGGTGGTAATAATAGCAGCAATAAT
AGCAGCTAGAATTCCTGCATGCTCAGTAATTCTCAGATGCTGTGCTCAGCACCTTATATGCATTGTCTCATTTAAGACTC
TACCCTGTAAGGTAAATTCTATGTGAGTCTCCATTTATATGTGAGGGAATTAGGAATTAGTGAAGTTATCTGATGTCCCA
GAGTTAGGAAGCAGCATAGCTGATATTTGAACCAAAATCAAGAGTCCAGAATCCATAGTCTTGCACCATACTCCTGTGTA
GGATATGGGATGCTCACTAAGTGGACAAGTTCCCTCATTGGGTCAAATGCAGTGACAGCACATGGGACATTAGGGAAAAT
CACTATCAACTTTGGGCAGTGTTGATATACTGCTGATTATCAGGACTGTGACTAAGGAAGGATGCCCCTCAATTTTTAGA
ACTTAACCTACATCAGAGTTGTCCATTAACTCCCTGGAGCCATCATGCCCGGCCCAAGCTAACTTCTAAGATGAAGAATA
TAATGAATATTTACTTGCCTAATATACACCGATGCCAGCTCTGTTAACATATTGTCTTTAGGCGAGACATGGTGGCTCAT
GCATGTAATCCCAGCACTTTGGGAGGCCAAGGCAAGCAGATCTCACCTGAAGTCAGGAGTTTAAGACCAGCCTGGCCAAC
ATGGCAAAACCCTGTCTCTAGTAAAAATACAAAAGTTAGCCAGGCATGGTGGCAGGTGCCTGTAATCCCAGCTACTTGGG
AGGCTGAGGCAGGAGAATCACTTGAACCCAGGAGGCGGAGGTTGCAGTGAGCTGAGATTGTGCCACTGCACTCCAGCCTG
GGTGACAAGAGCAAGACTCTGTCTCAAAAACAAAACAGAACAAACAACAACAACAACAAAAACATGTTGTCTTTAAA
ACCAAAAAATGAAAAACTGTTTAAGGAGAGGGGAACAATGTGGTGAATCTATTACCATTGGACATACTTCATGCCTGATC
TCCATATGACGTGTTTATCCTAACACTGATTTTTCTGTCAGTCAACACTTTTAAAGTGGCAATACTAGCTTGTACATATT
GATACTTGCTTAAGCACATATTCTTAGGTATTCTCATAACAGTTATATAAAGGAGTACTATAATTATATGAGTTTTGTAT
CTGTTAGCTTTTGCAGGGTAACAAACCACCCTAAAATGTAGTGACTTAAAACAATGACCATTTATTTGGCTCACAATTTT
GTGCATCAGTAGTTGGAGCTGGGTTTAGCTGGATGGTTCTTCTGGTCTCAGCTGTATTCACTCATGAATCTGCTGTTACT
ACCAGTTTGGTTGGCAGATGGGGGACTGGCTGATTTAGGTCAGCCTCTGTTAAACCACTTCCTCTCTGCTTTATGTGGTC
TCCTGTCTTCCAGCAGGCTAGTCCAGGCTTGTTCACATGGCTGGGGTCCTGAGAGACCAAGCAGATCAAGCATAGTTTCT
TGTGGCCCCAGCTCAGAACTAACACACCATCACTTCCACCTCATTTTTTGGCCAAAGTAGGTCCTAAGTCTGGCCTTATG
AGGTCTCTACCTTTTGACAGGTGACAAAGTTACATCAGAAGTGGGAGTGGATGCAGAAAGGGAAATAACTGTGGCCATTT
TTATAGCCTACCGGCTATTCAACAGATTAGCTTATTAATACTTAAAAAAAACACAGCTTCATTAAATCATCTAGTTTTTAA
GTGGTGGAGTCAGGATTTGAACCAAATTCAGCCCGGCTTGAGAACTCAGACTTGTAACCACTATACTCAGCTGCCTCTGA
TGTTTCTCACACTAATTTTTCTTCTCTTCACACATCTTTTTCTTTATGGTCTCACTAACAGGTTTTTAAGAGCAATCATG
AACCTATCAGGGCACAAATATCTTGGGTTCCTTCAGGACTGGTTTTTCAACATAAGGTCATTGGGCCAAGAGCTGGAGGC
TTTACAAAAATATGTATTCTTATGCCTATCTGACACTTAGCTATCATCTCTAAGAAAAGAATATAGGAAATACAGAAATA
CATTTTAGATAAACTCCTCTCCTCTGCATGCTAAATTTTGAGGACTGTTTTTTTAGGAACATTATGTAGCACTTTTTACT
ATCTTTTGAGCCCTTAGTAGAAACCCCACTAATCGCCTGCAAAGACACTTATCAAGCCTCCTTAAAGAAACAGAGGTTTT
ATCCTCTTGCCACATAATAGTAAGAATTAATTCCTACATCCTTTCTGTGAAAGGACATCTACAGCTCTTTTATAATAGTT
TTCAGCCACCCAACATGGAGCTAAGGCTTACCACCCCAAAGCCTTATGTCATAAAATGACCTGCTGATGAAGAAATATGA
CCTGGGCTGCACTCTCCCTACTAGTGTGCCCCTGGCCCAAACAGCCCTTGGTAGCTAAGAACAGGGACTATTATTTAGTC
TATATCCAGCAACTGTAGGGAGATCTCAGCTCTTACATATCAGCCTAGATCAAGCTTGTCCAACCTGCATGCAGCTCCCA
GGCCACATGCAGCTTAGGATGGCTTTGAATGCAGCCCAACACAAATTTGTAGACTTTCTTAAAACATGAGATTTATGCAC
AGACTTTTTTTTTTTTTCTCTCATTGGCTATCATTAGTGTCAGCGTTTTATGTGTGGCCCAAGACAATTCTTTTTCCA
ATGTGGCCCAAGGAAGCCAAAAGATTTGCCACTGCTGGCCTAGATAAATAATTCTACTTTTTAGACTTAAAGAGAAGCAG
AGAATCTATGAACTATATCTCAGCAGTTTAAGAAATAAATGTATGGTCCCAAGGAGAGCTTATTTCCAAACTTGCAGGTT
AGCTACCAGATAGAAAGGAACAGTGGGGTGAGTGTGAGGTTCTGCCATTTGCAAATAGGTGTGCAATTTGCTGTTAGGAT
ATTGAGAAGAAAGATGGGGGTAACGACCATTTGCACCCAGATGGATATAAAAATGCTCATTAAAATGTCTATTTTTGG
AAAATGGCAATGATCAACTGCATTTGACCCTCTGAATGTACTGGCAATCAAACTTCTACTTGTCTCATACTTTCTAGAAC
CACTTCAATTCGAAATGGAAACTAGGGTAATGGTTTTTCAAGTGTCTATTATATGAGACACAGAAAAAGGAGAATTAATT
AACAATAATCCTCCCCCAGCACATGACTCATTGGGATCCAGAGCTGCAGTTACTTTCATTAAGAGACAGGCATGCTTTTC
AATTGTCCAATGAGGTCAATTCCTTAGAGCCAAAAATGTAAAACCCAAAGTTGGATACATATCTCCAGTAACGTGTTAGA
AGGGGAATTACAATTCAAATGCCTATGGGGTCCAAGCAGGAAGTAGCTGAGGGAAGTTGGACAGGTGTTGACTCCATAAA
AGCTGGGAGCACTCATCTCTATCCACAGGCCAAGTCAGTCTGAGCTACCACCATTGGTGCCTGGAGAGTAGATGGGTCAG
TCGTGTCCTCTTAAAGGAGAGTCAAAAAATCAGAAAGCTTATCTGAATTTCCTGATCTTTTACTATCGGCGTCTAATTCA
ACCTCTTTTTGAAACTGCGTAGGAAAAAACAAAACAAAATCCAAAACTAAAAACTACATCTGCAGGCAGGATACATTCCT
TAGGCCACCAGTTTGCAGTATCAACATTAAATGAGTCAAATGAAATGCCTGAGAAGCCTACTAAACTCTGGCATTTTTAG
TAAAGCTACTGATGGAAAATTTTCTCTTTGCTATGCAAGCAATTTAGCCGACAGCACCAAGACTGTTGTTAAACTCATCA
```

```
ATTTTAATTCATGGTCAGACCTTAGTACCAGACCTATAACTTAGTTTTGCAGTGGAAAGGGGCAGAGACACAGCAGTTGC
CCTCAGAGTCCGCCTATTGGTAATCTCAGAGTTACATGCTGAGCAAACATGATTTACTCCAATTTTTCAGCACCCTCTGT
GGTGCGATCATTCACGCCCATAGTCACATCAGTCACAAGAATGAATTGCAGCAGCAGTTTTCCCCAAAAGAAGCTGAATT
TACTCTTAACTTGGATAAGAAACGTACACAAATTCTTCTGAATGCTATACATGTTTTTCCCCAAAATGTCCCAGTTAAGA
TTTACTTTAAGTTAACACATTTAATTTTGGTACTCTTGGCTTGAATGAATGAGAAATGTATGTCTCTTCAATAGTGTTTC
ATGATTTAACTAAGGACCAAAAGAATAAAACCAATAATGGACATTTTTTTTAAGCTAAGATGGACTTAGAGATCATTTCA
TGCCTCCTTCATTTTACAGCACAATGAGCTGAGACTTGACCAGAGTCACATGGAAATCAGTAATTAGGATCCCCATTTCT
TTCTGAAAGGACAAGTGGTGTCTGGGAATCAAGTTATCCCAAAACACTTCAGCAATCACTGCCTACCACACAGGGATGGA
GTTTTGAATAAAAAGAGAAACAGAGACAATTTTTCAGGACCAGCTTTCCTGTTACTGACATTAATTATAGTCACTTTAAT
TCAATATGCTTTCAGTTTTTCAAATTATTTTTGCATATGTTTAGTAACCTTGCATCTCATAAGCAACTCATGAATTCGGT
TAGGCAAAGGTACCTAACTCAGTTTTCTTTTCTTTTCTTTTCTTTTTTTTTTTTTTTTTTTGAGATGGAGTCTTGCTCT
GTCATGCAGTGGCATGATCTCGACTCACTGCAACCTATGCCTCCCGGGTTCAAGCAATTCTCGTCCCTCAGCCTCCAGAG
TAGCTGGGATTACAGGAGTGCACCACCATGCCCGGCTAATTTTTGTATTTTTAGTAGAGTTGGGTTTTTGCCATGTTAGC
CAGGCTAGTCTCGAACTCCTGACCTCAGGTGATTTATGCGCCTCAGCCTCCCAAAGTCCTGGGATTACAGGCATGGGCTA
CTGTGCCCAGCCCTAACTCAGTTTTCTAATAAAGACATTGAGGCCTGGAAGATTTATATATGTTACACAAGGGCAGTTG
TTTAATTATTGGCTGAGCTGGGACCAGAATCTAAGTCCTCTTGCTATTTTCCTATGCTGACTTTTTATTCATGCGATAAA
TACTTATTGAGCATATACTTTGTACCAGGCTTTGTGCCATGTACTGGTAAAGAGAAAAGACCTGCCCTGCTCTTGTAGAG
CATATTACATAGAGTTGAATCCATCATAAACAACTTTGTGGTACAAAGAGCCCTATTCCTCATATCTAATTGGATTAATA
AGGTTCACCAAAATATTTTCTTAGGAAAAAAATCTGTGTCAATTTACATCCAAGCCTCAAAGTCTCTACTTGTCCTGTTC
TGCACTAAAATATTTAAAGAAATAGCCTTCACCCTCCTTTCAATGATATTTGCAAGATAATATAGGACTGTGGCACTTCT
GATATATTTTGAGACCTGCAGAAAGCCCCTTATTTATTTAATTTTTTGCCCAGGAAAAATTAACACATGGGTTAGCCAAC
CATAATGATTTCTAAATCTTAGAAAGAAGACTAGGAGGAGGGCATTTTAGTAGGTACCAAAGTATTACAAGAGTAGTTAA
ATACATTCAGGGATACATCAACATGTTCCTCTATTCCTGGGGCAGTAGATCAAGCATGGAGGAAACATTTATTGATTTAA
TTAGTCTGATTGATATCCCTATCTCAAGTCACTCTCTGTGTGTTATCATGGGTATTGAATAGTATAGATAGGTTTGTATG
TCACTGTGTTTTGAATAAGCTGTAACACACATTTGATACATTAGATTCACTTTTAATCCTAACAACCCTATGAAGTAAAT
GTCACTATCCCAATTATAAAGATGAGCATACTCTGGAAAGGTAAGTGACTCACCCCAGCACTGAAAAACCCATGTCCTTA
ACCCAGTGCCACATTGCAGAAGCTCTGGAGGAAATGTGTTTATAGCACAAAGGTGCACACATTGGTAATATGGCTAGA
TTTATGGGGCATTCCAATTACAAATTAAGCCCAGATCCACAGCAAGCCCACCTAGCCTTTGTTCCCACTGTAAGTTCCTA
TGTTGGGAATCTCCCTATCCAGCATCACCCTTCTCCACTCCTTCTCCTAAAGACAAGAATGCTGAGGCCTTGTGCATGAA
TGCGTTTCTAAAGAATTTATATATAAATGAATTGTAGATCAAGTCCTGTGTTAAATCTCTTAGCTGGGAAATTAAGGAAG
TCTTGGTAAAGACTTAAAAATTCCTTCCCCATTTATATTTTATGAAACCGTTAAAAGAAGTTCATGAATTAGATTTGCAT
AAAGTCAAGCCTACCTTAGAAAGAAAATAAATTTGCTTTTAAATTAAGGCACTATTTTCTTCAATAAGTGAAAGGAAATA
TGACGGTGAGAGGGGTTATATAACCTGAACAACATGTACACCCATTCCAGTCATGAATAGCATCCCATTTTTCTGGTTCT
CAAGAGTAAGGTTAGGCTATAACATCAAGTGTCATTTAGAGTTGGAAATTTCTGATTGTACTGATTTCCTCTGTGCTTGG
GAGGCTGAGCAGCTAAAGAAATCTGATTTCATATAAGTCATTGGCTGTAGCTAAATGCCTTTTTCATTGTCCAGGAAGAA
ATAATGTTTATATTGAAATACCTACTTTCCAAAGCATTGCAGCCCCAATTTCAATGGTTACGTGAAGCAGATGGGAAAAT
GTTTTAAAATATCTGTAAAACAGAAGGCCATAGCTCCTGAACACTTTATCCTCATAAACTGCTTCTAGATAAGTAAGCCAG
TGAAAATATTAAGTGATGTAGATTCAAATTCAGCTGATGGTGACAAACACAGTGATGCCTCTCATTATATAATAATGGCA
TTCAAGAGCTCCAAAATGTGCAGGTGTTTAGAAACCATGTCACATGATGAATAGTTACATTTAGCATATACAAGAGAAGA
AACATGGTGGCTGAACTTGCATGTTTGAGGACCAATGTGTGTGGAAGGGGCAGTAGGCTAATTACATGTCTCAAACAGCAGA
ACAAAGATGAATGGGTGGAAATACTACAGAGATGTGTATATGTAGAAGAAAGGACTTCGAGATGATTGGCACTACCCACT
GCTGAGCAGGAGTGCATTTGAACTGGGCATGTTCACTAGACTGAGGCTAGAGACTCCCATGTTGGGAATGCCCTTGGACA
AAAAAACCGGTGTTGAATGGGAGATCTGGAGTCTGTACTGTTCTCAGATTTGGTTATTCCTTGACTTTCTAAATGAAGAC
TCTAGGGAAGCTGGTGAAAGTTATGCCAGCCCTATATGGTGTGAGCCATGCTAAGAGTCCAATTCATAACTGCAAATCCT
TCTTGTTCAGGAACACAGTACCATGCAGAAGTTACACTGCACGCAAGTTGACAAAATTGTGGCACAGTATGACAAAGAGA
AGTCGACTCATGAGAAAATCCTAGAGAAGGCAATGAAGAAGAAGGGGTAATACTGTTTATTTCCTTCTGAAGACTTTCTC
TATGTTTATATTTTGTCTTTTCCCACAGTTTTGCTGCTGTTTAGGTCAAGGAAGAACTTAGTGTTGACTTGATTTTCATC
ATTAAAAATTATTCCTGGGAAGTAAGAAACTCATTCATTTTAAATGCTCTAACATCATAACAGGTCAATGAGTGGATGTA
GCTTCTGGTTTGGGGAAGGTTCTCAGCAGAGATTCTGATATCTTTTAATTCATCATAATTATTTCTAATATGAGAAAGAA
ATCTTAGCCTCAAATAATTGAAAAAAATACCTCTTTACTCTGAGAATTGATTGTCCTTTTTGATTTTGAACTAATTTTAAA
TTTACAGAAAAGTTTCCAAAATACTACAGAGTTCCCTTATGCCCTTAACTCACTTTCCCTAAATGTTAACATCTCACATA
ATCATAGTACAGTTGTCAAACCAGGAAATTGACAGTGGTGCAATACCATTAACCAAGCCACAGTGATTTTTCACATTTTA
CCAGTTTTTCCACAAGTGCTTGTTTTCCACTCTGGGATCACCTCCAAAATTAAATAGCATTTAGTTGTCATCTCAGTATG
TGATAATTCCTCAATCTCTCCCTGTCTTTTGTGACTTTCACTTTTGAAGAGAGCTGGTCAGTTGTTTTGTAGAATGGCTC
TTCTTTTGGATTTGTCTGATATTTTCTCATGATGAGAGTGAGGTTATGCATTTGTGGCAAGCACACCATGGAAACAATGC
TGCTTCCTTCTCAGTCCACGATATTGGGGGGGATGGGGGTGTGACATTGATATGTCCCATTATTGGTGATGTTAACCTGGA
TGACTTGGTTAAGCTGGTATCTTACAGGTTTCTGCACTGTCAGATTACTTTTTTCCCTTTATAAATACTTTGGGGAAAAT
TCCATGAGACCACGCATACATTCTATTTATCTGAGAAATTATTTTTCCTGCCTTTGACCTTCTCCAGGGATTCCCTCTGG
TTTTTATCTCTTTCCTTCAATCTCTGCTGATGTCTTCTAGTTTTCCACAACCTTTGGCACAAGAGAAAAGGAGAATGAGA
TTTCTTTTTCCTACTTGGGTTTTTTGGCATTTGGTTTTTGTTTTTTATTTTTTGTTTTAACAGTCACCCAAGATATACCT
AGAAAACTAAACTCTGAAAACAGAACAGATTATGTTAGTTATAATTTTCTTGGACATTGGTTCATATTGCCGAAATTTCA
```

```
GATCCCAAGATCTAAGTTCATGACATGAAACCAATGTAATTTGCTTGAGGAGTAAATGGTGGGTAACTAAAATGCCTGCC
CTGGTATCAGGCCTCACTATTTGAGAGGTACCCTCATTTTTATTAAAGTTGTATTTTTCAGAAAAATACAAATCAGGCAC
TGATTATTGAGCATCTGTTATTATGCATCATCTGCCTTTTGTTTTCCCCGTAGGTTTCACTGTGTATTATTGAGGCAGAA
ATGTGCCTGTTGCTTAGAAAACATTTTGATCTGTATTTTAAGTGAGTAAGTGAGTCACCCACCTCGGAGCTCATGTGGCA
ATATTGGTAGTGATTACAGTAAGTTTTGAGGAGCTGTGATTCTTCTAGATTCTAACCACATCAGCTTTGGCCTATTTCTT
TTTTGTAAATTAATTTCTCATATTTAATGACATTTTAGGCTATAATATTGAAACTGATGGTAGACAGACTCTAATACCAA
GGAGCAAATTGTTGATGACTTTATAGTACTCTCCAAGAATAAGGTTATTTGTATTTACAAGATATCTTTTCAAAAAATGT
TTCATGCATTTTTGTGGATCGGTGTATTTAGAAACACAGTTTTGGCTTAACCAAACCAAAAATTTTTATTAAGGAGATTT
CAGATCCAAGCACCATAGAGTAAATTTCTTTGCTAAAAATAATATTACAGTTTTCTGATTCAATCAATCATGAGTGCTAC
CTTTAGAAATTTTTAAAAAATTGATAAAATATATCTTTACCTCATGATTGTTTTTACTCTCAAAGATAATGTGGGATCAA
AATGTAATTTATTATAAACTTGGTAATTATTAGAAGTGTCCCTGGTGTGACACTTAACCCCCCAAACAGGCTATTGAGTG
CTCTTGGAGGTCTCTCAGCAAATAAAGCCCTGTTTTATCCTAAACGTAGATGAGACTGGAAAAGTCTAATCTGTAGATTT
CTTCCTGAGCCTCTGCTGTCAAACGCAGCACTCCTAGCAGGCTCTTCACACAGAGCATTTCCAAGTACTCTAAAGAGTAG
AATCTTAGCAATACATTAAAAGTTACTTCAAAATCAGACATCAGATACTTTCTTCTGGAAAGCCATATCTGAGGAGAGTT
TCTCATCATTAATATTCACCATGATATCAAGGTTGCCTGGACTTAGTCTTGCTATGTTGCCCAGGCTGAAGTGCAGTAGC
ACAATCTTGGCCCAATGCAACCTCCGCCTCCCAGGTTCAAGCAATCGTCCTGCCTCCGCTTCCTGAGTAGCTGAGACTAC
AGGTACATGCTGCCATGCCGGCTAATTTTTTGTATTTTTAATAGAAATGGGGTTTCACCATGTTGGCCAGGCTGGTCTCG
AACTCCTGACCTCAAGTGATCTGCCTACCTTGGCTTCCCGAAGTGCTGGAATTACAAGCGTGAGCCACTGCACCCAGCCT
TCCTGGACTTTTAACTGCAACTGAGTTGCTAAAAAGTTAGTGAAAGGGGAAACTGTGACAGTCCTTGGAGCTTTCTCATG
CAGGATCTATTTTTTGAAACAAATGAAAGGTTTCCCCTTACTCTTACCTACTCAGCTGAAAGGAAAGATTGACCTGGGAAA
GGGAAGCTTTATTCAAGTGCTTCCAGCTTTGGCTAATGACTACCTCTCATATGCTTTCCACTCAAGGATCTTTAAGGTGA
TGGTAAAAGATGAATGGGGGAACAAGGGAAGACAAGCTAAAGTTGGGAAAATTCTAAATTACAGAAATTATTTTGCATA
GCCCGAATATAAAGAAGGTTGATGCACTAGAAAGGGGAAAATAAAATCTTGATTTTCAAATAAATATTACAAGTGCATGC
CCTTATGCCCTCAGATGAGAGTGTTGAATAAAACCTCTCATTGCATTTACTGTGAAAGATTTGTCTAGAATTAAGTCACG
GGAATGTTTCGGCAGTGCTGCAAACTTATTGAATAACAGAAGAGAGAAGAAACTTGCTCAGTGCCAGCCATTGAGAAGTG
CTAGATTTGATGCGAGACCTTGCATTTTTAGAGTAATGCTTCTCTCACAGGAACTTGGAATTATATTATAAGGAAGCATT
CTGACTTGAGCTATAATAACCTAGAGTATACATGCTGAAGATGAAGCTCAGGACCTGTTCTTGTTTCAGCAGCGATGTGG
ATATTCCAAAAGGAAGACATAATCACTGAAATAAGCAGTAATAGTTGCTGGGCACTCGCCTAGCATATATTTCCTACTCA
CAATAGAATAAGAAGCAAAAATTGCCCTGTGGTGCCCAAGGATTGCTATTTTCTCCTTCACTGGCATATTTAAAAGACAAA
AATCTGGCTTTATAATGAAATGGAAGAATGAAAAATTCACTTCAAGTCTGAGGACCTATTTTCCAGTTTTCATCCCAAAT
AATATTTTATTGAATAAGTGAAAAATAGCTTGAGAAAAACAGGAACCAAAATGGAAACTGACAACAGCCCCTTAATGATG
AAATAATTGTTAGGTTTGAAGTGCCTCTTTGTATAGTGGTTGAATTGAGTAATCATGAAACAGATAATATTTTGTAAGGT
GTGAATCTATTACTAGAAAGACCAGTGTTGCCCAATGAGCCATGACATCTCATATTTGATGAATATATTATCTTCATCAG
AAAGCTTTGTAGCAAAGACCCCATGGGAATGGGAAAATATCTAATTTCTGGCATGCATTTGCAACTTTCCATTTTCAGGGG
AAGTAATTGTCTCGAAATGAAAAAGAAACAGAAATCAAAATTCAGACGCTGACATCAGATCACAAATCTAAGGTAAGAA
AATGCCCATTTTTACAGCAGTGACTTGCAGAAGACACTTTGTAATTTTTTAGAAGGAGTACCTAGTGTAAATTAGAAATA
ACAGTAGCCCAGAGCTGGTCTAGTAGCCTGCAGCTGATCTGGGATGTCAGGGGAGCCCAGGGTTCATGTTTGCCTCAGAC
TCACTGGCTTTAAGAAGAGCCATTTCATGGAACATCTTCTTTTGTCTTGTCTGTTGAACTATGCTAACAATATTTATGTT
GCCTGCCTCAGAGGCATCAAGGAAAATAATAAATGAAGATACTAAAAGGAAATATTATATAAAATACAGAATATACTTCC
CTATAGAAATAACATTAAACATAGGACAGAAAACCGCCCAAATTGGGAGGTGATACTTACTCCATCCTAACTTTACATGG
AAATTTGGAATGAGAAAAAATTAAGAGCTGATATTCTAAAAGAGAAGCCTAATTACTCACATTTTCTTAAAGACAGGAGA
AAATAGGAGCTTGAGAGACTTTAATCAACTTTGTGGGCATTTGGAGAACTAAGTGTTAAGTCCCAGTTAAGAATGAAAAC
AATTCTTCCAGCAATGATAGTTTTTAAATGAAAAGGAAAAAAAAGAAAAAAAAAACCAACAACAACTGTTAAAGAAAGC
TCCCAAAAATAGCTGTGGTTGAACCAGCCAGCCTCATCTATCTATATGCAGCTGTGTCATCTGGCCTAATGGCCCTGCTG
CATGCATGGAGCAGGCAAGATGTTCAAGATGTTCTTGGTAGCTGTATGTTTTGTAGGGCATGAACTCTATAGAGGCAGTA
AGGCATGTGTCTTTAAGAGTTATTCCCCACTCTCTCTCTTCTTAACTGGAATGTTTGAGAATTAGTAGTTGTGCAAACTG
TCATGATTCTTAGATGCTTGGCATCAATAAGAAAGCCTTGGCAACAGTATCAGTTTGCTTTTGCTATATAATGAACTGT
GTCAAAGAAGAGCACATTGAAACAACTACTACAGTTTATTAGGTCACAATTCAGTGGGTCAGAAATTTGGGCCATACTTC
TGCTGATTTTGGCTGGAGACACTTGTGTATCTACTGTCAGCAGGGGGCGTATGGCCTTACTTATGTATCTGGTAGTTGTC
TGGCTGTTGCTTGTTTTCCTACATGACCTATCAACCTCCAGCAGGCTAGCACAGGCTCAGTCAGAGGGTAGCCACAGGGA
TCCCAGCATAGCAGAAGGGCAAGTACTTTCCAGCCTCTGCTTGCATCACATTTGCTACTATCTCATTGGCTAAAGCAAGT
CACAGGTCACCCAGCTTCAAGGAGCTGGAGAAACAGACTCCAGCTCTTGACAAGAGGCACTGCAAAACCCCACTGCAAAG
AGCTTGGATATTGGGTGGGGAGGACTTTCTACCCTAGTGTCTATCAGGGTTGACACAGTAAGTCATGATCCAGAAATAAA
AGAACACACAGCTCTCTATTCAGACATGTGGGCTTGTGGACATGAAGCTGGAGAAACATAAGGTGATAAAGAAAATCCTG
ATGGAATTGGTAAAAGAGCCTAAGGCCCACACAAATCAGAGTGTTGGCTGAGTGTGGTGGCTCACGCCTGTAATCCCGGC
ACTCTGGGAGGCCGAGGCAGGTGGATCACCTGAGATCGGGAGTTTGAGACCAGCCTGGCCAACATGGTGAAACCCTGTCT
CTAGTAAAAATACAAAAATTAGCTGGGCATGGTGGCACGCATCTGTAATCCCAGCTACTTGGGAAGCTGAGGCAGGAGAA
TCGCTTGAACCCAGGAGGTGGAGGTTGCAGTGAGCTGAGATCATGCCACTGCACTCCAGCCTGGGCAACAGAGGCAGACT
CTATCTAAAAAAACAAAACAAACAAACAAAAAACAAACAGAGTGTTGCTAAGTGAGGCAGTAAAATAAGGGTTCACAGT
TAACTATTCAGAGCCAAAGCAATTGATTTATTTTGATAAGCAATAACTTCCTGCTTCCCATTGATTAATACATATTATTT
TTTGGTGGTTATAGGTGTCTCATGATTCACCAATTAAGTTGGTGGGAGGAACCTGAAATACCTGAATAATGTTGTGCTTT
```

```
TGGGTTCATACCTGACCTATGAGGATTACAATGGCACCGTCCCCTTTTTCCCAAACAGGTCAAAGAGATTGTAGCACAGC
ACACAAAGGAATGGTCAGAAATGATCAATACCCACAGTGCTGAGGAGCAAGAAATCCGAGACCTGCACCTCAGCCAGCAG
TGTGAGCTGCTGAAAAAGCTACTCATCAATGCCCACGAGCAGCAAACCCAGCAGCTGAAACTGTCCCATGACAGGTGGGG
GAATTGCCGTCTCCAGAGTAAACATCTAATATTTAAAAACAAAGCTGATTTTGTGTGTATAAGAGAGCCAAGGTAATAA
GTGAAATCAATTTGACGTACAACATGTAGCTCTTTTGTGTATCTCATCATATGGCTGATTATGAGCAAGATGAGCTATAA
GAAATGGCACCCAGGGGCCAGGTGCAGTGGCTCACACCTGTAATCCCAGTGCTTTGAGAGGCCAAGCAGGAGGATTACT
TGAGCCCAGGACTTCAAGACATAGCAAGACCCCATCTCTATTTTGAAAAAAGAAATGGCCCCTGCCATCAGAGGGCTTAC
AGGTGTTGGCAAGATACAACACCCATATGAACAGTTAAGTCAGATAACAAAGTAGCGTATGACACATATTTTAAAATGTC
ATGGGCACTAAAGGCATCCAGCTCAGAGGAGCTCTACCTTTTCAGAGTGGTCATGGAGACTTTTCAAAATGGTAGGGCTG
GAACTGGCCTTTGGAGAATGATCTGGAGTCTCTTAGGTAGAAAAGACACACAGAGAGATAAAGACCAATCAGTTCAGCTC
CTAGAGTGACCAGGTTCTGATCATTTAATAGCTACCTGTGGAGAGAGTCTCCATTTCCCTTCCTTACAGTGTTAGGACTT
CTGTGAATTTTGAAAGATAAGCCTGATTAAAAAACAGCAAAGTATCAGTTTATTTTGGCCTGTGCCCACTTCAGCAAAG
TGAATATTGCATTTGAAATACTGCAAGCCATGAGCTGGACTTATCCCTGAGCAGAAGAAGGTTGGTGTTTGTTACATCTT
TTTATCATAGCTCGGTTGCAGCTTGCTTGGTTCTCACTTAGTGCCTACCGTATGCCACAGTTAGGGTCTCTTGAGACACA
ACAAGAGTAGAAAATAGAAGCATTATACAAATAAATCAAACAAAAAATGTGTTTAATGCCAACTGCATAAGTTATCTGG
CTGCATGCCCAAATTCCCAACATTACTTTGGAGTGACAGATACTGCCACAAGAACAAAGTGTTTTGCCCTGGTTTCCCA
TTCTCATTTGAGGCAACTAAATGCAGTCTCCCATGCTCCATTTTCTGTGGGATAATGTGCTGTACCCCCATGCATTTTAA
AGCATTCTATTTCGTCCTATAGGATTAGAAGTTGTCTTTTCTTTTTTCCTTAGAAATGCTCTTGATTCACTATGCAGAAA
GTTGTGTTCATCATTCTGCCTTTGATGATTGCTACAATGCCAGATTTTAGGTGCCTACCCTTATTCAAAGATACTTCACG
GAATACAGCATGGCACTTGTCTAATCTTCTTTTACATTGTTGAATAAAGAGAAAACATTAGCTCACAACAGTAGGCATT
ATCTGAAAGGAAAAAACAAAATATACTAATTCTTGAATTGCTTAGGAAGTCATTTGAAGAGATCATGACCTAGTTACTAA
AATACTTTAAAAAATAAATCCATGGGCTTCATTCCAAGATGGCTGAATAAGAACAGGTCCAGTCTGCAGCTCCTAGCATG
ATCGATGCAGAAGATGGGTGATTTCTGCATTTCCAACTGAGGTACCTGGTTCATCTCATTGGAACTGGTTGGACAGTGGG
TGCAGCCCAAGAAGTGTGAGCCAAAGCAGGGTGGGGTGTCACCTCACCCAGGAAGCACAAGGGGTCAGGGGACTTCCCTT
TCCTAGCCAAAGGAAGCCGTGACAGACTGTCTCTGGAAAAACAGGACACTTCTGCCCAAATACTGTGCTTTTCCCAAGGT
CTTAGCAACCGGCAGACAAGGCGATTCTCTCCTGTGCCTGGCTTGGCAGGTTCCACGCCCATGTAGCCAGGCTCACTGCT
AGTGCAGCAGTCAGATCGAACCGTGAGGCAGCAACCTGGCTGGGGTAGGAGCGTCCACCATTGCTGAGGCTTGAGTAGGT
AAACAAAGTGGCCAGGAAGCTCAAATTGGGTGGAGCCCACTGCAGCTCAGCAAGGCCTACTGCCTCTATAGACACCATCT
CTGTGGGCAGGGCATAGCTGAACAAAAGGCAGCAGACAACTTCTGCAGACTTAAACATCCCTGTCTGACAGCTCTGAAGA
GAGCAGTGGTTCTCCCAGCATGGCGTTTGAGCTCTGAGAACGGACAGACTGCCTCCTCAAGTGGGTCCCTGACCCCCATG
TAGCCTAACTGGGAGACACCACCAAGCAGGGGCCGACAGACACCTCATATAGGTGGGTGCCCCTCTGGGACAAAGCTGCC
ACAGGAAGGATCAGGCAGCAATATTTGCTGTTCTGCAGCCTCCGCTGGTGATACCCAGGCAAACAGGGTCTGGAGTGGAC
CTCCAGCAAACTCCAACAGACCTGCAGCTGAGGGACCTGACTAGTAGAAGGAAAACTAACAAACAGAAAGGAATAGCATC
ATCATCAACAAAAAGTTCATCTACACCAAAACCCCATCTGTAGGTCACCAACATCAAAGACAAAAGGTAGATAAAACCGC
AAAGATGGGGGAGAAACCAGAGCAGAAATGTTGAAAATTCTAAAAACCAGAGCACCTCTTCTCCTCCAAAGGATCGCAGCT
CCTTGCCAGCAATGGAACAAAGCTGGATGGAGAAGGACTTTGACAAGTTGACAGAAGTGGGCTTCAAAAGGTCAGTAATA
ACAAACTTCTCTGAGCTAAAGGAGCATGTTCAAACCCATCGCAAGGAAGCTAAAAACCTTGAAAAAAGGCTAGATGAATG
GATAACTATAATAAACAGTGTAGAGAAGACCTTAAATGACCTGACGGAGCTGAAAACCATGGCACGAGAGCTTCGTGACA
CATGCACAAGCTTCAATAGCTGATTCGATCAAGTGGAAGAAAGGGTATCAGTGATTGAAGATCAAATTAATGAAATAAAG
CAAGAAGAAAAGATTAGAGAAGAAAGAGTAAAAAGAAAGGAACAAAGCCTCCAAGAAATATGGGACTATGTGAAAAGACC
AAATCTGTGTTTGATTGGTGTACCCAAAAGTGATGGGGAGAATGGAACCAAGTTGGAAAACACTCTTTAGGATATCATCC
AGTAGAACTTCCCCAACTTAGCAAGGCATGCCAACATTCAAATTCAAGAAAGACAGAGAACACCGCAAAGATACTCCTCG
AGAAGAGCAACCCTAAGACACATAATTGTCAGATTCACCAAGGTTGAAATGATGGAAAAATGTTAAGGGCAGCCAGAGA
GAAAGGTCGGGTTACCCACAAAGGAAAGTCTATCAGACTAACAGCGGACCTCTCGGCAGAAACCCTACAAGCCAGAAGAA
AGTAGGGGCCAATATTCAACATTCTTAAAGAAAGGATTTTCAACCCAGAATTTCATATCCAGCCAAACTAAGCTTCATAA
GTGAAGGAGAAATAAAATCCTTTACAGGCAAGCAAATGCTGAAAGATTTTGTCACCGCCAGGCCTGCCTTATAAGAGCTC
CTGAAGGAAGCATTAAACATGGAAAGAAACAACCGGTACCAGCCACTGCAAAAACATGCCAAATTGTAAAGACCAACGAG
GCTAGGAAGAAACTGCATCAATTAATGGGCAAAATAACCAGTTAACATCATAATGACAGGATCAAATTCACATATAACAA
TATTAACCTTAAATGTAAATAGGCTAAATGCCCCAATTAAAAGACACAGACTGGCAAACTGGATTAAGAGTCAAGACCCA
TCAGTGTGCTGAATTCAGGAAACCCATCTCACATGCAGAGACACACACAGGCTCAAAATAAAGGGATGGAGGAAGATCTA
CCAAGCAAATGGAAAGCAAAAAAAAAAAAAAAAAAAAAAAGCAGGGGTTGCAATCCTAGTCTCTAATAAAACAGACTTTAAA
CCAACAAAGATCAAAAGAGACAAAGAAGGCCATTACATAATGGTAAAGGGATCAATTCAACAAGAAGAGTTAACTATCCT
AAATATATATGCACCCAATACAGGAGCATGCAGATTCATAAAGCAAGTCCTTAGAGACCTACAAAGAGACTTAGACTCCC
ACATAATAATAATGGGAGACTTTAACACCCCACTGTCAATATTAGACAGATCAATGAGACAGAAGGTTAATAAGATATCC
AGGACTTGAACTCGGCTCTTCACCAAGCAGACCTAATAGACATCTACAGGACTCTCCACCCCAAATCAACAGAATATACA
TTCTTCTCAGCACCACATCACACTTATTCTAAAATTGACAACATAATTGGAAGTAAAGCACTCCTCAGCAAATGTAAAGG
AACAGAAATCACAACAAACTATCTCTCAGGCCACAGTGCAATCAAATTAGAACTCAGGATTAAGAAACTCACTCAAAACT
GCAAAACTACATGGAAACAGAACAACCTGCTCCTGAATGACTACTGGGTAAATAATGGAATGAAGGCAGAAATAAAGATG
TTCTTTGAAACCAATGAGAACAAAGACACAACATACCAGAATCTCTGGGACACATTTAAAGCAGTGTGTAGAGGGAAATT
TATAGCACTAAATGCCCACAAGAGAAAGCAGGAAAGATATAAAATTGACACCCTAACGTCACAATTAAAAGAACTAGAGA
CGCAAGAGCAAACAAATTCCAAAGCTAGCAGAAGGCAAGAAATAACTAAGATCAGAGCAGAACTGAAAGAGATAGCGACA
```

```
CAAAAAACCCTTCAAAAAAATCAATGAATCCGGGAGCTGGTTTTTTGAAAAGATCAACAAAATTGATAGACTGCTAGCAA
GACTAATAAAGAAGAAAGAGAGAAGAATAAAAAAGATGCAATAAAAAAATGATAAAAGAGATATCACCACCGATCCCAC
AGAAATACAAACTACCATCAGAGAATACTATAAACACCTCTATGCAAATAAACTAGAAATCTAGAAGAAATGGATAAAT
TCCTGGACACATACACCCTCCCGAGACTAAACCTGGAAGAAGTTGAATCTCTGAATAGACCAATAACAGGCTCTAAAGTT
GAGGCAATAATTAATAGCCTACCAATCAAAAAAAGTCCAGGACCAGACAGATTCACAGCCGAATTCTACCAGAGATACAA
AGAGGAGCTGATACCATTCCTTCTGAAACTATTCCAATCAACAGAAAAGAGGGAATCCTCCCTAACTCATTCTATGAGG
CCAGCATCATCCTGATACCAAAGCCTGGCAGAGGCACAACAAAAAAGAGAATTTTAGACCAATATCCCTGATGAACATC
CATGCAAAAATCCTCAATAAGATAAATACTGGCAAACCAAATCCAGCAGCACATCAGAAAGCTTAACCACCATGATCATG
TCGGCTTCATCCCTGGGATGCAAGTCTGGTTCAACATATGCAAATCAATAAATGTAATCCATCACGTAAACAGAACCAAT
AACAAAAACCACATGATTATCTCAATAGATGCAGAAAAGGCCAACAAAATTCAACAGGCCTTCATGCTAAAAACTCTCAA
TAAACTAGATATTGATGGAATGTATCTCAAATTAATAAGAGCTATTTATGACAAACCCACAGCCAATGTCATACTGAATG
GGCAAAAACTGGAAGCATTCCCTCTGAAAACTGGCACAAGACAAGGATGCCCTCTGTCACCACTCCTATTCAACATAGTG
TTGGAAGTTCTGGCCAGGGCAATCAGGCAAGAGAAAGAAATAAAGGGTATTCATTTAGGAAAGAGGAAGTCAAATTGTC
CCTGTTTGCACATCACATGATAGTATATTTAGAAAACCCCTTCATCTCAGCCCCAAATCTCCTCAAGCTGATAAGCAACT
TCAGCAAAGTCTCAGGATACAAAATCAATGTGCAAAAATCACAAGCGTTCCTATACACCAATGACAGACAACCAGAGAGC
CAAATCATGAGTGAGCTTCCATTTGCATTTGCTACAAAGAGAATAAAATACCTAGGAATCCAACTTACAAGAGATGTGAG
GGACCTCTTCAAGGAGAGCTACAAACCACTGCTCAATGAAATAAAAGAGGACAGAAATGAATGGAAGAACATTCCATGCT
CATGGATAGGAAGAATCAGTATCGTGAAAATGCCCATACTGCCCAAGGTAATTTATAGATTCAATGCCATCCCCATCAAG
CTACCAATGACTTTCTTCACAGAATTGGAAAAAACTATTTTAAAGTTCATATGGAACCAAAAAAGAGCCCTCATTGCCAA
GACAATCCTAAGCAAAAAGAACAAAGCTGGAGGCATCATGCTACCCAACTTTAAACTATACTACAAGGCTACAGTAACCA
AACCAGCATGGTACTAGTACGAAAACAGAGATATAGACCAATGGAACAGAACAGAGCCCTTAGAAATAACACCACACATC
TACAACCATCTGATCTTTGACAAACCTGACAAAAACAAAAAATGGGGAGAGGATTCCCTATTTAATAAATGGTGCTGGGA
AAACTGTCTAACCATATGTAGAAAGCTGAAACTGGATCCCTTCCTTACACCTTATACAAAAATTAATTCAAGATGGATTA
AAGACTTAAATGTCAGACCTAAAACCATAAAAGCCCTAGAAGAAAACCTAGGCAATACCATTCAGGACATAGGCATGGGC
AAGGACTTCATGACTAAAACAAAAAGCAATGGCCACCAAAGCCAAAACAGACAAATGGGATCTAATTAAACTAAAGAGCT
TCTGCATGGCAAAAGAAACTACCATTAGAGTGAACAGGCAACTCACAGAATGGGAGAAAATTTTTGCAGTCTACCCATCT
GACAAAGGGCTAATATCCAGAATCTACAAAGAACTCAAACAAATTTACAAGAAAAAAACAACCCCATCAAAAAGTAGGCA
AAGGATATGAACAGACACTTCTCAAAAGGAGACATTTATGCAGCCAACAGACACATGAAAAAATGCTCATCATCACTGGT
CATCAGAGAAATGCAAATCAAAACCACAAGAGTTGCCATCTCACGCCAGTTAGAATGGCGATCATTAAAAAGTCAGGAAA
CAACAGATGCTGGAGAGGACGTGGAGAAATAGGAACGCTTTTACACTGTTGGTGGGAGTGTAAATTTGTTCAACGATTGT
GGAAGACAGTGTGGCGATTCCTCAAGGATCTAGAACTAGAAATCCCATTTGACCCAGCAATCCCATTACTGGGTATATAC
CCAGAGAATTATAAACCATGCTACTATAAAGACACATGCACACATGTGTTTATTGTGGCACTATTGACAATAGCAAAGAC
TTGGAACCAACCCAAATGTCCATCAGTGATAGACTGGATTAAAAAAATGTGGCACATATACAACATGGAATACTATGCAG
CCATAAAAAAGGATGAGTTCATGTCCTTTGCAGAGACATGGATGAAGCTGGAAACCATCATTCTCAGCAAACTATCACAA
GGACAGAAAACCAAACACCGTATGTTCTTACTCATAGGTGGGAATTGAACAGTGAGAACACTTGGACACAGTGTAGGGAA
CGTCACACACCGGGCCTTTTGGAGGGTGGGGGACTGGGGAGGGATAGCATTAGGAGAAATGTCTAATGTAAATGACGAG
TTGATGGGTGCAGCAAACCAACATGGCACATGTATACCTATGTATCAATCGTGCACGTTGTGCACATGCACCCTAGAACT
TAAAGTATAATAAAAAATAAATAAATAAATAAATCCATGCAGAAGCACTTTTCAAAGCAACAGCAGTATTTTAG
CTTTTTCTCAAACCCCTGGTGGTTGTGTTTCTTCCTCATGATCACAACTATTATTCTTGGTTAAAAATCTTTGCTTTCTT
GACATTTGTCAGCAACGTTTGGTCATTGGTGTTTGTTGAATGAATGAATACAGAAAGGTCAATTTCCTAACAAGTTATGG
TAAAATCTGAGAGTGATCTTCTTGTGTCCAGAGTTTCTATACATGTTCCTGCTGGAGTGGCTCTAGGGTAGCTGAGTGAG
CAACTCTCCAGTTCAGTATTGCCCATCTCTTTGGATGCAGAGCAAATAGCAGTGCCTAGGTAACCTGACTGCTTGTATGC
TGGTTTCCAGGCAGTGCTTTCAATAGATCTCCATAGGTTATAGAAAAAAATTTTTGTAATAATATACTATGTAGCATTTT
AAGTTTTATTCCTTTAATGCATGTGTGTCATGCATACACATGCATTCCCACATGCAGTGGTTGGTCACCTGCAAACCTAC
TTTTTTGCTATTCCCTGTGCTTACTTGTCTCTTCCAGCCCATCTGATCCCAAATAATATGAAGCAAAACTGAGATCTTTATAT
TTATGGGCAAAAATACTGTCTCCTCATCACAGGCCATCACTGTCACTTCACCACTAGGTGGCCACTGCCCCTCAAGGATC
AGCATCCACATAGCTTGGGGTCCCGTCCCTCCTTTTTCCAAAATACAGAAGTCGATGAGCTCAATAATAGAAAGAAAAAA
AAAGAAGAGTGAGAGGAGTGAGCCTAGTCTCATATTGCTATAAACAGAAGTATTTTTGTTTTTTTCCATGCTTAAATTATGT
CTATATATATAAAATGTAAACTTTACTGTTGTAACCATTAAGTGCATGACATTAAGTACATTCACTTTGTTGTGTACCCG
TCACTGCCACCCACCTCTAGAACTTTCTCATTTTCCCCAGGTGAATCTCTCTACCCATTAAACACAAACTCTCCATCCCC
CCTCCCTGTTTGTTTTCTTGTTTTTAATACATAGGGAAAGCAAGGAAATGCGAGCACACCAGGCTAAGATTTCTATGGAA
AATAGCAAAGCCATCAGCCAAGATAAATCTATCAAGAATAAAGCAGAACGGGAAAGGTAAGTCTGAGAGTGTTCACTGCA
GGAATATGGCATTGACTTGAGGACACAGTCTCAACTTTCTTTATGTGTGTACACACACACACACAACTGAGACAGCAAAACA
CTTGTGGCTTTTGACTAGGAATTCTGCTATTATAACTGCAATGGAGATTTGTTATTATCACTGTAGAACAATCGTCCACA
TTTTCTGGTTGCTTACTACCACCAGTAAAATTAGTTTACATTCAATATATATGTGCTGCTTTACAAACAAAGTTGCATAA
AATATGACATAATAATATAATTCTTTTTTTTATTTTATTCATTTTTTTTTTTTGCTTGTTTGTTTTTGAGATGGAGTCTTGC
TTTATTACCCATGCTAGAGTGCAGTGGCACAATCTCAGCTCACTGCAACCTCTGCCTGGGTTTGAGCGATTCTCCTGCCT
GAGCCTCCCTAGTAGCTGGGATTACAGGCATGCACCACCACACCTGGCTAATTTTTGTATTTTTAGGAGAGATGGGGTTT
CACCATGTTGGCCACACTGGTCTTGAACTCCTGACCTCAAGTGATCTGCCCGCCTCAGCCTCCCAAAGTGCTGGGATTAT
AGGCGTGAGCCACTGCGCCCAGCCTATAATCAATATAATTCTGAAGCATCCTTCCCAGGTTCCACTGGGTCATTCTGCAA
```

```
GCCTCTCCCCTGCTCAGGTGTCAGTCCGTTCTGGATGGGCCAGTTCTGAATAGCAGCAACAGTCAGGAGGGCGGTCTTCT
TCAGTAGTCCTTCACATGGGATCATCTTGAAAGCCCCCATCCCATTTGTCTCATTTGTCCTTATGTTAGGTGAGTTTTCT
GGAAGCAGATCAAGGCAGGAATTGATGTGCTTGTGATTCACTGAGGAAGGGACCTTCAGAGAAAGGGAGTGAGAAAGCAG
GAAGGCCCAGGGCAGGCCCTAAGCAAGGATGTGGTCTCACCTAGGGTCCCAGTAACCTCAAATAAAAAATTCCACCACAG
ATTTGTCCCTCTTTGAAGCAAGAGTACCTGGCTTTTGTGTTTCCATATTAGGCAGTCATTGGCCGCCTGAGACTGGGGGG
AAGGTTTGTGTGTCCTCTATAGAAGGTGCCAAGTGCAAGCCCTTTAGACACAGCTGGTGAAGGGGATCTGAGGAGCTGTC
AATAGTCATGTTACCCAAGGGATAGGCACTTAATTCCCAGCGATCACCAGTGCTGCCCATCATCCACCTCAAGGAACATC
TGAGAATACAAATGCTATCAGCTCCATAACCAAGAGCCTGGATTACGCCACCAGCAGTTAGGGACAGTGGGGTTCCTTCT
TTTGGGGGAATAATACCTAGCAAGAAGGTGGCCTTTTGGCCAAAGTCCAACAGAGAAAAGCTGTTTTATGAGCCAGGTGC
AGTGGCTCACACCTGTAATCCCAGCACTTTGGGAGGCCGAGGTGGGCAGATTACCTGAGGTCAGGAGTTTGGGACCAGCT
TGACCAACATGGTGAAACCTCACCTCTACTAAAAATACAAAAAATTAGCTGGGTGTGGTGGTACCTGCCTGTAATCCCA
GCTACTCAGGAGGCTGAGGCAGGAGAATTGCTTGAACCCCAGGGGCGGAGGTTGCAGTGAGCCGAGATCCTGCCATTGCA
CTCCAGCCTGGCCAACAGAGCAAAACTCCATCTCAAAAAAAAAAAGAAAGAAAAAATGTCATTCTCTGGATGTTATTCT
CAGATAAGCAACTTTGTTTAAGGGCCTTATATACATGGAAATGACACCTTCTTCAGTTTGAAAACACCAAATATTCCAGC
CATGATAAAACCTCATGAATTTGAACTGTTTTAGACTATCCAAAGTGAAATATAGAATTAACCATGACAAATGTGCCAAT
TAATAGCTTTTTACCATCTTTTTCAAATATGTTACAAAGTAGACTTTAGCTCGGTTGAGGTCAAATTATCTTCAATTTGG
AGTTACTGCTAACTTAATTAGCAAGGGTCTTAAAAATTTATCTTATTTTGAAAACTGTACTTATTTGTTGTAACTTTCTA
TTTTTATTTGTGCAACTGTATGGGGGTACGTGAGAAAACTTTACATGTATACAATGCCCAGTGATCAAGTCAGGGCATTT
AGGTTGTCCATCACCCAAGTACTATACATTTGTGGCTAAGTATAGTCACCCTACTCTGCTATCTCTTATAAGTTTGTATC
CAGTGGTATGTGGTTCCAAAATATCTACAAATTGGCTGTTTTTAAAAAATAATTTTTCTATACCAAATGATAGTTATAT
TCTAAACCCATACTGTGTGTATGTTTGTATCCACTTTATCAAACTGAGGAGAAATAAAATTTAAAAATTTGTCATAGCAC
TAAGAAAGATACATAGAAACTGAAAATCATACTTACATTTTTAGATCAAAGAAGACATTTTAACAGAAATTACCCAATAT
TTATAACTGAATGACAATGTAAACACTATTTATCAATGTGTAGAGGGTAGTTAAAGTGATATTTGGAGGGAAAAATATAG
CCTAGATGCATATATTTTTTTAAAAGATGTGAAAATTAATGAGCTAACCACTCAAAGAGAATATGAAGTTGGAAAAACAGA
GTGAACCCAAAGATCTCTGATGGAAAGAAATAAATTTAAAAAGCAAAAAATACTGAAATAGAAAACAAATGCAATAGAG
AACATTCAGAAAAACAAAAGCTTATTTGAAAAGAATAATAAATCAGTTAACTTTCACCAAGACTAAAGAAGAGAGAGAAA
GCACGAATTAGTGATGTTAGGAATGAAAAGGGGAATTAACTACAGAGAAAACGCTTTGAAAAACTAAGTCTACTATTAA
AAAAAACTACCAATAAATTTGAAACGTTAGATGGAGTGGAGAATTGCTTTGAAAAATATAAATATCCAAAACTGACTGAA
AATGGAGAAAACCTGAACAGACTGGCAATTATTACAGAAGTTCAAGAAGTTATAGGTCTTTTCACTCATGAAAAACACTC
TACCTCACCAGTAGTCAGGGAAATGCAAGTTAAGACAATAAGCTCCATTCCATAGGAGCATCCAACTGACAGAAAGTCTG
ATAGTACTAAGAGTGGGCAAAGATATGGGGAAAGGGTAACACCCAGACACTGCAGATGGAAATGCAGTTGGGGACAACTG
GATTGGAGTAAGGAAGAGCCTGTTCGTACCCTGCCAAGTGACAAGTCTGAGTATACACCCTACAGCAGCCTTTGCCTGTG
TGCACAAGAAGATATGCACCATACTTCATCCACTCATCTGTTTATTCAACAGACACTTATTGTGTCAGGTATTACAGGGC
CCACGCACCCGATCGTTGTAGCGTTGTTTGAAAATAACCTAATTTTTGTTAATGGCAATGAGGAAATAGTGTAGTCTGTT
CATAAATTGAAAAGTCCATACAGCAAATGAAAATAATGAAGTGGTCTGGAACATAGGTGTATCTCTAAAACATAAGACTA
AGTGGTAAGCTATGGGAAGAAACCCAGACTATGTTACTCTATGGGTACATTTAGAAAACACAGGAAGCGATGCCACTGTG
CCTGGCCAGCAACATCAGCATCACCTGGGAGCTTACTAGAAATTCAGACCTCCCACCCCAGGCCCACTGGATCAAAGTCA
CTAAGGGGAAGGCACAACAGTCTGTGGTTTTTATCATCTCTCCAGGTGATTGGTATGGCACAGGGAAAGTTTGAGAAGCTCT
TATATTACACATGGGCACATCTGTATGTGGTTTAAAATATTAAAACATGGGTGAGAAGATCCACACCAAATTCACAATAG
TGGTAACCTCTGGGGAGAGGAGGAATAAGGAAAACTGAATCAGGAAGAATACACATGGGGCTTTAATTACATCTGGAACTG
TTTATTGCCTTTAAAAATGTGAGATAAACATGGCAAAACATTAAAAATATCAGTTAATCTGAGGGACACATACACAGATT
TTTAAATATTCTTCTGGACATTATATGTTTGAAAATACTGCAATAACAATTAGAAAAATAAAGCAATAATTCCATCCA
GAGAATTTGATGGAATATAGCTCTTTATTTATTTGATATAATATTAATTTCATTCAATGTCATACCAACCGTTATTTGTGC
CCATTGCTTTTAGGCGAGTCAGGGAGTTAAACAGCAGCAACACTAAAAAGTTTCTGGAAGAAAGAAAGAGAGTAAGTATT
TTATTATATTTTTGGCATTCTTCCTTTAAAAAAACTATAAACAATAACATGCTTAGCCACCAGACCTCTAGCTAATTTGTT
TAATTTGATTTTTCTGTACATTAAACATTCCAGCTTCTCTGCTCCCTGAAAGCTAAAATAATGTCTCACTAAAATCTTTA
AGGTAATGATTTGGCACCATTTTATACAGAAAATGAAGCCAGGTTTAGTCATGCGTAAGTCCTCTATTATTTTCTAAGAT
TTTAGCAGGAAAGATCTTCCCAGAAATAATTATGAGTTTAAACTCTCCAAATTTTGAGATGATTTTTTAAAAAATTATTA
TCTCTTTCACTTAAATTATAAAGTTACAAGTCATCTCTCATCCCTGTTTTAAGATTGTAACCCAAAGTTCAATCAGAATT
TTTTTTTTTTTTTTTTTTGCAGCTTGCCATGAAGCAGTCCAAAGAAATGGATCAGTTGAAAAAAGTCCAGCTTGAACATC
TAGAATTCCTAGAGAAACAGAATGAGCAGGTATTTTACCTAAAATACGTAAAAACATGCAGGCAGCTAGCCAGCTTTGGG
ATACACATGCCATGGCCCACAGTGACCAGTGGCTTGCATATTGTAATTGAAAGGTAGATAGAAGAATTTGTTGCCCATTA
CTATATTTTCTCCATTATATCCAAAAAGAAAAGAAAGTACCAGTTTTTGGAGCAGGAGCAGAATCTGGCTGTGCCAAAAA
AAGAATATTTGGATAAATTATGGGGATTATGTGAATTCTAAGAATGAAATTACTAAAAGTGAGAGGATGAAAAAAAGAGA
TTACGAAGTATTTCTCCATTTTCATTTAGAGTTTTATAATTTGCAAACTATAGGCAAGGGATATGATGTTAAAAGCAAA
TGAGAGAAACCACCTATTTTCAAACTCAATCTGAAAACGAAGTTTAAAAACCTCCCTGGGACTTACAGGTGGTATCTAAA
GTCATGAGCCCCCATCACTCTACAAATCACTGAGCATGAGTGGATACCATAGTCTTGGTCTTTTTCCCTGCAAGACAAGA
CACTAAATAAAGAAATATACAAAATAAAGAGTTATCCAGGCCCGGGCGCAGTGGGCTCATGCCTGTAATCCCAGCACTTT
GAGAGGCCGAGGAGGGTGGATCAGGAGGTCAGGAGATCGAGACCATCCTGGCCAACATGGTGAAGCCCCGTCTCTACTAA
AAATACAAAAATTAGCCGGGCCTGGTGGCATGCACCTGTAGTCCCAGCTACTCAGTTGGCTGAAGCAGGAAAATCACTTG
AACCCAAGAGGCGGAGGTTGCAGTGAGCCGAGATCGCACCACTGCACTCCAGCCTGAGCGACAGAGTGACTCCATCTCAA
```

```
AAAAAAAAAAAAAGGAGTTACCCAAACACCAACTGTTTAGTTAGGCCAATGGGCATATACCAGCACCAAGGTAAATGTAT
TTTTATCTGGTGCATAGTACAGGTGGGCACCTTTCCAAATGGGCTACAGCCCATCAGCCTTCTACAGTAACCTTTAATTA
ATGGAAATCCCTCTAGTTCCTATGGCAACAAGACCCTCAGTTATCATTTACATTTCAGACTCTCTCAGTTCAAATGTTAT
TCTTTAGGTGTAGTTGGTATAGAAGTTATTATTCCTAGGTAATTCAATTTAGAAACGGAGAGGTTTTGATGAAATATGTA
CTTGTGCCACATTATTCCTTCCTGTTTTCTCCCATCTATAAAACAATCCTATTTAAGGAATACTTTTTTCCAGAGTAAAT
GACTCCCCTTGAAGAATACTTAAGTAGGAATTATACTAAAAGTACCTTTGAGAAAGTAAAAATAAAATAAAATAAAATA
AAATAAAATCTTTTTGTATGGCAATTGGGTAACAGATAAATCTGACTTCTAGTTTCAGATTCTTTACTTTTTGGTGGAC
AGAAATTTTATTCATCAACATACTTCCTAGTTATGTTTCCCATTTCCTTTTCAGCTAAGTGCTTCTATTCATAAGCAAGT
CCACTCCAAGGGGACAATACATGGTTATTGTCCCACAGGTACAATTCATTTGACCAACTCATAAACATCACATTCTGG
TACAAGTTACCTAGCTTATGTTTTTGCTCTTAAAGCACTTCTCAGAAAGTGCTCCTGGGGGAAATATGGGAATATGAGTA
AGGATAAAGTCACCATCTCCAAAGCTTTAGAAGCCACTTCAGTGATTCAACAATGGGTAGGGATAAGGATGCCTTGGAGA
AGGTTTTTAAAAATTCTCCCTAGAGGAAGGGCACAGTGAAAGGTAAGGCACAGTTATGTGTTAGTTAACTAAATTAAGAA
ACTACTTGGAAATTAATTGTGTTGCTTAGCAACAAGTTTGACTAAAGAATGTAAATGTGCTCAGAATTACTCATCACTAC
AATAGTGGTGTATGCCAGAGCTAGAGAAAACAGTCATCTTGTGTCGCTAGCTTTGGAGCCGAAGTACATAATTTTTAAAA
ATCAGTTTGAGCTTTTATCCTAACACATGGCTAATTCAAGCCACACCAGAGTTCTTGCATTAAACTGCTAGATTAGTGAT
AGCTGTATTGATTAGATTGTATTGGGAGGGTGCTGGCTTTTTGTTGCAGAAATTCAAATATGTCTTTTAAGGTCTACCAC
CCACAACTGACACTTTGAGTCACAAAAGCTTGGGAAAGCATAGAACATGAAAAGGCTTGAGACCTGATAAAGATTTAATG
AGATTCAAGGAAAAGACATTCAAGATATCATACTGATGCTTCTGGCAAGCAAAGAATTTGGACTTGAGTTTCTGTGTTAA
GCTTTGCTTTAGAAATATGGCCTGTTCAATTGCAAATTCAATCCCAGATCTTTCTAGTATACATTTTTTGGCATTTAAGG
GTGAGAAAGCTCTCAATTCACAGTTACTCTGTATGGTACAACACTGTCATGGCACAGTGCAAAAGATACAGTGACTTCA
TTTCTTTCCTCATAAAAAGGCAAGGTCTGGAACTATATAGTATAACTGCCTTCATAACCAGGTAAACATTTCATCAGCAA
GAACAGTTCTTTGTCTCCAGACAATGCAAACTTCTACAACAATCTTCTAAATAATTGTTAAATCCCAAGACATTTTTTCT
AAGCCAGAGATAAATGTAAACATTCTCCCATAGTATTTCCCTCAAAGGTTTCTTTCTGTTCTCCTGTGACCCTACTCCTA
ATCGGTGTCCCAAATCTCCCCTTCCAAAGCAAGGCCTGGAATGGATTCAGTCCATTAGCTAAACCAGTGTTGGGGGCACT
GGCAGAAACAAACCAGGCGTCAGTATGTTTTGGTCCTGATTCTGGATCCTCACCTTCTAGGTTATTTGTAAGCCACTTTA
TGTCCTTGCCTACTTGGTCAGCCAACTATTCACTTAACACTCTGAAGGTTGGGGATGTTTTTCCTACTTGAGTTTTTTCA
TAGACTTCCCAGCTACACAGTATATCTCATCTGTCTTGACATGTACCATTTCAAACATTAACGACTCCATTACTGTCACA
CACTCGAGTCTCAAAAATTGATTTTTATCATCAATCCTATCATTTAATAAAGAGAGGTAAAAGGTTTTGCTTAAAGGCCA
GGTTCTCCACTTACTGCCACAGAAGAGAGGCACAGTCTGGGAATGACAGGCGTTGGTTGTTTGTTTCGTTTCTTTTTTTT
CTGTTGAGATCAACTAGGGGAAAGTTTGTCACTGAGGGGTGTGTACATGTTTATCCTTAGCTTTTTGCTTTTCTGTATA
TGGTTTTGCATTCATTTTTCAAATTCACTTCAATATTCATTTATTTTCTTCGTTTTGTATGTATGACTCAATTTTGACAT
TACTATTTTTGTACAAACAGCTTTTGAAATCCTGTCATGCAGTGTCCCAAACGCAAGGTAGGACCGAAACTCTGATAAGC
AAGACGTTGCTTTCCTTCTCGACTACGTCCGTATTCTCTGTGCAGTCTCCATTTATGAGTCACATCTGGTCATAACTAAT
ATCTGAAGGGTTTGGATTTAAAAGCTTGACTATTCCATGCTTGGATTTCTGATGTCATATTCATGCCAAATGCCCTAAG
TCTTTAGAGAATTACTTCTGAGGGTATTTGAACCTGTATTTTCTATTGCAGATTTAGGAAACAGATCATGGTAATAAAGA
CATAACCAGAACAGTGATGTGCAGCTGTCATTTGGACTGACCAGAGCTAAGACAGGGTCACAGAGTCAGGTCACTCACAA
ATGTTAATATGACTCCAATTAATTTTTAAAGCGCTCGCTGAAAGTTGGTTGGTGGTTTTTAATGATGGAGCTGTGCTGTG
CATGTAGAAACCTTACACGATGACTCTTGTCATGCTCTCTGCTAAGTCCCATCTGCCAAAGACCCTAATCACTCCTCATA
AGTAAGGTGGTCATATAATTTATTGTCCATACCAGGATAGTTTTGAGAGTGAAAGGCAGCTCTGTTTATAATATCTAGGA
CTACAGGCATAAACTGGAATTGCCTCAGACAAATGAGAATGAATGGTCACCCAACTCATATAAGAACTTAGCTGTTGGAA
TAGTCTTGTAATGTCCTATGATTCTTCCTTCTACACCGTGGTCAATACTTTCTTCAGAAGCTAAAGGGGGACAAGCACAG
AGAATCATTTGCTACTATTTACCGAACTTGGAAGTATTTAATTTCCAGTGCTGATAATGTTTCACTGTCATCCCTTTTGA
ATATCGTTTGTGTCATAGTTTCATGTTTGTATATTTTTCTCCATTTCTTCATTTCTCTATTAGAGTATTACTATACTTTA
GGCATAAAAAATATCTGGGCAGAGGGAACCAACCATCCCAAATTTACACAGAAAGATTTAATTCTTTGGTGTTATTATAT
CCATCCTCACCAAAATGAAGCTTAACGCCAAAATATAGAAGTAAGAAGAAGAAGGAAATCACAGTATAGTCTGTGGGTTA
AATTAATTTTGTCACAAGAATTCATTTGGAAGATGCTTCTCATATATCTACAGAGTTGACCAAATCCCCTCCCAGTGACC
TTCCATTTAGTTGGAATATCTAAGCAGACATAAATAGTAACATCAGGGCACTTCAGAATCTTCATCCGATTTATATCTTC
ATAGGTCCATGTTTCTATTTTCAAATGTCCTTTATTTCAAAGCAGCATGTCACTAAAAAAAGAAATGGGCAATCATCAT
TCCTCAAAAGATACGTGCATTTGGTTGGGCAAAATCATCCAGGCTACCAGTTGGATAATAAAAGTCGAAATGTACTATTT
GATTTTTTCCTATGTTTCCAAGCAAGTATTTCTCACCAGACACTGCCCCCATCATATCCCCTTTCCTCTTCTCGAATTAG
CTGGTTTTCTTAGTGACAATCTGAACTCATGAGGTTGAATTCACCATTGCTTACTTCCTGGTAGTCCATAAAAAGTACCC
TAGAGAAGTAAGAGCCCACAGGGGATAGAGTTTGCACAATAAGATTCCTGAAAGCCTGCATGTTCCTGTAACATTCCATTT
ACAGAAGACTCCAGCCAATCCAATGTAATATGCTCTCACCTGAACTTCTCTATCAATTAGATTTTTAGGGAATTACTTTT
AACTAAATTTCAGGCTATTTAGGAAATAAGTTGTACATAAGCAATACTTATTCTAATGTGATAAAATTATCTACACACAG
TCAAAGCAACGTGTCAATCTGTAGAAACTAAATCAAACAGTTTGTGGTATGGTCTAAAGATAAAGCAAGTGGTCTACCCT
CTTCTCAAGTGGTTCTCCTGGCACTGTGTAAACCAGTCCCCAAAATTTAATGTGTATAAAAACTTCCTGGAGATCTTGTT
AAAATGAAGATTTTAACTGATTCAATAGATCTGGGGTAGACTCCAAGATTCTACGTTTCTAAGAAGGTCCCAGGCAATGC
TGATGCTGCTTGTACATGGACCATATATTTTGAGTAGCAAGGATGTGGTGACAGGATCATATTTATGGGAGAAGAGAGGCCA
GGGTTGTTAAGTGCCTTCAGATAAGGATTTCAACACACGTAAGGCCATGTTTCTGATGTTATAGGTGCAAGGAGATGCAGC
AGATGGTGAAATTGGAAGCCGAGATGGACCGCGACCAGCAACAGTAGTATGAAACTCCAAAATGCAAACTGAAGCAGCA
AACCCACAAAGCATCAAAAGACTCACTCACAAACTTCTGAACACAAACTCCATGGATGAAAGCTGTTTATTTTGTTTCCT
```

```
TTATGTGTAAACAAGATGATATCTGAAACCAGAGAGACTTGGAATGTCTGACTGACTTCTATTTAACAGCTTGAGTATTG
CATTTCCTTGGCCAAACAAAAATAGCTACAAATCCACAAAAATTTACTATTCCAGTAAGGCAGAGTCCAACCATTGATAA
TACAACTTAAACATGTTTGCTATAAAATACCATCACAAGTAAATGAGCTTGGTGTGAACAACTCTCCTTTGTGATGCCTT
AGGACATGTTTGAACTGCAGCAAAAAACAAAAACAAAAACAGTGCATTAGCAATTTCATAGCAAGTGCATGCACTAGGA
AAAGAAAACTCTGTCTACAAGTTTATTAGCAGAAGTGGTGGTCTGCTAGACAAATAATTTTGCAAAATTTTTCTACATCT
AAGTTACCTCATCAGTAAGTGCCATGTCTCTACCATGCCATCAGAGGCTAATTTCCTGTAAAAGTTGTGGAAATTGTTAG
AACAATAGAAAAATAGAGCAGTGTATGTGTGCCAAAACTCATCATTACTCAAAGGAGAACTGTGTTAGGCACATTTAAGA
AAGTTTACATCTGACATTGCTTTATAGGAATTGTTTCTGCAGATTCCGGATATTATAATTCACACCATAAAGATTGTGAA
GTGGTTATTGGCAAACGTTTGTAAATGTGACCATGTATAAAGTATTTATACTCTTTAATTCACACTGTTAGAGAGCAAAA
TCATCTAAGTATTGCCACATGACAAGATTAGTAAACAGGAATACTAGAACTATGTTTGCATGATACACAAGCACCAATAA
AGACTAATCCATACACAGTTAACCTAATGCCAAATAAATACTGGTTAAATAAATGTATGGCACAGAATATAATTTGACTA
TCAAGACTTTTAGCATAATGAAAAACCCTCTCTCTATATATATATGTGTATATGAATTATGTGGGCATTCTTGATACTTC
AAGTTCTAGTTTGAAAAAAATACATAACTAATTTAATTTTACACAAAAATATTTATGCAGATTTTCAGAATTTCATATCA
GGAAATGACCTTTTTATGTCTGTTAAATATCAAAACAATTTGCTACAGTGTTAATCTGCATGGTCTTTAAGCCTGCTGTA
GTTGAGTTGCAGACAGTGCATGAAAAGTATTCCGCTGGGAATTGAGCCATGCCACCAAAGCCAAGAGGAGCGCATGGAA
ACCCGGTAGTCTAGAACTAATCAGATTACTGATTTTAGGGCACAGCACCAGATGAATTGTTGTATATGCTTGTAAAAATT
GATTCTGTGTGTTCCTCTGAACAAAGCGGAGAAAATGATGATACCATCAATATTGAAATTAAACTTCCAACTTCTCTAAT
AAAAAATTAAAACACGCATAACACTCGTCAAGAGTATTTGCTCCCAAGACACATTCTAGCAAATGTTTTGCCTTTTTCAT
ATACATGATATCATCGTTATTTTCAAAGGGGGCTTATTAATACCCTCAGCATGTTTTTCACCCAAATGATGCAAAACATG
CAGATTCTAGTTGACTTCAGTTGTAATAGACTTGTTTTTCTCCTATTTATGATTTGAAGTGGATTCTGTAAATATCTCT
TGTTCTTAGTTTCCTTATCTGTAAACAGTGGAGTTAGACTACATATCTTTTGGCACTAACATCTCATGAAAAATTATGG
TTAATAAAATATCACCACATTTGGATTGCCAATTTTCAAAGATTCTGAGTGGCTTCTCATCATCAAATCAAATCAAATCC
AAGAGCTCAACTATGCTCACCATTCTGTAACACTTGACTGAATTCATCACTTGCCTCCTCTTGTTTCTGCTTTGGAGAGG
TACTACTAGTTTTTCCATTTTTCTTATTTCTTCCCTCTTTTCATCCTCCTAGGCCACCATAAGCATTTATATTTTCCAGT
GTTATACATGGAACCAGTTGTTTTTCTTTGATCCAGGTTACTTTTAGTATTTGAGACATCATACCATTAATCCATTGTA
GTAGGATTAAAATGTACACACAACTCAAATGTGCAACAAAAGCATGTGTTACCTGCTATTGTCAGTTCATATTGCACTCA
AACCTTGCATTATCCCTTATTATGAAGAAAAATTAATAGCCAATTAAAGTAACTCAGTGTCTTCCAGTTTTGTTTTTTTT
TTTTTAAAAAAAAAAAAAACATTTTCAGGGACATGTTTAGTTACTTGAGAGTCAAATCCCCTGTTAGAGATCCCTCCCCAT
TCAGGAGATCTTGCTTTTTTCCCCAGATGGCATCTGGGTATGAGGGAAGCTCACGTGTCCACCTGGCAATTGGTAGGAGC
ATCTGGTTTCCCCATTGTTGGACAGGTCCTAACTGCACTCTACTGCAGCTTTGCAAAGTGGGCCTTCCCACCCCAGTGGT
TCAAAGTCCCTGCTCAGCCAAGTCCTCATTCTGGTATCAGTTTATTCCAGCATTCTTCTGGGATATGCAGAACTTCCAGG
TCTTTGGGGGCTGTGCAGGAACCAAAGATGCTGTCTCAGGTCCATCCATCAAAACACTCCTTTGGTCATTTGTCAGCTAC
TAGGGCCATGTAGTTTCAATACCACAGAGTTTTCTAAATCCAGGAGGCTTGAAATGTTCCAGAGCAATGCCCCAAAAAGC
AAGAAGCAAGCAAGCTTTATACTGCCCCAAGACTATCTGGGTTTCTACAACATCTTGCTTCTCCCACCATCAGAACTTTT
TGTTCTTATTTGGGAGCCAGGGCACTTGAGTCTCAATTCTTACCTTTATTCTTTCTTTCAGCCCAGAAAAGATACTCAAC
TCCCATTCCTGACTGATGGAATACCCATACAGTACTATACTTCTCTTTTCCTAAGTAGGTTGCTCTTCACATTTTGGGGG
AATAAAAGGCTTCATGGAAAAAAGAGCCAGGTTACCAAGATAGGGAAACACATCAGAAAGTACCATCTTTCATCAAATTGA
AGGCATTATTGATTATAGCTTATACTATTATTTTATATACCACACAAAATGAAACACACTGCCAATTAAACTATAATACC
CTATCCGGTGATTGTCAAGGCACATCCCAATTTCAGAGATGTTAAAATGTGAAAATACATACATCTTGGAATCCATGAA
ATATGTAGTATTCCAAAAGTAGTATACAAACTCCTCAACCCTAAGCCTTAAAGAGGAGAGGAAATTTTAAAGATAGGACA
CAAGAGAATCATTTTATCACAAAACTGAAACTGGTTTTGTGGAACTGAAAAATGGTTCCAAAAAGGAACCATTCTGCAAC
TGAGTTTCAAATTTAGCAATGAGCTTCCTGGAAAGCAATTCAAAAAGGGGAAGAAATCTATCTTTCACCGTCTTTATTTG
AAGAATCTTGTTTCCTTTAGCTGTTTCTTTCAATCACATGCCTTTGATTTCTACTTCTTTGCATGTAATTCTCGAATCTA
TAATTCTAGCCTTTACCTTTCTTTCCCGAAGCTCTGACTCTCCCTCTCAGATTGTCAATAGTTAAATAGGCGGCCATGTG
CACATTGTATGTGATTAATTAAAAAAAAATAGAGACTCTTTCTCCATCTGTCCTCCCCACATATTTTAATAGTCCATAAGC
AGTGCTAAACCTTGTGGCCATCTTTTTCTGCTCCATCTCATTCCTCCAGCACCCAATCTGTTCATAAAACTTATGAATT
CTGTCCTGTCTTGGAAACTTTCTTCTCCTTCCTTATAACTCAACGTTCAGAATTTTAAACTTGCTGTTCAATTTCTTTGC
CCTCCAAATCCCTTTTACACAGAGATTAATCTTCCCAAATGACACCTGCAATCATATTACCATTCTGCTTGTAAATGCAG
AATTCCTGTCTACTGCCTAAAGAATAATGTTGAAATTAGTGATAACAGCATTCATTTTCTGCCCCAACATGCCTACTCA
TTTTAGTCAATATGAATTTATCACTAGTCTCTGTTCACATACACATAGTACACATCTTTTCTAGCCACATTACATAATAA
TGTTCTATTAAGTCTTGAAAAGCAACTTCCAAAAATGCCATTTCTGAAAAGTTCTCTCATTCTCCCAAGCAAGAAGTAAC
CTGCCCTTTTTCATTTATCCATTTATACTTTAAAAGCTATTTAAGCATAAGACTTTTCCACCAGTCCTGACTAATTTCTT
TTCAGGCTTTGTTCAGTTGCTCTTCAAAGAAGAGGTAGTCACTAGATATCGAATTACTAAGTTATTTTCTATGAAGTAAA
ATAACCATCTGTATAGACTCTTGGGCAGGAAATGCCTTTGAAAACTTAATGAAAACCTAAAAGAAGCTTTTTTGCAAAGT
GTCTTTTCAGTTCCAAGAAAAATAATTTTTCTCAATACTTATTGTTAGCATACTTTTAAAACAGTGCAATGATCACAGTG
GCCATCACGGATCTAACAGTGTGGATCCATCAACATTAAGAGAGATTATGGAGAGTGAAGAAGGAATACCCCTATTATTTTA
TTGCTGAGGTTATCTTAGGGACGCCTCGTTCAAAACTCTAGCTTCTCCTTCTTCATCACTTGTGCAAATCTAACAGAAGG
CCACACATTTCTACAACTCTTCTTTGATGCTTGACGTTGAACGGAATCTCAAGCACATATTTTAATTGCATCTTTAATAT
CATAATAAATCTGGTGATACCTCTGGGATGAATATTTATGAGCCATCTTCTTTAATAATATTTTGTGGAAAACGTTCTGC
TCCATAGGTTAGAATAATGAAAAAATATTAGGCGGGTGGGGGGAGTAGGGAGATGGTTAAAATAGCAGCATCATTGCTTT
TACATTAAAAAAATTGGTGTCTACACTGTACTTATCGTGATAAATGATGCTGTGCCTTTTCCACTCCTTTCCTCTCCTAGC
```

```
ATTACCTTCTTGGAAGTTCAGGGTAAGGGGCAATTTCTGAAGTACTTCATCCTGGAAATTTTTGAGTGTCCAGCACAATA
AAATTAGGAAAAATTTCCAAGGAAGGCCAATTTAATATTGTATACCTAATCACTTGCATCAGGACTCAATAGCTTTAGAT
GAATCAGAATGTACCAAAAACAAAACAGACAGAAAAAGGATTTGACCATCTGTCATTTCGGAACTTTGATATCATATAAT
GTGAATGAGGAAATGTTATACTGATCCATCAAAATTGTTTACCCTTTTCAAGCAATTCATGGATTTTCTCAAAATTCACA
TGCAGCTTTTAACACTTCTATCTTTTGTTTTTCCTTAGACAAATCTTGGTATTTTGTTAGGGTGTGGTATTTGTTACAGC
ACAGCATATTGAACCCTACAGACTGTACCCCTGTATTATGAATTCACTTCTACTACATCAAGTAGCCATATAAATGGTTA
TATTAGACATTTCTTCAGCATTGAACTAACTTTATTTGTTCATGGATTAAATATTTATTGGAAAACCACTCTTAACCTCT
CTACCCTTGTAACCCAGTTCCAACTCATTCTGCTCACCACACACAGCCAGTAAGTAGAGAGACAAGGAGTTGGAACAAG
GAAGGTGACTTTAGTTCAAAGAGCCAGAAAACTGAGAAGATGGTGGACTAGCGTCCTAAAGTACCATCTTTGGTCAGTAC
TCATTTCAGACTCTTTATGTTAAGGGTGGTGGGAAGAGGAGGAGTTGGGACTCAAGAGGTGACTGACAACTGCAGACATTT
GAGTGCCAACAAGGGTCTGAGGAGGTTGGAAACTTCTTTGTCTTTGTCTTTAGCCAAGTCACAATGTTCCTATAAATCTT
TAACAAAACATAGTTGCTTACATACTCTCTCTTAATCCCAGAGTACAAATACAAAATGGCTATTTTTGTATTTTATCTG
TGTGCCCAAATTAGCCTAGCCTATGTGCAGGAACAGGAAAAGGCCCCTTAAACAAAAATGGGGTCAGTTATGTTCTTTTA
GTGTTTCACTGTTACACACTTCCAACTTTTTCTCTTTTCTTTTTTTTTTTTTTTGAGACAGAGTGACTCTGTCGCCCAGGC
TGGAGTGCAGTGGCACTGTGTCGGCTCACTGCAACCTCCGTCTCCTGGGTTCAAGTGATCCTCCTGCCTCAACCTCCTGA
GTAGCTAGGATTACAGGCACGCACCACCATGCCTGGCTAATTTTTGTATTTTTAGTGGAGATGGGGTTTCACCATGTTGA
CCAGGCTGGTCTCAAACTCCTGACCTCAGGCAATCTGCCTGCCTCGGCCTCCCAAAGTACTGGGATTACAGGCATGAGCC
ACCGTATCCAGCCCTTCAACTTGTTTTTTTACCAGTAATGCACTCTATTTTGAAAACTTATCAACAGTTTTAGCTTTAGG
CGATATTCATAAGGTAGATGGGTGGCATTAATGGCCCCAATTTTTTATGCACAAAATTATTTAGAACAGTGTATAAAATT
ACCCTGAGGCTATGTGTATACAGTGTAATGACACATAAATATATTTTGTGTTTGGACTTGGATCCCATCCCCAAGATATC
TTATTATGTATATGCAGATATTCCAAAATCTAAAAACAACCGAAATCTGAAACACTTCTGGTCCCAAGCATTTTGGATAA
GGAACACTCAAGCTGTAGCAGCAAACTGGACACGAGTGGAGGCACATTTTTGTTTCTTCACTTTAAGTCTCTGACACTGC
CGTGAGAGCACATGAGAAGGCTGGCCTGCTGGCGGGTATGCGCGACTTGCAGAGAGTGGAGTTATCTCGTTCATTTTACC
AGCTGACAATAGATGTGTGAGCCTGCAGCTGAGATCAGCCATGCCTTTCCCAGAGCCGTGGGAACACCCAGCCAACCTAT
AACCATATGAGAAATAGGAAATGGATGTTGATTTGAGCCACTGAGTTTTGATATTTTTTGTTATGTGGCCATAACTAACT
GATACAGCCAAGCTCTGGGAACAGGAAGATGATAAAACAGTCTCATTTCAAAGTCCCAGAGACTGCAGCTTTCAAAAGCC
ACTGGAATCACCTACAGGCACGGAACATGGTTTTCTCAGGGAGTGATTCCAGCTGCTAAGCCTCATGTTTGATGTCTCAG
GTTTCTTTTTATTTGTAACCTTCTGAGTGTCTGCTGTTCACTTCCTCTAAGCTGGACTGCTGATGATTTCAACTAACTTT
GTCATTTGACAAACACCTCCTCCTCCTCATCCTCCTCCCCCTCCTTCTTCTTCCTCCTCCTCCTACTCCTTCTTCTTCTT
TCTTCTTTGACTTTCTCCCTCCCCCTCCCTGTCTTTTCATTTTTTTGGTTTGCTTCTAAACCTTCTCTCTTTTTCCTTTG
GGCTCCCTGGTCACTCAAATGACCTCCTGCCACAGTCTTGGATGCTAATATTAAGTAGCTGCTTTCGGACTGGTCTGTTT
GAGTTATGGGTCCTAAAATTGCAGATAAACGGGGAATTACTTTACCACATTACTTTGTCCAATTGCATAAAATATGGGAC
ACAGATTTTTTAAATGCAATAGTCTAGCAATGGAAATATATGGTATTGAGTAGTTAGACTGATTTTAAAGCAAAATTCTT
AAAAGAAAAATATAGGAGCATATATTAGGTATTAATAAGAGGAATGTATTTAAATATATTTCGAATAATTTAAAAGTATA
TTATGCTAAATAATATATGCTGAAATAAAATTCAGCAAAGTAAAAATATTTTCTAAGGCTTGGAAGTCTATTAAGCTAAA
CGTCTGCCAATCTTATGTCCCAGCAATTACACTCTAAGAGTAATGAGTGTAAGCCAGACATGGTGATACACACCTGCAGT
CCAAGCTACTTGGGAGGCTGACAATATAATTCAAGGCTATGCCTCCTCTTTAATCCCAGAGTACAAAGTAGTGTTCTATG
ATCGCACCCAAGAATAGCTACCACACCCCAGCGTGCAATACGTAACGAGACCCCATCTTAAAAAAGAAGAAGAAAAAAGA
AAAAAAGAAATGAGTGTATTGTGTATGTGTTCATCAAAAAGCATGTTCTTTATTCATAATAATAAAAAGCTAGAAACAAA
TCAGATGTGTACCAATAGGAGGATGCATTGATGAAATGTAGTATATTCACAGGGTAGAACACATGAAAGTACTTACCTTA
TGATTCCACATACATGAAGATCAAGGAAAGGCAATGCTAATCTTGAAGGAAGTCAGAGTAGTGGTCCTCTCTGAAAAAAC
CTTCCAGGATGATGGTGATGGTCTATGCAAGTGGTTCTCGAAGTGTGTATCAAGGATCCCTTGGCAAGCCATTGTGTCCC
CCAAGAGACTGTCAGAGGGTCTGTGTAGTCCTCTTGTTTCCAAATCCGTCTCTGTGGGAGGCCAGATTTCTTCATATACT
GCAGTGAAAATGACATATCACAACAGACCGAATGAAGAAAAAGACATTAGAACTCAGATGACTTCTGTAAAGCCATACGT
TAAAGACATTTGCAACAATGTAAAACAATGCCACTCCTCCCACCAACTTTTAGAAAATACAGTTATGTTTCACAAAAAAG
TTATTGTAATAGTACAATATTGGTTATATATTAACAAGTGACAGTTTTGTTATTTTTATTGAATTGAGAAATATTTTGAA
ATTCCTGTTTCAAATTTTTAACATAGTAATGCTTACAGATATAATTCACATAAACAAACTCTGAGTTTTTTAAAAATATT
AAGAGTGTTAAGGAATCCCTGAGGCCAAAGATTTTGAGAACTGCTGGTCTATAACTTACGCTGCGTGGAGGTTACATGGG
TGTTTATACAGGTAAACATTGATCAGGGAATCAGGGAATACACCTAAGATTTGTGTCTTCCATTGCATGTATAATTCTCA
ATTAAACCCATGTTTTAAAAATATAGCAATAGATATATGTTCATGGTATAAAGAGAATGGGGGGAATGAAATATAATTTA
GTATACAAAAATACATATACACATTCAGAAGACCAATAGGGATGATGCCAAAATAACAAAGTTTACTTCTGAAGCCTGTT
GCCTAGTGGAAACCATCGAAGAGGCATAATTACTCAGAAGAGTTTTGAGCATAAAATTAATTCAGCAGGCTTTGCTGCTC
CACAAGGAAACTATTCTACTCCTCTCAAAAGCCTGTGCTGAGTGAGGGAGGCAGAGTTGGATGTGGAATAGACTCTCTTT
ATTCTTCCCTTCTAGCATTTTCCATGCCAACTCTCCAAGTCTGCAACAGAAAATGATTTGAAACAATACAAGAGTAACTT
AAGAGAGAGCCTCTCACAAGGGACAGGAAATGACACACCCGAGAAAAATAGCAGAAATATCCACTTAGGAAAGGTGGCAG
CTGTCAGATTAAAGAGAACCCAGATGATGAATTTGGCATAAATTCTCCCAAGGGGATCTGCAGTAACATTAAATGATAGT
GCAAAGCAAAATGGAAATGGGTGCAACTGGGATCAGCTGAGATAGATGAAACTTTCTACTTGTAGAAGTCAGTGTGGCAA
TTAACAGTCTAATTTGGGATCCCACTGACTACAGCTTAGATCCTAACTCCGCTATTCACTAACTGCGGACTTCAGGCATC
CTACTTAATGCCGCAGAATTGGAGCTTTCCATCTGTAGAATGGATGTAATGCCACCTACCTCATGGTGCTGCTGTGAGAA
TCAAGTTAGCTTTTGAATCATTTAATACATACAGTTCCAGGCATATCCTAAGTGTTCAAAGAGTGGTATTTTGAAGATCA
TTTGTGGCTGTGTTTACCTTTTTAGCTGAGAACTCCTCTTCATCTTAATACATACTCTATAGAAACTCTTTTAGGAATTT
```

TAAAATAATATCATGTCATTATTTTTGCATGATATATAAAGGTATGATTTTAAATATTTGGAAAAATGTATTAGATTGAT
ATACTTTTAATAATTGGTTAGCAAAGTTCATTTCAATAAGATGTTTAATAGAGTAATATTCATTAGATACCTCTGAAGAG
ACAAATTCCAACTTTTTCAGTATACCAAGCTTTAAAGGAAGATAATACATTTTATTTATTCATTTATCAGTTAATAGATA
TTTGGGTTGTTTCTGATTTGGGGCTATTATGAATAATGTTGCTATGAACATTCACATACAAGTTTCTGTGTGGATGTATG
TTTTCTTTTCTCTTGGGTATATACCTAGGAGTAGAATTGCTGGGTCACATGGTAACTCTACGTTTAACTCTTTGAAGAAC
TGCCAGACTGTCTCCCAAAGTGGCTGCGTCATTTCACACTTCCACCAGCAGTGTATGAAGATTCTGAGTTCTCTACATCT
TTGTCAATACTTATTACCTTTTTTATTATAGTCATCCTAGTGTGTGTGTCGTTGGTATCTTATT

HUMAN mRNA SEQUENCE : hR7-046.1 (Seq ID No: 73)
ATGGCCAAACCTTATGAATTTAACTGGCAGAAGGAAGTTCCCTCCTTTTTGCAAGAAGGAACAGTTTTTGACAGATACGA
GGAGGAATCCTTTGTGTTTGAACCCAACTGCCTCTTCAAAGTGGATGAGTTTGGCTTCTTTCTGACATGGAGAAGTGAAG
GCAAGGAAGGACAGGTGCTAGAATGCTCCCTCATCAACAGTATTCGGTCGGGAGCCATACCAAAGGATCCCAAAATCTTG
GCTGCTCTTGAAGCTGTTGGAAAATCAGAAAATGATCTGGAAGGGCGGATAGTTTGTGTCTGCAGTGGCACAGATCTAGT
GAACATTAGTTTTACCTACATGGTGGCTGAAAATCCAGAAGTAACTAAGCAATGGGTAGAAGGCCTGAGATCAATCATAC
ACAACTTCAGGGCCAACAACGTCAGTCCAATGACATGCCTCAAGAAACAAGAAAACATAATGAACAATAACTGGAATGTG
TGTTTCTTTCTTTTCTGCCCTAGTATTACTAGAACATTTGCATCGGGAAAAACAGAAAAGGTGATCTTTCAAGCACTCAA
GGAGTTAGGTCTTCCCAGTGGAAAGAATGATGAAATTGAGCCCACAGCATTTTCTTATGAAAAGTTCTATGAACTGACAC
AAAAGATTTGTCCTCGGACAGATATAGAAGATCTTTTCAAAAAAATCAATGGAGACAAAACTGATTATTTAACGGTAGAC
CAATTAGTGAGCTTTCTAAATGAACATCAACGAGATCCTCGATTGAATGAAATTTTATTTCCATTTTATGATGCCAAAAG
GGCAATGCAGATCATTGAGATGTATGAACCTGATGAAGATTTGAAGAAAAAAGGCCTTATATCAAGTGATGGGTTTTGCA
GATATCTGATGTCAGATGAAAACGCCCCAGTCTTCCTAGATCGTTTAGAACTTTACCAAGAAATGGACCATCCTCTGGCT
CACTACTTCATCAGTTCTTCCCATAACACTTATCTCACTGGCAGACAGTTCGGCGGGAAGTCTTCGGTAGAAATGTACAG
ACAGGTTCTCCTGGCTGGTTGCAGATGTGTTGAACTTGACTGCTGGGATGGAAAAGGTGAAGACCAAGAACCAATAATAA
CTCATGGAAAAGCAATGTGTACAGATATCCTTTTTAAGGATGTAATTCAAGCCATCAAGGAAACTGCATTTGTCACATCA
GAATATCCTGTAATTCTCTCCTTTGAAAATCACTGCAGCAAATATCAACAGTACAAGATGTCCAAATATTGCGAAGATCT
ATTTGGGGATCTCCTGTTGAAACAAGCACTTGAATCACATCCACTTGAACCAGGCAGGGCTTTGCCATCCCCCAATGACC
TCAAAAGAAAAATACTCATAAAAAACAAGCGGCTGAAACCTGAAGTTGAAAAAAAACAGCTGGAAGCTTTGAGAAGCATG
ATGGAAGCTGGAGAATCTGCCTCCCCAGCAAACATCTTAGAGGACGATAATGAAGAGGAGATCGAAAGTGCTGACCAAGA
GGAGGAAGCTCACCCCGAATTCAAATTTGGAAATGAACTTTCTGCTGATGACTTGGGTCACAAGGAAGCTGTTGCAAATA
GCGTCAAGAAGGGCCTGGTCACTGTAGAAGATGAGCAGGCGTGGATGGCATCTTATAAATATGTAGGTGCTACCACTAAT
ATCCATCCATATTTGTCCACAATGATCAACTACGCCCAGCCTGTAAAGTTTCAAGGTTTCCATGTGGCAGAAGAACGCAA
TATTCATTATAACATGTCTTCTTTTAATGAATCAGTCGGTCTTGGCTACTTGAAGACACATGCAATTGAATTTGTCAATT
ATAACAAACGGCAAATGAGTCGCATTTACCCCAAGGGAGGCCGAGTCGATTCCAGTAATTACATGCCTCAGATTTTCTGG
AACGCTGGCTGCCAGATGGTTTCACTGAACTATCAAACCCCAGATTTAGCGATGCAATTGAATCAGGGAAAATTTGAGTA
TAATGGATCGTGCGGGTACCTTCTCAAACCAGATTTCATGAGGCGGCCTGATCGAACATTTGACCCCTTCTCTGAAACTC
CTGTTGATGGTGTTATTGCAGCCACTTGCTCAGTGCAGGTTATATCAGGTCAATTCTTATCAGATAAGAAAATTGGCACC
TACGTAGAGGTGGATATGTATGGGTTGCCCACTGACACCATACGTAAGGAATTCCGAACTCGCATGGTTATGAATAATGG
ACTCAATCCAGTTTACAATGAAGAGTCATTTGTATTTCGGAAGGTGATCCTGCCGGACCTGGCTGTCTTGAGAATAGCTG
TGTATGATGATAACAACAAGCTGATTGGCCAGAGGATCCTCCCGCTTGATGGCCTCCAAGCCGGATATCGACACATTTCC
CTTCGAAATGAGGGAAATAAACCATTATCACTACCAACAATTTTCTGCAATATTGTTCTTAAAACATATGTGCCTGATGG
ATTTGGAGATATCGTGGATGCTTTATCAGATCCAAAGAAATTTCTCTCAATTACAGAAAAGAGAGCAGACCAAATGAGAG
CTATGGGCATTGAAACTAGTGACATAGCCGACGTGCCCAGTGACACTTCCAAAAATGACAAGAAAGGAAAGGCCAACACC
GCCAAAGCAAATGTGACCCCTCAGAGTAGCTCTGAGCTCAGACCAACCACCACGGCTGCCCTGGCCTCTGGTGTGGAAGC
CAAGAAAGGTATTGAACTTATCCCTCAAGTAAGGATAGAAGACTTAAAGCAGATGAAGGCTTACTTGAAGCATTTAAAGA
AACAGCAGAAGGAGCTAAATTCTTTAAAGAAGAAACATGCAAAGGAACACAGTACCATGCAGAAGTTACACTGCACGCAA
GTTGACAAAATTGTGGCACAGTATGACAAAGAGAAGTCGACTCATGAGAAAATCCTAGAGAAGGCAATGAAGAAGAAGGG
GGGAAGTAATTGTCTCGAAATGAAAAAAGAAACAGAAATCAAAATTCAGACGCTGACATCAGATCACAAATCTAAGGTCA
AAGAGATTGTAGCACAGCACACAAAGGAATGGTCAGAAATGATCAATACCCACAGTGCTGAGGAGCAAGAAATCCGAGAC
CTGCACCTCAGCCAGCAGTGTGAGCTGCTGAAAAAGCTACTCATCAATGCCCACGAGCAGCAAACCCAGCAGCTGAAACT
GTCCCATGACAGGGAAAGCAAGGAAATGCGAGCACACCAGGCTAAGATTTCTATGGAAAATAGCAAAGCCATCAGCCAAG
ATAAATCTATCAAGAATAAAGCAGAACGGGAAAGGCGAGTCAGGGAGTTAAACAGCAGCAACACTAAAAAGTTTCTGGAA
GAAAGAAAGAGACTTGCCATGAAGCAGTCCAAAGAAATGGATCAGTTGAAAAAAGTCCAGCTTGAACATCTAGAATTCCT
AGAGAAACAGAATGAGCAGGCGAAGGAGATGCAGCAGATGGTGAAATTGGAAGCCGAGATGGACCGCAGACCAGCAACAG
TAGTATGAAACTCCAAAATGCAAACTGAAGCAGCAAACCCACAAAGCATCAAAAGACTCACTCACAAACTTCTGAACACA
AACTCCATGGATGAAAGCTGTTTATTTTGTTTCCTTTATGTGTAAACAAGATGATATCTGAAACCAGAGAGACTTGGAAT
GTCTGACTGACTTCTATTTAACAGCTTTGAGTATTGCATTTCCTTGGCCAAACAAAAATAGCTACAAATCCACAAAAATTT
ACTATTCCAGTAAGGCAGAGTCCAACCATTGATAATACAACTTAAACATGTTTGCTATAAAATACCATCACAAGTAAATG
AGCTTGGTGTGAACAACTCTCCTTTGTGATGCCTTAGGACATGTTTGAACTGCAGCAAAAAACAAAAACAAAAAACAGTG
CATTAGCAATTTCATAGCA

HUMAN PROTEIN SEQUENCE : hP7-046.1 (Seq ID No: 74)

EP 2 058 408 A2

MAKPYEFNWQKEVPSFLQEGTVFDRYEEESFVFEPNCLFKVDEFGFFLTWRSEGKEGQVLECSLINSIRSGAIPKDPKIL
AALEAVGKSENDLEGRIVCVCSGTDLVNISFTYMVAENPEVTKQWVEGLRSIIHNFRANNVSPMTCLKKQENIMNNNWNV
CFFLFCPSITRTFASGKTEKVIFQALKELGLPSGKNDEIEPTAFSYEKFYELTQKICPRTDIEDLFKKINGDKTDYLTVD
QLVSFLNEHQRDPRLNEILFPFYDAKRAMQIIEMYEPDEDLKKKGLISSDGFCRYLMSDENAPVFLDRLELYQEMDHPLA
HYFISSSHNTYLTGRQFGGKSSVEMYRQVLLAGCRCVELDCWDGKGEDQEPIITHGKAMCTDILFKDVIQAIKETAFVTS
EYPVILSFENHCSKYQQYKMSKYCEDLFGDLLLKQALESHPLEPGRALPSPNDLKRKILIKNKRLKPEVEKKQLEALRSM
MEAGESASPANILEDDNEEEIESADQEEEAHPEFKFGNELSADDLGHKEAVANSVKKGLVTVEDEQAWMASYKYVGATTN
IHPYLSTMINYAQPVKFQGFHVAEERNIHYNMSSFNESVGLGYLKTHAIEFVNYNKRQMSRIYPKGGRVDSSNYMPQIFW
NAGCQMVSLNYQTPDLAMQLNQGKFEYNGSCGYLLKPDFMRRPDRTFDPFSETPVDGVIAATCSVQVISGQFLSDKKIGT
YVEVDMYGLPTDTIRKEFRTRMVMNNGLNPVYNEESFVFRKVILPDLAVLRIAVYDDNNKLIGQRILPLDGLQAGYRHIS
LRNEGNKPLSLPTIFCNIVLKTYVPDGFGDIVDALSDPKKFLSITEKRADQMRAMGIETSDIADVPSDTSKNDKKGKANT
AKANVTPQSSSELRPTTTAALASGVEAKKGIELIPQVRIEDLKQMKAYLKHLKKQQKELNSLKKKHAKEHSTMQKLHCTQ
VDKIVAQYDKEKSTHEKILEKAMKKKGGSNCLEMKKETEIKIQTLTSDHKSKVKEIVAQHTKEWSEMINTHSAEEQEIRD
LHLSQQCELLKKLLINAHEQQTQQLKLSHDRESKEMRAHQAKISMENSKAISQDKSIKNKAERERRVRELNSSNTKKFLE
ERKRLAMKQSKEMDQLKKVQLEHLEFLEKQNEQAKEMQQMVKLEAEMDRRPATVV*


HUMAN mRNA SEQUENCE : hR7-046.2 (Seq ID No: 75)
GTGTGTCCCTCCAGTGCCGCTTGCCCCTTGTTCTCCCAAGCACCAGGGGGACCCCTGGTCTTAGATTCAAGTTGCATCAC
TAGACTAACCAGCTCTGGGAACCAGAGGGACACTGGAGGAACTAGTTTGGGAAATGAAAAACATCTGCAATGAGAGGTAA
TTTTATTCTCCTCTTCCTTTTACCCTAGTCTCACTCTCCATAGTAGGACCTGGATCTTTGCATCAGAGAAAACATAATGA
ACAATAACTGGAATGTGTGTTTCTTTCTTTTCTGCCCTAGTATTACTAGAACATTTGCATCGGGAAAAACAGAAAAGGTG
ATCTTTCAAGCACTCAAGGAGTTAGGTCTTCCCAGTGGAAAGAATGATGAAATTGAGCCCACAGCATTTTCTTATGAAAA
GTTCTATGAACTGACACAAAAGATTTGTCCTCGGACAGATATAGAAGATCTTTTCAAAAAAATCAATGGAGACAAAACTG
ATTATTTAACGGTAGACCAATTAGTGAGCTTTCTAAATGAACATCAACGAGATCCTCGATTGAATGAAATTTTATTTCCA
TTTTATGATGCCAAAAGGGCAATGCAGATCATTGAGATGTATGAACCTGATGAAGATTTGAAGAAAAAAGGCCTTATATC
AAGTGATGGGTTTTGCAGATATCTGATGTCAGATGAAAACGCCCCAGTCTTCCTAGATCGTTTAGAACTTTACCAAGAAA
TGGACCATCCTCTGGCTCACTACTTCATCAGTTCTTCCCATAACACTTATCTCACTGGCAGACAGTTCGGCGGGAAGTCT
TCGGTAGAAATGTACAGACAGGTTCTCCTGGCTGGTTGCAGATGTGTTGAACTTGACTGCTGGGATGGAAAAGGTGAAGA
CCAAGAACCAATAATAACTCATGGAAAAGCAATGTGTACAGATATCCTTTTTAAGGATGTAATTCAAGCCATCAAGGAAA
CTGCATTTGTCACATCAGAATATCCTGTAATTCTCTCCTTTGAAAATCACTGCAGCAAATATCAACAGTACAAGATGTCC
AAATATTGCGAAGATCTATTTGGGGATCTCCTGTTGAAACAAGCACTTGAATCACATCCACTTGAACCAGGCAGGGCTTT
GCCATCCCCCAATGACCTCAAAAGAAAAATACTCATAAAAAACAAGCGGCTGAAACCTGAAGTTGAAAAAAAACAGCTGG
AAGCTTTGAGAAGCATGATGGAAGCTGGAGAATCTGCCTCCCCAGCAAACATCTTAGAGGACGATAATGAAGAGGAGATC
GAAAGTGCTGACCAAGAGGAGGAAGCTCACCCCGAATTCAAATTTGGAAATGAACTTTCTGCTGATGACTTGGGTCACAA
GGAAGCTGTTGCAAATAGCGTCAAGAAGGGCCTGGTCACTGTAGAAGATGAGCAGGCGTGGATGGCATCTTATAAATATG
TAGGTGCTACCACTAATATCCATCCATATTTGTCCACAATGATCAACTACGCCCAGCCTGTAAAGTTTCAAGGTTTCCAT
GTGGCAGAAGAACGCAATATTCATTATAACATGTCTTCTTTTAATGAATCAGTCGGTCTTGGCTACTTGAAGACACATGC
AATTGAATTTGTCAATTATAACAAACGGCAAATGAGTCGCATTTACCCCAAGGGAGGCCGAGTCGATTCCAGTAATTACA
TGCCTCAGATTTTCTGGAACGCTGGCTGCCAGATGGTTTCACTGAACTATCAAACCCCAGATTTAGCGATGCAATTGAAT
CAGGGAAAATTTGAGTATAATGGATCGTGCGGGTACCTTCTCAAACCAGATTTCATGAGGCGGCCTGATCGAACATTTGA
CCCCTTCTCTGAAACTCCTGTTGATGGTGTTATTGCAGCCACTTGCTCAGTGCAGGTTATATCAGGTCAATTCTTATCAG
ATAAGAAAATTGGCACCTACGTAGAGGTGGATATGTATGGGTTGCCCACTGACACCATACGTAAGGAATTCCGAACTCGC
ATGGTTATGAATAATGGACTCAATCCAGTTTACAATGAAGAGTCATTTGTATTTCGGAAGGTGATCCTGCCGGACCTGGC
TGTCTTGAGAATAGCTGTGTATGATGATAACAACAAGCTGATTGGCCAGAGGATCCTCCCGCTTGATGGCCTCCAAGCCG
GATATCGACACATTTCCCTTCGAAATGAGGGAAATAAACCATTATCACTACCAACAATTTTCTGCAATATTGTTCTTAAA
ACATATGTGCCTGATGGATTTGGAGATATCGTGGATGCTTTATCAGATCCAAAGAAATTTCTCTCAATTACAGAAAAGAG
AGCAGACCAAATGAGAGCTATGGGCATTGAAACTAGTGACATAGCCGACGTGCCCAGTGACACTTCCAAAAATGACAAGA
AAGGAAAGGCCAACACCGCCAAAGCAAATGTGACCCCTCAGAGTAGCTCTGAGCTCAGACCAACCACCACGGCTGCCCTG
GCCTCTGGTGTGGAAGCCAAGAAAGGTATTGAACTTATCCCTCAAGTAAGGATAGAAGACTTAAAGCAGATGAAGGCTTA
CTTGAAGCATTTAAAGAAACAGCAGAAGGAGCTAAATTCTTTAAAGAAGAAACATGCAAAGGAACACAGTACCATGCAGA
AGTTACACTGCACGCAAGTTGACAAAATTGTGGCACAGTATGACAAAGAGAAGTCGACTCATGAGAAAATCCTAGAGAAG
GCAATGAAGAAGAAGGGGGGAAGTAATTGTCTCGAAATGAAAAAAGAAACAGAAATCAAAATTCAGACGCTGACATCAGA
TCACAAATCTAAGGTCAAAGAGATTGTAGCACAGCACACAAAGGAATGGTCAGAAATGATCAATACCCACAGTGCTGAGG
AGCAAGAAATCCGAGACCTGCACCTCAGCCAGCAGTGTGAGCTGCTGAAAAAGCTACTCATCAATGCCCACGAGCAGCAA
ACCCAGCAGCTGAAACTGTCCCATGACAGGGAAAGCAAGGAAATGCGAGCACACCAGGCTAAGATTTCTATGGAAAATAG
CAAAGCCATCAGCCAAGATAAATCTATCAAGAATAAAGCAGAACGGGAAAGGCGAGTCAGGGAGTTAAACAGCAGCAACA
CTAAAAAGTTTCTGGAAGAAAGAAAGAGACTTGCCATGAAGCAGTCCAAAGAAATGGATCAGTTGAAAAAAGTCCAGCTT
GAACATCTAGAATTCCTAGAGAAACAGAATGAGCAGGCGAAGGAGATGCAGCAGATGGTGAAATTGGAAGCCGAGATGGA
CCGCAGACCAGCAACAGTAGTATGAAACTCCAAAATGCAAACTGAAGCAGCAAACCCACAAAGCATCAAAAGACTCACTC

308

```
ACAAACTTCTGAACACAAACTCCATGGATGAAAGCTGTTTATTTTGTTTCCTTTATGTGTAAACAAGATGATATCTGAAA
CCAGAGAGACTTGGAATGTCTGACTGACTTCTATTTAACAGCTTGAGTATTGCATTTCCTTGGCCAAACAAAAATAGCTA
CAAATCCACAAAAATTTACTATTCCAGTAAGGCAGAGTCCAACCATTGATAATACAACTTAAACATGTTTGCTATAAAAT
ACCATCACAAGTAAATGAGCTTGGTGTGAACAACTCTCCTTTGTGATGCCTTAGGACATGTTTGAACTGCAGCAAAAAAC
AAAAACAAAAAACAGTGCATTAGCAATTTCATAGCA
```

HUMAN PROTEIN SEQUENCE : hP7-046.2 (Seq ID No: 76)
```
MNNNWNVCFFLFCPSITRTFASGKTEKVIFQALKELGLPSGKNDEIEPTAFSYEKFYELTQKICPRTDIEDLFKKINGDK
TDYLTVDQLVSFLNEHQRDPRLNEILFPFYDAKRAMQIIEMYEPDEDLKKKGLISSDGFCRYLMSDENAPVFLDRLELYQ
EMDHPLAHYFISSSHNTYLTGRQFGGKSSVEMYRQVLLAGCRCVELDCWDGKGEDQEPIITHGKAMCTDILFKDVIQAIK
ETAFVTSEYPVILSFENHCSKYQQYKMSKYCEDLFGDLLLKQALESHPLEPGRALPSPNDLKRKILIKNKRLKPEVEKKQ
LEALRSMMEAGESASPANILEDDNEEEIESADQEEEAHPEFKFGNELSADDLGHKEAVANSVKKGLVTVEDEQAWMASYK
YVGATTNIHPYLSTMINYAQPVKFQGFHVAEERNIHYNMSSFNESVGLGYLKTHAIEFVNYNKRQMSRIYPKGGRVDSSN
YMPQIFWNAGCQMVSLNYQTPDLAMQLNQGKFEYNGSCGYLLKPDFMRRPDRTFDPFSETPVDGVIAATCSVQVISGQFL
SDKKIGTYVEVDMYGLPTDTIRKEFRTRMVMNNGLNPVYNEESFVFRKVILPDLAVLRIAVYDDNNKLIGQRILPLDGLQ
AGYRHISLRNEGNKPLSLPTIFCNIVLKTYVPDGFGDIVDALSDPKKFLSITEKRADQMRAMGIETSDIADVPSDTSKND
KKGKANTAKANVTPQSSSELRPTTTAALASGVEAKKGIELIPQVRIEDLKQMKAYLKHLKKQQKELNSLKKKHAKEHSTM
QKLHCTQVDKIVAQYDKEKSTHEKILEKAMKKKGGSNCLEMKKETEIKIQTLTSDHKSKVKEIVAQHTKEWSEMINTHSA
EEQEIRDLHLSQQCELLKKLLINAHEQQTQQLKLSHDRESKEMRAHQAKISMENSKAISQDKSIKNKAERERRVRELNSS
NTKKFLEERKRLAMKQSKEMDQLKKVQLEHLEFLEKQNEQAKEMQQMVKLEAEMDRRPATVV*
```

Table 13

MOUSE GENOMIC SEQUENCE : mD7-104 (Seq ID No: 77)
```
TTACCACACTCGCGTAAAATCCTGAGACATATTTGCCCTCCCAGTGATGTGCAGTACTGAGCCCTCCATTGCGGATCAGC
AGTGGCTGTCCCAGGGTCCAGTAATGCTGTCTCATTGCAGTTTTGCTGTGTCAGATGACTGCAGGAGCCTGTGATGTGCC
CTCAGGCATCCTGGGGGACCCCCTCACCCGGCACCTCCTGCACAGAAAGCAGCCAGGGCAGTGACTGCTGTCAGTGGAAT
GTGACACAGGGACACAGGGGGTATAAGGGCTCAGTGAGTCATCACATCTGGATCCTTTGGGACAGGGTCTGGCTTGTTAA
GTGTAAATGACTGAAGCCTTAATCCCAGCACGCAGGGGACAGAGAGGTAGGCAGATCTCTCTGAGTCTGAGGGCTGTCTG
GTGTACAAAGTGAGCTCCAAGACAGGCCAAGGCTGTGTAAAGAGACACTGTCTCAGAATTCAAAACAGCAGCAGCAGCAA
CAACAACAATAAAAGTGATTGTCTTCATTTCTTGGTCCTGTGATAAAAAAAAGACCCTGTTAAAAGCACCTTAAGGAAAA
AAAAGGGGTCAGGCATGGTGGCGCATGCCTTTAATCCCAGCACTCGGGAGGCAGAGGCAGGCAGATTTCTGAGTTCGAGG
CCAGCCTGGTCTACAAAGTGAGTTCCAGGACAGCCAGGGCTATACAGAGAAAGCCTGTCTAGAAAAAACAAACAAACAAA
CAAACAAAAAAACCCAAACAAAAAAAAAGTAAAAGGGCTGGAGAGATGGCTCAGCGGTTAAGAACACCTGACTGCTC
CTCCAGAGATCCTGAGTTCAATTCCCAGCAACCGCATGATGGCTCACAACCTTAAGAAAAAGAAGATTTGTGAGTCTGAG
GGTGTTAGTCCATCAGGTGGAGAAAGTCCTGACTCTGGCCAAGAGTCAAGAAGCAGAGAAGCAGAGACCAATGACTTCT
GGCATACAACTTTTTCTCTCTGACAGTCCACCACCGAAGCAATGGCGTAGCCCACAGTTGGGGGAGGACATTCTACCTCC
ATTAACCCAATCAAGATAATCTCTTATACACATGGTCAGAGGCTAACCCATTCTAGATAACCCTTCCCAGGATATAGCTA
TGATTCAATAGCTTAGTCCAGGTGGCCAGGACCCAGTTAGGTCCCCCAAATGACCAGGTGTGTTTTGGACCAGGGAGTTG
GCATTCCCTCTTATCCCATAGGGCCAAACTACTTCCCTGCCCACTCACCATAGCTTTCAGTGTTGGCTATAAAATAGTTC
CCAAGTTAAAAATTGGCTTCATGGAGGCCTCCAGATCCTCACTGGGAGGAGCCTGCCTATATCCCACCCCTTTGCAGCTT
CACACCTGCACCCCCATGCCTGCACCCGCAGCTATTCCCTCCTTGGGCTAAGAGTAAGATGAGAACACCTCTTCTCCCTC
CGATCCCTTCAACCCAAATGCTGTATTGACCTGCCTCTGAGAGGGGAAGTAGATAGCGTCCCCACAGCTCGTGACTGTCA
TCCCACTCTTAGGGTGGCAGTGGGGAGGATCTTTAGTGGAGGCTAGCCTGGGCTACAGAGTGAGATTCTTCCTCTCTCAA
AACAAAAACACCCAGACCAAACCAAACCAACCAGCCAGCTGGTGGCCTGAGGGGCCATGGAGGGACTCCTGGTAGAGGAG
GGGGACAGTGGGCATGTGTCTCCTTCTCCACAGTTTTGGAACACCCAGGTCACAATAACGATTACTCCTGAGTGAGTGCA
TCTCTTGGGACAATGTAGTATTCATTTCTTCCACATCCAATTAGTTGAAACAATGGTCACACTGTCTGTCTGAGAATGAA
GAGCAACACTGGCTGTTGTCTCATCTAGTGACCCAGAAAATGCCACAGAGTGTTGGGAGGCAGAGCCCACTGTCCCTCTG
TGTCCCCTCCGCTCTCCCAGGCTATGTCCACCCTGAAGCTGGAAGGCCTGGAGGCTGCAGGGTACGTGATGGACAGGCTT
GGTGGTCCAACAGTATCATCTACTCAACATGTTCTAGAATTCTCCCCTTGTCTTTAGCGGCCTCTGTGATAAAGTCAATA
GACCTTCCTTCAAGTGAACCAGACTACGGGCTAGAGAGGTGGTTTAGCAGTTGACAGCACTGGTTGCTCTCGCAGAGGAC
CCAGGTTCAAAGCCCAGCACCCACATAGTGGCTCATGACTGTCTATAACTCCAAAGGATCCTAGCCCTCTTCTGACCTCT
GTGGGCACTTCACATATGTAGCACACAGATATACATGGAAACACATACACACATGAGTACAAATTGTAAAATTTTTGAGA
GAAAGAGACTGGGGGGCGCGTGCGAGAGAGAGAGAGAGAGAGAGAGAGAGAACAAGGAACTAAGTCATTGTCCAGTAC
TATCCTTCTGGCCAGGTACAGTTGAGATTCCCTACCTCTTAGTGTCTTTCCAGGTAACCAGGCATGGTTTTGTAATGGCC
TACATGACCTAGGGCCACTTGATCTTACATTCCTTCTCAAGCAGAGGAAGGCTTAACTCAGAACAGACGTCAGCAATGCT
CACACTCATCCTACAGACAGAAATGCAGAGCCGCTCTTACAGATAGCACAGGGACCCTGAGATAACCGCGGCTAGAAAGA
GGGTTTCAGGCTGCCTCACATGACATAATCCATGGGAGAGGGTTACATAAACTTTTTATTTGTAGCTGTTTGGGTGTGCA
CCATGCAGGTGCAGTTCTGGAAGAGGCCAGACGGGGGCGCCAGGTCCCCTGAACTGGATTTACAGATGGTTATGAACTGC
CTGGTGTAGGGTGTGGGAATATGAACAAGGCGGCCCTCTGCAAGAACAGTAAGTGCTTTTTACCACTGGGCAGAATTCTA
TTTTGTTTTGCCATTCCTTCATCTGTTGATGAACAATGAGGTTGACGCAAATCTTTGCTATAATGAAGGAAGGGCAGCTA
```

```
AGTAGTGTCTTCTTGTCTCCATCTTGGTGGGAAGCCAATCTCATGGTGACTGTGTAGCTCAGCATCCCATGGGGACTCCC
TCGAACAGGCACCTTCGTCCGAGACAACTCATCTCTGTACTGGCCTCACCCAGGAGGAAAAAGATAGCACAGGTCCTGAA
ACCAGGGCAAAGGTGAAGTCTAGCTGGCAGTCCCAGGAAGAGGGAAGTCCCTGAGAGAGGAACACAGGGGACATACGGAG
GCAGCTCTCTGTGATTTGCACTGTAACAGAGTGCTAGAAGTATTTGTTTGGTATTGGTTGTTCGTGAGATTGGGGGGGGG
GGGTCTCACGATTTAGCCTTGGCTGGAATTCATTAGGTAGACCAGGATGCCCTTGAACTTAGTGCTGGGATTAAAGGTGT
GTGTTACTACATTCTGCTTAATACTATGTTTCTTTGTTAGTTTTTGTTGGGGGGGGGGGTTCGAGACAGGGTTTCTCTGT
GTAGCCCTGACTGTCCTGGAACTCACTCTGCAGACCAGGCTGGCCTCGAACTCAGAAATCTACCTGCCTCTGCCTCCCAA
GTGCTGGGATTAAAGGTATGTGCCACCACTGCCCGGTTATATGTTGGGCTTTTAATAGTATGTTTAACCCTGTTTTTCTA
AATATTGTAATTCCCATGTGGAATCAATTTAGGATCTTGGAAACAATTCACATCTTTCTTTCTATCCTTTCTTTCTTTCT
TTCTTTCTTTCTTTCTTCTTTTTTGGTGGGAATTTTGGTTTAATTATAGCTTTTGAAGTAGGAAAATCTGTCTTCT
TTTTTTTTTGGNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNGACAGGGCATCCCTGGGGCTGAACAGCTGAATCAGACTGAGTCCTTGCCAGGTATGTGTCCAGGAGTCACCC
ATTGTGTGCTCAGGGCCCCCTCCCCACAGGCACCTCCGTGTCCCTAACTGTGGATGAGGAATCAGAGGGTTTAACAGCTT
TTCATGGCACAGGAAGAAGGAAGCCAGGGAAGCCAGAAAGCCCATTGGCTTCCTGCTGAGTGGAGATAAATCCAAGGGAA
AAGGAATGGAGGGAAGGAGATGGCCAGGATGTGGGGTGCCTGACATTTGAAAGGATAGACAGGAAACAAGTGGTCATTGA
TATGATGTTTGACTCTCCAGGCTGCTGGGGAGAGAAGCATCCCAGTAGATGGAGGGAGCTGATGTGCAAAGGCCCTGAGG
TAGGAACTGGCAGTTTGAGAGGCAAGAGTGACAGCCTTACCAATTACAGCAAGGACTTGGGTCTTTATTCTGAGGGAGAA
GGAAGCAAAAGAAAGATGGATATATATGTATGTATATGTATATATGTATGTGTGTGTATAATAATTTTATTTTATAT
AACATATATTCTATTTTATAATTTTATTTGCACATAGTTAACTCATGTAAAGTGTAAGACTGAAGGCATGGTGGCACACA
CCTTCAATCCCAGCACTTGGGAAGCAGGAGAAGACAGATCTCTGTGAGTTCGAGACCAGACAGGGCTATACGATGAGTTT
TTTTTTTTTAAGATTTATTTATTATTATACATAAGTACACTGTAGCTGTCTTCAGACACACCAGGGTGTCAGATCTCATTG
CGGGTGGTTGTGAGCCACCATGTGGTTGCTGGGATTTGAACTCAAGACCTTCAGAAGAGCAGTCAGTGCTCTTACCCATT
GAGCCATCTCGTCAGCTCCAAGATTTATTTTATTTTATTTATTTATTTTTATTTATTTGGTTTTTCGAGACAGGGTTTC
TCTGTGTAGCCCTGGCTGTCCTGGAACTCACTCTGTAGACCAGGCTGGCCTCGAACTCAGAAATTTGCCTGCCTCTGCCT
CCCGAGTGCTGGGATTAAAGGCGTGCGCCACCACGGCCCAGCTGAGATTTTGCTTTTTAAAGGTAAAATATAAATTAACT
GTAAGATGCAGTGGCTTTTGGCCCAGCGACAGAGTAGGAAGAAGAGTAACAGAGCTCAGCCCTCAGCAGTTTCTGCCCCT
TCCTCAGCCCTGGAAACCATTCAGTGTCTTTCTGTTTGGGTTCAGCAACATGATTGTTTTGTGCATGCTTGGATGCTCTT
GTGTCTGGCTTCTCTCCCTTACAGGTATCTTCTGGATTCATCCATGTGCATCAGAGACTTGGTATCCCTTCTTCCTCACA
GACCACGTTCAACACACCCATTTGGCTGCTAGATGCCTTAACTGCTGTGGAACACAAGAAAGGAATCCTGTGCACAGGAG
TTGCTGTGGATGTGTTTTCTCTCTCTCTCTCTCTCTCTCTCTCTCTCTCTCTCTCTCTGGGTACAGACCTGGAAACGGAGCA
GGGGGCTCATAGGGCCCATAGGTCTAGTGCCAGAGGGTGCCCACGAATGTATACATCCTGTTTTTTGGTGTCCTCTGCAG
GATTCCTTGGTCTCTTCCTCTCTTCCTCTGCACCGGAGATGGATCCAGGGCCATTTACAGGTTACTAGGCAAATGCATTT
TTGATGAGCCACCCTGTCCTCTTATTACCCTTAGAAATGGGCTCTCACTGAGTTGCTAGCCTTGACTCATTCTGGACCC
TAGGCTGGCTTTAATCTTAAGCTCCTTTTGCCTCAACCTCCCAAATAGCTGGATTGGCATGCCTGGCTCCTAGCTGTGTT
TTGATTGTGGAAAGTTCTGTACTGAAGGCCTCCTCTGACAGGGTCTTCTTTTTGTACATGACTAACACTCTCTCTCTC
TCTCTCTCTCTCTCTCTCTCTCTCTCTCTCTCTCTCACCTTGCGCTGGTTAGTTTTGTCAATTTGATACAAACTAG
AGTCACACAGGAAGAGGGATCTGTTCCATCAGATTAGCCTGAGGGCAATAGTGTACTTTTTCTGGATTTATGATTGATGG
TGGAAGAGTCCAGCTTCCTGTGGGTGGGACCACTCCTGGTCACCGGTCGGTCCTGAGATGTATAACAAAGTAAACTGAG
CAAGCCGTGAGGAGCAAGCCAGGAAGCAGTATCTATCCATGGCTTCTGCTTTAGTTCCTGCCTCCAGGTTCCTGCTCTGA
GTTCCTGCGTTGGCTTCCCCTAATGACAGACTTTCACTCATAAGCTGAAAGAAAACGACTCTTCCCCAAGTTGCCTTTGG
TCAGTGTTTTATCACAGCAAGAGAAAGCAAACCAGGACAGACTTTTCCTGGTGGACTTCCCCTACAGGCCTTCTCTGCCG
CCCGAGCAGAATTGATCGACCTTCCTCCCATCTCCGAGACCATCCTGTGTTGTCTCCCTAGCTCCCCCCATCAAGGCCAT
GGCTCTGTCATATGGGCTGTCTGTGTGTCATCCAATGACAGTGTAGCCTGTCCCCAAAGCCTGGCCCTGGCCTTGGGCTC
TCTGAGGTAAGCATCACGTAGGTTTGGACCTTGAAGACTTGGCAACAATTTGGACTGCGTGAGTTGATCAAGGCAAGTGG
GTATCAAGGAAAGGGGGGTGGCAGACTTGAGCTTTCAGCTGACTGGCAAACTCCCAGGGCCACTGGCAGCATCAGCGGGA
GAAAACAAGCCAGGACTGAAAGATGTTGAAGGCCTCAAGGAAATTTTATTTTTAAAAAGTTGATTTTTTGTTTTGTTTTG
TTTGTTTTGTTTTGTTTTTCAAGAGACAGGGTTTCTCTGTGTAGCTCTGGCTGTCCTGGAACTCACTCTGTAGACCAGGC
TGGCCTCAAACTCAGAAATCTGCCTGCCTCTGCCTCCCAAGTGCTGGGACTAAAGGCGTGTGCCACCACTGCCCGACTTG
TTTTATTTCTTAAACAAGGTTTCACTATGTAGCCCTGGCTAAGCAGACCAGAAATCTACCTCGTACTCCCAGAAATCCAC
CTGCCTCTGCCTCCTTAGTGGTAGCATTAAGAGTGTACATTGCCATGCCTGGCTCCATTATTTATTTTGTGTGTTTGAAC
CTTGGAGGTCAGAGGACAACTTGAGGGAGTGAGTTCTAGGGGCCAGGGATATATTTGATACTCAAAAAGAACCAGCTTCTC
CGATGGAGAAGTAGCTCACGGATTAAGAGCCCTGGCTGCTCTCCCACAGGGTCTGAATCTGCTTCCTGGCACCCACACGG
TGGCTCACAACTGCCTGTAACTTCGGTTTCAGGAGAACTGCCGCTTTCTCCTGGCTTTGTGAAACTGCACAAATACACAA
AAATTTTTAAAAGATCATAAAAAAAAACCTCTCTCTTTAGAGAGATAAATAAAACCATTTCAACTGAACAGACCACTTACT
TTCTTTTCTTTTGATTTGAATTTTATTGACTATTATTATTGAGTGTGGGGTTACCATGGCAACCGGATCTGTTCTCGCCA
TCCCTCATGGGTTCTTGGATTCCCGATTCAGGTCTTCAGGCTTGTGTGACAAGTGTCTTTCCTTGCTGAGCCACCTCACC
AGCCCTTTTCTTTGGCTTGAGATAGGAGTTCATGTAGCCTTGATTGGCCTTGAACTTCTGTTTCTTTGACCTCTTCCTTC
CAAGTGCTGGGATCACAGGGGTGCTCGCCATGCCTGTAAAGACCCCCATTTCCAACAAATTGGACACGAAGGCCTTCTTT
GATGAGTAGGCTTCAGGCTGAAACTTGAGAGAGGAGGAGTCAGGCACTGAGGATCTAGAGAATTCCTTCATAATAATCAT
```

```
GGCTGAGAAAACCCAGTCCCTCTGGTCTGAGATGACATCAGCTCCCTTTGGATAACCTCCTGCAGACCAGATCTCTAGGA
TTTCGCTGCCTCATTCGGCCTCCTCCTAGGTCACATAGAATGGCCCAGTAGTGACATCAGATACCCCGGGGTTAACACTC
CTAAAGAATACGGGCCACTCAGGTTCCCGTTCAAATCTAGAAGGTCTCCTCTTTGGGACCACCTTTAGCAAACATGTCCC
TGAGCACCACGTAGTTTCTGGAGAGACTTTCAGCCCCACTCCCCCCACCTCCCATCTCCATCATCAGGGGCACAGAGTGG
GAGCAGGTTCAGAGGAACCTGGGCTGTGAGGACTCCCACCCCGAGTCCTGAGGACCCTCTTCTGTAAGGTGGAGGTGTGA
ACTGCTCTTCTTAAAGCCAGTTGGTCGCTCATGAGCCTCTGAACTTGGAACATGCGCGGGTGTAACACAGCCAATGTTCC
CAGGCCCCGCCCACCCCTGCTCCTTTGTCTACTGAACTTCTAGGCTCCTTTCTGTCTTTCTCCTGTGTCTACACGTGTGC
ATGTTTGTGGGTGCACCTGAATGTGTTGTGTGAGACAGAATATCTTACTAGGAACCGGGTTAGAGAAGTCTGGCTGGCCA
TCTGGCCCCAGGGATCTTTCTTCCTGTCCCTGACTCCCCTGCACTGGAACTACCAGCCTAGAGTTTGTTTTTGTTTTGGA
CTTACTTATTTTATGTGTGCAATTGTCTTTCCTACATGTCTGTCTGGGTACCATGGGCATGCGTGGTATACTCAGAGGTC
AGAGGAGGGGCATCAGATCCCTTGGAACTGAAGTTAAGGATGGCTGTGAGCTGTCATGTGGGTTCTGGGGACTGAACCCA
GGTCCTCTGCAAGAGCAGCAAGTGCTCTTAACCACTGAGCCATGTCTACCTCTGAGCCATCTCTTCAGGCCTGGGTATCT
CACCTGAACACGGATGCTGGCGACCAATATCAGGTGTCAAGCACTCTACCAATACGGTCCTTTGCACAGCTAAGTCTTGA
TTCCATGTGATGGGTGGAATCTGTCAGGGCTGGAAATATGACTTGGCTGTCTCTGGAGAACCTACTCTGACTCAATATGA
GCTAACTCTGGGTCATATTGCTGAAGGTGGTCTTCAAGAGCAGTCCTACTGGGTTTTAACAGGAGCTGGGACTCGGTTTT
AACAGGTGTGGGGCTGGGTTTTCTCAGCTCCGTATCCACTTTTTGTGTCCTGAGTGGAATGCAGGCCTTGGCCTCACTGA
GGAAGGAAGTCACCCCATGCCTCAGGGTTTTTTTTTTTTTTTTTTTTTTTTTTTTTTTTTTTTGGCTGTAACACGA
AGCATTTAGAATTAAAGGCTTAAAGGCTCTGGCTGTGGTCTGAGAACGAGCTAACTGGGCACTGGGCAGAGCAGGGAAAG
ACAGAGTCCCTACCACCCTGCTGGTATCCTGAGTGGGGTTCATGGCCAAAGAACGCATAACTGAGCTCAACTGAGCTGGT
GGTGTCTCTGGGTGATAAGGAAGAGTCAGCAGAGAGCATAGCCGCCAGGCAGTCTGATAAGCCCCTCGAACACATTCCTG
GATGGAGTCAGGGGCCCAGCGCGTGTCTGGGGCCTGGAGTTTTGAGTGGAGGGTATGCTTAAGGCCTCGAGGGACACAGA
ATTCTGTACGAAGGGTGTGCAAAGGTCCCGGGAGACATGGAATTCCGAGGGGACAGTGTGCAAAGGCACGAGGGACACA
GAATGGAGGGTGTGCAAAAGCCCAAAGGGATATGGAATTCCGAGTGGGGGAAGTGCAAAGGGCCCCGAGGGACACGGAAT
TTTGAGTGGAGGGCGTTCATGGCCCAGAGGCTGGCCCGGGCTGACTGCAACTGATTTAATGCAGGGGAGCGGGAGGCAT
TCGCAGGAGTCCGGAAGAAAGAAGAGGTCGCAGGGGCGGGGTAGTGGATGCAGACGGTGCCAGGGTCTTCTGCCCTCTG
TAGAGGGCACATCGGTTCCCACCTAGACCAGCAACCACCAGGAAAGCCCAGCAGCTCGGAGGGGCGGCGCCCAAAGGAAG
CCACGCCCACGCCTCACCATCAGAGCACCGCCCACTCCCGCCTCTTCCCACCCCTCGCCGGAATCCCGCGCCGAACTCG
GGGGCGGGCTGCCCGGGCCATGGCGCATAAAGCCTCTGGCCACCTGCAGGGCTACTGCTGCTCCGGCCACCGCCAGGCAC
ACACCTTGCTGCTGAGGGAGTCTCGGCTTCTGTCATCTCTGTGGCCTCCGTCACCTCTGTCTCCGTCTCCTTCAGGTCCT
GAGCCCCGAGAGCCCCTTCCGCGCACGCGGACATGGGCGGCAGCTCCAGGGCGCGCTGGGTGGCCTTGGGGTTGGGCGCC
CTGGGGCTGCTGTTTGCTGCGCTCGGCGTTGTCATGATCCTCATGGTGCCCTCCCTCATCAAGCAGCAGGTGCTCAAGGT
GAGTGAAGTGTCTTGGGGAGATGGGGGTTGGGGATCGGGACGCAGGGTGGGACGGTGGAGCTACCTCCTCACCACCAGGG
AGGCCCAGGCTCACCCCAGAGGCTTGGTCTGAGTCACCAGCGTCCCAGGAGCCCTAAACCTCACTGAAGCAGAAGTATGGC
CTGGTTGTCCCTGAGTTTCGACTGTAGCTGTCGCGACCTCCAGCCCTTCTGAACGCGCCGGCCGGTGACTGTACCTAGTA
ACCTCAGAGCTGCGCGAAACCCCTGTACATCTGTGAGGCTCCCACGGGCTCCAGTTCTTTGGGTGCTCTGCCTGTTTCCT
CCCGCCCAGATGCCCCAGAGATGCCCTGGCTCGCCCCCACACTGCCTGCCAGTCTCCGAGAGTTAAGCTGCTTTCTGCCC
CCCACGCTCTGGCAGAGGTAGACACGACTCAGGTCTGCCGAGGATAGGCAGCCCAGCCTCTCCCTTGATCTCAGCCTTAG
GCCCTGTTTTGCCCTTTCAACCGGGCACCGATATTAGGCGGGTGGTGCCGGTCCAGCCACAGACTCTGCCCTGGGATCCG
GTGTCCTTCTATTTGAGCCGGGGAGCTCATTAAGTCCTGGAGATTACCGAGTAATTGTGTTTTCTGAGACGCAGGGTTACT
GCGGAAGGGGAAACGGGGGCTGGCTCTCAGGGAACATCCAGTTAGAACTGGATCTGGGATCCAGAAGGGGCTGTTGTCAC
AGGGTTTGACTGACATCTTTCCTCTTAGCGGGTTTGTTTACTGGAGTTTAAGAATGGCTTCGTGTCCCATGTCCCATCG
CCCGCTGAAAGGAGGGAATGAATGAGGGATACTCCCTCTTGGGCTGGCCGCTACCTGCCAGGTCCTGGCGGGTCTTCAGG
ATGGAGCCACAGGTGGCTTTCCTGGAGTTGTGGTGGGTATTTCCGAGTTCTCTTGGCCACACTGTCATTTCTCTTGCTTA
GAATTTTCAACCCTTGTACATGAAGGTCCCAGGAGTTGCCCTGTCTTGGGTAGAGGGAAGATCCTTCTTGTTGGAGCATC
AGAATTGCTCTGGGCCTTTCTGTTAGGTTTGGCAAAGTAGTGGTGACAGCCTTGTCTCAGTGGCAGCCTGTGGTTTCAGT
AAGAACCCAGCCCTGTGCACTCCGCACGTGGACTGGGTTTCCTGCTCAGCCAGCCTGTAGTGGGTAGAGTGAGCAGATAT
CCCCTGTTCTTCGATTTGGGGGTGAGGGGGAATCTGAGCTGGTTCCTTCTCAAACTCCTTTCGGGAATGTTTTCTTTTGG
GGGTTGGCACAGAGACTAAGGATGGGGGGGGGTTGGTGTGTGTGGGCGTGCACGTTAGCGTTGGGACTGCGCAGGCCTGC
TCCATCAGTCAGGGTCCCATCTGCCCGTGCTTGAGAAAGACCACGACTGGCAGAATTGCCCCATAGCTGGTAGCTTGGGG
CTTCACGTTTGAGCGAGCGGCTTCCACAATCTTACTTGTGAAAATCCCAAAGGCGATTGGTTGGGGGCTGAGTGGCGTGA
AGGAAGGAAGAGCCTAAGACACGCCTAGCCCAAAACAGCCTCTTCTTAGAGGCCCTGGCTGGAAGCCGACCTCTTGAGGG
AGGAAAAAAAAGTTCACTTCAGGGGATGGTGGGAGGATGGCAGCGAGGGGAGCAGGGAGGGACCCTGGAGTTGACTTTGG
ATTCTTGCGTTCTGTGCTACTTGAATGGAAGCCTTGTCTTTGTGTGTGTGTGTGTGTGTGTGTGTTTCTTTCTTTC
CTTTTTTTTTTTTTGTAAGTTAAAGAAGTTGATTTATAAAAGTCAGGAATACTAGGTTGTTTAAGAAAACAAGTTCAGGA
CTTGGGGTGTAGCTCAGTGGTAGAGAACTTACCTTGCATTCAACAGGCCTTGGGTTCCAACACACACACACACACCTGCA
TTCAACGGGGTTCCAACACACACACACACACAGAGAGAGAGAGAGAGAGAGAGAGAGAGATTTCACTCTTATTTTCTTCTGA
TGTATCATTTTTTTGACATAAATTGTGTGTGTGTGTGTGTGTGTGTGTGTGTGTGTGTGTGTGTAGGTCACCTGATTTCTTC
CCTTACAGATAGGGTCTCTTGATGAGCTAGACAGCCATCCCCCTGTCTCTGGGGTTACAGGTGCAATCCTATCGATGCAC
AATATTGTATATGTCACTGGGAGTTTGAACTCAGGTCCTCATGTTTGCCTAGCGTGGACATCTTTTACAAAACCCGAGTT
GGATAGTATGTGGTTTTCTTCTTTTCTTCCTCCTTTCTTTCCTTCCTTTCTTCTTCTTTTTTCTCCTTTCTCTTGATTCG
TGGTCTCACTAGATAGCCCAGGAACTCAGTGCATAGTCCAGGCTGGCCTCAAACTCAGAGATCCACCTACCTTTGCCTCT
```

```
TGAGTGTTAGGATTAAAGGTCTGTCTACCATGCCTGGCTTAAATAGTATGGTTTTTCTGTTCAGTGTTAGTATTCAAAGG
GGTGTGTGTATGCCTGTGTGTGTATGTGTGTATACACGCACACGTGTACACACACATACATGCACGTATACTCATGCACC
TGTATGGACGTGCTTGCCACGCGGTGAGACCAGAGGTTGAAGTGCAGTGTCTTCCACAGTGTCTCTCCACCTTATTTTTT
TGAGACAGGCTCTCTCATTAAATATGAGGTGTGCCTTCTCTGCTAGCCACTGAACAACAGGTCCCTGGAATCTGCCTGTC
TCCGCCTGTCCTGGTACTGGGGTTCCAGACATGTATCAGACGTGCTCAGCTATTTTTTTTTTCTGCGTGTCCCTGGCCATC
CTAGAACTCCCTCTGTAGGCTCTCCCTGCCTCCTGAGTGCTGAGACTAAAGGTATAGGCCACCACCCCTGGCTCTGTGTT
GAGCTTTTGTTTGGGTTTTGAGAATCAGAACTCAGATCCTCCAGCGAGCACTTTACCCACTGGGCGATCTTCCCAGCCCC
TTTCAAAGGTTTTGACAGGCCGAACATGCCAAGTCTGTTACTGCCTTTTCCCCCCTTACTGTGCTGGGATTCCTTTGTTT
ACAGTTCCTCTGTTGACTGGCCATGTCTTTGTCTCAGACTCCAGAGAACACATAGCGTCCTGTGTGAGTCCAGGAACACT
TGCACGGTCTGAGCCTTAGCTGTGGACCCACAGGTGCGCTCTGTTTGGAATTTGGAATTCTCTACTATTGCTCCCGCTGT
CAAGCTGTGGGACCATCCACTTCGATGGCAATGAAGTGTCATTTCTAAACCCCATCACCCTCTTTGGCTACCTGTGGTTT
TGATTTCACATCTTCAGCGCCACAAGGTTGGGCAGGAGGAACGTGATCCCCGTGTAGGTGACAGTTTTCAGAGCTCAGGG
GCTCAGGCCTACTGTACCATGTGCTAGCTGGAGCCGGTTCATCACTCAAGAGAGCCAAGTTTTTCCCCCAGCAGTGAGAC
AGGGGTCAGATCTGGTTTGCTGTCCCTGAGGGATTAGCAAGTTGCAAAGGAGATTACAAGCGGCCTTTGGAAATGCCCTG
TGAGCTTGCCTGCTTCTGGACTCAATCTAACCACGGCAGCGTTGTGAAACAGGCAGAGGCTGCGAATTCTTCCTCTCTAC
AGATAAGGGATCTGGCCCATAGCAAAGGTCGGCAGCTGGTTATTAAACATGGCCTGTGGTGGGCTGGGAGGGACTTCAAG
CGGCCATGCTCGGTTTACCTTCCATAGGGAAGTAGGAAGCAAGCAAGAGCCCTCTCTTTCTGTACAGTGAGCTGCTGGGC
AGTCCAAGAAAAGAATTAAGAAGGGCCAGGTTTCAGGAAGACATGCATGATAGAAAAAAGGCTCTGTTACTATTAAAGTC
CCTCTTCCCCAGCCTCTTACCCGGCATCCCAGCTACTTGGAGTCTGAGGCAGGAGGAATGAATATTCAAAGCCGGAGGAA
GTTCAGAGCCTGCCTGGGTTGCACAGGAAGCTCAGGGGAGCTTAGTGAGACCCCTGTCTTTATATCAAATGTAAGCAAGG
TGCATTAGTGCATATTTTTAAATCCCAGCGCTCAGAAGGCAGAGGCAGGCAGATCTCTGAGTCAAGTCAAGTGCCTGGAT
AGCCAGGGCTATAGAGAGAGATCCTGTCTCATAAAGAAACAAACACACAAATAAATAACACATAAATAAGTAAGTAAATA
AATAAATAGGTAAGTGGAAAACTGAAGATAGAGCTCACTGATTGATAGACTGCTTCCCAGTATGTGCACATCCCTGGGTT
CAATCCCCAGCACTGCAAAACATAAAGAGAAACACACACACACACACACACACACACACACACACACACCAAAATC
CCTCAGCCAGTCGGTGGTCGCAGCGGCGGCTCATACCTTTAATCCCAGCACTCGGGAGGCAGAGACAGAGTGATCTCTGT
GTCAGCCAGGGTTACACAGAGAAACCCTGTCTCAAGACAAAACAAGTCAACCAACCAACCAAACCCAAAATGACAAGAAT
GACATCAAAACCCTAACAGTATTCTATATTTTCAACAGACTCGTGTTAGTGCCAAAATGGTTTAGAAAAAGGTGCGGAAG
GGTGCGTGCCAGACACACAAGGTAGATAATGCTGGCTGCACGACTCTCTGTACATTTTTGTTTTATTTTATGTGTAGGGG
TGTTTTTCCTGCATGTACCTCTGTGCATGCAGTGCCCTTGTAGACCAGACAGGACATCAGATCCTCTGGAACTTGGACTT
ACAGATGGTTGTGAGCCACCATGAGGGTGCTAGGAATTTCACGAACCCAGGTCCTGTATCAGAGCGGCCAGCCTGGCTCT
TAACCACCGAGCAATCTCCAAACCATCATGATTCTTTTTGTAAAATATTATGTGTATGTATTTGTTGGTGTGTGAGTGGA
GGGGTGGTGCTGCAACATGCCTGTGCAGGTCATGGGACAACTTTGAGGAGTGATTTCTCTCCTGCTCCTAAGTGGGGTCC
TAGGGATCGAACTCAGGTCGTCAGGCTGCCGAGCCGTCTCACTGAGCCCAATGGTTACTATCTTGTTTGTAGTTCTTTAG
GGCAAGGTTTCGTTGTATTACTCAGGCTTTATTTGAAATCCTCCTGCCTCAGCTTCTTGTTCATCACTGTTCATCATGGA
AGGGAGTCAGGGCAGGAACTAGAACAAGGGAGGGACCTGGAGGCAGGAGCTGGTGCAGAGACCATGGAGGGATGCTGCTA
CGAACTTTCTCTTTATGACTTGCTCAGCTTGCTTTCTTACAGAACCGAGGACCACCAGCTCTGGGGAGACCCCTCCCACA
ATAGGCCACGCCCTTCCCCATCAATCACTAATTAAGAAAATGTTCTACAGGCTTGCCCATCGCCCAGTCTTTTTTTTTTT
AATATTTACTTATTTATTTAATGTATATGAGTACACCGTAGCTGTCTTCAGACACACCAGAAGAGGGCGTCAGATCTCAT
TACAGATGGTTATGAACCACCATGTGGTTGCTGGGAATTGAACTCACGACCTCTGGAAGAACAGTCAGTGCTCCTAACCA
CTGAGCCATCTCTCCAGCCCCCCCCCCCCCCCAGTCTTATGGAGGAATTTGTCTTAATTGAGGTTACCTCCTCTCAAATGAC
AATAGACTGTGTCAAGTTGACGCAAAGCTAGCCGGCATAGGCAGCGTAGTTGACACAGGTCCTTGTCAGTTAAGGAAACA
GCTGAAAATGGTAAGAAGTACCCCGTGACTTTCTTGGAGTGTGTGTAAGTGTCACACGGAGGGAAACCCACCAGCCCTGG
AAACCCTGTGTGTCTCACCTAGAACCCAGCCTGGCACGCGGCTTGCCCGTCCACAGGGCCACCGCCTTCAGCACAGAGCC
TCATAATCTTTCCCAAGTCTGGGACTGTATCAGCTCCACTGTCCTGACTTGGCATTTCCTGTTGCACTGTGCGTGATCTG
TTCATCTGCTGATGCCTCCCTCCTCTGTCCCATGTGTCCCCTAGGTGCAAAGTGGAAGCCTCAGGCTGCCCTCACCTGCT
CCGTTGTTCTTTCTGCTACCTCATACCTGCTTCACCATTACCTTTTATGACCCCTCTACCCCCATGGCCTTACTGCCTGC
TTGGACCCGTGTGGTAGTCTATCCTGCTCTTCTAGTGAGGGATGGTGCCCACCCTGCTCAGACTCTCTAACATTCCCGTC
TTTTTCTGAGTGAACCCCAAATTCCTCTCAGCAGCTTGAAGGCTCCCAATTTGTTTCTCTTTCCCACCCTCAATTCAGCA
CCCCACCCCCACACCACTATGCTCCTGGTTTGAGCCCATTAGACCTAGTCCCTCTTCAGAGGCCCTGGCGTGTCCCTTTG
GTGGCACATGCCTTTAATTCCCAGCACTTGGAAGGCAGAGAGGTAGGTGGATCTCTGAGTTCAAGGCCAGCCTGGTCTAT
AGAGTGGGTTTTATGACAGCCAGGGCTACACAGAGAAACCCTGTCTCAAAACGCCCTATCCCCCTAAAAAAATGATTTAT
TTATTATTTTCAGTGTTTTGTCTGTATTCACGTGTGTGGTGCTTGAGGAGGGTCAAAAGAGGTGTCAGGATTCCCTGGAA
CTGTGACCTGTCATGTGGGTGCTGGGAACCGAACTCAGATTCTCTATGGGTGCAGCCAGTGCTTGTAAACCTCGGAGCCA
TCCCTCCAGCCCACCATCCTTATTCTGTCACAGGCTTGGATGTGGCTCGCTCTTTTTCCTGATTGAGAAGTTGGTCAGCC
AGGTGAAGGGCTAGGGAGCTGACTGGATGCTGGCCAATAGCTGGCTCTCTACCCCTCCTTGCCTCAATTCTAATCTGATC
CCTTCTCCCACAGCTAGGAGTTGGGCTAGCCTGGGATATCTACTCTGTTCCTGAGTTGTTTGTGACCTCTGGCAGAGCC
CATTGGCTTGACACTTGGAAGGTGTCCCTGATGGCAGATAGCTCCTTGTAGATAGATACTGTGTTACGATGCTCCTCAGA
GCCTTTTATTAAGTATCTAAACGACTACAGTTGCTCTCTGCTTCATCTAGGGAGAGGGACACAAAGAGAGATTAAGTAAA
ACTTGTCCCCTGGTGTCCCATGTCTCCCCACCCCAACCCTCTACCTCCGCATTCCAGATTTAGGTGACAGAACCAAGATG
TAGCCTCTGCTAAAAGATTGTAACCCTTGAACAGCTGGCCTTGACTGGCCGTTCCTTCTTTTTCTCCCTTCTCTTCCATC
GATGTGCATGGCTAGCGGGTGTATTCCAGGGTTTTCTCCCACAAGAGAAGGCTGCAGATTCAGCACCCCTTGGTACCTCC
```

```
TCAGGAGGACTCTTAAGTGGGAAGGTCTGCCTTTTCCCAGTTTGTACCACCCTGTGACCTTGGGATCAAGGTCTGCCTGG
GCCATCGTGGGCTGCTCCCCAAGTGGAGACTGGGGCCCCAGATGTCAGGATGGGTGTCCCTGCAAGTTCCCTGTTCTCTG
GGCCCTGGGTAAGAATGCTTGGGTGTGCTGGGATTTCTTCTGTTTTCTTCCTCTTTCCTGATGCCTGGTTTCCGGTTCCC
ATGGCTTCCGGTCCCCCTGGCATGTGTGGAACTATTTTAGTTCCCTTCAGGGTGCAGCTGTGGCTATAGACTACCTATTT
TTAGTCCCAAGCATTTATTTCTGTTCCTTTCTGGCTCCTCGTACATCCACAAGTCCTCATAGCTTCAGTGTGTGTGTCTG
TTGGTATACACACCTTTTGGTGCGGGTCCACGTAATAGTGTCCCCCAGGTATAGTAGCACACAGACAGAGCTTGGTGTAT
GTATGGTCCAGTGCTGACATCCATGGATGTGAGTGTTTCTGTGTACCGCACGAGCTACAACCTATCCACATGGAGAAAAG
CCAGGAAGGGGATGCAGGCACATGGGTCATTGTCTGGGCCCATGTAGACACATGCACATATGTTGATGCCTGTTCCATTT
GCTGGGGGTGGGCAGTGGACATGGCTGCATGGCAGAATGATGGCCTTCGAGGTGCAAGCCACTGGGCTCCGACCTTAGCA
CTGCAGCAGACAGAAGGGGTCACGGGCACTGCATGGTCACTGTGTGCAGTAGTTAGGTGCTGGTATTTTGAAGCCAGACT
CTCCTGTTGCATTCCTGGAAGGAAGTTACCCTGGGACCCTTCCTGGAAGGATGGATTCTTTGCTATCTTCCCACAGTGAC
TCATCCCCTCATTCGCTCCAATTCCTGGAGTTCGGCTACCCGGAATCCTGTTCTCTGGCCTTGGATCCCAGCAGCCGACT
CTGGGGCCAGTCCTGGAGAGAGCCACTTGGTTCTCTGCAGAGCTTATCCTAGGTGCATCTTGGTTCCTAGGAGGCTTGGC
CCAGGCTGCCAGCTGCTGTGGCCTTCTGTGGCTTTTAGGGTACAGAGGAGTATTCAAATGTCCTTCCTTGGCCAGCAAAA
GAGAGGCCCCGGGCTACCGTGAGNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNAGAAGAAACATGGATGCTCTTGGGTATAGTGGAGGAGAAGGTTTATTGTAGATAAAAGGCACA
ACGGGAGAACATAGCTAGAGGCAGAGACCGCTGGAAGAGTCCAGAGTGGACATGACCTTGAACCCTGAGCTAGATGAGGG
GAGGGGGAGGGAAAAGGAGAGGAGAGAGGGGAGCCAGGTGCAACTGCCAAGAAGGAGGGAGGGCAATCAAAATGGTTGGA
TTATCCTGGGGAGAGTAGCCCAGCCCTCTGGGCTATAGAGTTCAGGGTAGGGGGCAGGGTATGCCAGCCTGAAGGGGTTC
TGAAACTGGTAGGGACTGAGGGATGCTGGGACGTGGCAGCCAGGTCTGCTTTGATATGTTAACTAGCCACCTAAACCATT
TGTGCAAGGTTTGAAACTCAATACCTTCAGTGCCAGATCCTGTGCCATCAATTTAGTTTGAGGAGGATTTAGCCTGGCAG
GGGTGGGTGGGGGTGTAGGTGGGGCTCTGCCCAAAATAGAAACAGGTTAGGAGGCTAAAGCAGGGGGATTGCTAGTTCTA
GGCCAGCCTGGGCTACCCTCCCCCCAAAATAAATAAAAAAAATTCCATAAAAATGAAAAACCAGAAAACAAAAACACTTG
ATCCTTTTACCTCTCTCCTCTCTCCTCACTCTCTCCCTCACTGGTGACCTTGGCCTCCACTGGCCTAGGGCAGGGTCAGG
CCAGGCTGAGCTACAAAGAGAAACAACAACAACAAAAAAACCCAGGTCTGGCTCAGCCTGGCTCTGAGTCCCAGTGGCTG
AGGAGGTTCCATGGCTGCCGAGGGAAGATTTCCACCCCTCTCCTCAGATCCCGGNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNCCCCAAACTGTCCCCTCCTGGGCCTTGACCTCAAA
GGAGTCTGGTGTGCCAGGTGGCTCATCTTTTTCCTGACCTTCCCCTTCAACTCCAGGCCTGTCCCCCTTCACCTTCCTGG
TTAGCTTGGCCTGTGGTCTTCTAGCTTGTGGCGGCAGCAAGCCACGACCTTGGCAGCGGCCTTCCCTCGCCCTCTCTGCT
TCCCACACGTGAGCCCTGGGGAAGTGAGCGTCTCCCACCTTTTTGTCCGTTTCCCGAGGCTGACTTCTGTCATTTTCTTA
TCTGGATCCTTAAATTCCTTTCCTCTGTCTCTTGTGTTCCTCCCAGCTCCCAGTACAGCAGCCAGAAACAGACCCTAGGT
CCTTCCCATGTGTCAAGAGGCCAGTACCTTGCCCTCTGATACCTTTCTGGCTTGCACACCCCACCCCTTGATTCCTCTCG
TGCGTGGGCTAGGTCTATAGTCTAATGTCTGACATTTCCAAGTGGGACTCCGCTGAGGTCAATTCAGACCCAATATGCCA
CACTAGTTACATGTACATTGGGCTTTTTTCCTTCTGCCACGTTGTCCCCATGGGGAGTCCCCAGGCTCAGGAGGGCAAGG
ACCTCTGTGGTGTTTGTTCTTTGTCCCCAGCGTTGGAACTGATGCCAGACACTGGGCCTTGGGTTCCCAAATGTTTACCC
AAAGTGGACAGGGTCGAATCACAGGGTGTTCTGGTACCCTGGCACTAGGAAGTCCTGTGCCAGGCTAGACCATGGGTGCC
GTGGTGCTACTGTCCTGCCATGGGTGATCCGGCTGATCCGGCACTTCCCCCTGTCACAGACAGAGTGCTCTTTCGAAGAC
ACAGTGCTTTAGTTTAAAGCTGTTTCAGGATCCAGCTAGGTGAGATGATGTGGATTTGATCCCCAGAGCCCACGTCAAAC
AAGCTGGTAATTCCATCCCTGGGGAGGCAGAGACAGGAGACTCCATGGGGCTCCCGGGATTCATTCCTGCAACGCAACTG
GGGAGCTCCAGGCTTTAGAAAGAATCGATCTTAAAAAAAAAAGATAGATAGTGCTGGAGGGAGCACACCTGAGGTTGGATT
GGCCTCTGGCCTCTACATATATACATACACACACACACACACACACACACAGAGAGAGAGAGGGAGAGAGAGAG
ACCCACACCCACAGAGGCAGACAGACAGACAAACACACACACCCAGAGAGACAGACAGACACACACAAAGAGCCAAACAC
ATACACCCACAGAGACACACACATAGAGACATACATGCACACAGAGGCATAGAGAAAGATAGACACACACACACACACAC
AGAGACGGAGGAGAGACATATAGAGACACACACACACACACACACACAGACACACATACAAAGACAGACAGACAGACA
GACAGACACACGCACACTCACTCACTCTTGCCCCGATCCATCAGTCCTTTCTTCACAGGCTCCAACCTGTCTGGCCAG
TCAGCGGTTCTGTTCTAAGCCCACAAGTCTCTCTCCTCCTCCATTCTCACATGCGCCGGAAGTTCCTTCTGCCCAGATAC
TTTTCCCCTTGGCTCTTTCACTCAGTTCCTCTCCTGTGTTTCAAAATAAATGACACGTATTTGTATTTTGGGGTGCTTGG
GAGGGACACAGAGAGGCACTCATACTAGACAAGTGTTCTCACACCAGTACACCCAAAGCCTGTGTGGGGATGTTTTTGAA
GACACTTCCTGGTCCCAGCTTCCCTGACCTCCCTTGACCTTTGGAAGTTTCCTTGCGCCCATCTCTAGTGAACTCATTCC
TTTGGTTGATGTCCTACCAGGATGGGGATCTGGTGTGTTTTGTACATAGCTTTACTCCTGCAGTGGTGGCCTGGCTGGGG
TGGGACCCTAGCAGCCATCGCTGAGGTCACAAGCCTTGCTGACTGCCTCTGCTCCATTGGGGGGGGGGGGGCTGTGCCT
CTGTTTTGCTTTTTTGTTTTTGTTTTTTGTTTTTGTTTTTGTTTTGTTTTTTTTTTTTTTTGGTTTTTCGAGACAGGG
TTTCTCTGTGTAGCCCTGGCTGTCCTGGAACTCACTTTGTAGACCAGGTTGGCCTTGAACTCAGAAATCTGCCTGCCTCT
GCCTCCCGAGTGCTGGGATTAAAGGCGTGCGCCACCACTGCCCAGCAAGGGCTGTGCCTCTGTCTCCAGGGGGCTGCCTC
CTGATGCCACTACCTAAGCCTCAGACCATGAGCCTCTGTCCCCTGGATGCCATTTCTTGAAGCCACCTGGGTGAGTTGGT
GTTCATATGTCCCAGACCACACCCACTGGCCCGAGTCTCTGGCAGGTGACCCTAGATCCAGGGTCTGAGTCTCTGCATGT
TTTCTCTTGACTCCAGCAACTTCTCACTGGTGTCTGTGCCAGGGCTGCTGGCTTCCCCCAGTAACCTCTTCCTGTCCCAC
CACAAAGGCAGTGGACCAGAAGGAAGAGACCCAGTTCTCAGCCATGTCATCGGGATGGCTGTGGGCGGTGGATAAATAAA
```

```
TCACACCTGTCTTAGCCAAGGGCGGGAGAGACCAGGAGGTGGGGTGCAGGTTTGCAATGCACGGCCTTCGGGAGTGAACG
GGAGTCTTCAGCAGCCTTGGTTTGACGTGGTCCGTGTACAGTCCTCCGCAGGGGACAAGTTTAGGTTTATGAATAAAGCG
AAGTCACTTCCTCCCCGGAGTTCCAGATAACCTGCCCCAGTGCGGGATGCAGTGCGGGATGCAGTGCGGGGTGCAGTGGA
CAGCTGCTTTCTCCTGCCAGTGATCTATATAGAAGGACCTGGGAGGTGTGGCTCACAGGTGCGAAGAACAAAAATAAACT
GGGTGCCATTACACACCTGTAATCCTGGCATTCCAGAGGCGAAAACAAGAGAACCAGGTGTTAAGTCATCCTGACTACAG
AGGGAATTGGAGGCCAGCCCTGGCTAAAGAGGCTGTCTAAAGGGTTCAGTGAGTAATGCTTGCCACCAAGCCTGTTGATT
GGCGTTCGATCCTGGGACCCACACAGTGGAAGAAAAGAACAGGCTCTTTCAAGTTGTCCTCTGACCGCTGTGGGTGTACC
TCAGGCACATTCACACTCCTCACACATACACTCAAATAACAAATGCAAAAAACAATAACAAATGCAAAGCAATGACGTTA
AGGGCTGGGAGAAGGGGGCGGGGCTTGGGGCTGTCCATTGCCTCTGATTGGCTTAGGTCACATGCTCATGTTTGAGCCAA
TCAGTGTGTGTCAAGAAAGCTTTCTTTGGAAAGAGGCGCTCTCTCTGAGGACTCAGGAGGGGATGGAGCGATGTTCTATG
GGTTGGCAGGAGGCAAGGGAGACAGGTAGCCAGAGTAAGGACAGAACCCTTGGCCCCTAGTTAGCACCGGATCACCCCAG
GTTCCGCTCGCTGCCTCTTATGTACGTACATGGGTGCTTTTTTTGACGCTGGCTCCAGGCTTTTTTGATTGGGGAGGCAG
CGCGCCAGGGTAAATTTTATAAATCTTAGGTTGTTGGGTCTTCAAGTACTTACTTGGGTTTCTCTCTTTTTATTTTATTT
CATTCTTATCCTCATTGTTATTTTGGTTTTTTGAAACAGGGTTTCTCTATGTAGTCCTGGCTGGCCTGGAACTTACTTTG
TAGACCAGGCTGTCCTCGAACTCATAGAGATCCATCTGCCTCTACCTCCCTCCTGAGTGCTAGGATTAGAGTATTTCACC
ACGCCCAGATTTTGCTACGGGCTACGGCTGCTTCTGCTTCTCCTTCCTATTTATTATTATTATTATTATTATTATTATTA
TTATTATTATTATTTGAGGCAGGGGCTCACACTGTAGCCCAGGCTGGCCTCACACTTTTGGCAATCCTTCTGCCTCTGCT
TCCCAAATCCTGGGAGTAAAGACATGTGCCGTAGTGCCTGGCCCATCTTTTTGTTTGTTAAAATCACATGTTTGTTTATT
TGTGTGTATTGTGGAGGGTAGGGGTGCCATATGTGTACTACACATGTGTAGAGGGCAGAGGACAACTTGCAAGAGTCAGT
TTTCTTCTTCTACCATTATGCATGGGGTTGAACTCAGGCAGGCTTGGCTGCTGGGGCTTTATTGGCTAAGTCATCTCTCC
TGCCCTCATGCTTTTGTTTTTGTTTTTATTTTGTTTCTTGAGACTGGGTTTCTCTGTGTAGCCCTGACTGTCTTAGAACT
CAATCAGTAGACCAGGCTGGCCTCGAACTCACAGAGATCTGTCTGCCTCTGCCTCCCAAGTGCTGGAATTAAATGTGTGT
GCCATCACCGCCCAGCTTAGCGCCTGTGTTTTATTTTTCTCTTTAAGGACCCAGATCTCAGAGGGAGCTGTGATCTGTTC
AAGGCCATAGTTGGTAGCCAGTGTTAGCTGGTCGTGGGCCAGGTTAACTGCCAGGCTGGCCGGGGAAGTGGGGTGCTGGG
CTAGGGAAAGATAAGAGATCTCGGTAAGTGAGGGCTAGTCATGTTGTTTTCTGGTGACTTCGGGCAGTCTCCCAGCTCCC
TCCCACCTACCTCTTCTTTGCTCTCATCTGACTGTAGGAGTGGGAGGAGGTGTTTTGATAAAGTAGGAGAGATGTCAGGA
CCCGGTATACCAAAGTTCAAGCTCTATGCGCCTTCTCTGAGGCTCTGAGTGCGCCGTAGGATGCAAACAGCGAGAACCAG
CCCAAGCCTCCGCGCCCTTCACTACACTCGGCATCAGGCTCAGATGCAGAGGAGGTAATCTGCTCTGGACGTGTTCTGCA
ATATAGCTCAGGAGTCGGCTCACGCCCCAGACAGAGCAGCTGTTGCCTCTTACCCAAGACTCTGAGGTACGCCATGTCCT
TCTGGAGCAGTATGGGGACTGAAGCTGAGCTTCTGCTAGTGTCTCTTTGTAAGCCGAGCTGGGCTCTCTGGCTACCCACA
TGTTTGCCGTCTGCCTGGAGGAGAGGTGGGAGGTGAGTGTGTTCCTGGAGGGTCACTGCCAAGGTTGTAAACCCATTGGA
AAGTCTCTGGGCTTGAGCCTTGCGGGGGATGGCTCTGCCAATTCCCTGCTGGGTAACCTCGGGCAAGTGCCTTAATTCCT
CTGTGCCTTTTCACCGTGTTCTTAATAGTGTCCTGTGTCTGAGGAGATCATACCTGTTAAACACCTAGAATAACAAGCTG
CTGAGTATCGCTCAGTATGCTGATGCACACATCCTCGGGGACCTGTCATCAATGCTTGCAACTGACTTCTCTTCTACATC
CTGCTCTGAGATGTCACAGGGCATGCTCATGGCACACCCAGCTTTCCCACCAGCCTTAAGAGTGGCTGGGGATGTCTCAA
GACTGATTCTGCAGTTGCCCAAGTACCTTGCTGGCTAGCCATGTCCCAGTAAACTCAGCCACCGGCTGTTCTCCCTCCCC
ACGTCTAGGTTTTTCTTTTAAAAATTATTGTTTTGGAAAAATCGCTGACACAATGGGACAGTGGGTCACATTGGCACTCA
CCCAAACCTCTTGGCCCTGCAGAGAATGTGCGTAGACCTCTGTTAGAGTCAAGGTCACTTATCCTCCTGCCCACAGCCTG
CAGCTACAGGCTGGCCCCTGTGTGTGAGGCCTCTGCCTCCAGCTCTGTCCTTTTTCCATTCAAATCTGTCTTACTTATTT
TCTGCCAGGGTAGGAACTGAACCTGGGGTCTCCTGCTTGATGTGCCATATCCCCAGTCCTTTCTCCTTAAAAACCTTTTT
ACACTTATTTATTTAAAGATTTTTTTTTTAAATTATGTGTCTTTGTGTGGGTATGTGCACATGAGTGCAGGTGCCTGTGGA
GGCCACATGAAGGCGCCAGATCCCCTGGAGCAAGAGTTCTAGTGAGCCACCTGACTATAGGGTACTGGAAATGGAACTTA
GGGTCTCTGCAATAGCACTCCATGCTTTTAACTGCTAAGCGGTCTCTCTGGCCCTTATTTTTGTTTGTTTGTTTGTTTGT
TTGTATAGCGATTAGAGGACATCCCTAGTTTGTCAGTTCCCTTTTCTGTCATGTTGACAGCAAGCACCTTTACTTGCTGA
GCCATTTTGAAAGCCCAACTCTTTCCTAATATTATTATTAAAGTTAGTCTCACTAAGTGTCCCAGGCTAGCCTTGGATGT
TCAGTCCTCCTGCTTTAGCCTGGGGTGGCAGGCTTGCCCACAGAGTCCATATTTGGTTGTAGCTGTTAAGTTTAGAGGTT
TGTCCTGTGTGGATCTCCTTCCTCCTATCAGTGGTACAACCAGGCCTCTCTTGCCACCTGTTCTGCAAGCCTTGTTTCCC
AGGAGCTGATGGCCACCTTCTAGGCAGGGCGCCCTGGTTGTGGTTGCTATGTGCTCTTCCCACAGACCCTGCACATAAAC
TGCCTAGGGTTATCCTGGCTGAGCAGGCAGTGCGAGCAGCGGGGCTTAGAGTTTCTTGCACCAGCTTCTGCTTGAGTTTC
CTGGCTTTCCCAGCCCCACCTCCATGTTCGGAGAGGTGTTGATCCCTCTGACGACCAGGCTGAGGCCAGCTCAGGGCTCC
CACCCACCCTTGCAGCTGTAGGAGTCCTGGCATGTCAGAGCTGGCCTTCTGGAGTTGGAGGCCCAGGAAGGTGGGACCTG
GGGGCTCATCCTGCTCTGCTCCCTACCGCCACCCCAACCCCCATCCTGACCTCTGAGCCCTGCAGAAATTCTAAGCCCTG
GCCCTGGTCCCCTCATCTGGCCTCTTAGCCCAGGAGGCTCCCCTGGGCTGAGAGCCCATCTTAGCCAGTGCTCCATTGTA
GCTGGGGTCCTGACCGGCGCTGGCCTCGGGATCTCCTTCTCCTGTCTGTTCCCAGCCCTCACTGTGCCTCTTGCGCTTCC
TTTCTTATTATTGTGAAATAGTACAAAGTTCTAACAAAGGTCATAATACACATCCGTCAGGTTAAATCAGCCCCAGGTGA
AAAGATGGAGCCTCATGGTCCCGAGGCCCTGCTGGAGGGAGCCACCACCCTTGGGTGCTCCTTGCCTTTTGTAGAACCAT
CCCTATATATCCGACTTGGTCTGGTTTTGAACTTCCTTTAGTGTCTACAAACTCTGACTTTATTGCTGTGGCATTTTGCT
TTCGTTGCTTTGTTTGTTTGTTTGTTTGGAGACCAAGCCTGCTCTGTAGCTCAGACTAACCTGGTATCAGTCTTTCTGTC
TCAGTCTCTGGCATGCAAGGCCCACAGATGCGCACCGCCAAGCCTGACCACATTTGCATCCTGGCTGTGGCCCGTTTTTT
TCAAGTTCATCTATGTAATAGCGTATACCTGTGCCTCCTAACTCTCTGTGACTGGACAATGTTTTGTGTGTGTGTGCGGT
TTTTAATGGTATAGATGTCACATTGTGTTTATCTGTCTTTAGCCGGTGGCAGCAGGTAGCTAGTGGCTCTTGTGGGTGTG
```

```
GGGAGCGTGGTGCTGTGGGGTGTGGACACATGCTCACTTGTCATGTCCTCTCTCTCTCCTTCCCTGTCTCCTCCTGTCAG
TGTCTCCCTCTGTGGCTCATCCTCTCTCTGGCTTCGATCTTGAGTACGTTCTTCCATCTGCTTCAGTCCCATCCATCCAC
ATCACTGTGGGTGAGAGCTTACGTTCCGAGGCCTTGGGCTGGGGTCCAGGTGTTGGCTCAGCCTTCTCCCAGCTATGTGA
CCTAGTGCCGTTGGCTCACCTCTCTGCCTTAGGTGACTTGCTTTACCAGGCCATCATCGTGAAAGGCCATCAGCTGTGTG
ACTTAAGCAACATCCGCTTCCTTTCTGGCTCTTCTGGAGGCTGCATGCCCTTGGCTGCCATGGCTGCTCCTGCTGTCTAG
CTTGCAGCCACTGCCTGCCTTTCCCTGTCTCCCTGCTGCTATCCTCAGTATCTCTCTCCTAGCTCTTCTTGCTGTAGACT
CCGGTCACACCGCATCAGGGACCATCCTGACTAATTTATTGCCTCCTTTTTTTTTTTAACCTACTGTGGCCCCATCATGG
GCTCTGAATCCAGTACATTCCAATACAGTTTGAGGCATGGGGGTGGGGTAGACAGGTGGGAGTGGTCGGCACCTGGAAC
GCTTCAGGCAATGACGCTATGTGGACACCTTGAAATCAGAGTCTCTGTGATTAGTGGCTGGGACCCTTGAAAGACTGGGT
CTTGTGGGAATGAAGAGGTGCCCTGGAGTAGCCATGCAGTAAGCAGTTGCTGTAGGGATGGGGTGGGGTCACACAGCTGCCT
TTGAGTCATCCGTGCTTCTATAAGTGACCCCAGTATCATTGCTGCACCCCATCTGATGGGCCAGAATCTCCTCTTAGGTC
AGGTGTCACTTTGGGGTGAATAGAAAGAGTCTCAAGGAAAAATCACACAAGAACAGGGGGTTTGCTGGGCAGTGGTGGTG
GCGCACGCCTTTAATCCCAGCACTTGGGAGGCAGAGGCAGGTGGATTTCAGAGTTCGAGGCCAGCCTGGTCTACAGAGTG
AGTTCCAGGACAGGCAGGGCTATACAGAGAAACCCTGTCTCAAAAAAACCACCCCCCCCAAAAAAAAAACCCAAAAAAAAC
CCCAGGGGGTTTTATGTTTATTTTGTTTATGTATTTACTTGTGTGTGTACGTGCACATGTATGTTTGTGTGTGTGTGCAT
GAGTTTTTGCGTATGCACGAGTGTGTGTGTGTGTGTGTGTGTGTGCATACAGACCTACAGACCCAAAGATGACATCAGGT
GTCTTCTTTTTCTGTCATTTTCTGTGGGAATAAAGGTAGATTCTAGTATTCTGGTTACAGTGTCCAGGGAGGTGGTGGCA
TATCCCAGCACTGGCCAATCACTTAGCCAGGTGTTACCATCTTATCTGGGTTGTCCGTGTGCATATCTGGCTCCTGGCTC
CTTGCTGTTGCCTGGTCTCCCTGTTTAATGTCTGTGAGAGCTTGTCTGTTACCATGGTGACAGATGCCCTGGTTTTTCCA
ACTCTATCAACCATATACAGCATACGCAACACACTTCCTGGGACCTGCCACATTGGGTGGCTTCAGATTGGTCCCTGGGA
TGTTTGAGTAGGCAGCTGGCTGCCTACTGTTAGCAGGCTGGTTACAAGTGTCCATTCACCCTCTCAGAGTTCTGTGTG
ACCAGCCTTCTTGAAGAAGACGACCTGTGGGTGTTTCAGTTTGCATTTCTGAGGTGTGTGTGTGTGTGTGTGTGTGTGTG
TGTGAGCATACATCTATGAGTGTGTGTATATGTGTAGGTGGGTGCATTTCTGTGTACTTGTGGATACCAGAGTCTAGGTT
GGGTGTTCTCCCCCATGATTCTCAAGTTTGCATGTTTGTTTATTCTGAGGCAAGCTCTTATTGTGTAGTGTAGGCTGACC
AGGAACTCACTGTGTAGATCAGGCTAGCCTCGGAACTCACTATGTAGATCAGGCTAGCCTCGAACTTACAGAGATGCACC
TGCCTTTTCCTCCTAAGTGCTGGGATTAATGGCATGCACCACCACACTTGATCGTAAATTATGTGTGTGTATATAAATTA
TATATTTATATAAATCTACAAAATGTATACTATATTTATGTTATATATATTTATATATATAAGTAAATGTATATTTGGTG
GTGCCGGAGTTTGAGCCCAGAGCTTCGTAGGTGCTAGTTAAGCTCTCTGCCACTCTCCTACCTCCCATGCCAGTTTATTT
ATTTATTTATTTATTTATTTATTATTTTATAGATTTATCTTTAGATTTATTTATTTTATGTAAGTACACTGTAGCTGTCTT
CAGACACTCCAGAAGAGGGCGTCAGATCTCATTACGGATGGGTGTGAGCCATGTGGTTGCTGGGATTTGAACTCAGGACC
TTCAGAAGGGCAGTCAGTGCTCTTACCCGCTGAGCCACCTCTCCAGCCCTGCCAGTTTATTTTTGACTCAGGGTCTCTCA
CTGAATCTGGAGCTTGCTGATTGTCTAGACTGTCTGACCATCAAGCTTCAGGAACCCTCCTTTCTCCACTGATCCCAATG
CCCAGAGTGCTGTAGTTATAGACCTGTATGTCTGGGTTTGATGACCCACTTGGCTTTTTTCATGGGTGCTGGGGCGAGGGT
CCGCACTCGGGTCCTCAGCTTGAACGGCTGGTACTTGTGTCTAGAGCCGTCTGTGCAGCCTGCTGAGCCTCGCTGGCTTG
TGAGTAAGGCTCTGAGAACTTGAAGGACTTTAACAGGCTAACCTGAGGTTTGGGCTTCACTTCTTCAGAGCATTTGTGTT
GCGCTGAGCATGTGGCTCACTTTGTAGCTGCTTGCCTGGCACACACGAGGCCCCGGGGGCAGCCCCCAGGACCCCATGAA
CTTTATATGATGGTCCCTATCTGTCATCTCAGATTCTGCTTGGCTACTCTAGGGAGTAGGAAGCCAATCTGGGCTAGAGA
GACACTGGCCCCCAAAAAGGAATCTGTGATGAAGGTCATGGGGCTCAGGAGTGTCTGCATCTTGCCTGGGATGACAGTTT
GATAGAGGCCAGCAGAAACGTCCCGCCAAGGGCTAGAGGCCAAGGACGAGTCTGTCCCATCACCAGAGCTGAAATCAGGG
TGTGTTTCTTTCAGTGGTGGTGCCCCAGCCTCCTAGAGAGGCCTGTGTTAGTCACCTAGGTCTAAACTTGTGTCCTTAAG
CCGTGGGAACTTCATGGCTAGGCTGGGCGCTTCTAGTGTCTTGAGGGCTCTTGGCTCTCTCCATTCCTGGAGGTGTTCAC
CGTTCCTCAGTTGACGGCATCAATGCTCCTGTTTCTGTCTCTATCTCTGTCTCAACGTGGCCTCCTCTGTATTTTCCATT
TCCTCTTCAAAACTGAGTTATTAGTATTATTTATGTGTATGTGCATATGCCTGCATGAGTTTATGTGCATAGCATGCGTG
CTTGGTCCCCATGAAGGCCGGAGGACATCAGAGCCCCTGGACCTGGAGTTACAGATAGTGTGAGCCACCACGTAGCTGCT
GGGAACCGAACACCGATGCCTGCATCCATCGAGCCATCTTTCCAGCTCCCCTGCTCTTCTTTGAGACCCAACCTAAGACC
AAACACTTTGACATTCATTCCTATTTTGAGTTAAGAGTCTCACCGTGCAGCCCAGGCAGGCTTTAAATTCACAATCCTCC
TGCCTCAGCTTCCCAAGTGCTGAAGTGACAGGCCTGTGCCACTGCGCTGATCTGATATTTTTAACTTGACATCTGGCCAG
ACCCTATTTCCGATTAAGGTCACACAGGGCACAGTCAAGTATCTTAGGATCACCACCTACATAGATGCATGGCAGAGTAC
CTTTTGCCCAGACAGCCCTACTGTGAAAATAAACTACAGGACAAAAATCACCAGTCTGTCCTGATAGCGGGCTCTCAGC
ACTTGAAGGTAGGAAGCAAGAGGATCAGAAGTTCAAGGTCATCTCTGCTAATAGTGAGTTTGAAGCCAGTCCAGAGGGGA
ATGACCCTCTGGGAAGACAAAGGTGACATGACACTCGCGTCCCCAGCGTCCCAAGCACAGAGGTAACTTTGGTTCATGG
CTCTTTGTTCCTTGCTGCAAAAAACATGTTTGTGTTTAGAAGAGCCCCTCCCTGCCCTCCCTGTCCTCCCTCTCCTGGCC
CCCGGTACCCGTGCGCACTTCCTGCACTTTGCAGTCTCGGATTGGGCACTCAGATGGCTCTGGGTCTCCCCCTTCTTTCC
AGAGCGCTGGAGGGATCAGCCGTGTTTCCCATCAGCCAAGGCCGCGGGGAATGGCTGTGCATTCAATAGCCATGTCTCTGG
GAAATTGGGCCTATTCCAGCACCATCTTCCCGTGTCTTCCGGAGCTTTCTCAGGAGATATATTTGTTTCTACAGACGCCA
CTAATTACTCCTGGCTGGGTGCCTGGAAATCACATCTGTGGATTAAGGTGCCACCAGATCTGAGCTTCCTCTAGGGCCCC
AGGAGTCCTGACATATCCCCCATTCAGCATACTTGCTCGCTTGCTTGCTTGAGCTCAGTTTGATTTCTCCACTCATACAG
CCCAGGACCCCTTGCCTAGGGAATGGGGCCGCCAATGGTGGACTAGGTCTTCTCAGGTCAGATAAGCTAATCATTTAGAC
AATCCCCACAGGCCAATCCAAATGTAGGCAGGCCGTCCTTCACTGAGACCCTCTTCCCAGGGGAATCTACGTTGTGTCAG
GTTGCCAGAGCTAACCGCTGCAAGGTTATAATTTTAAAGGACAAGACACATCATTGGGCGTGCTTAGCTCAAAGTTGTGG
CTTAAGTACGTAAAAGAGTAAAGGTTAATCAACACAGAATTTGGTCCCCGAAGTCACTTGCCGTGTTTAGTCACCCTCAG
```

```
AGCTTCACAGTAAGAACTTTTCTCAAAGAAAAAAAAAAAAAAAAAAACAGCCGGGCAGTGGTGTCGCATGTCTTTAATCCCA
GCACTTGAGAGGCTCATATTTCTGAGTTCAAGGCCAGCCTGGTCTACCCTCCAGGTACCATGCTGTGTGAGGTCCAGGCA
AGCTCACCAGATCCAGATCCGCTCCCATCTACTGAGGTCCACTCAAGGCATTTTCTGCAAGTGGAGGCTCACAGGCACTG
AGGTAGAGGTACCCGTGTGAGGCCAGATGAGTGAACCTTGACTCAAATCCCCATGTCCAGGAACTTGCTGTGCATCAAGA
TGGCAGGATTTATGTGGCAGCCCCTGGCAGAGCTGTCCCCTTAAGGATCTTGATCTTTAGACCATCTCCTCTCTGGATGG
TCATCTCTGGTGTGTGGCTGAGGGCAAGAACTTGATTCCTTGCGCCTTTGGTTTCCTGCTTGTCAGTGGGGCTGGGGCTG
AGAGAGATAAATGGGTATCATTGGGGAATCAGAGAGGTGTTGCCGGGATCGGATCGGGGAAGGGTTCTTGCTGCTAAGCCTAG
TAACCGGAATTGGATCTCTGGAACCCACATGGTGGAAGGACAGACAGACTCCTATAAGTTATCTATCCTCTGGCTTCCAC
ACGTGTGCTGTGGTGCATCTGTGCCTGTGCACATAATAAATAAATACACAAATAAGTAAATGTGATTTAAAAAAATAATT
GAAAGGGTCTAAGAAGTGTCCTGTGTTTTCTGTAATTCTCCACTAAGTTTATGGCCGATGGCCGTATGGAGTAGAATGGA
GGAGGCGGGCCCAGGTCTAGCTTCCGACTCAGTCAGGGTCTCATCTCCCAAGGACTCAGTTTCCCGGTCTGCAGAGTGGT
TGGTGTTAATTCCTGTATCCAGTTGTCAGGACTTAGGGGTCAGCCCACCTACCTATAAGGCTGTTGTCTGGGGTGGGAGT
CATTGATGATGATGAAGGTGTCCCCGGGGACCTCTCACACAAGGGGACGAGTCAAAGAAAGGTGAGGAGAAGGGCCAGC
CAGGGAAATGGCCATGGCTGTGGGCGGGAGGCTGCAGTGGACTGAGCAGCCTGGCCTTCGAGGTCTCCCCACAGTCGACC
TTCCCAGTCTGGGCTTTCATGGTGTGTGGTCCAGGAGCAGATAAGTGGACCGAGCTATCAGCAGTCAGGGTCACCGAGTC
CTCATGTGGAAGTGGAGGCGGGGGAGAAGGGGAAGGGAGATATAGAATGGGCTTTGTTTCAAGGTAGAGGCCAATGGAGA
GCCATTGTGGAAGAACCCTATATGTATAGCCTGGCCTCAGAGAGTGCCATTGCCCCCTACCCAGACCCACAGCGGGGTCT
AGCTGAGCTCTGGCTGAAAAAAAAAACCAAAACCCCCAAACAACAATAACAACACAAAACCTGTGGGATTGGAAGGTGC
CTGTCTCCTAGCAGCTGGGATACCCACAAACTCCAGCTCTAAGTGTCGATTTAAGACACTTGGTGCCTTAAGTCACCTCC
AGGTGTCTTTCAGTCCCACCAAAACCTTGGGGATTATTGATCTGGCAGATGGGACAGACACAGAGGAGCAGGGTAGAGAG
GAGGAGGAGTCTGAGGCTCTTCTGGGGAAGCAGGTGTTCCCTCTTCCTGTTGAGCTGACTGCCCCGTTGCATGCTGGGCC
CCTCCCAGGCCCTGGAGTTGCATGGTGGGCAGGAAACAGGGTCTTGATGGACTGTCTGCAGGACTTGACACCTGCACAA
GAGGGGCAGTGTGACTGGCCTGTGGTGAGTGTGGCTAACAAGGCCACTGGTGAGGGAGGGGCCTCTGGTGCTGAGGAGAA
GCAGGCCCCTCTGAGGGCCAGGCAAGGAGCATTTCCACCTGACAGAAAACGAAATGAGTATCCGAAGTAGTTTACAGGTG
GTGAGAGCCCTGGAGCAGATAGATGCTCAGGACACCTGTCACTGGGCTGCAGGTTGCTGAAGGTCAAAGGTCGTGCAAAC
CCACAAGGACAGCCTCTGTTGAACTTAGTAGGTCGTAGGTGATGTTGCTGGTACTGTAAAAGGGTGCAGCTGCCATGAGA
AGCTGGTGTCAGGTCACTGTCCCGTGCAGCCGTCCTCCTGGGGAGTCTGGACACCACACAACAGCTGAAGGTAGAAGGGT
CCCAATACTTAGTGATGCTGGATGGGCAGATAGGAAAAGCAGATGAAATACAGAGAGGTGAGCTGGAGAGGGTTTCAGAG
GTCAGGAGCTCACATGGCTCTTCCAAAGGACCAGAGTTCAGTTCCCTGCACCCACATCAGGCGAGTCACAGCTACTTGTA
ATATCAGCTCCAGGAGATCTGACACCCTTCTGGGAGCCTCTTTAGGGACTGTGCTCATGTGCACAGAGCAACACACACAT
TAATAAAATTAAGATTAAATCTTCAGAAATGAAAAGAGGGCTCTCCAAGAGGGAATAGTATGCAGCCGTATAAAGGAAGA
GAGAGATTCCATTTATTTATTTATTTTATTTATTTATCTATCTATCTATCTGGAGACAGGGTTTCATTATATAGTCCCCAG
GTAAATAGTTCAAACTCGTGGTCCTCCTGCCTCAGCTTTAAAGTCCTGCGATAGCTGCTGCAGAGGAGAGAATTCTCCCA
CACACTGGAACATGGATGAATGCTGAAGTTAGATTCTGAGAGACGGCAGGATGGTTGCAGCGTGATTTCGTTTACTGGGG
CCCCTAGAGTACCTATTGATGAGGTTGGAGAAATGGGACACTAAGGAGGTAGTGGGAGGGGAAAGGGGCCAGGGAGAGTG
TTAAGTGGAGACAGGGTCTCAGAAGGACATGGAAGGGCGTGGCCATTGGTGGGGACAGTTGCCCAAGCCATGGGTGTACT
TAACACCATCAGAATGGATGCTATAAAATGGCACTGCCTTTAATCCTAGCAGTGGAAGCAGAGGCAGGCAGATCTCTGAG
TTCAAGACCAGCTTGGTCTACAGATGGCGTTCCAAGACAGCTGAGGCTACAGAGAAACCATGTCTGGGGTTGGGGGCTTG
GGGGGAGAGTGACAAAACAAAACCCAAGTCTGGAGACTTTAGCATGATAAAAATGAATAAATCATTATGGCTGATGTCCC
CTGAAGGGACATGGGTCACAGCCCTCCTTACTCTGGTTTGTAAAAGGGGGACCCAGTGGCCATAGTGACTATGTTCCAGC
CTGATGAGTGTCTTAGAAAAAACCCAGTTCCCTGGAAATATGGCCCTGAGCTGATAGCTGATGCTGGCTGCCACTGCCCT
TCCTCTGAGGTTTGGCAGGGCTCTGGGATTGTTCTTGAACCCCAGCCAGTCTACAGCTGGAGGCTCTGTGATATGTCTTC
CTTGAAGTGTCTGGGAGTTGGTATCTTCCTAGGGACAGCCTTGTGCCCCAGGCTATTGCATGCAGTGCCACTGGAAGGAG
CCCTAAGGGGACCAGGCAACAGCCTGGGGATTAGGCTGTAGAGACCTGAGGATGGGGCCCCTGGCCAACCCCAACGGCCT
TGTGTGGCACTGGCTCAATGGAGAACTCACACGTTTCTAATGTCTGTAGCTGCTACTGAAAGCATTCTCTAGAAGTCTCC
CAAAGACTTCTCACTCAGTCTAAAGTCAGGGCTACCTTCCCTGTATGGTTGGGCTCTACCCAACAGGTGAACCACATGTG
TTTCTAGAGGGGTAGGAGGCAGAGAGGCCTTGGGTGGGCAACAAGGTAATCTGCTTCTGTGTCAATTTCTTATCTGGCAT
GTGGCTGTCGAGAAGCAGAGATCGGAGGATCATGGGCATGAGCTATACACTGAGGTCCAGGCTAGCCTGGGTGACATAAC
CTGTCAGAAAAACTACAAGAAAAGATTCGCATTCACCATCTGGCTTGGACCTTGGTTAAGTGATAACCATTGTCATTAGC
CTGACACTTAGGCGCCAGGATCAAGAAACAAGAACTCCCAGAATCCTTTGGGGCACCAGAAAGCCTTTCATTTTGAGACA
GGCTCTGGCTGTGTCTCTGGCTCTCGTGGAAGTCACACAGCAATCCCTTTGCCTTAGCCTAAGTGCAGAGATTGTAGACG
TGAGTTATACTAGCCTCCTCTGCATGGATTGGCTTGACCTAAACTGAGCTGGGCTCTCCATCGACTTGCTTCTGCGATTT
CAGAGTGGCAGCCTGACCTAGCTCTGCAGTGGCTTGGTGCAGCTCAGCCTGAGGCTGGTGAGATGCTTACAAGTCATAGC
TGGTTGCTGCTTGGTGCTGGTGGTCATGTTATGAGCAAACTGGTCCCTGGTCAGTGGGCTCCGGATTAGGGCTGTGATGG
AGGCCTCTAGGAATCTCGCTAAGTCTCTCACTGGTTGTTTTCATCTCTCTGAGCGATGTGTAGGATTGAGTGACAGATGG
CAGATAATTGGGAGTGGGTGACAGGCTTCAGTATTCTACTTTAATGGCTGTTACCCGGTGATGCCAGCATAGGCGTCTGA
TGCTCTGAGTACTTCCTCTCATGTCCCTGAGTCATGCAGACTTAGGAGGCCTCTTCTCCACTCACAACCGTTTCCCTGAG
GGCAGAATCCAGTTCGGTTAACTTTGCTTCGTAGTACTAAATACCAGTTTGGGGCCTCGACAGTGCGGGGGAGTGGGGCA
GGTTAGAATGAAAAAAAAAGGAGCAAACAGGTTCATTAACAGCTAAATGAATGAATGACTGATGAAAGGATGAATGAATG
AAGTGTTCTCAGCCTATGCTGTTCTGGGTCAGGGCTGAGTGGGAATGTCAAAGGGTGTGGCCACTATGTGAGCCATGGAG
```

```
TTGGGGTTAGAGGGAAGGCTGCCTGTGGGCTGCACCAGTGTTGGGATGCAACCCTTGTTGGGCAGCACCTAGTTTTCCAA
AGGGAGTCAGAAACGAGGCTTTTGAGACTAACCTTGGTTATGTCTGACAGGGTTTGGGGGTGGGGTGGGCACATAGACAC
TCTTGCTTTTTGTTTGTTTCCTTTTCTTTTCTGGGGAGTGTGTGGTGTCAACTTGACAGGGTCTAGAATCACCTTGGAGA
CAAATCTCTGGATGTGTCTGTGATGGAGCCACCCTAACTGCTGGAGCAGAGAAAAAGGAGAAAGATCCTAGCAGTCATTC
TCCCCTCCCTGCTTCCTGACTGTGGACACAGCCTGGCAAGCTGCCCCTGGCTTCTTCCTCTATTGCCTTTCCACCAAGAT
GGACTGCGGCTTTGATCAGTGAGCCAGATTAGCCTTCCTTAAGCTGCTTCTGTCCTACGTATGTATGTATGTATGTATGT
ATGCATGTATGTATGTATGTAGCAAGCCAGGGGCCTGTGCACATACCACACAAGTGCTCCAATGTTAGCATTTGGCCCTT
GAGACTTGAGCGTTGGAGTTTCTATCGAGTCTGGGAAAATTACTGCACACCAGAAGCCTGTTAAACAGTAACAGGCCCCA
GAGCGAGAGCCAACCTCACACGCCTCCCGTAAGCTTCTCCCACTGACAGCTGTCTGTGGGCAGACACGGTTGATATGCG
CACCTGCTCCCTGGTCAGCATGGTAGGGGTTCTCCTCTGAGGGACTTTATTGGGGTAGCCCTTGGGAGCAGAAGGAATAT
GGTCCGGGGATTGACATACACATAGGTGCCCACGTGTTTGCGTCCAGTCCAGTCCAACCAGGGAGCTTCCTAAGAACCCT
GGAGTGGCTCTCACTCCACTTGTCTCCAGGGAAACTCACTAGGTCCTGATGTTGCCCTAGAGGATGCTGGGACCTTCTGA
TGGAGCCACCTTAGCCCTCTTTCCTGGGGCTCTGACTTTTATAGCTGGGCAGGCTCTCTGCTTTGTCAAGCTGGGGTCAA
AGTGAGGTCACAAGGCTTATACCTTGAGGACACCTTTCTACTTCTGTCCCTAAGATGTATCTGCCTGCTCTGGTGTGTCA
CCGGAGCAGAAACCAGTTTCCCCATAGCCCGAGGGACTCACCGGCCACACCCTTTGATGTCTAGGGGTCATTTGAGGATG
AGATTATGCATCTCCCAGCCACTCAATCGGATGTCGGCTTCACCCTTGTGTATTAAGCTACTGTAGCAAAACCCTTGAAA
ACATTTATGGATAACCAGCGGGCAGGTGCTGGCGTTTCCCCAGGATGTACAGTGAACAGGTACTTAGCTCTAGCAGGCAA
ACCATGGCGCCGATACAGTTCCACCGACGTCGTGCATGCATTCTGGTTGCCCCAGAGCCTTGCCAACACTTGTGATTATC
AATCCCTCTCGTTTTGGCTGTGTTGGTATACGTCATAGAGATTCTTGTGAATTTGGGGGATATGTCCAGAGTTGGGGGGG
GCTTTATGATGTCACAGGCCACCAGTCTTTATGCTCTGTCATCCTTTGGGTATGGTTTTCAGTCTCAAAGCTTTCCCATG
ATCCAAGGTAGCTCTGTAGCTCTCTCCATCAGGACCGAATTTCTGGGTGGGGGTGGCGGGTGTTTCACTGAAACCACCAG
AACTTTTAGTGTGGTGGGAGGGGCTGCTGTTACAAAGATTGGTCCTGTCCATTTTCTTTTTTCTTTTTTTAAGATTTATT
TATTTATTACATGTAAGTATACTGTAGGTGTCTTCAGACACCTACAGTATGCATAGGGCATCAGATCTCATTACGGATGG
TTGTGAGCCACCTTGTGGTTGCTGGGATTTGAACTCAGGACCTTTGGAAGAGCAGTCAGTGCTCTTAACTGCTGAGCCAT
CTCTCCAGCCCTTGTCCATTTTCTTCCCTGCTGTGGTTTAGGCCTAGCACACAGCCTCTTGTGCACTGGGTGCTCAGCAG
AGGCCTCGTGGATGCCAGGAACTGAACCCAGGTCCTTTGTAAGAGCAGCCAGTGCTCCTAACCACTGAGCCATCTCTCCA
GCCATTCCTGGTGTGTTCCTGGTGTGTGGTGTCTTCATGCCCTAGGCTGGATTGTCCTTTGGGGCCCATCCTTCCTGTAT
ACATAGTAACTCTACAGGGCTAGTACATTGGGACTGGCTGGCTGGCTCAGAGCTGACAGGATATGTGTCTCAACCCAGAT
CCAGGGCTGGTGAACCCAGTGGCAATGACCAGAACTGTTTCCTCTCCCTCCTCCTCCTCCCTCTCCTCCTCCTTTTC
TTCTTCTTCCTTCTCTCCTTCCTCTTCCTCCTCCTCTTGTTTGGAGATAGGGTTTCTTTCTGCATCCCTAGCTGCCCT
GGTAGACCACCAGGTTAGCCTTGAACACAGAGATCCACCTGCTTCTGCCTCCTAAGAGTTGGATTAAAGGTGTGAGCCGA
CCTCTGTCAGCCTGGCTCAAAAACCTGCTTTTTTTTTTGAACCTGAAATGTGTTTGCTGTGATTCTTGGGGGGAGGAGGGA
CTGGATTTTGCAATTTCCCAGGTCAGATTAATGATTGCTTTGCGAGGGCTGCAGCACCTGAGCTGAGCCACACTGAGCAG
GGCCTGGAGAGGCTAGGTACAGTGTCCTGGAGATCCTGTGCAGTCCTGTGTGGGGATAGAGGCTGGGGGAGCTTCCATCT
CTGTGATCTTCATGAACCAGAGGCCTCTGAGCCTGATGCCTGTGGGATATATGGTCAGAGAGTTAAAGAGGTGTAGTGAC
CAGAAGTGTCCTGTTACCCACACAGAGGGTCTGTTGGGTCCCTAGCACCTCATGTGGCGGCCTTGATCAGCTCCAGCTGC
CTTTTTGTTTGTTTTTCTTTTGAATGGAGCCTCTGATGTCCAAGTGTGTTCGGTTCCTGCAGACTGACCTATATCCTGGC
CATCAGAGAACCCGCTAACCCCCGGATTATGGTGCTCACTCCCAGTTTGGCTTGCCCAGACCCCACCTCAGACTCAAAGC
CTTTGTTTCTCTGATGCCCAGCCACCCTACCCAATCCCTCTCTGGAAGGCAGATGGCTTATTCTGTGCTTATTCTGTACC
CAGGTCACTGCTGCCAAGCACATGCCTAGTATGTACTGGGGTCGTGGGGGAGCAGGGGTCCAAGCAAATGATCAGAATGC
TCACAGACCTTTGTGGTGTGGAGCATGGAGATTGCTCCAGTTTTCCTGTCTCTCGGGCACTTGGTGTGCATTTTGGACAT
CCCCAAGGCTTGCAGAATGGAGTCGAGGTAGCGTATGGCTCCCCACGGAAGGAGGAAACCTCAGTGCATTAGTCTGTCGG
TATTGCTGTAATGAAAGACACAGGCTTGGTGTTAGCATCACATCTGATGGGGCCACTTCATGTTGTTGCGAACTGCTTGG
AGCAGCTATGAGCCGAGAGATTACAGTGGGGAAGCCAGAAGGCAAAGAAAGTCTGCACCATCCCCACCACCAATCCCCAG
CCCTTTCCCAGTGGGGCTAAGCCTCCTGAGGGTCCCCTCCCAAGGCTTCATGTCAGAGACCAAGTCTCCAACTGCCACCG
AAAGCACGAGAATAAAGTGTTTGCAGAGCAGACATTTTTAAGGTAATGATCTGGTGGGCCAATGAGTGTCCTTTAGAGAA
GACAGAAAGAATGTGCAGCGGAGAGGAGGGGACAGAGAAGCAGGGGTGGGAGCCAAGAGGCTGTACGAGGCTGAGAGGC
TCACCGCTGGAGCTCAGGGATGCTTTGGTGACAGCTGGCCCAGGAAGCTGGTGCTTGGTAAGGCTCCCCACGTGGGAGCT
GTGGTCATTTATTTTAGAAATATAAATGTGTGTGTGTATGTGTGTGTGTGTGTATATCTGTGCACATGTGCATACTCGGT
GACTTAGGAGGCCAGAGGATGATGTCATATCCCATGGAACTAGAGTTACAGATGGTTGTGAGCCACCAAGTGGGTCCTGG
GATTTGAACCTGGGTCCTCTGGAAGAACCCAGCCAATGCTCTAACCAATGAACCACCTCACTTTGTTGTTCAAAGGCAGG
GTCTCTCACTAGGATGTGAGTCTCGCCATTTTTAGGCTAGCCTGGCTGGCCAGCAAGCCCTGGGCATTATCCCGCTGTCT
CCACCTCTCCAGAGCTGGGATCACAAGCATACACCACCATGCCTGGCTTTGTATGGGGGCCAAACTCGGCTCCTTCCACT
TATGAAGCAAGCACTTTATGGCTGAGCCATCTCCCTAGAATTCCAAACTCTGTTCTCAAGAACACACTATGTCAAAGGTG
CCCACAGGATGCTGGGGGTTTGTTTCCTACAATGATGGATTATGGCGCTCCAGAATTGGCTTGGGCTCCTTCCTCCAAGT
CCTCTGAGGTCCTTCCAGGTGCCTGGCACTGCTCAGAGTTCACGGATCCAGCATGAGCATCATTGGTGGGGTTTATGGCC
TGGCAGAGAGACGTGAGCACAATACACAGGATGAGTCATTAACGTAATCGGGAAGAAGGCAGGGAGAGCCTCGGAATAAA
CAAGAGTTTAAGAGGGAGCCCAGGGCTCGGGGTGGGCTCTGTGGGGGTTCCTGAGCACAGACACAGGATCCCAGGAGGCT
TCCTCTGCAAACATGGAAGGCAGAAGAAGCGACGACCAGTGCAAGGGCCTGGTGGGTGAGAGTGTGTGAACAGGCAAGGC
AGAGTAAGCATGCCAGGCTGGTCTAAAGGACTAATGGGGGTATCAGTAAGACACAGCCCTAGAGATGCCACTGAGCCAGA
TGGGCAAAGGCTTATAGGTCAGTGTTAGCATGGTAAAAATTTACAATAAATAAATAAACAAACAATCCATTTGGTTTCGGG
```

```
ATGGCCATACATATGCCAGGGTGCATGTAGAAGACACAGAACAATGTCTTGGAGTCTTCTGTTCTGTTGTGTGGGTCCTG
GGGATCCAGCTCTGGTCCTTAGGCTTGGTGATTAAGCTCCTTTATTTTGCAGGCCACAATCTGAAAGATGCTGGGCCCTG
GGCTTCAGATAGGGGAAGGCCACTCCCCACAGGCATTCCATTCCCCACTCTGGTGGCTGTACATGAGGTACCTCGTGGTT
CAGCCTCCCGCAACACCCCAACCACCTGCTTGCAGAGGGAACCGCCCATCACCCCATGGTTTTCAGTGAGATTCTGCCTA
GTCCTCTGTGCCAAAAAAGCCAAGCAGAATCTTTGGGGAGGCTGCCTTTCCTGCTCTCCTGAGAGTTCTCTGGGTGGCGA
AGGGAGACAATCTGTCTGAGATGAGGACCTTCGGGCGCTCTCCACCGCTCTGTTGGCCTCTCAAACCTTGAGAATCCCTC
CGGGGCAGAGTTTTCAAACTGGGATGTTATCTTTCTGATATCGGTTGTGACATCCCTGGGGTGTGTGGGCAACTGATGTT
TAAAACATGGCGGGAGTGTGCTGGTGATGATGGGGTGATAAAGAAATGGCTCAGAGGTTATGAGGAGTATGTCTCATCTC
ACACACACACACACACACACACACACACACACAATACACACACACACACGCGCACGCACATTTGAGTGTGGGGT
GCTAGGAACTGAATTCAGTCTCCTGGAACAGCCTCAAGTGCTTTTAACCACTAAGCCAGCTCCCCACCCCCACCCCACCC
CCGCAAATTAAAAACAAGCAAATATATAAAAGTCTAAAACACAGTGTGAGCCAGGTGTAATGATACCCCAGGCTCCCTAT
AATCTGGACATTGAAGAGGCGGAGGCAGAAAGATCAGGAGTTTCAGAGTCATCCTCAGCTACATATCAAATCAGAAATCC
TGGAGTTGGGGTGGAAATCATTTTGTACTGGAGAGGTTGCCCTGGGTGGCAGGCAGCATTTGGGAGCTGGTATTCGGGGT
GTTGTCCCAGGTAGAGGCTCCGCAGAGCCATAGGGCACTGCACAGATATGATCTATGTTAGAAGTGCACACAAGTACCCG
CTGGCTGCCCGGGCTGTGTACCTCACCCATGCCCTCACTTATTTAGCTTCAGCTTCCTATTGCAGTGTTCTGGGGAAGTA
TAGTCTACTTGACTTTACAGCTGACCTTTTAAAAATGATGGGTGCATTTCAGTTTCGGTATTCAATGGAGGGCGCAGCAA
GATGGCTCAGTGGGTGAAGGCACTTGCTGCCAAGCCTAATGGCCTGAGTTCGATCCCTGGGGCCCATATGGTGGAAGGAG
GGAGAACTGTCCTCTGACCTCCACACATGCACCCTGGCGTAAGCAAACCCACACACATACCCATATACACAAATAAGTAA
CTGCAGAGATTAAAAGAATATAAAAACCTTTATTGAGACACGCCAGCCTTGTTTATTCTGGAATTTCTCACAGTAGGACT
CACAACGCCCTGTGCATATTTCCTTCTGTATTCTCTCCCCAGTAGTATACTGAGTCCTTCCCCTTCCCCTCCCTTTTTTC
TTTTTGAGATGGTCTTACGTAGCCCAGGCTAGCCCTGAATTCACTATGTAGCTGAGGATGGCCTGGGACTCCTGCTCCTC
CTGCCTGTGCTTCTGGAGGGCTGGGGTGGCAGAGGTGGGCACTGTGCCCACTTTGTTCAGGGCTGGGGATGGATCTAATT
TAATCCACCAGGTCACACACACACGAGTGGAGCCATGAGCACCCGTGGGACTTGTCTCGTGTTGGCAGAGGCAGGTTGGT
AATCTCCCAGCAGCTCCACAGATGTTTCCTAGAGTCTCGGTTTATAAACACTCCTGTCATGGGTGGACACGGAGCCCCGC
ACGCTTGCTCGGCAGGCCAGCCTTTGCTGGAACACCCTCCTGGGACCTTTTCCTGGCAAAGCCTGTTTTGTCTTTGTTTT
GTCCTCTTGCTGTTGTGAAGCGGGTGATTGCCATGCAGCAAGGCTGGCCTCAGAAAGCCAGTCAACCCTCTGCCTTAGCT
GCCTGAGCGCTAAAGTGACTGGCGTGTGCTCCAACAGCGTGCACCCCGAGAGCCAGCTCTTAATTTTTTTTTTTTTTTAG
TTTTCCACCATGTGTTGATTTGGTTGTGGTGGTAGGGATTGAACCCAGGGCCTTGCACGGGCTGGGTAAGCTCTTTTCCA
CTGAGCTGCATCTATGTCTTCTCTTCTTTTAAATGTAAATCGTTTATTCAGTCTTTGAGAACCACAACATTCATACAAC
GTATTTCAATGATACACGCTTGCTACTCCTCCCAGATCCCCCTACAGGCCACTCCTGATTTATATCCATTTTGGTTTTG
GTTTTGGTTTTGTTCACCCCTTCGAGACAGGATCTCACTAGATAGTGCTGGCTGGTCTTAAACTCACATAGATCCTTGTG
CCTCTGCCTCCCAAGCGTTGGAAGTAAAGTCACGTGCCACCACAGCCAGTACTTTCTTCTTGTCTTTTCTTTTTCCCCCT
TATTTACCCACTCAATCCAATTGCTGTGGCCCATATACATATTGTAGGGGCAGTTTTACACTGTGTGGCAGTAAGCAAAA
TAGTAGTTCTGGGGTCATGGGGGAACAGCACCCCCAGAGCCTGTCTCAGCCATTTGATTCTCAGCCAGATTTATAGTATC
GGGCATGTTTTTCGTCCTATGGAGGAAGGCCTTAAATCCAGTCAGAGAGTATTGGCTAAACCCATAATATCCGTGCCACTA
ATGCGTCCGTGGGCAGATCTTACCAGGCTGATAAGCTGCAGGGTAGTTTGCAGGAGTCACAGCTGCCTAAGGGTGCCAGG
GTCTTGAAATGCCTCTCTGTTTTTGCCAGATGTTCAGGCTGTGGGTGACCAACGGAAGGTGCGGCAGGACATCTCTTAGG
TGGACTCAGCAGGGGATTTTTCTTTCAGTGGGTCCCGAGGAGGACAGGCCAGACCCAGCCATGGCTTTCATGACTCCGGC
CTTGCAGTACTCAAGGACAAACCCCAGGCCCAGAGAGCCAAGCTAGGCCTGAGAGAGTGTGGCCCAGTGTCCGCCTGCCT
CGGAGTCTACTCTGACGTGGGGCTGGGCTTGACCTGCCAGTTCTGGCTTGCCACCTCCCCTGGAGCCTCTGCCTGTTGTT
TCTGGCTGTTTGGGGACAGTGGGGTGGTTGTCTTTGTCCTGGCAGGTCGAGTTCAAAGTCAAAGCTGTTTGGCTTTTGGA
CACTGCTATCTCTGATGAGCTGGGGGTGGTGACTCTTAGCTCTGTGGCATTAGCAGAGGTCCCAGGTCAGGGAATGGTCC
ACCTCTTCCTCCTAGCCTTGGAGAAAATCAGTAGTTCTGTTTTGAGACAGAGTGTCTCATTCACTGTGTATCCCAGGCTG
ACCTCAAAGTTACCATCTAACTCCTTCAGTGTCCCAAGTGCTAGGATGACAGCCCTGCATGACTGGACCCAGGGCTGCCT
GCACACTAGGCAATCACTAAGCCCCGCCCCCGGTCCTGCTTACTGTCTTCTGTCCCTCACTGCTGGTGACTCAAGAATCA
TCCTCTTCCCTCTCCCTGCTCCTCAGTACATCACACAGCACTGGCTCTGCCCCCGCTCCACCCCCCCCCACCCCCACCCC
CCCACCCGGTGCATAAAGCCCTTGTCTTCCCGATTTGGTAAAAGCTTCACGGGACTGGAGACAGCCAGCCTGTCTACTTC
CCACTCCTGTCTGCTACTGCGGGAACCTCATCTGTCAAAAATGAATGAATGAGTGACCAAATGAATGAATGAAGAAAAGA
ACAAAAGAATGCGGGAGTAAATGTGAAATGATCAGTGAATAATGAATGAATGAACGAACGAACGGCTTTCTGGATGAGTT
ATTGACTGAAAGAATGGAGAGACAGCCACCTGTGCAGAGCTCCAGCCCTAGGAGCACCTCCCTCGATGAGCACTACCTAG
CCAGTGTTCTGTTTTGCTGTGAAGAGAGACCACAACGAAGACAACTCTGTATTATAAAGCATTTAACTGGGGGCTTGTTT
ACAGTTTCAGAGGCTTAGTCCGTTATCCTCATGGCAGGGAGCATGGTGCAAGTTAGGTGGAGTTAGTGTTGGAGCAAGTT
AGTGTTGGAGCAGTAGCTGAGATCACAGGCAGAGAGGGAGAGAGAGACAGGGAGGGAGGGAGACCAATGGACCCTGTG
CTTGGAGTGAGCTTTTGTAACCTCAAACCCTGCCCTAGCGACACACCTCCTTCAACAAGGCCACACCTCCTCCGACAAA
GACACGCCTCTTCCTCCGACAAGGCCACACCTCCTCCAACGACACACCCCCTAATCCCTGCCCATAGCGACACGCCTCCT
CCGACAACGACACACCTCCTAATCCTTCCAAGCCGTTCTCCAACTAAAGGCTAAGCTGCAAATATCTGAGCCTGTGGGGC
CTTTCTCATTCAGACCCACCCCAGCACACCCTTCCAGATGAGCCTTGGAGGGTCCATGGCGCAGGCCAAGTCTCAGGCAGCT
GTTGCAGAGCCGTAAAGTGGGGAAGCCCCTCCTCACACTCCTCCCTGTGTCTCCCCACAGAATGTCCGCATAGACCCGAG
CAGCCTGTCCTTCGGGATGTGGAAGGAGATCCCCGTCCCTTTCTACTTGTCTGTCTACTTCTTCGAAGTGGTCAACCCAA
ACGAGGTCCTCAACGGCCAGAAGCCAGTAGTCCGGGAGCGTGGACCCTATGTCTACAGGTGAGGCCAGGCAGGGTGGGGT
GGGACTGTGTGTGGNNNNNNNNNNNNNNNNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNAGAGGCGGTGTCTGTCCGAGGCG
AGTGCATACTCGCATGCGTGTCCTGGGGTGAGGGCTAATGTGAGTGTAAATGTGTTGTCATGGGGGCGGGGCACAGAGGA
CCCAACAGCGCACAAAATGGGCAAAATGGCTGCTGTTGAGTCAGAAAAGTCAAACAAACATGCAGAAGTGAAGCCAGTTA
CGCAGCATCTAAGAAGTGAGCGGTGCTGGGTAGTGGTGGCACACACCTTGAATCCCAGCACTTGGGAGGCAGAGGCAGGT
GGAATTCTGAGTTGAAAACTGGATCCAGGAGCGGCTGCCTGGTCCTCCACCTTGGACAGAGAGTTTTAACTTCCATAATG
CCTATTTTAGCCCCATCTGTGAGACAGAGGTAATGGTACCCACTGTGGGTAGGTTCAAGGATAAGATGAGATAGTTAGTT
GGAGCCACTTAAGCTCTGTCTGCCATGCCATAAACTCTCAGTAAACGCTGCCTGTGTGGATATCTGCTCCATGCTGGTTG
TCAAGTGAAAGGGAGACGTGGGGGGTGGGGGCAGCAGTTGGAGTTGTTTTCCAGAGAGGCTCAAAGAGCCTTATGGCTCA
CCAGGGAAAGCAGAGAAGAGCTGATTGGTGGTGGCAGTGTCTTGCCCTGGACAGACAGCGAGGATATAAACAAGAGGTGG
CAAGCTTGATTTGGTGGCCTTGGTGCTGTTGGGTATCAGTGTACCATCACCCCAGGGCATGCTGGGAGATGATGGGTGCT
TTCATGTGACCTCACAGAGCCACAGCTGACTCAATGCCTTTTTACAGGGAGTTCAGACAAAAGGTCAACATCACCTTCAA
TGACAACGACACCGTGTCCTTCGTGGAGAACCGCAGCCTCCATTTCCAGCCTGACAAGTCGCATGGCTCAGAGAGTGACT
ACATTGTACTGCCTAACATCTTGGTCCTGGTGAGACTGTGGCCCTGTGTCAACCCCATGCCAACCCTGCTTCCTCCCAGC
TTAGCCTTCAGGAGTCAGAGAGCAAGGGCCACCTCAGCCCCACTGTCTCCATGGTGACCATCCCCTCCTTCTCATTGCCT
TACCTCCCATCTGAGACTCCGGGACCTCCACTGTGGTCCCTGGGGAAGCATGAAGGTCAGAGCCACTGGGGGCCAGAGGG
CAGGTGGGAGTCCGGAAGAGCAGGCATTTATTGAGCACACTGTGTGGGTCGCTGAGTCTGTCCTTTGGTGTATGCGGGG
ATCTACTCAACCCTGCTGTTCAGGGGAGGAAGCAAAAGCCAGGCAGAGGCAGGGAATTTACACAGGGGCCAGAAGGACCC
TCCCCAAGGACAAAATCCCAGATACCAATTGGGCCAGTGTTACCAAGGCAGGATCGGATCCTGTGGTCAGACATCACGGC
CCTCGCTGGAAGTTTGAGGACAGGGTACACGGTGGTTGGTAGTGGGGGGACAGTCAAGGCATGATGTCACGAGGTAAGCA
TTACCATTCCCAGTGACACTAACTTGAGAGATTGGAACTGTGAGCTTTGTGTGATTTTCATGCTTTTTAGAAGATTCTTC
TGATCTCCCCCCACCCCCACCCTCAACCATAGGAAACTGTTGAAAACACCAGCTTTCCTGCAGTTGATGCGGGCCGATGC
TGACTGGATTGGTCCTTTAGCCACCTTGCCTCGAGTCCATTTTCAGAGGTGTCCCCGGGTCCGATGGGGCTGGAGGGAC
ACTTTTTGTTTTTATTAATTTATTATATTACTACTATCATTATCATTATTTTGAGATAGGGTGTTACTGGCAGCCATAG
CTGCCCTGTATCTCATTATGTAGACCAGGCCAGCCTTGAACTCACAGAGATCTGCCAGCCTCTGCCTCAAGTGCTGGGTG
TGAGCCACCACATTTGTCATATTAATTCCAGTTTTTAAAAATTATTTATTTTATGTATATGAGTACACTGTAGCTGGCTT
CAGACACACCAGAAGAGGTCATCGGATCCCATTACAGATGTTTGTGAACCACTGTGTGGTTGCTGGGAATTGAACTGAGG
ACTCTGGAAGAGCAGTCAGTAGTACTTTAACCACTGAGCCATCTCTCCAGCCCTTAATTCCATTTTTGATATTGAAAAAG
CAACAGCATTCTGTGCTGGGCCAGCAGGATGGCTCAGTAGGTAAAAGGTGCCTGTCACTAAGTCGGATGACCTGACTTTA
ATCTCCAGGACCCACATGACTGCCTCACATTGTCCTTTGACCTCTATACGTGCACTATAGCACCCCTCAATAAATATAAT
AAAAGCCCTGTGTTGAAGGAGACCTGTAAGTGTTGGTGGGTAGGCATCATGCGCTCGCTTGATGGAAAGAACCTGGAGAC
ACCAGCCATGCGTTGCCCAGGAGGTGGAAGGTGGCTTTTGGTTGAATGGCCTTTGGGAAAAAACAAGAGCCCAGATGCTC
CATACGGAGGAAGAACAAACAAGAAAACTATAAACTCAACTGAACTTGGGGCAGTTAGACCCTGGGGGGGGGCTTCGGGGG
AGTAGATATTCTGGGCTCCCAGTGTTGAGGTGGGGCAGGGACATGAGAAGTGAGGTGGAATCCCTGTGAGGAGACTGCGG
CAGGAGAATGCTGATTTCCAAGCCAGCCCAGATTAATCATTGAGACCTTGCTGTAAACATGTTCAAAAGGAGGCCACGGG
GAGTCGGGGAGGCTTTCTCCATATGCTGTGTTTCTAGGCTCATGTTACTGATAGAATAGGGTTCACGTCCTAGCTATAAA
CCGGGTATCCAAGGGGCTGGCGAGATGGCTCAGCGGGTAAGAGCACTACTGACTGCTCTTCCAGAGATCCTGAGTTCAAA
TCCCAGCAACCACATGGTGACTCACAACCACCTGTAATGAGATCTGACACCCTCTTCTGGTGTGTCTGAAGACAGCCACA
GTGTACTTATTTATAATAATAAATGAATTTGGGAACAAGAAGAGGCCCTAAAAATTCAATTCCCAACAACATGAAGGCTC
ACAACCATCTGTACAACTACAGTGTAGTCATATACATTAAAATAAATAAATAAATCTTTAAAAATCAAAAACAAAACAAA
ACAAGGTATTCATATCTTTGCTGGCCCCCTTCTGTGTTAGGTTTTCTCTTAACCTTGGCTCCAGCCTCATCCGTTCGCTT
GGTGAGGGATGGCATGGGAGCCTCCTTCTCTGTGCCCCGGCTCTGCGGAGTGTCTCTGGTGGTTCACTGGTTCCATTTAG
GCCTCAGGTGTGGCTCTACAGACACAGTGGCTCAGGAAGGCTTCCGGAGGTCAGGCCTGAGCTTCGTGTATGCCTCTGAG
TCCTTGTAGAGTTCACTCAATCTGAACCCATCATCGGGTGTTCTCGAGAGTACAGGTTACCCTGGCAACAAGGACCAGAG
AAAGGTCCATCCTGAGGGCCTGAGCTCTTTGCCTGAAGCCTGGTTGAAAGTGGGGGATGGTGTCCTACCATGTCCTTTAC
CCTAATCCCATGTCCTTGGCTTTCCAGGGGGGCTCGATATTGATGGAGAGCAAGCCTGTGAGCCTGAAGCTGATGATGAC
CTTGGCGCTGGTCACCATGGGCCAGCGTGCTTTTATGAACCGCACAGTTGGTGAGATCCTGTGGGGCTATGACGATCCCT
TCGTGCATTTTCTCAACACGTACCTCCCAGACATGCTTCCCATAAAGGGCAAATTTGGCCTGTTTGTTGGGGTAAGTGTC
TTCTGTCCCTTCAGAGAGTCAGGTTATCTCTCAAGGACCCTAACTCAAACCAGCTTAAACAAAAATTGGGAAATTATCAG
CTCATATAACAGGAGATGCCAGGATTACATTCAGGTGCGGCTTGATCCAGTTCGAGTGGGGGGGATATGGCTGGGGTTTAA
CTTAGAAGATTTGAGGCACAGCTTGCGGGGAGGAGGAGGAGGACTGGATCTTCGAAGCACACCTCTCAAATGTCCCCATC
AGTGCGACTTAGAGGCACCCACTATTTCTCACAGTTGACCTTTGGTAACCTGAGCTAAGGTTTGGTCTCTGACCCCTGAC
CCCAACTCTCTGGTTCAGCTCAGCTTTCTCTGTGGGGATCTACTGACGGGGTGTGCTTGCAGTAACGTTACCCACACCCT
CCAGGACCTGTTATTACTGAGTTCACGTGAGATTTACTACCCAGTGGTTAGAGCACTGGCTGTTCTTCAAGAGGACCCAG
GTTCGATTCCCAGCACCCACATGACGGCTCACACCCCATCTGTAGCTCCAGTTCCAGGGGATCTGACACCCTCTTCTGGC
TCACTCAGGCACTGCAGTGCAGGCAATGCACAGACCTACATGCAGGCAAAAAGACTCAGACACATAAAATAAAAATATGT
CCAATCTTGTGGAGAGGAGACGGGGTAAGGCACTGTGTCTCCCAGTGACCAGATGGGGACCCCATGGGGTGAACACATAA
CGAGGGCGGCAGGCTGAATACCCAGTCTTAAAGCTTTTACAGAGTTAGAGGCCCTGGGTAAAGCCCTGTACCTGTCTGTA
```

```
CCTGCCCTTCCCAGTACCCCATAGAGACTGCCATCCCTCTCTTCCTGACAATTGAGGTGGGGCCCTTCTACTGAGCTTCT
AGATCGTTCCTGTCAGAAATTTGTATTAAATTCTTTCTTTCTTTTTATCTTTGTTTTATTATTTTTATTGAGTCAGGGTC
TCTCTATGCAGTCCTGGCTGTCCTGGAATTCACTTTGTAGACCAGGCTGGCCTCAAACAGATGTCTGCCTACCTCTCTCT
CCTGAGTGTTGGGAGTAAGGGTGTGTGCCACTAAACCCCGCTAAAATGCCTTTTTACTTTATTAGCTGTTGGCCCTGAT
TGAGTAGACTTGTCATCTGTGTACATGGGAGTATGAGGCAGGAGTATGGCAAATTCAAAACCAGCCTGGACAACCCTGTC
TGAAAATGTGCAATAAAAAGAATGCTGGATGGAGGGTGGAGCTGGAGAGATGGCTCAGCAGTTAAGAGCACTGACTGCT
CTACCAGAGGTCCTGAGTTCAATTCCCAGCAACCACATGGTAGCTCACAACCATCTGTAATGAGATCTGATGCCCTCTTC
TGATGTGTCTGAAGAGAGCTACAGTGTACTCACATAAAATTTTTTAAAGGGTGGGGGGAAGCTGGGGGTGTGACTCAGTG
GATGAGTACTTGCCCACATGTGTAAGGCACTGGGTTCTGTCCCCATCATTTATGCAGCAACAGTGAGGGGACATACCTAT
AGCTGAAGAATAGGTGGGCTTGTGCCTATGTTGTGATCTATGCCCAGGGCTAAAGGTGCTGTTCCCTGCCTGTCTGTAGA
TGAACAACTCGAATTCTGGGGTCTTCACTGTCTTCACGGGCGTCCAGAATTTCAGCAGGATCCATCTGGTGGACAAATGG
AACGGACTCAGCAAGGTGAGCAGGAGGGCAGACAGTCCCATCGATTGGTGTGGGGACTACACCAGAACAAGCCTTGGCG
GAGGGTGTCCGGGGCACCCGAGGACTTCACGGATCCCACAACTGTCCCTGCGGTATTTCTGTCGGGAACTCTTTTCTGTT
CCTGAGTTGTCATTTCTAAGGCTGACAGGAAGACATTCCATAAAGATAAGACAATGAGGTCCAGCACCTTGCCTAGGCC
CACCCGGAAATCCCCCAACACTGCTTATAGAACCCAGCTTCCTGGCCATTTACCCACCACCACGCCTCTGTGTACCCAGA
GAAATGTGTTTTCCTTCCTTCTTCGAATACACAGAAATCCTGTCATGTGCTATTTTGCTGCTGGCTTTTCTCCTTAGGGG
CATCTTTATGGATAATGTGTTTTTGTTCTGTTGTGTTGTCCAGAGGCCCAGGTACTAAAGATGGAACGCAGGCCATGTGA
ATCTTTGACAAGGACACCACTGCTGAGCTAGACTTCTGCCCTTCACTGGGGGAATTCTAGGCAGGGGCTCTACCACTGAG
CCACGCCCCCAGCCCCTCACTGGGGGATTCTAGGCAGGTGCTCTACCACTGAGCCACACCCTTAAATTTTTTTATTCTGA
AATAAGGTCTCTGTGTATCCCAGGCTAGCTTCAAAAATTCAATCCTCCTGTCTCTGTTGCCTTGTAAATGTTTAATTCAT
AGATGTGTGTTACCACACCCAGCCAACAGCTATTCTTAGTGGGTGCTGTTCACATTAACCAGCTGCTGTTTTATATGCAT
TGGCATTTTGTCTGCATGTATGTCTGTGTGAGGGCCTTGGATCCCCTGGAACTGGAATTATAGACAGTTGTGAGCTGCCA
TCTGGGTGCTAGGCATTGAACCTGGGTCCTCTGGAAAAACAGCCAGTGCTCTTAACCACTGAGCCATCTCTCCAGCCCCT
AATCGTCTCCTTTCTACTATGAGAAGGGCTGATTGAAAGCTCTTACGGCAGAGTGGGAAGTGCGTTTAGTTTTGCTGTTG
CTGTTGCTGTTTTGTTTTGTTTTGTTTTGGAAGCAGGGTCTCAGTGTAGTCCTGGCTAGCCTGGAACTCGATATTGTACA
GGTGACTTAACTGAGTCCCCACAATACTGTATGTTCTGTCCCCACTGTGGGGAACAGGAAGCTGGGCTAGAACCTCTGTG
CACACAGCCTGCTCACGGGCTTGGATATGATGATGTCTGTACTAGGGCACACATTGGGTGTAGATCTTGGCTGTCCCTCA
AGGCTATATCTCATCCTGTCCTGCAGATCGATTATTGGCATTCAGAGCAGTGTAACATGATCAATGGGACTTCCGGGCAG
ATGTGGGCACCCTTCATGACACCCGAATCCTCGCTGGAATTCTTCAGCCCGGAGGCATGCAGGTAAGCCCTGTGTAGGGA
CTCCCTGCCTCCTACCAGGAAACTCTGCTTCTGAGATGGTTCAGGGTCCACTCAGGTAGCTCCCTGGAAGTGTGCTCAAA
GTGTCTGGCCTTCTGTGTACTATGCCTCAATGGTTTTTCTTGTCACTGTGACAAGATACCTGACAGAGGTGACTTGCGAG
GTGTCTGGGACTCAGTCATGGTGGCAGTGGTGAAGGGTACTGGTAGGAAAGTGTTAAAGTCTTGTAGCTTGGGCTCCAC
CATCCCCAGGGTCCTAACTGTGGCCTAGGAAGACATGGTGGGTCTTCAGAGACCCATTTCCAGGTGGTGCCCTAGCCTGT
GCATCCATTCTGGCCACACCAGAGATGACTCTGGCCTGGGGACTCACTGGGCTGATCTTTGAGCCCTCTTCCTCTCTAGC
TCACACAGGCAGCCATGCTTCATGCCAGGTGTGAACACAGCCACGACGGGGTGAGCAAAGTCTGTGTGCTCTGGTGGACT
TTCAAAAGCAGAACTTCGGGTTTGGTTTTGTTTTGTTTTTACATTTTATTTATTACTGGTTTTTTAATTTTGTGTATCTG
TGTGTGTGCACATGAGTGCAGGTGCCTGAGGAGGCCAGCAGAGGGCGTCAGATCACCTGCAGCTCCTGTAAGAGGTGGCT
GTGAGCTGCCCTACGAGGATGCTGGGACTCAAACGTTTTGTTTTGTTTTGTTTTGTTTTTTGAGACAGGGTTTCTCTGTA
TAGTCCTGGCTGTCCTGGAACTCACTTTGTAGACCAGGCCGGCCTCGAACTCAGAAATCCGCCTGCCTCAGCCTCCCAAG
TGCTGGGATTAAAGGTGTGCTGCGCCACCACGCCCGGCTGCTGGGACTCGAACTTAAGTGTTGCTCTAAACTACTGAGCC
ACCTCTCCAGCCCATTTATTTGTGTGTGTGTGTGTGTGTGTGTGTGTGTGCTGATAATGGAACCCAGGGCCTCACACACT
CTAAGTAAGTGGCCTGATACTGAGTCATGCCGCAGACACTAAATAGGATTTCAGCCTGGCAGAATTATATGAGAGCAGGG
TGCAGGCCAGGTGTCTCTTTCCTTCAGTCCTTCATGGGTTATCTCCAGAAACAAAGGCCTCTTCTTACACAATCACAAG
GATCGAAATCAGAGAATTTAACATCGCCCTAATAAGAGCCTCTAAGCTGGTGCTGCTGCATAAAATAACAAAACAAACTG
TGCTTATAATTTCAGATTAGGGGCCATCTTTGTGGCTGGTGGTGGGGGGGAGATCAAAGCAGGAACTCCAGGCAGCTAATC
AATCCACTTACAGGAACAGAGTCACACTGAATGCACTCATGCCTATAGCTCAGATGGCTGTCTCCATTCTTATACAGTCC
ATGAGCCAAACCTAGGGAATAGTGCCGCCCACAGTGGGCTGGCTCTTCCCACATCAATTAGGGGAATCCAGACAATTTCA
CACAGTGAGAGTCTCTTCACAGGAGATTCTAACTATGTCAAATTGACATTTAAAAACTTACCACCAGACTGTGTGAAAAG
ATTTTCTGAGAAGAGCTTTTGCTTAGTGTTGGGTCTTAGAAATCTGGGTGTCGGTCAGGTGTGGCAGTGCTTGCATTTAA
TCCTAGCACTCAAGGCAGAGGCAAGGGAATCCTTGGGAGTCTAAGGCCAGCCTGGTCTACATAGCTATTTCCAGTTCTTT
ACATAGAGAGACTATAGAACTACATAAGGAAGGGGCAGAGGGAGGGAGGGAGGGGGGAGAGAGAGAGAGAGAGAGAGAG
AGAGAGAGAGAGAGAGAGAGAGAGAGAGAGAGAGAGAGAGCGCCAATGTCTTATCCTCTTTTTTTTGGTTCATCCCAATTGG
TTTCCTCTTGTTTTGCTTTGTTGTTTTGAGTTGGGGGGGGGGTCTCACTGTGTAGCCTGGCAAAAAAGTATCCTCCTGC
CTCAGCCTGCACAGTCCTAGGATTGAAGCCATGAGCCACAGACCGAGTTCTGCCCTGGACTCTCTTTGTTTTGAGGATTG
AGCAGACATGTGTGTGTGTTATGGTGTTGACCATGGACAGTGTGGGTCACCCGGGCCTTGTTTCCCATCAGAAGAGTGTT
TGAGGCCAGACAGTGGGAGTGAATGGATGAAGAGGAGTCAGGGTGGACAAGGGAGACTGGGAAAGCCGAAGTCTGGGCTG
AGGGAGAGGTGGGATCCGGAGGTTCAGGGGCAGGAAGGGACATGACTAAGGCCCTGGCAGGTACAGTGAGACAGGAAGTG
GAGAGGGAAGCTGTGGATGGCATCTGTGGATGGCATCGCACTGCAGGGTGACTGCAGGGCCATGGTGGTTGCTCATCCCT
CCGCATGCACAGAAACCATTGCCCAAAGCCTTGGTTTCCACAGTCCTCATCAGCTCTCAAGCCCCTATCAAGAGAACAT
GGCACATCGTTGTAAGTGTAAGGGAACTCTGTAAACTTCAGATTATCTCAAGTGACTGACACAGCTACTGCAGACATGTG
CTGTGCATGTTTTAGCTTGCTTGCTTTCTTTCTTTCTTTCTTTTTTTTTTTGTGTGGTTTTTCGAGACAGGGTTTCTC
```

```
TGTGTAGCCCTTGCTGTCCTGGAACTCACTCTGTAGACCCAGGCTAGCCTGGAACTCAGAAATCCGCCTGCCTCTGCCTC
CCAAGTGCTGGGATTAAAGGCGTGTGCCACCACTGCCCGGCTGCTACCGTACTGTTATGAATCATAATGGAAATGTCTGC
GTTTTCCAGTGGGCTTTTGTGGGCGGTTACGACCCACAGGTTGAGAACCTCCGGCCTAGGGCATGCTGGCCAAACTGTGC
TAAACTGTGCTGAGTTCTGGTTTTTAATTTGCCTCATTGGAAGGATGGATTAGTGAGTGGGGAGCCAAGGGAGCAGGCAG
AACATTGAGGGACCGCTGAAGTTTCCTCAGCAACAGGTGACTAGTGTCATTGCTGGTCCCTGTGGTTTCTTCTGCAGGGG
TGCCCCAGCCTCTGTGCTCAAGGCACAGTTCTCACCCTGCCTCCCCTCTGCAGGTCCATGAAGCTGACCTACAACGAATC
AAGGGTGTTTGAAGGCATTCCCACGTATCGCTTCACGGCCCCCGATACTCTGTTTGCCAACGGGTCCGTCTACCCACCCA
ACGAAGGCTTCTGCCCATGCCGAGAGTCTGGCATTCAGAATGTCAGCACCTGCAGGTTTGGTGAGTGTCCTCTAAGTGTT
CTCCACCTCTAGGCTGAGGTGAGAGGGTGGTACCGGGATTTTATCTGCTGCCAGCTTGCTGTCCCATTAATCTCTGGTCT
CTGCACCCATGATGATCTCAGTGCTACAGATAGGGAGCTAAAGGTAACTGGAAAAAAGATAACAGCCACCCTGATAAATG
TCCCACCGGCACGCGTTATCCACAAGCCGTGTCGCTCCGAAGTCCGAAGGGTCCTGCCCCGAGGTTAAGATTCCATCAGT
GGGCACTGCTTCTTGGCCAAGGAGAGGAGCCTGCCCTCATCCTTTCCTGCTTCCGGAGGCACCACATTCCTTGGCTTGTG
GTCCCTTCCTTTCTCAACCTAGCATCCAGCTTGAGACTGGGTAGCGCGTTGTGGCCCAAGTTAGCTTCAAACTTGCGCAT
CCTTCCTGCCTCTGCCTTCAGAGGTATCGGGATAGAAGGCATGTACCCAGACACCAGATCCCCCCAAAACACTCCGTTCT
ATGACCACCTCCTCCTCCTATCCAGCCCATAGCTTTCAGGACCCCAGAGACTGACTGGGTAGAGGCCACTGGGATAAC
CTGGTCTGTTATCCTGGTGGCTCTAACTTCTTTGCCCACTAGGACGAGGCTGGAAACATTCCCTGTGGCCATTCACCTGC
TGGGAGCAAAAGCTAGAGTCCTGCCCTTCACTCAAACTGGCCACGCTCCTATTGTGTCAAACTCTCCTCTTTAGCCAAGT
TGGGTGTGAATCTTGGTACTGGCTGTGGCCCCCTGGCACATCTGCTCTGTGTTCTAATCCTGGGCCCTACCCTGCTGCTA
TTGCTCAAGTCTGCTCCGTGCCTCACCCCTCACCTGGCAGTCAGGCCCAGGCTGCTCTAAGCACCTCCCACACAGTCTTT
CTCCTTCCCACCCACCTTCTTCCCGTGTCACTCGGGTGCCCTTGCTCTCGCCAGCTCGTATTGCACCAGATGTCAATC
AAACTCAAATGAAATGCTTCCTCTGATCCTGATCAGATCCTCCCAGACACTCTCCCTGATCTTCCGTCCCCTCTGGCTCT
CCAGCCGCTCTCTGGTACACTCTCTCTTATGCCTTCCTGTTGCCTGCTCATGGCTGTCGATGTTATTTACTGAACCCTCC
CCTGCTTGTCTCCTCCTTGGACTGCAAACCCTGTGGCCATGTCCCAATAGGATACTGGGAGTCAAACTTCTAAGGCCCCT
ACACAGACGTGGGATCCAGGGCTTGCCTGCGGACAGGAAGTTTCACTCACAACTGCACCTCCGCCTTCCCCTCCTTGTAC
CAGCAAAAATCCCAGGGCTTGGTTGAACTGGTCCAGTTCTGACCTAGTCATTAGGCTGTGTTAATTGGCCAGGCTGGGTC
CCACACACCGCAGGGGCAGCAACAGGCCCACATCACACAGAACTCTGCTGTCAGGAACAGAATTTCAGCTGTGAAAGAGA
ATCTAAGTGTAGTTGTGTCTGTAGCTTGTTCTTTTGTTTTGTTTTGTTTTGTTTTCCGGAGCAGTGGGTTTAAGTATGC
GTGTGCGTGTCGCACACGCGTGGAGGGAGGTATAAACAGGTGCATCGATGGATGGAGACCGGAAGTGAGTGTCTTCCTTC
ATCACTCTTTGTTTGTTTGTTTGTTTGTTTATTGATGCAGATTTTTCAATCACTAATCCTGGATCTTGGGAGGTCCAGGG
ATCCACCTGTCTCTGCCTCCCAGTTACAGATGTGGCGCTGCTGGGAACCCGAATTCGGGTCCTCACACTTGCACAGGAGG
CTCTTAACTGACTAAACTGTTTTCCCAGCCCCTAAAGCTTGCCCCTTGTAAATCGGGTGAGAACAAGAAAGGGGAAGAGG
AACAGGGATTCGAGTGATAGGGAACACGGCCATGTGACCAACAACCCCACAAAGAGGCAACTCTTACCACACCCAACATC
TCCCCTTCCTGCTCCTCCTCCTCCCCACTGGCCTGACTTTTGGTCTCTCGGCTCTCTGCCTGGCATTACTGTTTACATAGATG
GAGTCACAACTGTTCTTTCTCCCTCGATCCCTGATGACTCCCATGATGGCTCTCATGATTATATGGTAAATCTGCAAGAT
AGCGGGTGATAGCCTAGATTGTTGGGTGGTGTTCCATGGTGGGGCTAACTCTGCTTGCCTTACTCCTTCCTGGCTCACGG
AGCTTTCTTTTGGCATTTCACCACTACAAGGATGCTTCTAGAACATTCTCGCACCTGCTGTAGGAGGAGTGCACCTGAGT
TCGAATTTCTGTGGCATTTTATCTTAAGATAAAGAATACACATAGGTTTCCCTTTCTGGGTCACGAGACTTTCTTGGACT
CAGGACTCCCCATGTCTCCTGTCCTTGTCTTGGGAGCACCTTGGATCTTCCCTGAAGTGGTCCTTAATCACCACGCGGGT
AATCCTGAGCAGTTAAGTCTGGATACACGGGCCATAAAGCTATAAATTAATTATACTTATTGCCCGGGTGCGTCTCTCTG
AGGGTGTTTGGAAATCAAAAGAAAAGATGAGCCACACCTATTGTTAGAAAGCCACGGGCATGCCACCTGCAGCAGGTCTG
TGCTGGCAGCCTTGACATCCTGTTGGAATCTGTGCTTCCAGGTCTGGTTAGGTGGGGGCTTCATCTCCCGGGGATCCATG
GCATCCCTCTGAAAGAAATGGGGTTCTCTCTGATCTTTGCTGGTGTCTCCTTTCACAGTATGGTTGGAAGGATGGGTAGG
GTTTTCCAAGTAGAAGGCAGCATATCTCACCGTGATTTTAGAAGGGTCCACAGAGGTGACATTAGGCCTCAGGGATTGAT
CAGGTCTCTTTTTCTGTTCTGTTGTATCCTTGGTGTTGTGTTTCCATGAGGGCTTGAAGGCCACCCTGAGGATCCTTCGCC
TGTCTTTCTGACAGGACCAGGTGTGTCTCTGGAGCCTCGAAGGTGCTAGGGTTCACCTGGATTCTCAGTGATCCGGGTTC
TCTGCTGCATCCCTGCCCACGTGCACCTCCTGGCTAGGCCATTTCTCTGCCAAGGCTGTCACAGGTGCAGCACTCTTGTG
TCTGACACCTGCACTTGAAGATGCCCATAGCAGCCGGTTCAGGGGTTGGGAGCAAGAGGTAGGGGTTGGGGGCTTCAAGC
AGTGGATGTCCTGGAGACCAGACTCGGATGGAACTCAGAGATCCACCTGCCTCTGGCTCCCAATTAAAGAGGTATGCTAG
CCTGTCTGGCTTTGGATTTTAAAGAAATGTTTTCTTTTTTTTAAAAGGTTTATTATTTATTTTATTTATGAGTACACCAT
AGCTGTCTTCAGACATGCCAGAAGAAGGCATCGGATCCCCATTACAGATGGTTGTGAGCCACCATGTGATTGCTGGGAAT
TGAACTCAGGACCTATGGAAGAGCAGTCAGTGCTCTTAACCACTGAGCCATCTCTCCAGCCCTGTTTGTTGTGTTTTTGA
GACAGAGTTTCTTTTTGTAGTCTTAGCTGTCCTGGAACTCACTATGTAGGCCAGGCTGGCCTCAAACTCAGAGATCTCCT
TGCCTCTTCCTCCTAGGTGCTGGGATTCAAGGTGTGCACCACCAGACCCAGCTAAAACACAATGCCCCCCCTGTGGATCT
GTTGCCCTGGGAGCCTCTTGTGGCCTCCCCAGGGAGACTCAGAACTCGGAACTCCCCAGGCCCTTGTCCTGAGATCTCTG
GGTTGAGCCACTTTGGCTGGTTGGTGGTAAAGTCCCACCTCAGGGCCCTGGGTGGGAATATCTCATTGGTGCTGTCCCTT
CCATTCCTGTGATGTCAGAGACCCACTCACGTGTACCCCTGAGCTACAATGTCTCCAAAAGCAGTCTGTGTGCCTGGCTT
GACATTCAGACTCCGCTTCCACTCCTAAATTCATATGGTGTTAATTTTCCTTTCCACTTTCATCTCCCATGATTCCCTGC
TGCACCTCCAGACCTGGAGGGGTGGGGGTGGGGGGTTGAAAATCACGTCCAAGCTCTCACCTCTTGCTACACTCCCACCC
GGAATGCCTTTCCCTTGCCGGGTAGCAGCTGCTCTNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNCCACCCAACACCCCACCCACCCCCAA
CACTGTAATCGGCCACTCTGGCTCCATCCACCCTTCCCAGGTCCTGTGTGTGCTCAGCTTGGTCCCCAGTGTTAGCATCC
```

```
CTGCATCCCTGGCACCGAGGCAGGCCAACGTGACGCTCCCGTCTTCTAACGCCTCTCTTCCCCTCCTCCAGGTGCGCCTC
TGTTTCTCTCCCACCCCCACTTTTACAACGCCGACCCTGTGTTGTCAGAAGCTGTTCTTGGTCTGAACCCTAACCCAAAG
GAGCATTCCTTGTTCCTAGACATCCATCCGGTAAGCTCCGTGTCCTGCTAGTGGCTGGGGCCCATTCCCAGAGATTGCCA
CTGGCCGCTGGGCACCCGCCCCGCTCCCCCCAATTCCCCAGGACAGAACGGGTTCCAACTGAGGCAGGGGAGTTCAGTTC
CTTCCACAGCGGCTGCTCCTAGAACTTCAAGTCATCAAAGTCTTACTTTACCCCAATTCCAGAGTTCAGTGATATCATTA
CCTTTTTTATTGGGGCCTATAAAAAGATATAAGATAGTAAATTAATTAAGACCTATCAGCCGGGACGTCTCCTTCACCAG
AGACAGATACCCTTGACAGTGTCAAGACACTAAAGACATCATCGTGGGCCAGTGTCTCACAGAGAAATCAGGGGGAGGAA
TTTGTCCTCTGTTAACTTCTCTCTGCCTCTGGTCACTGTAATATGTGTCCAGTCCAGGGTTCCTGCCCTACGTGACGCCC
TGAAGGCATTCCGTGAAGAACTCTTTTTTGTTTTACTGGGTGGGGATGGAAGCCTGGGTTTTGAACACACAAGGCCCCAG
GCTCTGATGCCGAGCTGTGCCTGCAGCTGGCCTTGAACTTGCAAATCCTCCTGCCTCGGCTTCCAGTGTTTCTTCACACC
TGGCTCACAGCTGAGGGTTATCGCAGAGGAAGGGTGGGCACATGGGCACAGTCGTCTGAAGTCTTTCCCAGGTTGTGCC
TAGCTCCCCAGCAAGGAACTTGACATAGAGGTGTTTTATATCAGGAAAGCCCCTCAGAATAGGGGGCCTAGTTGTCGAC
TGGGGACCTGTCCTGTGAGCCTCTCTCCCTGGGCAGATCCATGCTTCATCCCAGAGGACGAAGGTGTTTGGTATTATTA
ACTGTCTATGCAAATGGCACAGTGAGGCCAACACACTAGCTTAGAGAATGGGGCACCCTCACCTCACCCCCCAGAACTTA
GGTTCCAAGACCAACACTGGGCACCTACCCTGGACGACTTCTAGGGAGGGTTGCCGGGCCCACCCATGGTATGCGTGGTT
ATCACATCCATGCCTCTGTCCAAAAGTCATACCAGGTTTCTGGAGGCCTTTGCCCTGGGGGCAGGAGGGATCTGGGCGTC
CTGGGCAAAACGTGTCCTGGCCTTCTACTGTGGGTGATTACAATCTGAGAGGTGGCCTCCGGCCTCCCATCAATCTTTCA
AGAGTCTCATGATCTGGGCAGGGCTAAAGGCATCAAACGGTGGTCTCTTTTCACCAACCTTTTCCATTCTAGAACTTTCT
TCCTGATTTAAATAATACCTGTGAATTCTCCTAGTGATTTTTTGGTTGTTGTTGTTTTAATTTTATGTATGTGAGTACA
CTGTCACTCTCTTCAGACACACCAGAAGAGGACATTGGATCCAATTCCAGATGGTTGTAAGCCACCATGTGGTGGCTGGG
AATTGAACTCAGCACCTCTGGAAGAGCCGTCAGTGCTCTTAACCGCTGAGCCATCTCTCCAGCGAGATTGTTTTTTTTAA
AGCATCACATTTTAAAAAGAGAGCTGCAAGCAGGCAGCTCTCTGTGGCTACCTCTGTTAGGGAGCGCCAGGATGCTACAT
AGTGGGAAACTGGCTCGATTAAACCTACTCACCCTGGACCAGGCCCTTCCTTTGGTTCCCTGGGCTATTAGGAAATGTAA
ACAGGAGAAAAAAGACATCCTTACTGCGCATGCCCCACACTTGGGAGGCACTGTCTTAGGCAGCTTCGTGAGGGCTGGGA
GGATAGCTTAGTAGGTAGGGTGCTCCCCTGCCCTGCACAGAGCCCTGGCTTCCATCCCAGCACCATGTAAAGTTAGGCAT
AAGCCAGTCCTCAGAAGGTAGAGACAGGAAGATTTGGAGTTCAAGGTCATCCTTGGCTACATAGCAAATTGGAGTCCAAC
CTGGACCACATGAAATCTGTCCCCAAACGAACATGCAGATGTGGGGGGCGGGGGGTGTCATCCCTCTGGCAACAAGTGTC
TAGAGAGGGGATCTTGCGATAGCTGATCCCTTTGGGAGCAGGTATTTCCTGAGGGAGAGCCCAATGGGTGTGTGGGAAAT
TTCTAGAAGGGTGGATGCCCCAGCAGGTATTATCTGGTTTAAGTTGGATAAGAAACCCCAGGGCTGGAGCAGGCTGGAGC
AGGCTGGAGCAGGCTGGAGCAGGCTGGGAGGCTCCCCACAGGATCTTACATCTCCTCACTGTATTTATGCTCTAGGTCAC
TGGGATCCCCATGAACTGTTCTGTGAAGATGCAGCTGAGCCTCTACATCAAATCTGTCAAGGGCATCGGGTGAGTGGAGT
TGGGACGGGAGCTTCTGAAAGCTTCAGGGGCCAGGAGGTTTCATGCATTGGTGATGCAAGGCACAGGACAGGGCGAGGCC
CAAGCGTTGGGATCCAACCACCTCTTTTCCTGGGATTGCATCATTGCTCTGGATGGAGACTCAGACCCAGGCTGCATCCT
GACACTACCTCCATCCTCCACGCCCAAGGGCTCTTCTGCCATTAGTCGCATTGGTAATCTTGTCTCCCCCACAGTAGGAT
GGATTTGCCTGCTTGTATGTCTGTGCACCATGTAAGTGCAAGAGCCGCGAGGCCGGAAGAGGGCATAGGGACCTGGAGT
TAGACGGAGTTGTGAGCCACTGAATGGATGCTGAGACTTGAACCTGGGTCCTGCCAGTGTTCTCAACCACAATTCCCTGG
GTTTACTTTATGTTTAGCTTTGTGTAACGTACAGAGTGAGACTGAGAGTCCTCCTGCCCCCATTCCCTGCTTGCCCGGCA
GAAACATGGTGGTAGGAGTCCAGCCTTGGGGCTGGAGAGATGTCTTAGCAGTTAAGAGCACTGACGGCTCTTCCAGAGGT
CCTGAGTTCAATTCCCAGCAACCACATGGTGGCTCACAACCATCTCTAATGGGATCTGATGCCCTCTTCTGGAGTGTCTG
AAAACACTAAAGTGTACTTATGCAAATAAAATAAATAATTCTTTGAACGAAAGGACCCACATGCTGGTCACTCCTCAGCA
TCGCAGATCACATGCCTGGGAACCACTGAGGAAGAGGTGGACTCTGGGCTTCATGGTGAAAACTAAGTCCGGGAATTGCT
GGACTCAGCTAGGCTTCTGTACACACACACACCCTGGGACTCCTGCATGCCAGAAAACACTCTTTCACTGAATTACATCC
CAGCCCTCTTCACTTTTTAGGTCTCACTAGATTACCCAGGCTGCCCTTAATCTCTCTGTAGCCCATCATGGCCTTGAACA
TGCGCCACTTGATCTGACTCATCTGGGCTCTTGAAACCGGAGGCCAAAGTGGGCTGGCCACGGGCTTGAGTTCCTGAGGT
CAGCAGCCAAACATTGATTTGCTCCACTGCCTCAAATACTCTTTAGAAATCTTTATAGATTTATGGAGAGGTGTCAGAGA
CGCAAGCAAATCTTTGTTCTCTGTCCCCGGTGCATTCAGTACTGTCCGCTCCTGGCAGTATCTGTCACGGTAAGGAACCA
AAGTCTAGTGCAGCGTTATTGCCACAACTGCAGCTTCTCAGCACGAGATGCCGCCCGCTCACGCCTCCCGCCCGGCTCCC
GTGGCTGTGATGGGTTGTTTTGTTTTGTTTTTTTCTTTTAATCTTTGTTTCTTGTTGGTGTTGTGAGTTTTGGGATTTTG
TTTTGGTTTTTGGAGGGGAAGGTGGTTCATACAGAGGCCAAAAGAGGATGCCATGTGTCCTCTTTTATCCCTCTTCCCTT
TCTTCCCTCGCGGCAGGAATTCTCACCGAAGCCCCAGCTAGCTTCTACCTGTCTCAGCCCTGGGGTCACAGGTTCAAAGC
CACACACAGCTTTTAGCCTGGGTTCAGGGGTCTCACCTCGGGTTCATTTGCTTGAGCAGCAAATGCTTTCCCCACTGAAC
CATCCCCAGCACAGTGATAGTTTCTCCTCTAGCTTTTGGGGTGTTAAAGTACTTGCTGGACCTGGGTGTGTGTGGTGCGA
TCAGGTTCCCCTGTAGATAGTAACAGGGGCAGCCACCCAGGCTTTGGTGGGAGGAGACTCATTATTATTTTATTATTATT
GGTATTATTATTATTATTATTATTACTACTACTACTACTACTACTACTATTTGGCTTTTTGAGACAGGGTT
TCTCTGTGTTAATAACCTTGGCTGTCCTGGAACTCACTTTGTAGACCAGGTTGACCTCAAACTCACAGAGATCCACCTAT
CTCTGCCTTCCCAAGAGCTGGGATTAAAGGCATTGTGCCACCACATACACAGATATTATTATTTATTTTTAAAGTTGTAT
TTATGTATTTTATGAATGAGTGGTCTGTCTGAATGTACACCTGTGAGCCAGAGGAGGGCATCAGATGCCATGATAGATGG
TTGTGAGCCACCATGTGGTGGCTAGGAATTGAACTCAGGACCTTTTAACCGTTGAGTTATCTCTCTAGCCCCATTTTTAG
TTGTGTTTAGTGTGTGTGTGTGTGTGTGTGTGTGTGTGTGTGTGTGTGTGTGTGTATAGGTGCATGCCACAATGAACATGTG
AAGGTTGGGACAACTTTAGGGAGCCTGTTCCACCTTCTGGGTCCCGGGGATGGAACTCAGGTCTTCAGGCTTAGCAGCAA
GCACTGTCACCCACTCAGCTGAGCCTTGCCACGCCCCTTAATTAATTTTTTGCATCAGGGTTTCCTGTAGCCCAGGCTAG
```

```
CCTCAAACTTGGCTAAGGATAGTCAACTGTACGTAGCGTTACAGGCTCCCCTTTATCTTTCTGTCTCATTTTCTTGTGAA
GTGGTTTCGACTGAGCGAAGGCCTTTCACATGGTAACCAGTAGCTCTCCCACTGACACCCCAAGCCCCAAACATAGCTCC
ATTGCGAGGGTCACATCACCCCTGGTCACTCACTTTGTGTCCCATGTTTCCAGGCAAACAGGGAAGATCGAGCCAGTAGT
TCTGCCGTTGCTGTGGTTCGAACAGGTGAGTCTATGAAGGTAGAAGGGAGTCTGGGGTCTCTGTATGTTAGCTGAGGCTT
TTAGTTCGCTTTGATGCTAATGTTATGTTAACTTCCGGTACCTAAATTTAGCTATTTGTATGTTTAGCCTGCCCATATGT
ATGTATGTATGGTTATCATGTGAGTGCCCGGTACCCGCAGAAGCCCAGAGAGCATCAGGTCTCCTAGAACTGGAATTACA
GATGGTTGTGAGCCGTCAAGTCTGAAACGTGGGCTCTCTTGAAGAGCAGCCAGCGCTCTTAACCACAGAGCCATTTCCAG
GTTTTGTTAATGGCCCCTGAGAGGGGGGAGTGGAGATGGAATTCCGCACCAGGCCACCCTGCAGGCATCCTGATCTCGTC
GCCCTTCAGGTCCTAAGACTAGATTCTAACCAGCGAAAGAGATGACTGTTAGGCGAGGAGGCTTGGGGCCAGATCCACCT
CCCATTAGCCAGTGCCTCATTTCATGGGCGTGCCAACAGCAAGGGCGTCTGGGAAATGTAGTTCTCTTTGGACTTCTAGC
CAAAGCGCCCGAGGCTCTCTGTTCTGATTTGGGGCGGGGCACGGCACAGTGGGATGGGTGCCTTGGAGACGCAGCGTGCA
GCCCGACCTTTCTTCTGCAGAGCGGAGCAATGGGTGGCAAGCCCCTGAGCACGTTCTACACGCAGCTGGTGCTGATGCCC
CAGGTTCTTCACTACGCGCAGTATGTGCTGCTGGGGCTTGGAGGCCTCCTGTTGCTGGTGCCCATCATCTGCCAACTGCG
CAGCCAGGTAAGTAGGAGGGGCGGCCACGCCTCGGACTCGGCTCGGGTTTCAGCCGACCTCTGTTCCTGCAGCTAGCTC
TTGTTCTACCTCCTCTCTCGCACCCTGCAGTAACTTTTCTCTACAAGTCCTGGAAGGCCCAGGACCCTCCCAGGTCGGGG
CTAGGCTGAGGCTAGGTACCCAGTCATTTGGCCAACAAGTGGGTGCCACTTGGTGCCCGCTCTGGAAAGTGCCTGCCGAA
GGTAGAGGTGGGGGGGTCAGGGCTGGGCTGGTTAGAGAAGCAAAGGAAAGCCAGGTTAAACCAAACAGAGAAGATAGTTTT
TCCCAAGGAAAATGCTCTGGGCTGGAGTTGGGTGTGAGATGGAAGTGGGAGGAGCCACGTGTGGAACCATGAACTTAGAT
TTTCTAAGTCCAGAGAAAAAAGTGAAAGAAATTAGTTATAAATTTTGCTTGACCCAAATATATCAGCATATGAGGCTGAA
GTTTTACATCAGCCATAGAGCACTTGTTTAGCCAGTGTGAGGCCTTGTATTCAGGCCTCAGCGCTGACAGGAAAAAAAA
TAATCAAAAATAATGAAAATATTTAAAAAAGAAGAAGAAAGAAAGAAGGTCTGGAGAGATAGCTCAGTGATTATGAGC
ACTTACTGCTTGCAGGGACCCAGGTTCGATTCCCAGCACCTACCTGGTGTTCTGCAACCAACTGTAACCCTTGTTCTGTA
ACCCTAGTTCTGTAACCCTAGTTCCAGGTGATCTCAAGGCACACACAGTGCATATACTTGCATGGAGAACACTCATAC
ACATAAACTAATACAGGTGCCTATAATCCCAGCACTCAGGAAGGAAGGAGGCCAGGGGAGAAGGATCCGGTTCAAAATCA
TCCCTGGTTACATGTAGGGTTTGATCAAGGCCAGCCTAGGATACGTGACATCTTGTCTCCGAAAATCAATTAAACTTCAA
AAAAAGAATAGTGTGGGCGACTGGAGAGATGGCTCAGCAGTTAAGAGCACTGGCTGCTCTTCCAGAGGTCCTGAGTTCAA
GTCTCAGCAACCACATGGTGGCTCACAACCATCTGTAATGGGATCCGATGCCTGCAGAGCAACAGTGTACTTGTATACAT
AACATAAATAAATAAATCTTAAAAGACAAGAATAGGGCTGGTGAGATGGCTCAGTGGGTAAGAGCACCCGACTGCTCTTC
CAAAGGTCAGGAGTTCAAATCCCAGCAACCACATGGTGGCTCACAACTATCCGTAACGAGATCTGACTCCCTCTTCTGGA
GTGTCTGAGGACAGTTACAGTGTACTTACTAAATAAATAAATAAATAAATAAATAAATAAATAAATCTTTAAAAAAAAAC
TTTAAAAAGAAAAAGACAAGAATAGTGTGGTTCAGACAGGGGTAGGGGTCACTTCCATAAAGAAAGGATGACTGAGGAAT
GTAACATCAGGGCATGCAGCCCCTGAAAGCAGGAAGCAAAGGGGTCATGACTAGAACCTTCGTTCCCTTCTGGCCTCAGG
AGTACCGGCTCGTGCATGAGATCTTGGGGTTACGACATTGGTCAAGTCCCCTGGGGTGCTTGCTGATAGCTGTAGTTGTG
AAGCAGGGAATTTGAGGACAGGACTGTGATCAGCTGTGATTCAAGAGGGCTGCTGTCTGCAAACAGGGGAGCCACTGGCC
ACCTTTGAACACTCGTACAAGAGCCACTCACAGGGTCCCACCTGAGATGTCCTGGGGCCACAGGCTGGGCTTTCTGTGCG
TGCTTGGTGTGTGTGTTGGGGGGGTCTTCCCACCTGCTCTCCTGCAGTCAGCCAGCTGGCTGCCTTTCTTCTTTAAGGCT
TGCTTGCTTGCTTGCTTGCTTGCTTGCTTGCTTGCTTGCTTTGGGAAGAACGGATCCCTGTGGAGTCCCCAGCACC
CGGCTTCCCTTTCCAAACACAGGCCCCAAGTCTCTTGTCATCACCAAGGGGTGTCTGGTTGCCTCTGGCTCTCCATGTTG
ACTGTAGCTATGAGGATCTGGAGGACATCACGGTCTGGGCTTCCGGGCTGGTGTTCCTCCCTGCTGCTGCCCAGTGAGAA
CTGAGCCAGGCTTTTTCAGTCAACCATTGGAGGAGAGACCCCAAAATAGAAGGCAGGCGTCTCGCTGGGGTCTGGGGGCA
AGGTTGTTTGTCCTGCGGGTTTCATCAGAGAGAGGCCAAAGAAGCCTTGTCCTGACAAAGCTAGGCCTCACCTGTGTGTT
CCTGTCCTTGAGCATCTGGGGGCGGGGGGGTTAACTGCTTGCTGGTAAAGAACATACTCAGTCATACGCCATTCATAAAT
CAAGAGTATGTTAATGTTTCCCTCAGATTGACAAATACCATGGACTTGGAGAAGAGGTTAAGAGAGAGATTGTACCCAGT
AGGCTCCAGGCCCTAGGTTCAAATCCAGCCCCACCAGACCAGACCAGAGAAATTCAAACACTCAGCAGGCCAGTCCCGCT
GTGGCCACCTCCGCCACTCTCTTCCTGTCGGTGACTTTCTGCTTGCCGGACTTCCGGGGCGCTTCGCCCACAGTGCAGGG
TGGAACAAGACTCTGCAATCTTGTTCTCTTCTGTCCCTTTGCTTCTGGGGGGGACCCAGCGTCACCCCAGTGACACGGCAT
GCCATATAAACTAGCTTGGTTGTCTTCCAGGAGAAATGCTTTTTGTTTTGGAGTGGTAGTAAAAAGGGCTCCCAGGATAA
GGAGGCCATTCAGGCCTACTCTGAGTCCCTGATGTCACCAGCTGCCAAGGGCACGGTGCTGCAAGAAGCCAAGCTATAGG
TGCGTACCAGGTAACCCCCCTCTTCACCCCACCTACTCATAGCCAGTAGACCTACCGTCTCTACCTATAGCATCTTCCT
GGATGTTATTCACATGGAGAGCAGTTACCCTCCTGCCTCTCACCCTCCTGCGAGATGGGAATCTTGCCTCGGTTTCTTGG
ACCCTTTCAGTCATTGACTCTCATTTACAAAGTCCTGTTAGAAGATGACAGTTAGGGCTGGTGAGATGGCTCAGTGGGTA
AGAGCACCCGACTGCTCTTCCAAAGGTCCGGAGTTCAAATCCCAGCAACCACATGGTGGCTCACAACCATCCGTAACGAG
ATCTGACGCTCTCTTCTGGAGTGTCTGAAGACAGCTACAGAGTACTTACATATAATAAATAAATAAATAAATCTTTAAAA
AAAAAAAAGAAGATGACAGTTAGGCCAGTTGCTTCTCAGTGTCCTATCCCCTGCAGGTTGCTAAATACAGGTCCCTGGG
CATCCCGGAAAGCCAAGAGACTCTCCTAGCCCAAAAGGTTTGAAAACTTTTTTTTTTTTTTGGACTTCAAGGGCATGTAAA
AGGACACACTGACTCCAGTTCAGAATAGAGAGACTGAAAATGAAGACGTTTAAACATCAGTTAAGCACCAGCTACATACT
CAGTCAGGGCCTGAGGATAGAATTTCATGGTCAAGTACACAGGGTGTGCGACAGACTGCTCAATACAGTCTGAATTTAGA
TGGAGTCGTGTGTCCGCCCTGTGCTTTGCTGCGATGGGTGGTCCAGGTGGTGAGCCACCGAGTCAGGTATAGCTGGCGTC
CTGGTTTACTTAGTAGCCATCTCGCAAGGATCCAGGACCGTACTTCTCCAAGATCTCCAGGTCCTGTAGGGATCAAGGCT
CAGGAGAGAGGCTGAGCCCTACAGGGTGTGTCCCTGCCTGGGAAGGGACTGGAAGAGCTGTGGTCCCTGCAATTTGGGGT
TAGCAGAGGTGAGTGAGTGTCTCAATAGAAGCTGCTATTTTAGCTTGTGAGCCAACTTGGGTGTGAGCACTTTCTTGGAG
```

```
GGAGCTGGGGGTGGGGGGTGGAGGCGGGGCTTGGGGGGGCGGGGGAGGGGAGGCCACAGCTCAGCTCCTGGAGGCCAGCTG
ACTATTTGCTGCAATGTCATTTGCTTGAGAAGCAAATAAGGCAGAACTCTGGTGACTGTGTGATTGTCCTGGTGAGTGCC
ACCTCCCTGGAGGAGGAAGAAGCTCTGCAGCTCAGTGGGTCCCCATATGTCCCTGGAGCTGAGAAAGGGTCCTGCCAGGA
AGAGGTGGGGAGATGGTTAGTGTCTGCCAGTCATGGTGATGTTTGGGTATTGCCAGAGTGCGAGGGGACAAATGTAGGCT
CCGAGCAGAGGCTCCGCAGTCCCTTTAGCCCAGATGGAGCCCATAGGTACACAGCCATAGCCCTGTCCAGACCCTAATAG
AATGAACAAGATTGGGCTTACCTCTTTACTTTCCCAGCTTGGTCTAATCCTCGTTTGGCTTCAAGATTCCCCTGGGTGCC
CCCTAGTCCTGGCCATGTGCTAGGGAGGGCAGTGTCCTCTGAGGATCAGCTACAGCACATGGGTCCTTGCATAGGGACCT
CACCCTCCCCCCCCCGACCCCCCAGTGCCTGGCTAAGTGAAATTCGGTGGGGCCCCACACTCTGCCATGCACCTGGGTGT
GGGGGTGGTCCCTAGAGGCTGCTGCTGATTGCTATAGAATGGAAACAGACGTGCCGCTAGACCAAAGGGTATTATTATAT
GAATAACGTCCCTCTGGGTGGATGGGCATTTCTGTAAGGCAAGGGGGTTGGGGGTTGGCAGGCAGGGTTGGCTGTCCGGC
CAGCTGCTGTGAACACAGTAGCCTTTAAGAAAATGACCTGATCCTCTGCTTCTTTTTGGTCTGTCTCTCATTCTGAATTC
TCCTCTGTCAGACACAGTACAGGGAGGAGCCAGGACAGAGGAGGGGAAATCTGCCCACCCGCCCTGGCTGCCCAGAGCCT
TGTGGCTTTGTGTGTGTTTTCATGGGAGAGACCAAGCTACAGAGTTAACTCTTGCTTTATTCTCTGGCAACAAGGGCGTT
CTGGGCCACTCACGCTGGCTTCGAACTTGTGGACATCCTCCTGCCTCTGTCTCCCGAGTGCGGAGATTCCAAGCATGCAC
CACTACTGTGCCTTGTGTCCCCTTCCCCATTTTACTTCTGTTTTGGTTTTGGTTTTGTGTTTTGTTGTTTTCGTTTTTTG
GGTTTTGGTTTTGGGTTTTTGAGACAGGGTTTCTCTGTGTAGCCCTGGCTGTCCTGGAACTCACTCTATAGACCAGGCTG
GCCTCGAACTCAGAAATCTGCCTGCCTCTGCCTCCCAAGTGCTGGGATTAAAGGCGTGCGCCACCACTGTCCGGCCTTTT
ACTTCTGTTTTTTTGTCTGTCCATTTGTCTGTCTGTCTGTGTGTGGTGAAGCCAGGGCCTTTTGCTTAGTGTAGTCCACC
TCTGAGCTTCACCCCAGCACTTCCTGTTTTACTTCTGAGTGAGACTATCTGGAATGCTGGGGTGCTGTGTCGGGGGTGGA
GGAGTGGGATGGGGGGTGCTCGTTGGCTTTGATCCAAAGAGAAGACGCCTCCCTGCAGTTTCCTTCCCTGCCACTAGGGG
GATCGGTATGGGGGTTGCAGGGCAGGGGTGCCCAGTGTTCTTAAACCCGGCCTTCCATTCATTTTCCTAAGTTGGGAAGT
CTTTTCAAGTCAGAGTCTCCCAACTTACCAAGCCATCCTTTCCTGTTCTGCACCCTTTGCCCAACATCCTACTGTCCCTA
AGGCCCGAGGCCACCATTACAGCAGTCTGGTGAGAGTGTGTCTCTCTTACCTCTATGAACAAGAGCCAGTTTTAATAGCT
GTTCCTGGCCCGTCAGGTGACAGCATTTTAAACATGTGGGCAGACATTTAAGCCATTCCTAGTTTCTTTGTGCGGCGGTG
TTATCTACCCACTCCGGACTCTGCCCCCTTCACTGTAACAGGCCAGTCTCCCTGGCCTCAGTTTTCCCATCTCTAAAGGG
AGAGGTTGGAACCAAGAGACCTACATGACTGAGGAGTCCCCGTGTCTATAGAGTGGCATCTCTGGGTTCATGGCAGACAG
GGCTGGATGTGTCAGGAAGTGGTTTCTGTGCTGGTGGGATGTGCGGTGGGACTAGGGGAGGTAGGCAGGGCAGGGCTAGG
TGACAGGCTTGTGACTCAGCTTCCTTTTTTCTCCACAGGGTCCTGAAGACACTATAAGCCCCCCAAACCTGATAGCTTGG
TCAGACCAGCCACCCAGTCCCTACACCCCGCTTCTTGAGGACTCTCTCAGCGGACAGCCCACCAGTGCCATGGCCTGAGC
CCCCAGATGTCACACCTGTCCGCACGCACGGCACATGGATGCCCACGCATGTGCAAAAACAACTCAGGGACCAGGGACAG
ACCTGCTGCCAAGTGAGCCTGATGGGCCACAGGTGTGCTCTTCTAAATGGCCTGTGAGCCAGGCTGTGGGAACTCTAGCT
GCTGTCAGCCCCTCCTGTAGGAGCTGGCCCTGCCCAGGCTCCTGACTTCCCTCAGGAAGTCTTTCTGTCTTTCTCCATCA
GTCTGAAAGCCTTAGTTCCCACAGAGGACGGATCTGTCACTCCTAGGGGCTGGGCATATGTCGGCCTCTTGTGCCAAGGC
CAGGCAAGCAGCTCCAGGTCCTGACCAGTTTGCACACACACTCTGGAGCTGTATCTGGCGCTTTTTCTATCGTCTCTGCT
ATGTCACTGAATTAACCACTGTACGTGGCAGAGGTGGCAGGCCCCTCAGGGTCCTTATTTTTCAGGCATGGGGTCAAAGC
TAGAGGTATGGGCCGTCTACACCCCCCCGCCCCCCGGCATCTAGTGTACCTCACCAGAGGGTATTCGGAGGCCCAGCATC
CTGCAACCGACCCCTTTTTTCTACTGGAAGAGAAATTTTATCATCTTTTGAAAGGAAGTCATGACTGAAGCAATAAACCT
TTTCACTGATTCAACAACACTGGCTTCTGTGACTGTTTTCTGGGCAGGGCTGGGTCTCCAGAATCCAGGCCACATCAGTA
GGTGTTCCCATGACTGCCAGCGAGTCTCCTGGTGTGAGGCCAGCACCGGCCACTAGCCATGTTTCCACCTCAAGGCTAAT
GTGGTATGTGGCTTGGATGCACCAGGACAGGCTAGCTCTGTCCTTTCTCTGTCCCGTGGAACCTTCTGGGCCTTCCAGCA
GTCTGTGTCCAAGATCAGAACATCCTTGTGACCCCAAGTGACAAGCCTGCAGCGTCTGGGGGAGGGCTGGAAGGGAGGGT
CTAACTTTTGTCCCAAGTTCAAGCAGGGGTTCTACCTGGCATCTCTGAGAGTAAAACCATGTTTTGCCCTTAAGGGACCC
ACTAGGAACTGGGCAATAGATTTTCACTGTGAACATTAAAAACTACATAGCCGGGCTGGAGAGGTGGCTCAGTGGTTAAG
AGCACCGACTGCTCTTCCAAAGGTCCTGAGTTCAATTCCCAGCAACCACGCGATGACTAACAACCATCTAATGGGATCTG
ATGCCCTCTTCTGGTGTGTTTCTGAAGACAGCTACAGTGTACTCATATAAAATGAATAAATAATTAAAAAAAAAACTACA
TAGACACTCACTCGGTGGTTGTAATGGTCTTCACCCTTCCCTTTCTTAGAAAAGAATTTGAATTATTGTGTGTGGGTCCA
TGCCATGGCATAAGTCAGAGGGTGAGCTCTAAGAGTTGGTTCTTTTCTCACCATGTGGGTCCCGGGCATCGAACTCAGGT
CCCCAAGTTTGGCAGCAAGCTCCTCTAGCTGCGGAGCCATCTCGCTGGCCTTGCACTTCCTTAATGAGCACTGTTCTCTA
CCTGCCCTGGAAGCATTGAAAGTTTCCTACCTCATACTATAAACTGCATTATAATCGTGAGACATAAACTTCTATATAAG
GCAAACATTTTTAGTCTTGTAAGACGGGGTCTAGCTTTGTAGGCCAAGCTGGGCTGGACCTCATCATCCGCTTGCCTTCT
GCTCCTAATGCTGGGGTGACAGGCACACCCTCACCATAGCCAGATTTCTTTTTTTCCAAGACAGGATTTCTCTGTGTATC
CTTAGCTGTCTTGGAACTCACTCTGTAGGCCAGGCTGGCCTTGAACTCACAAACCCACCTGCCTCTGCCTCCGCCTCCGC
CTCCGCCTCCAAGTGCTGGGATTAAAGGTGTGCACCACTAGGCTCAGATTGTATTATTTCTAGGACTGAGAATAACCAGC
CTTTCTGGGCTACCCAGGTTGGTTGTAAACTTCTGGGCTCAAGGAATCCTCTGGTCTTAGCCTTCTGCGTGGCTGGGACC
AGAGACCTCTGTACCTGGGTTCCTGTGACTTTCCTGAGCTAGTGTCTGCGCCTGCTTCTACTGCACACAGACAGAGGACA
GGGCGACTCAGAGGCAGACATTCCTGCTCCAGAGTCTGGAGGCCCAAGAGGGTTGGGGCAGGCTGGCTGGGGCTCTGGGG
ACGATCCTCCCCTGCCCTGCAGGTGCCTGTGCCCTACTTGGCTTTTATGGGTGTGTGGAAACTCAACCCTGGCCCGGGGC
CTCCCCCCACCTTAATCATTTCTTAGTGTTTCCCCGCAAACAGAGCCCCATGGGATCAGATCAGGTCTTCTTTCACTGGG
TCTGAGACTAAACACACTCAGCCAGTAACAGTGACGTCCCATTCCACTCTCGTCACTAAGTCCTGTGCAACTTCATCAGC
CCGTCATGTTTTGCTGACAGCAGACACTGTCTCAGCTGACCTTTTCTACCACACGTATTTTTTTAAAAGATAAATATTTA
TTTATTATTATATGTAAGTACACTGTTGCTGTCTTCAGACACACCAGAAGAGGGCGTCAGATCTCATTACAGATGGTTGT
```

```
GAGCCACCATGAGGTTGCTGGGATTTGAACTCAGGACCTTCAGAAGAGCAGTCAGTGCTCTTACCCACTGAGCCATCTCT
CCAGTCCCTGCCACACATAGTTTACGTAGTTTCCTTGCACTCAGAGTTGGCAAATGCATGCGCTTCTGTGAGTTTGAATG
CATGGTCCTATCTGAGCACCCGCTGCTCCAAGTGTGTACACAGAACAGGGACTGGAGCTGGGGTCAATGGCAGGCTAGTC
ACCTAATGTGCAAGGAGCTTTGGGTTGGAGCCCCAGCATCAAATGAACTGGGGGCAAAAGTGTGAGGGAGAGGCAGGAAG
ATCAGAAGTTCAAGGTCATCTTGCTCTACACAGTGAGTTCAAAACCAGCCTGGGGCTACATAAGACCTGGATGAAGAAGG
AGAAGCAGGAGGAGGAGGAGGAGGGGGAGGAGGAGGCTGATTTGGTAGCAAAAACCTTTAATCCCACCACTCAGAAGGCAGAG
GATCTATGAGTTTGAAGCTAACCTGGTTTATACAGTAAGGTACAGCCAGCTAGGACTATGTAGTGGGATTCTGTGGAAGG
AAGAGGGAGAGGGGGAGGGGGAGGGGGAGNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNAGGTGGAGGAGGAGGAGGAGGAGGAGGAGGAGGCAGA
GGCAGCAGCCCGGTGGTGGTCATACTCTTTTAATCCCAGTCTTGGGAGGCAGAGGCAGGTGGATCTCTTGAGTTCAAGGC
CAGCCTGACCTACAGAGTTCCAGGATGGCCCAGGTTACACAGAGAAGCCCTGTCTCAGAAACAAAACAACAGGGGCTGGC
AAGATGGCTCAGCGGTTAAGAGCTGAGGTCCTGAGTTCAAAGCAAGCAGAGGTCCTAAATTGAATTCCCAGCAACCACAT
GATGGCTCACCTGAACAGCTACAGTGTACTCATATACATTAAATAAATTAATTTAATCAAATAAACAAAACAACAAGAAG
AAGCTAAACTTGGGGTTCAGATTCCACTTCCCCAGCGCTACCTCCCCCCTTTATCTACACACTGACCTTCCTCTCAGTGT
GTAACATTTGGCAGCCGGCAAGCTTTGTCTGTTCCAGATATTCTCAGAAGAACCAGGCAGTGGGGAGCCTGTCCAGTTG
GCTGCAGGGGCTCCTCCCTGCTGAGTAGCGTGCAGGGATCGGATGGCTGCCACTGGTTGTCAGTCATCTGGGATATTTTT
ACCCTGTGGGCTGACACGCCCACTGATCTCTCTCCACATTTATAGTAACCCGCTTGCAAAAGCTGGTTGCTCAATCTGCA
GCCTTCTGGGAACCAATGTGGAAAGGTTCCCCGACCGGTGTGTGTATGTTTGTGTGTGTGTGTGTGTGTAAGAGAGGT
CTGTGCTGTGCGGAGGCCCCAAAGCCCCAAAAGTCTAGACCTAGGAAACAGCTGCTTACAGCACCCCCTGCTGGATCTTG
GGGAAGGATCCTTTTTGGTTTGGTTTGGCCTGCCTCAGTGTGTGTGTGTGTGTGTGTGTGTGTGTGTGTGTGTCCAGAGA
TCCTCTTCTTCACCCTCCCTCTCCTCTCCAGGCTGGGATTACAAACACAAATGATCATGACCTGGCTTTCTTATGATA
TTAGGACTTGAACTCAGGTCTTTCTGCCCATTTTCCCGTTATTTACTGAGCTATCTCTTCAGCCCGTTCCTTTTTGTTTT
GTTGTTGTTGTTTGTTTTGTTTTGTTTTTTGAGGCAGGATTCCTCTGTGTAACCTGGCCGTCTTGGAACTCATACTGTAG
ACCAGGCTGGCCTTGAAGTCAGAGATCTGCCTCCTGAGGAATTAAAGGCATTGCCCACATCTGACTTCAAGAATTAAATT
ATCAAAGAGGAAGCCATGTGTCTGTGAGATTCTGGACTTGTCTGGCCAGTGATATTCTATGAAACCCATGTGACACATGG
CCCTTAGTCCTGCAGGCCCTTCAGTGTCCCATCACTCCTAGTTCTTGGAGGATGCTCTCTGTACCCTGTCACCACCTCCT
ATGCTGCCAGTGGAGGGTGTGTCACCTCTGATCATCTCCAGCCTCAACTAGGCCTCACTTTCATCAGCCCCTCTACCATA
GCTCTATCCTAGCCTCCTCTGGGAAGCCCCCCTATGATGGTAAATTATAACTGTCAACTTGACACAGCATGGAATCACTC
AAGAAGAAAGGCTGAGGGAGAGAATCCCTCAGAGAGAGATTGTGCATGGGGGCCCATGTGTGGGTATTTGCCTTGATCAT
TAATTGAAGTGGGAAGACTCACTCTCAGTGTGGGGCAGCACCATTCCCTAGGCCCCGTCCTGACCTGTGTAAGAGTGAAG
AAAGCTAGTTAAGCATGAGCCAGCAGGCAGCATGAGTGTGTCCATTTCCTCTCTGCTCCTGACTGTGGATGCGATGTGAC
TCACTACCGGAGTTCCTGTCAGGAATGGTGACCTCAAACTGTAAGTGAAATGTCCCTTGTTTCTCTGTGGTGCTGTCACC
ACCGCAATAGAAATGACACTAGGACACCCCCCCCCATAACTGCTCCCTCAAGCTTCCCTCCTCAACACCCCTGGAGTGCT
TTTGTACCCCAGTCAATGCCAGGTCGCTTTGGAATAGTTGAGATCCTAACTCTGGTTGTTGGAGCATGTACACACACACA
CACACACACACACACACACACACACACACCAGGCCTGGCATTGTGATGGACACTCTGGTCTGTGACCCAGAA
GCATGTCCAACTTCACAGGAAATATCTGCCCAACGCCTGCTGCATGCTTGGCACCAGGGTCCTGGGGTGGGGTGCAGGTG
GAGGCGCCCAACCGACGCCCTTCCTCCCAGATGGCTTTGCTATCCTGCTGCTTCTTGCAAGGGGCATGGCCGAATCACAG
ACCTTAGCAACCCCCACCCATTTAAGACAATACACCTGAGAGAGGCAGAGACAGGGGCTTCAGGAATTCAAGGAATAAAT
GAGCTAGATAGCAAGGTAGAGGCCAGCCTGGGCTACATGAGACTCTTGTCAGACAAACCAAAATGAAGCCGGAGGATTAT
AAGTTTTGGAACAGCCTGGGCTGAAGGTGAAGCCCTAACTCAGGAAAAAAAAAAAAAAAAAAAAAAAAAAAAAAGCAATGAG
CAAAAGCCAGAGTCTCTGCAGCGGGCGGAGCAGCCAAGGAGGTGGGGGGAACCTCTGTATTCATGTTCACTCCACTGTCC
TGCTTTGATCTGTCCCTGGGACATTTGGACTGTCTGACAGGCCGTTCCTGGCTCTCACATGCAGCCTGGGCCTTCTGTTC
TTCCCGTAACTACCCAGGAGTCTTTCTGAGATATCATCGACCACAGCTTGTCCTTTAAGCACGCCCTTTAAGCTTTTCCC
AACTCTGAAGACAACGCCCTCAATCCTCATCCTGTGCCTAGAGGACCGACTTCTGGGTAATTCCAGTATTGACCACTAGA
GGGCGGTTTGCACACGTCGGATGCCCAGTGGCGCCTTTAAGGGCTGGCCAGTGTCCCGTTATTCTTGGGATGTGTCAAAT
CCGACTTTAACTGTTCACGGGCTGGTTTGGGAATGGCTAACCTTGAGCAATTTTATTTTGTGAGTGTTCCAGAGAGCCTC
TCCCACACCAGAAAAATGTCTTTCTTAATCTTTAGCATTTGAAAAGGTCTTAACACTAAGTGGCTTGCTAAGGAGCCCTA
AGCTAAAACCCAGGTCTCATGATTAAGATTCTGAACCATGAACAACAGCCATCTGTGCTGCTAACCTCTGTCCCACTTGC
TGGGCTGCCTTGGAGCAATTCCCTACATCTCTGAGCTTGCCTTGCCATTGGCAGCTCATGGAAGGGAGCGAATATTAGAT
TACTGCCATGCAAAACCTCTGGCAGCCTCTATGACGCTAGAACAGGAGATGCATGGCGGTGCAGAACTCAACAGGGTGAG
GCAGGACAACCATGACATTGAGGCCAACCTGTATTACATAGCAATTACTTATTTACAAAATAAATAAGAAATAAAAGTGG
GTGTGTGCGCAATCTCTCCGGAGATGGGACTACAATAAAAACGCTTGGATGCCCAGCTCCCAGAAAGCAACTATTAGGCA
CCTGCTATATGCGGAGCACTTTCCACCCTGAGAAGGTGCTTAGGTTCTCTCATTCAGCAAGTGTCCGGGTGGCCACCATG
TGCCTAATGCCCTTCTGTGTGTTGGGATCGTGTTGGGCTGGGTTCCCTAGAAGGAGAATGGGAGGCAGAGGCTGGTGGCT
AAGGGATGTCCCCGGGTAGAGAGAATAAGCCTAGTAGTCAAGGGCAGCTGTGCAGTAAAGGCTGTCTATGGGACAGAAGT
ATAACAACCAGGAGCGGGGAGCAGCTTGAAGCAGCTGCAGGTACAGAGAACGGCCACCAGGGGTCGCTGTGGTTCCAGTC
ACTAGAGCAGAGCGCCTGGCCACAGACAAAACCCGCAGCTACTCCAGGCTTCTGTGGCAGTTTTAAAACCAAGGAACATC
TACTTTTTGAGGCTCTAGACACGTTAAGCGTGAAAAGCAAACAAACAAACAAATGTAACAAACCCAGGCTGCTTAAGATA
CACTGTTGAATATGTTTGTATTTAAAAGGCTATCCCCACTTTTTCTCTCTCTGACTCTTTTCTCTCCCCTCCCCACCCCA
CTCTCTCCACATGTTCCTGCCGGCCTCTTCCCTTCCCCATCCTCCCTTCTCAATCCCCCTTCACATGCCCCTCTTCCCAT
GCCCCCAAATAAACTCTATATTATATATATATTTTTAAAGGCTATCTTCAGTTCCCATATTCAGATGCCTTGTCGTGTTG
```

```
CCAAGCCATGGAGCTTGCAGACAATGTCTATGGAAATAGCAACAGTTCTTTAGCCCAGGAAGCCAGGCACAGGGTTGGGG
CGTTCATATGCATCTCCAAAGTAGGTCCGGGGTTCAGTTTGCACAGCTGTCTTTGGGATAATTATGAAACGGTTATTGGG
ATCTATGTCCTTGGGAACTATGAAATGGCAGATCCTGGGGGCTGGAGAAACAGCTCAGACGTGAAGACCACTTTGGTGTT
CTTGCAGAAGACTGCAGTTCAGTTCCCAGTACACATATCGGCTCACAGACACCTGTAACTGCAGAACCAGGGTGGTCTGG
CGCTCTCTTCTGGCCTTTATGGGTATTGCACACATGTGTGCACACTACCCAGCGAAGACCAAAGCTGGGCTCTGGCTTCTACA
TCCCTAAGATGTTGTCATTACTTGAATATTTGCTGTGTACTAGACAGATGGTACCACGTTGACCATTTGTGTTCAGTATT
TGTGACCTGAGAGAGTGCTGATGTCATCATTCCCTCGTTTTTACAATGAGGAGGCTGAGGCACAGAGAGATGACGGTTGA
TAGGGAAACAGGTTTGCACCGCAGCGGCTAGGCCAGCTCATGAGTGCTGCCCACTGTTCCTCTCTGTGCAGCACCCAAGG
ACTGCCCGCTGTGCTGGTGACCCCAGAAAGAGCATCTCAACCCAGGTCAGGAAGTGTGACCAGTGCCAAGGGACCCCGGG
AGGCCTAGGGAGGAAGGGAGGTCACGGGCTCAGGCCCCAAACTGCATCTGTCTAGAACAGAGATTCTGAACCTTCCTAAT .
GCTATGGCCCTTTAATACAGTCCCTTAAGTTATAGCGACCCCCAGCCACAAAGTTATCTTTGATGGCACTTCAGAACTGT
AATTTTGCTGCTGTTATGAATCACAATGGAAATACTTTCGGAGATAGAGCTTTGCCAAAGGGGTCTCAACCCACAGGTTG
AGAACCGCTGGTCTAGAAGGAGCCCCATTTATCTGGCCAGGCTGCTTTGGTGAGGGTCTCAGTTCAGGATTGTCCCCAGA
CAGGTGGCACACACTGGGTGAAACGAGGAAGAGTCACATGTCTAAAAATAGTGCCTGTCTCCCAGAGCCTGTCTGTCACA
GGACAGAGTGGAGCAGCTAGGAGGTGGGAAGGATTCTCTGGAATTTGGGGGTTAGGTATACAGGTTCAGGGGAGCTGCCC
CACTCTGAGGTTTGGCAGTGAGCTGGGCTGGAGAGCTGAGGTACTGGGGCTACACGTGGCACCATTCTGGAGTCAGGTTT
ACGCCATCCAGTGCTGTGCTTTTGCAGGAAGCATCTTCTATGTAGGAAGTGGAAGGAGCTTTGTGATGTGATACCCATTT
CTCTCATCTACTCCCTTAGGACATGGGTCTCCACCCCCAATCTGCCCCTCTTCCACCACCCTGCTTTTACCCTGCCCTGA
GACCACATCGCCCGCCACAGACAGATAGGAAAGGCTTTATGTGGAGAAGGACTTGTAAGCTGATACACACAGCACACACA
GTGCTCCCTCTGGTCTCCCTCTGTGGCTATGGCTACCATCTGCCCTCTGCTGGGCCACAGGAATGTCTTCTGTTGGAGGG
GCAGGGACTCCGTCACGGGCCTCTCTGACCCAGCTTTGTATGGCTCTTGGCCCTGTGGTCTCAGACTGTGGGCAGCAGCT
GTTTGGGGGCAGGACATGGTTTCCTCCTGACATCAGCTTTGGCAAGGGAGCAGGCAGGGGTGGGAGGCCAGCCCTGTGGC
AGCAGGCTGGATGCATTGTAGCTCCCTGGGCAAGGCTCTATGACCATACCGTGCCCACGTATAATTCACAGGAAAGCCAC
TTCCTGCCCCACCCACTCCAAGGTCCAGATGAGGGTGGGCGAGAGGGATTTTGGATGGACATTTCGCTCATGGCAATTGG
AAATGGTTTTCAGATGTATCACGGAATGTGGAACAAACCCAGGCAAACTTCCCTGCAATCCTATAAATCACCAGCAGATG
GCGCTATTGGCCACACGACAGGATTCTGGCATCCTGGATTGCTCAGTGGTAGAACTTGAACCTGGGACTGAAGTAGAAAC
TCTCAGGCCATAGGCTTACACACTGCAGGTGGTTTACAGCAGGCAGATGGGAAAGGCATGCCTGGCCCAGACGCTGGCCA
GAAAGGGTAGCTGCAAGAAGCAAATGAGGTACCTCCTTCCTACCAGTACCTACTATTCAGAGAGGCATGTTGGTCTCTGA
GTTCAAGCCTGGACTACGATCCCTCACAGAATTAATTTCCTGGAAGAAAAAAAAAAATCAATGCTTGTCCCTCGGTGCCA
GACATTAAAGTCCTCAGGATTGGCGGTGGCGGGCTGTTTTGACAGGGATGAGAATGGAGACACCCAAGAGACTGCTGGAA
GCTCAGTCAAAATGCCTCATTTCCTGACACCCGCGTCGCTGAGCACACTGTCATTGAGTCAGGACACGCAGCCACCCGCC
CCTGCAGCCATTCATCCCTCCCCACCCCCACCCTCCCACATTTCATTTGCGAGCCCCTCGGTGTGCAGCTCGGCTCGTGG
CGGCTGCGGCGGCAGAGCAGAAACCCCAAATGATCTATTTATGGGGCCGTCCTTCCGTGTTGCTTGCATATTGTAATTAA
GAGATAATGTCAGTGGAATAGAACGTTGTCACCCAGTAACGGCCTCCCGCCGCCTCGGAAATGTCACGGCAAAGTCACAG
ATATATCAAGCGTAAAACTGACGTTTGTTTTCAGAGCAGGGAACGCGGATCCTGGGACAGCGATACAGCTGCGGCTTCAT
TTCTGTCTTGGGGGTGATGAATGCCAGTGGATGGTCGGGCCCACAGGAGGAGGGGTTCTTGTCTGTTGCGGAGACCAACT
GGGCCTGTGCTTTGCTTGACAGATGGAGGATAAGGCTGCCCTCTGGGTCCCATAATCTGCTGTAGCCACCAAGGTGGCCC
GGGTGCCCTGGCTCCCGGCTGGGCAGTGTGTTTTCTGCAGCCCAAGCTGGCTGCCAGCTGGCCGGGTTCCCCTTCCTGTC
ACCATGTTGTCTCTGGAGAGCTAAGCTGCTGCTGGGGTGGCATGTCCTTGGACAACCTCAGTTTCCGAGTCACTCCCCCA
GAGCCACAACGG

MOUSE mRNA SEQUENCE : mR7-104.1 (Seq ID No: 78)
GGGGCGGGGCTGCCCGGGCCCATGGCGCATAAAGCCTCTGGCCACCTGCAGGGCTACTGCTGCTCCGGCCACCGCCAGGCAC
ACACCTTGCTGCTGAGGGAGTCTCGGCTTCTGTCATCTCTGTGGCCTCCGTCACCTCTGTCTCCGTCTCCTTCAGGTCCT
GAGCCCCGAGAGCCCCTTCCGCGCACGCGGACATGGGCGGCAGCTCCAGGGCGCGCTGGGTGGCCTTGGGGTTGGGCGCC
CTGGGGCTGCTGTTTGCTGCGCTCGGCGTTGTCATGATCCTCATGGTGCCCTCCCTCATCAAGCAGCAGGTGCTCAAGAA
TGTCCGCATAGACCCGAGCAGCCTGTCCTTCGGGATGTGGAAGGAGATCCCCGTCCCTTTCTACTTGTCTGTCTACTTCT
TCGAAGTGGTCAACCCAAACGAGGTCCTCAACGGCCAGAAGCCAGTAGTCCGGGAGCGTGGACCCTATGTCTACAGGGAG
TTCAGACAAAAGGTCAACATCACCTTCAATGACAACGACACCGTGTCCTTCGTGGAGAACCGCAGCCTCCATTTCCAGCC
TGACAAGTCGCATGGCTCAGAGAGTGACTACATTGTACTGCCTAACATCTTGGTCCTGGGGGGGCTCGATATTGATGGAGA
GCAAGCCTGTGAGCCTGAAGCTGATGATGACCTTGGCGCTGGTCACCATGGGCCAGCGTGCTTTTATGAACCGCACAGTT
GGTGAGATCCTGTGGGGCTATGACGATCCCTTCGTGCATTTTCTCAACACGTACCTCCCAGACATGCTTCCCATAAAGGG
CAAATTTGGCCTGTTTGTTGGGATGAACAACTCGAATTCTGGGGTCTTCACTGTCTTCACGGGCGTCCAGAATTTCAGCA
GGATCCATCTGGTGGACAAATGGAACGGACTCAGCAAGATCGATTATTGGCATTCAGAGCAGTGTAACATGATCAATGGG
ACTTCCGGGCAGATGTGGGCACCCTTCATGACACCCGAATCCTCGCTGGAATTCTTCAGCCCGGAGGCATGCAGGTCCAT
GAAGCTGACCTACAACGAATCAAGGGTGTTTGAAGGCATTCCCACGTATCGCTTCACGGCCCCCGATACTCTGTTTGCCA
ACGGGTCCGTCTACCCACCCAACGAAGGCTTCTGCCCATGCCGAGAGTCTGGCATTCAGAATGTCAGCACCTGCAGGTTT
GGTGCGCCTCTGTTTCTCTCCCACCCCCACTTTTACAACGCCGACCCTGTGTTGTCAGAAGCTGTTCTTGGTCTGAACCC
TAACCCAAAGGAGCATTCCTTGTTCCTAGACATCCATCCGGTCACTGGGATCCCCATGAACTGTTCTGTGAAGATGCAGC
TGAGCCTCTACATCAAATCTGTCAAGGGCATCGGGCAAACAGGGAAGATCGAGCCAGTAGTTCTGCCGTTGCTGTGGTTC
```

GAACAGAGCGGAGCAATGGGTGGCAAGCCCCTGAGCACGTTCTACACGCAGCTGGTGCTGATGCCCCAGGTTCTTCACTA
CGCGCAGTATGTGCTGCTGGGGCTTGGAGGCCTCCTGTTGCTGGTGCCCATCATCTGCCAACTGCGCAGCCAGGAGAAAT
GCTTTTTGTTTTGGAGTGGTAGTAAAAAGGGCTCCCAGGATAAGGAGGCCATTCAGGCCTACTCTGAGTCCCTGATGTCA
CCAGCTGCCAAGGGCACGGTGCTGCAAGAAGCCAAGCTATAGGGTCCTGAAGACACTATAAGCCCCCCAAACCTGATAGC
TTGGTCAGACCAGCCACCCAGTCCCTACACCCCGCTTCTTGAGGACTCTCTCAGCGGACAGCCCACCAGTGCCATGGCCT
GAGCCCCCAGATGTCACACCTGTCCGCACGCACGGCACATGGATGCCCACGCATGTGCAAAAACAACTCAGGGACCAGGG
ACAGACCTGCTGCCAAGTGAGCCTGATGGGCACAGGTGTGCTCTTCTAAATGGCCTGTGAGCCAGGCTGTGGGAACTCT
AGCTGCTGTCAGCCCCTCCTGTAGGAGCTGGCCCTGCCCAGGCTCCTGACTTCCCTCAGGAAGTCTTTCTGTCTTTCTCC
ATCAGTCTGAAAGCCTTAGTTCCCACAGAGGACGGATCTGTCACTCCTAGGGGCTGGGCATATGTCGGCCTCTTGTGCCA
AGGCCAGGCAAGCAGCTCCAGGTCCTGACCAGTTTGCACACACTCTGGAGCTGTATCTGGCGCTTTTTCTATCGTCTC
TGCTATGTCACTGAATTAACCACTGTACGTGGCAGAGGTGGCAGGCCCCTCAGGGTCCTTATTTTTCAGGCATGGGGTCA
AAGCTAGAGGTATGGGCCGTCTACACCCCCCCGCCCCCCGGCATCTAGTGTACCTCACCAGAGGGTATTCGGAGGCCCAG
CATCCTGCAACCGACCCCTTTTTTCTACTGGAAGAGAAATTTTATCATCTTTTGAAAGGAAGTCATGACTGAAGCAATAA
ACCTTTTCACTGATTC

MOUSE PROTEIN SEQUENCE : mP7-104.1 (Seq ID No: 79)
APRAPSAHADMGGSSRARWVALGLGALGLLFAALGVVMILMVPSLIKQQVLKNVRIDPSSLSFGMWKEIPVPFYLSVYFF
EVVNPNEVLNGQKPVVRERGPYVYREFRQKVNITFNDNDTVSFVENRSLHFQPDKSHGSESDYIVLPNILVLGGSILMES
KPVSLKLMMTLALVTMGQRAFMNRTVGEILWGYDDPFVHFLNTYLPDMLPIKGKFGLFVGMNNSNSGVFTVFTGVQNFSR
IHLVDKWNGLSKIDYWHSEQCNMINGTSGQMWAPFMTPESSLEFFSPEACRSMKLTYNESRVFEGIPTYRFTAPDTLFAN
GSVYPPNEGFCPCRESGIQNVSTCRFGAPLFLSHPHFYNADPVLSEAVLGLNPNPKEHSLFLDIHPVTGIPMNCSVKMQL
SLYIKSVKGIGQTGKIEPVVLPLLWFEQSGAMGGKPLSTFYTQLVLMPQVLHYAQYVLLGLGGLLLLVPIICQLRSQEKC
FLFWSGSKKGSQDKEAIQAYSESLMSPAAKGTVLQEAKL*

MOUSE PANTHER CLASSIFICATIONS
        FAMILY (SUBFAMILY)
            CF11923(PLATELET GLYCOPROTEIN IV)
        BIOLOGICAL PROCESS
            Lipid, fatty acid and steroid metabolism(2.03.00.00.00) > LIPID AND
FATTY ACID TRANSPORT(2.03.07.00.00)
            Signal transduction(2.11.00.00.00)
            Immunity and defense(2.16.00.00.00) > Macrophage-mediated
immunity(2.16.05.00.00)
            Apoptosis(2.26.00.00.00) > Other apoptosis(2.26.99.00.00)
            Cell structure and motility(2.27.00.00.00) > Cell
structure(2.27.01.00.00)
        MOLECULAR FUNCTIONS
            Receptor(1.01.00.00.00) > Other receptor(1.01.99.00.00)

MOUSE GENE ONTOLOGY
        BIOLOGICAL PROCESS
            steroid metabolism > cholesterol metabolism
            cell communication > cell adhesion
            lipid metabolism > fatty acid metabolism
            nucleotide metabolism > lipid metabolism
        MOLECULAR FUNCTION
            GO molecular function > cell adhesion
            mannosidase > beta-mannosidase
        CELL COMPONENT
            cell > plasma membrane
            cell > membrane fraction
            plasma membrane > integral plasma membrane protein
            lysosome > lysosomal membrane
            endoplasmic reticulum > endoplasmic reticulum lumen
MOUSE PROTEIN DOMAINS (INTERPRO SIGNATURES)
            IPR002159 (CD36)

HUMAN GENOMIC SEQUENCE : hD7-104 (Seq ID No: 80)
TTCTACCGCAGCTGTTGGACACTGCCTCTGAGCCTCTCTTTCTCTCCTCTAATTCCTTTCCCACCACAGAATCAGTTCCT
GTAGTAGTCTGAGCAGGAAGTGGAGAGAACACTCAAAATTGGGGACTTTGAAGATATATACAAAGGCGCAGGGAAAACGA
AAGGGAGAGTGAAGCTGCTCCCCACCCTGGGGCTGAGGGGAGGAGGGCAGGGAGTGTCACCTGGAACCCGGGAGGAGAGA

```
GGAGTCGAGAGAGGTCTGCCTTCCAGGGACCGTGACCTCTGCTCAAGAGCTGCAGCCAGCCTGTGTGTTTCAGTGCAGTG
CAGTTGGGGTATCACAGACCACCCCACATGCAGTGACTTGGAGGTGCTATTAATGATACTATTGGGGCAAGAGGTATAAA
CCGGGGTGACTGCACCGTCTAAGGTGCAGGGGTCCCTGATTGTCTGTTCCTTGGGCACACTGAGCACCTCCCTGACACAG
GGCCTTTGCACTTGCTGGTTTGTTTGCCAGAAAGGTCCTCCCCAGGTTCCGGTCTCCCCCGGCCAGCCCCCATCTCCCTG
TGGCATTACCTTTTTATTTCTCTCATTAGCACGTAGCACCATCTATAATCCTTATCTATTTATTTGCTTATACATATATT
TATAGATAAATAAAAAACATATATATATTTTAATATCTCTCTCTCTTTCCCTTACTAAAAAGGCCAGGGCAGACCAGAC
GTGGTGGTTCACACCTATAATCCCAGCACTTTGGGAGGTCGAGGTGGGAGGATCGCCTGACTCCAGGAGTTCGAGACTAG
CCTGGGCAACATAGTGCGACACCCATCTCTATTAAAAAAAATAAAAATTAAAAAAAATTAGCTTGGCTGGGTGCAGGGGC
TCACACCTGTAATCCTAGCACTTTGGGAGGCCAAGGTGGGTGGATCATTTGAGGTAAGAGTTCAAGATCAGCCTGGCCAA
CATGGTGAAACCCCATTTCTACTAAAAAATACAAAAATTAGCTGGGAGTGGTGGCAGGCGCCTGTAGTCCCAGCTACTCG
GGAGGGAGGCTGAGGCAGGAGAATCGCTTGAACCCGGGAGGCAGAGGCTGCAGTGAGCTGACATCACACCACTGCACTCC
AGCCTGGGCGACAGAGCAAGACTCTTGTCTCAAAACAAAACAAAACAAAAATTAGCTGGTGTGGTGGCACATGTCTGTA
GGAGGCTGAGGCGGGGGGATCGCTTGAGCCCAGGGGGTGGAGGCCGCAGTGAGCTTTGAACTCCAGCTTGGGTGACAGAA
CCATACCCTGTCTCAAAAAAAAGTAAGGCCAGGGCACAGAGGACCTGCCTGCTTTCTTCCCCATCTACCCCCAGGCCCTA
GCACACAGTAGGCCTGATACATAGCGAGTGCTCAGCAAGTATTTGCCACATAAATGAATGAATGAGCATGTCCATCCCTT
CTCTGGTAGGTAGGACAGGACCAGGTGGTCTCAGAGGAATCTTCTGGTTCCTGGGATAGGAATCATCCTCCTTTCTCCAG
CTGCTGTTGAGAGTGGCAGCTGATAGAAACTTCTGTCCTCCAGTGACCTGGCCCACGGAGCAACGTATTAGCTCGCGGCT
GCCAGCTGGGTCCTCCCCACACACTTGCACCTTTGTCCTGGAAAGCACGTCTGTGTTGGAGGGTTAGCCCTTGCCAGAGA
GGACATATCAGAGTCCCCAAAGGCCAGGGACAAAGGCAAGGATTGATCGCCAGCCCTGAGTCAGAGAGGAAAGACTCCTG
GAGGGAAGTCAGTGATCGGGGACTCAGAAAGGCGTGATTCTGAAGGAAGACAGGATTTACAGGGCATCTCAGTCTGCTCA
GGCTTCTAAAACAGAGTACCATAGACGGGGAGAGCTTACAGACGGCGGACATCTATTTCTCACATTTCTGGAGGGTGGGA
AGTCCAAGATCAAGGCGCCAGCAGATTTGATGGTGGTGAGGCCCTGCGTCCTGGTTCACAGATGGCAGCTTCTCTCTGTG
TCCTTGCCTGGTGGAAGGGGAGATGCAGCTCTCTAGAGCCTCGTGTGTCAGGGCCCTAATCCCATCGATGAGGCCTCTGC
CCCCATGACCTCGTCACTTCCCAAAAGCCCTACCTCCTAATACCATCACATAGTAATTAGGTTTCAACGTACAAATTTCA
GGGGACACATTCAGACTCTAGCACCGGGGCTAAAGTCCAGGGGCAGAGGCTAAGTTTCTGGAGGGGCTGTTTAGGGGCTC
TGGAGACCTTCTCTGATATTCACAATATTGCAGGGGGCATTTTGACCTACTGAAGAGAAACAGCTCCTAAAAAGTCACTT
GTAGCAACATGAGAGATTCGCATTCTGATTAAAATCTATGCAGGCACCTCCTGCCTCAGAGTCGTTGCCCTGGCTATTCC
CTCTGCCTGGAATGTTCTTCCCTGGATCTCAACACATTCCTGCACTTCCTGCAGGTCTTTGCCCAAATGTCACCTTCTCA
CTGAGGCTGTCTTCAAACACCCCATTTAAATGGCAACCCCTCTCCCCCAGTAGCATTTACACTGTTCTGTTTTTCCCCTA
AAGCACGCATCGTCCTCTAACTGCCTGTCCCATGGAAATGCAGCATGCACAAACCACACACAATTTTAAAAATTTTAT
GACAGAGGAACCTGTTCTGAAAAAAGAAAAAAAGGTAAAGAATTTTAGGGGTCATATTTTAAAAAGTAAAAGAAACAG
GTGAAATTAATTTAACTTGTAATATTATTCAACCCTTGGTCAGTGTGTGTGGATGAACCACGTCTACCAGAGTCCAAGGT
AAAAGCCTGCCTCTGCCGCCGGGCCACGCAGCTGCCTGGCATGGTGGCCTGGGGCAAGTTGCTCAGCCTCTCTGTGCCCC
GGCTCCCTTATCGGTGATATTCAAATAGTGTCATTTCAATGTGTAATCAACATAAGAATATTGAGATGTTTACGCTCTT
TTCATATGAAGCCTTTGAAGTCTGGGATGTATATGACATGGCATATCTCATTCAGACTAGCCCCGGGTCAAGTGTCCCAT
AGCCACATAGGGCCAGTGGCTACTGTATTGGATAGCACAGCTCTAATATCTTTTTGTTTTGTTTTTTGAGATGGGGTCTT
GCTCTGTCGTCCAGGCTGGAGTGCAGTGATGCCATCTTGGATCACTGCAACCTCTGCCTCCTGAGCTTAAGTGATCCTCC
CACCTCAGCCTCCTGAGTGTCTGGGACCACAGGTGCGTGCCACCATGCCCAGCTAATTTTTTTTTTTTAATTTATTGTA
GCAACAGGGTTTTGCTATGTTGGCCAGGCTGGTCTCAAACTCCTGAGCTCAAACAAAGTGCCTGCCTCGGCCAAAGTGCT
GGGATTACAGGTATGAGCCACCGTGCCCATAGCTCTAACATTTTATAAAATCCACCAATGATGATGTTTATCACTAATTG
TTATTGCTTGTCTATTAACATACCGGACCCGCAAGGCTAGGGGCTTGCTTATGGTCCCTGCTGGGTCCCCAAACGCCCAA
CATAGTGCCCAGCACAGAGTAGGAGCTCAATGTGAACTGAATGAATCCAGCAGGCACACGTGAGCTCTCTTTCCTCCCTC
ACCAGCAGCAATCCAGGTGGCAGATGTGGCTACAATGCTTTCCTGGCTGGGCGCGGTGGCTCACGCCTGTAATCCCAGCA
TTTTGGAAGGCCGCGGCGAGTGGATCACTTGAGCCCAGGAGTTCGAGACCAGCCTGGCCAACATAGTAAAACTGCATCTC
CATTAAAAATACAAAAATTGGCCGGGTGCAGTGGCTCACACCTGTAATCCCAGAACTTTGGGAGGCCAAGGTGGGCGGAC
CACCTGAGGTCAGGAGTTCAGGACAAGCCTGGCCAACACGGTGAAATCCCGTCTCTACAAAAATACAAAAATTAGCCAGG
CATGATGGCAGGTGCCTGTAATCCCAGCTACTCAGGAGGCTGAGGCGGGAGAATCGCTTGAACCCAGGAGAAGGTTGCAG
TGAGCTGAGATCACACCATTGCACTCTAGCCTGGGCGACAGAGTAAGACTTCTTCTTAAAAAAAAAAAAAAAAAAATTAGC
CAGTCGTGTGGCATGCACCTGTAATCCCAGCTGCTCAGGAGGCTGAGACAGGGAGAACTGCTTAAATCCGGGAGGCGGAG
GTTGCAGTGAGCTGAGATCACACCACTGTATTCCAGCCTGGGCCACAGAGCGAGTCTCCACCTCAAAAGAAAAAAAATA
AGGTTTCCTCTTCCTTTGCTTTTTATTTTGTCCTTTCTCGCACCCGTCTCTTTGCTTTCCTTCACTTTCTTCCTCTTTTT
CCTTTCCCTCAGCAAACAGCGTAGTGGTCATGTGTGTGGCAAGTCACTGGTTCACATCTCTGCTCTCTGCTCGCCAGGAG
CCAGCTGTGCCTCCTTTAAGGAAGCCCTTCAGCTTCCATTCACTGTGTTCTATTGCCGCTGGGGATGAGCGTGATTCTTC
CCAGGCTGGGGTGGGAATGCGGCTGCCTGCAGGAGGTGTTGGCTCCGCCTCACACAGGCACCTTGCTAAGCATATGACCA
AGATGAGCCCATGTAATCCTCATGACCCACCCCAGGGTGCAGGCATGAATGACTCAGACCAATCCCAAGGCACCGATAAGG
GAGCCGAGGCACAGAGAGGCTGAGCAACTTGCCCCAGGCCGCCATGCCAGGCAGCTGCGTGTTCCGGCGGCAGAGGCAAG
CTTTTACCTTGGACTCTGGTAGATGTGGTTCATCCACACACACCGACCAAGGGCCTACTGTGGGCCACGTGCTGTTGGGG
AGCCGGGGGCACTGCAATGGACGAAACAGGCAGATTCCTGCCCCGTGGAGCTTTTTTATCCTAGAAGAGGAAGGAAGCC
AATCAAACAAGCAAACACCCGCCCTGGAGGGCGGTCGGTGCACAGAGAGAAATGAAGCAGCCAAGGGGGATAGGGAGGGT
CCTATGGGTGGTTTGTAACTTTCAAAGGGTGGGCAGGAAGGAAGTCATTGCTGAGAAGATGGCATTGGAGCAAGGACCTG
AAGGAAGGGAGGGTGAGTCTCCTGGGTGTTAGGGAGAGGAGCATTCCAGGAGGGGACCAGGGAGTGCAAAGGTTCTGGGG
```

```
CACGAGCCGGGCAACAGGTGGAGGAGCTCCAGGAAGGAGGATGTGGCTGGAGCAGAGTGGGCAAGGAGAGGGAGGGGGAT
GAGGTCAGGGAGATGTGCCCATCTGGGTGCGTTTTGACTTTGTCTCTCTAGTATGAGATGTGGTGATCTTGTGGGCCATG
GTGAGGGCTTTGGCTTTTATGCTGAATGAGGTGGGAGCCACGGAAAGGTTTCTTTCTTTTTGTTTTCTTGAGAGACAGGG
TCTCACTGTGTTGCCCAGGCTGGAGTGCAGTGACACAATCATAGCTCACTCACTGCAGCCTCGACTTCCTGGGCTCACGT
GATTCTCCCACCTCAGCCTCCTCAGTAGCTGAAACTAGAGGTGTGTGCCACTTTACCTGGCTAACTTTTTATTTTTTGTA
GAGATGGTGTCTGGCTGTGTTGCCCAGGCTGATCTCGAACTCCTGGCCTCAAGGGATCCTCCTGCCTCAGCCTCCAAAAG
CACTGGGATTATAGGCATGGGCCACCATGCCTGGCCATTTCTTTAAAATAACAGTTTTCATTGAGATATAATTTGCATAT
CATACATTCACCCATTTAAAGTATATAATTTGGCCAGGCATGATGGCTCACACTTGTAATCCCAGCGCTTTGGGAGGCCA
GGGTGGGAGGATGACCAGAATTTTGAGAGCAGCCTGGGCAACAAAAGTGAGACCCTGTCTCAACAAAAATGAAAACATCA
GGTGTGCATAGTGGCATATACTTGTTGTCCAAGCTAGTCAGGAGGCCGAGGCGGGAGGATGGTTTGAACCCAAGACTTCA
AGACTGCACTCCAGCCTGGGTGACAGAGTGAGACCTTTTTTTCTCTTGAAAAAAAGAAAAAGTAAATAAAGTGTGTAATT
TGATGGTTTTTTGTATATTCACGGAGTTGTGCAACCATCATTACCATCACTTTTACCGTCACTTTTAGAACATTTTCACC
ACTCCAAGATGAAACCCCAAACCTGTAGCAGTCACTCCTCATTCTCCCCAGCCCCTGGCAACCACTGGTCTACTTTTTGT
CCCCATAGATGTGCCTACTATGGACATTTTATATAAGCAGAATCATACATGTGGCCTTTGTGTCTGGCTTCTTTCACTTA
TCAATGTTTTCAAGGTTCATCCATGGTGTAGCATGCGTCAGCCCTTCATTCCTTTTTATGCTGAGTCATATTCCAGTGCG
TGGTTATGCCACATTTTATTTACCTATTCACCAGCTGATGGGGGTGTGGGTTATTTTCACTGCTTGGCTGCTGTGAACAT
TTGTGTCCAGGCTTCCGTGTGGACATGCTCTTTCCTTTCTCCTGGGCATATACCCAGGAGTGGAACTGCTGGGTCTTAGG
GGACCCCTATGTTTACCTTTTGAGGCTGCACCATTTTACAATCCCACAAGCACCTCAAGAGGCTTCCAACTCCTCCAGGT
CTTTGCCTGCAGTCATTCTCCGTCTTTGATCACGAAAGGACTCTGAGCAGAGGAGGAAAAGCATCTGACTTGATTTTCCA
GGAGCCCTGTGACACCATGTTGATCATATTTTGTTATCAACACTCTTTCTTTTCTTCTGTATCTTCGACTTTCCCCAGGG
CAAGACCTCTCTCTCAAACATGACGAAGTCTTTTTTTCCCTTCTTAAGCTGCCTCCCTGCCACCGGAAGTGACTCCCCTC
AGCAAGGCCAAGGTGGCTCACGTGTCCCATCACCCTTTCATCTCTGCCCTTCTCTCTCGAGTGACAGCATGGCGTGACAT
GAGGGCTCTCCAGCCATGGCCTTGCAGCCCTCTTGGGTAAGCATCACGTACATTTGGACCTTGAAGACTTGGTCATGAAT
CAGAGTTAGATTGGGTGAGATGGTCAAGGCAGGTCAGTATCAAGGAAGTGGAGGAGGGTGTCAGGGCCAGGCAGACCGCA
GATTTTAGTTAACTGGCTAATTTCCGAGAACTACAAAAGTCATTAAAAAAAAAAGGCAACTGCATAGCTGAAGGCTGTTGA
AGGCCTTAAGGAATTCAATGAAGTGATATTAATTAATTAATCAATCGATCAGTTACTTGATTAATTAATGAGTTAATTAG
CTGATTGAGTAAAGTATTTATTGAGTACCTGCTATGTGCCAGGCATTTTTCCAGGTGCTGGAGATACATCGATAAGCAAA
ACAGGGTCAGCTTCTGTCTCTCGGGAGCTGGAGGAGATGAACAAGGAAACAGATGAGACCATTTCACACAAAGCAAGGTG
GCCTGACAGAGTGGGCTGGGACTCCTGTTTTCGACAGATGCAACAGCAAAGGCTTCTCTGAGGAGTGGGTTTCAGCTGAG
CCCAGAAGAGGAGAGTGGGGAGCCATGGAAGGATCCAGGGGAAGCGTATCCTAGACAGAGGAACAGGAAGTGCAAAGTTC
CTGACACAAGAAAAAGCTGGGGATGCTCAATGAACTGAAACAATGGGGGAGAGGGCAGGAGATGGAGGCAGAGAGATGGG
CGGGCGCCAGTTCATGTGGGTCCGGAAAATAACGACGACACTCCCCAGCTCAGTTGCACTGACACATTCCAGGGACTGTT
CTAAGCACTGGCACATTTTTTGACTTGTTTAATCCTCATGGTAGCCCTTGGAAAGGGGCTCTTCTAATCCCCGTTTCGTA
GATGAGGAGACTGAGGCACAGAAAGGCCCAAACCACTTGCCCCAGGACTCCAATGTGGGAAACCCCAGAGCCTGGATTTC
TCTGAACCTGTCCCCCGGGGCTACCACAGTCTCACCTCAGCCACTCTGACCTCCCTCAGGAGTTGGGCCCTGCCCCTTGG
GGCACTGGACCATATTCGGCCTCCTTAGAGGAAGGCAGTGCCAGGCAAAGCTGTCTAACGAGAACATAGCAGGTGCTCTG
TAAATATTGGTTCAGGCGAAGACATGTTGCTTAAGGATGGCCCTGCGAGAAGGCTGCTTTCTTCTGGAGTTAAATAAGAG
GTTTAGAAAGCCTCCCTCGGAGCACTCAAGGACTGAGCCTGCCTTACCCATGTGCTGGGGACACCACCCTCCTGTCCCTG
TGTAACCCAGGAGTCTCTTCTCAGTAACTTCATTCAGAAAATGAGGGGACTGGCCAGCACTTCAGGGACCAGAAGGCAGG
CCAGGTGGGGTGTGGTGGAGGACTTCAGGGGGGGCGTGGAGGGCTTCAGGGATACACTCACCCATCTAAGGGCAACTGCCA
CTTAGTGCCAGCCCACTTCTCTCCCTTTTTTCTTTTTTCTTTTTCCCCCAGGGTCTTGCTGTCACCCAGGCTGGAGTGCG
GTGGTAAGATCAGGGCTCACTGCAGCCTTGAACTCTGGGACTCAAGGGATCCTTCTGCCTCAGCCTCCTGAGTAGCTGGG
GCGACAAGCGCATGCCACTGTGGCTGGTTAATCTTTTCATTTTCTGTAGAGACTGGTCTCACTATGTTGCCCAGGCTGGT
CTCCAACTAGTGGCCTCAAGTGATCCCTCACCTGGACCTTCCAAAGTGCTGGGATTACAGTTGTGGGCCACCATGCACCG
GGCCTGTTCTGTTTTCTTGGAGCACTTGCCTGCAATTATCCTTCATTCATTTGCTCACGTGCTCATCATTGGTTTCCCTC
TTCATTAGAAAGTGGGGACTTGGTTTGGGTTAACTAAGCTTCCCTGTGCATCAGTTTTCATTTCTTTCTTTCTTTTTCTT
TTTCTTTTCTTTTTTTTTTTTTTTTGAGATGGAGTTTCGCTCTTGTTGCCCAGGCTGGAGTGCAATGGCGCTATCTCGGC
TCGCCACAACCTCCGCCTCCCGGGTTCAAGCGATTCTCCTGCCTCAGCCTCCCGAGTAGCTGAGATTACAGGCATGCGCC
ACTACGCCTGGCTAATTTTGTATTGTTAGTAGAGACGGGGTTTCGCCATGTTGGTCAGGCTGGTCTCCAACTCCCGACCT
CAGGTGATCCATGAAGTCCACCCGCCTCGGCCTCCCAAAGTGCTGGGATTACAGGCGTGAGCCACCTCCCAGTTTTCTTA
TTGTAAAATGGAGCCATTGTGTGCAAAGCACTCAGGACAGGGGCCAGCACCTAGAAGGCTCCTCAGTCATTCATTCTAGA
ATATTTACTGTGAGCAGGCATTCCCTGCCAGGCCACGTTCTAGAGCTCAGGACGCGTGGGGGGGGGGGGCCCGCCTCACGG
GTTGGCATCCCAGTTGGAGCACATGGTCAGAATGCAAGGACGCAAATGAACGTGAACCTGCCAGGGGGTGCTCAGTCATA
GGGTGATGGTGGCACCAGCGTTACGAAGGATAGGGCCAGGCGGATACCTGGGAGAACAGAATTGCCTGTGCAGGGTGTAT
GGNNNNNNNNNNNNNNNNNNNNNNNNNNNNNNNNNNNNNNNNNNNNNNNNNNNNNNNNNNNNTGTGCAGGGTGTATGGAGGCCCTGGG
GCTGGAGCCTGCGGGGCTTCTTCCAGGGACAGTGAGGCTGGAGATGGACTGCGGAGATGAGGGTCTAGAAGGTGGTGGCG
GGGCATGTGGACCGTTGTAAGGGCTCTGGGGTTCCTGGGTGGGCTGGCGAAGTCCTACTCACAGTGACCAACCATGATGA
TGGTCCCGATAGAGGAGGAGAGGGAGGAGGAGGGAAAAGGAAGGGTGAGGGGCTCAGAGGGGAGAGCTGGGAGGAGGGGA
GACATAGGTGGGGGAAGGGGTAGGAGAAAGGGGAAGGGAGCAAGAGGGTGAGGGGCACCAGGCCCCATAGACGTTTTGGC
TCAGCGGCCACGAGGCCTCCTCAGCTCCCGCCCCAAAACGGAAGCGAGGCCGTGGGGGCAGCGGCAGCATGGCGGGGCTT
GTCTTGGCGGCCATGGCCCCGCCCCCTGCCCGTCCGATCAGCGCCCCGCCCCGTCCCCGCCCCGACCCCGCCCCGGGCCC
```

```
GCTCAGGCCCCGCCCCTGCCGCCGGAATCCTGAAGCCCAAGGCTGCCCGGGGGCGGTCCGGCGGCGCCGGCGATGGGGCA
TAAAACCACTGGCCACCTGCCGGGCTGCTCCTGCGTGCGCTGCCGTCCCGGATCCACCGTGCCTCTGCGGCCTGCGTGCC
CGGAGTCCCCGCCTGTGTCGTCTCTGTCGCCGTCCCCGTCTCCTGCCAGGCGCGGAGCCCTGCGAGCCGCGGGTGGGCCC
CAGGCGCGCAGACATGGGCTGCTCCGCCAAAGCGCGCTGGGCTGCCGGGGCGCTGGGCGTCGCGGGGCTACTGTGCGCTG
TGCTGGGCGCTGTCATGATCGTGATGGTGCCGTCGCTCATCAAGCAGCAGGTCCTTAAGGTGGGTGAGGGAGACCCCAGG
GGGTCCGCGCACGGACCCGGGCTGTTGGGCGCTGGGCGCCGGGAGGACCCGCGCGTTGCGGTGGGTGGGCGACCGCAGCG
GAATCGGCGCCCGGGCCTGGCGCCGCAGAGCACGAGGGAGGCCAGGCGCTTCGGGAGGGGCTGCTGCCCGCCTCCCCACC
ACCCTCACCGCCCCAGACTGAGGCTAGGGGGGAACCCAGGGGCTTTGCTGGAGGCGCAGGCCAGGGTTGGGGAGGCCTGG
ACGGGAGACCACCCGGGCGCCTGACCCGCCCTAAAGGGTCTGGGCGCCCACGACGGATCAGGAGTGTGGACAGGTTGCTT
CCGTGGTGCCGCCCGAAGGAGCCCAAGAGTGTGTGTGAACGTCTGCTTTCACAGCAGCCGGTAGCAGGGTCCGGTAAAAT
TAGAGCTGCACTTTGCTTGTATCCTGTGAGCCCAGGAGGACAGGAACCTTCTGTGAGACCCACGTGGGTGCCCAGCTCAC
ATGGTAGGTGCTCAGCTGAGCATCCTCCACTGCCCAGAATCTCCCCATAGGAACCCTCTCCCAGAGGCAGAATGCGCGAC
TCTGTTCTCCCTGCTCACTGCCTCCATGATTCAGTCTGGAAACTATAAGCCGCTTTTCTCCCCAGATTCATGCAGAGGCA
GGCAGCAGCCCAAGGTCCTCTGAGTGCACGCAGCCTTAGGTCTTTCCTCTGATCCTAACTTTGCATACCATTGCCCTTTC
AACCAGGGAGACAGATTAGGCGGTCAGCTGGTCTGCAGACGGACAGACTCTGCCCTGGGTGCCTGGGTGGCCTGTTTGAA
TGAGCCTGGAGTTAGGGGAAGGGCTGGGAGATTGCAGAGTATCACATTTGAACTAAAAGCCATTTAGAGTTTTGAAGCCC
TGGAACCCACACATGCAGTGGCGGGGAGGGGAAACTGGGGCTCACTCTTATCAAAGTTCTAGCCAGATGCAGAGCTGGGG
TGAAGATGTGGACATTGTCACTCTAATGGACGTCTTTTCCCCTTGGCCTGCCTCTTTGGACTGGGTTTCTTAAGAATGGT
TCCTTGTCCACATTTCATGTCTGTTCCCTCATTCACAGAGCGAATGAATGAGTGAATGAATGAAGGATCTGCTTAGATCT
GGCTGCCACTTCTTGGGACCCAGCTAGCTGGCCTTAAAGATGGAGCAACATATGGTCTCTCTCAAGGTCTCTGGGTGA
GCAGTTGTCTAGGGCATCTTAGCCACGTTGTGTCTATTTTTGATTTTTCAAATCTTGTCCCGCCAAAGCCTGTAATCTGA
CTGCAAAGAGGTCCCGGTGGATGGTGACACTGTCTCTGGGGATGGCAGGAAAATCAGCAGAATGGCCTCTTCTCCCCCAT
GACAGCTCAGAGGCTTTCTCTGTTACACTGAGGGAGCTTTATTTTTCTGTGGCCTACTCATACTGAAACGTGTGTGTCAG
TGACAGCCTTGTGGCTGCTGCAGTGCCAGGACCCCACTGTGTGTATGCAGGGTCCCTAGAGGGGCTATCACTCAGGCAGT
TCAAGCATGTGACCCTGTGCTGTGAGTGAGCACCTGGTCTCTTGCTATGGGAGTGTGGGGACTTATGCCAGGTAAAGAGT
CTTGCCAACTCAGTTATTCGGGGGCTTATTTCAAGGTGTTTTCTTTCTCAGGATGTCATCAGGTTAAGTATGTCAGTAGG
GTGTGTGTGTGTGTGTGTGCACCACACATGCATGCACCACCAAATTTCCCCACTCCTCTCCTGGGCTTCTAAAGAGGAAA
ACCACATTGTCCTCAATTGTCCTGGAGCTAGGAGCATCTGGCTTAAAGTTGAACAGGAGCCTCCCAGCATCCTTCTGTGG
ATATCCCAAAGGCGATTGGTTTGGGGCCGAGTGGTGTGAAGGCAGGAAGACACGCCCAGCCCAAGACGGCCACTTCTGGA
ACCGCCCAGGCTGGAGCAGCGGCCAAGTGCCTTAGCATGGAAGAAGAGAGCCTGTGTTCAGGGGACCAGGCGGGTGAGAC
ACACGCATAAGCAGGGACTGCCCGCTGGTGTTTTCTTTGGCTACATGAGTCTGTGTGCAACTTGAATGGGAGGCCTTGTG
CTTTTATTTTTTTTTTTTAATATGCTTTTTGGTTAACAACAGAAGTCCCTACAGTTGGCCCTCTGTGTCTGCAGGTTCTGC
ATCCGTGGATTCAATCAACTGCAGATGAAAAGTTTTCAGAAAAAATCCCCCCAAAATAACAATACAACTAAAAAAAGACA
TAAAAACAATACTGTATAACAACTATTGACATAGCATTTACATCGTATTAGGTATTATAAGTAACCTAGAGATGATTTAA
AGAATACGGGAGGATGTGCATAGGTGATATGCAAATACCACAGCATTTTATGTCGGGGACTTGAGCATCTGCAGATCTTG
GTATCTATGGGGGTCCTGAAACCAATTCCCCTCTGACATTGAGGGACGACTTTATATAAAATATATGAATACTTGCTCGT
AAAAAAAAGTTCAAATGGAGGAAAAGAGAGAAAGTTTCCCCCATTTTATTCCCCTCCTGAGCTATTATCACCATCATTGA
TGTGGATCTTTCTAGACCCTTTTCTGTGTACCAGGAGCCTGTATGTATACATATTAATATATATTTTACATAACTTGAAC
TAAGTGTAGGTGAACTTGAACATGTATAATTATCTTGCAATTTGTTTTTCAATTCAAAGATACCAGCTGAAAAATTTTCC
ATGTCTCAGCAATAAGTCTACCCCGCGTTTTCCTTCATCACTGCTGGTATTCCTTTGAATGGTTATAGACTTGTTTACCT
GTTTCTCCATTGACTGATCATTTTCTCTGTCACAAATGACAGCGATATCCCGACACATCCCTCTTCGTGTGGTTCATGAA
CATTTCCATAGGGTGGATTTCTAGAAGTGGGGCCTTGGGAGCAGGTGTGCTCAGTTTGAGTTTTGATGATTACGGCCAAA
TTGCCCTTCTGCACAGCTCTGTTGCTGCATCCACCCACAAGGTATGGGACTGTGCTACTTCCCTATCTCTGCCATCACT
GGTAATTACTGATTTATCATTACCATTTTTTGGTAGCCAAGTTCCCGTGTGTCTCTGGTCCTCCAAGGAAGGCCCAGCTG
GTGGACATGGGGGAGAGGCCCCTCACTGCAGCTGGGCTGCAAAAAAGGGATGCTCATCTTCCGAGGCTGCTGTCTTCAAT
GATGAGCTGGGGTGAAGATGTGGATATTGTCACACTAGTTGACATCTTTTTCCCTTTGGCCTGCTTCTTTAGACTGAATT
TCTTAAGAATGGCTTCTTGTCCACATTTCATGTCTATGTCCTTTTCACAGAACTTGGATGTGTGCCCCCAGGGATCTGAA
CCATGTCTTCATTATTCACCGGATCACCTGAGGTCAGGAGTTTGAGACCAGCCTGGCCAACATGGCTAAACCCTGTCTCT
ACTAAAAATACAAAAACTAGCTGGACATGGTGGTGCACGCCTGTAATCCCAGCTATTCGGGACGCTGAAGCAGGAGAAT
AGCTTGAACCTGGAAGGCTGAGGTTGCAGTGAACTGAGATCACGCCACTGCCCTCCAGCCTGCGTAATAGAGTGAGACTC
CATCTCAAAAAAAAAAAAAAAAAAAATGAAGGAACTTAGAAATATGTCTCAGCATGGGTAGGCCATAAAAACACATTACT
AATTGAAAAAAGCTCTGGTGCTATTTATGTTTAAAAAAAACCCACCAAACAATATTCTATATTTTCTTTCCATTTTTATT
TTTATTTTCGTAGAGACAAGGTCTCACTATGTTACCCAGGCTGGTCTCGAACTCCTGGCCTTAAGTGATCCTTCTGCCTT
GGCCTTCCAAAACACTGGGATGATAGGTGTGAACCACCGCGCCCTGCCATTATTTTCAATGGCTTAATATTAGAATATGT
TAAAGTTTGGGAAAAGGTGAGACTAACAGAAGTGGCCACTCTAATGAGAGAAGGCAGGACACTGGGGGTGTGGCCAAAGG
AAACCTAATACTTTATCTGTAATGCTATTTTTTTTTTCTTTTTTGAGATGGAGTCTCGCTCTGTCGCCCAGGCTGGAGTG
CAGTGGCACGATCTCGGCTCACTGCAGCCTCTGCCTCCTGGGTTCACACCATTCTCCTGCCTCAGCCTCCTGAGGAGCTG
GGACTGCAGGTGCCTGCCACCACACCCAGCTAATTTTTTTTTTGTATTTTTAGTACAGATGGGGTTTCACCGAGTTAGCC
GGGATGGTCTTGNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNCTGCCTCAGC
CTCCTGAGTAGCTGGGACTACAGGCGCCCGCCACCGCACCCACCTAGTTTTTTGTATTTTTAGTGGAGACGGGGTTTCAC
TGTTTTTAGCCAGGATGGTCTCAATCTCCTGACCTCGTGATCCGCCCGTCTCGGCCTCCCAAAGTGCTGGGATTACAGGTG
TGAGCCACCGCGCCCGGCCAAAATCCCATCTCTTAAAAAAAAAAATTACGAATGAACAGATACATAGTTTTCCTCAAAGAC
TGGCCAGATGAATGCGCCACACAGACCGATGAGGCACACTGCAAAGGGGCCCTGTGGTCGAGGTTGCTTGGAGAGGCTTT
GGGGAAGCGCAACTCATTTCTTTTCCCTTCTGCCAGTGGGTAGGATTGGAGAGAGGTGTTTGGACAGGCACACTAAGGGC
CGTGATTGTGAACTTCATGGCATCCATTCATGGGCAGCTTACTGTGCACCAGGCAGGGTTCAAAGCACCTTTGTGAAAAT
TGTCTCATTTAGTCCCTGCAGAGGGCCGTGACATATGATTTATTGTCTTCAGTGTCCGTTAAGGAGACAAGCTGAAGAGA
GTAATTTGCCCCAAGTGACCTCGCTAGTGTATGTGTGTGCGTGCATGTGTGCGTGTGTGTGTGTGAAGGTGTCTGT
CTGTCCCAGAGGCAGATGCCATGAGAATGGGGACTCTTGCTCCCATTGCCCCTGGATCTGAGCCTGGCATGCAGCTGTGCT
CAGGTATGTGGGTGAGTAACGAAGGCCTGGTTCAGGTCCTGGGGGGCACACATCCAAGTTCCCGTATGTGCTCTGGTCC
TCCAAGGAAGGCCCAGCTGGTGGACGTGGGGAGAGGCCCCTCACTACTGCTGGGCTGCAGGGAAGGGGTCATCTTCCAAG
ACCACCATCTTCAATGATAGGCCTCATGGCCTTCTTCCATGTCCAGGAGAGAACCTGGGTCCCCTCGCTGTCTTTAAGT
TGGTTTTCTAGATGGACTCAACCTCGGTTGCCTTGTCCTGCTGCTGGGCTTCCCCCACCTATGATCCCTGTCTCAATAAA
CGGCACCTCCTTTTGTTTCCCCTGGGGTAGAGGCAAAAACCTTCGGTCATCTTTGCCTGCATGCTTTTTTCCTGATACCC
CTGTATCTGCTGAGGCAGGGGCTTTCCCATCACTGTCACTGCAGCGTCCCCGAGGGCCATAGCAGCGCCTGAGCTCCTTG
CCACCCCTTCAACACCAGTCAGGTTACAGAATGAATGAGGCGGGCGCGACCCCCAGCCCTGGCCTCAGGCTCTTTAGCTC
ACCCCTGCCTCCCTCAGCCAGAGCCACACAGTGAGCCTTGGGGAGCCCCGGCACCAGGGCCAGCCAGGGATGTCACCGGC
TGTCCCTGAGTTTGTGCCTTTTGGGAAATCTTTGTCAATCAGTTAAAAGATGGGGGTTGATGGGAGATAACAGTCAATGG
GCACTCGTTGTGGCCGGATACTGTTCCAAGTGTTTATGTGCATTATTCATTTTAGTATCTAAAATAGGTACTATTGTGTC
TTATTTTATTGAGGGAAATGGATGCACAGAGGCGGAAAGTAACTTGCCCCATGTCACATAGCTCCAAGCGGTTGAATCAG
GATTTCAAGTCTGGGGGCCGACTCCACAGCTGGTCCCGGTGGCCTCTGCGCACTGCGTCCTCCCGGTTCTCCGTGTGTCT
GAGCAGGTCGACTGCATGCTTAAGGCGGGCGGCGGGGGGTGCCCTTCTTATATTCTCTTCCTGGGCTGCCCCTTCCTTTT
CTGTGCATCACCAGGGAACAGCAGGGTCCCTTCGAGGCTTTGTCCTAATCATCACCCCGCCAAACTTTGCTGGTGCAGAG
TGCACCCTGGCCTCTCCCTTCCGGGGGTAGTCTGAGACCAGGGACATGTTCTCCAATCAAAGGCACCAAGTGAGGCTCCC
GGGGAGGCCACCTGTTCCTAGCCCAGGGCTCCCAGTGACCCCGGGGGCTGGACTCCCACCCATCCTGCAGGGGCCGCCCG
CTCTGCGCCTTTATCAGCCCCCCTGCGGAACCTGGGGGGGGAGGCCCAGTGTCTCCACCTACCGGGCGTTTCCTGTAGGAC
CCCCTTGTTTGGACATGTGGGGCAAAGCCCTAGAACTGATCACTGGGCACAGGTGGGGAGGGGGCTCCTCGAGGACAGCT
TGGATCACAGGTGTGTGCCAATTCTGTTTTGTTTTTCTTCCTCTTTCCTGCCATTGAACTTCCTACTCCTTCTAGCATGT
GAGAGACAGCTTCAACTTACTCCCTGGCGACGGCTGAAAAACATGGGCTATTTTTAGTTTGTGTGAGTCTAGTTTATTTG
CTCCTGGTAGATTTGCCTCTCCCTGTCTTCGTGAGTGTGTGTGTGTGTTTGCACACACGTGTGTTGGTGTGTGTACACAAGT
GCTGATGGTGAAAACAGTGACACACACACCTGTGTAGGCATGCACACACCTGCCTTGATGTGTGACGACGTGATGATGTG
TATGCAGACACCTGTGTTGATGTGTGCCTACATATGTTGACACAGGCACACATGGTGTGTCTGTACATGCAAATGGACAC
ACATGAACACACGTGTTTATGCATGTCCTTGTGTGTTGATGTGTCTGCACATACATGTTGATGTGTCCCGCTGTGGTTGG
CCTTTCTGGGGCCAGGCCCAGCACCTGGGGTTTCCAGGAAACATTCCCTGTCCCTTCCCGGAATGGCCGGTACTTGCTGT
GCCTCCGCCGGGAGATGCACCCTGATTAACTAGAACGTGGCGAAGCTCAGCCACCTGGAATCGCCTTACCCTGGCCTTTG
GTTTCTAGCAAATGGGATGGAAGTGAGTCCCCATGGAGGGCTTGCTCCATGAAGGTGTTTTTCCTGGATGAGTCTTGGTC
CCAGTGGCGATTTGCTGCAGGCTTGTTGGCACTGTCATTCGTGGGTCCTGCATGGAGGTTCCTTAGTGTCCAGAGGGTGA
TTCCATGTCTGTGGTCAGCAAAAGAGAGGCCCCTGGGGCCATAAGCCACACCCCCCAAGGCTGAACCCAGAGAGTTTGTG
GTTTAAATCTTGGGCCTGGAGGATTCTGTGGAGGAGAGTTCGGTCCAGGCCTTCTGCTCCAGGAGGCGCTGGCAGCTCCG
ACACCACAGACACATTAATTGTGCGGAGAATTCAACGCTAGGGCTCAGTCAAAAATCAAAAACGCAAAACAAAAAGAGAA
TCCCTTTTATTTACCTCGTCTCCCTCCCCCTGTTTGGACATGACCTTGAGCTCTGCTGGCCCACAAGTGGCCAGGCTGGA
GCTGTCTTGCAAAGAGAAACAAAAACAACACAAAAAGCTGCGTTCTTGACTTTGAAGCCTTAAAGGGCCCAGGGGCTGGC
TCCCTGTGGGTGTTGGGGGCGATTTTCCTCCCAAAGCCTGCGTGAGATATGGAGACACTCCGCCTGCTCAGAAGCTCCCC
AGGTCCCGGGTTCCAGGAAGCCTCATGGCCCAAGGCCTGGCCGTCTTAATTTTCTTGTTTTCTTTCCCTCCACTCCACTG
TTTCTTCCTGTTCACTGATTGCCTGTGCTGGGGAGGGGGAAGGAACGGGAGGGGCCTGACCTTGTTCACTTCCTGGCATT
TTGGGTGAAGTTCCTTACACCTTCTCTGGAAGCTTCTAGAAGGACCCTTGAGGCGCTTTAAATCTCCATTCTCTGGGTTT
TCCTCCCACCTTCCTGGCTGCTCTGTCTTCTTGCCTGCTGATGCCTCCTCATCTCCCTGGCCTCCTGCCTCCTGCTGGTG
GAGCTCCAGGCATCCTTTCTGTGTGTCCTCATCCCTCATCCCTGGGTGGGCTCCTCCAGCCTTGCGCCTGAATGGCATC
TCTGTCCTGACCACTCCCAAATTGGTATCCCTGGGCCCAGACCTTTCCCCTGAACCCACCCCCGAGTGTCCAACAGCAG
AGTCCGTATCTCAACTCGGGGTCAAAGGCAGCTCACGCTCGCCCCGGCTGGCTCCTCATCACCCTCGGCCACACCTGTGG
CTCCCACCAGCTTCCCACTCCATCATAGCCCCCCCATCTTCCAGTTGCCCGGGCCAACATCTTGCACTCAGCCTTGACTC
TGTTCTCTCCACTTCCCTCAGAGAACCCGTCAGAAAGTTTTCTAGCTTCATCATCTAAATATACCCAGATTCCAACTACT
TCTCACCACGCCCGAGGCGCCATCACCTCTGGTCCAAGGCACCATCACCTCTCCCCCAGATTCCTGCATCCGCCTCCTCA
CTGGCCTCCCTGTGCTCATCCTTGCTCCCCAGTGGGGTTTCTCAATAAGCAGCCATGGGAGCCCATCAAGATTGCACTTA
GCACCTCCTCCCCTCAAAGCCCTGACATGGCCAGACCCGTCCCCTAGGAGTCACAGCCAGGTCCTAACCTGGCCCAGGGC
```

```
CTGTGCCATCCACCCCTGCCACTTTTCTGGCGACGTCCGCATTCCCTTTTCCCTTGCACAATGGCCTCCTGGCCATTCCT
CAGCCCCATGGGCCCAATCGCCTTAGCTCTCCTGACACAAGCCCTCTCATCTCCCGTAGGTCTCTGTTCAGCTCTCACTT
GCTCACGGAGACCCTCCCCTGATTCCGTTTGTGCATCATGCAGGTTCCCCCTTGTCCCCCTGTGCTGCCAGCCCTCCTTA
CCCGCTCTGCTTAGACCTGTCAGCCACTGTAGATACCCTGGAACTTCCCTGGGTATCATGTTGATTGTTTATTGTCTATT
TCTCCCCATGAGAATGTCGGACTGTGAGGGCAGGGACCTTTGTGTTATTCGTTGCCGTGACCCAAGCCCCTGGAACAATG
CCTAGCACATAATCGCAGGTTCTCAAATATTTATTGAAAGGATGTTTAAAAGACAAGGGTTGTTTCACAGGATGGTTCGT
GACCAGTGGCAGGTTGTGGTCCTGTGCAGGGCAATGGGGCCTACTCCTCCACCTGCCGTGGCCGTCGCCCCAGACCGAGC
TGCCACCGTTGTTTCCCAGTGGATGCTGGAGTGTCATCATGGGGCTGTTTGCTCTGCTGATTTATACCACACTGTTGTCA
CTTCTCCCCATCAAAGCCAGGATGCTCTTTAAAACATGCAAATTGGGCCGGGCACAGTGGCTCATGCCTGTAATCCCAGC
ACTTTGGGAGGCCGAAGTGGGTGGGTCACCTGAGGTCAGGAGTTCAAGACCAGCCTGGCCAACATGGTGAAACCTCATCT
CTACTAAAAATACAAAAATTAGCCAGGCGTGGTGGCATGTGCCTGTAATTCCAGCTACTCGGGAGGTTGAGGCAGGAGAA
TTGTTTGAACCCGGGAGGTGGAGGTTGCAGTGAGCCGAGATTGCACCATTGCACTCCAGCCTGGGTGACAGAGCGAGACT
CCGTCTCCCAAAAAAAAAAAAAAAAAAAACAAACTATGCAAACTGGATCATTTCAAGAAGCTGAATAAAATCTTCCTGCA
TTTGGTATCAAAGCTGTGAGCCTCATTTTCGTGGGCCCTGCCCATCTCAACTCAGCATTCTCACCCCACTGCAACCTCAC
AGGCCTTCCTTTTCCTCCAACGTGTCACATGCTGTCCTGCCTCAGGGCCTTTGGAAGTGCTTTTCTTTCTGCCTGGACCG
TCTTTTTGTCTCCCTGGCTCTTATGCTTGGTTCCTCTTTTATGTTTAAATGTAACCTTTTGAAGCTTAAATGTAACCTTT
TGAAGAAGACACCCCGTGATTGCCCACCCCACCCAATGGACAGTTGCCCCCCACGTCCACTGTCTGTGCCATCTGTGTGG
TTTTTCAGCTGGAGGGCAATGGCCCCATCTCGGCTCACTGCAACCTCCTCCTCCCCGGTTCAAACGACTCTCCAGCCTTG
CCTCTTGAGTAGCTGGGATTACAGGCGTGCACCACTATGCCCAGCTAATTGGTATTTTTAGTAGAGATGGGGTCTCACC
ATGTTAGCCAGGCTGGTCTCGAACTCCTGACCTCAGGTGATCCACCTGTCTTAGCTTCCCAAAGTGCTTGGATTACAGGC
ATGAGCCACCGCGCCTGGCTTGCGCCATCTGTGTTATAACCACACCATGCTATTCCCTTCTGAGCCCTTAATAGTCTCTG
AAATTATCTCATCCACTTGTGTGAGTTCCATGAGGACAGAGACTTTATCTGTCTCGTTCATGGCTGCATTCCATAGTTGG
AGACCCTTGTCCAGGGCAGAGAACCACCTAGCGTAGGACCTTGGGCTAAGGCAGAGAGGCCCCAGTGGGGGTCCTCCGCA
GCGTGGACTTTGGAACCAGACTCCCTGAGTTCAGCAGTGGCTCTTGCACTTCCTGTCTCTGTGGCTGTGGGTGAGTTGCA
TAACCCTGCCGGGCGTGTTTCTCCGGGCCTCCCTCCTCGGGCTGTAGCAAGGGGTAAGGGAGGTCATACGCCCTGGGGAA
CACCCCAAAGCCTGGGTCCCTGGGATGCATCCACAGATTGTAGGCCTGAGTCTTAACACACTCCATCTGGATTTCAATGA
CTTCTGCCTGGTTCCAGTGCTGGGCTGTTTCCCTGTCTGAGCTTTGGTGGCTTTTGATTGACCCCTTTCCTGCCCCAGCA
TGAAGGTGGAGTAGCCAGAAGGAAGGGATGCGGGTGTATAAAGATGTCATCGGGGCTGTCTGTGGATGGTGGGTAAAGAG
ACAGCCACCTGTCATGGTGGAAGGGCCACAGGAAGCGTGGAGGCTGTTTCTGGGGCCACCTCCTGCCTCCAGGAGGAAGCA
GGGATCCTCCTGTTGGCATCTTGGTCCCATCAGGTGTCTTGGATGGGGCTGGGGCTGGGAGTGTTTCTCTCCCTGAGCAT
TTAAGCCAAGGTTCATTATTTAGAATGGAACCATTTATAGGCCGGGCGCCATTATTACATTATTCCTAAAGTGTTAGGAT
TACAGGTGTCATGCCTGTAATCCCGGCACTTTAGGAGGCTGAGGTGGGTGGATCACTTGAAGTCAGGAGTTCGAGACCAG
CCTGGCCAACATGGTGAAACCCCATCTCTACTAAAAACACAAAAATTAGCTGGGCATGGTGGCACACACCTGTAGTCCCA
GCTACTTGGAAGGCTGAGGCGGAGGACTGCTTGAACTCGGGAGGTGGAGGTTGCAGTGAGCTGAGATCGCGCCACTGCAC
TTCAGCCTAGGTGACAGTGAGACTCCATCTCAAAAAAAAAAAAAAAAAAAAATGGAATCCTTTATGTTTCACTTTGTTCTCA
TTAGCAGGACTGTGCGAGGCTGAGTCAGGAGACCGAGGTCCATTCTGCTTGTGATTTTGCTCCGTGCAGGAATGTGGAGG
TGTGGCCCATAAGCATGTCATTCATGCAAAATCATCTACATGCATTCAATTAGAAAAAAAAAGGAAAACAGCAGTGACCC
TCATAAGTAGACTTGGTTAGTGTCAATGTAGGGAGTGAGTTTCTTGTGACCCTGCTGTTCTTCCTGGGTCCCGTGACAGG
GCATCTGGACAGGCTGAATGCGATTCTAGAATTAGATTCCTATGTTTTTTATATGTACGCCCCAGTCTCACTTGCCAGTGG
CTTCCTCTGACGCTTAGAAAATAAATGGCATCTGCACCACGTCAGGGAGAAACTGACTGCTTTGGAATTCCCAGAAGCTC
TTGGAAGGGCAACCTTCCTGAGGGATATTGAAGGAGAATTAAAGAATTTTCTCAGTGGCTGGCACAACTTATGGAGTCAAT
GCTCATTGGCTGTGATTGGCTGAGCTGGGATCACATGTGCATCCTTGGACCAATTGCTGTGGCTCGAGTAATGGACTGCT
GTGATTGGTCAGCCTTGGGTCGCCTGCCCATTTGGAGCCAGGGAGGGAAGTAGTTCTTGCCCACAGAGACTCAGGTAGGA
GTAGGGTGGTTCCCAAAAGGCAAAAAGGAGGGGCTGTTGTCGGGGGAAGAGGCGGCCATAGGCAGCAAAAATGGGGCTGA
GCCCCTTTGCCCCCAGGTAGCTTCCCACTTCATCCCCACATTGCCTTTCACTGTCTCTGGCTGTTACAGTATTAGAGATT
ATTTTGTTTGGCTGACTATTCTGTTTACCAAGGCCTCTTGAGGAGGTAGTAGCTTACCAGCTGTTTATTATGTATGTATT
TACTTATATTTACTTACCTATTTATTTTGAGACGGAGTTTCTCTCTTGTTGCCCAGGCTGGAGTGCAATGGTGTGATCTC
GGCTCACCGCAACCTTTGCCTCCCAGGTTCAAGCGATTCTCTCACCTCAGCCTCCCAAGTAGCTGGGATTACAGGCTGCG
CCACTGTGCCCAGCTAATTTTGTATTTTTAGTAGAGACAGGGTTTCTCCATGTTGGCCAGGCTGGTCTCGAACTCCTGAC
CTCAGGTGATCCGCCCACCTCGACCTCCCAAAGTGCTGGGATTACAGGTGTGAGCCACGAAGCCCGGCCTATTTATTTAT
TTTTTTGAGACATGGTCTCAGTCTGTCGCCCGGGCTGGAGTGCAGTGGCATGATCTTGGCTCACTGCAACCTCCGCCTCC
CGGGTTCAAGTGATTCTCCTGCCTCAGCCTCCTGAGTAGTTGGGATTACAGGTGCCCACCACTACACCCGGCTAATTTTT
GTATTTTTAGTAGAGGCGAGGTTTCACCAGGTTGGCCAGGCTGGTCTGGAACTCCTGACCTCAAATGATCCACCCACCTT
GGCCTCCCAAAGTGCTGGGATTATAAGCATATGCCACAGTGCCCACCCTTTATTATTTATTTATTTAGATTTTTTGAGAC
AGGGTCTCACTCTGTCACCCAAGCTGGAGTGCAGTGGCATGATTACGGCTCACTGCAGCTTCAACCACTCAGGCTCAATC
GATCCTCCTACCTTGGCCTCCTGAATAACTGGGACTACAGGTGTGCACCACCATGCCCAGCTAATTTTTAGATTTTTTGT
AGAGACAGGGGTCTTGCTATGTTGCCCAGGCTGGTCTTGAACTCCTGGCCTCAAGCAATCCTCCCACCTTGGTCTTCCAA
AGTGCTGGGATTACATTCATGAATCACTGTACCTGGCCTAATTGCTCTAACGGGGAAAGTTTCATCAGCTGTCAGATTGT
TAGATCTGGACTGACTTGGGTTCCGAAGCTCAGTTCTATCTTTTATTTTTCCTTTGGAAACCAAATTCTGTAGGTAGCAG
AGGTTTACTCAAGGCCACGGAGATAGTTAGCAGCCAGTGCTGGGTGGGGCCGACTGTCCCAGGCTAGCTGGATGGAGGCC
AGGAGGGCCCGGGCAGCAAGGGTGGAAGGGGCCTGGGTGGGTGCCGGAGGAAGTCACTAACGAATGTTCTTCAGGCAGAA
```

```
AGTTCCCAGGGTCCCATTGATACCCCTGCCTGTTCACATCCTGGCGGCAGGACTGGGATGAGGCATTTTTTAAAAAGTTG
GGGAGATATCAGGGCTGGTGTGCCAAAGTTCAGGCTTTGTCCACCCAAGGTGAGAGGCTGCAGGGGTGTGCCGTGGGCAG
AGAGGGGATGAGGTAGGGGTGGGAGGGATGAGCTGCCCAGATGCACCCGTGGAGGAGTTGATCTGGAATCCTAGCCACTG
GGTTTACCCTGCAGTGTGTCCCAGCAGCATCCCAGGCTTCTCTCCAGCCTTTGGCAGGGCTTTAGCTTCCAGCAGCTCAG
GTGGACCCCAGCATATGGACTTTGAAGGCAGGCAGCCCAGGTTCAAATCCTGGCTTGGCCACTTCCCAGTTGGGTGCCTT
CAGGCAAGTGACTTACCCTCTCTGTGCCTTTTTTACTTTTCTGTAAAATGAAGTTAATAAAAGTGTCTCCTACATAGGGC
TTTTAAGGTGGCAGAATTGGCACATGTCACACTTCAAATAGCAGCTCGGCAAGTGTCAAGTAAGGGCTGGCTGTTTCCAA
CATCTTAACCGGATAGACACAGACATAGAGAGGGATCCATCTGTCAGCAGTTTGCTGAAATGGGACCATACTATGCACGC
TTTGCTGCACCGTGGGTTTCTCACGCTGTCATCGCTTGTCCATGTCGTCCTCAGTCAGCTGGGATTTTCTGGTGATGATC
ACGCTCTGATTCTCTAGATGCCTGGAAACGCTCCACGGTGCGGATGTACCACGGCTCGTTCTGCCGTTCTCACGAGGGAG
GCATCCAGCTTGTAAAATAAAGCAGCCCAGTGTGTTCTTCCACCACCACATCACCCAAGGTTCTATTTTCAAAGATTTTA
TTTTGTAAAAACGGGAAGAAAAGTACAAGTTGCTGCATGTGCATCCTCTCTCTCGTTGCTGAGCCTTTTGGGTGCATC
CTAAGGACAAGGACGTCCTTCTCCATGGCCGTGGACCACGGCCCCACTCAAGAAGGCCACGAGGATCCACACCTTTAATC
TGACAGTCTGTGTTCAGATTCCCTTACGGCCCCAATAACACTTTTTGTCATAGTTGTTCATTAAAAAATTTTGAATTGGC
CGGGCGTGGTGGCTCACGCTTGTAATCCCAGCACTGTGGGAGGCCGAGGCGGGTGGATCACAAGGTCAAGAGATTGAGAC
CATCCTGGGCAGCATGGTGAAACCCTGTCTCTACTAAAAGTAAAAAAATTAGCCAGGCATGGTGGCGCACACCTGTAGTC
CCAGCTACTCGGGAGGCTGAGGCAGGAGAATCGCTTGAACCCAGGAGGCGGAGGTTGCAGTGAGCCGAGATCGTGCCACT
GCACTCCAGCCTGGGCGACAGAGTGAGTCTCCGTCTCAGAAAAAGAAAATTTTTTTTTGAATCCAAGGTTGCATGCTGTG
TCTGGTTGCCTCATGTGTTTTTCCTCTTTAATCCAGACCTGTTCTCCAGCCCTTTTCTCTTTGCTGGGTCTTTCAGGACA
CTGGCATCTGTAAAGGGGCCAGGCGGGTGCTTTTGGCAGGATGGCCCTTGATTTGGATTTGTCTGATTGCTTTCTCAGGA
GTGCACGTGGTCTTCCCACAGACTCCCACACAAAAGCCTCATTGTGTGAATCAGATTGGAGCAGAGTGTTTCTGATGAGC
CGGGGTTGGGTGCCTGGATCCTCCCCCAGCTCCCAGCCTGGCAGTCACAGTGCAAGTCTGAACCTCTTGGGTTGAAGAAG
CCTGCAATTCCTCCTGGTCCCTTCCTCCACCTGCTTTCTAAAATTTCTGGCTCTCATTCCTTGCCTTTGAGCGGTGATGT
GTCCATCCTTCAGCGGCCCTCGGCCAGCCTCTGCCCACCCCAGTGCCATCACATGGGAAGAGATGGCGGATCTGATCCCT
GACTCATCCCCTGGGCCAGGAAGATTGTGGAAACCCTGGGGTCTCAGGGCTGGTCGGGTGGAAACGGGAGGCTGGATGGA
GTGGCAGGTGGGGGCTTTTCCTGTTTTGCCTCTCCCTAACTGGGCCTCCCGGCAGCCACTAGCCCAGATGCCCAGGCCTC
AGAGCCTTGTGGAAATTCTTGTGCCAGATCATCTTGGCCCTGTCTGGCCTCCTGGCAGGCAGGTGGAGGTCAAACTGGCC
TTACTTGGCCGAGAGCCCATTGCAGCCAGGATCCCTTGGCAGCTCTGGGAACAGCCAAGTCTCCCCTGTCCTTGGGATTT
CCTTCCACTGTCTGTTCCCAGGCTCCTGGACTCCACCTGCTGCAACCCCTCGCTTTGCACTTTCCACCCAGAGTCTTGGA
TTTCTGTTTTTTGTGAAATTTGCTCACTTTGAGGAAAGTGTAAGACATATGCAATCAAATATGTAACTAAGATGAACATG
CATGCAATCACCCCACGTGAAGAAGAGGGAAAGCTCATCTGCCCCGAAGCCCCTGCAGAAGACACTGTGTTTGGTCCCAG
CTTGCTTTGCTTTTTATAGTATCACTCCCTCATAGCCATCCCTAAACACCGGGATTAGTTTGGTCCGGTTTTGGACTTAA
CTTGGATGCAGGCATACTCTCAGTATTTTTGTTTGGTTTGGTCTTGCTGGTGTTACTTCATATGGTATTTGTGGGATCCA
CCCACATTGCTGCGATAAGCTGCACTCTATTTTCATGGCCATGTCATATCACAGTTCATGATGCTAATCGGAAGAAGCTG
AGTATCACAGTTCATCTGTCTTTTTTTTTTTTTTTTTTTTTTTTTGAGATGGAGTCTTGCTGTGTGGCCAGGCTAGA
GTGCAGTGGCGCGATCTCAGCTCACTGCAACTTCTGCCCCCCGGGTTCAAGCAATTCTCCTGCCTCAGCCTCCCAAGTGC
ACCACCATGCCCAGCTAATTTTTGTATTTTTAGTAGAGATGGGGTTTCACTATGTTGGCCAGGATGGTCTCGACCTCTTG
ACCTCATGATCCGCCTGCCTCGGCCTCCCAAAGTGCTGAGATTACAGGCGTGAGCCACCACGCCCGGCCAGTTCATCTGT
TTTAACCCTGATGCCGGTGACTGTGTTCACTTCTGGGTTATGACACACTCGTGTCCCTGCCTGCAAGGGACATGTGCCTG
AATCCCTCTAGGGCCCGACAGTCAGATTAGGGGGTCTGGTCATGGCGTGCGTGTCTTCACCTGCATGGAGAAGGGCCAAG
CTCTTGTCCAAAGTGGTTGCAGCGTTTCCAATCCTATCATTTGTTTTTCATTTAATAATTTATTGAGTTCAAATTCACAT
AACATATAATTAATCCTTGTAAATCGAATAATTCAGTGATACCACCACTTCTACCTAGTTTCAAAACATTTCCATCCCTC
CCAAGTAGAATTGCCCATTCTCCACTCTTTGCAGCTCCCCAGCCCCTGGCAATCACTCATTTTCTTTCTGTCTCTACAGA
TGCACCTGTTACGGACATTTCTTTCTTTCTTTCTTTTTTTTTGAGACAGAGTCTTGCTCTGTCGCCCAGGCTGGAG
TGCAGTGGTGCAATCTTGGCTCACTGCAACCTCCGCCTCCCGGGTTCAAGCGATTCTTCTGCCTCAGCCTCCCGAGTAGC
TGGGACTACAGGCGCCTGTCACCACACCCGGCTAATTTTTTTTTTTTGTATTTTTAGTAGAGACGGGTTTCACCATGTTG
GCCAGGCTAGTCTCAAACTCCTGACCTCAGGTCATCCACCTGCCTTGGTCCCAAAGTGTTGGGATTACAGGCCTGAGCCA
CCGTGCCCGGCCCTGGACATTTCTTTTTTTTTTGAGGCAGAGTCTTGCTGTGTCACCCAGGCTGGAATGCAGTGGCGTGA
TCTCGGCTCACTGCAACCTCCGCCTCCTGGGTTCAAGCGATTCTCCTGCCTCAGCCTCCCCAGTAGCTGGGACTACAGGC
GCCCGCCACCACGCCCGGCTAATTTTTTTTTTTTTTTTTTGTATTTTTAATATAGACGGGGTTTCACATGTTGGCCA
GGCTGGTCTCAAACTCCTGACCTCAGGTCAAACACCTGCCTCGGTCCCAAAGTGTTGGGATTACAGGCCTGAGCCACCGT
GCCCGGCCCTGCACATTTCTTATAAATGAAATCATGCAATGTGTGGCCTTAAGTGCCTGGCTGCTTCCGCTTAGCGTCAT
GTTTTGGGGTTCATCCGTCGGTGTGTGGCATGTGGAGTGCTGCTTTCCTTTTTGTAGCTGAGCAGGCCTCCACGGTGCGGGTG
CACTGCCATGCTGTTTTTCCGCGCCTCTGTGGATGGGCGTCTGGGCTGGTGGCTGCCGGGAGCGGTGCTGCTGGGAGCGCG
CATGTTCGGGAACTCGCTTGGCTTCTGCTTTCAGTGGTCTCCTTTTATGCCCTCCTTCCCCTCACCCCTCTCGCTCTTCA
GCCCGCCCTGCCCCTGCGCCCATCCTCTGTGTGGCGTCGTCTGTAAACCTGAGTGTGTCCTTGGACCTTGTGCAGTCCTA
GATTCCGGAGCCTGCAGGTGTGGGTCCAGATCCCGGCCCTGCCTCCTGCTGTGTGACCATGGACAGGCTCTTCGAGT
CTCTGGTCTCTGGTGGGTGTCTGAGTCTGCATGGGAGGCAGAGGTTGCAGTGAGCCGAGATCGTGCCACTGCACTCCAGT
CTGGGCAACAGAGCAGGACTCTGTCTCAAAAAAAAAAAAGAAAGAAAGAAAGAAAGAAAAAAATGTCCATAACAGGTGCA
TGGGCCAAAGACCATGTATTCCCGTACAGTTCTGGAGACCGGAAGTCTGAGATCAGGATGTTGGCAGGGCTGGCTTCTCC
GGAGACCCCTCTCCCGGCCTCATAGACGCCACCTTCTCCCTGTGTGCGCACATCCCCGGTGACTCTCTGCGGGTCCTAAT
```

```
CTCCTCTTTTAAGGGCGTCAGTCAGATTGGATTAGGGCTCACCCCACTGACCTCATTTAAACGTAATCACCGATTTAAAG
GCCCTGCCCCCGCGCCAATACAGTCACATTCTGATGCACCGAGGGTTAGGGCTGCAACATGGGAATTTTGGGGGGACAC
TGTTCAGCTCATAATAGTAGGGTGCCTATAAAATGGGCTAAAACCATGCCTTCCTATAGGATTCAGTGAGGACCGAGGGA
GCTAGAGGTGGAAGTGCTTTGAGCTGGGGGTGGCACAGGGAGGCCCCCTTCATGTGGCCTGTCACGTCTGAGGAGAGGGC
GGCTTTTCCATGCCATGCACGGTAACTCGTGTCAACATTCCTCGTGCTGCCCTGTTTAAAATCTATCCGTGTTCCTATGT
GTGTATCAGGCTTATTGCTTCCTTCTGCTGCCTGGACCCCCTAATTCATGTTCATCCTGTTGTATTTATTCCCTTAGCGA
CGGGGGTACCTTGGTTATGTCTGACTCCTGCCTCCACAAACAGCAAATATGCTGGACCCTGCTGCCTTGTACTTCTCCAC
AATTTCCTGGGGATGCGCAGGAGTGGAATTGCTGTAGGGCAGAGGGGGAGCCATTTTCACCCAACATGCCTTCCAGAAGG
GCCACTGCTGTTCACCCTCCCAGCTCTCACATCCTCTCCAGCACCTGAGACGATTTAGTCTTCGAGAATGTCTGGGGTCT
CAGTGAGGGTACGTGGTGCAGCATTGTTCTCATGTGCTTTTCTGAGGTGTGAATTTGAGTCTTCTTCCTTTCATAAGTCC
TCAGGTGGGGGTCTGAGGCTCAGAGATTTTGAGGGCCATGTTGGAGGGTCACAGAGCAAACTGGGCTAGACCCCACCAG
CTTGGGCTTGGAGGGCCTCCAGCTTGGATGTAGGGCCCTTCATAAATCCAGGATGATCTCATCTTGGGATCCTTAACTTG
GTAACATCTGTAGAGACCCTATTTTTAAATAAGGCCACATTCTGAGGTTCTAGGTGGGCATGAATTTGGCAGGGGACATG
CTTCAGACCAGCACTATGTGTGCAGGTGCCCAATCCTCAGATCTGCAGAGCTCTGGGTTCAAATCCAGGTCCCATTACTT
GGGTCCTGTGGAAACTCACTTTTTTCCTAAAAGCCGATTTCCACCTCTGCACATGGGGCCAATGTTCTTCCATCTCACAA
ACCAGAAGTCAAGGCCAGGCGCAGTGGCTCATGCCTGTAATCCCAGCACTTAGCGAGGCAGAGGTGGGAGGATCGCCTGA
GGCCAGGAGTTCAAGACCAGCCTGGGTAACATAGTAAGACCCCCCCCCCCATCTCCACAGAAAGTAAGAAACAAAAACA
AAACAAACTGGAGGCCAAGGAGAGCAGCTGCGGATGGTCACGTGCCTCTCCCTGGGTGTGCCCAGCTCTTACCCAGAAAG
CCCTGGCTGGCATTAAAAATAAGGAAGGTAAAATAGGGATATGATCAAACAAATTAAAAGTGTACGGAAAAAGAGTCAGC
CTTCTGGAAAGATCACAGCACGACACTGCTCCCCTGGCCCCAGCTGGTCTTTGCTCCAGAGGTGACTTGGGTTAATAGTT
CCTTGTTTTTCCTTCCTGCAATAAACATGTTTGCGTCCACAGAGGGGGACCCACACCCGTATCCTTCCCGTCCGTGGCAC
ACGTCCCACAAGGGCACACGGTTCCCATGCTTTTCTGCCTCTTGGCTGGGCACCTAGGTGGATCTTGGCATCTCTTGTT
CCCGCACAGAACCCATCCTCATCCGAGCCCTGGGCCTCTGTCTCCCAGCAGGCTTTGCAAATAGGACCTTGTAAAGGACA
GGGCTGTGTTTCCCAAAGGCCAGAGCAGTGAAGAGGGGCATGTGGGGTCGTGGGGCACACCTCTAGGACATTGGGGTCCA
GGGGTCCTTTCCAGTAAGACCTTGCTGTGCTGGAAACACCCTGTGTCTTTCAGGGCTTTTTTCAGAGTCAGGGTTGATG
TGACCGCCACGTTTCCTCTGGGCCTGACAGCTGTAATCTGTGGCTTGGGAGTGCAGATAAGTGTGATGAAGCTGCCAGCA
GGCCTGGGCATCTGTGTCCTTGGTGGGGGCAGGGAGAAGAGACAGGGACTGGGATGCTGGGGCCAGGGCCAGAGCCCCGC
TGAGTATGAGGTCCCCTTCCACCCTGGGAGGTGTGTGGGGAGGAGCTTCCTCGAℵCCCCTTTAGCTGGGACGGATGGAA
GAATGAGCAGCTTTGCATTTCCGGCAGCGTCTCCAGCTTTCCTGGGGCTTTCTCGGCTTGTGTTATTGATGAACTCCAGT
GCGTGAGGCCATTGGAGCCTTAGAGGCCTGTTTCCCCTGGACAGGTGACTGCCTGGGCCAGAGGCAGCACTGGCATGGTG
CCCTCTTGCTCCTGGCTGGGAGTGACACGGGGCGTGGGGCGCTTTGCATTCATCCCCAGCTGTTTGGGCACATTTACAAC
CCTCATTTTACAGATGGGACAAACCCCAGTGGAGAATCTGCTCCGGGCAGCTGTTTATGAACCACCTGAATGGGGCACAC
AGGACTGTGGGAGGAGGAGGAGGGGACCCCCAGGGCTGAAGTGGCAGGGAAAGCTACTCATCCTTTCGGCCCATGTTTACT
GCTCACAAATTGTGGGCCAGGTCTCGTGCCAAGTGTTGGAGCTACAGCCGTGGACAAAAGAGACCTGGTCCTGCTCACGG
CAGCTACGCTTTAGCAGGAGGGATGGGCCATGGAGAAGCACATGGAAGAGGTGGGGTGGCGAGAAGAGCTAGGAAGGGGA
CGAGGCAGGTGAGCTGACATGGAGCCACTGCTGGGAAGGGGACTCCTTCTGATGTGGGAGTCAGGAGGGCCTCTCAGAGG
AGGGGCTCTATGAGCTGGGGAGCAGCTGGCAAAGAGCACAGCAGGTTCAAAGGCCGGAGGTGCGAAGGAGCCTGCTGTGT
TGGAGGAAGAGGGAGCAGGCCAGTGGGGTTGGGAGAAGTGACTGGAGGGGGTGAAAGGGACAGGCATGTGGGGGCCTTG
CAGGACACCTGGAGGAGTTCAGATTTGGTTTTCAATTCCATGGAGGGTTCTGAGCAGGGAAGAGCCATGATCTGACTCAG
TATTTTTTTTTTTTTTTTGAGACAGAGTCTTTCTCTGTCGCCCAGGCTGGAGTGCAGTGGCATGGTATCAGCTCACTG
CAACCTCCGCCTCCCGAGTTCAAGCAATTCTCTTTTTTTTTTTTTTTGAGACGGAGTCTTGCTCTGTTGCCCAGGCGG
GGGAGTGCAGTGGCGCAATCTCCGCTCACTGCAAGTCCGCCTCCCTGGTTCATGCCATTCTCCTGCCTCAGCCTCCCGC
GTAGCTGGGACTACAGGCGCCCGCCACCACGCCCGGCTAATTTTTTTGTATTTTTAGTAGATACGGGGTTTCACCGTGTT
AGCCAGCATGGTCTCAATCTCCTGACCTCGTGATCCGCCCACCTCGGCCTCCCAAAGTGCTGGGATTACAGGCGTGAGCC
ACCGCGCCCAGCCTCAAGCAATTTTCTTGCCTCAGCCTTCTGAGTAGCTGGGATTACACGTGCGTGCCACTACACTCGGC
TAATTTTTGTATTTTTCGTAAAGATGGTGTGTTTCACCATGTTGGGCAGGCTGGTCTTGAACCCCTGACCTCAGGTGATCCA
CCTGCCTTGGCCTCCCAAAGGGCTGGGATTACAGGTGTGACCCACCACGCCCAGCCTGACTCAGTCTTGAAGAGCTCTCT
GGCTGCTGTCTGGAGAAGGGAGTGTCGGGGGCCAGGCTGGAAGCGGGGAGGCAGAGAAGATGGTGATTTGAGCCAGGTGG
GGGTAGTTTTCTAAATGGAAAGAAGGGAACATATTCGAGTTTTTTTTTCAGGGAGAGTCAACAAGATAGGTCAGTGGACTG
GGGTGCGAGGGAAGGAGAGGAATCAAGGGTGGTTCCTCAAGGTTTGAGGAGTTTGGTAGATGGTGGAGCCATTTTCCGAG
ACGAGAAGATCGGGGGAGGGAGGTTGAGAGCAGATGGAGTGTGGGCCTTAACTCTGGTCCTAAAATCAAGCGAGGACAAC
TCAACAACAAGAGTCAGACAACCCCATTAAAACACGGACCAAGCACCTGCAAAGAAGACATGCAGGCAGCCAGGCGCGGT
GGCTCACGCCTGTAATCCCAGCACTTTGGGAGGCCAAGGTGGGCGGATCACAAGGTCAGGAGTTCGAGGCCAGCCTGGCC
AAGATGGCGAAACCCCGTCTCTACTAAAAATACAAAAATTAGCCGGGTGTGGTGGCAGGCGCCTGTATGTAATCCCAGCT
ACTCAGGAGGCTGAGGCAGGAGAATCGTTTGAACCTGGGAGGTAGAGGTTGCAGTGAGCCAAGACTGTACTCCAGCCTGG
GCAACAGAGCAAGACTCCTGTCTTAAAAAAAAAAAAAAAATGCAGATAGCCAACGGGGACATGGAAGTGTGCTCAGCGACAC
TCGCTCATCGTTAGGGAAATGAGAATCAGTGAGACACACTCATACCCATTAGGATGGTCACTTGAGAAAACAGAAAATCT
CAAATATTGGTGAGGATGCGGAGAAATCGGGGCCCATGTATGCCGTTGGGGTGAGGATGCGGAGAAATCGGGGCCCATGT
GTGCCGTTGGGGTGAGTGTGCGGAGAAATCGGGGCCCATGTGTGCCGTTGGGGTGAGGATGCGGAGAAATCGGGGCCCAT
GTGTGCCGTTGGGGTGAGGATGCGGAGAAATCGGGGCCCATGTGTGCCGTTGGGGTGAGGGTGCGGAGAAATCGGGGCCC
ATGTGTGCCGTTGGGGTGAGGGTGCGGAGAAATCGGGGCCCATGTGTGCCGTTGGGGTGAGGGTGCGGAGAAATCGGGGC
```

```
CCATGTGTGCCGTTGGGGTGAGGGTGCGGAGAAATCGGGGCCCATGTGTGCCGTTGGGGTGAGGGTGCGGAGAAATCGGG
GCCCATGTGTGCTGTTGGGAATGTAGGATGGTGCAGCTGCTGTGTGGAGGCCCCCCCAAAATTTAACATAGAATTACCAT
ATGGTGGGGCAACCCCACTTCTGGGTATCAAAGAAATGGAAGTGGGAACTTGAAGAGGAATCTGGCCACCGCGTTCATTG
CAGCGCTGTTCACGATGGCCCAACGGTGGGAGCAACCCGAGTGTCCGCTGATGGGTGGTGGGTAAGAAGCTGGGGCCCAT
CCACACGTTGGAATAGGATTCAGCCTGGAAAAGGAAGGACGTTTGGACGCACGCTGCGACATGCACGGGCCTTGAGGAAG
TTATGCGAGTGAAATAAGCCACAACAGGACAAATACCGTACGATTCCATTTGCATGAGCTCCCTAGAGTAGCCAGATCCA
CAGAGACAGAAAAGAGAATGGTGGGTGCCAGGGGCTGGGGGAGAGGGGGTGCAGAGCTCTTTTTCAGTGGGTACAGGGTT
TCACTTTTGCAAGATGAAAGAGTTCTTTAGATGGATGTTGCTGATAATTGCACAACATTGCAAATGTCCTTAATGCCACT
GAACTGTACACTTAAAAATGGCTAAGTTGGGCTGGGCGTGTAGTGGCTCATGCCTGTAATCCCAGTACTTTTGGAGCCCA
AGGTGGGTGGATCACGAGGTCAGGAGTTCAAGACCAGCCTGGCCAAGATGGTGAAACCCCGTCTCTACTAAAAATACAAA
AATTAGCCTGGCATGGTGGCAGGCGCCTGTAATCCCAGCTACTTAGGAGGCTGAGGCAGAGAATTTCTTGAACCTGGGAG
GCAGAGGTTGCAGTGAGCTGAGATCACACCACTGCACTCCAGCCTGGGTGACAGACCGAGACTCCATCTCAAAAAAAAAA
AAAAAAAAAAGGCTAAGTTGGTAAATTTTATGTTTACCCCAATTAAAATTAAAAACTGTGGCAGGGTTTAACCTGAAGGG
AGAAATGGTACTGGCCCCTCCTGCTTGAATGGGTTGGGCTGTTGGTTTGTAGATGGTAGACCAGACGGCCCAGCCTGTTG
GCCAGGGTCAAATTCCAACATGCTGAGCCTGTTGCAGAAGACTTAGCCTTTGAAGCAGGCAGCCCTGAGCTTCTGATTCC
TCCAGGCCCAAACCACCGTTGCCCTATTTTACAAGACCTTGGCTGGCTTCATGACGTGTTCTTGAACTGGAGCCAGTGGT
GAGCTCAGGGAGGCTCGTGTAACAATAGTTCCCTGAGGCATGTTTATAGCCGGGTGCCCTCTTCACAACAGACACATGTC
CTGGGTCATCTCATTCTCTCTGCACTGCCACGGGGAGGATGGGGAGGCAGTCACGTCTCCATGTGGCATATCAGGGGACC
GGGGTGGGAGGGGCTGACTGAGCCGAGGCCAGAACTAGGTCTGCGCCCTGGCCCTCTCCCTAATGGCCAGATCCGGCCTC
ACCCGTGAAGCTGGCCTGGCAGGGGATGGCCCTTCCTGAATTTCTGTGTGGTCTAGGCTGGGGTTGGCATGCTTTTCTGT
ACAGAGACAGATAGTCAATATGCTCAGCTCTGCAGGCTGCACGGGTTGTGTCTCAGCTACTCAGCCCTGCTGCTGTGATG
CTGAGGCTGCCATTGACGACACAGAAATAAGTCGAGGTGGCCGCTGTGGGCCAGTGCCACTGCATTTATGAACACCAAAA
TGTGAATTTCATATAACTTTCACATGTCATGAAATGGCATTTTTCTTTCAATTGTTTGTTTATTTATTTTTAACATATTT
TTTGGAGACAGGGTCTCACTGTGTCACCCAGGCTGGGGGGCAGGGCACCATCATGGCTCACTGCAGCCTAGACCTCCTG
GGCTCGTTATCCTCCCACCTTACTCTCCTGAGTAGCTGGGACAACAGGCATGTGCCACCACGCCTGGCTCATTTTTTTTT
TTATAGGTTGGTGGGTGGGTGTAGGGGGGGTCCTCACTATGTTGCCCAGGCTCGTCTTGAACTCCTGGCCTCAAAGTG
ACCCTCCCCACCTTGGTCTCCCAAAGTGTTGGGATTACAGGCGTGCAGCACTGCACCCAGCTGGCTGACTTTCTTTCTTT
TCTTTTCTTTTCTTTTCTTTTCTTTTCTTTTCTTTTCTTTTCTTTTCTTTCTTTTTCTTTCCTTTCTTTTCTTTCTTCTC
CCTTCCTTCCTTCCTTCCTTCCTTCCTTTCTTTCTTTCCTTCTTTCTTTCTTTTCTTTCTTCCCTTTCTTTCTTTCTTTC
CCTTCTTTCCATTTGAAAATGCAAATGATTGTTAGCTTGTGGGGTGAGTAGAAATAGGCATCAGGCTGGATTTGGTCCA
CAGGCTGTAGCTTGTTGAAACCTTTCTAAGAACAGAGAAGAATAATGTTTTTGCTTGAAAACAGCCAGCTTTATTGAGGT
TGGGGTGTTTGAAAGAAGGCATATTCACTGTCTGACTTGTGTGGAAACAGTGGGGCTGGGATCAGCCCTGGTAGAAATTG
TTTTTGTCCAAAAAAGCAAGTAGAGGTGGGCTGCCTGGAGTTCCCGTCATGCAGGTGCAACTGGGGAGCAGGATGAGGCC
CTGATTTGGCGGGGAAGGAGAGATTGTGCTTGATTCATCTCTGTGCCCGGGGACCAGGACTGTATGCACCTTCCAGCATT
CTCTTCGCTGGTCTGAGCTCCTTACTTGCCACGCCTGTGCTGGGCCTTTACCAAGGCAGTGCCCAGCCTTTGGTCAGACC
CAAGGTCAAGACTCTGTGCCAACATTGGGTTTGTGCTCTTGGGCAAGCGAGTGGTTTAATCTCCCTGTACTTCACTTTCT
TCATCAGTAAAATGGAGACGAATTATTAGCTTTATTCTATTTTATTTTATTTCATTTTATTTTGAGACAGGGTCTTGCTC
TGTCACCCAGGCTGGAGTACAGTGGCACGATCATGGCTCACTGCAGCCTCTATCTCCTGGGCTCAAGGGATCCTCCTGCC
TCAGCCTTCCACGTACCTGGGTCTACAGGCATACACTACCACATTTGGCAAATTTTTAAACATTTTTTGTAGAGATGGGG
CTCACTGTGTTGCGCAGGCTGGTCTCGAACTCCTGGGCTCCAGTGATCCTCCTGCCCCAGCCTCCCAAAGTGCTGGGATT
ACAGGTGTGAGCCACCACACCCAGCCTAATTGTTAGGTTTAAATAAATGAAAGCATTGCTTTAAATGGGATGCTTTGAGC
TGGAAACAACATAAAACTTGCCTCCAGTGGCTTCTGTAATGCAGATGTTTACTGTTGTGCTTCCTGAGAGATGGGAGGTG
GTGGTTTCCAGATGCGTTCAGCAGTTCAGAACATCGTGCTCTGCCTCAGTAGATTTTGCCCAGTGGCTCTCCGAGGTGGC
TGTACTGATGGACATTCTGGCCAGTGACACATGCGCGAGACCTCTGGTGCTTGCCAGTGCGTGCCAGTGAATACGTGTGA
TGTCCCTGAGCTCTTCCTACTGGGTACCTGAGTCACTTGTGAATTGCGTGTCCTCTTCCCCACTTCCTGCTCAGTCCACG
AGGGCAGGAATCCTGTTGGATTAACTTTACCCCTGTAGCACTGATCGCCCGCTTTGGATGCTCAGGCAGGGTTGAAATGA
ATGAATGACTGAGTGAATGAATGAATGAATGAATGCCACGTTCTCATCGTGTGTTACTATAGGACAGGGATTGCAAACTG
TGCAGGAACATACATGGGGGAAGGGCCCTGGCATGAGGTGATAGGGAGTGGTGGGGCCCGTTGCAAGCTGGAGGGCACAC
CCCAACTGTGAAGCGGCTCCTGCTTTGCTCCAGCGGGCTGGGCCACGTGGCGTGCAGGCCCAGAGCTGGACATCACCCAG
GTTCCCAAAAGAAGCCAGAAGGCAGGAATTTACCTGCTGGTTTAAGATACTGTCAACAGATTCAAATATTTTGAAATATG
TGATTCAAAACCCATCTACTGGCCAAATCTGGCCCATGGGCCCAGTTTGTGGCATCTGTTTCAGGAAGAGACAAGACCTA
GTTTGTGTTTTTTTTAGTGGGGGCGGGGAGGATGCGGTGTATAAGAAATTTTGAGTGAATTAAAAAAAAATTTATGAAAAA
TTTCAAGGGCTGGGTGCAGTGTCTCACGTTCGTAATCCAGCTCTTTGGGAGGTTGAAGCGGGAGAATTGGTTTAGGCCAG
GAGTTTCAGACCAGCCTGGGCAACATAGCGAGACCCCTACAAAAAATAAAATCTACAAAAAATGAAAAATTAGCTGAGT
ATGGTGACAGAAGCTGCTGTGGTCCCAGCTACCAAGGAGGCTGAGGCAGGAAGATGGTTTGAGCCCAGGAGTTCAAGGCT
ACAGTGAGCTCGGACTGCACCAGTGCACTCCAGCCAGGGTGACAGAACAAGACCCTGTCTCTTAAAAAAAAAAAAAAATTC
AAACATACATAAAAATAGACCGGTGCGATGGTCATGGGCCCTGTAACTTGCTGAGGAGATACCAAGTCTTGCCTTCTGCA
CGTGCCTCCATACACTTTCCCCTTTTCCACATTGTTCTGGGGCTCATGTCAGACATTGCCTCATCCGTCAGTGTCTCTAG
TATAGCCACGGCACTTCATGGTGCATTGGAGTCTGGACGCTGGAGGGCTNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNCCAGGCATGATGGCTCACACCTGTAATCCCAGCACTTTGGGAGGCCAA
GGTGGGAGATTTTTGAGCCCAGGAGTTGAAGACCAGCTTGGGCAACATAGTGAGACCCCATCTCTATAAAAAATTTAAAA
ATTAGCCAGGCGTGGTGGTGTGTGCTTGTGGTCCCAGTTATGTGGGAGGCTGAGGAAGGAGGATTGCTTCAGCCCGGGAG
TTCGAGGCTGCAGTAAGCCGTGATCACACCACTGCATTCCAGCCTGGCAACAGAGTGAGACCCTGTCTCAAAAAAAAAAA
AAAAGTTAAGGATCTTGAGATGGGAGATGAACTTGGATTGTCCCAGGGGCCACAATGCTTTCACAAGGATCTTTGACAGA
GGGAGGCAGGAGGGTCAGAGTAGGAGATGTGACCTCAGAAGCAGGGGTTGGAGTGATAAGGGGACAGGAGCCAAGGAATG
CAGAGGCCTCTTAGAAGCTGGGAAACGGAAGGCAATGGATTCCCCCTGAGCTCCAGAAGGAAGCACCCTGCCTGCACCGT
GACTTTCATCCCATCTCAGGGGCAGTCCCAGTTTGGACTTCTCCCCCCGAGAACTGTAAGATGATAACTTTGTATTGATC
TGAGCCAGTGTTAATTGTTAATTCTTACAGCAGCAGTGGGAAGCTAATACAATGAAAATGCTTCCATTAAAGCCAGGTCA
TTAACATGATAGTATAATCTGGTTTGGGTGGGCTTAAATAAAATATTTAAACTTAAAATAATGTTGTGTTAATATGTGAA
CTTTTGAAATTGTCAGTGTTTTTACAAAAAGACTTTAATGTTCTGGAAAAAGTATAAATATGGGGTGCACATTTTTTCCT
TTTGCTTGAAGCTGTAATATGATTCGGTGAGGCACTGATTTGGCCCTCCCTGTATTGCCGCGACAGAGCCTGCTTGTTTT
AAAATAACTTTCTGTTTTAGAACAGTTTTTTTTTTTTTTGAGACCAAGTCTCACTCTGTCGCCCAGGCTGGAGTGCAGTG
GCACAATCTCGGCTCACTAGAACCTCTGCCTTCCAGGTTCAAGTGATTCTCCTTCCTCAGCCTCCCAAGTAGCTGGGACC
ACAGGTGCCTGACACCACACCTGGCTAATTTTTGTACTTTTAGTAGAGTCGGGATTTCACTATGTTGGCCAGGCTAGTCT
CCAACTCCTGACCTCAAGTGATCCACCTGCCTTGGCCTCCCAAAGTGCTAGGATTCCACAGGTGTGAGCCACCGCGCCCG
GCCTAGAACAGTTCGACTTTCAGAAAAATTGGGAAGATAGTGCTGAGTTCCCACATATCCCACAGCCAGTGTCCCCTGCT
ATTAACATCTTAAAGGAATGGGATACATTTGTTGCTATGAATGACCCAGTGTTGATACATCAGTAATAACTAACATCCAG
ACAGGATTCACGTTTCCTTCGTTTCTCCTTCGTATCATTTTCTGTCCCAGGACCCAATCCAGGGCCCCACATTGCGTTTG
TCCCTCACGTTGCCTTAGGATCCTCTGGTCTCTGTGGGTTCTCAGACTTTCCTGGCTTTGGATGACCTGGATTGCGTTCC
TGAGCACCGGCTAGGAATTTTGCAGGACGCCCCTCTGCTGGGATTTGTCTGTTGTTCTTCTATTGATTAGGCCCTAGTTC
CGGGTTTTGGAAGGAAGATCGCAGTGACGAAGGGCTGTTCCCAGCACATTATGTCAACATGACCTGTCCCTGCTGCATGG
GTCCTATTCTTTATTCCTTGTTACAGGTAAAGAAACAGGCACAGGAAGTTGTATCAGGCGCCTGCTGGTAGGTGGAGGAT
GGGCAGGGGGTTGAGGGGACAGGGAGTGGACAGAGCCCCTTTGCTGGTAAAGAGGGGACCCCAGACCTCTCACGTCTCTC
CAGGGCCCCTGGTGGTCTCCTGGCTGGCCTCGCTGCTCTGCTTCAGAAATCAGATTTGACGGGGCACTGGGACCCTGATT
GGGCCTTGGTGGCTGTAGGTGCTCTCTTATTAGCGACACCACTTGAGGGTGTATTGAACATCTGAAGAGTACCCACCCCT
GGATCAATAATTTTTTGCTTTAGGCCAGGCGCAGTGGCTCACGTCTGTAATCCCAGCACTTTGGGAGGCCAAGGTGGGCC
GATCACCTGAGGTCAGGAGTTGGAGACCTCAGCCTGGCCGACATGGTAAAACCCCATTTCTGTCAAAAATACAAAAATCA
GTCCAGGCGCGGTGGCTCATGCCTGTAATCCTAGCACTTTGGGAGGCCGAGGCAGGCAGATCACCTGAGGTCAGGAGTTT
GAGACCAGCCTGGCCAATTTGGCAAAACCCCGTCTCTACTTAAAAAAATACAAAAATTAGCCAGGTGTGGTGGCGGGCAT
CTGTAATCCCAGCTACTTGGGGGGCTGAGGCAGGAGAATTGCTTGAACCCGGGAGGCAGAGGTTGCAGTGAGCTGAGATC
ACGCCACTGCACTCCAGCCTGGGTGACAGAGCAAAACTCCGTCTAAAAAAAAAAAAAAAAAATCAGCTGGGCATGGTGGCA
TATGCCTGTAGTCCCAGCTATTCTGGAGGCTGAGGCAGGAGAATTGCTTGAACCTGGGAGGTGGAGGTTGCAGTGAGCTG
AGATCACACCACTGCACTCCAGCCTGGGCGACAGAGTGAGACTCTGTCTGAAAAAGAAAAAATTTTTTTTGCTTTAATT
TTTTTCTTTTTAAAAATTGAAGTATCAAATGCATTAAGAAAAGTGCACAATTTCGGTTAGAGGTTGATGGATTTTCACCT
AGGTATGCACGCTTGTAATGACCCCCTATACCAAGTGTAAACGCCTCCCCCGCACCCCTCCCTGTCAGTATCCACCCCTG
AGGTCCTGCTGCCCCCACCTCTATCCCCATGGATTGGTTTTGCCATTCCCCGGGACGTCACTTATATGGACTCATGCACG
TGGTCCCTGTGTGTGATTTACCCTGTCATTGTGTGTCTCAATTGGTCATTCCCTTTTGTCTCTGGGTAGCATTCCATGGT
ATGAGTGTGTCCCCATTTGTCATGCATTCACTTGCTTGTAGACATTTGTGTTGTTTCCAGTTTTGGGCTGTTATGGGTAC
GGCTGCTATGAGCATTTGTGGATAATCTTTGTGTGGGTGCATGCTTTCATTTTTCTGGGACAAATACTGGGGGTGGGATT
GCTATATTGTAGGGTGAACGTCCAGCTTTGGTAGAAAAAGTATATTCCACAGTGACTGCACTAATGCACTTCCACCAGCA
ATGTGTGAGCACTCTGGCTGCTCCACATCCTCACCAGCACTTGTTATTGTCAGTCTTTTTCATTGTAGCCATTCTGGTGT
ATGGCATGGGATCTCACTGTGAGTCCAGTGGACATTTCTCTGATGACTAAGCCACCCAGGGCTGGTGGGTGGCTCCAGGA
AGTCACAGGAATCCTTTCTCCTATCTCCTATTCTGCTCCGCCCTTCTTCATGTGTGGCTTTCATTCTTAAGGGTGCCTCA
```

TGATCCAAGATGGCTGCTCTAGCTCCATCCATTGTGACTGAATTCCGGGCAGCAGGAAGGGGGACTTATCAACAACTACA
TAGAAATTTCAGATATTGATAATGTCAGGGAGATAAAACAGGGTGATGAATATAATGTGATGGGGGAAGGGCTGCTTTAG
GAAGAATGGTATTTTCATCCATTTTGTTCACTATTGAAGTGCCCAGGCCTAGAGCAGAGCCTGCCATACAGTGGGGCTCA
ATAGAGATGGTTAAGGGATTGAAAATTGCTCAGGGAAGGTCTCACGGAGAAAGTGACTTTAGACTTGAGAAGTGAGGGGC
TCACTTTTGTGGGCATCAGGGGAAAGATTGCCCCTGGAGGAGCAAAGAGTCAGTGCAAAGGCCCTGTGGCAGAAACTTGC
CTGGTGGCTCAGGAGCACAGCAAGGCCAGTGTGGCTGGAGCAGAGTGAGCGAGGCAGATAGGAATGGGGGAGGAGACTGG
AGTGACTATAGGGAGCTGGGTTGTGCAAGACCTCATAGGTCACTGTCAGGAATTTAGATTCTATTCTGAGATAAATAGGG
AGGCATGGGGGAGGTTTGAGCAGAATGACATGCTCTGACTTTAATCTTAAAAGGATAACTTGGATTCTGTGGGGAGAACG
GGGCGGGGGGCAAGAGCAGAGGCAGGAAAACAGCAGGGAAACTACGGCACCCATCCATACAAGCCAGGATGGGGGCCAGG
ATGGTGTTGGAGAGAAGTGGATGGATTTGAGGCATGCTTTAAAGCGAGAGCAGCCCAGCCTTGCTGATGGAGGGTGCTAG
GTGGGAGGAAAATGGAGGACTCAGGAATGACTCAACCTCTTCGCTTAAGCAACTTACAAGCGTGGGTTTCTTGTAAAAAC
CTTTTATTTTAGTCTTCTTACCTTAGTCCTTTAAAAATCAAAAGAAGTTGGAACATGAGCCAAGGGTGTGGTGTTCCCAA
GTCCCAGATTGGATTAGCCTGTGTCGTAGTTGTTCCTGCATCTTAAGGCCCCATGGAGCTGGGGATGCCTGGAGCCCTGG
GATTGAGAGTGTAGTCAGCCACTGACTGCTGGTCCAGAGTGGGCAGGGCCTGGCCTTGTCCCAGAACTAGGCTTCGTCTG
AGCAAATTGGCTTCTTGGCTCATTTGGTCAGAGGTGTGTTTCTTGGAAGCCTGTTTTCTCTTCTCATAGGAGGAGGAATT
AGACCTTGCAATTCAGTGGCCAGGTCAAGTTAATGATTCATTTGTGAAGCTCCCGTGCAAGGAGCCCAGCGGGGATGGCC
TAGGAGAGAAGCGTGAGGGCACAGGCCTAGACGTGGATACGGAGCCTGGGGCTCTGTCTCTCCCTGCTCCTGTGACCTTG
GTCAGGCATTTGGCTTTGGAGCCTCAGTGTCTGCTTCGATGAGATGAGCCCATTGAGAATTTAAGAGGTGAAGTAGGTGGA
AGTGTTTCGCTACCTGAACAGGGCCGGGCAGATTGTCCAGACGATTCCGCTCCCCTGGTGTCTGAGGGCAGGGGCGTCTC
AGGGCAGGGGCCCCATCGTGTTCATCCCTGTGTCTTTAAATTCCTAGCATAGCATCTAGCATGGGGCGGATGATTGTACA
GGGCATGAACTGCAATCCCAATGAGGTCAGAGCAGCCCTCACTGTAGGCCGAGGTCGTTCCAGTGGGCTTCATGGAGGAGG
TGGCACCTGGGCGAGCGGGGTGTTAGGTTTGGAGGTCAGGGGCCTCTCCAGGTGCAGGGGAGAGTGCTGTGGGTGGTAGG
AAGAGAATGCTAAAAATTAATGTCTATGAAATCTTCATCTCATCCATCACACATTTACCGAGTTGTTGCCCCGGCCTAAA
CAGGCGGTACTCTCTGTGTATTTAAAAAAAAAAAAATCCCTTCTTAAGTATGAGCTGTTTAAAACACACAAAGGAGGACT
AGCGTTACCACCCGCTAGACATTTCCACGTTGATTATATGGTTTCAGGTCTCACTTTTGCTCATTCACCCAGAAAATATT
TACAGAGCTCCTGCAAGGTGCCAAGAGCTGTCCCAGGCCCTGAGGACAGACACATCGCCAAACAAAGCAGCTGAGGATCT
TGCCCTTGTGGGTTTATGGCCAGGCAGGTGATACAGATGGACAGCAATAAACCTGATAAGCTAATTATACCGTGTGCTCG
AAGGCCACAAGCAGCATGGAAACAACACGGCAGGTCGGGAGGTCAGGAGTTTTAGGGGCAGGTTGGATGAGCCTTCTGAG
AAGGGGGCATGTAAGCAGAGATTCGAAGGGGTGAGGGATTTGACCACGTGTTTAGGAGGAAGCAGTGGGTGCAAAGGCCC
TGGGGCAGGAGCGTGGCCTCTTAGACAGGATAGCAAGGAGGCTGTGGGGCTGGGCTGAGTAACAGACAGTGGTTGTGGAA
TGAGACCACCGCTTCTGCTGTTGTCCTTCCCAGCTTCTCCCCAGCCTCCCCTTTTCCCTAGTTTATAAGACAGCAGAAAA
GGGAGAAAGCAAAAAGATGGAAAGAAACAGAAGTAAGATAAATAGCTAGACGACCTTGGCGCCACCACCTGGCCCTGGTG
GTTAAAATAATAATAATAATAATAATATTAACCCCTGACCAAAACTACTGGTGTTATCTGTAAATTCCAGACGTTGTATG
AGAAAGCACTGTAAAACTTTTTGTTCTGTTAGCTGATGTATGTAGCCCCCAGTCACGTTCCTCACACTTACTCGATCTAT
TATGACCCTTTCACATGGACCCCTTAGAGTTGTAAGCCCTTAAAAGGGCTAGGAATTTCTTTTTCGGGGAGCTCGGCTCT
TAAGACGCGAGTCTGCCGATGCTCCCGGCCGAATAAAAACCTCTTCCTTCTTTAATCCGGTGTCTGAGGAGTTTTGTCTG
TGACTTGTCCTGCTACAGTTGGATGTGGGGCCAGAGAGAACACAGGGGCCAGACCAGGCAGAGGGGCTTGTAGGCCAATG
TTCGGACGGTGGCTCGTATGTTATTTTCAAGAAATAACATGTTCACAATGACAGTTAAAGCTTTCCCCACATCCCCAGCC
CCCAGCCCTCTCCTGAGATTGTTTGCAGGTATCATTTCCCTGAATGTTTTTCTACATTTCCACATGGGTTTGTCTTCAGA
AAGAATATAGAGTATTATTTCATGTGTTTGGAAAATTCACATCAAGAGGCCCATGTCAATTGTTTCTTTCTTTAGCCTTT
TCTTCACTCAAGATTTTTTTTTCTTTAAAAATAAAAATTGAGGCCAGGTGCAGTGGCTGACACCTATAATCCCAGCACTT
TGGGAGGCTGAAGCGAGTGGATCTCTTGAGCTCAGGAGTTCAAGACCAGCCTGGGCAACATAGTGAGACCTTGTCTCTAC
TAAAAGCAAAAAAATAAAAAAATAAAAAAATAGCCGGGCGTGGTGGTATGTGCCTTGTAGTCCCAGCTACTCAGGAGGCT
GAGGTGAGAGGATTGCTTGATCTGGGGAGATCGGGAAGTCAAGGCTTTCCAGCCTGGGCAACTGAGCGAGACTCTGTTTC
AAAATAAATAAATAATAAAAATTTATGTATATTTAAGGCATCCAAATGCTGTTTTGCTATATACACACACATAGTGAAATG
ATTACTACAGTTAAGCAGATTAACATATGTATCTCCTCATATAGTCACCTCTTTTTTTTTTTTTTTTTTGAGACAGGGT
CTCGCTCTGTCACCCAGGCTAGAGTGCAGTGGGGTGATCACGGTTCACTGCAGCCTTGAACTCTCAGGCTCAAGTGATCC
TCCCACTTAAGCCTCCTGAGTAGCTGGGACCATAGGTGCGCGCCACTGTGCCTGGCTAATTTTTGTATTTTTTATAGAGA
TGGGCTCCCTATGTTGCCCAGGTTGTTCTCAAACTCCTGACCTCAAGCGATCCTCCCACCTCGGCCTCCCAAAGTGCTGG
GATTACAGGCATGAGTCCCGGCCACCTCTTTGTTTTTAGTGGTGAGAGCACCTGAAATCTGCTTCCAGTATACAGTACAG
TATTAATCACAGTCCTCGTGTTGTGCATGCAACCTCCAGACCACCCATCCCACATGACTGCAGCTTTGCGCCCTGTGACC
TGCATCTCCCCACCTCCCCAGCCTCCGCCCCGTAAGCACCATTCAACTCTCTGTGTATTCAATGTCTTCAGATTCCTCAC
CTCTAAAAGACAAATCTGGAAGTATTTGTCCTGCAGTATTTGTCCTTCTGTCAAGATTCTGCCTTTGAGATATCTGCAAG
TCGATGGCGTTTACTCACTGTGCAGCCACCGTGTGCAGAAGCCCAGCTTATCTTTGACTGAGAACCTGGCTGGGCTCTCT
GTTGTTTTTCAAGGCAGGGTCTCGCTCTGTCACCCAGGTTGGAGTGCAATGGTGTGATCTCGGCTCACTGCAACCTCCGC
CTCCTGGGCTCAAGCGATTCTCCCACCTCAGCCTCCCGAGTAGCTGGGACTACAGGTGCATGTCACCGTGCTTGGGTAAT
TTTTGTAGTTTTAGTAGAGACGGGGTTGCACCATGTTGGCCCGGCTGGTCTGGAACTCCTGACCTCAAGTGATCCACCCG
CCTCGGCCTCCCAAAGTGTTGGGATCACAGGCGTGAGCCCCCGTGCCTGGCCCTCTGTTCTTTTTTTTGAGGTCTGACAAT
TCTTCTGGCGCTTTCTTGCACACTTAGGAGAATCTTTCTAGGGCAGAGTTTTCAAACTGTCAGATGTGATCAGATTGTGG
GTTGTGAAATAAGTGGGGTGGGTGGGGATCAGCTTTTTTTTTTTTTTTTTTTTTTGAGATGGAGTCTCGCTCTGTTGCCC
AGGCTGGAGTGCAGTGGCGCAATTTTGGCTCGCTGCAACCTCCGCCTCCTGGATTCAAGCGATTCTCCTGCCTCAGCCAC

```
CCGAGTAGCTGGGACTACAGGCATCTGCCACCACGCCTGGCTAATTTTTTTGTATTGTTAGTAGAGACAGGGTTTCACTG
TGTTGGCCAGGATGATCTCGATCTCCTGACCTCATGATTTGCCCGCCCCAGCCTCCCAAAGTGCTGGGATTACAGGCATG
AGCCACTGCACCTGGCCTGGGATCAGCATTTTAAAAACAGAATGGAATAGGATAGAATACCAGAGGGGCGAATAATGGAG
TCATAACGTAAGTTGCATCATGCTGGGAAGGTGCGTATTCCCTCATGAGGCTTCTGGTGGGTGTGCAGGTCGTGGCCCTA
GCCTGGCAGGAGCTGGGAAGCTGGTGCTCTGCACCATGTCTGAACAGGGAACCCACAGACCATGGGGCTTTGCTCACACC
TGACCCGATTAGAAACTGTCCCGACTCCCCAGTGGCCTGACCCCCGCTGTGCACAGCGGTCCTGTTTCCAAACATCTTTG
CCTGTAGTTGTTTTCTTGGATGTCCTAATTTTTGCTCATCTACTGGGAAATGATATCTCATTTTAATTTGGAGTTAATCT
TTCTTTTTGAAATTGTGATTAAGATCCAGTTAACGTAAAATTCACCAAAACCATTTACAAATGTACAGTTTAGTGGCATT
TAGTGCGTTCGCAAGGTTGCGCAATCGTTACCACGATCTAGTTCCAAAGCATTCTCATTACCGTGTACCCATTAAGCAGT
CACCCACCATTCCTTTCTGCCCCAGCCCCTGGCAACCACCAATCTGCTGTCTCTGGATTTGCCTATTCTGGGCATTTCGT
AAGTGTGGTCAGACACTAAGTGTGGCCTTTTGACTGGCTTCTTTCACTCCATGTCAGGTTTTTGAGGTTTGTCCATGTGG
TGGCATGTGTCAGTGTTGCATTCCTTTCTTTTTCTTTCTTTCTTTCTCTTTTTTTTTTTTTTTGAGATGAAATCTCGGTC
TGTTACCCAGGCTGGAGTGCAGTGGCGCGATCTCGGCTCTCACTGCAACCTCCGCCTCCCGGGTTCAAGCGATTCTCCTG
CCTCAGCCTCCCGAGTAGCTGGGAATACAGTTGGCTGCCACCATGCCCAGCTAATTTTTGCATTTTTTTTTTTTTTAATA
GAGGCGGGGTTTCACCATGTTGGCCAGGCTGGTCTTGAACTCCTGACCTCAGGTGATCCACTTGCCTCAGCCTCCCAAAG
TGCTGGGATTACAGATGTGAGCCACCACACCTGGCCGCATTCCTTTCTTTAAAATTATTTTTTTGGCTCTTTAAATACA
TTTCTTTTTTACGCAGTAGTAAACAGCCAACCCATGCATTCCTTTTTCTAAGTGAGTCAGATGCCGCTGTCTGGACAGAC
CACACTGCGTTCAGCCATTCATCTGTTGACGGACTCTTGGGTCGTTTTCGCCCATTGCGAACAGTGCTGCTGTGCACATT
CTTGTACGTGTTGTTTTTTACACCCATTTTCAGTTCTTCTTGGGTCGTTCTGGGGAGTGGAGTTGCAGGGTCATACGGT
AACTTTGCGTTTGATCGTTTGAGGAACGGTCAGACTGGTTTCCACAGTGGCTGTTCCACGTTACACTTCTGCCGGCAATG
CATAAAGGTTCCAGTTCCCACACCCTTGCCAACACTTGCTATTTTCCATGAAAAAAAATTCTAGCCATCCTGGTGGGTGA
GAAGTGGTGGCCCATTGTGACTTTGATTTGCATTTCCCTGATGATGTCAAGCACCTTTTCATGAGCTTGTTGGCTATTTG
TGTATTTTCTTTGGAGAAAGGTCTTTTCAAGTCCTTTGCCCATTTGCAGTTAATCTTTTATTTTTAAAATTTCACATTAT
AGTTTCAAAAGGGTTTTAGTGAAGTGTAGCACACGTATAGAAAAGCCCACATATAAAGGGAATAACACCATACATTTTTC
AGTGAACATAGTCCCGGGTAACCAGCAACCAGATCAGGAAACAGAATTTTCACCCGCTTATCCCCCAACCTCCCAGGTGA
CCCCTGTTCTGGTTTCTATTGATATGTTAGTCTTGCCTGTTTTAGAACGTCACATAAATGGGATTATATGTGTATGCACA
TATCTGCCTTTTGATCAACATGATTGCATTATATTTTAAAGGTAGTAATAGTATTCATTTTTAAATTTTTTTTTACTTAA
AATATTCTGGAGATTGTTCCATAGCGGTAAATATAGCATGTCATGGGTGGTGGTGTTGCTGTTGTTTTTTTCAGACAGGG
TCTTGCTTTGTGGCCCAGACTGGAGTGCAGGCACATGATCACGGCTCACTGCAGCCTTGAACTCCTGGGCTCAAGTGATC
CTCCCAGCTCAGTCTCCTGAGTAGCTGGGACCACAGGTGTGCGCCACCATGCCCGGCTAATATTTTTGTATTTTTAGTA
GAGACACGGTTTCACCATGTTGCCCAGGCTGGTCTTGAACTCCTGGCTTCAAGAAATTCTCCTGGCTTGGCCTCCCAAAG
TCCTTGGATTACAGGCGTGAGCCACCATGCCTGGCCTAATTTGATGAGTTGCATCAGATGTATACACTTCACCTATATGG
CAACCAAAATCAAGATAGCTGGGCCAGTTCACGTTAGTCCCCTCCCTCCATCCCGGTCCCCGGCAGCCACTGGTCTGGCT
TCTCTCTCTAGAGTATTCCCTTTCCCCGATGTCGCGGAAATGGTGTTGTGTAGTATGATTCTTCTTTTCTTTTTTTTGCT
TGGGGTAGTGCTTCTGAGGCTCACCTGTGTTGTGGTGTTCATCCATGGTTGGTTCTTTTTGATTGCCAGGTAGCATTCCA
TTGTGTGGATATGTGGCAATTCATTTATCTGTTGATGGATATTTGAGTTGTTTCCAACTATCAAATATGGATATATCATT
CCATATTTGTTTTTTGGTGAACATTCAGGCAAGTCACGCTCTTAAAGGCTTCCCAGCCTTTGCACAGGCTGTGTCCTCTGC
CAGGCACTCCTCCACCATCTTCTTTTCCTAGCAAAGCCCAGCTTATTTTAAAATTCCCAGCTCAGATGTCCCCTCCTCCA
GGCAGCCTTCCTGGGCTCAAAGGGTCTATTTCGCCAGATGGGAGGGAGTGGCGTCCAACAAGGGCTCACCTTGGCATTCT
ATCCCAACTCTGTGTTCCAGGGCCATGGTCTTTGACCTTGGGGGTGGTGGGGACAGAATCACAGAATCAGCCATGTTAGG
CAAAAGGCCACAGTGTCCCCACCACTGTTATCTAATCAGCTGCCTGGTCTAGTGTGGTGGAAAAAAGTCATCCTGCTGAT
CAAAAGCAGGGCAAAGTCCATCCCTTAACCCGGGTGGAGGTGGGGCAGAGGAGGAAGGGTCACTTAATGACTGCAGGGCT
GACCCTCTGGTGCCCGGGGTCTTCAGACCTTGTTCTAAGCACTTTACCTCCATGGACTCATTCAAACCTCACAGCAGCCC
ATGAGGCGACACTCTCCTTACGCCCATTGTATGGTTGAGGAAACTGAGACTCGGAGAAGGTCAGTGACCCATCCCTGATC
ACCCAGTTGGACAGGGCTGCGCTGGGTCTCTGAAGATTCCCTTTGGGCAGTGCTGAATGAATGGGCTGAAACCCAGGGCC
TGAAAGCCTCTTCTTTGGGGAGAGAGAGAGAGAGAGAGAGATTGAGACATGGACACGCGGCCGGATCCCATGACTTCTTG
GTTTAGCCCTGATTCATCTTCAGAACCAGGCCAAGCGGCCAGGGGAGGAGGAAAGCATGGTGAGATTCCAAAGGAGAAAG
GTATCTCCAGCCCAGCAGCCTCTGCTGAGAAACCAGGTGGGAGAGGGAGGCTGCAAAGGGAAATTGAGGCTGCGTGTGTG
TGTGTGCGCGTGTGTGTGCGTGTGTGTGTGTGTGTGTGTGTGGAGATGGAGTCTCGTTCTGTTGCCCAGGCTGGAGTGCA
GTGGTGTGATCACAGATTGCTGCAACCTCTGCTTCCTGGGCTCAAGTCATCCTCCCACCTCAGCCTCCCAAGCGGCTGGG
ACTATAGGCACCTGCCACCATGGATGGCTGATTTTTCTATTTTTTGTAGAGATGGAGTCTCCATGCTCAGGCTGGTCTTG
AACTCCTGTGCTCAAGTGATCTGCCTGCCTCGGCCCCTCAAAGTGCTGGGATTACAGGTGTGAGCCACCGCACTGGGCCT
TGTTTTGCTTTTTTAAAAAAGCAGCTTTATTAAGGTGTGATTGATATAAAACAATGGCACATATTTAATATATGCAGTTT
GATGGGCTGGTGTGTGCACACCGGGAAACCCGCCCCTGAGATCCAGGTGCTGAACATACCTGTCACCTCTACGGTTCCCT
CCTGCCCCCCACTGTTTTCTGTGGTGAGAACATATAGTTCGTGCTCTACTCCCCGAACACATTTTCCAGGGTTCTGGGTT
TTGATGCTGTGTGTTTCTATGGGGGGGAGCACCAGCAGCTGGATGTGTCCCCCAGGCTGGTGAAGGGGTCTGGCCTGTGGTG
AGCAGGGTCCCCAGGGCTGCAGGGTCTGTCCCAGACAGAGGGACCTGCAGTGCATGGGCTTTGAGGCTGGGTGAAGGCCA
GGACTGAAGGAAGGGCTGGGCGATCGGGGCCTGAGAGCTGGGAGGGGCTGGAGCTGGTGCCTGTGGGCTGTGGTCGCATG
GGGTGGTGGGAGCCGCCATGGGGTGGGGAGGGCGTTACCGAGGGGGCAGGACTGCTCTTTGATCCAGCTCACATACCACC
TCCCCCAGGAAGACCTCCCGGACTCCTCAGTGCCCTCCAACTATTGGTTGAATCTCTCTCCAGCCTTAGTTGTGCCTCAG
TTTCCTAATCTGTAACATGGGGGGTAATAATAGTGGAGGTGGCTGCCTCAAAGAGTAATGGTGCTGTTCAGAGGATGAGAA
```

```
CACAGTGAACAGCATCACCAGGCTCATGGCGCACCCCGGAAATGCTCGCTTCTGTTACCTCATTAATATTCAGTTTATGC
GCCTTCGTTTGCACCTATGGGCTGCTCAGACCATAAGGTGATCCTTCTGGTGCTGGCAGCTGGGTAGGAAGACACGTTTA
TCAATAAACAATTATGGCACTTGAAGGCCAGTCTCTGATCTCCTGATTTTTCCAAGAGAACTCTGAAATCCAGATTTTAC
ATTACATCTTCCAGTTTCTTGACAAAGAATTCAGCTTCTTCATTGACACGCCACGTAGGTCAAACTAGACACAGTTGTAG
CTACAGTGTCACTAGCTCCTTCGTACAGATGAGAAACTTGAGGTTCACAGGGGCTGACTTGCCCAAGTGGCCCCGTGGAG
TGCGACCCAGGGTCTGGCTGCCTCCAAATCTGCTCCTTCCGGGGGACATGGGGCTGGCCCTGACTGGCCAGCTCAGCTGT
CTGCCTCCTGTAGAGCCTCTGCCTGTTGGTTCTGGCTGTCCTTGTCTTAGAAGGCCATGTTCAAAAGAGGGTGGAAGGTG
ACGCAGCTGCCTGGCTTTGCGACATCAGCTCTAGTCTAATGGGCTGGGTCACTCCTGCCGGGCTCTGACGTGGTGTGGGC
ACTGAGGGGCCCGAGATATGTCCCGGCTGGGTTGAGGGTGGGGGTCAGGTGGACTAGGGGGGACCTCCCTGTGACACTGG
GGCAGCGAATTATAGATCTAGAGGAGGAAAGCTGGCAGAGGAGTCCAAGTGGACCCCAGGCGTGTTTCCTATAGTCCCTT
TAAGAAAACCATGTTTTTGCAACATTTTTAAATCAGGAGATTTCATATACATTTCTTTATTTCTGGTTTTCATGGAAAAC
TTCTGTGTTCTCATCTTCCGACTGGCTCACTCAGCCACACAGGGAGGCTCTCTCCCAGCGTCTCCCTACCACAGGGCCTT
TGCACAGGCTGTGGTTGCTGTGGAATGTCCCCTGCGCCTCACCTTCTAGATTTGTGAGCTCCCTTCCTCACTGCCTTCTG
TTTTGATTTTGGGTCTAGGTTGTGTTTGTGTGTGTTCTTTTTCTTTCTTTCTTTTCTTTTTTTTTTTTTATGAGACAGGG
TGTTGCTTGGACACCCAGGCTGGAGTACAAATGGCGCCATTACAGCTCACTGCAGCCTCGACCTCCTTGGCTCAAGTGAC
CCTCCCATCTCAGCCTCCCAAGTAGCTGGGACTACAGGTGTGCAGCACCATGCCCAGCTACTTTTTAAAAAATTATTTTT
TATAGGCCAGGCACAGTGGCTCATGCCTGTAATCCTGGCACTTTGGGAGGCCAAGGCAGACGGATCACTTGAGGTCAGGA
GACCAGCCTGGCCAACGTGGTGAAACCCCACTTCTACTAAAACAAAAACAAAAACTAAAACAAAAATTAACCAGGTATGG
TGGTGGGTGCCTGTAGTCCCAGCTACTCGGGAGGCTGAGGGAGGAGAACTGCTTGAACCCGGGATATGGGGTTTCGGTGA
GCCGAGATCGCGCCACTGCAGTCCAACCTGGGCCACAGAGCAAGACTCCCTCTCAAAAAAATAAAATAAAATAAAAATAC
AAAAATTAGCCAGGCGTGATGGTGGGGGCCTGTAGTCCCAGCTACTCAGGAGGCTAAGGCAGGAGAATCGCTTGAACACA
GAAGCAGAGGTTGCAGTTAGCCAAGATCATGCCACTGCACTCCAGCCTGGGCAACAGAGTGAGACTCCATCTCAAAAAAT
AAATAAATAAATAAAATTATTCTTTATAGAGACAGGGTCTTGCTATGTTGCCCAGGCTGGTCTCAAACTCTTGGGCTCTG
GTGATCCTCCTACCTCGGCCTCCCAAAGTTCTGGGATTACAGGCATGAGCCATGGTGCCTGGCCTATATTTCACTTACGT
GATGACTGTCTCAAATCCCAGTCTCTCCCCAATCCCTGCTGTGTTTTCTCTGTGCTCTGTCACGTGGCATGCGTTTTACC
TGAGTTGTTTGGTGCCTGCCTTTCCTCCGCTATGGTAAAGCCCCACATAGCTGGGCTTTTGGTCTGTCCTGGTCATTCTC
CCATCCCCAGCTCTTAGAACCCGCCTGGCAGGTGGTAGTGCTCAGTGCTTGCTTGTGGAATGAATGAATGATCCCCCTGG
CCACACGGAGCCCATGTTCCTGCATGAGGCTATCCCTGCCCTGAGCCCATCCGCTACCCTGAGCTCCGGCGCTCCCTTTA
GGTGGTGCATGGGCCCCTGTTTACCACAGTCCCTTGACTGGTGTCACCTGTTGCTGTTAGGACATATTTTAAGATGCATT
ATGACAGCATGGATAGGTTGCCTGTTTAATTCCATAAGCTCCAGGTGCGACAGCCTCATGTGCGAAGGGCTTTCCCACCA
CCTCCTATCCCAAGCTCCCGCCGAGGAGCCCCTTCCCTGGCCGGGCTCGGGCAGCTGTTCCGGAGCCTTGTGGTGGGGCG
TGGGGCCCTCATCACTCTCCTCACAAGCGTACTTGTCCCTTCCCCTGCAGAACGTGCGCATCGACCCCAGTAGCCTGTCC
TTCAACATGTGGAAGGAGATCCCTATCCCCTTCTATCTCTCCGTCTACTTCTTTGACGTCATGAACCCCAGCGAGATCCT
GAAGGGCGAGAAGCCGCAGGTGCGGGAGCGCGGGCCCTACGTGTACAGGTGAGGCTGTGTCCAGGTGAGGGTGGAGGGGC
CGGCTGAGGCTGGGCAGGGGAGGGGTCTCAGAGTGGACGGGATGGGGAGGCTGCTGACTGAGCCCCAGAGATTGTTCCGG
AAGCAGGCAAGTCATAGTCGGGGTAAGTGCTAGTCCCAGAGAAGTTTTTGTTTTAGGGTTTTTTTTTTTTTTTTTTTTTT
TTTTAGAGATGGGATCTTGCTATGTTGCCCAGGCTGGTTTTGAAGTCCTGGGCTCAAGCGATCCTCCGCCTCAGCCTCTC
AAAGTGCTGGGATTACAGGTGCGAATCACCAGACGTTGCCTAGAGAGGTTCTTTATGGAGCAGGGAGGGACCAATGGTGT
GCGTCTGGGTGGAGGGTGCATGTGTGAGTTACACACATACACATACACACACATACACATACACATACACACATACATACAT
ACACACGTACATACACATACACACACACACATACATACACACACATGTACCTACACATGCATACACACATACACACACACAC
ACACACACACATGCATGACCAGGAGCAGGGACCGACCCCCCAGACCCTATCTGGGCCAGAGGACCGGGTGGGTCAGCACG
GGAAGGGGTCAGCTGTTTGTGGAACATGCTGGCCCAAGGACCACAGAGTTGTGCCTTTGCTTTCTGCTTGTCCTGTACCT
GGCTGTGGCCTAGGGGAAGTGACTTCATTCCTCTGAGCCTCAGTTTGCCCGTCTGCAGACTGGAGAGACACAAGAGCCCC
TTCATGGGGTCACCGGGACACTCAGGATGCACTCGGAGCTCTGAGACGGCTGGCGGATGTGCCTGTTACAACTCCCTTAC
CTCCTGGCGTTTTCACAGCACCTCCCCTCCTCCACACCCCCACTTCCCAGTTCACAGACAGGGGAGCTGACTTGCCCCCG
GCACACGGTGTTCCAGGGATGGGGCGGGCAGAGGGTGTTCCCGCTGTTGGAGACCCACAGTCTGGTTCTGGGAAAGCCAA
GATGAAAACCCAGCAAATGTGCCTGAGGTTTGGGAATGGGAAACATGAATCAGCTGCTGCATTCCGTTCACTCATTCACT
CATTCGCTCGTTCATTTAACAAATGTTTACTGAGCACCTGCTAGGTGTTGGCTGCCGTTCTAAGTACAGGGGACCCAGCA
GTGGACAGAATGGGCAAAAATGCCAAAGCTCTCTATACTCCTTCATTCTGTGAGCACTGACTGAGCACCTCCTGTGTGCT
GGGGGCGCAGGCAGACCAAGGCCCTGCCTCACCAAGCTGATGTTCTGATGGGGAGAGAGTAAAGAAGTGGACAAATAAGG
GGAAATCAGGCAGCCATCAGTATTGTGCAGACAAAACAGTGTGAAGCCGGAGTGCAGGAGGCAGGGTGGCCTTGGGGCTC
GGTCTGACTGGAGGGTCGGGAGCACATCTGCAAAGGGACACTCACTGGACCCGCATGGTGGGAAGAGGCCCTGGGGAGAC
AGAGTCCCGGGCAGAGCATGTGCAAAGGTCTGAAGGCTGGGACAGAGGCCTGTGCGGCTGGGGTTACGAGGGAGGGAGAC
AGTGGAGAGACGAGGCCTGAGCCTTGCTGAGGGCCTCAGAGGTCACGTTAGGAGCCTGATTTTAACCTGCATGCAAAGTG
GGGTTGTGGTCCAGAGCATGGCACGATCTGATTTCTACTTTTTTTTTTGAGACGGAGTCTCTCTTTCACCCAGGTTGGAG
TGCGGTGGCGCGATCTCGGCTCACTGCAACGTCCGCCCCCCAGGTTCAAGCGATTCTCCTGCCTCAGCCTCCCGAGTAGC
TGGGATTACAGGCATCTGCCACCATGCCCAGCTAATTTTTGTATTTTTAGTAGAGACGTGGTTTCACCATGTTGGGCAGG
ATGGTCTTGAACTCCTGACATCAGGTGATCTGCCCACCTCAGCCTCCCAAAGTGCTGGGTTTACAGGCATGAGCCACTGC
TCCCGACCTGATTTCCACTTTTTCAAAGATTCTTCTGGATGGTGGAGAGTGGCTTGGACAGATGAGAGATCATAAGGACAG
CAGCAGCAACAGTCACAGCAGCTGATGTTTACCTCGTGCTTTCTCTGCACCCGGCGGCTGTGTTGATTGCTTTCCGGGTA
TCTGATTGCTTAATCCCCACAGCTGCCCTGTGAAGTAGGGCTTGTGATTACTTCCTTTTGTAGATGGGAGAGACGATGGCC
```

```
GTGTTGGGTGGGGGAGAGCAGAACGAGGCCGGGTGGGCGGCGACACCATGTCCTGCAGTGGGCAGGCAGCGGGAGGGACA
GACTTGGCGAAGGGGCCGAGCTCAGCTTTGGCTGTGGGGCCGGAGGTGTGCACAGACGTCCAGGGCCCCTGGTTCCCCAGG
CAGGCATTGCAGGCGAGTAGAAGGGAAACGTCCCATGCAGCGGGGCGGGGCGTCTGACCCACTGGCTTCCCCCACAGGGA
GTTCAGGCACAAAAGCAACATCACCTTCAACAACAACGACACCGTGTCCTTCCTCGAGTACCGCACCTTCCAGTTCCAGC
CCTCCAAGTCCCACGGCTCGGAGAGCGACTACATCGTCATGCCCAACATCCTGGTCTTGGTGAGGCTGCCCTGTGGCCCA
CGCCGCCTCGCACCCTGACCTCGTCCCCTGTCTCTCCTCCCGCCTGCCCCTTGTGCAGAGAGCAGTCCCTGAGGTGGTCG
GAGCGTGGGGACTCACGCCTGGTGGGTGGCTTTCGGCCCTGTGCTGTCTCCACCACCCCAGTGGGTTCTGAGTTTCCCA
GGTGGGTCCACCTGTCTTGGTTTGGAAGTCCTGGCCAAAGTACTTTTTTTTCCCTTTTCAATTTACATTTCTGAGATCTC
CAAAAGGGGCTGTCTTGTTGAGGGCTGAGCCACAGGCCCGCCTCTGGGACTGGGGCTGGAGTTCACTTAGCCTGAGTCCA
GTGGGGTGCAAGGGGGAGAAGGGGTTCTCGGGAGCACATGTGGCCTTGGCACTGGAGGAGCAGAGGGTGGTTCTGGTGTC
CCAGATGCCCCACGTGGCCACTCCAGGGGCCTCCTGCACCCCAGCATTTCCCTTCATGGGCTCTTTGCTGTGAGGCCCAG
CTGGGGCCAAGGGAGGATGGGCCAGCCACGTCCAGCCTCTGACACTAGTGTCCCTTCGCCTTGCAGGGTGCGGCGGTGAT
GATGGAGAATAAGCCCATGACCCTGAAGCTCATCATGACCTTGGCATTCACCACCCTCGGCGAACGTGCCTTCATGAACC
GCACTGTGGGTGAGATCATGTGGGGCTACAAGGACCCCCTTGTGAATCTCATCAACAAGTACTTTCCAGGCATGTTCCCC
TTCAAGGACAAGTTCGGATTATTTGCTGAGGTACGTGTGGCCTGGTGAGAAGCCAAAGATTCAGGCCTGTGTCCTGTCTT
CCCCTCACACAGCCTGGACACTGGTCACCAGCTTGCTTTGTAGCTGGCTGGGGATCTAGTGGCTGTGGGTTGTAAGTGAC
TGAGAACCTGACTCAAACCGGCTTGAGTGAAATGGGGAATGTTGGGGCTCATAGAACTGAAAATGCTAGGGTTGGATTCA
GGTACAGCTTGATCCAGGCTCAAATGATGTGACTGGGCCTTAGCTTAGCAAATTGGAGGCTTTGCTGGAGGAAGGGGGCA
TGGCTGCTGGGGAGTAATATCACAAGCTGACTCTTAATCTTGACTCTTGGCAACCTGGTAGGGTCACTGACTGGGCTTCG
GAGCCAACATCTCGTCCATGGAGGGTCTGACCCTGACCTTGGCTCCCTCACCGCAGGTTTCACTGAGTCCTTGGGACTGC
TCCAGCCTCAGCCATCTCTTTGGTCTCTGCTGATCAGGGTAGAGTGCTGGGGGGGATATCGTGGTGCTGCGTGTGATAGA
TACTCAGTACGTTTTTGTCAAGGGTGACGGGCTCCTGTCGTGTGGGTAAATGAGCAAGTTTGGGCTAAGTGGCATGATGC
TGTAAAGGCATCTTGTAAATCCTGAAGTGCTTTGCAAATGAAAGTTATTACAAAGTCCCTAGTTTAGTATAATCTACGTT
TGACTGCATATAATGAAAACCTCACAAGAAAAGTCTAGCGGTAGGCTGTTCAGTGTTGGTGTGGTGGCTTCATGGTCTTA
AGTGATCTGGAAGCCTTCTACTACTGTGCTCTGCCATCCTTGGCATGTGGTTTTCATCCTCAAGATAACCTCATTGTCCA
AGTTGGCTGCTAGAGTGTCAGCAATCACATCACATTCCAGGCAGCACAGCAAGAAGGAAAAAATGGTGGGGCTTTGGGGA
GCAGATGGAGGAACTGGGGGTAAAACAGCGTGTTCCAGTTTCCTCCCTCTTAGGGAGGAAGAAAACCAATGTCCACTTCC
TTTCTATTGGCCAGAACTGAGTCACATGGCCACACCTAGCTCCAGGGGAAGCTGGAAAGGTAGCCTTTTAGCTGGGTGTG
CTGATGCTCTGAGTATGGTTTGGGTCTTGTATGAAAGAGTAAGGAGAGATTGGGCTTTGGGTAGTAACTGGCAGTTTCTG
ATGCTCTCCGTCCATTATAATGATCATTTAAGGATCCTATTGGGTGAGCATTTATTAATAATAACAATAATAATGAAAT
AAAGACTATCATTTTGAGCTCTTACTGTCCGGTGCACTGTACCTGGCCGTTTTTGCACATGAGTCCCTTTAGTGCTCC
CCTTTGTAGTGGCTCTGGTTATTCTTGGCTCCACATGGGCACGTGACAGACACACCCGGGATGCTGGGCTGGGGCCTTGT
GCTGGGTCCTGCTCTGGAGGTCTGTGATGGGTGCAGTCACTCAGCCGGGCAACCTTGGAGCTCCTCAGTGTGCTCCACCC
TGCAAAGCACTTCTAGATATTTCCTTCAGTCACTCATACCACTTTGTGCTTTGCATGTCTTTGGGATTAGAAACCAGCTT
CTGGGAAATGCTCTCGGCAGCACCAAAGCCTGGGCCCCACTATCAAGAACCTCGATGGGTTAGGACAGGCCAGGTTTTGCT
GCTGTGACAAGCAGCCCCGGAATCTCCATGGCTTAGAACAACCAAGACTTATTCCTTTGCTTGCACTCCACGTCCAGTGT
GGGTGACTGGGGCACCCGCGCTTGTGGTGGTCACTTGGGGATCCAGGCTGAGGGAGGCTCTGTCTTGACACATACCTCCT
TGGCTGCTATAGCAGCGGCAGAGAGCCCGGTGAAAGGCGTGTGCACAGTCCTTAAAGCATCCCCCCAAGTGACATGCCAC
TTCTCCTTTTTCATTGCTCAGGGAGCTGACCTGGCCATGCTTTATTTTATGCTGGAGTAGGGTGTGGTGGGGGTGATCTG
CCATTTACCTGGGAGGAAAGCCAGACTCTCCTGGAGTTAGGAAGGGAGGGTGGAACACTGCAGCCACAGAACCCGGGACC
ATTCCTCCTAGAAAGCTCCCAAGCCTCCTCTCGGTCCCCAGACACTGGGCATTTGGCAGTGAACCAGATGCTGGGGGCCC
TGTCCTTCTGGTGGAGGGGGAGGAGGGCTCAGCCCAGAATGTTCAGACCAGGCCGGCTCAATGGCAGGCCTAAGCCTTAC
GATGCTGTTCCCTGCTGTGTCTGTAGCTCAACAACTCCGACTCTGGGCTCTTCACGGTGTTCACGGGGGTCCAGAACATC
AGCAGGATCCACCTCGTGGACAAGTGGAACGGGCTGAGCAAGGTGAGGGGCGAGAGGCGAGGGCCCCTGTCGCCAGGGAG
AGGGGAGGGTGGGCCCGGCCATGGCTGCTCGGGAGTGGCAGGGACCAGAGAGCTCCTTCTTCCTTTGTCGTGAAGAGGGT
GCTGGGAGGATGAACACTCTTGAAGTTGGAGGAGGGGATTTTACCTCTGGTTAAAGCTTGACCACCCAGAGGGGCAGGTGG
GGTTAGTGATTGCTTACTGAGCTTACTGAGCACCTGGCATCTGCTGGGAACTGAGTGCTTTCTTCCTTTCGATTCTTTAC
TTACAAAAATGTTTTGAAGGCCGGGCGTGGTAGTTCATGCCTGTAATCCCAGCACTTTGGGAGGCCGAGGTGGGGGGGAT
CACCTGAGGTCAGGAGTTCGAGACCAGCCAGGCCAACATGGCAAAACCCTGTCTCTACTAAAAATACAAAAAATTAGCTGG
GTGTGGTGGTTGCATGCCTGTAATCCCAGCTACTCATGAGCTGAGGCAGGAGAATCGCTTGAACACAGGAGGTGAAGGTTG
CAGTGAGCTGAGATCACGCAATTTCACTTCAGCCTGGGTGAGAAGAGTGAAACTCGGTCTCCAAAAAAAAGAGAAGAGAT
TCACATTACAAAATAAAACAAGAGAGGGAGATAAGTGCCCTTAGTATCACCTCCCACCCCTACCCCTTGCCCCAGAGGAA
ACCCACAGTTATCAGTTTGGGATATATTCTTCCCATCCATTTTTACCCATGTGCACACCTGTGTGTACGCAGAGAAATAC
GGAATTGTTTCTTCTTTGAACATCATGAAATCATACTACGTGCATTGTTTTGCAACTGGCTTTTTTCACCTTCGTGACCT
TTTCTCAATATGTGTGAAAATGACTGTGTGACTTCCCACTCCCCACTCCCCGTGGTAGTTACTGCAAAGACCCTGAGGGC
TAGGGACAAATCGTACCCCAACCGGTGCCCAGAGTGGCAAAAATCACCATCTGCAACTGGCAGTAGCCATTCACGATAGA
TGGAGTTCACATTCATTGTGGCTTGTATATTCCAATATACTGTACATGTGCACGTGATTTTAAACATCCTCATCATACAG
AAGATTGGAAAGTGAGCAGTTGGTTTTGTCGAGTCTGGGTCTCGCTCCCCAGAGTCAGTTAACCTGACAGGTTCTGTTTT
GGGGTTGCCCGGTGGTGGCCACCATTATGATTTTAAGTAGTTTGCCTGTGTGCCGATTGTTTTCAGGAGGTGCCTGTTGA
CTTCCGCTCTGAGGGCTGAGCACAGCCGCGCCCCACCTCTCACCTCACCTGCTCACCACACTTCTTTTCCAGATTCAGGC
TGGAAATGTGGGTGATAGGCCTATTTTGCAGATGAAAGAACTGAGTCCCTAGCGAAGTTACACATTCAGGGGTCTGGGAG
```

```
GGGGAAGCTGTGGTAGGCATTCTCTCTGTGTGTCTACATAGCCTGCCCTCTTCCCACCGTGCCAGTATTGGGAATTGAGT
GGCCGTGCGTGCACCAGGGTGAGTTAGGTGTGCAGCACCTGAGAGGGCTTATTAAGGGGCCTTGGCCCTACTGAGGGGTC
TAGTCTGGATGCTTCCCCCCAGGTTGACTTCTGGCATTCCGATCAGTGCAACATGATCAATGGAACTTCTGGGCAAATGT
GGCCGCCCTTCATGACTCCTGAGTCCTCGCTGGAGTTCTACAGCCCGGAGGCCTGCCGGTAATCACTGGGACTCGGGGCC
TCCTGGGTTTCCTGGGTAGCTCATGGCCAAATTCTGTGGTGTTGGCTGTGCACTTGGAAAGCATTTTGACTCATCGTGGA
TTTGACTCAGTAGCCCTTGGCACCAGCTTGAATTCTCTTTGGTCACACCACCAAAAGCCAAACTCCAGCTGCCAATGCCT
CTACACAGCCCAAAGAGAGGCTGCTGGTGGTGGTTTGCAGCCAGGTGGTGCTGGGCTGGGAGGAGGGGGGCAGGACATGTC
AAGTCACAGGGGGCCATAAAATGGAGGCGAGAGGTGGGCTCCCTCTGGCGTCTGGCTGGTGGCCCCCATGACCATCTGGG
TGCTGAGACATCAGCTGGCTCTGCTTGGCCCCTTCAGGCTCAGCTCTGTGTCCCATGGTCCCAGTTGTGGGGTAAGAGTA
GACAGTGGGTGGCTCCTACAGCCCTTTTCTGGAGGCCTTGCTGTGTCGTGTTCCATTCTGGTCACTCTGGAAAAGTCCTT
TACTTAGGAATTCACTCATGTGGACCTTGACTTATCTGCCCTCCCAGCAAACCCTGTGAAGCCCTACTGTGTGCCAGGTG
CCCGGTAGTGAGAGGGTGGGCCAAGTCTCCGTTCCCCAGGAGGCTGTGTCCTATCAGGGCAACTCAGGCAAATGCACAAA
CACTAGAGTGTCAGAGAGTGACAGTGCTTTTGCTCTGTGTGGCCACTTTTTACTATTTTAAGTTTTTACTTATTTATTTA
TTTAATTTTTTAGAGATGGGGATCTCGCTATGTTGCCCAGGCTAGTCTTGAACTCCTAGCCTCAAGGGATCTTCCTGCTT
CAGCCTCCCAAAGTGCTGGGATTGCAGGTGTGAGCTACTGTACCCAGCTGACTTTAAAAGCTGAATTTTAAACTTAAAGA
AAAGTTGCATGAGAAATACAAGTGGCCCCCGTCTCCCCTTCACCCAGGTTCACTAGCCGTGTTAAGCCCCCCACCCCCTC
TTCTCGCCGAGTGTCTTTTTCCAGAACCATCGGAGAGTAGGTTGGAGGCTGTATGCCCCCTTACCTCCAAATACTCAGTG
GGTGTTTTTCAGAAACGAGGACATTTTCTTACATAACCATAATGCTGTTATCAAAATGAGAAAATTTGGCATTGACATAA
TACTGTTATCTAAGCTGGCACTTTCCTATAAATCAAAATATGCAAGGCACACATGCAATTTAAAATTTTTTAGTGGCTGC
ATTAATACAAGTAACAAGAAATTGGTGAAATTATTTTTAATAATGTATTTTATTTGACCTCATATATCAAAATGGGATC
ATTTCAAGTGTTATTGATGAGGTTTTTTTTTGTTTTTTTTGCTAGCAAGTATTAGAAATCTGGAATGTATTTTATACTT
GCATCCCAATTTGGACTCACCCCATGTCAAGGGCGGGATGGACAGCCATGCGTGCTGAGTGGCTGCCGTGGACAGAGTGG
GTAGCACAGTGCATGTTCAAGTTCAGAAGTGCTTTTGAGAAAGTACAGGAGCATGAATGGATGGAGGGTGGATGGGGTGG
GTGTGGGGGACGGTGCTGCCACCCGAGGAAGTGACAGCTGAACTGAGATCTGACTGAAGGGCTGAAGTCTGGTGGATGAA
GATGCCAGAGGAGACTGTTCTTAGGCAGAGGGAGCAGTGATGCAAAGGGCCTGGGGCAGGAGTGAGTGTGCTTAGGAACT
GCAGGGAGGCCCTGGGCACAGGAAGTTGGGGCAGGGCTGAGCAGAGCCTTATGCAGGGGCTAGATAAGGTCAATGAATCCT
AATGGTAGGTTAGACTAGAAAGGGTCACAGATTTTCAGCAGGGGCATGATATATTCTGTTCTTTTTTTAATTTCATAACT
TTCAAAAACAATTTTTATTTTTAGAGACTGGGTCTCACTATGTTCCCCAGGCTGGTCTCACACTCCTGGGCTCAAGTGATC
CTCCCACCTCGGCCTCCCAAAGTGCTGGGATTACAGGTGTGAGCCATAATGCCTGGCCTCTGTTCTTTTGTAAAACTCCA
TCTGGCCACGGCTAATACATTGGTGTGGGTGGGGAGGCAGAGTCGGAGCGGAGAGGCCTGTTAGGAGGTCGCTGCAGCTC
CGCGGGTGAGAGATGGGGGCGGTTTGGACCCGGGAGGTGGTAGCGCCCGTGGGGAGAAGTGGCTGGATCTGGGCAGCCTT
TGGCAGGGCCTGGCTCTGGCCGCCGGGTCTGGGTGTCCCCTCTCATCCTGTCTGTCCCCTGCAGATCCATGAAGCTAATG
TACAAGGAGTCAGGGGTGTTTGAAGGCATCCCCACCTATCGCTTCGTGGCTCCCAAAACCCTGTTTGCCAACGGGTCCAT
CTACCCACCCAACGAAGGCTTCTGCCCGTGCCTGGAGTCTGGAATTCAGAACGTCAGCACCTGCAGGTTCAGTACGTGCC
GTCCCCTGTGCTGGGATGGCGGGAGGGTGTGAGGGTGGGGCCAGCTGAGGGTTTATCTGCCCAGTGCTGTCTGCTTAATC
TCTGGCCTCTGTACTCTTGATAATCCCATTAGGCGAAGATAATGAGGCCGCAGGAAGATAATCTGAAAAGGAAGAAATGC
CAGGTCTATTAGTTGCTGTCACAATATATTGCCATGAACTTCAGGGCTTAGAACAACACACACTGGTTATCTTGCAGTTC
TGTAGTCAGAAGTCTGCGTGGGTCTCACCGCGCTCAGAACAAGGCGGCTGTGTTCCTTCTGGAGGCTCTAGGAGAAGCTG
CGTCTGCCTTTTCTGGCTTCCAGAGGCCCCTCTGTGTTTCTTGGCTCCCGGGCCCTTCCTCCACCTTTGAGGCCAGCAAC
ATGGCATCCTTCTGATCCACTCTGTCCTCATATCTCTTCCTCTCCTCCCTCTTCTGCCCCCTCTTCCAGTTCTAAGGACC
CTTAAGACCACATCGTGCTCTCCTGGATACCCCAGGACAATCTCCCCATCTTGTTTATTATTATTTTTTTTGAGACAGAC
TCTCACTCTGTTACCCAGGTTGGAGTGCAGTGGCGCCATTTCAGCTCACTGCAACCTCCGCCTCCCAGGTTCAAGTGATC
CTCCCACCTCAGCCTCCCGAGTAGTTGGGACCACAGGCGTGCACCACCACACCTGGCTAATTTTTTGTATTTTTGGTAGA
GATGGGGGTTTCACCATGTTGCCCAGGCTGGTCTTGAACTCCTGACCTCAAGTGATCTGCCTGCCTCGGCCTCCCAAAGT
GCTGGGATTATAGGTGTGAGCCACCGCGCCCAGCCTATTTTTATATTTTGAGACAAGGTATCACTCTGTAGCCTGGAGCT
GGAGTATAGTGGTGCGATTATAGCTCACTGCAGCCTCGACCTCATGGGCTCAAGCGATCTTCCTGCCTCAGCCTCCTGAG
TAGCTGGGACCGCAGGTGTGGGCCATGATACCTAGCTAATTTTATTTTTGTAAAGTTTTTATAGAGATGTTGTTTCACCA
TGTTGCCCAGGCTGGTCTCAAACACCTGGCCTCAAGTGATCCACCTGCCTCTGCGTGTTAAAGCAATGGGATTACAGGTG
TGAGCCACTGTGCCCGGCCCAATTTCTGGACCTTCAAGGCAGCTGCTGAGCAGGCCCAGCGCCCGTTTACCCTGTGACCT
GACATAGTCATGGGTTCTGGGGCTTAGGGCATGTACGTGTCCTAAATGACATGAAATGAGGCTGGCGAGGGTCTGTGTGT
TACGTAACATCTTCTCGTTCCGCAGTGTGGGTGGCTAGGCAGATGTGATGACTGCTGTTGGCGTCGGTCCCCTGACATTG
TTGGTGTGTCTCAATTGTGTGCCAGTCCCTGAGACACCCCCACCTCAAGTTGCTCAGTTTGGACCCTCGGGCGTCAGTTT
GATTCCCTGTCCTCACCCAGCTCTTCCAGTGCGTCAACAAGCCCAGATGGCTTTACCTCCAGGTTGAGTCCTCACTCCTT
CCTCTCTCTTGCTCCCCACCCCAACCCCCGCCTCATTGTCCAGGCCACGGACACGTCCTTCCTGGTGATCCAGCCCTTTC
AGTAGCCCATCCCTCAATGCAGTGTTGCCGCCTTACCATCCATCCCCTAGACCATCCAGTGCGGTCACTGCTGGCCTCTT
GCGGCTGTTTAAATTGAAATTGATTTAAACGAAATCAAATTAAAAGGCAGTTCCTTGGTTGCACCGGCCGGAAGTTAAAT
GCTCATAAGCCATGTGTGTCTAGCAGCTACTGTCTTGGATAGCACAGATAGAGAACATTTCCATCATCATAGGAAGTTCT
GTAGCACTCCCGACAGCAGCGGAGGGATCTTTTAACAATGCAGGCCGAGTGTGGTGGTTCACGCCTGTAATCACAACCCT
GTAGGAGGCCGAGGTGGGAGGATCACTTCAGGCCAAGAGTTCGAGACCAGCCTGGGCAATATAGCAAGACCTCTCTCTGG
CAAAAAAAAAAAAAAAAAAAAAAATTAGCTAGGCATGGTGGTGCATGCCTGTAGTCCTAGCTACGTGGAAAGCTGGGGTGG
AAGGATGGCCTAAGCCCAGAGGTCAAGGCTGCAGTGAGCTGTGATCACCCCACTGCACTCCAGCCTGGGCAACAGAGGAA
```

341

```
GACCCATCTCGAAAAATATGCAGATGGCCGCCGTCCTCTTAAAATGTCTGGCAGCTCTGTCTGCATTCATAGTGAAGTCC
AAACTCCCACCATGGCCCTGGGCCCTGCAGGATCCAGCCCCTACCGGCCTCTCTGAAGGATCCAGCCCCGCCAGCCTCTC
TGACTCCATTTCTCCCTACACGTCCCCTGTTCATTATTCTCCAGCCCTGATGCCTTCGCCCTTTTTCTAAAGTATACCAA
GGTCAGTGCCTTGCACTGTGGTTTGCATGGGTTGCTTCCCTGCTTGGAGAGCTCTTTGTCTGGATTTCTGAGTGGATCA
TCCCTCACCTTCTTTTGGCCTGCATGCAAATGCCACCTCCTCCGGGAGGCCTCCTCTGATTCCTGTTCTAAAGTTGCCC
CCTCTCCCACCTGCTGCTCCCTATGGCTTCCTCTTCTTGTCTTCTTGTCTTGGTCTTCTTGTCTTCCTCTTCTTGTCTTG
GTGCTCATTGCTCCCTGAACTCATGGACTGTGGATTTTATTCATTGCCCCTTCCATTCACTGTCATCCCTGTAGGCAGGG
CCCCAAGCTCCTCTTGTTGGTTTTATTCTTAGCTTCTAGAACTGTGCCCCGCATGTAATGGGTACTCATACAGTATTTGA
ATATAGGGATGGTGAGTGCGTTCTGTACTTAGGAAATTGAGGCCTGATGCTGGATGCGACCAAGGTCACAGGGTTGGTAG
GACCGGAAGCGGCCCTCTATTTGACTGTGTCTTGCCTTCTGGGAGCCTTTTTGGTTGAAGCCCCGTCCTCCAGCTGGCAA
TGGCGACTCTAAAGGACTGGTCTGCAACAGGGGTCAGGAGGAGGGGCTGTCAACAGATTGGATGCTTTCCCCTATTTCAG
GCAGAATGGAACAACCTGTGAATGAATGGTTTGGAGCTGGGCCTGGGACGCCCGCTGCTGTCTCCTGGTCCTGTCTGGC
CCGTCTGGGCATTGGCCTCTGCTTGCTTCATTCTTTGCTGGTATCGGCGGGGATTTCTGCTCCTAAGGTTGAGGATGTTA
CACACCCAATAGGTGTGCTACAGCAACTGTCGCAAGTGGCAGATGCTGGACTCCACCTGATTTAGGCCAAAAGGGCACTT
CTTGGTTTGCGTAATGAAAAATTCCAGGGCAGATCTCTGCATTAGGCACAAATCCAGAACTTAGATGTTGTCTGGAAGCT
GCCTCTGCCAAGTCCCAGCTCAGTTTTCTGCTGGACTGGCTTTGCTGCCAAGCAGGCTCTCCCTCCTGGCTGCCTGTGGA
GGCTCCAGGCTCACCTCCGCCCAGCTCCGAGTCCTGCAGAAAGGGGGCCACCTGTGTTTCCTTCACTCCAGCAGAAGTTC
CAGGGTTTGCTCTAATTGGTCTGCCTGGCTCCCATGCCCAGCTGTAAACCAATCACAGTAGCCGAGGTGGACTATGCTGA
TTGGCCAGGCTTGTGTCACATGCTCAGATGGCTCCTGGGTGGGCCGGCCCCACTCCAAGCACAAGGCTGAAAGTAGGGAA
GGCTTAAAGTAGGGGTGTAGGTACCAGGAGAAGGGGGAGAAACGACATGCCCATCCCCTCCAAGGGCGTGGGACGAGGTG
TGTCCCGCCTTCTCTTAGCACATCTGCCAAAAGAATTTCAAAAGCGCAAAGGAATTTTAAAATCTTTATTTAAATAGAGT
TTTTTCTTATATTTATTTATATTTTTGAGACAGGGTTTCTCTGTTGCCCAGGCTCAAGTGCAGTGGTGCAATCACAGCTC
ACAGCAGCCTTGACCTCCTGGGCACAAATGATTCTCCCACCTCAGCCTCCCAGGTAGCTGGGAGCACAGGTACACACCAC
CATACCTGGCTAATTTATTTTATCTTATTTCAATTTTTTTTTTTTTTTTTTTTTTTGTAGAGGTGGGGTTGTGCCGTCTT
GCCCGGGCTTAGTTTTTGCTTCTAAATAAAATATACATAGATACAAAGAAGTTCCCAGATGACTCATGCAGTTTTTGGGG
TTTTTTGCTTTGTTTTTTGAGACGGGGTATCACTGTCGCCCAGGCTGGAGTGCAGTGGTGCCATCATGGCTCACAGCAGT
CTGACCTCCATCACTCAATTGATCCTCTTACCCCAGCCTCCTGAGTAGCTGGGACTACAAGCGGTTGCTACCATGCCCAG
CTAATTCTTAAATTTTTTTTAAGAGACGGGGTCTCACCATATTGCCCAGGCTAGTCTTGAAGTCCTGGGCTCAAGTGATC
CTCCCGCCCAAAGTGCTGGGATTACAGGCATGAACCGCTGCGCCTGGCTGACACCTGCAGTTTGAAACTTCACAAAGTCA
ACGCACCCCGTGGCCAGCACCCTGACTGAGAAACAGACAGGACAGGCCCTGGATGCCACTCGCGGTCCTCCCACCGCCCT
GACATCCAGCCCCGGAGCTCAGCTGGTTTGCATCTTCATGTGAATGGACCCATAATGCTAGACTCCTTTTCCTCCTTTTC
TGGCCCGTGCACCCCTTAGCACCCTGGTGCAATTCGTCCGTGACGTTGTGGGTGGTAGCAGCCCCTCCTCTCCGTTGCTG
TGTGGTGTTCCATCGTGGGTGCCACCTCCATCTTCTCCTTCCATTGCTCCTAGGCATCCAAGTTCCTGTTTTTCGACCGT
GACCGTGGTGCACACGTGTCCGTTTGGTGCACCTATGTCTGTGTTTCTGTGGCATGTTATCTAAAGAGTAAAGGTACCATG
CCTTTGCCTTTCTGGGCCTTGAAATCTGTTGGACTCAGACAACTCCCTCATCTCAAACCTCCCTGTTGCGTGAGGGCATC
CGACTTGCCTCACAGAGCCAACTAATTATCATCGGTGGCAATCCTGGGCAATTATGTCTGGCTAGATATGCCATAAAGCT
ACAAATTAATTACCACTTACAGCTGGGAAGATCTCTCTCGGCGTGCTTGGTAATAACATTGGAAGAAACACGTGCGTGGG
TCCATGCAGGCTCGGCCTCTGAAGCAAGGGCGGGGGCTGGCAAGTTGTTCCATCCCACCTTGGTCTCCACCTGGGGCTGT
GGGCAGGCCAGGGCTTGTTTGAGATTCCTCCAGAGTTCGGCTCCGTGGCTGCCTCCTCCACGGAGGAGGCACGAGAGGCA
GCGTTTTCCAGATTGTGGAGGACACTGTCCCTGCGGGTGCTTGGGATGGCTTCAGAAGGTCCAAGGAGGTGAGAGTGAGC
TTCTGGGTCCATGCTCTTTTCCTCCTTGCCATCACCTGCAGGAGAAGGTCTCTTGGTGTTGAGAGGACTTGAAGAGCCTT
CCACACTCATCAGCCTTTTTTGTTTTGTTTTTTCTTTTAACAGAAAAAGAGCAGGGCTCCCAGTTAAAGCCTCATGCAGT
AACAGGTTCTAGTTAGAACTTACAACATCATTATTTTCCACGGTATTTTTTAACTATGTCTTTTTTTTTTTTTTTTGAGA
CACAGTCTTGCTCTGTTGCCAAGGCTGGAGTGCAATGGTGCAATCTTGGCTCACCACAACTTCTGCCTCTTGGGTTCAAG
CGATTCTCCTGCCTCAGCCTCCTGAGTAGCTGGGATTACAGGTGTGCACCACCAGGCCCGGCTAATTTTGTATTTTTAGT
AGAGACGGGGTTTCTCCATATCGGTCAGGCTGGTCGTGAACTCCCAACCTCAACTGGTCCACCCGCCTCGGCCTCCTAAA
GTGCTGGGATTACAGGCGTGAGCCACCGCGCCCGGCCCTTTATTTTATTTTATTTTTTTTGAGACACAGTCTTGCTCTTT
TGCCGAGGCTGGAGTGCATTGGTGCAATCTTGGCTTACTGCAACCTCCGAAAACTATTGTCCAGGCTGGAGTGCAGTGAT
GCGATTTCAGCTCCCTGCAGCCTCCGCAACCTCTGCCGCCCAGGTTCAAGCAATTCTCCTGCCTCAGCCTCCCGAGTAGC
TGGGATTACAGGCACATACCATGCTCAGCTAATTTTTGTATTTTTAGTAGAGACGGGGTCTCACCATGTTGCCCAGGCTG
GTCTTGAACTTCTGACCTCAAGTGATCCACCCGCCTTGGCCTCCCAAAGAGTTGGGATTACAGGCATGAGCCACCGTGCC
TGGCCTCTGATTCAGGTTTTTGTTTTGTTTTGTTTTGTTTTCCTTACTGAGGCAAAATTCTTGTAACATAAAATTAACCA
TTTTAAAGTGAATGATTCGGTGGCATTTAGCAGGGTCACAAGGTTGTGCAGCCATCACTTGTATCTCGTTCCAGAACATT
TCTGTCACCCCAAAAGGAGACCCAGACTCATCAGAAGTCACTCCCCGCCCCCTCCCCCAGCCCCTGGTCACCACCCGTTG
GCTCTGTCTGTGGATTAGCACTGTCTGGATCCTTGTGAGCTTTTTAAAAAAAAATTGTTTTTCTTTTGAGACAGGATCTT
GCTCTGTTACCCAGGCTGGAGTGCAGTGGCACGGTCACGGCTCACTGTAGCCTCAACCTCTGAGGCTCAAGCGATTTTCC
TGCCTCAGCCTCCTGAGTAGCTGGAACTACATGCAGGTGCCACCATGCCCGGCTAATTTTTGTAGAGATAGGATCTTGCC
ATATTGTCCAGCCTGGTCTCCAATTCCTGGGTGCAAGTCATCTGCCTGCTGAGGCCTCCCAAAGTGTTGGGATTACAGGT
GTAAGCCATGGCGCCTGGCCCTCGTGAGCTTTTGAGAGAGGCCCGCTCGCTGTTCCTTGTTAACTTGTCCTTCAGGCCTG
TTGTCCATTGTCCCGAGGGTGAGCTGGTGTTTGCCTGGTTTGGTTGGTCAGTGGCGAGGTTGAGCTCACATTTGTAGCAG
GTGTGTCTACAGGGACACACTCCTGTGGATGCAGGGAGATCTGGCCACAGGTGCACACACCTGAGCTCCGATCATTGCTG
```

```
CGTGTCCTGCAGGGGACAGTGTGGGCTGCTTGGCTTGCGCTTGAGACTCCCCTCCCCACACCCAAGTTCAAATCTGGCGT
TGCCTTTTCCATCCGTGCCTCCCTAGCACCCTTGTGCACCATGCATTTCCCCCTCCCCACGCTTGCTTCTGCCTTCCCTT
CCACCTGGAATGCGCTTCCCGGGCATCCTTCACCTGGCTCCTGTGAAGAGGCCTCTTCCTTCTTTTGGTCTCTCCTGTCA
GAATCAGCGGCTCCTGCCTCACCCCTTCTCTGGCGCAGAGCTTGTCCCTCATCACAGGGCCTGGGGCTTTTTACAGAATG
GAGGAAGGGATCCTCTCTGTCTGGTTATCTTGTCATCGCCACGGGGGTGCCCTGCAGACCACAGCTCTGTGCAGACCTCC
GGCCTGGCAGGACCTGCCAATATACTGTCCTTGTCTGATGTCCCCTCCCTGCCCCTCTTCTAGGTGCCCCCTTGTTTCTC
TCCCATCCTCACTTCCTCAACGCCGACCCGGTTCTGGCAGAAGCGGTGACTGGCCTGCACCCTAACCAGGAGGCACACTC
CTTGTTCCTGGACATCCACCCGGTGAGCCCCTGCCATCCTCTGTGGGGGTGGGTGATTCCTGGTTGGAGCACACCTGGC
TGCCTCCTCTCTCCCCAGGCAGAGAGCTGCTGTGGGCTGGGGTGGTGGGAAGCCTGGCTTCTAGAATCTCGAGCCACCAA
AGTTCCTTACTTCACCCCGACTCCATAGTTCAAGGTAGTTCAAGGGTTTTATGATCCCTGTACTGGTTTCTATAAATGGG
CTCTAAGACAGTAAATTAATTAGAACTTATCAGCTGGATGTCTCCTACATGTGAGCTGGAGGCAGCCCTCTGGATGTGT
CAAGATACCATAAAGATCTTTAGGTACCAAGAAGAGCCTGGGGTATTTTGCAGATAAATCACACAGGGAATTTGTCCCGT
GTGAAGTTCTGTCTACAAGCAGGGAGCTGGACATGTGGCCCTCCCAAGGACACTCCTTCAGCTTGTGGCCTCTTCTCTTG
GCACCTCCGTTCTCTGTAAAATATGCCCAGTTGGTCTTGGTGGCTGCTACCATTGATTCAGAAATCTGTGGTTTTCTGTT
TCCTGGGTGGGCACTTGTGGTCTATCCCATGGGCCTTTAACAAAATTATTATTATTTTTGAGACAGGGTCTCACTCGGTT
GCCCAGGCTGGGGTGCAGTGGCATGATCATGGCTCACTGCAGCTTCGACCTCCCTGGGTTCAGGTGATCCTCCCACCTCA
GCCTCCCTAAGTAGCCAGGACTACAGACATGTACCACCATGCCTGGCTAATTTTTATATTTTTTGTAGAGACGCGGTTTT
GCCATGTTGCCCAGTCTGGTCTCGAACTCTTGGGCTCAAGCGATCCTCCTGTCTTGGCCTCCCAAAGTACCGGGATTACA
GGCATGAACCGCCGTGCCCAGCCAAAATTAATTCTCTAAATTGAAAAAGAAAAGCTGTCTATTTTTGAGTGTACAACATG
ATGTTATGATGTATGTATATAACTTGTGGAATACCTAAATCAAGCCAATTAACATATGCATTACCTCACATACTGATGAT
GTATTGGTGCTGAGAACATTTAAAATCTACTGTCAGCAACTTTCAAGCTTACAATACATTGCTATTCACTCTAGTCACTG
TGTTGTATTGTATAGTACAATACAGTGACTGTTGCTCTATTTCCCAGTAGGTCTCCTAAACTTACTCCCCTGTCTAACT
GAAATTTTGTATCCTTTGACGGACTTCCCCATCCCTGCCCCTGGTAGCCAGTATTCTACCCTCTGCTTCCATGAATTCAA
CTTTTTTTTTTTTTTTTTTCTGAGATGGAGTTTCACTCTGGTTACCCAGGCTGGAGTGCAGTGGCACGATCTCGGCTCAC
TGCAACCTCCGCCTCCCGGGTTCAAGCAATTCTCCCTGCCTCAGCCTCTCAAGTAGCTGGGATTACAGGCACACACCACC
ACACCCGGCTAATTTTTGTATTTTTAGTAGACACAGGGTTTTGCCACGTTGGCCACGCTGGTCTCGAACTCGTGACCTCA
GGTGATCCACCCGCCTCGGACTCCCAGGTTGCTGGGATTATAGGCGTGAGCCACTACGCCTGGCCTGAATTCAACTCTTT
TAGATTCCACATACAGGTGAGGTCATGCAGGGTTTGCTTTCCTGTGCCTGGCCTATTTTACTTCGCATAGTGTCCCCAGG
TCCGTCATGTTGTCACAAATAACAGGATTTCCCTGTTTTTATTTATTTATTTTTTGAGACAGAGTCTTGCTGCGTCACCC
AGGCTGGAGTGCAGTGGCATGATCGTGGCTCCCTGCAACCTCTGCCTCCCGGGTTCAAGCAATTCTCCTGCCTCGGCCTC
CAGAGTAGCTGGGATTAAAGGCACACACCACCACACCTGGCTAATTTTTGTATTTTTAGTGGAGACGGGATTTCACCATC
CTGACCAAGCTGGTCTGGAACTCCTGGCCTCAAGTGATCCGTCCACCTCGGCCTCCCAAAGTGCTGGGATTCCAGGCGTG
AGCCGCCGCGCCCAGCTGGATTTCCCTCTTTTTAAACGCTGAACAGTATCCCACTGTGTGTGCACCACGTTTCTGCTGCC
CATTCATTCACTGACGGACACTTCGCTTGATTCCGTATCTTGGCCACAGCGTACATGGGAGTGCCCCTGTCCCTTTGACA
CACTGATTTCATTTCCTCTGGGGGGATGCCCGGCAGTGGGATCACTGATCCGATGGTAGTTCTGGTTTTCGTTGTCTGAG
GAAGCTCCACACGTTTGCCCACAATGCTGTGCACCTTAGGCCCTTCCCGAAGCTCCATGGCGGATGCTGTTCTGCACCATT
TTTCTTTGTCTCCCAGGCCTCCTTAGCCGCTGACTAGGCCCTCTCGGCCCCCGGCCCCAGCCTGGTCTCTGCCTTCCCT
CTTTTAGTTAATTCCTCCTCCAGCCTCTCTTGCTCTGCTGGCTTCCGGAGAATTCCACCATGAACACAGTCTCCTGTGCG
CTCTCAGTGATCGCTTTCCTTTCAGGGCACGGATTTTCTTTCTTCCCCTCACATCAAGAAAGAAGGGGTTAAGTTTGGGA
GCCCAGAGAGAGGCAAACAGCTGTTTTGCCGCAGCCACTTTAGCTGCTGCGTCCAATTCTACCTCCAGATGAGCTTGAGG
CCGACGGGGAGAGGAAGTGGCCTTTATTTCTTTGTTTTTTGAGCTGGGCACGTTGGGGAGGGGGATCAGGAGATCAGCTT
GCTGCCTGTTGGCCTTTAGCCTTGAAAAACGAACGTGTGTTGGAAGCTGCTTCCGTGTGGCCACTTTTCTGCTCCTGAAA
CTGGGGGAGGGCATGGGGACGGGGGACTCAACAGGACGCTGCTTATGGTGGTATCACAGCTAAGACTTATTGTAATGACA
GAATTCAAAGCAAAATTAGCAAAGGGAAAAAGCACCTTGGGTGAAGTCGGGGGAGGCCAGGCTGGAGCTTCCAGAGTCCT
CTTCCAGGGGGGTTCCTACAGGATGCACTTAATGTCTTTAGCAATGAGTTGTGACAACATGTAGGAGATGTTGTCTAGCAG
GGAAGCTCATTAGAAATTCAGTGCCCATGGTGTTTTGCTAGGGGCCAGTCATGTGGGCACCTCTGCCTGGCAGGTACCAA
AATTCTAGGCTCTGGGAAGGAAAGCAGGTGCTGAGCAAGAACCATATTGTTTGTACAAATAATTTCGGCCCAGTGAACTA
CTCTCATCAGTTAGGGGATGGTGGGGACCCTCCTGAAATCTGGGTTCCCAGATGCCAGCAGAATTCCTAAGGAGAGCAGC
TTCAGGCCTGTGGCGTGAACTCTGTTCTACTCACGGGTGATCTCATGCCCATCCCACCAGGCTCTCGAGGGCAAGCACTC
CCCAGTCGTGGGCGTAAGTGAGGCCTTTAATGAGCTCTTCTTTGCCACAGGGGCCTGGAAGGTCCTCAGCATGCTGGGCC
ATAATGAAGAAAACATCCTTTCCCTATAGTGTACGTGATAATAACCTAGGCATTTATGGCCCTGCGTGTGGCTATCTTGC
ATACCTTACTTTTGCTCTTGCAAAAATTCTGTCAGGTCGGGAGAGTTCTCTCTGTTTTACAGATGAGGACGCGACTCTCA
GTTAAGAAGAACCAACTGCAGTTCTCTGACCTCAAATCTAGTTTTATTTCCACTATCATTTCCTGACTTCATTCTGGAAC
CACCTTCCTGTTGTTTTTGATGACACATGTGTGTCACTTCGGTTATTTACTTAAAAAAAAAAAGTCTCGTTAAAAAGAGCT
GGGTATAGTGGCTCATGGCTGTAATTCCACTGCTTTGGGAGGCTGAGGCAGGAAGATCACTTGAGGCTAGGAGTTTGACG
CCAGCCTGGGCAACATAGTGAAACTCTGTCTCTACAAAAATAATAATGATAAATAGGCTGGGTGTGGTGGCTCACGCCTG
TAATCCCACTTTGGGAGGCCAAGGTGGGCAGTTCGCTTGCTGCCAGGAGTTTGAGACCAGCCTGGTCAACATGGTGAAAC
CCTGTCTCTTTACTAAAAATACAAAAATTAGCCGAGTGTGCTGGCGCACACCTGTAGTCCCAGCTACTGGGGAGGCTGAGGC
AGGACAATCGCTTGAACTCAGGAGGCAGAGGTTGCCTGCACTCCAGCCTAGATGACTGAGTAAGACTCTGTCTCAAAAAG
AAATAAAAATAAATTTCTGCACAAAGGAAGCTTTTTCTTACTACCTCAAATAGGAAATTACAAAATTCTCCAAAAAAAGA
AGACAACCATAAAAATGAACAAGGAAAAAAATATTAGGCTCTATGTAAATATTGTTGTCTGTCAGCAGGAGTGAACCTG
```

```
GGGCCAGCTGTCAGCACCATTGTTAGAAGTGGAGATTTTGTGTAGGTGTATTAGAGGGGTATTAAAGACCATGCAAACAC
CTGGCTGAGACCTACTCTTCAAAATGATCAGGAGGAGTTGGGAGTGAATTGAGAAGGGACTTTTGTCTGTTTTATTGAAT
GTGGAGGTTGAGAGGGAGCATGGACAAATTGCCATTCTGCACTTTGGGAAACGCTGTCTTAGGAGGACAGGTGAAAGTGA
TTAGGTATTTTCCACCCTGAGACAGCCTTGCTTTATAGAAGGAACCTCTTTGAGGCTGTGAACATAACTGTGTGGGAAGC
ACTTGTTTCCCTATAAGGGGTTAGAATCAGGAGAGGAGATCCCAGTGCACCTGCCCAGCATTTCTAGAACTGTTGACCCC
CCCCAGCCTGTGGCTTGTTTTAGGTAAGATACAAGCAAGCTCCACTGGGCAGTTAGCTGGGACGCCCACCCTCTTGACTG
GGACCAGGGAGAGGAGGGTTGACGGTGTCCCTGGAGCTTGGGGGTGGCCAGTCTCCTCACTGTGTTTGTTGCCGCAGGTC
ACGGGAATCCCCATGAACTGCTCTGTGAAACTGCAGCTGAGCCTCTACATGAAATCTGTCGCAGGCATTGGGTGAGTGGG
GACTGGGAGCTGGGGCTGCATTGCTCATTGAGAGATTAGGTGCTCAGTGCTCCAGTGGTCCCAGACTCCAGTGACATACC
CCAGGAACCAGGGCATGGGGAGGGGAGAGGGTCCTATTGGGGGTGGAATCCAGTCCCTGCTGATCTTCTCCCTGAGGGGT
CTTGGTTTTGAGCCAGGTCCTGACCTGCATCCTGATATTCCTTCCCCAGAACACCACTTTTCTTTCTCTTTTTTGTTTTG
TTTTGTTTTGTTTTGAGATGGAGTCTCGCTCTTGTTGCCCAGGCTGGAGTGCAAGGGCGGGATATTGGCTCACCACAAC
CTCCGCCTCCCAGGTTCAAGCGATTCTCCTGACTCAGCCTCCTGAGTAGCTGGGATTACAGGCATGTGCCACCACGCCCG
GCTAATTTGGTATTTTTAGTAGAGATGGGGTTTCACCATGTTGGTCAGGCTGGTCTCAAACTCCTGGCCTCAAGTGATCC
ACCCACCTGGGCCTCCCAGCATGCTGGGATTGCAGGCGTGAGCCACCGTGCCCGGCCTTCACTTTTCTTTTTTAACATTC
ACCCCCTCCCTGCCAATTAGCCAGAGCTTCAATCCAAGCTGTTTCCTACACGACAGAGTGGCAGAAATGAAAGCCTGGCA
AAACCAAATGTTTGAAAATGGAAACGCTGAGGCAGGAGAATGGCGTGAACCCGGGAGGCGGAGCTTGCAGTGAGCCGAGA
TCGCGCCACTGCACTCCAGCCTGGGCGACAGAGCAAGACTCCGTCTCAAAAAAAAAAAAAAAAAAAAGAATTTTTCAAGCAC
TGATTTCCTCTTTTTGATTTTTAAAATAAAACAGCTTTATGCAAATATAATGCATGTACCATAAAACTCACCCCTTTAAA
ATGCACAATTCAAAATAAAAAAAATAACCCCCCTAATAAAATGCACAATTCAGTGATTTGCAGTATATTCACAGAATTGT
GTATCTATCACCACAATCAATTTTGGAACATTTTCATCATCCCCAAAAGAAACCCCACACTCATTAACACTCACTTCCCT
GTCCCCTCAGCCCCAGGCAACCGTTCATCCACTTCCTGTCTCTGCGGACTTACCTATTCTGGGCATTTCATATAAATGGA
ATCATACACTATGTGGTCTTTTGCGACTGGCTTCTTTCATTTAGGATCACGTTTTCAAGGTTCATCCATGTCGTAGCATG
GATCAGTATATTTTATGGCTGGATAATATTCCATTGTCTGGATAGACCACATTTATTTATCGGTTGATGGACGTTTGAGT
TGTTTCCACGCCTGGGCCGTTATGCATAATGCAGCTGTAAATGTTCTTGTGTCAGTCCCAGTGTGGACATCTGTTCTCGT
TTTCCTTAGGTATGTACCGAGGGGTGGAATCGCTGGGTCCTATGTTAACTCTTTGTTTAACCGTTTGAGGAACAGTCAGA
CTTGCACAGCCTCTGCCCCCCATCCCCATTCTTTTTTTTTTTTTTTTTTTTTTTTGAGACGGAGTCTCGCTCTGTCGCCCA
GGCTGGAGTGCAGTGGCGCGATCTCGGCTCACTGCAAGCTCCGCCTCCTGGGTTCACGCCATTCTCCTGCCTCAGCCTCC
CAAGTAGCTGGGACTACAGGCGCCCGCCACCGTGCCCGGCTAATTTTTTGTATTTTTAGTAGATACAGGGTTTCACCATG
TTAGCCAGGTTGGTCTCGATCTCCTGACCTCGTGATCCGCCCGCCTCGGGCTCCCAAAGTGCTGGGATTACAGGCGTGAG
CCACCGCGCCCGGCCCCATCCCCATTCTTAGCAGCAGTGCAGCAGGGTTCCAGTTCCTCCCCACGCGCACCAGCACTTGC
TATAACATTACACGTTGCTAGAACTTGGTGAGGGATTATACTGATTTGTTGCTATGTCAGTGTCTGTACTTCTTAGCATA
TCTGAAATAGTTTCGTTAAAAAAAATTCTCTTAGAAAAATCCCTGGGTTGCAGGAATGTGAGCATCTATTCAGCTTTGTC
ΛAATGCCTCTCGGCTGGAAAGAATAACACTTTGTCAGAGCACGGCAGCGAGTAATAACTGTGAGCTCTCTTCCTTCATCC
CGCCCTTGCATTTTATTTTTATATTTTGAGGCCACTTAGGGAATTTGTTCTTGATGGATTTGTGGGTGGGGAAACAGCCC
CAGGCATGGAAGAGGCGTTTGCAGCCCAAGTCCTCCCTCTGGTTCCACCGCGTGGCACCTGGGCTGCTAACTGGGATGCA
ACTGGGGCCAAGTGGGTGACCCAGATAGAAGAGGCGACCTGGGGCCGAGGATACAGCCCCTTCCCAGCACCAGCTGACTG
TAGCCCCATGGAAATGCGGGCTCAGTGTGGCCACATCCTCTGCATTTTTCAAAAGGACTTCCAAATCTGAATTTTAACAG
GAGCTCTGTCAATTTTTACTTATTGGGAGGTAATTCACATTCTTTTTTTTTTTTTTTTTTTTTTTTGAGACGAAGTCTT
GCCGTGTCACCCAGGCCAGAGTGCAGTGGCATGGATCTCAGCTCACTGCAACCTCTGCCTCCCGGGTTCCAGCGATTCTCC
TGCCTCAGCCTCTCAAGTAGCTGGGATTACAGGCGCTCGCCAGTACACCCAGCTAATTTTTGTATTTTTAGTGGAGACAG
GATTTCACCATGTTGGCCAGGCTGGTCTTCGAACTTGTGACCTCAGGTGATCCGCCTCCCAGAGTGCTGGGATTATAGGCA
TGAGCCCTGCGCTGGGCCTAATTCACATTCTTAACAAACAGTTCACGCGGGCAGCTGGATTGTGCCTGCCAGTGACCTGT
GGACCGGTCACCCAACCTCTCTGGGCACGCACAGCTGCTGACCTCCCTGTGGACTGGGAACAAGGCACTCCAGGAAAGTGG
TCTCAACAGCAGATGTGGAGGGCCACGAGGGATGGCCGTGGGAAAAGTCTAGAGACACAGCTGCCGGGAAGCAGAGCTGT
CTCGTGACCTGTCGGGGAAGCTTCTGTGCTCCTTGCTCGCTAGGTAAAGGCAGTGGGGTTGCATGTTACTTGAATACAAT
TCCCATTGGTTCTTACAAAGTCTTTCAGAGAAAAGCAGCCAAAGTAAATGGTACCGTCTGTTGTCTCCCCATCGTGTTTC
GTCCTCTGTAGGGTGATAGTGTGAGCCCATTTCACTGTGCAATACCCTACTTAACAAGGGGCCTGAGAGACCTCCAATAA
TTGTGATTGGGGGATTTCAGCCTTTTTTTTTTTTTAAGATGGATCTTGCTCTTGTCGCCCAGGCTGGAGTACAATGGCGC
GATTTCGGCTCACTGCAACCTCCGCCTCGGGTTCAAGCGATTCTCCTGCCTCAGCCTCCCGCGTAGCTGGGATTACAGGT
GCCCGCCACCACGCCCAGCAAATTTTTGTATTTTTAGTAGAGACGGGGTTTCACTGTGTTGGCCAGGCTGGTCTCAAACT
CCTGACCTCAGGTGATCACCTGCCTCGGCCTCCCAAAGTGCTGGGATTACAGGCGTGAGCCACCGCGCCCGGTCTCTGAT
TGTGGTATTTGAGCCTTTTGTGCTGACTTTCGTCACCGCCCCTCAGTGACTTCCATCTCCCCTCCCTACTGCTGTGCATG
GCAAAGTGACAGAATATTTGTGTGTCCATTTCTTTGCTGGGATTTAACAATTATAACAACAACAATAATAAATGTGCCAA
GCACTTTTATATGCCGGGTAATTTTCGAAGCACTTTACATGTATTACTGCATTGAATCCTCCCTAGAGTCCTAGGAAGAG
TTACGTTATAATATCTCGCTTATACAGAAGGGACACAGGCTCAGTAGCACGCCCAAGGTCACAGAGCTCGTAAGTGGCTG
TGTCAGGGGTCCCAAGGCCACCGCAGGCTCGACGATTTGCTGGGAAGCCTCAGGACGAAGCATATGGTTGTACACGTGGC
TATGATTTATTACGGTAAAAGGATTCAAAGCAAATCAGCAAAGGGAAAAGCCACACGGGGCCAAGTCCAGAGGAAAGCAG
GTGCCAGTTTCCAGAGCCCTCTCCCAGGGGAGTCACCCAATTCTTGCTGCAAGGAGTTGTGACAACCCACGTGACATGTC
ATCTGGCAGGGAAGCCCGCTAGAGAGACTGAGTGCCCAGGGTTTTTACTAGGGGCTGGTCATGTGGCCACCCTCTGCCTG
GCAGGTACTGAAATTCCAGACCCTTAGAAGGCAAGCTGGTGGTCAGTATAAACCACATTACTTGTACAGATTGCTCAGGC
```

**344**

```
ACAGTGAGCCATCCTTGTCAGTTAGGGAGCAATGGGAGGCCTCCCGAATCCAGGCTCCCAGACGCCAGCTCCGGGCCAGC
ATTGCCAGCAGCCTGTGTGGGGTGGAAGGGCTCGGGCCTGCCACGTGAGCACGTGCCTGCACAGCGGCCCAGGTGGGATT
CAAATCCAGGCAGCGACGCTTGAGTGTGCACTATTAACCACCTCGCCACACTGCCTTTTCTGCTAGACACCTGTTGTGGG
GGGGTCTGTGTGGGGCAAGTGGTGAGCCAGCCCACCCTGCTGCTCCATACTGAGTGTGAGTTGCTGTATTCATTTCCTTT
TGCTGCTGTCACAATTTTGCCACAAATGTCATGGCTCACTTTTAAAAACTATCTTGCAGTGTTGGGAGTCTGAAGTCTGA
AAGGAACACAACAAGGCTAAGATCAAGGTATCTGCAGGCCTTGTTCCTTCCAGAGGCTCCAGGGGAGAACCTGTGCCTTG
CCGTTTACAGCTTCTAGAAGCTGCCTACTTTCCTTAGCTCATGACCTTAATCCTAGCCCTCCCTGTTTTGTTGTTGTTGT
TGTTGTTGAGACACAGTCTCGCTCTGCTATCTAGACTGGAGTACAGTGGCATGATCTATAGTTCACTGCAGCCTTGG
CCTCCTGGGCTTAGGCGATCCTCCCACCTGAGCCTCCTGAGTAGCTGGGACTACAGGCATGTGCCACCATACCCTGCTAA
TTTTTGTGTTTTTTTTGTAGAGACAGGGGTCTCACTATGTTGCCCAGGCTGGTCTTGAACTCCAGGGTGCAAGTGATCCT
CCCACCTCAGCCTCCCAAAGTGCTGGGATTACAGGCGTGAACCACTGCACTTGGCCTCTCCCTCTTTAAAGCCAGCAGTG
TAGCACTGTCCAATTTCTCTCTGCCTCTAACCTCCTGCTTCCCTCTTTTTTTTTTTTTTTTTTTTGAGACGGAGTCT
CACTCTGTCGCCAGGCTGGAGTGCAATGGCATGATCTCCGCTCACTGCAACCTCTGACTCCCTGGTTCAAGTGATTCTCC
TGCCTCAGCCTCCCGAGTAGCTGGGATTACAGGCATGTGCCACCACGTCCGGCCAATTTTTATATTTTTAGTAGAGATGG
GGTTTCACCATGTTGCCCAGGATGGTCTCAATCTCCTGACCTTGTGATCCACCTGCCTCGGCCTCCCAAAGTGCTGGGAT
TACAGGCGTGAGCCACCACGCCCCAGCCCCTCTTTTTAAAAATTAATTTTTCTTTTTTTAGGGTTTTAAAATTTGTTTTT
GTTTTTTCGTTTTTCTTTCTCTTGCCTGATTGCTCTGGCTAGGACTTCCCGCTTCTCTCTTTAAGGACTTTTGTGATTCT
CTGGGGCCTACCTAGGTATTCCAGGATACTCTTCCCATCTCAGAACCTTGATGTAACCATATCTGCATGGACCCACCTAG
ATATTCCAGGACGCTCTCCCCATCTGAGAGCCTTGATGCAGTCATGTCTGCACGGTCCCCTTCGTCGTGGCAGGTCACAG
TTTCCCAGGTGTCGCATTGGTCTTCCGGTCTCCTTAGGCACCTTTTGGCTGTGACAGTCTCTCATCTCTCCTTCATTTTG
ATGACAGTGACACTTTTGAGAAGCACTGGTCAGGTATTTTGTAGGATGACCCTCTACTGGGATTGGTCTGGTGCTTTTCT
CATGATTAGACTGGGGTGATGGGTTTTGGGAGGAGGACCGCAGAGAGAAAGTTCCAGTTTCAGAGCATCTTGGGTACATT
CCGTCAACATACTGTATCACTGTTGATGTCGACCTTGATAGCCTGGATGAGGTCATGCCTGTGACGTTTCTGCAGTATCA
AGTTTACTTTTATTCTCCCTTTGGATTTTATTATCTTTTTAACTTGACGTGTAAGTTTTAACTTCACAGGTAATAAGAAC
ATATCTTCCTGAGGGGAAGAATATGGTTTCCTGTGACTCCCCGCCATCCCCAGCCGTACCCACCATCATCAATTTGCTGT
GCTTCCTTCCAGGCCTTTTCCTACTTATTTACATTCATATATATGTGGCCATAGAGAAGATACATATTGTCGTTTGTCTG
TTTGTCTTTGCTAGTCCAAGCAACACTGAACATCTTTGTGCCTCTGTGTGCATGTGTGAGCATCTCTAGGGCAGTTTCTG
AAGAGCGTAATTGCTGGGTCGTGAGGTATATTGGTGGAATTAGGTTTGGCAGTATAGATCAGTAAACTCAACAGTTGTGG
CTCAAATGGGACAAAGGTATATTGTTATTTTAAAAATCTTCTTTTAGAGGATGTCTGGAGGCAGACAGTCCAAGTATGGC
AAGAGACATTCATAGTCCAAGTTCCACTCATCTTTCTGTTCCATTTTCCCAACACATGGCTTCCATCCTCAAGGTCACCT
CTGGTCCCAGTGTCTGCAGAGCTCCAGCCCTTGCATCTGCGTTTCAGGCATCTGAAAGGGAGGAGGGAAAAAAGGTTCAC
CTCCCAGCTGTGAAAGCTTCTTTTTAAGCAGCCCTCCCGGCCATCCCACGTAACGCTTCTGCGAACATCTCATTAACCAG
AACTTGGTCACAGAGCCTCACTTGCCACAAGAGAGGCTGAAGTGGAGTCTTTAACTGGGTGCATTGCCATCCTGAGTAAA
ATTTGGGTCCTGTTTCTAAAAGGGACCAATGGATTTTGGCTGGGTCACTGGTGGTCTCTGCCCTGTAGGAGATGTGAGTT
TTTAGTTTAAAAAGATTATTATCAAGTTGTCAAGCTCTTTGCCAGGGGGAGATTCCTTCTGGCAGCACATCGAAACACTGG
TTCCCTCAGCCCATGCCAATCAAGTACCATGGACCCCTTATCCCTGGGGGTATATTCCAAGACCCCCAGTGGATGCCTGA
AGCTGGGGATAGTACTGAACCCTGTATAGCATATACTATGCTTCTTCCTATACACACACATCTATGATAAAGTTAATTTA
TAAATTAGGCATAGCCACAATTAACAATAACTAAGAATAGGGTAATTATAACAAATACTATAATAAAAGTTATGTGAACA
TGGTCTCTCCCTCAAAATATCTTATTGTACTGTACTGAGGGTAAGAAATGGCAGAAATCAAAACCATGGATAAGGAGGGA
TGACTGAGATTGCAGAATCAAATCCCTCCTGGCCTCAGAGCTTATGATCACAGGTTCAAAGCTGTGTGATGTCAAACAGC
CCTGTGGGAAAACATCTCACCTTGTCAACTAAAGAAAAAAAAAAAAACAAAACGCCTTTTAAAGATTTAAAGTTAGTCTTAT
TTACAAGTATTATTGAGGGCTGTAGACTGAGACCTTCAGCCTGGGGACAGTTTTGTCATATGGCTCCTAAAGTGTTTCAG
CTCATTGTTTATATTTGGTGGTGAGGGTTTAGTGTGTGCAAAATTATACTAAACCTGTTTAGATGTTGTATTCAAGCAGA
ATTAGATCAAGTTTGGGTGTAAGACTTTGTTTCACACAGCTATGTCTTGCTTATTTCCAGACAAACTGGGAAGATTGAGC
CTGTGGTCCTGCCGCTGCTCTGGTTTGCAGAGGTAAGGGTGCGTTGGGCACAGCGTCGGGGGCTTTTGTTAAGAGCCAAT
GTGGGCATTTGAGGCAGGAGGCGGGGGGAGCAGCTTGTAGAAAGGGAGAGGGCTGAGCCAGGGTAACCGGACTGTGACAT
GGACCAGCGTATCAGAAACTTCACCCTGTCCAAGCACCCTATGTCAGTTATCCCACCAAGGTGAAAGGGATCCCTAGAGA
TGGGGAAGACAGAAGCTGCATGAAGAGGTAAAGTCCCTGGCCCTGGGTGTAAAATAATTTTGTTGGGGCATATGACCTCT
TCTCCTGAAAGTGGGGAAGTTGGTTTCCAAGGTGTCAGCTTTTCTCTTAAACTTACTTCTCTCGCAGCTTTTCAGAATTC
CTTCTGGCCTAGCTTGTTTTATTTTATTATTTTATTTTATTTTATTTACTGTATTTATTGTTTCTTGCCCACCTCGTTTT
AAATCAGGGCTAGCTAATGGCCAGGACAAACAAGCCTCCGAATGTCAAGTTTACTTCCTACTCACAGCCGAATCTGAAGT
GGGTTGATGGGGAGGCAGAGCTCTGCTCCCTGCAGTCATTTAGGGTTCCAGGGCCCCGGTGGCCCCGCCATCCTGGAGAG
CCTCCATTCAGTGGCAGGGAACGAGATGGAGAGTCACGTGAGGCCAGGTCCACCTCCCATTGGTCAGGACACGATCATAT
GGCCCTGCCCAACTGCAAGGGAGTCGGGGAATTGTAGTCTCACCTTGTGTCCTGGAGGGGAGGAGGTCCCTGGCAGGCTCC
AACACATGCTTTAGCCGGGAAGCTTGAGGTGGGGAAAAGCTGAGGCGGGCACAGAGGAAGGTGTTGGGTGGCATCTGCGC
TGTAGCCCGCAGCGTGCGGCCCCAGCTCATGTGTTTGTCATTCTGTCTCCTCAGAGCGGGGCCATGGAGGGGGAGACTCT
TCACACATTCTACACTCAGCTGGTGTTGATGCCCAAGGTGATGCACTATGCCCAGTACGTCCTCCTGGCGCTGGGCTGCG
TCCTGCTGCTGGTCCCTGTCATCTGCCAAATCCGGAGCCAAGTAGGTGCTGGCCAGAGGGCAGCCCGGGCTGACAGCCAT
TCGCTTGCCTGCTGGGGGAAAGGGGCCTCAGATCGGACCCTCTGGCCAACCGCAGCCTGGACCCCACCTCCAGCAGCAGT
CCTGCGTCTCTGCCGGAGTGGGAGCGGTCACTGCTGGGGGCTGCGCAGCACGCTTGCGTCTTTTGCATGCCGCGTTGCCA
CTACTCTGCCTGTTCTGGAAGGCCTGGGACCCTCCCTTGGAGGGGGCACAGGTGGGCTTTGAGTAATGAGACCTGGTACT
```

```
TGCATCATCCATTCATCAAGTCAGCACCCGGGGATGCCAGGTTCTGTTAGGGGCGAGGGGACGTACAGCAGTAGAGGAGA
CAGCTGAGATCCCTGCTCAGGGGGATTGAGGGGGGCTGGCATCCCAGCCGGGGAGACAGATGAAAACCAAGTAAATCAGC
AGAAAAGATAATTTCACTCATGATAGGAGCTGTGAGGGGTTAGAGCCAAATAGAAATACAGCGTGAGCCACGTGTGAGGT
TTTCAGTTTAAATTTTCTAATAGCCACTTAACAGTCAAAGGAAACAGGTGGAATTAATTTTAATCTTATTTAACCCAAAT
ATATGCAAAGTATTATCACTTCAACATGTAATCAGTATAAACGGCATCAATATTTTTGCAGTGTTTTTGCATGAAGTCTT
TGAAATCCTGTGTGTACGATACATGTGCAGCAGGTCTCATTTTGGACTGGCCCCGTTTCAAGGGCTCACCAACTGAATGC
AGCTTCCAGACTGGAGAGTGCATGTCTGGAGCAAGTGGGGACAAGGACAGATGGAGCTTCAGGAAGGCCTCTCTGAAGAG
GTAGATAGTGAGCTTTGACATGGAGGCCAGGGAGGCACTCAGCACACAGCCACATGAGGAATGCTGCCTGGAGCGGCCAC
TGCAAAGGCCCTGTGGCAGGGACAGGCAAGGCACATTGGACGGTCAGGTAGGGCCAGGGTGGCTGGAGGGGAGTGAGGGC
AGGGACAGAGGTAGGAGGTGGTGTCTGAGAGTGGACAGGGCGGGCCACCCAGGGCCTCGTGGTGCACAGTGAGCAGTTTG
GAACTGATTCTGGGAGTGACAGAGCCATTGGAGGCTTTTAAAGAGGGGAATCACTAGGTCAGCTGCTGGCTGGGGAATGG
GCCCCGGGCTGGGAGGGGTGGAAGCCTGGGCCCAGCGGGGAGGCCCCAGTGCGTGAGGAAGTCGATGGCTTGAACAGGAT
GGGGCAGCTGCTATCAGGAGGGAACGGAAGTGGGGAAGAGCTCCAGCCCTGGGCTTCAGACCTCCAGAAGCAGCAGGAAG
AGGGGTGATAACAGTGTCCCTCCTTGCTTCGGGGTCATTTATTTATTCATTCATGCATTCACTGCAAGGCCCTTTGCACA
GGTCAGGAGATGCAGAAATGACCAGAACATGAGATCTGCAGCAAAGATGGGCAAGGCAGGCCTTTAGGCAAGTTACTACA
GTCACACGAAGGACAGGAGAGCTGTGATCACAGAGCTCAAGGGGCAGTGGAGATGGGCGAGTGGCTGCCATGGCAGGGGA
AGGGATGCTGCCCACTGGCAGGTCATCAGGAAAGGCTGTTTTTGGATGCATGGACCAGAAAGCCACTCTCTGAGGTGGCC
CCCACCTGCATCATCCTAGGGCCACAGGCCGAGGCCTTCTGAGGGGCCTTGGGGCTGCCCCGCTGCTCTGCTGGTCTTGG
CTCCTCTGCAGCACCTGCTTCCTTTAGCACTGGTGAATGATTCTTCCTCTTCCCCTGTCTCAGGGAAGGCTTGCCTGCTT
CCATGCTTGCATGCTGGGTGACAATAAAGCATTCCTGGTTCTTCACCTTGGCTTTCAGACCTGAAACCCGACTTCCTTTC
AAAATTCCTGTCCTCGGGGGGAAAAGGTTTCCACAATGTGAGATGGTAGCCTGGACACCAGCTGTCATCATGTTGCTGCT
CTAGCGTTTGAGCTCCCGATGAACAATGACCATTGGGTCCAACCAGCCCTTTTGGGAGCCCCCTTCTCTGTTACTGCCCC
AGGGGGAGCCAGAACCCAGCAGGGCTGTGACTCCAGCGAGCAAAATGGAACTCCCTCCAGTTTCTAGACTGAATTATGTA
GGGAGGACAGAAGGACTCAGAACCCACACTCCCAGACCATGAATACCTTCCCGTGGCAGAGCATCACCATAGGTGTCCAC
AAGTGACGACGCACAGCCACATGAGGGGGTCCCTGGGGACTGGCTGAGGGACTGGGCCAGAGGAGGCCATGACAGCCTCA
TCCCTCTGTCTCCTTCTGTCTGTCTCCAAATTGTCCACCAGAATGGGGACAGGGAAGGAGGGTCAGGAAGCACAGAAGGT
CTAGGCTGTGGCTAGGGTCCCTTTCCCTGCCGCAGCCCCCAAATCAGCATCCCACCCTCAAATCCAGTAAGAATGCTACG
ATCGGCAGTGTGGCTCCCTCCCTGCAGGTTTCATTGGAGGCCACGTAAGTGAAATTTATCCCATTATGTGTGTTTTGATG
TAGCAGGACCTTACCAGTTTTTTTTGTTTTTTTATTTTTATTTTTTTGGTTTCTTTTTTGAGATGGAGTCTTGCTGTGT
TGCCCAGGCTGGAGTGCAGTGGCACAATCTTGGCTCACTGCAACCTCCACCTCCCGGGTTCAAGCGGGAGCCTCAGCCTC
AGCCTCAGCCTCAGCCTCCCGAGCAGCTGGGATTACAGGCACCTGCCACCATGCCTGGCTAATTTTTGTATATTGAGTAG
AGGCGGGGTTTCACCATGTTGGCCAGGCTGGTCTTGAACTCCTGACCTCATGATCTGCCGCCTTGGCCTCCCAAAGTTCT
GGGATTACAGGTGTGAGCCACTGTGCCTGGCCGGCCTTATCAGTTTTTATGTAATTTGTCCTGATTTAAAAAAAAAAAAT
CACATGCGGGTAAACTCAACATATTTTAATTTCTCTTTAGAGAAATTTTCCACTTGAGCCAGGTATGGTGGCTCTTGCCT
GTAATCTGAGCTACTCGGGAGGTTGAGGTGGGAGGATCACTTGAGCCCAGGAGTTGGAGGCTGCAGTGAGCCATGATCGC
ACCATTGCACTCCAGCCTGGACAACAGAGTGAGATCCGTCTCAAAAATTAAAAAAAAGCAGTAATTTTTCTTCTCAAGT
CATTCTTTTAAAATAAATCAAAAGTATTTTAACAGAACAATCTGTTCCTCGTTGATTTTAAACAGCCCCCCAGCTAAGTC
CACTGGTTGTCGGTCTCCATGGCCCATCTCCCCACCTTGCTTCCTCGCTGGCTTAAATTTTCAAGTGGACGAGCCCTTTT
CAGCTTGGCTTGTCCACCTCCCAGGTGTGTGTTCCTGAAGATGCTTGTACTTATGTCTAAGAGCGGCAGCTCCCCACATCT
CAGCCACCTGCAATCGTTGAGGGTTGTTGGACTCTAAACTTATGTGCCTTTCCTGTTTCCTCTTTGCCTTTTGCAAATTG
AAGAACCGTGTAAAACCATTTTTATGTGGCTTCAACGTCAACTATAAATTAGCTTGGTTATCTTCTAGGAGAAATGCTAT
TTATTTTGGAGTAGTAGTAAAAAGGGCTCAAAGGATAAGGAGGCCATTCAGGCCTATTCTGAATCCCTGATGACATCAGC
TCCCAAGGGCTCTGTGCTGCAGGAAGCAAAACTGTAGGTGGGGTACCAGGTAATGCCGTGCGCCTCCCGCCCCCTCCCAT
ATCAAGTAGAATGCTGGCGGCTTAAAACATTTGGGGTCCTGCTCATTCCTTCAGCCTCAACTTCACCTGGAGTGTCTACA
GACTGAAGATGCATATTTGTGTATTTTGCTTTTGGAGAAACTGCCCTTCCTATGTTCTGAGTGAATAGCAGTTTTTTTTGA
TCCCAGAGGGCAACTTGTATTCTGTGGGCTGGTGCCTATTTGCAAGGTCACATTAGAAAGACAGGAGCAAGGCTTAGGCC
TGCTGCTTCTGGAATCTCTTGTGCAATAGACTCCCCTGGGGAAGTCTGCTAGAAATGCAGATCCCTGGGCAGCCTTCTAG
CCTTCTGGAATAGGATCATGGTGGGACCCTGGAATCTGTATTTTAATGGACCCTAATGAGATTCTGATGTAGAAACATGT
TTGAGAGGCATTGATCTAAATCTGGGATGACTGGCCAGGTGCGGTGGCTCACACCTGTAATCCAGCACTTTGGGAGGCCG
AAGCAGGTGGATCATTTGAGGTCAGGAGTTCAAGACCAGCCTGGCCAACATGGTGAAAACCTGTCTCTACTAAAAATACA
AAAAAAATGAGCTGGGTGTGGTGGCACACGCTTGTAATCCCAGCTACTCAGGAGGCTGAGGCAGGAGAATCGCTTGAACC
CGGGAGATGGAGGTTGCAGTGAGCCAAGATCGTGCCACTGCACTCCAGCCTGGGTGACAGAGTGAGACTCCGTCTCAAAA
AAATAAAACTAAATAAATAAATAAATAAATACATCTGGGATGACTGACCAAGAACAAAGAATGTAGGCATCAACTGAAC
ACCAACTGTATACCTGGGACTGGATCTGAGGGTAGGATTGCCAGATTGAGCACAAACAAACAAACGAAACACAAGAAACA
ACAGAAGGGTGCCTGTTAAATATGAATTTCTGGTAAGCAATAAGTAATTTTTATTGTGTTCCTGTGCAATGATAGGACGC
ACGTATCCTGAAAACCTGTCTGTAGTTCACCTGAAATTCACATTTAACTAGGCGTTCTGATTTTATGTGGCCGCCCTATC
TGCTGGGAACATAGGCTGATGCCCCTGGGGGTTCTGCGTTTCCTTGGCCAGGTTCCTGTAGGGCTGAGGTCATGGGGGAG
CCGTGGCCAGGGATGGTGTCCTTGCCAGGGGAGGTGACGGGCAGAGCTGCAGGCCCTACAACTTGGGGTTGGGCAGGGAT
GAGTCGCTCTACGGTGGTGCCTCTGATAATAGCTGTTATTTTAGTTTCTGAAAAATGTGCTGCCCTCAAGAGCTTCTGAG
GCTAGGCCTACTTGGGTGAAAATAGCTTTCCAGGGAGCCACATTGTGGGGACGTGAGGCAGCAGCCCCACTCTTTGGGGC
CACTTGGCTGTCTCTAACTCATCTCACTTGCCAGGGCTGGACAAGGAGATGAAAAGAAAATGCCAAGGGGTGGGCAGGC
```

```
AGGAGCCTGTTGGCGTTGGCAGGCGCTGCCAGCCCTGTGTCCCAGTCAAGCTCTGGGGCGCCCTTCCTGTCCATCTGGGA
GTAGGGGGTGGAGTCTGACCATGTTGGGGCGGAGTGTTAATATTAGGAGAGGATCCTCCCTGGGACAGGGTCCCCAGGCT
TGGGGGCAAGGCCTTCTGCCTGGACCCCCTTCCTTTCCTCTCTCCCAGGATTCCTCCAGGACACCGTAGTGCTGTGCACG
GTTTGGCCCCATCGGTGTGCCTGCTCCTTCCAGCCACTGCTCCCTTCTGCAGAAACTTGCCTGGGATTCCCAGGGTGCAG
GGGTGGGGGCTGGTCAGGGGCTGGTCAGGGCCTGGTCAGTGTAGAGCAGGGAGCTTTTCACTTTTAGCATAGATAGAGTG
GCCAGGGCCCCATGGCTGCACCTCCCTGGACATAACCAGGATGTCCTGTCATCTGCCCCCCAAACAAAACTGGGGTCTCC
CCTTTATTTTGCCAACAGAATCCAATTTTTAGGTGATGAGACAAGTGTTTGACTTGAAAAGACCCTCCTGCCCTGCCTCC
TGGAAGGTCTGGAGCCCAGCCCAGCGCCCCGGATCCCCTAGGCATGTGAAACGGGGAATGCAGTGTCCTCCAGTGGCTGG
CTGCAGCTTGGGGTGGGCACCTGGGTGGTGGCTGGTGGTCCCCTGCAGGCCACTGCTGCCCCCTCTTGGGTCCTGTTCT
GAGTCTCAGGCCTGAGTCCCTGGGAAGGCCCCACACCTTTGCCCTGTACCTGGGTATGGGGATGGCCCTGAGGGGCTGC
CTTGCTTGGGAGAAGGGGGGAAAACAGATATTTTTATATAAATAAGAAAAAGACAGCCCCTCTGGGCAGATGAGGCCTGC
TGTAGGGCAGGGGGCTTGGTAAACTGTGTGTCCACTGGTCTGTAACCCTTCAGCTGGGTCCTTTTGTGCAGTGAACAGCC
TGGACAACTGTCCAGGTGGCCCTTCAGGGATATGTTCCTGGACCTTTCTTAGCTTCTCACCGATCTTTCTTCCACGCCAA
GTCCATGTTCCTCAGTCAAACAAGTGGCAAAGAGGAGGAGGAGGAGGGGAAGGAGGAGGAACCTGCTTCACCCTCTGGCA
CCTGCGGGTTTTGTTCCACGTGTCTTCCTTGGGAAAGACAAGGGGTTCTTTTACAGCCTAGGGGTGGTTTCCCCTTTCTA
CTTCTGAGTGAGACCTTCTCAAGTCACGGCTCTTTGGGCCCACATGGCTAAGGTCTAGCCACCTGTGGGTGTCCTGTGCT
GCCTCAGTGGGTGGTGTGGGGGCGGGGCTGACCTCCCCCGCGCCTGGCTCAGTGCAGCAGACCCTGGCCCCTGTGCCCGC
TCTCTGCCCTGAGCAGGAGTTTGATTTTCCTACCCCGTGGTGAAGCAAGCACAGGTGGGCGGAGGTGGGCAGCGGTTTCC
AGCCCCAGCCACCGGGGCAGGGAGGCCTCTGTTGCTCAGGGCAAGGGAGGCAGGGGGCTTAGTGGGACGAAGGCTCACCC
CACACTCTTATCATTAGCATTATCTGTATTTTTAATTTTTAAGTTGAGAAATTTTCTACAACTGTCAAACTAGACAAATG
ACAGTATCCAGTTCCCCTCACCCAGACGCAGCCATTGCTGACAGCTGTCAGTGTTGAAGATGGCCAGCATCTCATGCCCT
TGGTTTCACTCGTTCCCTGGGTGGCCCTCGTCCCCCTCCCCACGTCCCGCGCCCCGCATACCCTGAGCAGCCATGGCCAG
GAACTTGGCACGTGGGTTTGTTTTTTCTGAATCTGCATGTGCGCAGCCATGAGCATGAGGTCACTTTTTGAATGTGACCG
AAGCTGCATCCTGCTGCGTGTATTGCTGTGTGGCTGGCTTTCCTCCCTGTCTTCTGTTCAGGAATACTGTTCAGGCTGAA
TACCGTCCTGTGACATGGGTTTAAAAACACAGGCCAGGCATGGTGTCTCACGCCTGGAATCCCAGCGCTTTGGGAGGCTG
AGGTGAGAGGATCGCCTGAGCCCAGGAGTTCGAGACCAGCCTGGGCAACATAGACCTGATCTCTACAAAAAATTAAAAAA
TGAGCCGGGCGTGGTGGTGGCACGTGTCATTGTCCCGGCCACTTGGGAGGCCGAGGTGGGAGGATCACTTGAGCCCAGGA
GTTCAAGGCCGTGGTGAGCCATGATGGCACCACCGTACTCCAGCCTGGGCGACAGAGAGAGACCGTCTCTAAAAAATAAA
AAATAAATCAACACATAAACATACGTTCTCCTCCTGAGGGACAGAGCTTGCCCCGGTTGTTCCATGTTGACTGTGCTGCA
CACAGGCCGCCCCCCTTGCTCTGCCCCTCACTGTATTAAGTCTCTTTGGGCCTCAGTTTCTCCATCTCTAAAGGAGGTCG
GGGAGGCCAGGCGGGTGTTGAGTGAGATGAAGAGGCCCTTGGAGTCATTCCAGGAGTCTATAGGTGACTCCAGTCTCTTC
TCACTTCTGACAGCCTGGTGGCGGGGGTGGAGTCTCTGCTGGGGATGTGGGTACAGGGCTGGGGTGGGCGGGGATGAGGG
TGGGGTTTGGTGACAGGGCGTGGGTGGTCACCCAGCTTTGCCTTTCTCCACAGGGTCCTGAGGACACCGTGAGCCAGCCA
GGCCTGGCCGCTGGGCCTGACCGGCCCCCCAGCCCCTACACCCCGCTTCTCCCGGACTCTCCCAGCGGACAGCCCCCCAG
CCCCACAGCCTGAGCCTCCCAGCTGCCATGTGCCTGTTGCACACCTGCACACGCCCTGGCACACATACACACATGCGT
GCAGGCTTGTGCAGACACTCAGGGATGGAGCTGCTGCTGAAGGGACTTGTAGGGAGAGGCTCGTCAACAAGCACTGTTCT
GGAACCTTCTCTCCACGTGGCCCACAGGCCTGACCACAGGGGCTGTGGGTCCTGCGTCCCCTTCCTCGGGTGAGCCTGGC
CTGTCCCGTTCAGCCGTTGGGCCCAGGCTTCCTCCCCTCCAAGGTGAAACACTGCAGTCCCGGTGTGGTGGCTCCCCATG
CAGGACGGGCCAGGCTGGGAGTGCCGCCTTCCTGTGCCAAATTCAGTGGGGACTCAGTGCCCAGGCCCTGGCCACGAGCT
TTGGCCTTGGTCTACCTGCCAGGCCAGGCAAAGCGCCTTTACACAGGCCTCGGAAAACAATGGAGTGAGCACAAGATGCC
CTGTGCAGCTGCCCGAGGGTCTCCGCCCACCCCGGCCGGACTTTGATCCCCCCGAAGTCTTCACAGGCACTGCATCGGGT
TGTCTGGCGCCCTTTTCCTCCAGCCTAAACTGACATCATCCTATGGACTGAGCCGGCCACTCTCTGGCCGAAGTGGCCGC
AGGCTGTGCCCCCGAGCTGCCCCCACCCCCCTCACAGGGTCCCTCAGATTATAGGTGCCCAGGCTGAGGTGAAGAGGCCTG
GGGGCCCTGCCTTCCGGGCGCTCCTGGACCCTGGGGCAAACCTGTGACCCTTTTCTACTGGAATAGAAATGAGTTTTATC
ATCTTTGAAAAATAATTCACTCTTGAAGTAATAAACGTTTAAAAAAATGGGATGCCTGCCTCTGTGACAGCCTTGTTTGC
TGAGGTCGTGGGGGTGGGGGCCTCTGGGAAGTTCCGGGCTCCTCTTCTCTTGGTCAATAGCTCCTTTCTGGTGGCTGCCA
AGAGCGTCTCTCCCAGGGCCGGGCTGCTGGCTTACCTTCCTGTTGTTTTCAAATTTCAACCTTGTGCAATGTTGAGTTTC
ATAGAAATACTGCATGAGTACGCCCTTGTTTAGAAGCAGCAGGGTCTGAGTCCCATCCCACAGCCCCCAGTGCAGACGCT
TTTGCCACTTTTGCATGGGGCCCCCTGGATGTGTTTCTGTGCATTTATCTACAAATCCTGGTGCCCGTAGGACATGCCCC
GTGTTGTTCTAGACCTTTGCTTTCTGCTTGTTACATAAATGGTGAAGAAGAGAAGCGGGTAAGAGAACAGATTGGAGCCA
TCTAAAAGTTCTCATCTTAAGTGTAGATCATTGCAAAGGATGGAATTTTCTCCGATTGTCATCATCGTTGATGTTTGAAT
ATGAGACCATTTCATCAGTATTTAAGTGTGCCCGCTGGGATCTAACTAGGAGAGGAGAGGGTATTCACTGCCAAACATTT
CAAAGAATATACGAACAAGCTCTTTTATGGTCAGAAATGTTAGCCCTTTTCCCCCCTTAAACTTGTATTTCCTTTCTTCT
TCCCTCCTAAAATTTTATCTAAATGATATTTATATTTGATGCTTTTAAGCCTTTTATTAATCACTCTTATACTTGCCTGC
AACATAAATATATAAGTTGAAGCAAACTTTTTTTTTTTTATTTCTTGTGACCATCAAGGGTAAATTTTTTTAGGTCTGGGG
CTGCCATAACAAAATACCATCAAGGCCGGGCGTGGTGGCACACGCCTGTAATCCCAGCCCTGTGGGAGGCCAAGACCGGA
GGATTGCTTGATGCCAGGAGTTCAAGACTAGTCTGGGTAATATGGTGAGACCCTCATCTCTACAAAAAAATACAAAAAT
TAGCCAGGCGTGGTGGTGGGCGCCTGTGGTCCCAACTACTCAGGAGGCTGAGGTAGGATTGCTGGAAGCCAAGAGGTTGA
GGCTGCAGTGAGCTGTGATCATGCCACTGCACTCCAGCCTGCGAGACAGAGTGAGACACTGTCTCAAAAAGAAAAAATAC
CATCAATAGGTGGCTTAAACCATTTATTTCTCACAGCTCTGGAGGCTGGAAGTCTGAGACCAGGGTTCCAGCAGGGTTGG
GTTCTGGTGAGGCGTCTCTTCCTGGCTTCAGACGGCCGCCTTCTCTGTGTCCTCACATGGCCTTTCCTCGGTGTGAAAGG
```

```
GGGAAGAGAGAGGGAGAGCGAGAGCTCTGTGGTGTCTCTTATAAGGACACTGATGCTATCAGATCAGGGCCTCATTTAAT
TATTTTCTTAGAGGCCCCATCTCCAGACACAGCCACACTGGAGGTTAGGGCTTCAACAGGAATTTTGGGTCAACACAAAC
ATTTAGCCTATACCATCTGGATTGCCTTTCAAGTCTCAGGACACAATTGGTCAAAACTATATCACTATGTTTTATGTGAC
ATATCAAAGTAGCAAAATTTTATTGGAATGCATGTCCTTTTGGTATGCATTTTATATATCTTTTTCATTTACAGATTAGA
GTTGTGTGTGAGTGTGTGTACAATTCCATGAATTTTGACATGTTTATAGAGTTGAGTCATTACCCCTACAATCAAGACAA
AACAATTTCATCATCCCCCAAGATTTCCTCATGTCTTCCCTTTGTTTTTGTTTGTTTTACAGTGGGCTCCCATAATTTTT
TTTTTTTTTTTTTTTGAGACAGAGTCTCGCTCTGTTGCCCAGGCTAGAGTGCAGTGGTTCAATCTTGACTCACTGCAGCCT
TCACCTCCCAGGCTCAGGAGAGCCTCCTGACTTAGCCTTCTGAGTAGCTGGGACCACAGATGTGTGCCACCACACCTGGC
TAATTTTTTTTTTTGTAGAGATGGGGGTCTGGCTCTGTTGCCCAGGCTGGTCTCAAACTTTTGGCTTCAAGCATTAGTCCC
ACCTCAACCTACCAAAGTGCTGGGATTAGAGGCGTGAGCCACTGCACCCAGCCTGCGACCTGTGCTGCCTTCCTCCCACT
TTGTAGGGAAGTCCCTCATCCCCATGCTTAGCCCTTGGCCACTAGCTATTTTTACCTTTTCCAGAATGTTCTAAATGGGA
TCTACAGCCTGTAACCCTCTGAGCCTGGCTTCCTTCACTCAGCACTGTCTCTAGATGCATGCCTGTTGTTGTGTGTAGCA
ATAGTTCCTTTCTTTTCACCGCTGAGCAGAACTCCATGGGGTGCACACACCAGTGCTTTTCAGCCGTTCACTTATTGAGG
GACACGTAGAGTGGTTGCAGCCTTTGGCAATTGCATTAGTTGGTTTTCACGCCACTGATAAAGACATACCCAAGACTGGG
TAACTTTTTAAAGAAAAAAGGTTTCATGCAACTCACAGTTCATATGGCTGGGGAGGCCTCACAATCATGGTGGAAGGCAAAG
GAGAAGCAAAGTCATGTCTTACATGGGACCAGAGAAGAGAGAATGAGAGCCAAGTGAAAGGGGAAACCCCGATCAAACC
ATCAGATCTTGTGAGACTTACTCACCACCAGGAGAACAGTATGGGGTTAACCGCCCGTATGATTCAATGATCTCCCACTG
GCTCCCTCCCACAACATGGGAATTATGGGAACTACAATTCGAGATGAGATTTGGGCAGGGACGCAGCCAAACCACATCAG
CAACTATGAACAAAGCTGCTGTGAACGTTCATGTACAGATTTGTGTGTGAAGGCGTACGTTCATTTCCCTGGGGTGTATA
CCTCGGAGTGGAGCTGCTGAGTCAAACTGGAGCTCTGCGTTTCACTTTTGGGGGACTGCCAGTTTCACAGTCGCTGTACC
AGGCTGCATTCCCATCAGCTCTGCGTCCTCACCAGCATCTTGGTGTTTTCAGTTTTAAAAAACTTCAGCCCTTCGTAGTG
GTTTCTCATTGTGGCTTTTATTTGCTTAGAAATACTTCTTGTCCTGAGATGAGTGGGACTTCCTCTCCACCCCCAATTAA
TTCATGTATCCGTGAATGACTGTCACGTATTGCTAGAATGAAACCAATTTTTACCATCAATTCTTTGTCCCTGCAAGTGC
AGAGAAGCCTTGTTTTCATAAGGAAGTCACTGGGAGTGGAATTCTTATACTCCTTCTGGGTAGTATGCGAATGTCACACG
GCGATCTGTCATTGGAAGCGATTCTCACTGATCTCCCAGCAGACAGGTCCGTTTGACCTTTCTTCAAATTTGTAGAAAGC
AGCAAAGTGGAAACCACCGGCTTGCAAAAGCATTTCTTACCCAATCTTTAGATGGGTTCTCGAAACCTGTGTCCGGGAAG
TTCCCTCTGATTGGGGCTGGAGGTCAGGGAGGCTTTGCCCGTTGGCTGTAGAGTGGAGGGGTGGGGGGTCTTGTGACCTG
CCCGGGGAAATGGCAGGCGAGTGACAAGCTCAGGAGAGAGTCCAGGCCCCGAGGATGGTTCCCTTCAAACTCCCCATGTC
TGATCCTCTGGGAAGCTGAGGCCAGCTTTGGTTATTTGATAGCCACGGTGAGGATTTTGACTTAATCTGTGTATGCAACA
CATCACAGACAGTTTTAACTCTTGCATTCAAGTCTACCTGCTCATAAAACAGAAAAAAGACAGGCCCCAGAAGGCATTC
GGTTTTTTTTTTTTTTTTTTTTTTTTTGGAGACAGGGTCTCACTTTGTCACCTAGGCTGGCACAATCACTGCTCACTG
CAGCCTCAACCTCCCAGGCTCAGGCGATCCTCTCACCTCAGCCAGCCTCCCAAGTAGGTGGGATTATAGGCCTGTGCCAC
TACACCCGGCTAATTTTTTGTATATTTTTGTAAAGATGGGGTTTCGCCGTGTCGCCCAGGCTGATCTCCAACTCCCAGGC
TCAAGCGATCCGCCCGCCTCGGCCTCCCAAAGTGCTGGGATAACAGGCATGAGCCACTGCCCTGGCCCTTAAGTCATTCT
TGTGTTTGTGTGTGTGTGGGGTGTGTGTGTGTGTGTGTGTGTGTGTGTGTGTGTGTGTTCCCGAGGGACTTAGCCTCCCG
TGTGTCTCATTGGGTGAACATCTTGCTTCTGAGTGAGGTTTGATTTTTTTGTAGGGTGTGCGTTTGGACACATCCTTGGT
CTTAAGCCCAGCCAACGACCTTAGCCAGGGCTCAGGGAGGGAAGCACCGCCTGCCCCACAGCTGGAGGGCTGGGGTTCGG
GACACCCGGTCGAGCTTCAGTGTCTCTGATTCTGAGCATCTTTGACTCTGCGAGACTTTCTTCTCATGCCTTCACTTCAG
GCGCCAGCCCCTGCCAACGATGGGACTCCACTACTTACCATGTTGATTTAATTTGGCTTACTTTTTTTCTTAAAGAATTT
TATTTTAAAAGGAAGGCAGTCATATCTGTGAAATTATGGGTCCTTTGGGCAAGTAATAGTTTTCCAGTCGCCATTAAAAT
AAATACCTAACTATTAAGGACGTTCCTCCCAGTACCTCGAAGCAGCCTGCTACCTGCAATCTCTGCACCATCCCATGATT
GTGTCCCATGCACCCTTCTTCCTGCTCGGCCTGCATCGCCCCTGCGTGGCCTTTTCCCCCCTCTCTGCCTCTCTCAGTCT
TCGCCATCATCAAAGGCGCAATTCCAGCCATGCCCTCTCCTCCAGGAAGCACCCCTCCCCCATCACTCCTGCTTGCTGTT
CTTCCCTGTCTCAGGACTCCTGGGGCACTTTGTATCTCAGTTGATACTAGAATGTTCTAGAATACCAGAAGTCCTGACAC
CAGTTGCTGTAACCCCAAACTCCAAGGACCTGCAGCATAAACCCCGGTACATGCCCTTGTGCTGGAAACGAATATTTTTG
GCCGGGGCGGTTACTCACATTCCTTAGTACTCCTTGGCTCACGCGGACACCCATTCTTTCAACACATACTTATCCAGTGC
TCACTACGCGCTAGGCACAGGGGGTGCAATTGTGGACCAGACAGAGGCGGCCCTTCCTCTCAAACAACTTTACAGTTCTG
CCACTTACTAGCTGTGGGACCCTGGACAAGTCACACAGCCTCTCTGGAGACCTACTGTTCTCTCCTTTAAAGCAGGTGAC
TCAGATCTGCTTTGCAGGGACGTCTCCTGCTTTGCAGCGATGTCACCCAGGAGAGATGGGTGAAGTGGATAGTGAATGGA
GTGGGCCAGGGCGGTGGTCCTCGGCTGAAAACAATGCCTCTCGGGGTCAGCGTGGGGCTCAAGAGGTGGCGGCCCCGTGG
GTGGGTCCAGGGCCAGGGACCCTGTGTCCGAGCCCCTGCCCTGGAGCCATCCTGTCTCACCTGAAGGCTCTGCCAGTCTG
GTCCACTGGCCTCCCGGCTCCCACGCGTGTCCCGAGCCGTCCATTCTCCAGGTAGCGGCCCTGAAATGTTAATCTGTCAC
AAGGGTCATCTTTGCTTGAATTGTGGGTGACTTTTCCCCATCCTGGGGGTAAAGTCCCCCCGACTTGTCTCTCCATCCGC
GATCCCCTGCAATCTGGCTGCAGCACGCGAGACTTCAGGACGATTCCGAGCTCGGCCACTGGGGGGCGAGCTGTGCACAC
AGGTGGTCCAGAGGCTGCTGCCTGGGAGGGGCTGCGGATCACCCGGTTCCTGGAGCGGAGTGGATTGATTTTAAACTGGG
GAAGCTGTGGTCCACAGCCTGGCTTCGGAAACCCGTGACCCCTAGAAATTGTATTTTACGTGTGTGTCTGGGCGTTTTAT
TCTGGGAAGCATTTCCTCAAGTCTTCAGATTTTCAAAGGGGTCTTAACCCTTTCTCTCTGGGGCCTTGGGAACAAGAGTG
ACAACCCTATAACAGGGCGACCAGGGTCACACGATGTCTGCTCCTCCTTAGCGTGCTACTCCCAGTACCTGCGTGCTACT
CCCAGTACCTGCGTGCTACTCCCAGTACCTGCGTGCTACTCCCTGCTCTGCTCAAGTTGCTGGGTGGCCTTGGAGCAATT
CATAACCTCTCTGAGACTGCCGTGAGGTGGGAGTGAAGATTGCTGTGTACGAATCTTTGAGAACCCCTGTAAGGGTGGAA
TCGAAATGGACGTGTGAGGGCTGGGCGCGGGGGCTCACGCCTATAATCCCAGCACTGTGGGAGGCCAGGGGTTCGAGACC
```

```
AGCCTGGGCAGTATGGTGAAACACCGTCTCTACTGAAAATACAAAAATTAGCTGGGTGTAGTGGCGGGCACCTGTAATCC
CAGCTAGACGGGAGGATGAGGCACGAGAATCGTTTGAACCCAGGAGGTGGAGGTTGTAGTGAGCCGAGATCATCCCACTG
CACTCCAGCCTGGGTGACAGAGTGAGACTCCATCTCAAAGAAAAGAAAAGAAAAGAAAAGAAAAGAAAAGAAAAGAAAAG
AAAAGAAATGGATGTGTGAGCATTCTGTACACAGCCTGGGACGATTATGAAAGCTCTCAAATGCTGAGCCTCCAGCAAAC
ATTTATCACACACCTACTGTGTGCAGAGCACTTGCTGGGCTCTGAGAGGGAGTCAAGCTCTGAGAAGTCTGTGTCGATTT
GTTCATTCCTTCATTCAGCAAATGCTGTTTGGGTGCTCACTGTGTGTCACACACCCTCATATTTATTGCGAATCGGCTTG
GGTTAGGTTCCCCAGAAGCAGACACTGAGACCAAGATTCCTGGCCAGGGAGTGTCCCAGGAGGGCAAAGGGGGACTCTAGC
AGCCCAGGGTCACGGACAGCTCTCAAAGGCAGACCAACAGGTCTTGGGTTTTGGATACAAAAATGCTCCAAAATCAGGGG
TGTGCAAATACAGTAAAAAGAGGATTCATTTGGATCTGCTTAGAACACCAAGGGTATCCGCTTCAGAAATACAGAGGTGA
CCGGGTGCAGTGGCTCACCCTTGGGAGGCCGAGGTGGGCGGATTGCTTGAGGTCAGGAGTTTGAGACCAGCCTGGGCAAC
ATAGTGAAACCCCATCTCTACTAAAAATACAAAAATTAGCCAGGTGTGGTGGCACACGCCTGTAATCCCAGCTGTGGGAG
GCTGAGCCACAAGAATCGGTTGAACCTGGAAGCAGAGGTTGCAGTGAGCCGAGATCTCACCACTGCACTCTAGCCTGGGC
GACAGAGTGAGACTCCATCTCAAAAAAAAAAAAAAAAAAGAAGAGAAAAGAAAAGAAAAACAACAACAACAAAGCTGG
ACGAAGCTAGAAAGCTCCCTGCCCTCACAAACTTGCCTTCTAGTGGGAGAGACAGCTGGCGAGTAAATGCATGAGTAAGG
TCACGTATGAGTAAGGTCACTCATAAGTAAGGTCACACATGAGTAAGGTCACTCTGGGAAGCAACGATGCCAGGATAGAA
AGAAGAGCAGGGGGTGCACCGGAGAAGAGGTGCTGGGCAGGGGGGCTGCTTAGATGAGAGCACATTTCCGGAAGTTTCCT
CTGAGAAGTGCCAACTCCTGAAGGCTTCATTGGGAATCTGGAGAAGAGCACTCCAGGAACCGGGAAGTACATGTGCAAAGG
GCCTGGGGTAGCAACACGCAGAACAGAAACGGTGCGCTGTGGCTGGAGCCCAGAGAGTGGGACTGAGTGGGAGAAACGCA
AACTGCTTTTCCTCCTCTGTTCACCCCACAACAATCAACACAGAAGACTTCTGTGACCAAATATGGGGGGCTCCCCCCAC
AGCAGATCAAATGAACACCTAATTCTGCAGCGGATGCCAGCTGGGGGCCCTCTGATTCAACTCAATTCTGCCGCTGTCTA
CCTGGAGGCAGTGTCAGACCCCACAGGTCGAGGGTTCAGACCCCAGGGCGGCCCCTTACTCCCGATGCCCCTCTCAAGCC
CTGGGATGTGTCACCTGTGCTTCTCACAGACCAGCTGTGAATTGGGGTTCCCACGACCCTGTCTTTGGGTTCAATTAATT
TGTTAGAACGGCTCACAGGGCTCAGGGAAGCACTGAGCTTATTTATTATAAAGGATGTTAGAAGGATACAGATGAAGAGA
TGCTAGGGAGAGGCATGGGGGAGGGGCACGGGGAAAGGGCATGGGGAAGGGGCATGGAGCTGCCATGCTCTCCAGGACAC
CACCCTCCAGGAGCCTTCCCATGCTCAAAAACTCTGTCCTCTTGAGTTTTTATGGAGGCTTCATTAGGTGGGCGTAATTT
TTAAAATTATATCATTTTAAATTTTTAAATGTTTAATTTTTGTAGGTTCATAGTAAGTGTATACAGTTGTGGGGTGCATG
AGATGTGTTGACACAGGCATGCAATGTGACGTAATCACATCATGGGAAGGGCGTATCCACCCCTTCAAGCATTTAGCCTT
TGTGTTACAAACAATCCAGTTACACTCCTTCAGTTATTTAAAAATATACAATCAAGTTATTCTTGACTATAGTCACCCTG
TTGTGGGATCAAATAGTAGGTCTTATTCATTCTTTCTCTATATGTATATGGTTTTTGTATACATGGGCATGATTGATTAA
ACCACAGGCCATTGGTGATCAGTTTGACCTTCAGCCTCTCTCCCCTCACTGCTGGAGGTTGGGGACTGGGCTGAAAGTCC
CAGTCCTGTAGCGCTGACTTGGTTTTTCCTGAGACCAGTCCCCATCCTGAAGCTGCCTAGGGGCTGCTGGCCACCAGTGA
GTCATTGGCAAACTAAACACTTTGGAGATTCCAAGGATTTTAAGGCAGAAGACCAAATAAATATTTTACACGATCACAGT
GGGAGGGGGTTGGAGGGAGATGGAGGTGGAGAGCTGGGGGGGCCTCGCAGTTAGGATTTTAAGTACAGTGGGAAACCACTG
GAGATTTTTCCATCAGGGAGGGCTGCGTTTGCTGTTTCTGCTGTGGGAGAGGCTGCGGGGGCAGGCGTGGATGCTGGAGG
TCTGGCTGGAGGCAGTGGCCCTCCTCCTGGTGACAGATGGTGGTGGTGGTGGTGGTGGCCTGGGCCAGGGCGGAGCA
GTGGATGAAGAAGGGACAGATGCGGGGTGTATTTTTAGGGGCTGGAGCTCATAGAACATGGTGCTAAACTGGATGTGGGA
AGGGAGGGGTGAGGACGCGTCCAGTGAGACTCTCAAGTGTCTGAGCCTGACGGCCAGGGGGAGGGAGGGGCTGCTTTTTG
TTTGTTAGTTTTTCATTTGTTTTTGTTTGCTTGTTTGTTGTTTGTTTTCTTTTTTGAGACAGAGTCTCGCTCTGTCGCCCA
GGCTGGAGTGCAGTGGTGCAATCTCTACTTACTGCAACTTCCACCTCCTGGGTTCAAGCGATTCTCCTGTCTCAGCCTCA
CAAGTAGCTGGGATCACAGGCGCCCGCCATCATGCATGGCTGATTTTTGTATTTTTAGTAGAGACGGGATTTCACCATGT
TGGCCAGGCTGGTCTCGAACTCCTGACCTTGTGATCTGCCTGCCTCGGCCTCTCAAAGTGTTGGGATTACAGGCATGAGC
CACCGCGCCAGGCCATTTTCATTTGTTTTTGAGACAGGGTCTCTGTCACCCAGGCTTGAGTGCAGTCGTGCGATCATAGC
TCACTGCAGCCTCGACCTCCTGGGCTCAAGTAATCCTCCTGCCTCAGCCTCCCGAGTAGCTGGGTCTACAGGCACATGCC
AGGACACCTGGCTAATTTTTAAATTATTATTATTATTATTATTATTATTATTTTAGAAAGAGAGTCACACTATGTTG
CCCAGGGTGGTCTCGAACTCCTGGCCTCAAGCGATCGGCCCACCTCTGCCTCCCAAAGTGCTGAGATTACAAGTGTGAGC
CAGAGGGAGGTGCCACTAACTGAGGAGGGAGGCAGGAAGGTGGGGCTGGGAGCAAGTAGGGCCCCGATCCCCCAGTGCAG
ACCCGGGCTTAGCTCAGCATGGATTGAATGGCTGTGAACTCCCTGTGGGGGCTGCAGAGGGAGGAGTCAGGACACTGAGG
TTGTTGTGTCACCCACACCGGATTCATCCCCTATCCCGGTCCACCCACCCCCAGGGCATCTGGAATGTGAGAGGCCAGAG
AGAGAAAGCACCACCAGGAATCCCAACGTAGCAACAGCACAAATAACAGCAGCCCCTTTTGATGCTCAGGGGAGCACATG
TTTCATGGAGCTATCCAGTGACCTGTACAGCAGGGAGACTTGAGGGGACCATTTCACAGATGGGAAGACGGAGGCTGAGG
GGTGAGGCTATATTTGCAAGGTGGATCTTTTTTGTGTGTGTGAGCAATTGGTCGCGCTGTCTCCCTCTTTAGACTTTACG
CTGCTGAAGGCAGGCACCGAGTTCTGTGGGGTTCGTGGTCCATGGGCATGCATGCAAATCAGTCTTTGCTGAGTTAATGA
ATAAATGATCTGGGGTGTAGCGAGGCTGGGGATGTGGTTGCGGGAGGGGAGTTCTGCA
```

HUMAN mRNA SEQUENCE : hR7-104.1 (Seq ID No: 81)

```
GCTCAGGCCCCGCCCCTGCCGCCGGAATCCTGAAGCCCAAGGCTGCCCGGGGGCGGTCCGGCGGCGCCGGCGATGGGGCA
TAAAACCACTGGCCACCTGCCGGGCTGCTCCTGCGTGCGCTGCCGTCCCGGATCCACCGTGCCTCTGCGGCCTGCGTGCC
CGGAGTCCCCGCCTGTGTCGTCTCTGTCGCCGTCCCCGTCTCCTGCCAGGCGCGGAGCCCTGCGAGCCGCGGGTGGGCCC
CAGGCGCGCAGACATGGGCTGCTCCGCCAAAGCGCGCTGGGCTGCCGGGGCGCTGGGCGTCGCGGGGCTACTGTGCGCTG
TGCTGGGCGCTGTCATGATCGTGATGGTGCCGCTCGCTCATCAAGCAGCAGGTCCTTAAGAACGTGCGCATCGACCCCAGT
AGCCTGTCCTTCAACATGTGGAAGGAGATCCCTATCCCCTTCTATCTCTCCGTCTACTTCTTTGACGTCATGAACCCCAG
```

```
CGAGATCCTGAAGGGCGAGAAGCCGCAGGTGCGGGAGCGCGGGCCCTACGTGTACAGGGGAGTTCAGGCACAAAAGCAACA
TCACCTTCAACAACAACGACACCGTGTCCTTCCTCGAGTACCGCACCTTCCAGTTCCAGCCCTCCAAGTCCCACGGCTCG
GAGAGCGACTACATCGTCATGCCCAACATCCTGGTCTTGGGTGCGGCGGTGATGATGGAGAATAAGCCCATGACCCTGAA
GCTCATCATGACCTTGGCGATTCACCACCCTCGGCGAACGTGCCTTCATGAACCGCACTGTGGGTGAGATCATGTGGGGCT
ACAAGGACCCCCTTGTGAATCTCATCAACAAGTACTTTCCAGGCATGTTCCCCTTCAAGGACAAGTTCGGATTATTTGCT
GAGCTCAACAACTCCGACTCTGGGCTCTTCACGGTGTTCACGGGGGTCCAGAACATCAGCAGGATCCACCTCGTGGACAA
GTGGAACGGGCTGAGCAAGGTTGACTTCTGGCATTCCGATCAGTGCAACATGATCAATGGAACTTCTGGGCAAATGTGGC
CGCCCTTCATGACTCCTGAGTCCTCGCTGGAGTTCTACAGCCCGGAGCCTGCCGATCCATGAAGCTAATGTACAAGGAG
TCAGGGGTGTTTGAAGGCATCCCCACCTATCGCTTCGTGGCTCCCAAAACCCTGTTTGCCAACGGGTCCATCTACCCACC
CAACGAAGGCTTCTGCCCGTGCCTGGAGTCTGGAATTCAGAACGTCAGCACCTGCAGGTTCAGTGCCCCCTTGTTTCTCT
CCCATCCTCACTTCCTCAACGCCGACCCGGTTCTGGCAGAAGCGGTGACTGGCCTGCACCCTAACCAGGAGGCACACTCC
TTGTTCCTGGACATCCACCCGGTCACGGGAATCCCCATGAACTGCTCTGTGAAACTGCAGCTGAGCCTCTACATGAAATC
TGTCGCAGGCATTGGACAAACTGGGAAGATTGAGCCTGTGGTCCTGCCGCTGCTCTGGTTTGCAGAGAGCGGGGCCATGG
AGGGGGAGACTCTTCACACATTCTACACTCAGCTGGTGTTGATGCCCAAGGTGATGCACTATGCCCAGTACGTCCTCCTG
GCGCTGGGCTGCGTCCTGCTGCTGGTCCCTGTCATCTGCCAAATCCGGAGCCAAGAGAAATGCTATTTATTTTGGAGTAG
TAGTAAAAAGGGCTCAAAGGATAAGGAGGCCATTCAGGCCTATTCTGAATCCCTGATGACATCAGCTCCCAAGGGCTCTG
TGCTGCAGGAAGCAAAACTGGTCCTGAGGACACCGTGAGCCAGCCAGGCCTGGCCGCTGGGCCTGACCGGCCCCCAGCC
CCTACACCCGCTTCTCCCGGACTCTCCCAGCGGACAGCCCCCAGCCCCACAGCCTGAGCCTCCCAGCTGCCATGTGCC
TGTTGCACACCTGCACACACGCCCTGGCACACATACACACATGCGTGCAGGCTTGTGCAGACACTCAGGGATGGAGCTGC
TGCTGAAGGGACTTGTAGGGAGAGGCTCGTCAACAAGCACTGTTCTGGAACCTTCTCTCCACGTGGCCCACAGGCCTGAC
CACAGGGGCTGTGGGTCCTGCGTCCCCTTCCTCGGGTGAGCCTGGCCTGTCCCGTTCAGCCGTTGGGCCCAGGCTTCCTC
CCCTCCAAGGTGAAACACTGCAGTCCCGGTGTGGTGGCTCCCCATGCAGGACGGGCCAGGCTGGGAGTGCCGCCTTCCTG
TGCCAAATTCAGTGGGGACTCAGTGCCCAGGCCCTGGCCACGAGCTTTGGCCTTGGTCTACCTGCCAGGCCAGGCAAAGC
GCCTTTACACAGGCCTCGGAGAACAATGGAGTGAGCCACAGCCCCTGTGCAGCTGCCCGGAGGGTCTCCGCCCCACCCCG
GCCGGACTTTGATCCCCCCGAAGTCTTCACAGGCACTGCATCGGGTTGTCTGGCGCCCTTTTCCTCCAGCCTAAACTGAC
ATCATCCTATGGACTGAGCCGGCCCACTCTCTGGCCGAAGTGGCCGCAGGCTGTGCCCCCGAGCTGCCCCCACCCCCTCAC
AGGGTCCCTCAGATTATAGGTGCCCAGGCTGAGGTGAAGAGGCCTGGGGGCCCTGCCTTCCGGGCGCTCCTGGACCCTGG
GGCAAACCTGTGACCCTTTTCTACTGGAATAGAAATGAGTTTTATCATCTTTGAAAAATAATTCACTCTTGAAGTAATAA
ACGTTTAAAAAAATGGGATGCCTGCCTCTGTGACAGCCTTGTTTGCTGAGGTCGTGGGGGTGGGGGCCTCTGGGAAGTTC
CGGGCTCCTCTTCTCTTGGTCAATAGCTCCTTTCTGGTGGCTGCCAAGAGCGTCTCTCCCAGGGCCGGGCTGCTGGCTTA
CCTTCCTGTTGTTTTCAAATTTCAACCTTGTGCAATGTTGAGTTTCATAGAAATACTGCATGAGTACGCCCTTGTTTAGA
AGCAGCAGGGTCTGAGTCCCATCCCACAGCCCCCAGTGCAGACGCTTTTGCCACTTTTGCATGGGGCCCCCTGGATGTGT
TTCTGTGCATTTATCTACAAATCCTGGTGCCCGTAGGACATGCCCCGTGTTGTTCTAGACCTTTGCTTTCTGCTTGTTAC
ATAAATGGTGAAGAAGAGAAGCGG
```

HUMAN PROTEIN SEQUENCE : hP7-104.1 (Seq ID No: 82)
MWKEIPIPFYLSVYFFDVMNPSEILKGEKPQVRERGPYVYREFRHKSNITFNNNDTVSFLEYRTFQFQPSKSHGSESDYI
VMPNILVLGAAVMMENKPMTLKLIMTLAFTTLGERAFMNRTVGEIMWGYKDPLVNLINKYFPGMFPFKDKFGLFAELNNS
DSGLFTVFTGVQNISRIHLVDKWNGLSKVDFWHSDQCNMINGTSGQMWPPFMTPESSLEFYSPEACRSMKLMYKESGVFE
GIPTYRFVAPKTLFANGSIYPPNEGFCPCLESGIQNVSTCRFSAPLFLSHPHFLNADPVLAEAVTGLHPNQEAHSLFLDI
HPVTGIPMNCSVKLQLSLYMKSVAGIGQTGKIEPVVLPLLWFAESGAMEGETLHTFYTQLVLMPKVMHYAQYVLLALGCV
LLLVPVICQIRSQEKCYLFWSSSKKGSKDKEAIQAYSESLMTSAPKGSVLQEAKLVLRTP*

Table 14

MOUSE GENOMIC SEQUENCE : mD7-157 (Seq ID No: 83)
```
GACTGCACAAGTCGATCATGAAACCCTGAGAGTGGCTTTCCAGATCCAAACAGGTCTGTGACTGGCAGCAATGTCACAAA
CCAACACTGGAGAGTATGGTCACTTAAACAAACAACACTACCAACAGACTTAAAACCCAAACTAATAAAGAGGGCTTACA
GTAAAAAGTCTGGGTTCTCAGCAAACTCCTCCACACACTTTTGGCTTTTGTGGCTGCCTCTAGGCAGTAAATAACATGCC
TAGCCTGCTGTTTCTTGATTTATTGACTTTGGACTGGAGCTATCAACTTCTACAAGTACTGGAGAGAGCCACCTTACTTC
CCCTTCCTGTTGTCGCCTTGCTTTGTTAAGTAGACGCTCTTGCGCAGACTCCTGCATGGACCTTGGCTTGGGCATTTGCA
ACTTTGTTTCCAAGATGAGTTCAAAGTCAAGGATTCTGGCCCTTGACAGGATCGTGTGGAAGGAGAACCTGGCCTACCTG
GTCCCTAGCCCATGCTGTTTCCTCTGAGTAACTCAGCCTGCAGTGGCTCTACAGAGCTGTGTGAGCACCCTGGCTAAGTA
CCTAAGAGAGGCTGGCAGAGGCTGTTACTTAGAGATGAGGGCAGGAGTGTTTGAGGGGGCAAGTGAAGCCAGTAAGCACT
TTATTCTCCTCCATTGGTCTTTTCTGCACATCTGGCTTTCCAGATCACAAGCCCTGGATCTCCAGAGAAAGATGAGCCAG
TAGCTGAAGCCAGTGGAAGGCTCGCACCCAGCACCCAAGGTTTTGGGGGGGAAAAGAACTGCCTTTCTATTGCCAAGTCTT
GAGTCACCTGGTAGAAACTTCAAATGACAGAATTGGCACTGGGCACCAATTCAAACCTGTTTAGTCAGAGGCCAGGCAAG
ATGCCAAACTGCCAGACAGGACAGTGATTGGCAGGGGCCAGTGATTGGCAGGGGCCACACAACAGCTGAGGGCACAGGAG
TCCTTAGAGCCTCTTTATCTTAGGGAAGAACATGGGTCCTGTTTGTGGCTTTGCATATAGAGAGGCCCTGTACTCATTAC
GTTACAGACTTTACTGTGCCTGGGCGTCCCAGCCTGTAAAATGGTTAGATAGGCAAGCAGGGGGTAGGCATTTGAGGACT
CTGGTTTTGAAATTCCGAACTGAGGCTGGCTCAGGGGTAATAATACACGAGAGATCCTGTCTTCTATCTCCAGCACCATG
```

```
AACAATAAATAAATAAATACATGTAAAAAGAAACAAATAATACCAGCTGAGGAAGATTCTTGCAGCTGTGCTAGGTGGAC
TGTTACTATGGCTACGGTAATGCCCACAAGGTGATTGTTAGACAGGGAATGGTCCAGACTGTTTAGGGTGAGTATGGCCA
GGTAGGGTACCAGATATGCATCCTTCTTTTGCTCTCTACTCCAAGTATGCCTTCATCTGAGCTCATGGCCAACAGCATG
TGTTCATACTCAGAATAGTAAGTTGGGGAAGATGGAAATGCATGGCTCTTGTTTTTAAAACTCTTTAGAATATTATGTAT
GTATGTATGTATGTATGTATGTATGTATGTATGTATGTATTGTGTATGTGTGTTTGGCCACACATACCATGTGCGCGTGG
AGGTCAGAGGTCAACTTATGGAGACAGCTCTCTCCTTCCACCATGGGGGTCCCAGGGATCAAACCTAGGCTGTCAGTGGT
AGCAACTACATTTAACCTGATCCCTCTCACTGCCTGGGCCTCTGATGCTCAGGAATGGGGAGCCCAGCAGTGCAGGCTCA
GGTTCCAGGGGCCACCTATTGTCCAGTGCTCAGTCACCTGGCCACCCTTAACTGCAAGGTGACCCAGCCAAGGAAGTCTG
GACCCAGCTGTAAAACAGGGGTTCTGCTCCTCACAGTGGTGGTGGCATTGGGAAGCAGCCAGCCTTGACACTGACTCGAG
AAGGTCCCAGTTGGAGCCGGGGCTGGGTTCTGACTTTAGATGGACTCCATTCCACCTCCAATGGCTTTCCATCCTTCAAC
CACTGCCAAATTTTTAATAGTTGGATTTCTCACATAAAATTCACATTTCTAATTTCTCTGGCAAAAACTGAGGACCAGGG
CTGGAGAGATGGCTCAGTGGTTAAGAGCACTGACTGCTCTTCTGAAGGTACTGAGTTAAAGTCCCAGCAACCACATGGTG
GCTCACAACCATCTATAATGAGATCTGATGCCCTCTTCTGGTGGGTCTGAAGACAAGCTACATTGTACTTACATATAATA
ATAAATTAATCTTTGGGCCGGAGTGAGTAAAGGCCCAGAGTTCAATTCCCAGCAACCACATGATGGCTCGAACCATCTGC
ACAGTTACAGTGTACTCAAATACATAAAATAAATCTTTAAAACAAGCAAGCAAGTAAACAAACAAACACCCCTGAGCACC
AGACTACATGGTCCCGTATTTACTGCAGCGAACATCCTCTGAGCTGGGCACTGCTGTTGAGCACTGGGCTCTCTTGTTCA
TGCTCTCTCCCTCCTCCTGCTGGTTCCACCTCCCTGCTCTGGGCCAGTTTTGTCGCCTGGCTTATGAGTATCATGGTTTG
TAATTCTTGTCCCAGTACAGCTACCAGCATGCAAGAGACAAAGAAATTGAGGCCAGAGAAACAAGGGACCAACCCCCAGG
AAGATCTCAGCATACCTCCTGCCTCACAGGACACCCCAGGCCCAGCCAGGTGCTTCTGTGCCTGGGAGGAAGAATGTCCT
CTTGTGGAGAAACAGGTAGGCTTGGGTCTGGTCTCCAACCTGAATTCTGTCCTCTTCTAAATACAGCAGGTGACAATAGT
TCTCCAAGACTCATATTCAGACTTTATCTTTGGCAGGGCATCCACAGAGTCTGCTTCCTTGGAGCTCAAGGGACAGTGAA
TATCTACTGTGTCTCTTAACTGGACCAGATGTGATATGAACTCAGGAGATGTGAGAAAGCAAAGGTTTGCCTCCCAACTT
GATGTGGAAAGTCTGTGGCTACCGGGTCTGTGTAAGCTGCAGTTTCTTTCTTCTGTGGTTGTGGCCAGAACCATGGTACT
AAAGCTATGGTGAGAACAGCCCCGGACAGGGTGTAGAGGTGAGACTGAGCCCTGAAAACACAATGTCTTCTTGGCTATAG
GAGCAGAGCTGCAGGCCTTAGCGTTCTCCAGCCCCTGGGGCTGCCTTCTGGGGATTTAGTTATGGCCCGGGTTGGTATAA
CCTTGGGAAGACCCAGCTTTCCCTTTGAGTCAGCTATTTTAAGGAATGACATGTTTCAGAACTGGGCTCAGCCTTCGATG
CATCCCCACATCTGGTTTTGGTCTCCGGATAACCTGTGTGTGTACCTAGATACTCATGAGTTTTGTTTTGAACAAACAAC
CTTTGCACAGCCACAATGGAAATAGTTCTAACGTGGTTTTTAAACAGCACCCACAGTCCCAGCCCATGGTTCTGCATCCT
TGCAGCATTGCCTGAGAGCTTGTGACAACTGGGGCACTGCATGGTGGGAAACACAGGCTTAGACAGGAAAGTTGAGGCCC
CAGTGGTAGGTCACCTCCTTAAGGTCACTTGAACCCAGTTTGAGAGTCTGCCCCTTACTGCTCCTCAGAATGGCACTCCT
TACCCCACACTGCTACTGTGCTCAGGCCTTGGCTTAAATACTCTAATTCTTGCTATATAGATGCTTGCCATGTAGATGGT
CCCTAGGTACCAGCTCTACCTTGGAGGAGAAATACTCTTGAGACAAGCAAGGGCACAGCCTTTGTGGTCTCTGTTTCTAC
TAAAGTGACCACTGTAGTAAGCATGCTGTCGTTTGCCTAGAACCAAGCAGTTTCTTAGAGTGCAGCACTTTTTAGGCAAA
AACAATCGTGGACAGAATGAGGTAGTTGGTTCCCTCCAGGTGAAAGCTGGGGCCAGTTGTTCCTGGCACGGGGCATGTAG
ACTCTTTCTAGCTCCTTGCTTGTGAGCCTGACAGCCCTGTCACGGTCACTTGTCACCAGAGTGTCCCTTCCTGTGTCTTC
CCCGAGATGAGGAGACCCAGGCCTCTGGAGTTCCACAGAGGAAACATACAAGACTCAAACCCAAAGTGTGACCCATCTCTT
ATCTGAGCCCACAGGAGACCCAGAGCTCACAAAGCTACTTAGTAAATGTTTAGACCACCTGCAGCTTGGCTGTTACTGAC
TGTGAATGGACAGGACTGGGNNNNNNNNNNNNNNNNNNNNNNNNNNNNNNNNNNNNNNNNNNNNNNNNNNNNNNNNNNNNN
NNNNNNNNNNNNNNNNNNTCAGATACCTCAAATTAAAGTTCAGCATGTGCATGTGGTCCCAACTACGTGGTAGGATGAGG
CAGGAGGATTGCTTAAGGCCAGAAGTTTAGAACCAGTCTACAGACAGCAAGTCCTGATCACAAGAAAATAGAAAGAAAGC
TGAAGGCGAGGGAGGAGGAGATGAGGAGGAGGAGGGAGAGGAGGGAGAGGAAGAGGGAAAGGGGGCAGGAAAAGGGAAGG
AGGGAGGAGAGGGGAGGAACGAAGAGGAGGGGAGAAGGGAGAAGAGAAAGAAGGAAAAGGGAAAGAGGGGAAGAAGGAAATG
GAAGGAAGGAGAGGGAGGAGAGAGAAGAAGAAGAGAGAAGAGGGGAAGGGGAGGAGGGGAGGAATTCGGTTACATAAACAAA
ATTATAAAGTAGTAAAACTCAGAGTGAAGAACTTTTTTCTTTGCATTCTGAGACAGGCTCTCACTCTGCTGGCTTGGAAT
TTACAGAGATCCTCCTGCCTCTGCCTCCAGAGTGCTGGGATTAAAAGTATGTGTGTGTGTCACCATGCCCCACAATGGAG
CACTTTCTAGTTATTACATTTAACTCTAATCTGTCACCATTGCTCAGCTCCTGTCTCCTTTTGCCTCCTTTGTCATGGGA
ATCAAGGTTCTGGGACCCAAGCACTGGATGGTTTGACAGGTGTGAGGGCAGGGTCTTTCCTGAGTTCGTCCTGTTAGCTG
GAGGCTCCTAGGGACAAGCCTTATAAAACAGGGAAAGTGGGCCCAGACAGGCATGCAGGGAATAGAAGCAGAGGCCCAGC
AATGCTACCTGGTCCAGAAATGTCAAAGATGGCCTTCATCCACCAGAAGATGGGAAGAAATGTTCTTGGAACCTTCAGAA
GGAACCAGCCCTGCTGACTCCTTCTGGACTCCAGAACCAACAAGACATTTGTGTTCTTTAAGCCCCTCGGTCTTTAGTAC
TTCGTTATAGAACCCAGGCAGACCCCTATACCTTGAGGTTGGTGACATTTACTACATGAAGATCGTGGCAATGCTGAGCC
TTGGGCTGGAGAGATGGCTCAGTGGTTAAGACCACTGACTGCTCTTCCAGAGGTCCTGAGTTCAATTCCCAGCAACCACA
TGATGGCTCACAACCATCTATAATGGGATTCCAGTGCCCTTTTCTGGTGTGTCTGAAGACAGCAACAGTGTACTCATACA
GAAAATAGATAAATACATCTTTTAAAAAGAACACAGCAGGGCGTGGTGGCACATGCCTTTAATCCCAGTACTCGGGAAGC
AGAGGCAGGCGAATTTCTGAGTTCAAGGCCAGCCTGGTCTACACAGTGAGTTCCAGGACAGCCAGGGCTATACAGAGAGA
CCCTGTCTCAAAAAACAAACAGAAAAAAAAAAAGAAAGAAAGAACACAAATGTGTTGTTGGTTCTGGTCAAGGGTTAAGG
TGTTAACAGGGTTGGTTCCTTCTGAGGGCCACAAGAACATTTCTTCCTGGCTTCTGGTGGATGGAGGCCATCTTTGGATG
GAGGCCATCTTTGACATTCCTGGGTCTGGTAGCAGAACCAAGTCAGGCAGAGTGGCCGATTCTGGCCTGCTCAGGACAGA
CAGATCCAAGGCTGCCTAACTTGCAGCTTCCCTGGTAACTGGAGTATGTGTCTCTCCCCGGGAGGGAGGTGTCCTCTCCT
AGTTCTCACCATACTTAACCAGCTGTCAGCTTCTCTACACTTGGGCCCATCCCCTTCTAAGCCTCCCCATCCTGTCTGTG
CCCTGAGTCTCTTCCTCACTCTTGGGTTATATGTAAATATTCATCTTCATATTTCCTGGGTTACCCCATTACTCCTGCAA
```

```
GTTTGAAACCTCTTTCCTGTCTTCATCAAGGCATGAGTACCCTAAAAAACAGACATATGTGTGTCTGGTCCCATAGAGAC
CATCCTGACACTTGGAAGATGAACAAATGAGATACATCAAGTAAGACTTCATGATGTAGATAAGCCAGGGATGGCATCCA
TTCAAGATGCTTTTGCTGGCTTTGCCTGGCAATGCAGTTACATTTGAGTCTCAGCCTCCTAGTCCACAATCCCTGGATGA
GACTCAGTGCTTGGACGGGACCTGAACACACAGGGACTCCAAGGTGTTGAATGTTCTTACAAGTAAAAGGATGTGGGACT
TCAGGGGAGGGGGGTGCCAGGGAAAGAGCTAACTTCCCTGGTTAAGAGGGAGAGGACAGGTGAAGTTTCTCTGGCAGAGG
AGGGATGAGAAGTGGGTTCCCTGTGTGGGTGGAACCCTTCTTTGGGGCCAACCCCTTCTATGCTTTTCTACTGTGGGCCC
CGATGTGACATTTGGTTTCCCCCAGTTTCCTGTGTCTGTCTAGTGGGCTCAGGGGGAGTGCCAACTCCAGGCTGAGGTGA
CGGTGAGGGCTGCAAATGTGACTCAGTCACCTCAAGAAACAGGGCCAACCTGGAGAAGAGCGATGTCACGGAACACTGAG
GATGTGGCCCGATAGTGTCGATTCACCGTGGAGTGGCACAGGCACGTCTCTCTTGCCTGTAAAACGAGGGCAGATATTAC
TCTTAGGTCTGCTGGCTGCTGGCATGGGTGTGTCTTCCAGCAGTATCCTCCCCCATGTCCCCCACTCCATCTCCTCATGT
GACTTACTTGAAAACACATCAGGATGGACCTCTCATTGTCCCCTACGGACTGAAGATCTAAGAAAGGCTCCAGCTCCGAA
GCACTGAAGTGCTACTGGGTGGCAAACGCTCAGACAGCAACAGGGTGCGCGGGGGTGGGGGGTGGTTTCCCAGTTCTAGA
TTGGGCACAGATGGATGACTCTGTATGGAGGAATCTAGATGCCAGACTTGACCTCGACCAAAGGACACAAAGCTTCTACG
AATGAGCTCACTGAGAACTTAGCCCAGTCAGGCTCTGCTGCACAGCCCAGGGTGGCCTTGAACTCTCCACCCTCTTGCCA
GAGCCTCTCGCTGGGATGTGATAGCCATCACACCAGGCTTACCATGAGCTAGGCAGTGCCATCAGCTACCCATCCCGACT
CTTTTCTGCAGAGCATGGTGAGACAGCCCGGCATGCTACTGGGGGTGGGCTCTAGAGCAGTGAGCTCACACACCCACATG
CCCCTGAAGNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNTGAAGTGTGGGTGTCACAAGTTAGGGATACAAGAAATCACTGGCACTCCCCTGTGTCCTTATCA
GGGCAGACAGTGCCTCTGGGGCCATGGGGATCTGTAGTCAAGCTTTTTCTCTGGGTCCTTTTTTTGACATTCATCTTCCT
TGGCTCCTCCCCACCCCCATCTGCTCAGTTCTAGAGATCCAGTTTGTGCTGGGCAGAGCAAGTTTGCATGCCCACCCTGT
AGCCAGAAGTTCCCACGTGGAAGGGGTTATGGCGGGATTGAGAGTCCCAGTGGGGCCTATGCAAACAGGTGCTTGTGTCA
TCTCAGGGGACTGGTGCTCCCTTTTGATATCCTTCTCAACTCTGTGGCCTCGTCTCTGTCTTTGGTTCCTTCATTCAGTT
GCACAGCAGTGAGCACCCTCCTCCTCCTCATCCCTAAAGTCAGGATCACCGACCCCAGGGCCTTTGCACAGGCTCTTCCA
TCCACTAGAATGCTCTGCTCTTGGTACCCCGTAGGCTGCCTCCTTGGATGCTGTCTACACTCAAGCATCATTTCTCACCA
TGAATCCAGCCCTGCTCCTTTATCAACTTCCCTCACCATATTTTCTCCCCATTATTCTTACTATAGCTCTGGATAGATTT
CATATTTATTGCTCATTAAATTTATGATCTGTATCCCTGCAGCTTTGAGCGGGCAGGGATTTTAGTATATGGCTTGTAGC
TGTCGCTCCTATGCCCTGCACAGTGCCTGGATCACAGCAGGCTTTCCACAAACATGTGACGGAATCCATCCGCTGGTCCA
TCTGTCCATCCACCTATCTATCTATCCATCCATCTTTTCTTATCCACTCACTAGCCATACATCTTCCCTCTAGACCAGAC
CCTATTCTGGCCCTGCTCTGGTAACTGCTGGGGAAAAGCAGACAAGACAAGCATGGGATTGACCTGAGGGGCAAGAAAC
ACAGTGGCGGAAAAACATGGCGGGAGGGCCCGGTGGGGAGGGAAGCGAGGTAGATCCTAGGGGACTGACAACCCCATGGG
CCTGGCCCACAACAGTGTTTCATACCCCTTGCTCAGAGGATCAGCTTCTGCTTGGATTGGCGTCCTGAGGGAAGTATGGC
TCAGCCAGCAGGTCTCTATGTTGGGAGCTGGGTGTGTGCATCAGGACTCACACACATACCCCGCCCCCATACTCGTGCCT
GATTGCAAGAGCATTGAAAGAGAGGTGGGTGGCAGGGTCTGAGGGAAGTGCCTGCACGAGGCCTCTCTCCATTGGTGGAG
CTCGCCTGGACTTCTCCCCTTGGCCCCCCTTCCAAGATACCTGGGGCAGCTGCCACCTCCCATCCCTAGCAAAAAGCTCCA
GTTCCTGCCCCCTCACCTTCTGCCTGCTCTGATCCCACCTGAGTCCAGCAGGACATATTGTGAGAGCCCCACCCACAGCC
CCGGAGCCCTCCTGTATCCAGCCTGTGGCTGGTGTGAATGGCGCTGCAGACAGGATATGGTCTGAAGAGGCTGGAGAAGC
CACAAAGGGCTGGCAGTTTGCAGGTGTGTGTGGGCGTGGCGGCCTGCCTCCCACAGCCTGAAAGGGTCTCTGAAGCCCAG
AGACAGCGTGATCCCGGCCTCCCACGGGGCAGCTTTTACTGTCTAGGGAAGAAATCCCCAAAGTCCATGGAGTCTGAAGA
CTCTGTCAAGCCTCGCTAGGAAACCTAGGAGTTTTAGAGGGCACTTGGCACCGGAAGCTAGCCGGGTAGGCGGAGCCTCA
CCTGGATTGAGTTCACAGCTGCCTAGACAGGCTCAGACTAGGTGCTGGGCACCTGGAAGGAGGAGGAGACATTAGCAGCA
AAGGCTGTTAACAGAAGTGCCTGCCTAGGCTTGGAGGCAAGACGCTGCTGTTCACAGTGCGAGACGGAGGTAAGAGAGGG
CAGCGGGTACGGCATGGACTTCGCCTGGCCTGGCCCGCTTGACCTTAGTCTAAGGTTTAGCGCAGTGCTTGGGAGTTCTC
GAGTGTGTGCTGGATCGTATCTCCCAGGGAACGACTGAGTTTGAGGTTCTAGCTGGTCCTAGGATACTCCTTGCCGCTCA
GAGTGTGGGGCTGGGCGTGCTCACGAGGGGGCACTGGAACACGTTGGCCAGCTGTGTCCCTCTTTGCTCAGAAGGGCACC
ACTGCCACGAGGGCCAGAGTGAAGCTTGGACCCTGGCGTGTGGTTGAGGACTGGCGGCATGAACCCCATTTTTCCCTTCG
TCCCTAGTGCTTCCCAAGCAGTTTCCTCCTGAGATCCACGGGCGGGGAGGGCGGGGGGTGGGGGGGACCGGTTTAGCAG
CAGCTCACTTTGGTTGTTTGACTTTTGAGGAGGAAGAGGCAGGCAGCCTTTCCTGGTAAGCCAGCAAAAGGGTAGATAGA
GCTCATGTATATGTCTCTCTGTTGCTGCCAGCCACAGGTCCCCATGCCTGTCAGCCCAGGTCTGTCACCCCGTCTGCAAG
AGTCACTAGAGGGGGTCCCAAGAGTGCACACCTCCTACCAGTTGCTTCCCCCACATGGTAACCCCAAAGATGCACCCAGA
```

```
TAGATAAAAGTTCAGGCTTGCCATGGCCTCTGGTGGGACCCTGGCATTCCTTCCAGCTGGACCCATGTCCCTAGCTGTAC
CATGATGGAGATAGACGGGCCCCCAGATGTCAAATCATATATGGCTTCTAAAGCCCCAGTGCCCAGCCCCCAGCCCTAGC
TCACAGCCCCCACTCAACCAGACAAAGGTGGGCGGCTGGCGCCGCTGTTTGCCTCTGCCAGTCTGACTCCCTGAGGCGTG
TGGGCTGCATGGTGGGCCCCTGCTAATGAGCATCTGTTATCACTCCATTATGCCGCCTGCGCCACCTGCACCCCAGCACT
TAGTGGGAGACCCCGGGGGGTTCCCTGGCAGCTGGGCAGAGGGGCATGCAGAAGGCAGGAGGTGTTGGGGGGGGGCAAGG
TCTTCCGGGGCTAGGGGGCCCCATGCTCTGTGGGATTTCTACAAAGTACTTAGGATTCCAGCTCTTTATTGTATCAAAAG
AGAAGTGCTGGGGATGGGCGGGGTGTAATCGCCACACTAGGATGGATGAGGCAGATCGGTTTTGAGTTCCAGGCCAGCCT
GGGCTACGTGGAGAGATGCTGTATTGAAAGAAAAAGGGGGAGGGAAAAAGGGGAGGAGGTGGTGGCAGTCTAGGGGGATT
TTGGGTGAGGAAACGAGTGTCTGTGAATTCCCAAGTGAGTCTCCCCAGCCAACATCTCACAAACGCATCTGTGCTGTGTA
TGTATTGAGAAGGGGATGTGGTTGCACATGAGGGCACAACCAAGATTCCGGTCCTGGAGACTGGCAGTGAGTGACCTGGC
ATAGTGTTCTTCGTAAGATGGAATGACCTACCCATTCTTCGTACGGGGCTGTACTGTCTTGTGGGGTAGTCCCTAAACCT
CTCTGGGCTTCCCTTGCCTGCCTTGGTACCGAGGAGATAATGCTTCTCTGCTTGATCAGAGACAGTTGGACCAGTTCTGG
TATCTGAGCAGCTTCCACTCAAACCTCAGGACAGGACCCGGGGAGGGGCGGCATAGACCGGGCACAAGTGTGAAACAGTT
GGCAGCAGCTGACATGGCCTTTTTGGGGAATGCTAGTCACCTCAGACCATGCCTGGCTCTCTAGCTGAGGCAGGGTGGCT
TCTGTGGAGGGCCGAAGTCCCTCTGTTCCTGCACACTGGAGATATTTTAGGAGCAGCCTCAAGGGGCTAGGGAGATAACT
CAGCTGGCAAACATGCTTGCTGTGGAAGCATGAAGGTCTGGGTTTGGATTCCCCAGCGCCACAGTAAAAAGCTGAGCTTG
GTGGTTTGTGCCCGTACTCGAGAGAGGCAGAGACAGGAGGGTCTCCAGCAATTCTAGCTGAATCAGAGAGCTTCAGGCTC
TGGGAGAGACTCTGTCTCCAAAGTAATTTGAAGGGCCAGCAAGATGTTTAGGCTAAAGGACTTTCTTTCAGGTCTCATG
ACCTGAGTTATCCCTCTGGGTCCCACATGAGGCAAGGAGAGAACCAACTTCTGTGAGTTATCGCACACACACAGACAC
ACACACAGACACACAACCTAATTCCTAAAACATTTAACACGTAAGATGGAGAGTGATGGAGGAAGACACACACAGGGTTG
ACTTCTGGCCTCCACCCCATTTGCACATAAACATGCACACATGCATGTATTTCAACATATGCTCAAGAGCAAACCCAGTG
GTCAGGAGGGTGCCGTACCCATTTTTACACATGAGAACACTGAGGCATGGAAAGATCAAAGCCCAGGAATTTGATGCTAG
ACTCTCACGGCAGTTATGGAGGGCTTTGGAGCTATACTGGGAGGAACTAGAGTGTCGTGGAGAGTGGTTTGGCAGCTTAG
TCAGTGGCTCCCGTGGGTCAGCCTGCTTCTGAGCAGAGTACAGGCCAGAGAGTCACACCCTGAAGAGGCCGAGCGGAAGT
CTTCACCCTGAGAGGAAGATCTCATCATAACAACTCCCCCTGATCACCTGGGCTGCAGAGGGGACCTCTGTTTAGAGATG
GGTAGGGTCTCACAGGAGGACCCCAGGCCTCTGGATCCTGAAGAACAGGCACCAGACTTGTGACAGGATTGGTGAGGGCT
TATTCAAGGTGCTCATCTTTGCCTTGGGCACAGGGGCTTCTGAATGCTGAGAGGCAGGGGCATGGCCCCATGTGACTCTCT
GAGCAAGGATCTGGCTGGATCCAGCCCCTCTTACTGGAGGGCTCTTGGAGCGGGGGTGGCTAAACTGAGGGTCAGACATTC
CGTCATGCCACAACATGAACAGGCGGTTCTTAGCCAGTTTGCCTGTCTCAGGTAGCTGGGCACCAGGTATGCCCACCTCT
GAGGGCAGTCAGTGGGGGATGGCCTTGGAGGCAGCCAGCATGAACTTGGCTAGGGAACAAGACACTGGATTGGCCTCTTC
CCTGCAGTCAGGAATGAGGTGAGCACAGGGAGAAAGGTGGTGTGCCCATGCCGGGGACCAAGACCACTTCTCGTTGGCAT
GCCATCAAACATCATTGGGGCCAGAAAGAGAGACCTGGGGGAAGGAACTGTCCTGGAAGTCAAACCCTGGTTCCAGCCAT
GACACTGGGCTAGGGGGCTCCTTCCCTCTCCTAGTGTAAACCCCTTGGCAGGGCTGTAGGGGAAAACACCACCCACTCCT
ATGGCTTATGACAAGTCTACCACTGCTATTTGGCACAAGGGGTGAAGAGGGACAGTGCTGTGGCCACGAGGGGGATTAAA
GAATTTCTGCTAGCATCTAGGATATGGGACTTGCCTCAGACCGCAACCTAGGGCTTTGGAAATCCGAGGCAGGTAAATTT
AGGCAGAACCTGCTTGGCTCCATTCCCATGGACCTAGAGTGGACTCCAAATAAGGTCTCAGAAATCAGGCATACTTGACT
TGGGCAGACTCAGCTCACATCTTGTCCTGGCCTTGATTCCACATTATCTGCTATGTTGTCCTTGAACCCCAAGCCCAGAC
TTGAATCCCTATGAGGCCCAGCCCATTTTCATGGCAGAGAGAGGCCCCTGTTTCCCCTCCTCTTGGACCAACCGTTTTTT
TTTTTCCGACTCCTCCCTTGCTAGGGGAAGGGATGCCCCCCAGTGGGGAAGGCTGTCCTGAGTGCTGGCACACAGCTGCC
TTTGTAAGTGTGGCTGAGTCACATGGACACCCCCCCATCCTTGCTGGACCCCGGGGCGATGTGACTGGAGGTGGGGAGGT
GGCGTTATCATGGGGCTGTGGCTTCCCCCACCCCCACGCTTGTGGGTGGGAGACTTTCCCACGGGAATGAAGGGAGCAGG
GAGGTGGACGGAGGAACACAGCCCTAGCACGTCTTCTGTGTTCGGAGCTACTGGATTGTGGGGTGGCTTTGGGAAGGTCC
CCTTTCTGTGCCTCAGTTTCCATCTCTGTAAAATGGTCCAGGCATTCCTGTTGTATAGAAAGGACCCTGGATGTTCTCAA
GGTGGAGCTTTATGCCCTTGTGTCCCTGTTAGCAGCAGCGGGCTGGGTATTGTGCTCTAAAATATACAAGGACGTTGAGC
TTGCAAGAGAAACCTGTACTTGAACTCTTTTTGTTGGTTAAAAACCACCTCTGATCAGGCAGTGGGTTTCCAGGCCCCT
TGGCAAGGGTGGATACAGCTTGTTGCTCTTGACAAGGCCAGCTTGTGTAACCTGTTCTATCAGGAAGATTGGAAAGACTG
AGGCCTGAAGGTCCCCTTGAAGTTCCACCAGCCACATACCCTGTCCTCAGTGCTCAGGCCTTGCCTCAGGCAGTAGCCAC
CGTAAGCAGCACTGTTTCTTATCCCTGTCCCCGGGGGTCCATGTGTCCCTGAAGGTCTCTCCTCAGTGCCCAGGGAGCAG
GCGCTGGTTTTTGTCTTCGACTGACAGCAAAGGGGTGTCTGGGCAGAGATGCTCGCCCAAGTCACCAGCTGGCAGAGTCT
GCTGTAGCCAGTTGGCCAGGGACCAGCATCAGTATCGTCTCTCTTCCATTTCTTATAAAGAAGGGGCACCCATCAGCCTT
CTGTGCTTCCCAACCCGTGCTGGCTCTGTCACCAGCCTGTAATCCCACAACCCTTCCCGTGGAGCTGCCATGTTTTTTTT
TTTTTTTTTTTCCTGGCTGGGCCCTGGTATTGTGCTTCCAGTAGCCATGGGAACCAGAGAGGACATGTGGTTCCCTAAT
GCTGCACGCCACAGATCCACTCGCATCGGACCATATATTAGGACGGCCTTTTCTCTCTTCCCAGATCTGTACCCCACCCC
TGACTATACATGTCAGGGATGCTTTTGATACCTCGCCTTTCCCTGTTTGACTTTCCTCAGCCTTGTTTCTGGTCTTTTGG
ATGAGAATCTTCACCCCAGCTTTTCTCTGTCTCCATCTTCAAGGCCCCAGCCTGAGCCCAGCCACCTGTCCACACTTCTT
ACACCACGCCCACCCAGTTCAAGCCGGTAGAGGAGAGATGGGCCTACTTAAGCTTTGTCAAAAATGATAGCCATCCGGGA
ATCCTGTTAAATTTTAAGTTCTTATGGGATGGATCTGGGGGCACATCTGAAATCTGAGGTTTCTCGTAAATTTTCATAAT
TTGAGAACCTAGCATAGATATCTTTCCTATGCTCAAACACCTTCCCAGACGCCCCTGTGCCTTGGATTAGACTTCAGTTC
CATTCACTCAAAAACCTTCCACTATCCAGCCCTACTTCCTTCAAAAATAGCTCTCCCCACCTCTCCACCCTGCTGCTGTC
CCCCTAGAAGTTTCTTCTGAGGGTCTCCTTTCCTTGAATTTCCTGACTTCTCTATTATCCAGCTAGGACTCTGGAGCCAT
TTGGTCCATAGCCAGCCTTTGAAGGGTGTGGCTGAGAGCCCCCTTGCTCACTCTATATATGGGCCTCTTCTGTGTGGTGG
```

```
AGACATGGTGGAGATGATGAGGTTAAGGGCCTGTCCCTACTGGATGTTTGCTGAGAGCCAGAAACCCAGCTGTGTCTTAG
CTGTGCTGCCTTGGATAAGTCCCTGGTTGTCTCTGATGTCTCATCTCACGTAGTGGTGATGGTGTGCAGGGTCCAGGTGC
CCTGGGCTCCTGCCTGTCTGCCTGCCCCTTCTACGTCCCAGCTGTGAGACCTTGAAGAAGTTAACTAACTTTCCTGAGC
ATTCTCTCTCTCTCTCTCTCTCTCTCTCTCTCTCTCTCTCTCTCTCTCTCTCTCTGCCTTGCCCTTTTCCTTCTTC
ATTACCACATTCCTGGAGAATTCCTTGAGACTCATTAATTGTCTGGGCAGGTGCTGGGAAGCAGGGAGGAGGCGGGAAGG
CTGGAACTCAGCGAGTGTGATTGCTGTTACCGCCATTATTGTTATTGTTGCCACTTGCCCTCATTAGGTTGTGAAATCCT
CCTGGGCGCTTCATTTGGCTCACACAGTATGGAGTATGGGAGAGCAGAGTATAGGAGCCTTTACTCTGGAGGGCCATGCN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNTGTGTGTGTGTGTGTCAGGGTCAGGATGTGAGT
TTTGAAAGGTTGAGACTGAGGGAGACTGGTCTGGCTCCCTGCTGGGAAGACGCTCAAGAGACATGGGTCAGCAGTCAGCC
ACATATGGCCTCCAGCCAGGCGGCCTGACACCCCCTTCCTCTTGGAGTGCCTTCCGCTCTGCCAGTCAGCACCGGCTCTG
CCTGCTGCCACAGAGGTTAGACTGTCCTTCAGGAAGGGGTAGTTGTTGGCTGGGCCTGGAGGAAGGGGCAGCAGCAGCTG
AGTAGCCTGTTGCCATGGCAAACTGGTTGCTTTGAAAGAACTTCAGGGGAGCCCCCAGGGACTTAATGATGGGGGCGGGG
TATGTGAGGGGACACACTCATCCCAGCTCATACCCATTTTTGAAACTGGCACCAGGGTCTGGGATGATGAGAGGAGAGAC
CCAGGTCCCCACTTGCCAGGCCTGAGTCACGGGCATCCCTTAGGGTCCCCGCCTCACCTCCGTCAGTCAGCCCTGCCTCC
TCCTGCCTCCCTCTGAGAAAGCCATGACTCACAGTTAGCCAGTCATCTCATCTCTAGCCTGAAGGGAGGAGCTGAGCAGG
AGGGTGTCCCGGCCCTCCCCCAGTTCTCCGGGTTCTCCAGCTCCACACAGCCTGCTGCCTGGGCTCTATCTACTATAGAG
AAGGGGCTGCTGGCTTGGTAGGAGCCTCCCCAACCCCAGCCAGTGCACTTGCTTGCTCTAGCTCACACTCTTACCCAGAG
ACCCAGACTGTAAAATTCAGCATTGGAGGTGAATCCTGAAAGGTGGTTCAGCTGCCTGGCTCCTTCCAGAACCCGTCGCC
TGGCACCAGGTACCGGACTAAGGGCAGCCAGTGGGTGGGAGTAGATGGGGAGGGCATGTGGCTGCTAAACAGAGATTATA
GAATGTAAAGGGATCTGGAGGGCGGGGGTCTTTAGTGCACCGCAGGGGTAGAGATGTGTGTAAACTGAGAGCTGCCTAGA
AAGAGGATGACATCAGAAGGACCAGAACTTTTGGAGCCATTAGTTGTTGGGCATTTGGGAAACCAAGCCTCCACTGGTGT
GGTTCAGTCTTGGGCCCAGCAGTGTGGTGGCAACGCTCTGGGCCTTTTGATAGGCTGGCTGTTCTCACCTTAGGCTGTGG
CATTCACCTGTAATCCCGGTATTTGGGGAGGTAGGGGTAGGTAGGCTCTCAAGTTCAAGGCCCGCCTGGGCTACGAAGAA
GCCGTGTGGGAGTTGGGAGACTCTTGAGTGGGCAGGCAGCTTGTCTGAGGTCACACAGCCAAGTTCAGAGACATTGAGAGC
CAGGCTCCGGAAGAGGGCACTGTGGTAGACTGAGCATGGTGCCCCTGACAGTGGCCTCCTCTATCTCTTAGGGACAGGAC
AGGGGCCCCTAGAGAACCAGGGCAGTAACAACTTTTGACAAAGGAACGAAGGCACAGTGCAATGTTGGTCTGAACAGGAG
GCACCTGGAATGAAGATCCTGGGAAGGGAAGCCAAGGCGCCAAGAGCTCCTTACCCACTAACCACAAGACCTCAGGGCCC
CAGCTGCCTGGGCCTGATAGGACCTCAGGCCCCTTCCCCTAGTATAGGAAATGGGGGCAGGGTTCCTGTCTAACCCCACT
CTCCAACCAGCCCTCTCTGTGTGGTGACCGTTAGGGCCTCTAAGGGGCTCATTTTACTGAGGGAGGGGGCTGGAGTGAAT
AGTGGCAGAACCCCTTTTCCACCTGGGCCATCGTGTCATGGGATGGGTGACATGGGTGGAGAAATCCTGTGTCTCCTTCA
GAAGGCCAAGGGAGGTTGCTGATTTGAAGCATGAAGTGCCTACTGTGTGCTGCACAGTGAGCAACAAGGCAAAGGTCAGT
TGGAACTTGCAAACGGCTCCCTCCTGGCAGCAGCCCTCCAGGATTTGGAGACCGCTGAAAGAGCAAGCTCTGGTCATTGA
GTGCTGCAGGCTGCCAAGGGGACCCCAGAGTTTAGGCCAGTCCCTGTGTGGTCCTCCCTGCCCTGTTCAGAGTCTGTACT
AGCTTTTGGGGCCAGACTTTTATCTCCTTTTGTTATTAGAGCCCCAGGAACTTGAGATACATCCCACCCTGATCCACCTT
GGTCAAAGTCAATGCAACCGGCTTCTCTTTGGGTTGCTAAATTGGCATGGGGGGGGGGGTGACCCTAACCACTGTGCCTC
TGTGTGGGCTGAGGACCTGGCTCCTAGAGTCTGGGGCAGGGGGCTCCTGGCTCCTGGTGCGAACTTTGGCTAGGAGGTTG
AGGTTGTCCTAATCAGCTTGTATGTGGCCCGCCATCCTGTTAGGGACAGGCTGGTAAGGAGGTCTGATTGCAGAGATGAC
AGGAGCTCTGGGTCTGTTAGAAAGGAGGAGTGAGTATGGCCCTAAAATGGGTAGAAGCAAAGAGGGAAGGGCGGGATCCG
GGCATAGATGTGTGGAATGTCCATGGCGGAGAATATAATATGGGGAGAATTTGGGGGAACAATGAAAGCTCGGAAACATG
GTGCCAGCCACAGAACTGCAGCATGACATGGGTCACAACAGAGGGTCTCAATCTCCCGTGCTGTGGGAAGCGATAGTGAG
AGGACCGGGCAGCAGGGTGAGCAGGGCAGAGGATGAACACTGTGGGTCCGACCTGGCTGCTCAGCCCCTGGCTGCTGAGT
CTCAGGCTTAGGGATGGCTTCCTGGGTAATGGAAAAGAAGTGGAACTTATAGTACAACCACAAGGAAGGGAAATGAAGAG
AGTAGACAGACTTACAGCCATAAAGGTGGCCACTGTGGATGACCTCCCGGCCAGTGGATGCTGAGCCCTCATCGGGATTC
GGGATGCAGCATGAGCTTCTAGTCAGTAGGGGAAGGGGCAGGCCCTTGATAAGGGTGTTAGAGGTACTGGTGGGGAGGTT
GGGGGATGTGTGACAACACAGGACCCAAACCTCAGTGTTGTGCCCCCTCCCCTCGGGGTAGAGGTCCTCAGGGTGGCCCT
ACTTTCTGACTCACATCAGATGGGGGCATCATGTGAGTGCGCCCGGCAGCCGAGGGTGACCAGCCTCCAGGGACCTGGCA
GATGGCAAGTAGGGGGTGGGGCAGGGCTGGCACAAAGGGGCCTCTCAGATGCAGTGGGTGGAGCACTGGCAAGCCATCCC
TTGCTCGGCTGAGAGCTTGTCCCTGCTCCAGGCTCTGGACCTCCAGCCTCATCTGGAGTTGCTGAGGGCCCCTGGGGAGC
CTGTAGATTCTGAGGTCAGGCCTGAGCTGCTGAGGAGAGGGACTAAGGGTAGTAGCTCTCTGCTAATCACTGACCTGCCC
GGCGTGAGGTGGTCTGAGATACTGTCTGGGGCCCCATGTGCTCTGAGCTCTCCAGGTTCCCTAGAAGCCCAGCCTGGATT
AGAACAATCCTGAACGTTCCATTCTGGACTCATTGCTTACTGTAGAGACAAGGAATGGGGGTGAGGAGACTTCCCTGGTG
AGGGATCCAAATCTTTGGGGCTCCATGTCTCATTGTTGTTTTCTGCCTCTCTTGGGAGGCAAGGAGGTCCTTGTCTGGGT
CCTAAGCTATCTGTAGGCAGGCATGTGCCCATGCCCCACCACTGGCAAGCCCTTGCATTTGGCGATGGGATTCAGGCACT
TGTCAAAGAGGACTTGACTATACTCCTTTCTGGTTGTGTGGGCTTGTGCCTCTGTTTCCTTGTCTTTAACCACATCTTTG
GGGGGCTGCTCCGAGCATCACAGAGTTAACACAAAAACATGCAGAATAGCACTGGAATCATGGGAAGTTAGCATTCATCC
TTCTGGCTCCTACTGGATGGATAGGCATACCGAAGCCGGAGTCGTCACTTCTTATCTCCAAGGCCTGTGGGGGAGCTGGA
TGCTGGAACAAGTCGGCTTGCCCCCAACGAGTCCAATCCGTGTCAGCAGCAAGGCTAGCATGGGTGGTTGGGCATCAGGC
TCCTGGGCTGGCACTGCCCTTAGTGATGTGTGGATGCAGTTCCAGGCAGGCAGAGATAAGAGCTTAGAGCTGAGAAGTTG
```

```
GCGGAGCATCCCACATCAGCAGGCAGTCCTCTCTGCATTAATGGGAGATGGTCTGGGGATGAGGGACTGGCTCTCAAGTC
CATGTTCCATGGTTCTGTGGCTAATAGCCTCAGCCTGCCTTTCCCATTTCAGGGCCACTTTGACTCTAGAATGACGAGAT
AGAAAACTGTCCTTACTGCTTCTTCATCCTACCCTTCTGTCCCCCAGGCCTCTGCCTTCTTCCTCCCAGCAATATCTGCC
TAGATTCCCACTTTCTGTAGCAGAAAGTGGGTGAGCACCAGAGACTCACATTATACTGTCAAAGATCACGGCTTCTCTGG
GCCTCGGCTTCCCTGCCTGTGAGTGGAAAGAACCAGACCCATCTCTTGTAAGATTTGTTGCAAAAAAACAGGGCTACAAA
TGTACAATCAGTACGCTGCACCTTTTGGGGGGNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNCCCCCCGCCATCCTGTC
CTCCCTCTCCTGTCCTCTTTCCTCTTTCCTCCCCTCCTCTTCAATTCCACTCTTTCCTTTTGCTTTTTCACACAGGGTTT
TACTATGTAGCCCAGGCTGGCCTCAAGCTCATGGTCTCCTCACCTCAGCCTCCCATGTGCTGAGATGACGACCAGGAGCT
GCCATGTTTGGCAAGCAACTTGCTTGTTTTGAGTCTCATTTGCAGTAAGGTGAGGATGAAGATACTTGGCTCTCTAAGAG
GCACTGTGGGGAGCGAAGGGAATAGCCACAGGTCGCTAAGGCTCAGGAGAAGTGGCCTTCATCCTGATCTGCCCCAGATC
TTCTCAGTCTCCCACTTTGGCAGTGCCCTAGCATAGATCAGATGGGTGGTTAGGACTCAGTGTCGGCCTGGTTCCGTCTG
AGCGCCTTCAGCTGTGTGAATTTGGATGGGTGTGTTTACCCTGCCCCCTGGTTTCCTTTAGTTTTAAAGCATGAGTAACA
ATGGCATCTTGAGAGCCGTGAGACATTAAGTGCTTCCTTCCCAGTCCTGTAAGTGCTGTGCTGCTAGTGGTACCATTTCT
GGAACCTTCTTTGGCAGAGTAGCAACAAACTTATCCCAGAGAAACAGTCTGCCCCATCTCAAAATGTCATGTCATCAGGA
AGGCTTCAAACCATAGATAGCCCATGCCCTCAGATATTCCACTGACAGGGTCTTGGTTGCTGTTCTGAGGATTGTCCTGA
CTCAATCCTGCCTTCTAAGACCTGGGGCCATCCTGTCAGACTCTTGCCTGGGTGTCTCTTGAAAACCAATCAGTTTTATT
CAAGGGTCACCTCCTTTGGGACTGTCTTCCTCAGAGTTCCATCTGGGGTCCTTGGGTGCGCAGACATTCTTGTCTGGAGA
GGATGGCAGTAAGATAGTGGACAGACAGACTGCACATGATGAGCAGTCTTTGCCATCTACGGTGTCTGATCCTCCTTTTC
CCAGGAGGACACTGTTGACCTTGACCTTCCAGAACTCCCATGGCCCATTAGACAGATGTTACTTGTCTGCAAGAGAAGTC
TCTCAGTGGTTTATGGTTTGGGTTCATTACTCGCAGATGCTCAGCAGTAGCGGGAATGCAGCCCATTTTGACACTATCTT
AGGGTCATCAGATTTCCTGGGTTCTGCAGTCTTTGAGAGAGATGACTTCACAGTTATATTACAGNNNNNNNNNNNNNNNNN
NNNNGAAGAGGCAGGCAGCCTTTCTTGATAAGCCAGCAAAAGGGTAGATAGAGCTCATGTATATGTCTCTCTGTTGCTGC
CAGCCACAGGTCCCCATGCCTGTCAGCCCAGGTTTGTCACCCCGTCTGCAAGAGTCACTAGAGGGGGTCCCAAGAGTGCA
CACCTCCTACCAGTTGCTTCCCCCACATGGTAACCCCAAAGATGCACCCAGATAGGTAAAAGTTCAGGCTTGCCATGGCC
TCTGGTGGGACCCTGGCATTCCTTCTAGCTGGACCCATGTCCCTAGCTGTACCATGATGGTGATAGACGGGGCCCCAGAT
GTCAAATCATATATGGCTTCTAAAGCCCCCGTGCCCAGCCCCCAGCCCTACCTCACAGCCCCCACTCAACCAGACAAAGG
TGGGCGGCTGGCGCCGCTGTTTGCCTCTGCCAGTCTGACTCCCTGAGGCGTGTGGGCTGCACGGTGGGCCCCTGCTAATG
AGCATCTGTTATCACTCCATTATGCCGCCTAAGCCACCTGCACCCCAGCACTTAGTGGGAGACCCCGGGGGGGTTCCCTG
GCAGCTGGGCAGAGGGGCATGCAGAAGGCAGGAGGTGTTGGGGGGGGCAAGGTCTTCCGGGGCTAGGGGGCCCCATGCTCT
GTGGGATTTCTACAAAGTACTTAGGATTCCAGCTTTTTATTGTATCAAAAGAGAAGTGCTGGAGATGGGCGGGGTGTAAT
CGCCACACTAGGGTGGATGAGGCAGATCAGTTTTGAGTTCCAGGCCAGCCTGGGCTATGTGGAGAGATGCTGTCTTGAAA
GAAAAGGGGGAGGGAAAAGGGGGAGGAGGTGGTGGCAGTCTAGGGGGATTTTGGGTGAGGAAACGGGTGTCTGTGAATTC
CCAAGTGAGTCTCCCCAGCCAACATCTCACAAACGCATCTGTGCTGTGTATGTATTGAGAAGGGGATGTGGTTGCACATG
AGGGCACAGCCAAGATTCCGGTCCTAGAGACTGGCAGTGAGTGACCTGGCATAGTGTTCTTCGTAAGATGGAACATGACC
TACCCATTCTTCGTACGGGGCTGTACTGTCTTGTGGGGTAGTCCCTAAACCTCTCTGGGCTTCCCTTGCCTGCCTTGGTA
CCAAGGAGATAATGCTTCTCTGCTTGATCAGAGACAGCTGGACCAATTCTGGTATCTGAGCAGCTTCCACTCAAACCTCA
GGACAGGACCCAGCGAGGGGCGGCATAGACCGGGCACAAGTGTGGAACAGTTGGCAGCAGCTGACATGGCCTTTTGGGGA
ATGCTAGTCACCTCAGACCATGCCTGNNNNNNNNNNNNNNNNNNNNNNNNNNACAGCTGGGTCATGCAGAACCTGGGCCTGATTCC
CGTCTGTTGTATCATCACCTGGGCACGTGCCCAAATGGCTTGGGTTTGGCCAAAAGCCTGTCTCCGCCATAGCCTCCATTC
CACTGCTCAAAGCCCTCATTCCATCTCTGGTACTTCTGAGGCTGAAATTTTCAATCCATGCTTCAGAGATGATGAAGTAG
GTTGAAGGTTTTTGGACAGTCCATGGTGACAGAGACCTGGACTCTGGTCACTTACCTGTGATCAGACATGTTCCCGTGTT
GTCGAAATGGCTGTTTGCACGGGAGGTAGTTAGCTCTCCATCCTTGCAAGTAGTTAAGTTGGTCTTGAAGGGACCTGAGG
GGTCAAGAGGCTCTTCTTCACAGCTGTTTCCAGCCATGAGCTTTTCCATCCCTAGAGGGGTGGCACCAAATGACCACATC
CTTTAGTGTTGTCAAGTGTTACGGAGCACATGGGTAGTAAGTAGCACATGGTTGTTCTAGTGTATCAGGTGATGTCTGTG
AGTGATGTCTCCCCTACTGCCTTGATGGTCATTGCACCTAGCTCCCCCATTGGTGTGTCTCACTACACCTTCAGGGGTGA
GGAGCTGGGGGTGAGCGAGGTTGTTTTGTTCAGAAAAGGCACACACCTATCTGCAGGGTCTGGGGTCTCTTGATCTTCCT
AAAGCCCTGAATCTAGGCAGGGTGTCCACGCTGCCTGCTCTTCTTCCCCAACACTGGCTATGGTATCACCTTGTGGGCAG
TGGGCAGGCCAATAAGGAACCTTCTTGCCAGACTCCTGCGAGCTGTACTTATCCTAGCTAGTAGAGAAGCTTGCCCCACC
CTCTCGGGATCCCTGCTGGTAGGGTCTGGCAGAGGAGGAGCCATCACCAGCTGTGATTGGGAAAGACACTTGGCATGTCT
CTCTAGAGACCCTGCTGTGGGTTTCAACACAGGCAGGTGTCCCATCTCTGTCTCCCTACTAAGGGAAAGCTTCAATAAAC
ATGATCTGCAAAGACAGTGGTGTTCACACAGCACATGGGTAGAGGGTTGGTCTGGCACAGCCCCTCTGCTGTGAGGCT
GTGGGGTTTCGGTAAAACCTCTGTGAGCCTCCTCCATCTTCACCTCTGCCAAATGACAGGGGCGGCTAGCACTCTACACC
GTGGCCCTGCCCATGCAAGGGTGACGTGCTACAAGAGCACCGTAGAGATAGAGATGAAGGAGTCAGGTTGGATTGGGATC
CAGTTTTACCAGCTCCATGGCCCTAGGCAAATAGTTTCTTTCTAAGCCTGTTTCCTCTGCCGGCCCATGCGATTAGAAAG
AAAACCCCTCCTCACAGATGACAGTGGAAGTGTGGGGGCGGGTGCACATCATGGGGCTCACAGGTGTGTGGTCCCTGCTG
TGACTTAACTCTGTGTGCCCATCCTAGTCTTCAGAGGCACCTGCCCTGGGTTCTCAGTTCTCAGGCTGTGCATGGTCTAG
GGATCCCTGCACTGCCCTAACACCCAGCAGAGGCATGCGATCTCCTAATCTAGTCAGAGGCTAGCGAGAGCCCAGTGCCC
TGTTCTTGAGCAGATGTCTCTTAGCCTGAATCTACAGTATCAGGAATCAGCTGTTGGGGAGACAGAGCCTGTGGCCTGGCT
TTGAGCCTCAATTTTTTTCATTTGTTCATGTGTTCAACTAGCTTCACTGGGCAGCTGCTCTGGTCTTACACTGTACCAGC
```

```
AGGGGTGTCAGAGGTGCTAAGACAGACAGACTGAAAGACAGACAGACGGACAGACGGACAGACAGACAGATGGACGGACA
GGGTTAGAGAGACAGCTCGGTGGTTAAGAGCCAACATTGCTCTATCAGGGGACTTTGGTTCCCAGCACCCACGTCGGGGA
GCTTACTCTAGTCCACCTTTAACCCCAGACATAGAGATCTTCTACCTCTGGCCCCACAGCACCGGTGCTCATAGGCACAG
ACACACTCAGAGAATAACAAGACAGCAAAGGCCACTGAAGAATAGCAAACAAGCTAAATGCAAGGCCACTTCAGAAATGC
CCTAGGGCTCTCAAAATAGAACAGGTCACTAGAGACACAGAAGTCACTTTGAATTGGGGAGTCACAGATGGGATTTCAAG
AGAGAGGACATTTAAATGAAGGCCCAAGAAGGCCAGTCGGAGGGGAGGTCTTAGAAAGTACCCATAATGGGGGAGGGGAG
CAGGTGCAAAAGCCCTGGGGTGCACCTGGACTTGGTGAGTGTGAGGAGGCAGGGTGACAGGTGGGACCAGGGTCACTGG
CTTGTGTGGACAGCTGTATTGCTCTGTAAGCATCGGAGAAGCTCTGTGAGAACAGAGTTGGTTCTATCCCAGGCTGTGGT
TTCTGAGCAGTCGTTAGCTCTGCCAGGTTTCTCATGGGTGAAGATGGGTAGGGGTCTGCAGATGGGAATGTGTGGCCGTG
CCCTGGTTTCTTGCTGTAGTGAGTGAGAACTGAGTGTGTGCGAAGAGTCTGAGTGCCCTCCTTCTGGGACCTCAGCACCC
TGCACCAGCTTCTCTGAGGTCTTAGGGTCCTTTGATCCAATATGGCTGCCTTCCCAGCTAGTGGGCCTTGGAGCTCTCTC
TGGAGGCAGGGGAAGGTGTCCCCACCCCACCCCCACCCCTGCCCAGGACAGCAGCTCCTTTCTTTGCTTTCTTTTCCTCT
TCCTCCAGACCCAAAATAGAGCCTGAATGGTCCCAGGCTCTGGCAGTGAGACAATAGTGGCCTGCTGACCGAGGCAGCCT
GCTGGCCGGGGTCCATGGGCTGGAGGCACTAGGCTAGTATGCCTGGGGCTCCAAGCTCAGCTGCCTTTCCCAGGGCCCAG
GCCAGCCCTTTCACCTGAGGCGTGAGATCTACTGGAGTCAGTGGTGCTCTGGGGAAAGGCTTGCCACAGCGCATCAGAGA
GCTGGGTGGGACCCTGTGCCTCAGCCTTCCCAGCCTGACTCCGTCCTTCTCTGCTGCCAACTGGAGCTGGAGGCCTTCAG
ATACCTCTCTCTGTTCTTGCTCAAGTTTGGAGCTCAGAGGTGACCCCACGTGATGAAGATCTTTCCCTTTTGACTCACTG
GGTAGAAAAGTGTCACAGAGGTAAGAGCTGTGCTCGCTTAGAAAGGAGTGAGTGCTCTGTCTAGAAATCAAATCAAGCTT
GGATGAGATGGGTTCTTAAGCGTCTCAGAGATCGGTGTTTGAGTTTCATACCCTGCCCTTCCCAGCTATGTAGCCTTGAG
CAATCATATTCCCTCCCCAGACTCTAGAGTCCTGTCTCAGCATGGGCTCAGCCAGGCTTACCACACAGAGCGGTAGAAAT
GAGCTTCAAAAATTCCCGGTGCTAGGGGCAAGGCTTCAGAAGCCTTGACTCTGTCCTGCTCTCCTCCTGGGTAGGTAAAG
AGGGGCCCAGAAACTTCCTGCCATAGCCTGGGAGCACTAGGATAAGCAGGAGGAGCCTCAGGCCTGGGCTGGCTCACTAA
GGGGATTATGAGTGAGCTGTGGCAATCTGGGAAGGCTTCCAGGAGGAGACAGACTCGGCGTCTTCAAGGAAGAATGGCAT
ACACTCTGTTCACTGCAGCCAATGGTAGGGATTGGAGGCTCTGCCACCTGGCTTGTGACCAAGGCCATGGGGAGGCAGGC
AGCAAGTGGCAGCCTCTAACAAGTATTCATTTGGTCAAGGGTCTCGCTCCATCCTGTTCTCAAACCACTTGGTGTTAAA
GTCACTCTGAGGGCTAGAAGTGGTGGCACACATCTGTGATCTCTGTACTTGAGACAGAGGCAGGAAGATAGCAAGTTCTC
GCTCCCGTTAACCCTGTATCCCCTCCCCGTTCCCCTGATACTGACGTATAGCTAATGGTGGGGTGGGGTGTTATTGGGGA
CCCTAGGAAAGGGGTATCGCACATAGCTGGTCCTCTAAGAAGACACAGACAGCCCACTTGCCTGGACTACATTGCAAGAC
CTTCTTCCCTCAAAGGGAAAAAAAAGTCACTGGGAGATTTCATGCAAAAATCTACCCTTCTGCTTCTCTAGAGAACTTG
GGTGCTCTGAGATTTTGGGGTCTCCCCAAGACAGATCCCTTAAGCTCTAGGACCCGTTCTCTCCGAGTGTGAACCCAGCT
TGTCCCTGCTAGTAACCTGCTTTAGCCCCAGGCAGCCTCAGGACGAAAGCTCTCACGCTTGGGTACAGACAAGTTGGAGA
GCCCGCGCTGTAAGCCAGGCAGTGACGCAGTCCATGCAGCTGCCAACGGTTGCCAGGGGAACGGTTGCCAGGGGCTGCTG
TCACCTGCGCCCCTTCCTCCCCCTTGGCGCTGGAGGCCCGGGCTTCTCAGCTCAGCTTCCCGCCTGCCCCCTTTGTTTGA
AGCCCTAGTAAGGGGATGGTGACTGGGGGGGGGGGGGTTAGGGGGACCCAGGCTTTCATTCGACCTTCAGCCCCCTGTACT
AAGAGGAAAGTGTCCCTTACTCCAGTAGTCTGCTGCCTGCAAACAGGGAGGCTCCGTCCAGTCACTTTTGCCTGGAGGAG
AAAACCAGGTAGATTCTTGCTCCTACTCCTTTTTGGCTTCTTTTTCCTCCCATCTCTCCCTTCCCACCATCTCCTTTCCT
TCTCCACAGCCTCCTTTCTTCCCTCTCTTTCTTGTTATCCTGAATCCTCCCTCCCCCCTCCTCTGACACTGACATGTAGT
TAATGGTGGGTTGTCAGGACCCTGGGGGCCATCATAGCTGGCCCTCTAAGGGGACGCAAACAGCCTGGAGAGCCATCAGG
AAGTGGTTAGGGGTGGCCACACTCCCTACCTCTCCAGCTGAACATTTTTGAGTCTCAGCTGTCACAGCATAGGTCTCAGA
GAAGGGTAGTGGGAGATAACTAGACAAGGAGAATTGGAAATACCACAGCCTTGGGAAGAGCCAGAGAGGAGCTGAGATCC
ATTGTCACCTGGGTAAGTTCATGCAGACCCTTGGACTGGGGTCTTGCGCAGATGGCAGTCTGCTTCACCCAAGGAGACCA
TTTCACTATGACTTCTAAAGAGAGGCTCTCACTAGCAGGTAAGCTTTCCATTCAAGAGCGGGTCTCTTCTGGAGCTAGCC
TGGACTCCCCATGCAAGCTTCATTCTTTGCTGTGTGACCCTAGTGGGTGACTTGGCTTCCCGGGGGCTTCTCATTTGTC
CGTTCAGTGGGTCATGGGCTTTTTCTCATGGGTTTTAAAGAAGATTAAATGTGTTAATGGGGGCTCAGGCCTGTAATATG
AACACTTGGGAGGCTGAGGCAGGAGGATGAGAAGTTCATTGCCAGGCTGGGCTACATAGAGAGACACCATCTCAAAACAC
AAGCACATAAATGTATTAATTGCTCAAAGAGCTTTGAGTCCTGCCCTGCCTTTCTGAAGTATTTTTATTATATTGCTGGA
GCTATATTTTATAAGTGTGGCTGTATTCCTGAAGGGGCAGGCACCTGCTTGTCACAGAGGAGCTGAGAGAACCTTTGTGT
GCTTGGGGATGGGGGGGGGCTCTGGTTAGTGCCTCCGGTCTCCTCCATAGTTGGGAGCTGCCTGGCTTTGTGTCCCTGAGC
TGGCAGCCTCTGGGCCAGGAAAGAGGGGGAGAAAAGGGAGGTTTGGTAGCTGACCTTGGGAACAGAAAGATCCTGCAGTC
CACAGACGGGCATTCTGCCTGGGCCTTGGGGAACCCAGTGCATCCCCCAGAAAGCCAGCGGGGTGGCCTTGGGCAACTCA
AGAAAGCAGATAGAAAAAGGGGAACTTAGAGGGGACACAGAGACTGCTGTAGTCTGAAAGGAAGAAAGTCTGGACAGAT
CTGTGCGTCTTCTTTGTCCCTCACTGCCTGCTCAAAGGTAGGTTTTTCCCTAATGTACATACCCTAAATTGGCCAAGGAT
GGGGGCCATAGTCTCCCATTGCAGAGGACGGGGTCATAGTTACCCATTGACCAAGCAGGAAACTGAGACCCACTGAGATG
CAGGCTAGCTCAAGCTTCCAGGTTTAGAGACCCACTGAGAGGCAGGCTAGCTCAAGCTTCCAGGTTTAGGCAGCCTCTCT
GTAGGTGCCACCTGGCTCTGAGAGGCAGATGCTGACCCTGTGTACCGATTCGAAAGGCTGACCACAGTGGCTCATTGAAT
ACAACTGTGTGACTGTGACAAAAATGAGAGTCTGGGGTTTTTGATGCTCAAAGCCCCGTGGAATCTTGGGGCCAACTGGA
GAGTCAGAGAAACTACGGGAGAGGGAGTGGGGCAATGCCCGAGAGCTGGATCGCTCTAGTGGACTGGATTACTTCCGGT
CAGGTACGACACTATTGTTTCCCCATGAGCTGCTGTTGCAGGTGAAGGGAGGCAGGATCGTCCTGGATGCACCCCAAGCT
CACGGGGGACCCGTCGCCTGGAAGTGTCAGAGATGGTGATGAGGGCCTGGGGAACACTGCAACCTAAATGGCTTCAGTGG
CTCATCTTGTGCTTGCTAATAGCCTTGGGGGACATCTCAGAAAAGAAAACAAGGGACGGGAGACAGAATATGTATGTGCA
CATACATGTGTGCACTTACATGAAGACGTGCTGATAGGCTACGCTCTCTGTGGTGCCAGGGCATCTGAGAATTTGTTTCT
```

```
CACATGAGTGATGGGTGTCTATGCAGAAAAGGGCCTGCCACGTTGGTTTAGGGAATGGCCCCCGTAAGTTTTGTGCTGTT
TCTACTTGTGGCACTTACCAGAGTCTTTGCTGTGCTAAGGCCCAAAGTAGGGTCCAAAGGTCTCTCTAATCAGAGGATAA
GTAGCAAGTCCCCCCACCGTAAGGTCAGGGAGAGGGAGGGAGGGGAGGGATGCAGTAACCGCGGTGGCTGAAACCATTGC
TTTGAGGTTAGAAGAACTAGGTTTGAATCACAGCGTGGCTCCTTCTTTGCTTTGTGATCTTGGCTGCGATGTAAGACCTT
TGCATCTAATGCATCGCTTTAGAATTAGTGGGAAGTGAGTGGGCTACAGAGTTGAGACCCTGGGCTGCCCTGTGGCAAGG
CTGCTGCTGGTGCTGGCACAGAGTGCTTGCTGGAGGATACCTGGATTCCAGAGTCCCTTCCATTGTTCTGGATAAATCCC
TGCTGCCTCCTAGCTACCATGATGCTGTAAAGGAAGCCTGAGCCAGGGACCGTGGATGTGTTACAAGTTAGCACCACAGG
AAGGGCCTGTGGGCAAGGTCAGTTAAGGAAGTAACCAAGGCTAGTTCACTCCTGCTTGGTGATGATCCCTAGCCTTCCTA
GCCGCCCCCCTCCCCAATGTGCATGCCTAGTTGTAGGAAGAGGAAGCTGCAGAATGGTCAGCCACTGAGGCCTTCTAAGG
GCACCTTTTGTACCCACAGGTCTTCTGCTGGGCTGGCGCTCAATGCCCACAGCTCCCCTCCCTCAGTTTCCCCATGGGGC
AGGGCTTGGCAGAGGGTATTGCAGCTCATGGATGTTGGCAGGAAATCACAAGAAGGTGAGACCTGTGCTAGCTGAGTAGT
CCTATATAGCAAGGCTGGGTCTAGATGGTCCTACCTACAGCCATGCTCAGTGTGGTAGCTCAGGCCTATAAACCTGGTGC
CTGACAGACTGAGGCAGGGGGATTGCTATGAGTTCAAGGCCAGCCTGGGCCAAGTGCGAGACAAACAAAAACAAACAAAC
AAACAAACAACACAGAAAGTGAGCCTTGCCTTAGTGGGTAAGACCTGGGAACCTGCCAGCCTCTAGGACAGGTGCCAACT
TGATTTTTATTATCATGCAGTCTAGACAGATTCCTGAAGCCTGAAGTCACGGTGCTGCCCTGTGACCCCAGTGCTCCCTC
TCAGTGAGGGGTAGGTTCCGAAGGGGTCAGCTTGCTTGGGTGTCAGTCTCCCAACTGTGGAGTGGGGGGCAGATGCAGAA
GGCAGAGCCCAGCATTTACCCAGAAGCTGGGGACATAGCCCAGTGAGCCCTGGCTGAGTGGGGAAGGGTAATGCAGGTCT
CCTGGGTCTGGTAAACAGCTCCTTTGAAGATGTGGCTATGTGGTGCTGATAAGTGAGGAGTTGTACTGATGAGCCAAGCC
TGGAAGGATCCCCTTAGCCCTGTGCCCCACTCCTACTCTAGCCTTGGATGTGTGTTTGAGCCACCTCCTCCGCAGAGATT
GGAGTCCACGCCAGCCATCCTCCCGTTCCTCTTTACCCAGTGCTCACTGCAAGTGCACACTCATGGGCTCCAGGACACAC
ACACACTTGCAGCTCTGCACGTTCATACCGTGAAGGCTCCTATCATCTCCCATCCTCTCCCCGAGTTTCCATAGCTCCCA
AGGAACACATTCTACAACTGTACAAAGTAGCTGATGGTAAGCTGAGGCCACTTCAGTTTCCCCAGATAGGCCCTGGGTCG
GTCACAGGAGACTCAGACCTGGAAACTAGTTAGCTCCTGCCTTCCTTGATCTGGCTTTATCTCCATCTCTTGCCGGAGGC
CCCTCAGATGACCAGCTCCCTGCCAAATACTGGGTAGGCCATATATCATGTCGTGGGGAAGGGACAGGGATCAGGAGAGGC
AGGCTGCCTTTGTCTCTTGTCCACATTTTGTTCTTTCACAGCTGAGTAAATCCAGCAGACCCTTGCCTGGCTCCTGGGTA
CCCCCTGGCCCCCATGGGTGGCCTGTCAATCACTGGATACTGCCAAAGCCAGACTCTGGGCCACACCTGAGCTGTGGTGC
AAGGATCCAAGCCATGTCCTTCAAAGTATCTTTCCTGGGGTTGGTCCCCATACACTAGCAGTTTGGGGCAACTCTGATGA
AGCCAGCCCTTATCAGGGAGGCTCACAGGGCCGTATATGGCTGGGAGGAAGTTGGATACTCCTGGTGCCTTGGGCTTTGA
GCAGAAGGTCACAGTGGAAATCTGTCATTCAGAACTCAAGAACACATCCCAGCAAAGACCTAAAGCTATGACAGATGCCC
TTAGGATAGCTAATTTGTAACCGTAGTGGGTGGAAAGGGGTCCCGCTCAACATGTGAGGTGACAGCCCAGGAAGGGTTAA
TTTAGAGCTCTCTTAGTCCCGTTCAACTTCCACCTCCATCTAGGAGGAAATGGGTTTTCCAGGAGCTGGTGGCGTCTAGG
TGACGCAGGTCCACATAGGATGGCAGGGGAAGTGACTCAGGCCTCGGGAGAGAAAGTGAGCTCTAATATTGCTGCTGCCC
ACCATGCTGGCCCTTTCCCTAGCAACCAATCATGCTAGGAATGGTGGGGGCTCACACAGCCTTAGCCCCCCTCTATCTTG
TGGAATCCCTGGGGACCCTGTGATTTTATCCATTGTATCAGGCTGGCTCAGCGTGTTCTGTTCTTAGCATGTTTACGGGA
AGCTGAGGTCAAGAATAACAGCCACTCTGAAAGGGGTTGTCAGTCCAGCAACTTCTGTCTAGGCTGGACTTGCCTGCTTT
GTGCCCGTGACCTCTGTAGCTCCTGCTTTTCTCTCTTTGGGGGACCCTAAGCCAGGGGGGTGTGTACCTCAGGAACTTTT
GAGGAGAGGGCTTGGGAGCAGCCTTGTGTGCTCTTGTGAGAGATGGCCTTTTTCTGGCCTCAGTTTCTGCATCTGTAAAA
TGGTAAACCCGGAACAGTACTCTGCAGATGTGGTTGTGACTATTGGGGTGGGGCTGCTAACATACCTTCTCAGTAACCAT
TGGCTCTGGCAGTGCTGTGGCAGGTGGCATTTGGTGGCTGAAGCCGGTCTGCTTTGCCGTTCTTCATCTACACAGGCCAA
AGGTGGTCCCTAGGAGGTGGGCCTCTGCTGCTGGCTGCAGGGACTAGGGTAACCAGATCTTGGGATGCCTGCAGGAGGCA
CTAGGGTGCCATCCTATGATGTTGCTCTTACCCTTTGCAGTGTGAGGCTGGGTGGCAGGTGTGTGTGTGTGTGTGTGTGT
GTGTGTGTGTGTATGTGTGTGTGTGTGTGTATGTATATATATATGTAGGCCTGGGAGGGGGCTCAGGCAGGAAT
GTGCTGAATGGATAAATAGGACTCTTCCTGACCCATGTGGTGGTCCCTTAGCTATCTGAAGAGACCCCCCTCCCCAGTTG
TTCTCCTTGTCCAGCTTCTGGCAGCCTGTGACATTCAGGGCCAGCTAGGAGGGAAGTTCTCTGGGTGGGGTCACAGCCTT
CCCGATACTCTGTCCGGAAGGGAGGTTCATGTTCTAGGAATTTCCCCTCAAGTGCTCCGAAGGCCAAGCTGGCCACCTGC
AGTAGACAGAGGCCTAAGGAGGGGAGCATGGGATAGAGAGTTGGGGGGAAGGGTCTGAGTCAGGGACGGAAGTGCACTGG
TCGGAAGGTACACAGAGACAGTCTAACTTCAGTGGAACCCCATGTGCGCTCCCCACCCCCACCCCCAGTTCAAGGCCCTT
TTTGATGAGTGGTAGACTTAATTCTCACAGTGACAGCTGAGGTAGGTTTGATTTGTAATCCTCTTTACAAGAGAAGGAAA
TGAAGCCCAAATATGGTGTAGTTAACTTAAGAGCACACAGCTAGGAAAATAGAGGGGNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNCA
ACCATCCTCCTCCTCCTTACCTCCCTCCTCCTCCTCCCCCTCCTCCCCTCCTTCTTCCTCTCCTTTTCCTCTTCTTCCTC
CTTCTCCTCCTCTTCCTCTTTCTCTTTCTCCTCTTTGTCTTCCTTCTCCTCTTTCTCTTCCTCCTCTTCCCTCTTCTTCT
TTCTTCACTTCCTCTTCTTCCTCCTCCTCAAACCCCAAGAATGCAGACAGGGGTGTTTATCCTCCTTAAAGTGACTCCAC
AAAGGTTCGAAGTGGAGGGGAAGTCCCTTGTTTGTGCTTGATCCCTTCCATGGGTTGCCATGGTGATCCAGCCTCACTGT
GCCCCGCCCATGAGGCGGAGCTTGCTCCCTTCCCTGCCTCTTTTGTTCTCAAGGCCATTTGGGGAGAGTCAGTGCAAGAC
AAATGGCTCTGTCCTGTCCTGGACCCTGTGCTGACAGTGGTGGCCCACTGAGAAGGCAAGAGCCCTTCCTAGTTAGCTGT
GTGACCACATGAAGTTGTTGGCTATCTCTGGGCTTCAGATCTAAAACGTCACAAAATGGAGATCGGATGGAAGTCTCAAG
TGATTTGTAAGCTTCAAGTTAAAGAGAGGAATCTGTCTGGAAGAAGAGAGATTTGGGTTAAAGGTTGAATGGACAGTGTC
AGGGACAGCAGAGAGCAGAAGGGAAGAGATGCTGGCCTTCTGCTGGCAGTTTGATGCAGGGAAACCCATGCCTCCATGGA
GGGAGCACGCTCTCCCCTACAACTTTCCTGTTCTCCGTGCAGTGCTCTGGCACCGGATGAGGACCGTGGCCTCTGAAATT
GACCTTCCCTTCTGCGTGTTTCAATGCTCCTCATGCCTGGGTGTATGGGACTGGCTCTCACTTGAAGGGCCCAGGGTGAC
```

```
CAGAATGAGACTGGTCCAGAAAGGAGACAGCAAGATCCTGCTTGAGCCACCCAGTGCTCAGTTTAACCTCTGCCTCTTCT
AGGAAGCCCTCCTAGTTTTCCCCCCAAATTCAGATTGCCTTTGCTCCCTAGGTTTCCAGCCTTCCCTTTGCCTAATGGTG
GAAGATGAGTGTCTGTCACTGTGGTCTGACACAATTCCCTACAGTCTGAGGGATGGTAGTCGGGGGTATGTCAGCACCCA
CTTGGCCACTGGGCCAGCTGGGAGGGCCAACAACGGAGGACATCGCCAGCGGTGGTATAGTTCATGTGGGTGTAGTGTAA
AGAAGCACCCAGCTAATAGTGACCCATGGAGTCAGTATGGAGAGATGGAGCTGACCCTCAGGGCGGGAGCACATAGACTA
AACCACACCTCTCTGCCACCAGCTTCTCAGGTCCTGGTGGCTGGCTCCCCTAGTGGTCATGACAGGAAACATCCTTCCC
TCCAGCACTTTTTATCACGTTCACCCTGCTGCTTTCCTGCTTCCGCCTCTGTACTAGGCTCTGTTTCCTGAATGAGGAGG
GACTGTGCCAGTTGATGGCGTGTGGATAAAAGATTCAGGCAGTGGGGCAAGGGGTGGGGTTATCAAATGTCATTGTGACT
GGTCCCAGGAGCAAGGTTCCTGTAACTACCTAGGCCTTAGGGACTGTGACACTTGGTTCCCAGGAAAAACTCAGATAGCC
CGTTCATTTCCCTGCATCTCCTCCAGGCCTGGCAGATGCAGGATAGTGTTCAGGTGGATCTAGACAAGGCCCCTCGTGGC
CCCTGGGCTCCTCTGTGAGCCACTTACACCTTGTGGCAGAGCAGCAAAAGGCAGAGCGTGTGGCCATGTCTATTTATAGC
CTCTTGATGCTCTGGAGAGAAGCCTGACCATTACTTTGTGACCTTTAGCTCACTCCCATCCATATACAGGCCTCCCGCTG
TGGCGCCGGCCCCTCCCCCATTCCCCTGTCCCCGTCCCCACCCCGGTCATAATGTTCTCACTCTCTTCCAGGTAGGAGTA
TAATGGCTGTCCAGGTGCTGCGGCAGATGGTCTACTTCCTACTGAGTCTGTTTTCTCTGGTGCAAGGTAGGCTTTCTTGG
GGCCCAGGGTTTGTGGTGACTTTGTCTAATGCCTCCATGTCTGTGTGAGGGCTCTCTGTGTAAAGGATGGGTCTGCAAAG
CAGCCCTGAGGGCACCTTGGGGGAAAACAGGCATCTCTTCCTTCTTAGATGCGGCTCCACAGAGCGAAACTTGGGATGGG
TGCAGTCCTCTCTTCCTTTGCAGGGAGTGTCCTTGGACAGTGCACACGTGTGTGTGCCACAGTCTAATACAATCATGGTC
TCATTAATTAAATAATTTGCATTAAACTTTGGCTTTTGACTTTGAAACAANNNNNNNNNNNNNNNNNNNNNTCCCCCCCCC
CCCCCACTCCCCTGCAAGACAGGCTTTCTCTGTGTAGCCCCGGCTGTCCTAGAACTCACTCTATAGACCAGGCTGGCCTC
GAACTCAGAAATCTACCTGCCTCTGCCTCCCAAGTGGTGGGATTAAAGGTGCACCACTGCCTGGTCTGAAACAAATTCTT
TATCTTCACTTTTATCTATTTATTTATTTATTTATTTATCTATCTATTTATCTATCTATTTATTTATTTGTTGGAAACCA
GTAAGGTCTAGCAAGATAGATACATTGTTATACATTGTTGTAGACTGATAGATACATTGTTGTAGACCAATAGATACATT
GTTGCAGACCCTTGAAATCTAGAGCCTTAGAATGTGTGTGAGTCCCAGGTCCTAGCCCCTGGCTGGTCCTGTTTATAGCC
TCTGCCTCCTCAGGTGCACACAGTGGCAGCCCCCGAGAAGACTTCCGCTTCTGTGGCCAGCGGAACCAGACCCAACAGAG
CACCCTCCACTATGATCAATCTTCAGAGCCTCACATCTTTGTGTGGAACACAGAGGAGACCCTCACAATTCGTGCCCCCT
TCCTGGCAGCCCCAGATATTCCCCGCTTCTTCCCAGAGCCTAGAGGGCTCTATCACTTCTGCCTCTACTGGAGTCGCCAC
ACTGGGAGACTCCACTTGCGCTATGGCAAGCATGACTACCTGCTTAGTAGCCAAGCCTCCAGACTCCTCTGCTTCCAGAA
ACAGGAGCAGAGCCTGAAGCAGGGAGCCCCGCTGATCGCCACCTCTGTCAGCTCCTGGCAGATTCCCCAGAACACCAGCC
TGCCTGGGGCTCCGAGCTTCATCTTCTCCTTCCACAGTAAGGCTGGGGGGACATATGAGTGTGGGGTGGGAGTCACGGAA
GACATGGAAAGCCCATGGAGGAGGACCTGGGTGCTGATGGCAGCTATTGTCACCTGACCTGGGTATCCTCTAGGAGGATA
GGTGGCTGGGGAGGGGAGGATTGGACTGTTTGCCTGTCCCTGTTATGATCTGAGATCTTTGTGATGACCATAGCATCAC
TTCAGCCATTAGGAGAGCATCTTAGCTACTTTTCCTCTCTATGATAAAACACTCTGCCAAAAACAACTTAAGGGAGAAAG
GTTTCCTTTGGCTCGCAGTTCTACGGCACACTCCATCATGGTGGGGGAAGTCCTGATGGCAGGTGCTTGAAGCAACTGGT
CATTAGCATTTGTAGTCAGGAAGCAGACAATGATAAATACTTCTGTTCAGCAAGCCTAGGGGATGGTGCTGCCCACTTTT
AGGGTGGATCTTCCTACCTCGGTTAGCCTAACATAGATCCCTCACAGACATGGCTCCTGTCAGGCGGATGAGCAATATTA
ATTATCACAGAGAACAAGGGATGGAGAGAAAGAGATTATTACTTACAGGTCCTGAGGTGCAGTGCACACCTGGAGGTCAT
GCTTATATACACACAAAGGTCAGGACGCAGCGGGAGGAGGCTAGGGGGGAACATAACTTGATACATACCTGGGAATGTAA
CACTAGGAAATGTGAGCTCTGGCAGAGCCACTCTCCAGCCAGAAGGCCCAGCTTCTATTCAAAGTACAGGGGCTGGAGGA
ATGGCTTAATGGTTAAGAGCATTTGCTGTTCTTGTAAAAGATCCAAGTTCAGTTCCCAGCAGCTACAATGGGTTGTCCCC
AAACATCTGTAACTCCAGTTCCAGGAGCCCCCACAACCTCCTCTGGTTTCTGTAGGAACCCAGTGTGTACACAATACGTG
TTTATACACACACACACACACACACACACACACATTAATTAATATTAAATCATTAAAAAACAAAACACAGGTAGAA
GCAACATAAACTGTGGGCATTCCGTGTACCCTAGTATCAGCAAAGAGGAAACTGGGGTACAGGGAGCTATGCAACTAACA
GATTGGGGCTTTAGGATGTGAAGCTAGCAGCTGATTAGCAGGGACAGGGAGAATTAAGGGTTGGGGAAGCAGGGAGGCTG
TCAGGGAGGCCTGGGGACCATGACCTTGTGGGTCAGGAAATGGATGCTTTCTCACTTGGGTCTCCTCCTAGAGACACCCA
TGTTTCCAGGGGCTCAGGTAGAGATGGCGAAGCTAGAGACTGGGAGATCCCCAAAGCTGGCACAATAACCCACATAATCA
CATAATTCCCGAGGAGATCTGCTTAGGCCTGGTCCCTCAGAGCCTTCTGTGCTCATGTGTAGCAGGCCCTAGGAGACACA
GCCCCACTTAGCTGGCTTCAAGTATCTGTGATCTGTGGCCCCTCTGCCTCTTGGGCCCTTGGGTCCCTCTTCTGTGGAGT
GCCATATGAGGGTTGCATAAGGCTGTATGCTGGCCGTGGGAACCTCAAGCAGTCTGTTGCCTTCCCAGATGCCCCACACA
AGGTCTCCCACAATGCATCTGTGGACATGTGTGATCTCAAGAAGGAATTGCAGCAGCTTAGCAGGTACCTGCAGCACCCT
CAAAAGGCTGCCAAGCGGCCCACCGCAGCGTTCATCAGCCAGTAAGTGTGGATTACCTAACTAGGGGATGATGGAAGGTC
TAACCTGGGCTAGGAGGAACTGGGAAATCTTCTCTGAGGCTGGACCTTCCTCATTGGCTCCTGCTGGGTTGTCAGTAAAG
ACAGATACTAATGCAATGCCCGGCCTCTTCCCTACAAGAGACTCCATCCTGGAGTCTGGCACGTTGAGTTCCCGAGAGAG
GTTTGGACATAGCATAAGTGGGACATAGGCATGAGCTGAATTCCAAAGTACTGTCCCTCGCTGCCACTCCCTTGAATCTG
CATCTCAGCTGCCTGTGTCCTTTGTCCTCAGCACCCTCTCCTCTCTGTTCCTGTCTATCCTGTGTATCTAGACCATCAC
CACTACCCCCTTCCCTCTGTCACCTGCCATGTTCTCCCCCACACCAGGCAGTTACAGAGCCTGGAGTCAAAGCTGACCTC
TGTGAGCTTCCTGGGAGACACATTATCCTTTGAGGAGGACCGGGTCAATGCTACAGTGTGGAAGCTGCCACCCACACCG
GTCTAGAGGATCTGCATATCCACTCCCAGAAGGGTCAGTGCCCTAGGGTGGGGTGGGCTAGAAGGGCAGGCAGGAGCA
CTCCCTACAGCATAGCGGTAACCCTTGTTTTCCCCTGGGTCTGGGGACAATCCACAGCCCACCGGGAGAGCTGGTGTCTG
AAGATCCAGGTTGCAAGACCCCACTGAAGACCTCTTAGCCTTTGCTCCTGCCACCCGACTCCCTGGTCTCTTGTGGGGTG
GCCCAGAGCCCAGTGTTTAGAAGGGGGTTGTTTGGCGTGAGGGGGGAATCTGGGGTGTTTGCTGGGCCGGGAACTGAGGA
TCCTCGATATGCAGGAGGAGCAGAGTGAGGTCCAGGCATACTCGCTGTTGCTTCCCCGGGCCGTATTCCAGCAGACCAGA
```

```
GGCCGTCGCCGGGATGACGCCAAGAGGCTCCTGGTAGTAGACTTCAGCAGCCAAGCTTTGTTCCAGGTACGGAGTCTCCT
CTCACCTGCTCCTAGGGTAACTGATAATACACACACACACACACACACACACACACACACACACACACACATATGCAA
ATATATGTATATGGGTATATATATACATATATGTGTATATATGTATGTGTATGTATATATATGTTTACATGCATATAT
ACATATGGGTATATACATACATACATACACACACACACACACACACACACACACACATATCTGACAGAGGGGTGGAAA
GGTGGTAGCTAACCTACTCCAGGAGCCAAATACAGGCAGGAAGGGCCCTCCAATGCACAGAGCTCCTAGGAGAGGTGTGA
ATAGTGCTGTTCTCATCCAGTGTGAATGACCAGCAAGACCCTCAGAGCAAAATGTTAGGAGCGACAGTAGTCTAGAAAGG
GTGGGCAGAGGCCTTCTTAGCTTTTCTTCTTCTCAGCCTTAAATCTCCAACCTGTCCCCCCTACCCCCAACCTCATCTAT
CTAGGACAAGAATTCTAGCCAAGTCCTGGGTGAGAAGGTCTTGGGTATTGTCGTGCAGAACACCAAAGTCACCAACCTCT
CAGATCCGGTGGTACTCACCTTCCAGCACCAGCCTCAGCCAGTAAGTGTGAGCAAGCCAACCCCAGGGCAAGGGTCCCTT
CCTGCTGGGTCACATGTCTGCTTCCTGTGTCTCCCAACCCACTTGTCTTTTATGGAAGGGTTGCCCAGCAGCCAGCTCCC
AGATTGGGGGCGGGGGTGGGGACTGCAAGGTGGGTAGTGAGGTGAATAGGAAGGTTAGGCCGTAGGAGGGAATGCGGAGG
GTGGGTGATAGAGGGAACATGAAAGATGAGCAAAGAGAACACAGAGTGGTCTATAGAGAGAAAACGCAGGGAGCTGGCTG
TGGAAAGGAACAGAGGCTGAGTTATGGTGGCCCATGCTTCTTGTCCCCACAGAAAAATGTGACTCTGCAGTGCGTGTTCT
GGGTTGAAGACCCGGCATGTGAGTAGGGCGGGGGTCTCTAGGGGAACAAGTATCTGGAGGAGGCATGGAGTTCTGTTGGG
GTAGGAGAGAGGAGAAGCCCCAGGGATGAATTTCTGGAGAAGATGGGCGTAGGGGAAAGGTGCGACGGAGGCCAGGGATC
TGCTGGTGGTGCAAAAGTTTGCACCAATGACCCAGTCTTGTGTGGAGTGCATGGTGGGTGGGGTCTTGAGGACTGGGGTT
GACCAGGGTCCTGTGCTTTCCCCTCAGCAAGCAGCACAGGGAGCTGGAGCAGTGCAGGCTGCGAGACAGTGAGCAGAGAC
ACACAGACATCCTGCCTGTGCAACCACCTGACCTACTTTGCAGTGCTGATGGTAGGGGTCTCCCCGAGATCCCCCTGCAG
TCCCGCCACAGGCCAAGCCTATGTCCTGTGTCATGTACTTGGAACCGGCTTCCTCATTTAATCCCAAATAACCTGCCAAG
ATAGCTCTGATCAATCACCCTATTTCAAAGGATGCTGAGGCTAAGTACTGTATATAAGGCTGCACAGTGTTGGTGTGTGA
CGAACGTGCAGATTCCTGCTCCCAACCCACAGGCCTGGGCCAGCAAACTGTTTCCTATAGGCCCCCAACTGGAAGCCACT
GAGATCGGCTCTTGCATTCCCCAAATATCAGAGGCTTTGTTGAAAATATGCTAAGTCAACAAAGGCATAGACTCTGCCCG
ACCCCAGCTCTGCTGAGCACAGTGGGTGAGCATCTGCTAGGTCTCAGGGCTGATGTAGCTTCAGTGGTCCTAGGAGATCT
CAAACTCTGAGCCGAGCTTCTACTGACAACTGGGGCTTGAGTGGACAATCCCCAGCTAAAGCTGCCCCCCTTAGGGCTTT
GTGGGGTTCAGGCCTGGCCTGAGCCTACTTCTTGGGCGAGGGTTCCTGTGGTGTGGGTCAGAGACTTACTGGCATGGGAG
GAAGACAAAAGGGTATTCATGTGGGAGGGGCTGGCTAGCTCCCAGCTGGCCAGGAACAGATGCTAGCCCCTTGGAGGGGA
CCGTGCTGAGATTGTCACTCTGTCCCCATCCAGGTGTCATCCACAGAGGTAGAAGCCACTCACAAACACTACCTCACGCT
CCTGTCCTACGTGGGCTGTGTCATCTCTGCTCTGGCTTGTGTCTTCACTATTGCTGCCTACCTCTGCTCCAGGTAGGCCA
AAGGAAGTAGAGTGGACCCATTTCATTCCTATAGACTAGGACTCGTACCGTGCAGGCTTGCTTCACCCAAAATATGTGCG
TTGTTGGGAAGCTATGGGTGGGCTCTCTGCTGACCACCACACTCTGTGGCCGTCGGACCCCAACAATAACATTAGTTAAT
CCAGCATTAGCACTGAGTATCTGCACAGTCAAAATCCAAACTGCAGNNNNNNNNNNNNNNNNNNNNNNNCGAGCTTCTACTGA
CAACTGGGGCTTGAGTGGACAATCCCCAGCTAAAGCTGCCCCCCTTAGGGCTTTGTGGGGTTCAGGTCTGGCCTGAGCCT
ACTTCTTGGGCGAGGGTTCCTGCAGTGTGGGTCAGAGACTTACTGGCATGGGAGGAAGACAAAAGGGTATTCATGTGGGA
TGGGCTGGCTAGCTCCCAGCTGGCCAGGAACAGATGCTAGCCCCTTGGAGGGGACCGTGCTGAGATTGTCACTCTGTCCC
CATCCAGGTGTCATCCACAGAGGTAGAAGCCACTCACAAACACTACCTCACGCTCCTGTCCTACGTGGGCTGTGTCATCT
CTGCTCTGGCTTGTGTCTTCACTATTGCTGCCTACCTCTGCTCCAGGTAGGCCAAAGGAAGTAGAGTGGACCCATTTCAT
TCCTATAGACTAGGACTCGTACCGTGCAGGCTTGCTTCACCCAAAATATGTGCGTTGTTGGGAAGCTATGGGTGGGCTCT
CTGCTGACCACCACACTCTGTGGCCGTCGGACCCCAACAATAACATTAGTTAATCCAGCATTAGCACTGAGTATCTGCAC A
AGTCAAAATCCAAACTGCAGGGCACACAGGGTAATAACACAATGATAATAAGAACTAGGGGACTTCTCATGGAGTGTCCA
CATGCCTCACACTGTTCAAAGAGGGCAGCAGACTTTGAGACCTCACAGGGAGGGAGTGACAGAAGTTGTCCAATTATCAG
TGTTTGGTATAGCCTCCATTAAGGTCTGCTAGAACCTGGGCCTGCCTGAGTGGAGGAGAGACACTCTTGGCTGTGGGTCT
TCAGTTGTCCTGAGGAAGGAGTAGGGGTGGAGCTGGGAAGGAGTAGGGGTGGAGCTGGGTACAGGTGGGCTGTAAGCAGG
TGCATCATAGCCAAGTGCATCCGCAGGTGCACACTCCCAAGGCTGGCCAGGTGCTTTGTGGAGGGTGAGGTAGGCAAAGC
ACTGAGCACAGGGCCAGGGGCATGGGAACTAGTAGGAAATGCAGAGCTTCCTGCACCAGACACTTTGCCAGATCCATCCT
CCTGCTGTGTAGAGGAGGAGGCATTCTGAGGTGGACTGGACTAGCCCAGGGCAGTACAGAAGTCAAGGATAGTAATGTCG
GGGCCAGTGTCTTCTGGTGAAATTCTGGAACTCCTTAGAATGCCGTGGTACAGGTACACACTGTGCTAGTGGGCAGATAT
CCACAGCTGCTCAGAGCCACATGGCCAGATAGCTATGGTGCAGACATGAAGGCCTCCTGGAAGAGGGGGGAGAGGGAAGGA
GGGAAGGGGGGGGGGAAGAGGAATATGAGAAAGGGGAACAGGACAGGGCCATGCCCCTGACTCTGCCCCTGCGCTGGCCT
GCTAGCCCATGCTCACGTTCTCCCTGTAGGAGGAAGTCACGTGACTACACCATCAAAGTCCACATGAACCTGCTGTCCGC
TGTCTTCCTGCTGGACGTGAGCTTCCTGCTCAGCGAGCCTGTGGCACTGACGGGCTCCGAAGCAGCCTGTCGCACCAGTG
CCATGTTCCTGCACTTCTCCCTGCTTGCCTGCCTCTCCTGGATGGGCCTCGAGGGCTACAATCTCTACCGACTGGTGGTG
GAGGTCTTCGGTACCTATGTGCCCGGCTATCTGCTCAAGCTGAGCATCGTGGGCTGGGGTAAGTGGCAGGGGGAGAGGGG
ACCCCAGAACCCACTGTCTCATCTACTACACACTAGGCTTCGTTCAGGCCAAGCACAGACAGCAGGTTCTGCCCTCCCCC
GTTAGGGAATCAAGCAGTTCCTCCAGCTGGACTGGGGGCATGGACTTTAGTAGAGACTCCAAGATCCCCCACAAACCTCC
GTGACACAGTGGCATTTTGTCTTGGCTGGTGATAACTTAGTTCACCTGTGACTCAGACTACTCAAGTCAGACTGAGGCAC
AGAGTTTCATGCATAATCATTTTACACAGTTNNNNNNNNNNNNNNNNNNNNNNNTATTCATGTGGGAGGGGCTGGCTAGCTCC
CAGCTGGCCAGGAACAGATGCTAGCCCCTTGGAGGGGACCGTGCTGAGATTGTCACTCTGTCCCCATCCAGGTGTCATCC
ACAGAGGTAGAAGCCACTCACAAACACTACCTCACGCTCCTGTCCTACGTGGGCTGTGTCATCTCTGCTCTGGCTTGTGT
CTTCACTATTGCTGCCTACCTCTGCTCCAGGTAGGCCAAAGGAAGTAGAGTGGATCCATTTCATTCCTATAGACTAGGAC
TCGTACCATGCAGGCTTGCTTCACCCAAAATATGTGCGTTGTTGGGAAGCTATGGGTGGGCTCTCTGCTGACCACCACAC
TCTGTGGCCGTCAGACCCCAACAATAACATTAGTTAATTCAGCATTAGCACTGAGTATCTGCACAGTCAAAATCCAAACT
```

```
GCAGGGCACACAGGGTAATAACACAATGATAATAATGGTAATGGAAACAGAACTAGGGGACTTCTCATGGAGTGTCCACA
TGCCTCACACTGTTCAAAGAGGGCAGCAGACTTTGAGACCTCACAGGGAGGGAGTGACAGAAGTTGTCCAATTATCAGTG
TTTGGTATAGCCTCCATTAAGGTCTGCTAGAACCTGGGCCTGCCTGAGTGGAGGAGAGACACTCTTGGCTGGGTCTTCAG
TTGTCCTGAGGAAGGAGTAGGGGTGGAGCTGGGTACAGGTGGGCTGTAAGCAGGTGCATCATAGCCAAGTGCATTCGCAG
GTGCACACTCCCAAGGCTGGCCAGGTGCTTTGTGGAGGCTGAGGTAGGCAAAGCACTGAGCACAGGGGCAGGGGCATGGG
AACTAGTAGGAAATGCAGAGCTTCCTGCACCAGACACTTTGCCAGATCCATCCTCCTGCTGTGTAGAGGAGGAGGCGTTC
TGAGGTGGACTGGACTAGCCCAGGGCAGTACAGAAGTCAAGGATAGTAATGGTGTGGGACCAGTGTCTTCTGGTGAAATT
CTGGAACTCCTTAGAATGCCATGGTACAGGTACACACTGTGCTAGTGGGCAGATATCCACAGCTGCTCAGAGCCACATGG
CCAGATAGCTATGGTGCAGACATGAAGGCCTCCTGGAAGAGGGGGAGAGGGAAGGAGGGAAGAGGGGGGAAAAAGAGGAG
GATGAGAAAGGGGAACAGGACAGGGCCATGCCCCTGATTCTGCCCCTGTGCTGGCCCGCTAGCCCATGCTCACGTTCTCC
CTGTAGGAGGAAGTCACGTGACTACACCATCAAAGTCCACATGAACCTGCTGTCCGCTGTCTTCCTGCTGGACGTGAGCT
TCCTGCTCAGCGAGCCTGTGGCACTGACGGGCTCCGAAGCAGCCTGTCGCACCAGTGCCATGTTCCTGCANNNNNNNNNN
NNNNNNNNNCTGACTGTGGCAGCCTTGGCTATGTGCTGTTCCCTCCCCCAATGAACTTTCTATTTCTACTTATAAGTGC
GTCAATAAGACCAAGGCAATGTCTTCTCTACTAGAGTTGTCTCCCTGCCCTCCCCACCAGCCCCATCATCCCCTCATCTC
CCTGACTGACCATACTTCCACAGTGTACCACAGGCCTTTTCTCCTGGGAGACCCAGGAAGGGACAGGGAAAAGTTGAAAG
AGAGTCTACCCATTATGGAAACAGAGGGGACCTACCCAGGCCCACGGGGATCAGAGTGGCGTTGGTGGATGTGAATAACTACGG
CCCCATTATCCTAGCTGTGCGCCGGACTCCGGAACATGTCACCTACCCCTCTATGTGAGTGGCTATGGCAAGGACAGGAA
TGTGGGTCTCGAGGGTCGGGCAGGGACAGCAGCCTTAATTGGCCAGGTTGTCTGGGAGCTATGGGCTCCAGTATGCTGCA
GGAATAGGCCCCACAATGGTCAGAGCCCGGGATTAAGGACCACAGGGACAAGGAAGCCAAGTCTGCCCTTTAGCAGTC
AGGTGCCTGTGCCCAGAGCCGGGCCTTTCTCTGAGCCTCAGAGGAGAAGTGTTGGGCTACATGGTCCCCCACCAGGCTTC
CTTCTCACATCCATTGGTTCTGAAGCAGATGGAAGTCTGTGGTGCAGAGAGCATGAACCACTTGTAACCACGCCGTGATT
ACTATGTGGCACCAGCTGGCAGGGACATCCTGAAACTAGACCTGAGGAGCCAGGGTGCTCAAGCCCCAGGTCAGGGAGG
AATGCTTTCCCCTGGCTCCACCCTGATGACATAGAACCCCAAATGTCCCCCTTCCCCCACGCCAGGCCTGGTTGAGGCTT
GCCCCAACCTTGTACTGTCAAGAACACGGCTTCTTTAACACCTTCCTTAAAGTTATCTGACTGGGTAACTGGATGCCAAA
GATGGCTCAGGGCTTAAGAGTGCGTACTCCTCCTGCAGAGGACCTGAGTTTGGTTCCCAGCACTCAAACCAGGCAGCTCA
CAACCATCTACAACTCCAGTTCCAAAAGATTGGATACCTCTGAGTTCTGTGTCCTCCGTGTGTACCAGCACACATGTGTA
CATATCTGCATAGAGGCACATACACATAATTAAAAATAATAAAAATAACTATATATTTTAAGAAACTAACTTTTAAGATG
ACAGGAGGCCAGTGGTGCTTGTCACTAGTGAATGGTCCCAACACCACACCCTGCACCCTCCAGGTCCCGCTGTTCACTCT
CCTGAGCTGAGCTGTTTCATTCTTGTCCAGGTGCTGGATCCGGGACTCCCTGGTGAGCTATGTCACCAACCTGGGCCTCT
TCAGTCTGGTGTTCCTGTTCAACCTGGCTATGCTGGCCACCATGGTGGTGCAGATCCTGCGGCTTCGCCCGCACAGCCAG
AACTGGCCCCACGTGCTGACCCTGCTGGGCCTCAGCCTGGTCCTTGGCCTCCCCTGGGCCTTGGTCTTCTTTTCCTTTGC
TTCCGGCACCTTCCAGCTTGTCATCCTCTACCTCTTCAGCATCATAACTTCCTTCCAAGGTAGGCATGATGAGGGCCCCG
CCCCTACTCAGTAGCCCTTGTCCCTCCAGCTGGTGGTAGGTAGGCTTTCCATATGTGGAGCGGGAGGGGGAGAGATGCCC
TAGGGACCCCCAGATGCTCCCTTCTCTGGGCCTCAGCTGTCACAGTCCAAAATGAGCACATGCAGGCCACACACAGGACA
AGCCTCTGCTGAGCACTTACTTCTGTGTCGGTCCTATGAGAAGCCCCTGGGTTCCAGCATGAGCTGACCACTCAGTTCTC
ATGAGGGGGACAGTAGAGTCAAGAAAGATTGAAGCCACCAAGGGGACTCAGAGTAGGAGAGTTGGCAATGACAAGTGTTG
CACCCGGGGCCCAGTGCAGACAGTGGTCTTGCGTCATCAGCGTCAGCATCACAGGGGACCTTGTTGGAAAGGCAAACTGT
GATCCACTCTGGCCCACCGATCCTTTCTGCATCTTAGCAAGATGCCCAGGGCATCCTGCCTATAACACCAGTCAGAGAGC
CCTGGNNNNNNNNNNNNNNNNNNNNNNNCCCCTCCGGGAGGACACATGCATGGCGTCCGCTCACGATGTCTGTGGCCCAGTGC
TGTGCCCACCCAGCCTTTGTTGGTTAGTGGCATACTAGAGAAGGCCCTGGTCCTTGAAGGCGTAGGGCTGTTGCTCTGGT
AGGTAGATACCTAGCTTGCCTTGGGGACGACTCTGGTCCTCAAAGGGCTCAGAAGCACACTGCCATTCTGTTTGTGGGGC
CGTTTCAGTCTGGAGCTAAGGCCTTGTCTTTCTGGCCACCTCTGGGTCCAGCTGTTGCTGCTGGGTGTTGAGACCTGCAG
ACCCAAGCTGGGGTTAGATCTCGAAGGAGGCTGACACATCCGGCCTGAGACACAGCTAACTGTCTTGACTTGCTGCTCTG
TCTCTGTGGTCACCATGCAGATCCCGAGGGTGGCACTGGGGGTAAATGTTCTGGGAGAAGGTTTGGAGGCAGAGCACCTT
AGGAGCTGAGCATCTCCCCCAGCCTTTCTGCAAACCCTCCTCTTCATTCCCCATCCCCAACCCCTCCTCTGTGTTCCCCT
AACCCTCCACCTGAAGCCTGGGTCCTAGACCAATGCTGTGATTTGGGGTGGTAGTTCCCAGCAGTTTCCTGGTGCCAGC
TATCAACTTCTGTCTGTTGTGTGGGCTTTGGCCTCTGACTCAGGGCAGGTTTCTGTCTGAGCCCTCTCTCCAAGCTGCCT
CACCTTTGCTCGCACCTCAGAGGGACCTCCATCTCTCCTGAAGCCTCCTCCCTCTGGCAAGTACTGGGATACAGCCACCC
TTTCAACCCAGCACTCTGAAGACCAAGACAGCCCCCTCTGGTGACACTGGCCAAGCTTGATCTTTTTCCTAAGAAGTGGT
CTTCAGATCCCTGCAGGTCGCTCAGAAGACACTGGGCTGCCTAGTGTGAATTCTGTCCTACTAACGTACAGTGAGCAGCT
CCTCACCCCCACCCCCGCAAAAGCTCTCACCAAGTCCTGGAGTGTCAGGCAGGGGGCTGGAAATCCAGGAGGACTTCCTG
CAAAAGGCAGCATTTCATCTTGACCTCAGCCTTCAGGTTGGGGAGAATGTTCTTTTTAAATACCAGTTCATTTGTCTTTT
GATATTAAAGCTCTTTATAGAGAGTCTGGAAACTGTAGGCGATTGTCGAGAAGAGAAATAAAAATGAGCTGTTATCTAAT
GCCATGGCAAAGCAGCACAGTTTGGAGAGTTGCCCGTCTCAGTTTTGTTTGGGTTTGTCTTTGTTTTTTTGTTTTTTTTT
CCTCCCACTTCTGTTACTAGTGTTGCTGATTCAGGAGTCTTGGGGATGATCAGGAAATGGCCAAGAGGGAAATGTGACAA
AGAAGGCATCTGAGGACACTTTCTCAGGAGAACAAGAGAAATGGCCCACTGGTACCTCCTCGGGTGGTGCCGGGCAGAGG
CCCGTCAAGAATAACCCGGAGATCCTGACCATCTGCTGCAGATGGCCATTGCTGGCGTGGAGGGAGACCTGGGTAGCCAC
ATCAGTTGCCTTTCATGGGGAAACCAGACTGGTTCATCAAAGCCATTGATCTTACAATGTTGGCATATACAAAACCTTCC
GTAGCCAAAGAGGTCACTCTTGAGAGCTGAGTTCAGATTTTAACTAGTCAGGTGATCCAAGGAACAGTTTGGCCATCAGT
CTGGAGCAGCTGGGCTGTGAGAAATGACTCAGGTTTTCTCCCAGCGTCTAGCCTGCTCCTCAAGTCCCTTCTCCCACTTA
```

```
TGTGGCACAGGTTAGACATGTCCCAGGTGTCTGTCTCCCTTGGGTTATATGTGATTGTCCCCATCTGAGTTCAAGTACAA
AGAGGTATAAAAAGCAGGGTGTGGTGGTACCTGCAGTCCCAGCACTTGAGATGCTGAGGCAGGAGATTGAGAGTTCAAGA
CCAGCCTGGGCTGTATAGTGAGCCTCTCAAGGAGTGAGGAGAGGAAGCTAAGTCCCCTTTATTCGAAGTCTGAGATTCGC
CCAGGTCCACAGCCCCACCCCAGCTCCCACCCTCAGCCCCTGCTGTGCCAGGGGAAGGTGGGGGTACTCTGGGGCTTGAC
AGGGTGAGTGGAACACTGCCTTGGCAGTTCTAGGTATACCAGCAGTTCCCACAGCATGGCACAGATGTGACAGATTTGAG
ACCTGTCTGGACTTTGGCCCGTGGAAGCCCCATGTCTCTCTCCATCTTCCTCCACAACTCTGGTGGCCTCTGCTTTTACT
TTGGAGTTCTTTTGTTTGTGTGCTTGATTGCTTTTTTTTTTTTTTTTTGGAAGGGGGGGGCAGGGTTTCTCTGTATCCC
TGCCTGTCTGGAACTCACTGTATAGACCAGGCTGGTCTCTACCTACCTACCTACCTCTGCCTTTTAATTGCTGGGATTAA
AGGCATGGACCACCATGCCCACCTTACTTTGGAGTCCTTATGTTCCTCCCCCTGCGGAAAGAACCAGACTTTCCCGAGCC
CCTCTGCTGCGTGTCCTGGCCTGACATCCCCCCTCCTCTCACAGGAAGAAAGCCTCCACCCCAATTTCTTCTTTCCTAAC
CTCTGGTTCTGAGGCTGGCTCAGAGAAGGAGATGGGACCCCTGATGTCTCTGAGTCTTCTTTGGCCCGAGTAGTTCTTTC
TAGGCTGAGGATCGGTAGACAACACACTTACCTTCCCATTAAGGCAAGCAAGAGAGTCTCTAAAGACACGTTTCTGATCT
GGATACAGTCAGGTGATACCGGCTGGCCTGGCAAAGGGCTTGAGACCTTGGAATAAGCTGCGGTGACAGGAGATAGGATG
GGACATGACTGTTATGTTAGTCCTGAAATGTGGGGGCCCAGGGCTCTGCAAAGGATCTCAGGCTAGTACCTAGCTAGTCT
CACTGGGTCCTTCTAGAAGCTCTGCAGGGCCTGGCTCTCCCACCTCTCTACCTCCCAGGGACACTGGGCACTATGCTTCT
GCCTGCAGGTCACCAACCTTTTTTTTTTCTCTCTCATGTATCCATGAGACCCATGGAGCCATATGAGACACAACCCAGCA
TATCAGGAACCACCGTGTCACGACATCCTTGCCTGAGGTTGGGAGAGACGCCACAAGCAATTAGGGGAGGCTGACTCAGG
GCAGAGCAGGCCCAGTGGCTCCCATCTTCCTGTCAGAGGAAGCTCAGGGACACAGGAACTTCTGGATTCTGACCTGGGAC
TCCAGGCTCTATCCGTCCCCACCCCCACCTTCTACAGAGCAAGAGTGACCTGTATCCCTTCCCACACCCCCTCAGATCCA
AAGGGCCCAGCAGGGGACCATGTGAAAGGAAGGAGAGACAGATGATGGTTATAACTGGACAGCTCCCCAGAAAGAGCAGA
GCTAGCTCAGTCCTAAGGGGGTGTCGGTAACAGGTGACGGTATTGAGGCTCTTTTACCCATCTGAGTAGCTGTGGACAAC
CAAGGGCCTGGCCTTCTCCATGACTTTCTTGGGGTCAGCCGACTGGTAGGCTCCACCTCTTCCCCAGGCATGTGCATGTG
TGTATGTGTGTTTGTGTGTATATGTGTGTATGCGTGTGTGTATGTGTATATGTGTGGTTGTGTGTATGTGTGTTTGGGTG
TATGTGTGTTTGTGTGTATATATGTGTGTTTGTGTGTATGTGTGTTTGTGTGTATGTGTGTTTGTGTGTATGTGTATATG
TGTGTTTGTGTGCATGTGTGTATGAGCCTATATATTCCCATCCTCCTGCCTGGACCAGCANNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNCAGG
TTAGGTTTGCCTCTGGCCTCATTCATAGGCTTTCCACATCTTTGGCCTCATGGGTGTGCTTGGCTGGTTACAGGTCTCAT
CAAATCCAGACACTGACAATCAGCCTGTGTATGCTCTGTGGAGGATGTAAATAAGCCGATAACCAGCCCAAGCACATGAT
AGTAATCACTTCTGTGAAGTGACAGTCACCCTTCCCCCTCCCCCAAGATGGTCACTGCTGGTCCAGGCAGGAAGAAAGGG
GACCACATACACACATACAAACACACATACAGAGGCAGGAATGAGTCTCAGACACCCTTGCTAGGGAGATGGGGAAGGTA
TGACTGACTCCAAGTCTTAGGGTTTCGCTGGGCAGAGACACCTATGGCTGCTGGGCTCCTACTCCAGCATCCACTCATAC
AGTGGTGGAAGGTTAGCCCAGTTCCCTCTGCAGGTGGTCTCAGGAGCATTTGAGATCCAGAGCTCCGCCCAGTTCCTCAC
ATTCACCTTTCACAACCCCTCGATGGCGCCATTCATTGGTCAGGAAGAACGAGAAAGAGGCCACAGGAAACAGTGGCCGC
ACGTCAGGGGGCAGGAAGAAGGTCAGTTGGACAGTTTGGGACTGAGGGACCCACACAGCCATAGAACTTCGCTGAGCACTG
ATGGCGACAGCCAGGAGTCTGGGCCTCCTGTTCTTTCTGCTGCTGACTTCAGGTGAGCGGGCGGGGCTCCTCTAGCTATC
CTGCTAAGCCTGGGAGACTCTGTGCTGCAAAGGGCCCCAGGGGAGCATGGGAAGAGACCCAGGGACTCGTAGTGACTCCC
TCATCATCCCTTCTGGCCTTGTCCCTCCAGGTCATCCCTGTCCATCTTGCTGGGTCTCAGGCTCCTGTTGTCACCCCATA
GTGTATTGTCGGTGGGCAAGAAAGACACTGCCCATCTGTTCTGGCTGGAACCAGGTTCTTGGACATGGTGGGTCAAACAA
GCTATTCAGTTCTGGGCTCCAGGCAAAGGCATCAGAGGCCCTGGAAGCTGCCTGTAAGGGCGGACAGCTGCTCTGCCCAC
ATAGTCCATCCATCACAGGCTCCCCGTTCCTGTCGGCACCATTCAGTGTCATCAGTGACAGGAGACGGGACCCAGAGCAC
TCCCATCACATACAGCTCAACTTAATACACCGACATGAGATTCCCAGTCCTGCCTCTACCCAAGTTGAGCCTGCTACCCC
CCTCACCTCCGTAGATGGCTATACCCTTCTTGACTGTTGAGAGATGGCTATGTGGAGTGATGAGGTGCTCCCGTCTCCAT
GCCCCGCCTCTCCTTGTTTGCTCCAACTTTGGCAAGTATCCTTAGGTGTCTGCCTTTCTGAGTCACTTATGGACAAGTGA
CTAGTCACACAGATTAGCATCTCTTGGTTCAGAAGAAAAAAAAATCTCTTCTCTTCCAATGCTGGGACAGTCAGTCAAGG
ATAGAGTTGAGTGTCTGACTCAGAGGTGGACCAGGTATTGTGACATATACTTGCACTTGGAAGGTAGAGAGGCAGAGGAT
CAAGACTTTGAGTTCTGTTTCAGCTCATAGTGAGTTTGAAGCCATCCTGAGCTATTGGAGATCCTGAGTATAAAAGTGGG
TAGAAATTGACAGGGCTGTCTTTCAGCAGAGCAGGCAATGCTGGACAGGCCAAAAAGACTCCTCCGAATGGAGATGTGGA
TCTAGTAGTGGCAGGGATGCTCATGACTGGGCAATGTGTCAGTCACCTGAGTGACATACGAGGTAGCATCGGATGCCTTT
TGTCCATTTACCTACTGGCTCTTTGCTTACATACCCAAGAAATATACATGGTGCTAAAAAGAGGCACAAAGTGGTAGCT
ACTTGCCTGAGGGAGGCTCTTGGCACCCTGAGTCTGAAGGCTGAGGAATGGGCTAGCCCCAGGTATTGGCTGCAGAAGGG
AAAGAGGCTGGCAGTAAGTGGAGGGAGGGAGGCTGAAGGGACAAGGGGCAGGGTATAGGGAACATATGGGTCACCTTCTG
GCCTCTGACCTCCTGCCTGCCCTCACCTGCTGCATCCCAACTGTTGAACTCACACCAAGGCGTCCAAAGTCCTCAGCAAA
CACCCTTGAGACACAGCACTTCCTAAGATCTTGAGTTTGAGGCTAGCCTGGGCTATATCCTAAGGACAAAGCGGGGTGGG
GAGCCACAAGTTGGATGTCAAGAGCTTCCCTTAGGTATCCCTGCTGGGATTCCCAGCCAAGGTGGAGGAAAGCTGTATTCT
TACCTACCCTGAGGCCATCCCCCCCTTTCCTAACCCTTGACACCCCCCTCCCCCATGGAGTCCTTCTTCCTCCTCTCTAA
CCCTCCTTAGATTTCATGTGAATGCAAATAGGGCCGATCTCAGAGACTGAGCCTAGCATCTGGTCTCAGTGCATCTTATA
AGCTCCACATACTATTTGAAAACCGAAGCAAAATTGAACAACATATAGACCCAATTTCTATAAATATGCTAATATGTTCT
TCCCTGTTCACTCTTTGTCCTGCCATGCCTTTATCATGAATTTTATTTTCTGTACTAATATTTGAAAATGAACCAGCTTT
```

```
CTTTAAATCGGAAGTGGTAATGTCCATGATATTTTTCGTGGGTTTTTTTGTTTGTTTGTTTAAGATTTATTTATTTCATG
AATGTGAGTACACTTTCACTGTCTTCAGACACACCATAAGAGGGCATCGGATCCCATTACAGATGGTTGTGAGCCACCAT
GTGGTTGCTGGGAATAGAATTCAGGACCTCTGGAAGAGCAGTCAGTGCTCTTAACCTCTGAGCCATCTCTCTAGCTCCAA
TGATATTTTTTCAATCAAATACCAGCAAAGGATATTTAAAAAGTATCAAAAACAAAAAAACAAAACAAAACAACAACAAA
AAACCCACAAAAACCAAAAAACAAACAAAAAACCCCCAAACCAACCAACCAACCAACCAACCAAACAAAAGTCACACCTT
AGTCAGGTGTTGGTGGTGCACGCCTTTAATCCCAGCACTTGGAAGTTGAGGCAGGAGGAACTCTGTGTTTGAGGCCAGT
TTGACCTACATAGTGAGTTCCAGAGCACCCAGGACTATGTGGAGAGACTAACTCTGTCTCAAAAAAAAAAAAAAAAAAAA
AAAAAGCTACATCCTCCAGAATACCTCAGATGGTCTGTGTGCTCAGGTATATTATCTCTTTTCATCTCTAAAACGTAAAG
GGTGAGAGGACCAAGCGCTTGCGTAATTTGCCAGAGGACAAACGGCTAAGCAGGGACAGGAAGCTTCCTGCATTCCATAG
CACAGGGCTCTGCACCAGAAGTCACGGAACTGGTCTCTAGTCCTGAAGCACCTTCCAACATGGGCTGACTACTCCCCAGC
CTCAGCCTGCTGCAGCCTTTACCCAGTCCAGGCAAAGATGGGGAGGCTAGGACAGAGCCAGCCACAAGACTTACAAGAGTT
AGAGAATCGGCCGCGGCGCCCGGGTGCCTAGCCTGGGAGGATCTGACGGAGCAAGGGCAGGAAACCAGGCAGGCTCTGTG
GAGGATGTGGCTGAAGAGCCACAGTTCTGAGAGATAAAACACTGTGCACTGCCAGGCAAAGCCTTTGTCTGGAGAACCAC
ATGCCACGTGTCGTCATCTCCCACCCCCACCCCCAACCCCACCCAGGCCCTGTGAGGTGGGAATGCCAGTTCTCCCCTGA
GAACTCCGGGACTGGGGAGGGTGGAGTCTGCCGGAGTGCCCCTAGCTGGGTGATGGACCAGCTACCTCTCCTAGGCCACT
GCCCCTCTCCCACTTGCCCCATCTCACCCCACTTCCTCCCCTAGATGAGGAGACCACGGAAGAGCCGAGGAATGTCTGCC
GTCGGCTTCAGGAGGGCCACGAGTATGACACCTTCGACTTGAATGACACTGCTCAGTGTTTCACCAAGTGTGGGCAATCG
GAGCACAGCCCCTGTGACGTGGGGAACCTGCAGAGGTGAGAGGAGAGGAATCTGGGCAAATGGGAGGAGTGGCCTGTTGG
GAAGCCATACTGTCATAAGTTCTCAGCGCCCTAGAATAGCATGCTGTTCCCCTTTCACCCTTTCATGTTCTCCCTAACCT
GGCCCTGCCTTGGTCATCAGAGTTGCTACTGATTAGGACAGTGGTTCTCAACTTGTGGGTCTTGATCCTTTTGAGGGTCA
AATGACCCTTTTCCAGGGGTCACCTACGACAGTTGGGAAACACAGGGGAAGGACTTTGGGGTATACAGGACAGCTTTCTG
GTGAATCCACACAGTGGTGGATAATGTAGCTGCTTAATTTCATAGTACTGGGAAAGAACTAGAGTGCTTTGGACTGTAAG
CATGCAAAGAGGTGCGGGGGACAGAGGTGGCAATGTCGAGATGAGATGAGGGCCGGGGGGTGGGTGGGTGTTCAGACTG
GAAACGCAAGCAAGAAGTCAGGGGTGAAGGACCAAGTTAACTAGACAATTATGGGAAGCAGAGAGATGATGTATTCAAAG
TTAAGAAGGGTGTGGTCAACTCGAGATTGATTGCTTGGACTTCAGGGACATCCAGGGGTGGTGACCAACGTGGAGGTGTG
ACCCATTAGGGTGGAATTCTGCTGGTACTATGAAGCTGCTCAGGAAAGATGGCTCAGGCAGGCTTCATGGGACCAAGAAG
CTGGCTAGATGGGGCAAGGGAGACAAAGCTCCCTGGTGCTGTCCCTGCTGGAGTCACAGTTGACACACTAGCTCTGCCTC
TCTGCTCTTCTCTGGGCTCTAGGCAGAGGGACCCAAAGCTCACCTGGGATACCACAGAAGTGCCCAGCTTACAGTCAGAA
CTCAGAAAATGGTTGTTGAAGGGAGGAATGAAAAATCTCTGCTCAAGTCTGGCTATTAAGACGGTGTTTATATTGAGGTT
CATTCTTTCTGTGGCCTGGAAATGCCCTGAGTCCTGCCTACAGCTTCTTAGCACTGTGAATGAAGCCCAGGGTACCTGT
GTGCTCATGTCCTGGATAAGCACCTTGGAGAGAGCAGGTGCCTTGGTTCTGAAGGGTGTGGTCTGCTCCCTCTCCATACT
TGGGACTGAAGGGTGTGGTCCGGTTCCACTCCATCCCTTCAGTTTGCCCTTTTCTCTTCATAACACTATCTTCAGCT
ACTTCTCTTGCAGCTGTAACTGGGTACATGGCCAGGAGCAGCCTAAGGGAGGGGGAGGGCATGGTGAATGGAGAGGCTCCGT
CCCCGGCAGCAGAGGCTTACAGCATGATGAGCTCATACACACCTTGTCAGGGAGGAACACAGAAGCAGAAGAGAATGGGA
AGCAGCTCTGGCTATCCTTCTACATATGCCACCTAGACCCAGCTTCTCAAAGGCTCCATGATCTCTGAAAACAGTACACC
AGAGGAGGTTGAAATATTGAAACACATGAACCTATGAGGACCATGAAAAATGCCCTGAGGTAGGTCCTGTTATCAACCCA
TTCTCAGATGAGCAAACTGAGGCTCTATGCTGTGAATAACTTGCCTGTGGTTACACAGCAGGTAGGAGGCAAAATTGATA
GGAGCTGATATAGAAGTCTCAGCGGAGGCACTTGATGTGACAGGCTGGACCCAGACTGCCAGTGCTCAGGTGGCTTCCAG
TATACCTGTCTGTAAAATGGGATTTACAGGATGATGGTGGAGGCTAAATAATTCTGCACTTGAGAGCCATGGTAACCCCG
CCATCCTCACTGTCAGAACGCCATGCCCTCAAGTGCCTGCTCCCAACCTCTCAGGCTTCCTCATAGTATCACTGAGGGAG
GAGTTATTAGCAGATAGCAGGAGCTCAAGAAATGTTAGTGTGCTTTATTTCAACGGCTGTCTTGATTATAAAAGTAGCAC
ATGGTGGGCATGATGGTGCATGGCATTAAGGCAGAGCAGGTAGATCCCTGAGTTCAAGGCCTGCTCTACAGAGTGAGTTC
CAGGACAGCCAGGGCTGCACAGAGAGAGCCTGTCTCAGAAAACAAACGATGACAAACGGTGATACATATTCATTGGGAAT
TCGCTATAGAAAGGAAGTGAGAATTTCCCTTAACCCATCAGCCAGAGACGGCTGCCATCTTCATCCGGATGTGTATCTTT
TTAGCATCTTACAGGACATATAACTGACAACTTATTTTGTGGTGTGGGTGGCTCCTGCTTTTGGACGTTCTTGGTCATGT
ATTGTTGTGAACTTGTATTCCTAGGCTTGGAATTCGTGACTTGGGGGGGCGGGGTGGGATTAACTCTAGCAGAAACAGGC
TAGAGTAGTCAGATCAGATTTCACTCCTGCAGGCAGAAGCTGACATTCCAGTTGCCCCGTGGCCTTGACAACCCCTGGTC
TTGTCTGCCTTTCTCCTTTAGCCATCCTTAAGGGTTTCTAGTGCACCTCGAGGTGACTGTGATGACCGACGTCAAGCGCC
TCCAGTTATTTCTTTCATATCTTAACATCTCTATTTCTGTTTATTGGTCTATAAAGCTGTTGGAATTTTCCATAGTGATT
TTGGGGATCCTTTATTTGTTCTGCATGCGTCTTCGTGAATACACATAGAGAATAATCTTCCTTTTCATGGGCTTCCCGTT
CACTTCCGCAGCACTGTCTTTTGACCACTGCTAAAGGTTTTAATTTTAGTTAGTGTAATCGATCAGATCTTTAGATTTAC
TTTAAGTATTGGTGTGTGTGTGCGCGCGTGCGTGCATGCGTGTGTGTGTGTGTGTGTGTGTGTGTGTGTGTGTGTGTGTG
CATGTGTGTGTGTGTGTATGCATGATGTGGGGGAACTAACATGCCACAGCATGTGTGTGTGTGTGTGAGTGTGTGTGT
ATGCATGATGTGGGGGAACTAACATGCCACAGCATATTCTCGAAGTCAGTTCTCTCCTTCCACCTTTACATATGTTCTGA
GACTTGTGACAAACACCTGCTCCCTGAGCCATCTCACCAGCCCTATTGCCTGTTACTCTGTTCTGTTCTGCTCTTCTCTG
TCTGTTTTCTATTCTAAACTAGGCAGGCATGGCTGTTGAAACATCTTTAATCCCCAAATTCAGGAGGTGGAAGCAGAGGA
TCAAAGAGACCAAGTCCATCCTCAGGTACTTGGTGAATGAGGCCAGCCTAACCTAGATAAGACTAGACTCTCACAGACAA
AAAGAGAAGAGAACAAAA
```

MOUSE mRNA SEQUENCE : mR7-157.1 (Seq ID No: 84)

```
AGACAGCGTGATCCCGGCCTCCCACGGGGCAGCTTTTACTGTCTAGGGAAGAAATCCCCAAAGTCCATGGAGTCTGAAGA
CTCTGTCAAGCCTCGCTAGGAAACCTAGGAGTTTTAGAGGGCACTTGGCACCGGAAGCTAGCCGGGTAGGCGGAGCCTCA
CCTGGATTGAGTTCACAGCTGCCTAGACAGGCTCAGACTAGGTGCTGGGCACCTGGAAGGAGGAGGAGACATTAGCAGCA
AAGGCTGTTAACAGAAGTGCCTGCCTAGGCTTGGAGGCAAGACGCTGCTGTTCACAGTGCGAGACGGAGGTAGGAGTATA
ATGGCTGTCCAGGTGCTGCGGCAGATGGTCTACTTCCTACTGAGTCTGTTTTCTCTGGTGCAAGGTGCACACAGTGGCAG
CCCCCGAGAAGACTTCCGCTTCTGTGGCCAGCGGAACCAGACCCAACAGAGCACCCTCCACTATGATCAATCTTCAGAGC
CTCACATTTGTGTGGAACACAGAGGAGACCCTCACAATTCGTGCCCCCTTCCTGGCAGCCCCAGATATTCCCCGCTTC
TTCCCAGAGCCTAGAGGGCTCTATCACTTCTGCCTCTACTGGAGTCGCCACACTGGGAGACTCCACTTGCGCTATGGCAA
GCATGACTACCTGCTTAGTAGCCAAGCCTCCAGACTCCTCTGCTTCCAGAAACAGGAGCAGAGCCTGAAGCAGGGAGCCC
CGCTGATCGCCACCTCTGTCAGCTCCTGGCAGATTCCCCAGAACACCAGCCTGCCTGGGGCTCCGAGCTTCATCTTCTCC
TTCCACAATGCCCCACACAAGGTCTCCCACAATGCATCTGTGGACATGTGTGATCTCAAGAAGGAATTGCAGCAGCTTAG
CAGGTACCTGCAGCACCCTCAAAAGGCTGCCAAGCGGCCCACCGCAGCGTTCATCAGCCAGCAGTTACAGAGCCTGGAGT
CAAAGCTGACCTCTGTGAGCTTCCTGGGAGACACATTATCCTTTGAGGAGGACCGGGTCAATGCTACAGTGTGGAAGCTG
CCACCCACAGCCGGTCTAGAGGATCTGCATATCCACTCCCAGAAGGAGGAGGAGCAGAGTGAGGTCCAGGCATACTCGCT
GTTGCTTCCCCGGGCCGTATTCCAGCAGACCAGAGGCCGTCGCCGGGATGACGCCAAGAGGCTCCTGGTAGTAGACTTCA
GCAGCCAAGCTTTGTTCCAGGACAAGAATTCTAGCCAAGTCCTGGGTGAGAAGGTCTTGGGTATTGTCGTGCAGAACACC
AAAGTCACCAACCTCTCAGATCCGGTGGTACTCACCTTCCAGCACCAGCCTCAGCCAAAAAATGTGACTCTGCAGTGCGT
GTTCTGGGTTGAAGACCCGGCATCAAGCAGCACAGGGAGCTGGAGCAGTGCAGGCTGCGAGACAGTGAGCAGAGACACAC
AGACATCCTGCCTGTGCAACCACCTGACCTACTTTGCAGTGCTGATGGTGTCATCCACAGAGGTAGAAGCCACTCACAAA
CACTACCTCACGCTCCTGTCCTACGTGGGCTGTGTCATCTCTGCTCTGGCTTGTGTCTTCACTATTGCTGCCTACCTCTG
CTCCAGGAGGAAGTCACGTGACTACACCATCAAAGTCCACATGAACCTGCTGTCCGCTGTCTTCCTGCTGGACGTGAGCT
TCCTGCTCAGCGAGCCTGTGGCACTGACGGGCTCCGAAGCAGCCTGTCGCACCAGTGCCATGTTCCTGCACTTCTCCCTG
CTTGCCTGCCTCTCCTGGATGGGCCTCGAGGGCTACAATCTCTACCGACTGGTGGTGGAGGTCTTCGGTACCTATGTGCC
CGGCTATCTGCTCAAGCTGAGCATCGTGGGCTGGGGTTTTCCTGTCTTCCTGGTCACTCTGGTGGCGTTGGTGGATGTGA
ATAACTACGGCCCCATTATCCTAGCTGTGCGCCGGACTCCGGAACATGTCACCTACCCCTCTATGTGCTGGATCCGGGAC
TCCCTGGTGAGCTATGTCACCAACCTGGGCCTCTTCAGTCTGGTGTTCCTGTTCAACCTGGCTATGCTGGCCACCATGGT
GGTGCAGATCCTGCGGCTTCGCCCGCACAGCCAGAACTGGCCCCACGTGCTGACCCTGCTGGGCCTCAGCCTGGTCCTTG
GCCTCCCCTGGGCCTTGGTCTTCTTTTTCCTTTGCTTCCGGCACCTTCCAGCTTGTCATCCTCTACCTCTTCAGCATCATA
ACTTCCTTCCAAGGCCCCTCCGGGAGGACACATGCATGGCGTCCGCTCACGATGTCTGTGGCCCAGTGCTGTGCCCACCC
AGCCTTTGTTGGTTAGTGGCATACTAGAGAAGGCCCTGGTCCTTGAAGGCGTAGGGCTGTTGCTCTGAGGGACCTCCATC
TCTCCTGAAGCCTCCTCCCTCTGGCAAGTACTGGGATACAGCCACCCTTTCAACCCAGCACTCTGAAGACCAAGACAGCC
CCCTCTGGTGACACTGGCCAAGCTTGATCTTTTTCCTAAGAAGTGGTCTTCAGATCCCTGCAGGTCGCTCAGAAGACACT
GGGCTGCCTAGTGTGAATTCTGTCCTACTAACGTACAGTGAGCAGCTCCTCACCCCCACCCCCGCAAAAGCTCTCACCAA
GTCCTGGAGTGTCAGGCAGGGGGCTGGAAATCCAGGAGGACTTCCTGCAAAAGGCAGCATTTCATCTTGACCTCAGCCTT
CAGGTTGGGGAGAATGTTCTTTTTAAATACCAGTTCATTTGTCTTTGATATTAAAGCTCTTTATAGAGAGTCTGGAAAC
TGTAGGCGATTGTCGAGAAGAGAAATAAAAATGAGCTGTTATCTAATGCCATGGCAAAGCAGCAC
```

MOUSE PROTEIN SEQUENCE : mP7-157.1 (Seq ID No: 85)
QKCLPRLGGKTLLFTVRDGGRSIMAVQVLRQMVYFLLSLFSLVQGAHSGSPREDFRFCGQRNQTQQSTLHYDQSSEPHIF
VWNTEETLTIRAPFLAAPDIPRFFPEPRGLYHFCLYWSRHTGRLHLRYGKHDYLLSSQASRLLCFQKQEQSLKQGAPLIA
TSVSSWQIPQNTSLPGAPSFIFSFHNAPHKVSHNASVDMCDLKKELQQLSRYLQHPQKAAKRPTAAFISQQLQSLESKLT
SVSFLGDTLSFEEDRVNATVWKLPPTAGLEDLHIHSQKEEEQSEVQAYSLLLPRAVFQQTRGRRRDDAKRLLVVDFSSQA
LFQDKNSSQVLGEKVLGIVVQNTKVTNLSDPVVLTFQHQPQPKNVTLQCVFWVEDPASSSTGSWSSAGCETVSRDTQTSC
LCNHLTYFAVLMVSSTEVEATHKHYLTLLSYVGCVISALACVFTIAAYLCSRRKSRDYTIKVHMNLLSAVFLLDVSFLLS
EPVALTGSEAACRTSAMFLHFSLLACLSWMGLEGYNLYRLVVEVFGTYVPGYLLKLSIVGWGFPVFLVTLVALVDVNNYG
PIILAVRRTPEHVTYPSMCWIRDSLVSYVTNLGLFSLVFLFNLAMLATMVVQILRLRPHSQNWPHVLTLLGLSLVLGLPW
ALVFFSFASGTFQLVILYLFSIITSFQGPSGRTHAWRPLTMSVAQCCAHPAFVG*

MOUSE PANTHER CLASSIFICATIONS
        FAMILY (SUBFAMILY)
                LATROPHILIN-RELATED(G PROTEIN-COUPLED RECEPTOR 56)
        BIOLOGICAL PROCESS
                Signal transduction(2.11.00.00.00) > Cell surface receptor mediated
signal transduction(2.11.01.00.00) > G-protein mediated signaling(2.11.01.07.00)
        MOLECULAR FUNCTIONS
                Receptor(1.01.00.00.00) > G-protein coupled receptor(1.01.01.00.00)

MOUSE GENE ONTOLOGY
        BIOLOGICAL PROCESS
                cell surface receptor linked signal transduction > G protein linked
receptor protein signaling pathway

cell communication > cell adhesion
cell communication > cell-cell signaling
G protein linked receptor protein signaling pathway > neuropeptide signaling pathway
neurogenesis > peripheral nervous system development
MOLECULAR FUNCTION
ligand binding or carrier > calcium binding
ligand binding or carrier > protein binding
molecular_function unknown > lymphocyte antigen
CELL COMPONENT
plasma membrane > integral plasma membrane protein
cell > membrane fraction
cell > plasma membrane
plasma membrane > intercellular junction
MOUSE PROTEIN DOMAINS (INTERPRO SIGNATURES)
IPR000203 (RECEPTOR PKD)
IPR000832 (G PROTEIN RECEP F2 4)
IPR000832 (7tm 2)
IPR000203 (GPS)
IPR000832 (GPCRSECRETIN)
IPR003910 (GPR56ORPHANR)
IPR000203 (GPS)

HUMAN GENOMIC SEQUENCE : hD7-157 (Seq ID No: 86)
TCTAAAGCTATGCCTCCGAGTATGGCGTGCTTTGGGGCCAGCTGTGACAGCTGGGGAGCAGCCCTATGCACCCCCACTAC
AGTCCAACATTTGGTTTCAGATTCTATTTATATACATTATGTACCTAGATACTTGTGGGTTTGATTTGGTTTTGTTTTGA
TTGCACAAATAACATTAGAACAGCTATAATGGAAACAATTCTAAAATGGAAAAAAAAAAAAGGCACGCACAATGCTATCAG
TTGGCTCGGCTTCCTAAAAGCACTGCTTAAGTGCTGGCAATAAGCCCCGCACTGTGCTGTCTATGGTTGATCTCACTAAA
GCTGCACAACCACCCAGTGAGGAAGAAACCTGTCACAGATGGAGAAACTGAGGCACCAAGGGGCAGGTCGCCTCCTCAAG
GTCACACAGTGAGGACTGCAATCCACCTCCAGGGTCCTGTGCTTGCCACCTCCTCAGGGCTGCCTCTTCTTGCCTGTCAT
CCCTGCCACCTCTCAGCTTGACCTGGAAGCATCAATTTGACATCACTGTTCACTCTGTGCAGAGCTTTTCCAAGTGGCCC
CTGGGTAGCAGCCCTGTCTTTGAGTGGGGAAACAGGTCCCTGAGACAGGTGGAGACATTGCCTTTGTTTTCTGCTTATTT
CACTGAATTTTGTATTACAGTCATCCCCCAATACCTGTGGGGGACCAAATTGTAGGATGCTCATGTCCCTCAGTCATGCC
CCCAACGACTTGTGCAGAGGGCCAACTGTATAAGCGTTTTCCTTTTGTTGTGACCATATGACCATGAGCTGGGGGGTGAC
CAGCTCTCTCAGTTTACTGGAACTGAGAGGTTTCCCAGGAGGCAGAGCTTTCAGTGCTAAGCAGAGACAGTCCCCGGCAA
AATGAGATGGTTGGTGACCCCAGAGGACAAACCTGGGTTGTGCTTTCTGAGCCATCTCCTGGTGCTGAGCACTTGGACCG
CTTCAGCTCTCCACGGGTCAGGCTGGACAGCCCTGTGACCACAGCTTGTCACTGGAGTGTCTTTTCTTCAGGGCCCCTCT
TGAGTAGGAGGAGACCAGGCTTTGAAGGTTCCTGAGAGGAGATGTACGGGACCTGAATACAGCTTTCCTGACCTGCCCCT
CATAGGAGCCTGCAGGGAGGTGAGATGGGGACCCAGGACAGGGCTGAGGGCAGGCAAGGGCCAGGGGTGTGTTGGAGCTT
GCTGTTTTGGCTTGCAAGAACTGGTGGTTAAATTTTTAGGCATTTTGTGAGTTGGTTGTTAAACATAGCCACTGTTAAGA
ATTAAAATTACATACACAAAATTAAATTACAGTCATGTGTTGCATAATGACGTTTTGGGCAATGACAGACCACATAGATGA
CGGTGGTCCCATAGATTATATGGGTCATATAAAGAAAGCTACAGATAGCACTTCATATTGGCCTTGCAGATCAACTAGG
GGGAAATGACTGATATTCAGTAACGGTGCTGGGACATTTGGTTTTCCATGTAAGAATATATAAATTTTAAAATGCATCCT
CTAGGTTTGGATAAATGCACTCTATGATGTTTGCACAATGGCAAAATCACCTAATGACGCATTTCTCAGAATGTGTCCTT
GTTCAGTGACTCATGACTGTGTATTTAAAAAGATAATAAATACTCAGAATGAAACACTTTCTGATTATTTTACCACCATC
ATTCGTTGTAATGGATTGAATCCTGTTCCTCTTTGCCCCAAATTCTTAAGTTAAAGTTCTAACCCTGAGTACTTCAGAAT
GTGAGCTTATTTGGAGATAAAGTCTTTACAGAAGTAAGCAAGTTAATTGGCGTTTATTAGCGTGGGCCTTAATCCGATAT
GACAGGTGTGCTTACAAAATGGGGGAATCAGACAGAGACACACACACACAGGGGAGGGCACCATGTGAAGATGAAGGCAGA
GATCGGAGTGAAATTTCTACAAGCCAAGCAGTGCCAAAGATGGCCAGCCAACCACCAGAAACCAGGGGGAGAGGTGTGGG
ACAGATTCTCTTTCATAGTCTTCAAAGGCACCAACCCTGCCAACACCTTGATCTTGGACTTCTAACGTCTAGAACTGTGA
GAGAATACATTTCTGATCAAGCCACCCTGTTTGTGGTACTTTGCTGTGATAGCCATAGCAAATGAATCCATCTGTGCTCT
TGAAGTTGCCATCTGTTGCACTGGTAGAGTAAAAAGAATGGTGAGCTTCTGTGCACCTTTTCCAAATGCCGTGTTCGATG
TTTGCTATCTTGCAATTGGCCGTGGCAGAAGTATTTATTTATTAAAACTTTTTTTTTTTGGTAGAGATTGGGGTGGGTCT
CAGGAGGTTGCCCAGGCTGGTCTTGAACTCCTGGCCTCAAGCGATCCTCCCACCTTGGCCTCCCAAAGTGTTGAGATTAC
AGACACTGCACCTAGTCAGAGAGTATTAAAACCACGGAAATCTACAAATGTTACAAATCAGGACTTGCTTTGTTGTTTTG
TTGATTGTCTAGATAGAGTTGAGAAAGCAGTGAAATGTTATTAAGGCATATTCCATGCAATCAATGTGTCATGTCTGTAG
CTGTTACATCACGAACAGCACAAACAATTTGAAAACCATGATCCGATTCAGCCAACTATTCAAAATCTGTATTCAAAATC
AGACTCAGCAAAGATTCCAGTATCTGAAGACCATAATCCCACTCGGCAAAAAATTGCTCACGTCATTGATGGACAAATGA
AATTCCGACACGTATCTTTGTTGTTTCCTTTTTTTTTCTTGTTAATGTAAATGAAAATATCAATATAGGTATCCAAACAAC
ACTTAGAGAGCCTGTTGTCAATATTTAGCAGCCCACCACTAATGTGGGGCGGAGGAGGCCAGCTCTGGCCCGCCCAGGAC
TGAGGGGCCCAAGGCTGCAGGGCCTGCACGGGTGGGTGGACCGACTGCCTGCGTTTCAGCTGCTCTGGGTGGACCAGTTC

```
CAGTGTCTCCTCCCTGGGAGTGGGGTGGGGGGCTGCCTTCCTACCCTCTACCCTGGGAGGCTTCTGCAGGGTTTCTGCTT
GGTTCCTAGTGATCTCCGGCTATCAGGACCCTGTGGTGTTAGCAGCTGAATTTGTTTTTCTTTTCTTTCTTTTCTTTTTT
TTTTTTTTTTTTTTTGAGAAGGAGTCTCTCTCTGTCACCCAGGCTGGAGCGCAGTGGCGCAATCTCGGCTCACTGCAACC
TCCACCTCCCGGGTTCAAGCAATTCTCCTGCTTCAGCCTCCCGAGTAGCTGGGATTACAGGCGCCCACCACCACGACTGG
CTAATTATTTTTTGTATTTTTAGTAGAGACAGGGGTTTCACCGTGTTGGCCAGGCTGGTCTCAACTCCTGACGTCAAGTG
ATCCGCCTGCCTTTGCCTCCCAAAGTGCTGGGATTTAGGCATGAGCCACTGCACCCAGCCAGCAGTTCAATTTCTTGATG
GGGAAGTAAGAGAAGAAATAAAAAATCAATGATGTAATTAATCAAGTAATTCAATAAATATGAAGGCAAATACATGTAAT
GAAAGGGCAATGAAAAAAGCAAACTAAAAAAACAAAGAGTGGAGATAATTATGCCTGGTCTGGCATGCAGGAGAGGCTG
CTAGGCCTAGCATTGATCGATAGCCTTTATCTTGTCAGAGCAGGGCTGAGTCCTTCAGCTACCCTCCTTGAAATATAAAT
CGATCCCCTCACTCCCTTTTGAATCCACCCATGGCTTCCTGCTTCCCTCGGACTAAAATTATACTTCCGTTCCTGTCTTC
ACAGCCCGGCTCTTTGCCCTCATCTCCTTTGCCTCTGTGCTCCAGTCACAATTACCAGATCCTAGTTTCTCCAACATGCT
GAGGATATTCCTCACTCCGAGCCTTTGCACATGCTGTTCCTTCTGTCTGGTATGCTGAGCCCCAGGCCCACTCTTTCCAC
GGCTGGCTCCTCCTTATCCATTAGGACCTTGTTACAAACATCACCTCCTCCAAGGAGCCTTCCCTAATTACCTGGCTAAA
GTGCATTCCCTATTTCATTTCATCTTCACTCTTTTGTTGTTTAGGTTGCTTTTTAAATTGCCCTCCCATTACATGTATTT
GTCTTTGTATTTATTGAATTATTTGATTAATTACTTCATTGATTGTTTAATTTCTTACCTTACTTCCTCATCAAGCCATC
AGCTCCATGAAGGCAGGGATGGTTTCTGGGCTCTGTCAGGTATGCAGAGCCCAGCTTGAGGCCTGGCACTTGGTGGGTAC
TTAGGAAATGTTCGTTGAATGAATGAATAAAATACATTCACTGAGAAACTGTATGACCTAGAGATAAATCATGGACCTAT
GGTCCAACAGAGATATGGATTCAAGTCCTGCCACTGGCACTTATTGCTGGTGTGCTCTGGGACAGGTTCCTAAATCTCTT
TAGGCCTCAGTCTCCCTGTCTATAATCCCCTATGTAATTCCCTTCAAATGATTGTTATGGGGATTAAATAAATCAATCCA
TGCAGTTTTAGGAATGGGCTTGGCACAGAGAGGTGCTTGGGAGTGTTGGATGCCAGTATTCTTATAAGTAAAGGGATGTG
GGACTTTGGGGTTCAGGGGTTCAGGGGTTCAGAGCCTGACTTTCCATAAAAGAGGACGGGATAACTCCAAGAGGAGTTCT
TGGAAGAAGGGGCATTTGAACTGGGCCATAAAGGGGGGTGGATGGCGTTTCTCTAGGCAGAGATGGCAGTAGGGAAATCA
GGAGCCAGGAGGGACATCTGGTCCCAGAAAGAGATCTGCGGGCAGGCCAGGCCTGCTCCTGGCTGGTCCCAGAAAGAGAC
CTGCGGGCAGGCCAGACCTGCTCCAGGGCTGGGAGGAAAGCCACTGGGTGCCAGGGATACGCTCAGTATGAGCTGCCTTG
TTCTAGGCCACGGCCTCTGCCCCCAGTCTTGACATTTGGTTTTCACCTGTTTCCTCTGGGTAGTTGACACACAGCAGCTG
CTGACTCTGAGGAGGCATGGGGAGGCCCAGCTGAAAATGCCCCCGGAGGAGGTGACTCAGTAGCTCCTATCTCGACCCTG
GCAGAACTCAGGTAACTCAAGACACGGGGCAAACCTGAAGAGGCCCCCAGAGCTGTACCAGGAAAGCTCTGCAGTGTCAT
GGAACACATGGAAGTGGCCTGATGCTACTGACTCACAGAGGCTTGGTGGATTGCTTTGAGTTGTTTTCGGTCACCCTCCA
TCTCCTTGTCTGTAAAACGGGGACAGGCACTCCTCAGAACTGCTGGGACAGTCCGGGGAGCCTTTTGGTTGGCACAGTGT
GTTTCAGAGTGGTTCTCTGTCCCCCCGCCCCAGGCATCCTCATGTGATTCGCTTGAAAACACGGCAGGACGGGTCCTGTC
ATTATCCCCGTTTCACAGATGAGGAACACTGAGGCTCAGAGGAGGGCCCTGACCTGCTCAGTACCACATAGCTGGAGAGT
GATAGATCCAGGGCTGGAGTCAGACCCCAGACAGTGCACAGATGGATGATCACATATTGACGGAACTTGGGTGTGACCAC
AGGATACAGAGAGTGGAGAACTGATCTGATGAACTGACTGCAGGAGGCAGGGTAATACCATGGCCTAGCTAGCCCTCACC
TCTTTTCTAGAAAGCATGGTGAGGATGGGGGTGCTGATCCTAGGGCTGGCTGCGGAGCAGTGGCATGGAGACCACCTTCC
AGATGTGGCCAGAATGGAGGAAAGTTTTCCTATTGCTCTAATATGAGGAGGCAACACTGCAGACCAGAATGAGTTAAAAG
GTCAGTAATTTATTTTTATTTTTATTTTTTGAGACAGTGTCTCTCTCTGTCGCCCAGGCCGGAGTACAGTGGCACAATCT
CAGTTCACTGCAACCTCTGCCCCCTGGGTTCTCTAGCGATTCTCCTGCCTCAGGCTCCCAGGTAGCTGGGACTACTGGTGTG
TAACACCATGTCCAGCTAATTTTTTGTATTTTTAGTAGAGACAGCATTTTGCCATGTTGGCCAGGCTGGTCTCAAACTCC
TGGCCTCAAGTGATCCACCCACCTCAGCTTCCCAAAGTGCTGGGATTAGAGGCGTGAGCCATTTCGCACAGACCAAAGGT
TAGTAATTTAAATTCTCCTCTGGATTTTGATGTGGCAGATGTTTACTGAGCACTTAGCCACGTGCCAGCCATGGGTGGTA
GCACTTAGCATTGTCTCATTTAGTCCTTACAACTCCATGGATAGATACTGCTATCACCCCCATTCTGCAGATGAGCAAAC
CAAGGCTAAGGGAGATAAACCAACCGGTCAGTGGAGCCGGGTACCGACCCAGACGGTCTGCTCTAGAGCCCCATGGCTAA
TCACGCCTCTGCTGCCTGCTCTGTGGGCTGTTTTCTAAGTAAATCTAAGGCCCTGGTACCCAGTAGTTGCTGTGCAAATA
TCGGGTTAGCGGATGAATACATGAATTAGTGAAAAATGGCCCAAGTAAAATAACCTGTGAGTAGGAGGTGATTTTAGAGA
CCATTTAGTCCTCCCTGTAGATGTGGTAAGTGTCTTATCTGCAACGCCCTAGCTGCACAACTCCTTGGCTTCCCTCTGAG
ACAGGTAGCTCACCCCCGACCTTGTGGGCAGCACCAGCTCTCAGAGGGAGCAGAGAGGCTGAGCCGCGGCCCTGCTGGA
GCACAAGATGTCAGAGCTGGGGACCCTTCCTGCCCCTCCACACCTCCTATGTGCCCCAAGAGGGAAGGAGTCTGCATTTC
CTGAGGCCCTCCACTGAGTGGGGATGAGGGGTGGGCAGGCTGAGACTGGGCTTACCCAGGGCTCAATAAGGGGCCTGGAG
TCCTGGTGGGAGTTCTGGAGTCAGACTGCCTGGGTGGGATCTCCGTCGCGTTCCTGAACCCCCTGTGCCCTATCTGTAAA
ACCCGCATTATAAAGTGGCTCCCTGAGGTCAGGAGTTCGAGACCAGCCTGGCCAACGTGGTGAAACCCCATCTCTACTAA
AAATACAAAAGGTAGCCAATTGTGGTGGCACATGCCTGTAATCCCAGCTACTTGGGAGGATGAGGCAGGAGAATTGCTTG
AACCCAGGAGGCGGAGGCTGCAGTCAGCCAAGGTCACATCACTGCACTCCAGCCTAGGGGACAAAGCAAGACTCCATCTC
AAAAAAAAAAAAAAAAAGAAAAAAAGTTATAAAGTGGCTTCCGCCTCTCGGGGTATGGTGAGGAGTCTGTGGGCTGATTC
CTGTAATGGGCCTAGCACAGTGCCTGGTTGCCAGGATTACATAATAGCTATTATTATCATCTGCAGCCACCAGACATAGA
ACTGGACACTCTCCGGCTCCACCCCTTCATCCCAGGGGTGCTGGGCTAGGTCCCGGATTGGTGGTGAGGGAGGAGTCCCA
GAGCCAAAGCCCTGACCTCACAAATGCTCCCTAGGTACCTGATGAAGCAATTTCCTGACCCTGTCTCTTTTCTTCCCTTT
ATTTATTTTATTTTATTTATTTATTTTTTTTTGAGACAGAGTTTCACCTTGTTGCCTAGGCTGCAGTGCAATGGCACGATC
TCAGCTCACTGCAACCTCCGCCTCCTGGGTTCAAGCGATTCTCCTGCCTCAGCCCCCAGAGTAGCTGAGATTACAGGCAT
GTGCCACCACTCCTGCTACTTTTGTATTTTTAGTAGAGACGGAGTTTCACCATGTGGGTCAGGCTGGTCTTGAACTCCCG
ACCTCAGGTGATCCACTCGCCTCGGCCTCCCAAAGTGCTGGGATTACAGGCGTGAGCCACCACGCGCAGCTTCTTCCCTT
CACTTTTAGAAGGCAAACAAGGCAAAGGCATGTGCCCTCATTTACTAGATGATCTGAGGCACAGAGAGGTTAAGAAACCT
```

```
CCCCGAGTGTACCTAGTGAGTGAAGGTCCCAGCCCAGGGCCTCAGGCCCAGCCCCTGCTGCTCCATCACAAGCCAGGGAC
CCTGAGGCCAGAGGTCATGGGGGATGGAGCATGGGTGTCAGGAGTGGCGGAGTTGCCAGCGGGTAGACAGGAAGTCACTG
GGACTCCCTTGGTGTCCTTATCAGGGTGGGTGACATCTCTGGGGCTGTGGGGAAACCCCGGAGCCTAGAGGTCAGGCTGT
CATCCTGCAGATCCCCCTCTCTCTGACACTCCTTCTCCCTTCTGGTACCCCAACCCTGTCTGTACTCAAGCAGGAGACTC
GTAGGGCAGAGCAGGCCTGCAGGCCCATGGGTGCGGCAAAGGTGGCATCGACTGTTGGGAATTTGCCATGGGGAAGAGGG
GCAAGAGGGAGCTGGGACTGGGAATGCCAGTGGGGTCTGGGCTGGTCTTCAGGAATGGACCTGGGCTGTGGCCAGCAGAT
AACCCTTCTTTACTGCCTTGGGGAGTCAGAGAGGGCTCCAGTCCAGACCTGGATCCACCTCTGCTCCCATCTGACCACCT
GTCCACATCCCTGTTTCACCTCTTGCATCTCATCTCTACCTCAGCTCCTTTGTTCAGCCACAGAATCAAGCTGGCCTCCT
CCTCCTTGCTCCTTAAACACCAATCCCTGGTCTCAGGGGATGTCCCAAAGGCCGCTTTCCCCACTATATTCAGTCCGTGC
ACAGGTGTCCCTTCCCAGAGAGCATCTAAGCTGCCCATCCCACCTCCATCACTCTTTCCCCTTTCCTGGCTTATTTTTGT
TCCATAATTCTTACCACTGGATTTATTTATTTATTTGAGACTGAGTCTCACTCTGTCACCCACCTGGAATGCAGTGTCG
AAATCTCGGCTCACTGCCACCTCCGCCTCCGGGGCTCAAGCAATTCTCCTGCCTCAGCCACCTGAGTAGCTGGGACTACA
GGCATCTGCCACCATGTCCGGCTAATTTTTGTTATGTTTAGTAGATACAAGGTTTCACCACGTTGGCCAGGCTGGTCTTG
AACTCCTGACCTCAGGTGATCCACCTGCCTCGGCCTCCCAAAGCACTGGGATTATAGGCGTCAGCCACTGCACTCCGCCG
CCACTGGATATTTTTAATAATTACTTGCTTATTCATTTCTTGTCTCTCCTACAGTTCTGAGGGCAGAGGTTTCTATGTTT
TCTTGGCTGCAGGATCCCCCATGCCTGGCACAGGACCTGGTGCACAGCAGGCCCTCAATAGACACTCGCTGAAGGCGTGG
TGCATCCCCTGCCTGTCCACACTTTCATCCATCCACCGTCTGTGCTTTCTTCCCACACTTCCTCACCCATCAATCACCCA
GAATTTCTCGATCCCAGGCCCTGTGCTGGTTCTTGGGTTTGGTGCGCTAGCAGGGCACACGGATCAGGAAAGAAAGAATT
CCGACACTTAGGGATTGGTCCCAGCCAAGGGGGAGCCTGGGGGACTATGGGAGAAACAGTGGGGTGAGGGGAGGTGAGGG
AAGGTTTATAGGGTGCATTGAGGTGCATGCCAGCTCGCACCGGTGACCCACAGTGGCACCTGATAGAGACTTTGT
AGAATAAATCAGCCATTGCCCGGATGGCTTCTAGAAGGGGTGGCGGGGCTCAGACGGTGTTCTCAATGTTGAGGATGTGA
TGCAGGAATCAGAAAGCATCTAGAGGAGGGGTACCTGGGTGTGAGAGCTTGAGAAACTTCCCCTGGAGAATTGCCAGACT
GAGACAAGGAGTGAAGGGTGGGACAGGGGCCAGGCAGGCAGGGTTAGGGGCGAGTGGGCCAAGGCCTGCCTGGAGGACTC
TGCTGGGCACCCTCCCAGGATGCCTTGAGCGGTGGCCGCTGAGCTCTTTCCTGGAAGAGGCTTCCATCCCTGCCCCTTTG
TCTACCTCAGTTCTCCCTCTGTCAGGTGTCCTGCAGGAGCCTGGAAACTCAGAGAGAAGAATGAGCAGGACTCCCCATCC
CCCTCGCTTCCCGCCTCCTGACCCCACCAGCTGCTGGCTGGGGCCTGTTTTTATTACAGGCAGGTGAGTCTGGCCCAGGA
TTCCAGGCGGGGCCTGGACTGAGGGAGGAGGCAGGAAGAAACTCACAAAGAGTTGGCAGATCACGGATGGAGGGCAGCAT
CTCCCAACAGCCTGGGCGGCCGCTGAGACCCAGAGAACCCAAGGACTCCCCTGGGCTCATCCAGCAGCCTCTGCTTCCCA
GGAGAGAGGTGCTGAAGTCCACGAAGAGGTGAGGGGTGGGGGGACTCAGCACCTAAGGGTCAGGTTGGCCCCTGCAAGGA
CACCTGGGAACAAGAGAGGGTGGCGGCTGCAGTGGCTGTGGGGCAGCCGGAAATGGGGCGAGACAGACAGGCGGAGCCTC
ACCTGGGGCTGCCCGCCAGCCCAGACAAGCTCAGACTGGGTGCCTGTGGCCCTGGGAGGAGGTGGAAGGGGAGGAGCAGG
CCACACAGGCACAGGCCGGTGAGGGACCTGCCCAGACCTGGAGGTAAGAGACGGGTGGGGGACAGGGGTGGGAAAGATAA
GTAGGGTGCCACTGCCAGCCTGTCCCCCTTGACCTTGGCCATCCCTCAGGTCTAGTTCAATGCCCTGAGAGTCCCGATGT
GTGTGGCCTGGCCCTTGGCCTGTTCAGGGTACTGGGCGTCTCTCAGTGGAGGCCCTGGCTGTTCTGGGGTTACCCCTTGC
AGTGCACAGCATGGCCGGGCATGCTGGCATGGTGGTCATCCTAGCACCGGGAAGCTGGCAGGTGTGAGGTGTGTTCCCGG
TGTCCAACGGACACTGCAGGACGCAGGGCAAGGGTGACGCCGCGGAGCCTGAGCATGGACGGGAGGCAGGCGGCAGGACC
TGAAGTCTCCTGCCTGCTTTCCGCAGCGCCCTGAGCAGCTTCCTCCTGGGATCCCACGGAAACCGGTTTGGGAGCAGGTT
GGCCCAGGTCGTTTGACTTTTGACTGGGGAGGAGAAGGCAGCCTCCCTTAGCGAGCCAAATGGAAGGAACAGGGTGCAGG
CCTGGGGGGGCGTGCCCAGCCAGCGGCCAGCTGAGGGTCCCCTGCTCCCCCCAGCTGCCTGCACCCAGCATCACAGCCCT
CCCTCCCCTGGCTGCAAGAGGCAGGAGGAGAACTTCAAACGGTCCCTCCCTCCACCGGCTGCTTGTCTTCTCTGGGCCAG
GCCCCTCCATGGTGGCTGAGGACTCTTTGGAACAGGGAACTGTCGTGGAAGAAAGGGCCCGGCCCTGCCACTGCCTCCTG
TGGGACCCTGGGCCAGGCTCCCCTGCTGTCCAGGCCTGTTTCCCTGTCTGTTCCATGAAGGAGAGGCAGGGGCAGTGGTC
TGTAAACCCATAAGCTGCAGCAGTAGGCCCCTCCCCTGGTCTCATGGGCCACACACGCCAGCCAGCCAGAGGTGGGTGTC
TGGAGTTGCGGGTGGGGTGCTGGCGCTGTGCCCTCTCTACCAACTTGGCTCCCTGGGGCGTGTGGGCAGCAAATGGCACC
CCCCACATTCCCTCTGTTAATTAGGCAGCCCCTGCTAATGAGTGGCTTCATCATTGCTCCATTACGCCTTCTGAGCCATC
TGTGCCCCAGGACTTAGGGGGAGACCCTGGGGGCTCCTGGCTGCTGGGCACAGGCACATGCAGAAGGCGGCATTTCTGGG
ATGTGTTGGGGGGCTGGGTGCTCCGTGGGCTTTCCACAAAGTAGTTTCAACTGCAGCTCTTCATTTTACCAAGAGAGAAG
TGGTGGCAGTCCTGGTGTCCAGGTGAGGAAACGGAGGCGCCCTCTTTATGATTCTCCCAGTGGGAAGTCTCTTGTAGCCA
ACACTTTAACACATTCTGTGCTGGGCACTGATGGGGTGTGTATGGGTGTAGGGAGTGGGGGTGGAGGTGGGGTGCACGGG
GAGATCAAAGCTGACCTCCAGGAACACTGTGGCATCGAGTGAGATGATACACAGTCCTTTCGAGTTGGAGCAAGTCCTGC
CATCTTTGCACACTGCTGTGCCACCTTGTGGGTAGTCACTAAACCTCTCTGGGCCTCATTTGGCTCCCTTGGCACAGGAG
CGACGATGCCTCACTGCCTGACCAGAGGCACCTGACACCAGGCCTGGCATCCGAGCAGCTGCCACTCAGACCTCACAGGC
CAGGGCCAAGGGAGCTGCTGATGCAAGAGGCACAATTTTAGGGGCAAGAGCAGCAAGTAGGATGGTCTTGAGGTAAGGGG
ATGCTGGTGACCCCATGCCAAGGCTGGGACCCCTAGTTCAGGAAGGGAGGTTTCTACAGAGGAGTCAGAGGCCTCTTCTG
CTGCCCTCTCCACTGGGGGAATTTGGATTTTAAAAGAGAACCCAAGCTGGGTGTGGTGGCCTACCTGTAATCCCAGCTCC
TCGTGAGGCTCAGGTGGGAGGATCATGTGAGCCCAGGCGTTTGAGGCTGCAGGGAGCTCTGATTGCATCACTGCACTCCA
GCCTGGGTGACAGAGCAGGATCCCGTCTCTAAAAAAAAAAAAAAACAAACAAAAAACCAGAGAGAGAGTGGGGAGTTTA
TGCTAAACCAAAGATACAGCAGTGGCAGTGACAGAGGCTCTCTAAGATAGGCGGATACCTCCATTTTACACAGGAAGATA
CTGAGGCACAGAAAGCTCTAATTTGGTGCAAAATCTCATGATTGGCTATTCAGAGGGCTTCAGGGTGATGCTGGGTGGCG
TCTAGGTGGAGTGATGGGCAGTTTTGAGCCCCCCAACCGCTGGGGCTCCTCCAGACAAGGCTGCTTCTAGCTGGGCGCCA
GGTAGGGATCATCCCAGGAGCCACACTCGGAGGAGGGGGCTGTGCTTCTGGCTCCAATACCCTGAGGTGCAGAGGACACCG
```

```
TGACATGCAGGGATGTGTAGAGTCTCACCGAAGAACCCAGACTTCTGGACGCCCAGCCGTGCACACTGCAAGAGGGGCCC
AGGCTGGCAGGCATGTGATTGGGTGAGGGAAGGGCTTATTAGAGGTGGCTCAGCCTTGCTCTGGGCTCAGGGGCTTCTGA
AGGCTGGGGGCAGGGGTATGGCCAGAATGACCTGTTCTGGATGGATCCATCCTTCCTGCCAAGTGCTCTTGGGAGGCTAG
GCAAAAGGTCAGACATTCCGTCATGCTTAGGAAGACGGGGCGGCCACACTGTCTACCTTCTATCTCAGGCAGCGGGGCAC
CCAGGCATGCCTTCCCCGCTGGGGAGGTCAGCAGGCGGGGGTGGACCTTGGAGGCCCAGCCAGCCTGAACCCTAAGGAAC
TGCATTCTGCAGTGGCCCTTTCCCGGCAGGGCAGGGACAGGGTGACCCTGGGGGAGGGAGAACGTCTGTCTGCATCTGGC
TCAAGGCCATTTCTTGTTGGCATGTGGTCAAGTTGCCATTGGAACCAAGAAGATGGTATGGAGGGGCAAAGCAGGACTTA
AATAAGGCAGCTCTAATCCTGTGTGACCTGAGTCAGTCCCTTTCCCTCTCTGAGTCTCAGTCTTCTCCTCTCACAAATGG
GGCAAATCCTCCTTGACAGGGTTGTGGGGGAAAATGCAAGTCACCTGCCTAGCACAGGATAAGTGCTAAACAGGGGTTCA
CCTGCTCCAAGTCTAGAACCTTCCGGGGGTGGGGTGGATAGAGGCCAGTGCTGCAGCCAGGTGTGGACGCAGGAGCCCGA
CTGGCATCCGGGGAGTGCCTCCTCCCTGACCACAAGCTCCAGCGCTTTTGAAACCAGATGCAGGTTAAACATGGGCAGGG
CACCTGCCGAGGCTCCACTCACCAAGGGAGCCAGTTAGATGGAGTCTGTCCTTGGAGCTCCAAATAAGGTATCAGAAAAT
GTCTTGGACCTGGTCATGCTCGCCCCTCCTGTGTGCCCGGCCTTTCATTCTATCATCTGAGCTGGCACCAAGGCTTGCAT
CCTAAGGCTGGTCCTGAAGCACCACTAGATTTGTCCCGTTTTAGCGATGAGGATGACCTTGCTTCCCCCGCATCTTGGCT
AGGCCAGGCTCCCTGCTCTTTCCCTGACTGGGGGATGGGCAGTGGGTGGGGGAGGCGGGCCTAGGGAACCAGACACACAG
CTCCCTTTGTGAAGGCGGCTGAGTCACATGTGCAACGCAATCCCTGCTGGGCCTGGGCCCTGAGGCGGGTGTGATGGGAG
ATGGCGTTATCATGGGACTATGGGTACCCCCACGCCTGTGTCCAGGAGGCTTTCCCACAGGAGATAGCAGGGAGGTGGCT
GGGAACATTGGCCGCAAAACACATGGTCTGAGATCAGGTCCTGACTCTGTTGCCAACTTGCTGCAAAGCTTTGGGAAGTT
CCTCTCTCTGAGCCTCAGTTTTCATCTCTGTTAAATGGGAACAATCATCACTGTTTTGTAGAGGAGGTAGTAGATGTGAA
AGGGTTCTGCAAACTGCAAATGGTGGAGTTCTGGGCCTTTGGCATTCATGTGGACTTTATCATAATAATAATGATGTTGG
CCAAGATTTATTGAGCACCTACTGTGTGCCTTGCATTGTGCTCAATGATTTATATGGTCACTCAGCTTGCAAGGGGAGAA
GCCTGGATATGAACTGCTCTTGTCTAAAAAATACCCACCTCTGAGGCTGGGCGCAGTGGCTCACACCTGTAATCCCAGCG
CTTTGGGAGGTCAAGGTGGGAGGATCGCTTGAGCTCAGGAGTTCAAGACTAGCGTGGGCAACGTATCAAGACCCTGTCTC
TCCAAAAAAATACAAAAGTTAGCCTGGTGTGGTGGGGCATGCCTATAGTCCTAGCTACTTGAGAAGTTGAGGTGGGAGGA
TCGTTTGAGCCCAGGAGATTGAGGCTGTAGAGAGCTATGATTGCATCACTACATTCCAGCAAGGGCAACAGAGCGAGGCC
CTGTCTCGAAAACAAAGAAAAAACCCACTTCTGAGCAGGCAGTGGTTGCTCATGGCCATTTGCACAGGTGGGGACAGCTC
TTCACTGTTTGCTAGGCTGGTTCCCACTAAATGCTGCTGCTCTGGCCAGAGGGTTAGAGATAGAAAAAGGGGCTGAAGAC
CTCCTCCTAGCCCTCCCAGCCCCACCATGTCTTCCTACTCCCCCAGGCCTCAGTCACAGCAGACAATAAGGATGCCCTAA
CCCCTTATCCCTGTCCTGAGGCTCTCATGTTCTTGAAGATCTCCTCCTACCTCCCCGGCAGCCTCCTCTCTACCTCCTTT
GCAGGCCCCTCTGAATAACAGTGCCCTGGCCAATGCATTGGGGTGCCCATTGTCTGCCAGGCCTGGGCTGCAAGATTTAC
GCATGTGGCCTCATTTAATCGGCCCAATAGAAGGTGCTATTTTGGTCCCCACTTGGCAGGCGAGGAGATAAATGGATGGA
GAGATGCTTGCCCAAGTCATCCAGCTGGCCGGCTGGGAGTAGAGGCCAGCTGGCCTGAACCCAGAGTCTGTGATCACAGA
CTCCGGGCTCAGAGAGGGTGCAGCAGCATTGCCCCCTCTCCTCTGTCCCTCAAAACGAAGGGTTTCCTGCACCTTCAGCT
TTCTGCTTTCCCCACACCATCTCTGGACTCCGTCGCCAGCCAATGATGCCATGACCTTCTCTGGGCCTGGCGACCCCAGT
CCCTCACTGCTGAGCCCCAACGACGTGTTTCCAGCTGCCTTGGGAAGCAGGGGGGACCTGTGGCTTCCCCACATGACACA
GAGCTGCCCCCATTCCATTGCACACAGGGCAGTCTCTTCTCTGCCTTCCGGATCTGCACCCCATCCTGTGCCCCGATTCT
TCAGGGACGTTTCAGACATCTTCCCCCTCTCCTCGGTCCCCACCTTCACTTGTCGGGTTGTGGGGGGTGGGGTGTCCCAG
CCCTTTTCCTGGTCTCTTGGACCAAAGTCTCCTCCCCGCTCTTCCCCATCTGCCTCCTTCGAGGCCCCATCCGGACCC
AACTTCCCCTCTACTCCTCTGCCCATCCCTCCGCATCATTGCTGGTACAAGTCCCTGGCGTCTAGACCAGGGCTTCTCAC
ACCTCCAATTCTGGAATCCCCTGGGCATCTTATTAAAATACACATTCAGATTCAGCAGGTCTCAGGCTATCTGTAGGTCT
GAAGACCACATTTTGAGGGCTAATCAGGCCTCTCCCTACCCAAACATCTTCTCTGGCTCCCCAGTACCTTTGATTTAAGC
CCAAACTCTACACTTTCAAAATCTTCCAGAATCTGCCCCTTGCTGCCCAGTTAGATCCCACTTCCTTCCAAGCATGGCTC
CATGCCCTACCTCTCCACCTCAGCCAATGCTGTTCCCTCTGCCTGGGGTGCCCTTCCTGCTGGTCTCTGTTTATCGAAGC
CCCCTTCTTCTCCTTCAGGTCCCAGCAGGGGCTCTGGGGTCTACACGCTTAGTTCAGATCCTGGCTGTTATAGAAAGGCG
TGGGTAGCCCTCCCTGTACCTCGGTTCCCCTTCTGTAAAGTGGGGATAGACTGTTGTGAGAACAGAAGGAGAAGATGAAG
TGCCTAGCAGGGGCCGGATATCTGGTGTCCCGTGTTTGGTTACAGCCCACACACCTGGAGGCCCAGCCAGGTCCAAGCTG
TGCGACCTTGGCTAAGTTACTTCATGTCTCTGACCATCTCTAACATACAAGTGGTACCCAACCCACAGGGTTGTGTGGAT
AAAATGAGAATGATCTGTACAAGCAGAGCATCTTACACCATAAAACCTCCAGACATAGCAGGTGATTATTAGTCCTGTTA
TCAGCACTCATAACAGATAGTGTTGTGTTTAACAAGTCATTGAATTTCTCCCCCTTTACTCCCAGAGACCCCTGGATGCT
GAATACCAACTCCAGGTATCGCTACTATGAGAACCAGGCTTGTCTCTCTAGCCAGACTCAGAGCTGGGGGCCTGCTTCCT
CACCTGCCTGCCCCTAGCTCATAGTAGGTCTCTGCAAAGTCCGACGGTGAGAGAGGCAGGCAGAGCTGGGCAGCCAACTC
CGCCCGGCCAGGCAGCCCCATAAGCCTCTCCTATGTGGCCAGAGTGGCAGCTGGCTCCGCACTCTCTTCAGCCATCTGCA
GAGAGAGTGGTGCCTTCAACTCTCCATCTGTGCCCTTCTGGTCAGCACGCTCTGTGGGGTGTGTGTGTGGTGGTGGTGGT
GGGGGGACAGCTGCCCTGTGGGCCTGGTCCCTGGGGAACAGCTGAGGCTGCCCGGGTTACCAGTTGATCAGGCCCTGAAG
GACTGACCTTGGGGAAACTGAGGCAGAGGTGGTGGTATGTCAGGGACAACCCATCCTCCCCTAGATAACTCACTTGTCCT
CCCTTGTTAGTGGGCCCCCAGTGTAGGCTGGCCTCTGCTGCTTTGGCAATAAGGGAGTTCCCACTGCAGCCCCGGGAGAG
AGCAGAGGGTATGACATGGGCCCAGTCCCCACTGTGCCTCCCTGCCCCAGGTATTGGCCCACAGCCTGGACTGGGGACTG
TGAATGTGGGAAGCACAGTGCCGTTTTCTGGAAGCTCTCACCTTATGGCCTCCCTGCACCCCACGGGGTGGCTTTGTGGC
CCTTCCTTTCTGCAGCTTGATTTCAAGCAGGTAGGTGAGGAGTGCAGTTCACCTGCAGCAGCCCTGGCTCCTAGGCTGCC
CGAGACGTTCCATTCATTCTTCCTCCACGTGGCATGGGGAAGCTTTTCCTGGCCCCCTTGTTTAGAAGGCAGATCTCTAC
CGGAGAGAAGGAATCTCAAGTTCCCCAAGCATCCACGTGTCCAGGCCCCAGGCTGAGGCCTGGGGTGCTGGATTGCTGAG
```

```
TGGTTAGGAGACCCGCTATGGAGCCAGACGGCCTGTGCTCCCGCCATCCCAACTCGGGCACCTCCCTGCTGTGTGATCTT
GGGCAGGTTAATTCTCATCTCTGAGCTACATGTATCTGCCTTAAGCTCCCTCTTCTTCATCACCACATTCACAGGGAGTA
TTTATTGAGGGCTTGGCAAGTACCAGGCACTGGTCTGAGTAGCAGGTTTGACTTCTCTCCACTGCCAGGGGCGCTCTGGA
GCTGGAATGCAGACGGTGGTCAGCGAGTGTAATTACTGTTATTGTCACCACTCACCCTTCTGGCATTGTAAAAGCCTCCC
AGCACATCTGACTGAGGAATCATCTATCCTCATTGCTCGGAGGAGGAGGGCAGACAGGAGCCTCTGCAGGGAGTGTGAGT
TATCCAAAGCCTCCTGACTCGAGAAGGGCACTGAACCCCAGCGAGGGAGGTGCTGCTGACAGAGGAATGACCAGAAGGG
GGATGGGGCATGGTCCATTTCCACACTTCTCTGTCTGAGCTTTTGGGGCCACTGGGAATGGCTCTTCACCCAGGCCTCGG
TTTCCCCTTTGGCTCAGCAGATGGATTAAAGGAGGCCGTTTCCAGAAGCCCCTGGGGTCTCAGAATCCAAAATGACTTGG
GATGTTGAAACTGTGGGTGCTTGAGGAGCACTGAGAGGGGAGACGGGTCACCCCCCGGGGGGACGCAGGTCAGCCGGTCA
GTCGTGCGTGGCCTGGCTGGCCTCCTGCCCGGCTGCCTGTCACCCGCTCCCTCCCGACTGCCTTCCGCTCTGCCGGTCAG
CACTGGCTCCCGTCGGCTGCCAAGGAGGCTGGACTCTCCTTCAGGAAAGGGCAGTTGTCGCCCTGACCTGGGGGGTGGGC
GGACAGCATCCAAGGGCCCTGTTGCCGTGGCAACCCCGGCCTCTCTGTGGGAACTTTGGGGAGACTGGCCCTGGGAGGCG
ACAGATGGAGGCTGAGGAAGCAGAGTGATCCCCGGCCCCTTCCTGTCCCTGAACACTGTCTGCCTGGATGAGGGGTGAGG
CTGAGGTGCCCCCTTGCCCGGCGCTGAGTCATGGGCATCCCCCAGGATCACTGCCTCAGCGCTCTCAGCCCTGCCCCCTC
CTGGTCTCCCTCTGAGAAATCCATGACTCACATATATCGTCCGTCATCCCCAGTCAAGCTCAGGGAGGAGGGAGGAGCCC
AGCTGGGCAGGAGAGGGGTGTCTCCCCACCAAACAAACCCGGTCCCTCCCTCTCCGCACTAGCTGTCTGCCCTGCCCTG
CCGTAGGAGATGGGCTGGGAGCCTCCCACGCTCTCCAGCTCACTCGGCAGGCAGCGGGGACCAGGGCTGGCAGGTTAAGC
CTCTGGGGGTGGATCCTGAAAGGTGGTCCAGCCGCCTGGCCCTGCGTGGGACCCTCCACCTGGCAGCAGGTACCCAAACA
AGGGCTGGACAGCAGGTGGGAAGGGGAATAGTGTGAGTGTGAGAGTGTGAGTGTGTGAGCGTGAGTGTGTGAGAGTGTGA
GTGTGTGAGTGTGAGTGTGTGAGAGTGAGTGTGAGTGTGAGTGTGAGCGTGAGAGTGTGAGAGTGTGTGAGTGTGAGTGT
GTGAGAGTGAGTGAGAATGTGAGTGTGAGTGTGAGAGTGTGACTGAGCGTTTGAGTGTGAGAGTGTGAGTGAGTGTGTGT
GAGTATGAGTGTGAGTGTGAGTGTGGGAGTGTATGTGAGTGTAAGTGTGGATGTGTGCATCCGTGCACGGGTGGCTGTTG
GACAGTGGGTAGGGATGGTGGTGAAATGGGGAAATGGGGGGGGTCTTGGTATCTTGGTGGGATCTAGTCCAGGTCAGCATG
GCTGTGGGTGGGTCTCTAGAGTGGGGGCGGACCCACAAGGAGATGCCTGGGAGGGGACTGGGGCCCAAGGGGAGCGACAC
CTCCCGGGGCTCCCAGGCATTTTGTTGCTGGAGAAACTGAGGCTCAGAGTGGTACAGTCACTGGGCCCAGCAAGCTGGGG
TCTGCTCTTTAGGCTGTGAGATCAGTGCTACTACACAGATGGGAGAACCGAGGCTCTGGGAGGGCAGGTGGCTTGCC
CAAGGTCACCTAGCACATCCAGGTATAGAAAGCCAGGGGCCAGGCTCAGGGAGGGGGTGGCACTTGAGGGAACTCCTGAG
GACAGCTTTCCTGCAGCTGTCCCTGGGGCTGGGTCCGGGGTCCCCACTGAGCAAGGGACAGCCGTGCTCTTCATCACCAA
GGCCAGCTTTGGGGAGAAGGTGGCCCCCGCAGGGTCTCTGAACGTGGGCCACGTGGGTGAGAGTCCTGGAACCGGTGACAGG
CAGACGGGGAAGCGCAGGCTGCGGGCTGAACACCCTGCCCGTTGCCACCAGACCTCAGGGCTCCATCTGCCTGGCACTGA
TAGGACTTCCCACCCTGGTCCCCGCGGGGGCTGGGAGTGTGTAGGAAACCAATGATTCCTGTCCTCCCCCGTCCCCAACC
CACCTCCCCTGCGTGGTAGAGTTGGGGTCTCCCTGTGCCCCACTGGGAGAGGTCTGATGTTCATTTTACTGCGTAGGGAG
GAGGGGATGGGTCAAGGCAGGGGCCTCCCACCCCCACCAATGTGGAGCAGTTGTAGCCTGGGGTCCACGGGCTGTGTAGG
AATAGCCTTTGTTGCTCCTTCAGTGGACCAAGAAAGTTATTTATAGAAGCATTTATTGAGCGCTTACTGTGCACTCTGTG
CTCTGTCGGGAACATGGCCGTGGGTGAGGCAAATAAGGTATCTCAGAACTCGTGAGTGGCCCAGACCCAGAGCCAAGCCC
CCTGCCCCTCCCAGAGGATAGCCCCGGGCAGCCTGTTGGCTCCTTGGGGTCAGAGACTGTGTGAGAAGGAGCATATCTGG
GCATTAATAACCATGGGCTGCCAAGTTGGCTCCCCAGAGCCCAGGCAGGGGTCCTGTTGGGCTCTTTCCTGCCAATGTCC
AGGTGTGGAATGAGCTCTTAGGGCCAGACCTGACCCCTTCTTTGCTGCAGGGACCCGAGATATGCACCCACCTTGGTCAC
AGCATCACTGTGGCCCCCTTGCCCTTCCTCTCCTCTCGCCTCAGGAGCTCAGCTGCTAAATGGGTGTGTAGGGGTGATCA
CAGCTGCCCTGGCTCCCGTAGGCCTGGGGCTTGGCTCCGAGAGTGTCGGGTGATGGAGAACGCCGCTCCAGGTGTGATCT
GTGACGGACAGGCTGTGTCCCGGCCTGGGCCACTGGGCAAGGGCCAGTCAGCCTGGTGTGACACTTCCTTTTGTCAGGGA
TACAAACAAACAAACAACAGTTAGGAGGTGAGATGGAGGCGTGAGATAAGCTTGCTAACAGAGGGCCCAGAGAAGGGGAA
GAGGACCAGGGAGCCTCTGCGGAAGGCACTTAGGCTGGGCTTGGGAGAAGGTGGGAGGGGAAGGGACTGATGTCTGTATG
AGCACTGTAGGGGGAGTCCTAGTGGAGGAAACTACTTAGGAAAACATTTGGGGAACGATACAAGCTGGGGACCACGGGGA
GGGTGATACCGGACCCCTGGCATGGGTCATTTCATGGGACTTGATGAGCCAGGCCAGCAGGTCTCAATCTGCTCTGTGGG
GGGGAAGAGAGGCAGGAGGACTCGCCAGGCAGGTGCTGTGTGGGGCTGGGGGCCCAGGCTGCAAACCCAGCAGGACGGGG
AGGGCCAGGATGGGTGTTCAGTGTGAGGTTGGCCAGGCAGTCCCAGTCTCTGGCTGCTGGGTTAAGGCTCAAGCTTGGGA
GCAGCTTCACGTGTGAGGGAGACGAGGGGGAGGTGGCGCCCAGTCGAGGGGAAGGACAGGAGAGAGGTGGCAGAGCTGCA
GCAGGGTTGGTGTAGGGGTTGGCCTCCTGGCAAGGCCCAGCATGACAAAGGCAGGTAAGAGGAGTGGGCAGGACAGCCAT
GGAGGGACCACTGCGGACCGTCCTCCAGCCGGACTGGGATGCGGGTTCCGCCATCCTGTGCAGAAGCTGTGTGCAGGTGG
GGTCTGGGGGGCTGTGCCATTTCAGGGATGCAGGATGGGCTTCCATGTGGCAGGGCTCCCAGGGAGGCCATGGAGGAGGC
CGTGGGGAAGTCGGGTGGAAGTAGGGAGGAGAGGGGAGTGTGACAACAGAGCCCCAAACCCCAGCACCACCTCCTCCTCC
CAGACTGGAGCTTGATCAGGGCTCAGCCCCCGTCCCCATGCTGGGGGTGGCCCTGTGGACCCCGCCTTCTGACTCACAGC
AGATGGGGACATCATGTGAGCAAACCCAGCAGTCAGGGGTGACCAGACACAGCAACTGGGCAGATGGCAAATGGGGGGCG
GGGCGGGCTGGCACAAAGGGGTCTCTCAAACCGGGTGGGGCGGGGCTGTGGCACGGCCTCAGCCTCCCAGGCCTCCTTTC
TCCTGAGCCGTCACTTGGCTGAGACCCTGCCCCTCCTCCTGTCTCTGGTGCCCCATCTGGGGCTCCTTGAGGCCCCCAGG
GGAGCCCTGTAGATTCTGGTCTTAGGTCTGGACTGCTGGGTAGAGGGGCCAAGGGTTGGGGAGTAGGAAGGGACACACAT
TTTCTGACCACTGCCCAGTGCCAGCCTGCCCTGGCAGGAGGTCAGCTAAGCCCCAAACTCTCCCTGGTTCCCTGATGGCT
CAGCCTGGATGGGAGCAATCCTGGACCTTCCCTGACCTCAGATTCTGCCAGGCCCTGGGCTCAGTGCCTTGCACACGACA
CCCACTTTTCAGATGAGGACATTGAGGCAGGGGAGGCTTACCCAGTGAGCAACCCCACATCTTTGGGGATCCAGAATTTG
GGCTCTTGTCCCTTGGTGGAGAGGAGGCTCCTCAGCCAGCCAGGCACATGCTCGTGTCTTGAACCCTGCCCCTGCCACTG
```

```
CCAAATCCACGCACCTGTAGGGTGACAGGGCCCGTAGAGACAGCAAGGGTGGCTTGCCCAGGGAGAATCTTGGCTTTGTT
TCTCTCTGGTTGTGGGAACATGGGCCTGTGCCTCGGTTTCCTCATCTGTAAACTGTGGATCACAATCACATCTGCTTCAG
AGGCTATTATGAGCATGAAGAGAGTTGATAGAGGAAAGCATATGGGAAAGTGCCTTTCCCATACGTGGGAATGTTAAGCC
TTTTTTTAATACCTCTCCCATTGGACAGATGGCTGTATTGAGGCCCAGAGATAACCTGAAATAACTGTGAGCACTTCTCC
CTGCCCCCGCTACTGCTGGGAGAGCTGCGGCCACCTGGACACCAGTGGCGCTCACTCACCCACCTCCAGCCTGACTCGTG
CTTTGGTGAGGAGGCCAGTGTGGTGGGCGTCGGGCTCCCGAGCTGGCGCCTGCGCAGGCTCAGGCAGTTTGTGGTATGCT
TGGCAGTTCCAGGCAGAGATAAAAGCTCAGCCCCGGAGGAGCTGGCATGCTTCCGGGCACCGGTGGACAGGCCTCTCTGC
ATTAGTGGGAGATGGCCTGGCGGTGGGGAGATGGCCTTGCTGTCCAAGAGCCAGCCCAACCCTGGTGGCCTGAAAAGTTC
TGCGGTGAATGGCTCTGGCTTGGCTTCCCAGACTCGGGGCCACTTACACCAACTGCAAAACAAGGAACAGTGAGATAGAG
AGCCGCCTCTGCCACCTCCCAGGCCCTTTCCCCTCCCGTCCCCAGGTCTCTGTCCTCCTAGTACCCCCAGCCTGGATGCT
CAAGGCCCATGGGGGAAAGCTGACTTTTGAGAGGAGCTGTGGAGACTTGCATTCCACTGCCAAAGATGACAGCTTCTCTG
GACCCGTTTCCCTGCCTGTAAATGGAGATAACAATACATCCCTTTGCAGGGTTGCTGTGGAATTTGGGGCAAAAGTGGCT
GCAAAGTATGTACCTAGCACAGTGCATGACTTCAAGAGGTTCTCCCTAAACCTCAGCTGGAGGTTTAATCTTCCAGGTCA
TGGGCTCATTGACTTAGGCTCAAATCCTTGAAAAATGACTTAGAGCAAGCGGCTGAATTTCTCACAACTTCATTACTTGG
GCTGTGAAAGTCTCTGATACCTAAATGCGGCTTTGTGGGGATGAACCGAGACAGCCACACGTCATAGGGGCTCATGAGAC
GCAGACCCTGTCCCCAGCTGTCCCAGACCTTTGCTTTTCCATCCCTCAGCCAGGCTGCCCCCTAGGAGAGGCAGCACGAG
GTGGGCTGTTAGGGGTGTGGGCTCTGGGGCTGCCTGATTCTGTCCCAGCTCTACTGGCTGTGTGGCCTGGGACAGGTATA
TTAACCTCTCTGTGCCTCGTCTTCCTAATTGTAAACATGAGGCTGGTAATGGCATCCTGAGAGGATTAAGTGAGCCTCTG
TATTTTAAGTGCTGAGTGCAGGGCTGGGCACACCTCAAGTGCTATTGTAATTGCCCCTTCTGGGCCATGACCAAGCCTTG
TTCTTAGAGAGCATTGGAGACTTTATCCGGAACAATGGTGCCATCCTCCCAGGATGTTACCGGGAAAACACCAATCCTG
TGACAGCTTCCCCTTTGGACATGCCCATTCCTGGGGTCTTGATCTGCTGCCCCGAGGCATTGTCCAGCCTGGGACTCCTT
CATGTTCCCCAAGACCTGAGACCTGACCCAGGTTTTGGAGTGAGTTAATGTGACCCTGGGCTGGGGAGCTGAGCTGGAGC
CATTACCCACAGAACCGCCGACCTTTCAAAGGACCAGAGGCCATGGCCCTAGCCCTGGAGAGGAGGCCTGGGTTCCAGTC
GAGCTGGTTTCCTGGGACAGAACAGCACTGTGGTCACTTCTCTGCTGGGTGAGTTCACCCTAGGCCCCTGGGAGCTAGGC
TGAGCAGCTGACCCCTTCTGGGTCCTTGGCTGAGTCTGGGCCTCAGGGTGTGGCTCTGTGGCTGGCACCCTGGGTCTCGG
ACCCTGTGGCAGGGGGTCAGACAACTGCCCAGACCCACCTGCTTTGATGGTGACAGATGCTAGGGGTGAGCCCAAGGGGC
TGGCAGGGGCCCCTGAGTCATGCTCAGCCCTGTGCCCTCTGCCCACTGCTCCTCTGCTGTGTCACCATCTGGGCACGGGC
CAAACAGTTCAGGGGCGACTCACAGCCCAAACTTCCGCCCCTGGGGCATACACTTCACTGCCTACAAAGACCCTCATTCA
ATCTTGAGTGCTGCTGGCCCTCTAAGACTTGAAACTTTATCCACATTTCACAGATGAGGAATCAGGCCCAAGAGCTCGGA
GCAGTCAGTGGTAGCAGAGCCCGAACCCAGTCTCCTGCCTCTTAGCCTGGACTTTTCCCCAGAGTACCACAGATGGCCAT
TCCCATGATAGGTAGTGAGTTCCCCACCTTTGTAGGTGTTCAAGTGTGGCAGGGGCGTTGCAGAAGCAACTTTGGCATCG
CATGGGGCTCCTCTTCATGGCTGCTTCCAGCCCTGAGCTTTCCTAGCCTTGGGGGAGATGGGCACCCAATTCCCACATTC
CCCGGAGTCGCTATGTCCTTAAGCATCACATGGGTCTTTCTAGCCGGCATCAGGCCATGCTTGTGGGCAGGTGGTGCCTCT
ATCTAGAAACCCTCTTCATAGCCTGGCTGTCTGGCAAGGTTCTTCCAAGGAACCTCCACCATGAAAGCCATGAATGCATC
TTCAGAGACCCCTGTCCATGTGTAAATAGCTCAGTGGGCTGGATGGGAGGGAGCAGAGGGGGAGAGGGACCCAGCTGAAG
AGGACTGGAGTGAACAGGGAGAGGCTGAGGGAGGTCAGGAGGGCAGGACACCCACCTCCTGAATCTTCAGGACCCCTTGC
TCTCCCTTATCCAACAGGCTTGAACCTGAATAGGGGCATTCCTGACCCCATGGCACTTCCCCTGCCCCCCACCTGCCTAG
AGCCATGGGCAGTGGGCAAAGCTGGAAGAAACCCTCGTGCACAGCTTCCTCAAAGCTGTACTTATCCTATGCCATGGCTG
TCCTGGCCACACCTTCTGGGGGACTCTGCCACCTGTGTGCACACCTAGGGGACCAGGTGCACACGGTCTGTCCTCCAGCT
CCTCTCCTGCCTGCCTCCTGGCACCTTAAGTGGAGGTGGTGGGAGCTGGGACCAGTGCAGCACCCTTGCCAGCCATGGTG
GGGGAGGACAGCCAGTGTCTCTTCATGGAGATCCTGCAGCAGGTGGTGGCATGCCCCAGGGTCCCGTCTCTGTCTACCTA
CCTGGGTTTGGGAAACTGGAGTCAGAACCTTTAGTGATTGCGATCTAGGAAGGACGGTAGTGTTTGCAAACCACGTGAGC
ACAGGAGTGAGCAAGGTCCAACCCCGCCCCTGTTCCCTGGGCCGTGAGGCTTTGGGCCCTGTGGCTCAGCCCCTCTGAGC
CTCCCTTTCCCTCTCTATGGAGTAGCTGCTGCCGACTCTCCATGTCGTTGCCAGGGCATGCAGGACAACAGAGGCAGCCC
AGGGAAAGGAGTGCACCATGCTCCTTTGGATCCAGTTTTACGAGCTCTGTGTCCCTGGGCAAGCTCCGTGTCCCTGTGTC
CCTGCTATAAAATGGGAATGATAGTACCTCCTCATAGAGTGGCCCTGGAAGCATGGGAGGCCAAGTGCATATCCTATGGT
TTGCACTTGTACGTGGCTCCCTGCCCTGCCCCAGCTCTGCCTCGTGAGTCTCTGCAGACTAGTCCTCGGGACACACCCCA
CCCCCAGCCTGCTAGATCGCAGGACCCCATGGCCATAGGCACGTGCCCTTTCTCCTCCTTGCTTGACCTTGGAGAGCCCA
TGGGGAGCCCAGGTGGGAGCTTGGGGTCTGCAGCAATCGGCAACGGGACCCACTTTTCTTTGCACATCTGCTACCTGGGCT
CCCCTTAGGCCCGAACCACAGGAGTGGGCCTTTGAGGGTCTTGGGGGTGGTAATTTTGCTTTAATGTAGAGAGAAAATCC
AAGGTTCAGTGCTCACAGATTCAGATGGAATCTCAGACATCTTCTAATCCAGCAGCCATTAAAGCTGTCTGTGGCCCCAG
TGGCATAGGAAAGGCATGCGCTCCCAGGCTTCCATGCTGTGGGACTTCTCAGAGCCTTTATCGGGAAAACAAAGGGGAGT
TTGCCTTGTGTTTCAGATCCTATCAGGCTATGCACAGTCTTGGGAACCGTTCTCTGGCCAAGCAGCCGGCAGAGGTGTGT
GTGCTCCTGATCCTGTTAGATGCTGGAGAGTGGAGATGGCTCAGTGCTTGAACCCTCTCCATCCCTATCCCCTACCCCT
CACATAGCCTGAGTGGCACTTTTGGGTTGGATCTTCTAGATCTTCTATCAGGAATGGCTATCTAGTATCTATCAGGAATG
GCTCCTTTTGGGAAGAGGTGTGGAGCCTTGGCCTGCCCTGGGTTAGAGTCCCAGCTCCGTCTCAGTCTTCATTTACTCATCC
AGCACTTCACTGGGGATCTTCTATGGACCAGGCACTGTGCTAGGTACTGGAGATGCAGGTGGTGAGCAAGACAGGTGAGAC
AGCAAAGCTGAGCAAGGGGATAACAGGTAAGCAAATGGCAGAGTCATGTCAATGTTGGGTGCCAATACGGGTAATAAATCA
CATGGAAGCGAGTGATGGATACGGAAGATAATTTTAATTGGGTGGTCATGGAAGGCTTCTCAGAGGAAGGGACATTTGAG
TTGAGGCCTGAGAAGGAGGCAGTCAGGGAAAGGGTCCTGGAAAGGATGTGCTATGCTGAGGGAAAAGTGCACGCAAAGAC
CCTGAGGTGTGTCTGAGGACAGAAGTCTAGAGTGGCTGGAGTTGGCGACTGCAAGGAAGGGATGGGGTGGCGTAAGGCTG
```

```
GGGGACGGGAGTAGGCTTGTGGTCACTGGCTTGTGATTTCAGCAGTCCTGCTGCTCCCTGAGCCTCAGTTCTCTGACTTA
CAAAATGAGCTTCATCATTTCAGTCCCCGCCCCGACCCTGGTCGGGAATGTTGTAGGTGTGAAGTAAGACAGTGCTTTGG
CTTTTGGCTCTGGGCAGCAATGAGCTATTGCAGTTATTTTTATTATTACTATGAGTGGGATGTTTAGGGACTATGGGTGG
GGCTGGGGAAGCTGTGCCTCTGTTTCTCCGGGTTGCTGTTGTAAGTGAGTGCCAAGTGTATGTAAAAGGCGTGGTGTGCC
TCGTCTGTACCTCCCCCCAGGTCCCCTGAACCACAGCCCCTTGCCAGCTCCCTTTAAGCACAGGATCCTTTGATGCAACA
GAGGCCGCCTTCTCCGGCCAGCAAGCCTTGTGAGCCTTTCCGGGAGAAGGTTTCACCAGAGAACAGCAAGTCCTTTCTTT
GCTTTCTTTTCCTCTTCCTCCGGGGGGCCAAAAATAGGGCCTGAATGGGACCCAGCTCTGGGCCTGCAGTGAGACAATAGC
AGCCTCCTGACTGAAGCTGCCTCCTGGCTCTGCTCCACGGAGGGGCGGGGGAGAAGGGGAGTTGCAGAGACACCATCCCC
TCAGCCCCAAGCCCCTGCTGCCTTTCCCATGGCCTCTGCCAGAAATCATCCCAAAGCCCCTTCCTCTGTTGGACTCCCCT
CAGAATGTGATTGCCAGTGGGGTTCCCCACTTGTGCTCTGAGAAAGTGCTTGTAGCTGGTTGACTGAGACCCCAGGACCT
TTTGCACCCCTGAGCAACCCCACCCTATCTGCTGGCCTGGAACCGAAAACCTCGGTGGGGGTTCTAACCCCTGGTTCTTT
TCTGTGCCCTGTCTGGGGCGTACCACAGTTTTTATGTCCTTTTCCCTTTGACTCAGTGGGTATGAAAAACTTAGAGATCA
AAGGAATGTGCCCTCTTGGAAGAGAGCGAGCTCCCTGTCACTGGAGATGTTCAAATAAAGTTGGCTGAGCATTGAACGTG
GGGGAGAGTTAGGGATTAGCCAAGTGCCAGATGGACAACTGGTCTTGATAGCTTCATGTCCCAGAGAAAATCTCCATTTA
AATTCCATACCCTTCCCTTACCAGCTAGGTGGCTCACAGCAAGTCACATCCCCCGAGTCTCTGTTTCCCAATCTGTAAGG
TGGGTCCCCTAGGGCTAACCTTGCAGAGTGGTAGAAATGGATCTCCAAAATGCCCAGTGTAGTTCCTGGCACACAGTAGG
AGTTTCAGAAGCTCTGCCTCTATTTTCCTCCTTCCCCAGACTGGCAAAAGAGAATCTGAGCTCCCAGCCCCGCCCCGGGA
GCTCTAGAAGGAAGCCAGGAGGAACCTCCAGGCCTAGGGCTAACTCAGAAGGGGGATTCTTGGTAGGCTGTGGCAATCTG
AGGAGGCTTCCTGGAGGAGGTGGACCCTAAGGAGTTTTCAGAGAAATATGATGCACACATTGTGCTCACAAAAGCCAGTG
GTGGGATTAGAGGGTGTTCAGCTTGTTTTGAGGGCAGGGCCACAGGCTGGCAGCTTCCAGAAGCAAATCCAGCTACTGTT
AGGCATAATTTGGTTACTGGGTGGCCTGCACTGGTTCTAAAAAGATTTGGTTTTGGGCCAGGCGCGGTGGCTCACACCT
GTAATCCCAGCACTTTGGGAGGCCGAGGCGGGCATATCATGAGGTCAGGAGTTCGACACCAGCCTGGCCAGCATAGTGAA
ACCCCGTCTCTACTAAAAATACAAAAATTAGCCGGGCATGGTGGTGGGCACCTGTAGTCCCAGCTACTCGGGAGGCTGAG
GCAGGAGAATCGCTTGAAACTGGAAGGCTGAGGTTGCAGTGAGCTGAGATCACGCCACTGCACTCCAGCCTGGGCGAAAG
AGCGAAACTCTGTTTGAGAAAAAAAAAAAAAAAGATTTGGTTTTAAAGTCAATGTTGACAAACTTGCAGATTTCATGTAAA
AACCTGGGGCTCTGCCTCTCTCGAGAAATTGGAAGCTCTGGCTCTTTGTGGTCCCTTCAAGACAGGCAGGTGTCCCCGGG
GCTCCCCATAAATCGCTGTCCTAACCCCTGCCCTCCCTCCTGCCAGCTCCCTGTCTGGCCTGGGCAGCGTCTGAGTTGAG
GACTTGGGAACAGGACAAGTTACGGAGCCACGTTGCTTTGCTGGGTCTGAGCCGGGTGTGACGTAGTCCCTGCAGCTGC
CAACGGTTGCCAGGGCAACGGTTGCCAGGGGCTGCTGTCACCTGCGCCCCTTCTCCCGCGCTGGCGGCTGGGGCTTCTCA
GCCTCTATTCCCTGGCTGTCCCCTTTGTTTGAAGCTCCAGTGAGGGAGCAGTGGCTGGGGTGGCCCAGCTTCAAAGTCTC
TGTCCTCTTGAAAAAAGGTGGTCGGGGGACATTGACCCACCAGCCCCGCAGGCTACTGCCTGCAAACAAGAACCCCTCAT
CTGCCACGCACGTTCTTAATGTATCTATAGTCTGCCTGATGCCACAAAGGAGTCCAGGTACGATTCTCCCGCCTCCTCTC
CCCTCCTCCCCGTCCCTTTCACTCCTGCTCCCTCTCCCTGCTCCCCGCTTCTCCCCTGTCTTCCTCCTTCACTCCCCTTC
CCCTCCAGCTCGCCTCCTTCCTCCCCATCCCTTCCCCTTTATCTTTTTCCTCTCCCGTGCATTCCCCTTCTCCCCAGCAT
TGATGGTGTAGCCAGTGGGTGGGGGTGTGGTGGCACTAGGGACCCTGGGGGGGTCAGATGGTACTCCAAGGGACTTGGGC
AGTTTAAGACAGCAGCCTCAACTACAAGGAAATAGTTGGGGGTGAGAGCTCCACACCCGTCCCTGCAGCCCAGCCCTTGC
TGAAGATTCTTTTGGGTCCCTGGGCTGCACCCCCGGTGTCAGAAACAAGCCTCTGACAAGTCCAGGGCGTGAGGGGACTG
GAAGTGCAGGAAAAGAGCCACCAGTGGGGCTGGGATCCAAGGTCACCTGGGTGAGTTCTGATGGGCTCCCTGATTTGGGCC
CTTGGGAGGATGGCGTCCAGCTGAACCCATGGAAACACATTGTTGGAAGCTTTCTTTACTGTGACTTTTCAAGAGATGTG
CTTTCTAAGAGTCAGGTAAGTTCAGGAGGGAAATAGGCTTGCAGATGGACAGCGTGTGTCCTGGAGCCAGCCCTGGGTTC
GCATCCAATCTTCATTACTTGCTAGCTGTGTGACCTCGGGCAGGTAACTTGACCTCCCTGGGCCTCGCTGGCCTTATTTG
TTGAGTGGGTGTGATGGTACTTGTCTCAAGGGTTTTGAGGAAGCTTAAATGCGTGAACATGTGTAAAAAGCTCACAATCC
TGTCTTGCATTTTCGAAGAGCTTAATGAACATAGTTGTGTCACTGGAGGTGGTGAGCACCCCGCCTGCGGGGTGTGCAAC
CTCTGATGGTCAGTGATGCTGTGTGCTCAGGAGTGGGGATGGAACTCAGTGCTTCTACTTCCCTTCCCTGGACGG
GGCCCAGGCACAGTCAGGCTCGGGCCCCCAGCCTTGGTAGGGGAGAACTTTGTTCTTCTCAGCCTACTTAGTAGGGGAGG
GTGCTCTCGGCCCCTCAGCCAGTGATCCAGGACCTCGAGGAGGGGGAGGAAAAAAGAAAGGGAGGGTTGGTGGCCAGCCT
GGGAATGCTAAGTGTCCTGCGCTCCAAAGATGGGAGTTTGCCAGACCTGGGGTAGTTGAACTGCTAAGCAAGGAGGGAGG
TGACTGGGCTGACCTTGCGGGCCTGGCCTTGGGCAACGTGGGAACACAGATAGGGAAGGGGAACCTCAGCCAGACAACTG
AGGTCCGGCTGTGGTTTGAGAGGGAAGACAGTCTAGAGGGGTCAGGGGAGACGGTTCAGCATCCTCTTTGTCCCTTGCTA
CCAGCTTGGAGGTAAGCTCTGTTCTTACCTGTAGGCATCCTGAAGCCTGCGGGAGCCTGGAGGGCCCTGGCTGCAGAGAA
GGCAGTCAGGGGACAAACATCAGAGTTGCCCACTTGACAGAGGAGAAAATAAGGCCCAGAGAAGGGACAGGCTGGCCTCA
AGATTCCAGTTGGCTGCAGAGCTTGTCTGAACCTTATGCCCCTAACTCCTGCCACCCCTGCTCTAGGCATGTACATTCCC
TGGGCAGGTACTGACTGGTGTGTGCCCTGGCGCCCAGGAGGCTGGCCTCAGCTGCTCCTTGGAGATGGCCAGGTGGAGAC
TTGGCCATGAACCTCTACGTGGCTGCAGACCACCCCAGGAGCATAAGCTGAACAGGCCTGGCACCAGAGTCTTCGGACTG
CTGGCTCTTTGGCTCTTTGAGGTCCCTTCAAGACAGGCTGGTGTCCCCAGTGCCTTCCTAATCCCTGTTTCTCCTTTAGG
TTCTAAGTCTCTTCAATCAATTCTAAAAGTCCTTTTTTTTTTTTTTTTTGAGACAGGGTCTCGCTCTGTCACACAGGCT
GGAGTGCAGTGGTGTGATCTTGGCTCATCGTAACCTCCACCTCCCGGGTTCAAGTGATTCTCATGCCTCAGCCTCCCGAG
TAGCTGGGATTACAGGTGTGTGCCACTATGCCCAGTAATTTTGTTTGTTTGTTTAGAGATAGGGTTTCACTGTGTTGGCC
AGACTGGTCTCGAATTCCTGGCCTCAAATGATCTGCCCACCTTGGCTTCCCAAAGTGCTGGGATTACAGGCGTGAGTCGC
CGTGCCTGGCCTCCAAAATTCTTCTTTTCTTTTTTTTGTTAATTTGTATTTAGAGACAAGGTCTCACTCTGTCACCCAGG
CTTGAGTACTGCGGCATGATCATAGCTCACTGCAGCCTCAAACTCCTGGGCTCAAGCAATTCTCTTGCCTCAGCCTCCCA
```

```
AGTAGCTGGTACTACAGGCAAGCACTACTACCCCTGGCTTCAATTCCAAAATTCTAAGAGACTTTGATTCTCATCTCCTA
AAACTCTTGCATCTTCATATTCTTTCTCTCTCTTTTCCAACATCCTGAGTGTCTTAGCTGCTTGAGATGAAGTCACAGGC
CCAGCTGGCCACGGCCAACTTTAATGGAATGGCACACCTGGGACAGGCCTGGCAGTGCCCTTTTCCCCACGAGCTGGTGC
TGCCGGCCGAAGGGAGGTGGGATAGGCCTGAATATACCCCAGGCTCACCAGGGACCTCTTTCCTGGAAGTGTCAGGGATG
ATGATGAGTGGCCCTGGGGAATGCCTAAGTGTCTCCTCTAGCTCATCTCATGCTAGCTAATGACCTTTGGGACAGCTCAA
AGGGGAAGGGAGGGGAGGGGGCTGGAGGTGCTGGCAGGCTTCTGTGTGGCGTCAAGATTTCTGAGAGCTTTTTTCCATGT
GGATAACAGGTGTCCGTGCAAAAAAGGCTCTACCTTCACGTTGGGGTTGGGAAACTGCCCAACCAAGCTTTATGGGTTTCT
TTCCTGCAGGACTTATCAGAGCCTTTGATATGCTAATATACAACAGAAAGTATGGCCCACAAGCATTTCTCTAATCTGGG
GACTAGTGCTCCCTGAAAGTGCTTTGGCAAGTTACCTCATGGTAAAGATGGGGAGACTGAGTCCCAGAGAAGAGAAAGAA
CTCAGAAGACCACAATGGCTAAAAGTTTGGGCTTGGAGGTTAGGCGGACCTGAGTTTGAGTCCCGGCTTTTCTGCTTATT
TGCTGTGTGATCTTGGGCATTACTTAACCTCTTTGGGCTTCAGTTTCCTAAAGTGGGGACACTGATAGCATTTGCTTCAC
CAAGTTGCTGCGAGGATTGCATGATTCATGGGCTCTGCTCATAGGGTTACTGTGAGGATTGAACAATTAATTTTTTGCCA
AGTGCTTTGCAGGATGCCAGCGGCTAATTGAGCACTTGGTGAGTGGAAGCTGTGATCCTCCCTCTCCTCTTAGAATCTCC
CTGGATTCCAGGGCCCTTTTCACTGCACCAGATAAGACCCCTGTTTAGTCCCACTCATCATCTGGTGTCCTGCCAGGCAT
GGGGACTGGGAGGATGTGGGGGCCTGAGGCCAGGACACTGGGACTCTCACAGACGTGTCACAAGGTTGTCATCACAGGGA
GGAGGACCAGTGTGTGCAAAGGCTTGGAGGTGGGAGCATCCAAGGCCAGGCTTCTTCCTGGCTGGATGACTATATGTTGG
CTTCTCTGAGGCCTCTTAAGCAAATGCCCAGTTCCGCATATGGGAGGCAGCAGGGCAGCCGGCTTTGGACAACTGACTCT
CTGGGAGGACTTGGAGTCCCTTCCTATGAGTGGGTCCTTCCCTCTCAGGCTGGCAGGGAGTGCCCCAACTTCTCCCCTTT
GTCACTCTTCCCACAGGGTGTGTCTGGGCAGGCAGAGGGTGTTGTGGCTCAAGCACTTGGTTGGACCAGAAGCCACGGGG
ACAGTGAGGCCTGAGCTGGCGGTATGGGGGGTGGGGGGTGTCCAGCAGGTCTGGTGTAAGAGTGTGAGGCTCACCCTGGG
CAGTGAGTGGGCCAGGCCTGGGCACCTGCCAGCCTCCGAGGCAGGCAGCTTGCTTCATTTTATTCTCCTTTATTGTAGGA
AAATTCCTGAAAACTGAAGTCACTCTGCTTCTGCCAGTGATCCTGGAATACCCCTCTTGGGGAGCTCTCTGTGGTCACCT
AGCTTGGACTTCCGTCACTCACTTGGGGAGTGGGAATGGGGGAGGGGAGTGGGAATAGGGGAGGAGAGTGCTTCTGCCTT
TGCAAGCTGTTATCCCAGGACCCATGGGTGCGGCTGAGGGGTGGGGGGGACTGGGCTGGGGGGTGGGGCGTAGCCCTGAGTG
CCACCTGGGTCTGACAGGGGTGTCTACTTTGAGCTGTTGGGTACATGCTATTTCCAAGTAAGATTTACTTTTATTAAAAA
AGTTTGGAAACCGTTGGAGGGAGGGTCCTCTCAGACGCCACGCCACCCACCATGGAGGAAACACCTGCAAGAGCCCTGCT
CTTTGCACTGTGCCCCGCCCTCCCTGTCCCCTTCCCAGCTTTGAAGTCTGTTCCTCTGAGGAGCTCCCCGCCTTCTCTTG
CTTTCTCCTCCTGGCCCCATGCATCCCTGTGTATGCACGGGCACACGCACTCATGCACACACTCATGCACACACATGC
ACAGTCATGCACGGGCACACACACTCATCACACACTCATGCTCAGGCACACACTCATGCACACAAGGACATTCATGCATG
GGCACACACACTGATCACACACTCATGCACGGGCACACAGCCATGTGCACACAGACATATGCACACTAATGCATGGGCAC
ACACACTCGTCACACACTCCTCACACATTCATGCACATGCACACACTCATGCACGGGCACACACACCCATGCACACACAT
ATGCACACTCATGCATGGGCACACACACTCATCACACACATGCGCAGGCACACACATGCACACACGGACACTTATGCACA
AGCACACACACTGATCACACACTCATGCAAGGGCACACACCCATGCGCACACACATGCACACTCATGCATGGGCACGCAC
ACTCATCACACACTCCTCACACACTCATGCACAAGCACACACATGCACGGGCACACACTGATCACACGCACTGGCACACA
CTGATCACACGTATGCACGGGCACACACACCCATGCACACACACATATGCACACTCATGCATGGGCACACACTCATCACTCC
TCACACACATGCACACACTGATCACACTCATGCAGGGCACACACACCCATGCACACACACATGTACACTCATGCATGGGC
ACACACACTCATCACTTCATAACTCATGCACACACACACTCATGCACGGGCACCCACTGATCACACACTCATGCATGGGC
ACACACCCCCATGCACACACACATGCACACTCATGCATGGGCGCACACACTCATCACACACTCATGCACATACACACACT
CATGCACACGCACACACACCCACATGCCTGTGTAACTCGCAGGAGTTCATCCTCACCCTGCCGCCCGACCCCCTGGGCCA
AATCCTTGCTTCCAAAGGCTCAGCAGCTCAGTGTCGTGCCCCAGCAGCCCACAGGAGAGCAGCTGCGGACGGGAGGGGAG
CTAGGGCAGTGGAAGTTGAGCTGGGGGGGTCTTCCCCTCTGCCTCTCCCAGCGAGGGCCCATTTAAGGGGAAGGCCTCCA
GGTGGGCAAGACTAGGCTGGAACCTGGAGATTGCCTCCGGGGCTCCCTCGTTCCTCCCTCCTTGGCTCCCTTCCCACCCC
ACCACCCCCCAACCTGATGCCTGCCCTTCTCCTGATGTCTTTCAGGAAGCCCCTTCCAGGGCCCCGCTAAATGCTGGGGG
AGCCACAGAGGAGCCTGATGGGTCTGCGTCACAGGGCAGGGAAGAGAGTGCTGGAACGCAGGCTGCCCTGGCCCCGAGTC
CAGGCCCTACCATCTAGCAGCTGAGGGATGCGGGGCCAGTCCCTCCCCTCTCTGACCATCTCTAAACCAGGGCTGCTGGT
GCCTTTCCCTGGTTCATAAGCCCGTGGTTCCCAAGCCTGCAGTATGTCCATCAACGGGATGCTTGTTAAAGCCAAGCTCC
CAAGCCCACCCCATCAGACCCTCTTTGGGCCGGGACTGTGAATCTGCATTTCAGCTCACTCCCAGAGGAAGGTCTGACCC
ACCGGCAGGCTTGGGAGCCTCTGATGTGAGCGCCTGTGGCAATGGGGGGCGTGTTGTGTTGAGGGTGGGTTGTGGGCCAG
CAGGAACATGCGTTAATTTTGGCAAGGAATGGACGGGTAGGTGAGGCTTCATGGGGAGGACTTGTCTTAGCTCGTCTCAA
AGATGAGCGGAATGTCACGGGGCTAAGTGCTATCCAAGGCAAAGGGACACCAAGGGGCAAAGGTCTGTGGGTGGGATGCC
TGTGCTCGGGGGCGGGCAGTGGTCACTGTGTGTAAAGGGAAGAAGGAAGCAGTGGATGGCCAGGTTCGTGGGTCTTCCCG
GCAGGAGACAGCAGAGAGGCGCCTGCCCGGGAAGGGTTAATCCCAGAGCCGTCCAGGTCCTGTCCACTCAACTTCCACCT
CCATCCTCTGAGGAAATGGGTTTTCCAGGAGCTTGGCCTGGGCAGCGGTGACTCGTGGCTCCACACGGAAGTGACTCAGG
TCCCCGGGAGAAAGCGAGCCGTGGGTCCCCACTGCCCACCGTGCAGAGGGGCAGGGCTGAGCCAGGTCTGGAGCTGGAG
CCCTGGGGGTGGGGCGGGGGATGGGCTGGTGGTGGGGTGGGGTCTCAGCTCAGACAAACCCTGGCCACCTTCCACTCTG
CTGGATCCTTGGGGAACCCCATGATGTGGCCCCCATTGTGTCAGACTGGACTGGCCTGTTCTGTCCTGTGTGTGTTTACG
GAAGGTCTGAGGTCAAGGGCGGCTGCTCTGGAAGCTGGGGGTTGTGGGGGCTTCCTTGGGCCTCAGCCCCAGCACCCTCT
ATCCCAGGCTGGATTCTCTGCTACTCTCCATTCTCCTCTCCTTCCCTTTGTGGGGATTCTAAAGCCGGGGAGTTGGGAAG
GATGTGCATCTCTGGGGCCTTTATCAATGCCAGCTCTACAAGGAGGGGGAGACTTGGCACCACGCTCGTGTGATCTCAGA
ACACACAGCCTTCCCCAGCCTCAGTGTCCGCATCTGTAAAATGGTAGCAGTGAGACCCGTATCACAGGGTGGTTCTGAGG
GTTTGGTGTGATAACGTGGATGTCACCACTAACATTATCATTGCGGGGGTGTTGCTAGAATGCCCCCTGCTCAGCCACTC
```

```
CCTCTGGCTCTGTGTGAGCAGAGGCAGGGCCCTTGCCTGTGGGGCCGTGGTTGGTACTGAGCGCCTGATGGCTGAGGCCC
TGTAGCGCTGGGGATCTGCGGCTCCAGAGGCAGAGACAGGTCAGGTCCCAGGGGTCTGTGCGGAGACTCATGCTACATCC
CTGGCGCCTGGGTGGGTGGGTGGGCAGGTGGCAGGTGTGTGTGCGTGGGGCAGACCTGGAGGGGTGGCATAGGCAGGGAT
GAAGGAGGGGCGCTAAGTGGACAAGCGTGACAGTGTCCCCCTGCCCAATCTGTGTGGCAACTTTCTTGTCCCCAGCCCAG
CTCTCCTGTCCCAACGGGCTTCTGGAGGAGGGAAGTTTTCTGGGTGGGGTTTAACCTTCCATTGCACTCTCCCGGAAAGG
AAGCTTCATGCTCTAAGAATTTCCCCTCAAGGTGCTCTGGTGGGTGAGCTGGTTGCTGGGGGCCGTACGGGAAGAGGGGG
AAACAGGGAGGAGGGGCCTGTGACAGGCCCCAGGGGGCTGAGTTAGGGGCAGGCATGAGCTGTTTAGAAAGTACAGAGATA
CTTAGCTGAGACTGATGGCACCTGCCTCTAAGCCGTTTACAGGTATCAATTCATTTAATTCTCACAATGACCTTTGAGTG
AGGTTGACTTCTTTATTCTTCTTTACTAGTGAGGAAAGCGAGGCCCAGAGAAGGTGAGTAACTAGCCCAAGGACACACAG
CTAGTGAGGGAGTCAGGATTTGAACCCAGGCAGTCTGGCTCTAGAGTCTACCCCATGGGTTAAAGGAAGCCAGCCAGCCC
CTCCCCTCAAGCCTCAAGCATGTTAATGGAGCCAGGGCTGTCCACCTTGGAGTAGTTGTTCAAACAGCTGAGGTGGAGTG
GGGATGACTCAGGAAGTGGCAGGGACAGTTGGTCCCTTGTGTGTGTCTGGATTTCTTCCGTGGAACCCCATGTTGTCGCT
TCACTTGTTTCCTTCCAATGATGGGGAGCTCTCTACCTGTACTCCCCTCCTTTACTCTCAAGGGCCAATCCAGGAGGGCT
GAAGTGACGCGGTGGTTCTGCTTTGTCCTTCAGCCATGCCTACTGACAGTGAAGGCTCACTTTGAAGGTCAGTAAGCCAG
GGAGATGCATGGATTCTGGCCAGTTCCTTCCTTGCTTGCTATGTGACCTCAGGAGGTCACTGGCCCTCTTTGGGCCTTGG
ATCCAAATTCTCCAAATGATTTGTCAGCTTCGAGTTTACAGGTAGGGAGTGGTGGTAGCAGTGAGGGGCTGCTTGGAGGA
AGTGGCATTTGAACTGACAATCAGAGGGGGGACAGCAACAGAGACTGACAGGGAAACAGAAGGAAAGAGGGGTCCGGAAGG
GACATGTCCCTGTCCCCGGATGTTGGGTTTCCTTCCAGTGGTCAGAGGTGGCAGGAGGCAGTTTCAAAAGGAATCAAAAT
GCTTACAAATCACAACCTGGGAAATGGTGCCCTTCCCTAAAACACTTTCACATCCTTCTTCTCAAAGGGCTCTGTGATGC
CACCAGGGCAAGATGATGTGCCTATTTGACCTACGACTGAACTGACAAGGCCTGAGAAATTGGGGTTCCATTCCACATGG
TGGGACGCAGGGAACTGGCTCTCAGTCGAAGGGGCCAGGTTTATTTGAGGGAGGACTGGCCCAGGAAGGAGACATTGAGC
TCCTGCCTGGGACACCCCTTCTTTTAATGCTGCTCTCAATGATTTGGTTCAAAAGCCACCTCTTCCTGAAAGTCCTCCTT
GGTTTCTCTTAAACTCAGACTCCACGTGTGTTCTCCGGCTTGTGGCCGATGACTGCCTCTGTCACTGTGGCCAGCTGCCG
CGCCACGCAGGATGAGGTTCTGTCTGTGTCTGTGAGCTGAGCACCCAGCTCTGCCACAGGGCCGGCTGGCCATAGGGAGGGCCAA
GCCTTCTGGCAAGTCTACAAGTGCCAGGGCCACAGGTTCCCTGCCCAGGCACTATAGTCCTGACCAGGCAGGGAAAGCCCC
TGGCCTCGGGAGCATTTTTGGGCAAGTCATTTAACTCCATGGGCCTCAGGTTCTCCATCTGTGCAATGGGTCAACAGCAG
TGCCCACTTCAGAGGGTTGGAGTGGGAATCAATGCAGATGCAGGGGTGGGACGGGTCAACACAGAGGCTGGCAGATAGTA
GCCGCTCAGCTAATAGAGAGCTTAGGGGGTCAGATTTGCTCCATCCTTGCCAGGCGGGGCAATACCGAGTCCTTGTCCC
CAGCAGAAACCGTGGTCCCTTTGCTGTTCCAGATGCTCTGGACCCTGGGTACTGGCTCCCCCTGGCGGTCATGATAGGAA
GCGCCCTTCCCCTCTGGCTCCTGTAGAGGGCGTCGCGGATGCATTGATCCCTTTAGTTCCTTCACTGTCCATCCCCACAGC
CTTTATCCAGCAACCTCTGTACAGCCAGGGCCTGCCCACAGAGATGGATCAGGTGCTCCCTGGCTGACCAAGGAGGAGGG
AATGCACGTCTCCCACTACCTGGAGGAGGCTACTGGGGGTGGGGAGGGGCTTTGGCCAGTGGTGTGAACCAAAGGAGGCT
GGGACTGACTCTGGAGGGAGTTGAGGAGGGCTTCCTGGAGGAGGTGTCCTCTGAGCTAGGTCTCAGGAATGATGATAGGG
AGGTAGCTGGGGAGAACTCTGGGAGCCCCCTTTTTTCTGTGTTCCCTCCGGGCCTGGAGGGGCAAGGTGGTGACGGTTCC
AGGTAGAACCCTCATGCCAGATTCTAGATAAGATAATCCCGTCACTCTGGGTTCAGGAGGGAGGTACTACGATTAGCCCG
ATTTTGAGTGACTTACACAAGGTCCTGCAGCGGGCAAAAGGTGGAACTGAGACTGGAACTCAGCTGGACTCTGGACAGCC
ACTCCCCGCCTTTTTTTTTTTAAAGCAGGGTCTCACTCTGTCACCCAGGCTGGAGTGCAGTGGCGCAGTTATAGCTCAC
TGCAGCCTCGACCTCCTGGGCTCAAGTGATCCTTCCACCTCAGCCTCCCTAGTAGCTGGGACCACAGGCATACACCGCCA
TGCCTGGCTAATTTTGTTTATTTATTGTAGAGATAGGGTCTCACTGTGTTGCCCAGGCTGGTCTGAATTCCTGTGCTCAA
GCGATCCTCCTTTCTCAGCCTCCCAAAGCGCTGGGATTACAGACATAAGTCACCACGCCTGGCCTCACTGCCTCTTGGGG
AGCGGCCTCTGGCCGCTCTGGGGAGGTCCATGGGCTTTTTCCACACTTGCTTCCCCACCTCACCTCCCACATCCATGCCA
CACTGGCCTCCTCTTCTGCAGGGCCAGCCCAACCACCACACAGTCCACACTCCCAGCTAACACTCCTGGTCTCTTCCAGG
TGGTGACTTCCAAGAGTGACTCCGTCGGAGGAAAATGACTCCCCAGTCGCTGCTGCAGACGACACTGTTCCTGCTGAGTC
TGCTCTTCCTGGTCCAAGGCAGGTCTTCCCAGGGGTGCCCTGGGCTGTTGGAACTTACGTTAAAATGCCCCTGTGCCTAG
GGTGGCTGCCAGCACACATGCTAACTCCTTTAGGCAGTTACAGCTCGGCTAGTGGAGGGTACCTTGGAGAAAAGCAAAAG
ACACTCCTTCCTTCTGGCAGGTGCAGCCCTGCAGTGCACAGCTTAGGGTATGGAATATGGATGGGGTACTGGCCCCACTC
ACCTCTGCAGCCAGAGCATTCTTGTGCAGTGTACCACCTGCACAACCGTCTATGTCAGCCATGGCCTCATTAAATACTTC
ATTGCTGTTAAAAATATGTTTTCCCCTTGAAATTACTATTCGTTTTCAGTTTCCTCTTTTTTTTTTTTTTTTGAGACGG
AGTCTTGCTCTGTCGCCCAGGCTGGAGTGCAGTGGCGGGATCTCGGCTCACTGCAAGCTCCGCCTCCCGGGTTCACGCCA
TTCTCCTGCCTCAGCCTCCCAAGTAGCTGGGACTACAGGCGCCCGCCACTATGCCCGGCTAATTTTTTGTATTTTTAGTA
GAGACGGGGTTTCACCGTTTTAGCCGGGATGGTCTCGATCTCCTGACCTCGTGATCCGCCCGCCTCAGCCTCCCAAAGTG
CTGGGATTACAGGCGTGAGCCACCGCGCCCGGCCTCAGTTTCCTCTTTTTATAGTTTGATGCTGATAAGTTTTGAGTGGG
AAAGACTAACACATTTTTGTAGACCCTTGGAATGTGTGTGAATCTCAGGCTCCAGGTTCACATGTACTCAGCCTAGGTCC
TGTCCCCTTCCATGCCTCTGGTCAGCCCCTGCCACGGGGTCCCATCTGCAGCCTCTGCCTCCTCAGGTGCCCACGGCAGG
GGCCACAGGGAAGACTTTCGCTTCTGCAGCCAGCGGAACCAGACACACAGGAGCAGCCTCCACTACAAACCCACACCAGA
CCTGCGCATCTCCATCGAGAACTCCGAAGAGGCCCTCACAGTCCATGCCCCTTTCCCTGCAGCCCACCCTGCTTCCCGAT
CCTTCCCTGACCCCAGGGGCCTCTACCACTTCTGCCTCTACTGGAACCGACATGCTGGGAGATTACATCTTCTCTATGGC
AAGCGTGACTTCTTGCTGAGTGACAAAGCCTCTAGCCTCCTCTGCTTCCAGCACCAGGAGGAGAGCCTGGCTCAGGGCCC
CCCGCTGTTAGCCACTTCTGTCACCTCCTGGTGGAGCCCTCAGAACATCAGCCTGCCCAGTGCCGCCAGCTTCACCTTCT
CCTTCCACAGTAAGGCAACTTCCAGGCGGAGGGAACAACTGGGCAGTGGTCTAGAGGCAGGGAAGGCAAAATGCAAAGTG
GACTGGGCTCACAATATCAGATCATGAAGACTGGGCTTTGCTCACAGGCACTGGGGAGCCAGTGAAGGTGTCTGAGGAGG
```

```
GGAGGAGTGTAATCCAAGTGCTAGGATTACAGGCATGAACCACTGCGCTCGGCCCCACTGTCCCTTGATGGTTACTTTGT
GGGCAGGCAGGGGTGAGGATGGAAGGGCAAGGGCCCGTCCTGAGGCTGCAGAGAAGGTCTTGGTGAAGGAGGATGAGGTC
TGAATAGGGTGAGAGAGCAGAGACTGGAAAGGGACTGAAGATCAAGAGCCCCAGGAAGAATTCTTCCTCAGCATCAGTGT
AGACTGTTTATTTCCTTTCACAGAGGAGGAAACTGGGGCACAGAGAGGATGCCCAAAGCTGCACAGCTAGCCAATGGGAG
AGCCAGGATTTGAACCCAGCAGCTGATTGGAGTGGGGTAGGGGTGAGGGGCGGAGTATGAGAGGTCAAAGGTCAGGAAAG
CAGTGGGGTTGACTCTGAGGTCCAGAAGCTGCATCTTCCCCCTTGAGTGTGACAGGTACATGTGGCCACTGCCATTCAGC
ACCATTAAATGCTGAATGGTGGCCAGGAAGGGAGGAGCATCAGAAGGAGCTTGAAGGTTTACTGGGGGGTTTTCATGTCT
TTCCAGCACCTCAAGTTTGAGGCAGGGCTTTCAGGGGTGGGGGGCACAGCACTTTGATTGTGTCAGTGTAACAGGCTAAT
GATTATGTCCAGTGCAACAGGCAGGCCCAGGCCCGTCAGTGGTGGGGGGTTCCCCACACCCCAGGCTGGGATTCTGCCCC
GCAACCTGGGAGGATTTAGGGCAAAGTTGATCCTAGTTGCAAATTGGCATTCTGAAGACCAGACTGAACTTGGTCTTGTT
TGGCCTGTAGAGGGTTTTTAAAAAACATGGACCAGGGATTTCAAATCACCTAAAACCCAGGCTTTCTCGGTTATGCATTC
AAAAGATGGAGCTACCCAGGGCCTGCCTTCCTCAGTGGCACAGAAAGTATACCCTCCAACCCCCACTGTTTGTCTTCCTC
ATCTGAGTCCCAGCTAATCCCTGGAGACACCTTAGTCGGCCGCCTTAGTAGGCCGCCCACGCTACCTGGGCAGAGGGGCC
CGAGCCTGGAGTCAGGGAGGCTGGTGGGTGTGGAGTCCCAGGAACCACCACCAAGCCCCGCACATCCCTCCTAAGGAGGC
CTGGCTGGGCCCTCTCTGCTCCGTGTGTGTAGCCGAAGGTGTCTGCGGAGGGTGCTGAGCAAGGCACATCCCACCCCATC
CCGCTGGGGCTGGCATTGCTGGCGGGTTCTGTAAGACACAACCCCCACGGGTTGGCCTCATCTGAGTGGCCTTGGCAGAG
TCCACTCTGCTTCTTGAAGCCTCAGTTTCCCCTTTGTAAAGTAAAGATCGAGGCATTCAGAGTCATGTCAGGGTGGTAGG
GGGCAGAATGGGAGGGTCCTGGGACCTGAATCGGCAGCCTCGGCGGGGGCCTGTCCACCCCTCCCCCAGGTCCTCCCCAC
ACGGCCGCTCACAATGCCTCGGTGGACATGTGCGAGCTCAAAAGGGACCTCCAGCTGCTCAGCCAGTTCCTGAAGCATCC
CCAGAAGGCCTCAAGGAGGCCCTCGGCTGCCCCCGCCAGCCAGTAAGTTTGGCACCTGGGGCTGTGAGGGGAGGCAGGAA
GGCATCAGAGATCTGGACCTGGGCAGGGTGGGACCTGGAGTAGGGGCTACTGCGAGGCCTTCCCTGGGACTGGAATGCTT
CTTTGATTTCTCTTTCTGCCTTCTGGGAGTCAACACATGCCCCCCAGGCTGAGCCCTCTCCTGTCTGAGTCAGTAACCAA
CATCACATCCACCATCCCAAGCCCTCCTCCCCCTCATAAAGAAAGGATGGGTGGTCCACAGTACAGCCCAGCAGACCAAG
GCTCAGAGACGGCAGAGCCGCTCCAAGGCCACAGAGTAGGTTGGCACAGGCGGAGCTGGGCCCAAACCCATGGTGCTGAG
TCCCAGGACAGCCCCCACCACACCATTGCTCTCCTTTCCCTTGGCTCCCAGGTCCTGGCCTCCTCTGTCTGACCCTACCC
CAGCTCATTCTTTCTCTCCCACACGCAGATGCCTGCTTTTCTCTTTTGTCTGCTCTCCACTCTCTCTTGCCCACCCCACC
CCTCTCTCTGCCTCTGCCTCTGCCTCTGCCTCTTCCCTGTGTCTCTGTCTGAGTCTCTCGTCCTCCTGCCTCAGTCTCCC
TGGTGGCCCGGCCCCCTCCCCACCATCACCACCGCTTTCTCCTCCCTGCCAGGCAGTTGCAGAGCCTGGAGTCGAAACTG
ACCTCTGTGAGATTCATGGGGGACATGGTGTCCTTCGAGGAGGACCGGATCAACGCCACGGTGTGGAAGCTCCAGCCCAC
AGCCGGCCTCCAGGACCTGCACATCCACTCCCGGCAGGAGGTCAGGGGCAGGCCTGGGCAGGAAGCAGATGCGGGTTGGG
CCGGGGCCAGATGGAGGTGGGGGCTGTGAGCACTCCCTGAAGCTCAGTGCCGTCGCAGCCTCTCCCTGGGGCCTCCCGAG
AAGGTTCCAGGCCCGAGGATAGCCATCCTAAGTCAGTAGTTCAACCGTGGGATTGAGGACCCTGTGTGGGCACAGTGGCA
GGGTCTGCTTCCTATACCAGTCTCCTGGGGCACCCGTGTGAACAGAAGACCAGCCCCTAAACGCCTGTCCACGCACCAC
CCCCCCCGTTTTTTTTTTTTTTCGAGACAGGGTCTTGCTTTGTCACCCAGACTGGAGTACAGTGGGGTAATCATGTCTCAC
TGCAGCCTCAAACTCCTGGTCTCAAGCGATTCTCCCACTTCAGCCTGTGAGCAGCTGGGACTGCAGGCTTGCTACCACAC
CTGGCTTTAAAAAAATTCTTTGCAAGGCTAGGTGCAGTGGCTCACATCTGTAATCCCAGCATTTTGGGAGGCCGAGGCAGG
CAGATCACTTGAGGTCAGGAGTTCGAGACCAGCCTGGCCAACATGGTGAAACCCTGTCTCTACTAAAAATATAAAAATTA
GCCGGGCATGGTAGTACATGCCTGTAATTCCAGCTACTCGGGAGGTTGAGGCAGGAGAATCGCTTGAACCCGGGAGGCGG
AGGTTGCAGTGAGCTGAGATTGTGCCACTACACTCCAGCCTGGGCAACACAGCGAGACTCTGTCTCAAAAAACATTTTTT
TATTTTTGTAAAGACAAGGTCTTGCTACGTTGTCCAGGTTGGTCTTGACCTCCGGGGTTCAAGCAGTCCTCTCGCCCTGG
CCTCTCAAAGTGCTGGGATTACAGGCGTGAGCCACTGCACCCAGCCACACACCCCATCTTGAGACTGCCCCCTGAGTGGC
TCTGGTTAGCTGGGAACCACCCACACTGCCCACATGGCCACCCCCAAGTTGGCTGCTGTAGGCAGAGCTGGCATCTGAAG
CCCCAGACAACAGAACATCATGTGGTTCTCCCAGGGCTATTTGCCCTGGAGTCCTGGATTCTAACCACAAGACTCCCCAG
CCAGAGCTGGTAGCAGGGAAGGGAGGGATGAGGAGGGCTGTCATGAGTCAGGCTTGACTTCCTGAAGAAATGGGCTTAGA
AGTGGCAGCGTCCAGGAACGGATGGGTGTGTGTGTGTGTGTGCTAGGGTGGGGGGCACGGATCTAGGGGTCCGCATTTGG
CTGAGCCCTAAAGGGACCTCTGCAGGAGGAGCAGAGCGAGATCATGGAGTACTCGGTGCTGCTGCCTCGAACACTCTTCC
AGAGGACGAAAGGCCGGAGCGGGGAGGCTGAGAAGAGACTCCTCCTGGTGGACTTCAGCAGCCAAGCCCTGTTCCAGGTA
TGGGGTCCTCACCCTCATGCCTCCCAGGAGAAAGCAGTTTTTTTCTGACAGAGGTGGAAAGAAGGCACGCAGATGAGCTC
CTTCCTCTGGGAGTCAAAGCCTTTCCTTGTAAAGTTACAAATTGCACTGCAATGTGCAAATCTCCCTGTGAGAGGGCTAA
GCAATGTTCTTCTTACTATAGTGTAAATGACTACATGGGCAAAAGTGGTTCCAGAGGGAGACGCAGCATCTCAAGGCAAT
GTGCTGGGAGAGGGTTATCTAGAGAGGGTAAGGGGCTTCTGGGCCCTTCTTCTCAGTCCTGAACTCCCTCCCTACTCTCT
TCCTCCAACCCCATGTATCTAGGACAAGAATTCCAGCCAAGTCCTGGGTGAGAAGGTCTTGGGGATTGTGGTACAGAACA
CCAAAGTAGCCAACCTCACGGAGCCCGTGGTGCTCACTTTCCAGCACCAGCTACAGCCGGTGAGTGGGGGCCAGCCATCA
AGAGAACAAGCGCCCCTCGGCCATGTCACCCTTTCCTCTCCCTCCCTCCAGACTCATTTGTCTTTATAATGAAACGATTG
CCTGATCGAGCAGTCAGGTCCAAATGGGGCGGGTGGTGGCTTGACTGTGTTCTAGACTTCCTCTGCCTGGCCTGTAAAGT
TGGAGGGGTGGGGGGCTGTCACAGAAACCACTCTCCTTTCTTGTCCCTACAGAAGAATGTGACTCTGCAATGTGTGTTCT
GGGTTGAAGACCCCACATGTGAGTATGCAGGGGTCTCTGGGCTGGACAAGTATCTGGAAGAGAAATAGAGTCCTGGGGGG
TGGGGGAGGTGGGGTTAATCACCGAGGGCTTCCTGGAGAAAGGAGGACTTTGAAGAGAGAGCGATGGCAGGCTATGAGTA
GGCCGGGTGGAAGAATGACACAGTCGTGCTTTTGGGGGTGGACACAGTGGGGTCCTGGAGGACTGGACTTGATTGGAGCC
CCGTGCTGTCCCCTCCTCAGTGAGCAGCCCGGGGCATTGGAGCAGTGCTGGGTGTGAGACCGTCAGGAGAGAAACCCAAA
CATCCTGCTTCTGCAACCACTTGACCTACTTTGCAGTGCTGATGGTGAGGGTCCCTGCTCCCACCTCGCAGCCACACCCC
```

```
AGGCCGTGTGCTTGTTCTGTGCAAGCACTTTTCATATATTCAGTCATTTATTCCTCATAACAGCTCTCCAAGGTAGCTTC
TATTGTTGTCCCATTTTATAGATGAGGAAATGGAGGCTCAGAGAGGTTAAGCAATTTACCCGTGGCCACACAGCTAGTCA
GTGAAAGAGCTGGTTTGTAGAGCCCCTGCTCTTTATTCCCAAATTTTGGTGTGTACGAAAATGCAGATTCCTGGCCCCTCA
CCTAGGGTTCTGATTTGGCAGTGATGGGGCCAAGAGTGTTTCTAATGAGGCCCCTGAGGTGGCCTCCAGTGCCCCCAAGA
CCTCAGAATTCATGCTCCCAGGACTGTAGGATTCTGCCTGTGGGTATCCACCTGGGGCTTGAACACCCCTGGGTGTGGGA
ATCTCATTCCCTCACAGGAAACCTATTCCCATGTTAAATGTTATAGAAGAATAATCAATGCCAGGACAGAATAGGGGTTC
TCTCATGAAGGGTTCTTGGCCTCTGTGGGTCAGGAAGCCGCTTGAGAATGTGGTCAAATCTAAAGATGCCCCCACTCCAG
AAAAATGCACTTATGGGACCACATTCTGCTCAGTTGGGAGAGGGGGTTCTTAGGCTTCCGAGGCCCACCAGGGACCCCAG
GTTAGCCCCTCACGCAGGGCAAGCCTCCAGGTTGTGTGGGGGACACAGAGGCCAGCTCTCTCCTGTCTCCTGGGGCCAGG
TCTCCTCGGTGGAGGTGGACGCCGTGCACAAGCACTACCTGAGCCTCCTCTCCTACGTGGGCTGTGTCGTCTCTGCCCTG
GCCTGCCTTGTCACCATTGCCGCCTACCTCTGCTCCAGGTGAGGCCTGAAAGGGGTGGGACAGGGGAGGGGACCCTCCAT
TGCACACACCTCCACCAGGGCGCCGCACACATCTCCGGTCATGGCCCGCCCGCATGACCTGGAACTGTGTGTGTGGTTAG
GGGAGCTGTTTGGGGTAAGCTGACCCTTGGGGGCCACGTCTCCTCCTCAGATCAGTGGTGTTTGGATAACATAGTATTTA
CAAATTTTAGCTGTCGACATTGTATTTATTTATTTTTATTTTTATTTTATTATTATTATTTTTTTGAGACAGAGCCTTGC
CTCTGTCGCCCAGGCTGGAGTGTAGTAGCATGATCTCGGCTCACTGCAACCTCTGCCTCCCGGGTTCAAGTGATTCTTGT
GCCTCAGCCTCCTGAGTAGTTGGGACTACTGGCGCCTGCCACCACACCTGGCTAATCTGTATATTTTTAGTAGAGACGGG
GTTTCACCATGTTGCTCAGGCTGGTCTCGAACTCCTGACCTCAAGTGATCCACCCACCTCAGCCCCCCGAAGCGCTGGGA
TTACAGGCATGAGTCACTGTGCCAGGCTAATTGTCAACATTTAAAATTGAGAGAGTCCACCTAAGAATCTGTTTCTTTCC
TAGAGTAAGACAGGAAAGCAAAATAAAAAACTTAAAAAAGGAAAGATTTTTCATTTCTGCCTTTGAATAAGCAGATTTGT
GAGAGCACTGGGTGGACACAATAATATCACAGCGGTAATGATCATACTTAACGTGTGTCAAACTTCCCGTGTGCCATGCA
CTGTTCTAAGTGTTTTACATGTGTGAACTCAATTCATCCTCACAACAGCCCTATGAAGGAACACTCAGAGAGGGTAAGTG
ACTTGCTTGAGGTCACACAGCAAGGAAGTGGCAGAGGTCAGATTGTCAGTGCTTGGCTTGGCCTAAATTGGGGTCCACTT
GCCCTCAGCGCTGAACCCATTTGGGTAGAGGAGAGGCACCCTCGGTTCTCACTGTCCCCAGCCACGAGGAAGGAGAGGAA
AGTGAAGCTGTATTTATGCGTGTGCACAAGCACGTGTGTGCCTATATGACTGTCCTGTGTGTGCACAGCTGTGTACCTGG
GGAGCTAAGTAGGTGATGGTGATGAGGAAGGACAGCTGGGTGATGAGATGGGCTCGGCGGAAGGCTGGGGGTGGAGGCAG
AAGTCCTCAACACCCAGTTGCTCACAAACATGCCCAAGGACACAGCACAGGAACCCTTAGGAACTGAGCACCTTATCTGT
GTCAGATGCTTTGTGTCAGTGTAACTCTCACCCCAACCCTATGAGGGTACCATCCTCCCACTTCACAGATAGGCAAACTG
AGAGTCTCAAGGGGAGGGGACCAGCTGAAGATGGTGCCATGAGTTAGGGGCAGGGCAGGGTGGGACTCCAGGATGGTGAC
ACTGGGGCCTGCATGTTCTGCTGCACCCTCTAGGGCCATGTAGAATCCTGGAGTGGGGGTGGGGTGGGGTGTGCACACAT
TGACTTGCAAACCTCAGACAGACCGATGCGGGCAGCCCACACTCAGACTCACAGGTGCACAGTTGTGCAGACAGACGGGA
AGACACAGAATGTGGATGGCATGGATTCTCCAACATGGTGAAAATGCCAAAGTGTTTCCGCTCTGACTTTGAGAGTATCT
GGTTTATTACTGTGGTTGTCTTCCTCGCTCTGCCTGGCTGAGGCTTCTGTTAAACGTGCACGTGGTGCCTGAGTCTGTGG
GTGCTGGGCACACAGTAGGTGCACAGGGGGGATGTGGGGAACAGTTTGGCTGCAGCCACAGGAATAGGATAGGGGCCATGT
ATGACTGCATGTGTGTGTCGGGGTGGGGGGCAGTCTGGGAAGGCTTCCTGGAGGAGATGTGGGCACACTCCCCTCTCTAC
CTTCCCACACTGGCCCACCAGGGTGCCCCTGCCGTGCAGGAGGAAACCTCGGGACTACACACCATCAAGGTGCACATGAACC
TGCTGCTGGCCGTCTTCCTGCTGGACACGAGCTTCCTGCTCAGCGAGCCGGTGGCCCTGACAGGCTCTGAGGCTGGCTGC
CGAGCCAGTGCCATCTTCCTGCACTTCTCCCTGCTCACCTGCCTTTCCTGGATGGGCCTCGAGGGGTACAACCTCTACCG
ACTCGTGGTGGAGGTCTTTGGCACCTATGTCCCTGGCTACCTACTCAAGCTGAGCGCCATGGGCTGGGGTAAGTGGTTGG
GCGGGGGGTGCCTCAGACCTGCCTCTCCCACTTGGCTCTGGCAAAACCCAGGCACCTGGTTCTGCCTCTCCACCTATCTC
TCTGACTTCCCTCCTGGCCTCCTTCACTCATCCTCAGGTGTCCTTCACCTTGGCTGAATCAAGGTCCCCTTTAGGAAGAA
GCAGTGAGCCCTGTCCCCAAGAAACACTTCCAAATTCTCCACATCACTCACTCCATTGCAAAGGGGATTTACGAGCAGGG
CCACCGTGTGTGAATGTGCAGGTTGTGCACTGCTCAAAGGCACCCAGTTCAGAGATCAGAGGGGCTGAAATCCAGTCCCG
CTCCAGTCATTTAATTCACTCGGGCTGGCAGTGGCCCTGCCTCAGGATCCCCCTGGACCTTGGGTAACAGATTCCCTGTG
GCTTGGCATTTTGTTCTTGGCCAATGACTGTTCAATTCACCTGTGGCCAGCCCTCTTATGTCAGACCTACAGCCCAGGGG
CTCATGATGCCACAAGTCTGTTTGGACATTGCACAGACCCCTCAACCTCCGCATGCCCCTGACTCCAGCTTGCTGCTCTT
CGCGGGTTTGTCATTGGGCCCCTTCTTTGTCCATTCCCCCATTTGCATGAAATTCCTTTGGAGGTGTCTCTGTGTCAGGA
CTTTGTGTTTGGAGGTGGGCAGCCCCCTCTAGGGAGCTTCTAATCTCCACCGAACGGGCGAGGTCAAGGCCCAGTGCAGA
CGTCCCCTGATGGCAGAACTCTTGCCGTCCCTCCTAAACTGTCTGCCCCTCCCATCTCCCCTGTGACATCCAGCCCTGCA
GCAGAGACCCACAGGCCTTTCTCCCAGGGGCCCCCAGGGTGACGAAGGGCAGGGCTAAGGTTGGAGGAGATGGCATCCCC
TTATAGGAAACCCATACTGGAATGGGGGAGGGGGGAGGGTCCAAACTTGGGGGAACTTCCCCACCAGATGGGGCTGGGTGGG
CTTCAGGAGCCCAAACTTCAGAGGGGCCCTTGCCCTCCAGACTCCCTGAGGCCAGCAGGGAATGGGCAGGGCCTCAGAGAG
CGGGAAGTAGAGCAACATGCATTGCCACCCTCAGGCTTCCCCATCTTTCTGGTGACGCTGGTGGCCCTGGTGGATGTGGA
CAACTATGGCCCCATCATCTTGGCTGTGCATAGGACTCCAGAGGGCGTCATCTACCCTTCCATGTGAGTGGCTGTGTGCA
ATGGGGGCAAGAAGGTGGGTCTCTGGGCACAGAGGCCAGAGTGCAGCCCGGGCCCAGGTCATGCTGGCCAGGGGACACCT
GGGTTCCAGTCTCCTCGAGGAATTGGCCTGGCAGTGGCTGAAGCACTGGTCTAAGAGTCATGGAGGGTGAGAAAAGGCCT
GGATCTACCACTCACTCACCAGGTGACCTTGCACAGGCCCCGGCCCTTCTCTGAGCCTCGGTTTCCTCATGCATGAATGG
GGGTATGGGTTGGAGCAGATGAGCCTCACCACATTCCTCAGCAGCACCTCTCTGTCTCCCCATGCAGATGGGAAGACAGA
GGCTCAGGGAGGGAAGAGATCCGCTCTTGGCCACACTGAGGGCTGGTGTGCTGTTCGGAGGCAGAGGATGAGTGGGGTTG
AAGTCCAGTCCTTCTCCACTCACCTGATTCACCCAGGCTGGCAGCGGCCCTGCCTGTGGATCCCCTGAACCCAAACCGGA
AGCCAGGGTTCCTGAGCTCCAGCCTGGGAAGGAATCCTTCCCATTGGCTCAGTGGTTTTGAAATTTTCAGCTCTTTATGT
TGTGACCCAGTGATTTCTGCCTCACTTTTGTACTATCTATATTATTACTTACTTAACATTTTCCTTTAAACAATCTGACT
```

```
TGTTAACTCAAATGTATTTTTAAAATTACATCAACACTGTAAATAGAAAACAAGCGCACTTGCTGTAAACAGTGGATAAT
CCTGAAAACAAACATGACAACCACAGCCCAGGAAATGCTGCCCGTGCTGGCCACACGCTGAGCCCTCCTGCCTTTGCCCG
CAGGTGCTGGATCCGGGACTCCCTGGTCAGCTACATCACCAACCTGGGCCTCTTCAGCCTGGTGTTTCTGTTCAACATGG
CCATGCTAGCCACCATGGTGGTGCAGATCCTGCGGCTGCGCCCCCACACCCAAAGTGGTCACATGTGCTGACACTGCTG
GGCCTCAGCCTGGTCCTTGGCCTGCCCTGGGCCTTGATCTTCTTCTCCTTTGCTTCTGGCACCTTCCAGCTTGTCGTCCT
CTACCTTTTCAGCATCATCACCTCCTTCCAAGGTAAGGAGAAGACCCGTCCCTTGGCCCAGGCAGGGTGTCTACACATGG
AGCAAGGGCAGGGAAGAGATCACCCAGGGAACCTGAAGACTTCCCTTCTCTGGGCCTCAGTCATCCCATTCATAAAATGG
GGACATCCAGGCCACAGTCAACAAGTCTTGATTGAGCACCTACTATGTGCAGGCCCTGTGTTAAGCCACAGGGCTTATGG
CTGAGCTGGTTGGGTGTGGGCCTTGTTCTTTTGGAACTCAGCTCTACTGGGGAAGACTGCGCATGAGCAGGCAAGCATAT
AGCCATCAAAGGTGGTCCCAGATAGTAGGGACATCGTCGTGAGCACGTGCTGCACAGGGCCTCGGCTGGGGTGGGGGACA
CCCTAAGAAGGGTGGCCAGGGAGGCCTCTCTGGATATGAGACGTGTGAAGACAAGAGAAGGATCCCCCCATGGAGAGATG
GGGGGGCCTCCCTACTCCAGGCGCGGTCCACAGACCAGCAGCAGCAGCGTTGTTAGGAGCTTTTTGGAAACGCAGCCTCT
GGGCCACCCGGGCCCTGCCACATCAGAATCTGCATTTTCACAAATGCCCAAGGGGTCACGTGCACATGATAGGATCAGGA
GCCCTGGTCTAGGGGAAGGAAGCTCCAGGCAGAGGGAGAGGCAAGAACAAAGATCCTGAGGTTGGAACCGGTTGTGTGTA
GGAAGGACAGTGAGGGAGAGCGTGGCCAGATGGCGGCGGGTCGGGGCGGAGGGGTGGGATCCCAGGGCAGGGTCATGATG
GCTCTGGCTTGATGCCAACAGGGTCCCTTTCTGTGCATGTACCTGTGTGTGTACATTGAGGTGCACACACAGGCATGCAC
ACCCCTCTGTTCCTGGCCGTGCAGAATGGAAGGGGGTTGTATGGCCAGGCCCAGGGGCTCTCATGGGGTTCAAGGCCAGC
CTCCCATACAGGGTCACCCTAGAATGGGAGCTGGGAGTTGGTGGTGGGGAGATGTTACCACATTTCGGTTATTTAACATT
CAAGCCTTCATTCCTGCCTGTGAGATGAGGGGACTCAGGATTGTAAGTGAGGCCCATACATTCACACAGACATTTACTAA
GCTGTGGATACATCATAGTGCTATTAATTTGAGGGGTGCAAAATCTCACAGTGCTTAAGTGGGTGGACTTCAGAGCCTGT
TCACCACAGTTTGAGCCCTGGCTCAGCCACGTCCCTTTACCTGAGCCTCCTGGGCCTCAGTTTCCTTGTTTACAAAAGAG
AATAGTAATGAATAGGATGGGCCTAGGAAGTTTGTGACAGTGAGAGGCCACACGGGGACGCAGCACAGTGCCCGGCTCAT
AGGAGGTGCTCGCTGGGTCTCATGGGTGCCTGACAGCACGTGCTTGGCAAACACTATGGAGAGGTGGGGCAGACCCGAGT
CACAATGGCTGGGGAGGGAGGAGGAGGGATGGGGTGGGGCTCCCCCACCTGCTGACCCCGTGCCCTCACTGCCCGCAGGC
TTCCTCATCTTCATCTGGTACTGGTCCATGCGGCTGCAGGCCCGGGGTGGCCCCTCCCCTCTGAAGAGCAACTCAGACAG
CGCCAGGCTCCCCATCAGCTCGGGCAGCACCTCGTCCAGCCGCATCTAGGCCTCCAGCCCACCTGCCCATGTGATGAAGC
AGAGATGCGGCCTCGTCGCACACTGCCTGTGGCCCCCGAGCCAGGCCCAGCCCAGGCCAGTCAGCCGCAGACTTTGGAA
AGCCCAACGACCATGGAGAGATGGGCCGTTGCCATGGTGGACGGACTCCCGGGCTGGGCTTTTGAATTGGCCTTGGGGAC
TACTCGGCTCTCACTCAGCTCCCACGGGACTCAGAAGTGCGCCGCCATGCTGCCTAGGGTACTGTCCCCACATCTGTCCC
AACCCAGCTGGAGGCCTGGTCTCTCCTTACAACCCCTGGGCCCAGCCCTCATTGCTGGGGGGCCAGGCCTTGGATCTTGAG
GGTCTGGCACATCCTTAATCCTGTGCCCCTGCCTGGGACAGAAATGTGGCTCCAGTTGCTCTGTCTCTCGTGGTCACCCT
GAGGGCACTCTGCATCCTCTGTCATTTTAACCTCAGGTGGCACCCAGGGCGAATGGGGCCCAGGGCAGACCTTCAGGGCC
AGAGCCCTGGCGGAGGAGAGGCCCTTTGCCAGGAGCACAGCAGCAGCTCGCCTACCTCTGAGCCCAGGCCCCCTCCCTCC
CTCAGCCCCCCAGTCCTCCGTCCATCTTCCCTGGGGTTCTCCTCCTCTCCCAGGGCCTCCTTGCTCCTTCGTTCACAGCT
GGGGGTCCCCGATTCCAATGCTGTTTTTTGGGGAGTGGTTTCCAGGAGCTGCCTGGTGTCTGCTGTAAATGTTTGTCTAC
TGCACAAGCCTCGGCCTGCCCCTGAGCCAGGCTCGGTACCGATGCGTGGGCTGGGCTAGGTCCCTCTGTCCATCTGGGCC
TTTGTATGAGCTGCATTGCCCTTGCTCACCCTGACCAAGCACACGCCTCAGAGGGGCCCTCAGCCTCTCCTGAAGCCCTC
TTGTGGCAAGAACTGTGGACCATGCCAGTCCCGTCTGGTTTCCATCCCACCACTCCAAGGACTGAGACTGACCTCCTCTG
GTGACACTGGCCTAGAGCCTGACACTCTCCTAAGAGGTTCTCTCCAAGCCCCCAAATAGCTCCAGGCGCCCTCGGCCGCC
CATCATGGTTAATTCTGTCCAACAAACACACACGGGTAGATTGCTGGCCTGTTGTAGGTGGTAGGGACACAGATGACCGA
CCTGGTCACTCCTCCTGCCAACATTCAGTCTGGTATGTGAGGCGTGCGTGAAGCAAGAACTCCTGGAGCTACAGGGACAG
GGAGCCATCATTCCTGCCTGGGAATCCTGGAAGACTTCCTGCAGGAGTCAGCCGTTCAATCTTGACCTTGAAGATGGGAAG
GATGTTCTTTTTACGTACCAATTCTTTTGTCTTTTGATATTAAAAAGAAGTACATGTTCATTGTAGAGAATTTGGAAACT
GTAGAAGAGAATCAAGAAGAAAAATAAAAATCAGCTGTTGTAATCGCCTAGCAAACTGGCGTAAGCATTTTGGGTCATTT
CCTTCTGAGCTTTTTTCCTGTGCTTCTATTGAAAGAGTCACTGATACTGATACAAATACCTTGGGGCCAATCAGGAAACAT
GAAAGAGAGAAGAGGGCTGTGTGTGAAGAAAAAAAAAAAATCGACTGCTGGAAACCTCATCAACCAGGGCACAGGGGAAG
ATAAATGGCAACTTGGTGTCTGTGCTGGGGAGGCAATGGGGAGTGGTGGGGCCTGTGGCGAACTGGAGACTCCCAACCCC
ATGCTGAGAGCGTGGCTGCTCATCAGCTCCAGCTGGTCACTACCTTGCAGGGATGAGGGCCCATGTAGCCAGACCACCTG
CCTTTTATGAGAAATGGGAAATCCAGACTGTGGTTTGACATCCCCTGATTTTAAAATTTTGGCGAATCCAAAACATTTTC
TGGCCAAAGAGATTACAGTCAAAAGCTGCCTGGTCCTGGGATTTATGCAACCAGTTTACTGATCACTCTGCAGCAGGTGG
GCTGCAAGAATTAAATCACTGGACTTCTCTCCCATCCCTGCCTTTAACCTTGTTCCACCAAATCAATCTCTCCTCCCGCT
CCTAAGGCCCAGGTGAGGCAGATCACGCAGGTTCCCATCTTCCCTGTGATGAGGGCAGTCACAACGGATCATTGCTACTG
TGTTCAGGTAAGAGGTCCCAGTGCAGCCAAAGTCCTCCCCAAGGCCCACAGTCCACTCTCTCCCTTCTGCAGGCTCCTCC
CTCCTCCCCTGGCCACTCTAGGGGGATGCCCAGCAGGGGAAGAGGCTCAGAGTCTTGGATTGGACAAGGTGGGTGTGAGG
CAGCTGGGTCTAGACTGGCAGTGCCCTCTGAGGGGCGTGAATGTGGGATCTTGGGACCTGCAGGGACCAAGAACCCCTGT
GCCCTGCTGCTCTCCCAGCCCTCCCTCCACCCCCTCCACTCCCTCCACTGACCTCTGTCCCCACCTTTCTGCAGGTGGGG
GGCTCCTCTTATGCCCCCAGCTCCAAAGGACAGAGGCCAAACCTTCCCAAGCCTCCCCTACCCCAGTGTCCGGATTGGGC
AGCCCCGGGGGATCAGAGCCAGCTTCCCTCCTTACAGGCTTCTAGGTCTCCGTGAGGGGGGTCTCTCAGAGCCCCATGGC
TGGGCTAGGTCCTGGGATGGGGGTTGAGGCAACTCCCCCCATCTTCCAGAATGGGGGGGAAATGACCCAGCCCCGTTCCT
CCGCAATTCTCCTGCTTCTCAAACATTTTGTTTCTGCATCTGAGGCTGGCTCAGGTAGTGGGGAGGGGTCTTGGGTTGGG
TCTCACCTTCTCAGGTAGGCCCTGCACAGGGCACCAACAGTTCCATGCTGATCCTTTTTACCCCGATGGTTATTATCATGC
```

```
ACTTTCCCAGGCTGTGGGGCTTTGGACAGTGGGATCGCCCTTTCTGCCAGCATGTGGCCAGGCGTGCATCTGGAAACATC
CCTTTAGCTTGGGAAATGGATTGGAGCAGCTGAGGGCTGAATGCTGGCTGGGTCTCGCTGGAGAACTTGAGGCCCTGCCC
ACTGGAGCCATTGGGAGAAGCTGGGGCAGGAGGCGGCAGGAAGGGACACTACTGTTTTGTTGGCCCTGGGGTTGGGGGGG
GTCTCAGGGTTCTGTGAGTGGCTCCAGGCTAGTCCCTGGTCCCCAGGGGGCGTTTCAGGGAAGGGGCCTTAGGAAAAGGT
GGCCCTCCTTCCAGCCCTGCCTGACCTTCCCCTGCACCTGCCCTCTGCCCACTTCCCAGGTGGGCTGGCTGCTCGGAATC
TCAGTCTGCTCACCCCCAGGCGCTTCTTCACACACTGGTACCTGCGAGCCTCTCTGAGGGCCCAGGTGCCAGGATGGGGC
TTCAGCGATGGGCCCAGCACATCCAAGTCCACTGCCCCACCTCACCCTCACCTGCAGCTGGGAGGGAGGTCTGCAGATCT
TCCAGGAGAAGGCTGGCCAGGTGTGGAGCAGTGGCAGGCCCAGCCATTCATCTGCCTTCGCAGCCCTCCCTGCAGAGGAA
GTTTCTGGGGCTCAGGAAGCCCTGGGCTCTGACCTGCTGCTCCATGCTGAATTCCCACCTGCTCCCACACCATGAGTGAC
CCTGACATCCCCTCCCTACCCCTTCCCAGACCCAGAGGGCTCAGCAGGAGACACTGAGTGGGAGGAGGAGGAAATGGAGG
CCAGCAGGGGCTGAGCGGCTCCCCAGTTCAATCCCAAGGCTGAGTAGGGCTAGTGCCAGCCCCGTGGGCCAGAGGGGGAT
GCCAGCAACCACACACCTTGGTGGGATGCCCTTCACCTTCCAATTGCCAAGTGGCCATGGCCTCATGCACAGTGCATCCG
TGGGGCCTCCAGAGTTGGCCCGAGTGGCTGGCGCCCTAATTCCTTTAGGCTGAAGCCCAGGAGTGTTTGCCACCCCCACC
TCCCACCTGATCAGTGACCATTTGGGGTGTAAAGACAACAATTTCACAGCTCTGATGATCAGAAATGATGTAATGGCCAC
AGGCGGCTCCGCCTGCGTCATCCATGATTTCATCACACACCTCGGGAGGCTCAGGGTGACAGACAGTGCATGCAAGGTCA
CCCGGCCAGGAAGTGGCCAGGATGGATGTTAGCTCAGGCCTGACCAGCCCCCGGGGCCGCACTGCTGTCTTCCCGCTACC
CTGCCTCTGGGTCCGTCCTGTTATCCAGGCTGGTGGGGGAAGCTCAGGCTGCAGGGCCAGCTCCTGCTCTCCTCCCACAC
CCTCCAGCCTGGTTCAAGCCATAGGCACCATCTGGGTCTTCGTCCTCATGCCCAGAGCCAGGCTCTGTGCCCAGCTTGTT
GCAGGTCTCATCAAACCCTGACACAGCCTTAGGAGGCAGCCCCATTGCCATCTTCACTTTACAGAGGAGGAAACTGAGGC
TCAGAGAGGCTCCCAGCTTGTGAGAGGAGGGGTCTGAGTAGGTGCGCCTGACTCCAAAGCCTGTGCTCACCACAGTTCCC
AGGGATCCGCTCTGAGCCAGGCCCCTGTGGCCACCCAGCCCCTAGCCCCGTAGATCCCACCCCAGTGGGGCCCCGGGGGC
ACATACCTGGGTGGGCTCAGCAGCTCCCTCTGCAGGTGGACCCAGGGTGCGGTGAGACTGGGAGCACTGCCCAGTTCCCC
ACACTCACCTTTCACAACCCAACCAATGGCGCCATCGAGCAGGAAGGGTGAGAAAGAGGACACAGGAAGCCAGAGTGGTG
GGGCTGCAGGGTGGGGGCAGGCCAGCTCAGCAGAGCCTGGGGCCAGAGGGCCAGACAGCCACAGAGCTCCTGGCGTGGGC
AAGGCTGGCCAAGGATGGCGACGCCCAGGGGCCTGGGGGCCCTGCTCCTGCTCCTCCTGCTCCCGACCTCAGGTGAGTGG
CTGGCACCTCATCCCCTCCTGCCACGCTGGGCCGACCCTGCCCTGCCGAGGGAGGGATCCAGGGACAGACGCAGGGACTG
GAGAAGGTGAGGAGTTGGGGTGTCTGGGATGTGTCCTCCGATACCCCCCGTGGCCGCCTCAGCACCTCCTCTAGCCCTGT
CCCTTTGGGTCAGTCCTGTCCTTCCACTAAACCTTGCTCAGGACTGGTCTCAGGTTCCCTTTGTTGCTCCACAGTGCCCT
GGAGATAGGGGAGGTCGGAGGGTGCTCCCTGACTCCCCAACCTGGGCTGTGTCTCCCTGACAGGCCTGGCTCTGAGACAG
GGGTTGGCCCCGGGCAGGCAATTCTCCAGCTGGCTTGTGAGATCCTGGGGACGGGATTCGGACACTCTGGAAGCTGAGGC
CAGGAAGGTCTGTCACCTCGTTCTTCTGGACCTCTCCTCCCGCACATGTCACCCCACACCGGCTCCCTCTCCCAGTCTCA
GCCACAACCTCTTGGTCCTCAGTGGCCTGAGCCAGGATCCACACTCCTCCCCGTCACTCTCCCGTCTCACACACAAACAC
ATGGTGCGTCTCCAGAGGCCATGGAAGTGCCCGGGCCTTGCCTTTGCTGGCCTCTCTCCCAGGAAAGCTGTCCCCCTACC
CCTTGCCCACTTAGCCAGCTCCATTTACCCTTCTTGGCCTCTGTGGCGTCTTAGCAACCCTCTCGGACAGAGGGAGGTGC
TCCCCCGTTAGTGCTCCCTCCTCAGTGCTCCTGTCTCAGGGCCTTGCGGGCCACTCTCGTTTTGCTCCTGACTCGGTGAG
GGGCCTGCATGTCCATCTCCCTGTGTGTCCAGCGTGTGGTCGCGTGACTTTGGCCCACATAGATCAACTTATTTGGTCTTC
AAATAACCCTCTTCTTTTCAAACATTGGAAGATTCAGTGGAGTGAATCAGTCCCTGGCTCAAAAAAGGGTGGGAGATGAC
AGCACTGTCCTCCTAGTGGCCCCGGGAGGCCCTGGTACAGAGCAGGCCACCAGGCAGCCCTTGATAGATGAATGAACGGA
GCCACCGATGGCAGCTGTGACCAGGGCTCATGGGCGTCCTCAGGTCCCAGCTGTCATCGCTGGTTGGGCCTTCAAGCCTC
CTCCAGGTGATGCTGTTTCCCCCATGGTGATGTGGGTGCTCTGGTTGGGGCAGAGCAGCAGCCATTTGATTGAGCAAGGA
GCTCTGAGAGGTCTACACACAGGAAGGTCTGCACACTTCCTGTGTGGACAGTGATGTGTTAGTCTCCATTCTCCATTTGC
GCATTCATTCATTCATTCATTCATTCAGGAAGCATCGATGGTGCCCTCTGCTCCTGCTGGTACCACTTGGCCCCTGCCCT
CAAAAGGCTCACAGTCTGCAGGAGTGACACAGTGTGGTGGGGGCTCGCATGTGAGACCCAGCTGTTAGGGCTGAGCAAGG
GGTGGATGCAATGGAGGGCCTGGCATGAAGGGACACGTGACAAAGGTGCGGGCAGGGCACGGGGAGCAAGGAGGGTGGTC
ACTGGGCAGGCTGGCACCTGGGGCAGCCTTCAGCACCGTGAGGTCTAAAGGGGCCAGGAGGGAGTGGCCCCCTGGGCTTC
TACAGCCAGACTGTGGCTACAGGAGGGAAATGGCGGTGGTTTGCTCACGGTGAGCCAGCAGGGGCTGAAGGAGCCATGCG
GGGTGGGGGCAAATGCCCACTCTGCCCTCCTCCTGCCCTCTGACCTCCAGTCCACTTTCCACTTGCTGACCCCCAAATGA
GGCAGTCCATGGAGGCCAGGCTCTGGAGCATAGAGGGTCTGGAGAGGAGGGGAGTGCTATCCCACCCACAGGCCTAGCAC
AGGGTCTCCTTGTCTACATGAAATGCTTGCAGTGTCTTCAAAGAATAAACACAGAAAAGATATTTAATGATCCTGTCTGT
GTAGGGTGCTTGCCAAGAGCTTCACTTACATGCCTGCGCTGAAATTCATGGCAATCCGGGAGGGAGGCCTGCACCTGGAC
CCATTCATGACAGCAGGGCCGGGATGGCAGAGAATGGCACCCGTCCTCAAGCTGACAATGTTATCTCCCCTCCTCTCCTG
TCCTCTTCCTTCCCCTCTCAACCCCTCCTCCCCTCTCCTCTCTTCCCTCTCCCTGTTTCTGTCTTCTTCCTCCTCTCCCT
CTTCTCCCCTTTTTTAAAAAGCCAACATAGATCATACTATATTCATGCTCATCTATCATTTTCATTTCCCATTAAACAAT
GCATCATAAATATCTTTATAAACTAACAGAAATTTACAGTTCCTTTATAAACTATCTAAAACGAATTATCACATATAGAG
TCCATTATTACAAAATCCCCTCTAAAATGTTATTTTTTTACCCTGCTGTTACTCTTTATTCAGCCATTATTTTTTATCATG
TCTTTTTTGTCTTAATGATTAAAAATGAACAGTTTTTTAAAAAATGTGACAGTAATACTGGACATGATTTTTAAAAAATC
AAATACCAGGGAGGGAAATAAAAAATGGAAAGCAGATGAGCCCCTGCCCGCCTCAGCCCCCAGCACCACCCATCGCAGAG
GGAGCTCTCCTAACACTTCCTGGGGGTGGCAATCCGAAAAGTGACAATGCAGAGCCAAGACCCACTTCCCACCCTCAAAG
GGAGAAGGATACAACAAAGCACTTCAGAGTAAAAGACATAAAAGTGGCCGCAGATATACAGGTAGATGCTCAAACTCCTT
AAATGGAAATAAGATTCAATGTCATCCCTCAGACTAGCAAAAATCCAAAAGTTCAGTAAGCTATAGAATAATAGGCATGT
GGATAGTCACTCTTATCCACTTTGAGTGAGAGTATAAATAGGTACACCATTTTCTGGACGGAAATTCAATAATACCCGGC
```

```
AGTTTTTTAAAGCACATACAGAAAGTCCTAGAATAGGAGTTTTTTTTTTTTTTTTTTTTTTTTTCGAGACAGAGTCTCTC
TTTGTCTTCCAAGCTGGAGTGCAGTGGTGTGATCTTGGCTCACTGCAACCTCCGCCTCCCGGGTTGAAGCAATTCTCCTG
CCTCAGCCTTCTGAGTAGCTGAGATTACAGGCACCCACCACCACGTCCGGCTAATTTTTGTATGTTTAGTAGGGCTTCCC
CATGCTGGCCAGGCTGGTCTCAAACTCCTGACCTCAGGTGATCCACCCATCTCGGCCTCCCAAAGTGCTGGGATTATAGG
CGTGAGTCACCGCACTCAGCCTTCCTGGCGGTGTTTTAAAGCACATACAGTTAGATACAGAAAGTCCTAAGATAGGAATG
TTTTCTATGAGTTCTCAACACAAGCTGAAAAGATGCACACTCAAGAATTCTTATGGCAGATTGTGAAAAACAATGTCCAC
CAAGAGGGGACCGGTTAAAAAAGTGTTGCAATGCCCATGGAATGGAATAGTTTGCAACTATATAAAGAATGAGGTGGAAA
AGTTCTTCAAGATATATTGCTAGTCCAAGCGTGGTAGCTCATGCCTATAAATCGCAGCACTTTGGAAGGCCAGGGTGAGC
GGATCGCTTGAGCCCAGGGGTTTGAGATCAGCCTGGGCAACACAGCAAAACCCTGTCTCTAAAAAAATAAAATAAACAA
CATAAAAAAAGGAAAAACTAGCCAGGCATGGTGGCATGTGCCTGTAGTCCCAGCTACTTGGGAGGCTGAGGTTGGAGGAT
CGATTGAGCCTGGGAGGTCTAGGCTGCAGTGAGCTGTGATCATGCTACTGCACTCCAGCCTGGGTGACAGAGCAAGACTC
TGTCTCAAAAGAAAAAAAAAGATATATTGTTAAGTGATCTTATTTGTGTAAATTTAAAAAACGATGTATCGGCAGGGCT
TGGTGGCTCACACCTGTAATCCCAGCAATTTGGGCAGCTGAGGCAGGAGGATCGCTTGAGGACAGGAGTTCGAGACCAGCC
TGGGCAACATAGTGAGAACCCCCTCTTCTTAATTAAAACATTTTTTCCTTAAAAAAATGATGTACTTATAAATACTTGCA
TATGCATACCTTCTGCAAAATTTCCAGAAGGCTGTGCAATAAACCGTTAAAGATGGTAAACTCTTTGAACACCTACAATG
AGCTATGTATGTCCTTTATCTCTAATCCTTCCCAAAACTTGCAAGGAATGTATTATTAGCTCCATTTTGCTGGCAAAAGA
TTGAGCCTCAGGTTGTTTAAGTAACTTGCCTCAGGACATACAGTTAGTAGATGACCCAAAGCTTCCATTTTGTGCCCTGT
ATTAGGTGAAAGGGAACCGAACTAAAAGTCACAAAAGCTGGTCTTTAGTCCTGGAGCAGCCTCCAACTAGCTAGTAACCT
TTCTCAAGTCATTTTGCTCGAGGGGCCTTGGTTTGCTGTATTTTCTACCTGTCCCAGGAGGTAGCTGTGATGGTTGTGTG
TGCTTCTTAGCACAGGTGTGAGCCACCACGCCCTGCCAATACATCATTTTTTAAATTTACACAAATAAGATAGTGTGAAT
GTGGGAGAATGAGGCAAACCAAGACAGAATACATCCAAGAGTTTAACCAGGAGGCCACAGTTGCAAGGGCTTTGGAAGGT
CTGATGTGGAATAACTGAAAAGCCAGGCAGACTCCCTGGAGGAAGGGGTAAAGGAGTAATAGCACAAACATATGAAACCT
TTTATTTACAGAGCCATGTGCCAAGCCTTTTGTACATGCTTTCCCCAAAGCATCCTACCAGCCCTGGGAGATGGGGTATT
TGCTTTTACCTTGGAAACAGCCCCAGAGAGGGTCTGCATCCTGCTCAAGTGCACCGTTAGTGTGATGCATGGAGCTGGAA
TTCTAGCCCAACCACCAGACTCTGGAGCCCCAGCTCCTTTCCCTGCTCCTCATCCTTGCTGCCCATCTTCGTCCAGCCCA
TTCACACTCTCTGCATTCCTTCCTTAGGTCAGGAAAAGCCCACCGAAGGGCCAAGAAACACCTGCCTGGGGAGCAACAAC
ATGTACGACATCTTCAACTTGAATGACAAGGCTTTGTGCTTCACCAAGTGCAGGCAGTCGGGCAGCGACTCCTGCAATGT
GGAAAACTTGCAGAGGTGAGGGGGCCCCCTGAGCTGGAGGGGGAATCTGAAGCTGCTGGGAGGAGGACTATCAGGAAGGG
ATGGGGCCATCTTCCCCCATGGCCCCAGAAAATGTTGCTCCCATCTGACTCTTCCACCTTGTTTGACACCACAGTCCCCC
TGACCCAACATCCCCATTCTAGCAGTTGGGGTACCTACTGATCTAGGGTATTCAGAGATGAATCCACTCCTATCCTGGCC
CTTGGGAGTTGTGGAATGACAGACACAGACCCAAATAATGATGACTCCAGAGTGTATACAGAATGGCAGACAAGGTGCCA
GGTTAGCCCAGAGGAGGGAAGTTGGCCAAGGCTTCACAGAGGAGGAAACCTGGAAAGGGCTTTGAGGGTGCACAGGAGTT
TTCCAGCTGGATCTAAACAATGGTGAGGATACAGGCAACAGAACTTGAAAGTAATGGGGGATAACTGCAATGCTTTGAAC
TGTAACAATGTAAACCGCGTGGGGATGGAAGAGACAATGGGCAGATGCGATGGGTGGGAGATAGGACTGGAAATATAAGC
AGAGACTAAGGTGTGAAGGGTTGTGTAAGTTATACCAATGGGTGAGGGCGGCAGGGCAATGACATATTTTAAGCGCAGGG
AGAGTATAGTCAACTCTGCGTTTTGGAATGATTGCTCAGGCTTCAGGGACCGGTGATGCAGGAAGACATGACCCAGTAGG
ATGCATCACAGGGGTCTGGGTGGGAGTGAGGGCCAAGTCAGCACCACTAGCAGCAATTGGCATTGCGTGCTTGGAAAGGA
GAGATGGATTGGGGAGATGATACGATGTAGACTCCGTAAGACAAGAGACTGGTTAGATGTGGGGGTGAGGGAAATGAAGG
GGTTTAGAATACAACATCTTTAGGAGCCATCTCCAAAAATATCTCTGCCTCTCTTTCCTTTTCCCACTCCTAATCAAAGT
TGCCCATAACTCTTCTGGGATGCCTAGAAAAGCACGTGGCTTACATTAGTACTTAATAAATATGTGTTAAAGGAATGAGT
TAAAGCCCTGCGCTCTCAGGCTTGGTGTTAGGACAGCCATCTCATTGGATCATGTTATTTCAGTGGCCTGAAAAGGCTTC
CATTCCCACCTGCAGGTGATCCAGGAGCTTCTTAGCACCCCAGAGCTGTTCAGAGGCTCCTCTTTGTGCTGCCCAGGTAT
ATGCCCAAAAGAATTTAAAACAAGTCTCCAAACAAAAGCTTATACATGGGCCAGGCACGGCGGCTCACACAAGTAATCCT
AGCACTTTGGGAGGCCGAGGTGGGCAGATCACTTGAGGTCGGGAGTTTGAGACCAGCCTGGCCAACATGGTGAAACCCTG
TCTCTACTGAAAATACAAAAAAAAAAAAAAAAAAAATTGCCAGGCTTGGTGACAGGCGCCTGTAATCCCAGCTACTCGGGAG
GCTGAGGTGGGAGAATTGCTTGAACCCAGGAGGTAAAGGTTGCAGTGACCTGAGATCACTCCACTGCACTCCAGCCTGGG
CGACAGAGTGAGACTCCATCTCAAAAAAAAAAAAAAAAAAGCAGCAGCAGCAGCAGCAGCATCTTGTACGTGAATAT
TTGTAGCAGCACTTTTCACAGTAGCCAAAAAAAAAAGAGGAAAATACCCAAATGTCCCTCAATGGATAACGGCATAATTA
AAATATGATCTCTCCATATGATGAAAT
```

HUMAN mRNA SEQUENCE : hR7-157.1 (Seq ID No: 87)

```
GGGCAGGAGAGGGGTGTCTCCCCACCAAACAAACCCGGTCCCTCCCTCTCCGCACTAGCTGTCTGCCCTGCCCTGCCGTA
GGAGATGGGCTGGGAGCCTCCCACGCTCTCCAGCTCACTCGGCAGGCAGCGGGGACCAGGGCTGGCAGGTTAAGCCTCTG
GGGGTGGATCCTGAAAGGTGGTCCAGCCGCCTGGCCCTGCGTGGGACCCTCCACCTGGCAGCAGGTGGTGACTTCCAAGA
GTGACTCCGTCGGAGGAAAATGACTCCCCAGTCGCTGCTGCAGACGACACTGTTCCTGCTGAGTCTGCTCTTCCTGGTCC
AAGGCAGGTGCCCACGGCAGGGGCACAGGGAAGACTTTCGCTTCTGCAGCCAGCGGAACCAGACACACAGGAGCAGCCT
CCACTACAAACCCACACCAGACCTGCGCATCTCCATCGAGAACTCCGAAGAGGCCCTCACAGTCCATGCCCCTTTCCCTG
CAGCCCACCCTGCTTCCCGATCCTTCCCTGACCCCAGGGGCCTCTACCACTTCTGCCTCTACTGGAACCGACATGCTGGG
AGATTACATCTTCTCTATGGCAAGCGTGACTTCTTGCTGAGTGACAAAGCCTCTAGCCTCCTCTGCTTCCAGCACCAGGA
GGAGAGCCTGGCTCAGGGCCCCCCGCTGTTAGCCACTTCTGTCACCTCCTGGTGGAGCCCTCAGAACATCAGCCTGCCCA
GTGCCGCCAGCTTCACCTTCTCCTTCCACAGTCCTCCCCACACGGCCGCTCACAATGCCTCGGTGGACATGTGCGAGCTC
```

```
AAAAGGGACCTCCAGCTGCTCAGCCAGTTCCTGAAGCATCCCCAGAAGGCCTCAAGGAGGCCCTCGGCTGCCCCCGCCAG
CCAGCAGTTGCAGAGCCTGGAGTCGAAACTGACCTCTGTGAGATTCATGGGGGACATGGTGTCCTTCGAGGAGGACCGGA
TCAACGCCACGGTGTGGAAGCTCCAGCCCACAGCCGGCCTCCAGGACCTGCACATCCACTCCCGGCAGGAGGAGGAGCAG
AGCGAGATCATGGAGTACTCGGTGCTGCTGCCTCGAACACTCTTCCAGAGGACGAAAGGCCGGAGCGGGGAGGCTGAGAA
GAGACTCCTCCTGGTGGACTTCAGCAGCCAAGCCCTGTTCCAGGACAAGAATTCCAGCCAAGTCCTGGGTGAGAAGGTCT
TGGGGATTGTGGTACAGAACACCAAAGTAGCCAACCTCACGGAGCCCGTGGTGCTCACTTTCCAGCACCAGCTACAGCCG
AAGAATGTGACTCTGCAATGTGTGTTCTGGGTTGAAGACCCCACATTGAGCAGCCCGGGGCATTGGAGCAGTGCTGGGTG
TGAGACCGTCAGGAGAGAAACCCAAACATCCTGCTTCTGCAACCACTTGACCTACTTTGCAGTGCTGATGGTCTCCTCGG
TGGAGGTGGACGCCGTGCACAAGCACTACCTGAGCCTCCTCTCCTACGTGGGCTGTGTCGTCTCTGCCCTGGCCTGCCTT
GTCACCATTGCCGCCTACCTCTGCTCCAGGAGGAAACCTCGGGACTACACCATCAAGGTGCACATGAACCTGCTGCTGGC
CGTCTTCCTGCTGGACACGAGCTTCCTGCTCAGCGAGCCGGTGGCCCTGACAGGCTCTGAGGCTGGCTGCCGAGCCAGTG
CCATCTTCCTGCACTTCTCCCTGCTCACCTGCCTTTCCTGGATGGGCCTCGAGGGGTACAACCTCTACCGACTCGTGGTG
GAGGTCTTTGGCACCTATGTCCCTGGCTACCTACTCAAGCTGAGCGCCATGGGCTGGGGCTTCCCCATCTTTCTGGTGAC
GCTGGTGGCCCTGGTGGATGTGGACAACTATGGCCCCATCATCTTGGCTGTGCATAGGACTCCAGAGGGCGTCATCTACC
CTTCCATGTGCTGGATCCGGGACTCCCTGGTCAGCTACATCACCAACCTGGGCCTCTTCAGCCTGGTGTTTCTGTTCAAC
ATGGCCATGCTAGCCACCATGGTGGTGCAGATCCTGCGGCTGCGCCCCCACACCCAAAAGTGGTCACATGTGCTGACACT
GCTGGGCCTCAGCCTGGTCCTTGGCCTGCCCTGGGCCTTGATCTTCTTCTCCTTTGCTTCTGGCACCTTCCAGCTTGTCG
TCCTCTACCTTTTCAGCATCATCACCTCCTTCCAAGGCTTCCTCATCTTCATCTGGTACTGGTCCATGCGGCTGCAGGCC
CGGGGTGGCCCCTCCCCTCTGAAGAGCAACTCAGACAGCGCCAGGCTCCCCATCAGCTCGGGCAGCACCTCGTCCAGCCG
CATCTAGGCCTCCAGCCCACCTGCCCATGTGATGAAGCAGAGATGCGGCCTCGTCGCACACTGCCTGTGGCCCCCGAGCC
AGGCCCAGCCCCAGGCCAGTCAGCCGCAGACTTTGGAAAGCCCAACGACCATGGAGAGATGGGCCGTTGCCATGGTGGAC
GGACTCCCGGGCTGGGCTTTTGAATTGGCCTTGGGGACTACTCGGCTCTCACTCAGCTCCCACGGGACTCAGAAGTGCGC
CGCCATGCTGCCTAGGGTACTGTCCCACATCTGTCCCAACCCAGCTGGAGGCCTGGTCTCTCCTTACAACCCCTGGGCC
CAGCCCTCATTGCTGGGGGCCAGGCCTTGGATCTTGAGGGTCTGGCACATCCTTAATCCTGTGCCCCTGCCTGGGACAGA
AATGTGGCTCCAGTTGCTCTGTCTCTCGTGGTCACCCTGAGGGCACTCTGCATCCTCTGTCATTTTAACCTCAGGTGGCA
CCCAGGGCGAATGGGGCCCAGGGCAGACCTTCAGGGCCAGAGCCCTGGCGGAGGAGAGGCCCTTTGCCAGGAGCACAGCA
GCAGCTCGCCTACCTCTGAGCCCAGGCCCCCTCCCTCCCTCAGCCCCCCAGTCCTCCCTCCATCTTCCTGGGGTTCTCC
TCCTCTCCCAGGGCCTCCTTGCTCCTTCGTTCACAGCTGGGGGTCCCCGATTCCAATGCTGTTTTTTGGGGAGTGGTTTC
CAGGAGCTGCCTGGTGTCTGCTGTAAATGTTTGTCTACTGCACAAGCCTCGGCCTGCCCCTGAGCCAGGCTCGGTACCGA
TGCGTGGGCTGGGCTAGGTCCCTCTGTCCATCTGGGCCTTTGTATGAGCTGCATTGCCCTTGCTCACCCTGACCAAGCAC
ACGCCTCAGAGGGGCCCTCAGCCTCTCCTGAAGCCCTCTTGTGGCAAGAACTGTGGACCATGCCAGTCCCGTCTGGTTTC
CATCCCACCACTCCAAGGACTGAGACTGACCTCCTCTGGTGACACTGGCCTAGAGCCTGACACTCTCCTAAGAGGTTCTC
TCCAAGCCCCCAAATAGCTCCAGGCGCCCTCGGCCGCCCATCATGGTTAATTCTGTCCAACAAACACACGGGTAGATT
GCTGGCCTGTTGTAGGTGGTAGGGACACAGATGACCGACCTGGTCACTCCTCCTGCCAACATTCAGTCTGGTATGTGAGG
CGTGCGTGAAGCAAGAACTCCTGGAGCTACAGGGACAGGGAGCCATCATTCCTGCCTGGGAATCCTGGAAGACTTCCTGC
AGGAGTCAGCGTTCAATCTTGACCTTGAAGATGGGAAGGATGTTCTTTTTACGTACCAATTCTTTTGTCTTTTGATATTA
AAAAGAAGTACATGTTCATTGTAGAGAATTTGGAAACTGTAGAAGAGAATCAAGAAGAAAAATAAAAATCAGCTGTTGTA
ATCGCCTAGCAAACTGGCGTAAGCATTTTGGGTCATTTCCTTCTGAGCTTTTTCCTGTGCTTCTATTGAAAGAGTCACTG
ATACTGATACAAATACCTTGGGGCCAATCAGGAAACATGAAAGAGAGAAGAGGGCTGTGTGTGAA
```

HUMAN PROTEIN SEQUENCE : hP7-157.1 (Seq ID No: 88)
```
MCELKRDLQLLSQFLKHPQKASRRPSAAPASQQLQSLESKLTSVRFMGDMVSFEEDRINATVWKLQPTAGLQDLHIHSRQ
EEEQSEIMEYSVLLPRTLFQRTKGRSGEAEKRLLLVDFSSQALFQDKNSSQVLGEKVLGIVVQNTKVANLTEPVVLTFQH
QLQPKNVTLQCVFWVEDPTLSSPGHWSSAGCETVRRETQTSCFCNHLTYFAVLMVSSVEVDAVHKHYLSLLSYVGCVVSA
LACLVTIAAYLCSRRKPRDYTIKVHMNLLLAVFLLDTSFLLSEPVALTGSEAGCRASAIFLHFSLLTCLSWMGLEGYNLY
RLVVEVFGTYVPGYLLKLSAMGWGFPIFLVTLVALVDVDNYGPIILAVHRTPEGVIYPSMCWIRDSLVSYITNLGLFSLV
FLFNMAMLATMVVQILRLRPHTQKWSHVLTLLGLSLVLGLPWALIFFSFASGTFQLVVLYLFSIITSFQGFLIFIWYWSM
RLQARGGPSPLKSNSDSARLPISSGSTSSSRI*
```

HUMAN mRNA SEQUENCE : hR7-157.2 (Seq ID No: 89)
```
CTCCCAACAGCCTGGGCGGCCGCTGAGACCCAGAGAACCCAAGGACTCCCCTGGGCTCATCCAGCAGCCTCTGCTTCCCA
GGAGAGAGGTGCTGAAGTCCACGAAGAGGTGGTGACTTCCAAGAGTGACTCCGTCGGAGGAAAATGACTCCCCAGTCGCT
GCTGCAGACGACACTGTTCCTGCTGAGTCTGCTCTTCCTGGTCCAAGGCAGGTGCCCACGGCAGGGGCCACAGGGAAGAC
TTTCGCTTCTGCAGCCAGCGGAACCAGACACACAGGAGCAGCCTCCACTACAAACCCACACCAGACCTGCGCATCTCCAT
CGAGAACTCCGAAGAGGCCCTCACAGTCCATGCCCCTTTCCCTGCAGCCCACCTGCTTCCCGATCCTTCCCTGACCCCA
GGGGCCTCTACCACTTCTGCCTCTACTGGAACCGACATGCTGGGAGATTACATCTTCTCTATGGCAAGCGTGACTTCTTG
CTGAGTGACAAAGCCTCTAGCCTCCTCTGCTTCCAGCACCAGGAGGAGAGCCTGGCTCAGGGCCCCCCGCTGTTAGCCAC
TTCTGTCACCTCCTGGTGGAGCCCTCAGAACATCAGCCTGCCCAGTGCCGCCAGCTTCACCTTCTCCTTCCACAGTCCTC
CCCACACGGCCGCTCACAATGCCTCGGTGGACATGTGCGAGCTCAAAAGGGACCTCCAGCTGCTCAGCCAGTTCCTGAAG
```

378

```
CATCCCCAGAAGGCCTCAAGGAGGCCCTCGGCTGCCCCCGCCAGCCAGCAGTTGCAGAGCCTGGAGTCGAAACTGACCTC
TGTGAGATTCATGGGGGACATGGTGTCCTTCGAGGAGGACCGGATCAACGCCACGGTGTGGAAGCTCCAGCCCACAGCCG
GCCTCCAGGACCTGCACATCCACTCCCGGCAGGAGGAGGAGCAGAGCGAGATCATGGAGTACTCGGTGCTGCTGCCTCGA
ACACTCTTCCAGAGGACGAAAGGCCGGAGCGGGGAGGCTGAGAAGAGACTCCTCCTGGTGGACTTCAGCAGCCAAGCCCT
GTTCCAGGACAAGAATTCCAGCCAAGTCCTGGGTGAGAAGGTCTTGGGGATTGTGGTACAGAACACCAAAGTAGCCAACC
TCACGGAGCCCGTGGTGCTCACTTTCCAGCACCAGCTACAGCCGAAGAATGTGACTCTGCAATGTGTGTTCTGGGTTGAA
GACCCCACATTGAGCAGCCCGGGGCATTGGAGCAGTGCTGGGTGTGAGACCGTCAGGAGAGAAACCCAAACATCCTGCTT
CTGCAACCACTTGACCTACTTTGCAGTGCTGATGGTCTCCTCGGTGGAGGTGGACGCCGTGCACAAGCACTACCTGAGCC
TCCTCTCCTACGTGGGCTGTGTCGTCTCTGCCCTGGCCTGCCTTGTCACCATTGCCGCCTACCTCTGCTCCAGGAGGAAA
CCTCGGGACTACACCATCAAGGTGCACATGAACCTGCTGCTGGCCGTCTTCCTGCTGGACACGAGCTTCCTGCTCAGCGA
GCCGGTGGCCCTGACAGGCTCTGAGGCTGGCTGCCGAGCCAGTGCCATCTTCCTGCACTTCTCCCTGCTCACCTGCCTTT
CCTGGATGGGCCTCGAGGGGTACAACCTCTACCGACTCGTGGTGGAGGTCTTTGGCACCTATGTCCCTGGCTACCTACTC
AAGCTGAGCGCCATGGGCTGGGGCTTCCCCATCTTTCTGGTGACGCTGGTGGCCCTGGTGGATGTGGACAACTATGGCCC
CATCATCTTGGCTGTGCATAGGACTCCAGAGGGCGTCATCTACCCTTCCATGTGCTGGATCCGGGACTCCCTGGTCAGCT
ACATCACCAACCTGGGCCTCTTCAGCCTGGTGTTTCTGTTCAACATGGCCATGCTAGCCACCATGGTGGTGCAGATCCTG
CGGCTGCGCCCCCACACCCAAAAGTGGTCACATGTGCTGACACTGCTGGGCCTCAGCCTGGTCCTTGGCCTGCCCTGGGC
CTTGATCTTCTTCTCCTTTGCTTCTGGCACCTTCCAGCTTGTCGTCCTCTACCTTTTCAGCATCATCACCTCCTTCCAAG
GCTTCCTCATCTTCATCTGGTACTGGTCCATGCGGCTGCAGGCCCGGGGTGGCCCCTCCCCTCTGAAGAGCAACTCAGAC
AGCGCCAGGCTCCCCATCAGCTCGGGCAGCACCTCGTCCAGCCGCATCTAGGCCTCCAGCCCACCTGCCCATGTGATGAA
GCAGAGATGCGGCCTCGTCGCACACTGCCTGTGGCCCCCGAGCCAGGCCCAGCCCCAGGCCAGTCAGCCGCAGACTTTGG
AAAGCCCAACGACCATGGAGAGATGGGCCGTTGCCATGGTGGACGGACTCCCGGGCTGGGCTTTTGAATTGGCCTTGGGG
ACTACTCGGCTCTCACTCAGCTCCCACGGGACTCAGAAGTGCGCCGCCATGCTGCCTAGGGTACTGTCCCCACATCTGTC
CCAACCCAGCTGGAGGCCTGGTCTCTCCTTACAACCCCTGGGCCCAGCCCTCATTGCTGGGGGGCCAGGCCTTGGATCTTG
AGGGTCTGGCACATCCTTAATCCTGTGCCCCTGCCTGGGACAGAAATGTGGCTCCAGTTGCTCTGTCTCTCGTGGTCACC
CTGAGGGCACTCTGCATCCTCTGTCATTTTAACCTCAGGTGGCACCCAGGGCGAATGGGGCCCAGGGCAGACCTTCAGGG
CCAGAGCCCTGGCGGAGGAGAGGCCCTTTGCCAGGAGCACAGCAGCAGCTCGCCTACCTCTGAGCCCAGGCCCCCTCCCT
CCCTCAGCCCCCCAGTCCTCCCTCCATCTTCCCTGGGGTTCTCCTCCTCTCCCAGGGCCTCCTTGCTCCTTCGTTCACAG
CTGGGGGTCCCCGATTCCAATGCTGTTTTTTGGGGAGTGGTTTCCAGGAGCTGCCTGGTGTCTGCTGTAAATGTTTGTCT
ACTGCACAAGCCTCGGCCTGCCCCTGAGCCAGGCTCGGTACCGATGCGTGGGCTGGGCTAGGTCCCTCTGTCCATCGGGG
CCTTTGTATGAGCTGCATTGCCCTTGCTCACCCTGACCAAGCACACGCCTCAGAGGGGCCCTCAGCCTCTCCTGAAGCCC
TCTTGTGGCAAGAACTGTGGACCATGCCAGTCCCGTCTGGTTTCCATCCCACCACTCCAAGGACTGAGACTGACCTCCTC
TGGTGACACTGGCCTAGAGCCTGACACTCTCCTAAGAGGTTCTCTCCAAGCCCCCAAATAGCTCCAGGCGCCCTCGGCCG
CCCATCATGGTTAATTCTGTCCAACAAACACACGGGTAGATTGCTGGCCTGTTGTAGGTGGTAGGGACACAGATGAGGC
GACCTGGTCACTCCTCCTGCCAACATTCAGTCTGGTATGTGAGGCGTGCGTGAAGCAAGAACTCCTGGAGCTACAGGGAC
AGGGAGCCATCATTCCTGCCTGGGAATCCTGGAAGACTTCCTGCAGGAGTCAGCGTTCAATCTTGACCTTGAAGATGGGA
AGGATGTTCTTTTTACGTACCAATTCTTTTGTCTTTTGATATTAAAAAGAAGTACATGTTCATTGTAGAGAATTTGGAAA
CTGTAGAAGAGAATCAAGAAGAAAAATAAAAATCAGCTGTTGTAATCGCCTAGCAAACTGGCGTAAGCATTTTGGGTCAT
TTCCTTCTGAGCTTTTTCCTGTGCTTCTATTGAAAGAGTCACTGATACTGATACAAATACCTTGGGGCCAATCAGGAAAC
ATGAAAGAGAGAAGAGGGCTGTGTGTGAA
```

HUMAN PROTEIN SEQUENCE : hP7-157.2 (Seq ID No: 90)
```
MCELKRDLQLLSQFLKHPQKASRRPSAAPASQQLQSLESKLTSVRFMGDMVSFEEDRINATVWKLQPTAGLQDLHIHSRQ
EEEQSEIMEYSVLLPRTLFQRTKGRSGEAEKRLLLVDFSSQALFQDKNSSQVLGEKVLGIVVQNTKVANLTEPVVLTFQH
QLQPKNVTLQCVFWVEDPTLSSPGHWSSAGCETVRRETQTSCFCNHLTYFAVLMVSSVEVDAVHKHYLSLLSYVGCVVSA
LACLVTIAAYLCSRRKPRDYTIKVHMNLLLAVFLLDTSFLLSEPVALTGSEAGCRASAIFLHFSLLTCLSWMGLEGYNLY
RLVVEVFGTYVPGYLLKLSAMGWGFPIFLVTLVALVDVDNYGPIILAVHRTPEGVIYPSMCWIRDSLVSYITNLGLFSLV
FLFNMAMLATMVVQILRLRPHTQKWSHVLTLLGLSLVLGLPWALIFFSFASGTFQLVVLYLFSIITSFQGFLIFIWYWSM
RLQARGGPSPLKSNSDSARLPISSGSTSSSRI*
```

HUMAN mRNA SEQUENCE : hR7-157.3 (Seq ID No: 91)
```
TTGAGACAGGGTCTCGCTCTGTCACACAGGCTGGAGTGCAGTGGTGTGATCTTGGCTCATCGTAACCTCCACCTCCCGGG
TTCAAGTGATTCTCATGCCTCAGCCTCCCGAGTAGCTGGGATTACAGGTGGTGACTTCCAAGAGTGACTCCGTCGGAGGA
AAATGACTCCCCAGTCGCTGCTGCAGACGACACTGTTCCTGCTGAGTCTGCTCTTCCTGGTCCAAGGCAGGTGCCCACGG
CAGGGGCCACAGGGAAGACTTTCGCTTCTGCAGCCAGCGGAACCAGACACACAGGAGCAGCCTCCACTACAAACCCACAC
CAGACCTGCGCATCTCCATCGAGAACTCCGAAGAGGCCCTCACAGTCCATGCCCCTTTCCCTGCAGCCCACCCTGCTTCC
CGATCCTTCCCTGACCCCAGGGGCCTCTACCACTTCTGCCTCTACTGGAACCGACATGCTGGGAGATTACATCTTCTCTA
TGGCAAGCGTGACTTCTTGCTGAGTGACAAAGCCTCTAGCCTCCTCTGCTTCCAGCACCAGGAGGAGAGCCTGGCTCAGG
GCCCCCGCTGTTAGCCACTTCTGTCACCTCCTGGTGGAGCCCTCAGAACATCAGCCTGCCCAGTGCCGCCAGCTTCACC
TTCTCCTTCCACAGTCCTCCCCACACGGCCGCTCACAATGCCTCGGTGGACATGTGCGAGCTCAAAAGGGACCTCCAGCT
GCTCAGCCAGTTCCTGAAGCATCCCCAGAAGGCCTCAAGGAGGCCCTCGGCTGCCCCCGCCAGCCAGCAGTTGCAGAGCC
TGGAGTCGAAACTGACCTCTGTGAGATTCATGGGGGACATGGTGTCCTTCGAGGAGGACCGGATCAACGCCACGGTGTGG
```

```
AAGCTCCAGCCCACAGCCGGCCTCCAGGACCTGCACATCCACTCCCGGCAGGAGGAGGAGCAGAGCGAGATCATGGAGTA
CTCGGTGCTGCTGCCTCGAACACTCTTCCAGAGGACGAAAGGCCGGAGCGGGGAGGCTGAGAAGAGACTCCTCCTGGTGG
ACTTCAGCAGCCAAGCCCTGTTCCAGGACAAGAATTCCAGCCAAGTCCTGGGTGAGAAGGTCTTGGGGATTGTGGTACAG
AACACCAAAGTAGCCAACCTCACGGAGCCCGTGGTGCTCACTTTCCAGCACCAGCTACAGCCGAAGAATGTGACTCTGCA
ATGTGTGTTCTGGGTTGAAGACCCCACATTGAGCAGCCCGGGGCATTGGAGCAGTGCTGGGTGTGAGACCGTCAGGAGAG
AAACCCAAACATCCTGCTTCTGCAACCACTTGACCTACTTTGCAGTGCTGATGGTCTCCTCGGTGGAGGTGGACGCCGTG
CACAAGCACTACCTGAGCCTCCTCCTACGTGGGCTGTGTCGTCTCTGCCCTGGCCTGCCTTGTCACCATTGCCGCCTA
CCTCTGCTCCAGGGTGCCCCTGCCGTGCAGGAGGAAACCTCGGGACTACACCATCAAGGTGCACATGAACCTGCTGCTGG
CCGTCTTCCTGCTGGACACGAGCTTCCTGCTCAGCGAGCCGGTGGCCCTGACAGGCTCTGAGGCTGGCTGCCGAGCCAGT
GCCATCTTCCTGCACTTCTCCCTGCTCACCTGCCTTTCCTGGATGGGCCTCGAGGGGTACAACCTCTACCGACTCGTGGT
GGAGGTCTTTGGCACCTATGTCCCTGGCTACCTACTCAAGCTGAGCGCCATGGGCTGGGGCTTCCCCATCTTTCTGGTGA
CGCTGGTGGCCCTGGTGGATGTGGACAACTATGGCCCCATCATCTTGGCTGTGCATAGGACTCCAGAGGGCGTCATCTAC
CCTTCCATGTGCTGGATCCGGGACTCCCTGGTCAGCTACATCACCAACCTGGGCCTCTTCAGCCTGGTGTTTCTGTTCAA
CATGGCCATGCTAGCCACCATGGTGGTGCAGATCCTGCGGCTGCGCCCCCACACCCAAAAGTGGTCACATGTGCTGACAC
TGCTGGGCCTCAGCCTGGTCCTTGGCCTGCCCTGGGCCTTGATCTTCTTCTCCTTTGCTTCTGGCACCTTCCAGCTTGTC
GTCCTCTACCTTTTCAGCATCATCACCTCCTTCCAAGGCTTCCTCATCTTCATCTGGTACTGGTCCATGCGGCTGCAGGC
CCGGGGTGGCCCCTCCCCTCTGAAGAGCAACTCAGACAGCGCCAGGCTCCCCATCAGCTCGGGCAGCACCTCGTCCAGCC
GCATCTAGGCCTCCAGCCCACCTGCCCATGTGATGAAGCAGAGATGCGGCCTCGTCGCACACTGCCTGTGGCCCCCGAGC
CAGGCCCAGCCCCAGGCCAGTCAGCCGCAGACTTTGGAAAGCCCAACGACCATGGAGAGATGGGCCGTTGCCATGGTGGA
CGGACTCCCGGGCTGGGCTTTTGAATTGGCCTTGGGGACTACTCGGCTCTCACTCAGCTCCCACGGGACTCAGAAGTGCG
CCGCCATGCTGCCTAGGGTACTGTCCCCACATCTGTCCCAACCCAGCTGGAGGCCTGGTCTCTCCTTACAACCCCTGGGC
CCAGCCCTCATTGCTGGGGGCCAGGCCTTGGATCTTGAGGGTCTGGCACATCCTTAATCCTGTGCCCCTGCCTGGGACAG
AAATGTGGCTCCAGTTGCTCTGTCTCTCGTGGTCACCCTGAGGGCACTCTGCATCCTCTGTCATTTTAACCTCAGGTGGC
ACCCAGGGCGAATGGGGCCCAGGGCAGACCTTCAGGGCCAGAGCCCTGGCGGAGGAGAGGCCCTTTGCCAGGAGCACAGC
AGCAGCTCGCCTACCTCTGAGCCC
```

HUMAN PROTEIN SEQUENCE : hP7-157.3 (Seq ID No: 92)
```
MCELKRDLQLLSQFLKHPQKASRRPSAAPASQQLQSLESKLTSVRFMGDMVSFEEDRINATVWKLQPTAGLQDLHIHSRQ
EEEQSEIMEYSVLLPRTLFQRTKGRSGEAEKRLLLVDFSSQALFQDKNSSQVLGEKVLGIVVQNTKVANLTEPVVLTFQH
QLQPKNVTLQCVFWVEDPTLSSPGHWSSAGCETVRRETQTSCFCNHLTYFAVLMVSSVEVDAVHKHYLSLLSYVGCVVSA
LACLVTIAAYLCSRVPLPCRRKPRDYTIKVHMNLLLAVFLLDTSFLLSEPVALTGSEAGCRASAIFLHFSLLTCLSWMGL
EGYNLYRLVVEVFGTYVPGYLLKLSAMGWGFPIFLVTLVALVDVDNYGPIILAVHRTPEGVIYPSMCWIRDSLVSYITNL
GLFSLVFLFNMAMLATMVVQILRLRPHTQKWSHVLTLLGLSLVLGLPWALIFFSFASGTFQLVVLYLFSIITSFQGFLIF
IWYWSMRLQARGGPSPLKSNSDSARLPISSGSTSSSRI*
```

HUMAN mRNA SEQUENCE : hR7-157.4 (Seq ID No: 93)
```
TTAAAGCAGGGTCTCACTCTGTCACCCAGGCTGGAGTGCAGTGGCGCAGTTATAGCTCACTGCAGCCTCGACCTCCTGGG
CTCAAGTGATCCTTCCACCTCAGCCTCCCTAGTAGCTGGGACCACAGGTGGTGACTTCCAAGAGTGACTCCGTCGGAGGA
AAATGACTCCCCAGTCGCTGCTGCAGACGACACTGTTCCTGCTGAGTCTGCTCTTCCTGGTCCAAGGCAGGTGCCCACGG
CAGGGGCCACAGGGAAGACTTTCGCTTCTGCAGCCAGCGGAACCAGACACCAGGAGCAGCCTCCACTACAAACCCACAC
CAGACCTGCGCATCTCCATCGAGAACTCCGAAGAGGCCCTCACAGTCCATGCCCCTTTCCCTGCAGCCCACCCTGCTTCC
CGATCCTTCCCTGACCCCAGGGGCCTCTACCACTTCTGCCTCTACTGGAACCGACATGCTGGGAGATTACATCTTCTCTA
TGGCCAAGCGTGACTTCTTGCTGAGTGACAAAGCCTCTAGCCTCCTCTGCTTCCAGCACCAGGAGGAGAGCCTGGCTCAGG
GCCCCCGCTGTTAGCCACTTCTGTCACCGCCGCTCACAATGCCTCGGTGGACATGTGCGAGCTCAAAAGGGACCTCCAGCT
GCTCAGCCAGTTCCTGAAGCATCCCCAGAAGGCCTCAAGGAGGCCCTCGGCTGCCCCGCCAGCCAGCAGTTGCAGAGCC
TGGAGTCGAAACTGACCTCTGTGAGATTCATGGGGGACATGGTGTCCTTCGAGGAGGACCGGATCAACGCCACGGTGTGG
AAGCTCCAGCCCACAGCCGGCCTCCAGGACCTGCACATCCACTCCCGGCAGGAGGAGGAGCAGAGCGAGATCATGGAGTA
CTCGGTGCTGCTGCCTCGAACACTCTTCCAGAGGACGAAAGGCCGGAGCGGGGAGGCTGAGAAGAGACTCCTCCTGGTGG
ACTTCAGCAGCCAAGCCCTGTTCCAGGACAAGAATTCCAGCCAAGTCCTGGGTGAGAAGGTCTTGGGGATTGTGGTACAG
AACACCAAAGTAGCCAACCTCACGGAGCCCGTGGTGCTCACTTTCCAGCACCAGCTACAGCCGAAGAATGTGACTCTGCA
ATGTGTGTTCTGGGTTGAAGACCCCACATTGAGCAGCCCGGGGCATTGGAGCAGTGCTGGGTGTGAGACCGTCAGGAGAG
AAACCCAAACATCCTGCTTCTGCAACCACTTGACCTACTTTGCAGTGCTGATGGTCTCCTCGGTGGAGGTGGACGCCGTG
CACAAGCACTACCTGAGCCTCCTCCTACGTGGGCTGTGTCGTCTCTGCCCTGGCCTGCCTTGTCACCATTGCCGCCTA
CCTCTGCTCCAGGGTGCCCCTGCCGTGCAGGAGGAAACCTCGGGACTACACCATCAAGGTGCACATGAACCTGCTGCTGG
CCGTCTTCCTGCTGGACACGAGCTTCCTGCTCAGCGAGCCGGTGGCCCTGACAGGCTCTGAGGCTGGCTGCCGAGCCAGT
GCCATCTTCCTGCACTTCTCCCTGCTCACCTGCCTTTCCTGGATGGGCCTCGAGGGGTACAACCTCTACCGACTCGTGGT
GGAGGTCTTTGGCACCTATGTCCCTGGCTACCTACTCAAGCTGAGCGCCATGGGCTGGGGCTTCCCCATCTTTCTGGTGA
CGCTGGTGGCCCTGGTGGATGTGGACAACTATGGCCCCATCATCTTGGCTGTGCATAGGACTCCAGAGGGCGTCATCTAC
CCTTCCATGTGCTGGATCCGGGACTCCCTGGTCAGCTACATCACCAACCTGGGCCTCTTCAGCCTGGTGTTTCTGTTCAA
CATGGCCATGCTAGCCACCATGGTGGTGCAGATCCTGCGGCTGCGCCCCCACACCCAAAAGTGGTCACATGTGCTGACAC
```

```
TGCTGGGCCTCAGCCTGGTCCTTGGCCTGCCCTGGGCCTTGATCTTCTTCTCCTTTGCTTCTGGCACCTTCCAGCTTGTC
GTCCTCTACCTTTTCAGCATCATCACCTCCTTCCAAGGCTTCCTCATCTTCATCTGGTACTGGTCCATGCGGCTGCAGGC
CCGGGGTGGCCCCTCCCCTCTGAAGAGCAACTCAGACAGCGCCAGGCTCCCCATCAGCTCGGGCAGCACCTCGTCCAGCC
GCATCTAGGCCTCCAGCCCACCTGCCCATGTGATGAAGCAGAGATGCGGCCTCGTCGCACACTGCCTGTGGCCCCCGAGC
CAGGCCCAGCCCCAGGCCAGTCAGCCGCAGACTTTGGAAAGCCCAACGACCATGGAGAGATGGGCCGTTGCCATGGTGGA
CGGACTCCCGGGCTGGGCTTTTGAATTGGCCTTGGGGACTACTCGGCTCTCACTCAGCTCCCACGGGACTCAGAAGTGCG
CCGCCATGCTGCCTAGGGTACTGTCCCCACATCTGTCCCAACCCAGCTGGAGGCCTGGTCTCTCCTTACAACCCCTGGGC
CCAGCCCTCATTGCTGGGGGCCAGGCCTTGGATCTTGAGGGTCTGGCACATCCTTAATCCTGTGCCCCTGCCTGGGACAG
AAATGTGGCTCCAGTTGCTCTGTCTCTCGTGGTCACCCTGAGGGCACTCTGCATCCTCTGTCATTTTAACCTCAGGTGGC
ACCCAGGGCGAATGGGGCCCAGGGCAGACCTTCAGGGCCAGAGCCCTGGCGGAGGAGAGGCCCTTTGCCAGGAGCACAGC
AGCAGCTCGCCTACCTCTGAGCCC
```

HUMAN PROTEIN SEQUENCE : hP7-157.4 (Seq ID No: 94)
```
MCELKRDLQLLSQFLKHPQKASRRPSAAPASQQLQSLESKLTSVRFMGDMVSFEEDRINATVWKLQPTAGLQDLHIHSRQ
EEEQSEIMEYSVLLPRTLFQRTKGRSGEAEKRLLLVDFSSQALFQDKNSSQVLGEKVLGIVVQNTKVANLTEPVVLTFQH
QLQPKNVTLQCVFWVEDPTLSSPGHWSSAGCETVRRETQTSCFCNHLTYFAVLMVSSVEVDAVHKHYLSLLSYVGCVVSA
LACLVTIAAYLCSRVPLPCRRKPRDYTIKVHMNLLLAVFLLDTSFLLSEPVALTGSEAGCRASAIFLHFSLLTCLSWMGL
EGYNLYRLVVVEVFGTYVPGYLLKLSAMGWGFPIFLVTLVALVDVDNYGPIILAVHRTPEGVIYPSMCWIRDSLVSYITNL
GLFSLVFLFNMAMLATMVVQILRLRPHTQKWSHVLTLLGLSLVLGLPWALIFFSFASGTFQLVVLYLFSIITSFQGFLIF
IWYWSMRLQARGGPSPLKSNSDSARLPISSGSTSSSRI*
```

Table 15

MOUSE GENOMIC SEQUENCE : mD7-161 (Seq ID No: 95)
```
TTGTATGGGCAGGTGAGCAGCTGCCAGTCTCAAGAATGAGACCCACAGGATGCTTCCTTAGAAGAGTTGTGGGCAGGCCG
TGAAGCCAGGAGGCATAAACGTGGGAAGAGAAGGAAGGTGGCGGGGCTCGTGACCGTTAGTATCATGAACTTTTCCAAGA
TTTCTAGACTGTCACATTTCTCAGAAACAAGAAAAAACACACAGAAAAAGCTGTGTCATGTGGTGGTTGCCACCCCTCTG
CAGGTGCCCAGTCAGCAACTGTAGGCCGGCCCTTGGTGCTGCTCCACAGAAACAGAAACAGTCAGCTGTGTCCTTCCTTT
CTCACATTCCTGTCCACAAAGTCCTCAAGTAACTCCAGCCAGGGGGAGTGTCCTGCTCAGCAGCTGAGGGGTCGCCGCCT
TTACTCCAAGGCACTAGGCTTCTCCTGGATATCCTTTGTCTCCCATGGTCTGAGAAGGTCCGCTTCAACCCTTGCTT
TTCACAGCCAGCCTCCCGCCTTGGGCCAGCTGGTGAGTCTGTACCTGGGGAGGTCACACTTTCTTTGGAAAGAACCCGCC
ATTATGAGCTGGCTAGAGGAAAATGTCCATGAGGTTCTGCAAGCGGTGGATGCTGGAGACCCTGCTGTGTGGAGGCCTGTGA
GAACAGGTGAGCTGAGCCTGGCAGGTCCACTAGAACCTAGCTCAGGGAGGAACCCTGCTGGCCTGAGTCCCTTCCGTGTG
CCCTCCTGGGTCTTTATCGCAGACACTGGGCCTCAGGTTTCAGTTTAGCTTTTGAAGGAGCCACCAGTGGTCCTGGGCAT
GGTTGTTGCTGTGAAGTGTGTGGGTGTTGTGAACTTGCTGTCTGTACAGATGTGAGGTGAGTGGGCTGGTAGCACAGCTT
CAACCCAAGAGTGACAAGGGAAGCTTTCCCAGAAGTCAGACAAGCCAGTGCATACCGGGGCTCTGCAGACTGGCTATGGG
TGACTTGGCACTTTGCACCGTGGGCGCCAGTTTATCTGCTGTTGTCTACTCTGGCCTCAGGCGGAAAGTGTTGTACCAGC
GTGCGCCCAGAAACATCCACCGCCACGTGATACTGTCTGAGATCAAGGAGGCTGTTGCTGCCCTACCCTCGGTAAGAGGG
GTGTGGAATGTGTGCCGGTCATAGAATACAGCTACCCACTGGTCGGCCTGTGTGCCGTATGGGTTGGAACTGGTGCAGAA
CGTCTGCCCCCATGGCCAGTTTCTCTGGGTCTTCCTCACCATGTGACTACCTCGGTGAGCTATTCCAGCATGGTGTCACA
CTGTCTGTGACAGAGACCTAGGTCTTCCTCACAGAACCCTGCAGTAGTCCCTGTAACACACGCCCCTGCAGCAGAACCTC
GGCTCTGCCCTCACAGATCCCTGTGTTTGGCCTCTAAGGTGTGGCCAAGGGTGGATCATTTGGTGAACCAGCAGCTCAGC
GGGACCCTGGCTCCCATCCCTAGACCGTGGTCTGCAGTCAGCACAGTCCATCATTGGCAACATGAGTACAGATAGTGCCA
CCTGTGTAACGTGCCCTTTGCCCTATGTCCACTGACTGGGGCAAAATGTCACATGTTCCCCCAGACTATTCTCTAGGAGA
GACTGTTCCACCCAGACCTAAAGTTTCTTGGAGCAGAAACGGCCACTTCTGCCAGGTGTTGTCACTGCCTCCCGGCAGAT
AGGTCAGCTCTGAGGTGAGAGCAGCTGAAGCTTTGGAATGCAAAGGTGGCCAGCACTGCTCCAGAATCTGTTTTGTGTCC
TCTCCTAAGGATGTGACCACACAGTCTGTGATGGGGTTCGACCCTCTGCCTCCCCTGGACACCATCTACTCTTACGTCAG
ACCAGAGAGGTACCGCCCCTGCTTTGTCCTCACGCCCACACAACCCACTTTGGTACTTTGGGGCTCGCTGGGGCTGCCCT
TACAGACTAGAAGGAAAATGGCAAACATGGGCTGGACAGGCCAGTCCACTGGGGAGTAGGGAAACAGTGTGACACGGAGC
TCCTCGAGTCCCCAGAGGTCTGAGCACTTCCCTGTGGGTTCTGTAGCAAGTTGAGAAGTGCAGAATAAGAAGCCACCTCT
GGCCTGGTGCACTGCAGCAGACAGCAGACACCACGCCTAGAACCAAAGATGGGCAGCAGTCACATTTGTGTGGAACCTGT
GGGGAAAACGTCTAAGAAGCACGTGCCACACTGGGAAGTGACAGTCACCTGCTTTGTAGAAAGGTGTGCCCCAACCAAAG
GGTGACAACTAGCCCTCTGCTATGTGCAAGCTGTCTCCCAAGAGCACAGTGTAAGGTCTAGTGAGTGCTGAAGGAAGGTC
GTGTGGCTTTGGGGGTGGGGGGGTAAGCGCTGACGTGGGCATGCCTGTTGTGTGCATGGGGCAGCGTTAGAATCTGGGTC
ATGAAAGAGCTCTCTTCCCTTCCCTCCTAGCCTTCACGGCTGCCCTGCCTGGGTCCATTGCTCCCATGTCTGTCTCAGGC
AAGAGCCACAGGCCTTCTAGACCCTGACAGGAGGTAGTGAGTAGAGACTTCCGTTAAAGTTTTAGATTAATATGAGAATT
TTCAGTGTGCTCAGAAACATCCTACCAGGCTGTCATAGCCCAGCCATTCTAGTCTTCTACCATGCTCATGGGGTCCCCAG
ACATAAGTGTGACCACACTGTGCTATAGAGCTGTGCTTGGCCCCTGCAGGGGCCTCCCAGGGTTGCCCTTACCTTTCCTG
TCATCTTTTGTGACTGACCCGTCTTCCCCAGCATCATATACTGTAGTCACCTTGTAGTCTCTCCATGTGACCCAGCAGAT
GAGGTCCCTAAAGAGCCAGCATCTGGGGCTCCCTCCCCACTTCCCTTGTTGCCCTCGGGCAGGCTAGTAGTCAGCAGTGC
TGACCCCAGATCCTGATGTGCAGTTTAGGGCCCAGAGCCTGAGTTCCACTCAAAGGCTGCCTGTCCCCAGCAGTTAAGAG
```

```
AGTGGGTAGGGACAGCTGACCCGGGCTGGAACCCCACAGTGTCTGTTAACTTTAATTTTCTGACCCCTTTCCCTCCTCCC
TCCCTCCTTCCCTCCCTCTCTCCCCTTTCCTTTCCCTTGCTTCCTTTCTCTCTCTCTCTCTCTCTCTCTTTCTTTCTTTT
AATTTTCAGGCTGAGTCCAGTCAGCCACGGAAACACAATCGCCCTCTTCTTCCGGTCCTTGTTGCCAAATTACACCACAG
AGGTATGTTGATTAAGCACGCTGGTCTTTCCCCTCCCAAGTTAACATGGGCAAGTCCTGCTCAGGGTGGAGGCAGAAACG
GGTGAGACTGTACCCTGCCTGTGGTGCCATGCTTATAAGTGGGGACTTGGCCTGCTGGAGTTGCGTGGCTATTCTAAGGT
GAGTGGTGTGGGCTGAGAGGCGGCTCAGCATCATGGTAGGTTGGCTTTGGCTAAGTGAGCGCATCTGGCTTTCTAGAGCA
GCTGCTTCTTCTTCTGGGCAGGGACTTTGGGAGGGGACAGGCTCAATCTTGGGGCCTAAGTTGCCTCAGCCGTTGAACCC
AGGATCTCAAGTCTACCCAGACTCAAGTCTATCCCACTTAGCTGTGTCTCATTGTTCAGGGGGAGAGGCTGGAGGAAGG
AGTGGCCGGGGGTCCGAACCGCAATCAAGGCTTGAACAGGCTGATGTTGGCCGTACGTGACATGATGGCCAACTTCCACT
TCAACGACCTGGAGGTGCCTCGGGAGGACAATCCTGAGGGCGAGGGGGACTGGGACTGAACTGTCCAGATGCTGTGCCCA
GAGCCGTGCAGTCACGTTCCCGAATTCATTTCTGGTTGGAGTTAACCAGTTCCGAGTAGAAATGCTGACCATGCCATCAC
CCGTGTCTTTTGTTTTCCCTCTGAAGGGAGTGGGTTATACTCTGGGTGAGCTTCCTCCAGTGTCCTCAGAAACAGTCCCA
GAAGCAGTGCGCCCCACACTGGGTGCACCCTATCATGTGCCTCACACTTCAGCATTTGTAACATGGTGCATAGAAGGGTT
GTCCCAGGGTCTGCAGTACCAGGACCCTATCTGCTCCTTGCTAGAGGCCCCTGAACCTTCCACACATGCTACACCCAGCA
TGTTTGCAACACTGAGGTCAAACCCAGGGCCTGGCACATGGTCTGCTGCTGACTGACAGTCTCAGCCTGGGAACCTTAGA
GGAAACTGGAAGGGCTGGAAGTGTCGCAGCTGGGCAGCTGCTCCCTTAGTATGCACTGCAAAAGCCAGCGGTGCTGGTGT
TCGTGTGTAATCTCAGCATTCAAGCAGAGAGGGGGACAAGAGGGTTAGAAGTTCAGGATCACCCTCTGCTACATAATTAA
GTTCAAAGCCAGTCTGAGTTATATGAGATCCCATCTCAAAAGAGATGCATGAGGGGCCTAGAATTCAAAGAGACGTTAAA
AAAAAAAGAAATTGTCCTAAGCAGGTAAGGAATACTTTACAGAGATTAAAGGGTATTTGATCTAGGGATGAGTGGGGAAC
CCTGACCAAGAACCCGAGTCCAGTGGCAGGAACAAAGACCATAGCCAGGCAAGGCCGGAGGTCTGTGTCTCATAAGCTGT
TTGTGGAAAGCTCCAGGCTGCTGAGGTGTTACAGCTGTGTCAGGTGTCTCCCAGGCAGCACTGACGGTTTGCATCCCACA
AAGCCTAGCGGAGGCTCTAGTCTGTGGAGCAGAGTCTTAGCTGTGCAATGACAGCGAGGGCCCCCTAAATCTTGGTCCAT
TGATGCCTTCCTGGGAGTCTACGTGTTTCAGACTGGATGATTGGGTTTGGAGTACACATAAAACACGCTTTAGGACTGAG
CCATTCTGATGTAACGGTACTGGCTGGCAGGCCCGATGGCCTGAATTCAGTCCCCAGGACTCATGTGCTGCACAGTGAGA
GCTCGCTCCTGTAGCTTGTCCTCTGACCTCCACACAATACACTGCCAGGTGCACACCTACAGAATTACACCCAGGGAAATG
GAAAAGGGACAGTTTGGGGTTAAAGCACTGTGCAGAGGCCCAGGCTGTGAACTTAAAGCACACATGGAGGGTGACTCACA
ACTACCCATGATTCCAGCTCCAGTGTACACTTAAGGAGGTCTCTTAATAGGTGCTCTGGGGCAAGGGCCCTGGATGTGA
CTGAGCAGGGGCCCAGAGCACTGCCCCTTCAGCAGAATATTGAGGGCTGACTTGTTTCCATCTATCAGCCTAATGTCATA
GCCCATTCCTAAGGGGGCCATTTACAGGAGGGACTCCGTGTTCAACGTACATGCCTGGTAAGAGGTCACAGGTCACATGA
ATTGGTGCTGAATGTCTTTTTTTCTCCAGCATGAATTGGCAAGGCCATGAAGTCTGGATACAGGCTCTACAACTTCTGCT
CGTGGGTCAGCAGGGGCCTGGGAGGGCAGGCCTGAGGGGCCTGGGAGGGGCAGGCCTAAGGGCATGGCTCCCACCAGTTG
CCTCACCTATCCCTGGGTTTGGTGGGATGATCTACACCCAGTCCTGGGGAGGGTTACTGCTGAGATGGGCCTCATTCAAT
GTGAGTTTCCAAGTTGGAATGGCCTGTGGTGAGTTTCAGCCAAGGGGAGGAAGGACTGCCTAGGAATGGATGTATGTTGT
GGAAATGACAAGCAAGCGCGGAAAAACCTGGCATCCTGCCTCTTCCTTTCAGCAGCTGTAAGTCCATCCGGATGAGGATGT
GACATCTGGAGCTGCAGCAGCTATTCTGCAACCACGTGACAACGAATTCCAAAATGAAGAGCCATGCTCTAGGAACAGTG
AAGCAGAGGATACCCCAGGCCTTCAGGAACGTCCCACCTGCAGGCCTGTTTCCTGAGAGGCCATCAGATGATCGGCTTCA
GAAGTGTTGCTCAGCCTTGCAACTGAATTCATTTCTAATGGTGAAAAATAAAACCAGTCCTTCACACCACCTGTGGATCA
TCATCGGGAAAAGTAACTTTCTGAGACAGAGTCTCACTGTGTAGCCCGTAGTGGCCTGGAGCTTGCTATGTAGACCAGGC
CGCCCTTTACTCTGTAGACCAGGCTGGCCTCGGAGATCCGCCTGCCTCTCTCTCCCAAATGTTAGGATTAAAAGAGTGCA
CCACCACACCTAACCTGCATGTCATCTCAACAGCTTTAAAGTCTGGCCTGTCACCCAGGTAGGGGTGACAGTGTGTCCCG
CTCCTCCCGTACCTCCCTGGCCCCCTTTCCCAAGAGGCGCCTGCCTATGTGTTCTGTGCAGAATTGTTGGTCATTTGTGCA
TTTTCATGTAGTCAGGGAGAAAACCTGTTTGCTGCCTCTCAGAGAAAGTTCACAAGAAATGAAAGACTTTGAAACAAAAA
CATGGTGATATGGCCTTTTTAACTTTGTTTTTTTTTTGTGTGTGTGTGGGTGTTTTGCCTGAGTATGTGTCTGTGCACTG
TGTACGTGCCCACAGAAACCAGAAAAGGACATACAAAGAGGCTGTTGGATCCCTTGGAACTGGAGTCAATATCCCTTTTT
CCTTTCTATTTCTTTTTGTTGTTTATCTATTTGCTTATTTTTCGAGACAGGGTTTCTCTGTGTAGCTTTGGCTGTCCTGT
CCTCCTATCCTGGAACTTGCATTTTATGTGAGGCTGGCCTGGAACTCACAGAGGTCCGCCTCCTCTGCCTCCTAAGTGCT
TGGATTAAAGGCATGCGCTACCATGGCTCAGCAATCACATTTCTTAGTCAGCTTGTGAATGGTGAAAAGCAAACCTGTGT
CCTCTGGAAGAGCAGCCAGTGCTGTTCACTACTGAGCCAGAGCCATCTCTTTAGCCCCAGGCATGGGCTTCCTGGGAACC
TGTGCTGTCCTGTGTGACCAGAGCCAGAAGGGACATGCTCACATTGCCTGAGGGCTTTCCAGATCTAAAGACAGGGCTGA
GCCTCTCCCCATTGGGATGCCGGCTGCTCTTTGCCTCACAGAGAGATGCCCCCTTAGCCAGTCTGCAGGCAGTCCCCCCT
GCTAAGTAGGTACATGCTCCTGTGTGCCGTGTGTCACGTATGTCTCCCAGGGCCACTGAGAAGTCAGCCTTGAAGCATGA
GCTTGAGGCTATTCGGCCCCATCACAGCATAGCCAAAGCTGGTTCTCCCGGCTCTGCTCCGCTGCTCCCTTCCCTGGAGG
TTCTAGCAGTTGTACCATCAACGTCCAAACGAGTCCTGCGGCGTGCATAGTCCCCCTCCTCTCAACACTCAGCAGCAGGG
GCTTGCAGGGAGGATGTGGCTGCCTCTGCCAGAGACTCTTTGGTTCTGCCTCCCTTTGAAGTTAGATTGTCTGGAGGGTA
AACTGTAGTTGTCTCAAAGAGTTCATTCTGAAGCCAGAAATACCAACGTCTCTGAAAGGCTATGGCACAGGAAGTTAGGG
CAGGGGGGGACACTTGGTCTTTCATGTCCCAGAAGTGAAAAGTGGCCTGCAGTCCCAGGATGCCTAGTTATCATGTGGCT
TCAGAGGCTAGGCCCGCATGCCTCCACAGCCACGGCTCTGACCATCCCATTGGACCTAAGACCTCCACTAGAAGCAGAAT
AAGGCACAAGTTGCAGGCGGCAGACAGCTGGGTGAATGTGACAGTGTCAGCCCTGCCTCTACAGGCTTCCGTGGAGGCTT
GGGACACTCAGGCCACCCAGAGTCACTATATCCTCCTCTGTCCCTGTGACACAGGACACCTGAGGGTGTGAACAGTTTGT
AAATCAGGTCCACCATCCACCAGTGTGCAGGGTGGTTTGGCTAGATCTGCTAAAGCTGAAGAGTACCTGTGTCATGTCTG
CAGGGTTGTACTGGTCGGGGTCATACACATAAGCACATGTGTGTGTCTGCCAGACGGTATACAGGACCTACCAACAGCTA
```

```
GTCATGATGAGGCTGGCCACAGGCTGCCAAATGTCCAGCATGGGCCATCGACATGGCGGACGCTGCACAACCTGAATGTT
TTAATCACCTAACTAACAAGGTGCCTTAGTGTGAACAAAGTATAAGCCTGAGGATTCTTTTCTCTATGAGGTTCTGAGAC
AGACAGTGGATAGTCCTGAGGCCCAGGGAGGCCCAACACAACTCTGCTGTAAGTTGTATCTTATCAAGCGATCCGGGAAA
ATAGACCTGACCACGTTTGACTTAGAGGGTGTGCACTTCTATTTGTCATTTATTGCAGGAGTAAAGACCAACGTCTGTTAA
TGTATGCTTTAAGTGTTGCTCTGTGGCTTGGGAACCACCTTGTGAGCAGACGTTGCCAGAGTTGGCAAAGAAGGAACTGA
CTCCTAGTTGAACGTGGTCGAGCACACATTTGATGCCAGCACTCTGGCAGCAGAGGGAGGCAGGTCTATTTCAGGTTACA
TAGTGAGACCCTGTCTCAAGACAACAGAAATGACTCTTAAGGGGACTGTTACAGACACTGAAAGTGCCCTCCAGCGAAGC
CCTGCCTCACTCCCCTGCTGCCTTTCTTTTCTTTTTTTTTCTTTTTCCAAAACAGGGTTTCTCTGTGTAGCCCTGGCTGT
CCTGGAACTCACTCTGTAGACCAGGCTGGCCTCGAACTTAGAAATCCGCCTGCCTCTGCCTCCCGAGTGCTGGGATTAAA
GGCGTGTGCCACCACCACAGCCCAGCTTCTGCTGCCTTTCTTAAGTTACTCTCCTCACCTGTATGCAGAAGGGGCCATCC
AGTGCTACTGAGACAGCATTGGATACCATGGAGAGAGTCCAGCAGCAACCAAGGGTGGGTTGCTACCACCACTGCGTGCT
ATCCTGACCACTATGAATAGTCACATGCCCAGCTGCTGGCCCACAGAGGCTGGAGACAGATCTGCCCTGATGTGGTTCCT
GGCTCCAGGGCTAGCATGTGCTCCACAGCGGAAATGAGACTCCTCAAGGGAAGGGGTGGGGGGTGAGACTAAGAAGATGG
GATGTGACTGACCAAAGCTTGCTGGCCAATAGCCTCGGGGCCCCCTCTAGTCAGTCAGCTTCCTACCACACCCGAACTG
TAGTTAAGGGAGGCTTCAGCAAGCTTGGGCCCATGAGGGACTTAAGCATCAGCTCTGCCAGAGCTCATCTGAGCCCCTTG
GCATAGGGCTCTTTCCTGCCTTGGCCCTGCCTCTACTTGTTGATTCTCAAAGTTCTTGGACCAGCCCCTAAAGCTACCTT
GAAGTCAGTAGCTGAGTTGCTGCTATATGAACACATCTTCAGCTTCAGGTGGAAATGCCACTGCCATTGGCACACTTCTA
ATGGAGAGTGGGAATGAAGGTGGGAGGTAGGGGTGCCTCATTCACACCCAGTGGCTGCCTGAGGCAGCGCAGCCTGAGAG
AGAGAGAAAGGAGGTTTCAGACAACCCAGGAAAGTGATTTTTGAAGTGGGCTTGGAGCTTTAGGTAGGGCCACTCAGGGC
AGGTTCTAGTGAGGTTGAATGCCTGAAAGAGATTGGTAAGTCACAGGGGCTTTGAACACAAAGGGGAGAGGCAAGACCCT
GTGTTGGAGTCCAAAGCTAGTTTTCCGGTTTCTCGGTGCTGCCTCTGCCCCCTAGAGGCAGCCTGGGCACTACACATCC
CTGGCACATGCCCATCATGTGGCCACCCTCAGGAGGAGGGGCTCCAGGGATGAGTTAAAAGATTAAGAGAAAAGCTCCCT
TCTTCTCCAGAGTCCCGTCTACCCTGGCTTGGCGAGGGAAAGGAACCAGACATATATCAGAGGCAAGTAACCAAGAAGTC
TGGAGGTGTTGAGTTTAGGCATGTCTCTACCTGAGCCCACTTGGGAAGAGACAGGGAGAACAGCAGAAGGCTAAGCTACTT
CACACTGGCAGTGAGAGTCCATGAGCAGAGCTCAGGGTCCTCAGCAGGAAGTGTCTATGCCATGCCGAGGCTGGCCTGTC
CAGCCAGAAAGAACACAAGTGTAAAGGAAAATCGGAGCGTGCCTGCCTGTATGGAAAGAGGCCGGTCTGAGGGATGTCAGAGAC
CCCCGACAACAACATGAAGTGAATCAGAAGCTGGGGCGTGATACCACCTGGAGCTGCAGCCTCCACAGACCGCTTCCTAC
TTCCTGACAAGACTATGTCCACTCAGGAGCCCCAGAAGAGTCTTCTGGGTTCTCTCAACTCCAATGCCACCTCTCACCTT
GGACTGGCCACCAACCAGTCAGAGCCTTGGTGCCTGTATGTGTCCATCCCAGATGGCCTCTTCCTCAGCCTAGGGCTGGT
GAGTCTGGTGGAGAATGTGCTGGTTGTGATAGCCATCACCAAAAACCGCAACCTGCACTCGCCCATGTATTACTTCATCT
GCTGCCTGGCCCTGCTGAGAGTGGCTTTGGTGCAGCAGCTGGACAACCTCATTGACGTGCTCATCTGTGGCTCCATGGTGTCCAG
TCTCTGCTTCCTGGGCATCATTGCTATAGACCGCTACATCTCCATCTTCTATGCGCTGCGTTATCACAGCATCGTGACGC
TGCCCAGAGCACGACGGGCTGTCGTGGGCATCTGGATGGTCAGCATCGTCTCCAGCACCCTCTTTATCACCTACTACAAG
CACACAGCCGTTCTGCTCTGCCTCGTCACTTTCTTTCTAGCCATGCTGGCACTCATGGCGATTCTGTATGCCCACATGTT
CACGAGAGCGTGCCAGCACGCTCAGGGCATTGCCCAGCTCCACAAAAGGCGGCGGTCCATCCGCCAAGGCTTCTGCCTCA
AGGGTGCTGCCACCCTTACTATCCTTCTGGGGATTTTCTTCCTGTGCTGGGGCCCCCTTCTTCCTGCATCTCTTGCTCATC
GTCCTCTGCCCTCAGCACCCCACCTGCAGCTGCATCTTCAAGAACTTCAACCTCTTCCTCCTCCATCGTCCTCAGCTC
CACTGTTGACCCCCTCATCTATGCTTTCCGCAGCCAGGAGCTCCGCATGACACTCAAGGAGGTGCTGCTGTGCTCCTGGT
GATCAGAGGGCGCTGGGCAGAGGGTGACAGTGATATCCAGTGGCCTGCATCCTGTGAGACCACAGGTACTCATCCCTTCC
TGATCTCCATTTGTCTAAGGGTCGACAGGATGAGCTTTAAAATAGAAACCCAGAGTGCCTGGGGCCAGGAGAAAGGGTAA
CTGTGACTGCAGGGCTCACCCAGGGCAGCTACGGGAAGTGGAGGAGACAGGGATGGGAACTCTAGCCCTGAGCAAGGGTC
AGACCACAGGCTCCTGAAGAGCTTCACCTCTCCCCACCTACAAGGCAACTCCTGCTCAGCCCTTCAGCTCATTCAGCCTG
GGCTCTCCTGCTGGGACAGACATGAAGTCTCTAAGTTAAAAGAATGACAGACAAGCCGGGCGGTGGTGGCGCATGCCTTT
AATTCCAGCACTTGGGAGGCAGAGGCAGGTGGATTTCTGAGTTCGAGGCCAGCCTGGTCTTCAGAGTGAGTTCCAGTGAC
GGCCAGGGCTATACAGAGAAACCCTGTCTCAAAAAAAAAAAAAAAAAAAAAAAAAAAAAAAAAAAAAAATGACAAACAAGAG
CTAAGGGGGAGCAACTACTCTTGGTGGAAGCTTCTGATAAGAGGATTTGTGGCCATGAAAACCCAGCCATAGCCATGCAG
TAGGCAGCTTAAGGAAGTGAGACCCTTCCTCAGCACATGGAGCAGAATTGTAGAATGTGCATCTTGCAGGAGGGGCCTAC
CCATTTGCACAAGCGGTGTGGGACTGACCTAAAAATGGCTCCTGGGAATCCCAAGAGGTGGGCACCCAACTGCTCACACC
AGCAGGCTACTCTGTCAAGTGCCTCCTCCACCAGCTGCAGGGGAGGCTGTTGGCTTATCACTGACCACAGCCTGTGTGGC
TCTGAGCCAGGAAGCAGTTGAAATGCTAAGGTCAGAGGGAGGGAGCCTCCTTGCCATCTTCCCTAGCTCTCTCCAGAAGC
TGAGTGAAGTTTGGGTGAGAGGACAGGGAGAGTGGTCACATTAGGGAGATGGGAGTCTCAGAAGCCACAGCTAGCTGCCT
GCCTGATTTAGGAAACCACGGTCCCGTCTTGGCTTCCTCTATGACTACAAACTTCAGATCAGAGCAAGCAGCGGCAGAAA
AAGTTTCCTCGTGCCTCTATGGTGACAAGAATCCCTTTGTTCAGGCTCCTTCTGGTTCATCCATACACTGTAGCAGGACC
GTAGCATCTCAGCCCCTGATATGACACATCCGTAACCTTGGCCAGAGCCTGTCCCTTTCCTGCCCCCACCCCACCCCCCA
TGTGTAGGACTTTCAACCTACTTTGAGCTCTCCTGAGAAGCCCAGGGGTGGAGAGTCCCTGAGGCACACGTAGGGTGGAC
CAGGACCAGGGGCTGAGGTCTGTTTCTATGGGACACTGCCCTTCTGGGAGGAGGTGGGGTGGTCTCAGTGCTCTGTCAGA
TTTGGGAATTAGACAAGACCTTTCAGGCGTATCTGAGTCCCAGGAGAGTAGCACCTGGGGTGAACTAAGAAGCTTGCACA
TCCCTGAGCCTTTCTGCTGCTACAACCTGGGATTCCTGCAGCAGAGTCTATCTTGAGTCCCCACTGGTACTGAACACTTC
TTTGTCCTCCTCCTTGGGGTGGCCTCTCAGTCTGGAGTGAGATGCCCTCAACCATCAAGTCAGGAAATGGCACCAGGCCA
GGACCTAGCCACACTATCAGCTCCAGCCCTGCTGGACTGAGATTGGAAGCTGGTTCCCGGCAGGGCCAGCCTTTGCTGGA
```

```
AGACAGGACCATGAGTCAGCCTTTAGCCTGACACCATAGCTCCCTGGGGACACAGAGAGCTCTGGCCTGATGTCATCTGT
CAGAGAAGCATCCCAGGCCTCTGGATGCTTCTAAGCATCTTCTCTGTCCAGACAAGGAGACAGAGACAGATGCTCACCCA
ATACTTCTTGAGCCAGGCCAGCTGGAACCACAGAAAGCCACATCATGGACACATTGTGGGGGAGGGGCATCTCTTGAGAA
CAAAAGACCCATTTCTAAACTCGGCTAACTTGGATGCTTACCCTCAGAGAAAGTTGTATGCGCAGCCGCTCTATCCAGCG
TGCACCCGTGCGTGCAAACTGGACGAAGGCATCTGCAGGTGTTTTTAAGCTTTTTAGCGGCTCTTGTGAAATGAATGTTT
GTGGACCATTGAAGTGTGGTACTATTGTCTCAGATTCTTCCCGAAGAATTTTCTGTCACTGAAATATCAAATAGTCCTCA
TAAGGTGTCACTGGTCCTAGATGAATATTAAATCAGTGTTGTCTGTGAATTTGCATGCCCTCCTGATTCTGCTTCCCAAT
GGGCCCCCTCCACACACAGTCTCCTTACCTAGGTAGCTCCAGCTCACCTCGTGCCCCCTCTTCACCCTCACTTGTACCAG
AAGCACAAGGGTACCAATGACATCAGGCATGGCTCAGACAAAAAAAGACTGTTCGACATGCTCCACTGAGTGGGTTGTAT
ATCCTTAGAATTTTTCACAGAGATTTTCATGGAGCAGGGTACTTGCCAGATACTGTGTGTGCATCCTCACACCCCAGGT
ACATTTGCACCCTCGGCGCAGCCCTTTGTGGAGCCGCGGACTGGTAGCCCTCTCTTCTCCAGAAAATAGTGACCTCAGAG
TTCGGAGCTATAATCCGCCACCGTCCTGACTAGCGTCCCCAGGTCTGCACAGAGGTCTCAAGAAGGGTTTCGCTTCCCCT
GCCTCTCCCACCTCAAGCGACTCGGCGGACTCAGAGAATATGGGTCTTGATTCTTAAGCACTTGAGAGAAAGAAAGACCC
TCTTCACAAAGCTGCAGGGTGGGGATGGGGCAGGGACAAAATGGGGAGGCAGCTACTGGGGTGGGGCACTAAGGGGCCTG
GGTTCTATTGTCCCCAGAGCGGATTGCCCACCGGTGGCCCCTCCTCCGAAGGCGCGGCCAGACTTGCTCCGCATTGTGCG
GGCGGATCTCGGTGCCGGTGCTGATGCTGCAGAGCCTGCCGCATTGTGCGGCGCTCCAGCATTGGCCCGCAGCGCTTACA
TCAGCCAGCGAGGGCGGGGCAGCCTCTTATAAGAGGGCGCGGCGGAGGCTGCCGATCCTCCGCAGCCTGCTGTCCGCCTG
CCTTTTCGTCTCTAGCCGCGTGAAGTCAGCATGAGGGAGATCGTGCACATCCAGGCCGGCCAGTGCGGCAACCAGATAGG
GGCCAAGGTGAGGCTGAGCCGTCCGGCGTCCTGCGTCCCCACAAGCGCCAGCCCCAGGGCGGTGGGTGGAGGGAGAGAAA
GGCGACTCTGGGAGCCGCTAGCGGAACAAAGGGAAGAGCCCAGCCCCTTGCCCCGCTCCTGGGACCCCGGGTCCCCAAT
CGGTTCCCCTCAATCACGCCACTCTGCACTGCGCCCAGGTTGCAGGAGAAGGCTGGGGGTAACCCTTCTGACTCGCATTC
CCATCCATTCTGCGCACCCACACACACCATCAGGTACAGGGGATGTGGTTGGGGGGCGCCGACTGGGCGTGGCCTTCTTT
GAGGTTGCCACTTAAGCCTTGAGTAAGATGGGTGTGTCGGGCAACTTTGGGTGGGGCTCTCCCCTAAAACCATAATGACT
GTACATGCCTGGATCCCCACTGGAGAACTAGACAGTCTTCAGGGAGCAAGGCCGCCCTCATATCCCCAACCTTGTTCAGC
TCACCCAGTTTCCTTGCCCCACCCTGTTCCTTTGTTGGAGGAGTTCGGCCTGAACTCCAATGACCTTGATCCAGAACTGG
ACTCTGAAAATCTCCCATCTCTAACTGTGCGCTCCCAAGGCCTCCCAGTCGGGATTGAGCACAGAACAAAGCCGACTGCA
AGGATTGCCTCAATTTCCCCTCTATTGTTAGCCCGCATCCAGGTGTTGAAGCCAAGGCAAAAGAAAAAGAAAAAGAAAAA
GAAAAAAACGGGGGGGGGGGCGCGGGGTTGGTGCAGTGACTCCGCAATCCTACTATCATTAGTGAGGTAGCTGTCCTGTTT
CCAAGTCCTTAGGAGACTGTTCCTTACCCAAGGTCACACAGCAAGTGGCAGACAGAACTACTTGATTCCTGCAGAAGCCC
AACCTCCACTTGGGTTGCATTTGAGTGGGTGGGGTCAGCTACCTCAATCTTCTCCCTTCTGAACAACATGAAACGGGAGC
TTTGTCCAGATGAATGCTGGACGGGGCTTAGGGATGCTAGTTCCCAAAGGAGGAGGTTGGGTAGACAGGTCCTGGGAGGG
AGGGGACGAAGCAAAGAGTACCTCTGGAGGCTGTTGCCATAGAAACCGGGCAGGAACAAAAAAGCTAATTACCACGGGCT
GGGCCAGTCACGTGGCTGACATGTAAATTACAAGTTTGGCTCTTGGGTGGAGGTTCCTCTAGTTAAAGGGGAAGGGAAGC
GGCACCACAGAGACTCCCTAATCACAGGGGAGACAGCTCGCTTCTGCCCTACAAGGGAAATTTGGGCGGGGTTGGGGGGGT
GGCGGGTAGGGGGGCATACAGAGGAAGCCTAAGGCAAGGGGCCTCAGATCAAATCAGGACTCCTGGGATGATCGCTGACA
GCTGCCCTGGCCTGAAGGGAGGGTGAGGGCTCATTAATGTGGTCACAGGTGCCAGGCTTTCCCTGGACGAACATCTGCTC
CTGAAAGGGCCTGGGGTGAGTGGGGGGAGGGAAGGTTTGCCATCGGAAGGCCTGTTTGGTGAGCTTGCTGCATCTGTGCTG
TTCTGTGCTCTCCTCTGGGTCTCCTGTTGAGTAAGGGCCTAGAAGTCAGGTCCACCACGCCCAGGCTCCGTGTGTTGCGC
TATGGCCACAGAACATTTCTGAGTGCTGGACCCAGCTGAGGTCTCTTATGCTCCTCAGCACTGTGGCCCATAGGCTTGCC
CATTTTTTTTAATATTTAGGGTTTTTATATATATATATAATTACGAGTGTTTAAATTTTTAATGCCAATTCTTTTTAACA
ATGCAAATAATGAGTAAATTTTCTTCCATTTCCACCGGCTCCCTTCTTAGTAATCAGTACAAGGCGTTTTTGGCAGGTTC
CATCCAGCCCTCTGCCCTGGAAGATCTCTGAGACCAGACTGAAGGCCCAGGCCCTGTGGGACGTTACCTGCTTAGTGTCT
GCCCCAGGGAAAATAAACTTCCTTGACCAGATTTACTTCCTATTTTGGTAGCTGGCCCTTGCGTCACTGCTGCGAGCTAC
AATGGAGAAAGTTCTCCCTAGCCACCTGCAGCAACTGAGGGAGTGGGCACAAAGAGCTGCTTTTTTGCTTATCGGTCTTT
GGGTGGTCCCTGTCTTAGCACAAAGGACTGCTGGCTTCAGAGAGGTGACTGGGTAGAAGTATGGGCTGGGTGGGGACAGG
GAGAGAAGTGTGAAGCCAGGAGAATTTCGGGGAAGCAGCAGAAGGGGGGCCTTAGCAATTCATAAGTTTCTCACCCGCTG
TGAGCGTGTTAAAGCACCAGGCCCAGGCTGGTGCACTGTTACACCTTGTGAAGTGTGGCATGGGTGTCACCCTCCCTTAA
GGCTGAGAAGATGAGGGTTATGGAAGCTGAGTCACTCAGCTTCAGATCACACCATGGATTTGGGCAAGGCAGGAATTTT
AGTCTAGGTGTGTGCAGATGTGTCAAGGCAGGCGGTGACATGTGCCCCCACCAGCAGCAGATGTGACTGGTCTACCCTTT
GTGTGCTCCCTTTGTTAGGGCCTGAGCTCCTAGATGCATTCTCCTATAAAGGGGTGCCCTAGTAAGTGTCTTTACGGAGC
TTTTGTCTATGACTCCTGTCTGTGCCCCATCAGCTGCCAGAACACACCCACCCCAGTAGAAGTCTCTGAAGGGTGTGACC
CATTGGAGAGCCAATGGGCCCGGTGAGAAGGGTGCTGAGAGTTTTAGGGGAATTGAACATCTTACAGGGCTTTTGCTCCT
GTCTTTGGGAGCCACACCCAACTCTGCTTCTGTTCATGTCCCACTACCCTGCTGTCGTTGCGACCCTCGCCCCAAGTGG
GTCAGCACCTCGGACAGCAACCCCGCGGTTTGCAGACTGGAGCCTGGAGTTTCAATTGGGTTCACTGCACCGGGTGGTTT
TATTTTCTGTAGTGATGCTACTTCATTTATATAATTGTATTAATTTACATATTAAATAGTCTGCGGTAATATGTTCTTAT
AATGTCTTTTGTGTGCTATTCTATGTTAGTGTCTTAATGTGTAGCTCAGACTGGCCATGAACTCATGGTCCTCCAGCCTC
CACCTTCTAAGTACTGGTATTATTACAATTGTATTCTACTGGACTGGGAAGATTTTATTTTTGAATCATGTTTTATTATT
TGTAATTTATATGTTGATATGTTTCTCGTGTTAAAATCAAAACACCGGATAGTGGAGATGTGGCTAAAGCACGTGTTTAG
CATGTAAGAAGCCATTAGGTGCACACTCATAGTTCCAGTTACTGGAGAGGCTGAGGCAGGAGGATTGACAAGATTGCTTC
TTGATTCTAAAAAAAAAAAATAATGATAATAAAATAAAAGGAACACTGCAGATAAGGTATCCTACTATATATTAATTTTAA
AATGTATATATTTAATTTAAATAGTGTGTGTGTGTGTGTGTGTGTGTGTGTGTGTGTGTGTGTGTGTGTGTACATGCCTGGC
```

```
TCAAATACTGATCAGAAGAGGCTATCAGACCTCTGAAACTTACACACAGTTGTGAGCCACCATGTGGGCAGTGCTTGGAA
CTGAGCTTGAGTCTTCTATAACAACAGCCATGTGTCCAGCCCATGGTGCACACCACATTTGTGCACACACACATGCACAT
GCCCATACTCACCCAAACTCAGACACACTCACACATAATTGAAACTAAAATAAAAATTTTAAATTTATTTATTTTATGTA
TATGAGGACACTGTAGCTGTCTTCAGACACACCAGAAGAGGGCGTCAGATCCCATTATAGATGGTTGTAAGCCATCGTGT
GGTTGCTGGGAATTGAACTTAGGACCTCTGGAAGGGCAGTCAGTGTTCTTAACCGCTGAGCCATCTCTCTAACCCAAAAT
AAAAATTTAAAAATACACAAAAGAGATGAAATCCAGGGGCTGGTGAGATGGCTCAGTGGGTAAGAGCACCCGACTGCTCT
TCCGAAGGTCTAGAGTTCAAATCCCAGCAACCACATGGTGGCTCACAACCATCTGTAACAAGATCTGACTCCCTCTTCTG
GTGTGTCTGAAGACAGCTACAGTGTACTTACATATAATAATAAATAAATCTTTAAAAAAAAAAAAAAGAGAGATGAAATC
CAGACCCCTTTGATGAGGGATGGCTTCTGAGATAGGTCCCTCAGAAAGATCCCTGTATGTTCTAGAGTGATGATGATCTG
TTTGGACTATTTTTAGGCTTTTAAAGAATTTTATTTTTTTGAAAGATAATTCTATGAGTGTGGGTGTCTTGCCTGTGTGA
ACATCTATGCATACTATGTATGTACAAGGCCAGAACAGGAGTTGGATGCCCTGACCCTGGAGTCACAGATGATGGCTTGC
TGCCTTGGGAGATTTTGTGAGATTTGTGAACCAATCCTGGTCCTCTTCAAGAGCAAGAAGTGGTTTTTTTGTTTTGTCTG
CTTGTTTTTTATTTTAAATTTTATTTAATGTCTGAGTGCTCTGCTACATATACATCTGCCTGCCTGAAGAGGGCATCAAA
TCCCTCTATAGATGGCTGTGAGCCACCATGTGGTTGTTGCTGGGAATTGAACTCAGGACCTCTGGAAGAGTAGTCAGTGC
TCTTAAGCCTGCAACAAGTGTTTTGGGGTTTTGTTTGTTTGTTTGTTTGTTTGCTTGGTTTTTTTTGTTTTTTTTTTGT
TTTTTTTTTTTTTTTTTGAGACAGGGTTTCTCTGTGTAGCCCTGGCTGTCCTGGAACTCACTCTGTAGACCAGGCTGG
CCTCGAACTCAGAAATCTGCCTGCCTCTACCTCCTGAGTGCTGGAATCAAAGGCGTGCGCCACGACGCCCGGCTCAACAG
TGTTCTTAACTGATGAGCCATGTCACCAGCCCACTTAAAAGTATTTTTACTAATTAAAAATCCAATGGCACCAAAATGT
AGAAAAAAAATGTGAAAAGTCTTTCTGTCAGCACAAGCCGATGCCCCAGGACTGGCACTTGCTGACTGTTTTCTGTCCTG
CTTTCACCTCTGAGCTGTCTTCTCCACAAGACTGCCACAGAGAGGCCCTTTGTCTCTAGACCTTTGTATCAACAACTTAT
CTGCCAGTCACTCCCCTGCCTGACCAGTGATGAGGGATCTCAGAGCCCAAGGAGGCAGCCCCCCTCCCCCCCCCTCCCG
CAGGCAGGCTGGGTAGTCTGCATGTCCTGATAAAATGTCTTCCTGTGTGCAGCCCTTTCATCCCCTCCCTCCCTGGCCCC
CAGAACAGAAGGCTGTGTTCTCACAAGAGCCTGTTGTCATGCAGTCAGCCCATTGTACAGAGTGGGAACACCAAAACTAA
GGCCCTATGGGGCAACTATGCACCCTGCATCTCCGTACTGTCTTGCCCCTCTGAAATGATGGCAGCCCCACGTGAGGAGC
ATTGCGTTCTTGTGCAAGGGGCTGAGTGTAGCAGCCAGTGAGCCAACCATCCTGGGTGTTGCCAGGCTCCTGGATACGGT
GGCATGTGACCTCCGACAAGCCATTCTGCTGCTGGAAACTGTGGTCCTTCACAGATCCACCTGTGGGTCACACTGCCTGA
GGCTAATGAGCTATCAAGATGGACTCTGTCAGAGGCCATGACTTGCCCAGGCTCTGAGGGTCACATGTAACAGCTAGAGC
CCCATGCTGGACCTGATCTCTCTGCTTCCTGTGCTTCCAGCTGCTCTGGGCAGCTTTCTACCTTGTGTGTGCCTCGAGCT
GTGTAGAACCCACCTGTGTCTTGAGTCCTAGTCCATTCTAGGGACCTGGTTGTTAGTACCAAGACACTCCTAGCCCCGCC
TAACTATGTGACCCTTGAGCTGCATTGCCAGGCCAATGCCCCTCAATCCCACAATCCACTTGGCTCAATTCCCACCCTCA
CTGGTGTGACGTCATTTTCCCTATAAATTAGGAGATCACCCCCTCCTCTCTCTCTCTTTTTCTCTCTCTTTCTTCTCTCC
CCCCTCTTTCTCTCTTTTTCCTCTTCTCTGCCCTTCTACCTGCTTCGCCACCCCTCTGCCTCTGCACAATAAACCTCTCC
CACGTGGAAACACCTTAGCCTGGTCTGCTGTTTGGTTTATCTGCTCAAAATATATATGTATTAATATTGCTTGATATTAA
TTAATTCATAAAACTGGTAATGTCCTCCAGATAGTGCAGAGAACAGGTATGCATATTTACTCTTTTCTAAAGCCTTATGT
TAGATCTAGAAAGATCTCAGAGTTTTTTAATTTTACACTGAGAGCTTTAAGACTTCAGAAGAAGCCGGGCAGTGGTGGCG
CACGCCTTTAATCCCAGCACTCAGGAGGCAGAGGTGGGCGGATTTCTGAGTTCGAGGCCAGCCTGGTCTACAGAGTGAGT
CCCAGGACAACCAGGGCTACACAGAGAAACCCTGTCTCGGAAACCCAGACCTCATGAGAGCACAGGTTCGGTAGAGCGTC
GATGTGTGATGAGCTCTTTCTTTCCTTCTGTCCTCAGTTCTGGGAGGTCATCAGCGATGAGCACGGCATAGACCCCAGCG
GCAACTATGTAGGGGACTCAGACCTGCAGCTGGAGCGCATCAGCGTATACTACAATGAGGCCTCCTGTGAGTACCGCTCC
ACACCCACCCTGACCCCGGCAAAGAGGGCTCCCATGTGAATAGAGTCCTAGCGTCCATATCCCCACCTTTCTTGATTTAC
CAAGAGGCTGGCGGGTATGACTGGAAACGAGGCATCCAAATTGACAGGATAAAGAGTGGTTACCGGGCTGGAGAGATGGC
TCAGTGCTTAAGAGTGGTTACTATGGTGTGAGATGAAAATATATTTAATGGTGGTGATGACCTTATGTAGTCTAGGGACA
GGCAGGAGACTCAATGATACATCCTTAGCAGGGGTCACTGGGGGCTTGATTGAGGCTACTTGATAATTGATACAGTCTTT
TAATTTTAATTAGATATTTTCTTTATTTACATTTCAAATGTCATCCCCTTTCCTGGTTTCCCCTCCAAACACCCCGTACA
TCCCCCCTCCTCCCCCTGCTCACCAACCCACCCACTCCCGATTCCTGGCCCTGGCATTCCCCTACACTGGGGCAGAGAGC
CTTCACGGACCAAGGGCCTCTCCTCCCATTGATGATTGACTAGGCCGTCCTAATTGGTCTT
```

MOUSE mRNA SEQUENCE : mR7-161.1 (Seq ID No: 96)

```
TTCCTGACAAGACTATGTCCACTCAGGAGCCCCAGAAGAGTCTTCTGGGTTCTCTCAACTCCAATGCCACCTCTCACCTT
GGACTGGCCACCAACCAGTCAGAGCCTTGGTGCCTGTATGTGTCCATCCCAGATGGCCTCTTCCTCAGCCTAGGGCTGGT
GAGTCTGGTGGAGAATGTGCTGGTTGTGATAGCCATCACCAAAAACCGCAACCTGCACTCGCCCATGTATTACTTCATCT
GCTGCCTGGCCCTGTCTGACCTGATGGTAAGTGTCAGCATCGTGCTGGAGACTACTATCATCCTGCTGCTGGAGGCGGGC
ATCCTGGTGGCCAGAGTGGCTTTGGTGCAGCAGCTGGACAACCTCATTGACGTGCTCATCTGTGGCTCCATGGTGTCCAG
TCTCTGCTTCCTGGGCATCATTGCTATAGACCGCTACATCTCCATCTTCTATGCGCTGCGTTATCACAGCATCGTGACGC
TGCCCAGAGCACGACGGGCTGTCGTGGGCATCTGGATGGTCAGCATCGTCTCCAGCACCCTCTTTATCACCTACTACAAG
CACACAGCCGTTCTGCTCTGCCTCGTCACTTTCTTTCTAGCCATGCTGGCACTCATGGCGATTCTGTATGCCCACATGTT
CACGAGAGCGTGCCAGCACGCTCAGGGCATTGCCCAGCTCCACAAAAGGCGGCGGTCCATCCGCCAAGGCTTCTGCCTCA
AGGGTGCTGCCACCCTTACTATCCTTCTGGGGATTTTCTTCCTGTGCTGGGGCCCCTTCTTCCTGCATCTCTTGCTCATC
GTCCTCTGCCCTCAGCACCCCACCTGCAGCTGCATCTTCAAGAACTTCAACCTCTTCCTCCTCATCGTCCTCAGCTC
CACTGTTGACCCCCTCATCTATGCTTTCCGCAGCCAGGAGCTCCGCATGACACTCAAGGAGGTGCTGCTGTGCTCCTGGT
GATCAGAGGGCGCTGGGCAGAGGGTGACAGTGATATCCAGTGGCCTGCATCCTGTGAGACCACAGGTACTCATCCCTTCC
```

TGATCTCCATTTGTCTAAGGGTCGACAGGATGAGCTTTAAAATAGAAACCCAGAGTGCCTGGGGCCAGGAGAAAGGGTAA
CTGTGACTGCAGGGCTCACCCAGGGCAGCTACGGGAAGTGGAGGAGACAGGGATGGGAACTCTAGCCCTGAGCAAGGGTC
AGACCACAGGCTCCTGAAGAGCTTCACCTCTCCCCACCTACAAGGCAACTCCTGCTCAGCC

MOUSE PROTEIN SEQUENCE : mP7-161.1 (Seq ID No: 97)
PDKTMSTQEPQKSLLGSLNSNATSHLGLATNQSEPWCLYVSIPDGLFLSLGLVSLVENVLVVIAITKNRNLHSPMYYFIC
CLALSDLMVSVSIVLETTIILLLEAGILVARVALVQQLDNLIDVLICGSMVSSLCFLGIIAIDRYISIFYALRYHSIVTL
PRARRAVVGIWMVSIVSSTLFITYYKHTAVLLCLVTFFLAMLALMAILYAHMFTRACQHAQGIAQLHKRRRSIRQGFCLK
GAATLTILLGIFFLCWGPFFLHLLLIVLCPQHPTCSCIFKNFNLFLLLIVLSSTVDPLIYAFRSQELRMTLKEVLLCSW*

MOUSE PANTHER CLASSIFICATIONS
       FAMILY (SUBFAMILY)
              CF11294(MELANOCORTIN-1 RECEPTOR)
       BIOLOGICAL PROCESS
              Signal transduction(2.11.00.00.00) > Intracellular signaling cascade(2.11.02.00.00) > NF-kappaB cascade(2.11.02.04.00)
              Signal transduction(2.11.00.00.00) > Cell surface receptor mediated signal transduction(2.11.01.00.00) > G-protein mediated signaling(2.11.01.07.00)
              Immunity and defense(2.16.00.00.00) > Macrophage-mediated immunity(2.16.05.00.00)
              Immunity and defense(2.16.00.00.00) > Granulocyte-mediated immunity(2.16.04.00.00)
              Developmental processes(2.23.00.00.00) > Ectoderm development(2.23.08.00.00)
       MOLECULAR FUNCTIONS
              Receptor(1.01.00.00.00) > G-protein coupled receptor(1.01.01.00.00)

MOUSE GENE ONTOLOGY
       BIOLOGICAL PROCESS
              G protein linked receptor protein signaling pathway > G protein signaling, linked to cyclic nucleotide second messenger
              cell surface receptor linked signal transduction > G protein linked receptor protein signaling pathway
              intracellular signaling cascade > protein kinase cascade
              G protein signaling, linked to cyclic nucleotide second messenger > G protein signaling, linked to cAMP nucleotide second messenger
              behavior > feeding behavior
       MOLECULAR FUNCTION
              ligand binding or carrier > lipid binding
              amine oxidase > amine oxidase (flavin-containing)
              monooxygenase > monophenol monooxygenase
              enzyme > guanylate kinase
              enzyme > 2-acetyl-1-alkylglycerophosphocholine esterase
       CELL COMPONENT
              cell > membrane fraction
              plasma membrane > integral plasma membrane protein
              cell > plasma membrane
              cytoplasm > endosome
              cytoplasm > lysosome
MOUSE PROTEIN DOMAINS (INTERPRO SIGNATURES)
              IPR000276 (G PROTEIN RECEP F1 2)
              IPR000276 (7tm 1)
              IPR001671 (MCRFAMILY)
              IPR001908 (MELNOCORTINR)
              IPR000276 (GPCRRHODOPSN)
              IPR000761 (MELNOCYTESHR)
              IPR000276 (G PROTEIN RECEP F1 1)

HUMAN GENOMIC SEQUENCE : hD7-161 (Seq ID No: 98)
AGCTCCCACCTTCCAGCTCCCAGCTCCCAGCTCCCGCCTCCCGCCTCCCAGCTCCCGCCTCCCGCCTCCCAGCTCCCAGC
TCCTGCCTCCCACCTGCCAGCTCCCAGCTCCCGCCTCCCTCCTCCCAGTTTCCACCTCCCAGCTCCCACCTCGCAGCTCC

```
CAGCTCCCAGCTCCCACCTCGCAGCTCCCAGCTCTCTCCTGCAGCTCTGAGGTCAGGGCTTTGGGGCTCACAGGGACTGG
CTGCCTCTTTCAGACCCAGATGCTGAGAGATGGGGCTCAGGGCTAAGTGGGTCTTTCCCCTCACAGGAAGGGAGAGGAGG
AGAGGAACAGTGTTATTTGGGGGTCACAGCTCTGCGTTTGCAGGCCACGTTCTTTCCCATCTCCACCGAATGTACAAGCG
CTTGCGTGTCGGCCTCTGTGGCAGGTGCGAGTGACACAGTGGAAGCAGGTGTGCAGTGAAGTGGGGAAAACATATTAATT
TTAGAATGTAGAAATAAACATAAAATCAAAACTGCACCAGCCTGGCCAACATGGCAAACCCCGTCTCAACTAAAAATACA
AAACTTAGCCAGCTGGGTGCAGTGACTCACGCCTGTAATTCCAGCAGTTTGAGAGGCCAAGGTGGGCAAATCACAAGGTC
AAGAGATTGAGACCAGCCTGGCCAACGTGCTGAAAACCCGTCTCTACTAAAATACAAAAAAATTAGCTGGGCGTGGTGCC
GCACACCTGTAGTCCCAGCTACTTGGGAGGCTGAGGCAGGAAAATTGTTTGAACCCGGGAGGCACAGGTTGCAGTGAGTG
GAGATCGTGCCACTGCACTCCATCCTGGCGACAGAGGAAGACTCCGTCTCAAAAAAAAAAAAAAAAAAAAAAATTGGGCG
TGGTGGGTTGTGCCTGTAGTCCCAGCTACTCAGGAGGCTGAGGCAGGAGAATCCCTTGAACCCGGAAGGAGGGGGTTGCC
GTGAGCTGAGATCACGCCACTGCACTCCAGCCTGGGTGACAGAGTGACTCCATCTCAAAAAAAAAAAAAAAAAAAAATCCT
CCAGTAAGGTGTTTGGAGGAAGGATGGTGGCTGTGCTGAGACGGAGAGGATGAATGTGCCCTCACCAGGCTCAGGGGAGA
GGGTCCTTGTGTTCCCCAGCAGAGTCACGATGTGCCGAGGCCCTGTGCCAGGAGCAGAGCACACGGCACATTGAGAGAAC
AAAAGCCCCATGGCCAGACCGGGAGAGCAGGGCTGCGGCGCAAGAGGGCCAACTGCCAGGCGGCCGGTGTGTCTGGCTTT
CAGAATCCGGGTCATTGAAAGGCTTTTTACCTTTTCCTTCTGGGCTGGAAAGTACTTGCCTTACCCAGGCCCCGTCACCC
GCACAGCGGTCGTCGTGATTATCCCTGCCGGCAGGGTGTTGCAGCATTGAGGAATCGCGGTTCTTTGCTGCCTCCCTCCG
CTGTGGAGCCCAGGCTTTCGCCCTCCCTAGAGCTCCTGAGGCCTGGCCGCTCAGCGTCCTGTAGAGCCGGGCCGGTAGCC
CTTCGCACCGTGAGGGTCTCCGCTTAACGCAGGCCCTGGAAGCTTCTGCCTTTGCGTAAGGAGTGGGTGCTGAAGGAGAC
GTTGAAACTGCAGCTTTTCTGAAAAGCAAGGCACCTGTGCAGTGGCCGAGGATGGCTGTGGGAGCCTCGCTCCGGACGCC
ACGCCTGCCTCAGCTGAGTTGGTCTCCCTCATCCTGTTCCCGCTGTCCACAGCCCCACCCTAAGCTGATGAGGAAGCCTC
ACGGGCCACCCGGGGAATCCAGGGCCTGTGCTCAGCTGCTGCTGCTCTGCCCCCTGGCGGCCCCTCCGTGTCCCTGCGAG
GGAGGCAGCTGGCAGGCAGCCCCGTGAGCCGGGTTGGGTCCCACGAGTCTCAGTTCAGTGTCGTTTCTGACTTTTCTTGA
CTTTCTTTCAGGCTAAGTCCTATCAGCCATGGAAACACCATTGCTCTCTTCTTCCGGTCACTGTTGCCAAACTATACCAT
GGAGGTAGGTTGAGCTCGTCCCAGCCCCTGCCTCCCCAGTGGGTGCGGGCATATCCATTCCAGTGCGAATGCCTGGAGCC
TCCTTGAACCAGGACTGTGCCAGCCGGCACAGGGAGCAGGGAAGGACCAAGGCTGCATTGTGCCAGTGGGGTGCCCATTC
TGCCGGGGAAACTGGGGCCTGCCCAGATGGTAGCAGATGCAGGCCGTGAGTCAGCCGGCTTGATCCCACTCTGTGCCTG
GCTTGGTCTAGACACGGCATTTGGTTTTGAGAGCTTCCTCCTCACAGGGACCTGGGGAGGGTTCAGGGTCCGGTGCTGGG
GCAAGGGCCACCTCGGGCTCCACAGTGCCTCCTGCTTGGGCCCCGGGCCCCTAGTCCCAGCACAGACAGCCCCCTTCTCA
GACATGTCCCCTTGCTGCAGGGGGAGAGGCCCGAGGAAGGAGTGGCTGGGGGTCTGAACCGCAACCAGGGCCTGAACAGG
CTGATGCTGGCTGTGCGCGACATGATGGCCAACTTCCACCTCAACGACCTGGAGGCGCCGCACGAGGACGACGCTGAGGG
GGAGGGGGAGTGGGACTGAGCGTCCGCAGAGGTGACCGAAAAGCCGTATGATGATGTTCCCGATTTCTCTGTTGGTCGGA
GTCGGCCAGTTGCCTGAAGTAGGGAAGCTGAGTGTGTCGCTCCCTGGTCCACTGTTTCTCCTATAAATGTAAATGGGTCA
CGCTCTGCCGTCCGCACCTTCTCCTTTCGCAGGCACTGAGCGCCGTCCCATGCCCTCGGATTCGAATCACACTCAGTCCA
TTCTCCTCCCAGTGGCTTGTGGGTGAGGTGGGGCCCAGACCCCATGCTTCCTGTGCTCCCAGGATCTGGGCACGGGTGGA
GAAGGGGCCCCACGGGGTCTTCAGTGCAGGTCCCTGCTGAATGCCAAGGCCCTGGGTGCACGTCCTCCTTGAACTTGCTG
TGGAGTTCGTCAACAGAGCTGCGTGCCTTGTTGGTAGAGGGTCTCAGGGCAGGGCTGGAGCAGAGAGGCCCCTATGTGGC
AGCCGGCCTTCAGTCTTGTTCCTCCAAGCAAGTGGGGTGGAGATGTTGGGGTCCTTTGGCTGGATGCCACTATCTGTTGA
ATCTGACAAAATAATTTTCTTTTTTTTTTTTGAGACAAGAGTCTCACTGTGTCGCGCAGGCTGGAGTGCAGTGGCGCAGT
CTCGGCTCACTGCAAGCTCCGCCTCCCGGGTTCACGCCATTCTCCTGCCTCAGCCTCCCGAGTAGCTGGGGCTACAGGCA
CCCGCCACCACGCCTGGCTAATGTTTGTATTTTTAGTAGAGACGGGGTTCCACCATGTTGGCCAGGATGGTCTCAATCTC
TTGACCTCATGATCCGCCCGCCTTGGCCCCCCACAGTGCTGGGGTTACAGCTGTGAGCCACCGCGCCTGGCTCTGCCAAA
ATAATTTTATTAGAGAACTTGAGGGCAGTGCCCAGAATTGAAGGCGAAGCCCCAGAAGCATGTTTTGCAGAGAAGTGCCC
AGGGAAGCTCTGAGGGCCCATGTAGCAAAGATCAGGGGATAGTCGGTCTGAGGGTGAATGGGCACTCGGACCAAGACCC
CAGTCTTGGGGGAGGGCTTAGCTGGAGCAGGTCCTGGCACAGTTGACTGATGGTGCACAGAACCCGTGCATCCCACGGCC
CCACGGTGCTGCAGCTGCAGGAGGGCGGAGGCTGCAGCCAGACAGCATCAGAAGCCAGCGTGGTTCTGGAAGGATCGAG
AACACCAAGGTGTTAGGGCTGCAGCAGGGGCTCTAGGGCGGCCAGTGAGGCAGGAAACATGTTCCAGCCCCAGCTAGGTACTGGT
CCGTGGACCCACCTCCCAGAAAGCCCATCACTGTGTAATCGTCTAACCTGGGGCTCGCCGAGGCCTGTGAGTTCATCCTT
TTGGCAGTTCCTGGTGTCTCCTTACTCTGCTCAGCATTTCCTGGGCGGGAGCTTAGGGTGCAGGACCCTCCCCAGGACGA
CGAGGGCCCAGTGTCCATGACAAGAGTTGGCCCGAGGGCTGAGCCACGTGTGCCCATCTCAGACGTGGGCCTGAGGGTGC
AGCCCTGGCCCTGTGCTGGCCATTTCTAGGAGCGGTGCCCTGAGGTCCCAGCTGTGATAGCCCCACGCTCTGCAGGAAGA
GATCATGGGGGCGGGGAGTTGGTGCTGCGGCCTCGTTCCTCTCTGCAGTGAGTGAACGATGTTTGTGGTCAGCAGGAGCC
TGTGGGGAGCACAGGCTGGTCCTCCTGGTGTCCCACCCACCCCTTTTTCCATGGGGGATCTGCACTCATCTCCAGGGAAG
ATGGTTGGGAGATAACCCCAGTCTGCTCTAGGTCCCCACCCTCCACAGCCAGGGTGGTCCGTGGTGAGCTTCAGCCATCG
AGATGCGGGAGTCTGCTAGAGTCTTCAGGGTCTTTTCTCTGAAAATGACAGGCTAGCAAGGAGACCTGGGTCCCCTGCCT
CTTCCATTCCAGATGCCTTGAGTCCACCCAAATAGGGGATGTGATGTTTGGAGCTGCAGCAGCCGCCCTACGGTTGGGAG
TCAGAGAAGAGCCGGTGTTCCAGGGACAATGCAGCAGAGGCTGAGCCCAGGCCTGCTGTCCTGAGAGGTGGCTGGATCAC
TGACACTTTGGCAGTGGTGCTGGGGTTTATGTCATGACCTGCAGCTGAGCCTACTTCCAATGACCGTGAGATCTGAAAGA
CTGTTTTGAGGGCGTAGCCTCTGCCATGATTGTGGGGAATGCTGTCCTGTTTCCTCCCTTGGCCCTGCTCAGCCCAGCGA
GAGGCTGAGGCGCACGTGGCTCCCCGGGTGCCCACAGGCAGCGTGGCTCACCAGCCGGGCCCTTTTCCACTGAGCCAGAA
CCCCCCAAAGCCTTCAATGCAGGCACCACGGTGAGCCCACGAGAAACCCTGCTTGCCACCTCCCACACCCCCACCCCCAA
```

GTTCAAAGGAAATGGTCCCTGAACCAAGGGCTGAGATCAGCTGTGGGTCCAGCTGTCCTGGGGAGCTGTACTGGAGCCCA
CCACGGTGGGGACTGTTGGTCCGGCGGTGACCCCCACCTCCATGTCTGTGGCCGCAGCTGGACAGGCCACTCCCTGGGCCA
CAGAGATGTTTTACCTCTCGCAGCCCTCGGGCACACATTGAGCAGATGTGTGTGTGTGTGCGTGTGTGGGTGGGTGCGCA
TTTGTGTGTGCCTGTGTGTGTGCGCATGTGGTGTGGGTGCACGTGTGTGCACGTGTGTGGGTAAACATTGTGTGTGCGCA
TACGTGTGTGGGTAAACATTTGTATGTGCACGCGTGTGTGGGTAAACGTGTGTGCGCACGTATGTATGTGTGTACATTTG
TATGTGTGTACATTTGTATGCATGTGTGCCTGTGTGTGTGTGCCTGTGTGTGTGGGTGCACATTTGTGTGTGTGTGTGTG
CCTGTGTGTGTGTGTGCACGTATGTATGTGTGCACACTTGTATGCATGTGTGCCTGTGTGTGTGGGTGCACATTTGTGTG
TGTGTGCCTGTGTGTGTGGGTGCACATTTGTGTGTGTGGGTGCACATTTGTGTGTGTGTGCGCCTGTGTGGGTGCACATT
TGTGTGTGTGTGTGCCTGTGTGTGTGCCTGTGTGTGTGGGTGCCTGTGTGTGTGGGGCACATTTGTGTGTGTGTGTGCCT
GTGTGTGGGTGCACATTTGTGTGTGTGCCTCTGTGTGTGTGCCTGTGTGTGGGGGTGCACATTTGTGTGTGCGCCTGTGT
GTGGGGGTGCACATTTGTGTGTGCGCCTGTGTGTGTGGGTGCACATTTGTGTGTGTGTGCGCCTGTGTGTGTGGGTGCCT
GTGTGTGTGTGGGGCACATTTGTGTGTGTGTGTGTGCCTGTGTGTGGGTGCACATTTGTGTGTGTGCCTGTGTGTGTGTG
CCTGTGTGTGGGGGTGCACATTTGTGTGTGTGTGTGCCTGTGTGTGGGGGTGCACATTTGTGTGTGTGTGCCTGTGTGTG
TGTGGGTGCACATTTGTGTGTGTGTGTGCCTGTGTGTGTGGGTGCACATTTGTGTGTGTGTGCCTGTGTGTGTGGGTGCA
CATTTGTGTGTGTGTGTGCCTGTGTGTGTGGGTGCACATTTGTGTGTGTGCCTGTGTGTGGGTGCACATTTGTGTGTGTG
TGCCTGTGTGTGTGTGCCTGTGTGTGTGGGTGCACATTTGTGTGTGTGTGTGCCTGTGTGTGTGGGTGCACATTTGTGTG
TGTGTGTGTGCCTGTGTGTGTGTTGCAGGCCCTGGATGCCAGACACTGAATAAACGCAGGAAGACGTCTGTCTTCATTCT
CCTCGTGGGTCGCTGGTCCAGAAACACCTGGATGGAAAGTGCTCTGCAGGAACGGTGCCTCTGCCTGTGGCGGGGACCCT
GGTGAGCGGATGGGCCAGCCCCACGTGTCTTCCGGCCACTCAGCATGCAGTGTTTCCAGGGGCACTAAGAGACCAAAATC
GAGATATGATTAGCTGTAGGATGTCATCTAATCACAGATCATCCCGAGGCTAATTTATCTCCCCCATGACCATAACACAT
CAAAAAGTTGACTTTTTGCAGCTCGGCTGTGCCTCATCTTCCCACGAAGCCCCGACAGGCACATCCAGTGAGGAACCACA
GTGGGAGTCCTGTGGCAGGGTCACCCCACTTCCGATGCCCTCCAGCTGCATCTTGGCACGAAAAAGGCTGCCCAGTTCTC
ATGCCCTTTCAAGTCCCGGGCTGGGGGGGTAGCAGTGGAGGCTGGGGTTAACCGCCGTCCGTCTCAAAGGCCTCATTGTGG
AGCTGCAAACACGAACGTCCTTGAAATGTGAGGGGACAGTGCTCTGGGCAGGGGCTGCTCACTGGTTCATGGCCAGAGG
TGAGCGGGCTCTGGGTCTGGGTCTGTGAGGTGCTGTGGACGTCGGGGGTGCTCCTGGGGCAGGGACACAGCCACGGCCCT
CACACCAGTGGAGCCGTCTTCCTTCCCGAGGCAGAGGCTTGGCCTTCTCACACCTTGGGACCCTTCTCTCCCCTGTGCTC
ATGCTGGAAGCACAGCGTGAGGGGGCAAAGGTCATGGGAGGCAAAAGGCTGGGCTGAGGCCGAGGCTCTGTGGCTGTGGC
CGGATACCAGGTCCTGTGGTGGTGTGGGCAGGGCGCGCTCTCTCCTCTGAGCAGCCTGGGGCTGCGTGTGTGAACAGAAA
CAGGCCTGCCGATTTGAAGATGGTTTGACCAAGTCTCCCAAAGCTGAAGAGAGGTCCCTGCCCATGTCCCCGGGTGGCA
ATCCACAGACGCACCTGTGTCCATCAGACGGTATCGTTGATAGGGAGTCTGAAGCCACCCAGACGTGGTCTGTTCACGCC
GTGGACACCACGGTCTGAGGCAGACGGTTACAGCCACCGGCATAGGCGAATCTCAGCCAGAGAGAGTGAACTGGGACACC
ATTCACGTGATGGGCCAGATGGGCTGGCAGCGGCTCGCTGGAGCCGTCCGGGTGGGAACGCTGTGTCTGTTGGTCTGGTG
CTGCTTATGTGGCTGGTTCAGGTCTGTCATCCGTCAACCTGCATATTTATCACTGGTGCATTTTCATGTATGTTGTACCT
CAATTAAAACATTTTAGGGCCGGGCGCGGTGGCTCACGCCTGTAATCCCAGCACTGTGGGAGGCGGAGGCGGGAGGATCA
TGAGGTCAGGAGATCAAGACCGTCCTGGCCAACATGGTGAAACCCCATCTCTACTAAAAAAAATACAAAAATTTGCCGGG
TGTGGCGGCACGCGCCTGTAGTTCCAGCTACTCAGGAGGCTGAGGTATGAGAATTACTTGAACCCAGTAGGCAGAGGCTA
CCGTGAGCCAAGATCATGCCACTGCACTCCAGCCTGGGTGACAGTGAGACTCTGTCTCAAAAAAAAAAAAAAAAAAAAAAT
TAAAGCAGATGGGGTGTGGGGGCTCATCCCTGTAATCTCAGCATTTTGGGGAGGCTGAGGCGGGACCGATCACCTGAGGTCA
GGAGTTCAAGACCAGCCTGGTCAACATGGTGAAACCTCATCTCTACAAAAAATACAAAAAATAGCCAGGTGTGGTAGTGG
GTGCCGTAGTCCCAGCTAGTTGGGAGGCTGAGGCATAAGTATTGCTTGAACCCAGAAGGTGGAGGTTGCAGTGAGNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNATATATTTTTTTTTTTTTGAGACTGT
ATCTCTGTTTCCCAGGCTGGAGTGCAGTGGTGTGATCTCGGCTCACTGCAACCTCCGCCTCCTGGGTTCAAGCAATTCTG
CCTCAGCCTCCCAAGTAGCTGGGATTATAGGCGTGCGCCACCACGCCTGGCTGATTTTTGTATTTTTAGTAGAGATGGAG
TTTCACCATATTACCCAAGCTGGTCTTGAACTCCTGACCTCATGATCCGCCTGCCTCAGCCTCCCAAAGTGCTGAGATTA
TAGGGGTGAGCCACTGTGCCCGGCAATAATAATTTTTTTTTTTTTTGAGACAGAGTTTTGCTCTTGTTGCCCAGGCTGGA
GTGCAATGGTGCAATCTCGGCTCACGGCAACCTCCGCCTCTCATGTTCAAGCTATTATCCTGACTCAGCTGGGATTACAG
GCACCCACCACCACACCTGGCTGATTTTGGTATTTTTAGTAGAGACGGGGTTTCTCCATGTCGGCCAGGCTGGTCTCGAA
CTCCTGACCTCAGGTGATCCACCCGCCTTGGGCTCCCAAAGTGCTGGGATTACAGCCCTGAGCCACCATGGCTGACAATA
ATAAAAAATTTTAAAGCAAAACTAAAGATTTATAAATTGTAAAATGCGGTTGTAACAAGTATTTACTGTACCAGTAAATA
TGAAAAATATTAATGTCCTCACACACATGGAGGTGGCTTGTGAGTGGTGTGAGCATTTCATGGTCAGAGTTTGGCAGTTG
GGCGAGAGACAGGATGGGATTTTAACTCGAACATCGCAGACACAAAACTGAAAACCCTGATGCGGTGCCTGCACCGTTGCC
TCCTTTCCTGCAGCTGTAAATGGGACGAGTAATTCCATCCCCCTCCCGCTTCCACCCTTCAGCACAGACGCAGTCTTCAG
CAAGGAAGTGCTGGGAACGCCCTGGAGTGAACCCAGGAAGATGCCTGCAGTGGGTGCCAGGGCCCCTCTCCACCGTCCCT
GCTGGGCTTCGGGGCCCACGCCCGACTGCTGTGAACGGCCTGCGGAGCACCACGTGCGACGGCTGGAGGCGAGAGGTCTGC
CTTTGATGTGGCTGTTGGTGCAGGGCCTGTGGTGCCTTCCGCAGCGGAAATGGCGCCGCCCAGGGGAGGGCGGGAGCAG
CGTCCCGGGTGCCCCTGTGAGGATGAGCGACGAGATGACTGGAGGGTCCCTGAAGACCTCACTAGGGTGCCCCCAGCCGG
TCCGCTCCCAGGAAGCGACACCCCCACAGCCCCAGGGCTGCAGCTGAGGGGGTCGCCACTCTGGCTGGGCGAGGCTGGGC
CCTTGGGGGCAGGCGCCAGAGTGGCCTCAGGCTCTACAAGATGCCTGAAAACACCAACCTCTCCAGGGCTCACTAGCATT
GGACGCTTTCACGCTCTGCCCTGGCCGGAAGCCCCCTCACCCCGCGCGATGTGCAAACTCCTGCAGGGCTCACTCAGTTT
CCAGAACTTTAATTATTGGAAAGTTCTCCCTGGTCCAGCCCCCAAATCTGCCGTGAACGTTGACAGCTGAGTTGCTGCTC

```
CATGCGTGCTTTGGCTGAGAGCAGAGGGGACCCCTGTCCTCCCTGAGCTGCTGACGAGGGGAGGGGTGAAGGGTGGGGCC
TCTGGAGAGGGCAGGTCCCGGGGAAGCTCCGGACTCCTAGAGGGGCGGCCAGGTGGGGGCCCTGGTGACCAGGACAGACT
GTGGTGTTTTTTAACGTAAAGGAGATCCGCGGTGTGAGGGACCCCTGGGTCCTGCACGCCGCCTGGTGGCAGGCCGGGC
CATGGTGGGTGCTCACGCCCCCGGCATGTGGCCGCCCTCAGTGGGAGGGGCTCTGAGAACGACTTTTTAAAACGCAGAGA
AAAGCTCCATTCTTCCCAGGACCTCAGCGCAGCCCTGGCCCAGGAAGGCAGGAGACAGAGGCCAGGACGGTCCAGAGGTG
TCGAAATGTCCTGGGGACCTGAGCAGCAGCCACCAGGGAAGAGGCAGGGAGGGAGCTGAGGACCAGGCTTGGTTGTGAGA
ATCCCTGAGCCCAGGCGGTAGATGCCAGGAGGTGTCTGGACTGGCTGGGCCATGCCTGGGCTGACCTGTCCAGCCAGGGA
GAGGGTGTGAGGGCAGATCTGGGGGTGCCCAGATGGAAGGAGGCAGGCATGGGGGACACCCAAGGCCCCCTGGCAGCACC
ATGAACTAAGCAGGACACCTGGAGGGGAAGAACTGTGGGGACCTGGAGGCCTCCAACGACTCCTTCCTGCTTCCTGGACA
GGACTATGGCTGTGCAGGGATCCCAGAGAAGACTTCTGGGCTCCCTCAACTCCACCCCCACAGCCATCCCCCAGCTGGGG
CTGGCTGCCAACCAGACAGGAGCCCGGTGCCTGGAGGTGTCCATCTCTGACGGGCTCTTCCTCAGCCTGGGGCTGGTGAG
CTTGGTGGAGAACGCGCTGGTGGTGGCCACCATCGCCAAGAACCGGAACCTGCACTCACCCATGTACTGCTTCATCTGCT
GCCTGGCCTTGTCGGACCTGCTGGTGAGCGGGAGCAACGTGCTGGAGACGGCCGTCATCCTCCTGCTGGAGGCCGGTGCA
CTGGTGGCCCGGGCTGCGGTGCTGCAGCAGCTGGACAATGTCATTGACGTGATCACCTGCAGCTCCATGCTGTCCAGCCT
CTGCTTCCTGGGCGCCATCGCCGTGGACCGCTACATCTCCATCTTCTACGCACTGCGCTACCACAGCATCGTGACCCTGC
CGCGGGCGCGGCGAGCCGTTGCGGCCATCTGGGTGGCCAGTGTCGTCTTCAGCACGCTCTTCATCGCCTACTACGACCAC
GTGGCCGTCCTGCTGTGCCTCGTGGTCTTCTTCCTGGCTATGCTGGTGCTCATGGCCGTGCTGTACGTCCACATGCTGGC
CCGGGCCTGCCAGCACGCCCAGGGCATCGCCCGGCTCCACAAGAGGCAGCGCCCGGTCCACCAGGGCTTTGGCCTTAAAG
GCGCTGTCACCCTCACCATCCTGCTGGGCATTTTCTTCCTCTGCTGGGGCCCCTTCTTCCTGCATCTCACACTCATCGTC
CTCTGCCCCGAGCACCCCACGTGCGGCTGCATCTTCAAGAACTTCAACCTCTTTCTCGCCCTCATCATCTGCAATGCCAT
CATCGACCCCCTCATCTACGCCTTCCACAGCCAGGAGCTCCGCAGGACGCTCAAGGAGGTGCTGACATGCTCCTGGTGAG
CGCGGTGCACGCGGCTTTAAGTGTGCTGGGCAGAGGGAGGTGGTGATATTGTGTGGTCTGGTTCCTGTGTGACCCTGGGC
AGTTCCTTACCTCCCTGGTCCCGTTTGTCAAAGAGGATGGACTAAATGATCTCTGAAAGTGTTGAAGCGCGGACCCTTC
TGGGTCCAGGGAGGGGTCCCTGCAAAACTCCAGGCAGGACTTCTCACCAGCAGTCGTGGGGAACGGAGGAGGACATGGGG
AGGTTGTGGGGCCTCAGGCTCCGGGCACCAGGGGCCAACCTCAGGCTCCTAAAGAGACATTTTCCGCCCACTCCTGGGAC
ACTCCGTCTGCTCCAATGACTGAGCAGCATCCACCCCACCCCATCTTTGCTGCCAGCTCTCAGGACCGTGCCCTCGTCAG
CTGGGATGTGAAGTCTCTGGGTGGAAGTGTGTGCCAAGAGCTACTCCCACAGCAGCCCCAGGAGAAGGGGCTTTGTGACC
AGAAAGCTTCATCCACAGCCTTGCAGCGGCTCCTGCAAAAGGAGGTGAAATCCCTGCCTCAGGCCAAGGGACCAGGTTTG
CAGGAGCCCCCTAGTGGTATGGGGCTGAGCCCTCCTGAGGGCCGGTTCTAAGGCTCAGACTGGGCACTGGGGCCTCAGC
CTGCTTTCCTGCAGCAGTCGCCCAAGCAGACAGCCCTGGCAAATGCCTGACTCAGTGACCAGTGCCTGTGAGCATGGGGC
CAGGAAAGTCTGGTAATAAATGTGACTCAGCATCACCCACCTTAGCCCCTTCCAGAAAGTGCTTGAAGTTTGCGGGTGGA
GGGATGGGGGAGGGGAAGGTGGGCAGGGGTGAGAGTCGAGAGGGAAGAAAGGAGTCCCGGAAAACGTGGCTGCCTCCCCA
GGTGAGGAAGCCACAGCCCCAGAGGCCCCAAATGCCTGGGGAGTGTGGAGGTCCCAACCAGGCTTGCGCTGACCCTGCTT
CTCGGTTTTCTCTCCGTGCTGACAAACCCCAGCCTATAGGAAGGACGAGCAGGTGCAGCAGGGCCCCAGTCCCCTCCACT
CTTGACGCTGTCCTAGCTGCAGAAGAGGCGGGTTCCCAGCCTTCCCTGTGACCACATGTGACCTCAGCCGGGACACATCC
CTTTGCTGGCCCTGGCCCTGAGTCCCTCCAGCCATGATGAGCCGTGAATGGGACCATCCCTGTCCACTCTGAGATGCCTG
GAAGGGGCTCAGTGCAGGTGGGCTGGGGGCTGGGTCTGCTGTCTGCCCAGCACTGCCATTCTGGGAGTAGGCAGGTGGG
GAAGGGGTCGGGGGTGGAGGGTCTCTGTGTTCAGCCAGTCCTGGGAAATGCTTGATGTGAGGCTTCTGAAGATGGCAGTGAG
GCAGAGGCCCAGCCGGCGGAGAAGTCCCTGGGAGTGAGTACCTGGGGATGAACTATGCTGCCTGGTGCTGCGGGAGCAGTG
GCGCCCCTGGGCCATCCCTCTGCTGAAACCTGGGCGTCTCGCTGAAGAGACCACCTCCATTTCCTCTGCAGAGACTGAGC
ACTCAGTCAGCCCCCTTCCTGGGACAGGCTCAATGGAGGCTGCAGGGCCATCAGCCGACTCCTACGCAGCCTCAGTCAGC
AGCCCCCTGGCCAGCCCCACCCCTGACTGCCGGCCTCAGAACTGGGAGCTGCTTCCTGGCAGGGCCCGCCTCTGCTGGGA
GACCGGACGGTGAGTCAGCCTTAAGCCCGGCACCAGACCCCTCTGAGGATGGAGCAGGAGCTGGCTGCCCTGAGGCTGCA
AAACTTCTTCCCTCGTGGAGACAGGGAGGCACCTCAGACACTCACCCCGGACTCCCTTGAACAGGGACAGGGAGGAACCC
CAGGCAGCTAGACCCCAGCAGCAGCCACACGAGCACACTGTGGGGCAGGGAGGGGCATCTCTTGAGAACAAAAGATCCAT
TTCTCGACTTTCCAAACTGGAGAGCTTCTTGAGAGAAAAGAGAGAGACAGGTACAGGTCCACGCCACCCACACACAGCCC
TGTGCACACAGACCGGACACAGGCGTCCACAGGCAAGTTCGCAGCTGCTCATTTTGTGAAGTGAATGCTGATTTGGGGGC
CGGGTGGGGTTCGTCTGTACATCGTGCACTGTCAGACCCTTCCTGAAGGATTTTTGTTACTGAAGTATCAGAAGGCCCTT
GTTCTAAGGTGGTGGCACTGTAATATCTGGCCCAGGAGGAGGTCAGTGCCGTGCCGGCGAGTCCTTTGCTCCCTTCCTTC
TCTGACCAGTAAACGCATCTCCAGTCGCTCCCCCGAGGCCCCCCTGCACGCAGTCTCCGACCTCAGAGAGTGAGTCAGAT
AGAAGCCGCTGCCCACCCTCCCCCACCCTGGCCGGGTTCTTGCCTGGCTTGTGCGTCCTTGTGACAATTCCTGACACGGA
TGCGCACCGGGCAGTGGCACGTCCAGGTGCTGTGCGCGCCCGGGCCCACAAAGCTCCTTTGGACGCTCATGGCAGCCCCT
TGGTGGAGGAGGTCGGGTGCCCACCCCCTCCCCGCCCACTGCGGAAGCCGGCGACCCACGGAGCTCGCTCTCGGCCGCCG
CCACCCCTCTGTGTTCGCGCCCTTCCGAGCTCTGATCCGACGCTTTGTTTCTTCTCAGTGGGTTCAGGGCCTGGGCCAGC
CTTTACCTACCTCCCCCACCCAAAACCGGCAAAAGCTCAGAGCACCTTGTCTGCCAAAAGACAGGGAGCTGGGATGGTGC
GGGTTGGTCTCTAAACCGGCGTGGGGAAAAAAGACCCTCCGTACAAAGCCGCAGGGTGGGGCTGTCGCAAGGGCGGAACC
GAGAGGGTAGCTGGGGGCGGGGTTCCCAGGGCCAAGAGGGGCCATTGTCCTCCCTGGAGCCCGGCGCCCCCACAGCCAGC
TCCTCTGGGAGACAGCCCCTCCTTTCGAATGCGCGGGGCCCTCAGACCGCGCCCGGCCCAGCGCTGGGGGATCCTTGGCT
GCGGGAGGGGCGCCGCCATTGCGCGCGCGGGCGGGGGACGCGCGGTGCGGAGCCTGCGGGCCGGGCGGGGGGCTCTGCGGCGG
CGCCTCCCGATTGGCCACCCGCGGTGACATCAGCCGATGCGAAGGGCGGGGCCGCGGCTATAAGAGCGCGCGGCCGCGGT
CCCCGACCCTCAGCAGCCAGCCCGGCCCGCCCGCGCCCGTCCGCAGCCGCCCGCCAGACGCGCCCAGTATGAGGGAGATC
```

```
GTGCACATCCAGGCCGGCCAGTGCGGCAACCAGATCGGGGGCCAAGGTGAGGCTGCGCGCCCCGGCCTGTCCCGGGCCCCG
GGGCGGGAGGAGGGAGGCGCCGTGCCCCGCGGGCCGCACCTCCAGCTGCCCCCGCCCCTGCGAACCTGCAACAAAGGGAT
GCGCCCAGCGCCGCGCCGGGCGGCCGGGACGCGGGGCCCCCGCCCTGGGACTCTCGCCTGCACCTCTCTGCCCCTGCCCA
GGTGACCTCGGGCTGTCGAGGCGCAGCTCACGTCAGTCGCGAAGCCTCAGCCCCCTCCACACCTGCGCCCCCTCCACACC
TGCACCCCCTCCACCGGGCCTCCGCAGGTGCAGCTGGGAGCCCTGTCTGGGCGTGGCCCTCTTTTTTGGGGCCGCGGCAT
AGCCTTGAGTGAGACGGGTGGGGTAGGCAGGTTTGGGTGGGGTGGGGGGTTCTCCTCACAGCCGTGATTTCCTCGGCCCG
AACCCCCCACTGGAGTAGCCGAACGTCCCCCGAGAGGCTAGAGCCGCCCCGATTTCCCCAGCCTGCTCCGACACCCCTCG
GCGTCCTAGCTCCACCCTGTCCCTTTGTTGGAGGGGTTGGGCCTGGACTCGAGATGACCTTGGTCCAGGACCAGGCTCTC
CATCGGCGCAGCCGCTCCTGGGCAACCCCCGCGGGGCTTGACCGCGCCCACCCAGGGTCTACCAGTCTGGGGATTGGATG
TGGGAACAAAGCCGGGCTGTGGGGTGTGGAGGCTGCACCGTGGCGCGCCCCTCCCTCCATTGTTAGCCCACCTCGCAGAT
GTTGGGGCCACGCAGAAAGGGGCGTTGGTGCAGCTGTGCAGGGACCCCTGCCTAAATCATTAAGGGGACGTCGTGTCCCC
TGGTCCTGGGAGGCTGTCCCTGGCCCAAGGTCACACAGCAGGTGCGAGAGAGCTGGGCTGGCCCTTGGTTCTCATCCCCT
GCAGGAGCCAACCTGGGGCTCGGGCGGGGCGGGTTGGGGGCGCTGCTTGCCAAAGGATCTGCCCTCCGAACAGTGTTGGG
GAACAGGGGATCGGGGGGGGGAGGCTGGCTTTGTCAGGACCAAGGCCAGGGACCCAGCTCAGGGCTGGTGCAGCGCTGATT
CTGGGAAGGAGGGGGTGCAGGAGACGGTCCTGGAGGGGGAAGGGGCCAGAGAAAGGCAGCCTCCGGAGGCTGTTGCCATGG
AAACCAGGCAGGAACAAAAAGCTAATTACAACAGGGCCAGCCACGTGGCTGACATGTAAATTGCAACTTTGGCTCCGGGT
GGAGGCGCCTCTGAGGGTGGGGAGGCCCAGTAGTGGGGGAGACTCTAATCACCCAAGGAGACAGCTGTGCCTGCCAAGGAGA
GCCTGGGGGAGGGGAGACCTGGGGGACAGTGGCCTCAAATCAAACCAGGACTCCCGGAGCAGCCTCTGACAGCTGCCTTG
GCCTGATGGGAGGGGCTGAGGGCTCATTAATGTGGTCACTGGGGCCAAGCCTCCCCTGAGCATCTGCTCCTGGGAGGGTC
AGGGGTCGCTGGGGGGGAGGGAGGGCAGGCAGCCCTGGGGCTCCTGGGAGGGTCAGGGGTCTCTGGGGAGAGGGAGGGCAG
GCAGCCCTGGGGCCCACCTGGGGTTTCTCCTTCTATGGCTGGGATGGGCCCAGAGCTGTGGTCCCACAAGGCACTTGTCA
GGCAAGAGCTGGGATCTAGAAGTCAGGTGCACATGCAGACATCCCCTGAGGCTCCACGGGTAGCAGAGGGAGCCCTTGTC
CCCTGGACCGTGTCCTTGGCTGCCTTGTGCTCTGCTTCTGAACACTGGAGCTCTTAGTTTGCCCGCTACTTTTTATTCCT
TTTGAGTTGTTTGTTTTTTCTTAATATATTTCTTTTTAAAAATTAATACCTGGCCGGGCACGGTGGATGAGGAATCTCAT
GTAATCCCAGCACTTTGGGAGACTGAAACTGGAGGATCACTTTAGCGAAGGAGTTCAAGACCAGCCGGGATAACATGGTG
AAACTCCGTTTCTTAAAAAAAAAAAAAAAAAAAATTAGCCTGGCGTGGTGGGCCGCGCCTGTAGTCCCAGCTACCTGGGA
GGCTGAGATGGGAGCATTGCTTCAGCGTGGGAGGTCGAGGCTGCAATGAACTATGACCGCACCACTGTACTCCAGACTAG
GCGATAGAGTGAGATCCTGTCTCACAGTAATAATAGTATCTGATTCTTTTTAACAATGAAAGCAACAGAGAAGGAAATTT
TCTCGGGGGCGGGGGCGGCAAGAGCGTGTTCCTTTGCTCTGCTGCCCTTGTGGAATCAATGGGAAGTTCCTTGGCCGTTC
TCGGCCTCCACTCCCGCAGCTGATAGGGGGTGCAACGCGGCTGCTTCCCTGTCTGGGGTCCTCGGGCGGTGCCTGGCTGG
GCAGGAAGGCCCGTGCACTCTGGGCAGAGGGAAGTCGGGGTGACTCGAGGCGGGAGCAAAGCCGGGTCCCGGGCGGTCA
CGCGGCAGCGCGGACGACTCACCGCGGTCACCCCTGCGCTGGGAGCCGAGCCCCCGGCAGCTCGCGGGGATGCAGTAAGC
CGGGCGCAGCCCGCCGGGAGGATGAGAAAACCCGAAGCCCGGAAAGCCGAGCGGCCGCCTGCAGTCACCCCGGGGGGGGC
AGGGGCGGGCCGGGCTATGCAGAAACACCGGGGCCGCGGGACACAGGACGCTGCCCCACTGCCGCAGAGCTGAATCTGG
ACCCCCGGGCTTTGCTTTCCTGGGGCCTGTGCTCCTCCCTCCCCGGGGCGGGGTTCGCCTGCAGCGGCGGAGGGGGGAGC
CTTTGTCCTCCGCGCGGGCTGCGGCACCGCCTCCTCGCGGCTGCGGGGCGGGGCTGGGGCGGGGCCTGGCCGTCTCAGC
CAATGGGAGGCGGAGTCCCTGGCTCGCCCCGCCCTCTCGCCTGAAAGACCTGCGGAGCCGCGGGCCGGGCGGGAACCGCA
TTCGGGTCCTGGAGGGGCTTGGAGGCCCCAGTCTCGCAGCCCCTTCATCGGGGACCCCCGCTCCCACCTGCCTTGGCCAC
TGCCCTCAAAGCCCGGCCCCGCTTTCCCCAGGACCCCAAGCGCTGCGCGAGGTCAGCACCAAGGACAGCGCCCGGGCCGA
GCGCCTGGCCTCGAACGCCGGGTTCTGCTTTGTGTTTCCAGGTTTTTGTTTTTGGTTATGAGAGGAGGCCTGGCTCTGTC
GCCCGGGCTGGCGTCCAGAGGCGCGATCTCGGCTCACTGCAGCCTCGGCCTCCCGAGTAGCTGGGACCGCAGGCGCTCAC
CACCACGCCTGGCCACAATTTTCCTTTTTTTCTTATTTTCTTTTAGATGGAGTTTGGCTCTTGTTGCCCAGGCTGGAGTGC
AGTGGCGCGATCTCGGCTCATTGCAACCTCCACTTCCTGGGTTCAAGCTATTCTCCTGCCTCAGCCTCCTGAGTAGCTGG
GATTACAGGCGCCCGCCACCACACCTGGCTTATTTTGTACTTTTACTAGAGATGGGGTTTCTCCATCTTGGTCAGGCTGG
TCTCAAACTCCTGACCTCAGGTGATTCACCTGCCTCAGCCTCCCAAAGTGCTGGGATTGCAGGCATGAGCCACCACGCCG
GGCCGATTTCCTTTTCTGTAGTGATGTTAACAATTTAGTTTATAAAATTATATTTTATTGTTTATTAAATATGTAACATA
GGCCAGGTGCGGTGGCTCACACCTGTAATCCCAGCACTTTGGGAGGCCGAGGTGGGTTGATTTAACTTAAGGTCAGCACT
TCAGGGCCAGCCTGGGCAACATGGCAAAACTCCGTGCCTACAAAAAATAAAAAACAAAAAAAATTAGCTGGACATGGAGG
CACGCCGGCCTGTGGTCCCAGCTACTTGGGAGACAGAGGTGGGAGGATCACCTGGGGGAGGTGGGCAGAGGTTAAAGTGA
GCTGAGGTCATGCCCCTGCATTCTAGCCTGGGCAATAGAGCAAGACCCTATCTAAAAAAAAAAAAGGAACAATATATTTT
ATTACTTTATCTCAAATTATAATTTTATTAATTTTAACTACAAAAGCAATTAATATATTCTCCTGGTTAAAAAAAACCTT
AAATATTGCAGATAAACCTGGTGCCCATCTAGCCACCTCTCTCTATCCAAGTGGGAACAAATTTCTTTTTCTATCCTTCT
GGTCCATTTTTTTTAAGTGACCTGTGCATATGGTTTTAAAACTTTAAATAGTATCTAAAACATTTGAACAAAATGAAGCC
AACATGTGCTGAATGTTTTCATGTCAAGCTTTGTGCTAAGGCCTGGATGTGGGCACCATTCACACGTTCATCTCACAGAT
GAGAAACAGGTCCTGCGAGGCACGCGCCTGGCCCACAGCTGTGCTCGTGCTGGGTCTTGAATCCACTCTGCTCCATTGC
TCCTGCTGCCCTGTGCCTCGCTCTGCAGAGGACTTCCCATAGATGTGTTACTCTCTCCACCCCTCCTTTACAGGGGCTGG
TGGTTGTGGGTTCCCTGGAACCATAAATTTGGGGTGTCCCAGTGCTTTGGGGAGCAAGGTCCCCGACTCAAGTGGACTGC
CAGCAACTAAATTAATGGGGGATCCTCAAAGAGAGTGTTAGGGAGGCAAGCAGTAGGTGGGAAGCTTGGACAAGCCCCAA
ATGCCTCAGGGACCTTTCTGTATTTTTTTTTTTGAGACAGGCTCTTACTCTGTCGCCTGGACTGGCATGATCTAGGCTCAC
TGCAACCTCCACCCCCCTGGCTCAAGTGATCCTCCCACCTCAGCCTCCTGAGTAGCTGGGGCTATAGGCATGCGTCACCA
TGCCCAACTAATTTTTTTGTTTTTTGGTAGAGACAGGGTTTCACCATGTTGCCCAGGCTGGTCTTGAACTCCTGGACTCA
```

```
AGTGATCCACCTCTCTTGGCCTCCCAAACTGCTGGGATTATAAATATGAGCCACCATGGCCAGCTGATTTTTTTGTTTGT
TTGTTTGAGATGGAGTCTCACTCTGTCACTCAGGCTGGAGTGCAGTGGAGTGATCTCGGCTCACTGCAACCTCCACCTCC
TGGGTTCAAGCAATTCTCCTGCCTTAGCCTCCTGAGTAGCTGGGATTACAGGCACCCGTCACCACACTGGCTAATTTTTT
TTTTTTTTTTTTTGAGGCGGAGTCTCTATCTGTTGCCCAGGCTGGAGCACGGTGGCACAATCTCGGTTCACTGCAAGCTCC
GCCTCCCAGGTTCACGCCATTCTCCTGAGTCAGCCTCCCAAGTAGCTGGGACTACAGGTGCCCGCCACCGCGCCTGGCTA
ATTTTTTGTATTTTTAGTAGAGACAGGGGTTTCACTGTGCCAGCCAGGATGGTCTCGATCTCCTGACCTCATGATCCGCCC
ACCTCGGCCTCCCAAAGTGCTAGGACCACAGACAGGCGTGAGCCACTGCGTCTGGCCTAATTTTTGTATTTTTAGTAGAG
ATGGGGTTTCACCATGTTGGTCAGGCTGGTCTCGAACTACTGACCTCGTGATCCACCCGCCTTGGCCTCCCAAAGTGCTG
GGATTGCAGGCGTGAGCCACCGCGCCCGGCCTTTTTTTTTTTTTTTTTAAGATAGGGTTTCACTCTTGTCCCACAGGCT
GGTGTGCAGTGGCTTGATCTCAGCTCACTGCAACCTCTGCCCCCTGGGTTCAAGCGATTCTCGTGTCTCAGACTCTGAAA
CAACTGAAATTACAGGTGCGCGCTACCACGCCCGGCTAATTTTTGTATTTTTAGTAGGGACAGGGTTTTGCCCTGTTGTC
CAGGTTGGTCCTGAACTCCTGACTTCAAGTGATCTGCCTGCCTCAGCCTCCCAAAGTGCTGGGATTATAGGCATGAGCCA
CCACACCCAGCCTGAATCATTTTTGTAATTTAAAAAATTTAACGCAAAAAGAATAAAACTTAGCAATAACAAAAATGCACA
AAAGATGGAAAGAAATCCACCCCTGACCTTTGTGGAGAGATGACCTCTGATAACAGTCTCAGGGTGAACGGGCACCTCTG
ATAACAGTCTCAGGGTGAACGGGCATCCCGAGCACTGTTAGCAGAGGTGGTCCATGTCCATGCTGTCCGCGTTACAATA
ATTACACTATGTTTATTCTTAAGGTTTTAAAAACATTTATTTTTAACAAATTTTCTTTGAAAAGCTACCGTGTGCACGTG
AGAAAATCTCAACAAGACCACAGGGCACCCGTGAGAAGAAGCTCCTCCCAGTCTGGGGCCCCTCCCCAGGCACAGCCTTC
CACACATCTACCAGAGGCACCCACGCATAGAGAAGCACCCACAAGCAGTTTTCTTTCTTTCTTTTATTTGAGACGGTGTT
TCCTTCTGTTGCCCAGGCTTGAGTGCAGTGGCATGACATAAGCTCACTGCAACCTCCACCTCCCGAGTTCGTGTGATTCT
CCGCCTCAGCCTCCCAAGCAGCTGGGATTACAGGTGCTCGCCACCACGCCTGGCTAATTTTTGTATTTTTAGTAGAGACA
GGGTTTTGCCATGTTGGCCAGGCTGGTCTTGAACTCCTGACCTCAGATGATCCACTGCCTCAGCCTCCCAAAGTACTGGG
ATTACAGGCGTGAGCCACCGTGCCTGGCACAAGCAGTTTTCTCCCCTTCCTTCCTTCCTTCCTTCCTTCCTTCCTTCCTT
CCTTCCTTCCTCTCTCTCTCTCTTTCCTTTCTTTCTTCCTTCATTCCTTCCTTCCTTCCTTCCTTCCTTTCTTTCTGACA
GTCTCGCACTATTGCCCAGGCTGGAGTGCAGTGGCGCGATCTCGACTCACCACAACCTCTGCCTCCTGGGTTCAAGCAAT
TCTCCTGCCTCAGCCTCTCGAGTAGCTGGGAGTACAGGTGCGCGCCACCATGCCTGGCTAATTTTTGTATTTTTGGGAGA
GATGGAGTTTCACTATGTTGGCCAGGCTGGTCTCGAACTCCTGACCTCGTGACCCACCGCCTCTGCCTCCCAAAGTGCA
GGGATTATGGGGGTGAGCCACCGTGCCCAGCTATGAGTGGTTTTGTTTCTATTTATTTATTATTATTCTATGAGACAGAG
TCTCACTCTGTCGCCCACGCTGGAGTACAGTGGTGCAATCTTGGCTTACTGCAACCTCCGTCTCCTGGGTTCAAGCAATT
CTTCTGCCTCAGCTTCCGGAGTAGCTGGAACGTGTGAGCCACCACCCCTGGCTAATTTTTGTATTTTTAGTAGAGACAGG
CTGGTCTCGAACTCCTGACTTCCGGTGATCTGCCCACCTCAGCCTCCCAAATTGTTGGTATTACAGGCGTGAGCCACCGC
ACCTGGCCTCTATTTATTTATTTATTTTGAGATGGGGTCTTGATATGAATTCCTAGACTCATGCTTCTCATGCTTGCTAC
CACCCAGTCCTGCCTCCTGCACAGCAGTTTCCACTAAAGTCACCCCTTTACGAGAGGCATCTTTTTTTTTTTTTTGAGAT
GGTGTCTCACTCTGTCGCCCAGGCTGGAGTGCAATGGCATGATCTCAGCTCACTGCAAGCTCCACCTCCTGGGT
```

HUMAN mRNA SEQUENCE : hR7-161.1 (Seq ID No: 99)
```
TCTGGAGAGGGCAGGTCCCGGGGAAGCTCCGGACTCCTAGAGGGGCGGCCAGGTGGGGGCCCTGGTGACCAGGACAGACT
GTGGTGTGTTTTTAACGTAAAGGAGATCCGCGGTGTGAGGGACCCCCCTGGGTCCTGCACGCCGCCTGGTGGCAGGCCGGGC
CATGTGGGTGCTCACGCCCCCGGCATGTGGCCGCCCTCAGTGGGAGGGGCTCTGAGAACGACTTTTTAAAACGCAGAGA
AAAGCTCCATTCTTCCCAGGACCTCAGCGCAGCCCTGGCCCAGGAAGGCAGGAGACAGAGGCCAGGACGGTCCAGAGGTG
TCGAAATGTCCTGGGGACCTGAGCAGCAGCCACCAGGGAAGAGGCAGGGAGGGAGCTGAGGACCAGGCTTGGTTGTGAGA
ATCCCTGAGCCCAGGCGGTAGATGCCAGGAGGTGTCTGGACTGGCTGGGCCATGCCTGGGCTGACCTGTCCAGCCAGGGA
GAGGGTGTGAGGGCAGATCTGGGGGGTGCCCAGATGGAAGGAGGCAGGCATGGGGGACACCCAAGGCCCCCTGGCAGCACC
ATGAACTAAGCAGGACACCTGGAGGGGAAGAACTGTGGGGACCTGGAGGCCTCCAACGACTCCTTCCTGCTTCCTGGACA
GGACTATGGCTGTGCAGGGATCCCAGAGAAGACTTCTGGGCTCCCTCAACTCCACCCCCACAGCCATCCCCCAGCTGGGG
CTGGCTGCCAACCAGACAGGAGCCCGGTGCCTGGAGGTGTCCATCTCTGACGGGCTCTTCCTCAGCCTGGGGCTGGTGAG
CTTGGTGGAGAACGCGCTGGTGGTGGCCACCATCGCCAAGAACCGGAACCTGCACTCACCCATGTACTGCTTCATCTGCT
GCCTGGCCTTGTCGGACCTGCTGGTGAGCGGGAGCAACGTGCTGGAGACGGCCGTCATCCTCCTGCTGGAGGCCGGTGCA
CTGGTGGCCCGGGCTGCGGTGCTGCAGCAGCTGGACAATGTCATTGACGTGATCACCTGCAGCTCCATGCTGTCCAGCCT
CTGCTTCCTGGGCGCCATCGCCGTGGACCGCTACATCTCCATCTTCTACGCACTGCGCTACCACAGCATCGTGACCCTGC
CGCGGGCGCGGCGAGCCGTTGCGGCCATCTGGGTGGCCAGTGTCGTCTTCAGCACGCTCTTCATCGCCTACTACGACCAC
GTGGCCGTCCTGCTGTGCCTCGTGGTCTTCTTCCTGGCTATGCTGGTGCTCATGGCCGTGCTGTACGTCCACATGCTGGC
CCGGGCCTGCCAGCACGCCCAGGGCATCGCCCGGCTCCACAAGAGGCAGCGCCCGGTCCACCAGGGCTTTGGCCTTAAAG
GCGCTGTCACCCTCACCATCCTGCTGGGCATTTTCTTCCTCTGCTGGGGCCCCTTCTTCCTGCATCTCACACTCATCGTC
CTCTGCCCCGAGCACCCCACGTGCGGCTGCATCTTCAAGAACTTCAACCTCTTTCTCGCCCTCATCATCTGCAATGCCAT
CATCGACCCCCTCATCTACGCCTTCCACAGCCAGGAGCTCCGCAGGACGCTCAAGGAGGTGCTGACATGCTCCTGGTGAG
CGCGGTGCACGCGGCTTTAAGTGTGCTGGGCAGAGGGAGGTGGTGATATTGTGTGGTCTGGTTCCTGTGTGACCCTGGGC
AGTTCCTTACCTCCCTGGTCCCCGTTTGTCAAAGAGGATGGACTAAATGATCTCTGAAAGTGTTGAAGCGCGGACCCTTC
TGGGTCCAGGGAGGGGTCCCTGCAAAACTCCAGGCAGGACTTCTCACCAGCAGTCGTGGGGAACGGAGGAGGACATGGGG
AGGTTGTGGGGCCTCAGGCTCCGGGCACCAGGGGCCAACCTCAGGCTCCTAAAGAGACATTTTCCGCCCACTCCTGGGAC
ACTCCGTCTGCTCCAATGACTGAGCAGCATCCACCCCACCCCATCTTTGCTGCCAGCTCTCAGGACCGTGCCCTCGTCAG
CTGGGATGTGAAGTCTCTGGGTGGAAGTGTGTGCCAAGAGCTACTCCCACAGCAGCCCCAGGAGAAGGGGCTTTGTGACC
```

AGAAAGCTTCATCCACAGCCTTGCAGCGGCTCCTGCAAAAGGAGGTGAAATCCCTGCCTCAGGCCAAGGGACCAGGTTTG
CAGGAGCCCCCCTAGTGGTATGGGGCTGAGCCCTCCTGAGGGCCGGTTCTAAGGCTCAGACTGGGCACTGGGGCCTCAGC
CTGCTTTCCTGCAGCAGTCGCCCAAGCAGACAGCCCTGGCAAATGCCTGACTCAGTGACCAGTGCCTGTGAGCA

HUMAN PROTEIN SEQUENCE : hP7-161.1 (Seq ID No: 100)
MAVQGSQRRLLGSLNSTPTAIPQLGLAANQTGARCLEVSISDGLFLSLGLVSLVENALVVATIAKNRNLHSPMYCFICCL
ALSDLLVSGSNVLETAVILLLEAGALVARAAVLQQLDNVIDVITCSSMLSSLCFLGAIAVDRYISIFYALRYHSIVTLPR
ARRAVAAIWVASVVFSTLFIAYYDHVAVLLCLVVFFLAMLVLMAVLYVHMLARACQHAQGIARLHKRQRPVHQGFGLKGA
VTLTILLGIFFLCWGPFFLHLTLIVLCPEHPTCGCIFKNFNLFLALIICNAIIDPLIYAFHSQELRRTLKEVLTCSW*

Table 16

MOUSE GENOMIC SEQUENCE : mD13-023 (Seq ID No: 101)
CACGGTAAGTGATCTCTGTGTCTTAGTTTATCACCCTGTTCGAACCAGGCCTGAACCTGCCTAGTAGTAGGATCCAGAGA
TGCCTTGGCCCGGACAGGTGAGCAGCAGGAAACTGAAGCCCGGTGTCCACAGAAACGCCATTTCCTGGCTGGAGAGGTGC
CTCAGCTACTAAAGCACTTACCACTCTGGAACAAGACCGAGCTCAGTTCATAGATGTTTATAACTCCAGTGCCAGGAGCT
CCTGGAGGAAGGAAAGCAGTCACTGGCCGCTACAAAGGATTTTCCAGTAAACTTGTTTAGAGAATATTAAGCTACATAAA
CGTAAAATTTGGCTAAAGGAGCATGCTTCTGTCTGGCGGTTCAGGGAAGACAGCATGGCTGACAGACCTTGATGAAACCA
CACCACTCCTACCAGGAAGAGCCTAATTCTGCCAGGGAGGCAGGATAACCCTGGTAACAGCTGCCCTGGAAAAGGCGCAT
GCACACACACACACACACACACACACGCACGCACACACACAGTCTGATTGGGGTGACCTTGATAATGTTTTA
CATTGTAAATCTCCCCATTTTCAGGAACAACCAAACCTTAATGCCTGTCCCTGTAAAATATGGTTAATTGCTCCAAGTGT
AACTTGGGGCCGTGAAGTTGTTTAAGCAGGGGATTTCTGTCCAAGGCTGGTTCCCTTTTGTGTATTACCCAGAGCTGGCC
ATTGTGCTATCATTGAAGAGCTTTTTCTTTATTAATCTATAAGTACTTGGAATGATTTCTCATTGGGAAGACATATTTTA
AAGCAATCCAGAACCCCTTTCAGGTCCCAATGGGCACTAGCAGACACCTAGCAGACACCTGTGTAGACACACACATCATT
TAAAATACAACAGATCTTCAAAACCAACACAACACATCAGAATCCTCATCTCAAAGGCGATACGGTCACCACAATCTATG
TCCCAGAGTGGTAACAATTTGATGAAGCTTTTGTAGAATGGAACAAAGGATCACAAATCAAGACATTTTCCAAATACAGT
GTTTGAAACATCAAGTAGCTGAGCCACAGAAAGCAGGGGGATTTCAGCTGCAGGCCTCAGCTGCTACCTGACGGCAGGCT
TGGTGTTTTGGTTGGGACTGGGCACATTCCAAAGGACTCACCTAATGGTCTCAAAAGCATTAGGTCACCTACAGGGGACC
GTGTGTTTTGCTTTTGTTTCTGTTATTTTCTAGGAACTTTCATTCCTATAGCAGACACCCGTAAACCTTTCGGTGCTGGG
AAGGCTGAGACAGGCAGATCCTGAGTTCCAGGCTAGTGAGCTACACAGTGAGACTGGCCCCAGAATATAACCATATCTGC
TTATGTATTACAGAAGGAACAGATATTGACGTGTGTGTGTGTGTGTGTGTGTGTGTGTGTGTGTGTGTAAGG
TGGGAGCGTACAAGCCACGTATAAAGAGGTTGTTTGGATGATAAAATGAGGGCAGCTGTGGTTGGCTGGAAATAAAGCT
GACCCACAGTTCAGAATTCCTTTCAGAAATCAGGGTTTTTAGAAAGAATGAAAGGTCTTGTGGCCATGATTGATTCTTGA
AGTGGAAGAAAAGCATTTGACAGGAACACTAATATCTGGGGAATCTAAACACAGTGGGGCCTTAAAGGGAAGGAGAGGCT
ATGCTTTAAAACCTGGAGAGAGTTGGGCATGGTCATACATGCCTTTAATCCAAGCACCCAGGAAGTAGCAGAGAGGCAGA
AGGCTCTTTAGGCCATCCTAGATGATGTAGTGAGTTCCAGGCTAGCCACATAGTGAGACCCTGTCTCAACAAACAAAAAC
CCGGGCAAGTACTTCATCAAAAGTAGCAATAGCTGAAGAGACGGCTCAACAGAGTACTGGCTGCTCTTCCAGAGGACCCA
GGTTCAAGCCCCAAAACCCCTATGACCGCTCTCAACTGTCTCTTGTGAGGATCTGACGCCCTCTTCTGGCCTCCTCGGGT
ACTACACTCACGTGATACACAGAAATACATGCAGACATAAATACCTATGCACATAAAAATAAATCTTAAAAACAAAACAA
AAAAATCAACTGAGAATAAAAGGCTGGACGGATAACTCAGTGGGTTACAGTTATGCCCTACATGAATGGAGACCTGATCT
GGGAGCCTCAGAATCTACCGCAAGCCAGGTTGAGAGACAAAGACACAGTTTGTGTGTGTGTGTGTGTGTGTCTGTGTGTG
TTTGTGTTTGTGTGTGTGTGCACGTGCGCACGCGCATAAGAGCTTCAGCAACTCTTCCTGGGGGAATCTCCTGACCAATT
CCCCAAACAAAGGAGAGAGCTTTCTTTTTATAATTTTTTTTCAGGATAGTGGGAAGTTTCACGTCATGTGCTTCAGTTCAG
TGTAGCCTATGGGTCAAAAACACAGAGGAGCCAACTACCATAGCCTCAGGGCTGAAGGCACTGCTGCAAGGCCATGGCCC
AAGCCGCCTCTTTACTCTCTTGTCCCAGAAGGTTGTAGGGTTCCACCTAAGCTTCAGGGTCTTGTTCTACCTTCCAAGGA
TCCAACTCAGGTTGTCAAGTCTGAACGAAAATGCTCTTACTCACTGAGGCATTTCACTGGCCCCTGACACCCCATTTTAA
CTGAAAACAAAACAGCCTCCTCTCCTTGCAGGACGGAGTCCTATTCACTTCAGACTGTGACTCCCTTAAGACACAACAGG
CTCCACAGAGGAGCCAGGTCTGGCCTGCGAGCCACACATTGATCATGGCAAACCCCAACCTATTCTCTTCTCACCAACCC
TGTGAAGGGGAGTCCAAGGAAGTCCAAGAGGGGAAGGAACACCACATGGCCTGTCCACATGCCAGTGCCTGCTCTCGCCA
GATCTGCTGTCTCTCCCTGCACAGCCTTGCTGGGGACGAGTGTCCTCCTGCTCAGGCCATGAATGGCACCCGGAGCAGTA
GACGCTGGAGCCCTTTTAAGGTCTTGATCCCTGTGAGGTTGGGATTCTTTCTCATTGCCGGTATGGGAGGGGTGAGTATG
GGTTTGACCTAGAATCCTTCTGACCTAACAAAGGCCTCTTGAAGCAGCCCATGGGTTCCTTCTCTGTTTATGGCGTCTCC
AAATAAACACACTTGAGAACTTGCTATAAGCACAGAGACACCATCCAGACAAGTCAAGGGCAGCCTTTGGCCTACATGCG
GATGGGGAGTGGGCTCCCATCCTCCAGCCTTCCTGATCGTCACTGTCTCCTTCCAGATTCTTCTTCCCACATCAATCTTC
TTTGGGAACAGAGAGGGAAACCTCTGGGTTCTTTTCTTGCCTTGGAAGAGAAATATTAAAACGTACTTCCTGCCTGGTGT
GGTGGCAAGAGCTTTTGATCCCAACACTAGGAGACCAGAGGCAGATCTCTGTGAGTTCAAGCTAGCCTGGCCTACATAGC
AAGCTCCAGGCCAGCTCAGGCTACACAATGAGACCCTGGTTAAATGAAACAAAACAACCCTCCCTGCAATCACGTCCCTT
GTGTTCAGGAAATGTACCAAGATTTTTGAGATATGTGCTGATGCGTATCTTCCAGTTTTGAAATGACTGGTCTACACTCA
GTCACAGCCTAGAGCTAGAGCCTTTTGTCACCCAACTTTTTTTTATGAGGAGACTGTGGCACAGGAAGTCTAAGCAGCAT
GCCTGTGAACAATAACTTATATATTTTAAAGCCAAGACTGCATGTTGAAGAAAATTAACTTCCCATCCCAGCTCCATGGG
AAAGATACTAAATGGGTCCGGCTTATCTCCACACAGGAGCTGCCTCTGGGCCAAGCGAGAAAGTACCAGGCTAGCCCACG

```
GAAGAAGAGTCAGGAACAACACTCAGGAGGGGCAGTTCTGGGGGAAATGGGCAAAGAGGCACACAGAATCCTCTCCTCTC
CTCTCCTCTCCTCTCNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNTCCCCTCCCCTCCCCTTCCCTCCCCTCCCTTTGCTTTGCTTCTCTCTTCTTTCCTGCGTGCTCTCTCTCTCCTCCC
CCTTTATATCCCTTCCTTCCTTCCTTTCTTTTTTTTTTTTTTTTCTTTTTTCTTTCCTTTATTTTTAAAGGCTGGGCTCTT
ACACAATTTGACAGGCAAGTTGGCTTGTGCTTCTCACTCTAGCTTTGGGGAGACAGAGAGGCAGGAAGATCATAAGTTCA
AGGCCAGAGTGGACTGTACAGGGAGACTGAAAAGCCTTGGAGCTAGGTCAGTGGTAGAGTGCTTTCTAGTAAACACAAGG
CCCTGCGCTCAGCTCCTGGCATTAAAAAGTGGAGAAAAGAAAGAAAGATAGGGGCTGGAGAGGGCCCAGTGGCTAGAGCA
CTTGGTACTGTTGCAGAGAATGTGGGTACATTGTCAGCAGTTCTAGAGTGGCTCTGAAACATCTCTAAGTCCAGTTCAGG
AATGCAATGCCCTTTTCTGGCCTCTGTAGATATTAGATTCATGCATACGTGAAGACAAAGCACTCGCACACATAAAATAA
ATCAATCTAAAAACATTTTTAGATATTAAAAGCCACTTCCTGCCAAGTGTGGTGATAAATGGCTTTAATCTCATCACTAG
GGAGGCAGTGGCAGGTTGATCTCTACAAGTTTGAAGCCAGCCTTCCCTACCTAAATAGTTCTAGGACAAGCAGGGCTACA
TAGAGAAACCCTGTTCAAAACAAACAAGCAAGCAAACAACAACAAAAAGAAAATAAAATGTCACTTTAGAAATGGGAGAT
TTGAGTCAGAATTGAAGAGAGTCCAGAATCGCTTTAGAAAATAAACATTAAGGGCTGGAGAGATGGCTCAGCGGTTAAGA
GCACTGACTGTTCTTTCAGAGGTTCTGAGTTCAACTCCCAGCAACCACACGGTGGCTCACAACCATCTGTGATGCCCTCT
TCTGGTGGGTCTAAAGACTGCTACAGTGTACTCATAAATAAAATAAATAAATCTGAAAAGAAAAGAAAAGAAAAGAAACA
TTAAATTCAAATCCCATCTTCAGCCCTTAGTATTAAGGTTGGCAGGGAGATCTCCGTGGTTCTCTTGAGTGCTCCAACTG
CCGGCCTGCATCACCCTGGCTGGGCATGTAACTTAGTAGGTAGAGTGCTTGCTTAACATGCACCGTATCTTGGGTTGAGC
CCAACACCCACACAAACTGGACACAAACTGGAGGTCGGCTGGGATTACCTGAGACCCCTATCTCAGAACCAAACCGACCTC
ACCCGCCACCACCATCATCACCACGAGAAACCGATCTACACACTTCGTCTTCGGGGAAGCTGGGACGACTGAGTAACTGC
CACCTGCAGCTTAAACCAAACTGCTCATGAGTCTGCTACCGTGGCCATGGTGGGTCCAAACGGACCAGGAGAGGGTTCAG
CGTCCCAGTTCTCAGGTGTAGCTAAGGCATGGCTTTGTGAATGGTTCCTTCTCAGTTTGAACATCTGAGAAGACATTTCC
TCTGTTCCTTCAAGAGGTGTGCTTTCCTGGGTTTCTCCTGAGCCTTTTTGAAAAAATGGTTTCATCTACCTCACAAACCT
GTCAGGAAGAGCCCCCCTCCTCCCACAACTCTCTCTCTCTCTCTCTCTCTCTCTCTCTCTCTCACACACACACACACACA
CACACACACCACCCCAAACAAACAAAAACAGACAACACACCTATTGGATCCATGATACCGCCATAGCTGTCTACCACTTC
CTCTGACTTGGGGTGTNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNTAGGGATGTGGGGTGAGAGTAGAGGGGTAGGGTGGGGGTAAAGAGGGAGGGGTGAGAGTAAGG
GGGGAGGGAGGGAGGAGTGAGAGTAAGGGAGTAGGGGTAGGGAGGGAGGGAATGAGAGGAGGGAGGGAGGAGGTGTGGGA
TTTTATTTCAGAATCTGACCTGGATGGTAACTTTGAGAATTCGTATGGGAAAAACCCCAAAAGGGGCTTCCAGGATGCAG
ATGCCTGGCTCTGCTGCTTGAGAAGACTTGGACCAAAGGGTCAGCCATATTGCCCTGGATAAGTAGGGTGCCAGGAGAGC
CTTGTGGGTAGCCTTTTAGAGCTGGCTCCAGGCCCAGAGTGCCATGAAATACAGTCCCAGGAGCACAGAGATCCTGGCAT
TTTCTGCTTGTCCCAGCGTGAGAGGCCATGTGTTCTCCCCCATCAAGTAGGGTTGGGCCTAGTGCACACACTGAGCCCCA
AGGGAAAGAAAGGCAGCACTTCCTGCCCCTCCAGCTCTAGAGGGGTGCAGGTTCTGTTTTGTTCATTTGTTTAGTCATGT
GTCCTATCCTGACCCTCTTTCCCCCTCCCCCACCCATTTTTGAAGATATGGTTCTCTGTGTAGTCCTGGCTGTCTTGGAA
CTTATGTAGACCAGGCTGGCCTCAAACCTAGAGATCTGCCTGCCTCTGCATCCTGAGTGCTGGGACTAAAAGCCTGGGCC
AACACCACCAGGCTTACCTGCCTTCTTTAATCCTCATAAGCGGTTAGGAGCAGGACAGAGGTCAGCGGGAGGCTCAGGAG
CTGGGTTCTACTTTGGACTCCTGCTGAGGAAGCTGCCCCACCCCCAGCGGTTCTCAGGAGGGGATGGAAGGCTGTGAGCA
ATGCACTCACTTCAGCTCCAGGAGTCCTACCCATGCACCAGGACTTGACACAGTGCTGTCCTGAAGATCTGGACCCTGGT
TCTAGGACAGCCACCAACCGCCATGCTCGGGAAATGGATAGACACACCCACAAAATCCAGAGAGGCTGCCTCAGGACAGA
GCAGGCAGGACAGGTGCTGCTGGCTCTGCTGGAACACCTTGCCATGTGTTCTGAAGCTTAGATCCTGACCAAGGACTCT
CTGGACAAAGGATGAATAGAGGCTGCAACCATGAGAACTAAGGGCCCTCCTTTTTAGAGCCGTCCTTTAGCATGGTGGCT
GCAGATGCTGAGCAAATGACACAGCCAGTGCAAGGGACAGTGACTGGTATAGGCCATTTGTCTTCTGTAAGTCATTCTAT
AGGGAACCATGTCATTCCCCCATCCTTTATGAGGTTTGAACATTTTCAAAAGGCCCACAGGGGGCATGTGCAATGGTGTG
TGTGTGTGTGTGTGTGTGTGTGTGTGTGTGTGTGTGTGTTCCAGAGATGGGAGCACAGTAAGAAATGTTCTTCACT
GGGGCTGGTGAGATGGCTCAGTGGTTAAGAGCACTGACTGCTCTTCCGAAGGTCCCGAGTTCAAGTCCCAGCAACCACAT
GTTGGCTCACAACCATCCATCATGAGATCTGACTCGCTCTTCTGGTGTGTCTGAAGACATCAGCAGTGGGCTTACATATA
ATAATAATAAATAAATCTTAAAATAAAACAAAAGAAGCATTCTTCACATACTGTGAGGCTATAGCAATCTGGTTTTG
AGTGACACTGAGCCTTGTCATGACCAATCTAGCCCTACTTGGTCCATCTGCAGCGGAATAGGAAGCTTTCAAAATTCTTT
CTCCCAAGGGCTCTGAAAAATAAGTGCCCCAGAGTTCCCAACAATCTTCTAAGCCAGGACTGTTTATAAATGAAGTTCTT
AAGAAGCCAGGGATTGAGGGGGGGCGGAGCAGAGGAGGCAATTTCATGGTGAGGAGATACTGGGAAGCTGAGGGGACACTG
AAGACAGAAATGCAGACGGTGAGTGTGGCTATGACACCTGACTAGGCTTTTGTGTGCTCCTTTGAGTGAAGAAGTGGGTC
GGGACAATGCTCGGGGAAAGAATCGGTGTGGGAGGAGGTGCTGAAAAGAGAGAGGGCGATTTCCTTGGAGGAGACTAGTG
CTTGGACCCCGGGGCTGGAAACCCCTTATGAGACTGTGTGTATTGGGGGGGATTGCTATCCTGTGAGTGTACAAGTGGAGT
CACGGGGATGAGACTCTGTAGGCTGCTGACTTTCAGGGGCTTAAGGGGTGCGTAAGAACGGTTGTCTGAAAATGTGGGTCA
GAAAGAATTGCAGGACACGGAACAAGTCCTGCTCAGTCGGCGTGCTGCAGGTGGGGCAGCATTTTAGACCCAAGTGTCTCA
CGGACTTCCTCTCTCTGGCTCTTTTTTTTCTTTTCTTCTCACCTGTAAAATGGGATTGTTGTCTAGAAAAAGAAGACACTC
TCAGCTCCGGGTGATTCCAACAGCTAGCTAGTATGGTTTGGATGCAGGTAGAGTCCTTTGGGAGTGGTCCTAAGTACTCC
AAGCTCCGGGGTCAGCGTTGCCCTCTAGTGGCGATTTTGTGAACAACAGGCGAAGCAACCAGGAAGGCGGGGAGGGCAGC
```

```
CTCTGTCAGGTCGCCTTCAGCTCAGCATCTATCTGTCAGTGGGAGCCAGGGCGCACAGGACCTCCTGGGCAGAAGGCATG
GTAGGTGAGCGGGATACGGGCCCTGACAGGAGCGGCTCTCAGAGTGAGATGGGTGGAAAAACTCTGTGAGACATTTGGGG
GTTGTGGGAGAGACCTCTCAGTTAGTCAGTTTATTTCTGTCATTTTTCTGATGACTGTCGTCTCAGCGCTGCTCTCTTGA
ACCTGGGCTGTCACCTGCCCATCCCCAGCTCCTCTCCGTGAGAGTTCCGAGAGTGAGAACTCCAGGTCTAGTAGGTGCAA
AGAATATAAATATTTAGACTGGGAGTGATTCAGGTGACAGAAAACAGAATCAAATTTGGGTCAAAGTACAGTTTTTTAG
CTTTTTCACTCCTTCACTTAACCCCTACGGTGGCTTTCTCTTCACACCAAGGTCACACACAGCGTTCTGTATGTCAGGTC
TGCCATGTGTCGATGCTTTTTGTGGATTAAATACCGGGATCTTGCACCTGGAGTTGGGGAGGTCAGGTCTGCCCTGGCAG
TTGCTTTAGGAGCCCCGGCTCTTTAAAGACCAAGCAATACTGCAGTTGCACCCACTTTTCTGAGGTCCACATCAAGTGGC
TCTCCTGAAGTCTGTCTTAAAAGGGAGACAGCCAAAGGAGATAGCCAAGCGTTCTTGGAAAAACCTGGCATTTCTTCAAA
GATTCCAGTAGATTGTAAAGGGCCAATCTTATCATTTAAAAGTATTTCATTTGTGTGTGTGTGTGTACTTGTGCATATA
TACTGTTTGCATGTATGTGTGTAGAGGATAGAGGATAACTCTTTGTTGTTGTTGTTGTTGATTGGTTTTTGTTGTTTGT
TTTTGTTTGTTTTTTAGACAGTGTCTCTCTCTGTAGTTCTAACTGTCCTGGAACTTGCTCTGTAGCCCAGGCTAGCCTCA
AACTCACATGAGACCTGCCTGCCTCAGCTTCCTTCGCGTTGGGATTAAAGCTGCGCACTACCACTGCCCGGCCAATTTTA
TTATTTTCAAGGTTTGCACTCCGGATACTCTGTAGTTGAAATGCACTTTAGTGGGTGGCAGATGCTTTCTTTCCCAGTGG
CATGTGACTAATCAGTCCTCTACAGTGTGATAACTTAGGACAGAGCTGGGATTACCTAAATGACTTCTGGGTATCTCCCC
CTTCTATCCTACAGACTCCTCCCTGCCTCAATTGTTCCATCTTTCCTGGAGCGCTCTCCCCAAATGCTTCAAGGAGCCCC
CTGGTCTGCAATATCAGTGAGTTCAAGTGGCCCTATCAACCTGAGGATCTGAACCTTACCGATGAGGCCCTGAGGCTGAA
GTATTTGGGGCCACAGCAGATGAAACAGTTTGTCCCCATCTGTGTCACGTACCTGCTGATCTTCGTGGTGGGCACTCTGG
GCAACGGGCTGACCTGCACCGTCATCCTGCGCAACAAGACTATGCGCACGCCCACCAACTTCTACCTCTTCAGCCTCGCT
GTGTCCGATATGCTGGTGCTCCTGGTGGGCTTGCCTCTGGAGCTTTATGAGATGCAGCAAATTACCGTTCCAGCTGGG
TGCGAGTGCCTGCTACTTCCGAATACTGCTCTTAGAGACCGTCTGCCTAGCTTCAGTGCTCAATGTCACAGCCCTGAGTG
TGGAGCGTTATGTGGCCGTGGTGCGCCCACTCCAAGCCAAGTCTGTGATGACACGGGCCCATGTGCGCCGCATGGTGGGG
GCCATCTGGGTCCTCGCTACTCTCTTCTCTCTGCCCAACACCAGCCTGCATGGCCTCAGTCAACTAACTGTGCCCTGCCG
GGGGCCGGTGCCCGACTCAGCTATATGTTCGCTGGTGGGTCCCATGGACTTCTACAAGTTGGTGGTACTGACTACCGCAC
TGCTCTTCTTCTGTCTGCCCATGGTCACCATCAGTGTGCTGTATCTGCTCATTGGGCTGCGGCTGCGGAGGGAGAGGATG
TTGCTCCAAGTGGAGGTCAAGGGCAGGAAAACCGCAGCAACCCAGGAGACCTCCCACAGAAGGATTCAGCTGCAAGATAG
GGGACGGAGACAGGTGACCAAGATGCTGTGTAAGTCATTGTTGAGGAGAAAGCTGAGCTTCCTCCAGTCTGGGGAAGGAA
TTGAGCTTCCAGGGTATTGTTTGCTTGCTTGCTTGCTTGTTGTTGTTATTGTTTTGAGAAAGGGTTTCAGGGCTGGTGAG
ATGGCTCAGCAGGTAAGAGCACTGACCACTCTCCTGGAGGTCATGAGTTCAAATCCCAGCAACCACATGGTGACTCACAA
CCATCCGTAATGAGAAACTAATAATAATAATAATAATAATAATAATAATAATAATAATAATAAAACAAAAAAAACCTATGGGC
CAGACTTAGCAGGGACCAGAGCCAAGTGGGGGCTTGGGGTGGAGGGAGCGAGGCCCAGAGAAAGACAGAAAAGGGAAGAGGGGA
AAAAAAGAGAGAAGGAGTTTCTTTGTGTAGCTCTGGCTGTTCTAGAACTTGCTGTGTAGACCAGAGATTCACCTGCCTCT
ACCTCCAAAGTACTGGGATTAAAGGTGTGCACCACCACTACCTGGCTCTCCTGTCTGCTTTTCTAGGACCGGAAGATAGT
GAGCTGTAGATGCAAACTCTAAGCATGATGAAGATCAAATCTCTTCTCTGTCCTTGCAGTTGCACTGGTTGTGGTATTCG
GCATCTGCTGGGCTCCATTCCATGCTGACCGTATCATGTGGAGCCTGGTGTATGGACACTCAACGGAAGGCCTGCACCTG
GCCTACCAGTGTGTCCACATTGCCTCTGGCATCTTCTTCTATCTCGGCTCAGCAGCCAACCCGGTGCTCTACAGCCTCAT
GTCTACTCGCTTCCGAGAGACCTTCCTGCAAGCCCTGGGCCTTGGAACCCAGTGCTGTCATCGCCGCCAACCCTATCATG
GCTCCCATAACCACATCAGGTTGACCCACAGGCAGCACCCTGTGTGACGTGGGCCACAGGAACAGCAGGGACGAACCTCTG
GCTGTGAATGAGGATCCAGGGTGTCAGCAAGAGACAGACCCCTCCTGAATAAAAATTTGGAAGGAGCCTTACCCACAAGGC
CAGAGGGCCTCAGAAAGTCTGGGGAGCTACAAAAGTGGTTTCTGCCCTGCCCACATATTTGAGTATTGAGGGAAACAGTA
AGAAAAAGAGAATTGAACCCCAATTGCCCCCATTGAAGGCCTGAAACACTGGCTCAGCAGTTAAGAATGTTTGCTGTTCT
TCCAGAGAACCTGGGTTCAGGTCTCGGCACCCTCATGACAACAGGCCATCCGACACTCTCTTGGCTTCTGACGGCATCTA
CCACATGCACATAGCACACACCCATGTATTAGAAAGACCCACGCTGAACCAAATATATGCTGTATGTCCCTCCAGACCCC
ATACCCCTAAATTCTTCTCCACAAAACTGTCACAGTTACCTAGTTACCTGGGGCTCAGCCACTATTCCTCTGGGGATCCC
TTCTTCCTCTCAAGTTCATACGTGTGTTTCCATTGGGACCCACCTGTCATCCTGGTAACTTCCGAACCTCCATAGACTCTCT
CTGCCCCTCCCCAGCCCTTGTTTTGTACTTGAGACTGACTTCACACCCTGACAGTTCCTGGCTCTCATTCTGCACCGAGA
TTCGGGCCTGATCAAGCTGTCAGGTGGTAGGGGTTCCCAGGGATCCAGGGGACCTGGACCTGACCTCAGCCTTTGTCACC
ATGTTTCCTTGTGGCAACAAGCAAATCACCCTGGCTTTCTAAGAGGCTTTGGTTCCTTGCTTGCAAAAATAAAAGGCGTG
CTTGCTTGCCCAAGTCACGTTTTGAAGGGAGCTTGCTGTGTTTTCATCTGTGTGTACAACAAAGTCAACCACTTGGTTTC
AGATTATCTGCACCACTCCCAAACCCTGCTTTCTCTCTGATCTTCTCATGGTCCTGCTCCAAGCTGTCAAGGGCAGGTCC
TTTGCTTGTCCTGAAACAGATAGGGCCAACCTATGCGCAGCCACTCCTGGCCTTTCTCACCTGCAGACAGGCTACCCTG
GCTGCCCTGGGGGAACACATGCTAGATCCTGTTAGCCAGGGGAGATCTGGAAGAAGCAGCAGGAACTGACTTAAGGACCA
TACACGGCTGTGCAGAGACAGGGTGTTGGTTAGTCTTTGTCAGCTTGACAGAAACTAGAGTCACCTGAGAGAGGGAACAT
CAGCCGAAGAATTACATCCATCGGACTGACCTGTGGACTTGTCTGTGGGACATTTTCATGATTAATGACTGATGTCACAG
GGCCCAACCCACCAAGGGCAGTGTCACTTCTGGGCAGGTGGTCCCAGGTTGTCTAAGAAAATAAGCTGAGCAAGCCAGTA
AGCCAGATTTCCCCTGGGGCTTCCGGTCCAGTTCCTGTCTCCAGGTTCCTGCCTCCAGGTTCCTGTCTCCAGGTTCCTGC
CTTAGTTTCCCTTGATGATGGACTATAAGCTGAATAAACCCTTTCATCCCCAAGGTAGCTTTGGTCAGGGAGTTGGGCAC
ACTGGCACACCTGGAAGTGAGGACTGGAAGATCAGGAGTTTGAAGCAATGAAAATCAAATGAGGACATGAAGCTCCGCA
AAGGGGACTAGGTGGTCAAGGGCAGTATTTCCCTGACATTTCATGCCACCCTGAGTCCCAGGGACAACCTGGTGCTGAGG
AAGGACCCCATTAATGGATGAACTGTGTCCTGCTGCAAGTTGCTGGGTCTTGTCTTGAGAGACCTGGTGTCCACAGCTAG
GGACTGCCCAGTGACCCTGCAGACAACCTGCTAACTGGTGTCCTGGGCTTCTCAGGGCACCAGGTGTCCAGGGGAGGAGA
```

```
TGGTAGAGAGCTATTTCATGATTCAGTTTTTATATGACAAAGACAACTTAAAGGGCAGAGAGTTGGGCACACTGGCACACC
TGGGAAGTGAGGACTGGAAGATCAGGAGTTTGAAGCTAGAGCTAGCCTGGGCTACACAATAAAACTCCCACTGAAAAACA
AATAAACAAGATTTTTATGGCTCATGTTTTCAGTCTATAGTCAGCTGGCTTCTCTCTCTCTCTCTGTTTTCAAGACAGGG
TTTCTCTGTGTAGCCCTGGACATCCTGGAATTCTCTTTGTAAACCAGGCTGGCCTCAAACTCATAGAGATCTGCCTGCCT
CTGCCTCTGGAGTGCTGGGATTAAAAGGTGTATGCCACCACGCAGGAAACAGTGAAACACTGCAGAAAAAAAATATGCTTGT
GAAGGACATGGTGAGATTGGGTTTCTTGCCTCACGGTAGTCAGGAAACAGTGAAACACTGCAGAAAAAAAATATGCTTGT
TTTTCCTCCCAAGGCATACCCTCAGTGACCTATTTCCTGTCATGTAGCCTTATCATGTCACCTTTAAAAGTTTCTGTCAC
CCCCCCCCCCAATAATCCTATGAACATATGAATCCATCAATAGCGTGCTTATGATCCAATCACCTTCTCAAATCCCCACTT
CCAGACATAGCAGCAGTGGACACTGTTGCTCTGGGAACCAAACAGTCAACACATGAGCCTTAGGAGGGTTAATAGTCCAA
TCACAACAGCTGCAGATTTGTTGTTTACTCAATTATACAAATATTAAATCCACTCTGTAGAAAAATCTGGAAAATACAAG
TAAACAAATGTGGAGAAAAGAAAGGGACGGGGACTGGAGTGATGGCTCAGTCCATAAAGCGCTTGCCTTATGAGTGTAAG
GACCCAAGTTAATCCTCAGAACCTATGGGACAATGCCTGGGGTCACAGTCGCCCATGATAAGTGACATCACTGAGGATGG
CATCCCGCTCCATGCACACAATGCACACACAAGCAAACACTTGTGCACGACAGTGTGCACCCACACACAGAGACTCATAC
ATGTGCATGCACAGACACATGCATGCACTTATGCACAGACATAACACACATCTGCATGTACCTGCATACTCACACACACA
GACACACATATGTGCATACACCTGCACACACACCGTACATACACATGTACATACACACCATACATACATTTTAAACAG
CATCTTCACTGGGAGCCGTAGGGAAAGAGCAATGGGAACTTGCTCAATAGGAACAGAGTTTTTGTCTTGCAAGATGAAAA
GTGGTGGAGATCAGCTGCATAATTATTACCTCAATAAAACTTAAGTTAGTTATGTTCCTTATGCTTATGAGCGAACAGCT
GTCGGAAGCAATGGAAGGAAGGAGGCTCATGGTTAAAGGGGGTCTATCCACCCTGGTGGAATGGCAGAGTTCATGGTGGT
AGGAATGTGCAGTCAAGACTCCTCACACCTGGACAGACCAGGAAGCAGAAGTGGGACTGTTGAGGCTCCGCTGGCTTGCT
CGCTTTTCTTTTTACTCAGTCCAGTACCCCCCACCCCCACCCCTGGTGTGGTGGTTTGAATACACTTGGTCCAAGGAGT
GACATTATTAGGAGGTGTGGCCTTGTTGGAAAATGTGTGTCACTGTGGGGGTGGGCTTTGAGAGACCTTCCTCCTAGCTG
CCCGGAAGTTAATCTTCTCCTATTTGCCTTTGGAACAAGATGTAGAACTCTCAGCTCCCCCAGCATCATGCCAGCCTGGA
TGCTGCCATGCTTCCCACCATGATGATAACAGACTTGAACCTTAGAACCTGTAAGCCAGCCCCAATTAAATGTTGTTATA
AGAGTTGCCTTGGTCATGGTGTCTCTTCACAGCAATGGAAACCCTAACTAAGACACCCTGTCTATGGGGTGGTCCACATT
CAGAATCTGTGACAGGTGGCCCTTGAGAAGTCAGCTTTAAATAGCATCTTTCCACAGGCCCCTTTGTTCCCAGAGTCCCC
CTTGTCCCGCCCACCACTCTCCAAGGGTGCTTAAACTCTAGAACACCTCTGGACAGAGATGCTCTGAAGTGTGCCTCTTA
GGTAATTTCAAATCCAGTCAAACTGAGCCATCCCAACACTGAACCACCCATTTGAATGGTAAACTTACTGTGTATATTTT
ATCACATTTTTTCCCATCACTTTGCTCTACTGAATATACTATGACATATTTCCTTCTTTAAGTGTTTTATCCGGTTGTTC
CCACCATCTCTAAACAAGTCTTTTCTGCTTTTTCCACAGAAGATGCTGTATCCAAACTCAAGGTTGCAACCCATTTGACC
CACAGGCTCCACTGTGACGTTCTTGGCCAGCCTGACTAAGCCCCCTGAGTCTCATACAAGTTACAGTGGGAGGAGTTCCT
AGATGTTGGCCTAAGAAGGCTCTGCCCCCTCCTGTACTCTGAGCTCAGAAGAGCTGACCAAACCAGGGAAGAAAAGCCAC
CCTCTCTGTTTGATCTACTATTGCACAGATTCTGGCTAAGCAAAGAGGGGAGGCACAAGGCCCATGAGCAGTCAGCAGAG
GCTAAGGGGGAAGGAAGAGATACTCTGATGGCAGTGGGAATCTCATAGAGGCTACAGTGGACCACGGAGCTGAGAGAAAA
GTTCCCTGTAGGAAGGGAGTTTCCTCAGGGATGATCCTGGATGTGTGGTGACAGGGTGCACACAGCATGAGGTCCTAAGC
ACCCTTGGAGAGTGGTGGGCGAGACAAGAGGGATTCTGGGAACAAAGGGGCCTGTGGAAAGATGCTATTTCAAGTTGAGT
TCTCAAGGGCCACCTGTCACAAGCACTGGGTAGCCAGCCCATGGGTAGATGTCAGAACTATTCTCAGAGACCACCTCTGG
ACTCCAAGAGACTTCTGTTCAGTGTCACATGGAACAGTGTTCCTGAGGGGTGGAGTGTGTGTGTGTGTGTGTGTGTGTGT
GTGTGTGTGTGTGTGTGTGTGAAGGGCTGTTCCACCAGAGAAGCACAGGGAACATGGGAAGACGGGCACATAGGAAGA
TGTGAGGTATAATGTGCTTCTTGCTGTGAACTTGCCACATCTATCGAGGGTGTCCTCAGGCTCTCAATGGCCAGGTTCCA
TAAGTCATAGGCCACCGGTGGCTCCTGGAATAGCAGCCAAGCTCGGTCCTTGAGAAAATGGGCTAGGATGGAGAGGGTAG
CAGCGATCCCTACCTAGGGCACTCACAGGGCACAACCAGAAGCATCGGCCCTGTAGTGCCCCTAGGGGGCGCTGCTGCTC
CTGTGCAGTGTCAGGGTAGCGCTTTGTGCTCCTTGGGCTAGGAGCCAACTTTTCTGAAAGGATATGGCGTGTTTGGGATG
TGTGTTGTAACATCTCTCTTTCTCATAGAATCTAGTCCATAGGCATGGCACTCCTGCACTACCGTGTGTGTCGATAGAGA
CCGTGTGGATGATCCCGCGGTCTCCAAGCACTTCTGCTGCTTAGAGACCTCTCCCCCACTTGGCCTTCTGGCTTGTGGCC
ACACTATTGTCTTGTAAAGGAAGTTAGGTGAGCCGGGCAGTGGTGGCTCAAGCCTTTAATACCAGCACTTCGGAGGCAGA
GGCGGGCAGATTTCTGAGCTCGAGGCCAGCCTGGTCTGCAGAGTGAGTTCCAGGACAGCCAGGGCTACACAGAGAAACCG
AAAGAAAAAAAAGAAAAGAAAAGAAAAGAAAAGAATAAAATAAAATTTAAAAAGTTAGGTGAGTGTTTAGTGAGGGCTG
AGAGTTCTTGCTGTTTAAAGTTCGGATCCACAGAAGCCTGTGTAGATGCCTGGTGTGTGCAGAGGGTGCAGGGGGTGGGG
TGGGAGTGGGGTGGGGGAGGGGCCTCAGGCTGGAATTCCAACCTCAGAAGGCACAGCCAGGATATCCCGGAACAAGTCAG
CTAGTAAGGTTGGTCATACCTTCAAGCTCTGGTTTTCTGTTTGTTTGTTTTTAAAGATTCCTTTATTTTATGTATATGAG
TACACTGCAGCTCTCTTCACACACAGCCAGAAGAGGATTCTATAACAGATGGCTGTGAGCCACCATGTGGTTGCTGGGAAC
TGAACTCAGGACCTCTGGAAGAGCAGTCAGTGCTCTTAACTACTGAGCCATCGCTCCAGCCCCTCAAGCTCTGGTTTCGA
TTGAGACTCTGCTTCAATAGAGAAGATGCAAGAAGAATCCAGGATAATTCCTGGTATTAACCTTAGGCCTCCACATGCAC
ACATATATACCATATGTGTAAAAAGATACATACACACCATACACACATGTACATTATACATATGTGCACAGGGGCACACC
ATACACACATGTACATTATACATATGTGCACAGGGGCATTCTAGGGCTCCTTACCTTGGTACACTTTTCCTCACCCTTAA
CACACACACACACACACACACACACACTTTTGAGACAAGGCCTCACTCATGTTTCCCACTGCTGTCAAAGTCTCAGTG
GAGCCAAGGGTGGTGATGAATTCCTGATTCTCCAGTCGTCCCCCTTTCAGCAGCACCACAAACGGCTGACTCCTGGGGGT
TTCATACCCAATACTTCTGGATTGCTGTCCAAGCTTATCAAGGCAACCCAAGCCACAACTGGTAACTCCTGCTGCCTAAG
TCAGCTCACACATTCCTGTCCCCAGTGACCTTCCTCCTGTAAAGCCACTGTCAGGACCTCCCCCACCAAACCCCCACCTC
CAAGAGCCAGTAGGATGGGTGCAGCAGGGCCAGCTCCTCCAGGACAGATGGCCTGAAAGTGACAGGCCCCCTGTAACTG
GACCAACCAGAGTCCATGCAGACCCTGGGCCTGAGCCTTGATCCCCATCCTCCCCTGAGGTAAGCAATAAAGCGGTGTAA
```

```
TTTTGTGGTAACCAGACAGCAATGTAAAGGACTGAGGGGACAACCCTAGACACGCTCCTTGGCTGCTACGGTTCCTTATG
GTTAAAAGTGTTAGCATCTGGGTGTTGAGTGGTATCCGGGAAGGGCATGAAGGAGCCAGCTGGCATGTAGCCCTGGGCTG
GCCTGCCGCATCCTACCGGCTCATGTGGCTCTAGAGTGTGTGTGTTGCAGTCTCAGCAATGGTGTTACAGGAGAAAAGTC
ACTGGGGACAGGAACGTGCAGACCAGCTTCGGCAGCAGGAGTTCCTGGTTGTGGCTTGAGTTGCCAGTGGTCTGTGTGTA
GTAATGAATCCACCGTTACTGAGGCTGGAGAGCTGAGGTATTTGAGCTGCGGGGGTGCTACTTGCTACTTTTGTGTTGGT
ATGAAGTCTTTATTCCACTTAAAGAGTTGGTAGTAGTGTTCTGTATTTTAAAACGCTGTTCCTAGATACAAGCTTTTTTG
AGGGTGAGGAATTTCTATTTCTAGGTTGTTGCAAACCAGAAGGTGCTATATTTAACCACACTAAAGCACCAAAAAAATT
GTCATGTTTTTATGCCAATTTTTAATATTTTCCAGCATAAGTAAAGCATTCCACGTGAACGTGAAAAACAGGCTGATGTG
GTGGCTTTACCCTGGACTTGGAGCTCTCTTATGCTGGTTCTTAGTCTTGTTAATAAATGCAGTTAAAACCAAGGAGCAT
TTTACTAGCATTTAAAAGTCAAGCAACAGAGCAAGCAAGATGTAAATCTTAGCCGCTGCTAGGAAGAGGGAAATGGGTGG
GAGGAGAATTGTTTCAGGCTAAGGAGCCTGGCAGCCGGTGAAAGCGATTGCTGCCACCGGCAGGTCTCACATGCTGCAAA
GAGAGAGCCACCTCCTGCGTGATATCCTCTGATCGCCACATGTGTGCTGCAGCAAGTATACACACACACACACACACA
CACAGGGAAAGAGAAAGGGAGAGAGAGAGAGAGGTGATCTAAAGCTATTATTTTTAATTATGTGTGTGTGTGTGTGTG
TGTGGGTGTCGATGGAAGCCTGAGGCACTGGGTCCCTCTGGAGCTGGCGTTACGGTGGTTGTGAGCCTCCAGAGGTGAGG
AGTGTACACTCTTAACACCTGGGCCATCTTCCCAGCATCCAAATATAATTAACCAAGAAAAGTGGTGCCTTTGAAGTTAG
ACCAAGCAAGCTGACAGGTTCAGCGATGGAGGTCAGCAAGCACTTGCTTGGTAGGAGTGGGAGGGGAGGCGTCAACGAAA
CAGTGAAAGGTCCAAAGGGATCAAACTGAAGTCATCAGACTTGGTGCCAAATGTCTCTACTCAACGAGCCATCTCCTGGC
CTCTTGATGACAAAAGCTCTGATGTTCCTGGGGAACTTTTGGATGTTGTATCTATGGCGAGCATGACATCAGGAGAGATT
ATATTCTTTTAGGTCGAGGAAGTTTTAAAAAGCTTAATGTGGACTGGTTAAAATTAGTAAGTGTTAGCTTGATGGTGATT
TGGCCATGGGAGGTATGAAAGCAGTTTATTGCCATATTAGAATCAAAGGTGGGGCTTAGTCTGTCTGTGGTACCACTGGC
CTGGAATCATTGTGTTGGAAACCTACTTGGGTCACGTCATGGAGGTGGAGACAGGAGAATCAGGCTCTCAGGGTCATCTC
TAACTACATAGCCAATAGCCCTGGCTAGCCCCACATCATAACCTGGACACACACACACACACACACACACACATACAC
ACACACACACACACCANNNNNNNNNNNNNNNNNNNTGTCAAACTATGGTAGACTTAGTTGACTTGGAATTTCCTAAA
TTGACTGAACTGTCCTTGAACTCACAGTGATTCACCTGTCTAAATCTTTAGGTCTAGCCTGTTGCACTTTCAAATACCTT
TAAATCCAGCTACTTATGTGGCAAGAAGATTTGTATTTGTGGCTAACCTGGGGAAACAAGTCATCTTGTTTCCACACTTC
AAAACAACTTAATATATTTTAGGCATACACACACACACACACACACACACACACACACACACACACATATATAT
ATATATATATATATATATATATATATAGTGATGGTTTGTATATCCTTGGACCAGGGAGTGGCACCATCTGAAGGTGTGGC
CTTGTTGGAATAGGTGTGACCTGGTTGGAATGGGTGTGTCACTGTGGGTGTGGGTATAAGATCCTCACCCTAGTTGCCTG
GAAGTCAGTCTTCCACTAGCAGCCTTTGGATGAAGACATAGAACTCTCAGCTCCTCCTGCACCATGCCTGCCTGGATACT
GCCATGCTCTTACCTTGATGATAATGGACTGAACCTCTGAACCTGTAAGCCAGCCCCAATTAAAAGTTGGTTTTTTATAA
GACTTGCCTTGGTCATGGTGTCTGTTCACAGCAGTAAAACCCTGATATATATATATATATATATATATATATAT
ATATATATGAAATTAAACACAATATTTAAGTTCCCCAGACTGTTAATGACAACATGACCATTTAGATGCTATTTAAATGT
GAGCATTGAAGCGTTTGCTCATGTGAAACATTTTTTAAAATGTTAATTTATTTAATTGTATATGTGTATATGTGCCTAAA
TGTATGTATGTGCATCACACGCATGTAGGAGCCTGTGAAGGCTGGAAGGGGCATCAGATTCCCTGGAACTGGAGTTAAAG
TCAGCTGTGAGCTACCATGTGGGTGCTGGGAACCGAACCCAGGTCCTTTGTGAGAGCAAGTACTCTTAACCACTCAGCAA
TTTCTTTAGACCCCAACACACATAAAGGTTTTTAATACTGGTTCTCATTTACAAAATGAAAACATAGATTGCCCTTTAAT
GTCTAACAACAGCAATTGGTTCAGTAAGTGACCCTCCTTATAACAGCTGAGATTAGGGTGTGTATCACTATGCCTTTTTG
TTTTGTTTTGTTACTTTTATTTTTTATAGTCCAGTCATTGGCCTCCTCTGTGTCCACCCTTGCAAAGATCCTCCTCCTCC
TGTCTCCTAGAGGATGTCCCCACTCGCCCCCCCCCAAGTTTTGGTTTTAGTATTTGTATTTCATGTATTTGTGTATGTGTC
TGCCGCATGTGTATGGGTGCCTGAGCTAGGTCTCGGACAGACAGCCCTCTCAGATGCTGTGNNNNNNNNNNNNNNNNNNN
NTGCTTGGTAGGAGTGGGAGGGGAGGCGTCAACGAAACAGTGAAAGGTCCAAAGGGATCAAACTGAAGTCATCAGACTTG
GTGCCAAATGTCTCTACTCAACGAGCCATCTCCTGGCCTCTTGATGAC
```

MOUSE mRNA SEQUENCE : mR13-023.1 (Seq ID No: 102)

```
CTGGTCTGCAATATCAGTGAGTTCAAGTGGCCCTATCAACCTGAGGATCTGAACCTTACCGATGAGGCCCTGAGGCTGAA
GTATTTGGGGCCACAGCAGATGAAACAGTTTGTCCCCATCTGTGTCACGTACCTGCTGATCTTCGTGGTGGGCACTCTGG
GCAACGGGCTGACCTGCACCGTCATCCTGCGCAACAAGACTATGCGCACGCCCACCAACTTCTACCTCTTCAGCCTCGCT
GTGTCCGATATGCTGGTGCTCCTGGTGGGCTTGCCTCTGGAGCTTTATGAGATGCAGCAAAATTACCCGTTCCAGCTGGG
TGCGAGTGCCTGCTACTTCCGAATACTGCTCTTAGAGACCGTCTGCCTAGCTTCAGTGCTCAATGTCACAGCCCTGAGTG
TGGAGCGTTATGTGGCCGTGGTGCGCCCACTCCAAGCCAAGTCTGTGATGACACGGGCCCATGTGCGCCGCATGGTGGGG
GCCATCTGGGTCCTCGCTACTCTCTTCTCTCTGCCCAACACCAGCCTGCATGGCCTCAGTCAACTAACTGTGCCCTGCCG
GGGGCCGGTGCCCGACTCAGCTATATGTTCGCTGGTGGGTCCCATGGACTTCTACAAGTTGGTGGTACTGACTACCGCAC
TGCTCTTCTTCTGTCTGCCCATGGTCACCATCAGTGTGCTGTATCTGCTCATTGGGCTGCGGCTGCGGAGGGAGAGGATG
TTGCTCCAAGTGGAGGTCAAGGGCAGGAAAACCGCAGCAACCCAGGAGACCTCCCACAGAAGGATTCAGCTGCAAGATAG
GGGACGGAGACAGGTGACCAAGATGCTGTTTGCACTGGTTGTGGTATTCGGCATCTGCTGGGCTCCATTCCATGCTGACC
GTATCATGTGGAGCCTGGTGTATGGACACTCAACGGAAGGCCTGCACCTGGCCTACCAGTGTGTCCACATTGCCTCTGGC
ATCTTCTTCTATCTCGGCTCAGCAGCCAACCCGGTGCTCTACAGCCTCATGTCTACTCGCTTCCGAGAGACCTTCCTGCA
AGCCCTGGGCCTTGGAACCCAGTGCTGTCATCGCCGCCAACCCTATCATGGCTCCCATAACCACATCAGGTTGACCCACAG
GCAGCACCCTGTGTGACGTGGGCCACAGGAACAGCAGGGACGAACCTCTGGCTGTGAATGAGGATCCAGGGTGTCAGCAA
GAGACAGACCCCTCCTGA
```

MOUSE PROTEIN SEQUENCE : mP13-023.1 (Seq ID No: 103)
LVCNISEFKWPYQPEDLNLTDEALRLKYLGPQQMKQFVPICVTYLLIFVVGTLGNGLTCTVILRNKTMRTPTNFYLFSLA
VSDMLVLLVGLPLELYEMQQNYPFQLGASACYFRILLLETVCLASVLNVTALSVERYVAVVRPLQAKSVMTRAHVRRMVG
AIWVLATLFSLPNTSLHGLSQLTVPCRGPVPDSAICSLVGPMDFYKLVVLTTALLFFCLPMVTISVLYLLIGLRLRRERM
LLQVEVKGRKTAATQETSHRRIQLQDRGRRQVTKMLFALVVVFGICWAPFHADRIMWSLVYGHSTEGLHLAYQCVHIASG
IFFYLGSAANPVLYSLMSTRFRETFLQALGLGTQCCHRRQPYHGSHNHIRLTTGSTLCDVGHRNSRDEPLAVNEDPGCQQ
ETDPS*

MOUSE PANTHER CLASSIFICATIONS
        FAMILY (SUBFAMILY)
                NEUROTENSIN RECEPTOR-RELATED(G PROTEIN-COUPLED RECEPTOR)
        BIOLOGICAL PROCESS
                Signal transduction(2.11.00.00.00) > Cell surface receptor mediated
signal transduction(2.11.01.00.00) > G-protein mediated signaling(2.11.01.07.00)
        MOLECULAR FUNCTIONS
                Receptor(1.01.00.00.00) > G-protein coupled receptor(1.01.01.00.00)


MOUSE GENE ONTOLOGY
        BIOLOGICAL PROCESS
                cell surface receptor linked signal transduction > G protein linked
receptor protein signaling pathway
                G protein signaling, linked to cAMP nucleotide second messenger > G
protein signaling, adenylate cyclase inhibiting pathway
                behavior > feeding behavior
                G protein linked receptor protein signaling pathway > G protein
signaling, linked to cyclic nucleotide second messenger
                cell-cell signaling > synaptic transmission
        MOLECULAR FUNCTION
                enzyme > 2-acetyl-1-alkylglycerophosphocholine esterase
                1-phosphatidylinositol 3-kinase > 1-phosphatidylinositol 3-kinase
regulator
                transcription factor > RNA polymerase II transcription factor
                amine oxidase > amine oxidase (flavin-containing)
                protein binding > lipoprotein binding
        CELL COMPONENT
                cell > membrane fraction
                plasma membrane > integral plasma membrane protein
                cell > plasma membrane
                cytoplasm > endoplasmic reticulum
                cytoplasm > Golgi apparatus
MOUSE PROTEIN DOMAINS (INTERPRO SIGNATURES)
                IPR000276 (GPCRRHODOPSN)
                IPR000276 (7tm 1)
                IPR000276 (G PROTEIN RECEP F1 2)
                IPR000276 (G PROTEIN RECEP F1 1)


HUMAN GENOMIC SEQUENCE : hD13-023 (Seq ID No: 104)
CAGGTACTGATGTCAGTGCATGCTTTTCAACATTTATAGATAGATATAAAAATAAGTATCAGGCTGGGTGCAGTGGCTCA
CGTCTGTAATCCCAGCATTTTGGGAGGCCAAGGCGGGCAGATAGCCTGAGGTCAGGAGTTTGAGACCAGCCTGGCCAACA
TGCTGAAACCCTGTCTCTACTAAAAATACAAAAATTAGCCGGGCGTCGGCTGCCCCGTCTGGGAAGTGAGGAGCGCTTCT
GCCTGGCTGCTGTGCAATCTTCCAAGTGTGAAGTGACAGCCTTTCTGCAGGTGTACCCAACAGCTCCGAAGAGACAGCGA
CCATCGAGAACGGGCCATGATGACGATGGCGGTTTTGTCGAAAAGAAAAGGGGGAAATGTGGGGAAAAGAAAGAGAGATC
AGATTGTTACTGTGTCTGTGTAGAAAGAAGTAGACATAGGAGACTCCATTTTGTTCTGTACTAAGAAAAATTCTTCTGCC
TTGGGATGCTGTTAATCTATAACCTTACCCTCAACCCCGTGCTCTCTGAAACATGTGCTGTGTCAACTCAGGATTAAATG
GATTAAGGGCGGTGCAAGATGTGCTTTGTTAAACAGATGCTTGAAGGCAGCATGCTCGTTAAGAGTCATCACCACTCCCT
AATCTCAAGTACCCAGGGACACAAACACTGTGGAAGGCCGCAGGGACCTCTGCCTAGGAAAACCAGAGACCTTTGTTCAT
GTGTTTATCTGTTGACCTTCTCTCCACTATTATCCTATGACCCTGCCACATCCCCCTCTCTGAGAAACACCCAAGAATGA
TCAATAAATACTAAAAAAACAAACAAACAAACAAAAAAAAACCCAAAAAAACAAAACAAAATTAGCCGGATGTGGTGGTA
GGTGCCTGTGATCCCAGCTACTTGGGAGGCTGAGGCAGGAGAATCGCTTGAACCCGGGAGATGACGTTGCAGTGAGCTGA
GATCATGCCACTGCACTCCAGCCTGGGTGACAGAGCAAAACTCCATCTCAAAAAAAAAAAAAAAGTATCAATATATATGTG
TGTATACATACAGTATATATAGTATGTGTGTATATATCTATATATTTATACATACATATATTCCCTAGCTCTGCCCACTG

```
GAGGGTCTGGGAGCAGCAACAACCCAACAGTAATGAGCACACTCAGCACCCAGATCTTGAGCACTGATTTCATTACCACC
AGATCTGACCCAGATCTCATGACTATTATTACATATATATATATTTTTTTAAGCAGAGTTTCGCTCTTGTTGCCCAGGCT
GGAGTGCAATGGTGTGATCTCAGCTCACTGTAACCTCTGCCTCCTGGGGTTCAAGTGATTCTCCTGCCTCAGCCTCCCAA
GTGGCTGGGATTACAGGCAAGCGCCACCATGCCCTGCTAATTTTGTATTTTTAGTAGAGTCGGGGTTTCACCATGTTGGT
CAGGCTGATCTTGAACTCCTGACCTCAGGTGATCCACCTACCTCGGCCTCCCAAAGTGCTGGGATTACAGGTGTGAGCCA
CCATGCCTGTCCTTTATTTATTTATTTATTTATTTATTTTTTGAGACAGGGTCTTGCTCTGTCCCTACACTTGTGTGCA
GTGGTACAAGCATAGTTCACTACAGCCTTGACTTCCTGGGCTTGGGTGATCCTTCCATCTTAGACTCCCAGGTAGCTTGG
GCTACAGGCATGCACCACCACACCTGGCTAGTTTTCGTATTTTTGGTAGAGATGGGGTTTCACCAAGTTGCCCATGCTGG
TCTTGAACTCCTGGGCTCAAGCAATCTGCCCGCCTCGGCCTCCCAAAGTGCTGGGATTACAGGCGTGATTACAGGCGTGA
TTACGGGCGTGAGTCACTGTGCCCGGCTGATCTTGGTTTCCATCATTCTCCACTAAAGGAGCCAGAGCTCCTTGGAAAAT
GGCTTATTCCAGGATAGCCCAGAGAAATAGGAGATGAGCCTAGAACATTGTATTGTGCCAGAAAATACAAAATATTCATA
ATGACACATCAAAAGGATATACGATCCAACTTGAAGGGGCTCCCACTGGTCAAATCTGGGACAAATTTGAACATCAACAC
AAATAATGATAATGAATTAGTATCCTTTGAATAGAATAAGAAATCATGGGTCCACATGGATATAAATAAAAGTGATAAGA
GGAAGTTTCTCCTTCTAGTCACAGTCAGCTCGGAAATGAAGGGATGATGGGGTTTAGAATCACCATTTGGCAGGCCATCA
TAGTAATAATTTATGCATACAAGAATCATACACAGGCCAAGCACGATGGCTCCCGCATGTAATCCCAGCATTTTGGGAGG
CCGAGGTGGGTAGATCCCTTGAGCACAGGAGTTCGAGACCAGTCTGGGCAACATGGTGAAACCTCATCTCTACGAAAAAA
ATAGAAACATTAGTGGACATGGTGGTGTGTGCCTGTAGTCTCAGCTACGCAGGAGGCTGAGGTGGGAGGATCAGCTGAGC
TTGGGAAGTCAGAGCTGCAGTGAGCCGTGATCGCACCACTGCACTCCAGCGCCTGGGTGACAAGCAAGACCCTGTCTTAA
AAAAAAAAAAAGTCATAAACAGATGCTAAAGTTCTCCTGGGCATCTTTTTGCAAATGGTTTTGCCAACCTTATAAGCCT
GCTGGGACAAGCTGTCCCCCAACCCTGGGTCTCACAATGCCTTGTGACTCCATTGTGCTCACACTAGCCGTCTGCCACCC
TCTCTGAGGCTGGGTGCCTTTGACAAGGGCTTGAATCCCTGAGTGGCAGGGTCATCTGTGGTTTGTTCTGGCTGCTTGGC
ACCTGGGCTGTCTGCCAGGGGCTGTGGATGACCCACTGGGTGGGGAATCTGCCCCTCCTCCCCTCCAGAAGGCTGCAAGA
CCCATGCTCACCCCACCTATGCAGCGAGAGTGCAGCAGCAGGACCTGACTTTGTAGCATCCTGTCTCTCACCCCGGGGCA
GGGGGTCCAGGGCAAGTGCTTCAAAAGGCGGGGACATGGTCCCAGCCTGGGCCAGTGTCCACGGGATGCCGGGTCACAG
TGCCAACCCTTCTAGCTGCAGCCCCACCGTCTCCCTGCCCCTCCAGCCTTCTTGTTGATTCTGTGAGCTGCCGGATGTTT
TTCAGGAAATCCGGCCTAAATCAGCTGGAGCCAGTTTTGATTTTTGAAAACCAAGATCCTTACCTGTTGCAGTAGCTTAC
ACCTGTAATCCCAACACTTTGGGAGACCGAGGTGGGTGGATCACTTAAGGCCGGGAGTTCAAGACCAGCCTAGACAACAT
AGTGTAACCCCGTCTCTACAAGAAATACAAAAATTAGCCAGGCATGGTGGTGCATGCCTGTAGTTCCAGCTACTTGAGGG
GCTGAGGTGGGAGGATCGATTGAGCCAGGCAGGTTGAGGCTGCAGTGAGCCGAGATCGCACCACTGCACCAGCCTGGGTG
ACAGAGTGAGACCCTGTCTCAAAAAAACAAACAAAACAAACAAACAAACAAAAAACAATTCAACAGGAGGATCTTCTG
GCACCCTGTCTTTCAGTCTTCACATACCTGGCCTTCTCTTAGCTTTAGGCCAACTGTGCCCATGCTGGTGCCCGCTCCTC
CATCACACACCCTCAGTGCACCTGCCTTTGGTCCCCTTTCCCTGTGTCCAGCAGTTGCTGTTGATAGGCCCAGCACTGTG
TACTTACCTCCTGATTCAGTCTTTGTTCCTAAGAACACCCCAACCCTAGAAAGACGCATTCCGTATGCATGCACCACTGT
CATTTCATCACCACTTTCCGGTTCTTTGATATATTTCCTCCAATTTAATGTTATTCCCTTTGTAATGCTGACCTTCCTCG
CACTCTGAATATTTACTGCTTTAGTATCTATTGGCTGTGGCAGCAGCCTGCCTTGGGAACAGGCAATAATGGAGAAGGTT
TGGAAAAGAGTTGTTGAACTGACTAAAAGTGGCCTGCTTTTTAAAAAGTGGCACGATGTGCCAGTGATTCTAAACAATGT
TGGTGATAAAATATTACTCCTGCCAGGCGGACTGCCTCCTTAACACCTACTCCCTGGTAGGCTACTGCCTATTGGACAGG
GGGAACTAATGGTATTCATAGAACACTTCTAAACTCAACCAGAACAGAAGCATTTAGTGGTAAAGAAGCATTATGTCTGC
AACTGACTCTCATATGTCCCCCAAAAATATAGGAATCCTTTGTATTATTCTTTCAACTTTCCTGTAGGTATGAAATAATGC
TAATATAAAAGGTTAAAAAGGTCAGGGCTGGGGCGCGGTGGCTCACACCTGTAATCCCAGCACTTTGGGAGGCCAAGGTGG
ATGCATCGCCTGAGGTCAGGAGTTCAAAACCAGCCTGACCAACATAGTGAAACCATGTCTCTACTAATTAGCCTAGTGTG
GTGTCACGCACCTGTAATAGCAGCTACTTGGGAGGTTGAGGCAGGAGAATTGCTTGAACCGGGGAGGTGGAGGTTGCAGT
GAGCTGAGATCACACACACCATTGCACTCCAGCCTGGGCAACAAGAGTGAAATTCTGTCTCAGAAAAAAAAAAAAAAAGTT
AAAAGGGAAAAGAAAAAAAACTACTAAATCAAACACAGCAAACTGCTGCAGACTAACAAGTTCCTGAGTCCTGATGCTGG
TCACCTGTTGCAGGGGGAGAAACTTTTGTTCTGAAACCTCCCTCTGATCATGACTATGAGAATTTATAAATGGGAAGACC
CCAAGAGGAGCTTCTGTAACACACCCAAGTGTGGTGTGTTAGCTGGGAAGGCTGGTGGACTTGAGGGTGGCCTTTATTGC
TTGGAGGAGTAGGTGCCAGGAGCACCCTGTGGGCACACAATAAGATCTGGCCCCAGTCCAGGGTGGCATGAGGGACACAC
AGGCCCTAGACATGAGAGACACAGGCTTTTTCTGGCCTTCTCACTGTGTGACTTGAACATGTCCTCCCTTTGGCCACAAG
CGGAGGTTGGGGCCTGGTACACAGGCTGAGCCCCTGTGAGGAGGTGACCGGCTCCCTGTGCCCAGCACCCTGCCTGAAGC
CTGTGAGCTGGCTGTCTCTGGGAGAGCTCAGGAACAGGCCTCACAGCCCTCAGGGGGCAAGGGGCTAGACAGTCCCCAGT
GGTCCCCTCTAGGAGCAGGAGAGGCAGGGGCGATGCTCCCCGGAGGTCTCCTCCGCTGCTGGTCCCCACCCGGCTGCAGG
GCCCCGACTGCCTGCACTGTGGCTGGGGCTCCCTGCAAGCCTGTGTGCTCCTTTCCACTGGGACTCAGCTCTCCTTCCCGG
GGTGCTGGGGACCTTGCCCTTCCAAACCCTACTAAGGACAGCTCCCACCCCAGCCCTGCCTCCCAGCAGCTTTCTGGTAT
CTTCCAGAGAATTCCAGGCTCTTCAACCCTGCACTGAGTGCTTAAACCCACATTATCACTCTTAATCCTCATGGCATCCC
TGAAGGACAGGGGCTATGTTTGTGTTTTACAGGCAAAGAAGCTGAGGCCTGAAGCGCAAAGTGGCTTATCTGAGGCCACG
CTGGCAGAATGCGGAGTCTCACTCTGGCCTCACAGCCTCCAAAGCCTGTACTCACCATGTTCCCTGCCTGGCTTGACCAT
CTCTCCCACCCAGTTTCTGTGACGACAGGGCTGGTGGCTCAGGGAGCATGGGTGCATGATCACACAAAAGCCACGTGG
CTCCCTGAGGTGCAATCCCAGCAGAGTTCGGAACCCCATCACCTGAGGGGCCCAGACCATCCTCCTCTAGCTCCTGG
CCATGGCCAGGCTGGGACCCCACAGAGCAGAGCTGGGTAGGGAGCTCTCTGACCTTGGGAGGTGCCCAGATGGGTACAGC
GACTCTCCCTTGCTTCGGATATTTACTGTTCTTGTTCTCCATGTTTGCCCAATGAAGCCTAGTGATGGGGGTCCTGGGTG
ATCCCCATTGCCCAGGCTGCAGAACAGTGCCTCCCTCCTGTCCAGAGCTACAGTGTCCCCAAGCTCATCTCATCCTCCCT
```

```
GCCTCCCCTCTGTAGGCAGTAAGTGGGGTGGGGTTGGGGCGTTTCTGGGGGAACCATGGGCTTGCCAGGAGCTGCGGGTC
AGCCCAAGGCTGAGAAGGTTCTGGTTTAAGCCTGGCCTGAGTTTAAGCAGCCCCTCTTGGCTCTCAGGGACTCAGCAGGA
TGAGGAGGGGGATACGGAGCTACTCACATGCCTCTGGCCCCATGAGTCCTGCACACGCCTGGGACTTGGGTGGTATCTCAG
CAGCTCCTGGAGGTCCCAGTGGCCCTCCCTGAATGCATTTGCTGTTGTGCAGAGTTGGGTCCTTGGCCCCCTCATCTGGG
CAGACGCCCACCAGCCAAGCTTAGATGGATGTGTACACCCAGAAAACTGGGGGGTGCCACCTCAGGACGGAGCAGGAGCA
GCAGGATGTCATACGTTTCTGATGGGACACTACCGTGGTGGCCTTGAGCTCAGGCCCGGAGGCCAGGGCCCCTAGGGCCC
TCTGCACAGTGCGGAAGTGAGGGCCACAGCCATTGTGGCCACTGAGCCTCTCCTTTTTGGGGGCTGCTCTTTAGCCTTTG
ATGGCTGCTAATGTTATGTGAATGATGCAACCCGCTGTAGGAATTCCTGACTGGCAAGAGTCATTCTGCCTCTGCAAGGC
TAGGTGGGTCCTTCCCCATCCCTCCAGGGCTGTGTGAACCAAAGGGTCCACGGGGACTTTCGGACTGCTCAGAGTCTGT
GCAGAGTGGTATGTGTGTGCGCGTGTGTGTGGTGTGGTATGTGCAGAGTGATGTGTGCATGCATGTGTGTGTATGTGTGT
GTACATGCATGTGCGTGTGTGTGTGTGTTCCAGTGAAGGGCTGGGGTCCAAATGCTCCTCACTTGCTAAGAGCAGCCTGT
GATGCCACAGTGGTCTCTGATGCTGAGTGATGTCATCCCTCCTTGCCCGTCTGTTGTGCTGGCAGCCCTGTTACTGAGGA
TTTCTCCTGGACTAGCTCATCAGGGGCTGAGTAGTTAATTCCCAGGGCACCACAGAATCCTTTCTCCCAAGGCCCTGAGG
ACCACGTGCCCCTGCACTTCCCACAACCGTCCCCTGCCTGGGACTCTGTTTACATGTGGAGTTCCTGGGAAGGCCGGCCT
GGTGCAGGCTCCTTGAGGGCCTAACTCCCATCCCCAAGCCCAGACAGTGACTTATGGAATTGGGGGGCAGTGGGGAGGAG
GAGGGTGCAGGAAAGCGGGGGGAAGGAGGAGCCTCCAGTTGCCACTAAATGGTGACAATCCCACCCCACATTTCTTGAGT
GTTGGCCATGTGCCAGCCCCTGTGCTGAATCTTGACGCTCAGTGTCTCATGTCACCTTCACAATAGTGGCGATGGTGGTT
AGCCCATTCTACAGGAAGGGGAACTGAGGCACACAGAGCTTCTGAGCTCACGGTGGCCTCTCGGGGTCCTGTGGCTTTGT
AACCTCTCCTGGTGCTCCTCAGTGTGATTCTGGGGCATGGGGCTCTCCAGAGGGCGTTTGTCAAACATGAGGATTTCTGG
GCCTTGCCCAGGCTAAGGAGACAAAACTCTGGGCAAAGGCCCCGGAACCAGCATTTTACTCTGTGTGATCCGTCATTTCT
AGGGTCCCTAGAAGTTTCTGATGAAACAGCTTCCAGCTAAGGAGCTGATTGCAGGACTAGGGAGTGGGAGCAGGTGAGGC
ACTGGGATTTGCATGTGGGCAGTGTGGGGGAGCAGGAGGCTGTGGCTGGGCATTCTTAACCCTTCCCTAAATTCCCTCCA
TCCTTGTCTATACCCCCACCCTTGGTACAGTGATGGGGGCAGAAGAACATTTGAACGGATAAGGGGGACAGTATTTGGGG
CTGAGGACAATGGTGAGGAGAGGGTGGCCCAGGGAGGCAGGTGAGGAGTGGGGAGGTCCACCCCCAGGCTAGACTTTGGT
GTGCTCTTTTTCAGAAGGCAGTGGGGGGGCCCCAAGGAAGGGTTTCGAGGGGGTGATGGTCGTTGGTGGCTGTGGGAGGA
TGGACTGGAGGGAGAATAGGGGGCTCTTGCCAGCTCCTGCAGGGCCGAGCGCCTGGACTCAGGGACTGGAGGCCCGAAGA
GCATGCGGGTGAGAGGGGCTGCGGAGCTGCAATCATGGGCCTTGGGACAGAAGAAGAGGCCTGGAGCCTCGGGAGGATGA
ACCGCACCGGGGCATGTCGACTTCAAGGGGCTGGTGGGCCGTCCCTGTCCTTGGAGGTGGGTCTGAATGGGGGGCAGGAG
GAGCTGGAGGCCGCAGGGACACCGTGACGGCGTTAACTAAACTCAGCACGCTGGAGGGCGGGAATCACATGTGAGCCCCA
GGTCTCTCGGGCTCCTCCTCTGCGGCTCTCTGAGGCTCAGTTTTCTCATCTGTAAAACGGGTTGTTGTGAGGGTTCCGTC
AGATAACGCTGCTTGTGGAGGTGTCGCCTCCACGCTGCTTGTGGAGGTGTCGCCTCCAACGGCCGCGTCCTCCGCCAGTG
CGGGAGGGGGCGCCGCCCGGGCTGCGGGGACTGCACTGCCCCCTGGCGGCGTTCCCGGCCGGACAGGCGGGGCGTCGGGG
CGCGGGCTGGGGCCGCTGTCAGTCAGTCCACTGGCTCCCGCGCCGCGTCTGTGTCCGTCGCTCGGAGGGTGGAAGCCGGG
GTCTCGCGGGCCGCGGGCCGCATGGTAGGTGCGCAGGCGCCGGCGTGGGCTAGGGCGCGGCAGCTGGGCCGGGGCTCGGT
GCGGGCAGGGTCGTCCTAGGGCGGGAAGGGTCCGAGGGCTCCGTCGGGGTGCCCCCCGTCCCCGCCACCGGGGAGCCTCG
CAACTTCTCGGGTCATTTGTGTCTTCCGGCCAGGGGCGCCGGGGGGCTGCGGGCTTGCGACCCTTCCTCGAGGACCAGGG
CGCCTCCCTTGAACCCGGGCTGGCCCTGCGCGTTCCCAACTGGTTCCCGAGGCTCCGCGCTGCTGTGCAGCGGGGGTGAG
GGCGGGCGGGAGGAAGGGTGTGGGGCTCAATAGAGGAGAGGGAACCCCAGAACTGGCAGTGCCAAGGGCGTCAAATATGG
AGACTGAACAGTTCAGGTGACAGAAAACAGAAAAAAGAATCAACTTGTGGATGAAGCATTTGTAGTGTGTAGCCTTGACA
CTCCCTCACTTGACTCTCATAGTGACTGGGTCCCCAGAGCAAGGGGGTGATCCCCGTGCCCTTCCGTATGCCAGGCAGGC
CCTGTGTTAATGTCTTTAATGGATTAAACTCCCTGGATCCTCCTAATAGGGAAACAGCCTCAGAGAGGTTACCTCGCTAA
AGGTCACACAGCCTGGAACTGGGGAGGCTGGATTCGAATCCAGGCAGTTGCTTCCTGAGCGCTTGCTCTTGAAGACCCTG
CAATACCAGGCATTGTGCCCATTTTACAGATGCCCAAACGAGTGCCTCATCTAGCATCATTGCTTGAAAGAGGAGACACC
CAAGATCTGTCATTTACTCAAAGGTTCCAATAGATCATGAAATTTAAAATCAAAACCCCACGTGTTTTTTTTTTTTAAA
TGTAGATTTTTACTTTTTCGTTGTTTGCTTTTTGTTTTTTGAGACAAGGTCTTTCTCTGTTGCCCAGGCTGGAGTCCGG
TGGCACAATCTCCGCTCACTGCAACCTCTGTCTCCTGGGTTCAAACAATTCTCCCGCCTCAGCCTCCCAAGTAGCTGGAA
TTACAGGCGTGTGCTACCACGCCCGGCTACTTTTTGTATTGTTAGTAGAGACGGGGTTTCACCATATTGGCCAGGCTGGT
CTTGAACTCCTGATCTCAAGTGATCCGCCTGCCTCGACCAAAGTGTTGGGATTACAGGTGTGAGCCACTGCACCAAGCTT
ATTTTTACATCTCTGACTTGTGGATGGAAGAGTTTTTTCTTTCTCAGTGGTGCCTAAAATAATGGTCATCATAGCGCTAA
TGTCACCTTTGGCTTGATGAGTGAGGACAGAGCCAGCATTCCCTGAATGATCTTTCTGGGCATCTCTCCCCTCTGTTCCA
CAGACTCCTCTCTGCCTCAATTGCTCTGTCCTCCCTGGAGACCTGTACCCAGGGGGTGCAAGGAACCCCATGGCTTGCAA
TGGCAGTGCGGCCAGGGGGCACTTTGACCCTGAGGACTTGAACCTGACTGACGAGGCACTGAGACTCAAGTACCTGGGGC
CCCAGCAGACAGAGCTGTTCATGCCCATCTGTGCCACATACCTGCTGATCTTCGTGGTGGGCGCTGTGGGCAATGGGCTG
ACCTGTCTGGTCATCCTGCGCCACAAGGCCATGCGCACGCCTACCAACTACTACCTCTTCAGCCTGGCCGTGTCGGACCT
GCTGGTGCTGCTGGTGGGCCTGCCCCTGGAGCTCTATGAGATGTGGCACAACTACCCCTTCCTGCTGGGCGTTGGTGGCT
GCTATTTCCGCACGCTACTGTTTGAGATGGTCTGCCTGGCCTCAGTGCTCAACGTCACTGCCCTGAGCGTGGAACGCTAT
GTGGCCGTGGTGCACCCACTCCAGGCCAGGTCCATGGTGACGCGGGCCCATGTGCGCCGAGTGCTTGGGGCCGTCTGGGG
TCTTGCCATGCTCTGCTCCCTGCCCAACACCAGCCTGCACGGCATCCGGCAGCTGCACGTGCCCTGCCGGGGCCCAGTGC
CAGACTCAGCTGTTTGCATGCTGGTCCGCCCACGGGCCCTCTACAACATGGTAGTGCAGACCACCGCTGCTCTTCTTC
TGCCTGCCCATGGCCATCATGAGCGTGCTCTACCTGCTCATTGGGCTGCGACTGCGGCGGGAGAGGCTGCTGCTCATGCA
GGAGGCCAAGGGCAGGGGCTCTGCAGCAGCCAGGTCCAGATACACCTGCAGGCTCCAGCAGCACGATCGGGGCCGGAGAC
```

```
AAGTGACCAAGATGCTGTGTAAGTGTCACGGCTGGGAAGAGGGGCTGAGCCAGGCACTGGGTATGAGTTTGGGGCTCCTG
GGGCCTGGGAGAAGGGGCTGAGTATCTGGGTTTATGGGGGACGAGGAACTCCTCTAGTCTCGTGTGTGTGTGTGTGTGTG
TGTGTGTGTGTGTCTGAGTTGCACTCCTGTGTACTCTTTTAGATTACCCTACTAGAATCTTACACCAAAAATACCTGT
TAATGGCTAACGGTAAGATTGTGTAATTTGTGGAGTCCAGTGCAACATGAAAATTCAGGGTCCCTTTTTCAAAAATGAAT
AAGAATGTCAAGACAGCAACAACAGAGCATTAAACCAAGCCCAGGACCCTGCTAAACTTGGGGCCCTGGCTCTGGAACCA
AAACACAGAAAGGGCAGGCATGTGGTTGGGCTCCAGGATGACAGAAAATGGGATCTGGACCCTCCAGGTCTCAGGTCTC
TCCTCTTCTTTTTTTTTTTTTTTTTTTTTTTTGAGACAGGGTCTTGCTCTGTCACTCAGGCTGTGGTGTGACCACGGCTC
ACTGCAGCCTCAACCTCCCAGGCTCAAGCGATCCTCCTACCTCACTCAGCCTCCTAAGTAGCTGGGGCTACAGGCAGGTG
CCACCATGCCCAGCTAATTTTTTCATTTTTGTAGAGACGGGGTCTCACTATGTTGCCAGAGCGGATCTTGAACTCCTGGT
CTCAAGTGATCCTCCCACCTCAGCCTCCCAAAGTGCTGGGATTACAGGCATAAGCCATTGCACCCGGCTCTCTTTGGTTA
TTAACGTGTCTTGGCCTTGTTCTTCTCGGCAGCAGCCTGTGAGGCATGGCCACCGGCACGCCATCTTCTTTCCTGAGGAA
CTGCTGTCTTTCCTTAGAGATGAGTGAAAAATCCCAAGAAGGATTCTGATGGGCGTGGCCTAGGTCATGCCCCACCTCCT
GAGCCAGAGTGGCAGGATGCCGAGGTGCACAGCCCCCAGTGGAACTACACAGTGAGAACTGGGGGGAGCAAAGTCCCCCA
GAGAAGGGGAGGAGGGTGTTGGAGAGGCCAGGTGCTGAATTTCCCCCATGCCATGTCTGCGGTTCCCTGGGAACCGCAGG
AGAGACCATTTGTCTAAACAGCCCCATCCCGAGGCGTGTCTGTGAAGGCCGGGCTCAAGGCACAGGGGATCAGGCCAGAG
GCCTGCGGATTAGGGCAGGCTAAGGTACTGGGATGAGGCTCTGGATCTCTGTGGCCTGGACCTGGCTCTTTTTCAAAAGC
TGTGGCCCTGGGGAAGCAAGACAAACTGATACTAAGCCGATCAAAGATCCGCCCCATCCACCTACCTGCATCCCAGCTCA
GCCCCACCTCAGCAGAGCCTGAATCAGGCCAGTGGGGTGAGCCAGGTCAGCAGAGGGCCACTGAGACTTTGGACAGAGCC
AGCTAACTGGGGGCCGCCCCATCTCCAGAGTGACTCGCTCCTCTCACCCCTCAGACACCGTCCCAGAGCTCAGTCTCTTG
TGCAGCTCCTGAAACGAGGCTGACCCCAGAGCTATGTGCATGCCTCCCAGCTCTGCACGGTGAAGCCTGTGCTCAGGACC
AGCTGCCCACTGCCGCCCTGCCTCCTGCCTACTACCCCGTCCCCTCCCTGAAAACATGGGTGGGCAGAGTCTGCAGTAGG
TGCTTAGCTGGGGTGGCTCACCCCTGGAAGGTGGGCTTCCTGCTTGCTGGCCTTCTCTCTTCCCTCCCTCCCTCCCTTGT
CCACTTTCCGCTGGCCCTCTGTGCACCAGGCCAGGGGCCAGGGAGGTTGTGAGCAACGTCCTTTTTGAAATCTGGGATTA
TGAGGAAAACCATGCTAGGGTATGATGGGGAAGACACTGCCCAGGAAAGCTAGTGAGTTGCAGCAGCAAACCCAAGAAAC
AAGGAACAAAAATCACAGCTGACCCTGTGGCAGAGACCCAGGGAAGGGCATAGTTGGGTAAGAACAAGGCAGGAGGGATT
TGGGGAGAAAGTGAAGGTTTGCCAAACTCTCCTACCTTTTGTTAGGGAGGGGGTGGGCAAGGGCCTGGCAAGTAAGATGG
GGGGCAGGTTAGTGCAGGCAAGACATTCAGATGGGCCTTGGGGAGCTGGGGCTGGAGCTGAAAAGGCCGGGGCCAAGG
CAGGCAGTGTGTGTGTGTGCATGCATGTGTGTGTGTGTGCATGCGTGTGTGTGTCTCACTCTCCCTGGCCCCATGACC
TCCGGTCTGCCGCTCTCTGAGCACGTGCCCCATTCTTCTTTTCTGTACTTATGCATCAGCAAGCATGGCCCCCAAGTGGG
CATCCAGAGTGGCCCTAGCCCCACAGCTTGTATCACCCCAGAAGCTCAACAGCCACAGCTAAGTGTCACCCAATTTCATA
TTCCAGGGTGAGAGAATCTGATTGGGCCGGCTGGGCTTAGGTGTCCACACCTGGTCCAGTTGGCTGTGGTCAGAGGCAGG
GATATGCCCCATTGCCCACAGTCAGTGACCATGGTGAGGGCAGCCTCTCTGAGAGAGGCATGGCCAGAACAGGCAAATTT
CCAGAAACTTCCACCTGGGTGATAGGTGAGGGTGGCCTGTGGGATAGGAGAGGATGTGAAAATGGGTGACCCAAACTGAA
GCCCGGGAAGGCAGCTGAGGGGCCTCGGGCGGGCACTCGGCCTCCCTTCTCTGTCCCTGCAGTTGTCCTGGTCGTGGTGT
TTGGCATCTGCTGGGCCCCGTTCCACGCCGACCGCGTCATGTGGAGCGTCGTGTCACAGTGGACAGATGGCCTGCACCTG
GCCTTCCAGCACGTGCACGTCATCTCCGGCATCTTCTTCTACCTGGGCTCGGCGGCCAACCCCGTGCTCTATAGCCTCAT
GTCCAGCCGCTTCCGAGAGACCTTCCAGGAGGCCCTGTGCCTCGGGGCCTGCTGCCATCGCCTCAGACCCCGCCACAGCT
CCCACAGCCTCAGCAGGATGACCACAGGCAGCACCCTGTGTGATGTGGGCTCCCTGGGCAGCTGGGTCCACCCCCTGGCT
GGGAACGATGGCCCAGAGGCGCAGCAAGAGACCGATCCATCCTGAGTGGAGCCTTAAAGTGGCTTCACCTGGAGGGGCCA
GAGGGTCACCTGGAGCTGGGGGAGACACATCTGCCTTCCTCTGCAGGGATGCCTTCACGTACTGTCCCTAGTTCAGCCTAG
AAATTCTGACCAGCACCTCAGTTTCCCTCAGAGGGAAACAGCAGGAGGAGGGATCCCTGACTGCTGAGGACTCACACTGA
CCAGACGCCACACCTTGTGCTTCTTATCTGTCCACTGCCACTCCCCAGTTCAAATCCTTACCCTGCAGAAATATCACAG
TTAGCTGGGGCTCAGCAGTCCTCCCTCTGGGGACTCCCTGCCACCACTGCCAGTTTCTGAAACGGTCCCACTGGGTCCTC
ACTGTCCTTCCCAGTTCCTGTTCAGGTTCTGGCAGGGGCCCAGGGATCCAGGGGACCTGGTTCCAATCTCAGCCCTGCTG
TCACCACCTTGTCATGCACCATCAAGCATATCAGTCTACCTTTCTTTTTTTCTGAGACAGAGTCTCACTCTGTCGCCCAG
GCTAGAGTGCAGTGGCGCGATTTTGACTCACTGCAACCTCCGCCTCCGGGGTTCAAGCGATTCTCCTGCCTCAGCCTCCC
GAGTAGCTGGGACTACAGGTGAGCCCCAGCATGCCCAGCTAATTTTTTTAATTTTTAGTAGAGACGGGGTTTCACCATG
TTGGCCAGGCTGGTCTCAAACTCTTGACCTCAGGTGATCCGCCGACCTCGGCCTCCCAAAGTCCTCGGATTACAGGCATG
AGCCACCACACCCGGCCAATCAGTCCACCTTTCTAGGCCTTGGTTCCTTGCCTGAAAAATGAAAGAGGCGCTGGCTTTCC
ACAGTGTCATGCTTTGGCACTTTAGCTATGGTTTTCTTTCTGTGTGTGTGTAAGCCACTGCTTATAATAAAACCAACAAT
ACCCTCAGACTGAAAGGGCGGAAGTTATTATCTGCATCTTTATCAACCCCAAGCCCCACTTCCTCCCTGACCTCCCCATG
CCCTCCCCAGCCTCTCCCAGCACAAGTGGGGCAAAGCCAGCATGCAAGCAGACCCCACCACCACAGCCCACCTCCGTCCT
CACATACGTGCAGGCTGGCTCGGGAGTCCAGTGAGCAGAGCATTGGACTTGGCTGGCCAGAGGGTCTCTGAGGGCAAGAG
ACATGGCCAACCAAGGGCAAGGAGTGACCCTGTGGAGGGTTCTGCCGAACTCAATGCAGTGAGAAGAGGGACAGGGACAA
GTAGTCCTTGAAACTGAGCCCCATTCTGAATCCCTGCAGGCCAAGTCATTGCTCAGCCAGGACTCAGTTCATGGAGGAAA
CTTGTCCTGCTGCAGTCCCTGAGTCTTGTCCTCCTGAGAGGAAGCCCTGGCTTCCAAGGCTGGGAGCTGGAGGATGACCT
TCGGTCGGTCTGTCTGGGTTCTCCCTGCAGACAGCTTCCTAGCTCATGCCCATAGCTCATGCTCCCTGCCGAGAAAGTGG
AGGACGTGGTACAGGGTTGCAGATGTTTAGTTTTAAAAATTCAATTATAAAAATAATAAATGCTCATGATAGAAAATTTG
GAAAGTGCAAATAAGCAAAAATGAAAACAATTTTAAAAATGTAAAACCTCTCTTGCCAGGGAATGGGGGAAGGGCAAGTG
AGGAGTTCTTTAATGGGTGAAGAGTTTCAGTTTTGCAAAATGAAAAAGTTCTGGAGATCAGTTGTGCAACAATATGAATA
TACATAACAATACTGAACTATACACTGAAATGGTTAAGATGGTACATTTTATGTTATGTGTATTTTACCACAATTTTTAT
```

400

```
AAAAAGAGGATTAAATCTAAAGGAAAGAAAAAATTAAAACCACCCATAACTTTACTCTGAAGCAGTAACAGTGGCATGTT
TCCTCCTAGTGATGTGTCATATGGTGGTTTTATTTTATATACACAATACACATAGACAATTTTGTATCTGGCCTTTTGTC
CAGTGGATGTTCTATCCAAGCTCACGCTTGCACTCATTTGACTCATGGGATCCACTCTGATGGGCCTTGGCCAGCCCTGG
GGGAGTAGGACTTGCATAAACCATCTGGGTGAATTCCCAGATGCTGGCCAGGAAGGCCTGGATGCCCCCTGCCTACTAAG
TCCCTGGCTCGGGGGAGGAAGAGCCGTCCTGCCTGTCAGTCCTGCCCAGTGGACAAGTTCTGGGAGAAGCACAGGGGCTC
CCGGACAGAGAGCAACAGAGGCTGGGGGTGGGGAGTGGGGACACTGAGGACAATGGGAGAAAATGGGAGAAGTGGGGACC
TCGTGGAGGCCAGAGAGTGGATGAGGGAGCTGAGGGGAGGCTGGTTGGCGGAGGTGGTTTCCTCAGTGGGGGGCCCTGAG
CCTGCCCTGACTTGAATGCACAGTCAAGCCCTGAGTGGGGACAGCTCCTAAGAAGTGGGCCTTGGGGGAGGGTGGGGGAT
AGGGGAGGGGAGTGACTGCAGGGACAGGGAGATTCTGCAGAGAAAGGAGCCTGTGAAAAGCTGTGCAGAGCCGTGAGCAC
CTGAGTGCTGCCCCTGAGCTCCCAGGGAAGTAGCCTTGGGGTGGGAGGGGGAGATGCCGGATCCCAAACTGCAGGCTGGA
CCCTGAAGTTGTTCTCAAGACGAGCTCTGGACTCCCAGAGGCCTGCTTCAACCACAGTCAGAGGCACCTCATGCTCGAGG
CACCCTGGACAGCACTATGAAGGGCCTCCATGTGCAGGCAGCAAGCAGTACGGGCAGTGGGGTTGTTAGAAGGGTGCCCT
CTGGGCCGGGCATGGTGGCTCATGCCTGTAATCCCAGCTCTTCGGGAAGCCAAGGCAGGTGGATCACCTGAGCTCAGGAG
TTCAAGACCAGTCTAGGCAACATGGTGAAACCCCCGTCTCTACAATACACACACAAAAAGTAGCCAGGTGTGGTGGCA
TACACCTGTGGTCCCAGCTACTTGGAAGGCTGAGGTGGGAAAATCACCTGAGCCCAGGAGGTCTAGGCTGCAGTAAGCCA
AAATTGTGCTACTGTACTCCAGCCTGGGCAAATTGGAGTGAGGCCCTGTCTCAAAAAAAAAAAAAAAAAAAAAAAAGAGAA
AAAAAAGAAGGGTGCCCCTTTGTGAGCACCATGTATGTCTCCGCAGCACGTCAGCAACAGGTCCTGTCAGTCACTGGTT
CCCATGGAAAACGGGAATAGCAGATCTGAACTCACCCCTTGAGAAACAGGGATGGCAATGGAAAGGGCAGAAGCAAGACC
TGGCAAAAACACCGGTCATGCTGGGGCTGCATTCTCCTTGGTCCCCAGTGCTACTCCCAGGTCAGGAGGCCTCCATGCTT
CTCAAAGTATCTAGCTCATCTGGGGCTCAAGTCACCTCCCCTTCCGACTGAGGTCACCATATCCCTCCTCCTCCTGTGGA
GTTCAGGCCCTTTCTACTTCCCCTCTTCTCCACTCCTGCTGCTTTCCTGGGTGACGTCAGAGTCCATGGGTGACCCCACT
GCTGCTCTGGGTGCCAGCTACTGATGGTTCCTGCAGCTCTGGAGACCCCACCCCAGTCTCCTAGCCCCTTGGTCACATCC
TCACTCCCTGTGCCTCCAGGTGCTCTCCCATTTCAGAAAGTGTGAGCTCTTGACTGCTACTGCCTCTATCCTGCTCTCTG
CTCCTATGACACATTTATTCTTTTATTTTTGTTTGAGATGGGGTCTTGCTTGGTCACCCAGACAGGAGTGCAGTGGCACA
AACATGACTCACTGCAGCAGTCAACCTGCTGGCCTCAAGCAATCCTCCCACCTCAGCCTCTCAAGTAGGACCACAGGTAC
ACATCACCATGCCTGGCTAATTGTTTTTCTTGTGTTGTAGAGGCACCGTCTCGCCATGTTGCCCAGGCTGGTTTGAAACT
CCTGGGCTCAAGCAATCTGCCCACCGTTGGCCTCTTAAAGTACTGGGATTACAGGCGGGAGCCACTGCATCCGGCCATAT
TTATTCTTTAACCTCATCTAGACTTCCAGGTTCTAGACGGCTTTCTCCCAATCCATCAGCCTCCTCTGAGCTTCACTTCT
TTTCTGATCCCACCATCAGTTCATCTACCCTACTCTGCCAGCGTTCCTGAGGGTGAGTACACTGGAATAATTCTTCCCTT
CTCAGAAAAAGGCTTAATTTCCTTGAAAATTGAAAGTCCAAGATTAGTCTCATCAACAAACAAAGCTGCATATGCCTACC
CTTCCTTCTGTTAGGCATCTACTCAGCAAAGGTAAAGTGTGTAGCACTGCACATCTGTTCTCTTCATTACCAGGTGGCAT
TTCCAGATCATTCCTGGCATCTGGGAGCCTATCTCATTTTTGTACACTTCTCCAAGTCTTCCCCTTTCCCTATTCTTGTC
TTCTAATGGATCCATCAGGTTCTTGTTCCAAATTTCTTTGTAGCGTGCTGGCTTCAGCAGCACATATACCAAATTTCCTT
GTAGAGAGGAGTTGCCTTGTGGGGGAGAGGGGGCACACCCTAGCATTTGAACTCCTTTCTTATTGGGGGGAATCTCTCAT
TGTGTGACTCAGTGGAAGACTAAGCCCAGTCTCCCACTTACAGGGGTGAAAGAGTTCTGGACACTCTCCACTGCTGACAT
CTATTGGTACATGATGGACCAAGCCAGCTGGGTGACCCAACGTGGGACCTGTATCTGGAGCAAGTGACACTAAGACGCAC
AACAACAGGGGCAGGCGTGTCCAGGGGCAGCAGCACTGACAGCAGTATCCTGCCCAGTCTGTCTTGGGTTCCGCCCTTTT
TCTCAGCCAGTTTTTCCTGCCTTCCTGTTGATATTTGAGCTACTGAGAGCATTTCAATAAGTTCTTTTTCTGCTGAAGTT
GGCTGGAGTCATTTGCAACCAAGAACCTTAACTGATCTATCCACTGGCTTGAAGGTTACACATTCTGTTTGTTCTACTAG
CAGTTATCTTTACAGTTTCTAAGATTAGCAAATATTATTGATTTCTTTTAAACTGCAAACTATAGTCTGTAATATTCTGT
AATTATTCTAATCCCCTCCTCCTCCTGGCAGGAAAAATGAGGACAGATTTCAGAATTTGATCCTAGAAACATACGTACA
CTATTTTATTTTATTTTATCTTATCTTCTTATCTTATTTTATAGAGACAGGGTCTCACTATGTTGCCCAGGCTGGTCTTG
AATTCCTGGGCTCAAATGATCCTCCTGCCTTGGCCTCCCAAAGTGCTGGGATTACAGGCAAGAGCCACTGCGCCTGACTC
ATACTTACATTTTTCTAACAGTCTAAACCTCTTCAGCACAAGAAAAGAACCCCAGTGTGCTTCTAACACACTGTGAGTTA
GTAGAGTGGAGTGTGGAGGTGGTGAGCAAGGACTCTCTGGTTGGGCTGCCCAGCTGTGACTCCTGGTTCTGCTGCTAATT
ATCCTTACATGTCAGAATGACCTAAGGCCAAAGTCAGACTGGACTGAGAGGTACTGACATTTCTAAAGAAAGAATGTACA
ATCTGCCTGCACACAAATGAGACTCAGAGGCAAACACTGACAGTCTGCAACATCGTTCCAAATGGTGTCCAGGTTTCTAA
ATCTACAAGGTGTAGTCTGAGGATGGTGGTGACTCTCTAAAGTAAATGTTCTTTCTGTGGCCCCTTCAAGGCAATACCCC
AACCTAACATTCAAGTTTCTGCACAAGGCAGGGCAGGAAAACTGGGGACTGGGCAGGATCAAAGGCCTGACGCATAGGTG
CGGGACAGTGGGGACTCAGGGAAGGCGAACTTGGGTAAGTGCGCCCTGGTCAATAAGAAGAGGCCATGGGCTGGAATTAG
ACTGGAGAGCCTGGAAGCTGCAGCCACATCCTATGGAACATCCACAAGAGTTTCTGGAATCTTTTAATCTAACATTCAAG
CCCCTTCTTCAGCTCTAAGCAACACAGGCATCATTCATCCAACATTTATTGAGCACCAACCATGTGCCAGGCACTTTTCA
AGGCTGAGGCGGGGTGAAGGAGAGGGCAGATAAACATACTGGCATATAAGTGCTTTGACAGCTGGAACTCAGGCATCGAA
GAGAGGCACCTGCCCCAGCCGAGGGAAGGCTTCCAGGAGGAGGTGATGCGCCCAAGTCCTGGGGAGAAGGGTCTGACTGG
CCCCTGAGCTTTGGCCAGGGAAGCAGTTCCACCAAAACTGAATCAGGGGAGGAGGTGGAGAGCCTAAAGGGAATGAGGCA
TGTCAGGAGGCAGAATGAACACTGGACAGGCACACACAAAAGCAAAGCAACTGAGGTCCACTATCATCACTGCTGGCGCG
TGGACTTCCCTGGCCACCCTTCCTGGAACTTGGACTTGGAACCTTCCCTCCAGTCTTTCTCCCTGTTCGTCTCACCTCCT
TTAGATGTGTTCAGGCAGGATTTCTTTTATTTGCATTTCAGGAAATTTGAAAAAAGTACAAAAAACCACCAAAAAAAATT
ACTTCCATTCCCAACACCCAAATTAACTGCTGTTAACATTCTGGCAATTTGATTCCATCATTTTCCTGCATATTACCCCC
CTTCAATTTAATGAGTAGGCTGGGCGCGGTGGCTCATGCCTATAATCCCAGCACTTTGGGAGGCTGAGGCAGGCGGATCG
CCTGAGGTCAGGAGTTCAAGACCAGCATGGCCGACGTGGCAAAACCCTGTCTCTACTAAAAATACAAAAATTAGCTGTGC
```

```
GTGGTGGCACGCGCCTGTAATCCCAACTACTCAGGAGGCTGAGACAGGAGAATCACTTGAACCGAGGAGGCAGAGGTTGC
AGTAAGCTGAGATCGTGCCACTGCACTCCAGCCTGGGTAACAGAGCAAGATTCCGTCTCAAAAAAAAAAAAAAAATTTAA
TGAGTAACAGATTATCCCCAATCATTTGCTTTGTAAAACAACACTGCTGGCCAGGCATGGTGGCTCATGCCTGTAATCCC
AGCACTTTGGGAGGCCAAGGTGGGCAGATCATCTGAGGTCAGGAGTTCAAGACCAGCCTGGCCAACACGGTGAAACCCCA
TCTCTACTAAAAATACAAAAAGTTAGCTGGGTGTGGTGGCAGATGCCTGTAATCCCAGCTACTCGGGAGGCTGAAGCAGG
AGAATCGCTTGAACCTGGGAGGTGGAGGTTGCAGTGGGCCGAGATTGTGCCATTGCACTCCAGCCTGGGCAACAAGAGGG
AAACTCTGTCTCAAAAAACCAAAAAACCAAAAACCAAAACAAAAAAAAAAATGGCCTAAAACCATCTAGTGGAA
GGTTGATGGTTATCCTGGATGGATCAAGCTGGCAACACTCAAATTGTCTGATCAATTTTAACACCACTGAATGTGGAACA
ACCAGATCTGAAGTGGTTAAGTGCCCCCTAGAGGGACATTACAGAAGTACACAGCACCAAAAAGAATTCTTGCAAAACAG
CTGAACCTGAATCTAACCAAGCCTCTAGTTTACAAAAACACTGATGAAGGAATATGGTAAAGAACCACAAGATTGTCAAC
CAAGTCCAGATTAGAGACAATTCTACAGGACAACTGACAGTTTCTCCAACAAATAAAAGGCATTTTTAAAGGGGGAGGTTC
TCGATTATAGGTTTTCAAAAGATTTAACGGCCAGGCGTGGTGGCTCATGCCTGTAATCCCAGCACTTTGGGAGGCTAAGG
TGGGAGGATCACTTGGGCCCAGGAGTTTGAGACCAGCCTGGGTAACAGAAGGCAATCTCTTCTCTACAAAAATGAAAAAA
TAAAGAAACTTGGCGTGGTGTGGTGGTGCACCAGCTACTTGGGAGGCTGAGGTGGGAGGACTGCTTGAGTCCAGGAGGTC
GAAGTGAGCCACGATTCAGCCACTGCACAGCCTGGTGACAGAGGGAGACTCCAGCTCAAAATTTTTTTTTAAAAAAGAGA
CATATCAACTAAATGTAATGTGTAGATATTTTAAAATCCTAATTCAAAACAAACCAACAATTAAAAGACACTTTTTATTT
TTGTTTTTTAAAGACAGGGTCTCACTTTGTTACCTAGGTTGGAGCACAGTGGTGGGATCACTGTAGTCTCAACCTCCTGG
TGGGCTCAAGTGATCCTCCTGTCTCAGCCTCCTGTGTAGCTGGGACTACTGGCACACACCCAGCTAATTTTTTTTTTTTT
TTTTTGGTAGAAATGAGATCTCGCTGTGTTGCCCAGGCTGGTCTCAAACTCCTGGCTTCAAGCAGGAGGATCCCTTGACC
CCAGGAGTTCAAGACCAGGCTGGGCAACATGGCAAAAGTCCATCTCTACAGAAACAACAACAACACCCCCCCAAAAAAA
ACCCCAAAAATTAACCAATGCATGCCTGTGGTCCCAGCTACTCAGGAGGCTGAGGCGGGAGGATTGCTTGAGCCTGGGAG
GTCAAGGCTGCAGTGAGCCATGATGGCACCACTGCACTCCAGCCTGGGTGAGAGCAAGTTCTTGTCTCAAACAACAACAA
CAACAACAAAAAACTCCCAAAATTTGCAATAAAGATTAACGTATATGTCTCTCCAGCAGTTTGAATGCTCTCTAGGTTAG
ACACATGCAAGTGGATTTGCTAAAGGAAGGAATGAGTATTTTTACCTTTACTAAAACTGCCAAATTTCTCTCCAAGTGTT
TGTAACAATTTGCCCTTTTACCAGTTTCTCCACAATCCCCAAACACTCGTAATTTCTGTCACTCTGACAGATAAAGTATC
TTATTAAGTTGCATTTCCCTAATGGTTGTATAGTTGAGCATGTCTTCATTCTTTGCCACTCAAATTTCTCCTGAAAATGG
CTTTTCATATCCTTCAGCCATTTTTGTTTTTTTTCTTGATTTGTATTTCATTCATATTACAATATTAAATTTGTTTTGTAT
ATGTTACAAATATTTCTAATTTGTTCCTTATGTTTTAAACTGGTTTATAGAAACTCCTGCATCCAGCAGTTGTAAATTGT
AATGTGGTCAAATCTGTCATCATTTCGTTGGTAGGGGAGAAAATATTTTCCTCTCCCATCTTAAGGGTTATGGCTGACAC
CCCTATAACAAGGTTAATGAGAGAAAGGCATAACAAATTTAAAGTCTTATGTGACACAGGAACCTTAAGAAACGAAGACC
CCATGACCTAAAGAAAACTGTATTTTTATGCTAAGTCTGATGAAAGAAGTAGATAGTTGTGCAGAAACATGATTGGACAA
AAGGGGCATCATCTAATGGTCATACATAGGGGTGGGAGGAGACTTCGGCATGGCCCGTTTGCTCAGATTCTTTCTTGCGT
CTCTGCATGACACTTCTCCTCCCCAGGTATGGCACAAGACTCCTCTGGAATGAGGGTCTTACAACCTACTTCACATGAGG
TAGGCCAGTGAATTCCTTTATGACCCTGCTTTATGGGAGGGAGCAGATTACTTACAGAGACCTTGCTTCTGTGGTTTTTCT
CAACTGCCAAGGTGCCATATTTTGGCACATTTATGGATTTTCTTGTGACTTATCCTGTCCTTGTTATTGATTTTAATAA
TACTTCCTGGCAGGGAGTGGTGATTCAATTTGCTAATACTTTATTTGAACCCTGCATCTGTTCTTAAATGAAATGGGCTT
TTGCCTTTTCTTCAGTTGTTCTTGATCAGATATATATAATATTTTCTTAAAATATTTGGGCTAATCTCATAAAGTTAGCT
AGGTAACAAGAGTTTGGTGAGGTACCAAATCTAAAACAGGTAGGCAGATTGTTTTTTCTATTCTCATTCTTTCCCAGTTT
GGGTCTTTCTGAAGCTAGGATAAGGCCAGGGCTCCTTACTCAAGATCACCCTTCCCTATCCCCTAAAAACACACACAATA
TCCCCAAAGTGTCACTGTTCAAGTTCCAACATGGGGCTAACTCCGAGGAGCTTGATCCTCCTACTGGGGCTACTGGTGTT
AATGGCCATGAGGCCTTGTGAGAGCACATGTTCATGACAGTCCAACTCAAAAAACTACGAGAGTATTAAGTGATGGAGAA
TTAAACCTTAACTACCGAGCCTCAACTTCTTTAAATGAAAATGA
```

HUMAN mRNA SEQUENCE : hR13-023.1 (Seq ID No: 105)
```
CAGACTCCTCTCTGCCTCAATTGCTCTGTCCTCCCTGGAGACCTGTACCCAGGGGGTGCAAGGAACCCCATGGCTTGCAA
TGGCAGTGCGGCCAGGGGGCACTTTGACCCTGAGGACTTGAACCTGACTGACGAGGCACTGAGACTCAAGTACCTGGGGC
CCCAGCAGACAGAGCTGTTCATGCCCATCTGTGCCACATACCTGCTGATCTTCGTGGTGGGCGCTGTGGGCAATGGGCTG
ACCTGTCTGGTCATCCTGCGCCACAAGGCCATGCGCACGCCTACCAACTACTACCTCTTCAGCCTGGCCGTGTCGGACCT
GCTGGTGCTGCTGGTGGGCCTGCCCCTGGAGCTCTATGAGATGTGGCACAACTACCCCTTCCTGCTGGGCGTTGGTGGCT
GCTATTTCCGCACGCTACTGTTTGAGATGGTCTGCCTGGCCTCAGTGCTCAACGTCACTGCCCTGAGCGTGGAACGCTAT
GTGGCCGTGGTGCACCCACTCCAGGCCAGGTCCATGGTGACGCGGGCCCATGTGCGCCGAGTGCTTGGGGCCGTCTGGGG
TCTTGCCATGCTCTGCTCCCTGCCCAACACCAGCCTGCACGGCATCCGGCAGCTGCACGTGCCCTGCCGGGGCCCAGTGC
CAGACTCAGCTGTTTGCATGCTGGTCCGCCCACGGGCCCTCTACAACATGGTAGTGCAGACCACCGCGCTGCTCTTCTTC
TGCCTGCCCATGGCCATCATGAGCGTGCTCTACCTGCTCATTGGGCTGCGACTGCGGCGGGAGAGGCTGCTGCTCATGCA
GGAGGCCAAGGGCAGGGGCTCTGCAGCAGCCAGGTCCAGATACACCTGCAGGCTCCAGCAGCACGATCGGGGCCGGAGAC
AAGTGACCAAGATGCTGTTTGTCCTGGTCGTGGTGTTTGGCATCTGCTGGGCCCCGTTCCACGCCGACCGCGTCATGTGG
AGCGTCGTGTCACAGTGGACAGATGGCCTGCACCTGGCCTTCCAGCACGTGCACGTCATCTCCGGCATCTTCTTCTACCT
GGGCTCGGCGGCCAACCCCGTGCTCTATAGCCTCATGTCCAGCCGCTTCCGAGAGACCTTCCAGGAGGCCCTGTGCCTCG
GGGCCTGCTGCCATCGCCTCAGACCCCGCCACAGCTCCCACAGCCTCAGCAGGATGACCACAGGCAGCACCCTGTGTGAT
GTGGGCTCCCTGGGCAGCTGGGTCCACCCCCTGGCTGGGAACGATGGCCCAGAGGCGCAGCAAGAGACCGATCCATCCTG
A
```

HUMAN PROTEIN SEQUENCE : hP13-023.1 (Seq ID No: 106)
QTPLCLNCSVLPGDLYPGGARNPMACNGSAARGHFDPEDLNLTDEALRLKYLGPQQTELFMPICATYLLIFVVGAVGNGL
TCLVILRHKAMRTPTNYYLFSLAVSDLLVLLVGLPLELYEMWHNYPFLLGVGGCYFRTLLFEMVCLASVLNVTALSVERY
VAVVHPLQARSMVTRAHVRRVLGAVWGLAMLCSLPNTSLHGIRQLHVPCRGPVPDSAVCMLVRPRALYNMVVQTTALLFF
CLPMAIMSVLYLLIGLRLRRERLLLMQEAKGRGSAAARSRYTCRLQQHDRGRRQVTKMLFVLVVVFGICWAPFHADRVMW
SVVSQWTDGLHLAFQHVHVISGIFFYLGSAANPVLYSLMSSRFRETFQEALCLGACCHRLRPRHSSHSLSRMTTGSTLCD
VGSLGSWVHPLAGNDGPEAQQETDPS*

Table 17

MOUSE GENOMIC SEQUENCE : mD14-023 (Seq ID No: 107)
GGGATTACTTGGAGATACACTTTGCTGTGGATTAGTGTTGCTTCTTTGGTTGGTCTGTAAGCTTAAGGCCCAAACTAGGA
GAGACAAGGAGGTTATTGCCCAGGCGCTTGCAGCACTAGAACATGGTGCTTCCCCTGATATATGGTTATCTATGCTTAAG
CAATAGGTTGCTGGCCATTCAGCTCTTGCACCCCACGAGGCTAGTCTCATTGCACGGGATAGAATGAGTGCACTTCAGCA
GCCCGAGAGAGTTGCACAGCTAAGCACTGCAGTAGAAGAGCTCTGCGGCATATATGAGCCTATTCTAGGGAGACATGTCA
TCTTTCAAGAAGGTTGAGTGTCCAAGTGTCCTTCTCCCCAGGAAAAACGACACGGGACCAGACCAGGACCCCTCTGGGTG
ATGAGCCTGGAAGGAGGTTATGTGTACGGCTCCTTTACCTGCACACTGGGGATTTGACCTCTATCTCCACTCTCATTAAT
ATGGGTGGCCTATTGCTCTTATTAAAAGGAAAGGGGGATATGTGGGAAGCCGCCCTCACATTTGCCGTTGCAAGATGGCG
CTGACATCCTGTGTTCTAAGTGGTAAACAATAATCTGCGCATGTGCCAAGGGTAGTTCTCCACCCCATGTGCTCTGCCTT
CCCCGTGACGACAACTCGGCTGATGGGCTGCAGCCAATCAGGGAGTGACACGTCCTAGGCGGAGGATAATTCTCCTTAAA
AGGGATGGGGTTTTGCCATTCTCTCTCTTGCTCTCTTGCTCTCTTGCTTGCTTACTCTCTTGCTCCTGAAGATGTAAGCA
ATAAAGCTTTTGCCGCAGAAGATTCCAGTTTGTTGCGTTCTTCCTGGCCGGTCGCGAACGCGTGTAAGGGGATGATAAAT
ATTGTTATCCATATCATGAGTTTATAACTACTTCTTGTTGTATTTTAGGTATGGCACCGGACAGACTGAAGAAGGTGACT
ACCTCAGTAATAAAGGAATCAGAAGTGCCAGGATCACCACAGAGAAGAAATGGATTTGTAGGAAGCTGTAATGGCAGCCC
TTGTTAAATTACTTAGCTTTTTTGATTATGAAATTAATTTTTTATTTAAAAATACTTATTTATTTTTTTCATGTTTATAT
TTTGAGATAGAGTCTCTGTATATATTCCTGCCTGTTCTGGAATTTACTATGTAGACCAGGTTTCCCTTGAAATCACAGAA
ATCTGCCTGCCTCTGCCTCTACATCTGAGTGCTGTTATATTAAATGTTTATCATGGTATTCCTAATGAAAGGGGTTGTCT
TATAAACAGTTTTAAGGGGAGGTTCTGATTCTAAGGTCTAGTTGCCTAATTGGTTCTTGATTTGTCAATAAAGAAGGCCT
GGTGCCAATTGTTCTGCAGAAGGTACAAGTGGGATTTCCGGGTCCTAGGAAGTAAAGGCAGACACAAAGGAGAGGGCCTT
TTTCTGCCAGGCTTTGAAACGAGGAGGAGCATGCAGAAATCATGTAAGATCTCAGGGCAGCCAGGGCCTGCAGCTCCACT
GTGTCTGGGTTGCCAGAGATATTTGGCAGGGGCTAATTGGAACAGCCCACTTAGTTTGGAGCAGAAGGAAGGATGGAGTG
GTGGTTTGGTAAGGGCATACATTTCCAGGCAGGAAATATTTGTGCCCACCAATTGGGATAAGCAGCAGGTTAAGGCAACA
AAGCTGTCAGAGTGTCTCTTTTCTGCAGATTCAAGGCTCTCAGGAGTGAGCTGCTCCTCCTGGAGCAGAAGTTGGTAGAG
AGAGAGGCCAAGTTGGGGCTGGCAGCCACTGATCCAGGAGCCAAGCCAGGTAGAGTGGAAGGGCCCCAGGAGGGGCTGAT
AGCAATGGTCCTGGGCAAGGCCATAGTGTGATTACACAACTACAGAGAACACATGGTAAATAAGGTGTAGCAGCTACTTT
GGGATTGAGACTGAAGGTTTCACAAACCACTAGATGTAAAACTACCATGTCACCAAGGTGTATCATCCCTGGTTTATACT
CAGCAATTTTATATTTTACTACACAGAAACCTGTATACTCGATTTACAAGCTTTTCCATTCATAATAGTTAGGAATGGAA
CCAGCTTTGATGTTCTTTAAGAGGTGAATGGATAACGGAAACTTGGTACACATACAAATGGAATACCATTCACCTGTAAA
GAAAAATGAAGTCTTGGAATTTTCAAGTAAATTGATGGAACTAGAAAGCCTTGTAGTTAGTGTTGTGACCTAGGTCCTGA
AAGACAAACACTGTATATTCTTTCATGCAGAACCTAGCTTGAAATCTCTTGTTTTATGTGCTTAACTTGGGGCACAAGTA
GGAGCCAAGCAAACTAGAAAGGGCATGGGCTCTTTTAATTCATTAAAAGTTAAATCAGGACCAGATAAAGTATCTAAACA
GTCCCATAAAAACCTAAGTAAACAGATGTAATCATTAAAAGTATTCCCCCTCCCTCAAAAAAAAAAAAAAAAAAAAAAAAA
AAGCCCAGGGCCAGGTGGTTTTAGCACAGATTTTTAAAGAAGACGTAATACCAATACTTCTCAAATCATTCCATAAAATA
GAAATAGACAGAACACTACCTACTTAGTTCTGTGAAGCCAAAGGTATTAAGCTAATAGCTAAACCCCACTAAGACCAAAC
AAAGAAAGAGAACTTCAGATGTGCCAGAAGAGGGCATCAGATCTCATTATGGGTGGTTGTGAGCCACCATGTGGTTGCTG
GGATTTGAACTCAGGACCTTTGGAAGAGCGGTCAAGTGCTCCTATCTGCTGAGCCATCTCACCAGCCCGAAAGAGAACTT
CAGACCAAGTTTGGATATGAATATCTATGCAAAAATACTCAATAAAATTCTTGCCAAATAATTTGCAGATTCATCTGGAA
TAACAAAAAACCTAGGATAGCAAAAACTCTTCTCAATGATAAAAAAACCTCTGGTGGAATCACCATGCCTGACCTAAAGC
TGTACTACAGAGCAATTGTGATAAAAACTGCATGGTACTGGTACAGCAACAGACAGGTAGACCACTGGAAGAGAATTGAA
GACCCAGAAATGATCCCACACACCTATGGTCACTTGATCTTTGACAAAGGAACAAAAACCATCCAGTCCAAAAAAACAAA
ACAAAAACAAAACAAAACAAAAAACCCCAGCATTTTCAACAAATGGTGCTGGCTCAACTGGCAGTTATCATGTAGAGGAA
TGTGAATTGATCCATTCTTATCTCCTTGTAGTAAGGTGAAGTGTATGTGGATCAAAGAACTCCACATAAAACCAGAAACA
CTGAAACATATAGAGGAGAAAGTAGGGAAAATCCTCGAAGCACAGGGGAAAAAATTCCTGAACAGAACAACAATGGCTTG
TGCTATAAGATCCAGAATTGACAAATGGGACTTCATGAAATTGCAAAGCTTCTGTAAGGCAAAAGACACTGTCACAAAAA
CCAAAAGGCCACCAACACACCAACAGATTGGGAAAGGATCTTTACCAATCCTAAATCAGATAGGGGACTAATATCCAATG
TATATATATATATATATATATATATATATATATATATATATATATATATATAACGAACTCAAGAAGATGGACTCCAGAAAAC
CAAATAGTCCCATTAAAAAATGGGGCATAGAGCTAAACAAGGAAATCTCAAGTGAGGAATATCGAATGGCTGAGAAGCAC
CTGAAAAAATGTTCAAGATCCTTAATCATCAGGAAAATGCAAATCAAAACAACCCTGAGATTCCACTTCACACAAGTCAG
AATGGCTAAGATCAAAAATTCAGGTGACAGCCAGATGCTGGCCAGGATGTGGAGAAAGAGGGACATTCCTCCATTGCTGGT
GGGATTGCAAGCTGGTACAACCACTGTGGAAATCAGTCTGGTGGTTCCTCAGAAAATTGGACATAGTACTACTGAAAGAT

```
CCAGCAATACCTCTCCTGGGCATATACCCTGACGATGTTCCAACATGTAATAAGGACACATGCTCCACTATGTTCGTAGC
AGCCTTATTTATAAGAGACAGAAGCTGAAAAGAACCCAGATGTCCCTCAACAAAGGAATGGATACAGAAAATGTGGTAAA
TTTACACAATGGAGTACTACACAGCACGTAAAAACAATGAATTCATGAAATTCTTAGGCAAATGGATGCATCTGGAGGAT
ATCATCCTGAGTGAGGTAACAAAATCACAAAAGAACACACATGGTATACACTCACTGATAAGTGGATACTAGCCCAGAAA
CTTAGAATATCCAAGATACAATTTGCAAAACACATGAAACTCACGAAGAAGGTAGACCAAAATATGGATACTTCATTCCT
TCTTAGAATGAGGAACAAAATACTCATGGAAGGAGTTACAGAGACAAAGTTCTGAGCTAAGATGGAAGGAAAGACCATAC
AGAGACTGCTCTACCCGGGGATCCATCCCATATACAACCACCAAACCCAGACACTATTGCATATGCCAGAAAGATTTTAC
TGACAGGACCCTGACATAGCCCTCTCTTTGGAGACTATGCCAATGCCTGGCAAATACAGAAGTGGATGCTCACAGTCTTC
TATAGGATGGAACACAGGGCCCCTAATGAAGGAGCTAGAGAAAGTACCAAAGGATCTAAAGGGGTCTGCAACCCTATAGG
AGGAACAATGATATGAACTAACCAGTACCCCTGAGAGCTGTGTCTCCAGTTGCATATGTAGAAGAGGATGGCCTAGTAGG
CCATCAATGGGAGGAGAAGCCCTTCGTATTGATCATATACCCCAGTACAGGAACATGCCAGGGCCAGGAAGCAGGAGTGG
GTAGGTTGGGGAGTAGGGAGGCGGGAGGGTGTAGGGGGCATTGGGATAGCATTTGAAATGTAAATGAAGAAAATATCTAA
TAAAAATTTAAAAAAAAAAAAACCTTGCCAAATAATTCCAAGTGGGTTGTCCTGGTGCTCTTTGTATTCTGTTGTTATTT
CTGCTTCCTCAAGAGGGGATTTCCTAAGTCTGAAGTGTATAATTTACTCAGTTGAGTTCATGTGAACCATCTCCTCATTT
TAACTGGTAAAATGAAGGCTGGGTGGGGCTTGTGATTAGGCAGTGGAAGGGAAAGGTGGGGCTGGAGGTTTTAGAGGATA
GAGGAAGAAGAATGGAGGAGAGGAGCGGGGAGGAAGATGGAGAAGCACCACATGTCCTGGAGAAGTCGCAAGTCAGAAGA
AATCTCATAGCTGAACAAGGAATCTCATAGCTGGGGAGTAGATTAATGTAGTGGTAATCTAACCAATCTAGGCATACAGT
TTATGAATATCGTAACTGAGTTGTGTGTTATTTTCACAGGCTTTTTGGGGTTGGAGATTTACTGCAACAGACTGGCACCC
ATTGTATTGCTTAGAACCCATCTAACCTGAGATAAAATTCCAATTTCCTCTCCCTGAATAAATAGCTGGGGCAGCCATC
TAAGGCTGCAACAGCGTGTGGATTGAAAGCTGAGTGGGTCTGAGTGGTCTACAGAGACCTGCAGAGAGGCTCCCTTCCCA
AGTCCCAGGTAGACAGCTACAGCCAGGGGCAGCACCCCAGCCTGGAGCCTAGCCAGGGACAAGGAACTTTGGATGTCAGC
CGCCTGCGAGGTAGTTTTCTGGCAGACACAGTGAGCTTTCTTGGCATCTATGCCTGAGGGTGCCTTTCTGTGCTTGCTTC
CCAGTATCTCTTGGGTCACAGGACATGGGACAGCCAGAACTGTTCTTCCAACTGCTTGTTCTCCAGGCTGCCTGAGATCA
GCACACAAGTTGGCTGCTGCAACCCAAACAAAGAGAGCCTACTCAGAAGCATAGAAGCTAGAAGCTGAGAGTATTTCTACAGTCAGC
CCCTTCCTACCAATAAAAAGAATTGGAGAGGACTGCAATGTCATATATACATATACATACATATATATATATATATAT
ATATATATATATATATATATATATTTGATTGATAATACATATACATAGATATACATGTGTATATGTATATATATATATAT
ATATATATATATATATATATATATATATATATATTTGATACTTAAATTGAGAGAGATAATAAATCTTTTGGGCTTATATGGA
ATGATATTTTGAATATCGTAACTGAGTTGTGTGTTATTTTCACAGGCTTTTTGGGGTTGGAGATTTACTGCAACAGACTG
GCACCCATTGTATTGCACATTACAGGGATAGATTTAAAGGAAAGAGACACCTAGTCACTTACTGTTATTACAATTTTTAT
TCTCTTGATTTATTATGTCTTCCCATAATAAATGGAGCCCTAGATTAAAAAAAAACTTATGTAGCTTAGAAAAGGGTTTCA
AGGAAACACTAAAACGAAAAGTTCAGGATTTTATAACTCAAGATAAGCACCAGAAAGTGGAGAATAAAGGTTGGAGAAAG
AAGCTGGAAAAGATGCTAGAAAAAAAGGATGCAGAATTTCATAGATCAGACTGAACAGCAAAGAGATAGAGATAAAATTT
GGAAGCAGAGTCTCGAGGATAATTTATGAATGCTACTACGTCAAAATAAGGCTGATTCTACAACATCAGAACTACAACAT
TAAAATTATGGAAGCAGGGCCTAGAACAGAAGATACAGAAATTTATAGAGCAGCATAAAGAGCAGAAAGACACCTCTCAA
ATCCGTTGTCTGGAGGGTGATCCTATCTCGTGCCCCACGTCGGGTGCCACTTGACCCGTCCAGCAGGTCCAGTTATTCGA
GGGTCTCGAGGGGACCTTCTCCTAAGAACCAAATGGAGGGAGGGAAAGATGGAAACCAAGTGCAAGAACAACACAGAAGC
AGTCTGAGTAGCATCAAGGTCTCTCTGAATTCAGCAAGGCTCAAGGCTTAAATGCACAAGCAAAAGGGGAAGTACTTCTC
AGCAGGAGGAATGGGGCAGGGGAAATGCACAAGCAAAGGAGGAAGTACTTCTCAGCAGGAGGAATAGGGCAGTGGAAATG
CATAAGCAAAGGAGGAAGTACTTCTCAGCAGGAGGAATAGGGCAGTGGAAATGCACAAGCAAAGGAGGAAGTACTTCTCA
GCAGGAGGAATAGGGCAGTGGAAATGCATAAGCAAAGGAGGAAGTACTTCTCAGCAGGAGGAATAGGGCAGTGGAAATGC
ACAAGCAAAGGAGGAAGTACTTCTCAGCAGGAGGAATAGGGTAGTGGAAATGCATAAGCAAAGGAGGAAGTACTTCTCAG
CAGTACTGAGAAATTTTTCTTTTGGCAACTACATGGGGAAGCAGGAACTTGGTGGTATCAGGGTACATCTTGTGGTCAGG
ACATCTGGTGCTGATTTAGGGCTGGAACATTCTGTGGTTGTTCTTGGAACGGTGCGTCGGTGGCCTGCTTTGACCCCCTT
TACTATTCGGGGGGAGAGGCCCAATTCGGAGATCAAGACAATAGAGTTGCTTTAATAGCTCCCAACAACCATTTATTGCT
CTTGCAGAAGACTGGGGTTTCATTCTGAGCACCCATACGGTGGCTTACACACCCATCCCTAATTCCAATTCCAGGGATCC
AATATACTTTTCTAAATTCTTCTAAAACTAATCATGCATGTAGTATACATACATACATGCATACATATATACATACATTC
TAGCATACAGGCCAACAAAGCACTGTTACATGTAAGATAAATAAGACTGAAACAGTCATTTTAAAAGTATATACACATCG
CTTCTCAGCCTTTTGGCTAAGTCAAGTGTAAAAGTATATACAGACCCCATACCCTATCTCAGTTTCCCCATTGTCTGCTG
CAGACTCTCCCTGCTCCTCTATCTCAGCTGTGAGTACTCACCTTTCCAGATGCCATAAAATGTGTCCATGAAGCAGAGAG
GTCCCACCTGAGTTCTTTCAGCTTGTCACAGCAATGCCCTCTGCTCATCACTACTCACACTGCAGTGCTTATCAGTAGCA
TGTCAGAAACAAGCAGTGCTGGAGAGATGCTGAGAGACCATGTCTACAGCACTGCTGACACAGAACCATGCTCTGAGTTC
AGAATTTTGCATGAGTCTCACCAGCAAATATTATACAAAGATGTTGTGACAGGCTGTCAGGAAACTTCTAGAGAAGTGAG
AGGAGGAGGTTAGTAGTGGCAGTTGGGTGTCTCCTCTCAAATGAAACTGCCACTCCTTGTAGTTCTCTGACTTGGTTTGC
ATAAGCTTACTGCCAGGCTTAGTTTAGGGACGTTTTGGGTAAGAAGCCCTTGGGATATTTTGCCTGTTAAAGTGGCATGA
GATAGGGCAAGCCGAGGCTTGTGCTCTGAGTTGTTTCTTGTGTTGAGAGAAGGTTTACCTTGTCATCAGCTTGGGGATAT
TTAAATGGTTGCAAATATGTCATTTTCACAGGGATCCTTAAAGGTGCTGAAAATATTCACATAAAGATATCTTGCCACTT
AAATGCAGGCTGTCCTTTCCAGCATGGGGATATCTGGGTGGATAGGAGCATATTACATTTTACACTTAAATACAGCTTTG
TAGTCGCTGTACAATTTAGCCTAAAAAGGTGTTAGTTCTACATCAACATCGTCATCTGCATCAATGTATAACCATCCTGT
TATGGAGACAGTGATTTTGCTCTTAAATCTAAGATGATTTTTGCCCAAGACAAGTTCACAAACACTCTCCCTTATTTTTC
TAAAAATTCAAATGACTTTATGATATTAAGTTTTGTGCTTGGGTCTGTTTGTCTACAAATGGACTGTAGTAGTTTGTGTT
TATTTTATTTTGTTTTGTTTTCTTTTCTTTTATTTTTTAAATAAAAATACATCTTTATGTGTAAAAAAAACAAAGAACAA
```

AACCCACCATTTTTCCATTATAAAAATCCATAAAAAGAATCGTATGAATTAGAGGCTGCATTTGCAAGGTGAAAGGCTGA
AAAGGTTGAATGATGATTAAAACTGTACAAATTCTTTTTAAACTAAGTGGAACCTAAATTAAGTAAACACTGTTAGTCAG
TGCTATGTAACGTGGCTATAAAATTCAGCAAGTAACCACAAAATTCTTTCCTTCCCTTTTTCTACTTCTCTGACCCAGAG
CTTTTCTCTGTACTCAGACTGGTCTTAAACTTTTGATCCCTCTTTTTTGGGGCAGAGGGGTCAAATTACCCTTTCACAGG
GGTCTCATATTTGACATCCTGCATATCAAATATTTACATTATAATTCATAATCGTACCAGAATTACAGTTATGAAGTAAC
AACAAAGTAATTTCATGGTCGAGAATCATCACAATGTGCAGAACTGTATTAAAGTGTCACAGCATGGGCAAGGTTGAGAA
ACACTGGTCTAGAGGCATTCCTTGTGGTGATAAAGGTGGCAGTGGGCATTATCTTTCTGTCTCCACACCACTAGCAAAAT
GTCTCCATATGTAAACATGTAATATTAGACAGCCTGAGTCTGTAGTCCTGAGGCAGGATCTGGCTCCAGAACAAAGCATC
TCTTATTCCCTGAGAGGCAAGAAGTGTGTTAGCTCTGACTGTGATCCCCAACTCTCTCCTTTCTGACTCTGTCTCCTAG
AGATTCTGTCTCTGGGCTTTTCAACACACAGACTCCTCTGACCTTGCTCTGGGTGTCCCTGTAAATAATGCCCTCAAAAC
TCAGAGTTGTAACACCATGGGGCTCACCTACCCTGAGGATCACTGTCTGATGTGTGGTGTTCAGGGCAAGAAAGAATTGG
TAATATTTTCTTTTTGTTGTTTTTGTAGTTGTTGTGTGTGTGTGTTTGTGTGTTTTTTTGTTGTCATCTTTTATTTCCAA
ATTGATTCCCGCTGGACAGTAAAACTGGTCTCATCATCCAGCCCCTTTTGGCTTTGTTAAAATCTCTTTGTCTACAGCAT
TAGTTTCACTTCATTACAGTCTAAAGAAATGCCTTTCTTACAATGCTTCAGTTTGAGTCAAAGAAATTTGCACAGGAAGG
AGGAGCTCAAGCTACCGGATTGGCCGGTTGGGTTGAGTTGGCTAAATCCCATATATTTGTGCTTGTCAGCTTTCAAGTTT
CAATCCTATAGTGGTAACTCAGCTTCTCAGCTCTGAGATGAACATTACAAGGGCTTCCCTACCTATGCTCAGCACCACTT
GCAGCTGCAGGAGGGAGAAGTGGGTATGTATTCAAGAGTATTTGAACCAAACTATAAGAGCAGAGAGGAACTTCACCAGG
GACAGGAAGGCAAAAGAGCTGAACCTTGAACAAAAGAGTAACAGAAGGGCTTCTGTGAGCCTGTCACTGTGGGCCTGCAG
AGCAGGAGAATGCAGTTGGGATTAGCTATGAGGTTGTTACATTAGTTATTCTATTGGAGCATACAATAGTTCTCAGGCAA
GAGAAATGAACAGTGAGTCACCTTCTAAGAGCCAGAGCTGTAGCTACAGCTTTCTTTCTTTGTACTTATCTTGCTAGTCC
ACTCTTCTCAGAGATCTGGTAAATTACAGTCTGGTGGATTACACCAAATTGCTGTAGTAAAAATTTGCTTTACATGTCCC
TGAGTGAAGAAACTCAGACAACAGCTTTGCAATGTGCATAGTGACAGAAGTTGCCTGGGAAGCTTGGAGCTTGTGTTTT
GCAAATCCATTGTAATTAAAATTGAATTGTAAGGAGGTGGCATGGGGTGGGGGTGGGGGTGGGGGGCTGAAAACTTCCC
TGGACCAATTCCTTCCCATTTTGAGTTCTTGATAAGTTACAGAAAAAGAATATTCCCGAAACACAGACTGTTGAGAAGGA
TTCAACTGCATGAATTCAGAACTCTTCAGCTGGGGAACAGGGTACCCGTAAGTATAGCTTTACAAGGTAAGTCTGGTCTT
GAACTTTCTAACGAAATTCAAGACAGTCTATCAGAAGTAAAGTGGGGAATAGCTTTATAAGGTTCGTCTGGCCTTGAACT
TTCTTATGAAATTCAAGATAGTCTATCAGAAGTAAGTGGGAAATAGCTTTACAAGGTACGTCTGGCCTTGAACTTTCTAA
CGAAATTCAAGACAGTCTATCAGAAGTAAAGTGGGAAATAGCTTTACAAGGTATGTTTGGCCTTGAACTTTCTCCAGTGT
TAGGAGCCTTTTTGTTCCTTTTCACATGTTATCAAGTGGTTAAGGCAGGGCTGAAAATTCTGGATGAAATTCAGGGCAAT
CTATCAGAAGTAAAGCGGGGAGAGAGAGTAGGAGCAAAGAGGAAATATGGTACACAAAATAAGTATACAGGCCTTTCCAC
GGGTCTTGAACCCGAGGAAAAGTTTAGGTCAGGTAAGAATACATGGGGAGAGATTAGAAGGAAGGAAAAGAAAAAAGAAA
AGAAAAAAGATCAATTAGCGGAGGTTTCTAGGAGAAGGAGCCTATACTCATCACTAGATGAGCTCAAGGAGCCAGCTCTT
AGTAGCTCTGAATCAGATGAAGAATTCTCCTCTGAAGAAACAGACTTGGAGGAGGAAGCAGCTCATTATGAGAGAAAAGG
GTACCAGCCAGATAAAATGCTAGTTAATCAGTTAAGGAAAAAGCCAAAAGTGGCTGGTGAAGGCCAGCATGCTGCTCGGC
CTCCATGGTAGTCTGCTTCAAGGTCATAGTGCACTTCTGCCCTATTCGGAGCCCCGCCCTGGGTAAGGCGTCAGCCCGGC
GCAGAGAGGCAATGGACAGAGAGGCAATGTGAAGACTCATTCATTCAAAAGGAGGAACAAAGGAAAATGCAACAGGCATT
TCCTGTCTTTGAAGGAGCCAAGCAGTGTTGGTGCATGCCATTAATCCCAGCACTTGGGAAACAGAGGCAGGCTCTTGCAG
CCTACAAGGTCTTATTGTCCACACTGGAGTTGTAGATCAAGATTATAAAGGGGGAACTTCAGGTTCTCTGTTCTTGTCCT
CAAGTTGTCTTTTCTATATCACAAGGGGACAGAATAGCTCAACTAATAATTTTGCCAAGCCTACATGGCTGTTTTCCTTC
CTCTGGTATTCCTCGAGCTGCCAGCGGGATTGGTTCTACTGAAAATGATTCTGATTACTTAATAATGCCTTTAGATTGTT
GTCAAATTTTATCTACGCCTCATGTAGGGGTAAACCCTCGTGGCGTCAGGCCATTACAGGTCTGGCAAATGGATGTCACG
CGTATTTCCTCCTTTGGGAGAAATCAATATTTACATGTCTCTATTGACACCTGCTCTGGCATCATGTTTGCTTCTCCTTT
AACTGGAGAAAATGCCTCACATGTGATTCAACATTGCCTTGAGGCATGGAGTGCTTGGGGGAAACCCAAACTCCTTAAGA
ATGATAATGGACCAGCTTATACATCTCAAAAATTCCAGCAGTTCTGCCATCAGATGGACGTGACCCACCTGACTGGACTT
CCATACAACCCTCAAGGACAGGGTATTGTTGAGCGTGCGCATTGCACCCTCAAATTCTATCTATTAAAACAGAAAGAGGG
AGTCGAGGCTCTACCCCGAGTACCAAGAGTGTCTATGTCTATGGCACTCTTTACACTCAATTTTTTAAATCTTGATGCTC
ATGGCCATACTGCGGCTGAACGTCATTGTTCAGAGCCAGATAGGCCCAATGAGATGGTTAAATGGAAAAATGTCCTTGAT
AATAAATGGTATGGCCCTGATCCTATTTTGATAAGATCCAGGGGAGCTGTTTGTGTTTTTCCACAGAATGAAGAAAACCC
ATTTTGGATACCAGAAAGACTCACCGGAAAAATCCAGACTGACCAAGGGAATACTGATGTCCCTCGTCTTGGTGATGTCC
AGGGCGACAATAACAAAGAGAGAGCAGCGTTGGGGGATAATGTCGACATTTCCACTCCCAATGCCGGTGATGTATAATGC
TCAAGTATTCCCCTGCTTTTTTACCACTAACAAGGAACTGGGTTTGGCCTTGTTCAGACAGCCTTGGCTCTGTCTGGACA
GGCCCAGACGACTGACACCATTAACACATTGTCAGCCTCGGTGACCACAGTCATAGATAAACAGGCCTCAGCTAATGTCA
AGATACAGGGAGGTCTCATGCTGGTTAATCAACACATAGATCTTGTCCAGGAACAACTAGATGTATTATGGCAAATAGCT
CAGCTGGGATATGAACAAAAGTTTCCGGGATTGTCTGTTACTTCCATTCAGTATGAGAAATTTACTAGGGCAGCTAATTT
GTCAAAAAGTCTTTCTCAGTATATGTTACAGAATTGGACGACTGAATTTGAACAGACCCTTCGGGAATTGAGACTTGCCA
TCATTCAGGTCAACTCCACGCGCTTGGACCTGTCCCTGATCAAAGGATTTCCCAATTGGATCTCCTCAGCATTTTCCTTC
TTTAAAGAATGGGTGGGCGTGGGATTACTTGGAGATACACTTTGCTGTGGATTAGTGTTGCTTCTTTGGTTGGTCTGTAA
GCTTAAGGCTCAAACTAGGAGAGACAAGGAGGTTATTGCCCAGACGCTTGCAGCACTAGAACATGGTGCTTCCCCTGATA
TATGGTTATCTATGCTTAAGCAATAGGTTGCTGGCCATTCAGCTCTTGCACCCCACGAGGCTAGTCTCATTGCACGGGAT
AGAGTGAGTGCGCTTCAGCGACCCCGAGAGAGTTGCACAGCTAAGCACTGCAGTAGAAGGGCTCTGCGGCATATATGAGCC
TATTCTAGGGAGACATGTCATCTTTCAAGAAGGTTGAGTGTCCAAGTGTCCTTCTCCCCAGGAAAAACGACACGGGACCA

```
GACCAGGACCCCTCTGGGTGATGAGCCTGGAAGGAGGTTATGTGTACGGCTCCTTTACCTGCACACTGGGGATTTGACCT
CTATCTCCACTCTCATTAATATGGGTGGCCTATTGCTCTTATTAAAAGGAAAGGGGGATATGTGGGAAGCCGCCCTCACA
TTTGCCGTTGCAAGATGGCGCTGACATCCTGTGTTCTAAGTGGTAAACAAATAATCTGCGCATGTGCCAAGGGTAGTTCT
CCACCCCATGTGCTCTGCTTTCCCCGTGACGACAACTCGGCCGATGGGCTGCAGCCAATCAGGGAGTGACACGTCCTAGG
CGGAGGATAATTCTCCTTAAAAGGGATGGGGTTTTGCCATTCTCTCTCTTGCTCTCTTGCTCTCTTGCTTGC
TTGCTCTCTTGCTCCTGAAGATGTAAGCAATAAAGCTTTTGCCGCAGAAGATTCCAGTTTGTTGCGTTCTTCGTGGCTGG
TCGTGAACCCGTGTAAGTGAATATAATGGGGTTTTCTACTTCAGCCTTCAGAAAGTAAGCAAAATTATGTTTCTTGTGTT
TCAAACTGTATATGCTTCATTATATTGTGTTCCTTTATTTTCCTTTTTCTCTCATTTCTGTTTCTTCATCTCTTTTTGTC
ATTTATTGAAGAATACTGGATTTTGAGAAACAAGATTGATATTTATCAAAATACAAAATTCACAGCAAATGTTAATGCTT
ATCACTTAAGCCAAGTATTCTGACTGAAACTATTATTATTATTACTGATAATGATGATGACAATTGATTGATTCTTCAGC
ATTTCCAATAATGTTAGGACAATGACATCTCAAGAAGAGGAAATATGAAGCATTTAGTCAATAGAAACTGAGTTTAGATA
AAGAAGCAACAGGTTAAAATCTTTGAAAGCTTGAACTTGCATATTGTTTTTGTGAGGTCATTAAGAAATCTGGCAGTTGTG
AGGGTGGTGGTGGTGCTTGCCTTTAATCCTAGCACTTGGGAGGCAGAGGCAGGCGGATTTTTCAGTTCGAGGCCAGCCTG
GTCTACAAAGTGAGTTTCAGGACAGCCAGGACTATACAGAGAAACCCTGTCTTGAAAAACCCAAAAAAAAAAAAAAAAAA
AAAAAGGAAAGAAATAAAGAATTCTGGCAGGCAGCCTTAGCTAGTTAACGAATATATTAATGCATGCGTATCATGGTGGA
TTATCTTAGGGTAAGGAGCTAGGTCTGTTTTCTAGCTGTCAAGCTACAATAGCCAATGGTGTCAGAATCCTGGGGAAGAA
TAAATTTTAATCTTTGAGTTATATATTAATTAAAAAATGACAGAGTTTATCTATAGTCCAAACAGTTCCCAGGTCCCTAT
GATCTGCATGGCAACTTGGGTGGAGTCACTAGGGCTTTCAAGCCCAGAAGAAGGAAGGCCTTGCCCTCCTTGGAACAGGT
AATGCCTGGAATGAGAAATTATTGACCTTCACTCAGGCAATATAAGTCACTTGACTCTTAGAATATCCAGTTTTGTCCAC
AGTGTTTCTCAGTGGTTGTCATACCACAATCTAGGCAGTCTCTTGCAGCTGTGTCTATATCTCCCGAGTCCTTGGGGATA
TGCTGTTAGGCTTTGGGAGATTCTGTCTGTCATAAACGTATTAATTATTAGCAGACTTGTGAGAAGATTGAAGGCTATAT
CGATTGGTCATATCTGAGTCAGAAAACTTATGTTTGTTAAATTATTTGTTTGAGAATGTATATAGTAAATATAGCACAAT
ATAATTTGCTGGAGCTGGGGAATTTTGACCCGTTCTGTTGCTGGTCCACAGAGCGAGACAGGCATGCATGCATATTTTGT
TAAGGCACCAGGCAAGTGCCTAGTCCAGGGGCGTGGCATGTGCATGCAGCCTGAATAGCACAGATTGTAAGGAGTCACAA
TAACAGCTTGCATGCCCCAGGTAGCTCAGGTGGTGATGGTGTCATGGGGTTGGGGTGGTCCTGGGGTGTGCAGGGCTCAT
ACACAAGTAGGTGGGGTGGGGCTTCCTCCTAGCAGCCAGAAGCTCTGGCACAGTGCTTCCAGCTGCAGGCTGGTGCTGCC
CTATCTCACCTAGTGGGTAAGGGAAGGGAAGTACCTGAGTTAGCTTTCAGTCTCCTAGCAAATTGTGCAATATCCCAACA
GCAGGCAGCTCATGCTCATTTCATGAGTAATGAGCAGAGTGTGTGGGAGGAGAGTGAGAGAGAAGAGAGAGAAGGCAGAG
AGAGAGAGAGAGAGAGAGAGAGAGAGAGAGAGAGAGAGAGAGAGAGAGACCCATGCACAGGCACGTGTGCATGGGA
ATGAGCACAAATAGCAGGGCAGACAGAGTTCCTGGCAGGAAGCAAGGGCAGGCCAGACCTGCTTTTCACTTGGTGAAC
CAGGAACAGACCAAATAGCAAGAGAATGAGGGTATTCCCAGCTGGCAGCAGCCACCACCGCAGGGGGGCTGTTAGCAAGC
AAACTATGGAGGGGTGGGGTGGGGGTGGGGGTGGGGATGGATCACTATCTAGAGAGTTGTTTTAAAGTGCCAGTTTAGT
GGCCATGTAGAGATAGTCCAGGAAATCCCAGTAAATAGGGACAAGGCAGCGAAGACCAACCCATGGATGAGAATATTATC
CTACACCTACCAGCATTTTAAGGATACTATTAATAACCAGAGAGGCTGACAGCCAGGCCAGACAACCAGGTCATCACATG
ATGGGCTGGGCCTAAGCTCTGGGCAGGTGCCTTGAGCAACAGAGAGCATTTCACCAGTCCTGGGATTTCTCAACAGAGAG
AAAGGAACCTTCCCAGAGGAGAGGGGGAAATCTCACCAAAGGGAAAGTGTCCTAGGGAAGGACCAGGGTAGCTCCAAGCT
CAAGAACTCTCCCCGGGGACCGTGGGGACATTTACACTGGCCAGTGTGGGCAGGGTGCTTACCAGTTCTCACAGGTTTGG
GCTAGTGAGCATTGGCTCTCCATGTGGTTGCAGGAAGGATGCAGGGAGGATGTAAACCTGTTGAGTTTCTAAATCTGAGT
CTCAGTACCAAATGTTGTGCTTATAAAAAGATAAGGAGGGAAGGAATTTGATTGGTGGATGTGTAGCAAGGTATGGTGGA
AGGGGCTGCCTCTGTGGGCCCATGGATAGGCATCCCTTCCCCCTGAGGAACAAGCCACATGCATATAGTATATTCAGGGC
ATGGGAGGGGAGTTGAGAGGGTAGGAGAGAGAGAGGAGAAAGAGAGAGAGAGAGAGAGAGAGAGAGAGAGAGAGAGAGAAG
GAGAGAGAGAAGACTAGAGTAGAGGCTGACCATGAATGCATAGAGAAAGGGGGGAGGGGAAGGGGGAGAGAAGGGGAAAG
GGCCCCAGAGAACATGGTGAGAGCAAGAAGGCAAGAGAGCAAGAGAGCAAGAGAACAGGAGGAGGGAGCAGCCCCTTTTA
CAATGAGTCAGGCACACCAGGCTGTTGCCAGGTAACTGTGGGGCAGAGCTTAGACAAAAGGTGAACACCACTGATCTAAG
AATAGTTATGTATTCAGAAACTTGAAAGCAGCTCAAAATAAAATATCTGCCTATATTTTGCAGAAGGTTTTTAAAAAAGC
CAATGGTAAACTTGTAGATTAGATTAGCTCAAGCAGAAATCAGTTGGTGACAATTGAGCTATCAGTAAAAAACAAACCAA
AATACTACAACTGTGTGATCAGACTCTTAAGCTCCTAAAATAAGCTGGACTACAGCTGGTTTTATGGCTTACTCTAGAGC
TTGCTGAGAGCCCAAAGCTGAACAACATTTGGACAGAGTGACAGATGATCAGGGGCAAGTATATGGTCAAATTTTTCCTT
TGTAATGCCCCTGCCTCAGTGCTCAGTCATATTGATTAACACTGTCAACATTGGTGGATATTTGTGTTGTGAATTTAACA
AACATTGGTAATTAAAAAAATATTTTTTTATTATTCTTTAATCTTCTTTTACAATCCAGTAGTTATCCCATTCCCAGTCTG
CCCTCCAACTGTTCCTCATCCCATTCCTACTCCCTTGAGTTCAAAAGGATAGCCCCACCCCTCAACCCCCACACCACCAA
ACCTCCCCACATTCTGGGGCCTCAAGTCTCTAGAGGGTTAGGTGCATCTCCTCTCACTGAGGCCATACCAGGCAGTCCTA
TGCTATATGTGTGTCGGGGCCTCATATCAGCTAGTCTATGCTGCCTGGTTGGTAGCTCAGTGTCTGAGAGATTTTGGGGG
TCCAGGTTATTTGAGACTGCTGGTCTCCTATGGGGTAGTCCTTCTCAGCTCCTTCCAGCCTTTCCTTAATTCAGTCACAG
GGGTCAGCAGCTTCTGTCCATTGCTTGGGTCTAAGTGTATGCATCAGGCTCTTTCAGGTGTTATTTGAGGGCAGTCGTGC
TAGGCTCCTGTCTGTAACACACCATAGCTTCAGTAATATTGTGTCAGGCCTTGGAGCCTCCTCTAGAGCTGGATCCCAAT
TTGGGCTGGTCACTGAATCTCCTTTACCTCAGTCTCTTCTCCATGTTTGTCTCTGCAGTTCTTTTAGACAAGAATAATTC
TGGGTCATAGGTTTCGACCCCATCCACTTGATGCCCTGTCTTTCTACTGGAGGTAGAGTCTACAAGTTCCCTCTCCCCAC
TGTTAGGCATTTTGTCTAGGGTCCCTCCCTTTTAGTCCTGATTGTCTCTTAACTCCCAGGTCTGTGGTAATTCTAGAAGT
CCCCAACCCCCACCCCTGGCTAACCTCCTGCATACCCAGATTCCCTGTTTCTATTCTTTCTGCCTGCCCTCAGGGCTTTG
ACCCTGTTAACTCACTAATACCTGATCATGCTCCCCTTTTCCCCTCCCTGTCCCCTCTCCAACCAAGGTGCCTCCTCCCT
```

```
CTGCCTCTTGTGGTTGCTTTCTTCTTCCTCCCAAGTGGTATTGAGGCATCCACACTTGGGCCCCTCAGTTTGTTAACCTT
GACTTCTATCCTGGGCATTCTGTACTTTTTTTGGCTAATATAAACTTATTAGTGAGTACATAGCATTAAGAATCTCTAAA
GTCACTGCCAATACTCATAATGATGGAAACACAGATTTTCATGTCTTCTTGTATGAATATGTGGGTAGGAGGGCCAGAGC
ATATAATATGTATATTCTCATTTGCAGAAAGTAGTGATGTGTTTTCAACTGCCTTTTTCCAATCCCAGTAGCAATGAACA
TATATAATGCCATTGCATGTTCAAATATTTGGCCCATTCTTATTCTTTTTTTCTCTTTAAGTAAATGAAATCTCCAAACT
ATCAGAATGCTCTAGATACAACTTCTTGTTGATCTATATCTTACAAAAATTGCAAATTAACTTTGTCGAACTCGGAAAAG
AAGGATTTCTTTTTTTGTTTTTGTTTTTGTTTTTTGTTTGATTTTTGAATTAGAGCAAAGTAGGCATATTTCAGTTTAA
GCTAATGTGGATAAGGATTTTGATATTACACATGAGATATTTTTATTCAAGGCAAGTTCACAGAGATTTTTCTTTTTAAT
TTTCCTCAGATAGTTTTATAGCCCTAGGCTTCATGTTTGAGTTTCCTTTTGTAGGAGGAGGTGGGAGAGAGTGAGACACA
GTACAGTGTGCGCAGCAAGGCCAGGGTCAACCTCCTGGGATAACTAACTATAAGTTAGGCCCACATGTCAGACTTACTTC
TCAAGTTATTTTATTATTTTTTTTGTATTTTTCTTTATTATTATTATGATTATCATTATTACTATTAATTAGGTTCATG
TATCTATGATTTCACCAAAGCCACACTTATATCTTTGTTTCTGTCACTCTGATTTGGTGGTATCACTTCTGCAAATTTGG
CCTCATTTTCTAAATTGTTCAGCTAGACTGATTCTATTCCCTTTTCATGAAATTTAAAATTTACTTTGTGCTAGAGAGAT
AGCATGTGGCTAAAAGCACTGACTACTCTTTTGGGGAACCTAGGTTCGGTTCCCAGTGTCCTCATGGCCCCTCACAACCG
TAATCTCAGCTCCAGGGAACTCAACATCCTTTCTTGTCTCCTCAGGCAGCAGGCATGCATACAGCACATACACAGACACA
CATGCAGGCAAATTACCCATATGCATTATATACAAATACATTTTAAATGAATAAAATTACCTTTCTGATTTTTTGAAATG
CCCAATATCCACAAATGTGTTCCTTCAATTAATTTCTCACTTGCTAATAGATTTTGTTATTTTAGACTAATATTCGTTTT
TCACATGATTTTGGTAGGCTTGTTAAAACATCCCCATATTTCATCCAATAGTTATGCCAGTGTGCTCTATTACACTTATG
TGCCTTTATTAATTAATGTATTTATTGACTAGTTTCTTTGAGGTTGATCCTTACATAGTCCAGGTTGAGTTCAAACTTCC
AATGTAGCCAAGGCTAGCCTTGTCCTCCTGACTCCAGCTTCTGCCTCCCTAGCACTGGAAATATAAAGTGTACCAACCT
GTTTGTTTCATTGACTGGAACAGGAGTTACACAGGTGGTTATGAGCTGCCCCTTTAAGAGCTGGGATTTGGAAATCAACA
AGGGTCCTCTAGAAGAGCATCAACTGTTCTTAACCCCTCAGTCCTAACCAGTATATTCTGATGGAACAAAATCTATGGAT
AGCTTGGTTGTTGTTTGCTTTCAACCTGATCTTTACTCCTTCTGTTTCTTGCCTCTTCATTTACCCTTAGTTCAATGACT
GGACGACTCCATGTTTTCTTTTCCTCTAGTCTTCTGTGAGTGATGACATCATTTATAGACCAGGACCTTCTGTAAAGGGG
TTTGCAGTGGGTGAATACAAGCAAATCTACACATAATTGTTAAATGTTTCTTGAGATTGATATTCTCATATTAATATTT
TTTAAGTAAAATGGTTCAAGAAAAATGTCTCAGAATCGTCTCCCCCAAGTATTCTGCTCTGCTTTACTGCCTCTGTGGAG
TGATCATGGTCCTCACTGTAGCTGTAGTTGCTCATTTTTTTGCTTTCAGAGGCAAGTGACTTATTGTACAAATTCTGTGG
TATTCTGTCCACCTTAACATTGTCAGTTATGTTCAATGACCACTGTGAAACAGGCATTGTGGGAAGGACTCTAAAGAAAA
CACAGTGGAAGTTCCTGTTCTTTGGAAACTTAGGTTTGATCAGGAAGATGCTGAGAAGGAATAGCACAGGCTGTGATGGG
CACAGGAGGCCCGGCTCATCATCCCTGGGAAGATAACACCCAGCAGGCTCTACACAGAGATGCAGAGGACATAGGTACAC
TTGGGGAAACTGTCTAAGGTAGAGAACAAGGAAAGAAAAACTCCACCGAGGAACCTCAAGGAAGGGTAAAGACAGAGAAG
AGTAATGTGGTCAGGAAGGTACAGTGCCATGATCCATGATCCATGAGTTTAGTTACCAGGCTCAACTGTACTTTGAATGT
ATTAAAAGCAGTTTCTGAAGTCATAAATTTATAGGTTTTAGTACCTCACTGTTCAGAATAGTGTAATAAAATCTTACACT
GTCCTCTTAATTTTGTGAATCATCCTTTAGTGCAACATGTCTATTCTGTATACAGTACCTACCCAATAGTTCTCACTACT
GTCTCAATTATTAGGATGGCTGTCAATAGGTGTCCCTACAGAGTGATGACATCATTTACAGACCAGGACCTTCTGTAAAG
GGGTTTGCAGTGAGTCAATACAAGCAAAATCTACACATAATTGTTAAATGTTTCTTGAGATTGATATTCTCATATTAATA
TTTTTAAGTAAAATGGTTCAAGAAAAATGTCTCTATTAGTAACCCCTATTGTAGTAACTGTTGACCCAAATCTCAGATA
GTGATGCTAGTATAGAAATGCACTTCCTGGAAGCCTACTGAAAGCCAGCATCTGAGACTGAGTGGGACATAGGCCCACCA
TGCTCGGGGGAGGATTCCTACAGATCACCTGGGAAAGCTACAATCACATTCACAGCTCACAGCTGAGCTCAGGAGGGTAT
TCCAAAAAGAGCCAAAGCATGTGTGCTTTAATTCATTGTATTGTCTAAATTTTAGTGAGAATGACAGAACAGATCTCAAT
CAAAAATACCTATGCTGCTTGCCCGAGAAACTGGATTGGATTTGGAAATAAATGATTTTACTTTTCTGAATACTCAAGAA
AGGGGACATTCAGCCAGACCTTTTGCATGGCACACGAGGTCCAACTAGATCAGTTTGACAACAAGGAGGAGCTGTAAGAA
ATAGTCAGGGATTGGTTTGTCTGTTGAAAATTATATTACCTTGAGATAGAGATAAAGCATGAGGAAGAATCCCATCCCAA
GCATGTGGAGACATGGGGAATGTGTGAGTGTGTACCAATTGCTGATGCTAGACTTCTGAGTGGAGTCCTGAGACATTCAA
GAAATATTCTCTCACATAGTGCTCATAATCATCTAGAAGGTCAGATATGCCATTTTACTGGGATACCTGGTGGTCATGAG
TGTTTTCCTGTTAAATGACACATGTTGCATACTGTTAATAACTGCAATAGAAAGTTAGCCCAGAACTATTCTGGCCACAG
AAGAATGTATCATTACATTTGATACTTTAATTAAAATGGCATACTTTACAATGACTGATGTCAAGTGGATCCACTGAGAT
CGGGAGACTAATATACCTCCAAATTCTGTGACCCTCAGTTACACCCTCCTGTTACCTCTATAGGTAGCTGTGGAGTAGAG
AGACTCCAGGATTATATGAAACAAAGACACCCATGTATTTTGGCTTTTTGTGTTTTATGACAGAATTTCCTAGGGAGATA
CGAGGGGACTTTTGACTACTGGATCGGCCTGCACAGAGCGTCGTCTAAGCACCCTTGGATGTGGACAGACAACACTGAGT
ATAACAACATGTATGTTTTCACAATGTCTTCCCTTCTGTGTTCATGCATTGTGACATGCATGAGTTGTGGCTATGAGGGT
TGAATGGTTCCATGAAGCCAACTGTACTGGAATGGAAGAAAACCAACTCTTGGACTGGAAGGTGTGCCGTGGGCTTAGGT
TCTGTGCCTCATAAAATAGCCTATCCCTAAAAAAATTCCACATTCTAACAATTTTTTTTTTAATTTTTTCAAGACAGAGTTT
CTCTGGATAGCCCTGGCTGTCCTGAAACTCACTTTGTAGACCAGGCTGGCCTCAATCTCAGAAATCCACCTGCCTCTGCC
TCCCAAGTGCTGGGATTAAAGCGCATGAGCCACCACACCAGGCACATCCTAACAAATTTAAATCAATTGGATACTCTCCCA
TCTACTAGCCATCTTCTTCAAAGCATTTACATAGTGTTATTTCCCTCCTAATATTTAGCACCATGTTGGCATCTGAGGTA
GATGTTGTCTGCTATCATCTCTTTAAAATCTTTCCTTGATGATAACTGCCCTGGCATGATGTAGTCAGAACTGATGGCCA
AGGGCTCTAAAACCATAGGAAAGTACTACAGAACTGCAAGCCTGAGAATTATCAGTCTTCTGTTTGCAAGGTTGTACCTG
TTCACAGAAGCAGAGACTCCAGGGGCATTGAGAGTGCTCCTAAGGCCTTGTCATCAATGATCACTGGGGACAGCTCTTG
AATGTGAGCATAGCCATTTTGAACAGACCCATTTCATTGGAAGGGTAAAACAAAGAACCTTTCATATTCATAATTAAATA
CTTTCTAGCAATTTATTAAGTGGTATATTTTCTCTCACTTTTTTAGATGTTCAAAAATACTTCTAGAGGGCATTGAAAAT
```

```
TACCCTTGTGAAGCTACCCAGTGCTTCATATCATTTGAACATGGCAGCAAACATTGTGCACACATTCCAGGGTGAAACAC
AGATATGTGACAGAGGTATGGTGATCAAAATCAAATCCTGAGGATGTGCAAGTCTCTAGGTGCAAAAGTAGTGCATGGGA
TTCTGGTGGACGCTGCTGATCCATATCATAAATATGTGGTGAAGATGAGTGAGATAGGAGTGAATGCAATGGCAGACTCT
GCAGAAATCTATTTATTTATTTATTTTTGGCTTTAGGGTTTCCATCCAAGGAGTTGAAAAACATGGTTTTTCTGAGTGACA
ATGGGATCAGCAGTAGCAGGGACTACATAAATAGGAAGTGGATTTGTAGCAAGTCCAGCAACTAAACTTTATAGTACCAG
CAGTTTTGTACCTGGGTAGAGAGCACGTCATAGCAATCATGAGGAACACATAACAAGTTTTCTACTGTTGCTGCTTCATT
AATGATCTGCTAAATTTATCATCACAAGTAGTGGGTAATACATGGTGACATCAAAGATGAAAACTCTCTCTGAGGACTGT
TGATGTGTGTACTTGGGAGATCATGAAGAGTTCTACATTAAATAGGTATTCAGCTGATGTGGGGCATTGGTCTCTGCACA
GACAGGCTGGTCTCCAGGTGAGCTGAGGTCTGAACCCTGATGGTCATAATTCACTTTCATGACATGGTAGGTGTTCCTTC
ATGCTCCTTGAACTCTGGCCTCTGCCTAAGTTACTGCCCCCCACACCCTCCACAANNNNNNNNNNNNNNNNNNNNNTCTGC
TCCATCAAGATCTACTGCGCAATCTGGTCAGTGTTGGGAACCTCTTCCTTTATCCCTCTCTCCCATAACCCCGGGGATAC
AGGGTGTGGCCCTGGGGCCCCAAGGTCAGGGGCTGCCCCTTGACCACCCCTCAACGAGTGGGTCAGAGGCTTGGATGCC
CATCCGAGGCTGAGTGGAAAGTGTGTGGCAGCTGTCCTGCCTCCACCTGCCCAGAGCATAGGAGGAACTCTGGCTGGACA
TAGGCCATTTTCCACTCCCTGTGTACCAGGGTCCCCTTTTTTAGTTCCCAACAAGCTGGTACTCATATCTAAATATAAA
CTAAATCACATCAGTATTAGTAACATCTACTTTTTTTCTCTTTTCTTATTATTTTATTTATTTGCATTCCAAATGCTGTC
TCCTCATGGTTCGCCTTTCCAGAGTTCTTCACTCCATACCCCCTCCCCTTTACTTCTGAGGGAATGTTCTCCTCACCACC
ATCCCCTCACCCATCCCCATCCTACCCCACTCATTCTCTGGGGAATTAGATCTCTACAGGATTAGATGCATCCTCTTCCA
CTGAGATCAGACAAAGAAGTCCTCTGCTACTTATGTCCTGGGGGCCATGGACAAGCCTATGTATTCTCTTTGGTTGGTGG
CTTAGTCTTTGGTCCTAACGGGCCAGGTTACTTGACACTGTTGGTCTTCCTATGGAGTTGCTATCCCCTTCATCTCCTTT
AATCCTTCCCTGAACCCGTCCATAGGTGTCCCTGACTTCAGTCCAATGGTTGGCTGTATCTGTATCTGTCTAAGTCAGCT
GCTGGTAGAGAATCTCAGAGGATGCACAAGTCTGAGTGGTGTGAAGTCTAACTGTCCTCTGAGGGGACCATTACAGGATG
TAAAGAAGGAAGATGCTGTTATTCCATTATCTAAACTAAGGCACACAAGTGATAAATGTATTGCTTGTCTTGACTTAATT
GACTACATGCAGCTTGCCCACTCCATTGTTCCCTTATTGTTGTAAAATTCTGGGCCAAAGTCTTACAGGGGACCTGGCTG
CAAAGACTGATTCAAGGTCCCTGAAACCAGGTGTCCCTGACAAGTCCCAGTCACTAATGTTAACTTCCTTGTCTTAAGCC
CTTGTGTATAAATATGCTTTGTCAATGCAACAGTCTACCCTGCTCCCCCTCACTTTGTGTTTCCATTCAATAAAATGTCG
TATAATCTTCCTTCGGAGTTGTAGTTTAGACCCTGAATTAGCAGCCTCCTGGAGCATTCAGAAAACTAAGCTTTTATTTT
TCATCTTCTTTCTCTAAGGTGAGTTTTCTCAGCTTCTACCCTGAACCCTACAAAAGTTCTAATATTCTGTGCCTGTTTTG
AATTTGTTTTTATTATTGGACAAACACAAGCATATCTCATTCAGGAAAATGTGTTTCTTAGTGTTCTGTTTCTATTTCAT
TTCACCTTGTTATTTTTAAATCCATTTCATTTTAGATAAATGGTGTGACTCTGTTCCTAATATAAAGAAATACTTTCTC
ATATCGAAATTACTTTTCATTTAGCAGTTTTAATTATTGTATTTCTAGCACATGTTAATCTCAAAGATGGAAGGAGAAAA
ATCACTGACCCAAATCATTATGGCCAAACTTATTGAAGCAAGTAATTAATTGAAACAAAATCATGGTTACTAAGATGACA
TAACAAGATAGTCATAGCAACTGGTAGTCATACCAAGTTAGTCATAGATGGTCATATAACCCGTTGAAACTAAGGTAGGG
TTTTTTTTTTTTTTATTCCCTCCCCCCCAAAGTTCGGTTTTTATTATGGTTATAAGCCAGTTCTTGAAGCAAAGACATGATT
GCCATTTCTGGAACATACAGAACCACTTGTAGTTAAAGTTCCAGGTGGGATAAAGTCCAATCCTTGAGAAACAGGTTTGA
CTATAAACAGGAATGAACCTAGTTTGTCTTTACTATAAGATGGCTTTTAAGCCTAAGATGGAGTCAGGGTGATTCTTCNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNTGAACAATGAATTAACTACA
AGGTCTGAGCTATAGGTTTTGTTTGTTCGGTGAATAAAGTGAAGACAGGACAATTCCAAGGTATGCATAAGGGGGAGACT
TTTTATTGTAGATATGTGAGAGAGTACAGGCAGAGGCATCTAGAAGAGTCTACAGCAGGGAAAGAAACTAACAGACTGAG
TGTGGCTAGCCTATTCAGCGTGGCCAAGAAAGATGAGAGAAGGAAGGAGAATGAGAGAGGAAGACATGAGACAGAGAGAG
AGAGAGAGAGGGAGAGAGAGAGAGAGAGAGAGAGAGAGAGAGAGAGAGAGAGAGAGAGAGAGAGAGAGAGAATCACTAAAAT
GGCAGTGTTGTGTAGGGATGAGATGCTGACAGAAACAAGGCTAATGAGCTGGAGAAGTTTGTGGGAGGGGTCAGGGTAAG
AAGAGCTGTGAGGAGCCAAAGAACTAAGTGAGCCTGGAGGCCAGCTTGCATTTTGTTATGATAGTAGACATGAAAGTCAG
CCATTTGTCCTGAGTTTGTTTTCTACCCGACAGTTGGTTTATTTGAGTTTGGGATTTTGTTTTCTTTTTGATATTTTCTT
TTTCTTTTTCTTTTTCTTTTGCTTTTTGGCTTGTTTTTCGTGTGTGTGTGTGTGTGTGTGTGTGTGTGTGTGTGTGTGTGT
GTGTGTGTTTAGCTTGCTAGGACACTCTGGTGAATTAGAGGATGTTGCTGTAGAACATGCTCCTTATGTCCCTGAGTGAA
GAAAACTCTAACAGCACAGCTTTCAATGTGCACAGCTAAGGAAGTTACCTGTGGTACTGGGGCTTGTGTCTTGAAGATCC
ACAGTGCTTGAAAAGAAAGGGGAGAGGTGAGGTGCAGTGTGAGGCTGTAGCCTCCCCCAGCCTTTAGCAGGCTGATTCAG
ACCTTGCTGCCACTGTGAAGGAGACCACTTGGAGTGACCTAGATGGTTCTATTGTTCTGTGCTATGGCTGAGTGGCTGAA
CCTATCTTCCTGAGCTATTCCTGAGAGAGCTGACAACCTGTGGAATTGAAGACCTAATGCTAAGAGCTCTGGCAGACTAA
GTGCTAGCTACTTAGCAACAAAGCATCAAGAAGCCTGGCAAGTCAGGGGATGGGCCTACCCCTTTGTAAGCACAGACTCT
TAGTAAACATTGGGCCTTGATCAGAATTTTGTCTTGGTCTCCTTTCTATTTTTTTTATTAGGTATTTTCCTTGTTTACAT
TTTCAATGNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNCCCACTCCCCCTTTTTGGCCCTGGCGTTCCCCTGTACTGGGGCATATAAAGTTTGCAAGTCCAATG
GGCCTCTCTTTGCAGTGATGGCCGACTAGGCCATCTTTTGATACATATGCAGCTAAAGACAAGAGCTCCCGGTTACTGGT
TAGTTCATATTGTTGTTTCACCTATAGGGTTGCAGTTCCCTTTAGCTCCTTGGGTAATTTCTCTAGCTCCTCCATTGGGG
GCCGTGTGACCCATCCAATAGCTTACTGTGATCATCCACTTCTGTGTTTGCTAGGCCCTGGCATAGTCTCACAAGAAAGA
GCTATATCTGGGTCCTTTCAGCATCTGGGTTCTTTCCAGATTCTGGCTATTATAAACAAGGCTGCTATGAACATAGTGGA
ACATGTGTTCTTCTTACCAGTTGGGACATCTTCTGGATATATGCCCAGGAGAGGTATTGCGGGATCCTCTGGTAGTACTA
TGTCCAATTTTCTGAGGAACCATCAGACTGATTTCCAGAGTGGTTGTACAAGCTTGCAATCCCACCAACAATGGAGGAGT
GTTCCCCTTTCTCCACATGCTCGCCAGCATCTGCTGTCACCTGAGTTTTTGATCTTAGCCATTCTGACTGGAGTGAAGTG
GAATCTCAGTGTTGTTTTGATTTGCATTTCTCTGATGATTAAGGATGTTGAACATTTTTTCAGGTGCTTCTCTGCCATTC
GGTATTCTTCAGGTGAGAATTCTTTGTTCAGCTCTGAGCCCCATTTTTAATGGGGTTATTTGATTTTCTGGAGTCCACCT
TCTTGAGTTCTTTATATATATTGGATATTAGTCCCCTATGCGATTTGGGATAGGTAAAGATCCTTTTCCAATCTGTCGGT
GGCCTTTTTGTCTTATTGACTGTGTCTTTTGCTTTGCAGAAGCTTTGCAATTTTATGAGGTCCCATTTATCGATTCTTGA
TCTTACAGCACAAGCCATTGCCGTTCTATTCAGGAATTTTCCCCCTGTACCCATATCTTCGAGGCTTTTCCCTACTTTCT
CCTCTATAAGTTTCAGTGTCTCTGGTTTTATGTGGAGTTCCTTAATCCACTTAGATTTGACCTTAGTACAAGGAGATAGG
AATGTATTAATTCGAAATCTTCTACATGATAACAGCCAGTTGTGCCAGCACCATTTGTTGAAAATGCTGTCTTTTTTCCA
CTTAATGGTTTTAGCTCCCTTGTCAAAGATCAAGTGATCATAGGTGTGTGGATTCATCTCTGGGTCTTCAATTCTGTTCC
ATTGGTCTACTTGTCTGTCACTATACCAGTACTATGCAGTTTTGATCACAATTGCTCTGTAGTACAGTTTTAGGTCCGGC
ATGGTGATTCCACCAGAGGTTCTTTTATCCTTGGGAAGAGTTTTTGTTATCCTCATTTTTGTTATTCCAGATGAATCTGC
CGATTGCCCTTTCTAATTCGTTGAAGAATTGAGTTGGAATTTTGATGGGGATTGCATTGAATCTGTTTGATTGCTTTTGG
CAAGATAGCCATTTTTACAATGTTGATCCTGCCAATCCATGAGCATGGGAGATCTTTCCATCTTCTGAGATCTTCTTTAA
TTTCTTTCTTTAGAGACTTGAAGTTCTTCTCATACAGATCTTTCACTTCCTTAGTTAGAGTCACGCCAAGGTATTTTATA
TTATTTGTGACTATTGAGAAGGGCGTTGTTTCCCTAATTTCTTTCTCAGCCTGTTTATCCTTTGTGTAGAGAAAGGCCAT
TGACTTGTTTGAGTTAATTTTATATCCAGCTACTTCACTGAAGCTTTTTATCAGGCTTAGGAGTTCTCTGGTGGAATTTT
TAGGGTCACTTATATATACTATCATATCATCTGCAAAAAGTGATATTTTGACTTTTTCCTTTCCAATTTGTATCCCCTTG
ATCTCCTTTTGTTGTCTAATTGCTCTGGCTAGGACTTCAAGTACAATGTTGAATAGGTAGGGGGAGAGTGGACAGCCTTG
TCTAGTCCCTGATTTTAGTGGGATTGCTTCCAGCTTCTCACAATTTACTTTGATGTTGGCTATTGGTTTGCTGTAGATTG
CTTTTATCATGTTTAGGTATGGGCCTTGAATTCCTGATCTTCCCAAGACTTTTATCATGAATGGATGTTGGATTTTGTCA
AATGCTTTCTCAGCATCTAACGAGATGATCATGTTGTTTTTGTCTTTGAGTTTGTTTATATACTGGATTACATTGATGGA
TTTCCATATTTGAACCATCCCTGCATATCTGGGATGAAACCTACTTGGTCAGGATGGATGATTGTTTTGATGTGTTCTTG
GATTCGGTTAGCAAGAACTTTATTGAGGATTTTTGCATCGATATTCATAAGGGAAATTGGTCTGACGTTCTCTTTGTTGG
GTCTTTTTGTGGTTTAGGTATCAGAGTAATTGTGGCTTCATAGAATGAGTTGGGTAGAGTACCTTCTGTTTCTATTTCGT
GGAATAGTTTGTGAAGAACTGGAATTAGGTCTTCTTTGAAGGTCTGCTAGAACTCTGCACTAAACCCGTCTGGTCCTGGG
CTTTTTTTGGCTGGGAGACTATTAATGACTGCTTCTATTTCTTTAGGGGATATAGGACTGTTGAGATCATTAACCTGATC
TTGATTTAGCTTTGGTACCTGGTATCTGTCTAGAAACTTATCCATTTCATCCAGGTTTTCCAGTTTTGTTGAGTATAGCC
TTTTGTAGAAGGATCTGATGGTGTTTTGGATTTCTTCAGGATCTGTTGTTTTGTCTCCCTTTTCATTTCTGATTTTATTA
ATTAGGATGCTTTCCCCTGTGCCCTCTAGTTGGTCTGGCTAAGAGTTTATCTATCTTGTTGATTTTCTCAAAGAACCACC
TCCTTGATTGGTTGATTCTTTGAATAGTTCTTCTTGTTTCCACTTGGTTGATTTCACCCCTGAGTTTGATTATTTCCTGC
CTTCTANNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNCCTGCCCTCCGCGGAGTCCCGGAACCAAGGGTGGCTCCCGCCAG
AGCCTGAGGAAGAAACATCTCCCGGCCGGGCGGACCCCTGTGCTCTCACCAGGAAGGTGGCCGGTTGTCTCTTGGTCTCC
TTTCTTCTCTCTCATACAGCTCTGGCCTTCCACTCAGGAACCTGGTTCCTGTGGCTTCAGGCAGCTACATGAGGCTGGGT
ACCCAGAAGCACCAACAAATTCTCTGACGTTTTCGGTTTATGGTAAATCAATTCTTGGCCTTTTGGTGAAGATCAAGTGT
AGGATTATGGTAAATTATGGAAATGAAGAATCCTGTGCGAGTTCTGTTTCAATCTTCTGAAGGAAATCTGTCTTCTGTGG
TTTGAAAAATGTCTGTTTCAGAGTTTTGTGTTTATTCATTTCTTCCCTCTTTCCTTACTTTAACTCCTGTTTACTTGCTTG
TTTATTTTAGCAAAATTATATTGGGTATCCTGGGAAACAATGTTCATACATCACAAAAGAGCACGTTTGTGAGGAATGTA
```

```
GGTTTTTTTTTAAAATTTAAGTCAAATATACCCATACCTGAAAAAATTTTCTTTCTTTTCCCTTGCAACAACTCCAAGTT
TTATGAGAGAATGTAGCCCAGTCCAGGAATTCATCTTATCATTCTATCTGGGTTTGTATATACTTGACATTTTCCATTTC
AAACACCTAGTGTTCCCATTTCCTCTCTGTTCCACAGTTACCTGGCAATGGCCAGGTATGCCTGACTCATTTTAAAAGGG
GCTGCTTGCCCCCTTCACTTTTTTTTCCTCTTGCTTCTTGCTCCTGCTCTGTCCTCTCACCGCCCTTCTCTCTCCATTC
CCCTCCTCCCTCTCTGCCCACATACTCATGACCAGCTTCTACTATTCTACTCTCTCTGTCTCTCTCTCTTTGTCTCTCTG
TCTCTGTCTCTCCCTTTCTGTCTCTAATATCCTCCCAAATCTCCTCCTCATGCCTTAATGAACTCTATTCTATACTAAAC
TGTTGTGTGTCTAGTCCCTTAGGGGGAAGGGATGCCTCAGCATGGGTTTGTAGAGGCACCACCTTCCCACATACCTGAGT
ACACCTCTACCAACCATATTCCCTCTTACCTTATCTTTTTAATAAAACACAACATTTGGTGCTGAGATTCAGATTTAGAA
ACTCTGCAAGTTTGCATTCTCCCTGCTACCACATGGAGAGCCAGTGCTTGCTCTCTAGCCCAAAGTTGGACAACTGGTAA
GCCCTCTCCCCCTACTGGCCAGTGTTAATGTCCCCATGGTCCCAGGGGAAGAGTTCTTGAGCTCAAATCTATCCCTGTGC
TGCCCTTGAGGATGGAGACATTCAGTCTTTTCACTGACCTTCATTCTTGGACATTTCCCCTTGAGTGAGATTTTCCTCTT
TAGTCTGAGATGGTTCCTTTCTCACTGCTGAGAAATCCCAGGCCTGGGTGAACCACTCTCTGTTGTTCGTGACACTGGTG
CAGAGCTTAGCCCTCAGACCACTATGTGATAACCTGGTGATTTGGCCTGGCTGGAAGTCTCTCTGGTTATTAATCGTATC
CTCAGGTCGCTGTTAGGTGTAGTTAATAACCCTACTACCACTCAATACATTGGTCTTTGCTTCCTTGTCCTGACTTACTG
AGATGTCCCAGTCTAATTCACAATATGCAGCAAGCAATATTTCTACATAGTCACTAAACTGGTACTTTAAAACAACTCTC
TTGCTAGTTATGCTCTGCTCCCAATTCACCCTGTGAAAAATGGGTCTGGCTCTGCCCCATGCAGGGGACTTTGCTTTCCT
GCACATGTAATCATCTACCAACCATATACTTGCACTCTCTCATGTGTGGGGCTCCTGTACTCTGCCTGCCAATGCTGCCA
CCACCTGTGCCTGGCCTCTCAACCTCTGCCAAAGCCATGAGGAAAGCAGTTTTCACCATTCCCCCTTGCACTTCCTGTGC
TCTGCCCAGGACTCATGAAGTGAGCTGGAGGTCCACTGCTGCTGCAGCAGGAAAAGCACTCACACAGCCCTGCACTGCTG
TTGGTGCTGCTGTCCCACATCTGAGGGAATGGCAGGACTGCTGCAGGCATACTTTTATTCAGGTGTCTGCTGCCAGGTTC
TCCCATCTCAGTCCCTCACCGCCCCTGCCTTTTAAAATCCACCTTGAGTGGAGTTGTTAGAAGATGGACCTCTACTTCAT
GTGCTTCCTTTCCCTGCCCCACACCCACCACAGACTGTGACAGGCAGGGTGGGGCAGTGCCTGCCCACAGCCAGAAGTA
CTGGGCTGGTACTTCTGGCTGCTAGGAGGAAGTCCCGCCCCACTTGCTTGCCTACCTGCCTTCCCTCGATCCTGGTGCTG
CTGGGCCCACTTCATCCATGACCCATAGCAGGCTGGGTGCCCAAGTGAGCCACAGCACAGTCCTGATTCTTTGACTCTGT
TGCCTCCACCATTGTGGCTCCTCACCATCTGTGCTCTTCAGGCTGCATATGCACGTCCCAAGCCTTTGACACATGCAATG
CGTGCCTGTCTCTACCTTTGCTTACTGGTCAATTCCCACTGCCTGCCCATCTGCTCAATTTGTGCCACAGGCCTGAACCC
ATTTGGACACACTCACTTTTCTCACCACAGTCTCAAGCCACCACTGTCAGCTCTCTCATTTCTTTACATTCTGGTCTACA
GCTACTCTAAGCCAAGTGTTTCTCTACTAATGCAAAATGTGTTTTTCTATATTATACAAAGTGTTAAACTTACTTAATAA
TTAAGATGTTCATTTCTATCCTGCCTTGGGAAATAATGATTTTTACTATTACTACTAAGTTATGCTCTATTTACTATATA
CCTTCTTAAACAAATAATTTAACAAACACAAGTTGTGTAACTCAAGTATCACCAATCTATACAGTTCTCAGTCTTCTCAG
AAGTATGCTAATTATTAATACATTTAAGTTTATGACAGACAGAGACTCCCAAGATAATGGGTTTAGCTATAAGAGAGTGC
CTGGATTGTGGTATGATAACTACTGAGAGACTGTGTGGACAAGACTGGATACCTAAGAGTCAAGTGACTCATATTGTCTG
AATCAAGGTGAGTCATTTCTCATTTCACATGTATCTATTCCACAGGGGGNNNNNNNNNNNNNNNNNNNNNCCATCTGTGCTC
TTCAGGCTGCATATGCACGTCCCAAGTCCTTTGACACATGCAATGCGTGCCTGTCTCTACCTTTGCTTACTGGTCAATTC
CCACTGCCTGCCCATCTGCTCAATTTGTGCCACAGGCCTGAACCCATTTGGACACACTCACTTTTCTCACCACAGTCTCA
AGCCACCACTGTCAGCTCTCTCATTTCTTTACATTCTGGTCTACAGCTACTCTAAGCCAAGTGTTTCTCTACTAATGCAA
AATGTGTTTTTCTATATTATACAAAGTGTTAAACTTACTTAATAATTAAGATGTTCATTTCTATCCTGCCTTGGGAAATA
ATGATTTTTACTATTACTACTAAGTTATGCTCTATTTACTATATACCTTCTTAAACAAATAATTTAACAAACACAAGTTG
TGTAACTCAAGTATCACCAATCTATACAGTTCTCAGTCTTCTCAGAAGTATGCTAATTATTAATACATTTAAGTTTATGA
CAGACAGAGACTCCCAAGATAATGGGTTTAGCTATAAGAGAGTGCCTGGATTGTGGTATGATAACTACTGAGAGACTGTG
TGGACAAGACTGGATACCCAAGAGTCAAGTGACTCATATTGTCTGAATCAAGGTGAGTCATTTCTCATTTCACATGTTTC
TATTCCACAGGGGAAAACAAAACAAAACAAACAAACAGTTCTTTGTAAAAGGCTCTTGGTTTTCTGGGCTTAAAGGCCAG
ACTGATTCTGCCTAAGTTTCCTTGGATACCTGTTGCGGATATCCCTGTGGCTAATTATATTTGATGTTAATTCCATTCTC
CTGTGAGGGGCTGTGAACAAGGAATGAGTCAGCACTGAGGTGATTTCCTGTGAACCTTCTTCCCACCTTAATTTATAAAT
AAAGGCTAGAGCTGGTGATTGGGCAGAGGGGAAGGTGGAGCTGAAGGATTTGAGAGAGAGGGGGGTGGAGAAAGACAGAG
AAACAGAGAGAGAGAGAGAGAGAGAGGAGACAGAGAGAGACAGAGAGAGACAGAGAGAGAAAGAAGATGATGGAG
GAAGAGGAAGAAGGGAAGAGGAGCAGAAGCACCTAGCCTGGAGGAAACTGCAAGTTATTAGGGGTCTCTGATACTGGGAT
GATGGTAGTGTAGTGGTAGATCTGCCCAATCTCACAGCATGTATTCATATTAACTGAGCTGTGTTTTCATTGCCTGGGCT
TATTTGGGTTAGAGAATTACTGCAACAGATACCACAGAGAAGTGTTTAGCCAGTGGACTATAGATGAACTCTGTCATTTT
AAAATTATTATGTGCCTGCTAGATTAAAATTTCTTACCCAGATTTCTGACTACATTGACAGCTAAGGAAACTGTACCTTG
ACAGCTAAAAAACAGACCTAGCTCCTTACCCTATGGTAATCCACCACTATATTTACTCATTAGTAAATTCGTTAGTTAGC
TAAGACTGCCAGATTTCTTATAGACATCACGATAAACGATACACAAGTTCAGATATAAGCTTTAATAGATGTAATGTATT
GCTTTTTTTTTAACCTAAATCAGTTTCCATTGACTAAATACTTCATATTTCCTCTTCTTGCTATGTCATTGTCCTAACA
CTAATCAAGAGAGTTCTTTTTTTTTTGTTTTTTGTTTTTTCAGACAGAATTTGGTAAACTGAAATATGTACATGTAATCAG
GATTTCAATGGGCAACTGCCTTAACTTCAGGAAAGGCTGACTCTGTGATTACACATTTGTTAGAAGTTATAGCCATTATG
AGAATACTCATAGAAACTAAACCTGACAATGTCTGGCATAGGTCACTAATAAGATAAAGCAGTTTTTATTACATATTATA
ATATAAAACACATTCCAGGCCTACCACATAATCCTACCGGACAAGCGATTGTGGAGAGATTTAATTGAACTATAAACAAG
ATGCTTAACAGGCAAAGGGGTGGGGGGGAATAAAAAACCCAGAGATAGATTACATAGTGCTTTATTAACTTTGAAATTTT
TAAATTCTAGTGAACAACAAAACTATGGCTGCAGAAGGACATTGGGTTGTGGAAAAAAACTGCTGAACTAAACAAGCCTA
TTTATTTTAAGCATATTCTGCATTCAAAATGGAAACAGGAAATGTGTTACAGTACTGGAGGTGTTACACCTATATTTCCA
CAGGAAGAGAAAAGCTGTGGGTTCCTTCCAAATGGATAAAAATCAGATATGACCAAGGGAGAGCCCTGAAGAGCTTTGCT
```

```
GCAGACAAGAAGGAGACTAACAAGATTAACAAAATAGGTGATGTATATGTGGTGATGTATATATGTCTCTGTACACAGAG
ACAGAGCTGGAACCTGCTGAGACCTACATGTCTATATCCAGCCAATAACACTTATTGAGTACAAAATTCTCATTACCTGT
TATTACATACACTCTATAAGATGGCGTGAATGGAAAATTGGATTGTAGTTTGATTTTATGCGATTAAACTAATCTATGAA
GAAAGACAGTTGTCTTCCTTTATTGATCTTTACTAACAAATCTGTGAATCACGCAGGCTGCATTCGAATGTTTTTACAGA
GTTATGCGTTGCTTGTGAGGTTTGTGTGTTGCAGAATAATAGAGGAGAAAGATAGCTCTGACAAGACTCACACTCTGGGA
TGTTGAAATTCTGGGGCTTTCTATTCCAGCTCCTTATTTGTTTCTATCTCAATTACATTGAAGATGTTTCCTCTAAAGAC
TTGCTTCCAGGATATTTCCAGAACAGCTAGCTCTCCCATCCAGCCTTTCAGACTGTTATAGTCAGGATATCAACTAAGCC
ATGAACTTTCTTAGCACATAGAGACTAGAAGGCAAATGATGCCAACTAGCCTTCCTGTGACAAAACCAATTTTCCTATTT
CCCTTTGGGCACCCAGAAGGGAATTCTTTACCCCAATTCAGCTAAAAGAAAGCAAAGAAGATTGTTGTCCCAGTCCTTT
AGGTTGGGGTGGATGGTTCTGGTTATTTTATGAATTGGAGATATGTTGTCATTTTAAGGGATAATTTGCTAATGTTGTAG
CTTTGGACAGGGAGGAAACTAGAGAGCTTAGACTCAGGGATTTCTACCTTTCCTTTCCTCTTATCTATCCTTCCGTCTTA
GGTAAAGTAAAGAGGAGTAGAAGAGAAAAAGCCAGATATAGTAGTATCATAGAAATTAAAGGAGTAGGCAAATAGGGATA
TGTAATCAAATTTAGTCTTAGACAAAACAACGAATAGCCACATCTTACATTGGTTGAAGTTTTTGTAATTGATGCAAATT
AAAGTTATACATTTACATTGGTACACAATTTATAGATTGATATAGATCTAAGGTTTTCTTTATTGTGAATTTTTTATATT
GATGCAAAGTTTGAAATTAATGTGCTTACTCTCTGACAGGCATTGTGTCTATGCAACTTATGTCGACTATAATGCTTAGA
TGCAGTCCTTCTAATAACTGACATTACAAACTGCCTAGGATGATTGAGTAATGCAAGTTAAAGGCCAAATAGTAAACTCA
TAGTTACATTATATTCTGATTTGATTATGAGTTACTCAATTAGAAATGACTAAGAATAGTTAGGATTAACAGTTCAGCT
GTACTTGACATAGATAGTCTTCAAAAGCATCAGAAGTCCACAGAATATGACATTTAAGGCCATTTCATTATTAAAGATCA
TTTGAGTAGAGACATGTCTTCTCCTGACAATAGCCCCTTCTTCGTTCAAAGAAGAAATTGAGCATCAGTGGGGTTTCTCC
AGTTGTGGCCAGATAGACTCTGGTAAGATTTGCCCCAATTCATCTATAGACAAAATACTGTCCAGGAAAAGGACACACT
ATGTGGAATTTTTGACTGATAACACTGTCAAGACAGGGTAAGCAGTTCTTTATAATTCTGCTTTACAAAGGTCTGTCAGA
TAATCCTGGCCAGAAGGCTGAAGACTGATGCTATAACATTTTGAGGAATGAGTGACGGTCCAGGTTAGTTAGCTGTCTCT
ACAAATTGGTTAAGTTTTGGAAGTTATTTTTTGTGATGTTTAATTTGTTCATGGATTCTTCTGGGGGTTGAAGATAAGTT
ATAGTTTCATAGTACAGGCAAGTTGTTTTGTATTGAGATAGATGATATAGATTTGAGAGGATGGTTTTCAGATGGTGAAA
GAAGCTAAAACCAGAACACAATTCAGGTAGAGATTTGCAAGTTCTTTAAGTTAGGATTGGTGGTAGAGTGCTTTATCTAA
ATTGACACATATGATGGCCTGGGTGTTAAGTGTATCTTATACTTCATAATTTGCATGACTGGGATGATTGAGATCAATTT
ATACCTGAGAGAAAAAAGTCTTTTAATTGGACAAAAACGAGGAATGTTGTGGGTTGCCCTGGGGCAGTTTGTACTCTGAT
GTTAATTCTGCTTTCTTAAGAGGGGCTACCTTGAAAATGAGTGGATCACATACTCAAGTGATTTCATGTGACCATTCTCC
CCATTTTAACTTGTAAAATAAAGGCTAAAGCCTATGATTGGGCAGTGGAAGGGAAATGTGAGGCTGGGGCTTTTAGAGAG
TAGAGGAAGAAGGATGGAGGAGAGGCGGTTGGAGGAAGATGAATCAGAACCACATGGCTTGGAGAAGCCGCTGGTACAAG
GAATCTCATAGCTGGGAACAAATTAATGTAGCTCTGTATCTACCCAATCTAGCCACACACCTTATAAATATTGTAACTGA
GTTGTGTGGGTTTTGCACAGGCTTATTGGGGTAGAGATTTATTGCAACACAAGAACACATCAAAAGGATCATTCAACACG
ACCAAGTAGGCTTTATCCCATGGATGCAGGGATGGTTCAATATTCCGAAATCCAACAACATAATCCACTATATAAACAAA
CTCAAAAAATATCATATAATCATCTGATTAGATGCTGAAAAAGCCTTTGGCAAAATACAACACCCCTTCATGTTAAAATT
ATTGGAAAGATCACGAATTAAAGGCACACACCTAAACATCATAAAAGCATTATACAGCAGAACAATGGTCAACATCAGGT
TAAATGTAGAGAAACATGAAGCAATCTCAATTATAATCAGGGACAAGACAAGGCTGCTCACTCTCTCCACATCTATTCAA
TACAGCAGTTGAAGTTTTAGCTAGAGCAATTAGACAACAAAAGGATGTCAAAGGGTTACAAATGAGAAAGGAAGAAGTCA
AGGTATCGCTATTTGCTGATGATATGATAGTATGCATAGTTGATCACCAAAATTCTAACAGAGAACTCCTACATCTGATA
AACAACTTCAGCTAAGTGTCTGAAATAAAATTAACTCAGACAAATCAATATTCTTCCTCTACAGAATGGATAATCTGTTT
TTTTTCTTTCTTTCTTTTTTTGGTTTTTTGGACAGGGTTTCTCTTTATAGTCCTGGCTGTCATGGAACTCATGGTGTAGA
CCAGGGTAGACTCGAACTCAGAAATCTGCCTCCCTCTGCCTCCCGTGTGCTGGGATTTAAGGCTTCCACCACCATGCACA
GCTAAAAGTAAGCATTTTCAACAAATGGTCATGGTCTGTATGTAGAGGAATTCAAATTGATCCATTTTTATNNNNNNNNNN
NNNNNNNNNNNCATAAAAGCATTATACAGCAGAACAATGGTCAACATCAGGTTAAATGTAGAGAAACATGAAGCAATCTC
AATTATAATCAGGGACAAGACAAGGCTGCTCACTCTCTCCACATCTATTCAATACAGCAGTTGAAGTTTTAGCTAGAGCA
ATTAGACAACAAAAGGATGTCAAAGGGTTACAAATGAGAAAGGAAGAAGTCAAGGTATCGCTATTTGCTGATGATATGAT
AGTATGCATAGTTGATCACCAAAATTCTAACAGAGAACTCCTACATCTGATAAACAACTTCAGCTAAGTGTCTGAAATAA
AATTAACTCAGACAAATCAATATTCTTCCTCTACAGAATGGATAATCTGTTTTTTTTTCTTTCTTTCTTTTTTTGGTTTTT
TGGACAGGGTTTCTCTTTATAGTCCTGGCTGTCATGGAACTCATGGTGTAGACCAGGGTAGACTCGAACTCAGAAATCTG
CCTCCCTCTGCCTCCCGTGTGCTGGGATTTAAGGCTTCCACCACCATGCACAGCTAAAAGTAAGCATTTTCAACAAATGG
TCATGGTCTGTATGTAGAGGAATTCAAATTGATCCATTTTTATCTCCTTGTACAAAGCTGAAGTCTAAGCAGATCAAGGA
CTGTGGAGAGCCGGTACATGCTGCGAGCAATCGCATGCGTGCTGCCAGTAATCTCTGAGGAGAGCCGTGTGTGCCGCGAG
CAATCGCCATTATAAGATGGCGCGGGCCTCCGCTGTGCCTAACTAGTAAACAAGTCCTGTACGCAGGTGCAAGAGTGAAT
TCACTCCTAGTCACTCCCATTCTCGGGGTGTAATAGTGGGGTGATGGGCAAGCAACGAATCGGGAGCTGTCACGCCATAT
CAGGTGCTGAAACGTCACGCTGCGGGTTATAAAAGCAGTGCCATTTTCCCGGTTTGGGATCTTCCTGAGAAGCAAGCAAT
AAAGCTTTTGCCGCGGAAGATTCTGGTTTGTTGCGTCTTTCTTGCCGGTCGAGCGGGGACGCAATAAGTGGCGCCGAAGAA
CCCGGGAACTGTCATCACACCGGCGCAAGGGAAGATCCCTCGTTCGCGGGGCAGAATCAGAACCGTGGGACAAAAGGCTC
TACAAGGTACGTACAATCTTGAAATTTCTCCAGAGTTAGAAGCCCTTTTGGATTGTTTGNNNNNNNNNNNNNNNNNNNNNA
TGGAAAGTCCGGGGAAGGACTGAAGGAGCTGAAGAGTATTGCAACCCCTTAGGAAGACAACAGACTCAAACAACTGGA
TCCCCAAGAGCTCCTAGGGACTAAACAAACAACCAAAGAGTATACATGGATGGGTCCATGATTCCAGCTAATTTGTAGCA
GAAGAGTGAGTGATTCATCATGTGTCAGTGGGAGTGGAGGCACTTGGTCCTGTGGCAGCTTGATGCCTCAGAGAAGAGGA
ATGCTAGAGGGGCAAGGAAGGAGTGGGTATGTGGGTGGGGAGAGAACCCTCTTATAGGCAAAGGAGAGGTGGAATGAGGT
```

```
GAGAGGTTCATGGAGGGGGAGATCAAAAAGGGGAACAACATTTGAAATGTAAATAAATAAAGTAATTAATAATAAAAAAA
GAAATTGTTGACAACAGTAAAGTTTTGGTGAGGCAAAAGAAAGGAGTCAGGCAAAGTAGAGAAAGGTTGAGTATGGCTA
GTGATCTTCTGAAATGAATAGAGTTGTATTTGAGATGCCCCCACCCCCAACCCCCCATCCCCTGTTTATATAGAACCAGC
GTCCAAAGGCACAGCTGAATGGTAGACTTCACACTTCTCACGTACAAGTTTGATCTGAATTTGATCCCTAGCACCACACA
CAATAAAAGTGCCTTCCACAAAAAAAAAAAAAAAAAAGCATTTTTGTACCTGCGAAATCTAAAACATTTCTCAGCTCTGTTT
CTTTCCTTTGCAGTCTAGCATGTATTCCTGACTTCTCTAATCCCTTCCCAGTGTTTTAGTTCTAAGGGAGGGTGTGGTCT
GACCTACTTAGAAGGTAGGCACCCAATCACTGGCAGCTTACCAAGTTAGCTGATTTGAAAATCTGACCACAAGCTCACAC
GCTGTCCGAAAAGTTTGTTCATTCATCAGAAGCTCATTGCTTCAGCAAGAAGATAGTGGTGCAGAGCCTCCCATGCCAGA
TTGCTTGGAAACAGGAGAAAAACTCTAAGTATTTAATGAAATGCTAACTAAACTAAAGTGGGGGAGGCTTCCTCAGGGGA
GCTGGATCTTGCTCCTGTTAGCCTGCCACAGTGGGTCTCTATAGATATGCTGAGGCTAGGGTGGGGTGGAGGGTAGTATG
GGAGGCTGCCCTCACATTCGCCATTGCAAGATGGCGCTGACATCCTGTGTTCTAAGTGGTAAACAAATAATCTGCGCATG
TGCCAAGGGTAGTTCTCCACCCCATGTGCTCTGCCTTCCCCTTGACGACAACTCGGCCTATGGGCTGCAGCCAATCAGGG
AGTGACACGTCCTAGGCGGAGGATAATTCTCCTTAAAAGGGGACGGGTTTTCGCCATTCTCTCTCTTGCACTCTTGCGCT
CTGGCTCCTAAAGATGTAAGCAATAGAGCTCTTGCTCTCTGGCTCCTGAAGATGTAAGGAATCTTTTGCCACAGAAGATT
CCGGTTTGTTGCGTCTTTCCTGGCCGGTCGCGAACGCGTGTAAGAGGGTAGGAGCTCTGCTGTGGTAGGAAAGTAATCAT
GCCACTCTGTGCTTTCTGGTATGGCCTGTGTTACTTAAATGTGTTGGGGGAAAGTGCAACCAACAGAGAATTTGTTAGTA
CCTCTTTCCAGGGTTTCTCCAGTTTGGACTCCACTAACTTACAGAAAGTGCTATAAACAAATGGCTCCCAACATGCCTAG
AGGCTGGGATTTACTGTGCTCAATTGCTGTAAGTAAACTGTAGCAGAAGTGGGTGTTGTTGGTGTCAAGTTTGAACAAGG
CCCCGTGCCATCACCCCCACCACCAGCAGATTTAGAGATACCCTCAGATACTCCTTTGGGGAAATGTCTGTGTGTCTCCC
ATTGACTTGAGGGCCTGCACTCACACTTGATCCTGAGATCCCTGCTACTGCAGAAGGATGTGGTCCTTTTGAGTTTGTAA
ATCAAAGCAGGTGCCCAGATTCCTGGAGGGTTGACTAGAGGACCTTGGGCTTGCTCTGGCACACCTTGCCACCCAGCCCA
CACCTAAAGTGCCCCTCCTCCACACCTCCCTACAACTTTCCTTTCAGGCTCCATAACACATGGTGCACTCCCCCTTCCC
CAAACCTCCAAGCTCTCAGAATTTCTCTCTTCCTGAGGACACTGGTATCAGGCAATATACTCTTTCTGGCCTTAAATGAC
TCTTGTGGCATCAGGGAAGGATCAGGTGTTTTCCAGTAGGGTGGGGGTGGGAGATTTATCCCATCCACAAATCCATCTAC
AGTTTTAGTTCACTGGGTGCTGGGAATGAACCAAGCCCTCTCTCTCTGCAAGAGCAGCAAGCTCCCTTCCCTGTTGAGCC
ATAACTTTACCCCCACTTTAATACTTTTGTTTAGGAATAAAATATCAAATTTCTTGAAAAGCAGAGTTCACAATTGTTGT
TAGATCACGGGCCTAGTGGAAGCCTAGGGAAACCAGACAAGCTCCTTCTTAATGGACAGCTTAGCTGCTATGATGATTGG
AAACAATGTTCTGGGAGGTGGGGGACAGGCCGAGGAAGAGGGAGATCTAACCATGCCTCCCTTCAACCCTAGGGCCCTAC
TCCATGCCATCCTGTGCACACCTAAAGTACCCCCCTCCACAGCTATCCTGGTCCCTTAAACAGACCCTTAAACAGAGTGT
AGAATGGGGTCTCCTTGAGGTGTGATCTGCTTGCTGCCTCTGACTGTGAGGTTCCACCAGGTTCCACCAAGTCCCATAGT
CCTCACAGTAAGCCATAGCGCCTGCTGTGTTGGGAAAACTTAGAAAAGTAAAGATTTCCTTTGTTCTTCAGACTTTTCTA
TGGGTTAAAAATGGTAGCCAGGTCCTCACAGCACAGGCCAAGGGACATAAAGCAACTGAATTTGGTGACAGTTACTGTAT
CTAATGTTTTCACCTCCTCTGACTGATACCCAAAGAGAAAGGTCATGAGTAATACTACTGTTTCTCAGATAAGCCATGTG
CTTCTGAGGGCAAGTAGTCTAGATGAACACTAGAGCGCCTTAAGAGAGTCCATGACTGAGCAATAAGATGGTGAGGGTCT
AAAAATNNNNNNNNNNNNNNNNNNNNNNNNNNNNNNNNNNNNNNNNNNNNNNNGATGGTGAGGTTCTAAAATGGCGACTTTTTT
CATCACCTTCCGGACCTGAGAACAAATCTTGGCTACTTAAAACAGGCCTGTGCAGCCTTTTCTCCTCTCATTGGTGCCCC
TGCCAGTGAGCAAATCCAAATAGTTCAAGGCCAGAGCAGGATGTGGTTTTTGATTGACACAGTAAGATGAACGAACTTGC
TCTTTGTTTCATTATGGTGAATATATTCAAAATCACTTGGGCTAGCTTTAGAAATATGGTACCTTGTTGTGAGCAGTATT
CATCCTACTGTGTCTTTGAACACCAAATTTGATACCACTTTGAAGAAATTAATATCTGTTCTGTCTCTACTTCCCTCCAG
CCTCTGGCAAGAGATATTTTTACTTGCTTGTGTGTTCACAATAGCCAGCACATGGAACACACTAACAGGCTTCTCTGCTG
ACTGAATAAGCCAATTTGAGCTGAATTAAAAGTAGAAAAGCCTATTTGTTTCAGAACAGTTCCAGGGCAAGTTCACTGGT
CTCAGGGGACAAGACGGAAGTCCTGTAGGCTATGGCAGAGAAGGCTTTTATAGATTGTTGGTGAAGGAAAATGACCTGCC
TGCCACAAGCTGGGCTTGAGCCCCAGTGTGGTCACCTAGGCTGGGGACAAAGGTTGCTACAGCTATCATGAATGCGGAAC
TGGACTTTTGGTGCCAGGGCTGGGGTTGTGGGAGGTACGCAGTGTGCGCCAAGTCAAAGGCTCCAACCAAACAGACATCA
GCATCTATCAGTGGATGAGTGTGGAAAAACTGTGATACTTACTCCCATATATACTGGAATACTATGCACTAGTAAGATAG
GATGTCTTTTGTGACAACATGGGTGGATCTGGGTGACATGCTGAGACAAATTAGTCAGGCACTGAAAGGCAACATTGTTC
ATCAGTTGTAGAGGGGTTTGTCAGCTAAAAGCAGACAGGAGTTTCCACTCTTTTCTTGGATTGGAAAGATTTTTGAAATC
ACAGTGCAGAACTAGAAATCACAATGAAACCAAACCACTCCAATATGAAGGGGTTTAGAAATCTCCCAAGATTTACTTTC
TATAGGAGTGTGAGGAGGGCTGAGGAGTGCTCCCAGGGGAAGCTCGATTGCTCACAGCAACACATGGCTGAGAGGTGCTG
AGGCTCGAAATGAGCACAGTAGAATTTATTATACTATCTTTTATATTCTGTAGAACTAGAAAGAATGAAGCCAGGGAGT
TCTGTGTGGATCCAAAGGAAACAGTTCAGTGCCTTAAAGCTTTCTTTCTAATCCTAAGGATTCTTTTCCTGCTTGTTAAT
GTAATAGAAGCTTCCTGGTATGTTAGGTGTGTGAAATGAGTGAAATGAAAGGGTTAAAGTCAGTGAAAACTCTGTAAAA
AGTATAATTTACATCCAGTAAAATTAATTCAATTCTGTGAATTTTGAAAACTATATAATCTTGCATTCTTCATCATGATA
TATAGGGGCTGAAGATTTGGCTCAACCATTGAGAGCATTTATNNNNNNNNNNNNNNNNNNNNNNNCTTATCTATGTTTCGACA
AAAGAGAGATTTTGGCATTACTGCTGCCGTGATCATAGCAATTTCAGCCAGTGCAGCTGCTGCTACAGCTGCAGGGTACG
CTATGGTTAGTGCGGTTCAATCAGGCACAAAATTAAATCAGCTTTCAGCAGATCTGGCTGATGCCATCACTGTCCAAACT
TCTGCTAGTACCAATTTAAAGGGAGGGCTGATGATTTTGAATCAGCACCTTGACCTGGCGGAGGAACAGATAGGTGTTCT
ATACCAGATGGCCCAGTTGGGTTGTGAAAGAAACTGGAAGCTCTGTGCATTACCAGTGTCCAATATGAAAATTTTACTT
ATGCAGCTAATCTGTCTAGACAGCTTTCTTTATATCTTGCAGGAAACTGGTCTGAAAGATTCGATGAGACCCTTGAGCCC
CTGAGAGCGGCCGTTCTAAAGATCAACTCGACACGAGTGGACCTGTCATTGACAGAGGCCTCTCCTCCTGGATTTCATC
TGCCTTTTCTTATTTTAAGGAATGGGTGGGAGTAGGTTTATTTGGCGTTGCCACCTGCTGTGGACTTGTGGTCATGCTCT
```

```
GGTTGGTTTGCAAGCTCAGAACCCAACAAACACGTGACAAGGTTGTGATAACTCAAGCACTTGCTGCCATCGAGCAAGGG
GCCTCCCCTGAAATCTGGCTATCTATGCTCAAAAATTAATTGACATTGGTGGCTGGCCTCTTTTATAGAGTTCGCCCTAG
GTCATTTAGTAATTAATGTCACAATGCACTGCGGTATCCAGGGACGGGCAACTTTCCTCATGCACAGATCAACCTAAGAC
ACGGGGCCCGGTGGCGATAGGGTTACCCTATGACAGGAAAGGCTGTGACATTGGAGGAACGACCTAAGACAGGAGCCACA
GCGGATGGGCATAATTACACAGGCCTAGACCAGCCTCAAATTTTAATAAAATAAAAAGGGGGATATGTGGAGAGCCGGTA
CATGCTGCGAGCAATCGCATGCGTGCTGCCAGTAATCTCTGAGGAGAGCCGTNNNNNNNNNNNNNNNNNNNNNATTAAGCA
ACCCAGTGAAAAATGGGGTACAGAGCTAAACAAAGAATTATCAACTGAGGAATCTTGAATGGCTGAGAATTACCTACAA
AAATGTTCAACATCCTTAGTTATCATGGCAATGTAAATCAAAAGAACACTGAGATTCTACTTCATACCAGCCAAAATGGT
TAAGATAAAACAACTCAGGTGACTGCAGTTGCTGGTGAGGAAGTGAGAAAGAGGAACTCTCCTCCATTGCTTGTGGGACT
GCAAGCTGGTGCAACCATTTTGGAAATCAATCTGCCAGCTACTCAGAAAATTGGAAATAAGTATTCCTGAATAACTAGCT
ACACAACTCCTGGTCATATATCCAAGAGATGCTCCATTATCCCACAAAGACATTTGCTGAGCTATGTTCATAATAGCCTT
TTTTATAATAATCAAAAGCTGGAAACAATGCAGATCTCCCTCAATGGAAGAATGAATACAGAAGATATGTTACATTTACA
CAATGGAATACTATTCAGTTATTAAAAACAAGGGCTACATGAATTTTGCAGCAAATGGATAGACCTAGAAAATATCCCAA
GTGAGGTAACCCAGACCCAAAAGGATATACATCATCTCTACTCCCTGATAAGTGATTATTTGCCCAAAAGCTCAGAATAC
CCATGATACAAGTCAGAGACCATACGAAGATTAACAAGAAGGAGGCCAAAGTGTGGAAGGTTCAATCTCACTTAGGAGGG
GAAAAAAATAATCATGTGAGGTAGAGACAGAAAGGGACCTGGGTGTTAGAGGGGAGAGAAACGGAAAAAGGGGGTGGTCA
GGATCAAGTGTGAGAAGAGACAGGAGAGAAGTCCAAAGGACTGGCAGAAGGAATAGAAATATGTGGCACAGGGGGTGGGG
TTGGGGAATGGAGGGAGGAGGAGAAACCACTAGAAAGTGCCAGATCCAGGGATGCAAGGGGCTCCTAGGACCCAATGGGT
ATGGCATTAACTGAAATGCCCAACAGTGGGGAGGTAGAACCTGTAGAGACCACCTCCAGTAGATAGATATGGACCCCCCA
GTTGAGGGATGGGGCCACTCACCCTCAAAAATTTTAACCCAGAATTGTTCCTATCTAAAGGAAATGCAGAGTCAAAAAAT
GGAGCAGAGACTGAAGGAAAGGCCACCTAGAGAATGCCCCCCCCCCCAACTTAGAATTTATTCCATCTGCAGACACCAAA
TCCCTACTATATTACTGATGTTAAAATGTGCTTGCAGACAGGAGCCTGGTAAGGCTGTCCTCTGAGAGGCTCTGCTAGAA
CCTGACTAAGACAGATGCAGATACTCACAGTCAACTTTTGGACTGAGCCCAGAGACCAAGATGGAAGAGTCAGAGGAAGG
ACTGAAGGAGCTGAAGAGTATTGCAACCCCCTTAGGAAGAACAACAGTAACAAACAACTGGATTCCCCAAGAGCTCCTAGG
GACTAAACAGACAACCAAAGAGTATACATGGATGGGTCCATGATTCCAGCTACATTTGTAGCAGAGGAGTTCTTCATCAT
GTGTCAGTGGGAGTGGAGGCACTTGGTCCTGTGGCGGCTTGATGCCTCAGAGAAGGGGAATGCTAGAGGGGCAAGGAAGG
AGTGCATATGTGGGTGGAAGAGAACCCTCTTATAGGCAAAGGGAAGGTGGAATGAGGTGAGAGGTTCATGGAGGGGGAGAT
CAAAAAAGGGAACAACATTTGAAATGTAAATAAATGAAGTAATAAATAATAAAAAAAGAAATTGTTGATAACAGTAAAGT
TTTGGTGAGGCAAAAAGAAAGAAAGGAGTCAGGCAAAGTAGAGAAAGGTTGAGTATGGCTAGTGATCTTCTGAAATGAAT
AGAGTTGTATTTGAGATGCCCCCCCAACCCCCCACCCCTGTTTATATAGAACCGGAGTCCAAAGGCACAACTGGATGGTA
GACTTCACACTTCTCATGTACAAGTTTGATCTGAATTTGATCCCTAGCACCACACACAATAAAAGTGACTTCCACAAAAA
AAAAAAAAAAATAAAAAAAAAAAAATAAAAGCATTTTGTACCCTCGAAATCTAAAACATTTCTCAGCTCTGTTTCTTTCC
TTTACAGTCTAGCATGCATTCCTGTCTTCTCTAATCCCTTCCCAGTTTTTTAGTTCTGGGGGAGGGTGTGGTCTGACCTA
CTTAGGAGGTAGGTACCCAATCACTGGCAGTTTATCAAGTTAGCTGATTTGAAAATCTGACCACAAGCTCACACGCTGTC
CGAAAAATTTGTTCATTCATCAGAGGTTCATTGCTTCAGCAAGAAGATAGTGGTGCAGAGCCTCCCATGCCAGATTGCTT
GAAAACAGGAGAAAAACCGTATGTATTTAATGAAATGCTAACTAAACTAAAGTAGGGGAGTCTTCCTCAGGGGAGCTGGA
TCTTGCTCCTGTTACCTGCCACAGTGGGTCTCTATAGATCAGCAGAGGCTGGGGTGGGGTGGAGGGTTGGAGCTCTGCTG
TAGTAGGAAAGTAAGGATGCCACTCTGTGCTTTCTGGTATGGCCTGTGTTATTTAAATGTGTTGGGGTAAAGTGCAACCA
ACAGAGAACTTGTTAGTACCTCTTTCCAGGGTATCTCGAGTTTGCACTCCACTAACTTACAGAAAGTGCTATAAACAAAT
GGCTCCCAACATGCCTAGAGGCTGGGATTTACTGTGCTCAATTGCTGTAAGTAAACTGTAGCAGCAGTGGGTGTTGTTGG
TGTCAAGTTTGAACAAGGCCTGGTGCCATCACCCCCACCCCCAGCAGTTTTAGAGATACCCTCAGATACTCCTTTGGGGA
AATGTCTGTGTGTCTCCTATTGACTTGAGGGCCTGCACTCACACTTGATCCTGAGATCCCTGCTACCGCAGAAGGACATG
GTCCTTTTGAGTTTGTAAATCAAAGCAGGTGCCCAGATTCCTGGAGGGTTGACCAGAGGACCTTGGCATGATCTGGCACA
CCCTGCCACCCAGCCCACACCTAAAGTGCCCCTCCTCCACACCTCCCTACAACTTTCCTTTCAGGCTCCCACAGCACATG
GTGCACTCCCCCTTCCCCAAACCTCCAAGCTCTAGGAATTTCTCTCTCTTCCTGAGGCACTGGTATCAGGCAATACTCT
TTCTGGCCTTAAATGACTCTTGTAGCATCAGGGAAGGATCAGGTTTTTTCCAGTAGGGTGGGGGTGGGAGATTTATCCCA
TCCACAAATCCATCTACAGTTTTAGTTCACTGGGTGCTGGGAATGAACCAAGCCCTCTCTCTCTGCAAGAGTAGCAAGCT
CCCTTCCCTGTTGAGCCATGACTTTACCCCCACGTTAATACTTTTGTTTAGGAATAAAATATCAAATTTCTTGAAAAGCA
GANNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNN
NNGCCAAGGGACATAAAGCAACTGAATTTGGTGAAAGTTTCTGTATCTAATGTTTTCACCTCCTCTGACTGATACCCAAA
GAGAAAGGTCATGAGTAATACTACTGTTTCTCAGGTAAGCCAGGTGCTTCTGAGGGCAAGTAGTCTAGATGAACACTAGA
GGGCCTTAAGAAAGTCCATGACTGAGCAATAAGATGGTGAGGTTCTAAAATGGCGACTTTTTTCATCACCTTCCGGACCT
GAGAACAAATCTTGGCTACTTAAAACAGGTCTGTGCAGCCTTTTCTCCTCTCATTGGTGCCCCTGCCAGTGAGCAAATCC
AAACAGTTCAAGGCCAGAGCAGGATGTGGTTTTTGATTGACACAGTAAGATGAACGATCATGTTCTTTGTTTCATTATGG
TGAATATATTCAAAATCCCTTGGGTTATCTTAAGAAATATGGTACCTTGTTGTGAGCAGTATTCATCCTACTGTGTCTTT
```

```
GAACACCAAATTTGATACCACTTTGAATAAATTATTGTCTGTTCTGTCTCTACTTCCCTCCAGCCTCTGGTAAGAGATAT
TTTTACTTGCTTGTGTGTTTACAATAGCCAGCACATGGAACACACTAATAGGCTTCTCTGCTGACTTAATAAGCCAATTT
GAGCTGAATTAAAAGTAGAAAAGCATATTTATTTCAGAACAGTTCCAGGGCAAGTTCACTGGTCTCAGGGGACAAGACGG
AAGTCCTCCAGGCTATGGCAGAGAAGGCTTTTATAGATTGTCGGTGAAGGCAAATGACCTGTCAGCCACAAGCTGGGCTT
GAGCCCCAGTGTGGTTACCTAGGCTGGGGACAAGGTTGCTACAGCTACCATGAATGTGGAACTGGGCTTTTGGTGCCAGG
GCTGGGGTGGTGGGAAGTGCGTGGTATGGGTCAAGTCAAAGGCTCCAACCAAACGTACATCAGCATTTATCAGTGAGTGA
GTGTGGAAAATTGTGATACATACTCCCATATATACTGGAATACTATGCACTAGTAAGATAGGATGTCTTTTGTGACATT
TTTATCCTATTTTGTGTGTGGACCTGGGTGACATGCTGAGACAAATTAGTCAGGCACTGAAAGCCCAACATTGTTCATCA
GTTGTAGAGGGGTTTGTCAGCTAAAAGCAGACAGGAGTTTACACTCTTTTCTTGGATTTGGAAAGATTTTTGAAATCACA
GTGCAGAACTAGAAATCACAATGAAACCAAACCACTCCTTTATAATATGAAGGGGTTTAGAAATCTCCCAAGACTTCCTT
TCTATAGGGAGTGTGAGGAGGGCTGAAGAGTACTCCCAGGAGAAACTTCATTGCTCACAGCAACACATGGCTGAGAGGTG
CTGAGGCTGGAAATGAGCACAGTAGAATTTATTATACTATTTTTTTATATTCTGTAGAACTAGAAAGAATGAAGGCTGGG
AGTTCTGTGTGGATCCAAAGGAAACAGTTCAGTGCCTTAAAGCTTTCTTTCTAATCCTAAGGATCCTTTTCCTGCTTGTT
AATGTAATAGAAGCTTCCTGGTATTTTACGTATGTGAAAATGAGTGAAATGCAAGGGTTAAAGTCAGTAGAAACTCTGTA
AAAAGTATAATTTACATCCAGTAAAATTAATTCATTTCTGTGAATTTTGAAAACTATATAATCTTGCATTCTTCATCATG
ATATATAGGGGCTGAAGATTTGGCTCAACCATTGAGAGCATTTATTGCTCTTGCAGAGGACTGGGGTTTCATTCCAAGCA
CCCATATGGTGGCTCACACACCCACCCCTAATTCCAATTCCAGGGATCCAATATACTTTTCTAAGCTCTTCTAATACTAA
TCATGCCTGTAGTATACATACATACATACATACATTNNNNNNNNNNNNNNNNNNNNNNCAATGAAACCAAACCACTCCTTTA
TAATATGAAGGGGTTTAGAAATCTCCCAAGTACTTCCTTTCTATAGGGAGTGTGAGGAGGGCTGAAGAGTACTCCCAGGT
AGTAAACTTCATTGCTCACAGCAACACATGGCTGAGAGGTGCTGAGGCTGGAAATGAGCACAGTAGAATTTATTATACTA
TTTTTTTATATTCTGTAGAACTAGAAAGAATGAAGGCTGGGAGTTCTGTGTGGATCCAAAGGAAACAGTTCAGTGCCTTA
AAGCTTTCTTTCTAATCCTAAGGATCCTTTTCCTGCTTGTTAATGTAATAGAAGCTTCCTGGTATTTTACGTATGTGAAA
ATGAGTGAAATGCAAGGGTTAAAGTCAGTAGAAACTCTGTAAAAAGTATAATTTACATCCAGTAAAATTAATTCATTTCT
GTGAATTTTGAAAACTATATAATCTTGCATTCTTCATCATGATATATAGGGGCTGAAGATTTGGCTCAACCATTGAGAGC
ATTTATTGCTCTTGCAGAGGACTGGGGTTTCATTCCAAGCACCCATATGGTGGCTCACACACCCACCCCTAATTCCAATT
CCAGGGATCCAATATACTTTTCTAAGCTCTTCTAATACTAATCATGCCTGTAGTATACATACATACATACATTCTA
GCATACAGGACAACAAAGCACTGTTACATGTAAGATAAATAAGACTGAAACAGTCATTTAAAAGTATATACACATCGCT
TCTCAGCCTTTTGGCTAAGTCAAGTGTAAAAGTATATACAGACCCCATACCCTATCTCAGTTTCCCCATTGTCTGCTGCA
GACTCTCCCTGCTCCTCTACCTCAGCTGTGAGTACTCACCTTTCCAGATGCCATAAAATGTGTCCATGAAGCAGAGAGGT
CCCACCTGAGTTCTTTCAGCTTGTCACAGCAATGCCCTCTGCTCATCACTCACACTGCAGTGCTTATCAGTAGCATGTCA
GAAACAAGCAGTGCTGGAGAGATGCTGAGAGACCATGCTACAGCACTGCTGTCAGAACAATGCTCTGAGTTCAGAATTT
TGCATGAATCTCACCAGCAAATATTATACAAAGATGTTGTGAAAGGCTGTCAGGAAACTTCTGGAGAAGTGAGAGGAGGA
GTTTAGTAGTGGCAGTTGGGTGTCTCCTCTTAGAGGAAACTGCCACTCCCTGTAGTTCTCTGACCTAGTTTGCATAAGCT
TACTGGCAGGCTTAGTTTAGGGACTTTTTGGGTAAGAAGCCCTTGGGATATTTTGCCTGTTAAAGTGGCATGAGATAGGG
CAAGCCCAGGCTTGTGTTCTGAGTTGTTTCTTGTGTTGAGAGAAGGTTTACCTTGTCATCAGCTTGGGGAAATTTCAATG
GTCACAAATGTGTCATTTTCACAGGGATCCTTATATTCACATAAAGATATCGTGCCACTTAAATGCAGGCTGTCCTTTCC
AGCATGGGGATATGTGGGTGGATGGGAGCATATCATATTTTATACTTACAAACAGTTTTGTAGTAGCTGTACAATTTAGC
CTTAAAAAGGCGTGTTAGTTCTACATCAACATTGATATCCACATCAATGTATAACCATCCTGTTGTGCAGACAGTGATTT
TGCTCTTAAATCTAAGATGATTTTGCCCAAGACAAGTTCACAAACATTCTCCCTTATTTTTCTAAATATCAAATGTCTAT
GATATTAAGTTTTGTGCTTGGGTCTATATGTCTACAAATGGACTGTAGTAGTTTGTGTTTATTTTATTTTATTTCATTTT
TTAAATAAAAATACATCTTTATGTGTAAAAAAAAAAAAAAAAAAACCAAAATCCAAGACCTACCAGTTTTCCATTATAA
AAATCCATAAAAAGAATCGTATGAATTAAGAGGCTGCAATTNNNNNNNNNNNNNNNNNNNNNNNNCATAAAGATATCGTGCCAC
TTAAAATGCAGGCTGTCCTTTCCAGCATGGGGATATGTGGGTGGATGGGAGCATATCATATTTTATACTTACAAACAGTT
TTGTAGTAGCTGTACAATTTAGCCTTAAAAAGGCGTGTTAGTTCTACATCAACATTGATATCCACATCAATGTATAACCA
TCCTGTTGTGCAGACAGTGATTTTGCTCTTAAATCTAAGATGATTTTGCCCAAGACAAGTTCACAAACATTCTCCCTTAT
TTTTCTAAATATCAAATGTCTATGATATTAAGTTTTGTGCTTGGGTCTATATGTCTACAAATGGACTGTAGTAGTTTGTG
TTTATTTTATTTTATTTCATTTTTTAAATAAAAATACATCTTTATGTGTAAAAAAAAAAAAAAAAAAAAAACCAAAATCCAA
GACCTACCAGTTTTCCATTATAAAAATCCATAAAAAGAATCGTATGAATTAAGAGGCTGCAATTGCAAGGTGAAAGGCTG
AAAAGGCTGAAAGAAGATTAAAACTGTACAAATTCTTTTTAAACTAAGTGGAACCTAAATTAAGTAAACACTGTTAGTCT
CTGCTATGTAACGTGGCTATACAATTCAGCAAGCAACCACAAAATTCTTTCCTTCTCTTTTTCTACTTCTCTGACCCAGA
GCTTTTTCTATGTACTCAGACTGATCTTAAACTTTTGATCTCCCTGCCTTGGCCTCCAGAGTCCTGAGATTACAGACCAT
TATGCTGAGCTCCAATCAGGACTCATCTGAAAATGAAAGATAGTGATATTTACTTTAGAACAACAACAACAACAACAA
AAACCCAAAACCATAAAATTAAAAGAACCAGGCACAGTGGTGAACACACACCTGTAGTCCCAGCTACTCTTGGGGCTGAG
GAAGAAGCCATCTGAGCCCAAGTGGTTGAGGCCAGCCCTGGCAACAGCTATTCCTTTACCCCACTCCCCACCAAAAGGAG
ACATATCTGTGACATGTGTCCCATAGAGAAGGTAGTGTGGGACACTGTTTTCCCTGGTGTGCCTTGCTTCCTCAAAGTCA
AGGCTGATTTTCACAAGCACCCTGTGACATGTGGTTAGCACTCTAAAGAAGTAAACAGTTATGGTTCATGACATTGTGTT
CCATTCCTTCTACCCTCCCCTACCCCACTACTGGAGACCTTACCTAGTGGTTTATGCATGCTAGGTAAGTGTTCTAGCAC
TGGACTTTACCCCGGCCCTCTTTTTATGTTAGGACAGGGTCTTACTGGCCTAGAATTAAGGCTGTAGTCCAGGCAGACTT
ATGTCTATAACCTGTTTCAGCCTCCCAAGTATCTGGGATTACAGGTCTGAGTCACCTGCCTGACCTAGAGGTGGGTTTAA
CATGACAGCTCTCCTCTTCCATGAGCACACTTAACCACACAGTGACCAAAATAGCTGAGATGAAATTTCAATGGGTATTAA
GCCTCTAGCCAAGCCCCTCTCCCCAGTGATTCTAGCATGTGTCTCCCTGGGATTGCTAATTTTCTGGAGGATTCTGGGCC
```

```
AGGTGCTGTTCCCATTCAACTTCAACCAACTTGTACAGGTCCATTGTGGCCTGGTCATCTTCTACCAAGGAATGTCCACT
ATTTCCAACCTGGATGTCCCGACTCAGCAGCTTCTTGGTGAGATGTTTCAGTGACAAAGTGACATTTTCTGGGCAGTCAT
CCTTCCGGTGGAGGAGTGGTATCTGGAAAGTGTCTCGGGTGAGGGACTTGGGATGAAAGTACTGTAGGGCTTTGTAGTCA
TTGTGAGCGGCATGCCCTACCACTACCTTCCCTGAGAGTATCTTCAAGATCTCACTCTGAGCAGTCTTAAAGGGTGTAGC
GTTAACCATGCGGCACTTAAGGATGCCACTCCATCTGGTCTGGTCGTCCACGATGTAGCAAAGGGGTGTGAGGATGGGGG
AGGACGTACTCATCATACAGCACATCGCCATTGTAATTCACAATGCTGCATCCAGCCAGGGAACTAAGGCACCCCTTAGG
TCTTGTGCCCACCATTTCACAGTCTATTGCCACCATCTTCCCTGGCAAGTTTTGGGGTACTGTAGGGCACTTATCTTCAG
AACGAGCTTGGGTGGAGTTCTGTACAGCATTTTTCTTCAAGGGCTTCTTCTTGGAGCCTTTATCCTTTGACTGAGCTTGG
GTGGAGTTCTCTGTAGCAATTTTCTTTTTCAAGGGCTTCTTCTTGGAGCTCTTAGTCTTTGGAAGGGCACTCTGAAGCTC
CCCTAGCAAATCTATTTTAGTCACAACAGAAGCAGTCTTCTTTGAAGGAGCAGGGGTCAGCCAAGACAATGGTGCTTTCT
TGTCTTTGGACTGCTCTGAGTCCCTCTTGGAGGCAACTGTGTCTTTCTTCTTCTTTGGGCATGGAAGGATCTTCAAAATG
CCATCTACTCTTGAAGTTTCCCCTTTCTTTGGAGGTTCTGAATTCAACTTAGACACCTTGCTAGGGGATTGGTTCTTTTT
ATTCAGAAATAATTTCTTTTTCCAAGAAACGTTGCTTCTTGACAAAATGTTGATGTTTGGCATTTCCTCCAAATGTCTTTT
TCGAAGGTTGCCCAAAGTCTAAATTCAGGAGTATGGTAGACATGAGATCAGTGGATGGTGGGAGAAAGCTGGGAAGAATG
GCTCACTGATGGCAAAAGGATGAGAGGCTCATTCTCTGTGGAATTCCATAATTCAACATATGGAGGTTTGCAATTAGCCT
TGGGGACAAAAGTAATCCTGATACCCGCAACATAGGCCTCCAAGTTGGGTCCCCACTAAGATCGCAAGCATCCGGAGCAG
GAATGCGGGTGACAACGGGAGGGCAGCTAGCGAGGGCCCACAGAGCCCCCACCTCAGGCAGCAAGGCCAGCAATTCAGAG
TGCATGAGCATCAGGAATGATGGTGGCGGCATGGGTGGGGGCTGGATGAGCTCCACGGCCCCTCGGGCCCATGGAAGTCT
ATTTTATTTTTTAGCAGACAGATAGAAGGTTGTTTCAGTAGCACACACTGGAGTGATGCCACCTTTTCCCTGTCTAAAGA
CTGTTCATCTCTGTTAAGTGTCTGTTGACCACCCACGTGTGTTTGACCTCATTGTGGAGTCCTATTTTCTGCTGTGTTGT
TTCAGTGTCTACTCTGATGCTAGCACCAGGCTTTCATTCCTGTTCCTGTAAGAACATGAGACATCAGGTCAGGCCTTGTG
ATCTTTCTGATTTTGACCTCTTCATTCTCAGAATAATTTTTGACTATTAATTTTTGACTCTTTAATTTTCATATTACTTC
TCATACAACTTGGTGCTATGATTTTTTTTTTTCTGAAGGCAACCTCCCCTCTGGAACATGTTGCACATATATGTGTGTTC
AGGAATTGTTGATATGAGCAATATGGAGTGTTCTAGTTCATTATGTGTTTAGAGCTGCCTTGATTTCTTTTTTTTTTTTT
TATTTCAAGAATGTAATATGTGTGAGTGTTTGGCCTGCATAGACATCTATGGATCATGTGCATTCCTAGTGCCCACCAAG
GTCAGGAAAGTTCTTAGAGTTCCTGAAACTGGAATTACAGAGGCTTGTGAGCTCTTATATCGGTGCTAAATTTGAACCCA
GGTGGTTGGAAAGATCAACAAGTGCTCTTAACCACTGAGCCAACTTTCCTATATTTATTTGTTTATAAATTTACATGTAG
TTTTTGTTATGCTGACTATGAACATCCTATTTTTAGATTTGTAATTTTATGGTTTATTTGGCATTACTGTAAAGAGCACT
GTCTAAGATATTAATTTCCACTTGACAGGAAAATATAACTGATTTTACCATGGTGATGCCCTGTCCTGCAACTTCATAAA
CTCATCTTTTTTTACATCCAGTATTCTTTTATAGATTTGTTAGAATTTTTATGTAGATAGAACCATGTCATCTGTGAAGA
GAAACACTTTCGCCTGTTTCTGAGAAATGTAAACTTCCTTTCGTTCTTTAAGAAACTTATTTATGTACCTGACTGTTTGC
CTGCAGGTTTGTGTATCACATGAATGCACAGTGCCTAAAGAAGCCAGAAGAAGGCGAAGGCTCCCCCGGAACTGGAGGTG
TGCAATGTTGTAAGCTACCTTCAATTCAAGTCCTCTGGAAGAGCTGAAAGAGATCTTAACAGCTGAGCCATTCTCCAGAT
ACCTGACCCTATTTCTTTGTCCTGGCGAGCACCTCCTGGGAAAAGTCTACCTGGAGTAATGAGCAGACATCTGACTCTTG
CTCCTGATTGCTGGGAACACATTCATAATGGCACCATTGAGAGTGCAGCTGACTGAAGTTGGACTTACTGTTTTGTTTTT
AGTGGATGACTTTTGTAGGGTTGAAGAAGTTCCTTTCGCTTCCATTTTCACATAATTTTGCTGTTATTGAGATATTTCT
AGTTTTTCCTCTTTTCTTATTTTGTTATTATGTCTAATTACATCAACTTATTTTCTAGTGTTTCCATAACTCCTTCTAGC
CAGAGCGCTTTTTATGGAGCCAAATCCAGATTTTTGTTGTTTGCATTTCACTGGGATCCCCACTCCCTCCATTTTTGCGC
TTCCATTATTCACCCTGAGTTCAGTGACCAGAAGGCTCCCTGGCAGAAGTTCTGTTTCCATCCTCTCTATACTTCTTTCT
ACCATGGACATCCTTTGTGGACCAGGGCCTTCTCTAAAGGGTTGGCAGAAGCCTTTCAGGAACTGATAGGCAATTGTCA
AGTGGGTTTTGTGGTTATTTTATTTTATTTTATTTAAGAAATTCTTGAAATTCGCATTCTTATATTCATATTTTACAGAT
AAAATTCTCCAAAGAAAAAGTCTCAGAGCCATCTCCCCTGAGTCTTCTGCTAAGCTTTACTGCTGCTATGGAGTGATCAT
GGTCCTCACTGTAGCTGTAGTTGCTCTTTCTGTTGCTTTGTCAGGTAAGTGACATACCCTCCAAATTCTGTGACACTCTG
TCCATATTCACATTGCCAGTTATGCTTTCTAAGCACTGTGATCCAGGCACTGTGGCAAGGGCTCTAGAGGAAACACACTG
GAAGGTCCTGTTCTCTGAGAATTTAGGTTCCAACAGGAAGATGCAGTGAAGGAACACAGAGGCTTTGATGGGGACATCCC
CGGGAAGATGACATCCAGCAAGCTCTAGACAGAGATGCAGGGGACATAGGTCCCCTTGGGGGAACAATTCAAGGCAGAGA
ATAACAAGAGGGAATCTCCAAGTAGAAACTTCAAAGGTGAGGCCAGGAAGGTACAGTGCCTTTGACCATGACCCATGAGT
TTAGATACCAGGCTCAACTCTATTTTGAAAGTATTAAATGCAAAGTTCCTGAAGTAAGAAATTTATAGGATTTTAGTACC
ACAATATTCAGAATAGTGCAATACAATCTTGCACTGTCCTCTTAAGTATTTGAAGTCATCCTTTAGTGCAAGGTGTCTGC
ACCGTATATACGACCTACCCAAAAATTCTCATAGAAATCTCAATTATCAGGCTGGGTGTCAGTAGGTGTCCCTACAGAGT
GCTTGCTGCTGTAGCAATCCCCACTGTAGTCAATGGTCATCCAAAGCTCAGAAAGTGATGCTGTTATAGAAGTGCACTCC
CTGGGAGCCCTACTGACAGTGAGCACCTGAGAGAGAATGGGACACAGGCCCACGGTGGGAGGCCTTTAGTTAAAGGCACA
TCTCGATCAGGAGAGGATTCCTACAGATCAGTTAGGAAAGCTACCATCAGATTCACACCTCACAGCTGAGCTCAGGAGAG
TGTGGCAAAACGAGAGAAGACCTGCTTGCTATGATCCATCATATTCTCTACATTTTAGTAACAAAGACAGAACAGATCCT
AATCAACAAGACCTATGCTGCTTGCCCGAAAAACTGGATTGGAGTTGGAAATAAATGTTTTTATTTTTCTGAATACACAA
GTAACTGGACATTTGCCCAGACCTTCTGCATGGCACAAGAGGCCCAACTAGCTCGGTTTGACAACGAGAAGGAGCTGGTA
AGCAATGGGCAGGGATTGGTTTGTCTGTCTGTTCTGTTGAATATTATATTGCCTTGAGATAGAGAGTTACAGATGAGGCC
CGAGGAAGGATCCCACCCAAGCACATGGAGACATAGGGAATGTGAGTGTGTGCCATTTGCTGATGCTTGACTTCTGACTG
GAGCCCTGAGATAGTCAAGAAACATTCTCTCATGAAGTGCTCATAGTCAGCTGGAAGGTCAAATATGCCATTTTACTGGG
ATACCTGGTGACCATGAGTGTTTTCCCATATGCTGGCATATGTTGGGTACAGAAGGAGACAACTGATAATAACTGCAGTG
GAAGGTTAACCCAGAACTGTCCAAACCACAGAGGAATGTGACCCTCAGTTACATCCTCCTGTTATCTCTAGAGAAAGGTG
```

```
TGGAGTGGAGAGACTCCAGGATCATCTGAAACAAATAGACACATGTATTCTTGACTTTTTTGTGTTTTATGACAGAATTT
CCTAATGAGATACAAGGCAAATTTTGATTCCTGGATTGGACTGCACAGAGAGTCGTCAGAGCACCCTTGGAAGTGGACAG
ACAACACTGAGTATAACAACATGTATGTTTTCACGATGTTTTTCCTTCTATTATGTTCATGTGTTGTGATATGTGTGTGT
CGTGGCTATGAGAGATGGAAGTCAATGTCATGTGAAGCCAACTGTACTGGGAAGAAAGAAAAAAAAATGAACCCTTGCCT
GGAGGTGTGGCTCAGGGGTAGAGAGTGTGTATAAATGCAATATCCAATCCCCAGAAAGCTCTACACACCCACAAATTTAA
ATACTTCAGAGGTTTTCCTGTTTATTGACCGTCATTTTCAAAACCTTTGCATCATGTCATTTTACTCAAAATATTTACCA
TAATGATGGTGTCTGAGAGTAGCTATTGTTTGCTCTGGCTCCAACTTAAACATTTCTGTTGTTGATAAATGTCCTGTGAG
GGATATAGACAGAGCCTTAGATGGGCAGTGGGGGCTCTGGAATCCCAGAAAGCCACTGCAGTATCTGCAAGCCTGAGATT
CAGCTTTCCACTATTTGCATGTCTGCACCTGTTCAGGAAAGCAGAGACTCTAAGTACATTTGGAACCTCCTCTAAAGTCT
CGTCATCACTGAGCACCCAAAACAGTCCTTGGGTTTGAGCTGTTTTACTGGGATGGTAAATCACAGACTCAGTCACATCC
ATCACTGAAGCCCTTAGAGCAATTTACTAAGTGGGCGTCCCCATATATAAAATGCCTAAAACAGAATTGAAAATCACCCT
TGGTGGGGTCACTCATGGCTGCAGTTCATTTGAACATGGCAGCGAGCACCAGCCCAATGCCTTGTACACACATTACAGGA
TTCACCATGGACAAATGACAAAGGAGTGGTGTTCAAATCCTGAGAATATGAGACAGTAGGTGTAAAACTAATGCAGGTGA
TTCCTCAGGGACTTTTTGATTCATATTACCAAAAATTAGTGGAGACTGGTGAGATTTCATTGCAGGAGCAAATGCAGTTC
TGGGCTCTGTAGGCTTACTTTTTTGGTTTCTTTTCAGGATTCCCATCCAGGGAGTGGAAACATGTGCCTACCTGAGCGGC
AATGGGATCAGCAGTTCCAGGCACTATATACCTCGGATATGGATCTGTAGCAAGCTTAACAACTATAGCCTCCACTGCCC
AACTCCTGTTCCTGTCTAGCATTTACCAAGAGACTCTTCCTAGCCTGTTATCTATGGGTGCTACTTTTTCCCCTATGGTC
CCACAGTGCTATCAAACGGGATTGAGAATATTTTTTAACGTCGCAAATGAAAACCATCAAGGCTGGAGAGATTGCTCCGT
AGTTAAGAGACTGACTGCTCTTCTGCATGTCCCGAGTTCACATCTGAGCAACCACATGGTGTCTTACAAACATCTGTAAT
GACATCTTATGTCCTCTTCTGTGGTGTGTGAAAACAGCTACACTATACCTACATATGATAAATAAGTAAATCTTAAAAAA
GAAAAAGAAACCACCTTAGAGAGGTGCACACATGGAGGATTACAAGACCATAGATGAGTTTTAAATAGATGTCAGCACT
CATACCTTAAGCCTAAAGTACAACTAATGTTAGGGAACCCCACTTTTATGATATTAAGGTTTTGTGCAGAGAATTCTTCT
TTTGAATTTATGAGACCACAAAAATGAGTCCCCCAACATGGGTGTAACCTTTAATAATGAAAGCAGAATGGCTGGGATCC
TGAGAAGCATCTGTAATCCCAGGTACCGAAGGGTTGACACAGGAGGGTCTCAGGTTCACCAGTGGGGACGGAAAGCACAA
AAACACTTTAATAATGATAGGGAAGATGAGATGTCTGTTGCATCCCGTAATTTGAAGAAACATATTATACATGCTTTTCA
AAAATTCAAACCAACATACAGAAGAATTTTTAAAAAGTGTGTAAAATTAACATTTACTTTTTACTCTGCAATTTAAAAAG
ATGAGGGACATTGGCGAAATGTTTCTCCACTTTAATTTTAATATTCCTATTTCCAATTATACTGTTCTAAGTATAATTAT
ACTTACTTACTTTTCCTGTTCTAAGTGTACTGCATTTTATAATCAAATACCATTTTAATTTCACTATTTTATTCCACGAG
CCTTCTGGCCCAGGCCAGTGTAATGGTGGAGTCTATATAGAATCACTACAAGATAGTTCATCTTTGTGGATCTGCAGAGA
TCTGCTTCAAAGGGGGAGTGTTTCTACTCAGCAATTGTTATATATGTTTAACAATAACAGTAAAAGCACATTAATAGCAG
GAATCTCTCCTAAAACGAGTCACTCATAGCCTTGCTTAATGAGGGCTCACCTGTCACAGATTATATAATAATCTGAGGCA
GGCCACTTCAGGATGATCACCTGCTAGTTATTAGCTTGTCTCATTATGGGTCCTGACCATTTTTTTGGATTTATTTCAC
ATCAAATGTGAGTTAAACTCCTTAGGCATTTAATACTTAATTTTACACTAGCATGTGCATTACTGAGCCTTTAAAAAAAT
ATTGAAAATCTTATAGATTGAGCTACCATGTACTGATGAGTCCCAAAAGAGAAAAATCATAGGCTAAACGCAGCTCTTG
GTCTCTTCATCCTTTCTTCTCCTTCTTGGTCTTTTTAAAGACTATATCAACTTGATATGCCAAGGGGGAGTTGATATCCA
TGGGAGGCCTCCAGGTCTTTAAGGAGAAAGGGATGGAGAAATGGAGGAAGGGAAGGGGAGAGGGACATAGGGGGAGGGAG
GAGGGAAGGGAAATTAAAAAAAAAAAATAAAACCTAAACACTACATTTGTACGGACTTCATTTATGCTTATTGCTTGTATG
TTCATATGTATTTGCCCCACCTGTGCTGGGCAGACAATGCAGAGACAGCTCAGACAGAGATCAGACAGGACCAGAAGGAA
TCAGCTCCCTGCTTACTGTGAGTGGTGATGTGGATGCTGGAAATGGAGCAAGACAAGTGCTCCTAACTGCTGAAGCCAT
CTTTCCAGCCTTTTTGTGTTCCTTTTATTGGCACTGCTGATCAAGCTCTGCAAAAGTAGCACGTATCCCTGCAGTTTTTG
TAATTTTCTTATAAGTTCTTGCCTAAATCTGTGACCCTCCTTAAGTAGCTGGGGTCACAGGTGTGTTTCACCACACCAGC
CATTCTACTTCTCAATACTAAAGACCAAGAACATTTTAAAAATGCGTTGATGCTTTGCCTGCATGTGTGTCTGTGTAAGG
GTGCCACATCCTCTGAAACTGGAGTTACAGGCAGATGTGGCTCCCATATGGGTGCTGGGAATTGAATCAGGTCCAATGCT
TCTAATCACACATACATTTTGAGTAACGCATTGTCTTTATTTTAAAGCCAGGTTTAATTATTTATTAGATGAATGCAAGC
ACATTTCACTCTGAAAATGACTTTCTTAGCCCCCCCCCCATTAGTCCTAATAATACTATTTTTTTGCAATCCATTTTACTT
CCTACATCCTAAGAGATAGGTAGACTATGGTCCCAGGGATGGTTCAGAGAAATAAATGTTAAACATTAAGAAAATGCTAA
TTTCAAGAGAGTTAAAAGTTATCATTAATATTTCCATGATGTTTGTAGAACATGATTTTAATTTTTTCAACCATTGATAC
TCACCAAACAGAATAGTTATTTTAAGATGTATTTTCTTGTTATGTCTCCCTTTTCATTTCTGATTTTATTAATTTGGTTA
CTGACTCTGTGCTCTCAGTTTAGTCTGTCTAAAGGTTTATGTATCTTATTGATTTTCTCAAAGAACCAGCTCCTAGTTTT
GTTGAGTCTTTGTATACATCTTTTTGTTTCTATTTGGCTTGATTCTTTACTACCTTCTACTCCTCTTGGGTGCATTTGTT
TCTTTTTGTTCTAGAGCTTTCAGGCGTGCTATCAAGCTGCTAGTGTAAGCCTTCTTCAGTTTCTTTTTAGAGGCACTTAG
AGCTATGAGTGTTTCTCCTACCAGTGCTTTCATTGTGTCTTAAAAGTTTTTGTATGATGTGTCTCCATTTTCATTAACTT
CTAAAAAGTCTTTAATTTTTTTTTATTTCTTCCTTGACCAAGTTATCATTAAGTAGAGCATTGTTCAGCTTCCATATGT
ATGTGTGCTTTTCGTTGTTTTTGTTGGTATTTAAGACTGGGCTTAGCCTATGGTGATCTGATAGGTTGCATGGGATTATT
TCAGACTTCTTGTATCTGTTGAGGCCTGTTTTGTGAACACTTATATGGTCAATTTTGGAGAAGGTATCATGAGGTGCTGA
GATGAAAGTATATTCTTTGGCTTTTGGATGAAGTGTTAGATCCATTTGGTTTATAACATGTTAATTTCACTGTGTCTCTG
CTTTGTTTCTGTTTCCATGATCTGTCCATTGCTGAGAGTGGGGTATTGAAGTCTCCCACTATTATTGTGGAGGGTGCTAT
GTGTGCTTTGAACTTCAGTAAAGTTTATTTTATGAATGTGGGTGCCCTTGCATTTGGAGCATAGATGTTCAGAATTGAGA
GTTCATCTTGGTAGATTTTTCCTTTGACCAGTATGGTGTCCCTCCTTATCTTTTTTGATAACTTCTAGTTGAAAGTAGAT
TTTATTCAATATTAGAATGGCTGCTGAATCTTGTTTCTTGGGTCCATTTGCTTGGAAAATTGTTTTGCAACCTTTTACTC
TGAATTATTGTCTGTCTTTGTCACTGAGATGTATTGCCTGTATGCAGCAAAATGCTGAGTCCTGTTTACATATCCAGTCT
```

```
GTTGACAAAAGAAGCTAAAGGATGCAAAAATAAAATATAGGGACATTTACAGATGCCCACTTGAGAGAATTTCATAGTTT
CACAAGCGTGCCTTGAAGCTGTCATGTGAGTTCATAGAAGAGCAGGTATGTATGATACTGGTCAGATTAAAAGCCTTCCA
GGCCTTTGTTTTAAGCTGTCAGGCTTTTGTCTTAGGTCAAGAGGGCATAGGAGTTTTGCCTGCATGTATGTCTGAGAACC
ACATAAGTGAAGCAGAGGCCAGAAGAAGGTGTCTGAGCCTCCAGAATGAGAGTTATATAGATGGTGTGAGCAGCATGCGC
ACTGAGAAATTCACCCTGCTCTTCTGCAAAATCAGCAAGTGCTCTTAATCAATGAGCCATCGCTCCAGCCATTTACTCAA
AGTCCAAATGTGGAATCACTGTTTTGCTGCTGGATGCTGCTGTATATTGCCTTTATGCATTCTGCTACAGGAACAGAACA
CAACATTCATAGTCATAAACACATTACTCTTTCCAAAATGCACTGTGTTTACTCATTTTTCATCTGTTGCACTTAGTTCT
CTATTACACTGTCACACACTGACATTTCTTGCATTATTTTTTTAAGGTAGTATATATATATTTTATTTTTTTATTATATA
TTTTCTTCATTTACATTTCAAATGCTATCCTGAAAGTCTCCTACACAGTCCCCCCACCCTGCTCCCCTACCCACTCAGTC
CCACCTCTTGGCACTGGTGTTCCCCTGTACTGGGGCATATAAAGTTTGCAAGACCAAGGGGCCTCTCTTACCAATGATGG
CCGACTGTGCCATCTTCTGCTACATATGCAGCTAGAGACACGAGCTCTGGGGGTACTGGTTAGTTCATATTGTTGTTCCA
CCCATAGGGATACAGAACCCTTCAGTACCTCCATTGGGGGCCCTTTGTTCATCTAATAGATGACTGTAAGCATCCACTT
CTGTATTTGCCAGGCACTGGCATAGCCTCATATGAGACAGCTATATTAGGGTCCTTTCAGCAAAATCTTGCAGGGGTATG
CAATAGTGTTTGGTGGCTGATTATGGAATGGATTCCATGGTGGGGCAGTCTCTGGATGGTCCATCCTTTTGACTCAGATC
CAAACATTTTCTCTGTAACTCCTTCCATGGGTATTTTGTTCCCCAGTTTAAGAAGGAATGAAGAATCAATGTGTTTGTCT
TCCTTCTTCTTGATTTTCTTGTGCTTTGCAAACTGTATTTTGGGTATTCTAAGTTTCTGGGCTAATATCCACTTATCAGT
GAGTACATATCAAGTGACTTCTTTTGTGATTGGTTTACCTCATGCAGGATGTTATCCTCCAGATACATCCATTTGCTAAG
AATTTCATAAATTCATTGTTTTTAATAGCTGAGTAGTACACCATTGTGTAAATGTACCACATGTTTTGTATCCATTCTTC
TGTTGAGGGACATCTGGGTTCTTTCCAGCTTCTGGCTATTATAAATAAGGCTGCTATGAACATAATAGAGCATGTGTTCT
TATTACCAGTTGGAACTTCTTCTGGGTATATGCCCAGGAGAGGTATTGCTGGATCTTCCAGTAGTATTATGTCCAATTTT
CTGAGGAACCTCCAGACTGACTTCCAGAGTGGTTGTACAAGCTTGCAATCACTCCAGCAATGGAGGAGTGTTCCTCTTTC
TCCACATCCTCTCCAGCATCTGCTGTCACCTAAATTTTTGATCTTAGCAATTCTGAGTGGTGTGAGGTGGAATCTGAGGG
TTGTTTTGATTTACATTTTCCTGATGATTAAGGATGTTGAACATTTTTTTACGTGCTTCTCCGCCATTCGGTATTCCTCA
GTTGAGTATTCTTTGTTTAGCTCTGTACTCCATTTTTAATGGCGTTATTTGAATTTCTGGAGTCCAGCTTCTTGAGCTCT
TTGTATATATTGGATATTAGTCCCCTATCAGATTTAGGATTGGTAAACATCCCAGTCTGTTGGTGGCCTTTTTGTCTTAT
TGACAGTATCTTTTGCCTTACAGATGCTTTGCAATTTTATGAGGTCCCATTTGTCGATTCTTGATCTTACAGTACAAGCC
ATTGCTGTTCTGTTCAAGAATTTTTCTTCTGTGCCCTTATCTTTGAGGCTTTTCCTAACTTTCTCCTCTATAAATTTCAG
TGTCTCTGGTTTTATGTGGAGTTCTTTCATCCACTTAGAATTGAGCTTTGTACAAGGAGATAAGAATAGATCAATCCACA
TTCTTCTACATGATAACCACCAGTTGTGCCAGCAACATTTGTTGAAAATGCTGTCTTTTTTCCACTGGATGGTTTTAGCT
CCTTTGTCAAAGATTAAGTGACCATAGGTGTGTGGGTTCATTTCTGGGTCTTTTATTCTATTCCATTGTTCTACCTGTCT
GTCACTGGACCAGTACCATGCAGTTTTTATCACAATTGCTCTGTAGTAGAGCTTGAGGTCAGGCATGGTTATTCCACCAG
GAGGTTCTTTTATTGTTGAGGATAGTTTTTGCTATCCTAGGTTTTTGTTATTCCAGATGAATTTGCAAATTGGCCTCGCT
ATCTCAGTGTAGAGCTGAGTTGGAATTTTGATGGTGATTACATTGAATATGTAGATTGTCCAGGATCTTCTGTCTTTCAC
AGTCTCTGGTGAAAAGTCTGGTGTAATTCTAATAGGCCTACCTTTATATGTTGCTTGACCTTTGCCCCTTATTGCTTTTA
ATATTCTAACTTTATGTAGTGCATTTGTTGTTCTGATTATTATTTGTCGGGAGGAATTTCTTTTCTGGTGCAGCCTATTT
GGAGTTCTATAGGCTTCTTGTATGTTCATGTGCATCTCTTTCTTTAGGTTAGGGAAGTTTTCTTCTATAATTTTGTTGAA
GGTATTTGCTGGCCCTTTAAGTTGAAAATCTTCAGTCTCATCTACTCCTATTATCCGTAGATTTGGTCTTCTCATTGTGT
CCTGGATTTCCTGCATGTTTTGAGTTAGGATCTTTTTGAATTTTGTGTTTTCTTTGATTGTTGTGTCCATGTTTTCTATG
GAATCTTCTGCACCTGAGATTCTCTCTTCCATCTCTTGTATTCTGTTGTTGATGCTCGCATCTATGGTTCCAGATTTCTT
TCCTAGGGTTTCTATCTCCAGAGTTGTCTCCCTTTGGGTTTTCTCTATTGTTTCTTCTTCCATTTTCAGATCTTGGATCA
TTTTGTTCTATTCCAACACCTGTTTGGTTGTGTTTTCCTGTAATTTTTTAAGGGATTTTTGCGTTTCTTCTTTAAGGACT
TCTACTTGTTTAGCAGTGTTTTCCTGTAATTCTCCAAGGGATTTTTGTGCTTCTTCTTTAAGGCCCTCTAAATGTTTAGC
CATGTTCTCCTGTATTTCTTTAGGTGAGTTATTAATTTCCTTCTTAAAATCCTCTACCAGCATCATGAGATATGATTTTA
AATCTGAATCTTGCTTTTCAGGTGTGTTGGGGACTCGCTGAGGTGGGAGTGCTGGGTTTGGATGATGGTGAGCAGTTTTG
ATTTCTGTTAGTAAGATTGTTCCATTCGCCTTTCACCATCTGGTAATCTCTGGCATTAGTTGTTATAGCTGTCTTTGGCT
GGAGGTTGTTCCTCCTGTGATTCTGTTAGTCTCTGTTAGCACTCCTGGGAGTCCAACTCTCTCCTGAGTCTCAGTGGGCA
GAGTACTCTCAGCAGGCCATCTCTCCTCTGACAGGGGGAGGAGCACAGATGTCTGGAGCTCAGATCTACTTCTTGGCTGAA
GATGATGACCCAAAACTGGGCCTGTCCCAGAAGCTATGTTGCTTCTTCAGTGTGCAGGTTCCCCTGCACAGACTGGTCTC
TGAGGGACCAGGGATACAAGATGGCTCTCTCACCTGTTCCAGCTGTTAGAGCTGTCCCTAGTGGCCACCTCTCCTTTGGC
GGAAAAGGAGCACAGGGTCTAGAACTCAGATCTGCTGTGTTGTATCATTTTGATTTACTTATCATGAATCATAATACAAT
AGGAATTCTCATGTGGGCATGCACTAAGAAAGGGAATTGTTTGCCCTGGGATTGGTCAGGGGATCCGTTTAAAGCTGTGA
CTGTTTTCTTTTCTACCTGTACCTCCACAGCCTCTCTTAATGCTGTCTATAGCAAAAATCAGCATCATTGTGTATTACT
AGAGTTGCCAAATTTGGAGACTCTATAATGGATCCTCATCAAAATTAAAATAAATCCCCAAATTAATCACAACTGGGGAT
GGAGTAGGCTTTTCACATTCATGACCCCATTTCCTTCAATAGGAGCACACCTTGTCTAGCAAGGCCATATCTCCTAATTC
TTCCTAAACAGCTCCACCAACTAGGTACCAAGCATTTGAATATTTGAGTCTATGGGGCCATTCTCATTCACCATAGTGTT
ATATAGAAATATGGGTAAAGGACTTTATGAGCCATCTCTGCTAAAGGGAAACATTGTTCTGTTCATGGTAAAGCTCTTTG
TCATGTACTAAGTGCTGGATAGATGGCTCAGCTATGCAAAGCTAGGATCACAACTGAAAAGACAAGAACAATGGCTAAAA
CTTCCCATTGTTAGACACTCTTTCCATCATGAATTTTGGGAGGTGAAGAACTAAATGAGAAGGTCAAAACCAGGCAACCT
CATTAGCTCAAAAAAAACCTGAACAAAGACTACCTTCAGCAAGGATGGAAAATGTGGTGTTGGGTTGCTTCTGACTTACC
TCCTGTGCTAGAAGACCTCAGGAGATAAATTCTCAGATCCTTGTTTTGCACCTCAAATTTGTGCTGACATCTGAGAAAAT
TACCGCTTGGACATCAGAAAATCTGAGTGACCCATCTTCAACTGCATTGACAATTTCCAAGTAACAGAATTTAAAAACCC
```

```
TCTGACTACATTTCTATTAACTCTCATTTTATAATATATTTTATTTAATATATTATATTACTATATTAATTATACATCCT
TATATTTTATATAGTTAATATGCTAAATATTATATTTTTTAAATTTAGCCTTGGTTGACTTGGAGCTCCCTATGTAGACC
AGGCTAGGCTCTGATTCATAGAGCTCTACCTGTGACAGCGTCCCCAGGGCCGGGATCAGAGCCCATGTGCTACCATACCA
TCACCTAAATATTTTATCATAAATACTTTTTAAATAGTCTGAGACATTTGCAAATTCCTTGCCATATGTTCCTAAATGAT
AAAAATAATTCTAAACGCATCTTGCATGAGGATGAGGATGTTCTGCTGTTACATTTTTGGGGTACCTACATTCTAAACCC
AAATTCTTTAAGCCTGTGAACTCAGCTCTGAGCAATCTTACACCCTCTCCTGGCCTCTACGGGCACCTGCATGGTTCCAC
TGAGAGTCACACCCACATATATAGTCAAAATCTAGAGCAGTTTTTCTATACCTGTGAGATGCAACCTCTTTTTTGGGGTG
CGGGGGTCGAATTACCCTTTCACAGGGGTCTCATATGAGACATCCTGCATATCAAATATTTACATTATAATTCATAATCG
TACCAGAATTACAGTTATGAAATATCAACAAAATAATTTTATGGTTGAGAGTCACCACAATGTGCAGAACTGTATTGAAG
AGTCACAGTATGGGCAAAGTTGAGAAACACTGGTCTAGAGGCATTCCTTGTGCTGATAAAGGTGGCAGTGGGCATTATTC
TGTCTCCATACCAGTAGCAAAATGTTTCCATATGTAAACATGTAATATTAGACAGCCTGAGTCTGTAGTCCTGAGGTAGG
ATCTGGCTCCAGAACAAAGGATCTCTTATTCCCTGAGAGGCAAGAAGTTTGTTTAGCTCTGACTGTGATCCCCAACTCTC
TCCTTTCTGACTCTGTCTCCTAGAGATTCTGTCTCTGGGCTTTTCAACACACAGACTCCTCTGACCTTGCCCTGGATGTC
CCTGTAAAATGCCCCCAAAACCCAGAGCTGTAACAACGTGGGGCTCGCCTACCTTAAAGATCACTGTCTGATGTGTGGTA
TTCAGGGCAGGAAAGGATTGGTAATATTTTCTTTTTGTTCTTGTCATGTTTGTGTGTGTGTTTGTGTGTTTTTTTGTTGT
CGTCTTTTATTTCCTAATTGATTCCCGCTGGACAGTAAAACTGGTCTCACCCTCCAGCCCCTTTTGGCTTTGTTAAAAGC
TCTTTGTCTACAGCATTAGTTTCACTTCATTACAGTCTAATGAAATGCCTCCCTTACAATGCTTCAATTTGAGTCAAACA
AGTTTGCACAGGAAGGAGTAGCTCAAGCTTCCGGATTGGCCAGTTGGGCTAAATACTGAAACCTCCTCTGTATTTGTGCT
TCTCGGCTTTCAATTTTCAATCCTGTAGTGGAAACTCAGCTTCTCAGCTCTGAGATGAGTGTTACAAAGGCTTCCCTTCC
TATGCTCAGCACTACAAGCAGCCCGCAGGAGGGAGAAGTGGGTATGTATTCAATACTATTTGAACCAAACTATAAGAGCA
GAGAGGAGCTTCACCAGGGAAGGAAGGCAAAAGAGCTGAACCTTGAACAAAATAAGGACAGAAGGGCGTCTGTGAGCCTG
TCAAGGTGTGTCTGCAGAGCAGGAGAATGCAGATCGGATTAGCTATGAGGTTGTTACATTAGTTATTCTATTGGAGTATA
CAATACTCAAATAGTTCTCAGGCAAGAGAAATGAACAGTGAGTCACCTTCTAAGAGCCACAGCTTTCTTTCTTTGTACTT
ATCTTGCTAGAGCACTCTTCCTAGGGATCTGGTAAGCTACAATCTGGTGGATTACCTTATTGTGGAAAATGCTAGCTCTA
CAAGTCCCTGAGTGGAGGAAATTCAAACAGTAGCTTTGCAATGTGCATAGTGACAGAAGTTGCCTGGGAAGCTTGGGGTT
TTTGTTTTTCAGATCCATTGTAATTGAAAAGAATTGTAAGGAGGTGGCATGGGGTGGGGGTGGGGAGTTGAACCTACCC
AGGACCACATCCTTTCCATTTTTTTGAGCTCTTGATAAGTTATAGAAAAGAATATAATGAGTTTTCTTCTTCAGCCTTC
AGAAAGGAAGCAAAAGTGTGTTTCTTGTGTCTCAAACTGTCTATGCTTCATTATATTGTGTTCCTTTATCTTCCTTTTTT
CCTTTATTACTGTTCCTTCATCTCTTTTTTTCTTTTTGTCATGTATTGAAGAATACTGGATTTTGAGAAAACAAGATCGAT
ATTTATCAAAATACAAAATTCACAACAAATGTTAATGCTTATCACTTAAGCCAAGTATTCTGACTGAAACTATTATTATT
ATTACTGACAATGATGATGACAATTGATTGATTCTTCGGCATTTTGAATAATGTTAGGACAGTGCCATAGCAAGAAGAGC
AAATATGAAGCATTTTGTCAATAGAAACTGAGTTTTGGTAAAGAAGTGCTACAGACAGACCCCTTTTTTGGGTCTCCTAT
CTGCATGGATCCGGCTACTGGTTGCAGGTGGGCGAAGGAGTCAGTGAAAGTGGGGAGACAAACAGACACAACACGAGAGA
GTGTTTTGAATCTGAATGTAATGTCCAAAGGAAAAACAGACTTTTTTATACAGAAGAAAAACAAAACAAAACAAAACAAA
AACAAGAAAAGCCAGGCGGGACATATCTGCCAAGTTATAGTGACACAAAACAAAAGGAATGTATACATCAAAAGGTGGTG
GAGGATCAGGCTGCATTTACCACTTAGAATGGAGCCAGGTGTAATGCTAGTCTATGTTAAACCGGCAACCAGAGGTTCTT
AATAAATACCTGATAATGCTGTTCCTTTGGTCCTCGTGAAGAAA
```

MOUSE mRNA SEQUENCE : mR14-023.1 (Seq ID No: 108)
```
CAATCCTATAGTGGTAACTCAGCTTCTCAGCTCTGAGATGAACATTACAAGGGCTTCCCTACCTATGCTCAGCACCACTT
GCAGCTGCAGGAGGGAGAAGTGGAATTTCCTAGGGAGATACGAGGGGACTTTTGACTACTGGATCGGCCTGCACAGAGCG
TCGTCTAAGCACCCTTGGATGTGGACAGACAACACTGAGTATAACAACATGTTTGTGTATCACATGAATGCACAGTGCCT
AAAGAAGCCAGAAGAAGGCGAAGGCTCCCCCGGAACTGGAGGTGTGCAATGTTATAAAATTCTCCAAAGAAAAAGTCTCA
GAGCCATCTCCCCTGAGTCTTCTGCTAAGCTTTACTGCTGCTATGGAGTGATCATGGTCCTCACTGTAGCTGTAGTTGCT
CTTTCTGTTGCTTTGTCAGTAACAAAGACAGAACAGATCCTAATCAACAAGACCTATGCTGCTTGCCCGAAAAACTGGAT
TGGAGTTGGAAATAAATGTTTTTATTTTTCTGAATACACAAGTAACTGGACATTTGCCCAGACCTTCTGCATGGCACAAG
AGGCCCAACTAGCTCGGTTTGACAACGAGAAGGAGCTGAATTTCCTAATGAGATACAAGGCAAATTTTGATTCCTGGATT
GGACTGCACAGAGAGTCGTCAGAGCACCCTTGGAAGTGGACAGACAACACTGAGTATAACAACATGATTCCCATCCAGGG
AGTGGAAACATGTGCCTACCTGAGCGGCAATGGGATCAGCAGTTCCAGGCACTATATACCTCGGATATGGATCTGTAGCA
AGCTTAACAACTATAGCCTCCACTGCCCAACTCCTGTTCCTGTCTAGCATTTACCAAGAGACTCTTCCTAGCCTGTTATC
TATGGGTGCTACTTTTTCCCCTATGGTCCCACAGTGCTATCAAACGGGATTGAGAATATTTTTAACGTCGCAAATGAAA
ACCATCAAGGCTGGAGAGATTGCTCCGTAGTTAAGAGACTGACTGCTCTTCTGCATGTCCCGAGTTCACATCTGAGCAAC
CACATGGTGTCTTACAAACATCTGTAATGACATCTTATGTCCTCTTCTGTGGTGTGTGAAACAGCTACACTATACCTAC
ATATGATAAATAAGTAAATCTTAAAAAAGAAAAAGAAACCACCTTAGAGAGGTGCACACATGGAGGATTACAAGACCAT
AGATGAGTTTTAAATAGATGTCAGCACTCATACCTTAAGCCTAAAGTACAACTAATGTTAGGGAACCCCACTTTTATGAT
ATTAAGGTTTTGTGCAGAGAATTCTTCTTTTGAATTTATGAGACCACAAAAATGAGTCCCCAACATGGGTGTAACCTTT
AATAATGAAAGCAGAATGGCTGGGATCCTGAGAAGCATCTGTAATCCCAGGTACCGAAGGGTTGACACAGGAGGGTCTCA
GGTTCACCAGTGGGGACGGAAAGCACAAAAACACTTTAATAATGATAGGGAAGATGAGATGTCTGTTGCATCCCGTAATT
TGAAGAAACATATTATACATGCTTTTCAAAAATTCAAACCAACATACAGAAGAATTTTTAAAAAGTGTGTAAAATTAAGA
TTTACTTTTTACTCTGCAATTTAAAAAGATGAGGGACATTGGCGAAATGTTTCTCCACTTTAATTTTAATATTCCTATTT
CCAATTATACTGTTCTAAGTATAATTATACTTACTTACTTTTCCTGTTCTAAGTGTACTGCATTTTATAATCAAATACCA
```

```
TTTTAATTTCACTATTTTATTCCAGGAGCCTTCTGGCCCAGGCCAGTGTAATGGTGGAGTCTATATAGAATCACTACAAG
ATAGTTCATCTTTGTGGATCTGCAGAGATCTGCTTCAAAGGGGGAGTGTTTCTACTCAGCAATTGTTATATATGTTTAAC
AATAACAGTAAAAGCACATTAATAGCAGGAATCTCTCCTAAAACGAGTCACTCATAGCCTTGCTTAATGAGGGCTCACCT
GTCACAGATTATATAATAATCTGAGGCAGGCCACTTCAGGATGATCACCTGCTAGTTATTAGCTTGTCTCATTATGGGTC
CTGACCATTTTTTTTGGATTTATTTCACATCAAATGTGAGTTAAACTCCTTAGGCATTTAATACTTAATTTTACACTAGC
ATGTGCATTACTGAGCCTTTAAAAAAATATTGAAAATCTTATAGATTGAGCTACCATGTACTGATGAGTCCC
```

MOUSE PROTEIN SEQUENCE : mP14-023.1 (Seq ID No: 109)
```
NPIVVTQLLSSEMNITRASLPMLSTTCSCRREKWNFLGRYEGTFDYWIGLHRASSKHPWMWTDNTEYNNMFVYHMNAQCL
KKPEEGEGSPGTGGVQCYKILQRKSLRAISPESSAKLYCCYGVIMVLTVAVVALSVALSVTKTEQILINKTYAACPKNWI
GVGNKCFYFSEYTSNWTFAQTFCMAQEAQLARFDNEKELNFLMRYKANFDSWIGLHRESSEHPWKWTDNTEYNNMIPIQG
VETCAYLSGNGISSSRHYIPRIWICSKLNNYSLHCPTPVPV*
```

MOUSE PANTHER CLASSIFICATIONS
       FAMILY (SUBFAMILY)
              NKG2 TYPE II INTEGRAL MEMBRANE PROTEIN(C-TYPE LECTIN-RELATED)
       BIOLOGICAL PROCESS
              Immunity and defense(2.16.00.00.00) > Natural killer cell mediated immunity(2.16.07.00.00)
              Immunity and defense(2.16.00.00.00) > B-cell- and antibody-mediated immunity(2.16.02.00.00)
       MOLECULAR FUNCTIONS
              Receptor(1.01.00.00.00) > Other receptor(1.01.99.00.00)
              Signaling molecule(1.02.00.00.00) > Membrane-bound signaling molecule(1.02.07.00.00)
              Defense/immunity protein(1.25.00.00.00) > Other defense and immunity protein(1.25.99.00.00)

MOUSE GENE ONTOLOGY
       BIOLOGICAL PROCESS
              humoral defense mechanism > antimicrobial response
              stress response > defence response
              signal transduction > cell surface receptor linked signal transduction
              defence response > cellular defense response
              intracellular protein traffic > endocytosis
       MOLECULAR FUNCTION
              sugar binding > lectin
              molecular_function unknown > lymphocyte antigen
              lectin > mannose binding lectin
              mannose binding > mannose binding lectin
              protein binding > lipoprotein binding
              defense/immunity protein > major histocompatibility complex antigen
       CELL COMPONENT
              plasma membrane > integral plasma membrane protein
              cell > membrane fraction
              cell > plasma membrane
              extracellular > extracellular space
MOUSE PROTEIN DOMAINS (INTERPRO SIGNATURES)
              IPR002353 (ANTIFREEZEII)
              IPR001304 (CLECT)
              IPR001304 (CLECT)
              IPR001304 (lectin c)
              IPR001304 (lectin c)
              IPR001304 (C TYPE LECTIN 2)
              IPR001304 (C TYPE LECTIN 2)

HUMAN GENOMIC SEQUENCE : hD14-023 (Seq ID No: 110)
```
CCAGCTGGAAACCACCGGACTGACGATTGTTGTGCTTAGGATAAGGCACTTTGTGATGAGTTAGGAAAACAACAATCAGA
TATGTACACTTCAAAATTGACCTCAGGAAGGTAAGCAGAAAAATAGATAAAATGGGTGAGAAAATTTCTTTATTATGTTT
CATTGTGATTGTCTCACTTTTAGCAAGAAAGGAAAAGCCAGTGGTAGAGTCATCTAAATACGCTGCTTGCCCAAAAAGCT
```

```
GAATTAGATTTAGGAGTAAATGTTTTTATTTTTCTGAAGGCATAAGAAATTAGACATTGAGTCAAACGTTCTGTGTCTCA
TTAGAAGCCAGCCTTGCTCAGTTTGCAACTATGGAGGAACTGGTAAGAGATATTTTTGGATCATGTTTTCTGTTAAATAC
TTAGCCCCCTGAGGCAGGTCGGCTACATAGGGTTAAAGCTGAAATACTAAGAAGGATCCCATTCCAGGGGCATGAGATGT
GTTTTCATGTCTAACATTTGTTGAGGGTGTGTTCTCTGCCTGGAACCCTGTCAGACATTTTAGATATATTTCCCCATGTA
ATGCTCATGGTGAGCTGGTAAGATGAGATGTATTGTTCTCATTCTATTAAGAAGTAAGGTAATTATATAATTTGTCATCT
AGATTAAGACATATAGGGCATTAAAAGTGATCATTAATACATTGGTGGAATGACAAGCATAAACTGAGAATTTCCCAATG
AAACTGGGATTTATGCTCACTTTAAATAAAGTAATTTAGGATCAGAGACGTTAAATGACTTAAACTGAGAACTGGGAATT
AGTCTCACTCCAGAATTTATGCTGCTTAATTATACCACGCTGCCAGTGAAAAAGAGAAGGGATAGGGAAGAGAGACTCCA
GAGTAATATGAAAGAAAGGGGCTCATGGTGTTAGTTTCTGTTTTATTATAGAATTTCCTGAAAAGATACAAAGGCCCTTC
TGACCATTGGATTGGCCTTAGAAGAGAATCATCCCATCGCATTTGGAAATGGACAGACAACATGGAATATAATAACATGT
ATGTTTTGAGACTTCTCATCTTTTGCTGTGTTTATGTATGGTTATGTGTGTGAGTTGTGTTTATGAGATAAGAAATGTAG
TATCATGGAAAACTTACTGCAATATGAGGTAAGAAATCTAAACCCTTTTTATGCTCTTCAGTTGGTTAGCTGTGTCTTTG
GGAAAGCTTTACCTGTGATTTCAGTTGAAGTCTTTAATGTGACAGTGACATTTCTCACAATTTTACACTTTCATCCACTG
ATTTAAAACTGCTGAAATCCCTACTTATAGCTGATGTGCAACAAAGGTTCTACAATCATAGGAAGCCACTGAGGAGCTGA
TATCCACAAGGCTGTTAAGCCCACTCTTGCAGGTGTTGCATCCATTTACAATAGGAAAGCATCCAGGGAACTCATGGGAA
TCTCTCCCTCCTAAAGCTTTATCACCAGAAGGAAACTAGTAACAGTTCCTTGGACTGTGAACATTTCAAAGGGGTATTTT
GAATATAATCATTACATTGCAGCAGATAAAATGTAGGAACCCAGTCATATCCATTAATCAAAGCTAAGCCCTTTCAGGAA
TTTACTAGTTTTTTTTTTTTTTTTTTAGTTTTCACACATTTATAAAACAATTTGGGAGGAGGCTGGAAATCATCTTGACTGA
AGACCACCAATGGCAGCAGTTCATTTGGGTACAGCAGCAAGCATCAGTTAAATACCTATCAACATAGCTGGGCTTGGTAT
GGATGAATGATAAGCTTGTGATTTTCAAGCAAGGACCTTGAGGACATAAAGGACACTAAATACAGAAATGGTTTATATTA
CTATGAAGCAATATTAATGACAGACTTTTGCTTTTCTCTTATTCACATTGAAATATGGAGGGGTTGCAGAAGATGATGGG
GAGGAACAAATGCAGGTGTCTAACTTACAAATATTTAATATTTTTGTGTTTGGATTTAGGCTTGCTATCAGAGGAAGTGG
AGAATGTGCCTTCCTGAATGACAATGGAGTCAACAGTGGCAGAATCTACATGAACAGAAATGGATTTGTAGCAAGCCAA
ACAATTATGTCTACAGTTGCCAGTTATGTCCCCACTGGGATACTACCTAGTAGAGCTGTGAGAAGAGGGCCACCATCCTC
CAGACTCCAGAATGGTGGAATCATCAGCAGCTTCCACCATGCCCCTGGAAAAACTGCAAGTAACAGACCTGCACATGTAT
CCCCTACATCTAAAATAAAAGTTGGAAAAAATGGAACTTTGATGTTAAGATGCACTGATACAAAACTTAAATTTTGGTCT
CCTACATTAAAACAATATGAGTCTCTTAAAATATTGATTTGTTCTTAGTAAGATTACAAGAGGCTTTGATTTTTAATTCT
GCAATGTTTCCTTAACAGCCATTCTGAATTACAAACTAATTCCTATTTCTGCCAACATTCCTTCCTGAGAACTGTCTAGTC
CTGGTTTGGGATTTATCTTAAAGGTCTAGAAAGGCAACATTTTACTTCAGCAAAACTTGATTCTATATTGTTGTTTTTTC
TTAATGTGTCAAAATTGTTTTGTTACTAGGATTTCCTATGTTGTTATTAAATGTATTCCCCTGATCAAGGTCACTGGCCT
AGAAAACAAAGATTCTGTGTTTTACCAAGATAATTCCCAGTGCTTCATGGTGTCTTTATTAGGCTTTGACTGGTTAGGGA
AACTGAGCCTTAAAAGGGTAAAAGTTTTTCCATCCATGTAACTTTCTGAGGTCTTTAGGTTGTCACTCTGATTAAATGAG
TAGTATTCTTAGCAGTGACTTATTATCTGTTGAAGGAAGTATTTTAAGCTTTTTGACATCTTTGGCAGGTTTCTCTAGAA
TCCAAATCCTAAATTAAGCCTTTTGGCCTAAAATTGACTGGGATTTTTCCAGGTGGACCCCCTAGATATGCTCCTCCAAA
GGCTTGTCTTTCTTGTAGAGTTATTAAATGATTAGGCTTATTTGGCAAATACTGCTAGAAGCATTGTCAAATAAGAAATG
GTGTTTTACCTTTCTTTAAGCTGTGTTTGTGTAAGTTCATCATTTATAGAAATGTGAAATTTTATGAGATTCCTAAGATA
CTGACATGCTGAGATACCTGTAATAATTGCAATTTTTATGTTGGATGATTGTATGCCACAGAAGCACTTAAATTTCCTGG
TTAATTGGTAACGTTAATCAGATCTTTACCTATGGCTGTTCTGGGTTTTTGTCATTCACAGTTATTATTTTAATTTCTTC
TTTGGAAGCGTTCGCAATCAGCTATAGTCCAAAATTGCTAATTAAGATCAAGCAAAACAAAATTAATTACGTAAAATTAA
TTTATAAGGATGTTTTTATGACGTAAATGTTTTATTTTCCAGAGCCAAGGAAATTTTCTGTCACAAGCTATCTACCGTTT
GCAATAATTTGATAAATAATCCTTTGTGAACAAATATGGAAGCATTTGCTTTTTCTCTCTACTTGATTCCTTCAAAATTC
AGAAACTATTTGTGGATATTTTTAGTTTTATTTATACAACTTCAGTAAAAATCCACTCTCTCTTTATAAGCAGGATCCAG
TTGGAAACATTATTTATATTGCTAAGGTTTGGCTTGAAATGTCATATTTCAGAATGTGCATAGAGGCCAGGGGTGGTGGA
TCACACCTGTAATCCCAGCATGTTGGCAGGCTGAGGTGGGTGGATCACTTGAGGCCAGGAGTTTGAGATCAGCCTGGTCA
AAATGGTGAAACCTCATCTCTTCTAAAAACACAAAATTAGCCAGGCATCGTGGCGCACGCCTGTAGTCCCAGCTACTCGA
GAGGCTGAGGCATGAGAATCGCTTGAACCCAGGAGGTGGAGGTTGCAGTGAGTGGAAATCCCACCACTGCACTCCAGCCT
GGGCGACAGCGTGAGACTGTGTCTCAGAAAAAAAAAAAAAAGTGCGTAAAATGCCTTGCTTCATGAGTTCCCAGCTTTACA
GGGAATGAGTAAAAATTGTCACTTTCTGGCAGGTTCAAGAACCTTATTATAAGCAAATCCAAAGGCTGCTTTGGTTTGG
TTTCCTAGCCTCAAGAGGTTCTAAAATCTAAGACCTCCTACATCATCAACGGGAGAGAAAAATTATGCTTCTAAGGAAAG
CAAAGTTACACCTGTTTTTAGATTTTAACCCTGTACATTGTCTTCAAGTCCTTGTTATCTGCCTGTGGACTGAACTAGAT
CCTGAATTCTCCTAAGTTCCTCCAATATTAGGTTATAACAGTCCTGATGGAGTCCCCCAACCCTCTTGCCTCGATCCTGA
CTAGGGATGCTCCAGGGACATTCAGGTGACTTCCCCTTCTTAAAAATCTGACCAGCTAGGGACATTAGATACCAGAATTG
GGAACAAACTGGATCCATAAGATACTATGGTCAGTAATGAGATCTTATTGGTCAGTGACTTTTTTTTTTTTTTAAATGG
AGTCTCACTCTGTCACCCAGAGTGGAGTGCAGTGGCATGATCTCAGCTCGCTACAACCTCCATCTCCCGAGTTCAAGTG
ATTCTCATGCCTCAGCCCCTCAATTAGCTGGGGTTACAGGCATGTGCCACCACACCTGGCTAATTTTTGTATTTTTAGTA
GAGATTGGGTTTTGCCGTGTGGGCCAGACTGGCCTCTGGCCTCGAACTCCTGACCTCAGGTGATCCACCTGCCTCGGCCT
CCCAAAGTGTTGAGATTACAGGCATGAGCCACTGCACCTGGCCTGCTCAGTGACTTCTAACATTGCTGCTGTACCTGGGT
AAATACTTCCAGTCATATTTAAGCGGGCTTGGAGTTGGCAAAATCTCTGAGAGAGACATCTTCGGAAAGCCTCCATGCTG
GACTTAGTGGATAAAGTTTCCAATGTCCAGATATTTTCAGCTGGTTTCTCCTTGGTATAGGATTTCTTCTGCATTCTACC
CTGCACATCTTAATGATAACCCTCATATTTTGGCTAAGCATTTGACTCCCCTTTAGAATTGTTCTAACTTGTTTTTAACA
GATGTCTACAAGAGATTCCCACCAGGATCCTGCCCATGCAATCTTTGAGACTTTAAACTCACTCCAGCTGGAAATAGGAA
```

```
CCAACTTAACCCCAAGAGACTTTGGTTCACATTTAACCAGTACTTTCCTGAATGCCTCTCCTAAGTAAATGGCTCTAGTTT
CTCTATTGGCCACTGCACTGCCACTAGGATTCCTGCAAGGGATCCGATGGGCCCAGAGCAGGTAGCCAACCACTTCTCTG
TTGGATGGAATGCAATGCTGTTTGCCAGCATGTTTGAGCCAGTCCTTCAAGATGGATTCTGAGTGACTAACTGGACCTAA
ATTTAAATAGCACAAAGCAGCCATTTGCTAACTAGATTTCATACAAATGCTCTGAGTTCCTGGAAAACCCACACTCCTTA
ACTTTGGGACTTTCAAGACTCACCTAAATCAATCAATTAAAGCTCACTTGTGTCAGCTAATCAGGGATCAGCTGTATTGA
CTAATGAAAACTAAGCGAGTTTCAAATTTTAACCATTTATTTGTGTAGAAGACTTGATTGGGAGCCTGGGTATGAATACT
TGCTATAAAATCTGAGCATTCCCTTTGTTCTCTGGAGCAAACAATGTTAAGTTGTTAACTCTCTGTACCTTTCTCTAGAA
CTGGATGGAAACAACTCCATCTGAAATGCAGCCCTAATACCAGGGAACTCCTACAGTAAAGCGCTAAACAACTTTATTAC
TCTAACCAACGTTTATGTCCCTAATCAGCAAGAAGTAGTTAGAATGGTCATCATCCCTTTCCCTCAAGATTGAGGAATGG
ACATAAAAAGGAGGGGATTTGTAACTGCTCCAGACTGATCTGTTGTCAGAAAGGGGCTCTTGAACCAGACCATGAAAGAG
GGTTCTTGGATCTCCTGCAGGAAGGAATTCAAGGAGAGTCACAGAGTGTAGAGAGAATAGACAGTTTATTGAAAGCTACT
CAGTTACAGAGTAGGGCATCCTCCAAAAGCAGGAGGAGGAATGTGCTGTCTTTGTTTTAAACTCTTCTTTTATAGGGGTT
TAATCTATGTAAAACCTAAGCTATGTCTACATGTGGGTAGGCTGTCAGTGTGACAAAATTTAGTATTTTGTTGATATAAA
GAAACTTATCCTTGTCATCTTAGTGCATAAGTACATCAAAGTATGACTTTAGCTACCTTAAAAGCATGTATTGTTATGTG
ATATTGGGACATCTGGACATTCTGCTGTCGTAGGAGTTTGTCCTTGCAGCATTACTAAATCGCTTCCTTAGCTGTAAACA
TCTTATAATCGTAGGTCATAACTGTCAAGGAAGTGCCTTGCTAGTTTTTAGATGGAGTTGATTTTAAAATAGTGTCACTC
TGGCTTTCCTATGCTCCTGCTTTCCTAACAAATCTGGTTCAACTTTTTTTTTTTTTATTCAACATAATCTTTTTTTTCCAT
ACTACCTCATTTACAGAGTGCTTCCTATAGATATGGTTGGTCTGTATGTTTCCTTTTTCAGCTTTGATTCTTATTTTTTT
GAATCACACATGCTTTATGGGGGCTTCCACTACCATGAGCCATATTTTCTGTATTACCAGCAAGATGTTTGGGTTTGTTT
TTTGTTTTCTTTATATTTCAAATTTTATTTTAGATTTAGGAAGTACGTGTGCAGGTTTGTTAACATGGGTGTATTGCATG
ACGCTGATCTTACAATACCAATGATCCCATCACTCAGGTAGTGAGCATAATATCCAATAGGTAGTTTTTCAGAACTTGCC
TTATCTTCCCTCTCTCCCTGCTCTCTTAATTCGAAGCCTATTTTTCCTATCTTTATGTCCACATGTTTCCAGCATTTAGC
TCCCACTTATACGTGAGAGTATGTGGTATTTGTTTTTCTGTGTCTGCATTAATTTACTTAGGATGATGGCTTCCAGCTGC
ATCTTTTGTTGCAAAGGACATGACTTTGTTCTTTTTTATGACTGTGTAGTATTCCATGGTGTATATGTACCACATTTTCT
TTATCCAGAACACTGTTGATAGGCATCTACATTGATTCCATGTCTTGGCTATTGTGAATAGTGCAGCAATAAACATGCAT
GGTTCAACTTTTATGGTAACAAAGCTGTGAGTTGTTCTTCAGTTGTCATGGACCCTCACTCAGGTCATGTAACCTGAGCA
TGGACAGGTGAAACAAGTGTGCAACCACAAGGGAAACCTAATCGTTCAGACTGAGGTACAGGGACCGAATTAAGAGGTGG
ACACAGGATGGCAGGATCCAGGATCCAACTGGATAGAACTCAGGCATCACTTCATGGCAGGATCCAGTCAGATCTTGCCT
CCCAGCATCACCTCATTGCAAGATCCAATCAGATTATTCCTCACTACCCTATGCTTATAAAACCTGACCCACCTCCCAGC
TTGGCGAGGTACTACTTTGGGAGCTTTGTCCAGTGTTCTCTTTACTTTCTGTAAGAACAAAATCTCCTTGCTAAATCCAC
CTTGTCGGTGGTCATTGGGTTGATACCTGCCAAGTGACTGAACCCACCTGTTGTGTGGGTAACATTTAGAACATATTTTC
CATTTCTTTATTTTCCTTGACTGTCTGCATTGGTGTCTGTGCACCAGATAAAACAGCTTCCTTTCCAGTCTTCACAGACA
GGTCTCAGATAGGACAACACCTTTACCAATCAGTCCAGACAGAGGTTCTGGGTGCCACTTAAACCTTCCTGGTAGTTGAA
ACCACAATGTTTGTTCTTAATGGTCCTCAGGCATCTAGAGTATGCCAGGCCCTTCCAGTGCTCTCAGATACATGAGATAA
AATCCAGTTCCTCAGGTAGCAGCCAGAAAAGCTGGGGTGCTAGTTATTTGGTCTGTCTCCTTTGCTTTTCAGAGAGAAGC
TGGAAGCCAGGCTTTATCACTGCACTGAGCCAAAGTGGGGAGCAGGGGGAAGTGCCCACATGCTTATTAAAAACCGCTCT
GAAAATATTTGAAGTAACAGGGCTATAACAATTATTATTTTAAAAGCAACTGCTGTTTTGGTTTGTGTAGCCCCAGAGGT
TTAGCAGATGCTGGGCCCTGTCAGCTCACCAAAACAGGGAAATTAGAAACCAGTCTATCTGGTAGTATCTAGAGAAGTTG
TGATACCAGATAGGTGGTCCAAACCCTTCACTCCTCAGGGATAGACTAAGAGATGGGAGTTCCTTCCCGATCATATGGCA
TTGTATTAGGGTTGGGGGTTATGATCACAGTGTGTGCAAGTTTTTCCTATCAGTTTTGATGTGGATAGTTTCACACTCA
CCTAGGGTGCAGGAGTGGCTCAGTCAGTTTCTGGATTTCCAATGATGGGAATTTGCCTCTGTGTTGCTGTTGAATTGATG
GTGGGGAAGGAGAGTTTAGGGCCTTCTATTCTCGCACAAATGACAACTTATATTTACACAAAAGCCTGTATATAAAATGTGC
TCTAGTTACTACATTTGTAGACATTTCTCTCGCACAAATGACAACTTATATTTACACAAAAGCCTGTATATAAAATGTGC
ATAACAGCTTTATTCATAATAGTCAAAACTTGTAAACAACTGTATGTCTCACAATGGGTGAAAAATTAAGTAAATTCTGT
TATAGGCATACAGTGGATTACTACTCAGCAATAAAAAGGAATGGGCTATTGATACATATAACAACTTCCTTGAATCTCAA
AGTACTTAGGCTAAGTAAGAAAAGCCAATTACTGAAGATCGCATTCTGTGTGATTTAATTTACGTAACATTATTGAAATG
ACAAAATGGTATAGATAGAGAATGAGGGTTATGGATGGGTGAGTGGAGAAGAAGGGGCAGTATGGAGAAAGGGTGGTGTG
GCTCTCCGTCGTGATGGAAGAGTTTCGTTTCCTCATTGTATCAATGGCATTATCTTAGTCGTGATATTATAGTGTAGTTT
TTTAGATGTTACCATTGGGAGAAACTGAGTAGGAAACTGAGTAAGAAGATGTGGGATCTTTCTTTAGTATTTCTTAGAACTGCATGTGAAT
CTACAACTAGCTCAAAATAAAAAGTTTAATTATAAAATAAAAGCTACATGAAATGAAGCAAAAAATAATTCACCCTTGTC
ACGCACACAGAGTCAGAGACTGTAACATAATTTGCAGGATCTAGAGCAGAATACAAATGTAAAACATCTTGTTAAAAAAT
TATTAATAATTTTGAGACATTGATAAAGCATTAAGCCGCCTGTGGGGCCCTTTAAGCATGATAAACTGTGCTACCACACA
GATTGCACATTCACGTATCTGGCCCTGCAAATGGAATGATTTTTGCCCATGATCAATTCACCATGGCCTCTTTGGGCTCA
GTGAATTTGCTTCTTCAGGAGGGTAATTTTCTCTTCTTTCTCTGCTAAGCTGTTTAACAGTAGTTGCCCTGCCTAATGGG
CTTCATCCATCCATTTCTCTCAGATTATTTTCATGATGCACTAGGATGAAGCACACCCTTTCTCCTAGTCTTGAGGAAAC
GTCGATATTCAGAATATTTAAACGCAGGCACTGACCAATCAGAAGAGTTTCTGGCCAACGTTCCACACTTGAGGGAAATG
ACATTATCTGAGCCCTGAAGAAAACGTTGTAGATATTCTCCAGATCAAAGCATCGACAGGAAGATTTTAGATGTTGAAG
TTCGTAATATTTCCTAAAGCAGGTATGAATTACTAGTAACTTAATAGGTATATTAACTGATGAAGTTTTCATTTCTCAGA
ACAAACCAGTCAAGGAAGGTGCTATTATACTCCTTTTATTCATATAGATCTTGAGGCTGAGACAGTTTAATCAATATGCT
ATAATTATTGTGTAATAATAAATTACCATAAACTAGGGGTGCTATGATCTCAATATTTGTATCTCCCACTCCCAAATTCA
CATGTTGAAATCCTGACTCCCAAGGTGATGGCATTAGGAGATGAAACCTTTGTGAGGTCATTAAGTCATGAGGGTAGAAT
```

```
ACCGATGAATGGGATTAGTGCCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTGAGAAATAAATTTCTGTTGTTTATAAGTTACCCATTTTATGGTATTTTGTTACAGGCACATGAACTAA
GACAAGTGGATTAAAAACAACACAAATTATTATTTTACTGTCTAGAAATGAGAGGTTCAAAATGGCTAAAGTCAGGGTGC
CACTGATAGGGCTGCATTTCTTTTGGAGGCTCTAGGGAAGAAGCCGTTTCCTTGCCTTTTTCAGCTTCCAGAGGCCACCT
GCATTCCTTAGCTTATGGTCCCTTGCTACAGCTTTAAAGCCAGCAGCACAGCACCTTCAAATCTCTCTGACAGTGACA
TTCCTGCCTTCCCCTAATAAAGACCCATGTGATTAGATTAGGCCCACCTGGATTATCCAAAATAATCCCTCTGTCTTTAG
ACCCTTAACCATATGTGCAAAATGTCTTTTGTCATGTAAAATAACATCCTTACCTCAAGGTTTTGGGAATTAGCTTATTA
ATGTCTTTAGGGGGTCATTATTCTGCTTTCCACATACATTTAAAATTAACTATCAGGCTACAAAAGTCATATATGAATTG
AGCTTGTTTTGCCCCAAAGGCTGTGTTCTTTTTTTATTTTTTTATTTTTATTTTTTGAGATGGACTCTCACTGTTGCCC
AGGCAGGAGTGCAGTGGCACAATCTCGGCTCACTGCAACCTCCGCCTCCTGGGTTCAAGTGATTCTCCAGCCTCAGCCTC
CCAAGTAGTTGGGATTACAGGTGCGCACCACCACGCCCAGCTAGTTTTTGTATTTTTAGTAGAGACAAGGTTTTGCCATG
TTGGCCAGGCTGGTCTCCAACTCCGGACCTGAGGTGATCCACCTGCCTCGGCCTCCCAAAGTGCTGGGATTATAGGTGTG
AGCCACCACACCCTGCCAGCTGTGTTCTTTTACATCATAATACACTGCCTTTATATCCCTCACTTATTCAATATCATTA
CCATCCATCATGCTGTCACAAACACAAAAACCCAAGAAAGTGGGATACAGGTCATTGAGAATAAATAATTTTTATAAAC
TTTTGTTCTTTCTTGTAACTTTTTATCAGTCAGCTATTCCAATGAAGCAAAATCTGATAATATATTGCTTTTATACTACT
TAGTTGAGACACTCACATCTATTGAAGAGTAATGGGCACTTTGAAGGGGCTTTTTTCTTTCCTTTTATATCCGGTCAATT
AGTCATTAATTTCTGCCTTCTAATATAGACTCAACTAAAAAATTATATTTTGAAAAAAATTTATTTTCTAAATGAAATTA
AAATTACTCTGTATGTGTACTTCTTTTCCTCACACTGGAATTCTCTAAGGACAGTGTAAATGCACATCACTGTATCTTAT
GTGTGGTATCTTATGTGTGGTATCAGCTATTTAACACAAGGTTGCATAAGAAAGTAAGCACGACGATATGGCTTAGAAGT
CATGAGATTCACATAGTTTAATATAAAGCTCTTGTGATAATTGGAGAGTTATTTGTCACATTCTCCTTTCATAATTTA
TGCTCCAGCTCTCTCAGATTTCAGGTGTTTTCCCAACTCTGAGATATATTCTCTTCTCTCTGGGCCTAACTAAAGAAACT
AGAAGACTTTCTTCAACTGGAAGACTTCTTCCTCCACCTCAAATGCCTCACTTTATTTTTTTAATTAATTAATTAATTT
ATTTATTTATTGTTTTGAGACGGAGTCTCGCTGTTTCGCCCAGGCTGGACTGCAATGGCACTATCTCGGCTCACTGCAAG
CTCTGCCTCCTGGGTTCATGCCATTCTCCTGCCTCAGCCTCCCGAATAGCTGGGACCACAGGCGCCTGCCACCGTGCCCG
GCTAATTTTTTGTATTTTTAGTAGAGACAGGGTTTCACTGTGTTAGCCAGGATGTATGTATTTATTTATTTTGAGACAGA
GTCTCACTCTGTCGCCCACGTGGTGCAACCTTGGCTCACTGCAACCTCTGCCTCTAGGATTCAAGCAATTCTCCTGCCTC
AGCCTCCCAAGTAGCTAGGACTACAGGTGCAAGCCACCATGCCCAGCTAATTTTTGTATTTTTAGTAGAGATAGGATTTC
ACCATGTTGGTCAGGCTGATCTTGAACTCCTGACCTCAGGTGATCCACCCGCCTTGGCCTCCCAAAGTGCTGGGATTGCA
GGTATAAGCCACCATGCCTGGCCTAAATGCCTCACTTTATTTAACTAACTCCAGCTCATTTGATTGGTACTTTCTTGGGA
AAACTTCTTCTGGGTTAAGTGCCCTCATTAACTGCTCTCCTAATAAATTATTCTACTGCCATTCACATCTTGTTACAATA
AGTCATGTATACCTACTTATTTTTCAGTTTCATATTAACTTCCTTAAAGAAAGGGACATTACCCTCAAAGTCCACCTGGC
GTGCTGGCTGAAAGATGTTAAGTTTGAAAATATTTGTTAAAGGGTAAATGATTATGTACATCTTTAGGACTTCATGAACT
TACTTTCAGTTTAGTTTCCACATAAGGAGAAACTTACAGCAACGTAGAAACCAAGAGATTGATATGAATAAATGATGTGA
CTAACTCACAAATACCATGTCATTTGATATTTGACCTTTAAAGTAGTTTTCATATCAGATCTGTCAGTCATGGGTTCTAT
AGCTTACTGTGAACTTAGACATATTAGTTAGTCCCTCACCCAAAATCATCTGAAAAATAAAGGTAATATCAGCCTCATTA
GTTAGTTATGCCAATTAAATAAGAACACATATTTAAATCTCATATTAATGTGTATGGAGCATAGTAAGCAGTAATAACCT
GGCATGTTGTCATGCCAGGCTAGTAACAGTAAAAGTAATAGTAGTTTCACTGGAAGGTCCAAGATTAGTGTGGAAATTCC
AAGATGATTAGTGTGGAAATTCCAAGATAAGTGTGGAAATTTCAATTTATCAGGGTTTACCTTTTGTCCCCTTTAAGCTA
ACAATGAAATTATAAAAGGATATAAAAGGGATAAGCCTGACAGTTCATATCCACTTCTGTGTATGATAATATAACTGGAG
AGACATCAAACTCATGCTGAGAACAACTAGAAGAGTTAGAATTGAAGAAAAAGGATTTCATTAAGATATTAGAGAGTGTT
CAAGGCAACTGGAGGCAGAACGAAGATTCTGGAAAGGGGCCACAGAGAAGTGAGTCAGCATTCCAGGAGCCACCCTTCTC
TTAGGGGCATCTTGGCAGTGGGTCAGATGCTGAGATTCTGGGGAGAGGGCTGTGGATACCAGGCATTTTTTTGGGGGAGA
GAAAAATCTCTTCCTAAAGCACTTGAAGGACCAAAACCCCAGAGAGGAGAGAGGGCCAAAGAAATGAGAAAAATTTTTC
TCTCAAGCAATTTTCAAATTATTAAATTTCACAGGAAAGGAGGTTAGGAAGAACTATGTGAAAAACAGACTTGAACTTTT
GGCAATCACATGTTGTTCAGGAGACAAAACCAAATTCAACTTCCAAGAAAGAGAAGCAGCTTTGATAAAAAGCAGGTTCT
CAGATGGAATTACTAGAAGGCTTTATCATAGGTCCTAGGACAAACTAGAAATGATGAAATAGTAAAGAAAAAGATATATA
AAATCTTACAGAAACTGGAACTCAGTCCTAATGCAACTTCATTTCTATTTGATAAAGGCAATAGCTGTCCAATCTGGAAC
TTATTTCTTACAGGTAAGGGGGGCTACTGTATAGAATTCAAAGCAGGGAAAATATGTTGTCAGAGTTAAGATAGAGATT
GTATTTGGGGTAATGAGCGTTGCAGGACATGAGTAGGGCACATAGCGTGTTGGTATTGTTTTACGTGGTTTTAAGATATA
CTTCATCTAGGCACTTGTTACATGTTATATTTTTAATGAATGTGTTTAGTTGTATGCTTATGATTTGTGCACATTACTAT
AAATTATATTTTAGTAAAAGGTGCCCTTACTGGCATTTATATACAAGAAATCAACACATCTGACAAGAAGCTCTTGCTGC
TAAAGAGCCTCTGTGCTCCTGGATCATGCCAGAGGGAGCACAGAAGCTGCACACCGTTCAAGCCCATTTTTGAAAAGTTG
TTGTAGCAGCCATAGCAGCAAATTCCCAAAATATCTTGAAGAAATTGAAAACAAGATATTTTTGAATGAGCGTAAACTC
TTGGGAATGAATTTTAATTTGAGATTAAAATTGCTGTTGTCTTTACTTACCTAACATTCATTTCCTCTGAGAGCCAACTC
CTTCCTTTCAATAAATTCTTATTTTCTATCATAATATTGCTTTGTACCGCACAAAATACAATTATAATTTTTAATAGTCA
AAGAAATTTTAAGTTATCAAACATTAGTTTCTTTCGTAACCAAAATACTGTTAGAGAAGTAGCTTCAACTTTTTAAAATT
CTTACAAACACCAGTTTATTCTGTTGTGATGAGCTGATTTTTGAAACAATTTATGCTTCATTTGTTCCTCTAGCTATATT
TAACTTAAAGGCTTGACATTTTGAGTTACTGGGGCGGTTACACACTTACACGTTTTAGTCGTTGGGCTTTTTTTTTGTTT
TGTTTTGAATCATTCTTGGGGTTGAAAACGTAGAGACTGTGGTTTAGATCACTCATAGAAACTGGAGGCAAAATGCATGA
CAGTAACAATGTGGAGAAAGACATTACACCATCTGAATTGCCTGCAAACCCAGGTAAGAAGCTGGGCTCTACAGAGTAAG
```

```
TGTGAGGACTGGAATGGGAAGGTAGTGGTAGTGATGGGCTCACACAGGAAAGGTGCTGATCTAGTAAAAAGTTTTCAAGG
ATAAGAGATGGAATGTGGGAGTCCATAAATTGGCTTATAATTTGTGTAAAACATGCATATATTTATATTCCCAGTTCTAA
CAACTAAGGAGAGGGAACAATGAGAGAAAGATGTAAGACAATGAGATAAGGTTAAGTTTAGGATTGAAAATAACAGTTAA
TTTCACTAAAAAAAAATCTATACAAACTCTAAAGTGTTACTCAGATACAAGATACCATCATTATTACATAGGTAGAAAAC
CTATCATGGAAGCTCTTAAGAGAGAAAAGGTAGAAAATAGTATGTAAAGGACATTTACAAATCAAAAAAGTAACAAAGGT
TGAGTAGAAACTTAGTAGGGCATTCTAATGGAATTATGTTACATTGGATGCACTGGAAATATATATATATATAGCTTTAT
ATGATGCTTTAGTAAGTGAAAAAAATGACTTTTGCCACAGTTAGGGAATATCAAGGTTTTATAACTTAGGACTTAAAAGT
TTGGAGTTTTCTCCTCCCAACTTAAAAGGTAATCAATTTGGAGAAAAGGTAATTCAGAGGCAGAAAAGATGAACTCTAA
ATGTTGTTAAATGACTTCATTTATTTCTTCATTCATTGGGTATTTTGAGCATTTTATACCAAATGCTTTAAACTGTTTGT
CGGATAGTTCCAACATCTCTGTCATGTCCTCCTTGGCTTGTGTTTTCTTTCCCCATGGAAGTTGACATTTTCGTGGTTCT
TCATATACTGTGTAATTTTGGATTGTAAATTTGAACATTTTGAACAAATATTTAGTTAGACTGGATCTTATTTAAATTTTG
GAGTATATTGATACTATTGTTTTAGCAAGCAATCACCCTGGTGAGATGAAGACAATCTGTGGGATCTGGTTCCAATGTCA
GTTCCATTCTGAAAGCCTTTGTAGTGCTATTCAGACATGTGCATCAACCAGGGGTGAAATTGGGAACTGTGCAATGCTCT
ACTCATTAGTTCAGGTCTTAGCATCTTTAGTGTGCCAATTAGAACCATATCTAGGCATGCATAAGTTGAGGGGCAGGTGA
ATCCAGGAGTTTATGAATAATGTTCATGGGTCTCTTTCTAAAGTTTCTTCCTCTTCACCATCTGACCCGTACTTTCAGGT
TCCCAGGGGCTTCTCCTTTCCATCCTCCAGCTAGAAATGCGGGCCTCCTTTTATTCTGATGAACCATATACTTCTGCAAT
TATAGCTGCATCTTTAAGCTCATGTGGCAGACGGACAGTGAGAAAGAAGAAAAAAAAAGCAAGAGGTTTGGCCCCACTCT
CATGGAGCTACAGCTCAACTGAATGGAAAGGATTGACTTTAGATTTCTTTTCTTTTTTTGAGGTGGAGTCTCACTCTGTC
GCCCAGGCTAGAGTGCAGTGGCGCGATCTCGGCTCACTGCAAGCCCCGCCTCCTGGGTTCACGCCATTCTGCCTCAGCCT
CCTGAGTAGCTGGGACTACAGGCGCCCGCCACCAGGCCCGGCTACTTTTTGTATTTTTAGTAGAGACGGGGTTTCACCG
TGTTAGCCAGGATAGTCTCGATCTCCTGACTTTGTGATCCGCCCGCCTCCGCCTCCCAAAGTGCTGGGATTACAGGCGTG
AGCCACCGCGCCCGGCCGAGGATTAACCTTTCTCAAGGTTTTGGCTATTGCATGTTGCTGTAGTTTCCCCTACCATCATC
AGATTGCCTGAGGTTTGAGGCACATGAGAATGTAGACAAGAGGGGGAGAAAAAATAAACGTGGTATTTTCACACTCTATT
TGAGATAGGAGTTTGCTTTTCTGTTCCTCAGACCATACCTCTAGTGCTTCTCCTGGAGCCCTCTCTTTATGCTGACAGAG
GCTCAATTCCATGTTTCTGGCTGTATTAAGTTCGGGGCAGTGATACTAGAGGAGGGAAATTTAAATTCACCACTGGTTTG
ATGGCTCTTTAAATTTGGATATTCTTGTAAGATCTGCTGCTAGTATATCCTTTTTCCTTTTGCTCTTTTTTGTTTTGTTT
AAAGACCATATTTTTAGAGCCATTTTGGGATCACAGCAAAATTGTGAGAAAGGTAGTTTTTTTTTTCATTTTCTCCTTGT
TGCCACACATATATAGACTCTGTCTTAGTCTGTTTTGTGCTGCTATTACAGAATACCCAAGACTGGGTAATTTATAATGA
ACAAAATCTATTGGCTCATAGTTCTGGAGGCTGGAAAGTTCAAGACTGAGAAACACATCTTGCTCCATCATCCCATGGCA
AAATTTGGAAGGGCAAGAGAGCACATGCATAAGAAAGAGAGCAAGAGGCACCAAACTTGCTTTTATAACAAATCCACTTT
CGTGATAGTAAACCCACTCCCATTATAAGGACATTATGACCTGATTACCTCTTAAAGGACCTGCCTCTCAACACTATTGC
ATTGGGGATTAAGTATCTAACACATGAACTTTGGAGACACATTCAAACCATAGCAGTCTCTAACATCAACATTCTTCACC
AGCAGCACATTTATTACAATTAATAACCTACATTGGCATATTGTAGCACTCAAAATTCATAATCTGATGGACTTCCCTTT
GTCAGTGACCTGACCTTTCTCCCTGGCTGCCCTTAATATTTTTTCTTCCATTTCGACCTTGGAGTATGTGTCTTGGGGTT
GATCTACTCATGAAGTATCTTATTGGGGTTCTCTGGATTTCCTGAATTTGAATATTGGCCTGTCTTGCTAGGTTGGGGAA
GTTCTCCTGGATTATATCCTGAAGTGTGTGTTTTCCAACTTGGTTCCATCCTCCCTGTCTCTTTCAGGTACTCCAGTCAGTG
GTAGGTTCGGTCTTTTTACATAGTCCCATAGTTCTTGGAAGTTTTGTTCATTTCTTTTCATTCTTTTTTCTCTAATCTTG
TCTGTCTGCCTTATTTCAGCAAGAGAGTCTTCAAGCTCTGATATTTTCTCTTCTGCTTGGTCAATTCAGCTATTGATACT
TGTGTTTGCAACATGATGTTCTCGTGCTGTGTTTTTCAGCTCCATCAGGTCATTTATGTTCCTCTCTGAACTGGTTATTC
TGGTTAACAGCTCCTGTAATCTTTTATCATGCTTCTTAGCTTCTTTGCATTGGGTTAGAACATAATGCTTTAGCTCAGCG
AAATTCGTTATTACCAACTTTCTGAAGCCTACTTCTGTCAGTTTATCCATCTCAGCTTCAGCCTTGTTCTGTGCCCTCAC
TCATCTTCGTGGATTTATCCACCTTTGATCTTTGAGACTGATGACCTTTGAATGGGGTTTTTGTGGAGTCTTTTTTGTAG
ATGTTGTTGTTGTTACTTTCTGTTTGTTTGTTTATCTTCTAACAGTCAGCCTTTCTGAGGCAAGTCTGCTGCAGTTTGCT
GGGGATCCACTCCAGACCCTGTTCACATGGGTATCACCAGTGGAGGCTGCAGAACAGCAGATTGCTGCCTGCTTCTTCCT
TCAGAAGTTTTGCCCCAGAGGGCACTGGCCTGATGCCAGCCGGAACTCTTCTGTATGAGGTGTCTGGTGACCCTTGTTG
GGAAGTCTCACCCAGTCAGGAGGCATGGGATCAAGGACCCGCTTATGTAGAGAATCTGACATTCCCTTAGCAGAGCTGGT
GTGCTGTGCTGGGGGAATCCCACTCATCGGGTTCATCTGGTCTCTTCAGAGCCAGCAGGCAGAAAAGATAAGTCTGCTGA
ACCTGAAACTGCCTCTCCCCTCAGGTTCTCTGACTCAGCGAGATGAGAGTTCTGTCTGTAAGCCCCTGACCAGAGCTGCT
GGAATTCTTGCAGGGATGACCTGCCTGGTGAGGAGGGATGGCTCAGGGTCCCACCTAAAGAGGCAGTCTGGCCACAACCT
GCCACAGCCAGTTTGCTGCACTGTGGGAAATCTGGCCCAGTCCAAACCTCTCAGGCTCCTTTGCACTGTCAGGGGCAAAC
CGCCAACTAATGGTGGTCACCCCTCCCCCCAGGAACTTAGTCATTCCAGGCACACTCCAGACTACTCTGCTGGCAGCAGG
GATCTCAAGCCAGTGGGTCTTAGCTTGCAGGGATCCATGGGAGTAGAACCTACTGATTGAGACTGCTGGGCTCTCCTCCT
TGGCTTCAGCCCCCTTTCCATGGGACTGGATGGTTCTCGTGCCTCACTGGAGTTCCAGGCACCACTGGAGTATGTAAAAA
CTCCTGCAGCTCAGTGCCTGCCTAAACAGCCACCAATGGGAGTGGCTGTCATTGGTTTGCCCAGGTTTGTGCTTGAGACC
CAGGGCCCTGGTGGTGTAGGCTCATGAGGGAATCTTCTGATCTGCAGATTGCAAAAATCTGTGGGAGAAGCATAGTACAC
CTGGTGGGTAGGACAGACCCTCGCCACTTCCCTTACCTCAGGGAGGCAAGTCTCCCCACCCTGTGCAGCTCCTGGGTGAA
ATGACACCCCACCTGCTTTCTTCCTGCTTCCTGTGGGTTGCACCAGCTGCCCAATGAGATGAACTTGGTACCTCAGATGG
AGATGCAGAATTCACCTGCCTTCCGCATTCATCTCACTGGGAGCTTCAGACCAGAGCTGTTTCTGTTTGGCCATTTGGGC
CCCTCCCTTCAAGTATGTTTTCTTTTGTAAGAAACTGCCAAAGTGGCTGTAGCATTTCGCTTTCTTACCAGTAATGCATG
AGACTTCTGGTTGCTCCACATCCTTGCTAGTATTTGATATTGTTAATGTTTTAATGTAACACTTAAGTAATATGTAATGA
```

```
TATCTCATTATTTTAACTTGCAATTCTCAAAAGACATATGATATTAATAATTTGTCATTTCATTATTTTGATTATGTCTT
TTGCAGGTTGAAAGTTTTTAGTTTTAATGAAGTCCAGTTTATCAGTTCTTTCTTTCATGGATTGTGCCTTTGGTATTATA
TTTAAAAAGTCATCACCATACCCAAAGTTATCTAGGCTTTCTCTTATATTATCTTCTACGAGTTTTACAGTTTTGTGTTT
TACCTTTAGGACTATGATACATTTTGAGTCATTTTGGGGAAAAATGTAAAATCTGGGCCTAGATTCATTTATTTGTATGT
GGGTGTCTAGTTATTCAGCATCGTTAGTTGAAAAGACTGTCTTTTCTTAATTGTATTGCTTTGCTTGTTTATGAAAGATC
AGTTGACTATATTTATCTGGGTCTATTTCTTTTTAGTCTGCTACTCTGCTTTCTATCCTTTTTCATTAATTTATTTGTCT
ATTCCTTTTTTTTTTTTTTTTTAAGAGACGGAGTCTCACTCTCTTGCCCAGGCCGGAGTGCAGTGGCGCAATCTCGGCT
CACTGCAAGCTCCGCCTCCTGGGTTCACCCACCATTCTCCTGCCTCAGCCTCCTGAGTAGCTGGGACTACAGACACCTGC
CACCACGCCCGGCTAATTTTTGTATTTTTAGTAGAGACGGGGTTTCACCGTGTTAGCCAGGATGGTATAGATCTCCTGAC
CTCATGATCCACCTGCCTTGGCCTCCCAAAGTGCTAGGATTACAGGCGTGAGCCACCGCGCCCAGCCTTATTTGTCTGTT
CTTTAACAAATACCACACTGTCTTGATTACTGTAGTTTTAGAGTAAATCATGAAGTCAGGTAGTATCAGTTGTTTAACTT
TGCTCATCTCCTTCAATAATGTGTTTGCTTTCTGGGTCTTTTGCCTATCTATATAAACTTTAGAATCAGTTTGTTGATA
TTTATACAATAACTTGCTGAGATTATTATTATCATTGCATTTAATCTGTAGATTAAGCTGGGAAGAATTGATATCTTGGC
AATGTTGAGTCTTTCTCTCCATGAACATAAAATACTTCTCCATTTATTTAGTTCTTCATTGATTTCCTTCATCAGTTTGT
AGTTTTCCACATATAGATTTTGTATATATTTTGTTAGATTTATACCTAAGTATTTTATTTTTTGAATGATAATGTATATG
GCACTGTGTTCTCAATTAAAAGTCCATTTGTTTATTTCTGGCATATAGAAAAGTGATTGACTTTTGTGTATTAGCCATGC
ATTCTGCAACCTTACATATCAGTAACCTTGCATGTCAGTTCCCAAAATTGTCAATTATTTTGGATTTTCTACATAGATGA
TTATGTCATCTGCAGAGAAATACAGTTTTATTTCTTACTTTCTCATCTGTATACCTTTTATTTCCTTTATTTCCTTTTCT
TGTCTTACTGCATTATCTGGTACTTCCAGAACAATGTTGTAAAGTATTGGCGAGAGGGACATCTTTGCCTTGTCCTTGAT
CTTAGTGGGAAAACTTCAAGTTTCTCACCATACTTGAGGATGTTAGCTGTAGGTTATTGTAGATATTCTTTATTAAGGTG
AAGAAGTTCTTCCCTATTTCTAGTTTACTGGGAGTTTTTGTCACAAATCTGTGTTGAATTTTGTTTACTGAAAGTTATCA
CAAATGGGCGTTGAATTTTGTTAATTTTTTTGTATATATTGATATAATCAAGTGATTTTTTTTAGCTTGTTAATGTTATG
GATAACATTAATTGATTTTTGAATGTTGAACCTGCCTTAAATAGCTGAAATAAATTTAACTTCATCACAGTATATAATTC
TTTTTATACATCATTGGATTGGATTTGCTGATGTTTTGTTAAGGTTTTGCATCTATGTTCATGAGGTATATTGGTCCATA
GTTTTCTTTTCTTGTGATGTCTTAATCTGATTTTCCTATTAGGATAAAGCTGGCCTCATAGAATGAGTTAAGAAGTATTC
TCTCGGCTCTAACTTCTAAAACAAATTATAGACAATTGGTATAATTTTTTCCTTAAATATTTGGTTGAATTCACCAGTGA
GTCCATCTTGACATGGTGCTTTCTGTTTTGGAAGGTCAACTTCTGTAATAAATGTAAACATATTTAGGTCATCTATTTCT
CCATGTATGAGTTTAGACAAATTATGTCTCTCAAGTGATTGATCCTCTGTGTGGACATAGAACTGTCCATTTTAGCATCC
ATGGGATCTGTAGTAACAACTCCTTATTTATTTCTGATATAGATAATTTGTGTCCTTCCTCTTTTCTTCTTAATTAGCCT
GGCTAGAGCCTTATCCATTTTTTTTTTTTTAATCTTTCAAACCTAGTTTTTGGTTTGATAACTTTTTTCTATCAGTGTC
CCATTTTTTATTTCTGCTCTAATTATTTCTTTTCTTCTGTTTAATTTGTACCTGATTTGCTCTTCTATTTCTAGTTTCCT
AAGGTGTAAACTTAGATCATTAATTTTAGATCTTTATTTTTTTCTAATAGATGCATTCAATACTATCAATTTCTCTTTAA
ACAATGCTTTTACTGCATCCCACAAATTTTGTTAAGCTGTATTTTCATTTTCATTTAGTTGAAGTATTTTAAAACTTCTA
TTGTGATTTCTTCCTTGACCCATATGTTGTTTAGAAACGTATTGTTTAATCTCCACATGTTTGTGGAGATTAGTGGGATT
TTAAAATTTTCTTTCTGTTATTGACTCTAGTTTATTTACATTGTGGTCTGAGAGCAGACGCTGTATGATTTCTATTTTGT
TAAATTTGTTAGGATATGTTTTGGCCCAGAAGGTGGTCTATTATGGTGATTATTCCATGTAATCTTGAGAGTGTGTATTC
TGCTGTTGTTTGACAAAGTAGTCTATAGATGTCAATTATATTCGGTTAATTGATGATGTTGCTGAGTTCACCTAAGTTCT
ACTGATTTTCTGCCTGCTGGATCTGGGCATTTCTTTCTTTCTTTTTTTTTTTTTTTTTTTTATTTGTTGTTTTTGAGAC
AGAGTCTCGCTCTGTCACCCAGACTGGAGTGCAGTAATGCAATCTCATCTCATTTCAACCACTGCTTCCCGAGTTCAAGC
AATCCTCCCACCTCAGTCTCCTGAGTAGCTGGGAATACCGGCAAATACCACCACACTTGGCTCATTTTTGTCTTTTTGTA
GAGACAGATTTTACCACATTGCTCAGGTTGGTCTTGAACTCCTGAGATCAAATAATCCAGCCTTTTGGGCCTCCCAAAGT
GCTGAGATTACAGTCATGAGCCAATATACCCTGCTGGATCTGTGCATTTCCAATAGAGTGTTGTTGAAATCTCCAACTAT
GATAATGAATTCATCGATTTCTCCTTGCAGTTCTGTTAATTTTTGCCTCACGTAGTTAAATACTCTCTTGTTAGGTGCGT
GCACATTTAGGTTTGTTATGCGGAAACATAACATAGTTTCTTGGAAAGCTAATTCTTATACCATTATGCAATGCTTTTCT
TCATTCCTGATAAATTTCCTTGCTTTGAGGTTTGTTCTGTTTGAAATTTGTATAGCTTCTCCTGCTTTCTTTTGTTTGTG
CCAGTATGATATAATACATATATATATTCATGATCCATTTACTTTTAATTTGTGTGTCTTTATATTTAAAGTGCATTTCC
TGCAGACATCATATAGTTTAATCTCTGATTTTTAATTGGTGCATTTAGATCATTGATATTAATGATTATTAATATAGGTG
GATTAAATATCTACCATATTTCTTTCTGTTTTCTATTGTTGCCTTTGTTCTTTGTTTCTATTTTTGTTTTTCATTCTCT
TCTTTTCAGAGACTGAGTCTCATTCTGTTCATTAGGCTAGAGTGTGGTGGTATAATCATAGCTCCCTGTACCCTTGAAAT
CCTAGGCTCAAGTGATTCTCCTACCTGAGCCCTGAGTACCTGGTATTACGGGCATGTGCCACAAAGCCTGGCTAATTTTT
ATTTAATTTTTGTAGCGACAGAGTCTTTGCTACGTTGCCCAGGCTGGTCTTGAACTTCTGGCCTCAAATGATCCTCCTGC
CTCAGTGTCTCAAAGGGTTGGGATTATAAGTGTAAGCTACAACACCCAGCCATGTCTTCCATTTTTTCCTGCATTTTTA
GTTTTTAACTTAGCATTTATAAAGTTCCATATTTTCTCATTCTTAGTATATCAATTATACTTTAAAAATGTGATTTTAGT
GGTTACCCTAGTGTTTGCAATATACACTCACAACTAACATAAGTTTGCTTTCAAATAATACTATACCATTTCACAGGTAC
TGTGAGTACCTTATAATAAAAAATCTAATTCTCTGCTTTTATCCCATGTGTCATTTCTGTTATTTATTTTATTTACATG
TAAGAATACATAAGCATATAAATATATATAAAAGATGTATACATAAGCACACATAATTAAATATATTGCTACTATTGTTA
TTTTGAACAAACTGCCATTTGTTAGATCAATTAAGAATAAGAAAAATAGTTTTATTTTACCTTCACTTATTTTTTATATT
GTTCCTTTTTCATATAATTTAGGTTTCTAACCTGTATAATTCTCTTTCCTTTTAAAATAATTGCATTTAACTTTTTTGTT
TTTTGATATAAGACAAGTCTACTGGCAACAAGTTATCTCAATGTTTCTCCAAAATAGTGGTTGTTTCTCATTCACTTTGA
AGAATTTTTTGGCTAAAGAATTCTAAGTTGATAAGGTTTTTTCTCTTAATACTTTAAATATTTGTTTCCACTGTCTTCT
TGCTTGCATGGTTTTTGAGAAGAATGTAAATGTCATTTTTATCTTTGTTTCTCTACATGTAGAATTTATTTTCCTCTGGA
```

```
TTCTCTTATGATTTCTTAAATCTTTGATAAAGGGGGTACAGGGGCATATGTGAACTCTATATTTTTAATTCAGTATTTCT
GTCAACTTAAACTGATAAAAACTAAAATCTATTGATTAAGACAAATATCTTGGGAAACATCAATGTAAAGCTTGCTGATC
TCTCTAGTGTTGGGGGGAATGTTGGAGAAAAATATGCCTATTTAGCTAAGGACAAACCTAGATAAATTAACTAGAGGTGT
GAATTGTATGCAAGAGATTGTGTCAGAACAGGGTTCCAATAATGAACTTCATAAGAGGTTTTAGATAACCTAAGACATTT
TGAAATATAACACAACTACAGGAAATTATACAATACAAATATGTAGCTTGATTAATTATTATAAGATAAACACCTTTGTT
GACTCCTGATATGGCCTAGCTGTGTCTTTACCCAAATCTCATCTTGAATTGTAGTTCTCATAATCCCCACTTGTCATGGG
AGGGACCCAGTGGGAGATAACTGAATCATGGAGTTGGTTACCCTCGTGCTGTTCTTGTGATAGCGAGTGAGTTTTCATGA
TATCTGATGGTTTTATAAGGGGCTTTTACCCCTTTGTTAGGCACTTCTGTCTCCTGCTGCCATGTGAAGAAGAACACGTT
TGCTTCCTCTTCCACCATTATTGTAAGTTTCCTGAGGCCTCCCCAGCCATGTAGAATTGTGAGTCAGTTAAACTTCTTTC
CTTTTTAAATTATCCAGTCTTGGGCAGTTCTTCATAGCAGTGAGGATGGACTAATACAACTGCTAACATTGTCTAAGAAA
GATCCTTGTTATGTGGCTCAGAGGCCCTCCATGTATTCTTAACTATTCACTCTCCTATCCTTCCAAGAATAACACTAATT
TGACCTCTACAATAATCACTTTATCACTCCAGTTTTGCCTTTTTTCCTCCAAAACAATGCCTTTTAAGTCTATTTTAATC
GATAGATTTCCTCTTAATATCATTTAAAAATATTTCTTTACATTTTTAGACAGGAATCAGAATAATTTGCTATGTTGAAT
TTCCAGTTACTTGGATTTTGTTGATTTCATTCCTGTGGTTTAGTTGACATGAATCTCTCCAATTGAAAGGGTAACTTGAA
TATGGTAGCTGGAAAGTTAAAATCAATTCTTTTAACTTTGGAAAATGATTAAATTCTGGAGATAGAATAGTAAGTTCATA
AGATGACAGGTCATTTGCATCCTCTAGTGAAAAGCGAAGGAATTAAATAAAAATAACATTTTGATGCTTAATGTTTCTGG
CAGTATTATGTCTGTATTTAATTGTTAAAATGTTTTTCAATAATTTTTTCCAGGTTGTCTGCATTCAAAAGAGCATTCTA
TTAAAGCTACCTTAATTTGGCGCTTATTTTTCTTAATCATGTTTCTGACAATCATAGTGTGTGGAATGGTTGCTGCTTTA
AGCGGTAAGTGAACTGTAATCTTTATTCATCTAGAGAGAATTATACTTGTAAAAAGAAGAGGAAGAATCTGAATAATTTT
GTTATTCTTTACAGCTTTTAATATATTGTCTCACTAACTGAGTTACACTCCAATAACTTTTTAAGTGGCACCAAAAATTT
TAATTATGTAAATAGGGTTCACATTATTCCAAGTTTAGTGAGATGATCAGAGTCTTCAGAAGGTAGAAGACAGACCACTA
TTCCTTAAGAATATTGATGCAAAAATTAAAAAAAAATAGTAAACTGAAAAATAGTCTCTTGTCCGATCTAGGAACATGGTT
AATTTTACCTCCACAACAACCTTGTTTCATGAAAAATAGCAAAACAGATGGTTTAAAGTACACAAAACTACTAAGTGGCT
GTATAACATCACAGGCAGCCTGTTCTTTCTTCCTAGCTATTTGAGCATGTTCTGGCCCTTGTTTTTTTAGCATATGTAG
CCCATCTTCCTAGAAAACAAACTCCGTTTAGTGATTGTACCCTAAAAGCAATTAGCTATAAAAATTATCTTGTACTTCAA
AACATGCTTGCAGTGCATCTAATATAGACCAAGCGAAGACATAGAAAACTACAAAAGCACTTCCCCCAAACCTCATGCCA
ATCTGGATATCAACCTAAAATCTACCCCCTGTTACTACATTCTGAAGTAATTTCAGAGAGACCCTTCAAATACTGCTTAC
GTAACTTAAGAGTCAGCAATACTTGGAGCACTCCATATAACACTCTAAATAATTTCTATTCATTTGCAAATCTTTTGCTT
ATTATCTTTTGGCATTTTGAAACCACAGATAATATACTTTATACATTTATGAAGCAAAATAATTTTTTATTTCAGAGA
TTTAAGTCAACTAATTGTAACTATTTAAGAAAGAAAAATTGGAAAGAGACAATTTCTGTACTCTGTAAGTACCTCAGTA
TTAATCAAGGGAATGTATTCTGAAATATAATTTAAATTTATAGCTCTTTTTTTTACAGTAAGCATATGAAATTGACACTT
ATGACTGTTTTCCTTTATTATCCATTATGTGTTTCTCTTCCTTTCAGCTAGCACTTTGGAAATGATTTCTTGTGTGGAGC
CAATAGCTGGAGCCATCTTCTAATCTGGTCATTTTCTTTCTTTCTTTTTTTAAATGTGTTATTATGTATTACTTTATATC
ATTTTTATGTTTTATTTAACATTTAGATTGTTAGTTAACTATTCAAGTGTGGTATTTCCCCATTTAAATCGAGTTATTTA
GATTGGCTTTAGTTATAAAAATTCCTAATAGAGCTTTGTAGTTATAGCTCTGGTCCCTAACTTTTTTTTTAAATTATACT
TTAAGTTCTGGGATAAATGTGCAGAACCCGCAGGTTCGTTACATAGGTATACACGTGCCATGGTGGTTTGATGCACCTAT
CAACCCATCATCTATATTAGGTATTTCTCCTAATGCTATCCCTCCCGTTGCCCCCAACCCTCAACACGCCTCAGTGTGT
GATGTTCCGCTCCCTGCATCCATGTGTTCTCATTGTTCAGCTCCCACATATGAGTGAGAACCTGCAGTATTTGGTTTTCT
GTTCCTGTGTTAGTTTGATGAGAATGATGGTTTCCAGTTTCATTTGTCTCCCTGCAAAGAATATGAACTCATTCTTCTTT
ATGGCTGTATAGTATTCCATGGTGTATATGTGCCACATTTTCTTTATCCAGTCTATCATTGATGAGCATTTGGGTTGGTT
CCAAGTCTTTGCTATTGTGAATAGTGCCACAATAAACATACGAGTGCATGTGTCTTTATAGTAGAATGATTTATAATCCT
TTGGGTATAGACCCAGTATTTCTGGTTATAGATCCTTGAGGAATTGCCACACTGTATTTTCCAAAATGGTTGAACTAATT
TACACTCCCACCAACAGTGTAAAAGTGTTCCTATTTCTCTACATCCTCTTCAGCGTCTGTTGTTTCCTGACTTTTTAATG
ATTGTCATTGTAACTGGCATGAGATGGTATCTCATTGTTGTTTTGATTTGAATTTCTCTAATGATCAGTTATGATGAGCT
TTTTATCATATATTTGTTGGCCACATAAATGTCTTCTTTTGTGAAGTGTCTGTTCATATCTTTTGCCCATTTTTTGATAG
GGTTTTTTGTTTTATTCTTGTAAATTTTTTTAAGTTCCTTGTAGATTCTAGATATTAGCTGTTTGTCAAACGGATAGATT
GCAAAATTTGTCTCCCATTCTGTAGGTTGCCTGTTCACTCTGATGATAGTTTGTTTTTTTGTTTGTTTGTTTTTTTTT
TTTTTGTGCAGAAGCTCTTTAGTTAAATTAGATCCCGCTTGTCAATTTTTGCTTTTGTTGCAATTGCTTTTGGTGTTTTA
ATCATAAAACCTTTGCCCATGCCTGTGTCCTGAATGGTATTGCCTAGGTTTTGTTCTCAGGTTTTTATGGTTTTAGGTCT
TATGTTTAAGTCTTTAATCCATCTTGAGCTAATTTTTGTGTAAGGTGTAAGGAAGGGGTCCAGCTTCAGTTTTCTGCATA
TGGCTAGCCAGTTTTCTCAACACCATTTATTAAATAGGGAATCCTTTCCCCCATTGCTTGTTTTTGTCAGGTTTGTCAAA
GATCAGATGGTTGTAGATGTTGGGTGTTATTTCTGAGGCCTCTGTTCTGTCCATTGGTCAATATATCTGTTTTGGTACCA
ACACCATGCTGTTTTGGTTACTGTAGCCTTATAGTTTAATTTGAAGTCAGGTAGCATGATGCCTCCAGCTTAGTTCTTTT
TGCTTAGGATTGTCTTGGCTATACAGGCTCTTTTTTGGTTCCATATGAAATTTAAAGTTTTTTTTTTTTTAATTCTGTG
AAGAAAGTCAATGGTAGCTTGATGGGAATAGCATTCAATCTATAAATTACTTTGAGCTATATGACTATTTTCATAATATT
GATTCTTCCTATCTATGAGCATGGAATGATTTTCCATTTGTTTGTGTCATCTCTTATTTCCTTGAGCAGTGATTTGTAGT
TCTCCTTGAAGAGGTCCTTCACATCTCTTGTAAGTTGTATTCTTAGGTATTTTATTCTCTTAGTAGCAATTGTGAATGGG
AGTTCACTCATGATTTGGCTCTCTGTTTGTCTGTTATTGGTGTATAGGAATGCTTGTGATTTTGCACATTGATTTCGTA
TCCTGAGAATTTGCTGAAGTTGCTTATCAGCTTAATAAGTTTTAAGGGGATGAGACAATGGGGTTTTCTAAATATACAAT
TTGCCCTGACTAGAACTTCCAATACTATATTGAATAGGAGTGGTGAGAGAGGGCATCCTTGCCTTGTGCCAGTTTACAAA
```

```
GGGAATGCTTCCAGCTTTTGGCCACTCAGTATGATATTCGCTATGAGTTTGTCATAAATAGCTTTTATTATTTTAATGTA
TGTTCCATCAATACCTAGTCTATTGTGTGTTTTTATCATGAAGGGGTGTTCAATTTTATCAAAGGCCTTTTCTGCATCTA
TTGAGATCATCATGTGGTTTTTGTCATTGGTTCTGCTTATGTAATGGATTACATTTATTAATTTACATATATTGAACCAG
ACTTTCATCCCAGGGATGAAACTGACTTGATCGTGGTGGATAAGCTTTTGATGTGCTGCTGGATTCGGCTGGCCAGTATT
TTATTGAGGATTTTTGCATTGATGTTCATCAGGAATATTGGCCTGAAATTTTCTTTTTTGTTGTATCTCTGCTAGGTTTT
GGTATCAGGATGATGCTGGTCTCATATAATGAGTTAGGGAGAATTCCTTCTTTTTCTATTGTTTGGAATAATTTCAGAAG
GAATGGTACCAGCTCCTCTTCGTACCTCTGGTAGAATTCGGCAGTGCATTTGTCTGGACATGGGCTTGTTTTGGTTGGGT
AGGCTATTAATTACTGCCTCAGTTTCAGAACCTGTTATTGGTCTATTCAGGAATCTGATTTCTTCCTGGTTTAGTCTTGG
GAGGGTGTATGTGTCCAGGAATTTATCCATTTCTTCCTTGCCTGGGTATCACCAGCAAAGGCTGAAGAAAAGCAAAGATT
GCTGCCTGCTCCTTCCTCTGGAAGCTTCATCCCAGAGGGGCACGCACCAGATGCCAGCTGAGCTGTCCTGTATGAGGTGC
CTATCAACCCCTGCTAGGAGTTGTCTCCCAGTCAGGAGGCATGGGGGTCAGGGACCCACTTGAAAAGGCAGTCTTTCCCT
CAGAAGAGCTCGAGCACTGTGCTGGGAGATCCACTGCTCTTTTCAGAGCTGGCTGGCAGGAATGTTTAAGTCTCCTGAAG
CCGTGACCACAGCCACCCTTTCCCCCAGGTGCTCTGTCCCAGGGAGATAAGAGTTTTATCTATAAGCCCCTGACTGGGGC
TGCTGCCTTTCTTTCAGAGATGCCCTGCCCAGAGAGGAGGAATCTAGAGAGGCAGTCCGGCTGCAGTGGCTTTGCTGCAC
TGGCTTTGCTGCACTGTGGTGGGCTCCGCCCAGTCCGAACTTCCCCCAGGGCTTTGTTTACACTGTGAGGGGAAAATCAC
CTACTCAAGCCTCAGTAATGGCGGATGCACCTCCCCTCACCAAGCTTGAGCATCTGGGGTCCACTTCAGACTGCTGTGCT
GGCAGCAAGAATTTCCAGCCAGTGGGTCTTAGCTTGCTGGGCTCTGTGGGGATTGGACCCACTGAGCAAGACCACTTGGC
TCCCTGGCTTCAGCCCCCTTTCCAGCAGAGTGAATGATTCTGTCTCAGTGGGTTCCAGGCTCCACTGGGGTATGAAAAAA
ACTCCTGCAGTTATCTTGGTGACTGCCCAAATCGCCACCCAGTTTTGTGCTTGAAACCCAGGGTTCTGGTAGTGTTGGCA
CTCCAGAGAATCTCCTGGTCTGTGGGTTGCAAAAACCGTGGGAAAAGCGTAGTATCTGGGCCAGATAGCACCTCACAGCA
CAGTCCCTCACAACTTCCCTTGGCTAGGGGAGGGAGTTCTCCCACCCCTTGTGCTTCCTGGGTGAAGCAGCGCCCCACCC
TGCTTCTGCTTGCCCTCTGTGGGCTGCACCCACTTGTGTAACCAGTCCCAGTGAGATGATCCTGGTACCTCAGTTGGAAA
TGCAGAAATCACCTGCCTTCTGCATTGGTCTCACTGGGAACTGCAGACCAGAGCTGTTTCTAATCAGCCATCTTGCCCTC
TCTGGTCTGGTCGTTTTCTTTAAATTGGTTGATACAGGAGCAGTGATAGCACAACAAATGCACAGATTTGGGGAAAGT
CATCCTGCCTTATGGTCTGGTTCAAGAAATTACATTTTAATAGTTATAATTTGGTATCACCTTGTTTGTGATACCAAACC
AGATACAATACACGTTTGCCTCATGTTATGATTTGTTATTCAGATTACACCAGTTATTATTCATAACTAAGAGTGATTTC
TCATCTCACAAGAGCCAAATCCAAGGATAATGGTGCCAATTGATAGTAATGATTCTATGAATACCCAGCATTCTGGTCTA
TCATAGACACTTTCAGAACCATTGAGTTGAAGGTAGAAGGTGGTTATATAATAGAAGATGAACTGGTAGCTACTAGGGGC
TCAGTGACAACTATCTGGAGAGACATTCATTCATCTCGATCCCACATGAAGAGAGCATTTCTCCTGATTATATAAGAAGT
GGGTCAGAAAAGCCTGTCCAGTGAAGTATTGCTGCCTTCAAAGTGTAGAAAACCTCACTACATCTCCTTAGTGGAAGGAA
GTTCACTGTACAACAACTTATTTCATATTTATGATAGTATTTAGACATATACAAGGCTTTTTCACATCAAGAAACCTTAT
TCACATAAGGCATCTCTATCCTGCCCTTCATTTTACCAAGTCATCTGGAGCAGCAATCGCCAACCTTGTTGGCATGAGGG
ACCAGTTTTGTGAAAGACAACGTTTTCATGGACTGGGGTCAAGGAATGGTTTGGGGATAATTTAAGTGCATTACTTTTAT
TGTGCCCTTTATTTCCATTATTATTACATTGTGTAATAATATATAATAAAATAATTATACAACTCACCATAATGTAGAAT
CAGTGGGAACCCTGAGCTAGTTTTTCTGAAAGTAGATGGTACCATCTGTGAGTGATGGGAGACAGTGACAGTTCATCAGG
TATTAGATTCTCACAAGGAGCCCACAACCTAGATTCCTCACATGAGAAGTTCCCAATAGGGTTTGCCCTCCTATGAGAAT
CTAATGCCACTGCTGATCTGACAGGAGGTGGAGCTCATGAGGTAATGTGAGTGATGGGGAGTGGCTGTAAATACAGATGA
AGCTTCACTTACTCATTCGCTGCTTACCTCCTGCTGTGCAGCCTGCTTCCTGACTCATCCATGGACCAGTACTGATCCAT
GGCCTAGGGGTTGGGGACCCCTAATCTAGAGCACTTGGAGAACTATCTGTTCTCCAAAGCTGATCAAATGCTATCATTAA
TGTATCTAATATTTTAAGAAAGGGTAACACTGTTGAGAGCCAAATAGATACATGGCCCAGAGCAAGCTTAAGTTACTAAT
AACTCCTTTTTCAGCTCACCCCCTGCTGAAGGCATGAGTTTGAATCTCAGTTTTGCCATTTGCTGTGTAATGTATGCAAT
TATATTTAGCATCATATTTCTCACTTGAAAAATGAAAATAATACATTTAATACTTAACAGGAGTGTCAGAAAGTATATTA
GCACTTGGTAATTTATACAATACAATATAAAAGTAAGAAATTTTTATTTTATTTATTTTTATTTTATTTTCAGCAATAAG
AGCTAACTGCCATCAAGAGCCATCAGTATGTCTTCAAGCTGCATGCCCAGAAAGCTGGATTGGTTTTCAAAGAAAGTGTT
TCTATTTTTCTGATGACACCAAGAACTGGACATCAAGTCAGAGGTTTTGTGACTCACAAGATGCTGATCTTGCTCAGGTT
GAAAGCTTCCAGGAACTGGTAAGAAAATAGTTCTGGCCAGAATCAAAGATTCAGCCCTACAAGGATATGTTTTCCTGTGA
AATTATCTAAGAGGTAGGTTTAGACATCTGCTTTTACATTGATTTTTTTTTTTTTTTTTTTGCATAACGAAAGAGTAAC
CTAGCATGTATTATATTTTACAGTGAACCATCTAAAATTACCTTAATATTCGTGGCAGGAACAGGCCCAGAGGGCAAGCA
AGCCAGAGCCTTCTTTGACTTGTGAGCCAGAATTGTGCAAATAAGGATTAGAAAAGTATTGGTAGAAACCCAGTTTTAAG
TTTGTATGAAGTTAGCAACATTGTTTCAAAATAAATCAAACAAGGCCAAGAGCAGTGGCACATGCCTGTAATCCCAGCAC
TTTGGGAGGCCAAGGCGGGTGTATCACTTGAGGTCAGGAGTTTGAGATCAGCTTGGCCAACATGGTGAAACCCCATCTCA
ACTAAAAAATACAAAAATTAGCTGGGCATGGTGGCATACGCCTGTAGTCCAGCTACTCAGGAGGCTGAGGCAGCAGAAT
TGCTTGAACCTGGGAGGTGGAGGCCTACAGTTAGCTGAAATCATGCTACTGTACTCCAGCCTAACAGAGTGAGACTCTAT
CTCAAAAAAATAATAAAATAAAAACAATAAGTCAAGCAAGAATGATGTCATAGAGATTGGTAGACTAAAAAGCTACAGAA
ATCTGTTCCTCCACTGAGAAAACTATTGAACTGTCAAAAACTGTCTGAAGTAACTATTTTGGAATTCTCGAGTCTAGTTA
AACACTGGAAGCATCAAGGGAAGAGTTTGATAAAGAGGATGATAAATTTTGGTTAATGTTGGTGAATTTCAGCCTTTCCA
CTCAATAATAACTATTTTCCATACCCCATTATTGCAGGGATCCATGGGAACTGCTGCCCATGTTCTTGTAATGAATTCCT
GCAGCCAGGGTGAACAATAAGCACCTTTTTGTCCAAATATCAGGGTTATTGCTGATTCTGCCTTTGAATGCTGAGGGGC
AGACACAGAAGTGGGCTATCATTACATCAGTCCTCATCAGCTGAAGTGGCTTCCCAAGGATTTAAATAAATAGTATGTGT
TTTTCCTCCCTTTAGGAAGCAGTCATTTAAGACAATTTTTATTAGATAACTGGCTGACAGCAGAGATAACAGAACAGAGA
TTTCAATGACCATGCACAACAGAGAATAAAAATAGTTGGGAAAAAATCATGACCAAATGACTCTGAGCCACAACAACCAA
```

```
GATTTGACAATCCCTGAAGAGCAAAATAATTAAGTTACCAGAGTTACCACAACATGGTACTCATAATGTCCAGTTCTCAA
AAAAAAATTACAAAACATGCAAAGAAAAGTATGGTTCATTCACAGGAAGAAAAAGTAATCTGACAGAAACTATCCCTGAA
GAGGCTCAGATATTAAAAATATGAGTCAAAAATGTTAAATCAGCTGTCTTAAGTATAACCAATGAGTTAAAGGAAACTAG
ACAAAAAGCTAAAGGAAACCGAAAACATAATAAATGAACAAAATTAGAATATCAATATAAAGGTAGAAATTGTAAAAAAG
AACCAAGCAAAAATTCCAGAGCTGAAAAGTACAATGACTGAAATTTAAAAATAATTTTAAAAACTCAATGAAGAAGTTCA
ACAGCAGATTTGAGAAGTAAGAGATCAGAAAACTTGAAAATAAGATAATTGAAACAATCCAGACTAAGAAAAACAAAGAA
AAAGAATGAAGATAAATAAATTCTAAGGAACCTGTAGGACATCAGCAAACATACTAACATATGTACTGTAGAAATCCAGG
AAAGAGAAGAGAAAGAGAAGCAGAGAAATACACTTAAAGAAATAATGAACAAAACTTTCCAAAATCTGAGGAAATACATA
AATATATACATCCAAGAGGCTCAATGAACTCCAAAAGGGTAAACTTAAAGAGATCTACATTGAGACAAAATATAGTCAAG
TTGACAAAATCCACAGAGAGAATTTTGAAAGCAGCCAGAATGAAGCAACTCATCATTTACATAAGACCCTGAATAAAATT
AATAGCTGATTTTCTCTGAGAAACCATGGAGATCAGAAGGTAGTGGAATGGCATATTTAAATGTCTGAAAGAAAAAATAA
AACTGCCAACCATGAATTCTATGTATAGCAAAGTTGTCCTTCAAGAATGAAGGAAAAAGTAACACATTTTCAGATAACCA
ATAATTAAGGGATTTTATTACCAGTAGACATGTGCTACAGAAAATGCTAAAGGAAACCTTTTAGGCTGAACTGAAAGTAC
ACTAGACAGCAATTCAGAGCCTCCAAAATAAAGAATATTCATAAAAGTAACAATAGAGGTAAATATAAAACCCAGAATTA
CTACATGTGTCATATAGTTTATAACTTCTCCTATTTATAGCTTTCTATATTTATATTTATCTATAACTTCATAGGCAAAT
GAATAAAAATTATAAATATGATAGTGGTCATATAATGTATAAAGATGCAATCTGTGACAGTCTTATGAAGCAGGGATGAA
GACATATAGGATCAAAATGTTTGCATAGTTATTGAAGCTATGTTGATATTATGAAATTATATTGTTACAAGTTTAAGATG
CTAATTATAATTCTCAAGGTAACCACTAATAAAATTACCAAAATTATGCAGAAAAGGAAAAAAGAAAAACAATACACTAT
AAAAAAACCAATTAAATACAAAAAATGTCAGTAACAGACAACTTGAGAAACAAAGACATATAAGATATAGAGAAACAAAT
GATTAAATGGCAAAAGTAAATCTTGTTTTAGTAATCACATTAAATAGAAAAGGATGAAGCCATCCTATTAAAGGGCTGAG
ACTGACAAGTTGGCTAAAAACTAAATAAATTAAAAAGAAAACAAGACTCATCTACATGCTGTCTATAAGAGACTTGCC
TTAGATATAAGGACACAAAGAAGTTGAAAGTAAAAGGACTGAAAAGATATTCCATACAAACAGTAGTAACCAAGATAGT
GCCGAGTGGCTATATTTTTGTCAAACAAAATAAACTAAAGTAAAATTTACAAGAGAAAAGAAGGGCATTATGCATTGAC
AAAAATTTTGACATAGCCAAATAATTATCTGTTATAAAATATATGTACTTAATAATACAGCCTCAAAATATATGAAGCAA
TAATTGCTATAATTTAAGGGAGAAAAGAACAGTTCTATGAAAAGTTAGAGAATGAAATATTCCACTTTCAACATGAGATT
AAACAACTAGACATAAGATCAATAAGGAAATAGAAAATTTGAACAACACTATAAACCAATTATCCCTAACAGGCATATAC
AGGCCACAAAACAAGTGTTAGTAAGTGTGAAAATTTGAAGTCATAAAAAGTATCTTTTGCAATTACAATGGAATGAAGCT
AGAAATCAATAACTAGAAAAACCAGAAAAGTCACGCATATGTAGAAATTTAAAAACCTGCTCTTCAACAGCCATTGGTCA
AAGAAGAAATCACAAGGGACATTAGAAAATACCTTGAGACAAATGAAGTAAAAATACAAATAGCACGTTTATGGTATACA
CTGAACATAGTTCTAAGAGGGAAATTTATAGCTGTGAGCAGTTAACTAAAAAAGAAGAAAGATCTCAAATCCATAGCCTA
ACTGTACACTGTAAGGAACTAAAAAAAGTAAAACAAAAATAGAAGTCATCTTTATGATTTGAAAGAGTAAAAGATTTACC
TAATAAGTCCCTAAATTTACTAATAATAAAGAAAATTGTTTATATATTTAATTGCGTTAAAATTCAGAACTTGTAATCAT
GAAAAGGACAGTACACATTGACAAGGAAACACAGCAAAGGAAACCAGCCTATGCTGCTGCTGTTGTGAGGATAATTTGGT
ACACTTACATTAGTTTGGTGTCTTTTCTTTCTCTCTCTTTCTTTCTTTCTCTTTCTTTCGTTCGTTCGTTCATTCG
TTTCTTTTTGAGACAGAATCTCACTCTATTGCCCAGGCTGGAGTGCAGTGGCGTGATCTTGGCTCACTACAACTTTTGTC
TCCCAGGTTCAAATGATTCTCATGCCTCAGCCTCCCAAATAGCTGGGATTATAGGTGCATGCCATCACGCCCAGCTAATT
TTTGTATTTTTTTTAATAGAGAGGGGGCTTCATCATGTTGGCCAAGCCTAGTCTCAAACTCTTGGCCTCAGGTGATCCGC
CTGCCTCGGCCTCCCAAAGTACTGGGATTACAGGTGCCTGGCCTGGTGGTGTCATTTCTTAAAGTTGACAAAAAGCATAT
CCTGGGGCCTAAAAATTCTATTCTAGGACAGGTGCCAAGAATGTCATAGTAGCATACATTCCAAACTTGATAAAACCCTG
GTGTCAACCGATAGTATAATAGATAAATTGGAGAAGAGTCATACAAAGGAGTACAATACAGAAACAAAAGTAACCAAATT
ATCAACAATTTCTCTCAGTTTTAAATTATCTTCTTTTGATATGTATGATAATATAGCACACCTATTCTGTATGTATTACT
AAACAATACAAAATCAAAAGGAAGAAAATTATGAGTAGTTAAGAATATAGCATAGCAGCAACATTTCTGGGAGAGGATGG
GTTATGTTAGATTAATGAATATCATCTCTGTGTTTTCTGAAAGAATTTCCTGTTGAGATATAAAGGCCCATCTGATCACT
GGATTGGGCTGAGCAGAGAACAAGGCCAACCATGGAAATGGAAATGGTACTGAATGGACAAGACAGTAAGTTCTAAAA
ATCTGGCAGTAATATTTGTATTTGAATTTACTTTGCATTAAATCTGAAGTGTTCTCTAGTTACATGCTTTAAAAAATTCT
CATTTTAAGGTTAGTCATGAAAGAAGATGGTGCCAACTTGTATGTTGCAAAGGTTTCACAAGTTCCTCGAATGAATCCAA
GACCTGTCATGGTGAGGTAGACTGACTGTGAACTTGGCTCCAGGCTTATCTATGTCATTTTCAAACACTTTCATTTTAAG
CAAACCATACAATATCTTTAAGTCTGTTCCTTACCTCCACAACAAAATTAAATTGCACTTGTCCTCCTGATTTCACAGGG
TTGATGTGAGGAACAGAGGTTTTGATGTATCAGGGAAAGATTATGAGTGACAGCAATTATACCTATTATTTAAAATAAGA
CAATAGTTTTAAAATTTTAAAATGGGTAAAGTTTGGCACTAGAAAATTTAATCTCAATTGTATATTTATAGGATCTTCAG
ATTACTAAAAGATTTGAGATAATGCTGGAAAAATTGGATTCACACAATTTCACTCAATGTTTGTCTGAGAGATGAGACA
GTTTTGAAAAGCTACTTTATTGTAATACATTCATCAATATTGGAAATATAACTTTATTTAATAAAAAGAGCCCCAGACTG
GACATTGGCAGGTTTGAAATGAGTTTTTTCTCATTAGCTTTTGACCTTGGATGGGATGGTAAGTTTTAGAAATCAGAGAA
CATGTACATTTATACATTGTTGTATCTACACTGCCTTGCACATTGTGACTGCTTCATAAATATCTAGAATTAACTTTTAT
TTCTTATTTTACAATACGGAAGTAGTAAATTTTCTCTACCTAATTCTTAAAATGGTTTTTTGTTTGTTTGTATTTTTGAG
AGACAGGGTCTTACTCTGTTACCCAGGCTGGAGTGCAGTAGTACCATCGTGGCTCACTGCAGCCTTGACTTCCCTGGCTC
AAGTGAGCCTCCCATCTCAGCCTCCTGAGTAGCTGGGACTACAGGTGTGTGCCACCTTGCTTGGCTTTTTTTTTTTTTTT
TTTTTTTCAGCGATGGGGTCTCACTATGTTGCCTGGGCTGATCTTGAACTCCTGAGCTCAAGCAATCCTCCCACCTCGG
CCTCCCAAAATGTTGGAATTACAGGTGTGAGCCACCATGCCTGGCCTCTCAAAATATTTTAAGGATCAAATATATTATTA
ACTAACCAGTTTTTGGAAACTGCTCATCACTTAAAGAAATGTAAAATATTATATGATTAAGGTCTAACAAGTTTCAACAA
```

```
TTAGCAAATTATATCATAGATGATAGTGATTCCAATGAGCAAAGAGGAAAAATTTATAATCCAAATGCTGACCTAAAATA
TCTGTGCCAAGCCATCTAAACTCAGCTAAATAGCACTGCAGTTTCAGTACTAAAACCACCAGGGAAGTAGGAGGAATAAA
ATCAAGCATGGTTTTTTAGAAATAGCTGCTGAGTCTTCAGTTATTTAAGGAAGCAAAATATTGGGAAACTGTGTAAAGAAA
ACGTGTCAGACTTCTCCCATCAGCCAGCTAAGGCTTTGGATGTACTTGAAAGAATATTATGCTTACAGACATGAAATAGG
TTTGATTCAGGACTTTGCAGTATTCCTATAGTTGATTTATAACATCTCCTGCTAAGCAAAGCCCACTGACTAATTAGTCA
CCACTACACAAGGAAAAACAGCATTATTTTTAGAGGCTGAATTAATGTTAGTTTTCTCATTTTCTCATCTTCATTCTCTC
TGCTGTTGAAGAAATGTTCAGTGGCCAACTGATTCTGCTTCTTCTCTTGCAGGTTTCCTATCCTGGGAGCAGGAGAGTGT
GCCTATTTGAATGACAAAGGTGCCAGTAGTGCCAGGCACTACACAGAGAGGAAGTGGATTTGTTCCAAATCAGATATACA
TGTCTAGATGTTACAGCAAAGCCCCAACTAATCTTTAGAAGCATATTGGAACTGATAACTCCATTTTAAAATGAGCAAAG
AATTTATTTCTTATACCAACAGGTATATGAAAATATGCTCAATATCACTAATAACTGGGAAAATACAAATCAAATCATA
GTAAAATATTACCTGTTTTCATGGTGCTAATATTACCTGTTCTCCCACTGCTAATGACATACCCGAGACTGAGTAATTTA
TAAATAAAAGAGATTTAATTGACTCATAGTTCCACATGGCTGGGGAGGTCTTGCAATCATGACAGAAGGCAAATGGGAAG
CAAAGTCATGTCTTATGTGGTGGCAGGCAGGGGGACTTGTGCACAGGAACTCCTATTTATACAACCATCAGATATCTTGA
GACAAGAACAGTATGGGGCTCCCTGGTGTGATTCCGTCCTGCGCGGCTGTTCTCTGGAGCAGCATTCATTTATCTTCGTC
TGCCTTGTCTCCTACCTAAGTGTGTGTCGCCACCCGATGGAAGATTCGATGGACATGGACATGAGCCCCCTGAGGCCCCA
GAACTATCTTTTCAGTTGTGAACTAAAGGCCGACAAAGATGATCACTTTAAGGTGGATAATGATGAAAATGAGACCAGTT
ATCTTTAAGAACGGTCAGCTCAGGGGCTGGTGCAAAGGATGAACTGCACATTGTTGAAGCAGAGGCCATGAATGACGAAG
GCAGTCCAATTAAAGTAACACTGGCAACTTTGAAAATGTCTGTACAGCCAACGGTTTCTCTTGGGGGCTTTGAAATAACA
CCACCAGTGGACTTAAGGTTGAAGTGTGGTTCAGGGCCAGTGCATATTAGTGGACAGCACTTAGTAGCTGTGAAGGAAGG
TGCAGAGTCAGAAGATGAAGAAGAGGAGGATGTGAAACTCTTAAGTATATCTGGAAAGCAGTCTGCCCCTGGAGGTGGGC
AGAAAAAAGTAAAACTTGCTGCTGCTGCTGCTGATGATGATGATGAAGATGATGATGATGATGACGAGGAAGCTGAA
GAAAAAGCGCCAGTGAAGAAATCTATACGAGATACTCCAGCCAAAAATGCACAAAAGTCAAATCAGAATGGAAAAGACTC
AAAACCATCATCAACACCAAGATCAAAAGGACAAGAATCCTTCAAAAAACAGGAAAAATCTCCTAAAACACCAAAAGGAT
CTAGTTCTGTAGAAGACATTAAAGCAAAAATGCAAGCAAGTATAGAAAAACGTGGTTCTCTTCCCAAAGTGGAAACCAAG
TTCATCAATTATGTGAAGAATTTCTTCTGGATGACTGACCAAGAGGCTATTCAAGATCTCTGGCAGTGGAGGAAGTCTCT
TTAAGAAAATAGTTTAAACAATTTGTTAAAAATTTTCCATCTTATTTCATTTCTGTAACAGTTGATATCTGGCTGTCCTT
TTTATAGTGCAGAGTGAGAACTTTCCCTACCATGTTTGATAAATGTTGTCCAGGTTCTATTGCCAAGAATGTGTTGTCCA
AAATGCCTGTTTAGTTTTTAAAGATGGAACTCCACCCTTTGCTTGGTTTTAAGTATGTATGGGATGCTATGATAGGACAT
AGTAGTAGCAGTGGTCAGACATGGAAATGGTGGGGAGACAAAAGTATACACGTGAAATAAAACTCAGTATTTTAATAAAG
TAAAAAAAAAAAAAAGGTATGGGGAAAACTGCCCCCATAAAGCAATTGTCTCCACCTGGCCCTGCCCTTTGCATGTGGAG
ATTATTACAATTCAAGGTGAAATTTGGGTGGAGACACAACCAAACCATATCAGATGGCTATATCAAAAAGACAAGAAATA
ACAAGCATTGGTGAGAGTGTGGAGAAGACGGAACCCTTGTACACAGTTGGTGGGAACACAGTTTGGTGCAGCCATTATAG
AAAACGTCATGGAGGTTCCTAAAGAAATTAAAAAGAGAACTACCATATGAGCCAGCAATCCCTTTTCCAGATATACACCC
AAAGGAAATGAAATCACCACCTCATAAAGATATCTGCTTTCCTGTTTATTATAACACATCTTTATTCATAATAGCCAAGA
TACGGAACTAACTGAAGTGACCATTAACAGATGAATGGATAAAGAAATTGTCATATATATAAAACACACATTATATAC
ATACATACATATATATATATAAAAAAATAGAATATTATTCAGCCTTGTAAAAAGAAATCCTGTTATGTTGGGAACAAGCC
CCCCAAAATCTGGCCATAAACTCACCCCAAAACTGGCCATAAACAAAATCTCTGCAGCACTGTGACATGTTAGTGATGGC
CATAACACCCACGCTGGAAGGTTGTGGGTTTATGGGAATGAGGGCAAGGAACACCTGGCCTGCCCAGGGTGGAAAACCAC
TTAAAGGCGTTCTTAAGCTACAAACAATAGCATGAGCGATCTGTGCCTTAAGGACATGTTCCTGCTGCAGTTAACTAGCC
CAATCTATTCCTTTAATTCGGCCCATCCCTTCATTTCCCATAAGGGATACTTTTAGTTAATTTAATATCTATAGAAACAA
TGCTAATGACTGGCTTGCTGTTAATAAATACATGGGTAAATCTCTGTTCTGGGCTCTCAGCTCTGAAGGCTGTGAGACCC
CTGATTTCCCACTTCACACCTCTATATTTCTGTGTGTGTGTCTTTAGTTCCTCTGGCGCTGCTGGGTTAGGATCTACCCA
ACTGAGCTGGTCTCAGCACTGTTATTTGAAACAATGTAAATAAACCTATAGGGTTTATTATATATACTAAGTGAAATAAG
CCAGACACAGAAAAAAATGTTGCATGATCTCACTTATAGTGAAACTTAAAAAAAAAACCCAAATACATAGAAATAGAGTA
GAATGGTGGTTACCAGAGATGAGGAAAATACAGAGACACAGGTCAAAGATACAGAGTTGCAGTTACAAAGGGTGAGTAT
GTCTAGATATCTAACATACAGTATGAGGACTATAATAGTATTGCACTGTGTACTGAAAACTTGATGACAGATTTTAGATA
TTCTTACCACACAGACAAAAAGAAATGTAAGTATGTGAGATGAAGCTTATGTTAATTAGATTAAGTGTATGTACATAAAA
ACATCACATTATACACATCAAATATATACAATAAAAAAGCACATTGGGAGATACATGATAAATTTCTATCTGCAGTTGCT
ATTTGCATTTTTAAAGGATATTTTTATTTTTTAAATTTTTAGCTGACAACTGTATAATTATGGAATACAATGTGATGTTC
TGATATATGTGTACTATGGAATGATTAAATCAAACTAACATTTCATCATCTCACATACTTATCATTTTCTGTGGTGAG
AACATTTGAAATTTATTCTCTTGACAATTTTGAAAGATCCAATACACTATTATTAACTATAGTCACTATGCTGTGCAATA
GATCTCAAAAGCTTATTCCTCTTAACTGAAACCTACTACCCTGTGACATGTGGGGAACATCTCTCTATTCCCCAGCCCCA
GTCCCTAAATGTTGAATACACAATGGGGAAAAAATAGTCTTCAATGAATGGTGGTGGGAAAATTGTATATCCACATGCAG
AAGAATAAAATTGGACCCTTACCTCCTACCATATATAAAAACCAACTGAAATCGATTAAAGACTTAAACTGTAAAACTA
CTAAAAGAAAATATAGGGAGAAAGCTTCACAACGTTGGTCTGGGCAATAATTTTTGGATTTGACCTCAAAAGTAGATGCA
ACCACAGTGAAAATAAACAAATGAAATTGCATAAAATAAAAAATGTTTCTGCACAGCAAAGGAAATAATTAATGGAGTAA
AGAGACAACTTACAGATTGGGAGAATATATTTCCAAACTATTGTTGGGGCTCAGAAACGGATACCCTAACATATAGTGAT
TTGACAGGTGGAACTAAAGAAGACTCGAGGTCTCTCTGACCTCCTCCTCAACCTCTGTTTCTTAATCCTCTGTTTTCCCA
GAGCACAGGAGGAAGTTCTCTGAAGTTCCTTTATCTGCTTTAAGTTCAGACTCACCAAATAAGAAAACAGTTAATTCTGG
TGCCTTCCATGGGTTTTTCATTAACTGAAGTCATATTGTAGGAAAAGAATGAAGTCTCTTAACCCACCTGGATAGATTTT
TCTCACAAACCATTGTCTTCTCTGCAAGCCCAGTAGACTTTGTCCCAGGCCATTTATGTGATCTTCACCCCATTAAGTTC
```

```
TCCCCAAATTATTTACTCTTCCCCCAAAATCACCCACACTTCCCCATCTCCCTCTCCCCTAAGGACAAGGGTATATCACC
ATCTATACCCCATTGCTTGGTGGGGTGATCACTCTGATTTCTCTGCATGCATTTTTATAGATTTGTTTGCCATTTATCCT
ATTAATCTGCATTCTGTCAATTGTGGCAAGTTATCAGCGTATTTTTTAACTTTTAATTATTCTTAATGGAAAGACACTTC
TGTATACACTGGAAATCTCAGGAAATTTCTTTTTTCCTTAAGCTTAATTGAAATGTTTACTTTTCAGTAAAATTTCAAGC
TTGAGTAATGTTCATGCCTGCTTTTCATTGAAATAGAATGAAAAGGTTGCACAATATTATTTTCATGAAATTACTTCTAG
TATAATTCTGAATAAACACTTTGAAAGGCAGTGAACTGTAAATATATTACAGCTATCTCTTATTCTTCAGGGTGGGTCTC
AATTATGTAAAGATGATTGGCTTTCTGTTTTAAGTAATAGTCATTTTGAAAACACTTTAAAACTGTCAAGTGAAGTGTG
ATGGCTAATTTTATATGTCAACTTGACTGAGATAAGGTATACTCAGCAGGGAAAGCATTATCTCTGGATGTGTCTATGAG
GGCTTTTCTGAAAGAGATTGATGTTGGAATCACTAAGCTGTGTAAAGATGATCTACCTTCACCAATGTTGGCAGGCATCA
TCCAATCCGTGCAGGTCTTACCCAAACAGAGCAAAAAGTCAGGTAGGGTAAATTTATTCTCTTCTAAAGCTAGGGCATCC
ATTTCTTTCTACTCTCAGACATTGGAGTTCCCCAGTTTCCCAGACCTTTGGCCTTACACTGAGAATTATGCTTTGTTTTA
TTTAGGCATATTTTAATAATACTTCATGTATTGGTCAGGAAGACATTTCCTGGTTCTAAGGCTTTCGACTCAGACTAGAT
TACAACACTGGCTTTCCTGGTTCTCTAGCTTGCAGAAGAGAATACTAGGAAGCTTGTACTTCCTACATAGGTAAGAAGAG
GAAACTAAATCTCTGGCTTATAAACTATGTAACCAACCACAATTCACAAAGATTCCATAATTTCCTTGGCAGGAGGCTAA
GATCTAAAGCCCAATATGATTGTACTTCTCTTATCAGTTTTCTGCAAACAAAAGAGAATAGAACTAAAATACAGCTTTGT
TAAAATAATGTTGATTCATTTGCATGAAATATCAGATCACAGAAACAACATATTTTTATAAAATATGAGCTCTGTCACAC
TGGCCCTCATAGACACATCCAGAGATAATGCTTTCCCTGCTGAGTATACCTTATCTCAATCAAGTTGACATATAAAATTA
GCCATCACACTTCACTTGACAGTTTTAAAATTAGGTTGTTTTTTCCAAATGACAAAAAGCAGGAGCAAACTGCAAGTGCT
CCTGCAACACATCTAACAAGAAAGCCTTATCTGACTTTTATGAGGTTCAGTATAATGCCCAAACTGCATATTTCAAACTC
TTGCCATCAGATTCACCTAAGAAGACAAAAGATCTATTGCCTTTGAACCTACATTCCCAAAGCGTGCAACATGAACTTGC
CTACATCCAAGGGGTAGGACCTTCTTACGTAGTGAATTCCCAAAGTGTTGCTGAAATTATGAACAGTGCTCTGCCTCATA
CTGACAAGTGAAACCTCTATCTCTGATTGCCCAAAAATGTCTAATGAAGCTATAGCATCACTACAATTTGGAATAGACAC
TTAATCCAGAATTGAAGAGATATAGATAAAAATGAACTGAGTGCAATGCCAAGAGATAGGATAAATATGGCCCCCTCCAA
AAAAAGATCCTAAGTCCATGACAATGGTGCTAGAGATTTCTCACAAAACTCCAAGGAGTAAAACAAGAAACTATAAAGAC
AGATGGAGAACCACATGTTATGGTGCATCATATAAACAACGAAGAGATGGTAGTCAATTGTGTAAGAATCTTGAAGTTGT
TGGGTTCAAATAAAGAGAATTATGAAATGAAATAAGTATCCAATTAACACAGGAAATAATATGAACAATGGGGTTAGGAG
AACAGAGATCAATGTAAGAGATGTTTCCAAAAGTAATGATGTTGTCATGGAAGTTGGCAGATGATCAAATTATCAGGTAT
GTAAAACTGATCATGAAGGTGTTCCAAGGAAATTACTCTAATTGTTTCATCAATATTGAACCCAAAATTAAATCAACACA
AACATAGAAAAGGAAAAAAAAAAAAAGGCAAAAGGCACAGTGTAATGATGCTCTCTTTACTCCTTTGGAAGGGATTGATA
AAATTGCAACTACTTCTTGTATAAAGAATCATCCATTTTATAGATGACTACAAATTCAATCCTAAACCTTCAAATACAAT
GAAAAGAAAGTATGTACTGAGAGCTGGAATGACTACTCACCTAGGCCAAACTCTCACCTAGGAATGAAGCATCAGGCACA
CAGTGCACTGAGCATGGCTGAAGGTGCATTGACTAATCTTCTAAAACCTACTCCAGAATGAGAGAGACTGGTACAACTCC
ATTTGTGCTAAATTTGGAGATATAAACAATATTTTGGAGTTAAAACCTAGAAGTTTTCAAATCTACATAAAAAATCAATT
TCTTTTTTTTCTTGCAAAAGCAGAAAAGATGAGAGGACATTGGCACCTGCCAAGAGTGTTGATGAAATGGGGTACTTCCC
TGGCCTCTTTGCAGGAGTTGCAAAGGGGTTGGCTTGTTTACTCAGCCCACAGCTCTCAACCCCTCATGGGAGGGGAAACT
CACAGGTGAGTGGGTGTAGGGATCAGGATGAGGGCTTCTGTGTGCCAGCAGGAGTAGAACTCTGTGCTGCCCCATGGCAA
CATCTAGGGGAGTTCCCATGACCCCTCAAGCCCCAGAGGGTGAGTGTTACAGTGTGCTCTTTTAGCTTTTCTATCCACAG
ATGACTTAAGTATTTAACAGCTCAGTGGAGGGTCAGGGTGACAGCCTTTTGGACCTGCCTTCTTGGTACCTCAGTTCTTG
TTTAGCGTCCAGGAAGAATCAGGTCATGCAAACAAATTGAAGGGTAGTGAATGTGGAGGATTTTATTGAGGGATGGAAGT
GGCTCTCAGCGGGATAGGGAGCTGGAAAGTGGATGGAATGGGAAGGTGGTATTCCCCTGGAGTTCAGACAAACTCTTCTC
CGAGGTCCCACCATCAAGCTGTCCCTCTGAGCTCAAACTGCTTCTCTCTGACATCCGGCTGCTTCTTCTTTTCTTTCTTT
CTCTGTCACTCTGCTATTCCGCTGCTCTGCTGCTCTGCCACTCTGGTGATGGGGCCTGTGGTTTTTATGGGTAGAGGATG
GGGTGTGGGGTGGGCCAAAAAGCAACATGTGAGTGGGAAAGCAGGAAATGCATGTTCTCATTTTGGTCTGTGATCCCAGG
CTTGAGGATGTGTGGCTCTTGCTTGGGACAGCCCTCTTCTGCCCAGTATTTCCCTGCTTCCCACCCATATCACCAACACT
GCTCCTAACCCTACTCAAAAGGTACATTAAAATATAGAGGTGGAAAGTTATATGCTGCTTCTCTCAGAGCAAACCCTTTC
TCTCTTATGTAAAATAACTAACTTTCCTCCACAGCATAATTTAAAAAGAAAACTAATGATGACATTAGTCAGAAATCTGT
AGACATCTCTACTCCTCTAGTAACTCTGAAAACTGGGTCTGCTAATGAGTTGGTAGCATTAAATCTGAAGACTGAAAGTT
TTCTATTCTGTTCCTTCTCTTTTTACCCTTACTTTAGTGGAACAGTAAATTTCAGTAAAATCAGTGAAGGTGTATCTGCC
AAGGTTCCAATCAACACAGAGGAAGAGAAGACTTGTTCTATAACTAAACTAGTAGACAACCCAGAAAAATTATCTCTACT
TATAATAAACAGAAATCCTAATTACTGCATCCAACTACCTGACCATCATTCATTTTTAAGAGACAGAATTTTTAACGTTC
CAAAGTTCTCACCCAAAAGAGATCTGACAGTTTCTGACTGCACTCCATTCTGGAGAAGTTGCTACTAAATTGAATAGAGT
AAAGTTATTGTTCCTTTAAATACAATATCTATGAATGGACAATCAAGATTTATGTCCCTTAAGGCATAAGACAGCTGTTA
AATGTTGTTCCTCCATTGTACTAATCAACTTCATACTACTGGAGGATATTTGTAAAAATATTTATATACACACATAC
ACACACTCTTAAAATAATTTGTGCTAGGTTATTTATTTATTTATTTATTTATTTATTTATTGAGACAAGGTCTC
ACTCTGTTGCTCAGGCTGGAGTGCAGTGGCATGATCATAGCTCACTGCAGCCTCAGCTTCCCAAACTCTGGCGATCCTCT
CACATCAGCCTCCCAAGCAGCTGGGACCACAGGCATGTGCCATTATACCTGACTAATTTTTGAATTTTTTGTAGAGACAG
AGTTACACCGTGTTGCCAAGGCTGGTCTCAAAATTTTGGGGTCAAACAATCTACCTGCCTTGGTCTTTCAAAGTGCTTGG
ATTATAGTTGTGAGCCACTGAGCTCAGCCTGTCCTAGGTCTTTTAATGTCAAAAGAACACTTGCCCGCATATATATATAT
ATGTATACATATTTGTCATTTCTTTGTCTTAAAACTGGAATATCCTGCTTTAAAACTTTAAGACAAAGTGTAAATGATTT
TCTTTTTTTAATGAGAAATAGTCACCTTTTGTCAATTTTATTTGCTGATTTTATTGACTTGCGTATTCTACAGTGGAGAC
CTCAACATATTCTATGTATTGTGTTTTAGTAGCAAGGTGAACATATGTATGATATTTTCCACCTTAATGTTACTCTTTAA
```

```
TTGAATACATTTATAAGTTACATTAAAGCTTATTTTATGTTGAAAGAAGGAGCTAGAAAACTAGTGATGTTAAACAATGT
GTTAACATTTATTTTCAATGATGAATCATTTAGTTTACAAGACATATCTTGTATTTTCTTTAGGTAATTGCCAAATCAGA
GTCAAATTTATGGTAAGTTCATAGTACTTTAATATTTTATTAATATGAAATACTTGATTTTTACATTCTTAGAATTTTAA
TTGCGCAAGGAGAACTTCCAATAAATATTTTCTGTATATAATTATGACACTTTCTTCACAAATATTACATATTCTATGCT
CCAAGGCCTTGAACAATTTCTTGTGAAAATGTTGCTGCATTGTCACAGTTATTTCACAATTTTCATATAGAGAGGAATAG
GGATTAAGTTATGTTCATTTGCTTTTTTTAACTTTTAAGTTCAGAGATACATGTGCAGGATTTGTAGATTTGTTACATAG
GTAAACACATGTCATGGAGATTTATTGTACAGATTATTTTATCACTCAAGTATTATGCCTAGTATCCATTATTTTTTAAA
TTCTCTCCCTCCTTTCACCCTCTGCTGTTGGTAGGCCCCCGTTTTTGTTATTTCCCTCTAGGTATCTATGTGTTCTCAT
CATTTAGCTCTCACTTACAAGTGAGAACATGTGGTATTTGATTTTCCATTCCTGTGATAGTTTGGTAAGAATAATGGCAT
CTAGTTCCATATATGTCTCTGCAAAGGACATGATCTTGTTCCTTTTATGGCTGCATAGTATTCCATGGTGTATATGTAC
CACATTTTCTTTATCCAGTCTATCGTTGATGGGCATTTAGGTTGATTTTGTGTCTTTGCTATTGTGAATAGTGCTGCAAT
GAACATATGTGTGCGTGTGTAGTTATTATAGAACAATTTATATTGCTTTGGGTATACGCCCAGTAATTGGATTGCTGGGT
CGAATAGTAGTGCTGTCTCTAGGTCTTTGAGAAATTGCCATACTGTCTTCCACAATGGTTGACCTAATTTACACTCCCAC
CAACAATGTAAAAGCATTCCTTTTTCAACACAACCTTGCCAACATCTATTATTTTTTGACTTTTTAATAATAGCCATCTG
ACTGGTGTGGGATGGTGTCTCATTGTGGTTTTGATCTGCATTTCTCTAATGATCAGTGATATTGAGCTTTTTTTCATATA
ATTGTTGGCCACATGTATGTCTTCTTTTGAGAAATGTCTTTTCATGTCCTTTGCTTACTTTTTAATGAGGTCTTTTTTTT
TTGTAAATTTGTTTAAGTTCCTTATAAGTGCTGGATATTAGATCATTATCAGATGCATGAATTACAAAAATTTTATCCCA
TTCTGTAGGTTGTTTACTCTGTTGATAGTTTCTCTTGCTGTGCAGAAGATCTTTAGTTTAATTAGATCCCATTTGTCAAT
CTTTCCTTTTCTTACAATTGCTTTTGGCATCTTCGTCATGAAATATTTGCCTGTGTCTATATCCTGAATGTTATTGCTTA
GGTTTTCTTCTATGGTTTTTATAGTTTTGGGTTTTACACTAAAGTCTTTAATCCATCTTGAGTTGATTTTTGTATATGAT
ATAAGGAAGGGTCCCAGTTTCAATTTTCTGCATATGGCTAGCCAGTTCTCCCAGCACTATTTACAGGGGAATCCTTTCCC
ATTGCTCATTTTTGTCAGGTTTGTCAAAGATCAGATGGTTGTAGGTGTGTGGTCTTATTTCTGAATTCTCTAATCTGTTC
CATTGGTCTATGTGTCTGTTGTTGCACTGGTACCATGCTGTTTTGGTTACTGTAGCCTTGTAGTATAGTTTGAAATTGGG
TAGTGTGATGCCTCTGGCTTTGCTGTTTTTGCTTAATATTATTCAGGCTATTCAGGCTCTTTTTTATTCCATATAAATTT
TATTCCATATAAATTTTAAAATAGGTATTTTTCTATTGAATCTGTAAGTTGCATTGGGAAGTACGGCCATTTTCAAGATC
TTGATTCTTCCTATGCATAAGCATGGAATGTTTTTCCATTTGTTTGTGTCTTCTCTGATTTCTTTGAGCAGTGGTTTGTA
CTTCTCCTTGAGGAAGCCCTTCACTTTTCTTGTTAGCTGTATTCCTAGGTATTTGTTCTCTTTGTAGCAATTGTGATTT
GGCTCTCTGCTTGCCTGTTGCTGGTGTGTAGGAATGCTAGTGATTTTTGCAAATTGATTTTGTAACCTGAGACTTTGCTG
AAGTTGCTTCTCAGCTTAAGAAGCTTTTGGGCTGAGATGATGGGACTTTCCAAATATAGAATCATGTCATCTGCAAACAA
AGACAATTTTACTTCCTCTCTTCCTCTTTGAATATGCTTTATTTCTTTCCCTTGCCTGATTGCCCAGGCCAAAACTTCTA
ATACTATGTTGAAAAGGTGTAGTGAGAGAGGTTATCCTTGTCTTGTGTCAGTCTTCAAGGGGAATTCTTTGAGCTTTTGC
CCATTTAATATGATATTGACTGTAGATTTGTCATATATGACTCATTATTTTGATGCCTGTTCCTTAAACACCAGGTAGAT
TGAGAGTTTTTAACATGAAGGGATATTGAATTTTATTGAAAGCCTTTTCTGCATTTACTGAGATAAGCATGTGGTTTTTG
TCTTTAGTTTTATTTATCTGATGAATTACATTTATTGGTTGGTGTATGTTGAACCAACCTTGCATCCCAAAGATGAAGCC
GACTTGATCGTGGTGAATAAGCTTTTCAATGTGCTGCTGGATTCAGTTTGTCAGTATTTTGTTGAGAATTTTTGCATATG
ATCATCAAGGATATTGGCCTGAAGTTGTCTTTTTGTTTGTATTTCTGCCAGGTTTTGGTATCAGGATGGTGCTGCCCTCA
TAGAATGAGTTAGGGAGAAGTCACTTCTTTCAAGTTTCTGAAATAGTTTCAGTAGGAATGGTACCAGCTTTTCT
```

HUMAN mRNA SEQUENCE : hR14-023.1 (Seq ID No: 111)
```
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGAATTTCCTGTTGAG
ATATAAAGGCCCATCTGATCACTGGATTGGGCTGAGCAGAGAACAAGGCCAACCATGGAAATGGATAAATGGTACTGAAT
GGACAAGACAGTTAGTCATGAAAGAAGATGGTGCCAACTTGTATGTTGCAAAGGTTTCACAAGTTCCTCGAATGAATCCA
AGACCTGTCATGGTTTCCTATCCTGGGAGCAGGAGAGTGTGCCTATTGAATGACAAAGGTGCCAGTAGTGCCAGGCACT
ACACAGAGAGGAAGTGGATTTGTTCCAAATCAGATATACATGTCTAGATGTTACAGCAAAGCCCCAACTAATCTTTAGAA
GCATATTGGAACTGATAACTCCATTTTAAAATGAGCAAAGAATTTATTTCTTATACCAACAGGTATATGAAAATATGCTC
AATATCACTAATAACTGGGAAAATACAAATCAAATCATAGTAAAATATTACCTGTTTTCATGGTGCTAATATTACCTGT
TCTCCCACTGCTAATGACATACCCGAGACTGAGTAATTTATAAATAAAAGAGATTTAATTGACTCATAGTTCCACATGGC
TGGGGAGGTCTTGCAATCATGACAGAAGGCAAATGGGAAGCAAAGTCATGTCTTATGTGGTGGCAGGCAGGGGGACTTGT
GCACAGGAACTCCTATTTATACAACCATCAGATATCTTGAGACAAGAACAGTATGGGGCTCCCTGGTGTGATTCCGTCCT
GCGCGGCTGTTCTCTGGAGCAGCATTCATTTATCTTCGTCTGCCTTGTCTCCTACCTAAGTGTGTGTCGCCACCCGATGG
AAGATTCGATGGACATGGACATGAGCCCCCTGAGGCCCCAGAACTATCTTTTCAGTTGTGAACTAAAGGCCGACAAAGAT
GATCACTTTAAGGTGGATAATGATGAAAATGAGACCAGTTATCTTTAAGAACGGTCAGCTCAGGGGCTGGTGCAAAGGAT
GAACTGCACATTGTTGAAGCAGAGGCCATGAATGACGAAGGCAGTCCAATTAAAGTAACACTGGCAACTTTGAAAATGTC
TGTACAGCCAACGGTTTCTCTTGGGGGCTTTGAAATAACACCACCAGTGGACTTAAGGTTGAAGTGTGGTTCAGGGCCAG
TGCATATTAGTGGACAGCACTTAGTAGCTGTGAAGGAAGGTGCAGAGTCAGAAGATGAAGAAGAGGAGGATGTGAAACTC
TTAAGTATATCTGGAAAGCAGTCTGCCCCTGGAGGTGGTGCTGCTGCTGATGATGATGATGAAGATGATGATGATGATGA
```

```
TGACGAGGAAGCTGAAGAAAAAGCGCCAGTGAAGAAATCTATACGAGATACTCCAGCCAAAAATGCACAAAAGTCAAATC
AGAATGGAAAAGACTCAAAACCATCATCAACACCAAGATCAAAAGGACAAGAATCCTTCAAAAAACAGGAAAAATCTCCT
AAAACACCAAAAGGATCTAGTTCTGTAGAAGACATTAAAGCAAAAATGCAAGCAAGTATAGAAAAACGTGGTTCTCTTCC
CAAAGTGGAAACCAAGTTCATCAATTATGTGAAGAATTTCTTCTGGATGACTGACCAAGAGGCTATTCAAGATCTCTGGC
AGTGGAGGAAGTCTCTTTAAGAAACAGTTGATATCTGGCTGTCCTTTTTATAGTGCAGAGTGAGAACTTTCCCTACCATG
TTTGATAAATGTTGTCCAGGTTCTATTGCCAAGAATGTGTTGTCCAAAATGCCTGTTTAGTTTTTAAAGATGGAACTCCA
CCCTTTGCTTGGTTTTAAGTATGTATGGGATGCTATGATAGGACATAGTAGTAGCAGTGGTCAGACATGGAAATGGTGGG
GAGACAAAAGTATACACGTGAAATAAAACTCAGTATTTTAATAAAGTAAAAAAAAAAAAAAAAGGTATGGGGAAAACTGCCC
CCATAAAGCAATTGTCTCCACCTGGCCCTGCCCTTTGCATGTGGAGATTATTACAATTCAAGGTGAAATTTGGGTGGAGA
CACAACCAAACCATATCAGATGGCTATATCAAAAAGACAAGAAATAACAAGCATTGGTGAGAGTGTGGAGAAGACGGAAC
CCTTGTACACAGTTGGTGGGAACACAGTTTGGTGCAGCCATTATAGAAAACGTCATGGAGGTTCCTAAAGAAATTAGAAA
TGAAATCACCACCTCATAAAGATATCTGCTTTCCTGTTTATTATAACACATCTTTATTCATAATAG
```

HUMAN PROTEIN SEQUENCE : hP14-023.1 (Seq ID No: 112)
MNDEGSPIKVTLATLKMSVQPTVSLGGFEITPPVDLRLKCGSGPVHISGQHLVAVKEGAESEDEEEEDVKLLSISGKQSA
PGGGAAADDDDEDDDDDDDEEAEEKAPVKKSIRDTPAKNAQKSNQNGKDSKPSSTPRSKGQESFKKQEKSPKTPKGSSSV
EDIKAKMQASIEKRGSLPKVETKFINYVKNFFWMTDQEAIQDLWQWRKSL*

HUMAN mRNA SEQUENCE : hR14-023.2 (Seq ID No: 113)
```
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGAATTTCCTGTTGAG
GGACAAGACAGTTTCCTATCCTGGGAGCAGGAGAGTGTGCCTATTTGAATGACAAAGGTGCCAGTAGTGCCAGGCACTAC
ACAGAGAGGAAGTGGATTTGTTCCAAATCAGATATACATGTCTAGATGTTACAGCAAAGCCCCAACTAATCTTTAGAAGC
ATATTGGAACTGATAACTCCATTTTAAAATGAGCAAAGAATTTATTTCTTATACCAACAGGTATATGAAAATATGCTCAA
TATCACTAATAACTGGGAAAATACAAATCAAAATCATAGTAAAATATTACCTGTTTTCATGGTGCTAATATTACCTGTTC
TCCCACTGCTAATGACATACCCGAGACTGAGTAATTTATAAATAAAAGAGATTTAATTGACTCATAGTTCCACATGGCTG
GGGAGGTCTTGCAATCATGACAGAAGGCAAATGGGAAGCAAAGTCATGTCTTATGTGGTGGCAGGCAGGGGGACTTGTGC
ACAGGAACTCCTATTTATACAACCATCAGATATCTTGAGACAAGAACAGTATGGGGCTCCCTGGTGTGATTCCGTCCTGC
GCGGCTGTTCTCTGGAGCAGCATTCATTTATCTTCGTCTGCCTTGTCTCCTACCTAAGTGTGTGTCGCCACCCGATGGAA
GATTCGATGGACATGGACATGAGCCCCCTGAGGCCCCAGAACTATCTTTTCAGTTGTGAACTAAAGGCCGACAAAGATGA
TCACTTTAAGGTGGATAATGATGAAAATGAGACCAGTTATCTTTAAGAACGGTCAGCTCAGGGGCTGGTGCAAAGGATGA
ACTGCACATTGTTGAAGCAGAGGCCATGAATGACGAAGGCAGTCCAATTAAAGTAACACTGGCAACTTTGAAAATGTCTG
TACAGCCAACGGTTTCTCTTGGGGGCTTTGAAATAACACCACCAGTGGACTTAAGGTTGAAGTGTGGTTCAGGGCCAGTG
CATATTAGTGGACAGCACTTAGTAGCTGTGAAGGAAGGTGCAGAGTCAGAAGATGAAGAAGAGGAGGATGTGAAACTCTT
AAGTATATCTGGAAAGCAGTCTGCCCCTGGAGGTGGGCAGAAAAAAGTAAAACTTGCTGCTGCTGCTGCTGATGATGATG
ATGAAGATGATGATGATGATGACGAGGAAGCTGAAGAAAAAGCGCCAGTGAAGAAATCTATACGAGATACTCCAGCC
AAAAATGCACAAAAGTCAAATCAGAATGGAAAAGACTCAAAACCATCATCAACACCAAGATCAAAAGGACAAGAATCCTT
CAAAAAACAGGAAAAATCTCCTAAAACACCAAAAGGATCTAGTTCTGTAGAAGACATTAAAGCAAAAATGCAAGCAAGTA
TAGAAAAACGTGGTTCTCTTCCCAAAGTGGAAACCAAGTTCATCAATTATGTGAAGAATTTCTTCTGGATGACTGACCAA
GAGGCTATTCAAGATCTCTGGCAGTGGAGGAAGTCTCTTTAAGAAAATAGTTTAAACAATTTGTTAAAAATTTTCCATCT
TATTTCATTTCTGTAACAGTTGATATCTGGCTGTCCTTTTTATAGTGCAGAGTGAGAACTTTCCCTACCATGTTTGATAA
ATGTTGTCCAGGTTCTATTGCCAAGAATGTGTTGTCCAAAATGCCTGTTTAGTTTTTAAAGATGGAACTCCACCCTTTGC
TTGGTTTTAAGTATGTATGGGATGCTATGATAGGACATAGTAGTAGCAGTGGTCAGACATGGAAATGGTGGGGAGACAAA
AGTATACACGTGAAATAAAACTCAGTATTTTAATAAAGTAAAAAAAAAAAAAAAGGTATGGGGAAAACTGCCCCCATAAAG
CAATTGTCTCCACCTGGCCCTGCCCTTTGCATGTGGAGATTATTACAATTCAAGGTGAAATTTGGGTGGAGACACAACCA
AACCATATCAGATGGCTATATCAAAAAGACAAGAAATAACAAGCATTGGTGAGAGTGTGGAGAAGACGGAACCCTTGTAC
ACAGTTGGTGGGAACACAGTTTGGTGCAGCCATTATAGAAAACGTCATGGAGGTTCCTAAAGAAATTA
```

HUMAN PROTEIN SEQUENCE : hP14-023.2 (Seq ID No: 114)
MNDEGSPIKVTLATLKMSVQPTVSLGGFEITPPVDLRLKCGSGPVHISGQHLVAVKEGAESEDEEEEDVKLLSISGKQSA
PGGGQKKVKLAAAAADDDDEDDDDDDDEEAEEKAPVKKSIRDTPAKNAQKSNQNGKDSKPSSTPRSKGQESFKKQEKSPK
TPKGSSSVEDIKAKMQASIEKRGSLPKVETKFINYVKNFFWMTDQEAIQDLWQWRKSL*

HUMAN mRNA SEQUENCE : hR14-023.3 (Seq ID No: 115)
```
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
```

CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGAATTTCCTGTTGAG
ATATAAAGGCCCATCTGATCACTGGATTGGGCTGAGCAGAGAACAAGGCCAACCATGGAAATGGATAAATGGTACTGAAT
GGACAAGACAGTTTCCTATCCTGGGAGCAGGAGAGTGTGCCTATTTGAATGACAAAGGTGCCAGTAGTGCCAGGCACTAC
ACAGAGAGGAAGTGGATTTGTTCCAAATCAGATATACATGTCTAGATGTTACAGCAAAGCCCCAACTAATCTTTAGAAGC
ATATTGGAACTGATAACTCCATTTTAAAATGAGCAAAGAATTTATTTCTTATACCAACAGGTATATGAAAATATGCTCAA
TATCACTAATAACTGGGAAAATACAAATCAAATCATAGTAAAATATTACCTGTTTTCATGGTGCTAATATTACCTGTTC
TCCCACTGCTAATGACATACCCGAGACTGAGTAATTTATAAATAAAAGAGATTTAATTGACTCATAGTTCCACATGGCTG
GGGAGGTCTTGCAATCATGACAGAAGGCAAATGGGAAGCAAAGTCATGTCTTATGTGGTGGCAGGCAGGGGACTTGTGC
ACAGGAACTCCTATTTATACAACCATCAGATATCTTGAGACAAGAACAGTATGGGGCTCCCTGGTGTGATTCCGTCCTGC
GCGGCTGTTCTCTGGAGCAGCATTCATTTATCTTCGTCTGCCTTGTCTCCTACCTAAGTGTGTGTCGCCACCCGATGGAA
GATTCGATGGACATGGACATGAGCCCCCTGAGGCCCCAGAACTATCTTTTCAGTTGTGAACTAAAGGCCGACAAAGATGA
TCACTTTAAGGTGGATAATGATGAAAATGAGACCAGTTATCTTTAAGAACGGTCAGCTCAGGGGCTGGTGCAAAGGATGA
ACTGCACATTGTTGAAGCAGAGGCCATGAATGACGAAGGCAGTCCAATTAAAGTAACACTGGCAACTTTGAAAATGTCTG
TACAGCCAACGGTTTCTCTTGGGGGCTTTGAAATAACACCACCAGTGGACTTAAGGTTGAAGTGTGGTTCAGGGCCAGTG
CATATTAGTGGACAGCACTTAGTAGCTGTGAAGGAAGGTGCAGAGTCAGAAGATGAAGAAGAGGAGGATGTGAAACTCTT
AAGTATATCTGGAAAGCTGCTGCTGATGATGATGATGAAGATGATGATGATGATGACGAGGAAGCTGAAGAAAAGC
GCCAGTGAAGAAATCTATACGAGATACTCCAGCCAAAAATGCACAAAGTCAAATCAGAATGGAAAAGACTCAAAACCAT
CATCAACACCAAGATCAAAAGGACAAGAATCCTTCAAAAAACAGGAAAAATCTCCTAAAACACCAAAAGGATCTAGTTCT
GTAGAAGACATTAAAGCAAAAATGCAAGCAAGTATAGAAAAACGTGGTTCTCTTCCCAAAGTGGAAACCAAGTTCATCAA
TTATGTGAAGAATTTCTTCTGGATGACTGACCAAGAGGCTATTCAAGATCTCTGGCAGTGGAGGAAGTCTCTTTAAGAAA
ATAGTTTAAACAATTTGTTAAAAATTTTCCATCTTATTTCATTTCTGTAACAGTTGATATCTGGCTGTCCTTTTTATAGT
GCAGAGTGAGAACTTTCCCTACCATGTTTGATAAATGTTGTCCAGGTTCTATTGCCAAGAATGTGTTGTCCAAAATGCCT
GTTTAGTTTTTAAAGATGGAACTCCACCCTTTGCTTGGTTTTAAGTATGTATGGGATGCTATGATAGGACATAGTAGTAG
CAGTGGTCAGACATGGAAATGGTGGGGAGACAAAAGTATACACGTGAAATAAAACTCAGTATTTTAATAAAGTAAAAAAA
AAAAAAAAGGTATGGGGAAAACTGCCCCCATAAAGCAATTGTCTCCACCTGGCCCTGCCCTTTGCATGTGGAGATTATTAC
AATTCAAGGTGAAATTTGGGTGGAGACACAACCAAACCATATCAGATGGCTATATCAAAAGACAAGAAATAACAAGCAT
TGGTGAGAGTGTGGAGAAGACGGAACCCTTGTACACAGTTGGTGGGAACACAGTTTGGTGCAGCCATTATAGAAACGTC
ATGGAGGTTCCTAAAGAAATTA

HUMAN PROTEIN SEQUENCE : hP14-023.3 (Seq ID No: 116)
MFLTIIVCGMVAALSAIRANCHQEPSVCLQAACPESWIGFQRKCFYFSDDTKNWTSSQRFCDSQDADLAQVESFQELNFL
LRYKGPSDHWIGLSREQGQPWKWINGTEWTRQFPILGAGECAYLNDKGASSARHYTERKWICSKSDIHV*

HUMAN mRNA SEQUENCE : hR14-023.4 (Seq ID No: 117)
GTTTCCTATCCTGGGAGCAGGAGAGTGTGCCTATTTGAATGACAAAGGTGCCAGTAGTGCCAGGCACTACACAGAGAGGA
AGTGGATTTGTTCCAAATCAGATATACATGTCTAGATGTTACAGCAAAGCCCCAACTAATCTTTAGAAGCATATTGGAAC
TGATAACTCCATTTTAAAATGAGCAAAGAATTTATTTCTTATACCAACAGGTATATGAAAATATGCTCAATATCACTAAT
AACTGGGAAAATACAAATCAAATCATAGTAAAATATTACCTGTTTTCATGGTGCTAATATTACCTGTTCTCCCACTGCT
AATGACATACCCGAGACTGAGTAATTTATAAATAAAAGAGATTTAATTGACTCATAGTTCCACATGGCTGGGGGAGGTCTT
GCAATCATGACAGAAGGCAAATGGGAAGCAAAGTCATGTCTTATGTGGTGGCAGGCAGGGGGACTTGTGCACAGGAACTC
CTATTTATACAACCATCAGATATCTTGAGACAAGAACAGTATGGGGCTCCCTGGTGTGATTCCGTCCTGCGCGGCTGTTC
TCTGGAGCAGCATTCATTTATCTTCGTCTGCCTTGTCTCCTACCTAAGTGTGTGTCGCCACCCGATGGAAGATTCGATGG
ACATGGACATGAGCCCCCTGAGGCCCCAGAACTATCTTTTCAGTTGTGAACTAAAGGCCGACAAAGATGATCACTTTAAG
GTGGATAATGATGAAAATGAGACCAGTTATCTTTAAGAACGGTCAGCTCAGGGGCTGGTGCAAAGGATGAACTGCACATT
GTTGAAGCAGAGGCCATGAATGACGAAGGCAGTCCAATTAAAGTAACACTGGCAACTTTGAAAATGTCTGTACAGCCAAC
GGTTTCTCTTGGGGGCTTTGAAATAACACCACCAGTGGACTTAAGGTTGAAGTGTGGTTCAGGGCCAGTGCATATTAGTG
GACAGCACTTAGTAGCTGTGAAGGAAGGTGCAGAGTCAGAAGATGAAGAAGAGGAGGATGTGAAACTCTTAAGTATATCT
GGAAAGCAGTCTGCCCCTGGAGGTGGTGATGATGATGAAGATGATGATGATGATGACGAGGAAGCTGAAGAAAAA
AGCGCCAGTGAAGAAATCTATACGAGATACTCCAGCCAAAAATGCACAAAGTCAAATCAGAATGGAAAAGACTCAAAAC
CATCATCAACACCAAGATCAAAAGGACAAGAATCCTTCAAAAAACAGGAAAAATCTCCTAAAACACCAAAAGGATCTAGT
TCTGTAGAAGACATTAAAGCAAAAATGCAAGCAAGTATAGAAAAACGTGGTTCTCTTCCCAAAGTGGAAACCAAGTTCAT
CAATTATGTGAAGAATTTCTTCTGGATGACTGACCAAGAGGCTATTCAAGATCTCTGGCAGTGGAGGAAGTCTCTTTAAG
AAAATAGTTTAAACAATTTGTTAAAAATTTTCCATCTTATTTCATTTCTGTAACAGTTGATATCTGGCTGTCCTTTTTAT
AGTGCAGAGTGAGAACTTTCCCTACCATGTTTGATAAATGTTGTCCAGGTTCTATTGCCAAGAATGTGTTGTCCAAAATG
CCTGTTTAGTTTTTAAAGATGGAACTCCACCCTTTGCTTGGTTTTAAGTATGTATGGGATGCTATGATAGGACATAGTAG
TAGCAGTGGTCAGACATGGAAATGGTGGGGAGACAAAAGTATACACGTGAAATAAAACTCAGTATTTTAATAAAGTAAAA
AAAAAAAAAAGGTATGGGGAAAACTGCCCCCATAAAGCAATTGTCTCCACCTGGCCCTGCCCTTTGCATGTGGAGATTAT
TACAATTCAAGGTGAAATTTGGGTGGAGACACAACCAAACCATATCAGATGGCTATATCAAAAGACAAGAAATAACAAG

CATTGGTGAGAGTGTGGAGAAGACGGAACCCTTGTACACAGTTGGTGGGAACACAGTTTGGTGCAGCCATTATAGAAAAC
GTCATGGAGGTTCCTAAAGAAATTA

HUMAN PROTEIN SEQUENCE : hP14-023.4 (Seq ID No: 118)
MNDEGSPIKVTLATLKMSVQPTVSLGGFEITPPVDLRLKCGSGPVHISGQHLVAVKEGAESEDEEEEDVKLLSISGKQSA
PGGGDDDDEDDDDDDDDEEAEEKAPVKKSIRDTPAKNAQKSNQNGKDSKPSSTPRSKGQESFKKQEKSPKTPKGSSSVEDI
KAKMQASIEKRGSLPKVETKFINYVKNFFWMTDQEAIQDLWQWRKSL*

HUMAN mRNA SEQUENCE : hR14-023.5 (Seq ID No: 119)
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGGTTTCCTATCCTGG
GAGCAGGAGAGTGTGCCTATTTGAA

HUMAN PROTEIN SEQUENCE : hP14-023.5 (Seq ID No: 120)
MFLTIIVCGMVAALSAIRANCHQEPSVCLQAACPESWIGFQRKCFYFSDDTKNWTSSQRFCDSQDADLAQVESFQELVSY
PGSRRVCLFE

HUMAN mRNA SEQUENCE : hR14-023.6 (Seq ID No: 121)
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGAATTTCCTGTTGAG
ATATAAAGGCCCATCTGATCACTGGATTGGGCTGAGCAGAGAACAAGGCCAACCATGGAAATGGATAAATGGTACTGAAT
GGACAAGACAGTTAGTCATGAAAGAAGATGGTGCCAACTTGTATGTTGCAAAGGTTTCACAAGTTCCTCGAATGAATCCA
AGACCTGTCATGGTTTCCTATCCTGGGAGCAGGAGAGTGTGCCTATTTGAATGACAAAGGTGCCAGTAGTGCCAGGCACT
ACACAGAGAGGAAGTGGATTTGTTCCAAATCAGATATACATGTCTAGATGTTACAGCAAAGCCCCAACTAATCTTTAGAA
GCATATTGGAACTGATAACTCCATTTTAAAATGAGCAAAGAATTTATTTCTTATACCAACAGGTATATGAAAATATGCTC
AATATCACTAATAACTGGGAAAATACAAATCAAAATCATAGTAAAATATTACCTGTTTTCATGGTGCTAATATTACCTGT
TCTCCCACTGCTAATGACATACCCGAGACTGAGTAATTTATAAATAAAAGAGATTTAATTGACTCATAGTTCCACATGGC
TGGGGAGGTCTTGCAATCATGACAGAAGGCAAATGGAAGCAAAGTCATGTCTTATGTGGTGGCAGGCAGGGGGACTTGT
GCACAGGAACTCCTATTTATACAACCATCAGATATCTTGAGACAAGAACAGTATGGGGCTCCCTGGTGTGATTCCGTCCT
GCGCGGCTGTTCTCTGGAGCAGCATTCATTTATCTTCGTCTGCCTTGTCTCCTACCTAAGTGTGTGTCGCCACCCGATGG
AAGATTCGATGGACATGGACATGAGCCCCCTGAGGCCCCAGAACTATCTTTTCAGTTGTGAACTAAAGGCCGACAAAGAT
GATCACTTTAAGGTGGATAATGATGAAAATGAGACCAGTTATCTTTAAGAACGGTCAGCTCAGGGGCTGGTGCAAAGGAT
GAACTGCACATTGTTGAAGCAGAGGCCATGAATGACGAAGGCAGTCCAATTAAAGTAACACTGGCAACTTTGAAAATGTC
TGTACAGCCAACCGGTTTCTCTTGGGGGCTTTGAAATAACACCACCAGTGGACTTAAGGTTGAAGTGTGGTTCAGGGCCAG
TGCATATTAGTGGACAGCACTTAGTAGCTGTGAAGGAAGGTGCAGAGTCAGAAGATGAAGAAGAGGAGGATGTGAAACTC
TTAAGTATATCTGGAAAGCAGTCTGCCCCTGGAGGTGGGCAGAAAAAAGTAAAACTTGCTGCTGCTGCTGCTGATGATGA
TGATGAAGATGATGATGATGATGATGACGAGGAAGCTGAAGAAAAAGCGCCAGTGAAGAAATCTATACGAGATACTCCAG
CCAAAAATGCACAAAGTCAAATCAGAATGGAAAAGACTCAAAACCATCATCAACACCAAGATCAAAAGGACAAGAATCC
TTCAAAAAACAGGAAAAATCTCCTAAAACACCAAAAGGATCTAGTTCTGTAGAAGACATTAAAGCAAAAATGCAAGCAAG
TATAGAAAAACGTGGTTCTCTTCCCAAAGTGGAAACCAAGTTCATCAATTATGTGAAGAATTTCTTCTGGATGACTGACC
AAGAGGCTATTCAAGATCTCTGGCAGTGGAGGAAGTCTCTTTAAGAAAATAGTTTAAACAATTTGTTAAAAATTTTCCAT
CTTATTTCATTTCTGTAACAGTTGATATCTGGCTGTCCTTTTTATAGTGCAGAGTGAGAACTTTCCCTACCATGTTTGAT
AAATGTTGTCCAGGTTCTATTGCCAAGAATGTGTTGTCCAAAATGCCTGTTTAGTTTTTAAAGATGGAACTCCACCCTTT
GCTTGGTTTTAAGTATGTATGGGATGCTATGATAGGACATAGTAGTAGCAGTGGTCAGACATGGAAATGGTGGGGAGACA
AAAGTATACACGTGAAATAAAACTCAGTATTTTAATAAAGTAAAAAAAAAAAAAAAGGTATGGGGAAAACTGCCCCCATAA
AGCAATTGTCTCCACCTGGCCCTGCCCTTTGCATGTGGAGATTATTACAATTCAAGGTGAAATTTGGGTGGAGACACAAC
CAAACCATATCAGATGGCTATATCAAAAAGACAAGAAATAACAAGCATTGGTGAGAGTGTGGAGAAGACGGAACCCTTGT
ACACAGTTGGTGGGAACACAGTTTGGTGCAGCCATTATAGAAAACGTCATGGAGGTTCCTAAAGAAATTA

HUMAN PROTEIN SEQUENCE : hP14-023.6 (Seq ID No: 122)
MNDEGSPIKVTLATLKMSVQPTVSLGGFEITPPVDLRLKCGSGPVHISGQHLVAVKEGAESEDEEEEDVKLLSISGKQSA
PGGGQKKVKLAAAAADDDDEDDDDDDDEEAEEKAPVKKSIRDTPAKNAQKSNQNGKDSKPSSTPRSKGQESFKKQEKSPK
TPKGSSSVEDIKAKMQASIEKRGSLPKVETKFINYVKNFFWMTDQEAIQDLWQWRKSL*

HUMAN mRNA SEQUENCE : hR14-023.7 (Seq ID No: 123)
CACTCATAGAAACTGGAGGCAAAATGCATGACAGTAACAATGTGGAGAAAGACATTACACCATCTGAATTGCCTGCAAAC
CCAGGTTGTCTGCATTCAAAAGAGCATTCTATTAAAGCTACCTTAATTTGGCGCTTATTTTTCTTAATCATGTTTCTGAC
AATCATAGTGTGTGGAATGGTTGCTGCTTTAAGCGCAATAAGAGCTAACTGCCATCAAGAGCCATCAGTATGTCTTCAAG
CTGCATGCCCAGAAAGCTGGATTGGTTTTCAAAGAAAGTGTTTCTATTTTTCTGATGACACCAAGAACTGGACATCAAGT
CAGAGGTTTTGTGACTCACAAGATGCTGATCTTGCTCAGGTTGAAAGCTTCCAGGAACTGAATTTCCTGTTGAGATATAA
AGGCCCATCTGATCACTGGATTGGGCTGAGCAGAGAACAAGGCCAACCATGGAAATGGATAAATGGTACTGAATGGACAA
GACAGTTTCCTATCCTGGGAGCAGGAGAGTGTGCCTATTTGAATGACAAAGGTGCCAGTAGTGCCAGGCACTACACAGAG
AGGAAGTGGATTTGTTCCAAATCAGATATACATGTCTAGATGTTACAGCAAAGCCCCAACTAATCTTTAGAAGCATATTG
GAACTGATAACTCCATTTTAAAATGAGCAAAGAATTTATTTCTTATACCAACAGGTATATGAAAATATGCTCAATATCAC
TAATAACTGGGAAAATACAAATCAAATCATAGTAAAATATTACCTGTTTTCATGGTGCTAATATTACCTGTTCTCCCAC
TGCTAATGACATACCCGAGACTGAGTAATTTATAAATAAAAGAGATTTAATTGACTCATAGTTCCACATGGCTGGGGAGG
TCTTGCAATCATGACAGAAGGCAAATGGGAAGCAAAGTCATGTCTTATGTGGTGGCAGGCAGGGGGACTTGTGCACAGGA
ACTCCTATTTATACAACCATCAGATATCTTGAGACAAGAACAGTATGGGGCTCCCTGGTGTGATTCCGTCCTGCGCGGCT
GTTCTCTGGAGCAGCATTCATTTATCTTCGTCTGCCTTGTCTCCTACCTAAGTGTGTGTCGCCACCCGATGGAAGATTCG
ATGGACATGGACATGAGCCCCCTGAGGCCCCAGAACTATCTTTTCAGTTGTGAACTAAAGGCCGACAAAGATGATCACTT
TAAGGTGGATAATGATGAAAATGAGACCAGTTATCTTTAAGAACGGTCAGCTCAGGGGCTGGTGCAAAGGATGAACTGCA
CATTGTTGAAGCAGAGGCCATGAATGACGAAGGCAGTCCAATTAAAGTAACACTGGCAACTTTGAAAATGTCTGTACAGC
CAACGGTTTCTCTTGGGGGCTTTGAAATAACACCACCAGTGGACTTAAGGTTGAAGTGTGGTTCAGGGCCAGTGCATATT
AGTGGACAGCACTTAGTAGCTGTGAAGGAAGGTGCAGAGTCAGAAGATGAAGAAGAGGAGGATGTGAAACTCTTAAGTAT
ATCTGGAAAGCAGTCTGCCCCTGGAGGTGGGCATGATGATGATGATGAAGATGATGATGATGATGACGAGGAAGCTG
AAGAAAAAGCGCCAGTGAAGAAATCTATACGAGATACTCCAGCCAAAAATGCACAAAAGTCAAATCAGAATGGAAAAGAC
TCAAAACCATCATCAACACCAAGATCAAAAGGACAAGAATCCTTCAAAAAGACATTAAAGCAAAAATGCAAGCAAGTATA
GAAAAACGTGGTTCTCTTCCCAAAGTGGAAACCAAGTTCATCAATTATGTGAAGAATTTCTTCTGGATGACTGACCAAGA
GGCTATTCAAGATCTCTGGCAGTGGAGGAAGTCTCTTTAAGAAAATAGTTTAAACAATTTGTTAAAAATTTTCCATCTTA
TTTCATTTCTGTAACAGTTGATATCTGGCTGTCCTTTTTATAGTGCAGAGTGAGAACTTTCCCTACCATGTTTGATAAAT
GTTGTCCAGGTTCTATTGCCAAGAATGTGTTGTCCAAAATGCCTGTTTAGTTTTTAAAGATGGAACTCCACCCTTTGCTT
GGTTTTAAGTATGTATGGGATGCTATGATAGGACATAGTAGTAGCAGTGGTCAGACATGGAAATGGTGGGGAGACAAAAG
TATACACGTGAAATAAAACTCAGTATTTTAATAAAGTAAAAAAAAAAAAAAAGGTATGGGGAAAACTGCCCCCATAAAGCA
ATTGTCTCCACCTGGCCCTGCCCTTTGCATGTGGAGATTATTACAATTCAAGGTGAAATTTGGGTGGAGACACAACCAAA
CCATATCAGATGGCTATATCAAAAAGACAAGAAATAACAAGCATTGGTGAGAGTGTGGAGAAGACGGAACCCTTGTACAC
AGTTGGTGGGAACACAGTTTGGTGCAGCCATTATAGAAAACGTCATGGAGGTTCCTAAAGAAATTAGAAATGAAATCACC
ACCTCATAAAGATATCTGCTTTCCTGTTTATTATAACACATCTTTATTCATAATAG

HUMAN PROTEIN SEQUENCE : hP14-023.7 (Seq ID No: 124)
MHDSNNVEKDITPSELPANPGCLHSKEHSIKATLIWRLFFLIMFLTIIVCGMVAALSAIRANCHQEPSVCLQAACPESWI
GFQRKCFYFSDDTKNWTSSQRFCDSQDADLAQVESFQELNFLLRYKGPSDHWIGLSREQGQPWKWINGTEWTRQFPILGA
GECAYLNDKGASSARHYTERKWICSKSDIHV*

HUMAN mRNA SEQUENCE : hR14-023.8 (Seq ID No: 125)
CACTCATAGAAACTGGAGGCAAAATGCATGACAGTAACAATGTGGAGAAAGACATTACACCATCTGAATTGCCTGCAAAC
CCAGGTTGTCTGCATTCAAAAGAGCATTCTATTAAAGCTACCTTAATTTGGCGCTTATTTTTCTTAATCATGTTTCTGAC
AATCATAGTGTGTGGAATGGTTGCTGCTTTAAGCGCAATAAGAGCTAACTGCCATCAAGAGCCATCAGTATGTCTTCAAG
CTGCATGCCCAGAAAGCTGGATTGGTTTTCAAAGAAAGTGTTTCTATTTTTCTGATGACACCAAGAACTGGACATCAAGT
CAGAGGTTTTGTGACTCACAAGATGCTGATCTTGCTCAGGTTGAAAGCTTCCAGGAACTGAATTTCCTGTTGAGATATAA
AGGCCCATCTGATCACTGGATTGGGCTGAGCAGAGAACAAGGCCAACCATGGAAATGGATAAATGGTACTGAATGGACAA
GACAGTTTCCTATCCTGGGAGCAGGAGAGTGTGCCTATTTGAATGACAAAGGTGCCAGTAGTGCCAGGCACTACACAGAG
AGGAAGTGGATTTGTTCCAAATCAGATATACATGTCTAGATGTTACAGCAAAGCCCCAACTAATCTTTAGAAGCATATTG
GAACTGATAACTCCATTTTAAAATGAGCAAAGAATTTATTTCTTATACCAACAGGTATATGAAAATATGCTCAATATCAC
TAATAACTGGGAAAATACAAATCAAATCATAGTAAAATATTACCTGTTTTCATGGTGCTAATATTACCTGTTCTCCCAC
TGCTAATGACATACCCGAGACTGAGTAATTTATAAATAAAAGAGATTTAATTGACTCATAGTTCCACATGGCTGGGGAGG
TCTTGCAATCATGACAGAAGGCAAATGGGAAGCAAAGTCATGTCTTATGTGGTGGCAGGCAGGGGGACTTGTGCACAGGA
ACTCCTATTTATACAACCATCAGATATCTTGAGACAAGAACAGTATGGGGCTCCCTGGTGTGATTCCGTCCTGCGCGGCT
GTTCTCTGGAGCAGCATTCATTTATCTTCGTCTGCCTTGTCTCCTACCTAAGTGTGTGTCGCCACCCGATGGAAGATTCG
ATGGACATGGACATGAGCCCCCTGAGGCCCCAGAACTATCTTTTCAGTTGTGAACTAAAGGCCGACAAAGATGATCACTT
TAAGGTGGATAATGATGAAAATGAGACCAGTTATCTTTAAGAACGGTCAGCTCAGGGGCTGGTGCAAAGGATGAACTGCA
CATTGTTGAAGCAGAGGCCATGAATGACGAAGGCAGTCCAATTAAAGTAACACTGGCAACTTTGAAAATGTCTGTACAGC
CAACGGTTTCTCTTGGGGGCTTTGAAATAACACCACCAGTGGACTTAAGGTTGAAGTGTGGTTCAGGGCCAGTGCATATT
AGTGGACAGCACTTAGTAGCTGTGAAGGAAGGTGCAGAGTCAGAAGATGAAGAAGAGGAGGATGTGAAACTCTTAAGTAT

EP 2 058 408 A2

```
ATCTGGAAAGCAGTCTGCCCCTGGAGGTGGGCAGAAAAAAGTAAAACTTGCTGCTGCTGCTGCTGATGATGATGATGAAG
ATGATGATGATGATGACGAGGAAGCTGAAGAAAAAGCGCCAGTGAAGAAATCTATACGAGATACTCCAGCCAAAAAT
GCACAAAGTCAAATCAGAATGGAAAAGACTCAAAACCATCATCAACACCAAGATCAAAAGGACAAGAATCCTTCAAAAA
ACAGGAAAATCTCCTAAAACACCAAAAGGATCTAGTTCTGTAGAAGACATTAAAGCAAAAATGCAAGCAAGTATAGAAA
AACGTGGTTCTCTTCCCAAAGTGGAAACCAAGTTCATCAATTATGTGAAGAATTTCTTCTGGATGACTGACCAAGAGGCT
ATTCAAGATCTCTGGCAGTGGAGGAAGTCTCTTTAAGAAAATAGTTTAAACAATTTGTTAAAAATTTTCCATCTTATTTC
ATTTCTGTAACAGTTGATATCTGGCTGTCCTTTTTATAGTGCAGAGTGAGAACTTTCCCTACCATGTTTGATAAATGTTG
TCCAGGTTCTATTGCCAAGAATGTGTTGTCCAAAATGCCTGTTTAGTTTTTAAAGATGGAACTCCACCCTTTGCTTGGTT
TTAAGTATGTATGGGATGCTATGATAGGACATAGTAGTAGCAGTGGTCAGACATGGAAATGGTGGGGAGACAAAAGTATA
CACGTGAAATAAAACTCAGTATTTTAATAAAGTAAAAAAAAAAAAAAAGGTATGGGGAAAACTGCCCCCATAAAGCAATTG
TCTCCACCTGGCCCTGCCCTTTGCATGTGGAGATTATTACAATTCAAGGTGAAATTTGGGTGGAGACACAACCAAACCAT
ATCAGATGGCTATATCAAAAAGACAAGAAATAACAAGCATTGGTGAGAGTGTGGAGAAGACGGAACCCTTGTACACAGTT
GGTGGGAACACAGTTTGGTGCAGCCATTATAGAAAACGTCATGGAGGTTCCTAAAGAAATTA
```

HUMAN PROTEIN SEQUENCE : hP14-023.8 (Seq ID No: 126)
MNDEGSPIKVTLATLKMSVQPTVSLGGFEITPPVDLRLKCGSGPVHISGQHLVAVKEGAESEDEEEEDVKLLSISGKQSA
PGGGQKKVKLAAAAADDDDEDDDDDDDEEAEEKAPVKKSIRDTPAKNAQKSNQNGKDSKPSSTPRSKGQESFKKQEKSPK
TPKGSSSVEDIKAKMQASIEKRGSLPKVETKFINYVKNFFWMTDQEAIQDLWQWRKSL*

HUMAN mRNA SEQUENCE : hR14-023.9 (Seq ID No: 127)
```
ACCGATGAATGGGATTAGTGCCCTTACAAAAGGGGCCCACCAAAGCTGCCTTGTTCCTTCTACTATGTGAGGACACATCT
AGAAGTTACCATCAATGAACCAGAAAGTGGGCCCTCACCAGGCACCAAATCTGCCAGCACTCTGATCTTGGACTTCCAGC
CTCCTGAACTGTTGTCTGCATTCAAAAGAGCATTCTATTAAAGCTACCTTAATTTGGCGCTTATTTTTCTTAATCATGTT
TCTGACAATCATAGTGTGTGGAATGGTTGCTGCTTTAAGCGCAATAAGAGCTAACTGCCATCAAGAGCCATCAGTATGTC
TTCAAGCTGCATGCCCAGAAAGCTGGATTGGTTTTCAAAGAAAGTGTTTCTATTTTTCTGATGACACCAAGAACTGGACA
TCAAGTCAGAGGTTTTGTGACTCACAAGATGCTGATCTTGCTCAGGTTGAAAGCTTCCAGGAACTGAATTTCCTGTTGAG
ATATAAAGGCCCATCTGATCACTGGATTGGGCTGAGCAGAGAACAAGGCCAACCATGGAAATGGATAAATGGTACTGAAT
GGACAAGACAGTTTCCTATCCTGGGAGCAGGAGAGTGTGCCTATTTGAATGACAAAGGTGCCAGTAGTGCCAGGCACTAC
ACAGAGAGGAAGTGGATTTGTTCCAAATCAGATATACATGTCTAGATGTTACAGCAAAGCCCCAACTAATCTTTAGAAGC
ATATTGGAACTGATAACTCCATTTTAAAATGAGCAAAGAATTTATTTCTTATACCAACAGGTATATGAAAATATGCTCAA
TATCACTAATAACTGGGAAAATACAAATCAAAATCATAGTAAAATATTACCTGTTTTCATGGTGCTAATATTACCTGTTC
TCCCACTGCTAATGACATACCCGAGACTGAGTAATTTATAAATAAAAGAGATTTAATTGACTCATAGTTCCACATGGCTG
GGGAGGTCTTGCAATCATGACAGAAGGCAAATGGGAAGCAAAGTCATGTCTTATGTGGTGGCAGGCAGGGGGACTTGTGC
ACAGCAACTCCTATTTATACAACCATCAGATATCTTGAGACAAGAACAGTATGGGGCTCCCTGGTGTGATTCCGTCCTGC
GCGGCTGTTCTCTGGAGCAGCATTCATTTATCTTCGTCTGCCTTGTCTCCTACCTAAGTGTGTGTCGCCACCCGATGGAA
GATTCGATGGACATGGACATGAGCCCCCTGAGGCCCCAGAACTATCTTTTCAGTTGTGAACTAAAGGCCGACAAAGATGA
TCACTTTAAGGTGGATAATGATGAAAATGAGACCAGTTATCTTTAAGAACGGTCAGCTCAGGGGCTGGTGCAAAGGATGA
ACTGCACATTGTTGAAGCAGAGGCCATGAATGACGAAGGCAGTCCAATTAAAGTAACACTGGCAACTTTGAAAATGTCTG
TACAGCCAACGGTTTCTCTTGGGGGCTTTGAAATAACACCACCAGTGGACTTAAGGTTGAAGTGTGGTTCAGGGCCAGTG
CATATTAGTGGACAGCACTTAGTAGCTGTGAAGGAAGGTGCAGAGTCAGAAGATGAAGAAGAGGAGGATGTGAAACTCTT
AAGTATATCTGGAAAGCAGTCTGCCCCTGGAGGTGGTGATGATGATGATGAAGATGATGATGATGATGACGAGGAAG
CTGAAGAAAAAGCGCCAGTGAAGAAATCTATACGAGATACTCCAGCCAAAAATGCACAAAGTCAAATCAGAATGGAAAA
GACTCAAAACCATCATCAACACCAAGATCAAAAGGACAAGAATCCTTCAAAAAACAGGAAAATCTCCTAAAACACCAAA
AGGATCTAGTTCTGTAGAAGACATTAAAGCAAAAATGCAAGCAAGTATAGAAAAACGTGGTTCTCTTCCCAAAGTGGAAA
CCAAGTTCATCAATTATGTGAAGAATTTCTTCTGGATGACTGACCAAGAGGCTATTCAAGATCTCTGGCAGTGGAGGAAG
TCTCTTTAAGAAAATAGTTTAAACAATTTGTTAAAAATTTTCCATCTTATTTCATTTCTGTAACAGTTGATATCTGGCTG
TCCTTTTTATAGTGCAGAGTGAGAACTTTCCCTACCATGTTTGATAAATGTTGTCCAGGTTCTATTGCCAAGAATGTGTT
GTCCAAAATGCCTGTTTAGTTTTTAAAGATGGAACTCCACCCTTTGCTTGGTTTTAAGTATGTATGGGATGCTATGATAG
GACATAGTAGTAGCAGTGGTCAGACATGGAAATGGTGGGGAGACAAAAGTATACACGTGAAATAAAACTCAGTATTTTAA
TAAAGTAAAAAAAAAAAAAAAGGTATGGGGAAAACTGCCCCCATAAAGCAATTGTCTCCACCTGGCCCTGCCCTTTGCATG
TGGAGATTATTACAATTCAAGGTGAAATTTGGGTGGAGACACAACCAAACCATATCAGATGGCTATATCAAAAAGACAAG
AAATAACAAGCATTGGTGAGAGTGTGGAGAAGACGGAACCCTTGTACACAGTTGGTGGGAACACAGTTTGGTGCAGCCAT
TATAGAAAACGTCATGGAGGTTCCTAAAGAAATTA
```

HUMAN PROTEIN SEQUENCE : hP14-023.9 (Seq ID No: 128)
MNDEGSPIKVTLATLKMSVQPTVSLGGFEITPPVDLRLKCGSGPVHISGQHLVAVKEGAESEDEEEEDVKLLSISGKQSA
PGGGDDDDEDDDDDDDDEEAEEKAPVKKSIRDTPAKNAQKSNQNGKDSKPSSTPRSKGQESFKKQEKSPKTPKGSSSVEDI
KAKMQASIEKRGSLPKVETKFINYVKNFFWMTDQEAIQDLWQWRKSL*

Table 18

435

MOUSE GENOMIC SEQUENCE : mD14-032 (Seq ID No: 129)
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNACCGTGCTTCCCGCCATGATGATACTGGC
ATAAACCTCAGAAGCTATAAACCAACCCCCAATTAAGTGCTTTTCTTTATAAGTGTTGTCCTAGTCATGGTGTCTCCTCA
CAGCAATAGAAACCCTAAGACATTTTTTTTTCATATCCATACTGCCATTTGTATTGCATGTTTTGAGAGCTGCCTATTAA
TTTTGTGAGACCAACAGCTATTAACTTGGATGATGTTTTCCCGTTTGTTTGTGTGTTTGTTTGTGTGTGTGTTTGTGTGT
TTGTTTGTGTGTGTGTATATGTGTGCGTGTTTGTGTGTGTGTGTTTGTGTGTGTGTTTGTGTGTGTGTTTGTGTGTGTGT
TTATGTGTGTGTGTTTGTGTGTGTGTGTTTGTGTGTTTGTGTGTGTGTGTGTGTGTGTGTGAGAGAGAGAGAGAGAGAGA
GAGAGAGAGAGAAACAGGATCTCTGTGTGGTTCTGGCTGTCTGAGAGCTCACACAGATTCTCTAACAGTGTGCACCAGCA
GACGCTGTGGAGATCTTGAAGTGCTTTCCCTCCAGTCTCCTCTAACAGTCCTAGATCCTGCGCTTAAACAAAGGTCCGAG
ATATATTTAGATTTGATTTTGTACATGGTGAAATAAAGAGACTGAGTTTCATTCTTCTCCATGTGGACACCCAGTTTCTC
CCAGTTTCCCCGGCACCATTTGTTGAAGGAGATGTCTGTGTTAGTTAGGGTTTTACTGCTGTGAACAGACACCATGACCA
AGGCAACTCTTATAAGGACAACATTTAATTGGGGCTGGCTTACAGGTTCAGAGGTTCAGTCCATTGTCATCGAGGTGGGA
GGTTGGCAGCATCCAGGCAGACATGGTGCAGGAGGAGCTGAGAGTTCTGACTCTTCATCTGAAGGCTGCTAGTGGAAGAC
TGGCTCCAGGTAGCTAGAGTGAGGATCTTAAGCCCACATCCACAGTGACATGCCTACTCCAACAAGGCCACACCCCTCTA
AAGTCTGGCACTTGGAAGGTGGAGGCAGGAGAGTTGAGTTCAAAACTAGTCTCAGCTATATATACAGTGACTTTAAGGGC
CAAGCATATACAAACCATCACAATGTCTCTTTTCCAATGTATATTTTATTTTATTTTATTTTTTAAAGCATCTTTATAAG
AACCAAGAAATCATTATCTGGGAGTGATGGCCCACACCTTTAATCCCAGCACTCAGGGGGCAGAGGCAGGCAGAGCTCTG
AGTTTGAGTCCAGCCTGGTCTACAAAGTAAATTCCGGGACAGTCGGCTACACAGAGAAACCCCTGTCTCCAAAACAAAAC
AACCAAAAATCAAACAAATAATAATAATAATAAAACAAGAAATCACCTGGCTATAGCTGCATGAATTGTTACTATTTTAC
TGCCTCGTTTAGCTCAGTTTGTCCTTAAAATCACTGTATATATAGCTGAGACTAGTCTTGAACTCAACTCTCCTGCCTCC
ACCTTCCAAGTGCCAGACTTAGAAGGCACAGAATTGAACTTGTGGGGGCTGTCAGCTCCAGCAACCTTTTGACAAACCTA
TGTCCTGAGAGAGGCCTGCCTGCTGTGGGTTAGCTGGAATCATTTTAACTCACCTATGCAGGAGCTCACAGAGTATCATG
AGGCACAGCAGCTCAAATGTGGCTGCAAGGTACTCTTTAAAAGAGGCATGAGGACTTGACCTAGGTCCCAGCACACATGT
AAAAACCACACTTTCATAAACGTCTGTGTGCCCAAATACCACTCCCCATGTGGTGGGTCTCTGCAAACACCAGGGCAGGC
AGAGTAACAATCCCTGTGAAGCAAGAAGGAAGTCCTCTTCAACAGAACTGGAAAGCTGGTGTTTCAAACTTCCAGAGTCT
GACACTGGGCAGATTATCTGAAGTACTGTACAATTTGGAGAAAGTCCCCCGAGTGGTGCCCCGGACCAATCAGAAACCCG
AAACCTAGATTTAATTTGTTTTCCAAGACAGGGTTTCTCTGTGTAGCCCTGGCTATCCTGGAGCTTGTTCTGTAGATGTG
TGTACGCTGGCCTCCAACTCAAAAATCTGTCTGCCTCTGCTTTCTGAGTGCTGGGATTAAAGGCGTGCGCCACCACTGCC
AGACTAAATTCTTTAAGTCCAAAACCTGGGACTGTCTTACCTTCAGAGATCCCAAATACAAAATACTCAGGTAGACTTAG
TTTTAGGGGGCAAACTGGTGAGGGGTAGCCAATCACTGGGGTTGAGGTTCCCCTAGGACCATAATCCTTACCCTTGCCTA
GAGAAGCCCCACCTCAGGATTCAGGGCATCTTCTCAGGGCTTAGTGGTATCTTAATTAGGGTTTTACTGATGTGAGGAGA
CACCATGACCCAAGCAACTCATAAAGAACAATATTTAAATAGGGCTGGCTTACAGGTTCAGAGGTTCAGTCCATTATAAT
CACAGCAGGAAGCATGGCAGCATCCAGGCAGACATGGTACTGGAGAAGGAGCTGAGAGTTCTGCTTCTTGATCCCAAGGC
AGCCAAGAGGAGACTATCTTCAGTAGACAGCTAAGAGGAGCATCTCTTCCACACTGGGCAGAGCTTGTGCACTAGGAACC
CTCAAAGCCACCCCCCCCCCAAACAATGACACACTTCCTCGAACAAGGCTACACCTATTCCATCAAAGCCACACCTCCTA
ATAGTGCCACTTCCCATGGGCCAAACACATTCAAACCACCATACACAGGTTTAAGTATACAGAACGTCTTTATAAGCTGG
TCGGGGACTTCACTAAGTGTTCAGCATCAGTGGCCCCAAGCCTTTCTCAGGATGATCTCTTAATTACAAAGCCATATCC
TGGTCTGACATACTTCCGTTAACAAGGACAGTTAGCCAGAAGCGGAGCTACAGAAGCCAAAAAGCAAGGTCAGTACATTT
TAAGGACTTTCCAGAACTCTGGACTTTGTTGGATTAGGATATTGATTTTGATTTTGCCAGTGGTGGTGTCTCTGTGCTGA
GTTTTGCAGCCTGAATGGTATTTCCATCATGGCGACAGTTGTGCTAAGGTCTGGGGGCCTGGAATATTCCCCACAGGTCT
TAGTGAATGCGTCAAATCATGGGCTCATCCTGTTCTATAGAGGTATAGTTGGTCTTAAGGACTATCTATCAACTTGACTC
AACTGATACATAATTGAACATACAGTTTAGGATGCAGGACCCACCATTAATCCAATCAGAATGAGAATTAAAGACCCCAG
GGCTGATAGCAGAGTTGTTAGTCATGGGAACAGGAGAGCAGAGACTGGTACCAATTTTCTGATTTACGTTTTTGTTTCT
TTCCCTTTTTGAGGTTTTTCCCCACCATAACAAGACTTTCTCTAAGTACTTCTGATCACTTAGCATAGAAACAACAGGAT
TTCCCTAAAGCCAGACATAATCCCCCTTATCGCACAAGGAGTAAGTCTAAACCTCTGGATTTTGTAGGACCCCATCTGTA
AGGAAGTCTAACTGTTATTTTCATTCAGAAATGGAAACTTTTATTTTGTCTTGTTTTTCAAGACAGGGTCTTCTGTGTAG
CCCTGGCTGTAGTGGAATTCACTCTGTAGACCAGGCTGGCCTCGAACTCAGAGAGATCCACCTGCCTCTGCCTCCTGGAT
GCTAGGGTTAGAGGCATGTGCCACCACTACCAGGACAGGAAAATGGAAACTTTTTTTTTTTTTTTTTTTTTTTGGGTT
TTCGAGACAGGGTTTCTCTGTGTAGCCCTGGCTGTCCTGGAACTCACTCTGTAGACCAGGCTGGCCTCGAACTCAGAAAT
CCACCTGCCTCTGCCTCCCGAGTGCTGGGATTAAAGGCATGCACCACCATACCTGGCAGAAACTTTTAAGGTCCTGATCA
ACCTGTCTACTTAGTTTCTTAAACTTTTTATCTCCCATGATAAAAGCCAATCTGCCCATGATAGCTGAACTGGCCTCTCC
TGCTCCAACCAGAAGAGGGAAGGGGCGGGGAGAGGGATGGAGAGGTTTTTTCCCTTTGTGGTCCAGGGCGCGAGCAAACA
TGGTTTTCCCTTCTTCTCCATCATATAGGTACACCTGGAGCACAGTGTGTACTGTGATGCATAACAGATGGCTCTCAGTG
TGTGTAGAAGATGCACATTTAGTCAAAACATTAGGGCTTGCCCACCGAGGAATCTCAGCGGCTCGGTAGCATTGCTGTTC
TCCAGACGAGCTAACCCACAAGGCAGACTAGTGGGCATCAGAACAGGGGAAGGGGCGTTGGTAGCTCTGGTGAGTGAAGG
GCTCCCAGCACTTTATTAAACTGCTGTAACCTAAAAAGTTGTGTTCAGTGAGGTTATTGGTGGGACTCTAGGAGGATGCC

```
AGGTGGGACAGAATCACATGTGAATCAAGACAGCAAGCCAGATAGTCAGACACCAGCAGAAGAATCATGTCCATAGCCTT
ATCTACATCTATGTCTATGTGTATACACATACAAACATACATGTGTGTATACACATGTATATGTGTGTGTGTGGTCTTAG
GGTTTTACTGCTGTGAACAGACACCATGACCAAGGCAACTCTTATAAGGACAACATTTAATTGGGCTGCCTTACAGGTTC
AGACGTTCAGTTCATTATCATCAAGTTAGGAACATGGAAGCATCCAGGCAGACAAGGTGCAGGAGGAGCTGAGAGTTCTA
CATCTTCATCTGAAGGCTGCTAGAAGAATACTGGCTTCCAGGCAGCTAGAATGAGGGTCTTAAAGCCCACACCCACAGTG
ACACACCTACTCCAATAGGGCCACACCTTCTAACTGTACCACTCCCTGGGCCAAGCATATACAAACCATCATATATAAGT
ATATGTACATATGAGGAGAGAAGAGTTATGTATGGACTCGAGAATCTGGCCTTAGAAAGGCAGGTAGACCAACGAACCCC
TCAAATCCCTCACAAAGACTGCACTAGGGCTGGAGAGATGGCTCAGCGGTTAAGAGCACTGACTGCTCTTCCAGAGGTCC
TGAGTTCAAATCCCAGCAACCACATGGTGGTTCACATCCATCCGTAATGAAATCTGGCACCCTCTTCTGGAGTGTCTGAA
GACAGCTACAGTGTACTTACATACAATAAATAAATAAATCTTTAAAAAAAAAAAAAGACTGCACTGGCAGCGCAGGGGAG
TCTAGGAAGAAAAGCATATATCTATTTTTTGTTTGGTTGTTTGGTTATTTTTGGTTTGGTTTGGTTTTTGGGTTGTTTTG
TTTTAGGGTTTTTTTTTGTTGTTGTTTTTGTTTTGTTCTGTGTGTGTGTGGTTTTTGTTTGGTTTGTTTTTTGTTTTTTT
TCAGACAGGGTTTCTCTGTGTAGCCTTGGCTGTCCTGGAACTCACTCTGTAAACCAGGCTGGCCTCGAACTCAGAAATCC
ACCTGCCTCTGCCTCCCGAGTGCTGGGATTAAAGGCGTGCGCCAAAATTGCTCAACTTGAAAGACAACTTTATATAAGAT
GTCATGTTGGGACCAGCGAGATGGCTCAGAGGGTAAAGGTCCTTGCCCCTAAGCATAAATAAGGGCCTGGGTTCAATTCC
TGAGACCCAAATTCTGGAAGGAGAGAGCTGACTCCCCACCAGTTGGCCTTTGACCACCACCCATGTGGTGTGGCAAGTAT
AGGTACACACACACACACACACACAAATACATGTTGTTAAAAAAAAAAAAAAGGAATGTGTTTTTCAAATGCTTCAGC
CACCACAGCAGAAAGCCTGGTAGGCCAGCAGGCTGGGGCTGACAGGATGCTGGGCACACTGGTAACCATCTCAGGTAGAT
GCCAAGATGGTTGCTGCATTCCAACTCCTACCTAGAGGGTTGTCCTGTAACTCAGAAGTCAGCCTGCCTCTGCATCCTGA
GTGTAAAGGTGTGGGCTACCACACCTGGATATCTGCCTTTGGTATTTATATTGTTTTATATTGTTTTACAGGCCCCTTAC
TGGCCTGCCAGTCACGATTCATCCAGCCCCAGGGATCCTCCTGTCCCCACTTCTCTGATGCAGGGCTTTCAAGCACACAC
CGCAGTACATGGCTTTTTTATGTGGGTCCTGGGGCTCGAACTCAGGTCCTGAAGCTTGTAAAGGAACACTTTACCTCTCC
AGTCAGACTGTGTAAGCACACGCAGGCGCGCGCAGTTCCCAAAGAGACCTCAAGAGGGCGCCAGATCTCCTGGAACTGAA
ATTAGTGACAGACTAAAAGAGCAGCTAGTGCTCTTAGGTGCTGAACTATCTTTCCATCTTTTCTGCAAACAGTTTCTAGA
TCAGTGGTTCTCAACCTGTGGGTTGAGACCCCTTAGGGAGCTAAATATCAGATATCCTGCATATCAGATATTTACATTAC
AATTCACAACAGTAGCAAAATTACAGTTATGAAGTAGCAATGAAAGTGATTTATGGTTGGGGATGTGGCCACAATGTGA
GAAACAGTATTAAATGGTCCCAAGCTATAAGAAGGTTGAGACCACAGCTCCGGATTAAGATAAAATTGCTGCATTGTCT
ACACCCACTTGGTGGCAGACAGAAAAATGACAAAACTATAACAAGAAGGATTGATGGAGGGATGACTGTAGACATCACTA
CTAGGTCTTCCAGGTATGGCTCGATTGGAGGGCTGGGCCTTGGGCAAAGGGCTGGGCCAAAGTTAAAGTCAGTGACTATA
GGTTGGTGATTGCTGCTACTGCAGGCATGCACAGGAGTGGGCAATGTGGAGTCTCATTTGTCAGAGGACCAATTAGAGAG
GCAGAATGGGGGGGCTATTTGCTGCCAAGTGAATAAGGAAAGGTGCCCAGGGCTCCAGGCAGGGCTGCCCACCCTCAGTGG
GGTGTGGTGATTATAGTCCTGGCATAAAAAGCCACAATTCCTGATTGCCTCAAATCAGGGACTGAAGGGCTCTGCCCATC
ATCTGCCACCTCATGACAGAGACAGGCAAGCACACTGCCCTCCACCCCCAACTGTAGGTGCTCCTTTCCTGCTGGACCAG
AAACTGCCCAGCGCCAGCCTCCTCTGGTCAGTAGATGGTCTCTTTCCAATAGAACCTTCTGGAATGAGTCTGGAAATATT
ACAGGCTTTCCCAGTGCCTCAGTGACCCAGATTTCACAGTTTCCATGATGCACTTGGGTGTTTAGCCCCACTCCCACCTC
CTTCCTTTGCATGAGCTGGGAAGCCTGGGGCACAGGCCTGATTCCAACTCTCTCACAATAGGTCCCTTCCTCCAGGGAGC
CCTCCCTTCCCCTTAGGTTAGTCAGCGGCTCTCCTAGTCTCCGTTGATTTTCAACTGTCCTCTGATCACAGCCTTGTCTG
GGGGTGGGTCTGAAAGGGGGCTGTGATACACAGAGGGCTGAAGGAAAGGAGAGCACTTCTCTTTTCTTTTTTGATTTATT
TATTTATTATATGTAAGTACACTGTAGCTGTCTTCAGACACTCCAGAAGAGGGCATCAGATTTCGTTAGGATAGTTGTGA
GCCACCATGTGGTTGCTGAGATTTGAACTCAGGACCTATGGAAGAGCAGTAGGCACTCTTAACCACTGAGCCATCTCATC
AGCCCCCAGGAGAGCACTTCTTAAGCCCCATCGGGTCACAAGCTGGGCAAGGGTCACGGGATTTAGTAGATGAATACTTA
GAAACAAACAAACAAACAAACAAAGCACGTTTCAGAGAATGAAGGGGTTGCCGCCAAGCCTGGTAACCTGAGTTAGATTC
CTGAGACCCATGGTGGCGAGAAGCACCGCCCTCCATGTTAAAAGAAAATGTTAGCCAGGCGTGGTGGCGCACGCCTTTAA
TCCCAGCACTCGGGAGGCAGAGGCAGGCGGATTTCTGAGTTCGAGGCCAGCCTGGTCTACAAAGTGAGCTCCAGGACAGC
CAGAGCTATAAAGAGAAACCCTGTCTCTTTTTTTTAAAAAAAAAAAAAAAGAAGAAGAAAAAAAGAAAGAAAGAAAAGAA
AAGAAAATGTTAAGTTCTTTAGAGTGAACTTGCCCCAGATTTTAAAACAAAAACAAAAAACCCTCTGCTACTCAGTGACT
TTCACACTTCCCTGAACTTCTGTTTCTAGGGGAGGTAGTAGGGTGCGGTGGGGGGGGGGTGTTGCTCAAACAGAGGCT
GCCTCACTGGTTCCCCTTGGCCAGCCACGCTGGGTGGGAAGAAAATGGAGGTGCTGGGGAGGAGGTCCGGGAAGACTGGT
CCAACCAGTGTGCACGCTTCATCTCAGGGCATGGGTGGAGCCCTGAAAGGCTGGGAGACAGTGGAAGGGGATGATAGGGC
TCGGCAGCCTCCAAGCCTGGCTGGGTCCCGTGGCACCCGCTGGATGCCCCACTGAACTTCACTCTTAAGAGAGCAAGACC
CCAGCCTGTGGCTCTCAGTGCCCTTGCCATCTCTACAGGAAGCAAGATGGAGACCCTGGACCCCACCGGGCTGCTTCTCA
AAGGGCCTCCTGTTCTCAGGTAAGGAGGAAACAGCCTCAGAGGGCTGAGGGACAAGATGAGGCAGGGTAGTGACATCACA
GCTCCATCCCTATTTCATCTATGGCATCTTCTAGAATGTCAGGACTATGAGGGTAGGAATTAGTGTTTGTTTTCCCAGGG
GGTTCTGGGGAGTGAGCGAAGGAGGGAGGGAAGGGACTATAGAGACTGAGCCAGGACTGGTGAGTCACACATAGACTCGC
AGAGTTTGTAAGGTAGAAGGACATCCTGAACTACATAGGCAGTTCAAGGCTAGCCTGGTCTACATGAGACCTTGTTTCAA
ATAAAAAGCAAAATCTAGGAACAAGCCAGCCATAGTGGTGTATATCTGTAATGGCAGCACTTTGGAGGCTGAGGCAGAA
GGATCACAAGTTGGAGCTGGACTGCATAATGAGTTCCAGGCAACCCTGACTCAAAAGAAGAAGGAAAATGAGGAGGGAGG
AGGAGGGGGGAGGGGGGGAAGAAGGAGGAGGAAGAGGAAGAGGAGGGAGAGGAGAAGGAGAAGGAGGAGAAGAAACCAGGAGATGGTG
GCACATGAGGAGGAGGAGGAAGAGGAGAAAGAAGAGGAGGAGGAGGAGAAGGAGAAGGAGGAGAAGAAACCAGGAGATGG
TGACACATGACTTTGATCCCAGCACTCAGGAGACAGAGGCAAATGAATCTCTAAGGCCAGCCTGGTCTACAGAGTGAGT
TCCAGAGCTACACAGAGAAACCCTGTCTTGAAGAATGAGGAGGAAGAATAGGAAGAGGAGGGAGAAGACAAACCTAGATGA
```

```
TGTAATTGGTCTATGATGAAATGTATTTACTTATTCATCCTTTAAATATTGGTTGAATAAGTAAATTCAGAGGGTCCAAG
TGTTTAGGAACAGACCACATAGCATCTCAGGACAGAAAACCCTTCTGCCACTGGCGTGGCAGATCTGTGGCATCTGTGCC
TATATTCCTACTCCCAGGGCAGACATCACTAATCAATCTCATTGTCATTTCCAATGTGCAGCCCTTATGGTCTTTGCTGC
GGTTGAGAGTCTGTAAAGAGGAAGGTTTTGTTACCTCTGCCCAAAACTCGACCTCAGGCCTCCTCCATACTTTTCCACAG
CCTTGTTCCTGGCCCTTGAGCTGCCCCAAGGCTCACAGGCAGCCCTCCACATCGAGATGATCCCAGAGCAGCCTCAAAAG
AACCAGGACCTTCTTCTGTCCCTTCACGGTGTTCCCAGCACTGCTCAGGATTTCATCTGGTACCTGGGGGAGGAGGCTTC
TGGAGGCACAAGGCTGTTCTCTTATATCCCCGGACTACCACGGCCCCAGAGGGATGGCAGTGCCATGGGACAGCGAGACA
TTGTTGGCTTCCCAAATGGTTCCATGCTGCTTCGAAATGCCCAACCTTCAGATAGTGGCACCTATCAGGTGGCCGCCACC
ATGAACCCTGCCTGGACAATGAAGGCCAAGATTAACGTCCATGTAGCTGGTAAGTGGGACAGCTGTGATGGCAGGGAGAC
CAAGTGCAAAGACCATGCCATTTAGCCTCCTCATTCATTATATACAGTGTGCATTGATTGAGCATCTGCTGAGTACCAGA
CCATGGATCTCCCAGACCCTAAGTATATACCCCATTTCATTAGTTCTCACCATACTCTGATTGTTCCCATATTCACTGAGC
ATCTTTTATATGCCTGGTCCTGATGAATTGTTCAATTTTCTTGATCACAATTCAAGTATTTGTTGATGTAGCCACACCAG
ATGAAGAGCTAGGGGACAGGGTGGGGAACAAAGGTGACATCTAACATTTCTTGGGAATCTTACCAGGCCAAGTATGAATC
TAAATACTTTCCCTAGATCACACTGATCATTAGGACTGGCTAATAGCTTCGAGTAGAGAGCTGTGCATCTCATAGAAGCT
TTACAGCCTCTCTGGTCTTATAAGACATTACTAGTGCTTGCTACTCACAAGAATCACATGCTAGGTTTTGACAAGTGTCC
CCCCAGAACCAGGGTCACCTCTGAAGGAGGACCACAACAATATAGTAAGTACTTTGCACTTAAAGAAGCTTGCTGAGGTG
GTCGCCATCGTTATCTCCATTCTATAGATAGGGAAACTGAGGCACACTGGGGTTATAATTTGCCTGGCTCAGAAAATTAA
ACAGGCTAACATGACACAGGAGAGGAAGTCAGAGAGGTCTCGCTGAAGATGACCTGTAGTTAGGTTTGGTGGGATATAAG
TGTCATCCGCCACTGTGTAGACTGAGGAAGGGTAGTTGGGAATTCTCAGGCAGCCTAAGCCACATAATGAGATCCTGGGC
TACTGAACAGGACCATGTTTCAAAAATGTCAACAGGATCAGTAAGCTAGCCCAAGGGGTAAGGTGCTTGCCACCAAGCCT
GATGGTCCCCAGGACCTCAAGGTAGATGGAGAGAGCCAACTCCCAAAGGTTGTCCTCTGACCTCCACTTGCTGGTGCAGA
CACATATGTCAATAAATAAATAAACTAATAAGAAAAACAGACAAACAAGCAAATGCCAGGTGGTAGTGGTGCTTGCTTTT
AATCCCAGCACTCAGGAAGCAGAGGCAGCAGATCTGTGAGTTCAAGGACAGGCTGTTCCACAGAGTGAGATCCAGAACAA
CCAGAGCTACTCAGAGAAACCCTGTCTAAAAACACCAAGGAAAGGAAAAACAAAGAGAAGAAAAAAGAAAGAAAGAAGA
AAGATCACACGCCCCAGCCCCTCACTGGGGGATTCTAGGCAGGGGCTCTCCCACTGAGCCACGCCCCAGCCCCTCTCTG
GGGGATTCTAGGCAGGGGCTCTACCACTGAGCCACGCCCCAGCCCCTCACTGGGGGATTCTAGGCAGGGGCTCTCCCAC
TGAGCCACGCCCCAGCCCCTCACTGGGGGATTCTAGGCAGGGGCTCTACCACTGAGCCACACCCCCAGCCCCTCACTAG
GGGATTCTAGGCAGGGGCTCTACCGCTGAGCCACGCCCCAGCTCCCTTAGCTGCTGCTAACTCTGTCCTTGTCCCCATG
CAGTGTCACTGATGTGCCATTTTTTGATTACAAGTCAGTCAGTGCCCCACATTAAAAGAAGAGGGCTAAGGTAAGTGATG
GTGGCGCACACCTTTAATCCCAGCACTCTGGAGGCAGAGGCAGGGGGATCTCTGTGAGTTTGAGCCCGTCTACAGAGTGA
GTTCCAGGACAGCCAGGGCTACACAGAGAAACCCTGTCTGGAAAAATAAAAACAAACAGAAAACAAAGAAGAGGGCTCAG
ACCCTCAAGCTTGTTAGAGTAACAACTCAGCACAGGATGGGGTGTGGGAGACACTGTTTCCTTTTGAGCTCTAGAAATCA
AAATGTTTCACCTACACTGATAAATTCACTGTTTATGCTCAGGAAGAAATAGTAACATGTTACCAGAGGGAGACAGTTCA
CCTGAGGAGACTGCTTCTGTCTGGTGGACAGCGATGAGGAAGTGATGAGGGACTTGGAATACTTTAAACACCATGGCCCA
GGACTATGAGCCCTGCCTGGGTTGAGATTCTAGCGCAGCCTGATGGCGGAGGCAGGAGGATAGAAAGTCCAAAGCAAGTC
TGGGCAATTTATTGAAATCCTGCTTTTTTTTGTTTTTGTTTTTGTTTTTTTGGTTTTTCGAGACAGGGTTTCTCTGTGT
AGCCCTGGCTGTCCTGGAACTCACTCTGTAGACCAGGCTGGCCTCGAACTCAGAAATCCACCTGCCTCTGCCTCCCAAGT
GCTGGGATTAAAGGCGTGCGCCACCACGCCCCTCAAAATTACAGAAAAGAGGAGGGGAGGAAGGGAAAATGGGAAAAGGA
AAGGGAAAGCATTGATTTTAAAAAATGATTTACAAGGCTCTCAGATTGCTCACAGATAAGAAGACCACTGGCTGCTCTTC
CAGAAGAGCCGGGTTTGGTTCCTAGCACCCACACGGCAACTCACAACCACCTAGAGTTCCAATGCCCTTCTTGCCTCTGT
AAATACATGACATGCACATGGTACGCAGACACACATGCTGGCAAAACACTCAGACACATACAATAAGATTTTTAAAAATT
CACATAGGCTGTACATGTGGCTCAGTTGGCAGAGTGCATGCCTGGCATACACGAAGCCCTGGGTTAAATCCCTAGCAACT
TATAAACTGCACATGGTGAACACCTGCATTCCCGGGTCTGGGTCAGTCTCAGAGGCAGAAGTTCCCCTGAGATTTGCTGG
CGACCCAGCCTAGCCAAGGGGACACTCCAGACTCAGTGAGAGACACTGTCTCAAACACCAACATAGACACTGATTGAAGA
AGACACTTGACACCCACTTCTGGCCTTATCTACATGCACACACACGCATACACACACACACAGACACACACACTCATG
TGCAGGAATTTGAGCATCCTTAGCTATATAGTTCAAGGTCATCCTTAGCTACACAGTAAATTTGAGGCCTGCCTTGACTA
TAAGAGACCCTATCTCAAAATAAAAATATTTTTTTAAAAACTGATAACATGAGTGGGCAGAGTGAGGGGCTGTGGGCGTG
GCTCAGTGGGAGAGCCCCTGCCTAGAATCCCCCAGTGAGGGGCTGTGGGTGTGGCTCAGTGGTAGAGCCCCCCTGCCTAG
AATCCCCCAGTGAGGGGCTGGGGGCGTGGCTCAGTGGTAGAGCCCCTGCCTAGAATCCCCCAGTGAGGGGCTGGGGGCGT
GGCTCAGTGGTAGAATGGATGCCTAGCCTGCTTAGCATGGCACTGCAAAGTAAGGAATTAGGTGAGGGCAGACAGATGTC
TTCCCTGAGACAACCATCACTCCTAGGTGCACGCGAGGTCTCCTCACTCCTCCCTGTCCATGCCGGGATCACGGTAGCCA
TCATCATTGGGAGTCTTGCTATCGGGTCACTACTTGTATGTGGCATTGCCTATGTCCTGGTCACTCGAAGCCGGAAGGGC
CGGAGCCCCAGGTACAGTTCCCAGAACTCCCTCTGTGTCCTCTAACTCCTCTTTCCCCTCTAACTCTCCTGTCTTTCCCA
GGCTGGGTGAAAATCAAGTTAATAGGTAGGAATCAGGGAGACAGAGTCTCTAGGATGTCGGGGTCCCTCTCCTCCACCCT
AGCATCTCAGGGTTCTTCTATGTACCCCTAGGAATCCAGCCACAGAGAAGCCAGAAGTGAACCTGAGTCCTGAATCTGGT
AAGCCAGGAGAAAGTGCCTTTGCACCCAGCCCTTCCGTGTCTCCCCCAGTTAAAGGCCTCCATGCCCACATGCAGTCAGA
GTCCTTGGGGACCTTTGGGCACACTGGGAGCTCCGAGAGATTTAAGGCCCCTCCTAACCCTCTAGGGTTCCTTCTCTTA
ACAAGTCTTCTGCTAATAAGCCTTCCATAGCTCCCCCAGTGCCTTGGGACAGACTTCTGTCTCCCTAGCATGGCTCTCAG
GTCCCTCATTATCTGCAGACAGTGGGCTCTCTTGCCAAGCTCCTGCAGGCTTCCCTCCTGCCTCTGGCCAGTTCTCACTT
CCCACTGTCTCAGATGAACCCTCTTCCAGGTAGTCCTCCCTGATATCCAGCCTGGCCAGGACTGTTTCTGTTTCCACATC
CCCTATTCCCCATCCCCCATCCCCAGCCTTAGACTCTCTGGATTGTCACTCTCTGGGGATGGGTCTCTTTTCTCCCAAGG
```

```
GACTTTGGGTTTAAGAAGGGTGTGTGGCATGGATTGTCTGGGTCACCACTGTGTCCCCAGCACATATCTGGCAGGAGGAGAA
ATTTGCACAAATGTTAGTTGAGCAACTGAGTGAGCGAAATGGACAGGCAGGTAGAACGGGTTCTACAGTGGTGAGTCATC
TGCCCATGGGACCCCCTTCCTCCTCTACTTCAGGTGACAGCAACATCTATGAAGTGATGCCATCTCCAGTCTTCCTGGTG
TCTCCCATCAGTGACATGGAGCAAGGAAGCCCACCCATGGTGAGTCCTGTCCCCTTAGATCTTGCCTCTCGAAAAACCTC
TACCCGGAGTTAGCTATGGTGCCTGGGCACCTGGAAGCCATGACTCTTCATTTCTCTCTCCAGTCAACTCATTCTGTCTT
CCTCTCCTTCTTTTGCATATGTGGGGGTGGAGGGAGGAACACGCACCATGACATGCATGCGGATGTAGAGGACAACTTGT
GGGAGTCAGTTCTCTCCTTCCATCATGTGAGTGCTTGGATTTGAACTCATATCATCAGGGTTAGCGACAAGCACCTTGCC
CCCACTGAGCCATCTTGCCAGCACCTCTCTCCAATTCTTGTGTGTCTCATCACCTGCGATAGCCCAACGACAACTAAAGC
AAGGATTCCATCGAAGTCCAGTGGTGAACCAGTGAGTTTTGTAGGGTCACTTACAGGACCGTGGCAGCCACACCCAGTAC
TGGTGAGGGCTCATGGAAGCTACACGATGTGCAGGCAGAGACACCAAAGAATCACAACGTCCCCATCAACTATTATCCCA
GAGTGTGGGCACTGGAGGCAGGAGAGTCAGGAGCTTAAAGTCAGCTTCCAAGTTTGAGGCCAAGCCAGAAGGGGATAGCA
GAGCCAGGGACAGGCAGGATTCCGGTCCAGTTGGCAGTGAGTGCTGAAGGGGTGGGAGAAGAGAGAGGCCTTCTTCGGGA
ATCTTAGTGCGTCTCAGCTTGGCTCATTTCAGTGAAGGCCTTCCCTGTGGGGATCTTTGACGATGCATCTCTGTGAGACC
ATCTTTGCTTGTTCATGTTTCATTTTGTTCTTTAAAATCACTTACTTCTTGTATGAGGGTTCTGCTGCATGTACACCCGC
ATACCAGAAGAGGGCATCAGATCCCCTGAGAGACCCACCATGTGGTAGCTGGGATTTGAACTCAGGACCTCTGGAAGAGC
AGCCAGTGCTCTTCAATGCTGAATCATCGCTTCCAGCCCCTTGTTCGTGTTCCGTTATTGGGGAGGATGTAGATGCTTTA
TTCGGGGTGAACCAGGGATTATATAATTTTGGGGAAGGGCATAAGAGTACCCAGGAAGGGAAGGCTCTGGCTGAAGGCTA
CGGGGATTGAGATACTCTGTGGTATAGAGGAGGAAGAACTCCAGGTGCTGAGCTATGTGACTGAATACCCATGGTCCTGG
CAGTTGGTGGCCATGGTTACATGCTGCAGAGCAGGCATTGGAGGCTGGAGTGTGGCAGCGCCACTCCTGCCAGCTCAGAT
GTCTGAGATTCCCACCTCAGCAGTTCTTATGCCCATCTGGTAGCGCTGTCCAAGTGCTTGACAAGCCTGGACTAGATTGA
GACCCTGCCTCAAAACAAAAACAAAAACCAACAACAACGAAAGGAGGTGGAGGGAGTTGGCTGGAACATGGCTCAGCATG
TTCTTGCTACTTTTAAGGACCCAAATTTGGATCCCAGTACCCTCATTGGCAGCTCACAACTACCTATAATTCTAGCTTCA
GAGGACCTGATGCCCTGTACCAGACCTCAGTGGGCAGTCACGTGCACGTGCTCACATATCATGCACACACATACTCAC
ACACATACACGCAGAGACACACACACTCACACACAGACACACATAGCACAGACACATACCCATACACCATGCACAAACAT
ACAGACAGACAGACAGACATAGCACAGACACATACACACATACTCACACACATAAACAAACATACTCACAAACACACACA
CCAGACATACATACACACATATACACCACACACAGACAGACACACATACAGACAAACACATAAACATACACTCACACA
CATAGACATACACACTCAAAAATACACACACACCACATATACACAAACATACACACAGACATACAGGCATCCATACAGAT
ACATGCACACATACACACACAATCACAGACACAAATACAGACACATACACACATATGCATACTCTCACACAGCACAGACA
CACCATACCATACACACAGAGACATACAGACACACAGACATACAAATACATGCACACATACACACACACACTCACACA
CATAATCACACACACAGCACAGACACAAACAGACACACACCTATACACACAGATAGCACACACATACACATACCAT
AGACACACAGCACACACACACACACACACACACACACACCAATAAAAATTAGAAGAAATCTTTAACGGATCA
GGAAAACAAGGAGCCAGACAATGGCTGAGCAGGCACTTGCTCCTCTAACCTGGGCCCTGAGCTCCATTCCTGGGACCCAC
ATAAAAGTCAAAGAGAACTGATCCCCAAACTCACCCTCAGACGTCTGTATGTGTGCACCCTACATACAATTATTGACGTT
GATGATGATTTGTTTTGTGTTTTCAAGACAGGGGTTCTCTGTATAGCCCTGGCTATCCTAGAACTCACTCTGTAGAACAG
GCTGGCCTCGAACTCAGAAATCTGCCTGCCTCTGCCTCCCAAGTGCTGGGATTAAAGGCATGCGCCACCACCCCTGGTAA
TAATAATATTTGTAAAAGACTGACAGAGACAGAGAGAGAATGTAAAAACAACAAAGGCAATTTGCTTAACCCTCGTGTTG
AGGAGAAGCTAGGCTTCTGAACAGGCCAGAAGCCCTCACGGTCGGGCCCATTGCCTAAGATGTGTGACACATGCTAAGTG
GCCTCATAACCAGAGGGTCAGTGGGGACTCTGAATCTGGGCCTGTGCGTCATACATAACACCAGGATCAAAGACTGGAGG
GTGTGAGGAACCCTCTCACCTGTACCTCTCCCCTGCAGAGTCAGCCTCCACCACCTCCACCAGCATCTCAGGAGCCAGAG
CCAGAGCACCAGCACTATCAGGTATGGATTGCTTACCCCCCACCCCACAAGAACCCATGGAGCCATGAGCTAGAAGGTC
AGATGGGCAGGGACATGGACTGTCCTCTCCTCTCTCCCTTCAAGAAAGCTCCCCAGTGAAACCTGCTTCAGCTTCCTTGA
GACTGAACCACTGGACCTGGCCTTGCCTTCCTGGGCCTGGGCTCCTTCTCTGTAGAAATGATCGAAAGCTTCAAATCTAT
ACAGGATGAGCAAGGTCCAGAGAGGGAAAAGAGATTGCGTAGGGTCACACAGCAGAGCAGGTCAGGCCTTTATGCAATTC
CACTACATGAAGGGACCCATTTCTGGATTGGGTATCAGGTCTCTCCAATCTTCTCCTGGGTCCCTCTCCCTCTCCCCTCT
CCCCCTCCCTCTCCCCCTCCCTCTTCCTTTCCCTCTTGGTTTATTAGTATCTCGTCCTCTCCTCTTTCCTGTCTGGCCTT
GCCCCTGTTATTTTGGGTACACCTCTCCTGCTCCCTAGTACCCCCTAACTCTGGGGCTCACCCTCTTTCAGTCTCCTGGT
TCTATTCTCTGTCATATCCCCAACCCCACTCTGGGCCACCACCGCTGTCTCTCTTCCACCGCCACCTCTCCTGCCCCTCA
TAGTTCCTGTTGCCCTCTTCCTAGGACCTACTAAACCCTGACCCAGCCCCATACTGCCAGCTGGTGCCAACTTCCTGATG
GACCCTCGGCCCAGCCTGCACAGAAGACAGAATCTCAACTTCCAGACCCCACCCCTCAGGGCTTCACACCTATAGAAAGG
ACACATGGGACACCCACAGGACAACGCTGCAGGGAAACTGGCAAGATGGGCTCTGCCATAGACTCAGCATGACATGGAGT
CAGTGCCTCTCGGGATGAGAGACTGACAACCCCAGATCCCCAATGTCCCAACAACCTTCAGTCTGTGCCTCTTGGGCCAC
CTGCCTCTAAGAAAGCAGGCTCCTGCCCCACCCAAGGCCAGCGGCCCCATCCAGGTATTTGAGTGATGGGAAGAAGGCCT
TGGTACTCCACCATAAAATTCCATCCAGCTTTAACCAGTTACCCAGAAGCCCTTTCCTTGGGAAGAAAGCACTACCAGGA
AGGCCGCACAACCACCGGAGGTTCTCAACCTGTGGGTCAAACCTCTTCAGAGTCAAATAATTGTTTCATAGGGTTTGC
CTAAGACCATCAGAAATGCCAGATATGTAGATTATGGCTCATAACAGTAGCAAAATTACAGTTGTGAAGTAGCAACAAA
TTATTTTTATGGTCGGAGGTTGTGGTGACAACTTGAGGAACTGTATTAAGGGTCTCAGCAATACAAAGGTTGAGAGCCAC
TGCTCATAGAGCCAAACACCTGTCAGGAAGATAACAGCCTTGGGCAAATTAACTGCCCGACCCCTGACCTGCTTCAAAAT
CAGCCACACAGAGGACTGGGACTGTACAGCACAGCTGTAGAGCGCCTGCCCAGAGCCCACCCGTGAGGGACTGAGAACAT
GGGGGCGCGGCTCTGCAGATGGGCTGCTTGCGGAACTGGAGAGAGGCCTGGGTTTAATCCTCAACCGCAGGGGGACAAAA
CAGTAGTTAGTCCTCAGCCACTGGGAAGACGTGACACGTGACACGCCTGGAGGAAGTCTCTGGAAGCTTGAAAGCTCAAG
AAACTGCCCCACCCTGTGGCCTCTGGGCCCAAGGATCAGTGACGGCCACTTTGGACTCACGGCGTTTGATTGTAAGATGA
```

```
TAAACTGTAGTTGTTTTAAGCCACTCTTTGATAGCAGGTTGTTCCAGCAAACAGGAAATGGGGATGCTGAAGGATCCCAG
GGAGGGGACGTTACAACCACAGCTGATTGGCAATATGCAAATGAGCCCCTCGGAGGGACTTCCCCAGGCTTTGATCTCAT
ACAACCAGTGCATGACTAAGCTGGCCATCCAGATCAACCATGTGACCTGAGGGCCAGAGGAGACAGAAGGAGTGAATAAA
CCAGGGCTTAATTATGTCTTTTTAAAAGTTCTTCTTTTGAGACAAAGTCTCTCTGTGTAGCCCTGGCTGTCCTGCAATTT
GCTTTGTAGACCAGGCTGACCTCAAACTCACAGAGATCCACCTGCCTCTGCCTCTTGGTTGCTGAGTTAAAGGCGTGTGC
TACCAGCAGCCAGCCAAATTTATAATTTAAAAAAAAAAATTTATTTGAGTGTGTGTCTGCATGTGCGTGTGCGTGCGTGC
GTGCGTGCATGCGTGCGTGCATCTGTGTCTGTGTCTGTGTCTGTGTGTGTTTTCAAGTTCGGTTAGATGATGTT
CACACTGTGCTCTTTCTCCCTCTTCTGGCCTTAGGTGGAATTGCATTTCCACAGCCCCTAATTAGAATGCAAAAAGCTCT
GATTAATGAAATGTGGGTGCAAGCAGTGCCCAGTAGTCCAGGTGCGCCATCACTTTCCCTCCAGACTCCAGTCACAGTGA
CTAGCCCTGTCAGTCAGGTTCCTGAATGAGCCCACCTAAAGCTGAGTTCTCCTGACCCTTTTCATAGCTGTCTACCTGAG
ACCGAAGTATCACAGGAGTGAGAAATATAAACCCTAAATGAGCCACGCTGTGAGTAAAATGGGGCTTCTTATTACTGCAC
CGTAGCCCAGCTCAATCTGACCACAGGTGAGGAGACCCAGCCTTCTCAACATAAAAACTACATTTCCCAGGAGTCCTTGC
AACTAGATCTGGCCACCTCGTGGTTAAGTTCAATATAATCAAAAATGGAAGTTGTATAAACAGTTCACAGAGAGGTCTTC
CCTGCAAGAAACAGAGCAAGTGAGAGAGTTGGGGTCCTGGGGATGCTGTAGACAGATTCACCATGCTAATTAGAGAGAGA
TCTGAGCAAAAACTCCCTAACTTAAGTGACTTCTCTGGGAGTCACAATTTTCCAGGCTCTTTCTAAACACGTTTAACCC
ACATCCAAGCAGCATTTTCCAGATAGGGCGTCTAAGTCTAACTGCTCCACTCTCACAGGACAGTCTGGAGAATGCTGCAC
TAGTCATAGTGTCCCTAATAAATGCATTCAAAATATATGCATTTATTAAGTATATAGCAAGCCAAAGTAAAAAGAATAAA
TAACAAGTCAGGCATGGAGGCTGTACTGCCTGGGTTTGTGTCAACTTGACATAGGCTGGAGCTACCACAGAGAAAGGAGC
CTTTCTTGAGGAAACGCCTCCATGAGATCCATTTTCTCAATTAGTGATCAAGGGGGCAGGGCCCATTGTGGGTGGTGGTG
CCATCCCTGGGCTGGTAGCCTTGGGTTCTATAAGAGAGCAGGCTGAACAAGCCAGGGGAAGCCAGCCATTAAGGAACATC
CCTCCATGGCCTCTGCATCAGCTCCTGCTTCCTGACCTGCTTGAGTTCCAGTCCTGACTTCCTTTGGTGATGAACAGCGG
TTTGGAGGTGTAATCTGAATAAACCCTTTCCTCCCCAACTTGCTTCTTGGTTTTGATGTTTTGTCCAGGAATAGAAACCC
TGACTAAAATAATAGTGCATACCTTTGATCCCAGCAATCAGGAGGCAGAGAGGCAGGCAGATCTCTGTGAGTTTGAGGCC
AGCTTGGTTGGTCTACAGAGCAAGTTCCAAGACAGCCGGGGCTACATAGAAAACCCTTGCCTTACAAAAACAAAAGATGA
ATAAAATTTTAAAGTAAATTAAAGATAATAAATAGCAAGCAAAAGGTAAATAGCAAAACTATCAAAGCAGATGCTCAC
AATATCCAGTGGGAACCTACCTACTGGGCATGGACATCAGGCGTCAGCAGTGGCCCCACTCAGCTTCTCTCCATGGAGGA
GCTCCTGGCTCCTCTCTGTGCAGACACCTTTATATCAGAGGACAAAGGATAACAGCCTCGGGCTGGAGAGTTGGCTCAGC
CATTAAGAGCACGTGCTTCTTTTCCTAGAGGACCTGAGTTCCATTCCCAGCACACCTGCCTGTAACTCCTGCCTGTAACT
CCTGCCTGTAACTCCTGCCTGTTACTCCTGTAGTCACAGACACATTCACACAATAAAAATAAAAGTAAAAGAAGAAGAAG
AAGAAGAAGAAGAAGAAGAAGAAGAAGAAGAAGAAGAAGAAGAGGAGGAGGAGGAGGAGGAGGAGGAGGAGGAGGAGGAG
GAGGAGGAGGAGGAGGAGGAGGAGGAGGAGGAGGAGGAGGAGTAAGACCTTGGAAATGACTTGTCTTCTAGCTGTCC
TCAAAGACATAATACTTTTTAAAAAAAAAGATTTACTTATTTATTTATTTGTTTAATGTATATGAGGACACTGTAGCTGT
GCAGATGGTTGTGAGCCATCATGTGGTTGCTGGGAATTGAGGACCTCTCACTCTGGCCCCACTTGCTCCAGCCCAAAGAT
TTATTTATTATTATATGTAAGTACACTGTAGCTGTCTTCAGGCACCCCAGAAGAGGGCATCAGATCTCATTACGGATGGT
TGTGAGCCACCATGTGGCTGTTGGGATTTGAATTCAGGACCTTCCGAAGAGCAGTCAGTGCTCTTAACCACAGAGCCATC
ACTCCAGCCTCAACATAATGCTTTTTTACTCTTGAGTTGTTTTGCTGTCTTTTCCTCCCTGCTACTGAGGTAGGGATGTT
TAGCCCTTCCCCAGACAAGGGGCCTTGGGGGACACTGAGACAAACATGCTCGGGTCTGACGTGGGGGGTGTTTAAGCTCC
AAGAGCTTAAACAGAGGAGTTCAATTCTCAGCACCTACACTGGGCCGTTCACAACTGCCTGTGACTCCAGCTTCAGAGGA
TCCAGCGCCCTCTTCTGGCCTCCTCAGGTCCTACACTCATGTGCACAACCCCCCTGCTCCCCACCCCACCCCACACATAC
ACATAAAAATAAGTCTCTAAGCCAGGCCTGGTGGCACACGCTTTTAATCCCAGAACTTGGGAGATGGAGAAACCCTGTCT
CAAAAAGATTGAAATAAATAAAATTTTAAAATCAGTTTAAAAATCAACAAAATCAATGACAAAATCCTGTATAATTTATT
AAATATTATGTATATAATATGCTGTATCACATAATATCATATTCTATATTATATATTTTGTGGGTAGGCCATGACCTCTG
ACCTCAGATTTTCAAGAGTCTTAGTCTATACATTGCCACCTTGTGACTGGAGACACTGGGGCTGGAGAGCCGATGTGGCT
GTCTCGAGGCAGCACGCTGGTGATGGGAAGGAAAGGTTGTTTGTTGTTGTTGTTGTTGTTGTTGTTGTTGTTGTTGTTGT
TGTTGTCAGGATGAGCTGGATTTTGCTGCAGTAACAATTAGCCCCGTGCTGTCCCCAGCTTGAAAAGCTTGTAAGATTTA
TTTTTTTTCCCCTCCTACAAGTTTGTCTCAGGTGGATAGGTGTGACAAGGGTCAGAAGAACTCAGCTGCATGTGGACATCT
TCAGAAATGAGGCTGACAAGGTTACTCACTGTAGCAGGGAAAAGCAGGGCACGTGGGTGTGCACCCAGCGATTTTTATAA
CACACGTTCCCCCTCCCCCCCAAAAGCTGGAAATAAGGCTTCCACTCACTTTCCATTGGCTGAATAAAGTCACATGGTAC
TGAGTTCTATAAAGACCGCAGTCTTGCAAATCTCCTCCGACCGGAAGTGGGAGACCTGGAGTACTGTATGCATCCTGGGA
ATTGAACCCTGATCCAGCTCCCCTGACAAAAATATCTAAGCAAGCCTGCCCTGCTTAAGTTATTTTTTCCATGGACTCCA
TTTATCCAGCAGACACTGAACCAGAGCATAGAGGCTAGGGGCAAACCGCAAGTGCCGATAGATGCGACGAGGGTAACCTT
GGCAGAGGCTGGTTGTAGATTGAAAAGGGAGGGTTAGAAAGCAGGAAGGGTGAAGAGAAACCCATCCATTCATGAGCTCC
ACACGTATTTATTGAGCACCTGCTGGATACAGCACTGTTTTAGGTCCCAGGAGACATCTATATAACCAAAACAGAGACCT
CTTCCTTGGGGGGCTGAGCTCCCCGCAAGGAGACTACGGGACAATCGAAGAGGTGCGGACCAAACCACAGAGGTGACATT
GTTGGGAAGGGGAGCAATGGAATAAATGTTCAGACCACTAAGATATTTGTGCTTCTGTGTCCATAAACTAAAAACAGGCC
AGCAAGATGCTCAGTGGGTAAAGGGCCTTGCGGCCAAGCCTCGTGGCCTGAGTTTTGCTCTCTGGTACCCACACATTGG
AAGGAGGGAACTGACTTCTACATACGACATACACACAATAATAAGTAAGTGGGTTTTTTGGTTTGGCTGGGAGATATATA
TCGGAAGGAGTGTGAAAAGCAGTCATTCTTGAATCCAGGAACCTGCAGATCCCACACAGTTCATTAAGTAGGTGTGCATG
CTGAACCAGCAGGAGGCAGCAAAGGCTAACACATAGCCCGCCTAGCACAAACTCGGCCACAACCTGATGCCCACCCCAAC
CCCCATCTCCCACCCCACTTTCCCCACCTTGTGAGTATTCACAGCCCACTCTACTGCCTCCTGTCAAAATACCCAACCTT
GATCAGCCTCTGCCTGCAGCAAGAACAAATGCTATCAGCCCCTGAACTTAGCTTTCCCAGTCCCCACAGCCCAGCTTAGA
```

```
CCAGGCAGCTCCCCTCCCCTCCACCTCAGCCTCTCCATGGCCTGTCTTTCTCCCCACGCAAAGCAGCCCAAGGAGCTTTG
TTCACTCCAAAAATCAACCCTTCCCCTCCCTGTCTGGAACAGGCCATTGCACCCAGTGCCTTTAAAGACAAACTTTTCAG
GCTGGGTGTGAGGTGCACCCCTTTAATCCCAGCACTGGAGGGGCAAGGGGGGAGCAGGAGCAGGTGGGTCTCTATGAGGT
CAAAGCCAATCTGATCTACATAGTGAGTTCCACGCCAGCCAAGACTACATAGAGAGACCCTGTCTCAAAAACCGAAACAA
ACAAGCAAACAACCTCCCCCCCCCCAAAAAAAAATAGCAGCAGTATGGTGGTACACACCTGTGATCCCAGTGGGGACTTCT
GGTCCCCCTTTAGTATTCTGTACCCCTTATCCCCACTTTGGGATCCACAGAACTGAACTGCCTTCTGTACCCCAAAACTC
AACCTGGGGCTCTCCTCTCCCTCTCTCTGGAAGAATGCAAACAAATACAAAAGAGAAGGACCCAAAGTGTAACCAAAGCA
GGTGTCAAGGAGGACGGGATTCCAGGCAACGCAGCCTGAGCTGGGTGTCCTTGACTGGAACTGGGCAGCAGCAGGGGTCC
CTGTCACCGGCCCCTGCCACTCCCAGGATACAGTCAGCTTTTAAAAGATAGTGGCTCCTTGTTTGACAAAAGAGGAAGTG
GGCCTGGGGAAGGAAGTAGGCTGGGCTCTGAGCCCAGAGTCAAAATGATTCAGGGGGGAAACCTTCTGTTCGGAAGCCAC
CTCTGTGGGCGAAAGGTCCTGTGCCCGCTTACAGGGTCACAGGATCATCCCGCATAGACTGGTGATCAGAAGTGTAGACG
CCTCTCCCAAAACAGCAGACTTCCATGAACTCCCAAGGGCACCAGGGACGGTGTGCAGGCCTCTGTAGGGTCACAGGGGT
GACCTCATCCTGCCCCTGTGGTTGGTGATGAGCTGAGATGGAGGTAGCCCTGGGCCAGGAAGTGAAGGCCAGAACTGAAA
GCAGCCGTCCACAGCACACCAGTCGGGAAGAGAGAAACCAGTGCAGAGTTAGTATTCAGAGACTTTCTGCTATCTGTCTG
TCTGTCTGTCTGTCTGCCTGTCTGCCTGGCTGTCTGTCTTGGAACTCATTCTGAATATCAGTCTGGCCTCAGACTCACAG
AGATCCTCCTGCCCCTGCCTCCCTTCCAGGTGCTGGGGTCACAGGCCAATTGGGCCGCCATGGTTGGCTTTCTTTGCTCT
TATACAATCCAGGGCCTCAGACCAGGGAATGGCACCTCTCACTTTGAGGTGAGTCCTCCCACATCCATTAACACAGTCAG
ACAATCCCCCACAGACACTCCAGAGGCCATGCTGCTGCCCTCAACAGTCACTCAGAGACTCACTTCCCGGGTGATTTTTC
AGGGTGTGTGAAGTTGAGACATTAATCATCACACTGGAGGATTCTAGGTGGTTCTCTACCTCCAAAGTCGGCCTTCCCTG
TTTGAAGTGAGTCCTCCATACACACTTCCCAGGCCCCAAATCATGGGTTCTACCAACTATGCCCAATATACGCATGAGGG
ACTGGAGATGAACCTGCATCTTCACGCCACCTTAAAAGCTGGCTTGGCCCTCTGTCTATAACCCCAGCGCAGAAGGGCAG
AGACAGGAGGATCACTGGAACTGGCTGGCAGCCGGCCTAGCTCTGGGTTCAGCAAGAAACCCTGCCTCAGGGAACAGGTG
GAGATGTGGACATCCCTTGTTTTCTTAGGGGCCAGGGAGGGGGTCGGAAACACACATATGCACCGACCCACACAACATAC
AGTGGAGAAAGTGCCTGCTTTTTGAGGAATGAGGGCCTGGGTTCAGATGCCTGGCATCAAGGTCAAAAGCCAAGTGTGAC
AACTCGTGCCCAAACCCTAGCACTGGGGTGGACGTGGTGGTGGTGAAGGAGGGGTGTCTACGCTCACCAGCCAGGCAGAG
CACAGAAATGGTGTGCTCTAGGCTCAGTGAAAGACCCTGGCTCGAAAGAAAGAAAGAAAGAAAGAAANNNNNNNNNNNN
NNNNNNNNNNNNNNNNNNNNNNNNNNNNNNNNNNNNNNNNNNNNNNNNNNNNNNNNNNNNNNNNNCAGGCAGGCAGGCAGGCA
GGCAAGAAAGAAAGCAAGCAAGAAAGCAGGTTGGGTGCACAGCTTTGACCCTAGCCCTTTAGAGACAGAGACAGGTTGAT
CTCTGTGAGTTTGGGGCCAGCCTGATCTACGTAGAGCGTTCCAGACCAGCGGGGGGGGGGGGAGTGCATAGTGAGACCC
TGTCTCAAAAAAAAAAGTAAAAATTGTAAAAAAGAAAGAAAATAAAAGGGAAAGGTGATCGCTAGACATGGAGCTATAGC
ACGGCATTTACCTAGTCTGCAGGGGGTCCTGAGTTCAACTCCTACTGCCTACATGAAAAATTAACAACCGTTGTGTTGTG
TGGGGCAGGAGAGACAGCACCCACCTGGTGACTCACAACCACCTGCAACTCCAGTTCTAGGGGGTCAGACATCCTCTCCA
GGCTATTTCAAGCACTACATGCATGTGGAAGCTAAATATAATACACACAATAAAAATAAAGGTTAATAATAACAGTAG
TAATAATGNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
TTTTCCATACCTCTTTTTTACTTTTTATTTTGAGACAGGATTTTCATAATTGTCTGAACTGGCCTGGAATGTACGGTATA
TGCCTCGGTCTTCCAAATAACTGGGATGAGAGGCCTGTGCCACCGGGCCTGACTGCTCACTCGATGCTGTGTGTACAGTA
AGGAGTGAAGAGCACCTGGGGTCTTTTGATTGGTCTAACAGCCTCCGTGGCCCAGCTGGAAACTGGGATTTTGCCTCCCA
GCCCTGTTTGGAGGGACACCCCCTACAGGTCAGGTTTACCATAACTGCTCTGCCCTGTGTCCTCGGCTTCCCCAGATGAC
```

```
CAGGGAAATGAGGAAGGGGACTCCTCTCCCGATTTACTGAGACAAGGGTCCTGCTGCAGAGCCTCCCCAATCTAATCCAG
GTTCCCAGAGTATTGACAGATCAGAAGTAGGCAGAGTCTTGACAGGAAGGGAGATGGTTGCTCCACTTCCAGCCTTGAGC
TCCCCAGGGAACTATTGAGGTGGCAGATGAGATAAGAGAGCTTGGGAAGGGAGCCTCATAAAACAAGGTCACAGACCGTG
TCCTCAAACGGCCGGGAGAAGCGAAAGATTGGCCTGTTTGCATGCGTTTTGTTTTGATGTGGTTTTGAGGGTATTTTGTT
GTTGTTGATTTTGTCGGTTTTTAAATTTGGTTACATTTTATCTATTGGGGGATGGCTGGTGCCCAGACCTTGGCACAGGT
GTGGAGGGCAAAGGACAACTGTTCTCTCCTTTCACCCTGTGGTGCCAACAATAGAGCTGAGGTCATCAGACTTGGCAGCC
AGGGCCTTTGCCCAATACACCAGCTCACTGGCCCTGTTTCTGGGGTTTGAGGGGTTTCGGTTGTTTTTGTTTTTGAGACA
GAGAGTTGCTGTGTGGCTCAGGCTGGCTTCGAACCGGCAGCAATCCTCTTGCTTCCGCCTCCCTAATGCTGAGATTACGG
GTGTGTGCCTCAGCAGCAGGCTGCTCTAGCATCAGAAGGCAGTGGTTCAGCGGCTCATTTAACCTCATACGATAGTGGGG
CCAGAGGCTGGATAAAGGGACAATAAATGTGTCACCACTGTTAGCTCCAGTTCAAATGGCTCACACAAAGGCTAGGGATG
GGCCTTTTGGGGCACCTGCAGTTCCAGCTCCTTTAGAAACTGAGGCAGAAAAACAGTCTCATGTTCAGGGCCAGCACAGG
CTGTGTAGAGAGATCCCATGTAGCAAAAAGAAAATAGGCCAGCATGGTAGCGCAGATCTCTTGTCTTGGATCCCAGCACT
TCAAAAGAAGAGGCAGGTGGATCTCTGTGAGTTTCAGGCTAGCCTGGTCAACATAATCTGGTTCCATGGTGAGACCCTAT
CTAAAGAAAAGAAAAGTGAAATTGAAAAATCAGGTTCAGGAACTTTGAACAGTTTGATAAAGTGGTTGCCTAGCACAAAA
CCCTACCTTTGAGCTTCTGTGTGGAGTAAAGGCTGTGGGTAGTGTGCCTGTAATCCCAGCATTCAAGAGGTGGGAGGCTA
GAGGCAGTTCAAGGGTTCAAGGTCACCCTCCAGCACACAGTAAGTTCCAGGCAGCCTGGGTTCCATGAGGCTTTATTCCA
AAAATAGAAACAAAATTTTAAAACCATATCTCCTTCTAATGTAACATTCTAGGCCATCTGTATGTGTTGACTTCTCGGCC
GGAAGTTGAGCTGCCTGTTTGTTCTGTTCCAGGCTGGACCCCAGCTCACACACAGACAACGCTTACACACACGGTGTGT
CAATCGGAGAGTGCCCAGTGCACCCCTCCACCCCCTGCACTTTCCTGCTCCAGCTGGAGCAGCCCAGGTATACCAGCTCT
AACCCCAGAAGAGTCAGGAGTCAAGGTTGTGGCCACCACTGCTCCCTTCAACAGACAGGTTGAACGTGCAACCCTGGGC
CAAGCACGTACCAAGTACACAAGCCACTACAGCTTTTGCTTAAACTCAAAGGGTTCACACAGGGGCCAGGGATGGACCTT
TGTTGATAGGGCGATTGACCAGCATTCCAGAAGCTGATGGTGGGTGGATTCCCAGCCCTGTATAAAAACCCCTGTAATCC
CAGCACCCAGGAAATGGAGACAGGAGGAGAAGGTCAAGGCCATCCTCAACTACATGGTGAATTCAAGGCTAGCCTGGGCT
ACACAGACAGTGTCTCAAAAACAAAACAAAACAAAATGTTCATACTAGCAGAAGACCAGGAAGCAGAAAGGGAGGAGGGA
GGAGGACAGAGATAGCAAGGGCAGCAGACAGAGAAGGGTCATGTGATGCTTTGACGACTTGAAGTCATGACACAGGTTAG
GGACAAAAGGTACCTCTGCCCTGCCAGAAACCTGGGCCTCCCTGGAGACTCTCAGCTCTGTGTCATCCACACCTGGTGTC
AGCTTCCACGAGCTCTCTGAACCACTACACAGTGATGGGGACACTGTGGGGGCACAGTGCAGGGAGGGGAGAGACACCGC
CCTCAACAGTGAGAACTGAAGGTGTCCAAGGCTCACATGGGGAGGGGGGAAGCTGAGATCATGCTGGGAAGACACACTGG
AGGAGGCGGGAGGCTTGGGTGCACAGGCTGAGGAGTCTGTATTCTGCAGAGGGCTCTGGGCAGCCCGGCAGCCTCTGTGC
TAATGGAGTAAGGGAGGCATAAGGACTGAGACCCAGGTCCTTCTACCGGTCTCAGGGGCAGTGGCATCCTCCTGGCTGAG
AGCAGGGAAATGGAGCAGGTCTGGGAAGATACTAAGGTCGATTTGGGACAAGGTGCCTGAGTCAGAGGCATCCAGGGACA
TTGGGGAGCTATGCTGAACAACAATGGGAATGAGTCCCAGTATTTAGGCGAGAGTGTGGGAGCAGGCCAGACAGGGAGA
GCAAGGTGAGGTAAGGTCACAGTTGAAGGAGGGCAAGAGGCAGCAAACCCAGCCTCTGCCACCCCCACTCTCCCTAGCCT
TCCTCAGCCCTCCCCAGCCCTCCTTCCCCTTGAGGGCAGCCCAGGACACAGTGATACTCTTGAGGAACTGGGACAGGAGA
GCTGCAGGTATGGCCTCCTGCCCAGAAGTAGGCTGCAGGGCAGCAGAGGAGCCAACACCCACAACTACGCCATCAACTAA
GCCATCAGCGATGACGTCTGTCACATGGTGACCAAAGATTCTGAGGTCACTGCCTGGACATCCTCCCTCCACCCCAGTGC
AGGGGGCAGACCCCCTTTCTAAATGGGCAACCCCCCTTTCTGGATTTGCTTTTCCATTTTCAGTGAGGGTTTTGTTTTTG
TTTTTAGAGAGGGTCTCATTATGTTCCCTGGCCTGGCCTGGAACTCACAATGATCCTCCTGCATCAGGACACCATCAGAC
CTAACCTTTAAGTTTTTCTGTTGTTCTGAGACAGGGTCTCACATAGCTCAGGCTGACCTGGAACTCCTTAGGTAGCTGAG
CATCCTCTGACCCTCCCACCTCCATCTCCTGAGTGACAGGGTGATAGGCATGGTCCAGCACACCTGGTTTATGTAGTTCT
GGGTATTGAGCCCGGGGGCTTCATGCACGCCAGGCAAGCACTCTACCCACTGAGCCCCAGCCCCAGCTGGTTCTACATTT
GCCTCCCTGATCCTCAACAAACACAGGGACTTAACTGATTTTTGTAACTAAACTCTCTGACCATCTGTAACAAGGCTGGC
ACAGAGTAGGTGCTCATTCGATATTTGTGGAAGGAATGAGTTTGCGTTTATTGAGCAGTTACTGTATGTCAGGCATGATT
GTAAGCACCATGCATGCTTGTGTTACAGGACCCACGTTGGGCGGGGGACAGCTGCCCTTCATTACTAGGGGTGAATACTG
CCTGAAGAACCCACCAACATTCACAGTTGCTCCAGGTCTTCCTATAAATGGCAGCGGGTTTTAAACAGCCTCAGCACAAG
GGCTCTACCCCTGAGCCACTCCCCCAGGCCCTCACTGGTGGATTCTAGGCAGGGGCTCTACCCCTGAGCACACCCCCAG
GCCCTCACTAGGGGGGGGGGGTTTAGACAGGGGTTCTACCACTGAACCATGCCCCTGACTTCATAGTTTTTATTTATTTA
TTTTTAAAGAATTATTTATTTCTTTTATGTATGTGAGTACACTGTCGCTCTCTTCAGACACACCAAAAGAGGCATCAGAT
CCCATTACAGATGGTTGTGAGCCACCATGTGGTTGCTGGGAATTGAACTCAGGACCTCTGGAAGAACAGCCAGTGCTTTT
AAGCGCTGAGCCATCTCCCCTAGTTCTTATTTTTAATTACCAAAGGAGGATGAAGAGTTCAGGTAATCCAGAAATGTTTG
TTCACAAGGATCAGTGAGCCTTACCTTCCAGAATCTTGCCAATGTCTTTGTATTTATGGCATCACATGCCGATCAAGTGT
GTGTGTGTGTGTGTGTGTGTGTGTGTGTGTGTGTGTGTGTGTGTGTGTTTAAAAAAAATATTCTGGGCCGGGTTAGTGGT
GGCACATGCCCTTAATCCTAGCACTTGGGAGGCTGAGGCAGGCAGATTTCTGAGTTTAAGGCCAGCCTGGTCTACAGAGT
GAGTTCCAGGACAGCCAGGACTACACAGAGAAACCCTGTCTTGAAAACAAAACAAAACAAAACACCAACAACAAACTT
CTGACATTCTGAGCATCCTTGTACTTCTAGTCACCTAGATGGCTGTTATGCAAATGCTATGCAAATTCTTGTTGAAGAGA
AGAGAGAGGACTGGAGAGATTGCTCAACAGTTAGAGCATTCATTGGCTGCTTTTCCAAAGGACCCAGGTTTGATTCCCAG
CAACCCATGTGAATGAGCTATGGACCACGGAAGCTGTGTAGTAAGACCTTGTGTCAAAAAAAGCTGTTTAGTGGGTGGAT
GCTCTTGCAGAGGGCCTGAGTTCTGTTCCCAGAACCCTAACTGGGAGGCTCACAACCACCTGTTACTCCAGAGGACCAAA
CACCCCTGGCCTTCATGGGTACCTGCACACTCATGCAGAGAGCCACACACAACATATAAAGACACTTAATTAAGAAATAA
AAATAATTGGCTTTTTGAAAAAGGGAAGAAATATCAGTGCGGTGGCTCAATGGGTAAAGCCGCTTGCTGCCAAGCCTGAT
GACCCGAGTTCAATCCCTGGAGCCACAGATAGGAGAGGAGACTGCCAAAGGTATCCTCTGGCCTCCACATGCATGAGATG
```

```
GCACATGCCACCCACCTCATGTAATAATGACAAGACAAAGAAAGGACAGTAGTAGGAGTGGAGAAAAGAGAAGGAAGGAA
GGGCAGAGCTTAGGGCAGAGCTTAGCTCAGACAACGGAGCACTTACTGTGTATCCATGGGGCCCTGGGTTCCTTCCCAAG
CACAACACACAAACCAGGCATGGTGGCAAACACCTATCATTTCAACAACACTCAGGAGGCAAATGTGGGAAGATCAGACG
TTCAAGGTTATCCTTGGCTACACAAGAAGTTCAAGTCTAGCCTGGGCTACATGGAACTCTATTTCAAAAACAAAACAAGA
CAAAACAAAACAAACAAGCAAACAAAAACCAGAGTCTTTTATTTCCCTTTGATTTTTTTGAGGCAGGGTCTCATGTAGCC
CAGGCTGGTCTCCTCATCATTGCCTAGCTGAAGAGGACTTTGAAGTCAAGACCCCCCCCCAAACAAATGTAAGTGTTAAA
GATGCCCACCCACCCCACAGAGTTGTGCCATTCACTGTGGTGACACTGGCCACTCCCTGGCTGTCCCGAGGGCTGGAGAA
AGAGAAGTCTCCAACATCTACCCCTCAGTGACCCCACTATGCAGCAGAGACATCACAGGCATGGTAGTCAAGAACTGCGT
CAAAATTTTCCTGCCTCCCTCCTCCCTCCTCCCTGGCGACCTGGTTCTCCTCTCAGTACCAAATCTGGGCCTTTGGCACA
AAGTGGATTAGGCAGCTGCCCCAGGAGGCCTGGCCAGGTCATGGCCCCTGGCCGGTGCCTGCCACACCCACCCTAGTGCC
CAAACAGGGCCCCAGATCTTCTGTGACTCGATCCCTCCAAATTCCGGTGATCCGCCCCGGTTCCAAATTGCCGAAAGATC
CCAGGTCCAGTGGCAGACTGAGCCAAGCCTCAACCAGGAAGGGCCTCTGAGCAACTGGGACACCATCACCACCAACCATC
TCGAACATCAGCCTTGGATGAAAATGCCATTGACCTGGTACAGCTGGTTCCTCCTCAGTGGTGAGCGAGAAGGGGTCCCC
CACCCTGTCAGCCCACTCCCTGCATCTCCAAGAGCCTTGAGACAGAGAGGGGAAGCCCTGCCCGGGAGAAGGAAAGTGTG
AGCCTTCTTCAGGCATGCACTAGGGAGAGCAACTTTGCTTCCCTCTGGAGCAGTTTGCTCTTGAGAGGAGGCCTGTAGGG
CAGGCTGGAGACTGCCACTAAGAGATGTCAGCAGGGAGGCTTTAGAGCAGCCGGAGCAAAACAAGCCACAGAGCTGACTA
GTGTCAGGGCTTCCGGACTCTTGTTTGTGGCTTGGTGTGGGTATCTTTGAGGTAAGGACTCCTATAGCCAGGTGGGCCTC
CCACTTACTTTGTAGTCGAGGATGGCCTTGAACATCTAACCCTCCCCTCTAGTGCTGGGGTTTCAGCTGTGTCATCATGC
CAAGTTTATTGGGTTCTGGGGTTTGAACCCAGGGTTTCACAGGCACTCTACATCCCAAGCACCTTTATGCATCACTGGTG
ACCCGGCTAACCTGGAACTCTCTCTATAGACCAGGTTGGCACCGAACTCACTGAGATCTGCCTGCCTCTGCCTCCTGAAT
GCTGGGATTAAGGGCGTGTGCCACCATACCCAGCACTACAGAGGCTGTTGTGTTGTTGTTGTTGTTGTTGTTGTTGTTGA
GGCAGGATCTCATTATATAGCCCAGGTTGACTTCAAACTTGAGACAGTCGTCATGACACAGCTTCCTGAGTGTGACCACT
GTGCCCGGTTTCTGCTGCTCAGCCTCAGGCTAACTTAGCTGTATATAACAGGGCCTAGTTGCCAGGGTCAGCCAGAGGTC
CTCAGAAGCAGGGGTGATTAGCACTTACTGTTTCCCGACTTCGAGTCCTTTCTCCTTGTTAGCAACCACCCCAGAAGGAT
GTTCACTTGTGGCCCGACTTCCTAGAGAGGAAACCTAGGGCTTGAAGAGGTGGCTTTCTGGCCAGGGACTCACAACACAA
ATGGCAGAGTTCTGCCTTAAGCCCAGAACCTGCCACTACCATCCACATCCTCTCAGCTGGGAAGGGACTGAAAGCCTGGC
CAAGCCTGCAAGTGTGTCTCGTGTGAGAAATGGCAGTGCAGAGGCGTCAGACAAGCCCAGTGAGAAGGAAGAACGAGGAC
CAGCTCTGTCTGCGTGCAGAGAAGGGGTGGGAGAGGCTCAGGATGTGGCTCAGACCGTTGGGTGCTCCGCTAACACTGGT
GAAGCATTGTGTTTGATCCGCAACACTGCCTAAACTGGGTGTTGCGCTGTGTGCCCGGAATCTCACCACTCGAGAGGTGA
AGACAGAGAGATCAGAAATTCAAGGTTATCCCTAGCCATATGTCAAGCTTGAAGCCAGCATCTGGGGCCATAGACAGAGG
GGATGGGGTGGGGGTGGGGTGAGGGGATTAAGGAAAAGGACAGGAAGGAAACTGAAAAGAGGGAGGTACTTCACAGATC
TCTGCTCTCTATGTGGGGGGTCCTTGGGCATACAGAATGAGCACCCCAAGCTGGGGTGAAAGACTTGGACAGGCCCTGAT
GTTTGCATGAGGTCACAGACACAGTTGGCTACATGAGTGGAGAGACCTGTTTGAGTCTCAGCCTGGTGCCTCTAATGGAA
AACTTGGTGTCCCACCTTGCTCTGCAGAGTAATGGCTAGGCAAGGCATCAGGCCAGGTGGCAGGGGCTGGGGCTGAATGG
TAGAGTCTCTTCCTAGAACCCCCCAGTGAGGGGCTGGGGACATGGCTCAGTGGTAGAGACCCTGCCTAGAATCCCCCAGA
GAGGGGCTGGGGGCGTGGCTCAGTGGTAGAGCCCCTGCCTAGAATCCCCCAGAGAGGGGCTGGGGGCGTGGCTCAGTGGT
AGAGCCCCTGCCTAGAATCCCCCAGAGAGGGGCTGGGGGCGTGGCTCAGTGGTAGAGCCCCTCCCTAGAATCCCCCAGAG
AGGGGCTGGGGGTGTGGCTCAGTGGTAGAGCCCCTGCCTAGAATCTCCCAGAGAGGGGCTGGGGCATACCTAGCAAGAGT
AAGGTTCTAGGTTTTATCTCAGGAGAAGTGTGGGTATAGAAGGCACAAATCTTTCTTTTGTCTGACCTTGTAGACACTGG
GGATGTCCCCCTGTGAGCTTTTCACCTCCTGTCAAATGAGGTCAATTCTGCCCCTTCTGTTCCTTCTCAGCCTGGATCCT
GAACACTGGGGCCGAGATCTCCATCACCCCCGAGCCTGCACAACCTGCAGAGGGGGACAATGTCACGCTGGTAGTCCATG
GGCTTTCAGGGGAACTGCTTGCCTACAATTGGTACGCAGGGCCTACGCTCAGCCTGACCTTCCTGGTGGCCAGTTACATT
GTCAGCACTGGTGATGAGACCCCCGGACCAGCCCACACAGGGCGGGAAGCTGTCCGTCCTGATGGCAGCCTTGATATCCA
TGGTGCCTTACCTGGGCACACAGGCACCTACATCCTGCAGACTCTTAACAGGCAGTTTCAGACGGAGGTGGGCTACGGAC
ACATGCAGGTCTATGGTGAGCCCACCCCCTCTGACATCTCCGGGACTTTCCACTCTCTTCCTCATCCTCACCCTCCTTTGA
GGAACAAAAGACAAAAAAATTATTCTTTAAAATAGCATTGTGTGTGTGTGTGTGTGTGTGTGTCTGTCTGTGTGTGTCTGTG
TGTGACAGTATACACGTGGAAGTCAGAGAACAACTTCGGAGAGTCAGCTCTTTCTTTCTACCCTGTGGAACCCAGGGATC
AAAACTCAGGTCATCAGGTTTGGTGGCAAGCACTTTTACCCACTGAACCATCTTGCCAGCTCTTAAAGATGCTTTTACAG
TTGGGTGTGGGGGTGCCTAAATCAACACAAAAATGCTCACCTTCTCCATTCTGGCTTCCAGGATGATAACTGTCTTCCAA
AGCCCTTCTTTGTCACTATGGCTCGGGGGTACAGAAGTACAGCTTGGTGGTACTTCTTGCCCGGGGACAGGAGGTGCCCC
TCCCCTCCCCCTCTGTCTCTCGCTCCGCCCATAGAGATCCTGGCCCCTCCCACGGTCATGGCCAACGACACTGCGCTGGT
GGAGCGTAGGGATACCCTTCGTCTCGTCTGTAGCAGCCCCAGCCCCGCTGAGGTCCGCTGGTTCTTCAATGGTGACGCTC
TGCCAGTTGCCGTCCGCTTGGGCATGTCCCCCGATGGCAGGATGCTGACCCGGCATGGCGTCCGCAGGGAGGAGGCAGGT
GCCTACCAGTGCGAGGTGTGGAACCCCGTCAGCGTCAGCCGCAGCGAGCCACTGAACTTGACGGTATACTGTGAGTCTTC
CAAGGCACCTGGTATCACAGATGGGGAAACTGAGGCCCACGACGAGAAGTCCACAGTTACACAGCAAGTCAGCAAGCAAG
CTGGGGAAGGAGCCAGGGCCCCTCTAAAAATATACTTGCCTACTTTATGAAAGTACGGGTTATGGCTAATACATCCAGGT
CGATGTAGGACCCTGTAGATGAGAGAGAGGGAGTACACACCTGCCATCCCAGGTATCCGGAGGTGGAGGGAAGATCCTGAGG
TGGAGGGAAGACCAGGCCATGGCATATGTCTGGCGTGCACCAAATTCCATCTGCAACACCACAAAAATGCACAGCAATCC
TCTGAGCCCTAGGGAAATGGAGACAGGATCCCGAGCACCCTTCCCCAACAGCTACATGTTAAGTCCAAGGTCATCCTGTG
CTACATGGAGGCCTGTCTGAAAAGAAATAGGAAAAAGGAAAGATGCCTAAGGAAACGGATAGCATGGACACAAGGACACT
GAGCTTGGAAAGGGTCATTAACCATCATTGGAACCCAGGGCTCAGCGGGGACATCACAAAAGCTCATCACAAGAAACACA
```

```
CAGTACTTCCTGGGCCAGCAGCTGTGTGTGTCAAATCCTGAGAATACAAATGATGGGGTTGCCTAGCCATGGTGGGGCAC
CCCTTTAATCCAGGAGGCAGAGGCAGGTGAATTGCTGTGAGTTCAATGTCAGCCAGGGCTACATAATGAGGCTCTTTCTC
AAAGGAAAAAAAGTAATAATAATAATTATAATAATAATAGCAGTAATTTCAAAAGTAAGGCCGGTGTACAGAAACTAAA
ATTCTAGAATCCTACTACCAGAGTATTCCAGATGCCGGAAACTCTAGAACCAGAGTCTAGACTGCCAAGCCTAAAATCAC
AAGAAGTAGAGCCCTAGAATCAAGTTCTAACATCTGAGAGTCTAGAACCTCAGAGGTGAAGATTTCTAGAGCCCAAGAAG
CCTAGAACCTAGAAGCATGGGTTCTTTTTGTTTGGTTGTTTTTGTTTTGTTTTGTTTTGTTTTTTTGAGACAGGGTTTCT
CTGTGTAGCCTTGGCTCTCCTGGAACTCACTCTGTAGACCAGGCTGGCCTTAATCTCAGAGAGCCACCTGCCTCTGCCTC
CCAAGAGCTGGAACTAACAGCATGTGCCACCACCACCCAGCTCTACACATTCTTTTTTTTTTTTACAGATAAAAACAAAA
CAAAAGAAAAAACAAACCAAAACCCAAAAAACCAAAAAACTCAAAAACCAAGTTCTCACGGGAGGGGAAGTGAGTCTCAA
GATTGCAGTGGTAGAAGTCAAGATGTGATTGGGAAAGACTCAAGTTCTTTCTGCCCTCTACTCAGCCCTGCCAACCCCAG
GGACCCAATCCGGTAGATTGAAGACCTGAGGTGCATGCCTCTGTCCCTCCAACCCCACTCCTGCCCCTCCTGTGTCTCT
CCCTCCTGCCCTACAGTCGGCCCTGAACGAGTAGCTATCCTTCAAGACTCTACCACCCGCACGGGCTGCACTATTAAAGT
GGACTTCAACATGTCCCTCACCCTGTGGTGTGTGGCGCGGTCTTGCCCTGAGCCAGAGTACGTGTGGGCCTTCAATGGAA
AGGCCTTGAAGAATGGCCAGGATCACCTGAACATCAGCAGTATGACTGCAGCCCATGAGGGCACGTACACGTGCATCGCT
AAGAACTCCAAGACGCTGCTGTCTGGCTCTGCCTCAGTGGTAGTCAAGCTGTCCGGTGAGTCAGCCCCAGCCTGATAGCC
ACCTGCTCTCTAGAGAGGCCCAGCTGGCCTTTGCTGACTGGGCTGCCATTTTCCAGAAAATAATTGGATTCCCAGCACAC
TCATTGGCCAGTTGTGACACGGTGGCGTATCAGATTGCCCTGTCTTGTCTAGGTGCGGAAAGAGGCAGGCAAAGCCTATT
GCATAGGAAAGAAATGGTGACTCATATCCTCTGCCTTGAGGTCAGACGGAGTGACAAATGAGCCCAAGTCCCCATCTTGT
GGGGTGACAGAGTGGGACACCAGGGTCCCTGTCCTTCCATGTAATTGCCCCACATAGTAAAAAGGCACTCGCTGCTGAGA
GCCCTGCCCAACCCAAGGCACACAAACCTTCTGCCAACTAGATTGTGTGCATCAATTAGTCTAGATAGGATGTGGGCATG
CTCGAGTGCCCATGCGAGTGTTCTCTCTCTCTCTCTCTCTCTCTCTCTCTCTCTCTCTCTCTCTCACACACACACACA
CACACACACACACACACACACACACACACACCACAGCTGGAAAACCACACACCAGAAGCACTTTCTTCACACCTCT
GTTCCATGCATGGGGGGGTTGTGGTGCTGAGGGAACAGAGCTCATGCCCCAAGAATAGCGGCTTTGAACATCAAGTTCTCA
TTTTACAGCAGATGTATAGTGGTGTAGGCAAGAGGATCAGGAGTTCAAGGACATCCTTAGCTACACAGACAGTTCAAAGT
TAGCCTGGACTACATGAGACCTTGTCTCAAGGGGGAAAAAATGTCTCATTCATTCTAGCTGGGTTTCTTCTCAAATGGCC
ATTGTCCCTTGGGGACAGTGGGAATCGTCCACCCCCTCCTCCCTCCCTTCTTCCTCCACTAACAGGCTGGGTCTCATGCA
AAGGGTGGGGATGGACCCTCTTTCACGCAGAGCAAAGATGACTGTCGCAAAGGTAAGTGTGCCTCCTGTCTTACCTGTGA
GGGACCTTGGCTGGAAACAGGGTGGACCAGATGACCTCAGGGCCAACATGGGGGGTATCCAAGGTCTGAAGGTAGGTGCA
GGCTGTGCCTAATGTAACACTTCCAAAACTGCTTCCCCAAAAGCTGCCAGGAATCCTGTGGCAGGTGCTGAGATCGAGAC
AGAGACATGGCTGTTATCCCACCTGGGAAACAAGCTGAGGTGATGGAGGAGGCATGGTCTTTCTCTGACCTCAGAGGGAT
CAGTGAAGCTGCCAATCAAAGAGAAGAGGCAATGCCTCACGTTCTCTGGGCTCCCTGGCATTGGTGTGGGAGACATAAAA
CTGTCTAGTCAAAGGGAGGAGGCACAAGGTGAAAGAATTTCCAGGTCATTAGGAGACATGACACTACTTTAAATTTCATA
GTCTGATGGGGGAGACACAAGTCCCACTTTGGGAACATGTCAAATCTGATGGGGGAGTACAATCTTCCCACTTTCTATGA
AATTTTTCTATCAGAGACAATTATTCCCAACTTTGGGCATCTTCAACTTGATTAGAATGTTCCACTCCCAATAGTCCACA
ATTTCTCAGTCTGATGGAGGAGACAGAAACTCCCACCTTCTAGGGAATCTTAATCTTGGGTCCCAAACTTGATTGAAAAA
TCTAGAATGTCCCAATCTGATGGCGGAGACACAATCCACACCTTGTGAGAATTTTCCATGTGATACAGGATGCACAGCTT
CATCTCTCCTAGAGTCGTCTAATCTGATGGAGGAGTTGTCACCTCCCATATTCTTAGGTCTGCTGCTCTCACAGAAAAGA
CACTATGTGGACGAGACATTCCAACCATCTGATGGGGGAGACACAGCCCCATATTCTAGAATTTCCAGTCTGATAGGGGA
GGCATGGTCCCCAGAGGTTTTTTTTTTCCTACCCTGATGGAGGAGACATGGCCAGACCCTGTGGGGTCTTTCAATTAATTA
CATAGGGCCTGTACCATCATCCAAGGCTGAAATTTAAACAGGAGAGATGTCTTCCTGGCATCTGAGAAAGAGAGTCTATG
TGATCACCCTAGAAATCACCTCCATCCTCTGTCTCCTCCCCAGCACCTCATGGGGAGAAGCATCAGTGGGGGAGCTGGCC
AAGGGAGGGGGAGGACTCCACACCACACTCAAGCCGTTCTGGCTGACTGTGGCGGTGGTTCTCTCCCCAGCGGCAGCTGT
AGCCATGATGATCGTTCCTGTACCGACTAAGCCAACGGAGGGCCAGGACGTGACCCTGACTGTCCAGGGCTACCCCAAGG
ACCTGCTAGTCTATGCCTGGTATCGTGGACCTGCCTCGGAACCCAACCGACTGCTCAGCCAGCTGCCGTCAGGGAACTGG
ATCGCTGGCCCGGCGCACACAGGCCGGGAGGTGGGCTTCGCTAATTGCTCACTGCTGGTGCAAAAGCTGAACCTCACTGA
TGCCGGGCGCTACACACTCAAGACCGTCACGGGCAAGACCGACACTCTGGAGGTGGAGCTACAGGTGGCCCGTG
AGTGGAGCAGTGTGGGATGGGATGGGGGGGGGGCACCCAAAGATGTCCTTCTCCTCCCTCACATGCCTGTGGGACACAGGC
ATGTGCCTCTTCCATCTGACCTGAGTGTCCCCATGTGGGCGTTTGCCTTGCCTCTCAAACGATGACGTCCACCAAGGTAT
ACGCCTTCCATATGGATATATAGACCGTAAAAGGGGTGTCAAGTGAGGGTGGGCATCTTTTCTCATTAGGTGAGTGGAAA
CTAAACCATGGGGATCTAGCCTCTTCCACCACACTGGGGAGCTGGGGTGAATGCAGGTGTTTCCTCCCCCATAAGACAT
GGCATGAGGGCAAGAAGTTTCCACCTTCAGACACCAGAGCTCTCCCAACCTGAATGTACACTAGGATGCAGGCTGCTAGG
TGTGCCTCTTCCATCAGAGTTCTCTGTTGGGAACCACCTCTCCCCTATCAACCTGGGGGCTCCCACAGTGGAAAACCATG
TCTCTCTCATCAGACTGAAAATTGTGTCTCCTCCATCAGACTGGGAGCTCCCAGAATCAGGCACCATGTCTCCTCCATCA
GACTGGGGGCTCTCAAAGTCAGAGATCATGCCTCCTCCATCAGACTGGGAGCTCCCAGAGTCAGGGACCATGCCTCCTCC
ATCAGACTGGGAGCTCCCAGAATCAGGAACCATGTCTACACCAACAGACTGGGAGCTCCCAGAGTCAGGGCCATGTCTCC
TCCATCAGACTGGAACCTTGTCTCCTCCATCAGGCTAGGAGCACCCAGTCAGGAAACATGCTCTTATAGTCCAAGGCTAT
GTCTTCTCCATGAAACTGGTATCTCCCAGAGTCAAAAGCCATGTCTCTTCCATCAGACCAGAAATTCCCAGAAATCTTCA
AGACCTTACTAGGGCAGGTGCTCTGGACAGGCGCTCCCAGACGGAGAACAAGAGTCTGGGGTCCTGCCTGTCGCATCAGA
CTAGACACTCAGAAGTCAAATTTCCGAAGCCAGGACCAGTCCGGACCCAACCACCAGATGTCTGGGTTCTCACAGGCTCA
CTCCTAAGCCTGTGCACTCGGGGATTCCTTTTCAGCTTCCTTTGTCACAGTCCAGCTGGAGGACCAGTCTTTCTTCATGC
AGCACCAGACCCCAGATCATGGCACAGGCTGAGCGGGGCGGGTGGGGACCGCGGGAGGCGCGGTGGGCGGGGCCGCGGGA
```

```
GGGTCCTCCTGCTGACTGACAACCTCCTCGTCCCCGCCCCGGTTCTCCTGCACAGCCCTGGAGTAGCCACCGCCGCCTCT
TCAGCTGCAGCGCAGGCCGCTGAGACCTGGAGAGAAACGCCCCTTGCAGCATCTCCCCGCATCCGCCCCTGAGCCCAGGA
CCGCTCACCTGCAGCACGGATGCCGGCCATGGTCCCGCCATCCTGCCTCCATCTAGTCTTAGCGCCGAGCTGTGGGAGAG
CTGCCAAGTCCATAAATGTCCCAATGAACGCACATGGGTGTCAGCCTCTCCTTCCACCGCCGCCAGCCTGGCCAAGCGCA
GCTAGTGTTCTTTGCTTTGGTACCCACCCCAGAGCATGCCCAGACTGGGCGCATGCGCACCTAGGGATGCTTAACCCACA
CCCCCTCTGCTCCAGGGCAGAGCCTGGCACCTTTGCATAGGTGTGCAGTTCACAAGGAAGTGTATCCTGTTCCCCCAGTG
GATTAAGGATGGGCCCTCTCCAAAAAGTGTGGAGTAGGAAAAGACCCGCCTCACTGAGTAACTCCTGCCTTCATCCTACC
CTAAGGCAGAGTTACTTCCCCTCAAAAAATGGTGTTTCTCAGCCGGGCGTGGTGGCGCACGCCTTTAATCCCAGCACTCG
GGAGGCAGAGGCAGGCGGATTTCTGAGTTCGAGGCCAGCCTGGTTTACAAAGTGAGTGCCAGGACAGCCAGGGCTACACA
GAGAAACCCTGTCTCAAAAAACAAAAAAACAAAACAAACAAACAAACAACAACAACAACAAAAAAGGTGTTTCTC
AAGGGACTGTCCAGAACTGTCGGGACCTCATCCATTACATCAAGAACGCAACTGAACACGGTGGTGGGTGCCCGTAATGC
CATCAGAGCTGGAGGCAGGAGGGTTAGGAGTTCAAAGCATTGCCCAGTCTATGCAACAAAGTTTAAGATCAGCCTGGCTT
ACAGGAGATAGTATCTCAGGAAAATTTAAAAGCTATAGAGAAACTGAAGAGAGTTGTAAAAGTAGTTTATATGAATCTAG
GATCAGTTATCCCACACATCTTTTTAAATGCTCAGAAGTTCATTGTTGAGAGTAGAGTGGAGTCCCCACAATGTCATCAG
CTATCGATTTTTATCTTCCTTAGCGCAGGGCTTTCAGTTTCCCAGTCACCTCAAGTGCCCGGATGGCTGTCGGGTTTTTT
TTTTTTTTTCTGGTTGCAAGGGACTCTGAGAAGTAGTCTTTCTCTTGGGTCTGTTGATACCTCCAAACAATGTCAAGACT
GCTCAGAGAGAATAGTTCCAAAGTGGGAAGCTGACAGCCTCTGCTATGATGGGTTGAAAATTCTGCCTCCCTGGCTAGAC
AAAATGGCCTCTTTCACATGCAGCCACTGCGGAGGAGCCATGGAAAACCCATGGCCAGATCTCAGAAAGCCTGGGGACCT
GGGATGCCACCTGAGGCTGTGTGGATGGGGCTTTGCATGAGGGGTGAGTTCTTAACTAGGCCAGCAGGAGGGGGACAGGG
AGGAGACCTCTCAAGATTCTAGGTCAAGCTAGAGTCTTTATACTTAGTACCCAGAAGCTCAGGTGTCCCAGGGCCAAGCC
ACACATCCCTCTCAGCATCTCTAGGCGCCATCTCAAGCCAGTTTAAGTCTCCTTCATTTACAGTCCATGTGAGAAACGAC
AATCATCACAAGAGAGTGACATCACAAGATAGTGACATCACAAGACAGTGACATCACAAGACAGTGACATCACAAGACAG
TGGCATCACAAGATAGTGACATCACAAGGCAGTGACATCACAAGATAGTGACATCACAAGACAGTGACATCACAAGACAG
TGACATCACAAGACAGTGACCGTTCTTGTAGCCAGGTTTTGTGGTGAAGGCACTTAGCTCTGGGTCCCAACCCCGCAAGA
GAATGATCATGAGAATAATGTGGGTAGTTTTATTTTTCATTGCTGAGGAATGAACCTAGGACCTTGTTAAGACTAGAAAA
TCAGCCGGGCGTGGTGGTGCACACCTTTAATCCCAGCACTTGGGAGACAGAGGCAGGCGAATTTCTGAGTTTGAGACCAG
CCTGGTCTACAGAGTGAGTTCCAGGACAGCCAAGGCTATACAGAGAAACCCTGTCTCAAAAAAAAAAAAAGAAAGAAAGA
AAGGAAGGAAGGAAGAAAGAAAGAAAGAAAGAAAGAAAGAAAGAAAGAAAGAAAGAAAGAAAGAAAGAAAGGGAAAGAAA
AGCTCTCTACCACTGAGCCACGCCCCCAGCACCTCCCTGGGAGATTTCAGGCAGGGGCTCTACACTGAGCTATGCCTCCA
ACCTAGCCCATTCCCTTTGCTTTTGTTGAGGCAGATTCTTACCATGTAGACCAAGTTGGCACAGAACTTTTGATCCTGCC
ACCCATGTCCCCTCTAGTGTTGGGACTCTGGGTCTGTGCCACATGACCAGGTTTTGTCCCTTTACTTTATCGGTTCCTAT
AACATCTGTTATGCATAGTTACTGCCAGTTCTGAATTTCACAAGTAAGGAAACCAAGTTTTGGGAGTGCTGTCAACTTTA
CTCCAGCTATTGTAGTTCCCCAGGCTGCCAGGAGAGGGCGCAGGTCGTGTTTCTGAACAGGCAAATACTAAACCTCAGCA
CGCTAGGGAAATTGGCTCTTTGCATTTCTTCATCTGTAACACGCACACCATCATAGTACTTGCCTCCTAAGACTGAAGAA
GCTATCAATATGCAGAAGGAAACCTGCCCCGAGTATTGTCAATAATGCTCATGACCTAGAACAAGGTATTTGCCTTTAGC
CACAGACAGATAGAAAACAAACACATTTATCTGAATCTCCTAGTGTTAGAGCTATTACAATTCCTCAGTGAAGGGCAAAG
GATGTGGCCTAGAATCCCCCAGTGAGGGGCTGGGGGCGTGGCTCAGTGGTAGAGCCCCTGCCTAGAATCCCCCAAGGGAC
TGGCTGAGGGAGTGGCTCTTGGCTAGGATGATGAGTCCCTGGGTTCCATCCCCAGTAGTTGGGGAAACAACAACAACAAC
AACAATATGGGTTTCTGCAATGCATCTTCCCACATGTGTGGGTCTTTCTCATTTTGTATAATACTTAATGGACATGTCAT
AAAAATAATTCATTCTGCCTTCCAAGAAAATAATAACCCTAAGCCAGTGTAAGTTCTTGGACTTGCAATACCAATTCCAA
CAGCATCTGGAACCTGTGATATGGATGAAGTTTCTGACCTCCCCTCCAAGCTTCACTTTGAAAGGGGCTCCAGTTCCCAA
AGCTCCTAAATGTTTATATGCACATCAGTCTCGGTGAGTTTGAGGCCAGCCTGATCTACAGAGCTAGTTCCAAGACAGCC
AGGACTACACAGAGAAACCCTGTCGAGCGAGCGAGCGAGCGAGAGAGAGAGAGAGAGAGAGAGAGCAGTTGTAGAATTGC
TGTGTTGGTAGAATGCTTGCCTGCCGTGCATGAAGCCCTGGGTTCAACCCCAGCACCACATAAACCCAGTGAGGTGGCAT
CCAATTGCAATGCCAGCTCTGTGGATGGAGGTGGAGGCAGGGGGTCAGAAGCTCAAGGCCAATCTCAGCTATATCCATCC
AGATCCAAGCCAGCCTGAGGCACAGAAGAGCCTGCCTTTGGCCAGGTACAGTGGCACATATCTTTAATCCCAGGACGTGG
AAAGCAGAGGCAAGAGGAGTTTGAGGCCTGACTGGTGTACTTACGTGGGTTCCAGGCCAGTCAGGGTTACACAGTGAGAG
CCTGTGTGAAACAAAACAAACAAAGCAACGCAATCCTGGGTTCAATCCCCAGCACCCATGTGGTGGCTCACAACCATCT
ATCTTGGGATCTGATGCCCTCTTCTGACAAGCAAGTATGTGAATACAGATAGAGCACTCATATACATAAAATAGATAAAT
CTCTTTTTTAAAAAATAAGAGAGAGAGAGAGAGAGAGAGAGAGAGAGAGAGAGAAGAAACCAAGCAGAAAGGGGGAGAAGCAAT
ATGTCCCAATTTTAAATTAATAGGATGCACAGACAGGTCACTGGTTTTCCATCACCAGAGGTCACCAAGCACAGGTAGCA
TTTGGAGCCTTCAGATTCTATCAGCACAGGAGAAGGCAGACACAGCGACAGAGACAGAGAGACAGAGACAGAGAGAGATG
GAAAGCACCTCCAAGACATCTCTAGCTCCTCCAGCCCCGTCCCCAGATGCCCTTTCTCAAGAATCTGCCATAGATGGCTT
GGGCTGGACAGGGAACGACCTTGAGGCCGCACACACAATGGCCCAGTTGCCTGTGGAGCCTAGCCAGGAAAGGAAGGAAG
TCTGACCCCCTTTTTTTTTTCCTCTACAGACTGTAGCGCCAAGGTGGGGAGGAGTAGGATAAGGAGGAGGCCGCGAGCG
AGCGGGAGAAGGGCTGGGGGAGCGGGGGGGGGGGGGGGAGAAGCCTCAAGGGGCGGGGGAGGGACCTGGCGCCAGGC
TGCCAGTGTTCACCTTTCAGAGGCAAGAAAGGTATCTCACCCATGCATCCCATTCTAACCCCCAGTGCCCAGAGAGGGAG
CGCTCGCCTCTAGAGGGCGGCACAGCAGACGACCTCTGGTGGGAGCTCTTGTACAACTCCCCCACCCCCCACCCCACCCC
ACCCCCACCCCGACACACAACATGTTTTAGAGGTGGAGTCAGCCTCTTCATTCCTCACAGGAGAAAATCAAAGCCTCTAG
AGCTAAGGTTTCTTCTCTCTTCGATGCAACAGACCTAGGCCTTGTTTGAATGTCTGTGAGTTTATTTTGTGCTTTGGGGG
CAGCGTTTTGCTACAGCCCGGGCTGACTTGGAAGCGCCTCCTGAATACCGGCAGGACAGGTGTTCCCAGGAGCGCCCGGC
```

445

```
TTCGCAGTTCTTCCCAAGGAGGAAATGCCTCCTGCTCTTCACGCTCATCCCTCCTTTATGTCCCAGAACTCCAGAGGAAT
GAAGCAAATAAGTAAATATGAGAACGGGGCTGGGGCGGGTGACTGGGCAGCAAGATGGCTTGTGGGTACATGCGCTTGCT
GTCAATCCTGATGACCGGAGTTCCATCGCTGGGATCCACATGGCGGAAAGAGAGGGCTGACTGCCACAGGTTGTCCTCTG
ACTTCTGCCTACATGTTGTGACATGTGCACACACCCAAATAAATAAATACATTTCAAGAATTGACCTGGGGACACAGCAC
AGCTTGTGGGATGCCTGCCCAGTACGCACAAAGCCACCCCATAAACCAAGCAGGGTAATGCACACCTATATTCCAGTGGA
GGAGGAGGCAGGAGGTTTTAATTCAGTCCGGGGCTAACCTGGACTACAAAGACCCTAAAACAAAAGACCCACTCTTTTTG
GCTAGGCATCGTGGGGCACACCTGCAATCCCAGGACTCCAGAGATGAAGCAGGAGACTCATGAGTTCAAAGTCATCCTCG
GCTACAAAGCAAGTTTGAGGCTAGCCTGGGCTGCCAAAGAAACTATCTCCAAAAAGCAAGAATTAACAGAGTGGCCGAGA
AGGCTGGAGGTGGGTCTTTGTCCACTTGAATGTCCAGAGCCAATGGTGGCCAACTTGAGGCACTTCAAGGCACAGAACTG
CATGAGTGGTTCAAGATGCCAGAAGTCTAAGACCAAGGCACTGGTGAGGGAGACCTCACTGTGAGGACTCCTGCCTAGGC
TAGTAGGCAGCCATCTTGCTTTGGCCTCATAGGAGTCTCTTCTCTGAGATACACAGAGAGGCTGAGGCAGATGGGGCAGA
ACCCAGGCTCTCTGATAAAGGTACAAATCTCATGAAGCCAGAGCTTTATTTATTTATTTATTTATTTATTTATTTATTTA
TTTATTTATTTATTTATTTAGACAGGGTTTCTCTGTGTAGCCCAGGATGTCCTGGAACTCACTTTGTAATCCAGGCTGGC
CTCTAACTCAGAGATCAACCTGCCTCTGCTTCCCGAGTACAGAAATTAAAGATGTGTGCCACTTCCCAGTAGAGACCCTC
TTTTATGACCCCACTTAACCTTAATGATGCTCTTATGGGCTCTGTCTCTAAGTGCAGTCTTTTTAAGGGGTTAGAGCACA
TGGGAGTGAGAAGCACACACTGTCCCCACCTGTGTCACCCACCTTCAAGGACAGTGCCAGGCAAACAACTGGTGCTCAAC
AGATGCTTGAAAAATGGGTCAGTGAGTGCGTGAGTCAGTGAACACCTGAAACCTGCATGTTGGCCATGCAGTCTGTAAAG
GAGCCAGGTAAAGTTTAGGGGTGTGTATTGAAGAGGGCAACAGGATCTCCAGCCTCAGCAGTCCAAATGGGAAGCAGAGA
ATTGGGAAAGTCTGTAGACAAGTCATTAAAGTGATATCCTGAAAGCTGTGAACAAAGAAATACAAGGGTATGAAATTTAG
ACAGATGAGAAGCAGCCTCCCAGGCAAGGACATTCAAAATATGCATTCGAGACTGGGGGCGTGGCTCAGTGGTAGAGCCC
CTGCCTAGAATCCCCCAGTGAGGGGCTGGGGGGCGTGGCTCAGTGGAAGAGCCCCTGCCTAGTATTATGAGTCCCTGGGT
TCTAGCTCCAACACTTCAGTGCAGTGGAAAAACTAAAAACTGGGCTGGCAAAATGGCACAGCTGACAAAAGTATTTATAG
CAGAAGCCAAAGGACCTGAGTCCAGTCTCTAGAACCAAACCTGAAGAAGAAAGAACTGAGTCCTGGAAGTTTTCCTCTG
ACCTCCACATGGGTGTTTTGTTATGTAAGGCAGCACACATTCACGTGTGCGTGGCCACGCTAATAATATCAATAATAACG
ATAATAATATCAACAACAGAAATAAACAGAATAAAGTGTACGTGAGTTTCCTCTGCTGCTGTAACATATAACCACAAATT
TGGTGGCTTAAGCTATGATCCCTAAAGCTAAAACTAGATGTTCCCTGAGCTGCCCTCTCGGGGCTCCGGAGAAGAAATTC
TGCCTCTCGTCCCATTCCTGGAAGCTGCCTGTGGTTCCCTGACCTGTGCGCCGCACGTGCCACAGCTAGAAGGCAAACGG
GGGTAGGGGGTGGGGAGTGTCACATCCAGTATGACCCAGTAAAGACAACAAGAGGAATATAGAGGAAGTGGAACAGGGGT
GGGAGTCAGGACAGTTAGCCAGTTGCCTGAGGAAGGTGGGCACTGTGGAGAGCTCCGGGCAGAGAATGGACAAGCTCTAA
CTTGGATGCCACTAGCTCCCCTGCAGCTGCCTGTAACATGGGCCTCACCGGAAGGGGAGAAAAGAGGTAGGGGCATGTC
AAGCTTTGGGGCCCAGCAACTGTCAGACAAAGCACACAGGCAGGTGAGGGGGACCAGAGAATAGAGTAAGTCACACATCC
TCTGGACCCTGAGACACAGGGGCTCCGGAGGGAAGTGGGTGACGAGTGGGCTGTGGTTTTTCACCCATCTGTGAAGAAGC
CTCGTTTTCTCTCCGACTTCCCCTCCTGTCCCGCAGAGCTGCCTTGTTTGTTCCCCCATCCTTCGTTTATGATGGGAAAG
GCTGGATTTCCCCTAACCCAGCAGACACCTCTTCCCTCTGCCTAGCCCTACCCTCCCACCTCGGGAAGCCCCGTGCGGCC
TCCCAGAATGTCCCAGAAGGATGAGTGTACTGATATACCCTCTACTACCCATTCATCCAGCCAGACGGGTCCCCAAAAGA
GGAAGCAGGTCACCCGCAGTCACCCAGCATGTGGCCAGCTGGGAACATCAGGACTTCACCTTCCTACGCCCCTCCCAGCA
CCCCAAGACACTCATGTGAGAAACGGGTTTATAAGGACGTTCTGCAGAAGGGGGAGTCTAGATTCAAACCCAGGTCTCAG
AATTGGTTTTGCTGTAAGTTTAGAGGCAAAGGCCACAGATTAATGGAACCTGGCTTATGCCAGGAAGACAAACCTGAGCC
CCAGACAACCAAGGCCCACCAGATAGAGAAGTACAGGTAGGAGCTGAGAATAGTCAGAAAGGCCTTCCTGGAGGAGGCAT
ACATCTCTCTAAGGCTCTCTAAGTATGGGATGAATCAGCCTAAGTGTCCCCTAGAGATGGAACAGTGTATGCAACAGCCT
GAGGCTTTGTAATTACATCCATTGCTCTCCCGGTCTGAGTAAACAGTTCAGTGTCCTCTCATTCATTCAATAAACACTTC
CCGGAGTGAAGACATGTGGAGAGCGGTGGTCCCACTGGATCGTGACCTTGACCTGGGAATCAGAGAGGGTTCTGGAAGAA
GGAAGACGTGCAGAGGAAGGGAAAAAGAAGAAAGGACACTCCAGATAGAGGGAGCAGCCTCTGCAAAGGCTGGAAGTAGC
TTCTTACAGTGACACGATTTACCATCCGCCCGCAGTTCCTCTGAAGCTTGGTGTCCTTGCTCAGAGAGGCCATCCCTGTG
GCCTTTGGGGTACACAGAGGAGAAAAACAGTAGGTTGTACCTCGGAAGCCAGCCTCCTCAATGCCCAGGGGAATGAATAC
AGCGAAGCCATTAGAATTTGTACCTAGGTCGGTCCCGGCCTCTGACTAGGAGATTGGCTTGTAGTAGCCTCCAGGTGGCG
CCAGTGAGCCACCCGCCGAGCCTCTCCTGCTACAAAGGTGGATCTGGCCTGAGCAGGGCACTGGGTCCCCCAGTCGTGT
GCCCATGCTCCCTGACCTATTAGAGGACAGGGATTCCTGATCCGTTCCCTATTGCCTGGGAAACCTTTTTCCCAACTCAG
ATTTATTGGAAGCCCACGGTGTCACCACCCGACCTGCCCATACTCCTGTGAAACTCCTGGGCTGGGAATGGTGGGGG
```

MOUSE mRNA SEQUENCE : mR14-032.1 (Seq ID No: 130)
```
CCTTGTTCCTGGCCCTTGAGCTGCCCCAAGGCTCACAGGCAGCCCTCCACATCGAGATGATCCCAGAGCAGCCTCAAAAG
AACCAGGACCTTCTTCTGTCCCTTCACGGTGTTCCCAGCACTGCTCAGGATTTCATCTGGTACCTGGGGGAGGAGGCTTC
TGGAGGCACAAGGCTGTTCTCTTATATCCCCGGACTACCACGGCCCCAGAGGGATGGCAGTGCCATGGGACAGCGAGACA
TTGTTGGCTTCCCAAATGGTTCCATGCTGCTTCGAAATGCCCAACCTTCAGATAGTGGCACCTATCAGGTGGCCGCCACC
ATGAACCCTGCCTGGACAATGAAGGCCAAGATTAACGTCCATGTAGCTGCCTGGATCCTGAACACTGGGGCCGAGATCTC
CATCACCCCCGAGCCTGCACAACCTGCAGAGGGGGACAATGTCACGCTGGTAGTCCATGGGCTTTCAGGGGAACTGCTTG
CCTACAATTGGTACGCAGGGCCTACGCTCAGCCTGACCTTCCTGGTGGCCAGTTACATTGTCAGCACTGGTGATGAGACC
CCCGGACCAGCCCACACAGGGCGGGAAGCTGTCCGTCCTGATGGCAGCCTTGATATCCATGGTGCCTTACCTGGGCACAC
AGGCACCTACATCCTGCAGACTCTTAACAGGCAGTTTCAGACGGAGGTGGGCTACGGACACATGCAGGTCTATGAGATCC
TGGCCCCTCCCACGGTCATGGCCAACGACACTGCGCTGGTGGAGCGTAGGGATACCCTTCGTCTCGTCTGTAGCAGCCCC
```

```
AGCCCCGCTGAGGTCCGCTGGTTCTTCAATGGTGACGCTCTGCCAGTTGCCGTCCGCTTGGGCATGTCCCCCGATGGCAG
GATGCTGACCCGGCATGGCGTCCGCAGGGAGGAGGCAGGTGCCTACCAGTGCGAGGTGTGGAACCCCGTCAGCGTCAGCC
GCAGCGAGCCACTGAACTTGACGGTATACTTCGGCCCTGAACGAGTAGCTATCCTTCAAGACTCTACCACCCGCACGGGC
TGCACTATTAAAGTGGACTTCAACATGTCCCTCACCCTGTGGTGTGTGGCGCGGTCTTGCCCTGAGCCAGAGTACGTGTG
GGCCTTCAATGGAAAGGCCTTGAAGAATGGCCAGGATCACCTGAACATCAGCAGTATGACTGCAGCCCATGAGGGCACGT
ACACGTGCATCGCTAAGAACTCCAAGACGCTGCTGTCTGGCTCTGCCTCAGTGGTAGTCAAGCTGTCCGCGGCAGCTGTA
GCCATGATGATCGTTCCTGTACCGACTAAGCAACGGAGGGCCAGGACGTGACCCTGACTGTCCAGGGCTACCCCAAGGA
CCTGCTAGTCTATGCCTGGTATCGTGGACCTGCCTCGGAACCCAACCGACTGCTCAGCCAGCTGCCGTCAGGGAACTGGA
TCGCTGGCCCGGCGCACACAGGCCGGGGAGGTGGGCTTCGCTAATTGCTCACTGCTGGTGCAAAAGCTGAACCTCACTGAT
GCCGGGCGCTACACACTCAAGACCGTCACACTGCAGGGCAAGACCGACACTCTGGAGGTGGAGCTACAGGTGGCCC
```

MOUSE PROTEIN SEQUENCE : mP14-032.1 (Seq ID No: 131)
```
LFLALELPQGSQAALHIEMIPEQPQKNQDLLLSHGVPSTAQDFIWYLGEEASGGTRLFSYIPGLPRPQRDGSAMGQRDI
VGFPNGSMLLRNAQPSDSGTYQVAATMNPAWTMKAKINVHVAAWILNTGAEISITPEPAQPAEGDNVTLVVHGLSGELLA
YNWYAGPTLSLTFLVASYIVSTGDETPGPAHTGREAVRPDGSLDIHGALPGHTGTYILQTLNRQFQTEVGYGHMQVYEIL
APPTVMANDTALVERRDTLRLVCSSPSPAEVRWFFNGDALPVAVRLGMSPDGRMLTRHGVRREEAGAYQCEVWNPVSVSR
SEPLNLTVYFGPERVAILQDSTTRTGCTIKVDFNMSLTLWCVARSCPEPEYVWAFNGKALKNGQDHLNISSMTAAHEGTY
TCIAKNSKTLLSGSASVVVKLSAAAVAMMIVPVPTKPTEGQDVTLTVQGYPKDLLVYAWYRGPASEPNRLLSQLPSGNWI
AGPAHTGREVGFANCSLLVQKLNLTDAGRYTLKTVTLQGKTDTLEVELQVAX
```

MOUSE PANTHER CLASSIFICATIONS
      FAMILY (SUBFAMILY)
            CARCINOEMBRYONIC ANTIGEN(Unassigned)
      BIOLOGICAL PROCESS
            Biological process unclassified(2.99.00.00.00)
      MOLECULAR FUNCTIONS
            Cell adhesion molecule(1.05.00.00.00) > CAM family adhesion molecule(1.05.01.00.00)

MOUSE GENE ONTOLOGY
      BIOLOGICAL PROCESS
            defence response > immune response
            cell communication > cell-cell signaling
            protein-membrane targeting > post-translational membrane targeting
            cell communication > cell adhesion
            cell communication > signal transduction
      MOLECULAR FUNCTION
            molecular_function unknown > tumor antigen
            defense/immunity protein > immunoglobulin
            B cell receptor > immunoglobulin
            GO molecular function > cell adhesion
            protein kinase > protein tyrosine kinase
            enzyme > protein kinase
      CELL COMPONENT
            extracellular > extracellular space
            plasma membrane > integral plasma membrane protein
            cell > membrane fraction
            cell > plasma membrane
            extracellular matrix > basement membrane
MOUSE PROTEIN DOMAINS (INTERPRO SIGNATURES)
            IPR003598 (IGc2)
            IPR003599 (IG)
            IPR003006 (ig)

HUMAN GENOMIC SEQUENCE : hD14-032 (Seq ID No: 132)
```
ACAAAAAAATTTATTTATTTACTTTCATTTATTTATTTATTTATTTATTTTGAGATGGAGCCTCTCCCTGTCACCCAGGC
TGGAGTGCGGTGGCACGATCTCGGCTCACTGCAACCTTTGCCTCCTGGGTTCAAGCGATTTCTGGCTTATTTTTGTATTT
TTAGTAGAGACGGGGGTTTTACCACGTTGGCCACGCTGGTCTTGAACTCCTGACCTCAAGTGATCCACCCACCTCGGCCT
CTCAAAGTGCTGGGATTACTGGCGTGAATTACCGCACTGGGCCAAAAATATTTAAAAATTAGCCAGGCGTGGTGGTGTGT
GCCTGTGGTCCCAGCTACCTGGGAGACTGGGGTGGAAGGACTGCTTAACCCCAAGAGGTCAAGGCTGCGGTGAGCTGTGA
TTGTGCCACCACACTCCAGCCTGGGCGACAGAGCGAGACTCTGTCTCAAAAAAAAAAAAAAAAGGTGAGGTCACCCAGGGT
```

```
ATCAGTGGACAAGCTGAGAACAAAACCAAAGTTTCTAACACCGAAAATTTGTCTTCTGCTCATCCCAGCCTGAAAGGAAG
GTGAACCAAGATGCAATCCATGCCCATTCAAGTGGGGTCTGTGTGTCCAGGCAGAGGTGTCTGCCCTCTACAAAGGGCAC
AGGGGAGAGAGTGATGCTTAACCCAAAGGAGGTCTCGCTGATGGTGGAATTTCAGGCACCGTGTTTATGTTTGGCCTAAT
TAGCTTGAGACCAGTGTCCTTGACGGGAACTAAGACGCCCCCCACGCCCATCCCCGGCTCCCAGGCTCTCTCCACCCAAG
TTCTGCCCACCAGGGCACACTGCTTCCCTCTGCCACCTCCTCTTCCCACGAGCCCTGGTACACCCTAGATTCTCAGGGCT
GTGGCTCCTTGCTTTACAAATGAGTAAGTTGGTGGCAGGGAAGGAAGTGGGCTGGGCCATGACCAAGAGTCACTATGATT
CACGGGGAAAACCCTGCCCTCCCGATTCAACTCTGTGTGCAAAAAGGTTTTGTGCGGGTTCCTAGGGTCTCAGGGTCAAT
GGATGTAGATAGACCACTAGAGGTATACACAGCCACTGTCCACCAACTCTCCCACCCCCCGGGTCTGCCTACAGCCCTGG
ATGGGGATCCTGTACCGGGGGGGCGGTTGGGGGGTTGGACAGGTGTGGGTTGAGGTGTTGTTTACATGTTGAACACACTG
GGGTTCCAGGTAAGTTTCCGTCAGAAGAAAGCACTTCACTGCTGAGGCAGAAATTTCAAAATATTGAACCACTTTTCGAT
CCTGGCAGAAGGGAATTTTCCCAGGGTGATAAAAGTGTTCTAAAATTAGATTTGAGTGATGGCTGCCCAAAAGGAATTAT
GATATTATTCAAGAAATCATGGCTTTGCTGAACAACAGAAAACTCGAACACTGAGCATTTGAAGATATTAAGGAATTGTT
GATTTTTATGTTCACTGTTTAGGTGAAATGATAGCATTGTGAGTGTGTTATTTTTAAATGAGTCCTTATCTTTTAAAGAT
ACAGGCAGAAATAGCTATAAAATGATCTGATGGCTGGGATTTGCTTTCAAATAATACAGGAGAGGGCCGTGGGTGAGGAG
AGGGAACCAGATTGCCTATGAGCTGAGTGATGGGTATGTGGTTGCGAGGGCAGACACCATTCTTCTCTCTGCTTTCGATG
GGTGCGTAGAATTTCCCATAATAAAAAGTGGTTTTTAAATTGTTTTGACCATTGGCCTCGCGCGGTGGCTCACGCCTATA
ATCCCAGCACTTTGGGAGGCAGAGGCAGGCAGATCACTTGAGGGCAGGAGTTCGAGACCAGCCTGGGCAACCTGGTGAAA
CCCCATCTCTATTGAAAACACATAAAAATTACCCGAGTATAGTGGCATGCGCCTGTAATCCCAGCCACTCGAGAAGCTGA
GGCAGGAGAATCGCTTGAACCCGGGAGGCAGAGGTTGCAGTGAGCTGAGGTCGTGCCACTGCACTCCAGCCTGGGCAACA
GAGCGAGACTCCATCTCAAAAAAGAAAAAAAAATTGTTTTAACCATTCTAAAGGAATGAGAAAAGAATTCTACAACTTT
TGTTTTTTTGTTTTTTTTTTTGAGACAGAGTCTCACTCTGTCCCCCAGGCTGGAGTGCAGTAGCGTGATCACAACTTACT
GCAGCTTCCATCTCCTGGGCTCAAGTGATCCTCCTGCCTCAGCCTCCAGAGTAGCTGGAATTACAGGCATGTGCCACCAT
ACCTGGCTAATTTTTTTTTTTTTTTTTTGAGATGAAGTTTCACTCTTGTTGCCCAGGCTGGAGTGCAATGGCGGGATCT
TGGCTCACTGCAACCTCTGCCTCCTGGGTTCAAGCGATTCTCCTGCCTCAGCCTCCCCAGTCGCTGGGATTACAGGCATG
CGCCACCATGCCCAGCTAATTTTGTATTTTTAGTAGAGACGGGGTTTCTCCATGTTGGTCAGGCTGGTCTCGAACTCCCG
ACCTCAGATGATCTGCCCGCCTCAGCCTCCCAAAGTGCTGGGATTATAGGTGTGAGCCACTGTGCCCAGCCTTTCTTTTT
ATTTTATTTTTAGTAGAGATGGGACTCACTATGTTGCCCTGGCTGGTCTGAAACTCCTGGGCTCAAGCAATCCTCACACC
TCAGCCTCCCAAAATGCTGGGGTTACAGGAGTGAGGCACCATGCCCAACCCAGGCCTAACCATAATTCTAAATCCAAGTC
CAACCCCTTCCCACTTTCCTGAAGGGAAAAATGAGGTGCAGAGAGAGGAAGGAACCCCTAACCAGGTCCCTTTAGGGTCC
CAAGAGAACCCTGGCACTCTCTGCTGGCCTCCATCCCCCTAACTCTGCCCTTCACTTCCTCCCCTCCAGCACCTTCCAGA
AAAGCTCTAGGTGGCTAGTCCCCCTCTGACCACCTTCTCCCACTTTCCCCTGCCCCTCACTCAGCTCCAGTCACACCAGG
CTCCCTGCTGTCCTTATGCTCATCAAAGCCCCTCCTGCCCCAGGGCCTTGAATTCACTTGTTGTACCTTCTACCAAAAAC
ACTCTCCTCCCACACACCCACAGGCTCCCTCCTCTGTCGCCTCCTTCAGGCCAGAAAAAGCGCCACCACCACCACCTCCC
AAACTAAAACAGCAACCCCTGCTCCTCTCTATCCACTTGCCCTATTTTATTTAATTTTATTTATTTTGAGACACAATCTC
ACACTGTTGCCCAGGCTGGAATGCAGTGGTGCAATCTCAGTTCACTGCAATCTCCACCTCTTAGGCTCAAGCAATTCTCC
CACCTTAGCCTCCCAAGTAGCTGGAATTACTGGTGCACACCACCACCACGCCTGGCTAATTATTGTACTTTTTTGTAGAG
ATAGGATCTCACTGTGGTGCCCAGGCTGGTCTCAAACGCCTGGCCTAAGCGATCCACCCACCTTGGCCTCCCAAAGTGCT
GGGATTACAGGCGTGAGCCCCTGCACCTGACCTATTTTACTTTTTATAGCTTTTATCTCTGCCTGACATTAAATGCCATA
TCAGGTTTTTTTTATTATTATTATTGCCCATCTCCCTCCAATAGGATTTAGCCCAACAAAGGAAAGGATGTCTCTGTGCA
AACAGCCTGGTACATAGTCCATGCTTGACAAATATTTGTTAAATGATTGAATGGCTTGGAAAAACCCAGATGAATGAGGT
GAGGAGGGTGGGGACGGGTATCCAAGCACAGGGAACAGCCTATGGAAAGGTCTGGAGCTTCTCCGATCCACCTGCCTCCT
CTCCTGCATGCAAGCCTAGGTGCTTTTTTTTTTCTGAGACAGAGTCTCACTCTGTCACCCAGGCTGGAGTGCAGTGGTGTG
ATCTCGGCTCACAGCAACCTCTGACTTCCGGGCTTAAGTGATTCTCCTGCCTCAGCCTCCTAAGTGTCTGGGATTACAGG
TGCCCACCACCACACCGGGCTAATTTTGTATTTTTTTAGTAGAGGTGGGGTTTCTCCATGTCGGTCAGGCTGGTCTCGA
ACTCCCGACCTCAGGTGATCCACCCGCCTCGGCCTCCCAAAGTGCTGGGATTATAGGCGTGAGCCACCACGCCTGGCCAC
CTGGGTGTTCTTTCTAGGGTCAGGAATTAAGGGGACATTTGGTCCTTTCTCCCCAAGAACAACCTCTCCAGGCCCAGCCA
GAGCCAACTCCAGGCCTTGACAGGCCGGCACAGGAAGCCACACCCTGAGCAAATATGACGGCCCCCTCTGCCTCGCCCAA
GATAAGGGGAGGTTGAAAGGGGTTTGGAAACCTGCTTCAGCCTAAGACATTAGCTCCAGCCAAACCCTCAGTCTCAAGGG
CCTCCCCATCCCTGCCCAGAGAGGACAGGACAAGGATTCTGCCACGGAGGCCCAAGTCTGACGTTTACATCTAATATATA
TTGATGATGTCAGTGGTGGGCAGGGTCTTGAAGAGCAGATAAAGGAGTTCCCAGCCTCCCTCCCGCTCCCAAGGGAACTA
AGTGACTGGTGAGACAAGAGGGCTGGGCGGGCAAGGGGTGACCCGTGTCCCCAGATGGCTGTGCAGAGAAGATAAGACTC
ATCTGCTCCAATCTTATGAGGCAATGGCCCGGGGCCTCAGTTACTTGTCTGCAAAATGGGTTTTCTTGGGGGTGGGCAG
CGGGGAGATGGATAACAGGAAAAGCTGAAGCTGTCAAAAAGAATAATGGGCCAGGCACGGTGGCTCACACCTGTAATCCC
AGCACTTTGAGAGGCCAAGGCAGGTGGATCACTTGAGGTTAGGAGTTTGAGACCAGCCTGGCCAACATGTCAAAACCCTG
TCTGTACTAAAAATATTAAAATTAGCCAGGCATGGTGGCGCGTGCTTGTAATCCCAGCTACTCGGGAGGCTGAGGTGAGA
GAATCGCTTGAACCTGGGAGGTGGAGGTTGCAGTGAGCCAAGATCGCACCACTGCACTCCAGCCTGGATGACAGATCAAG
ACTCCATTTAAAAAGAAGAAGAAGAAGAAGAAGAAGAATGATATCAGCAGAATACATCTACAAGCTGACCACTTCT
CCCATCCCTGGTTCAGCCGCCACTACCTCCTGCCTGGTCCAGCACTACCCAACAGGAGCAAGAGTGGAGCCGCAAACACA
ATTCAACCTTTTCCAGCCTCATCTCATGAAGAAAGGTGAAACGAATGTATTTAACACAACATATGCAAAATGTCATTTCA
GCACAGAATCAATATAAAAAACTGATTATCTGTTTTACATTATTGTGTATGCATGCGCTCTTTTTTTTTTTTTTTTTTCT
TAATGAGACAACAAGGTCTTGCTCTGTCGCCCAGGCTGGAGTGCAGTGGTGAGATCACAGCTCACTGCAGACTCAACCTC
```

```
CTGGGCTCAGGCGATCCTCCTACCTCAGCCTGCCAAATAGCTGGGACTACAGGCGTGCAACACCACTCCTGGCTCATTTT
TAAATTTTTTTGTAGAGACGTGCTCTACGCTGCCCAGGCTGGTCTCGAACTCCTGGGCTCAAGCCATCCTCCCGCCTCAG
CCTCCCAAAATGCTGGGATTACAGGTGTGAGCCACCGCGCCCGGCCCCCCTTATTGAACCTCTACTCCAATGTCGCTCTG
ACAATGTCGCCTTGCTGCCCTGCATAAACCAGTGCCCCGACCACCTCCATCCCTTCCCCTGCTTTATTTTTCTCCACAGC
ATTGTCCCCACCTGTTCTAACACTGCTCATGTTTGTATTCGTCTATTAACTGTCTCCCCAATCAATAGGTCAGCTCTGTG
TCTACCGTATTCACGGCTGTAACCCCACAGCGCCTAGCGCAGTGCCCGGCACATAGTTGGTGCTCCATAAACATTTGTCA
AATGGTGGAGGAACAACGACCACTGTGCCCCCAGTACGTGCTCTGAGGCAGGCCCAGCACGGAACACTCCATGTGCCTAT
TTTGATCCTCAGAAGAGTCCCAGGGAAGTCAATGTCATGAACACCCCCCACCCACTGCACAAACAGGAAAAAGCCCCTGC
CCACAAGAACCCCGTCTGCTTTCTGCGCAGCCCGGCGGCGCACAGCAGGTGCTAAAGAGCCTGCAGCTCTTGACTGAACC
AAGGATCTGGGCTCACAGCCGGGAGCACCAGAGGAGGGGCCAGGGAGGTGGAGGATGGGGGCAGACTAGGAAGGGTGGAT
GTGACGATAGAGGGCCTCTAGAGCCCGGCCTGGTCACACTCCGCAGGCAGACAGCAGATCACCAGGAACCTGAGCCTCTC
TGAAGACTCCTCTCTCTGTGTCATCCACACCTGGTGTCCAGTTCCACAAACATGCTGTGAGCCTCTGCTCTGTGCCAGGT
GATTTGCTGGGCAGTGCTGGGGACACAGTGGTGACAGCCCAGCCGTGCCCAGCTGAGGCTCCCAGGCCAGTGGGGGAAAA
AGACCCAGCCCCAGACAGCGAGAACCCAAGGCAGTCAGGGCTGAGACAGGGAATTCCAGGCCCTGAAGTCCAGGAGGGCT
TCCTGGAGGAGAGGGTTTGGGGAAGGCAGTGGAATAGAGCAGAGGGAAGCAGGGATATTCTGGGTAGAGTGCAAGTGAAA
CAGAAGAACTGAAGCAGAGGGTTCCCGGAAGCAAGTGGTATTCTATGTAGCTGGTCAGGTGGGCAGGAGTCAGGCCGGGC
AGGGCCTCCTGGGCTACAGTGAGGAACCTGGGCTTCCTCCCAAGGGCACTGGGGAGCCACAGCAGGTTCTGAGCACGGAG
GGGCAGAGGCAGGTTTTTGTTTTGGAAAGAGCCTCTGTCTGCCATGTGGAGGGGCAATGGGAGTCGGGGAGACCAGGGCC
AGAGACTAGGGAGGAGGCTGGTGGGGAGACAGGAGGACCCAGGCAAGGTGGGGTGGTGGAGTGGTCACCTAGACCAGGGC
AGGGGCTTCAGGGAGGGGAGAGAAGATATACGTAGAGTCAGTTGCCAGAAGGGGTGGCGTGTTCGGGGAGTAGGGGGAGGC
ATCAGGGATGAGGCCCCAGGTCTCTTTTCCCAGCGATGAGGTGGGTAGTGGCGCCTTCCCTGATAAGGGCACCAGGGGAA
GATACACTTCTGGGGGAAGATGCCAAGGTCAGTTTGGGACAAGGTGAGTGAGAGGTGCCCAGGGACATCAGGGAGGCATC
CTGATTGATACTGGGATGAGGTCCCAGTGCTCTGGGCCAGGACGCAGAAGAGTGAGGTGAGGTCACAGGTGGGGAGAGGG
GAGCAGGAGGCGCCAGGACATCCTCTGTGCCTCTCACCTGGCCTCACCCGCACCTCCTCAGGGCCTTCTCCCCCTTGTTA
GAGCCCTGCCCCACGAGGGCAGCCCAGGACACAGTGCTGGAGGCAAACCAGGACAGGTGAGCACAGGTGTGGCCCCCTGC
CCGGAAGCAGGCTGCAGAGGCCTCAGAGGAACCAGGGCTCAGAAGCCATCAGGATGCAGGTCATAAGAACACAGCTATCA
GCAGCTCTGGCACCACAACCCCCTCGGCTCCTGCCCAGCATCATCTCAGAGACACCTTCCCTGTTACCCTCCCTCCACCC
CAATTTAAAGTAGCAAACCAGGCCAGGGGCAGTGGCTCATGGCTGTAATCCCAGCACTTTGGGAGGCCAAGGTGGGAAGA
TCACTTGAGGCCAAGAGTTTGAGACCAGGCAGAGCAACATAGCGAGATCCCATCTCTACAAAAAATAAAATGGCAAATCC
CCTTTCTGACTTTATTTTTCAATCTGACGTGGAAGATATTTACCTTTCCTTTATTTACTCCGCAAATGTCGGCCCCCTGA
GGGCAGGGATTTCATCCGTCTTGTGCACGGCTGCATCCTCAGCGCCTAGAACAGCACCTGGCAAACAATAGGTGCTCATT
CAGTATTTGTCCAATAAGTGAATGAGTGATTTACTGAGCATTTACTCTGCGCCAGTCGTGGTAAGTACTGTACTTCACAT
GTGTATCTACTTAATCCTTTCAACAGCCCTGTGATGTGAGTACTATTGTTTTCCCTATTTTACAAATAAGGAAACTGAGG
CTTGGCAGAGTCCATAAAATTGCCCAGGGTCCTGGAGGTAGGAAACAGTGGAACTATGTCCAGAGCCTATACTTTTTTTTT
TTTGAGATGGAGTTTCACTCTTTTGCCCAGGGTTCAAGCAATTCTCCTGCTTCAGCCTCCCAAGTAGCTGGGATTACAGG
TGCCCGCGACCACACCCAGCTAATTTTTGTATTTTTAGTAGAGACAGGGTTTCACCATGCTGGCCAGGCTGGTCTTGAAC
TCCTGACCTCAGTTGATCCACCACCTCGGCCTCCCAAAGTGCTAGGACTACAGGCATGAGCCACTGCGTCCGGCCATGA
GCCACCACGCCCGGCCAGAGCCTATACTTTTAATAACAGGCATACCAATCAGACCCACGTCCACCAGGCTCTGGCTTAAA
TCCCTGACTTGTAGCCTCTCACTAACTGCTGTCAACAAACCTGAAGTAGGGTCCATATCATCTCTATTTTTTTGTTTGTT
TTTGAAATGGAGTCTTGCTCTGTCACCCAGGCTGGAGTGCAGTGGCAGGATCTCGGCTCACTGCAACCTCTACCTCCTGG
ATTCAAGCGATTCTCCTACCTCAGCCTCCCTAGTAGCTGGGATTACAGGCATGTGCCACCATGACCGGCTAACTTTTATT
TTTTGTTTTTGCTTTTGTTTTTTGAGATAGAGTCTCACTCTGTCACCCAGGCTGGAGTGCAGTGGTGTGATCTCAGCTCG
CTGCAACCTCCACCTCCTGGGTTCAAGAGATTCTCCTGCCTCAGCCTCTCGAGTAGCTGGGACTACAGGTGCACACCACC
ACACCCAGCTAATTTTTTATATTTTTAGTACATACGAGGTTTCACCATATTGGCCAGGCTGGTCTCGAACTCCTGACCTC
GTGAGCCACCTGCCTTGGCCTCCCAAAGTGTTGGGATTACAGGCGTTAGCCACCACACCCAGCTAACTTATATTTTTAAT
AGAGACGGGGTTTCGCCATGTTGGTCAGGCTGGTTGCAAACTCCTCACCTCAGGTGATCTGCCCATCTCAGCCTCCCAAA
GTGCTGGGACTACAGGCACCTGCCACCACGCCTGGCTATTTTTATTATTATTATTTTTAGTAGAGATGGGGTTTCGCCAT
GTTGGCCAGGCTGATCTCGAACTCCTGACCTCAAATGATCCGCCTGCCTTGGCCTCCCGAAGTGCTGGGATGACAGGCAT
GAGCCAGTGCACCCGGCCATCATCTCTATTTATCAGAATAATAAATGGAGACTCAACAAGGGACTGTCCCCAGCCCCACT
TTGGGTAAGTGCAGAGCTGGTGTCAAAACTCAGCTCTCCTTGGCCCCAAAGCCTCCGAATACGCCCATTTGACAGAGAGG
GGAAAACAAGGCCTCCGAAGTGGGAGAAAGAGAATGTGAACTTCTGAGCACTTTGCAGGAAGCCAACAAGAAGGAGAGCC
CCAAAGAAAGCATGTGGCCATACAGTTTCCTGCCCTTCTGTCTGGGTTTGCACACAGAGCAGGGCTCAAATCCCCTCTCC
CTGCAGGAACCCGGACAAGTGGGAGTCCCCACCTAGCTCATCTCCATCTGCAAAGGGGGGTCCTAAAGGTGCCCACTTTA
CAGGGTTGTTTTGCCATCCGCTGAGACAACACCTGTAGGTCCCCTGCACTGGGTGGGGCTGGGAAAGGTGCAGTCACCTT
TTACTGATTTTATTCCTTGGTGACTCCCAGGCTCTCTGAGGAGGCCACGGGCCTGGGTGGTTGGTAAGAACTACCCGAAA
ACCTTCCCCTACTTTTCCTCTCTTCTCCTCGGTGACCTGGTTCCTCTCCCAGTACCAAATCCAGGCCCTCGGCACAAAGA
GGGTTAGGCGGCTGCCCCAGGAGGCCTGCCCAGGTCACGGCCTCTGGCCGGTGCCCGCCACACCCACCCTGGTACCCAAA
CCGGGCCCCAGATCCTTGGTGACTCGATCCCTCCAAATTCAGGTGATCTCCCCTGGCTCCAAATCACCAAACCCTCCCAG
GTCCTGCGGCAGACGGAGCCGAGCCCCAACCAGGAAGGGAGTCCGAGCACTGGGACTTCAACGCCACCATCTCCAAGACT
CGGTTTGGGGTGAAAGATGGCGCTGACTGGGTACAGCTGGCTGCTCCTCAGTGGTGAGCGAGAATGGCACCCCCCAGCAC
CTCAGCCCCCCGAGGACCCCAGGGAGGGGAGGGGACCCCAGTCTGAGAGAGGGAAGGTGTGAGCAGAAGTCCAGCGTGC
```

```
TAGGGAGGGAGGGCAGCCCTGCTTCACACCGGCAGTTTACCCCTCCAAGAGGCATCTGCATTGGGCAGGCTGGGGACAGC
CACCCAGAGAGGTTTTCAGGGTGGGCTTTGGAGATTTCTAAGCCCAGGGCACAAGCCAGGACAAGGGAGCCGCTGTTGCT
CCTTCATTCCTGCAGGTTTCTGTTTTATCAGCTATGGAAGCCTTGATGAGGCTAAACTCTTCCCAGCAGCTGTTTCTCCC
GGAAGATGTAGCTTAGGGCAGTTGCCTTTCCCTTCTCAGCCTCTGTGTACCCACCTGTGAATGGGGCCTCACTGACAGGG
TCATGTAAAGGTCATTATAAGCAGCACCACATATGCCGTGTGCCCGGAGCTGTTCTGAGAGCTTCTCATGGTTTAAAACC
ACCTATGAGGGGCCGGGCAAGGTGGCTCACACCACTAATCCCAGCACTTTGGGAGGCCGAGGCGGGCGGATCACCTGAGG
TCAGGAGTTCAAGACCAGCCTGGTTAACATAGTAAAACCCCATCTCTACTAAAATACAAAAAATTAGCTAGGTGTGGTGG
CACATGCCTGTAGTCCCAGCTGCTCGGGAGGCTGAGGCAGGAGAATCACTTGAATCCAGGAGGTGGAGGTCACAGTGTGC
CGAGACTGCACCACTGCACTCCAGCCTGGGCAACAAAGCAAGACTCCATCTCAAAAAAAAAAAACAACCCCAAACCACCT
ATGAGGGAGGCCCACACATGAACCGCACTTCATAGGGAGAAACGAGGCTCAGAAAGGTGAAACCATTGGCCGCTGTCAC
ACGGCCAGAGAGTGGCAGGACTGAGGGCTAGACCCAGAGACCCTCACTCCTAGCCACACTCTAACAGCCTGGAAAGTACC
AGAAAGCTGGCCAGCCATGTCAGTCCTTTTGACGTGAGCACTGTCACAGCCGCCCGTTCCGGGCATTCAAACCACACGTC
ATTCAAACCCAAGCAGCCCCGACCAGCTCATCGGGCTTGGGGCTGATCTGCTTGGTTGAGAAGGAAAGGGGTGGAGTGTG
TTCATCTTACCCCTGCTTTCCGCTGTAGGCGAGCTTGGAGAGGCAGAGGCAGAGCCCCAGACTGGGAGTCAGGACCCAGG
GGGCAGGCCCTGACATCCTACGTGGGGCCATACAATTAGCTGGGCTGGATACGCAGCCGGATCTCGCGAGACTTAGTTTG
GTGACTGTCACGATGGGAAAACCCTTTGGGTCCGGCTTAAAGCCGCAGGTCCTGTGGGCCGAGCACTTGCTTTGCACTTG
CTGTATGCAAGATGTGATGCTAGATCCTTGGCACAGTCCATCAGACCCAATCCTCACAACACACGCCTCCCTCCCTCGGA
CTGAGTCCCTCCCTGGGACTGAGCTCCTGCATCTGACTAACATGTATTGGGCACTTACTGTATACCAGGCATTGTAAGCA
TGGTATGTATACAGAATCCTTACAACCTTACCAGGCAGGTAGACCTGTCATCCCCATGTGAGAGTTGGAAGAACAGAAAC
ACAGAGAAAGACAGTGTACTCGAAGCCATGCAGATAAACTCCCAGAAAGATTAGGTGGCTGAGATGTTGAGAGTCAGAGT
TGCAGGTTCAAATCCTGGTTTTGCCTGTCCTCATTTACACAGAGGACACTGGGCTGGGGACGTGCCCCGAGCCTGAGTCT
CTTCTTCTGTCAAATGGGGCGATTGATCAAACCTGCCTCATCCAACCCCTCTGACTCCTCTTCCTCAGCCACATTCCTGA
ATGTGGGGGCCGAGATCTCTATCACCCTGGAGCCTGCCCAGCCGAGCGAAGGGGACAACGTCACGCTGGTCGTCCATGGG
CTTTCGGGGGAACTGCTCGCCTACAGCTGGTATGCGGGGCCCACACTCAGCGTGTCATACCTGGTGGCCAGCTACATCGT
GAGCACAGGCGATGAGACTCCTGGCCCGGCCCACACGGGGCGGGAGGCTGTGCGCCCCGATGGCAGCCTGGACATCCAGG
GCATCCTGCCCCGGCACTCAGGCACCTACATCCTGCAGACCTTCAACAGGCAGTTGCAGACCGAGGTGGGCTACGGACAC
GTGCAGGTCCATGGTGAGACACCCCCCAACACCCGCCTCTGCCCCAGCTGGGCCTTCCCATCTCCTTCTCAATCCTTCTT
TTTTTAAAAAAAAAAAAAAATCCAACAAATCATCAGGGAAACCATCAGGGAATAAGTTTCTAAAATAACCTCAAAATGCT
CACCTCCTTCAAGATGGCCTCCAGGATTAGACCTGCCTCCTAAAACCATTCTTTGTCCCTCCGGCTGAGGGTGTGGGGGC
CCAGCCACAATCCGGCCATGCCCCTTGTTGGGGGAAGGGAAGGCCCTCAGAGCAGGTGGTGTCCGGCCCCCTCCCCCTCT
GTCTCTTGCCCCCACAGAGATCCTGGCCCAGCCCACAGTCTTGGCCAACAGCACAGCGCTGGTGGAACGTCGAGACACCC
TGCGCCTTATGTGCAGCAGCCCCAGCCCCACCGCCGAGGTCCGCTGGTTCTTCAACGGTGGGGCCCTGCCCGTCGCTCTC
CGCCTGGGCCTGTCCCCTGACGGCCGGGTGCTGGCCAGGCATGGCATCCGCCGGGAGGAGGCCGGCGCCTATCAGTGTGA
GGTGTGGAACCCGGTCAGTGTCAGCCGCAGCGAGCCCATCAACCTGACCGTGTACTGTGAGTCCTCCTGGCCCCACTGGA
GATACCCAGTGTCCAAACCTCATGGATGGGGGAAACTGAGGCCAGGAGAGGGGGCCTAAGGGCACACAGCGAGTCAGCAGG
CAAGCTGGGATTGGGGACCAGGGACCCCTCTTAAAGTGCACTTGCCCTCGCCAGGTGCAGTGGCTCACGCCTGTAATCCC
AGCACTCTGGGGGGCCTAGGCGGGAGGATCACCTGAACCCAGAAGTTTGAGACCAGCCTGGGCAACATGGCAAGACCCCA
TCTCTACCAAAAAACATAAAAATTAGCCAGGCATGGTGGCACATGCCTGTAGTCCCAGCCACTCAGGAGGCTGAGATGGG
AGGATTCCTTGAGCCCCGGAGGCCGAGGTTGCAGGTGCAGTAAGCTATGATTGCACCACTGCACTCCAGCCTGGGCGACA
GAGCAAGATGCTGTCTCAAAAAAACAAACAAACAAAAAAAAAAAACAACAACAAACAAAAAAAAATACACTTGCCCCATATCC
TAAAGGACTTCTACAGACCACCTTTACTATTGAATAATCCTTCAGTTAGGAGCAATATCTACAGAGAAAGGAAACAGGGC
CAGTTGGTTTGAAAAGGGAGCAGGGGAGGGAATAGTGTAATGGGAGGGGGACACAAAATTTAACAACATAACCAAAGCATG
CAGTCGTTCCCAGGAGCAGCAGATGTGCAGTGTGGAATCCTGGGAAATAAATCACCAAGGTACATGAGGACCCATGATG
TAGAAAAACTTAGTGCCTACAATCCTAGAATCTTACAACCAGAGAATTCCAGACTTCAGAAGCACTGAATTCCAGACTCT
GAGAATCTTAGAACCTCTAGACGCAGAGATTCTTAGAATCTTAGAATCTAACCCTGAAATCTTAGTCTAGAATCTTAGAA
ACATAGATTTCTAGNNNNNNNNNNNNNNNNNNNNNATTCTACACTCTTAGAATGGCTTTGTCAGAAAAAAAAAAAATCATTT
AGGCTTTTTTTATTTGTCAATGGAGCCATAGATTTCAAAAATACCTTGAAATATTATCTTGCCCAACACATTCTTTTTAC
AGGAGAGAAAGGTGAGATCCTCTGAGAGGGAGTGATTCTCAAGATCCTAGAACAAGTTAGAGTTAAGGCCAGAGCTTTTT
TTCCCCTCCACTAGGCCAAGATATTCCCAGGGACCTAGCTTGGTGGAGAGAAAACCAGGGGTACCAACCTCCACTCCTCC
TTCCCTCTACTGCCCCATCTATCTCCCTCCTGCCCCCACAGTTGGCCCAGAGCGTGTGGCCATCCTCCAGGATTCCACCA
CCCGCACAGGCTGCACCATCAAAGTTGACTTCAACACGTCCCTCACCCTGTGGTGCGTGTCCAGGTCCTGCCCAGAGCCC
GAGTATGTGTGGACCTTCAACGGGCAGGCCCTAAAGAACGGCCAAGACCACCTCAACATCAGCAGCATGACAGCCGCCCA
GGAGGGGACGTACACATGTATTGCGAAGAACACCAAGACCCTGCTATCTGGATCTGCCTCAGTCGTGGTCAAGCTCTCTG
GTGAGTCACCCCCAGCCTGACCACCCCCCAGTCCCATGGAGGCCTCATCAGGCTGCGAAGGTTAAGCCACCATTTGCCC
AACAGAGAATTGGAACATGGTAGATTCACAGTTCATTCATGAGCTGTGACAAAGTGGCATATCAGGCAGATCTGTTTGCA
TCTGGGTCTAAAGAGAGACAGCCCGTGTCCTGTTCACCTAGAGAACAAATGGCAAACCATTGTGGGTTGACAGAGTGGAA
AATCACTCAGATATCACTTCAGGTGGGTGCCACTGCTCTAAATTCAGTCACCAGCTGTTTAGGGGCTTGACCCTCTACCC
GCCCCCACCCCACCACACAGACAGCTGAAATTCTGTGCAGCCAACTAGACTACCCAGTTATCTGACCCAGATGACGGGTA
TACACACACACACACACACACACACACACACACACACACACAACTGAAGAATCACAGCCAATTGCTGGCAAATGGA
GAAACAGCTGGGGAGGCATTGTCATCTTGCTTCTGTCCTAAAATGACCTGAGGGCTGGGGCAGTGCAGGCAGCTGGCATT
TCAGAGCCATTGGGATCCTCAGGCGACATTTTCTCATCTTCCGGAGATGGTGGCTTTGAACATTAAGGCCTCATTAATTC
```

```
TACCTCGGTTCCTTCCCAAGTGGCCATCAGCCCCCGGGGGACAGTGGGGAATGGTCGTTCCCTCCCTCCCACTAACAGGC
TGGGTCTCATCCAAGGGGTGGATTTTCACCCAAAGCAGAAATGGCATCCCCAGAGGTAAGTGTGACTCCCGCTCCACTCA
TCTGGGGCTTTGGCAGCTGGTCTTGACAGGAGACAGGGGATGGACCAGATGACCTCAGGCGCCACCCCAAGCCCTGTCT
GTGCAGTCCAAGAAGGGGGGTGTCCAAGGTATGCAGGTATGTGCAGGGAGTGGATACAGTGCCCCCAAGGGCTTCTAGCA
CTGTCAGCAACTCTGTGGCAGGTGCTGGGAGAGACACAGAAGAAAAGGAGACGCCACCCTCCCTCCACCTTGGGAGACTA
ATCTGATGGAGGAGACAAGGCCTCTGTCCTTGGAGAGATGATCCTGGGACAGGGAAACATGGCTCCTTTATTCAAGGAAT
AGCTGCTCAAAGAGAAGAGGCAATGCCTCATCCTTCATGTCAGTAATGAGGGAGATATGACAACCCACCCTCAGTGAGCT
CCTAGTCAGATGGAGGAGACACAGTCTGCACTTGGGGAGAAATTTCCTTCTGATGGGGAGACTTGGTCTCCAGGAGACAT
GGCCCTGTTTTTTGAGCATTCCTAGTCTGATGGGGTATCCACCTTTGGGGTACTCCTAATCTGATGGAGGAGTACAGGAC
CCCACTTTCTAGGAATTTCCAGTCTGATGGAATAGACATTTCCCTCTAACTTCCAGGGTCCCCAACTTGATTGGAAAAAC
TCAGCAACAATTTAGAATTTCTCAATCTGATGGAGGAAACAGGGCTGCCGCCTTGTAGGAATTTTCAATCTGATGGAGGA
GACATGCTCTCACCCTCAAGAATCTTTCAATCTGATGGAGGAGCTATGGCTTACACATTCTGGGGTCTCTCGGTCTGACA
GACACAAGTCCCAACCATCTCATGGGGAAGACATGGTCCCCATTTTCTGGGATTTCCACTCTGATAGGGGAGACATGGCC
CCTATATTTTGGGAATTTCCAATCTGATGGAGGAGACATGACCCAAGCCTCTAGGGTCTTCCAGTTTGATGGGAGAGACA
CCTCTCAAAGTCTGGGAATTTGTAGTCTGATAGGGGAAACACAGACCCCAGCAGACACTACTGGGACCCAGAATCCACCT
CCACCACCGCACAAGTCACCTCCTTCCTCAGTCTCCTTCCCAGCACCCTACATGGGGAGTGCCAGCCAGCTGGGGGGAGG
CCAGCAAGGGTTGGGAAGGGGACACCATGCCAGATGCAGACCAAACTGACCCAGCCCGCTCGCTCTCTCCCCAGCTGCAG
CAGTTGCCACGATGATCGTGCCCGTGCCCACCAAGCCAACGGAGGGCCAGGACGTAACACTGACCGTGCAGGGCTACCCC
AAGGACCTGCTGGTCTACGCCTGGTACCGCGGGCCTGCCTCCGAGCCCAACCGGCTGCTCAGCCAGCTGCCGTCAGGAAC
CTGGATTGCAGGCCCCGCGCACACAGGCCGGGAGGTGGGCTTCCCCAACTGCTCGCTGTTGGTGCAGAAGCTGAACCTCA
CAGACACTGGCCGCTACACACTCAAGACCGTCACAGTGCAGGGCAAGACTGAGACACTGGAAGTGGAGCTGCAGGTGGCC
CGTGAGTGTGTGGGAAGGGGCAAGGCGTGCCCCTTTTTAGACAAGGGCTCCCAAAAGGAGCCTTTGCTTCCTCCATCAGG
CTGGGGGGACATAGACCAAAGATGGATACCCATCCCCCATCAGGCTGAAGGAAGCAGCCTTGTCTCCTCCGCCTAACTTG
GGGGTCCCCAAATAGGGTTCTGCCTCTTCTATCAAACTAATGACCTACACTGTGGGTAGACAGGCCTCCTCCATCAGACT
GAGGGTTGCTAAAGGGGACCCCCATGCCTTCTCTCTCAAATAAGAGGTACAGCACACTGAAGGTTTTAAAAGAGAAGGCA
TAGTCTGATGGTGGAGCATCCTTTTTCCTGCAGACAGGTGGACCCTAAAACATAGGGTCCATATTGGGGTAATGGGTTAA
TTATAGGAGTTTACCTCTCCTATCAGACTGCAGGCAGAGAATAAGGATGAATCAGGCCTCCCTATTCAGACTGGGAGATC
CTGTGCAGTGGGGATCATGTCTCCTCCTTTCTATTATTGTGAGGGCAAAGTTTCTATCATCAGATGGTGGAGTTTCTCTT
TCAAACTGAAATAAGGATGATTGCTCCATTAGGATGGAGGCCTGTAGGTGTGGAAGTTGTGTCTCCTCACAGAATGGAAG
TTCCCAGATGTTGGGGACCATGTCTCCCCATCAGACTGGGAGCTCTTAGAAGTCAGGAACAATGTCTCCCCATTGGATT
GGGAATTCCCAAGTGTTGGAGACCATGTCACCTCCCTCAGACTGGGAGCTCTCAGATGTTGGGAACCGTGTCTCCCCATC
AACTGGGAGCTTCTAGAGTCAGAAACTATGCCTCCTCCATCAGACTAGGAGCTCCCAGAGTCAAGATCCAGGTCTCCTCC
ATCAGACTGAGAGCTCCCAGAGTTATAGACCATGTTTCCTCCGTCAGACTGGGAGCTCCCAGAGGCAGAGACCACGTCTC
CTTCATCAGACTGGGAGCTCCCAGAGGCATGGTCCATGTCTCCTCCATCAGACTGGGAGCTCCCGGAGTCAGGGACTATG
TCTCCCTCGTCAGACTGGGAACTCCAGGAGTGAGGGTCCATGTCTCCTCCATCAGACTGGGAGCTCCCAGAGTCAGGGAC
CATGTTTCCTCCATCAGACTGGGAGCTCCCAGAGTCAGGGACCATGTCTCCTCCATCAGACTGGGAGCTCCCAGAGTCAG
GGTCTATGTCTCCTCCATCAGACCGGGAGCTCCCAGAGTCAGGGACCATGTCTCCTTCATCAGACTGGGAGCTCCCAGAG
TCAGGGACCATGTCTCCTTCATCAGACTGGGAGCTCCCAGAGTCAGGGACCATGTCTCCTCCATCAGACCGGGAGCTCCC
AGAGTCAGGGTCTATGTCTCCTCCATCAGACCGGGAGCTCCCAGAGGCATGGTCCATGTCTCCTCCATCAGACTGGGAGC
TCCCAGAGTCAGGGTCTATGTCTCCTCCATCAGACTGGGAGCTCCCAGAGTCAGGGACCATGTCTCCTCCATCAGACTGG
GAGCTCCCAGAGTCAGGGACCATGTCTCCTCCATCAGACTGGGAGCTCCCAGAGTCAGGGACCATGTCTCCTCCATCAGA
CTGGGAGCTCCCAGAGTCAGGGAGCATGTCTCCTCCATCAGACTGGGAGCTCCCAGAGTCAGGGACCATGTCTCCTCCAT
CAGACTGGGAGATCCCAGAGTCAGGGTCTATGTCTCCTCCATCAGACTGGGAGCTCCCAGAGTCAGGGTCCATGTATCCT
CCATCTGACTGAGCATCCCCAGAGGTCTGAGTCCATGTCCCTACAGCAGGGCCAGGTTTCCCCCATTAGCTCTCAGACAC
TGGGGATGAGATCATTCCCCAAGTCTGGAATCTTGCCTCCCCCAGCAGACAGAGCATTCTCAGAAGCCATGTCCCTGAGT
CCGGGATGGTGCCTCCCCTATCACTGGAAATTTCCAGGATCAAGGACTCAACCACTGCCAGTCACCCACTTTCATCCAGT
CACTGATCAAACCCTGGTTTTCTGGGTCAGTGTTAGGGGAGAATGCTGTTCAGTGGCAGCAGGCCACCCCGGCAAAGCCT
TGGGATTCCTTTTCACCCATTCTCTCTTCTCACAGTCAACCTCCCTCCCTCGCACAAGGACCAACGCTACCTTCCTGAAC
CTGAACCTGGCACAGGCTGAGCGTGGGGTGGATTGGGCCACTGGGTGGCCCGGGGTGGGCAGGGGAGCAGAAGGGGTGGG
GGCACTGGGGACCTGGTCCTCCATCTCTCTGACATCCTCCTTCGCCCCCTCGCCCCATATGCCCCACAGCCCTGGGGTAA
CAGCGTGACCCTGGAGACGTCCAGAGAGAAGAGCTCTTCGCACCATCCTCTGGTCCTCGCCCTCTGAGTGGGAACCACTC
CCCCACAGCGAGGATGCCAGGCTGTGGTCCTGCTGTTCTCCTGCCTCCACCCTAGAGCTAGAGCCACAGGGCCCCACTCC
CTCGCTGAGCTGTTGGGGAGCCACCGAGGCCATAAACGTCCTGGTTAATGCACACGTGTGTCAGCCTCTCCTTCCACCAC
CGCTAGCCCCGCCATTTCCTGGTGGCCGCACTCATTTCCTTTCCCCCCAAGACTTTTCTCAGTCTGGGTGTGCACGTGC
AGGAGGATGGGCCCAGATCACCGTGCTCCACACCCCACTGCTGGATGCCAGGGCCTTGTGCCCCTCCCGCAGTGCTCCAG
GTTATATGTGAGAGTCCTGCTCCCAGCTGGCCAAGGACTGCCCCTCTTGGAGCACCAGCATTCTGTGGGACATGGCTCT
CCCCTACACTGCACCTCATTTTGTCACCTGGCCCCGAAACAGGGCTGCTTGTGCTCAGGAAATGACCATATTTTGCAAGA
AGTCTTCATCACCTTCTGGTAGTCCTCACCCATTTAATCAAAGACACAACTTTGTCCTGGGATTCCCGCATGTCTCTC
CAACATTATCTGCCCAACCCTGACAATATCTCCTTCCTTCTGCAGCCACAGGAAAGGCCCTGTGGATGGATGAATGCAAG
CGGGGCAGAGGGTAGAGGCCAGGAGTGGAGAGCTGGGAAGTGGGGGCCAGAATGAGTGTGTTTTCTAACTCGTCTCATTAG
TGGGAGCCACAGGCCCCACCCAGCCGCAATGCCCCACTGGAAACAGGTTCTTGGCATTGGTCAGGAGTGGTGATGGCCGA
```

```
GTCGAGGCTTAGAATAGAAAGACAGGTTTGGGCAGAGACGAGAAAGGCCCTTTTGGGGATGATTGTGTTGAGTTGGGGTG
ATGGGTGGTGAAAGACCTCCAGAATGAGGGTTATAAAACCTGGATCAAAATCCTGCATTTGCCACGGATTCACTAGAGCC
TCAGTTTGCCCATCAGTAAAAAGGTGTGGACTTGAGGTGGACCAGACTTTTCCAAACCATTTTTAAAAGCCTCAGAGCCC
TTTCTTCAAATACAAGTTTACATGGCAGGTCAATTTATTATTAATATTTAAAATAGCAATGCAACAAATATAGCCCCAGC
ACAGTGCCCATGCCTGTAATCCTAACGCTTTAGGAGGCTAAGGTGAGAGAAACGCTTGAGCCCAGAAATTTCAGACCAGC
CTGGGCAACATGGCAAAATCCCGTCTCTATTTTTGAAAATAAATAAATAAATAAAACCAACTATAGATACCAATTCAATA
GCTATACTCGTCTCCCCCAAAATTTCTATAGAATTGGAAAAAAAATTACTTAAATTTAAATACCACTGGGATTAGCCAGA
CATGGTGGCGCATGCCTATAGATCACTTGAGCCCAGGAATTTGAGGCTGCAGTGAGCCATGATTGCACCACTGCACTCCA
GCCTGAGCAACAGAGTGAGACTACGTCTCATAAAAAATGTTAAATTTTACAAATAAAAGGTAACACTTGGGGCCGGGCGC
GGTGGCTCACCCCTGTAATTCCAGCACTTTGGGAGGCTGAGGCAGGTAGATCACAAGGTCAGGAGTACAAGACCAGCCTG
ACCAACATGGTGAAACCCCGTCTCTACTAAAAATACAAAATTATCCAGCCGTGGTGGCATGCTCCTGTAATCCCAGCTA
CTCAAGAGGCTGAGGCAGGAGAATTGCTTGAATCTGGGAGGCAGAGGTTGTAATGAGCCAAGATCGCACCACTGCACTCC
AGCCTGGGCAACAGAGCAAGAAGACTCCGTCAAAAAAAAAAAAAAAAAAAAGTAACACTTGGACTACAGAAGCAACTAAAAT
ACAAGATATCAACCAGAATCAAATGACAGTAAAAGCATCATATACATCAAACCTGTGGGACATGGCCACAGCCATCCTTG
AAGAGAAAAATGATAGCTTATAAGCATTGAGAGTACTAAAGGAAAAGACTTGAAAATGAATAAAACAAGCTTTCTACTC
AAGAAACTAGAAAAAGTCCACAAAACGAACCAAAGAAAAAAGCTAAGGAATGAAGAAAGGTAAAAGCAGAAATAGATGAA
ATGTAAAACAAACATGGCCAATAAAACCTAAAGCTGATTCTTTGAAAATGCAATGAAATAGACAGACTTTCTGCAGGTA
TGATCAAGAAAAAAAAAAAAACAGAGAAGTATAAATAAGAAACACAAATAATAAAGAGCCAAGGAGATTAATATATAAAA
TAAAAGGCCCTGGGGTGGGCGCTGCGCTCACACCTGTAATCCCAGCACTCTGGGAGGCTGAGCCAGGAGGATCACTTGAG
CCCAGGAGTCCCAGACCAGCCTGAGCAATGTAGCAAGACCTCATCTCTACAAAAGTTTTTCAAAAATTATCCAGGAATAG
TGGTGCATGCCTGTAGTCTCAGCTACATGGGAGGCTAAGGCAGGAGGATTGCTTGAGCCCAGGAGGCCAAGGTTGCAGTG
AGCTATAATCACCCTACTGCACTCCATCCTGGGCGACAGAGCAAGACCTTGTCTCTAAAAAAAGAGAGAGAGAGAGAAAA
GAGAAACTGGTCTGGTTGAAGCAAAGGTTGCTCAATAAATATACATTGACTGAAGAGGGATCTGTTGGTTGCTCCCCAGC
CCACTGCCCTCCCTGCTTCCATGACACACACACTGCCTCCCTCTCTAGAAGCTGAAAATGTCAGGTACTCACTTTCCCAA
ACTGCCTTGCAGCTGCAAGCAGCCATGTGACCAATTCAAGCCCATGAGGAAGGAGAGAGCCTAAAGTTCCACCCACTGAC
TCCACCTCACCGGAGGGGACACTGAGGGAGCTCTATGGAGTTTGAAAGCTATTATCTAAATTAGTGGTCTCCATTGTGGG
GCTTCTAGCTGGGACGTGAGGATGGACTTGAGCCCATACTACTAACCCCAACACTATCCAGCCCAGAAAGAAGAAATAGC
ATCCATATTACCACCTTCACCCCACCCCCATCCACCCAAGTTCACATAAAAACTTGACATAGATGTGGCTAGAGTCTAGA
TATAACAGTTAGAAATTCTTGGCCGGGCGAGGTTGCTTATGCCTGTAATCCCAGCACTTTGGGAGGCCGAGGCGGGTGGA
TCACGAGGTCAGAAGTTCAAGACCATCCTGGCTAACATGGTGAAACCCCGTCTCTACTAAAAATAGAAAAAATTAGCCAG
GTGCAGTGGCAGGCACCTGTAGTCCCAGCTACTGGGGAGGCTGAGGCAGGAGTATGGTGCAAACCTGGGAGGCGGAGTTT
GCAGTGAGCCGAGATCATGCCACTGCACTCCAGCCTGGGCGACACAGCAAGACCCCGTCTCAAAAAAAGAAAAAAAAATT
AGCTGGGCGTGGTGGCAGGTGCCTGTAGTCCCAGCTACTCGGGAGGCTGAGGCAGGAGAATGGCGTGAACCTGGGAGGCA
GAGCTTGCAGTGAGCCGAGATCACGCCACTGCACTCCAGCCTGGGTGACACAGCGAGACTCTGTCTCGAAAAAAAAAAAA
AAAAAAAAGAAAGAAAGAAATTCTCTTCAGTGGCTAGGCTGGGCACTGTGGCTCACACCTGTAATCCCAGCACTTTGGGA
GGCCGAGGTGGGCTGATCATCTGAGGTCAGGAGTTCGAGACCAGCCTGGCCAATATGGTGAAGCCCCATCTCTACTAAAA
ATACACAAAAATTAGCCGGGCATGGTGTGATCAAGAAAAAAAAAACAGAAGTATAAATAAGGAACACAAATAATAAAAAG
CCAAGGAGATTAATATATAAGATAAAAGGGCACCTGTAATCCCTGCTACTTGGGAGGCTGAGGCAGGAGAATTGCTTGAA
CCCATGGGGCAGGGGTTGCAGTAAGCTGAGATCATACCATTGCACTCCAGCCTGGGTAACAAGAGTGAAACTCAGTCTCA
AAAAAAAAAAGAAAAGAAATTATCTTCAGTGGCTAGTAAGAAATATTGGAACTAGCATAAACAATTAGGCAGAATAGATA
TACAGGGAGTAGCTTATATGGCTAGAAGATTATTTTTCTCATTTCTCAAACATAAAAAATCTCCAGAAGCAGATTGTTCA
GAGCTAGAATGGAGGCCCTGTAGTGTCATCAGGGACACAGCTCCTTCTATTTTTCTGTTCCATTATCCTTACCTTGGGGT
TTCCATCCTCTCCATCACCTCATGGCCCAAGATGGCTGCTGGAGTCACAGCTGCCTCATCCATGTTCCAGGCAAGAAGCA
GAAAGAAGGGAACAAAGGGAAACAAACAAAAAAAAACTCCCCCTAGCCAGTTTATGGACTGTGCCCCTTTTAAGGGAACT
TTACCTAAGGCCCATCCAACAACCCCTGCTTAGATCTGATTGGCCAGCTCATAGTACCATGGCCACCCTAGCTGCAAGGG
AGGCAGGAAATTGCAGTCATTGCCTTGGGTATGTTGCTGCCCCAATTAACATCAAGGTTCTTGTATTAAGAAAGGCAGAG
AGAATGGGTCCCGGGTGGGCAGCTGGCAGGCTTGGCCACTCCAAGCTTTGGAACTTCTACCTCCTCAGCTAGGCAAAATG
GCCTTCCTCACCTGAGCCCAAGGAGTAAGAGTCACAGCCCCCATAGCAGGGGTTTGAGAAAGATGGGAGCTGCTGCTCTG
AGCTGTGATTGTGTGCAGTTGATGCAGAATGAGGCCAGCAGGAGGGGCAGAAGGAGGGAAGGAGAAGCAGCTCAGGTTTC
TAGGTCAAGCAAAATCCCATAGCCTCAGGGTGTGGTGGAGGCTCAAATGTCCCCAAGACCAGGTCCTCCGTCCCTCTGGG
CATCTCTGTGCTCTTTTACTGAATTATCTCAAGTAGTGCAATCCCCTTCATTAAGAGTGTAGGAAAGAATAATAACAATA
ACCACAAAATAATAATCACTCCTATATCTCGAGCATCAGTGTATTAGTCCATTCTCACATTGCTATAAAGACATACCTGA
GACTGGGTAATTTATAAAGAAAAGAGGTTTAATCAGCTCATGATTCTGTGGGCTATACAGGCTTCTGCTTCTGGGGAGAC
CTCAGGAAACTTACAATCATGGCAGAAGGTGAAAGGGAAGCAAGCACGTCTTCACATGGCCGGCAGGAGAGGCAGGTGGG
GTGCTACACACTTTTAAACAAGCAGCACTAGGGGGACGGTGCTAAAACATTAGAAACTGCCCCCACGATCCAATCACCTC
CCACGAGGTCCCTCCTCCTACACTGAGGATCATAACTGGACATGAGATTTGGGTGGGGACTAAGAACTAAACCATATCAA
TTGGTTTTATCCTTTTTCATGCAAGATCTCAATTCCTACAGCAGCTCTTAAGAGCAGTTACTATCATTCCCCCAACTTAC
AGGTGAGGAAACTAAGGCACAGAGACTTGAAAACTTTGCTCCAAGTCCCACAGTTCCCATGTGTCCTAGGTGGAGGTGCT
GGTGGGGGAGTCCTCTCCATTCTTCAGCAGGGCAGAGGGTTTCAGGGCACAGCTCTGAGGACAGACAGAACTCTTTTTAG
CTGTGGGAACTTGGGTACATTGCTTGCTTTTCCTCCTCGTAAAATNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNN
```

```
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNGGGGGGCGCGGAAGA
GGGCGGGGGGCGAGGGGGGGGTCGGTGGGGAGAGAACCCCCGAGGGGGGCCCCGGGCGAGCAGAGTGTCGGGGAGGTGCT
TGTGGCGGTTGTTATGTATGAGAGCGAGCCCAACGTGACAGTTACGAGAGCTGGTTGGGGGCGTGTGGGACAAAGTTGT
TCTGTGCACAGACTGGGAGGAACCGCCAAGGTCTAGTGCACTGGGGGCTCGGACGCTGGGCGCCAAACAATCCGCCCGGG
TGGCGCTCCCAAAAGGGCGGGGGCCAAAGGTGTTGCCACCCTGGCCCGCAGAAGTTTTGTATTTACACCCACACACGC
GATTTATTTGCCCCGACCTATATGGGTACACACGCCATCCCTATATAGATGCGAGAGAGCGATCTCTCTCTGAAAA
AAAAAAAAAAAAAAAAAAAATATATATATATATATATATATATTTTTTTTTTTTTTTTTTTTTTTTTCCCTGAGATGGAG
TCTTGCCCTCTCGTCCAGGCTACTAGAGTGCAGTAGTGCAATCTTGGCTCACTGCAACCTCCACCTCCCATGTCCAAGTG
ATTCTCCAGCCTCAGCATCCCGAGTAGCTGGGATTACAGTACCTGCCACCAGGCCAACTAATGTTTGTATTTGTGTAGAG
ATGGGGTTTCACCATGTTGGCCAGGCTGGTCTTGAACTCTATCTAGAATATACAATATATCAAACATTTCAACACAAAAA
AAAAAATCTGACTTTTTTTTTTTGGAGATGTAATCTCAATCTGTCACCCAGGCTAGAGTGCAGTGGCATGATCTCAGCTC
ACTCCAACCTCTGCCTCCCAGGTTCAAGTGATTCTCCTGCCTCAGCCTCCTGAGTAGTTGGAACTACAGGCTTGTACCAC
CACGCCCAGCTAATTTTTGTATTTTTAGTAGAGTCGGGGTTTCACCGTGTTGGCCAGGCTGGTTTCAAATTCCTGACCTC
AAGTGATCGACCCGCATCGGCCTCCCGAAGTGCTGGGATTATAGGTGTGAGCCATCACGACTGGCCATTTTTTGTCTTTT
TGATGACATCTTGCTCTGTCATCCAGGCTGAGTGCAGTGGCGCAACTATAGCTCACTGCAGCCTCCAATTCCTGGGTTCA
AGCAATCCTCCCACATCAGCCTCCCAAGTGAGTAGCTGGGACCACCACTGTGCACCACCACACCTGGCTGTTTTTAACTT
TTTGTGCAGACAGGGTCTCGCTGTATTGCTCAGGCTGGTCTCGAACTCCTGGCTTCAAGCAACCCTCCTACCTCTGCTTC
CTAAAGTGCCAGGATTATAGGTGTAAGCCACCATGGCTGGCCAACAAT
```

HUMAN mRNA SEQUENCE : hR14-032.1 (Seq ID No: 133)
```
GTCCTGCGGCAGACGGAGCCGAGCCCCAACCAGGAAGGGAGTCCGAGCACTGGGACTTCAACGCCACCATCTCCAAGACT
CGGTTTGGGGTGAAAGATGGCGCTGACTGGCCACATTCCTGAATGTGGGGGCCGAGATCTCTATCACCCTGGAGCCTGCC
CAGCCGAGCGAAGGGGACAACGTCACGCTGGTCGTCCATGGGCTTTCGGGGGAACTGCTCGCCTACAGCTGGTATGCGGG
```

```
GCCCACACTCAGCGTGTCATACCTGGTGGCCAGCTACATCGTGAGCACAGGCGATGAGACTCCTGGCCCGGCCCACACGG
GGCGGGAGGCTGTGCGCCCCGATGGCAGCCTGGACATCCAGGGCATCCTGCCCCGGCACTCAGGCACCTACATCCTGCAG
ACCTTCAACAGGCAGTTGCAGACCGAGGTGGGCTACGGACACGTGCAGGTCCATGAGATCCTGGCCCAGCCCACAGTCTT
GGCCAACAGCACAGCGCTGGTGGAACGTCGAGACACCCTGCGCCTTATGTGCAGCAGCCCCAGCCCCACCGCCGAGGTCC
GCTGGTTCTTCAACGGTGGGGCCCTGCCCGTCGCTCTCCGCCTGGGCCTGTCCCCTGACGGCCGGGTGCTGGCCAGGCAT
GGCATCCGCCGGGAGGAGGCCGGCGCCTATCAGTGTGAGGTGTGGAACCCGGTCAGTGTCAGCCGCAGCGAGCCCATCAA
CCTGACCGTGTACTTTGGCCCAGAGCGTGTGGCCATCCTCCAGGATTCCACCACCCGCACAGGCTGCACCATCAAAGTTG
ACTTCAACACGTCCCTCACCCTGTGGTGCGTGTCCAGGTCCTGCCCAGAGCCCGAGTATGTGTGGACCTTCAACGGGCAG
GCCCTAAAGAACGGCCAAGACCACCTCAACATCAGCAGCATGACAGCCGCCCAGGAGGGGACGTACACATGTATTGCGAA
GAACACCAAGACCCTGCTATCTGGATCTGCCTCAGTCGTGGTCAAGCTCTCTGCTGCAGCAGTTGCCACGATGATCGTGC
CCGTGCCCACCAAGCCAACGGAGGGCCAGGACGTAACACTGACCGTGCAGGGCTACCCCAAGGACCTGCTGGTCTACGCC
TGGTACCGCGGGCCTGCCTCCGAGCCCAACCGGCTGCTCAGCCAGCTGCCGTCAGGAACCTGGATTGCAGGCCCCGCGCA
CACAGGCCGGGAGGTGGGCTTCCCCAACTGCTCGCTGTTGGTGCAGAAGCTGAACCTCACAGACACTGGCCGCTACACAC
TCAAGACCGTCACAGTGCAGGGCAAGACTGAGCACTGGAAGTGGAGCTGCAGGTGGCCCCCCTGGGGTAACAGCGTGAC
CCTGGAGACGTCCAGAGAGAAGAGCTCTTCGCACCATCCTCTGGTCCTCGCCCTCTGAGTGGGAACCACTCCCCCACAGC
GAGGATGCCAGGCTGTGGTCCTGCTGTTCTCCTGCCTCCACCCTAGAGCTAGAGCCACAGGGCCCCACTCCCTCGCTGAG
CTGTTGGGGAGCCACCGAGGCCATAAACGTCCTGGTTAA
```

HUMAN PROTEIN SEQUENCE : hP14-032.1 (Seq ID No: 134)
```
LATFLNVGAEISITLEPAQPSEGDNVTLVVHGLSGELLAYSWYAGPTLSVSYLVASYIVSTGDETPGPAHTGREAVRPDG
SLDIQGILPRHSGTYILQTFNRQLQTEVGYGHVQVHEILAQPTVLANSTALVERRDTLRLMCSSPSPTAEVRWFFNGGAL
PVALRLGLSPDGRVLARHGIRREEAGAYQCEVWNPVSVSRSEPINLTVYFGPERVAILQDSTTRTGCTIKVDFNTSLTLW
CVSRSCPEPEYVWTFNGQALKNGQDHLNISSMTAAQEGTYTCIAKNTKTLLSGSASVVVKLSAAAVATMIVPVPTKPTEG
QDVTLTVQGYPKDLLVYAWYRGPASEPNRLLSQLPSGTWIAGPAHTGREVGFPNCSLLVQKLNLTDTGRYTLKTVTVQGK
TETLEVELQVAPLG*
```

Table 19

MOUSE GENOMIC SEQUENCE : mD22-005 (Seq ID No: 135)
```
CTCCCTCCAGCCTCTTTCATCTGAACCGTGGCGCGCTCGTGCCTCTAGTTAATCAGCCTTCCAAGCCACACCAAGAGTCA
TTTCCCGGGGGTAGGCTCTGCTGGAGCCTGGAAACTAGGAGCTGCCTGCAGTCTTGGGGTCTCCTCTGCCTGTGAGCTGC
TCCTTCCAGTCCAAGGGTTAGGGCCAGAAAGGAGGACAACTTTGGAGTCAAAAGACTGACCACTGTGGTTAGCACCTGAG
AGAACCTTCAGTTTCTTCTCTTGCTACGCCACCCCCTTCGGGTACAAGTCCCAAAATGGTTTGCCAGTTTTCGTTCCCGT
AACTGGAGCGTGGGGAAGCCCCTCAACCCTTCCAGAGAGCCCATCCCGCCTCAGGACTTGAAGGCACACTTCACCAGGTG
AGTGCAGTTCGTGCAGTTCCCGGTGGCAACGCCTGGAGTCCCTCCCCCGCCCCCCACCCCGCCCCCTCCCCGCCTCCGGC
CGCTGTGGCGCAGACCTTGACTCACGAGCTGCAAGAGTCGTAAAGGGTCCCCAAGCCTCAGTGCCGGGTAAGCAAGGCAG
CGGGTCGCAGCCCCCTCCTGGGCGGGCCTTCGCGTCCCGCCCCCGGCCACCACTCGACACCCTCTGCCTGACGTCAAGGG
GTTGGGGGCGGAGAGCGGCGGTGGCGGCGGCTGCAGCGGAGAAAGAACCGGCGCCCGGCGCGGTGGAGCGCCGAATCCGA
GCTCAGGGGCGCCGAGAGCCGTGCTGAGACCGAGGGGCGGTGGCACGGGACAGCCTTAGCGTGCTGGCGGGCGGGCGCAG
TCTGGAGCGGCGCGGGGGCAGGGGAGGACGGTGGCCGGAGTCAAACGCGAGGGCAGCGCCCAGGGATTGGAGCTGCACGA
AAGAGGTGAGTGAGGCCGGAGCGGTGGGGCCCCGGGGCCGCTGCTTTGTCCCCTTGCTTGGGTAGGGGCTAGCGCGCGCG
CTAGAGGACAGCGTCTAGGCGTGCAGGGCGGGGCGACAGCAAAGACAGCTGTCCGATCCGGGTCCCTCTTGAGTCCGCCG
CGGGCTGCCCAGTGGCGATCGGCATCAAGGCAAGCAGTGCCTGCAGCTCCACTGGCCGGAGCAGCGGGAGCCTGGGGGTA
CGGACAGCGCTAGGGGCAGTGGGGGACCCTTGGGCTGTCCAGCGCGCGGTCCGAATCTTTGCCAGGCTCGCGAACTGCTC
GGGTTCCCCCCACCTCGACGGAAAGTTAGGGGCGGGCTTGGACCGTGGCCGATGTTGTGGGATCGGATTTCCCTAGCGCG
GCCCCCCGCCCTCCCGTTCTGCTCCGGAGGCGGGCTGGCACTGAGAACCAGCCCCCTAGCCAAAGCTCCGCCGCCCGGTC
CCTCTCTGGAGCGCCGCAATCCACGACCGGGTGTGCAAGTGTGGGGTGCAAATCCTGGTCCTGCACCCCTCCATCCCCTT
GGCTGTCCGTGTCCCTTGCTTCGGAAGAGCTTGGGGGTTGGGTCACCGCTACGCCTGCAGATTCTCGGAGCGCACCACC
GGGTTTTGACCCCAGCCTAGGGTAATTGGCAGAGAGGAAACTGAGGGTGTGTCAGGGGGGACTGCTCCGGGGAAGGCAGC
CCCAGCCCCCCGCCCTTCCCGCGCGGAGGTTCTTGGCCGACAGGGGGCGTCCGCAAGCCCGGCTTTGCCACGCTCCCAGC
GCTGCCGCTTTGTTTGCATTCAAATCCCCGGAGCTGGGGAGGGGAGAGTCCACAGTAGGGAGACCGAGACCGGGTCTTG
GCTAGGAACCTTTGTTTTGGGGTGCAATCCTCAGATTCTGCATCTCTGTTCCCAGCCCCTAGCGTCTAGATTAGTGGAGA
TCCGTTCCGTTTGCCTACTGCCTAGTGCATGGTCCCCGAGGTGCAATCCCGGAGTTAAGAACGTTCGTCAGAGCCGGGGA
GGAGGGGACGCAGCCCTTGACGATCATTTGTGGGCATCCCTGTTGTCCAGGAGAACCCCGCTTCGGCCTCCCCAACCCA
TTTCACTTCCCTCCACGATCCCGCTCTCGCGGAGGCGAGGAGATTCCCAGACAGAAGCCTGGAACTGACTCGTGGCCCGG
TCGCCTCTCTGCGGGAGGGTCAGTGTCTTGTAGAAGGCTGTTGGAGAAACAGGACTGGTCCTGGTCATTTCCACGCCCAT
CCCACCCACTTCCTGCCTCCCTCTTGGGCAACAAAGGGGCCTCGGAAGCTGGCCCATTTGGTTCCCGGCCCCACTCCTA
CTCCAGCCCTGCTGATGTGGAACCGGGTTTGGGCTCTGCCCGGCTATTGTCTGCGCTGGGGGAGGGGACGGGCTGGGGCC
GGGAACTCTACACGCTGCCTGCTTCACCAAGTCCCCCACTGAAGCTCTTTGCCCAACCGGCTGCCTGCGGCTGCTGAATA
AGGGGTATATCTCCCCGAATTAGGTCTTGGGGCTGGCCTGGGTCCCACTGGTAGCAAGCCCTGTTTTTTATGGTTATTGT
AAGAAACTCTGAAACCTTTAAGGCCAGAAGGAGCCAATGAGGGGGAGTCCCTCTTTTATTTTTTTCCACACAACTCCCTG
```

```
TTGAAGGGAGGGAGTAGGGATGGCCTGGAGGCCACAGGCGGCTTTTGATGACTGCCCACGCCTAGTGCTTGCTGACTAAG
TTCCTCTCCTTGCTCTTCTGGGAGACCCCCAGGGCTCTTGTGGGCAGAGGCCAGTTGCAGGATCAGGAGGAGATGTAAAG
GCTCTGGGGGAGGTTTGTTCCAGTGTGCGTCCTGTGTTGCTTGTCTCTTGGTGCGGGCCCCCTTCCGGTCTGTGATGCTG
ACCAGTTTCTTTTCCTTCAGCAGGGCTGCTGGACTGAAGTTTAGACCCTGGGTGTCTGCCATGGCCCCACACTGGGCTGT
CTGGCTGCTGGCAGCAGGGCTGTGGGGCCTGGGCATCGGGGCTGAGATGTGGTGGAACCTTGTGCCCCGGAAGACAGTAT
CTTCTGGGGGTGAGTGCCTGAAGCATGGGAGGCAGGGCCAGAGGGAGGCTGGATAGACTGCTGGGGGAAGGGCGAGGAG
GGGAGTGATGCTTGCTTGCAGGAAGGAGCTGCTGCTGAGGAATTACCCCCAAGCATGCACCTTTACACCGATATGTGTGG
CTGTGGCTTTCTAAGAAACGCGAGTGGCCCCAGGGGCCCAGAACTGGTAGTGCTGGTCTAGACCTGGGCTTTGCCCCCTT
CTGACACTGGGATAACTTTATGGGATCTCTTACCAGCGCACCCCCACCCTCACCCAGACCTGGCTGGCCTGGTGTGCAAG
GGAGGTAGTTTCTGAATCAACCTCCCTGTTCCTCACTTGAATTGTCTCTCTTGCTTGGAAGCCTAGAGAGAGAAACCTGA
ACCCCAGGGCAAGTGTAAACCTAACCCTATCTCCAGTCTTTCTAGCTGTCTGTCCCTAGAAGCCCCTTTAATTCACCCTA
ATGGTCTCCTCTGCTGCTCTAACACTGGAGCCTTCTGATGGCTCTGGGTGGACACATATAAGCTCGCGCTTCGTGGGGGA
GGAGACAGAGTGAACCAGACAGCTCTCCTTTGCTTTCGCGCACACCTGTATGGAGAGGTGATGTCAGGTGTTTGTATCAT
CTCTGTGCTCCTGGGACATGGGATGAGCTCACCTCCCACAGGCTACACTCAGCTGCTATTAAAGCCCCTCCCATGTCGAC
CCAACTCTCGTTGGTCACTGCCTCCCGACCCTGAAGCTGGCTTAAGTAGCGATCCTGTGAACAGGTGTGCAGGTATACGG
TCCTCTGACAGGTTACATGACTGTATGTATGATCACGCCCTACGTGTGCTGGCCGGAAGCACAACTTCCCAGGCTTCAGC
ACCACCTCTGAGTCTCAGGTTCCTTTTATCTCCCAGTCCTAACCTCTTCTTCCTTCTCCACACGCCACTTTGCCAGTCCC
CTGCCCATCTGTGCGTGTGTCTCTGAAAGCCTGGATTGGTGCCTGCTGGGACTTCTTAGACAAGGAATGCTTACAACTAT
TCTGTGCCTCCTGATGCAGTAAATCCGCCACCAGGGTCTGCCTAGACCTTGCCATGGTCAGCAAGTAGCAGAGCAGGAAA
TGGCAACCGAGGAAGCGGGGCTGGGGGTGGGGGGGAAGTGTCCAGTTCTGTGCCTGAGGTACCGCTGACCCTGAGGGAGC
GTCATCAGTCAGATGGCTTCCTCTGGCCGGAAGCTTTGGCTGAGGTATCCTTCCTTCCCCGTTACCAGCCCAGATTACCT
CCTCCCAGGCTGAGGAAGGGCCTTGGAAGCCTGGGTAGGATGGAGCTGTTGAGCCAAAGGCTATTCAGAAAAAGTCCTTT
ATGGCCCATAGAGGTTTGGTTGTCGTCTGTGTGTGTGTAGTGGTAGCTTTTGTGGAGAATCGTGATGACGAGGCCCAGGG
GAGCCTTGGACAAAGGAAGATTGATGGATTCCTGGAACCCCAGGGAGGATGTCCTCTGCCCCTACTCATCCCCACCTTGT
TCTAATATCTGTTTCTGCAGAGCTGGTCACAGTAGTGAGGCGGTTCTCCCAGACAGGCATCCAGGACTTCCTGACACTGA
CCCTGACAGAACATTCTGGCCTTTTATATGTGGGGGCCCGAGAGGCGCTGTTTGCCTTCAGTGTAGAGGCTCTGGAGCTG
CAAGGAGCGGTGAGAGGTGGGGCAGTGGGAGGCACTCAGAGGGGAGAGGGAAAGGACTCAGGGTGTAGCCTGTGCAGGCT
GTCCCTCAGCCCAGCTCTCTTGGGGTGTCAAGATTAGCAATTGCTTTCTGCTCCTGGTTTCTGCTAGTTGGGGTGGTTTG
GGGCTTCTGTCAGAATGGGGACCAGGCGTCATCAATGGTGTAGCTTAAGGGGTGTTTGCAGCACACTTAGCGCAGACCTG
TGACATTGCTGATGTGTCTGTCCAATCCTCAGATCTCTTGGGAGGCTCCAGCTGAGAAGAAAATTGAATGTACCCAGAAA
GGGAAGAGCAACCAGGTGGGTGCTTAGAGAGCTTCCCCTGCCAGCCTCCTTCTTTAATCTGCACCCTGGTGATCTCCTAT
CTCTGACTCTCAGTTCTCACTAACCTGTATACCTTTCTCTCCAAAGACCGAATGCTTCAACTTCATCCGCTTCCTTCAGC
CATACAATTCCTCCCATCTGTATGTCTGCGGCACCTATGCCTTCCAGCCCAAGTGCACCTACATCGTGAGTGGTTCCCCC
TCTCTTTGGGTGTGCCCCTTACCCACCTGTTCCTAACCTATGACCTCCTTCCTTGACCTCTCGGGGCCATGATATTTTCA
GACTGTATAGAGCCCCATAGTAACACCCATCTTCGATGTCTTCCTGCCCCTAGAACATGCTCACGTTCACCTTGGACCGT
GCAGAATTTGAGGATGGGAAGGGTAAATGCCCATATGACCCAGCTAAGGGTCACACCGGACTCCTTGTGGGTGAGTGGCT
GCTGCCAGCACAGAGGGGAGAATCTGGGCCTCTGTCAGTCACGCTCTGAGTACTTGTCTTCAGAGCAGTCTCTACTTTAG
TTCCCTGGCCTCTCTGACCGCTGTTGTCTCCCGCAGACGGTGAGCTGTACTCAGCCACACTCAATAACTTCCTGGGCACA
GAGCCGGTTATCCTTCGATACATGGGGACCCACCACTCCATCAAGACAGAGTACCTGGCTTTTTGGCTGAATGGTGAGTC
ATGGGATGGGACTGGGGAAGCCCAGAGTGGAAGGGCCTTCCAGCGCCCTGGGACTCTTGTCACTGGCCCTTTTTTCTTCG
TCCCAGAACCCCACTTTGTAGGCTCTGCCTTTGTCCCTGAGAGTGTGGGAAGCTTCACGGGAGACGATGACAAGATCTAC
TTCTTCTTCAGTGAGCGGGCAGTGGAGTATGACTGCTATTCCGAGCAGGTGGTGGCTCGTGTGGCGAGAGTCTGTAAGGT
ACCAAGTTCCGCAGTGGTGGGCAGCGGGAGCCCTGAAGGCCTGGCAGGGGCTGACCAGCCTTGTCTCCTTGCCATCTGCC
AGGGTGACATGGGGGGAGCACGGACGCTGCAGAAGAAATGGACGACGTTCCTGAAGGCTCGGTTGGTGTGCTCAGCCCCT
GACTGGAAGGTCTACTTCAACCAGCTGAAGGCGGTGCACACCCTGCGGGGCGCCTCTTGGCACAACACCACCTTCTTCGG
GGTTTTTCAAGCGCGATGGTAAGCGGCTATGGGGTAGCTGGGGGTAGGGAGGAGCAGGTGGTCAGGTAGGGATAGTGTGT
GGGTGTGAGGGCCCCGCACAGGCGTCTGGAGGAAGTCCAGCGGGTCAGTGCCTGGCAGCCAAGATGACTTTGCGCCTGC
TCTTTCTAAAGCTGGGTGGGGGAAGGTGAGCAGAAAGCTGCCTGGGGTGACACCTCAGGGCTACCTCATGGTCGTGACTG
CTCCATACTAAGCTTTGCCCTTCCTACTTTTTCCTCTGTTCCCTGGTATGGTGGGAGCAAGTAGGCACCCAACCTGACTT
CCAAGGTCCTTCTTTAACAGGGGCGATATGGACCTGTCTGCAGTTTGTGAGTACCAGTTGGAACAGATCCAGCAAGTGTT
TGAGGGTCCCTACAAGGAGTACAGTGAGCAAGCCCAGAAGTGGGCCCGCTATACTGACCCGGTACCCAGCCCTCGGCCTG
GTTCGGTGAGTTTTCGGCTTGGGGATGTATTCCCAACATCGGCTCCAGGACCCACTTATTCGCCAGCTGACTGACAGCCG
CCCTCCCCACGCCCCTCCCCGACACAGTGTATCAACAACTGGCACCGAGACAATGGCTACACCAGTTCCCTGGAACTGCC
GGACAACACCCTCAACTTCATCAAGAAGCACCCCCTGATGGAGGACCAGGTGAAGCCTCGGTTGGGCCGCCCCCTACTTG
TGAAGAAGAACACTAACTTCACACACGTGGTGGCCGACAGGGTCCCAGGGCTTGATGGTGCCACCTATACAGTGTTGTTC
ATTGGTACAGGTAGGCGCTTCAGGTGTGCCCAAGGCTGGGAACCTGGGGAGGCCCCATAGCCTGATTCCTCATTTGCATA
TTCCCATCACCTCTCCAGGAGATGGCTGGCTGCTGAAGGCTGTGAGCCTGGGGCCCTGGATCCACATGGTGGAGGAACTG
CAGGTGTTTGACCAGGAGCCAGTGGAAAGTCTGGTGCTGTCTCAGAGCAAGGTAGTAAGACTGGGCCACACGCACCTCCC
TCTCTGTTCTCTGCTCCATTGTCTATCTGGCGCCCAGGCTCTATGCCATCCATAGTTGGGCTCATGTAGCTCTCGAGTTC
CCAGAAGTAAGAGATCTAAGGTGGCCAGAACCTGTCCTCTGAAGGTTCTCTTATAGAGCCAGTCCCTGTGGGGCCCCCAT
TCCCAGAATCCAGCCTTGGCAAACTCCTTCCAAGTGGGGCTGCAGCTCTATCTGCAGTCTCTTCTCGGTCAAGATGTTTT
```

GGTACCCAAGCAACAGAGACATCCGATTTCTTTGAGTCCCTAGTGGGTGTCTGAGCCAGGCCCTCAGTCTCCGGGCTCAT
TTAAGTGTTCACGTTTTTTCTTTTCTTTCTTTCTTTCTTTTTTTAACACCTTTATGCAAAACTCATATTCGCATCACCCT
CTGGATTCTGATTTGCTTGCCCTTTACCAGCTACTATGACTCAAAGTTGCTCCCTACAACGCTGAATAATGAGATGGCAG
CAAGGGAATTGTGGGGCTGAGCAAGCATGCAGATTGGCAGTCACCATTCAAACAGAGGAACCCCACCTTCCATAGGCACT
GCTGTCACTGTGCTGGCTCCGGGGGCCGGGGGAAACTGAGTTTCCTGGCCCGAGTGTGAGCAAGTGAGCGTACGCCTCAG
CCTCACGGGTCTGCCGTCCGTCCCTGTCTAAGAAGGTCATGTCTGTAAAGTGATTAGGATTTTGATGGCTTGTCGTAAGC
CCTCATCAAAATTGTTCCTCTGGCCTGGATTTTTCTTCCGCTGCCTTTTTCTTCCTTGGGGGACCTAGTATGTTTATGCT
CTCTGAACAAATCTCAGCCATCACCCCGAGGCGTGGTGTGGCTTACCCTGTGCTTTGTTTTACTGTAGAAGGTGCTCTTT
GCTGGCTCCCGCTCTCAGCTGGTTCAGCTGTCTCTGGCCGACTGCACAAAGTACCGTTTCTGTGTAGACTGTGTCCTGGC
CAGGGACCCTTACTGTGCCTGGAATGTCAACACCAGCCGCTGTGTGGCCACCACCAGTGGTCGCTCGGGGTGAGTCAGTC
GTCTCTGAGTGCCAGGGCCAGGAGGGGGAGGCTAAGGTGATTGGAGTTGGATCTCTGATGCCACTTCTCTTCCCTACCAG
GTCCTTTCTGGTCCAACATGTGGCGAACTTGGACACTTCAAAGATGTGTAACCAGTATGGCATTAAAAAAGGTGAGCTGT
TTTCCTTTTGTTCCCTCATTTTGAGGATGGGCCCCTGGATCACAGCCAGAGTTAGTTTATCACCTCTCACTGTGGGACCT
GGGGTTCCTGCATTAACACTGGGTTCTCTCTCATTTCTGCCTCTGCAGTCAGATCTATTCCCAAGAACATCACCGTTGTG
TCAGGCACAGACCTGGTCCTACCCTGCCACCTCTCGTCCAATTTGGCCCATGCCCACTGGACCTTCGGAAGCCAGGACCT
GCCTGCAGAACAACCTGGCTCCTTTCTTTATGACACGGGACTCCAGGCGCTGGTGGTGATGGCCGCACAGTCCCGTCACT
CTGGACCCTATCGTTGCTATTCAGAGGAGCAGGGGACAAGACTGGCTGCAGAAAGCTACCTTGTTGCTGTCGTGGCCGGC
TCGTCGGTGACACTGGAGGCACGGGCTCCCTTGGAAAACCTGGGGCTCGTGTGGCTCGCTGTGGTGGCCCTGGGGGCTGT
GTGCCTGGTGCTGCTGCTGCTGGTCCTATCGCTCCGCCGGCGACTTCGAGAAGAGCTAGAAAAGGGTGCCAAGGCATCTG
AGAGGACACTGGTGTACCCCTTGGAACTGCCCAAGGAGCCTGCCAGTCCCCCCTTCCGTCCTGGCCCCGAAACTGATGAG
AAACTTTGGGATCCTGTCGGGTACTACTATTCGGATGGCTCTCTCAAGATTGTGCCTGGTCACGCCCGGTGCCAGCCTGG
GGGTGGGCCCCCTTCCCCACCTCCTGGCATACCTGGCCAGCCTCTGCCTTCTCCAACTCGGCTCCACCTAGGAGGTGGTC
GGAACTCAAATGCCAATGGTTATGTGCGTTTACAGTTGGGCGGAGAGGACCGAGGAGGATCTGGGCACCCACTGCCTGAG
CTCGCGGATGAATTACGACGGAAACTACAACAGCGCCAGCCGCTGCCTGACTCCAACCCAGAGGAGTCTTCAGTATGAGG
GGACCCCCCCACCTCATTGGCGGGGGGGGTCTCATGGGAGGTGCACTCTTAACTTTTGCACAGGCACCAGCTACCTCAGG
GACATGGCAGGGGCACTTGCTCTGCCTGGGACAGACACTGCCCAGCATTTGCCCGGCCGTGAGGACCTGCTCAGCATGGG
CACTGCCACTTGGTGTGGCTCACCAGGACTTCAGCCTCACAGGAGACACACCCTCCTCTGTGAATTTGAGACATGTGGGA
CCCCAGCAGCCAAAACTTTGCAAGGAAGAGGTTTCAAGATGTGGGCGTGTTTGTGCATATATGTGTTGGTATGCATGTGG
AAGAATGTGTGTGTGTGTGTGTGTGTGTTGTAACTTTCCTGTCTCTATCACGTCTTCCCTTGGCCTGGGGTCCTCCTGGT
TGAGTCTTTGGAGCTATGAAGGGGAAGGGGGTCATAGCACTTTGCTTCTCCTACCCCCAGCTGTCCCAAGCTTTGGGGCA
GTGATGTACATACGGGGAAGGGAAGGACAGGGTGTTGTACCCCTTTTGGGGGAGTGCGGGACTCGGGGGTGGGCCTAGCC
CTGCTCCTAGGGCTGTGAATGTTTTCAGGGCGGGGGTTGGGGGTGGAGATGGAACCTCCTGCTTCAGGGGGAGGGGTGGG
CAGGGCCTCCCACTTGCCCTCCGGGTTCGGTGGTATTTTATATTTGCGCTCTTCTGACAGGGCTGGGAAGGGTTGTTGGG
GGAGGGAAGGGAGGAGGTGGGCATGCTATGGATACTGGCCTATCCTCTCCCTGCTCTGGGAAAAGGGCTAACAGTGTAAC
TTATTGTGTCCCCACATATTTATTTGTTGTAAATATTTGAGTATTTTTATATTGACAAATAAAATGGAGAAAATGAAATT
T

MOUSE mRNA SEQUENCE : mR22-005.1 (Seq ID No: 136)
CTCCCTCCAGCCTCTTTCATCTGAACCGTGGCGCGCTCGTGCCTCTAGTTAATCAGCCTTCCAAGCCACACCAAGAGTCA
TTTCCCGGGGGTAGGCTCTGCTGGAGCCTGGAAACTAGGAGCTGCCTGCAGTCTTGGGGTCTCCTCTGCCTGTGAGCTGC
TCCTTCCAGTCCAAGGGTTAGGGCCAGAAAGGAGGACAACTTTGGAGTCAAAAGACTGACCACTGTGGTTAGCACCTGAG
AGAACCTTCAGTTTCTTCTCTTGCTACGCCACCCCCTTCGGGTACAAGTCCCAAAATGGTTTGCCAGTTTTCGTTCCCGT
AACTGGAGCGTGGGGAAGCCCCTCAACCCTTCCAGAGAGCCCATCCCGCCTCAGGACTTGAAGGCACACTTCACCAGGTG
AGTGCAGTTCGTGCAGTTCCCGGTGGCAACGCCTGGAGTCCCTCCCCCGCCCCCACCCCGCCCCCTCCCCGCCTCCGGC
CGCTGTGGCGCAGACCTTGACTCACGAGCTGCAAGAGTCGTAAAGGGTCCCCAAGCCTCAGTGCCGGGTAAGCAAGGGGT
TGGGGGCGGAGAGCGGCGGTGGCGGCGGCTGCAGCGGAGAAAGAACCGGCGCCCGGCGCGGTGGAGCGCCGAATCCGAGC
TCAGGGGCGCCGAGAGCCGTGCTGAGACCGAGGGGCGGTGGCACGGGACAGCCTTAGCGTGCTGGCGGGCGGGCGCAGTC
TGGAGCGGCGCGGGGGCAGGGGAGGACGGTGGCCGGAGTCAAACGCGAGGGCAGCGCCCAGGGATTGGAGCTGCACGAAA
GAGGGCTGCTGGACTGAAGTTTAGACCCTGGGTGTCTGCCATGGCCCCACACTGGGCTGTCTGGCTGCTGGCAGCAGGGC
TGTGGGGCCTGGGCATCGGGGCTGAGATGTGGTGGAACCTTGTGCCCCGGAAGACAGTATCTTCTGGGGAGCTGGTCACA
GTAGTGAGGCGGTTCTCCCAGACAGGCATCCAGGACTTCCTGACACTGACCCTGACAGAACATTCTGGCCTTTTATATGT
GGGGGCCCGAGAGGCGCTGTTTGCCTTCAGTGTAGAGGCTCTGGAGCTGCAAGGAGCGATCTCTTGGGAGGCTCCAGCTG
AGAAGAAAATTGAATGTACCCAGAAAGGGAAGAGCAACCAGACCGAATGCTTCAACTTCATCCGCTTCCTTCAGCCATAC
AATTCCTCCCATCTGTATGTCTGCGGCACCTATGCCTTCAGCCCAAGTGCACCTACATCAACATGCTCACGTTCACCTT
GGACCGTGCAGAATTTGAGGATGGGAAGGGTAAATGCCCATATGACCCAGCTAAGGGTCACACCGGACTCCTTGTGGACG
GTGAGCTGTACTCAGCCACACTCAATAACTTCCTGGGCACAGAGCCGGTTATCCTTCGATACATGGGGACCCACCACTCC
ATCAAGACAGAGTACCTGGCTTTTTGGCTGAATGAACCCCACTTTGTAGGCTCTGCCTTTGTCCCTGAGAGTGTGGGAAG
CTTCACGGGAGACGATGACAAGATCTACTTCTTCTTCAGTGAGCGGGCAGTGGAGTATGACTGCTATTCCGAGCAGGTGG
TGGCTCGTGTGGCGAGAGTCTGTAAGGGTGACATGGGGGGAGCACGGACGCTGCAGAAGAAATGGACGACGTTCCTGAAG
GCTCGGTTGGTGTGCTCAGCCCCTGACTGGAAGGTCTACTTCAACCAGCTGAAGGCGGTGCACACCCTGCGGGGCGCCTC
TTGGCACAACACCACCTTCTTCGGGGTTTTTTCAAGCGCGATGGGGCGATATGGACCTGTCTGCAGTTTGTGAGTACCAGT

456

```
TGGAACAGATCCAGCAAGTGTTTGAGGGTCCCTACAAGGAGTACAGTGAGCAAGCCCAGAAGTGGGCCCGCTATACTGAC
CCGGTACCCAGCCCTCGGCCTGGTTCGTGTATCAACAACTGGCACCGAGACAATGGCTACACCAGTTCCCTGGAACTGCC
GGACAACACCCTCAACTTCATCAAGAAGCACCCCCTGATGGAGGACCAGGTGAAGCCTCGGTTGGGCCGCCCCCTACTTG
TGAAGAAGAACACTAACTTCACACACGTGGTGGCCGACAGGGTCCCAGGGCTTGATGGTGCCACCTATACAGTGTTGTTC
ATTGGTACAGGAGATGGCTGGCTGCTGAAGGCTGTGAGCCTGGGGCCCTGGATCCACATGGTGGAGGAACTGCAGGTGTT
TGACCAGGAGCCAGTGGAAAGTCTGGTGCTGTCTCAGAGCAAGAAGGTGCTCTTTGCTGGCTCCCGCTCTCAGCTGGTTC
AGCTGTCTCTGGCCGACTGCACAAAGTACCGTTTCTGTGTAGACTGTGTCCTGGCCAGGGACCCTTACTGTGCCTGGAAT
GTCAACACCAGCCGCTGTGTGGCCACCACCAGTGGTCGCTCGGGGTCCTTTCTGGTCCAACATGTGGCGAACTTGGACAC
TTCAAAGATGTGTAACCAGTATGGCATTAAAAAAGTCAGATCTATTCCCAAGAACATCACCGTTGTGTCAGGCACAGACC
TGGTCCTACCCTGCCACCTCTCGTCCAATTTGGCCCATGCCCACTGGACCTTCGGAAGCCAGGACCTGCCTGCAGAACAA
CCTGGCTCCTTTCTTTATGACACGGGACTCCAGGCGCTGGTGGTGATGGCCGCACAGTCCCGTCACTCTGGACCCTATCG
TTGCTATTCAGAGGAGCAGGGGACAAGACTGGCTGCAGAAAGCTACCTTGTTGCTGTCGTGGCCGGCTCGTCGGTGACAC
TGGAGGCACGGGCTCCCTTGGAAAACCTGGGGCTCGTGTGGCTCGCTGTGGTGGCCCTGGGGGGCTGTGTGCCTGGTGCTG
CTGCTGCTGGTCCTATCGCTCCGCCGGCGACTTCGAGAAGAGCTAGAAAAGGGTGCCAAGGCATCTGAGAGGACACTGGT
GTACCCCTTGGAACTGCCCAAGGAGCCTGCCAGTCCCCCCTTCCGTCCTGGCCCCGAAACTGATGAGAAACTTTGGGATC
CTGTCGGGTACTACTATTCGGATGGCTCTCTCAAGATTGTGCCTGGTCACGCCCGGTGCCAGCCTGGGGGTGGGCCCCCT
TCCCCACCTCCTGGCATACCTGGCCAGCCTCTGCCTTCTCCAACTCGGCTCCACCTAGGAGGTGGTCGGAACTCAAATGC
CAATGGTTATGTGCGTTTACAGTTGGGCGGAGAGGACCGAGGAGGATCTGGGCACCCACTGCCTGAGCTCGCGGATGAAT
TACGACGGAAACTACAACAGCGCCAGCCGCTGCCTGACTCCAACCCAGAGGAGTCTTCAGTATGAGGGGACCCCCCCACC
TCATTGGCGGGGGGGGTCTCATGGGAGGTGCACTCTTAACTTTTGCACAGGCACCAGCTACCTCAGGGACATGGCAGGGG
CACTTGCTCTGCCTGGGACAGACACTGCCCAGCATTTGCCCGGCCGTGAGGACCTGCTCAGCATGGGCACTGCCACTTGG
TGTGGCTCACCAGGACTTCAGCCTCACAGGAGACACACCCTCCTCTGTGAATTTGAGACATGTGGGACCCCAGCAGCCAA
AACTTTGCAAGGAAGAGGTTTCAAGATGTGGGCGTGTTTGTGCATATATGTGTTGGTATGCATGTGGAAGAATGTGTGTG
TGTGTGTGTGTGTGTTGTAACTTTCCTGTCTCTATCACGTCTTCCCTTGGCCTGGGGTCCTCCTGGTTGAGTCTTTGGAG
CTATGAAGGGGAAGGGGGTCATAGCACTTTGCTTCTCCTACCCCCAGCTGTCCCAAGCTTTGGGGCAGTGATGTACATAC
GGGGAAGGGAAGGACAGGGTGTTGTACCCCTTTTGGGGGAGTGCGGGACTCGGGGGTGGGCCTAGCCCTGCTCCTAGGGC
TGTGAATGTTTTCAGGGCGGGGGTTGGGGGTGGAGATGGAACCTCCTGCTTCAGGGGGAGGGGTGGGCAGGGCCTCCCAC
TTGCCCTCCGGGTTCGGTGGTATTTTATATTTGCGCTCTTCTGACAGGGCTGGGAAGGGTTGTTGGGGGAGGGAAGGGAG
GAGGTGGGCATGCTATGGATACTGGCCTATCCTCTCCCTGCTCTGGGAAAAGGGCTAACAGTGTAACTTATTGTGTCCCC
ACATATTTATTTGTTGTAAATATTTGAGTATTTTTATATTGACAAATAAAATGGAGAAAATGAAATTT
```

MOUSE PROTEIN SEQUENCE : mP22-005.1 (Seq ID No: 137)
MAPHWAVWLLAAGLWGLGIGAEMWWNLVPRKTVSSGELVTVVRRFSQTGIQDFLTLTLTEHSGLLYVGAREALFAFSVEA
LELQGAISWEAPAEKKIECTQKGKSNQTECFNFIRFLQPYNSSHLYVCGTYAFQPKCTYINMLTFTLDRAEFEDGKGKCP
YDPAKGHTGLLVDGELYSATLNNFLGTEPVILRYMGTHHSIKTEYLAFWLNEPHFVGSAFVPESVGSFTGDDDKIYFFFS
ERAVEYDCYSEQVVARVARVCKGDMGGARTLQKKWTTFLKARLVCSAPDWKVYFNQLKAVHTLRGASWHNTTFFGVFQAR
WGDMDLSAVCEYQLEQIQQVFEGPYKEYSEQAQKWARYTDPVPSPRPGSCINNWHRDNGYTSSLELPDNTLNFIKKHPLM
EDQVKPRLGRPLLVKKNTNFTHVVADRVPGLDGATYTVLFIGTGDGWLLKAVSLGPWIHMVEELQVFDQEPVESLVLSQS
KKVLFAGSRSQLVQLSLADCTKYRFCVDCVLARDPYCAWNVNTSRCVATTSGRSGSFLVQHVANLDTSKMCNQYGIKKVR
SIPKNITVVSGTDLVLPCHLSSNLAHAHWTFGSQDLPAEQPGSFLYDTGLQALVVMAAQSRHSGPYRCYSEEQGTRLAAE
SYLVAVVAGSSVTLEARAPLENLGLVWLAVVALGAVCLVLLLLVLSLRRRLREELEKGAKASERTLVYPLELPKEPASPP
FRPGPETDEKLWDPVGYYYSDGSLKIVPGHARCQPGGGPPSPPPGIPGQPLPSPTRLHLGGGRNSNANGYVRLQLGGEDR
GGSGHPLPELADELRRKLQQRQPLPDSNPEESSV*

MOUSE PANTHER CLASSIFICATIONS
        FAMILY (SUBFAMILY)
                SEMAPHORIN(SEMAPHORIN 4B)
        BIOLOGICAL PROCESS
                Signal          transduction(2.11.00.00.00)          >          Cell
communication(2.11.03.00.00)
                Developmental       processes(2.23.00.00.00)          >          Ectoderm
development(2.23.08.00.00) > Neurogenesis(2.23.08.01.00)
        MOLECULAR FUNCTIONS
                Signaling    molecule(1.02.00.00.00)    >    Membrane-bound    signaling
molecule(1.02.07.00.00)

MOUSE GENE ONTOLOGY
        BIOLOGICAL PROCESS
                ectoderm development > neurogenesis
                apoptosis > anti-apoptosis
                defence response > immune response

cell communication > cell adhesion
MOLECULAR FUNCTION
    defense/immunity protein > immunoglobulin
    B cell receptor > immunoglobulin
    GO molecular function > cell adhesion
CELL COMPONENT
    cell > membrane fraction
    extracellular > extracellular space
MOUSE PROTEIN DOMAINS (INTERPRO SIGNATURES)
    IPR003659 (PSI)
    IPR003599 (IG)
    IPR001627 (Sema)
    IPR002165 (PSI)
    IPR000694 (PRO RICH)

HUMAN GENOMIC SEQUENCE : hD22-005 (Seq ID No: 138)

```
GCTGGAGAAAGAGCCGGCGCCCGCCGTGGTGGCGCGGGGAGCCCGAGCCTAGGGGCGCGGAGCCGGGCGGGGACGGAGGG
GCGGTGGCAGAGAGGCCGCGGAGGGCTGGCGGGCGAGCGCGGGCAGGCGGCGACGCGGGGGCAGGGGTGGACGGCGGTCA
GAGCCGAACGCGAGGGCGGCGCCCGGGGACTGGAGCTGCGCGCAATAGGTGAGCGAGGCCGGGNNNNNNNNNNNNNNNNNN
NNNNNNNNNNNNNNNNNNNNNNNNNNNNNNNNNNNNNNNNNNNNNNNNNNNNNNNNNNCCGCTTTGTCCCTTTGCTTGGGCA
GGGGCTGCCGGACGCGGGAGGGACAGGGGCTGGGGCGCGGGGAGCCGCGGCGGCGGAGACGGCTGTACGCGGGGATCCTT
CCTGCGACCCCCGCGGCTGCTTCGCAGGGTCTCGGGAGTGGGGCGCACAGCACCTGCAGCCCCGCAGCCCCGGGGCCGGA
GGGGCGCGCCAGCGAGGGTTCCTCGCGCGGTCCGGTCCGCGGCCGGGATCCTCGCCAGGCTCCTGGACTGCCCTCGGCGC
TCCCACCCCCGATGGAAAGTTTGGGGCGGGCTTGGAGGGAGGCCGATGTTGTGGGATCGGATTTCCCTGCCGCGGCCCCC
CGCCCGCCCGCCCATTCCGCTGGGGAGGCGGGTTGTCCCGGAAAGCCAGGCCCCCGGGCCGAGCCGGGCCGCGCCGTCCC
CGGCTGGAGCGCTCGCCCTGCAGGGAGGGCCGCGGTCCACGGCTGGCCGCTCGGGGTGGGCGAGGGTGTGGGGTGCCCCG
CCTGGATCCCGCACCCCTCCCCCCAACCCCTCCTCCCGGCTGCCCGTTTCCTCGCCCTGGGGGGAGCTGCGGGTGGGAGC
ACCGCTGCCGGGCGGATCCTCGGAGCACGCCACGGGGTGCGGCCCGATCCTCGGGTAAGGAGCCGAGATGAGGCCGCGGG
CGTTTTGGGGGGCTGGTCTGGGCGCAGCCCCCGCTCTCCCGCGCCGGGGCTCTGGGCCGACAGGGGCGTCCGCAGGCC
GGGCTCGCCACGCTCCCCGCGCCGCCGCTTTGTTTGCGTTCAAATCCCTGGAGCTGGGGGAGGGGAGAACCCGGGGGGGAC
CCGGGACGGGGTCTTGGCGGGGACCCTTTGTTTGGGGGTGAGCAGGTCCCCAGCTTCTGCGTCTCCGCCCCTGGCACGGG
AAGGCTGGAGCAGGGGAAGCCAATCCCGCTGGCCCGCCCAGGCCAGGGTCCCTGGGGCGCAGTCCCCGAGTTGGGAAGGT
TCGTCAGAGTCGCGGGAGCGCAGCGGGTGCCGGCCGTGCGCGGCGCGTTGCCAGCGGCCCGGGAGGAACCCCCTCGCGTC
CCCGCCCCTACCTCCCCCCGTCGCCTGCGCGCGCCGCCGGAGTCCCCGACTCGACCCGAGCTCGGTCCCCCCACCGCCCC
TCCGCCAGAGGGCTGGTGTTTCGGAGGAGGCAGTGCGAGGAACAGGATGAGGCCCTGGTCATTTCCACGCCCATCCCACC
CACTTCCTGCCTCCCTCTTGGGCAACAAAGGGGCCTCGGGAAGCTGGGCCCCTTCGCTCCCGGCCCTTCTCACACTCCTG
CCCTGCTGATGTGGAACGGGGTTTGGGGTTCTGCAGGGCTATTGTCTGCGCTGGGGAAGGGGACAGGCCGGGACCGGGAC
CTCCGCTCGCAGCCGGCCGCACCAGGTTTCCCCTTTGGGGCTTTCCCGACCGCCTACCCCACCGCATGCCCTCGTCGCCC
TAAGGAGTCTTCCTCCCAGAATTAGGTGTTGGCAGGGTCCGGAGCCCCAGTGGCTGCAAGGCCTGCTGCCTGAGGTTCTT
TCAAGAAACTCAAACCTCTTAGGCCTGAGTGTGTATGTTGGGCGGGGGTCCCCTTTTTATTTCTCAAATGATTTCCTGTT
GCGCAGAGGTAGTGGTGGGTCTGGAGGCCAGGGAGGGCTTCCCGGAGCCTGTTTAGCCTTCAGCCAACTCAACTCCTCCC
CGCTTCCCAGGGAGACCTGTGGTCTTTTAGGCAGAGGCCAAGTGTGGGGACTTAGGTCCACCTCCAAAGAGAAGGGGAAG
GAGGGCACCGGGGCTCCTGGAAGGCCTGATGAGGAGTCCTGTGGCCTCTCCTGCTGCGGGCCCCTCTGGTTTGCTTTCTC
TGGCTGTGATTTCTGACCATGTCTTTTCCCTCAGCAGGACAGCTGGCCTGAAGCTCAGAGCCGGGGCGTGCGCCATGGCC
CCACACTGGGCTGTCTGGCTGCTGGCAGCAAGGCTGTGGGGCCTGGGCATTGGGGCTGAGGTGTGGTGGAACCTTGTGCC
GCGTAAGACAGTGTCTTCTGGGGGTGAGTGCCTGGAGGGGTGAGGAGGAAGTCAGGGACAGGCTGCTGAGGAGTTGCCCC
CATAGTGTGCTGGCTTTATTCTCACGTGTGTGGCACAGCTTTCTAAGAAACTCGAGTTGGAAGAACGGGCAGTGTGGGTT
GGGACCTGGGATTAGCCTCCTCTGGGGGAGCTCTGTCCAACTCCCCACCCCAGACTGCCTGCCCAGGTGTGGGAGGGGGA
GAGCCTTCTGCCTCGCCCTCTCCACGCCTCGCTTCTTGAATTGTCACTGGCGCTGGGAGCCTAAAGAGAAACTGGAACCG
GGATAAATATAAACTGGAGGCTCTCTCCCCTGGCTCCCCAGGCCCCCCTAAGACCTCCTACTGCCCTGTACACATCATAC
ACTCTGGTGGCCCTGGAAGTGTGTGTGTATGTGCACATGCTCGCATGGTTGCCGGGGCACAGCGGGGACCCAGCAGTGCT
CCCTGTGCACACACCCGTGTGAAAGGTGATGTCAGGTGTTTGTATCAGCCGTGCGCTCTGGGGACCTGGGATGAGCTCAC
CTCCCACAGGCTCCACTCGGGTGCCAGTGAAGCCCTGCCATGCCGGTCTGTGTCTGGATCCATTGTTGCCTGCAAAGACT
AAGGCTGGTTTGGGGGGACCCTGAGGACAGGTCGGCAGCCATATTTGGCAGCTGATGTAGGAATCACGTGACTGTATAAA
TGTCGTAGTGCCCTGCATGCCGGGTGGCAGCAAGACTTCCCAGGCCCCAGCCCCATCTCCGCTCTCTGAGCCTCTGGTTT
CTCCGGACCTCCCTGGCCTGGCTCACAGCCCAGCTCCCCTCCTGCCATCCCCACCTGTCCTAGTGGCCTGCTGTGCTCGC
ATCTCTGGCAGCCTGGATTGGGGTCCTGCTGGAGTGAAGGGTTTCCTGAGGCAGGGAGTACTCATAGCCGTCTGTGCCTC
CTGCTCTGCTGGACTCTGATAGAGGCAGTGGGCAGCAGGGCAAAGAAGGAAGCAGCCGCCGAGGAAGCGAAGGTGGGGTG
GGGGTGCCCAGCCCTGCGGCCGGAGGTGCCATTGACCCTGAGAGCAAGAGCCATCAGTCTGATGGCTTCCTCTGGCCGGA
AGCTGTGGCTGAGATATCCTTCCTTCCTCCCAGCAGCCTGGATTTGCCTCCTCCCAGGCTGGGAAAGGCTAGGGAGGCCC
AGGTGGGGCTGTCAGGGCCAAAGGCTGTTTCAAAAGTGTCCTTTATTGCCAATCAGGGGGTTTGGCTGGCATCTGCTTGT
```

```
GGCATGGGGTGCTGGCTCTCGTGGAGCAAGACTGAGGGCTGTCGAGCCCCAGGGGTCCTGCCCATCAGCAGCCATGCTTA
CCCCTGCAGAGCTGGCCACGGTAGTACGGCGGTTCTCCCAGACCGGCATCCAGGACTTCCTGACACTGACGCTGACGGAG
CCCACTGGGCTTCTGTACGTGGGCGCCCGAGAGGCCCTGTTTGCCTTCAGCATGGAGGCCCTGGAGCTGCAAGGAGCGGT
GAGAGGTGGGGCAGTGGGAGGCAGTCGGGCCTGCCAGAGGGCAAGGCCCAGAGGGGCTGTGCTTGGCTAGGTGGTCCCTC
AGCCTGGCTGCCCCCACAGAAGTGCTGCTGGGCATCCGGGGCCTGCCCTCTGGTTGGGGTCAGCATGCGGCATTCCTCTC
CTCTCTGGCACTTACTGAATTCTGTGTAGTGGGGTGGTTTGGGGCTTCCATTCTGTACTAAAGGAAGTCCCCAGAGAATA
GGGACCATGCCTCTGCACTGGGCGTCCCTGTGTTGCTGCTGCAGGCTGTGCTTGTGACACGTGCTCAGTCCGTACCTGTA
ACGTGGCTGACATCCCCTTCCCTTTCCCCAGATCTCCTGGGAGGCCCCCGTGGAGAAGAAGACTGAGTGTATCCAGAAAG
GGAAGAACAACCAGGTGGGTGCTTGGACGCTGCTCCTGGTGCTGCCTTCACCCCAGGACGTCTCCTCCCATTCTGGCCCT
CGCGCTCTCACTAACCGGACACCTTTCTGCCCCCAGACCGAGTGCTTCAACTTCATCCGCTTCCTGCAGCCCTACAATGC
CTCCCACCTGTACGTCTGTGGCACCTACGCCTTCCAGCCCAAGTGCACCTACGTCGTGAGTGCTGCCCTCCTACCTCGGT
GTCCCCAGCCCCCCGCCCTCCTCACCCTTCTCTGGACTCGTGGATGTGGCCCACAGAGCCCTGCCCTTAAGCATCTCCTC
ATCACCTCTCTCTCTGTCCTTAGAACATGCTCACCTTCACTTTGGAGCATGGAGAGTTTGAAGATGGGAAGGGCAAGTGT
CCCTATGACCCAGCTAAGGGCCATGCTGGCCTTCTTGTGGGTGAGTGGCTGCCCCCCATGCAGGCTGGGTGAGTCCTTGG
GGCTTGGTTCCACTTGGGTCTGTGTTGGCCGGATGTGTCCATACCTCATGTGTGGCCTAGCTGACCTCTGTCTCCCATAG
ATGGTGAGCTGTACTCGGCCACACTCAACAACTTCCTGGGCACGGAACCCATTATCCTGCGTAACATGGGGCCCCACCAC
TCCATGAAGACAGAGTACCTGGCCTTTTTGGCTCAACGGTGAGCCACCGGGGGGCCTGTGGGAGCCATGGGTGGGAGGTCC
CCCGGGGGCCCAGCCCCTCCCTGCTGACCTCCTCCCCTTCCCACAGAACCTCACTTTGTAGGCTCTGCCTATGTACCTGAG
AGTGTGGGCAGCTTCACGGGGGACGACGACAAGGTCTACTTCTTCTTCAGGGAGCGGGCAGTGGAGTCCGACTGCTATGC
CGAGCAGGTGGTGGCTCGTGTGGCCCGTGTCTGCAAGGTACCGAGTCTCACAGCGGTGGGCCCTGGGAGCCCTGCTGGCC
CAGCGGGGCTGACCGGCAGTGTCCCCTTGCCGTCTGCCAGGGCGATATGGGGGGCGCACGGACCCTGCAGAGGAAGTGGA
CCACGTTCCTGAAGGCGCGGCTGGCATGCTCTGCCCCGAACTGGCAGCTCTACTTCAACCAGCTGCAGGCGATGCACACC
CTGCAGGACACCTCCTGGCACAACACCACCTTCTTTGGGGTTTTTCAAGCACAGTGGTGAGTTGGCCAGGGCTCCCACAG
AGTGGGGAGGAGCTGGGGTTCAGGTCAGGGACGGTGCATGGGTGGGAGCCTGGACCAGGCCTCAGAGGCAGTCCCGGAAG
CCAGGTGTCCAGAAGCCAAGGAGAATGCTGGCACCTGCCCCTTTCAGAGCTGCCTGTGAAGGGGCAGGGCCCCATGCCAG
GTGGCACTTGAGCTGCCAGGGCATTCTGGTACCCATGGCCAGTTTGTGCCAGGCCCTGCCTTCCTGCTGCTTGCCCTTAG
CTGGGGTGGGAAGAGATAAGGTGGGTACCTGACCCTGGGCTCCCTCTCGCTGTGACAGGGGTGACATGTACCTGTCGGCC
ATCTGTGAGTACCAGTTGGAAGAGATCCAGCGGGTGTTTGAGGGCCCCTATAAGGAGTACCATGAGGAAGCCCAGAAGTG
GGACCGCTACACTGACCCTGTACCCAGCCCTCGGCCTGGCTCGGTGAGTGCTAGGGCAGGGATGCATCTCCCCACCCAGC
TGCGGTGCCATCCCACCCTGCTGATGGCCTGCCCCCCACACCCTGCCCCAGTGCATTAACAACTGGCATCGGCGCCACG
GCTACACCAGCTCCCTGGAGCTACCCGACAACATCCTCAACTTCGTCAAGAAGCACCCGCTGATGGAGGAGCAGGTGGGG
CCTCGGTGGAGCCGCCCCCTGCTCGTGAAGAAGGGCACCAACTTCACCCACCTGGTGGCCGACCGGGTTACAGGACTTGA
TGGAGCCACCTATACAGTGCTGTTCATTGGCACAGGTGGGTGCATGGGTATGCAGGCATGGCTGCTCAAGGCTGGGCAGA
AGCCCGTGTGCCCAGGGCAGGGAGATTTGGAGCTGCCCCACGGCCTTGTGCCCTCTCATTTACACCTTCTCCTCTGCACC
CCCCAGGAGACGGCTGGCTGCTCAAGGCTGTGAGCCTGGGGCCCTGGGTTCACCTGATTGAGGAGCTGCAGCTGTTTGAC
CAGGAGCCCATGAGAAGCCTGGTGCTATCTCAGAGCAAGGTAGTAAGTTGGGCCCTATCTGCCACTGTCCCCAGCACTAT
CCCATCTCACTCTCTCCATCTGGCTGCTTCTCCCAGTTGGGCCTATGTGCTTGGCCAGTGCAGAGTTAGGAACACACACA
CATGTGGATGCACACCTTGCCTAACTTGGGGTGGGTTCCTGGAAACGAGAATGAAAACAGAATGGAATCTGCACTGCACA
GGGCCATTATATATAGCCAGGTCACGGGGCTGCGCTCCCAGCATCAAGCCTTGGCAACTGCTCCCATGTTTCTTTCAGAC
CCTCCAAGCTGTGCTGTGGTTCTGGCTCTGGCCTCTTCCCTGACCATGCCCCTGTGACCAGACCTCCCAGGCTGTGAGAT
GTTTATGATGCCCTCACCATGGTGGTTTTCCTTCCAGCCCCCATTTCCGTGACTGTTTCCCTGAAGTGCTTGCATTATAC
CCTTGTGCAATACTCTTTTTGGTTTTTTTTTGAGATGGAGTCTCACTCTGTCACCCAGGCTAGAGTGCAGTGACGCGATC
TCAGCTCACTGCAACCTCCACCTCCCAGGTTGAAGCTATTCTTATGCCTCAGCCTCCTGAGTAGCTGGGATTACAGGTGC
CTGCCACTATGCCCAGCTAAAGGTTTTTTGTTTTTGTTTTTGTTTTCTTTGAGATGGAGTCTCACTCTGTCGCCCAGGCT
GGAGTGCGGTGGCATGATCTCTGCTCACTGCAACCTCCACCTCCCGGGTTCAAGCAATTCTGCCTCGGCCTCCCAAGTAA
CTGGGACTACAGGCACGTGCCACCATGCCCAGCTAATTTTTTTTTTTTTTTTTTTTTGAGATGGAGTCTCGCTCTGTC
ACCCAGGCTGGAGTGCAGTGGCGCAATCTCGGCTCACTGCAAGCTCTGCCTCCAGGTTCACACCATTCTCCTGCCTCAG
CCTTCCATGTAGCTGGGACTACAGGCTCCCATCACCACGCCTGGCTAATTTTTTGTATTTTTAGTAGAGACGGGGTTTCA
CCGTGTTAGCCAGGATGGTCTCGATCTCCTGACCTTGCGATCCGCCCGACTCAGCCTCCCAAAGTGCTGGGATTACAGGC
GTGAGCCACTGCGCCTGGCCAGCCGGCTAATTTTTGTATTTTTAGTAGAGACAAGGTTTTACCATGTTGGCCAGGCTGGT
CTTGAACTCCTAACCTCAAGTGATTTGCCCACCTCAGCCTCCCAAAGTGCTGGGATTCCAGGCATGACCTGCTGTTCCTA
GTTGCCTTGTGCAATACTCTTGTGGCATGTTTGCTACACCTCCTGAACTTTGATTTGTTTGCCTTTTACCAGCTATTATG
ACTCAAAATTGTCCCCTAGAACATGGAATAATGGCAGAAAGAAAGTGTGTGGTTGAATAAACACACAGATTGGCATCCAC
CGTTGAAACAGGAAAACATCTTATGTTATGCTGCTGCTGTTGTGAGGGCTGATGGGCCTTGAAATGTATTTCCTGCACTA
TGTGTGTGTGAGTGTGTGTGATTATACTTTTTGGCCTCACAGCCCCATCATCCCTTTCTAATAACGTCACGTCGATAAGG
GGCTTAGGATTGCATCTGGCCTGTGTAAGCCCTCTGAGTTCTGCGGTTCTTAGAGTTCCCTTTTCAGCACTATAGCTCTG
CCTTGTTCCCTTGTTCCTCCTTCTGGCGCCCCGTGCTGTGCCCCCTGCAGGAGTCCAAGCTGTCCCCATGCTGCGTTCTG
GTCCGGCCGCCCCTCCCGTGGTGTGGCCCTGGCCGACCCCCCTCCTGCGCCCCGCTTTTCTCGCAGAAGCTGCTCTTTGC
CGGCTCCCGCTCTCAGCTGGTGCAGCTGCCCGTGGCCGACTGCATGAAGTATCGCTCCTGTGCAGACTGTGTCCTCGCCC
GGGACCCCTATTGCGCCTGGAGCGTCAACACCAGCCGCTGTGTGGCCGTGGGTGGCCACTCTGGGTGAGTTGGGCTCTAC
ATAGGCCAGGACCTCCAGGGACTCAGGGTGGTTGGAGCCAGGCTGCTGATGTACCCTACATCCCCTACCAGATCTCTACT
```

```
GATCCAGCATGTGATGACCTCGGACACTTCAGGCATCTGCAACCTCCGTGGCAGTAAGAAAGGTGAGCTTTTTCATTCCC
GTCGCATCGGGCTGAGCCCTGGACCAGAGCTGGAGTTTCTGTTCTCCTCTTCCCCAGCCCTGCTTTCCTGCACTAACATG
CACTCTGTTTTCTCTGCCACTACAGTCAGGCCCACTCCCAAAAACATCACGGTGGTGGCGGGCACAGACCTGGTGCTGCC
CTGCCACCTCTCCTCCAACTTGGCCCATGCCCGCTGGACCTTTGGGGGCCGGGACCTGCCTGCGGAACAGCCCGGGTCCT
TCCTCTACGATGCCCGGCTCCAGGCCCTGGTTGTGATGGCTGCCCAGCCCCGCCATGCCGGGGCCTACCACTGCTTTTCA
GAGGAGCAGGGGGCGCGGCTGGCTGCTGAAGGCTACCTTGTGGCTGTCGTGGCAGGCCCGTCGGTGACCTTGGAGGCCCG
GGCCCCCCTGGAAAACCTGGGGCTGGTGTGGCTGGCGGTGGTGGCCCTGGGGCTGTGTGCCTGGTGCTGCTGCTGCTGG
TGCTGTCATTGCGCCGGCGGCTGCGGGAAGAGCTGGAGAAAGGGGCCAAGGCTACTGAGAGGACCTTGGTGTACCCCCTG
GAGCTGCCCAAGGAGCCCACCAGTCCCCCCTTCCGGCCCTGTCCTGAACCAGATGAGAAACTTTGGGATCCTGTCGGTTA
CTACTATTCAGATGGCTCCCTTAAGATAGTACCTGGGCATGCCCGGTGCCAGCCCGGTGGGGGGCCCCCTTCGCCACCTC
CAGGCATCCCAGGCCAGCCTCTGCCTTCTCCAACTCGGCTTCACCTGGGGGGTGGGCGGAACTCAAATGCCAATGGTTAC
GTGCGCTTACAACTAGGAGGGGAGGACCGGGGAGGGCTCGGGCACCCCCTGCCTGAGCTCGCGGATGAACTGAGACGCAA
ACTGCAGCAACGCCAGCCACTGCCCGACTCCAACCCCGAGGAGTCATCAGTATGAGGGGAACCCCCACCGCGTCGGCGGG
AAGCGTGGGAGGTGTAGCTCCTACTTTTGCACAGGCACCAGCTACCTCAGGGACATGGCACGGGCACCTGCTCTGTCTGG
GACAGATACTGCCCAGCACCCACCCGGCCATGAGGACCTGCTCTGCTCAGCACGGGCACTGCCACTTGGTGTGGCTCACC
AGGGCACCAGCCTCGCAGAAGGCATCTTCCTCCTCTCTGTGAATCACAGACACGCGGGACCCCAGCCGCCAAAACTTTTC
AAGGCAGAAGTTTCAAGATGTGTGTTTGTCTGTATTTGCACATGTGTTTGTGTGTGTGTATGTGTGTGTGCACGCGCG
TGCGCGCTTGTGGCATAGCCTTCCTGTTTCTGTCAAGTCTTCCCTTGGCCTGGGTCCTCCTGGTGAGTCATTGGAGCTAT
GAAGGGGAAGGGGTCGTATCACTTTGTCTCTCCTACCCCCACTGCCCCGAGTGTCGGGCAGCGATGTACATATGGAGGTG
GGGTGGACAGGGTGCTGTGCCCCTTCAGAGGGAGTGCAGGGCTTGGGGTGGGCCTAGTCCTGCTCCTAGGGCTGTGAATG
TTTTCAGGGTGGGGGGAGGGAGATGGAGCCTCCTGTGTGTTTGGGGGGAAGGGTGGGTGGGGCCTCCCACTTGGCCCCGG
GGTTCAGTGGTATTTTATACTTGCCTTCTTCCTGTACAGGGCTGGGAAAGGCTGTGTGAGGGGAGAGAAGGGAGAGGGTG
GGCCTGCTGTGGACAATGGCATACTCTCTTCCAGCCCTAGGAGGAGGGCTCCTAACAGTGTAACTTATTGTGTCCCCGCG
TATTTATTTGTTGTAAATATTTGAGTATTTTTATATTGACAAATAAATGGAGAAAATGAAACGA
```

HUMAN mRNA SEQUENCE : hR22-005.1 (Seq ID No: 139)

```
CTGTGCTCGCATCTCTGGCAGCCTGGATTGGGGTCCTGCTGGAGTGAAGGGTTTCCTGAGGCAGGGAGTACTCATAGCCG
TCTGTGCCTCCTGCTCTGCTGGACTCTGATAGAGGCAGTGGGCAGCAGGGCAAAGAAGGAAGCAGCCGCCGAGGAAGCGA
AGGTGGGGTGGGGGTGCCCAGCCCTGCGGCCGGAGGTGCCATTGACCCTGAGAGCAAGAGCCATCAGTCTGATGGCTTCC
TCTGGCCGGAAGCTGTGGCTGAGATATCCTTCCTTCCTCCCAGCAGCCTGGATTTGCCTCCTCCCAGGCTGGGAAAGGCT
AGGGAGGCCCAGGTGGGGCTGTCAGGGCCAAAGGCTGTTTCAAAAGTGTCCTTTATTGCCAATCAGGGGGTTTGGCTGGC
ATCTGCTTGTGGCATGGGGTGCTGGCTCTCGTGGAGCAAGACTGAGGGCTGTCGAGCCCCAGGGGTCCTGCCCATCAGCA
GCCATGCTTACCCCTGCAGAGCTGGCCACGGTAGTACGGCGGTTCTCCCAGACCGGCATCCAGGACTTCCTGACACTGAC
GCTGACGGAGCCCACTGGGCTTCTGTACGTGGGCGCCCGAGAGGCCCTGTTTGCCTTCAGCATGGAGGCCCTGGAGCTGC
AAGGAGCGATCTCCTGGGAGGCCCCCGTGGAGAAGAAGACTGAGTGTATCCAGAAAGGGAAGAACAACCAGACCGAGTGC
TTCAACTTCATCCGCTTCCTGCAGCCCTACAATGCCTCCCACCTGTACGTCTGTGGCACCTACGCCTTCCAGCCCAAGTG
CACCTACGTCAACATGCTCACCTTCACTTTGGAGCATGGAGAGTTTGAAGATGGGAAGGGCAAGTGTCCCTATGACCCAG
CTAAGGGCCATGCTGGCCTTCTTGTGGATGGTGAGCTGTACTCGGCCACACTCAACAACTTCCTGGGCACGGAACCCATT
ATCCTGCGTAACATGGGGCCCCACCACTCCATGAAGACAGAGTACCTGGCCTTTTGGCTCAACGAACCTCACTTTGTAGG
CTCTGCCTATGTACCTGAGAGTGTGGGCAGCTTCACGGGGGACGACGACAAGGTCTACTTCTTCTTCAGCGAGCGGGCAG
TGGAGTCCGACTGCTATGCCGAGCAGGTGGTGGCTCGTGTGGCCCGTGTCTGCAAGGGCGATATGGGGGGCGCACGGACC
CTGCAGAGGAAGTGGACCACGTTCCTGAAGGCGCGGCTGGCATGCTCTGCCCCGAACTGGCAGCTCTACTTCAACCAGCT
GCAGGCGATGCACACCCTGCAGGACACCTCCTGGCACAACACCACCTTCTTTGGGGTTTTTCAAGCACAGTGGGGTGACA
TGTACCTGTCGGCCATCTGTGAGTACCAGTTGGAAGAGATCCAGCGGGTGTTTGAGGGCCCCTATAAGGAGTACCATGAG
GAAGCCCAGAAGTGGGACCGCTACACTGACCCTGTACCCAGCCCTCGGCCTGGCTCGTGCATTAACAACTGGCATCGGCG
CCACGGCTACACCAGCTCCCTGGAGCTACCCGACAACATCCTCAACTTCGTCAAGAAGCACCCGCTGATGGAGGAGCAGG
TGGGGCCTCGGTGGAGCCGCCCCCTGCTCGTGAAGAAGGGCACCAACTTCACCCACCTGGTGGCCGACCGGGTTACAGGA
CTTGATGGAGCCACCTATACAGTGCTGTTCATTGGCACAGGAGACGGCTGGCTGCTCAAGGCTGTGAGCCTGGGGCCCTG
GGTTCACCTGATTGAGGAGCTGCAGCTGTTTGACCAGGAGCCCATGAGAAGCCTGGTGCTATCTCAGAGCAAGAAGCTGC
TCTTTGCCGGCTCCCGCTCTCAGCTGGTGCAGCTGCCCGTGGCCGACTGCATGAAGTATCGCTCCTGTGCAGACTGTGTC
CTCGCCCGGGACCCCTATTGCGCCTGGAGCGTCAACACCAGCCGCTGTGTGGCCGTGGGTGGCCACTCTGGATCTCTACT
GATCCAGCATGTGATGACCTCGGACACTTCAGGCATCTGCAACCTCCGTGGCAGTAAGAAAGTCAGGCCCACTCCCAAAA
ACATCACGGTGGTGGCGGGCACAGACCTGGTGCTGCCCTGCCACCTCTCCTCCAACTTGGCCCATGCCCGCTGGACCTTT
GGGGGCCGGGACCTGCCTGCGGAACAGCCCGGGTCCTTCCTCTACGATGCCCGGCTCCAGGCCCTGGTTGTGATGGCTGC
CCAGCCCCGCCATGCCGGGGCCTACCACTGCTTTTCAGAGGAGCAGGGGGCGCGGCTGGCTGCTGAAGGCTACCTTGTGG
CTGTCGTGGCAGGCCCGTCGGTGACCTTGGAGGCCCGGGCCCCCTGGAAAACCTGGGGCTGGTGTGGCTGGCGGTGGTG
GCCCTGGGGCTGTGTGCCTGGTGCTGCTGCTGCTGGTGCTGTCATTGCGCCGGCGGCTGCGGGAAGAGCTGGAGAAAGG
GGCCAAGGCTACTGAGAGGACCTTGGTGTACCCCCTGGAGCTGCCCAAGGAGCCCACCAGTCCCCCCTTCCGGCCCTGTC
CTGAACCAGATGAGAAACTTTGGGATCCTGTCGGTTACTACTATTCAGATGGCTCCCTTAAGATAGTACCTGGGCATGCC
CGGTGCCAGCCCGGTGGGGGGCCCCCTTCGCCACCTCCAGGCATCCCAGGCCAGCCTCTGCCTTCTCCAACTCGGCTTCA
CCTGGGGGGTGGGCGGAACTCAAATGCCAATGGTTACGTGCGCTTACAACTAGGAGGGGAGGACCGGGGAGGGCTCGGGC
```

ACCCCCTGCCTGAGCTCGCGGATGAACTGAGACGCAAACTGCAGCAACGCCAGCCACTGCCCGACTCCAACCCCGAGGAG
TCATCAGTATGAGGGGAACCCCCACCGCGTCGGCGGGAAGCGTGGGAGGTGTAGCTCCTACTTTTGCACAGGCACCAGCT
ACCTCAGGGACATGGCACGGGCACCTGCTCTGTCTGGGACAGATACTGCCCAGCACCCACCCGGCCATGAGGACCTGCTC
TGCTCAGCACGGGCACTGCCACTTGGTGTGGCTCACCAGGGCACCAGCCTCGCAGAAGGCATCTTCCTCCTCTCTGTGAA
TCACAGACACGCGGGACCCCAGCCGCCAAAACTTTTCAAGGCAGAAGTTTCAAGATGTGTGTTTGTCTGTATTTGCACAT
GTGTTTGTGTGTGTGTGTATGTGTGTGTGCACGCGCGTGCGCGCTTGTGGCATAGCCTTCCTGTTTCTGTCAAGTCTTCC
CTTGGCCTGGGTCCTCCTGGTGAGTCATTGGAGCTATGAAGGGGAAGGGGTCGTATCACTTTGTCTCTCCTACCCCCACT
GCCCCGAGTGTCGGGCAGCGATGTACATATGGAGGTGGGGTGGACAGGGTGCTGTGCCCCTTCAGAGGGAGTGCAGGGCT
TGGGGTGGGCCTAGTCCTGCTCCTAGGGCTGTGAATGTTTTCAGGGTGGGGGGAGGGAGATGGAGCCTCCTGTGTGTTTG
GGGGGAAGGGTGGGTGGGGCCTCCCACTTGGCCCCGGGGTTCAGTGGTATTTTATACTTGCCTTCTTCCTGTACAGGGCT
GGGAAAGGCTGTGTGAGGGGAGAGAAGGGAGAGGGTGGGCCTGCTGTGGACAATGGCATACTCTCTTCCAGCCCTAGGAG
GAGGGCTCCTAACAGTGTAACTTATTGTGTCCCCGCGTATTTATTTGTTGTAAATATTTGAGTATTTTTATATTGACAAA
TAAAATGGAGAAAATGAAACGA


HUMAN PROTEIN SEQUENCE : hP22-005.1 (Seq ID No: 140)
MASSGRKLWLRYPSFLPAAWICLLPGWERLGRPRWGCQGQRLFQKCPLLPIRGFGWHLLVAWGAGSRGARLRAVEPQGSC
PSAAMLTPAELATVVRRFSQTGIQDFLTLTLTEPTGLLYVGAREALFAFSMEALELQGAISWEAPVEKKTECIQKGKNNQ
TECFNFIRFLQPYNASHLYVCGTYAFQPKCTYVNMLTFTLEHGEFEDGKGKCPYDPAKGHAGLLVDGELYSATLNNFLGT
EPIILRNMGPHHSMKTEYLAFWLNEPHFVGSAYVPESVGSFTGDDDKVYFFFRERAVESDCYAEQVVARVARVCKGDMGG
ARTLQRKWTTFLKARLACSAPNWQLYFNQLQAMHTLQDTSWHNTTFFGVFQAQWGDMYLSAICEYQLEEIQRVFEGPYKE
YHEEAQKWDRYTDPVPSPRPGSCINNWHRRHGYTSSLELPDNILNFVKKHPLMEEQVGPRWSRPLLVKKGTNFTHLVADR
VTGLDGATYTVLFIGTGDGWLLKAVSLGPWVHLIEELQLFDQEPMRSLVLSQSKKLLFAGSRSQLVQLPVADCMKYRSCA
DCVLARDPYCAWSVNTSRCVAVGGHSGSLLIQHVMTSDTSGICNLRGSKKVRPTPKNITVVAGTDLVLPCHLSSNLAHAR
WTFGGRDLPAEQPGSFLYDARLQALVVMAAQPRHAGAYHCFSEEQGARLAAEGYLVAVVAGPSVTLEARAPLENLGLVWL
AVVALGAVCLVLLLLVLSLRRRLREEELEKGAKATERTLVYPLELPKEPTSPPFRPCPEPDEKLWDPVGYYYSDGSLKIVP
GHARCQPGGGPPSPPPGIPGQPLPSPTRLHLGGGRNSNANGYVRLQLGGEDRGGLGHPLPELADELRRKLQQRQPLPDSN
PEESSV*


HUMAN mRNA SEQUENCE : hR22-005.2 (Seq ID No: 141)
GGCGATATGGGGGGCGCACGGACCCTGCAGAGGAAGTGGACCACGTTCCTGAAGGCGCGGCTGGCATGCTCTGCCCCGAA
CTGGCAGCTCTACTTCAACCAGCTGCAGGCGATGCACACCCTGCAGGACACCTCCTGGCACAACACCACCTTCTTTGGGG
TTTTTCAAGCACAGTGGGGTGACATGTACCTGTCGGCCATCTGTGAGTACCAGTTGGAAGAGATCCAGCGGGTGTTTGAG
GGCCCCTATAAGGAGTACCATGAGGAAGCCCAGAAGTGGGACCGCTACACTGACCCTGTACCCAGCCCTCGGCCTGGCTC
GTGCATTAACAACTGGCATCGGCGCCACGGCTACACCAGCTCCCTGGAGCTACCCGACAACATCCTCAACTTCGTCAAGA
AGCACCCGCTGATGGAGGAGCAGGTGGGGCCTCGGTGGAGCCGCCCCCTGCTCGTGAAGAAGGGCACCAACTTCACCCAC
CTGGTGGCCGACCGGGTTACAGGACTTGATGGAGCCACCTATACAGTGCTGTTCATTGGCACAGGAGACGGCTGGCTGCT
CAAGGCTGTGAGCCTGGGGCCCTGGGTTCACCTGATTGAGGAGCTGCAGCTGTTTGACCAGGAGCCCATGAGAAGCCTGG
TGCTATCTCAGAGCAAGAAGCTGCTCTTTGCCGGCTCCCGCTCTCAGCTGGTGCAGCTGCCCGTGGCCGACTGCATGAAG
TATCGCTCCTGTGCAGACTGTGTCCTCGCCCGGGACCCCTATTGCGCCTGGAGCGTCAACACCAGCCGCTGTGTGGCCGT
GGGTGGCCACTCTGGATCTCTACTGATCCAGCATGTGATGACCTCGGACACTTCAGGCATCTGCAACCTCCGTGGCAGTA
AGAAAGTCAGGCCCACTCCCAAAAACATCACGGTGGTGGCGGGCACAGACCTGGTGCTGCCCTGCCACCTCTCCTCCAAC
TTGGCCCATGCCCGCTGGACCTTTGGGGGCCGGGACCTGCCTGCGGAACAGCCCGGGTCCTTCCTCTACGATGCCCGGCT
CCAGGCCCTGGTTGTGATGGCTGCCCAGCCCCGCCATGCCGGGGCCTACCACTGCTTTTCAGAGGAGCAGGGGGCGCGGC
TGGCTGCTGAAGGCTACCTTGTGGCTGTCGTGGCAGGCCCGTCGGTGACCTTGGAGGCCCGGGCCCCCTGGAAAACCTG
GGGCTGGTGTGGCTGGCGGTGGTGGCCCTGGGGGCTGTGTGCCTGGTGCTGCTGCTGCTGGTGCTGTCATTGCGCCGGCG
GCTGCGGGAAGAGCTGGAGAAAGGGGCCAAGGCTACTGAGAGGACCTTGGTGTACCCCCTGGAGCTGCCCAAGGAGCCCA
CCAGTCCCCCCTTCCGGCCCTGTCCTGAACCAGATGAGAAACTTTGGGATCCTGTCGGTTACTACTATTCAGATGGCTCC
CTTAAGATAGTACCTGGGCATGCCCGGTGCCAGCCCGGTGGGGGGCCCCCTTCGCCACCTCCAGGCATCCCAGGCCAGCC
TCTGCCTTCTCCAACTCGGCTTCACCTGGGGGGTGGGCGGAACTCAAATGCCAATGGTTACGTGCGCTTACAACTAGGAG
GGGAGGACCGGGGAGGGGCTCGGGCACCCCCTGCCTGAGCTCGCGGATGAACTGAGACGCAAACTGCAGCAACGCCAGCCA
CTGCCCGACTCCAACCCCGAGGAGTCATCAGTATGAGGGGAACCCCCACCGCGTCGGCGGGAAGCGTGGGAGGTGTAGCT
CCTACTTTTGCACAGGCACCAGCTACCTCAGGGACATGGCACGGGCACCTGCTCTGTCTGGGACAGATACTGCCCAGCAC
CCACCCGGCCATGAGGACCTGCTCTGCTCAGCACGGGCACTGCCACTTGGTGTGGCTCACCAGGGCACCAGCCTCGCAGA
AGGCATCTTCCTCCTCTCTGTGAATCACAGACACGCGGGACCCCAGCCGCCAAAACTTTTCAAGGCAGAAGTTTCAAGAT
GTGTGTTTGTCTGTATTTGCACATGTGTTTGTGTGTGTGTGTATGTGTGTGTGCACGCGCGTGCGCGCTTGTGGCATAGC
CTTCCTGTTTCTGTCAAGTCTTCCCTTGGCCTGGGTCCTCCTGGTGAGTCATTGGAGCTATGAAGGGGAAGGGGTCGTAT
CACTTTGTCTCTCCTACCCCCACTGCCCCGAGTGTCGGGCAGCGATGTACATATGGAGGTGGGGTGGACAGGGTGCTGTG
CCCCTTCAGAGGGAGTGCAGGGCTTGGGGTGGGCCTAGTCCTGCTCCTAGGGCTGTGAATGTTTTCAGGGTGGGGGGAGG
GAGATGGAGCCTCCTGTGTGTTTGGGGGGAAGGGTGGGTGGGGCCTCCCACTTGGCCCCGGGGTTCAGTGGTATTTTATA
CTTGCCTTCTTCCTGTACAGGGCTGGGAAAGGCTGTGTGAGGGGAGAGAAGGGAGAGGGTGGGCCTGCTGTGGACAATGG

CATACTCTCTTCCAGCCCTAGGAGGAGGGCTCCTAACAGTGTAACTTATTGTGTCCCCGCGTATTTATTTGTTGTAAATA
TTTGAGTATTTTTATATTGACAAATAAAATGGAGAAAATGAAACGA

HUMAN PROTEIN SEQUENCE : hP22-005.2 (Seq ID No: 142)
MGGARTLQRKWTTFLKARLACSAPNWQLYFNQLQAMHTLQDTSWHNTTFFGVFQAQWGDMYLSAICEYQLEEIQRVFEGP
YKEYHEEAQKWDRYTDPVPSPRPGSCINNWHRRHGYTSSLELPDNILNFVKKHPLMEEQVGPRWSRPLLVKKGTNFTHLV
ADRVTGLDGATYTVLFIGTGDGWLLKAVSLGPWVHLIEELQLFDQEPMRSLVLSQSKKLLFAGSRSQLVQLPVADCMKYR
SCADCVLARDPYCAWSVNTSRCVAVGGHSGSLLIQHVMTSDTSGICNLRGSKKVRPTPKNITVVAGTDLVLPCHLSSNLA
HARWTFGGRDLPAEQPGSFLYDARLQALVVMAAQPRHAGAYHCFSEEQGARLAAEGYLVAVVAGPSVTLEARAPLENLGL
VWLAVVALGAVCLVLLLLVLSLRRRLREELEKGAKATERTLVYPLELPKEPTSPPFRPCPEPDEKLWDPVGYYYSDGSLK
IVPGHARCQPGGGPPSPPPGIPGQPLPSPTRLHLGGGRNSNANGYVRLQLGGEDRGGLGHPLPELADELRRKLQQRQPLP
DSNPEESSV*

HUMAN mRNA SEQUENCE : hR22-005.3 (Seq ID No: 143)
GCTGGAGAAAGAGCCGGCGCCCGCCGTGGTGGCGCGGGGAGCCCGAGCCTAGGGGCGCGGAGCCGGGCGGGGACGGAGGG
GCGGTGGCAGAGAGGCCGCGGAGGGCTGGCGGGCGAGCGCGGGCAGGCGGCGACGCGGGGGCAGGGGTGGACGGCGGTCA
GAGCCGAACGCGAGGGCGGCGCCCGGGGACTGGAGCTGCGCGCAATAGCAGGACAGCTGGCCTGAAGCTCAGAGCCGGGG
CGTGCGCCATGGCCCCACACTGGGCTGTCTGGCTGCTGGCAGCAAGGCTGTGGGGCCTGGGCATTGGGGCTGAGGTGTGG
TGGAACCTTGTGCCGCGTAAGACAGTGTCTTCTGGGGAGCTGGCCACGGTAGTACGGCGGTTCTCCCAGACCGGCATCCA
GGACTTCCTGACACTGACGCTGACGGAGCCCACTGGGCTTCTGTACGTGGGCGCCCGAGAGGCCCTGTTTGCCTTCAGCA
TGGAGGCCCTGGAGCTGCAAGGAGCGATCTCCTGGGAGGCCCCCGTGGAGAAGAAGACTGAGTGTATCCAGAAAGGGAAG
AACAACCAGACCGAGTGCTTCAACTTCATCCGCTTCCTGCAGCCCTACAATGCCTCCCACCTGTACGTCTGTGGCACCTA
CGCCTTCCAGCCCAAGTGCACCTACGTCAACATGCTCACCTTCACTTTGGAGCATGGAGAGTTTGAAGATGGGAAGGGCA
AGTGTCCCTATGACCCAGCTAAGGGCCATGCTGGCCTTCTTGTGGAACCTCACTTTGTAGGCTCTGCCTATGTACCTGAG
AGTGTGGGCAGCTTCACGGGGGACGACGACAAGGTCTACTTCTTCTTCAGGGAGCGGGCAGTGGAGTCCGACTGCTATGC
CGAGCAGGTGGTGGCTCGTGTGGCCCGTGTCTGCAAGGGCGATATGGGGGGCGCACGGACCCTGCAGAGGAAGTGGACCA
CGTTCCTGAAGGCGCGGCTGGCATGCTCTGCCCCGAACTGGCAGCTCTACTTCAACCAGCTGCAGGCGATGCACACCCTG
CAGGACACCTCCTGGCACAACACCACCTTCTTTGGGGTTTTTCAAGCACAGTGGGGTGACATGTACCTGTCGGCCATCTG
TGAGTACCAGTTGGAAGAGATCCAGCGGGTGTTTGAGGGCCCCTATAAGGAGTACCATGAGGAAGCCCAGAAGTGGGACC
GCTACACTGACCCTGTACCCAGCCCTCGGCCTGGCTCGTGCATTAACAACTGGCATCGGCGCCACGGCTACACCAGCTCC
CTGGAGCTACCCGACAACATCCTCAACTTCGTCAAGAAGCACCCGCTGATGGAGGAGCAGGTGGGGCCTCGGTGGAGCCG
CCCCCTGCTCGTGAAGAAGGGCACCAACTTCACCCACCTGGTGGCCGACCGGGTTACAGGACTTGATGGAGCCACCTATA
CAGTGCTGTTCATTGGCACAGGAGACGGCTGGCTGCTCAAGGCTGTGAGCCTGGGGCCCTGGGTTCACCTGATTGAGGAG
CTGCAGCTGTTTGACCAGGAGCCCATGAGAAGCCTGGTGCTATCTCAGAGCAAGAAGCTGCTCTTTGCCGGCTCCCGCTC
TCAGCTGGTGCAGCTGCCCGTGGCCGACTGCATGAAGTATCGCTCCTGTGCAGACTGTGTCCTCGCCCGGGACCCCTATT
GCGCCTGGAGCGTCAACACCAGCCGCTGTGTGGCCGTGGGTGGCCACTCTGGATCTCTACTGATCCAGCATGTGATGACC
TCGGACACTTCAGGCATCTGCAACCTCCGTGGCAGTAAGAAAGTCAGGCCCACTCCCAAAAACATCACGGTGGTGGCGGG
CACAGACCTGGTGCTGCCCTGCCACCTCTCCTCCAACTTGGCCCATGCCCGCTGGACCTTTGGGGGCCGGGACCTGCCTG
CGGAACAGCCCGGGTCCTTCCTCTACGATGCCCGGCTCCAGGCCCTGGTTGTGATGGCTGCCCAGCCCCGCCATGCCGGG
GCCTACCACTGCTTTTCAGAGGAGCAGGGGGCGCGGCTGGCTGCTGAAGGCTACCTTGTGGCTGTCGTGGCAGGCCCGTC
GGTGACCTTGGAGGCCCGGGCCCCCCTGGAAAACCTGGGGCTGGTGTGGCTGGCGGTGGTGGCCCTGGGGGCTGTGTGCC
TGGTGCTGCTGCTGCTGGTGCTGTCATTGCGCCGGCGGCTGCGGGAAGAGCTGGAGAAAGGGGCCAAGGCTACTGAGAGG
TTGGGATCCTGTCGGTTACTACTATTCAGATGGCTCCCTTAAGATAGTACCTGGGCATGCCCGGTGCCAGCCCGGTGGGG
GGCCCCCTTCGCCACCTCCAGGCATCCCAGGCCAGCCTCTGCCTTCTCCAACTCGGCTTCACCTGGGGGGTGGGCGGAAC
TCAAATGCCAATGGTTACGTGCGCTTACAACTAGGAGGGAGGACCGGGGAGGGCTCGGGCACCCCTGCCTGAGCTCGC
GGATGAACTGAGACGCAAACTGCAGCAACGCCAGCCACTGCCCGACTCCAACCCCGAGGAGTCATCAGTATGAGGGGAAC
CCCCACCGCGTCGGCGGGAAGCGTGGAGGTGTAGCTCCTACTTTTGCACAGGCACCAGCTACCTCAGGGACATGGCACG
GGCACCTGCTCTGTCTGGGACAGATACTGCCCAGCACCCACCCGGCCATGAGGACCTGCTCTGCTCAGCACGGGCACTGC
CACTTGGTGTGGCTCACCAGGGCACCAGCCTCGCAGAAGCATCTTCCTCCTCTGTGAATACACAGACACGCGGGACCC
CAGCCGCCAAAACTTTTCAAGGCAGAAGTTTCAAGATGTGTGTTTGTCTGTATTTGCACATGTGTTTGTGTGTGTGTGTA
TGTGTGTGTGCACGCGCGTGCGCGCTTGTGGCATAGCCTTCCTGTTTCTGTCAAGTCTTCCCTTGGCCTGGGTCCTCCTG
GTGAGTCATTGGAGCTATGAAGGGGAAGGGGTCGTATCACTTTGTCTCTCCTACCCCCACTGCCCCGAGTGTCGGGCAGC
GATGTACATATGGAGGTGGGGTGGACAGGGTGCTGTGCCCCTTCAGAGGGAGTGCAGGGCTTGGGGTGGGCCTAGTCCTG
CTCCTAGGGCTGTGAATGTTTTCAGGGTGGGGGGAGGGAGATGGAGCCTCCTGTGTGTTTGGGGGGAAGGGTGGGTGGGG
CCTCCCACTTGGCCCCGGGGTTCAGTGGTATTTTATACTTGCCTTCTTCCTGTACAGGGCTGGGAAAGGCTGTGTGAGGG
GAGAGAAGGGAGAGGGTGGGCCTGCTGTGGACAATGGCATACTCTCTTCCAGCCCTAGGAGGAGGGCTCCTAACAGTGTA
ACTTATTGTGTCCCCGCGTATTTATTTGTTGTAAATATTTGAGTATTTTTATATTGACAAATAAAATGGAGAAAATGAAA
CGA

HUMAN PROTEIN SEQUENCE : hP22-005.3 (Seq ID No: 144)

MAPHWAVWLLAARLWGLGIGAEVWWNLVPRKTVSSGELATVVRRFSQTGIQDFLTLTLTEPTGLLYVGAREALFAFSMEA
LELQGAISWEAPVEKKTECIQKGKNNQTECFNFIRFLQPYNASHLYVCGTYAFQPKCTYVNMLTFTLEHGEFEDGKGKCP
YDPAKGHAGLLVEPHFVGSAYVPESVGSFTGDDDKVYFFFRERAVESDCYAEQVVARVARVCKGDMGGARTLQRKWTTFL
KARLACSAPNWQLYFNQLQAMHTLQDTSWHNTTFFGVFQAQWGDMYLSAICEYQLEEIQRVFEGPYKEYHEEAQKWDRYT
DPVPSPRPGSCINNWHRRHGYTSSLELPDNILNFVKKHPLMEEQVGPRWSRPLLVKKGTNFTHLVADRVTGLDGATYTVL
FIGTGDGWLLKAVSLGPWVHLIEELQLFDQEPMRSLVLSQSKKLLFAGSRSQLVQLPVADCMKYRSCADCVLARDPYCAW
SVNTSRCVAVGGHSGSLLIQHVMTSDTSGICNLRGSKKVRPTPKNITVVAGTDLVLPCHLSSNLAHARWTFGGRDLPAEQ
PGSFLYDARLQALVVMAAQPRHAGAYHCFSEEQGARLAAEGYLVAVVAGPSVTLEARAPLENLGLVWLAVVALGAVCLVL
LLLVLSLRRRLREELEKGAKATERTLVYPLELPKEPTSPPFRPCPEPDEKLWDPVGYYYSDGSLKIVPGHARCQPGGGPP
SPPPGIPGQPLPSPTRLHLGGGRNSNANGYVRLQLGGEDRGGLGHPLPELADELRRKLQQRQPLPDSNPEESSV*

HUMAN mRNA SEQUENCE : hR22-005.4 (Seq ID No: 145)
TCGGGAAGCTGGGCCCCTTCGCTCCCGGCCCTTCTCACACTCCTGCCCTGCTGATGTGGAACGGGGTTTGGGGTTCTGCA
GGGCTATTGTCTGCGCTGGGGAAGGGGACAGGCCGGGACCGGGACCTCCGCTCGCAGCCGGCCGCACCAGGACAGCTGGC
CTGAAGCTCAGAGCCGGGGCGTGCGCCATGGCCCCACACTGGGCTGTCTGGCTGCTGGCAGCAAGGCTGTGGGGCCTGGG
CATTGGGGCTGAGGTGTGGTGGAACCTTGTGCCGCGTAAGACAGTGTCTTCTGGGGAGCTGGCCACGGTAGTACGGCGGT
TCTCCCAGACCGGCATCCAGGACTTCCTGACACTGACGCTGACGGAGCCCACTGGGCTTCTGTACGTGGGCGCCCGAGAG
GCCCTGTTTGCCTTCAGCATGGAGGCCCTGGAGCTGCAAGGAGCGATCTCCTGGGAGGCCCCCGTGGAGAAGAAGACTGA
GTGTATCCAGAAAGGGAAGAACAACCAGACCGAGTGCTTCAACTTCATCCGCTTCCTGCAGCCCTACAATGCCTCCCACC
TGTACGTCTGTGGCACCTACGCCTTCCAGCCCAAGTGCACCTACGTCAACATGCTCACCTTCACTTTGGAGCATGGAGAG
TTTGAAGATGGGAAGGGCAAGTGTCCCTATGACCCAGCTAAGGGCCATGCTGGCCTTCTTGTGGATGGTGAGCTGTACTC
GGCCACACTCAACAACTTCCTGGGCACGGAACCCATTATCCTGCGTAACATGGGGCCCACCACTCCATGAAGACAGAGT
ACCTGGCCTTTTGGCTCAACGAACCTCACTTTGTAGGCTCTGCCTATGTACCTGAGAGTGTGGGCAGCTTCACGGGGGAC
GACGACAAGGTCTACTTCTTCTTCAGGGAGCGGGCAGTGGAGTCCGACTGCTATGCCGAGCAGGTGGTGGCTCGTGTGGC
CCGTGTCTGCAAGGGCGATATGGGGGGCGCACGGACCCTGCAGAGGAAGTGGACCACGTTCCTGAAGGCGCGGCTGGCAT
GCTCTGCCCCGAACTGGCAGCTCTACTTCAACCAGCTGCAGGCGATGCACACCCTGCAGGACACCTCCTGGCACAACACC
ACCTTCTTTGGGGTTTTTTCAAGCACAGTGGGGTGACATGTACCTGTCGGCCATCTGTGAGTACCAGTTGGAAGAGATCCA
GCGGGTGTTTGAGGGCCCCTATAAGGAGTACCATGAGGAAGCCCAGAAGTGGGACCGCTACACTGACCCTGTACCCAGCC
CTCGGCCTGGCTCGTGCATTAACAACTGGCATCGGCGCCACGGCTACACCAGCTCCCTGGAGCTACCCGACAACATCCTC
AACTTCGTCAAGAAGCACCCGCTGATGGAGGAGCAGGTGGGGCCTCGGTGGAGCCGCCCCCTGCTCGTGAAGAAGGGCAC
CAACTTCACCCACCTGGTGGCCGACCGGGTTACAGGACTTGATGGAGCCACCTATACAGTGCTGTTCATTGGCACAGGAG
ACGGCTGGCTGCTCAAGGCTGTGAGCCTGGGGCCCTGGGTTCACCTGATTGAGGAGCTGCAGCTGTTTGACCAGGAGCCC
ATGAGAAGCCTGGTGCTATCTCAGAGCAAGAAGCTGCTCTTTGCCGGCTCCCGCTCTCAGCTGGTGCAGCTGCCCGTGGC
CGACTGCATGAAGTATCGCTCCTGTGCAGACTGTGTCCTCGCCCGGGACCCCTATTGCGCCTGGAGCGTCAACACCAGCC
GCTGTGTGGCCGTGGGTGGCCACTCTGGATCTCTACTGATCCAGCATGTGATGACCTCGGACACTTCAGGCATCTGCAAC
CTCCGTGGCAGTAAGAAAGTCAGGCCCACTCCCAAAAACATCACGGTGGTGGCGGGCACAGACCTGGTGCTGCCCTGCCA
CCTCTCCTCCAACTTGGCCCATGCCCGCTGGACCTTTGGGGGCCGGGACCTGCCTGCGGAACAGCCCGGGTCCTTCCTCT
ACGATGCCCGGCTCCAGGCCCTGGTTGTGATGGCTGCCCAGCCCCGCCATGCCGGGGCCTACCACTGCTTTTCAGAGGAG
CAGGGGGGCGCGGCTGGCTGCTGAAGGCTACCTTGTGGCTGTCGTGGCAGGCCCGTCGGTGACCTTGGAGGCCCGGGCCCC
CCTGGAAAACCTGGGGCTGGTGTGGCTGGCGGTGGTGGCCCTGGGGGCTGTGTGCCTGGTGCTGCTGCTGCTGGTGCTGT
CATTGCGCCGGCGGCTGCGGGAAGAGCTGGAGAAAGGGGCCAAGGCTACTGAGAGGACCTTGGTGTACCCCCTGGAGCTG
CCCAAGGAGCCCACCAGTCCCCCCTTCCGGCCCTGTCCTGAACCAGATGAGAAACTTTGGGATCCTGTCGGTTACTACTA
TTCAGATGGCTCCCTTAAGATAGTACCTGGGCATGCCCGGTGCCAGCCCGGTGGGGGGCCCCCTTCGCCACCTCCAGGCA
TCCCAGGCCAGCCTCTGCCTTCTCCAACTCGGCTTCACCTGGGGGGTGGGCGGAACTCAAATGCCAATGGTTACGTGCGC
TTACAACTAGGAGGGGAGGACCGGGGAGGGCTCGGGCACCCCTGCCTGAGCTCGCGGATGAACTGAGACGCAAACTGCA
GCAACGCCAGCCACTGCCCGACTCCAACCCCGAGGAGTCATCAGTATGAGGGGAACCCCCACCGCGTCGGCGGGAAGCGT
GGGAGGTGTAGCTCCTACTTTTGCACAGGCACCAGCTACCTCAGGGACATGGCACGGGCACCTGCTCTGTCTGGGACAGA
TACTGCCCAGCACCCACCCGGCCATGAGGACCTGCTCTGCTCAGCACGGGCACTGCCACTTGGTGTGGCTCACCAGGGCA
CCAGCCTCGCAGAAGGCATCTTCCTCCTCTGTGAATCACAGACACGCGGGACCCCAGCCGCCAAAACTTTTCAAGGCA
GAAGTTTCAAGATGTGTGTTTGTCTGTATTTGCACATGTGTTTGTGTGTGTGTATGTGTGTGTGCACGCGCGTGCGCG
CTTGTGGCATAGCCTTCCTGTTTCTGTCAAGTCTTCCCTTGGCCTGGGTCCTCCTGGTGAGTCATTGGAGCTATGAAGGG
GAAGGGGTCGTATCACTTTGTCTCTCCTACCCCCACTGCCCCGAGTGTCGGGCAGCGATGTACATATGGAGGTGGGGTGG
ACAGGGTGCTGTGCCCCTTCAGAGGGAGTGCAGGGCTTGGGGTGGGCCTAGTCCTGCTCCTAGGGCTGTGAATGTTTTCA
GGGTGGGGGGAGGGAGATGGAGCCTCCTGTGTGTTTGGGGGGAAGGGTGGGTGGGGCCTCCCACTTGGCCCCGGGGTTCA
GTGGTATTTTATACTTGCCTTCTTCCTGTACAGGGCTGGGAAAGGCTGTGTGAGGGGAGAGAAGGGAGAGGGTGGGCCTG
CTGTGGACAATGGCATACTCTCTTCCAGCCCTAGGAGGAGGGCTCCTAACAGTGTAACTTATTGTGTCCCCGCGTATTTA
TTTGTTGTAAATATTTGAGTATTTTTATATTGACAAATAAAATGGAGAAAATGAAACGA

HUMAN PROTEIN SEQUENCE : hP22-005.4 (Seq ID No: 146)
MAPHWAVWLLAARLWGLGIGAEVWWNLVPRKTVSSGELATVVRRFSQTGIQDFLTLTLTEPTGLLYVGAREALFAFSMEA
LELQGAISWEAPVEKKTECIQKGKNNQTECFNFIRFLQPYNASHLYVCGTYAFQPKCTYVNMLTFTLEHGEFEDGKGKCP

```
YDPAKGHAGLLVDGELYSATLNNFLGTEPIILRNMGPHHSMKTEYLAFWLNEPHFVGSAYVPESVGSFTGDDDKVYFFR
ERAVESDCYAEQVVARVARVCKGDMGGARTLQRKWTTFLKARLACSAPNWQLYFNQLQAMHTLQDTSWHNTFFGVFQAQ
WGDMYLSAICEYQLEEIQRVFEGPYKEYHEEAQKWDRYTDPVPSPRPGSCINNWHRRHGYTSSLELPDNILNFVKKHPLM
EEQVGPRWSRPLLVKKGTNFTHLVADRVTGLDGATYTVLFIGTGDGWLLKAVSLGPWVHLIEELQLFDQEPMRSLVLSQS
KKLLFAGSRSQLVQLPVADCMKYRSCADCVLARDPYCAWSVNTSRCVAVGGHSGSLLIQHVMTSDTSGICNLRGSKKVRP
TPKNITVVAGTDLVLPCHLSSNLAHARWTFGGRDLPAEQPGSFLYDARLQALVVMAAQPRHAGAYHCFSEEQGARLAAEG
YLVAVVAGPSVTLEARAPLENLGLVWLAVVALGAVCLVLLLLVLSLRRRLREELEKGAKATERTLVYPLELPKEPTSPPF
RPCPEPDEKLWDPVGYYYSDGSLKIVPGHARCQPGGGPPSPPPGIPGQPLPSPTRLHLGGGRNSNANGYVRLQLGGEDRG
GLGHPLPELADELRRKLQQRQPLPDSNPEESSV*
```

**Claims**

1. An isolated nucleic acid comprising at least 10 contiguous nucleotides of a sequence selected from the group consisting of the polynucleotide sequences of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19, or its complement.

2. A host cell comprising a recombinant nucleic acid of claim 1.

3. An expression vector comprising the isolated nucleic acid according to claim 1.

4. A host cell comprising the expression vector of claim 3.

5. The polynucleotide according to claim 1, wherein said polynucleotide, or its complement or a fragment thereof, further comprises a detectable label.

6. The polynucleotide according to claim 1, wherein said polynucleotide, or its complement or a fragment thereof, is attached to a solid support.

7. The polynucleotide according to claim 1, wherein said polynucleotide, or its complement or a fragment thereof, is prepared at least in part by chemical synthesis.

8. The polynucleotide according to claim 1, wherein said polynucleotide, or its complement or a fragment thereof, is an antisense fragment.

9. The polynucleotide according to claim 1, wherein said polynucleotide, or its complement or a fragment thereof, is single stranded.

10. The polynucleotide according to claim 1, wherein said polynucleotide, or its complement or a fragment thereof, is double stranded.

11. The polynucleotide according to claim 1, comprising at least 15 contiguous nucleotides.

12. The polynucleotide according to claim 1, comprising at least 20 contiguous nucleotides.

13. A microarray for detecting a cancer associated (CA) nucleic acid comprising:

    at least one probe comprising at least 10 contiguous nucleotides of a sequence selected from the group consisting of the polynucleotide sequences SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19, or its complement.

14. The microarray according to claim 13, comprising at least 15 contiguous nucleotides.

15. The microarray according to claim 13, comprising at least 20 contiguous nucleotides.

16. An isolated polypeptide, encoded within an open reading frame of a CA sequence selected from the group consisting of the polynucleotide sequences of SEQ ID NOS: 4, 10, 16, 22, 28, 34, 40, 46, 52, 58, 66, 72, 80, 86, 98, 104, 110, 132, and 138 shown in Tables 1-19, or its complement.

17. The polypeptide of claim 16, wherein said polypeptide comprises the amino acid sequence encoded by a polynucleotide selected from the group consisting of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19.

18. The polypeptide of claim 16, wherein said polypeptide comprises the amino acid sequence encoded by a polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19.

19. The polypeptide of claim 16, wherein said polypeptide comprises the amino acid sequence of an epitope of the amino acid sequence of a CA polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19.

20. The polypeptide of claim 16, wherein said polypeptide is expressed on a cell surface, wherein the CA protein selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146.

21. The polypeptide of claim 16, wherein said polypeptide or fragment thereof is attached to a solid support.

22. An isolated antibody or antigen binding fragment thereof, that binds to a polypeptide according to any one of claims 16-20.

23. The isolated antibody or antigen binding fragment thereof according the claim 22, wherein said antibody or fragment thereof is attached to a solid support.

24. The isolated antibody or antigen binding fragment thereof according the claim 22, wherein said antibody is a monoclonal antibody.

25. The isolated antibody or antigen binding fragment thereof according the claim 22, wherein said antibody is a polyclonal antibody.

26. The isolated antibody or antigen binding fragment thereof according the claim 22, wherein said antibody or fragment thereof further comprises a detectable label.

27. An isolated antibody that binds to a polypeptide, or antigen binding fragment thereof, according to any of claims 16-20, prepared by a method comprising the steps of: (i) immunizing a host animal with a composition comprising said polypeptide, or antigen binding fragment thereof, and (ii) collecting cells from said host expressing antibodies against the antigen or antigen binding fragment thereof.

28. The monoclonal antibody according to claim 24, wherein the monoclonal antibody binds to the extracellular domain of the CA protein.

29. The monoclonal antibody according to claim 24, wherein the monoclonal antibody binds to at least one human cancer cell line.

30. The monoclonal antibody according to claim 24, wherein the monoclonal antibody is prepared by a process comprising:

     (a) providing a hybridoma capable of producing the monoclonal antibody; and
     (b) culturing the hybridoma under conditions that provide for the production of the monoclonal antibody by the hybridoma.

31. A hybridoma that produces the monoclonal antibody according to claim 24.

32. The antibody according to claim 22, wherein the antibody is a humanized antibody.

33. The antibody according to claim 22, wherein the CAP is expressed on a cancer cell surface but not on a normal cell surface.

34. The antibody according to claim 22, wherein the CAP is differentially expressed on a cancer cell surface relative to a normal cell surface.

35. The antibody according to claim 22, wherein the antibody is linked to a therapeutic agent.

36. The antibody according to claim 24, wherein the antibody is linked to a therapeutic agent.

**37.** A pharmaceutical composition comprising the antibody according to claim 22 and a pharmaceutically acceptable excipient.

**38.** A pharmaceutical composition comprising the antibody according to claim 35 and a pharmaceutically acceptable excipient.

**39.** A pharmaceutical composition comprising the antibody according to claim 36 and a pharmaceutically acceptable excipient.

**40.** A kit for detecting cancer cells comprising the antibody according to claim 22.

**41.** A kit for detecting cancer cells comprising the monoclonal antibody according to claim 24.

**42.** A method for detecting a presence or an absence of cancer cells in an individual, the method comprising:

contacting cells from the individual with the antibody according to any of claims 22 or 24;
and detecting a complex of a CAP from the cancer cells and the antibody,

wherein detection of the complex correlates with the presence of cancer cells in the individual.

**43.** A method for inhibiting growth of cancer cells in an individual, the method comprising: administering to the individual an effective amount of a pharmaceutical composition according to any of claims 37, 38, or 39.

**44.** A method for delivering a therapeutic agent to cancer cells in an individual, the method comprising: administering to the individual an effective amount of a pharmaceutical composition according to any of claims 37, 38, or 39.

**45.** A kit for diagnosing the presence of cancer in a test sample, said kit comprising at least one polynucleotide that selectively hybridizes to a CA polynucleotide sequence selected from the group consisting of the polynucleotide sequences SEQ ID NOS: 4, 10, 16, 22, 28, 34, 40, 46, 52, 58, 66, 72, 80, 86, 98, 104, 110, 132, and 138 shown in Tables 1-19, or its complement.

**46.** A kit for diagnosing the presence of cancer in a test sample, said kit comprising at least one polynucleotide that selectively hybridizes to the sequence of a polynucleotide sequence selected from the group consisting of the polynucleotide sequences SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19, a fragment thereof, or their complement.

**47.** An electronic library comprising a polynucleotide, or fragment thereof, comprising a CA polynucleotide sequence selected from the group consisting of the polynucleotide sequences of SEQ ID NOS: 4, 10, 16, 22, 28, 34, 40, 46, 52, 58, 66, 72, 80, 86, 98, 104, 110, 132, and 138 shown in Tables 1-19, or its complement.

**48.** An electronic library comprising a polynucleotide, or fragment thereof, comprising a CA polynucleotide sequence selected from the group consisting of the polynucleotide sequences of SEQ ID NOS: 5, 11,17,23, 29, 35,41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19.

**49.** An electronic library comprising a polypeptide, or fragment thereof, comprising a CA polypeptide sequence selected from the group consisting of the polypeptide sequences of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19.

**50.** A method of screening for anticancer activity comprising:

(a) providing a cell that expresses a cancer associated (CA) gene encoded by a nucleic acid sequence selected from the group consisting of the sequences SEQ ID NOS: 4, 10, 16, 22, 28, 34, 40, 46, 52, 58, 66, 72, 80, 86, 98, 104, 110, 132, and 138 shown in Tables 1-19, or fragment thereof;
(b) contacting a tissue sample derived from a cancer cell with an anticancer drug candidate; and
(c) monitoring an effect of the anticancer drug candidate on an expression of the CA polynucleotide in the tissue sample.

**51.** The method of screening for anticancer activity according to claim 50, wherein the CA gene comprises at least one nucleic acid sequence selected from the group consisting of the sequences SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19.

**52.** The method of screening for anticancer activity according to claim 50, further comprising:

(d) comparing the level of expression in the absence of said drug candidate to the level of expression in the presence of the drug candidate.

**53.** The method of screening for anticancer activity according to claim 51, wherein the drug candidate is an inhibitor of transcription and further wherein the nucleic acid sequence is selected from the group consisting of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19.

**54.** A method for detecting cancer associated with expression of a polypeptide in a test cell sample, comprising the steps of:

(i) detecting a level of expression of at least one polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or a fragment thereof; and
(ii) comparing the level of expression of the polypeptide in the test sample with a level of expression of polypeptide in a normal cell sample, wherein an altered level of expression of the polypeptide in the test cell sample relative to the level of polypeptide expression in the normal cell sample is indicative of the presence of cancer in the test cell sample.

**55.** A method for detecting cancer associated with expression of a polypeptide in a test cell sample, comprising the steps of:

(i) detecting a level of activity of at least one polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or a fragment thereof, wherein said activity corresponds to at least one activity for the polypeptide listed in Table 21; and
(ii) comparing the level of activity of the polypeptide in the test sample with a level of activity of polypeptide in a normal cell sample, wherein an altered level of activity of the polypeptide in the test cell sample relative to the level of polypeptide activity in the normal cell sample is indicative of the presence of cancer in the test cell sample.

**56.** A method for detecting cancer associated with the presence of an antibody in a test serum sample, comprising the steps of:

(i) detecting a level of an antibody against an antigenic polypeptide selected from the group consisting of SEQ ID NOS: 6, 12, 18, 24, 30, 36, 42, 48, 54, 60, 62, 68, 74, 76, 82, 88, 90, 92, 94, 100, 106, 112, 114, 116, 118, 120, 122, 124, 126, 128, 134, 140, 142, 144, and 146 shown in Tables 1-19, or antigenic fragment thereof; and
(ii) comparing said level of said antibody in the test sample with a level of said antibody in the control sample, wherein an altered level of antibody in said test sample relative to the level of antibody in the control sample is indicative of the presence of cancer in the test serum sample.

**57.** A method for screening for a bioactive agent capable of modulating the activity of a CA protein (CAP), wherein said CAP is encoded by a nucleic acid comprising a nucleic acid sequence selected from the group consisting of the polynucleotide sequences SEQ ID NOS: 5,11,17,23,29,35,41,47, 53, 59, 61,67, 73, 75, 81, 87, 89, 91, 93, 99, 105, 111, 113, 115, 117, 119, 121, 123, 125, 127, 133, 139, 141, 143, and 145 shown in Tables 1-19, said method comprising:

a) combining said CAP and a candidate bioactive agent; and
b) determining the effect of the candidate agent on the bioactivity of said CAP.

**58.** The method of screening for the bioactive agent according to claim 57, wherein the bioactive agent affects the expression of the CA protein (CAP).

**59.** The method of screening for the bioactive agent according to claim 57, wherein the bioactive agent affects the activity of the CA protein (CAP), wherein such activity is G-protein coupled receptor activity.

**60.** A method for diagnosing cancer comprising:

a) determining the expression of one or more genes comprising a nucleic acid sequence selected from the group consisting of the human genomic and mRNA sequences outlined in Tables 1-19, in a first tissue type of a first individual; and
b) comparing said expression of said gene(s) from a second normal tissue type from said first individual or a second unaffected individual;

wherein a difference in said expression indicates that the first individual has cancer.

**61.** A method for treating cancers comprising administering to a patient an inhibitor of a CA protein (CAP), wherein said CAP is encoded by a nucleic acid comprising a nucleic acid sequence selected from the group consisting of the human nucleic acid sequences in Tables 1-19.

**62.** The method for treating cancers according to claim 61, wherein the inhibitor of a CA protein (CAP) binds to the CA protein.

**63.** The method for treating cancers according to claim 61, wherein the inhibitor is a G-protein coupled receptor antagonist.

**64.** A method for inhibiting expression of a cancer associated (CA) gene in a cell comprising:

contacting a cell expressing a CA gene with a double stranded RNA comprising a sequence capable of hybridizing to a cancer associated (CA) mRNA corresponding to the polynucleotide sequences of SEQ ID NOS: 5, 11, 17, 23, 29, 35, 41, 47, 53, 59, 61, 67, 73, 75, 81, 87, 89, 91, 93, 99, 105, 111, 113, 115, 117, 119, 121, 123, 125, 127, 133, 139, 141, 143, and 145 shown in Tables 1-19, in an amount sufficient to elicit RNA interference; and inhibiting expression of the CA gene in the cell.

**65.** The method of claim 64, wherein the double stranded RNA is provided by introducing a short interfering RNA (siRNA) into the cell by a method selected from the group consisting of transfection, electroporation, and microinjection.

**66.** The method of claim 64, wherein the double stranded RNA is provided by introducing a short interfering RNA (siRNA) into the cell by an expression vector.

**Figure 1**

## Figure 2.

**Average Ct**

**Sample**

| | Housekeeper | Target |
|---|---|---|
| Human Genomic_clontech | 23.40229767 | 24.718297 |
| Malignant mammary adenocarcinoma pleural effusion | 24.57459367 | 25.62105175 |
| Mammary ductal carcinoma plueral effusion | 23.935565 | 25.064095 |
| Mammary ductal carcinoma_human _45537 | 24.06496275 | 24.8361145 |
| Mammary ductal carcinoma_human_45523 | 24.286542 | 25.59857167 |
| Mammary ductal carcinoma_human_45535 | 24.271219 | 25.62112767 |
| transformed primary embryonal kidney_human | 24.136573 | 25.86051867 |

# Figure 3.

**Target Normalized with Housekeeper**

| Sample | ΔCt | Stdev (ΔCt) |
|---|---|---|
| Human Genomic_clontech (Calibrator) | 1.69 | 0.22 |
| Malignant mammary adenocarcinoma pleural effusion | -1.85 | 0.14 |
| Mammary ductal carcinoma plueral effusion | 1.12 | 0.24 |
| Mammary ductal carcinoma_human _45537 | 1.02 | 0.16 |
| Mammary ductal carcinoma_human_45523 | 0.49 | 0.26 |
| Mammary ductal carcinoma_human_45535 | 1.03 | 0.18 |
| transformed primary embryonal kidney_human | 1.81 | 0.15 |

## Figure 4.

**Target Normalized with Housekeeper**

| Sample | ΔCt | Stdev (ΔCt) | ΔΔCt | Stdev (ΔΔCt) | Comparative expression level |
|---|---|---|---|---|---|
| Human Genomic_clontech (**Calibrator**) | 1.69 | 0.22 | 0 | 0.22 | 1 |
| Malignant mammary adenocarcinoma pleural effusion | -1.85 | 0.14 | -3.54 | 0.14 | 11.60 |
| Mammary ductal carcinoma plueral effusion | 1.12 | 0.24 | -0.57 | 0.24 | 1.48 |
| Mammary ductal carcinoma_human _45537 | 1.02 | 0.16 | -0.67 | 0.16 | 1.59 |
| Mammary ductal carcinoma_human_45523 | 0.49 | 0.26 | -1.20 | 0.26 | 2.30 |
| Mammary ductal carcinoma_human_45535 | 1.03 | 0.18 | -0.66 | 0.18 | 1.58 |
| transformed primary embryonal kidney_human | 1.81 | 0.15 | 0.12 | 0.15 | 0.92 |

## EP 2 058 408 A2

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 36709403 A
- US 10388838 B
- US 10669920 B
- US 10737318 B
- US 5644048 A
- US 5386023 A
- US 5637684 A
- US 5602240 A
- US 5216141 A
- US 4469863 A
- US 5235033 A
- US 5034506 A
- US 5837832 A
- US 6410229 B, Lockhart
- US 5470967 A
- US 5714331 A
- EP 0225807 A
- US 5419966 A
- WO 9525116 A
- WO 9535505 A
- US 5445934 A
- US 5384261 A
- US 5700637 A
- US 9701019 W
- US 9701048 W
- US 4959314 A
- WO 8705330 A
- US 4640835 A
- US 4496689 A
- US 4301144 A
- US 4670417 A
- US 4791192 A
- US 4179337 A
- US 4753894 A, Frankel
- US 4938948 A, Ring
- US 4956453 A, Bjorn
- EP 519596 A
- US 4816567 A
- US 5545807 A
- US 5545806 A
- US 5569825 A
- US 5625126 A
- US 5633425 A
- US 5661016 A
- US 5530101 A
- US 5585089 A
- WO 9824893 A
- WO 9110741 A
- WO 9630498 A
- WO 9402602 A
- US 5939598 A
- WO 9633735 A
- US 5681702 A
- US 5597909 A
- US 5545730 A
- US 5594117 A
- US 5591584 A
- US 5571670 A
- US 5580731 A
- US 5624802 A
- US 5635352 A
- US 5594118 A
- US 5359100 A
- US 5124246 A
- US 5681697 A
- US 9701019 B
- WO 9104753 A
- WO 9010448 A
- WO 0044895 A, Kreutzer
- WO 0136646 A, Zernicka-Goetz
- WO 9932619 A, Fire
- WO 0129058 A, Mello and Fire
- WO 9314778 A
- WO 9007936 A
- WO 9403622 A
- WO 9325698 A
- WO 9325234 A
- WO 9311230 A
- WO 9310218 A
- US 4777127 A
- GB 2200651 A
- EP 0345242 A
- WO 9102805 A
- WO 9412649 A
- WO 9303769 A
- WO 9319191 A
- WO 9428938 A
- WO 9511984 A
- WO 9500655 A
- WO 9507994 A
- WO 9617072 A
- WO 9530763 A
- WO 9742338 A
- WO 9011092 A
- US 5580859 A
- WO 9513796 A
- WO 9423697 A
- WO 9114445 A

- EP 0524968 A
- US 5206152 A
- WO 9211033 A
- US 5149655 A
- US 9303868 W

- WO 9202526 A
- US 4683195 A
- US 4683202 A
- WO 0143869 A

**Non-patent literature cited in the description**

- **SORENSEN et al.** *J. of Virology,* 2000, vol. 74, 2161
- **HANSEN et al.** *Genome Res.,* 2000, vol. 10 (2), 237-43
- **SORENSEN et al.** *J. Virology,* 1996, vol. 70, 4063
- **SORENSEN et al.** *J. Virology,* 1993, vol. 67, 7118
- **JOOSTEN et al.** *Virology,* 2000, vol. 268, 308
- **LI et al.** *Nature Genetics,* 1999, vol. 23, 348
- Mammary Tumors in the Mouse. Elsevier/North-Holland Biomedical Press
- **MARSHALL.** *Cell,* 1991, vol. 64, 313-326
- **WEINBERG.** *Science,* 1991, vol. 254, 1138-1146
- Cancer: Principles and Practice of Oncology. 2001, vol. 20, 495-508
- **BEAUCAGE et al.** *Tetrahedron,* 1993, vol. 49 (10), 1925
- **LETSINGER.** *J. Org. Chem.,* 1970, vol. 35, 3800
- **SPRINZL et al.** *Eur. J. Biochem.,* 1977, vol. 81, 579
- **LETSINGER et al.** *Nucl. Acids Res.,* 1986, vol. 14, 3487
- **SAWAI et al.** *Chem. Lett.,* 1984, 805
- **LETSINGER et al.** *J. Am. Chem. Soc.,* 1988, vol. 110, 4470
- **PAUWELS et al.** *Chemica Scripta,* vol. 26, 141
- **MAG et al.** *Nucleic Acids Res.,* 1991, vol. 19, 1437
- **BRIU et al.** *J. Am. Chem. Soc.,* 1989, vol. 111, 2321
- **ECKSTEIN.** Oligonucleotides and Analogues: A Practical Approach. Oxford University Press
- **EGHOLM.** *J. Am. Chem. Soc.,* 1992, vol. 114, 1895
- **MEIER et al.** *Chem. Int. Ed. Engl,* 1992, vol. 31, 1008
- **NIELSEN.** *Nature,* 1993, vol. 365, 566
- **CARLSSON et al.** *Nature,* 1996, vol. 380, 207
- **DENPCY et al.** *Proc. Natl. Acad. Sci. USA,* 1995, vol. 92, 6097
- **KIEDROWSHI et al.** *Angew. Chem. Intl. Ed. English,* 1991, vol. 30, 423
- **LETSINGER et al.** *Nucleoside & Nucleotide,* 1994, vol. 13, 1597

- Carbohydrate Modifications in Antisense Research. ASC Symposium Series 580
- **MESMAEKER et al.** *Bioorganic & Medicinal Chem. Lett.,* 1994, vol. 4, 395
- **JEFFS et al.** *J. Biomolecular NMR,* 1994, vol. 34, 17
- *Tetrahedron Lett.,* 1996, vol. 37, 743
- **JENKINS et al.** *Chem. Soc. Rev.,* 1995, 169-176
- **RAWLS.** *C & E News,* 02 June 1997, 35
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *PNAS,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *PNAS,* 1993, vol. 90, 5873-5787
- **ALTSCHUL et al.** *Methods in Enzymology,* 1996, vol. 266, 460-480
- **SCHENA et al.** *Science,* 1995, vol. 270, 467-470
- **DERISI et al.** *Nature Genetics,* 1996, vol. 14, 457-460
- **NIELSEN et al.** *Science,* vol. 254, 1497-1500
- **NIELSEN.** *Curr. Opin. Biotechnol.,* 1999, vol. 10, 71-75
- **URDEA et al.** *Nucleic Acids Symp. Ser.,* 1991, vol. 24, 197-200
- **PEVZNER et al.** *J. Biomol. Struc. & Dyn.,* 1991, vol. 9, 399-410
- **MASKOS ; SOUTHERN.** *Nuc. Acids Res.,* 1992, vol. 20, 1679-84
- **R. DRMANAC et al.** *J. Biomol. Struc. & Dyn.,* 1991, vol. 5, 1085-1102
- **P. A. PEVZNER.** *J. Biomol. Struc. & Dyn.,* 1989, vol. 7, 63-73
- **T. SANO ; C. R. CANTOR.** *Bio/Technology,* 1991, vol. 9, 1378-81
- technical section on cross-linkers. Pierce Chemical Company catalog, 1994, 155-200

- **SMITH et al.** Direct Mechanical Measurements of the Elasticity of Single DNA Molecules by Using Magnetic Beads. *Science,* 1992, vol. 258, 1122-1126
- **PEASE et al.** *Proc. Natl. Acad. Sci. USA,* 1994, vol. 91 (11), 5022-5026
- **GUO et al.** *Nucleic Acids Res.,* 1994, vol. 22, 5456-5465
- **MASKOS ; SOUTHERN.** *Nucleic Acids Research,* 1992, vol. 20, 1679-1684
- **GERMER, S. et al.** *Genome Res.,* 2000, vol. 10, 258-266
- **HEID, C. A. et al.** *Genome Res.,* 1996, vol. 6, 986-994
- **HUNTER et al.** *Nature,* 1962, vol. 144, 945
- **DAVID et al.** *Biochemistry,* 1974, vol. 13, 1014
- **PAIN et al.** *J. Immunol. Meth.,* 1981, vol. 40, 219
- **NYGREN, J.** *Histochem. and Cytochem,* 1982, vol. 30, 407
- **SMITH ; WATERMAN.** *Adv. Appl. Math.,* 1981, vol. 2, 482-489
- **GO et al.** *Int. J. Peptide Protein Res.,* 1980, vol. 15, 211
- **QUEROL et al.** *Prot. Eng.,* 1996, vol. 9, 265
- **OLSEN ; THOMSEN.** *J. Gen. Microbiol.,* 1991, vol. 137, 579
- **CLARKE et al.** *Biochemistry,* 1993, vol. 32, 4322
- **WAKARCHUK et al.** *Protein Eng.,* 1994, vol. 7, 1379
- **TOMA et al.** *Biochemistry,* 1991, vol. 30, 97
- **HAEZERBROUCK et al.** *Protein Eng.,* 1993, vol. 6, 643
- **MASUL et al.** *Appl. Env. Microbiol.,* 1994, vol. 60, 3579
- **T.E. CREIGHTON.** Proteins: Structure and Molecular Properties. W.H. Freeman & Co, 1983, 79-86
- **APLIN ; WRISTON.** *LA Crit. Rev. Biochem.,* 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **GARNIER et al.** *J. Mol. Biol.,* 1978, vol. 120, 97
- *Adv. in Enzymol.,* 1978, vol. 47, 45-148
- *J. Mol. Biol.,* 1982, vol. 157, 105-132
- *J. Virol.,* 1985, vol. 55 (3), 836-839
- *CABIOS,* 1988, vol. 4 (1), 181-186
- **WELLING, G. W. et al.** *FEBS Lett.,* 1985, vol. 188, 215-218
- **KOHLER ; MILSTEIN.** *Nature,* 1975, vol. 256, 495
- **GODING.** Monoclonal Antibodies: Principles and Practice. Academic Press, 1986, 59-103
- **WALDMANN, T. A.** *Science,* 1991, vol. 252, 1657-1662
- **WINTER et al.** *Nature,* 1991, vol. 349, 293-299
- **LOBUGLIO et al.** *Proc. Nat. Acad. Sci. USA,* 1989, vol. 86, 4220-4224
- **SHAW et al.** *J Immunol.,* 1987, vol. 138, 4534-4538
- **BROWN et al.** *Cancer Res.,* 1987, vol. 47, 3577-3583
- **RIECHMANN et al.** *Nature,* 1988, vol. 332, 323-327
- **VERHOEYEN et al.** *Science,* 1988, vol. 239, 1534-1536
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **MORRISON et al.** *Proc. Natl. Acad. Sci,* 1984, vol. 81, 6851-6855
- **MORRISON ; OI.** *Adv. Immunol.,* 1988, vol. 44, 65-92

- **VERHOEYER et al.** *Science,* 1988, vol. 239, 1534-1536
- **PADLAN.** *Molec. Immun.,* 1991, vol. 28, 489-498
- **PADLAN.** *Molec. Immunol.,* 1994, vol. 31 (3), 169-217
- **KETTLEBOROUGH, C.A. et al.** *Protein Eng.,* 1991, vol. 4 (7), 773-83
- **CHOTHIA et al.** *J. Mol. Biol.,* 1987, vol. 196, 901-917
- **KABAT et al.** *U.S. Dept. of Health and Human Services NIH Publication,* 1991, 91-3242
- **MERRIFELD.** *J. Am. Chem. Soc.,* 1963, vol. 85, 2149
- **CAPRINO ; HAN.** *J. Org. Chem,* 1972, vol. 37, 3404
- **MILSTEIN ; KOHLER.** *Nature,* 1975, vol. 256, 495-497
- **GULFRE ; MILSTEIN.** Methods in Enzymology: Immunochemical Techniques. Academic Press, 1981, vol. 73, 1-46
- **NEVELLE et al.** *Immunol Rev,* 1982, vol. 62, 75-91
- **ROSS et al.** *Eur. J Biochem,* 1980, vol. 104
- **VITTETA et al.** *Immunol Rev,* 1982, vol. 62, 158-183
- **RASO et al.** *Cancer Res,* 1982, vol. 42, 457-464
- **TROWBRIDGE et al.** *Nature,* 1981, vol. 294, 171-173
- **LOCKHART.** *Nature Biotechnology,* 1996, vol. 14, 1675-1680
- **ZLOKARNIK et al.** *Science,* 1998, vol. 279, 84-8
- **HEID et al.** *Genome Res.,* 1996, vol. 6, 986-994
- **STEIN ; COHEN.** *Cancer Res.,* 1988, vol. 48, 2659
- **VAN DER KROL et al.** *BioTechniques,* 1988, vol. 6, 958
- **WU-PONG.** *BioPharm,* November 1994, 20-33
- **MUKHOPADHYAY ; ROTH.** *Crit. Rev. in Oncogenesis,* 1996, vol. 7, 151-190
- **FIRE et al.** *Nature,* 1998, vol. 391, 806
- *Sharp, P.A., RNA interference - 2001, Genes & Development,* 2001, vol. 15, 485-490
- **ELBASHIR, S.M. et al.** *Nature,* 2001, vol. 411, 494-498
- **CAPLEN, N.J. et al.** *Proc. Natl. Acad. Sci. USA,* 2001, vol. 98, 9742-9747
- **HARBORTH, J. et al.** *J. Cell Sci.,* 2001, vol. 114, 4557-4565
- **LEE, N.S. et al.** *Nat. Biotechnol.,* 2002, vol. 20, 500-505
- **MIYAGISHI, M. ; TAIRA, K.** *Nat. Biotechnol,* 2002, vol. 20, 497-500
- **PAUL, C.P. et al.** *Nat. Biotechnol,* 2002, vol. 20, 505-508
- **PAULE, M.R. ; WHITE, R.J.** *Nucleic Acids Res.,* 2000, vol. 28, 1283-1298
- **ZIAUDDIN, J. ; SABATINI, D.M.** *Nature,* 2001, vol. 411, 107-110
- **BERSTEIN et al.** *Nature,* 2001
- **HUTVAGNER et al.** *Science,* 2001, vol. 293, 834
- **ELBASHIR et al.** *Genes Dev.,* 2001, vol. 15, 188
- **ALFONSO GENNARO.** Remington: The Science and Practice of Pharmacy. Lippincott, Williams, & Wilkins, 1995
- **FINDEIS et al.** *Trends Biotechnol.,* 1993, vol. 11, 202
- **CHIOU et al.** Gene Therapeutics: Methods And Applications Of Direct Gene Transfer. 1994
- **WU et al.** *J. Biol. Chem,* 1988, vol. 263, 621
- **WU et al.** *J. Biol. Chem,* 1994, vol. 269, 542
- **ZENKE et al.** *Proc. Natl. Acad. Sci.,* 1990, vol. 87, 3655
- **WU et al.** *J. Biol. Chem,* 1991, vol. 266, 338
- **JOLLY.** *Cancer Gene Therapy,* 1994, vol. 1, 51
- **KIMURA.** *Human Gene Therapy,* 1994, vol. 5, 845
- **CONNELLY.** *Human Gene Therapy,* 1995, vol. 1, 185
- **KAPLITT.** *Nature Genetics,* 1994, vol. 6, 148
- **CURIEL.** *Hum. Gene Ther,* 1992, vol. 3, 147
- **CURIEL.** *Hum. Gene Ther.,* 1992, vol. 3, 147
- **WU.** *J. Biol. Chem,* 1989, vol. 264, 16985
- **PHILIP.** *Mol. Cell Biol.,* 1994, vol. 14, 2411
- **WOFFENDIN.** *Proc. Natl. Acad. Sci,* 1994, vol. 91, 1581
- **WOFFENDIN et al.** *Proc. Natl. Acad. Sci. USA,* 1994, vol. 91 (24), 11581
- **BROWER.** *Nature Biotechnology,* 1998, vol. 16, 1304-1305
- **MULLIS et al.** *Meth. Enzymol.,* 1987, vol. 155, 335
- **SAMBROOK et al.** Molecular Cloning: A Laboratory Manual. Cold Spring Harbor Laboratory, 1989
- **SAIKI et al.** *Science,* 1985, vol. 239, 487
- **SAMBROOK et al.** Molecular Cloning: A Laboratory Manual. CSH Press, 1989, 14.2-14.33
- **HANAHAN et al.** *Cell,* 2000, vol. 100, 57-70
- *Nature Genet.,* 2000, vol. 25, 25-29
- **APWEILER.** *Bioinformatics,* 2000, vol. 16 (12), 1145-1150
- **LUO et al.** *Nature Med,* 1999, vol. 5, 117-122
- **S.M. WEISSMAN.** *Mol Biol Med,* 1987, vol. 4 (3), 133-143
- **PATANJALI et al.** *Proc. Natl. Acad. Sci. USA,* 1991, vol. 88 (5), 1943-1947

- **SAMBROOK, J.T. et al.** Molecular Cloning: A Laboratory Manual. Cold Spring Harbor Laboratory Press
- Effective Large-Scale Sequence Similarity Searches. **CLAVERIE.** Computer Methods for Macromolecular Sequence Analysis. Academic Press, 1996, vol. 266, 212-227
- Automated DNA Sequencing and Analysis Techniques. **CLAVERIE et al.** Automated DNA Sequencing and Analysis Techniques. Academic Press, 1994, vol. 36, 267
- **CLAVERIE et al.** *Comput. Chem.,* 1993, vol. 17, 191
- **ALTSCHUL.** *J. Mol. Biol.,* 1990, vol. 215, 40
- **ALTSCHUL et al.** *J. Mol. Bio.,* 1990, vol. 215, 403
- **DELLI BOVI et al.** *Cancer Res.,* 1986, vol. 46, 6333-6338
- **CESARONE, C. et al.** *Anal Biochem,* 1979, vol. 100, 188-197
- **SOUTHERN, E. M.** *J. Mol. Biol.,* 1975, vol. 98, 503-517
- **FEINBERG, A. P. et al.** *Anal. Biochem.,* 1983, vol. 132, 6-13
- **SAMBROOK et al.** Molecular Cloning: A Laboratory Manual. Cold Spring Harbor Lab. Press, 1989
- PCR Protocols. **WRIGHT ; MANOS et al.** PCR Protocols. Academic Press, 1990, 153-158
- **SAMBROOK et al.** Molecular Cloning: A Laboratory Manual. Cold Spring Harbor Laboratory Press, 1989, 16.17-16.22
- **KEOWN et al.** *Methods in Enzymology,* 1990, vol. 185, 527-537
- **MARKS et al.** *Brit. J. Urol.,* 1995, vol. 75, 225
- **SKEA et al.** *J. Immunol.,* 1993, vol. 151, 3557
- **MATHER et al.** *J. Nucl. Med.,* 1990, vol. 31, 692
- **ZHANG et al.** *Nucl. Med. Biol.,* 1992, vol. 19, 607